# EUROPEAN PATENT APPLICATION

(11) **EP 1 939 205 A1**
(43) Date of publication of application: **02.07.2008**
(21) Application number: 06832385.6
(22) Date of filing: 23.10.2006
(51) Int. Cl.: C07D 487/04, A61K 31/55, A61K 31/553, A61K 31/554, A61P 3/06, A61P 9/04, A61P 9/10, A61P 43/00, C07D 498/04, C07D 498/14, C07D 513/04

(54) **TRICYCLIC COMPOUND**

(30) Priority: 21.10.2005 JP 2005306663
(71) Applicant: Daiichi Sankyo Company, Limited, Chuo-ku Tokyo 103-8426 (JP)
(72) Inventor: SUGITA, Kazuyuki, Edogawa-ku, Tokyo134-8630 (JP); OTSUKA, Masami, Edogawa-ku, Tokyo134-8630 (JP); OKI, Hitoshi, Edogawa-ku, Tokyo134-8630 (JP); HAGINOYA, Noriyasu, Edogawa-ku, Tokyo134-8630 (JP); ICHIKAWA, Masanori, Edogawa-ku, Tokyo134-8630 (JP); ITOH, Masao, Edogawa-ku, Tokyo134-8630 (JP)
(74) Representative: Hartz, Nikolai
(86) International application number: PCT/JP2006/321056
(87) International publication number: WO 2007/055093

(57) **Abstract**

The present invention relates to tricyclic compounds each represented by the following formula (I): 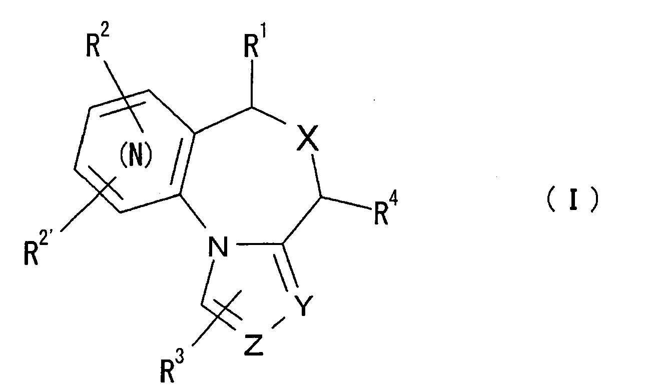 (wherein, R¹, R², R^{2'}, R³, R⁴, X, Y and Z have the same meanings as defined in the specification); and a drug containing the compound.

Since the compounds according to the present invention exhibit an excellent squalene synthetase inhibitory effect and cholesterol synthesis inhibitory effect so that they are useful as a drug such as preventive and/or remedy for diseases in mammals including humans such as hyperlipemia, e.g., hypercholesterolemia, hypertriglyceridemia, and low HDL cholesterolemia and/or arteriosclerosis.

## Description

### TECHNICAL FIELD

The present invention relates to novel tricyclic compounds.

### BACKGROUND ART

In recent years, the number of arteriosclerosis, ischemic heart diseases and ischemic brain diseases attributed to arteriosclerosis is increasing in Japan due to its Westernization of diet and the growing number of its elderly peaple. Hypercholesterolemia is cited as one of the primary risk factors of arteriosclerosis. As a treatment for such a disease, it is effective to administer a drug for reducing the blood cholesterol level. In terms of the lowered level of the blood cholesterol, it is preferable to prevent the biosynthesis of cholesterol without causing any damage to the biosynthesis of essential physiological components, such as ubiquinone and dolichol. So it is suggested that an enzyme, which is present downstream of farnesyl pyrophosphate in the cholesterol biosynthesis pathway, be targeted. Thus it is preferable to inhibit a squalene synthetase which is the first enzyme involved in the sterol biosynthesis. Although there are already compounds known for the inhibition of squalene synthetase, as described in Patent Documents 1 to 3, it is still hard to say these compounds have the suuficient inhibitory activities as required.
[Patent Document 1] WO2005/012272
[Patent Document 2] Japanese Patent Publication No. 2005-68138
[Patent Document 3] WO2005/068472

### DISCLOSURE OF THE INVENTION

### PROBLEMS TO BE SOLVED BY THE INVENTION

An object of the present invention is to provide compounds having a squalene synthetase inhibitory effect and cholesterol synthesis inhibitory effect.

### MEANS FOR SOLVING THE PROBLEMS

The present inventors have carried out an intensive investigation. As a result, it has been found newly that compounds represented by the formula (I) have an excellent squalene synthetase inhibitory effect and cholesterol synthesis inhibitory effect, leading to completion of the present invention.

In the present invention, there is thus provided a compound represented by the following formula (I):

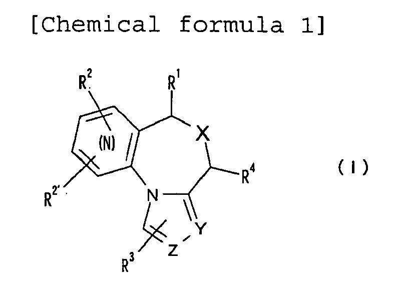

(wherein, R¹ means an aryl group or heteroaryl group which may have from one to three substituents selected from the class consisting of halogen atoms, hydroxy group, nitro group, cyano group, C₁₋₆ alkyl groups, C₁₋₆ alkoxy groups, halogeno(C₁₋₆ alkyl) groups, halogeno(C₁₋₆ alkoxy) groups, and C₁₋₆ alkylenedioxy groups which may have, at the alkylene portion thereof, substituents;
R² and R²' each independently means a hydrogen atom, halogen atom, hydroxy group, nitro group, cyano group, C₁₋₆ alkyl group, C₁₋₆ alkoxy group, halogeno(C₁₋₆ alkyl) group, halogeno(C₁₋₆ alkoxy) group, hydroxy(C₁₋₆ alkyl) group, hydroxy(C₁₋₆ alkoxy) group, (C₁₋₆ alkoxy) (C₁₋₆ alkyl) group, or (C₁₋₆ alkoxy) (C₁₋₆ alkoxy) group;
R³ means a hydrogen atom, halogen atom, cyano group, C₁₋₆ alkyl group, C₂₋₆ alkenyl group, C₂₋₆ alkynyl group, C₁₋₆ alkoxy group, C₁₋₆ alkanoyl group, (C₁₋₆ alkoxy) (C₁₋₆ alkyl) group, halogeno(C₁₋₆ alkyl) group, halogeno(C₂₋₆ alkenyl) group, halogeno(C₁₋₆ alkoxy) group, halogeno(C₁₋₆ alkoxy)(C₁₋₆ alkyl) group, halogeno(C₁₋₆ alkanoyl) group, hydroxy(C₁₋₆ alkyl) group, azido(C₁₋₆ alkyl) group, carbamoyl group, mono- or di-(C₁₋₆ alkyl) carbamoyl group, C₁₋₆ alkanoylamino group, C₁₋₆ alkanoylamino (C₁₋₆ alkyl) group, (C₁₋₆ alkanoyloxy)(C₁₋₆ alkyl) group, or aryl group;
X means CH₂, O, or S;
Y means N or C-R^{3'} (wherein, R^{3'} has the same meaning as that of R³) ;
Z means N or C-R^{3"} (wherein, R^{3"} has the same meaning as that of R³) ;
R⁴ means a carboxyl group, carboxycarbonyl group, carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and arylalkenyl groups, carboxy(C₂₋₆ alkenyl) group, carboxy(C₁₋₆ alkoxy) group, carboxy(C₁₋₆ alkylthio) group, carboxy(C₁₋₆ alkyl)thio(C₁₋₆ alkyl) group, carboxyaryl group, carboxy(C₁₋₆ alkoxy)(C₁₋₆ alkyl) group, mono- or di-(carboxy(C₁₋₆ alkyl))carbamoyl group (with the proviso that the carboxy group of the groups may be esterified or amidated) or a group represented by the following formula:

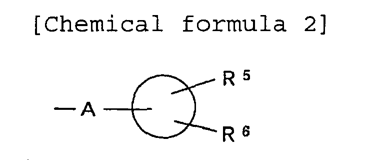

(wherein, A means a single bond, C₁₋₄ alkylene group which may have a hydroxy group and/or halogen atom, C₁₋₄ alkylenecarbonyl group which may have a hydroxy group and/or halogen atom, C₁₋₄ alkylenecarbonylamino group which may have, on the amino group thereof, a C₁₋₆ alkyl group as a substituent or C₁₋₄ alkyleneamino group having, on the amino group thereof, a C₁₋₆ alkyl group as a substituent, R⁵ means a carboxyl group, carboxycarbonyl group, carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and arylalkenyl groups, carboxy(C₂₋₆ alkenyl) group, carboxy(C₁₋₆ alkoxy) group, carboxy(C₁₋₆ alkyl) thio group, carboxy(C₁₋₆ alkyl) thio (C₁₋₆ alkyl) group, carboxyaryl group, carboxy(C₁₋₆ alkoxy)(C₁₋₆ alkyl) group, or mono- or di-(carboxy(C₁₋₆ alkyl))carbamoyl group (with the proviso that the carboxy group of these groups may be esterified or amidated),
R⁶ means a hydrogen atom, hydroxy group, halogen atom, C₁₋₆ alkyl group, carboxy group, C₁₋₆ alkoxy group, carboxy(C₁₋₆ alkoxy) group, halogenoalkyl group, halogeno(C₁₋₆ alkoxy) group, or aralkyl group (with the proviso that the carboxy group of these groups may be esterified or amidated), and the group:

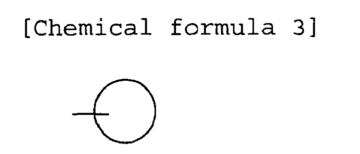

means a saturated or unsaturated cyclic hydrocarbon group or saturated or unsaturated 4- to 7-membered heterocyclic group), and the following partial structure:

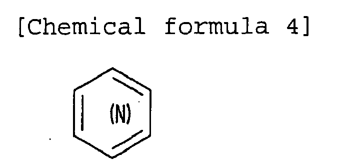

means a benzene ring or pyridine ring); salt of the compound; or solvate of the compound or the salt.
In the present invention, there are also provided a drug containing the compound represented by the formula (I), salt of the compound or solvate of the compound or salt; and a preventive and/or remedy for hypercholesterolemia, hyperlipemia, and/or arteriosclerosis containing any of them.
In the present invention, there is also provided use of the compound represented by the formula (I), salt of the compound, or solvate of the compound or salt for the preparation of a drug.
In the present invention, there is also provided a method of preventing and/or treating hypercholesterolemia, hyperlipemia, and/or arteriosclerosis, which comprises administering an effective amount of the compound represented by the formula (I), salt of the compound, or solvate of the compound or salt.

### ADVANTAGE OF THE INVENTION

As will be apparent from Examples described below, the compounds of the present invention have an excellent squalene synthetase inhibitory effect and cholesterol synthesis inhibitory effect so that they are useful as a drug for prevention or treatment of hypercholesterolemia, hyperlipemia, and/or arteriosclerosis.

### BEST MODE FOR CARRYING OUT THE INVENTION

Substituents of compounds of the present invention represented by the formula (I) will next be described, respectively.

### <R¹>

R¹ means an aryl or heteroaryl group which may have from one to three substituents selected from the class consisting of halogen atoms, hydroxy group, nitro group, cyano group, C₁₋₆ alkyl groups, C₁₋₆ alkoxy groups, halogeno (C₁₋₆ alkyl) groups, halogeno (C₁₋₆ alkoxy) groups, and C₁₋₆ alkylenedioxy groups which may have, on the alkylene portion thereof, substituents. R¹ will next be described specifically.

The aryl group means an aryl group having from 6 to 14 carbon atoms. Examples include phenyl group, naphthyl group, anthryl group, and phenanthryl group. The heteroaryl group means a 5- or 6-membered group having a hetero atom such as nitrogen atom, oxygen atom, or sulfur atom and examples include pyridyl group, thienyl group, and furyl group.

The aryl group or heteroaryl group may have from one to three substituents. These substituents will next be described.
Examples of the halogen atom include fluorine atom, chlorine atom, and bromine atom.
The C₁₋₆ in the C₁₋₆ alkyl group, C₁₋₆ alkoxy group, or the like means a linear, branched, or cyclic C₁₋₆, which will equally apply to the other groups. More specifically, it means a linear or branched C₁₋₆, or cyclic C₃₋₆.
Examples of the C₁₋₆ alkyl group include methyl group, ethyl group, isopropyl group, tertiary butyl group, and cyclopropyl group.
Examples of the C₁₋₆ alkoxy group include methoxy group, ethoxy group, isopropoxy group, tertiary butoxy group, and cyclopropoxy group.
The halogeno (C₁₋₆ alkyl) group means a C₁₋₆ alkyl group substituted with from one to three halogen atoms such as fluorine atom, chlorine atom, and bromine atom and examples include fluoromethyl group, trifluoromethyl group, and trifluoroethyl group.
The halogeno(C₁₋₆ alkoxy) group means a C₁₋₆ alkoxy group substituted with from one to three halogen atoms and examples include fluoromethoxy group, trifluoromethoxy group, fluoroethoxy group, and trifluoroethoxy group.
The C₁₋₆ alkylenedioxy group which may have, on the alkylene portion thereof, substituents means -O-(C₁₋₆ alkylene)-O- and examples include methylenedioxy group and ethylenedioxy group. Examples of the substituent on the alkylene portion thereof include halogen atoms.

R¹ is preferably an aryl group which may have from one to three substituents, preferably an aryl group which may have one or two substituents. As the substituent, halogen atoms, hydroxy group, and C₁₋₆ alkoxy groups are preferred. The aryl group is more preferably a phenyl group or naphthyl group. R¹ is more preferably a 2,3-dihydroxyphenyl group, 2,3-di-(C₁₋₆ alkoxy)phenyl group, or 2,3-dihalogenophenyl group. The 2,3-di-(C₁₋₆ alkoxy)phenyl group is preferably a 2,3-dimethoxyphenyl group. The 2,3-dihalogenophenyl group is preferably a 2,3-dichlorophenyl group.

### <R² and R^{2'} >

R² and R^{2'} each independently means a hydrogen atom, halogen atom, hydroxy group, nitro group, cyano group, C₁₋₆ alkyl group, C₁₋₆ alkoxy group, halogeno (C₁₋₆ alkyl) group, halogeno (C₁₋₆ alkoxy) group, hydroxy(C₁₋₆alkyl) group, hydroxy(C₁₋₆ alkoxy) group, (C₁₋₆ alkoxy) (C₁₋₆ alkyl) group, or (C₁₋₆ alkoxy) (C₁₋₆ alkoxy) group. R² will next be described more specifically.
The halogen atom, C₁₋₆ alkyl group, C₁₋₆ alkoxy group, halogeno (C₁₋₆ alkyl) group, and halogeno (C₁₋₆ alkoxy) group in R² are similar to those described in R¹.
The hydroxy(C₁₋₆ alkyl) group means a C₁₋₆ alkyl group substituted with from one to three hydroxy groups and examples include hydroxymethyl group and hydroxyethyl group.
The hydroxy(C₁₋₆ alkoxy) group means a C₁₋₆ alkoxy group substituted with from one to three hydroxy groups and examples include hydroxyethoxy group and hydroxypropoxy group.
The (C₁₋₆ alkoxy) (C₁₋₆ alkyl) group means a C₁₋₆ alkyl group substituted with a C₁₋₆ alkoxy group and examples include methoxymethyl group, methoxyethyl group, and ethoxymethyl group.
The (C₁₋₆ alkoxy) (C₁₋₆ alkoxy) group is a C₁₋₆ alkoxy group substituted with a C₁₋₆ alkoxy group and examples include methoxymethoxy group, methoxyethoxy group, ethoxymethoxy group, and methoxypropoxy group.

R² and R^{2'} are each preferably a hydrogen atom or halogen atom. The halogen atom is preferably a fluorine atom or chlorine atom. Above all, it is preferred that R² is a halogen atom and R² is a hydrogen atom.

### <R³>

R³ means a hydrogen atom, halogen atom, cyano group, C₁₋₆ alkyl group, C₂₋₆ alkenyl group, C₂₋₆ alkynyl group, C₁₋₆ alkoxy group, C₁₋₆ alkanoyl group, (C₁₋₆ alkoxy) (C₁₋₆ alkyl) group, halogeno (C₁₋₆ alkyl) group, halogeno (C₂₋₆ alkenyl) group, halogeno(C₁₋₆ alkoxy) group, halogeno(C₁₋₆ alkoxy)(C₁₋₆ alkyl) group, halogeno(C₁₋₆ alkanoyl) group, hydroxy(C₁₋₆ alkyl) group, azido(C₁₋₆ alkyl) group, carbamoyl group, mono- or di-(C₁₋₆ alkyl)carbamoyl group, C₁₋₆ alkanoylamino group, C₁₋₆ alkanoylamino(C₁₋₆ alkyl) group, (C₁₋₆ alkanoyloxy)(C₁₋₆ alkyl) group, or aryl group. R³ will next be described more specifically. Description on R³ will equally apply to R^{3'} or R^{3"}.

The halogen atom, C₁₋₆ alkyl group, C₁₋₆ alkoxy group, (C₁₋₆ alkoxy) (C₁₋₆ alkyl) group, halogeno(C₁₋₆ alkyl) group, halogeno (C₁₋₆ alkoxy) group, hydroxy(C₁₋₆ alkyl) group, and aryl group in R³ are similar to those described in R¹ or R².
Examples of the C₂₋₆ alkenyl group include vinyl group, allyl group, propenyl group, and butenyl group.
Examples of the C₂₋₆ alkynyl group include ethynyl group and propynyl group.
The halogeno (C₂₋₆ alkenyl) group means a C₂₋₆ alkenyl group substituted with from one to three halogen atoms and examples include difluorovinyl group and dichloroallyl group.
Examples of the C₁₋₆ alkanoyl group include formyl group and acetyl group.
The halogeno(C₁₋₆ alkoxy)(C₁₋₆ alkyl) group means a (C₁₋₆ alkoxy)(C₁₋₆ alkyl) group substituted with from one to three halogen atoms and examples include trifluoromethoxymethyl group, dichloromethoxyethyl group, and fluoroethoxy(chloro)methyl group.
The halogeno(C₁₋₆ alkanoyl) group is a C₁₋₆ alkanoyl group substituted with from one to three halogen atoms and examples include fluoroacetyl group, trifluoroacetyl group, fluoropropionyl group, and trifluoropropionyl group.
The azido(C₁₋₆ alkyl) group means a C₁₋₆ alkyl group substituted with an azide group and examples include azidomethyl group and azidoethyl group.
The mono- or di-(C₁₋₆ alkyl)carbamoyl group means a carbamoyl group having a nitrogen atom substituted with one or two C₁₋₆ alkyl groups which are the same or different and examples include methylcarbamoyl group, dimethylcarbamoyl group, and N-ethyl-N-methylcarbamoyl group.
The C₁₋₆ alkanoylamino group means an amino group substituted with a C₁₋₆ alkanoyl group and examples include formylamino group, acetylamino group, and isobutyrylamino group.
The (C₁₋₆ alkanoyl)amino(C₁₋₆ alkyl) group means a C₁₋₆ alkyl group substituted with a (C₁₋₆ alkanoyl)amino group and examples include (acetylamino)methyl group and (acetylamino)ethyl group. The (C₁₋₆ alkanoyl)oxy(C₁₋₆ alkyl) group means a C₁₋₆ alkyl group substituted with a C₁₋₆ alkanoyloxy group and examples include formyloxymethyl group and acetyloxymethyl group.

R³ is preferably a hydrogen atom, halogen atom, C₁₋₆ alkyl group, (C₁₋₆ alkoxy)(C₁₋₆ alkyl) group, halogeno(C₁₋₆ alkyl) group, halogeno(C₁₋₆ alkoxy)(C₁₋₆ alkyl) group, or halogeno(C₁₋₆ alkanoyl) group, more preferably, a halogeno(C₁₋₆ alkyl) group, still more preferably a trifluoromethyl group, chlorodifluoromethyl group, difluoromethyl group, or difluoroethyl group. The description in R³ will equally apply to R^{3'} and R^{3"}.

### <R⁴>

R⁴ means a carboxyl group, carboxycarbonyl group, carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and arylalkenyl groups, carboxy(C₂₋₆ alkenyl) group, carboxy(C₁₋₆ alkoxy) group, carboxy(C₁₋₆ alkylthio) group, carboxy(C₁₋₆ alkyl)thio(C₁₋₆ alkyl) group, carboxyaryl group, carboxy(C₁₋₆ alkoxy)(C₁₋₆ alkyl) group, mono- or di-(carboxy(C₁₋₆ alkyl))carbamoyl group (with the proviso that the carboxy group of the groups may be esterified or amidated), or a group represented by the following formula:

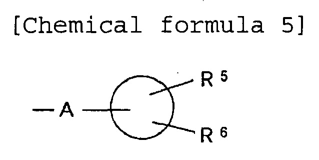

(wherein, A means a single bond, C₁₋₄ alkylene group which may have a hydroxy group and/or halogen atom, C₁₋₄ alkylenecarbonyl group which may have a hydroxy group and/or halogen atom, C₁₋₄ alkylenecarbonylamino group which may have, on the amino group thereof, a C₁₋₆ alkyl group as a substituent or C₁₋₄ alkyleneamino group having, on the amino group thereof, a C₁₋₆ alkyl group as a substituent, R⁵ means a carboxyl group, carboxycarbonyl group, carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and aryl(C₂₋₆ alkenyl) groups, carboxy(C₂₋₆ alkenyl) group, carboxy(C₁₋₆ alkoxy) group, carboxy(C₁₋₆ alkyl)thio group, carboxy(C₁₋₆ alkyl)thio(C₁₋₆ alkyl) group, carboxyaryl group, carboxy(C₁₋₆ alkoxy) (C₁₋₆ alkyl) group, or mono- or di-(carboxy(C₁₋₆ alkyl))carbamoyl group, with the proviso that the carboxy group of these groups may be esterified or amidated,
the group:

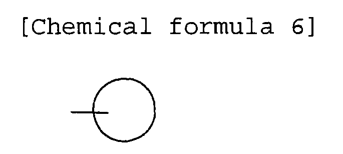

means a saturated or unsaturated cyclic hydrocarbon group or a saturated or unsaturated 4- to 7-membered heterocyclic group),
R⁶ means a hydrogen atom, hydroxy group, halogen atom, C₁₋₆ alkyl group, carboxy group, C₁₋₆ alkoxy group, carboxy(C₁₋₆ alkoxy) group, halogenoalkyl group, halogeno(C₁₋₆ alkoxy) group, or aralkyl group, with the proviso that the carboxy group of these groups may be esterified or amidated.
R⁴ will next be described more specifically.

Examples of the carboxy(C₁₋₆ alkyl) group of the carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and aryl(C₂₋₆ alkenyl) groups include carboxymethyl group, carboxyethyl group, and carboxypropyl group.
The C₁₋₆ alkoxyimino group which may be a substituent on the alkylene group of the carboxy(C₁₋₆ alkyl) group means an imino group substituted with a C₁₋₆ alkyl group and examples of it include methoxyimino group and ethoxyimino group. The aralkyl group which may also be a substituent means an aryl group substituted with a C₁₋₆ alkyl group and examples include benzyl group, phenethyl group, and naphthylmethyl group. The aryl(C₂₋₆ alkenyl) group means a C₂₋₆ alkenyl group substituted with an aryl group and examples include phenylvinyl group, phenylallyl group, and phenylpropenyl group.
The carboxy(C₂₋₆ alkenyl) group means a C₂₋₆ alkenyl group substituted with a carboxy group and examples include carboxyvinyl group, carboxyallyl group, and carboxypropenyl group.

The carboxy(C₁₋₆ alkoxy) group means a C₁₋₆ alkoxy group substituted with a carboxy group and examples include carboxymethoxy group and carboxyethoxy group.
The carboxy(C₁₋₆ alkyl)thio group means a C₁₋₆ alkylthio group substituted with a carboxy group and examples include carboxymethylthio group and carboxyethylthio group.
The carboxy(C₁₋₆ alkyl) thio (C₁₋₆ alkyl) group means a (C₁₋₆ alkyl) thio (C₁₋₆ alkyl) group substituted with a carboxy group and the (C₁₋₆ alkyl) thio (C₁₋₆ alkyl) group means a C₁₋₆ alkyl group substituted with a C₁₋₆ alkylthio group. Accordingly, examples of the carboxy(C₁₋₆ alkyl) thio (C₁₋₆ alkyl) group include carboxymethylthiomethyl group, carboxyethylthiomethyl group, and carboxymethylthioethyl group.
The carboxyaryl group means an aryl group substituted with a carboxy group and examples include carboxyphenyl group and carboxynaphthyl group.
The carboxy(C₁₋₆ alkoxy)(C₁₋₆ alkyl) group means a (C₁₋₆ alkoxy)(C₁₋₆ alkyl) group substituted with a carboxy group and examples include carboxymethoxymethyl group, carboxyethoxymethyl group, and carboxymethoxyethyl group.
The mono- or di-(carboxy(C₁₋₆ alkyl))carbamoyl group means a carbamoyl group substituted with one or two carboxy(C₁₋₆ alkyl) groups (same or different) and examples include N-carboxymethyl-carbamoyl group, N,N-di(carboxymethyl)carbamoyl group, and N-carboxyethyl-N-carboxymethyl-carbamoyl group.

The carboxy group of the above-described groups may be esterified or amidated and the term "esterified" or "amidated" means that the carboxy group of the above-described groups is esterified or amidated into a C₂₋₇ alkoxycarbonyl group, C₇₋₁₅ aryloxycarbonyl group, C₈₋₁₆ aralkyloxycarbonyl group, carbamoyl group, N-(C₁₋₆ alkyl)carbamoyl group, N,N-di(C₁₋₆ alkyl)carbamoyl group, N-(C₈₋₁₆ aralkyl) carbamoyl group, N-(C₁₋₆ alkyl)-N-(C₈₋₁₆ aralkyl)carbamoyl group, 1-pyrrolidinylcarbonyl group, piperidinocarbonyl group, morpholinocarbonyl group, or the like.
Examples of the C₂₋₇ alkoxycarbonyl group include methoxycarbonyl group and ethoxycarbonyl group.
Examples of the C₇₋₁₅ aryloxycarbonyl group include phenoxycarbonyl group and naphthoxycarbonyl group.
Examples of the C₈₋₁₆ aralkyloxycarbonyl group include benzyloxycarbonyl group.
Examples of the N-C₁₋₆ alkylcarbamoyl group include N-methylcarbamoyl group and N-ethylcarbamoyl group.
Examples of the N,N-di(C₁₋₆ alkyl)carbamoyl group include N,N-dimethylcarbamoyl group and N-ethyl-N-methylcarbamoyl group.
Examples of the N-C₈₋₁₆ aralkylcarbamoyl group include N-benzylcarbamoyl group.
Examples of the N-C₁₋₆ alkyl-N-C₈₋₁₆ aralkylcarbamoyl group include N-benzyl-N-methylcarbamoyl group.

The group represented by the following formula:

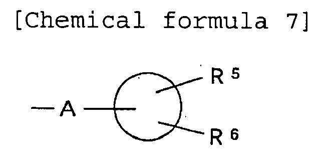

will next be described specifically.
In the group, examples of the C₁₋₄ alkylene group as A include a methylene group, ethylene group, trimethylene group, propylene group, and ethylethylene group. Examples of the C₁₋₄ alkylenecarbonyl group include a methylenecarbonyl group, ethylenecarbonyl group, trimethylenecarbonyl group, propylenecarbonyl group, and ethylethylenecarbonyl group. A is preferably a C₁₋₄ alkylene group which may have a hydroxy group or a C₁₋₄ alkylenecarbonyl group which may have a hydroxy group.

R⁵ has the same meaning as that of R⁴. R⁵ is preferably a carboxy group, carboxy(C₁₋₆ alkyl) group which may have, on the alkylene group thereof, from one to four substituents selected from the class consisting of a hydroxy group, halogen atoms, C₁₋₆ alkoxyimino groups, aralkyl groups, and aryl (C₂₋₆ alkenyl) groups, carboxy(C₁₋₆ alkoxy)group, carboxy (C₁₋₆ alkylthio)group, or carboxy (C₁₋₆ alkoxy) (C₁₋₆ alkyl) group.

R⁶ means a hydrogen atom, hydroxy group, halogen atom, C₁₋₆ alkyl group, carboxy group, C₁₋₆ alkoxy group, carboxy(C₁₋₆ alkoxy) group, halogenoalkyl group, halogeno(C₁₋₆ alkoxy) group, or aralkyl group. The carboxy group of these groups may be esterified or amidated. Substituents in R⁶ are similar to those described above. R⁶ is preferably a hydrogen atom.
The group:

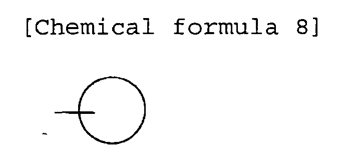

means a saturated or unsaturated cyclic hydrocarbon group or a saturated or unsaturated 4- to 7-membered heterocyclic group.
Examples of the saturated or unsaturated cyclic hydrocarbon group include a cyclopentyl group, cyclopentenyl group, cyclohexyl group, cyclohexenyl group, and phenyl group.
Examples of the saturated or unsaturated 4- to 7-membered heterocyclic group include furyl group, thienyl group, pyrrolyl group, oxazolyl group, isoxazolyl group, thiazolyl group, isothiazolyl group, imidazolyl group, pyrazolyl group, 1,2,3-oxadiazolyl group, 1,2,4-oxadiazolyl group, 1,3,4-oxadiazolyl group, furazanyl group, 1,2,3-thiadiazolyl group, 1,2,4-thiadiazolyl group, 1,3,4-thiadiazolyl group, 1,2,3-triazolyl group, 1,2,4-triazolyl group, tetrazolyl group, piperidinyl group, pyrrolidinyl group, azetidinyl group, pyridyl group, pyridazinyl group, pyrimidinyl group, pyrazinyl group, and piperazinyl group. They may have one or two oxo groups.
The group

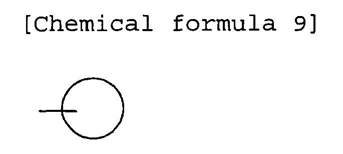

is preferably a saturated or unsaturated 4- to 7-membered heterocyclic group, of which oxazolyl group, thiazolyl group, pyrazolyl group, tetrazolyl group, piperidinyl group, pyrrolidinyl group, azetidinyl group, or the like is preferred.

In the present invention, R⁴ is preferably a carboxy(C₁₋₆ alkyl) group which may have, on the alkylene group thereof, from one to four substituents selected from the class consisting of a hydroxy group, halogen atoms, C₁₋₆ alkoxyimino groups, aralkyl groups, and aryl(C₂₋₆ alkenyl) groups, a carboxy(C₁₋₆ alkoxy)(C₁₋₆ alkyl) group (with the proviso that the carboxy group of these groups may be esterified or amidated), or
the group represented by the following formula:

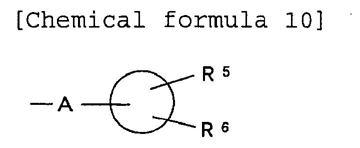

(in the group, A means a C₁₋₄ alkylene group which may have a hydroxy group or a C₁₋₄ alkylenecarbonyl group which may have a hydroxy group, R⁵ means a carboxy group, carboxy(C₁₋₆ alkyl) group which may have on the alkylene group thereof, from one to four substituents selected from the class consisting of a hydroxy group, halogen atoms, C₁₋₆ alkoxyimino groups, aralkyl groups, and aryl(C₂₋₆ alkenyl) groups, carboxy(C₁₋₆ alkoxy)group, carboxy(C₁₋₆ alkyl)thio group, or carboxy(C₁₋₆ alkoxy)(C₁₋₆ alkyl) group (with the proviso that the carboxy group of these groups may be esterified or amidated); R⁶ preferably means a hydrogen atom; and the group:

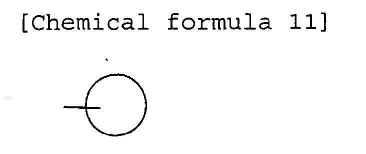

preferably means a saturated or unsaturated 4- to 7-membered heterocyclic group).

### <X, Y and Z>

In the present invention, following compounds are preferred as the parent of the compound represented by the formula (I).
(1) Compounds having CH₂ as X, C-R^{3'} as Y, and C-R^{3"} as Z.
(2) Compounds having CH₂ as X, N as Y, and N as Z.
(3) Compounds having O as X, N as Y, and N as Z.
(4) Compounds having O as X, C-R^{3'} as Y, and C-R^{3"} as Z.
(5) Compounds having O as X, N as Y, and C-R^{3"} as Z.
(6) Compounds having S as X, N as Y, and N as Z.
(7) Compounds having S as X, N as Y, and C-R^{3"} as Z.
(8) Compounds having S as X, C-R^{3'} as Y, and C-R^{3"} as Z.

Examples of the salt of the compound according to the present invention include pharmaceutically acceptable salts, for example, alkali metal or alkaline earth metal salts such as sodium salt, potassium salt, calcium salt, and magnesium salt, organic amine salts such as ammonium salt, trimethylamine salt, triethylamine salt, dicyclohexylamine salt, and ethanolamine salt, mineral acid salts such as hydrochloride, nitrate, and sulfate, and organic acid salts such as tartrate, fumarate, and methanesulfonate.
The compound or salt thereof according to the present invention may be present as a hydrate or various solvates and they are also embraced in the present invention. The compounds of the present invention may have a stereoisomer such as optical isomer, diastereomer or geometrical isomer and all of these isomers and mixtures thereof may also be embraced in the present invention.

The preparation process of the compounds of the present invention will hereinafter be described. In each reaction, when a raw material compound has, as a substituent, an amino group, carboxyl group, hydroxy group, or the like, a protecting group ordinarily employed in peptide chemistry may be introduced thereinto. After completion of the reaction, the protecting group is removed if necessary to obtain a desired compound. Examples of the amino-protecting group include substituted or unsubstituted C₁₋₆ alkanoyl groups (such as formyl group, acetyl group, and ethylcarbonyl group), C₂₋₇ alkoxycarbonyl groups (such as methoxycarbonyl group and ethoxycarbonyl group), and C₈₋₁₆ aralkyloxycarbonyl groups (such as benzyloxycarbonyl group). Examples of the carboxy-protecting group include substituted or unsubstituted C₁₋₆ alkyl groups (such as methyl group, ethyl group, and tertiary butyl group), C₂₋₆ alkenyl groups (such as allyl group), substituted or unsubstituted C₆₋₁₉ aralkyl groups (such as benzyl group, nitrobenzyl group, 4-methoxybenzyl group, and triphenylmethyl group). Examples of the hydroxyl-protecting group include substituted or unsubstituted aralkyl groups and methoxymethyl group, benzyloxymethyl group, tetrahydropyranyl group, tert-butyldimethylsilyl group, tert-butyldiphenylsilyl group, and 2-trimethylsilylethyl group.

Of the compounds (I), compounds represented by the formula (I-1) can be prepared, for example, in accordance with the following reaction scheme.

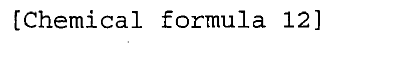

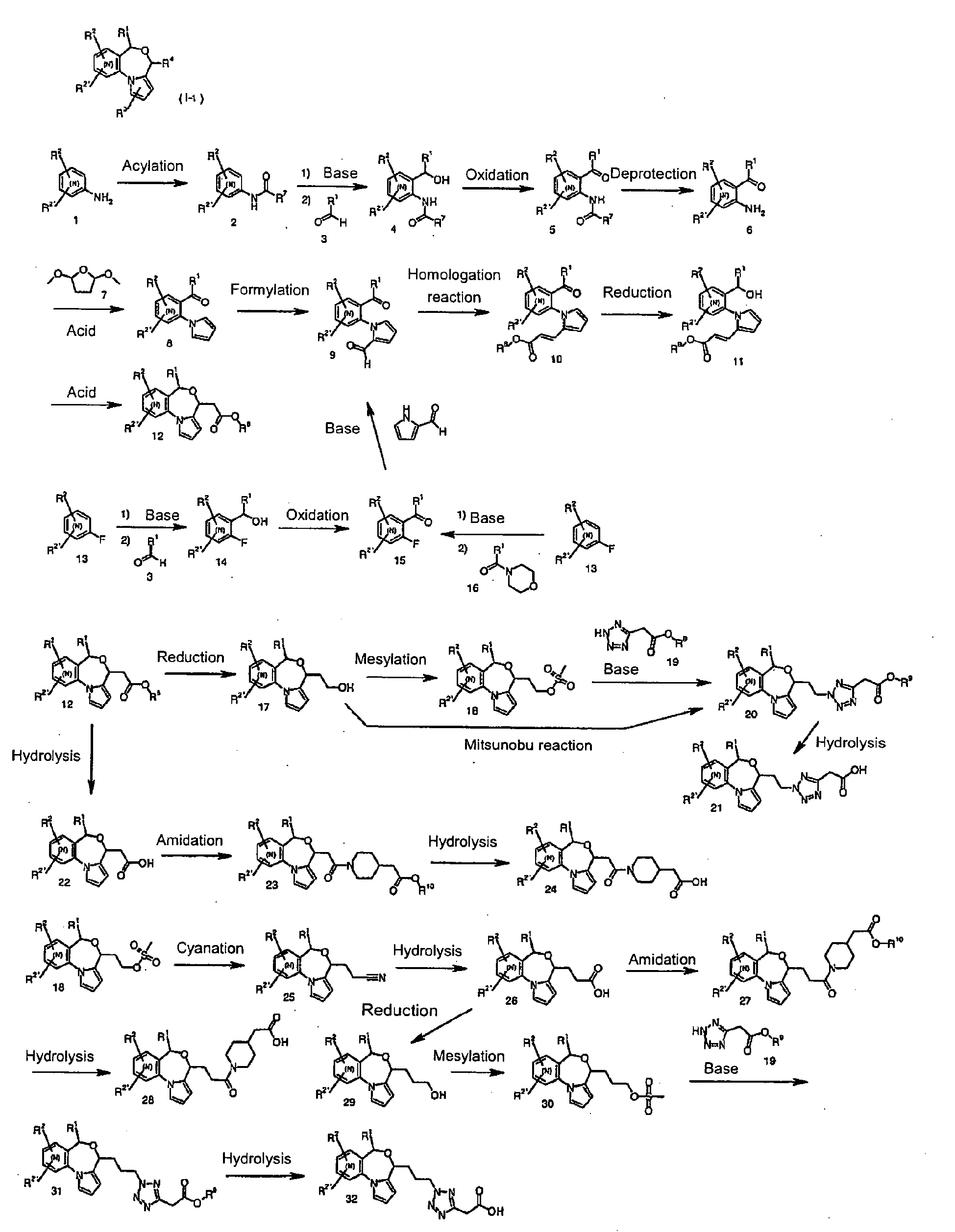

[wherein, R¹, R², and R^{2'} have the same meanings as described above, and R⁷, R⁸, R⁹, and R¹⁰ each independently means a substituted or unsubstituted C₁₋₆ alkyl group, a C₂₋₆ alkenyl group, a substituted or unsubstituted C₆₋₁₉ aralkyl group].

Compound 6 can be prepared in accordance with the process disclosed, for example, in Journal of Organic Chemistry, 45, 4798-4801(1980) or a process based thereon.

Compound 8 can be prepared from Compound 6.

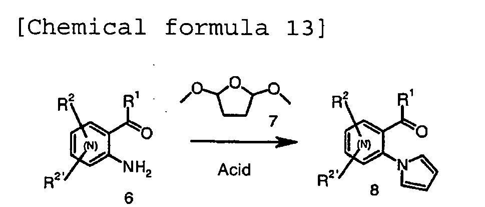

Synthesis of Compound 8 from Compound 6 is achieved reacting Compound 6 with a reactive acetal derivative (Compound 7) such as 2,5-dimethoxytetrahydrofuran or 1,1,4,4-tetramethoxybutane. Compound is usually reacted with from 1 mole to excess moles, preferably from 1 to 2 moles, per mole of Compound 6, of 2,5-dimethoxytetrahydrofuran in acetic acid or a mixture of acetic acid with an inert solvent such as dichloroethane or dioxane in the presence of a salt or without adding a salt at a temperature of from -78°C to 200°C, preferably from 0°C to 160°C. Examples of the salt include carboxylates such as sodium acetate and potassium formate and the salt is used in an amount of from catalytic to excess amount, preferably from 1/10 to 2 times the weight of the reaction solvent. The reaction time is from 1 minute to 48 hours, preferably from 5 minutes to 1 hour.

Compound 9 can be prepared from Compound 8.

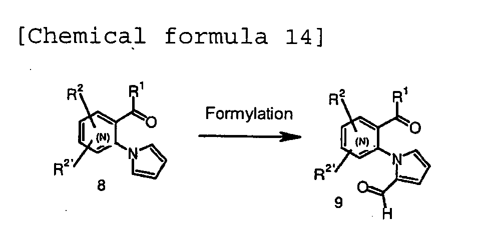

Synthesis of Compound 9 from Compound 8 is achieved reacting Compound 8 with a formylation reagent. Described specifically, Compound 8 is dissolved in dimethylformamide or a solution of dimethylformamide in dichloromethane, dichloroethane, or the like and phosphorus oxychloride or thionyl chloride is added to the resulting solution at from -78°C to 50°C, preferably from -50°C to room temperature. The resulting mixture is reacted at from -20°C to room temperature for from 2 minutes to 1 hour to form chloromethylenedimethyliminium chloride. When the solvent such as dichloromethane or dichloroethane has not been added, the solvent such as dichloromethane or dichloroethane is then added. When the solvent has already been added, on the other hand, the reaction with Compound 8 is effected at from -78°C to 120°C, preferably at from -50°C to 80°C without adding the solvent. The reaction time is from 1 minute to 48 hours, preferably from 5 minutes to 2 hours.

Compound 10 can be prepared from Compound 9.

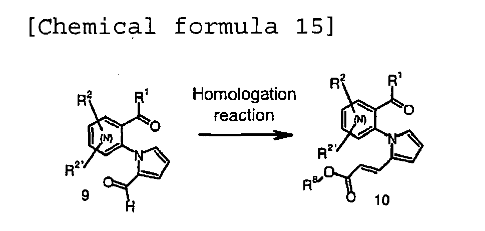

Synthesis of Compound 10 from Compound 9 can be achieved treating Compound 9 with a phosphonylidene compound or with a phosphonate anion. For example, Compound 9 is treated with a phosphonylidene compound in an inert solvent at from 0°C to the boiling point of the solvent used for the reaction, preferably from 20°C to 130°C. Examples of the phosphonylidene compound include methyltriphenyl phosphonylidene acetate, ethyltriphenyl phosphonylidene acetate, tertiary butyltriphenyl phosphonylidene acetate, allyltriphenyl phosphonylidene acetate, and benzyltriphenyl phosphonylidene acetate. The phosphonylidene compound is used in an amount of from 1 mole to excess moles, preferably from 1 mole to 5 moles per mole of Compound 9. Examples of the inert solvent include toluene, benzene, dichloroethane, tetrahydrofuran, dioxane, and ethyl acetate, and mixtures thereof. The reaction time is from 5 minutes to 60 hours, preferably from 10 minutes to 24 hours. Alternatively, Compound 9 is treated with a phosphonate anion, which has been prepared treating a phosphonate compound with a base, at from -78°C to the boiling point of the solvent used for the reaction, preferably from -20°C to 100°C. Examples of the phosphonate compound include triethylphosphonoacetate, trimethylphosphonoacetate, tertiary butyldiethylphosphonoacetate, tertiary butyldimethylphosphonoacetate, allyldiethylphosphonoacetate, allyldimethylphosphonoacetate, benzyldiethylphosphonoacetate, and benzyldimethylphosphonoacetate. It is used in an amount of from 1 mole to excess moles, preferably from 1 mole to 5 moles per mole of Compound 9. Examples of the inert solvent include toluene, benzene, dichloroethane, tetrahydrofuran, dioxane, and ethyl acetate, and mixtures thereof. The reaction time is from 5 minutes to 60 hours, preferably from 10 minutes to 24 hours.

Compound 11 can be prepared from Compound 10.

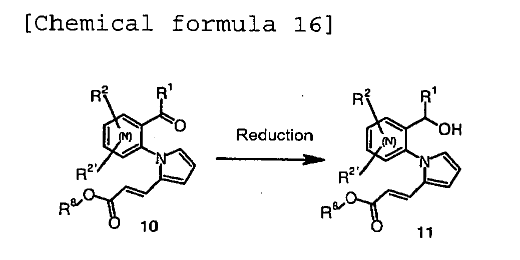

Synthesis of Compound 11 from Compound 10 can be achieved treating Compound 10 with a reducing agent. For example, Compound 10 is treated with a reducing agent in methanol, ethanol, tetrahydrofuran, diethyl ether, dioxane, dichloromethane, toluene, or the like, or a mixed solvent thereof at from -78°C to the boiling point of the solvent used for the reaction, preferably from -20°C to 100°C. Examples of the reducing agent include sodium borohydride, lithium borohydride, lithium aluminum hydride, diisobutylaluminum hydride, borane dimethylsulfide complex and borane tetrahydrofuran complex. It is used in an amount of from 1 mole to excess moles, preferably from 1 mole to 5 moles, per mole of Compound 10. The reaction time is from 5 minutes to 60 hours, preferably from 10 minutes to 24 hours.

Compound 12 can be prepared from Compound 11.

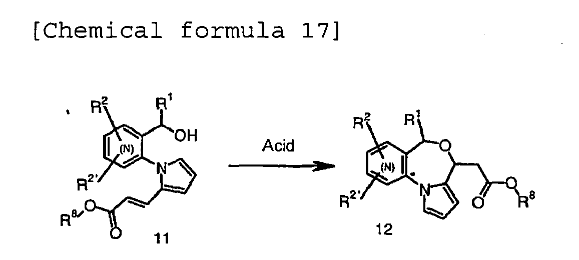

Synthesis of Compound 12 from Compound 11 can be achieved treating Compound 11 with an acid. For example, Compound 11 is treated with an acid in dichloromethane, dichloroethane, tetrahydrofuran, diethyl ether, dioxane, toluene, ethyl acetate, or the like, or a mixed solvent thereof at from -78°C to the boiling point of the solvent used for the reaction, preferably at from -20°C to 100°C. Examples of the acid include trifluoroacetic acid, acetic acid, sulfuric acid, toluenesulfonic acid, camphorsulfonic acid, boron trifluoride-diethyl ether complex, aluminum chloride, and pyridinium-para-toluenesulfonate. It is used in an amount of from 0.001 mole to excess moles, preferably from 0.01 mole to 10 moles per mole of Compound 11. The reaction time is from 5 minutes to 60 hours, preferably from 10 minutes to 24 hours.

Compound 14 can be prepared from Compound 13.

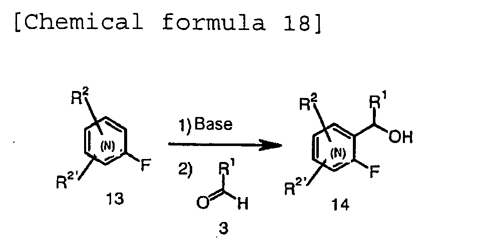

Synthesis of Compound 14 can be achieved carrying out deprotonation of Compound 13 with a deprotonation agent and then, reacting the resulting compound with an aldehyde. Described specifically, Compound 13 is treated with from 1 mole to excess moles, preferably from 1 mole to 1.5 moles of a deprotonation agent such as primary butyl lithium, secondary butyl lithium, or tertiary butyl lithium, in a solvent not adversely affecting the reaction such as toluene, tetrahydrofuran, dioxane, diethyl ether, or hexane, or a mixed solvent thereof at a reaction temperature of from -100°C to 50°C, preferably from -85°C to 10°C. The reaction time is from 1 minute to 12 hours, preferably from 10 minutes to 5 hours. The reaction mixture is then reacted with from 1 mole to excess moles, preferably from 1 mole to 5 moles of an aldehyde such as 2,3-dimethoxybenzaldehyde at a reaction temperature of from -100°C to 100°C, preferably from -78°C to 40°C. The reaction time is from 1 minute to 12 hours, preferably from 10 minutes to 5 hours.

Compound 15 can be prepared from Compound 14.

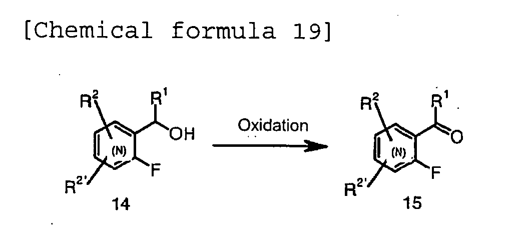

Synthesis of Compound 15 from Compound 14 can be achieved reacting Compound 14 with an oxidizing agent. Described specifically, Compound 14 is reacted with from 1 mole to excess moles, preferably from 1 mole to 20 moles of an oxidizing agent such as manganese dioxide at from -30°C to 200°C, preferably from 0°C to 100°C in a solvent not adversely affecting the reaction such as benzene, toluene, dichloroethane, chloroform, dichloromethane, dioxane, tetrahydrofuran, dimethylsulfoxide, or dimethylformamide, or a mixed solvent thereof. The reaction time is from 5 minutes to 200 hours, preferably from 1 hour to 60 hours.

Compound 15 can be prepared from Compound 13.

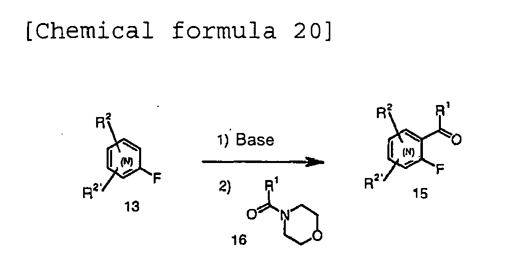

Synthesis of Compound 15 from Compound 13 can be achieved deprotonating Compound 13 with a deprotonation agent and then, reacting the resulting compound with carboxylic acid-morpholineamide, N,O-dimethylamide, dialkylamide, ester, or the like. Described specifically, Compound 13 is treated with from 1 mole to excess moles, preferably from 1 mole to 1.5 moles of a deprotonation agent such as primary butyl lithium, secondary butyl lithium, or tertiary butyl lithium in a solvent not adversely affecting the reaction such as toluene, tetrahydrofuran, dioxane, diethyl ether, or hexane, or a mixed solvent thereof at a reaction temperature of from -100°C to 50°C, preferably from -85°C to 10°C. The reaction time is from 1 minute to 12 hours, preferably from 10 minutes to 5 hours. The reaction mixture is then reacted with from 1 mole to excess moles, preferably from 1 mole to 5 moles of morpholineamide, N,O-dimethylamide, dialkylamide, ester, or the like at a reaction temperature of from -100°C to 100°C, preferably from -78°C to 40°C. The reaction time is from 1 minute to 12 hours, preferably from 10 minutes to 5 hours.

Compound 9 can be prepared from Compound 15.

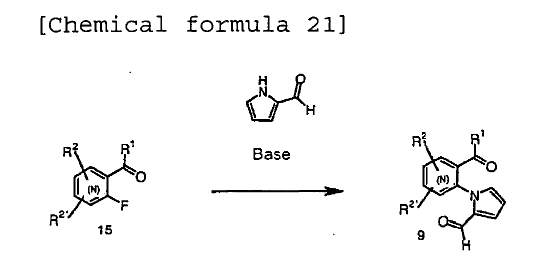

Synthesis of Compound 9 from Compound 15 is achieved treating Compound 15 with 2-formylpyrrole serving as a nucleophilic agent in a solvent not adversely affecting the reaction such as dioxane, tetrahydrofuran, diethyl ether, dimethylformamide, dimethylsulfoxide, toluene, or benzene, or a mixed solvent thereof at from -10°C to the boiling point of the solvent used for the reaction, preferably from 0°C to 150°C. If necessary, a carbonate such as sodium hydrogen carbonate or potassium carbonate, a hydroxide such as sodium hydroxide, a metal hydride such as sodium hydride, an alkoxide such as tertiary butoxy potassium, or an amine such as triethylamine, diisopropylethylamine, N-methylmorpholine, 4-dimethylaminopyridine, or 1,8-diazabicyclo[5,4,0]undec-7-ene can be added as the base.
It is used in an amount of from 1 mole to excess moles, preferably from 1 mole to 5 moles per mole of Compound 15. The reaction time is from 5 minutes to 200 hours, preferably from 30 minutes to 20 hours.

Synthesis of Compound 9 can also be achieved by a process of reacting Compound 15 with pyrrole, introducing a hydroxymethyl group into the pyrrole ring and then oxidizing the resulting hydroxy group or a process of formylating the pyrrole ring.

Compound 17 can be prepared from Compound 12.

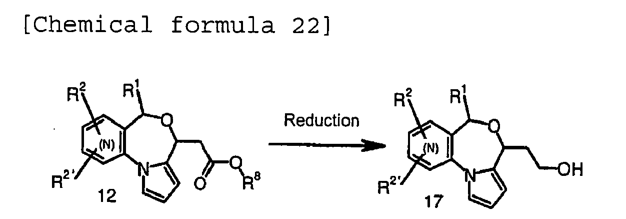

Synthesis of Compound 17 from Compound 12 can be achieved treating Compound 12 with a reducing agent. For example, Compound 12 is treated with a reducing agent in a solvent such as methanol, ethanol, tetrahydrofuran, diethyl ether, dioxane, dichloromethane, or toluene, or a mixed solvent thereof at from -78°C to the boiling point of the solvent used for the reaction, preferably from -20°C to 100°C. Examples of the reducing agent include sodium borohydride, lithium borohydride, lithium aluminum hydride, diisobutylaluminum hydride, borane dimethylsulfide complex, and borane tetrahydrofuran complex. It is used in an amount of from 1 mole to excess moles, preferably from 1 mole to 5 moles per mole of Compound 12. The reaction time is from 5 minutes to 60 hours, preferably from 10 minutes to 24 hours.

Compound 18 can be prepared from Compound 17.

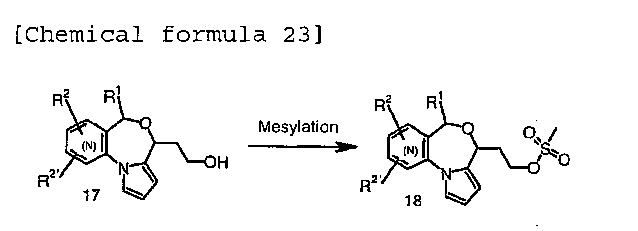

Synthesis of Compound 18 from Compound 17 is achieved treating Compound 17 with a mesylation agent. For example, Compound 17 is treated with a mesylation agent in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 50°C. Examples of the mesylation agent include methanesulfonylation agents such as methanesulfonyl chloride and methanesulfonic anhydride. It is used in an amount of from 1 mole to excess moles, preferably from 1 mole to 5 moles per mole of Compound 17. Further, a base such as triethylamine, diisopropylethylamine, N-methylmorpholine, 4-dimethylaminopyridine, or 1,8-diazabicyclo[5,4,0]undec-7-ene is used in an amount of from 1 mole to excess moles, preferably from 1 mole to 10 moles per mole of Compound 17. The reaction time is from 1 minute to 60 hours, preferably from 10 minutes to 5 hours.

Compound 20 can be prepared from Compound 18.

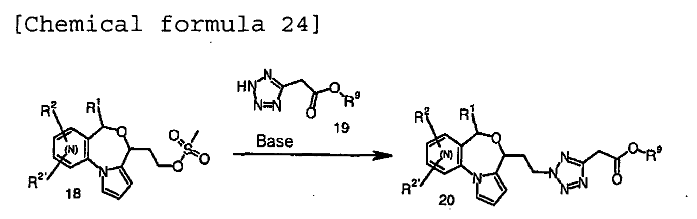

Synthesis of Compound 20 from Compound 18 is achieved reacting Compound 18 with Compound 19 in the presence of a base. For example, Compound 18 is treated with Compound 19 in the presence of a base in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from -20°C to 100°C. From 1 mole to excess moles, preferably from 1 mole to 5 moles, per mole of Compound 18, of Compound 19 and the like are reacted with Compound 18 in the presence of from 1 mole to excess moles, preferably from 1 mole to 10 moles of a base such as potassium carbonate, sodium carbonate, or tertiary butoxy potassium. From a catalytic amount to excess amount, preferably from a catalytic amount to 5 equivalents of an iodide such as tetrabutylammonium iodide or potassium iodide can be added as needed. The reaction time is from 1 minute to 240 hours, preferably from 10 minutes to 60 hours.

Compound 20 can be prepared from Compound 17.

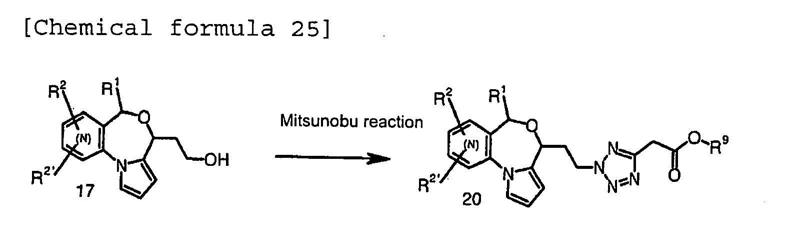

Synthesis of Compound 20 from Compound 17 can be achieved directly without preparing Compound 18. For example, it can be obtained in accordance with Mitsunobu reaction using a compound having an acidic proton. For example, Mitsunobu reaction can be effected treating 1 mole of Compound 17 and from 1 mole to excess moles, preferably from 1 mole to 3 moles, of each of a nucleophilic agent such as tetrazole acetate, diethyl azodicarboxylate, and triphenylphosphine in an inert solvent such as dichloromethane, dichloroethane, tetrahydrofuran, acetonitrile, N,N-dimethylformamide, ethyl acetate, or toluene at from -78°C to the boiling point of the solvent, preferably from -50°C to 60°C.

Compound 21 can be prepared from Compound 20.

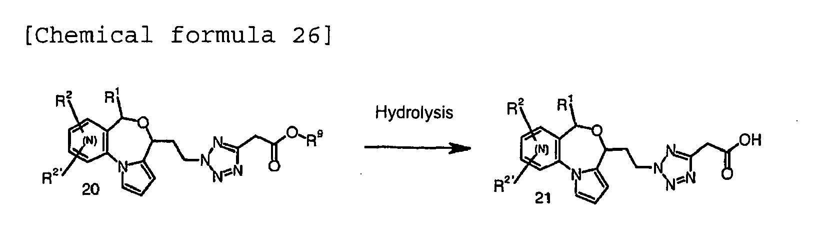

Synthesis of Compound 21 from Compound 20 is achieved by a conventional hydrolysis reaction, for example, hydrolysis reaction in which Compound 20 is treated with a metal hydroxide such as sodium hydroxide or lithium hydroxide or a carbonate such as sodium carbonate or potassium carbonate (from 1 mole to excess moles per mole of Compound 20) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing deallylation reaction in the presence of a palladium catalyst. The process differs, depending on the kind of the ester.

Compound 22 can be prepared from Compound 12.

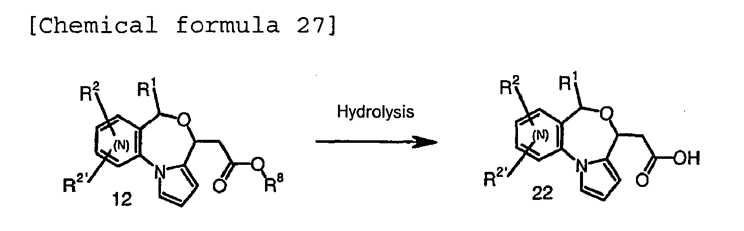

Synthesis of Compound 22 from Compound 12 is achieved by a conventional hydrolysis reaction, for example, hydrolysis reaction in which Compound 12 is treated with a metal hydroxide such as sodium hydroxide or lithium hydroxide or a carbonate such as sodium carbonate or potassium carbonate (from 1 mole to excess moles per mole of Compound 12) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 23 can be prepared from Compound 22.

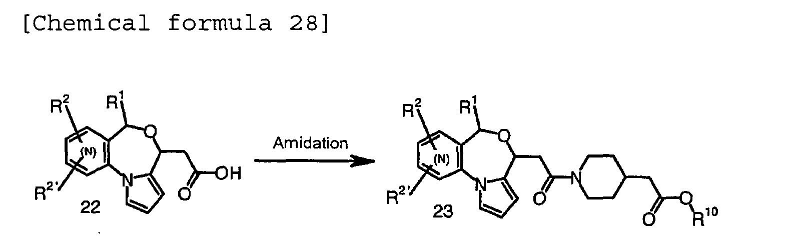

Synthesis of Compound 23 from Compound 22 is achieved acylating Compound 22. Compound 22 is condensed with piperidine-4-acetate ester or salt thereof. For example, Compound 22 is reacted using from 1 mole to excess moles, preferably from 1 mole to 5 moles, per mole of Compound 22, of piperidine-4-acetate ester or salt thereof in the presence of a condensation agent such as N,N-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate or 2-(1H-benzotriazol-1-yl)-1,1,3,3-tetramethyluronium tetrafluoroborate in an inert solvent such as benzene, toluene, xylene, diethyl ether, dichloroethane, chloroform, dichloromethane, dioxane, tetrahydrofuran, dimethylsulfoxide, or dimethylformamide, or a mixture thereof at from -30°C to the boiling point of the solvent used for the reaction, preferably from 0°C to 50°C. The above-described condensation agent is used in an amount of from 1 mole to excess moles, preferably from 1 mole to 5 moles per mole of Compound 22. The above-described reaction may be performed in the presence of a base such as triethylamine, diisopropylethylamine, N-methylmorpholine, 4-dimethylaminopyridine, or 1,8-diazabicyclo[5,4,0]undec-7-ene if necessary. Moreover, an N-hydroxy compound such as 1-hydroxybenzotriazole, N-hydroxysuccinimide, or N-hydroxyphthalimide, or a phenol compound such as 4-nitrophenol, 2,4-dinitrophenol, 2,4,5-trichlorophenol, or pentachlorophenol may be added as a reaction accelerator. The reaction time is from 10 minutes to 48 hours, preferably from 30 minutes to 12 hours.

Compound 24 can be prepared from Compound 23.

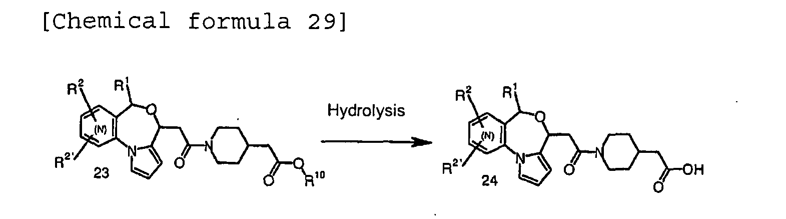

Synthesis of Compound 24 from Compound 23 is achieved by a conventional hydrolysis reaction, for example, hydrolysis reaction in which Compound 23 is treated with a metal hydroxide such as sodium hydroxide or lithium hydroxide or a carbonate such as sodium carbonate or potassium carbonate (from 1 mole to excess moles per mole of Compound 23) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing deallylation reaction in the presence of a palladium catalyst. The process differs, depending on the kind of the ester.

Compound 25 can be prepared from Compound 18.

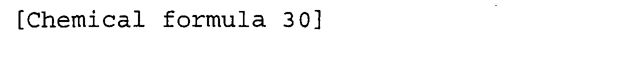

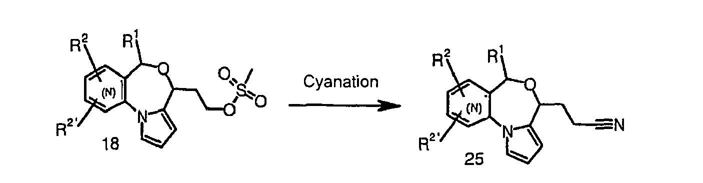

Synthesis of Compound 25 from Compound 18 is achieved reacting Compound 18 with a cyanation agent. For example, Compound 18 is treated with a cyanation agent such as sodium cyanide, potassium cyanide, or ammonium cyanide in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from 0°C to the boiling point of the solvent used for the reaction, preferably from 10°C to 150°C. The cyanation agent is used in an amount of from 1 mole to excess moles, preferably from 1 mole to 5 moles per mole of Compound 18. The reaction time is from 1 minute to 240 hours, preferably from 10 minutes to 60 hours.

Compound 26 can be prepared from Compound 25.

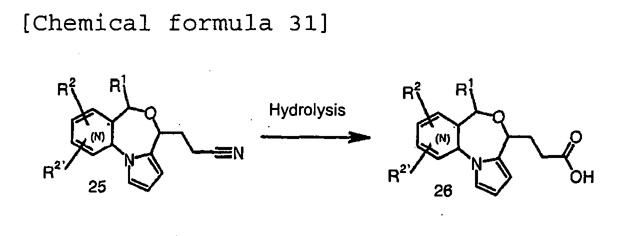

Synthesis of Compound 26 from Compound 25 is achieved by a conventional hydrolysis reaction, for example, treatment of Compound 25 with from 1 mole to excess moles, preferably from 1 mole to 5 moles, per mole of Compound 25, of a metal hydroxide such as sodium hydroxide, lithium hydroxide, barium hydroxide, or cesium hydroxide at from 0 to 200°C, preferably from 0 to 120°C in a solvent (for example, an alcohol solvent such as methanol, ethanol, or isopropanol, an ether solvent such as tetrahydrofuran, or water, or a mixed solvent thereof).

Compound 29 can be prepared from Compound 26.

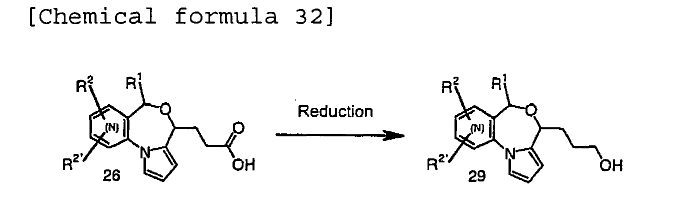

Synthesis of Compound 29 from Compound 26 can be achieved treating Compound 26 with a reducing agent. For example, Compound 26 is treated with a reducing agent in tetrahydrofuran, diethyl ether, dioxane, or the like, or a mixed solvent thereof at from -78°C to the boiling point of the solvent used for the reaction, preferably from -20°C to 100°C. Examples of the reducing agent include lithium aluminum hydride, borane dimethylsulfide complex, and borane tetrahydrofuran complex. It is used in an amount of from 1 mole to excess moles, preferably from 1 mole to 5 moles per mole of Compound 26. The reaction time is from 5 minutes to 60 hours, preferably from 10 minutes to 24 hours.

Synthesis of Compound 28 can be achieved based on the process employed for the synthesis of Compound 24. Compound 32 can be synthesized based on the process employed for the synthesis of Compound 21.

Of Compounds (I), compounds represented by the formula (I-2) can be prepared, for example, in accordance with the following reaction scheme.

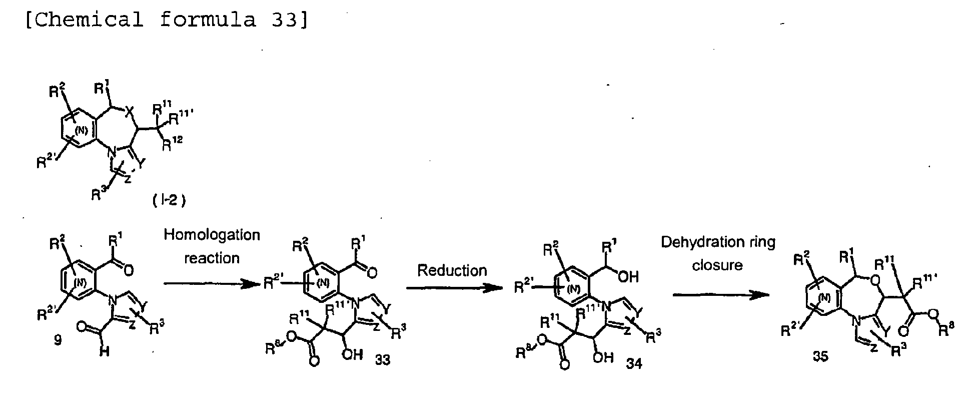

[wherein, R¹, R², and R^{2'} have the same meanings as described above, R⁸ means a substituted or unsubstituted C₁₋₆ alkyl group, a C₂₋₆ alkenyl group, or a substituted or unsubstituted C₁₋₆ aralkyl group, R¹¹ and R^{11'} each independently means a hydrogen atom, hydroxy group, halogen atom, aryl group, arylalkenyl group, or alkyl group, and R¹² means a carboxy group or carboxyalkyl group (with the proviso that carboxy group of these groups may be esterified or amidated)].

Compound 33 can be prepared from Compound 9.

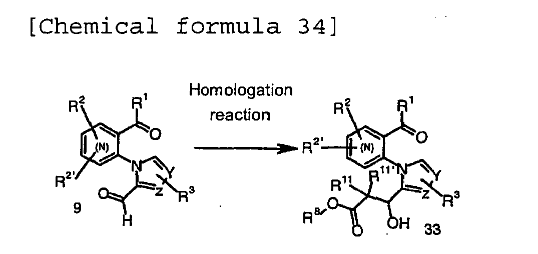

Synthesis of Compound 33 from Compound 9 can be achieved treating Compound 9 with an organic zinc compound. The organic zinc compound can be prepared by treating a substituted bromoacetate ester and zinc in an inert solvent at from 0°C to the boiling point of the solvent used for the reaction, preferably from 20°C to 130°C. Examples of the inert solvent include toluene, benzene, dichloroethane, tetrahydrofuran, dioxane, diethyl ether, and ethyl acetate, and mixtures thereof. The reaction time is from 5 minutes to 60 hours, preferably from 10 minutes to 24 hours. Compound 33 is synthesized by treating Compound 9 with the resulting organic zinc compound in an inert solvent at from -78°C to the boiling point of the solvent used for the reaction, preferably from -20°C to 150°C. The organic zinc compound is used in an amount of from 1 mole to excess moles, preferably from 1 mole to 5 moles per mole of Compound 9. Examples of the inert solvent include toluene, benzene, dichloroethane, tetrahydrofuran, dioxane, diethyl ether, and ethyl acetate, and mixtures thereof. The reaction time is from 5 minutes to 60 hours, preferably from 10 minutes to 24 hours.

Compound 34 can be prepared from Compound 33.

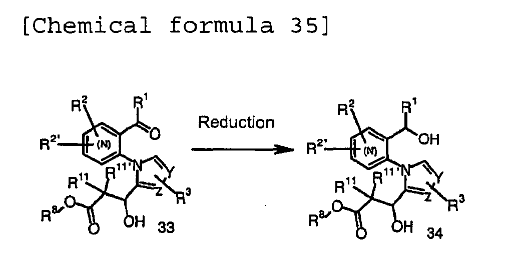

Synthesis of Compound 34 from Compound 33 can be achieved treating Compound 33 with a reducing agent. For example, Compound 33 is treated with a reducing agent in methanol, ethanol, tetrahydrofuran, diethyl ether, dioxane, dichloromethane, toluene, or the like, or a mixed solvent thereof at from -78°C to the boiling point of the solvent used for the reaction, preferably from -20°C to 100°C. Examples of the reducing agent include sodium borohydride, lithium borohydride, lithium aluminum hydride, diisobutylaluminum hydride, borane dimethylsulfide complex, and borane tetrahydrofuran complex. It is used in an amount of from 1 mole to excess moles, preferably from 1 mole to 5 moles per mole of Compound 33. The reaction time is from 5 minutes to 60 hours, preferably from 10 minutes to 24 hours.

Compound 35 can be prepared from Compound 34.

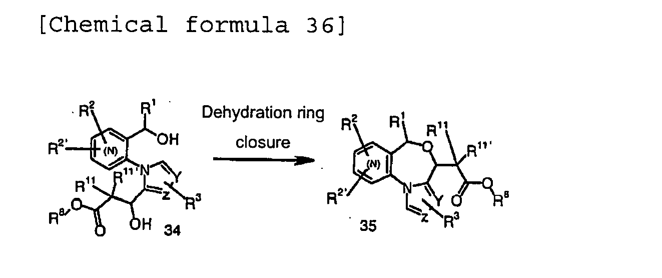

Synthesis of Compound 35 from Compound 34 can be achieved treating Compound 34 with a dehydrating agent. For example, Compound 34 is treated with a dehydrating agent in dichloromethane, dichloroethane, tetrahydrofuran, diethyl ether, dioxane, toluene, ethyl acetate, or the like, or a mixed solvent thereof at from -78°C to the boiling point of the solvent used for the reaction, preferably from -20°C to 150°C. Examples of the dehydrating agent include diphosphorus pentoxide and polyphosphoric acid. It is used in an amount of from 1 mole to excess moles, preferably from 1 mole to 10 moles, per mole of Compound 34. The reaction time is from 5 minutes to 60 hours, preferably from 10 minutes to 12 hours.

In accordance with the processes employed for the synthesis of Compound 21, Compound 22, Compound 24, Compound 28, and Compound 32 while using Compound 35, the corresponding compounds can be synthesized, respectively.

Of the synthesis intermediates of Compound (I), the following Compound 38 can be prepared, for example, in the following reaction scheme.

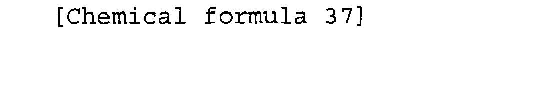

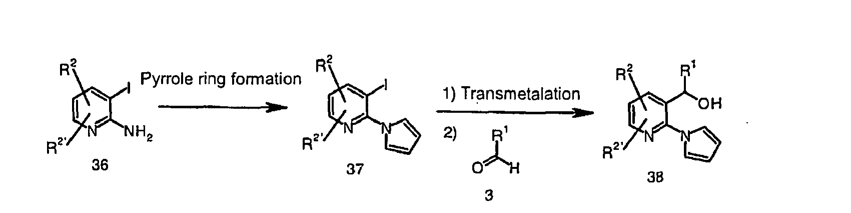

[wherein, R¹, R², and R²' have the same meanings as described above].

Compound 37 can be prepared from Compound 36.

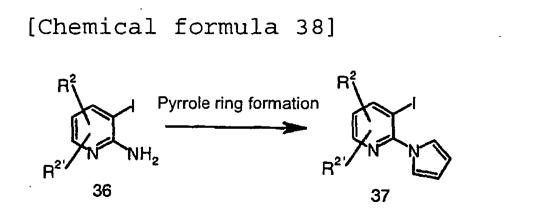

Synthesis of Compound 37 from Compound 36 can be achieved reacting Compound 36 with a reactive acetal derivative such as 2,5-dimethoxytetrahydrofuran or 1,1,4,4-tetramethoxybutane. Compound 36 is reacted with from 1 mole to excess moles, preferably from 1 mole to 2 moles of 2,5-dimethoxytetrahydrofuran at from -78°C to 200°C, preferably from 0°C to 160°C in acetic acid, dichloroethane, tetrahydrofuran, dioxane, or the like, or a mixed solvent thereof in the presence of a salt or without adding a salt. Examples of the salt include carboxylates such as sodium acetate and potassium formate and hydrochlorides such as 4-chloropyridine hydrochloride. It is used in an amount of from catalytic to excess, preferably from 1/10 to twice the weight of the reaction solvent. The reaction time is from one minute to 48 hours, preferably from 5 minutes to 1 hour.

Compound 38 can be prepared from Compound 37.

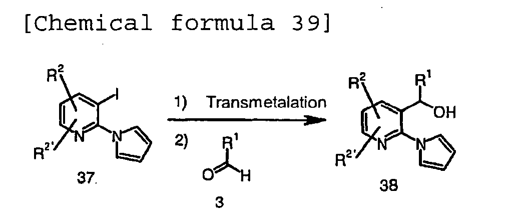

Synthesis of Compound 38 can be achieved by, after transmetalation of Compound 37 with a Grignard reagent such as isopropylmagnesium bromide or a lithium reagent such as n-butyl lithium, reacting the resulting compound with an aldehyde. More specifically, Compound 37 is reacted with from 1 mole to excess moles, preferably from 1 mole to 1.5 moles, per mole of Compound 37, of a metal reagent at from -100°C to 50°C, preferably from -85°C to 10°C in a solvent not adversely affecting the reaction such as toluene, tetrahydrofuran, dioxane, diethyl ether, or hexane, or a mixed solvent thereof. The reaction time is from 1 minute to 12 hours, preferably from 10 minutes to 5 hours. The reaction mixture is then reacted with from 1 mole to excess moles, preferably from 1 mole to 5 moles of an aldehyde such as 2,3-dimethoxybenzaldehyde at a reaction temperature of from -100°C to 100°C, preferably from -78°C to 50°C. The reaction time is from 1 minute to 12 hours, preferably from 10 minutes to 5 hours. After the hydroxy group of Compound 38 thus obtained is oxidized using a process based on the process employed in the conversion of Compound 4 to Compound 5, the processes employed for the synthesis of Compound 21, Compound 22, Compound 24, Compound 28, and Compound 32 are employed, whereby compounds corresponding to them can be synthesized, respectively.

Of Compounds (I), Compound 40 can be prepared, for example, in accordance with the following reaction scheme.

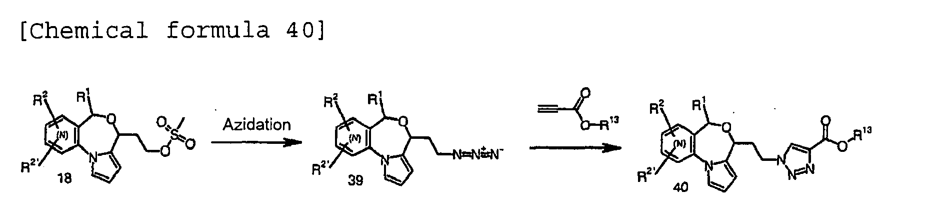

[wherein, R¹, R², and R^{2'} have the same meanings as described above and R¹³ means a substituted or unsubstituted C₁₋₆ alkyl group, a C₂₋₆ alkenyl group, or a substituted or unsubstituted C₆₋₁₉ aralkyl group].

Compound 39 can be prepared from Compound 18.

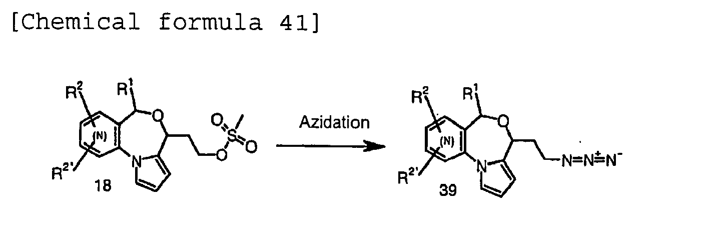

Synthesis of Compound 39 from Compound 18 can be achieved treating Compound 18 with an azide. For example, Compound 18 is treated with an azide such as sodium azide or lithium azide in a solvent not adversely affecting the reaction such as dioxane, tetrahydrofuran, dichloroethane, chloroform, dichloromethane, toluene, benzene, dimethylformamide, dimethylsulfoxide, acetonitrile, or water, or a mixed solvent thereof at from 0°C to the boiling point of the solvent used for the reaction, preferably from 10°C to 150°C. The azide is used in an amount of from 1 mole to excess moles, preferably from 1 mole to 5 moles per mole of Compound 18. The reaction time is from 1 minute to 240 hours, preferably from 10 minutes to 60 hours.

Compound 40 can be prepared from Compound 39.

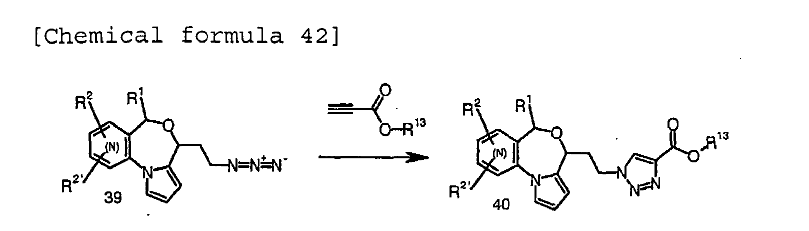

Synthesis of Compound 40 from Compound 39 can be achieved reacting Compound 39 with a propionate compound. For example, Compound 40 is synthesized by treating a propiolate with Compound 39 in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from 0°C to the boiling point of the solvent used for the reaction, preferably from 10°C to 150°C. The propiolate is used in an amount of from 1 mole to excess moles, preferably from 1 mole to 5 moles per mole of Compound 39. The reaction time is from 1 minute to 240 hours, preferably from 10 minutes to 60 hours.

Compound 40 thus obtained can be hydrolyzed into the corresponding carboxylic acid compound by using a process based on the process employed for the conversion of Compound 20 to Compound 21.

Of Compounds (I), Compound 44 can be prepared, for example, in accordance with the following reaction scheme.

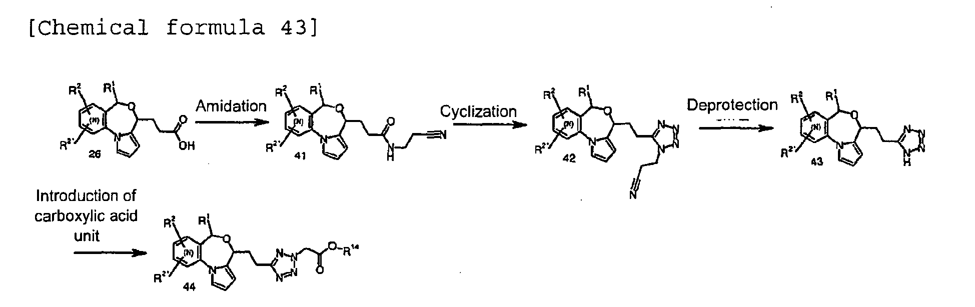

[wherein, R¹, R², and R² have the same meanings as described above and R¹⁴ means a substituted or unsubstituted C₁₋₆ alkyl group, a C₂₋₆ alkenyl group, or a substituted or unsubstituted C₆₋₁₉ aralkyl group] .

Compound 41 can be prepared from Compound 26.

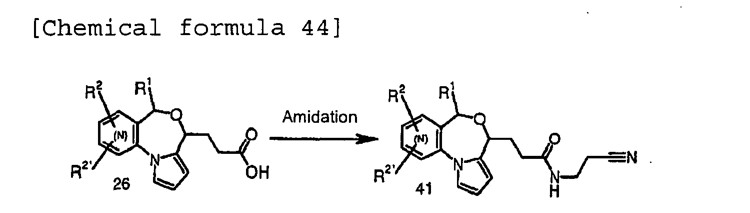

Synthesis of Compound 41 from Compound 26 can be achieved reacting Compound 26 with cyanoethylamine or salt thereof. For example, Compound 26 is reacted with from 1 mole to excess moles, preferably from 1 mole to 5 moles, per mole of Compound 26, of cyanoethylamine or salt thereof in the presence of a condensation agent such as N,N-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate, or 2-(1H-benzotriazol-1-yl)-1,1,3,3-tetramethyluronium tetrafluoroborate in an inert solvent at from -30°C to the boiling point of the solvent used for the reaction, preferably from 0°C to 50°C. The condensation agent is used in an amount of from 1 mole to excess moles, preferably from 1 mole to 5 moles, per mole of Compound 26. Examples of the inert solvent include dichloromethane, N,N-dimethylformamide, tetrahydrofuran, dioxane, ethyl acetate, and acetonitrile, and mixtures thereof. The reaction may be performed in the presence of a base such as triethylamine, diisopropylethylamine, N-methylmorpholine, 4-dimethylaminopyridine, or 1,8-diazabicyclo[5,4,0]undec-7-ene, if necessary. Moreover, an N-hydroxy compound such as 1-hydroxybenzotriazole, N-hydroxysuccinimide, or N-hydroxyphthalimide or a phenol compound such as 4-nitrophenol, 2,4-dinitrophenol, 2,4,5-trichlorophenol, or pentachlorophenol may be added as a reaction accelerator. The reaction time is from 10 minutes to 48 hours, preferably from 30 minutes to 12 hours.

Compound 42 can be prepared from Compound 41.

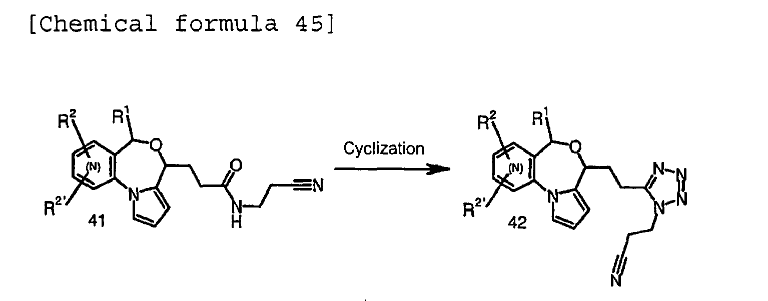

Synthesis of Compound 42 from Compound 41 is achieved by the tetrazolylation of Compound 41. For example, Compound 42 is synthesized by treating Compound 41 with trimethylsilylazide, triphenylphosphine, and diethylazodicarboxylate in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from 10°C to 100°C. These reagents are used in an amount of from 1 mole to excess moles, preferably from 1 mole to 5 moles per mole of Compound 41. The reaction time is from 1 minute to 240 hours, preferably from 10 minutes to 60 hours.

Compound 43 can be prepared from Compound 42.

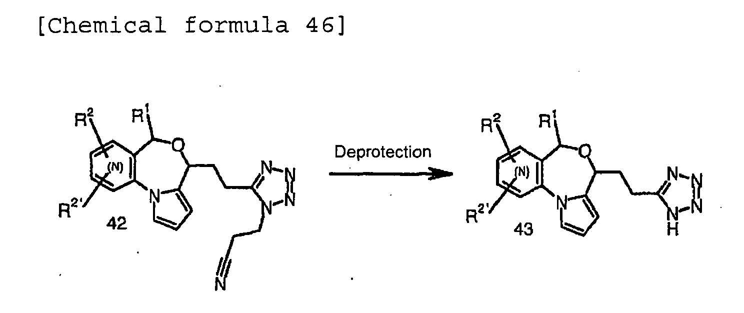

Compound 43 can be prepared by eliminating the protecting group from Compound 42. Compound 43 is synthesized by treating Compound 42 with a base such as 1,8-diazabicyclo[5,4,0]undec-7-ene in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from 10°C to 100°C. The base is used in an amount of from catalytic to excess, preferably from 0.5 to 10 moles per mole of Compound 42. The reaction time is from 1 minute to 240 hours, preferably from 10 minutes to 60 hours.

Compound 44 can be prepared from Compound 43.

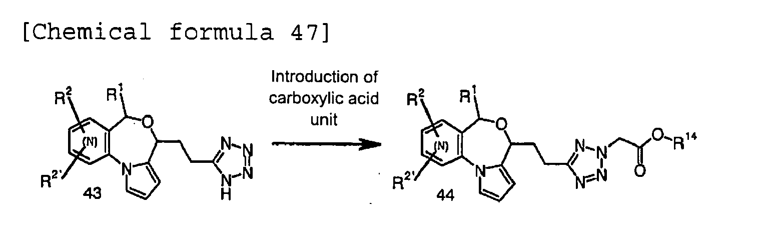

Synthesis of Compound 44 from Compound 43 is achieved reacting Compound 43 with a halogenoacetate or the like. For example, Compound 43 is reacted with a bromoacetate or the like in the presence of a base in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from -20°C to 100°C. The bromoacetate or the like is used in an amount of from 1 mole to excess moles, preferably from 1 mole to 5 moles per mole of Compound 43. As the base, a carbonate such as sodium carbonate or potassium carbonate is usable. The reaction time is from 1 minute to 240 hours, preferably from 10 minutes to 60 hours.

By the deprotection of the ester of Compound 44 by a process based on the process employed for the synthesis of Compound 21 from Compound 20, the corresponding carboxylic acid compound can be synthesized.

Of Compounds (I), Compound 46 can be prepared, for example, in accordance with the following reaction scheme.

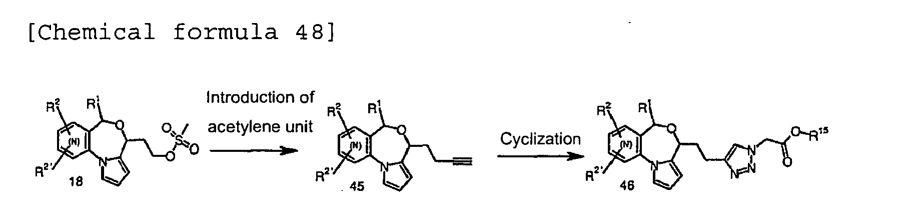

[wherein, R¹, R², and R^{2'} have the same meanings as described above and R¹⁵ means a substituted or unsubstituted C₁₋₆ alkyl group, a C₂₋₆ alkenyl group, or a substituted or unsubstituted C₆₋₁₉ aralkyl group].

Compound 45 can be prepared from Compound 18.

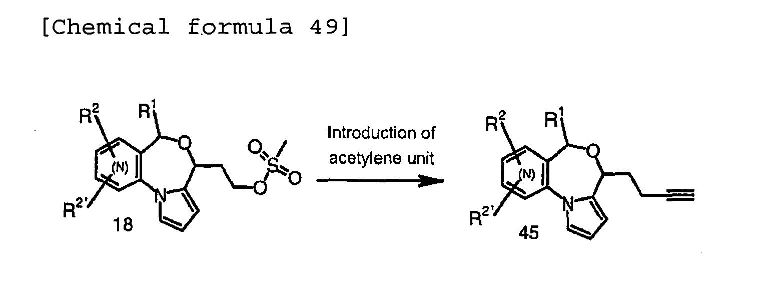

Synthesis of Compound 45 from Compound 18 is achieved reacting Compound 18 with an acetylide. For example, Compound 18 is treated with an acetylide such as lithium acetylide ethylenediamine complex in the presence of a phase transfer catalyst such as tetrabutylammonium iodide in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from 0°C to the boiling point of the solvent used for the reaction, preferably from 10°C to 150°C. The acetylide is used in an amount of from 1 mole to excess moles, preferably from 1 mole to 5 moles per mole of Compound 18. The phase transfer catalyst is used in an amount of from catalytic to excess, preferably from 0.1 mole to 5 moles per mole of Compound 18. The reaction time is from 1 minute to 240 hours, preferably from 10 minutes to 60 hours.

Compound 46 can be prepared from Compound 45.

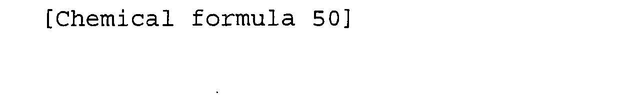

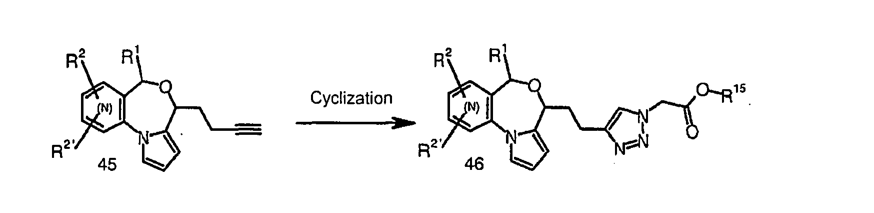

Synthesis of Compound 46 from Compound 45 is achieved by the triazolylation of Compound 45. For example, Compound 46 is synthesized by treating Compound 45 with azidoacetate in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from 10°C to 150°C. The azidoacetate is used in an amount of from 1 mole to excess moles, preferably from 1 mole to 5 moles, per mole of Compound 45. The reaction time is from 1 minute to 240 hours, preferably from 10 minutes to 60 hours.

The resulting Compound 46 can be hydrolyzed into the corresponding carboxylic acid compound by using a process based on the process employed for the conversion of Compound 20 to Compound 21.

Of Compounds (I), the compound represented by the formula (I-3) can be prepared, for example, in accordance with the following reaction scheme.

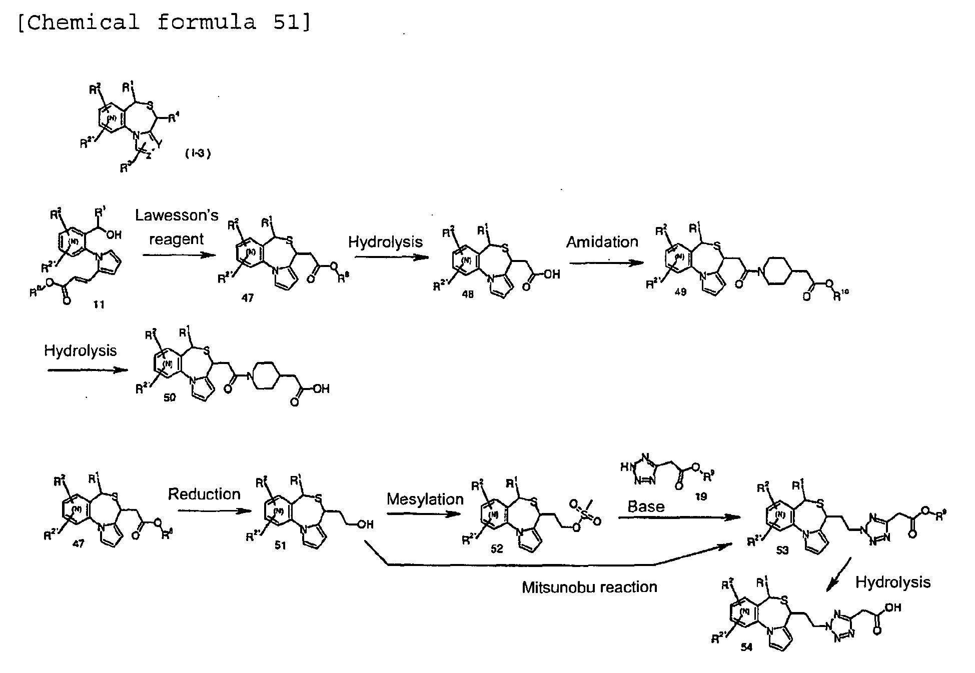

[wherein, R¹, R², and R^{2'} have the same meanings as described above, and R⁸, R⁹, and R¹⁰ each independently means a substituted or unsubstituted C₁₋₆ alkyl group, a C₂₋₆ alkenyl group, or a substituted or unsubstituted C₆₋₁₉ aralkyl group].

Compound 47 can be prepared from Compound 11.

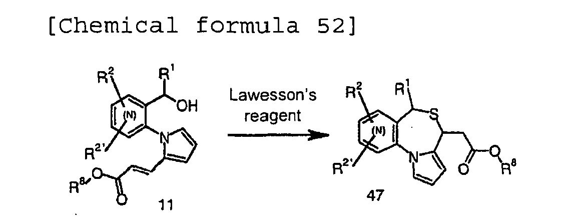

Synthesis of Compound 47 from Compound 11 can be achieved treating Compound 11 with Lawesson's reagent, diphosphorus pentasulfide, or the like. Compound 11 is treated in a solvent not adversely affecting the reaction such as dioxane, tetrahydrofuran, dichloroethane, chloroform, dichloromethane, xylene, toluene, benzene, or ethyl acetate, or a mixed solvent thereof at from -78°C to the boiling point of the solvent used for the reaction, preferably from 0°C to 150°C. The Lawesson's reagent, diphosphorus pentasulfide, or the like is used in an amount of from 0.5 mole to excess moles, preferably from 1 mole to 5 moles per mole of Compound 11. The reaction time is from 1 minute to 100 hours, preferably from 30 minutes to 50 hours.

In accordance with the processes employed for the synthesis of Compound 21, Compound 22, Compound 24, Compound 28, and Compound 32 while using Compound 47, the corresponding compounds can be synthesized, respectively.

Of Compounds (I), compounds represented by the formula (1-4) can be prepared, for example, in accordance with the following reaction scheme.

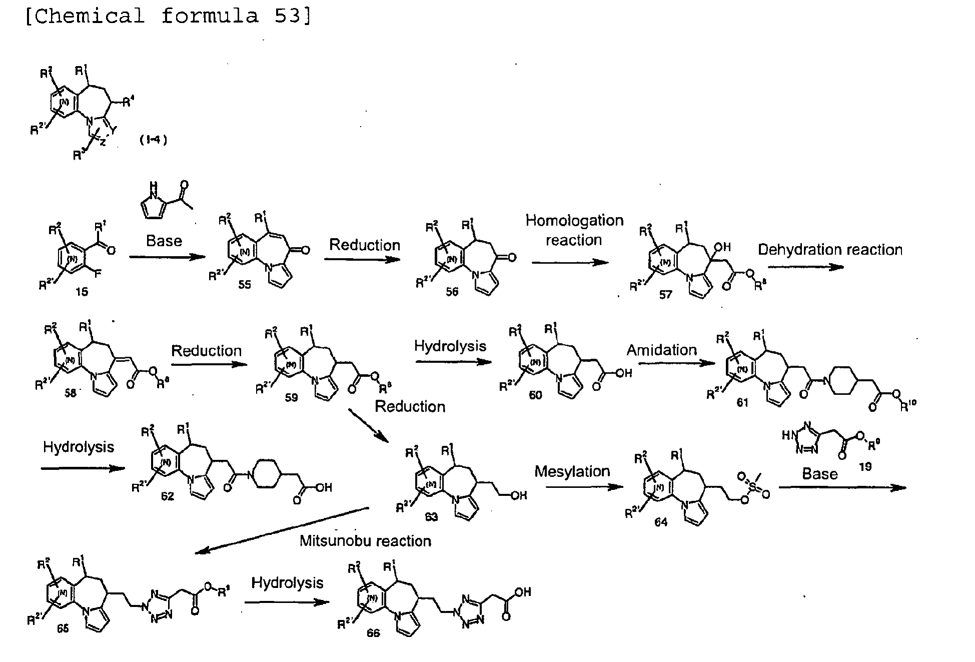

[wherein, R¹, R², and R^{2'} have the same meanings as described above and R⁸, R⁹, and R¹⁰ each independently represents a substituted or unsubstituted C₁₋₆ alkyl group, a C₂₋₆ alkenyl group, or a substituted or unsubstituted C₆₋₁₉ aralkyl group].

Compound 55 can be prepared from Compound 15.

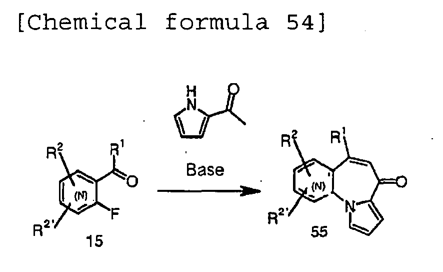

Synthesis of Compound 55 from Compound 15 is achieved reacting Compound 15 with acetylpyrrole in the presence of a base. For example, Compound 15 is treated with 2-acetylpyrrole serving as a nucleophilic agent in a solvent not adversely affecting the reaction such as dioxane, tetrahydrofuran, diethyl ether, dimethylformamide, dimethylsulfoxide, toluene, or benzene, or a mixed solvent thereof at from -10°C to the boiling point of the solvent used for the reaction, preferably from 0°C to 150°C. A carbonate such as sodium hydrogen carbonate or potassium carbonate, a hydroxide such as sodium hydroxide, a metal hydride such as sodium hydride, an alkoxide such as tertiary butoxy potassium, or an amine such as triethylamine, diisopropylethylamine, N-methylmorpholine, 4-dimethylaminopyridine, 1,8-diazabicyclo[5,4,0]undec-7-ene may be added as the base as needed. It is used in an amount of from 1 mole to excess moles, preferably from 1 mole to 5 moles, per mole of Compound 15. The reaction time is from 5 minutes to 200 hours, preferably from 30 minutes to 20 hours.

Compound 56 can be prepared from Compound 55.

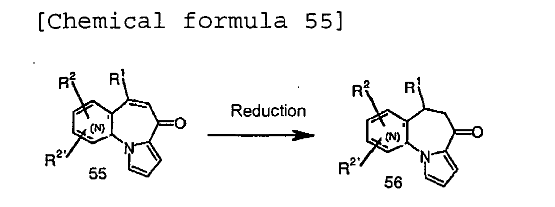

Synthesis of Compound 56 from Compound 55 is achieved by reducing Compound 55. For example, Compound 55 is subjected to a reduction reaction in a hydrogen atmosphere in the presence of, as a catalyst, rhodium-alumina, palladium-carbon, platinum oxide, ruthenium-carbon, palladium hydroxide, Raney nickel, or the like, preferably rhodium-alumina in a solvent not adversely affecting the reaction such as dioxane, tetrahydrofuran, diethyl ether, dimethylformamide, dimethylsulfoxide, toluene, benzene, ethyl acetate, ethanol, or methanol, or a mixed solvent thereof at from -10°C to the boiling point of the solvent used for the reaction, preferably from 0°C to 150°C. The catalyst is used in a catalytic amount or 2 moles per mole of Compound 55. The reaction time is from 5 minutes to 20 hours, preferably from 30 minutes to 5 hours.

Compound 57 can be prepared from Compound 56.

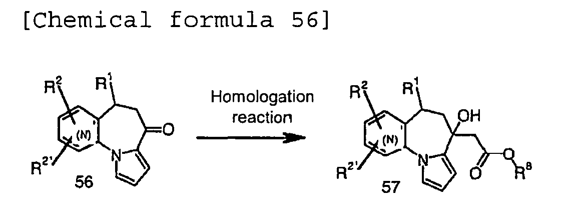

Synthesis of Compound 57 from Compound 56 is achieved subjecting Compound 56 to a homologation reaction. For example, Compound 56 is reacted with cerium enolate, which has been prepared from lithium enolate of an acetate and anhydrous cerium chloride, in a solvent not adversely affecting the reaction such as dioxane, tetrahydrofuran, diethyl ether, toluene, or benzene, or a mixed solvent thereof at from -100°C to the boiling point of the solvent used for the reaction, preferably from -78°C to 25°C. Cerium enolate is used in an amount of from 1 mole to excess moles, preferably from 1.5 moles to 5 moles per mole of Compound 56. The reaction time is from 5 minutes to 200 hours, preferably from 30 minutes to 20 hours.

Compound 58 can be prepared from Compound 57.

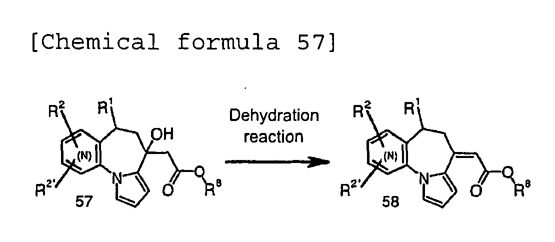

Synthesis of Compound 58 from Compound 57 is achieved subjecting Compound 57 to a dehydration reaction. For example, Compound 57 is treated with a methanesulfonylation agent in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 50°C. Examples of the methanesulfonylation agent include methanesulfonyl chloride and methanesulfonic anhydride. It is used in an amount of from 1 mole to excess moles, preferably from 1 mole to 5 moles per mole of Compound 57. Further, a base such as triethylamine, diisopropylethylamine, N-methylmorpholine, 4-dimethylaminopyridine, or 1,8-diazabicyclo[5,4,0]undec-7-ene may be added to Compound 57 in an amount of from 1 mole to excess moles, preferably from 1 mole to 10 moles. The reaction time is from 1 minute to 60 hours, preferably from 10 minutes to 5 hours.

Compound 59 can be prepared from Compound 58.

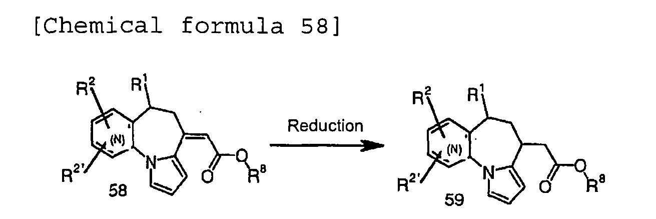

Synthesis of Compound 59 from Compound 58 is achieved reducing Compound 58. For example, Compound 58 is subjected to a reduction reaction in a hydrogen atmosphere in the presence of, as a catalyst, rhodium-alumina, palladium-carbon, platinum oxide, ruthenium-carbon, palladium hydroxide, or Raney nickel, preferably palladium-carbon, in a solvent not adversely affecting the reaction such as dioxane, tetrahydrofuran, diethyl ether, dimethylformamide, dimethylsulfoxide, toluene, benzene, ethyl acetate, ethanol, or methanol, or a mixed solvent thereof at from -10°C to the boiling point of the solvent used for the reaction, preferably from 0°C to 150°C. The catalyst is used in an amount from catalytic or 2 moles per mole of Compound 58. The reaction time is from 5 minutes to 20 hours, preferably from 30 minutes to 5 hours.

In accordance with the processes employed for the synthesis of Compound 21, Compound 22, Compound 24, Compound 28, and Compound 32 while using Compound 59, corresponding compounds can be synthesized, respectively.

Of Compounds (I), compounds represented by the formula (I-5) can be prepared, for example, in accordance with the following reaction scheme.

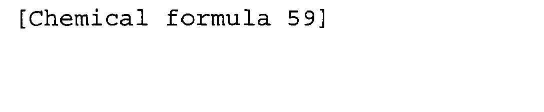

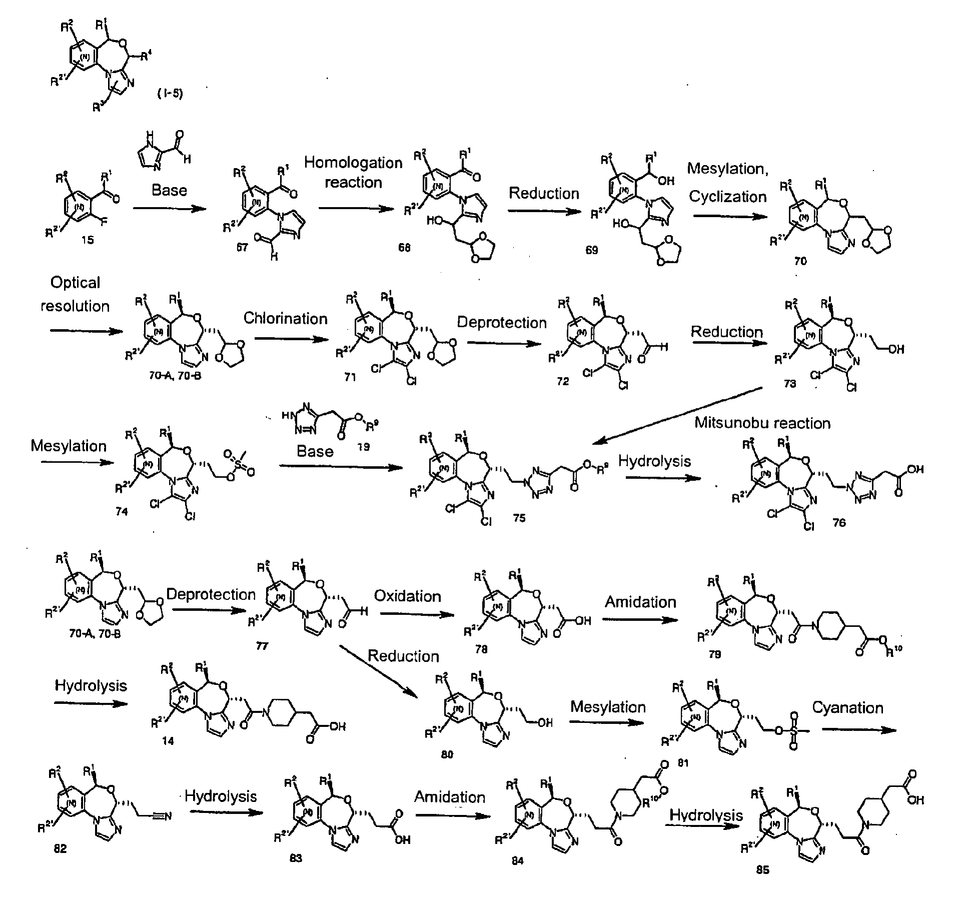

[wherein, R¹, R², and R^{2'} have the same meanings as described above and R⁹ and R¹⁰ each independently represents a substituted or unsubstituted C₁₋₆ alkyl group, a C₂₋₆ alkenyl group, or a substituted or unsubstituted C₆₋₁₉ aralkyl group]

Compound 67 can be prepared from Compound 15.

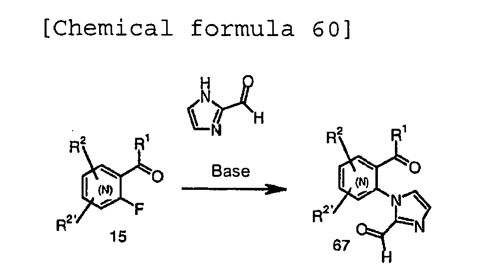

Synthesis of Compound 67 from Compound 15 is achieved reacting Compound 15 with 2-formylimidazole in the presence of a base. For example, Compound 15 is treated with from 1 mole to excess moles, preferably from 1 mole to 5 moles of 2-formylimidazole serving as a nucleophilic agent in a solvent not adversely affecting the reaction of Compound 15 such as dioxane, tetrahydrofuran, diethyl ether, dimethylformamide, dimethylsulfoxide, toluene, or benzene, or a mixed solvent thereof at from -10°C to the boiling point of the solvent used for the reaction, preferably from 0°C to 150°C. A base, for example, a carbonate such as sodium hydrogen carbonate or potassium carbonate, a hydroxide such as sodium hydroxide, a metal hydride such as sodium hydride, an alkoxide such as tertiary butoxy potassium, or an amine such as triethylamine, diisopropylethylamine, N-methylmorpholine, 4-dimethylaminopyridine, or 1,8-diazabicyclo[5,4,0]undec-7-ene may be added as needed. It is used in an amount of from 1 mole to excess moles, preferably from 1 mole to 5 moles per mole of Compound 15. The reaction time is from 5 minutes to 200 hours, preferably from 30 minutes to 20 hours.

Synthesis of Compound 67 from Compound 15 can also be achieved introducing imidazole into Compound 15 by a nucleophilic substitution reaction, introducing a hydroxymethyl group into the 2-imidazole ring, and oxidizing the resulting hydroxy group; or introducing a formyl group into the 2-imidazole ring.

Compound 68 can be prepared from Compound 67.

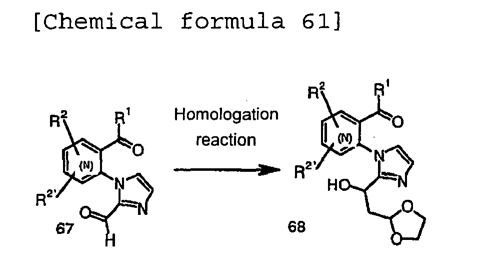

Synthesis of Compound 68 from Compound 67 is achieved subjecting Compound 67 to a homologation reaction. For example, Compound 67 is treated with a nucleophilic agent unit capable of accomplishing two-carbon homologation, for example, a Grignard reagent such as (1,3-dioxolan-2-ylmethyl)magnesium bromide in a solvent not adversely affecting the reaction such as dioxane, tetrahydrofuran, diethyl ether, xylene, or toluene, or a mixed solvent thereof at from -78°C to the boiling point of the solvent used for the reaction, preferably from 0°C to 200°C. The nucleophilic agent unit is used in an amount of from 1 mole to excess moles, preferably from 1 mole to 5 moles per mole of Compound 67. The reaction time is from 5 minutes to 240 hours, preferably from 30 minutes to 10 hours.

Compound 69 can be prepared from Compound 68.

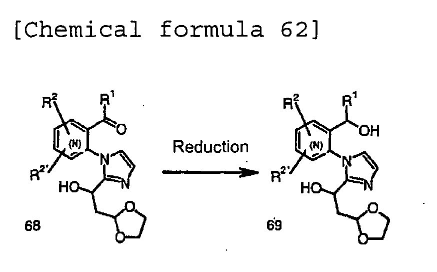

Synthesis of Compound 69 from Compound 68 can be achieved treating Compound 68 with a reducing agent. For example, Compound 68 is treated with a reducing agent in methanol, ethanol, tetrahydrofuran, diethyl ether, dioxane, dichloromethane, toluene, or the like, or a mixed solvent thereof at from -78°C to the boiling point of the solvent used for the reaction, preferably from -20°C to 100°C. Examples of the reducing agent include sodium borohydride, lithium borohydride, lithium aluminum hydride, diisobutylaluminum hydride, borane dimethylsulfide complex, and borane tetrahydrofuran complex. It is used in an amount of from 1 mole to excess moles, preferably from 1 mole to 5 moles per mole of Compound 68. The reaction time is from 5 minutes to 60 hours, preferably from 10 minutes to 24 hours.

Compound 70 can be prepared from Compound 69.

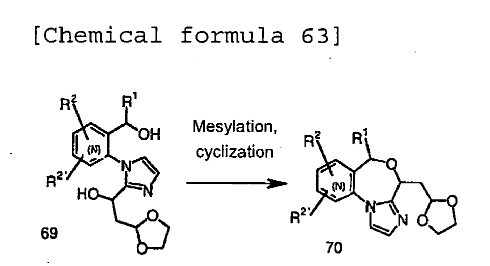

Synthesis of Compound 70 from Compound 69 is achieved by the methanesulfonylation of Compound 69, followed by cyclization. For example, Compound 69 is treated with a methanesulfonylation agent in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Examples of the methanesulfonylation agent include methanesulfonyl chloride and methanesulfonic anhydride. It is used in an amount of from 1 mole to excess moles, preferably from 1 mole to 5 moles per mole of Compound 69. Further, a base such as triethylamine, diisopropylethylamine, N-methylmorpholine, 4-dimethylaminopyridine, or 1,8-diazabicyclo[5,4,0]undec-7-ene is used in an amount of from 1 mole to excess moles, preferably from 1 mole to 10 moles per mole of Compound 69. The reaction time is from 1 minute to 200 hours, preferably from 10 minutes to 18 hours.

Compound 70-A and Compound 70-B which are optically active Compounds 70 are available by optical resolution of Compound 70.

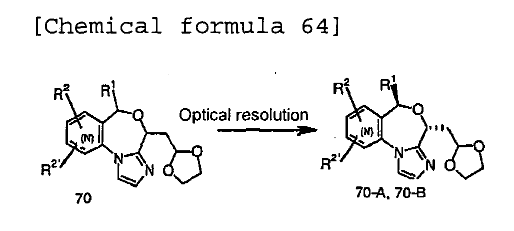

Compound 70-A and Compound 70-B can be obtained by optical resolution of Compound 70 by using an optically active column or the like. As the optically active column, CHIRALCEL OD (product of Daicel Chemical Industries, diameter: 50 mm, length: 500 mm, flow rate: 50 mL/min, solvent: hexane:isopropanol=13:7) is usable.

Compound 77 can be prepared from Compound 70-A and Compound 70-B.

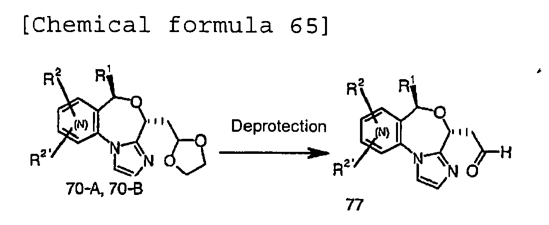

Synthesis of Compound 77 from Compound 70-A and Compound 70-B is achieved eliminating a protecting group from Compound 70-A or Compound 70-B. For example, Compound 70-A or Compound 70-B is treated with an acid such as perchloric acid in a solvent not adversely affecting the reaction such as dioxane, tetrahydrofuran, dichloroethane, chloroform, dichloromethane, carbon tetrachloride, toluene, benzene, ethyl acetate, or dimethylformamide, or a mixed solvent thereof at from -78°C to the boiling point of the solvent used for the reaction, preferably from 0°C to 120°C. The acid is used in an amount of excess moles per mole of Compound 70-A or Compound 70-B. Use of a 30% aqueous perchloric acid solution in an amount of from one fifth to five times the amount of the reaction solvent is preferred. The reaction time is from 1 minute to 60 hours, preferably from 10 minutes to 5 hours.

Compound 80 can be prepared from Compound 77.

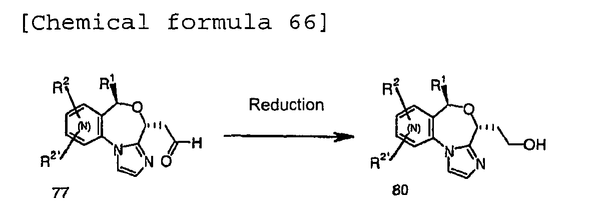

Synthesis of Compound 80 from Compound 77 can be achieved treating Compound 77 with a reducing agent. For example, Compound 77 is treated with a reducing agent in methanol, ethanol, tetrahydrofuran, diethyl ether, dioxane, dichloromethane, toluene, water, or the like, or a mixed solvent thereof at from -78°C to the boiling point of the solvent used for the reaction, preferably from -20°C to 100°C. Examples of the reducing agent include sodium borohydride, lithium borohydride, lithium aluminum hydride, diisobutylaluminum hydride, borane dimethylsulfide complex, and borane tetrahydrofuran complex. It is used in an amount of from 1 mole to excess moles, preferably from 1 mole to 5 moles per mol of Compound 77. The reaction time is from 5 minutes to 60 hours, preferably from 10 minutes to 24 hours.

In a similar manner to those employed for the synthesis of Compound 21, Compound 27, and Compound 32, while using Compound 80, the corresponding compounds can be synthesized, respectively.

Compound 71 can be prepared from Compound 70-A or Compound 70-B.

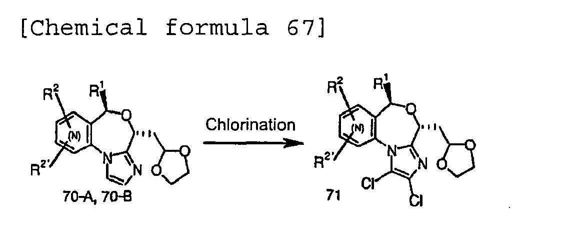

Synthesis of Compound 71 from Compound 70-A or Compound 70-B can be achieved treating Compound 70-A or Compound 70-B with a chlorination agent. For example, Compound 70-A or Compound 70-B is treated with a chlorination agent such as N-chlorosuccinimide in methanol, ethanol, tetrahydrofuran, diethyl ether, dioxane, dichloroethane, carbon tetrachloride, chloroform, dichloromethane, toluene, xylene, dimethylformamide, dimethylsulfoxide, or the like, or a mixed solvent thereof at from -78°C to the boiling point of the solvent used for the reaction, preferably from 0°C to 150°C. Examples of the chlorination agent include N-chlorosuccinimide. It is used in an amount of from 1 mole to excess moles, preferably from 1 mole to 5 moles per mole of Compound 70-A or Compound 70-B. The reaction time is from 5 minutes to 60 hours, preferably from 10 minutes to 24 hours.

In accordance with a process based on the process employed for the preparation of Compound 80 from Compound 70-A or Compound 70-B while using Compound 71, Compound 73 can be synthesized. In similar manners to those employed for the synthesis of Compound 21, Compound 27, and Compound 32 while using Compound 73, the corresponding compounds can be synthesized, respectively.

Of Compounds (I), compounds represented by the formula (I-6) can be prepared, for example, in accordance with the following reaction scheme.

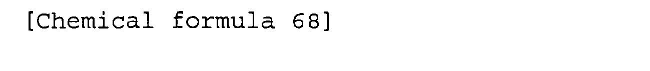

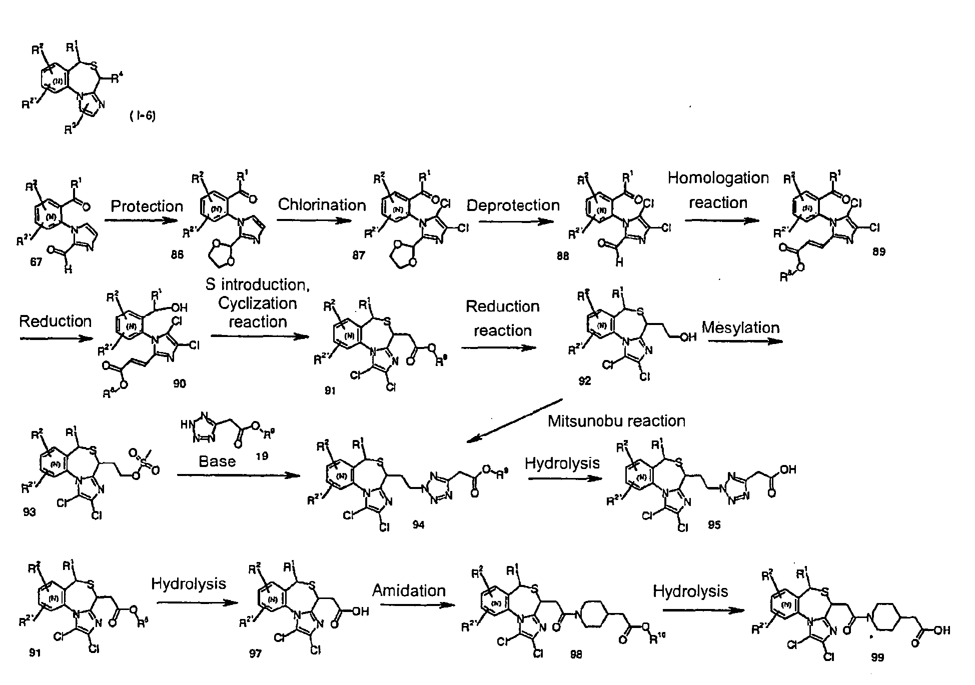

[wherein, R¹, R², and R^{2'} have the same meanings as described above and R⁸, R⁹, and R¹⁰ each independently represents a substituted or unsubstituted C₁₋₆ alkyl group, a C₂₋₆ alkenyl group, or a substituted or unsubstituted C₆₋₁₉ aralkyl group].

Compound 86 can be prepared from Compound 67.

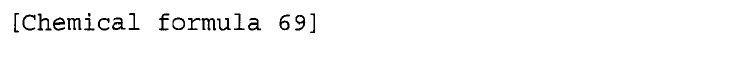

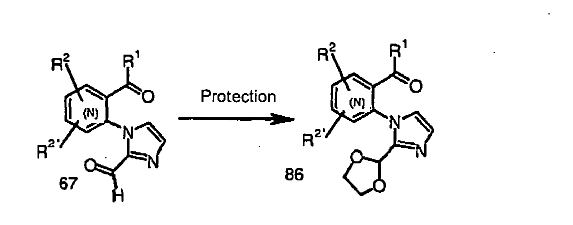

Synthesis of Compound 86 from Compound 67 is achieved by protecting the formyl group of Compound 67. For example, Compound 67 is treated with ethylene glycol or bistrimethylsilylated product in the presence of an acid such as toluenesulfonic acid, camphorsulfonic acid, sulfuric acid, hydrochloric acid, or Lewis acid in a solvent not adversely affecting the reaction such as dioxane, tetrahydrofuran, xylene, toluene, benzene, or dichloroethane, or a mixed solvent thereof at from 0°C to the boiling point of the solvent used for the reaction, preferably from 0°C to 200°C. The acid is used in an amount of from a catalytic amount to excess moles, preferably from 0.01 to 2 moles per mole of Compound 67. Ethylene glycol or bistrimethylsilylated product thereof is used in an amount of from a catalytic amount to excess moles, preferably from 0.01 to 2 moles per mole of Compound 67. The reaction time is from 5 minutes to 240 hours, preferably from 5 hours to 20 hours.

Compound 87 can be prepared from Compound 86.

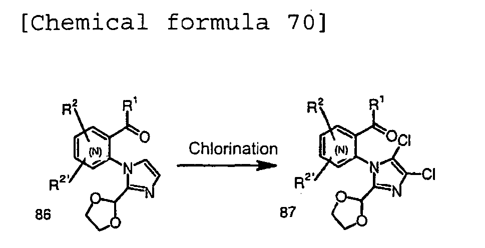

Synthesis of Compound 87 from Compound 86 is achieved by chlorinating Compound 86. For example, Compound 86 is treated with a chlorination agent in a solvent not adversely affecting the reaction such as dioxane, tetrahydrofuran, dichloroethane, chloroform, dichloromethane, carbon tetrachloride, toluene, benzene, or ethyl acetate, or a mixed solvent thereof at from -78°C to the boiling point of the solvent used for the reaction, preferably from 0°C to 120°C. Examples of the chlorination agent include N-chlorosuccinimide. It is used in an amount of from 2 moles to excess moles, preferably from 2 moles to 10 moles per mole of Compound 86. The reaction time is from 1 minute to 60 hours, preferably from 10 minutes to 5 hours.

Compound 88 can be prepared from Compound 87.

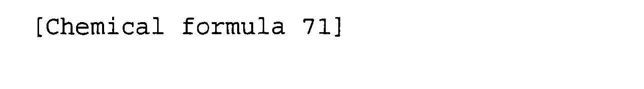

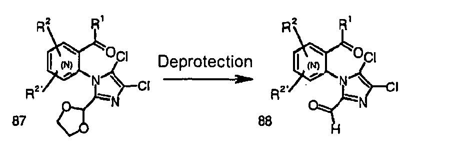

Synthesis of Compound 88 from Compound 87 is achieved by eliminating the protecting group from Compound 87. For example, Compound 87 is reacted with an acid such as perchloric acid, hydrochloric acid, sulfuric acid, acetic acid, or Lewis acid in a solvent not adversely affecting the reaction such as acetone, dioxane, tetrahydrofuran, ether, dichloromethane, isopropanol, ethanol, methanol, acetonitrile, or water, or a mixed solvent thereof at from -78°C to the boiling point of the solvent used for the reaction, preferably from 0°C to 150°C. The acid is used in an amount of from 0.01 mole to excess moles, preferably from 1 mole to 1000 moles per mole of Compound 87. The reaction time is from 1 minute to 24 hours, preferably from 5 minutes to 5 hours.

Compound 89 can be synthesized from Compound 88.

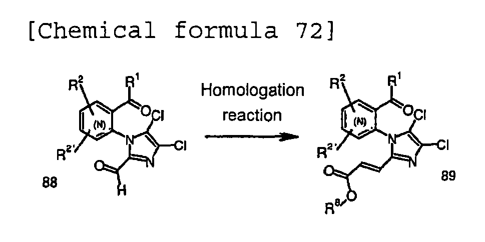

Synthesis of Compound 89 from Compound 88 is achieved by subjecting Compound 88 to a homologation reaction. For example, Compound 88 is treated with triphenylphosphoranylidene acetate, trialkylphosphonoacetate, or the like in a solvent not adversely affecting the reaction such as dioxane, tetrahydrofuran, dichloroethane, chloroform, dichloromethane, xylene, toluene, benzene, ethyl acetate, dimethylsulfoxide, dimethylformamide, or acetonitrile, or a mixed solvent thereof at from -78°C to the boiling point of the solvent used for the reaction, preferably from 0°C to 150°C. Triphenylphosphoranylidene acetate, trialkylphosphonoacetate, or the like is used in an amount of from 1 mole to excess moles, preferably from 1 mole to 5 moles per mole of Compound 88. When a trialkylphosphonoacetate is used, an amine such as triethylamine, diisopropylethylamine, N-methylmorpholine, 4-dimethylaminopyridine, or 1,8-diazabicyclo[5,4,0]undec-7-ene, an alkoxide such as tertiary butoxy potassium, a metal hydride such as sodium hydride, or a metal amide such as lithium hexamethyldisilazide is used in an amount of from 1 mole to excess moles, preferably from 1 mole to 10 moles. The reaction time is from 1 minute to 60 hours, preferably from 10 minutes to 10 hours.

Compound 90 can be prepared from Compound 89.

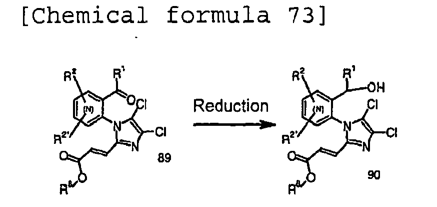

Synthesis of Compound 90 from Compound 89 is achieved by treating Compound 89 with a reducing agent. For example, Compound 89 is treated with from 0.5 to excess moles, preferably from 1 mole to 5 moles, per mole of Compound 89, of a reducing agent such as sodium borohydride, lithium borohydride, or lithium aluminum hydride at from -78°C to 100°C, preferably from -50°C to 50°C in a solvent not adversely affecting the reaction such as toluene, dichloroethane, dichloromethane, dioxane, tetrahydrofuran, isopropanol, ethanol, methanol, dimethylformamide, or acetonitrile, or a mixed solvent thereof. The reaction time is from 1 minute to 10 hours, preferably from 3 minutes to 2 hours.

Compound 91 can be prepared from Compound 90.

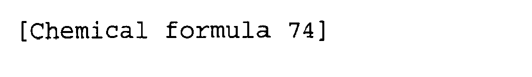

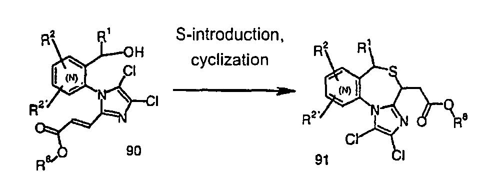

Synthesis of Compound 91 from Compound 90 is achieved by cyclizing Compound 90 after a mercapto group has been introduced therein. For example, Compound 90 is treated with Lawesson's reagent, diphosphorus pentasulfide, or the like in a solvent not adversely affecting the reaction such as dioxane, tetrahydrofuran, dichloroethane, chloroform, dichloromethane, xylene, toluene, benzene, or ethyl acetate, or a mixed solvent thereof at from -78°C to the boiling point of the solvent used for the reaction, preferably from 0°C to 150°C. Lawesson's reagent, diphosphorus pentasulfide, or the like is used in an amount of from 0.5 to excess moles, preferably from 1 mole to 5 moles per mole of Compound 90. The reaction time is from 1 minute to 100 hours, preferably from 30 minutes to 50 hours. Synthesis of Compound 91 from Compound 90 can also be achieved by isolating an SH compound before ring closure, adding a base thereto as needed and then heating.

Compound 92 can be prepared from Compound 91.

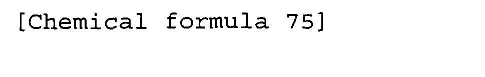

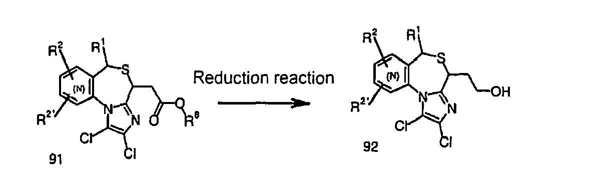

Synthesis of Compound 92 from Compound 91 is achieved by treating Compound 91 with a reducing agent. The treatment is performed with from 1 mole to excess moles, preferably from 1 mole to 10 moles, per mole of Compound 91, of a reducing agent such as sodium borohydride, lithium borohydride, lithium aluminum hydride, or diisobutylaluminum hydride in a solvent not adversely affecting the reaction such as toluene, dichloroethane, dichloromethane, dioxane, tetrahydrofuran, isopropanol, ethanol, methanol, water, dimethylformamide, or acetonitrile, or a mixed solvent thereof at from -78°C to 100°C, preferably from -50°C to 50°C. The reaction time is from 5 minutes to 60 hours, preferably from 30 minutes to 12 hours.

Compound 93 can be prepared from Compound 92.

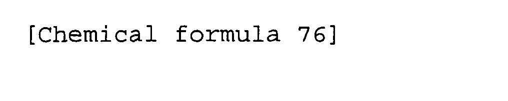

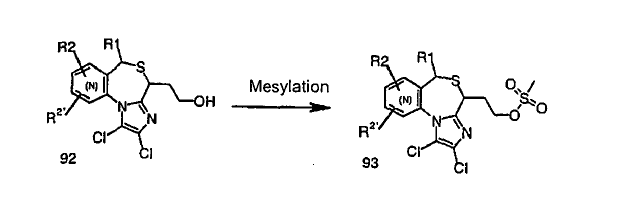

Synthesis of Compound 93 from Compound 92 is achieved by methanesulfonylation of Compound 92. For example, Compound 92 is treated with a methanesulfonylation agent in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 50°C. Examples of the methanesulfonylation agent include methanesulfonyl chloride and methanesulfonic anhydride. It is used in an amount of from 1 mole to excess moles, preferably from 1 mole to 5 moles per mole of Compound 92. Further, a base such as triethylamine, diisopropylethylamine, N-methylmorpholine, 4-dimethylaminopyridine, or 1,8-diazabicyclo[5,4,0]undec-7-ene is used in an amount of from 1 mole to excess moles, preferably from 1 mole to 10 moles per mole of Compound 92. The reaction time is from 1 minute to 60 hours, preferably from 10 minutes to 5 hours.

Compound 94 can be prepared from Compound 93.

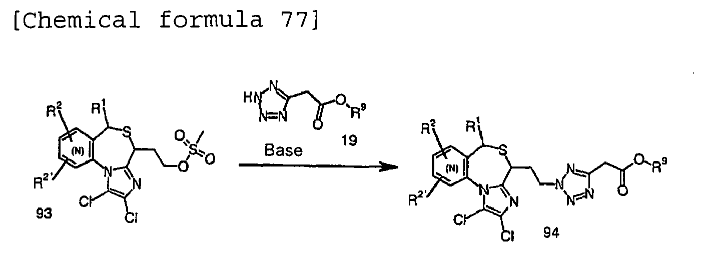

Synthesis of Compound 94 from Compound 93 is achieved by reacting Compound 93 with Compound 19 in the presence of a base. For example, Compound 93 is treated with Compund 19 in the presence of a base in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from -20°C to 100°C. One mole of Compound 93 is reacted with from 1 mole to excess moles, preferably from 1 mole to 5 moles of Compound 19 and the like in the presence of from 1 mole to excess moles, preferably from 1 mole to 10 moles of a base such as potassium carbonate, sodium carbonate, or tertiary butoxy potassium. It is possible to add an iodide such as tetrabutylammonium iodide or potassium iodide in an amount from catalytic to excess, preferably from catalytic to five times the equivalent.
The reaction time is from 1 minute to 240 hours, preferably from 10 minutes to 60 hours.

Compound 94 can be prepared from Compound 92.

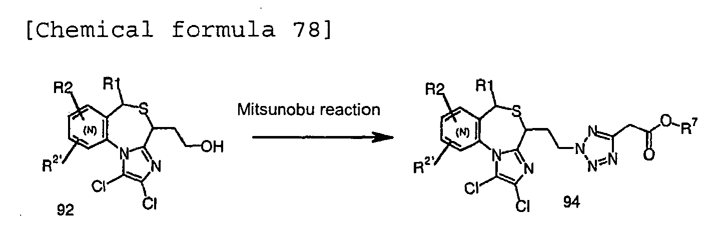

Compound 94 can also be synthesized from Compound 92 directly without preparing Compound 93. For example, the synthesis can be achieved in accordance with a Mitsunobu reaction by using a compound having an acidic proton. For example, the reaction can be achieved by treating 1 mole of Compound 92 and from 1 mole to excess moles, preferably from 1 mole to 3 moles of each of a nucleophilic agent such as tetrazole acetate, diethyl azodicarboxylate, and triphenylphosphine in an inert solvent such as dichloromethane, dichloroethane, tetrahydrofuran, acetonitrile, N,N-dimethylformamide, ethyl acetate, toluene, or the like at from -78°C to the boiling point of the solvent, preferably from -50°C to 60°C.

Compound 95 can be prepared from Compound 94.

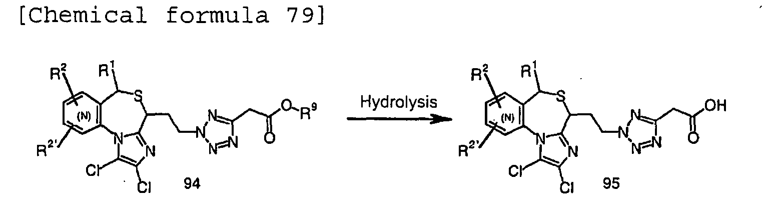

Synthesis of Compound 95 from Compound 94 is achieved by a conventional hydrolysis reaction, for example, hydrolysis reaction in which Compound 94 is treated with a metal hydroxide such as sodium hydroxide or lithium hydroxide or a carbonate such as sodium carbonate or potassium carbonate (from 1 mole to excess moles per mole of Compound 94) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

By treating Compound 91 in similar manners to those employed for the synthesis of Compound 24, Compound 27, and Compound 32, the corresponding derivatives can be prepared, respectively.

Of Compounds (I), compounds represented by the formula (I-7) can be prepared, for example, in accordance with the following reaction scheme.

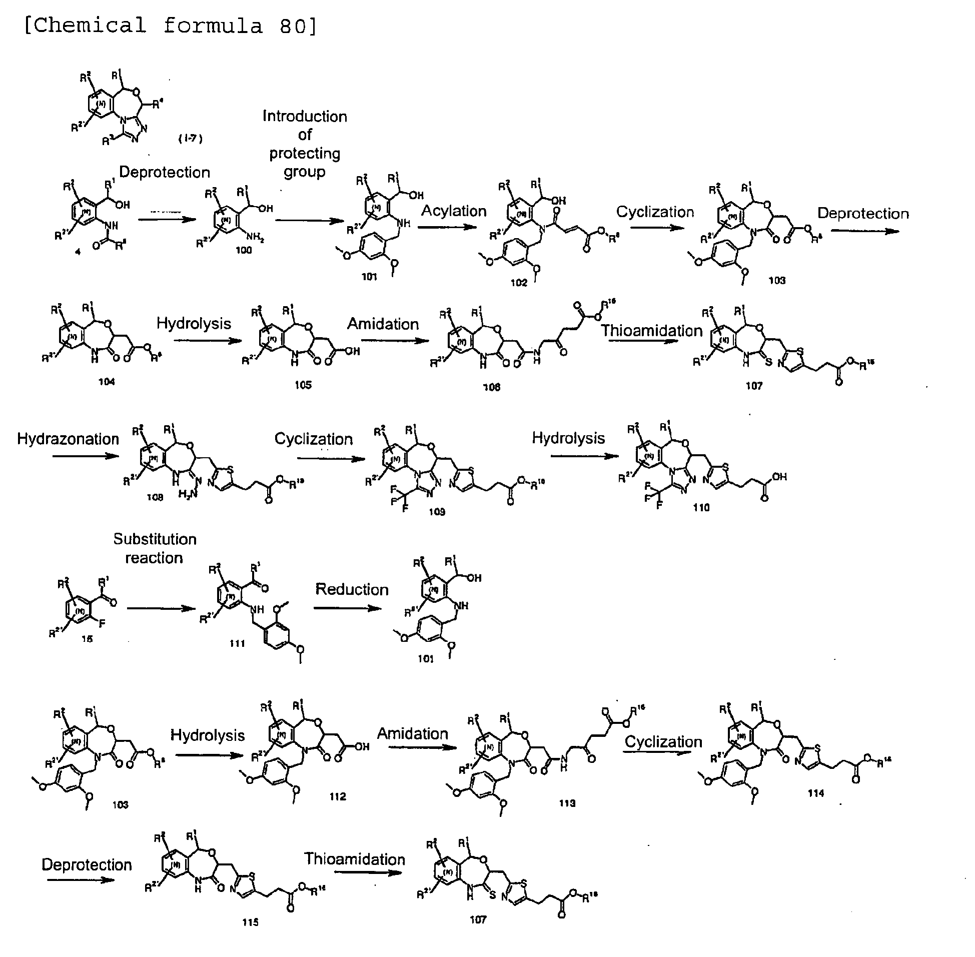

[wherein, R¹, R², and R^{2'} have the same meanings as described above and R⁸ and R¹⁶ each independently represents a substituted or unsubstituted C₁₋₆ alkyl group, a C₂₋₆ alkenyl group, or a substituted or unsubstituted C₆₋₁₉ aralkyl group].

Compound 100 can be prepared from Compound 4.

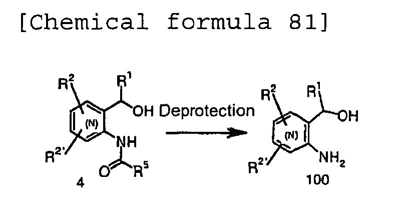

A synthesis process of Compound 100 from Compound 4 differs depending on the kind of the protecting group of Compound 4. Compound 100 can be prepared employing a process suited for the protecting group. Described specifically, when the protecting group is a t-butoxycarbonyl group, deprotection is performed under an acidic condition, while when it is a pivaloyl group, a hydrolysis reaction using a strong alkali or strong acid is employed.

Compound 101 can be prepared from Compound 100 by employing a reductive amination reaction.

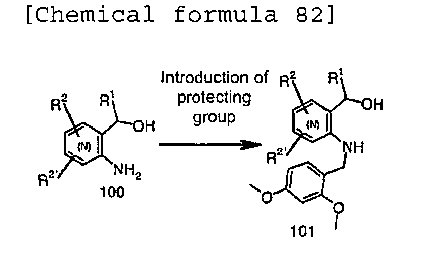

Compound 101 is synthesized from Compound 100 in accordance with an introduction reaction of a protecting group. Compound 100 is reacted with from 1 mole to excess moles, preferably from 1 mole to 5 moles of 2,4-dimethoxybenzaldehyde in the presence of from 1 mole to excess moles, preferably from 1 mole to 10 moles of a reducing agent such as sodium borohydride, sodium triacetoxyborohydride, or sodium cyanoborohydride under an acidic condition, which has been formed by adding from 1 to 1000 moles of acetic acid, formic acid, or propionic acid, in a solvent not adversely affecting the reaction such as dioxane, tetrahydrofuran, dichloroethane, chloroform, dichloromethane, toluene, benzene, isopropanol, ethanol, methanol, or water, or a mixed solvent thereof at from - 78°C to the boiling point of the solvent used for the reaction, preferably from -20°C to 100°C. The reaction time is from one minute to 24 hours, preferably from 30 minutes to 10 hours.

Compound 102 can be prepared from Compound 101.

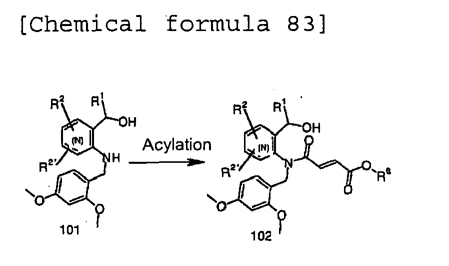

Synthesis of Compound 102 from Compound 101 can be achieved by treating Compound 101 with chloride of fumaric acid or the like in the presence of a base. Described specifically, Compound 101 is reacted with from an equivalent mole to excess moles, preferably from 1 mole to 5 moles of a chloride of fumaric acid or the like in a solvent not adversely affecting the reaction such as benzene, toluene, xylene, diphenyl ether, dichloroethane, dichloromethane, dioxane, tetrahydrofuran, dimethylsulfoxide, or dimethylformamide, or a mixed solvent thereof at from -78°C to the boiling point of the solvent, preferably from -10°C to 50°C. Examples of the base include hydrogen carbonates such as sodium hydrogen carbonate and potassium hydrogen carbonate, carbonates such as sodium carbonate and potassium carbonate, tertiary amines such as triethylamine, and aromatic amines such as pyridine. Of these, hydrogen carbonates are preferred. The base is used in an amount of from equivalent mole to excess moles, preferably from 1 mole to 5 moles. The reaction time is from 5 minutes to 60 hours, preferably from 30 minutes to 10 hours.

Compound 103 can be prepared from Compound 102.

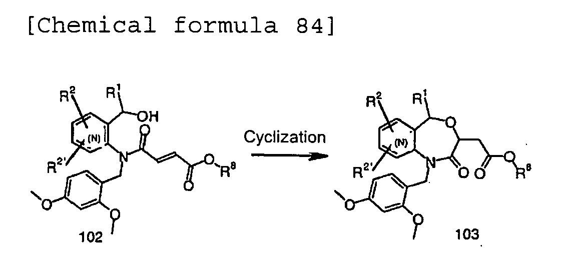

Synthesis of Compound 103 from Compound 102 is achieved by treating Compound 102 under a basic condition. For example, it is reacted in the presence of a base, for example, a carbonate such as sodium hydrogen carbonate, sodium carbonate, potassium carbonate, or cesium carbonate, an organic amine such as triethylamine, diisopropylethylamine, N-methylmorpholine, 4-dimethylaminopyridine, or 1,8-diazabicyclo[5,4,0]undec-7-ene, or a metal hydroxide such as sodium hydroxide in a solvent not adversely affecting the reaction such as benzene, toluene, xylene, diphenyl ether, dichloroethane, dichloromethane, dioxane, tetrahydrofuran, dimethylsulfoxide, dimethylformamide, isopropanol, ethanol, or methanol, or a mixed solvent thereof at from -78°C to the boiling point of the solvent used for the reaction, preferably from 0°C to 150°C. The base is used in an amount of from 0.01 mole to excess moles, preferably from 0.1 mole to 2 moles per mole of Compound 102. The reaction time is from 3 minutes to 48 hours, preferably from 10 minutes to 12 hours.

Compound 104 can be prepared from Compound 103.

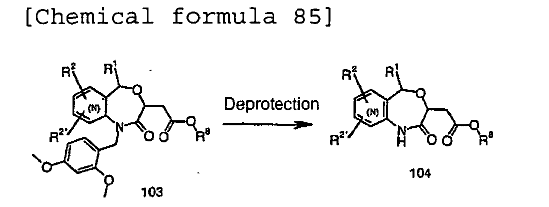

Synthesis of Compound 104 from Compound 103 is achieved by a deprotection reaction. Compound 103 is treated with ammonium cerium nitrate, trifluoroacetic acid, or the like in a solvent not adversely affecting the reaction such as dioxane, tetrahydrofuran, dichloroethane, chloroform, dichloromethane, toluene, benzene, acetone, acetonitrile, ethanol, or methanol, or a mixed solvent thereof at from -78°C to the boiling point of the solvent used for the reaction, preferably from -20°C to 80°C. Ammonium cerium nitrate, trifluoroacetic acid or the like is used in an amount of from 1 mole to excess moles, preferably from 1 mole to 5 moles per mole of Compound 103. The reaction time is from 5 minutes to 60 hours, preferably from 10 minutes to 5 hours.

Compound 105 can be prepared from Compound 104.

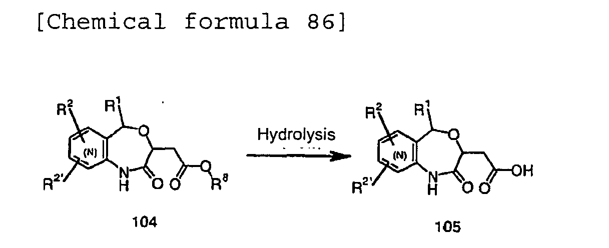

Synthesis of Compound 105 from Compound 104 is achieved by a conventional hydrolysis reaction, for example, hydrolysis reaction in which Compound 104 is treated with a metal hydroxide such as sodium hydroxide or lithium hydroxide or a carbonate such as sodium carbonate or potassium carbonate (from 1 mole to excess moles per mole of Compound 104) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 106 can be prepared from Compound 105.

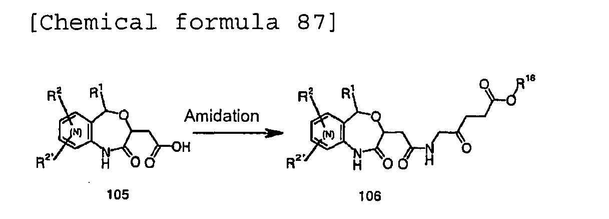

Synthesis of Compound 106 from Compound 105 is achieved reacting Compound 105 with 5-amino-4-oxopentanoate or salt thereof. For example, Compound 105 is reacted with 5-amino-4-oxopentanoate or salt thereof in the presence of a condensation agent such as N,N-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate, or 2-(1H-benzotriazol-1-yl)-1,1,3,3-tetramethyluronium tetrafluoroborate in an inert solvent such as benzene, toluene, xylene, diethyl ether, dichloroethane, chloroform, dichloromethane, dioxane, tetrahydrofuran, dimethylsulfoxide, or dimethylformamide, or a mixture thereof at from -30°C to the boiling point of the solvent used for the reaction, preferably from 0°C to 50°C. The condensation agent is used in an amount of from 1 mole to excess moles, preferably from 1 mole to 5 moles per mole of Compound 105. The reaction may be performed in the presence of a base such as triethylamine, diisopropylethylamine, N-methylmorpholine, 4-dimethylaminopyridine, or 1,8-diazabicyclo[5,4,0]undec-7-ene if necessary. The base may be used in a catalytic amount or excess amount. Further, as a reaction accelerator, an N-hydroxy compound such as 1-hydroxybenzotriazole, N-hydroxysuccinimide, or N-hydroxyphthalimide or a phenol compound such as 4-nitrophenol, 2,4-dinitrophenol, 2,4,5-trichlorophenol, or pentachlorophenol may be added. The reaction accelerator may be used in an amount ranging from a catalytic amount to an excess amount. The reaction time is from 10 minutes to 48 hours, preferably from 30 minutes to 12 hours.

Compound 107 can be prepared from Compound 106.

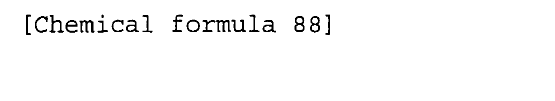

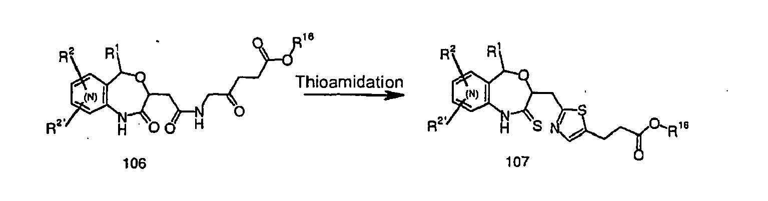

Synthesis of Compound 107 from Compound 106 is achieved by thioamidation of Compound 106. For example, Compound 106 is treated with diphosphorus pentasulfide, Lawesson's reagent, or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 150°C. Diphosphorus pentasulfide, Lawesson's reagent, or the like is used in an amount of from 1 mole to excess moles, preferably from 1 mole to 5 moles per mole of Compound 106. The reaction time is from 5 minutes to 60 hours, preferably from 10 minutes to 5 hours.

Compound 108 can be prepared from Compound 107.

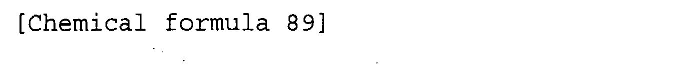

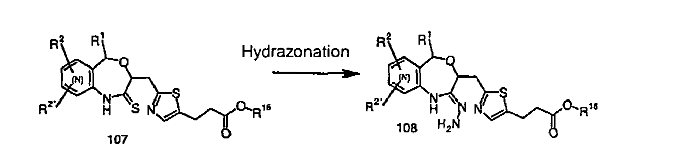

Synthesis of Compound 108 from Compound 107 is achieved by a hydrazonation reaction of Compound 107. For example, Compound 107 is treated with hydrazine or hydrate thereof in a solvent not adversely affecting the reaction such as dioxane, tetrahydrofuran, isopropanol, ethanol, methanol, or water, or a mixed solvent thereof at from -10°C to the boiling point of the solvent used for the reaction, preferably from 0°C to 100°C. Hydrazine or hydrate thereof is used in an amount of from 1 mole to excess moles, preferably from 1 mole to 5 moles per mole of Compound 107. The reaction time is from 5 minutes to 240 hours, preferably from 5 hours to 100 hours.

Compound 109 can be prepared from Compound 108.

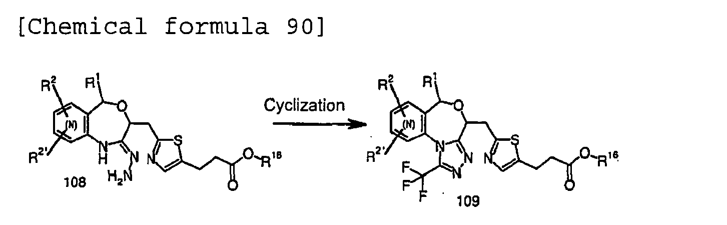

The reaction for preparing Compound 109 from Compound 108 is a cyclization reaction. Compound 108 is treated with trifluoroacetic anhydride and trifluoroacetic acid in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Trifluoroacetic anhydride is used in an amount of from 1 mole to excess moles, preferably from 1 mole to 10 moles per mole of Compound 108. Trifluoroacetic acid is used in an amount of from 0.1 mole to excess moles, preferably from 1 mole to 100 moles per mole of Compound 108. The reaction time is from 1 minute to 60 hours, preferably from 10 minutes to 5 hours. Trifluoroacetic anhydride, trifluoroacetic acid, and the solvent are distilled off under reduced pressure. To the residue is added a solvent not adversely affecting the reaction such as dioxane, tetrahydrofuran, dichloroethane, chloroform, xylene, toluene, or benzene, or a mixed solvent thereof and the reaction is effected at from 30°C to the boiling point of the solvent used for the reaction, preferably from 50°C to 200°C. The reaction time is from 5 minutes to 60 hours, preferably from 30 minutes to 12 hours.

Compound 110 can be prepared from Compound 109.

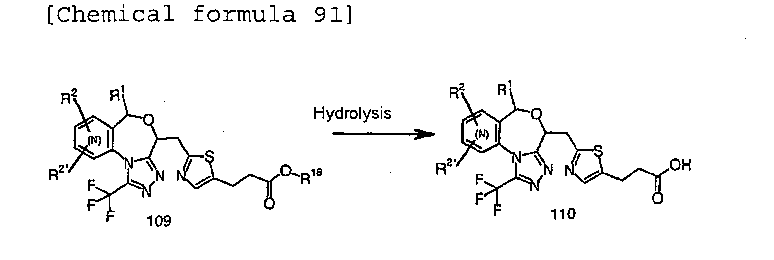

Synthesis of Compound 110 from Compound 109 is achieved by a conventional hydrolysis reaction, for example, hydrolysis reaction in which Compound 109 is treated with a metal hydroxide such as sodium hydroxide or lithium hydroxide or a carbonate such as sodium carbonate or potassium carbonate (from 1 mole to excess moles per mole of Compound 109) at from 0 to 100°C, preferably from 0 to 70°C in a solvent (for example, an alcohol solvent such as methanol or ethanol, an ether solvent such as tetrahydrofuran or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 101 which is a synthesis intermediate can also be synthesized from Compound 15 in accordance with the following process.

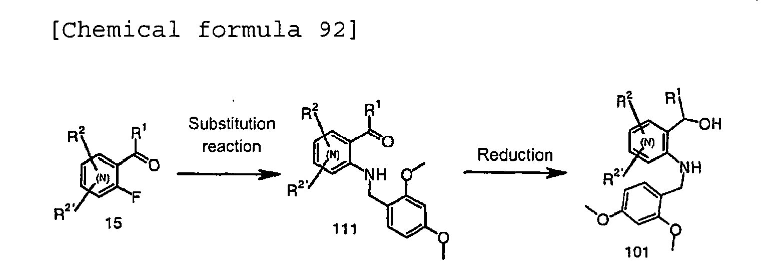

[wherein, R¹, R², and R^{2'} have the same meanings as described above].

Compound 111 can be prepared from Compound 15.

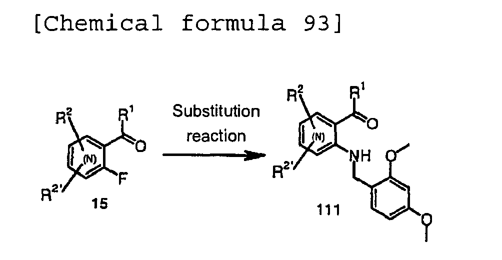

Compound 111 is synthesized from Compound 15 in accordance with a nucleophilic substitution reaction of Compound 15. For example, Compound 15 is treated with from 1 mole to excess moles, preferably from 1 mole to 10 moles, per mole of Compound 15, of a nucleophilic agent such as 2,4-dimethoxybenzylamine, allylamine, benzylamine, or t-butylamine in a solvent not adversely affecting the reaction such as dioxane, tetrahydrofuran, diethyl ether, dimethylformamide, dimethylsulfoxide, toluene, or benzene, or a mixed solvent thereof at from -10°C to the boiling point of the solvent used for the reaction, preferably from 0°C to 150°C. A base, for example, a carbonate such as sodium hydrogen carbonate or potassium carbonate, a hydroxide such as sodium hydroxide, a metal hydride such as sodium hydride, an alkoxide such as tertiary butoxy potassium, or an amine such as triethylamine, diisopropylethylamine, N-methylmorpholine, 4-dimethylaminopyridine, or 1,8-diazabicyclo[5,4,0]undec-7-ene may be added as needed. The base may be used in an amount of from 1 mole to excess moles, preferably from 1 mole to 5 moles per mole of Compound 15. The reaction time is from 5 minutes to 200 hours, preferably from 30 minutes to 20 hours.

Compound 101 can be prepared from Compound 111.

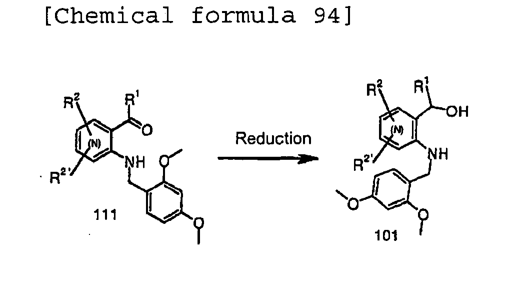

Synthesis of Compound 101 from Compound 111 can be achieved by treating Compound 111 with a reducing agent. For example, Compound 111 is treated with a reducing agent in methanol, ethanol, tetrahydrofuran, diethyl ether, dioxane, dichloromethane, toluene, or the like, or a mixed solvent thereof at from -78°C to the boiling point of the solvent used for the reaction, preferably from -20°C to 100°C. Examples of the reducing agent include sodium borohydride, lithium borohydride, lithium aluminum hydride, diisobutylaluminum hydride, borane dimethylsulfide complex, and borane tetrahydrofuran complex. It is used in an amount of from 1 mole to excess moles, preferably from 1 mole to 5 moles per mole of Compound 111. The reaction time is from 5 minutes to 60 hours, preferably from 10 minutes to 24 hours.

Compound 107, which is a synthesis intermediate, can also be prepared from Compound 103 in the following process.

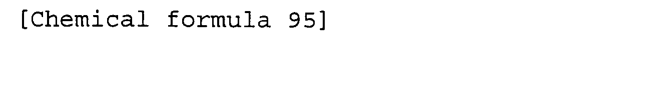

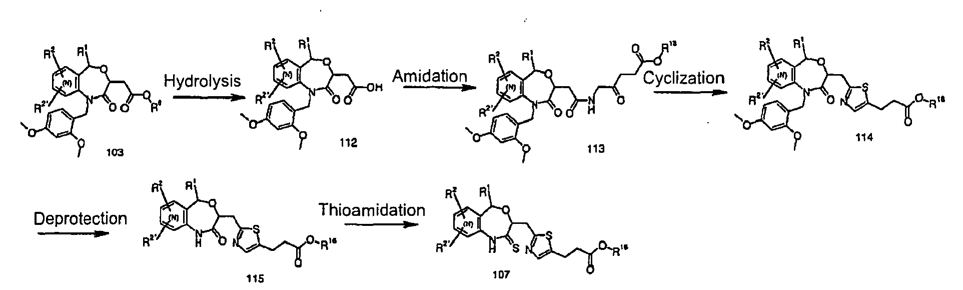

[wherein, R¹, R², and R^{2'} have the same meanings as described above and R⁸ and R¹⁶ each independently represents a substituted or unsubstituted C₁₋₆ alkyl group, a C₂₋₆ alkenyl group, or a substituted or unsubstituted C₆₋₁₉ alkenyl group].

Compound 112 can be prepared from Compound 103.

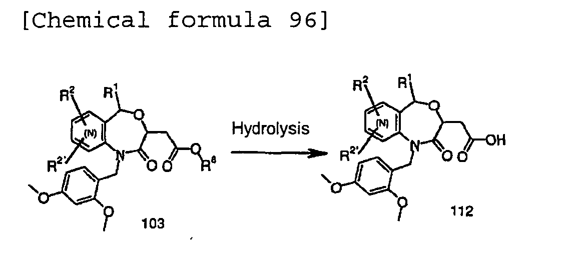

Synthesis of Compound 112 from Compound 103 is achieved by a conventional hydrolysis reaction, for example, hydrolysis reaction in which Compound 103 is treated with a metal hydroxide such as sodium hydroxide or lithium hydroxide or a carbonate such as sodium carbonate or potassium carbonate (from 1 mole to excess moles per mole of Compound 103) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 113 can be prepared from Compound 112.

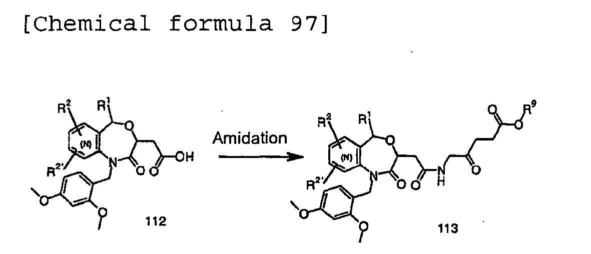

Synthesis of Compound 113 from Compound 112 is achieved reacting Compound 112 with 5-amino-4-oxopentanoate or salt thereof. For example, Compound 112 is reacted with 5-amino-4-oxopentanoate or salt thereof in the presence of a condensation agent such as N,N-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate, or 2-(1H-benzotriazol-1-yl)-1,1,3,3-tetramethyluronium tetrafluoroborate in an inert solvent such as benzene, toluene, xylene, diethyl ether, dichloroethane, chloroform, dichloromethane, dioxane, tetrahydrofuran, dimethylsulfoxide, or dimethylformamide, or a mixture thereof at from -30°C to the boiling point of the solvent used for the reaction, preferably from 0°C to 50°C. The condensation agent is used in an amount of from 1 mole to excess moles, preferably from 1 mole to 5 moles per mole of Compound 112. The reaction may be performed in the presence of a base such as triethylamine, diisopropylethylamine, N-methylmorpholine, 4-dimethylaminopyridine, or 1,8-diazabicyclo[5,4,0]undec-7-ene if necessary. Further, as a reaction accelerator, an N-hydroxy compound such as 1-hydroxybenzotriazole, N-hydroxysuccinimide, or N-hydroxyphthalimide or a phenol compound such as 4-nitrophenol, 2,4-dinitrophenol, 2,4,5-trichlorophenol, or pentachlorophenol may be added. The reaction time is from 10 minutes to 48 hours, preferably from 30 minutes to 12 hours.

Compound 114 can be prepared from Compound 113.

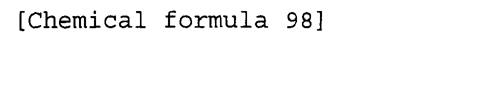

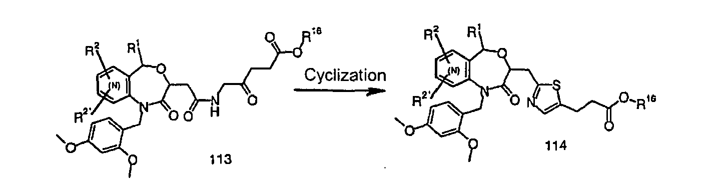

Synthesis of Compound 114 from Compound 113 is achieved by a cyclization reaction. For example, Compound 113 is treated with diphosphorus pentasulfide, Lawesson's reagent, or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 150°C. Diphosphorus pentasulfide, Lawesson's reagent, or the like is used in an amount of from 1 mole to excess moles, preferably from 1 mole to 5 moles, per mole of Compound 113. The reaction time is from 5 minutes to 60 hours, preferably from 10 minutes to 5 hours.

Compound 115 can be prepared from Compound 114.

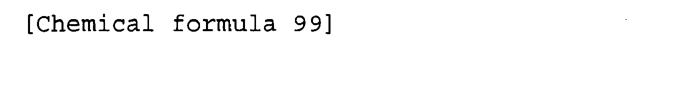

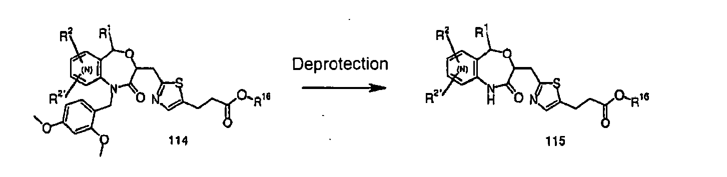

Synthesis of Compound 115 from Compound 114 is achieved by a deprotection reaction. For example, Compound 114 is treated with ammonium cerium nitrate, trifluoroacetic acid, or the like in a solvent not adversely affecting the reaction such as dioxane, tetrahydrofuran, dichloroethane, chloroform, dichloromethane, toluene, benzene, acetone, acetonitrile, ethanol, or methanol, or a mixed solvent thereof at from -78°C to the boiling point of the solvent used for the reaction, preferably from -20°C to 80°C. Ammonium cerium nitrate is used in an amount of from 1 mole to excess moles, preferably from 1 mole to 5 moles per mole of Compound 114. Trifluoroacetic acid is used in an amount of excess moles per mole of Compound 114. The reaction time is from 5 minutes to 60 hours, preferably from 10 minutes to 5 hours.

Compound 107 can be prepared from Compound 115.

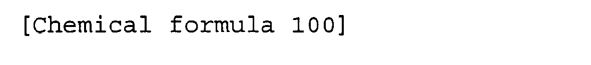

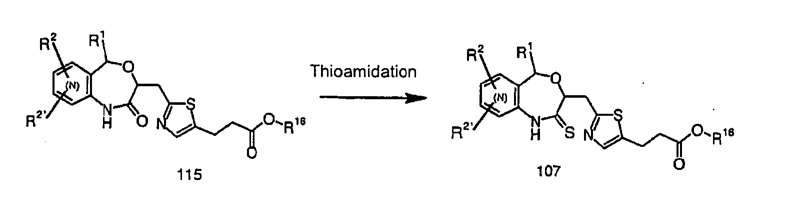

Synthesis of Compound 107 from Compound 115 is achieved by a thioamidation. For example, Compound 115 is treated with diphosphorus pentasulfide, Lawesson's reagent, or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 150°C. Diphosphorus pentasulfide, Lawesson's reagent, or the like is used in an amount of from 1 mole to excess moles, preferably from 1 mole to 5 moles per mole of Compound 105. The reaction time is from 5 minutes to 60 hours, preferably from 10 minutes to 5 hours.

Of Compounds (I), the following compounds can be prepared, for example, in accordance with the following reaction scheme.

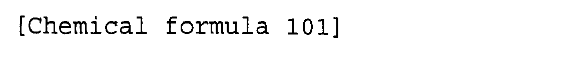

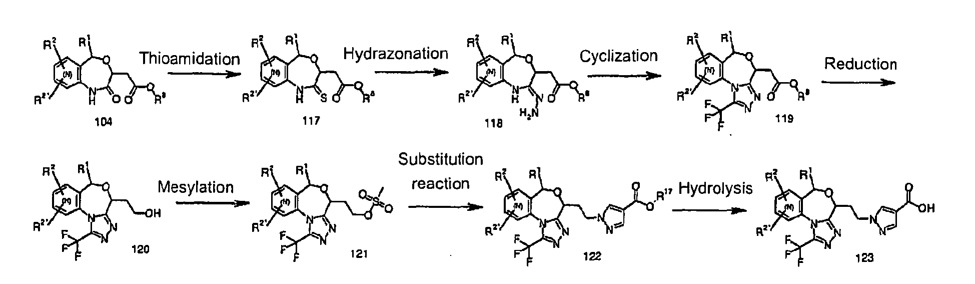

[wherein, R¹, R², and R^{2'} have the same meanings as described above and R⁸ and R¹⁷ each independently represents a substituted or unsubstituted C₁₋₆ alkyl group, a C₂₋₆ alkenyl group, or a substituted or unsubstituted C₆₋₁₉ aralkyl group]

Compound 117 can be prepared from Compound 104.

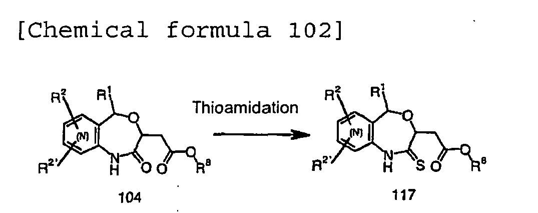

Synthesis of Compound 117 from Compound 104 is achieved by a thioamidation reaction. For example, Compound 104 is treated with diphosphorus pentasulfide, Lawesson's reagent, or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 150°C. Diphosphorus pentasulfide, Lawesson's reagent, or the like is used in an amount of from 1 mole to excess moles, preferably from 1 mole to 5 moles per mole of Compound 104. The reaction time is from 5 minutes to 60 hours, preferably from 10 minutes to 5 hours.

Compound 118 can be prepared from Compound 117.

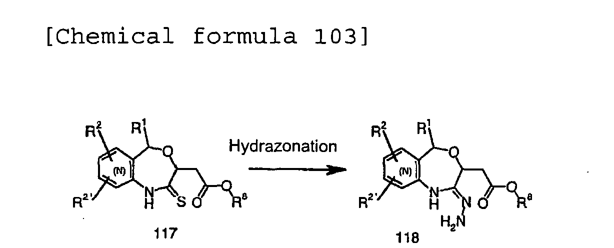

Synthesis of Compound 118 from Compound 117 is achieved by a hydrazonation reaction. For example, Compound 117 is treated with hydrazine or hydrate thereof in a solvent not adversely affecting the reaction such as dioxane, tetrahydrofuran, isopropanol, ethanol, methanol, or water, or a mixed solvent thereof at from -10°C to the boiling point of the solvent used for the reaction, preferably from 0°C to 100°C. Hydrazine or hydrate thereof is used in an amount of from 1 mole to excess moles, preferably from 1 mole to 5 moles per mole of Compound 117. The reaction time is from 5 minutes to 240 hours, preferably from 5 hours to 100 hours.

Compound 119 can be prepared from Compound 118.

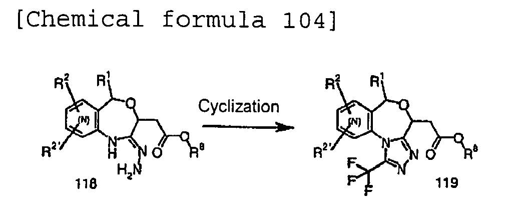

Synthesis of Compound 119 from Compound 118 is achieved by a cyclization reaction. For example, Compound 118 is treated with trifluoroacetic anhydride and trifluoroacetic acid in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Trifluoroacetic anhydride is used in an amount of from 1 mole to excess moles, preferably from 1 mole to 10 moles per mole of Compound 118. Trifluoroacetic acid is used in an amount of from 0.1 mole to excess moles, preferably from 1 to 100 moles per mole of Compound 118. The reaction time is from 1 minute to 60 hours, preferably from 10 minutes to 5 hours. Trifluoroacetic anhydride, trifluoroacetic acid, and the solvent are then distilled off under reduced pressure and to the residue is added a solvent not adversely affecting the reaction such as dioxane, tetrahydrofuran, dichloroethane, chloroform, xylene, toluene, or benzene, or a mixed solvent thereof and the reaction is effected at from 30°C to the boiling point of the solvent used for the reaction, preferably from 50°C to 200°C. The reaction time is from 5 minutes to 60 hours, preferably from 30 minutes to 12 hours.

Compound 120 can be prepared from Compound 119.

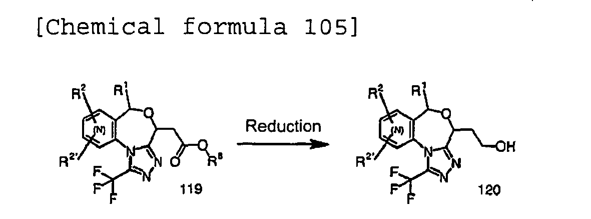

Synthesis of Compound 120 from Compound 119 is achieved treating Compound 119 with a reducing agent. Compound 119 is treated with from 1 mole to excess moles, preferably from 1 mole to 10 moles, per mole of Compound 119, of a reducing agent such as sodium borohydride, lithium borohydride, lithium aluminum hydride, or diisobutylaluminum hydride in a solvent not adversely affecting the reaction such as toluene, dichloroethane, dichloromethane, dioxane, tetrahydrofuran, dimethylformamide, or acetonitrile, or a mixed solvent thereof or a solvent such as methanol, ethanol, tetrahydrofuran, dioxane, dimethylsulfoxide, or water, or a mixed solvent thereof at from -78°C to 100°C, preferably from -50°C to 50°C. The reaction time is from 5 minutes to 60 hours, preferably from 30 minutes to 12 hours.

Compound 121 can be prepared from Compound 120.

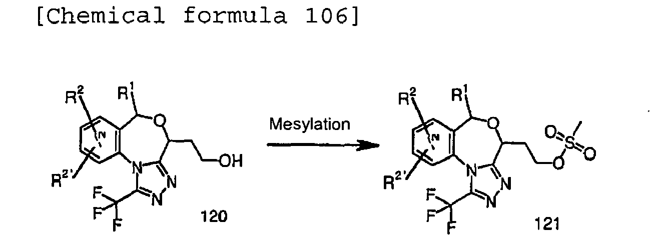

Synthesis of Compound 121 from Compound 120 is achieved by a methanesulfonylation reaction. For example, Compound 120 is treated with a methanesulfonylation agent such as methanesulfonyl chloride or methanesulfonic anhydride in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 50°C. The methanesulfonylation agent is used in an amount of from 1 mole to excess moles, preferably from 1 mole to 5 moles per mole of Compound 120. Further, a base such as triethylamine, diisopropylethylamine, N-methylmorpholine, 4-dimethylaminopyridine, or 1,8-diazabicyclo[5,4,0]undec-7-ene may be added to Compound 120 in an amount of from 1 mole to excess moles, preferably from 1 mole to 10 moles per mole of Compound 120. The reaction time is from 1 minute to 60 hours, preferably from 10 minutes to 5 hours.

Compound 122 can be prepared from Compound 121.

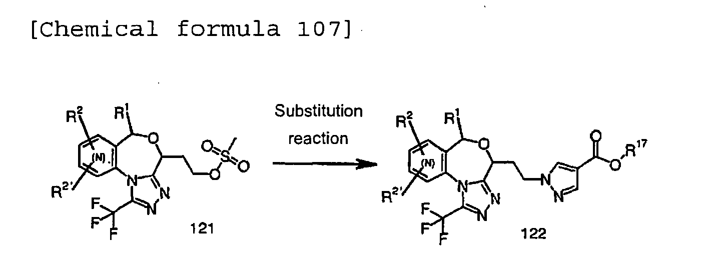

Synthesis is achieved by treating Compound 121 with a heteroaromatic ester having deprotonation ability or salt thereof in a solvent not adversely affecting the reaction such as dioxane, tetrahydrofuran, dichloroethane, chloroform, dichloromethane, toluene, benzene, dimethylformamide, dimethylsulfoxide, or acetonitrile, or a mixed solvent thereof at from -78°C to the boiling point of the solvent used for the reaction, preferably from -20°C to 100°C. Further, a salt prepared separately in advance from a heteroaromatic ester having deprotonation ability such as pyrazole-4-carboxylate and a deprotonation agent such as sodium hydride, butyl lithium, or tertiary butoxy potassium is used in an amount of from 1 mole to excess moles, preferably from 1 mole to 5 moles per mole of Compound 121. The reaction time is from 1 minute to 240 hours, preferably from 10 minutes to 60 hours.

Compound 123 can be prepared from Compound 122.

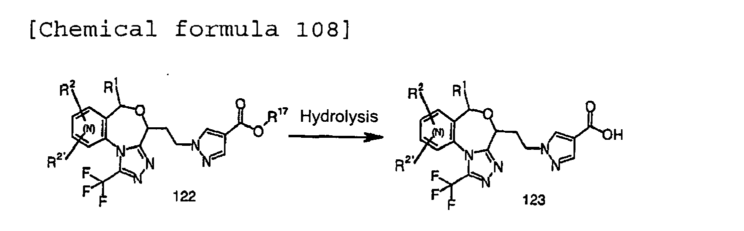

Synthesis of Compound 123 from Compound 122 is achieved by a conventional hydrolysis reaction, for example, hydrolysis reaction in which Compound 122 is treated with a metal hydroxide such as sodium hydroxide or lithium hydroxide or a carbonate such as sodium carbonate or potassium carbonate (from 1 mole to excess moles per mole of Compound 122) at from 0 to 100°C, preferably from 0 to 70°C in a solvent (for example, an alcohol solvent such as methanol or ethanol, an ether solvent such as tetrahydrofuran or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Of Compounds (I), compounds represented by the formula (I-8) can be prepared, for example, in accordance with the following reaction scheme.

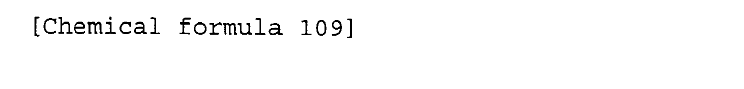

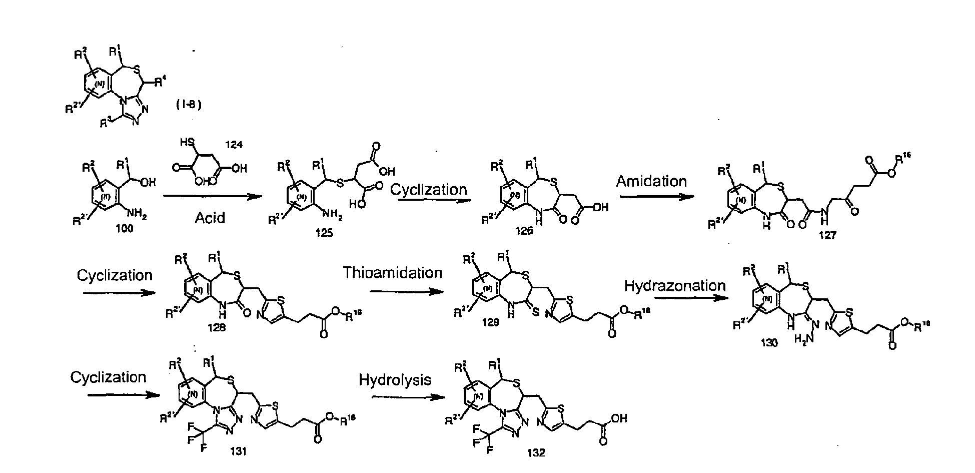

[wherein, R¹, R², and R^{2'} have the same meanings as described above and R¹⁶ represents a substituted or unsubstituted C₁₋₆ alkyl group, a C₂₋₆ alkenyl group or a substituted or unsubstituted C₆₋₁₉ aralkyl group]

Compound 125 can be prepared by a reaction between Compound 100 and thiomalic acid.

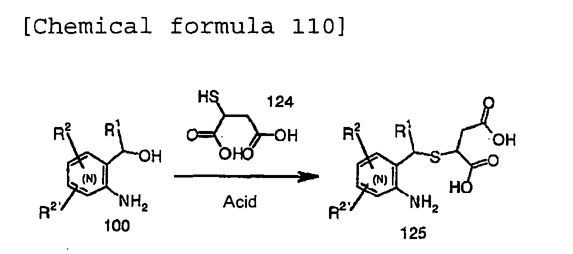

Synthesis of Compound 125 from Compound 100 is achieved by reacting Compound 100 with thiomalic acid under an acidic condition. The reaction is effected in the presence of an acid such as acetic acid, trifluoroacetic acid, hydrochloric acid, sulfuric acid, nitric acid, or toluenesulfonic acid in a solvent such as dichloromethane, dichloroethane, toluene, xylene, or dioxane or in a solventless manner. The acid is used in an amount of from a catalytic amount to excess amount, preferably from 0.1 mole to excess moles per mole of Compound 100. Thiomalic acid is used in an amount of from 1 mole to excess moles, preferably from 1 mole to 5 moles per mole of Compound 100. The reaction temperature is from -50°C to 200°C, preferably from 70°C to 130°C. The reaction time is from 10 minutes to 24 hours, preferably from 30 minutes to 4 hours.

Compound 126 can be prepared from Compound 125.

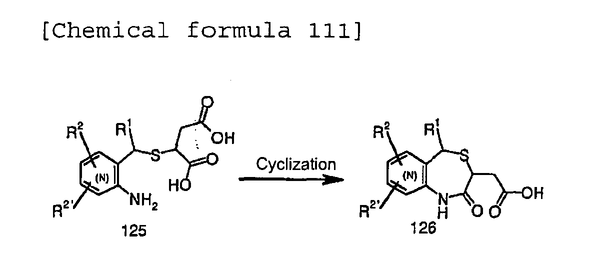

Synthesis of Compound 126 from Compound 125 can be achieved by heating Compound 125 to cause dehydration and ring closure. Described specifically, the reaction is effected at from 30°C to 300°C, preferably from 80°C to 200°C in a solventless manner or in a solvent not adversely affecting the reaction such as benzene, toluene, xylene, diphenyl ether, ethanol, methanol, isopropanol, dichloroethane, dioxane, tetrahydrofuran, dimethylsulfoxide, or dimethylformamide, or a mixed solvent thereof. The reaction time is from 3 hours to 60 hours, preferably from 5 hours to 24 hours.

Compound 127 can be prepared from Compound 126.

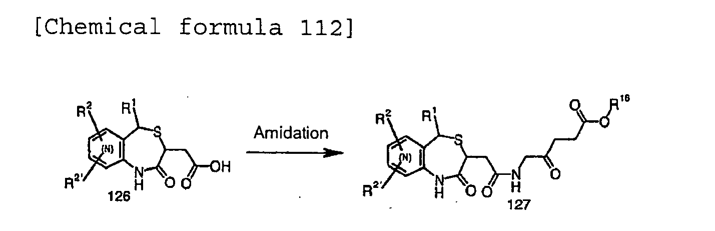

Synthesis of Compound 127 from Compound 126 is achieved reacting Compound 126 with 5-amino-4-oxopentanoate or salt thereof. For example, Compound 126 is reacted with 5-amino-4-oxopentanoate or salt thereof in the presence of a condensation agent such as N,N-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate, or 2-(1H-benzotriazol-1-yl)-1,1,3,3-tetramethyluronium tetrafluoroborate in an inert solvent such as benzene, toluene, xylene, diethyl ether, dichloroethane, chloroform, dichloromethane, dioxane, tetrahydrofuran, dimethylsulfoxide, or dimethylformamide, or a mixture thereof at from -30°C to the boiling point of the solvent used for the reaction, preferably from 0°C to 50°C. The condensation agent is used in an amount of from 1 mole to excess moles, preferably from 1 mole to 5 moles per mole of Compound 126. The reaction may be performed in the presence of a base such as triethylamine, diisopropylethylamine, N-methylmorpholine, 4-dimethylaminopyridine, or 1,8-diazabicyclo[5,4,0]undec-7-ene if necessary. Further, as a reaction accelerator, an N-hydroxy compound such as 1-hydroxybenzotriazole, N-hydroxysuccinimide, or N-hydroxyphthalimide or a phenol compound such as 4-nitrophenol, 2,4-dinitrophenol, 2,4,5-trichlorophenol, or pentachlorophenol may be added. The reaction time is from 10 minutes to 48 hours, preferably from 30 minutes to 12 hours.

Compound 128 can be prepared from Compound 127.

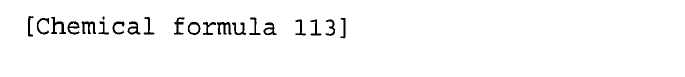

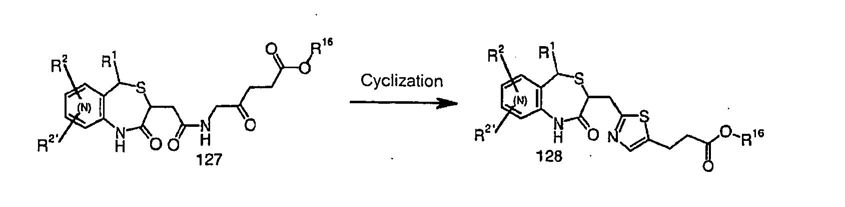

Synthesis of Compound 128 from Compound 127 is achieved by treating Compound 127 with diphosphorus pentasulfide, Lawesson's reagent or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 100°C. Diphosphorus pentasulfide, Lawesson's reagent, or the like is used in an amount of from 1 mole to excess moles, preferably from 1 mole to 5 moles per mole of Compound 127. The reaction time is from 5 minutes to 60 hours, preferably from 10 minutes to 5 hours.

Compound 129 can be prepared from Compound 128.

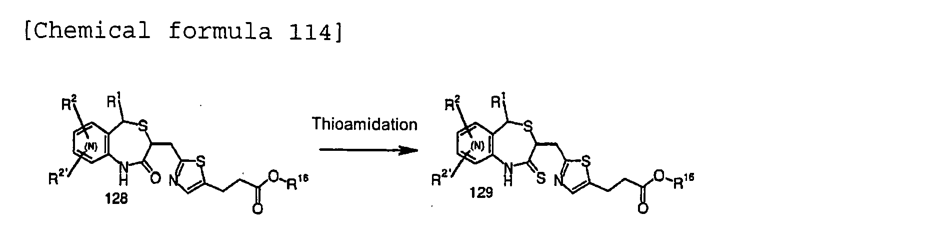

Synthesis of Compound 129 from Compound 128 is achieved by treating Compound 128 with diphosphorus pentasulfide, Lawesson's reagent or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 100°C. Diphosphorus pentasulfide, Lawesson's reagent, or the like is used in an amount of from 1 mole to excess moles, preferably from 1 mole to 5 moles per mole of Compound 128.
The reaction time is from 5 minutes to 60 hours, preferably from 10 minutes to 5 hours.

Compound 130 can be prepared from Compound 129.

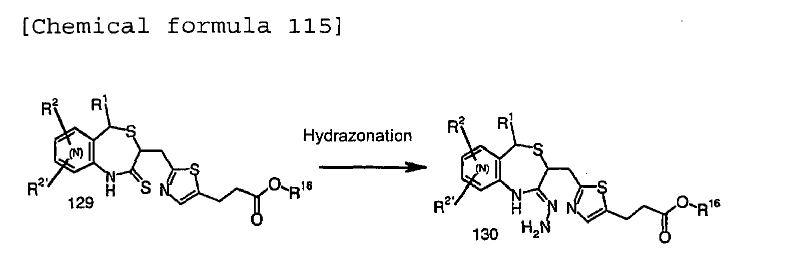

Synthesis of Compound 130 from Compound 129 is achieved treating Compound 129 with hydrazine or hydrate thereof in a solvent not adversely affecting the reaction such as dioxane, tetrahydrofuran, isopropanol, ethanol, methanol, or water, or a mixed solvent thereof at from -10°C to the boiling point of the solvent used for the reaction, preferably from 0°C to 50°C. Hydrazine or hydrate thereof is used in an amount of from 1 mole to excess moles, preferably from 1 mole to 5 moles per mole of Compound 129. The reaction time is from 5 minutes to 240 hours, preferably from 5 hours to 60 hours.

Compound 131 can be prepared from Compound 130.

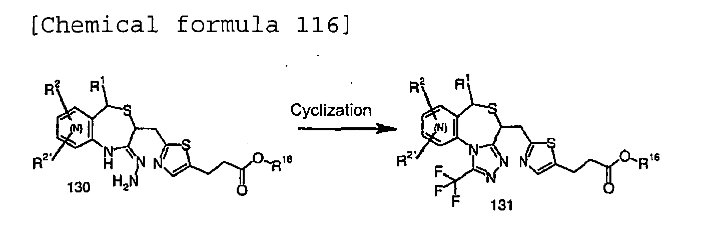

The preparation is achieved by treating Compound 130 with trifluoroacetic anhydride and trifluoroacetic acid in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Trifluoroacetic anhydride is used in an amount of from 1 mole to excess moles, preferably from 1 mole to 10 moles per mole of Compound 130. Trifluoroacetic acid is used in an amount of from 0.1 mole to excess moles, preferably from 1 mole to 100 moles per mole of Compound 130. The reaction time is from 1 minute to 60 hours, preferably from 10 minutes to 5 hours. Trifluoroacetic anhydride, trifluoroacetic acid, and the solvent are distilled off under reduced pressure. To the residue is added a solvent not adversely affecting the reaction such as dioxane, tetrahydrofuran, dichloroethane, chloroform, xylene, toluene, or benzene, or a mixed solvent thereof and reaction is effected at from 30°C to the boiling point of the solvent used for the reaction, preferably from 50°C to 200°C. The reaction time is from 5 minutes to 60 hours, preferably from 30 minutes to 12 hours.

Compound 132 can be prepared from Compound 131.

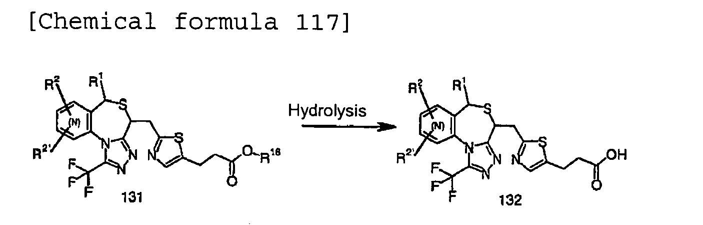

Synthesis of Compound 132 from Compound 131 is achieved by a conventional process, for example, a hydrolysis reaction in which Compound 131 is treated with a metal hydroxide such as sodium hydroxide or lithium hydroxide or a carbonate such as sodium carbonate or potassium carbonate (from 1 mole to excess moles per mole of Compound 131) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Of Compounds (I), the following compounds can be prepared, for example, in accordance with the following reaction scheme.

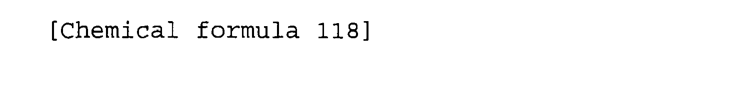

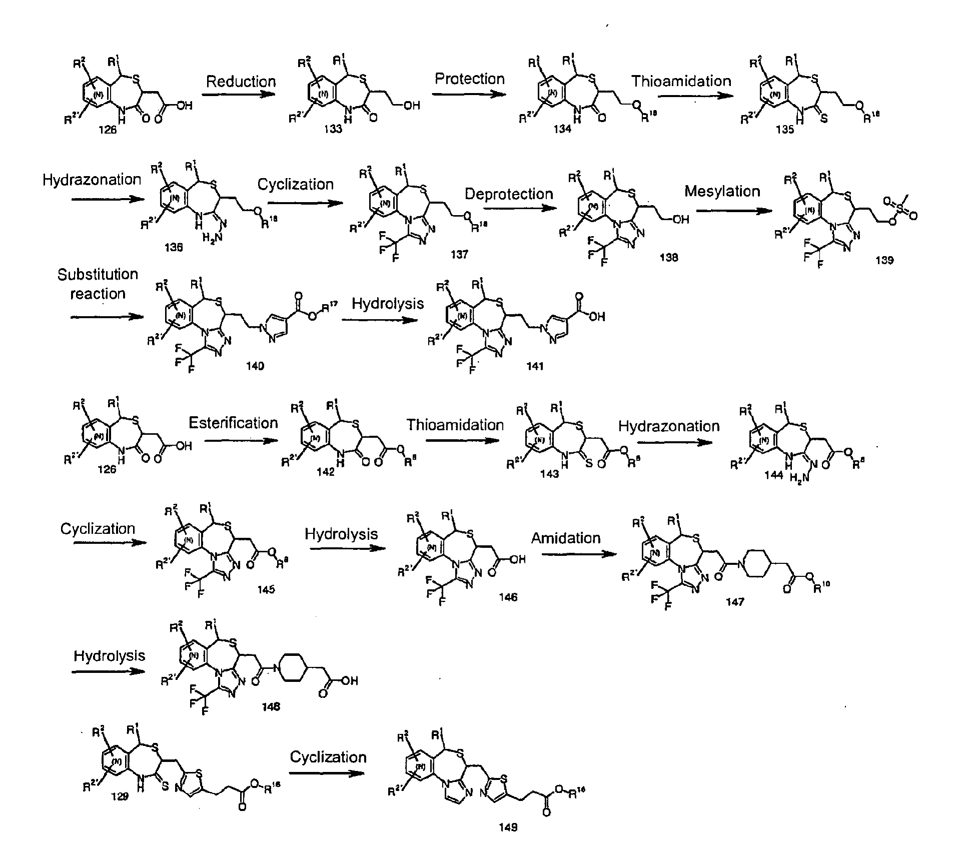

[wherein, R¹, R², and R^{2'} have the same meanings as described above, R⁸, R¹⁰, R¹⁶, and R¹⁷ each independently means a substituted or unsubstituted C₁₋₆ alkyl group, a C₂₋₆ alkenyl group, or a substituted or unsubstituted C₆₋₁₉ aralkyl group, and R¹⁸ means a substituent typically employed as a protecting group such as t-butyldiphenylsilyl group, t-butyldimethylsilyl group, allyl group, or methoxymethyl group].

Compound 133 can be prepared from Compound 126.

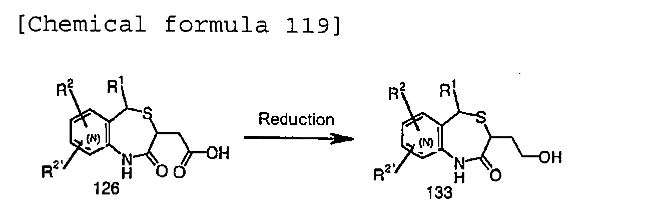

Synthesis of Compound 133 from Compound 126 can be achieved introducing Compound 126 into a mixed acid anhydride thereof and then treating it with a reducing agent. The synthesis is performed treating Compound 126 with from 1 mole to excess moles, preferably from 1 mole to 5 moles of a base such as triethylamine, diisopropylethylamine, N-methylmorpholine, 4-dimethylaminopyridine, or 1,8-diazabicyclo[5,4,0]undec-7-ene and from 1 mole to excess moles, preferably from 1 mole to 3 moles of an acid chloride such as chloroformate or pivalic chloride in a solvent not adversely affecting the reaction such as toluene, dichloroethane, dichloromethane, tetrahydrofuran, dimethylformamide, ethyl acetate, or acetonitrile, or a mixed solvent thereof at a reaction temperature of from -78°C to 100°C, preferably from -20°C to 10°C. The reaction time is from 1 minute to 12 hours, preferably from 10 minutes to 5 hours. The reaction mixture is then reduced by treating with from 1 mole to excess moles, preferably from 1 mole to 5 moles of a reducing agent such as sodium borohydride at a reaction temperature of from -78°C to 100°C, preferably from -20°C to 10°C. Alternatively, the present reaction may be performed directly by reducing Compound 126 by treating it with from 1 mole to excess moles, preferably from 1 mole to 5 moles of a reducing agent such as borane tetrahydrofuran complex or borane dimethylsulfide complex in a solvent not adversely affecting the reaction such as toluene, dichloroethane, dichloromethane, or tetrahydrofuran, or a mixed solvent thereof at a reaction temperature of from -78°C to 200°C, preferably from -50°C to 50°C without introducing Compound 126 into the mixed acid anhydride.

Compound 134 can be prepared from Compound 133.

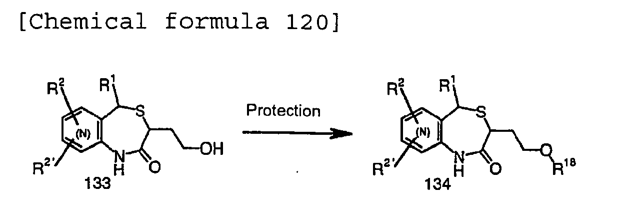

Synthesis of Compound 134 from Compound 133 can be achieved reacting Compound 133 with a protecting-group-introducing agent. Described specifically, the synthesis is effected by reacting Compound 133 with from 1 mole to excess moles, preferably from 1 mole to 5 moles of a protecting-group-introducing agent such as t-butylchlorodiphenylsilane and from 1 mole to excess moles, preferably from 1 mole to 10 moles of a base such as imidazole in a solvent not adversely affecting the reaction such as benzene, toluene, xylene, diphenyl ether, ethanol, methanol, isopropanol, dichloroethane, dioxane, tetrahydrofuran, dimethylsulfoxide, or dimethylformamide, or a mixed solvent thereof at -78°C to 200°C, preferably from -10°C to 100°C. The reaction time is from 5 minutes to 60 hours, preferably from 5 hours to 24 hours.

Compound 135 can be prepared from Compound 134.

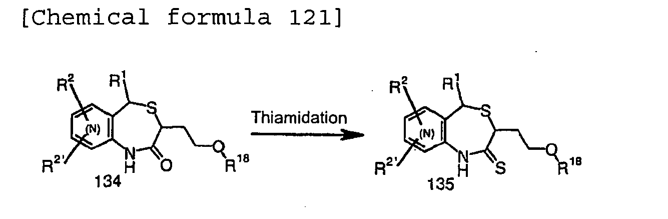

Synthesis of Compound 135 from Compound 134 is achieved treating Compound 134 with diphosphorus pentasulfide, Lawesson's reagent, or the like in a solvent not adversely affecting the reaction such as dioxane, tetrahydrofuran, diethyl ether, dichloroethane, chloroform, dichloromethane, toluene, or benzene, or a mixed solvent thereof at -10°C to the boiling point of the solvent used for the reaction, preferably from 0°C to 150°C.
Diphosphorus pentasulfide, Lawesson's reagent, or the like is used in an amount of from 1 mole to excess moles, preferably from 1 mole to 5 moles per mole of Compound 134. The reaction time is from 5 minutes to 60 hours, preferably from 10 minutes to 5 hours.

Compound 136 can be prepared from Compound 135.

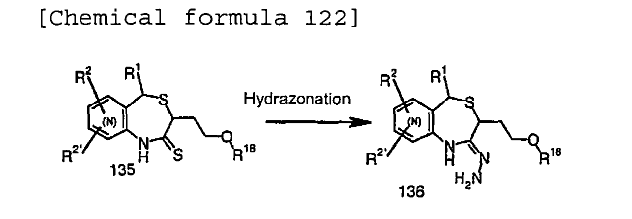

Synthesis of Compound 136 from Compound 135 is achieved treating Compound 135 with hydrazine or hydrate thereof in a solvent not adversely affecting the reaction such as dioxane, tetrahydrofuran, isopropanol, ethanol, methanol, or water, or a mixed solvent thereof at -10°C to the boiling point of the solvent used for the reaction, preferably from 0°C to 50°C. Hydrazine or hydrate thereof is used in an amount of from 1 mole to excess moles, preferably from 1 mole to 5 moles per mole of Compound 135. The reaction time is from 5 minutes to 240 hours, preferably from 5 hours to 60 hours.

Compound 137 can be prepared from Compound 136.

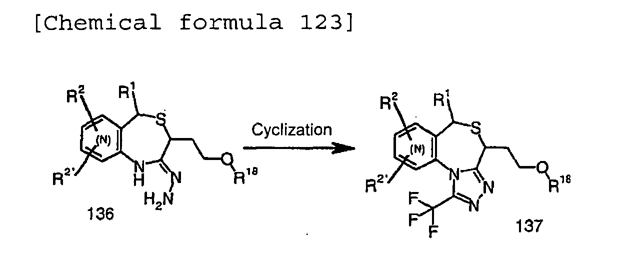

Synthesis of Compound 137 from Compound 136 is achieved treating Compound 136 with trifluoroacetic anhydride and trifluoroacetic acid in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Trifluoroacetic anhydride is used in an amount of from 1 mole to excess moles, preferably from 1 mole to 10 moles per mole of Compound 136. Trifluoroacetic acid is used in an amount of from 0.1 mole to excess moles, preferably from 1 to 100 moles per mole of Compound 136. It is used in an amount of from 1 mole to excess moles, preferably from 1 mole to 10 moles. The reaction time is from 1 minute to 60 hours, preferably from 10 minutes to 5 hours. Trifluoroacetic acid anhydride, trifluoroacetic acid, and the solvent are then distilled off under reduced pressure. To the residue is added a solvent not adversely affecting the reaction such as dioxane, tetrahydrofuran, dichloroethane, chloroform, xylene, toluene, or benzene, or a mixed solvent thereof and reaction is effected at from 30°C to the boiling point of the solvent used for the reaction, preferably from 50°C to 200°C. The reaction time is from 5 minutes to 60 hours, preferably from 30 minutes to 12 hours.

Compound 138 can be prepared from Compound 137.

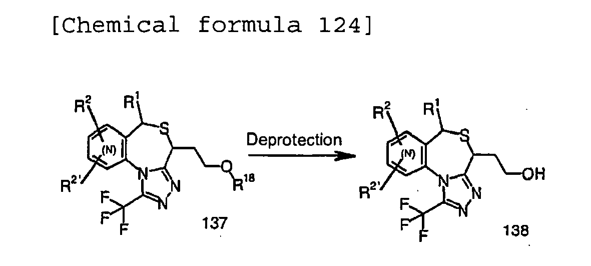

Synthesis of Compound 138 from Compound 137 is achieved by a deprotection method suited for the protecting group employed as R¹⁸. When R¹⁸ is a silyl group such as t-butyldimethylsilyl group, Compound 137 is reacted with a desilylation agent, for example, an ammonium salt of fluorine such as tetrabutylammonium fluoride or hydrogen fluoride pyridine complex in a solvent not adversely affecting the reaction such as dioxane, tetrahydrofuran, isopropanol, ethanol, methanol, or water, or a mixed solvent thereof at from -10°C to the boiling point of the solvent used for the reaction, preferably from 0°C to 100°C. Tetrabutylammonium fluoride is used in an amount of from 1 mole to excess moles, preferably from 1 mole to 10 moles per mole of Compound 137. The reaction time is from 5 minutes to 100 hours, preferably from 5 hours to 60 hours. When R¹⁸ is an allyl group, a method using palladium can be employed, while when it is a methoxymethyl group, a method using an acid can be employed.

Compound 139 can be prepared from Compound 138.

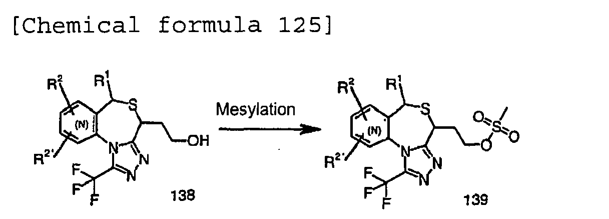

The preparation is achieved treating Compound 138 with a methanesulfonylation agent in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 50°C. Examples of the methanesulfonylation agent include methanesulfonyl chloride and methanesulfonic anhydride. It is used in an amount of from 1 mole to excess moles, preferably from 1 mole to 5 moles per mole of Compound 138. Further, a base such as triethylamine, diisopropylethylamine, N-methylmorpholine, 4-dimethylaminopyridine, or 1,8-diazabicyclo[5,4,0]undec-7-ene is used in an amount of from 1 mole to excess moles, preferably from 1 mole to 10 moles per mole of Compound 138. The reaction time is from 1 minute to 60 hours, preferably from 10 minutes to 5 hours.

Compound 140 can be prepared from Compound 139.

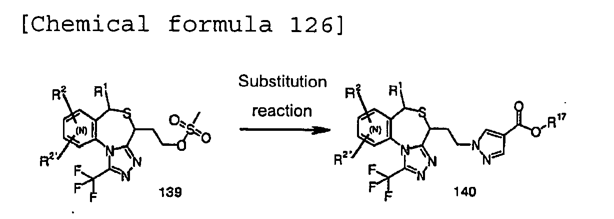

The preparation is achieved by treating Compound 139 with a heteroaromatic ester having deprotonation ability or salt thereof in a solvent not adversely affecting the reaction such as dioxane, tetrahydrofuran, dichloroethane, chloroform, dichloromethane, toluene, benzene, dimethylformamide, dimethylsulfoxide or acetonitrile, or a mixed solvent thereof at from -78°C to the boiling point of the solvent used for the reaction, preferably from -20°C to 100°C. A salt prepared separately in advance from a heteroaromatic ester having deprotonation ability such as pyrazole-4-carboxylate and a deprotonation agent such as sodium hydride, butyl lithium, or tertiary butoxy potassium is used in an amount of from 1 mole to excess moles, preferably from 1 mole to 5 moles per mole of Compound 139. The reaction time is from 1 minute to 240 hours, preferably from 10 minutes to 60 hours.

Compound 141 can be prepared from Compound 140.

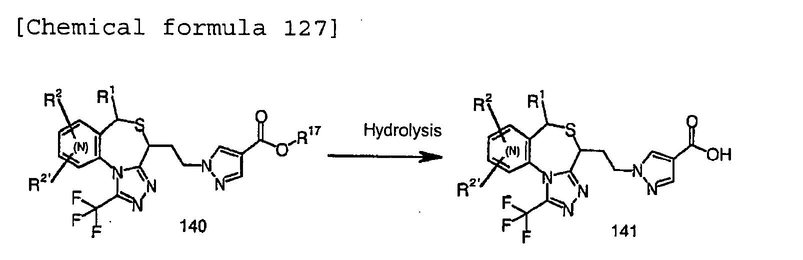

Synthesis of Compound 141 from Compound 140 is achieved by a conventional process, for example, a hydrolysis reaction in which Compound 140 is treated with a metal hydroxide such as sodium hydroxide or lithium hydroxide or a carbonate such as sodium carbonate or potassium carbonate (from 1 mole to excess moles per mole of Compound 140) at from 0 to 100°C, preferably from 0 to 70°C in a solvent (for example, an alcohol solvent such as methanol or ethanol, an ether solvent such as tetrahydrofuran, or water, or a mixed solvent thereof), a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 142 can be prepared from Compound 126.

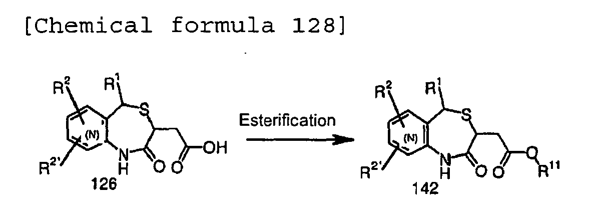

Synthesis of Compound 142 from Compound 126 is achieved by a conventional process, for example, esterification in which Compound 126 is treated in the presence of a catalytic amount or excess amount, preferably from 0.1 mole to 10 moles of an acid such as sulfuric acid or toluenesulfonic acid at from 0 to 150°C, preferably from 0 to 100°C in methanol or ethanol, or esterification using a thionyl chloride-alcohol system or a methyl iodide-potassium carbonate system.

In accordance with the process employed for the synthesis of Compound 137 while using Compound 142, Compound 145 can be synthesized, while in accordance with synthesis processes based on those employed for the synthesis of Compound 21, Compound 24, Compound 27, and Compound 32 while using Compound 145, corresponding compounds can be synthesized, respectively.

Compound 149 can be prepared from Compound 129.

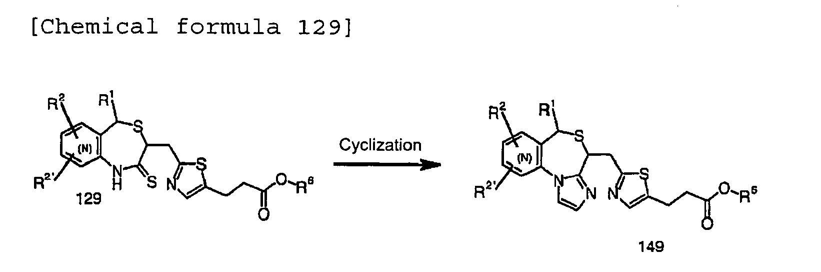

Synthesis of Compound 149 from Compound 129 is achieved treating Compound 129 with aminoacetaldehyde-dimethylacetal or the like in a solvent not adversely affecting the reaction such as dioxane, tetrahydrofuran, dichloroethane, chloroform, dichloromethane, toluene, or benzene, or a mixed solvent thereof at from -78°C to the boiling point of the solvent used for the reaction, preferably from -20°C to 100°C. Aminoacetaldehyde-dimethylacetal or the like is used in an amount of from 1 mole to excess moles, preferably from 1 mole to 10 moles per mole of Compound 129. Further, a salt such as mercury chloride is used in an amount of from 0.5 mole to excess moles, preferably from 1 mole to 10 moles per mole of Compound 129. The reaction time is from 1 minute to 60 hours, preferably from 10 minutes to 5 hours. The reagents and solvent are distilled off under reduced pressure. To the residue is added a solvent not adversely affecting the reaction such as dioxane, tetrahydrofuran, dichloroethane, chloroform, dichloromethane, xylene, toluene, benzene, dimethylformamide, or dimethylsulfoxide, or a mixed solvent thereof. An acid such as toluenesulfonic acid hydrate is added further and the reaction is effected at from 0°C to the boiling point of the solvent used for the reaction, preferably from 30°C to 200°C. The reaction time is from 5 minutes to 60 hours, preferably from 30 minutes to 12 hours.

Of Compounds (I-7), the following compounds can be prepared, for example, in accordance with the following reaction scheme.

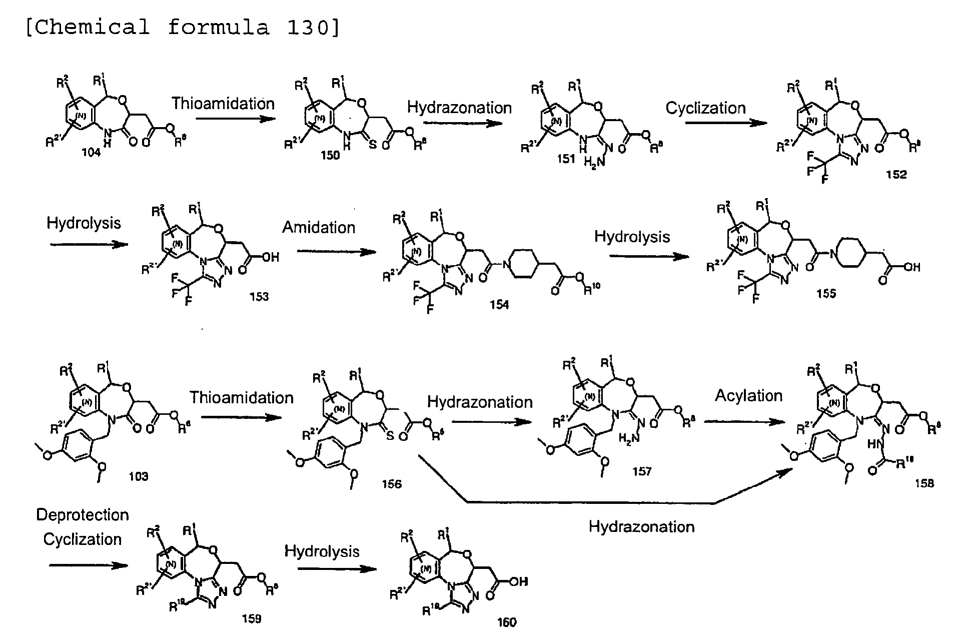

[wherein, R¹, R², and R^{2'} have the same meanings as described above, R³ and R¹⁰ each independently means a substituted or unsubstituted C₁₋₆ alkyl group, a C₂₋₆ alkenyl group, or a substituted or unsubstituted C₆₋₁₉ aralkyl group, and R¹⁹ means R³ or a precursor structure to be introduced into R³].

Compound 150 can be prepared from Compound 104.

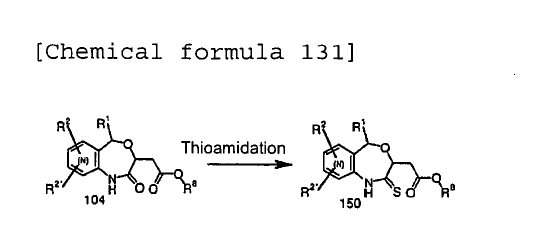

Synthesis of Compound 150 from Compound 104 is achieved by thioamidation of Compound 104. For example, Compound 104 is treated with diphosphorus pentasulfide, Lawesson's reagent, or the like in a solvent not adversely affecting the reaction such as dioxane, tetrahydrofuran, diethyl ether, dichloroethane, chloroform, dichloromethane, toluene,, or benzene, or a mixed solvent thereof at from -10°C to the boiling point of the solvent used for the reaction, preferably from 0°C to 150°C. Diphosphorus pentasulfide, Lawesson's reagent, or the like is used in an amount of from 1 mole to excess moles, preferably from 1 mole to 5 moles per mole of Compound 104. The reaction time is from 5 minutes to 60 hours, preferably from 10 minutes to 5 hours.

Compound 151 can be prepared from Compound 150.

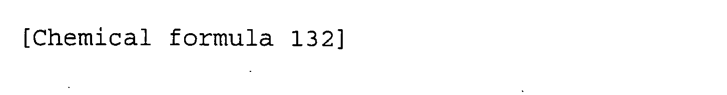

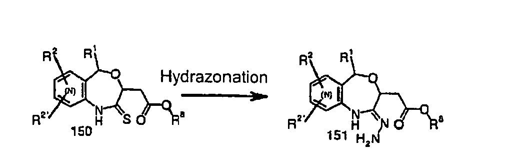

Synthesis of Compound 151 from Compound 150 is achieved by hydrazonation of Compound 150. For example, Compound 150 is treated with hydrazine or hydrate thereof in a solvent not adversely affecting the reaction such as dioxane, tetrahydrofuran, isopropanol, ethanol, methanol,, or water, or a mixed solvent thereof at from -10°C to the boiling point of the solvent used for the reaction, preferably from 0°C to 50°C. Hydrazine or hydrate thereof is used in an amount of from 1 mole to excess moles, preferably from 1 mole to 5 moles per mole of Compound 150. The reaction time is from 5 minutes to 240 hours, preferably from 5 hours to 60 hours.

Compound 152 can be prepared from Compound 151.

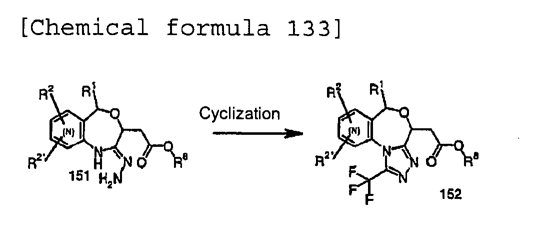

Synthesis of Compound 152 from Compound 151 is achieved treating Compound 151 with trifluoroacetic anhydride in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Trifluoroacetic anhydride is used in an amount of from 1 mole to excess moles, preferably from 1 mole to 10 moles per mole of Compound 151. The reaction time is from 1 minute to 60 hours, preferably from 10 minutes to 5 hours.

Compound 153 can be prepared from Compound 152.

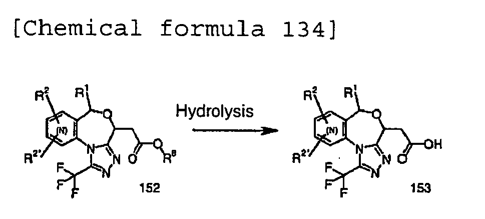

Compound 153 can be synthesized from Compound 152 by a conventional process, for example, an acidic hydrolysis reaction in which Compound 152 is treated with an inorganic acid (from 1 mole to excess moles per mole of Compound 152) such as hydrochloric acid or sulfuric acid in a solvent (for example, an alcohol solvent such as methanol or ethanol, an ether solvent such as tetrahydrofuran, a carboxylic acid such as acetic acid, or water, or a mixed solvent thereof) at from 0 to 200°C, preferably from 0 to 150°C. The synthesis can also be achieved by a basic hydrolysis reaction in which Compound 152 is treated with a metal hydroxide such as sodium hydroxide or lithium hydroxide or a carbonate such as sodium carbonate or potassium carbonate (from 1 mole to excess moles per mole of Compound 152) in a solvent (for example, an alcohol solvent such as methanol or ethanol, an ether solvent such as tetrahydrofuran, or water, or a mixed solvent thereof) at from 0 to 100°C, preferably from 0 to 70°C. The synthesis can also be achieved by a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 154 can be prepared from Compound 153.

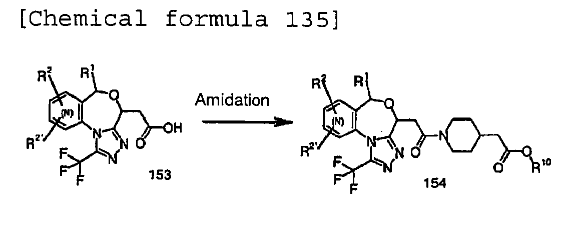

Synthesis of Compound 154 from Compound 153 is achieved condensing Compound 153 with piperidin-4-acetate or salt thereof. For example, Compound 153 is reacted with from 1 mole to excess moles, preferably from 1 mole to 5 moles, per mole of Compound 22, of piperidin-4-acetate or salt thereof in the presence of a condensation agent such as N,N-dicyclohexylcarbodiimide, 1-ethyl-3-(3-dimethylaminopropyl)carbodiimide, diethyl cyanophosphate, benzotriazolyloxy-tris[pyrrolidino]-phosphonium hexafluorophosphate, or 2-(1H-benzotriazol-1-yl)-1,1,3,3-tetramethyluronium tetrafluoroborate in an inert solvent such as benzene, toluene, xylene, diethyl ether, dichloroethane, chloroform, dichloromethane, dioxane, tetrahydrofuran, dimethylsulfoxide, or dimethylformamide, or a mixture thereof at from -30°C to the boiling point of the solvent used for the reaction, preferably from 0°C to 50°C. The condensation agent is used in an amount of from 1 mole to excess moles, preferably from 1 mole to 5 moles per mole of Compound 153. The reaction may be performed in the presence of a base such as triethylamine, diisopropylethylamine, N-methylmorpholine, 4-dimethylaminopyridine, or 1,8-diazabicyclo[5,4,0]undec-7-ene if necessary. Further, as a reaction accelerator, an N-hydroxy compound such as 1-hydroxybenzotriazole, N-hydroxysuccinimide, or N-hydroxyphthalimide or a phenol compound such as 4-nitrophenol, 2,4-dinitrophenol, 2,4,5-trichlorophenol, or pentachlorophenol may be added. The reaction time is from 10 minutes to 48 hours, preferably from 30 minutes to 12 hours.

Compound 155 can be prepared from Compound 154.

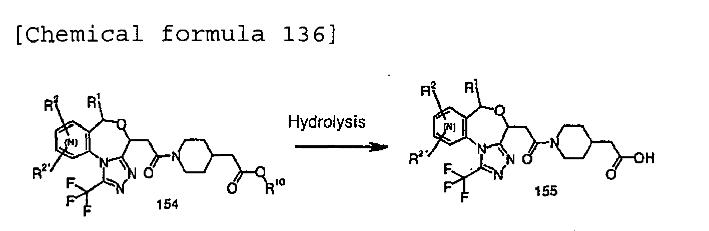

Compound 155 can be synthesized from Compound 154 by a conventional process, for example, an acidic hydrolysis reaction in which Compound 154 is treated with an inorganic acid (from 1 mole to excess moles per mole of Compound 154) such as hydrochloric acid or sulfuric acid in a solvent (for example, an alcohol solvent such as methanol or ethanol, an ether solvent such as tetrahydrofuran, a carboxylic acid such as acetic acid, or water, or a mixed solvent thereof) at from 0 to 200°C, preferably from 0 to 150°C. The synthesis can also be achieved by a basic hydrolysis reaction in which Compound 154 is treated with a metal hydroxide such as sodium hydroxide, or lithium hydroxide or a carbonate such as sodium carbonate or potassium carbonate (from 1 mole to excess moles per mole of Compound 154) in a solvent (for example, an alcohol solvent such as methanol or ethanol, an ether solvent such as tetrahydrofuran, or water, or a mixed solvent thereof) at from 0 to 100°C, preferably from 0 to 70°C. The synthesis can also be achieved by a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Compound 156 can be prepared from Compound 103.

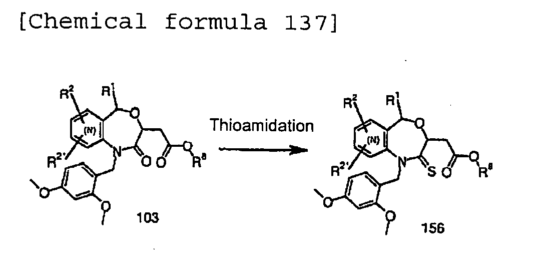

Compound 156 is synthesized from Compound 103 by the thioamidation of Compound 103. For example, Compound 103 is treated with diphosphorus pentasulfide, Lawesson's reagent, or the like in a solvent not adversely affecting the reaction such as dioxane, tetrahydrofuran, diethyl ether, dichloroethane, chloroform, dichloromethane, toluene, or benzene, or a mixed solvent thereof at from -10°C to the boiling point of the solvent used for the reaction, preferably from 0°C to 150°C. Diphosphorus pentasulfide, Lawesson's reagent, or the like is used in an amount of from 1 mole to excess moles, preferably from 1 mole to 5 moles, per mole of Compound 104. The reaction time is from 5 minutes to 60 hours, preferably from 10 minutes to 5 hours.

Compound 157 can be prepared from Compound 156.

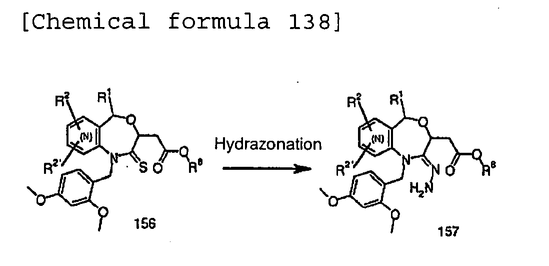

Synthesis of Compound 157 from Compound 156 is achieved by hydrazonation of Compound 156. For example, Compound 156 is treated with hydrazine or hydrate thereof in a solvent not adversely affecting the reaction such as dioxane, tetrahydrofuran, isopropanol, ethanol, methanol, or water, or a mixed solvent thereof at from -10°C or to the boiling point of the solvent used for the reaction, preferably from 0°C to 80°C. Hydrazine or hydrate thereof is used in an amount of from 1 mole to excess moles, preferably from 1 mole to 5 moles per mole of Compound 156. In this reaction, an inorganic salt such as mercury chloride, mercury sulfate, mercury trifluoroacetate or mercury acetate may be added. The reaction time is from 5 minutes to 240 hours, preferably from 5 hours to 60 hours.

Compound 158 can be prepared from Compound 157.

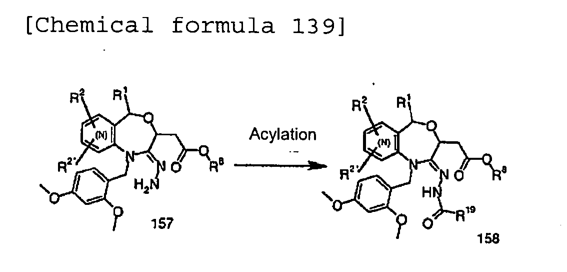

Synthesis of Compound 158 from Compound 157 is achieved by acylation of Compound 157. For example, Compound 157 is treated with an acid anhydride such as trifluoroacetic anhydride, chlorodifluoroacetic anhydride, or acetic anhydride or an acid chloride such as fluoroacetyl chloride, pivaloyl chloride, or acetyl chloride in a solvent not adversely affecting the reaction such as dioxane, tetrahydrofuran, dichloroethane, chloroform, dichloromethane, toluene, benzene, or ethyl acetate, or a mixed solvent thereof at from -78°C to the boiling point of the solvent used for the reaction, preferably from -20°C to 100°C. Trifluoroacetic anhydride is used in an amount of from 1 mole to excess moles, preferably from 1 mole to 10 moles per mole of Compound 157. The reaction time is from 1 minute to 60 hours, preferably from 10 minutes to 5 hours.

Compound 158 can also be prepared from Compound 156.

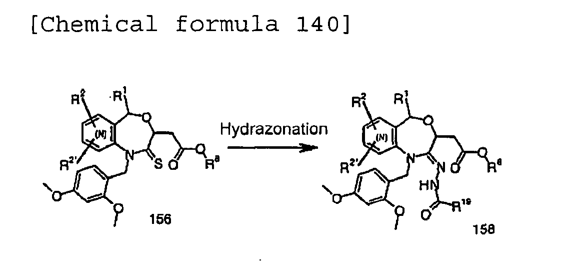

Synthesis of Compound 158 from Compound 156 can also be achieved by hydrazonation of Compound 156. For example, Compound 156 is treated with acylhydrazine in a solvent not adversely affecting the reaction such as dioxane, tetrahydrofuran, or dichloromethane, or a mixed solvent thereof at from -10°C to the boiling point of the solvent used for the reaction, preferably from 0°C to 80°C. Acylhydrazine is used in an amount of from 1 mole to excess moles, preferably from 1 mole to 5 moles per mole of Compound 156. Also in this reaction, an inorganic salt such as mercury chloride, mercury sulfate, mercury trifluoroacetate, or mercury acetate may be added. The reaction time is from 5 minutes to 240 hours, preferably from 5 hours to 60 hours.

Compound 159 can be prepared from Compound 158.

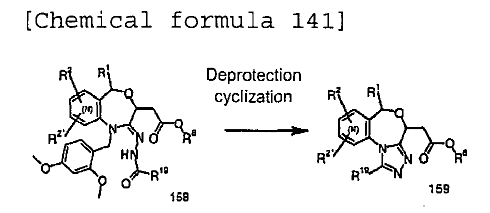

Synthesis of Compound 159 from Compound 158 is achieved by treating Compound 158 with an acid such as trifluoroacetic acid, hydrochloric acid gas, or sulfuric acid in a solvent not adversely affecting the reaction such as dioxane, tetrahydrofuran, dichloromethane, or chloroform, or a mixed solvent thereof or in a solventless manner at from -10°C to the boiling point of the solvent used for the reaction, preferably from 0°C to 80°C. This reaction may be effected in the presence of an equimolar or large excess amount of anisole or thioanisole. The reaction time is from 5 minutes to 240 hours, preferably from 5 hours to 60 hours.

Compound 160 can be prepared from Compound 159.

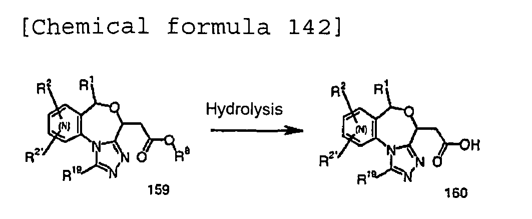

Compound 160 can be synthesized from Compound 159 by a conventional process, for example, an acidic hydrolysis reaction in which Compound 159 is treated with an inorganic acid (from 1 mole to excess moles per mole of Compound 159) such as hydrochloric acid or sulfuric acid in a solvent (for example, an alcohol solvent such as methanol or ethanol, an ether solvent such as tetrahydrofuran, a carboxylic acid such as acetic acid, or water, or a mixed solvent thereof) at from 0 to 200°C, preferably from 0 to 150°C. The synthesis can also be achieved by a basic hydrolysis reaction in which Compound 159 is treated with a metal hydroxide such as sodium hydroxide or lithium hydroxide or a carbonate such as sodium carbonate or potassium carbonate (from 1 mole to excess moles per mole of Compound 159) in a solvent (for example, an alcohol solvent such as methanol or ethanol, an ether solvent such as tetrahydrofuran, or water, or a mixed solvent thereof) at from 0 to 100°C, preferably from 0 to 70°C. The synthesis can also be achieved by a catalytic reduction reaction in the presence of a catalyst such as palladium-carbon, a process using an acid such as trifluoroacetic acid, or a process utilizing a deallylation reaction in the presence of a palladium catalyst. The process differs, depending on the kind of the ester.

Of Compounds (I), compounds represented by the formula (I-9) can be prepared, for example, in accordance with the following reaction scheme.

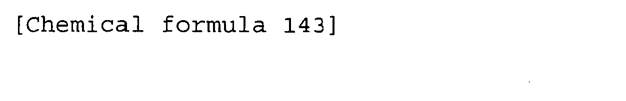

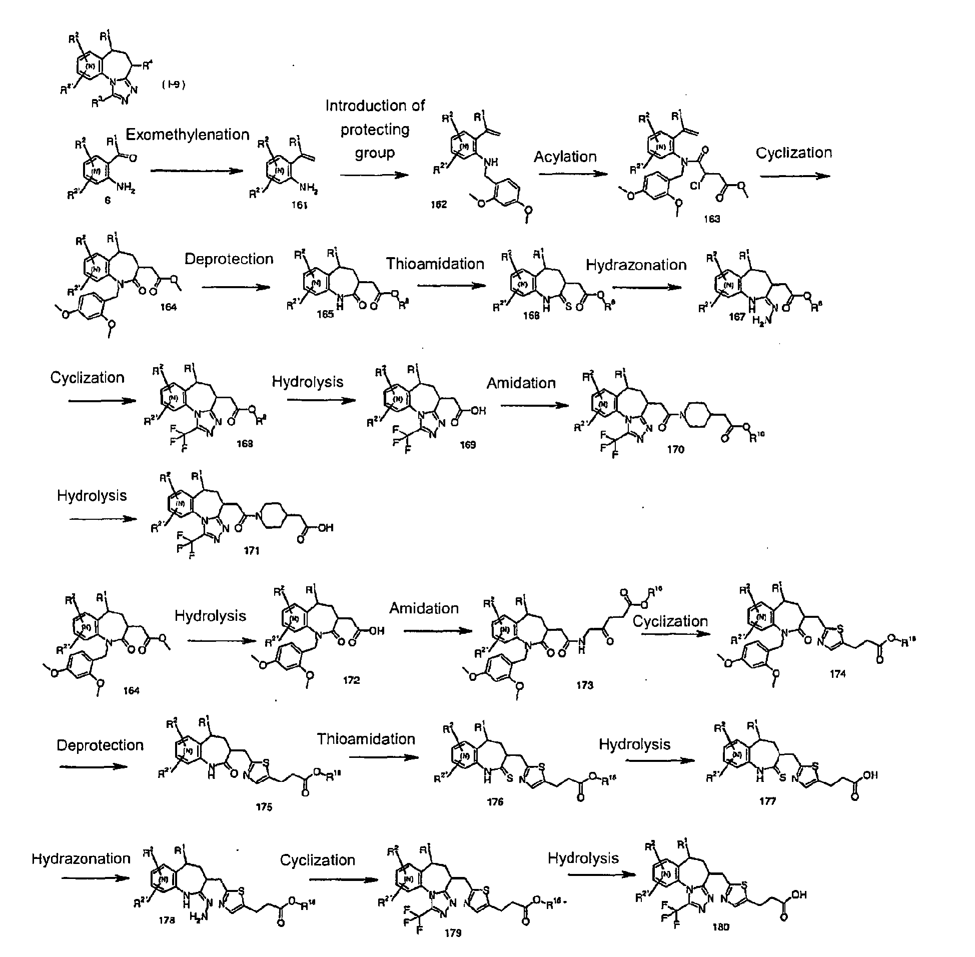

[wherein, R¹, R², and R^{2'} have the same meanings as described above and R⁸, R¹⁰, and R¹⁶ each independently represents a substituted or unsubstituted C₁₋₆ alkyl group, a C₂₋₆ alkenyl group, or a substituted or unsubstituted C₆₋₁₉ aralkyl group]

Compound 161 can be prepared by the exomethylenation of Compound 6.

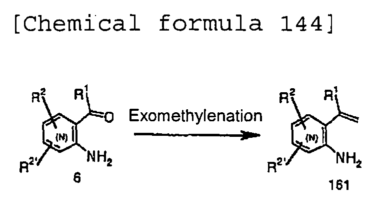

Synthesis of Compound 161 from Compound 6 is achieved by reacting Compound 6 with phosphorus ylide, which has been generated by the reaction between a methylphosphonium salt such as methyltriphenylphosphonium bromide and a base such as n-butyl lithium, t-butoxy potassium, or lithium hexamethyldisilazide, in a solvent not adversely affecting the reaction such as tetrahydrofuran, dioxane, or toluene. The methylphosphonium salt is used in an amount of from an equal amount to an excess amount relative to Compound 6, preferably from 1 mole to 5 moles. The base is used in an amount of from 1 mole to excess moles, preferably from 1 mole to 5 moles per mole of Compound 6. The reaction temperature is from -50°C to 200°C, preferably from 0°C to 130°C. The reaction time is from 10 minutes to 24 hours, preferably from 30 minutes to 10 hours.

Compound 162 can be prepared from Compound 161.

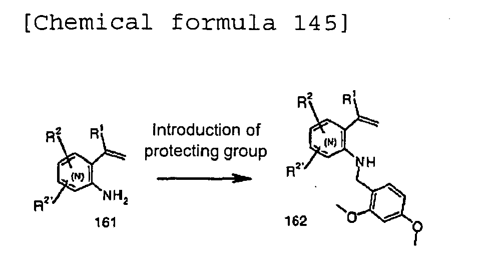

Synthesis of Compound 162 from Compound 161 is achieved by reacting Compound 161 with from 1 mole to excess moles, preferably from 1 mole to 5 moles of 2,4-dimethoxybenzaldehyde in the presence of from 1 mole to excess moles, preferably from 1 mole to 10 moles of a reducing agent such as sodium borohydride, sodium triacetoxyborohydride, or sodium cyanoborohydride in a solvent not adversely affecting the reaction such as dioxane, tetrahydrofuran, dichloroethane, chloroform, dichloromethane, toluene, benzene, isopropanol, ethanol, methanol, or water, or a mixed solvent thereof at from -78°C to the boiling point of the solvent used for the reaction, preferably from -20°C to 100°C under an acidic condition containing from 1 to 1000 moles of acetic acid, formic acid, or propionic acid. The reaction time is from 1 minute to 24 hours, preferably from 30 minutes to 10 hours.

Compound 163 can be prepared from Compound 162.

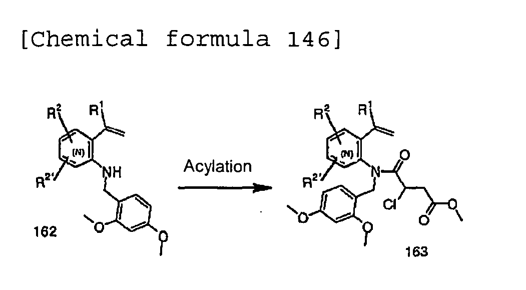

Synthesis of Compound 163 from Compound 162 is achieved by acylation of Compound 162. For example, Compound 162 is treated with an acid chloride such as methyl 3,4-dichloro-4-oxobutanoate in the presence of a base. Described specifically, Compound 162 is reacted with from an equimolar amount to excess moles, preferably from 1 mole to 5 moles of 3,4-dichloro-4-oxobutanoate in a solvent not adversely affecting the reaction such as ethyl acetate, benzene, toluene, xylene, diphenyl ether, dichloroethane, dichloromethane, dioxane, tetrahydrofuran, dimethylsulfoxide, or dimethylformamide, or a mixed solvent thereof at from -78°C to the boiling point of the solvent, preferably from -10°C to 50°C. Examples of the base include hydrogen carbonates such as sodium hydrogen carbonate and potassium hydrogen carbonate, carbonates such as sodium carbonate and potassium carbonate, tertiary amines such as triethylamine and aromatic amines such as pyridine. Of these, the hydrogen carbonate is preferred. It is used in an amount of from an equimolar amount to excess moles, preferably from 1 mole to 5 moles. The reaction time is from 5 minutes to 60 hours, preferably from 30 minutes to 10 hours.

Compound 164 can be prepared from Compound 163.

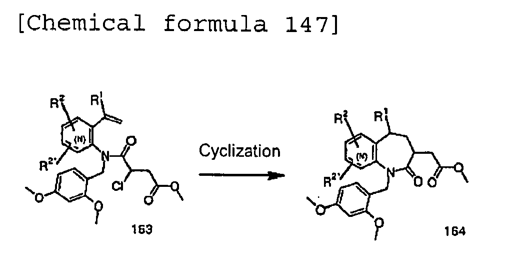

Synthesis of Compound 164 from Compound 163 is achieved treating Compound 163 with tributyltin hydride and 2,2'-azobis(2-methylpropionitrile) in a solvent not adversely affecting the reaction such as dioxane, tetrahydrofuran, diethyl ether, dichloroethane, chloroform, dichloromethane, toluene, or benzene, or a mixed solvent thereof at from 0°C to the boiling point of the solvent used for the reaction, preferably from 0°C to 150°C. Tributyltin hydride is used in an amount of from 1 mole to excess moles, preferably from 1 mole to 5 moles per mole of Compound 163. 2,2'-Azobis(2-methylpropionitrile) is used in a catalytic amount, preferably from 0.001 to 0.5 mole per mole of Compound 163. The reaction time is from 5 minutes to 60 hours, preferably from 10 minutes to 5 hours.

From Compound 164, Compound 171 which is a corresponding derivative can be synthesized in a similar manner to that employed for the synthesis of Compound 155.
From Compound 164, Compound 180 which is a corresponding derivative can be synthesized in a similar manner to that employed for the synthesis of Compound 110.

Of Compounds (I), compounds represented by the formula (1-9) can be prepared, for example, in accordance with the following reaction scheme.

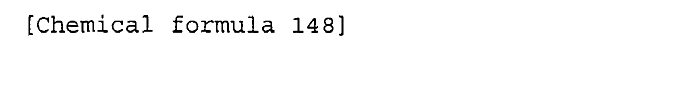

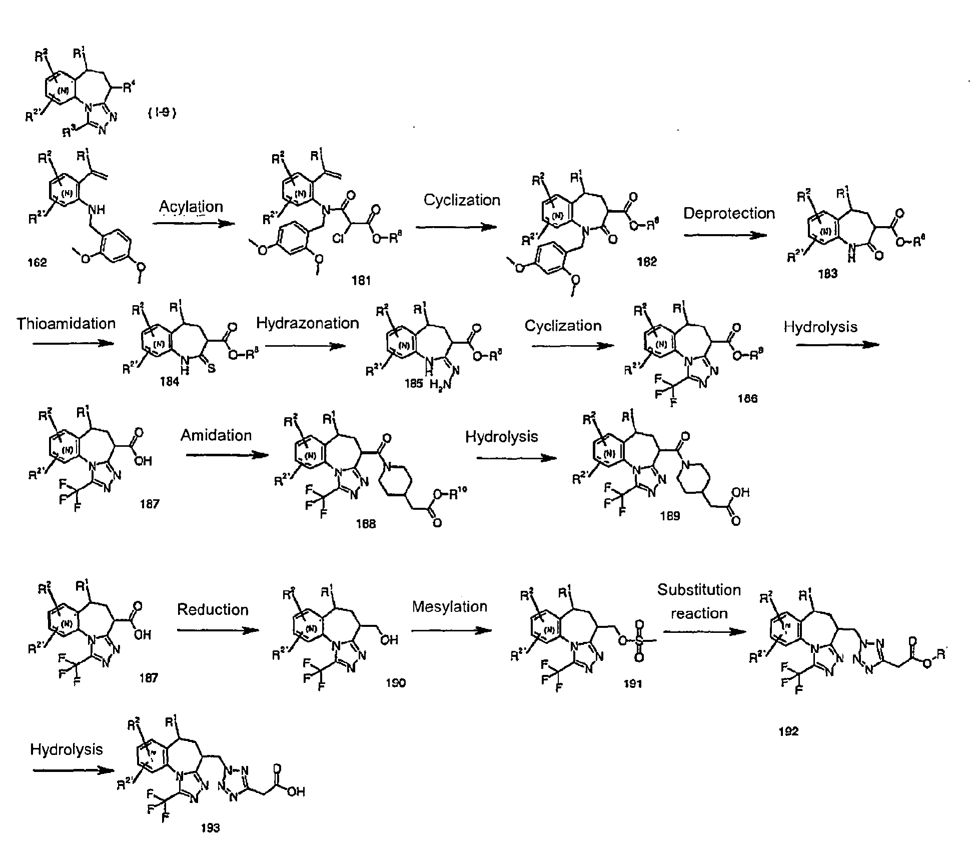

[wherein, R¹, R², and R^{2'} have the same meanings as described above and R⁸, R¹⁰, and R¹⁷ each independently represents a substituted or unsubstituted C₁₋₆ alkyl group, a C₂₋₆ alkenyl group, or a substituted or unsubstituted C₆₋₁₉ aralkyl group]

Compound 181 can be prepared from Compound 162.

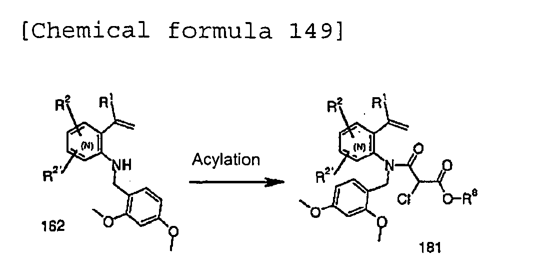

Synthesis of Compound 181 from Compound 162 is achieved by the acylation of Compound 162. For example, Compound 162 is treated with an acid chloride such as methyl 2,3-dichloro-3-oxopropanoate in the presence of a base. Described specifically, Compound 162 is reacted with from an equimolar amount to excess moles, preferably from 1 mole to 5 moles, per mole thereof, of 2,3-dichloro-3-oxopropanoate or the like in a solvent not adversely affecting the reaction such as ethyl acetate, benzene, toluene, xylene, diphenyl ether, dichloroethane, dichloromethane, dioxane, tetrahydrofuran, dimethylsulfoxide, or dimethylformamide, or a mixed solvent thereof at from -78°C to the boiling point of the solvent, preferably from -10°C to 50°C. Examples of the base include hydrogen carbonates such as sodium hydrogen carbonate and potassium hydrogen carbonate, carbonates such as sodium carbonate and potassium carbonate, tertiary amines such as triethylamine, and aromatic amines such as pyridine. Of these, the hydrogen carbonate is preferred. It is used in an amount of from an equimolar amount to excess moles, preferably from 1 mole to 5 moles. The reaction time is from 5 minutes to 60 hours, preferably from 30 minutes to 10 hours.

Compound 182 can be prepared from Compound 181.

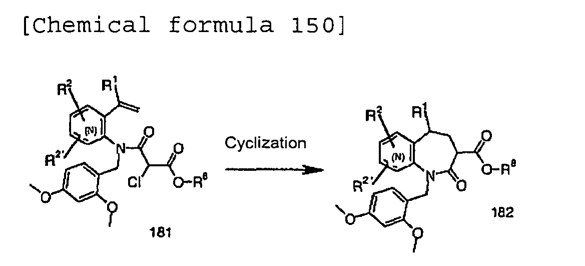

Synthesis of Compound 182 from Compound 181 is achieved by treating Compound 181 with tributyltin hydride and 2,2'-azobis(2-methylpropionitrile) in a solvent not adversely affecting the reaction such as dioxane, tetrahydrofuran, diethyl ether, dichloroethane, chloroform, dichloromethane, toluene, or benzene, or a mixed solvent thereof at from 0°C to the boiling point of the solvent used for the reaction, preferably from 0°C to 150°C. Tributyltin hydride is used in an amount of from 1 mole to excess moles, preferably from 1 mole to 5 moles per mole of Compound 181, while 2,2'-azobis(2-methylpropionitrile) is used in an amount of a catalytic amount, preferably from 0.001 to 0.5 mole per mole of Compound 181. The reaction time is from 5 minutes to 60 hours, preferably from 10 minutes to 10 hours.

From Compound 182, Compound 189 which is a corresponding derivative can be synthesized in a similar manner to that employed for the synthesis of Compound 155.
From Compound 182, Compound 193 which is a corresponding derivative can be synthesized in a similar manner to that employed for the synthesis of Compound 32.

The preparation processes of the compounds of the present invention are not limited to the above-described ones. The compounds of the present invention thus prepared can be isolated and purified by a known separation process such as recrystallization, distillation, partitioning, or chromatography. When the compounds of the present invention thus prepared are in the free form, they can be converted into salts in a known manner. When they are salts, on the other hand, they can be converted into free compounds or other salts.

As shown in Examples which will be described later, the compounds of the present invention have a squalene synthetase inhibitory effect and cholesterol synthesis inhibitory effect so that they are useful as a drug for preventing and/or treating diseases in mammals including humans such as hyperlipemia, e.g., hypercholesterolemia, low HDL cholesterolemia, arteriosclerosis and metabolic syndrome. Described specifically, they are useful as a drug for preventing and/or treating arteriosclerosis, ischemic diseases, myocardial infarction, angina pectoris, heart failure, aneurysm, cerebral arteriosclerosis, stroke, transient cerebral ischemic attack, cerebral infarction, peripheral arteriosclerosis, intermittent claudication, thrombosis, hypertension, osteoporosis, diabetes, diabetes complication, pancreatic disorder, restenosis after percutaneous transluminal coronary angioplasty (PTCA) or stent implantation, nephritis or nephropathy.

When each of the compounds of the present invention, salts thereof, or solvates of them is used as a drug, an appropriate dose of it to a patient may be determined after due consideration of the sex, age, and symptoms of the patient, kind of the drug, amount of the drug to be administered, administration route, dose frequency, timing of dose, and the like. The daily dose to an adult patient is typically from 0.1 mg to 1 g, preferably from 0.5 mg to 500 mg, but is not limited to it. The daily dose may be administered once or in from two to four divided portions.

Each of the compounds of the present invention, salts thereof, or solvates of them may be formulated in a known manner after addition of a pharmaceutically acceptable carrier thereto. Oral dosage forms include tablets, powders, granules, capsules, and liquids. Parenteral dosage forms include injections and suppositories. Drug additives may be added as needed without impairing the advantage of the present invention. Examples of the drug additives include excipients, lubricants, and fluidizers.
Examples of the excipient include crystalline cellulose, powder cellulose, corn starch, potato starch, silicon dioxide, precipitated calcium carbonate, anhydrous calcium hydrogen phosphate, magnesium oxide, calcium lactate, calcium silicate, synthetic hydrotalcite, lactose, sucrose, mannitol, erythritol, trehalose, glucose, and fructose.
Examples of the lubricant include magnesium stearate, calcium stearate, sucrose fatty acid ester, glycerin fatty acid ester, polyethylene glycol, hydrogenated oil, and sodium stearyl fumarate.
Examples of the fluidizer include hydrous silicon dioxide, light silicic anhydride, synthetic aluminum silicate, titanium oxide, heavy silicic anhydride, aluminum magnesium hydroxide, tricalcium phosphate, talc, magnesium aluminometasilicate, and granulated calcium hydrogen phosphate. These drug additives may be used either singly or in combination.
The compounds of the present invention may each be used in combination with or as a combination of one or more drugs selected from drugs for hyperlipemia such as atorvastatin calcium hydrate, ethyl icosapentate, elastase, gamma-oryzanol, clinofibrate, clofibrate, colestimide, cholestyramine, simvastatin, soysterol, dextran sulfate sodium sulfur, nicomol, niceritrol, pitavastatin calcium, fenofibrate, pravastatin sodium, fluvastatin sodium, probucol, bezafibrate, polyenephosphatidylcholine, riboflavin butyrate, and rosuvastatin calcium; antihypertensives such as azelnidipine, atenolol, amosulalol hydrochloride, alacepril, aranidipine, imidapril hydrochloride, indapamide, uradipil, esidri, enalapril maleate, efonidipine hydrochloride, olmesartan medoxomil, cadralazine, captopril, carteolol hydrochloride, carvedilol, candesartan cilexetil, quinapril hydrochloride, guanabenz acetate, guanfacine hydrochloride, clonidine hydrochloride, cilazapril, cilnidipine, celiprolol hydrochloride, tilisolol hydrochloride, temocapril hydrochloride, delapril hydrochloride, telmisartan, doxazosin mesilate, todralazine hydrochloride, trandolapril, tripamide, nadolol, nicardipine hydrochloride, nifedipine, nipradilol, nilvadipine, valsartan, barnidipine hydrochloride, hydralazine hydrochloride, hydrochlorothiazide, pindolol, felodipine, budralazine, bunazosin hydrochloride, bunitrolol hydrochloride, prazosin hydrochloride, furosemide, propranolol hydrochloride, betaxolol hydrochloride, benazepril hydrochloride, bevantolol hydrochloride, perindopril erbumine, penbutolol sulfate, bopindolol malonate, manidipine hydrochloride, meticrane, metoprolol tartrate, labetalol hydrochloride, lisinopril, and losartan potassium; and diabetic drugs such as acarbose, acetohexamide, gliclazide, glybuzole, glimepiride, chlorpropamide, tolbutamide, nateglinide, pioglitazone hydrochloride, voglibose, mitiglinide calcium hydrate, and metformin hydrochloride; and antiplatelet agents such as aspirin, ticlopidine hydrochloride, cilostazol, and clopidogrel sulfate.

The present invention will hereinafter be described by Examples. It should however be borne in mind that the present invention is not limited only to them.

### [Examples]

The ¹H-NMR spectrum is measured using a JNM-EX-400 (400MHz) spectrometer manufactured by JEOL while using tetramethylsilane as an internal standard and all the δ values are given in ppm. Values shown with a solvent mixture are volumetric mixing ratios of the solvents unless otherwise specifically indicated. In Examples, "%" is on a weight basis unless otherwise specifically indicated. A ratio of eluting solvents in silica gel column chromatography is a volumetric ratio unless otherwise specifically indicated. The term "room temperature" (normal temperature) as used herein means a temperature from about 20°C to 30°C.

### Referential Example 1

### (5-Chloro-2-(1H-pyrrol-1-yl)phenyl)(2,3-dimethoxyphenyl)methanone

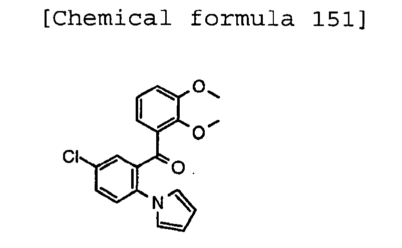

(2-Amino-5-chlorophenyl)(2,3-dimethoxyphenyl)methanone (2.91 g) was dissolved in acetic acid (60 mL). To the resulting solution was added 2,5-dimethoxytetrahydrofuran (2.07 mL) and the resulting mixture was heated under reflux for 30 minutes. After cooling the reaction mixture to room temperature, it was concentrated. The concentrate was diluted with ethyl acetate and washed with a saturated aqueous solution of sodium bicarbonate and saturated brine. The organic layer was dried over anhydrous sodium sulfate. The filtrate was concentrated. The residue thus obtained was purified by silica gel column (ethyl acetate:hexane=1:20) to give the title compound (1.80 g).
MS(ESI)m/z:342(M⁺+H).
¹H-NMR(CDCl₃)δ:3.52(3H,s),3.83(3H,s),6.06(2H,t,J=2.2Hz),6.73(2 H,t,J=2.2Hz);6.97(2H,d,J=5.1Hz),7.02-7.03(1H,m),7.30-7.32(1H,m),7.48-7.51(2H,m).

### Referential Example 2

### 1-(4-Chloro-2-(2,3-dimethoxybenzoyl)phenyl)-1H-pyrrolo-2-carbaldehyde

### 1-(4-Chloro-2-(2,3-dimethoxybenzoyl)phenyl)-1H-pyrrolo-3-carbaldehyde

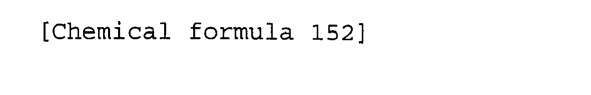

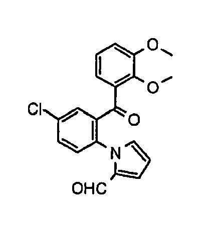

To N,N-dimethylformamide (1.71 mL) was added phosphorus oxychloride (1.62 mL) under ice cooling and the resulting mixture was stirred at room temperature for 10 minutes. A solution of (5-chloro-2-(1H-pyrrol-1-yl)phenyl)(2,3-dimethoxyphenyl)methanone (1.80 g) in dichloroethane (40 mL) was added dropwise to the reaction mixture. After warming to 50°C, the reaction mixture was stirred under heat for 2 hours. An aqueous solution (40 mL) of sodium acetate trihydrate (3.94 g) was added to the reaction mixture, followed by heating under reflux for 30 minutes. After cooling to room temperature, the reaction mixture was extracted with chloroform and then washed with saturated brine. The organic layer was dried over anhydrous magnesium sulfate. The filtrate was concentrated. The residue thus obtained was purified by silica gel column (ethyl acetate:hexane=1:8) to give the title compound (2-aldehyde) (1.19 g) and the title compound (3-aldehyde) (0.52 g).
2-Aldehyde compound
MS(ESI)m/z:370(M⁺+H).
¹H-NMR(CDCl₃)δ:3.62(3H,s),3.83(3H,s),6.19(1H,dd,J=3.9,2.4Hz),6 .84-6.91(2H,m),6.92-6.97(3H,m),7.29(1H,d,J=8.3Hz),7.53(1H,dd,J=8.3,2.4Hz),7.59( 1H,d,J=2.4Hz),9.39(1H,s).
3-Aldehyde compound
MS(ESI)m/z:370(M⁺+H).
¹H-NMR(CDCl₃)δ:3.49(3H,s),3.83(3H,s),6.51-6.51(1H,m),6.74-6.75(1H,m),6.98-7.02(2H,m),7.04-7.07(1H,m),7.32-7.35(2H,m),7.54-7.57(2H,m),9.66(1H,s).

### Referential Example 3

### Methyl (E)-3-(1-(4-chloro-2-(2,3-dimethoxybenzoyl)phenyl)-1H-pyrrol-2-yl)-2-propenoate

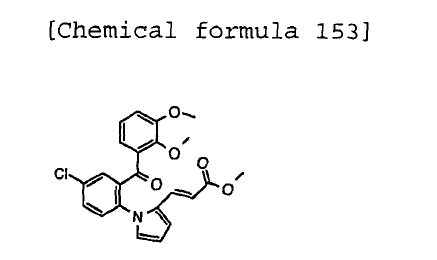

1-(4-Chloro-2-(2,3-dimethoxybenzoyl)phenyl)-1H-pyrrolo-2-carbaldehyde (1.19 g) was dissolved in toluene (20 mL). To the resulting solution was added methoxycarbonylmethylene triphenylphosphorane (2.15 g) and the mixture was heated and stirred overnight at 100°C. The reaction mixture was cooled to room temperature and then concentrated. The residue thus obtained was purified using a silica gel column (ethyl acetate:hexane=1:9) to give the title compound (1.20 g).
¹H-NMR(CDCl₃)δ:3.55(3H,s),3.72(3H,s),3.80(3H,s),5.92(1H,d,J=15 .9Hz),6.07-6.10(1H,m),6.49-6.52(1H,m),6.78-6.80(1H,m),6.89-6.85(1H,m),6.92-6.94(2H,m),7.19(1H,d,J=15.9Hz),7.24(1H,d,J=8.3Hz),7.55(1H,d d,J=8.3,2.4Hz),7.60(1H,d,J=2.4Hz).

### Referential Example 4

### Methyl (E)-3-(1-(4-chloro-2-((2,3-dimethoxyphenyl)(hydroxy)methyl)phenyl)-1H-pyrrol-2-yl)-2-propenoate

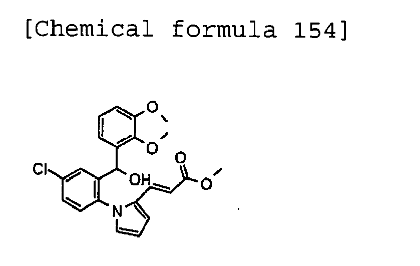

Methyl (E)-3-(1-(4-chloro-2-(2,3-dimethoxybenzoyl)phenyl)-1H-pyrrol-2-yl)-2-propenoate (1.20 g) was dissolved in methanol (30 mL). To the resulting solution was added sodium borohydride (160 mg), followed by stirring at room temperature for 1 hour. Water was added to the reaction mixture and then the mixture was extracted with ethyl acetate. The organic layer was dried over anhydrous sodium sulfate. The filtrate was concentrated to give the title compound (1.25 g).
MS(ESI)m/z:428(M⁺+H).
¹H-NMR(CDCl₃)δ:3.50(1.5H,s),3.53(1.5H,s),3.64(1.5H,s),3.70(1.5 H,s),3.78(1.5H,s),3.83(1.5H,s),5.65-5.71(1.0H,m),5.72(0.5H,d,J=6.1Hz),5.92(0.5H,d,J=15.9Hz),6.1 8(0.5H,t,J=3.3Hz),6.36(0.5H,t,J=3.3Hz),6.42-6.45(0.5H,m),6.46-6.48(0.5H,m),6.62-6.66(1.0H,m),6.70-6.77(1.5H,m),6.84-6.84(0.5H,m),6.86-6.86(0.5H,m),6.93-6.95(0.5H,m),6.96-7.01(0.5H,m),7.11(0.5H,d,J=8.3Hz),7.15(0.5H,d,J=8.3Hz),7.23 (0.5H,d,J=15.9Hz),7.36-7.39(1.0H,m),7.67(0.5H,d,J=2.4Hz),7.71(0.5H,d,J=2.4Hz).

### Example 1

### Methyl 2-(8-chloro-6-(2,3-dimethoxyphenyl)-4H,6H-pyrrolo[1,2-a][4,1]benzoxazepin-4-yl)acetate

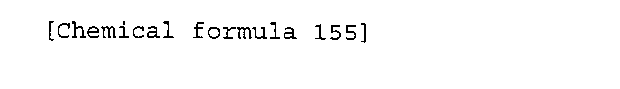

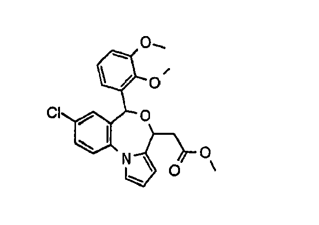

Methyl (E)-3-(1-(4-chloro-2-((2,3-dimethoxyphenyl)(hydroxy)methyl)phenyl)-1H-pyrrol-2-yl)-2-propenoate (1.25 g) was dissolved in methylene chloride (30 mL). To the resulting solution was added trifluoroacetic acid (0.270 mL) and the mixture was stirred overnight at room temperature. The reaction mixture was diluted with chloroform and then washed with a saturated aqueous solution of sodium bicarbonate and saturated brine. The organic layer was dried over anhydrous sodium sulfate. The filtrate was concentrated. The residue thus obtained was purified by silica gel column (ethyl acetate:hexane=1:15) to give the title compound (627 mg).
MS(ESI)m/z:462(M⁺+H).
¹H-NMR(CDCl₃)δ:3.02(1H,dd,J=15.0,6.0Hz),3.10(1H,dd,J=15.0,8.2H z),3.44(3H,s),3.71(3H,s),3.85(3H,s),4.90(1H,dd,J=8.2,6.0Hz) ,5.71(1H,s),6.28-6.29(1H,m),6.37(1H,t,J=3.2Hz),6.72(1H,d,J=2.0Hz),6.94(1H,dd ,J=8.0,1.5Hz),7.09-7.12(1H,m),7.16(1H,t,J=8.0Hz),7.23-7.27(1H,m),7.35-7.37(2H,m).

### Example 2

### Methyl 2-((4R,6S)-8-chloro-6-(2,3-dimethoxyphenyl)-4H,6H-pyrrolo[1,2-a][4,1]benzoxazepin-4-yl)acetate (isomer A) Methyl 2-((4S,6R)-8-chloro-6-(2,3-dimethoxyphenyl)-4H,6H-pyrrolo[1,2-a][4,1]benzoxazepin-4-yl)acetate (isomer B)

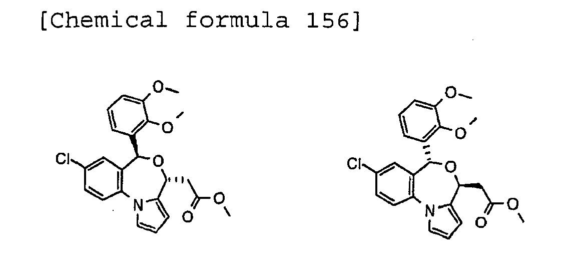

Methyl 2-(8-Chloro-6-(2,3-dimethoxyphenyl)-4H,6H-pyrrolo[1,2-a][4,1]benzoxazepin-4-yl)acetate (12.0 g) was isolated and purified by HPL [CHIRALCEL OD(Daicel, 50ϕ × 500 mm), eluting solvent hexane:isopropanol=50:50, dissolution rate: 50 mL/min], whereby the isomer A (5.9 g) was obtained from the fraction with a retention time of 23 minutes and the isomer B (5.9 g) was obtained from the fraction with a retention time of 36 minutes, respectively.

### Example 3

### 2-(8-Chloro-6-(2,3-dimethoxyphenyl)-4H,6H-pyrrolo[1,2-a][4,1]benzoxazepin-4-yl)acetic acid

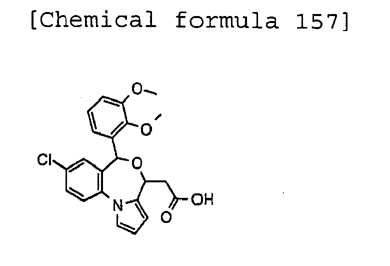

Methyl 2-(8-chloro-6-(2,3-dimethoxyphenyl)-4H,6H-pyrrolo[1,2-a][4,1]benzoxazepin-4-yl)acetate (142 mg) was dissolved in methanol-water-tetrahydrofuran (2:1:0.5, 3.5 mL). To the resulting mixture was added potassium carbonate (206 mg), followed by stirring overnight at room temperature. After addition of acetic acid (0.0855 mL), the resulting mixture was diluted with chloroform and washed with water. The organic layer was dried over anhydrous magnesium sulfate. The filtrate was concentrated. The residue thus obtained was purified by preparative TLC (methanol:chloroform=1:10) to give the title compound (80.6 mg).
MS(ESI)m/z:414(M⁺+H).
¹H-NMR(CDCl₃)δ:2.98-3.12(2H,m),3.44(3H,s),3.84(3H,s),4.82-4.90(1H,m),5.72(1H,s),6.24-6.37(2H,m),6.71-6.75(1H,m),6.92(1H,d,J=8.1Hz),7.08-7.10(1H,m),7.14(1H,t,J=8.1Hz),7.25-7.28(1H,m),7.33-7.36 (2H,m) .

### Example 4

Ethyl 2-(1-(2-(8-chloro-6-(2,3-dimethoxyphenyl)-4H,6H-pyrrolo[1,2-a] [4,1]benzoxazepin-4-yl)acetyl)-4-piperidinyl)acetate

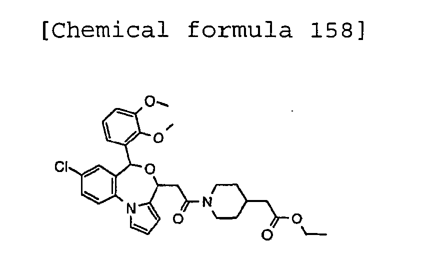

2-(8-Chloro-6-(2,3-dimethoxyphenyl)-4H,6H-pyrrolo[1,2-a][4,1]benzoxazepin-4-yl)acetic acid (80.6 mg) and ethyl 2-(4-piperidinyl)acetate (35.8 mg) were dissolved in methylene chloride (3 mL). To the resulting solution were added 1-ethyl-3-(3-dimethylaminopropyl)carbodiimide hydrochloride (43.7 mg) and 1-hydroxybenzotriazole (8.7 mg), followed by stirring at room temperature for 3 hours. The reaction mixture was diluted with chloroform and washed with a saturated aqueous solution of sodium bicarbonate and saturated brine. The organic layer was dried over anhydrous sodium sulfate. The filtrate was concentrated. The residue thus obtained was purified by preparative TLC (methanol:chloroform=1:100) to give the title compound (82.6 mg).
MS(ESI)m/z:567(M⁺+H).
¹H-NMR(CDCl₃)δ:1.06-1.23(2H,m),1.26(3H,t,J=7.1Hz),1.72-1.80(2H,m),1.95-2.06(1H,m),2.11-2.17(1H,m),2.22-2.27(1H,m),2.54-2.70(1H,m),2.79-2.91(1H,m),2.95-3.13(1H,m),3.22-3.30(1H,m),3.42(3H,s),3.85(3H,s),4.01-4.07(1H,m),4.09-4.17(2H,m),4.61-4.73(1H,m),4.91-5.01(1H,m),5.72-5.75(1H,m),6.23-6.25(1H,m),6.35-6.37(1H,m),6.67-6.71(1H,m),6.92-6.96(1H,m),7.08-7.10(1H,m),7.13-7.19(1H,m),7.23-7.31(1H,m),7.33-7.37(2H,m).

### Example 5

### 2-(1-(2-(8-Chloro-6-(2,3-dimethoxyphenyl)-4H,6H-pyrrolo[1,2-a][4,1]benzoxazepin-4-yl)acetyl)-4-piperidinyl)acetic acid

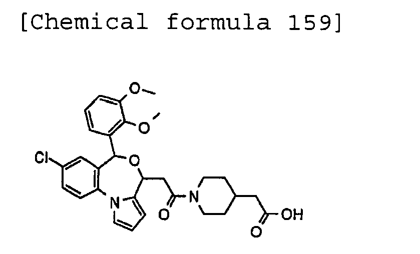

Ethyl 2-(1-(2-(8-chloro-6-(2,3-dimethoxyphenyl)-4H,6H-pyrrolo[1,2-a][4,1]benzoxazepin-4-yl)acetyl)-4-piperidinyl)acetate (82.6 mg) was dissolved in methanol-water-tetrahydrofuran (2:1:0.5, 3.5 mL). To the resulting solution was added potassium carbonate (90.6 mg), followed by stirring overnight at room temperature. The temperature was raised to 45°C and stirring was continued for further 7 hours. To the reaction mixture was added acetic acid (0.0375 mL) and the mixture was diluted with chloroform and washed with saturated brine. The organic layer was dried over anhydrous magnesium sulfate. The filtrate was then concentrated. The residue thus obtained was purified by preparative TLC (methanol:chloroform=1:10) to give the title compound (63.6 mg).
MS(ESI)m/z:539(M⁺+H).
¹H-NMR(CDCl₃)δ:1.01-1.24(2H,m),1.59-1.82(2H,m),1.93-2.02(1H,m),2.13-2.15(1H,m),2.24-2.28(1H,m),2.53-2.69(1H,m),2.79-2.91(1H,m),2.95-3.12(1H,m),3.23-3.32(1H,m),3.41(3H,s),3.85(3H,s),4.02-4.06(1H,m),4.62-4.73(1H,m),4.90-4.99(1H,m),5.72-5.76(1H,m),6.24(1H,br s),6.36(1H,t,J=3.2Hz),6.68-6.70(1H,m),6.92-6.95(1H,m),7.08-7.10(1H,m),7.15(1H,t,J=7.9Hz),7.21-7.36(3H,m).

### Referential Example 5

### 5-Chloro-3-iodo-2-(1H-pyrrol-1-yl)pyridine

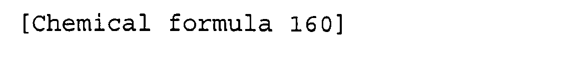

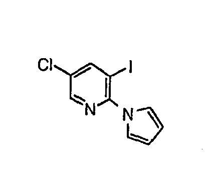

5-Chloro-3-iodo-2-amino-pyridine (3.50 g) was dissolved in 1,4-dioxane (70 mL). To the resulting solution were added 2,5-dimethoxytetrahydrofuran (2.31 g) and 4-chloropyridine hydrochloride (2.89 g). The resulting mixture was heated under reflux for 1 hour. After the reaction mixture was cooled to room temperature, it was concentrated. The concentrate was diluted with water and then extracted with ethyl acetate. The organic layer was dried over anhydrous sodium sulfate. The filtrate was then concentrated. The residue thus obtained was purified by silica gel column (ethyl acetate:hexane=1:10) to give the title compound (4.14 g). ¹H-NMR(CDCl₃)δ:6.34(2H,t,J=2.1Hz)7.18(2H,t,J=2.1Hz)8.26(1H,d ,J=2.4Hz),8.39(1H,d,J=2.4Hz).

### Referential Example 6

### [5-Chloro-2-(1H-pyrrol-1-yl)-3-pyridinyl](2,3-dimethoxyphenyl)methanol

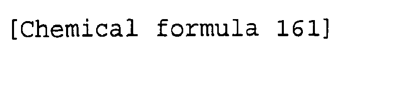

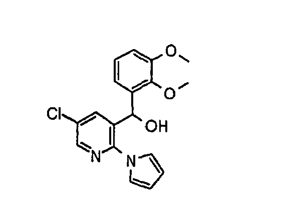

5-Chloro-3-iodo-2-(1H-pyrrol-1-yl)pyridine (1.50 g) was dissolved in tetrahydrofuran (30 mL). A 0.68M isopropyl magnesium bromide tetrahydrofuran solution (8.69 mL) was added dropwise to the resulting solution under a nitrogen atmosphere while stirring at -40°C for 30 minutes. A tetrahydrofuran solution (10 mL) of 2,3-dimethoxybenzaldehyde (1.06 g) was added at -40°C. The temperature was raised to room temperature and stirring was performed for 3.5 hours. A saturated aqueous solution of ammonium chloride was added and the resulting mixture was extracted with ethyl acetate. The organic layer was dried over anhydrous sodium sulfate. The filtrate was then concentrated. The residue thus obtained was purified by silica gel column (ethyl acetate:hexane=1:9) to give the title compound (1.18 g).
MS(ESI)m/z:327(M⁺-OH).
¹H-NMR(CDCl₃)δ:3.00-3.01(1H,m),3.65(3H,s),3.86(3H,s),6.18(1H,d,J=3.9Hz),6.28(2H ,t,J=2.2Hz),6.71-6.74(1H,m),6.91(1H,dd,J=8.3,1.5Hz),7.01(2H,t,J=2.2Hz),7.04( 1H,t,J=8.3Hz),7.90(1H,d,J=2.7Hz),8.38(1H,d,J=2.7Hz).

### Referential Example 7

### [5-Chloro-2-(1H-pyrrol-1-yl)-3-pyridinyl](2,3-dimethoxyphenyl)methanone

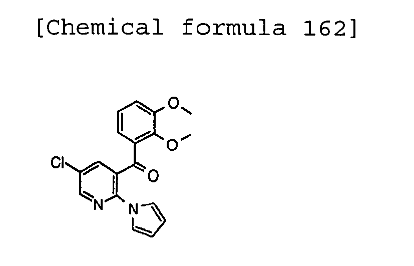

[5-Chloro-2-(1H-pyrrol-1-yl)-3-pyridinyl](2,3-dimethoxyphenyl)methanol (1.45 g) was dissolved in dichloromethane (30 mL). To the resulting solution was added manganese dioxide (7.14 g). The resulting mixture was stirred overnight at room temperature. After the reaction mixture was cooled to room temperature, it was concentrated, diluted with water and extracted with ethyl acetate. After manganese dioxide was filtered out, the filtrate was concentrated to give the title compound (1.45 g).
¹H-NMR(CDCl₃)δ:3.52(3H,s),3.82(3H,s),6.11(2H,t,J=2.3Hz),7.02-7.04(4H,m),7.16-7.19(1H,m),7.80(1H,d,J=2.7Hz),8.50(1H,d,J=2.7Hz).

### Referential Example 8

### 1-[5-Chloro-3-(2,3-dimethoxybenzoyl)-2-pyridinyl]-1H-pyrrolo-2-carbaldehyde

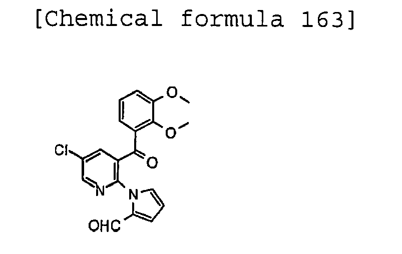

To N,N-dimethylformamide (1.38 mL) was added phosphorus oxychloride (1.30 mL) under ice cooling. The reaction mixture was stirred at room temperature for 10 minutes. A solution of [5-chloro-2-(1H-pyrrol-1-yl)-3-pyridinyl](2,3-dimethoxyphenyl)methanone (1.45 g) in dichloroethane (20 mL) was added dropwise to the reaction mixture under ice cooling. The temperature was raised to 50°C and stirring was performed for 1.5 hours under heat. To the reaction mixture was added an aqueous solution (20 mL) of sodium acetate trihydrate (3.20 g), followed by heating under reflux for 1 hour. After cooling to room temperature, the reaction mixture was extracted with chloroform and the extract was washed with saturated brine.
The organic layer was dried over anhydrous sodium sulfate. The filtrate was then concentrated. The residue thus obtained was purified by silica gel column (ethyl acetate:hexane=1:6-4) to give the title compound (1.50 g).
MS(ESI)m/z:371(M⁺+H).
¹H-NMR(CDCl₃)δ:3.58(3H,s),3.82(3H,s),6.18(1H,dd,J=3.9,2.7Hz),6 .83(1H,dd,J=3.9,1.7Hz),6.93-6.99(3H,m),7.10-7.10(1H,m),8.02(1H,d,J=2.4Hz),8.58(1H,d,J=2.4Hz),9.40(1H,d, J=0.7Hz) .

### Referential Example 9

### Methyl (E)-3-(1-[5-chloro-3-(2,3-dimethoxybenzoyl)-2-pyridinyl]-1H-pyrrol-2-yl)-propenoate

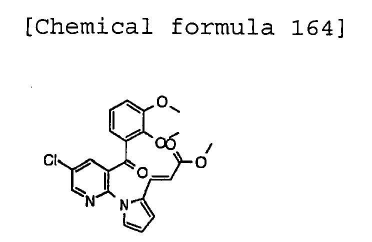

1-[5-Chloro-3-(2,3-dimethoxybenzoyl)-2-pyridinyl]-1H-pyrrolo-2-carbaldehyde (920 mg) was dissolved in toluene (20 mL). To the resulting solution was added methoxycarbonylmethylene triphenylphosphorane (1.24 g).
The resulting mixture was heated under reflux overnight. After cooling to room temperature, the reaction mixture was concentrated. The residue thus obtained was purified by silica gel column (ethyl acetate:hexane=1:9) to give the title compound (1.05 g).
MS(ESI)m/z:426(M⁺+H).
¹H-NMR(CDCl₃)δ:3.52(3H,s),3.74(3H,s),3.78(3H,s),5.96(1H,d,J=15 .6Hz),6.04-6.06(1H,m),6.48-6.50(1H,m),6.84-6.85(1H,m),6.93-6.98(3H,m),7.38(1H,d,J=15.6Hz),8.00(1H,d,J=2.7Hz),8.64(1H,d ,J=2.7Hz).

### Referential Example 10

### Methyl (E)-3-(1-{5-chloro-3-[(2,3-dimethoxybenzoyl)(hydroxy)methyl]-2-pyridinyl}-1H-pyrrol-2-yl)-propenoate

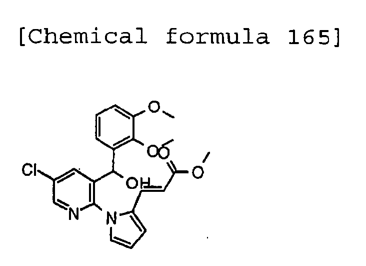

Methyl (E)-3-(1-[5-chloro-3-(2,3-dimethoxybenzoyl)-2-pyridinyl]-1H-pyrrol-2-yl)-propenoate (1.05 g) was suspended in methanol (20 mL). To the resulting suspension was added sodium borohydride (140 mg) and the mixture was stirred at room temperature for 1 hour. Water was added to the reaction mixture, followed by extraction with ethyl acetate. The organic layer was dried over anhydrous sodium sulfate. The filtrate was then concentrated to give the title compound (1.08 g).
MS(ESI)m/z:429(M⁺+H).
¹H-NMR(CDCl₃)δ:2.81(1H,d,J=5.1Hz),3.54(3H,s),3.69(3H,s),3.81(3 H,s),5.79(1H,d,J=5.1Hz),5.83(1H,d,J=15.9Hz),6.28-6.32(1H,m),6.55-6.59(1H,m),6.72-6.74(1H,m),6.82-6.85(2H,m),6.95(1H,t,J=8.1Hz),7.09(1H,d,J=15.9Hz),8.06(1H,d ,J=2.4Hz),8.48(1H,d,J=2.4Hz).

### Example 6

### Methyl 2-[3-chloro-5-(2,3-dimethoxyphenyl)-5H,7H-pyrido[2,3-e]-pyrrolo[2,1-c][1,4]oxazepin-7-yl]acetate

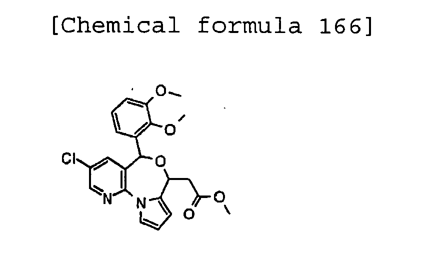

Methyl (E)-3-(1-{5-chloro-3-[(2,3-dimethoxybenzoyl)(hydroxy)methyl]-2-pyridinyl}-1H-pyrrol-2-yl)-propenoate (1.21 g) was dissolved in dichloroethane (20 mL). To the resulting solution was added trifluoroacetic acid (0.523 mL) and the mixture was stirred overnight at room temperature. A saturated aqueous solution of sodium bicarbonate was added and the mixture was extracted with chloroform. The organic layer was dried over anhydrous sodium sulfate. The filtrate was then concentrated. The residue thus obtained was purified by preparative TLC (ethyl acetate:hexane=1:7) to give the title compound (690 mg).
¹H-NMR(CDCl₃)δ:3.04(1H,dd,J=15.1,5.7Hz),3.11(1H,dd,J=15.1,8.2H z),3.50(3H,s),3.72(3H,s),3.86(3H,s),4.97(1H,dd,J=8.2,5.7Hz) ,5.72(1H,s),6.31-6.32(1H,m),6.39(1H,t,J=3.3Hz),6.95-6.98(1H,m),7.04-7.05(1H,m),7.18(1H,t,J=7.9Hz),7.23-7.25(1H,m),7.49-7.50(1H,m),8.38(1H,d,J=2.4Hz).

### Example 7

### 2-[3-chloro-5-(2,3-dimethoxyphenyl)-5H,7H-pyrido[2,3-e]-pyrrolo[2,1-c][1,4]oxazepin-7-yl]aceticacid

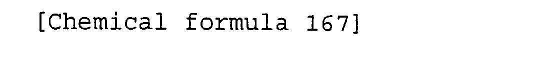

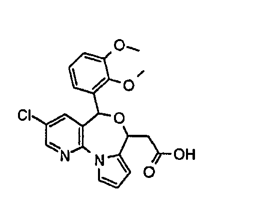

Methyl 2-[3-chloro-5-(2,3-dimethoxyphenyl)-5H,7H-pyrido[2,3-e]-pyrrolo[2,1-c][1,4]oxazepin-7-yl]acetate (205 mg) was dissolved in methanol-water-tetrahydrofuran (2:1:2, 5 mL). To the resulting solution was added potassium carbonate (198 mg), followed by stirring overnight at room temperature. After addition of acetic acid (0.0821 mL), the resulting mixture was diluted with chloroform and washed with water. The organic layer was dried over anhydrous magnesium sulfate. The filtrate was then concentrated. The residue thus obtained was purified by preparative TLC (methanol:chloroform=1:10) to give the title compound (109 mg).
MS(ESI)m/z:415(M⁺+H).
¹H-NMR(CDCl₃)δ:2.99-3.13(2H,m)3.49(3H,s)3.85(3H,s)4.91-4.96(1H,m),5.74(1H,s),6.20-6.22(1H,m),6.33-6.35(1H,m),6.36-6.38(1H,m),6.93-6.95(1H,m),7.05-7.06(1H,m),7.14-7.16(1H,m),7.23-7.25(1H,m),7.48-7.51(1H,m),8.36-8.38(1H,m).

### Example 8

### Ethyl 2-(1-(2-[3-chloro-5-(2,3-dimethoxyphenyl)-5H,7H-pyrido[2,3-e]-pyrrolo[2,1-c][1,4]oxazeopin-7-yl]acetyl}-4-piperidinyl)acetate

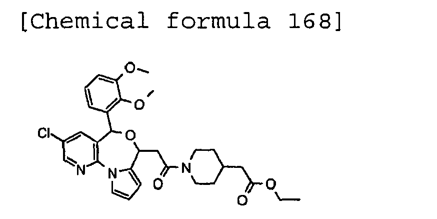

2-[3-Chloro-5-(2,3-dimethoxyphenyl)-5H,7H-pyrido[2,3-e]-pyrrolo[2,1-c][1,4]oxazepin-7-yl]acetic acid (187 mg) and ethyl 2-(4-piperidinyl)acetate (84.9 mg) were dissolved in dichloromethane (5 mL). To the resulting solution were added 1-ethyl-3-(3-dimethylaminopropyl)carbodiimide hydrochloride (104 mg) and 1-hydroxybenzotriazole (20.7 mg). The resulting mixture was stirred at room temperature for 3 hours. The reaction mixture was diluted with chloroform and washed over a saturated aqueous solution of sodium bicarbonate and saturated brine. The organic layer was dried over anhydrous sodium sulfate. The filtrate was then concentrated. The residue thus obtained was purified by preparative TLC (ethyl acetate:hexane=1:1) to give the title compound (259 mg).
MS (ESI) m/z : 568 (M⁺+H).
¹H-NMR(CDCl₃) δ:1.04-1.22 (2H,m), 1.23-1.28 (3H,m), 1.76-1.78(2H,m),2.01-2.04(1H,m),2.10-2.15(1H,m),2.23-2.27(1H,m),2.55-2.69(1H,m),2.79-2.90(1H,m),2.96-3.13(1H,m),3.25-3.34(1H,m),3.48-3.48(3H,m),3.86-3.87(3H,m),4.02-4.04(1H,m),4.09-4.17(2H,m),4.62-4.73(1H,m),4.99-5.08(1H,m),5.73-5.77(1H,m),6.27-6.30(1H,m),6.37-6.39(1H,m),6.95-6.97(1H,m),7.00-7.03(1H,m),7.16-7.18(1H,m),7.22-7.30(1H,m),7.48-7.51(1H,m),8.35-8.37(1H,m).

### Example 9

### Ethyl 2-(1-{2-[(5S,7R)-3-chloro-5-(2,3-dimethoxyphenyl)-5H,7H-pyrido[2,3-e]-pyrrolo[2,1-c][1,4]oxazeopin-7-yl]acetyl}-4-piperidinyl)acetate (isomer A) Ethyl 2-(1-{2-[(5R,7S)-3-chloro-5-(2,3-dimethoxyphenyl)-5H,7H-pyrido[2,3-e]-pyrrolo[2,1-c][1,4]oxazeopin-7-yl]acetyl}-4-piperidinyl)acetate (Isomer B)

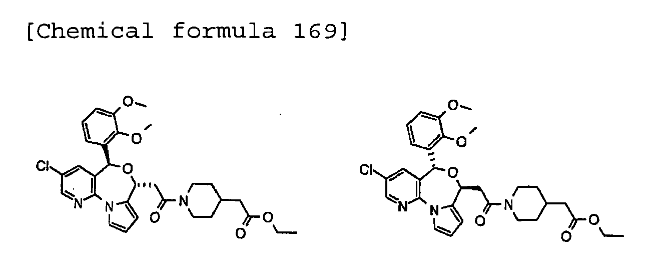

Ethyl 2-(1-{2-[3-chloro-5-(2,3-dimethoxyphenyl)-5H,7H-pyrido[2,3-e]-pyrrolo[2,1-c][1,4]oxazeopin-7-yl]acetyl}-4-piperidinyl)acetate (256 mg) was resolved optically by an optically active column (CHIRALCEL-OD) to give the isomer A (85.5 mg) and the isomer B (105 mg).
Flow rate: 15 mL/min
Developing solvent: 20% 2-propanol-n-hexane
Retention time: isomer A: 13 min., isomer B: 17 min.

### Example 10

### 2-(1-{2-[(5S,7R)-3-Chloro-5-(2,3-dimethoxyphenyl)-5H,7H-pyrido[2,3-e]-pyrrolo[2,1-c][1,4]oxazeopin-7-yl]acetyl}-4-piperidinyl)acetic acid

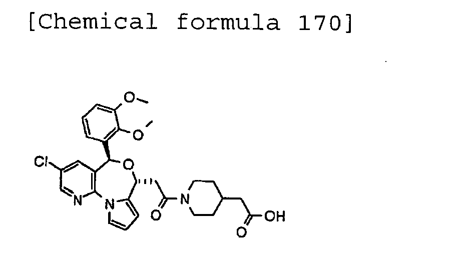

Ethyl 2-(1-{2-[(5S,7R)-3-chloro-5-(2,3-dimethoxyphenyl)-5H,7H-pyrido[2,3-e]-pyrrolo[2,1-c][1,4]oxazeopin-7-yl]acetyl}-4-piperidinyl)acetate (85.5 mg) was dissolved in methanol-water-tetrahydrofuran (2:1:2, 5mL). To the resulting solution was added potassium carbonate (62.4 mg) and the mixture was stirred overnight at 50°C. After addition of acetic acid (0.0259 mL), the resulting mixture was diluted with chloroform and washed with saturated brine. The organic layer was dried over anhydrous magnesium sulfate. The filtrate was then concentrated. The residue thus obtained was purified by preparative TLC (methanol:chloroform=1:10) to give the title compound (68.3 mg).
MS(ESI)m/z:540(M⁺+H).
¹H-NMR(CDCl₃)δ:0.79-0.89(1H,m)0.97-1.10(1H,m),1.16-1.29(2H,m),1.93-2.05(1H,m),2.12-2.17(1H,m),2.26-2.29(1H,m),2.54-2.69(1H,m),2.79-2.90(1H,m),3.01-3.11(1H,m),3.25-3.34(1H,m),3.46-3.50(3H,m),3.86(3H,s),4.02-4.05(1H,m),4.63-4.73(1H,m),4.99-5.06(1H,m),5.73-5.78(1H,m),6.27-6.29(1H,m),6.37-6.39(1H,m),6.94-6.96(1H,m),7.00-7.02(1H,m),7.15-7.17(1H,m),7.22-7.31(1H,m),7.48-7.50(1H,m),8.35-8.37(1H,m).

### Example 11

### 2-(1-{2-[(5R,7S)-3-Chloro-5-(2,3-dimethoxyphenyl)-5H,7H-pyrido[2,3-e]-pyrrolo[2,1-c][1,4]oxazeopin-7-yl]acetyl}-4-piperidinyl)acetic acid

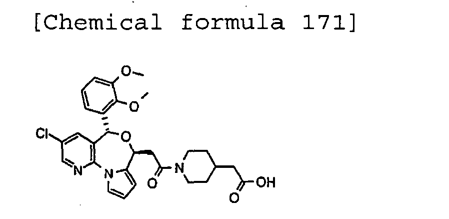

Ethyl 2-(1-{2-[(5R,7S)-3-chloro-5-(2,3-dimethoxyphenyl)-5H,7H-pyrido[2,3-e]-pyrrolo[2,1-c][1,4]oxazeopin-7-yl]acetyl}-4-piperidinyl)acetate (105 mg) was treated in a similar manner to that described above by using potassium carbonate (76.4 mg) to give the title compound (88.7 mg).
MS(ESI)m/z:540(M⁺+H)
¹H-NMR(CDCl₃)δ:0.79-0.89(1H,m),0.97-1.10(1H,m),1.16-1.29(2H,m),1.93-2.05(1H,m),2.12-2.17(1H,m),2.26-2.29(1H,m),2.54-2.69(1H,m),2.79-2.90(1H,m),3.01-3.11(1H,m),3.25-3.34(1H,m),3.46-3.50(3H,m),3.86(3H,s),4.02-4.05(1H,m),4.63-4.73(1H,m),4.99-5.06(1H,m),5.73-5.78(1H,m),6.27-6.29(1H,m),6.37-6.39(1H,m),6.94-6.96(1H,m),7.00-7.02(1H,m),7.15-7.17(1H,m),7.22-7.31(1H,m),7.48-7.50(1H,m),8.35-8.37(1H,m).

### Example 12

### Ethyl 4-[8-chloro-6-(2,3-dimethoxyphenyl)-4H,6H-pyrrolo[1,2-a][4,1]benzoxazepin-4-yl]-3-hydroxybutanoate

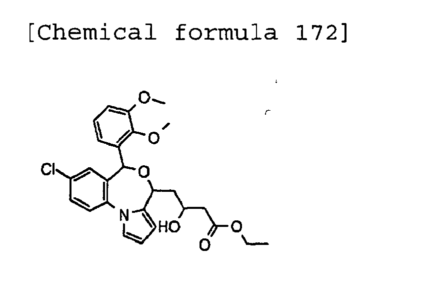

Ethyl acetate (0.225 mL) was dissolved in tetrahydrofuran (3 mL). To the resulting solution was added 1.8M lithium diisopropylamide (0.383 mL) under a nitrogen atmosphere at -78°C while stirring for 15 minutes. A tetrahydrofuran solution (2 mL) of 2-[8-chloro-6-(2,3-dimethoxyphenyl)-4H,6H-pyrrolo[1,2-a][4,1]benzoxazepin-4-yl]acetaldehyde (183 mg) was added dropwise, followed by stirring at the same temperature for 30 minutes. To the reaction mixture was added a saturated aqueous solution of ammonium chloride and the mixture was extracted with ethyl acetate. The organic layer was dried over anhydrous magnesium sulfate. The filtrate was then concentrated.
The residue thus obtained was purified by preparative TLC (ethyl acetate:hexane=1:3) to give the title compounds, that is, the isomer A (55.4 mg) and the isomer B (52.0 mg). Isomer A (low polarity fraction)
MS (ESI)m/z:486.7 (M⁺+H).
¹H-NMR(CDCl₃)δ:1.28(3H,t,J=7.1Hz),2.10-2.17(1H,m),2.24-2.31(1H,m),2.54(1H,dd,J=16.4,8.5Hz),2.62(1H,dd,J=16.4,3.9Hz ),3.29(1H,d,J=4.4Hz),3.44(3H,s),3.85(3H,s),4.18(2H,q,J=7.3H z),4.50-4.57(1H,m),4.70-4.73(1H,m),5.74(1H,s),6.31-6.33(1H,m),6.37(1H,t,J=3.2Hz),6.72(1H,d,J=2.0Hz),6.94-6.96(1H,m),7.08-7.10(1H,m),7.19(1H,t,J=8.1Hz),7.30-7.33(1H,m),7.34-7.38(2H,m).
Isomer B (high polarity fraction)
MS(ESI)m/z:486.7(M⁺+H).
¹H-NMR(CDCl₃)δ:1.26(3H,t,J=7,1Hz),2,16-2.21(1H,m),2.30-2.40(1H,m),2.51-2.62(2H,m),3.43(3H,s),3.59(1H,d,J=2.2Hz),3.81(1H,d,J=1.7Hz) ,3.85(3H,s),4.15(2H,q,J=7.1Hz),4.36-4.44(1H,m),4.64-4.68(1H,m),5.74(1H,s),6.31-6.32(1H,m),6.38(1H,t,J=3.3Hz),6.74(1H,d,J=2.0Hz),6.93-6.95(1H,m),7.09-7.10(1H,m),7.18(1H,t,J=7.9Hz),7.23-7.26(1H,m),7.34-7.38(2H,m).

### Example 13

### 4-[8-Chloro-6-(2,3-dimethoxyphenyl)-4H,6H-pyrrolo[1,2-a][4,1]benzoxazepin-4-yl]-3-hydroxybutanoic acid

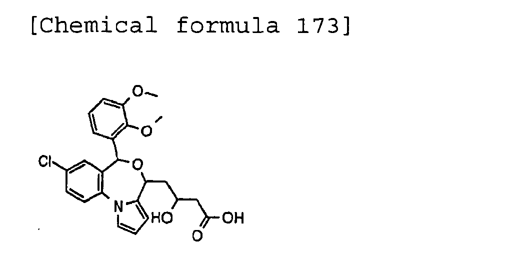

Ethyl 4-[8-chloro-6-(2,3-dimethoxyphenyl)-4H,6H-pyrrolo[1,2-a][4,1]benzoxazepin-4-yl]-3-hydroxybutanoate (isomer A, 55.4 mg) was dissolved in ethanol (2 mL). To the resulting solution was added a 1N aqueous solution (0.125mL) of sodium hydroxide, followed by stirring overnight at room temperature. After addition of a 1N aqueous solution (0.125 mL) of hydrochloric acid, the resulting mixture was diluted with water and extracted with ethyl acetate. The organic layer was dried over anhydrous sodium sulfate. The filtrate was then concentrated. The residue thus obtained was purified by preparative TLC (methanol:chloroform=1:10) to give the title compound (19.8 mg) .
MS (ESI) m/z: 458 (M⁺+H) .
¹H-NMR(CDCl₃)δ:1.95-2.05(1H,m),2.11-2.19(1H,m),2.33-2.46(2H,m),3.38(3H,s),3.77(3H,s),4.34-4.41(1H,m),4.58-4.61(1H,m),5.68(1H,s),6.21-6.23(1H,m),6.28-6.30(1H,m),6.67-6.69(1H,m),6.81-6.83(1H,m),7.00-7.03(1H,m),7.07-7.11(1H,m),7.23-7.26(3H,m).

### Example 14

### 4-[8-Chloro-6-(2,3-dimethoxyphenyl)-4H,6H-pyrrolo[1,2-a][4,1]benzoxazepin-4-yl]-3-hydroxybutanoic acid

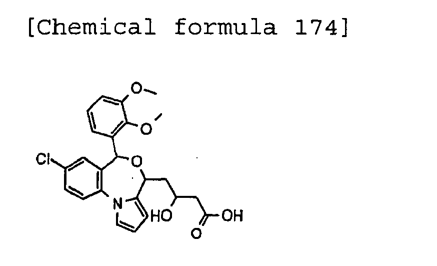

Ethyl 4-[8-chloro-6-(2,3-dimethoxyphenyl)-4H,6H-pyrrolo[1,2-a][4,1]benzoxazepin-4-yl]-3-hydroxybutanoate (isomer B, 52.0 mg) was treated in a similar manner to that described above by using a
1N aqueous solution (0.118 mL) of sodium hydroxide to give the title compound (24.0 mg).
MS(ESI)m/z:458(M⁺+H).
¹H-NMR(CDCl₃)δ:2.00-2.07(1H,m),2.23-2.33(1H,m),2.40-2.54(2H,m),3.40(3H,s),3.80(3H,s),4.27-4.35(1H,m),4.56-4.61(1H,m),5.70-5.72(1H,m),6.24-6.27(1H,m),6.32-6.34(1H,m),6.69-6.72(1H,m),6.87-6.89(1H,m),7.03-7.06(1H,m),7.16-7.20(2H,m),7.28-7.30(2H,m).

### Referential Example 11 1-(2-Bromo-4-chloro-6-fluorophenyl)-1H-pyrrole

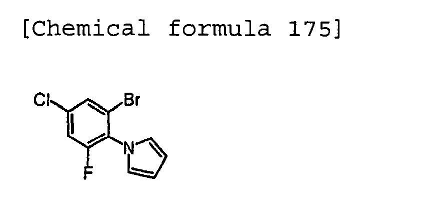

2-Bromo-4-chloro-6-fluoroaniline (5.00 g) was dissolved in acetic acid (25 mL). To the resulting solution was added 2,5-dimethoxytetrahydrofuran (3.11 mL). The resulting mixture was stirred at 60°C for 1 hour. The solvent was distilled off under reduced pressure. Ethyl acetate was added to the residue, followed by washing with a saturated aqueous solution of sodium bicarbonate and saturated brine. After drying over anhydrous sodium sulfate, the solvent was distilled off under reduced pressure. The residue was purified by silica gel column chromatography (hexane:ethyl acetate=20:1) to give the title compound (6.01 g).
¹H-NMR(CDCl₃)δ:6.37-6.38(2H,m),6.73-6.74(2H,m),7.23(1H,dd,J=8.79,4.39Hz),7.51-7.52(1H,m).

### Referential Example 12

### (5-Chloro-3-fluoro-2-pyrrol-1-ylphenyl)-(2,3-dimethoxyphenyl)methanol

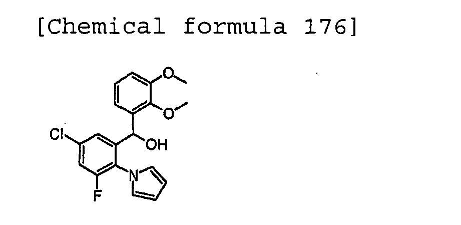

1-(2-Bromo-4-chloro-6-fluorophenyl)-1H-pyrrole (1.84 g) was dissolved in ether (30 mL). Under a nitrogen atmosphere, n-butyl lithium (1.6M n-hexane solution, 4.60 mL) was added dropwise to the resulting solution at -78°C. After stirring at 0°C for 15 minutes, the reaction mixture was cooled to -78°C. A solution of 2,3-dimethoxybenzaldehyde (1.22 g) in ether (5 mL)-tetrahydrofuran (2 mL) was added dropwise. The temperature was elevated to 0°C and stirring was performed for 1 hour. A saturated aqueous solution of ammonium chloride was added and the resulting mixture was extracted with ethyl acetate. The extract was washed with saturated brine and dried over anhydrous sodium sulfate. The solvent was then distilled off under reduced pressure. The residue thus obtained was purified by silica gel column chromatography (hexane:ethyl acetate) to give the title compound (2.19 g).
¹H-NMR(CDCl₃)δ:2.67(1H,dd,J=4.52,1.34Hz),3.58(3H,s),3.85(3H,s) ,5.85(1H,d,J=4.64Hz),6.27(2H,s),6.60-6.66(3H,m),6.88(1H,dd,J=8.18,1.1OHz),7.01(1H,t,J=8.06Hz),7. 18(1H,dd,J=9.03,2.44Hz),7.36(1H,s).

### Referential Example 13

### (5-Chloro-3-fluoro-2-pyrrol-1-ylphenyl)-(2,3-dimethoxyphenyl)methanone

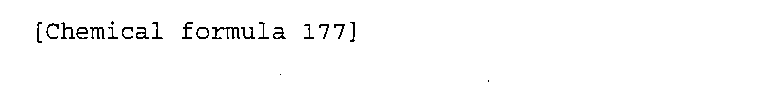

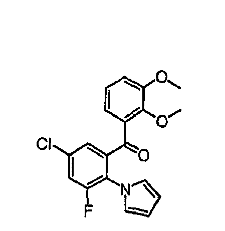

(5-Chloro-3-fluoro-2-pyrrol-1-ylphenyl)-(2,3-dimethoxyphenyl)methanol (2.19 g) was dissolved in dichloromethane (40 mL). To the resulting solution was added magnesium oxide (5.15 g) at room temperature and the resulting mixture was heated under reflux for 13 hours. Magnesium oxide (5.15 g) was added and the resulting mixture was heated under reflux for 1 hour. The reaction mixture was allowed to cool and then filtered. The solvent was then distilled off to give the title compound (1.78 g).
¹H-NMR(CDCl₃)δ:3.53(3H,d,J=0.98Hz)3.83(3H,d,J=0.98Hz) 6.06-6.07(2H,m),6.66(2H,m),6.98-7.02(3H,m),7.32-7.35(2H,m).

### Referential Example 14

### 1-[4-Chloro-2-(2,3-dimethoxybenzoyl)-6-fluorophenyl]-1H-pyrrolo-2-carbaldehyde

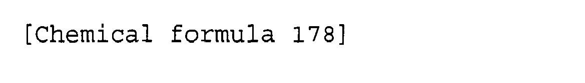

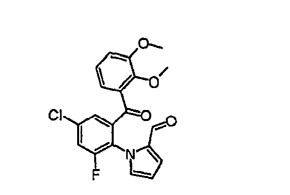

While stirring dimethylformamide (330 µl) under ice cooling, phosphorus oxychloride (397 µl) was added thereto in portions. After the temperature was returned to room temperature, stirring was performed for 15 minutes. After ice cooling again, a solution of (5-chloro-3-fluoro-2-pyrrol-1-ylphenyl)-(2,3-dimethoxyphenyl)methanone (1.39 g) in dimethylformamide (5 mL) was added dropwise over 5 minutes and the mixture was stirred at 50°C for 2 hours. The reaction mixture was allowed to cool and then, an aqueous solution (5 mL) of sodium acetate trihydrate (1.58 g) was added and the resulting mixture was stirred at 50°C for 30 minutes. The reaction mixture was then allowed to cool, followed by extraction with chloroform and drying over anhydrous sodium sulfate. The solvent was distilled off under reduced pressure. The residue was purified by silica gel column chromatography (hexane:ethyl acetate=19:1) to give the title compound (882 mg). In addition, 1-[4-chloro-2-(2,3-dimethoxybenzoyl)-6-fluorophenyl]-1H-pyrrolo-3-carbaldehyde (160 mg) was obtained.
¹H-NMR(CDCl₃)δ:3.65(3H,s),3.84(3H,s),6.25-6.26(1H,m),6.85-6.99(5H,m),7.34-7.37(2H,m),9.41(1H,m).

### Referential Example 15

### Methyl 3-{1-[4-chloro-2-(2,3-dimethoxybenzoyl)-6-fluorophenyl]-1H-pyrrol-2-yl}-acrylate

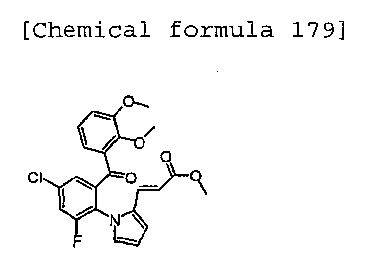

1-[4-Chloro-2-(2,3-dimethoxybenzoyl)-6-fluorophenyl]-1H-pyrrolo-2-carbaldehyde (722 mg) was dissolved in toluene (15 mL). To the resulting solution was added triphenylphosphoranylidene (1.25 g) and the resulting mixture was heated under reflux for 1 hour. The solvent was distilled off under reduced pressure. The residue was purified by silica gel column chromatography (hexane:ethyl acetate=10:1) to give the title compound (808 mg).
¹H-NMR (CDCl₃) δ:3.57 (3H, s),3.72(3H,s),3.80(3H, s),5.93(1H, d, J=15 .62Hz),6.14-6.15(1H,m),6.54-6.54(1H,m),6.73-6.75(1H,m),6.89-6.93(3H,m),7.13(1H,d,J=15.62Hz),7.38-7.40(2H,m).

### Referential Example 16

Methyl 3-(1-{4-chloro-2-[(2,3-dimethoxyphenyl)hydroxymethyl]-6-fluorophenyl}-1H-pyrrol-2-yl)-acrylate

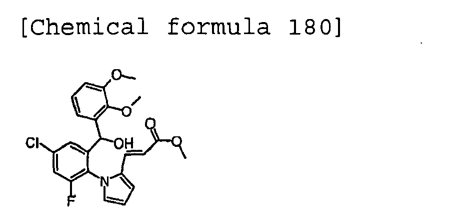

Methyl 3-{1-[4-chloro-2-(2,3-dimethoxybenzoyl)-6-fluorophenyl]-1H-pyrrol-2-yl}-acrylate (808 mg) was dissolved in methanol (20 mL). Under ice cooling, sodium borohydride (103 mg) was added and the resulting mixture was stirred at room temperature for 1.5 hours. Under ice cooling, water was added and the mixture was extracted with ethyl acetate. The extract was washed with saturated brine and dried over anhydrous sodium sulfate. The solvent was then distilled off to give the title compound (822 mg).

### Example 15

### Methyl 2-[8-chloro-6-(2,3-dimethoxyphenyl)-10-fluoro-4H,6H-pyrrolo[1,2-a] [4,1]benzoxazepin-4-yl]acetate

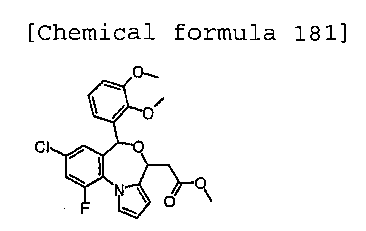

Methyl 3-(1-{4-chloro-2-[(2,3-dimethoxyphenyl)hydroxymethyl]-6-fluorophenyl}-1H-pyrrol-2-yl)-acrylate (821 mg) was dissolved in dichloromethane (20 mL). Under ice cooling, trifluoroacetic acid (156 µl) was added and the resulting mixture was stirred for 20 hours. The reaction mixture was washed with a saturated aqueous solution of sodium bicarbonate and then dried over anhydrous sodium sulfate. The solvent was distilled off under reduced pressure. The residue was purified by silica gel column chromatography (hexane:ethyl acetate) to give the title compound (357 mg).
¹H-NMR(CDCl₃)δ:3.07(2H,ddd,J=32.35,15.14,6.96Hz),3.45(3H,s),3. 72(3H,s),3.84(3H,s),4.87-4.94(1H,m),5.58(1H,s),6.30-6.34(1H,m),6.38(1H,t,J=3.30Hz),6.51-6.55(1H,m),6.92-6.96(1H,m),7.14-7.29(4H,m).

### Example 16

### Methyl 2-[(4R,6S)-8-chloro-6-(2,3,-dimethoxyphenyl)-10-fluoro-4H,6H-pyrrolo[1,2-a][4,1]benzoxazepin-4-yl]acetate

### Methyl 2-[(4S,6R)-8-chloro-6-(2,3-dimethoxyphenyl)-10-fluoro-4H,6H-pyrrolo[1,2-a][4,1]benzoxazepin-4-yl]acetate

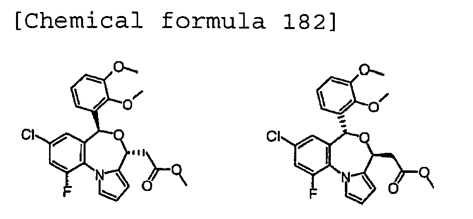

Methyl 2-[8-chloro-6-(2,3-dimethoxyphenyl)-10-fluoro-4H,6H-pyrrolo[1,2-a][4,1]benzoxazepin-4-yl]acetate (17.35 g) was isolated and purified by HPLC [CHIRALCEL OD (Daicel, 50ϕ × 500mm), eluting solvent: hexane:isopropanol=50:50, dissolution rate: 50 mL/min]. The isomer A was obtained from the fraction with a retention time of 23 minutes, while the isomer B was obtained from the fraction with a retention time of 30 minutes.

### Example 17

### 2-[8-Chloro-6-(2,3-dimethoxyphenyl)-10-fluoro-4H,6H-pyrrolo[1,2-a] [4,1]benzoxazepin-4-yl]acetic acid

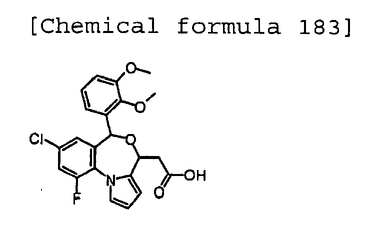

Methyl 2-[8-chloro-6-(2,3-dimethoxyphenyl)-10-fluoro-4H,6H-pyrrolo[1,2-a][4,1]benzoxazepin-4-yl]acetate (1.21 g) was dissolved in a tetrahydrofuran-methanol-water (1:1:2) solvent mixture (40 mL). To the resulting solution was added potassium carbonate (1.50 g) and the mixture was stirred at 55°C for 4 hours. Under ice cooling, 1N hydrochloric acid was added to make the reaction mixture weakly acidic. After extraction with ethyl acetate, the extract was washed with saturated brine and dried over anhydrous sodium sulfate. The solvent was distilled off under reduced pressure. The residue was purified by silica gel column chromatography (chloroform:methanol) to give the title compound (534 mg).
¹H-NMR(CDCl₃)δ:1.56(1H,br s),3.06-3.19(2H,m),3.47(3H,s),3.85(3H,s),4.90(1H,dd,J=7.81,5.62Hz), 5.63(1H,s),6.35-6.41(2H,m),6.54-6.57(1H,m),6.95(1H,dd,J=7.93,1.59Hz),7.14-7.28(4H,m).

### Example 18

### Ethyl 2-(1-{2-[(4S,6R)-8-chloro-6-(2,3-dimethoxyphenyl)-10-fluoro-4H,6H-pyrrolo[1,2-a][4,1]benzoxazepin-4-yl]acetyl}-4-piperidinyl)acetate (isomer A)

### Ethyl 2-(1-{2-[(4R,6S)-8-chloro-6-(2,3-dimethoxyphenyl)-10-fluoro-4H,6H-pyrrolo[1,2-a][4,1]benzoxazepin-4-yl]acetyl}-4-piperidinyl)acetate (isomer B)

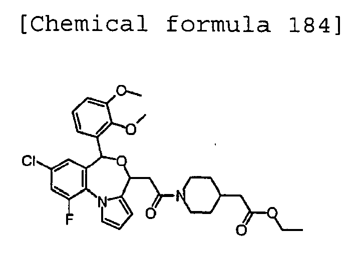

2-[8-Chloro-6-(2,3-dimethoxyphenyl)-10-fluoro-4H,6H-pyrrolo[1,2-a][4,1]benzoxazepin-4-yl]acetic acid (100 mg) was dissolved in dichloromethane (3 mL). To the resulting solution were added ethyl piperidine acetate (48 µl), 1-ethyl-3-(3-dimethylaminopropyl)carbodiimide hydrochloride (53 mg), and 1-hydroxybenzotriazole (11 mg). The resulting mixture was stirred at room temperature for 13 hours. Ethyl acetate was added. The resulting mixture was washed with 1N hydrochloric acid, a saturated aqueous solution of sodium bicarbonate, and saturated brine and then, dried over anhydrous sodium sulfate. The solvent was distilled off under reduced pressure. The residue was purified by silica gel column chromatography (chloroform-methanol) to give 158 mg of a racemic compound. By optical resolution by HPLC (CHIRALPAK AD, 20% isopropanol-hexane, 15 mL/min), the isomer A (41.7 mg) was obtained from the fraction with a retention time of 8 minutes, while the isomer B (39.0 mg) was obtained from the fraction with a retention time of 32 minutes, respectively.
¹H-NMR(CDCl₃)δ:1.26(3H,t,J=7.08Hz),1.78(2H,d,J=12.21Hz),2.29-1.93(3H,m),2.70-2.55(1H,m),2.91-2.79(1H,m),3.14-2.97(1H,m),3.32-3.23(1H,m),3.44(3H,s),3.85(3H,s),4.04(1H,d,J=12.94Hz),4.13(
2H,ddd,J=14.22,7.14,2.99Hz),4.74-4.61(1H,m),4.97(1H,dq,J=19.84,4.44Hz),5.61(1H,d,J=12.94Hz), 6.25-6.28(1H,m),6.38(1H,t,J=3.30Hz),6.50(1H,d,J=8.06Hz),6.94(1H, d,J=8.06Hz),7.12-7.23(3H,m),7.25-7.30(1H,m).

### Example 19

### 2-(1-{2-[(4R,6S)-8-Chloro-6-(2,3-dimethoxyphenyl)-10-fluoro-4H,6H-pyrrolo[1,2-a][4,1]benzoxazepin-4-yl]acetyl}-4-piperidinyl)acetic acid

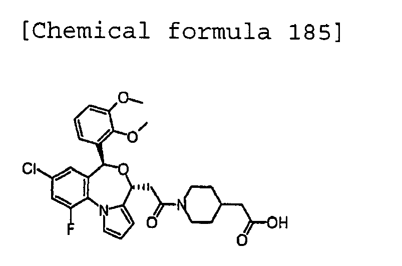

Ethyl 2-(1-{2-[(4S,6R)-8-chloro-6-(2,3-dimethoxyphenyl)-10-fluoro-4H,6H-pyrrolo[1,2-a] [4,1]benzoxazepin-4-yl]acetyl}-4-piperidinyl)acetate (39.0 mg) was dissolved in a tetrahydrofuran-methanol-water (1:1:2) solvent mixture (4 mL). To the resulting solution was added potassium carbonate (37 mg), followed by stirring at 55°C for 14 hours. Under ice cooling, 1N hydrochloric acid was added to make the reaction mixture weakly acidic. After extraction with ethyl acetate, the extract was washed with saturated brine and dried over anhydrous sodium sulfate. The solvent was then distilled off under reduced pressure. Ethyl acetate-hexane was added to the residue and the solid thus formed was collected by filtration to give the title compound (31.5 mg).
¹H-NMR(CDCl₃)δ:1.07-1.86(5H,m),2.02(1H,br s),2.20(1H,t,J=7.20Hz),2.28-2.33(1H,m),2.55-2.71(1H,m),2.85(1H,ddd,J=23.68,14.28,4.52Hz),2.97-3.14(1H,m),3.28(1H,dt,J=16.76,7.14Hz),3.44(3H,s),3.85(3H,s) ,4.05(1H,d,J=11.96Hz),4.70(1H,t,J=15.26Hz),4.97(1H,dq,J=20. 20,4.35Hz),5.62(1H,d,J=15.14Hz),6.28-6.26(1H,m),6.38(1H,t,J=3.30Hz),6.50(1H,d,J=8.79Hz),6.94(1H, d,J=7.81Hz),7.12-7.25(3H,m).
Elemental analysis for C₂₉H₃₀ClFN₂O₆
Calculated: C,62.53;H,5.43;N,5.03.
Found: C,62.57;H,5.71;N,4.76.

### Referential Example 17

### 2-[(4R,6S)-8-Chloro-6-(2,3-dimethoxyphenyl)-10-fluoro-4H,6H-pyrrolo[1,2-a][4,1]benzoxazepin-4-yl]-1-ethanol

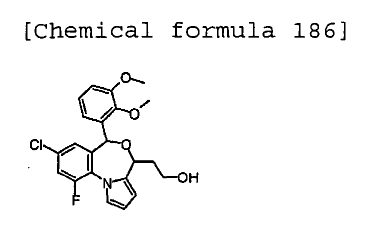

Methyl 2-[8-chloro-6-(2,3-dimethoxyphenyl)-10-fluoro-4H,6H-pyrrolo[1,2-a][4,1]benzoxazepin-4-yl]acetate (356 mg) was dissolved in tetrahydrofuran (10 mL). Under ice cooling, lithium aluminum hydride (49.5 mg) was added to the resulting solution and the resulting mixture was stirred at the same temperature for 1 hour. Under ice cooling, water (100 mL), a 15% aqueous solution (100 mL) of sodium hydroxide, and water (300 mL) were added successively. Magnesium sulfate was then added to dry the resulting mixture. After filtration through celite, the solvent was distilled off under reduced pressure to give the title compound (282 mg).
¹H-NMR(CDCl₃)δ:2.19-2.28(2H,m),2.37-2.47(1H,m),3.47(3H,s),3.85(3H,s),3.96(2H,q,J=5.70Hz),4.64(1 H,dd,J=9.77,3.91Hz),5.63(1H,s),6.37-6.35(1H,m),6.40(1H,t,J=3.30Hz),6.54-6.55(1H,m),6.95(1H,dd,J=8.18,1.59Hz),7.16-7.29(5H,m).

### Referential Example 18

### 2-[(4R,6S)-8-Chloro-6-(2,3-dimethoxyphenyl)-10-fluoro-4H,6H-pyrrolo[1,2-a] [4,1]benzoxazepin-4-yl]ethylmethanesulfonate

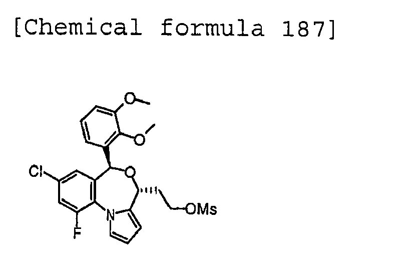

2-[(4R,6S)-8-Chloro-6-(2,3-dimethoxyphenyl)-10-fluoro-4H,6H-pyrrolo[1,2-a][4,1]benzoxazepin-4-yl]-1-ethanol (281 mg) was dissolved in dichloromethane (5 mL). Under ice cooling, triethylamine (132 µl) and methanesulfonyl chloride (58.5 µl) were added. The resulting mixture was stirred at the same temperature for 1 hour. The reaction mixture was washed with water and a saturated aqueous solution of sodium bicarbonate and then dried over anhydrous sodium sulfate. The solvent was distilled off under reduced pressure to give the title compound (328.5 mg).
¹H-NMR(CDCl₃)δ:2.57-2.43(2H,m),2.98(3H,s),3.45(3H,s),3.67(1H,s),3.85(3H,s),4.50 -4.60(2H,m),5.60(1H,s),6.31-6.33(1H,m),6.40(1H,t,J=3.17Hz),6.53(1H,brs),6.93-7.00(1H,m),7.13-7.32(5H,m).

### Example 20

### Ethyl 2-(2-{2-[(4R,6S)-8-chloro-6-(2,3-dimethoxyphenyl)-10-fluoro-4H,6H-pyrrolo[1,2-a] [4,1]benzoxazepin-4-yl]ethyl}-2H-1,2,3,4-tetrazol-5-yl)acetate (isomer A)

### Ethyl 2-(2-{2-[(4R,6S)-8-chloro-6-(2,3-dimethoxyphenyl)-10-fluoro-4H,6H-pyrrolo[1,2-a][4,1]benzoxazepin-4-yl]ethyl}-1H-1,2,3,4-tetrazol-5-yl)acetate (isomer B)

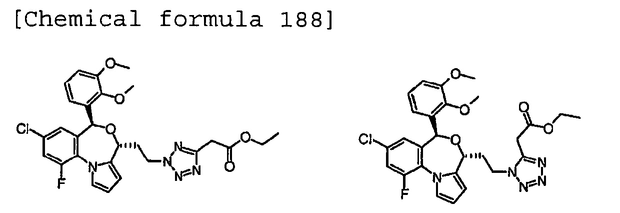

2-[(4R,6S)-8-Chloro-6-(2,3-dimethoxyphenyl)-10-fluoro-4H, 6H-pyrrolo [1, 2-a] [4,1]benzoxazepin-4-yl]ethylmethanesulfonate (493 mg) was dissolved in dimethylformamide (5 mL). To the resulting solution were added ethyl 2-(1H-1,2,3,4-tetrazol-5-yl)acetate (310 mg), potassium carbonate (412 mg), and tetrabutylammonium iodide (367 mg) and the resulting mixture was stirred at 80°C for 2 hours. After the reaction mixture was allowed to cool, ethyl acetate was added. The resulting mixture was washed with water and dried over anhydrous sodium sulfate. The solvent was distilled off under reduced pressure. The residue was purified by silica gel column chromatography (hexane:ethyl acetate), whereby the title compound A (143 mg) and the title compound B (66.1 mg) were obtained. The title compound A was subjected to optical resolution using HPLC (CHIRALPAK AD (Daicel, 20ϕ × 250 mm), 50% isopropanol-hexane, 23 mL/min), whereby the isomer A (51.6 mg) was obtained from the fraction with a retention time of 4 minutes and the isomer B (46.4 mg) was obtained from the fraction with a retention time of 14 minutes, respectively.
Isomer A
¹H-NMR(CDCl₃)δ:1.25(3H,dd,J=13.67,6.59Hz),2.70-2.76(2H,m),3.48(3H,s),3.84(3H,s),3.99(2H,d,J=6.35Hz),4.10-4.21(2H,m),4.42(1H,q,J=4.39Hz),4.59-4.66(2H,m),5.63(1H,s),6.32(1H,t,J=9.16Hz),6.40(1H,t,J=3.30H z),6.54(1H,s),6.99-6.95(1H,m),7.16-7.27(5H,m).
Isomer B
¹H-NMR(CDCl₃)δ:1.24 (3H,q,J=5.53Hz), 2.74-2.81(2H,m),3.46(3H,s),3.86(3H,s),3.93(2H,s),4.00-4.06(1H,m),4.19(2H,q,J=7.16Hz),4.44(1H,q,J=4.56Hz),4.93(2H, t,J=7.45Hz),5.63(1H,s),6.35(1H,d,J=3.66Hz),6.40(1H,t,J=3.30 Hz),6.53(1H,s),6.97(1H,d,J=8.06Hz),7.18-7.32(3H,m).

### Example 21

### 2-(2-{2-[(4R,6S)-8-Chloro-6-(2,3-dimethoxyphenyl)-10-fluoro-4H,6H-pyrrolo[1,2-a][4,1]benzoxazepin-4-yl]ethyl}-2H-1,2,3,4-tetrazol-5-yl)acetic acid

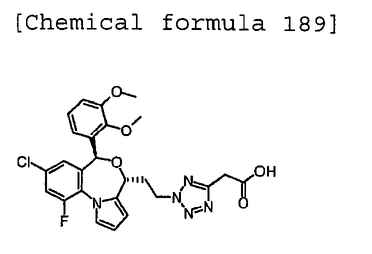

Ethyl 2-(2-{2-[(4R,6S)-8-chloro-6-(2,3-dimethoxyphenyl)-10-fluoro-4H,6H-pyrrolo[1,2-a][4,1]benzoxazepin-4-yl]ethyl}-2H-1,2,3,4-tetrazol-5-yl)acetate (46.0 mg) was treated with potassium carbonate (46.0 mg) to give the title compound (30.5 mg).
¹H-NMR(CDCl₃)δ:2.74-2.81(2H,m),3.46(3H,s),3.86(3H,s),3.99(2H,s),4.43(1H,q,J=4.5 6Hz),4.93(2H,t,J=7.08Hz),5.63(1H,s),6.36-6.34(1H,m),6.39(1H,t,J=3.30Hz),6.52-6.54(1H,m),6.95-6.98(1H,m),7.17-7.32(4H,m).

### Referential Example 19

### 3-[(4R,6S)-8-Chloro-6-(2,3-dimethoxyphenyl)-10-fluoro-4H,6H-pyrrolo[1,2-a][4,1]benzoxazepin-4-yl]propanenitrile

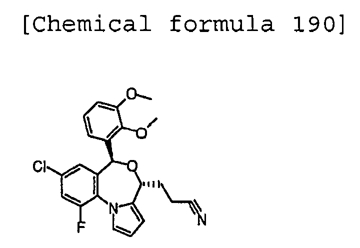

2-[(4R,6S)-8-Chloro-6-(2,3-dimethoxyphenyl)-10-fluoro-4H,6H-pyrrolo[1,2-a][4,1]benzoxazepin-4-yl]ethylmethanesulfonate (932.2 mg) was dissolved in dimethylsulfoxide (10 mL). To the resulting solution was added sodium cyanide (190 mg) and the resulting mixture was stirred at 50°C for 13 hours. After the reaction mixture was allowed to cool, water was added, followed by extraction with ethyl acetate. The extract was dried over anhydrous sodium sulfate. The solvent was then distilled off under reduced pressure. The residue was purified by silica gel column chromatography (hexane:ethyl acetate) to give the title compound (704.7 mg).
¹H-NMR(CDCl₃)δ:2.51-2.30(2H,m),2.68(2H,t,J=7.32Hz),3.45(3H,s),3.86(3H,s),4.50(1 H,dd,J=9.64,4.03Hz),5.61(1H,s),6.29-6.32(1H,m),6.40(1H,t,J=3.30Hz),6.54(1H,br s),6.94-7.00(1H,m),7.17-7.35(4H,m).

### Example 22

### 3-[(4R,6S)-8-Chloro-6-(2,3-dimethoxyphenyl)-10-fluoro-4H,6H-pyrrolo[1,2-a][4,1]benzoxazepin-4-yl]propionic acid

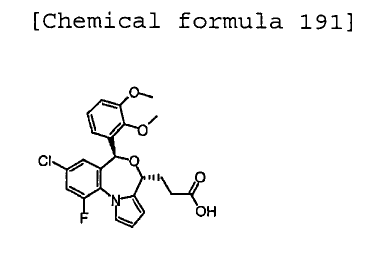

3-[(4R,6S)-8-Chloro-6-(2,3-dimethoxyphenyl)-10-fluoro-4H,6H-pyrrolo[1,2-a][4,1]benzoxazepin-4-yl]propanenitrile (704 mg) was dissolved in 2-isopropanol (7.5 mL). To the resulting solution was added a 5N aqueous solution (7.5 mL) of sodium hydroxide and the resulting mixture was refluxed for 24 hours. Under ice cooling, 1N hydrochloric acid was added to make the reaction mixture acidic. The reaction mixture was extracted with ethyl acetate. The extract was washed with saturated brine and dried over anhydrous sodium sulfate. The solvent was then distilled off under reduced pressure to give the title compound (749.5 mg).
¹H-NMR(CDCl₃)δ:2.30-2.78(4H,m),3.45(3H,s),3.84(3H,s), 4.44-4.39(1H,m),5.59(1H,s),6.35-6.40(2H,m),6.52(1H,s),6.97(1H,t,J=12.33Hz),7.21-7.26(4H,m).

### Example 23

### Ethyl 2-(1-{3-[(4R,6S)-8-chloro-6-(2,3-dimethoxyphenyl)-10-fluoro-4H,6H-pyrrolo[1,2-a][4,1]benzoxazepin-4-yl]propanoyl}-4-piperidinyl)acetate

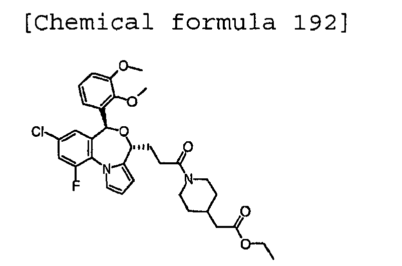

3-[(4R,6S)-8-Chloro-6-(2,3-dimethoxyphenyl)-10-fluoro-4H,6H-pyrrolo[1,2-a][4,1]benzoxazepin-4-yl]propionic acid (100 mg) was dissolved in dichloromethane (5 mL). To the resulting solution were added ethyl piperidine acetate (58 µl), 1-hydroxybenzotriazole (34.3 mg), and 1-ethyl-3-(3-dimethylaminopropyl)carbodiimide hydrochloride (64.4 mg). The resulting mixture was stirred at room temperature for 12 hours. After addition of ethyl acetate, the resulting mixture was washed with 1N hydrochloric acid, a saturated aqueous solution of sodium bicarbonate, and water and then, dried over anhydrous sodium sulfate. The solvent was then distilled off under reduced pressure. The residue was purified by silica gel column chromatography (hexane:ethyl acetate) to give the title compound (58.2 mg).
¹H-NMR(CDCl3)δ:1.04-1.20(2H,m),1.26(3H,t,J=7.08Hz),1.48-1.62(2H,m),1.49-1.62(2H,m),1.69-1.81(2H,m),1.94-2.08(1H,m),2.15-2.29(2H,m),2.35-2.46(2H,m),2.97-3.07(1H,m),3.45(3H,d,J=2.20Hz),3.85(3H,d,J=1.95Hz),4.14(2H, q,J=6.59Hz),4.38-4.44(1H,m),4.56-4.64(1H,m),5.57(1H,s),6.38(2H,t,J=2.93Hz),6.52(1H,s),6.97(1 H,t,J=12.94Hz),7.13-7.34(4H,m).

### Example 24

### 2-(1-{3-[(4R,6S)-8-Chloro-6-(2,3-dimethoxyphenyl)-10-fluoro-4H,6H-pyrrolo[1,2-a] [4,1]benzoxazepin-4-yl]propanoyl}-4-piperidinyl)acetic acid

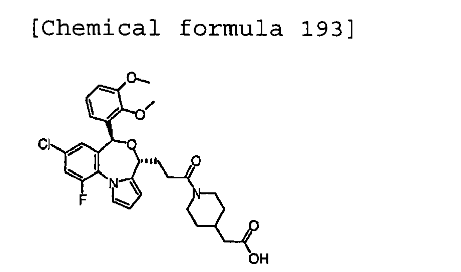

Ethyl 2-(1-{3-[(4R,6S)-8-chloro-6-(2,3-dimethoxyphenyl)-10-fluoro-4H,6H-pyrrolo[1,2-a][4,1]benzoxazepin-4-yl]propanoyl}-4-piperidinyl)acetate (58.0 mg) was treated with potassium carbonate (53.5 mg) to give the title compound (50.1 mg).
¹H-NMR(CDCl₃)δ:1.20-1.09(2H,m),1.74-1.84(2H,m),2.01(1H,br s),2.26-2.32(2H,m),2.37-2.44(2H,m),2.52-2.67(3H,m),3.02(1H,t,J=12.82Hz),3.45(3H,s),3.98-3.82(4H,m),4.38-4.43(1H,m),4.58-4.65(1H,m),5.57(1H,s),6.36-6.41(2H,m),6.50-6.53(1H,m),6.92-6.97(1H,m),7.14-7.37(4H,m).
Elemental analysis for: C₃₀H₃₂ClFN₂O₆
Calculated: C,63.10;H,5.65;N,4.91.
Found: C,62.83;H,5.67;N,4.89.

### Example 25

### Ethyl 2-(1-{2-[(4R,6S)-8-chloro-6-(2,3-dimethoxyphenyl)-10-fluoro-4H,6H-pyrrolo[1,2-a][4,1]benzoxazepin-4-yl]ethyl}-1H-1,2,4-triazol-5-yl)acetate

### Ethyl 2-(1-{2-[(4R,6S)-8-chloro-6-(2,3-dimethoxyphenyl)-10-fluoro-4H,6H-pyrrolo[1,2-a][4,1]benzoxazepin-4-yl]ethyl}-1H-1,2,4-triazol-3-yl)acetate

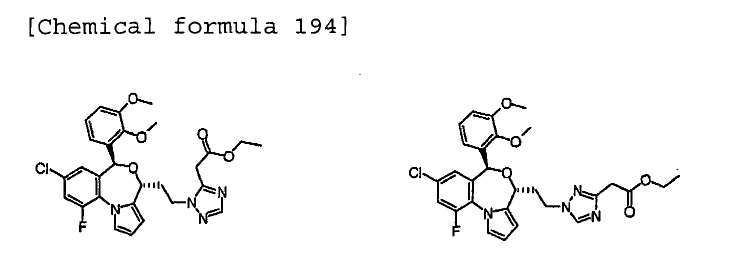

2-[(4R,6S)-8-Chloro-6-(2,3-dimethoxyphenyl)-10-fluoro-4H,6H-pyrrolo[1,2-a] [4,1]benzoxazepin-4-yl]ethylmethanesulfonate (330.3 mg) was treated with ethyl 2-(1H-1,2,4-triazol-3-yl)acetate (104 mg), potassium carbonate (276 mg), and tetrabutylammonium iodide (246 mg) to give the 5-acetate compound (88.3 mg) and 4-acetate compound (114 mg), respectively.
5-Acetate compound
¹H-NMR(CDCl₃)δ:1.24(3H,t,J=7.08Hz)2.69-2.60(2H,m),3.45(3H,s),3.79-3.92(SH,m),4.21-4.09(2H,m),4.47-4.34(3H,m),5.61(1H,s),6.36-6.33(1H,m),6.39(1H,t,J=3.30Hz),6.55-6.53(1H,m),7.01-6.95(1H,m),7.34-7.16(4H,m),7.84(1H,s).
4-Acetate compound
¹H-NMR(CDCl₃)δ:1.24(3H,t,J=7.08Hz),2.62(2H,q,J=6.92Hz),3.46(3H ,s),3.76(2H,s),3.86(3H,s),4.22-4.09(3H,m),4.31-4.54(3H,m),5.62(1H,s),6.32-6.35(1H,m),6.38-6.41(1H,m),6.53(1H,br s),6.97(1H,d,J=8.06Hz),7.16-7.30(4H,m),8.02(1H,s).

### Example 26

### 2-(1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,4-triazol-3-yl)acetic acid

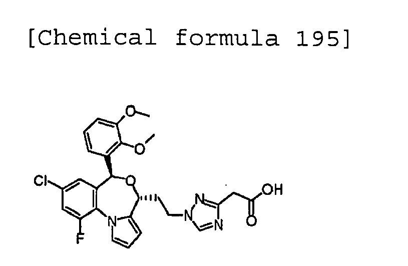

Ethyl 2-(1-{2-[(4R,6S)-8-chloro-6-(2,3-dimethoxyphenyl)-10-fluoro-4H,6H-pyrrolo[1,2-a][4,1]benzoxazepin-4-yl]ethyl}-1H-1,2,4-triazol-3-yl)acetate (113.7 mg) was treated with potassium carbonate (113.3 mg) to give the title compound (96.1 mg).
¹H-NMR(CDCl₃)δ:1.27(1H,br s),2.67-2.59(2H,m),3.46(3H,s),3.85(5H,d,J=9.03Hz),4.34-4.50(3H,m),5.62(1H,s),6.31-6.35(1H,m),6.39(1H,t,J=3.30Hz),6.52-6.55(1H,m),6.97(1H,dd,J=7.32,2.20Hz),7.16-7.25(4H,m),8.10(1H,s).
Elemental analysis for: C₂₆H₂₄ClFN₄O₅·0.25H₂O
Calculated: C,58.76;H,4.65;N,10.54.
Found: C,59.20;H,4.91;N,10.05.

### Referential Example 20

### (4R,6S)-4-(2-Azidoethyl)-8-chloro-6-(2,3-dimethoxyphenyl)-10-fluoro-4H,6H-pyrrolo[1,2-a][4,1]benzoxazepine

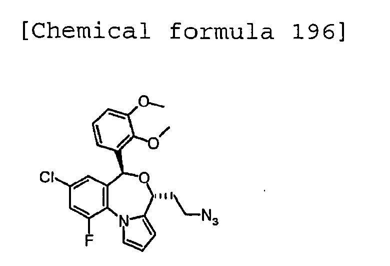

2-[(4R,6S)-8-Chloro-6-(2,3-dimethoxyphenyl)-10-fluoro-4H,6H-pyrrolo[1,2-a] [4,1]benzoxazepin-4-yl]ethylmethanesulfonate (842 mg) was dissolved in a dimethylformamide-water (10:1) solvent mixture (11 mL). To the resulting solution was added sodium azide (662 mg) and the resulting mixture was stirred at 80°C for 13 hours. After the reaction mixture was allowed to cool, water was added, followed by extraction with ethyl acetate. The extract was washed with saturated brine and dried over anhydrous sodium sulfate. The solvent was then distilled off under reduced pressure to give the title compound (750 mg) .

### Example 27

### 1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-5-ethyl ester

### 1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-4-ethyl ester

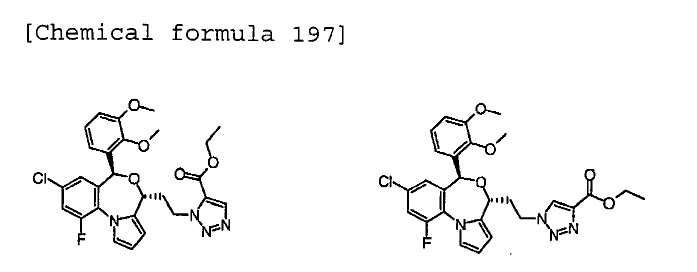

(4R,6S)-4-(2-Azidoethyl)-8-chloro-6-(2,3-dimethoxyphenyl)-10-fluoro-4H,6H-pyrrolo[1,2-a][4,1]benzoxazepine (749 mg) was dissolved in toluene (10 mL). Ethyl propionate (390 µl) was added and the resulting mixture was heated under reflux for 8 hours. After the reaction mixture was allowed to cool, the solvent was distilled off under reduced pressure. The residue was purified by silica gel column chromatography (hexane:ethyl acetate) to give the triazol-5-ester compound (248 mg) and the triazol-4-ester compound (560 mg).
Triazol-5-ester compound
¹H-NMR(CDCl₃)δ:1.36(3H,t,J=7.08Hz),2.55-2.79(2H,m),3.46(3H,s),3.85(3H,s),4.35(2H,q,J=7.08Hz),4.46(1 H,q,J=4.39Hz),5.03(2H,t,J=7.32Hz),5.61(1H,s),6.35-6.41(2H,m),6.53-6.56(1H,m),6.94-6.98(1H,m),7.16-7.25(3H,m),7.26-7.27(1H,m),7.35-7.40(1H,m),8.10-8.11(1H,m).
Triazol-4-ester compound
¹H-NMR(CDC1₃)b:1.39(3H,t,J=7.08Hz),2.60-2.78(2H,m),3.45-3.47(3H,m),3.86(3H,s),4.41(3H,q,J=7.08Hz),4.74(2H,t,J=7.57H z),5.63(1H,s),6.31-6.35(1H,m),6.40(1H,t,J=3.30Hz),6.53-6.55(1H,m),6.95-6.99(1H,m),7.17-7.27(4H,m),8.09-8.11(1H,m) .

### Example 28

### 1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-5-carboxylic acid

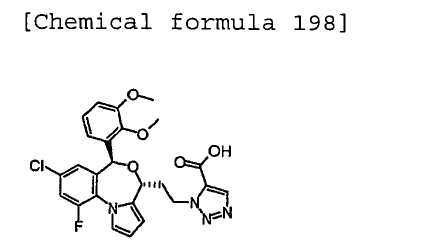

1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-5-ethyl ester (79.0 mg) was dissolved in tetrahydrofuran-methanol-water (1:1:2, 4 mL). Potassium carbonate (80.7 mg) was added and the resulting mixture was stirred at 55°C for 1 hour. Under ice cooling, a 1N aqueous hydrochloric acid solution was added to make the reaction mixture acidic, followed by extraction with ethyl acetate. The extract was washed with saturated brine and then dried over anhydrous sodium sulfate. The solvent was distilled off under reduced pressure. To the residue thus obtained was added ether-hexane. The solid thus formed was collected by filtration to give the title compound (46.3 mg).
Elemental analysis for: C₂₅H₂₂ClFN₄O₅·0.25H₂O
Calculated: C,58.03;H,4.38;N,10.83.
Found: C,58.24;H,4.54;N,10.35.

### Example 29

### 1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazole-4-carboxylic acid

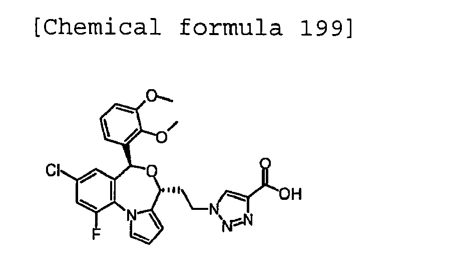

1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-4-ethyl ester (94.1 mg) was dissolved in tetrahydrofuran-methanol-water (1:1:2, 4 mL). Potassium carbonate (96.2 mg) was added and the resulting mixture was stirred at 55°C for 1 hour. Under ice cooling, a 1N aqueous hydrochloric acid solution was added to make the reaction mixture acidic, followed by extraction with ethyl acetate. The extract was washed with saturated brine and then dried over anhydrous sodium sulfate. The solvent was distilled off under reduced pressure. To the residue was added ether-hexane and the solid thus formed was collected by filtration to give the title compound (71.8 mg). Elemental analysis for: C₂₅H₂₂ClFN₄O₅·0.25H₂O
Calculated: C,58.03;H,4.38;N,10.83.
Found: C,57.72;H,4.44;N,10.62.

### Referential Example 21

### 2-[(4R,6S)-8-Chloro-6-(2,3-dimethoxyphenyl)-4H,6H-pyrrolo[1,2-a][4,1]benzoxazepin-4-yl]-1-ethanol

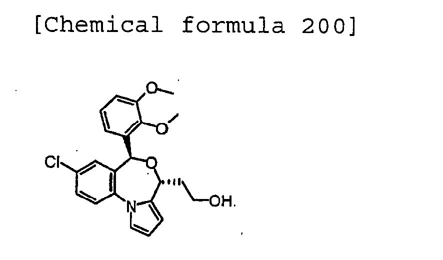

2-[(4R,6S)-8-Chloro-6-(2,3-dimethoxyphenyl)-4H,6H-pyrrolo[1,2-a][4,1]benzoxazepin-4-yl]methyl ester (5.92 g) was dissolved in tetrahydrofuran (100 mL). Under ice cooling, lithium aluminum hydride (856 mg) was added and the resulting mixture was stirred for 3 hours. Under ice cooling, water (1 mL), a 15% aqueous solution (1 mL) of sodium hydroxide, and water (3 mL) were added successively. The mixture was stirred for 3 hours. Magnesium sulfate was added to dry the mixture. After filtration through celite, the solvent was distilled off under reduced pressure to give the title compound (4.80 g).
¹H-NMR(CDCl₃)δ:2.16-2.50(3H,m),3.45(3H,s), 3.86(3H,s),3.93-3.99(2H,m),4.61-4.66(1H,m),5.76(1H,s),6.31-6.35(1H,m),6.39(1H,t,J=3.05Hz),6.72-6.75(1H,m),6.95(1H,d,J=8.30Hz),7.09-7.11(1H,m),7.19(1H,t,J=7.81Hz),7.25-7.28(1H,m),7.33-7.39(2H,m).

### Referential Example 22

### 2-[(4R,6S)-8-Chloro-6-(2,3-dimethoxyphenyl)-4H,6H-pyrrolo[1,2-a][4,1]benzoxazepin-4-yl]ethylmethanesulfonate

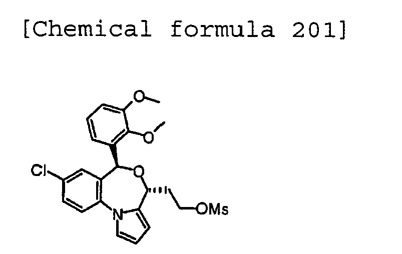

2-[(4R,6S)-8-Chloro-6-(2,3-dimethoxyphenyl)-4H,6H-pyrrolo[1,2-a][4,1]benzoxazepin-4-yl]-1-ethanol (4.79 g) was dissolved in dichloromethane (60 mL). Under ice cooling, triethylamine (2.51 mL) and methanesulfonic acid chloride (1.11 mL) were added and the resulting mixture was stirred for 3 hours. The reaction mixture was washed with water and a saturated aqueous solution of sodium bicarbonate, and then dried over anhydrous sodium sulfate. The solvent was distilled off under reduced pressure to give the title compound (5.77 g).
¹H-NMR(CDCl₃)δ:2.41-2.57(2H,m),2.98(3H,s),3.43(3H,s),3.85(3H,s),4.48-4.62(3H,m),5.73(1H,s),6.32-6.29(1H,m),6.39(1H,t,J=3.17Hz),6.72(1H,d,J=2.20Hz),6.96(1H, dd,J=8.06,1.46Hz),7.11(1H,q,J=1.46Hz),7.20(1H,t,J=8.06Hz),7 .25-7.31(1H,m),7.33-7.41(2H,m).

### Referential Example 23

### 3-[(4R,6S)-8-Chloro-6-(2,3-dimethoxyphenyl)-4H,6H-pyrrolo[1,2-a] [4,1]benzoxazepin-4-yl]propionitrile

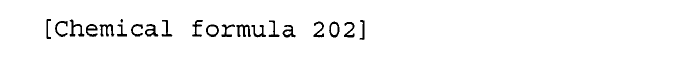

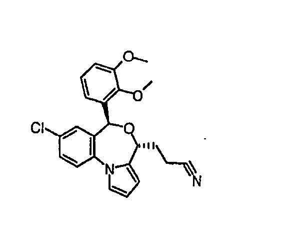

2-[(4R,6S)-8-Chloro-6-(2,3-dimethoxyphenyl)-4H,6H-pyrrolo[1,2-a][4,1]benzoxazepin-4-yl]ethylmethanesulfonate (5.77 g) was dissolved in dimethylsulfoxide (60 mL).
Sodium cyanide (1.21 g) was added and the resulting mixture was stirred at 50°C for 13 hours. After the reaction mixture was allowed to cool, water was added, followed by extraction with ethyl acetate. The extract was washed with saturated brine and then dried over anhydrous sodium sulfate. The solvent was distilled off under reduced pressure. The residue was purified by silica gel column chromatography (hexane:ethyl acetate) to give the title compound (4.51 g).
¹H-NMR(CDCl₃)δ:2.48-2.30(2H,m),2.68(2H,t,J=7.32Hz),3.43(3H,s),3.86(3H,s),4.49(1 H,dd,J=9.28,3.91Hz),5.73(1H,s),6.27-6.30(1H,m),6.39(1H,t,J=3.17Hz),6.72-6.74(1H,m),6.96(1H,d,J=8.06Hz),7.10-7.13(1H,m),7.17-7.23(1H,m),7.25-7.29(1H,m),7.33-7.41(2H,m).

### Example 30

### 3-[(4R,6S)-8-Chloro-6-(2,3-dimethoxyphenyl)-4H,6H-pyrrolo[1,2-a][4,1]benzoxazepin-4-yl]propionic acid

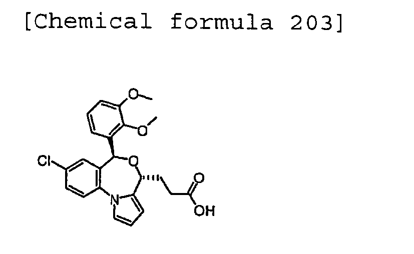

3-[(4R,6S)-8-Chloro-6-(2,3-dimethoxyphenyl)-4H,6H-pyrrolo[1,2-a][4,1]benzoxazepin-4-yl]propionitrile (4.51 g) was dissolved in 2-isopropanol (50 mL). A 5N aqueous sodium hydroxide solution (50 mL) was added and the resulting mixture was heated under reflux for 4 days.
Under reduced pressure, 2-isopropanol was distilled off. Under ice cooling, 1N hydrochloric acid was added to make the residue acidic, followed by extraction with ethyl acetate. The extract was washed with saturated brine and dried over anhydrous sodium sulfate. The solvent was distilled off under reduced pressure. The residue was purified by silica gel column chromatography (chloroform:methanol) to give the title compound (4.56 g).
¹H-NMR(CDCl₃)δ:1.55(1H,br s),2.80-2.30(4H,m),3.43(3H,s),3.85(3H,s),4.41(1H,q,J=4.48Hz),5.72(1 H,s),6.35-6.32(1H,m),6.38(1H,t,J=3.17Hz),6.71(1H,d,J=2.20Hz),6.94(1H, dd,J=8.30,1.46Hz),7.09-7.10(1H,m),7.18(1H,t,J=7.93Hz),7.25-7.39(3H,m).

### Referential Example 24

### 3-[(4R,6S)-8-Chloro-6-(2,3-dimethoxyphenyl)-4H,6H-pyrrolo[1,2-a][4,1]benzoxazepin-4-yl]-1-propanol

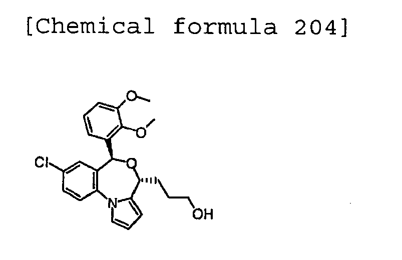

3-[(4R,6S)-8-Chloro-6-(2,3-dimethoxyphenyl)-4H,6H-pyrrolo[1,2-a][4,1]benzoxazepin-4-yl]propionic acid (267 mg) was dissolved in tetrahydrofuran (5 mL). Under ice cooling, lithium aluminum hydride (31 mg) was added and the resulting mixture was stirred for one and a half hours. Under ice cooling, water (50 µl), a 15% aqueous solution (50 µl) of sodium hydroxide, and water (150 µl) were added successively, followed by stirring for 1 hour. Anhydrous magnesium sulfate was added to dry the reaction mixture. After filtration through celite, the solvent was distilled off under reduced pressure to give the title compound (257 mg).
¹H-NMR(CDCl₃)δ:1.71-1.67(1H,m),2.00-1.75(2H,m),2.11-2.22(1H,m),3.43(3H,s),3.68-3.77(2H,m),3.85(3H,s),4.39(1H,dd,J=8.30,4.64Hz),5.71(1H,s), 6.31-6.35(1H,m),6.38(1H,t,J=3.17Hz),6.72(1H,d,J=1.95Hz),6.95(1H, dd,J=8.18,1.59Hz),7.09(1H,q,J=1.46Hz),7.19(1H,t,J=7.93Hz),7 .30-7.34(1H,m),7.35-7.37(2H,m).

### Referential Example 25

### 3-[(4R,6S)-8-Chloro-6-(2,3-dimethoxyphenyl)-4H,6H-pyrrolo[1,2-a][4,1]benzoxazepin-4-yl]propylmethanesulfonate

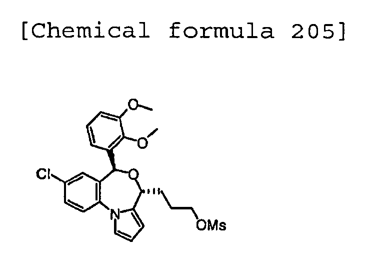

3-[(4R,6S)-8-Chloro-6-(2,3-dimethoxyphenyl)-4H,6H-pyrrolo[1,2-a][4,1]benzoxazepin-4-yl]-1-propanol (256 mg) was dissolved in dichloromethane (5 mL). Under ice cooling, triethylamine (130 µl) and methanesulfonic acid chloride (72 µl) were added. The resulting mixture was stirred for six and half hours. The reaction mixture was washed with a saturated aqueous solution of sodium bicarbonate and dried over anhydrous sodium sulfate. The solvent was distilled off under reduced pressure to give the title compound (414 mg). The resulting compound was provided for the subsequent reaction without purification.
¹H-NMR(CDCl₃)δ:2.05-1.89(2H,m), 2.23-2.10(2H,m),2.97(3H,s),3.43(3H,s),3.85(3H,s),4.41-4.27(4H,m),5.70(1H,s),6.32-6.29(1H,m),6.40-6.37(1H,m),6.73-6.70(1H,m),6.95(1H,d,J=8.30Hz),7.11-7.08(1H,m),7.19(1H,t,J=8.06Hz),7.31-7.27(1H,m),7.39-7.32(2H,m).

### Referential Example 26

### 2-[(4R,6S)-8-Chloro-6-(2,3-dimethoxyphenyl)-4H,6H-pyrrolo[1,2-a][4,1]benzoxazepin-4-yl]acetaldehyde

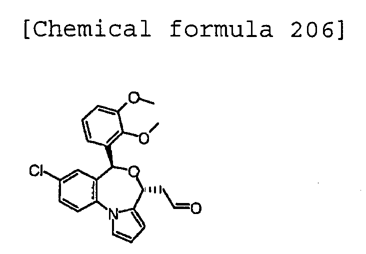

2-[(4R,6S)-8-Chloro-6-(2,3-dimethoxyphenyl)-4H,6H-pyrrolo[1,2-a][4,1]benzoxazepin-4-yl]-1-ethanol (536 mg) was dissolved in dichloromethane (10 mL). Under ice cooling, Dess-Martin Periodinane (1.17 g) was added and the resulting mixture was stirred for 2 hours. The reaction mixture was washed with a saturated aqueous solution of sodium bicarbonate and then dried over anhydrous sodium sulfate. The solvent was distilled off under reduced pressure to give the title compound (700 mg). The resulting compound was provided for the subsequent reaction without purification.
¹H-NMR (CDCl₃)δ: 2.98-3.19(3H,m),3.44(3H,s),3.85(3H,s),4.99(1H,dd,J=8.30,4.88Hz), 5.75(1H,s),6.27-6.25(1H,m),6.39(1H,t,J=3.05Hz),6.75(1H,d,J=1.95Hz),6.95(1H, d,J=8.06Hz),7.10-7.29(3H,m),7.37-7.40(1H,m),9.92(1H,s).

### Referential Example 27

### Methyl 4-[(4R,6S)-8-chloro-6-(2,3-dimethoxyphenyl)-4H,6H-pyrrolo[1,2-a][4,1]benzoxazepin-4-yl]-2-butenoate

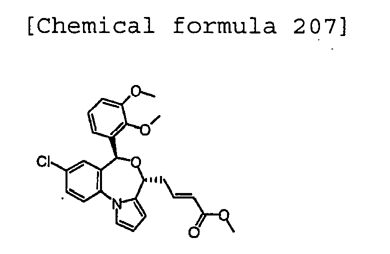

2-[(4R,6S)-8-chloro-6-(2,3-dimethoxyphenyl)-4H,6H-pyrrolo[1,2-a][4,1]benzoxazepin-4-yl]acetaldehyde (700 mg) was dissolved in dichloromethane (10 mL), followed by stirring. To the reaction mixture was added methyl triphenylphosphonoacetate (1.18 g). The resulting mixture was stirred at room temperature for 14 hours. The solvent was distilled off under reduced pressure. The residue was purified by silica gel column chromatography (hexane:ethyl acetate) to give the title compound (424 mg).
¹H-NMR(CDCl₃)δ:3.02-2.90(2H,m),3.44(3H,s),3.73(3H,s),3.85(3H,s),4.48-4.53(1H,m),5.70(1H,s),5.97(1H,d,J=15.62Hz),6.29-6.32(1H,m),6.38(1H,t,J=3.17Hz),6.73(1H,d,J=2.20Hz),6.95(1H, dd,J=8.30,1.46Hz),7.06-7.15(2H,m),7.19(1H,t,J=8.06Hz),7.27-7.39 (3H,m) .

### Example 31

### Methyl 4-[(4R,6S)-8-chloro-6-(2,3-dimethoxyphenyl)-4H,6H-pyrrolo[1,2-a][4,1]benzoxazepin-4-yl]butanoate

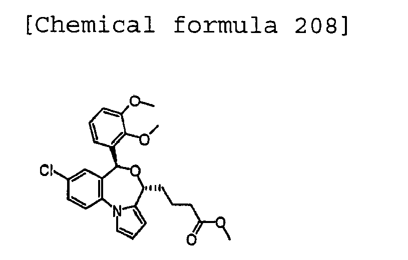

Methyl 4-[(4R,6S)-8-chloro-6-(2,3-dimethoxyphenyl)-4H,6H-pyrrolo[1,2-a][4,1]benzoxazepin-4-yl]-2-butenoate(423 mg) was dissolved in methanol (10 mL). To the resulting solution was added 10% palladium-carbon (400 mg), followed by stirring under a hydrogen atmosphere for 16 hours.
After removal of the catalyst by filtration, the solvent was distilled off under reduced pressure. The residue was purified by silica gel column chromatography (hexane:ethyl acetate) to give the title compound (211 mg).
¹H-NMR(CDCl₃)δ:1.92-2.05(4H,m),2.40(2H,t,J=7.20Hz),3.43(3H,s),3.66(3H,s),3.85(3 H,s),4.39-4.33(1H,m),5.68(1H,s),6.33-6.30(1H,m),6.37(1H,t,J=3.17Hz),6.79-6.70(1H,m),6.96-6.92(1H,m),7.08(1H,q,J=1.46Hz),7.18(1H,t,J=8.06Hz),7.42-7.30(3H,m).

### Example 32

### 4-[(4R,6S)-8-Chloro-6-(2,3-dimethoxyphenyl)-4H,6H-pyrrolo[1,2-a][4,1]benzoxazepin-4-yl]butanoic acid

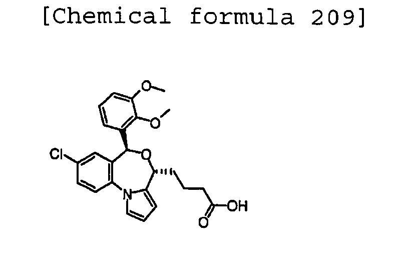

Methyl,4-[(4R,6S)-8-chloro-6-(2,3-dimethoxyphenyl)-4H,6H-pyrrolo[1,2-a][4,1]benzoxazepin-4-yl]butanoate (210 mg) was dissolved in a tetrahydrofuran-methanol-water (1:1:2) solvent mixture (8 mL). The resulting solution was reacted and treated in a similar manner by using potassium carbonate (255 mg) to give the title compound (176 mg).
¹H-NMR(CDCl₃)δ:1.20-1.37(1H,m),1.76-2.26(3H,m),2.26-2.51(2H,m),3.43(3H,s),3.85(3H,s),4.36-4.37(1H,m),5.69(1H,s),6.30-6.33(1H,m),6.37(1H,t,J=3.05Hz),6.71-6.72(1H,m),6.93-6.95(1H,m),7.07-7.10(1H,m),7.13-7.40(4H,m).

### Referential Example 28

### Ethyl 3-{1-[4-chloro-2-(2,3-dimethoxybenzoyl)phenyl]-1H-pyrrol-2-yl}-3-hyrdoxy-2,2-dimethylpropionate

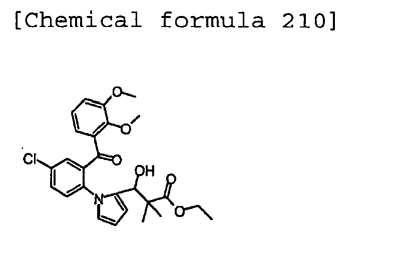

Ethyl 2-bromoisobutylate (6.07 mL) and activated zinc (2.65 g) were added to benzene (40 mL). Under a nitrogen atmosphere, the resulting mixture was heated under reflux for 1 hour. After ice cooling, a solution of 1-[4-chloro-2-(2,3-dimethoxybenzoyl)phenyl]-1H-pyrrolo-2-carbaldehyde (3.00 g) in benzene (30 mL) was added to the reaction mixture over 10 hours. After the resulting mixture was returned to room temperature, a trace of iodine was added. The resulting mixture was heated under reflux for 1 hour again. Under ice cooling, 1N hydrochloric acid was added. After filtration through celite, extraction was performed with ethyl acetate. The extract was washed with a saturated aqueous solution of sodium bicarbonate and saturated brine and then, dried by adding anhydrous sodium sulfate. The solvent was distilled off under reduced pressure. The residue was purified by silica gel column chromatography (hexane:ethyl acetate) to give the title compound (2.89 g).
¹H-NMR(CDCl₃)6:0.99(3H,s),1.07-1.35(6H,m),3.65(3H,s),3.84(3H,s),4.12(2H,q,J=7.08Hz),4.63(1 H,s),5.90(1H,s),6.16(1H,s),6.47(1H,s),6.80-7.10(3H,m),7.22-7.35(1H,m),7.46-7.60(2H,m).

### Referential Example 29

### Ethyl 3-{1-[4-chloro-2-(2,3-dimethoxyphenyl)(hydroxy)phenyl]-1H-pyrrol-2-yl}-3-hydroxy-2,2-dimethylpropionate

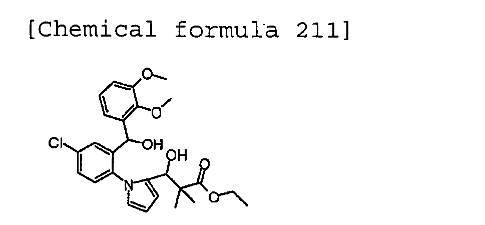

Ethyl 3-{1-[4-chloro-2-(2,3-dimethoxybenzoyl)phenyl]-1H-pyrrol-2-yl}-3-hydroxy-2,2-dimethylpropionate (2.89 g) was dissolved in methanol (30 mL). While cooling, sodium borohydride (450 mg) was added in portions and the resulting mixture was stirred at room temperature for 24 hours. After the solvent was distilled off under reduced pressure, water was added to the residue, followed by extraction with ethyl acetate. The extract was washed with saturated brine and dried over anhydrous sodium sulfate. The solvent was distilled off under reduced pressure to give the title compound (2.68 g).
¹H-NMR(CDCl₃)δ:1.20-1.26(6H,m),1.35(3H,s),3.53(3H,s),3.86(3H,s),4.09-4.20(2H,m),4.43(1H,d,J=8.30Hz),5.57(1H,d,J=3.91Hz),6.08-6.11(1H,m),6.16-6.20(1H,m),6.24-6.30(1H,m),6.48-6.51(1H,m),6.88-6.92(2H,m),7.06(1H,t,J=7.93Hz),7.22-7.47(3H,m).

### Example 33

### Ethyl 2-[8-chloro-6-(2,3-dimethoxyphenyl)-4H,6H-pyrrolo[1,2-a][4,1]benzoxazepin-4-yl]-2-methylpropionate

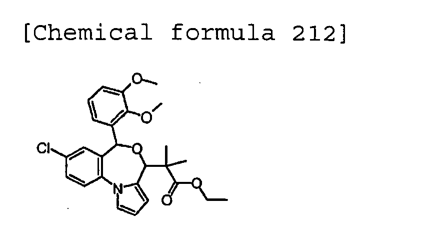

Ethyl 3-{1-[4-chloro-2-(2,3-dimethoxyphenyl)(hydroxy)phenyl]-1H-pyrrol-2-yl}-3-hydroxy-2,2-dimethylpropionate (2.67 g) was dissolved in toluene (60 mL). Diphosphorus pentoxide (1.16 g) was added and the resulting mixture was stirred at 80°C for 3 hours. After the reaction mixture was allowed to cool, ethyl acetate was added. The resulting mixture was washed with a saturated aqueous solution of sodium bicarbonate and saturated brine and dried over anhydrous sodium sulfate. The solvent was distilled off under reduced pressure. The residue was purified by silica gel column chromatography (hexane:ethyl acetate) to give the title compound (1.96 g).
¹H-NMR(CDCl₃)δ:0.87(1H,s),0.99(1H,s),1.07(2H,t,J=7.08Hz), 1.22( 1H,t,J=7.08Hz),1.46(2H,s),1.48(2H,s),3.43 and 3.54(1H,s and 2H,s),3.86-3.86(3H,m),4.01-4.15(2H,m),4.63 and 5.50(0.7H,s and 0.3H,s),5.68 and 6.07(0.7H,s and 0.3H,s),6.22-6.38(2H,m),6.65(1H,dd,J=26.61,2.20Hz),7.41-6.89(6H,m).

### Example 34

2-[8-Chloro-6-(2,3-dimethoxyphenyl)-4H,6H-pyrrolo[1,2-a][4,1]benzoxazepin-4-yl]-2-methylpropionic acid

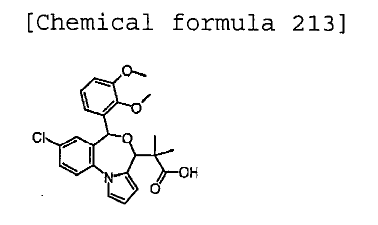

Ethyl 2-[8-chloro-6-(2,3-dimethoxyphenyl)-4H,6H-pyrrolo [1,2-a] [4,1]benzoxazepin-4-yl]-2-methylpropionate (1.43 g) was dissolved in 2-isopropanol (10 mL). A 5N aqueous sodium hydroxide solution (1.22 mL) was added. The resulting mixture was stirred at 80°C for four hours.
After the reaction mixture was allowed to cool, water was added thereto. The solvent was distilled off under reduced pressure. Under ice cooling, the residue was made acidic with 1N hydrochloric acid, followed by extraction with ethyl acetate. The extract was washed with saturated brine and then, dried over anhydrous sodium sulfate. The solvent was distilled off under reduced pressure to give the title compound (1.43 g).
¹H-NMR(CDCl₃)δ:0.99(1H,s)1.15(1H,s)1.36(2H,s)1.56(2H,s)3.4 6 and 3.59(2H,s and 1H,s),3.85-3.87(3H,m),4.40 and 5.33(0.7H,s and 0.3H,s),5.75 and 6.18(0.7H,s and 0.3H,s),6.29-6.44(2H,m),6.62-6.94(1H,m),6.95-7.12(2H,m),7.15-7.49(4H,m).
Elemental analysis for: C₂₄H₂₄ClNO₅
Calculated: C,65.23;H,5.47;N,3.17.
Found: C,65.13;H,5.40;N,2.70.

### Example 35

### Ethyl 2-(1-{2-[8-chloro-6-(2,3-dimethoxyphenyl)-4H,6H-pyrrolo[1,2-a][4,1]benzoxazepin-4-yl]-2-methylpropionyl}4-piperidinyl)acetate

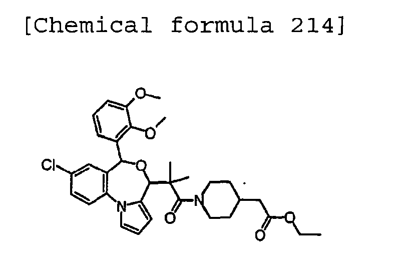

2-[8-Chloro-6-(2,3-dimethoxyphenyl)-4H,6H-pyrrolo[1,2-a][4,1]benzoxazepin-4-yl]-2-methylpropionic acid (249 mg) was dissolved in dichloromethane (6 mL). The resulting solution was reacted and treated in a similar manner by using ethyl piperidine-4-acetate (106 mg) to give the title compound (161.3 mg).
¹H-NMR(CDCl₃)δ:0.53-0.33(1H,m), 0.94-0.71(1H,m),1.25(3H,td,J=7.14,3.82Hz),1.48(3H,s),1.59(3H,s), 1.78-2.16(4H,m),2.47-2.58(1H,m),2.68-2.79(1H,m),3.44(3H,s),3.86(3H,s),4.07-4.22(3H,m),4.45-4.54(1H,m),4.68(1H,s),5.70(1H,s),6.32(1H,t,J=3.30Hz),6.44-6.49(1H,m),6.69(1H,d,J=1.71Hz),6.93-6.98(1H,m),7.05(1H,q,J=1.38Hz),7.18(1H,t,J=7.93Hz),7.25-7.38(3H,m).

### Example 36

### Ethyl 2-(1-{2-[(4S,6R)-8-chloro-6-(2,3-dimethoxyphenyl)-4H,6H-pyrrolo[1,2-a][4,1]benzoxazepin-4-yl]-2-methylpropionyl}-4-piperidinyl)acetate (isomer A)

### Ethyl 2-(1-{2-[(4R,6S)-8-chloro-6-(2,3-dimethoxyphenyl)-4H,6H-pyrrolo[1,2-a][4,1]benzoxazepin-4-yl]-2-methylpropionyl}-4-piperidinyl)acetate (isomer B)

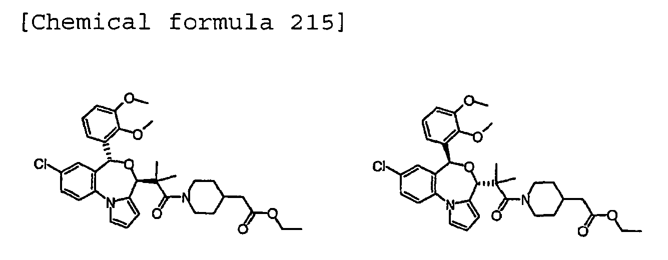

Ethyl 2-(1-{2-[8-chloro-6-(2,3-dimethoxyphenyl)-4H,6H-pyrrolo[1,2-a][4,1]benzoxazepin-4-yl]-2-methylpropionyl}4-piperidinyl)acetate (161.3 mg) was isolated and purified using HPLC [CHIRALPAK AD (Daicel, 20ϕ × 250 mm), eluting solvent: hexane:2-isopropanol=80:20, dissolution rate: 10 mL/min], whereby the isomer A was obtained from the fraction with a retention time of 10.4 minutes and the isomer B was obtained from the fraction with a retention time of 12.0 minutes, respectively.

### Example 37

### 2-(1-{2-[(4S,6R)-8-Chloro-6-(2,3-dimethoxyphenyl)-4H,6H-pyrrolo[1,2-a][4,1]benzoxazepin-4-yl]-2-methylpropionyl}-4-piperidinyl)acetic acid

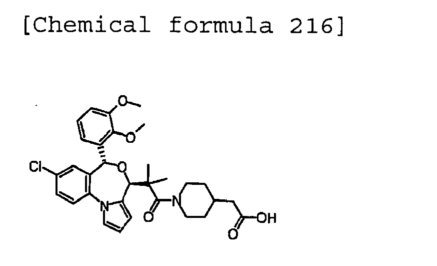

Ethyl 2-(1-{2-[(4S,6R)-8-chloro-6-(2,3-dimethoxyphenyl)-4H,6H-pyrrolo[1,2-a][4,1]benzoxazepin-4-yl]-2-methylpropionyl}-4-piperidinyl)acetate (63.0 mg) was dissolved in a tetrahydrofuran-methanol-water (1:1:2) solvent mixture (4 mL). The resulting solution was reacted and treated in a similar manner by using potassium carbonate (59 mg) to give the title compound (47.3 mg).
Elemental analysis for: C₃₁H₃₅CN₂O₆
Calculated: C,65.66;H,6.22;N,4.94.
Found: C,65.22;H,6.12;N,4.64.

### Example 38

### 2-(1-{2-[(4R,6S)-8-Chloro-6-(2,3-dimethoxyphenyl)-4H,6H-pyrrolo[1,2-a] [4,1]benzoxazepin-4-yl]-2-methylpropionyl}-4-piperidinyl)acetic acid

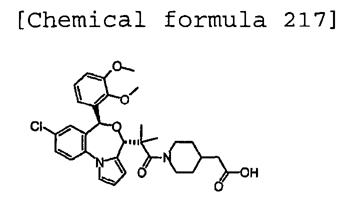

Ethyl 2-(1-{2-[(4R,6S)-8-chloro-6-(2,3-dimethoxyphenyl)-4H,6H-pyrrolo[1,2-a][4,1]benzoxazepin-4-yl]-2-methylpropionyl}-4-piperidinyl)acetate (66.0 mg) was dissolved in a tetrahydrofuran-methanol-water (1:1:2) solvent mixture (4 mL). The resulting solution was reacted and treated in a similar manner by using potassium carbonate (61 mg) to give the title compound (45.1 mg). ¹H-NMR(CDCl₃)δ:0.30-0.52(1H,m) 0.78-0.92(1H,m),1.35-1.70(8H,m),1.78-1.95(1H,m),1.98-2.24(2H,m),2.47-2.59(1H,m),2.69-2.80(1H,m),3.44(3H,s),3.86(3H,s),4.10-4.24(1H,m),4.47-4.58(1H,m),4.68(1H,s),5.70(1H,s),6.32(1H,t,J=3.30Hz),6.47(1 H,d,J=2.44Hz),6.69(1H,s),6.96(1H,dd,J=8.30,1.46Hz),7.06(1H, q,J=1.46Hz),7.18(1H,t,J=8.06Hz),7.31-7.27(1H,m),7.33-7.35(2H,m).
Elemental analysis for: C₃₁H₃₅CN₂O₆
Calculated: C,65.66;H,6.22;N,4.94.
Found: C,65.44;H,6.18;N,4.75.

### Example 39

### Ethyl 2-(1-{2-[(4R,6S)-8-chloro-6-(2,3-dimethoxyphenyl)-4H,6H-pyrrolo[1,2-a][4,1]benzoxazepin-4-yl]acetyl}-4-piperidinyl)acetate (isomer A)

### Ethyl 2-(1-{2-[(4S,6R)-8-chloro-6-(2,3-dimethoxyphenyl)-4H,6H-pyrrolo[1,2-a][4,1]benzoxazepin-4-yl]acetyl}-4-piperidinyl)acetate (isomer B)

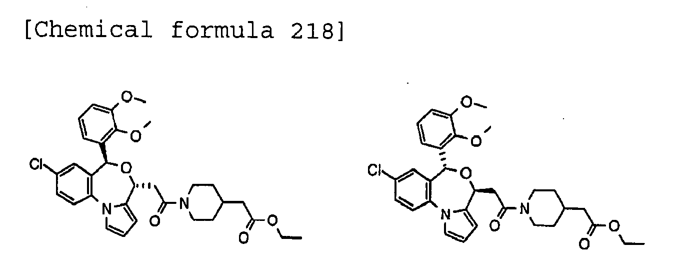

Ethyl 2-(1-{2-trans-[8-chloro-6-(2,3-dimethoxyphenyl)-4H,6H-pyrrolo[1,2-a][4,1]benzoxazepin-4-yl]acetyl}-4-piperidinyl)acetate (89.0 mg) was subjected to optical resolution with an optically active column
(CHIRALCEL-OD) to give the isomer A (30.2 mg) and the isomer B (33.3 mg), respectively.
Flow rate: 10 mL/min
Developing solvent: 20% 2-propanol-n-hexane
Retention time: isomer A: 19 min., isomer B: 35 min.

### Example 40

### 2-(1-{2-[(4R,6S)-8-Chloro-6-(2,3-dimethoxyphenyl)-4H,6H-pyrrolo [1, 2-a] [4,1]benzoxazepin-4-yl]acetyl}-4-piperidinyl)acetic acid

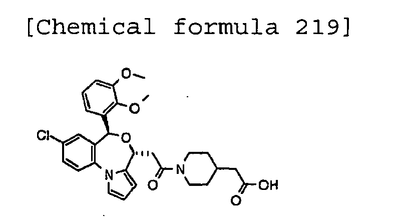

Ethyl 2-(1-{2-[(4R,6S)-8-chloro-6-(2,3-dimethoxyphenyl)-4H,6H-pyrrolo[1,2-a][4,1]benzoxazepin-4-yl]acetyl}-4-piperidinyl)acetate (30.2 mg) was dissolved in methanol-water-tetrahydrofuran (2:1:0.5, 3.5 mL). Potassium carbonate (22.1 mg) was added and the resulting mixture was stirred overnight at room temperature. After the temperature was raised to 45°C, stirring was continued for further 7 hours. After addition of acetic acid (0.0091 mL), the resulting mixture was diluted with chloroform and washed with saturated brine. The organic layer was dried over anhydrous sodium sulfate. The filtrate was then concentrated. The residue thus obtained was purified by preparative TLC (methanol:chloroform=1:10) to give the title compound (24.1 mg).
MS(ESI)m/z:539(M⁺+H).
¹H-NMR(CDCl₃)δ:1.01-1.24(2H,m),1.59-1.82(2H,m),1.93-2.02(1H,m),2.13-2.15(1H,m),2.24-2.28(1H,m),2.53-2.69(1H,m),2.79-2.91(1H,m),2.95-3.12(1H,m),3.23-3.32(1H,m),3.41(3H,s),3.85(3H,s),4.02-4.06(1H,m),4.62-4.73(1H,m),4.90-4.99(1H,m),5.72-5.76(1H,m),6.24(1H,br s),6.36(1H,t,J=3.2Hz),6.68-6.70(1H,m),6.92-6.95(1H,m),7.08-7.10(1H,m),7.15(1H,t,J=7.9Hz),7.21-7.36(3H,m).

### Example 41

### 2-(1-{2-[(4S,6R)-8-Chloro-6-(2,3-dimethoxyphenyl)-4H,6H-pyrrolo[1,2-a][4,1]benzoxazepin-4-yl]acetyl}-4-piperidinyl)acetic acid

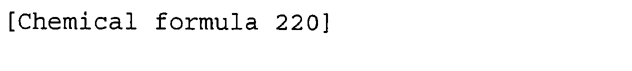

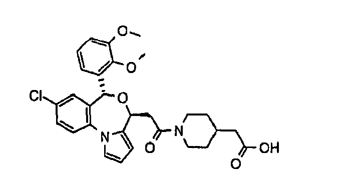

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]acetyl}-4-piperidinyl)acetic acid, ethyl 2-(1-{2-[(4S,6R)-8-chloro-6-(2,3-dimethoxyphenyl)-4H,6H-pyrrolo[1,2-a][4,1]benzoxazepin-4-yl]acetyl}-4-piperidinyl)acetate (33.3 mg) was treated while using potassium carbonate (24.3 mg) to give the title compound (20.4 mg).
MS(ESI)m/z:539(M⁺+H).
¹H-NMR(CDCl₃)δ:1.01-1.24(2H,m),1.59-1.82(2H,m),1.93-2.02(1H,m),2.13-2.15(1H,m),2.24-2.28(1H,m),2.53-2.69(1H,m),2.79-2.91(1H,m),2.95-3.12(1H,m),3.23-3.32,(1H,m),3.41(3H,s),3.85(3H,s),4.02-4.06(1H,m),4.62-4.73(1H,m),4.90-4.99(1H,m),5.72-5.76(1H,m),6.24(1H,br s),6.36(1H,t,J=3.2Hz),6.68-6.70(1H,m),6.92-6.95(1H,m),7.08-7.10(1H,m),7.15(1H,t,J=7.9Hz),7.21-7.36(3H,m).

### Example 42

### Methyl 2-(1,8-chloro-6-(2,3-dimethoxyphenyl)-4H,6H-pyrrolo(1,2-a)(4,1)benzoxazepin-4-yl)acetate

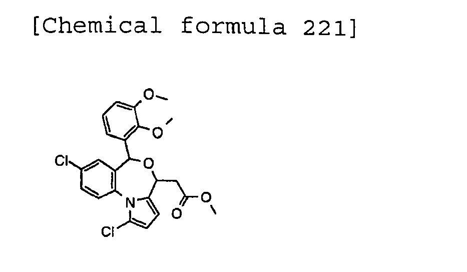

Methyl₂-(8-chloro-6-(2,3-dimethoxyphenyl)-4H,6H-pyrrolo(1,2-a)(4,1)benzoxazepin-4-yl)acetate (52.0 mg) was dissolved in tetrahydrofuran (3 mL). N-chlorosuccinimide (16.2 mg) was added and the resulting mixture was stirred overnight at room temperature. Water was added to the reaction mixture, followed by extraction with chloroform. The organic layer was dried over anhydrous sodium sulfate. The filtrate was then concentrated. The residue thus obtained was purified by preparative TLC (ethyl acetate:n-hexane=1:6) to give the title compound (52.3 mg).
MSm/z:462(M⁺+H).
¹H-NMR(CDCl₃)δ:2.99(1H,dd,J=15.3,6.5Hz),3.06(1H,dd,J=15.3,7.8H z),3.42(3H,s),3.71(3H,s),3.85(3H,s),4.72-4.74(1H,m),5.62(1H,s),6.23(1H,d,J=3.9Hz),6.29(1H,d,J=3.9Hz) ,6.76(1H,d,J=2.4Hz),6.94(1H,dd,J=7.9,1.3Hz),7.16(1H,t,J=7.9 Hz),7.21-7.22(1H,m),7.41(1H,dd,J=8.5,2.4Hz),7.51(1H,d,J=8.5Hz).

### Example 43

### 2-(1,8-Chloro-6-(2,3-dimethoxyphenyl)-4H,6H-pyrrolo(1,2-a)(4,1)benzoxazepin-4-yl)acetic acid

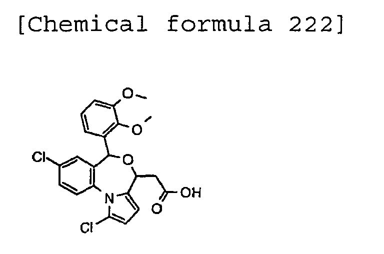

Methyl 2-(1,8-chloro-6-(2,3-dimethoxyphenyl)-4H,6H-pyrrolo(1,2-a)(4,1)benzoxazepin-4-yl)acetate (73.1 mg) was dissolved in methanol-water (2:1, 3 mL). Potassium carbonate (65.6 mg) was added and the resulting mixture was stirred overnight at room temperature. After addition of acetic acid (0.0272 mL) to the reaction mixture, the resulting mixture was diluted with chloroform and washed with water. The organic layer was dried over anhydrous magnesium sulfate. The filtrate was then concentrated.
The residue thus obtained was purified by preparative TLC (methanol:chloroform=1:10) to give the title compound (28.3 mg).
MS(ESI)m/z:447(M⁺+H).
¹H-NMR(CDCl₃)δ:2.94-3.06(2H,m),3.42(3H,s),3.84(3H,s),4.69(1H,t,J=6.3Hz),5.63(1H ,s),6.24(1H,d,J=3.7Hz),6.27(1H,d,J=3.7Hz),6.75-6.78(1H,m),6.92(1H,d,J=7.9Hz),7.13(1H,t,J=7.9Hz),7.22(1H,d, J=7.9Hz),7.39(1H,dd,J=8.5,2.2Hz),7.49(1H,d,J=8.5Hz).

### Example 44

### Ethyl 2-(1-(2-(1,8-chloro-6-(2,3-dimethoxyphenyl)-4H,6H-pyrrolo(1,2-a)(4,1)benzoxazepin-4-yl)acetyl)-4-piperidinyl)acetate

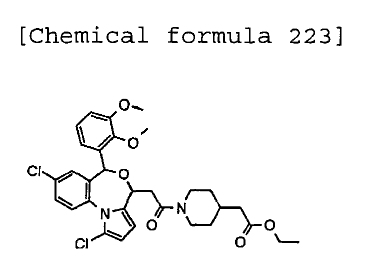

2-(1,8-Chloro-6-(2,3-dimethoxyphenyl)-4H,6H-pyrrolo(1,2-a)(4,1)benzoxazepin-4-yl)acetic acid (14.0 mg) and ethyl 2-(4-piperidinyl)acetate (5.9 mg) were dissolved in methylene chloride (2 mL). To the resulting solution were added 1-ethyl-3-(3-dimethylaminopropyl)carbodiimide hydrochloride (7.2 mg) and 1-hydroxybenzotriazole (1.4 mg). The resulting mixture was stirred for 3 hours at room temperature. The reaction mixture was diluted with chloroform and washed with saturated brine. The organic layer was dried over anhydrous sodium sulfate. The filtrate was then concentrated. The residue thus obtained was purified by preparative TLC (methanol:chloroform=5:100) to give the title compound (16.6 mg).
MS (ESI) m/z : 601 (M⁺+H).
¹H-NMR(CDCl₃)δ:1.08-1.22(2H,m),1.26(3H,t,J=7.1Hz),1.71-1.81(2H,m),1.95-2.06(1H,m),2.14-2.19(1H,m),2.23-2.26(1H,m),2.78-2.90(1H,m),3.15-3.22(1H,m),3.41(3H,s),3.85(3H,s),3.99-4.03(1H,m),4.09-4.17(2H,m),4.59-4.69(1H,m),4.76-4.85(1H,m),5.62-5.67(1H,m),6.16-6.19(1H,m),6.27-6.28(1H,m),6.73-6.75(1H,m),6.93-6.95(1H,m),7.13-7.18(1H,m),7.19-7.25(1H,m),7.37-7.40(1H,m),7.48-7.50(1H,m).

### Example 45

### 2-(1-(2-(1,8-Chloro-6-(2,3-dimethoxyphenyl)-4H,6H-pyrrolo(1,2-a)(4,1)benzoxazepin-4-yl)acetyl)-4-piperidinyl)acetic acid

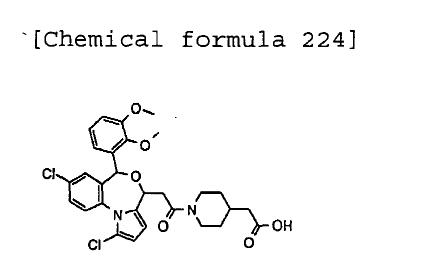

Ethyl 2-(1-(2-(1,8-chloro-6-(2,3-dimethoxyphenyl)-4H,6H-pyrrolo(1,2-a)(4,1)benzoxazepin-4-yl)acetyl)-4-piperidinyl)acetate (16.6 mg) was dissolved in methanol-water-tetrahydrofuran (2:1:1, 2 mL). To the resulting solution was added potassium carbonate (11.4 mg). The resulting mixture was stirred at room temperature for 4 hours. The temperature was raised to 45°C and then, the reaction mixture was stirred overnight further. After addition of acetic acid (0.0047 mL), the resulting mixture was diluted with chloroform and washed with saturated brine. The organic layer was dried over anhydrous magnesium sulfate. The filtrate was then concentrated.
The residue thus obtained was purified by preparative TLC (methanol:chloroform=1:10) to give the title compound (11.0 mg).
MS (ESI)m/z:573(M⁺+H).
¹H-NMR(CDCl₃)δ:1.08-1.24(2H,m),1.63-1.83(2H,m),1.99-2.30(3H,m),2.52-2.68(1H,m),2.78-2.89(1H,m),2.95-3.12(1H,m),3.16-3.23(1H,m),3.40(3H,s),3.85(3H,s),3.99-4.02(1H,m),4.59-4.69(1H,m),4.75-4.84(1H,m),5.63-5.66(1H,m),6.17-6.18(1H,m),6.27-6.28(1H,m),6.74(1H,dd,J=8.4,2.3Hz),6.94(1H,d,J=8.4Hz),7.12-7.25(2H,m),7.37-7.39(1H,m),7.48-7.50(1H,m).

### Example 46

### Ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate

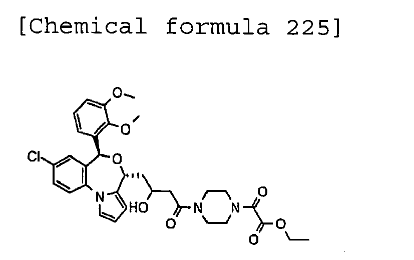

4-[(4R,6S)8-Chloro-6-(2,3-dimethoxyphenyl)-4H,6H-pyrrolo[1,2-a] [4,1]benzoxazepin-4-yl]-3-hydroxybutanoic acid (isomer A) (100 mg) was dissolved in N,N-dimethylformamide (2 mL). Ethyl 2-oxo-2-(1-piperazinyl)acetate hydrochloride (62.1 mg), triethylamine (0.091 mL) and diethyl cyanophosphate (0.040 mL) were added. The resulting mixture was stirred overnight at room temperature. The reaction mixture was diluted with ethyl acetate and washed with saturated brine. The organic layer was dried over anhydrous sodium sulfate. The filtrate was then concentrated. The residue thus obtained was purified by preparative TLC (ethyl acetate:hexane=1:1) to give the title compound (88.8 mg).
MS(ESI)m/z:626 (M⁺+H) .
¹H-NMR(CDCl₃)δ:1.36-1.40(3H,m),2.19-2.25(2H,m),2.45-2.54(1H,m),2.62-2.72(1H,m),3.43(3H,s),3.44-3.53(3H,m),3.60-3.68(3H,m),3.74-3.75(1H,m),3.85(3H,s),4.02-4.05(1H,m),4.35-4.38(2H,m),4.53-4.55(1H,m),4.72(1H,dd,J=9.5,3.2Hz),5.74(1H,s),6.30-6.31(1H,m),6.37(1H,t,J=3.2Hz),6.72(1H,d,J=2.0Hz),6.95(1H,dd ,J=8.3,1.5Hz),7.08-7.09(1H,m),7.17(1H,t,J=8.3Hz),7.29-7.32(1H,m),7.34-7.38(2H,m).

### Example 47

### Ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate

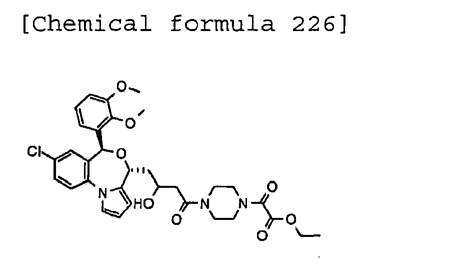

4-[(4R,6S)8-Chloro-6-(2,3-dimethoxyphenyl)-4H,6H-pyrrolo[1,2-a] [4,1]benzoxazepin-4-yl]-3-hydroxybutanoic acid (isomer B) (100 mg) was dissolved in N,N-dimethylformamide (2 mL). To the resulting solution were added ethyl 2-oxo-2-(1-piperazinyl)acetate hydrochloride (62.1 mg), triethylamine (0.091 mL) and diethyl cyanophosphate (0.040 mL). The resulting mixture was stirred overnight at room temperature. The reaction mixture was diluted with ethyl acetate and washed with saturated brine. The organic layer was dried over anhydrous sodium sulfate. The filtrate was then concentrated. The residue thus obtained was purified by preparative TLC (ethyl acetate:hexane=1:1) to give the title compound (101 mg).
MS(ESI)m/z:626(M⁺+H).
¹H-NMR(CDCl₃)δ:1.37(3H,t,J=7.2Hz),2.20-2.28(1H,m),2.30-2.39(1H,m),2.49-2.64(2H,m),3.43(3H,s),3.43-3.48(3H,m),3.53-3.61(3H,m),3.75-3.76(1H,m),3.85(3H,s),3.96-3.98(1H,m),4.33-4.37(2H,m),4.42-4.46(1H,m),4.66-4.74(1H,m),5.74(1H,s),6.31-6.33(1H,m),6.36-6.38(1H,m),6.72-6.74(1H,m),6.94-6.96(1H,m),7.09-7.09(1H,m),7.16-7.20(1H,m),7.24-7.25(1H,m),7.36-7.40(2H,m).

### Example 48

Methyl 1-{4-[(4R,6S)8-chloro-6-(2,3-dimethoxyphenyl)-4H,6H-pyrrolo[1,2-a][4,1]benzoxazepin-4-yl]-3-hydroxybutanoyl}-3-azetidine carboxylate

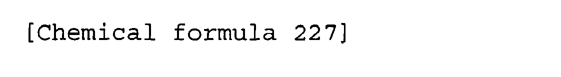

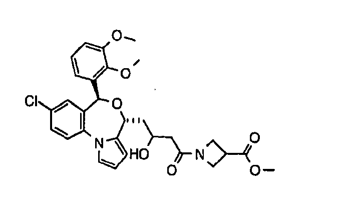

In a similar manner to that employed for the synthesis of ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate, 4-[(4R,6S)-8-Chloro-6-(2,3-dimethoxyphenyl)-4H,6H-pyrrolo[1,2-a][4,1]benzoxazepin-4-yl]-3-hydroxybutanoic acid (isomer A) (92.5 mg) and methyl 3-azetidine carboxylate hydrochloride (45.9 mg) were treated to give the title compound (86.6 mg).
MS(ESI)m/z:555(M⁺+H).
¹H-NMR(CDCl₃)δ:2.15-2.27(3H,m),2.36-2.45(1H,m),3.43(3H,s),3.44-3.51(1H,m),3.75(3H,s),3.85(3H,s),4.14-4.34(4H,m),4.46-4.53(1H,m),4.68-4.74(1H,m),5.74-5.74(1H,m),6.29-6.31(1H,m),6.35-6.37(1H,m),6.71-6.72(1H,m),6.94-6.96(1H,m),7.07-7.09(1H,m),7.16-7.22(1H,m),7.32-7.37(3H,m).

### Example 49

Methyl 1-{4-[(4R,6S)8-chloro-6-(2,3-dimethoxyphenyl)-4H,6H-pyrrolo[1,2-a][4,1]benzoxazepin-4-yl]-3-hydroxybutanoyl}-3-azetidine carboxylate

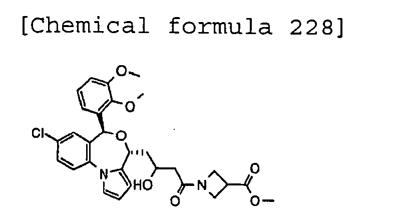

4-[(4R,6S)8-Chloro-6-(2,3-dimethoxyphenyl)-4H,6H-pyrrolo[1,2-a][4,1]benzoxazepin-4-yl]-3-hydroxybutanoic acid (isomer B) (100 mg) and methyl 3-azetidine carboxylate hydrochloride (49.6 mg) were treated in a similar manner to that employed for the synthesis of ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate to give the title compound (98.5 mg).
MS (ESI) m/z: 555 (M⁺+H) ¹H-NMR(CDCl₃)δ:2.18-2.33(4H,m),3.43(3H,s),3.46-3.51(1H,m),3.76(3H,s),3.85(3H,s),4.13-4.33(4H,m),4.37-4.40(1H,m),4.64-4.67(1H,m),5.72-5.73(1H,m),6.30-6.32(1H,m),6.35-6.38(1H,m),6.72-6.73(1H,m),6.93-6.95(1H,m),7.07-7.10(1H,m),7.16-7.20(1H,m),7.32-7.41(3H,m).

### Example 50

Ethyl 2-(1-{4-((4R,6S)8-chloro-6-(2,3-dimethoxyphenyl)-4H,6H-pyrrolo[1,2-a][4,1]benzoxazepin-4-yl]-3-hydroxybutanoyl}-4-piperidinyl)acetate

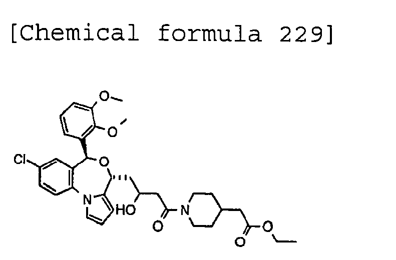

4-[(4R,6S)8-Chloro-6-(2,3-dimethoxyphenyl)-4H,6H-pyrrolo[1,2-a][4,1]benzoxazepin-4-yl]-3-hydroxybutanoic acid (isomer A) (75.3 mg) and ethyl 2-(4-piperidinyl)acetate (36.6 mg) were treated in a similar manner to that employed for the synthesis of ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate to give the title compound (75.4 mg).
MS (ESI) m/z : 611.8 (M⁺+H).
¹H-NMR(CDCl₃)δ:1.10-1.21(2H,m), 1.24-1.28 (3H,m), 1.76-1.79(2H,m),2.00-2.06(1H,m),2.16-2.26(3H,m),2.38-2.45(1H,m),2.54-2.67(2H,m),2.97-3.05(1H,m),3.42(3H,s),3.78-3.83(1H,m),3.84(3H,s),4.09-4.17(2H,m),4.37-4.44(1H,m),4.49-4.52(1H,m),4.59-4.64(1H,m),4.71-4.74(1H,m),5.73-5.74(1H,m),6.28-6.30(1H,m),6.35-6.37(1H,m),6.71-6.71(1H,m),6.93-6.95(1H,m),7.07-7.09(1H,m),7.15-7.20(1H,m),7.31-7.36(3H,m).

### Example 51

### Ethyl 2-(1-{4-[(4R,6S)8-chloro-6-(2,3-dimethoxyphenyl)-4H,6H-pyrrolo[1,2-a][4,1]benzoxazepin-4-yl]-3-hydroxybutanoyl}-4-piperidinyl)acetate

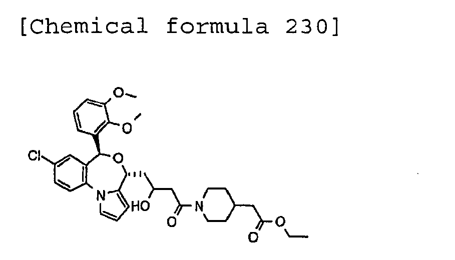

In a similar manner to that employed for the synthesis of ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate, 4-[(4R,6S)8-Chloro-6-(2,3-dimethoxyphenyl)-4H,6H-pyrrolo[1,2-a] [4,1]benzoxazepin-4-yl]-3-hydroxybutanoic acid (isomer B) (60.0 mg) was treated while using ethyl 2-(4-piperidinyl)acetate (29.2 mg) to give the title compound (63.8 mg).
MS(ESI)m/z:611.8(M⁺+H).
¹H-NMR(CDCl₃)δ:1.10-1.19(2H,m),1.26(3H,t,J=7.1Hz),1.72-1.79(2H,m),1.98-2.05(1H,m),2.20-2.27(3H,m),2.53-2.59(3H,m),2.96-3.04(1H,m),3.42(3H,s),3.79-3.82(1H,m),3.85(3H,s),4.10-4.16(2H,m),4.16-4.23(1H,m),4.35-4.40(1H,m),4.55-4.62(1H,m),4.65-4.68(1H,m),5.71-5.72(1H,m),6.29-6.31(1H,m),6.35-6.38(1H,m),6.72-6.72(1H,m),6.92-6.95(1H,m),7.07-7.09(1H,m),7.14-7.18(1H,m),7.33-7.40(3H,m).

### Example 52

Ethyl 2-(4-{4-[(4R,6S)8-chloro-6-(2,3-dimethoxyphenyl)-4H,6H-pyrrolo[1,2-a] [4,1]benzoxazepin-4-yl]-3-hydroxybutanoyl}-2-oxo-4-piperazinyl)acetate

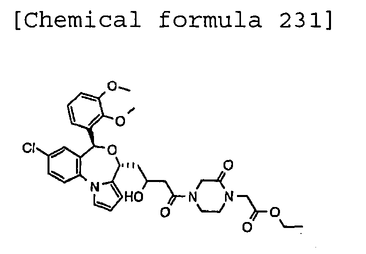

In a similar manner to that employed for the synthesis of ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate, 4-[(4R,6S)8-Chloro-6-(2,3-dimethoxyphenyl)-4H,6H-pyrrolo[1,2-a][4,1]benzoxazepin-4-yl]-3-hydroxybutanoic acid (isomer B) (60.0 mg) was treated while using ethyl 2-(2-oxo-4-piperazinyl)acetate hydrochloride (43.8 mg) to give the title compound (83.6 mg).
MS(ESI)m/z:627(M⁺+H).
¹H-NMR(CDCl₃)δ:1.27-1.29(3H,m),2.22-2.36(2H,m),2.47-2.67(2H,m),3.37-3.40(1H,m),3.43(3H,s),3.43-3.46(1H,m),3.74-3.77(1H,m),3.85(3H,s),3.87-3.91(1H,m),4.09-4.23(6H,m),4.42-4.47(1H,m),4.66-4.74(1H,m),5.72-5.74(1H,m),6.30-6.32(1H,m),6.36-6.38(1H,m),6.72-6.74(1H,m),6.93-6.95(1H,m),7.08-7.10(1H,m),7.18-7.22(1H,m),7.34-7.39(3H,m).

### Example 53

2-(4-{4-[(4R,6S)8-Chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetic acid

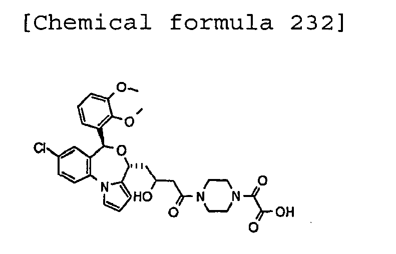

Ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate (88.8 mg) was dissolved in methanol-water-tetrahydrofuran (1:0.5:1, 2.5 mL). To the resulting solution was added potassium carbonate (58.8 mg). The resulting mixture was stirred overnight at room temperature. After addition of acetic acid (0.0244 mL), the resulting mixture was diluted with chloroform and washed with saturated brine. The organic layer was dried over anhydrous sodium sulfate. The filtrate was then concentrated. The residue thus obtained was purified by preparative TLC (lower layer of methanol:chloroform:water=3:7:1) to give the title compound (28.7 mg).
MS(ESI)m/z:598(M+H)⁺.
¹H-NMR(CD₃OD)δ:2.04-2.11(1H,m),2.25-2.33(1H,m),2.57-2.63(1H,m),2.65-2.75(1H,m),3.37(3H,s),3.46-3.71(8H,m),3.84(3H,s),4.45-4.52(1H,m),4.64-4.66(1H,m),5.65(1H,s),6.32-6.37(2H,m),6.63-6.63(1H,m),7.05-7.09(1H,m),7.20-7.25(2H,m),7.36-7.37(1H,m),7.42-7.50(2H,m).

### Example 54

### 2-(4-{4-[(4R,6S)-8-Chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetic acid

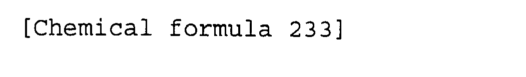

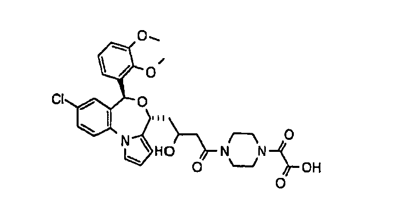

In a similar manner to that employed for the synthesis of 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetic acid, ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-1-piperazinyl)-2-oxoacetate (101 mg) and potassium carbonate (66.9 mg) were treated to give the title compound (39.7 mg).
MS (ESI) m/z : 598 (M⁺+H) .
¹H-NMR(CD₃OD)δ:2.23-2.30(2H,m),2.65-2.68(2H,m),3.37(3H,s),3.48-3.61(8H,m),3.85(3H,s),4.26-4.33(1H,m),4.64-4.66(1H,m),5.64(1H,s),6.37-6.43(2H,m),6.63-6.65(1H,m),7.06-7.09(1H,m),7.20-7.24(2H,m),7.30-7.32(1H,m),7.44-7.51(2H,m).

### Example 55

### 1-{4-[(4R,6S)-8-Chloro-6-(2,3-dimethoxyphenyl)-4H,6H-pyrrolo[1,2-a][4,1]benzoxazepin-4-yl]-3-hydroxybutanoyl}-3-azetidine carboxylic acid

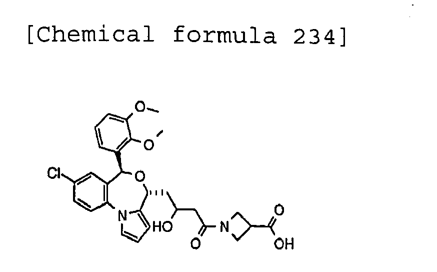

Methyl 1-{4-[(4R,6S)8-chloro-6-(2,3-dimethoxyphenyl)-4H,6H-pyrrolo[1,2-a][4,1]benzoxazepin-4-yl]-3-hydroxybutanoyl}-3-azetidine carboxylate (86.6 mg) was dissolved in methanol-water-tetrahydrofuran (1:0.5:1, 2.5 mL). Potassium carbonate (64.7 mg) was added and the resulting mixture was stirred overnight at room temperature. After neutralization of the reaction mixture with an ion exchange resin "Amberlite IR-120B", the resin was filtered out. The filtrate was concentrated. The residue thus obtained was purified by preparative TLC (lower layer of methanol:chloroform:water=3:7:1) to give the title compound (66.8 mg).
MS(ESI)m/z:541(M⁺+H)
¹H-NMR(CD₃OD)δ:2.00-2.09(1H,m),2.23-2.31(1H,m),2.32-2.37(2H,m),3.36-3.37(3H,m),3.41-3.44(1H,m),3.84-3.85(3H,m),3.98-4.10(4H,m),4.44-4.47(1H,m),4.62-4.64(1H,m),5.64-5.65(1H,m),6.32-6.38(2H,m),6.61-6.64(1H,m),7.04-7.08(1H,m),7.19-7.25(2H,m),7.37-7.39(1H,m),7.43-7.50(2H,m).

### Example 56

### 1-{4-[(4R,6S)-8-Chloro-6-(2,3-dimethoxyphenyl)-4H,6H-pyrrolo[1,2-a][4,1]benzoxazepin-4-yl]-3-hydroxybutanoyl}-3-azetidine carboxylic acid

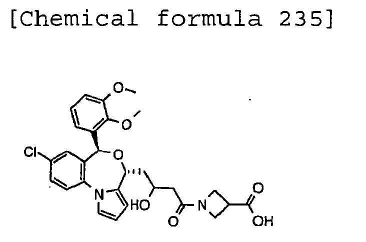

In a similar manner to that employed for the synthesis of 1-{4-[(4R,6S)-8-chloro-6-(2,3-dimethoxyphenyl)-4H,6H-pyrrolo[1,2-a][4,1]benzoxazepin-4-yl]-3-hydroxybutanoyl}-3-azetidine carboxylic acid, methyl 1-{4-[(4R,6S)8-chloro-6-(2,3-dimethoxyphenyl)-4H,6H-pyrrolo[1,2-a][4,1]benzoxazepin-4-yl]-3-hydroxybutanoyl}-3-azetidine carboxylate (98.5 mg) and potassium carbonate (73.6 mg) were treated to give the title compound (55.8 mg).
MS(ESI)m/z:541(M⁺+H).
¹H-NMR(CD₃OD)δ: .17-2.29(2H,m),2.34-2.37(2H,m),3.37(3H,s),3.42-3.44(1H,m),3.85(3H,s),3.98-4.11(4H,m),4.35-4.40(1H,m),4.61-4.63(1H,m),5.61-5.64(1H,m),6.36-6.40(2H,m),6.62-6.64(1H,m),7.06-7.08(1H,m),7.21-7.24(2H,m),7.31-7.33(1H,m),7.44-7.51(2H,m).

### Example 57

### 2-(1-{4-[(4R,6S)-8-Chloro-6-(2,3-dimethoxyphenyl)-4H,6H-pyrrolo[1,2-a][4,1]benzoxazepin-4-yl]-3-hydroxybutanoyl}-4-piperidinyl)acetic acid

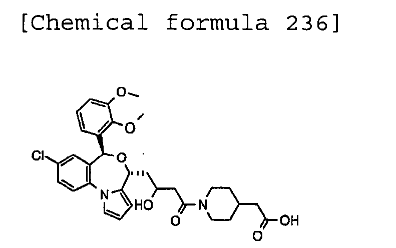

In a similar manner to that employed for the synthesis of 1-{4-[(4R,6S)-8-chloro-6-(2,3-dimethoxyphenyl)-4H,6H-pyrrolo[1,2-a][4,1]benzoxazepin-4-yl]-3-hydroxybutanoyl}-3-azetidine carboxylic acid, ethyl 2-(1-{4-[(4R,6S)8-chloro-6-(2,3-dimethoxyphenyl)-4H,6H-pyrrolo[1,2-a][4,1]benzoxazepin-4-yl]-3-hydroxybutanoyl}-4-piperidinyl)acetate (75.4 mg) was treated while using potassium carbonate (51.2 mg) to give the title compound (70.4 mg).
MS(ESI)m/z:581 (M⁺+H).
¹H-NMR(CDCl₃)δ:0.81-0.88(1H,m),1.11-1.22(1H,m),1.26-1.33(2H,m),1.78-1.84(2H,m),2.02-2.04(1H,m),2.16-2.22(1H,m),2.28-2.30(1H,m),2.39-2.48(1H,m),2.55-2.68(2H,m),2.97-3.05(1H,m),3.42(3H,s),3.79-3.82(1H,m),3.85(3H,s),4.50-4.53(1H,m),4.61-4.64(1H,m),4.71-4.73(1H,m),5.73-5.75(1H,m),6.28-6.31(1H,m),6.35-6.38(1H,m),6.70-6.73(1H,m),6.93-6.95(1H,m),7.08-7.09(1H,m),7.16-7.18(1H,m),7.31-7.37(3H,m).

### Example 58

### 2-(1-{4-[(4R,6S)-8-Chloro-6-(2,3-dimethoxyphenyl)-4H,6H-pyrrolo[1,2-a][4,1]benzoxazepin-4-yl]-3-hydroxybutanoyl}-4-piperidinyl)acetic acid

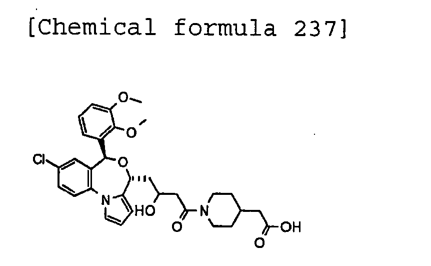

In a similar manner to that employed for the synthesis of 1-{4-[(4R,6S)-8-chloro-6-(2,3-dimethoxyphenyl)-4H,6H-pyrrolo[1,2-a][4,1]benzoxazepin-4-yl]-3-hydroxybutanoyl}-3-azetidine carboxylic acid, ethyl 2-(1-(4-[(4R,6S)8-chloro-6-(2,3-dimethoxyphenyl)-4H,6H-pyrrolo[1,2-a][4,1]benzoxazepin-4-yl]-3-hydroxybutanoyl}-4-piperidinyl)acetate (63.8 mg) was treated while using potassium carbonate (43.3 mg) to give the title compound (51 mg).
MS(ESI)m/z:581(M⁺+H).
¹H-NMR(CDCl₃)δ:0.79-0.85(1H,m),1.15-1.18(2H,m),1.60-1.62(1H,m),1.76-1.82(2H,m),2.02-2.05(1H,m),2.28-2.34(3H,m),2.53-2.59(2H,m),2.98-3.02(1H,m),3.42(3H,s),3.81-3.82(1H,m),3.85(3H,s),4.37-4.42(1H,m),4.56-4.62(1H,m),4.64-4.68(1H,m),5.71-5.73(1H,m),6.29-6.31(1H,m),6.35-6.38(1H,m),6.72-6.72(1H,m),6.92-6.95(1H,m),7.08-7.09(1H,m),7.15-7.19(1H,m),7.32-7.41(3H,m).

### Example 59

2-(4-{4-[(4R,6S)-8-Chloro-6-(2,3-dimethoxyphenyl)-4H,6H-pyrrolo[1,2-a][4,1]benzoxazepin-4-yl]-3-hydroxybutanoyl}-2-oxo-4-piperazinyl)acetic acid

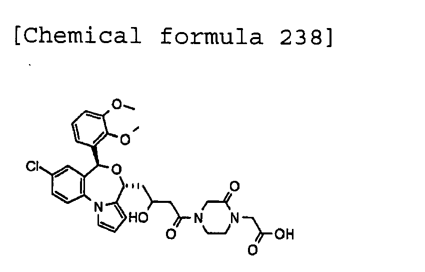

In a similar manner to that employed for the synthesis of 1-{4-[(4R,6S)-8-chloro-6-(2,3-dimethoxyphenyl)-4H,6H-pyrrolo[1,2-a][4,1]benzoxazepin-4-yl]-3-hydroxybutanoyl}-3-azetidine carboxylic acid, ethyl 2-(4-{4-[(4R,6S)8-chloro-6-(2,3-dimethoxyphenyl)-4H,6H-pyrrolo[1,2-a][4,1]benzoxazepin-4-yl]-3-hydroxybutanoyl}-2-oxo-4-piperazinyl)acetate (83.6 mg) was treated while using potassium carbonate (55.4 mg) to give the title compound (34.3 mg).
MS(ESI)m/z:596(M⁺-H).
¹H-NMR(CD₃OD)δ:2.20-2.33(2H,m),2.62-2.70(2H,m),3.35-3.38(3H,m),3.41-3.44(2H,m),3.81-3.84(2H,m),3.84-3.86(3H,m),3.96-3.99(2H,m),4.16-4.24(2H,m),4.28-4.33(1H,m),4.62-4.65(1H,m),5.62-5.64(1H,m),6.36-6.42(2H,m),6.62-6.64(1H,m),7.05-7.09(1H,m),7.19-7.24(2H,m),7.28-7.33(1H,m),7.42-7.51(2H,m).

### Example 60

### Methyl 3-[(4R,6S)-8-chloro-6-(2,3-dimethoxyphenyl)-4H,6H-pyrrolo[1,2-a][4,1]benzoxazepin-4-yl]propanoate

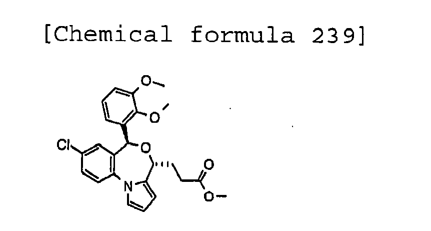

3-((4S,6S)-8-chloro-6-(2,3-dimethoxyphenyl)-4H,6H-pyrrolo(1,2-a)(4,1)benzoxazepin-4-yl)propanoic acid (200 mg) was dissolved in N,N-dimethylformamide (4 mL). Potassium carbonate (96.9 mg) and methyl iodide (0.058 ml) were added and the resulting mixture was stirred at room temperature for 3 hours. The reaction mixture was concentrated. The residue thus obtained was diluted with ethyl acetate and then washed with saturated brine. The organic layer was dried over anhydrous sodium sulfate. The filtrate was then concentrated. The residue thus obtained was purified by silica gel column (ethyl acetate:hexane=1:1) to give the title compound (180 mg).
MSm/z:442 (M⁺+H).
¹H-NMR(CDCl₃)δ:2.29-2.47(2H,m),2.54-2.62(1H,m),2.66-2.74(1H,m),3.43(3H,s),3.66(3H,s),3.85(3H,s),4.36-4.39(1H,m),5.70(1H,s),6.33-6.34(1H,m),6.38-6.38(1H,m),6.70-6.72(1H,m),6.94-6.96(1H,m),7.08-7.10(1H,m),7.16-7.21(1H,m),7.28-7.41(3H,m).

### Example 61

### Methyl 3-[(4R,6S)-1,8-dichloro-6-(2,3-dimethoxyphenyl)-4H,6H-pyrrolo[1,2-a][4,1]benzoxazepin-4-yl]propanoate

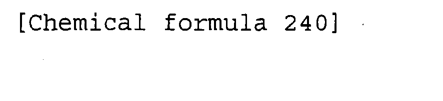

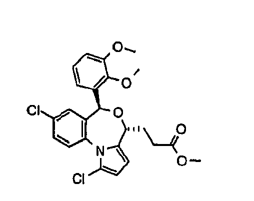

Methyl 3-[(4R,6S)-8-chloro-6-(2,3-dimethoxyphenyl)-4H,6H-pyrrolo[1,2-a][4,1]benzoxazepin-4-yl]propanoate (91.9 mgl) was dissolved in tetrahydrofuran (2 mL). N-chlorosuccinimide (29.2 mg) was added and the resulting mixture was stirred overnight at room temperature. Water was added to the reaction mixture, followed by extraction with ethyl acetate. The organic layer was dried over anhydrous sodium sulfate. The filtrate was then concentrated. The residue thus obtained was purified by preparative TLC (ethyl acetate:n-hexane=1:4) to give the title compound (89.4 mg).
MSm/z: 275 (M⁺+H).
¹H-NMR(CDCl₃)δ:2.27-2.33(1H,m),2.33-2.41(1H,m),2.50-2.60(1H,m),2.62-2.68(1H,m),3.42(3H,s),3.66(3H,s),3.85(3H,s),4.19-4.23(1H,m),5.61(1H,s),6.27(1H,d,J=3.9Hz),6.29(1H,d,J=3.9Hz) ,6.75(1H,d,J=2.4Hz),6.93-6.96(1H,m),7.16-7.20(1H,m),7.24-7.26(1H,m),7.40(1H,dd,J=8.5,2.4Hz),7.49(1H,d,J=8.5Hz).

### Example 62

### 3-[(4R,6S)-1,8-Dichloro-6-(2,3-dimethoxyphenyl)-4H,6H-pyrrolo[1,2-a][4,1]benzoxazepin-4-yl]propanoic acid

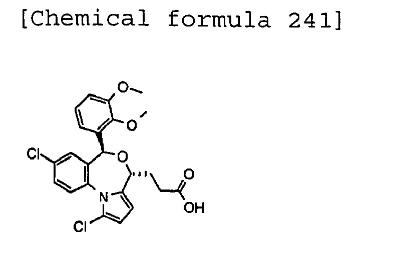

Methyl 3-[(4R,6S)-1,8-dichloro-6-(2,3-dimethoxyphenyl)-4H,6H-pyrrolo[1,2-a][4,1]benzoxazepin-4-yl]propanoate (89.4 mg) was dissolved in methanol-water-tetrahydrofuran (1:0.5:1, 2.5 mL). Potassium carbonate (77.8 mg) was added and the resulting mixture was stirred overnight at room temperature. After neutralization of the reaction mixture with an ion exchange resin "Amberlite IR-120B", the resin was filtered out. The filtrate was concentrated to give the title compound (89.9 mg).
¹H-NMR(CDCl₃)δ:1.99-2,16 (3H, m),2.26-2.36(1H,m),3.31(3H,s),3.74(3H,s),3.99-4.03(1H,m),5.49(1H,s),6.06-6.06(1H,m),6.13-6.15(1H,m),6.66-6.66(1H,m),6.74-6.76(1H,m),6.97-7.01(1H,m),7.18-7.20(1H,m),7.28-7.28(1H,m),7.34-7.36(1H,m).

### Example 63

### Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)acetate

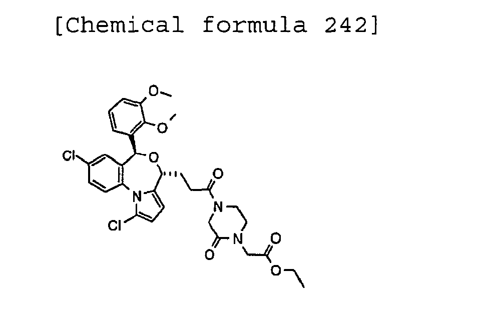

3-[(4R,6S)-1,8-Dichloro-6-(2,3-dimethoxyphenyl)-4H,6H-pyrrolo[1,2-a][4,1]benzoxazepin-4-yl]propanoic acid (89.9 mg, 0.194 mmol) and ethyl 2-(2-oxo-4-piperazinyl)acetate hydrochloride (52.0 mg) were dissolved in dichloromethane (3 mL). To the resulting solution were added 1-ethyl-3-(3-dimethylaminopropyl)carbodiimide hydrochloride (44.7 mg), 1-hydroxybenzotriazole (8.9 mg), and triethylamine (0.0325 mL). The resulting mixture was stirred at room temperature for 3 hours. The reaction mixture was diluted with chloroform and washed with saturated brine. The organic layer was dried over anhydrous sodium sulfate. The filtrate was then concentrated. The residue thus obtained was purified by preparative TLC (methanol:chloroform=1:10) to give the title compound (105 mg).
MS (ESI)m/z : 630 (M+H)+.
¹H-NMR(CDCl₃)δ:1.28(3H,t,J=6.2Hz), 2.34-2.43(2H,m),2.49-2.70(2H,m),3.39-3.44(5H,m),3.83-3.89(5H,m),4.13-4.29(9H,m),5.60-5.63(1H,m),6.28-6.30(2H,m),6.74-6.77(1H,m),6.94-6.96(1H,m),7.16-7.26(2H,m),7.39-7.41(1H,m),7.48-7.51(1H,m).

### Example 64

### 2-(4-{3-[(4R,6S)-8-Chloro-6-(2,3-dimethoxyphenyl)-4H,6H-pyrrolo[1,2-a][4,1]benzoxazepin-4-yl]propanoyl}-2-oxo-1-piperazinyl)acetic acid

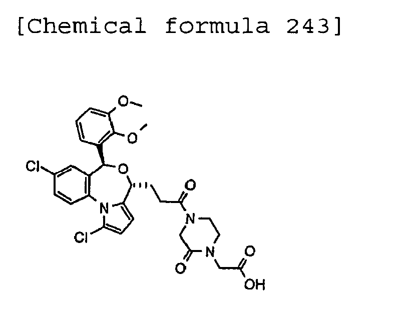

Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)acetate (105 mg) was dissolved in methanol-water-tetrahydrofuran (1:0.5:1, 2.5 mL). Potassium carbonate (69.0 mg) was added and the resulting mixture was stirred overnight at room temperature. After neutralization of the reaction mixture with an ion exchange resin "Amberlite IR-120B", the resin was filtered out. The filtrate was concentrated. The residue thus obtained was purified by preparative TLC (lower layer of methanol:chloroform:water=3:7:1) to give the title compound (89.5 mg).
MS(ESI)m/z:603(M⁺+H).
¹H-NMR(CDCl₃) δ:2.22-2 .53 (4H,m), 3.36 (3H,s), 3.59-3.61(2H,m),3.70(3H,s),3.78-3.81(4H,m),4.08-4.11(2H,m),5.55-5.57(1H,m),6.22-6.24(2H,m),6.69-6.72(1H,m),6.88-6.90(1H,m),7.10-7.13(1H,m),7.21-7.23(1H,m),7.32-7.34(1H,m),7.43-7.45(1H,m).

### Example 65

Pyruvic acid {[2-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)acetyl]oxy}methyl ester

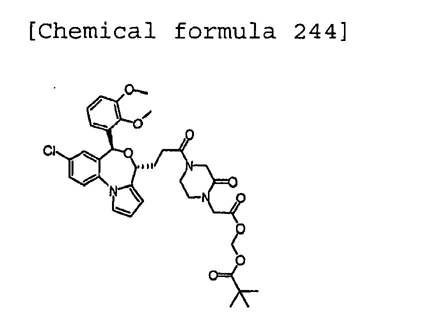

2-(4-{3-[(4R,6S)-8-Chloro-6-(2,3-dimethoxyphenyl)-4H,6H-pyrrolo[1,2-a][4,1]benzoxazepin-4-yl]propanoyl}-2-oxo-1-piperazinyl)acetic acid (37 mg) was dissolved in N,N-dimethylformamide (1 mL). To the resulting solution were added triethylamine (10 µl), benzyltriethylammonium chloride (13 mg) and pivaloyloxymethyl chloride (12 µl). The resulting mixture was stirred overnight at room temperature. After the reaction mixture was concentrated under reduced pressure, ethyl acetate and a 10% aqueous solution of citric acid were added to the residue and the layers are separated. The organic layer was dried over anhydrous sodium sulfate. The solvent was distilled off under reduced pressure. The residue was purified by thin layer silica gel chromatography (chloroform:methanol=20:1) to give the title compound (33 mg).
MS(FAB)m/z:682(M⁺+H).
¹H-NMR(CDCl₃)δ:1.22(9H,s),2.38-2.47(2H,m),2.51-2.76(2H,m),3.37-3.48(5H,m),3.75-3.91(5H,m),4.10-4.31(4H,m),4.41(1H,t,J=6.3Hz),5.69-5.73(1H,m),5.79(2H,s),6.33-6.40(2H,m),6.71(1H,s),6.93-6.98(1H,m),7.09(1H,s),7.15-7.33(2H,m),7.35(2H,s).

### Referential Example 30

### Ethyl 2-(2-oxo-1,4-diazepan-1-yl)acetate hydrochloride

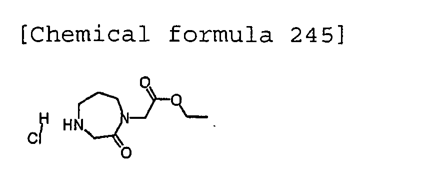

In N,N-dimethylformamide (15 mL) was dissolved tert-butyl 3-oxo-1,4-diazepane-1-carboxylate (321 mg) synthesized in the process as described in Document (W09614844). Sodium hydride (72 mg) was added to the resulting solution, followed by stirring overnight at room temperature. Ethyl 2-bromoacetate (200 µl) was added and the mixture was stirred overnight at room temperature. After concentration of the reaction mixture under reduced pressure, water and ethyl acetate were added to the residue and the layers separated. The organic layer was dried over anhydrous sodium sulfate. The solvent was then distilled off under reduced pressure. The residue was purified by silica gel column chromatography (hexane:ethyl acetate=3:1) to give a colorless caramel. The resulting substance was dissolved in chloroform (1 mL). To the resulting solution was added a 4N hydrochloric acid-dioxane solution (30 mL). The resulting mixture was allowed to stand at room temperature for 30 minutes. The reaction mixture was concentrated under reduced pressure to give the title compound (210 mg).
MS (FAB) m/z: 201 (M⁺+H)
¹H-NMR(CDCl₃)δ:1.19(3H,t,J=7.1Hz),1.95(2H,brs),3.18-3.72(4H,m),3.91(2H,brs),4.06-4.20(4H,m),9.68(2H,brs).

### Example 66

### Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1,4-diazepan-1-yl)acetate

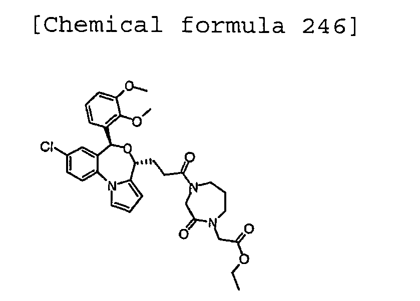

2-(4-{3-[(4R,6S)-8-Chloro-6-(2,3-dimethoxyphenyl)-4H,6H-pyrrolo[1,2-a][4,1]benzoxazepin-4-yl]propanoic acid (100 mg) was dissolved in N,N-dimethylformamide (10 mL). To the resulting solution were added 1-ethyl-3-(3-dimethylaminopropyl)carbodiimide (67 mg), ethyl 2-(2-oxo-1,4-diazepan-1-yl)acetate hydrochloride (83 mg), 1-hydroxybenzotriazole monohydrate (36 mg) and triethylamine (144 µl). The resulting mixture was stirred overnight at room temperature. After concentration of the reaction mixture under reduced pressure, water (20 mL) and dichloromethane (20 mL) were added to the residue and the layers separated. The organic layer was dried over anhydrous sodium sulfate. The solvent was then distilled off under reduced pressure. The residue was purified by silica gel column chromatography (hexane:ethyl acetate=1:2) to give the title compound (120 mg).
MS(FAB)m/z:610(M⁺+H).
¹H-NMR(CDCl₃)δ: 1.25(3H,t,J=7.0Hz), 1.88-2.00(2H,m), 2.35-2.48(2H,m),2.58-2.84(2H,m),3.37-3.53(5H,m),3.65-3.88(5H,m),4.03-4.26(6H,m),4.36-4.43(1H,m),5.69(1H,s),6.33-6.40(2H,m),6.71(1H,s),6.90-6.98(1H,m),7.06-7.10(1H,m),7.18-7.23(1H,m),7.25-7.41(3H,m).

### Example 67

### 2-(4-{3-[(4R,6S)-8-Chloro-6-(2,3-dimethoxyphenyl)-4H,6H-pyrrolo[1,2-a][4,1]benzoxazepin-4-yl]propanoyl}-2-oxo-1,4-diazepan-1-yl)acetic acid

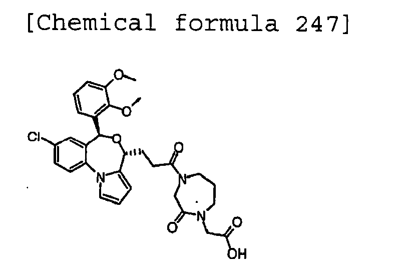

Ethyl 2-(4-{3-[(4R,6S)-8-chloro-6-(2,3-dimethoxyphenyl)-4H,6H-pyrrolo[1,2-a][4, 1] benzoxazepin-4-yl]propanoyl}-2-oxo-1,4-diazepan-1-yl)acetate (115 mg) was dissolved in methanol (5 mL). To the resulting solution were added tetrahydrofuran (0.5 mL), water (2 mL), and anhydrous potassium carbonate (76 mg). The resulting mixture was heated and stirred overnight at 65°C. After concentration of the reaction mixture under reduced pressure, chloroform and a 10% aqueous citric acid solution were added to the residue and the layers separated. The organic layer was dried over anhydrous sodium sulfate and then the solvent was distilled off under reduced pressure, whereby the title compound (107 mg)..
MS (FAB) m/z : 582 (M⁺+H) .
¹H-NMR(CDCl₃)δ:1.82-1.98(2H,m),2.32-2.46(2H,m),2.55-2.86(2H,m),3.35-3.50(5H,m),3.60-3.87(5H,m),4.02-4.42(5H,m),5.68(1H,s),6.36(2H,s),6.71(1H,s),6.90-6.96(1H,m),7.07(1H,s),7.15-7.41(4H,m).

### Example 68

### 4-{3-[(4R,6S)-8-Chloro-6-(2,3-dimethoxyphenyl)-4H,6H-pyrrolo[1,2-a][4,1]benzoxazepin-4-yl]propanoyl}-2,6-piperazinedione

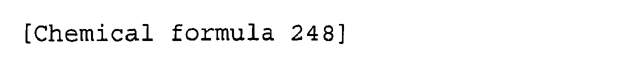

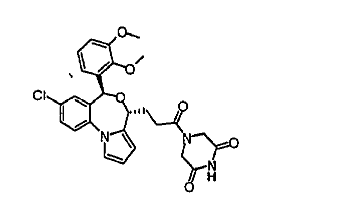

2-(4-{3-[(4R,6S)-8-chloro-6-(2,3-dimethoxyphenyl)-4H,6H-pyrrolo[1,2-a][4,1]benzoxazepin-4-yl]propanoic acid (100 mg) was dissolved in N,N-dimethylformamide (2 mL). To the resulting solution were added 1-ethyl-3-(3-dimethylaminopropyl)carbodiimide hydrochloride (67 mg), 2,6-piperazinedione hydrochloride (55 mg) synthesized in accordance with the process described in the document (J.Chem.Soc.Perkin I,1009-1014(1972)), 1-hydroxybenzotriazole monohydrate (36 mg) and triethylamine (49 µl). The resulting mixture was stirred at room temperature for 3 days. After concentration of the reaction mixture under reduced pressure, water (20 mL) and dichloromethane (20 mL) were added to the residue and the layers separated. The organic layer was dried over anhydrous sodium sulfate and then the solvent was distilled off under reduced pressure. The residue was purified by silica gel column chromatography (hexane:ethyl acetate=1:2) to give the title compound (102 mg).
MS(FAB)m/z:524(M⁺+H).
¹H-NMR(CDCl₃)δ:2.39-2.47(2H,m),2.60-2.78(2H,m),3.43(3H,s),3.86(3H,s),4.27-4.48 (5H,m), 5.71 (1H,s), 6.33-6.35(1H,m),6.39(1H,t,J=3.2Hz),6.72(1H,d,J=2.0Hz),6.96(1H,dd ,J=7.8,2.0Hz),7.09-7.11(1H,m),7.17-7.37(4H,m),7.96(1H,s).

### Example 69

### Benzyl 2-(4-{3-[(4R,6S)-8-chloro-6-(2,3-dimethoxyphenyl)-4H,6H-pyrrolo[1,2-a][4,1]benzoxazepin-4-yl]propanoyl}-2,6-piperazinyl)acetate

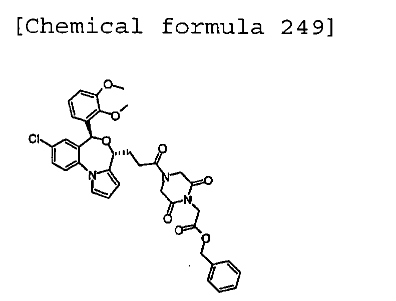

4-{3-[(4R,6S)-8-Chloro-6-(2,3-dimethoxyphenyl)-4H,6H-pyrrolo[1,2-a][4,1]benzoxazepin-4-yl]propanoyl}-2,6-piperazinedione (100 mg) was dissolved in N,N-dimethylformamide (2 mL). To the resulting solution was added sodium hydride (8 mg) and the resulting mixture was stirred at room temperature for 30 minutes. Benzyl 2-bromoacetate (36 µl) was added and the resulting mixture was stirred overnight at room temperature. After concentration of the reaction mixture under reduced pressure, water and ethyl acetate were added to the residue and the layers separated. The organic layer was dried over anhydrous sodium sulfate and then, the solvent was distilled off under reduced pressure. The residue was purified by silica gel column chromatography (hexane:ethyl acetate=1:2) to give the title compound (94 mg).
MS(EI)m/z:671(M⁺).
¹H-NMR(CDCl₃)δ:2.38-2.46(2H,m),2.58-2.77(2H,m),3.43(3H,s),3.85(3H,s),4.38-4.58(7H,m),5.17(2H,s),5.71(1H,s),6.34(1H,s),6.37-6.40(1H,m),6.72(1H,s),6.96(1H,d,J=7.1Hz),7.09(1H,s),7.17-7.22(1H,m),7.24-7.41(8H,m).

### Example 70

### 2-(4-{3-[(4R,6S)-8-Chloro-6-(2,3-dimethoxyphenyl)-4H,6H-pyrrolo[1,2-a][4,1]benzoxazepin-4-yl]propanoyl}-2,6-piperazinyl)acetic acid

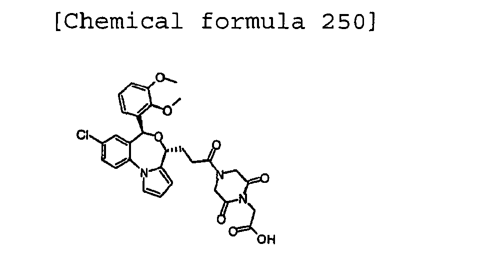

Benzyl 2-(4-{3-[(4R,6S)-8-chloro-6-(2,3-dimethoxyphenyl)-4H,6H-pyrrolo[1,2-a][4,1]benzoxazepin-4-yl]propanoyl}-2,6-piperazinyl)acetate (88 mg) was dissolved in ethyl acetate (2 mL). To the resulting solution was added 10% palladium-carbon (water content: 50%) (40 mg) and the resulting mixture was stirred overnight at room temperature under a hydrogen gas stream. The reaction mixture was filtered and the filtrate was concentrated under reduced pressure to give the title compound (74 mg).
MS(EI)m/z:581(M⁺).
¹H-NMR(CDCl₃)δ:2.38-2.47(2H,m),2.60-2.79(2H,m),3.43(3H,s),3.85(3H,s),4.36-4.58(7H,m),5.71(1H,s),6.32-6.41(2H,m),6.72(1H,s),6.96(1H,d,J=7.6Hz),7.06-7.42(4H,m).
Elemental analysis for: C₂₉H₂₈ClN₃O₈·1.0H₂O
Calculated: C,58.05;H,5.04;N,7.00.
Found: C,58.34;H,4.95;N,6.90.

### Referential Example 31

### Ethyl ((3R)-3-amino-2-oxopiperidin-1-yl)acetate hydrochloride

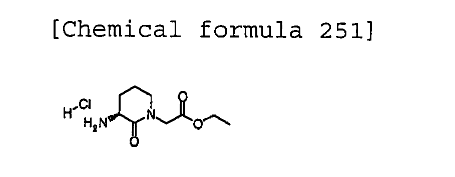

(3R)-2-Oxo(3-aminotrityl)piperidine (3.56 g) synthesized in accordance with the process described in the documents (Synthesis, 614-616(1978);Synthesis, 417-419(1991)) in N,N-dimethylformamide (20 mL). Silver oxide (3.48 g), ethyl bromoacetate (1.66 mL) and tetra-n-butylammonium iodide (3.69 g) were added, followed by stirring overnight at room temperature. The reaction mixture was filtered through celite. The filtrate was then concentrated under reduced pressure. The residue was purified by silica gel chromatography (hexane:ethyl acetate=6:1). The purified product was then dissolved in a 4N hydrochloric acid-dioxane solution (12.0 mL) and the resulting solution was stirred at room temperature for 30 minutes. After concentration of the reaction mixture under reduced pressure, diethyl ether and water were added to the residue and the layers separated. The water layer was neutralized with a saturated aqueous solution of sodium hydrogen carbonate and then, a salt was added to saturation. The resulting solution was then extracted with chloroform. The organic layer was dried over anhydrous sodium sulfate. The solvent was distilled off under reduced pressure. To the residue was added a 1N hydrochloric acid-ethanol solution (20.0 mL). The resulting mixture was concentrated under reduced pressure to give the title compound (359 mg).
MS(FAB)m/z:201(M⁺+H).
¹H-NMR(DMSO-d₆)δ:1.21(3H,t,J=7.2Hz),1.71-1.98(3H,m)2.16-2.24(1H,m),3.34-3.41(2H,m),3.86-3.95(1H,m),4.00-4.16(4H,m),8.37(3H,s).

### Example 71

### Ethyl2-[(3R)-3-({3-[(4R,6S)-8-chloro-6-(2,3-dimethoxyphenyl) -4H, 6H-pyrrolo [1, 2-a] [4,1]benzoxazepin-4-yl]propanoyl}amino)-2-oxopiperidinyl]acetate

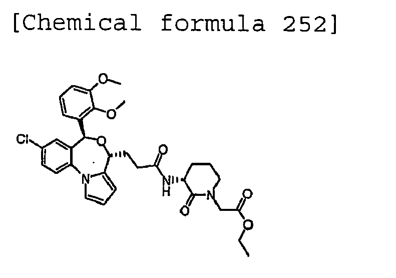

2-(4-{3-[(4R,6S)-8-Chloro-6-(2,3-dimethoxyphenyl)-4H,6H-pyrrolo[1,2-a][4,1]benzoxazepin-4-yl]propanoic acid (100 mg) was dissolved in N,N-dimethylformamide (2 mL). To the resulting solution were added 1-ethyl-3-(3-dimethylaminopropyl)carbodiimide hydrochloride (134 mg), ethyl ((3R)-3-amino-2-oxopiperidine-1-yl)acetated hydrochloride (72 mg), 1-hydroxybenzotriazole monohydrate (36 mg) and triethylamine (192 µl). The resulting mixture was stirred at room temperature for 13 hours. After concentration of the reaction mixture under reduced pressure, water (20 mL) and dichloromethane (20 mL) were added to the residue and the layers separated. The organic layer was dried over anhydrous sodium sulfate. The solvent was then distilled off under reduced pressure. The residue was purified by silica gel column chromatography (hexane:ethyl acetate=1:2) to give the title compound (92 mg).
MS(EI)m/z:609(M⁺)
¹H-NMR(CDCl₃)δ:1.29(3H,t,J=7.2Hz),1.41-1.54(1H,m),1.89-2.00(2H,m),2.34-2.66(SH,m),3.35-3.45(5H,m),3.79-3.89(4H,m),4.16-4.39(5H,m),5.70(1H,s),6.32-6.38(2H,m),6.45-6.50(1H,m),6.71(1H,s),6.92-6.96(1H,m),7.06-7.09(1H,m),7.19(1H,t,J=8.1Hz),7.28-7.40(3H,m).

### Example 72

### 2-[(3R)-3-({3-[(4R,6S)-8-Chloro-6-(2,3-dimethoxyphenyl)-4H,6H-pyrrolo[1,2-a][4,1]benzoxazepin-4-yl]propanoyl}amino)-2-oxopiperidinyl]acetic acid

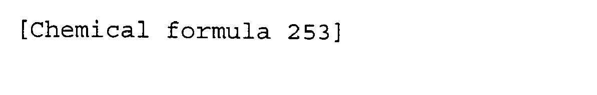

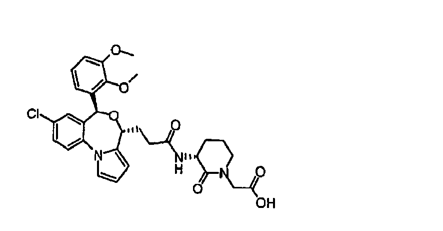

Ethyl 2-[(3R)-3-({3-[(4R,6S)-8-chloro-6-(2,3-dimethoxyphenyl)-4H,6H-pyrrolo[1,2-a][4,1]benzoxazepin-4-yl]propanoyl}amino)-2-oxopiperidinyl]acetate (88 mg) was dissolved in methanol (5 mL). To the resulting mixture were added tetrahydrofuran (0.5 mL), water (2 mL) and anhydrous potassium carbonate (60 mg). The resulting mixture was heated and stirred at 65°C for on hour. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure, whereby the title compound (77 mg).
MS(EI)m/z:581(M⁺).
¹H-NMR(CDCl₃)δ:1.47-1.59(1H,m),1.86-1.98(2H,m),2.29-2.52(4H,m),2.56-2.67(1H,m),3.29-3.52(5H,m),3.78-3.89(4H,m),4.22-4.41(3H,m),5.70(1H,s),6.32-6.39(2H,m),6.69-6.78(2H,m),6.94(1H,d,J=7.8Hz),7.08(1H,s),7.18(1H,t,J=7.8Hz) ,7.28-7.40(3H,m).

### Example 73

### Ethyl 3-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)propanoate

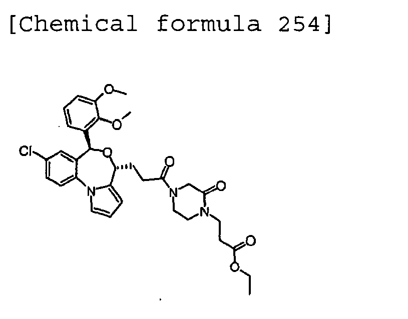

2-(4-{3-[(4R,6S)-8-Chloro-6-(2,3-dimethoxyphenyl)-4H,6H-pyrrolo[1,2-a][4,1]benzoxazepin-4-yl]propanoic acid (100 mg) was dissolved in N,N-dimethylformamide (10 mL).
To the resulting solution were added 1-ethyl-3-(3-dimethylaminopropyl)carbodiimide hydrochloride (67 mg), ethyl 2-oxopiperazinepropionate hydrochloride (66 mg) prepared in accordance with the process described in the patent (WO01030780), 1-hydroxybenzotriazole monohydrate (36 mg), and triethylamine (65 µl). The resulting mixture was stirred at room temperature for 13 hours. After concentration of the reaction mixture under reduced pressure, water (20 mL) and dichloromethane (20 mL) were added to the residue and the layers separated. The organic layer was dried over anhydrous sodium sulfate. The solvent was distilled off under reduced pressure. The residue was purified by silica gel column chromatography (hexane:ethyl acetate=1:2) to give the title compound (102 mg).
MS(FAB)m/z:610(M⁺+H).
¹H-NMR(CDCl₃)δ:1.26(3H,t,J=7.2Hz),2.27-2.76(6H,m),3.25-3.49(5H,m),3.59-3.89(7H,m),4.04-4.23(4H,m),4.37-4.43(1H,m),5.70(1H,s),6.31-6.40(2H,m),6.71(1H,s),6.95(1H,d,J=7.6Hz),7.09(1H,s),7.15-7.41(4H,m).

### Example 74

### 3-(4-{3-[(4R,6S)-8-Chloro-6-(2,3-dimethoxyphenyl)-4H,6H-pyrrolo[1,2-a][4,1]benzoxazepin-4-yl]propanoyl}-2-oxo-1-piperazinyl)propanoic acid

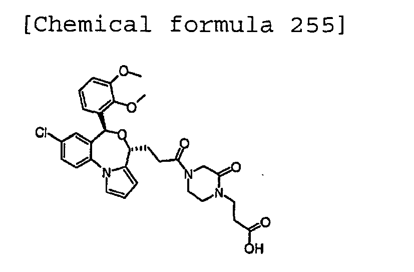

Ethyl 3-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)propanoate (97 mg) was dissolved in methanol (20 mL). To the resulting solution were added tetrahydrofuran (1 mL), water (2 mL), and anhydrous potassium carbonate (66 mg). The resulting mixture was heated and stirred at 65°C for 1 hour. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (83 mg).
MS(FAB)m/z:582(M⁺+H).
¹H-NMR(CDCl₃)δ:2.32-2.78(6H,m),3.43(5H,brs), 3.59-3.91(7H,m),4.11-4.27(2H,m),4.40(1H,br s),5.70(1H,s),6.31-6.42(2H,m),6.71(1H,s),6.90-7.00(1H,m),7.09(1H,s),7.17-7.44(4H,m).
Elemental analysis for: C₃₀H₃₂ClN₃O₇·1.0H₂O
Calculated: C,60.05;H,5.71;N,7.00.
Found: C,60.27;H,5.49;N,6.90.

### Example 75

### Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)acetate

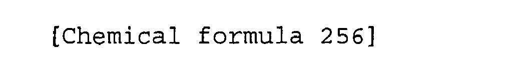

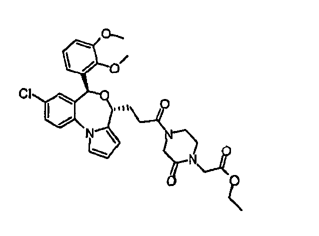

2-(4-{3-[(4R,6S)-8-Chloro-6-(2,3-dimethoxyphenyl)-4H,6H-pyrrolo[1,2-a][4,1]benzoxazepin-4-yl]propanoic acid (100 mg) was dissolved in N,N-dimethylformamide (10 mL).
To the resulting solution were added 1-ethyl-3-(3-dimethylaminopropyl)carbodiimide hydrochloride (67 mg), ethyl 2-oxopiperazineacetate hydrochloride (62 mg) synthesized in accordance with the process described in the document (WO9846591), 1-hydroxybenzotriazole monohydrate (36 mg) and triethylamine (65 µl). The resulting mixture was stirred at room temperature for 16 hours. After concentration of the reaction mixture under reduced pressure, water (20 mL) and dichloromethane (20 mL) were added to the residue and the layers separated. The organic layer was dried over anhydrous sodium sulfate. The solvent was distilled off under reduced pressure. The residue was purified by silica gel column chromatography (hexane:ethyl acetate=1:2) to give the title compound (100 mg).
MS(EI)m/z:595(M⁺).
¹H-NMR(CDCl₃)δ:1.28(3H,t,J=7.2Hz),2.37-2.47(2H,m),2.52-2.73(2H,m),3.39-3.44(5H,m),3.75-3.92(5H,m),4.11-4.30(6H,m),4.41(1H,t,J=6.3Hz),5.71(1H,s),6.33-6.40(2H,m),6.71(1H,s),6.95(1H,d,J=7.1Hz),7.09(1H,s),7.15-7.39 (4H,m) .

### Example 76

### 2-(4-{3-[(4R,6S)-8-Chloro-6-(2,3-dimethoxyphenyl)-4H,6H-pyrrolo[1,2-a][4,1]benzoxazepin-4-yl]propanoyl}-2-oxo-1-piperazinyl)acetic acid

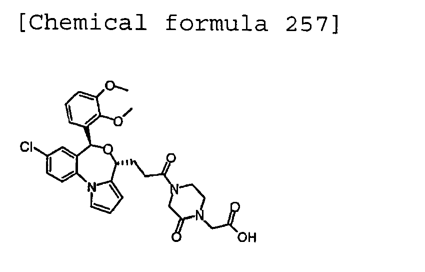

Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-2-oxo-1-piperazinyl)acetate (94 mg) was dissolved in methanol (20 mL). To the resulting solution were added tetrahydrofuran (1 mL), water (2 mL), and anhydrous potassium carbonate (65 mg). The resulting mixture was stirred overnight at 65°C. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (80 mg).
MS(FAB)m/z:568(M⁺+H).
¹H-NMR(CDCl₃)δ:2.35-2.46(2H,m),2.51-2.76(2H,m),3.36-3.46(5H,m),3.80-3.89(5H,m),4.10-4.30(4H,m),4.35-4.44(1H,m),5.70(1H,s),6.31-6.40(2H,m),6.71(1H,s),6.91-6.98(1H,m),7.08(1H,s),7.19-7.38(4H,m).

### Referential Example 32

### Methyl 4-aminocyclohexane carboxylate hydrochloride

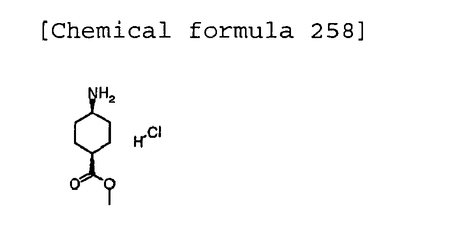

Thionyl chloride (0.761 mL) was added dropwise to methanol (20 mL) under ice cooling. Then, a solution of 4-aminocyclohexane carboxylate (0.500 g) in methanol (10 mL) was added and the resulting mixture was stirred at room temperature for 1 day. To the residue obtained by concentrating the reaction mixture under reduced pressure was added methanol, followed by concentration again. The above-described operation was performed twice. Ether was added to the residue, followed by trituration to give the title compound (0.479 g).
¹H-NMR(CDCl₃)δ:1.62-1.70 (2H,m), 1.76-1.85 (2H,m), 1.94-2.02(2H,m),2.13-2.21(2H,m),2.54-2.59(1H,m),3.27-3.33 (1H,m), 3.73 (3H, s), 8.27 (2H, br s).
MS (ESI) m/z : 158 (M⁺+H) .

### Example 77

### Methyl 4-({3-[(4R,6S)-8-chloro-6-(2,3-dimethoxyphenyl)-4H,6H-pyrrolo[1,2-a][4,1]benzoxazepin-4-yl]propanoyl}amino)cyclohexane carboxylate

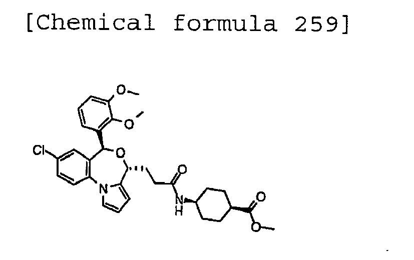

3-[(4R,6S)-8-Chloro-6-(2,3-dimethoxyphenyl)-4H,6H-pyrrolo[1,2-a][4,1]benzoxazepin-4-yl]propionic acid (0.200 g) and 1-hydroxybenzotriazole (93.1 mg) were dissolved in dichloromethane (10 mL). The resulting solution was stirred at room temperature for 10 minutes. To the reaction mixture were added methyl 4-aminocyclohexanecarboxylate hydrochloride (0.118 g) and 1-ethyl-3-(3-dimethylaminopropyl)carbodiimide hydrochloride (0.117 g), followed by stirring at room temperature for 18 hours. The residue obtained by concentrating the reaction mixture under reduced pressure was partitioned between water and dichloromethane. The water layer was extracted with dichloromethane. The organic layers were combined, washed with saturated brine, and dried over anhydrous sodium sulfate. The solvent was distilled off under reduced pressure. The residue thus obtained was purified by Yamazen medium pressure preparative chromatogram system (hexane:ethyl acetate= from 60:40 to 15:85) to give the title compound (172 mg).
MS(ESI)m/z:567(M⁺+H).
¹H-NMR(CDCl₃) δ:1.38-1.55 (2H,m), 1.56-1.74 (4H,m), 1.78-1.88(2H,m),2.35-2.56(5H,m),3.43(3H,s),3.67(3H,s),3.85(3H,s),3.86-3.91(1H,m),4.35-4.39(1H,m),5.60(1H,d,J=7.8Hz),5.72(1H,s),6.32-6.35(1H,m),6.36-6.39(1H,m),6.70-6.71(1H,m),6.94-6.98(1H,m),7.08-7.10(1H,m),7.20(1H,t,J=8.1Hz),7.27-7.37(3H,m).

### Example 78

### 4-({3-[(4R,6S)-8-Chloro-6-(2,3-dimethoxyphenyl)-4H,6H-pyrrolo[1,2-a][4,1]benzoxazepin-4-yl]propanoyl}amino)cyclohexane carboxylic acid

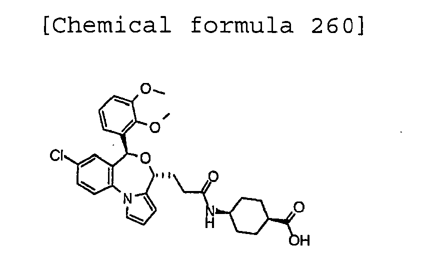

Methyl 4-({3-[(4R,6S)-8-chloro-6-(2,3-dimethoxyphenyl)-4H,6H-pyrrolo[1,2-a][4,1]-benzoxazepin-4-yl]propanoyl}amino)cyclohexane carboxylate (70.0 mg) was dissolved in methanol (5 mL). To the resulting solution were added potassium carbonate (51.2 mg) and water (5 mL). After stirring at 60°C for 3 hours and at room temperature for 18 hours, the reaction mixture was concentrated under reduced pressure. The residue was neutralized with a 1N aqueous hydrochloric acid solution and then, extracted three times with dichloromethane. The organic layers were combined, washed with saturated brine and then, dried over anhydrous sodium sulfate. The residue obtained by distilling off the solvent was triturated with hexane-ether to give the title compound (52.5 mg).
¹H-NMR(CDCl₃)δ:1.34-1.91(8H,m)2.3S-2.45(3H,m)2.49-2.57(2H,m),3.43(3H,s),3.85-3.89(1H,m),3.86(3H,s),4.36-4.40(1H,m),5.61(1H,d,J=7.8Hz),5.73(1H,s),6.32-6.35(1H,m),6.37-6.39(1H,m),6.71(1H,s),6.95(1H,d,J=8.1Hz),7.08-7.10(1H,m),7.18-7.23(1H,m),7.27-7.33(1H,m),7.34-7.38(2H,m).
IR(ATR)cm⁻¹:3382,2935,1724,1631,1490,1278,1172,1054,713.
MS(ESI)m/z:535(M⁺-H₂O+H).
Elemental analysis for: C₃₀H₃₃ClN₂O₆·0.25H₂O
Calculated: C,64.63;H,6.06;N,5.02.
Found: C,64.68;H,6.29;N,5.04.

### Example 79

### Ethyl (1S,3R)-3-({3-[(4R,6S)-8-chloro-6-(2,3-dimethoxyphenyl)-4H,6H-pyrrolo[1,2-a][4,1]benzoxazepin-4-yl]propanoyl}amino)cyclohexanecarboxylate

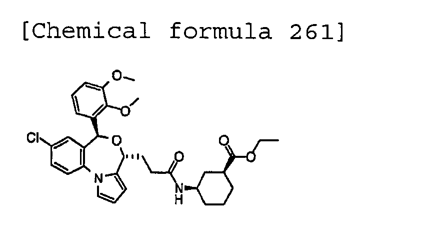

3-[(4R,6S)-8-Chloro-6-(2,3-dimethoxyphenyl)-4H,6H-pyrrolo[1,2-a][4,1]benzoxazepin-4-yl]propionic acid (0.100 g) and 1-hydroxybenzotriazole (46.5 mg) were dissolved in dichloromethane (10 mL). The resulting solution was stirred at room temperature for 10 minutes. Ethyl (1S,3R)-3-aminocyclohexane carboxylate hydrochloride (63.1 mg), N-methylmorpholine (0.0334 mL), and 1-ethyl-3-(3-dimethylaminopropyl)carbodiimide hydrochloride (58.2 mg) were added further. The resulting mixture was stirred at room temperature for 18 hours. The reaction mixture was partitioned between water and dichloromethane. The organic layer was extracted with dichloromethane. The organic layers were combined, washed with saturated brine, and then dried over anhydrous sodium sulfate. The solvent was distilled off under reduced pressure. The residue thus obtained was purified by a chromatogram system (hexane:ethyl acetate=from 50:50 to 20:80) to give the title compound (122 mg).
MS (ESI) m/z : 581 (M⁺+H) .
¹H-NMR(CDCl₃)δ:0.85-1.30(4H,m), 1.26(3H,t,J=7.2Hz) 1.71-1.80(2H,m),1.86-1.93(1H,m),2.11-2.17(1H,m),2.32-2.55(5H,m),3.44(3H,s),3.69-3.80(1H,m),3.86(3H,s),4.10(4H,q,J=7.2Hz),4.38(1H,t,J=6.5Hz) ,5.63(1H,d,J=8.1Hz),5.72(1H,s),6.32-6.34(1H,m),6.38(1H,t,J=3.2Hz),6.72(1H,d,J=2.0Hz),6.96(1H,dd ,J=8.2,1.6Hz),7.09(1H,dd,J=2.7,1.7Hz),7.20(1H,t,J=7.9Hz),7. 27-7.38 (3H,m) .
IR (ATR) cm⁻¹: 2935, 1725, 1641, 1481, 1274, 1174, 1049, 713.

### Example 80

### (1S,3R)-3-({3-[(4R,6S)-8-Chloro-6-(2,3-dimethoxyphenyl)-4H, 6H-pyrrolo [1, 2-a] [4,1]benzoxazepin-4-yl]propanoyl}amino)cyclohexane carboxylic acid

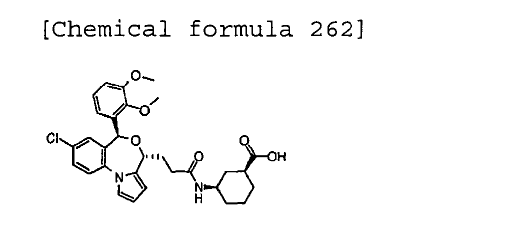

Ethyl (1S,3R)-3-({3-[(4R,6S)-8-chloro-6-(2,3-dimethoxyphenyl)-4H,6H-pyrrolo[1,2-a][4,1]benzoxazepin-4-yl]propanoyl}amino)cyclohexanecarboxylate (0.120 g) was dissolved in methanol (10 mL). To the resulting solution were added potassium carbonate (85.6 mg) and water (10 mL). The resulting mixture was stirred at 60°C for 3 hours and then, at room temperature for 18 hours. The reaction mixture was concentrated under reduced pressure. The residue thus obtained was neutralized with a 1N aqueous hydrochloric acid solution and then extracted three times with dichloromethane. The organic layers were combined, washed with saturated brine, and then dried over anhydrous sodium sulfate. The residue obtained by distilling off the solvent was triturated with dichloromethane-hexane-ether to give the title compound (84.2 mg).
MS(ESI)m/z:553(M⁺+H).
¹H-NMR(CDCl₃)δ:0.84-2.46(13H,m),3.39(3H,s),3.57-3.68(1H,m),3.81(3H,s),4.33(1H,t,J=6.8Hz),5.69(1H,s),6.03-6.13(1H,m),6.27-6.29(1H,m),6.32(1H,t,J=3.1Hz),6.70(1H,d,J=2.0Hz),6.89-6.93(1H,m),7.03-7.06(1H,m),7.15(1H,t,J=8.1Hz),7.27-7.34(3H,m).
IR(ATR)cm⁻¹:3293,2933,1643,1544,1488,1276,1051,711.
Elemental analysis for: C₃₀H₃₃ClN₂O₆·2.0H₂O
Calculated: C,61.17;H,6.33;N,4.76.
Found: C,61.50;H,6.11;N,4.58.

### Example 81

### Ethyl 2-({3-[(4R,6S)-8-chloro-6-(2,3-dimethoxyphenyl)-4H,6H-pyrrolo[1,2-a] [4,1]benzoxazepin-4-yl]propanoyl}amino)isonicotinate

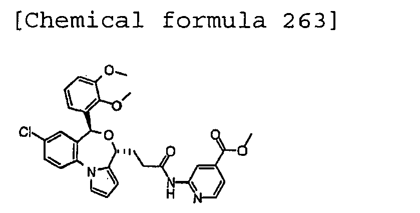

3-[(4R,6S)-8-Chloro-6-(2,3-dimethoxyphenyl)-4H,6H-pyrrolo[1,2-a][4,1]benzoxazepin-4-yl]propionic acid (0.200 g), benzotriazol-1-yl-oxy-tris(dimethylamino)phosphonium hexafluorophosphate (0.310 g), methyl 2-aminoisonicotinate (92.0 mg) and triethylamine (0.0977 mL) were added. The resulting mixture was stirred at room temperature for 18 hours. The reaction mixture was partitioned between water and dichloromethane. The water layer was extracted with dichloromethane. The organic layers were combined, washed with saturated brine, and dried over anhydrous sodium sulfate. The solvent was distilled off under reduced pressure. The residue thus obtained was purified by a chromatogram system (hexane:ethyl acetate= from 70:30 to 40:60) to give the title compound (38.8 mg).
¹H-NMR(CDCl₃)δ:2.44-2.59(2H,M), 2.66-2.85(2H,m),3.43(3H,s),3.84(3H,s),3.93(3H,s),4.46(1H,m),5.74 (1H,s),6.35-6.40(2H,m),6.72(1H,d,J=1.7Hz),6.91(1H,dd,J=8.1,1.5Hz),7.07-7.11(2H,m),7.26-7.36(3H,m),7.56(1H,dd,J=5.1,1.5Hz),8.34-8.37(2H,m),8.70(1H,br s).
MS(ESI)m/z:562(M⁺+H).

### Example 82

### 2-({3-[(4R,6S)-8-chloro-6-(2,3-dimethoxyphenyl)-4H,6H-pyrrolo[1,2-a][4,1]benzoxazepin-4-yl]propanoyl}amino)isonicotinic acid

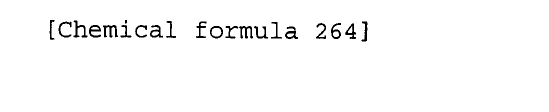

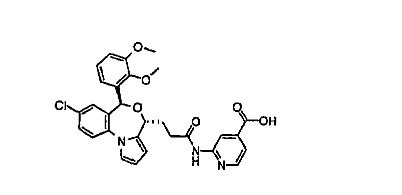

Ethyl 2-({3-[(4R,6S)-8-chloro-6-(2,3-dimethoxyphenyl)-4H,6H-pyrrolo[1,2-a][4,1]benzoxazepin-4-yl]propanoyl}amino)isonicotinate (35.0 mg) was dissolved in methanol (5 mL). To the resulting solution were added potassium carbonate (25.8 mg) and water (5 mL). The resulting mixture was stirred at 60°C for 3 hours and then at room temperature for 18 hours. The reaction mixture was concentrated under reduced pressure. The residue thus obtained was neutralized with a 1N aqueous hydrochloric acid solution and then, extracted three times with dichloromethane. The organic layers were combined, washed with saturated brine and then, dried over anhydrous sodium sulfate. The residue obtained by distilling off the solvent was triturated with dichloromethane-hexane-ether, whereby the title compound (16.6 mg).
MS(ESI)m/z:548(M⁺+H).
¹H-NMR(CDCl₃)δ:2.32-2.83(4H,m),3.40(3H,s),3.82(3H,s),4.39-4.46(1H,m),5.70-5.73(1H,m).,6.33-6.40(2H,m),6.70-6.72(1H,m),6.88(1H,d,J=8.1Hz),6.98-7.20(2H,m),7.27-7.34(3H,m),7.52-7.55(1H,m),8.20-8.29(1H,m),8.62-8.80(2H,m).
IR(ATR)cm⁻¹ :2938,1704,1579,1488,1415,1272,1222,1170,1054,769,711.
Elemental analysis for: C₂₉H₂₆ClN₃O₆·0.5H₂O
Calculated: C,62.53;H,4.89;N,7.54.
Found: C,62.63;H,4.75;N,7.30.

### Referential Example 33

### Ethyl (1S,3R)-3-[N-(2-nitrophenylsulfonyl)amino]cyclohexane carboxylate

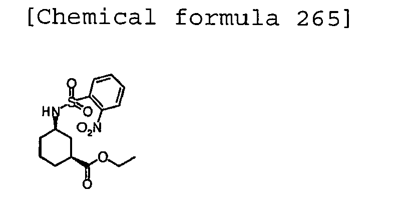

Ethyl (1S,3R)-3-aminocyclohexane carboxylate (0.500 g) was dissolved in dichloromethane (30 mL). Under ice cooling, triethylamine (0.810 mL) and 2-nitrophenylsulfonyl chloride (0.640 g) were added and the resulting mixture was stirred at room temperature for 1 hour. To the residue obtained by concentrating the reaction mixture under reduced pressure were added ethyl acetate and water. The water layer was extracted with ethyl acetate. The organic layers were combined, washed with saturated brine and dried over anhydrous sodium sulfate. The solvent was distilled off under reduced pressure. The residue thus obtained was purified by silica gel column chromatography (hexane:ethyl acetate= from 60:40 to 40:60) to give the title compound (0.700 g).
¹H-NMR(CDCl₃)δ:1.22(3H,t,J=7.1Hz),1.25-1.44(4H,m),1.79-1.94(3H,m),2.10-2.16(1H,m),2.27-2.36(1H,m),3.31-3.41(1H,m),4.09(2H,q,J=7.1Hz),5.29(1H,d,J=7.6Hz),7.71-7.79(2H,m),7.86-7.89(1H,m),8.16-8.19(1H,m).
MS(ESI)m/z:357(M⁺+H).

### Referential Example 34

### Ethyl (1S,3R)-3-{N-methyl-N-(2-nitrophenyl)sulfonylamino}cyclohexane carboxylate

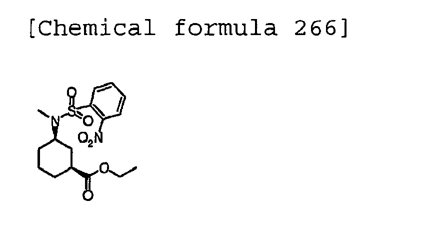

Ethyl (1S,3R)-3-[N-(2-nitrophenyl)sulfonylamino]cyclohexane carboxylate (0.600 g) was dissolved in dimethylformamide (20 mL). Cesium carbonate (1.37 g) was added and the resulting mixture was stirred at room temperature for 30 minutes. Methyl iodide (0.262 mL) was added and the resulting mixture was stirred at room temperature for 3.5 hours. The reaction mixture was filtered and the filtrate was concentrated under reduced pressure. To the residue were added ethyl acetate and water. The water layer was extracted with ethyl acetate. The organic layers were combined, washed with saturated brine and then dried over anhydrous sodium sulfate. The solvent was distilled off. The residue thus obtained was purified by silica gel column chromatography (hexane:ethyl acetate= from 65:35 to 45:55) to give the title compound (598 mg).
¹H-NMR(CDCl₃)δ:1.24(3H,t,J=7.1Hz),1.39-1.48(2H,m),1.55-1.71(3H,m),1.85-1.98(3H,m),2.47(1H,m),2.84(3H,s),3.82-3.90(1H,m),4.10(2H,q,J=7.1Hz),7.60-7.63(1H,m),7.67-7.70(2H,m),8.01-8.04(1H,m).
MS(ESI)m/z:371(M⁺+H).

### Referential Example 35

### Ethyl (1S,3R)-3-(N-methyl)aminocyclohexane carboxylate

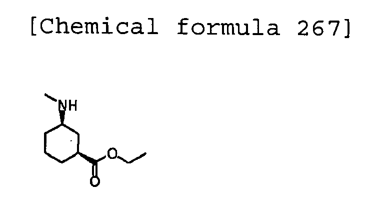

Ethyl (1S,3R)-3-(methyl[(2-nitrophenyl)sulfonyl]amino)cyclohexane carboxylate (598 mg) was dissolved in dimethylformamide. Thioglycolic acid (0.674 mL) and lithium hydroxide (0.232 g) were added. The resulting mixture was stirred at room temperature for 2 hours. To the reaction mixture was added lithium hydroxide (0.232 g), followed by stirring for 5 minutes. Diethyl ether and a saturated aqueous solution of sodium hydrogen carbonate were added to the reaction mixture and the layers separated. The water layer was extracted with diethyl ether. The organic layers were combined, washed with saturated brine and dried over anhydrous sodium sulfate. The residue obtained by distilling off the solvent was partitioned between ethyl acetate and water. The water layer was extracted with ethyl acetate. The organic layers were combined, washed with saturated brine and dried over anhydrous sodium sulfate. The solvent was distilled off to give the title compound (0.100 g).
¹H-NMR(CDCl₃)δ:0.94-1.45(4H,m)1.25(3H,t,J=7.1Hz),1.81-1.89(1H,m),1.91-1.97(2H,m),2.17-2.24(1H,m),2.28-2.41(2H,m),2.44(3H,s),4.13(2H,q,J=7.1Hz).

### Example 83

### Ethyl (1S,3R)-3-(N-{3-[(4R,6S)-8-chloro-6-(2,3-dimethoxyphenyl)-4H,6H-pyrrolo[1,2-a][4,1]benzoxazepin-4-yl]propanoyl}-N-methylamino)cyclohexane carboxylate

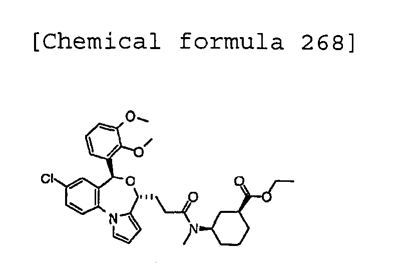

3-[(4R,6S)-8-Chloro-6-(2,3-dimethoxyphenyl)-4H,6H-pyrrolo[1,2-a] [4,1]benzoxazepin-4-yl]propionic acid (0.100 g) and 1-hydroxybenzotriazole (46.5 mg) were dissolved in dichloromethane (10 mL). The resulting solution was stirred at room temperature for 10 minutes. Ethyl(1S,3R)-3-(N-methyl)aminocyclohexane carboxylate (49.0 mg) and 1-ethyl-3-(3-dimethylaminopropyl)carbodiimide hydrochloride (50.8 mg) were added and the resulting mixture was stirred at room temperature for 2 hours. The reaction mixture was partitioned between water dichloromethane. The water layer was extracted with dichloromethane. The organic layers were combined, washed with saturated brine and, dried over anhydrous sodium sulfate. The solvent was distilled off under reduced pressure. The residue thus obtained was purified by silica gel column chromatography (hexane:ethyl acetate= from 50:50 to 20:80) to give the title compound (98.0 mg).
¹H-NMR(CDCl₃)δ:0.85-1.97(8H,m),1.26(3H,t,J=7.1Hz),2.33-2.79(5H,m),2.80-2.85(3H,m),3.44(3H,s),3.85(3H,s),4.12(2H,q,J=7.1Hz),4.38-4.43(1H,m),4.47-4.56(1H,m),5.71(1H,d,J=5.6Hz),6.35-6.39(2H,m),6.69-6.72(1H,m),6.93-6.95(1H,m),7.07-7.10(1H,m),7.15-7.20(1H,m),7.27-7.31(1H,m),7.33-7.36(2H,m).
MS(ESI)m/z:595(M⁺+H).

### Example 84

### (1S,3R)-3-(N-{3-[(4R,6S)-8-Chloro-6-(2,3-dimethoxyphenyl)-4H,6H-pyrrolo[1,2-a][4,1]benzoxazepin-4-yl]propanoyl}-N-methylamino)cyclohexane carboxylate

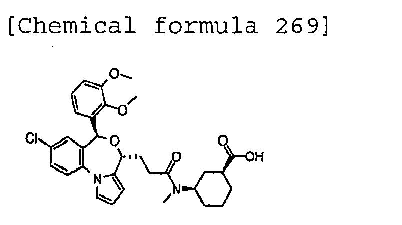

Ethyl (1S,3R)-3-(N-{3-[(4R,6S)-8-chloro-6-(2,3-dimethoxyphenyl)-4H,6H-pyrrolo[1,2-a][4,1]benzoxazepin-4-yl]propanoyl}-N-methylamino)cyclohexanecarboxylate (98.0 mg) was dissolved in methanol (5 mL). To the resulting solution were added potassium carbonate (68.3 mg) and water (5 mL). The resulting mixture was stirred at 60°C for 3 hours and then at room temperature for 18 hours. The reaction mixture was reduced under pressure. The residue thus obtained was neutralized with a 1N aqueous hydrochloric acid solution and then extracted three times with dichloromethane. The organic layers were combined, washed with saturated brine and then, dried over anhydrous sodium sulfate. The solvent was distilled off. The residue thus obtained was triturated with dichloromethane-hexane-ether to give the title compound (72.0 mg).
MS (ESI) m/z : 567 (M⁺+H).
¹H-NMR(CDCl₃)δ:0.83-1.94(8H,m),2.04-2.66(5H,m),2.78(3H,s),3.39(3H,s),3.82(3H,s),4.33-4.41(2H,m),5.67-5.71(1H,m),6.32-6.37(2H,m),6.68-6.71(1H,m),6.90-6.95(1H,m),7.04-7.08(1H,m),7.14(1H,t,J=7.8Hz),7.27-7.33(3H,m).
IR(ATR)cm⁻¹:2931,1633,1488,1276,1099,1051,1004,711.
Elemental analysis for: C₃₁H₃₅ClN₂O₆·2.0H₂O
Calculated: C,61.74;H,6.52;N,4.64.
Found: C,61.52;H,6.55;N,4.28.

### Example 85

### Ethyl (1S,3R)-3-[{2-[(4R,6S)-8-chloro-6-(2,3-dimethoxyphenyl)-4H,6H-pyrrolo[1,2-a][4,1]benzoxazepin-4-yl]ethyl}(methyl)amino]cyclohexane carboxylate

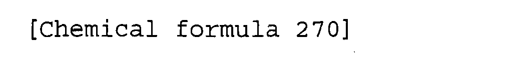

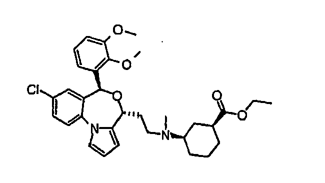

2-[(4R,6S)-8-Chloro-6-(2,3-dimethoxyphenyl)-4H,6H-pyrrolo-[1,2-a][4,1]benzoxazepin-4-yl]ethyl 4-methylbenzenesulfonate (0.100 g) and (1S,3R)-3-(N-methyl)aminocyclohexane carboxylate (0.0400 g) were dissolved in acetonitrile (20 mL). To the resulting solution was added potassium carbonate (0.0400 g) and the resulting mixture was stirred at 55°C for 3 days. To the residue obtained by concentrating the reaction mixture under reduced pressure were added dichloromethane and water. The water layer was extracted with dichloromethane. The organic layers were combined, washed successively with a saturated aqueous solution of ammonium chloride and saturated brine, and dried over anhydrous sodium sulfate. The solvent was distilled off. The residue thus obtained was purified by silica gel column chromatography (chloroform:methanol=95:5-90:10) to give the title compound (36.2 mg).
MS (ESI) m/z : 567 (M⁺+H).
¹H-NMR(CDCl₃)6:1.24(3H,t,J=7.1Hz),1.18-2.49(9H,m),2.05(3H,s),2.69-3.04(4H,m),3.43(3H,s),3.85(3H,s),4.12(2H,q,J=7.1Hz),4.38-4.43(1H,m),5.68(1H,s),6.33-6.36(1H,m),6.37-6.39(1H,m),6.70-6.72(1H,m),6.95(1H,d,J=8.1Hz),7.07-7.10(1H,m),7.17-7.38(4H,m).

### Example 86

### (1S,3R)-3-[{2-[(4R,6S)-8-chloro-6-(2,3-dimethoxyphenyl)-4H,6H-pyrrolo[1,2-a][4,1]benzoxazepin-4-yl]ethyl}(methyl)amino]cyclohexane carboxylic acid

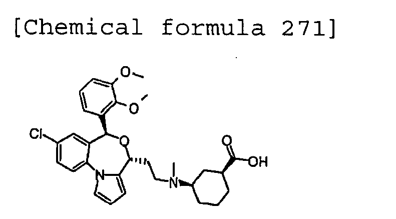

Ethyl (1S,3R)-3-[{2-[(4R,6S)-8-chloro-6-(2,3-dimethoxyphenyl)-4H,6H-pyrrolo[1,2-a][4,1]benzoxazepin-4-yl]ethyl}(methyl)amino]cyclohexane carboxylate (36.2 mg) was dissolved in methanol (5 mL). To the resulting solution were added potassium carbonate (26.5 mg) and water (5 mL). After stirring at 60°C for 3 hours and then at room temperature for 18 hours, the reaction mixture was concentrated under reduced pressure. The residue thus obtained was neutralized with a 1N aqueous hydrochloric acid solution and then extracted three times with dichloromethane. The organic layers were combined, washed with saturated brine and then, dried over anhydrous sodium sulfate. The solvent was distilled off under reduced pressure. The residue thus obtained was triturated with dichloromethane-hexane-ether to give the title compound (23.0 mg).
MS(ESI)m/z:539(M⁺+H)
¹H-NMR(CDCl₃)δ:0.88-3.11(13H,m),2.04(3H,s),3.42(3H,s),3.84(3H,s),4.35-4.41(1H,m),5.68(1H,s),6.32-6.38(2H,m),6.70(1H,d,J=1.7Hz),6.92-6.97(1H,m),7.06-7.08(1H,m),7.18(1H,t,J=8.1Hz),7.24-7.34(3H,m).
IR(ATR)cm⁻¹:2933,1589,1492,1334,1267,1101,1000,835,717.

### Example 87

### Ethyl 2-(1-(3-[(4R,6S)-8-chloro-6-(2,3-dimethoxyphenyl)-4H,6H-pyrrolo[1,2-a][4,1]benzoxazepin-4-yl]propanoyl)-4-piperidinyl)acetate

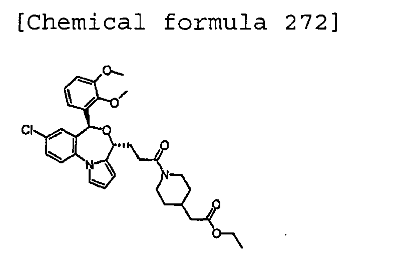

3-[(4R,6S)-8-Chloro-6-(2,3-dimethoxyphenyl)-4H,6H-pyrrolo[1,2-a][4,1]benzoxazepin-4-yl]propionic acid (1.04 g) was dissolved in dichloromethane (20 mL). To the resulting solution were added ethyl piperidine-4-acetate (500 mg), 1-hydroxybenzotriazole (112 mg), and 1-ethyl-3-(3-dimethylaminopropyl)carbodiimide hydrochloride (560 mg). The resulting mixture was stirred at room temperature for 13 hours. After addition of ethyl acetate, the resulting mixture was washed with 1N hydrochloric acid, a saturated aqueous solution of sodium bicarbonate, and saturated brine and dried over anhydrous sodium sulfate. The solvent was distilled off under reduced pressure. The residue was purified by silica gel column chromatography (hexane:ethyl acetate) to give the title compound (1.32 g).
¹H-NMR(CDCl₃)δ:1.11(2H,br s), 1.26 (3H, t, J=7. 08Hz), ,1.50-1.53(1H,m),1.69-1.80(2H,m),2.00(1H,br s),2.19-2.26(2H,m),2.36-2.43(2H,m),2.55-2.61(2H,m),3.01(1H,s),3.43(3H,s),3.85(3H,s),4.13(2H,q,J=7.1 6Hz),4.42-4.37(1H,m),4.64-4.56(1H,m),5.70(1H,s),6.40-6.34(2H,m),6.70(1H,d,J=1.95Hz),6.95(1H,dd,J=8.18,1.59Hz),7. 10-7.07(1H,m),7.21-7.15(1H,m),7.28-7.31(2H,m),7.36-7.34(2H,m).

### Example 88

### 2-(1-{3-[(4R,6S)-8-Chloro-6-(2,3-dimethoxyphenyl)-4H,6H-pyrrolo[1,2-a][4,1]benzoxazepin-4-yl]propanoyl}-4-piperidinyl)acetic acid

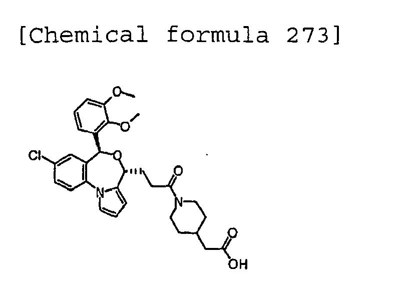

Ethyl 2-(1-{3-[(4R,6S)-8-chloro-6-(2,3-dimethoxyphenyl)-4H,6H-pyrrolo[1,2-a][4,1]benzoxazepin-4-yl]propanoyl}-4-piperidinyl)acetate (86 mg) was dissolved in a tetrahydrofuran-methanol-water (1:1:2) solvent mixture (4 mL). To the resulting solution was added potassium carbonate (82 mg), followed by stirring at 55°C for 23 hours. Under ice cooling, the reaction mixture was neutralized with a 1N aqueous hydrochloric acid solution. The solution was then extracted with ethyl acetate. The extract was washed with saturated brine and dried over anhydrous sodium sulfate. The solvent was distilled off. The residue thus obtained was triturated with ethyl acetate-hexane to give the title compound (61.2 mg).
¹H-NMR(CDCl₃)δ:1.89-1.04(4H,m),2.00(1H,br s),2.46-2.20(4H,m),2.75-2.47(3H,m),3.07-2.96(1H,m),3.43(3H,s),3.98-3.80(4H,m),4.44-4.36(1H,m),4.66-4.57(1H,m),5.70(1H,s),6.40-6.33 (2H,m) 6.72-6.69(1H,m), 6.98-6.93 (1H,m), 7.11-7.07(1H,m),7.21-7.15(1H,m),7.39-7.25(3H,m).
Elemental analysis for: C₃₀H₃₃ClN₂O₆
Calculated: C,65.15;H,6.01;N,5.07.
Found: C,65.36;H,6.20;N,4.80.

### Example 89

### Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-1-piperazinyl)-2-oxoacetate

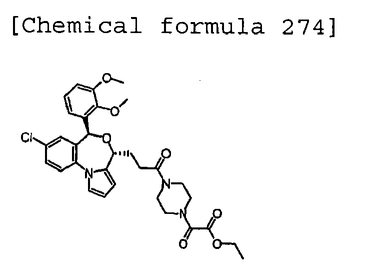

3-[(4R,6S)-8-Chloro-6-(2,3-dimethoxyphenyl)-4H,6H-pyrrolo[1,2-a][4,1]benzoxazepin-4-yl]propionic acid (981 mg) was dissolved in dichloromethane (20 mL). To the resulting solution were added ethyl 2-oxo-2-(1-piperazinyl)acetated hydrochloride (613 mg), triethylamine (415 µl), 1-hydroxybenzotriazole (105 mg), and 1-ethyl-3-(3-dimethylaminopropyl)carbodiimide hydrochloride (571 mg). The resulting mixture was stirred at room temperature for 13 hours. Ethyl acetate was added. The resulting mixture was washed with 1N hydrochloric acid, a saturated aqueous solution of sodium bicarbonate and saturated brine and dried over anhydrous sodium sulfate. The solvent was distilled off under reduced pressure. The residue was purified by silica gel column chromatography (hexane:ethyl acetate) to give the title compound (1.26 g).
¹H-NMR(CDCl₃)δ:1.32(3H,dt,J=45.74,7.14Hz),1.98-2.22(4H,m),2.36-2.45(1H,m),2.50-2.80(1H,m),3.38-3.45(4H,m),3.47-3.76(5H,m),3.86(3H,s),4.31-4.44(3H,m),5.71(1H,s),6.33-6.36(1H,m),6.38(1H,t,J=3.17Hz),6.71(1H,s),6.93-6.98(1H,m),7.09(1H,t,J=1.46Hz),7.18(1H,t,J=7.81Hz),7.23-7.39(3H,m).

### Example 90

### 2-(4-{3-[(4R,6S)-8-Chloro-6-(2,3-dimethoxyphenyl-4H,6H-pyrrolo[1,2-a][4,1]benzoxazepin-4-yl)propanoyl)-1-piperazinyl)-2-oxoacetic acid

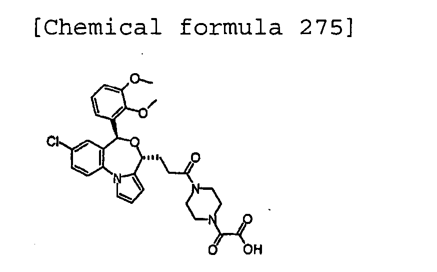

Ethyl 2-(4-{3-[(4R,6S)-8-chloro-6-(2,3-dimethoxyphenyl)-4H,6H-pyrrolo[1,2-a][4,1]benzoxazepin-4-yl]propanoyl}-1-piperazinyl)-2-oxoacetate (42.0 mg) was dissolved in a tetrahydrofuran-methanol-water (1:1:2) solvent mixture (4 mL). To the resulting solution was added potassium carbonate (39 mg). The resulting mixture was stirred at 55°C for 5 hours. Under ice cooling, the reaction mixture was neutralized with a 1N aqueous hydrochloric acid solution. The solution was extracted with ethyl acetate. The extract was washed with saturated brine and then dried over anhydrous sodium sulfate. The solvent was distilled off and the residue thus obtained was triturated with ethyl acetate-hexane, whereby the title compound (35.1 mg).
Elemental analysis for: C₂₉H₃₀ClN₃O₇·0.5H₂O
Calculated: C,60.36;H,5.41;N,7.28.
Found: C,60.58;H,5.47;N,7.02.

### Example 91

### Ethyl 2-(1-{4-[(4R,6S)-8-chloro-6-(2,3-dimethoxyphenyl)-4H,6H-pyrrolo[1,2-a][4,1]benzoxazepin-4-yl]butanoyl}-4-piperidinyl)acetate

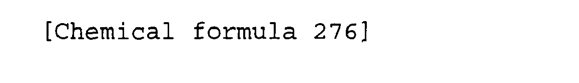

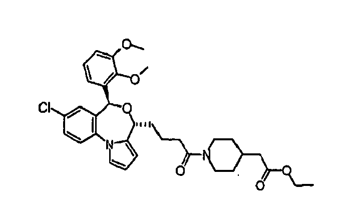

4-((4R,6S)-8-Chloro-6-(2,3-dimethoxyphenyl)-4H,6H-pyrrolo(1,2-a)(4,1)benzoxazepin-4-yl)butanoic acid (68.4 mg) was dissolved in dichloromethane (2 mL). To the resulting solution were added ethyl piperidine-4-acetate (29 mg), 1-hydroxybenzotriazole (7.1 mg), and 1-ethyl-3-(3-dimethylaminopropyl)carbodiimide hydrochloride (36 mg). The resulting mixture was stirred at room temperature for 17 hours. Ethyl acetate was added to the reaction mixture. The resulting mixture was washed with 1N hydrochloric acid, a saturated aqueous solution of sodium bicarbonate and saturated brine, and dried over anhydrous sodium sulfate. The solvent was distilled off under reduced pressure. The residue was purified by silica gel column chromatography (hexane:ethyl acetate) to give the title compound (73.2 mg).
¹H-NMR(CDCl₃)δ:1.13-1.16(2H,m) 1.26(3H,t,J=7.08Hz),1.87-1.99(6H,m),2.20-2.26(2H,m),2.37-2.43(2H,m),2.55(1H,t,J=11.96Hz),3.00(1H,t,J=12.70Hz),3.38-3.51(4H,m),3.87-3.79(4H,m),4.13(2H,q,J=7.16Hz),4.34-4.40(1H,m),4.57-4.64(1H,m),5.69(1H,s),6.29-6.33(1H,m),6.36(1H,t,J=3.17Hz),6.71(1H,d,J=1.95Hz),6.93(1H, t,J=7.93Hz),7.06-7.13(1H,m),7.13-7.20(1H,m),7.29-7.41(3H,m).

### Example 92

### 2-(1-{4-[(4R,6S)-8-Chloro-6-(2,3-dimethoxyphenyl)-4H,6H-pyrrolo[1,2-a][4,1]benzoxazepin-4-yl]butanoyl}-4-piperidinyl)acetic acid

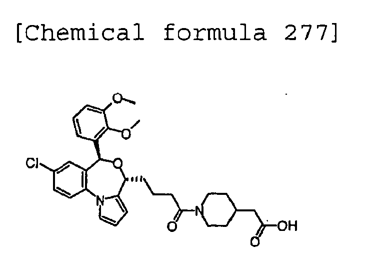

Ethyl 2-(1-{4-[(4R,6S)-8-chloro-6-(2,3-dimethoxyphenyl) -4H, 6H-pyrrolo [1, 2-a] [4, 1] benzoxazepin-4-yl]butanoyl}-4-piperidinyl)acetate (73 mg) was dissolved in a tetrahydrofuran-methanol-water (1:1:2) solvent mixture (4 mL). To the resulting solution was added potassium carbonate (68 mg), followed by stirring at 55°C for 24 hours. Under ice cooling, the reaction mixture was neutralized with a 1N aqueous hydrochloric acid solution. The solution was then extracted with ethyl acetate. The extract was washed with saturated brine and then, dried over anhydrous sodium sulfate. The solvent was distilled off and the residue thus obtained was triturated with ethyl acetate-hexane to give the title compound (59.5 mg).
¹H-NMR(CDCl₃)δ:1.35-1.07(3H,m),1.72-2.19(9H,m),2.23-2.31(2H,m),2.41(2H,t,J=7.57Hz),2.51-2.60(1H,m),2.96-3.05(1H,m),3.42(3H,s),3.79-3.87(4H,m),4.34-4.40(1H,m),4.58-4.66(1H,m),5.69(1H,s),6.27-6.33(1H,m),6.36(1H,t,J=3.17Hz),6.70-6.72(1H,m),6.90-6.96(1H,m),7.06-7.13(1H,m),7.13-7.19(1H,m),7.28-7.41(3H,m).
Elemental analysis for: C₃₁H₃₅ClN₂O₆
Calculated: C,65.66;H,6.22;N,4.94.
Found: C,66.10;H,6.57;N,4.84.

### Referential Example 36

### 2-({(3R)-1-[(Benzyloxy)carbonyl]piperidinyl}oxy)acetic acid

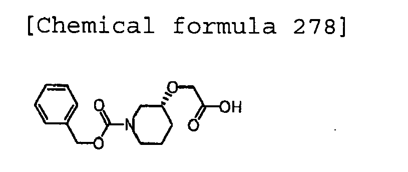

To a solution of benzyl (3R)-3-[2-(t-butoxy)-2-oxoethoxy]-1-piperidine carboxylate (500 mg) in methylene chloride (3.0 mL) was added trifluoroacetic acid (3.0 mL). The resulting mixture was stirred at room temperature for 90 minutes. After concentration of the reaction mixture under reduced pressure, azeotropy with toluene was performed twice, followed by concentration under reduced pressure, whereby the title compound (510 mg). ¹H-NMR(CDCl₃)δ:1.47(1H,s), 1.72-1.93(3H,m),3.81(7H,m),5.14(2H,s),7.14-7.40(5H,m).

### Referential Example 37

### Benzyl (3R)-3-(2-ethoxy-2-oxoethoxy)-1-piperidine carboxylate

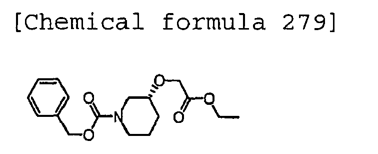

2-({(3R)-1-[(Benzyloxy)carbonyl]piperidinyl}oxy)acetic acid (510 mg) was dissolved in benzene (100 mL). To the resulting solution were added ethanol (8.0 mL) and concentrated sulfuric acid (15 µl). The resulting mixture was heated under reflux for 4 hours. After concentration of the reaction mixture under reduced pressure, the residue was dissolved in ethyl acetate. The resulting solution was washed successively with a saturated aqueous solution of sodium hydrogen carbonate and saturated brine and then dried over anhydrous sodium sulfate. The solvent was distilled off under reduced pressure. The crude product thus obtained was purified by silica gel column chromatography (n-hexane/ethyl acetate=4/1 to 1/1) to give the title compound (387 mg).
¹H-NMR(CDCl₃)δ:1.27(3H,dd,J=8.8,4.2Hz) 1.45(1H,s), 1.54-1.66(1H,m),1.79(1H,s),1.97(1H,s),3.14-3.20(2H,m),3.44(1H,s),3.66(1H,dt,J=13.4,4.8Hz),3.88(1H,s),4 .08-4.22(4H,m),5.12(2H,s),7.34(5H,ddd,J=22.0,11.0,6.8Hz)

### Referential Example 38

### Ethyl 2-[(3R)piperidinyloxy]acetate

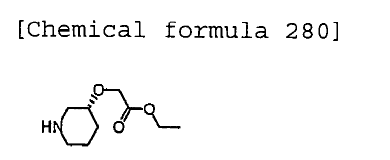

Benzyl (3R)-3-(2-ethoxy-2-oxoethoxy)-1-piperidine carboxylate (385 mg) was dissolved in ethanol (20 mL). To the resulting solution was added 10% palladium-carbon (77 mg). The resulting mixture was stirred overnight in a hydrogen gas atmosphere. After the catalyst was filtered off, the filtrate was concentrated under reduced pressure to give the title compound (210 mg).
¹H-NMR(CDCl₃)δ:1.29(3H,td,J=6.4,1.9Hz),1.43(1H,tt,J=13.3,4.3Hz ),1.60(1H,tt,J=13.1,4.3Hz), 1.77(1H,tt,J=10.0,3.4Hz), 1.92-1.96(1H,m), 2.06(1H,brs), 2.65-2.73(2H,m), 2.80-2.85(1H,m), 3.08(1H,dd,J=12.5,2.9Hz), 3.36-3.42(1H,m), 4.12(2H,dd,J=22.5,16.4Hz), 4.22(2H,q,J=7.1Hz).

### Example 93

### Ethyl 2-[((3R)-1-{3-[(4R,6S)-8-chloro-6-(2,3-dimethoxyphenyl)-4H,6H-pyrrolo[1,2-a][4,1]benzoxazepin-4-yl]propanoyl}piperidinyl)oxy]acetate

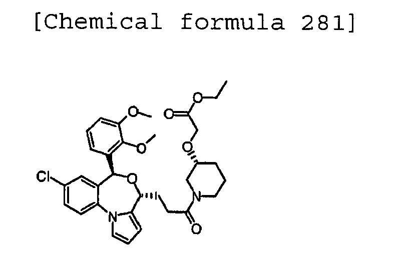

To a solution of 3-[(4R,6S)-8-chloro-6-(2,3-dimethoxyphenyl)-4H,6H-pyrrolo[1,2-a][4,1]benzoxazepin-4-yl]propanoic acid (100 mg), ethyl 2-[(3R piperidinyloxy)acetate (65.7 mg), 1-hydroxybenzotriazole (47.4 mg), and N-methylmorpholine (28.3 µl) in N,N-dimethylformamide (10 mL) was added 1-ethyl-3-(3-dimethylaminopropyl)carbodiimide hydrochloride (67.3 mg). The resulting mixture was stirred at room temperature for 3 days. After concentration of the reaction mixture under reduced pressure, the residue thus obtained was dissolved in ethyl acetate. The resulting solution was washed successively with water, saturated sodium hydrogen carbonate, and saturated brine and then dried over anhydrous sodium sulfate. The solvent was distilled off under reduced pressure. The residue thus obtained was purified by thin layer chromatography (methylene chloride/methanol=9/1) to give the title compound (100 mg).
¹H-NMR(CDCl₃)δ:1.59(7H,ddt,J=149.3,84.6,32.0Hz),2.39(2H,t,J=7. 2Hz),2.60(2H,dt,J=42.2,8.3Hz),3.20-3.51(7H,m),3.71-3.85(4H,m),4.02-4.21(4H,m),4.40(1H,s),5.70(1H,s),6.37(2H,q,J=4.4Hz),6.70(1H ,d,J=1.7Hz),6.94(1H,d,J=7.8Hz),7.08(1H,s),7.18(1H,t,J=7.9Hz ),7.28-7.37(3H,m).
MS(ESI)m/z:587(M⁺+H).

### Example 94

### 2-[((3R)-1-{3-[(4R,6S)-8-Chloro-6-(2,3-dimethoxyphenyl)-4H,6H-pyrrolo[1,2-a][4,1] benzoxazepin-4-yl]propanoyl}piperidinyl) oxy]acetic acid

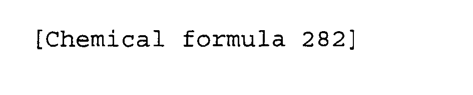

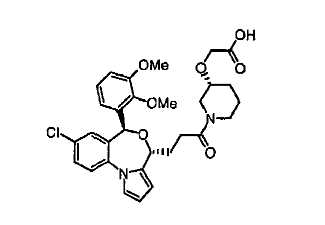

To a solution of ethyl 2-[((3R)-1-{3-[(4R,6S)-8-chloro-6-(2,3-dimethoxyphenyl)-4H,6H-pyrrolo[1,2-a] [4,1]benzoxazepin-4-yl]propanoyl}piperidinyl)oxy]acetate (137 mg) in a tetrahydrofuran (10 mL)/ethanol (20 mL)/water (10 mL) solvent mixture was added potassium carbonate (95.1 mg). The resulting mixture was stirred at room temperature for 3 days. The reaction mixture was adjusted to about pH 3 by the addition of 1N hydrochloric acid, and then concentrated under reduced pressure. The residue thus obtained was dissolved in methylene chloride. The resulting solution was washed successively with water and saturated brine, and then dried over anhydrous sodium sulfate. The solvent was distilled off under reduced pressure. The residue thus obtained was purified by thin layer chromatography (silica gel, methylene chloride/methanol=9/1) to give the title compound (131 mg).
¹H-NMR(CDCl₃)δ:1.54(4H,dd,J=173.1,59.1Hz),2.36-2.42(2H,m),2.65(2H,ddd,J=44.8,16.0,7.6Hz),3.35-3.88(11H,m),4.08(2H,s),4.36-4.41(1H,m),5.70(1H,s),6.29-6.38(2H,m),6.70(1H,s),6.93-7.37(6H,m). MS(FAB)m/z:569.2040
IR(cm-1):2935,1732,1587,1481,1428,1323,1273,1221,1099,1053,1003,8 22,712.
Elemental analysis for: C₃₀H₃₃O₇N₂Cl·0.5H₂O
Calculated: C,62.33;H,5.93;N,4.85.
Found: C,62.57;H,5.94;N,4.78.

### Example 95

### Ethyl (3S)-1-(3-((4R,6S)-8-chloro-6-(2,3-dimethoxyphenyl)-4H,6H-pyrrolo(1,2-a)(4,1)benzoxazepin-4-yl)propanoyl)-3-piperidine carboxylate

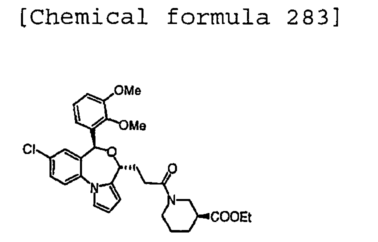

3-[(4R,6S)-8-Chloro-6-(2,3-dimethoxyphenyl)-4H,6H-pyrrolo[1,2-a][4,1]benzoxazepin-4-yl]propanoic acid (1 g) was dissolved in dichloromethane (10.0 mL). To the resulting solution were added (s)-(+)-ethylnipecotate (440 mg), 1-ethyl-3-(3-dimethylaminopropyl)carbodiimide hydrochloride (896 mg), and 1-hydroxybenzotriazole (179 mg). The resulting mixture was stirred at room temperature for 14.6 hours. The reaction mixture was concentrated under reduced pressure. The residue was purified by silica gel column chromatography (ethyl acetate:n-hexane=1:1) to give the title compound (1.21 g).
MS(ESI)m/z:567(M⁺+H).
¹H-NMR(CDCl₃)δ:1.21-1.28(3H,m),1.38-1.49(1H,m),1.63-1.81(2H,m),1.99-2.09(1H,m),2.36-2.46(3H,m),2.52-2.84(2H,m),2.98-3.06(1H,m),3.43(3H,s),3.80-3.87(4H,m),4.09-4.18(2H,m),4.38-4.42(1H,m),5.70(1H,s),6.34-6.39(2H,m),6.70-6.72(1H,m),6.94(1H,d,J=8.1Hz),7.08(1H,dd,J=2.7,1.5Hz),7.17( 1H,t,J=8.0Hz),7.26-7.36(3H,m).

### Example 96

### (3S)-1-(3-((4R,6S)-8-Chloro-6-(2,3-dimethoxyphenyl)-4H,6H-pyrrolo(1,2-a)(4,1)benzoxazepin-4-yl)propanoyl)-3-piperidine carboxylic acid

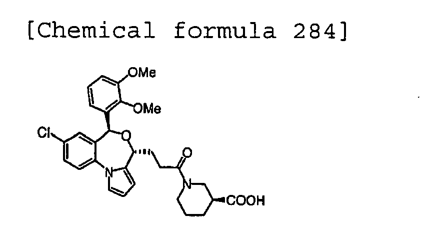

To a methanol solution (18 mL) of ethyl (3S)-1-(3-((4R,6S)-8-chloro-6-(2,3-dimethoxyphenyl)-4H,6H-pyrrolo(1,2-a)(4,1)benzoxazepin-4-yl)propanoyl)-3-piperidine carboxylate (100 mg) were added distilled water (9 mL) and potassium carbonate (49 mg). The resulting mixture was stirred at 50°C for 2 hours. A 10% aqueous solution of citric acid was added, followed by extraction with ethyl acetate. The organic layer was washed with saturated brine and then dried over sodium sulfate. The solvent was then distilled off and the residue was solidified with an ethyl acetate - n-hexane solvent mixture to give the title compound (85 mg).
MS(ESI)m/z:539(M⁺+H).
¹H-NMR(CDCl₃)δ:1.25-1.32(1H,m),1.41-1.53(1H,m),1.65-1.87(2H,m),1.99-2.13(1H,m),2.37-2.78(4H,m),2.91-3.15(2H,m),3.43(3H,s),3.76-4.03(5H,m),4.37-4.42(1H,m),5.71(1H,s),6.34-6.39(2H,m),6.70-6.72(1H,m),6.94(1H,d,J=7.8Hz),7.07-7.10(1H,m),7.17(1H,t,J=8.0Hz),7.27-7.36(6H,m).
Elemental analysis for: C₂₉H₃₁ClN₂O₆·0.25H₂O
Calculated: C,64.08;H,5.84;N,5.15.
Found: C,64.22;H,5.98;N,4.82.

### Example 97

### Ethyl 2-((2-(1-(3-((4R,6S)-8-chloro-6-(2,3-dimethoxyphenyl)-4H,6H-pyrrolo(1,2-a)(4,1)benzoxazepin-4-yl)propanoyl)-4-piperidinyl)acetyl)amino)acetate

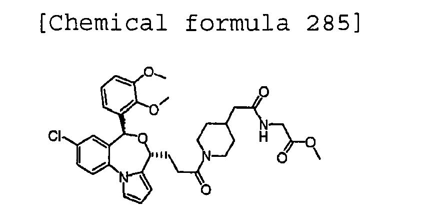

To a solution of 2-(1-{3-[(4R,6S)-8-chloro-6-(2,3-dimethoxyphenyl)-4H,6H-pyrrolo[1,2-a][4,1]benzoxazepin-4-yl]propanoyl}-4-piperidinyl)acetic acid (65 mg) in methylene chloride (5.0 mL) were added glycine methyl ester hydrochloride (18 mg), 1-ethyl-3-(3-dimethylaminopropyl)carbodiimide hydrochloride (45 mg), 1-hydroxybenzotriazole (18 mg), and triethylamine (20 µl). The resulting mixture was stirred at room temperature for 17 hours. The reaction mixture was purified by preparative thin layer chromatography (development phase: 5% methanol-chloroform) to give the title compound (53 mg).
MS(ESI)m/z:625(M⁺+H).
¹H-NMR(CDCl₃)δ:1.01-1.16(2H,m),1.69-1.82(2H,m),2.01-2.20(4H,m),2.34-2.68(5H,m),2.97-3.05(1H,m),3.43(3H,s),3.76(3H,s),3.84-3.94(4H,m),4.04(2H,dd,J=5.1,2.5Hz),4.36-4.42(1H,m),4.60(1H,d,J=13.5Hz),5.70(1H,s),6.01(1H,t,J=4.9Hz ),6.34-6.38(2H,m),6.69-6.71(1H,m),6.95(1H,dd,J=8.1,1.2Hz),7.08(1H,dd,J=2.7,1.5Hz), 7.18(1H,t,J=8.1Hz), 7.27-7.31(1H,m), 7.34-7.37(2H,m).

### Example 98

2-((2-(1-(3-((4R,6S)-8-Chloro-6-(2,3-dimethoxyphenyl)-4H,6H-pyrrolo(1,2-a)(4,1)benzoxazepin-4-yl)propanoyl)-4-piperidinyl)acetyl)amino)acetic acid

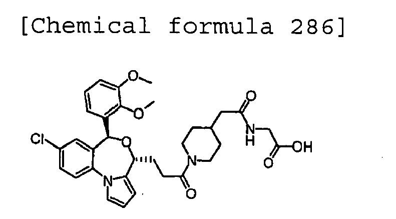

To a methanol solution (10.0 mL) of ethyl 2-((2-(1-(3-((4R,6S)-8-chloro-6-(2,3-dimethoxyphenyl)-4H,6H-pyrrolo(1,2-a)(4,1)benzoxazepin-4-yl)propanoyl)-4-piperidinyl)acetyl)amino)acetate (53 mg) were added distilled water (5.0 mL) and potassium carbonate (35 mg). The resulting mixture was stirred at room temperature for 13 hours. The reaction mixture was adjusted to pH 4.0 with an ion exchange resin "IR-120B". The resin was filtered out and the solvent was distilled off. The residue was purified by preparative thin layer chromatography (development phase: a 7:3:1=chloroform:methanol:water organic layer), followed by lyophilization from 1,4-dioxane-water, whereby the title compound (62 mg).
MS (ESI) m/z : 610 (M⁺+H) .
¹H-NMR(CDCl₃)δ:0.84-1.09(3H,m),1.55-1.69(2H,m),1.83-2.64(5H,m),2.82-2.95(1H,m),3.35-3.38(3H,m),3.53-3.82(8H,m),4.30-4.45(2H,m),5.66(1H,s),6.29-6.32(2H,m),6.66-6.69(1H,m),6.89(1H,d,J=8.1Hz),7.01-7.03(1H,m),7.13(1H,t,J=8.1Hz),7.24-7.30(2H,m),7.50-7.60(1H,m).

### Example 99

Methyl 1-(3-((4R,6S)-8-chloro-6-(2,3-dimethoxyphenyl)-4H,6H-pyrrolo(1,2-a)(4,1)benzoxazepin-4-yl)propanoyl)-3-azetidine carboxylate

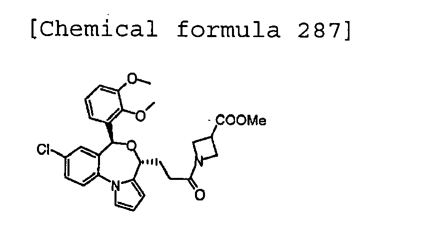

To a solution of 3-[(4R,6S)-8-chloro-6-(2,3-dimethoxyphenyl)-4H,6H-pyrrolo[1,2-a][4,1]benzoxazepin-4-yl]propanoic acid (58 mg) in methylene chloride (3.0 mL) were added 3-methylazetidine hydrochloride (27 mg), 1-ethyl-3-(3-dimethylaminopropyl)carbodiimide hydrochloride (52 mg), 1-hydroxybenzotriazole (21 mg), and triethylamine (23 µl). The resulting mixture was stirred at room temperature for 15 hours. The reaction mixture was purified by preparative thin layer chromatography (10% methanol-chloroform) to give the title compound (51 mg).
MS(ESI)m/z:525(M⁺+H).
¹H-NMR(CDCl₃)δ:2.28-2.48(4H,m),3.24-3.47(4H,m),3.75(3H,s),3.85(3H,s),4.06-4:34(4H,m),4.36-4.40(1H,m),5.70(1H,s),6.32-6.34(1H,m),6.37(1H,t,J=3.2Hz),6.71(1H,d,J=2.0Hz),6.95(1H,d, J=8.1Hz),7.08(1H,dd,J=2.7,1.5Hz),7.19(1H,t,J=8.1Hz),7.26-7.41(3H,m).

### Example 100

1-(3-((4R,6S)-8-Chloro-6-(2,3-dimethoxyphenyl)-4H,6H-pyrrolo(1,2-a)(4,1)benzoxazepin-4-yl)propanoyl)-3-azetidine carboxylic acid

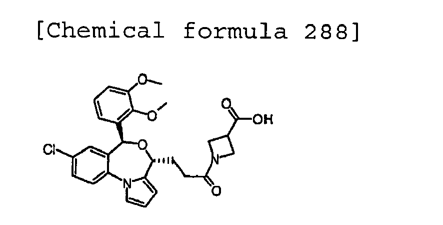

To a methanol solution (10.0 mL) of methyl 1-(3-((4R,6S)-8-chloro-6-(2,3-dimethoxyphenyl)-4H,6H-pyrrolo(1,2-a)(4,1)benzoxazepin-4-yl)propanoyl)-3-azetidine carboxylate (51 mg) were added distilled water (5.0 mL) and potassium carbonate (40 mg). The resulting mixture was stirred at 50°C for 5 hours. The reaction mixture was cooled to room temperature and then, adjusted to pH 4.0 with an ion exchange resin "IR-120B". The resin was filtered out and the solvent was distilled off. The residue was purified by preparative thin layer chromatography (a 7:3:1=chloroform:methanol:water organic layer), followed by lyophilization from 1,4-dioxane-water, whereby the title compound was (47 mg).
MS(ESI)m/z:512(M⁺+H).
¹H-NMR(CDCl₃) δ:2.09-2.34 (4H,m),3 .02-3.13 (1H,m),3.29-3.44(4H,m),3.78(3H,s),3.87-4.05(2H,m),4.10-4.19(1H,m),4.26-4.33(1H,m),5.64(1H,s),6.24-6.30(1H,m),6.65-6.70(1H,m),6.83-6.90(1H,m),6.96-7.02(1H,m),7.09-7.15(1H,m),7.22-7.30(3H,m).

### Referential Example 39

Ethyl 2-(3-azetidinyl)acetate hydrochloride

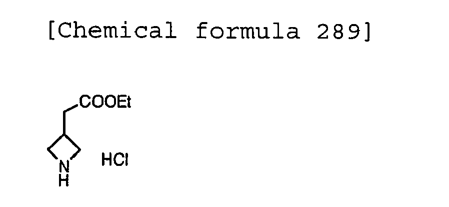

To a solution of ethyl 2-(1-benzhydryl-3-azetidinylidene)acetate (1.41 g) in ethanol (15.0 mL) were added 1N hydrochloric acid (3.6 mL) and 10% palladium-carbon (1.5 g), followed by catalytic hydrogenation at 5 atmospheres for 14 hours. The catalyst was filtered out and the solvent was distilled off under reduced pressure. To the residue was added ethanol (10 mL) and the title compound was obtained as a 0.3M ethanol solution.

### Example 101

Ethyl 2-(1-(3-[(4R,6S)-8-chloro-6-(2,3-dimethoxyphenyl)-4H,6H-pyrrolo[1,2-a][4,1]benzoxazepin-4-yl]propanoyl)-3-azetidinyl)acetate

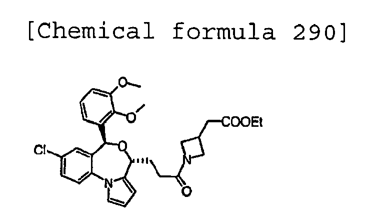

To a solution of 3-[(4R,6S)-8-chloro-6-(2,3-dimethoxyphenyl)-4H,6H-pyrrolo[1,2-a][4,1]benzoxazepin-4-yl]propanoic acid (86 mg) in methylene chloride (5.0 mL) were added a 0.3M solution (1.30 mL) of ethyl 2-(3-azetidinyl)acetate hydrochloride in ethanol, 1-ethyl-3-(3-dimethylaminopropyl)carbodiimide hydrochloride (115 mg), 1-hydroxybenzotriazole (31 mg), and triethylamine (56 µl). The resulting mixture was stirred at room temperature for 21 hours. The reaction mixture was purified by preparative thin layer chromatography (5% methanol-chloroform) to give the title compound (109 mg).
MS(ESI)m/z:553(M⁺+H).
¹H-NMR(CDCl₃)δ:1.22-1.27(3H,m),2.29-2.44(4H,m),2.51-2.54(1H,m),2.60(1H,dd,J=7.8,3.4Hz),2.82-2.97(1H,m),3.42(3H,s),3.60-3.66(1H,m),3.73-3.81(1H,m),3.84(3H,s),4.08-4.16(3H,m),4.26(1H,t,J=8.5Hz),4.36-4.40(1H,m),5.69-5.71(1H,m),6.32-6.34(1H,m),6.37(1H,t,J=3.2Hz),6.70-6.72(1H,m),6.94(1H,dd,J=8.3,1.5Hz),7.06-7.09(1H,m),7.16-7.20(2H,m),7.26-7.35(2H,m).

### Example 102

2-(1-(3-[(4R,6S)-8-Chloro-6-(2,3-dimethoxyphenyl)-4H,6H-pyrrolo[1,2-a][4,1]benzoxazepin-4-yl]propanoyl)-3-azetidinyl)acetic acid

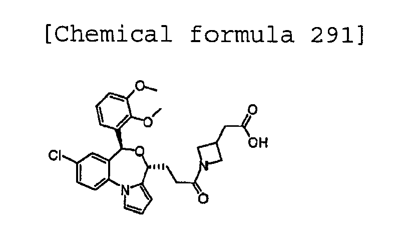

To a methanol solution (20.0 mL) of 3-[(4R,6S)-8-chloro-6-(2,3-dimethoxyphenyl)-4H,6H-pyrrolo[1,2-a][4,1]benzoxazepin-4-yl]propanoic acid (109 mg) were added distilled water (10.0 mL) and potassium carbonate (82 mg). The resulting mixture was stirred at room temperature for 16 hours. The reaction mixture was adjusted to pH 4.0 with an ion exchange resin "IR-120B". The resin was filtered out and the solvent was distilled off. The residue was purified by preparative thin layer chromatography (10% methanol-chloroform), followed by lyophilization from 1,4-dioxane-water to give the title compound (88 mg).
MS(ESI)m/z:525(M⁺+H).
¹H-NMR(CDCl₃) δ:2.28-2.42(4H,m), 2.53(1H,d, J=7.4Hz), 2.61(1H,d, J=7.4Hz), 2.80-2.94(1H,m),3.42(3H,s),3.59-3.68(1H,m),3.73-3.79(1H,m),3.84(3H,s),4.07-4.14(1H,m),4.25(1H,t,J=8.6Hz),4.34-4.39(1H,m),5.69(1H,s),6.31-6.34(1H,m),6.37(1H,t,J=3.2Hz),6.69-6.72(1H,m),6.92-6.96(1H,m),7.06-7.09(1H,m),7.18(1H,t,J=7.8Hz),7.27-7.30(1H,m),7.34-7.36(2H,m).

### Referential Example 40

3-Benzyl 1-ethyl 3-azabicyclo(3.1.0)hexane-1,3-dicarboxylate

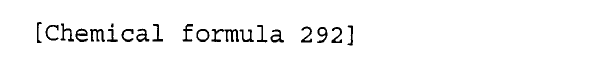

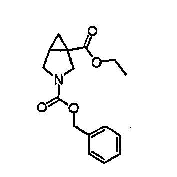

To a dimethylsulfoxide solution (4.0 mL) of trimethylsulfoxonium iodide (264 mg) was added 55% sodium hydride in oil (44 mg) at room temperature. The resulting mixture was stirred for 30 minutes. A dimethylsulfoxide solution (4.0 mL) of 1-benzyl 3-ethyl 2,5-dihydro-1H-pyrrol-1,3-dicarboxylate (J. Chem. Soc. Chem. Commun., 24, 1767-1769(1992)) (275 mg) was added to the reaction mixture at room temperature and the mixture was stirred for 35 minutes, followed by stirring at 70°C for 20 hours. After the reaction mixture was cooled to room temperature, ethyl acetate was added. The resulting mixture was washed with saturated brine and dried over anhydrous sodium sulfate. The solvent was distilled off. The residue was purified by silica gel column chromatography (ethyl acetate:n-hexane=1:2 to 2:3) to give the title compound (242 mg).
MS(ESI)m/z:290(M⁺+H).
¹H-NMR(CDCl₃)δ:0.84(1H,t,J=5.1Hz),1.25(3H,t,J=7.2Hz),1.57-1.62(1H,m),2.03-2.09(1H,m),3.47-3.88(4H,m),4.15(2H,q,J=7.2Hz),5.10-5.13(2H,m),7.29-7.38(5H,m).

### Referential Example 41

Ethyl 3-azabicyclo(3.1.0)hexane-1-carboxylate

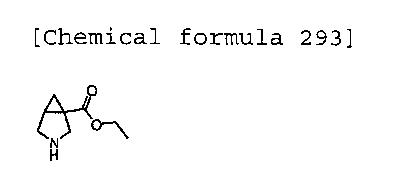

To a solution of 3-benzyl 1-ethyl 3-azabicyclo(3.1.0)hexane-1,3-dicarboxylate (242 mg) in ethanol (10.0 mL) was added 10% palladium-carbon (240 mg) and the resulting mixture was catalytically hydrogenated overnight at normal pressure. The catalyst was filtered out and the solvent was distilled off under reduced pressure, whereby the title compound (109 mg).
¹H-NMR(CDCl₃) δ:1.24-1.32(4H,m), 1.57(1H,dd,J=8.1,5.9Hz), 2.07-2.13(1H,m), 3.24-3.29(2H,m), 3.36(1H,d,J=11.8Hz), 3.58(1H,d,J=11.8Hz), 4.16(2H, q,J=7.1Hz).

### Example 103

Ethyl 3-(3-((4R,6S)-8-chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylate

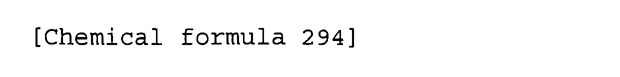

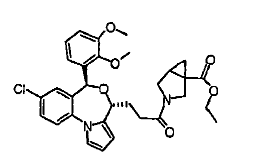

To a solution of 3-[(4R,6S)-8-chloro-6-(2,3-dimethoxyphenyl)-4H,6H-pyrrolo[1,2-a][4,1]benzoxazepin-4-yl]propanoic acid (300 mg) in methylene chloride (10.0 mL) were added ethyl 3-azabicyclo(3.1.0)hexane-1-carboxylate (109 mg), 1-ethyl-3-(3-dimethylaminopropyl)carbodiimide hydrochloride (268 mg), and 1-hydroxybenzotriazole (107 mg). The resulting mixture was stirred at room temperature for 12 hours. The reaction mixture was purified by silica gel chromatography (ethyl acetate:n-hexane=3:2) to give the title compound (612 mg).
MS(ESI)m/z:565(M⁺+H).
¹H-NMR(CDCl₃)δ:0.62-0.80(1H,m),1.26(3H,t,J=7.1Hz),1.52-1.63(1H,m),2.04-2.14(1H,m),2.31-2.46(2H,m),2.51-2.64(2H,m),3.43(3H,s),3.57-3.98(7H,m),4.17(2H,q,J=7.1Hz),4.34-4.41(1H,m),5.70(1H,s),6.33-6.35(1H,m),6.37(1H,t,J=3.2Hz),6.71(1H,t,J=2.4Hz),6.95(1H,dt ,J=8.2,1.5Hz,Hz),7.08(1H,dd,J=2.9,1.5Hz,Hz),7.16-7.22(1H,m),7.28-7.38(3H,m).

### Example 104

Ethyl 3-(3-((4R,6S)-8-chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylate (isomer A) Ethyl 3-(3-((4R,6S)-8-chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylate (isomer B)

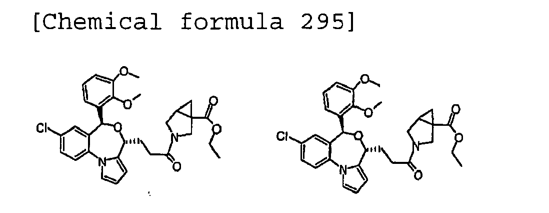

Ethyl 3-(3-((4R,6S)-8-chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylate (600 mg) was dissolved in 25% isopropanol-hexane (30 mL). The resulting solution was separated by chiralcel OD (35% isopropanol-hexane) into the isomer A (85 mg) and the isomer B (105 mg).
Isomer A
MS(ESI)m/z:565(M⁺+H) .
¹H-NMR(CDCl₃)δ:0.62-0.80(1H,m),1.26(3H,t,J=7.1Hz),1.52-1.63(1H,m),2.04-2.14(1H,m),2.31-2.46(2H,m),2.51-2.64(2H,m),3.43(3H,s),3.57-3.98(7H,m),4.17(2H,q,J=7.1Hz),4.34-4.41(1H,m),5.70(1H,s),6.33-6.35(1H,m),6.37(1H,t,J=3.2Hz),6.71(1H,t,J=2.4Hz),6.95(1H,dt ,J=8.2,1.5Hz,Hz),7.08(1H,dd,J=2.9,1.5Hz,Hz),7.16-7.22(1H,m),7.28-7.38(3H,m).
Isomer B
MS(ESI)m/z:565(M⁺+H).
¹H-NMR(CDCl₃)δ:0.67-0.81(1H,m), 1.23-1.28(3H,m), 1.53-1.66(1H,m),2.06-2.14(1H,m),2.34-2.65(4H,m),3.41-3.44(3H,m),3.59-3.98(7H,m),4.12-4.20(2H,m),4.33-4.42(1H,m),5.69-5.71(1H,m),6.32-6.39(2H,m),6.71(1H,t,J=2.4Hz),6.93-6.97(1H,m,Hz),7.07-7.09(1H,m,Hz),7.19(1H,dd,J=16.4,8.3Hz),7.25-7.37(3H,m).

### Example 105

3-(3-((4R,6S)-8-Chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylic acid (isomer A)

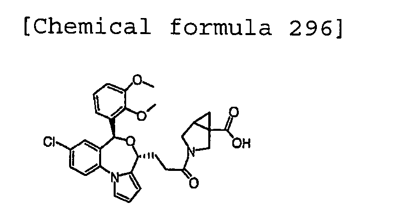

To a methanol solution (5.0 mL) of ethyl 3-(3-((4R,6S)-8-chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylate (isomer A) (81 mg) were added distilled water (5.0 mL) and potassium carbonate (59 mg). The resulting mixture was stirred at 50°C for 17 hours. The reaction mixture was adjusted to pH 4.0 with an ion exchange resin "IR-120B". The resin was filtered out and the solvent was distilled off. The residue was purified by preparative thin layer chromatography (development phase: a 7:3:1=chloroform:methanol:water organic layer), followed by lyophilization from 1,4-dioxane-water to give the title compound (67 mg).
MS (ESI)m/z:537 (M⁺+H) .
¹H-NMR(CDCl₃)δ:0.68-0.79(1H,m),1.58-1.65(1H,m),2.08-2.17(1H,m),2.32-2.42(2H,m),2.50-2.60(2H,m),3.39-3.48(4H,m),3.55-3.62(1H,m),3.70-3.79(1H,m),3.82-3.97(4H,m),4.34-4.39(1H,m),5.68-5.71(1H,m),6.31-6.39(2H,m),6.69-6.72(1H,m),6.91-6.96(1H,m),7.05-7.09(1H,m),7.14-7.21(1H,m),7.24-7.39(3H,m).

### Example 106

3-(3-((4R,6S)-8-Chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylic acid(isomer B)

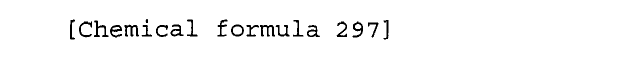

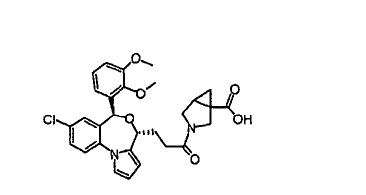

To a methanol solution (5.0 mL) of ethyl 3-(3-((4R,6S)-8-chloro-6-(2,3-dimethoxyphenyl)-4H,6H-pyrrolo(1,2-a)(4,1)benzoxazepin-4-yl)propanoyl)-3-azabicyclo(3.1.0)hexane-1-carboxylate (isomer B) (105 mg) were added distilled water (5.0 mL) and potassium carbonate (77 mg). The resulting mixture was stirred at 50°C for 17 hours. The reaction mixture was adjusted to pH 4.0 with an ion exchange resin "IR-120B". The resin was filtered out and the solvent was distilled off. The residue was purified by preparative thin layer chromatography (development phase: a 7:3:1=chloroform:methanol:water organic layer), followed by lyophilization from 1,4-dioxane-water to give the title compound (75 mg).
MS(ESI)m/z:537(M⁺+H).
¹H-NMR(CDCl₃)δ:0.72-0.91(1H,m),1.59-1.72(1H,m),2.11-2.22(1H,m),2.30-2.65(4H,m),3.40-3.51(4H,m),3.59-3.72(1H,m),3.74-3.98(5H,m),4.33-4.44(1H,m),5.69-5.71(1H,m),6.32-6.38(2H,m),6.69-6.73(1H,m),6.92-6.97(1H,m),7.06-7.09(1H,m),7.15-7.22(1H,m),7.24-7.38(3H,m).

### Example 107

ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-5-yl)acetate

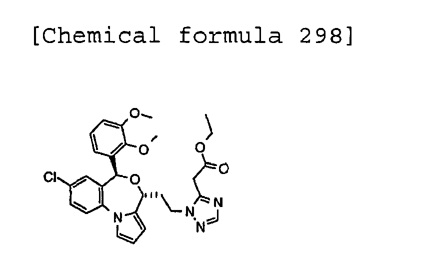

Ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-yl)acetate

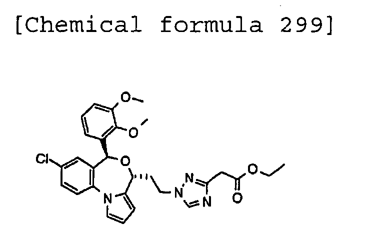

Ethyl 2-(1H-1,2,4-triazol-3-yl)acetate (87.0 mg) was dissolved in N,N-dimethylformamide (2 mL). At 0°C, sodium hydride (55%, 36.7 mg) was added to the resulting solution, followed by stirring at 0°C for 30 minutes. An N,N-dimethylformamide solution (2 mL) of 2-[(4R, 6S)-8-Chloro-6-(2,3-dimethoxyphenyl)-4H,6H-pyrrolo[1,2-a] [4,1]benzoxazepin-4-yl]ethyl methanesulfonate (134 mg) was added dropwise. Tetrabutylammonium iodide (104 mg) was added further and the resulting mixture was stirred overnight at 80°C. After addition of water, the resulting mixture was extracted with ethyl acetate. The organic layer was dried over anhydrous sodium sulfate. The filtrate was then concentrated. The residue thus obtained was purified by preparative TLC (ethyl acetate:hexane=1:1) to give the title compounds, that is, the 5-isomer (53.1 mg) and 3-isomer (68.3 mg).
5-isomer (low polarity fraction)
MS (ESI) m/z : 537 (M⁺+H).
¹H-NMR(CDCl₃)δ:1.21-1.26(3H,m),2.60-2.65(2H,m),3.42-3.43(3H,m),3.79-3.92(5H,m),4.14-4.19(2H,m),4.35-4.48(3H,m),5.73(1H,s),6.31-6.32(1H,m),6.36-6.38(1H,m),6.73-6.74(1H,m),6.95-6.97(1H,m),7.09-7.10(1H,m),7.16-7.21(1H,m),7.27-7.30(1H,m),7.32-7.39(2H,m),7.83-7.83(1H,m). 3-isomer (high polarity fraction)
MS(ESI)m/z:537(M⁺+H) .
¹H-NMR(CDCl₃)δ:1.22-1.27(3H,m),2.58-2.63(2H,m),3.43-3.43(3H,m),3.75-3.75(2H,m),3.86-3.87(3H,m),4.14-4.22(2H,m),4.33-4.37(1H,m),4.40-4.49(2H,m),5.74(1H,s),6.30-6.32(1H,m),6.37-6.38(1H,m),6.71-6.73(1H,m),6.96-6.98(1H,m),7.08-7.10(1H,m),7.19-7.23(1H,m),7.27-7.29(1H,m),7.32-7.38(2H,m),8.02-8.02(1H,m).

### Example 108

methyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-5-carboxylate

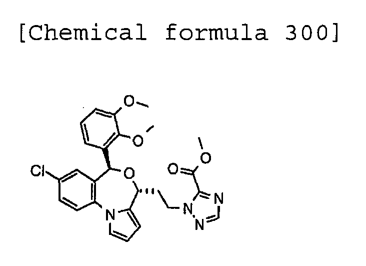

methyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-carboxylate

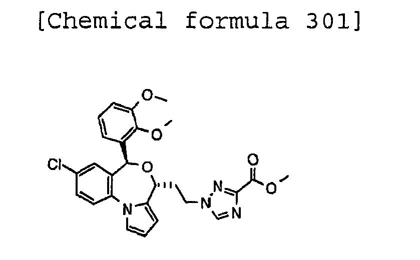

In a similar manner to that employed for the synthesis of ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-5-yl)acetate and ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-yl)acetate, 2-[(4R, 6S)-8-chloro-6-(2,3-dimethoxyphenyl)-4H,6H-pyrrolo[1,2-a][4, 1] benzoxazepin-4-yl]ethyl methanesulfonate (145 mg) and methyl 1H-1,2,4-triazol-3-carboxylate (77.1 mg) were treated, whereby the title compounds, that is, 5-isomer (51.8 mg) and 3-isomer (88.5 mg) were obtained. 5-isomer (Low polarity fraction)
MS (ESI)m/z:509 (M⁺+H).
¹H-NMR(CDCl₃)δ:2.61-2.68(2H,m),3.43(3H,s),3.86(3H,s),3.96(3H,s),4.40-4.43(1H,m),4.90-4.96(2H,m),5.73(1H,s),6.34-6.36(1H,m),6.37-6.39(1H,m),6.72-6.74(1H,m),6.95-6.97(1H,m),7.09-7.10(1H,m),7.19-7.23(1H,m),7.32-7.39(3H,m),7.94-7.94(1H,m). 3-isomer (High polarity fraction)
MS(ESI)m/z:509(M⁺+H).
¹H-NMR(CDCl₃)δ:2.60-2.69(2H,m),3.43-3.44(3H,m),3.86-3.87(3H,m),3.97-3.98(3H,m),4.30-4.35(1H,m),4.54-4.62(2H,m),5.75(1H,s),6.29-6.30(1H,m),6.36-6.38(1H,m),6.72-6.73(1H,m),6.96-6.98(1H,m),7.09-7.10(1H,m),7.21-7.27(2H,m),7.32-7.39(2H,m),8.18-8.18(1H,m).

### Example 109

Ethyl 1-{2-[(4S,6R)-8-chloro-6-(2,3-dimethoxyphenyl)-4H,6H-pyrrolo[1,2-a][4,1]benzoxazepin-4-yl]ethyl}-1H-pyrazole-5-carboxylate

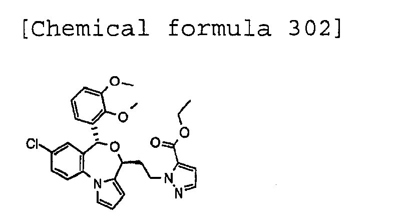

Ethyl 1-{2-[trans-8-chloro-6-(2,3-dimethoxyphenyl)-4H,6H-pyrrolo[1,2-a][4,1]benzoxazepin-4-yl]ethyl}-1H-pyrazole-3-carboxylate

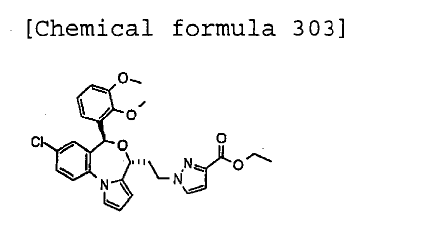

In a similar manner to that employed for the synthesis of ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-5-yl)acetate and ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-yl)acetate, 2-[trans-8-chloro-6-(2,3-dimethoxyphenyl)-4H,6H-pyrrolo[1,2-a][4,1]benzoxazepin-4-yl]ethyl methanesulfonate (389 mg) and ethyl 1H-pyrazole-3-carboxylate (160 mg) were treated, whereby the title compounds, that is, the 5-isomer (196 mg) and 3-isomer (201 mg) were obtained. The 5-isomer (racemic mixture), the title compound, was subjected to optical resolution with CHIRALCEL-OD (flow rate: 15 mL/min, developing solvent: 20% 2-propanol-hexane, retention time: 8 minutes for isomer A, 12 minutes for isomer B), whereby the isomer A (92.4 mg) with a shorter retention time and the isomer B (82.9 mg) with a longer retention time were obtained, respectively. 5-Isomer (low polarity fraction)
MS(ESI)m/z:522 (M⁺+H).
¹H-NMR(CDCl₃)δ:1.33(3H,t,J=7.2Hz),2.50-2.66(2H,m),3.43(3H,s),3.85(3H,s),4.30(2H,q,J=7.2Hz),4.40-4.45(1H,m),4.83-4.88(2H,m),5.72(1H,s),6.36-6.37(2H,m),6.73(1H,d,J=2.2Hz),6.80(1H,d,J=2.0Hz),6.94-6.96(1H,m);7.08(1H,t,J=2.2Hz),7.19(1H,t,J=7.9Hz),7.31-7.37(2H,m),7.41-7.45(2H,m).
3-Isomer (high polarity fraction)
MS(ESI)m/z:522 (M⁺+H).
¹H-NMR(CDCl₃)δ:1.38(3H,t,J=7.2Hz),2.59-2.66(2H,m),3.43(3H,s),3.86(3H,s),4.28-4.33(1H,m),4.38(2H,q,J=7.2Hz),4.44-4.52(1H,m),4.53-4.61(1H,m),5.74(1H,s),6.29-6.30(1H,m),6.36(1H,t,J=3.3Hz),6.72(1H,d,J=2.2Hz),6.75(1H,d, J=2.2Hz),6.97(1H,dd,J=8.1,1.5Hz),7.07-7.09(1H,m),7.21(1H,t,J=7.9Hz),7.30-7.38(3H,m),7.44(1H,d,J=2.4Hz).

### Example 110

2-(1-{2-[(4R,6S)-8-Chloro-6-(2,3-dimethoxyphenyl-4H,6H-pyrrolo[1,2-a][4,1]benzoxazepin-4-yl)ethyl]-1H-1,2,4-triazol-3-yl}acetic acid

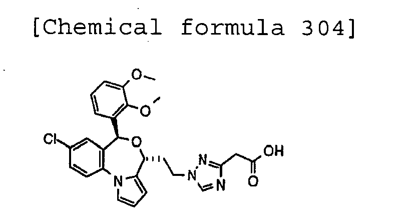

Ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-yl)acetate (68.3 mg) was dissolved in methanol-water-tetrahydrofuran (1:0.5:1, 2.5mL). To the resulting mixture was added potassium carbonate (52.7 mg). The resulting mixture was stirred overnight at room temperature. The reaction mixture was neutralized with an ion exchange resin (Amberlite IR-120B) and the resin was filtered out. The filtrate was concentrated. The residue thus obtained was purified by preparative TLC (lower layer of methanol:chloroform:water=3:7:1) to give the title compound (52.1 mg).
MS(ESI)m/z:509(M⁺+H).
¹H-NMR(CDCl₃)δ:2.51-2.57(2H,m),3.40(3H,s),3.60-3.66(2H,m),3.83(3H,s),4.26-4.43(3H,m),5.70(1H,s),6.25-6.28(1H,m),6.31-6.33(1H,m),6.69-6.71(1H,m),6.91-6.93(1H,m),7.04-7.06(1H,m),7.16(1H,t,J=8.1Hz),7.23-7.25(1H,m),7.28-7.34(2H,m),8.22-8.24(1H,m).

### Example 111

2-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,4-triazol-5-yl)acetic acid

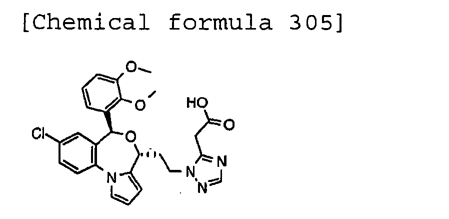

In a similar manner to that employed for the synthesis of 2-(1-{2-[(4R,6S)-8-chloro-6-(2,3-dimethoxyphenyl-4H,6H-pyrrolo[1,2-a][4,1]benzoxazepin-4-yl)ethyl]-1H-1,2,4-triazol-3-yl}acetic acid, ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-5-yl)acetate (53.0 mg) and potassium carbonate (40.9 mg) were treated, whereby the title compound (45.9 mg).
MS(ESI)m/z:509(M⁺+H).
¹H-NMR(CDCl₃)δ:2.45-2.52(2H,m),3.39(3H,s),3.53-3.60(2H,m),3.80(3H,s),4.22-4.37(3H,m),5.68(1H,s),6.24-6.26(1H,m),6.28-6.30(1H,m),6.69-6.71(1H,m),6.87-6.89(1H,m),7.01-7.04(1H,m),7.14(1H,t,J=7.6Hz),7.27-7.31(3H,m),7.56-7.58(1H,m).

### Example 112

1-{2-[(4R,6S)-8-Chloro-6-(2,3-dimethoxyphenyl)-4H,6H-pyrrolo[1,2-a][4,1]benzoxazepin-4-yl]ethyl}-1H-1,2,4-triazol-3-carboxylic acid

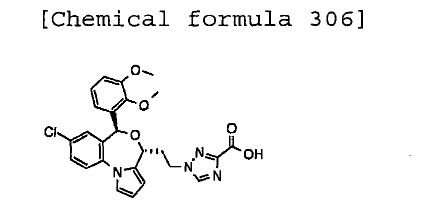

In a similar manner to that employed for the synthesis of 2-(1-{2-[(4R,6S)-8-chloro-6-(2,3-dimethoxyphenyl-4H,6H-pyrrolo[1,2-a][4,1]benzoxazepin-4-yl)ethyl]-1H-1,2,4-triazol-3-yl}acetic acid, methyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-carboxylate (88.5 mg) was treated while using potassium carbonate (72.1 mg) to give the title compound (36.8 mg) .
MS(ESI)m/z:495(M⁺+H). ¹H-NMR(CDCl₃)δ:2.03-2.17(1H,m),2.40-2.52(1H,m),3.35(3H,s),3.80(3H,s),4.28-4.37(2H,m),4.47-4.53(1H,m),5.66(1H,s),6.18-6.27(2H,m),6.65-6.68(1H,m),6.87-6.89(1H,m),6.97-7.00(1H,m),7.09-7.12(1H,m),7.23-7.26(3H,m),7.30-7.36(1H,m).

### Example 113

1-{2-[(4R,6S)-8-Chloro-6-(2,3-dimethoxyphenyl)-4H,6H-pyrrolo[1,2-a][4,1]benzoxazepin-4-yl]ethyl}-1H-pyrazole-5-carboxylic acid

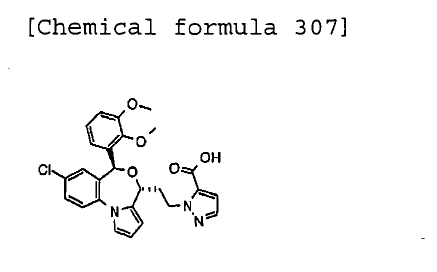

In a similar manner to that employed for the synthesis of 2-(1-{2-[(4R,6S)-8-chloro-6-(2,3-dimethoxyphenyl-4H,6H-pyrrolo[1,2-a][4,1]benzoxazepin-4-yl)ethyl]-1H-1,2,4-triazol-3-yl}acetic acid, ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-5-carboxylate (92.4 mg) and potassium carbonate (74.3 mg) were treated, whereby the title compound (66.4 mg).
MS(ESI)m/z:494 (M⁺+H).
¹H-NMR(CDCl₃)δ:2.37-2.50(2H,m),3.34(3H,s),3.70(3H,s),4.37-4.42(1H,m),4.48-4.57(1H,m),4.77-4.85(1H,m),5.73(1H,s),6.16-6.18(1H,m),6.20-6.23(1H,m),6.53-6.55(1H,m),6.68(1H,d,J=2.2Hz),6.78-6.80(1H,m),6.99-7.00(1H,m),7.11(1H,t,J=7.9Hz),7.22-7.24(1H,m),7.28-7.32(3H,m).

### Example 114

1-{2-[trans-8-Chloro-6-(2,3-dimethylphenyl)-4H,6H-pyrrolo[1,2-a][4,1]benzoxazepin-4-yl]ethyl}-1H-pyrazole-3-carboxylic acid

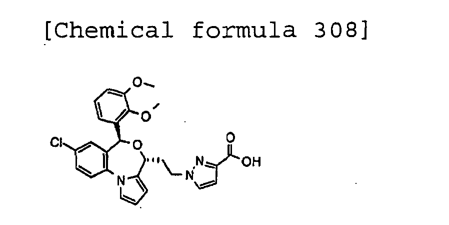

In a similar manner to that employed for the synthesis of 2-(1-{2-[(4R,6S)-8-chloro-6-(2,3-dimethoxyphenyl-4H,6H-pyrrolo[1,2-a][4,1]benzoxazepin-4-yl)ethyl]-1H-1,2,4-triazol-3-yl}acetic acid, ethyl 1-{2-[trans-8-chloro-6-(2,3-dimethoxyphenyl)-4H,6H-pyrrolo[1,2-a][4,1]benzoxazepin-4-yl]ethyl}-1H-pyrazole-3-carboxylate (157 mg) and potassium carbonate (125 mg) were treated, whereby the title compound (116 mg).
MS(ESI)m/z:494 (M⁺+H). ¹H-NMR(CD₃OD)δ:2.35-2.46(2H,m), 3.35(3H,s),3.85(3H,s),4.14-4.20(1H,m),4.33-4.45(2H,m),5.61-5.63(1H,m),6.20-6.22(1H,m),6.30-6.33(1H,m),6.54-6.56(1H,m),6.60-6.63(1H,m),7.05-7.07(1H,m),7.14-7.17(1H,m),7.20-7.29(2H,m),7.39-7.41(2H,m),7.56-7.58(1H,m).

### Example 115

Ethyl 2-[[(1-{2-[trans-8-chloro-6-(2,3-dimethoxyphenyl)-4H,6H-pyrrolo[1,2-a][4,1]benzoxazepin-4-yl]ethyl}-1H-pyrazol-3-yl)carboxyl](methyl)amino]acetate

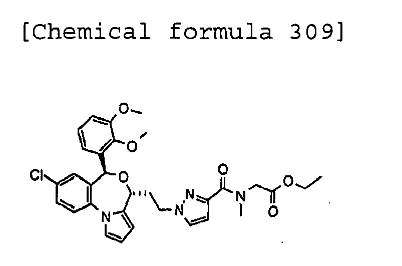

1-{2-[trans-8-Chloro-6-(2,3-dimethylphenyl)-4H,6H-pyrrolo[1,2-a][4,1]benzoxazepin-4-yl]ethyl}-1H-pyrazole-3-carboxylic acid (73.0 mg) and sarcosine ethyl ester hydrochloride (27.2 mg) were dissolved in dichloromethane (2 mL). To the resulting solution were added 1-ethyl-3-(3-dimethylaminopropyl)carbodiimide hydrochloride (34.0 mg), 1-hydroxybenzotriazole (6.8 mg), and triethylamine (0.025 mL). The resulting mixture was stirred overnight at room temperature. The reaction mixture was diluted with chloroform and washed with saturated brine. The organic layer was dried over anhydrous sodium sulfate. The filtrate was then concentrated. The residue thus obtained was purified by preparative TLC (ethyl acetate:hexane=1:2) to give the title compound (92.5 mg).
MS(ESI)m/z:593(M⁺+H) .
¹H-NMR(CDCl₃)δ:1.24-1.30(3.0H,m),2.50-2.63(2.0H,m),3.13(1.5H,s),3.35(1.5H,s),3.43-3.44(3.0H,m),3.86(3.0H,s),4.11-4.14(1.OH,m),4.18-4.24(2.0H,m),4.29-4.48(3.0H,m),4.52-4.65(1.OH,m),5.73-5.75(1.0H,m),6.30-6.32(1.0H,m),6.37-6.38(1.0H,m),6.71-6.75(2.0H,m),6.96-6.98(1.0H,m),7.09-7.10(1.0H,m),7.19-7.24(1.0H,m),7.29-7.31(1.0H,m),7.33-7.40(3.0H,m).

### Example 116

2-[[(1-{2-[trans-8-Chloro-6-(2,3-dimethoxyphenyl)-4H,6H-pyrrolo [1, 2-a] [4,1]benzoxazepin-4-yl]ethyl}-1H-pyrazol-3-yl) carboxyl] (methyl) amino] acetic acid

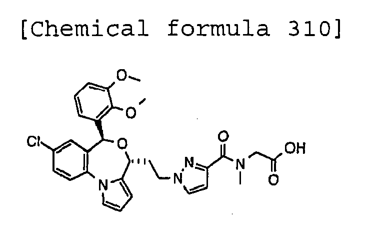

In a similar manner to that employed for the synthesis of ethyl 2-[[(1-{2-[trans-8-chloro-6-(2,3-dimethoxyphenyl)-4H,6H-pyrrolo[1,2-a][4,1]benzoxazepin-4-yl]ethyl}-1H-pyrazol-3-yl)carboxyl](methyl)amino]acetate, ethyl 2-[[(1-{2-[trans-8-chloro-6-(2,3-dimethoxyphenyl)-4H,6H-pyrrolo[1,2-a][4,1]benzoxazepin-4-yl]ethyl}-1H-pyrazol-3-yl)carboxyl](methyl)amino]acetate (92.5 mg) was treated while using potassium carbonate (64.7 mg) to give the title compound (84.5 mg).
MS(ESI)m/z:465(M⁺+H).
¹H-NMR(CDCl₃)δ:2.44-2.52(2H,m),3.09-3.12(2H,m),3.41(3H,s),3.71-3.71(4H,m),3.78-3.81(1H,m),3.83(3H,s),4.27-4.39(2H,m),5.71(1H,s),6.22-6.25(1H,m),6.28-6.30(1H,m),6.50-6.52(1H,m),6.68-6.70(1H,m),6.92-6.94(1H,m),7.01-7.03(1H,m),7.17-7.19(1H,m),7.22-7.25(1H,m),7.27-7.32(3H,m).

### Referential Example 42

3-[(4R,6S)-8-Chloro-6-(2,3-dimethoxyphenyl)-4H,6H-pyrrolo[1,2-a] [4,1]benzoxazepin-4-yl]-N-[2-cyanoethyl]propanamide

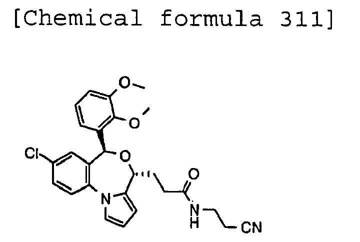

3-[(4R,6S)-8-Chloro-6-(2,3-dimethoxyphenyl)-4H,6H-pyrrolo[1,2-a][4,1]benzoxazepin-4-yl]propionic acid (200 mg) and cyanoethylamine (0.052 mL) were dissolved in dichloromethane (4 mL). To the resulting solution were added 1-ethyl-3-(3-dimethylaminopropyl)carbodiimide hydrochloride (108 mg) and 1-hydroxybenzotriazole (21.5 mg). The resulting mixture was stirred overnight at room temperature. The reaction mixture was diluted with chloroform and washed with saturated brine. The organic layer was dried over anhydrous sodium sulfate. The filtrate was then concentrated. The residue thus obtained was purified by preparative TLC (methanol:chloroform=1:10) to give the title compound (232 mg).
MS(ESI)m/z:480(M⁺+H).
¹H-NMR(CDCl₃)δ:2.36-2.41(2H,m),2.46-2.62(4H,m),3.40-3.44(2H,m),3.44(3H,s),3.86(3H,s),4.39-4.43(1H,m),5.74(1H,s),6.25(1H,br s),6.32-6.33(1H,m),6.37-6.39(1H,m),6.73(1H,d,J=2.2Hz),6.97(1H,dd,J=8.1,1.5Hz),7.09-7.10(1H,m),7.21(1H,t,J=8.1Hz),7.27-7.29(1H,m),7.33-7.39(2H,m).

### Referential Example 43

3-(5-{2-[(4R,6S)-8-Chloro-6-(2,3-dimethoxyphenyl)-4H,6H-pyrrolo[1,2-a] [4,1]benzoxazepin-4-yl]ethyl}1H-1,2,3,4-tetrazol-1-yl)propanenitrile

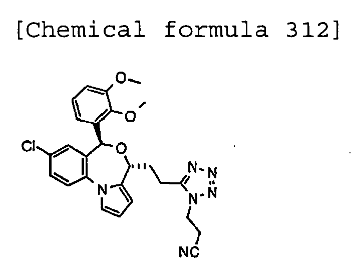

3-[(4R,6S)-8-chloro-6-(2,3-dimethoxyphenyl)-4H,6H-pyrrolo[1,2-a] [4,1]benzoxazepin-4-yl]-N-[2-cyanoethyl]propanamide (224 mg), triphenylphosphine (135 mg), and trimethylsilylazide (0.068 mL) were dissolved in tetrahydrofuran (10 mL). Under a nitrogen atmosphere, a 40% diethylazodicarboxylate toluene solution (0.234 mL) was added and the resulting mixture was stirred overnight at room temperature. To the reaction mixture was added a saturated aqueous solution of sodium bicarbonate, followed by extraction with ethyl acetate. The organic layer was dried over anhydrous sodium sulfate. The filtrate was then concentrated. The residue thus obtained was purified by preparative TLC (methanol:chloroform=1:100) to give the title compound (613 mg) as a mixture with a byproduct.
MS(ESI)m/z:505(M⁺+H)

### Referential Example 44

(4R,6S)-8-chloro-6-(2,3-dimethoxyphenyl)-4-[2-(1H-1,2,3,4-tetrazol-5-yl)ethyl]-4H,6H-pyrrolo[1,2-a][4,1]benzoxazepine

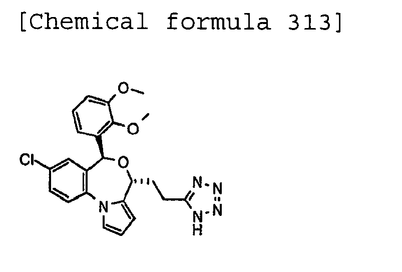

3-(5-{2-[(4R,6S)-8-Chloro-6-(2,3-dimethoxyphenyl)-4H,6H-pyrrolo[1,2-a][4,1]benzoxazepin-4-yl]ethyl}1H-1,2,3,4-tetrazol-1-yl)propanenitrile (613 mg) was dissolved in dichloromethane (15 mL). To the resulting solution was added 1.8-diazabicyclo[5.4.0]-7-undecene (0.454 mL), followed by stirring overnight at room temperature. The reaction mixture was concentrated. The residue thus obtained was purified by preparative TLC (methanol:chloroform=1:100) to give the title compound (161 mg).
MS(ESI)m/z:452(M⁺+H).
¹H-NMR(CDCl₃)δ:2.46-2.54(2H,m),3.19-3.28(1H,m),3.36-3.44(1H,m),3.64(3H,s),3.90(3H,s),4.49-4.53(1H,m),5.80(1H,s),6.32-6.35(1H,m),6.40(1H,t,J=3.3Hz),6.85(1H,d,J=2.2Hz),7.01-7.03(1H,m),7.09-7.12(2H,m),7.20(1H,t,J=8.3Hz),7.37(1H,d,J=8.3Hz),7.42(1H,dd ,J=8.3,2.3Hz).

### Example 117

Ethyl 2-(5-{2-[,(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-2-yl)acetate

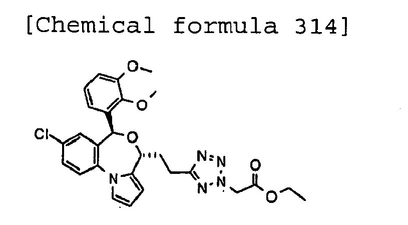

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-1-yl)acetate

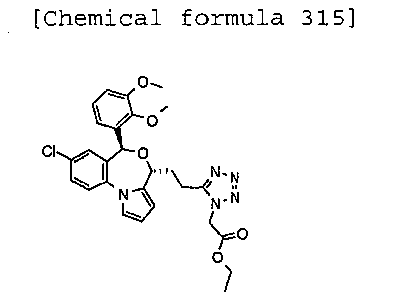

(4R,6S)-8-Chloro-6-(2,3-dimethoxyphenyl)-4-[2-(1H-1,2,3,4-tetrazol-5-yl)ethyl]-4H,6H-pyrrolo[1,2-a][4,1]benzoxazepine (161 mg) was dissolved in N,N-dimethylformamide (3 mL). To the resulting solution were added potassium carbonate (73.9 mg) and ethylbromoacetate (0.079 mL). The resulting mixture was stirred overnight at room temperature. The reaction mixture was concentrated. The concentrate was diluted with ethyl acetate and washed with saturated brine. The organic layer was dried over anhydrous sodium sulfate. The filtrate was then concentrated. The residue thus obtained was purified by preparative TLC (ethyl acetate:hexane=2:3) to give the title compounds, 2-yl isomer (63.2 mg) and 1-yl isomer (80.8 mg).
The 2-yl isomer
MS(ESI)m/z:538 (M⁺+H).
¹H-NMR(CDCl₃)δ:1.24(3H,t,J=7.1Hz),2.52-2.58(2H,m),3.09-3.18(1H,m),3.27-3.35(1H,m),3.42(3H,s),3.83(3H,s),4.22(2H,q,J=7.1Hz),4.40-4.44(1H,m),5.31(2H,s),5.71(1H,s),6.34-6.37(2H,m),6.68-6.70(1H,m),6.91-6.94(1H,m),7.07-7.08(1H,m),7.17(1H,t,J=7.9Hz),7.29-7.35(3H,m).
The 1-yl isomer
MS(ESI)m/z:538(M⁺+H).
¹H-NMR(CDCl₃)δ:1.23(3H,t,J=7.2Hz),2.56-2.62(2H,m),2.99-3.06(1H,m),3.13-3.21(1H,m),3.40(3H,s),3.83(3H,s),4.21(2H,q,J=7.2Hz),4.45(1H ,dd,J=7.8,5.1Hz),5.05(2H,dd,J=20.9,17.7Hz),5.72(1H,s),6.31-6.32(1H,m),6.36(1H,t,J=3.3Hz),6.69(1H,d,J=2.0Hz),6.93(1H,dd ,J=7.8,2.2Hz),7.07-7.09(1H,m),7.14-7.17(2H,m),7.31-7.37(2H,m).

### Example 118

2-(5-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-2-yl)acetic acid

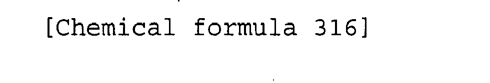

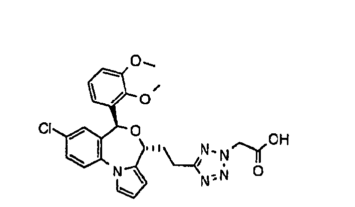

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-2-yl)acetate (63.2 mg) was dissolved in methanol-water-tetrahydrofuran (1:0.5:1, 2.5 mL). Potassium carbonate (48.7 mg) was added and the resulting mixture was stirred at room temperature for 4 days. The reaction mixture was neutralized with an ion exchange resin (Amberlite IR-120B). After the resin was filtered out and the filtrate was concentrated, the residue thus obtained was purified by preparative TLC (lower layer of methanol:chloroform:water=3:7:1) to give the title compound (43.6 mg).
MS (ESI) m/z : 508 (M⁺-H) .
¹H-NMR(CD₃OD)δ:2.48-2.59(2H,m),3.07-3.15(1H,m),3.20-3.27(1H,m),3.38(3H,s),3.85(3H,s),4.43-4.48(1H,m),5.13-5.15(2H,m),5.66(1H,s),6.37-6.40(2H,m),6.61-6.63(1H,m),7.06-7.08(1H,m),7.21-7.24(2H,m),7.31-7.33(1H,m),7.44-7.50(2H,m).

### Example 119

2-(5-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-1-yl)acetic acid

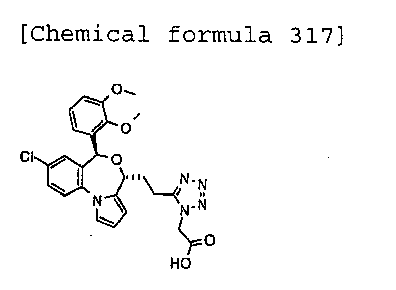

In a similar manner to that employed for the synthesis of 2-(5-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-2-yl)acetic acid, ethyl 2-(5-{2-[(4R,6S)-8-chloro-6-(2,3-dimethoxyphenyl)-4H,6H-pyrrolo[1,2-a] [4,1]benzoxazepin-4-yl]ethyl}-1H-1,2,3,4-tetrazol-1-yl)acetate (80.8 mg) was treated while using potassium carbonate (62.3 mg) to give the title compound (73.5 mg).
MS(ESI)m/z:510(M⁺+H).
¹H-NMR(CD₃OD)δ:2.55-2.62(2H,m),3.05-3.13(1H,m),3.18-3.26(1H,m),3.37-3.39(3H,m),3.85-3.87(3H,m),4.47-4.53(1H,m),4.94-4.96(2H,m),5.65-5.67(1H,m),6.39-6.42(2H,m),6.62-6.65(1H,m),7.06-7.09(1H,m),7.21-7.30(3H,m),7.44-7.52(2H,m).

### Referential Example 45

(4R,6S)-4-(2-Azidoethyl)-8-chloro-6-(2,3-dimethoxyphenyl)-4H,6H-pyrrolo[1,2-a] [4,1]benzoxazepine

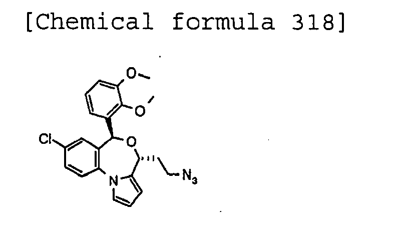

2-[(4R,6S)-8-Chloro-6-(2,3-dimethoxyphenyl)-4H,6H-pyrrolo[1,2-a][4,1]benzoxazepin-4-yl]ethyl methanesulfonate (891 mg) was dissolved in N,N-dimethylformamide-water (10:1, 22 mL). To the resulting solution was added sodium azide (727 mg). The resulting mixture was stirred overnight at 80°C. After the reaction mixture was concentrated, the concentrate was diluted with ethyl acetate and then washed with water. The organic layer was dried over anhydrous sodium sulfate, whereby the title compound (780 mg).

### Example 120

Ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylate

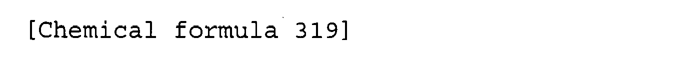

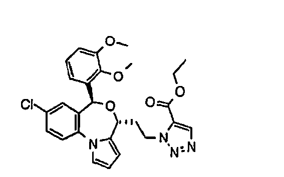

Ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-carboxylate

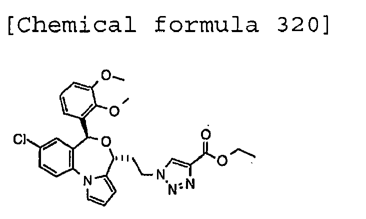

(4R,6S)-4-(2-Azidoethyl)-8-chloro-6-(2,3-dimethoxyphenyl)-4H,6H-pyrrolo[1,2-a][4,1]benzoxazepine (180 mg) was dissolved in toluene (5 mL). Ethyl propiolate (0.094 mL) was added and the mixture was heated under reflux overnight. After the reaction mixture was concentrated, the residue was purified by preparative TLC (methanol:chloroform=1:100) to give the title compounds, 5-isomer (61.4 mg) and 4-isomer (140 mg).
The 5-isomer (low polarity fraction)
MS (ESI) m/z : 523 (M⁺+H) .
¹H-NMR(CDCl₃)δ:1.36(3H,t,J=7.1Hz),2.57-2.74(2H,m),3.44(3H,s),3.86(3H,s),4.35(2H,q,J=7.1Hz),4.44-4.47(1H,m),5.03(2H,t,J=7.4Hz),5.74(1H,s),6.35-6.39(2H,m),6.73(1H,d,J=2.2Hz),6.96(1H,dd,J=7.9,1.5Hz),7.09-7.10(1H,m),7.21(1H,t,J=7.9Hz),7.32-7.41(3H,m),8.10(1H,s).
The 4-isomer (high polarity fraction)
MS(ESI)m/z:523 (M⁺+H) .
¹H-NMR(CDCl₃)δ:1.39(3H,t,J=7.2Hz),2.62-2.74(2H,m),3.44(3H,s),3.86(3H,s),4.38-4.43(3H,m),4.70-4.76(2H,m),5.76(1H,s),6.30-6.31(1H,m),6.38(1H,t,J=3.3Hz),6.74(1H,d,J=2.2Hz),6.96-6.99(1H,m),7.10-7.11(1H,m),7.21(1H,t,J=7.9Hz),7.27-7.29(1H,m),7.33-7.39(2H,m),8.10(1H,s).

### Example 121

Ethyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]acetate

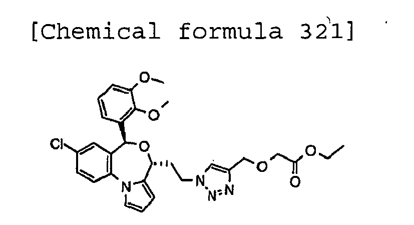

Ethyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)methoxy]acetate

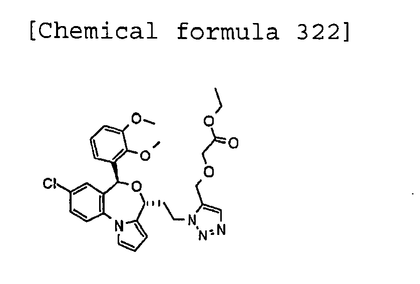

In a similar manner to that employed for the synthesis of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylate and ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-carboxylate, (4R,6S)-4-(2-azidoethyl)-8-chloro-6-(2,3-dimethoxyphenyl)-4H,6H-pyrrolo[1,2-a][4,1]benzoxazepine (151 mg) and ethyl 2-(2-propynyloxy)acetate (151 mg) were treated, whereby the title compounds, 4-isomer (69.7 mg) and 5-isomer (50.0 mg) were obtained.
4-Isomer (low polarity fraction)
MS (ESI) m/z:567(M⁺+H)
¹H-NMR(CDCl₃)δ:1.28(3H,t,J=7.2Hz),2.63-2.68(2H,m),3.44(3H,s),3.86(3H,s),4.12-(2H,s),4.21(2H,q,J=7.2 Hz),4.38-4.42(1H,m),4.64-4.70(2H,m),4.72(2H,s),5.76(1H,s),6.30-6.31(1H,m),6.37-6.39(1H,m),6.74(1H,d,J=2.2Hz),6.97(1H,dd,J=8.1,1.5Hz),7.10-7.11(1H,m),7.22(1H,t,J=8.1Hz),7.29-7.39(3H,m),7.65(1H,s). 5-Isomer (high polarity fraction)
MS(ESI)m/z:567(M⁺+H)
¹H-NMR(CDCl₃)δ:1.25(3H,t,J=7.2Hz),2.63-2.78(2H,m),3.43(3H,s),3.86(3H,s),4.01(2H,s),4.17(2H,q,J=7.2 Hz),4.46-4.49(1H,m),4.64-4.79(4H,m),5.74(1H,s),6.32-6.34(1H,m),6.37-6.38(1H,m),6.73(1H,d,J=2.0Hz),6.96(1H,dd,J=8.2,1.3Hz),7.09-7.10(1H,m),7.19(1H,t,J=8.2Hz),7.32-7.38(3H,m),7.61(1H,s).

### Example 122

Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-methylpropanoate

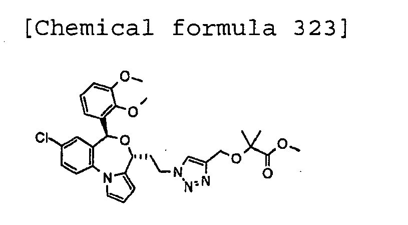

Methyl 2-[(1-{2-[(4R,6S)-8-chloro-6-(2,3-dimethoxyphenyl)-4H,6H-pyrrolo[1,2,-a][4,1]benzoxazepin-4-yl]ethyl}-1H-1,2,3-triazol-5-yl)methoxy]-2-methylpropanoate

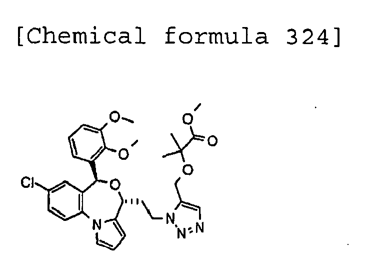

In a similar manner to that employed for the synthesis of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylate and ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-carboxylate, (4R,6S)-4-(2-azidoethyl)-8-chloro-6-(2,3-dimethoxyphenyl)-4H,6H-pyrrolo[1,2-a] [4,1]benzoxazepine (145 mg) and methyl 2-methyl-2-(2-propynyloxy)propanoate (160 mg) were treated while using ethyl 2-(2-propynyloxy)acetate (151 mg) to give the title compounds 4-isomer (69.2 mg) and 5-isomer (50.1 mg). 4-Isomer (low polarity fraction)
MS (ESI)m/z:581 (M⁺+H) .
¹H-NMR(CDCl₃)δ:1.47-1.48(6H,m),2.56-2.73(2H,m),3.43-3.44(3H,m),3.73-3.73(3H,m),3.86-3.86(3H,m),4.40-4.42(1H,m),4.58-4.59(2H,m),4.62-4.69(2H,m),5.76(1H,s),6.30-6.31(1H,m),6.37-6.39(1H,m),6.72-6.73(1H,m),6.97(1H,d,J=8.3Hz),7.09-7.11(1H,m),7.31-7.38(1H,m),7.33-7.36(3H,m),7.63(1H,s).
5-Isomer(high polarity fraction)
MS (ESI) m/z : 581.(M⁺+H).
¹H-NMR(CDCl₃)6:1.42-1.43 (6H,m),2.62-2.78 (2H,m),3.43-3.43(3H,m),3.64-3.65(3H,m),3.86-3.86(3H,m),4.49-4.52(1H,m),4.55-4.57(2H,m),4.65-4.80(2H,m),5.75(1H,s),6.32-6.33(1H,m),6.36-6.38(1H,m),6.73(1H,s),6.95(1H,d,J=8.1Hz),7.09-7.10(1H,m),7.18(1H,t,J=8.1Hz),7.33-7.38(3H,m),7.57(1H,s).

### Example 123

1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazole-4-carboxylic acid

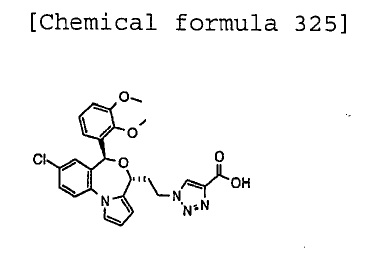

Ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-carboxylate (48.7 mg) was dissolved in methanol-water-tetrahydrofuran (1:0.5:1, 2.5mL). Potassium carbonate (38.6 mg) was added and the resulting mixture was stirred at room temperature for 2 days. The reaction mixture was neutralized with an ion exchange resin (Amberlite IR-120B). The resin was filtered and the filtrate was concentrated. The residue thus obtained was purified by preparative TLC (lower layer of methanol:chloroform:water=3:7:1) to give the title compound (41.5 mg).
MS(ESI)m/z:495(M⁺+H).
¹H-NMR(CDCl₃)δ:2.30-2.42(2H,m),3.32(3H,s),3.77(3H,s),4.16-4.40(3H,m),5.61(1H,s),6.18-6.20(2H,m),6.64-6.66(1H,m),6.82-6.84(1H,m),6.92-6.95(1H,m),7.05-7.09(1H,m),7.18-7.24(3H,m),7.91-7.94(1H,m).

### Example 124

1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-carboxylic acid

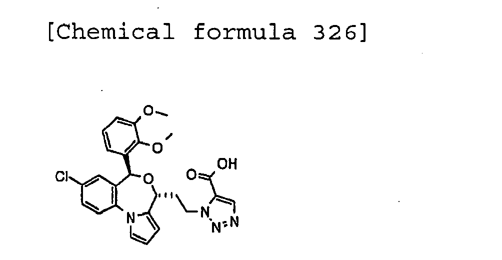

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazole-4-carboxylic acid, ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylate (132 mg) and potassium carbonate (105 mg) were treated, whereby the title compound (118 mg).
MS(ESI)m/z:495(M⁺+H).
¹H-NMR(CDCl₃)δ:2.30-2.42(2H,m),3.29(3H,s),3.68(3H,s),4.31-4.34(1H,m),4.56-4.73(2H,m),5.67-5.69(1H,m),6.11-6.13(2H,m),6.62-6.65(1H,m),6.72-6.75(1H,m),6.90-6.92(1H,m),7.00-7.07(1H,m),7.20-7.31(3H,m),7.72-7.75(1H,m).

### Example 125

2-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)methoxy]acetic acid

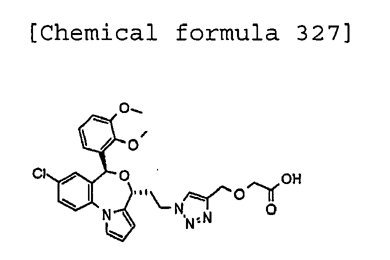

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazole-4-carboxylic acid, ethyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]acetate (69.7 mg) and potassium carbonate (51.0 mg) were treated, whereby the title compound (63.3 mg). MS(ESI)m/z:539(M⁺+H).
¹H-NMR(CDCl₃)δ:2.47-2.60(2H,m),3.38(3H,s),3.81(5H,s),4.35-4.37(1H,m),4.45-4.61(4H,m),5.69(1H,s),6.23-6.26(1H,m),6.27-6.29(1H,m),6.68(1H,s),6.89-6.92(1H,m),7.01-7.02(1H,m),7.12-7.17(1H,m),7.25-7.30(5H,m),7.63(1H,s).

### Example 126

2-[(1-{2-[(4R,65)-8-Chloro-6-(2,3-dimethoxyphenyl)-4H,6H-pyrrolo[12-a][4,1]benzoxazepin-4-yl]ethyl}-1H-1,2,3-triazol-5-yl)methoxy]acetic acid

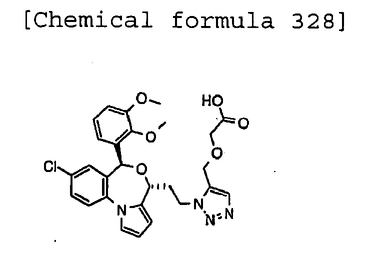

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazole-4-carboxylic acid, ethyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)methoxy]acetate (50.0 mg) and potassium carbonate (36.6 mg) were treated, whereby the title compound (42.9 mg).
MS(ESI)m/z:539(M⁺+H). ¹H-NMR(CDCl₃)δ:2.48-2.62(2H,m),3.35(3H,s),3.77-3.79(5H,m),4.39-4.41(1H,m),4.45-4.58(4H,m),5.68(1H,s),6.25-6.28(2H,m),6.68(1H,s),6.86-6.88(1H,m),7.00-7.02(1H,m),7.10-7.14(1H,m),7.24-7.30(3H,m),7.56(1H,s).

### Example 127

2-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-methylpropanoic acid

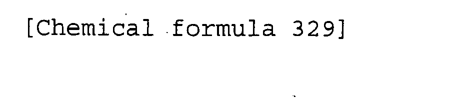

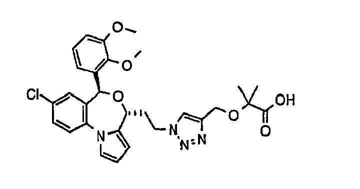

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazole-4-carboxylic acid, methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-methylpropanoate (69.2 mg) was treated while using potassium carbonate (49.4 mg) to give the title compound (50.5 mg).
MS(ESI)m/z:567(M⁺+H) . ¹H-NMR(CDCl₃) δ:1.47 (6H, s), ,2.52-2.67(2H,m),3.43(3H,s),3.86(3H,s),4.38-4.40(1H,m),4.56-4.65(4H,m),5.74(1H,s),6.27-6.29(1H,m),6.35-6.37(1H,m),6.72-6.73(1H,m),6.96(1H,d,J=8.3Hz),7.08-7.10(1H,m),7.20(1H,t,J=7.9Hz),7.26-7.37(4H,m),7.56(1H,s).

### Example 128

2-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)methoxy]-2-methylpropanoic acid

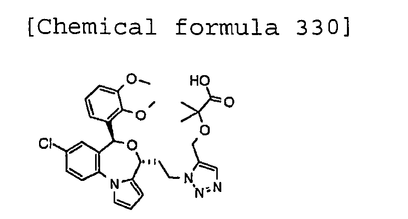

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazole-4-carboxylic acid, methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)methoxy]-2-methylpropanoate (50.1 mg) was treated while using potassium carbonate (35.8 mg) to give the title compound (41.2 mg).
MS(ESI)m/z:567(M⁺+H). ¹H-NMR(CDCl₃)δ:1.41(6H,s),2.63-2.71(2H,m),3.42(3H,s),3.84(3H,s),4.46-4.50(1H,m),4.56-4.74(4H,m),5.74(1H,s),6.29-6.32(1H,m),6.34-6.36(1H,m),6.72-6.73(1H,m),6.94(1H,d,J=8.1Hz),7.07-7.09(1H,m),7.17(1H,t,J=8.1Hz),7.32-7.37(3H,m),7.57(1H,s).

### Referential Example 46

(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)methanol

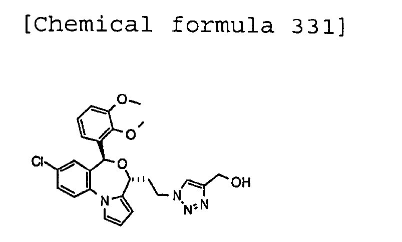

Lithium aluminum hydride (19.9 mg) was suspended in tetrahydrofuran (2 ml). Under ice cooling, a tetrahydrofuran solution (2 mL) of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-carboxylate (91.3 mg) was added dropwise. The reaction mixture was stirred for 30 minutes without changing the condition. Water was added to the reaction mixture. The resulting mixture was extracted with chloroform. The organic layer was dried over anhydrous sodium sulfate to give the title compound (86.8 mg). MS(ESI)m/z:481(M⁺+H).
¹H-NMR(CDCl₃)δ:2.57-2.74(2H,m),3.44(3H,s),3.87(3H,s),4.39-4.41(1H,m),4.62-4.73(2H,m),4.76(2H,d,J=6.1Hz),5.76(1H,s),6.29-6.31(1H,m),6.38(1H,t,J=3.2Hz),6.73(1H,d,J=2.2Hz),6.97(1H,dd ,J=8.1,1.5Hz),7.10-7.11(1H,m),7.21(1H,t,J=8.1Hz),7.29-7.31(1H,m),7.33-7.35(1H,m),7.35-7.39(1H,m),7.55(1H,s).

### Referential Example 47

(1-(2-[(4R,6S)-8-Chloro-6-(2,3-dimethoxyphenyl)-4H,6H-pyrrolo[1,2-a] [4,1]benzoxazepin-4-yl]ethyl)-1H-1,2,3-triazol-5-yl)methanol

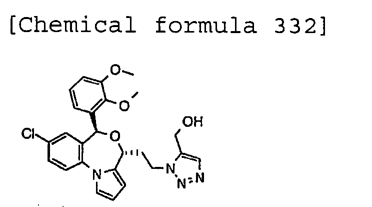

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methanol, lithium aluminum hydride (13.4 mg) and ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylate (61.6 mg) were treated, whereby the title compound (53.3 mg).
MS(ESI)m/z:481(M⁺+H). ¹H-NMR(CDCl₃)δ:2.37(1H,br s),2.67-2.73(2H,m),3.43(3H,s),3.86(3H,s),4.43-4.47(1H,m),4.63-4.76(4H,m),5.75(1H,s),6.31-6.33(1H,m),6.37(1H,t,J=3.2Hz),6.74(1H,d,J=2.0Hz),6.96(1H,dd ,J=7.9,1.3Hz), 7.09-7.10(1H,m), 7.20(1H,t,J=7.9Hz), 7.32-7.38(3H,m), 7.53(1H,s).

### Referential Example 48

(1-{2-[(4R,6S)-8-Chloro-6-(2,3-dimethoxyphenyl)-4H,6H-pyrrolo[1,2-a] [4,1]benzoxazepin-4-yl]ethyl}-1H-1,2,3-triazol-4-yl)methyl methanesulfonate

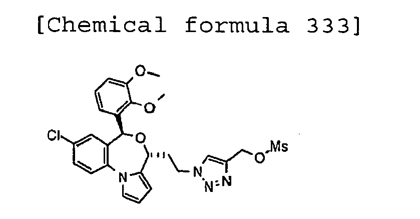

(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)methanol (86.8 mg) was dissolved in dichloromethane (2 mL). Triethylamine (0.038 mL) and methanesulfonyl chloride (0.021 mL) were added at 0°C, followed by stirring for 40 minutes. To the reaction mixture was added a saturated aqueous solution of sodium bicarbonate and the resulting mixture was extracted with chloroform. The organic layer was dried over anhydrous sodium sulfate, whereby the title compound (123 mg).

### Referential Example 49

(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)methyl methanesulfonate

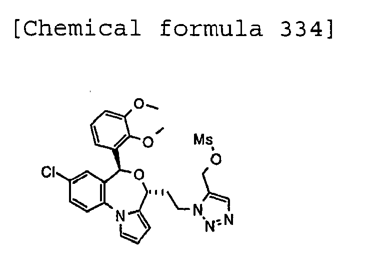

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methyl methanesulfonate, 1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)methanol (53.3 mg), triethylamine (0.023 mL) and methanesulfonyl chloride (0.013 mL) were treated, whereby the title compound (83.0 mg).

### Referential Example 50

2-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)acetonitrile

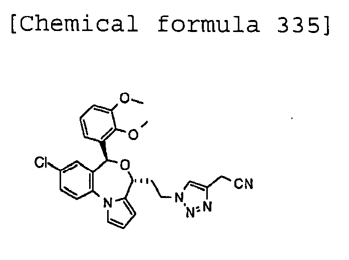

(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)methyl methanesulfonate (101 mg) was dissolved in dimethylsulfoxide (3 mL). Sodium cyanide (21.6 mg, 0.440 mmol) was added and the resulting mixture was stirred overnight at 50°C. The reaction mixture was poured into ice water, followed by extraction with ethyl acetate. The organic layer was dried over anhydrous sodium sulfate and purified by preparative TLC (ethyl acetate:hexane=1:1) to give the title compound (67.0 mg).
MS (ESI)m/z:491 (M⁺+H) . ¹H-NMR(CDCl₃)δ:2.58-2.75(2H,m),3.44(3H,s),3.83(2H,s),3.87(3H,s),4.40(1H,dd,J=9.
3,3.9Hz),4.63-4.76(2H,m),5.76(1H,s),6.30-6.31(1H,m),6.39(1H,t,J=3.3Hz),6.74(1H,d,J=2.2Hz),6.98(1H,dd ,J=7.9,1.5Hz),7.11-7.12(1H,m),7.22(1H,t,J=7.9Hz),7.28(1H,dd,J=7.9,1.5Hz),7.33-7.40(2H,m),7.62(1H,s).

### Referential Example 51

2-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)acetonitrile

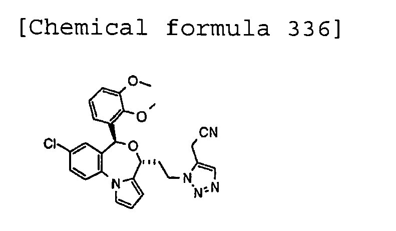

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)acetonitrile, (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)methyl methanesulfonate (62.0 mg) and sodium cyanide (14.6 mg) were treated, whereby the title compound (20.2 mg) .
MS (ESI) m/z : 491 (M⁺+H). ¹H-NMR(CDCl₃)δ:2.66-2.71(2H,m),3.44(3H,s),3.77(2H,s),3.87(3H,s),4.42(1H,t,J=6.6 Hz),4.58-4.72(2H,m),5.77(1H,s),6.30-6.31(1H,m),6.39(1H,t,J=3.3Hz),6.75(1H,d,J=2.2Hz),6.98(1H,dd ,J=7.9,1.6Hz),7.11-7.12(1H,m),7.22(1H,t,J=7.9Hz),7.27(1H,dd,J=7.9,1.3Hz),7.34-7.40(2H,m),7.66(1H,s).

### Example 129

2-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)acetic acid

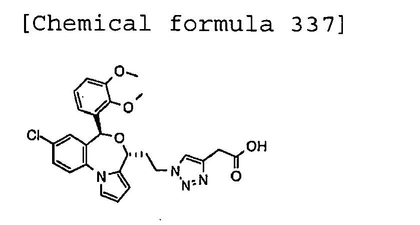

2-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)acetonitrile (67.0 mg) was dissolved in 2-propanol (2 mL). To the resulting solution was added a 5M aqueous sodium hydroxide solution (1 mL). The resulting mixture was heated under reflux overnight. After the reaction mixture was cooled to room temperature and neutralized with 1N hydrochloric acid, the neutralized solution was extracted with chloroform. The organic layer was dried over anhydrous sodium sulfate and purified by preparative TLC (lower layer of methanol:chloroform:water=3:7:1) to give the title compound (61.1 mg). MS(ESI)m/z:509(M⁺+H).
¹H-NMR(CDCl₃)δ:2.45-2.63(2H,m),3.40(3H,s),3.56-3.59(2H,m),3.83(3H,s),4.35-4.38(1H,m),4.45-4.61(2H,m),5.71(1H,s),6.24-6.26(1H,m),6.31(1H,t,J=3.2Hz),6.70(1H,d,J=2.0Hz),6.91-6.93(1H,m),7.03-7.04(1H,m),7.16(1H,t,J=8.1Hz),7.26-7.32(3H,m),7.49(1H,s).

### Example 130

2-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)acetic acid

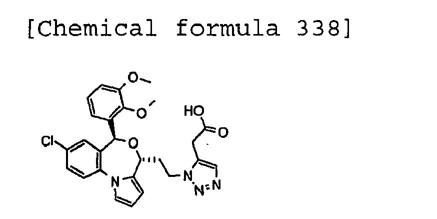

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)acetic acid, 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)acetonitrile (20.2 mg) and a 5M aqueous sodium hydroxide solution (1 mL) were treated, whereby the title compound (15.1 mg). MS(ESI)m/z:509(M⁺+H).
¹H-NMR(CDCl₃)δ:2.41-2.54(2H,m),3.33(3H,s),3.40-3.43(2H,m),3.75(3H,s),4.32-4.45(3H,m),5.66(1H,s),6.22-6.24(2H,m),6.65-6.67(1H,m),6.83-6.84(1H,m),6.95-6.98(1H,m),7.08-7.11(1H,m),7.20-7.23(2H,m),7.27-7.29(1H,m),7.36-7.38(1H,m).

### Example 131

Ethyl 2-(2-{2-[(4R,6S)-8-chloro-6-(2,3-dimethoxyphenyl)-4H, 6H-pyrrolo [1,2-a][4,1]benzoxazepin-4-yl]ethyl}-2H-1,2,3,4-tetrazol-5-yl)acetate

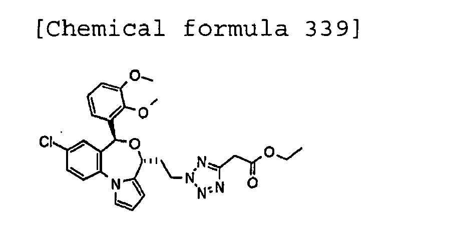

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate

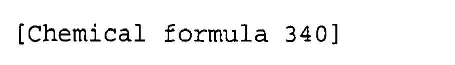

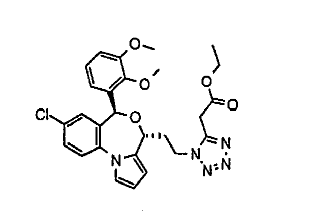

2-[(4R,6S)-8-Chloro-6-(2,3-dimethoxyphenyl)-4H,6H-pyrrolo[1,2-a] [4,1]benzoxazepin-4-yl]ethyl methanesulfonate (126 mg) and ethyl 1H-tetrazole 5-acetate (82.3 mg) were dissolved in N,N-dimethylformamide (2 mL). To the resulting solution were added potassium carbonate (109 mg) and tetrabutylammonium iodide (97.4 mg). The resulting mixture was stirred overnight at 80°C. The reaction mixture was diluted with ethyl acetate and washed with saturated brine. The organic layer was dried over anhydrous sodium sulfate. The filtrate was then concentrated. The residue thus obtained was purified by preparative TLC (methanol:chloroform=1:100) to give the title compounds, that is, 2H-isomer (85.4 mg) and 1H-isomer (65.4 mg).
2H-Isomer (low polarity fraction)
MS(ESI)m/z:538(M⁺+H).
¹H-NMR(CDCl₃)δ:1.24(3H,t,J=7.2Hz),2.75-2.79(2H,m),3.44(3H,s),3.86(3H,s),3.93(2H,s),4.18(2H,q,J=7.2 Hz),4.41-4.45(1H,m),4.91-4.95(2H,m),5.75(1H,s),6.32-6.34(1H,m),6.38-6.39(1H,m),6.72(1H,d,J=2.0Hz),6.96(1H,dd,J=8.1,1.3Hz),7.10-7.11(1H,m),7.21(1H,t,J=8.1Hz),7.32-7.38(3H,m).
1H-Isomer (high polarity fraction)
MS (ESI)m/z:538 (M⁺+H) .
¹H-NMR(CDCl₃)δ:1.24 (3H,t,J=7.2Hz) 2.66-2.76(2H,m),3.43(3H,s),3.86(3H,s),3.99-4.00(2H,m),4.14-4.20(2H,m),4.40-4.43(1H,m),4.60-4.67(2H,m),5.75(1H,s),6.30-6.32(1H,m),6.38-6.39(1H,m),6.74(1H,d,J=2.2Hz),6.97(1H,dd,J=8.1,1.7Hz),7.10-7.12(1H,m),7.19(1H,t,J=8.1Hz),7.33-7.40(3H,m).

### Example 132

Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]acetate

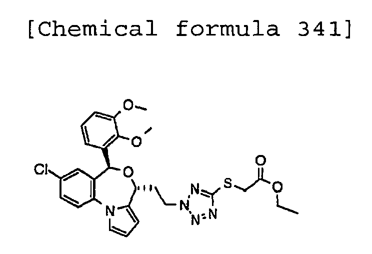

Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]acetate

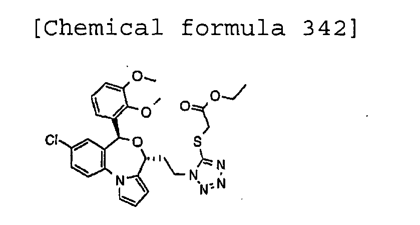

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate and ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate, 2-[(4R,6S)-8-chloro-6-(2,3-dimethoxyphenyl)-4H,6H-pyrrolo[1,2-a][4,1]benzoxazepin-4-yl]ethyl methanesulfonate (134 mg) and ethyl([1H-tetrazol-5-yl]sulfanyl)acetate (105 mg) were treated, whereby the title compounds, that is, 2H-isomer (83.5 mg) and 1H-isomer (28.1 mg) were obtained. 2H-Isomer (low polarity fraction)
MS(ESI)m/z:570 (M⁺+H).
¹H-NMR(CDCl₃)δ:1.24(3H,t,J=7.2Hz),2.66-2.80(2H,m),3.44(3H,s),3.86(3H,s),3.94(2H,s),4.18(2H,q,J=7.2 Hz),4.40-4.43(1H,m),4.87-4.91(2H,m),5.76(1H,s),6.31-6.33(1H,m),6.38(1H,t,J=3.2Hz),6.72(1H,d,J=2.2Hz),6.95-6.98(1H,m),7.10-7.11(1H,m),7.22(1H,t,J=8.1Hz),7.32-7.34(2H,m),7.36-7.38(1H,m).
1H-Isomer (high polarity fraction)
MS(ESI)m/z:570(M⁺+H).
¹H-NMR(CDCl₃)δ:1.26(3H,t,J=7.2Hz),2.57-2.73(2H,m),3.43(3H,s),3.86(3H,s),4.11(2H,s),4.20(2H,q,J=7.2 Hz),4.38-4.42(1H,m),4.58-4.62(2H,m),5.75(1H,s),6.30-6.32(1H,m),6.38(1H,t,J=3.2Hz),6.74(1H,d,J=2.2Hz),6.96-6.98(1H,m),7.10-7.11(1H,m),7.22(1H,t,J=8.1Hz),7.32-7.38(3H,m).

### Example 133

Ethyl 2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-carboxylate

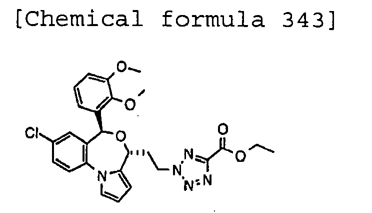

Ethyl 2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-carboxylate

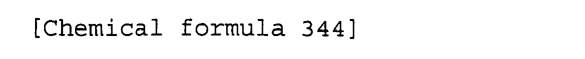

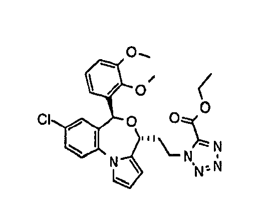

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate and ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate, 2-[(4R,6S)-8-chloro-6-(2,3-dimethoxyphenyl)-4H,6H-pyrrolo[1,2-a][4,1]benzoxazepin-4-yl]ethyl methanesulfonate (134 mg) and ethyl 1H-tetrazole 5-carboxylate (105 mg) were treated, whereby the title compounds, that is, 2H-isomer (63.3 mg) and 1H-isomer (30.3 mg) were obtained. 2H-Isomer (low polarity fraction)
MS(ESI)m/z:525(M⁺+H).
¹H-NMR(CDCl₃)δ:1.45(3H,t,J=7.2Hz),2.79-2.85(2H,m),3.44(3H,s),3.86(3H,s),4.39-4.43(1H,m),4.51(2H,q,J=7.2Hz),5.01-5.06(2H,m),5.75(1H,s),6.32-6.33(1H,m),6.38-6.39(1H,m),6.72(1H,d,J=2.2Hz),6.96-6.98(1H,m),7.09-7.12(1H,m),7.21(1H,t,J=7.9Hz),7.30-7.36(3H,m).
1H-Isomer (high polarity fraction)
MS(ESI)m/z:525(M⁺+H).
¹H-NMR(CDCl₃)δ:1.42-1.47(3H,m),2.60-2.78(2H,m),3.43(3H,s),3.86(3H,s),4.44-4.53(3H,m),5.06-5.09(2H,m),5.74-5.75(1H,m),6.32-6.35(1H,m),6.38-6.39(1H,m),6.72-6.75(1H,m),6.96-6.98(1H,m),7.09-7.12(1H,m),7.21(1H,t,J=8.1Hz),7.33-7.39(3H,m).

### Example 134

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)-2-(methoxyimino)acetate

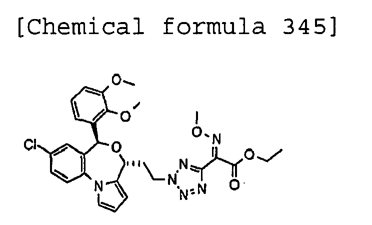

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)-2-(methoxyimino)acetate

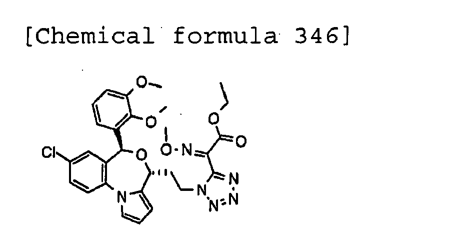

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate and ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate, 2-[(4R,6S)-8-chloro-6-(2,3-dimethoxyphenyl)-4H,6H-pyrrolo[1,2-a][4,1]benzoxazepin-4-yl]ethyl methanesulfonate (155 mg) and ethyl 2-(methoxyimino)-2-(1H-tetrazol-5-yl)acetate (92.2 mg) were treated, whereby the title compounds, that is, 2H-isomer (75.0 mg) and 1H-isomer (43.1 mg) were obtained. 2H-Isomer (low polarity fraction)
MS(ESI)m/z:582(M⁺+H).
¹H-NMR(CDCl₃)δ:1.33(3H,t,J=7.1Hz), 2.73-2.88(2H,m),3.44(3H,s),3.86(3H,s),4.13(3H,s),4.36(2H,q,J=7.1 Hz),4.43-4.47(1H,m),5.00-5.04(2H,m),5.77(1H,s),6.33-6.35(1H,m),6.38-6.40(1H,m),6.72(1H,d,J=2.2Hz),6.97(1H,dd,J=8.1,1.5Hz),7.11-7.12(1H,m),7.21(1H,t,J=8.1Hz),7.33-7.38(3H,m).
1H-Isomer (high polarity fraction)
MS(ESI)m/z:582(M⁺+H).
¹H-NMR(CDCl₃)δ:1.28(3H,t,J=7.2Hz),2.63-2.69(2H,m),3.43(3H,s),3.86(3H,s),4.09(3H,s),4.26-4.34(2H,m),4.37-4.43(2H,m),4.51-4.56(1H,m),5.74(1H,s),6.28-6.29(1H,m),6.37-6.39(1H,m),6.71(1H,d,J=2.4Hz),6.97(1H,dd,J=7.6,2.0Hz),7.10-7.11(1H,m),7.19-7.26(2H,m),7.33-7.39(2H,m).

### Example 135

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)-2-methylpropanoate

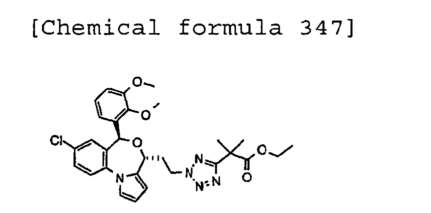

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)-2-methylpropanoate

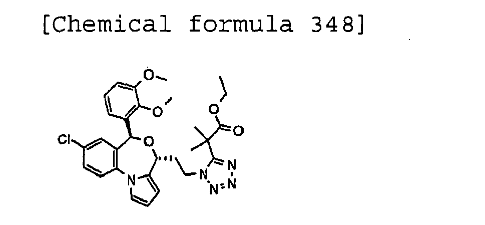

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate and ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate, 2-[(4R,6S)-8-chloro-6-(2,3-dimethoxyphenyl)-4H,6H-pyrrolo[1,2-a][4,1]benzoxazepin-4-yl]ethyl methanesulfonate (179 mg) and ethyl 2,2-dimethyl-2-(1H-tetrazol-5-yl)acetate (138 mg) were treated, whereby the title compounds, that is, 2H-isomer (191 mg) and 1H-isomer (28.7 mg) were obtained. 2H-Isomer (low polarity fraction)
MS (ESI) m/z : 566 (M⁺+H).
¹H-NMR (CDCl₃) δ:1.14 (3H, t, J=7.1Hz) 1.64 (6H, s) 2.68 - 2.80(2H,m),3.44(3H,s),3.86(3H,s),4.10(2H,q,J=7.1Hz),4.36-4.39(1H,m),4.89-4.93(2H,m),5.76(1H,s),6.32-6.33(1H,m),6.39(1H,t,J=3.2Hz),6.71-6.72(1H,m),6.97(1H,d,J=8.1Hz),7.10-7.11(1H,m),7.22(1H,t,J=8.1Hz),7.31-7.41(3H,m).
1H-Isomer (high polarity fraction)
MS(ESI)m/z:566(M⁺+H)
¹H-NMR(CDCl₃)δ:1.15-1.19(3H,m), 1.73(6H,s),2.65-2.80(2H,m),3.43(3H,s),3.87(3H,s),4.12-4.16(2H,m),4.33-4.39(1H,m),4.47-4.52(2H,m),5.74(1H,s),6.32-6.33(1H,m),6.38-6.40(1H,m),6.73-6.74(1H,m),6.97(1H,d,J=8.1Hz),7.11-7.12(1H,m),7.22(1H,t,J=8.1Hz),7.27-7.29(1H,m),7.34-7.41(2H,m).

### Example 136

Ethyl 1-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)cyclopropanecarboxylate

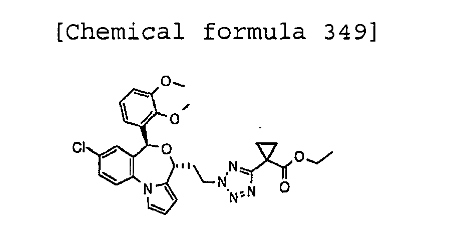

Ethyl 1-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)cyclopropanecarboxylate

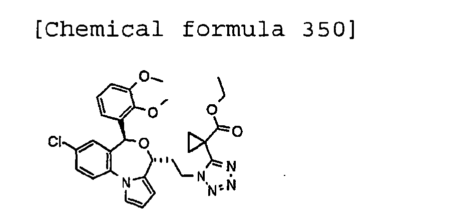

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate and ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate, 2-[(4R,6S)-8-chloro-6-(2,3-dimethoxyphenyl)-4H,6H-pyrrolo[1,2-a][4,1]benzoxazepin-4-yl]ethyl methanesulfonate (179 mg) and ethyl 1-(2H-1,2,3,4-tetrazol-5-yl)cyclopropanecarboxylate (136 mg) were treated, whereby the title compounds, that is, 2H-isomer (80.9 mg) and 1H-isomer (65.1 mg) were obtained. 2H-Isomer (low polarity fraction)
MS (ESI) m/z:564 (M⁺+H).
¹H-NMR(CDCl₃)δ:1.15(3H,t,J=7.1Hz),1.39-1.43 (2H, m), 1.70-1.73(2H,m),2.68-2.83(2H,m),3.44(3H,s),3.86(3H,s),4.12(2H,q,J=7.1Hz),4.37-4.40(1H,m),4.89-4.93(2H,m),5.75(1H,s),6.32-6.33(1H,m),6.37-6.39(1H,m),6.72(1H,d,J=2.2Hz),6.97(1H,dd,J=8.1,1.5Hz),7.10-7.11(1H,m),7.21(1H,t,J=8.1Hz),7.31-7.38(3H,m).
1H-Isomer (high polarity fraction)
MS(ESI)m/z:564 (M⁺+H).
¹H-NMR(CDCl₃)δ:1.15(3H,t,J=7.1Hz), 1.40-1.45(1H,m), 1.51-1.56(1H,m),1.79-1.84(2H,m),2.72-2.79(2H,m),3.43(3H,s),3.86(3H,s),4.11(2H,q,J=7.1Hz),4.46-4.50(1H,m),4.52-4.57(1H,m),4.63-4.71(1H,m),5.75(1H,s),6.31-6.33(1H,m),6.38-6.40(1H,m),6.74-6.74(1H,m),6.96-6.98(1H,m),7.11-7.13(1H,m),7.16-7.23(2H,m),7.34-7.41(2H,m).

### Example 137

2-(2-{2-[(4R,6S)-8-Chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid

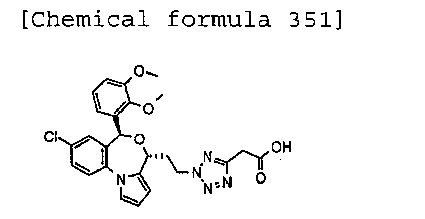

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (85.4 mg) was dissolved in methanol-water-tetrahydrofuran (1:0.5:1, 2.5 mL). Potassium carbonate (65.8 mgl) was added and the resulting mixture was stirred overnight at room temperature. After the reaction mixture was neutralized with an ion exchange resin (Amberlite IR-120B), the resin was filtered out and the filtrate was concentrated. The residue thus obtained was purified by preparative TLC (lower layer of methanol:chloroform:water=3:7:1) to give the title compound (61.2 mg). MS(ESI)m/z:510(M⁺+H).
¹H-NMR(CDCl₃)δ:2.56-2.67(2H,m),3.38(3H,s),3.61-3.66(2H,m),3.81(3H,s),4.35-4.40(1H,m),4.66-4.83(2H,m),5.68(1H,s),6.23-6.28(2H,m),6.66-6.68(1H,m),6.87-6.89(1H,m),7.10-7.15(1H,m),7.11-7.13(1H,m),7.23-7.30(3H,m).

### Example 138

2-(2-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)acetic acid

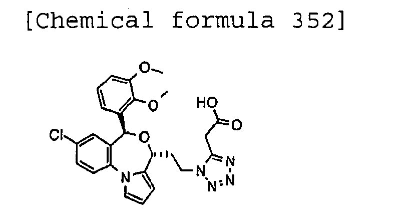

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate (65.4 mg) and potassium carbonate (50.4 mg) were treated, whereby the title compound (47.3 mg).
MS(ESI)m/z:510(M⁺+H).
¹H-NMR(CDCl₃)δ:2.45-2.55(2H,m),3.34(3H,s),3.53-3.55(2H,m),3.76(3H,s),4.32-4.45(3H,m),5.66(1H,s),6.19-6.25(2H,m),6.64-6.66(1H,m),6.81-6.83(1H,m),6.92-6.94(1H,m),7.08-7.13(1H,m),7.16-7.22(2H,m),7.30-7.32(1H,m).

### Example 139

2-[(2-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]acetic acid

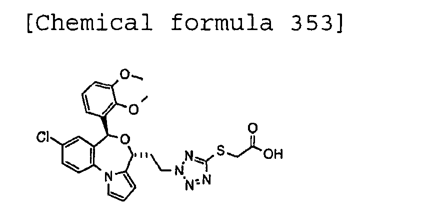

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]acetate (83.5 mg) and potassium carbonate (60.7 mg) were treated, whereby the title compound (78.1 mg).
MS(ESI)m/z:542(M⁺+H).
¹H-NMR(CDCl₃)δ:2.56-2.71(2H,m),3.31-3.37(1H,m),3.38(3H,s),3.67-3.71(1H,m),3.80(3H,s),4.37-4.41(1H,m),4.70-4.83(2H,m),5.69(1H,s),6.24-6.29(2H,m),6.66-6.69(1H,m),6.88-6.90(1H,m),6.99-7.01(1H,m),7.13-7.15(1H,m),7.21-7.29(3H,m).

### Example 140

2-[(2-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]acetic acid

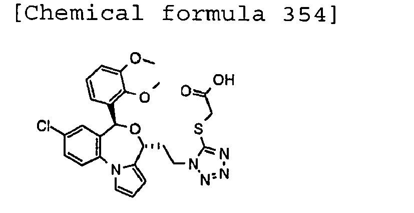

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]acetate (28.1 mg) and potassium carbonate (20.4 mg) were treated, whereby the title compound (22.6 mg).
MS(ESI)m/z:542(M⁺+H).
¹H-NMR(CDCl₃)δ:2.45-2.52(2H,m),3.36(3H,s),3.68-3.70(2H,m),3.79(3H,s),4.33-4.42(3H,m),5.67(1H,s),6.22-6.27(2H,m),6.67(1H,s),6.85-6.88(1H,m),6.97-7.00(1H,m),7.12-7.14(1H,m),7.24-7.33(3H,m).

### Example 141

2-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-5-carboxylic acid

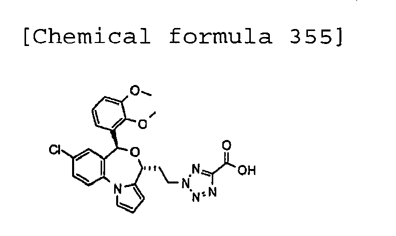

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-carboxylate (60.3 mg) and potassium carbonate (47.7 mg) were treated, whereby the title compound (42.6 mg) .
MS (ESI) m/z : 496 (M⁺+H) .
¹H-NMR(CDCl₃)δ:2.41-2.55(2H,m), 3.35(3H,s),3.79(3H,s),4.32-4.35(1H,m),4.49-4.51(1H,m),4.66-4.68(1H,m),5.63-5.66(1H,m),6.15-6.22(2H,m),6.64-6.67(1H,m),6.83-6.94(2H,m),7.07-7.10(1H,m),7.21-7.25(3H,m).

### Example 142

2-(2-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-5-yl)-2-(methoxyimino)acetic acid

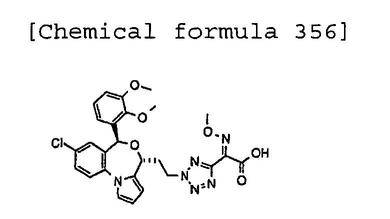

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)-2-(methoxyimino)acetate (75.0 mg) and potassium carbonate (53.5 mg) were treated, whereby the title compound (56.4 mg).
MS (ESI) m/z : 553 (M⁺+H).
¹H-NMR(CDCl₃)δ:2.57-2.66(2H,m),3.38(3H,s),3.74(3H,s),3.81(3H,s),4.37-4.41(1H,m),4.69-4.77(1H,m),4.81-4.88(1H,m),5.68(1H,s),6.22-6.24(1H,m),6.25-6.27(1H,m),6.66(1H,d,J=2.0Hz),6.87-6.90(1H,m),6.99-7.00(1H,m),7.11(1H,t,J=7.9Hz),7.21-7.29(3H,m).

### Example 143

2-(2-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)-2-(methoxyimino)acetic acid

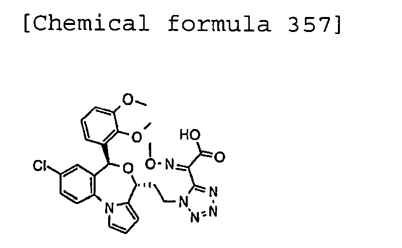

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)-2-(methoxyimino)acetate (43.1 mg) and potassium carbonate (30.7 mL) were treated, whereby the title compound (31.7 mg).
MS(ESI)m/z:553(M⁺+H).
¹H-NMR(CDCl₃)δ:2.34-2.41(1H,m),2.48-2.57(1H,m),3.37(3H,s),3.69(3H,s),3.79(3H,s),4.26-4.37(2H,m),4.41-4.44(1H,m),5.69(1H,s),6.17-6.20(1H,m),6.23-6.26(1H,m),6.65-6.68(1H,m),6.84-6.86(1H,m),6.97-6.99(1H,m),7.10(1H,t,J=8.1Hz),7.21-7.29(3H,m).

### Example 144

2-(2-{2-[(4R,6S)-8-Chloro-6-(2,3-dimethoxyphenyl-4H,6H-pyrrolo[1,2-a][4,1]benzoxazepin-4-yl)ethyl]-2H-1,2,3,4-tetrazol-5-yl}-2-methylpropanoic acid

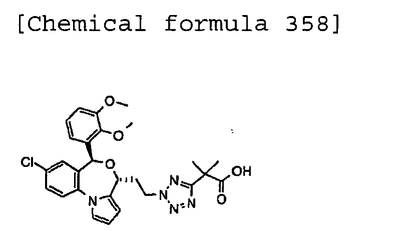

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)-2-methylpropanoate (191 mg) and potassium carbonate (140 mg) were treated, whereby the title compound (154 mg) .
MS(ESI)m/z:538(M⁺+H)
¹H-NMR(CDCl₃)δ:1.57-1.58(6H,m), 2.61-2.78(2H,m),3.43(3H,s),3.85(3H,s),4.36-4.38(1H,m),4.81-4.93(2H,m),5.74(1H,s),6.29-6.32(1H,m),6.34-6.36(1H,m),6.71(1H,d,J=2.2Hz),6.94-6.96(1H,m),7.07-7.09(1H,m),7.19(1H,t,J=7.9Hz),7.29-7.39(3H,m).

### Example 145

2-(2-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)-2-methylpropanoic acid

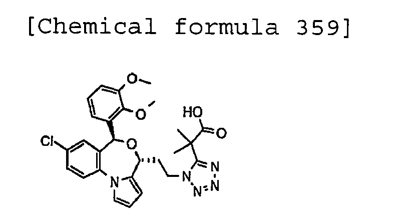

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)-2-methylpropanoate (28.7 mg) and potassium carbonate (21.0 mg) were treated, whereby the title compound (20.3 mg).
MS(ESI)m/z:538(M⁺+H).
¹H-NMR(CDCl₃)δ:1.65-1.66(6H,m),2.68-2.79(2H,m),3.41(3H,s),3.85(3H,s),4.37-4.45(1H,m),4.49-4.52(1H,m),4.58-4.66(1H,m),5.71(1H,s),6.31-6.34(1H,m),6.35-6.37(1H,m),6.72(1H,d,J=2.2Hz),6.93-6.95(1H,m),7.09-7.10(1H,m),7.18(1H,t,J=7.9Hz),7.30-7.39(3H,m).

### Example 146

1-(2-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-5-yl)cyclopropanecarboxylic acid

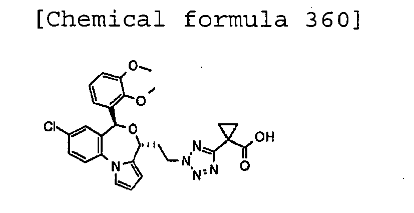

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 1-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)cyclopropanecarboxylate (80.9 mg) and potassium carbonate (59.5 mg) were treated, whereby the title compound (70.8 mg).
MS (ESI)m/z:536 (M⁺+H) .
¹H-NMR(CDCl₃)0:1.26-1.32(1H,m),1.36-1.41 (1H,m),1.67-1.72(2H,m),2.57-2.74(2H,m),3.42(3H,s),3.85(3H,s),4.33-4.37(1H,m),4.80-4.86(2H,m),5.72(1H,s),6.26-6.29(1H,m),6.33-6.35(1H,m),6.69-6.71(1H,m),6.94(1H,d,J=8.1Hz),7.06-7.08(1H,m),7.18(1H,t,J=8.1Hz),7.28-7.35(3H,m).

### Example 147

1-(2-{2-[(4R,6S)-8-Chloro-6-(2,3-dimethoxyphenyl)-4H,6H-pyrrolo[1,2-a][4,1]benzoxazepin-4-yl]ethyl)-1H-1,2,3,4-tetrazol-5-yl)cyclopropanecarboxylic acid

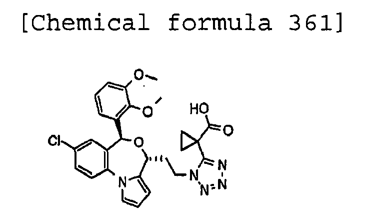

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, ethyl 1-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)cyclopropanecarboxylate (65.1 mg) and potassium carbonate (47.9 mg) were treated, whereby the title compound (41.6 mg).
MS(ESI)m/z:536(M⁺+H).
¹H-NMR(CDCl₃)δ:1.03-1.07(2H,m),1.50-1.56(2H,m),3.36(3H,s),3.79(3H,s),4.44-4.47(1H,m),4.51-4.65(2H,m),5.68(1H,s),6.27-6.30(2H,m),6.67(1H,s),6.88(1H,d,J=8.1Hz),7.01-7.03(1H,m),7.12(1H,t,J=8.1Hz),7.23-7.25(3H,m).

### Example 148

Ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-5-carboxylate

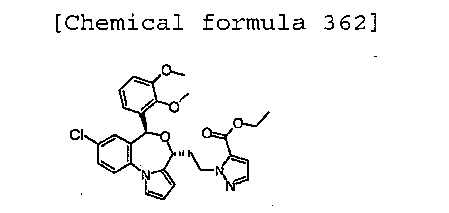

Ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-3-carboxylate

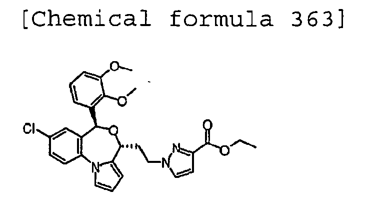

In a similar manner to that employed for the synthesis of ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-5-yl)acetate and ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-yl)acetate, 2-[(4R,6S)-8-chloro-6-(2,3-dimethoxyphenyl)-4H,6H-pyrrolo[1,2-a][4,1]benzoxazepin-4-yl]ethyl methanesulfonate (815 mg) was treated using ethyl 1H-pyrazole-3-carboxylate (111 mg), sodium hydride (42 mg), and tetrabutylammonium iodide (209 mg) to give the title compounds, that is, 5-isomer (78 mg) and 3-isomer (92 mg), respectively.

### Referential Example 52

(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-5-yl)methanol

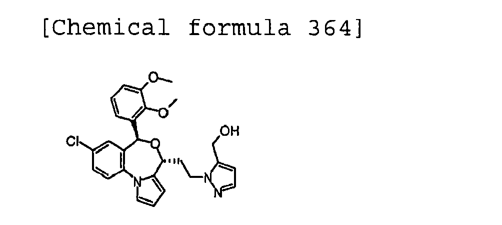

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)methanol, ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-5-carboxylate (78 mg) was treated using lithium aluminum hydride (9.3 mg) to give the title compound (77 mg).
MS(ESI)m/z:480(M⁺+H).
¹H-NMR(CDCl₃)δ:2.56-2.71(2H,m),3.43(3H, s),3.67-3.72(1H,m),3.86(3H,s),4.37-4.55(3H,m),4.60-4.70(2H,m),5.74(1H,s),6.16(1H,d,J=1.7Hz),6.31-6.40(2H,m),6.74(1H,d,J=2.2Hz),6.96(1H,dd,J=8.3,1.5Hz),7.07-7.10(1H,m),7.18-7.23(1H,m),7.30-7.44(3H,m).
IR(ATR)cm⁻¹:2935,1587,1481,1277,1099,1055,1005,748.

### Referential Example 53

(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-5-yl)methyl methanesulfonate

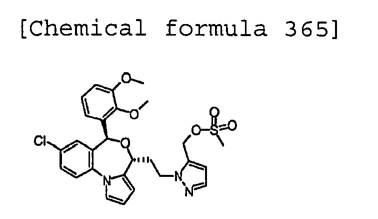

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)methyl methanesulfonate, (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-5-yl)methanol (76 mg) was treated using methanesulfonyl chloride (16 µl) and triethylamine (24 µl) to give the title compound (97 mg).
¹H-NMR(CDCl₃)δ:1.99-2.26(2H,m),2.44-2.71(20H,m),3.03(3H,s),3.13(30H,s),3.38(3H,s),3.43(30H,s),3 .86(3H,s),3.87(30H,s),4.25-4.69(5H,m),5.72(1H,s),5.74(10H,s),6.11-6.24(1H,m),6.31-6.40(2H,m),6.45-6.47(0H,m),6.54-6.61(1H,m),6.74(1H,d,J=2.2Hz),6.78(1H,d,J=2.2Hz),6.93-7.05(1H,m),7.06-7.24(2H,m),7.29-7.44(4H,m),8.23-8.28(10H,m).

### Referential Example 54

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-5-yl)acetonitrile

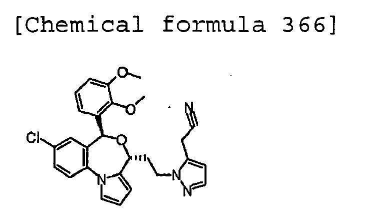

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)acetonitrile, (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-5-yl)methyl methanesulfonate (88 mg) was treated using sodium cyanide (16 mg) to give the title compound (34 mg).
MS (ESI) m/z:489(M⁺+H)⁻.
¹H-NMR(CDCl₃)δ:2.51-2.67(2H,m),3.43(3H,s),3.72(2H,s),3.87(3H,s),4.27-4.49(3H,m),5.74(1H,s),6.21-6.26(1H,m),6.29-6.32(1H,m),6.36-6.39(1H,m),6.75(1H,d,J=2.2Hz),6.94-7.00(1H,m),7.09-7.15(1H,m),7.22(1H,t,J=8.0Hz),7.29-7.41(3H,m),7.43-7.46(1H,m).

### Example 149

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-5-yl)acetic acid

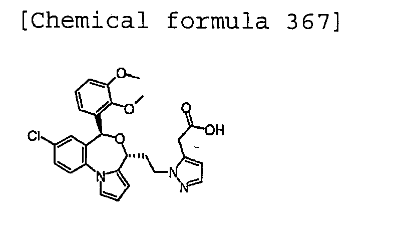

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)acetic acid, 2-(1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-5-yl)acetonitrile (88 mg) was treated, whereby the title compound (25 mg).
¹H-NMR(CDCl₃)δ:2.47-2.78(2H,m),3.40(3H,d,J=1.2Hz), 3.47-3.73(2H,m),3.83(3H,d,J=1.7Hz),4.14-4.47(3H,m),5.72(1H,s),6.10(1H,s),6.27-6.37(2H,m),6.69-6.76(1H,m),6.87-6.97(1H,m),7.04-7.09(1H,m),7.10-7.19(1H,m),7.29-7.43(4H,m).
IR(ATR)cm⁻
¹:2935,1718,1587,1481,1277,1173,1099,1039,1003,760,714.
MS(ESI)m/z:508(M⁺+H).

### Referential Example 55

(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-3-yl)methanol

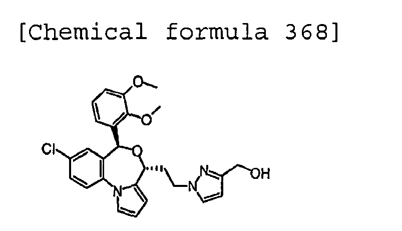

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methanol, ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-3-carboxylate (91 mg) was treated using lithium aluminum hydride (11 mg) to give the title compound (93 mg).
MS (ESI) m/z :480 (M⁺+H).
¹H-NMR (CDCl₃) δ :2.53-2.64 (2H,m), 3.44 (3H, s), 3.86 (3H, s), 4.27-4.53(3H,m),4.64(2H,s),5.74(1H,s),6.17(1H,d,J=2.2Hz),6.28-6.32(1H,m),6.35-6.39(1H,m),6.70-6.77(1H,m),6.97(1H,dd,J=8.3,1.5Hz),7.08-7.11(1H,m),7.21(1H,t,J=8.1Hz),7.29-7.40(4H,m).
IR(ATR)cm⁻¹:2937,1720,1587,141,1277,1223,1101,1032,750,715.

### Referential Example 56

(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-3-yl)methyl methanesulfonate

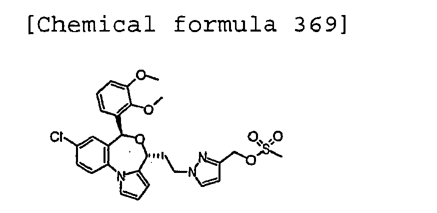

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methyl methanesulfonate, (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)methanol (85 mg) was treated using methanesulfonyl chloride (18 µl) and triethylamine (37 µl) to give the title compound (108 mg).

### Referential Example 57

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-3-yl)acetonitrile

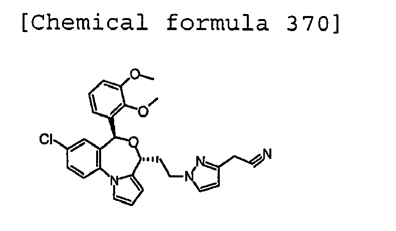

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)acetonitrile, (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)methyl methanesulfonate (99 mg) was treated using sodium cyanide (17 mg) to give the title compound (40 mg).
MS(ESI)m/z:480(M⁺+H).
¹H-NMR(CDCl₃)δ:2.51-2.65(2H,m),3.44(3H,s),3.68-3.72(2H,m),3.86(3H,s),4.26-4.50(3H,m),5.74(1H,s),6.18-6.23(1H,m),6.28-6.33(1H,m),6.37(1H,t,J=3.2Hz),6.71-6.76(1H,m),6.93-7.05(1H,m),7.07-7.11(1H,m),7.21(1H,t,J=8.1Hz),7.29-7.40(4H,m).
MS(ESI)m/z:489(M⁺+H).

### Example 150

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-3-yl)acetic acid

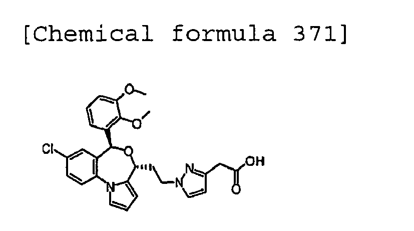

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)acetic acid, 2-(1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)acetonitrile (40 mg) was treated using a 5N aqueous sodium hydroxide solution (0.82 mL) to give the title compound (21 mg).
¹H-NMR(CDCl₃)δ:2.50-2.60(2H,m),3.43(3H,s),3.59-3.70(2H,m),3.86(3H,s),4.24-4.51(3H,m),5.73(1H,s),6.07(1H,s),6.26-6.31(1H,m),6.34-6.38(1H,m),6.72(1H,d,J=2.0Hz),6.94-7.00(1H,m),7.06-7.10(1H,m),7.20(1H,t,J=8.1Hz),7.29-7.37(4H,m).
IR (ATR) cm⁻
¹:2933,1716,1481,1277,1173,1099,1051,1003,758,714.

### Referential Example 58

2-[2-(4-Methoxybenzyl)-2H-1,2,3,4-tetrazol-5-yl]-1-ethanol

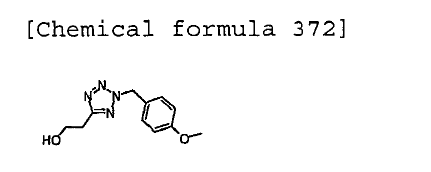

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)methanol, ethyl 2-[2-(4-methoxybenzyl)-2H-1,2,3,4-tetrazol-5-yl]acetate (5.24 g) was treated using lithium aluminum hydride (1.17 g) to give the title compound (4.61 g).
MS(ESI)m/z:235(M⁺+H).
¹H-NMR(CDCl₃)δ:2.34-2.41(1H,m),3.12(2H,t,J=5.9Hz),3.80(3H,s),3.99-4.05(2H,m),5.63-5.69(2H,m),6.87-6.92(2H,m),7.27-7.36(2H,m).
IR(ATR)cm⁻¹:2937,1612,1514,1248,1176,1026,779.

### Referential Example 59

2-[2-(4-Methoxybenzyl)-2H-1,2,3,4-tetrazol-5-yl]ethyl methanesulfonate

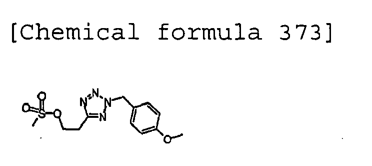

In a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)methyl methanesulfonate; 2-[2-(4-methoxybenzyl)-2H-1,2,3,4-tetrazol-5-yl]-1-ethanol (5.23 g) was treated using methanesulfonyl chloride (2.07 mL) and triethylamine (4.67 mL) to give the title compound (6.20 g) .
MS (ESI)m/z:313 (M⁺+H) .
¹H-NMR(CDCl₃)δ:2.91(3H,s),3.30-3.36(2H,m),3.80(3H,s),4.59-4.64(2H,m),5.66(2H,s),6.86-6.92(2H,m),7.28-7.36(2H,m).
IR(ATR)cm⁻¹:1612,1514,1350,1248,1169,1028,960,906,779,526.

### Referential Example 60

3-[2-(4-Methoxybenzyl)-2H-1,2,3,4-tetrazol-5-yl]propionitrile

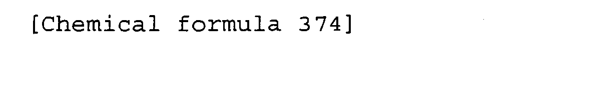

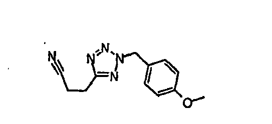

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)acetonitrile, 2-[2-(4-methoxybenzyl)-2H-1,2,3,4-tetrazol-5-yl]ethyl methanesulfonate (6.19 g) was treated using sodium cyanide (1.89 g) to give the title compound (2.77 g).
¹H-NMR(CDCl₃)δ:2.82-2.89(2H,m),3.22-3.28(2H,m),3.80(3H,s),5.66(2H,s),6.87-6.93(2H,m),7.31-7.37(2H,m).
MS (ESI) m/z:266 (M⁺+Na).

### Referential Example 61

3-[2-(4-Methoxybenzyl)-2H-1,2,3,4-tetrazol-5-yl]propionic acid

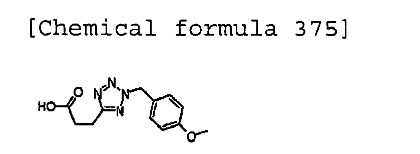

In a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)acetic acid, 3-[2-(4-methoxybenzyl)-2H-1,2,3,4-tetrazol-5-yl]propionitrile (2.10 g) was treated, whereby the title compound (2.26 g).
¹H-NMR(CDCl₃)δ:2.86-2.92(2H,m),3.16-3.24(2H,m),3.79(3H,s),5.64(2H,s),6.86-6.91(2H,m),7.29-7.35(2H,m).
IR(ATR)cm⁻
¹:2931,1712,1610,1512,1440,1294,1244,1213,1030,931,796.
MS (ESI) m/z : 263 (M⁺+H).

### Referential Example 62

Methyl 3-[2-(4-methoxybenzyl)-2H-1,2,3,4-tetrazol-5-yl]propanoate

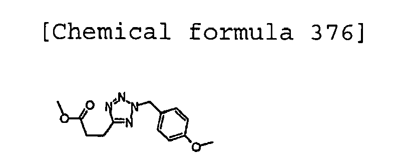

To a methanol solution (17 mL) of 3-[2-(4-methoxybenzyl)-2H-1,2,3,4-tetrazol-5-yl]propionic acid (447 mg) was added tetramethylsilyldiazomethane (2M hexane solution, 1.28mL) under ice cooling, followed by stirring for 3 hours. The solvent was distilled off under reduced pressure to give the title compound (372 mg).
MS(ESI)m/z:277(M⁺+H).
¹H-NMR(CDCl₃)δ:2.81-2.86(2H,m),3.17-3.22(2H,m),3.68(3H,s),3.80(3H,s),5.63(2H,s),6.82-6.95(2H,m),7.29-7.38(2H,m).
IR(ATR)cm⁻¹:2952,1736,1612,1514,1248,1176,1028,841,779.

### Referential Example 63

Methyl 3-[2H-1,2,3,4-tetrazol-5-yl]propanoate

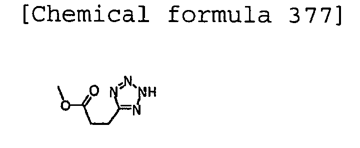

To a methanol solution (10 mL) of methyl 3-[2-(4-methoxybenzyl)-2H-1,2,3,4-tetrazol-5-yl]propanoate (379 mg) were added 20% palladium hydroxide-carbon (150 mg) and a 1,4-dioxane solution (5 mL) of 4N hydrochloric acid. The resulting mixture was stirred in a hydrogen atmosphere for 6 hours. The catalyst was removed by filtration through celite. The organic solvent was distilled off under reduced pressure. The residue thus obtained was subjected to LH-20 column chromatography (methanol) to give the title compound (178 mg).
1H-NMR(CDCl₃)δ:2.82-2.91(2H,m),3.27-3.36(2H,m),3.75(3H,s).
MS(ESI)m/z:15.7(M⁺+H).

### Example 151

Methyl 3-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)propanoate

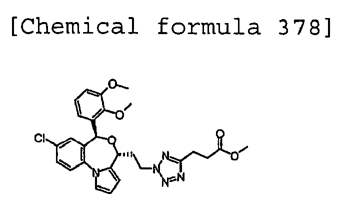

Methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-. 1,2,3,4-tetrazol-5-yl)propanoate

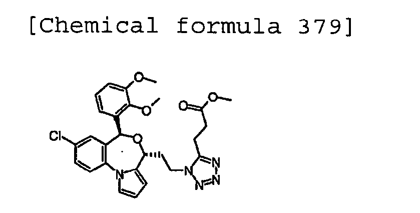

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate and ethyl 2-(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)acetate, 2-[(4R,6S)-8-chloro-6-(2,3-dimethoxyphenyl)-4H,6H-pyrrolo[1,2-a][4,1]benzoxazepin-4-yl]ethyl}methanesulfonate (224 mg) was treated using methyl 3-[2H-1,2,3,4-tetrazol-5-yl]propanoate (88 mg), potassium carbonate (130 mg) and tetrabutylammonium iodide (173 mg) to give the title compounds, 2H-isomer (143 mg) and 1H-isomer (57 mg), respectively. 2H-Isomer (low polarity fraction)
MS(ESI)m/z:560(M⁺+Na).
¹H-NMR(CDCl₃)δ:2.65-2.86(2H,m),3.14-3.22(2H,m),3.44(3H,s),3.48-3.51(2H,m),3.68(3H,s),3.86(3H,s),4.35-4.43(1H,m),4.81-4.94(2H,m),5.75(1H,s),6.28-6.34(1H,m),6.35-6.40(1H,m),6.70-6.73(1H,m),6.84-7.01(1H,m),7.07-7.12(1H,m),7.17-7.24(1H,m),7.28-7.40(3H,m).
IR(ATR)cm⁻
¹:2937,1736,1489,1431,1277,1171,1059,1028,760,715.
1H-Isomer (high polarity fraction)
MS(ESI)m/z:538(M⁺+H).
¹H-NMR(CDCl₃)δ:2.55-2.77(2H,m),2.83-2.98(2H,m),2.99-3.14(2H,m),3.43(3H,s),3.66(3H,s),3.86(3H,s),4.36-4.42(1H,m),4.58-4.72(2H,m),5.75(1H,s),6.27-6.33(1H,m),6.37-6.41(1H,m),6.73(1H,d,J=2.2Hz),6.95-7.01(1H,m),7.10-7.13(1H,m),7.21(1H,t,J=8.1Hz),7.28-7.40(3H,m).
IR(ATR)cm⁻
¹:2951,1736,1481,1429,1275,1171,1028,1003,760,715.

### Example 152

3-(2-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-5-yl)propanoic acid

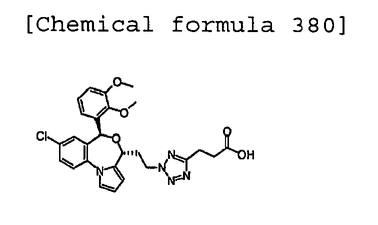

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl)-2H-1,2,3,4-tetrazol-5-yl)acetic acid, methyl 3-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)propanoate (123 mg) was treated using potassium carbonate (95 mg) to give the title compound (75 mg).
¹H-NMR(CDCl₃)δ:2.64-2.90(4H,m),3.12-3.21(2H,m),3.44(3H,s),3.86(3H,s),4.36-4.46(1H,m),4.83-4.95(2H,m),5.75(1H,s),6.26-6.35(1H,m),6.36-6.40(1H,m),6.71-6.78(1H,m),6.91-7.04(1H,m),7.08-7.15(1H,m),7.18-7.24(1H,m),7.29-7.40(3H,m).
IR(ATR)cm⁻
¹:3417,2935,1712,1491,1279,1173,1101,1063,823,762,717.
Elemental analysis for: C₂₆H₂₆ClN₅O₅·0.75H₂O
Calculated: C,58.10;H,5.16;N,13.03.
Found: C,58.30;H,4.95;N,12.82.

### Example 153

3-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)propanoic acid

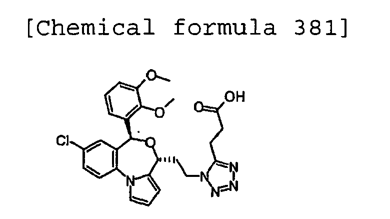

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl]acetic acid, methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)propanoate (60 mg) was treated using potassium carbonate (92 mg) to give the title compound (38 mg).
¹H-NMR(CDCl₃)b:2.54-2.77(2H,m),2 .82-3.13(4H,m),3.42(3H,s),3.85(3H,s),4.35-4.47(1H,m),4.55-4.70(2H,m),5.75(1H,s),6.26-6.31(1H,m),6.37(1H,t,J=3.2Hz),6.72-6.78(1H,m),6.91-7.02(1H,m),7.06-7.15(1H,m),7.16-7.22(1H,m),7.28-7.40(3H,m).
IR(ATR)cm⁻
¹:2935,1728,1491,1429,1325,1279,1174,1101,825,762.

### Referential Example 64

Methyl 4-pentynoate

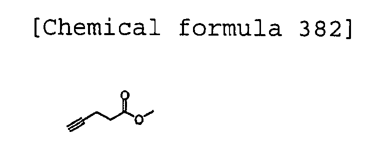

To a dichloromethane solution (50 mL) of 4-pentynoic acid (943 mg) were added methanol (481 mL), 1-ethyl-3-(3-dimethylaminopropyl)carbodiimide hydrochloride (2.63 g), and 1-hydroxybenzotriazole (370 mg). The resulting mixture was stirred for 30 minutes. Distilled water was added to the reaction mixture and the layers separated. The organic layer thus obtained was dried over anhydrous sodium sulfate. The solvent was distilled off under reduced pressure to give the title compound (1.02 g).
¹H-NMR(CDCl₃)δ:1.97-1.99(1H,m),2.47-2.60(4H,m),3.71(3H,s).

### Example 154

Mixture of methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)propanoate and methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)propanoate

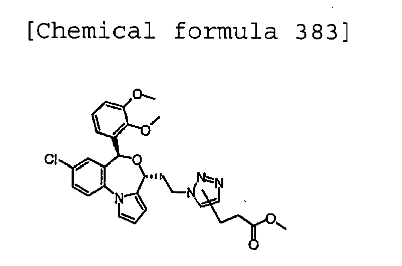

In a similar manner to that employed for the synthesis of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylate and ethyl 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-carboxylate, (4R,6S)-4-(2-azidoethyl)-8-chloro-6-(2,3-dimethoxyphenyl)-4H,6H-pyrrolo[1,2-a][4,1]benzoxazepine (150 mg) was treated using methyl 4-pentynoate (47.5 mg) to give the title compound (115 mg).
¹H-NMR(CDCl₃)δ:2.54-2.76(4H,m),2.93-3.04(2H,m),3.43(3H,s),3.44(3H,s),3.65(3H,s),3.66(3H,s),3.86 (3H,s),3.86(3H,s),4.34-4.49(1H,m),4.51-4.73(2H,m),5.75(1H,s),6.25-6.33(1H,m),6.36-6.40(1H,m),6.71-6.79(1H,m),6.94-7.05(1H,m),7.08-7.13(1H,m),7.18-7.25(1H,m),7.28-7.47(4H,m).
MS(ESI)m/z:537(M⁺+H).

### Example 155

Methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)propanoate

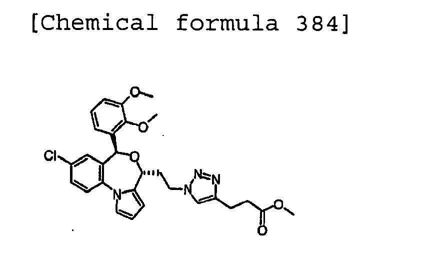

Methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)propanoate

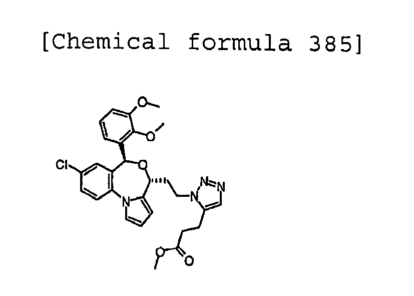

A mixture (115 mg) of methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)propanoate and methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-yl)propanoate was separated by HPLC under the conditions of CHIRALCEL OD (ϕ20 × 250 mm), mobile phase of 50% 2-propanol-hexane, flow rate at 10 mL/min, whereby the title compounds, 4-isomer (57 mg) with a shorter retention time and 5-isomer (38 mg) with a longer retention time were obtained, respectively.
4-Isomer
¹H-NMR(CDCl₃)δ:2.54-2.73 (4H,m),2.95-3.03(2H,m),3.44(3H,s),3.65(3H,s),3.87(3H,s),4.34-4.42(1H,m),4.56-4.71(2H,m),5.75(1H,s),6.28-6.32(1H,m),6.36-6.39(1H,m),6.74(1H,d,J=2.2Hz),6.95-7.00(1H,m),7.09-7.12(1H,m),7.18-7.21(4H,m),7.28-7.41(1H,m). 5-Isomer
¹H-NMR(CDCl₃)δ:2.60-2.72(4H,m),2.91-3.01(2H,m),3.43(3H,s),3.67(3H,s),3.86(3H,s),4.36-4.44(1H,m),4.52-4.67(2H,m),5.75(1H,s),6.30-6.33(1H,m),6.36-6.39(1H,m),6.73(1H,d,J=2.0Hz),6.95-7.00(1H,m),7.09-7.11(1H,m),7.18-7.23(1H,m),7.31-7.43(4H,m).

### Example 156

3-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-4-yl)propanoic acid

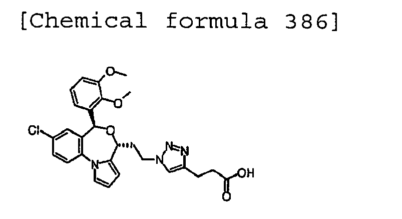

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazole-4-carboxylic acid, methyl 3-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)propanoate (57 mg) was treated using potassium carbonate (73 mg) to give the title compound (26 mg).
1H-NMR(CDCl₃)δ:2.54-2.78(4H,m),2.96-3.03(2H,m),3.44(3H,s),3.86(3H,s),4.34-4.40(1H,m),4.57-4.72(2H,m),5.75(1H,s),6.27-6.31(1H,m),6.36-6.39(1H,m),6.74(1H,d,J=2.2Hz),6.95-7.00(1H,m),7.08-7.12(1H,m),7.19-7.24(1H,m),7.28-7.40(4H,m).
IR(ATR)cm⁻
¹:2933,1716,1481,1323,1277,1221,1099,1061,1003,822,760,714.
MS (ESI) m/z : 523 (M⁺+H).
Elemental analysis for: C₂₇H₂₇ClN₄O₅·0.5H₂O
Calculated: C,60.96;H,5.30;N,10.53.
Found: C,61.34;H,5.39;N,10.21.

### Example 157

3-(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)propanoic acid

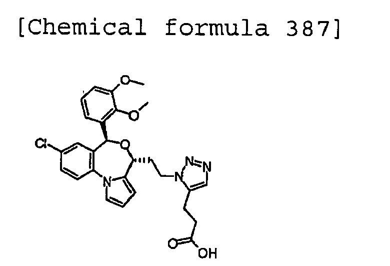

In a similar manner to that employed for the synthesis of 1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5-carboxylic acid, methyl 3-(1-{2-[(4R,6S)-8-chloro-6-(2,3-dimethoxyphenyl)-4H,6H-pyrrolo[1,2-a] [4,1]benzoxazepin-4-yl]ethyl}-1H-1,2,3-triazol-5-yl)propanoate (38 mg) was treated using potassium carbonate (49 mg) to give the title compound (15 mg). ¹H-NMR(CDCl₃)δ:2.56-2.73(4H,m), 2.85-3.03(2H,m),3.43(3H,s),3.85(3H,s),4.35-4.44(1H,m),4.51-4.68(2H,m),5.75(1H,s),6.28-6.32(1H,m),6.35-6.40(1H,m),6.71-6.75(1H,m),6.95(1H,d,J=8.1Hz),7.07-7.12(1H,m),7.16-7.22(1H,m),7.29-7.39(3H,m),7.44-7.49(1H,m).
IR(ATR)cm⁻
¹:2937,1716,1481,1428,1277,1171,1099,1054,1003,908,823,725.
MS(ESI)m/z:523(M⁺+H).

### Referential Example 65

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-3-yl)acetonitrile

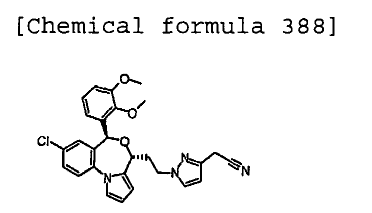

To a dichloromethane solution (47 mL) of (1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)methanol (4.69 g) subjected to azeotropy with toluene were added triphenylphosphine (3.08 g) and carbon tetrabromide (3.89 g) under ice cooling. The resulting mixture was stirred for 1 hour while keeping ice cooling. Under ice cooling, sodium cyanide (2.40 g) and dimethylsulfoxide (47 mL) were added. The reaction mixture was stirred at 40°C for 2 hours (disappearance of a bromine derivative and generation of the intended product were confirmed by TLC). After the reaction mixture was cooled to room temperature, saturated brine and ethyl acetate were added to carry out separating and extracting operations. The organic layer was washed with distilled water and saturated brine and then dried over anhydrous sodium sulfate. The organic solvent was distilled off under reduced pressure and the residue was subjected to column chromatography (silica gel 800 g, chloroform:methanol=200:1) to give the title compound (3.44 g).
MS(ESI)m/z:489(M⁺+H).
¹H-NMR(CDCl₃)δ:2.52-2.62(2H,m),3.44(3H,s),3.70(2H,s),3.86(3H,s),4.27-4.48(3H,m),5.74(1H,s),6.21(1H,dd,J=2.2,0.5Hz),6.29-6.32(1H,m),6.37(1H,t,J=3.2Hz),6.73(1H,d,J=2.0Hz),6.97(1H,dd ,J=8.1,1.5Hz),7.09(1H,dd,J=2.9,1.5Hz),7.21(1H,t,J=8.0Hz),7. 30-7.39(4H,m).

### Example 158

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-3-yl)acetic acid

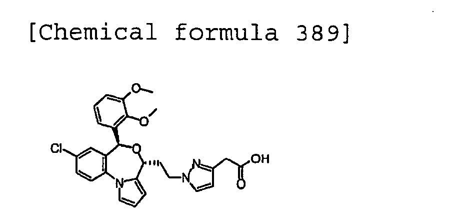

To a solution of 2-(1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-3-yl)acetonitrile (3.44 g) in a mixture of 2-propanol (42 mL) and methanol (28 mL) was added a 5 mol/l aqueous solution (28 mL) of sodium hydroxide at room temperature. The resulting mixture was stirred for 4 hours under reflux heating. After completion of the reaction, the solvent was distilled off under reduced pressure. The residue thus obtained was dissolved in dichloromethane. Citric acid monohydrate (9.85 g) was added in portions. A small amount of citric acid monohydrate was added further until the water layer became weakly acidic and separating and extracting operations were performed. The organic layer thus obtained was washed with saturated brine and dried over anhydrous sodium sulfate. The organic solvent was distilled off under reduced pressure. The residue was dissolved in a small amount of dichloromethane, followed by the addition of normal-hexane to precipitate a solid component. The solid component was collected by filtration and washed with normal-hexane. The resulting solid component was stirred for 30 minutes in a mixed solution of diethyl ether-normal-hexane to give the title compound (3.08 g).
¹H-NMR(CDCl₃)δ:2.50-2.66(2H,m),3.44(3H,s),3.73(2H,s),3.86(3H,s),4.29-4.35(1H,m),4.35-4.50(2H,m),5.74(1H,s),6.10(1H,d,J=2.2Hz),6.29-6.32(1H,m),6.37(1H,t,J=3.2Hz),6.73(1H,d,J=2.2Hz),6.97(1H,dd ,J=8.1,1.5Hz),7.09(1H,dd,J=2.9,1.7Hz),7.21(1H,t,J=8.1Hz),7. 29-7.41(4H,m).
IR (ATR) cm⁻
¹:2933,1716,1481,1277,1173,1099,1051,1003,758,714.
Elemental analysis for: C₂₇H₂₆ClN₃O₅
Calculated: C,63.84;H,5.16;N,8.27.
Found: C,63.66;H,5.18;N,8.02.

### Referential Example 66

(4R,6S)-4-(3-Butynyl)-8-chloro-6-(2,3-dimethoxyphenyl)-4H,6H-pyrrolo[1,2-a] [4,1]benzoxazepine

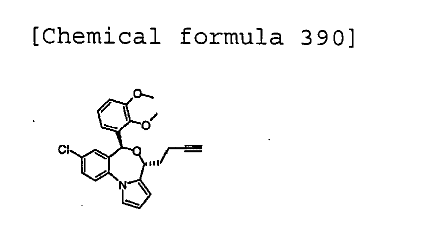

2-[(4R,6S)-8-Chloro-6-(2,3-dimethoxyphenyl)-4H,6H-pyrrolo[1,2-a] [4,1]benzoxazepin-4-yl]ethyl methanesulfonate (798 mg) was dissolved in dimethylsulfoxide (8 mL). To the resulting solution were added tetra-n-butylammonium iodide (617 mg) and lithium acetylide ethylenediamine complex (461 mg). The resulting mixture was stirred overnight at room temperature. Water was added to the reaction mixture under ice cooling. After stirring for 5 minutes, the reaction mixture was extracted with diethyl ether. The organic layer was dried over anhydrous magnesium sulfate. The solvent was then distilled off under reduced pressure. The residue was purified by silica gel column chromatography (hexane:ethyl acetate=20:1) to give the title compound (248 mg).
MS(FAB)m/z:408(M⁺+H) .
¹H-NMR(CDCl₃)δ:2.16-2.39(2H,m),2.42-2.58(2H,m),2.84-2.92(1H,m),3.44(3H,s),3.85(3H,s),4.45-4.56(1H,m),5.71(1H,s),6.31(1H,s),6.35-6.43(1H,m),6.71-6.76(1H,m),6.92-7.01(1H,m),7.10(1H,s),7.15-7.23(1H,m),7.26-7.28(1H,m),7.31-7.39(2H,m).

### Example 159

Ethyl 5-{2-[(4R,6S)-8-chloro-6-(2,3-dimethoxyphenyl)-4H,6H-pyrrolo[1,2-a][4,1]benzoxazepin-4-yl]ethyl}-3-isoxazolecarboxylate

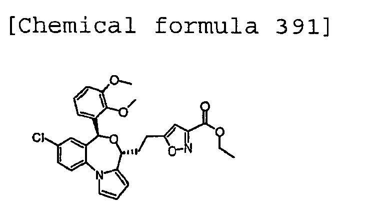

(4R,6S)-4-(3-Butynyl)-8-chloro-6-(2,3-dimethoxyphenyl)-4H,6H-pyrrolo[1,2-a][4,1]benzoxazepine (363 mg) was dissolved in diethyl ether (20 mL). To the resulting solution was added triethylamine (123 µl). The resulting mixture was stirred overnight at room temperature. Ethyl acetate and water were added to the reaction mixture and the layers separated. The organic layer was then dried over anhydrous sodium sulfate and the solvent was distilled off under reduced pressure. The residue was purified by silica gel column chromatography (hexane:ethyl acetate= from 30:1 to 10:1) to give the title compound (44 mg).
MS(FAB)m/z:523(M⁺+H).
¹H-NMR(CDCl₃)δ:1.34-1.44(3H,m),2.34-2.57(2H,m),2.99-3.25(2H,m),3.44(3H,s),3.86(3H,s),4.35-4.46(3H,m),5.73(1H,s),6.27-6.48(3H,m),6.73(1H,s),6.91-6.98(1H,m),7.06-7.12(1H,m),7.16-7.41(4H,m).

### Example 160

5-{2-[(4R,6S)-8-Chloro-6-(2,3-dimethoxyphenyl)-4H,6H-pyrrolo[1,2-a] [4,1]benzoxazepin-4-yl]ethyl}-3-isoxazolecarboxylic acid

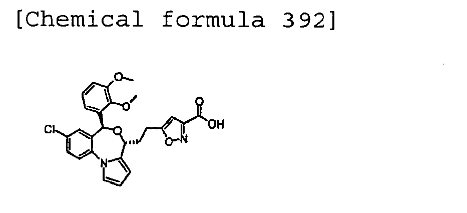

Ethyl 5-{2-[(4R,6S)-8-chloro-6-(2,3-dimethoxyphenyl)-4H,6H-pyrrolo[1,2-a][4,1]benzoxazepin-4-yl]ethyl}-3-isoxazolecarboxylate (30 mg) was dissolved in methanol (2 mL). To the resulting solution were added water (2 mL) and anhydrous potassium carbonate (24 mg) and the resulting mixture was stirred under heat at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was then distilled off under reduced pressure to give the title compound (27 mg).
MS(FD)m/z:494(M⁺).
¹H-NMR(CDCl₃)δ:2.36-2.60(2H,m)3.02-3.29(2H,m),3.44(3H,s),3.86(3H,s),4.39-4.45(1H,m),5.74(1H,s),6.33(1H,s),6.39(1H,s),6.50(1H,s),6.73 (1H,s),6.92-7.00(1H,m),7.11(1H,s),7.17-7.41(4H,m).
Elemental analysis for: C₂₆H₂₃ClN₂O₆·1.5H₂O
Calculated: C,59.83;H,5.02;N,5.37.
Found: C,60.22;H,4.75;N,5.04.

### Example 161

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol)acetate (position isomer A)

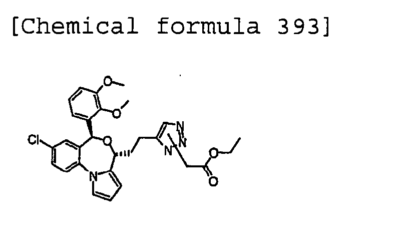

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol)acetate (position isomer B)

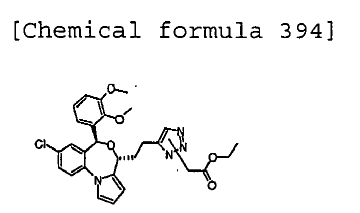

(4R,6S)-4-(3-butynyl)-8-chloro-6-(2,3-dimethoxyphenyl)-4H,6H-pyrrolo[1,2-a][4,1]benzoxazepine (167 mg) was dissolved in toluene (6 mL). To the resulting solution was added ethyl azidoacetate (495 mg). The resulting mixture was heated under reflux overnight. The reaction mixture was concentrated under reduced pressure. The residue was purified by silica gel column chromatography (hexane:ethyl acetate=3:1) to give a mixture of the title compounds. From the mixture, isomers were separated by high performance liquid chromatography (development solvent: a 30% isopropyl alcohol, n-hexane solution) using CHIRALPAK AD (Daicel Chemical), whereby a position isomer A (49 mg, 22%) eluted with a retention time of 28 minutes and a position isomer B (54 mg) eluted with a retention time of 28 minutes were obtained, respectively as the title compounds.
Position isomer A:
MS(FAB) m/z :537 (M⁺+H).
¹H-NMR (CDCl₃) δ:1.25 (3H,t,J=7.1Hz), 2.27-2.39(1H,m), 2.41-2.52(1H,m),2.79-2.89(1H,m),2.93-3.04(1H,m),3.43(3H,s),3.86(3H,s),4.21(2H,q,J=7.1Hz),4.37-4.42(1H,m),5.07(2H,s),5.73(1H,s),6.29-6.32(1H,m),6.39(1H,t,J=3.2Hz),6.73(1H,d, J=2.0Hz),6.96(1H,d, J=7.9Hz),7.10-7.12(1H,m),7.19(1H,t,J=7.9Hz),7.33-7.41(3H,m),7.54(1H,s).
Position isomer B:
MS(FAB)m/z:537(M⁺+H).
¹H-NMR(CDCl₃)δ:1.28(3H,t,J=7,1Hz),2.36-2.54(2H,m),2.93-3.03(1H,m),3.10-3.19(1H,m),3.44(3H,s),3.85(3H,s),4.25(2H,q,J=7.1Hz),4.41(1H ,dd,J=8.7,4.6Hz),5.09(2H,s),5.73(1H,s),6.32-6.35(1H,m),6.37(1H,t,J=3.1Hz),6.72(1H,s),6.95(1H,d,J=7.8Hz) ,7.09(1H,s),7.19(1H,t,J=7.8Hz),7.31-7.38(2H,m),7.45(1H,s).

### Example 162

2-(5-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol)acetic acid (position isomer A)

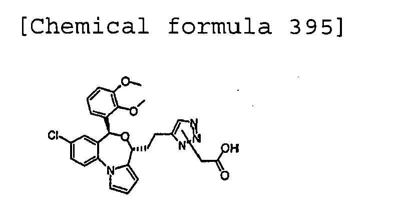

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol)acetate (position isomer A, 45 mg) was dissolved in methanol (10 mL). To the resulting solution were added water (3 mL) and anhydrous potassium carbonate (35 mg). The resulting mixture was stirred under heat at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (37 mg).
MS(FAB)m/z:509(M⁺+H)
¹H-NMR(CDCl₃)δ:2.27-2.39(1H,m),2.39-2.50(1H,m),2.78-2.90(1H,m),2.94-3.06(1H,m),3.42(3H,s),3.84(3H,s),4.39(1H,br s),5.09(2H,br s),5.72(1H,s),6.28(1H,s),6.37(1H,s),6.73(1H,s),6.91-6.97(1H,m),7.09(1H,s),7.13-7.20(1H,m),7.22-7.25(1H,m),7.34(2H,s),7.56(1H,s).

### Example 163

2-(5-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol)acetic acid (position isomer B)

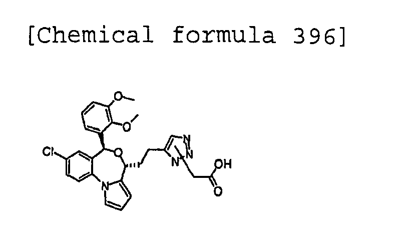

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol)acetate (position isomer B, 48 mg) was dissolved in methanol (10 mL). To the resulting solution were added water (3 mL) and anhydrous potassium carbonate (37 mg). The resulting mixture was stirred under heat at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (45 mg).
MS(FAB)m/z:509(M⁺+H).
¹H-NMR(CDCl₃)δ:2.31-2.49(2H,m),2.87-2.99(1H,m),3.04-3.17(1H,m),3.42(3H,s),3.83(3H,s),4.39(1H, br s),5.05(2H,s),5.72(1H,s),6.27-6.39(2H,m),6.71(1H,s),6.93(1H,s),7.07(1H,s),7.12-7.20(1H,m),7.23-7.37(3H,m),7.45(1H,s).

### Example 164

Ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-imidazol-4-yl)acetate

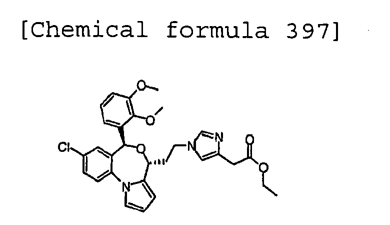

2-[(4R,6S)-8-Chloro-6-(2,3-dimethoxyphenyl)-4H,6H-pyrrolo[1,2-a][4,1]benzoxazepin-4-yl]ethyl methanesulfonate (798 mg) was dissolved in N,N-dimethylformamide (10 mL).
To the resulting solution were added potassium carbonate (221 mg), tetra-n-butylammonium iodide (148 mg), and ethyl imidazol-(4)5-acetate (152 mg) synthesized in accordance with the process described in the document (J.Med.Chem., 30, 2222-2227) and the resulting mixture was stirred under heat at 80°C for 2 hours. After concentration of the reaction mixture under reduced pressure, water (20 mL) and ethyl acetate (20 mL) were added to the residue and the layers separated. The organic layer was dried over anhydrous sodium sulfate. The solvent was distilled off under reduced pressure. The residue was purified by silica gel column chromatography (hexane:ethyl acetate=9:1) to give the title compound (15 mg).
MS(FAB)m/z:536(M⁺+H).
¹H-NMR(CDCl₃)δ:1.25(3H,t,J=7.1Hz),2.34-2.45(1H,m),2.50-2.63(1H,m),3.43(3H,s),3.59(2H,s),3.86(3H,s),4.09-4.29(4H,m),4.33-4.39(1H,m),5.74(1H,s),6.27-6.29(1H,m),6.38(1H,t,J=3.2Hz),6.72-6.75(1H,m),6.90(1H,s),6.97(1H,d,J=8.3Hz),7.09-7.11(1H,m),7.18-7.40(4H,m),7.43(1H,s).

### Example 165

2-(1-{2-[(4R,6S)-8-Chloro-6-(2,3-dimethoxyphenyl)-4H,6H-pyrrolo[1,2-a][4,1]benzoxazepin-4-yl]ethyl}-1H-imidazol-4-yl)acetic acid

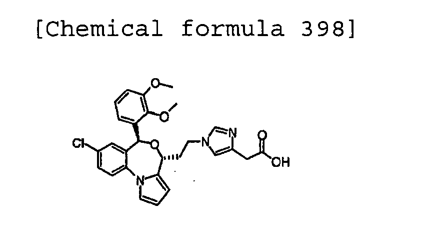

Ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-imidazol-4-yl)acetate (15 mg) was dissolved in methanol (1 mL). To the resulting solution were added tetrahydrofuran (0.5 mL), water (1 mL) and anhydrous potassium carbonate (12 mg). The resulting mixture was stirred under heat at 65°C for 2 hours. After concentration of the reaction mixture under reduced pressure, acetic acid (10 µl), chloroform and water were added to the residue and the layers separated. The organic layer was dried over anhydrous sodium sulfate and then, the solvent was distilled off under reduced pressure. Water was added to the residue and azeotropy with acetic acid was performed under reduced pressure, whereby the title compound (13 mg).
MS(EI)m/z:507(M⁺).
¹H-NMR(CDCl₃)δ:2.34-2.45(1H,m) ,2.51-2.63(1H,m),3.43(3H,s),3.60(2H,s),3.86(3H,s),4.14-4.41(3H,m),5.74(1H,s),6.27(1H,s),6.37(1H,s),6.73(1H,s),6.85 (1H,s),6.97(1H,d,J=7.1Hz),7.10(1H,s),7.17-7.40(4H,m),7.63(1H,s).

### Referential Example 67

3-(4-{3-[(4R,6S)-8-Chloro-6-(2,3-dimethoxyphenyl)-4H,6H-pyrrolo[1,2-a][4,1]benzoxazepin-4-yl]propanohydrazine

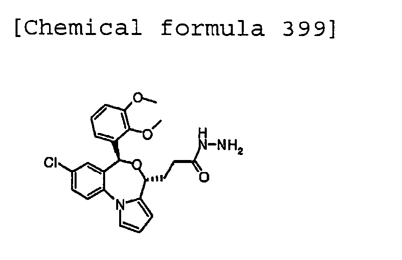

2-(4-{3-[(4R,6S)-8-Chloro-6-(2,3-dimethoxyphenyl)-4H,6H-pyrrolo[1,2-a][4,1]benzoxazepin-4-yl]propanoic acid (500 mg) was dissolved in N,N-dimethylformamide (6 mL). To the resulting solution were added potassium carbonate (243 mg) and methyl iodide (219 µl). The resulting mixture was stirred at room temperature for 90 minutes. The reaction mixture was filtered through a Kiriyama funnel, followed by concentration under reduced pressure. The residue was dissolved in ethanol (15 mL). To the resulting solution was added hydrazine monohydrate (5 mL). The mixture was stirred under heat at 65°C overnight. After concentration of the reaction mixture under reduced pressure, chloroform and a 10% aqueous citric acid solution were added to the residue and the layers separated. The organic layer was washed successively with a saturated aqueous solution of sodium hydrogen carbonate and water. The solvent was then concentrated under reduced pressure to give the title compound (415 mg).
MS (FAB)m/z:442 (M⁺+H).
¹H-NMR(CDCl₃)δ:2.35-2.55(4H,m),3.44(3H,s),3.86(5H,s),4.34-4.42(1H,m),5.71(1H,s),6.31-6.34(1H,m),6.38(1H,t,J=3.1Hz),6.71(1H,d,J=1.7Hz),6.81-6.86(1H,m),6.95(1H,d,J=7.9Hz),7.07-7.10(1H,m),7.19(1H,t,J=7.9Hz),7.25-7.28(2H,m),7.32-7.40(2H,m).

### Referential Example 68

5-{2-[(4R,6S)-8-Chloro-6-(2,3-dimethoxyphenyl)-4H,6H-pyrrolo[1,2-a][4,1]benzoxazepin-4-yl]ethyl}-1,3,4-oxadiazol-2(3H)-one

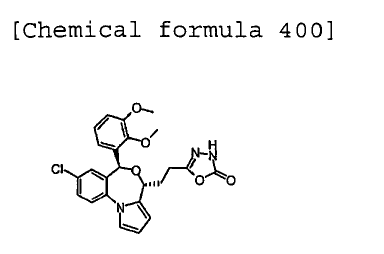

3-(4-{3-[(4R,6S)-8-Chloro-6-(2,3-dimethoxyphenyl)-4H,6H-pyrrolo[1,2-a][4,1]benzoxazepin-4-yl]propanohydrazine (148 mg) was dissolved in tetrahydrofuran (5 mL). To the resulting solution was added 1,1'-carbonylbis-1H-imidazole (92 mg). The resulting mixture was heated under reflux for 4 hours. The reaction mixture was concentrated under reduced pressure and the residue was purified by silica gel column chromatography (hexane:ethyl acetate=4:1) to give the title compound (95 mg).
MS(EI)m/z:467(M⁺).
¹H-NMR(CDCl₃)δ:2.35-2.55(2H,m),2.78-2.87(1H,m),2.91-3.00(1H,m),3.44(3H,s),3.86(3H,s),4.40-4.45(1H,m),5.73(1H,s),6.31-6.34(1H,m),6.39(1H,t,J=3.2Hz),6.72(1H,d,J=2.2Hz),6.94-6.98(1H,m),7.10-7.12(1H,m),7.20(1H,t,J=7.9Hz),7.26-7.30(1H,m),7.35-7.40(2H,m),8.25(1H,br s).

### Example 166

2-[5-{2-[(4R,6S)-8-Chloro-6-(2,3-dimethoxyphenyl)-4H,6H-pyrrolo[1,2-a][4,1]benzoxazepin-4-yl]ethyl}-2-oxo-1,3,4-oxadiazol-3(2H)-yl]acetic acid

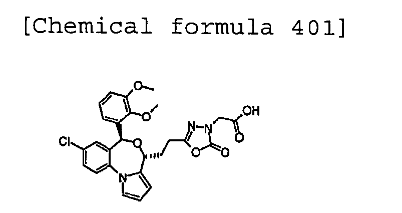

5-{2-[(4R,6S)-8-Chloro-6-(2,3-dimethoxyphenyl)-4H,6H-pyrrolo[1,2-a][4,1]benzoxazepin-4-yl]ethyl}-1,3,4-oxadiazol-2(3H)-one (73 mg) was dissolved in tetrahydrofuran (2 mL). To the resulting solution were added triphenylphosphine (103 mg) and benzyl glycolate (33 µl). A toluene solution (40% solution, 178 µl) of diethyl diazodicarboxylate was added. The resulting mixture was stirred at room temperature for 4 hours. After concentration of the reaction mixture under reduced pressure, the residue was purified by silica gel column chromatography (hexane:ethyl acetate=7:1) and dissolved in ethyl acetate (2 mL). To the resulting solution was added 10% palladium-carbon (water content: 50%) (85 mg). Under a hydrogen gas stream, the resulting mixture was vigorously stirred at room temperature under a constant pressure for 7 hours. The reaction mixture was filtered through a Kiriyama funnel. The filtrate was concentrated under reduced pressure. The residue was purified by thin layer silica gel chromatography (chloroform:methanol=10:1) to give the title compound (35 mg).
MS(FAB)m/z:526(M⁺+H).
¹H-NMR(CDCl₃)δ:2.20-2.42(2H,m),2.57-2.77(1H,m),2.79-2.94(1H,m),3.36(3H,s),3.79(3H,s),4.10-4.42(3H,m),5.65(1H,s),6.26(2H,br s),6.67(1H,s),6.81-6.91(1H,m),7.10(1H,m),7.15(1H,s),7.20-7.39(3H,m).

### Referential Example 69

Ethyl 5-[(4R,6S)-8-chloro-6-(2,3-dimethoxyphenyl)-4H,6H-pyrrolo[1,2-a][4,1]benzoxazepin-4-yl]-3-oxopentanoate

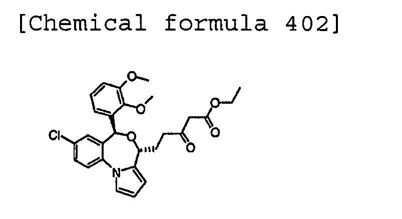

Monoethyl malonate potassium salt (160 mg), magnesium chloride (134 mg), and triethylamine (324 µl) were dissolved in ethyl acetate (4 mL). The resulting mixture was stirred under heat at 40°C overnight. The reaction mixture thus obtained was provided as Reaction mixture A. 2-(4-{3-[(4R,6S)-8-Chloro-6-(2,3-dimethoxyphenyl)-4H,6H-pyrrolo[1,2-a][4,1]benzoxazepin-4-yl]propanoic acid (200 mg) was dissolved in tetrahydrofuran (4 mL). To the resulting solution was added 1,1'-carbonylbis-1H-imidazole (92 mg), followed by stirring overnight at room temperature. The reaction mixture thus obtained was provided as Reaction mixture B. Reaction B was added dropwise to Reaction A under ice cooling. The resulting mixture was stirred overnight at room temperature. Water was then added to the reaction mixture and the layers separated. The organic layer was dried over anhydrous sodium sulfate. The solvent was distilled off under reduced pressure. The residue was purified by silica gel column chromatography (hexane:ethyl acetate=5:1) to give the title compound (64 mg).
MS(FAB)m/z:497(M⁺+H).
¹H-NMR(CDCl₃)δ:1.23-1.28(3H,m),2.32-2.41(2H,m),2.77-2.97(2H,m),3.40-3.48(5H,m),3.85-3.85(3H,m),4.14-4.21(2H,m),4.33-4.39(1H,m),5.70(1H,s),6.31-6.34(1H,m),6.37-6.39(1H,m),6.69-6.72(1H,m),6.93-6.97(1H,m),7.08-7.10(1H,m),7.17-7.22(1H,m),7.27-7.29(1H,m),7.32-7.38(2H,m).

### Example 167

Ethyl 2-(3-{2-[(4R,6S)-8-chloro-6-(2,3-dimethoxyphenyl)-4H,6H-pyrrolo[1,2-a][4,1]benzoxazepin-4-yl]ethyl}-5-oxo-4,5-dihydro-1H-pyrazol-1-yl)acetate

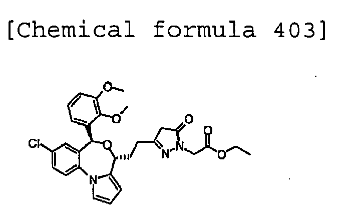

Ethyl 5-[(4R,6S)-8-chloro-6-(2,3-dimethoxyphenyl)-4H,6H-pyrrolo[1,2-a][4,1]benzoxazepin-4-yl]-3-oxopentanoate (103 mg) was dissolved in ethanol (3 mL). To the resulting solution were added hydrazinoacetic acid hydrochloride (32 mg) and triethylamine (29 µl). The resulting mixture was heated under reflux for 3 hours. After concentration of the reaction mixture under reduced pressure, chloroform and water were added to the residue and the layers separated. The organic layer was dried over anhydrous sodium sulfate and the solvent was distilled off under reduced pressure. The residue was purified by silica gel column chromatography (hexane:ethyl acetate=2:1) to give the title compound (71 mg).
MS(FAB)m/z:552(M⁺+H).
¹H-NMR(CDCl₃)δ:1.23-1.31(3H,m),2.27-2.46(2H,m),2.59-2.87(2H,m),3.27(2H,br s),3.41-3.46(3H,m),3.81-3.88(3H,m),4.16-4.24(2H,m),4.37-4.44(3H,m),5.71(1H,s),6.30-6.35(1H,m),6.36-6.41(1H,m),6.72(1H,s),6.92-6.99(1H,m),7.10(1H,s),7.23-7.29(1H,m),7.31-7.41(3H,m).

### Example 168

2-(3-{2-[(4R,6S)-8-Chloro-6-(2,3-dimethoxyphenyl)-4H,6H-pyrrolo[1,2-a][4,1]benzoxazepin-4-yl]ethyl}-5-oxo-4,5-dihydro-1H-pyrazol-1-yl)acetic acid

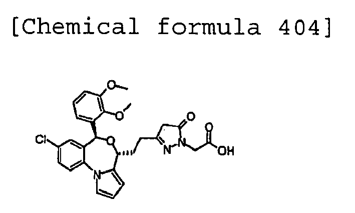

Ethyl 2-(3-{2-[(4R,6S)-8-chloro-6-(2,3-dimethoxyphenyl)-4H,6H-pyrrolo[1,2-a][4,1]benzoxazepin-4-yl]ethyl}-5-oxo-4,5-dihydro-1H-pyrazol-1-yl)acetate (68 mg) was dissolved in methanol (3 mL). To the resulting solution were added tetrahydrofuran (3 mL), water (5 mL) and a 1N aqueous sodium hydroxide solution (571 µl). The resulting mixture was stirred under heat at 65°C overnight. After concentration of the reaction mixture under reduced pressure, chloroform and a 10% aqueous citric acid solution were added to the residue and the layers separated. The organic layer was dried over anhydrous sodium sulfate and the solvent was then distilled off under reduced pressure to give the title compound (90 mg).
MS(FAB)m/z:524 (M⁺+H).
¹H-NMR(CDCl₃)δ:2.17-2.46(2H,m),2.57-2.68(1H,m),2.72-2.84(1H,m),3.27(2H,s),3.35-3.46(3H,m),3.75-3.89(3H,m),4.31-4.48(3H,m),5.67-5.74(1H,m),6.23-6.41(2H,m),6.67-6.75(1H,m),6.91-7.03(1H,m),7.06-7.19(1H,m),7.22-7.39(3H,m).

### Example .169

Ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-imidazol-4-yl)-2-methylpropanoate

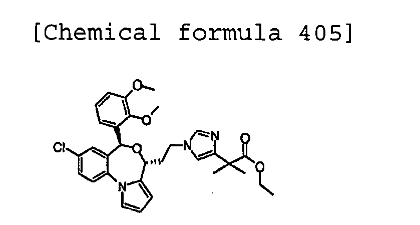

2-[(4R,6S)-8-Chloro-6-(2,3-dimethoxyphenyl)-4H,6H-pyrrolo[1,2-a][4,1]benzoxazepin-4-yl]ethyl methanesulfonate (647 mg) was dissolved in N,N-dimethylformamide (30 mL). To the resulting solution were added potassium carbonate (373 mg), tetra-n-butylammonium iodide (500 mg), and ethyl 2-methyl-2-(1H-imidazol-4-yl)propanoate (370 mg) synthesized in accordance with the document *(*Bioorg. Med. Chem., 12, 2251-2273). The resulting mixture was stirred under heat at 70°C for 4 hours. After concentration of the reaction mixture under reduced pressure, water (40 mL) and ethyl acetate (40 mL) were added to the residue and the layers separated. The organic layer was dried over anhydrous sodium sulfate and the solvent was distilled off under reduced pressure. The residue was purified by silica gel column chromatography (hexane:ethyl acetate=1:1) to give the title compound (130 mg).
MS(EI)m/z:563(M⁺).
¹H-NMR(CDCl₃)δ:1.18(3H,t,J=7.1Hz),1.48-1.49(6H,m),2.33-2.43(1H,m),2.50-2.61(1H,m),3.43(3H,s),3.86(3H,s),4.10(2H,q,J=7.1Hz),4.16-4.27(2H,m),4.30-4.36(1H,m),5.74(1H,s),6.27-6.30(1H,m),6.36-6.39(1H,m),6.74(1H,d,J=2.0Hz),6.77-6.79(1H,m),6.98(1H,d,J=8.1Hz),7.09-7.11(1H,m),7.22(1H,t,J=8.1Hz),7.29-7.41(4H,m).

### Example 170

2-(1-{2-[(4R,6S)-8-Chloro-6-(2,3-dimethoxyphenyl)-4H,6H-pyrrolo[1,2-a][4,1]benzoxazepin-4-yl]ethyl}-1H-imidazol-4-yl)-2-methylpropanoic acid

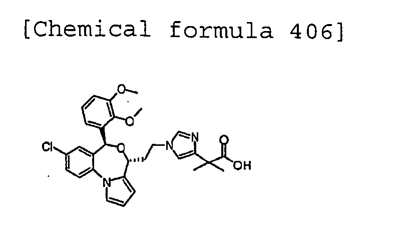

Ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-imidazol-4-yl)-2-methylpropanoate (135 mg) was dissolved in methanol (2 mL). To the resulting solution were added tetrahydrofuran (2 mL), water (5 mL), and a 1N aqueous sodium hydroxide solution (956 µl). The resulting mixture was heated under reflux overnight. After concentration of the reaction mixture under reduced pressure, a 10% aqueous citric acid solution was added to the residue and the layers separated. The organic layer was washed with water and then, dried over anhydrous sodium sulfate. The solvent was distilled off under reduced pressure to give the title compound (120 mg).
MS(FAB)m/z:536(M⁺+H).
¹H-NMR(CDCl₃)δ:1.45(3H,s), 1.49(3H,s),2.33-2.44(1H,m), 2.52-2.65(1H,m),3.44(3H,s),3.87(3H,s),4.19-4.36(3H,m),5.75(1H,s),6.27-6.30(1H,m),6.37-6.40(1H,m),6.74-6.76(1H,m),6.79(1H,s),6.98(1H,d,J=8.1Hz),7.11(1H,s),7.21-7.41(4H,m),7.49(1H,s).

### Example 171

Ethyl 2-(2-{3-[(4R,6S)-8-chloro-6-(2,3-dimethoxyphenyl)-4H,6H-pyrrolo[1,2-a][4,1]benzoxazepin-4-yl]propyl}-2H-1,2,3,4-tetrazol-5-yl)acetate

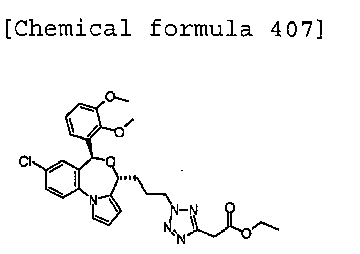

Ethyl 2-(1-{3-[(4R,6S)-8-chloro-6-(2,3-dimethoxyphenyl)-4H,6H-pyrrolo[1,2-a][4,1]benzoxazepin-4-yl]propyl}-1H-1,2,3,4-tetrazol-5-yl)acetate

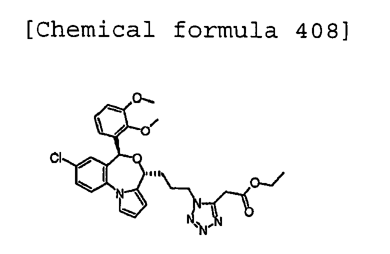

3-[(4R,6S)-8-Chloro-6-(2,3-dimethoxyphenyl)-4H,6H-pyrrolo[1,2-a][4,1]benzoxazepin-4-yl]propylmethanesulfonate (414 mg) was dissolved in dimethylformamide (5 mL). To the resulting solution were added ethyl 2-(1H-1,2,3,4-tetrazol-5-yl)acetate (194 mg), potassium carbonate (257 mg) and tetrabutylammonium iodide (229 mg). The resulting mixture was stirred at 80°C for 1 hour. After the reaction mixture was allowed to cool, water was added, followed by extraction with ethyl acetate. The extract was washed with saturated brine and dried over anhydrous sodium sulfate. The solvent was distilled off under reduced pressure. The residue was purified by silica gel column chromatography (hexane:ethyl acetate), whereby the title compounds, that is, 2H-isomer (197 mg) and 1H-isomer (99 mg) were obtained, respectively. 2H-Isomer
¹H-NMR(CDCl₃)δ:1.28-1.23(3H,m)2.46-1.94(4H,m),3.43(3H,s),3.85(3H,s),3.95(2H,s),4.16(2H,dq,J=29 .11,7.16Hz),4.38(1H,q,J=4.31Hz),4.78-4.62(2H,m),5.69(1H,s),6.27-6.24(1H,m),6.36(1H,t,J=3.30Hz),6.72(1H,d,J=2.20Hz),6.97-6.93(1H,m),7.08(1H,q,J=1.46Hz),7.19(1H,t,J=8.06Hz),7.39-7.28(3H,m). 1H-Isomer
¹H-NMR(CDCl₃)δ:1.20-1.31(3H,m), 1.97-2.41(4H,m),2.92(1H,d,J=28.32Hz),3.43(3H,s),3.85(3H,s),3.97( 2H,s),4.07-4.26(2H,m),4.32-4.50(2H,m),5.72(1H,s),6.26-6.26(1H,m),6.37(1H,t,J=3.30Hz),6.72(1H,d,J=2.20Hz),6.93-6.98(1H,m),7.09(1H,q,J=1.38Hz),7.19(1H,t,J=8.06Hz),7.24-7.42(3H,m).

### Example 172

2-(2-{3-[(4R,6S)-8-Chloro-6-(2,3-dimethoxyphenyl)-4H,6H-pyrrolo[1,2-a][4,1]benzoxazepin-4-yl]propyl}-2H-1,2,3,4-tetrazol-5-yl)acetic acid

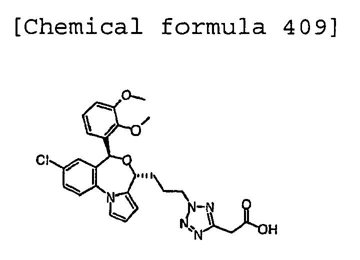

Ethyl 2-(2-{3-[(4R,6S)-8-chloro-6-(2,3-dimethoxyphenyl)-4H,6H-pyrrolo[1,2-a][4,1]benzoxazepin-4-yl]propyl}-2H-1,2,3,4-tetrazol-5-yl)acetate (197 mg) was dissolved in a tetrahydrofuran-methanol-water (1:1:2) solvent mixture (8 mL). The resulting solution and potassium carbonate (197 mg) were treated in a similar manner to that employed for the synthesis of 2-(5-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-2-yl)acetic acid, whereby the title compound (168.8 mg).
¹H-NMR(CDCl₃)δ:1.90-2.80(4H,m),3.40(3H,s),3.71(3H,s),3.81-3.84(2H,m),4.31-4.39(1H,m),4.53-4.76(2H,m),5.67(1H,s),6.21-6.27(1H,m),6.32-6.36(1H,m),6.70(1H,br s),6.92(1H,d,J=8.30Hz),7.08-7.04(1H,m),7.16(1H,t,J=8.06Hz),7.23-7.40(3H,m).

### Example 173

2-(1-{3-[(4R,6S)-8-Chloro-6-(2,3-dimethoxyphenyl)-4H,6H-pyrrolo[1,2-a][4,1]benzoxazepin-4-yl]propyl}-1H-1,2,3,4-tetrazol-5-yl)acetic acid

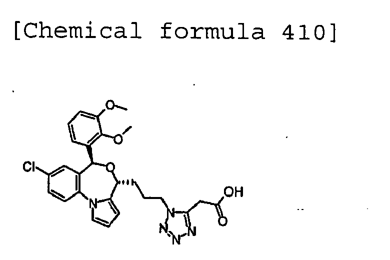

Ethyl 2-(1-{3-[(4R,6S)-8-chloro-6-(2,3-dimethoxyphenyl)-4H,6H-pyrrolo[1,2-a][4,1]benzoxazepin-4-yl]propyl}-1H-1,2,3,4-tetrazol-5-yl)acetate (99 mg) was dissolved in a tetrahydrofuran-methanol-water (1:1:2) solvent mixture (8 mL). The resulting solution and potassium carbonate (100 mg) were treated in a similar manner to that employed for the synthesis of 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-1-yl)acetic acid, whereby the title compound (68 mg).
Elemental analysis for: C₂₆H₂₆ClN₅O₅·0.25H₂O
Calculated: C,59.09;H,5.05;N,13.25.
Found: C,58.90;H,5.05;N,13.04.

### Referential Example 70

2,3-Dihydro-1,4-benzodioxin-5-carbaldehyde

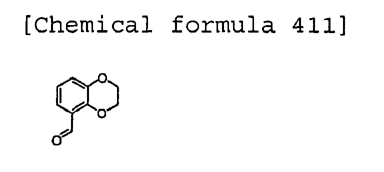

To an N,N-dimethylformamide solution (360 mL) of 2,3-dihydroxybenzaldehyde (5.16 g) were added 1,2-dibromoethane (3.86 mL) and potassium carbonate (13.94 g). The resulting mixture was stirred at 70°C for 3 hours. After the reaction mixture was cooled to room temperature, the solid component was filtered. The filtrate thus obtained was concentrated under reduced pressure. The residue thus obtained was subjected to silica gel column chromatography (hexane:ethyl acetate=5:1) to give the title compound (3.90 g).
MS (ESI)m/z:165(M⁺+H).
¹H-NMR(CDCl₃) δ:4.31-4.35 (2H,m),4.38-4.41(2H,m),6.91(1H,t,J=7.8Hz),7.10(1H,dd,J=8.1,1.7Hz),7.38-7.41(1H,m),10.37(1H,s).
IR(ATR)cm⁻
¹:2879,1680,1597,1477,1282,1248,1203,1082,887,781,723.

### Referential Example 71

tert-Butyl 4-chloro-2-[2,3-dihydro-1,4-benzodioxin-5-yl(hydroxy)methyl]phenylcarbamate

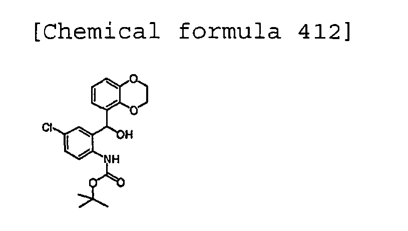

sec-Butyl lithium (0.99 mol/l, 44.3 mL) was added dropwise to a tetrahydrofuran solution (140 mL) of tert-butyl 4-chlorophenylcarbamate (4.16 g) at -78°C. While warming to 0°C, the reaction mixture was stirred for 1.5 hours. Under ice cooling, the mixture was stirred for further 0.5 hour. The reaction mixture was cooled to -78°C again and 2,3-dihydro-1,4-benzodioxin-5-carbaldehyde (3.90 g) was added thereto. The reaction mixture was mildly stirred for 2 hours while warming to room temperature. A saturated aqueous solution of ammonium chloride was then added. A separating operation was performed and the water phase was washed with ethyl acetate. The organic layers were combined and dried over anhydrous sodium sulfate. After concentration under reduced pressure, the residue was purified by silica gel column chromatography (hexane:ethyl acetate=9:1) to give the title compound (3.54 g).
MS (ESI)m/z:318 (M⁺-tBuO).
¹H-NMR(CDCl₃)δ:1.50(9H,s),3.14(1H,d,J=4.9Hz),4.22-4.37(4H,m),6.04(1H,d,J=5.4Hz),6.77-6.82(1H,m),6.85-6.89(2H,m),7.08(1H,d,J=2.5Hz),7.23(1H,dd,J=8.8,2.5Hz),7.91( 2H,d,J=8.3Hz),7.95(1H,br s).

### Referential Example 72

tert-Butyl 4-chloro-2-(2,3-dihydro-1,4-benzodioxin-5-ylcarbonyl)phenylcarbamate

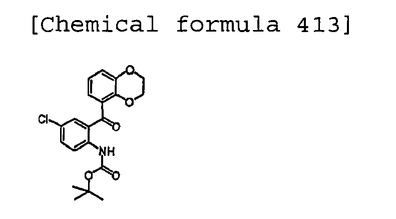

In a similar manner to that employed for the synthesis of [5-chloro-2-(1H-pyrrol-1-yl)-3-pyridinyl](2,3-dimethoxyphenyl)methanone, tert-butyl 4-chloro-2-[2,3-dihydro-1,4-benzodioxin-5-yl(hydroxy)methyl]phenylcarbamate (3.53 g) was treated using manganese dioxide (7.83 g) to give the title compound (4.16 g).
MS(ESI)m/z:290(M⁺-tBuO).
¹H-NMR(CDCl₃)δ:1.53 (9H,s) 4.20-4.25(2H,m),4.27-4.31(2H,m),6.86(1H,dd,J=7.6,1.7Hz),6.93(1H,t,J=7.6Hz),7.04( 1H,dd,J=7.8,1.5Hz),7.44-7.48(2H,m),8.47(1H,dd,J=7.4,2.5Hz),10.65(1H,s).
IR(ATR)cm⁻
¹:3276,2979,1728,1641,1574,1506,1248,1147,1086,829.

### Referential Example 73

(2-Amino-5-chlorophenyl)(2,3-dihydro-1,4-benzodioxin-5-yl) methanone

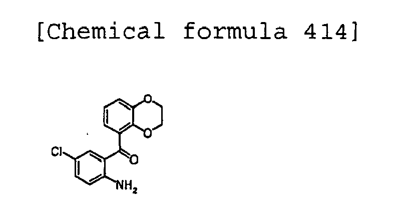

In accordance with the process described in J. Org. Chem., 55(4), 1379-1390(1990), the title compound (2.15 g) from tert-butyl 4-chloro-2-(2,3-dihydro-1,4-benzodioxin-5-ylcarbonyl)phenylcarbamate (4.16 g) by using trifluoroacetic acid (8.2 mL).
MS(ESI)m/z:290(M⁺+H).
¹H-NMR(CDCl₃)δ:1.55(9H,s),4.22-4.25(2H,m),4.28-4.32 (2H,m), 6.38 (2H,br s),6.65(1H,d,J=8.8Hz),6.83(1H,dd,J=7.6,1.7Hz),6.91(1H,t,J=7 .8Hz),6.99(1H,dd,J=8.1,1.5Hz),7.22(1H,dd,J=8.8,2.4Hz),7.31( 1H,d,J=2.4Hz).
IR(ATR)cm⁻¹:3452,3336,1624,1468,1281,1088,926,804,733.

### Referential Example 74

[5-Chloro-2-(1H-pyrrol-1-yl)phenyl](2,3-dihydro-1,4-benzodioxin-5-yl)methanone

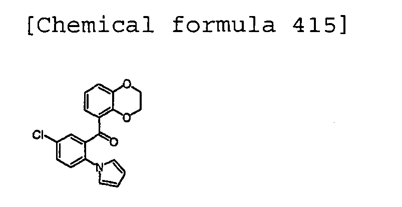

The title compound (2.07 g) was obtained from (2-amino-5-chlorophenyl)(2,3-dihydro-1,4-benzodioxin-5-yl)methanone (2.14 g) by using 2,5-dimethoxytetrahydrofuran (977 µl) in a similar manner to that employed for the synthesis of (5-chloro-2-(1H-pyrrol-1-yl)phenyl)(2,3-dimethoxyphenyl)methanone.
MS(ESI)m/z:340(M⁺+H).
¹H-NMR(CDCl₃)δ:4.11-4.16(2H,m),4.19-4.22(2H,m),6.05(2H,t,J=2.2Hz),6.66(2H,t,J=2.2Hz),6.69(1H,d, J=7.8Hz),6.84-6.90(2H,m),7.29(1H,d,J=8.6Hz),7.50(1H,dd,J=8.3,2.5Hz),7.57( 1H,d,J=2.5Hz).
IR(ATR)cm⁻
¹:1664,1595,1495,1469,1282,1254,1090,924,806,754,725.

### Referential Example 75

1-[4-Chloro-2-(2,3-dihydro-1,4-benzodioxin-5-ylcarbonyl)phenyl]-1H-pyrrol-2-carbaldehyde

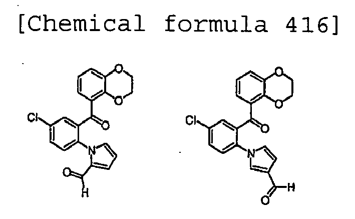

The title compound (0.75 g) was obtained from [5-chloro-2-(1H-pyrrol-1-yl)phenyl](2,3-dihydro-1,4-benzodioxin-5-yl)methanone (1.34 g) by using phosphorus oxychloride (441 µl) and N,N-dimethylformamide (610 µl) in a similar manner to that employed for the synthesis of 1-(4-chloro-2-(2,3-dimethoxybenzoyl)phenyl)-1H-pyrrolo-2-carbaldehyde and 1-(4-chloro-2-(2,3-dimethoxybenzoyl)phenyl)-1H-pyrrolo-3-carbaldehyde.
MS(ESI)m/z:368(M⁺+H)⁻.
¹H-NMR(CDCl₃)δ:4.09-4.21(4H,m),6.20(1H,dd,J=3.9,2.7Hz),6.68(1H,t,J=8.0Hz),6.81-6.85(2H,m),6.86-6.90(2H,m), 7.29(2H,d,J=8.3Hz), 7.53(1H,dd,J=8.6,2.5Hz), 7.61( 1H,d,J=2.5Hz), 9.36(1H,s).
IR(ATR)cm⁻
¹:1662,1589,1468,1281,1252,1086,1036,897,806,756,731.

### Referential Example 76

Benzyl (E)-3-{1-[4-chloro-2-(2,3-dihydro-1,4-benzodioxin-5-ylcarbonyl)phenyl]-1H-pyrrol-2-yl}-2-propenoate

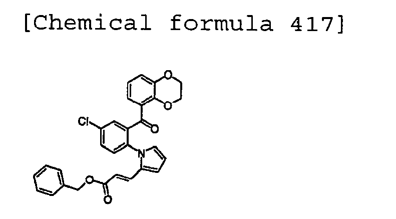

The title compound (410 mg) was obtained from 1-[4-chloro-2-(2,3-dihydro-1,4-benzodioxin-5-ylcarbonyl)phenyl]-1H-pyrrol-2-carbaldehyde (355 mg) by using benzyl(triphenylphosphoranylidene)acetic acid (429 mg) in a similar manner to that employed for the synthesis of methyl (E)-3-(1-(4-chloro-2-(2,3-dimethoxybenzoyl)phenyl)-1H-pyrrol-2-yl)-2-propenoate.
MS(ESI)m/z:500(M⁺+H).
¹H-NMR(CDCl₃)δ:3.94-4.06(4H,m),5.16(2H,d,J=5.6Hz),6.03(1H,d,J=15.7Hz),6.10-6.12(1H,m),6.54(1H,dd,J=4.0,1.3Hz),6.64-6.69(1H,m),6.76(1H,q,J=1.4Hz),6.84(1H,dd,J=8.1,1.5Hz),6.88( 1H,dd,J=7.8,1.7Hz),7.20(1H,d,J=8.3Hz),7.29-7.40(6H,m),7.55(1H,dd,J=8.3,2.5Hz),7.61(1H,d,J=2.5Hz).
IR(ATR)cm⁻¹:1699,1620,1450,1282,1244,1142,1088,727.

### Referential Example 77

Benzyl (E)-3-(1-{4-chloro-2-[2,3-dihydro-1,4-benzodioxin-5-yl(hydroxy)methyl]phenyl}-1H-pyrrol-2-yl)-2-propenoate

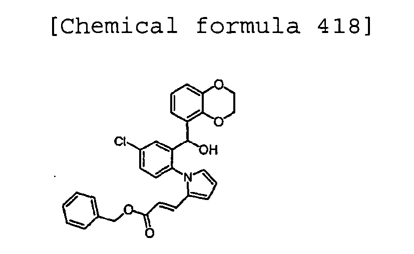

The title compound (430 mg) was obtained from benzyl (E)-3-{1-[4-chloro-2-(2,3-dihydro-1,4-benzodioxin-5-ylcarbonyl)phenyl]-1H-pyrrol-2-yl}-2-propenoic acid (406 mg) by using sodium borohydride (50 mg) in a similar manner to that employed for the synthesis of methyl (E)-3-(1-(4-chloro-2-((2,3-dimethoxyphenyl)(hydroxy)methyl)phenyl)-1H-pyrrol-2-yl)-2-propenoate.
MS(ESI)m/z:484(M⁺-OH).
¹H-NMR(CDCl₃)δ:3.68 (1H, d,J=17.2Hz),3.86-4.15(4H,m),4.70(1H,d,J=5.9Hz),5.13(1H,d,J=17.4Hz),5.74-6.01(2H,m),6.14-6.17 and 6.52-6.57(1H,m),6.29-6.37(1H,m),6.65-6.78(4H,m),6.86-7.00(1H,m),7.08-7.12(1H,m),7.28-7.40(5H,m),7.54 and 7.79(1H,d,J=2.5Hz).
IR(ATR)cm⁻
¹:1685,1618,1473,1450,1279,1244,1165,1088,1036,957,731.

### Example 174

Benzyl 2-[8-chloro-6-(2,3-dihydro-1,4-benzodioxin-5-yl)-4H,6H-pyrrolo[1,2-a][4,1]benzoxazepin-4-yl]acetate

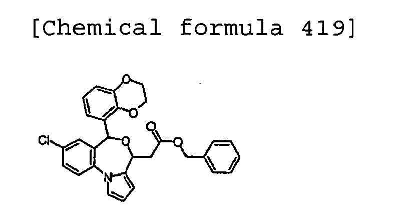

The title compound (177 mg) was obtained from benzyl (E)-3-(1-{4-chloro-2-[2,3-dihydro-1,4-benzodioxin-5-yl(hydroxy)methyl]phenyl}-1H-pyrrol-2-yl)-2-propenoate (418 mg) by using trifluoroacetic acid (77 µl) in a similar manner to that employed for the synthesis of methyl 2-(8-chloro-6-(2,3-dimethoxyphenyl)-4H,6H-pyrrolo(1,2-a)(4,1)benzoxazepin-4-yl)acetate. MS(ESI)m/z:502(M⁺+H).
¹H-NMR(CDCl₃)δ:2.46-2.76 and 2.98-3.19(2H,m),3.66 and 3.71(1H,s),3.96-4.01 and 4.03-4.08(2H,m),4.10-4.16(2H,m),4.87-4.95(1H,m),5.10 and 5.17(1H,s),5.65 and 5.66(1H,s),6.26-6.33(1H,m),6.35-6.38(1H,m),6.76-6.82(1H,m),6.85-7.02(2H,m),7.09-7.15 and 7.20-7.24(2H,m),7.30-7.41(7H,m).
IR(ATR)cm⁻¹:1736,1604,1473,1281,1171,1099,1051,889,823,717.

### Example 175

2-[8-Chloro-6-(2,3-dihydro-1,4-benzodioxin-5-yl)-4H,6H-pyrrolo[1,2-a][4,1]benzoxazepin-4-yl]acetic acid

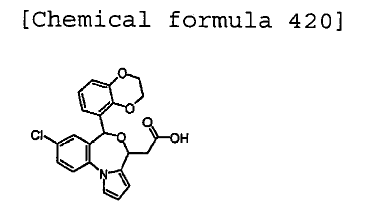

To an ethyl acetate solution (8 mL) of benzyl 2-[8-chloro-6-(2,3-dihydro-1,4-benzodioxin-5-yl)-4H,6H-pyrrolo[1,2-a] [4,1]benzoxazepin-4-yl]acetate (173 mg) was added 10% palladium-carbon (wet, 102 mg). The resulting mixture was stirred for 29 hours in a hydrogen atmosphere. The catalyst was filtered off, followed by washing with ethyl acetate sufficiently. The filtrates were combined and concentrated under reduced pressure to give the title compound (173 mg).
MS (ESI) m/z :410 (M⁻-H).
¹H-NMR(CDCl₃)δ:2.98-3.20(2H,m),3.97-4.19(4H,m),4.85-4.93(1H,m),5.63-5.72(1H,m),6.25-6.44(2H,m),6.70-7.03(3H,m),7.06-7.24(2H,m),7.33-7.45(2H,m).

### Example 176

Ethyl 2-(1-{2-[8-chloro-6-(2,3-dihydro-1,4-benzodioxin-5-yl)-4H,6H-pyrrolo[1,2-a][4,1]benzoxazepin-4-yl]acetyl}-4-piperidinyl)acetate

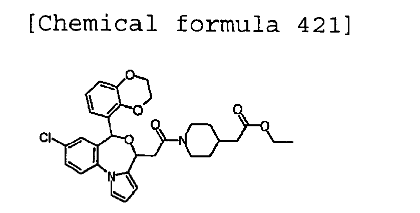

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (100 mg) was obtained using 2-[8-chloro-6-(2,3-dihydro-1,4-benzodioxin-5-yl)-4H,6H-pyrrolo[1,2-a][4,1]benzoxazepin-4-yl]acetic acid (34 mg), 1-ethyl-3- (3-dimethylaminopropyl)carbodiimide hydrochloride (98 mg), and 1-hydroxybenzotriazole (60 mg).
MS(ESI)m/z:565(M⁺+H).
¹H-NMR(CDCl₃)δ:1.02-1.23(1H,m),1.26(3H,t,J=7.1Hz),1.61-1.82(1H,m),1.92-2.30(4H,m),2.51-2.70(1H,m),2.73-2.92(1H,m),2.96-3.14(1H,m),3.20-3.30(1H,m),3.97-4.18(8H,m),4.55-4.73(1H,m),4.90-5.01(1H,m),5.66(1H,d,J=11.0Hz),6.24(1H,s),6.29-6.39(1H,m),6.71-6.83(1H,m),6.85-7.02(3H,m),7.07-7.26(1H,m),7.29-7.41(2H,m).

### Example 177

Ethyl 2-(1-{2-[(4R,6S)-8-chloro-6-(2,3-dihydro-1,4-benzodioxin-5-yl)-4H,6H-pyrrolo[1,2-a][4,1]benzoxazepin-4-yl]acetyl}-4-piperidinyl)acetate
Ethyl 2-(1-{2-[(4S,6R)-8-chloro-6-(2,3-dihydro-1,4-benzodioxin-5-yl)-4H,6H-pyrrolo[1,2-a][4,1]benzoxazepin-4-yl]acetyl}-4-piperidinyl)acetate

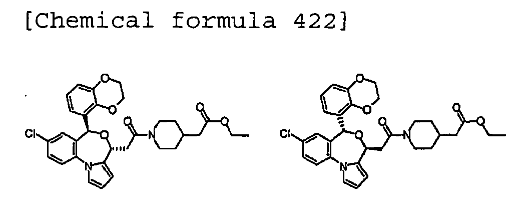

Ethyl 2-(1-{2-[8-chloro-6-(2,3-dihydro-1,4-benzodioxin-5-yl)-4H,6H-pyrrolo[1,2-a][4,1]benzoxazepin-4-yl]acetyl}-4-piperidinyl)acetate (80 mg) was subjected to CHIRALPAK AD using a 20% isopropanol-hexane solution at a flow rate of 10 mL/min to give ethyl 2-(1-{2-[(4R,6S)-8-chloro-6-(2,3-dihydro-1,4-benzodioxin-5-yl)-4H,6H-pyrrolo[1,2-a][4,1]benzoxazepin-4-yl]acetyl}-4-piperidinyl)acetate (32 mg) corresponding to a peak with a retention time of 29.7 minutes and ethyl 2-(1-{2-[(4S,6R)-8-chloro-6-(2,3-dihydro-1,4-benzodioxin-5-yl)-4H,6H-pyrrolo[1,2-a][4,1]benzoxazepin-4-yl]acetyl}-4-piperidinyl)acetate (32 mg) corresponding to a peak with a retention time of 15.3 minutes.

### Example 178

2-(1-{2-[(4R,6S)-8-Chloro-6-(2,3-dihydro-1,4-benzodioxin-5-yl)-4H,6H-pyrrolo[1,2-a][4,1]benzoxazepin-4-yl]acetyl}-4-piperidinyl)acetic acid

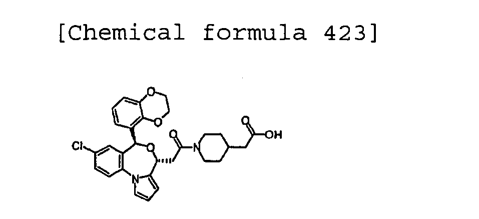

The title compound (19.6 mg) was obtained from ethyl 2-(1-{2-[(4R,6S)-8-chloro-6-(2,3-dihydro-1,4-benzodioxin-5-yl)-4H,6H-pyrrolo[1,2-a][4,1]benzoxazepin-4-yl]acetyl}-4-piperidinyl)acetate (22.5 mg) by using potassium carbonate (16.5 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid. MS(ESI)m/z:537(M⁺+H).
¹H-NMR(CDCl₃)δ:1.06-1.24(1H,m),1.64-1.87(2H,m),1.94-2.10(1H,m),2.14-2.37(2H,m),2.78-2.92(1H,m),2.96-3.14(1H,m),3.21-3.32(1H,m),3.38-3.61(1H,m),3.96-4.17(5H,m),4.59-4.76(1H,m),4.89-5.01(1H,m),5.67(1H,d,J=12.7Hz),6.25(1H,s),6.37(1H,t,J=3.2Hz ),6.74(1H,dd,J=7.2,2.1Hz),6.85-6.98(2H,m),7.09(1H,s),7.17-7.25(1H,m),7.30-7.39(1H,m).
IR(ATR)cm⁻¹:2922,1722,1597,1473,1281,1080,889,823,717.
Elemental analysis for: C₃₂H₄₃ClN₂O₇·0.25H₂O
Calculated: C,64.32;H,5.49;N,5.17.
Found: C,64.62;H,5.79;N,4.77.

### Example 179

2-(1-{2-[(4S,6R)-8-Chloro-6-(2,3-dihydro-1,4-benzodioxin-5-yl)-4H,6H-pyrrolo[1,2-a][4,1]benzoxazepin-4-yl]acetyl}-4-piperidinyl)acetic acid

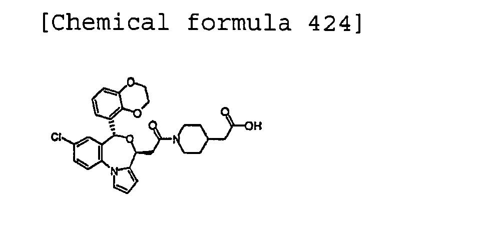

The title compound (14.2 mg) was obtained from ethyl 2-(1-{2-[(4S,6R)-8-chloro-6-(2,3-dihydro-1,4-benzodioxin-5-yl)-4H,6H-pyrrolo[1,2-a][4,1]benzoxazepin-4-yl]acetyl}-4-piperidinyl)acetate (19.4 mg) by using potassium carbonate (14.2 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid. MS(ESI)m/z:537(M⁺+H).
¹H-NMR(CDCl₃) δ:1.06-1.24 (1H,m), 1.64-1.87 (2H,m),1.94-2.10(1H,m),2.14-2.37(2H,m),2.78-2.92(1H,m),2.96-3.14(1H,m),3.21-3.32(1H,m),3.38-3.61(1H,m),3.96-4.17(5H,m),4.59-4.76(1H,m),4.89-5.01(1H,m),5.67(1H,d,J=12.7Hz),6.25(1H,s),6.37(1H,t,J=3.2Hz ),6.74(1H,dd,J=7.2,2.1Hz),6.85-6.98(2H,m),7.09(1H,s),7.17-7.25(1H,m),7.30-7.39(1H,m).
IR(ATR)cm⁻¹:2926,1724,1599,1473,1282,1080,891,762,717. Optical rotation:[α]_{D}=+150.75°(C=0.0008 g/mL,MeOH)

### Referential Example 78

tert-Butyl 4-chlorophenylcarbamate

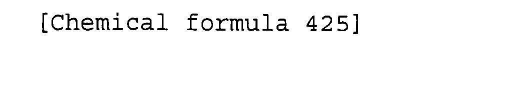

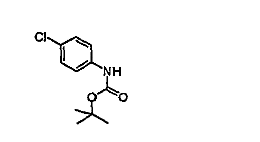

To a tert-butanol solution (200 mL) of 4-chloroaniline (30.1 g) was added di-tert-butyl dicarbonate (77.2 g) at 0°C. The resulting mixture was stirred at room temperature for 2.5 hours. After the reaction mixture was cooled to room temperature, the solvent was concentrated under reduced pressure. To the residue was added hexane (200 ml) and crystals were filtered to give the title compound (45.7 g).
¹H-NMR(CDCl₃)δ:1.51(9H,s),6.45(1H,s),7.24(2H,dd,J=6.8,2.2Hz),7 .28-7.33(2H,m).

### Referential Example 79

tert-Butyl 4-chloro-2-[hydroxy(2-methoxyphenyl)methyl]phenylcarbamate

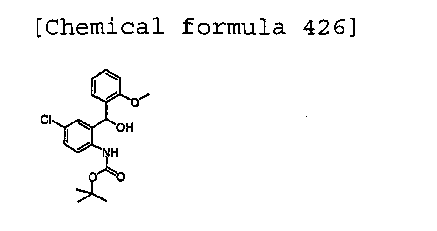

The title compound (4.23 g) was obtained from tert-butyl 4-chlorophenylcarbamate (3.06 g) and 2-methoxybenzaldehyde (2.11 mL) in a similar manner to that employed for the synthesis of tert-butyl 4-chloro-2-[2,3-dihydro-1,4-benzodioxin-5-yl(hydroxy)methyl]phenylcarbamate. MS(ESI)m/z:290(M⁺-tBuOH).
¹H-NMR(CDCl₃)δ:1.49(9H,s),3.34(1H,d,J=4.9Hz),3.88(3H,t,J=7.8Hz ),6.05-6.09(1H,m),6.94-7.01(2H,m),7.05-7.08(1H,m),7.17-7.24(2H,m),7.30-7.35(1H,m),7.92(1H,d,J=8.8Hz),8.07(1H,s).

### Referential Example 80

tert-Butyl 4-chloro-2-(2-methoxybenzoyl)phenylcarbamate

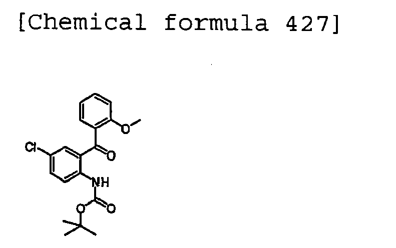

The title compound (4.24 g) was obtained from tert-butyl 4-chloro-2-[hydroxy(2-methoxyphenyl)methyl]phenylcarbamate (4.20 g) by using manganese dioxide (10.0 g) in a similar manner to that employed for the synthesis of 5-chloro-2-(1H-pyrrol-1-yl)-3-pyridinyl](2,3-dimethoxyphenyl)methanone.
MS (ESI) m/z: 290 (M⁺-tBoc).
¹H-NMR(CDCl₃)δ:1.53(9H,s),3.76(3H,s),6.99-7.09(2H,m),7.27-7.30(1H,m),7.36(1H,d,J=2.9Hz),7.43-7.53(2H,m),8.48(1H,d,J=8.8Hz),10.71(1H,s).
IR (ATR) cm⁻
¹:3296,2979,1732,1639,1576,1508,1238,1147,1022,833,752,642.

### Referential Example 81

(2-Amino-5-chlorophenyl)(2-methoxyphenyl)methanone

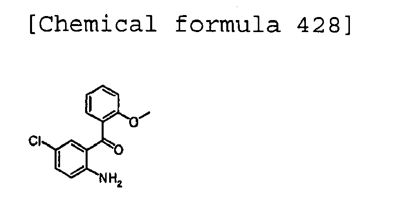

The title compound (3.07 g) was obtained from tert-butyl 4-chloro-2-(2-methoxybenzoyl)phenylcarbamate (4.00 g) by using trifluoroacetic acid (8.52 mL) in accordance with J. Org. Chem., 55(4), 1379-1390(1990).
MS (ESI) m/z : 262 (M⁺+H) .
¹H-NMR(CDCl₃)δ:3.78(3H,s),6.37(2H,br s),6.65(1H,d,J=8.8Hz),6.97-7.07(2H,m),7.18-7.26(3H,m),7.42-7.47 (1H,m) .
IR(ATR)cm⁻
¹:3469,3340,1616,1577,153,1462,1230,1149,1020,947,820,750.

### Referential Example 82

[5-Chloro-2-(1H-pyrrol-1-yl)phenyl](2-methoxyphenyl)methanone

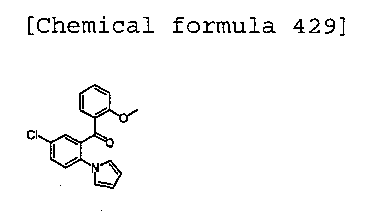

The title compound (5.29 g) was obtained from (2-amino-5-chlorophenyl)(2-methoxyphenyl)methanone (5.57 g) by using 2,5-dimethoxytetrahydrofuran (2.95 mL) in a similar manner to that employed for the synthesis of (5-chloro-2-(1H-pyrrol-1-yl)phenyl)(2,3-dimethoxyphenyl)methanone.
MS (ESI) m/z: 312 (M⁺+H) .
¹H-NMR(CDCl₃)δ:3.62(3H,s),6.02(2H,t,J=2.2Hz),6.65(2H,t,J=2.2Hz ),6.73-6.79(1H,m),6.84-6.90(1H,m),6.98-7.25(2H,m),7.27-7.42(2H,m),7.42-7.60(3H,m).
IR(ATR)cm⁻¹:1637,1595,1481,1296,1242,1155,1016,953,756,721.

### Referential Example 83

1-[4-Chloro-2-(2-methoxybenzoyl)phenyl]-1H-pyrrol-2-carbaldehyde

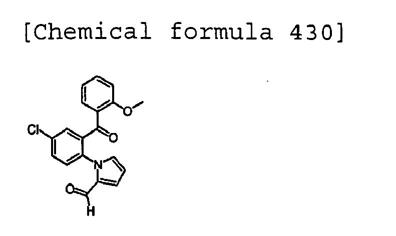

The title compound (781 mg) was obtained from [5-chloro-2-(1H-pyrrol-1-yl)phenyl](2-methoxyphenyl)methanone (717 mg) by using phosphorus oxychloride (707 µl) and N,N-dimethylformamide (748 µl) in a similar manner to that employed for the synthesis of 1-(4-chloro-2-(2,3-dimethoxybenzoyl)phenyl)-1H-pyrrolo-2-carbaldehyde and 1-(4-chloro-2-(2,3-dimethoxybenzoyl)phenyl)-1H-pyrrolo-3-carbaldehyde.
MS(ESI)m/z:340(M⁺+H) .
¹H-NMR(CDCl₃)δ:3.63 (3H,s),6.16(1H,dd,J=3.9,2.9Hz),6.72-6.87(4H,m),7.27-7.37(3H,m),7.52(1H,dd,J=8.3,2.5Hz),7.59(1H,d,J=2.0Hz),9.34( 1H,s).
IR(ATR)cm⁻¹:1655,1599,1483,1389,1294,1244,1093,1024,748.

### Referential Example 84

Methyl (E)-3-{1- [4-chloro-2-(2-methoxybenzoyl)phenyl]-1H-pyrrol-2-yl}-2-propenoate

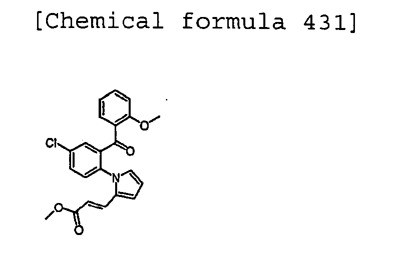

The title compound (1.07 g) was obtained from 1-[4-chloro-2-(2-methoxybenzoyl)phenyl]-1H-pyrrol-2-carbaldehyde (961 mg) by using methyl (triphenylphosphoranylidene)acetate (1.80 g) in a similar manner to that employed for the synthesis of methyl (E)-3-(1-(4-chloro-2-(2,3-dimethoxybenzoyl)phenyl)-1H-pyrrol-2-yl)-2-propenoate.
MS(ESI)m/z:396(M⁺+H).
¹H-NMR(CDCl₃):1.55(9H,s),3.57(3H,s),3.72(3H,s),5.93(1H,d,J=15 .7Hz),6.03-6.06(1H,m),6.44(1H,dd,J=3.9,1.2Hz),6.67-6.71(2H,m),6.82(1H,td,J=7.5,0.9Hz),7.15-7.21(2H,m),7.27-7.36(2H,m),7.54(1H,dd,J=8.3,2.5Hz),7.62(1H,d,J=2.5Hz).
IR(ATR)cm⁻
¹:1701,1653,1626,1545,1483,1435,1296,1246,1174,741.

### Referential Example 85

Methyl (E)-3-(1-{4-chloro-2-[hydroxy(2-methoxyphenyl)methyl]phenyl}-1H-pyrrol-2-yl)-2-propenoate

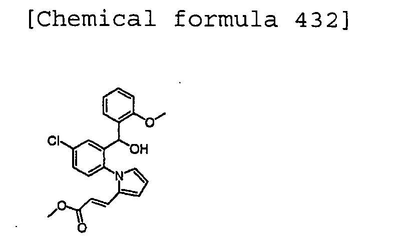

The title compound (2.55 g) was obtained as a mixture of atropisomers from methyl (E)-3-{1-[4-chloro-2-(2-methoxybenzoyl)phenyl]-1H-pyrrol-2-yl}-2-propenoate (2.41 g) by using sodium borohydride (375 mg) in a similar manner to that employed for the synthesis of methyl (E)-3-(1-(4-chloro-2-((2,3-dimethoxyphenyl)(hydroxy)methyl)phenyl)-1H-pyrrol-2-yl)-2-propenoate.
MS (ESI) m/z :380 (M⁺-OH) .
¹H-NMR(CDCl₃) δ:2.60 and 2.68(1H,d,J=5.9Hz),3.62 and 3.68(3H,s),3.65 and 3.70(3H,s),5.70 and 5.91(1H,d,J=15.7Hz),5.75-5.82(1H,m),6.14-6.18 and 6.34-6.38(1H,m),6.64-6.91(4H,m),6.96-7.25(3H,m),7.31-7.39(1H,m),7.59 and 7.72(1H,d,J=2.5Hz).
IR(ATR)cm⁻¹: 3423,1695,1622,1487,452,1240,1169,1026,831,752,725.

### Example 180

Methyl 2-[8-chloro-6-(2-methoxyphenyl)-4H,6H-pyrrolo[1,2-a] [4,1]benzoxazepin-4-yl]acetate

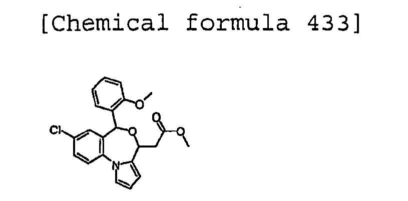

The title compound (371 mg) was obtained from methyl (E)-3-(1-{4-chloro-2-[hydroxy(2-methoxyphenyl)methyl]phenyl}-1H-pyrrol-2-yl)-2-propenoate (1.23 g) by using trifluoroacetic acid (286 µl) in a similar manner to that employed for the synthesis of methyl 2-(8-chloro-6-(2,3-dimethoxyphenyl)-4H,6H-pyrrolo(1,2-a)(4,1)benzoxazepin-4-yl)acetate.
MS(ESI)m/z:398(M⁺+H).
¹H-NMR(CDCl₃)δ:2.98-3.13(2H,m),3.55(3H,s),3.72(3H,s),4.88-4.93(1H,m),5.71(1H,s),6.27-6.31(1H,m),6.37(1H,t,J=3.2Hz),6.69(1H,d,J=2.2Hz),6.83(1H,d, J=7.8Hz),6.97-7.12(2H,m),7.29-7.39(3H,m),7.66(1H,dd,J=7.6,1.7Hz).
IR(ATR)cm⁻
¹:2951,1738,1489,1433,1244,1161,1047,1024,833,756,727.

Example 181 2-[8-Chloro-6-(2-methoxyphenyl)-4H,6H-pyrrolo[1,2-a][4,1]benzoxazepin-4-yl]acetic acid

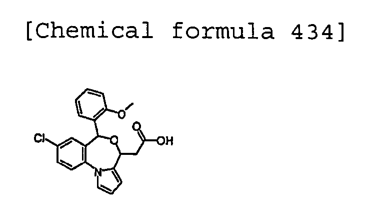

The title compound (419 mg) was obtained from methyl 2-[8-chloro-6-(2-methoxyphenyl)-4H,6H-pyrrolo[1,2-a] [4,1]benzoxazepin-4-yl]acetate (617 mg) by using a 1N aqueous sodium hydroxide solution (1.2 mL) in a similar manner to that employed for the synthesis of 2-(8-chloro-6-(2,3-dimethoxyphenyl)-4H,6H-pyrrolo(1,2-a)(4,1)benzoxazepin-4-yl)acetic acid.
¹H-NMR(CDCl₃)δ:2.98-3.19(2H,m), ,3.61(3H,s),4.86-4.93(1H,m),5.66-5.83(1H,m),6.27-6.41(2H,m),6.65-6.92(3H,m),6.96-7.24(2H,m),7.29-7.42(2H,m),7.58-7.74(1H,m).

### Example 182

Ethyl 2-(1-{2-[8-chloro-6-(2-methoxyphenyl)-4H,6H-pyrrolo[1,2-a][4,1]benzoxazepin-4-yl]acetyl}-4-piperidinyl)acetate

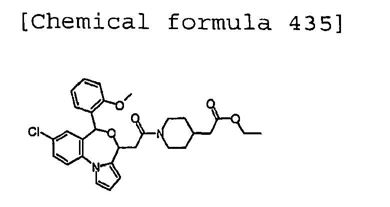

The title compound (135 mg) was obtained from 2-[8-chloro-6-(2-methoxyphenyl)-4H,6H-pyrrolo[1,2-a] [4,1]benzoxazepin-4-yl]acetic acid (419 mg) by using ethyl piperidine-4-acetate (34 mg), 1-ethyl-3-(3-dimethylaminopropyl)carbodiimide hydrochloride (313 mg), and 1-hydroxybenzotriazole (192 mg) in a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate.
MS (ESI) m/z : 537 (M⁺+H). ¹H-NMR(CDCl₃)δ:1.01-1.23(1H,m),1.26(3H,t,J=7.1Hz),1.49-1.84(2H,m),1.90-2.08(1H,m),2.09-2.30(2H,m),2.41-2.73(1H,m),2.77-2.92(1H,m),3.22-3.32(1H,m),3.46-3.59(3H,m),4.00-4.09(1H,m),4.09-4.17(2H,m),4.59-4.76(1H,m),4.91-5.03(1H,m),5.70-5.86(1H,m),6.21-6.41(2H,m),6.63-6.69(1H,m),6.77-6.87(1H,m),6.94-7.18(2H,m),7.30-7.41(3H,m),7.51-7.81(1H,m).
IR (ATR) cm⁻¹ : 2935,1728,1630,1489,1286,1244,1161,1086,1026,748.

### Example 183

Ethyl 2-(1-{2-[(4R,6S)-8-chloro-6-(2-methoxyphenyl)-4H,6H-pyrrolo[1,2-a][4,1]benzoxazepin-4-yl]acetyl}-4-piperidinyl)acetate Ethyl 2-(1-{2-[(4S,6R)-8-chloro-6-(2-methoxyphenyl)-4H,6H-pyrrolo[1,2-a][4,1]benzoxazepin-4-yl]acetyl}-4-piperidinyl)acetate

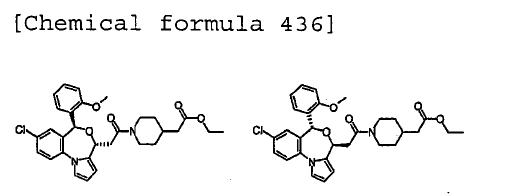

Ethyl 2-(1-{2-[8-chloro-6-(2-methoxyphenyl)-4H,6H-pyrrolo[1,2-a][4,1]benzoxazepin-4-yl]acetyl}-4-piperidinyl)acetate (135 mg) was separated into cis and trans isomers by CHIRALCEL OD while using a 20% isopropanol-hexane solution at a flow rate of 10 ml/min. Then, by CHIRALPAK AD using a 20% isopropanol-hexane at a flow rate of 10 ml/min, ethyl 2-(1-{2-[(4R,6S)-8-chloro-6-(2-methoxyphenyl)-4H,6H-pyrrolo[1,2-a][4,1]benzoxazepin-4-yl]acetyl}-4-piperidinyl)acetate (36 mg) corresponding to a peak with a longer retention time and 2-(1-{2-[(4S,6R)-8-chloro-6-(2-methoxyphenyl)-4H,6H-pyrrolo[1,2-a] [4,1]benzoxazepin-4-yl]acetyl}-4-piperidinyl)acetate (47 mg) corresponding to a peak with a shorter retention time were obtained.

### Example 184

2-(1-{2-[(4R,6S)-8-Chloro-6-(2-methoxyphenyl)-4H,6H-pyrrolo[1,2-a][4,1]benzoxazepin-4-yl]acetyl}-4-piperidinyl)acetic acid

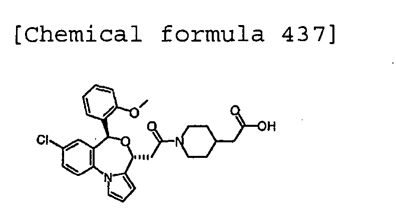

The title compound (34.4 mg) was obtained from ethyl 2-(1-{2-[(4R,6S)-8-chloro-6-(2-methoxyphenyl)-4H,6H-pyrrolo[1,2-a][4,1]benzoxazepin-4-yl]acetyl}-4-piperidinyl)acetate (36.4 mg) by using potassium carbonate (28.1 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid.
MS(ESI)m/z:491(M⁺-OH).
¹H-NMR(CDCl₃)δ:1.02-1.32(1H,m),1.61-1.87(2H,m),1.92-2.08(1H,m),2.12-2.23(1H,m),2.23-2.33(1H,m),2.54-2.72(1H,m),2.77-2.92(1H,m),2.95-3.14(1H,m),3.23-3.34(1H,m),3.56 and 3.57(3H,s),4.01-4.11(1H,m),4.63-4.77(1H,m),4.91-5.03(1H,m),5.71 and 5.75(1H,s),6.23-6.26(1H,m),6.37(1H,t,J=3.3Hz),6.65(1H,dd,J=7.8,2.0Hz),6.84( 1H,d,J=8.3Hz),7.03-7.12(2H,m),7.29-7.38(3H,m),7.61-7.71(1H,m).
IR(ATR)cm⁻¹:2929,2846,1601,1489,1244,1169,1092,1049,1026.
Elemental analysis for: C₂₈H₂₉ClN₂O₅·0.25H₂O
Calculated: C,65.46;H,5.79;N,5.46.
Found: C,65.27;H,5.95;N,5.27.

### Example 185

2-(1-{2-[(4S,6R)-8-Chloro-6-(2-methoxyphenyl)-4H,6H-pyrrolo[1,2-a][4,1]benzoxazepin-4-yl]acetyl}-4-piperidinyl)acetic acid

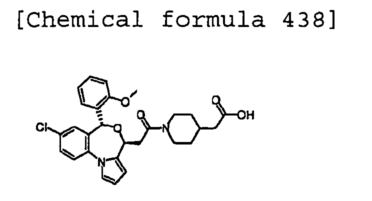

The title compound (38.4 mg) was obtained from ethyl 2-(1-{2-[(4S,6R)-8-chloro-6-(2-methoxyphenyl)-4H,6H-pyrrolo[1,2-a][4,1]benzoxazepin-4-yl]acetyl}-4-piperidinyl)acetate (46.8 mg) by using potassium carbonate (36.1 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid.
MS (ESI) m/z : 509 (M++H) . ¹H-NMR(CDCl₃)δ:1.06-1.32(2H,M),1.61.86(2H,m),1.94-2.09(1H,m),2.17-2.33(2H,m),2.53-2.73(1H,m),2.76-2.91(1H,m),2.95-3.14(1H,m),3.22-3.34(1H,m),3.56 and 3.57(3H,s),4.02-4.11(1H,m),4.63-4.78(1H,m),4.91-5.02(1H,m),5.71 and 5.75(1H,s),6.22-6.26(1H,m),6.37(1H,t,J=3.2Hz),6.63-6.68(1H,m),6.82-6.86(1H,m).,7.03-7.11(2H,m),7.30-7.37(3H,m),7.61-7.71(1H,m).
Elemental analysis for: C₂₈H₂₉ClN₂O₅·1.25H₂O
Calculated: C,63.27;H,5.97;N,5.27.
Found: C,63.27;H,5.79;N,4.87.

### Referential Example 86

3,4-Dimethoxypyridine-2-carbaldehyde

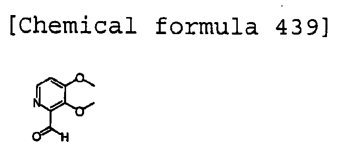

To a methanol solution (30 mL) of 2-chloromethyl-3,4-dimethoxypyridine (3.00 g) were added 2-nitropropane (1.58 mL) and metallic sodium (0.38 g) at 0°C. The resulting mixture was stirred for 17 hours while warming to room temperature. The temperature of the reaction mixture was raised to 70°C and stirring was performed for further 8.5 hours. After cooling to room temperature, the solvent was concentrated under reduced pressure. Diethyl ether and a 1N aqueous sodium hydroxide solution were added to the residue thus obtained and the layers separated. The organic layer was washed with saturated brine and dried over anhydrous sodium sulfate. The solvent was distilled off under reduced pressure. The residue thus obtained was subjected to silica gel column chromatography (dichloromethane:methanol=20:1) to give the title compound (1.41 g).
¹H-NMR(CDCl₃)δ:3.97(3H,s),4.00(3H,s),7.01(1H,d,J=5.4Hz),8.43(1 H,d,J=5.4Hz),10.34(1H,s).

### Referential Example 87

[5-Chloro-2(1H-pyrrol-1-yl)phenyl](3,4-dimethoxy-2-pyridinyl)methanol

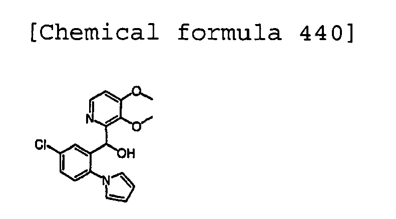

The title compound (1.39 g) was obtained from 1-(2-bromo-4-chlorophenyl)-1H-pyrrole (3.06 g) and 3,4-dimethoxypyridine-2-carbaldehyde (1.68 g) by using normal-butyl lithium (2.66 mol/l, 3.15 mL) in a similar manner to that employed for the synthesis of (5-chloro-3-fluoro-2-pyrrol-1-ylphenyl)-(2,3-dimethoxyphenyl)methanol.
MS(ESI)m/z:345(M⁺+H).
¹H-NMR(CDCl₃)δ:3.34(3H,s),3.88(3H,s),5.62(1H,d,J=5.4Hz),5.75(1 H,d,J=5.4Hz),6.34-6.36(2H,m),6.86(1H,d,J=5.6Hz),6.98-7.00(1H,m),7.02-7.05(2H,m),7.24-7.26(3H,m),8.25(1H,d,J=5.4Hz).
IR (ATR) cm⁻
¹:3294,1585,1489,1427,1290,1228,1103,1036,993,822,725.

### Referential Example 88

[5-Chloro-2(1H-pyrrol-1-yl)phenyl](3,4-dimethoxy-2-pyridinyl)methanone

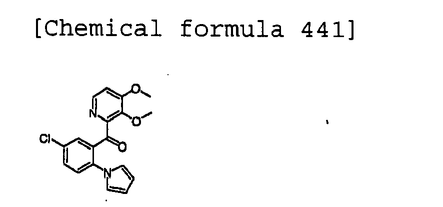

The title compound (1.40 g) was obtained from [5-chloro-2(1H-pyrrol-1-yl)phenyl](3,4-dimethoxy-2-pyridinyl)methanol (1.48 g) by using manganese dioxide (3.72 g) in a similar manner to that employed for the synthesis of [5-chloro-2-(1H-pyrrol-1-yl)-3-pyridinyl](2,3-dimethoxyphenyl)methanone.
¹H-NMR(CDCl₃)δ:3.86(3H,s),3.88(3H,s),5.97(2H,t,J=2.2Hz),6.65(2 H,t,J=2.1Hz),6.75(1H,d,J=5.4Hz),7.29(1H,d,J=8.6Hz),7.53(1H, dd,J=8.6,2.5Hz),7.71(1H,d,J=2.5Hz),8.00(1H,d,J=5.1Hz).

### Referential Example 89

1-{[4-Chloro-2-[(3,4-dimethoxy-2-pyridinyl)carbonyl]phenyl]-1H-pyrrol-2-carbaldehyde

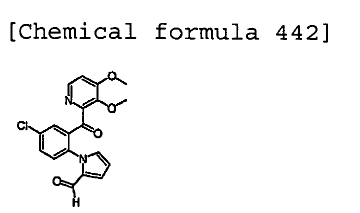

The title compound (1.53 g) was obtained from [5-chloro-2(1H-pyrrol-1-yl)phenyl](3,4-dimethoxy-2-pyridinyl)methanone (3.30 g) by using phosphorus oxychloride (1.08 mL) and N,N-dimethylformamide (1.49 mL) in a similar manner to that employed for the synthesis of 1-(4-chloro-2-(2,3-dimethoxybenzoyl)phenyl)-1H-pyrrolo-2-carbaldehyde and 1-(4-chloro-2-(2,3-dimethoxybenzoyl)phenyl)-1H-pyrrolo-3-carbaldehyde. ¹H-NMR(CDCl₃)δ:3.83(3H,s),3.88(3H,s),6.07-6.09(1H,m),6.74-6.81(3H,m),7.28(4H,d,J=8.6Hz),7.57(1H,dd,J=8.3,2.5Hz),7.79( 1H,d,J=2.5Hz),8.00(1H,d,J=5.4Hz),9.41(1H,s).
IR(ATR)cm⁻¹:1684,1655,1489,1415,1302,1072,984,825,764,553.

### Referential Example 90

Methyl (E)-3-(1-{4-chloro-2-[(3,4-dimethoxy-2-pyridinyl)carbonyl]phenyl}-1H-pyrrol-2-yl)-2-propenoate

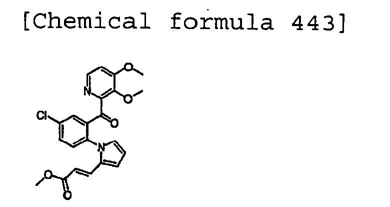

The title compound (1.43 g) was obtained from 1-{[4-chloro-2-[(3,4-dimethoxy-2-pyridinyl)carbonyl]phenyl]-1H-pyrrol-2-carbaldehyde (1.51 g) by using methyl(triphenylphosphoranylidene)acetate (2.72 g) in a similar manner to that employed for the synthesis of methyl (E)-3-(1-(4-chloro-2-(2,3-dimethoxybenzoyl)phenyl)-1H-pyrrol-2-yl)-2-propenoate.
MS(ESI)m/z:427(M⁺+H).
¹H-NMR(CDCl₃)δ:3.73(3H, s) 3.84(3H, s),3.86(3H,s),5. 92 (1H,d,J=15 .7Hz),6.02-6.05(1H,m),6.38(1H,dd,J=3.9,1.2Hz),6.67-6.74(2H,m),7.15-7.21(1H,m),7.23-7.30(1H,m),7.58(1H,dd,J=8.3,2.5Hz),7.78(1H,d,J=2.5Hz),7.94( 1H,d,J=5.4Hz)
IR(ATR)cm⁻¹ :2945,1682,1624,1574,1483,1304,1236,119,982,820,719.

### Referential Example 91

Methyl (E)-3-(1-{4-chloro-2-[(3,4-dimethoxy-2-pyridinyl)(hydroxy)methyl]phenyl}-1H-pyrrol-2-yl)-2-propenoate

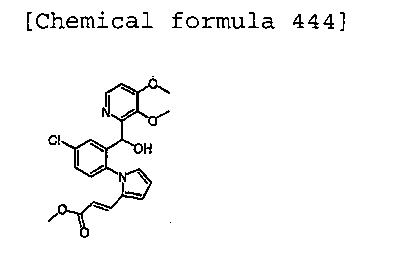

The title compound (1.41 g) was obtained as a mixture of atropisomers from methyl (E)-3-(1-{4-chloro-2-[(3,4-dimethoxy-2-pyridinyl)carbonyl]phenyl}-1H-pyrrol-2-yl)-2-propenoate (1.44 g) by using sodium borohydride (0.19 g) in a similar manner to that employed for the synthesis of methyl (E)-3-(1-(4-chloro-2-((2,3-dimethoxyphenyl)(hydroxy)methyl)phenyl)-1H-pyrrol-2-yl)-2-propenoate.
MS(ESI)m/z:429(M⁺+H).
¹H-NMR(CDCl₃)δ:3.40 and 3.45(3H,s),3.67 and 3.71(3H,s),3.86 and 3.88(3H,s),5.34-5.60(2H,m),5.88 and 6.12(1H,d,J=15.8Hz),6.30-6.43(1H,m),6.68-6.91(2H,m),6.94-7.23(3H,m),7.28-7.35(2H,m),8.12 and 8.23(1H,d,J=5.6Hz).
IR(ATR)cm⁻¹:1699,1624,1585,1489,1290,1242,1169,1032,827,723.

### Referential Example 92

(E)-3-(1-{4-Chloro-2-[(3,4-dimethoxy-2-pyridinyl)(hydroxy)methyl]phenyl}-1H-pyrrol-2-yl)-2-propenoic acid

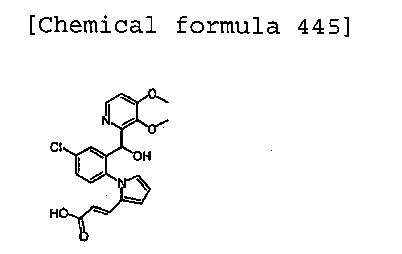

The title compound (1.08 g) was obtained from methyl (E)-3-(1-{4-chloro-2-[(3,4-dimethoxy-2-pyridinyl)(hydroxy)methyl]phenyl}-1H-pyrrol-2-yl)-2-propenoate (1.41 g) by using potassium carbonate (1.36 g) in a similar manner to that employed for the synthesis of 2-(8-chloro-6-(2,3-dimethoxyphenyl)-4H,6H-pyrrolo(1,2-a)(4,1)benzoxazepin-4-yl)acetic acid.
MS(ESI)m/z:415(M⁺+H).
¹H-NMR(CDCl₃)δ:3.42 and 3.49(3H,s),3.85 and 3.88(1H,s),5.53 and 5.80(1H,d,J=29.4Hz),5.89 and 6.07(0H,d,J=15.9Hz),6.30-6.46(1H,m),6.69-6.92(2H,m),6.98-7.22(2H,m),7.29-7.47(2H,m),8.11 and 8.22(1H,d,J=5.6Hz).
IR(ATR)cm⁻
¹:3014,1681,1618,1587,1489,1450,1298,1174,1034,827,746.

### Referential Example 93

Ethyl 2-{1-[(E)-3-(1-{4-chloro-2-[(3,4-dimethoxy-2-pyridinyl)(hydroxy)methyl]phenyl}-1H-pyrrol-2-yl)-2-propenoyl]-4-piperidinyl}acetate

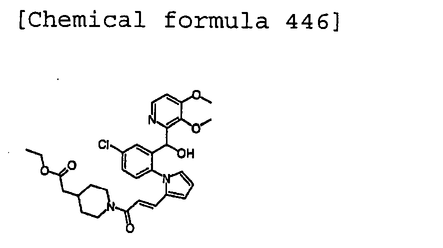

The title compound (184 mg) was obtained as a mixture of atropisomers from (E)-3-(1-{4-chloro-2-[(3,4-dimethoxy-2-pyridinyl)(hydroxy)methyl]phenyl}-1H-pyrrol-2-yl)-2-propenoic acid (200 mg) by using ethyl 2-(4-piperidinyl)acetate (104 mg), 1-ethyl-3-(3-dimethylaminopropyl)carbodiimide hydrochloride (134 mg), and 1-hydroxybenzotriazole (32 mg) in a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate.
MS (ESI)m/z:568 (M⁺+H) .
¹H-NMR(CDCl₃)δ:1.01-1.23(2H,m),1.26(3H,t,J=7.1Hz),1.67-1.81(2H,m),1.92-2.09(1H,m),2.13-2.30(2H,m),3.40 and 3.49(3H,s),3.87 and 3.87(3H,s),4.14(2H,q,J=7.1Hz),5.48-5.84(1H,m),6.06-6.48(2H,m),6.61-6.96(2H,m),7.03-7.44(5H,m),8.16-8.44(1H,m).
IR (ATR) cm⁻
¹:2931,1728,1637,1489,1446,1286;1225,1153,1095,1028,829,717

### Example 186

Ethyl 2-(1-{2-[8-chloro-6-(3,4-dimethoxy-2-pyridinyl)-4H,6H-pyrrolo[1,2-a][4,1]benzoxazepin-4-yl]acetyl}-4-piperidinyl)acetate

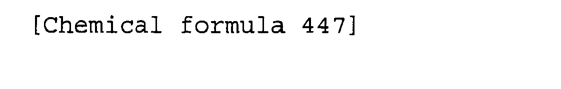

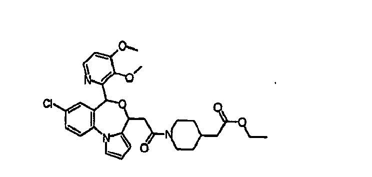

The title compound (371 mg) was obtained as a cis-trans mixture (having a cis isomer as a main product) from ethyl 2-{1-[(E)-3-(1-{4-chloro-2-[(3,4-dimethoxy-2-pyridinyl)(hydroxy)methyl]phenyl}-1H-pyrrol-2-yl)-2-propenoyl]-4-piperidinyl}acetate (1.23 g) by using trifluoroacetic acid (286 µl) in a similar manner to that employed for the synthesis of methyl 2-(8-chloro-6-(2,3-dimethoxyphenyl)-4H,6H-pyrrolo(1,2-a)(4,1)benzoxazepin-4-yl)acetate.
MS (ESI) m/z : 568 (M⁺+H).
¹H-NMR(CDCl₃)δ:1.05-1.23(2H,m),1.26(3H,t,J=7.2Hz),1.56-1.86(3H,m),1.92-2.11(1H,m),2.16-2.31(2H,m),3.40 and 3.48(3H,s),3.86-3.90(1H,m),3.87 and 3.87(3H,s),4.14(2H,q,J=7.1Hz),4.46-4.78(2H,m),5.15-5.80(1H,m),5.52 and 5.60(1H,s),6.05-6.14(1H,m),6.29-6.39(1H,m),6.39-6.46 and 7.35-7.42(1H,m),6.59-6.79(1H,m),6.81(1H,d,J=5.4Hz),6.86(1H,d,J=5.6Hz),6.91-6.97(1H,m),7.10-7.20(1H,m),7.20-7.32(2H,m),8.18 and 8.24(10H,d,J=5.4Hz).
IR (ATR) cm⁻
¹:2935,1728,1637,1585,1489,1446,1410,1284,1225,1153,1028,72 5.

### Example 187

Ethyl 2-(1-{2-[(4R,6R)-8-chloro-6-(3,4-dimethoxy-2-pyridinyl)-4H,6H-pyrrolo[1,2-a][4,1]benzoxazepin-4-yl]acetyl}-4-piperidinyl)acetate Ethyl 2-(1-{2-[(4S,6S)-8-chloro-6-(3,4-dimethoxy-2-pyridinyl)-4H,6H-pyrrolo[1,2-a][4,1]benzoxazepin-4-yl]acetyl}-4-piperidinyl)acetate

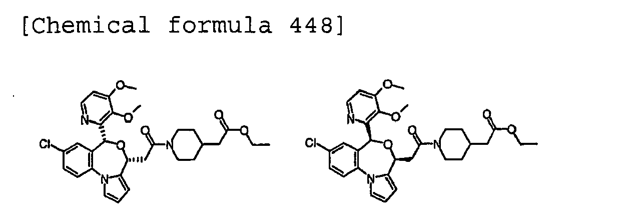

Ethyl 2-(1-{2-[8-chloro-6-(3,4-dimethoxy-2-pyridinyl)-4H,6H-pyrrolo[1,2-a][4,1]benzoxazepin-4-yl]acetyl}-4-piperidinyl)acetate (88 mg) was subjected to CHIRALPAK AD using a 20% isopropanol-hexane solution at a flow rate of 10 mL/min to give ethyl 2-(1-{2-[(4R,6R)-8-chloro-6-(3,4-dimethoxy-2-pyridinyl)-4H,6H-pyrrolo[1,2-a][4,1]benzoxazepin-4-yl]acetyl}-4-piperidinyl)acetate (27 mg) corresponding to a peak with a shorter retention time and ethyl 2-(1-{2-[(4S,6S)-8-chloro-6-(3,4-dimethoxy-2-pyridinyl)-4H,6H-pyrrolo[1,2-a][4,1]benzoxazepin-4-yl]acetyl}-4-piperidinyl)acetate (26 mg) corresponding to a peak with a longer retention time.

### Example 188

2-(1-{2-[(4R,6R)-8-Chloro-6-(3,4-dimethoxy-2-pyridinyl)-4H,6H-pyrrolo[1,2-a][4,1]benzoxazepin-4-yl]acetyl}-4-piperidinyl)acetic acid

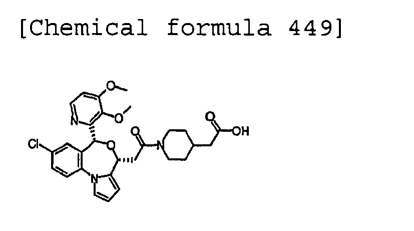

The title compound (21 mg) was obtained from ethyl 2-(1-{2-[(4R,6R)-8-chloro-6-(3,4-dimethoxy-2-pyridinyl)-4H,6H-pyrrolo[1,2-a][4,1]benzoxazepin-4-yl]acetyl}-4-piperidinyl)acetate (26 mg) by using potassium carbonate (20 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid.
MS (ESI) m/z : 540 (M⁺+H).
¹H-NMR(CDCl₃)δ:0.84-1.36(3H,m),1.59-1.89(2H,m),1.92-2.11(1H,m),2.19-2.34(2H,m),2.48-2.79(1H,m),2.80-3.10(1H,m),3.42 and 3.50(3H,s),3.86-3.92(1H,m),3.87 and 3.89(3H,s),4.48-4.82(1H,m),5.45-5.75(1H,m),6.03-6.44(2H,m),6.55-6.78(1H,m),6.81-7.06(2H,m),7.09-7.45(2H,m),8.19 and 8.23(1H, d, J=5.4Hz).
IR(ATR)cm⁻
¹:2931,1720,1633,1585,1489,1286,1286,1225,1032,829,723.

### Example 189

2-(1-{2-[(4S,6S)-8-Chloro-6-(3,4-dimethoxy-2-pyridinyl)-4H,6H-pyrrolo[1,2-a][4,1]benzoxazepin-4-yl]acetyl}-4-piperidinyl)acetic acid

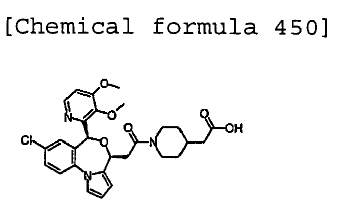

The title compound (18 mg) was obtained from ethyl 2-(1-{2-[(4S,6S)-8-chloro-6-(3,4-dimethoxy-2-pyridinyl)-4H,6H-pyrrolo[1,2-a][4,1]benzoxazepin-4-yl]acetyl}-4-piperidinyl)acetate (26 mg) by using potassium carbonate (19 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid.
MS(ESI)m/z:540(M⁺+H).
¹H-NMR(CDCl₃)δ:0.82-0.94(1H,m), 1.00-1.35(4H,m) 1.59-1.87(2H,m),1.91-2.09(1H,m),2.14-2.34(2H,m),2.45-2.78(1H,m),2.78-3.10(1H,m),3.41-3.54(3H,m),3.86-3.94(4H,m),4.44-4.78(1H,m),5.47-5.74(1H,m),6.02-6.41(2H,m),6.52-6.76(1H,m),6.80-7.05(2H,m),7.07-7.39(2H,m),8.18-8.25(1H,m).

### Example 190

trans-Methyl 2-[8-chloro-6-(3,4-dimethoxy-2-pyridinyl)-4H, 6H-pyrrolo [1, 2-a] [4, 1] benzoxazepin-4-yl] acetate cis-Methyl 2-[8-chloro-6-(3,4-dimethoxy-2-pyridinyl)-4H,6H-pyrrolo [1, 2-a] [4, 1] benzoxazepin-4-yl] acetate

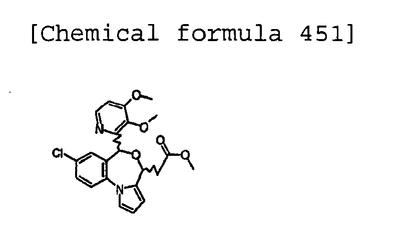

The title compounds, that is, trans isomer (185 mg) and cis isomer (201 mg) were obtained from methyl (E)-3-(1-{4-chloro-2-[(3,4-dimethoxy-2-pyridinyl)(hydroxy)methyl]phenyl}-1H-pyrrol-2-yl)-2-propenoate (620 mg) and trifluoroacetic acid (167 µl) in a similar manner to that employed for the synthesis of methyl 2-(8-chloro-6-(2,3-dimethoxyphenyl)-4H,6H-pyrrolo(1,2-a)(4,1)benzoxazepin-4-yl)acetate. trans Isomer
¹H-NMR(CDCl₃)δ:3.11-3.16(2H,m),3.55(3H,s),3.62(3H,s),3.92(3H,s),4.99-5.05(1H,m),5.78(1H,s),6.31-6.37(2H,m),6.89(1H,d,J=5.4Hz),6.93-6.95(1H,m),7.06-7.09(1H,m),7.34-7.43(2H,m),8.42(1H,d,J=5.6Hz). cis Isomer
¹H-NMR(CDCl₃)δ:2.70-2.77(1H,m),2.92-3.00(1H,m),3.52(3H,s),3.65(3H,s),3.86(3H,s),5.34-5.42(1H,m),6.17-6.26(3H,m),6.77(1H,d,J=5.6Hz),7.05-7.14(2H,m),7.32(1H,dd,J=8.6,2.2Hz),7.38(1H,d,J=8.6Hz),8.19( 1H,d,J=5.4Hz).

### Example 191

trans-2-[8-Chloro-6-(3,4-dimethoxy-2-pyridinyl)-4H,6H-pyrrolo[1,2-a][4,1]benzoxazepin-4-yl]acetic acid

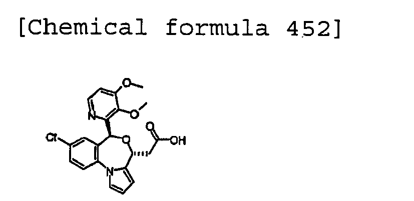

The title compound (20 mg) was obtained from trans-methyl 2-[8-chloro-6-(3,4-dimethoxy-2-pyridinyl)-4H,6H-pyrrolo[1,2-a][4,1]benzoxazepin-4-yl]acetate (46 mg) by using potassium carbonate (44 mg) in a similar manner to that employed for the synthesis of 2-(8-chloro-6-(2,3-dimethoxyphenyl)-4H,6H-pyrrolo(1,2-a)(4,1)benzoxazepin-4-yl)acetic acid.
¹H-NMR(CDCl₃)δ:2.35-2.77(1H,m),2.90-3.24(1H,m),3.50(3H,s),3.90(3H,s),4.78-5.09(1H,m),5.83(1H,s),6.27-6.44(2H,m),6.61-6.86(2H,m),7.10(1H,s),7.30-7.48(2H,m),7.94-8.27(1H,m).

### Example 192

cis-2-[8-Chloro-6-(3,4-dimethoxy-2-pyridinyl)-4H,6H-pyrrolo[1,2-a][4,1]benzoxazepin-4-yl]acetic acid

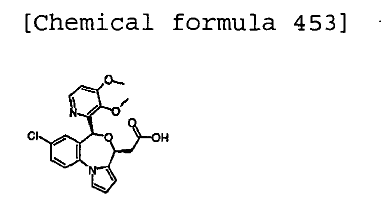

The title compound (20 mg) was obtained from cis-methyl 2-[8-chloro-6-(3,4-dimethoxy-2-pyridinyl)-4H,6H-pyrrolo[1,2-a][4,1]benzoxazepin-4-yl]acetate (46 mg) by using potassium carbonate (44 mg) in a similar manner to that employed for the synthesis of 2-(8-chloro-6-(2,3-dimethoxyphenyl)-4H,6H-pyrrolo(1,2-a)(4,1)benzoxazepin-4-yl)acetic acid.
¹H-NMR(CDCl₃)δ:3.15(3H,s)3.18-3.48(2H,m)3.86-3.91(1H,m),3.87(3H,s),4.80-4.85(1H,m),6.04-6.14(2H,m),6.29-6.43(1H,m),6.59-6.72(1H,m),6.73-6.92(1H,m),7.44-7.53(1H,m),7.65-7.69(1H,m),7.96-8.27(1H,m).

### Referential Example 94

3-Fluoro-2-methoxybenzaldehyde

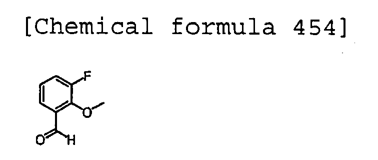

To an N,N-dimethylformamide solution (150 mL) of 3-fluoro-2-hydroxybenzaldehyde (3.33 g) were added iodomethane (2.22 mL) and potassium carbonate (9.87 g) at room temperature. The resulting mixture was stirred at 50°C for 2 hours. After cooling to room temperature, water and diethyl ether were added to the mixture and the layers separated. The water layer was washed three times with diethyl ether. The organic layers were combined. The solvent was distilled off under reduced pressure to give the title compound (3.55 g).
¹H-NMR (CDCl₃) δ:4.10 and 4.11(3H,s),7.06-7.13(1H,m),7.30-7.37(1H,m),7.59-7.64(1H,m),10.41 and 10.41(1H,s).

### Referential Example 95

[5-Chloro-2(1H-pyrrol-1-yl)phenyl](3-fluoro-2-methoxyphenyl)methanol

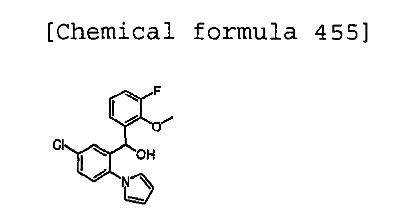

The title compound (3.51 g) was obtained from 1-(2-bromo-4-chlorophenyl)-1H-pyrrole (5.31 g) and 3-fluoro-2-methoxybenzaldehyde (3.55 g) by using normal-butyl lithium (1.6 mol/l, 14.4 mL) in a similar manner to that employed for the synthesis of (5-chloro-3-fluoro-2-pyrrol-1-ylphenyl)-(2,3-dimethoxyphenyl)methanol.
¹H-NMR(CDCl₃)δ:2.56(1H,d,J=4.7Hz),3.67 and 3.68(3H,s),5.96(1H,d,J=4.7Hz),6.25-6.27(2H,m),6.68-6.69(2H,m),6.80-7.14(3H,m),7.23(1H,d,J=8.3Hz),7.34(1H,dd,J=8.3,2.5Hz),7.50( 1H,d,J=2.5Hz).

### Referential Example 96

[5-Chloro-2-(1H-pyrrol-1-yl)phenyl](3-fluoro-2-methoxyphenyl)methanone

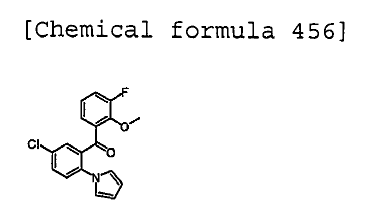

The title compound (3.59 g) was obtained from [5-chloro-2(1H-pyrrol-1-yl)phenyl](3-fluoro-2-methoxyphenyl)methanol (4.56 g) by using manganese dioxide (6.79 g) in a similar manner to that employed for the synthesis of [5-chloro-2-(1H-pyrrol-1-yl)-3-pyridinyl](2,3-dimethoxyphenyl)methanone.
MS (ESI)m/z:330 (M⁺+H) .
¹H-NMR(CDCl₃)δ:3.66(3H,d,J=2.5Hz),6.03-6.06(2H,m),6.65-6.69(2H,m),6.86-6.95(1H,m),7.08-7.17(2H,m),7.30-7.34(1H,m),7.50-7.56(2H,m).
IR(ATR)cm⁻¹:1666,1477,1427,1263,1068,999,723.

### Referential Example 97

1-[4-Chloro-2-(3-fluoro-2-methoxybenzoyl)phenyl]-1H-pyrrol-2-carbaldehyde

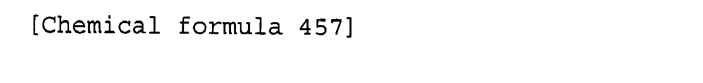

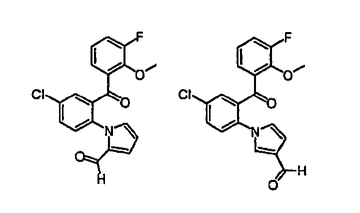

The title compound (2.04 g) was obtained from [5-chloro-2-(1H-pyrrol-1-yl)phenyl](3-fluoro-2-methoxyphenyl)methanone (3.58 g) by using phosphorus oxychloride (1.21 mL) and N,N-dimethylformamide, (1.68 mL) in a similar manner to that employed for the synthesis of 1-(4-chloro-2-(2,3-dimethoxybenzoyl)phenyl)-1H-pyrrolo-2-carbaldehyde and 1-(4-chloro-2-(2,3-dimethoxybenzoyl)phenyl)-1H-pyrrolo-3-carbaldehyde.
MS(ESI)m/z:358 (M⁺+H) .
¹H-NMR(CDCl₃)δ:3.83(3H,s),3.88(3H,s),6.07-6.09(1H,m),6.74-6.81(3H,m),7.28(4H,d,J=8.6Hz),7.57(1H,dd,J=8.3,2.5Hz),7.79( 1H,d,J=2.5Hz),8.00(1H,d,J=5.4Hz),9.41(1H,s).
IR(ATR)cm⁻¹:1662,1477,1263,1093,999,849,750.

### Referential Example 98

Methyl (E)-3-{1-[4-chloro-2-(3-fluoro-2-methoxybenzoyl)phenyl]-1H-pyrrol-2-yl}-2-propenoate

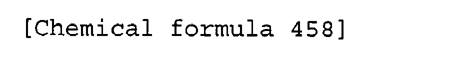

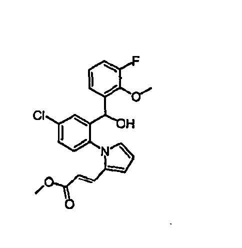

The title compound (1.93 g) was obtained from 1-[4-chloro-2-(3-fluoro-2-methoxybenzoyl)phenyl]-1H-pyrrol-2-carbaldehyde (2.20 g) by using methyl (triphenylphosphoranylidene)acetate (4.12 g) in a similar manner to that employed for the synthesis of methyl (E)-3-(1-(4-chloro-2-(2,3-dimethoxybenzoyl)phenyl)-1H-pyrrol-2-yl)-2-propenoate.
MS (ESI)m/z:414 (M⁺+H).
¹H-NMR(CDCl₃)δ:3.67-3.69(3H,m),3.72(3H,s),5.93(1H,d,J=15.9Hz),6.06-6.10(1H,m),6.46-6.50(1H,m),6.71-6.75(1H,m),6.81-6.90(1H,m),6.99-7.12(2H,m),7.19(1H,d,J=15.9Hz),7.25(2H,d,J=8.3Hz),7.58(1H,d d,J=8.3,2.5Hz),7.64(1H,d,J=2.5Hz).
IR(ATR)cm⁻¹:1705,1628,1263,1171,976,806,739,526.

### Referential Example 99

Methyl (E)-3-(1-{4-chloro-2-[(3-fluoro-2-methoxyphenyl)(hydroxy)methyl]phenyl}-1H-pyrrol-2-yl)-2-propenoate

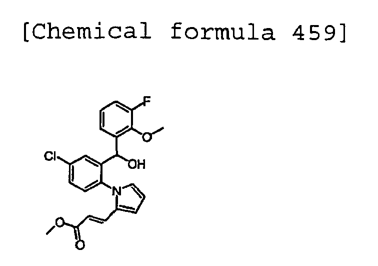

The title compound (1.99 g) was obtained as a mixture of atropisomers from methyl (E)-3-{1-[4-chloro-2-(3-fluoro-2-methoxybenzoyl)phenyl]-1H-pyrrol-2-yl}-2-propenoate (1.80 g) by using sodium borohydride (0.25 g) in a similar manner to that employed for the synthesis of methyl (E)-3-(1-(4-chloro-2-((2,3-dimethoxyphenyl)(hydroxy)methyl)phenyl)-1H-pyrrol-2-yl)-2-propenoate.
MS(ESI)m/z:416(M⁺+H).
¹H-NMR(CDCl₃)δ:2.52-2.65(1H,m),3.63-3.65(3H,m),3.65(1H,s),3.70(1H,s),5.67-5.70(1H,m),5.72-5.76(1H,m),5.92(0H,d,J=15.9Hz),6.18-6.21(0H,m),6.35-6.38(5H,m),6.43-6.45(0H,m),6.63-6.78(2H,m),6.80-6.87(1H,m),6.88-7.04(2H,m),7.10-7.24(1H,m),7.36-7.42(1H,m),7.64-7.67(1H,m).
IR(ATR)cm⁻¹:1687,1624,1479,1267,1171,1036,827,725.

### Referential Example 100

Methyl 2-[8-chloro-6-(3-fluoro-2-methoxyphenyl)-4H,6H-pyrrolo[1,2-a] [4,1]benzoxazepin-4-yl]acetate

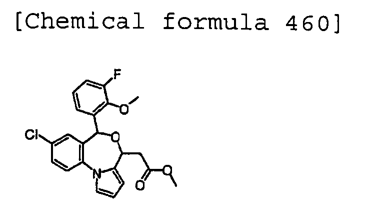

The title compound (322 mg) was obtained as a cis-trans mixture from methyl (E)-3-(1-{4-chloro-2-[(3-fluoro-2-methoxyphenyl)(hydroxy)methyl]phenyl}-1H-pyrrol-2-yl)-2-propenoate (580 mg) by using trifluoroacetic acid (161 µl) in a similar manner to that employed for the synthesis of methyl 2-(8-chloro-6-(2,3-dimethoxyphenyl)-4H,6H-pyrrolo(1,2-a)(4,1)benzoxazepin-4-yl)acetate.
MS(ESI)m/z:416 (M⁺+H).
¹H-NMR(CDCl₃)δ:2.62-2.85 and 2.98-3.15(2H,m),3.56-3.61(3H,m),3.68 and 3.72(3H,s),3.99(OH,d,J=2.2Hz),4.87-4.93(1H,m),5.68 and 6.10(1H,s),6.27-6.31(1H,m),6.35-6.40(1H,m),6.67(1H,d,J=2.2Hz),6.86-7.19(3H,m),7.29-7.48(3H,m).
IR(ATR)cm⁻¹:1736,1479,1269,1171,1099,1001,825,762,715.

### Example 193

2-[8-Chloro-6-(3-fluoro-2-methoxyphenyl)-4H,6H-pyrrolo[1,2-a] [4,1]benzoxazepin-4-yl]-1-ethanol

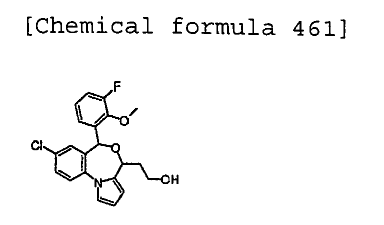

The title compound (747 mg) was obtained from methyl 2-[8-chloro-6-(3-fluoro-2-methoxyphenyl)-4H,6H-pyrrolo[1,2-a][4,1]benzoxazepin-4-yl]acetate (810 mg) by using lithium aluminum hydride (118 mg) in a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methanol.
MS(ESI)m/z:388(M⁺+H).
¹H-NMR(CDCl₃)δ:2.18-2.29(1H,m),2.36-2.47(1H,m),3.59(3H,d,J=2.2Hz),3.92-4.02(2H,m),4.59-4.70(1H,m),5.72(1H,s),6.34-6.40(2H,m),6.68-6.76(1H,m),7.08-7.20(3H,m),7.34-7.51(3H,m). IR(ATR)cm⁻¹:2941,1479,1323,1269,1099,1051,1003,823,71

### Referential Example 101

2-[8-Chloro-6-(3-fluoro-2-methoxyphenyl)-4H,6H-pyrrolo[1,2-a][4,1]benzoxazepin-4-yl]ethylmethanesulfonate

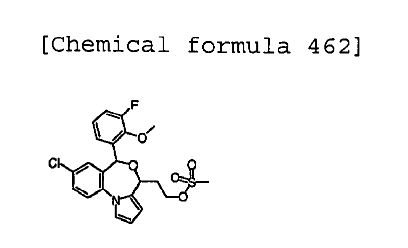

The title compound (821 mg) was obtained from 2-[8-chloro-6-(3-fluoro-2-methoxyphenyl)-4H,6H-pyrrolo[1,2-a][4,1]benzoxazepin-4-yl]-1-ethanol (745 mg) by using methanesulfonyl chloride (162 µl) and triethylamine (365 µl) in a similar manner to that employed for the synthesis of (1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methyl methanesulfonate.
¹H-NMR(CDCl₃)δ:2.42-2.57(2H,m),2.94-3.15(6H,m),3.56-3.62(3H,m),4.35-4.64(3H,m),5.69(1H,s),6.29-6.33(1H,m),6.37-6.42(1H,m),6.68(1H,d,J=2.2Hz),7.04-7.21(3H,m),7.29-7.53(3H,m).
IR (ATR) cm⁻¹: 1479, 1350,1171, 1101, 1001,968,820, 717, 526.

### Referential Example 102

3-[8-Chloro-6-(3-fluoro-2-methoxyphenyl)-4H,6H-pyrrolo[1,2-a][4,1]benzoxazepin-4-yl]propanenitrile

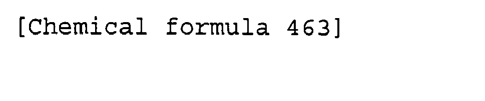

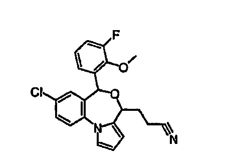

The title compound (520 mg) was obtained from 2-[8-chloro-6-(3-fluoro-2-methoxyphenyl)-4H,6H-pyrrolo[1,2-a][4,1]benzoxazepin-4-yl]ethylmethanesulfonate (815 mg) by using sodium cyanide (172 mg) in a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)acetonitrile.
¹H-NMR(CDCl₃)δ:2.29-2.52(2H,m),2.63-2.72(2H,m),3.57 and 3.58(3H,s),4.49(1H,dd,J=9.4,3.8Hz),5.70(1H,s),6.28-6.33(1H,m),6.38-6.42(1H,m),6.69(1H,d,J=2.2Hz),7.09-7.21(3H,m),7.31-7.52(3H,m).
IR(ATR)cm⁻¹:1479,1269,1176,1103,1011,833,729.

### Example 194

3-[8-Chloro-6-(3-fluoro-2-methoxyphenyl)-4H,6H-pyrrolo[1,2-a][4,1]benzoxazepin-4-yl]propanoicacid

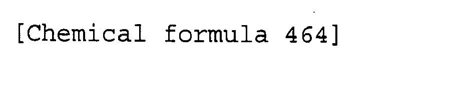

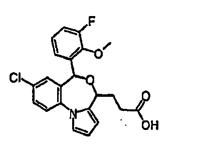

The title compound (containing ethyl acetate, 482 mg) was obtained from 3-[8-chloro-6-(3-fluoro-2-methoxyphenyl)-4H,6H-pyrrolo[1,2-a][4,1]benzoxazepin-4-yl]propanenitrile (439 mg) by using a 5N aqueous sodium hydroxide solution (7 mL) in a similar manner to that employed for the synthesis of 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)acetic acid.
MS (ESI) m/z : 438 (M⁺+Na).
¹H-NMR (CDCl₃) δ:2.27-2.79(3H,m), 3.51-3.61(3H, m), 4.38-4.45(1H,m), 5.69(1H,s), 6.26-6.43(2H,m), 6.65-6.69(1H,m), 7.07-7.19(3H,m), 7.33-7.52(3H,m).
IR(ATR)cm⁻¹:2939,1736,1707,149,1246,1099,1003,825,715.

### Example 195

Ethyl 2-(1-{3-[8-chloro-6-(3-fluoro-2-methoxyphenyl)-4H,6H-pyrrolo[1,2-a][4,1]benzoxazepin-4-yl]propanoyl}-4-piperidinyl)acetate

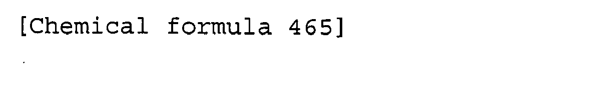

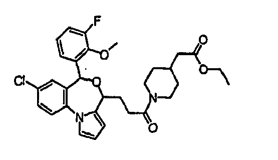

The title compound (173 mg) was obtained from 3-[8-chloro-6-(3-fluoro-2-methoxyphenyl)-4H,6H-pyrrolo[1,2-a][4,1]benzoxazepin-4-yl]propanoic acid (219 mg) by using ethyl 2-(4-piperidinyl)acetate (108 mg), 1-ethyl-3-(3-dimethylaminopropyl)carbodiimide hydrochloride (151 mg), and 1-hydroxybenzotriazole (36 mg) in a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate.
MS(ESI)m/z:569(M⁺+H).
¹H-NMR(CDCl₃)δ:0.86-0.98(1H,m),1.03-1.20(2H,m),1.26(3H,t,J=7.1Hz),1.67-1.82(2H,m),1.92-2.10(1H,m),2.16-2.29(2H,m),2.33-2.45(2H,m),2.48-2.72(3H,m),2.93-3.07(1H,m),3.56-3.60(3H,m),3.81-3.96(1H,m),4.13(2H,q,J=7.1Hz),4.37-4.43(1H,m),4.51-4.65(1H,m),5.67(1H,s),6.34-6.42(2H,m),6.66(1H,d,J=2.2Hz),7.05-7.18(3H,m),7.29-7.53(3H,m).
IR (ATR) cm⁻
¹:2935,1728,1637,1479,1433,1269,1153,1097,1005,823,714.

### Example 196

Ethyl 2-(1-{3-[(4S,6R)-8-chloro-6-(3-fluoro-2-methoxyphenyl)-4H,6H-pyrrolo[1,2-a][4,1]benzoxazepin-4-yl]propanoyl}-4-piperidinyl)acetate Ethyl 2-(1-{3-[(4R,6S)-8-chloro-6-(3-fluoro-2-methoxyphenyl)-4H,6H-pyrrolo[1,2-a][4,1]benzoxazepin-4-yl]propanoyl}-4-piperidinyl)acetate

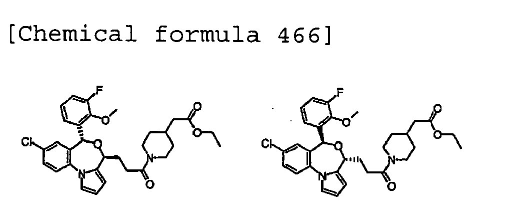

Ethyl 2-(1-{3-[8-chloro-6-(3-fluoro-2-methoxyphenyl)-4H,6H-pyrrolo[1,2-a][4,1]benzoxazepin-4-yl]propanoyl}-4-piperidinyl)acetate (173 mg) was subjected to CHIRALPAK AD using a 20% isopropanol-hexane solution at a flow rate of 10 mL/min to give ethyl 2-(1-{3-[(4S,6R)-8-chloro-6-(3-fluoro-2-methoxyphenyl)-4H,6H-pyrrolo[1,2-a][4,1]benzoxazepin-4-yl]propanoyl}-4-piperidinyl)acetic acid (74 mg) corresponding to a peak with the shortest retention time and ethyl 2-(1-{3-[(4R,6S)-8-chloro-6-(3-fluoro-2-methoxyphenyl)-4H,6H-pyrrolo[1,2-a][4,1]benzoxazepin-4-yl]propanoyl}-4-piperidinyl)acetic acid (75 mg) corresponding to a peak with a long retention time.

### Example 197

2-(1-{3-[(4S,6R)-8-Chloro-6-(3-fluoro-2-methoxyphenyl)-4H,6H-pyrrolo[1,2-a][4,1]benzoxazepin-4-yl]propanoyl}-4-piperidinyl)acetic acid

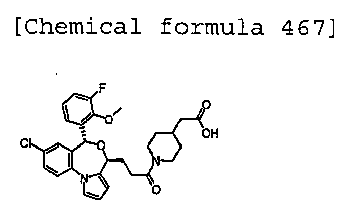

The title compound (60 mg) was obtained from ethyl 2-(1-{3-[(4S,6R)-8-chloro-6-(3-fluoro-2-methoxyphenyl)-4H,6H-pyrrolo[1,2-a][4,1]benzoxazepin-4-yl]propanoyl}-4-piperidinyl)acetate (72 mg) by using potassium carbonate (53 mg) in a similar manner to that employed for the synthesis of 2-(1-(2-(8-chloro-6-(2,3-dimethoxyphenyl)-. 4H,6H-pyrrolo(1,2-a)(4,1)benzoxazepin-4-yl)acetyl)-4-piperidinyl)acetic acid.
MS(ESI)m/z:541(M⁺+H).
¹H-NMR(CDCl₃)δ:1.04-1.36 (2H,m), 1.71-1.86(2H,m) 1.94-2.08(lH,m),2.22-2.34(2H,m),2.34-2.46(2H,m),2.49-2.73(3H,m),2.96-3.08(1H,m),3.51-3.59(3H,m),3.83-3.97(1H,m),4.36-4.44(1H,m),4.56-4.67(1H,m),5.67(1H,s),6.34-6.41(2H,m),6.66(1H,d,J=2.0Hz),7.07-7.17(3H,m),7.32-7.49(3H,m).
IR(ATR)cm⁻
¹:2931,1724,1593,1479,1269,1173,1099,1005,823,714.
Elemental analysis for: C₂₉H₃₀ClFN₂O₅
Calculated: C,64.38;H,5.59;N,5.18.
Found: C,64.24;H,5.83;N,5.13.

### Example 198

2-(1-{3-[(4R,6S)-8-Chloro-6-(3-fluoro-2-methoxyphenyl)-4H,6H-pyrrolo[1,2-a][4,1]benzoxazepin-4-yl]propanoyl}-4-piperidinyl)acetic acid

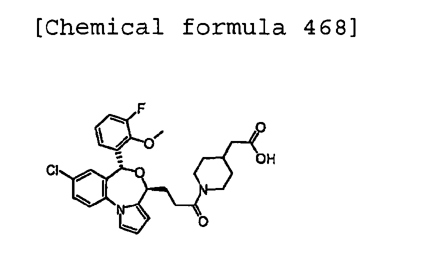

The title compound (63 mg) was obtained from ethyl 2-(1-{3-[(4R,6S)-8-chloro-6-(3-fluoro-2-methoxyphenyl)-4H,6H-pyrrolo[1,2-a][4,1]benzoxazepin-4-yl]propanoyl}-4-piperidinyl)acetate (75 mg) by using potassium carbonate (56 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid.
MS (ESI)m/z: 541 (M⁺+H).
¹H-NMR(CDCl₃)δ:1.05-1.36(2H,m),1.72-1.86(2H,m),1.94-2.08(1H,m),2.23-2.33(2H,m),2.34-2.45(2H,m),2.49-2.73(3H,m),2.96-3.08(1H,m),3.56-3.60(3H,m),3.83-3.96(1H,m),4.36-4.44(1H,m),4.56-4.66(1H,m),5.67(1H,s),6.34-6.41(2H,m),6.66(1H,d,J=2.2Hz),7.07-7.18(3H,m),7.33-7.49 (3H,m) .
IR(ATR)cm⁻
¹:2939,1720,1595,1479,1269,1173,1099,1005,825,733.
Elemental analysis for: C₂₉H₃₀ClFN₂O₅
Calculated: C,64.38;H,5.59;N,5.18.
Found: C,64.26;H,5.83;N,5.15.

### Referential Example 103

[5-Chloro-2(1H-pyrrol-1-yl)phenyl](2-fluoro-3-methoxyphenyl)methanol

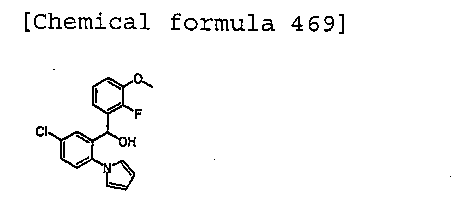

The title compound (4.70 g) was obtained from 1-(2-bromo-4-chlorophenyl)-1H-pyrrole (6.78 g) and 2-fluoro-3-methoxybenzaldehyde (4.28 g) by using normal-butyl lithium (1.6 mol/l, 19.8 mL) in a similar manner to that employed for the synthesis of (5-chloro-3-fluoro-2-pyrrol-1-ylphenyl)-(2,3-dimethoxyphenyl)methanol.
¹H-NMR(CDCl3)δ:2.22-2.25(1H,m),3.86(3H,s),6.00(1H,d,J=4.7Hz),6.27-6.29(2H,m),6.71-6.73(2H,m),6.88-6.96(2H,m),7.03-7.10(1H,m),7.20-7.24(1H,m),7.33(1H,dd,J=8.6,2.5Hz),7.46(1H,d,J=2.5Hz).

### Referential Example 104

[5-Chloro-2-(1H-pyrrol-1-yl)phenyl](2-fluoro-3-methoxyphenyl)methanone

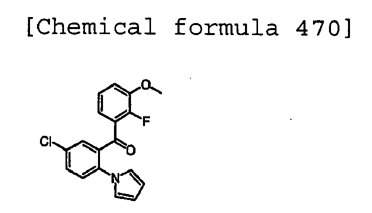

The title compound (4.85 g) was obtained from [5-chloro-2(1H-pyrrol-1-yl)phenyl](2-fluoro-3-methoxyphenyl)methanol (6.89 g) by using manganese dioxide (10.25 g) in a similar manner to that employed for the synthesis of [5-chloro-2-(1H-pyrrol-1-yl)-3-pyridinyl](2,3-dimethoxyphenyl)methanone.
MS (ESI)m/z:330 (M⁺+H) .
¹H-NMR(CDC1₃)δ:3.82 (3H, s) ,5.99-6.01 (2H,m), 6. 64-6.67(2H,m),6.94-7.01(2H,m),7.03-7.09(1H,m),7.31-7.34(1H,m),7.54(1H,dd,J=8.6,2.5Hz),7.60(1H,d,J=2.5Hz).
IR(ATR)cm⁻¹:1655,1579,1481,1271,1065,978,725.

### Referential Example 105

1-[4-Chloro-2-(2-fluoro-3-methoxybenzoyl)phenyl]-1H-pyrrol-2-carbaldehyde

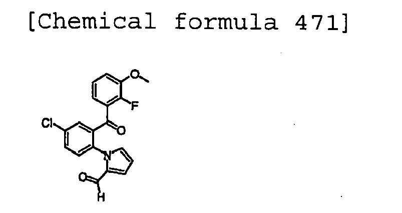

The title compound (1.75 g) was obtained from [5-chloro-2-(1H-pyrrol-1-yl)phenyl](2-fluoro-3-methoxyphenyl)methanone (4.85 g) by using phosphorus oxychloride (1.64 mL) and N,N-dimethylformamide (2.28 mL) in a similar manner to that employed for the synthesis of 1-(4-chloro-2-(2,3-dimethoxybenzoyl)phenyl)-1H-pyrrolo-2-carbaldehyde and 1-(4-chloro-2-(2,3-dimethoxybenzoyl)phenyl)-1H-pyrrolo-3-carbaldehyde.
MS (ESI) m/z:358 (M⁺+H).
¹H-NMR(CDCl₃)δ:3.84(3H,s),6.20(1H,dd,J=4.0,2.6Hz), 6.80-6.84(1H,m),6.89-6.92(1H,m),6.93-7.03(3H,m),7.32(1H,d,J=8.3Hz),7.58(2H,dd,J=8.3,2.5Hz),9.37-9.39(1H,m).
IR(ATR)cm⁻¹:1664,1479,1273,1070,980,822,741.

### Referential Example 106

Methyl (E)-3-{1-[4-chloro-2-(2-fluoro-3-methoxybenzoyl)phenyl]-1H-pyrrol-2-yl}-2-propenoate

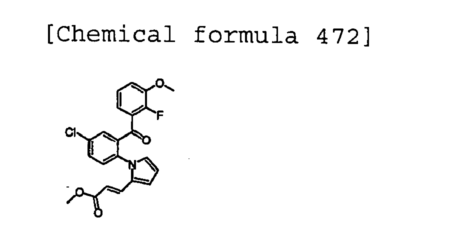

The title compound (1.55 g) was obtained from 1-[4-chloro-2-(2-fluoro-3-methoxybenzoyl)phenyl]-1H-pyrrol-2-carbaldehyde (1.75 g) by using methyl (triphenylphosphoranylidene)acetate (3.26 g) in a similar manner to that employed for the synthesis of methyl (E)-3-(1-(4-chloro-2-(2,3-dimethoxybenzoyl)phenyl)-1H-pyrrol-2-yl)-2-propenoate.
MS(ESI)m/z:436 (M⁺+H).
¹H-NMR(CDCl₃)δ:3.73(3H,s),3.81(3H,s),5.90(1H,d,J=15.7Hz),6.05-6.08(1H,m),6.42-6.46(1H,m),6.71-6.77(1H,m),6.89-7.02(3H,m),7.18(1H,d,J=15.7Hz),7.29(1H,d,J=8.3Hz),7.61(1H,d d,J=8.3,2.5Hz),7.68(1H,d,J=2.5Hz).
IR(ATR)cm⁻¹:1707,1660,1616,1485,1446,1277,1176,1070,974,725.

### Referential Example 107

Methyl (E)-3-(1-{4-chloro-2-[(2-fluoro-3-methoxyphenyl)(hydroxy)methyl]phenyl}-1H-pyrrol-2-yl)-2-propenoate

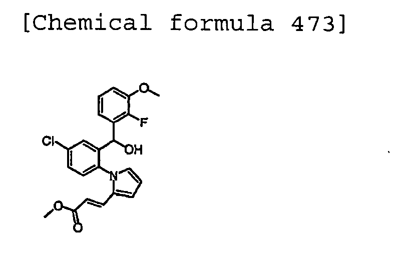

The title compound (1.56 g) was obtained as a mixture of atropisomers from methyl (E)-3-{1-[4-chloro-2-(2-fluoro-3-methoxybenzoyl)phenyl]-1H-pyrrol-2-yl}-2-propenoate (1.54 g) by using sodium borohydride (0.21 g) in a similar manner to that employed for the synthesis of methyl (E)-3-(1-(4-chloro-2-((2,3-dimethoxyphenyl)(hydroxy)methyl)phenyl)-1H-pyrrol-2-yl)-2-propenoate.
MS(ESI)m/z:416(M⁺+H) .
¹H-NMR(CDCl₃)δ:2.24 and 2.40(1H,d,J=4.2Hz),3.63 and 3.70(3H,s),3.80 and 3.84(3H,s),5.63 and 5.72(1H,d,J=4.2Hz),5.91 and 5.91(1H,d,J=15.7Hz),6.22-6.25 and 6.34-6.38(1H,m),6.46-6.48(2H,m),6.74-6.80(1H,m),6.84-6.94 and 6.99-7.07(2H,m),7.10-7.26(1H,m),7.35-7.41(1H,m),7.63 and 7.79(1H,d,J=2.2Hz).
IR(ATR)cm⁻¹:3477,1693,1618,1485,1263,1171,1028,789,719.

### Example 199

Methyl 2-[8-chloro-6-(2-fluoro-3-methoxyphenyl)-4H,6H-pyrrolo[1,2-a] [4,1]benzoxazepin-4-yl]acetate

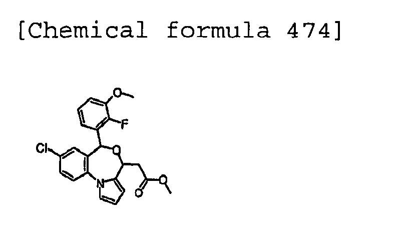

The title compound (623 mg) was obtained from methyl (E)-3-(1-{4-chloro-2-[(2-fluoro-3-methoxyphenyl)(hydroxy)methyl]phenyl}-1H-pyrrol-2-yl)-2-propenoate (1.55 g) by using trifluoroacetic acid (0.43 mL) in a similar manner to that employed for the synthesis of methyl 2-(8-chloro-6-(2,3-dimethoxyphenyl)-4H,6H-pyrrolo(1,2-a)(4,1)benzoxazepin-4-yl)acetate.
MS (ESI) m/z : 416 (M⁺+H).
¹H-NMR(CDCl₃)δ:2.99-3.14(2H,m),3.72(3H,s),3.87(3H,s),4.87-4.96(1H,m),5.71(1H,s),6.27-6.32(1H,m),6.38(1H,t,J=3.2Hz),6.72(1H,d,J=2.0Hz),6.93-7.04(1H,m),7.09-7.26(3H,m),7.30-7.44(2H,m).
IR(ATR)cm⁻¹:1737,1487,1437,1335,1282,1171,1049,823,719.

### Example 200

2-[8-Chloro-6-(2-fluoro-3-methoxyphenyl)-4H,6H-pyrrolo[1,2-a][4,1]benzoxazepin-4-yl]acetic acid

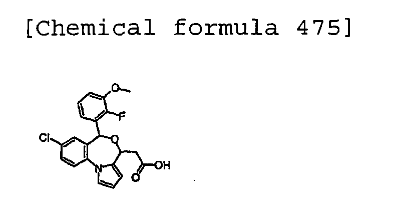

The title compound (462 mg) was obtained from methyl 2-[8-chloro-6-(2-fluoro-3-methoxyphenyl)-4H,6H-pyrrolo[1,2-a][4,1]benzoxazepin-4-yl]acetate (459 mg) by using potassium carbonate (457 mg) in a similar manner to that employed for the synthesis of 2-(8-chloro-6-(2,3-dimethoxyphenyl)-4H,6H-pyrrolo(1,2-a)(4,1)benzoxazepin-4-yl)acetic acid.
MS(ESI)m/z:402(M⁺+H).
¹H-NMR(CDCl₃)δ:3.04-3.19(2H,m),3.88(3H,s),4.88-4.94(1H,m),6.28-6.42(2H,m),6.73-6.81(1H,m),6.82-6.96(1H,m),6.96-7.07(1H,m),7.09-7.22(2H,m),7.33-7.47(2H,m).

### Example 201

Ethyl 2-(1-{2-[8-chloro-6-(2-fluoro-3-methoxyphenyl)-4H,6H-pyrrolo[1,2-a] [4,1]benzoxazepin-4-yl]acetyl}-4-piperidinyl)acetate

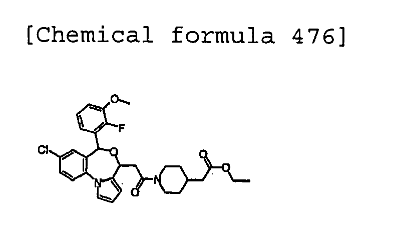

The title compound (157 mg) was obtained from 2-[8-chloro-6-(2-fluoro-3-methoxyphenyl)-4H,6H-pyrrolo[1,2-a][4,1]benzoxazepin-4-yl]acetic acid (171 mg) by using ethyl 2-(4-piperidinyl)acetate(95 mg), 1-ethyl-3-(3-dimethylaminopropyl)carbodiimide hydrochloride (123 mg), and 1-hydroxybenzotriazole (29 mg) in a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate.
MS(ESI)m/z:555(M⁺+H).
¹H-NMR(CDCl₃)δ:0.99-1.24(2H,m),1.23-1.28(3H,m),1.50-1.70(1H,m),1.72-1.82(1H,m),1.93-2.09(1H,m),2.09-2.31(2H,m),2.50-2.72(1H,m),2.79-2.94(1H,m),2.95-3.14(1H,m),3.21-3.31(1H,m),3.88(3H,d,J=2.0Hz),3.99-4.08(1H,m),4.09-4.17(2H,m),4.59-4.75(1H,m),4.92-5.04(1H,m),5.71(1H,d,J=4.9Hz),6.22-6.32(1H,m),6.37(1H,t,J=3.2Hz),6.69(1H,dd,J=9.4,2.1Hz),6.92-7.07(1H,m),7.08-7.11(1H,m),7.15-7.25(2H,m),7.27-7.40(2H,m).
IR(ATR)cm⁻¹:2935,1728,1633,1487,1282,1171,1099,1049,727.

### Example 202

2-(1-{2-[8-Chloro-6-(2-fluoro-3-methoxyphenyl)-4H,6H-pyrrolo[1,2-a][4,1]benzoxazepin-4-yl]acetyl}-4-piperidinyl)acetic acid

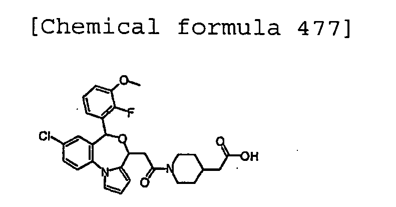

The title compound (80 mg) was obtained from ethyl 2-(1-{2-[8-chloro-6-(2-fluoro-3-methoxyphenyl)-4H,6H-pyrrolo[1,2-a] [4,1]benzoxazepin-4-yl]acetyl}-4-piperidinyl)acetic acid (125 mg) by using potassium carbonate (93 mg) in a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid.
MS(ESI)m/z:527(M⁺+H).
¹H-NMR(CDCl₃)δ:0.95-1.13(1H,m),1.14-1.32(2H,m),1.62-1.86(1H,m),1.92-2.09(1H,m),2.11-2.37(2H,m),2.50-2.73(1H,m),2.76-2.93(1H,m),2.95-3.15(1H,m),3.19-3.34(1H,m),3.87 and 3.88(3H,s),3.99-4.10(1H,m),4.61-4.76(1H,m),4.93-5.03(1H,m),5.71(1H,d,J=5.4Hz),6.21-6.32(1H,m),6.37(1H,t,J=3.2Hz),6.66-6.73(1H,m),6.93-7.03(1H,m),7.08-7.11(1H,m),7.13-7.25(2H,m),7.31-7.41(2H,m).
IR(ATR)cm⁻¹:2929,1718,1589,1489,1281,1173,1049,823,727.
Elemental analysis for: C₂₈H₂₈ClFN₂O₅
Calculated: C,63.82;H,5.36;N,5.32.
Found: C,63.80;H,5.61;N,5.12.

### Referential Example 108

[5-Chloro-2-(1H-pyrrol-1-yl)phenyl](2,3-dichlorophenyl)methanol

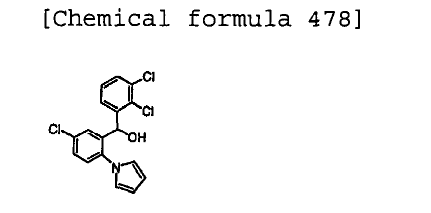

1-(2-Bromo-4-chlorophenyl)-1H-pyrrole (6.00 g) was dissolved in diethyl ether (200 mL). To the resulting solution was added dropwise an n-butyl lithium hexane solution (18.00 mL) at -78°C. The resulting mixture was warmed to ice cooling temperature and stirred for one hour. To the reaction mixture was added 2,3-dichlorobenzaldehyde (4.91 g) under ice cooling, followed by stirring for 30 minutes. To the reaction mixture was added a saturated aqueous solution of ammonium chloride. The resulting mixture was extracted with ethyl acetate. The organic layer was dried over anhydrous sodium sulfate and the solvent was distilled off under reduced pressure. The residue was purified by silica gel column chromatography (hexane:ethyl acetate=20:1) to give the title compound (6.10 g).
MS (EI) m/z : 351 (M⁺) .
¹H-NMR(CDCl₃)δ:6.05(1H,s),6.29-6.33 (2H,m) , 6.78-6.81(2H,m),7.17-7.20(1H,m),7.25-7.31(2H,m),7.33-7.38(1H,m),7.43(1H,d,J=7.8Hz),7.52(1H,d,J=7.8Hz).

### Referential Example 109

1-[4-Chloro-2-(2,3-dichlorobenzoyl)phenyl]-1H-pyrrol-2-carbaldehyde

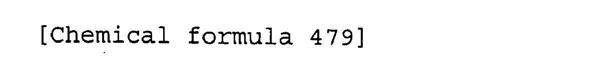

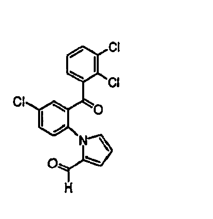

[5-Chloro-2-(1H-pyrrol-1-yl)phenyl](2,3-dichlorophenyl)methanol (6.10 g) was dissolved in dichloromethane (200 mL). To the resulting solution was added manganese dioxide (15.0 g). The resulting mixture was stirred under heat at 50°C overnight. After the reaction mixture was filtered through a Kiriyama funnel, the filtrate was concentrated under reduced pressure. Phosphorus oxychloride (4.84 mmol) was dissolved in dichloromethane (200 mL). Under ice cooling, N,N-dimethylformamide (5.36 mL) was added dropwise. To the reaction mixture was added dropwise the remaining dichloromethane solution (50 mL) of the previously-obtained residue under ice cooling, followed by stirring at room temperature for 3 hours. An aqueous solution (200 mL) of sodium acetate (21.3 g) was added to the reaction mixture and the layers separated. The organic layer was dried over anhydrous sodium sulfate and then, the solvent was distilled off under reduced pressure. The residue was purified by silica gel column chromatography (hexane:ethyl acetate=9:1) to give the title compound (3.43 g).
MS(EI)m/z:377(M⁺).
¹H-NMR(CDCl₃)δ:6.21-6.24(1H,m),6.79-6.82(1H,m),6.90(1H,s),7.06-7.19(2H,m),7.29(1H,d,J=8.5Hz),7.42-7.45(1H,m),7.60(1H,dd,J=8.5,2.0Hz),7.71(1H,d,J=2.0Hz),9.35( 1H,s).

### Referential Example 110

Methyl (E)-3-{1-[4-chloro-2-(2,3-dichlorobenzoyl)phenyl]-1H-pyrrol-2-yl}-2-propenoate

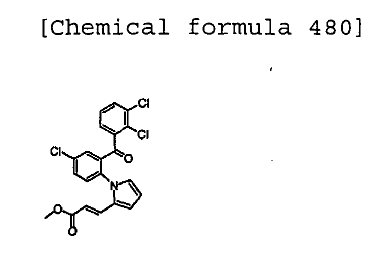

1-[4-Chloro-2-(2,3-dichlorobenzoyl)phenyl]-1H-pyrrol-2-carbaldehyde (3.42 g) was dissolved in toluene (200 mL). To the resulting solution was added methyl (trimethylphosphoranylidene)acetate (7.85 g). The resulting mixture was stirred overnight at 70°C. The reaction mixture was concentrated under reduced pressure. The residue was purified by silica gel column chromatography (hexane:ethyl acetate=10:1) to give the title compound (4.05 g).
MS(EI)m/z:433(M⁺).
¹H-NMR(CDCl₃)δ:3.73(3H,s),5.88(1H,d,J=15.9Hz),6.03-6.07(1H,m),6.37-6.42(1H,m),6.66-6.70(1H,m),6.99-7.05(2H,m),7.12(1H,d,J=15.9Hz),7.23-7.28(2H,m),7.35-7.41(1H,m),7.65(1H,dd,J=8.3,2.4Hz),7.81(1H,d,J=2.4Hz).

### Example 203

Methyl 2-[8-chloro-6-(2,3-dichlorobenzoyl)-4H,6H-pyrrolo[1,2-a][4,1]benzoxazepin-4-yl]acetate

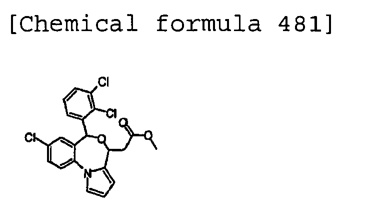

Methyl (E)-3-{1-[4-chloro-2-(2,3-dichlorobenzoyl)phenyl]-1H-pyrrol-2-yl}-2-propenoate (100 mg) was dissolved in methanol (3 mL). Sodium borohydride (17 mg) was added and the resulting mixture was stirred at room temperature for one hour. The reaction mixture was concentrated under reduced pressure. Water and ethyl acetate were added to the residue and the layers separated. The organic layer was dried over anhydrous sodium sulfate. The solvent was distilled off under reduced pressure. The residue was dissolved in dichloromethane (5 mL). To the resulting solution was added trifluoroacetic acid (27 µl), followed by stirring overnight at room temperature. A saturated aqueous solution of sodium hydrogen carbonate was added to the reaction mixture and the layers separated.
The organic layer was then dried over anhydrous sodium sulfate and the solvent was distilled off under reduced pressure. The residue was purified by silica gel column chromatography (hexane:ethyl acetate=10:1) to give the title compound (73 mg).
MS(FAB)m/z:436 (M⁺+H).
¹H-NMR(CDCl₃)δ:3.03(1H,dd,J=15.1,5.7Hz),3.11(1H,dd,J=15.1,8.3H z),3.73(3H,s),4.91(1H,dd,J=8.3,5.7Hz),5.68(1H,s),6.29-6.32(1H,m),6.39(1H,t,J=3.3Hz),6.56(1H,d,J=2.2Hz),7.11-7.14(1H,m),7.33-7.45(3H,m),7.50(1H,dd,J=7.8,1.5Hz),7.71(1H,d,J=7.8Hz).

### Example 204

2-[8-Chloro-6-(2,3-dichlorobenzoyl)-4H,6H-pyrrolo[1,2-a][4,1]benzoxazepin-4-yl]acetic acid

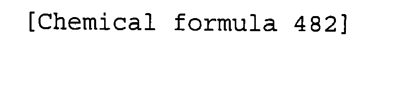

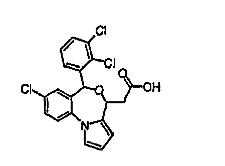

Methyl 2-[8-chloro-6-(2,3-dichlorobenzoyl)-4H,6H-pyrrolo[1,2-a][4,1]benzoxazepin-4-yl]acetate (200 mg) was dissolved in tetrahydrofuran (4 mL). Methanol (2 mL) and 1N aqueous sodium hydroxide solution (733 µl) were added. The resulting mixture was stirred overnight at room temperature. After concentration of the reaction mixture under reduced pressure, chloroform and a 10% aqueous citric acid solution were added to the residue and the layers separated. The organic layer was dried over anhydrous sodium sulfate and the solvent was distilled off under reduced pressure to give the title compound (61 mg).
MS(EI)m/z:421(M⁺).
¹H-NMR(CDCl₃)δ:3.04-3.19(2H,m),4.88-4.95(1H,m),5.70(1H,s),6.32-6.37(1H,m),6.38-6.42(1H,m),6.57(1H,d,J=2.2Hz),7.13-7.15(1H,m),7.31-7.45(3H,m),7.50(1H,d,J=8.1Hz),7.73(1H,d,J=8.1Hz).
Elemental analysis for: C₂₀H₁₄Cl₃NO₃
Calculated: C,56.83;H,3.34;N,3.31.
Found: C,56.44;H,3.54;N,3.14.

### Example 205

Ethyl 2-(1-{2-[8-chloro-6-(2,3-dichlorobenzoyl)-4H,6H-pyrrolo[1,2-a] [4,1]benzoxazepin-4-yl]acetyl}-4-piperidinyl)acetate

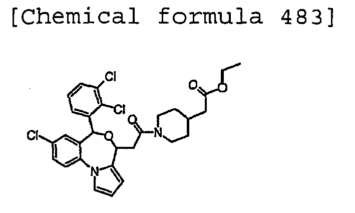

2-[8-Chloro-6-(2,3-dichlorobenzoyl)-4H,6H-pyrrolo[1,2-a][4,1]benzoxazepin-4-yl]acetic acid (47 mg, 0.11 mmol) was dissolved in dichloromethane (2 mL). To the resulting solution were added 1-ethyl-3-(3-dimethylaminopropyl)carbodiimide hydrochloride (32 mg), ethyl 2-(4-piperidinyl)acetate (23 mg), and 1-hydroxybenzotriazole monohydrate (17 mg). The resulting mixture was stirred overnight at room temperature. Water was added to the reaction mixture and the layers separated. The organic layer was dried over anhydrous sodium sulfate. The solvent was distilled off under reduced pressure. The residue was purified by silica gel column chromatography (hexane:ethyl acetate=3:1) to give the title compound (47 mg).
MS(EI)m/z:574(M⁺).
¹H-NMR(CDCl₃)δ:1.06-1.29(5H,m),1.67-1.85(2H,m),1.96-2.09(1H,m),2.15-2.20(1H,m),2.22-2.28(1H,m),2.83-2.93(1H,m),3.21-3.30(1H,m),4.04(1H,d,J=11.5Hz),4.14(2H,dd,J=7.2,2.8Hz),4.67 (1H,s),5.69(1H,d,J=9.8Hz),6.26(1H,s),6.38(1H,t,J=3.3Hz),6.5 3(1H,s),7.11(1H,s),7.32-7.47(3H,m),7.48(1H,s),7.68-7.78(1H,m).

### Example 206

2-(1-{2-[8-Chloro-6-(2,3-dichlorobenzoyl)-4H,6H-pyrrolo[1,2-a][4,1]benzoxazepin-4-yl]acetyl}-4-piperidinyl)acetic acid

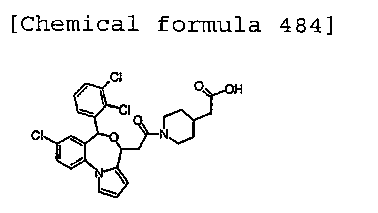

Ethyl 2-(1-{2-[8-chloro-6-(2,3-dichlorobenzoyl)-4H,6H-pyrrolo[1,2-a][4,1]benzoxazepin-4-yl]acetyl}-4-piperidinyl)acetate (44 mg) was dissolved in methanol (1 mL). To the resulting solution were added tetrahydrofuran (0.5 mL), water (1 mL), and anhydrous potassium carbonate (32 mg). The resulting mixture was stirred at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was then distilled off under reduced pressure to give the title compound (42 mg).
MS(EI)m/z:546(M⁺).
¹H-NMR(CDCl₃)δ:1.07-1.34 (2H,m), 1.69-1.89(2H,m),1.95-2.10(1H,m),2.20-2.24(1H,m),2.28-2.33(1H,m),2.56-2.72(1H,m),2.82-2.93(1H,m),2.96-3.16(1H,m),3.22-3.31(1H,m),4.00-4.08(1H,m),4.64-4.75(1H,m),4.94-5.04(1H,m),5.69(1H,d,J=9.5Hz),6.27(1H,s),6.37-6.40(1H,m),6.51-6.54(1H,m),7.12(1H,s),7.31-7.46(3H,m),7.48-7.53(1H,m),7.67-7.77(1H,m).
Elemental analysis for: C₂₇H₂₅ClN₂O₄·1.25H₂O
Calculated: C,56.86;H,4.86;N,4.91.
Found: C,56.84;H,4.56;N,4.61.

### Referential Example 111

[5-Chloro-2-(1H-pyrrol-1-yl)phenyl](1-naphthyl)methanol

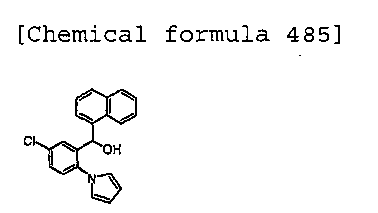

1-(2-Bromo-4-chlorophenyl)-1H-pyrrole (6.00 g) was dissolved in diethyl ether (200 mL). An n-butyl lithium hexane solution (18.00 mL) was added dropwise to the resulting solution at -78°C. The reaction mixture was raised to a temperature of ice cooling and stirred for 1 hour. 1-Naphthaldehyde (3.81 mL) was added under ice cooling, followed by stirring for 30 minutes. To the reaction mixture was added a saturated aqueous solution of ammonium chloride. The resulting mixture was extracted with ethyl acetate. After the organic layer was dried over anhydrous sodium sulfate, the solvent was distilled off under reduced pressure. The residue was purified by silica gel column chromatography (hexane:ethyl acetate=20:1) to give the title compound (5.78 g).
MS(EI)m/z:333(M⁺).
¹H-NMR(CDCl₃)δ:6.25-6.28(2H,m),6.48(1H,s),6.80-6.84(2H,m),7.24-7.52(5H,m),7.56-7.63(2H,m),7.79-7.86(2H,m).

### Referential Example 112

1-[4-Chloro-2-(1-naphthoyl)phenyl]-1H-pyrrol-2-carbaldehyde

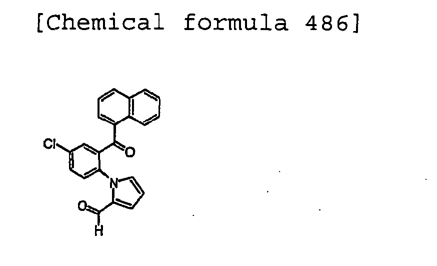

5-Chloro-2-(1H-pyrrol-1-yl)phenyl](1-naphthyl)methanol (5.78 g) was dissolved in dichloromethane (200 mL). Manganese dioxide (15.1 g) was added and the resulting mixture was stirred overnight under heat at 50°C. The reaction mixture was filtered through a Kiriyama funnel. The filtrate was concentrated under reduced pressure. Phosphorus oxychloride (4.84 mmol) was dissolved in dichloromethane (200 mL) and N,N-dimethylformamide (5.36 mL) was added dropwise under ice cooling, followed by stirring for 20 minutes. To the reaction mixture was added dropwise a dichloromethane solution (50 mL) of the previously-obtained residue under ice cooling, followed by stirring at room temperature for 3 hours. An aqueous solution (200 mL) of sodium acetate (21.3 g) was added to the reaction mixture and the layers separated. After the organic layer was dried over anhydrous sodium sulfate, the solvent was distilled off under reduced pressure. The residue was purified by silica gel column chromatography (hexane:ethyl acetate=10:1) to give the title compound (2.95 g).
MS(EI)m/z:359(M⁺).
¹H-NMR(CDCl₃)δ:5.96(1H,dd,J=4.0,2.6Hz),6.60(1H,dd,J=4.0,1.6Hz) ,6.88(1H,s),7.25-7.35(2H,m),7.47-7.62(4H,m),7.69(1H,d,J=2.4Hz),7.79-7.83(1H,m),7.88(1H,d,J=8.3Hz),8.54(1H,d,J=8.3Hz),9.29(1H,s)

### Referential Example 113

Methyl (E)-3-{1-[4-chloro-2-(1-naphthoyl)phenyl]-1H-pyrrol-2-yl}-2-propenoate

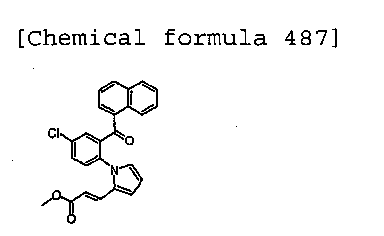

1-[4-Chloro-2-(1-naphthoyl)phenyl]-1H-pyrrol-2-carbaldehyde (2.95 g) was dissolve in toluene (200 mL). To the resulting solution was added methyl (trimethylphosphoranylidene)acetate (7.13 g). The resulting mixture was stirred overnight at 70°C. The reaction mixture was concentrated under reduced pressure. The residue was purified by silica gel column chromatography (hexane:ethyl acetate=10:1) to give the title compound (3.18 g).
MS(FAB)m/z:415(M⁺+H) .
H-NMR(CDCl₃)δ:3.75(3H,s),5.76-5.84 (2H,m),6.18-6.21(1H,m),6.65-6.68(1H,m),7.16(1H,d,J=15.6Hz),7.20-7.27(2H,m),7.30(1H,d,J=8.5Hz),7.34(1H,d,J=7.1Hz),7.46-7.56(2H,m),7.64(1H,dd,J=8.5,2.4Hz),7.75-7.81(2H,m),7.84(1H,d,J=8.1Hz),8.48(1H,d,J=8.1Hz).

### Example 207

Methyl 2-[8-chloro-6-(1-naphthyl)-4H,6H-pyrrolo[1,2-a][4,1]benzoxazepin-4-yl]acetate

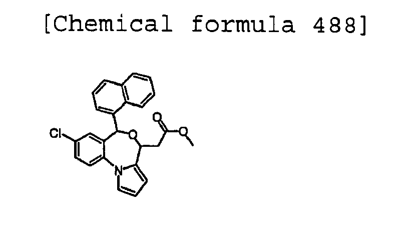

Methyl (E)-3-{1-[4-chloro-2-(1-naphthoyl)phenyl]-1H-pyrrol-2-yl}-2-propenoate (100 mg) was dissolved in methanol (3 mL). Sodium borohydride (18 mg) was added. The resulting mixture was stirred at room temperature for one hour. The reaction mixture was concentrated under reduced pressure. Water and ethyl acetate were added to the residue and the layers separated. The organic layer was dried over anhydrous sodium sulfate. The solvent was distilled off under reduced pressure. The residue was dissolved in dichloromethane (5 mL). Trifluoroacetic acid (28 µl) was added and the resulting mixture was stirred overnight at room temperature. A saturated aqueous solution of sodium hydrogen carbonate was added to the reaction mixture and the layers separated. The organic layer was dried over anhydrous sodium sulfate. The solvent was distilled off under reduced pressure. The residue was purified by silica gel column chromatography (hexane:ethyl acetate=9:1) to give the title compound (63 mg).
MS (FAB)m/z:418 (M⁺+H) .
¹H-NMR(CDCl₃)δ:3.07(1H,dd,J=15.1,5.6Hz),3.16(1H,dd,J=15.1,8.3H z),3.74(3H,s),5.04(1H,dd,J=8.3,5.6Hz),6.07(1H,s),6.30-6.37(1H,m),6.40-6.44(1H,m),6.59(1H,d,J=2.2Hz),7.18-7.22(1H,m),7.26-7.33(2H,m),7.34-7.46(3H,m),7.49-7.61(1H,m),7.80-7.92(3H,m).

### Example 208

2-[8-Chloro-6-(1-naphthyl)-4H,6H-pyrrolo[1,2-a][4,1]benzoxazepin-4-yl]acetic acid

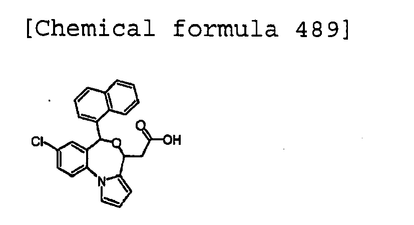

Methyl 2-[8-chloro-6-(1-naphthyl)-4H,6H-pyrrolo[1,2-a] [4,1]benzoxazepin-4-yl]acetate (300 mg) was dissolved in tetrahydrofuran (5 mL). To the resulting solution were added methanol (3 mL) and a 1N aqueous sodium hydroxide solution (1.16 mL). The resulting mixture was stirred overnight at room temperature. After concentration of the reaction mixture under reduced pressure, chloroform and a 10% aqueous citric acid solution were added to the residue and the layers separated. After the organic layer was dried over anhydrous sodium sulfate, the solvent was distilled off under reduced pressure to give the title compound (153 mg).
MS(EI)m/z:403(M⁺).
¹H-NMR(CDCl₃)δ:3.11-3.18(1H,m),3.18-3.25(1H,m),5.01-5.07(1H,m),6.11(1H,s),6.37-6.41(1H,m),6.43-6.47(1H,m),6.62(1H,s),7.20-7.23(1H,m),7.25-7.33(2H,m),7.37-7.49(3H,m),7.56(1H,t,J=7.8Hz),7.80-7.91(3H,m).

### Example 209

Ethyl 2-(1-{2-[8-chloro-6-(1-naphthyl)-4H,6H-pyrrolo[1,2-a][4,1]benzoxazepin-4-yl]acetyl}-4-piperidinyl)acetate

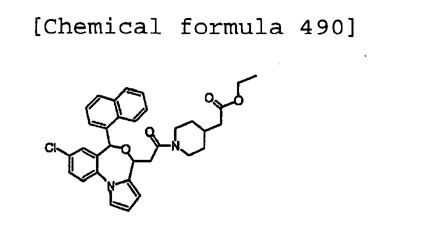

Ethyl 2-(1-{2-[8-chloro-6-(1-naphthyl)-4H,6H-pyrrolo[1,2-a][4,1]benzoxazepin-4-yl]acetyl}-4-piperidinyl)acetate (50 mg) was dissolved in dichloromethane (2 mL). To the resulting solution were added 1-ethyl-3-(3-dimethylaminopropyl)carbodiimide hydrochloride (36 mg), ethyl 2-(4-piperidinyl)acetate (25 mg), and 1-hydroxybenzotriazole monohydrate (19 mg). The resulting mixture was stirred overnight at room temperature. Water was added to the reaction mixture and the layers separated. The organic layer was dried over anhydrous sodium sulfate. After the solvent was distilled off under reduced pressure, the residue was purified by silica gel column chromatography (hexane:ethyl acetate=3:1) to give the title compound (47 mg).
MS (FAB) m/z : 557 (M⁺+H) .
¹H-NMR(CDCl₃)δ:1.09-1.32(5H, m), 1.59-1.82 (2H,m),1.87-2.06(2H,m),2.14-2.32(1H,m),2.77-3.14(2H,m),3.27-3.39(1H,m),3.98-4.18(3H,m),4.62-4.73(1H,m),5.05-5.19(1H,m),6.02-6.07(1H,m),6.28-6.32(1H,m),6.40-6.44(1H,m),6.54-6.63(1H,m),7.18(1H,s),7.26-7.46(5H,m),7.48-7.59(1H,m),7.77-7.91(3H,m).

### Example 210

2-(1-{2-[8-Chloro-6-(1-naphthyl)-4H,6H-pyrrolo[1,2-a][4,1]benzoxazepin-4-yl]acetyl}-4-piperidinyl)acetic acid

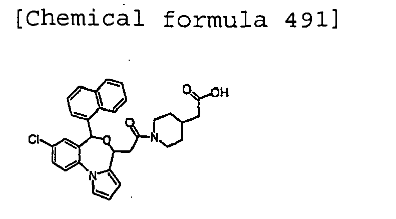

Ethyl 2-(1-{2-[8-chloro-6-(1-naphthyl)-4H,6H-pyrrolo[1,2-a][4,1]benzoxazepin-4-yl]acetyl}-4-piperidinyl)acetate (44 mg) was dissolved in methanol (1 mL). To the resulting solution were added tetrahydrofuran (0.5 mL), water (1 mL) and anhydrous potassium carbonate (33 mg). The resulting mixture was stirred under heat at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (42 mg).
MS (EI) m/ z: 52 8 (M⁺)
¹H-NMR(CDCl₃) δ:0.76-1.07 (1H,m), 1.12-1.34 (2H,m) 1.46-2.08(3H,m),2.16-2.33(1H,m),2.49-2.71(1H,m),2.83-3.04(2H,m),3.29-3.40(1H,m),3.98-4.07(1H,m),4.63-4.73(1H,m),5.04-5.19(1H,m),6.00-6.08(1H,m),6.26-6.34(1H,m),6.40-6.46(1H,m),6.53-6.63(1H,m),7.17(1H,s),7.27-7.46(5H,m),7.47-7.58(1H,m),7.69-7.92(3H,m).

### Referential Example 114

(5-Chloro-2-pyrrol-1-yl-phenyl)-(2-ethoxy-3-methoxyphenyl)methanol

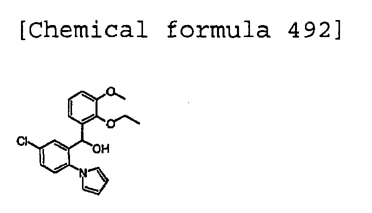

1-(2-bromo-4-chlorophenyl)-1H-pyrrole (2.00 g) was dissolved in ether (50 mL). At -78°C, n-butyl lithium (1.6M hexane solution) (6.30 mL) was added dropwise. The reaction mixture was stirred for 1 hour under ice cooling and then stirred for 5 minutes at room temperature. After the reaction mixture was cooled to -78°C, 2-ethoxy-3-methoxybenzaldehyde (1.66 mL) was added. The resulting mixture was stirred at room temperature for 1 hour. Ethyl acetate and water were added to the reaction mixture. The water layer was extracted three times with ethyl acetate. The organic layers were combined, washed with saturated brine and dried over anhydrous sodium sulfate. The solvent was distilled off under reduced pressure. The residue thus obtained was purified by a Yamazen medium pressure preparative chromatogram system (hexane:ethyl acetate= from 75:25 to 67:33) to give the title compound (2.09 g).
MS (ESI)m/z:340 (M⁺-OH).
¹H-NMR(CDCl₃)δ:1.18(3H,t,J=7.1Hz),2.96(1H,d,J=3.9Hz),3.70(1H,d q,J=9.5,7.1Hz),3.84(3H,s),4.02(1H,dq,J=9.5,7.1Hz),5.97(1H,d ,J=3.9Hz),6.21-6.23(2H,m),6.62(1H,dd,J=7.9,1.4Hz),6.67-6.69(2H,m),6.88(1H,dd,J=8.3,1.4Hz),6.99(1H,t,J=7.9Hz),7.22( 1H,d,J=8.3Hz),7.33(1H,dd,J=8.3,2.3Hz),7.56(1H,d,J=2.3Hz). .IR(ATR)cm⁻¹:3451,2973,1494,1261,1016,798,727.

### Referential Example 115

(5-Chloro-2-pyrrol-1-yl-phenyl)-(2-ethoxy-3-methoxyphenyl)methanone

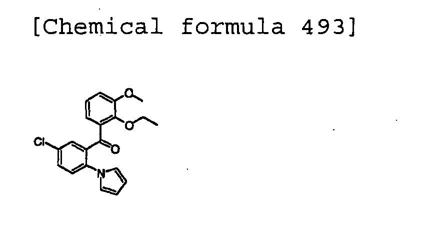

(5-Chloro-2-pyrrol-1-yl-phenyl)-(2-ethoxy-3-methoxyphenyl)methanol (2.09 g) was dissolved in dichloromethane. Manganese dioxide (5.77 g) was added and the resulting mixture was heated under reflux for 5 hours. After stirring further at room temperature for 13 hours, manganese dioxide (1.00 g) was added and the resulting mixture was heated under reflux for 1.5 hours. The reaction mixture was filtered through celite. The filtrate was concentrated under reduced pressure to give the title compound (1.75 g).
MS(ESI)m/z:356(M⁺+H).
¹H-NMR(CDCl₃)δ:0.90(3H,t,J=7.1Hz),3.77(2H,q,J=7.1Hz),3.81(3H,s ),6.04(2H,t,J=2.2Hz),6.72(2H,t,J=2.2Hz),6.95-6.96(1H,m),6.97(1H,d,J=2.7Hz),7.06-7.09(1H,m),7.30(1H,d,J=8.3Hz),7.48-7.52(2H,m).
IR(ATR)cm⁻¹:2977,1664,1577,1494,1259,1068,725,516.

### Referential Example 116

1-[4-Chloro-2-(2-ethoxy-3-methoxybenzoyl)phenyl]-1H-pyrrol-2-carbaldehyde 1-[4-Chloro-2-(2-ethoxy-3-methoxybenzoyl)phenyl]-1H-pyrrol-3-carbaldehyde

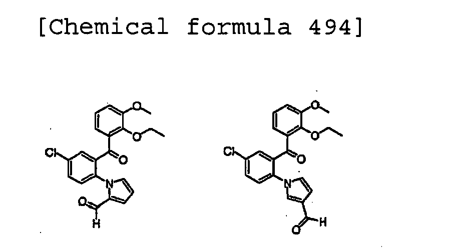

Dimethylformamide (0.762 mL) was dissolved in dichloroethane (10 mL). Under ice cooling, phosphorus oxychloride (0.550 mL) was added. The resulting mixture was stirred at room temperature for 10 minutes. After ice cooling again, a dichloroethane solution (10 mL) of (5-chloro-2-pyrrol-1-yl-phenyl)-(2-ethoxy-3-methoxyphenyl)methanone (1.75 g) was added dropwise. The reaction mixture was heated under reflux for 3.5 hours and then, cooled to room temperature. A saturated aqueous solution (14 mL) of sodium acetate was added. The resulting mixture was heated under reflux for one hour and then cooled to room temperature. Dichloromethane and water were added and the layers separated. The aqueous layer was extracted with dichloromethane. The organic layers were combined, washed with saturated brine and dried over anhydrous sodium sulfate. The solvent was distilled off under reduced pressure. The residue thus obtained was purified by a Yamazen medium pressure preparative chromatogram system (hexane:ethyl acetate=66:34) to give the title compound (2-substituted pyrrole) (997 mg) and position isomer (3-substituted pyrrole) (470 mg). 2-Substituted pyrrole
MS(ESI)m/z:384(M⁺+H).
¹H-NMR(CDCl₃)δ:1.00(3H,t,J=7.1Hz),3.73(3H,s),3.81-3.91(2H,m),6.14(1H,dd,J=4.1,2.7Hz),6.83(1H,dd,J=4.1,1.7Hz), 6.89-6.94(4H,m),7.28(1H,d,J=8.3Hz),7.54(1H,dd,J=8.3,2.5Hz),7.62( 1H,d,J=2.5Hz),9.39(1H,s).
IR(ATR)cm⁻¹:2979,1658,1490,1259,1068,977,906,765,549. 3-Substituted pyrrole
MS (ESI)m/z:384 (M⁺+H) .
¹H-NMR(CDCl₃)δ:0.87(3H,t,J=7.1Hz),3.76 (2H,q,J=7.1Hz),3.80(3H,s ),6.47(1H,dd,J=2.9,1.7Hz),6.72-6.73(1H,m),6.92-7.01(2H,m),7.08(1H,dd,J=7.1,2.5Hz),7.31-7.33(2H,m),7.59(1H,d,J=2.5Hz),7.56(1H,dd,J=8.3,2.5Hz),9.64( 1H,s).

### Referential Example 117

Methyl 3-[1-[4-chloro-2-(2-ethoxy-3-methoxybenzoyl)phenyl]-1H-pyrrol-2-yl]acrylate

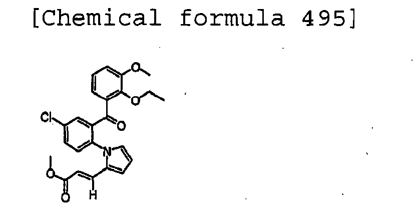

1-[4-Chloro-2-(2-ethoxy-3-methoxybenzoyl)phenyl]-1H-pyrrol-2-carbaldehyde (997 mg) was dissolved in toluene (20 mL). Methyl (triphenylphosphoranylidene)acetate (1.74 g) was added and the resulting mixture was heated under reflux for 4 hours. To the reaction mixture was added methyl (triphenylphosphoranylidene)acetate (1.74 g), followed by heating under reflux for further one hour. Then, the reaction mixture was cooled to room temperature. The residue obtained by distilling off the solvent under reduced pressure was purified by silica gel column chromatography (hexane:ethyl acetate= from 4:1 to 3:2) to give the title compound (1.10 g).
MS(ESI)m/z:440(M⁺+H).
¹H-NMR(CDCl₃)δ:0.94(3H,t,J=7.2Hz),3.72(3H,s),3.65-3.94(2H,m),3.75(3H,s),5.91(1H,d,J=15.9Hz),6.01-6.03(1H,m),6.45-6.47(1H,m),6.75(1H,dd,J=2.4,1.7Hz),6.89-6.93(3H,m),7.22(1H,d,J=8.3Hz),7.22(1H,d,J=15.9Hz),7.56(1H,d d,J=8.4,2.6Hz),7.63(1H,d,J=2.4Hz).
IR(ATR)cm⁻¹:2944,1695,1658,1450,1259,973,833,736,441.

### Referential Example 118

Methyl 3-[1-[4-chloro-2-[(2-ethoxy-3-methoxyphenyl)hydroxymethyl]phenyl]-1H-pyrrol-2-yl]acrylate

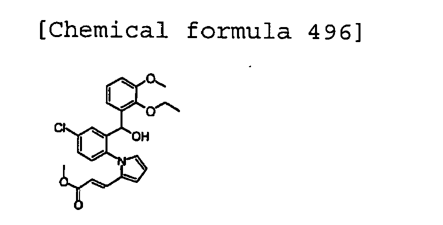

Methyl 3-[1-[4-chloro-2-(2-ethoxy-3-methoxybenzoyl)phenyl]-1H-pyrrol-2-yl]acrylate (1.10 g) was dissolved in methanol (100 mL). Under ice cooling, sodium borohydride (0.142 g) was added to the resulting solution. The reaction mixture was stirred at room temperature for 1.5 hours. Acetone (10 mL) was added and the resulting mixture was stirred for 10 minutes. The solvent was distilled off under reduced pressure. Ethyl acetate and saturated ammonium chloride were added to the residue. The water layer was extracted with ethyl acetate. The organic layers were combined, washed with saturated brine and dried over anhydrous sodium sulfate. The solvent was distilled off to give the title compound (1.08 g).
MSESIm/z:424 (M⁺-OH) .
IR(ATR)cm⁻¹:3440,1683,1617,1481, 1253,1174,1024,730.

### Referential Example 119

3-[1-[4-Chloro-2-[(2-ethoxy-3-methoxyphenyl)hydroxymethyl]phenyl]-1H-pyrrol-2-yl]acrylic acid

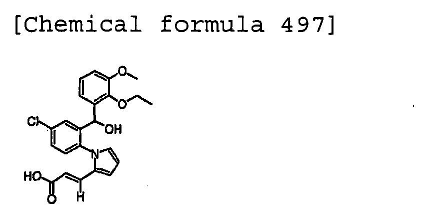

Methyl 3-[1-[4-chloro-2-[(2-ethoxy-3-methoxyphenyl)hydroxymethyl]phenyl]-1H-pyrrol-2-yl]acrylate (470 mg) was dissolved in methanol (10 mL). To the resulting solution were added potassium carbonate (441 mg) and water (10 mL). The resulting mixture was stirred at 60°C for 3.5 hours. The reaction mixture was concentrated under reduced pressure. The residue was neutralized with a 1N aqueous hydrochloric acid solution, followed by extraction with ethyl acetate. The organic layers were combined, washed with saturated brine and dried over anhydrous sodium sulfate. The solvent was distilled off to give the title compound (454 mg).
MSESIm/z:410(M⁺-OH).
IR(ATR)cm⁻¹:2948,1677,1610,1484,1243,1035,723,607.

### Referential Example 120

Ethyl [1-[3-[1-[4-chloro-2-[1-(2-ethoxy-3-methoxyphenyl)-1-hydroxymethyl]phenyl]-1H-pyrrol-2-yl]acryloxy]piperidin-4-yl]acetate

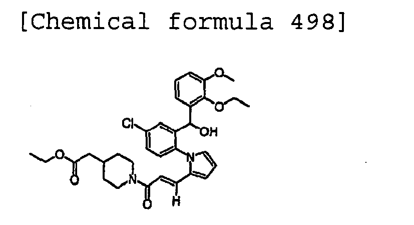

3-[1-[4-Chloro-2-[(2-ethoxy-3-methoxyphenyl)hydroxymethyl]phenyl]-1H-pyrrol-2-yl]acrylic acid (454 mg) and 1-hydroxybenzotriazole (211 mg) were dissolved in dichloromethane (10 mL). The resulting solution was stirred at room temperature for 10 minutes. Ethyl piperidin-4-yl-acetate (236 mg) and 1-ethyl-3-(3-dimethylaminopropyl)carbodiimide hydrochloride.(265 mg) were added and the resulting mixture was stirred at room temperature for 18 hours. The reaction mixture was partitioned between water and dichloromethane. The water layer was extracted with dichloromethane. The organic layers were combined, washed with saturated brine and dried over anhydrous sodium sulfate. The solvent was distilled off under reduced pressure. The residue thus obtained was purified by a Yamazen medium pressure preparative chromatogram system (chloroform:methanol= from 95:5 to 90:10) to give the title compound (581 mg).
MS (ESI) m/z : 581 (M⁺+H) .

### Example 211

Ethyl 2-[1-[2-[8-chloro-6-(2-ethoxy-3-methoxyphenyl)-4H,6H-pyrrolo[1,2-a][4,1]benzoxazepin-4-yl]acetyl]4-piperidinyl]acetate

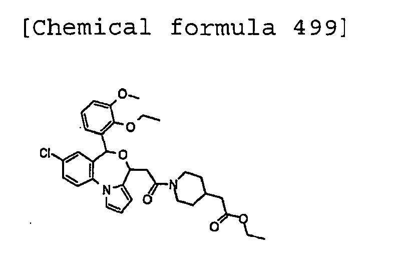

Ethyl [1-[3-[1-[4-chloro-2-[1-(2-ethoxy-3-methoxyphenyl)-1-hydroxymethyl]phenyl]-1H-pyrrol-2-yl]acryloxy]piperidin-4-yl]acetate (242 mg) was dissolved in dichloromethane (10 mL). Anhydrous aluminum chloride (11.1 mg) was added and the resulting mixture was stirred at room temperature for 63 hours. The reaction mixture was neutralized with a saturated aqueous solution of sodium hydrogen carbonate, followed by extraction three times with dichloromethane. The organic layers were combined, washed with saturated brine and dried over anhydrous sodium sulfate. The residue obtained by distilling off the solvent was purified by silica gel column chromatography (ethyl acetate:hexane=3:2) to give the title compound (77.2 mg).
MS(ESI)m/z:581(M⁺+H).
¹H-NMR(CDCl₃)δ:0.82(3H,dt,J=7.1,7.1Hz),1.04-1.22(2H,m),1.26(3H,dt,J=1.5,7.1Hz),1.72-2.26(4H,m),2.54-2.69(1H,m),2.86(1H,ddd,J=22.9,14.2,4.8Hz),2.97-3.12(1H,m),3.26(1H,dt,J=14.2,8.3Hz),3.43-3.51(2H,m),3.80-3.87(1H,m),3.83(3H,s),4.04(1H,d,J=13.2Hz),4.13(2H,dq,J=4.2, 7.1Hz),4.67(1H,t,J=16.2Hz),4.95(1H,ddd,J=19.2,8.6,4.8Hz),5. 73(1H,d,J=14.6Hz),6.23-6.26(1H,m),6.36(1H,t,J=3.2Hz),6.71(1H,dd,J=10.9,1.8Hz),6.91 -6.94(1H,m),7.06-7.07(1H,m),7.15(1H,t,J=8.1Hz),7.28-7.37(3H,m).
IR(ATR)cm⁻
¹:2931,1729,1635,1488,1276,1151,1097,1027,713,457.

### Example 212

2-[1-[2-[8-Chloro-6-(2-ethoxy-3-methoxyphenyl)-4H,6H-pyrrolo[1,2-a][4,1]benzoxazepin-4-yl]acetyl]4-piperidinyl]acetic acid

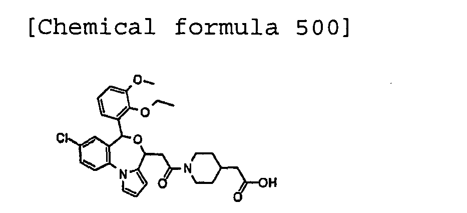

Ethyl 2-[1-[2-[8-chloro-6-(2-ethoxy-3-methoxyphenyl)-4H,6H-pyrrolo[1,2-a][4,1]benzoxazepin-4-yl]acetyl]4-piperidinyl]acetate (79.4 mg) was dissolved in methanol (5 mL). To the resulting solution were added potassium carbonate (56.7 mg) and water (5 mL). The resulting mixture was stirred at room temperature for one hour, at 60°C for 6 hours and then at room temperature for 18 hours. The reaction mixture was concentrated under reduced pressure. The residue was neutralized with a 1N aqueous hydrochloric acid solution, followed by extraction three times with dichloromethane. The organic layers were combined, washed with saturated brine, and dried over anhydrous sodium sulfate. The residue obtained by distilling off the solvent was triturated with hexane-ether to give the title compound (57.9 mg).
MS (ESI) m/z : 553 (M⁺+H). ¹H-NMR(CDCl₃)δ:0.77-2.33(5H,m)1.21(3H,t,J=7.0Hz)2.53-2.72(1H,m),2.78-2.93(1H,m),2.95-3.13(1H,m),3.22-3.32(1H,m),3.48(2H,q,J=7.0Hz),3.80-3.84(2H,m),3.83(3H,s),4.02-4.09(1H,m),4.61-4.74(1H,m),4.89-5.00(1H,m),5.74(1H,d,J=16.6Hz),6.24-6.26(1H,m),6.35-6.37(1H,m),6.69-6.74(1H,m),6.91-6.95(1H,m),7.06-7.08(1H,m),7.14(1H,t,J=8.1Hz),7.26-7.35(3H,m).
IR(ATR)cm⁻¹:2929,1722,1587,1488,1276,1027,713,457.
Elemental analysis for: C₃₀H₃₃ClN₂O₆·0.33H₂O
Calculated: C,64.46;H,6.07;N,5.01.
Found: C,64.58;H,6.38;N,4.74.

### Referential Example 121

(5-Chloro-2-pyrrol-1-yl-phenyl)-(3-methoxyphenyl)methanol

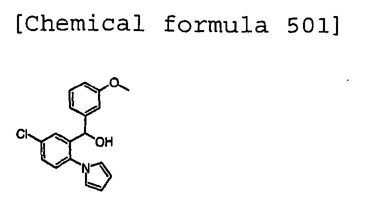

1-(2-Bromo-4-chlorophenyl)-1H-pyrrole (2.00 g) was dissolved in ether (50 mL). At -78°C, n-butyl lithium (1.6M hexane solution) (6.30 mL) was added dropwise to the resulting solution. The reaction mixture was stirred under ice cooling for 1 hour and then, at room temperature for 5 minutes. The reaction mixture was cooled to -78°C. 3-Methoxybenzaldehyde (1.20 mL) was added and the resulting mixture was stirred at room temperature for 1 hour. Ethyl acetate and water were added to the reaction mixture. The water layer was extracted three times with ethyl acetate. The organic layers were combined, washed with saturated brine, and dried over anhydrous sodium sulfate. The solvent was distilled off under reduced pressure. The residue thus obtained was purified by a Yamazen medium pressure preparative chromatogram system (hexane:ethyl acetate= from 75:25 to 67:33) to give the title compound (1.51 g) .
MS (ESI)m/z:314 (M⁺+H) .
¹H-NMR(CDCl₃)δ:2.16(1H,d,J=3.9Hz),3.76(3H,s),5.76(1H,d,J=3.9Hz ),6.29(2H,t,J=2.0Hz),6.65-6.68(3H,m),6.70(1H,d,J=7.6Hz),6.78(1H,dd,J=8.4,1.7Hz),7.16-7.22 ( 2H, m), 7.30 (1H, dd, J=8.4, 2.3Hz), 7.68 (1H, d, J=2 . 3Hz).
- IR(ATR)cm⁻¹:2834,1596,1494,1255,1016,925,823,723,501.

### Referential Example 122

(5-Chloro-2-pyrrol-1-yl-phenyl)-(3-methoxyphenyl)methanone

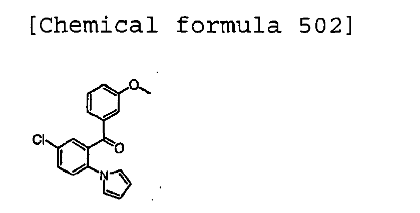

(5-Chloro-2-pyrrol-1-yl-phenyl)-(3-methoxyphenyl)methanol was dissolved in dichloromethane. Manganese dioxide (1.51 g) was added and the resulting mixture was heated under reflux for 5 hours. The reaction mixture was stirred at room temperature for 13 hours. Manganese dioxide (1.00 g) was added and the resulting mixture was heated under reflux for 1.5 hours. The reaction mixture was filtered through celite and the filtrate was concentrated under reduced pressure to give the title compound (1.28 g).
MS(ESI)m/z:312(M⁺+H).
¹H-NMR(CDCl₃)δ:3.79(3H,s),6.05(2H,t,J=2.1Hz),6.68(2H,t,J=2.1Hz ),7.00-7.03(1H,m),7.09-7.12(1H,m),7.19(1H,d,J=8.1Hz),7.20-7.22(1H,m),7.38(1H,d,J=8.3Hz),7.50(1H,d,J=2.5Hz),7.55(1H,dd ,J=8.3,2.5Hz). IR(ATR)cm⁻
¹:3010,1664,1592,1482,1324,1282,1108,1033,877,819,727.

### Referential Example 123

1-[4-Chloro-2-(3-methoxybenzoyl)phenyl]-1H-pyrrol-2-carbaldehyde 1-[4-Chloro-2-(3-methoxybenzoyl)phenyl]-1H-pyrrol-3-carbaldehyde

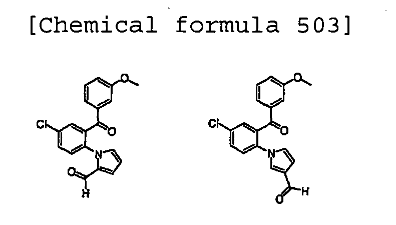

Dimethylformamide (0.636 mL) was dissolved in dichloroethane (10 mL). Under ice cooling, phosphorus oxychloride (0.459 mL) was added. The resulting mixture was stirred at room temperature for 10 minutes. After ice cooling again, a dichloroethane solution (10 mL) of (5-chloro-2-pyrrol-1-yl-phenyl)-(3-methoxyphenyl)methanone (1.28 g) was added dropwise. The reaction mixture was heated under reflux for 2 hours and then cooled to room temperature. A saturated aqueous solution (12 mL) of sodium acetate was added. The resulting mixture was heated under reflux for one hour and then cooled to room temperature. The reaction mixture was partitioned between dichloromethane and water. The water layer was extracted with dichloromethane. The organic layers were combined, washed with saturated brine and dried over anhydrous sodium sulfate. The solvent was distilled off under reduced pressure. The residue thus obtained was purified by a Yamazen medium pressure preparative chromatogram system (hexane:ethyl acetate= from 80:20 to 70:20) to give the title compound (2-substituted pyrrole) (590 mg) and a position isomer (3-substituted pyrrole) (470 mg). 2-Substituted pyrrole
MS(ESI)m/z:340(M⁺+H).
¹H-NMR(CDCl₃)δ:3.78(3H,s),6.21-6.23(1H,m), 6.86-6.87(1H,m),6.90-6.92(1H,m),7.03(1H,dt,J=7.4,2.0Hz),7.18-7.24(3H,m),7.34(1H,d,J=8.3Hz),7.53-7.58(2H,m),9.39(1H,br s).
IR(ATR)cm⁻¹:2834,1662,1482,1413,1282,1035,746,680,603,539. 3-Substituted pyrrole
MS(ESI)m/z:340(M⁺+H).
¹H-NMR(CDCl₃)δ:3.80(3H,s),6.53(1H,dd,J=2.9,1.5Hz),6.70-6.71(1H,m),7.05(1H,ddd,J=8.2,2.7,1.3Hz),7.10(1H,dt,J=7.8,1.
3Hz),7.20-7.25(2H,m),7.29(1H,t,J=1.7Hz),7.40(1H,d,J=8.6Hz),7.56(1H,d, J=2.4Hz),7.62(1H,dd,J=8.6,2.4Hz),9.65(1H,s).

### Referential Example 124

Methyl 3-[1-[4-chloro-2-(3-methoxybenzoyl)phenyl]-1H-pyrrol-2-yl]acrylate

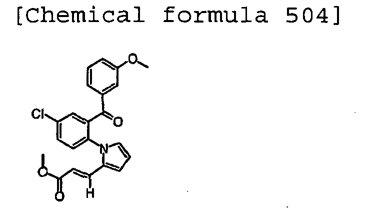

1-[4-Chloro-2-(3-methoxybenzoyl)phenyl]-1H-pyrrol-2-carbaldehyde (590 mg) was dissolved in toluene (20 mL). Methyl (triphenylphosphoranylidene)acetate (1.16 g) was added and the resulting mixture was heated under reflux for 4 hours. Methyl (triphenylphosphoranylidene)acetate (1.00 g) was added, followed by heating under reflux for further one hour. The reaction mixture was then cooled to room temperature. The residue obtained by distilling off the solvent was purified by silica gel column chromatography (hexane:ethyl acetate=from 4:1 to 3:2) to give the title compound (690 mg).
MS(ESI)m/z:418(M⁺+Na).
¹H-NMR(CDCl₃)δ:3.72(3H,s),3.77(3H,s),5.94(1H,d,J=15.9Hz),6.11( 1H,dd,J=3.4,2.9Hz),6.53(1H,dd,J=3.8,1.3Hz),6.73-6.74(1H,m),7.00-7.03(1H,m),7.04-7.07(1H,m),7.13-7.15(1H,m),7.19(1H,t,J=7.9Hz),7.25(1H,d,J=15.9Hz),7.33(1H,d ,J=8.4Hz),7.59(1H,d,J=2.4Hz),7.62(1H,dd,J=8.4,2.4Hz).
IR(ATR)cm⁻¹:2946,1700,1666,1623,1482,1168,1035,721,524.

### Referential Example 125

Methyl 3-[1-[4-chloro-2-[(3-methoxyphenyl)hydroxymethyl]phenyl]-1H-pyrrol-2-yl]acrylate

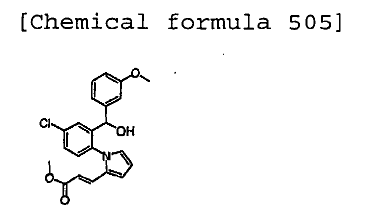

Methyl 3-[1-[4-chloro-2-(3-methoxybenzoyl)phenyl]-1H-pyrrol-2-yl]acrylate (690 mg) was dissolved in methanol (50 mL). Under ice cooling, sodium borohydride (99.0 mg) was added and the resulting mixture was stirred at room temperature for 1.5 hours. After acetone (10 mL) was added and the resulting mixture was stirred for 10 minutes, the solvent was distilled off under reduced pressure. Ethyl acetate and saturated ammonium chloride were added to the residue. The water layer was extracted with ethyl acetate. The organic layers were combined, washed with saturated brine and then, dried over anhydrous sodium sulfate. The solvent was distilled off to give the title compound (660 mg).
MS(ESI)m/z:380(M⁺-OH). IR(ATR)cm⁻
¹:3423,2948,1685,1623,1484,1241,1168,1033,723,607.

### Referential Example 126

3-[1-[4-Chloro-2-[(3-methoxyphenyl)hydroxymethyl]phenyl]-1H-pyrrol-2-yl]acrylic acid

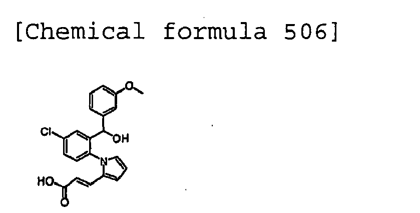

Methyl 3-[1-[4-chloro-2-[(3-methoxyphenyl)hydroxymethyl]phenyl]-1H-pyrrol-2-yl]acrylate (440 mg) was dissolved in methanol (10 mL). To the resulting solution were added potassium carbonate (459 mg) and water (10 mL). The resulting mixture was stirred at 60°C for 3.5 hours. The reaction mixture was concentrated under reduced pressure. The residue was neutralized with a 1N aqueous hydrochloric acid solution, followed by extraction with ethyl acetate. The organic layers were combined, washed with saturated brine and then, dried over anhydrous sodium sulfate. The solvent was distilled off to give the title compound (426 mg).
MSESIm/z:366(M⁺-OH).

### Referential Example 127

Ethyl [1-[3-[1-[4-chloro-2-[1-(3-methoxyphenyl)-1-hydroxymethyl]phenyl]-1H-pyrrol-2-yl]acryloxy]piperidin-4-yl]acetate

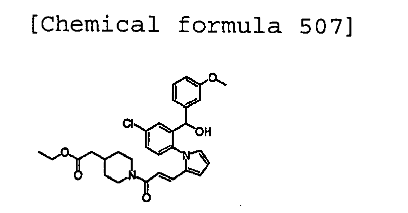

3-[1-[4-Chloro-2-[(3-methoxyphenyl)hydroxymethyl]phenyl]-1H-pyrrol-2-yl]acrylic acid (426 mg) and 1-hydroxybenzotriazole (221 mg) were dissolved in dichloromethane (10 mL) and the resulting solution was stirred at room temperature for 10 minutes.
To the reaction mixture were added ethyl piperidin-4-yl-acetate (247 mg) and 1-ethyl-3-(3-dimethylaminopropyl)carbodiimide hydrochloride (276 mg), followed by stirring at room temperature for 18 hours. The reaction mixture was partitioned between water dichloromethane and the water layer was extracted with dichloromethane. The organic layers were combined, washed with saturated brine and, dried over anhydrous sodium sulfate. The solvent was distilled off under reduced pressure. The residue thus obtained was purified by a Yamazen medium pressure preparative chromatogram system (chloroform:methanol=95:5) to give the title compound (600 mg).
MS(ESI)m/z:537(M⁺+H). IR (ATR) cm⁻
¹:2935,1727,1627,1585,1484,1257,1095,1029,719,607.

### Example 213

Ethyl 2-[1-[2-[8-chloro-6-(3-methoxyphenyl)-4H,6H-pyrrolo[1,2-a][4,1]benzoxazepin-4-yl]acetyl]4-piperidinyl]acetate

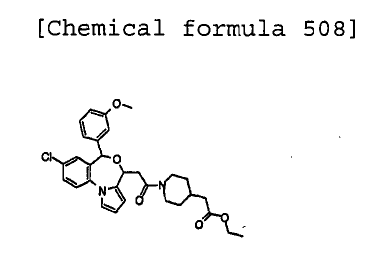

Ethyl [1-[3-[1-[4-chloro-2-[1-(3-methoxyphenyl)-1-hydroxymethyl]phenyl]-1H-pyrrol-2-yl]acryloxy]piperidin-4-yl]acetate (248 mg) was dissolved in dichloromethane (10 mL). Trifluoroacetic acid (0.105 mL) was added and the resulting mixture was stirred at room temperature for 7 hours. The reaction mixture was neutralized with a saturated aqueous solution of sodium hydrogen carbonate, followed by extraction with dichloromethane three times. The organic layers were combined, washed with saturated brine and dried over anhydrous sodium sulfate. The residue obtained by distilling off the solvent was purified by thin layer silica gel column chromatography (development with tetrahydrofuran:hexane=1:2) to give the title compound (77.4 mg) .
MS(ESI)m/z:537(M⁺+H). ¹H-NMR(CDCl₃)δ:1.06-2.28(8H,m),2.59-2.63(2H,m),2.78-2.92(1H,m),2.97-3.12(1H,m),3.20-3.32(1H,m),3.68-3.74(1H,m),3.81(3H,s),3.99-4.10(1H,m),4.14(2H,q,J=7.1Hz),4.59-4.73(1H,m),4.94-5.02(1H,m),5.39(1H,d,J=3.4Hz),6.25(1H,br s),6.37(1H,t,J=3.1Hz),6.55-6.64(1H,m),6.67-6.73(1H,m),6.86-6.92(2H,m),7.06-7.13(1H,m),7.28-7.38(3H,m).
IR(ATR)cm⁻¹:2933,1727,1633,1486,1151,1097,1027,717,551,462.

### Example 214

2-[1-[2-[8-Chloro-6-(3-methoxyphenyl)-4H,6H-pyrrolo[1,2-a][4,1]benzoxazepin-4-yl]acetyl]4-piperidinyl]acetic acid

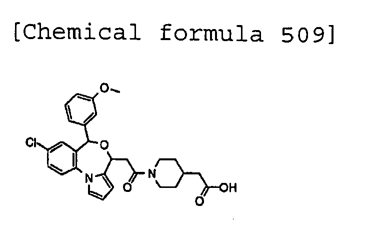

Ethyl 2-[1-[2-[8-chloro-6-(3-methoxyphenyl)-4H,6H-pyrrolo[1,2-a][4,1]benzoxazepin-4-yl]acetyl]4-piperidinyl]acetate (77.4 mg) was dissolved in methanol (5 mL). Potassium carbonate (59.8 mg) and water (5 mL) were added and the resulting mixture was stirred at 60°C for 4.5 hours. The reaction mixture was concentrated under reduced pressure. The residue thus obtained was neutralized with a 1N aqueous hydrochloric acid solution, followed by extraction with dichloromethane three times. The organic layers were combined, washed with saturated brine and dried over anhydrous sodium sulfate. The residue obtained by distilling off the solvent was triturated with dichloromethane-hexane-ether to give the title compound (53.9 mg).
MS(ESI)m/z:509(M⁺+H). ¹H-NMR(CDCl₃)δ:0.84-2.29(5H,m),2.51-2.69(2H,m),2.77-2.92(1H,m),2.99-3.12(1H,m),3.22-3.33(1H,m),3.69-3.71(1H,m),3.82(3H,s),4.00-4.10(1H,m),4.60-4.73(1H,m),4.92-5.03(1H,m),5.39-5.40(1H,m),6.24-6.26(1H,m),6.36-6.38(1H,m),6.58-6.63(1H,m),6.67-6.77(1H,m),6.86-6.92(2H,m),7.06-7.13(1H,m),7.28-7.36(3H,m). IR(ATR)cm⁻
¹:2923,1720,1585,1486,1257,1151,1097,1039,717,551,464.

### Example 215

Methyl 3-((4R,65)-8-chloro-1-cyano-6-(2,3-dimethoxyphenyl)-4H,6H-pyrrolo(1,2-a)(4,1)benzoxazepin-4-yl)propanoate

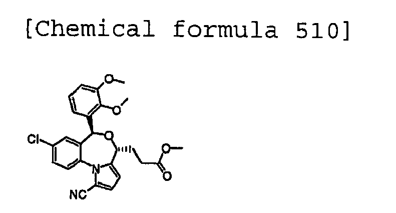

To an acetonitrile solution (20.0 mL) of methyl 3-((4R,6S)-8-chloro-6-(2,3-dimethoxyphenyl)-4H,6H-pyrrolo(1,2-a)(4,1)benzoxazepin-4-yl)propanoate (2.67 g) was added N,N-dimethylformamide (20.0 mL). At -40°C, chlorosulfonyl isocyanate (789 µl) was added. The resulting mixture was stirred at the same temperature for 20 minutes and then under ice cooling for one hour. After addition of water, the resulting mixture was extracted with ethyl acetate. The organic layer was washed with saturated brine and dried over anhydrous sodium sulfate. The solvent was distilled off and the title compound (3.06 g).
MS(ESI)m/z:467(M⁺+H).
¹H-NMR(CDCl₃)δ:2.99-3.14(2H,m),3.45(3H,s),3.72(3H,s),3.86(3H,s),4.79(1H,t,J=7.1 Hz),5.61(1H,s),6.35(1H,d,J=4.2Hz),6.80(1H,d,J=2.4Hz),6.96(1 H,dd,J=7.1,2.4Hz),7.07(1H,d,J=4.2Hz),7.15-7.21(2H,m),7.48-7.51(1H,m),7.61(1H,d,J=8.5Hz).

### Example 216

Methyl 3-((4R,6S)-8-chloro-6-(2,3-dimethoxyphenyl)-1-formyl-4H,6H-pyrrolo(1,2-a)(4,1)benzoxazepin-4-yl)propanoate

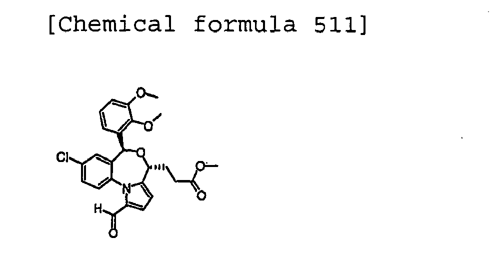

To a solution of methyl 3-((4R,6S)-8-chloro-1-cyano-6-(2,3-dimethoxyphenyl)-4H,6H-pyrrolo(1,2-a)(4,1)benzoxazepin-4-yl)propanoate (252 mg) in a mixture of pyridine (4.0 mL), acetic acid (4.0 mL) and distilled water(4.0 mL) was added Raney nickel R-100 (2.0 mL). The resulting mixture was subjected to catalytic hydrogenation at 5.0 atmospheres for 15 hours. The catalyst was filtered out and the solvent was distilled off under reduced pressure. Ethyl acetate was added. The resulting mixture was washed with a 10% aqueous citric acid solution. The organic layer was dried over anhydrous sodium sulfate. The residue obtained by distilling off the solvent was purified by preparative thin layer chromatography (developed with 1:2=ethyl acetate:n-hexane) to give the title compound (117 mg) .
MS(ESI)m/z:471(M⁺+H). ¹H-NMR(CDCl₃)δ:2.29-2.49(2H,m),2.53-2.61(1H,m),2.64-2.74(1H,m),3.39(3H,s),3.67(3H,s),3.85(3H,s),4.21(1H,dd,J=9. 3,3.9Hz),5.69(1H,s),6.46(1H,d,J=4.2Hz),6.76(1H,d,J=2.0Hz),6 .96(1H,d,J=6.6Hz),7.16-7.26(3H,m),7.38-7.47(2H,m), ,9.72(1H,s).

### Example 217

Methyl 3-((4R,6S)-8-chloro-6-(2,3-dimethoxyphenyl)-1-(hydroxymethyl)-4H,6H-pyrrolo(1,2-a)(4,1)benzoxazepin-4-yl)propanoate

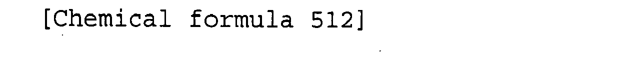

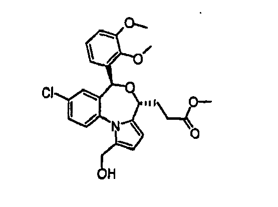

To a tetrahydrofuran solution (10.0 mL) of methyl 3-((4R,6S)-8-chloro-6-(2,3-dimethoxyphenyl)-1-formyl-4H,6H-pyrrolo(1,2-a)(4,1)benzoxazepin-4-yl)propanoate (163 mg) was added sodium borohydride (13 mg). The resulting mixture was stirred at room temperature for 2 hours.
Sodium borohydride (13 mg) was added further and the resulting mixture was stirred for 4 hours. A 10% aqueous citric acid solution was added and the resulting mixture was extracted with ethyl acetate. The organic layer was dried over anhydrous sodium sulfate. The residue obtained by distilling off the solvent was purified by preparative thin layer chromatography (developed with 1:2=ethyl acetate:n-hexane) to give the title compound (156 mg).
MS(ESI)m/z:472 (M⁺+H) .
¹H-NMR(CDCl₃) δ:2.27-2.72(4H,m),3.37(H,s),3.66(3H,s),3.84(3H,s),4.27(1H,dd,J=9.0 ,4.6Hz),4.45(1H,d,J=12.9Hz),4.82(1H,d,J=12.9Hz),5.51(1H,s), 6.27(1H,d,J=3.5Hz),6.38(1H,d,J=3.5Hz),6.74(1H,d,J=2.4Hz),6. 94(1H,dd,J=8.3,1.5Hz),7.18(1H,t,J=7.9Hz),7.26-7.29(1H,m),7.39(1H,dd,J=8.3,2.4Hz),7.77(1H,d,J=8.3Hz).

### Example 218

3-((4R,6S)-8-Chloro-6-(2,3-dimethoxyphenyl)-1-(hydroxymethyl)-4H,6H-pyrrolo(1,2-a)(4,1)benzoxazepin-4-yl)propanoic acid

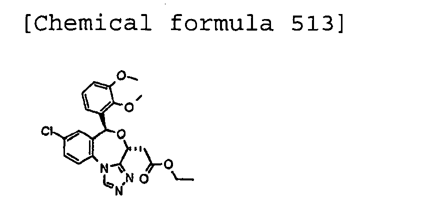

To a methanol solution (20.0 mL) of methyl 3-((4R,6S)-8-chloro-6-(2,3-dimethoxyphenyl)-1-(hydroxymethyl)-4H,6H-pyrrolo(1,2-a)(4,1)benzoxazepin-4-yl)propanoate (156 mg) were added distilled water (10.0 mL) and potassium carbonate (137 mg). The resulting mixture was stirred at 50°C for 3 hours. A 10% aqueous citric acid solution was added and the resulting mixture was extracted with ethyl acetate. The organic layer was dried over anhydrous sodium sulfate. The solvent was distilled off to give the title compound (164 mg).
MS(ESI)m/z:458 (M⁺+H).
¹H-NMR(CDCl₃)δ:2.25-2.47(2H,m),2.54-2.75(2H,m),3.37(3H,s),3.83(3H,s),4.28(1H,dd,J=9.3,4.6Hz),4. 45(1H,d,J=12.9Hz),4.82(1H,d,J=12.9Hz),5.52(1H,s),6.26(1H,d, J=3.7Hz),6.38(1H,d,J=3.7Hz),6.74(1H,d,J=2.3Hz),6.93(1H,dd,J =8.4,1.5Hz),7.17(1H,t,J=7.9Hz),7.26-7.29(1H,m),7.39(1H,dd,J=8.4,2.3Hz),7.76(1H,d,J=8.4Hz).

### Example 219

Ethyl 2-(1-(3-((4R,6S)-8-chloro-6-(2,3-dimethoxyphenyl)-1-(hydroxymethyl)-4H,6H-pyrrolo(1,2-a)(4,1)benzoxazepin-4-yl)propanoyl)-4-piperidinyl)acetate

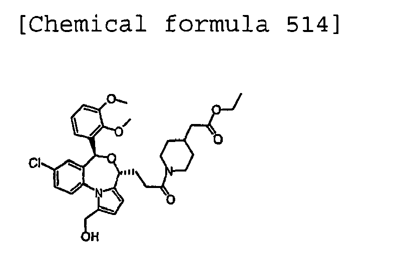

To a solution of 3-((4R,6S)-8-chloro-6-(2,3-dimethoxyphenyl)-1-(hydroxymethyl)-4H,6H-pyrrolo(1,2-a)(4,1)benzoxazepin-4-yl)propanoic acid (70 mg) in methylene chloride (5.0 mL) were added ethyl piperidin-4-yl-acetate (31 mg), 1-ethyl-3-(3-dimethylaminopropyl)carbodiimide hydrochloride (58 mg), and 1-hydroxybenzotriazole (23 mg). The resulting mixture was stirred at room temperature for 14 hours. The reaction mixture was purified by preparative thin layer chromatography (development phase: 5% methanol-chloroform) to give the title compound (79 mg).
MS (ESI)m/z:611(M⁺+H).
¹H-NMR(CDCl₃)δ:1.01-1.18(2H,m),1.26(3H,t,J=7.2Hz),1.74(2H,t,J=13.7Hz),1.94-2.06(1H,m),2.17-2.25(2H,m),2.34-2.41(2H,m),2.46-2.71(3H,m),2.94-3.07(1H,m),3.36(3H,s),3.82-3.94(4H,m),4.13(2H,q,J=7.2Hz),4.25-4.31(1H,m),4.43(1H,d,J=13.0Hz),4.54-4.63(1H,m),4.81(1H,d,J=13.0Hz),5.51(1H,s),6.27(1H,d,J=3.7Hz ),6.37(1H,d,J=3.7Hz),6.73(1H,d,J=2.4Hz),6.92-6.96(1H,m),7.17(1H,t,J=8.1Hz),7.24-7.26(1H,m),7.38(1H,dd,J=8.5,1.7Hz),7.78(1H,d,J=8.3Hz).

### Example 220

2-(1-(3-((4R,6S)-8-Chloro-6-(2,3-dimethoxyphenyl)-1-(hydroxymethyl)-4H,6H-pyrrolo(1,2-a)(4,1)benzoxazepin-4-yl)propanoyl)-4-piperidinyl)acetic acid

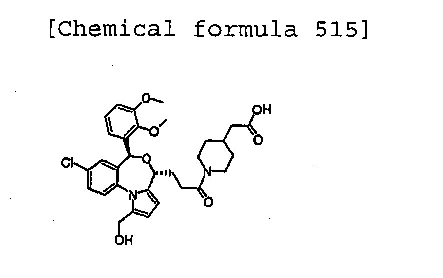

To a methanol solution (14.0 mL) of ethyl 2-(1-(3-((4R,6S)-8-chloro-6-(2,3-dimethoxyphenyl)-1-(hydroxymethyl)-4H,6H-pyrrolo(1,2-a)(4,1)benzoxazepin-4-yl)propanoyl)-4-piperidinyl)acetate (79 mg) were added distilled water (7.0 mL) and potassium carbonate (54 mg). The resulting mixture was stirred at room temperature for 16 hours. A 10% aqueous citric acid solution was added and the resulting mixture was extracted with ethyl acetate.
The organic layer was dried over anhydrous sodium sulfate. The residue obtained by distilling off the solvent was purified by preparative thin layer chromatography (development phase: 10% methanol-chloroform), followed by lyophilization from 1,4-dioxane-water to give the title compound (47 mg).
MS (ESI) m/z : 583 (M⁺+H).
¹H-NMR(CDCl₃)δ:1.01-1.19(2H,m),1.71-1.83(2H,m),1.94-2.05(1H,m),2.22-2.30(2H,m),2.34-2.42(2H,m),2.49-2.70(3H,m),2.94-3.07(1H,m),3.37(3H,s),3.82-3.94(4H,m),4.25-4.31(1H,m),4.45(1H,d,J=12.9Hz),4.59(1H,d,J=12.0Hz),4.83(1H, d,J=12.9Hz),5.50(1H,s),6.29(1H,d,J=3.5Hz),6.39(1H,d,J=3.5Hz ),6.73(1H,d,J=2.1Hz),6.94(1H,dd,J=8.0,1.2Hz),7.17(1H,t,J=8. 0Hz),7.24-7.28(1H,m),7.39(1H,dd,J=8.6,2.1Hz),7.78(1H,d,J=8.6Hz).

### Example 221

Ethyl 2-(1-(3-((4R,6S)-1-(azidemethyl)-8-chloro-6-(2,3-dimethoxyphenyl)-4H,6H-pyrrolo(1,2-a)(4,1)benzoxazepin-4-yl)propanoyl)-4-piperidinyl)acetate

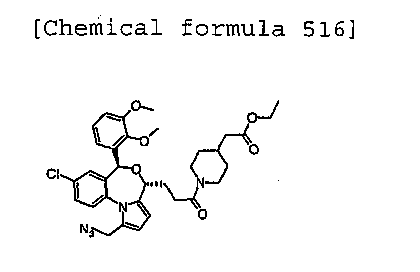

To an N,N-dimethylformamide solution (2.0 mL) of ethyl 2-(1-(3-((4R,6S)-8-chloro-6-(2,3-dimethoxyphenyl)-1-(hydroxymethyl)-4H,6H-pyrrolo(1,2-a)(4,1)benzoxazepin-4-yl)propanoyl)-4-piperidinyl)acetate (306 mg) were added triphenylphosphine (393 mg) and carbon tetrabromide (497 mg) under ice cooling. The resulting mixture was stirred at room temperature for 1 hour. Sodium azide (49 mg) was added and the resulting mixture was stirred at room temperature for 15 hours. Ethyl acetate was added to the reaction mixture. The resulting mixture was washed with saturated brine and dried over anhydrous sodium sulfate. The solvent was then distilled off. The residue was purified by silica gel chromatography (ethyl acetate:n-hexane=1:1) to give the title compound (69 mg).
MS(ESI)m/z:636(M⁺+H).
¹H-NMR(CDCl₃)δ:1.01-1.19(2H,m),1.26(3H,t,J=7.2Hz),1.68-1.80(2H,m),2.18-2.25(2H,m),2.34-2.42(2H,m),2.46-2.69(3H,m),2.95-3.08(1H,m),3.35(3H,s),3.38(3H,s),3.83-3.94(4H,m),4.13(2H,q,J=7.2Hz),4.24-4.29(2H,m),4.30(1H,d,J=12.4Hz),4.50(1H,d,J=12.4Hz),4.55-4.63(1H,m),5.53(1H,s),6.30(1H,d,J=3.7Hz),6.42(1H,d,J=3.7Hz) ,6.73(1H,d,J=2.4Hz),6.94(1H,dd,J=8.3,1.5Hz),7.17(1H,t,J=7.9 Hz),7.25-7.29(1H,m),7.38(1H,dd,J=8.3,2.4Hz),7.67(1H,d,J=8.3Hz).

### Example 222

Ethyl 2-(1-(3-((4R,6S)-1-((acetylamino)methyl)-8-chloro-6-(2,3-dimethoxyphenyl)-4H,6H-pyrrolo(1,2-a)(4,1)benzoxazepin-4-yl)propanoyl)-4-piperidinyl)acetate

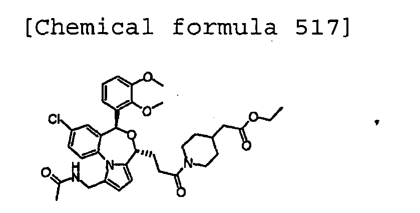

To a tetrahydrofuran solution (1.0 mL) of ethyl 2-(1-(3-((4R,6S)-1-(azidemethyl)-8-chloro-6-(2,3-dimethoxyphenyl)-4H,6H-pyrrolo(1,2-a)(4,1)benzoxazepin-4-yl)propanoyl)-4-piperidinyl)acetate (69 mg) were added distilled water (0.20 mL) and triphenylphosphine (43 mg). The resulting mixture was stirred at room temperature for 3 days. After the solvent was distilled off, the residue was dissolved in methylene chloride (5.0 mL). To the resulting solution were added pyridine (1.0 mL) and acetic anhydride (0.51 mL). The resulting mixture was stirred at room temperature for 27 hours. The reaction mixture was concentrated. The concentrate was diluted with ethyl acetate and washed successively with a 10% aqueous citric acid solution and saturated brine. The organic layer was dried over anhydrous sodium sulfate. The solvent was distilled off. The residue was purified by preparative thin layer chromatography (development phase: ethyl acetate) to give the title compound (29 mg).
MS (ESI) m/z : 652 (M⁺+H). ¹H-NMR(CDCl₃)δ:1.02-1.19(2H,m),1.26(3H,t,J=7.1Hz),1.65-1.83(2H,m),1.89(3H,s),1.95-2.07(1H,m),2.16-2.26(2H,m),2.33-2.40(2H,m),2.48-2.69(3H,m),3.43(3H,s),3.83-3.93(4H,m),4.13(2H,q,J=7.1Hz),4.22-4.28(1H,m),4.58(1H,d,J=4.9Hz),4.60(1H,s,5.48(1H,s),6.28(1H, d,J=3.5Hz),6.33(1H,d,J=3.5Hz),6.73(1H,d,J=2.2Hz),6.95(1H,dd ,J=8.2,1.5Hz),7.18(1H,t,J=8.2Hz),7.23-7.25(1H,m),7.32(1H,d,J=8.3Hz),7.37-7.41(1H,m).

### Example 223

2-(1-(3-((4R,6S)-1-((Acetylamino)methyl)-8-chloro-6-(2,3-dimethoxyphenyl)-4H,6H-pyrrolo(1,2-a)(4,1)benzoxazepin-4-yl)propanoyl)-4-piperidinyl)acetic acid

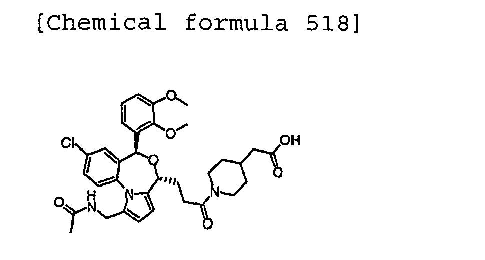

To a methanol solution of (6.0 mL) of ethyl 2-(1-(3-((4R,6S)-1-((acetylamino)methyl)-8-chloro-6-(2,3-dimethoxyphenyl)-4H,6H-pyrrolo(1,2-a)(4,1)benzoxazepin-4-yl)propanoyl)-4-piperidinyl)acetate (29 mg) were added distilled water (3.0 mL) and potassium carbonate (18 mg). The resulting mixture was stirred at 50°C for 6 hours. The reaction mixture was adjusted to pH 4 with an ion exchange resin IR-120B. The resin was filtered out and the solvent was distilled off. The residue was purified by preparative thin layer chromatography (development phase: 10% methanol-chloroform), followed by lyophilization from 1,4-dioxane-water to give the title compound (17 mg).
MS(ESI)m/z:624(M⁺+H).
¹H-NMR(CDCl₃)δ:1.01-1.20(2H,m),1.72-1.82(2H,m),1.93-2.06(1H,m),2.21-2.29(2H,m),2.34-2.41(2H,m),2.46-2.70(3H,m),2.95-3.07(1H,m),3.35(3H,s),3.38(3H,s),3.82-3.94(4H,m),4.23-4.29(1H,m),4.30(1H,d,J=12.6Hz),4.50(1H,d,J=12.6Hz),4.56-4.63(1H,m),5.52(1H,s),6.29(1H,d,J=3.7Hz),6.42(1H,d,J=3.7Hz) ,6.72(1H,d,J=2.4Hz),6.94(1H,dd,J=8.3,1.3Hz),7.17(1H,t,J=7.9 Hz),7.38(1H,dd,J=8.5,2.4Hz),7.67(1H,d,J=8.3Hz).

### Example 224

Ethyl 2-(1-(3-((4R,6S)-8-chloro-6-(2,3-dimethoxyphenyl)-1-(methoxymethyl)-4H,6H-pyrrolo(1,2-a)(4,1)benzoxazepin-4-yl)propanoyl)-4-piperidinyl)acetate

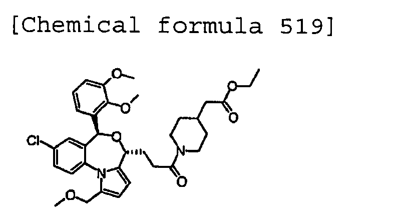

To a tetrahydrofuran solution (2.0 mL) of ethyl 2-(1-(3-((4R,6S)-8-chloro-6-(2,3-dimethoxyphenyl)-1-(hydroxymethyl)-4H,6H-pyrrolo(1,2-a)(4,1)benzoxazepin-4-yl)propanoyl)-4-piperidinyl)acetate (198 mg) were added 60% sodium hydride in oil (38 mg) and methyl iodide (0.09 mL). The resulting mixture was stirred at room temperature for 3 hours. After addition of a 10% aqueous citric acid solution, the resulting mixture was extracted with ethyl acetate. The organic layer was washed with saturated brine and dried over anhydrous sodium sulfate. The residue obtained by distilled off the solvent was purified by preparative thin layer chromatography (development phase: ethyl acetate:n-hexane=3:2) to give the title compound (58 mg).
MS(ESI)m/z:62S(M⁺+H) .
¹H-NMR(CDCl₃)δ:1.01-1.19(2H,m),1.26(3H,t,J=7.2Hz),1.68-1.80(2H,m),2.18-2.25(2H,m),2.34-2.42(2H,m),2.46-2.69(3H,m),2.95-3.08(1H,m),3.35(3H,s),3.38(3H,s),3.83-3.94(4H,m),4.13(2H,q,J=7.2Hz),4.24-4.29(2H,m),4.30(1H,d,J=12.4Hz),4.50(1H,d,J=12.4Hz),4.55-4.63(1H,m),5.53(1H,s),6.30(1H,d,J=3.7Hz),6.42(1H,d,J=3.7Hz) ,6.73(1H,d,J=2.4Hz),6.94(1H,dd,J=8.3,1.5Hz),7.17(1H,t,J=7.9 Hz),7.25-7.29(1H,m),7.38(1H,dd,J=8.3,2.4Hz),7.67(1H,d,J=8.3Hz).

### Example 225

2-(1-(3-((4R,6S)-8-Chloro-6-(2,3-dimethoxyphenyl)-1-(methoxymethyl)-4H,6H-pyrrolo(1,2-a)(4,1)benzoxazepin-4-yl)propanoyl)-4-piperidinyl)acetic acid

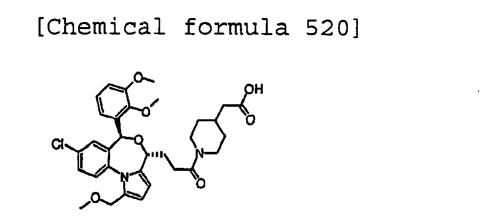

To a methanol solution (6.0 mL) of ethyl 2-(1-(3-((4R,6S)-8-chloro-6-(2,3-dimethoxyphenyl)-1-(methoxymethyl)-4H,6H-pyrrolo(1,2-a)(4,1)benzoxazepin-4-yl)propanoyl)-4-piperidinyl)acetate (68 mg) were added distilled water (3.0 mL) and potassium carbonate (43 mg). The resulting mixture was stirred at room temperature for 2 days. After addition of a 10% aqueous citric acid solution, the resulting mixture was extracted with ethyl acetate. The organic layer was washed with saturated brine and dried over anhydrous sodium sulfate. The solvent was then distilled off. The residue was purified by preparative thin layer chromatography (development phase: 10% methanol-chloroform), followed by lyophilization from 1,4-dioxane-water to give the title compound (61 mg).
MS(ESI)m/z:597(M⁺+H).
¹H-NMR(CDCl₃)δ:1.01-1.20(2H,m),1.72-1.82(2H,m),1.93-2.06(1H,m),2.21-2.29(2H,m),2.34-2.41(2H,m),2.46-2.70(3H,m),2.95-3.07(1H,m),3.35(3H,s),3.38(3H,s),3.82-3.94(4H,m),4.23-4.29(1H,m),4.30(1H,d,J=12.6Hz),4.50(1H,d,J=12.6Hz),4.56-4.63(1H,m),5.52(1H,s),6.29(1H,d,J=3.7Hz),6.42(1H,d,J=3.7Hz) ,6.72(1H,d,J=2.4Hz),6.94(1H,dd,J=8.3,1.3Hz),7.17(1H,t,J=7.9 Hz),7.38(1H,dd,J=8.5,2.4Hz),7.67(1H,d,J=8.3Hz).

### Example 226

Ethyl 2-(1-(3-((4R,6S)-8-chloro-1-cyano-6-(2,3-dimethoxyphenyl)-4H,6H-pyrrolo(1,2-a)(4,1)benzoxazepin-4-yl)propanoyl)-4-piperidinyl)acetate

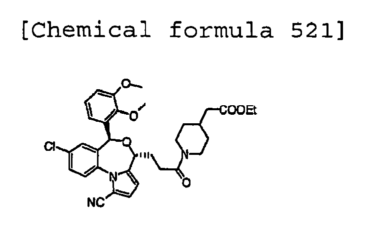

To an acetonitrile solution (1.0 mL) of ethyl 2-(1-(3-((4R,6S)-8-chloro-6-(2,3-dimethoxyphenyl)-4H,6H-pyrrolo(1,2-a)(4,1)benzoxazepin-4-yl)propanoyl)-4-piperidinyl)acetate (58 mg) was added N,N-dimethylformamide (1.0 mL). At -78°C, chlorosulfonyl isocyanate (8.7 µl) was added and the resulting mixture was stirred for 5 minutes under ice cooling. After addition of water, the resulting mixture was extracted with ethyl acetate. The organic layer was washed with saturated brine and dried over anhydrous sodium sulfate. The solvent was then distilled off. The residue was purified by preparative thin layer chromatography (development phase: 1:1=ethyl acetate:n-hexane) to give the title compound (29 mg) and raw material (25 mg).
MS(ESI)m/z:606(M⁺+H).
¹H-NMR (CDCl₃) δ:1.05-1.21(2H,m), 1.26(3H,t,J=7.1Hz),1.68-1.83(3H,m),1.96-2.06(1H,m),2.20-2.26(2H,m),2.34-2.43(2H,m),2.50-2.69(2H,m),3.03(1H,t,J=12.7Hz),3.44(3H,s),3.84-3.92(4H,m),4.14(2H,q,J=7.1Hz),4.28-4.33(1H,m),4.53-4.62(1H,m),5.60(1H,s),6.42(1H,d,J=4.2Hz),6.78(1H,d,J=2.2Hz) ,6.97(1H,dd,J=7.7,1.8Hz),7.07(1H,d,J=4.2Hz),7.16-7.23(2H,m),7.45-7.49(1H,m),7.59(1H,d,J=8.5Hz).

### Example 227

2-(1-(3-((4R,6S)-8-Chloro-1-cyano-6-(2,3-dimethoxyphenyl)-4H,6H-pyrrolo(1,2-a)(4,1)benzoxazepin-4-yl)propanoyl)-4-piperidinyl)acetic acid

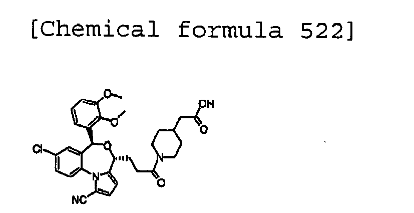

To a methanol solution (10.0 mL) of ethyl 2-(1-(3-((4R,6S)-8-chloro-1-cyano-6-(2,3-dimethoxyphenyl)-4H,6H-pyrrolo(1,2-a)(4,1)benzoxazepin-4-yl)propanoyl)-4-piperidinyl)acetate (29 mg) were added distilled water (5.0 mL) and potassium carbonate (20 mg). The resulting mixture was stirred at room temperature for 13 hours. The reaction mixture was adjusted to pH 4 with an ion exchange resin IR-120B. The resin was filtered out and the solvent was distilled off. The residue was purified by preparative thin layer chromatography (development phase: a 7:3:1=chloroform:methanol:water organic layer), followed by lyophilization from 1,4-dioxane-water to give the title compound (20 mg).
MS(ESI)m/z:578(M⁺+H). ¹H-NMR(CDCl₃)δ:1.05-1.20(2H,m),1.72-1.85(2H,m),2.26-2.41(4H,m),2.50-2.69(1H,m),2.97-3.07(1H,m),3.41-3.47(4H,m),3.84-3.92(5H,m),4.27-4.32(1H,m),4.54-4.62(1H,m),5.60(1H,s),6.41(1H,d,J=4.2Hz),6.78(1H,d,J=2.5Hz) ,6.97(1H,dd,J=7.7,1.6Hz),7.07(1H,d,J=4.2Hz),7.15-7.23(2H,m),7.47(1H,d,J=8.8Hz),7.59(1H,d,J=8.6Hz).

### Example 228

Methyl 3-((4R,6S)-8-chloro-1-cyano-6-(2,3-dimethoxyphenyl)-4H,6H-pyrrolo(1,2-a)(4,1)benzoxazepin-4-yl)propanoate

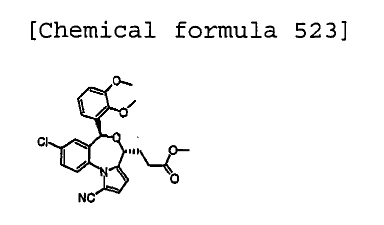

To an acetonitrile solution (5.0 mL) of methyl 3-((4R,6S)-8-chloro-6-(2,3-dimethoxyphenyl)-4H,6H-pyrrolo(1,2-a)(4,1)benzoxazepin-4-yl)propanoate (672 mg) was added N,N-dimethylformamide (5.0 mL). At -40°C, chlorosulfonyl isocyanate (199 µl) was added and the resulting mixture was stirred at the same temperature for 1.5 hours. Chlorosulfonyl isocyanate (199 µl) was added and the resulting mixture was stirred at -40°C for 45 minutes and then under ice cooling for 15 minutes. After addition of water, the resulting mixture was extracted with ethyl acetate. After the organic layer was washed with saturated brine and dried over anhydrous sodium sulfate, the solvent was distilled off to give the title compound (866 mg).
MS(ESI)m/z:467(M⁺+H) .
¹H-NMR(CDCl₃)δ:2.27-2.47(2H,m),2.52-2.72(2H,m),3.44(3H,s),3.66(3H,s),3.86(3H,s),4.28(1H,dd,J=9. 3,4.4Hz),5.60(1H,s),6.39(1H,d,J=4.2Hz),6.79(1H,d,J=2.4Hz),6 .96(1H,dd,J=8.0,1.7Hz),7.07(1H,d,J=4.2Hz),7.18(1H,t,J=8.0Hz ),7.23(1H,dd,J=8.0,1.7Hz),7.48(1H,dd,J=8.5,2.4Hz),7.60(1H,d ,J=8.5Hz).

### Example 229

Methyl 2-(1-(2-((4R,6S)-1-(aminocarbonyl)-8-chloro-6-(2,3-dimethoxyphenyl)-4H,6H-pyrrolo(1,2-a)(4,1)benzoxazepin-4-yl)acetyl)-4-piperidinyl)acetate

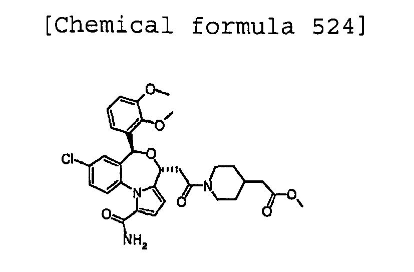

To an acetonitrile solution (1.0 mL) of methyl 2-(1-(2-((4R,6S)-8-chloro-6-(2,3-dimethoxyphenyl)-4H,6H-pyrrolo(1,2-a)(4,1)benzoxazepin-4-yl)acetyl)-4-piperidinyl)acetate (231 mg) was added N,N-dimethylformamide (1.0 mL). At -20°C, chlorosulfonyl isocyanate (40 µl) was added and the resulting mixture was stirred at the same temperature for 2.5 hours. Chlorosulfonyl isocyanate (40 µl) was added again and the resulting mixture was stirred for 1 hour. After addition of a saturated aqueous solution of sodium hydrogen carbonate, the resulting mixture was extracted with ethyl acetate. The organic layer was washed with saturated brine and dried over anhydrous sodium sulfate. The residue obtained by distilling off the solvent was purified by preparative thin layer chromatography (development phase: 1:7=methanol:chloroform) to give the title compound (190 mg).
MS(ESI)m/z:596(M⁺+H).
¹H-NMR(CDCl₃)δ:1.07-1.28(2H,m),1.74-1.81(1H,m),1.98-2.07(1H,m),2.15-2.30(2H,m),2.58-2.70(1H,m),2.85-3.13(1H,m),3.20-3.28(1H,m),3.42(3H,s),3.65-3.78(5H,m),3.85(3H,s),3.97-4.05(1H,m),4.59-4.76(2H,m),5.79-5.84(1H,m),6.19-6.22(1H,m),6.70-6.74(1H,m),6.93-7.00(2H,m),7.15-7.34(4H,m).

### Example 230

2-(1-(2-((4R,6S)-1-(Aminocarbonyl)-8-chloro-6-(2,3-dimethoxyphenyl)-4H,6H-pyrrolo(1,2-a)(4,1)benzoxazepin-4-yl)acetyl)-4-piperidinyl)acetic acid

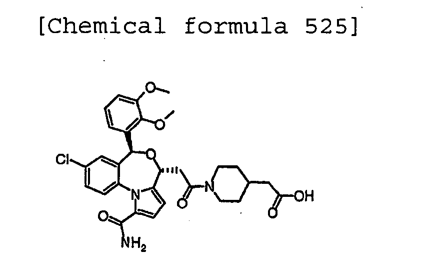

To a methanol solution (30.0 mL) of methyl 2-(1-(2-((4R,6S)-1-(aminocarbonyl)-8-chloro-6-(2,3-dimethoxyphenyl)-4H,6H-pyrrolo(1,2-a)(4,1)benzoxazepin-4-yl)acetyl)-4-piperidinyl)acetate (190 mg) were added distilled water (15.0 mL) and potassium carbonate (132 mg). The resulting mixture was stirred at 50°C for 19 hours.
The reaction mixture was adjusted to pH 4.0 with an ion exchange resin IR-120B. The resin was filtered out and the solvent was distilled off. The residue was purified by preparative thin layer chromatography (development phase: a 7:3:1=chloroform:methanol:water organic layer), followed by lyophilization from 1,4-dioxane-water to give the title compound (90 mg).
MS(ESI)m/z:582(M⁺+H). ¹H-NMR(CDCl₃)δ:1.05-1.28(2H,m),1.64-1.84(2H,m),1.92-2.06(1H,m),2.15-2.19(1H,m),2.27(1H,d,J=6.8Hz),2.53-2.69(1H,m),2.81-2.95(1H,m),2.97-3.13(1H,m),3.18-3.28(1H,m),3.41(3H,s),3.84(3H,s),4.01(1H,d,J=12.9Hz),4.57-4.77(2H,m),5.79-5.84(1H,m),6.03(2H,br s),6.20(1H,t,J=3.2Hz),6.73(1H,dd,J=8.8,1.7Hz),6.87-6.90(1H,m),6.93-6.96(1H,m),7.16(1H,t,J=7.9Hz),7.22-7.26(1H,m),7.30-7.32(2H,m).

### Example 231

3-((4R,6S)-1-(Aminocarbonyl)-8-chloro-6-(2,3-dimethoxyphenyl)-4H,6H-pyrrolo(1,2-a)(4,1)benzoxazepin-4-yl)propanoic acid

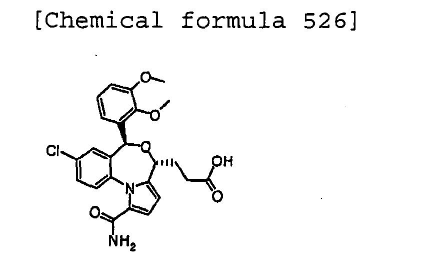

To an isopropanol solution (2.0 mL) of methyl 3-((4R,6S)-8-chloro-l-cyano-6-(2,3-dimethoxyphenyl)-4H,6H-pyrrolo(1,2-a)(4,1)benzoxazepin-4-yl)propanoate (300 mg) was added a 1N aqueous sodium hydroxide solution (3.2 mL). The resulting mixture was heated under reflux for 13 hours. Isopropanol was distilled off under reduced pressure. A 10% aqueous citric acid solution was added and the resulting mixture was extracted with ethyl acetate. The organic layer was dried over anhydrous sodium sulfate. The solvent was distilled off and the title compound (344 mg) was obtained.
MS(ESI)m/z:471(M⁺+H).
¹H-NMR(CDCl₃)δ:2.27-2.48(2H,m),2.55-2.78(2H,m),3.42(3H,s),3.85(3H,s),4.13-4.18(1H,m),5.77-5.84(3H,m),6.30-6.34(1H,m),6.73-6.76(1H,m),6.90-6.98(2H,m),7.16-7.21(1H,m),7.24-7.27(2H,m),7.33-7.35(1H,m).

### Example 232

Ethyl 2-(1-(3-((4R,6S)-1-(aminocarbonyl)-8-chloro-6-(2,3-dimethoxyphenyl)-4H,6H-pyrrolo(1,2-a)(4,1)benzoxazepin-4-yl)propanoyl)-4-piperidinyl)acetate

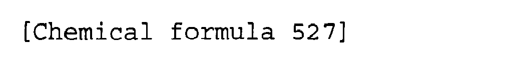

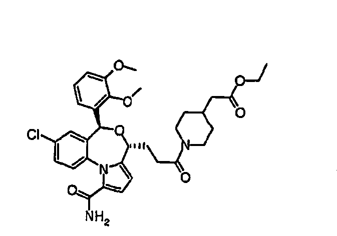

To a methylene chloride (3.0 mL) solution of 3-((4R,6S)-1-(aminocarbonyl)-8-chloro-6-(2,3-dimethoxyphenyl)-4H,6H-pyrrolo(1,2-a)(4,1)benzoxazepin-4-yl)propanoic acid (88 mg) were added ethyl piperidin-4-yl-acetate (38 mg), 1-ethyl-3-(3-dimethylaminopropyl)carbodiimide hydrochloride (72 mg), and 1-hydroxybenzotriazole (29 mg). The resulting mixture was stirred at room temperature for 4 hours. The reaction mixture was purified by preparative thin layer chromatography (development phase: 5% methanol-chloroform) to give the title compound (65 mg).
MS (ESI) m/z : 624 (M⁺+H) . ¹H-NMR(CDCl₃)δ:1.03-1.22(2H,m),1.26(3H,t,J=7.2Hz),1.68-1.82(2H,m),1.95-2.06(1H,m),2.18-2.26(2H,m),2.34-2.42(2H,m),2.49-2.72(3H,m),2.97-3.07(1H,m),3.43(3H,s),3.84-3.94(4H,m),4.10-4.17(3H,m),4.54-4.63(1H,m),5.67(2H,br s),5.78(1H,s),6.33(1H,d,J=3.9Hz),6.73(1H,s),6.90(1H,d,J=3.9 Hz),6.95(1H,dd,J=7.9,1.6Hz),7.16-7.24(2H,m),7.33-7.35(2H,m).

### Example 233

2-(1-(3-((4R,6S)-1-(Aminocarbonyl)-8-chloro-6-(2,3-dimethoxyphenyl)-4H,6H-pyrrolo(1,2-a)(4,1)benzoxazepin-4-yl)propanoyl)-4-piperidinyl)acetic acid

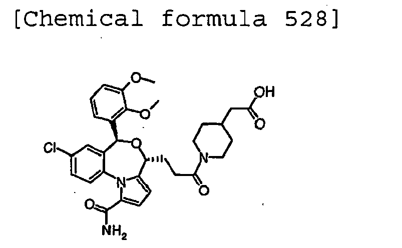

To a methanol solution (10.0 mL) of ethyl 2-(1-(3-((4R,6S)-1-(aminocarbonyl)-8-chloro-6-(2,3-dimethoxyphenyl)-4H,6H-pyrrolo(1,2-a)(4,1)benzoxazepin-4-yl)propanoyl)-4-piperidinyl)acetate (65 mg) were added distilled water (5.0 mL) and potassium carbonate (43 mg). The resulting mixture was stirred at room temperature for 17 hours. The reaction mixture was adjusted to pH 4.0 with an ion exchange resin IR-120B. The resin was filtered out and the solvent was distilled off. The residue was purified by preparative thin layer chromatography (development phase: a 7:3:1=chloroform:methanol:water organic layer), followed by lyophilization from 1,4-dioxane-water to give the title compound (66 mg).
MS(ESI)m/z:596(M⁺+H).
¹H-NMR(CDCl₃)δ:1.00-1.20(2H,m),1.71-1.85(2H,m),1.93-2.05(1H,m),2.22-2.30(2H,m),2.34-2.43(2H,m),2.51-2.72(3H,m),2.96-3.07(1H,m),3.38-3.45(4H,m),3.71(3H,s),3.81-3.93(4H,m),4.11-4.18(1H,m),4.55-4.63(1H,m),5.75-5.86(3H,m),6.30-6.35(1H,m),6.73(1H,s),6.90-6.92(1H,m),6.93-6.98(1H,m),7.18(1H,t,J=7.8Hz),7.22-7.25(2H,m),7.33-7.36(2H,m).

### Example 234

Ethyl 2-(1-(3-((4R,6S)-8-chloro-6-(2,3-dimethoxyphenyl)-1-((dimethylamino)carbonyl)-4H,6H-pyrrolo(1,2-a)(4,1)benzoxazepin-4-yl)propanoyl)-4-piperidinyl)acetate

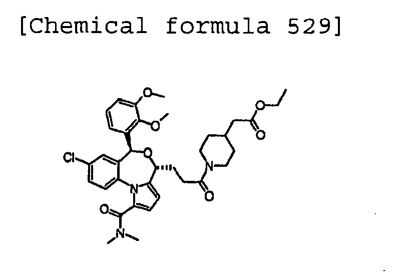

To an N,N-dimethylformamide solution (5.0 mL) of ethyl 2-(1-(3-((4R,6S)-1-(aminocarbonyl)-8-chloro-6-(2,3-dimethoxyphenyl)-4H,6H-pyrrolo(1,2-a)(4,1)benzoxazepin-4-yl)propanoyl)-4-piperidinyl)acetate (65 mg) was added 55% sodium hydride in oil (23 mg) under ice cooling. The resulting mixture was stirred at the same temperature for 1 hour. Methyl iodide (78 µl) was added and the resulting mixture was stirred at room temperature for 3 days. After addition of ethyl acetate, the resulting mixture was washed successively with a 10% aqueous citric acid solution and saturated brine. The organic layer was dried over anhydrous sodium sulfate. The solvent was distilled off. The residue was purified by preparative thin layer chromatography (development phase: 10% methanol-chloroform) to give the title compound (87 mg).
MS(ESI)m/z:652(M⁺+H). ¹H-NMR(CDCl₃)δ:1.05-1,17(2H,m),1.23(3H,t,J=6.6Hz),1.69-1.82(2H,m),1.99-2.06(1H,m),2.20-2.28(2H,m),2.36-2.44(2H,m),2.49-2.68(3H,m),3.01-3.07(1H,m),3.45(3H,s),3.48(3H,s),3.67-3.73(3H,m),3.84-3.90(5H,m),4.14(2H,q,J=6.6Hz),4.23-4.28(1H,m),4.55-4.62(1H,m),5.79(1H,s),6.36(1H,d,J=3.4Hz),6.61(1H,d,J=3.7Hz) ,6.69(1H,d,J=2.2Hz),6.98(1H,d,J=6.8Hz),7.18-7.24(2H,m),7.29-7.33(2H,m).

### Example 235

2-(1-(3-((4R,6S)-8-Chloro-6-(2,3-dimethoxyphenyl)-1-((dimethylamino)carbonyl)-4H,6H-pyrrolo(1,2-a)(4,1)benzoxazepin-4-yl)propanoyl)-4-piperidinyl)acetic acid

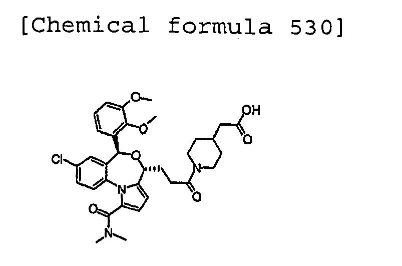

To a methanol solution (6.0 mL) of ethyl 2-(1-(3-((4R,6S)-8-chloro-6-(2,3-dimethoxyphenyl)-1-((dimethylamino)carbonyl)-4H,6H-pyrrolo(1,2-a)(4,1)benzoxazepin-4-yl)propanoyl)-4-piperidinyl)acetate (68 mg) were added distilled water (3.0 mL) and potassium carbonate (43 mg). The resulting mixture was stirred at 50°C for 18 hours. The reaction mixture was adjusted to pH 4.0 with an ion exchange resin IR-120B. The resin was filtered out and the solvent was distilled off. The residue was purified by preparative thin layer chromatography (development phase: 10% methanol-chloroform), followed by lyophilization from 1,4-dioxane-water to give the title compound (61 mg).
MS (ESI) m/z : 624 (M⁺+H) .
¹H-NMR(CDCl₃) δ: 0.88-1.23 (2H,m), 1.67-1.79 (2H,m), 1.92-2.04(1H,m),2.22-2.76(9H,m),2.87-3.03(3H,m),3.43-3.54(3H,m),3.80-3.91(4H,m),4.22-4.33(1H,m),4.52-4.64(1H,m),5.74-5.77(1H,m),6.33-6.41(1H,m),6.61-6.64(1H,m),6.67-6.69(1H,m),6.96-6.99(1H,m),7.19-7.24(1H,m),7.25-7.36(3H,m).

### Referential Example 128

{5-Chloro-2-[2-(2-methylpropenyl)pyrrol-1-yl]phenyl}-(2,3-dimethoxyphenyl)methanone

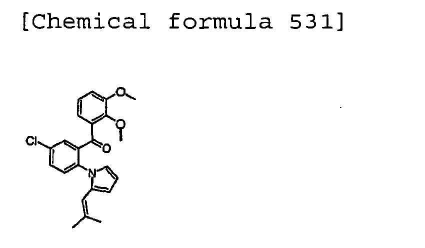

Isopropyltriphenylphosphonium iodide (5.0 g) was suspended in tetrahydrofuran (40 mL). To the resulting suspension was added n-butyl lithium (1.6M hexane solution, 8.1 mL) at 0°C. After the resulting mixture was stirred at the same temperature for 40 minutes, a 1-[4-chloro-2-(2,3-dimethoxybenzoylphenyl)-1H-pyrrol-2-carbaldehyde (1.95 g)/tetrahydrofuran (10 mL) solution was added dropwise. After completion of the dropwise addition, water was added to the reaction mixture, followed by extraction with ethyl acetate. The extract was dried over magnesium sulfate.
The solvent was distilled off under reduced pressure. The residue was purified by silica gel chromatography (hexaneethyl acetate,4:1) to give the title compound (1.88 g).
MS(ESI)m/z:396(M⁺+H).
¹H-NMR(CDCl₃)δ:1.76(3H,s),1.78(3H,s),3.51(3H,s),3.81(3H,s),5.6 1(1H,s),5.95-5.96(1H,m),6.01(1H,t,J=3.2Hz),6.59(1H,q,J=1.5Hz),6.85-6.94(3H,m),7.22(1H,d,J=8.3Hz),7.50(1H,dd,J=8.3,2.4Hz),7.58( 1H,d,J=2.4Hz).

### Referential Example 129

[5-Chloro-2-(2-isobutylpyrrol-1-yl)phenyl]-(2,3-dimethoxyphenyl)methanone

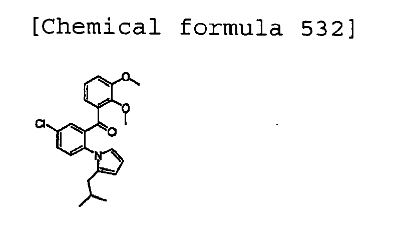

{5-Chloro-2-[2-(2-methylpropenyl)pyrrolo-1-yl]phenyl}-(2,3-dimethoxyphenyl)methanone (500 mg) was dissolved in tetrahydrofuran (20 mL). To the resulting solution was added 10% Pd-C (50% wet, catalytic amount). The resulting mixture was stirred in a hydrogen atmosphere for 1 hour. The catalyst was filtered off and the solvent was distilled off from the filtrate to give the title compound (503 mg).
MS(ESI)m/z:398(M⁺+H).
¹H-NMR(CDCl₃)δ:0.79(6H,d,J=6.6Hz),1.59-1.69(1H,m),2.19(2H,d,J=6.9Hz),3.53(3H,s),3.82(3H,s),5.76-5.77(1H,m),5.90(1H,t,J=3.2Hz),6.49(1H,dd,J=2.7,1.7Hz),6.86-6.91(1H,m),6.93-6.95(2H,m),7.22(1H,d,J=8.8Hz),7.49(1H,dd,J=8.6,2.5Hz),7.55( 1H,d,J=2.5Hz).

### Referential Example 130

1-[4-Chloro-2-(2,3-dimethoxybenzoyl)phenyl]-5-isobutyl-1H-pyrrol-2-carbaldehyde

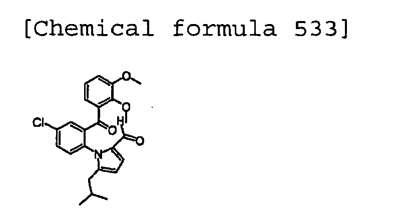

Under ice cooling, phosphorus oxychloride (0.26 mL) was added to N,N-dimethylformamide (2 mL). The resulting mixture was stirred at the same temperature for 15 minutes.
A [5-chloro-2-(2-isobutylpyrrol-1-yl)phenyl]-(2,3-dimethoxyphenyl)methanone (500 mg)/methylene chloride (5 mL) solution was added and the resulting mixture was stirred at the same temperature for 15 minutes. A saturated aqueous solution of sodium bicarbonate was added and the resulting mixture was stirred. The resulting mixture was extracted with ethyl acetate. After drying over magnesium sulfate, the solvent was distilled off under reduced pressure. The residue was purified by silica gel chromatography (hexane-ethyl acetate,3:1) to give the title compound (449 mg).
MS(ESI)m/z:426(M⁺+H). ¹H-NMR(CDCl₃)δ:0.80(3H,d,J=6.6Hz),0.87(3H,d,J=6.6Hz),1.71-1.81(1H,m),2.11(1H,q,J=7.6Hz)-,2.36(1H,dd,J=15.4,7.1Hz),3.71 (3H,s),3.84(3H,s),6.06(1H,d,J=4.2Hz),6.78(1H,dd,J=6.6,2.5Hz ),6.82(1H,d,J=3.7Hz),6.92-7.00(2H,m),7.23(1H,d,J=8.3Hz),7.54-7.59(2H,m),9.19(1H,s).

### Referential Example 131

Methyl 3-[1-[4-chloro-2-(2,3-dimethoxybenzoyl)phenyl]-5-isobutyl-1H-pyrrol-2-yl]acrylate

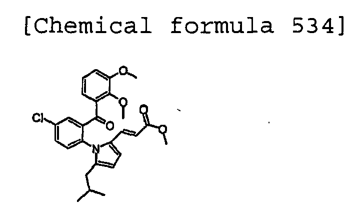

1-[4-Chloro-2-(2,3-dimethoxybenzoyl)phenyl]-5-isobutyl-1H-pyrrol-2-carbaldehyde (449 mg) was dissolved in N,N-dimethylformamide (5 mL). To the resulting solution was added methyl triphenylphosphoranylidene acetate (1.08 g). The resulting mixture was stirred at 80°C for 12 hours. The reaction mixture was diluted with ethyl acetate, washed twice with saturated brine, and dried over magnesium sulfate. After the solvent was distilled off under reduced pressure, the residue was purified by silica gel chromatography (hexane-ethyl acetate,3:1) to give the title compound (460 mg).
MS (ESI)m/z:482 (M⁺+H).
¹H-NMR(CDCl₃)δ:0.76(3H,d,J=6.6Hz),0.82(3H,d,J=6.6Hz),1.64(1H,t d,J=13.5,6.6Hz),2.01-2.07(1H,m),2.26(1H,q,J=7.5Hz),3.63(3H,s),3.69(3H,s),3.78(3H ,s),5.75(1H,d,J=15.7Hz),5.93(1H,d,J=3.9Hz),6.48(1H,d,J=3.9H z),6.68(1H,dd,J=7.2,2.1Hz),6.86-6.93(2H,m),7.02(1H,d,J=15.7Hz),7.17(1H,d,J=8.3Hz),7.58(1H,d d,J=8.3,2.5Hz),7.64(1H,d,J=2.5Hz).

### Referential Example 132

Methyl 3-[1-(4-chloro-2-[(2,3-dimethoxyphenyl)hydroxymethyl]phenyl)-5-isobutyl-1H-pyrrol-2-yl]acrylate

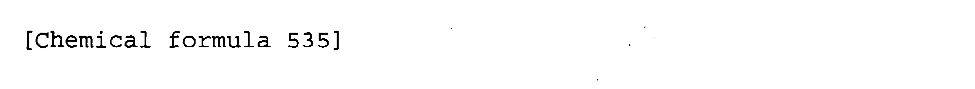

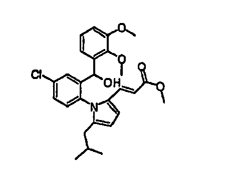

Methyl 3-[1-[4-chloro-2-(2,3-dimethoxybenzoyl)phenyl]-5-isobutyl-1H-pyrrol-2-yl]acrylate (460 mg) was dissolved in methanol (5 mL). Sodium borohydride (72 mg) was added and the resulting mixture was stirred for 30 minutes. The reaction mixture was partitioned between ethyl acetate and water. The organic layer was dried over magnesium sulfate. The solvent was distilled off under reduced pressure to give the title compound (430 mg).
MS(ESI)m/z:484(M⁺+H).

### Example 236

Methyl 2-(8-chloro-6-(2,3-dimethoxyphenyl)-1-isobutyl-4H,6H-pyrrolo(1,2-a)(4,1)benzoxazepin-4-yl)acetate

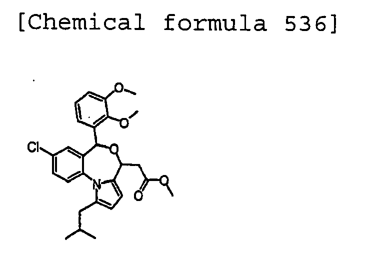

Methyl 3-[1-{4-chloro-2-[(2,3-dimethoxyphenyl)hydroxymethyl]phenyl}-5-isobutyl-1H-pyrrol-2-yl]acrylate (430 mg) was dissolved in methylene chloride (15 mL). Trifluoroacetic acid (69 µL) was added and the resulting mixture was stirred for 30 minutes. The reaction mixture was diluted with ethyl acetate. The organic layer was washed with water and dried over magnesium sulfate.
The solvent was distilled off under reduced pressure. The residue was purified by silica gel chromatography (hexane-ethyl acetate, 4:1) to give the title compound (104 mg) and collect the raw material (205 mg).
MS(ESI)m/z:484(M⁺+H). ¹H-NMR(CDCl₃)δ:0.81(3H,d,J=6.9Hz),0.83(3H,d,J=6.9Hz),1.59-1.67(1H,m),2.52(1H,dd,J=15.0,8.2Hz),2.79(1H,dd,J=15.0,6.1Hz ),2.97-3.10(2H,m),3.43(3H,s),3.71(3H,s),3.85(3H,s),4.74(1H,dd,J=8. 1,6.1Hz),5.60(1H,s),6.10(1H,d,J=3.7Hz),6.17(1H,d,J=3.7Hz),6 .71(1H,d,J=2.2Hz),6.93(1H,dd,J=8.1,1.5Hz),7.16(1H,t,J=8.0Hz ),7.23-7.26(1H,m),7.30(1H,d,J=8.6Hz),7.37(1H,dd,J=8.6,2.3Hz).

### Referential Example 133

trans-2-[8-Chloro-6-(2,3-dimethoxyphenyl)-1-isobutyl-4H,6H-pyrrolo[1,2-a][4,1]benzoxazepin-4-yl]-1-ethanol

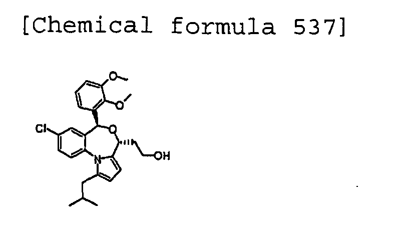

To a suspension of lithium aluminum hydride (12.8 mg) in tetrahydrofuran (15 mL) was added a tetrahydrofuran (5.0 mL) solution of methyl 2-(8-chloro-6-(2,3-dimethoxyphenyl)-1-isobutyl-4H,6H-pyrrolo(1,2-a)(4,1)benzoxazepin-4-yl)acetate (109 mg) in portions under ice cooling. The resulting mixture was stirred under ice cooling for 90 minutes. After a 10% aqueous solution of potassium sodium tartrate was added to terminate the reaction, the reaction mixture was extracted with ethyl acetate (three times).
The organic layers combined were washed with water (twice) and saturated brine, and dried over anhydrous sodium sulfate. The solvent was distilled off under reduced pressure to give the title compound (119 mg). ¹H-NMR(CDCl₃)δ:0.82 (6H,t,J=7.6Hz),2.17-2.55(4H,m),2.79(1H,dd,J=14.6,6.1Hz),3.44(3H,s),3.90(5H,dd,J =23.3,9.2Hz),4.48(1H,d,J=6.1Hz),5.01(0.08H,s),5.65(0.92H,s) ,6.17(2H,d,J=36.9Hz),6.73(1H,s),6.96(1H,t,J=8.7Hz),7.20-7.34(4H,m).

### Example 237

trans-Ethyl 2-(2-{2-[8-chloro-6-(2,3-dimethoxyphenyl)-1-isobutyl-4H,6H-pyrrolo[1,2-a][4,1]benzoxazepin-4-yl]ethyl}-2H-1,2,3,4-tetrazol-5-yl)acetate (low polarity) trans-Ethyl 2-(1-{2-[8-chloro-6-(2,3-dimethoxyphenyl)-1-isobutyl-4H,6H-pyrrolo[1,2-a][4,1]benzoxazepin-4-yl]ethyl}-1H-1,2,3,4-tetrazol-5-yl)acetate (high polarity)

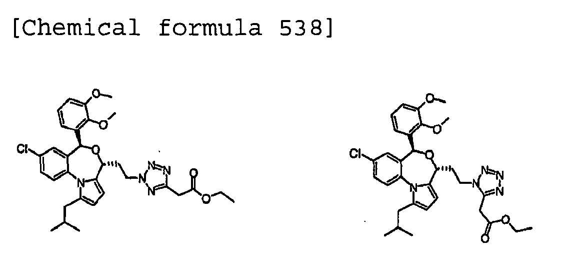

To a tetrahydrofuran (20 mL) solution of trans-2-[8-chloro-6-(2,3-dimethoxyphenyl)-1-isobutyl-4H,6H-pyrrolo[1,2-a] [4,1]benzoxazepin-4-yl]-1-ethanol (119 mg) and triethylamine (94 µl) was added a tetrahydrofuran (2.0 mL) solution of methanesulfonyl chloride (38.7 mg) under ice cooling. The resulting mixture was stirred at room temperature for 2.5 hours. The reaction mixture was diluted with ethyl acetate, washed successively with a saturated aqueous solution of sodium hydrogen carbonate and saturated brine, and dried over anhydrous sodium sulfate. The solvent was distilled off under reduced pressure to give an oily residue. The residue was dissolved in N,N-dimethylformamide (15 ml). Ethyl 1H-tetrazol-5-acetate (105 mg), potassium carbonate(155 mg) and tetra-n-butylammonium iodide (83.1 mg) were added and the resulting mixture was stirred at 40°C for one hour and at 80°C for 4 hours. After the reaction mixture was cooled to room temperature and diluted with ethyl acetate, the diluted solution was washed successively with water (three times) and saturated brine and dried over anhydrous sodium sulfate. After the solvent was distilled off under reduced pressure, the residue thus obtained was purified by thin layer chromatography (silica gel, N-hexane/ethyl acetate=1/1) to give the title compounds. (Low polarity): yield; 70.5 mg
¹H-NMR(CDCl₃)δ:0.82(6H,t,J=7.1Hz),1.23-1.65(4H,m),2.49-2.81(4H,m),3.42(3H,d,J=13.2Hz),3.89(5H,d,J=24.7Hz),4.20(3H, ddt,J=49.0,23.1,8.1Hz),4.88(1H,td,J=14.0,7.1Hz),5.65(1H,s), 6.17(2H,dd,J=38.6,2.9Hz),6.72(1H,s),6.96(1H,d,J=8.3Hz),7.28 (4H,ddd,J=46.2,21.7,11.4Hz).
(High polarity): yield; 34.9 mg. Purified after the subsequent step because it was a crude product.

### Example 238

trans-2-(2-{2-[8-Chloro-6-(2,3-dimethoxyphenyl)-1-isobutyl-4H,6H-pyrrolo[1,2-a] [4,1]benzoxazepin-4-yl]ethyl}-2H-1,2,3,4-tetrazol-5-yl)acetic acid

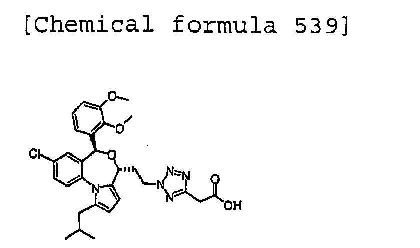

To a solution of trans-ethyl 2-(2-{2-[8-chloro-6-(2,3-dimethoxyphenyl)-1-isobutyl-4H,6H-pyrrolo[1,2-a][4,1]benzoxazepin-4-yl]ethyl)-2H-1,2,3,4-tetrazol-5-yl)acetate (32.1 mg) in a tetrahydrofuran (2.0 mL)/ethanol (4.0 mL) mixture was added an aqueous solution (2.0 mL) of potassium carbonate (22.4 mg). The resulting mixture was stirred at 50°C for 3 hours. After cooling to room temperature, a 1N aqueous solution of hydrochloric acid was added to make the reaction mixture acidic. The resulting mixture was concentrated under reduced pressure. Water was added to the residue, followed by extraction with methylene chloride (three times). The organic layers were combined and dried over anhydrous sodium sulfate. The solvent was distilled off under reduced pressure. The residue was subjected to azeotropy three times and then lyophilized from dioxane and water to give the title compound (16.8 mg).
¹H-NMR(CDCl₃)δ:0.82(6H,t,J=7.3Hz), 1.60-1.67(1H,m),2.52(1H,dd,J=14.9,8.3Hz),2.68-2.81(3H,m),3.43(3H,s),3.86(3H,s),3.99(2H,s),4.27(1H,dd,J=9. 3,4.2Hz),4.88-4.93(2H,m),5.64(1H,s),6.12(1H,d,J=3.4Hz),6.21(1H,d,J=3.4Hz) ,6.72(1H,d,J=2.2Hz),6.96(1H,d,J=8.3Hz),7.18-7.37(4H,m).
HRMS-FAB(C₂₉H₃₃O₅N₅Cl);m/z :
Calculated (m/z):566.2170.
Found (m/z):566.2166.

### Example 239

trans-2-(1-{2-[8-Chloro-6-(2,3-dimethoxyphenyl)-1-isobutyl-4H,6H-pyrrolo[1,2-a][4,1]benzoxazepin-4-yl]ethyl}-1H-1,2,3,4-tetrazol-5-yl)acetic acid

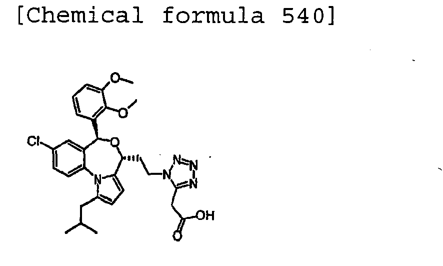

To a solution of trans-ethyl 2-(1-{2-[8-chloro-6-(2,3-dimethoxyphenyl)-1-isobutyl-4H,6H-pyrrolo[1,2-a][4,1]benzoxazepin-4-yl]ethyl}-1H-1,2,3,4-tetrazol-5-yl)acetate (34.9 mg) in a mixture of tetrahydrofuran (2.0 mL)/ethanol (4.0 mL) was added an aqueous solution (2.0 mL) of potassium carbonate (24.3 mg). The resulting mixture was stirred at 50°C for 3 hours. After the reaction mixture was cooled to room temperature, a 1N aqueous solution of hydrochloric acid was added to make it acidic, followed by concentration under reduced pressure. Water was added to the residue. The resulting mixture was extracted with methylene chloride (three times). The organic layers were combined and dried over anhydrous sodium sulfate. The solvent was distilled off under reduced pressure. The residue thus obtained was purified by thin layer chromatography, followed by lyophilization from dioxane and water to give the title compound (7.16 mg).
¹H-NMR(CDCl₃)δ:0.79(6H,t,J=6.8Hz),1.59(1H,t,J=6.6Hz),2.60(4H,t t,J=59.6,19.9Hz),3.63(8H,dt,J=81.8,45.8Hz),4.38(3H,dd,J=98. 9,14.6Hz),5.60(1H,s),6.09(2H,d,J=36.4Hz),6.70-7.30(6H,m).
HRMS-FAB(C₂₉H₃₂O₅N₅Cl);m/z:
Calculated (m/z):566.2170.
Found (m/z):566.2166.

### Referential Example 134

1-[4-Chloro-2-(2,3-dimethoxybenzoyl)phenyl]-5-(2-methoxypropenyl)-1H-pyrrol-2-carbaldehyde

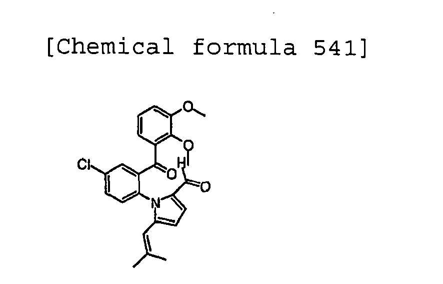

Under ice cooling, phosphorus oxychloride (0.83 mL) was added to N,N-dimethylformamide (10 mL). The resulting mixture was stirred at the same temperature for 15 minutes. To the reaction mixture was added a {5-chloro-2-[2-(2-methylpropenyl)-pyrrol-1-yl]-phenyl}-(2,3-dimethoxyphenyl)-methanone (1.60 g)/methylene chloride (10 mL) solution.
The resulting mixture was stirred at the same temperature for 15 minutes. A saturated aqueous solution of sodium bicarbonate was added and the resulting mixture was stirred. After extraction with ethyl acetate and drying over magnesium sulfate, the solvent was distilled off under reduced pressure. The residue was purified by silica gel chromatography (hexane-ethyl acetate, 3:1) to give the title compound (1.10 g).
¹H-NMR(CDCl₃)δ:1.80(3H,s),1.88(3H,s),3.66(3H,s),3.83(3H,s),5.5
7(1H,s),6.23(1H,d,J=4.2Hz),6.79(1H,dd,J=6.6,2.7Hz),6.89-7.00(3H,m),7.21(1H,d,J=8.3Hz),7.54-7.59(2H,m),9.27(1H,s).
MS(ESI)m/z:424(M⁺+H).

### Referential Example 135

Methyl 3-[1-[4-chloro-2-(2,3-dimethoxybenzoyl)phenyl]-5-(2-methylpropenyl)-1H-pyrrol-2-yl]-acrylate

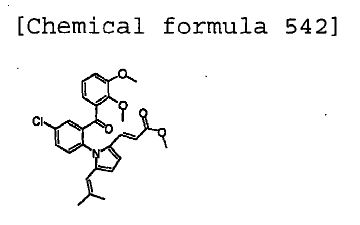

1-[4-Chloro-2-(2,3-dimethoxybenzoyl)phenyl]-5-(2-methoxypropenyl)-1H-pyrrol-2-carbaldehyde (1.10 g) was dissolved in N,N-dimethylformamide (10 mL). To the resulting solution was added methyl triphenylphosphoranylidene acetate (2.66 g). The resulting mixture was stirred under heat at 100°C for 12 hours. The reaction mixture was diluted with ethyl acetate, washed twice with saturated brine, and dried over magnesium sulfate. After the solvent was distilled off under reduced pressure, the residue was purified by silica gel chromatography (hexane-ethyl acetate,3:1) to give the title compound (1.03 g).
¹H-NMR(CDCl₃)δ:1.77(3H,s),1.83(3H,s),3.59(3H,s),3.70(3H,s),3.7 7(3H,s),5.49(1H,s),5.84(1H,d,J=15.6Hz),6.13(1H,d,J=4.2Hz),6 .58(1H,d,J=4.2Hz),6.70(1H,dd,J=7.1,2.2Hz),6.85-6.92(2H,m),7.06(1H,d,J=15.6Hz),7.16(1H,d,J=8.4Hz),7.57(1H,d d,J=8.4,2.6Hz),7.63(1H,d,J=2.2Hz).
MS(ESI)m/z:480(M⁺+H).

### Referential Example 136

Methyl 3-[1-{4-chloro-2-[(2,3-dimethoxyphenyl)hydroxymethyl]phenyl}-5-(2-methylpropenyl)-1H-pyrrol-2-yl]acrylate

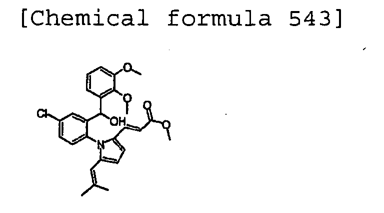

Methyl 3-[1-[4-chloro-2-(2,3-dimethoxybenzoyl)phenyl]-5-(2-methylpropenyl)-1H-pyrrol-2-yl]-acrylate (1.03 g) was dissolved in methanol (50 mL)-tetrahydrofuran (10 mL). Sodium borohydride (162 mg) was added and the resulting mixture was stirred for 30 minutes. The reaction mixture was partitioned between ethyl acetate and water. After the organic layer was dried over magnesium sulfate, the solvent was distilled off under reduced pressure to give the title compound (980 mg).
MS (ESI) m/z : 482 (M⁺+H).

### Example 240

Methyl 2-(8-chloro-6-(2,3-dimethoxyphenyl)-1-(2-methyl-1-propenyl)-4H,6H-pyrrolo(1,2-a)(4,1)benzoxazepin-4-yl)acetate

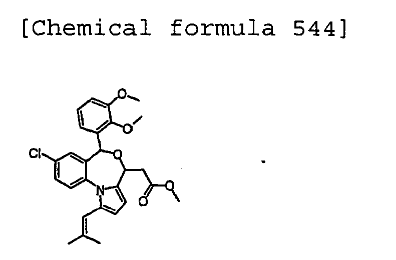

Methyl 3-[1-{4-chloro-2-[(2,3-dimethoxyphenyl)hydroxymethyl]phenyl}-5-(2-methylpropenyl)-1H-pyrrol-2-yl]acrylate (480 mg) was dissolved in methylene chloride (20 mL). To the resulting solution was added 1M HCl (20 mL) and the resulting mixture was stirred for 24 hours vigorously. After the organic layer was washed with water and dried over magnesium sulfate, the solvent was distilled off under reduced pressure. The residue was purified by silica gel chromatography (hexane-ethyl acetate, 3:1) to give the title compound (95 mg). ¹H-NMR(CDCl₃)δ:1.87(3H,s),1.89(3H,s),2.99-3.11(2H,m),3.44(3H,s),3.72(3H,s),3.85(3H,s),4.76(1H,dd,J=8.
1,6.1Hz),5.68(1H,s),5.95(1H,s),6.25-6.29(2H,m),6.73(1H,d,J=2.2Hz),6.93(1H,dd,J=8.1,1.5Hz),7.16( 1H,t,J=8.1Hz),7.23-7.26(1H,m),7.33(1H,dd,J=8.3,2.2Hz).

### Referential Example 137

2-[8-Chloro-6-(2,3-dimethoxyphenyl)-1-(2-methyl-1-propenyl)-4H,6H-pyrrolo[1,2-a][4,1]benzoxazepin-4-yl]-1-ethanol

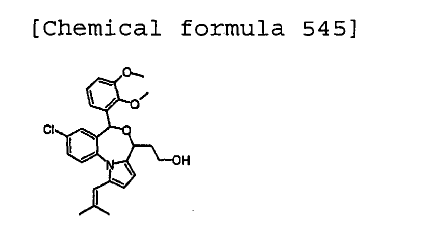

To a suspension of lithium aluminum hydride (11.2 mg) in tetrahydrofuran (7.0 mL) was added a tetrahydrofuran (4.0 mL) solution of methyl 2-(8-chloro-6-(2,3-dimethoxyphenyl)-1-(2-methyl-1-propenyl)-4H,6H-pyrrolo(1,2-a)(4,1)benzoxazepin-4-yl)acetate (95.0 mg) in portions under ice cooling. The resulting mixture was stirred for one hour under ice cooling. After a 10% aqueous solution of potassium sodium tartrate was added to terminate the reaction, the reaction mixture was extracted with ethyl acetate (three times). The organic layers were combined and washed with water (twice) and saturated brine, and then dried over anhydrous sodium sulfate. The solvent was distilled off under reduced pressure to give the title compound (109 mg).
¹H-NMR(CDCl₃)δ:1.91(6H,t,J=13.1Hz),2.18-2.46(3H,m),3.45(3H,s),3.87(3H,d,J=13.4Hz),3.95(2H,q,J=5.5Hz ),4.50(1H,dd,J=9.6,3.8Hz),5.73(1H,s),5.94(1H,s),6.32(2H,t,J =8.5Hz),6.75(1H,d,J=2.4Hz),6.94-6.98(1H,m),7.19(1H,t,J=7.9Hz),7.25(2H,d,J=8.3Hz),7.32(1H,dt ,J=14.6,5.1Hz).

### Example 241

Ethyl 2-(2-{2-[8-chloro-6-(2,3-dimethoxyphenyl)-1-(2-methyl-1-propenyl)-4H,6H-pyrrolo[1,2-a][4,1]benzoxazepin-4-yl]ethyl}-2H-1,2,3,4-tetrazol-5-yl)acetate Ethyl 2-(1-{2-[8-chloro-6-(2,3-dimethoxyphenyl)-1-(2-methyl-1-propenyl)-4H,6H-pyrrolo[1,2-a][4,1]benzoxazepin-4-yl]ethyl}-1H-1,2,3,4-tetrazol-5-yl)acetate

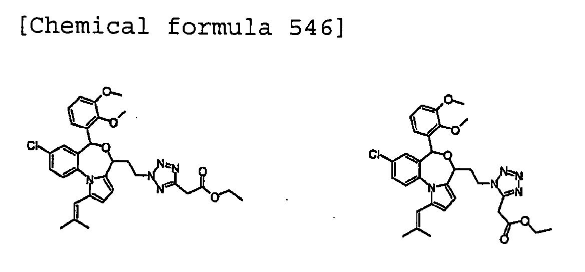

To a tetrahydrofuran (30 mL) solution of 2-[8-chloro-6-(2,3-dimethoxyphenyl)-1-(2-methyl-1-propenyl)-4H,6H-pyrrolo[1,2-a][4,1]benzoxazepin-4-yl]-1-ethanol (180 mg) and triethylamine (165 µl) was added a tetrahydrofuran (3.0 mL) solution of methanesulfonyl chloride (68.3 mg) under ice cooling. The resulting mixture was stirred at room temperature for 2.5 hours. The reaction mixture was diluted with ethyl acetate, washed successively with a saturated aqueous solution of sodium hydrogen carbonate and saturated brine, and dried over anhydrous sodium sulfate. The solvent was distilled off under reduced pressure to give an oily residue. The resulting residue was dissolved in N,N-dimethylformamide (30 mL). To the resulting solution were added ethyl 1H-tetrazol-5-acetate (186 mg), potassium carbonate (274 mg), and tetra-N-butylammonium iodide (147 mg). The resulting mixture was stirred at 40°C for one hour and then at 80°C for 1.5 hours. After cooling to room temperature, the reaction mixture was diluted with ethyl acetate, washed successively with water (three times) and saturated brine and dried over anhydrous sodium sulfate. The solvent was distilled off under reduced pressure. The residue thus obtained was purified by thin layer chromatography (silica gel, n-hexane/ethyl acetate=1/1) to give a 2H-isomer (115 mg), that is, the title compound having a lower polarity and a 1H-isomer (60 mg), that is, the title compound having a higher polarity. 2H-Isomer:
¹H-NMR(CDCl₃)δ:1.25(3H,t,J=7.1Hz),1.88(6H,dd,J=10.6,1.1Hz),2.7
2-2.80(2H,m),3.44(3H,d,J=3.7Hz),3.86(3H,d,J=2.2Hz),3.91(2H,d, J=11.7Hz),4.18(2H,ddd,J=14.3,7.1,4.5Hz),4.30(1H,q,J=4.6Hz), 4.89-4.94(2H,m),5.71(1H,d,J=12.2Hz),5.93(1H,s),6.30(2H,t,J=4.3Hz ),6.74(1H,d,J=2.4Hz),6.96(1H,dd,J=8.1,1.5Hz),7.22(2H,dt,J=1 3.5,5.3Hz),7.30-7.34(2H,m).
1H-Isomer:
¹H-NMR(CDCl₃)δ:1.24(3H,t,J=7.1Hz),1.89(6H,d,J=11.2Hz) 2.70(2H, dt,J=15.3,6.7Hz),3.45(3H,t,J=6.7Hz),3.86(3H,s),3.98(2H,dd,J =25.1,17.1Hz),4.18(2H,ddd,J=14.3,7.2,1.3Hz),4.26(1H,q,J=4.4 Hz),4.63(2H,dq,J=24.2,6.2Hz),5.71(1H,s),5.92(1H,s),6.29(2H, q,J=3.8Hz),6.75(1H,d,J=2.2Hz),6.97(1H,dd,J=7.8,1.7Hz),7.16-7.24(3H,m),7.35(1H,dd,J=8.5,2.4Hz).

### Example 242

2-(2-{2-[8-Chloro-6-(2,3-dimethoxyphenyl)-1-(2-methyl-1-propenyl)-4H,6H-pyrrolo[1,2-a][4,1]benzoxazepin-4-yl]ethyl}-2H-1,2,3,4-tetrazol-5-yl)acetic acid

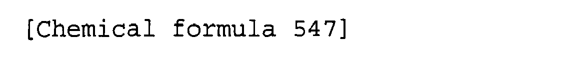

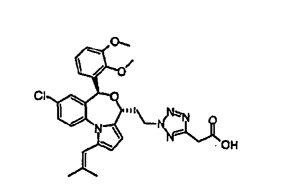

To a solution of ethyl 2-(2-{2-[8-chloro-6-(2,3-dimethoxyphenyl)-1-(2-methyl-1-propenyl)-4H,6H-pyrrolo[1,2-a][4,1]benzoxazepin-4-yl]ethyl}-2H-1,2,3,4-tetrazol-5-yl)acetate (15 mg) in a tetrahydrofuran (2.0 mL)/ethanol (4.0 mL) mixture was added an aqueous solution (2.0 mL) of potassium carbonate (10.5 mg). The resulting mixture was stirred at 50°C for 3 hours. After the reaction mixture was cooled to room temperature, a 1N aqueous hydrochloric acid solution was added to make it acidic, followed by concentration under reduced pressure. Water was added to the residue. The resulting mixture was extracted with methylene chloride (three times). The organic layers were combined and dried over anhydrous sodium sulfate. The solvent was distilled off under reduced pressure. The residue was subjected to azeotropy with ethanol three times and then lyophilized from dioxane and water to give the title compound (6.75 mg).
¹H-NMR(CDCl₃)δ:1.89(6H,d,J=9.8Hz),2.76(2H,d,0=7.6Hz),3.45(3H,s ),3.86(3H,s),3.99(2H,s),4.29(1H,d,J=4.4Hz),4.93(2H,t,J=7.0H z),5.73(1H,s),5.94(1H,s),6.30(2H,s),6.74(1H,s),6.97(1H,d,J= 8.1Hz),7.27(4H,tt,J=25.8,8.6Hz).
HRMS-FAB(C₂₉H₃₁O₅N₅Cl) ;m/z:
Calculated (m/z):564.2014
Found (m/z):564.1992

### Example 243

2-(1-{2-[8-Chloro-6-(2,3-dimethoxyphenyl)-1-(2-methyl-1-propenyl)-4H,6H-pyrrolo[1,2-a][4,1]benzoxazepin-4-yl]ethyl}-1H-1,2,3,4-tetrazol-5-yl)acetic acid

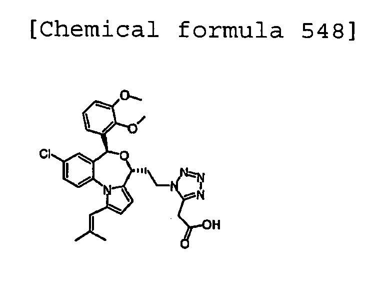

To a solution of ethyl 2-(1-{2-[8-chloro-6-(2,3-dimethoxyphenyl)-1-(2-methyl-1-propenyl)-4H,6H-pyrrolo[1,2-a][4,1]benzoxazepin-4-yl]ethyl}-1H-1,2,3,4-tetrazol-5-yl) acetate (15 mg) in a tetrahydrofuran (2.0 mL)/ethanol (4.0 mL) mixture was added an aqueous solution (2.0 mL) of potassium carbonate (10.5 mg). The resulting mixture was stirred at 50°C for 3 hours. After the reaction mixture was cooled to room temperature, a 1N aqueous hydrochloric acid solution was added to make it acidic, followed by concentration under reduced pressure. Water was added to the residue. The resulting mixture was extracted with methylene chloride (three times). The organic layers were combined and dried over anhydrous sodium sulfate. After the solvent was distilled off under reduced pressure, the residue was subjected to azeotropy with ethanol three times and then, lyophilized from dioxane and water to give the title compound (13.6 mg). ¹H-NMR(CDCl₃)δ:1.89(6H,d,J=10.7Hz),2.69-2.71(2H,m),3.43(3H,s),3.86(3H,s),4.26(1H,q,J=4.3Hz),4.57-4.69(2H,m),5.71(1H,s),5.92(1H,s),6.28(2H,d,J=7.3Hz),6.75(1H
,s),6.97(1H,d,J=8.8Hz),7.20-7.32(4H,m),m).
HRMS-FAB(C₂₉H₃₁0₅N₅C1);m/z:
Calculated (m/z):564.2014
Found (m/z):564.1992

### Referential Example 138

{5-Chloro-2-[3-(2-methylpropenyl)-pyrrol-1-yl]-phenyl}-(2,3-dimethoxyphenyl)-methanone

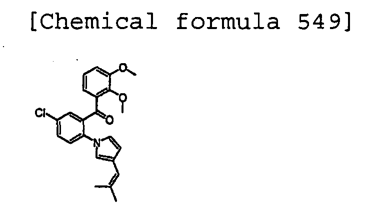

Isopropyltriphenylphosphonium iodide (2.0 g) was suspended in tetrahydrofuran (20 mL). To the resulting suspension was added n-butyl lithium (1.6M hexane solution, 2.8 mL) at 0°C. The resulting mixture was stirred at the same temperature for 40 minutes. Then, a 1-[4-chloro-2-(2,3-dimethoxybenzoylphenyl)-1H-pyrrol-3-carbaldehyde (910 mg)/tetrahydrofuran (10 mL) solution was added dropwise to the reaction mixture. After completion of the dropwise addition, water was added and the resulting mixture was extracted with ethyl acetate. After the extract was dried over magnesium sulfate, the solvent was distilled off under reduced pressure. The residue was purified by silica gel chromatography (hexane-ethyl acetate, 4:1) to give the title compound (540 mg).
¹H-NMR(CDCl₃)δ:1.76(3H,s),1.81(3H,s),3.49(3H,s),3.81(3H,s),5.9
0(1H,s),6.04(1H,t,J=2.3Hz),6.62-6.65(2H,m),6.94-6.97(2H,m),7.02-7.04(1H,m),7.29(1H,d,J=8.3Hz),7.46-7.49(1H,m),7.51(1H,d,J=2.5Hz).
MS(ESI)m/z:396(M⁺+H).

### Referential Example 139

1-[4-Chloro-2-(2,3-dimethoxybenzoyl)phenyl]-4-(2-methoxypropenyl)-1H-pyrrol-2-carbaldehyde

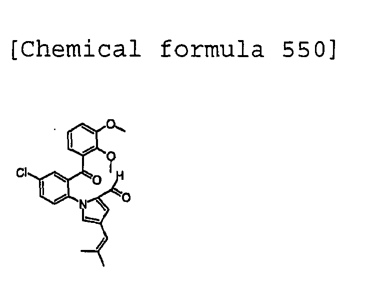

Under ice cooling, phosphorus oxychloride (300 µL) was added to N,N-dimethylformamide (2 mL) and the resulting mixture was stirred at the same temperature for 15 minutes. To the reaction mixture was added a {5-chloro-2-[3-(2-methylpropenyl)-pyrrol-1-yl]-phenyl}-(2,3-dimethoxyphenyl)-methanone (400 mg)/methylene chloride (3 mL) solution. The resulting mixture was stirred at the same temperature for 15 minute. A saturated aqueous solution of sodium bicarbonate was added to the reaction mixture, followed by stirring. After extraction with ethyl acetate and drying over magnesium sulfate, the solvent was distilled off under reduced pressure. The residue was purified by silica gel chromatography (hexane-ethyl acetate,4:1) to give the title compound (260 mg).
¹H-NMR(CDCl₃)δ:1.78(3H,s),1.89(3H,s),3.63(3H,s),3.81(3H,s),6.1 3(1H,d,J=2.7Hz),6.36(1H,s),6.82(1H,d,J=2.9Hz),6.86-6.90(1H,m),6.92-6.93(2H,m),7.25-7.29(1H,m),7.51(1H,dd,J=8.6,2.5Hz),7.61(1H,d,J=2.5Hz),9.49( 1H, s).
MS(ESI)m/z:424(M⁺+H).

### Referential Example 140

Methyl 3-[1-[4-chloro-2-(2,3-dimethoxybenzoyl)phenyl]-4-(2-methylpropenyl)-1H-pyrrol-2-yl]-acrylate

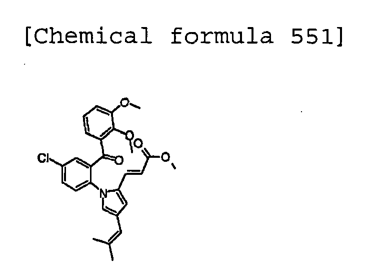

1-[4-Chloro-2-(2,3-dimethoxybenzoyl)phenyl]-4-(2-methoxypropenyl)-1H-pyrrol-2-carbaldehyde (1.90 g) was dissolved in N,N-dimethylformamide (50 mL). Methyl triphenylphosphoranylidene acetate (4.59 g) was added and the resulting mixture was stirred under heat at 120°C for 12 hours. The reaction mixture was diluted with ethyl acetate, washed twice with saturated brine and dried over magnesium sulfate. After the solvent was distilled off under reduced pressure, the residue was purified by silica gel chromatography (hexane-ethyl acetate,3:1) to give the title compound (1.76 g).
¹H-NMR(CDCl₃)δ:1.71(3H,d,J=1.0Hz) 1.87(3H,d,J=1.0Hz),3.55(3H,s ),3.71(3H,s),3.76-3.78(1H,m),3.78(3H,s),5.66(1H,d,J=15.9Hz),5.97(1H,s),6.03(1 H,d,J=2.7Hz),6.72(1H,d,J=2.9Hz),6.82-6.84(1H,m),6.88-6.92(2H,m),7.23(1H,d,J=8.3Hz),7.26-7.31(2H,m),7.54(1H,dd,J=8.3,2.4Hz),7.64(1H,d,J=2.4Hz).
MS(ESI)m/z:480(M⁺+H).

### Referential Example 141

Methyl 3-[1-{4-chloro-2-[(2,3-dimethoxyphenyl)hydroxymethyl]phenyl}-4-(2-methylpropenyl)-1H-pyrrol-2-yl]acrylate

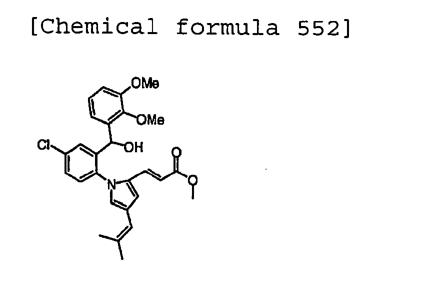

Methyl 3-[1-[4-chloro-2-(2,3-dimethoxybenzoyl)phenyl]-4-(2-methylpropenyl)-1H-pyrrol-2-yl]-acrylate (1.76 g) was dissolved in methanol (10 mL). Sodium borohydride (280 mg) was added and the resulting mixture was stirred for 40 minutes. The reaction mixture was partitioned between ethyl acetate and water. After the organic layer was dried over magnesium sulfate, the solvent was distilled off under reduced pressure to give the title compound (1.63 g).
MS (ESI) m/z :482 (M⁺+H) .

### Example 244

Methyl 2-(8-chloro-6-(2,3-dimethoxyphenyl)-2-(2-methyl-1-propenyl)-4H,6H-pyrrolo(1,2-a)(4,1)benzoxazepin-4-yl)acetate

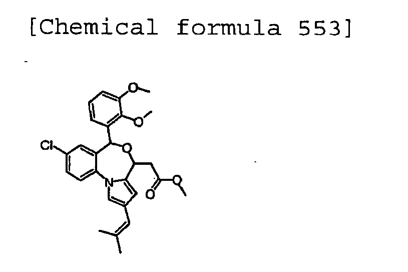

Methyl 3-[1-{4-chloro-2-[(2,3-dimethoxyphenyl)hydroxymethyl]phenyl}-4-(2-methylpropenyl)-1H-pyrrol-2-yl]acrylate (1.44 g) was dissolved in methylene chloride (30 mL). Trifluoroacetic acid (0.28 mL) was added and the resulting mixture was stirred at room temperature for 22 hours. The reaction mixture was diluted with methylene chloride, washed with a saturated aqueous solution of sodium bicarbonate and dried over magnesium sulfate. The solvent was distilled off under reduced pressure. The residue was purified by silica gel chromatography (hexane-ethyl acetate, 4:1) to give the raw material (960 mg) and the title compound (373 mg).
MS (ESI)m/z:482 (M⁺+H) .

### Referential Example 142

2-[8-Chloro-6-(2,3-dimethoxy)-2-(2-methyl-1-propenyl)-4H,6H-pyrrolo[1,2-a][4,1]benzoxazepin-4-yl]-1-ethanol

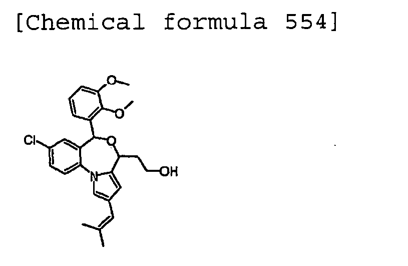

To a suspension of lithium aluminum hydride (91.1 mg) in tetrahydrofuran (30 ml) was added a tetrahydrofuran (15 ml) solution of methyl 2-(8-chloro-6-(2,3-dimethoxyphenyl)-2-(2-methyl-1-propenyl)-4H,6H-pyrrolo(1,2-a)(4,1)benzoxazepin-4-yl)acetate (770 mg) in portions under ice cooling. The resulting mixture was stirred at the same temperature for 3 hours. A 10% aqueous solution (100 ml) of potassium sodium tartrate was added to terminate the reaction mixture, followed by extraction with ethyl acetate (twice). The organic layers were combined, washed successively with water (three times) and saturated brine and dried over anhydrous sodium sulfate. The solvent was distilled off under reduced pressure. The residue thus obtained was purified by chromatography (silica gel, n-hexane/ethyl acetate=from 4/1 to 2/1) to give the title compound (581 mg).
¹H-NMR(CDCl₃)δ:1.47-1.80(7H,m),(0.7H,dq,J=23.1,5.9Hz),2.51(0.3H,d,J=4.6Hz),3.47 (3H,d,J=9.3Hz),3.57-3.63(2H,m),3.86(3H,d,J=2.0Hz),4.68(0.3H,dd,J=10.4,3.3Hz),5. 40(0.7H,dd,J=9.0,6.1Hz),5.77(0.3H,s),6.00(0.7H,s),6.11-6.41(2H,m),6.72(1H,dd,J=16.6,2.0Hz),6.95-7.00(2H,m),7.26(4H,ddt,J=55.1,21.O,10.OHz).

### Referential Example 143

2-[8-Chloro-6-(2,3-dimethoxy)-2-(2-methyl-1-propenyl)-4H,6H-pyrrolo[1,2-a][4,1]benzoxazepin-4-yl]ethyl methanesulfonate

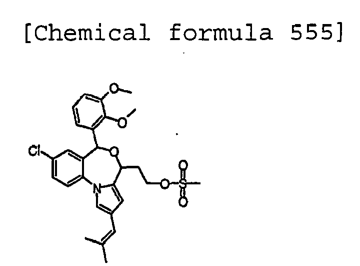

To a methylene chloride (35 mL) solution of 2-[8-chloro-6-(2,3-dimethoxy)-2-(2-methyl-1-propenyl)-4H,6H-pyrrolo[1,2-a][4,1]benzoxazepin-4-yl]-1-ethanol (581 mg) and triethylamine (268 µl) was added a methylene chloride (5.0 mL) solution of mesyl chloride (119 µl) under ice cooling. The resulting mixture was stirred at room temperature for 2 hours. The reaction mixture was washed successively with water and a saturated aqueous solution of sodium hydrogen carbonate and then, dried over anhydrous sodium sulfate. The solvent was distilled off under reduced pressure. The residue thus obtained was purified by column chromatography (silica gel, n-hexane/ethyl acetate=3/1) to give the title compound (698 mg). ¹H-NMR(CDCl₃)δ:1.55-1.88(7H,m),2.56(1H,s),2.93(3H,d,J=8.1Hz),3.45(3H,d,J=10.7Hz ),3.86(3H,s),4.04-4.48(2H,m),4.58(0.3H,dd,J=9.5,4.2Hz),5.33(0.7H,dd,J=8.8,6.1 Hz),5.75(0.3H,s),5.98(0.7H,s),6.08(0.7H,s),6.18(0.3H,s),6.2 3(0.3H,d,J=2.7Hz),6.41(0.7H,d,J=2.7Hz),6.68(0.7H,s),6.72(0. 3H,s),6.94-7.37(6H,m).

### Example 245

Ethyl 2-(2-{2-[8-chloro-6-(2,3-dimethoxy)-2-(2-methyl-1-propenyl)-4H,6H-pyrrolo[1,2-a][4,1]benzoxazepin-4-yl]ethyl}-2H-1,2,3,4-tetrazol-5-yl)acetate
Ethyl 2-(1-{2-[8-chloro-6-(2,3-dimethoxy)-2-(2-methyl-1-propenyl)-4H,6H-pyrrolo[1,2-a][4,1]benzoxazepin-4-yl]ethyl}-1H-1,2,3,4-tetrazol-5-yl)acetate

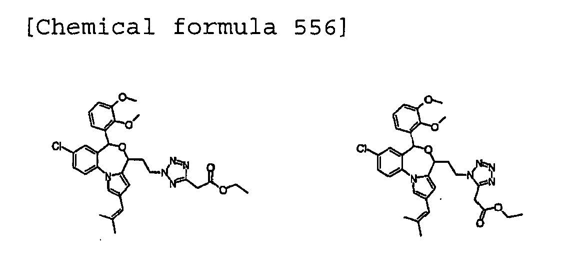

2-[8-Chloro-6-(2,3-dimethoxy)-2-(2-methyl-1-propenyl)-4H,6H-pyrrolo[1,2-a][4,1]benzoxazepin-4-yl]ethyl methanesulfonate (607 mg) was dissolved in N,N-dimethylformamide (50 mL). To the resulting solution were added ethyl 1H-tetrazol-5-acetate (534 mg), potassium carbonate (788 mg) and tetra-n-butylammonium iodide (421 mg). The resulting mixture was stirred at 40°C for one hour and then, at 80°C for 1.5 hours. After cooling to room temperature, the reaction mixture was diluted with ethyl acetate, washed successively with water (three times) and saturated brine and then, dried over anhydrous sodium sulfate. The solvent was distilled off under reduced pressure. The residue thus obtained was purified by thin layer chromatography (silica gel, n-hexane/ethyl acetate=3/1) to give a 2H-isomer (359 mg), the title compound having a lower polarity, and a 1H-isomer (185 mg), the title compound having a higher polarity.
2H-isomer:
¹H-NMR(CDCl₃)δ:1.26(3H,dd,J=6.7,5.0Hz),1.76-2.21(7.4H,m),2.78(0.6H,d,J=41.5Hz),3.46(3H,d,J=10.0Hz),3.88 (5H,d,J=17.8Hz),4.16(2H,dq,J=25.9,7.2Hz),4.49(2H,tt,J=19.7, 6.5Hz),4.80(0.4H,t,J=7.0Hz),5.30(0.6H,t,J=7.3Hz),5.76-6.38(3H,m),6.70(1H,d,J=19.3Hz),6.98(2H,t,J=13.8Hz),7.17-7.39(4H,m).

### Example 246

2-(2-{2-[8-Chloro-6-(2,3-dimethoxy)-2-(2-methyl-1-propenyl)-4H,6H-pyrrolo[1,2-a] [4,1]benzoxazepin-4-yl]ethyl}-2H-1,2,3,4-tetrazol-5-yl)acetic acid

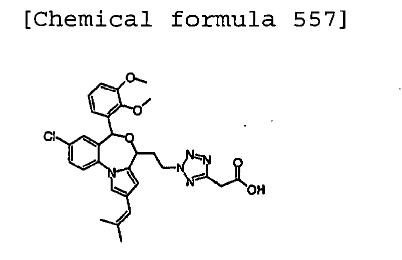

To a solution of ethyl 2-(2-{2-[8-chloro-6-(2,3-dimethoxy)-2-(2-methyl-1-propenyl)-4H,6H-pyrrolo[1,2-a][4,1]benzoxazepin-4-yl]ethyl}-2H-1,2,3,4-tetrazol-5-yl)acetate (30 mg) in a tetrahydrofuran (2.0 mL)/ethanol (4.0 mL) mixture was added an aqueous solution (2.0 mL) of potassium carbonate (21.0 mg). The resulting mixture was stirred at 50°C for 3 hours. After the reaction mixture was cooled to room temperature, a 1N aqueous hydrochloric acid solution was added to make it acidic, followed by concentration under reduced pressure. Water was added to the residue. The resulting mixture was extracted with methylene chloride (three times). The organic layers were combined and dried over anhydrous sodium sulfate. The solvent was distilled off under reduced pressure. The residue was purified by thin layer chromatography (silica gel, ethyl acetate) and lyophilized from dioxane and water to give the title compound (12.61 mg).

### Example 247

Methyl 2-[8-chloro-6-(2,3-dimethoxyphenyl)-4H,6H-pyrrolo[1,2-a][4,1]benzothiazepin-4-yl]acetate

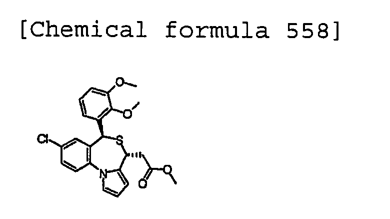

Methyl 3-(1-{4-chloro-2-[(2,3-dimethoxyphenyl)(hydroxy)methyl]phenyl}-1H-pyrrolo-2-yl)-2-propanoate (4.58 g) was dissolved in toluene (46 mL). Lawesson's reagent (4.33 g) was added and the resulting mixture was heated under reflux for 75 minutes in a nitrogen gas stream. The reaction mixture was cooled to room temperature. The solvent was distilled off under reduced pressure. The residue was purified by silica gel column chromatography (hexane:ethyl acetate=5:1) to give the title compound (1.79 g).
¹H-NMR(CDCl₃)δ:2.86(1H,dd,J=15.9,7.4Hz),3.11(1H,dd,J=15.7,7.6H z),3.39(3H,s),3.68(3H,s),3.82(3H,s),4.38(1H,t,J=7.5Hz),5.39 (1H,s),6.17-6.20(1H,m),6.33(1H,t,J=3.2Hz),6.85-6.95(3H,m),7.13(1H,t,J=8.1Hz),7.24-7.31(3H,m).

### Example 248

Methyl 2-[(4R,6S)-8-chloro-6-(2,3-dimethoxyphenyl)-4H,6H-pyrrolo[1,2-a][4,1]benzothiazepin-4-yl]acetate

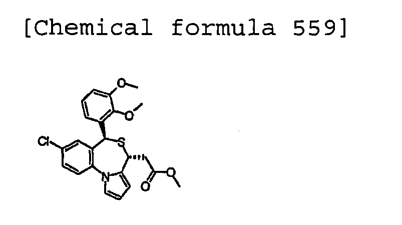

Methyl 2-[8-chloro-6-(2,3-dimethoxyphenyl)-4H,6H-pyrrolo[1,2-a][4,1]benzothiazepin-4-yl]acetate (1.43 g) was optically resolved using Shimadzu HPLC (flow rate: 50 mL/min, 50% 2-propanol-hexane) equipped with CHIRALPAK AD (Daicel Chemical, 50 mm × 250 mm) to give the title compound (0.62 g).

### Example 249

2-[(4R,6S)-8-Chloro-6-(2,3-dimethoxyphenyl)-4H,6H-pyrrolo[1,2-a][4,1]benzothiazepin-4-yl]acetic acid

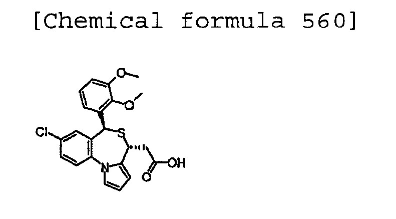

To a tetrahydrofuran-methanol-water mixed solution (1.0 mL-1.0 mL-1.0 mL) of methyl 2-[(4R,6S)-8-chloro-6-(2,3-dimethoxyphenyl)-4H,6H-pyrrolo[1,2-a][4,1]benzothiazepin-4-yl]acetate (173 mg) was added potassium carbonate (323 mg). The resulting mixture was stirred at 80°C for 13 hours. After the reaction mixture was cooled to room temperature, the solvent was distilled off under reduced pressure. To the residue were added dichloromethane and a 10% aqueous citric acid solution and the layers separated. The organic layer thus obtained was washed with saturated brine and dried over anhydrous sodium sulfate. The solvent was distilled off under reduced pressure. To the residue were added dichloromethane and hexane to solidify it. The resulting solid was collected by filtration and dried under reduced pressure to give the title compound (166 mg).
MS(ESI)m/z:430(M⁺+H).
¹H-NMR(CDCl₃)δ:2.90(1H,dd,J=16.3,7.0Hz),3.15(1H,dd,J=16.2,7.8H z),3.39(3H,s),3.83(3H,s),4.37(1H,t,J=7.6Hz),5.39(1H,s),6.20 -6.23(1H,m),6.34(1H,t,J=3.3Hz),6.86-6.88(1H,m),6.90-6.94(2H,m),7.14(1H,t,J=8.0Hz),7.23-7.31(3H,m).

### Example 250

Ethyl 2-(1-{2-[(4R,6S)-8-chloro-6-(2,3-dimethoxyphenyl)-4H,6H-pyrrolo[1,2-a] [4,1]benzothiazepin-4-yl]acetyl}-4-piperidinyl)acetate

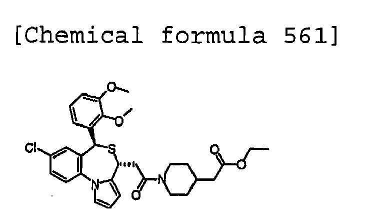

To a dichloromethane solution (5.5 mL) of 2-[(4R,6S)-8-chloro-6-(2,3-dimethoxyphenyl)-4H,6H-pyrrolo[1,2-a][4,1]benzothiazepin-4-yl]acetic acid (152 mg) were added ethyl 2-(4-piperidinyl)acetic acid (72.4 mg), 1-ethyl-3-(3-dimethylaminopropyl)carbodiimide hydrochloride (87.8 mg), and 1-hydroxybenzotriazole (16.2 mg). The resulting mixture was stirred at room temperature for 7.5 hours. To the reaction mixture were added distilled water and dichloromethane and the layers separated. The organic layer thus obtained was washed with saturated brine and dried over anhydrous sodium sulfate. The solvent was distilled off under reduced pressure. The residue was purified by silica gel column chromatography (chloroform-methanol) to give the title compound (121 mg).

### Example 251

2-(1{2-[(4R,6S)-8-Chloro-6-(2,3-dimethoxyphenyl)-4H,6H-pyrrolo[1,2-a][4,1]benzothiazepin-4-yl]acetyl}-4-piperidinyl)acetic acid

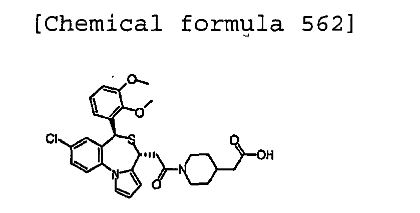

To a tetrahydrofuran-methanol-water mixed solution (2.0 mL-2.0 mL-2.0 mL) of ethyl 2-(1-{2-[(4R,6S)-8-chloro-6-(2,3-dimethoxyphenyl)-4H,6H-pyrrolo[1,2-a][4,1]benzothiazepin-4-yl]acetyl}-4-piperidinyl)acetate (118 mg) was added potassium carbonate (167 mg). The resulting mixture was stirred at 60°C for 3.5 hours. The solvent was distilled off under reduced pressure. To the residue were added dichloromethane and a 10% aqueous citric acid solution and the layers separated. The organic layer was washed with saturated brine and then dried over anhydrous sodium sulfate. The solvent was distilled off under reduced pressure. The residue thus obtained was purified by thin layer chromatography (dichloromethane:methanol=10:1) to give the title compound (98 mg).
MS(ESI)m/z:555(M⁺+H). ¹H-NMR(CDCl₃)δ:1.11-1.34(2H,m),1.70-1.91(2H,m),1.98-2.08(1H,m),2.26-2.33(2H,m),2.51-2.66(1H,m),2.89-3.00(1H,m),3.01-3.14(2H,m),3.38(3H,s),3.82(3H,s),3.90-4.02(1H,m),4.46-4.53(1H,m),4.54-4.66(1H,m),5.38(1H,s),6.12(1H,s),6.32(1H,s),6.81-6.94(3H,m),7.09-7.16(1H,m),7.23-7.31(3H,m).

### Referential Example 144

2-[(4R,6S)-8-Chloro-6-(2,3-dimethoxyphenyl)-4H,6H-pyrrolo[1,2-a][4,1]benzothiazepin-4-yl]-1-ethanol

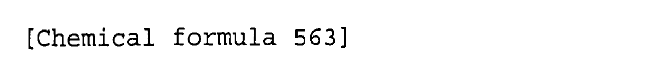

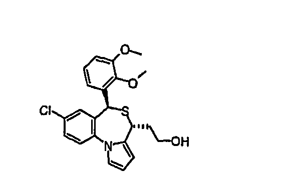

A tetrahydrofuran solution (4.5 mL) of methyl 2-[(4R,6S)-8-chloro-6-(2,3-dimethoxyphenyl)-4H,6H-pyrrolo[1,2-a][4,1]benzothiazepin-4-yl]acetate (205 mg) was cooled to -10°C. Lithium aluminum hydride (18.5 mg) was added to the resulting solution, followed by stirring for 30 minutes while warming to 0°C. To the reaction mixture were added a 1 mol/l aqueous solution (18 µl) of sodium hydroxide and distilled water (36 µl) at 0°C. The resulting mixture was stirred at room temperature for 10 minutes. The reaction mixture was filtered through Celite 545 and washed sufficiently with diethyl ether. The filtrates were combined and concentrated under reduced pressure to give the title compound (189 mg).
MS(ESI)m/z:416(M⁺+H). ¹H-NMR(CDCl₃)δ:1.82-1.88(1H,m),2.13-2.23(1H,m),2.34-2.45(1H,m),3.40(3H,s),3.72-3.77(2H,m),3.83(3H,s),4.07-4.12(1H,m),5.39(1H,s),6.20-6.23(1H,m),6.34-6.37(1H,m),6.85-6.88(1H,m),6.90-6.94(2H,m),7.14(1H,t,J=8.1Hz),7.23-7.30(3H,m).

### Example 252

Ethyl 2-(2-{2-[(4R,6S)-8-chloro-6-(2,3-dimethoxyphenyl)-4H,6H-pyrrolo[1,2-a][4,1]benzothiazepin-4-yl]ethyl}-2H-1,2,3,4-tetrazolo-5-yl)acetate
Ethyl 2-(2-{2-[(4R,6S)-8-chloro-6-(2,3-dimethoxyphenyl)-4H,6H-pyrrolo[1,2-a][4,1]benzothiazepin-4-yl]ethyl}-1H-1,2,3,4-tetrazolo-5-yl)acetate

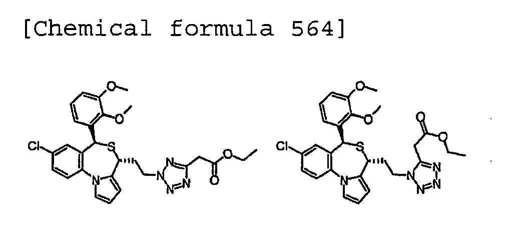

To a toluene solution (4.5 mL) of 2-[(4R,6S)-8-chloro-6-(2,3-dimethoxyphenyl)-4H,6H-pyrrolo[1,2-a][4,1]benzothiazepin-4-yl]-1-ethanol (185 mg) were added ethyl(1H-tetrazolo-5-yl)acetate (108 mg) and triphenylphosphine (234 mg). After the reaction mixture was cooled to 0°C, 40% diethylazodicarboxylate (toluene solution, 406 µl) was added dropwise thereto. The resulting mixture was stirred at room temperature for 1.5 hours and the solvent was distilled off under reduced pressure. The residue thus obtained was purified by silica gel column chromatography to give ethyl 2-(2-{2-[(4R,6S)-8-chloro-6-(2,3-dimethoxyphenyl)-4H,6H-pyrrolo[1,2-a][4,1]benzothiazepin-4-yl]ethyl}-2H-1,2,3,4-tetrazolo-5-yl)acetate (141 mg) having a lower polarity and ethyl 2-(2-{2-[(4R,6S)-8-chloro-6-(2,3-dimethoxyphenyl)-4H,6H-pyrrolo[1,2-a][4,1]benzothiazepin-4-yl]ethyl}-1H-1,2,3,4-tetrazolo-5-yl)acetate (54 mg) having a higher polarity. Ethyl 2-(2-{2-[(4R,6S)-8-chloro-6-(2,3-dimethoxyphenyl)-4H,6H-pyrrolo[1,2-a][4,1]benzothiazepin-4-yl]ethyl}-2H-1,2,3,4-tetrazolo-5-yl)acetate
MS(ESI)m/z:554(M⁺+H). ¹H-NMR(CDCl₃)δ:1.24-1.28(3H,m),2.54-2.64(1H,m),2.76-2.87(1H,m),3.40(3H,d,J=0.7Hz),3.83(3H,s),3.93(3H,s),3.94-3.99(1H,m),4.19(2H,q,J=7.1Hz),4.77(2H,t,J=7.2Hz),5.40(1H,s) ,6.24-6.27(1H,m),6.35-6.38(1H,m),6.86-6.95(3H,m),7.15(1H,t,J=8.0Hz),7.22-7.32(3H,m).
Ethyl 2-(2-{2-[(4R,6S)-8-chloro-6-(2,3-dimethoxyphenyl)-4H,6H-pyrrolo[1,2-a][4,1]benzothiazepin-4-yl]ethyl}-1H-1,2,3,4-tetrazolo-5-yl)acetate
MS(ESI)m/z:554(M⁺+H). ¹H-NMR(CDCl₃)δ:1.24-1.30(3H,m),2.53-2.64(1H,m),2.76-2.86(1H,m),3.40(3H,s),3.81-3.88(1H,m),3.83(3H,s),3.97-4.03(1H,m),4.16-4.36(4H,m),4.45-4.50(2H,m),5.40(1H,s),6.24-6.27(1H,m),6.36-6.39(1H,m),6.87-6.96(3H,m),7.15(1H,t,J=8.0Hz),7.19-7.26(2H,m),7.30(1H,dd,J=8.3,2.2Hz).

### Example 253

2-(2-{2-[(4R,6S)-8-Chloro-6-(2,3-dimethoxyphenyl)-4H,6H-pyrrolo[1,2-a][4,1]benzothiazepin-4-yl]ethyl}-2H-1,2,3,4-tetrazolo-5-yl)acetic acid

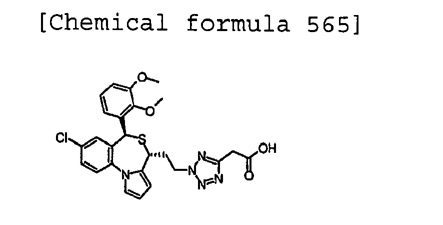

To a tetrahydrofuran-methanol-water (1.0 mL-1.0 mL-1.0 mL) mixed solution of ethyl 2-(2-{2-[(4R,6S)-8-chloro-6-(2,3-dimethoxyphenyl)-4H,6H-pyrrolo[1,2-a][4,1]benzothiazepin-4-yl]ethyl}-2H-1,2,3,4-tetrazolo-5-yl)acetate (138 mg) was added potassium carbonate (207 mg). The resulting mixture was stirred at 60°C for 6 hours. The solvent was distilled off under reduced pressure. To the residue were added dichloromethane and a 10% aqueous citric acid solution and the layers separated. The organic layer was washed with saturated brine and then dried over anhydrous sodium sulfate. The solvent was distilled off under reduced pressure. The residue was solidified by dichloromethane and hexane to give the title compound (189 mg).
MS(ESI)m/z:526(M⁺+H).
¹H-NMR(CDCl₃)δ:2.54-2.64(1H,m),2.76-2.87(1H,m),3.40(3H,s),3.83(3H,s),3.93-3.98(1H,m),4.00(2H,s),4.78(2H,t,J=7.4Hz),5.40(1H,s),6.23-6.27(1H,m),6.35-6.39(1H,m),6.86-6.95(3H,m),7.15(1H,t,J=8.0Hz),7.22-7.32(3H,m).

### Example 254

2-(2-{2-[(4R,6S)-8-Chloro-6-(2,3-dimethoxyphenyl)-4H,6H-pyrrolo[1,2-a][4,1]benzothiazepin-4-yl]ethyl}-1H-1,2,3,4-tetrazolo-5-yl)acetic acid

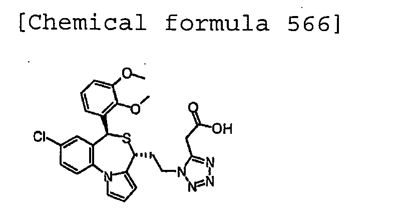

To a tetrahydrofuran-methanol-water (0.5mL-0.5mL-0.5mL) mixed solution of ethyl 2-(2-{2-[(4R,6S)-8-chloro-6-(2,3-dimethoxyphenyl)-4H,6H-pyrrolo[1,2-a][4,1]benzothiazepin-4-yl]ethyl}-1H-1,2,3,4-tetrazolo-5-yl)acetate (53 mg) was added potassium carbonate (79 mg). The resulting mixture was stirred at 60°C for 6 hours. The solvent was distilled off under reduced pressure. To the residue were added dichloromethane and a 10% aqueous citric acid solution and the layers separated. The organic layer was washed with saturated brine and then, dried over anhydrous sodium sulfate. The solvent was distilled off under reduced pressure. The residue was solidified by dichloromethane and hexane to give the title compound (50 mg).
MS (ESI) m/z : 526 (M⁺+H) .
¹H-NMR(CDCl₃)δ:2.54-2.64 (1H,m), 2.76-2.86(1H,m),3.39(3H,s),3.77-3.82(1H,m),3.83(3H,s),3.96(1H,dd,J=48.5,17.6Hz),4.11-, 4.25(1H,m),4.44-4.51(2H,m),5.40(1H,s),6.22-6.25(1H,m),6.36-6.40(1H,m),6.87-6.96(3H,m),7.15(1H,t,J=8.0Hz),7.19-7.33(3H,m).

### Referential Example 145

8-Chloro-6-(2,3-dimethoxyphenyl)-4H-pyrrolo[1,2-a][1]benzazepin-4-one

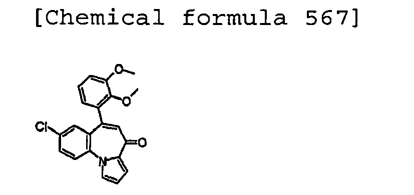

(5-Chloro-2-fluorophenyl)(2,3-dimethoxyphenyl)methanone (200 mg) and 2-acetylpyrrole (111 mg) were dissolved in dimethylformamide (5 mL). To the resulting solution was added sodium hydride (45 mg). The resulting mixture was stirred at 80°C for 3 days. To the reaction mixture were added 2-acetylpyrrole (111 mg) and sodium hydride (45 mg), followed by stirring for further 5 hours. After the reaction mixture was allowed to cool, water was added. The resulting mixture was extracted with ether, followed by washing with saturated brine. After drying over anhydrous sodium sulfate, the solvent was distilled off under reduced pressure. The residue thus obtained was purified by silica gel column chromatography (hexane-ethyl acetate) to give the title compound (64.7 mg).
¹H-NMR(CDCl₃)δ:3.52(3H,S),3.90(3H,S),6.61(1H,t,J=3.30HZ),6.68 ( 1H,s),6.98(1H,t,J=3.78Hz),7.03(1H,d,J=8.30Hz),7.19-7.12(3H,m),7.44-7.36(2H,m),7.56(1H,t,J=2.32Hz).

### Referential Example 146

8-Chloro-6-(2,3-dimethoxyphenyl)-5,6-dihydro-4H-pyrrolo[1,2-a][1]benzazepin-4-one

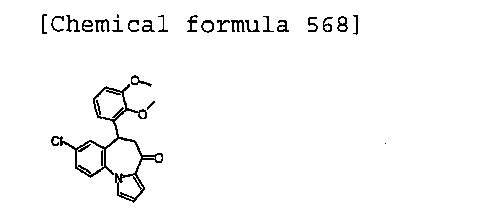

8-Chloro-6-(2,3-dimethoxyphenyl)-4H-pyrrolo[1,2-a][1]benzazepin-4-one (6.06 g) was dissolved in ethyl acetate (100 mL). To the resulting solution was added 5% rhodium-alumina catalyst (500 mg). The resulting mixture was hydrogenated at room temperature for 24 hours. After the catalyst was removed by filtration through celite, the solvent was distilled off under reduced pressure. The residue thus obtained was purified by silica gel column (hexane-ethyl acetate) to give the title compound (744.0 mg).
MS (ESI)m/z:368 (M⁺+H) .
¹H-NMR(CDCl₃)δ:3.13-3.23(2H,m),3.59(3H,s),3.89(3H,s),4.95(1H,dd,J=11.11,2.81Hz) ,6.47(1H,t,J=3.30Hz),6.77(1H,d,J=1.95Hz),6.82(1H,d,J=7.81Hz ),6.95(1H,d,J=8.30Hz),7.15(1H,t,J=8.06Hz),7.21-7.28(2H,m),7.28-7.25(2H,m).

### Referential Example 147

Ethyl 2-[8-chloro-6-(2,3-dimethoxyphenyl)-4-hydroxy-5,6-dihydro-4H-pyrrolo[1,2-a][1]benzazepin-4-yl]acetate

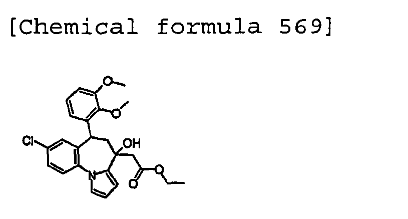

Ethyl acetate (528 µl) was dissolved in tetrahydrofuran (20 mL). To the resulting solution was added a lithium hexamethyldisilazide solution (1M solution, 5.41 mL) in portions at -78°C in a nitrogen atmosphere.
The resulting mixture was stirred for 30 minutes. A suspension of anhydrous cerium chloride (2.00 g) in tetrahydrofuran (10 mL) was added to the reaction mixture at -78°C through a cannula, followed by stirring for 30 minutes. A solution of 8-chloro-6-(2,3-dimethoxyphenyl)-5,6-dihydro-4H-pyrrolo[1,2-a][1]benzazepin-4-one (588 mg) in tetrahydrofuran (10 mL) was added in portions at -78°C, and the resulting mixture was stirred for 10 hours while warming to 0°C. After a saturated aqueous solution of ammonium chloride was added under ice cooling, ethyl acetate was added and the insoluble material was removed from the resulting mixture by filtration through celite. After extraction with ethyl acetate, washing with saturated brine and drying over anhydrous sodium sulfate, the solvent was distilled off under reduced pressure. The residue was purified by silica gel column chromatography (hexane-ethyl acetate) to give the title compound (509.2 mg).
¹H-NMR(CDCl₃)δ:1.07(3H,t,J=7.08Hz),2.32(2H,dd,J=60.06,14.65Hz) ,2.72-2.55(2H,m),3.42(3H,s),3.84(3H,s),3.87-4.03(2H,m),4.50-4.56(2H,m),6.26(1H,t,J=3.17Hz),6.33-6.35(1H,m),6.76-6.79(1H,m),6.87-6.93(3H,m),7.10(1H,t,J=8.06Hz),7.21(1H,d,J=8.54Hz),7.24-7.28(1H,m).

### Referential Example 148

Ethyl 2-[8-chloro-6-(2,3-dimethoxyphenyl)-5,6-dihydro-4H-pyrrolo[1,2-a][1]benzazepin-4-ylidene]acetate

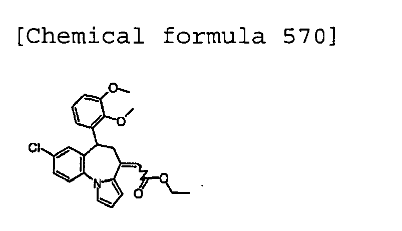

Ethyl 2-[8-chloro-6-(2,3-dimethoxyphenyl)-4-hydroxy-5,6-dihydro-4H-pyrrolo[1,2-a][1]benzazepin-4-yl]acetate (509 mg) was dissolved in dichloromethane (10 mL). Under ice cooling, triethylamine (624 µl) and methanesulfonic acid chloride (173 µl) were added and the resulting mixture was stirred for 1.5 hours while warming to room temperature. Triethylamine (624 µl) and methanesulfonic acid chloride (173 µl) were added and the resulting mixture was stirred for further 14 hours. After dichloromethane was added, the resulting mixture was washed with a saturated aqueous solution of sodium bicarbonate and dried over anhydrous sodium sulfate. The residue obtained by distilling off the solvent under reduced pressure was purified by silica gel column chromatography (hexane-ethyl acetate) to give the title compound (A) (153 mg) and the title compound (B) (265 mg).
The title compound (A)
¹H-NMR(CDCl₃)δ:1.28(3,H,t,J=7.08Hz),3.46(3H,s),3.63(1H,dt,J=19. 37,2.81Hz),3.78-3.88(4H,m),4.15(2H,q,J=7.08Hz),4.76(1H,dd,J=13.18,2.93Hz),6 .05(1H,t,J=2.32Hz),6.37(1H,t,J=3.17Hz),6.54(1H,q,J=1.79Hz), 6.73(1H,s),6.93(1H,dd,J=7.81,1.71Hz),7.08(1H,t,J=2.20Hz),7. 11-7.31(3H,m).
The title compound (B)
¹H-NMR(CDCl₃)δ:1.08-1.17(3H,m),3.29-3.49(2H,m),3.57(3H,s),3.87(3H,s),3.98-4.22(2H,m),5.00(1H,d,J=6.35Hz),6.10(1H,d,J=6.10Hz),6.49-6.35(3H,m),7.45-6.64(6H,m).

### Example 255

Ethyl 2-[8-chloro-6-(2,3-dimethoxyphenyl)-5,6-dihydro-4H-pyrrolo[1,2-a][1]benzazepin-4-yl]acetate

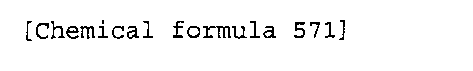

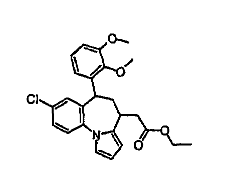

Ethyl 2-[8-chloro-6-(2,3-dimethoxyphenyl)-5,6-dihydro-4H-pyrrolo[1,2-a][1]benzazepin-4-ylidene]acetate (500.5 mg) was dissolved in ethyl acetate (10 mL). To the resulting solution was added 10% palladium-carbon (310 mg). The resulting mixture was hydrogenated at room temperature for 2 hours. After filtering off the catalyst, the solvent was distilled off under reduced pressure. The residue thus obtained was purified by silica gel column chromatography (hexane-ethyl acetate) to give the title compound (445.9 mg).

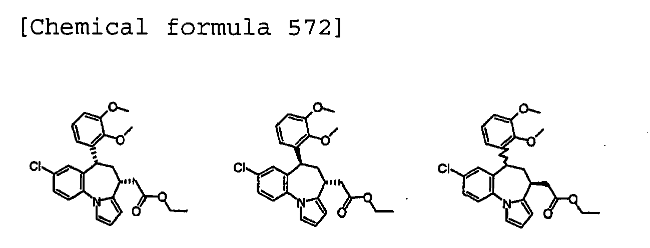

Ethyl 2-[8-chloro-6-(2,3-dimethoxyphenyl)-5,6-dihydro-4H-pyrrolo[1,2-a][1]benzazepin-4-yl]acetate (445.9 mg) was optically resolved using CHIRALCEL OD (Φ20 × 25cm) (10% isopropanol-n-hexane, flow rate: 15 ml/min) to give a first eluted fraction with a retention time of 6.5 minutes, a second eluted fraction with a retention time of 10 minutes and a third eluted fraction with a retention time of 16 minutes.

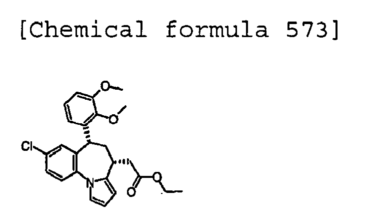

First eluted fraction (64.8 mg)
¹H-NMR(CDCl₃)δ:1.19(3H,t,J=7.08Hz),2.19-2.27(1H,m),2.64-2.71(1H,m),3.47(3H,s),3.61(1H,d,J=6.59Hz),3.80(3H,s),3.87(1 H,d,J=3.66Hz),4.11-4.02(2H,m),4.58(1H,t,J=8.06Hz),6.08(1H,q,J=1.63Hz),6.21(1H, t,J=3.17Hz),6.55-7.25(7H,m).

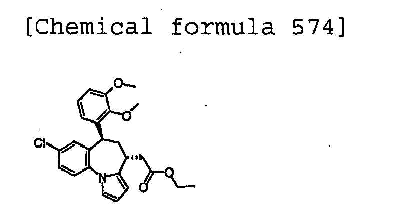

Second eluted fraction (98.3 mg)
¹H-NMR(CDCl₃)δ:1.24(3H,t,J=7.08Hz),1.90(1H,td,J=11.78,6.18Hz), 2.60-2.76(2H,m),2.87(1H,dd,J=15.50,6.71Hz),3.09-3.18(1H,m),3.34(3H,s),3.83(3H,s),4.13(2H,q,J=7.08Hz),4.32(1 H,dd,J=13.18,6.10Hz),6.03-6.07(1H,m),6.28(1H,t,J=3.17Hz),6.77(1H,s),6.90(1H,d,J=8.06H z),6.93(1H,t,J=2.20Hz),7.03(1H,d,J=6.59Hz),7.14(1H,t,J=8.06 Hz),7.23-7.28(2H,m).

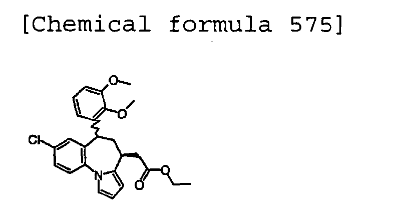

Third eluted fraction (139.1 mgl)

### Example 256

2-[(4R,6S)-8-Chloro-6-(2,3-dimethoxyphenyl)-5,6-dihydro-4H-pyrrolo[1,2-a][1]benzazepin-4-yl]acetic acid

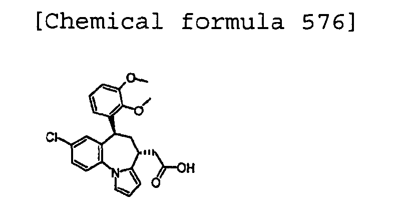

Ethyl 2-[(4R,6S)-8-chloro-6-(2,3-dimethoxyphenyl)-5,6-dihydro-4H-pyrrolo[1,2-a][1]benzazepin-4-yl]acetate (98.3 mg) was dissolved in a tetrahydrofuran-methanol-water (1:1:2) solvent mixture (4 mL). Potassium carbonate (309 mg) was added and the resulting mixture was stirred at 70°C for 17 hours. Under ice cooling, 1N hydrochloric acid was added to the reaction mixture to make it acidic, followed by extraction with ethyl acetate. The extract was washed with saturated brine and then dried over anhydrous sodium sulfate. The solvent was distilled off under reduced pressure to give the title compound (46.7 mg).
MS (ESI) m/z : 412 (M⁺+H). ¹H-NMR(CDCl₃)δ:1.91(1H,td,J=11.78,6.10Hz),2.66-2.79(2H,m),2.93(1H,dd,J=15.99,6.71Hz),3.07-3.18(1H,m),3.34(3H,s),3.83(3H,s),4.31(1H,q,J=6.43Hz),6.05-6.10(1H,m),6.29(1H,t,J=3.17Hz),6.77(1H,s),6.90(1H,dd,J=8.06 ,1.22Hz),6.95(1H,q,J=1.46Hz),7.02-7.07(1H,m),7.14(1H,t,J=8.06Hz),7.25(3H,br s).
Elemental analysis for: C₂₃H₂₂ClNO₄·1.25H₂O
Calculated: C,63.59;H,5.68;N,3.22.
Found: C,63.25;H,5.22;N,2.99.

### Example 257

Ethyl 2-(1-{2-[(4R,6S)-8-chloro-6-(2,3-dimethoxyphenyl)-5,6-dihydro-4-pyrrolo[1,2-a][1]benzazepin-4-yl]acetyl}-4-piperidinyl)acetate

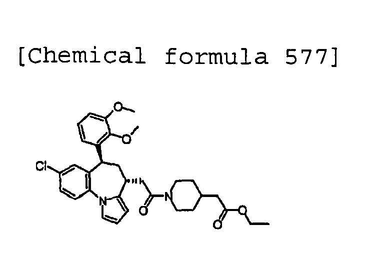

2-[(4R,6S)-8-Chloro-6-(2,3-dimethoxyphenyl)-5,6-dihydro-4H-pyrrolo[1,2-a][1]benzazepin-4-yl]acetic acid (42.9 mg) was dissolved in dichloromethane (5 mL). To the resulting solution were added ethyl piperidine-4-acetate (35.7 mg), 1-ethyl-3-(3-dimethylaminopropyl)carbodiimide hydrochloride (29.9 mg), and 1-hydroxybenzotriazole (8.0 mg). The resulting mixture was stirred at room temperature for 8 hours. Ethyl acetate was added and the resulting mixture was washed with 1N hydrochloric acid, a saturated aqueous solution of sodium bicarbonate and saturated brine and dried over anhydrous sodium sulfate. The solvent was distilled off under reduced pressure and the residue was purified by silica gel column chromatography (hexane-ethyl acetate) to give the title compound (39.2 mg). ¹H-NMR(CDCl₃)δ:1.20-1.07(2H,m),1.26(3H,t,J=7.08Hz),1.69-1.91(3H,m),1.96-2.08(1H,m),2.19-2.27(2H,m),2.52-2.72(2H,m),2.76-2.93(2H,m),3.00-3.11(1H,m),3.13-3.25(1H,m),3.33(3H,s),3.83(3H,s),3.91-3.98(1H,m),4.13(2H,q,J=7.08Hz),4.31(1H,q,J=6.59Hz),4.60(1H, d,J=11.23Hz),6.00(1H,br s),6.28(1H,t,J=3.17Hz),6.74-6.77(1H,m),6.89(1H,d,J=7.57Hz),6.91-6.94(1H,m),7.05-7.16(2H,m),7.19-7.29(2H,m).

### Example 258

2-(1-{2-[(4R,6S)-8-Chloro-6-(2,3-dimethoxyphenyl)-5,6-dihydro-4H-pyrrolo[1,2-a][1]benzazepin-4-yl]acetyl}-4-piperidinyl)acetic acid

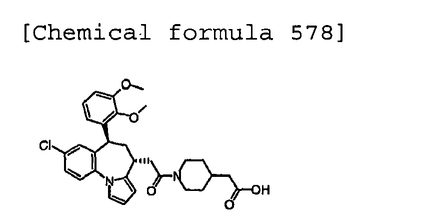

Ethyl 2-(1-{2-[(4R,6S)-8-chloro-6-(2,3-dimethoxyphenyl)-5,6-dihydro-4-pyrrolo[1,2-a][1]benzazepin-4-yl]acetyl}-4-piperidinyl)acetate (39.2 mg) was dissolved in a tetrahydrofuran-methanol-water (1:1:2) solvent mixture (4 mL). Potassium carbonate (38.3 mg) was added and the resulting mixture was stirred at 70°C for 5 hours. Under ice cooling, 1N hydrochloric acid was added to make the reaction mixture acidic, followed by extraction with ethyl acetate. The extract was washed with saturated brine and dried over anhydrous sodium sulfate. The solvent was then distilled off under reduced pressure to give the title compound (46.7 mg) .
¹H-NMR(CDCl₃)δ:1.10-1.34(3H,m),1.75-1.90(2H,m),1.97-2.10(1H,m),2.27-2.33(2H,m),2.51-2.94(4H,m),3.01-3.27(2H,m),3.33(3H,s),3.83(3H,s),3.92-4.01(1H,m),4.32(1H,q,J=6.35Hz),4.62(1H,d,J=14.40Hz),6.00(1H ,brs),6.28(1H,t,J=3.17Hz),6.76(1H,s),6.87-6.91(1H,m),6.92-6.95(1H,m),7.05-7.17(2H,m),7.24-7.21(2H,m).
Elemental analysis for: C₃₀H₃₃ClN₂O₅
Calculated: C,67.09;H,6.19;N,5.22.
Found: C,66.74;H,6.54;N,4.90.

### Referential Example 149

2-[(4R,6S)-8-Chloro-6-(2,3-dimethoxyphenyl)-5,6-dihydro-4H-pyrrolo[1,2-a][1]benzazepin-4-yl]-1-ethanol

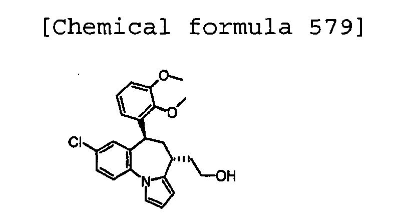

Ethyl 2-[(4R,6S)-8-chloro-6-(2,3-dimethoxyphenyl)-5,6-dihydro-4H-pyrrolo[1,2-a][1]benzazepin-4-yl]acetate (102 mg) was dissolved in tetrahydrofuran (5 mL). Under ice cooling, lithium aluminum hydride (11.5 mg) was added and the resulting mixture was stirred for 1 hour. Under ice cooling, water (25 µl), a 15% aqueous solution of sodium hydroxide (25 µl), and water (75 µl) were added, followed by stirring for 2 hours. After addition of anhydrous magnesium sulfate and drying thereover, the solvent was distilled off under reduced pressure. The residue was purified by silica gel column chromatography (hexane-ethyl acetate) to give 90.3 mg of the title compound. ¹H-NMR(CDCl₃)δ:1.21-1.29(2H,m),1.86-1.96(2H,m),2.14-2.23(1H,m),2.57-2.67(1H,m),2.73-2.84(1H,m),3.35(3H,s),3.76-3.85(5H,m),4.26-4.33(1H,m),6.06-6.09(1H,m),6.30(1H,t,J=3.05Hz),6.75-6.78(1H,m),6.88-6.95(2H,m),7.03-7.07(1H,m),7.15(1H,t,J=8.06Hz),7.24-7.23(2H,m).

### Example 259

Ethyl 2-(2-{2-[(4R,6S)-8-chloro-6-(2,3-dimethoxyphenyl)-5,6-dihydro-4H-pyrrolo[1,2-a][1]benzazepin-4-yl]ethyl}-2H-1,2,3,4-tetrazol-5-yl)acetate

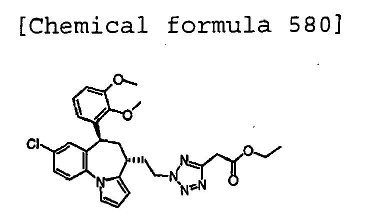

2-[(4R,6S)-8-Chloro-6-(2,3-dimethoxyphenyl)-5,6-dihydro-4H-pyrrolo[1,2-a][1]benzazepin-4-yl]-1-ethanol (90.3 mg) was dissolved in tetrahydrofuran (5 mL). To the resulting solution were added ethyl tetrazole acetate (46.1 mg), triphenylphosphine (77.4 mg) and 40% diethyldiazocarboxylate (145 µl) and the resulting mixture was stirred for 2 hours. The solvent was distilled off under reduced pressure. The residue was purified by silica gel column chromatography (hexane-ethyl acetate) to give 76.4 mg of the title compound. ¹H-NMR(CDCl₃)δ:1.25(3H,t,J=7.20Hz),1.90-2.00(1H,m),2.32-2.41(1H,m),2.55-2.70(3H,m),3.35(3H,s),3.84(3H,s),3.93(2H,s),4.15-4.34(4H,m),4.62-4.85(2H,m),6.13-6.17(1H,m),6.33(1H,t,J=3.17Hz),6.75-6.77(1H,m),6.90(1H,dd,J=8.30,1.46Hz),6.94-6.96(1H,m),7.00-7.04(1H,m),7.15(1H,t,J=8.06Hz),7.25-7.24(1H,m).

### Example 260

Ethyl 2-(2-{2-[(4R,6S)-1,8-dichloro-6-(2,3-dimethoxyphenyl)-5,6-dihydro-4H-pyrrolo[1,2-a][1]benzazepin-4-yl]ethyl}-2H-1,2,3,4-tetrazol-5-yl)acetate

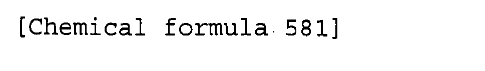

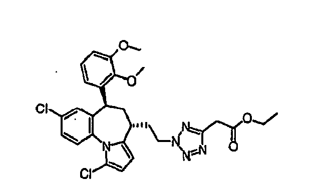

Ethyl 2-(2-{2-[(4R,6S)-8-chloro-6-(2,3-dimethoxyphenyl)-5,6-dihydro-4H-pyrrolo[1,2-a][1]benzazepin-4-yl]ethyl}-2H-1,2,3,4-tetrazol-5-yl)acetate (76.4 mg) was dissolved in tetrahydrofuran (3 mL). N-chlorosuccinimide (21.0 mg) was added and the resulting mixture was stirred for 16 hours. Water was added and the resulting mixture was extracted with chloroform. The extract was dried over anhydrous sodium sulfate. The solvent was distilled off under reduced pressure to give 58.1 mg of the title compound.
MS (ESI) m/z : 571 (M⁺+H).
¹H-NMR(CDCl₃)δ:1.26(3H,t,J=7.07Hz),1.92-1.821(1H,m),2.29-2.39(1H,m),2.46-2.59(3H,m),3.33(3H,s),3.83(3H,s),3.92(2H,s),4.19(3H,q,J=7.2
4Hz),4.60-4.82(2H,m),6.07(1H,d,J=3.66Hz),6.23(1H,d,J=3.66Hz),6.75-6.78(1H,m),6.88-7.01(2H,m),7.14(1H,t,J=7.93Hz),7.27-7.37(1H,m).

### Example 261

2-(2-{2-[(4R,6S)-8-Chloro-6-(2,3-dimethoxyphenyl)-5,6-dihydro-4H-pyrrolo[1,2-a][1]benzazepin-4-yl]ethyl}-2H-1,2,3,4-tetrazol-5-yl)acetic acid

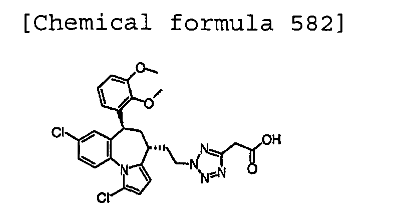

2-(2-{2-[(4R,6S)-1,8-dichloro-6-(2,3-dimethoxyphenyl)-5,6-dihydro- Ethyl 4H-pyrrolo[1,2-a][1]benzazepin-4-yl]ethyl}-2H-1,2,3,4-tetrazol-5-yl)acetate (58.1 mg) was dissolved in a tetrahydrofuran-methanol-water (1:1:2) solvent mixture (8 mL). Potassium carbonate (56.3 mg) was added and the resulting mixture was stirred at 55°C for 2 hours. Under ice cooling, 1N hydrochloric acid was added to make the reaction mixture acidic, followed by extraction with ethyl acetate. The extract was washed with saturated brine and dried over anhydrous sodium sulfate. The solvent was distilled off under reduced pressure to give the title compound (38.1 mg).
¹H-NMR(CDCl₃)δ:1.80-1.93(1H,m),2.25-2.42(1H,m),2.44-2.61(3H,m),3.33(3H,s),3.83(3H,s),3.98(2H,s),4.12-4.27(1H,m),4.56-4.89(2H,m),6.07(1H,d,J=3.66Hz),6.23(1H,d,J=3.66Hz),6.77(1H, s),6.87-7.02(2H,m),7.14(1H,t,J=7.93Hz),7.24-7.38(2H,m).
Elemental analysis for: C₂₆H₂₅Cl₂N₅O₄·1.5H₂O
Calculated: C,54.84;H,4.96;N,12.30.
Found: C,55.16;H,4.65;N,11.73.

### Referential Example 150

(5-Chloro-2-{2-[2-(1,3-dioxolane-2-yl)-1-hydroxyethyl]-1H-imidazol-1-yl}phenyl)(2,3-dimethoxyphenyl)methanone

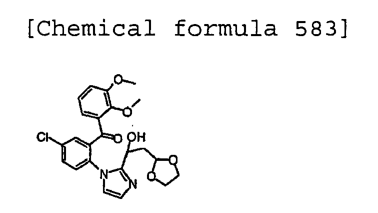

1-[4-Chloro-2-(2,3-dimethoxybenzoyl)phenyl]-1H-imidazol-2-carbaldehyde (500 mg) was dissolved in tetrahydrofuran (10 mL). To the resulting solution was added (1,3-dioxolan-2-ylmethyl)magnesium bromide (0.5M tetrahydrofuran solution, 4.04 mL). The resulting mixture was heated under reflux for 30 minutes in a nitrogen gas stream. After the reaction mixture was cooled to a temperature of ice cooling, water and ethyl acetate were added and the layers separated. The organic layer was washed with saturated brine and dried over anhydrous sodium sulfate. The solvent was distilled off under reduced pressure The residue was purified by silica gel column chromatography (chloroform:methanol=100:3) to give the title compound (516 mg).
MS(FAB)m/z:459(M⁺+H).
¹H-NMR(CDCl₃)δ:2.09-2.26(1H,m),2.33-2.43(1H,m),3.52(3H,s),3.80-3.89(5H,m),3.94-3.98(2H,m),4.76-4.82(1H,m),5.09(1H,br s),6.81-6.86(2H,m),7.01(3H,br s),7.25-7.28(1H,m),7.53-7.59(2H,m).

### Referential Example 151

trans-8-Chloro-6-(2,3-dimethoxyphenyl)-4-(1,3-dioxolan-2-ylmethyl)-4H, 6H-imidazo[1,2-a][4,1]benzoxazepine

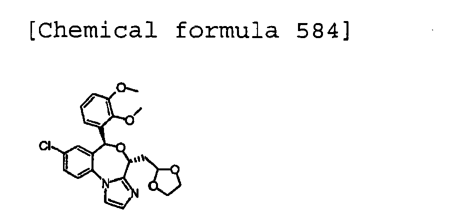

(5-Chloro-2-{2-[2-(1,3-dioxolan-2-yl)-1-hydroxyethyl]-1H-imidazol-1-yl}phenyl)(2,3-dimethoxyphenyl)methanone (34.5 g) was dissolved in ethanol (750 mL). Sodium borohydride (11.4 g) was added and the resulting mixture was stirred at room temperature for 2 hours. Ethyl acetate and a saturated aqueous solution of ammonium chloride were added to the reaction mixture and the layers separated. The organic layer was dried over anhydrous sodium sulfate. The solvent was distilled off under reduced pressure to give a pale yellow foam. The foam thus obtained was dissolved in dichloroethane (7500 mL). In a cooling bath containing methanol-ice, triethylamine (39.0 mL) and methanesulfonate chloride (8.73 mL) were added successively, followed by stirring overnight at room temperature. To the reaction mixture was added a 1N aqueous sodium hydroxide solution (2000 mL) and the layers separated. The organic layer was dried over anhydrous sodium sulfate. The solvent was distilled off under reduced pressure. The residue was purified twice by silica gel column chromatography (hexane:ethyl acetate= from 1:1 to 2:3) to give the title compound (10.1 g).
MS (FAB) m/z : 443 (M⁺+H).
¹H-NMR (CDCl₃) δ:2.56-2.60(3H,m), 3.48(3H,s), 3.83-3.99(5H,m),4.65-4.70(1H,m),5.27-5.31(1H,m), 5.66(1H,s), 6.77(1H,d,J=2.2Hz), 6.96(1H,d,J=8.3Hz) ,7.19(1H,t,J=8.1Hz), 7.2-7.27(1H,m), 7.31-7.42(4H,m).

### Referential Example 152

(4R,6S)-8-Chloro-6-(2,3-dimethoxyphenyl)-4-(1,3-dioxolan-2-ylmethyl)-4H,6H-imidazo[1,2-a][4,1]benzoxazepine (isomer A)

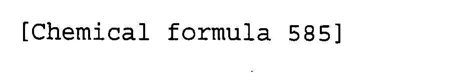

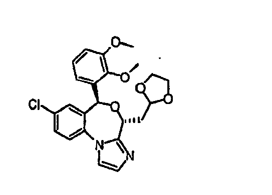

(4S,6R)-8-Chloro-6-(2,3-dimethoxyphenyl)-4-(1,3-dioxolan-2-ylmethyl)-4H,6H-imidazo[1,2-a][4,1]benzoxazepine (isomer B)

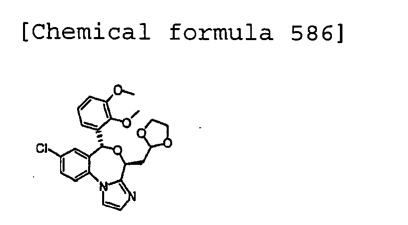

trans-8-Chloro-6-(2,3-dimethoxyphenyl)-4-(1,3-dioxolan-2-ylmethyl)-4H,6H-imidazo[1,2-a][4,1]benzoxazepine (10.1 g) was dissolved in a chloroform-hexane-isopropanol mixture (chloroform:hexane:isopropanol=1:10:3). The resulting solution was optically resolved using CHIRALCEL. OD (Daicel Chemical Industries, inner diameter: 5.0 cm, length: 50 cm, flow rate: 50 mL/min, hexane:isopropanol=13:7, preparative isolation: 11 times) to give the fraction eluted at 29 minutes as the isomer A (4.44 g) and the fraction eluted at 37 minutes as the isomer B (4.09 g).

Referential Example 153 2-[(4R,6S)-8-Chloro-6-(2,3-dimethoxyphenyl)-4H,6H-imidazo[1,2-a][4,1]benzoxazepin-4-yl]-1-ethanol

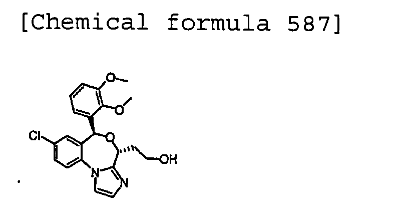

(4R,6S)-8-Chloro-6-(2,3-dimethoxyphenyl)-4-(1,3-dioxolan-2-ylmethyl)-4H,6H-imidazo[1,2-a][4,1]benzoxazepine (2.56 g) was dissolved in dichloromethane (350 mL). A 30% aqueous solution (360 mL) of perchloric acid was added and the resulting mixture was vigorously stirred overnight at room temperature. The organic layer was washed with a saturated aqueous solution of sodium hydrogen carbonate and then, dried over anhydrous sodium sulfate. The solvent was distilled off under reduced pressure. The residue was dissolved in tetrahydrofuran (100 mL). To the resulting solution were added water (20 mL) and sodium borohydride (1.22 g), followed by stirring at room temperature for 2 hours. Ethyl acetate and water were added to the reaction mixture and the layers separated. The organic layer was dried over anhydrous sodium sulfate. The solvent was distilled off under reduced pressure. The residue was purified by silica gel column chromatography (hexane:ethyl acetate=1:1-chloroform:methanol=100:3) to give the title compound (2.00 g).
MS(FAB)m/z:401(M⁺+H).
¹H-NMR(CDCl₃) δ:2.37-2.53 (2H,m), 3.50 (3H,s), 3.76-3.88 (major-4H,m),3.97-4.05(1H,m),4.68(1H,t,J=5.7Hz),5.68(major-1H,s),6.77-6.81(1H,m),6.93-6.99(1H,m),7.18-7.30(3H,m),7.35-7.45 (3H,m) .
Elemental analysis for: C₂₁H₂₁ClN₂O₄,0.25H₂O·0.3CHCl₃ Calculated: C,58.33;H,4.94;N,6.30.
Found: C,58.26;H,4.94;N,6.18.

### Referential Example 154

2-[(4S,6R)-8-Chloro-6-(2,3-dimethoxyphenyl)-4H,6H-imidazo[1,2-a][4,1]benzoxazepin-4-yl]-1-ethanol

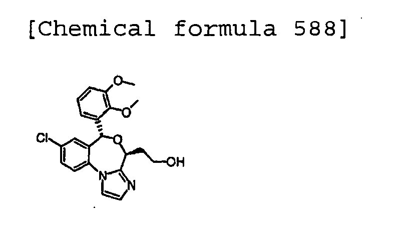

(4S,6R)-8-Chloro-6-(2,3-dimethoxyphenyl)-4-(1,3-dioxolan-2-ylmethyl)-4H,6H-imidazo[1,2-a][4,1]benzoxazepine (2.24 g) was dissolved in dichloromethane (200 mL). To the resulting solution was added a 30% aqueous solution (220 mL) of perchloric acid and the resulting mixture was vigorously stirred overnight at room temperature. The organic layer was washed with a saturated aqueous solution of sodium hydrogen carbonate and then dried over anhydrous sodium sulfate. The solvent was distilled off under reduced pressure. The residue was dissolved in tetrahydrofuran (100 mL). To the resulting solution were added water (20 mL) and sodium borohydride (750 mg). The resulting mixture was stirred at room temperature for 2 hours. To the reaction mixture were added ethyl acetate and water and the layers separated. The organic layer was dried over anhydrous sodium sulfate. The solvent was distilled off under reduced pressure. The residue was purified by silica gel column chromatography (hexane:ethyl acetate=1:1-chloroform:methanol=100:3) to give the title compound (1.74 g).
MS(FAB)m/z:401(M⁺+H).
¹H-NMR(CDCl₃)δ:2.37-2.53(2H,m),3.50(3H,s),3.76-3.88(major-4H,m),3.97-4.05(1H,m),4.68(1H,t,J=5.7Hz),5.68(major-1H,s),6.77-6.81(1H,m),6.93-6.99(1H,m),7.18-7.30(3H,m),7.35-7.45(3H,m).

### Example 262

Ethyl 1-{2-[(4R,6S)-8-chloro-6-(2,3-dimethoxyphenyl)-4H,6H-imidazo[1,2-a][4,1]benzoxazepin-4-yl]ethyl}-1H-pyrazole-4-carboxylate

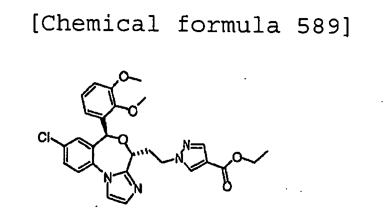

2-[(4R,6S)-8-Chloro-6-(2,3-dimethoxyphenyl)-4H,6H-imidazo[1,2-a][4,1]benzoxazepin-4-yl]-1-ethanol (450 mg) was dissolved in dichloromethane (22 mL). Triethylamine (425 µl) and chloromethanesulfonate (128 µl) were added successively and the resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a crudely purified product of 2-[(4R,6S)-8-chloro-6-(2,3-dimethoxyphenyl)-4H,6H-imidazo[1,2-a][4,1]benzoxazepin-4-yl]ethylmethanesulfonate. Ethyl 4-pyrazolecarboxylate (228 mg) was dissolved in N,N-dimethylformamide (13 mL). Sodium hydride (65 mg) was added to the resulting solution, followed by stirring at room temperature for one hour. Tetra-n-butylammonium iodide (408 mg) was added and then, the above-described crudely purified product was added. The resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue was purified by silica gel column chromatography (hexane:ethyl acetate= from 1:1 to 1:2) to give the title compound (459 mg).
MS(FAB)m/z:523(M⁺+H).
¹H-NMR(CDCl₃)δ:1.32(3H,t,J=7.1Hz),2.71-2.88(2H,m),3.48(3H,s),3.87(3H,s),4.26(2H,q,J=7.1Hz),4.41-4.57(3H,m),5.68(1H,s),6.78(1H,d,J=2.2Hz),6.98(1H,dd,J=8.0,1 .3Hz),7.21(1H,t,J=8.0Hz),7.25-7.30(2H,m),7.35(1H,d,J=8.5Hz),7.36-7.38(1H,m),7.41(1H,dd,J=8.5,2.2Hz),7.85(1H,s),7.90(1H,s).

### Example 263

1-{2-[(4R,6S)-8-Chloro-6-(2,3-dimethoxyphenyl)-4H,6H-imidazo[1,2-a][4,1]benzoxazepin-4-yl]ethyl}-1H-pyrazole-4-carboxylic acid

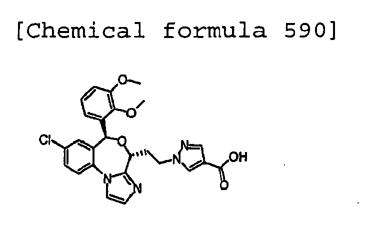

Ethyl 1-{2-[(4R,6S)-8-chloro-6-(2,3-dimethoxyphenyl)-4H,6H-imidazo[1,2-a][4,1]benzoxazepin-4-yl]ethyl}-1H-pyrazole-4-carboxylate (410 mg) was dissolved in methanol (20 mL). To the resulting solution was added a 1N aqueous sodium hydroxide solution (3.92 mL) and the resulting mixture was stirred under heat at 50°C for 90 minutes. After concentration of the reaction mixture under reduced pressure, 1N hydrochloric acid (3.92 mL) was added to the residue, followed by extraction with chloroform. The organic layer was washed with water and then dried over anhydrous sodium sulfate. The solvent was distilled off under reduced pressure. To the residue was added a 20:1 hexane:diethyl ether solvent mixture and the solid thus precipitated was collected by filtration to give the title compound (333 mg).
MS(FAB)m/z:495(M⁺+H).
¹H-NMR(CDCl₃) δ:2.72-2.94 (2H,m),3.52 (3H,s),3.87 (3H, s) 4.45-4.55(2H,m),4.62(1H,t,J=6.0Hz),5.69(1H,s),6.82(1H,d,J=2.2Hz) ,6.98(1H,d,J=8.5Hz),7.20(1H,t,J=8.1Hz),7.29(1H,d,J=8.1Hz),7 .40(1H,d,J=8.5Hz),7.43-7.48(2H,m),7.68(1H,s),8.01(1H,s),8.34(1H,s).
Elemental analysis for: C₂₅H₂₃ClN₄O₅
Calculated: C,60.67;H,4.68;N,11.32.
Found: C,60.32;H,4.80;N,10.93.

### Example 264

Ethyl 1-{2-[(4S,6R)-8-chloro-6-(2,3-dimethoxyphenyl)-4H,6H-imidazo[1,2-a][4,1]benzoxazepin-4-yl]ethyl}-1H-pyrazole-4-carboxylate

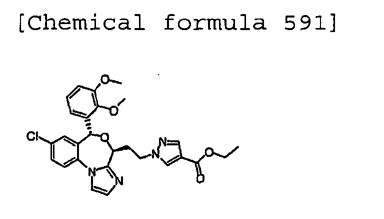

2-[(4S,6R)-8-Chloro-6-(2,3-dimethoxyphenyl)-4H,6H-imidazo[1,2-a] [4,1]benzoxazepin-4-yl]-1-ethanol (50 mg) was dissolved in dichloromethane (2 mL). To the resulting solution were added triethylamine (47 µl) and chloromethanesulfonate (14 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a crudely purified product of 2-[(4S,6R)-8-chloro-6-(2,3-dimethoxyphenyl)-4H,6H-imidazo[1,2-a][4,1]benzoxazepin-4-yl]ethylmethanesulfonate. Ethyl 4-pyrazolecarboxylate (26 mg) was dissolved in N,N-dimethylformamide (2 mL). Sodium hydride (8 mg) was added to the resulting solution, followed by stirring at room temperature for one hour. To the reaction mixture were added tetra-n-butylammonium iodide (45 mg) and then the crudely purified product. The resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue thus obtained was purified by preparative thin layer silica gel column chromatography (hexane:ethyl acetate=1:2) to give the title compound (57 mg). MS (FAB) m/z : 523 (M⁺+H). ¹H-NMR(CDCl₃)δ:1.32(3H,t,J=7.1Hz),2.71-2.88(2H,m),3.48(3H,s),3.87(3H,s),4.26(2H,q,J=7.1Hz),4.41-4.57(3H,m),5.68(1H,s),6.78(1H,d,J=2.2Hz),6.98(1H,dd,J=8.0,1 .3Hz),7.21(1H,t,J=8.0Hz),7.25-7.30(2H,m),7.35(1H,d,J=8.5Hz),7.36-7.38(1H,m),7.41(1H,dd,J=8.5,2.2Hz),7.85(1H,s),7.90(1H,s).

### Example 265

1-{2-[(4S,6R)-8-Chloro-6-(2,3-dimethoxyphenyl)-4H,6H-imidazo[1,2-a][4,1]benzoxazepin-4-yl]ethyl}-1H-pyrazole-4-carboxylic acid

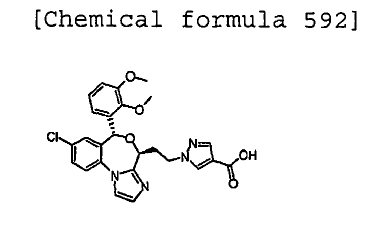

Ethyl 1-{2-[(4S,6R)-8-chloro-6-(2,3-dimethoxyphenyl)-4H,6H-imidazo[1,2-a][4,1]benzoxazepin-4-yl]ethyl}-1H-pyrazole-4-carboxylate (49 mg) was dissolved in methanol (1 mL). To the resulting solution was added a 1N sodium hydroxide aqueous solution (470 µl). The resulting mixture was stirred under heat at 50°C for 90 minutes. After concentration of the reaction mixture under reduced pressure, 1N hydrochloric acid (470 µl) was added to the residue and the mixture was extracted with chloroform. The organic layer was washed with water and then dried over anhydrous sodium sulfate. The solvent was distilled off under reduced pressure to give the title compound (45 mg).
MS(FAB)m/z:495(M⁺+H). ¹H-NMR(CDCl₃)δ:2.72-2.94(2H,m),3.52(3H,s),3.87(3H,s),4.45-4.55(2H,m),4.62(1H,t,J=6.0Hz),5.69(1H,s),6.82(1H,d,J=2.2Hz) ,6.98(1H,d,J=8.5Hz),7.20(1H,t,J=8.1Hz),7.29(1H,d,J=8.1Hz),7 .40(1H,d,J=8.5Hz),7.43-7.48(2H,m),7.68(1H,s),8.01(1H,s),8.34(1H,s).
Elemental analysis for: C₂₅H₂₃ClN₄O₅·0.5H₂O·0.3CHCl₃
Calculated: C,56.59;H,4.51;N,10.31.
Found: C,56.89;H,4.54;N,9.96.

### Referential Example 155

1-[4-Chloro-2-(2,3-dimethoxybenzoyl)phenyl]-1H-imidazo1-2-carbaldehyde

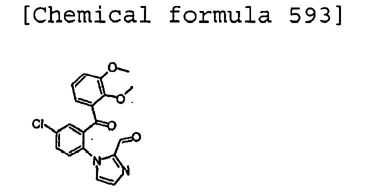

Imidazol-2-carbaldehyde (16 mg) was dissolved in dimethylsulfoxide (1 mL). At room temperature, sodium hydride (60% in oil, 7.0 mg) was added and the resulting mixture was stirred for one hour. To the reaction mixture was added (5-chloro-2-fluorophenyl)(2,3-dimethoxyphenyl)methanone (50 mg) and the resulting mixture was stirred under heat at 95°C for 2 days. After the reaction mixture was cooled to room temperature, ethyl acetate was added and the layers separated. The organic layer was dried over anhydrous sodium sulfate. The solvent was distilled off under reduced pressure. The residue was purified by preparative thin layer silica gel column chromatography (hexane:ethyl acetate=1:1) to give the title compound (34 mg). MS(FAB)m/z:371(M⁺+H). ¹H-NMR(CDCl₃)δ:3.61(3H,s),3.85(3H,s),6.86-6.91(1H,m),6.99-7.02(2H,m),7.20(2H,d,J=8.8Hz),7.28(1H,d,J=8.3Hz),7.57(1H,dd ,J=8.3,2.2Hz),7.60(1H,d,J=2.2Hz),9.66(1H,s).

### Referential Example 156

{5-Chloro-2-[4,5-dichloro-2-(1,3-dioxolan-2-yl)-1H-imidazol-1-yl]phenyl}(2,3-dimethoxyphenyl)methanone

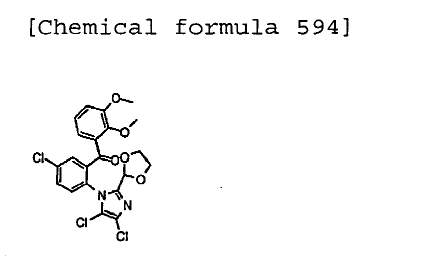

1-[4-Chloro-2-(2,3-dimethoxybenzoyl)phenyl]-1H-imidazol-2-carbaldehyde (5.00 g) was dissolved in toluene (150 mL). To the resulting solution were added ethylene glycol (3.62 mL) and p-toluenesulfonic acid hydrate (1.30 g). The resulting mixture was heated under reflux for 6 hours using a Dean-Stark trap. After cooling to room temperature, the reaction mixture was diluted with ethyl acetate and then washed successively with a saturated aqueous solution of sodium hydrogen carbonate and saturated brine. The organic layer was dried over anhydrous sodium sulfate. The solvent was distilled off under reduced pressure. The residue was dissolved in carbon tetrachloride (150 mL). N-chlorosuccinimide (4.67 g) was added and the resulting mixture was stirred under heat at 60°C for 80 minutes. The reaction mixture was concentrated under reduced pressure. The residue was purified by silica gel column chromatography (ϕ4.5 × 12cm, hexane:ethyl acetate=from 8:1 to 4:1) to give the title compound (3.16 g).
MS(ESI)m/z:483 (M⁺+H).
¹H-NMR(CDCl₃)δ:3.63(3H,s) ,3.76-3.85(4H,m),3.87(3H,s),5.68(1H,s),6.84-6.88(1H,m),6.99-7.03(2H,m),7.24-7.29(1H,m),7.61(1H,dd,J=8.4,2.3Hz),7.74(1H,d,J=2.3Hz).

### Referential Example 157

4,5-Dichloro-1-[4-chloro-2-(2,3-dimethoxybenzoyl)phenyl]-1H-imidazol-2-carbaldehyde

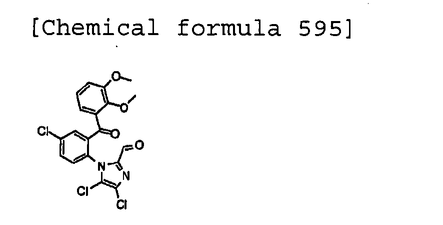

{5-Chloro-2-[4,5-dichloro-2-(1,3-dioxolan-2-yl)-1H-imidazol-1-yl]phenyl}(2,3-dimethoxyphenyl)methanone (3.16 g) was dissolved in tetrahydrofuran (50 mL). After a 70% aqueous solution (150 mL) of perchloric acid was added under ice cooling, the resulting mixture was stirred at room temperature for 20 minutes. To the reaction mixture were added ethyl acetate (750 mL), hexane (250 mL) and water (1000 mL) and the layers separated. The organic layer was washed successively with a saturated aqueous solution of sodium hydrogen carbonate and saturated brine and then dried over anhydrous sodium sulfate. The solvent was distilled off under reduced pressure. The residue was purified by silica gel column chromatography (ϕ3.0 × 14cm, hexane:ethyl acetate= from 4:1 to 2:1) to give the title compound (1.99 g).
MS(FAB)m/z:439(M⁺+H).
¹H-NMR(CDCl₃)δ:3.68(3H,s),3.88 (3H,s) 6.80-6.85(1H,m),7.02-7.06(2H,m),7.24-7.27(1H,m),7.65(1H,dd,J=8.3,2.4Hz),7.67-7.70(1H,m),9.47(1H,s).

### Referential Example 158

Ethyl (E)-3-{4,5-dichloro-1-[4-chloro-2-(2,3-dimethoxybenzoyl)phenyl]-1H-imidazol-2-yl}-2-propenoate

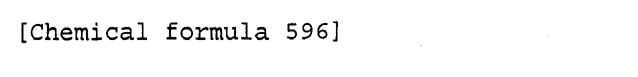

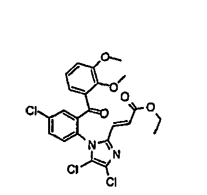

4,5-Dichloro-1-[4-chloro-2-(2,3-dimethoxybenzoyl)phenyl]-1H-imidazol-2-carbaldehyde (1.99 g) was dissolved in toluene (50 mL). To the resulting solution was added (trimethylphosphoranylidene)ethyl acetate (2.05 g). The resulting mixture was stirred at 80°C for 2 hours. The reaction mixture was concentrated under reduced pressure. The residue was purified by silica gel column chromatography (ϕ3.5 15cm, hexane:ethyl acetate=8:1) to give the title compound (1.91 g).
MS(EI)m/z:508(M⁺).
¹H-NMR(CDCl₃)×δ:1.28(3H,t,J=7.1Hz) ,3.61(3H,s),3.81(3H,s),4.17-4.24(2H,m),6.66(1H,d,J=15.5Hz),6.84-6.89(1H,m),6.94(1H,d,J=15.5Hz),6.99-7.04(2H,m),7.22(1H,d,J=8.3Hz),7.66(1H,dd,J=8.3,2.3Hz),7.74( 1H,d,J=2.3Hz).

### Example 266

Ethyl 2-[trans-1,2,8-trichloro-6-(2,3-dimethoxyphenyl)-4H,6H-imidazo[1,2-a][4,1]benzothiazepin-4-yl]acetate

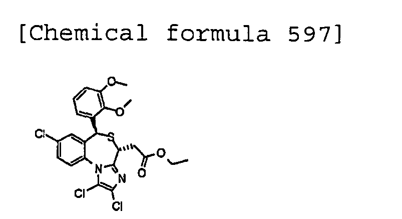

Ethyl (E)-3-{4,5-dichloro-1-[4-chloro-2-(2,3-dimethoxybenzoyl)phenyl]-1H-imidazol-2-yl}-2-propenoate (1.73 g) was dissolved in methanol (50 mL). Sodium borohydride (129 mg) was added and the resulting mixture was stirred at room temperature for 20 minutes. To the reaction mixture were added a saturated aqueous solution of ammonium chloride and ethyl acetate and the layers separated. The organic layer was dried over anhydrous sodium sulfate. The solvent was distilled off under reduced pressure. The residue was dissolved in toluene (30 mL). To the resulting solution was added Lawesson's reagent (1.53 g) and the resulting mixture was heated under reflux at room temperature for 36 hours. After concentration of the reaction mixture under reduced pressure, the residue was purified by silica gel column chromatography (ϕ4.0 × 13cm, hexane:ethyl acetate=20:1) to give the title compound (429 mg).
MS (ESI) m/z : 529 (M⁺+H).
¹H-NMR(CDCl₃) δ:1.25 (3H,t, J=7.1Hz),2.77 (1H, dd, J=17.1,5.8Hz) ,3.3 9-3.48(4H,m),3.85(3H,s),4.13(2H,q,J=7.1Hz),4.30(1H,dd,J=8.8,5 .8Hz),5.39(1H,s),6.94-6.98(2H,m),7.13-7.23(2H,m),7.31(1H,d,J=8.3Hz),7.40(1H,dd,J=8.3,2.2Hz).

### Referential Example 159

2-[trans-1,2,8-Trichloro-6-(2,3-dimethoxyphenyl)-4H,6H-imidazo[1,2-a][4,1]benzothiazepin-4-yl]-1-ethanol

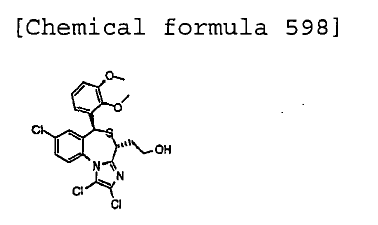

Ethyl 2-[trans-1,2,8-trichloro-6-(2,3-dimethoxyphenyl)-4H,6H-imidazo[1,2-a][4,1]benzothiazepin-4-yl]acetate (25 mg) was dissolved in tetrahydrofuran (1 mL). To the resulting solution were added water (0.1 mL) and sodium borohydride (27 mg). The resulting mixture was stirred at 40°C for 14 hours. After ethyl acetate and water were added to the reaction mixture and the layers separated, the organic layer was dried over anhydrous sodium sulfate. The solvent was distilled off under reduced pressure. The residue was purified by preparative thin layer silica gel column chromatography (200 × 200 × 1 mm, hexane:ethyl acetate=1:1) to give the title compound (18 mg) .
MS(FAB)m/z:485(M⁺+H).
¹H-NMR(CDCl₃)δ:2.12-2.22(1H,m),2.49-2.58(1H,m),3.43(3H,s),3.74-3.80(1H,m),3.84-3.94(4H,m),4.04-4.09(1H,m),5.38(1H,s),6.94-6.98(2H,m),7.16(1H,t,J=7.9Hz),7.22(1H,dd,J=7.9,1.5Hz),7.26-7.29(1H,m),7.37-7.41(1H,m).

### Example 267

Ethyl 2-(2-{2-[trans-1,2,8-trichloro-6-(2,3-dimethoxyphenyl)-4H,6H-imidazo[1,2-a][4,1]benzothiazepin-4-yl]ethyl}-2H-1,2,3,4-tetrazol-5-yl)acetate

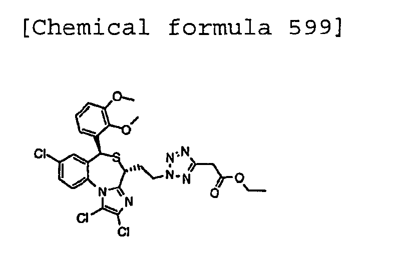

2-[trans-1,2,8-Trichloro-6-(2,3-dimethoxyphenyl)-4H,6H-imidazo[1,2-a][4,1]benzothiazepin-4-yl]-1-ethanol (18 mg) was dissolved in tetrahydrofuran (1 mL). To the resulting solution were added triphenylphosphine (14 mg), ethyl 2-(2H-1,2,3,4-tetrazol-5-yl)acetate (6.4 mg) and a 40% toluene solution (20 µl) of diethyldiazocarboxylate. The resulting mixture was stirred at room temperature for one hour. The reaction mixture was concentrated under reduced pressure. The residue was then purified by preparative thin layer silica gel column chromatography (200 × 200 × 1 mm, hexane:ethyl acetate=2:1) to give the title compound (14 mg). MS (FAB) m/z : 623 (M⁺+H) . ¹H-NMR(CDCl₃)δ:1.25(3H,t,J=7.2Hz),2.45-2.55(1H,m),2.96-3.06(1H,m),3.43(3H,s),3.80-3.85(4H,m),3.89(2H,s),4.15-4.21(2H,m),4.78-4.96(2H,m),5.39(1H,s),6.93-6.98(2H,m),7.15-7.18(2H,m),7.30(1H,d,J=8.5Hz),7.40(1H,dd,J=8.5,2.3Hz).

### Example 268

2-(2-{2-[trans-1,2,8-Trichloro-6-(2,3-dimethoxyphenyl)-4H,6H-imidazo[1,2-a] [4,1]benzothiazepin-4-yl]ethyl}-2H-1,2,3,4-tetrazol-5-yl)acetic acid

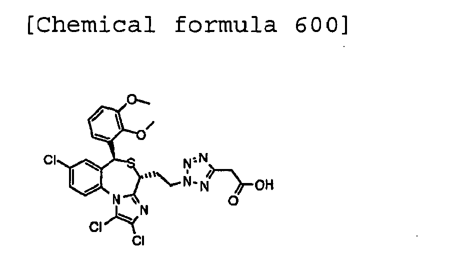

Ethyl 2-(2-{2-[trans-1,2,8-trichloro-6-(2,3-dimethoxyphenyl)-4H,6H-imidazo[1,2-a][4,1]benzothiazepin-4-yl]ethyl}-2H-1,2,3,4-tetrazol-5-yl)acetate (161 mg) was dissolved in methanol (30 mL). To the resulting solution were added water (8 mL) and anhydrous potassium carbonate (107 mg) and the resulting mixture was stirred under heat at 50°C for 2 hours. After concentration of the reaction mixture under reduced pressure, chloroform and 0.1N hydrochloric acid (15 mL) was added to the residue and the layers separated. After the organic layer was dried over anhydrous sodium sulfate, the solvent was distilled off under reduced pressure. The residue was solidified with diethyl ether-hexane to give the title compound (143 mg).
MS (EI)m/z:594 (M⁺) .
¹H-NMR(CDCl₃)δ:2.45-2.57(1H,m),2.96-3.06(1H,m),3.42(3H,s),3.80-3.84(1H,m),3.85(3H,s),3.97(2H,s),4.79-4.96(2H,m),5.39(1H,s),6.92-6.98(2H,m),7.15-7.17(2H,m),7.29(7H,d,J=8.5Hz),7.40(1H,dd,J=8.5,2.3Hz)
Elemental analysis for: C₂₄H₂₁ClN₆O₄
Calculated: C,48.38;H,3.55;N,14.10.
Found: C,48.53;H,3.70;N,13.89.

### Example 269

Methyl 3-(2-{[8-chloro-6-(2,3-dimethoxyphenyl)-4H,6H-imidazo[1,2-a][4,1]benzothiazepin-4-yl]methyl}-1,3-thiazol-5-yl)propanoate

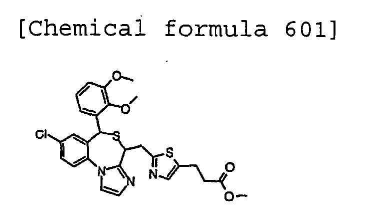

Methyl 3-(2-{[trans-7-chloro-5-(2,3-dimethoxyphenyl)-2-thioxo-1,2,3,5-tetrahydro-4,1-benzothiazepin-3-yl]methyl}-1,3-thiazol-5-yl)propanoate (50 mg) was dissolved in tetrahydrofuran (1 mL). To the resulting solution were added aminoacetaldehyde-dimethylacetal (15 µl) and mercury chloride (26 mg) successively. The resulting mixture was stirred at room temperature for 30 minutes. Ethyl acetate (5 mL) was added to the reaction mixture and a precipitate thus formed was filtered off. The filtrate was concentrated under reduced pressure. The residue was dissolved in N,N-dimethylformamide (1 mL). Tosic acid hydrate (17 mg) was added and the resulting mixture was stirred under heat at 100°C for 3 hours. The reaction mixture was diluted with ethyl acetate and washed with a saturated aqueous solution of sodium hydrogen carbonate. The organic layer was dried over anhydrous sodium sulfate. The solvent was distilled off under reduced pressure. The residue was purified by preparative thin layer silica gel column chromatography (hexane:ethyl acetate=1:1) to give the title compound (7 mg).
MS (FAB) m/z : 556 (M⁺+H).
HRMS-FAB(C₂₇H₂₇ClN₃O₄S₂) ;m/z:
Calculated (m/z):556.1132
Found (m/z):556.1108

### Referential Example 160

(5-Chloro-2-{3-[2-(1,3-dioxolan-2-yl)-1-hydroxyethyl]-5-methyl-4H-1,2,4-triazol-4-yl}-phenyl)(2,3-dimethoxyphenyl)methanone

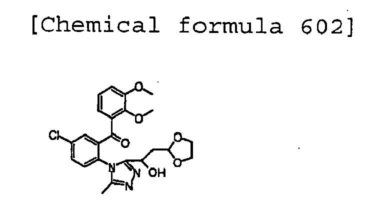

To a solution of 4-[4-chloro-2-(2,3-dimethoxybenzoyl)phenyl]-5-methyl-4H-1,2,4-triazol-3-carbaldehyde (200 mg) in tetrahydrofuran (10 mL) was added (1,3-dioxolan-2-ylmethyl)magnesium bromide (0.5M tetrahydrofuran) (2.08 mL). The resulting mixture was heated under reflux for 4 hours. After cooling the reaction mixture to room temperature, water and ethyl acetate were added thereto and the resulting mixture was filtered through celite. The filtrate was extracted twice with ethyl acetate. The organic layers were combined and dried over anhydrous sodium sulfate, followed by concentration under reduced pressure. The residue was purified by column chromatography (silica gel, chloroform/acetone=3/1, methylene chloride/methanol=9/1) to give the title compound (168 mg).
¹H-NMR(CDCl₃)δ:2.31(5H,dtt,J=58.6,17.5,7.4Hz),3.63(3H,t,J=7.0H z),3.76-3.99(7H,m),4.77(0.3H,q,J=5.8Hz),4.84(0.7H,t,J=4.6Hz),5.07(0 .3H,t,J=4.8Hz),5.17(0.7H,t,J=4.5Hz),6.89-7.40(4H,m),7.62(2H,q,J=3.5Hz).

### Referential Example 161

1-{4-[4-Chloro-2-(2,3-dimethoxybenzoyl)phenyl]-5-methyl-4H-1,2,4-triazol-3-yl}-2-(1,3-dioxolan-2-yl)ethylmethanesulfonate

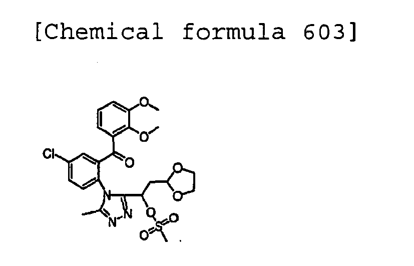

To a solution of (5-chloro-2-{3-[2-(1,3-dioxolan-2-yl)-1-hydroxyethyl]-5-methyl-4H-1,2,4-triazol-4-yl}-phenyl)(2,3-dimethoxyphenyl)methanone (375 mg) and 4-dimethylaminopyridine (290 mg) in dichloromethane (10 mL) was added dropwise a solution of methanesulfonyl chloride (181 mg) in dichloromethane (5 mL) under ice cooling. The resulting mixture was stirred overnight at room temperature. The reaction mixture was washed successively with a saturated aqueous solution of sodium hydrogen carbonate and saturated brine, dried over anhydrous sodium sulfate, and then concentrated under reduced pressure. The residue thus obtained was purified by column chromatography (silica gel, chloroform/methanol=19/1) to give the title compound (380 mg).
¹H-NMR(CDCl₃)δ:2.23-2.39(3.6H,m),2.56(1.4H,ddt,J=27.7,15.3,5.6Hz),2.99(3H,t,J=9 .0Hz),3.60-3.91(8H,m),5.00(0.3H,t,J=5.0Hz),5.07(0.7H,t,J=4.6Hz),5.76(0 .7H,dd,J=7.7,6.0Hz),5.82(0.3H,t,J=6.6Hz),6.97-7.69(6H,m).

### Referential Example 162

8-Chloro-6-(2,3-dimethoxyphenyl)-4-(1,3-dioxolan-2-ylmethyl)-1-methyl-4H,6H-[1,2,4]-triazolo[4,3-a][4,1]benzoxazepine

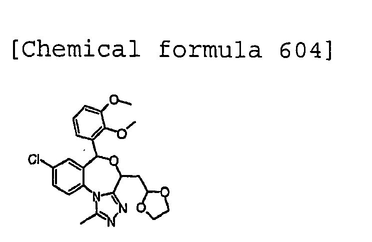

To a solution of 1-{4-[4-chloro-2-(2,3-dimethoxybenzoyl)phenyl]-5-methyl-4H-1,2,4-triazol-3-yl}-2-(1,3-dioxolan-2-yl)ethylmethanesulfonate (208 mg) in ethanol (5.0 mL) was added sodium borohydride (42.7 mg). The resulting mixture was stirred at room temperature for 90 minutes and then at 80°C for 1 hour. Water was added to the reaction mixture and the resulting mixture was extracted twice with ethyl acetate. The organic layers were combined, washed twice with water and saturated brine, and dried over anhydrous sodium sulfate. After concentration under reduced pressure, the residue was purified by thin layer chromatography (silica gel, chloroform/methanol=9/1) to give the title compound (91 mg).
¹H-NMR(CDCl₃)δ:2.32-2.40(5H,m),3.49-3.58(3H,m),3.78-3.99(7H,m),5.17-5.26(2H,m),6.00(1H,s),6.80-7.46(6H,m).

### Referential Example 163

2-[8-Chloro-6-(2,3-dimethoxyphenyl)-1-methyl-4H,6H-[1,2,4]triazolo[4,3-a][4,1]-benzoxazepin-4-yl]-1-ethanol

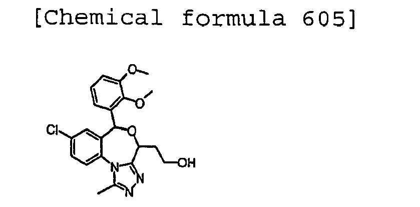

To a mixed solution of a 70% aqueous solution (20 mL) of perchloric acid, water (20 mL), and dichloromethane (30 mL) was added a solution of 8-chloro-6-(2,3-dimethoxyphenyl)-4-(1,3-dioxolan-2-ylmethyl)-1-methyl-4H,6H-[1,2,4]-triazolo[4,3-a][4,1]benzoxazepine (161 mg) in dichloromethane (40 mL). The resulting mixture was vigorously stirred at room temperature for one hour. The reaction mixture was separated into an organic layer and a water layer. The organic layer was washed with a saturated aqueous solution of sodium hydrogen carbonate and dried over anhydrous sodium sulfate. After filtration, the filtrate was concentrated under reduced pressure. The residue thus obtained was dissolved in ethanol (5.0 mL).
To the resulting solution was added sodium borohydride (35.4 mg). The resulting mixture was stirred at room temperature for 1 hour. After addition of water, the resulting mixture was extracted twice with ethyl acetate. The organic layers were combined, washed with saturated brine and dried over anhydrous sodium sulfate. After filtration, the filtrate was concentrated under reduced pressure. The residue thus obtained was purified by thin layer chromatography (chloroform/methanol=9/1) to give the title compound (109 mg).
¹H-NMR(CDCl₃)δ:2.42-2.67(5H,m),3.51(2.7H,s),3.55(0.3H,s),3.80-3.98(5H,m),4.65(0.1H,d,J=6.3Hz),5.10(0.9H,s),5.60(0.1H,s),6 .06(0.9H,s),6.69-7.48(6H,m).
MS(ESI)m/z:416(M⁺+H).

### Example 270

Ethyl 2-(2-{2-[8-chloro-6-(2,3-dimethoxyphenyl)-1-methyl-4H,6H-[1,2,4]triazolo[4,3-a][4,1]-benzoxazepin-4-yl]ethyl}-2H-1,2,3,4-tetrazol-5-yl)acetate
Ethyl 2-(2-{2-[8-chloro-6-(2,3-dimethoxyphenyl)-1-methyl-4H, 6H-[1,2,4]triazolo[4,3-a][4,1]-benzoxazepin-4-yl]ethyl}- 1H-1,2,3,4-tetrazol-5-yl)acetate

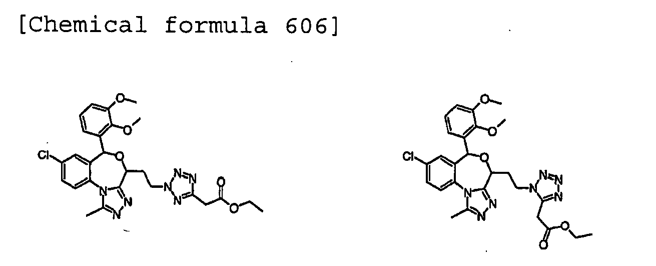

To a solution of 2-[8-chloro-6-(2,3-dimethoxyphenyl)-1-methyl-4H,6H-[1,2,4]triazolo[4,3-a][4,1]-benzoxazepin-4-yl]-1-ethanol (109 mg) and triethylamine (55 µL) in dichloromethane (10 mL) was added a solution of methanesulfonyl chloride (36.0 mg) in dichloromethane (3.0 mL) under ice cooling. The resulting mixture was stirred overnight at room temperature. To the reaction mixture were added triethylamine (110 µL) and methanesulfonyl chloride (60 mg) further. After stirring at room temperature for 30 minutes, the reaction mixture was washed with a saturated aqueous solution of sodium hydrogen carbonate and water and dried over anhydrous sodium sulfate. After filtration, the filtrate was concentrated under reduced pressure. The residue thus obtained was dissolved in N,N-dimethylformamide (15 mL). To the resulting solution were added ethyl 1H-tetrazol-5-acetate (123 mg), potassium carbonate (181 mg) and tetrabutylammonium iodide (96.8 mg). The resulting mixture was stirred at room temperature for one hour, at 40°C for one hour, and at 80°C for 2 hours. After addition of water, the resulting mixture was extracted with ethyl acetate. The organic layer was washed successively with water (twice) and saturated brine, and dried over anhydrous sodium sulfate. After filtration, the filtrate was concentrated under reduced pressure. The residue was separated and purified by thin layer chromatography (chloroform/methanol=19/1) to give the respective title compounds.
Low polarity fraction (2H-isomer):61.4 mg
¹H-NMR(CDCl₃)δ:1.25(3H,t,J=7.1Hz),2.36(0.3H,s),2.44(2.7H,s),2. 78(2H,d,J=6.1Hz),3.51(3H,d,J=8.3Hz),3.81(3H,s),3.92(2H,s),4 .19(2H,dt,J=11.9,5.0Hz),4.90-4.92(3H,m),5.61(0.1H,s),6.07(0.9H,s),6.68-7.45(6H,m).
High polarity fraction (1H-isomer):40.2 mg
¹H-NMR(CDCl₃)δ:1.27(3H,t,J=7.2Hz),2.36(0.3H,s),2.40(2.7H,s),2. 74(2H,s),3.49(0.3H,s),3.50(2.7H,s),3.81(3H,s),3.82-4.12(2H,m),4.19(2H,q,J=7.2Hz),4.56-4.66(2.1H,m),4.93(0.9H,s),5.61(0.1H,s),6.05(0.9H,s),6.63-7.47(6H,m).

### Example 271

2-(2-{2-[8-Chloro-6-(2,3-dimethoxyphenyl)-1-methyl-4H,6H-[1,2,4]triazolo[4,3-a][4,1]-benzoxazepin-4-yl]ethyl}-2H-1,2,3,4-tetrazol-5-yl)acetic acid

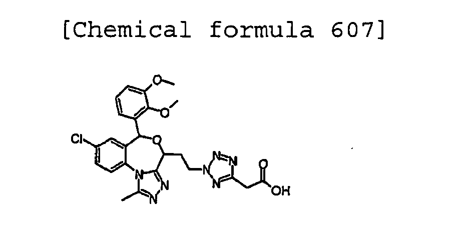

A solution of ethyl 2-(2-{2-[8-chloro-6-(2,3-dimethoxyphenyl)-1-methyl-4H,6H-[1,2,4]triazolo(4,3-a][4,1]-benzoxazepin-4-yl]ethyl}-2H-1,2,3,4-tetrazol-5-yl)acetate (61.4 mg) and potassium carbonate (46.0 mg) in a tetrahydrofuran (4.0 mL)/ethanol (8.0 mL)/water (4.0 mL) solvent mixture was stirred at 50°C for 3 hours. To the reaction mixture was added a 1N aqueous hydrochloric acid solution to make it acidic, followed by dilution with water. The resulting solution was extracted twice with ethyl acetate. The organic layers were combined, dried over anhydrous sodium sulfate and concentrated under reduced pressure. The residue thus obtained was purified by thin layer chromatography (chloroform/methanol/water=7/3/1) to give the title compound (31.8 mg).
¹H-NMR(CDCl₃)δ:2.42(3H,s),2.64(2H,br s),3.49(3H,s),3.83(5H,m,J=11.8Hz),4.81(2H,s),5.01(1H,s),5.5 9(0.1H,s),6.00(0.9H,s),6.74(1H,s),6.84(1H,d,J=8.1Hz),6.99(1 H,t,J=8.0Hz),7.16-7.27(2H,m),7.43(1H,dd,J=8.6,2.5Hz).
IR (ATR) cm⁻
¹:1720,1587,1479,1429,1269,1221,1186,1068,1049,1001,826,760.
HRMS(FAB)m/z:
Calculated: C₂₄H₂₅N₇O₅Cl;526.1606.
Found: 526.1566.
Elemental analysis for: C₂₄H₂₄N₇O₅Cl·1.50H₂O
Calculated: C,52.13;H,4.92;N,17.73.
Found: C,52.28;H,4.52;N,17.31.

### Example 272

2-(2-{2-[8-chloro-6-(2,3-dimethoxyphenyl)-1-methyl-4H,6H-[1,2,4]triazolo[4,3-a][4,1]-benzoxazepin-4-yl]ethyl}-1H-1,2,3,4-tetrazol-5-yl)acetic acid

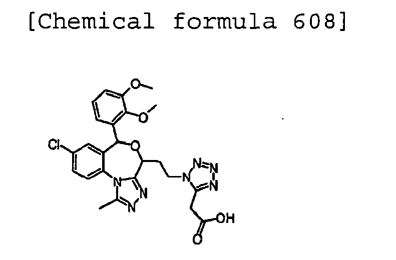

A solution of ethyl 2-(2-{2-[8-chloro-6-(2,3-dimethoxyphenyl)-1-methyl-4H,6H-[1,2,4]triazolo[4,3-a][4,1]-benzoxazepin-4-yl]ethyl}-1H-1,2,3,4-tetrazol-5-yl)acetate (40 mg) and potassium carbonate (30.0 mg) in a tetrahydrofuran (4.0 mL)/ethanol (8.0 mL)/water (4.0 mL) mixture was stirred at 50°C for 3 hours. To the reaction mixture was added a 1N aqueous hydrochloric acid solution to make it acidic, followed by dilution with water. The resulting solution was extracted twice with ethyl acetate. The organic layers were combined, dried over anhydrous sodium sulfate and then concentrated under reduced pressure. The residue thus obtained was purified by thin layer chromatography (chloroform/methanol/water=7/3/1) to give the title compound (18.1 mg).
¹H-NMR(CDCl₃)δ:2.29(5H,br s),3.43(3H,br s),3.77-3.83(5H,m),4.43(2H,br s),4.99(1H,br s),5.58(0.1H,s),5.97(0.9H,s),6.78-6.79(2H,m), 6.92(1H,br s),7.21-7.35(3H,m). IR(ATR)cm⁻
¹:1608,1481,1429,1375,1269,1221,1176,1066,1047,1001,825,760
HRMS(FAB)m/z:
Calculated: C₂₄H₂₅N₇O₅Cl;526.1606.
Found: 526.1582.

### Referential Example 164

3-[8-Chloro-6-(2,3-dimethoxyphenyl)-1-methyl-4H,6H-[1,2,4]triazolo[4,3-a][4,1]-benzoxazepin-4-yl]propanenitrile

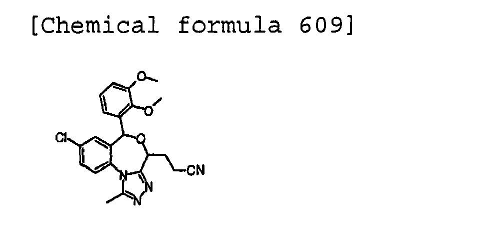

To a solution of 2-[8-chloro-6-(2,3-dimethoxyphenyl)-1-methyl-4H,6H-[1,2,4]triazolo[4,3-a][4,1]-benzoxazepin-4-yl]-1-ethanol (279 mg) and triethylamine (420 µL) in dichloromethane (10 mL) was added a solution of methanesulfonyl chloride (245 mg) in dichloromethane (5.0 mL) under ice cooling. The resulting solution was stirred at room temperature for 30 minutes. The reaction mixture was washed with a saturated aqueous solution of sodium hydrogen carbonate and dried over anhydrous sodium sulfate. The filtrate was dried under reduced pressure. The residue thus obtained was dissolved in dimethylsulfoxide (20 mL). To the resulting solution was added sodium cyanide (76.1 mg), followed by stirring overnight at 50°C. Water was added to the reaction mixture under ice cooling. The resulting mixture was extracted three times with ethyl acetate. The organic layers were combined, washed successively with a saturated aqueous solution of sodium hydrogen carbonate and saturated brine, and dried over anhydrous sodium sulfate. After filtration, the filtrate was concentrated under reduced pressure. The residue thus obtained was purified by column chromatography (silica gel, dichloromethane/methanol=20/1) to give the title compound (296 mg).
¹H-NMR(CDCl₃)δ:1.59(3H,s),2.43(4H,br s),2.57-2.82(4H,m),3.49(0.3H,s),3.52(2.7H,s),3.81(2.7H,s),3.87(0.3H ,s),4.58(0.1H,dd,J=7.2,5.5Hz),5.01(0.9H,s),5.60(0.1H,s),6.0 8(0.9H,s),6.66(1H,d,J=7.3Hz),6.84(1H,dd,J=6.3,1.7Hz),6.96-7.01(1H,m),7.20-7.25(1H,m),7.33(1H,s),7.49(1H,ddd,J=15.8,8.5,2.4Hz).
MS(ESI)m/z:425(M⁺+H).

### Example 273

3-[8-Chloro-6-(2,3-dimethoxyphenyl)-1-methyl-4H,6H-[1,2,4]triazolo[4,3-a] [4,1]-benzoxazepin-4-yl]propanoic acid

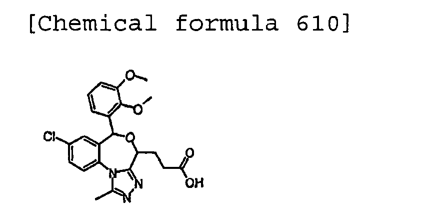

To a solution of 3-[8-chloro-6-(2,3-dimethoxyphenyl)-1-methyl-4H,6H-[1,2,4]triazolo[4,3-a] [4,1]-benzoxazepin-4-yl]propanenitrile (295 mg) in isopropyl alcohol (15 mL) was added a 10N aqueous sodium hydroxide solution (15 mL). The resulting mixture was stirred at 70°C for 3 days. With 6N hydrochloric acid, the reaction mixture was made acidic, followed by successive extraction with ethyl acetate (twice) and dichloromethane (twice). The organic layers were combined and dried over anhydrous sodium sulfate. After filtration, the filtrate was concentrated under reduced pressure to give the title compound (212 mg).
¹H-NMR(CD₃OD)δ:2.38(3H,s),2.50-2.61(2H,m),3.35-3.44(5H,m),3.82(3H,d,J=33.9Hz),4.53(0.2H,t,J=6.5Hz),4.86(0.
8H,s),5.52(0.2H,s),6.05(0.8H,s),6.87-7.16(3H,m),7.53-7.60(3H,m).
IR(ATR)cm⁻¹:1736,1481,1431,1323,1269,1192,1109,1051,1007.
HRMS(FAB)m/z:
Calculated: C₂₂H₂₃N₃O₅Cl;444.1326.
Found: 444.1347.
Elemental analysis for: C₂₂H₂₂N₃O₅Cl·0.75H₂O
Calculated: C,57.77;H,5.18;N,9.19.
Found: C,58.04;H,5.07;N,8.83.

### Example 274

Ethyl 2-(1-{3-[8-chloro-6-(2,3-dimethoxyphenyl)-1-methyl-4H,6H-[1,2,4]triazolo[4,3-a][4,1]-benzoxazepin-4-yl]propanoyl}-4-piperidinyl)acetate

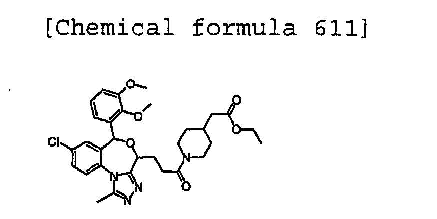

To a solution of 3-[8-chloro-6-(2,3-dimethoxyphenyl)-1-methyl-4H,6H-[1,2,4]triazolo[4,3-a][4,1]-benzoxazepin-4-yl]propanoic acid (100 mg), ethyl 2-(4-piperidinyl)-acetate (57.9 mg), 1-hydroxybenzotriazole (45.7 mg) and N-methylmorpholine (49.5 µL) in N,N-dimethylformamide (20 mL) was added 1-ethyl-3-(3-dimethylaminopropyl)carbodiimide hydrochloride (64.8 mg). The resulting mixture was stirred overnight at room temperature. After concentration of the reaction mixture under reduced pressure, ethyl acetate was added to the concentrate. The resulting mixture was washed twice with water and once with saturated brine and then, dried over anhydrous sodium sulfate. After filtration, the filtrate was concentrated under reduced pressure. The residue was purified by thin layer chromatography (dichloromethane/methanol=9/1) to give the title compound (111 mg) .
¹H-NMR(CDCl₃)δ:1.05-1.27(5H,m), 1.73(2H,t,J=17.1Hz), 1.99-2.23(5H,m),2.58(6H,dt,J=55.7,20.8Hz),2.95(1H,dt,J=27.1,12.3 Hz),3.49(3H,t,J=6.5Hz),3.84(4H,d,J=19.5Hz),4.13(2H,q,J=7.2H z),4.52-4.55(1.2H,m),5.08(0.8H,s),5.56(0.2H,s),5.99(0.8H,s),6.84(2H ,t,J=12.3Hz),7.01(1H,dt,J=16.4,7.0Hz),7.19-7.31(2H,m),7.44(1H,ddd,J=16.5,8.5,2.4Hz).

### Example 275

2-(1-{3-[8-Chloro-6-(2,3-dimethoxyphenyl)-1-methyl-4H,6H-[1,2,4]triazolo[4,3-a][4,1]-benzoxazepin-4-yl]propanoyl}-4-piperidinyl)acetic acid

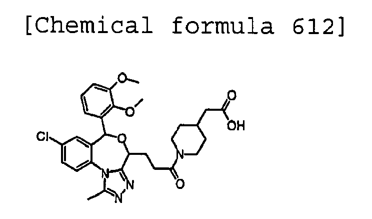

To a solution of ethyl 2-(1-{3-[8-chloro-6-(2,3-dimethoxyphenyl)-1-methyl-4H,6H-[1,2,4]triazolo[4,3-a][4,1]-benzoxazepin-4-yl]propanoyl}-4-piperidinyl)acetate (111 mg) in tetrahydrofuran (5.0 mL)/ethanol (10 mL) was added an aqueous solution (5.0 mL) of potassium carbonate (77.1 mg). The resulting mixture was stirred overnight at 50°C. To the reaction mixture was added 1N hydrochloric acid to adjust the pH to approximately 3. Water was added and the resulting mixture was extracted twice with ethyl acetate. The organic layers were combined, washed with saturated brine, and dried over anhydrous sodium sulfate. After filtration, the filtrate was concentrated under reduced pressure. The residue thus obtained was purified by thin layer chromatography (dichloromethane/methanol=9/1) to give the title compound (65.7 mg).
¹H-NMR(CDCl₃) δ:1.14-1.22 (2H,m), 1. 98-2.43 (12H,m),2.96-2.99(1H,m),3.48-3.86(8H,m),4.50-4.57(1.2H,m),5.15(0.8H,s),5.56(0.2H,s),5.96(0.8H,s),6.81-7.31(5H,m),7.42-7.49(1.0H,m). IR(ATR)cm⁻
¹:2933,1716,1633,1481,1431,1269,1223,1173,1066,1003,825,762
HRMS(FAB)m/z:
Calculated: C₂₉H₃₄N₄O₆Cl;569.2167.
Found: 569.2167.

### Referential Example 165

{5-Chloro-2-[(2,4-dimethoxybenzyl)amino]phenyl}(2,3-dimethoxyphenyl)methanol

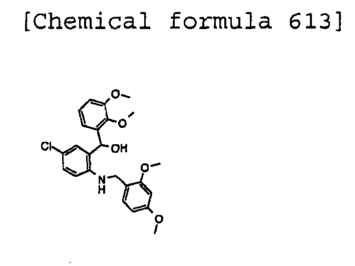

(2-Amino-5-chlorophenyl)(2,3-dimethoxyphenyl)methanol (106 g) and 2,4-dimethoxybenzaldehyde (62.7 g) were dissolved in a solvent mixture of methanol (1500 ml) and acetic acid (750 ml). To the resulting solution was added Molecular Sieves 3A powder and the resulting mixture was stirred at 60°C for 2 hours. After the reaction mixture was cooled to room temperature, sodium cyanoborohydride (27.0 g) was added and the resulting mixture was stirred at room temperature for 3 hours. The reaction mixture was concentrated. The residue was diluted with ethyl acetate and washed with a saturated aqueous solution of sodium bicarbonate and saturated brine. The organic layer was dried over anhydrous sodium sulfate. After concentration of the filtrate, the residue was purified by silica gel column (ethyl acetate:hexane=1:4) to give the title compound (97 g).
MS m/z:444(M⁺+H).
¹H-NMR(CDCl₃)δ:3.77(3H,s),3.78(3H,s),3.79(3H,s),3.88(3H,s),4.2 4(2H,s),6.02(1H,s),6.38(1H,dd,J=8.3,2.4Hz),6.44(1H,d,J=2.4H z),6.59(1H,d,J=8.8Hz),6.84(1H,dd,J=7.8,1.5Hz),6.91(1H,dd,J= 8.1,1.5Hz),6.98-6.99(1H,m),7.03-7.08(3H,m).

### Referential Example 166

Ethyl (E)-4-{4-chloro(2,4-dimethoxybenzyl)-2-[(2,3-dimethoxyphenyl)(hydroxy)methyl]anilino}-4-oxo-2-butenoate

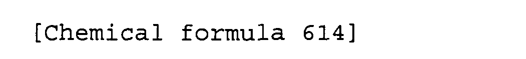

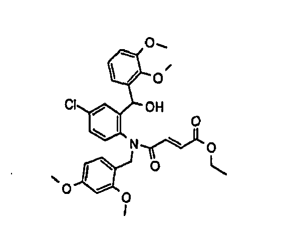

{5-Chloro-2-[(2,4-dimethoxybenzyl)amino]phenyl}(2,3-dimethoxyphenyl)methanol (97 g) was dissolved in dichloromethane (1000 mL). Sodium hydrogen carbonate (37 g) and a methylene chloride solution (500 mL) of monoethyl chlorofumarate (53 g) prepared separately were added dropwise to the resulting solution at 0°C. The mixture was stirred overnight at room temperature. The reaction mixture was diluted with dichloromethane and washed with a saturated aqueous solution of sodium bicarbonate and saturated brine. The organic layer was dried over anhydrous sodium sulfate. The filtrate was then concentrated to give the title compound (137 g).

### Referential Example 167

Ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate and (cis)-isomer

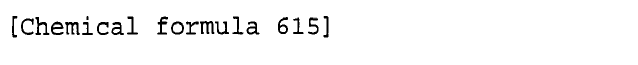

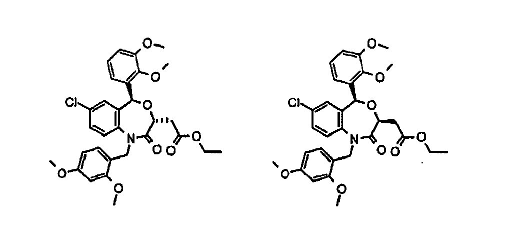

Ethyl (E)-4-{4-chloro(2,4-dimethoxybenzyl)-2-[(2,3-dimethoxyphenyl)(hydroxy)methyl]anilino}-4-oxo-2-butenoate (137 g) was dissolved in ethanol (1500 mL). To the resulting solution was added potassium carbonate (50 g) and the resulting mixture was stirred overnight at room temperature. The reaction mixture was concentrated. The residue was diluted with ethyl acetate and washed with saturated brine. The organic layer was dried over anhydrous sodium sulfate. The filtrate was then concentrated. Crystals thus formed were collected by filtration while using hexane to give the title trans isomer (75 g). The base solution was concentrated and the residue thus obtained was purified by silica gel column (ethyl acetate:hexane=1:3) to give the cis isomer (24 g). trans Isomer
MS m/z : 570 (M⁺+H).
¹H-NMR(CDCl₃) δ:1.25(3H,t,J=7.1Hz),2.79(1H,dd,J=16.4,6.1Hz),3.0 9(1H,dd,J=16.6,7.8Hz),3.20(3H,s),3.66(3H,s),3.76(3H,s),3.86 (3H,s),4.13-4.15(2H,m),4.48(1H,dd,J=7.8,6.1Hz),4.86(1H,d,J=14.9Hz),5.49 (1H,d,J=14.9Hz),5.97(1H,s),6.40-6.42(2H,m),6.54(1H,d,J=2.2Hz),6.92-6.94(1H,m),7.13-7.15(2H,m),7.23-7.25(1H,m),7.29-7.32(2H,m).
cis Isomer
MS m/z:570(M⁺+H).

### Referential Example 168

Ethyl 2-[(trans)-7-chloro-5-(2,3-dimethoxyphenyl)-2-oxo-1,2,3,5-tetrahydro-4,1-benzoxazepin-3-yl]acetate

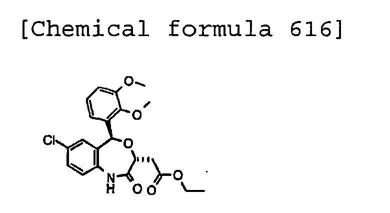

Ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate (6.00 g) was dissolved in acetone (160 mL). At 0°C, an aqueous solution (40 mL) of ammonium dicerium(IV) nitrate (17.3 g) was added and the resulting mixture was stirred at room temperature for one hour. To the reaction mixture was added a saturated aqueous solution of sodium bicarbonate, followed by extraction with ethyl acetate. The extract was washed with saturated brine. The organic layer was dried over anhydrous sodium sulfate. The filtrate was then concentrated. The residue was purified by silica gel column (ethyl acetate:hexane=1:2) to give the title compound (3.58 g).
MS m/z:420(M⁺+H).
¹H-NMR(CDCl₃)δ:1.22(3H,t,J=7.2Hz),2.80(1H,dd,J=16.5,6.7Hz),3.0 4(1H,dd,J=16.5,6.7Hz),3.65(3H,s),3.89(3H,s),4.08-4.13(2H,m),4.65(1H,t,J=6.7Hz),6.22(1H,s),6.68(1H,d,J=2.4Hz) ,6.97(1H,dd,J=8.1,1.7Hz),7.01(1H,d,J=8.5Hz),7.05-7.08(1H,m),7.15(1H,t,J=8.1Hz),7.27(1H,dd,J=8.5,2.4Hz).

### Referential Example 169

Ethyl 2-[(trans)-7-chloro-1-(2,4-dimethoxybenzyl)-5-(2,3-dimethoxyphenyl)-2-thioxo-1,2,3,5-tetrahydro-4,1-benzoxazepin-3-yl]acetate
Ethyl 2-[(cis)-7-chloro-1-(2,4-dimethoxybenzyl)-5-(2,3-dimethoxyphenyl)-2-thioxo-1,2,3,5-tetrahydro-4,1-benzoxazepin-3-yl]acetate

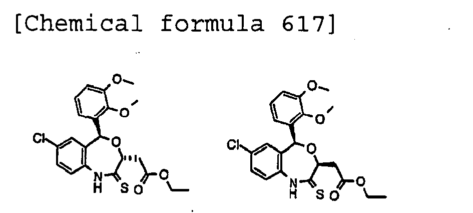

Ethyl 2-[(trans)-7-chloro-5-(2,3-dimethoxyphenyl)-2-oxo-1,2,3,5-tetrahydro-4,1-benzoxazepin-3-yl]acetate (3.00 g) was dissolved in toluene (100 mL). To the resulting solution was added Lawesson's reagent (4.91 g). The resulting mixture was heated under reflux for one hour. After cooling to room temperature, the reaction mixture was concentrated. The residue was purified twice by silica gel column (chloroform, ethyl acetate:hexane=1:3) to give the cis isomer (0.97 g) and the title trans isomer(1.27 g). trans Isomer
MS m/z:436 (M⁺+H).
¹H-NMR(CDCl₃)δ:1.24(3H,t,J=7.1Hz),2.96(1H,dd,J=16.5,6.5Hz),3.2 6(1H,dd,J=16.5,6.7Hz),3.63(3H,s),3.88(3H,s),4.10-4.16(3H,m),4.71(1H,t,J=6.6Hz),6.13(1H,s),6.66(1H,d,J=2.2Hz) ,6.97(1H,dd,J=8.1,2.0Hz),7.06(1H,d,J=8.1Hz),7.10-7.13(1H,m),7.16(1H,t,J=8.1Hz),7.33(1H,dd,J=8.1,2.2Hz),9.70( 1H,s).
cis Isomer
MS m/z:436(M⁺+H).
¹H-NMR(CDCl₃)δ:1.22(3H, t,J=7.2Hz),3.00(1H,dd,J=16.8,9.0Hz),3.2 9(1H,dd,J=16.8,3.9Hz),3.81(3H,s),3.87(3H,s),4.09-4.18(2H,m),5.06(1H,dd,J=9.0,3.9Hz),6.34(1H,s),6.83-6.93(4H,m),7.04(1H,t,J=8.1Hz),7.17-7.19(1H,m),9.47(1H,s).

### Referential Example 170

Ethyl 2-[(trans)-7-chloro-1-(2,4-dimethoxybenzyl)-5-(2,3-dimethoxyphenyl)-2-thioxo-1,2,3,5-tetrahydro-4,1-benzoxazepin-3-yl]acetate
Ethyl 2-[(cis)-7-chloro-1-(2,4-dimethoxybenzyl)-5-(2,3-dimethoxyphenyl)-2-thioxo-1,2,3,5-tetrahydro-4,1-benzoxazepin-3-yl]acetate

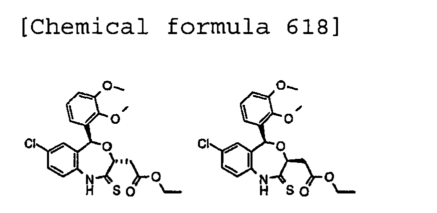

Ethyl 2-[(cis)-7-chloro-1-(2,4-dimethoxybenzyl)-5-(2,3-dimethoxyphenyl)-2-thioxo-1,2,3,5-tetrahydro-4,1-benzoxazepin-3-yl]acetate (970 mg) was dissolved in ethanol (10 mL). To the resulting solution was added potassium carbonate (308 mg) and the resulting mixture was stirred overnight at room temperature. After concentration of the reaction mixture, the residue was diluted with chloroform and washed with saturated brine. The organic layer was dried over anhydrous sodium sulfate. The filtrate was then concentrated. The residue thus obtained was separated and purified by silica gel column (ethyl acetate:hexane=1:3) to give the cis isomer (340 mg) and the title trans isomer (406 mg).

### Example 276

Ethyl 2-[(trans)-8-chloro-6-(2,3-dimethoxyphenyl)-1-methyl-4H,6H-[1,2,4]triazolo[4,3-a][4,1]-benzoxazepin-4-yl]acetate

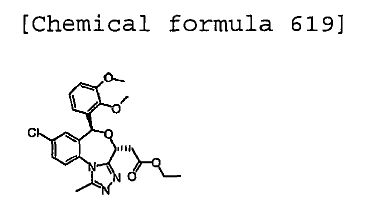

To a solution of ethyl 2-[(trans)-7-chloro-5-(2,3-dimethoxyphenyl)-2-thioxo-1,2,3,5-tetrahydro-4,1-benzoxazepin-3-yl]acetate (73.2 mg) in isopropyl alcohol (10 mL) was added acetylhydrazine (37.3 mg). The resulting mixture was heated under reflux. The same amount of acetylhydrazine was added further three times in total. After confirmation of the disappearance of the raw material, the resulting mixture was concentrated under reduced pressure. After the residue was dissolved in acetic acid (10 mL), the resulting solution was heated under reflux for 75 minutes, followed by concentration under reduced pressure. Chloroform was dissolved in the residue. The resulting solution was washed successively with a saturated aqueous solution of sodium hydrogen carbonate and saturated brine, and dried over anhydrous sodium sulfate. After filtration, the filtrate was concentrated under reduced pressure. The crude product was purified by thin layer chromatography (dichloromethane/methanol=9/1) to give the title compound (75.4 mg).
¹H-NMR(CDCl₃)δ:1.28(3H,t,J=7.1Hz),2.66(3H,s),3.21(1H,dd,J=16.4 ,7.6Hz),3.41-3.46(1H,m),3.49(3H,d,J=2.7Hz),3.86(3H,s),4.18(2H,dt d,J=16.8,6.1,3.0Hz),4.94(1H,dd,J=7.7,6.0Hz),5.60(1H,s),6.82 (1H,d,J=2.4Hz),6.97(1H,dd,J=8.1,1.7Hz),7.20(2H,tt,J=11.0,4. 0Hz),7.32(1H,d,J=8.5Hz),7.49(1H,dd,J=8.5,2.4Hz).

### Referential Example 171

2-[(trans)-8-Chloro-6-(2,3-dimethoxyphenyl)-1-methyl-4H,6H-[1,2,4]triazolo[4,3-a][4,1]-benzoxazepin-4-yl]-1-ethanol

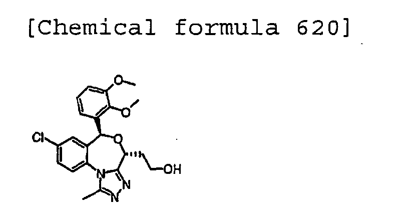

To a solution of lithium aluminum hydride (11.8 mg) in tetrahydrofuran (1.0 mL) was added a solution of ethyl 2-[(trans)-8-chloro-6-(2,3-dimethoxyphenyl)-1-methyl-4H,6H-[1,2,4]triazolo[4,3-a] [4,1]-benzoxazepin-4-yl]acetate (94.6 mg) in tetrahydrofuran (5.0 mL) under ice cooling. The resulting mixture was stirred at the same temperature for one hour. To the reaction mixture was added a 10% aqueous solution of potassium sodium tartrate to terminate the reaction. The reaction mixture was extracted three times with chloroform. The organic layers were combined and dried over anhydrous sodium sulfate. After filtration, the filtrate was concentrated under reduced pressure. The residue was purified by thin layer chromatography (dichloromethane/methanol=9/1) to give the title compound (62 mg).
¹H-NMR(CDCl₃)δ:2.50-2.56(2H,m),2.66(3H,s),3.17(1H,t,J=5.5Hz),3.50(3H,s),3.85-4.02(5H,m),4.64(1H,t,J=6.1Hz),5.61(1H,s),6.84(1H,d,J=2.2Hz) ,6.98(1H,dd,J=7.9,1.6Hz),7.18-7.24(2H,m),7.32(1H,d,J=8.5Hz),7.49(1H,dd,J=8.4,2.3Hz).

### Referential Example 172

2-[(trans)-8-Chloro-6-(2,3-dimethoxyphenyl)-1-methyl-4H,6H-[1,2,4]triazolo[4,3-a][4,1]-benzoxazepin-4-yl]propanonitrile

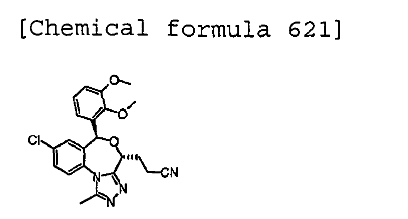

To a solution of 2-[(trans)-8-chloro-6-(2,3-dimethoxyphenyl)-1-methyl-4H,6H-[1,2,4]triazolo[4,3-a][4,1]-benzoxazepin-4-yl]-1-ethanol (62.0 mg) and triethylamine (104 µL) in dichloromethane (5.0 mL) was added a solution of mesyl chloride (35 µL) in dichloromethane (3.0 mL). The resulting mixture was stirred at room temperature for 40 minutes. The reaction mixture was washed with water and dried over anhydrous sodium sulfate. After filtration, the filtrate was concentrated under reduced pressure. The residue thus obtained was dissolved in dimethylsulfoxide (10 mL). To the resulting solution was added sodium cyanide (14.6 mg) and the resulting mixture was stirred overnight at 50°C. After addition of water and a saturated aqueous solution of sodium hydrogen carbonate, the resulting mixture was extracted three times with ethyl acetate. The organic layers were combined, washed three times with water and once with saturated brine, and then dried over anhydrous sodium sulfate. After filtration, the filtrate was concentrated under reduced pressure. The residue was purified by thin layer chromatography (dichloromethane/methanol=9/1) to give the title compound (68.3 mg).
¹H-NMR(CDCl₃)δ:2.70(7H,dd t,J=57.5,28.2,9.9Hz),3.49(3H,d,J=0.7Hz),3.87(3H,s),4.58(1H, dd,J=7.2,5.5Hz),5.60(1H,s),6.84(1H,d,J=2.2Hz),7.00(1H,dd,J= 7.6,2.0Hz),7.20-7.25(2H,m),7.33(1H,d,J=8.5Hz),7.51(1H,dd,J=8.5,2.4Hz).

### Example 277

3-[(trans)-8-Chloro-6-(2,3-dimethoxyphenyl)-1-methyl-4H,6H-[1,2,4]triazolo[4,3-a] [4,1]-benzoxazepin-4-yl]propanoic acid

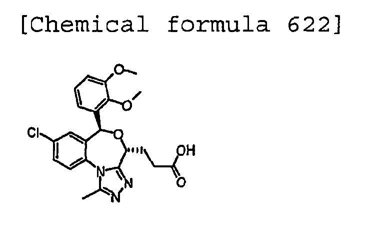

To a solution of 2-[(trans)-8-chloro-6-(2,3-dimethoxyphenyl)-1-methyl-4H,6H-[1,2,4]triazolo[4,3-a][4,1]-benzoxazepin-4-yl]propanonitrile (68.3 mg) in isopropyl alcohol(5.0 mL) was added a 5N aqueous sodium hydroxide solution (5.0 mL), followed by further addition of methanol (4.0 mL). The reaction mixture was heated under reflux for one hour. After the reaction mixture was made acidic with a 6N aqueous hydrochloric acid solution, water was added for dilution. The diluted mixture was extracted four times with chloroform. The organic layers were combined and dried over anhydrous sodium sulfate. After filtration, the filtrate was concentrated under reduced pressure to give the title compound (66.7 mg).
MSm/z :444 (M⁺+H).

### Example 278

Ethyl 2-(1-{3-[(trans)-8-chloro-6-(2,3-dimethoxyphenyl)-1-methyl-4H,6H-[1,2,4]triazolo[4,3-a][4,1]-benzoxazepin-4-yl]propanoyl}-4-piperidinyl)acetate

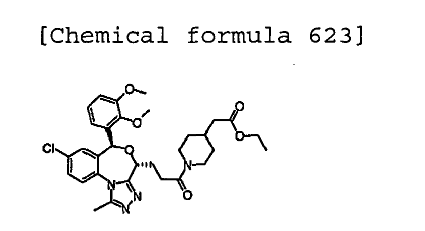

To a solution of 3-[(trans)-8-chloro-6-(2,3-dimethoxyphenyl)-1-methyl-4H,6H-[1,2,4]triazolo[4,3-a][4,1]-benzoxazepin-4-yl]propanoic acid (66.7 mg), ethyl 2-(4-piperidinyl)-acetate (38.5 mg), 1-hydroxybenzotriazole (30.4 mg), and N-methylmorpholine (24.7 µL) in N,N-dimethylformamide (10 mL) was added 1-ethyl-3-(3-dimethylaminopropyl)carbodiimide hydrochloride (43.1 mg). The resulting mixture was stirred overnight at room temperature. After concentration of the reaction mixture under reduced pressure, ethyl acetate was added to the residue. The resulting mixture was washed three times with water and once with saturated brine, and dried over anhydrous sodium sulfate. After filtration, the filtrate was concentrated under reduced pressure. The residue was purified by thin layer chromatography (dichloromethane/methanol=9/1) to give the title compound (60 mg).
¹H-NMR(CDCl₃)δ:1.05-1.27(6H,m),1.73(2H,t,J=14.4Hz),1.99(1H,s),2.22(2H,dd,J=7.0, 3.1Hz),2.61(7H,dtd,J=60.3,24.4,9.2Hz),3.00(1H,t,J=13.4Hz),3 .48(3H,s),3.88(4H,dd,J=29.9,23.8Hz),4.13(2H,q,J=7.2Hz),4.56 (2H,q,J=6.2Hz),5.56(1H,s),6.80(1H,d,J=2.4Hz),6.97(1H,d,J=8. 1Hz),7.20-7.28(3H,m),7.46(1H,dd,J=8.5,2.4Hz).

### Example 279

2-(1-{3-[(trans)-8-Chloro-6-(2,3-dimethoxyphenyl)-1-methyl-4H,6H-[1,2,4]triazolo[4,3-a][4,1]-benzoxazepin-4-yl]propanoyl}-4-piperidinyl)acetic acid

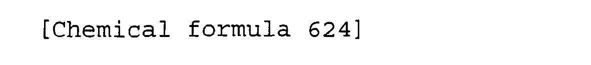

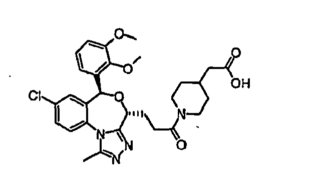

To a solution of ethyl 2-(1-{3-[(trans)-8-chloro-6-(2,3-dimethoxyphenyl)-1-methyl-4H,6H-[1,2,4]triazolo[4,3-a][4,1]-benzoxazepin-4-yl]propanoyl}-4-piperidinyl)acetate (60.0 mg) in tetrahydrofuran (2.5 mL) and ethanol (5.0 mL) was added an aqueous solution (2.5 mL) of potassium carbonate (41.5 mg). The resulting mixture was stirred overnight at 50°C. To the reaction mixture was added 1N hydrochloric acid to adjust it to approximately pH3, followed by dilution with water. The diluted mixture was washed three times with chloroform. The organic layers were combined and dried over anhydrous sodium sulfate. After filtration, the filtrate was concentrated under reduced pressure. The residue was purified by thin layer chromatography (dichloromethane/methanol=9/1) to give the title compound (49.2 mg).
¹H-NMR(CDCl₃)δ:1.10-1.26(2H,m),1.87(4H,dd,J=63.1,46.2Hz),2.27-2.36(2H,m),2.49-2.76(7H,m),3.01(1H,t,J=14.0Hz),3.48(3H,s),3.86(3H,d,J=6.1Hz ),3.96(1H,d,J=12.5Hz),4.56(2H,t,J=5.9Hz),5.56(1H,s),6.81(1H ,s),6.98(1H,d,J=8.3Hz),7.20-7.29(3H,m),7.47(1H,dd,J=8.5,2.3Hz).
IR (ATR) cm⁻
¹:2933,1716,1635,1589,1481,1431,1279,1223,1171,1068,1003,75 0.
HRMS(FAB)m/z:
Calculated: C₂₉H₃₄O₆N₄Cl;569.2167.
Found: 569.2159.

### Referential Example 173

Ethyl 2-[(trans)-2-[2-acetylhydrazono]-7-chloro-5-(2,3-dimethoxyphenyl)-1,5-dihydro-4,1-benzoxazepin3(3H)-yl]acetate

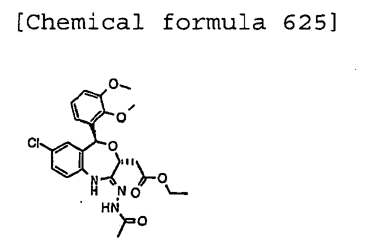

Ethyl 2-[(trans)-7-chloro-5-(2,3-dimethoxyphenyl)-2-thioxo-1,2,3,5-tetrahydro-4,1-benzoxazepin-3-yl]acetate (820 mg) was dissolved in 2-propanol (20 mL). To the resulting solution was added acetylhydrazine (1.25 g). The resulting mixture was heated under reflux for 2 days. The reaction mixture was concentrated to give the title compound (895 mg).
MS(ESI)m/z:476(M⁺+H).

### Example 280

Ethyl 2-[(trans)-8-chloro-6-(2,3-dimethoxyphenyl)-1-methyl-4H,6H-[1,2,4]triazolo[4,3-a][4,1]benzoxazepin-4-yl]acetate

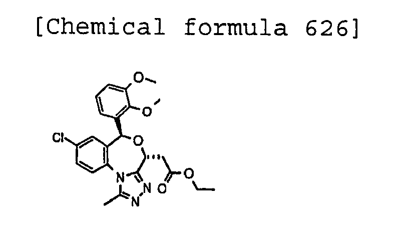

Ethyl 2-[(trans)-2-[2-acetylhydrazono]-7-chloro-5-(2,3-dimethoxyphenyl)-1,5-dihydro-4,1-benzoxazepin3(3H)-yl]acetate (895 mg) was dissolved in acetic acid (20 mL). The resulting solution was heated under reflux for 3 hours. The reaction mixture was cooled to room temperature and then concentrated. The residue was diluted with chloroform and washed with a saturated aqueous solution of sodium bicarbonate and saturated brine. The organic layer was dried over anhydrous sodium sulfate. The filtrate was then concentrated. The residue thus obtained was purified by silica gel column (methanol:chloroform=5:95) to give the title compound (693 mg).
MSm/z:458(M⁺+H).
¹H-NMR(CDCl₃)δ:1.28(3H,t,J=7.1Hz),3.21(1H,dd,J=16.4,7.6Hz),3.4 4(1H,dd,J=16.6,6.1Hz),3.49(3H,s),3.86(3H,s),4.14-4.23(2H,m),4.94(1H,dd,J=7.6,6.1Hz),5.60(1H,s),6.82(1H,d,J=2 .2Hz),6.97(1H,dd,J=7.9,1.7Hz),7.18(1H,t,J=7.9Hz),7.23(1H,dd ,J=7.9,1.7Hz),7.33(1H,d,J=8.5Hz),7.49(1H,dd,J=8.5,2.2Hz).

### Referential Example 174

2-[(trans)-8-Chloro-6-(2,3-dimethoxyphenyl)-1-methyl-4H,6H-[1,2,4]triazolo[4,3-a][4,1]benzoxazepin-4-yl]-1-ethanol

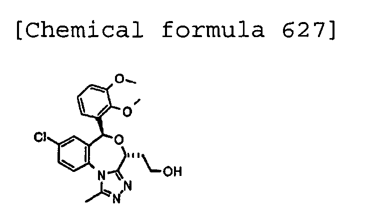

Lithium aluminum hydride (302 mg) was suspended in tetrahydrofuran (10 mL). Under stirring at -15°C, a solution of ethyl 2-[(trans)-8-chloro-6-(2,3-dimethoxyphenyl)-1-methyl-4H,6H-[1,2,4]triazolo[4,3-a][4,1]benzoxazepin-4-yl]acetate (668 mg) in tetrahydrofuran (10 mL) was added dropwise slowly to the resulting suspension, followed by stirring for 30 minutes as was. To the reaction mixture was added an aqueous solution of potassium sodium tartrate. The resulting mixture was concentrated and the residue was extracted with chloroform. The organic layer was dried over anhydrous sodium sulfate. The filtrate was concentrated to give the title compound (600 mg).
MS m/z: 416 (M⁺+H).
¹H-NMR(CDCl₃)δ:2.48-2.57(2H,m),2.66(3H,s),3.17-3.19(1H,m),3.50(3H,s),3.87(3H,s),3.89-3.93(1H,m),3.97-4.03(1H,m),4.64(1H,t,J=6.0Hz),5.61(1H,s),6.84(1H,d,J=2.2Hz) ,6.98(1H,dd,J=8.1,1.7Hz),7.20(1H,t,J=7.9Hz),7.24-7.26(1H,m),7.32(1H,d,J=8.5Hz),7.49(1H,dd,J=8.5,2.4Hz).

### Referential Example 175

2-[(trans)-8-Chloro-6-(2,3-dimethoxyphenyl)-1-methyl-4H,6H-[1,2,4]triazolo[4,3-a][4,1]benzoxazepin-4-yl]ethyl methanesulfonate

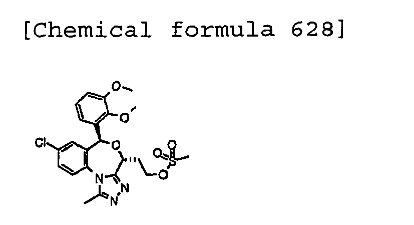

2-[(trans)-8-Chloro-6-(2,3-dimethoxyphenyl)-1-methyl-4H,6H-[1,2,4]triazolo[4,3-a][4,1]benzoxazepin-4-yl]-1-ethanol (57 mg) was dissolved in dichloromethane (2 mL). To the resulting solution were added triethylamine (0.028 mL) and methanesulfonyl chloride (0.016 mL) at 0°C, followed by stirring for 0.5 hour. To the reaction mixture was added a saturated aqueous solution of sodium bicarbonate, followed by extraction with chloroform. The organic layer was dried over anhydrous sodium sulfate to give the title compound (89 mg).

### Example 281

Ethyl 1-{2-[(trans)-8-chloro-6-(2,3-dimethoxyphenyl)-1-methyl-4H, 6H-[1,2,4]triazolo[4,3-a][4,1]benzoxazepin-4-yl]ethyl-1H-pyrazole-4-carboxylate

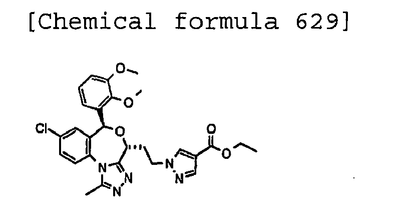

Ethyl 4-pyrazolecarboxylate (52 mg) was dissolved in N,N-dimethylformamide (1 mL). At 0°C, sodium hydride (55%, 16 mg) was added and the resulting mixture was stirred for 0.5 hour at 0°C. A solution of 2-[(trans)-8-chloro-6-(2,3-dimethoxyphenyl)-1-methyl-4H,6H-[1,2,4]triazolo[4,3-a][4,1]benzoxazepin-4-yl]ethyl methanesulfonate (121 mg) in N,N-dimethylformamide (2 mL) was added dropwise to the reaction mixture. Tetrabutylammonium iodide (91 mg) was added further. The resulting mixture was stirred overnight at 50°C. Water was added to the reaction mixture and the resulting mixture was extracted with chloroform. The organic layer was dried over anhydrous magnesium sulfate. The filtrate was then concentrated. The residue thus obtained was purified by preparative TLC (methanol:chloroform=1:9) to give the title compound (78 mg).
MS(ESI)m/z:538(M⁺+H) ¹H-NMR(CDCl₃)δ:1.32(3H,t,J=7.1Hz),2.66(3H,s),2.79-2.87(2H,m),3.49(3H,s),3.87(3H,s),4.26(2H,q,J=7.1Hz),4.38-4.42(1H,m),4.47-4.59(2H,m),5.59(1H,s),6.82(1H,d,J=2.2Hz),6.99(1H,dd,J=7.6,2 .2Hz),7.19-7.23(2H,m),7.30(1H,d,J=8.5Hz),7.48(1H,dd,J=8.5,2.4Hz),7.84( 1H,d,J=0.5Hz),7.91(1H,d,J=0.5Hz).

### Example 282

1-{2-[(trans)-8-Chloro-6-(2,3-dimethoxyphenyl)-1-methyl-4H,6H-[1,2,4]triazolo[4,3-a][4,1]benzoxazepin-4-yl]ethyl-1H-pyrazole-4-carboxylic acid

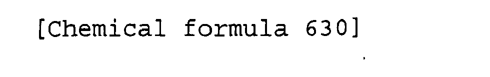

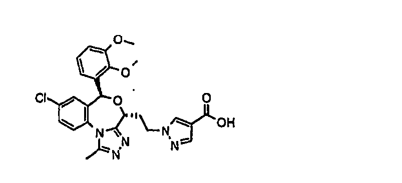

Ethyl 1-{2-[(trans)-8-chloro-6-(2,3-dimethoxyphenyl)-1-methyl-4H,6H-[1,2,4]triazolo[4,3-a][4,1]benzoxazepin-4-yl]ethyl-1H-pyrazole-4-carboxylate (78 mg) was dissolved in methanol-water-tetrahydrofuran (2:1:2, 5 mL). Potassium carbonate (60 mg) was added to the resulting solution, followed by stirring at room temperature for 5 days and then at 55°C overnight. After the reaction mixture was neutralized with an acidic resin (Amberlite IR-120B), the resin was filtered out. The residue was washed with methanol. The filtrate was then concentrated. The residue thus obtained was purified by preparative TLC (methanol:chloroform=1:9) to give the title compound (41 mg).
MS (ESI) m/z : 510 (M⁺+H).
¹H-NMR(CDCl₃)δ:2.62(3H,s),2.71-2.83(2H,m),3.46(3H,s),3.85(3H,s),4.36-4.45(3H,m),5.56(1H,s),6.79(1H,d,J=2.2Hz),6.96(1H,dd,J=8.1,1 .2Hz),7.18(1H,t,J=7.9Hz),7.23(1H,d,J=7.3Hz),7.30(1H,d,J=8.1 Hz),7.44(1H,dd,J=8.1,2.2Hz),7.77(1H,s),7.94(1H,s).

### Referential Example 176

Ethyl 2-[(trans)-7-chloro-5-(2,3-dimethoxyphenyl)-2-[hydrazono]-1,5-dihydro-4,1-benzoxazepin3(3H)-yl]acetate

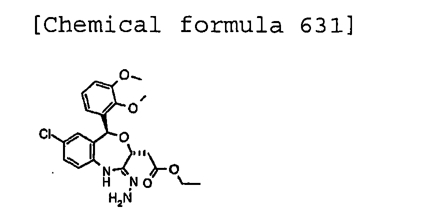

Ethyl 2-[(trans)-7-chloro-5-(2,3-dimethoxyphenyl)-2-thioxo-1,2,3,5-tetrahydro-4,1-benzoxazepin-3-yl]acetate (129 mg) was dissolved in ethanol (3 mL). Hydrazine monohydrate (0.028 mL) was added and the resulting mixture was stirred at room temperature for one hour. The reaction mixture was concentrated to give the title compound (128 mg).
MS(ESI)m/z:434(M⁺+H).

### Referential Example 177

Ethyl 2-[(trans)-7-chloro-2-[2-(cyclopropylcarbonyl)hydrazono]-5-(2,3-dimethoxyphenyl)-1,5-dihydro-4,1-benzoxazepin3(3H)-yl]acetate

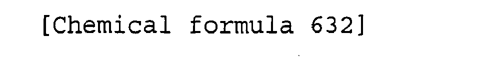

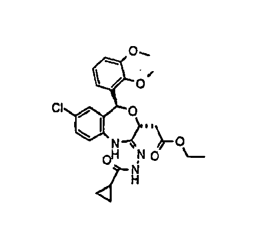

Ethyl 2-[(trans)-7-chloro-5-(2,3-dimethoxyphenyl)-2-[hydrazono]-1,5-dihydro-4,1-benzoxazepin3(3H)-yl]acetate (128 mg) was dissolved in tetrahydrofuran (3 mL). To the resulting solution was added cyclopropane carbonyl chloride (0.032 mL). The resulting mixture was stirred at room temperature for 1.5 hours. To the reaction mixture was added a saturated aqueous solution of sodium bicarbonate, followed by extraction with chloroform. The organic layer was dried over anhydrous sodium sulfate to give the title compound (148 mg).
MS(ESI)m/z:502(M⁺+H).

### Example 283

Ethyl 2-[(trans)-8-chloro-1-cyclopropyl-6-(2,3-dimethoxyphenyl)-4H,6H-[1,2,4]triazolo[4,3-a] [4,1] benzoxazepin-4-yl] acetate

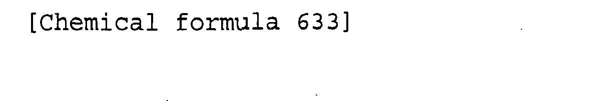

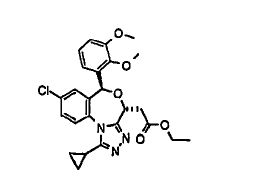

Ethyl 2-[(trans)-7-chloro-2-[2-(cyclopropylcarbonyl)hydrazono]-5-(2,3-dimethoxyphenyl)-1,5-dihydro-4,1-benzoxazepin3(3H)-yl]acetate (148 mg) was dissolved in acetic acid (4 mL). The resulting solution was heated under reflux for 3 hours. After cooling to room temperature, the reaction mixture was concentrated. The residue was diluted with chloroform and then washed with a saturated aqueous solution of sodium bicarbonate and saturated brine. The organic layer was dried over anhydrous sodium sulfate. The filtrate was then concentrated. The residue thus obtained was purified by preparative TLC (methanol:chloroform=5:95) to give the title compound (60 mg).
MS m/z:484 (M⁺+H).
¹H-NMR(CDCl₃)δ:1.03-1.10(1H,m),1.17-1.24(2H,m),1.27(3H,t,J=7.1Hz),1.38-1.44(1H,m),1.93-2.00(1H,m),3.20(1H,dd,J=16.5,7.7Hz),3.42(1H,dd,J=16.5,5.7Hz ),3.50(3H,s),3.86(3H,s),4.14-4.22(2H,m),4.95(1H,dd,J=7.7,5.7Hz),5.64(1H,s),6.82(1H,d,J=2 .4Hz),6.97(1H,dd,J=7.9,1.7Hz),7.18(1H,t,J=7.9Hz),7.22-7.25(1H,m),7.48(1H,dd,J=8.4,2.3Hz),7.58(1H,d,J=8.4Hz).

### Referential Example 178

2-[(trans)-8-Chloro-1-cyclopropyl-6-(2,3-dimethoxyphenyl)-4H,6H-[1,2,4]triazolo[4,3-a][4,1]benzoxazepin-4-yl]-1-ethanol

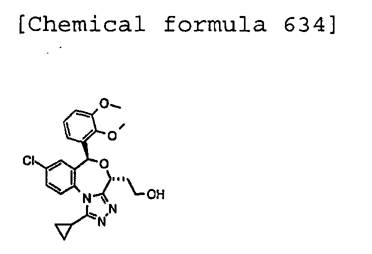

Lithium aluminum hydride (26 mg) was suspended in tetrahydrofuran (1 mL). Under stirring at -15°C, a solution of ethyl 2-[(trans)-8-chloro-1-cyclopropyl-6-(2,3-dimethoxyphenyl)-4H,6H-[1,2,4]triazolo[4,3-a][4,1]benzoxazepin-4-yl]acetate (60 mg) in tetrahydrofuran (2 mL) was slowly added dropwise, followed by stirring for 30 minutes as was. An aqueous solution of potassium sodium tartrate was added and the resulting mixture was concentrated. The residue was extracted with chloroform. The organic layer was dried over anhydrous sodium sulfate. The filtrate was concentrated to give the title compound (53 mg).
MSm/z:442(M⁺+H).
¹H-NMR(CDCl₃)δ:1.06-1.11(1H,m),1.19-1.25(2H,m),1.37-1.42(1H,m),1.93-2.00(1H,m),2.47-2.55(2H,m),3.43-3.47(1H,m),3.51(3H,s),3.87-3.89(4H,m),3.97-4.02(1H,m),4.64(1H,t,J=6.0Hz),5.64(1H,s),6.84(1H,d,J=2.2Hz) ,6.98(1H,dd,J=7.9,1.6Hz),7.20(1H,t,J=7.9Hz),7.25(1H,d,J=1.6 Hz),7.49(1H,dd,J=8.4,2.3Hz),7.57(1H,d,J=8.4Hz).

### Referential Example 179

2-[(trans)-8-Chloro-1-cyclopropyl-6-(2,3-dimethoxyphenyl)-4H,6H-[1,2,4]triazolo[4,3-a][4,1]benzoxazepin-4-yl]ethyl methanesulfonate

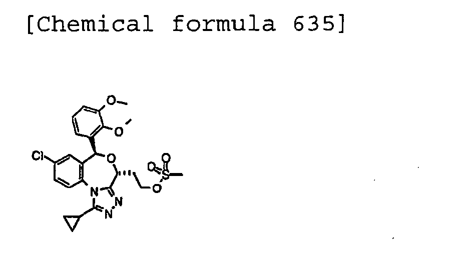

2-[(trans)-8-Chloro-1-cyclopropyl-6-(2,3-dimethoxyphenyl)-4H,6H-[1,2,4]triazolo[4,3-a][4,1]benzoxazepin-4-yl]-1-ethanol (53 mg) was dissolved in dichloromethane (2 mL). To the resulting solution were added triethylamine (0.025 mL) and methanesulfonyl chloride (0.014 mL) at 0°C, followed by stirring for 0.5 hour. A saturated aqueous solution of sodium bicarbonate was added and the resulting mixture was extracted with chloroform.
The organic layer was dried over anhydrous sodium sulfate to give the title compound (74 mg).
MSm/z:520(M⁺+H).

### Example 284

Ethyl 1-{2-[(trans)-8-chloro-1-cyclopropyl-6-(2,3-dimethoxyphenyl)-4H,6H-[1,2,4]triazolo[4,3-a][4,1]benzoxazepin-4-yl]ethyl-1H-pyrazole-4-carboxylate

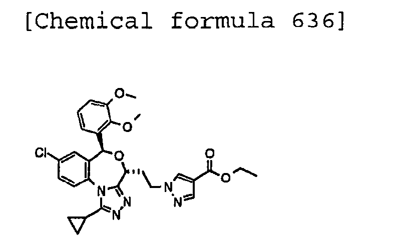

Ethyl 4-pyrazolecarboxylate (25 mg) was dissolved in N,N-dimethylformamide (1 mL). At 0°C, sodium hydride (55%, 7.8 mg) was added and the resulting mixture was stirred at 0°C for one hour. A solution of 2-[(trans)-8-chloro-1-cyclopropyl-6-(2,3-dimethoxyphenyl)-4H,6H-[1,2,4]triazolo[4,3-a] [4,1]benzoxazepin-4-yl]ethyl methanesulfonate (62 mg) in N,N-dimethylformamide (2 mL) was added dropwise to the reaction mixture. Tetrabutylammonium iodide (44 mg) was added further and the resulting mixture was stirred overnight at 80°C. After addition of water, the resulting mixture was extracted with chloroform. The organic layer was dried over anhydrous magnesium sulfate. The filtrate was then concentrated.
The residue thus obtained was purified by preparative TLC (methanol:chloroform=1:9) to give the title compound (45 mg).
MS(ESI)m/z:564(M⁺+H).
¹H-NMR(CDCl₃)δ:1.03-1.11(1H,m),1.18-1.25(2H,m),1.32(3H,t,J=7.2Hz),1.37-1.44(1H,m),1.92-1.99(1H,m),2.75-2.87(2H,m),3.49(3H,s),3.87(3H,s),4.26(2H,q,J=7.2Hz),4.41(1H ,dd,J=7.3,5.4Hz),4.47-4.59(2H,m),5.62(1H,s),6.82(1H,d,J=2.2Hz),6.99(1H,dd,J=7.8,2 .0Hz),7.19-7.24(2H,m),7.47(1H,dd,J=8.4,2.2Hz),7.56(1H,d,J=8.4Hz),7.84( 1H,s),7.90(1H,s).

### Example 285

1-{2-[(trans)-8-Chloro-1-cyclopropyl-6-(2,3-dimethoxyphenyl)-4H,6H-[1,2,4]triazolo[4,3-a][4,1]benzoxazepin-4-yl]ethyl-1H-pyrazole-4-carboxylic acid

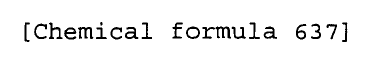

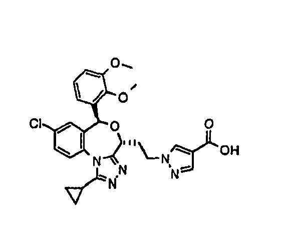

Ethyl 1-{2-[(trans)-8-chloro-1-cyclopropyl-6-(2,3-dimethoxyphenyl)-4H,6H-[1,2,4]triazolo[4,3-a][4,1]benzoxazepin-4-yl]ethyl-1H-pyrazole-4-carboxylate (45 mg) was dissolved in methanol-water-tetrahydrofuran (1:0.5:1, 2.5 mL). To the resulting solution was added lithium hydroxide monohydrate (6.6 mg). The resulting mixture was stirred overnight at 50°C. After the reaction mixture was neutralized with an acidic resin (Amberlite IR-120B), the resin was filtered out and the residue was washed with methanol. The filtrate was then concentrated and the residue thus obtained was purified by preparative TLC (methanol:chloroform=1:9) to give the title compound (41 mg).
MS(ESI)m/z:536(M⁺+H).
¹H-NMR(CDCl₃)δ:1.02-1.08(1H,m),1,15-1.26(2H,m),1.34-1.40(1H,m),1.93-1.97(1H,m),2.76-2.83(2H,m),3.48(3H,s),3.86(3H,s),4.40-4.48(3H,m),5.60(1H,s),6.81(1H,d,J=2.2Hz),6.97(1H,dd,J=8.1,1 .5Hz),7.17-7.25(2H,m),7.45(1H,dd,J=8.4,2.2Hz),7.55(1H,d,J=8.4Hz),7.81( 1H,s),7.95(1H,s).

### Referential Example 180

Ethyl 2-[(trans)-7-chloro-2-[2-isobutyrylhydrazono]-5-(2,3-dimethoxyphenyl)-1,5-dihydro-4,1-benzoxazepin3(3H)-yl] acetate

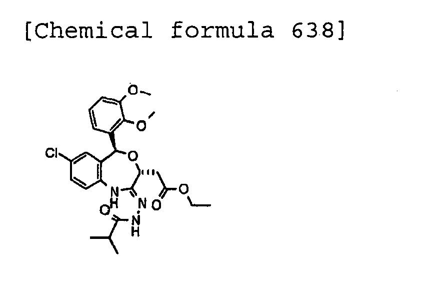

Ethyl 2-[(trans)-7-chloro-5-(2,3-dimethoxyphenyl)-2-[hydrazono]-1,5-dihydro-4,1-benzoxazepin3(3H)-yl]acetate (135 mg) was dissolved in tetrahydrofuran (3 mL). To the resulting solution was added 2-methylpropanoyl chloride (0.039 mL). The resulting mixture was stirred at room temperature for 1.5 hours. A saturated aqueous solution of sodium bicarbonate was added and the resulting mixture was extracted with chloroform. The organic layer was dried over anhydrous sodium sulfate to give the title compound (157 mg).

### Example 286

Ethyl 2-[(trans)-8-chloro-6-(2,3-dimethoxyphenyl)-1-isopropyl-4H,6H-[1,2,4]triazolo[4,3-a][4,1]benzoxazepin-4-yl] acetate

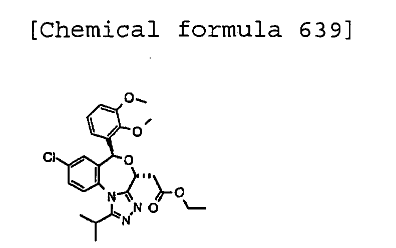

Ethyl 2-[(trans)-7-chloro-2-[2-isobutyrylhydrazono]-5-(2,3-dimethoxyphenyl)-1,5-dihydro-4,1-benzoxazepin3(3H)-yl]acetate (156 mg) was dissolved in acetic acid (5 mL). The resulting solution was heated under reflux overnight. After cooling to room temperature, the reaction mixture was concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (78 mg).
MS m/z:486(M⁺+H).
¹H-NMR(CDCl₃)δ:1.14(3H,d,J=6.8Hz),1.28(3H,t,J=7.2Hz),1.64(3H,d ,J=6.8Hz),3.21(1H,dd,J=16.5,7.7Hz),3.33-3.44(2H,m),3.46(3H,s),3.86(3H,s),4.16-4.21(2H,m),4.90(1H,dd,J=7.6,5.9Hz),5.52(1H,s),6.79(1H,d,J=2 .4Hz),6.96(1H,dd,J=7.9,1.7Hz),7.18(1H,t,J=7.9Hz),7.23(1H,dd ,J=7.9,1.7Hz),7.38(1H,d,J=8.5Hz),7.47(1H,dd,J=8.5,2.4Hz).

### Referential Example 181

2-[(trans)-8-Chloro-6-(2,3-dimethoxyphenyl)-1-isopropyl-4H,6H-[1,2,4]triazolo[4,3-a][4,1]benzoxazepin-4-yl]-1-ethanol

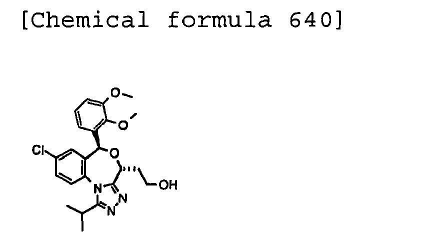

Lithium aluminum hydride (33 mg) was suspended in tetrahydrofuran (1 mL). Under stirring at -15°C, a solution of ethyl 2-[(trans)-8-chloro-6-(2,3-dimethoxyphenyl)-1-isopropyl-4H,6H-[1,2,4]triazolo[4,3-a][4,1]benzoxazepin-4-yl]acetate (78 mg) in tetrahydrofuran (2 mL) was slowly added dropwise. The reaction mixture was stirred for 30 minutes as was. To the reaction mixture was added an aqueous solution of potassium sodium tartrate, followed by concentration. The residue was extracted with chloroform. The organic layer was dried over anhydrous sodium sulfate. The filtrate was concentrated to give the title compound (66 mg).
MS m/z:444(M⁺+H).
¹H-NMR(CDCl₃)δ:1.15(3H,d,J=6.8Hz),1.64(3H,d,J=6.8Hz),2.44-2.59(2H,m),3.34-3.41(1H,m),3.48(3H,s),3.61-3.62(1H,m),3.85-3.88(4H,m),3.98-4.04(1H,m),4.57-4.60(1H,m),5.53(1H,s),6.81(1H,d,J=2.2Hz),6.97-6.99(1H,m),7.20(1H,t,J=8.1Hz),7.24-7.25(1H,m),7.38(1H,d,J=8.5Hz),7.48(1H,dd,J=8.5,2.2Hz).

### Referential Example 182

2-[(trans)-8-Chloro-6-(2,3-dimethoxyphenyl)-1-isopropyl-4H,6H-[1,2,4]triazolo[4,3-a][4,1]benzoxazepin-4-yl]ethyl methanesulfonate

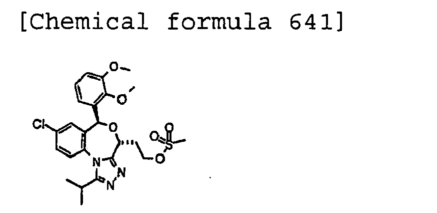

2-[(trans)-8-Chloro-6-(2,3-dimethoxyphenyl)-1-isopropyl-4H,6H-[1,2,4]triazolo[4,3-a][4,1]benzoxazepin-4-yl]-1-ethanol (78 mg) was dissolved in dichloromethane (2 mL). To the resulting solution were added triethylamine (0.037 mL) and methanesulfonyl chloride (0.020 mL) at 0°C. The resulting mixture was stirred for one hour. A saturated aqueous solution of sodium bicarbonate was added and the resulting mixture was extracted with chloroform. The organic layer was dried over anhydrous sodium sulfate and the title compound (77 mg) was obtained.
MSm/z:522 (M⁺+H).

### Example 287

Ethyl 1-{2-[(trans)-8-chloro-6-(2,3-dimethoxyphenyl)-1-isopropyl-4H,6H-[1,2,4]triazolo[4,3-a][4,1]benzoxazepin-4-yl]ethyl-1H-pyrazole-4-carboxylate

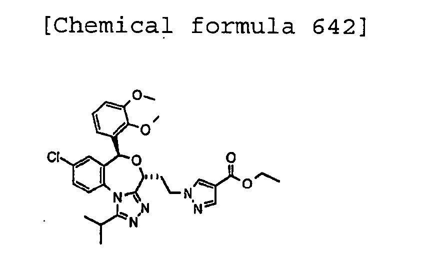

Ethyl 4-pyrazolecarboxylate (31 mg) was dissolved in N,N-dimethylformamide (1 mL). At 0°C, sodium hydride (55%, 9.7 mg) was added and the resulting mixture was stirred at 0°C for one hour. A solution of ethyl 2-[(trans)-8-chloro-6-(2,3-dimethoxyphenyl)-1-isopropyl-4H,6H-[1,2,4]triazolo[4,3-a] [4,1]benzoxazepin-4-yl]ethyl methanesulfonate (77 mg) in N,N-dimethylformamide (2 mL) was added dropwise to the reaction mixture. Tetrabutylammonium iodide (55 mg) was added further, followed by stirring at 80°C for 4 hours. To the reaction mixture was added water and the resulting mixture was extracted with chloroform. The organic layer was dried over anhydrous magnesium sulfate. After concentration of the filtrate, the residue thus obtained was purified by preparative TLC (methanol:chloroform=5:95) to give the title compound (71 mg).
MS (ESI) m/z:566 (M⁺+H).
¹H-NMR(CDCl₃)δ:1.15(3H,d,J=6.8Hz),1.32(3H,t,J=7.1Hz),1.65(3H,d ,J=6.8Hz),2.77-2.87(2H,m),3.34-3.41(1H,m),3.47(3H,s),3.87(3H,s),4.26(2H,q,J=7.1Hz),4.35-4.37(1H,m),4.47-4.61(2H,m),5.52(1H,s),6.79(1H,d,J=2.2Hz),6.98(1H,dd,J=7.8,2 .0Hz),7.18-7.25(2H,m),7.36(1H,d,J=8.3Hz),7.46(1H,dd,J=8.3,2.2Hz),7.84( 1H,s),7.90(1H,s).

### Example 288

1-{2-[(trans)-8-Chloro-6-(2,3-dimethoxyphenyl)-1-isopropyl-4H,6H-[1,2,4]triazolo[4,3-a][4,1]benzoxazepin-4-yl]ethyl-1H-pyrazole-4-carboxylic acid

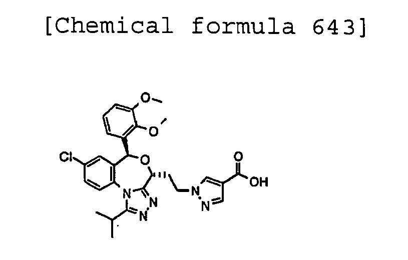

Ethyl 1-{2-[(trans)-8-chloro-6-(2,3-dimethoxyphenyl)-1-isopropyl-4H,6H-[1,2,4]triazolo[4,3-a][4,1]benzoxazepin-4-yl]ethyl-1H-pyrazole-4-carboxylate (70 mg) was dissolved in methanol-water-tetrahydrofuran (1:0.5:1, 2.5 mL). To the resulting solution was added lithium hydroxide monohydrate (10 mg). The resulting mixture was stirred at 50°C for 2 hours. After the reaction mixture was neutralized with an acidic resin (Amberlite IR-120B), the resin was filtered out and the residue was washed with methanol. The filtrate was concentrated and the residue thus obtained was purified by preparative TLC (methanol:chloroform=1:9) to give the title compound (59 mg).
MS(ESI)m/z:538(M⁺+H).
¹H-NMR(CDCl₃)δ:1.13(3H,d,J=6.8Hz),1.63(3H,d,J=6.8Hz),2.76-2.88(2H,m),3.33-3.40(1H,m),3.46(3H,s),3.85(3H,s),4.37(1H,t,J=6.2Hz),4.44-4.55(2H,m),5.50(1H,s),6.78(1H,d,J=2.2Hz),6.97(1H,dd,J=8.1,1 .5Hz),7.17-7.24(2H,m),7.37(1H,d,J=8.3Hz),7.45(1H,dd,J=8.3,2.2Hz),7.82( 1H,s),7.96(1H,s).

### Referential Example 183

Ethyl 2-[(trans)-7-chloro-2-[2-(2-methoxyacetyl)hydrazono]-5-(2,3-dimethoxyphenyl)-1,5-dihydro-4,1-benzoxazepin3(3H)-yl]acetate

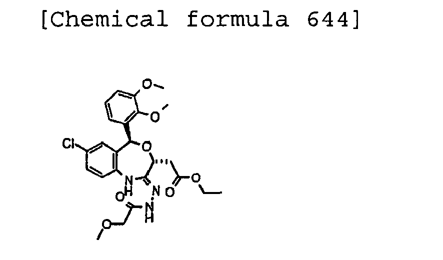

Ethyl 2-[(trans)-7-chloro-5-(2,3-dimethoxyphenyl)-2-[hydrazono]-1,5-dihydro-4,1-benzoxazepin3(3H)-yl]acetate (159 mg) was dissolved in tetrahydrofuran (4 mL). To the resulting solution was added 2-methoxyacetyl chloride (0.040 mL). The reaction mixture was stirred at room temperature for 2.5 hours. To the reaction mixture was added a saturated aqueous solution of sodium bicarbonate and the resulting mixture was extracted with chloroform. The organic layer was dried over anhydrous sodium sulfate to give the title compound (185 mg).

### Example 289

Ethyl 2-[(trans)-8-chloro-6-(2,3-dimethoxyphenyl)-1-(methoxymethyl)-4H,6H-[1,2,4]triazolo[4,3-a] [4,1]benzoxazepin-4-yl]acetate

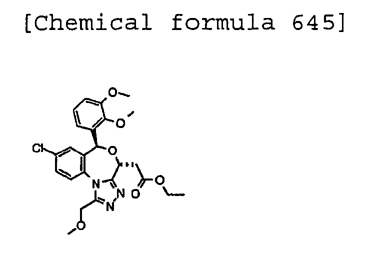

Ethyl 2-[(trans)-7-chloro-2-[2-(2-methoxyacetyl)hydrazono]-5-(2,3-dimethoxyphenyl)-1,5-dihydro-4,1-benzoxazepin3(3H)-yl]acetate (185 mg) was dissolved in acetic acid (4 mL). The resulting solution was heated under reflux overnight. The reaction mixture was cooled to room temperature and then concentrated. The residue was diluted with chloroform and washed with a saturated aqueous solution of sodium bicarbonate and saturated brine. The organic layer was dried over anhydrous sodium sulfate. After the filtrate was concentrated, the residue thus obtained was purified by preparative TLC (methanol:chloroform=5:95) to give the title compound (76 mg).
MS m/z:488 (M⁺+H).
¹H-NMR(CDCl₃)δ:1.21(3H,t,J=7,1Hz),3,17(1H,dd,J=16.5,7.7Hz),3.3 5-3.41(7H,m),3.79(3H,s),4.09-4.14(2H,m),4.56(1H,d,J=12.9Hz),4.78(1H,d,J=12.9Hz),4.91(1H, dd,J=7.6,5.9Hz),5.52(1H,s),6.74(1H,d,J=2.2Hz),6.90(1H,dd,J= 7.9,1.7Hz),7.11(1H,t,J=7.9Hz),7.15-7.17(1H,m),7.41(1H,dd,J=8.5,2.2Hz),7.67(1H,d,J=8.5Hz).

### Referential Example 184

2-[(trans)-8-Chloro-6-(2,3-dimethoxyphenyl)-1-(methoxymethyl)-4H,6H-[1,2,4]triazolo[4,3-a][4,1]benzoxazepin-4-yl]-1-ethanol

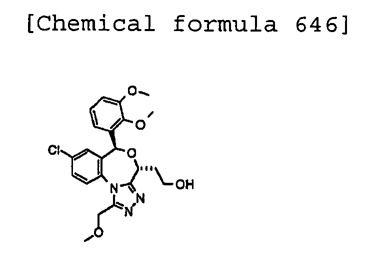

Lithium aluminum hydride (32 mg) was suspended in tetrahydrofuran (1 mL). Under stirring at -15°C, a solution of ethyl 2-[(trans)-8-chloro-6-(2,3-dimethoxyphenyl)-1-(methoxymethyl)-4H,6H-[1,2,4]triazolo[4,3-a][4,1]benzoxazepin-4-yl]acetate (76 mg) in tetrahydrofuran (2 mL) was slowly added dropwise. The reaction mixture was stirred as was for 15 minutes. An aqueous solution of potassium sodium tartrate was added to the reaction mixture. After concentration, the residue was extracted with chloroform. The organic layer was dried over anhydrous sodium sulfate. The filtrate was concentrated to give the title compound (66 mg).
MS m/z:446(M⁺+H).
¹H-NMR(CDCl₃)δ:2.45-2.51(2H,m),3.02(1H,brs),3.39(3H,s),3.39(3H,s),3.79(3H,s),3.
83-3.87(1H,m),3.91-3.96(1H,m),4.56(1H,d,J=12.9Hz),4.62(1H,t,J=6.1Hz),4.78(1H,d ,J=12.9Hz),5.53(1H,s),6.75(1H,d,J=2.4Hz),6.91(1H,dd,J=7.9,1 .5Hz),7.13(1H,t,J=7.9Hz),7.17-7.19(2H,m),7.41(1H,dd,J=8.5,2.4Hz),7.67(1H,d,J=8.5Hz).

### Referential Example 185

2-[(trans)-8-Chloro-6-(2,3-dimethoxyphenyl)-1-(methoxymethyl)-4H,6H-[1,2,4]triazolo[4,3-a] [4,1]benzoxazepin-4-yl]ethyl methanesulfonate

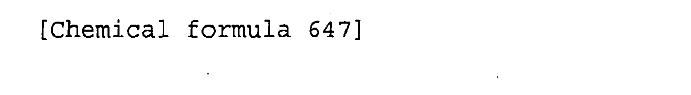

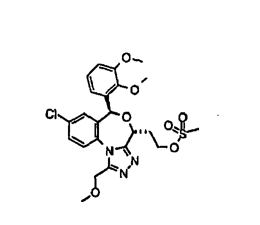

2-[(trans)-8-Chloro-6-(2,3-dimethoxyphenyl)-1-(methoxymethyl)-4H,6H-[1,2,4]triazolo[4,3-a][4,1]benzoxazepin-4-yl]-1-ethanol (67 mg) was dissolved in dichloromethane (3 mL). To the resulting solution were added triethylamine (0.032 mL) and methanesulfonyl chloride (0.018 mL) at 0°C. The resulting mixture was stirred for one hour. To the reaction mixture was added a saturated aqueous solution of sodium bicarbonate, followed by extraction with chloroform. The organic layer was dried over anhydrous sodium sulfate to give the title compound (81 mg) .
MS m/z:524 (M⁺+H) .

### Example 290

Ethyl 1-{2-[(trans)-8-chloro-6-(2,3-dimethoxyphenyl)-1-(methoxymethyl)-4H,6H-[1,2,4]triazolo[4,3-a][4,1]benzoxazepin-4-yl]ethyl-1H-pyrazole-4-carboxylate

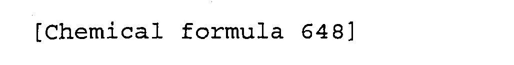

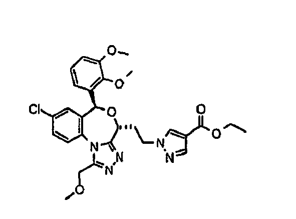

Ethyl 4-pyrazolecarboxylate (25 mg) was dissolved in N,N-dimethylformamide (1 mL). At 0°C, sodium hydride (55%, 7.9 mg) was added and the resulting mixture was stirred at 0°C for one hour. A solution of ethyl 2-[(trans)-8-chloro-6-(2,3-dimethoxyphenyl)-1-(methoxymethyl)-4H,6H-[1,2,4]triazolo[4,3-a][4,1]benzoxazepin-4-yl]ethyl methanesulfonate (79 mg) in N,N-dimethylformamide (2 mL) was added dropwise to the reaction mixture. Tetrabutylammonium iodide (56 mg) was added further and the resulting mixture was stirred at 80°C for 5 hours. Water was added and the resulting mixture was extracted with chloroform. The organic layer was dried over anhydrous magnesium sulfate. After concentration of the filtrate, the residue thus obtained was purified by preparative TLC (methanol:chloroform=5:95) to give the title compound (59 mg).
MS(ESI)m/z:568(M⁺+H).
¹H-NMR(CDCl₃)δ:1.32(3H,t,J=7.2Hz),2.82-2.92(2H,m),3.45(3H,s),3.45(3H,s),3.87(3H,s),4.26(2H,q,J=7.1 Hz),4.45(1H,dd,J=7.6,5.4Hz),4.49-4.57(2H,m),4.63(1H,d,J=12.9Hz),4.85(1H,d,J=12.9Hz),5.59(1H, s),6.81(1H,d,J=2.2Hz),6.99(1H,dd,J=7.8,2.2Hz),7.19-7.26(2H,m),7.47(1H,dd,J=8.5,2.2Hz),7.73(1H,d,J=8.5Hz),7.85( 1H,s),7.92(1H,s).

### Example 291

1-{2-[(trans)-8-Chloro-6-(2,3-dimethoxyphenyl)-1-(methoxymethyl)-4H,6H-[1,2,4]triazolo[4,3-a][4,1]benzoxazepin-4-yl]ethyl-1H-pyrazole-4-carboxylic acid

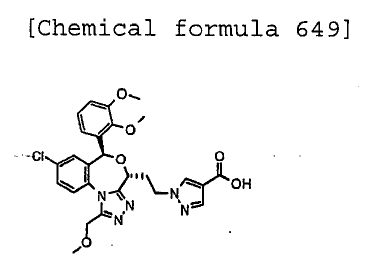

Ethyl 1-{2-[(trans)-8-chloro-6-(2,3-dimethoxyphenyl)-1-(methoxymethyl)-4H,6H-[1,2,4]triazolo[4,3-a][4,1]benzoxazepin-4-yl]ethyl-1H-pyrazole-4-carboxylate (59 mg) was dissolved in methanol-water-tetrahydrofuran (1:0.5:1, 2.5 mL). To the resulting solution was added lithium hydroxide monohydrate (13 mg). At 50°C, the resulting mixture was stirred overnight. After the reaction mixture was neutralized with an acidic resin (Amberlite IR-120B), the resin was filtered out and the residue was washed with methanol. After concentration of the filtrate, the residue thus obtained was purified by preparative TLC (methanol:chloroform:water=3:7:1) to give the title compound (48 mg).
MS(ESI)m/z:540(M⁺+H).
¹H-NMR(CDCl₃)δ:2.77-2.90(2H,m),3.42(3H,s),3.43(3H,s),3.85(3H,s),4.44-4.52(3H,m),4.62(1H,d,J=12.9Hz),4.85(1H,d,J=12.9Hz),5.57(1H, s),6.79(1H,d,J=2.2Hz),6.95-6.98(1H,m),7.19(1H,t,J=7.9Hz),7.23-7.25(1H,m),7.45(1H,dd,J=8.4,2.2Hz),7.71(1H,d,J=8.4Hz),7.82( 1H,s),7.96(1H,s).

### Referential Example 186

Ethyl 2-[(trans)-7-chloro-5-(2,3-dimethoxyphenyl)-2-[2-(2-fluoroacetyl)hydrazono]-1,5-dihydro-4,1-benzoxazepin3(3H)-yl]acetate

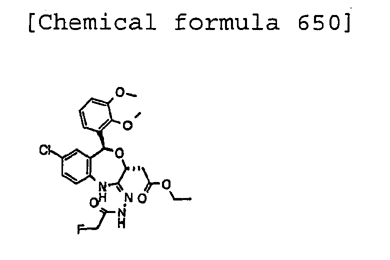

Ethyl 2-[(trans)-7-chloro-5-(2,3-dimethoxyphenyl)-2-[hydrazono]-1,5-dihydro-4,1-benzoxazepin3(3H)-yl]acetate (159 mg) was dissolved in tetrahydrofuran (4 mL). The resulting solution was added 2-fluoroacetyl chloride (127 mg). The resulting mixture was stirred at room temperature for 5.5 hours. To the reaction mixture was added a saturated aqueous solution of sodium bicarbonate, followed by extraction with chloroform. The organic layer was dried over anhydrous sodium sulfate to give the title compound (181 mg).
MS(ESI)m/z:495(M⁺+H).

### Example 292

Ethyl 2-[(trans)-8-chloro-6-(2,3-dimethoxyphenyl)-1-(fluoromethyl)-4H,6H-[1,2,4]triazolo[4,3-a][4,1]benzoxazepin-4-yl]acetate

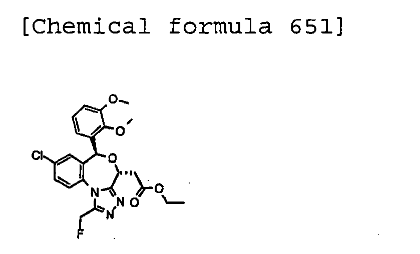

Ethyl 2-[(trans)-7-chloro-5-(2,3-dimethoxyphenyl)-2-[2-(2-fluoroacetyl)hydrazono]-1,5-dihydro-4,1-benzoxazepin3 (3H)-yl]acetate (180 mg) was dissolved in acetic acid (5 mL). The resulting solution was heated under reflux overnight. After cooling to room temperature, the reaction mixture was concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (68 mg).
MS m/z:476(M⁺+H).
¹H-NMR(CDCl₃)δ:1.28(3H,t,J=7.2Hz),3.24(1H,dd,J=16.5,7.4Hz),3.4 4-3.50(4H,m),3.86(3H,s),4.16-4.23(2H,m),5.00-5.03(1H,m),5.42(1H,dd,J=48.1,11.7Hz),5.58(1H,s),5.86(1H,dd, J=48.1,11.7Hz),6.84(1H,d,J=2.4Hz),6.98(1H,dd,J=7.8,2.0Hz),7 .17-7.24(2H,m),7.52(1H,dd,J=8.5,2.4Hz),7.62(1H,d,J=8.5Hz).

### Referential Example 187

2-[(trans)-8-Chloro-6-(2,3-dimethoxyphenyl)-1-(fluoromethyl)-4H,6H-[1,2,4]triazolo[4,-3-a][4,1]benzoxazepin-4-yl]-1-ethanol

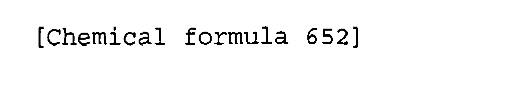

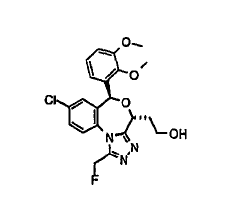

Lithium aluminum hydride (30 mg) was suspended in tetrahydrofuran (1 mL). Under stirring at -15°C, a solution of ethyl 2-[(trans)-8-chloro-6-(2,3-dimethoxyphenyl)-1-(fluoromethyl)-4H,6H-[1,2,4]triazolo[4,3-a][4,1]benzoxazepin-4-yl]acetate (68 mg) in tetrahydrofuran (2 mL) was slowly added dropwise to the resulting suspension. The reaction mixture was stirred as was for 15 minutes. An aqueous solution of potassium sodium tartrate was added and the resulting mixture was concentrated. The residue was extracted with chloroform. The organic layer was dried over anhydrous sodium sulfate. The filtrate was concentrated to give the title compound (81 mg).
MS m/z:434(M⁺+H).
¹H-NMR(CDCl₃)δ:2.45-2.52(2H,m),3.40(3H,s),3.79(3H,s),3.85-3.94(2H,m),4.66(1H,t,J=6.2Hz),5.34(1H,dd,J=48.1,11.7Hz),5.5 1(1H,s),5.79(1H,dd,J=48.1,11.7Hz),6.78(1H,d,J=2.2Hz),6.91(1 H,dd,J=7.9,1.3Hz),7.11-7.18(2H,m),7.45(1H,dd,J=8.5,2.2Hz),7.54(1H,d,J=8.5Hz).

### Referential Example 188

2-[(trans)-8-Chloro-6-(2,3-dimethoxyphenyl)-1-(fluoromethyl)-4H,6H-[1,2,4]triazolo[4,3-a] [4,1]benzoxazepin-4-yl]ethyl methanesulfonate

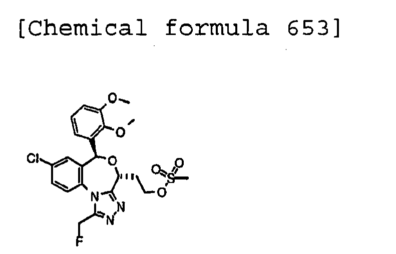

2-[(trans)-8-Chloro-6-(2,3-dimethoxyphenyl)-1-(fluoromethyl)-4H,6H-[1,2,4]triazolo[4,3-a][4,1]benzoxazepin-4-yl]-1-ethanol (62 mg) was dissolved in dichloromethane (2 mL). To the resulting solution were added triethylamine (0.030 mL) and methanesulfonyl chloride (0.017 mL) at 0°C. The resulting mixture was stirred for one hour. To the reaction mixture was added a saturated aqueous solution of sodium bicarbonate. The resulting mixture was extracted with chloroform. The organic layer was dried over anhydrous sodium sulfate to give the title compound (73 mg).
MS m/z:512(M⁺+H).

### Example 293

Ethyl 1-{2-[(trans)-8-chloro-6-(2,3-dimethoxyphenyl)-1-(fluoromethyl)-4H,6H-[1,2,4]triazolo[4,3-a][4,1]benzoxazepin-4-yl]ethyl-1H-pyrazole-4-carboxylate

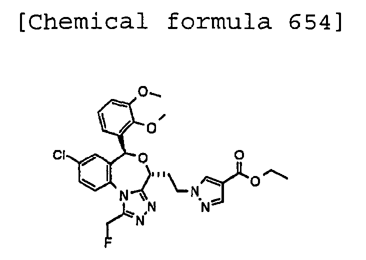

Ethyl 4-pyrazolecarboxylate (24 mg) was dissolved in N,N-dimethylformamide (1 mL). At 0°C, sodium hydride (55%, 7.2 mg) was added and the resulting mixture was stirred at 0°C for one hour. A solution of 2-[(trans)-8-chloro-6-(2,3-dimethoxyphenyl)-1-(fluoromethyl)-4H,6H-[1,2,4]triazolo[4,3-a][4,1]benzoxazepin-4-yl]ethyl methanesulfonate (73 mg) in N,N-dimethylformamide (2 mL) was added dropwise to the reaction mixture. Tetrabutylammonium iodide (53 mg) was added further and the resulting mixture was stirred at 80°C for 3 hours. Water was added and the resulting mixture was extracted with chloroform. The organic layer was dried over anhydrous magnesium sulfate. After concentration of the filtrate, the residue thus obtained was purified by preparative TLC (methanol:chloroform=5:95) to give the title compound (19 mg) .
MS (ESI)m/z:556(M⁺+H).
¹H-NMR(CDCl₃)δ:1.23-1.27(3H,m), 2.77-2.86(2H,m),3.39(3H,s),3.80(3H,s),4.17-4.22(2H,m),4.40-4.49(3H,m),5.34(1H,dd,J=48.1,11.7Hz),5.50(1H,s),5.79(1H,dd, J=48.1,11.7Hz),6.77(1H,d,J=2.2Hz),6.93(1H,dd,J=7.2,2.6Hz),7 .12-7.18(2H,m),7.44(1H,dd,J=8.5,2.2Hz),7.53(1H,d,J=8.5Hz),7.78( 1H, s), 7. 85 (1H, s) .

### Example 294

1-{2-[(trans)-8-Chloro-6-(2,3-dimethoxyphenyl)-1-(fluoromethyl)-4H,6H-[1,2,4]triazolo[4,3-a][4,1]benzoxazepin-4-yl]ethyl-1H-pyrazole-4-carboxylic acid

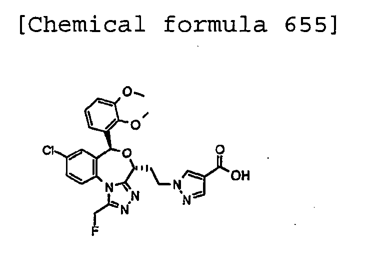

Ethyl 1-{2-[(trans)-8-chloro-6-(2,3-dimethoxyphenyl)-1-(fluoromethyl)-4H,6H-[1,2,4]triazolo[4,3-a][4,1]benzoxazepin-4-yl]ethyl-1H-pyrazole-4-carboxylate (19 mg) was dissolved in methanol-water-tetrahydrofuran (1:0.5:1, 2.5 mL). To the resulting solution was added lithium hydroxide monohydrate (4.3 mg). At 50°C, the resulting mixture was stirred overnight. After the reaction mixture was neutralized with an acidic resin (Amberlite IR-120B), the resin was filtered out and the residue was washed with methanol. The filtrate was concentrated and the residue thus obtained was purified by preparative TLC (methanol:chloroform=1:9) to give the title compound (13 mg).
MS (ESI) m/z: 528 (M⁺+H)
¹H-NMR(CDCl₃)δ:2.72-2.84(2H,m),3.38(3H,s),3.79(3H,s),4.40-4.46(3H,m),5.34(1H,dd,J=48.3,11.7Hz),5.49(1H,s),5.78(1H,dd, J=48.3,11.7Hz),6.76(1H,d,J=2.2Hz),6.91(1H,dd,J=7.6,2.0Hz),7 .11-7.17(2H,m),7.41-7.45(1H,m),7.52(1H,d,J=8.3Hz),7.77(1H,s),7.88(1H,s).

### Example 295

Ethyl 2-[(trans)-8-chloro-6-(2,3-dimethoxyphenyl)-1-(trifluoromethyl)-4H,6H-[1,2,4]triazolo[4,3-a][4,1]benzoxazepin-4-yl]acetate

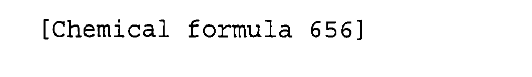

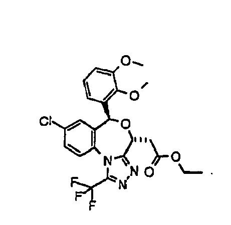

Ethyl 2-[(trans)-7-chloro-5-(2,3-dimethoxyphenyl)-2-[hydrazono]-1,5-dihydro-4,1-benzoxazepin3(3H)-yl]acetate (30 mg) was dissolved in dichloromethane (3 mL). To the resulting solution were added trifluoroacetic anhydride (0.027 mL) and trifluoroacetic acid (0.106 mL). The resulting mixture was stirred at room temperature for one hour. After stirring the reaction mixture at 55°C and distilling off dichloromethane, toluene (3 mL) was added to the residue. The resulting mixture was heated under reflux for further one hour. After cooling to room temperature, the reaction mixture was concentrated. The residue was diluted with dichloromethane, and washed with a saturated aqueous solution of sodium bicarbonate. The organic layer was dried over anhydrous sodium sulfate. After concentration of the filtrate, the residue thus obtained was purified by preparative TLC (methanol:dichloromethane=1:9) to give the title compound (24 mg).
MS (ESI)m/z:512 (M⁺+H).
¹H-NMR(CDCl₃)δ:1.28(3H,td,J=7.1,1.4Hz),3.24(1H,dd,J=16.7,7.0Hz ),3.43(3H,d,J=1.2Hz),3.49(1H,dd,J=16.7,6.5Hz),3.86(3H,d,J=1 .2Hz),4.19(2H,ddd,J=14.3,7.1,1.5Hz),4.93(1H,t,J=6.8Hz),5.55 (1H,s),6.8S(1H,d,J=1.2Hz),6.98(1H,dd,J=6.8,2.9Hz),7.18(2H,d d,J=9.5,5.1Hz),7.52(2H,s).

### Referential Example 189

2-[(trans)-8-Chloro-6-(2,3-dimethoxyphenyl)-1-(trifluoromethyl)-4H,6H-[1,2,4]triazolo[4,3-a] [4,1]benzoxazepin-4-yl]-1-ethanol

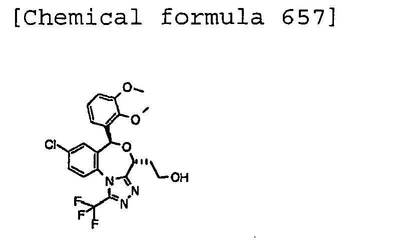

To a solution of ethyl 2-[(trans)-8-chloro-6-(2,3-dimethoxyphenyl)-1-(trifluoromethyl)-4H,6H-[1,2,4]triazolo[4,3-a][4,1]benzoxazepin-4-yl]acetate (50 mg) in tetrahydrofuran (10 mL) was added lithium borohydride (4.3 mg). The resulting mixture was stirred overnight at room temperature. Lithium borohydride (4.3 mg) was added again and the resulting mixture was stirred at 50°C for 1 hour. Water was added and the resulting mixture was extracted with chloroform. The organic layers were combined and dried over anhydrous sodium sulfate. After concentration of the filtrate, the residue thus obtained was purified by preparative TLC (methanol:dichloromethane=1:9) to give the title compound (28 mg).
¹H-NMR(CDCl₃)δ:2.58(2H,dt,J=11.4,4.3Hz),3.45(3H,s),3.86(3H,s), 3.98(2H,dt,J=8.0,2.8Hz),4.67(1H,t,J=6.3Hz),5.56(1H,s),6.86( 1H,s),6.99(1H,dd,J=7.6,2.2Hz),7.18-7.24(2H,m),7.52(2H,d,J=1.7Hz).
HRMS(FAB)m/z:
Calculated: C₂₁H₁₉N₃O₄ClF₃;470.1094.
Found: 470.1100.
IR(ATR)cm⁻¹:3359,1483,1282,1196,1155,1086,1047,999,822,754.

### Referential Example 190

2-[(trans)-8-Chloro-6-(2,3-dimethoxyphenyl)-1-(trifluoromethyl)-4H,6H-[1,2,4]triazolo[4,3-a] [4,1]benzoxazepin-4-yl]ethyl methanesulfonate

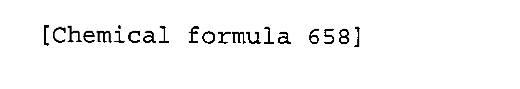

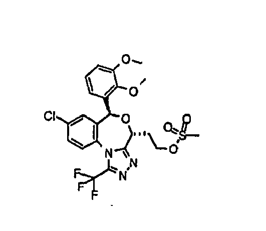

2-[(trans)-8-Chloro-6-(2,3-dimethoxyphenyl)-1-(trifluoromethyl)-4H,6H-[1,2,4]triazolo[4,3-a] [4,1]benzoxazepin-4-yl]-1-ethanol (89 mg) was dissolved in dichloromethane (15 mL). To the resulting solution were added triethylamine (0.131 mL) and methanesulfonyl chloride (0.044 mL) at 0°C. The resulting mixture was stirred for 0.5 hour. A saturated aqueous solution of sodium bicarbonate was added and the resulting mixture was extracted with chloroform. The organic layer was dried over anhydrous sodium sulfate to give the title compound (103 mg).

### Example 296

Ethyl 1-{2-[(trans)-8-chloro-6-(2,3-dimethoxyphenyl)-1-(trifluoromethyl)-4H,6H-[1,2,4]triazolo[4,3-a][4,1]benzoxazepin-4-yl]ethyl-1H-pyrazole-4-carboxylate

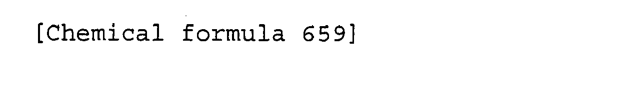

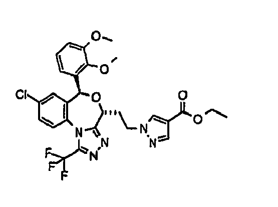

Ethyl 4-pyrazolecarboxylate (32 mg) was dissolved in N,N-dimethylformamide (1 mL). At 0°C, sodium hydride (55%, 9.1 mg) was added to the resulting solution, followed by stirring at 50°C for one hour. A solution of 2-[(trans)-8-chloro-6-(2,3-dimethoxyphenyl)-1-(trifluoromethyl)-4H,6H-[1,2,4]triazolo[4,3-a][4,1]benzoxazepin-4-yl]ethyl methanesulfonate (103 mg) in N,N-dimethylformamide (2 mL) was added dropwise to the reaction mixture. Tetrabutylammonium iodide (69 mg) was added further and the resulting mixture was stirred overnight at room temperature. To the reaction mixture was added water and the resulting mixture was extracted with ethyl acetate.
The organic layer was dried over anhydrous sodium sulfate. After concentration of the filtrate, the residue thus obtained was purified by preparative TLC (methanol:dichloromethane=1:9) to give the title compound (79 mg).
MS(ESI)m/z:592(M⁺+H).
¹H-NMR(CDCl₃)δ:1.32(3H,t,J=7.2Hz),2.83-2.95(2H,m),3.43(3H,s),3.87(3H,s),4.26(2H,q,J=7.1Hz),4.40(1H ,dd,J=7.4,5.5Hz),4.53(2H,td,J=13.7,7.0Hz),5.55(1H,s),6.85(1 H,d,J=2.0Hz),7.00(1H,t,J=4.9Hz),7.21(2H,d,J=5.1Hz),7.50(2H, dt,J=10.7,4.6Hz),7.83(1H,s),7.91(1H,s).

### Example 297

1-{2-[(trans)-8-Chloro-6-(2,3-dimethoxyphenyl)-1-(trifluoromethyl)-4H,6H-[1,2,4]triazolo[4,3-a][4,1]benzoxazepin-4-yl]ethyl-1H-pyrazole-4-carboxylic acid

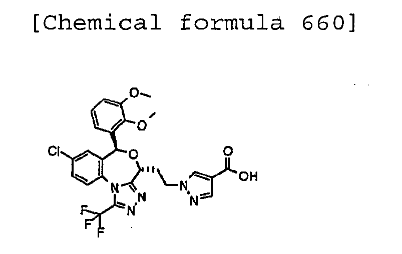

Ethyl 1-{2-[(trans)-8-chloro-6-(2,3-dimethoxyphenyl)-1-(trifluoromethyl)-4H,6H-[1,2,4]triazolo[4,3-a][4,1]benzoxazepin-4-yl]ethyl-1H-pyrazole-4-carboxylate (136 mg) was dissolved in ethanol-water-tetrahydrofuran (10:5:5, 20 mL). To the resulting solution was added lithium hydroxide monohydrate (19 mg) and the resulting mixture was stirred overnight at 50°C. The reaction mixture was neutralized with 1N hydrochloric acid and then, extracted with dichloromethane. The organic layer was dried over anhydrous sodium sulfate. After concentration of the filtrate, the residue thus obtained was purified by preparative TLC (methanol:dichloromethane=1:9) to give the title compound (120 mg).
MS(ESI)m/z:564(M⁺+H).
¹H-NMR(CDCl₃)δ:2.87-2.93(2H,m),3.43(3H,s),3.86(3H,s),4.41(1H,t,J=6.3Hz),4.56(2H ,dt,J=18.8,6.9Hz),5.55(1H,s),6.85(1H,d,J=1.7Hz),7.00(1H,dd, J=6.2,3.5Hz),7.21(2H,t,J=3.1Hz),7.50(2H,dt,J=10.7,4.5Hz),7. 88(1H,s),7.97(1H,s).
IR(ATR)cm ¹:1712,1685,1556,1483,1284,1200,1171,1146,1074,995,768.
HRMS(FAB)m/z:
Calculated: C₂₅H₂₂O₅N₅ClF₃; 564.1262.
Found: 564.1245.

### Example 298

Ethyl 2-(2-{2-[(trans)-8-chloro-6-(2,3-dimethoxyphenyl)-1-(trifluoromethyl)-4H,6H-[1,2,4]triazolo[4,3-a][4,1]-benzoxazepin-4-yl]ethyl}-2H-1,2,3,4-tetrazol-5-yl)acetate
Ethyl 2-(2-{2-[(trans)-8-chloro-6-(2,3-dimethoxyphenyl)-1-(trifluoromethyl)-4H,6H-[1,2,4]triazolo[4,3-a][4,1]-benzoxazepin-4-yl]ethyl}-1H-1,2,3,4-tetrazol-5-yl)acetate

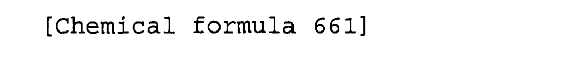

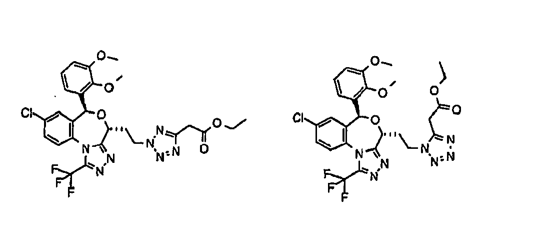

2-[(trans)-8-Chloro-6-(2,3-dimethoxyphenyl)-1-(trifluoromethyl)-4H,6H-[1,2,4]triazolo[4,3-a][4,1]benzoxazepin-4-yl]ethyl methanesulfonate (127 mg) and ethyl 1H-tetrazole 5-acetate (109 mg) were dissolved in N,N-dimethylformamide (2 mL). To the resulting solution were added potassium carbonate (160 mg) and tetrabutylammonium iodide (86 mg). The resulting mixture was stirred at 80°C for 4 hours. The reaction mixture was diluted with ethyl acetate and washed with saturated brine. The organic layer was dried over anhydrous sodium sulfate. After concentration of the filtrate, the residue thus obtained was purified by preparative TLC (methanol:dichloromethane=1:9) to give the title compounds, 2H-isomer (73 mg) and 1H-isomer (39 mg).
Low polarity fraction (2H-isomer)
¹H-NMR(CDCl₃)δ:1.25(3H,t,J=7.1Hz),3.04(2H,q,J=6.6Hz),3.44(3H,s ),3.88(5H,d,J=7.8Hz),4.18(2H,q,J=7.1Hz),4.47(1H,t,J=6.5Hz), 5.03(2H,tt,J=20.4,6.8Hz),5.58(1H,s),6.83(1H,s),7.00(1H,dd,J =6.1,3.7Hz),7.21(2H,dt,J=9.5,3.8Hz),7.50(2H,s).
High polarity fraction (1H-isomer)
¹H-NMR(CDCl₃)δ:1.28(3H,t,J=7.2Hz),3.01(2H,ddd,J=15.0,9.6,5.6Hz ),3.43(3H,d,J=2.2Hz),3.79-4.19(7H,m),4.53(1H,t,J=6.2Hz),4.74(2H,dq,J=26.9,7.0Hz),5.58 (1H,s),6.86(1H,d,J=2.0Hz),7.00(1H,dd,J=5.9,3.9Hz),7.22(2H,q ,J=2.8Hz),7.50(2H,t,J=2.3Hz).

### Example 299

2-(2-{2-[(trans)-8-Chloro-6-(2,3-dimethoxyphenyl)-1-(trifluoromethyl)-4H,6H-[1,2,4]triazolo[4,3-a] [4,1]-benzoxazepin-4-yl]ethyl}-2H-1,2,3,4-tetrazol-5-yl)acetic acid

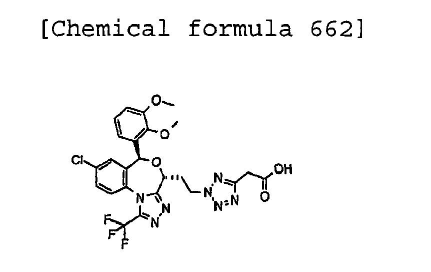

Ethyl 2-(2-{2-[(trans)-8-chloro-6-(2,3-dimethoxyphenyl)-1-(trifluoromethyl)-4H,6H-[1,2,4]triazolo[4,3-a][4,1]-benzoxazepin-4-yl]ethyl}-2H-1,2,3,4-tetrazol-5-yl)acetate (71 mg) was dissolved in ethanol-water-tetrahydrofuran (8:4:4, 16 mL). To the resulting solution was added potassium carbonate (49 mg). At 50°C, the resulting mixture was stirred for 1.5 hours. The reaction mixture was neutralized with 1N hydrochloric acid and then, extracted with dichloromethane. The organic layer was dried over anhydrous sodium sulfate. After concentration of the filtrate, the residue thus obtained was purified by preparative TLC (methanol:chloroform:water=3:7:1) to give the title compound (46 mg).
MS (ESI) m/z : 580 (M⁺+H) .
¹H-NMR(CDCl₃)δ:2.99(2H,q,J=6.7Hz),3.42(3H,s),3.83(2H,s),3.86(3 H,s),4.49(1H,t,J=6.3Hz),5.00(2H,dt,J=20.0,6.8Hz),5.56(1H,s)
,6.82(1H,s),6.98(1H,q,J=3.3Hz),7.17-7.22(2H,m),7.49(2H,s).
IR (ATR) cm ¹:1732,1483,1284,1200,1171,1146,1070,995,825,768,748.
HRMS(FAB)m/z:
Calculated: C₂₄H₂₂O₅N₇F₃;580.1323.
Found: 580.1311.

### Example 300

Ethyl 2-[(trans)-8-chloro-6-(2,3-dimethoxyphenyl)-4H,6H-[1,2,4]triazolo[4,3-a][4,1]benzoxazepin-4-yl]acetate

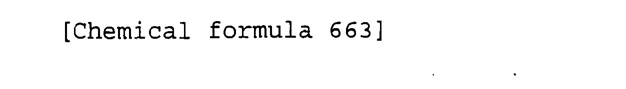

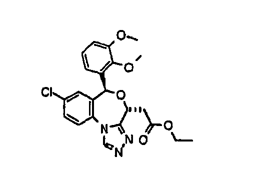

Ethyl 2-[(trans)-7-chloro-5-(2,3-dimethoxyphenyl)-2-[hydrazono]-1,5-dihydro-4,1-benzoxazepin3(3H)-yl]acetate (199 mg) was dissolved in chloroform (6 mL). To the resulting solution were added ethyl orthoformate (0.382 mL) and sulfuric acid (0.092 mL). The resulting mixture was stirred at room temperature for 3 hours. The reaction mixture was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=1:9) to give the title compound (155 mg).
MS m/z:444 (M⁺+H).
¹H-NMR(CDCl₃)δ:1.26(3H,t,J=7.2Hz),3.22(1H,dd,J=16.6,7.8Hz),3.4 7(1H,dd,J=16.5,5.5Hz),3.54(3H,s),3.87(3H,s),4.15-4.20(2H,m),5.16(1H,dd,J=7.8,5.6Hz),5.74(1H,s),6.80(1H,d,J=2 .2Hz),6.98(1H,dd,J=7.6,2.2Hz),7.16-7.23(2H,m),7.42(1H,d,J=8.5Hz),7.47(1H,dd,J=8.5,2.2Hz),8.61( 1H, s) .

### Example 301

Ethyl 2-[(trans)-1,8-dichloro-6-(2,3-dimethoxyphenyl)-4H,6H-[1,2,4]triazolo[4,3-a][4,1]benzoxazepin-4-yl]acetate

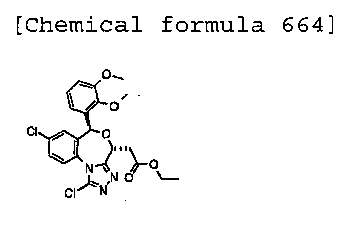

Ethyl 2-[(trans)-8-chloro-6-(2,3-dimethoxyphenyl)-4H,6H-[1,2,4]triazolo[4,3-a][4,1]benzoxazepin-4-yl]acetate (155 mg) was dissolved in carbon tetrachloride (4 mL). To the resulting solution was added N-chlorosuccinimide (75 mg). The resulting mixture was heated under reflux overnight. The reaction mixture was cooled to room temperature and then concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by silica gel column (methanol:chloroform=1:9) to give the title compound (62 mg). In addition, the reaction raw material (58 mg) was collected.
MS m/z:478 (M⁺+H).
¹H-NMR(CDCl₃)δ:1.26-1.30(3H,m),3.19(1H,dd,J=16.6,7.3Hz),3.43(1H,dd,J=16.6,6.3Hz ),3.50(3H,s),3.87(3H,s),4.16-4.22(2H,m),4.95(1H,dd,J=7.3,6.3Hz),5.66(1H,s),6.84(1H,d,J=2 .2Hz),6.98(1H,dd,J=7.6,2.2Hz),7.16-7.22(2H,m),7.50-7.58(2H,m).

### Referential Example 191

2-[(trans)-8-Chloro-6-(2,3-dimethoxyphenyl)-4H,6H-[1,2,4]triazolo[4,3-a][4,1]benzoxazepin-4-yl]-1-ethanol

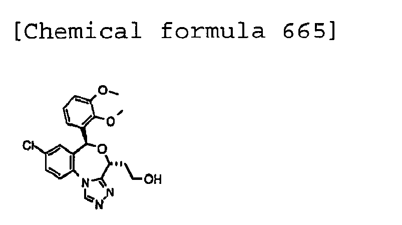

Lithium aluminum hydride (27 mg) was suspended in tetrahydrofuran (1 mL). Under stirring at -15°C, a solution of ethyl 2-[(trans)-8-chloro-6-(2,3-dimethoxyphenyl)-4H,6H-[1,2,4]triazolo[4,3-a][4,1]benzoxazepin-4-yl]acetate (58 mg) in tetrahydrofuran (1 mL) was slowly added dropwise to the resulting suspension. The reaction mixture was stirred as was for 15 minutes. An aqueous solution of potassium sodium tartrate was added and the resulting mixture was extracted with chloroform. The organic layer was dried over anhydrous sodium sulfate. The filtrate was concentrated to give the title compound (37 mg).
MS m/z:402(M⁺+H).
¹H-NMR(CDCl₃)δ:2.47-2.64(2H,m),3.55(3H,s),3.88(3H,s),3.91-4.01(2H,m),4.87(1H,dd,J=7.0,5.5Hz),5.76(1H,s),6.82(1H,d,J=2 .4Hz),6.99(1H,dd,J=7.3,2.2Hz),7.18-7.25(2H,m),7.41-7.49(2H,m),8.61(1H,s).

### Referential Example 192

2-[(trans)-1,8-Dichloro-6-(2,3-dimethoxyphenyl)-4H,6H-[1,2,4]triazolo[4,3-a][4,1]benzoxazepin-4-yl]-1-ethanol

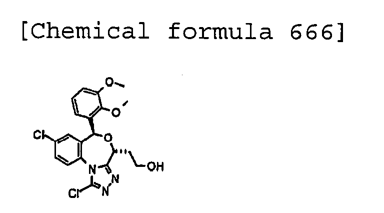

Lithium aluminum hydride (27 mg) was suspended in tetrahydrofuran (1 mL). Under stirring at -15°C, a solution of ethyl 2[(trans)-1,8-dichloro-6-(2,3-dimethoxyphenyl)-4H,6H-[1,2,4]triazolo[4,3-a][4,1]benzoxazepin-4-yl]acetate (62 mg) in tetrahydrofuran (1 mL) was slowly added dropwise to the resulting suspension. The reaction mixture was stirred as was for 15 minutes. An aqueous solution of potassium sodium tartrate was added and the resulting mixture was extracted with chloroform. The organic layer was dried over anhydrous sodium sulfate. The filtrate was concentrated to give the title compound (33 mg).
MS m/z:436(M⁺+H).
¹H-NMR(CDCl₃)δ:2.48-2.58(3H,m),3.51(3H,s),3.87(3H,s) ,3.92-3.99(2H,m),4.66-4.69(1H,m),5.67(1H,s),6.85(1H,d,J=2.0Hz),6.99(1H,dd,J=7.8,1 .7Hz),7.18-7.25(2H,m),7.52-7.57(2H,m).

### Example 302

Ethyl 2-(2-{2-[(trans)-8-chloro-6-(2,3-dimethoxyphenyl)-1-(fluoromethyl)-4H,6H-[1,2,4]triazolo[4,3-a][4,1]-benzoxazepin-4-yl]ethyl}-2H-1,2,3,4-tetrazol-5-yl)-2-methylpropionate

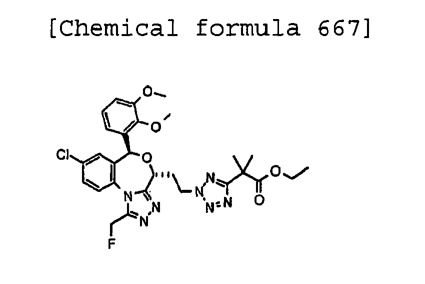

2-[(trans)-8-Chloro-6-(2,3-dimethoxyphenyl)-1-(fluoromethyl)-4H,6H-[1,2,4]triazolo[4,3-a][4,1]benzoxazepin-4-yl]ethyl methanesulfonate (76 mg) and ethyl 2,2,-dimethyl-(1H-1,2,3,4-tetrazol-5-yl)acetate (25 mg) were dissolved in N,N-dimethylformamide (3 mL). To the resulting solution were added potassium carbonate (21 mg) and tetrabutylammonium iodide (55 mg). The resulting mixture was stirred overnight at 80°C. The reaction mixture was diluted with ethyl acetate and washed with saturated brine. The organic layer was dried over anhydrous sodium sulfate. After concentration of the filtrate, the residue thus obtained was purified by preparative TLC (methanol:chloroform=5:95) to give the title compound (42 mg).
MS(ESI)m/z:600(M⁺+H).
¹H-NMR(CDCl₃)δ:1.14(3H,t,J=7.2Hz),1.63(6H,d,J=2.7Hz),2.98-3.06(2H,m),3.47(3H,s),3.87(3H,s),4.09(2H,q,J=7.2Hz),4.51(1H ,dd,J=7.8,5.6Hz),4.96-5.04(2H,m),5.42(1H,dd,J=48.1,11.7Hz),5.61(1H,s),5.87(1H,dd, J=48.1,11.7Hz),6.83(1H,d,J=2.2Hz),7.00(1H,dd,J=7.9,1.5Hz),7 .22(1H,t,J=7.9Hz),7.31(1H,dd,J=7.9,1.0Hz),7.51(1H,dd,J=8.5, 2.4Hz),7.60(1H,d,J=8.5Hz).

### Example 303

2-(2-{2-[(trans)-8-Chloro-6-(2,3-dimethoxyphenyl)-1-(fluoromethyl)-4H,6H-[1,2,4]triazolo[4,3-a][4,1]-benzoxazepin-4-yl]ethyl}-2H-1,2,3,4-tetrazol-5-yl)-2-methylpropionic acid

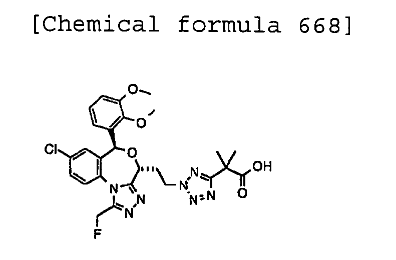

Ethyl 2-(2-{2-[(trans)-8-chloro-6-(2,3-dimethoxyphenyl)-1-(fluoromethyl)-4H,6H-[1,2,4]triazolo[4,3-a][4,1]-benzoxazepin-4-yl]ethyl}-2H-1,2,3,4-tetrazol-5-yl)-2-methylpropionate (42 mg) was dissolved in methanol-water-tetrahydrofuran (1:0.5:1, 2.5 mL). To the resulting solution was added lithium hydroxide monohydrate (8.9 mg). After the resulting mixture was stirred at 50°C for 4 hours. The reaction mixture was neutralized with an acidic resin (Amberlite IR-120B), the resin was filtered out and the residue was washed with methanol. After concentration of the filtrate, the residue thus obtained was purified by preparative TLC (methanol:chloroform:water=3:7:1) to give the title compound (31 mg).
¹H-NMR(CDCl₃)δ:1.53(6H,d,J=5.6Hz),2.92-2.97(2H,m),3.45(3H,s),3.86(3H,s),4.55(1H,t,J=6.6Hz),4.92-4.96(2H,m),5.42(1H,dd,J=48.1,11.7Hz),5.58(1H,s),5.85(1H,dd, J=48.1,11.7Hz),6.82(1H,d,J=2.2Hz),6.98(1H,dd,J=7.9,1.2Hz),7 .19(1H,t,J=7.9Hz),7.30(1H,d,J=7.9Hz),7.49(1H,dd,J=8.5,2.4Hz ),7.59(1H,d,J=8.5Hz).

### Example 304

Ethyl 2-[(2-{2-[(trans)-8-chloro-6-(2,3-dimethoxyphenyl)-1-(fluoromethyl)-4H,6H-[1,2,4]triazolo[4,3-a][4,1]-benzoxazepin-4-yl]ethyl}-2H-1,2,3,4-tetrazol-5-yl)sulfanyl]-2-methylpropionate ethyl 2-[(2-{2-[(trans)-8-chloro-6-(2,3-dimethoxyphenyl)-1-(fluoromethyl)-4H,6H-[1,2,4]triazolo[4,3-a][4,1]-benzoxazepin-4-yl]ethyl}-1H-1,2,3,4-tetrazol-5-yl)sulfanyl]-2-methylpropionate

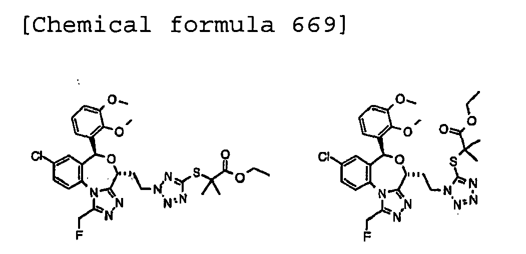

2-[(trans)-8-Chloro-6-(2,3-dimethoxyphenyl)-1-(fluoromethyl)-4H,6H-[1,2,4]triazolo[4,3-a][4,1]benzoxazepin-4-yl]ethylmethanesulfonate (83 mg) and ethyl 2-methyl-2-(1H-1,2,3,4-tetrazol-5-ylsulfanyl)propionate (33 mg) were N,N-dimethylformamide (3 mL). To the resulting solution were added potassium carbonate (23 mg) and tetrabutylammonium iodide (60 mg). The resulting mixture was stirred overnight at 80°C. The reaction mixture was diluted with ethyl acetate and washed with saturated brine. The organic layer was dried over anhydrous sodium sulfate. After concentration of the filtrate, the residue thus obtained was purified by preparative TLC (methanol:chloroform=1:99) to give the 2H-isomer (28 mg) and 1H-isomer (5 mg).
Low polarity fraction (2H-isomer)
MS (ESI) m/z : 632 (M⁺+H).
¹H-NMR(CDCl₃)δ:1.18(3H,t,J=7.2Hz),1.52(3H,s),1.56(3H,s),3.00-3.05(2H,m),3.47(3H,s),3.87(3H,s),4.07(2H,q,J=7.2Hz),4.46(1H ,t,J=6.6Hz),4.94-5.01(1H,m),5.05-5.12(1H,m),5.42(1H,dd,J=48.1,11.7Hz),5.59(1H,s),5.86(1H,dd, J=48.1,11.7Hz),6.81(1H,d,J=2.2Hz),7.00(1H,dd,J=7.9,1.7Hz),7 .23(1H,t,J=7.9Hz),7.27-7.28(1H,m),7.49(1H,dd,J=8.3,2.2Hz),7.59(1H,d,J=8.3Hz).
High polarity fraction (1H-isomer)
MS (ESI)m/z: 632 (M⁺+H).
¹H-NMR(CDCl₃)δ:1.18(3H,t,J=7.1Hz),1.72(6H,d,J=2.7Hz),2.88-2.93(2H,m),3.46(3H,s),3.87(3H,s),4.12(2H,q,J=7.2Hz),4.48(1H ,t,J=6.5Hz),4.68-4.78(2H,m), 5.42(1H,dd, J=48.0, 11.6Hz), 5.59(1H,s), 5.87(1H,dd, J=48.1,11.7Hz),6.84(1H,d,J=2.4Hz),7.00(1H,dd,J=8.1,1.6Hz),7 .22(1H,t,J=8.1Hz),7.30(1H,dd,J=8.1,1.6Hz),7.52(1H,dd,J=8.4, 2.3Hz),7.60(1H,d,J=8.3Hz).

### Example 305

2-[(2-{2-[(trans)-8-Chloro-6-(2,3-dimethoxyphenyl)-1-(fluoromethyl)-4H,6H-[1,2,4]triazolo[4,3-a][4,1]-benzoxazepin-4-yl]ethyl}-2H-1,2,3,4-tetrazol-5-yl)sulfanyl]-2-methylpropionic acid

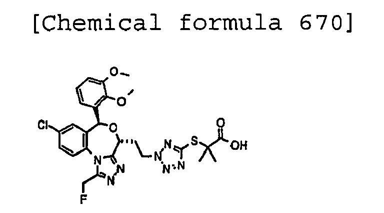

Ethyl 2-[(2-{2-[(trans)-8-chloro-6-(2,3-dimethoxyphenyl)-1-(fluoromethyl)-4H,6H-[1,2,4]triazolo[4,3-a][4,1]-benzoxazepin-4-yl]ethyl}-2H-1,2,3,4-tetrazol-5-yl)sulfanyl]-2-methylpropionate (28 mg) was dissolved in methanol-water-tetrahydrofuran (1:0.5:1, 2.5 mL). To the resulting solution was added lithium hydroxide monohydrate (5.6 mg). The resulting mixture was stirred at 50°C for 4 hours. After the reaction mixture was neutralized with an acidic resin (Amberlite IR-120B), the resin was filtered out and the residue was washed with methanol. After concentration of the filtrate, the residue thus obtained was purified by preparative TLC (methanol:chloroform:water=3:7:1) to give the title compound (28 mg).
MS (ESI) m/z : 604 (M⁺+H) .
¹H-NMR(CDCl₃)δ:1.36(3H,s),1.44(3H,s),2.93-2.98(2H,m),3.44(3H,s),3.85(3H,s),4.50(1H,t,J=6.5Hz),4.97-5.01(2H,m),5.42(1H,dd,J=48.1,11.7Hz),5.57(1H,s),5.84(1H,dd, J=48.1,11.7Hz),6.79(1H,d,J=2.2Hz),6.97(1H,dd,J=8.1,1.5Hz),7 .20(1H,t,J=8.1Hz),7.26-7.27(1H,m),7.48(1H,dd,J=8.5,2.2Hz),7.60(1H,d,J=8.5Hz).

### Example 306

2-[(2-{2-[(trans)-8-Chloro-6-(2,3-dimethoxyphenyl)-1-(fluoromethyl)-4H,6H-[1,2,4]triazolo[4,3-a][4,1]-benzoxazepin-4-yl]ethyl}-1H-1,2,3,4-tetrazol-5-yl)sulfanyl]-2-methyl-methylpropionic acid

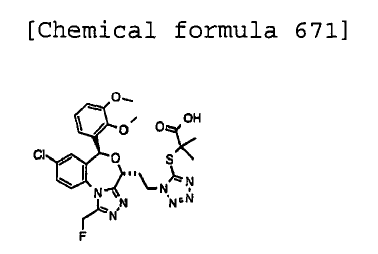

Ethyl 2-[(2-{2-[(trans)-8-chloro-6-(2,3-dimethoxyphenyl)-1-(fluoromethyl)-4H,6H-[1,2,4]triazolo[4,3-a] [4,1]-benzoxazepin-4-yl]ethyl}-1H-1,2,3,4-tetrazol-5-yl)sulfanyl]-2-methylpropionate (5.1 mg) was dissolved in methanol-water-tetrahydrofuran (1:0.5:1, 2.5mL). To the resulting solution was added lithium hydroxide monohydrate (1.0 mg) and the resulting mixture was stirred at 50°C for 4 hours. The reaction mixture was neutralized with an acidic resin (Amberlite IR-120B).
Then, the resin was filtered out and the residue was washed with methanol. After concentration of the filtrate, the residue thus obtained was purified by preparative TLC (methanol:chloroform:water=3:7:1) to give the title compound (6 mg).
MS(ESI)m/z:604(M⁺+H).
¹H-NMR(CDCl₃)δ:1.46(3H,s),1.48(3H,s),2.75-2.82(2H,m),3.42(3H,s),3.84(3H,s),4.53(1H,t,J=6.2Hz),4.59-4.68(2H,m),5.42(1H,dd,J=48.0,11.8Hz),5.57(1H,s),5.84(1H,dd, J=48.0,11.8Hz),6.81(1H,d,J=2.2Hz),6.96(1H,dd,J=8.1,1.5Hz),7 .18(1H,t,J=8.1Hz),7.26-7.28(1H,m),7.48(1H,dd,J=8.5,2.2Hz),7.60(1H,d,J=8.5Hz).

### Referential Example 193

Ethyl 2-[(trans)-7-chloro-2-[2-(3,3-dimethylbutanoyl)hydrazono]-5-(2,3-dimethoxyphenyl)-1,5-dihydro-4,1-benzoxazepin3(3H)-yl]acetate

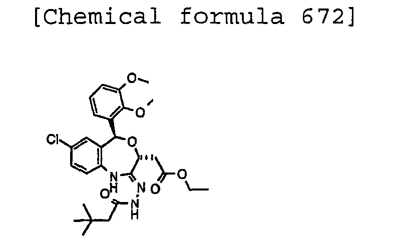

Ethyl 2-[(trans)-7-chloro-5-(2,3-dimethoxyphenyl)-2-[hydrazono]-1,5-dihydro-4,1-benzoxazepin3(3H)-yl]acetate (251 mg) was dissolved in tetrahydrofuran (5 mL). To the resulting solution was added 3,3-dimethylbutanoyl chloride (0.096 mL). The resulting mixture was stirred at room temperature for 1.5 hours. A saturated aqueous solution of sodium bicarbonate was added and the resulting mixture was extracted with chloroform. The organic layer was dried over anhydrous sodium sulfate to give the title compound (306 mg).
MS(ESI)m/z:532 (M⁺+H).

### Example 307

Ethyl 2-[(trans)-8-chloro-6-(2,3-dimethoxyphenyl)-1-neopentyl-4H,6H-[1,2,4]triazolo[4,3-a][4,1]benzoxazepin-4-yl]acetate

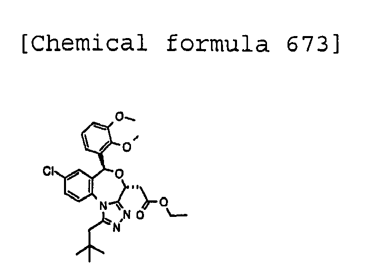

Ethyl 2-[(trans)-7-chloro-2-[2-(3,3-dimethylbutanoyl)hydrazono]-5-(2,3-dimethoxyphenyl)-1,5-dihydro-4,1-benzoxazepin3(3H)-yl]acetate (306 mg) was dissolved in acetic acid (6 mL). The resulting solution was heated under reflux for 2.5 hours. The reaction mixture was cooled to room temperature and then concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (220 mg).
MS(ESI)m/z:514 (M⁺+H).
¹H-NMR(CDCl₃)δ:0.93(9H,s),1.28(3H,t,J=7.2Hz),2.95(1H,d,J=14.4H z),3.03(1H,d,J=14.2Hz),3.22(1H,dd,J=16.4,7.8Hz),3.43(1H,dd, J=16.4,5.6Hz),3.48(3H,s),3.86(3H,s),4.19(2H,q,J=7.2Hz),4.90 (1H,dd,J=7.8,5.6Hz),5.66(1H,s),6.78(1H,d,J=2.2Hz),6.97(1H,d d,J=7.9,1.5Hz),7.18(1H,t,J=7.9Hz),7.24(1H,dd,J=7.9,1.5Hz),7 .34(1H,d,J=8.5Hz),7.49(1H,dd,J=8.5,2.3Hz).

### Referential Example 194

2-[(trans)-8-Chloro-6-(2,3-dimethoxyphenyl)-1-neopentyl-4H,6H-[1,2,4]triazolo[4,3-a][4,1]benzoxazepin-4-yl]-1-ethanol

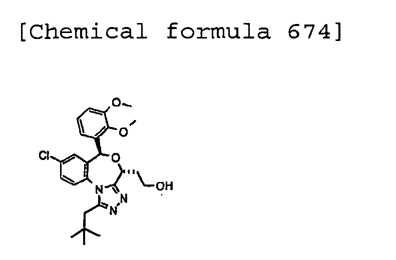

Lithium aluminum hydride (19 mg) was suspended in tetrahydrofuran (1 mL). Under stirring at -15°C, a solution of ethyl 2-[(trans)-8-chloro-6-(2,3-dimethoxyphenyl)-1-neopentyl-4H,6H-[1,2,4]triazolo[4,3-a][4,1]benzoxazepin-4-yl]acetate (23 mg) in tetrahydrofuran (2 mL) was slowly added dropwise to the resulting suspension. The reaction mixture was stirred as was for 15 minutes. To the reaction mixture was added an aqueous solution of potassium sodium tartrate. The resulting mixture was concentrated and the residue was extracted with chloroform. The organic layer was dried over anhydrous sodium sulfate. The filtrate was concentrated to give the title compound (21 mg).
MS (ESI) m/z : 472 (M⁺+H) .
¹H-NMR(CDCl₃)δ:0.87(9H,s),2.39-2.53(2H,m),2.88(1H,d,J=14.4Hz),2.96(1H,d,J=14.4Hz),3.43(3H, s),3.80(3H,s),3.81-3.86(1H,m),3.90-3.99(1H,m),4.51(1H,t,J=6.0Hz),5.59(1H,s),6.73(1H,d,J=2.2Hz) ,6.91(1H,dd,J=8.1,1.5Hz),7.13(1H,t,J=8.1Hz),7.19-7.21(1H,m),7.26(1H,d,J=8.5Hz),7.42(1H,dd,J=8.5,2.3Hz).

### Referential Example 195

2-[(trans)-8-Chloro-6-(2,3-dimethoxyphenyl)-1-neopentyl-4H,6H-[1,2,4]triazolo[4,3-a][4,1]benzoxazepin-4-yl]ethyl methanesulfonate

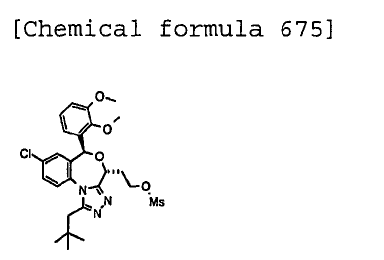

2-[(trans)-8-Chloro-6-(2,3-dimethoxyphenyl)-1-neopentyl-4H,6H-[1,2,4]triazolo[4,3-a][4,1]benzoxazepin-4-yl]-1-ethanol (21 mg) was dissolved in dichloromethane (2 mL). To the resulting solution were added triethylamine (0.0095 mL) and methanesulfonyl chloride (0.0053 mL) at 0°C. The resulting mixture was stirred for one hour. A saturated aqueous solution of sodium bicarbonate was added and the resulting mixture was extracted with chloroform. The organic layer was dried over anhydrous sodium sulfate to give the title compound (32 mg).
MS(ESI)m/z:550(M⁺+H).

### Example 308

Ethyl 2-(2-{2-[(trans)-8-chloro-6-(2,3-dimethoxyphenyl)-1-neopentyl-4H,6H-[1,2,4]triazolo[4,3-a][4,1]-benzoxazepin-4-yl]ethyl}-2H-1,2,3,4-tetrazol-5-yl)acetate
Ethyl 2-(2-{2-[(trans)-8-chloro-6-(2,3-dimethoxyphenyl)-1-neopentyl-4H,6H-[1,2,4]triazolo[4,3-a] [4,1]-benzoxazepin-4-yl]ethyl}-1H-1,2,3,4-tetrazol-5-yl)acetate

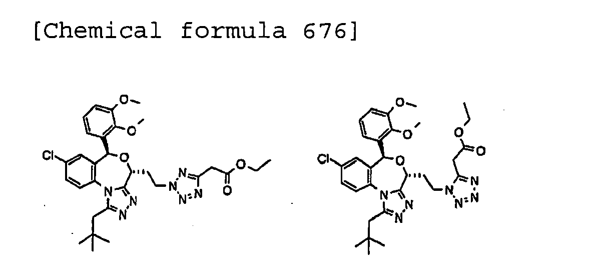

2-[(trans)-8-Chloro-6-(2,3-dimethoxyphenyl)-1-neopentyl-4H,6H-[1,2,4]triazolo[4,3-a][4,1]benzoxazepin-4-yl]ethyl methanesulfonate (63 mg) and ethyl 1H-tetrazole 5-acetate (36 mg) were dissolved in N,N-dimethylformamide (2 mL). To the resulting solution were added potassium carbonate (47 mg) and tetrabutylammonium iodide (42 mg).
The resulting mixture was stirred overnight at 80°C. The reaction mixture was diluted with chloroform and washed with saturated brine. The organic layer was dried over anhydrous sodium sulfate. After concentration of the filtrate, the residue thus obtained was purified by preparative TLC (methanol:chloroform=1:9) to give the title compounds 2H-isomer (28 mg) and 1H-isomer (20 mg).
Low polarity fraction (2H-isomer)
MS(ESI)m/z:610(M⁺+H).
¹H-NMR(CDCl₃)δ:0.94(9H,s),1.24(3H,t,J=7.1Hz),2.95-3.09(4H,m),3.50(3H,s),3.88(3H,s),3.88(2H,s),4.15-4.20(2H,m),4.41-4.43(1H,m),5.01-5.05(2H,m),5.66(1H,s),6.77-6.78(1H,m),7.00(1H,dd,J=7.9,1.6Hz),7.21-7.23(1H,m),7.26-7.28(1H,m),7.32-7.35(1H,m),7.48-7.49(1H,m).
High-polarity fraction (1H-isomer)
MS(ESI)m/z:610(M⁺+H).
¹H-NMR(CDCl₃)δ:0.94(9H,s),1.25-1.28(3H,m),2.93-3.02(4H,m),3.49(3H,s),3.88(3H,s),4.09-4.09(2H,m),4.16-4.19(2H,m),4.49-4.51(1H,m),4.74-4.76(1H,m),4.83-4.86(1H,m),5.66(1H,s),6.80(1H,d,J=2.4Hz),7.00(1H,dd,J=7.8,2 .0Hz),7.19-7.26(2H,m),7.33(1H,d,J=8.5Hz),7.49(1H,dd,J=8.5,2.3Hz).

### Example 309

2-(2-{2-[(trans)-8-Chloro-6-(2,3-dimethoxyphenyl)-1-neopentyl-4H,6H-[1,2,4]triazolo[4,3-a] [4,1]-benzoxazepin-4-yl]ethyl}-2H-1,2,3,4-tetrazol-5-yl)acetic acid

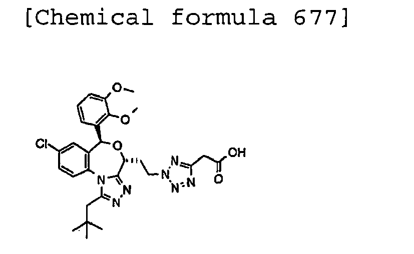

Ethyl 2-(2-{2-[(trans)-8-chloro-6-(2,3-dimethoxyphenyl)-1-neopentyl-4H,6H-[1,2,4]triazolo[4,3-a][4,1]-benzoxazepin-4-yl]ethyl}-2H-1,2,3,4-tetrazol-5-yl)acetate (28 mg) was dissolved in methanol-water-tetrahydrofuran (1:0.5:1, 2.5 mL). To the resulting solution was added potassium carbonate (19 mg). The resulting mixture was stirred at room temperature overnight and at 60°C for 2 hours. The reaction mixture was neutralized with an acidic resin (Amberlite IR-120B). The resin was then filtered out and the residue was washed with methanol. After concentration of the filtrate, the residue thus obtained was purified by preparative TLC (methanol:chloroform:water=3:7:1) to give the title compound (19 mg).
MS(ESI)m/z:582(M⁺+H).
¹H-NMR(CDCl₃)δ:0.86(9H,s),2.78-2.88(2H,m),2.93(1H,d,J=14.2Hz),2.99(1H,d,J=14.2Hz),3.46(3H, s),3.57-3.60(2H,m),3.84(3H,s),4.43-4.45(1H,m),4.85(2H,s),5.60(1H,s),6.72-6.72(1H,m),6.94(1H,d,J=8.3Hz),7.16(1H,t,J=7.9Hz),7.27(1H,s) ,7.38-7.44(2H,m).

### Example 310

2-(2-{2-[(trans)-8-Chloro-6-(2,3-dimethoxyphenyl)-1-neopentyl-4H,6H-[1,2,4]triazolo[4,3-a][4,1]-benzoxazepin-4-yl]ethyl}-1H-1,2,3,4-tetrazol-5-yl)acetic acid

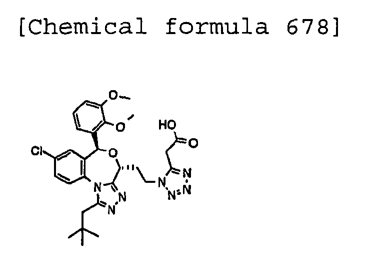

Ethyl 2-(2-{2-[(trans)-8-chloro-6-(2,3-dimethoxyphenyl)-1-neopentyl-4H,6H-[1,2,4]triazolo[4,3-a][4,1]-benzoxazepin-4-yl]ethyl}-1H-1,2,3,4-tetrazol-5-yl)acetate (20 mg) was dissolved in methanol-water-tetrahydrofuran (1:0.5:1, 2.5 mL). To the resulting solution was added potassium carbonate (14 mg). The reaction mixture was stirred overnight at room temperature. The reaction mixture was neutralized with an acidic resin (Amberlite IR-120B). The resin was then filtered out and the residue was washed with methanol. After concentration of the filtrate, the residue thus obtained was purified by preparative TLC (methanol:chloroform:water=3:7:1) to give the title compound (15 mg).
MS (ESI)m/z:582 (M⁺+H) .
¹H-NMR(CDCl₃) δ:0.79 (9H,s),2.45-2.66 (2H,m),2.87-2.97(2H,m),3.44(3H,s),3.74-3.76(2H,m),3.84(3H,s),4.48-4.57(3H,m),5.60(1H,s),6.71(1H,s),6.92(1H,d,J=8.1Hz),7.13-7.17(1H,m), 7.30-7.32(1H,m),7.39-7.42(1H,m),7.45-7.48(1H,m).

### Referential Example 196

3-[(trans)-8-Chloro-6-(2,3-dimethoxyphenyl)-1-neopentyl-4H,6H-[1,2,4]triazolo[4,3-a][4, 1] -benzoxazepin-4-yl]propanenitrile

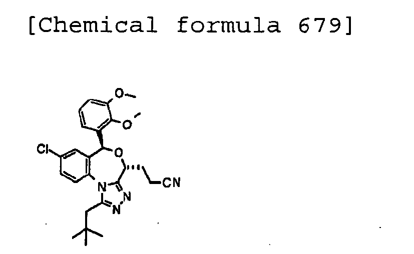

2-[(trans)-8-Chloro-6-(2,3-dimethoxyphenyl)-1-neopentyl-4H,6H-[1,2,4]triazolo[4,3-a] [4,1]benzoxazepin-4-yl]ethyl methanesulfonate (25 mg) was dissolved in dimethylsulfoxide (2 mL). To the resulting solution was added sodium cyanide (6.7 mg). The resulting mixture was stirred at 50°C for 4 days. After cooling to room temperature, the reaction mixture was poured into water, followed by extraction with ethyl acetate. The organic layer was dried over anhydrous sodium sulfate. After concentration of the filtrate, the residue thus obtained was purified by preparative TLC (methanol:chloroform=1:9) to give the title compound (22 mg).
MS(ESI)m/z:481(M⁺+H).
¹H-NMR(CDCl₃)δ:0.94(9H,s),2.62-2.70(2H,m),2.74-2.88(2H,m),2.96(1H,d,J=14.2Hz),3.05(1H,d,J=14.2Hz),3.49(3H, s),3.88(3H,s),4.53(1H,dd,J=7.2,5.5Hz),5.66(1H,s),6.81(1H,d, J=2.4Hz),7.00(1H,dd,J=7.8,1.7Hz),7.22(1H,t,J=7.8Hz),7.26-7.27(1H,m),7.35(1H,d,J=8.5Hz),7.51(1H,dd,J=8.5,2.4Hz).

### Example 311

3-[(trans)-8-Chloro-6-(2,3-dimethoxyphenyl)-1-neopentyl-4H,6H-[1,2,4]triazolo[4,3-a][4,1]-benzoxazepin-4-yl]propanoic acid

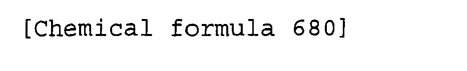

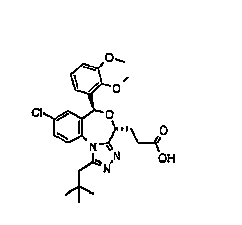

3-[(trans)-8-Chloro-6-(2,3-dimethoxyphenyl)-1-neopentyl-4H,6H-[1,2,4]triazolo[4,3-a][4,1]-benzoxazepin-4-yl]propanenitrile (22 mg) was dissolved in 2-propanol (1 mL). To the resulting solution were added a 5M aqueous solution (0.5 mL) of sodium hydroxide and methanol (0.5 mL). The resulting mixture was stirred overnight at 80°C. The reaction mixture was returned to room temperature, neutralized with 1N hydrochloric acid, and extracted with chloroform. The organic layer was dried over anhydrous sodium sulfate. After concentration of the filtrate, the residue thus obtained was purified by preparative TLC (methanol:chloroform=1:9) to give the title compound (18 mg).
MS (ESI) m/z : 500 (M⁺+H) .

### Example 312

Ethyl 2-(1-{3-[(trans)-8-chloro-6-(2,3-dimethoxyphenyl)-1-neopentyl-4H,6H-[1,2,4]triazolo[4,3-a][4,1]-benzoxazepin-4-yl]propanoyl}-4-piperidinyl)acetate

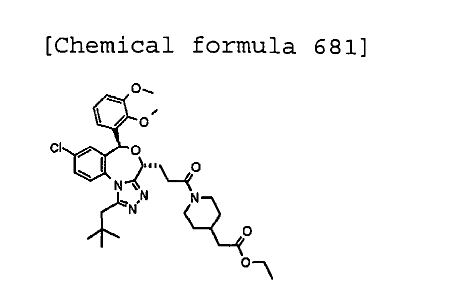

3-[(trans)-8-Chloro-6-(2,3-dimethoxyphenyl)-1-neopentyl-4H,6H-[1,2,4]triazolo[4,3-a][4,1]-benzoxazepin-4-yl]propanoic acid (18 mg) and ethyl 2-(4-piperidinyl)acetate (7.1 mg) were dissolved in dichloromethane (2 mL). To the resulting solution were added 1-ethyl-3-(3-dimethylaminopropyl)carbodiimide hydrochloride (8.6 mg) and 1-hydroxybenzotriazole (1.7 mg). The resulting mixture was stirred overnight at room temperature. The reaction mixture was diluted with chloroform and washed with saturated brine. The organic layer was dried over anhydrous sodium sulfate. After concentration of the filtrate, the residue thus obtained was purified by preparative TLC (methanol:chloroform=1:9) to give the title compound (13 mg).
MS(ESI)m/z:653(M⁺+H).
¹H-NMR(CDCl₃)δ:0.93(9H,s) 1.09-1.18(2H,m),1.26(3H,t,J=7.1Hz),1.70-1.75(4H,m),1.97-2.03(1H,m),2.50-2.57-(2H,m),2.63-2.77(3H,m),2.93-3.05(3H,m),3.48(3H,s),3.87(3H,s),3.98-4.01(1H,m),4.10-4.16(3H,m),4.48-4.50(1H,m),4.54-4.57(1H,m),5.62(1H,s),6.77(1H,d,J=2.2Hz),6.98(1H,dd,J=8.2,1 .3Hz),7.20(1H,t,J=8.2Hz),7.29-7.32(2H,m),7.47(1H,dd,J=8.4,2.3Hz).

### Example 313

Ethyl 2-(1-{3-[(trans)-8-chloro-6-(2,3-dimethoxyphenyl)-1-neopentyl-4H,6H-[1,2,4]triazolo[4,3-a][4,1]-benzoxazepin-4-yl]propanoyl}-4-piperidinyl)acetic acid

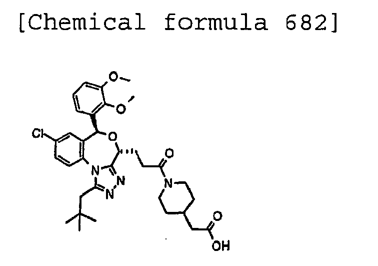

Ethyl 2-(1-{3-[(trans)-8-chloro-6-(2,3-dimethoxyphenyl)-1-neopentyl-4H,6H-[1,2,4]triazolo[4,3-a][4,1]-benzoxazepin-4-yl]propanoyl}-4-piperidinyl)acetate (13 mg) was dissolved in methanol-water-tetrahydrofuran (1:0.5:1, 2.5 mL). To the resulting solution was added potassium carbonate (8.8 mg). The resulting mixture was stirred at room temperature overnight and at 60°C for 3 days. The reaction mixture was neutralized with an acidic resin (Amberlite IR-120B). The resin was then filtered out and the residue was washed with methanol. After concentration of the filtrate, the residue thus obtained was purified by preparative TLC (methanol:chloroform=1:9) to give the title compound (6.3 mg).
MS (ESI) m/z:625 (M⁺+H).
¹H-NMR(CDCl₃)δ:0.92(9H,s),1.07-1.18(2H,m),1.70-1.83(2H,m),1.94-2.01(1H,m),2.20-2.24(2H,m),2.48-2.56(2H,m),2.60-2.71(3H,m),2.94-3.07(3H,m),3.48(3H,s),3.86(3H,s),3.94-3.97(1H,m),4.47-4.54(2H,m),5.61(1H,s),6.77-6.77(1H,m),6.97-7.00(1H,m),7.19(1H,t,J=8.1Hz),7.28-7.30(1H,m),7.33(1H,dd,J=8.4,3.1Hz),7.47(1H,dd,J=8.4,2.3Hz).

### Referential Example 197

3-[(trans)-8-Chloro-1-cyclopropyl-6-(2,3-dimethoxyphenyl)-4H,6H-[1,2,4]triazolo[4,3-a] [4,1]-benzoxazepin-4-yl]propanenitrile

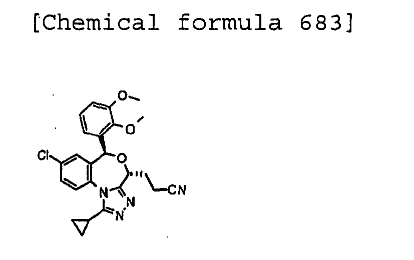

3-[(trans)-8-Chloro-1-cyclopropyl-6-(2,3-dimethoxyphenyl)-4H,6H-[1,2,4]triazolo[4,3-a][4,1]-benzoxazepin-4-yl]ethyl methanesulfonate (350 mg) was dissolved in dimethylsulfoxide (8 mL). To the resulting solution was added sodium cyanide (99 mg). The resulting mixture was stirred overnight at 50°C. After cooling to room temperature, the reaction mixture was poured into water, followed by extraction with ethyl acetate. The organic layer was dried over anhydrous sodium sulfate. After concentration of the filtrate, the residue thus obtained was purified by preparative TLC (methanol:chloroform=1:9) to give the title compound (321 mg).
MS(ESI)m/z:451(M⁺+H).
¹H-NMR(CDCl₃)δ:1.05-1.12(1H,m),1.19-1.25(2H,m),1.38-1.45(1H,m),1.94-2.00(1H,m),2.76-2.85(2H,m),3.49(3H,s),3.87(3H,s),4.58(1H,dd,J=7.1,5.6Hz),5. 64(1H,s),6.84(1H,d,J=2.4Hz),7.00(1H,dd,J=7.8,2.0Hz),7.22(1H ,t,J=7.8Hz),7.25(1H,d,J=1.7Hz),7.50(1H,dd,J=8.5,2.3Hz),7.58 (1H,d,J=8.5Hz).

### Example 314

3-[(trans)-8-Chloro-1-cyclopropyl-6-(2,3-dimethoxyphenyl)-4H,6H-[1,2,4]triazolo[4,3-a][4,1]-benzoxazepin-4-yl]propanoic acid

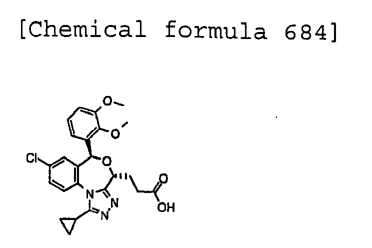

3-[(trans)-8-Chloro-1-cyclopropyl-6-(2,3-dimethoxyphenyl)-4H,6H-[1,2,4]triazolo[4,3-a][4,1]-benzoxazepin-4-yl]propanenitrile (304 mg) was dissolved in 2-propanol (6 mL). To the resulting solution were added a 5M aqueous solution (1 mL) of sodium hydroxide and methanol (2 mL). The resulting mixture was stirred overnight at 80°C. The reaction mixture was returned to room temperature, neutralized with 1N hydrochloric acid, and then extracted with chloroform. The organic layer was dried over anhydrous sodium sulfate. The filtrate was concentrated. The residue thus obtained was purified by preparative TLC (methanol:chloroform=1:9) to give the title compound (193 mg).
MS (ESI) m/z :470 (M⁺+H) .

### Referential Example 198

Ethyl 4-((3-[(trans)-8-chloro-1-cyclopropyl-6-(2,3-dimethoxyphenyl)-4H,6H-[1,2,4]triazolo[4,3-a][4,1]-benzoxazepin-4-yl]propanoyl)amino)-3-oxobutanoate

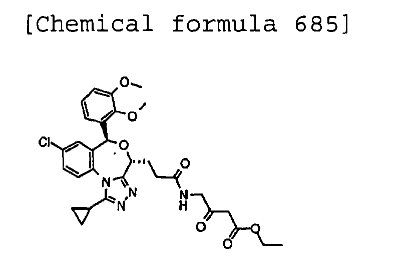

3-[(trans)-8-Chloro-1-cyclopropyl-6-(2,3-dimethoxyphenyl)-4H,6H-[1,2,4]triazolo[4,3-a][4,1]-benzoxazepin-4-yl]propanoic acid (99 mg) and ethyl 4-amino-3-oxobutanoate hydrochloride (42 mg) were dissolved in dichloromethane (2 mL). To the resulting solution were added 1-ethyl-3-(3-dimethylaminopropyl)carbodiimide hydrochloride (49 mg), 1-hydroxybenzotriazole (9.7 mg), and triethylamine (0.032 mL). The resulting mixture was stirred at room temperature for 2.5 hours. The reaction mixture was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (44 mg).
MS(ESI)m/z:597(M⁺+H)
¹H-NMR(CDCl₃)δ:1.05-1.08(1H,m),1.17-1.24(2H,m),1.27(3H,t,J=7.2Hz),1.36-1.42(1H,m),1.94-1.98(1H,m),2.55-2.64(4H,m),3.44(2H,s),3.49(3H,s),3.87(3H,s),4.16-4.21(4H,m),4.51-4.52(1H,m),5.60(1H,s),6.53-6.56(1H,m),6.81(1H,d,J=2.4Hz),6.98(1H,dd,J=7.9,1.7Hz),7.20( 1H,t,J=7.9Hz),7.25-7.26(1H,m),7.47(1H,dd,J=8.5,2.2Hz),7.56(1H,d,J=8.5Hz).

### Example 315

Ethyl 2-(2-{2-[(trans)-8-chloro-1-cyclopropyl-6-(2,3-dimethoxyphenyl)-4H,6H-[1,2,4]triazolo[4,3-a][4,1]benzoxazepin-4-yl]ethyl}-1,3-thiazol-5-yl)acetate

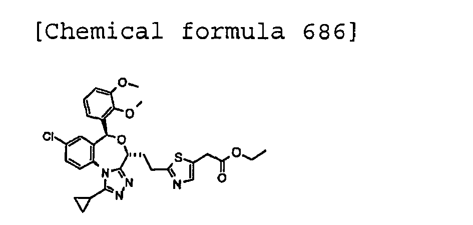

Ethyl 4-({3-[(trans)-8-chloro-1-cyclopropyl-6-(2,3-dimethoxyphenyl)-4H,6H-[1,2,4]triazolo[4,3-a][4,1]-benzoxazepin-4-yl]propanoyl}amino)-3-oxobutanoate (44 mg) was dissolved in tetrahydrofuran (3 mL). To the resulting solution was added Lawesson's reagent (44 mg). The resulting mixture was stirred at 70°C for one hour. The reaction mixture was cooled to room temperature and then concentrated. The residue thus obtained was purified by preparative TLC (methanol:chloroform=5:95) to give the title compound (31 mg).
MS(ESI)m/z:595(M⁺+H).
¹H-NMR(CDCl₃)δ:1.02-1.09(1H,m),1.16-1.24(2H,m),1.26(3H,t,J=7.1Hz),1.36-1.43(1H,m),1.93-1.99(1H,m),2.73-2.79(2H,m),3.25-3.41(2H,m),3.49(3H,s),3.76(2H,d,J=1.0Hz),3.87(3H,s),4.17(2H ,q,J=7.1Hz),4.51(1H,dd,J=7.1,5.9Hz),5.63(1H,s),6.81(1H,d,J= 2.2Hz),6.98(1H,dd,J=7.9,1.5Hz),7.20(1H,t,J=7.9Hz),7.29(1H,d d,J=7.9,1.0Hz),7.41(1H,s),7.46(1H,dd,J=8.4,2.3Hz),7.56(1H,d ,J=8.4Hz).

### Example 316

2-(2{2-[(trans)-8-Chloro-1-cyclopropyl-6-(2,3-dimethoxyphenyl)-4H,6H-[1,2,4]triazolo[4,3-a][4,1]benzoxazepin-4-yl]ethyl}-1,3-thiazol-5-yl)acetic acid

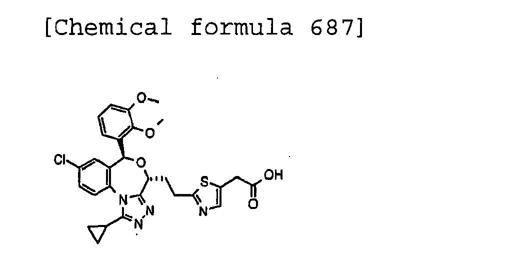

Ethyl 2-(2-{2-[(trans)-8-chloro-1-cyclopropyl-6-(2,3-dimethoxyphenyl)-4H,6H-[1,2,4]triazolo[4,3-a][4,1]-benzoxazepin-4-yl]ethyl}-1,3-thiazol-5-yl)acetate (31 mg) was dissolved in methanol-water-tetrahydrofuran (1:0.5:1, 2.5 mL). To the resulting solution was added lithium hydroxide monohydrate (6.6 mg). The resulting mixture was stirred at 50°C for 6 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform:water=3:7:1) to give the title compound (31 mg).
MS (ESI) m/z : 567 (M⁺+H).
¹H-NMR(CDCl₃)δ:0.98-1.01(1H,m),1.11-1.19(2H,m),1.23-1.28(1H,m),1.89-1.96(1H,m),2.58-2.65(2H,m),3.04-3.12(1H,m),3.18-3.25(1H,m),3.44(3H,s),3.58(2H,s),3.84(3H,s),4.44(1H,t,J=6.6 Hz),5.56(1H,s),6.78(1H,d,J=2.2Hz),6.93(1H,dd,J=8.3,1.2Hz),7 .14(1H,t,J=8.3Hz),7.22-7.25(2H,m),7.43(1H,dd,J=8.5,2.3Hz),7.55(1H,d,J=8.5Hz).

### Referential Example 199

Methyl 4-({3-[(trans)-8-chloro-1-cyclopropyl-6-(2,3-dimethoxyphenyl)-4H,6H-[1,2,4]triazolo[4,3-a][4,1]-benzoxazepin-4-yl]propanoyl}amino)-4-oxopentanoate

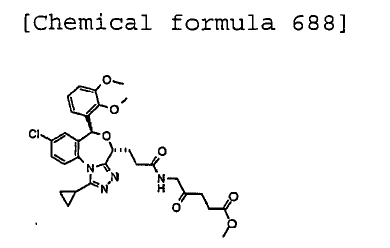

3-[(trans)-8-Chloro-1-cyclopropyl-6-(2,3-dimethoxyphenyl)-4H,6H-[1,2,4]triazolo[4,3-a][4,1]-benzoxazepin-4-yl]propanoic acid (47 mg) and methyl 5-aminolevulinate hydrochloride (22 mg) were dissolved in dichloromethane (2 mL). To the resulting solution were added 1-ethyl-3-(3-dimethylaminopropyl)carbodiimide hydrochloride (23 mg), 1-hydroxybenzotriazole (4.6 mg), and triethylamine (0.015 mL). The resulting mixture was stirred at room temperature for 3 hours. The reaction mixture was diluted with chloroform, washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (53 mg).
MS(ESI)m/z:597(M⁺+H).
¹H-NMR(CDCl₃)δ:1.02-1.10(1H,m),1.19-1.26(2H,m),1.37-1.40(1H,m),1.94-1.98(1H,m),2.55-2.71(8H,m),3.49(3H,s),3.67(3H,s),3.86(3H,s),4.12-4.13(2H,m),4.50-4.53(1H,m),5.60(1H,s),6.47-6.50(1H,m),6.81(1H,d,J=2.2Hz),6.97(1H,dd,J=7.9,1.5Hz),7.20( 1H,t,J=7.9Hz),7.25-7.27(1H,m),7.46(1H,dd,J=8.5,2.3Hz),7.56(1H,d,J=8.5Hz).

### Example 317

Methyl 2-(2-{2-[(trans)-8-chloro-1-cyclopropyl-6-(2,3-dimethoxyphenyl)-4H,6H-[1,2,4]triazolo[4,3-a][4,1]benzoxazepin-4-yl]ethyl}-1,3-thiazol-5-yl)propionate

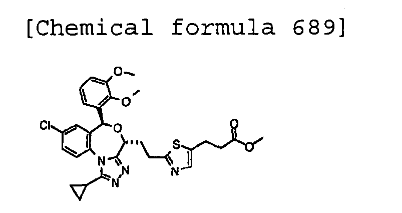

Methyl 4-({3-[(trans)-8-chloro-1-cyclopropyl-6-(2,3-dimethoxyphenyl)-4H,6H-[1,2,4]triazolo[4,3-a][4,1]-benzoxazepin-4-yl]propanoyl}amino)-4-oxopentanoate (53 mg) was dissolved in tetrahydrofuran (2 mL). To the resulting solution was added Lawesson's reagent (54 mg). The resulting mixture was stirred at 70°C for one hour. The reaction mixture was cooled to room temperature and then concentrated. The residue was purified by preparative TLC (methanol:chloroform=5:95) to give the title compound (51 mg).
¹H-NMR(CDCl₃)δ:1.04-1.07(1H,m) 1.18-1.26 (2H,m),1.36-1.42(1H,m),1.94-1.98(1H,m),2.60-2.64(2H,m),2.71-2.77(2H,m),3.06-3.10(2H,m),3.22-3.38(2H,m),3.49(3H,s),3.68(3H,s),3.87(3H,s),4.49(1H,t,J=6.6 Hz),5.62(1H,s),6.81(1H,d,J=2.4Hz),6.98(1H,dd,J=7.9,1.5Hz),7 .20(1H,t,J=7.9Hz),7.27-7.29(1H,m),7.31-7.31(1H,m),7.47(1H,dd,J=8.3,2.4Hz),7.56(1H,d,J=8.3Hz).

### Example 318

Methyl 2-(2-{2-((trans)-8-chloro-1-cyclopropyl-6-(2,3-dimethoxyphenyl)-4H,6H-[1,2,4]triazolo[4,3-a][4,1]benzoxazepin-4-yl]ethyl}-1,3-thiazol-5-yl) propanoate

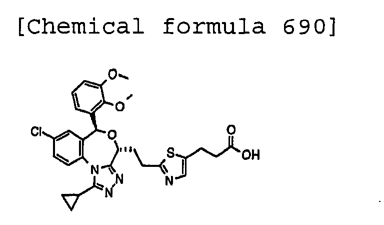

Methyl 2-(2-{2-[(trans)-8-chloro-1-cyclopropyl-6-(2,3-dimethoxyphenyl)-4H,6H-[1,2,4]triazolo[4,3-a][4,1]benzoxazepin-4-yl]ethyl}-1,3-thiazol-5-yl)propionate(51 mg) was dissolved in methanol-water-tetrahydrofuran (1:0.5:1, 2.5 mL). To the resulting solution was added lithium hydroxide monohydrate (11 mg). The resulting mixture was stirred at 50°C for 8 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform:water=3:7:1) to give the title compound (23 mg).
MS(ESI)m/z:581(M⁺+H).
¹H-NMR(CDCl₃)δ:1.01-1.08(1H,m),1.18-1.24(2H,m),1.32-1.39(1H,m),1.93-1.97(1H,m),2.56-2.59(2H,m),2.67-2.72(2H,m),3.00-3.04(2H,m),3.16-3.24(1H,m),3.27-3.34(1H,m),3.48(3H,s),3.86(3H,s),4.48(1H,t,J=6.6Hz),5.60(1H ,s),6.81(1H,d,J=2.4Hz),6.96(1H,dd,J=7.9,1.2Hz),7.18(1H,t,J= 7.9Hz),7.26-7.28(1H,m),7.29(1H,s),7.46(1H,dd,J=8.3,2.4Hz),7.56(1H,d,J=8 .3Hz).

### Referential Example 200

2-[(trans)-7-Chloro-1-(2,4-dimethoxybenzyl)-5-(2,3-dimethoxyphenyl)-2-oxo-1,2,3,5-tetrahydro-4,1-benzoxazepin-3-yl]acetic acid

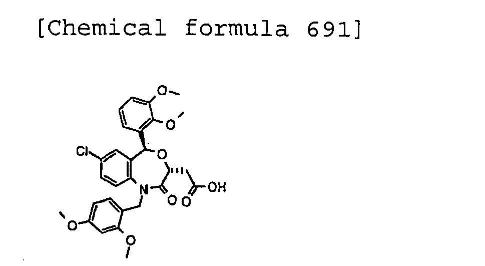

Ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate (859 mg) was dissolved in methanol-water-tetrahydrofuran (8:4:8, 20 mL). To the resulting solution was added lithium hydroxide monohydrate (190 mg). The resulting mixture was stirred at room temperature for 5.5 hours. The reaction mixture was made acidic with 1N hydrochloric acid, extracted with chloroform, and washed with saturated brine. The organic layer was dried over anhydrous sodium sulfate. The filtrate was then concentrated to give the title compound (958 mg).
MS(ESI)m/z:542(M⁺+H).

### Referential Example 201

Methyl 5-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetyl}amino)-4-oxopentanoate

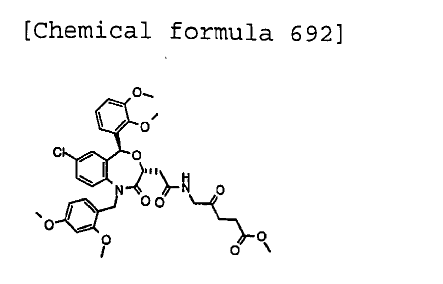

2-[(trans)-7-Chloro-1-(2,4-dimethoxybenzyl)-5-(2,3-dimethoxyphenyl)-2-oxo-1,2,3,5-tetrahydro-4,1-benzoxazepin-3-yl]acetic acid (300 mg) and methyl 5-aminolevulinate hydrochloride (121 mg) were dissolved in dichloromethane (6 mL). To the resulting solution were added 1-ethyl-3-(3-dimethylaminopropyl)carbodiimide hydrochloride (127 mg), 1-hydroxybenzotriazole (25 mg), and triethylamine (0.085 mL). The resulting mixture was stirred at room temperature for 17 hours. The reaction mixture was diluted with chloroform, washed with a saturated aqueous solution of sodium bicarbonate and saturated brine. The organic layer was dried over anhydrous sodium sulfate. After concentration of the filtrate, the residue was purified by preparative TLC (methanol:chloroform=1:9) to give the title compound (302 mg).
MS(ESI)m/z:669(M⁺+H).

### Referential Example 202

Methyl 3-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)propanoate

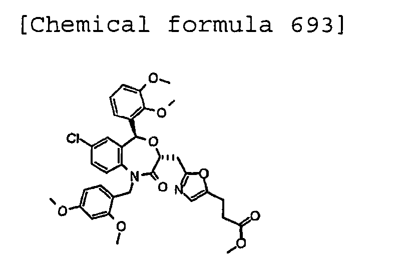

Methyl 5-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetyl}amino)-4-oxopentanoate (471 mg) was dissolved in N,N-dimethylformamide (3 mL). To the resulting solution was added phosphorus oxychloride (0.098 mL). The resulting mixture was stirred at 70°C for one hour. After cooling the reaction mixture to room temperature, a saturated aqueous solution of sodium bicarbonate was added thereto at 0°C. The resulting mixture was extracted with ethyl acetate, followed by washing with saturated brine. The organic layer was dried over anhydrous sodium sulfate. After concentration of the filtrate, the residue was purified by preparative TLC (ethyl acetate:hexane=1:1) to give the title compound (462 mg).
MS(ESI)m/z:651(M⁺+H).
¹H-NMR(CDCl₃)δ:2.64(2H,t,J=7.4Hz),2.81(1H,t,J=7.3Hz),2.96(2H,t ,J=7.4Hz),3.10(1H,t,J=7.3Hz),3.20(3H,s),3.24(1H,dd,J=15.7,7 .2Hz),3.39(1H,dd,J=15.7,6.5Hz),3.67(3H,s),3.68(3H,s),3.76(3 H,s),3.85(3H,s),4.52-4.54(1H,m),4.85(1H,d,J=15.1Hz),5.49(1H,d,J=15.1Hz),6.00(1H, s),6.38-6.40(2H,m),6.51(1H,d,J=2.2Hz),6.66-6.66(1H,m),6.92(1H,dd,J=8.3,1.5Hz),6.99-7.01(1H,m),7.12(1H,t,J=8.1Hz),7.23-7.32(3H,m).

### Referential Example 203

Methyl 3-({2-[(trans)-7-chloro-5-(2,3-dimethoxyphenyl)-2-oxo-1,2,3,5-tetrahydro-4,1-benzoxazepin-3-yl]methyl}-1,3-oxazol-5-yl)propanoate

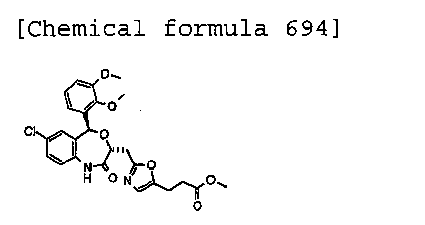

Methyl 3-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)propanoate (462 mg) was dissolved in acetone (12 mL). To the resulting solution was added an aqueous solution (3 mL) of ammonium dicerium(IV) nitrate (1167 mg) at 0°C. The resulting mixture was stirred at room temperature for one hour. To the reaction mixture was added a saturated aqueous solution of sodium bicarbonate. The resulting mixture was extracted with ethyl acetate. The extract was washed with saturated brine. The organic layer was dried over anhydrous sodium sulfate. After concentration of the filtrate, the residue was purified by preparative TLC (ethyl acetate:hexane=2:1) to give the title compound (240 mg).
MS(ESI)m/z:501(M⁺+H).
¹H-NMR(CDCl₃)δ:2.61(2H,dd,J=8.1,7.1Hz),2.91(2H,dd,J=8.1,7.1Hz) ,3.21(1H,dd,J=15.6,7.6Hz),3.39(1H,dd,J=15.6,5.9Hz),3.62(3H, s),3.69(3H,s),3.89(3H,s),4.68(1H,dd,J=7.6,5.9Hz),6.23(1H,s) ,6.62-6.62(1H,m),6.66(1H,d,J=2.2Hz),6.92-6.99(3H,m),7.12(1H,t,J=8.1Hz),7.27-7.28(1H,m),7.76-7.79(1H,m).

### Referential Example 204

Methyl 3-({2-[(trans)-7-chloro-5-(2,3-dimethoxyphenyl)-2-thioxo-1,2,3,5-tetrahydro-4,1-benzoxazepin-3-yl]methyl}-1,3-oxazol-5-yl)propanoate
Methyl 3-({2-[(cis)-7-chloro-5-(2,3-dimethoxyphenyl)-2-thioxo-1,2,3,5-tetrahydro-4,1-benzoxazepin-3-yl]methyl}-1,3-oxazol-5-yl)propanoate

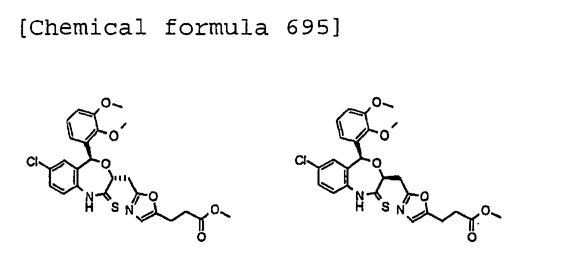

Methyl 3-({2-[(trans)-7-chloro-5-(2,3-dimethoxyphenyl)-2-oxo-1,2,3,5-tetrahydro-4,1-benzoxazepin-3-yl]methyl}-1,3-oxazol-5-yl)propanoate (240 mg) was dissolved in toluene (5 mL). To the resulting solution was added Lawesson's reagent (213 mg). The resulting mixture was stirred at 70°C for one hour. The reaction mixture was cooled to room temperature and then concentrated. The residue was purified by preparative TLC (ethyl acetate:hexane=1:1) to give the trans isomer (86 mg) and cis isomer (100 mg). trans Isomer
MS(ESI)m/z:517(M⁺+H).
¹H-NMR(CDCl₃)δ:2.63-2.65(2H,m),2.94-2.96(2H,m),3.42(1H,dd,J=15.6,7.8Hz),3.54(1H,dd,J=15.6,5.6Hz ),3.61(3H,s),3.69(3H,s),3.88(3H,s),4.73(1H,dd,J=7.8,5.6Hz), 6.15(1H,s),6.62(1H,d,J=2.4Hz),6.64-6.66(1H,m),6.91-6.96(2H,m),7.06(1H,d,J=8.5Hz),7.12(1H,t,J=8.1Hz),7.32(1H,dd ,J=8.4,2.3Hz),9.65(1H,br s).
cis Isomer
MS(ESI)m/z:517(M⁺+H).
¹H-NMR(CDCl₃)δ:2.57-2.59(2H,m),2.91-2.93(2H,m),3.37(1H,dd,J=16.2,9.4Hz),3.63(1H,dd,J=16.2,3.9Hz ),3.69(3H,s),3.71(3H,s),3.86(3H,s),5.19(1H,dd,J=9.3,3.9Hz), 6.33(1H,s),6.66-6.68(1H,m),6.79(1H,dd,J=7.8,1.5Hz),6.86-6.95(3H,m),7.01(1H,t,J=8.1Hz),7.18(1H,dd,J=8.5,2.4Hz),9.51( 1H,br s).

### Referential Example 205

Methyl 3-({2-[(trans)-7-chloro-5-(2,3-dimethoxyphenyl)-2-[hydrazono]-1,5-dihydro-4,1-benzoxazepin3(3H)-yl]methyl}-1,3-oxazol-5-yl)propanoate

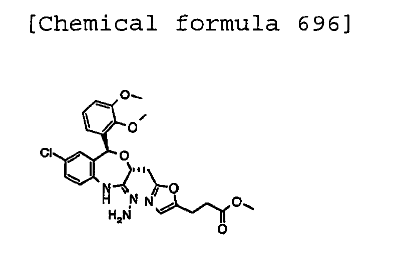

Methyl 3-({2-[(trans)-7-chloro-5-(2,3-dimethoxyphenyl)-2-thioxo-1,2,3,5-tetrahydro-4,1-benzoxazepin-3-yl]methyl}-1,3-oxazol-5-yl)propanoate (86 mg) was dissolved in ethanol (3 mL). To the resulting solution was added hydrazine monohydrate (0.0404 mL). The resulting mixture was stirred at room temperature for 21 hours. The reaction mixture was concentrated to give the title compound (86 mg).
MS(ESI)m/z:515(M⁺+H).

### Example 319

Methyl 3-({2-[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)propanoate

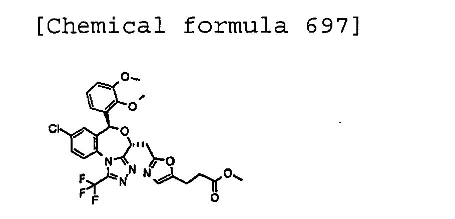

Methyl 3-({2-[(trans)-7-chloro-5-(2,3-dimethoxyphenyl)-2-[hydrazono]-1,5-dihydro-4,1-benzoxazepin3(3H)-yl]methyl}-1,3-oxazol-5-yl)propanoate (86 mg) was dissolved in dichloromethane (3 mL). To the resulting solution were added trifluoroacetic anhydride (0.066 mL) and trifluoroacetic acid (0.257 mL). The resulting mixture was stirred at room temperature for one hour. The reaction mixture was stirred at 55°C to distill off dichloromethane. Toluene (3 mL) was added to the residue and the resulting mixture was heated under reflux for 3 hours. After cooling to room temperature, the reaction mixture was concentrated. The residue was diluted with chloroform, and washed with a saturated aqueous solution of sodium bicarbonate. The organic layer was dried over anhydrous sodium sulfate. After concentration of the filtrate, the residue thus obtained was purified by preparative TLC (ethyl acetate:hexane=1:1) to give the title compound (58 mg).
MS(ESI)m/z:593(M⁺+H).
¹H-NMR(CDCl₃)δ:2.66(2H,t,J=7.6Hz),2.98(2H,t,J=7.6Hz),3.43(3H,s ),3.65(1H,dd,J=15.9,7.3Hz),3.69(3H,s),3.86(3H,s),3.87(1H,dd ,J=15.9,6.3Hz),4.99(1H,dd,J=7.3,6.3Hz),5.57(1H,s),6.67(1H,s ),6.82-6.84(1H,m),6.97(1H,dd,J=8.1,1.5Hz),7.07-7.09(1H,m),7.16(1H,t,J=8.1Hz),7.51-7.53(2H,m).

### Example 320

3-({2-[(trans)-8-Chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)propanoic acid

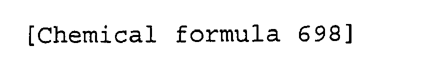

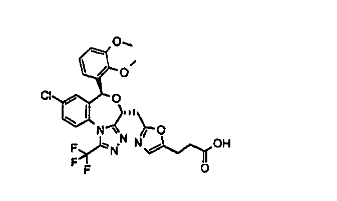

Methyl 3-({2-[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)propanoate (58 mg) was dissolved in methanol-water-tetrahydrofuran (2:1:2, 5 mL). To the resulting solution was added potassium carbonate (40 mg). The resulting mixture was stirred at room temperature for 5 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform=1:9) to give the title compound (50 mg).
MS (ESI) m/z : 579 (M⁺+H) .
¹H-NMR(CDCl₃)δ:2.62(2H,t,J=7.3Hz),2.93(2H,t,J=7.3Hz),3.43(3H,s ),3.65(1H,dd,J=15.9,7.3Hz),3.85(3H,s),3.87(1H,dd,J=15.9,6.6 Hz),4.97-4.99(1H,m),5.57(1H,s),6.67(1H,s),6.82-6.84(1H,m),6.96(1H,dd,J=8.1,1.5Hz),7.08-7.10(1H,m),7.16(1H,t,J=7.9Hz),7.51-7.52(2H,m).

### Referential Example 206

Ethyl 2-[(trans)-7-chloro-5-(2,3-dimethoxyphenyl)-2-oxo-1,2,3,5-tetrahydro-4,1-benzoxazepin-3-yl]acetate

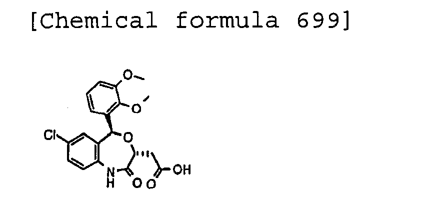

Ethyl 2-[(trans)-7-chloro-5-(2,3-dimethoxyphenyl)-2-oxo-1,2,3,5-tetrahydro-4,1-benzoxazepin-3-yl]acetate (1.06 g) was dissolved in methanol-water-tetrahydrofuran (10:5:10, 25 mL). To the resulting solution was added lithium hydroxide monohydrate (212 mg). The resulting mixture was stirred at room temperature for 4 hours. The reaction mixture was acidified with 1N hydrochloric acid and then, extracted with chloroform. The extract was washed with saturated brine. The organic layer was dried over anhydrous sodium sulfate. The filtrate was then concentrated to give the title compound (1.10 g).
MS (ESI)m/z:392 (M⁺+H).

### Referential Example 207

Methyl 5-({2-[(trans)-7-chloro-5-(2,3-dimethoxyphenyl)-2-oxo-1,2,3,5-tetrahydro-4,1-benzoxazepin-3-yl]acetyl}amino)-4-oxopentanoate

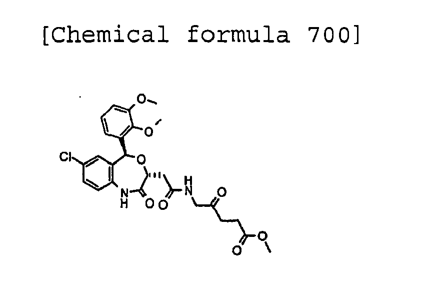

Ethyl 2-[(trans)-7-chloro-5.-(2,3-dimethoxyphenyl)-2-oxo-1,2,3,5-tetrahydro-4,1-benzoxazepin-3-yl]acetate (1096 mg) and methyl 5-aminolevulinate hydrochloride (610 mg) were dissolved in dichloromethane (20 mL). To the resulting solution were added 1-ethyl-3-(3-dimethylaminopropyl)carbodiimide hydrochloride (643 mg), 1-hydroxybenzotriazole (129 mg), and triethylamine (0.429 mL). The resulting mixture was stirred at room temperature for 19 hours. The reaction mixture was diluted with chloroform and washed with a saturated aqueous solution of sodium bicarbonate and saturated brine. The organic layer was dried over anhydrous sodium sulfate. After concentration of the filtrate, the residue was purified by silica gel column (methanol:chloroform=1:10) to give the title compound (1140 mg).
MS(ESI)m/z:519(M⁺+H).

### Referential Example 208

Methyl 3-({2-[(trans)-7-chloro-5-(2,3-dimethoxyphenyl)-2-thioxo-1,2,3,5-tetrahydro-4,1-benzoxazepin-3-yl]methyl}-1,3-thiazol-5-yl)propanoate
Methyl 3-({2-[(cis)-7-chloro-5-(2,3-dimethoxyphenyl)-2-thioxo-1,2,3,5-tetrahydro-4,1-benzoxazepin-3-yl]methyl}-1,3-thiazol-5-yl)propanoate

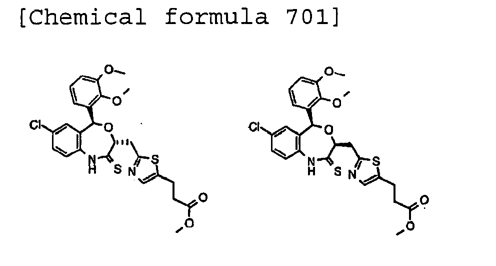

Methyl 5-({2-[(trans)-7-chloro-5-(2,3-dimethoxyphenyl)-2-oxo-1,2,3,5-tetrahydro-4,1-benzoxazepin-3-yl]acetyl}amino)-4-oxopentanoate (1140 mg) was dissolved in tetrahydrofuran (30 mL). To the resulting solution was added Lawesson's reagent (977 mg). The resulting mixture was stirred at 70°C for one hour. The reaction mixture was cooled to room temperature and then concentrated. The residue was purified twice by silica gel column (methanol:chloroform=1:10, ethyl acetate:hexane=1:1) to give the trans isomer (138 mg) and cis isomer (172 mg). trans Isomer
MS(ESI)m/z:533(M⁺+H).
¹H-NMR(CDCl₃)δ:2.67(2H,t,J=7.3Hz),3.12(2H,t,J=7.3Hz),3.60(3H,s ),3.63(1H,dd,J=15.1,7.3Hz),3.70(3H,s),3.78(1H,dd,J=15.1,5.1 Hz),3.88(3H,s),4.64(1H,dd,J=7.3,5.1Hz),6.15(1H,s),6.61(1H,d ,J=2.4Hz),6.96-6.98(2H,m),7.03(1H,d,J=8.3Hz),7.13(1H,t,J=7.9Hz),7.30(1H,dd ,J=8.5,2.4Hz),7.39(1H,s),9.64(1H,s).
cis Isomer
MS(ESI)m/z:533(M⁺+H).
¹H-NMR(CDCl₃)δ:2.61(2H,t,J=7.3Hz),3.08(2H,t,J=7.3Hz),3.59(1H,d d,J=15.6,8.8Hz),3.69(6H,s),3.85(1H,dd,J=15.6,3.7Hz),3.86(3H ,s),5.15(1H,dd,J=8.8,3.7Hz),6.22(1H,s),6.84-6.91(4H,m),7.04(1H,t,J=7.9Hz),7.17(1H,dd,J=8.5,2.4Hz),7.39( 1H,s),9.50(1H,s).

### Referential Example 209

Methyl 3-({2-[(trans)-7-chloro-5-(2,3-dimethoxyphenyl)-2-[hydrazono]-1,5-dihydro-4,1-benzoxazepin3(3H)-yl]methyl}-1,3-thiazol-5-yl)propanoate

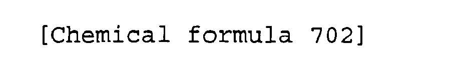

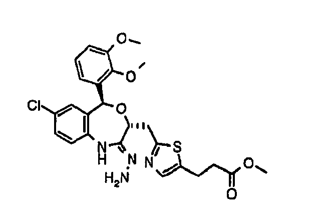

Methyl 3-({2-[(trans)-7-chloro-5-(2,3-dimethoxyphenyl)-2-thioxo-1,2,3,5-tetrahydro-4,1-benzoxazepin-3-yl]methyl}-1,3-thiazol-5-yl)propanoate (138 mg) was dissolved in ethanol (3 mL). To the resulting solution was added hydrazine monohydrate (0.0628 mL). The resulting mixture was stirred at room temperature for 19 hours. The reaction mixture was concentrated to give the title compound (137 mg).
MS(ESI)m/z:531(M+H)⁺.

### Example 321

Methyl 3-({2-[(trans)-8-chloro-6-(2,3-dimethoxyphenyl)-1-(trifluoromethyl)-4H,6H-[1,2,4]triazolo[4,3-a] [4,1]benzoxazepin-4-yl]methyl}-1,3-thiazol-5-yl)propanoate

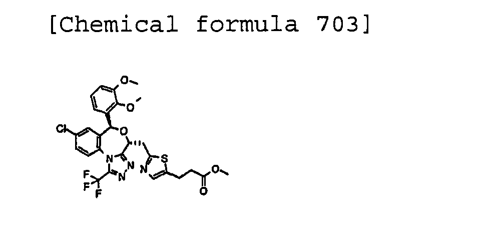

Methyl 3-({2-[(trans)-7-chloro-5-(2,3-dimethoxyphenyl)-2-[hydrazono]-1,5-dihydro-4,1-benzoxazepin3(3H)-yl]methyl}-1,3-thiazol-5-yl)propanoate (137 mg) was dissolved in dichloromethane (4 mL). To the resulting solution were added trifluoroacetic anhydride (0.102 mL) and trifluoroacetic acid (0.398 mL). The resulting mixture was stirred at room temperature for one hour. After the reaction mixture was stirred at 55°C and dichloromethane was distilled off, toluene (4 mL) was added to the residue. The resulting mixture was heated under reflux for 2.5 hours. The reaction mixture was cooled to room temperature and then concentrated. The residue was diluted with chloroform and washed with a saturated aqueous solution of sodium bicarbonate. The organic layer was dried over anhydrous sodium sulfate. After concentration of the filtrate, the residue thus obtained was purified by preparative TLC (ethyl acetate:hexane=2:3) to give the title compound (50 mg).
MS (ESI)m/z:609 (M⁺+H) .
¹H-NMR(CDCl₃)δ:2.67(2H,t,J=7.3Hz), ,3.13(2H,t,J=7.3Hz),3.43(3H,s ),3.70(3H,s),3.85(3H,s),3.86(1H,dd,J=15.1,7.6Hz),4.06(1H,dd ,J=15.1,5.6Hz),4.89(1H,dd,J=7:6,5.6Hz),5.57(1H,s),6.81-6.82(1H,m),6.98(1H,dd,J=7.8,1.7Hz),7.13(1H,dd,J=7.8,1.5Hz), 7.18(1H,t,J=7.8Hz),7.37(1H,s),7.50-7.50(2H,m).

### Example 322

3-({2-[(trans)-8-Chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-5-yl)propanoic acid

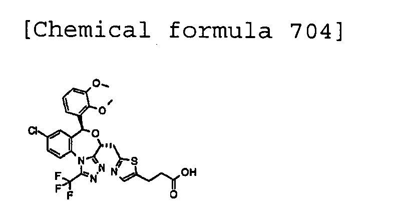

Methyl 3-({2-[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-5-yl)propanoate (50 mg) was dissolved in methanol-water-tetrahydrofuran (2:1:2, 5 mL). To the resulting solution was added potassium carbonate (34 mg). The resulting mixture was stirred at room temperature for 14 hours and then at 50°C for 3 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform=1:9) to give the title compound (38 mg).
MS(ESI)m/z:595(M⁺+H).
¹H-NMR(CDCl₃)δ:2.67(2H,t,J=7.2Hz),3.10(2H,t,J=7.2Hz),3.42(3H,s ),3.85(1H,dd,J=15.3,7.2Hz),3.86(3H,s),4.06(1H,dd,J=15.3,6.0 Hz),4.90(1H,dd,J=7.2,6.0Hz),5.57(1H,s),6.80-6.83(1H,m),6.97(1H,dd,J=7.8,1.7Hz),7.12-7.19(2H,m),7.40(1H,s),7.49-7.51(2H,m).

### Referential Example 210

2-{[(2-Amino-5-chlorophenyl)(2,3-dimethoxyphenyl)methyl]sulfanyl}succinic acid

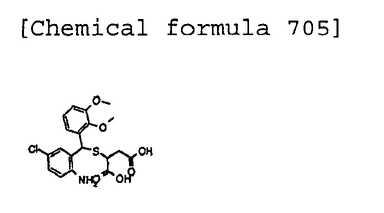

(2-Amino-5-chlorophenyl)(2,3-dimethoxyphenyl)methanol (7.32 g) and thiomalic acid (3.82 g) were dissolved in a solvent mixture of concentrated hydrochloric acid (70 mL) and acetic acid (70 mL). The resulting solution was stirred at 100°C for 2 hours. After the reaction mixture was cooled, a 1N aqueous sodium hydroxide solution (350 mL) was added thereto. The resulting mixture was extracted with dichloromethane. The extract was washed with saturated brine. The organic layer was dried over anhydrous magnesium sulfate. The filtrate was then concentrated to give the title compound (9.41 g).
MS (ESI) m/z: 426 (M⁺+H).

### Referential Example 211

2-[7-Chloro-5-(2,3-dimethoxyphenyl)-2-oxo-1,2,3,5-tetrahydro-4,1-benzothiazepin-3-yl]acetic acid

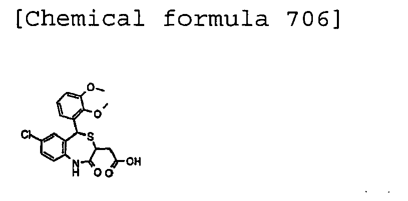

2-{[(2-Amino-5-chlorophenyl)(2,3-dimethoxyphenyl)methyl]sulfanyl}succinic acid (9.41 g) was dissolved in a solvent mixture of xylene (200 mL) and N,N-dimethylformamide (6 mL). The resulting mixture was heated under reflux for 18 hours. The reaction mixture was cooled to room temperature and then concentrated to give the title compound (9.01 g).
MS(ESI)m/z:408(M⁺+H).

### Referential Example 212

Methyl 2-[(trans)-7-chloro-5-(2,3-dimethoxyphenyl)-2-oxo-1,2,3,5-tetrahydro-4,1-benzothiazepin-3-yl]acetate methyl 2-[(cis)-7-chloro-5-(2,3-dimethoxyphenyl)-2-oxo-1,2,3,5-tetrahydro-4,1-benzothiazepin-3-yl]acetate

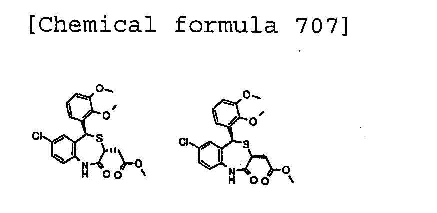

2-[7-Chloro-5-(2,3-dimethoxyphenyl)-2-oxo-1,2,3,5-tetrahydro-4,1-benzothiazepin-3-yl]acetic acid (9.01 g) was dissolved in methanol (100 mL). To the resulting solution was added concentrated sulfuric acid (1 mL). The resulting mixture was heated under reflux for 3 hours. After cooling the reaction mixture to room temperature, the solvent was distilled off partially. The residue was diluted with chloroform and washed with a saturated aqueous solution of sodium bicarbonate and saturated brine. The organic layer was dried over anhydrous sodium sulfate. After concentration of the filtrate, the residue thus obtained was recrystallized from a dichloromethane-petroleum ether solvent mixture to give the trans isomer (4.51 g). The residue obtained by concentrating the base solution was purified by silica gel column (ethyl acetate:hexane=1:2) to give the trans isomer (0.88 g) and cis isomer (0.52 g). trans Isomer
MS(ESI)m/z:422(M⁺+H).
¹H-NMR(CDCl₃)δ:2.45(1H,dd,J=17.1,3.9Hz),3.12(1H,dd,J=17.0,10.4 Hz),3.60(3H,s),3.68(3H,s),3.83(1H,dd,J=10.3,3.9Hz),3.88(3H, s),6.20(lH,s),6.85(1H,d,J=2.2Hz),6.97(1H,dd,J=8.3,1.3Hz),7. 09(1H,d,J=8.3Hz),7.18(1H,t,J=7.9Hz),7.25(1H,dd,J=8.3,2.2Hz) ,7.31(1H,dd,J=7.9,1.3Hz).
cis Isomer
MS(ESI)m/z:422(M⁺+H).
¹H-NMR(CDCl₃)δ:2.47(1H,dd,J=17.1,4.9Hz),3.06(1H,dd,J=17.1,9.6H z),3.49(3H,s),3.65(3H,s),3.79(3H,s),3.84-3.88(1H,m),5.51(1H,s),6.82-6.84(1H,m),6.92-6.94(1H,m),7.04(1H,t,J=8.1Hz),7.25-7.27(1H,m),7.42-7.44(1H,m),7.52(1H,d,J=2.2Hz).

### Referential Example 213

Methyl 2-[(trans)-7-chloro-5-(2,3-dimethoxyphenyl)-2-oxo-1,2,3,5-tetrahydro-4,1-benzothiazepin-3-yl]acetate

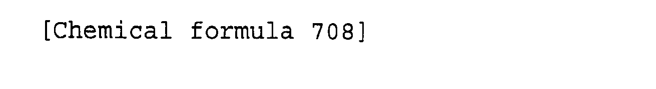

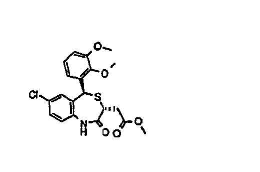

Methyl 2-[(cis)-7-chloro-5-(2,3-dimethoxyphenyl)-2-oxo-1,2,3,5-tetrahydro-4,1-benzothiazepin-3-yl]acetate (517 mg) was dissolved in methanol (10 mL). To the resulting solution was added potassium carbonate (85 mg). The resulting mixture was stirred at room temperature for 3 days. The reaction mixture was concentrated. The residue was diluted with ethyl acetate and washed with saturated brine. The organic layer was dried over anhydrous sodium sulfate. After concentration of the filtrate, the residue thus obtained was purified by silica gel column (ethyl acetate:hexane=1:3) to give the title compound (381 mg).

### Referential Example 214

2-[(trans)-7-Chloro-5-(2,3-dimethoxyphenyl)-2-oxo-1,2,3,5-tetrahydro-4,1-benzothiazepin-3-yl]acetic acid

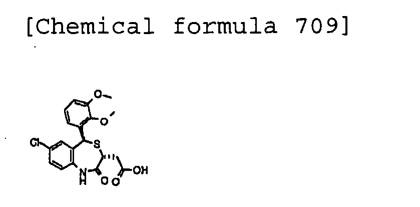

Methyl 2-[(trans)-7-chloro-5-(2,3-dimethoxyphenyl)-2-oxo-1,2,3,5-tetrahydro-4,1-benzothiazepin-3-yl]acetate (1.06 g) was dissolved in methanol-water-tetrahydrofuran (8:4:8, 20 mL). To the resulting solution was added lithium hydroxide monohydrate (0.21 g). The resulting mixture was stirred at 50°C for 2.5 hours. The reaction mixture was acidified with 1N hydrochloric acid, followed by extraction with chloroform. The extract was washed with saturated brine. The organic layer was dried over anhydrous sodium sulfate. The filtrate was then concentrated to give the title compound (1.00 g).
MS (ESI)m/z:408 (M⁺).
¹H-NMR(CDCl₃)δ:2.57(1H,dd,J=17.0,3.5Hz),3.12(1H,dd,J=17.0,9.9H z),3.60(3H,s),3.79-3.82(1H,m),3.88(3H,s),6.19(1H,s),6.86(1H,d,J=2.2Hz),6.98(1H ,dd,J=8.3,1.6Hz),7.09(1H,d,J=8.3Hz),7.19(1H,t,J=8.3Hz),7.30 -7.31(1H,m),7.36(1H,s).

### Referential Example 215

methyl 2-[(trans)-7-chloro-5-(2,3-dimethoxyphenyl)-2-thioxo-1,2,3,5-tetrahydro-4,1-benzothiazepin-3-yl]acetate

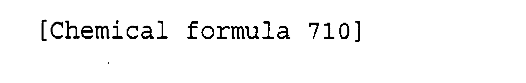

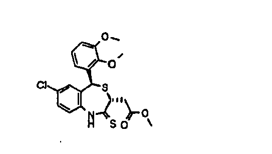

Methyl 2-[(trans)-7-chloro-5-(2,3-dimethoxyphenyl)-2-oxo-1,2,3,5-tetrahydro-4,1-benzothiazepin-3-yl]acetate (510 mg) was dissolved in toluene (20 mL). To the resulting solution was added Lawesson's reagent (831 mg). The resulting mixture was heated under reflux for one hour. The reaction mixture was cooled to room temperature and then concentrated. The residue was purified by silica gel column (chloroform) to give the title compound (643 mg).
MS (ESI) m/z: 438 (M⁺+H)
¹H-NMR(CDCl₃)δ:2.62 (1H,dd,J=16.8, 3.9Hz), 3.44(1H,dd,J=16.8, 10.0 Hz),3.61-3.61(3H,m), 3.67(3H,s), 3.87(3H,s), 4.07(1H, dd, J=10.0, 3.9Hz), 6 .03(1H,s), 6.85(1H, d,J=2.4Hz), 6.96-6.99(1H,m), 7.11(1H,d,J=8.3Hz), 7.18(1H,t,J=7.9Hz), 7.24-7.30(2H,m).

### Referential Example 216

Methyl 2-[(trans)-2-[2-acetylhydrazono]-7-chloro-5-(2,3-dimethoxyphenyl)-1,5-dihydro-4,1-benzothiazepin-3(3H)-yl] acetate

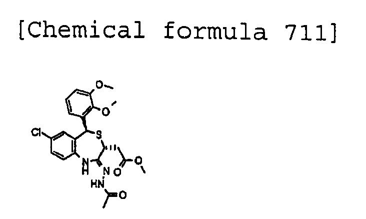

Methyl 2-[(trans)-7-chloro-5-(2,3-dimethoxyphenyl)-2-thioxo-1,2,3,5-tetrahydro-4,1-benzothiazepin-3-yl]acetate (228 mg) was dissolved in 2-propanol (8 mL). To the resulting solution was added acetylhydrazine (1.04 g). The resulting mixture was heated under reflux for 3 days. The reaction mixture was concentrated to give the title compound (249 mg).
MS (ESI) m/z :478 (M⁺+H).

### Example 323

Methyl 2-((trans)-8-chloro-6-(2,3-dimethoxyphenyl)-1-methyl-4H,-6H-[1,2,4]triazolo[4,3-a][4,1]benzothiazepin-4-yl)acetate

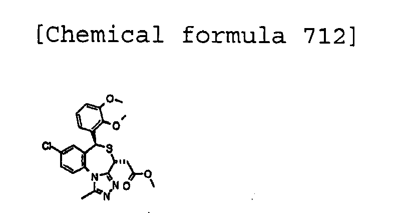

Methyl 2-[(trans)-2-[2-acetylhydrazono]-7-chloro-5-(2,3-dimethoxyphenyl)-1,5-dihydro-4,1-benzothiazepin-3(3H)-yl]acetate (348 mg) was dissolved in acetic acid (6 mL). The resulting solution was heated under reflux for 2.5 hours. The reaction mixture was cooled to room temperature and then concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (69 mg).
MS(ESI)m/z:460(M⁺+H).
¹H-NMR(CDCl₃)δ:2.49(3H,s),2.92(1H,dd,J=17.1,6.1Hz),3.46(3H,s), 3.61(1H,dd,J=17.1,8.3Hz),3.70(3H,s),3.85(3H,s),4.34(1H,dd,J =8.3,6.1Hz),5.33(1H,s),6.95-6.98(2H,m),7.17(1H,t,J=7.9Hz),7.21-7.23(2H,m),7.40-7.42(1H,m).

### Referential Example 217

2-[(trans)-8-Chloro-6-(2,3-dimethoxyphenyl)-1-methyl-4H,6H-[1,2,4]triazolo[4,3-a][4,1]benzothiazepin-4-yl]-1-ethanol

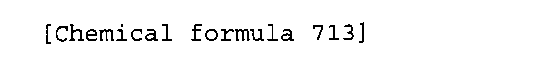

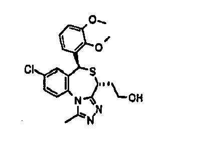

Lithium aluminum hydride (31 mg) was suspended in tetrahydrofuran (1 mL). Under stirring at -15°C, a solution of methyl 2-((trans)-8-chloro-6-(2,3-dimethoxyphenyl)-1-methyl-4H,6H-[1,2,4]triazolo[4,3-a][4,1]benzothiazepin-4-yl)acetate (69 mg) in tetrahydrofuran (1 mL) was slowly added dropwise. The reaction mixture was stirred as was for 30 minutes. To the reaction mixture was added an aqueous solution of potassium sodium tartrate, followed by concentration. The residue was extracted with chloroform. The organic layer was dried over anhydrous sodium sulfate. The filtrate was concentrated. The residue thus obtained was purified by preparative TLC (methanol:chloroform=1:9) to give the title compound (40 mg).
MS(ESI)m/z:432(M⁺+H).
¹H-NMR(CDCl₃)δ:2.24-2.32(1H,m),2.49(3H,s),2.59-2.67(1H,m),3.48(3H,s),3.77-3.83(1H,m),3.85(3H,s),3.95-4.00(1H,m),4.09(1H,dd,J=7.4,5.5Hz),5.32(1H,s),6.96-6.98(2H,m),7.16-7.25(3H,m),7.40(1H,dd,J=8.4,2.3Hz).

### Referential Example 218

2-[(trans)-8-Chloro-6-(2,3-dimethoxyphenyl)-1-methyl-4H,6H-[1,2,4]triazolo[4,3-a][4,1]benzothiazepin-4-yl]ethyl methanesulfonate

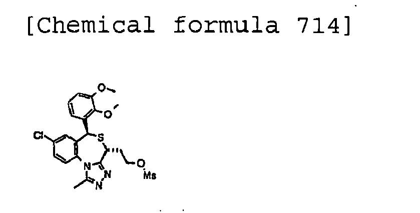

2-[(trans)-8-Chloro-6-(2,3-dimethoxyphenyl)-1-methyl-4H,6H-[1,2,4]triazolo[4,3-a][4,1]benzothiazepin-4-yl]-1-ethanol (20 mg) was dissolved in dichloromethane (2 mL). To the resulting solution were added triethylamine (0.0097 mL) and methanesulfonyl chloride (0.0054 mL) at 0°C. The resulting mixture was stirred for 1.5 hours. To the reaction mixture was added a saturated aqueous solution of sodium bicarbonate. The resulting mixture was extracted with chloroform. The organic layer was dried over anhydrous sodium sulfate to give the title compound (24 mg).
MS(ESI)m/z:510(M⁺+H).

### Example 324

Ethyl 1-{2-[(trans)-8-chloro-6-(2,3-dimethoxyphenyl)-1-methyl-4H,6H-[1,2,4]triazolo[4,3-a][4,1]benzothiazepin-4-yl]ethyl-1H-pyrazole-4-carboxylate

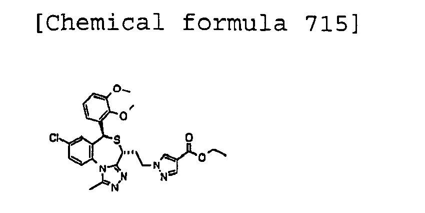

Ethyl 4-pyrazolecarboxylate (9.7 mg) was dissolved in N,N-dimethylformamide (1 mL). At 0°C, sodium hydride (55%, 3.0 mg) was added and the resulting mixture was stirred at 0°C for one hour. A solution of ethyl methanesulfonate (24 mg) in N,N-dimethylformamide (1 mL) was added dropwise to the reaction mixture. Tetrabutylammonium iodide (17 mg, 0.05 mmol) was added further. The resulting mixture was stirred at 80°C for 3 hours. After addition of water, the resulting mixture was extracted with chloroform. The organic layer was dried over anhydrous magnesium sulfate. After concentration of the filtrate, the residue thus obtained was purified by preparative TLC (methanol:chloroform=1:9) to give the title compound (22 mg).
MS(ESI)m/z:554(M⁺+H)
¹H-NMR(CDCl₃)δ:1.33(3H,t,J=7.2Hz),2.50(3H,s),2.52-2.57(1H,m),2.97-3.02(1H,m),3.46(3H,s),3.69(1H,dd,J=9.2,5.2Hz),3.85(3H,s),4.
27(2H,q,J=7.2Hz),4.38-4.46(1H,m),4.48-4.55(1H,m),5.31(1H,s),6.93-6.98(2H,m),7.15-7.20(3H,m),7.37(1H,dd,J=8.4,2.3Hz),7.77(1H,s),7.80(1H,s).

### Example 325

1-{2-[(trans)-8-Chloro-6-(2,3-dimethoxyphenyl)-1-methyl-4H,6H-[1,2,4]triazolo[4,3-a][4,1]benzothiazepin-4-yl]ethyl-1H-pyrazole-4-carboxylic acid

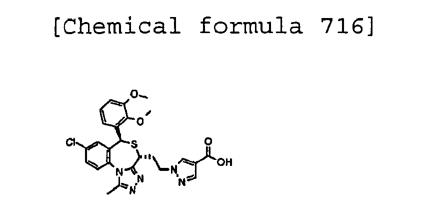

Ethyl 1-{2-[(trans)-8-chloro-6-(2,3-dimethoxyphenyl)-1-methyl-4H,6H-[1,2,4]triazolo[4,3-a] [4,1]benzothiazepin-4-yl]ethyl-1H-pyrazole-4-carboxylate (22 mg) was dissolved in methanol-water-tetrahydrofuran (2:1:2, 5 mL). To the resulting solution was added lithium hydroxide monohydrate (3.3 mg). The resulting mixture was stirred at 50°C for 3 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform:water=3:7:1) to give the title compound (17 mg).
MS(ESI)m/z:526(M⁺+H).
¹H-NMR(CDCl₃)δ:2.46-2.49(4H,m),2.89-2.96(1H,m),3.44(3H,s),3.71-3.74(1H,m),3.84(3H,s),4.31-4.45(2H,m),5.28(1H,s),6.91(1H,d,J=2.2Hz),6.95(1H,dd,J=7.9,1 .8Hz),7.13-7.22(3H,m),7.37(1H,dd,J=8.3,2.0Hz),7.78(1H,s),7.86(1H,s).

### Referential Example 219

Methyl 2-[(trans)-7-chloro-2-[2-(cyclopropylcarbonyl)hydrazono]-5-(2,3-dimethoxyphenyl)-1,5-dihydro-4,1-benzothiazepin-3(3H)-yl]acetate

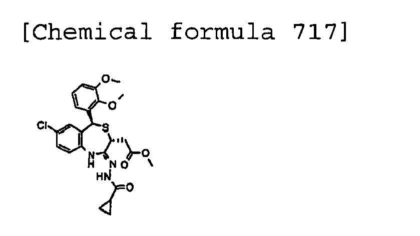

Methyl 2-[(trans)-7-chloro-5-(2,3-dimethoxyphenyl)-2-thioxo-1,2,3,5-tetrahydro-4,1-benzothiazepin-3-yl]acetate (86 mg) was dissolved in tetrahydrofuran (3 mL). To the resulting solution were added cyclopropane carbohydrazide (99 mg) and mercury chloride (64 mg). The resulting mixture was stirred at room temperature for 0.5 hour. Water was added to the reaction mixture, followed by extraction with chloroform. The organic layer was dried over anhydrous sodium sulfate. The filtrate was then concentrated to give the title compound (99 mg).
MS(ESI)m/z:504(M⁺+H) .

### Example 326

Methyl 2-[(trans)-8-chloro-1-cyclopropyl-6-(2,3-dimethoxyphenyl)-4H,6H-[1,2,4]triazolo[4,3-a][4,1]benzothiazepin-4-yl]acetate

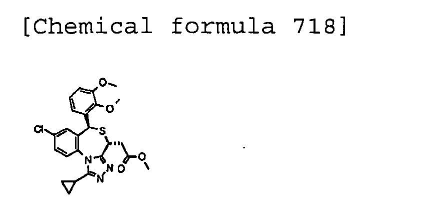

Methyl 2-[(trans)-7-chloro-2-[2-(cyclopropylcarbonyl)hydrazono]-5-(2,3-dimethoxyphenyl)-1,5-dihydro-4,1-benzothiazepin-3(3H)-yl]acetate (99 mg) was dissolved in acetic acid (3 mL). The resulting solution was heated under reflux for 2.5 hours. The reaction mixture was cooled to room temperature and then concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (70 mg).
MS(ESI)m/z:486(M⁺+H).
¹H-NMR(CDCl₃)δ:0.91-0.98(1H,m),1.06-1.13(1H,m),1.22-1.31(2H,m),1.75-1.79(1H,m),2.91(1H,dd,J=17.2,6.3Hz),3.48(3H,s),3.59(1H,dd,J =17.2,8.1Hz),3.69(3H,s),3.85(3H,s),4.35(1H,dd,J=8.1,6.3Hz), 5.39(1H,s),6.96(1H,dd,J=8.1,1.5Hz),6.99(1H,d,J=2.0Hz),7.17( 1H,t,J=8.1Hz),7.25(1H,dd,J=6.6,1.5Hz),7.37(1H,d,J=8.1Hz),7. 39-7.41(1H,m).

### Referential Example 220

2-[(trans)-8-Chloro-1-cyclopropyl-6-(2,3-dimethoxyphenyl)-4H,6H-[1,2,4]triazolo[4,3-a][4,1]benzothiazepin-4-yl]-1-ethanol

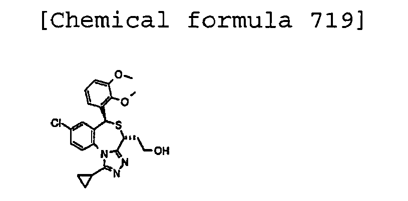

Lithium aluminum hydride (300 mg) was suspended in tetrahydrofuran (5 mL). Under stirring at -15°C, a solution of methyl 2-[(trans)-8-chloro-1-cyclopropyl-6-(2,3-dimethoxyphenyl)-4H,6H-[1,2,4]triazolo[4,3-a][4,1]benzothiazepin-4-yl]acetate (704 mg) in tetrahydrofuran (5 mL) was slowly added dropwise to the resulting suspension. The reaction mixture was stirred as was for 30 minutes. To the reaction mixture was added an aqueous solution of potassium sodium tartrate, followed by concentration. The residue was extracted with chloroform. The organic layer was dried over anhydrous sodium sulfate. The filtrate was concentrated to give the title compound (597 mg).
MS(ESI)m/z:458(M⁺+H).
¹H-NMR(CDCl₃)δ:0.93-0.99(1H,m),1.07-1.15(1H,m),1.20-1.31(2H,m),1.74-1.81(1H,m),2.23-2.31(1H,m),2.55-2.63(1H,m),3.50(3H,s),3.75-3.80(1H,m),3.86(3H,s),3.95-4.00(1H,m),4.08(1H,dd,J=7.4,5.2Hz),5.37(1H,s),6.97(2H,dd,J= 8.3,1.7Hz),7.18(1H,t,J=8.3Hz),7.24-7.26(1H,m),7.35(1H,d,J=8.3Hz),7.40(1H,dd,J=8.3,2.3Hz).

### Referential Example 221

2-[(trans)-8-Chloro-1-cyclopropyl-6-(2,3-dimethoxyphenyl)-4H,6H-[1,2,4]triazolo[4,3-a][4,1]benzothiazepin-4-yl]ethyl methanesulfonate

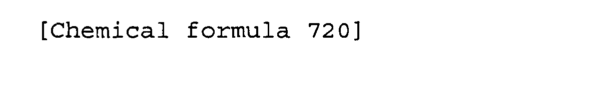

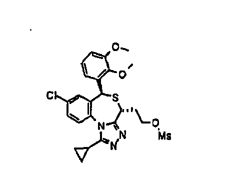

2-[(trans)-8-Chloro-1-cyclopropyl-6-(2,3-dimethoxyphenyl)-4H,6H-[1,2,4]triazolo[4,3-a][4,1]benzothiazepin-4-yl]-1-ethanol (597 mg) was dissolved in dichloromethane (10 mL). To the resulting solution were added triethylamine (0.273 mL) and methanesulfonyl chloride (0.151 mL) at 0°C. The resulting mixture was stirred for 2 hours. To the reaction mixture was added a saturated aqueous solution of sodium bicarbonate. The resulting mixture was extracted with chloroform. The organic layer was dried over anhydrous sodium sulfate to give the title compound (684 mg).
MS (ESI) m/z : 537 (M⁺+H) .

### Referential Example 222

3-[(trans)-8-Chloro-1-cyclopropyl-6-(2,3-dimethoxyphenyl)-4H,6H-[1,2,4]triazolo[4,3-a][4,1]benzothiazepin-4-yl]propanenitrile

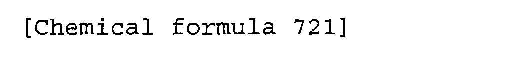

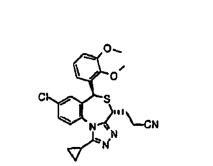

2-[(trans)-8-Chloro-1-cyclopropyl-6-(2,3-dimethoxyphenyl)-4H,6H-[1,2,4]triazolo[4,3-a][4,1]benzothiazepin-4-yl]ethyl methanesulfonate (684 mg) was dissolved in dimethylsulfoxide (12 mL). To the resulting solution was added sodium cyanide (190 mg). The resulting mixture was stirred overnight at 50°C. After cooling to room temperature, the reaction mixture was poured into water, followed by extraction with ethyl acetate. The organic layer was dried over anhydrous sodium sulfate. After concentration of the filtrate, the residue thus obtained was purified by silica gel column (methanol:chloroform=5:95) to give the title compound (550 mg).
MS(ESI)m/z:467(M⁺+H).
¹H-NMR(CDCl₃)δ:0.93-1.00(1H,m),1.08-1.16(1H,m),1.24-1.32(2H,m),1.75-1.81(1H,m),2.25-2.34(1H,m),2.67-2.81(2H,m),2.85-2.91(1H,m),3.49(3H,s),3.86(3H,s),4.03(1H,dd,J=8.7,5.2Hz),5. 40(1H,s),6.97-7.00(2H,m),7.19(1H,t,J=7.9Hz),7.24(1H,dd,J=7.9,1.7Hz),7.37( 1H,d,J=8.3Hz),7.42(1H,dd,J=8.3,2.2Hz).

### Example 327

3-[(trans)-8-Chloro-1-cyclopropyl-6-(2,3-dimethoxyphenyl)-4H,6H-[1,2,4]triazolo[4,3-a] [4,1]benzothiazepin-4-yl]propanoic acid

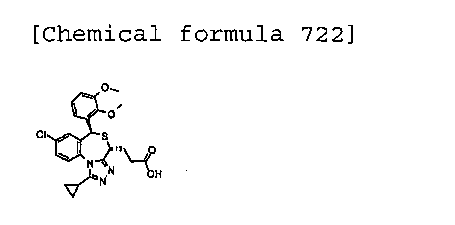

3-[(trans)-8-Chloro-1-cyclopropyl-6-(2,3-dimethoxyphenyl)-4H,6H-[1,2,4]triazolo[4,3-a][4,1]benzothiazepin-4-yl]propanenitrile (550 mg) was dissolved in 2-propanol (10 mL). To the resulting solution were added a 5M aqueous sodium hydroxide solution (2 mL) and methanol (3 mL). The resulting mixture was stirred at 80°C for 18 hours. The reaction mixture was returned to room temperature, neutralized with 1N hydrochloric acid and extracted with chloroform. The organic layer was dried over anhydrous sodium sulfate. The filtrate was concentrated to give the title compound (577 mg).
MS (ESI)m/z:486 (M⁺+H). ¹H-NMR(CDCl₃)δ:0.94-1.01(1H,m),1.09-1.15(1H,m),1.23-1.26(1H,m),1.28-1.32(1H,m),1.76-1.80(1H,m),2.29-2.39(1H,m),2.65-2.75(3H,m),3.50(3H,s),3.86(3H,s),3.98(1H,dd,J=7.8,5.1Hz),5., 37(1H,s),6.96-6.99(2H,m),7.19(1H,t,J=7.9Hz),7.23-7.25(1H,m),7.35(1H,d,J=8.5Hz),7.41(1H,dd,J=8.5,2.4Hz).

### Referential Example 223

Methyl 5-({3-[(trans)-8-chloro-1-cyclopropyl-6-(2,3-dimethoxyphenyl)-4H,6H-[1,2,4]triazolo[4,3-a][4,1]benzothiazepin-4-yl]propanoyl}amino)-4-oxopentanoate

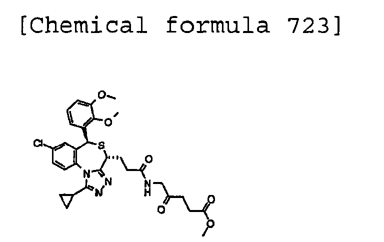

3-[(trans)-8-chloro-1-cyclopropyl-6-(2,3-dimethoxyphenyl)-4H,6H-[1,2,4]triazolo[4,3-a] [4, 1] benzothiazepin-4-yl] propanoic acid (36 mg) and methyl 5-aminolevulinate hydrochloride (16 mg) were dissolved in dichloromethane (2 mL). To the resulting solution were added 1-ethyl-3-(3-dimethylaminopropyl)carbodiimide hydrochloride (17 mg), 1-hydroxybenzotriazole (3.4 mg), and triethylamine (0.011 mL). The resulting mixture was stirred at room temperature for 3 hours. The reaction mixture was diluted with chloroform and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (28 mg).
MS (ESI) m/z : 613 (M⁺+H) .
¹H-NMR(CDCl₃)δ:0.92-0.98(1H,m),1.06-1.13(1H,m),1.21-1.26(1H,m),1.28-1.34(1H,m),1.74-1.81(1H,m),2.31-2.40(1H,m),2.56-2.73(8H,m),3.48(3H,s),3.68(3H,s),3.85(3H,s),3.96(1H,dd,J=8. 5,5.4Hz),4.12-4.16(2H,m),5.36(1H,s),6.56-6.59(1H,m),6.96-6.97(2H,m),7.18(1H,t,J=8.1Hz),7.24-7.26(1H,m),7.34(1H,d,J=8.3Hz),7.38(1H,dd,J=8.3,2.2Hz).

### Example 328

Methyl 3-(2-(2-[(trans)-8-chloro-1-cyclopropyl-6-(2,3-dimethoxyphenyl)-4H,6H-[1,2,4]triazolo[4,3-a][4,1]benzothiazepin-4-yl]ethyl)-1,3-thiazol-5-yl)propanoate

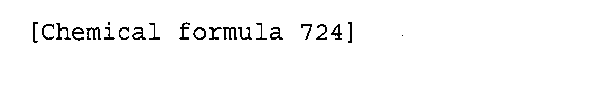

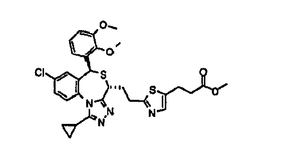

Methyl 5-({3-[(trans)-8-chloro-1-cyclopropyl-6-(2,3-dimethoxyphenyl)-4H,6H-[1,2,4]triazolo[4,3-a][4,1]benzothiazepin-4-yl]propanoyl}amino)-4-oxopentanoate (28 mg) was dissolved in tetrahydrofuran (2 mL). To the resulting solution was added a Lawesson's reagent (28 mg). The reaction mixture was stirred at 70°C for one hour. The reaction mixture was cooled to room temperature and then concentrated. The residue was purified by preparative TLC (methanol:chloroform=5:95) to give the title compound (24 mg) .
MS(ESI)m/z:611(M⁺+H). ¹H-NMR(CDCl₃)δ:0.92-0.98(1H,m),1.06-1.13(1H,m),1.20-1.25(1H,m),1.28-1.34(1H,m),1.75-1.80(1H,m),2.40-2.49(1H,m),2.60-2.64(2H,m),2.86-2.95(1H,m),3.06-3.10(2H,m),3.20-3.25(2H,m),3.49(3H,s),3.68(3H,s),3.85(3H,s),3.93(1H,dd,J=8. 1,6.1Hz),5.36(1H,s),6.95-6.98(2H,m),7.17(1H,t,J=7.9Hz),7.24-7.26(1H,m),7.28-7.31(1H,m),7.34(1H,d,J=8.5Hz),7.39(1H,dd,J=8.5,2.2Hz).

### Example 329

3-(2-{2-[(trans)-8-Chloro-1-cyclopropyl-6-(2,3-dimethoxyphenyl)-4H,6H-[1,2,4]triazolo[4,3-a][4,1]benzothiazepin-4-yl]ethyl}-1,3-thiazol-5-yl)propanoic acid

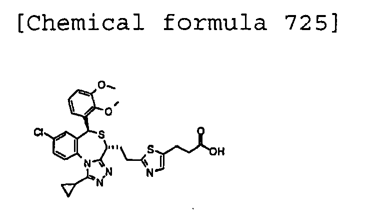

Methyl 3-(2-{2-[(trans)-8-chloro-1-cyclopropyl-6-(2,3-dimethoxyphenyl)-4H,6H-[1,2,4]triazolo[4,3-a][4,1]benzothiazepin-4-yl]ethyl}-1,3-thiazol-5-yl)propanoate (24 mg) was dissolved in methanol-water-tetrahydrofuran (1:0.5:1, 2.5 mL). To the resulting solution was added lithium hydroxide monohydrate (4.9 mg). The reaction mixture was stirred at room temperature for 3 days. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform:water=3:7:1) to give the title compound (23 mg).
MS (ESI) m/z : 597 (M⁺+H).
¹H-NMR(CDCl₃)δ:0.89-0.96(1H,m),1.05-1.12(1H,m),1.16-1.22(1H,m),1.27-1.33(1H,m),1.74-1.80(1H,m),2.35-2.43(1H,m),2.55-2.59(2H,m),2.81-2.88(1H,m),3.00-3.04(2H,m),3.13-3.17(2H,m),3.47(3H,s),3.84(3H,s),3.89-3.93(1H,m),5.33(1H,s),6.94-6.97(2H,m),7.16(1H,t,J=8.1Hz),7.23-7.25(1H,m),7.26(1H,s),7.34-7.36(1H,m),7.38-7.40(1H,m).

### Referential Example 224

Methyl 5-({2-[(trans)-7-chloro-5-(2;3-dimethoxyphenyl)-2-oxo-1,2,3,5-tetrahydro-4,1-benzothiazepin-3-yl]acetyl}amino)-4-oxopentanoate

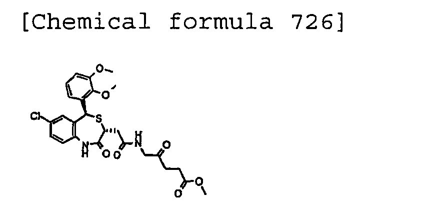

2-[(trans)-7-Chloro-5-(2,3-dimethoxyphenyl)-2-oxo-1,2,3,5-tetrahydro-4,1-benzothiazepin-3-yl]acetic acid (300 mg) and methyl 5-aminolevulinate hydrochloride (160 mg) were dissolved in dichloromethane (6 mL). To the resulting solution were added 1-ethyl-3-(3-dimethylaminopropyl)carbodiimide hydrochloride (169 mg), 1-hydroxybenzotriazole (33.8 mg), and triethylamine(0.0819 mL). The resulting mixture was stirred at room temperature for 3.5 hours. The reaction mixture was diluted with chloroform and washed with a saturated aqueous solution of sodium bicarbonate and saturated brine. The organic layer was dried over anhydrous sodium sulfate. The filtrate was then concentrated to give the title compound (348 mg).
MS(ESI)m/z:535(M⁺+H).

### Referential Example 225

Methyl 3-({2-[(trans)-7-chloro-5-(2,3-dimethoxyphenyl)-2-oxo-1,2,3,5-tetrahydro-4,1-benzothiazepin-3-yl]methyl}-1,3-thiazol-5-yl)propanoate

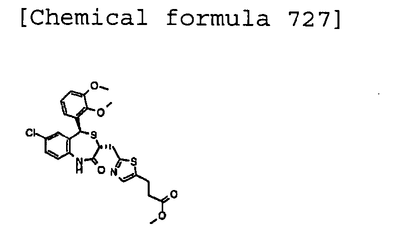

Methyl 5-({2-[(trans)-7-chloro-5-(2,3-dimethoxyphenyl)-2-oxo-1,2,3,5-tetrahydro-4,1-benzothiazepin-3-yl]acetyl}amino)-4-oxopentanoate (348 mg) was dissolved in tetrahydrofuran (8 mL). To the resulting solution was added Lawesson's reagent (289 mg). The resulting mixture was stirred at 70°C for one hour. After cooling to room temperature, the reaction mixture was concentrated. The residue was purified by silica gel column (methanol:chloroform=1:10) to give the title compound (460 mg).
MS(ESI)m/z:533(M⁺+H).
¹H-NMR(CDCl₃)δ:2.64-2.65(2H,m),3.08-3.10(2H,m),3.59(3H,s),3.69(3H,s),3.80-3.84(2H,m),3.87(3H,s),3.95-3.97(1H,m),6.19-6.22(1H,m),6.82-6.82(1H,m),6.96-6.98(1H,m),7.02-7.04(1H,m),7.15-7.21(2H,m),7.41-7.44(1H,m).

### Referential Example 226

Methyl 3-({2-[(trans)-7-chloro-5-(2,3-dimethoxyphenyl)-2-thioxo-1,2,3,5-tetrahydro-4,1-benzothiazepin-3-yl]methyl}-1,3-thiazol-5-yl)propanoate

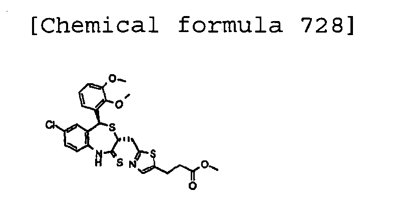

Methyl 3-({2-[(trans)-7-chloro-5-(2,3-dimethoxyphenyl)-2-oxo-1,2,3,5-tetrahydro-4,1-benzothiazepin-3-yl]methyl}-1,3-thiazol-5-yl)propanoate (346 mg) was dissolved in toluene (10 mL). To the resulting solution was added Lawesson's reagent (289 mg). The resulting mixture was heated under reflux for one hour. After cooling to room temperature, the reaction mixture was concentrated. The residue was purified by silica gel column (ethyl acetate:hexane=1:1) to give the title compound (215 mg).
MS (ESI)m/z:549 (M⁺+H) .
¹H-NMR(CDCl₃)δ:2.65(2H,t,J=7.3Hz),3.10(2H,t,J=7.3Hz),3.33(1H,d d,J=15.1,5.4Hz),3.61(3H,s),3.69(3H,s),3.87(3H,s),3.94(1H,dd ,J=15.1,8.1Hz),4.18(1H,dd,J=8.1,5.4Hz),6.04(1H,s),6.82(1H,d ,J=2.2Hz),6.97(1H,dd,J=8.3,1.5Hz),7.04(1H,d,J=8.3Hz),7.17(1 H,t,J=8.3Hz),7.22-7.25(2H,m),7.35(1H,s).

### Referential Example 227

Methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate

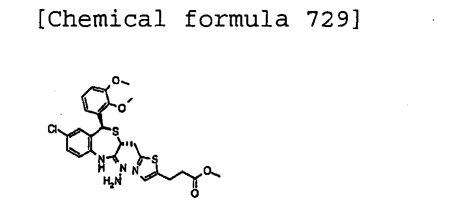

Methyl 3-({2-[(trans)-7-chloro-5-(2,3-dimethoxyphenyl)-2-thioxo-1,2,3,5-tetrahydro-4,1-benzothiazepin-3-yl]methyl}-1,3-thiazol-5-yl)propanoate (215 mg) was dissolved in ethanol (5 mL). To the resulting solution was added hydrazine monohydrate (0.0950 mL). The resulting mixture was stirred at room temperature for 2 days. The reaction mixture was concentrated to give the title compound (214 mg).
MS (ESI)m/z:547 (M⁺+H) .

### Example 330

Methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate

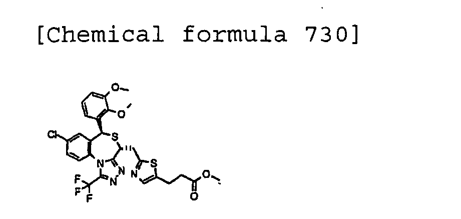

Methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate (214 mg) was dissolved in dichloromethane (5 mL). To the resulting solution were added trifluoroacetic anhydride (0.155 mL) and trifluoroacetic acid (0.603 mL). The resulting mixture was stirred at room temperature for 0.5 hour. The reaction mixture was stirred at 55°C to distill off dichloromethane. To the residue was added toluene (5 mL). The resulting mixture was heated under reflux for 3 hours. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by preparative TLC (ethyl acetate:hexane=1:1) to give the title compound (168 mg).
MS(ESI)m/z:625(M⁺+H).
¹H-NMR(CDCl₃)δ:2.65(2H,t,J=7.4Hz),3.11(2H,t,J=7.4Hz),3.43(3H,s ),3.65(1H,dd,J=15.5,7.2Hz),3.69(3H,s),3.84(3H,s),4.09-4.15(1H,m),4.47(1H,t,J=7.2Hz),5.35(1H,s),6.95-6.98(2H,m),7.13-7.18(2H,m),7.33-7.36(2H,m),7.41(1H,dd,J=8.5,2.2Hz).

### Example 331

3-({2-[(trans)-8-Chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoic acid

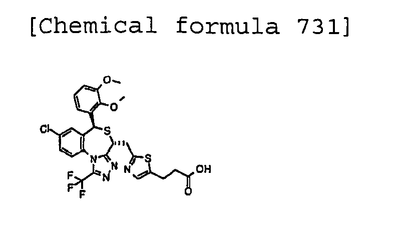

Methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate (168 mg) was dissolved in methanol-water-tetrahydrofuran (2:1:2, 5 mL). To the resulting solution was added lithium hydroxide monohydrate (22.5 mg). The resulting mixture was stirred at room temperature for 18 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform=1:9) to give the title compound (99.9 mg).
MS(ESI)m/z:611(M⁺+H).
¹H-NMR(CDCl₃)δ:2.67(2H,t,J=7.3Hz),3.09(2H,t,J=7.3Hz),3.42(3H,s ),3.63(1H,dd,J=15.4,7.1Hz),3.84(3H,s),4.08-4.14(1H,m),4.47(1H,t,J=7.1Hz),5.34(1H,s),6.96-6.99(2H,m),7.14(1H,t,J=7.9Hz),7.18(1H,dd,J=7.9,1.8Hz),7.34-7.36(2H,m),7.41(1H,dd,J=8.5,2.4Hz).

### Referential Example 228

(trans)-7-Chloro-5-(2,3-dimethoxyphenyl)-3-(2-hydroxyethyl)-1,5-dihydro-4,1-benzothiazepin-2(3H)-one

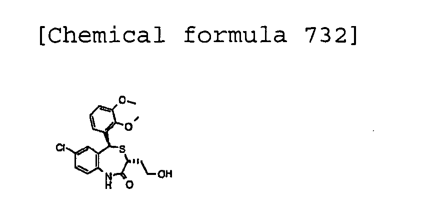

2-[(trans)-7-Chloro-5-(2,3-dimethoxyphenyl)-2-oxo-1,2,3,5-tetrahydro-4,1-benzothiazepin-3-yl]acetic acid (1024 mg) was dissolved in tetrahydrofuran (20 mL). To the resulting solution were added N-methylmorpholine (0.331 mL) and ethyl chloroformate (0.288 mL) at 0°C. The resulting mixture was stirred for 2.5 hours. To the reaction mixture were added sodium borohydride (285 mg) and methanol (20 mL) at 0°C, followed by stirring for 0.5 hour. The reaction mixture was acidified with 1N hydrochloric acid, extracted with ethyl acetate and washed with saturated brine. The organic layer was dried over anhydrous sodium sulfate. After concentration of the filtrate, the residue thus obtained was purified by silica gel column (ethyl acetate:hexane=5:4) to give the title compound (723 mg).
MS (ESI)m/z:394 (M⁺+H) .
¹H-NMR(CDCl₃)δ:1.81-1.88(1H,m)2.24-2.33(1H,m),3.59(3H,s),3.61-3.62(1H,m),3.65-3.80(2H,m),3.87(3H,s),6.17(1H,s),6.84(1H,d,J=2.2Hz),6.97(1H ,dd,J=8.3,1.5Hz),7.05(1H,d,J=8.3Hz),7.19-7.22(2H,m),7.33(1H,dd,J=7.7,1.5Hz).

### Referential Example 229

(trans)-3-(2-{[t-Butyl(diphenyl)silyl]oxy}ethyl)-7-chloro-5-(2,3-dimethoxyphenyl)-3-(2-hydroxyethyl)-1,5-dihydro-4,1-benzothiazepin-2(3H)-one

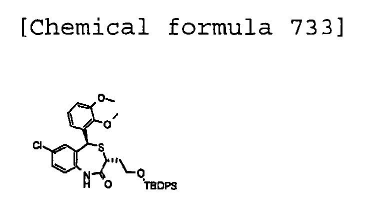

(trans)-7-Chloro-5-(2,3-dimethoxyphenyl)-3-(2-hydroxyethyl)-1,5-dihydro-4,1-benzothiazepin-2(3H)-one (959 mg) was dissolved in dichloromethane (20 mL). To the resulting solution were added t-butylchlorodiphenylsilane (0.760 mL) and imidazole (322 mg). The resulting mixture was stirred at room temperature for 17 hours. The reaction mixture was diluted with ethyl acetate and washed with saturated brine. The organic layer was dried over anhydrous sodium sulfate. After concentration of the filtrate, the residue thus obtained was purified by silica gel column (ethyl acetate:hexane=1:3) to give the title compound (1392 mg). ¹H-NMR(CDCl₃)δ:0.93(9H,s),1.76-1.84(1H,m),2.25-2.33(1H,m),3.60(3H,s),3.72-3.81(3H,m),3.88(3H,s),6.19(1H,s),6.85(1H,d,J=2.2Hz),6.95-6.99(2H,m),7.10(1H,s),7.17-7.21(2H,m),7.33-7.37(SH,m),7.39-7.44(2H,m),7.55-7.58(4H,m).

### Referential Example 230

(trans)-3-(2-{[t-Butyl(diphenyl)silyl]oxy}ethyl)-7-chloro-5-(2,3-dimethoxyphenyl)-3-(2-hydroxyethyl)-1,5-dihydro-4,1-benzothiazepin-2(3H)-thione

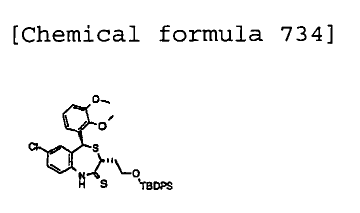

(trans)-3-(2-{[t-Butyl(diphenyl)silyl]oxy}ethyl)-7-chloro-5-(2,3-dimethoxyphenyl)-3-(2-hydroxyethyl)-1,5-dihydro-4,1-benzothiazepin-2(3H)-one (1392 mg) was dissolved in toluene (25 mL). To the resulting solution was added Lawesson's reagent (979 mg). The resulting mixture was heated under reflux for one hour. The reaction mixture was cooled to room temperature and then concentrated. The residue was purified by silica gel column (chloroform) to give the title compound (1187 mg).
¹H-NMR(CDCl₃)δ:0.89 (9H,s), 1.98-2.04 (1H,m),2.47-2.51(1H,m), 3.61(3H,s), 3.79-3.81(2H,m), 3.88(3H,s), 4.06(1H,t,J=6.7Hz), 6.04(1H,s), 6.85(1H ,d,J=2.2Hz),6.97-7.00(2H,m),7.17-7.23(2H,m), 7.29(1H,dd,J=7.8,1.5Hz), 7.34-7.37(4H,m), 7.39-7.45(2H,m), 7.53-7.58(4H,m), 9.33(1H,s).

### Referential Example 231

(trans)-3-(2-{[t-Butyl(diphenyl)silyl]oxy}ethyl)-7-chloro-5-(2,3-dimethoxyphenyl)-3-(2-hydroxyethyl)-1,5-dihydro-4,1-benzothiazepin-2(3H)-one hydrazone

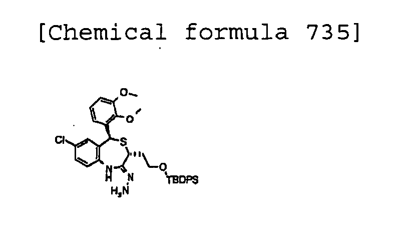

(trans)-3-(2-{[t-Butyl(diphenyl)silyl]oxy}ethyl)-7-chloro-5-(2,3-dimethoxyphenyl)-3-(2-hydroxyethyl)-1,5-dihydro-4,1-benzothiazepin-2(3H)-thione (1187 mg) was dissolved in ethanol (20 mL). To the resulting solution was added hydrazine monohydrate (0.444 mL). The resulting mixture was stirred at room temperature for 2 days. The reaction mixture was concentrated to give the title compound(1183 mg).
MS (ESI) m/z : 646 (M⁺+H) .

### Referential Example 232

2-[(trans)-8-Chloro-6-(2,3-dimethoxyphenyl)-1-(trifluoromethyl)-4H,6H-[1,2,4]triazolo[4,3-a][4,1]benzothiazepin-4-yl]-1-ethanol

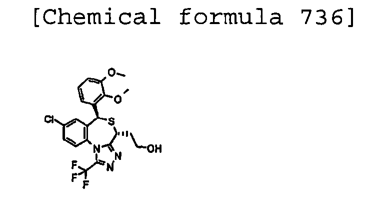

(trans)-3-(2-{[t-Butyl(diphenyl)silyl]oxy}ethyl)-7-chloro-5-(2,3-dimethoxyphenyl)-3-(2-hydroxyethyl)-1,5-dihydro-4,1-benzothiazepin-2(3H)-one hydrazone (1183 mg) was dissolved in dichloromethane (20 mL). To the resulting solution were added trifluoroacetic anhydride (0.724 mL) and trifluoroacetic acid (2.82 mL). The resulting mixture was stirred at room temperature for 0.5 hour. The reaction mixture was stirred at 55°C to distill off dichloromethane. To the residue was added toluene (20 mL), followed by heating under reflux for 3 hours. After cooling to room temperature, the reaction mixture was concentrated. To the residue was added a saturated aqueous solution of sodium bicarbonate. The resulting mixture was extracted with chloroform. The organic layer was dried over anhydrous sodium sulfate. After concentration of the filtrate, the residue thus obtained was dissolved in tetrahydrofuran (20 mL). To the resulting solution was added a 1M tetrabutylammonium fluoride/tetrahydrofuran solution (5.49 mL) and the mixture was stirred overnight. The reaction mixture was concentrated. The residue thus obtained was purified by silica gel column (methanol:chloroform=1:9) to give the title compound (614 mg).
MS(ESI)m/z:486(M⁺+H). ¹H-NMR(CDCl₃)δ:2.28-2.31(1H,m),2.70-2.72(1H,m),3.43-3.44(3H,m),3.82-3.84(1H,m),3.85-3.85(3H,m),3.92-3.94(1H,m),4.10(1H,dd,J=7.6,5.9Hz),5.34(1H,d,J=1.2Hz),6.97-6.99(2H,m),7.16-7.18(1H,m),7.22-7.24(1H,m),7.34-7.37(1H,m),7.41-7.43(1H,m).

### Referential Example 233

2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl methanesulfonate

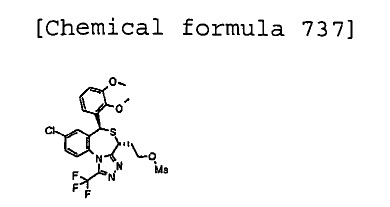

2-[(trans)-8-Chloro-6-(2,3-dimethoxyphenyl)-1-(trifluoromethyl)-4H,6H-[1,2,4]triazolo[4,3-a][4,1]benzothiazepin-4-yl]-1-ethanol (103 mg) was dissolved in dichloromethane (3 mL). To the resulting solution were added triethylamine (0.088 mL) and methanesulfonyl chloride (0.033 mL) at 0°C. The resulting mixture was stirred at room temperature for one hour. To the reaction mixture was added a saturated aqueous solution of sodium bicarbonate, followed by extraction with chloroform. The organic layer was dried over anhydrous sodium sulfate to give the title compound (125 mg).
MS(ESI)m/z:564(M⁺+H) .

### Example 332

Ethyl 1-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-pyrazole-4-carboxylate

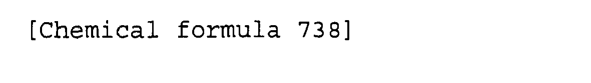

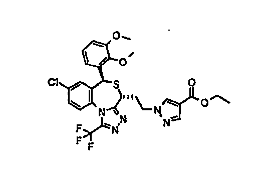

Ethyl 4-pyrazolecarboxylate (58 mg) was dissolved in N,N-dimethylformamide (2 mL). At 0°C, sodium hydride (55%, 18 mg) was added and the resulting mixture was stirred at 50°C for 30 minutes. The reaction mixture was returned to room temperature. A solution of 2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl methanesulfonate (78 mg) in N,N-dimethylformamide (2 mL) was added dropwise. To the reaction mixture was added tetrabutylammonium iodide (51 mg) further. The resulting mixture was stirred at room temperature for 3 days. To the reaction mixture was added water and the resulting mixture was extracted with ethyl acetate. The organic layer was dried over anhydrous sodium sulfate. After concentration of the filtrate, the residue thus obtained was purified by preparative TLC (methanol:chloroform=5:95) to give the title compound (56 mg).
MS(ESI)m/z:608(M⁺+H) . ¹H-NMR(CDCl₃)δ:1.33(3H,t,J=7.1Hz),2.48-2.57(1H,m) 2.96-3.04(1H,m),3.43(3H,s),3.71(1H,dd,J=9.2,5.2Hz),3.85(3H,s),4.
28(2H,q,J=7.1Hz),4.38-4.44(1H,m),4.47-4.53(1H,m),5.33(1H,s),6.95(1H,d,J=2.2Hz),6.97-6.99(1H,m),7.17-7.18(2H,m),7.32(1H,d,J=8.5Hz),7.41(1H,dd,J=8.5,2.2Hz),7.78( 2H,d,J=2.2Hz).

### Example 333

1-{2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl-1H-pyrazole-4-carboxylic acid

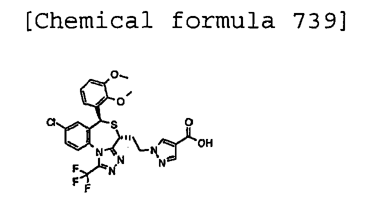

Ethyl 1-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl-1H-pyrazole-4-carboxylate (56 mg) was dissolved in methanol-water-tetrahydrofuran (2:1:2, 5 mL). To the resulting solution was added lithium hydroxide monohydrate (16 mg). The resulting mixture was stirred at 55°C for 2 days. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform:water=3:7:1) to give the title compound (47 mg).
MS(ESI)m/z:580(M⁺+H).
¹H-NMR(CDCl₃)δ:2.44-2.46(1H,m),2.92-2.94(1H,m),3.40(3H,s),3.75-3.80(1H,m),3.84(3H,s),4.29-4.30(1H,m),4.39-4.40(1H,m),5.30(1H,s),6.92(1H,d,J=2.2Hz),6.96(1H,dd,J=7.8,1 .7Hz),7.13-7.17(2H,m),7.35-7.39(2H,m),7.70-7.72(1H,m),7.83-7.85(1H,m).

### Example 334

Methyl (1-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-pyrazol-3-yl)acetate Methyl (1-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-pyrazol-5-yl)acetate

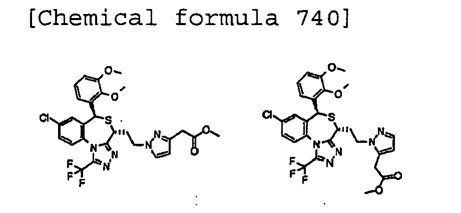

Methyl 3-pyrazoleacetate (98 mg) was dissolved in N,N-dimethylformamide (2 mL). To the resulting solution was added sodium hydride (55%, 31 mg) 0°C. The resulting mixture was stirred at room temperature for 10 minutes. To the reaction mixture was added dropwise a solution of 2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl methanesulfonate (144 mg) in N,N-dimethylformamide (2 mL). Tetrabutylammonium iodide (94 mg) was added further. The resulting mixture was stirred overnight at 70°C. Water was added and the resulting mixture was extracted with ethyl acetate. The organic layer was dried over anhydrous sodium sulfate. After concentration of the filtrate, the residue thus obtained was purified twice by preparative TLC (ethyl acetate:hexane=2:3, methanol:chloroform=1:99) to give the 3-yl isomer (23 mg) and 5-yl isomer (9.2 mg). 3-yl Isomer
MS(ESI)m/z:608(M⁺+H).
¹H-NMR(CDCl₃)δ:2.44-2.48(1H,m),2.95-3.00(1H,m),3.43(3H,s),3.55(2H,s),3.69(3H,s),3.78-3.79(1H,m),3.84(3H,d,J=5.6Hz),4.28-4.34(1H,m),4.37-4.44(1H,m),5.32(1H,s),6.13(1H,d,J=2.2Hz),6.95-6.98(2H,m),7.17-7.19(2H,m),7.25-7.26(1H,m),7.34-7.36(1H,m),7.41-7.43(1H,m).
5-yl Isomer
MS(ESI)m/z:608(M+H)⁺.
¹H-NMR(CDCl₃)δ:2.58-2.61(1H,m),2.95-3.01(1H,m),3.42(3H,s),3.67(2H,s),3.69(3H,s),3.80-3.85(1H,m),4.22-4.29(1H,m),4.37-4.44(1H,m),5.33(1H,s),6.07(1H,d,J=1.7Hz),6.93-6.94(1H,m),6.96-6.99(1H,m),7.14-7.19(2H,m),7.32-7.33(1H,m),7.37-7.38(2H,m).

### Example 335

(1-{2-[(trans)-8-Chloro-6-(2,3-dimethoxyphenyl)-1-(trifluoromethyl)-4H,6H-[1,2,4] triazolo[4,3-a][4,1]benzothiazepin-4-yl]ethyl}-1H-pyrazol-3-yl)acetic acid

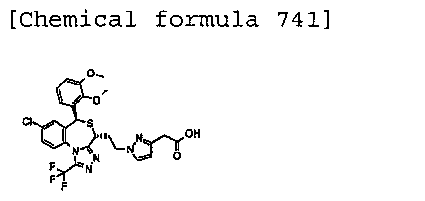

Methyl (1-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-pyrazol-3-yl)acetate (23 mg) was dissolved in methanol-water-tetrahydrofuran (1:0.5:1, 2.5 mL). To the resulting solution was added lithium hydroxide monohydrate (3.2 mg). The resulting mixture was stirred at room temperature for 17 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform=1:9) to give the title compound (19 mg).
MS(ESI)m/z:594(M⁺+H).
¹H-NMR(CDCl₃)δ:2.42-2.43(1H,m),2.90-2.92(1H,m),3.42(3H,s),3.47(2H,s),3.72-3.73(1H,m),3.84(3H,s),4.28-4.35(2H,m),5.31(1H,s),6.04-6.05(1H,m),6.93(1H,d,J=2.2Hz),6.97(1H,dd,J=7.8,2.2Hz),7.15-7.19(2H,m),7.24(1H,d,J=2.0Hz),7.35(1H,d,J=8.5Hz),7.40(1H,dd ,J=8.5,2.2Hz).

### Example 336

(1-{2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl}-1H-pyrazol-5-yl)acetic acid

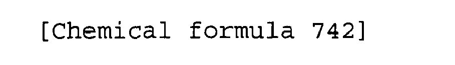

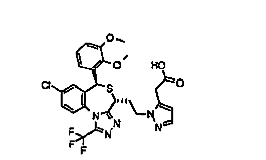

Methyl (1-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-pyrazol-5-yl)acetate (9.8 mg) was dissolved in methanol-water-tetrahydrofuran (1:0.5:1, 2.5 mL). To the resulting solution was added lithium hydroxide monohydrate (1.4 mg). The resulting mixture was stirred at room temperature for 17 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform=1:9) to give the title compound (6.3 mg).
MS(ESI)m/z:594(M⁺+H).
¹H-NMR(CDCl₃)δ:2.42-2.43(1H,m),2.90-2.92(1H,m),3.42(3H,s),3.47(2H,s),3.72-3.73(1H,m),3.84(3H,s),4.28-4.35(2H,m),5.31(1H,s),6.04-6.05(1H,m),6.93(1H,d,J=2.2Hz),6.97(1H,dd,J=7.8,2.2Hz),7.15-7.19(2H,m),7.24(1H,d,J=2.0Hz),7.35(1H,d,J=8.5Hz),7.40(1H,dd ,J=8.5,2.2Hz).

### Referential Example 234

3-[(trans)-8-Chloro-6-(2,3-dimethoxyphenyl)-1-(trifluoromethyl)-4H,6H-[1,2,4]triazolo[4,3-a][4,1]benzothiazepin-4-yl]propanenitrile

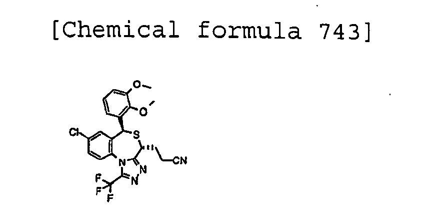

2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl methanesulfonate (120 mg) was dissolved in dimethylsulfoxide (2 mL). To the resulting solution was added sodium cyanide (31 mg). The resulting mixture was stirred overnight at 55°C. After cooling to room temperature, the reaction mixture was poured into water, followed by extraction with ethyl acetate. The organic layer was dried over anhydrous sodium sulfate. After concentration of the filtrate, the residue thus obtained was purified by preparative TLC (methanol:chloroform=1:9) to give the title compound (84 mg).
MS(ESI)m/z:495(M⁺+H).
¹H-NMR(CDCl₃)δ:2.34-2.44(1H,m),2.70-2.88(3H,m),3.44(3H,s),3.86(3H,s),4.01(1H,dd,J=7.9,5.5Hz),5. 37(1H,s),6.98-7.01(2H,m),7.19-7.21(2H,m),7.36-7.38(1H,m),7.45(1H,dd,J=8.5,2.4Hz).

### Example 337

3-[(trans)-8-Chloro-6-(2,3-dimethoxyphenyl)-1-(trifluoromethyl)-4H,6H-[1,2,4]triazolo[4,3-a][4,1]benzothiazepin-4-yl]propanoic acid

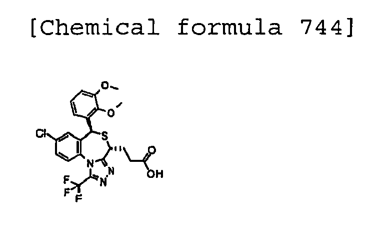

3-[(trans)-8-Chloro-6-(2,3-dimethoxyphenyl)-1-(trifluoromethyl)-4H,6H-[1,2,4]triazolo[4,3-a][4,1]benzothiazepin-4-yl]propanenitrile (84 mg) was dissolved in 2-propanol (3 mL). To the resulting solution were added a 5M aqueous sodium hydroxide solution (0.5 mL) and methanol (1 mL). The resulting mixture was stirred at 80°C for 20 hours. The reaction mixture was returned to room temperature, was neutralized with 1N hydrochloric acid and extracted with chloroform. The organic layer was dried over anhydrous sodium sulfate. After concentration of the filtrate, the residue thus obtained was purified by preparative TLC (methanol:chloroform=1:9) to give the title compound (64 mg).
MS (ESI) m/z: 514 (M⁺+H)
¹H-NMR(CDCl₃) δ: 2.67-2.68 (2H,m) 3.43 (3H, s),3.85 (3H, s),3.94-3.95(1H,m),5.33(1H,s),6.97-6.99(2H,m),7.15-7.22(2H,m),7.33-7.35(1H,m),7.40-7.42(1H,m).

### Referential Example 235

Ethyl 4-({3-[(trans)-8-chloro-6-(2,3-dimethoxyphenyl)-1-(trifluoro)-4H,6H-[1,2,4]triazolo[4,3-a][4,1]-benzoxazepin-4-yl]propanoyl}amino)-3-oxobutanoate

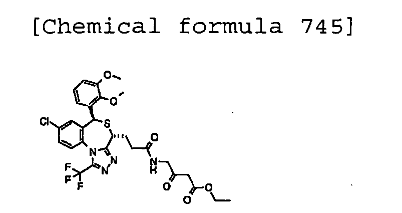

3-[(trans)-8-Chloro-6-(2,3-dimethoxyphenyl)-1-(trifluoromethyl)-4H,6H-[1,2,4]triazolo[4,3-a][4,1]benzothiazepin-4-yl]propanoic acid (29 mg) and ethyl 4-amino-3-oxobutanoate hydrochloride (11 mg) were dissolved in dichloromethane (2 mL). To the resulting solution were added 1-ethyl-3-(3-dimethylaminopropyl)carbodiimide hydrochloride (139 mg), 1-hydroxybenzotriazole (2.6 mg), and triethylamine (0.0088 mL). The resulting mixture was stirred at room temperature for 17 hours. The reaction mixture was diluted with chloroform, and washed with a saturated aqueous solution of sodium bicarbonate and saturated brine. The organic layer was dried over anhydrous sodium sulfate. After concentration of the filtrate, the residue thus obtained was purified by preparative TLC (methanol:chloroform=5:95) to give the title compound (23 mg).
MS(ESI)m/z:641(M⁺+H).

### Example 338

Ethyl 2-(2-{2-[(trans)-8-chloro-6-(2,3-dimethoxyphenyl)-1-(trifluoro)-4H,6H-[1,2,4]triazolo[4,3-a][4,1]-benzoxazepin-4-yl]propanoyl}-1,3-thiazol-5-yl)acetate

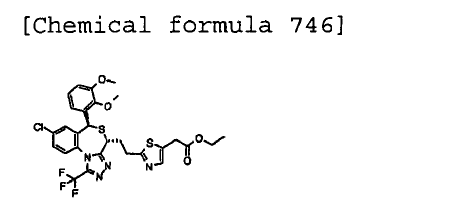

Ethyl 4-({3-[(trans)-8-chloro-6-(2,3-dimethoxyphenyl)-1-(trifluoro)-4H,6H-[1,2,4]triazolo[4,3-a][4,1]-benzoxazepin-4-yl]propanoyl}amino)-3-oxobutanoate (23 mg) was dissolved in tetrahydrofuran (2 mL). To the resulting solution was added Lawesson's reagent (21 mg).
The resulting mixture was stirred at 70°C for one hour. The reaction mixture was cooled to room temperature and then concentrated. The residue was purified by preparative TLC (methanol:chloroform=1:10) to give the title compound (19 mg).
MS(ESI)m/z:639(M⁺+H). ¹H-NMR(CDCl₃)δ:1.25-1.30(3H,m),2.49-2.52(1H,m),2.96-2.98(1H,m),3.25-3.27(2H,m),3.43(3H,s),3.76(2H,s),3.85(3H,s),3.96(1H,dd,J=8. 3,6.1Hz),4.19(2H,q,J=7.1Hz),5.33(1H,s),6.96-6.98(2H,m),7.17(1H,t,J=8.1Hz),7.22(1H,dd,J=8.1,1.8Hz),7.35( 1H,d,J=8.5Hz),7.38(1H,s),7.40-7.42(1H,m).

### Example 339

2-(2-{2-[(trans)-8-Chloro-6-(2,3-dimethoxyphenyl)-1-(trifluoro)-4H,6H-[1,2,4]triazolo[4,3-a][4,1]-benzoxazepin-4-yl]propanoyl}-1,3-thiazol-5-yl)acetic acid

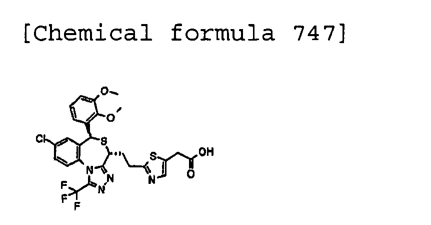

Ethyl 2-(2-{2-[(trans)-8-chloro-6-(2,3-dimethoxyphenyl)-1-(trifluoro)-4H,6H-[1,2,4]triazolo[4,3-a][4,1]-benzoxazepin-4-yl]propanoyl}-1,3-thiazol-5-yl)acetate (19 mg) was dissolved in methanol-water-tetrahydrofuran (1:0.5:1, 2.5 mL). To the resulting solution was added lithium hydroxide monohydrate (2.5 mg). The resulting mixture was stirred at room temperature for 18 hours. The reaction mixture was neutralized with an acidic resin (Amberlite IR-120B). The resin was filtered out and the residue was washed with methanol. After concentration of the filtrate, the residue thus obtained was purified by preparative TLC (methanol:chloroform=1:9) to give the title compound (13 mg).
MS (ESI) m/z: 611 (M⁺+H) . ¹H-NMR(CDCl₃)δ:2.38-2.40(1H,m),2.79-2.82(1H,m),3.08-3.11(2H,m),3.39(3H,s),3.60-3.63(2H,m),3.83(3H,s),3.90-3.92(1H,m),5.29(1H,s),6.94-6.95(2H,m),7.13-7.20(3H,m),7.37-7.40(2H,m).

### Example 340

Methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate

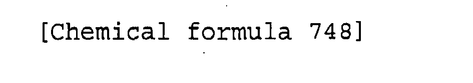

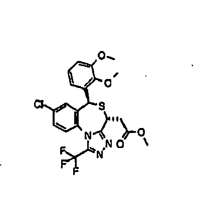

Methyl 2-[(trans)-7-chloro-5-(2,3-dimethoxyphenyl)-2-thioxo-1,2,3,5-tetrahydro-4,1-benzothiazepin-3-yl]acetate (438 mg) was dissolved in tetrahydrofuran (10 mL). To the resulting solution was added hydrazine monohydrate (0.194 mL). The resulting mixture was stirred at room temperature for 2 hours. After concentration of the reaction mixture, the residue was dissolved in methylene chloride (10 mL). To the resulting solution were added trifluoroacetic anhydride (0.395 mL) and trifluoroacetic acid (15.41 mL). The resulting mixture was stirred at room temperature for 15 minutes. The reaction mixture was stirred at 55°C for one hour. Toluene (10 mL) was added, followed by heating under reflux for further one hour. The reaction mixture was cooled to room temperature and then concentrated. The residue was diluted with ethyl acetate and washed with a saturated aqueous solution of sodium bicarbonate and saturated brine. The organic layer was dried over anhydrous sodium sulfate. After concentration of the filtrate, the residue thus obtained was purified by preparative TLC (ethyl acetate:n-hexane=1:2) to give the title compound (226 mg).
MS(ESI)m/z:514(M⁺+H).
¹H-NMR(CDCl₃)δ:2.92(1H,dd,J=17.2,5.6Hz),3.43(3H,s),3.64(1H,dd, J=17.2,8.9Hz),3.71(3H,s),3.85(3H,s),4.31(1H,dd,J=8.9,5.6Hz) ,5.35(1H,s),6.95-7.00(2H,m),6.97-6.99(2H,m),7.15-7.22(2H,m),7.38(1H,d,J=8.5Hz),7.42-7.46(1H,m).

### Example 341

2-[8-Chloro-6-(2,3-dimethoxyphenyl)-1-(trifluoromethyl)-4H,6H-[1,2,4]triazolo[4,3-a][4,1]benzothiazepin-4-yl]acetic acid

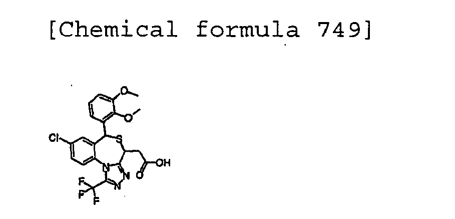

To a solution of methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate (226 mg) in methanol (10 mL) and water (5 mL) was added potassium carbonate (182 mg). The resulting mixture was stirred at room temperature for 12 hours. To the reaction mixture was added an ion exchange resin IR-120B to adjust its pH to approximately 3.0. The resin was then filtered out and the residue was concentrated to give the title compound (219 mg).
MS(ESI)m/z:500(M⁺+H). ¹H-NMR(CDCl₃)δ:2.93-3.07(1H,m),3.30-3.44(3H,m),3.61-3.73(1H,m),3.75-3.88(3H,m),4.24-4.29(1H,m),5.34-5.40(1H,m),6.74-7.01(2H,m),7.14-7.23(2H,m),7.36-7.80(2H,m).

### Example 342

Ethyl 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetate

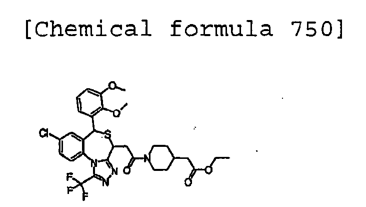

To a solution of 2-[8-chloro-6-(2,3-dimethoxyphenyl)-1-(trifluoromethyl)-4H,6H-[1,2,4]triazolo[4,3-a][4,1]benzothiazepin-4-yl]acetic acid (390 mg) in dichloromethane (5.0 mL) were added ethyl 2-(4-piperidinyl)acetate (200 mg), 1-ethyl-3-(3-dimethylaminopropyl)carbodiimide hydrochloride (299 mg), and 1-hydroxybenzotriazole monohydrate (119 mg). The resulting mixture was stirred at room temperature for one hour. Triethylamine (0.218 mL) was added, followed by stirring for further 2 hours. After concentration of the reaction mixture, silica gel chromatography (ethyl acetate:n-hexane=1:1 Rf=0.45) was performed to give the title compound (238 mg).
MS (ESI) m/z : 652 (M⁺+H) . ¹H-NMR(CDCl₃)δ:1.06-1.49(6H,m),1.69-1.90(2H,m),2.00-2.10(1H,m),2.22-2.29(2H,m),2.56-2.66(1H,m),2.77-2.93(1H,m),3.04-3.23(1H,m),3.29-3.43(3H,m),3.74-3.86(3H,m),3.94-4.17(3H,m),4.46-4.53(2H,m),5.32-5.41(1H,m),6.59-7.79(6H,m).

### Example 343

2-(1-{2-[8-Chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetic acid

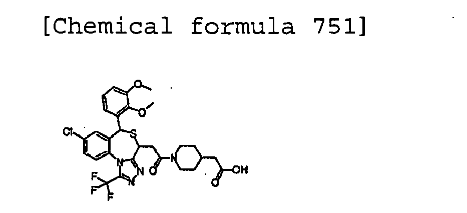

To a solution of ethyl 2-(1-{2-[8-chloro-6-(2,3-dimethoxyphenyl)-1-(trifluoromethyl)-4H,6H-[1,2,4]triazolo[4,3-a] [4,1]benzothiazepin-4-yl]acetyl}-4-piperidinyl)acetate (238 mg) in methanol (10 mL) and water (5 mL) was added potassium carbonate (151 mg). The resulting mixture was stirred at room temperature for 16 hours. After the reaction mixture was adjusted to approximately pH 3 with an ion exchange resin IR-120B, the resin was filtered out and the residue was concentrated. The residue was purified by preparative reversed phase thin layer chromatography (methanol/water=4/1), followed by purification by silica gel chromatography (15% methanol-chloroform) and washing with a solvent mixture of methylene chloride and n-hexane to give the title compound isomer A (90 mg) and the title compound isomer B (98 mg), respectively.
Isomer A
MS (ESI) m/z : 625 (M⁺+H).
¹H-NMR(CDCl₃)δ:1.08-1:28(2H,m),1.68-1.95(3H,m),2.16-2.29(2H,m),2.53-2.63(1H,m),2.78-2.92(1H,m),3.06-3.24(1H,m),3.30(3H,s),3.37-3.47(1H,m),3.75(3H,s),3.96-4:08(1H,m),4.39-4.50(2H,m),5.40(1H,s),6.56-6.61(1H,m),6.74-6.86(2H,m),7.12-7.22(1H,m),7.40-7.45(1H,m),7.77-7.81(1H,m).
Elemental analysis for: C₂₈H₂₈ClF₃N₄O₅S·0.75H₂O·0.25CHCl₃
Calculated: C,50.74;H,4.52;N,8.38.
Found: C,50.46;H,4.44;N,8.20.
Isomer B
MS(ESI)m/z:625(M⁺+H).
¹H-NMR(CDCl₃)b:1.08-1.28(2H,m),1.68-2.04(3H,m),2.19-2.31(2H,m),2.53-2.63(1H,m),2.75-2.87(1H,m),3.02-3.18(1H,m),3.41(3H,s),3.61-3.74(1H,m),3.84(3H,s),3.92-4.01(1H,m),4.43-4.50(2H,m),5.31(1H,s),6.96-6.99(2H,m),7.16(1H,t,J=8.1Hz),7.21-7.25(1H,m),7.40-7.43(2H,m).
Elemental analysis for: C₂₈H₂₈ClF₃N₄O₅S·0.75H₂O
Calculated: C,52.67;H,4.66;N,8.77.
Found: C,52.33;H,4.43;N,8.68.

### Example 344

2-(1-{3-[(4R,6S)-8-Chloro-6-(2,3-dimethoxyphenyl)-4H,6H-pyrrolo[1,2-a] [4,1]benzoxazepin-4-yl]propanoyl}-4-piperidinyl)-N,N-dimethylacetamide

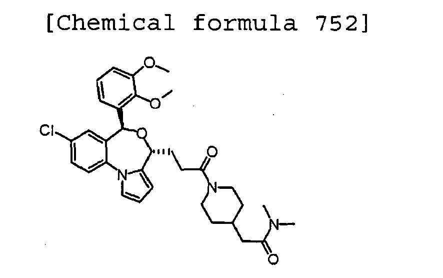

To a methanol solution (20 mL) of 2-(1-{3-[8-Chloro-6-(2,3-dimethoxyphenyl)-4H,6H-pyrrolo[1-,2-a][4,1]benzoxazepin-4-yl]propanoyl}-4-piperidinyl)acetic acid (103 mg) were added a 50% aqueous dimethylamine solution (34 µl) and 4-(4,6-dimethoxy-1,3,5-triazine-2-yl)-4-methylmorpholinium chloride n-hydrate (206 mg). The resulting mixture was stirred at room temperature for 12 hours. After the solvent was distilled off, ethyl acetate was added. The resulting mixture was washed with a 10% aqueous citric acid solution and dried over anhydrous sodium sulfate. The residue obtained by distilling off the solvent was purified by preparative thin layer chromatography on silica gel (ethyl acetate) and lyophilized from dioxane to give the title compound (108 mg).
MS (ESI) m/z : 669 (M + H)⁺.
¹H-NMR (CDCl₃) δ: 0.98-1.16 (2H, m), 1.72-1.87 (2H, m), 2.15-2.73 (7H, m), 2.94-3.08 (8H, m), 3.43 (3H, s), 3.83-3.94 (4H, m), 4.36-4.42 (1H, m), 4.61 (1H, d, J = 13.2 Hz), 5.69-5.71 (1H, m), 6.34-6.39 (2H, m), 6.70 (1H, d, J = 1.7 Hz), 6.95 (1H, dd, J = 8.2, 1.6 Hz), 7.07-7.09 (1H, m), 7.18 (1H, t, J = 8.1 Hz), 7.27-7.30 (1H, m), 7.34-7.35 (2H, m).
Elemental analysis for: C₃₂H₃₈ClN₃O₅·0.75H₂O
Calculated: C, 64.75; H, 6.71; N, 7.08.
Found: C, 64.96; H, 6.70; N, 6.72.

### Referential Example 236

4-(tert-Butyl) 2-methyl 2,4-morpholine dicarboxylate

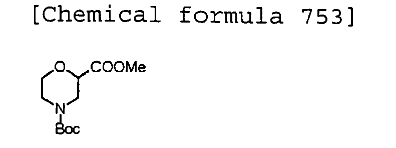

To a solution of 4-(tert-butoxycarbonyl)-2-morpholine dicarboxylic acid (231 mg) in dimethylformamide (2.0 mL) were added methyl iodide (125 µl) and potassium carbonate (152 mg). The resulting mixture was stirred at room temperature for 24 hours. To the reaction mixture was added a 10% aqueous citric acid solution. The resulting mixture was extracted with ethyl acetate. After the organic layer was dried over anhydrous sodium sulfate, the solvent was distilled off to give the title compound (232 mg).
MS (ESI) m/z : 246 (M + H)⁺.
¹H-NMR (CDCl₃) δ: 1.47 (9H, s), 3.02-3.18 (2H, m), 3.58 (1H, td, J = 11.1, 2.6 Hz), 3.74-3.80 (4H, m), 3.98-4.14 (3H, m).

### Referential Example 237

Methyl 2-morpholine carboxylate hydrochloride

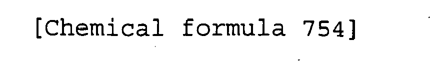

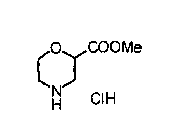

To a solution of 4-(tert-butyl) 2-methyl 2,4-morpholine dicarboxylate (232 mg) in dioxane (1.0 mL) was added 4N hydrochloric acid-dioxane (1.18 mL). The resulting mixture was stirred at room temperature for 15 hours. The solvent was distilled off under reduced pressure to give the title compound (232 mg).
¹H-NMR (CDCl₃) δ: 3.03-3.19 (2H, m), 3.26-3.45 (2H, m), 3.55-3.69 (1H, m), 3.82 (3H, s), 4.03-4.22 (2H, m), 4.58-4.69 (1H, m).

### Example 345

Methyl 4-{3-[(4R,6S)-8-chloro-6-(2,3-dimethoxyphenyl)-4H,6H-pyrrolo[1,2-a][4,1]benzoxazepin-4-yl]propanoyl}-2-morpholine carboxylate

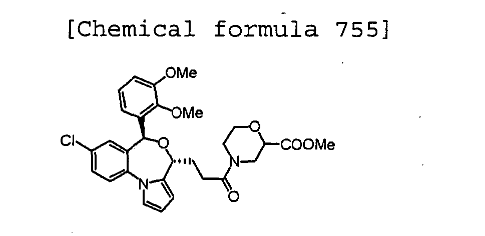

To a solution of 3-[(4R,6S)-8-chloro-6-(2,3-dimethoxyphenyl)-4H,6H-pyrrolo[1,2-a][4,1]benzoxazepin-4-yl]propanoic acid (86 mg) in dichloromethane (5.0 mL) were added methyl 2-morpholine carboxylate hydrochloride (44 mg), 1-ethyl-3-(3-dimethylaminopropyl)carbodiimide hydrochloride (77 mg), 1-hydroxybenzotriazole (31 mg), and triethylamine (33 µl). The resulting mixture was stirred at room temperature for 18 hours. The reaction mixture was purified by preparative thin layer chromatography on silica gel (development phase: 5% methanol-chloroform) to give the title compound (119 mg).
MS (ESI) m/z : 555 (M + H)⁺.
¹H-NMR (CDCl₃) δ: 2.31-4.64 (21H, m), 5.70-5.74 (1H, m), 6.33-6.42 (2H, m), 6.70-6.75 (1H, m), 6.94-7.41 (6H, m).

### Example 346

4-{3-[(4R,6S)-8-Chloro-6-(2,3-dimethoxyphenyl)-4H,6H-pyrrolo[1,2-a][4,1]benzoxazepin-4-yl]propanoyl}-2-morpholine carboxylic acid

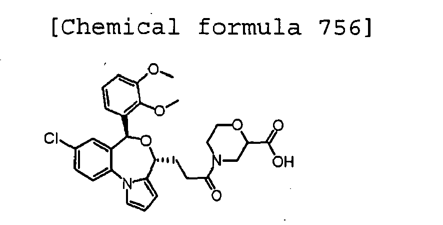

To a methanol solution(10 mL) of methyl 4-{3-[(4R,6S)-8-chloro-6-(2,3-dimethoxyphenyl)-4H,6H-pyrrolo[1,2-a] [4,1]benzoxazepin-4-yl]propanoyl}-2-morpholine carboxylate (154 mg) were added distilled water (5 mL) and potassium carbonate (115 mg). The resulting mixture was stirred at 50°C for 3 hours. After addition of a 10% aqueous citric acid solution, the resulting mixture was extracted with ethyl acetate. The organic layer was dried over sodium sulfate. The residue obtained by distilling off the solvent was solidified with an isopropyl ether-n-hexane solvent mixture to give the title compound (70 mg).
MS (ESI) m/z : 541 (M + H)⁺.
¹H-NMR (CDCl₃) δ: 2.37-2.46 (2H, m), 2.55-2.79 (2H, m), 3.43 (3H, s), 3.56-3.72 (1H, m), 3.81-3.88 (4H, m), 3.99-4.22 (4H, m), 4.39-4.44 (1H, m), 5.71 (1H, s), 6.34-6.40 (2H, m), 6.69-6.74 (1H, m), 6.93-6.97 (1H, m), 7.08-7.11 (1H, m), 7.13-7.22 (1H, m), 7.27-7.32 (1H, m), 7.34-7.41 (2H, m).
Elemental analysis for: C₂₈H₂₉ClN₂O₇·1.25H₂O·0.25 diisopropyl ether
Calculated: C, 60.15; H, 5.99; N, 4.76.
Found: C, 60.14; H, 5.75; N, 4.57.

### Referential Example 238

Ethyl 2-(1-benzyl-4-hydroxy-4-piperidinyl)acetate

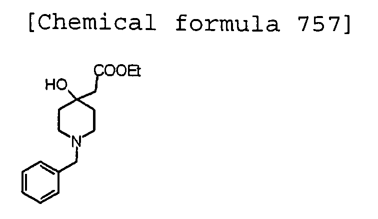

To a solution of ethyl acetate (180 µl) in tetrahydrofuran (10.0 mL) was added dropwise a 1.8M lithium diisopropylamide/tetrahydrofuran solution (0.67 mL) at -78°C under a nitrogen gas stream. The resulting mixture was stirred for 0.5 hour. The reaction mixture was added dropwise to a solution of 1-benzyl-4-piperidinone (185 µl) in tetrahydrofuran (5.0 mL) at -78°C under a nitrogen gas stream. The reaction mixture was stirred at the same temperature for 3 hours. Saturated aqueous ammonium chloride was added and the resulting mixture was extracted with ethyl acetate. The organic layer was washed with saturated brine and dried over anhydrous sodium sulfate. The solvent was then distilled off. The residue thus obtained was purified by silica gel column chromatography (ethyl acetate) to give the title compound (263 mg).
MS (ESI) m/z : 278 (M + H)⁺.

### Referential Example 239

Ethyl 2-(4-hydroxy-4-piperidinyl)acetate

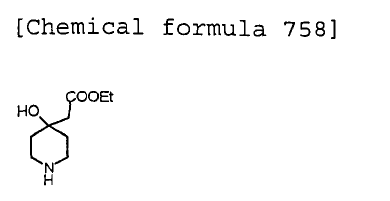

To a solution of ethyl 2-(1-benzyl-4-hydroxy-4-piperidinyl)acetate (263 mg) in ethanol (10.0 mL) was added 10% palladium-carbon (200 mg). The resulting mixture was subjected to catalytic hydrogenation at 5 atmospheres for 12 hours. The catalyst was filtered out and the solvent was distilled off under reduced pressure to give the title compound (182 mg).
MS (ESI) m/z : 188 (M + H)⁺.
¹H-NMR (CDCl₃) δ: 1.28 (3H, t, J = 7.1 Hz), 1.54-1.74 (4H, m), 2.82-2.90 (2H, m), 3.05 (2H, t, J = 11.6 Hz), 3.34-3.44 (2H, m), 4.13-4.22 (2H, m).

### Example 347

Ethyl 2-(1-{3-[(4R,6S)-8-chloro-6-(2,3-dimethoxyphenyl)-4H,6H-pyrrolo[1,2-a][4,1]benzoxazepin-4-yl]propanoyl}-4-hydroxy-4-piperidinyl)acetate

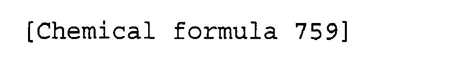

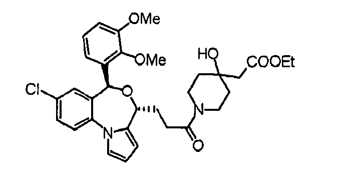

To a solution of 3-[(4R,6S)-8-chloro-6-(2,3-dimethoxyphenyl)-4H,6H-pyrrolo[1,2-a][4,1]benzoxazepin-4-yl]propanoic acid (64 mg) in dichloromethane (5.0 mL) were added ethyl 2-(4-hydroxy-4-piperidinyl)acetate (34 mg), 1-ethyl-3-(3-dimethylaminopropyl)carbodiimide hydrochloride (58 mg), and 1-hydroxybenzotriazole (23 mg). The resulting mixture was stirred at room temperature for 13 hours. The reaction mixture was purified by preparative thin layer chromatography on silica gel (development phase: 5% methanol-chloroform) to give the title compound (70 mg).
MS (ESI) m/z : 597 (M + H)⁺.
¹H-NMR (CDCl₃) δ: 1.28 (3H, t, J = 7.2 Hz), 1.38-1.49 (2H, m), 2.35-2.70 (7H, m), 2.99-3.10 (1H, m), 3.42-3.51 (4H, m), 3.63-3.72 (2H, m), 3.85 (3H, s), 4.18 (2H, q, J = 7.2 Hz), 4.32-4.42 (2H, m), 5.70 (1H, d, J = 2.9 Hz), 6.34-6.39 (2H, m), 6.70 (1H, s), 6.95 (1H, dd, J = 8.1, 1.5 Hz), 7.07-7.09 (1H, m), 7.17 (1H, td, J = 8.0, 2.5 Hz), 7.27-7.30 (1H, m), 7.34-7.36 (2H, m).

### Example 348

2-(1-{3-[(4R,6S)-8-Chloro-6-(2,3-dimethoxyphenyl)-4H,6H-pyrrolo[1,2-a][4,1]benzoxazepin-4-yl]propanoyl}-4-hydroxy-4-piperidinyl)acetic acid

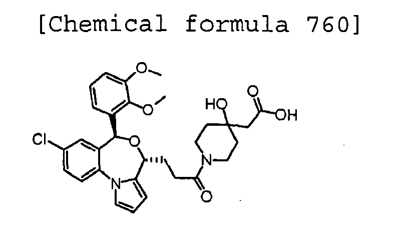

To a methanol solution (5.0 mL) of ethyl 2-(1-{3-[(4R,6S)-8-chloro-6-(2,3-dimethoxyphenyl)-4H,6H-pyrrolo[1,2-a][4,1]benzoxazepin-4-yl]propanoyl}-4-hydroxy-4-piperidinyl)acetate (70 mg) were added distilled water (2.5 mL) and potassium carbonate (49 mg). The resulting mixture was stirred at room temperature for 3 days. The reaction mixture was adjusted to pH 4.0 with ion exchange resin IR-120B. The resin was filtered out and the solvent was distilled off. The residue thus obtained was purified by preparative thin layer chromatography on silica gel (development phase: a 7:3:1=chloroform:methanol:water organic layer), followed by lyophilization from 1,4-dioxane-water to give the title compound (67 mg).
MS (ESI) m/z : 569 (M + H)⁺.
¹H-NMR (CDCl₃) δ: 1.19-1.39 (2H, m), 1.50-1.69 (2H, m), 1.92-2.62 (6H, m), 2.88-3.02 (1H, m), 3.30-3.54 (5H, m), 3.78-3.81 (3H, m), 4.00-4.11 (1H, m), 4.27-4.33 (1H, m), 5.65 (1H, d, J = 3.2 Hz), 6.27 (2H, s), 6.66-6.68 (1H, m), 6.89 (1H, d, J = 8.1 Hz), 6.98-7.01 (1H, m), 7.13 (1H, t, J = 7.8 Hz), 7.22-7.30 (3H, m).
Elemental analysis for: C₃₀H₃₃ClN₂O₇·1.0H₂O·0.5CHCl₃
Calculated: C, 56.60; H, 5.61; N, 4.33.
Found: C, 56.50; H, 5.53; N, 4.29.

### Referential Example 240

1-(tert-Butyl) 4-ethyl 4-hydroxy-1,4-piperidinedicarboxylate

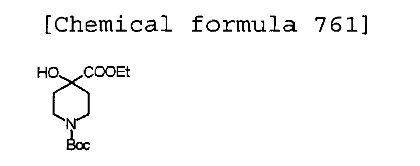

To a solution of 1-(tert-butyl) 4-ethyl 1,4-piperidinedicarboxylate (637 mg) in tetrahydrofuran (2.5 mL) was added a 1.8M lithium diisopropylamidetetrahydrofuran solution (2.06 mL) at -78°C over 15 minutes. The reaction mixture was stirred for 30 minutes under ice cooling. A solution of N-fluorobenzenesulfonamide (1.33 g) in tetrahydrofuran (3.75 mL) was stirred at the same temperature for 30 minutes and then at room temperature for 18 hours. To the reaction mixture was added ethyl acetate. The resulting mixture was washed with saturated brine and dried over anhydrous sodium sulfate. The solvent was distilled off under reduced pressure. The residue thus obtained was purified by silica gel chromatography (ethyl acetate:n-hexane= from 1:1 to 1:0) to give the title compound (497 mg).
MS (ESI) m/z : 274 (M + H)⁺.
¹H-NMR (CDCl₃) δ: 1.30 (3H, t, J = 7.2 Hz), 1.47 (9H, s), 1.54-1.60 (2H, m), 1.89-2.00 (2H, m), 3.03-3.22 (3H, m), 3.82-4.08 (2H, m), 4.25 (2H, q, J = 7.1 Hz).

### Referential Example 241

Ethyl 4-hydroxy-4-piperidinedicarboxylate hydrochloride

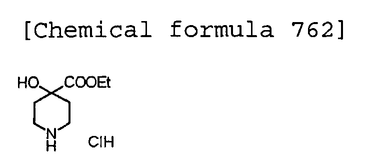

To 1-(tert-butyl) 4-ethyl 4-hydroxy-1,4-piperidinedicarboxylate (497 mg) was added 4N hydrochloric acid-dioxane (2.41 mL). The resulting mixture was stirred at room temperature for 4 hours. The solvent was distilled off under reduced pressure to give the title compound (473 mg).
MS (ESI) m/z : 174 (M + H)⁺.
¹H-NMR (CD₃OD) δ: 1.30 (3H, t, J = 7.2 Hz), 1.89-2.00 (2H, m), 2.15-2.27 (2H, m), 3.27-3.34 (4H, m), 4.23 (2H, q, J = 7.1 Hz) .

### Example 349

Ethyl 1-{3-[(4R,6S)-8-chloro-6-(2,3-dimethoxyphenyl)-4H,6H-pyrrolo[1,2-a][4,1]benzoxazepin-4-yl]propanoyl}-4-hydroxy-4-piperidine carboxylate

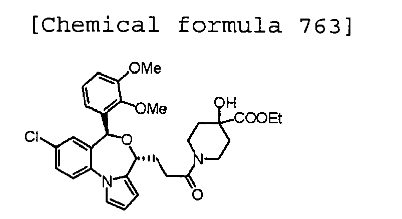

To a solution of 3-[(4R,6S)-8-chloro-6-(2,3-dimethoxyphenyl)-4H,6H-pyrrolo[1,2-a] [4,1]benzoxazepin-4-yl]propanoic acid (69 mg) in dichloromethane (5.0 mL) were added ethyl 4-hydroxy-4-piperidinedicarboxylate hydrochloride (38 mg), 1-ethyl-3-(3-dimethylaminopropyl)carbodiimide hydrochloride (58 mg), 1-hydroxybenzotriazole (23 mg), and triethylamine (25 µl). The resulting mixture was stirred at room temperature for 13 hours. The reaction mixture was purified by preparative thin layer chromatography on silica gel (development phase: 5% methanol-chloroform) to give the title compound (65 mg). MS (ESI) m/z : 585 (M + H)⁺.
¹H-NMR (CDCl₃) δ: 1.29 (3H, t, J = 7.2 Hz), 1.58-1.65 (3H, m), 1.87-1.98 (2H, m), 2.36-2.45 (2H, m), 2.53-2.63 (1H, m), 2.67-2.75 (1H, m), 2.96-3.05 (1H, m), 3.43 (3H, s), 3.75-3.86 (4H, m), 4.20-4.28 (2H, m), 4.40-4.52 (2H, m), 5.71 (1H, s), 6.35-6.39 (2H, m), 6.71 (1H, d, J = 1.5 Hz), 6.94 (1H, dd, J = 8.2, 1.3 Hz), 7.08-7.09 (1H, m), 7.17 (1H, td, J = 8.0, 1.6 Hz), 7.29 (1H, dd, J = 7.8, 1.2 Hz), 7.34-7.36 (2H, m).

### Example 350

1-{3-[(4R,6S)-8-Chloro-6-(2,3-dimethoxyphenyl)-4H,6H-pyrrolo[1,2-a][4,1]benzoxazepin-4-yl]propanoyl}-4-hydroxy-4-piperidine carboxylic acid

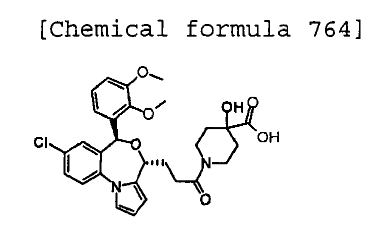

To a methanol solution (5.0 mL) of ethyl 1-{3-[(4R,6S)-8-chloro-6-(2,3-dimethoxyphenyl)-4H,6H-pyrrolo[1,2-a][4,1]benzoxazepin-4-yl]propanoyl}-4-hydroxy-4-piperidine carboxylate (84 mg) were added distilled water (2.5 mL) and potassium carbonate (60 mg). The resulting mixture was stirred at room temperature for 17 hours. The reaction mixture was adjusted to pH 4.0 with an ion exchange resin IR-120B. The resin was filtered out and the solvent was distilled off. The residue was purified by preparative thin layer chromatography on silica gel (development phase: an organic layer of chloroform:methanol:water=7:3:1), followed by lyophilization from 1,4-dioxane-water to give the title compound (69 mg).
MS (ESI) m/z : 555 (M + H)⁺.
¹H-NMR (CDCl₃) δ: 1.34-1.92 (4H, m), 2.14-2.85 (4H, m), 3.11-3.59 (6H, m), 3.73-3.78 (3H, m), 4.11-4.32 (2H, m), 5.58-5.65 (1H, m), 6.20-6.29 (2H, m), 6.62-6.68 (1H, m), 6.81-6.88 (1H, m), 6.93-7.13 (2H, m), 7.19-7.27 (3H, m). Elemental analysis for: C₂₉H₃₁ClN₂O₇·1.25H₂O·0.25CHCl₃ Calculated: C, 55.56; H, 5.45; N, 4.39.
Found: C, 55.42; H, 5.45; N, 4.25.

### Referential Example 242

Benzyl 4-(2-methoxy-2-oxoethoxy)-1-piperidine carboxylate

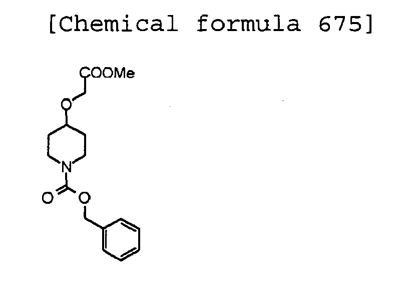

To a solution of phenylmethyl 4-({2-[(1,1-dimethylethyl)oxy]-2-oxoethyl}oxy)-1-piperidine carboxylate (260 mg) in dichloromethane (10.0 mL) was added trifluoroacetic acid (5.0 mL). The resulting mixture was stirred at room temperature for 2 hours. After concentration of the reaction mixture, N,N-dimethylformamide (10.0 mL) was added to the residue, followed by the addition of potassium carbonate (113 mg) and methyl iodide (93 µl). The resulting mixture was stirred at room temperature for 14 hours. Saturated brine was added and the resulting mixture was extracted with ethyl acetate. The organic layer was dried over anhydrous sodium sulfate. The solvent was distilled off under reduced pressure. The residue thus obtained was purified by silica gel column chromatography (ethyl acetate:n-hexane=1:4) to give the title compound (219 mg).
MS (ESI) m/z : 308 (M + H)⁺.
¹H-NMR (CDCl₃) δ: 1.55-1.66 (2H, m), 1.80-1.92 (2H, m), 3.17-3.25 (2H, m), 3.55-3.62 (1H, m), 3.75 (3H, s), 3.80-3.89 (2H, m), 5.12 (2H, s), 7.29-7.37 (5H, m).

### Referential Example 243

Methyl 2-(4-piperidinyloxy)acetate

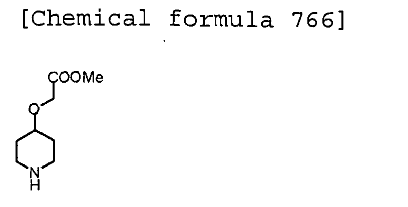

To a solution of benzyl 4-(2-methoxy-2-oxoethoxy)-1-piperidine carboxylate (219 mg) in methanol (20.0 mL) was added 10% palladium-carbon (200 mg). The resulting mixture was subjected to catalytic hydrogenation at normal pressure for 7 hours. The catalyst was filtered out and the solvent was distilled off under reduced pressure to give the title compound (117 mg).
MS (ESI) m/z : 173 (M + H)⁺.

### Example 351

Methyl 2-[(1-{3-[(4R,6S)-8-chloro-6-(2,3-dimethoxyphenyl)-4H,6H-pyrrolo[1,2-a][4,1]benzoxazepin-4-yl]propanoyl}-4-piperidinyl)oxy]acetate

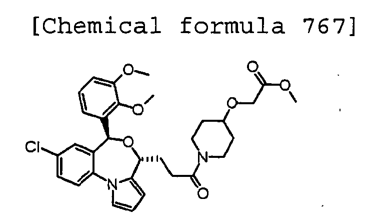

To a solution of 3-[(4R,6S)-8-chloro-6-(2,3-dimethoxyphenyl)-4H,6H-pyrrolo[1,2-a][4,1]benzoxazepin-4-yl]propanoic acid (86 mg) in dichloromethane (2.0 mL) were added methyl 2-(4-piperidinyloxy)acetate (52 mg), 1-ethyl-3-(3-dimethylaminopropyl)carbodiimide hydrochloride (77 mg), and 1-hydroxybenzotriazole (31 mg). The resulting mixture was stirred at room temperature for 17 hours. The reaction mixture was purified by preparative thin layer chromatography on silica gel (development phase: ethyl acetate:n-hexane=2:1) to give the title compound (126 mg). MS (ESI) m/z : 583 (M + H)⁺.
¹H-NMR (CDCl₃) δ: 1.53-1.90 (4H, m), 2.35-2.43 (2H, m), 2.52-2.60 (1H, m), 2.65-2.74 (1H, m), 3.20-3.35 (2H, m), 3.43 (3H, s), 3.59-3.65 (1H, m), 3.68-3.78 (4H, m), 3.84-4.03 (4H, m), 4.10-4.15 (3H, m), 4.37-4.43 (1H, m), 5.69-5.72 (1H, m), 6.34-6.39 (2H, m), 6.70 (1H, s), 6.95 (1H, dd, J = 8.3, 1.5 Hz), 7.08 (1H, dd, J = 2.7, 1.5 Hz), 7.18 (1H, td, J = 8.0, 2.1 Hz), 7.26-7.32 (1H, m), 7.33-7.37 (2H, m).

### Example 352

2-[(1-{3-[(4R,6S)-8-Chloro-6-(2,3-dimethoxyphenyl)-4H,6H-pyrrolo[1,2-a][4,1]benzoxazepin-4-yl]propanoyl}-4-piperidinyl)oxy]acetic acid

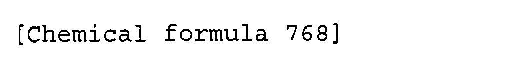

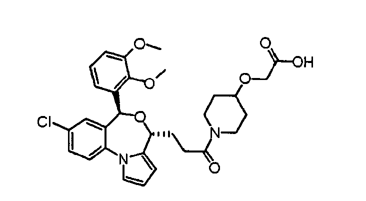

To a methanol solution (20.0 mL) of methyl 2-[(1-{3-[(4R,6S)-8-chloro-6-(2,3-dimethoxyphenyl)-4H,6H-pyrrolo[1,2-a] [4,1]benzoxazepin-4-yl]propanoyl}-4-piperidinyl)oxy]acetate (126 mg) were added distilled water (10.0 mL) and potassium carbonate (90 mg). The resulting mixture was stirred at room temperature for 15 hours. The reaction mixture was adjusted to pH 4.0 with an ion exchange resin IR-120B. The resin was filtered out and the solvent was distilled off. The residue thus obtained was purified by preparative thin layer chromatography on silica gel (development phase: chloroform:methanol:water=7:3:1 organic layer), followed by lyophilization from 1,4-dioxane-water to give the title compound (110 mg).
MS (ESI) m/z : 569 (M + H)⁺.
¹H-NMR (CDCl₃) δ: 1.42-1.56 (2H, m), 1.78-1.91 (2H, m), 2.65-2.32 (5H, m), 2.94-3.18 (2H, m), 3.49-3.59 (1H, m), 3.71 (3H, s), 3.83 (3H, s), 3.97-4.14 (3H, m), 4.36-4.40 (1H, m), 5.69 (1H, s), 6.32-6.36 (2H, m), 6.69 (1H, s), 6.91-6.95 (1H, m), 7.05-7.08 (1H, m), 7.16 (1H, t, J = 8.1 Hz), 7.27-7.30 (1H, m), 7.32-7.35 (2H, m).
Elemental analysis for: C₃₀H₃₃ClN₂O₇·1.0H₂O·0.25CHCl₃
Calculated: C, 58.87; H, 5.80; N, 4.54.
Found: C, 58.73; H, 5.65; N, 4.48.

### Referential Example 244

Benzyl 4-{[2-(tert-butoxy)-2-oxoethoxy]methyl}-1-piperidine carboxylate

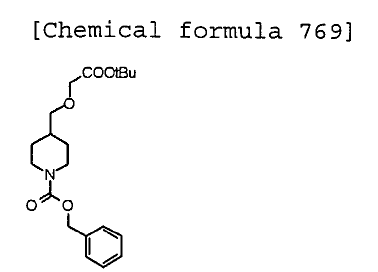

To a solution of phenylmethyl 4-(hydroxymethyl)-1-piperidine carboxylate (499 mg) in toluene (3.0 mL) were added dropwise an aqueous solution (0.5 mL) of tetrabutylammonium hydrogen sulfate (27 mg), tert-butyl bromoacetate (443 µl) and an aqueous solution (2.5 mL) of sodium hydroxide (800 mg) at room temperature. The resulting mixture was stirred vigorously for 12 hours. The organic layer was washed with saturated brine and dried over anhydrous sodium sulfate. The solvent was distilled off under reduced pressure. The residue thus obtained was purified by silica gel column chromatography (ethyl acetate:n-hexane=1:3) to give the title compound (425 mg).
MS (ESI) m/z : 364 (M + H)⁺.
¹H-NMR (CDCl₃)δ: 1.12-1.26 (2H, m), 1.48 (9H, s), 1.75-1.86 (3H, m), 2.70-2.84 (2H, m), 3.36 (2H, d, J = 6.1 Hz), 3.94 (2H, s), 4.11-4.27 (2H, m), 5.12 (2H, s), 7.28-7.37 (5H, m).

### Referential Example 245

Benzyl 4-[(2-methoxy-2-oxoethoxy)methyl]-1-piperidine carboxylate

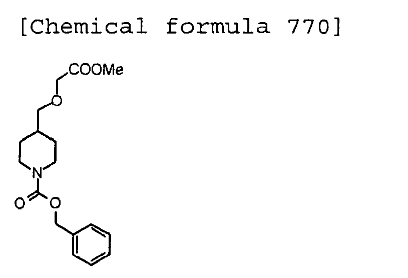

To a solution of benzyl 4-{[2-(tert-butoxy)-2-oxoethoxy]methyl}-1-piperidine carboxylate (425 mg) in dichloromethane (10.0 mL) was added trifluoroacetic acid (5.0 mL). The resulting mixture was stirred at room temperature for 2 hours. After the reaction mixture was concentrated, N,N-dimethylformamide (10.0 mL) was added to the residue, followed by the addition of potassium carbonate (178 mg) and methyl iodide (146 µl). The resulting mixture was stirred at room temperature for 14 hours. Saturated brine was added and the resulting mixture was extracted with ethyl acetate. The organic layer was dried over anhydrous sodium sulfate. The solvent was distilled off under reduced pressure. The residue thus obtained was purified by silica gel column chromatography (ethyl acetate:n-hexane=1:4) to give the title compound (365 mg).
MS (ESI) m/z : 322 (M + H)⁺.
¹H-NMR (CDCl₃) δ: 1.12-1.26 (2H, m), 1.73-1.89 (3H, m), 2.72-2.84 (2H, m), 3.37 (2H, d, J = 6.4 Hz), 3.75 (3H, s), 4.07 (2H, s), 4.12-4.26 (2H, m), 5.12 (2H, s), 7.28-7.38 (5H, m).

### Referential Example 246

Methyl 2-(4-piperidinylmethoxy)acetate

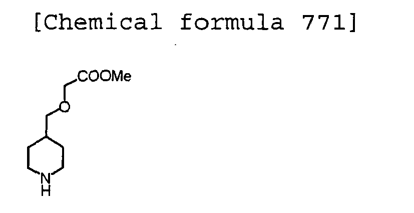

To a solution of benzyl 4-{[2-(tert-butoxy)-2-oxoethoxy]methyl}-1-piperidine carboxylate (365 mg) in methanol (20.0 mL) was added 10% palladium-carbon (300 mg). The resulting mixture was subjected to catalytic hydrogenation for 7 hours at normal pressure. The catalyst was filtered out and the solvent was distilled off under reduced pressure to give the title compound (195 mg).
MS (ESI) m/z : 188 (M + H)⁺.

### Example 353

Methyl 2-[(1-{3-[(4R,6S)-8-chloro-6-(2,3-dimethoxyphenyl)-4H, 6H-pyrrolo [1, 2-a] [4, 1] benzoxazepin-4-yl] propanoyl } -4-piperidinyl)methoxy]acetate

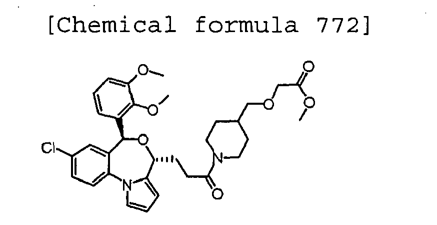

To a solution of 3-[(4R,6S)-8-chloro-6-(2,3-dimethoxyphenyl)-4H,6H-pyrrolo[1,2-a][4,1]benzoxazepin-4-yl]propanoic acid (86 mg) in dichloromethane (2.0 mL) were added methyl 2-(4-piperidinylmethoxy)acetate (56 mg), 1-ethyl-3-(3-dimethylaminopropyl)carbodiimide hydrochloride (77 mg), and 1-hydroxybenzotriazole (31 mg). The resulting mixture was stirred at room temperature for 17 hours. The reaction mixture was purified by preparative thin layer chromatography (development phase: ethyl acetate:n-hexane=2:1) to give the title compound (117 mg).
MS (ESI) m/z : 597 (M + H)⁺.
¹H-NMR (CDCl₃) δ: 1.05-1.20 (2H, m), 1.70-1.91 (3H, m), 2.35-2.43 (2H, m), 2.50-2.73 (3H, m), 2.95-3.04 (1H, m), 3.32-3.40 (2H, m), 3.43 (3H, s), 3.75 (3H, s), 3.84-3.97 (4H, m), 4.07 (2H, s), 4.37-4.42 (1H, m), 4.57-4.65 (1H, m), 5.70 (1H, s), 6.34-6.39 (2H, m), 6.69-6.71 (1H, m), 6.95 (1H, dd, J = 8.3, 1.5 Hz), 7.08 (1H, dd, J = 2.7, 1.5 Hz), 7.18 (1H, t, J = 7.9 Hz), 7.29 (1H, dd, J = 7.9, 1.0 Hz), 7.33-7.36 (2H, m).

### Example 354

2-[(1-{3-[(4R,6S)-8-Chloro-6-(2,3-dimethoxyphenyl)-4H,6H-pyrrolo[1,2-a][4,1]benzoxazepin-4-yl]propanoyl}-4-piperidinyl)methoxy]acetic acid

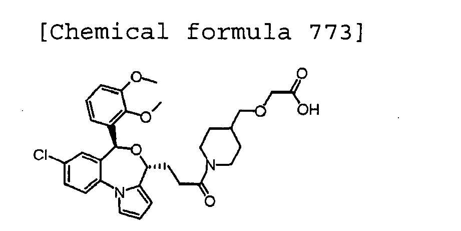

To a methanol solution (20.0 mL) of methyl 2-[(1-{3-[(4R,6S)-8-chloro-6-(2,3-dimethoxyphenyl)-4H,6H-pyrrolo [1,2-a] [4,1]benzoxazepin-4-yl]propanoyl}-4-piperidinyl)methoxy]acetate (117 mg) were added distilled water (10.0 mL) and potassium carbonate (81 mg). The resulting mixture was stirred at room temperature for 15 hours. The reaction mixture was adjusted to pH 4.0 with an ion exchange resin IR-120B. The resin was filtered out and the solvent was distilled off. The residue thus obtained was purified by preparative thin layer chromatography (development phase: a chloroform:methanol:water=7:3:1 organic layer), followed by lyophilization from 1,4-dioxane-water to give the title compound (110 mg).
MS (ESI) m/z : 583 (M + H)⁺.
¹H-NMR (CDCl₃) δ: 1.18-1.01 (2H, m), 1.70-1.91 (3H, m), 2.35-2.43 (2H, m), 2.50-2.73 (3H, m), 3.04-2.93 (1H, m), 3.38-3.30 (2H, m), 3.42 (3H, s), 3.82-4.03 (6H, m), 4.35-4.42 (1H, m), 4.54-4.63 (1H, m), 5.69 (1H, s), 6.33-6.39 (2H, m), 6.69-6.71 (1H, m), 6.92-6.96 (1H, m), 7.06-7.09 (1H, m), 7.16 (1H, t, J = 7.8 Hz), 7.28-7.30 (1H, m), 7.33-7.36 (2H, m).
Elemental analysis for: C₃₁H₃₅ClN₂O₇·0.75H₂O·0.25CHCl₃ Calculated: C, 59.89; H, 5.95; N, 4.47.
Found: C, 59.78; H, 5.84; N, 4.40.

### Referential Example 247

Benzyl 4-[3-(tert-butoxy)-3-oxopropoxyl-1-piperidine carboxylate

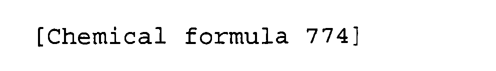

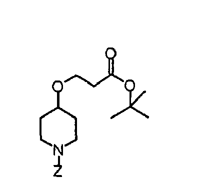

To a solution of phenylmethyl 4-hydroxy-1-piperidine carboxylate (235 mg) in 1,4-dioxane (5.0 mL) were added tert-butyl acrylate (366 µl) and tert-butoxy potassium (6 mg). The resulting mixture was stirred at room temperature for 30 minutes and then heated under reflux for 8 hours. After concentration of the reaction mixture, the residue was purified by preparative thin layer chromatography on silica gel (development phase ethyl acetate:n-hexane=1:3) to give the title compound (119 mg).
MS (ESI) m/z : 364 (M + H)⁺.
¹H-NMR (CDCl₃) δ: 1.45 (9H, s), 1.50-1.61 (2H, m), 1.70-1.84 (2H, m), 2.47 (2H, t, J = 6.3 Hz), 3.21-3.29 (2H, m), 3.46-3.53 (1H, m), 3.66-3.78 (4H, m), 5.12 (2H, s), 7.26-7.38 (5H, m).

### Referential Example 248

Benzyl 4-(3-methoxy-3-oxopropoxy)-1-piperidine carboxylate

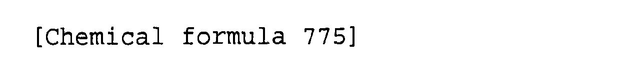

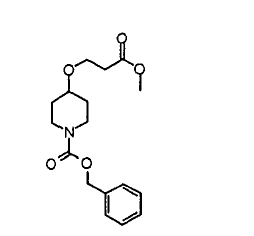

To a solution of benzyl 4-[3-(tert-butoxy)-3-oxopropoxy]-1-piperidine carboxylate (119 mg) in dichloromethane (10.0 mL) was added trifluoroacetic acid (5.0 mL). The resulting mixture was stirred at room temperature for 14 hours. After the reaction mixture was concentrated, N,N-dimethylformamide (10.0 mL) was added to the residue. To the resulting mixture were added potassium carbonate (50 mg) and methyl iodide (41 µl), followed by stirring at room temperature for 28 hours. To the reaction mixture was added saturated brine. The resulting mixture was extracted with ethyl acetate. The organic layer was dried over anhydrous sodium sulfate. The solvent was distilled off under reduced pressure. The residue was purified by silica gel column chromatography (ethyl acetate:n-hexane=1:4) to give the title compound (77 mg). MS (ESI) m/z : 322 (M + H)⁺.
¹H-NMR (CDCl₃) δ: 1.50-1.60 (2H, m), 1.75-1.85 (2H, m), 2.58 (2H, t, J = 6.3 Hz), 3.20-3.28 (2H, m), 3.47-3.53 (1H, m), 3.69-3.79 (7H, m), 5.12 (2H, s), 7.29-7.38 (5H, m).

### Referential Example 249

Methyl 3-(4-piperidinyloxy)propanoate

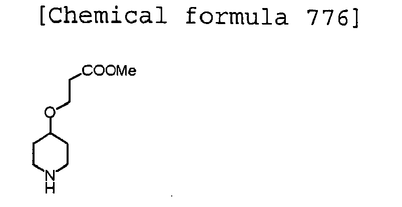

To a solution of benzyl 4-(3-methoxy-3-oxopropoxy)-1-piperidine carboxylate (77 mg) in methanol (10.0 mL) was added 10% palladium-carbon (70 mg). The resulting mixture was subjected to catalytic hydrogenation for 7 hours at normal pressure. The catalyst was filtered out and the solvent was distilled off under reduced pressure to give the title compound (43 mg).
MS (ESI) m/z : 188 (M + H)⁺.

### Example 355

Methyl 3-[(1-{3-[(4R,6S)-8-chloro-6-(2,3-dimethoxyphenyl)-4H,6H-pyrrolo[1,2-a] [4,1]benzoxazepin-4-yl]propanoyl}-4-piperidinyl)oxy]propanoate

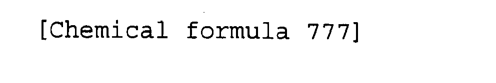

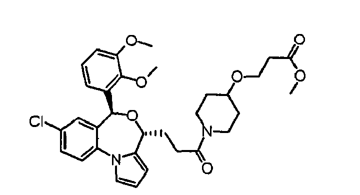

To a solution of 3-[(4R,6S)-8-chloro-6-(2,3-dimethoxyphenyl)-4H,6H-pyrrolo[1,2-a][4,1]benzoxazepin-4-yl]propanoic acid (86 mg) in dichloromethane (2.0 mL) were added methyl 3-(4-piperidinyloxy)propanoate (41 mg), 1-ethyl-3-(3-dimethylaminopropyl)carbodiimide hydrochloride (77 mg), and 1-hydroxybenzotriazole (31 mg). The resulting mixture was stirred at room temperature for 17 hours. The reaction mixture was purified by preparative thin layer chromatography on silica gel (development phase ethyl acetate:n-hexane=2:1) to give the title compound (110 mg). MS (ESI) m/z : 597 (M + H)⁺.
¹H-NMR (CDCl₃) δ: 1.05-1.21 (1H, m), 1.49-1.60 (1H, m), 1.70-1.90 (2H, m), 2.35-2.43 (2H, m), 2.51-2.72 (4H, m), 3.21-3.41 (2H, m), 3.43 (3H, s), 3.50-3.57 (1H, m), 3.60-4.08 (11H, m), 4.37-4.42 (1H, m), 5.70 (1H, s), 6.34-6.39 (2H, m), 6.70 (1H, d, J = 1.7 Hz), 6.95 (1H, dd, J = 8.2, 1.5 Hz), 7.08 (1H, dd, J = 2.9, 1.7 Hz), 7.18 (1H, t, J = 8.2 Hz), 7.27-7.32 (1H, m), 7.34-7.35 (2H, m).

### Example 356

3-[(1-{3-[(4R,6S)-8-Chloro-6-(2,3-dimethoxyphenyl)-4H,6H-pyrrolo[1,2-a][4,1]benzoxazepin-4-yl]propanoyl}-4-piperidinyl)oxy]propanoic acid

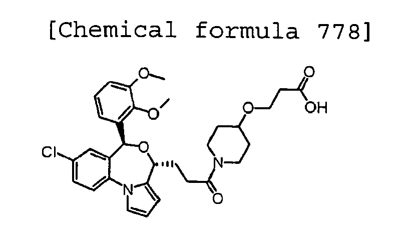

To a methanol solution (20.0 mL) of methyl 3-[(1-{3-[(4R,6S)-8-chloro-6-(2,3-dimethoxyphenyl)-4H,6H-pyrrolo[1,2-a][4,1]benzoxazepin-4-yl]propanoyl}-4-piperidinyl)oxy]propanoate (110 mg) were added distilled water (10.0 mL) and potassium carbonate (76 mg). The resulting mixture was stirred at room temperature for 15 hours. The reaction mixture was adjusted to pH 4.0 with an ion exchange resin IR-120B. The resin was filtered out and the solvent was distilled off. The residue thus obtained was purified by preparative thin layer chromatography on silica gel (chloroform:methanol:water=7:3:1 organic layer), followed by lyophilization from 1,4-dioxane-water to give the title compound (66 mg).
MS (ESI) m/z : 583 (M + H)⁺.
¹H-NMR (CDCl₃) δ: 1.51-1.59 (2H, m), 1.75-1.84 (2H, m), 2.35-2.42 (2H, m), 2.51-2.72 (4H, m), 3.21-3.38 (2H, m), 3.43 (3H, s), 3.53-3.58 (1H, m), 3.64-3.75 (3H, m), 3.83-3.89 (4H, m), 4.36-4.42 (1H, m), 5.70-5.71 (1H, m), 6.34-6.38 (2H, m), 6.70-6.71 (1H, m), 6.95 (1H, dd, J = 8.2, 1.3 Hz), 7.07-7.09 (1H, m), 7.18 (1H, t, J = 8.0 Hz), 7.28-7.35 (3H, m).
Elemental analysis for: C₃₁H₃₅ClN₂O₇·1.25H₂O
Calculated: C, 61.48; H, 6.24; N, 4.63.
Found: C, 61.44; H, 5.93; N, 4.48.

### Example 357

Methyl 3-[(4R,6S)-8-chloro-6-(2,3-dimethoxyphenyl)-1-(2H-1,2,3,4-tetrazol-5-yl)-4H,6H-pyrrolo[1,2-a] [4,1]benzoxazepin-4-yl]propanoate

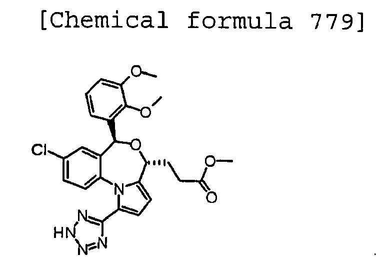

To a 1,3-dimethyl-2-imidazolidinone solution (3.0 mL) of methyl 3-[(4R,6S)-8-chloro-1-cyano-6-(2,3-dimethoxyphenyl)-4H,6H-pyrrolo[1,2-a][4,1]benzoxazepin-4-yl]propanoate (233 mg) were added triethylamine hydrochloride (344 mg) and sodium azide (130 mg). The resulting mixture was stirred at 130°C for 8 hours. After the reaction mixture was cooled to room temperature, distilled water (1.0 mL) was added thereto. An aqueous solution (0.5 mL) of sodium nitrite (207 mg) was added in portions under ice cooling. The reaction mixture was adjusted to pH from 4.5 to 5.0 with 2N hydrochloric acid and extracted with ethyl acetate. The organic layer was washed with saturated brine and dried over anhydrous sodium sulfate. The solvent was distilled off and the residue was purified by silica gel column chromatography (10% methanol-chloroform) to give the title compound (55 mg).
MS (ESI) m/z : 510 (M + H)⁺.
¹H-NMR (CDCl₃) δ: 1.22-1.27 (3H, m), 2.29-2.44 (4H, m), 2.51-2.54 (1H, m), 2.60 (1H, dd, J = 7.8, 3.4 Hz), 2.82-2.97 (1H, m), 3.42 (3H, s), 3.60-3.66 (1H, m), 3.73-3.81 (1H, m), 3.84 (3H, s), 4.08-4.16 (3H, m), 4.26 (1H, t, J = 8.5 Hz), 4.36-4.40 (1H, m), 5.69-5.71 (1H, m), 6.32-6.34 (1H, m), 6.37 (1H, t, J = 3.2 Hz), 6.70-6.72 (1H, m), 6.94 (1H, dd, J = 8.3, 1.5 Hz), 7.06-7.09 (1H, m), 7.16-7.20 (2H, m), 7.26-7.35 (2H, m).

### Example 358

3-[(4R,6S)-8-Chloro-6-(2,3-dimethoxyphenyl)-1-(2H-1,2,3,4-tetrazol-5-yl)-4H,6H-pyrrolo[1,2-a][4,1]benzoxazepin-4-yl]propanoic acid

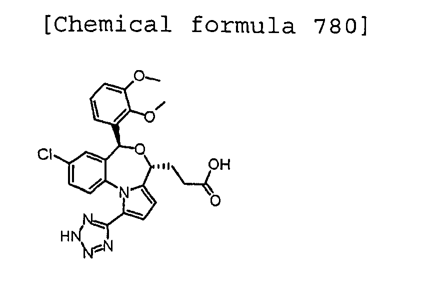

To a methanol solution (20.0 mL) of methyl 3-[(4R,6S)-8-chloro-6-(2,3-dimethoxyphenyl)-1-(2H-1,2,3,4-tetrazol-5-yl)-4H,6H-pyrrolo[1,2-a][4,1]benzoxazepin-4-yl]propanoate (95 mg) were added distilled water (10.0 mL) and potassium carbonate (77 mg). The resulting mixture was stirred at room temperature for 2 days. To the reaction mixture was added a 10% aqueous citric acid solution and the resulting mixture was extracted with ethyl acetate.
The organic layer was dried over anhydrous sodium sulfate. The solvent was distilled off to give the title compound (100 mg).
MS (ESI) m/z : 496 (M + H)⁺.
¹H-NMR (CDCl₃) δ: 2.30-2.51 (2H, m), 2.58-2.80 (2H, m), 3.57 (3H, s), 3.87 (3H, s), 4.29 (1H, dd, J = 9.5, 4.4 Hz), 5.78 (1H, s), 6.47 (1H, d, J = 3.9 Hz), 6.72 (1H, d, J = 2.4 Hz), 6.91 (1H, d, J = 8.4 Hz), 6.99 (1H, dd, J = 8.4, 1.5 Hz), 7.12 (1H, d, J = 3.9 Hz), 7.22-7.33 (3H, m).

### Example 359

Ethyl 2-(1-{3-[(4R,6S)-8-chloro-6-(2,3-dimethoxyphenyl)-1-(2H-1,2,3,4-tetrazol-5-yl)-4H,6H-pyrrolo[1,2-a] [4,1]benzoxazepin-4-yl]propanoyl}-4-piperidinyl)acetate

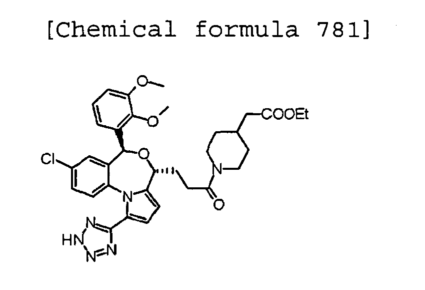

To a solution of 3-[(4R,6S)-8-chloro-6-(2,3-dimethoxyphenyl)-1-(2H-1,2,3,4-tetrazol-5-yl)-4H,6H-pyrrolo[1,2-a][4,1]benzoxazepin-4-yl]propanoic acid (100 mg) in dichloromethane (20.0 mL) were added ethyl piperidin-4-yl-acetate (69 mg), 1-ethyl-3-(3-dimethylaminopropyl)carbodiimide hydrochloride (116 mg), and 1-hydroxybenzotriazole (31 mg). The resulting mixture was stirred at room temperature for 19 hours. The reaction mixture was purified by preparative thin layer chromatography on silica gel (developed with: chloroform:methanol:water=7:3:1 organic layer) to give the title compound (133 mg).
MS (ESI) m/z : 649 (M + H)⁺.
¹H-NMR (CDCl₃) δ: 1.04-1.17 (2H, m), 1.23 (3H, t, J = 6.0 Hz), 1.48-3.10 (9H, m), 3.28-3.37 (1H, m), 3.45 (3H, s), 3.82-3.95 (4H, m), 4.10 (3H, q, J = 6.0 Hz), 4.24-4.29 (1H, m), 4.58-4.64 (1H, m), 5.83 (1H, s), 6.37 (1H, d, J = 3.4 Hz), 6.71 (1H, d, J = 2.2 Hz), 6.91-7.05 (3H, m), 7.15-7.30 (3H, m).

### Example 360

Ethyl 2-(1-{3-[(4R,6S)-8-chloro-6-(2,3-dimethoxyphenyl)-1-(2-methyl-2H-1,2,3,4-tetrazol-5-yl)-4H,6H-pyrrolo[1,2-a][4,1]benzoxazepin-4-yl]propanoyl}-4-piperidinyl)acetate (low polarity substance)

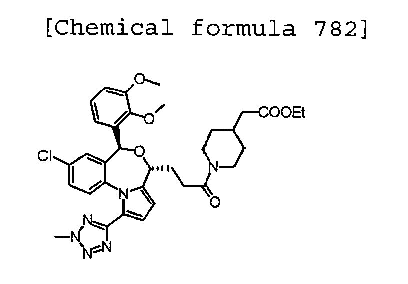

Ethyl 2-(1-{3-[(4R,6S)-8-chloro-6-(2,3-dimethoxyphenyl)-1-(1-methyl-2H-1,2,3,4-tetrazol-5-yl)-4H,6H-pyrrolo[1,2-a][4,1]benzoxazepin-4-yl]propanoyl}-4-piperidinyl)acetate (high polarity substance)

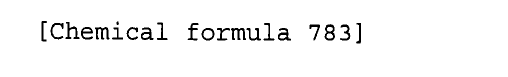

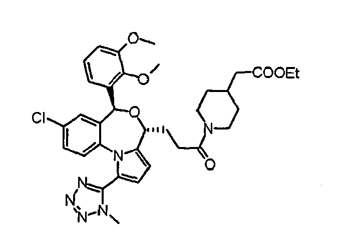

To a solution of ethyl 2-(1-{3-[(4R,6S)-8-chloro-6-(2,3-dimethoxyphenyl)-1-(2H-1,2,3,4-tetrazol-5-yl)-4H,6H-pyrrolo[1,2-a][4,1]benzoxazepin-4-yl]propanoyl}-4-piperidinyl)acetate (133 mg) in N,N-dimethylformamide (10.0 mL) were added methyl iodide (128 µl) and potassium carbonate (85 mg). The resulting mixture was stirred at room temperature for 20 hours. To the reaction mixture was added a 10% aqueous citric acid solution. The resulting mixture was extracted with ethyl acetate. The organic layer was dried over anhydrous sodium sulfate. The residue obtained by distilling off the solvent was purified by silica gel column chromatography (10% methanol-chloroform) to give ethyl 2-(1-{3-[(4R,6S)-8-chloro-6-(2,3-dimethoxyphenyl)-1-(2-methyl-2H-1,2,3,4-tetrazol-5-yl)-4H,6H-pyrrolo[1,2-a][4,1]benzoxazepin-4-yl]propanoyl}-4-piperidinyl)acetate (low polarity substance, 34 mg) and ethyl 2-(1-{3-[(4R,6S)-8-chloro-6-(2,3-dimethoxyphenyl)-1-(1-methyl-2H-1,2,3,4-tetrazol-5-yl)-4H,6H-pyrrolo[1,2-a][4,1]benzoxazepin-4-yl]propanoyl}-4-piperidinyl)acetate (high polarity substance, 33 mg), respectively.
Ethyl 2-(1-{3-[(4R,6S)-8-chloro-6-(2,3-dimethoxyphenyl)-1-(2-methyl-2H-1,2,3,4-tetrazol-5-yl)-4H,6H-pyrrolo[1,2-a][4,1]benzoxazepin-4-yl]propanoyl}-4-piperidinyl)acetate
MS (ESI) m/z : 663 (M + H)⁺.
¹H-NMR (CDCl₃) δ: 1.04-1.21 (2H, m), 1.26 (3H, t, J = 7.2 Hz), 1.68-1.82 (2H, m), 1.95-2.07 (1H, m), 2.19-2.26 (2H, m), 2.37-2.46 (2H, m), 2.51-2.62 (2H, m), 2.64-2.74 (1H, m), 2.97-3.07 (1H, m), 3.42 (3H, s), 3.83-3.95 (4H, m), 4.13 (2H, q, J = 7.2 Hz), 4.24-4.29 (4H, m), 4.55-4.64 (1H, m), 5.85 (1H, s), 6.44 (1H, d, J = 3.8 Hz), 6.76 (1H, d, J = 2.2 Hz), 6.95 (1H, d, J = 8.4 Hz), 7.03 (1H, d, J = 3.8 Hz), 7.08 (1H, dd, J = 8.4, 1.8 Hz), 7.18 (1H, t, J = 7.9 Hz), 7.23 (1H, dd, J = 8.4, 2.2 Hz), 7.25-7.28 (1H, m).
Ethyl 2-(1-{3-[(4R,6S)-8-chloro-6-(2,3-dimethoxyphenyl)-1-(1-methyl-2H-1,2,3,4-tetrazol-5-yl)-4H,6H-pyrrolo[1,2-a][4,1]benzoxazepin-4-yl]propanoyl}-4-piperidinyl)acetate
MS (ESI) m/z : 663 (M + H)⁺.
¹H-NMR (CDCl₃) δ: 1.06-1.20 (2H, m), 1.26 (3H, t, J = 7.2 Hz), 1.67-1.82 (2H, m), 1.97-2.07 (1H, m), 2.19-2.28 (2H, m), 2.41-2.49 (2H, m), 2.52-2.73 (3H, m), 3.05 (1H, t, J = 12.9 Hz), 3.52 (3H, s), 3.62 (3H, s), 3.85-3.90 (4H, m), 4.14 (2H, q, J = 7.2 Hz), 4.32-4.37 (1H, m), 4.56-4.63 (1H, m), 5.78 (1H, s), 6.55 (1H, d, J = 3.9 Hz), 6.73-6.77 (2H, m), 6.87 (1H, d, J = 3.9 Hz), 6.99 (1H, dd, J = 8.3, 1.5 Hz), 7.17-7.31 (3H, m).

### Example 361

2-(1-{3-[(4R,6S)-8-Chloro-6-(2,3-dimethoxyphenyl)-1-(2-methyl-2H-1,2,3,4-tetrazol-5-yl)-4H,6H-pyrrolo[1,2-a][4,1]benzoxazepin-4-yl]propanoyl}-4-piperidinyl)acetic acid

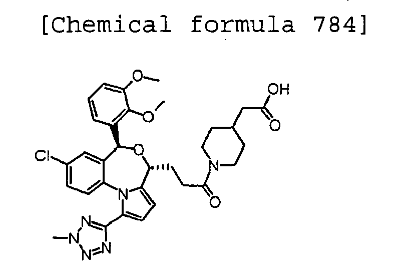

To a methanol solution (10.0 mL) of ethyl 2-(1-{3-[(4R,6S)-8-chloro-6-(2,3-dimethoxyphenyl)-1-(2-methyl-2H-1,2,3,4-tetrazol-5-yl)-4H,6H-pyrrolo[1,2-a][4,1]benzoxazepin-4-yl]propanoyl}-4-piperidinyl)acetate (34 mg) were added distilled water (5.0 mL) and potassium carbonate (21 mg). The resulting mixture was stirred at 50°C for 2 hours. To the reaction mixture was added a 10% aqueous citric acid solution. The resulting mixture was extracted with ethyl acetate. The organic layer was dried over anhydrous sodium sulfate. The residue obtained by distilling off the solvent was purified by preparative thin layer chromatography on silica gel (development phase: a chloroform:methanol:water=7:3:1 organic layer), followed by lyophilization from 1,4-dioxane-water to give the title compound (22 mg).
MS (ESI) m/z : 635 (M + H)⁺.
¹H-NMR (CDCl₃) δ: 1.06-1.22 (2H, m), 1.74-1.83 (2H, m), 1.97-2.06 (1H, m), 2.25-2.32 (2H, m), 2.39-2.47 (2H, m), 2.52-2.74 (3H, m), 2.99-3.09 (1H, m), 3.42 (3H, s), 3.85 (4H, s), 4.24-4.32 (4H, m), 4.58-4.66 (1H, m), 5.85 (1H, s), 6.44 (1H, d, J = 3.9 Hz), 6.76 (1H, d, J = 2.4 Hz), 6.94-6.98 (1H, m), 7.02-7.05 (1H, m), 7.07-7.11 (1H, m), 7.16-7.30 (3H, m).
Elemental analysis for: C₃₂H₃₅ClN₆O₆·0.25CHCl₃
Calculated: C, 58.23; H, 5.38; N, 12.63.
Found: C, 58.47; H, 5.66; N, 12.27.

### Example 362

2-(1-{3-[(4R,6S)-8-Chloro-6-(2,3-dimethoxyphenyl)-1-(1-methyl-2H-1,2,3,4-tetrazol-5-yl)-4H,6H-pyrrolo[1,2-a][4,1]benzoxazepin-4-yl]propanoyl}-4-piperidinyl)acetic acid

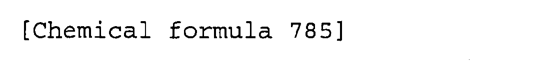

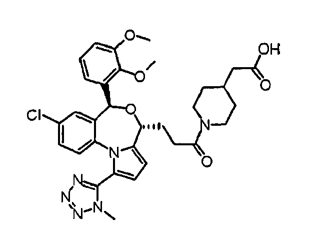

To a methanol solution (10.0 mL) of ethyl 2-(1-{3-[(4R,6S)-8-chloro-6-(2,3-dimethoxyphenyl)-1-(1-methyl-2H-1,2,3,4-tetrazol-5-yl)-4H,6H-pyrrolo[1,2-a][4,1]benzoxazepin-4-yl]propanoyl}-4-piperidinyl)acetate (33 mg) were added distilled water (5.0 mL) and potassium carbonate (21 mg). The resulting mixture was stirred at 50°C for 2 hours. To the reaction mixture was added a 10% aqueous citric acid solution, followed by extraction with ethyl acetate. The organic layer was dried over anhydrous sodium sulfate. The residue obtained by distilling off the solvent was purified by preparative thin layer chromatography on silica gel (development phase: chloroform:methanol:water=7:3:1 organic layer), followed by lyophilization from 1,4-dioxane-water to give the title compound (23 mg).
MS (ESI) m/z : 635 (M + H)⁺.
¹H-NMR (CDCl₃) δ: 1.06-1.22 (2H, m), 1.73-1.87 (2H, m), 1.95-2.08 (1H, m), 2.24-2.35 (2H, m), 2:42-2.50 (2H, m), 2.52-2.74 (3H, m), 2.99-3.09 (1H, m), 3.53 (3H, s), 3.61 (3H, s), 3.85-3.94 (4H, m), 4.32-4.39 (1H, m), 4.58-4.66 (1H, m), 5.77 (1H, s), 6.53-6.58 (1H, m), 6.72-6.79 (2H, m), 6.87 (1H, d, J = 3.9 Hz), 7.00 (1H, d, J = 8.3 Hz), 7.16-7.30 (3H, m).
Elemental analysis for: C₃₂H₃₅ClN₆O₆·0.25CHCl₃
Calculated: C, 58.23; H, 5.38; N, 12.63.
Found: C, 58.40; H, 5.67; N, 12.34.

### Referential Example 250

Ethyl 2-(1-{3-[(4R,6S)-8-chloro-6-(2,3-dimethoxyphenyl)-1-formyl-4H,6H-pyrrolo[1,2-a][4,1]benzoxazepin-4-yl]propanoyl}-4-piperidinyl)acetate

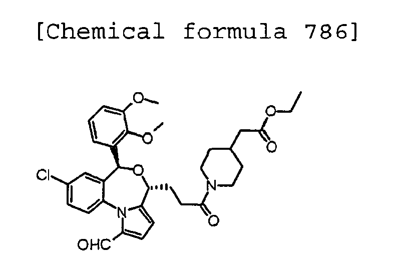

To a solution of ethyl 2-(1-{3-[8-chloro-1-cyano-6-(2,3-dimethoxyphenyl)-4H,6H-pyrrolo[1,2-a][4,1]benzoxazepin-4-yl]propanoyl}-4-piperidinyl)acetate (1.33 g) in a mixture of pyridine (8.0 mL), acetic acid (8.0 mL) and distilled water (8.0 mL) was added Raney nickel (8 mL). The resulting mixture was stirred at room temperature for 22 hours in a hydrogen atmosphere. After the insoluble material was filtered out and the residue was concentrated, ethyl acetate was added. The resulting mixture was washed with saturated brine and dried over anhydrous sodium sulfate. The residue obtained by distilling off the solvent was purified by silica gel chromatography (ethyl acetate:n-hexane=2:1) to give the title compound (1.23 g).
MS (ESI) m/z : 609 (M + H)⁺.
¹H-NMR (CDCl₃) δ: 1.04-1.18 (2H, m), 1.26 (3H, t, J = 7.1 Hz), 1.70-1.83 (2H, m), 1.95-2.07 (1H, m), 2.20-2.26 (2H, m), 2.37-2.44 (2H, m), 2.49-2.71 (3H, m), 2.98-3.08 (1H, m), 3.39 (3H, s), 3.84-3.93 (4H, m), 4.14 (2H, q, J = 7.1 Hz), 4.20-4.25 (1H, m), 4.54-4.62 (1H, m), 5.69 (1H, s), 6.48 8 (1H, d, J = 4.2 Hz), 6.75 (1H, d, J = 2.2 Hz), 6.96 (1H, dd, J = 7.9, 1.8 Hz), 7.16-7.23 (3H, m), 7.38-7.46 (2H, m), 9.71 (1H, s).

### Example 363

Ethyl 2-(1-{3-[(4R,6S)-8-chloro-1-(difluoromethyl)-6-(2,3-dimethoxyphenyl)-4H,6H-pyrrolo[1,2-a][4,1]benzoxazepin-4-yl]propanoyl}-4-piperidinyl)acetate

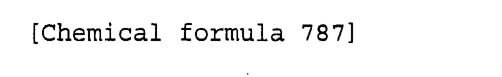

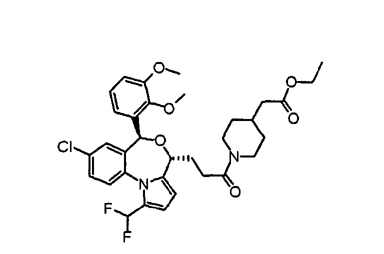

To a methylene chloride solution (1.0 mL) of ethyl 2-(1-{3-[(4R,6S)-8-chloro-6-(2,3-dimethoxyphenyl)-1-formyl-4H,6H-pyrrolo[1,2-a][4,1]benzoxazepin-4-yl]propanoyl}-4-piperidinyl)acetate (100 mg) was added diethylammonium sulfur trifluororide (0.87 mL) under ice cooling. The reaction mixture was stirred at room temperature for 15 hours. The reaction mixture was purified by preparative thin layer chromatography on silica gel (development phase: ethyl acetate:n-hexane=1:1) to give the title compound (25 mg).
MS (ESI) m/z : 631 (M + H)⁺.
¹H-NMR (CDCl₃) δ: 1.02-1.20 (2H, m), 1.25 (3H, t, J = 7.1 Hz), 1.75 (2H, t, J = 15.1 Hz), 1.94-2.07 (1H, m), 2.19-2.25 (2H, m), 2.39 (2H, dd, J = 13.8, 7.4 Hz), 2.44-2.70 (3H, m), 2.97-3.07 (1H, m), 3.38 (3H, s), 3.83-3.91 (4H, m), 4.13 (2H, q, J = 7.1 Hz), 4.22-4.27 (1H, m), 4.55-4.63 (1H, m), 5.53 (1H, s), 6.35 (1H, d, J = 3.7 Hz), 6.54-6.82 (3H, m), 6.94 (1H, dd, J = 8.0, 1.5 Hz), 7. 17 (1H, t, J = 8.0 Hz), 7.23 (1H, d, J = 7.6 Hz), 7.40 (1H, dd, J = 8.5, 2.3 Hz), 7.53 (1H, d, J = 8.5 Hz).

### Example 364

2-(1-{3-[(4R,6S)-8-Chloro-1-(difluoromethyl)-6-(2,3-dimethoxyphenyl)-4H,6H-pyrrolo[1,2-a][4,1]benzoxazepin-4-yl]propanoyl}-4-piperidinyl)acetic acid

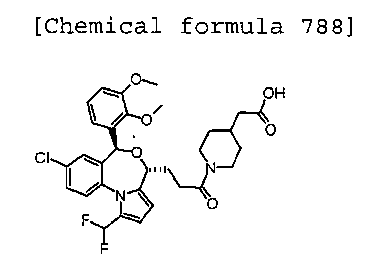

To a methanol solution (10.0 mL) of ethyl 2-(1-{3-[(4R,6S)-8-chloro-1-(difluoromethyl)-6-(2,3-dimethoxyphenyl)-4H,6H-pyrrolo[1,2-a][4,1]benzoxazepin-4-yl]propanoyl}-4-piperidinyl)acetate (29 mg) were added distilled water (5.0 mL) and potassium carbonate (16 mg). The resulting mixture was stirred at room temperature for 13 hours. The reaction mixture was adjusted to pH 4.0 with an ion exchange resin IR-120B. The resin was filtered out and the solvent was distilled off. The residue was purified by preparative thin layer chromatography on silica gel (development phase: 10% methanol-chloroform), followed by lyophilization from 1,4-dioxane-water to give the title compound (17 mg).
MS (ESI) m/z : 603 (M + H)⁺.
¹H-NMR (CDCl₃) δ: 1.02-1.20 (2H, m), 1.69-1.81 (2H, m), 1.94-2.07 (1H, m), 2.21-2.28 (2H, m), 2.35-2.43 (2H, m), 2.49-2.70 (3H, m), 2.97-3.07 (1H, m), 3.38 (3H, s), 3.82-3.94 (4H, m), 4.12-4.14 (1H, m), 4.22-4.27 (1H, m), 4.55-4.63 (1H, m), 5.53 (1H, s), 6.36 (1H, d, J = 3.7 Hz), 6.67-6.77 (3H, m), 6.95 (1H, dd, J = 8.0, 1.5 Hz), 7.17 (1H, t, J = 8.0 Hz), 7.23 (1H, d, J = 7.6 Hz), 7.40 (1H, dd, J = 8.5, 2.3 Hz), 7.54 (1H, d, J = 8.5 Hz).
Elemental analysis for: C₃₁H₃₃ClN₂O₆F₂·1.5H₂O
Calculated: C, 59.09; H, 5.76; N, 4.45.
Found: C, 59.13; H, 5.64; N, 4.26.

### Example 365

2-(1-{3-[(4R,6S)-1-[(Acetyloxy)methyl]-8-chloro-6-(2,3-dimethoxyphenyl)-4H,6H-pyrrolo[1,2-a][4,1]benzoxazepin-4-yl]propanoyl}-4-piperidinyl)acetic acid

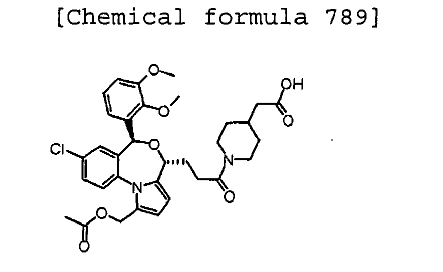

2-(1-{3-[(4R,6S)-1-[Hydroxymethyl]-8-chloro-6-(2,3-dimethoxyphenyl)-4H,6H-pyrrolo[1,2-a][4,1]benzoxazepin-4-yl]propanoyl}-4-piperidinyl)acetic acid (96 mg) was dissolved in acetic acid (10 mL). To the resulting solution was added a 37% aqueous formalin solution (0.08 mL). The resulting mixture was stirred at room temperature for 16 hours. The reaction mixture was diluted with chloroform. The water layer was adjusted to pH 5 with a saturated aqueous solution of sodium bicarbonate. The organic layer was dried over anhydrous sodium sulfate. After concentration, the residue thus obtained was purified by preparative thin layer chromatography on silica gel (development phase: 10% methanol-chloroform) to give the title compound (75 mg).
MS (ESI) m/z : 625 (M + H)⁺.
¹H-NMR (CDCl₃) δ: 1.06-1.18 (2H, m), 1.96-2.04 (2H, m), 2.07 (3H, s), 2.26-2.41 (4H, m), 2.51-2.66 (4H, m), 2.98-3.06 (1H, m), 3.41 (3H, s), 3.84-3.93 (4H, m), 4.25-4.29 (1H, m), 4.57-4.64 (1H, m), 4.77 (1H, d, J = 13.2 Hz), 5.47 (1H, d, J = 13.2 Hz), 5.50 (1H, s), 6.32 (1H, d, J = 3.4 Hz), 6.50 (1H, d, J = 3.7 Hz), 6.74 (1H, d, J = 2.4 Hz), 6.94 (1H, dd, J = 8.1, 1.5 Hz), 7.17 (1H, t, J = 8.1 Hz), 7.23-7.26 (0H, m), 7.37-7.40 (1H, m), 7.43-7.47 (1H, m).
Elemental analysis for: C₃₃H₃₇ClN₂O₈·0.25H₂O·0.25CHCl₃
Calculated: C, 60.54; H, 5.81; N, 4.25.
Found: C, 60.56; H, 5.98; N, 4.38.

### Example 366

Ethyl 2-(2-{2-[(4R,6S)-1-[(acetyloxy)methyl]-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate

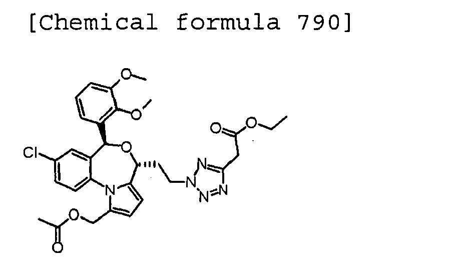

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (1080 mg) was dissolved in acetic acid (50 mL). To the resulting solution was added a 37% aqueous formalin solution (0.96 mL). The resulting mixture was stirred at room temperature for 20 hours and then at 50°C for 18 hours. The reaction mixture was concentrated. The residue was diluted with ethyl acetate, and washed with a saturated aqueous solution of sodium bicarbonate. The organic layer was dried over anhydrous sodium sulfate. After concentration, the residue thus obtained was purified by silica gel chromatography (ethyl acetate:n-hexane=1:1) to give the title compound (798 mg).
MS (ESI) m/z : 610 (M + H)⁺.
¹H-NMR (CDCl₃) δ: 1.25 (3H, t, J = 7.2 Hz), 2.07 (3H, s), 2.67-2.85 (2H, m), 3.42 (3H, s), 3.85 (3H, s), 3.92 (2H, s), 4.16-4.35 (3H, m), 4.77 (1H, d, J = 13.0 Hz), 4.87-4.93 (2H, m), 5.48 (1H, d, J = 13.2 Hz), 5.56 (1H, s), 6.30 (1H, d, J = 3.7 Hz), 6.51 (1H, d, J = 3.7 Hz), 6.76 (1H, d, J = 2.2 Hz), 6.95-6.98 (1H, m), 7.20 (1H, t, J = 8.1 Hz), 7.28-7.31 (1H, m), 7.40 (1H, dd, J = 8.5, 2.3 Hz), 7.46 (1H, d, J = 8.6 Hz).

### Example 367

Ethyl 2-(2-{2-[(4R,6S)-1-(hydroxymethyl)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate

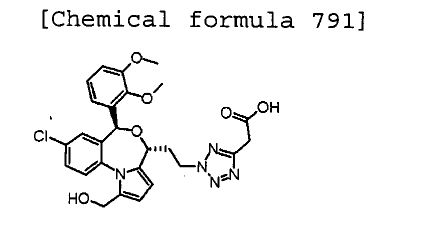

To a methanol solution (10.0 mL) of ethyl 2-(2-{2-[(4R,6S)-1-[(acetyloxy)methyl]-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (61 mg) were added distilled water (5.0 mL) and potassium carbonate (41 mg). The resulting mixture was stirred at 50°C for 14 hours. To the reaction mixture was added a 10% aqueous citric acid solution, followed by extraction with 1 chloroform. The organic layer was dried over anhydrous sodium sulfate. The residue obtained by distilling off the solvent was purified by preparative thin layer chromatography on silica gel (development phase: chloroform:methanol:water=7:3:1 organic layer) and lyophilized from 1,4-dioxane to give the title compound (62 mg).

MS (ESI) m/z : 540 (M + H)⁺.
¹H-NMR (CDCl₃) δ: 2.58-2.68 (2H, m), 3.32 (3H, s), 3.81 (3H, s), 4.27-4.37 (2H, m), 4.66-4.85 (3H, m), 5.47 (1H, s), 6.10 (1H, d, J = 3.2 Hz), 6.23 (1H, d, J = 3.2 Hz), 6.70 (1H, d, J = 2.4 Hz), 6.90 (1H, d, J = 7.3 Hz), 7.14 (1H, t, J = 8.1 Hz), 7.26-7.34 (2H, m), 7.76 (1H, d, J = 8.5 Hz).
Elemental analysis for: C₂₆H₂₆ClN₅O₆·1.5H₂O·0.25CHCl₃
Calculated: C, 52.80; H, 4.98; N, 11.73.
Found: C, 52.81; H, 4.65; N, 11.37.

### Example 368

Ethyl 2-(2-{2-[(4R,6S)-8-chloro-6-(2,3-dimethoxyphenyl)-1-(methoxymethyl)-4H,6H-pyrrolo[1,2-a][4,1]benzoxazepin-4-yl]ethyl}-2H-1,2,3,4-tetrazol-5-yl)acetate

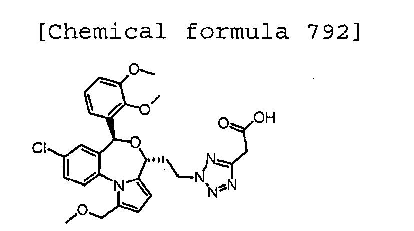

To a methanol solution (5.0 mL) of ethyl 2-(2-{2-[(4R,6S)-1-[(acetyloxy)methyl]-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (122 mg) was added a methylene chloride solution (0.30 mL) of 1N trifluoroacetic acid. The resulting mixture was stirred at room temperature for 20 hours. Then, methanol (5 mL), distilled water (5.0 mL), and potassium carbonate (138 mg) were added and the resulting mixture was stirred at 50°C for 4 hours. An ion exchange resin IR-120B was added to the reaction mixture. After the resin was filtered off, the solvent was distilled off. The residue thus obtained was purified by preparative thin layer chromatography on silica gel (development phase: 10% methanol-chloroform) and lyophilized from 1,4-dioxane-water to give the title compound (113 mg).
MS (ESI) m/z : 554 (M + H)⁺.
¹H-NMR (CDCl₃) δ: 2.59-2.77 (2H, m), 3.32 (3H, s), 3.36 (3H, s), 3.82 (3H, s), 4.27 (1H, d, J = 12.5 Hz), 4.29-4.35 (1H, m), 4.47 (1H, d, J = 12.5 Hz), 4.68-4.92 (2H, m), 5.53 (1H, s), 6.21 (1H, d, J = 3.2 Hz), 6.36 (1H, d, J = 3.7 Hz), 6.72 (1H, d, J = 2.2 Hz), 6.91 (1H, d, J = 7.6 Hz), 7.15 (1H, t, J = 8.2 Hz), 7.26-7.29 (1H, m), 7.35 (1H, dd, J = 8.5, 2.2 Hz), 7.63 (1H, d, J = 8.5 Hz).
Elemental analysis for: C₂₇H₂₈ClN₅O₆·0.5H₂O·0.25CHCl₃ Calculated: C, 55.18; H, 5.01; N, 11.81.
Found: C, 55.02; H, 4.84; N, 11.73.

### Example 369

Ethyl 2-(2-{2-[(4R,6S)-8-chloro-6-(2,3-dimethoxyphenyl)-1-(isopropoxymethyl)-4H,6H-pyrrolo[1,2-a][4,1]benzoxazepin-4-yl]ethyl}-2H-1,2,3,4-tetrazol-5-yl)acetate

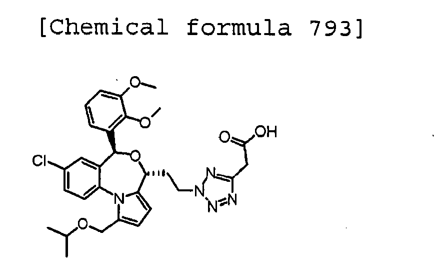

To an isopropanol solution (5.0 mL) of ethyl 2-(2-{2-[(4R,6S)-1-[(acetyloxy)methyl]-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (105 mg) was added a methylene chloride solution (0.30 mL) of 1N trifluoroacetic acid. The resulting mixture was stirred at 50°C for one day. Then, methanol (5 mL), distilled water (5.0 mL) and potassium carbonate (119 mg) were added and the resulting mixture was stirred at 50°C for 14 hours.
The reaction mixture was concentrated. The residue was diluted with ethyl acetate, washed with a 10% aqueous citric acid solution, and dried over anhydrous sodium sulfate. The residue obtained by distilling off the solvent was purified by preparative thin layer chromatography on silica gel (development phase: chloroform:methanol:water=7:3:1 organic layer) and lyophilized from 1,4-dioxane-water to give the title compound (48 mg).
MS (ESI) m/z : 582 (M + H)⁺.
¹H-NMR (CDCl₃) δ: 1.19 (6H, t, J = 6.7 Hz), 2.59-2.72 (2H, m), 3.35 (3H, s), 3.64-3.76 (3H, m), 3.81 (3H, s), 4.22 (1H, d, J = 12.0 Hz), 4.30-4.35 (1H, m), 4.53 (1H, d, J = 12.0 Hz), 4.65-4.89 (2H, m), 5.49 (1H, s), 6.16 (1H, d, J = 3.4 Hz), 6.32 (1H, d, J = 3.4 Hz), 6.70 (1H, d, J = 2.2 Hz), 6.89 (1H, d, J = 7.8 Hz), 7.13 (1H, t, J = 8.1 Hz), 7.23-7.25 (1H, m), 7.33 (1H, dd, J = 8.5, 2.2 Hz, Hz), 7.74 (1H, d, J = 8.5 Hz).
Elemental analysis for: C₂₉H₃₂ClN₅O₆·0.75CHCl₃
Calculated: C, 53.15; H, 5.02; N, 10.42.
Found: C, 53.39; H, 4.81; N, 10.48

### Example 370

Ethyl 2-(2-{2-[(4R,6S)-8-chloro-6-(2,3-dimethoxyphenyl)-1-(ethoxymethyl)-4H,6H-pyrrolo[1,2-a][4,1] benzoxazepin-4-yl]ethyl}-2H-1,2,3,4-tetrazol-5-yl)acetate

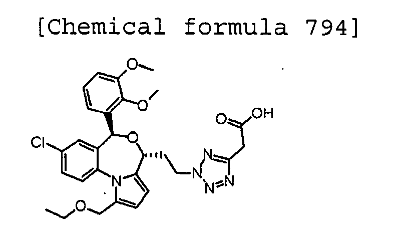

To an ethanol solution (5.0 mL) of ethyl 2-(2-{2-[(4R,6S)-1-[(acetyloxy)methyl]-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (112 mg) was added a methylene chloride solution (0.37 mL) of 1N trifluoroacetic acid. The resulting mixture was stirred at 50°C for 14 hours. Then, methanol(5 mL), distilled water (5.0 mL) and potassium carbonate (127 mg) were added. The resulting mixture was stirred at 50°C for 14 hours. The reaction mixture was concentrated. The residue was diluted with ethyl acetate, washed with a 10% aqueous citric acid solution and dried over anhydrous sodium sulfate. The residue obtained by distilling off the solvent was purified by preparative thin layer chromatography on silica gel (development phase: chloroform:methanol:water=7:3:1 organic layer) and lyophilized from 1,4-dioxane-water to give the title compound (48 mg).
MS (ESI) m/z : 568 (M + H)⁺.
¹H-NMR (CDCl₃) δ: 1.20 (3H, t, J = 7.0 Hz), 2.53-2.71 (2H, m), 3.34 (3H, s), 3.41-3.61 (2H, m), 3.80 (3H, s), 4.26 (2H, d, J = 12.0 Hz), 4.26-4.33 (1H, m), 4.49 (2H, d, J = 12.3 Hz), 4.63-4.86 (2H, m), 5.49 (1H, s), 6.18 (1H, d, J = 3.7 Hz), 6.31 (2H, d, J = 3.7 Hz), 6.69 (1H, d, J = 2.2 Hz), 6.88 (1H, d, J = 8.1 Hz), 7.11 (13H, t, J = 8.1 Hz), 7.20-7.26 (1H, m), 7.32 (1H, dd, J = 8.7, 2.3 Hz), 7.68 (1H, d, J = 8.6 Hz).
Elemental analysis for: C₂₈H₃₀ClN₅O₆·0.5CHCl₃
Calculated: C, 52.97; H, 5.15; N, 10.84.
Found: C, 52.67; H, 4.75; N, 10.51

### Referential Example 251

(4R,6S)-4-(2-{[tert-Butyl(diphenyl)silyl]oxy}ethyl)-8-chloro-6-(2,3-dimethoxyphenyl)-4H,6H-pyrrolo[1,2-a] [4,1]benzoxazepine

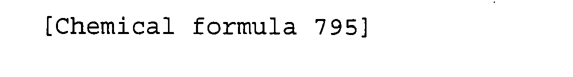

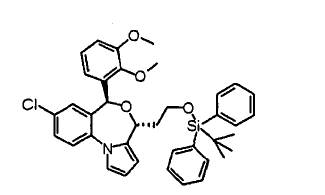

2-[(4R,6S)-8-Chloro-6-(2,3-dimethoxyphenyl)-4H,6H-pyrrolo[1,2-a] [4,1]benzoxazepin-4-yl]-1-ethanol (1120 mg) was dissolved in methylene chloride (30 mL). To the resulting solution were added imidazole (990 mg) and tert-butyldiphenylsilyl chloride (936 µl). The resulting mixture was stirred at room temperature for 1.5 hours. The reaction mixture was concentrated. The residue thus obtained was purified by silica gel chromatography (ethyl acetate:n-hexane=1:2) to give the title compound (2080 mg). ¹H-NMR (CDCl₃) δ: 0.95 (9H, s), 2.29-2.34 (2H, m), 3.42 (3H, s), 3.84 (3H, s), 3.90-4.02 (2H, m), 4.64 (1H, t, J = 6.6 Hz), 5.67 (1H, s), 6.22-6.24 (1H, m), 6.36 (1H, t, J = 3.2 Hz), 6.71 (1H, d, J = 2.2 Hz), 6.92 (1H, dd, J = 8.1, 1.7 Hz), 7.07-7.16 (3H, m), 7.31-7.44 (7H, m), 7.56-7.64 (4H, m), 7.70-7.73 (1H, m).

### Referential Example 252

(4R,6S)-4-(2-{[tert-Butyl(diphenyl)silyl]oxy}ethyl)-8-chloro-6-(2,3-dimethoxyphenyl)-4H,6H-pyrrolo[1,2-a][4,1]benzoxazepinel-carbonitrile

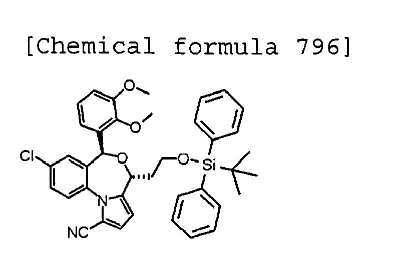

To an acetonitrile solution (5 mL) of (4R,6S)-4-(2-{[tert-butyl(diphenyl)silyl]oxy}ethyl)-8-chloro-6-(2,3-dimethoxyphenyl)-4H,6H-pyrrolo[1,2-a][4,1]benzoxazepine (517 mg) was added N,N-dimethylformamide (5 mL). At -40°C, chlorosulfonyl isocyanate (141 µl) was added. The resulting mixture was stirred at the same temperature for one hour and under stirring for 10 minutes. After addition of water, the resulting mixture was extracted with ethyl acetate. The organic layer was washed with saturated brine and dried over anhydrous sodium sulfate. After concentration, the residue thus obtained was purified by silica gel chromatography (ethyl acetate:n-hexane=1:2) to give the title compound (538 mg).
¹H-NMR (CDCl₃)δ: 0.92 (9H, s), 2.23-2.33 (2H, m), 3.43 (3H, s), 3.84-3.99 (2H, m), 3.85 (3H, s), 4.55 (1H, dd, J = 8.4, 5.2 Hz), 5.56 (1H, s), 6.27 (1H, d, J = 3.9 Hz), 6.79 (1H, d, J = 2.2 Hz), 6.94 (1H, dd, J = 7.6, 2.2 Hz), 7.03-7.13 (3H, m), 7.31-7.48 (8H, m), 7.52-7.61 (5H, m), 7.70-7.73 (1H, m).

### Referential Example 253

(4R,6S)-8-Chloro-6-(2,3-dimethoxyphenyl)-4-(2-hydroxyethyl)-4H,6H-pyrrolo[1,2-a][4,1]benzoxazepine1-carbonitrile

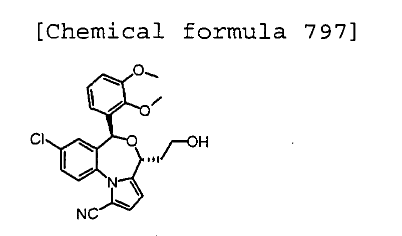

(4R,6S)-4-(2-{[tert-Butyl(diphenyl)silyl]oxy}ethyl)-8-chloro-6-(2,3-dimethoxyphenyl)-4H,6H-pyrrolo[1,2-a][4,1]benzoxazepinel-carbonitrile (120 mg) was dissolved in tetrahydrofuran (10 mL). To the resulting solution was added a 1M tetrabutylammonium fluoride/tetrahydrofuran solution (0.25 mL). The resulting mixture was stirred at room temperature for 12 hours. The reaction mixture was diluted with ethyl acetate, washed with saturated brine, and dried over anhydrous sodium sulfate. After concentration, the residue thus obtained was purified by silica gel chromatography (ethyl acetate:n-hexane=2:1) to give the title compound (74 mg).
MS (ESI) m/z : 425 (M + H)⁺.
¹H-NMR (CDCl₃) δ: 2.16-2.25 (1H, m), 2.34-2.44 (1H, m), 3.46 (3H, s), 3.86 (3H, s), 3.90-3.99 (2H, m), 4.52 (1H, dd, J = 9.8, 3.9 Hz), 5.64 (1H, s), 6.38 (1H, dd, J = 4.1, 0.6 Hz), 6.82 (1H, d, J = 2.4 Hz), 6.97 (1H, dd, J = 7.4, 2.4 Hz), 7.08 (1H, d, J = 4.1 Hz), 7.17-7.26 (2H, m), 7.48 (1H, dd, J = 8.4, 2.4 Hz), 7.60 (1H, d, J = 8.4 Hz).

### Example 371

Ethyl 2-(2-(2-[(4R,6S)-8-chloro-1-cyano-6-(2,3-dimethoxyphenyl)-4H,6H-pyrrolo[1,2-a][4,1]benzoxazepin-4-yl]ethyl)-2H-1,2,3,4-tetrazol-5-yl)acetate

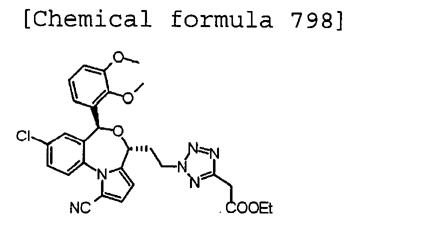

Ethyl 2-(2-{2-[(4R,6S)-8-chloro-1-cyano-6-(2,3-dimethoxyphenyl)-4H,6H-pyrrolo[1,2-a][4,1]benzoxazepin-4-yl]ethyl}-1H-1,2,3,4-tetrazol-5-yl)acetate

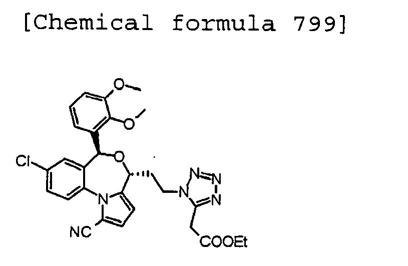

(4R,6S)-8-Chloro-6-(2,3-dimethoxyphenyl)-4-(2-hydroxyethyl)-4H,6H-pyrrolo[1,2-a][4,1]benzoxazepine1-carbonitrile (74 mg) was dissolved in methylene chloride (10 mL). To the resulting solution was added triethylamine (0.05 mL). Under ice cooling, methanesulfonyl chloride (0.02 mL) was added. The resulting mixture was stirred at the same temperature for 2 hours. The reaction mixture was diluted with ethyl acetate, washed with saturated brine, dried over anhydrous sodium sulfate, and then concentrated.
The mesyl compound thus obtained was dissolved in N,N-dimethylformamide (10 mL). To the resulting solution were added n-tetrabutylammonium iodide (63 mg), potassium carbonate (70 mg) and ethyl 1H-tetrazol-5-acetate (66 mg). The resulting mixture was stirred at 60°C for 14 hours.
The reaction mixture was diluted with ethyl acetate, washed with saturated brine, dried over anhydrous sodium sulfate, and concentrated. The residue thus obtained was purified by preparative thin layer chromatography on silica gel (development phase: ethyl acetate:n-hexane=1:1) to give ethyl 2-(2-{2-[(4R,6S)-8-chloro-1-cyano-6-(2,3-dimethoxyphenyl)-4H,6H-pyrrolo[1,2-a][4,1]benzoxazepin-4-yl]ethyl}-2H-1,2,3,4-tetrazol-5-yl)acetate (low polarity substance, 55 mg) and ethyl 2-(2-{2-[(4R,6S)-8-chloro-1-cyano-6-(2,3-dimethoxyphenyl)-4H,6H-pyrrolo[1,2-a][4,1]benzoxazepin-4-yl]ethyl}-1H-1,2,3,4-tetrazol-5-yl)acetate (high polarity substance, 22 mg), respectively. Ethyl 2-(2-{2-[(4R,6S)-8-chloro-1-cyano-6-(2,3-dimethoxyphenyl)-4H,6H-pyrrolo[1,2-a][4,1]benzoxazepin-4-yl]ethyl}-2H-1,2,3,4-tetrazol-5-yl)acetate
MS (ESI) m/z : 563 (M + H)⁺.
¹H-NMR (CDCl₃) δ: 1.25 (3H, t, J = 7.1 Hz), 2.68-2.84 (2H, m), 3.46 (3H, s), 3.87 (3H, s), 3.92 (2H, s), 4.19 (2H, q, J = 7.1 Hz), 4.33 (1H, dd, J = 9.8, 3.9 Hz), 4.90-4.94 (2H, m), 5.66 (1H, s), 6.38-6.39 (1H, m), 6.81 (1H, d, J = 2.2 Hz), 6.99 (1H, dd, J = 8.2, 1.6 Hz), 7.08 (1H, d, J = 4.2 Hz), 7.21 (1H, t, J = 7.9 Hz), 7.27-7.29 (1H, m), 7.48 (1H, dd, J = 8.5, 2.4 Hz), 7.59 (1H, d, J = 8.5 Hz).
Ethyl 2-(2-{2-[(4R,6S)-8-chloro-1-cyano-6-(2,3-dimethoxyphenyl)-4H,6H-pyrrolo[1,2-a][4,1]benzoxazepin-4-yl]ethyl}-1H-1,2,3,4-tetrazol-5-yl)acetate MS (ESI) m/z : 563 (M + H)⁺.
¹H-NMR (CDCl₃) δ: 1.25 (3H, t, J = 7.1 Hz), 2.63-2.81 (2H, m), 3.45 (3H, s), 3.87 (3H, s), 4.01 (2H, s), 4.15-4.20 (2H, m), 4.34-4.38 (1H, m), 4.51-4.67 (2H, m), 5.66 (1H, s), 6.38-6.40 (1H, m), 6.82 (1H, d, J = 2.4 Hz), 6.97-7.01 (1H, m), 7.08 (1H, d, J = 3.7 Hz), 7.18-7.21 (2H, m), 7.50 (1H, dd, J = 8.4, 2.3 Hz), 7.61 (1H, d, J = 8.5 Hz).

### Example 372

2-(2-{2-[(4R,6S)-8-Chloro-1-cyano-6-(2,3-dimethoxyphenyl)-4H,6H-pyrrolo[1,2-a][4,1]benzoxazepin-4-yl]ethyl}-2H-1,2,3,4-tetrazol-5-yl)acetic acid

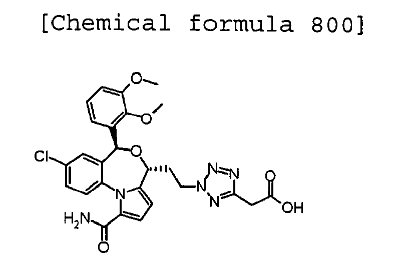

Ethyl 2-(2-{2-[(4R,6S)-8-chloro-1-cyano-6-(2,3-dimethoxyphenyl)-4H,6H-pyrrolo[1,2-a][4,1]benzoxazepin-4-yl]ethyl}-2H,-1,2,3,4-tetrazol-5-yl)acetate (55 mg) was dissolved in isopropanol (5 mL). To the resulting solution was added a 1N aqueous sodium hydroxide solution (0.49 mL). After heating under reflux for 1 hour, a 1N aqueous sodium hydroxide solution (0.49 mL) was added and the resulting mixture was heated under reflux for 14 hours. A 5N aqueous sodium hydroxide solution (0.20 mL) was added and the resulting mixture was heated under reflux for 0.5 hour. Then, a 5N aqueous sodium hydroxide solution (0.39 mL) was added, followed by heating under reflux for 24 hours. Isopropanol was distilled off and to the residue was added a 10% aqueous citric acid solution. The resulting mixture was extracted with ethyl acetate. The organic layer was washed with saturated brine, dried over anhydrous sodium sulfate, and concentrated. The residue thus obtained was purified by preparative thin layer chromatography on silica gel (development phase: 7:3:1=chloroform:methanol:water organic layer), followed by lyophilization from 1,4-dioxane-water to give the title compound (40 mg).
MS (ESI) m/z : 553 (M + H)⁺.
¹H-NMR (DMSO-d₆) δ: 2.38-2.62 (4H, m), 3.22 (3H, s), 3.75 (3H, s), 4.09 (1H, dd, J = 8.8, 4.2 Hz), 4.76 (2H, t, J = 7.4 Hz), 5.54 (1H, s), 6.33 (1H, d, J = 3.7 Hz), 6.46 (1H, d, J = 2.4 Hz), 6.95 (1H, d, J = 3.7 Hz), 6.98-7.08 (2H, m), 7.15-7.21 (2H, m), 7.28 (1H, d, J = 8.5 Hz), 7.39 (1H, dd, J = 8.5, 2.4 Hz), 7.73 (1H, br s).
Elemental analysis for: C₂₆H₂₅ClN₆O₆·0.75CHCl₃·0.5 1,4-dioxane
Calculated: C, 50.24; H, 4.47; N, 12.23.
Found: C, 50.38; H, 4.50; N, 12.11.

### Example 373

Ethyl 1-{2-[(4R,6S)-8-chloro-1-cyano-6-(2,3-dimethoxyphenyl)-4H,6H-pyrrolo[1,2-a][4,1]benzoxazepin-4-yl]ethyl}-1H-pyrazole-4-carboxylate

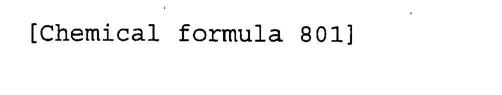

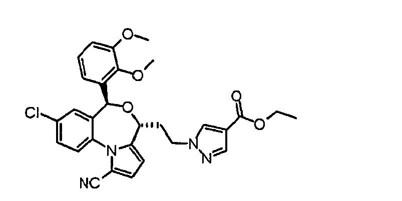

To an acetonitrile solution (5 mL) of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-4-carboxylate (522 mg) was added N,N-dimethylformamide (5 mL). At -40°C, chlorosulfonyl isocyanate (174 µl) was added and the resulting mixture was stirred at the same temperature for 5 minutes and under ice cooling for 2 hours. Water was added to the reaction mixture, followed by extraction with ethyl acetate. The organic layer was washed with saturated brine, dried over anhydrous sodium sulfate and then concentrated. The residue thus obtained was purified by silica gel chromatography (ethyl acetate:n-hexane=1:2) to give the title compound (520 mg).
MS (ESI) m/z : 547 (M + H)⁺.
¹H-NMR (CDCl₃) δ: 1.32 (3H, t, J = 7.2 Hz), 2.58-2.67 (2H, m), 3.45 (3H, s), 3.86 (3H, s), 4.22-4.30 (3H, m), 4.36-4.51 (2H, m), 5.64 (1H, s), 6.38 (1H, d, J = 4.2 Hz), 6.82 (1H, d, J = 2.2 Hz), 6.99 (1H, dd, J = 7.9, 1.6 Hz), 7.07 (1H, d, J = 3.9 Hz), 7.19-7.27 (0H, m), 7.48 (1H, dd, J = 8.5, 2.4 Hz), 7.59 (1H, d, J = 8.5 Hz), 7.86 (1H, s), 7.92 (1H, s).

### Example 374

1-{2-[(4R,6S)-1-(Aminocarbonyl)-8-chloro-6-(2,3-dimethoxyphenyl)-4H,6H-pyrrolo[1,2-a][4,1]benzoxazepin-4-yl]ethyl}-1H-pyrazole-4-carboxylic acid

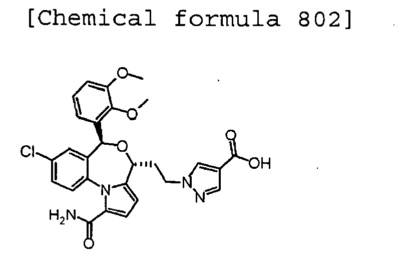

Ethyl 1-{2-[(4R,6S)-8-chloro-1-cyano-6-(2,3-dimethoxyphenyl)-4H,6H-pyrrolo[1,2-a][4,1]benzoxazepin-4-yl]ethyl}-1H-pyrazole-4-carboxylate (520 mg) was dissolved in isopropanol (20 mL). To the resulting solution was added a 1N aqueous sodium hydroxide solution (9.51 mL).
The resulting mixture was heated under reflux for 12 hours. The reaction mixture was acidified with an ion exchange resin IR-120B. The resin was then filtered out and the residue was concentrated. The residue thus obtained was purified by preparative thin layer chromatography on silica gel (development phase: 15% methanol-chloroform) and solidified with isopropanol-isopropyl ether to give the title compound (306 mg).
MS (ESI) m/z : 537 (M + H)⁺.
¹H-NMR (CDCl₃) δ: 2.57-2.67 (2H, m), 3.43 (3H, s), 3.86 (3H, s), 4.04-4.09 (1H, m), 4.37-4.54 (2H, m), 5.75-5.83 (3H, m), 6.29 (1H, d, J = 3.9 Hz), 6.78 (1H, s), 6.90 (1H, d, J = 3.9 Hz), 6.98 (1H, d, J = 7.8 Hz), 7.19-7.26 (2H, m), 7.32-7.37 (2H, m), 7.90-7.96 (2H, m).
Elemental analysis for: C₂₇H₂₅ClN₄O₆·0.5 H2O
Calculated: C, 59.40; H, 4.80; N, 10.26.
Found: C, 59.50; H, 5.04; N, 9.90.

### Referential Example 254

1-(2-Bromo-4-chlorophenyl)-1H-pyrrole

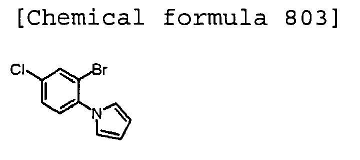

To an acetic acid solution (20 mL) of 2-bromo-4-chloroaniline (5.16 g) was added 2,5-dimethoxytetrahydrofuran (3.56 mL). The resulting mixture was stirred at 80°C for 2 hours. The solvent was distilled off under reduced pressure. The residue thus obtained was purified by silica gel column chromatography (n-hexane:ethyl acetate= from 1:0 to 2:1) to give the title compound (6.30 g).
1H-NMR (CDCl3) δ: 6.32-6.35 (2H, m), 6.82-6.85 (2H, m), 7.25 (1H, d, J = 8.3 Hz), 7.35 (1H, dd, J = 8.3, 2.2 Hz), 7.69 (1H, d, J = 2.2 Hz).

### Referential Example 255

[5-Chloro-2(1H-pyrrol-1-yl)phenyl](2-fluoro-3-methoxyphenyl)methanol

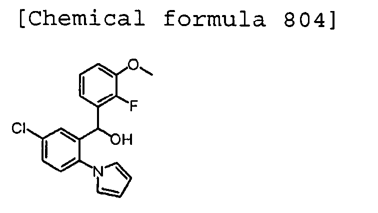

To a diethyl ether solution (150 mL) of 1-(2-bromo-4-chlorophenyl)-1H-pyrrole (6.78 g) was added normal-butyl lithium (1.6 mol/l, 19.8 mL) at -78°C. While warming to 0°C, the resulting mixture was stirred for 2.5 hours. The reaction mixture was cooled to -78°C again, followed by the addition of 2-fluoro-3-methoxybenzaldehyde (4.28 g). While warming to room temperature, stirring was continued for 2 hours. To the reaction mixture were added a saturated aqueous solution of ammonium chloride and ethyl acetate and the layers separated. The organic layer was washed with saturated brine and dried over anhydrous sodium sulfate. The solvent was distilled off under reduced pressure to give the title compound (4.70 g).
1H-NMR (CDCl3) δ: 2.22-2.25 (1H, m), 3.86 (3H, s), 6.00 (1H, d, J = 4.7 Hz), 6.27-6.29 (2H, m), 6.71-6.73 (2H, m), 6.88-6.96 (2H, m), 7.03-7.10 (1H, m), 7.20-7.24 (1H, m), 7.33 (1H, dd, J = 8.6, 2.5 Hz), 7.46 (1H, d, J = 2.5 Hz).

### Referential Example 256

[5-Chloro-2(1H-pyrrol-1-yl)phenyl](2-fluoro-3-methoxyphenyl)methanone

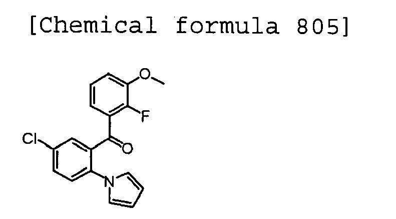

To a dichloromethane solution (200 mL) of [5-chloro-2(1H-pyrrol-1-yl)phenyl](2-fluoro-3-methoxyphenyl)methanol (6.89 g) was added manganese dioxide (10.25 g). The resulting mixture was stirred at 50°C for 25 hours. To the reaction mixture was added manganese dioxide (4.10 g) and the resulting mixture was stirred overnight at 50°C. The catalyst was filtered and the filtrate was washed with dichloromethane. The filtrates were combined and concentrated under reduced pressure to give the title compound (4.85 g).
MS (ESI) m/z : 330 (M + H)⁺⁻.
1H-NMR (CDCl₃) δ: 3.82 (3H, s), 5.99-6.01 (2H, m), 6.64-6.67 (2H, m), 6.94-7.01 (2H, m), 7.03-7.09 (1H, m), 7.31-7.34 (1H, m), 7.54 (1H, dd, J = 8.6 , 2.5 Hz), 7.60 (1H, d, J = 2.5 Hz).

### Referential Example 257

1-[4-Chloro-2-(2-fluoro-3-methoxybenzoyl)phenyl]-1H-pyrrol-2-carbaldehyde

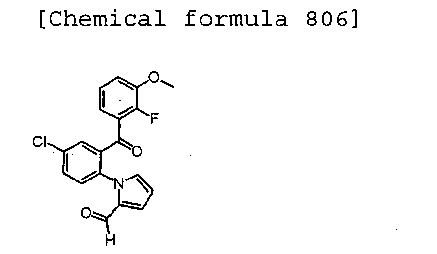

Phosphorus oxychloride (1.64 mL) was added to N,N-dimethylformamide (2.28 mL) under ice cooling and the resulting mixture was stirred for 30 minutes. To the reaction mixture was added [5-chloro-2(1H-pyrrol-1-yl)phenyl](2-fluoro-3-methoxyphenyl)methanone(4.85 g). The resulting mixture was stirred at 50°C for 30 minutes. A saturated aqueous solution of sodium acetate was added, followed by stirring at 50°C for 15 minutes. Ethyl acetate and distilled water were added to the reaction mixture and the layers separated. The organic layer thus obtained was dried over anhydrous sodium sulfate to give the title compound (1.75 g).
MS (ESI) m/z : 358 (M + H)⁺⁻.
1H-NMR (CDCl₃) δ: 3.84 (3H, s), 6.20 (1H, dd, J = 4.0, 2.6 Hz), 6.80-6.84 (1H, m), 6.89-6.92 (1H, m), 6.93-7.03 (3H, m), 7.32 (1H, d, J = 8.3 Hz), 7.58 (2H, dd, J = 8.3, 2.5 Hz), 9.37-9.39 (1H, m).

### Referential Example 258

Methyl (E)-3-{1-[4-chloro-2-(2-fluoro-3-methoxybenzoyl)phenyl]-1H-pyrrol-2-yl}-2-propenoate

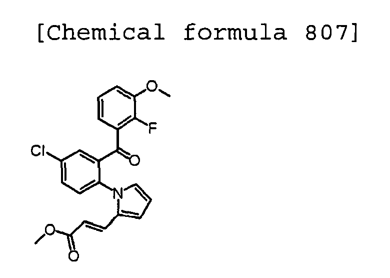

To a toluene solution (50 mL) of 1-[4-chloro-2-(2-fluoro-3-methoxybenzoyl)phenyl]-1H-pyrrol-2-carbaldehyde (1.75 g) was added methyl (triphenylphosphoranylidene)acetate (3.26 g). The resulting mixture was stirred at 110°C for 11 hours. The reaction mixture was concentrated under reduced pressure. To the residue thus obtained were added dichloromethane and distilled water and the layers separated. The organic layer thus obtained was washed with saturated brine and dried over anhydrous sodium sulfate. The solvent was distilled off under reduced pressure. The residue was purified by silica gel column chromatography (n-hexane:ethyl acetate=3:1) to give the title compound (1.55 g).
MS (ESI) m/z : 436 (M + H)⁺⁻.
1H-NMR (CDCl₃) δ: 3.73 (3H, s), 3.81 (3H, s), 5.90 (1H, d, J = 15.7 Hz), 6.05-6.08 (1H, m), 6.42-6.46 (1H, m), 6.71-6.77 (1H, m), 6.89-7.02 (3H, m), 7.18 (1H, d, J = 15.7 Hz), 7.29 (1H, d, J = 8.3 Hz), 7.61 (1H, dd, J = 8.3, 2.5 Hz), 7.68 (1H, d, J = 2.5 Hz).

### Referential Example 259

Methyl (E)-3-(1-{4-chloro-2-[(2-fluoro-3-methoxyphenyl)(hydroxy)methyl]phenyl}-1H-pyrrol-2-yl)-2-propenoate

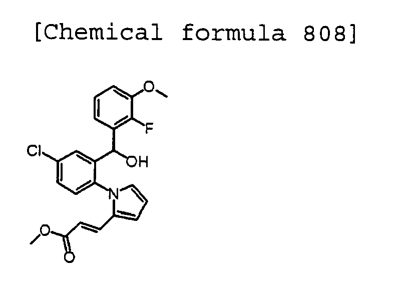

To a methanol solution (40 mL) of methyl (E)-3-{1-[4-chloro-2-(2-fluoro-3-methoxybenzoyl)phenyl]-1H-pyrrol-2-yl}-2-propenoate (1.54 g) was added sodium borohydride (0.21 g) under ice cooling. While warming to room temperature, the resulting mixture was stirred for 6 hours. Acetone was added to terminate the reaction. The reaction mixture was concentrated under reduced pressure. To the residue were added ethyl acetate and a saturated aqueous solution of sodium chloride and the layers separated. The organic layer thus obtained was dried over anhydrous sodium sulfate. The solvent was distilled off under reduced pressure. The residue was purified by silica gel column chromatography (hexane:ethyl acetate=4:1) to give the title compound (1.56 g) as a mixture of atropisomers. MS (ESI) m/z : 416 (M + H)⁺⁻.
1H-NMR (CDCl₃) δ: 2.24 and 2.40 (1H, d, J = 4.2 Hz), 3.63 and 3.70 (3H, s), 3.80 and 3.84 (3H, s), 5.63 and 5.72 (1H, d, J = 4.2 Hz), 5.91 and 5.91 (1H, d, J = 15.7 Hz), 6.22-6.25 and 6.34-6.38 (1H, m), 6.46-6.48 (2H, m), 6.74-6.80 (1H, m), 6.84-6.94 and 6.99-7.07 (2H, m), 7.10-7.26 (1H, m), 7.35-7.41 (1H, m), 7.63 and 7.79 (1H, d, J = 2.2 Hz).

### Example 375

Methyl 2-[(trans)-8-chloro-6-(2-fluoro-3-methoxyphenyl)-4H,6H-pyrrolo[1-2-a][4,1]benzoxazepin-4-yl]acetate

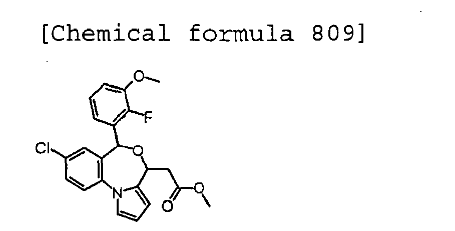

In a similar manner to that employed for the synthesis of methyl 2-(8-chloro-6-(2,3-dimethoxyphenyl)-4H,6H-pyrrolo(1,2-a)(4,1)benzoxazepin-4-yl)acetate, the title compound (623 mg) was obtained from methyl (E)-3-(1-{4-chloro-2-[(2-fluoro-3-methoxyphenyl)(hydroxy)methyl]phenyl}-1H-pyrrol-2-yl)-2-propenoate (1.55 g) by using trifluoroacetic acid (0.43 mL).
MS (ESI) m/z : 416 (M + H)⁺⁻.
1H-NMR (CDCl₃) δ: 2.99-3.14 (2H, m), 3.72 (3H, s), 3.87 (3H, s), 4.87-4.96 (1H, m), 5.71 (1H, s), 6.27-6.32 (1H, m), 6.38 (1H, t, J = 3.2 Hz), 6.72 (1H, d, J = 2.0 Hz), 6.93-7.04 (1H, m), 7.09-7.26 (3H, m), 7.30-7.44 (2H, m).

### Example 376

2-[(trans)-8-Chloro-6-(2-fluoro-3-methoxyphenyl)-4H,6H-pyrrolo[1-2-a][4,1]benzoxazepin-4-yl]acetic acid

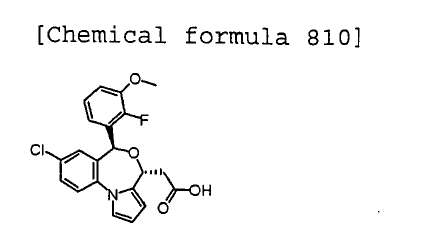

In a similar manner to that employed for the synthesis of 2-(8-chloro-6-(2,3-dimethoxyphenyl)-4H,6H-pyrrolo(1,2-a)(4,1)benzoxazepin-4-yl)acetic acid, the title compound (462 mg) was obtained from methyl 2-[(trans)-8-chloro-6-(2-fluoro-3-methoxyphenyl)-4H,6H-pyrrolo[1-2-a][4,1]benzoxazepin-4-yl]acetate (459 mg) by using potassium carbonate (457 mg).
MS (ESI) m/z : 402 (M + H)⁺⁻.
1H-NMR (CDCl₃) δ: 3.04-3.19 (2H, m), 3.88 (3H, s), 4.88-4.94 (1H, m), 6.28-6.42 (2H, m), 6.73-6.81 (1H, m), 6.82-6.96 (1H, m), 6.96-7.07 (1H, m), 7.09-7.22 (2H, m), 7.33-7.47 (2H, m).

### Example 377

Ethyl-2-(1-{2-[(trans)-8-chloro-6-(2-fluoro-3-methoxyphenyl)--4H,6H-pyrrolo[1-2-a][4,1]benzoxazepin-4-yl]acetyl}-4-piperidinyl)acetate

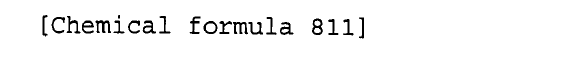

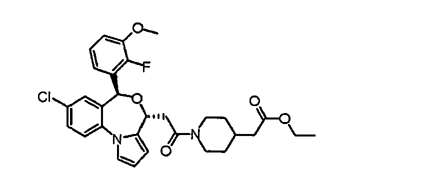

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (157 mg) was obtained from -2-[(trans)-8-chloro-6-(2-fluoro-3-methoxyphenyl)--4H,6H-pyrrolo[1-2-a][4,1]benzoxazepin-4-yl]acetic acid (171 mg) by using ethyl-2-(4-piperidinyl)acetate(95 mg), 1-ethyl-3-(3-dimethylaminopropyl)carbodiimide hydrochloride (123 mg), and 1-hydroxybenzotriazole (29 mg).
MS (ESI) m/z : 555 (M + H)⁺.
¹H-NMR (CDCl₃) δ: 0.99-1.31 (5H, m), 1.51-1.83 (2H, m), 1.93-2.31 (3H, m), 2.51-2.72 (1H, m), 2.78-3.14 (2H, m), 3.21-3.31 (1H, m), 3.87 and 3.88 (3H, s), 3.99-4.08 (1H, m), 4.09-4.17 (2H, m), 4.59-4.74 (1H, m), 4.93-5.03 (1H, m), 5.70-5.73 (1H, m), 6.24-6.31 (1H, m), 6.36-6.38 (1H, m), 6.66-6.72 (1H, m), 6.94-7.04 (1H, m), 7.08-7.13 (1H, m), 7.15-7.21 (1H, m), 7.32-7.22 (1H, m), 7.40-7.32 (2H, m).

### Example 378

2-(1-{2-[(trans)-8-Chloro-6-(2-fluoro-3-methoxyphenyl)-4H,6H-pyrrolo[1-2-a][4,1]benzoxazepin-4-yl]acetyl}-4-piperidinyl)acetic acid

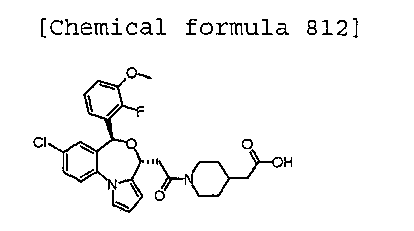

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (80 mg) was obtained from ethyl-2-(1-{2-[(trans)-8-chloro-6-(2-fluoro-3-methoxyphenyl)-4H,6H-pyrrolo[1-2-a][4,1]benzoxazepin-4-yl]acetyl}-4-piperidinyl)acetate (125 mg) by using potassium carbonate (93 mg).
MS (ESI) m/z : 527 (M + H)⁺.
¹H-NMR (CDCl₃) δ: 0.83-1.35 (2H, m), 1.61-2.08 (2H, m), 2.12-2.36 (2H, m), 2.49-3.15 (3H, m), 3.21-3.35 (1H, m), 3.87 and 3.88 (3H, s), 3.99-4.10 (1H, m), 4.92-5.03 (1H, m), 5.70-5.73 (1H, m), 6.24-6.27 (1H, m), 6.36-6.38 (1H, m), 6.67-6.71 (1H, m), 6.96-7.03 (1H, m), 7.09-7.11 (1H, m), 7.43-7.14 (4H, m).
Elemental analysis for: C₂₈H₂₈ClFN₂O₅
Calculated: C, 63.82; H, 5.36; N, 5.32.
Found: C, 63.80; H, 5.61; N, 5.12.

### Referential Example 260

tert-Butyl 4-(3-methoxy-3-oxopropyl)-1-piperidine carboxylate

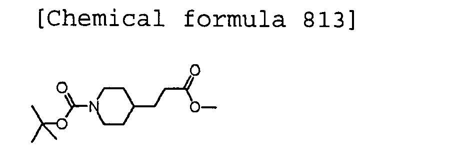

To an N,N-dimethylformamide solution (7.5 mL) of 3-[1-(tert-butoxycarbonyl)-4-piperidinyl]propanoic acid (200 mg) were added potassium carbonate (313 mg) and iodomethane (118 mg). The resulting mixture was stirred at 60°C for 4 hours. To the reaction mixture were added ethyl acetate and distilled water and the layers separated. The organic layer thus obtained was dried over anhydrous sodium sulfate and distilled under reduced pressure to remove the solvent. The resulting residue was purified by silica gel column chromatography (dichloromethane:methanol=100:1) to give the title compound (154 mg).
MS (ESI) m/z : 294(M + Na)⁺.
¹H-NMR (CDCl₃) δ: 1.03-1.15 (2H, m), 1.34-1.44 (1H, m), 1.45 (9H, s), 1.53-1.69 (6H, m), 2.31-2.37 (2H, m), 2.60-2.73 (2H, m), 3.67 (3H, s), 4.16-4.02 (2H, m).

### Referential Example 261

Methyl 3-(4-piperidinyl)propanoate hydrochloride

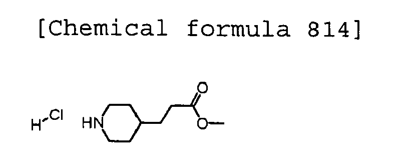

tert-Butyl 4-(3-methoxy-3-oxopropyl)-1-piperidine carboxylate (205 mg) was dissolved in a 4N hydrochloric acid-1,4-dioxane solution (7.5 mL) under ice cooling. The resulting mixture was stirred as was for 2.5 hours. The reaction mixture was concentrated under reduced pressure to give the title compound (106 mg).
MS (ESI) m/z : 172(M + H)⁺.
¹H-NMR (CDCl₃) δ: 1.55-1.71 (5H, m), 1.85-1.94 (2H, m), 2.32-2.37 (2H, m), 2.76-2.90 (2H, m), 3.44-3.52 (2H, m), 3.68 (3H, s), 9.35 (1H, br s), 9.66 (1H, br s).

### Example 379

Methyl 3-(1-{2-[(trans)-8-chloro-6-(3,4-dimethoxy-2-pyridinyl)-4H,6H-pyrrolo[1,2-a][4,1]benzoxazepin-4-yl]acetyl}-4-piperidinyl)propanoate

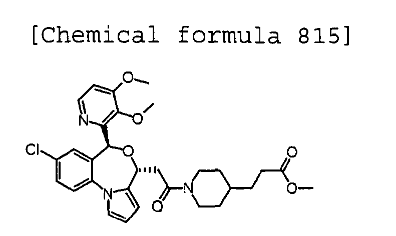

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (74 mg) was obtained from 2-[(trans)-8-chloro-6-(3,4-dimethoxy-2-pyridinyl)-4H,6H-pyrrolo[1,2-a][4,1]benzoxazepin-4-yl]acetic acid (96 mg) by using methyl 3-(4-piperidinyl)propanoate hydrochloride (49 mg), 1-ethyl-3-(3-dimethylaminopropyl)carbodiimide hydrochloride (65 mg), 1-hydroxybenzotriazole (30 mg), and triethylamine (24 mg). MS (ESI) m/z : 568 (M + H)⁺.
¹H-NMR (CDCl₃) δ: 0.75-1.15 (2H, m), 1.35-1.72 (5H, m), 2.28-2.36 (2H, m), 2.39-2.52 (1H, m), 2.59-2.69 (1H, m), 2.73-3.06 (2H, m), 3.46-3.57 (3H, m), 3.75-3.58 (1H, m), 3.68 (3H, s), 3.85-3.89 (3H, m), 4.53-4.63 (1H, m), 5.42-5.57 (1H, m), 6.18-6.28 (3H, m), 6.77-6.82 (1H, m), 7.00-7.10 (2H, m), 7.20-7.42 (2H, m), 8.21-8.28 (1H, m).

### Example 380

3-(1-{2-[(trans)-8-Chloro-6-(3,4-dimethoxy-2-pyridinyl)-4H,6H-pyrrolo[1,2-a][4,1]benzoxazepin-4-yl]acetyl}-4-piperidinyl)propanoic acid

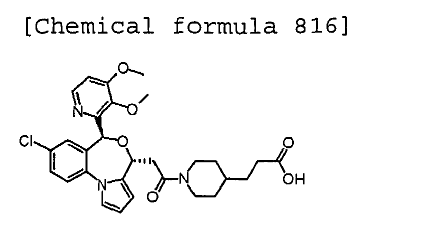

In a similar manner to that described in Referential Example 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (44 mg) was obtained from methyl 3-(1-{2-[(trans)-8-chloro-6-(3,4-dimethoxy-2-pyridinyl)-4H,6H-pyrrolo[1,2-a] [4,1]benzoxazepin-4-yl]acetyl}-4-piperidinyl)propanoate (70 mg) by using potassium carbonate (52 mg).
MS (ESI) m/z : 554 (M + H)⁺.
¹H-NMR (CDCl₃) δ: 0.91-1.34 (2H, m), 1.43-1.70 (5H, m), 2.25-2.65 (4H, m), 2.74-3.05 (2H, m), 3.44 (3H, s), 3.50 (3H, s), 3.58-3.73 (1H, m), 3.87 (3H, s), 3.88 (3H, s), 4.52-4.64 (1H, m), 5.44-5.56 (1H, m), 6.16-6.29 (3H, m), 6.80-6.84 (1H, m), 6.96-7.00 (1H, m), 7.05-7.10 (1H, m), 7.26-7.42 (2H, m), 8.32-8.28 (1H, m).
Elemental analysis for: C₂₉H₃₂ClN₃O₆·1.0H₂O·0.5CHCl₃
Calculated: C, 56.09; H, 5.50; N, 6.65.
Found: C, 56.23; H, 5.47; N, 6.55.

### Referential Example 262

6-fluoro-2,3-diemethoxybenzaldehyde

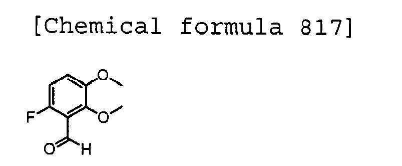

To a diethyl ether solution (250 mL) of 4-fluoro-1,2-dimethoxybenzene (10.5 g) was added normal-butyl lithium (1:6 mol/1, 50.2 mL) at -78°C. While warming to -60°C, the resulting mixture was stirred for 2 hours. The reaction mixture was cooled to -78°C, followed by the addition of N,N-dimethylformamide (15.6 mL) thereto. Stirring was continued until the temperature became room temperature.
To the reaction mixture was added distilled water and the resulting mixture was extracted with ethyl acetate. The organic layer thus obtained was washed with saturated brine and dried over anhydrous sodium sulfate. The solvent was then distilled off under reduced pressure. The resulting residue was purified by silica gel column chromatography (n-hexane:ethyl acetate=8:1) to give the title compound (9.8 g).
MS (ESI) m/z : 185 (M + H)⁺.
¹H-NMR (CDCl₃) δ: 3.88 (3H, s), 3.98 (3H, s), 6.81-6.88 (1H, m), 7.06-7.11 (1H, m), 10.40-10.39 (1H, m).

### Referential Example 263

[5-Chloro-2-(1H-pyrrol-1-yl)phenyl](6-fluoro-2,3-dimethoxyphenyl)methanol

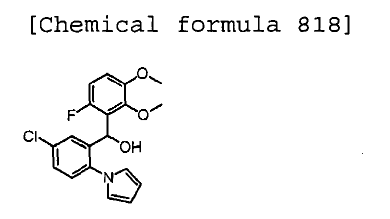

To a diethyl ether solution (440 mL) of 1-(2-bromo-4-chlorophenyl)-1H-pyrrole (11.4 g) was added normal-butyl lithium (1.6 mol/l, 30.6 mL) at -78°C. While warming to - 40°C, the resulting mixture was stirred for 3.5 hours. The reaction mixture was cooled to -78°C again and 6-fluoro-2,3-diemethoxybenzaldehyde (9.8 g) was added thereto.
While warming to 0°C, stirring was continued for 2 hours.
A saturated aqueous solution of ammonium chloride and ethyl acetate were added to the reaction mixture and the layers separated. The organic layer thus obtained was washed with saturated brine and dried over anhydrous sodium sulfate. The solvent was distilled off under reduced pressure to give the title compound (7.7 g).
MS (ESI) m/z : 344 (M - OH)⁺.
¹H-NMR (CDCl₃) δ: 3.50-3.55 (1H, m), 3.68 (3H, s), 3.83 (3H, s), 6.06 (1H, d, J = 9.3 Hz), 6.25-6.27 (2H, m), 6.66-6.72 (1H, m), 6.75-6.80 (3H, m), 7.16-7.20 (1H, m), 7.27-7.31 (1H, m), 7.58-7.55 (1H, m).

### Referential Example 264

[5-Chloro-2-(1H-pyrrol-1-yl)phenyl](6-fluoro-2,3-dimethoxyphenyl)methanone

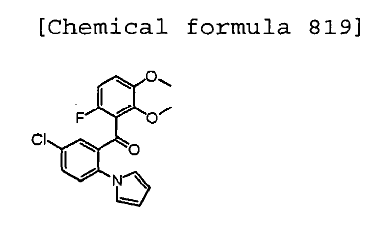

To a dichloromethane solution (100 mL) of [5-chloro-2-(1H-pyrrol-1-yl)phenyl](6-fluoro-2,3-dimethoxyphenyl)methanol (7.70 g) was added manganese dioxide (purity: 88%, 21.0 g). The resulting mixture was stirred at 60°C for 7 hours. The catalyst was filtered and the filtrate was washed with dichloromethane. The filtrates were combined and concentrated under reduced pressure to give the title compound (7.32 g).
MS (ESI) m/z : 360 (M + H)⁺.
¹H-NMR (CDCl₃) δ: 3.63 (3H, s), 3.80 (3H, s), 6.06-6.08 (2H, m), 6.62-6.67 (1H, m), 6.71-6.73 (2H, m), 6.79-6.85 (1H, m), 7.26-7.31 (1H, m), 7.51-7.55 (1H, m), 7.68-7.66 (1H, m).

### Referential Example 265

1-[4-Chloro-2-(6-fluoro-2,3-dimethoxybenzoyl)phenyl]-1H-pyrrol-2-carbaldehyde

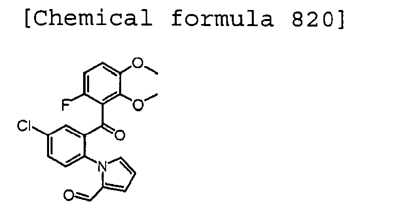

Phosphorus oxychloride (2.27 mL) was added to N,N-dimethylformamide (3.15 mL) at room temperature. The resulting mixture was stirred for 40 minutes. The reaction mixture was ice cooled and a dichloroethane solution (100 mL) of [5-chloro-2-(1H-pyrrol-1-yl)phenyl](6-fluoro-2,3-dimethoxyphenyl)methanone (7.31 g) was added thereto. The resulting mixture was stirred at 60°C for 5.5 hours. A saturated aqueous solution of sodium acetate was added and the resulting mixture was stirred at 60°C for 15 minutes. Dichloromethane and distilled water were added to the reaction mixture and the layers separated. The organic layer thus obtained was dried over anhydrous sodium sulfate to give the title compound (4.23 g).
MS (ESI) m/z : 358 (M + H)⁺⁻.
1H-NMR (CDCl₃) δ: 3.67 (3H, s), 3.80 (3H, s), 6.17-6.20 (1H, m), 6.62-6.67 (1H, m), 6.79-6.84 (1H, m), 6.87-6.90 (2H, m), 7.26-7.30 (1H, m), 7.54-7.58 (1H, m), 7.72 (1H, d, J = 2.5 Hz), 9.42 (1H, s).

### Referential Example 266

Methyl (E)-3-{1-[4-chloro-2-(6-fluoro-2,3-dimethoxybenzoyl)phenyl]-1H-pyrrol-2-yl}-2-propenoate

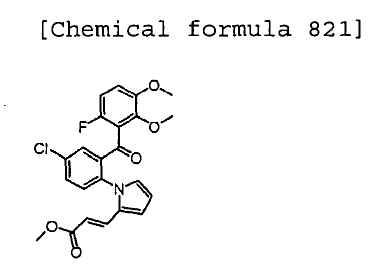

To a toluene solution 150 mL) of 1-[4-chloro-2-(6-fluoro-2,3-dimethoxybenzoyl)phenyl]-1H-pyrrol-2-carbaldehyde (4.23 g) was added methyl (triphenylphosphoranylidene)acetate (3.65 g). The resulting mixture was stirred at 110°C for 28.5 hours. The reaction mixture was concentrated under reduced pressure. To the residue thus obtained were added dichloromethane and distilled water and the layers separated. The organic layer thus obtained was washed with saturated brine and dried over anhydrous sodium sulfate. The solvent was distilled off under reduced pressure and the residue was purified by silica gel column chromatography (n-hexane:ethyl acetate= from 6:1 to 2:1) to give the title compound (3.97 g).
MS (ESI) m/z : 444 (M + H)⁺⁻.
1H-NMR (CDCl₃) δ: 3.68 (3H, s), 3.71 (3H, s), 3.77 (3H, s), 5.92 (1H, d, J = 15.9 Hz), 6.10-6.13 (1H, m), 6.52-6.55 (1H, m), 6.58-6.64 (1H, m), 6.76-6.81 (2H, m), 7.16-7.23 (2H, m), 7.59 (1H, dd, J = 8.3, 2.5 Hz), 7.74 (1H, d, J = 2.5 Hz).

### Referential Example 267

Methyl (E)-3-(1-{4-chloro-2-[(6-fluoro-2,3-dimethoxyphenyl)(hydroxy)methyl]phenyl}-1H-pyrrol-2-yl)-2-propenoate

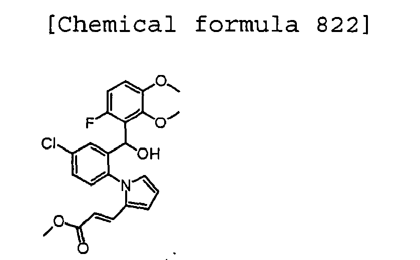

To a methanol solution (90 mL) of methyl (E)-3-{1-[4-chloro-2-(6-fluoro-2,3-dimethoxybenzoyl)phenyl]-1H-pyrrol-2-yl}-2-propenoate (3.97 g) was added sodium borohydride (0.51 g) under ice cooling. While warming to room temperature, the reaction mixture was stirred for 3 hours. Sodium borohydride (1.52 g) was added further and the resulting mixture was stirred for 16.5 hours. The reaction mixture was concentrated under reduced pressure. To the residue thus obtained were added ethyl acetate and a saturated aqueous solution of sodium chloride and the layers separated. The organic layer was dried over anhydrous sodium sulfate. The solvent was distilled off under reduced pressure. The resulting residue was purified by silica gel column chromatography (hexane:ethyl acetate= from 6:1 to 2:1) to give the title compound (3.99 g) as a mixture of atropisomers.
MS (ESI) m/z : 416 (M + H)⁺⁻.
1H-NMR (CDCl₃) δ: 3.09-3.11 (10H, m), 3.27-3.32 (1H, m), 3.58 (3H, s), 3.60 (3H, s), 3.62 (3H, s), 3.70 (3H, s), 3.72 (3H, s), 3.81 (3H, s), 5.62 (10H, d, J = 15.9 Hz), 5.83-6.01 (1H, m), 6.11-6.14 (0H, m), 6.32-6.36 (1H, m), 6.52-6.78 (4H, m), 6.90-6.92 (10H, m), 7.03-7.10 (1H, m), 7.16-7.23 (0H, m), 7.33-7.38 (1H, m), 7.88-7.79 (1H, m).

### Example 381

Methyl 2-[8-chloro-6-(6-fluoro-2,3-dimethoxyphenyl)-4H,6H-pyrrolo[1,2-a] [4,1] benzoxazepin-4-yl] acetate

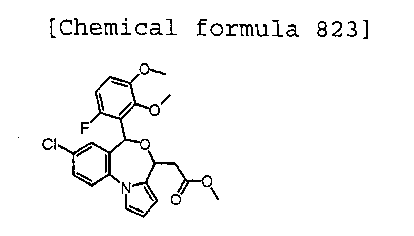

In a similar manner to that employed for the synthesis of methyl 2-(8-chloro-6-(2,3-dimethoxyphenyl)-4H,6H-pyrrolo(1,2-a)(4,1)benzoxazepin-4-yl)acetate, the title compound (261 mg) was obtained from methyl (E) -3- (1-{4-chloro-2-[(6-fluoro-2,3-dimethoxyphenyl)(hydroxy)methyl]phenyl}-1H-pyrrol-2-yl)-2-propenoate (1100 mg) by using trifluoroacetic acid (0.285 mL) .
MS (ESI) m/z : 446 (M + H)⁺⁻.
1H-NMR (CDCl₃) δ: 2.66-3.06 and 3.16-3.32 (2H, m), 3.53 and 3.63 (3H, s), 3.65-3.92 (6H, m), 4.96-5.01 and 5.43-5.32 (1H, m), 5.86-5.95 (1H, m), 6.50-6.15 (2H, m), 6.92-6.73 (2H, m), 7.18-6.95 (2H, m), 7.48-7.30 (2H, m).

### Referential Example 268

2-[(trans)-8-Chloro-6-(6-fluoro-2,3-dimethoxyphenyl)-4H,6H-pyrrolo[1,2-a][4,1]benzoxazepin-4-yl]-1-ethanol

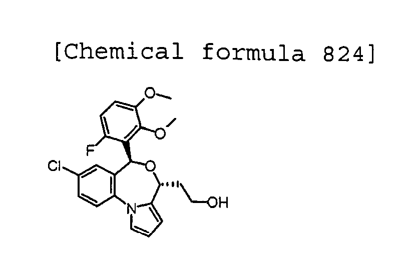

To a solution of methyl 2-[8-chloro-6-(6-fluoro-2,3-dimethoxyphenyl)-4H,6H-pyrrolo[1,2-a][4,1]benzoxazepin-4-yl]acetate (261 mg) in tetrahydrofuran (8.0 mL) was added lithium aluminum hydride (36.3 mg) under ice cooling. The resulting mixture was stirred for one hour. While still ice cooling, a 1N aqueous sodium hydroxide solution (0.18 mL) was added. The resulting mixture was filtered through Celite 545. The filtrate was concentrated under reduced pressure to give the title compound (246 mg).
MS (ESI) m/z : 418 (M + H)⁺⁻.
1H-NMR (CDCl₃) δ: 2.09-2.44 (2H, m), 2.62-2.68 and 2.97-3.04 (1H, m), 3.58 and 3.61 (3H, s), 3.79-3.92 (5H, m), 4.65-4.74 and 5.13-5.20 (1H, m), 5.88-5.93 (1H, m), 6.41-6.30 (2H, m), 7.14-6.74 (4H, m), 7.53-7.27 (2H, m).

### Referential Example 269

2-[(trans)-8-Chloro-6-(6-fluoro-2,3-dimethoxyphenyl)-4H,6H-pyrrolo[1,2-a][4,1]benzoxazepin-4-yl]ethyl methanesulfonate

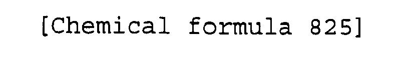

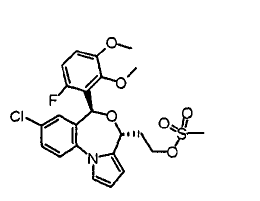

A dichloromethane solution (5.0 mL) of 2-[(trans)-8-chloro-6-(6-fluoro-2,3-dimethoxyphenyl)-4H,6H-pyrrolo[1,2-a] [4,1]benzoxazepin-4-yl]-1-ethanol (246 mg) were added triethylamine (123 µl) and methanesulfonyl chloride (55 µl) under ice cooling. While warming to room temperature, the reaction mixture was stirred for 5.5 hours. After the reaction mixture was ice-cooled again, triethylamine (41 µl) and methanesulfonyl chloride (14 µl) were added. While warming to room temperature, stirring was continued for 5.5 hours. To the reaction mixture were added dichloromethane, distilled water, and a saturated aqueous solution of sodium hydrogen carbonate and the layers separated. The organic layer thus obtained was dried over anhydrous sodium sulfate. The solvent was distilled off under reduced pressure to give the title compound (280 mg).
1H-NMR (CDCl₃) δ: 2.40-2.47 (1H, m), 2.97 (3H, s), 2.99-3.06 (1H, m), 3.80-3.91 (3H, m), 4.24-4.74 (3H, m), 5.88-5.93 (1H, m), 6.22-6.47 (2H, m), 6.93-6.82 (2H, m), 7.14-6.97 (2H, m), 7.44-7.34 (2H, m).

### Example 382

Ethyl 2-(2-(2-[(trans)-8-chloro-6-(6-fluoro-2,3-dimethoxyphenyl)-4H,6H-pyrrolo[1,2-a][4,1]benzoxazepin-4-yl]ethyl)-2H-1,2,3,4-tetrazol-5-yl)acetate
Isomer A (low polarity)

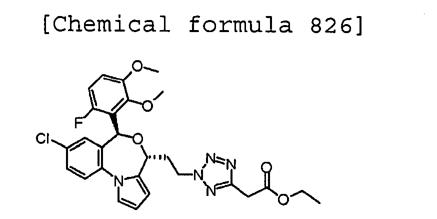

Ethyl 2-(2-{2-[(trans)-8-chloro-6-(6-fluoro-2,3-dimethoxyphenyl)-4H,6H-pyrrolo[1,2-a][4,1]benzoxazepin-4-yl]ethyl}-1H-1,2,3,4-tetrazol-5-yl)acetate
Isomer B (high polarity)

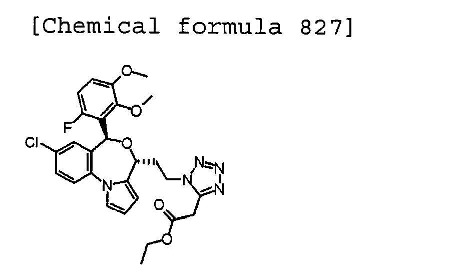

In a similar manner to that employed for the synthesis of ethyl 2-(2-{2-[(4R,6S)-8-chloro-6-(2,3-dimethoxyphenyl)-10-fluoro-4H,6H-pyrrolo[1,2-a][4,1]benzoxazepin-4-yl]ethyl}-2H-1,2,3,4-tetrazol-5-yl)acetate (A) and ethyl 2-(2-{2-[(4R,6S)-8-chloro-6-(2,3-dimethoxyphenyl)-10-fluoro-4H,6H-pyrrolo[1,2-a][4,1]benzoxazepin-4-yl]ethyl}-1H-1,2,3,4-tetrazol-5-yl)acetate (B), the title compounds, that is, isomer A (66 mg) and isomer B (55 mg) were obtained from 2-[(trans)-8-chloro-6-(6-fluoro-2,3-dimethoxyphenyl)-4H,6H-pyrrolo[1,2-a][4,1]benzoxazepin-4-yl]ethyl methanesulfonate (280 mg) by using ethyl 2-(1H-1,2,3,4-tetrazol-5-yl)acetate(106 mg), potassium carbonate (156 mg), and tetrabutylammonium iodide (209 mg).
Isomer A
MS (ESI) m/z : 578 (M + Na)⁺.
¹H-NMR (CDCl₃) δ: 1.21-1.29 (3H, m), 2.32-2.55 and 2.67-2.78 (2H, m), 3.58 and 3.62 (3H, s), 3.81 and 3.86 (3H, s), 3.93 and 3.94 (2H, s), 4.15-4.22 (2H, m), 4.56-4.96 (3H, m), 5.91-5.98 and 6.21-6.23 (1H, m), 6.27-6.46 (2H, m), 6.79-6.88 (1H, m), 6.90-6.96 (1H, m), 6.99-7.04 (1H, m), 7.07-7.14 (1H, m), 7.43-7.33 (2H, m).
Isomer B
MS (ESI) m/z : 578 (M + Na)⁺.
¹H-NMR (CDCl₃) δ: 1.21-1.29 (3H, m), 2.02-2.34 and 2.60-2.81 (2H, m), 3.55 and 3.55 (3H, s), 3.84 and 3.86 (3H, s), 3.98 and 3.99 (2H, s), 4.08-4.24 (2H, m), 4.33-4.63 and 4.72-4.83 (3H, m), 5.86-5.96 and 6.20-6.25 (1H, m), 6.28-6.42 (2H, m), 6.85-6.95 (2H, m), 6.97-7.01 (1H, m), 7.09-7.07 (1H, m), 7.44-7.31 (2H, m).

### Example 383

2-(1-(2-[(trans)-8-Chloro-6-(6-fluoro-2,3-dimethoxyphenyl)-4H,6H-pyrrolo[1,2-a][4,1]benzoxazepin-4-yl]ethyl)-1H-1,2,3,4-tetrazol-5-yl)acetic acid

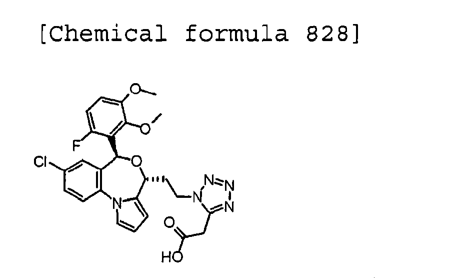

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (43 mg) was obtained from ethyl 2-(1-{2-[8-chloro-6-(6-fluoro-2,3-dimethoxyphenyl)-4H,6H-pyrrolo[1,2-a][4,1]benzoxazepin-4-yl]ethyl}-1H-1,2,3,4-tetrazol-5-yl)acetate(47 mg) by using potassium carbonate (35 mg).
MS (ESI) m/z : 528 (M + H)⁺, 550(M + Na)⁺.
¹H-NMR (CDCl₃) δ: 2.11-2.52 (2H, m), 3.43-3.59 (5H, m), 3.72 and 3.76 (3H, s), 4.16-4.54 and 4.74-4.84 (3H, m), 5.86 (1H, s), 6.03-6.40 (3H, m), 6.68-7.00 (4H, m), 7.09-7.43 and 7.49-7.76 (2H, m).
Elemental analysis for: C₂₅H₂₃ClFN₅O₅·0.75H₂O·0.75CHCl₃
Calculated: C, 49.02; H, 4.03; N, 11.10.
Found: C, 49.39; H, 4.07; N, 10.78.

### Example 384

2-(1-{2-[(trans)-8-Chloro-6-(6-fluoro-2,3-dimethoxyphenyl)-4H,6H-pyrrolo[1,2-a][4,1]benzoxazepin-4-yl]ethyl}-2H-1,2,3,4-tetrazol-5-yl)acetic acid

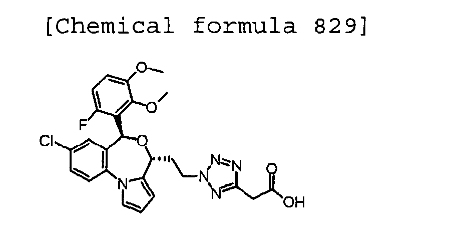

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (57 mg) was obtained from ethyl 2-(1-{2-[8-chloro-6-(6-fluoro-2,3-dimethoxyphenyl)-4H,6H-pyrrolo[1,2-a][4,1]benzoxazepin-4-yl]ethyl}-2H-1,2,3,4-tetrazol-5-yl)acetate(65 mg) by using potassium carbonate (97 mg).
MS (ESI) m/z: 528 (M + H)⁺, 550 (M + Na)⁺.
¹H-NMR (CDCl₃) δ: 2.20-2.70 (2H, m), 3.51 and 3.58 (3H, s), 3.69 (2H, s), 3.77 and 3.82 (3H, s), 4.48-4.85 (3H, m), 5.90 (1H, s), 6.11-6.42 (2H, m), 6.72-6.90 (2H, m), 6.95-7.06 (2H, m), 7.37-7.26 (2H, m).
Elemental analysis for: C₂₅H₂₃ClFN₅O₅·0.5H₂O·0.5CHCl₃·0.25 n-hexane
Calculated: C, 52.46; H, 4.57; N, 11.33.
Found: C, 52.21; H, 4.18; N, 11.22.

### Example 385

Methyl 2-[(4R,6S)-8-chloro-6-(6-fluoro-2,3-dimethoxyphenyl)-4H,6H-pyrrolo[1,2-a][4,1]benzoxazepin-4-yl]acetate

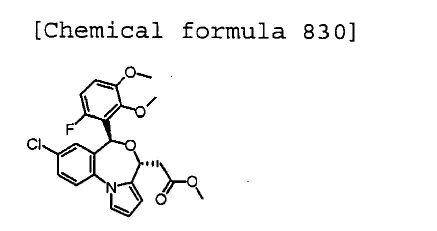

Methyl 2-[(4S,6R)-8-chloro-6-(6-fluoro-2,3-dimethoxyphenyl)-4H,6H-pyrrolo[1,2-a][4,1]benzoxazepin-4-yl] acetate

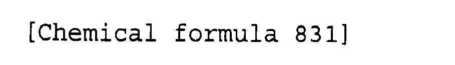

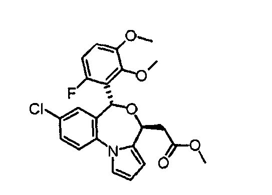

A cis-trans isomer was obtained by resolution of Methyl 2-[8-chloro-6-(6-fluoro-2,3-dimethoxyphenyl)-4H,6H-pyrrolo[1,2-a][4,1]benzoxazepin-4-yl]acetate (503 mg) while using HPLC equipped with CHIRALPAK AD (Daicel Chemical), followed by optical resolution by using HPLC equipped with CHIRALPAK OD (Daicel Chemical) to give a trans isomer. In obtaining each of the title compounds, the isomer A (190 mg) and isomer B (210 mg), 50% 2-propanol-hexane was used as a solvent and the flow rate was set at 10 mL/min.

### Example 386

2-[(4R,6S)-8-Chloro-6-(6-fluoro-2,3-dimethoxyphenyl)-4H,6H-pyrrolo[1,2-a][4,1]benzoxazepin-4-yl]acetic acid

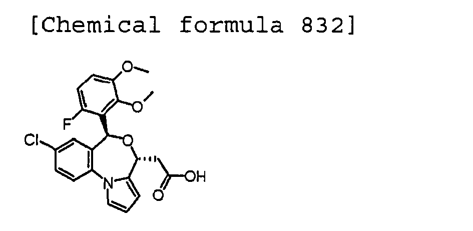

In a similar manner to that employed for the synthesis of 2-(8-chloro-6-(2,3-dimethoxyphenyl)-4H,6H-pyrrolo(1,2-a)(4,1)benzoxazepin-4-yl)acetic acid, the title compound (81 mg) was obtained from methyl 2-[(4R,6S)-8-chloro-6-(6-fluoro-2,3-dimethoxyphenyl)-4H,6H-pyrrolo[1,2-a] [4,1]benzoxazepin-4-yl]acetate (87 mg) by using potassium carbonate (80 mg).
MS (ESI) m/z : 432 (M + H)⁺, 454(M + Na)⁺.
¹H-NMR (CDCl₃) δ: 2.91-2.75 and 3.19-3.00 (2H, m), 3.57 and 3.66 (3H, s), 3.81 and 3.86 (3H, s), 4.90-4.97 and 5.38-5.32 (1H, m), 5.94 and 6.45 (1H, s), 6.42-6.21 (2H, m), 6.96-6.75 (2H, m), 7.17-6.99 (2H, m), 7.46-7.27 (2H, m). Elemental analysis for: C₂₂H₁₉ClFNO₅·0.5H₂O
Calculated: C, 59.94; H, 4.57; N, 3.18.
Found: C, 59.70; H, 4.68; N, 2.91.

### Example 387

Ethyl 2-(2-{2-[(4R,6S)-8-chloro-6-(6-fluoro-2,3-dimethoxyphenyl)-4H,6H-pyrrolo[1,2-a][4,1]benzoxazepin-4-yl]ethyl}-2H-1,2,3,4-tetrazol-5-yl)acetate
Isomer A (low polarity)

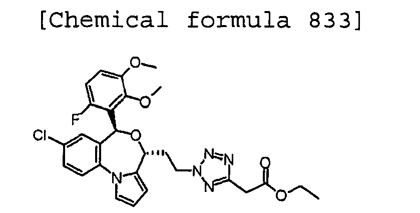

Ethyl 2-(2-{2-[(4R,6S)-8-chloro-6-(6-fluoro-2,3-dimethoxyphenyl)-4H,6H-pyrrolo[1,2-a][4,1]benzoxazepin-4-yl]ethyl}-1H-1,2,3,4-tetrazol-5-yl)acetate
Isomer B (high polarity)

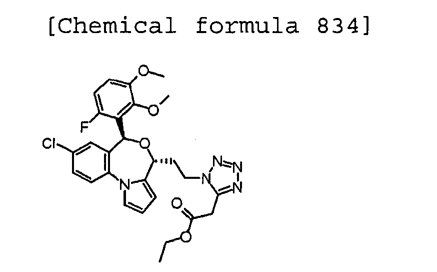

In a similar manner to that employed for the synthesis of ethyl 2-(2-{2-[(4R,6S)-8-chloro-6-(2,3-dimethoxyphenyl)-10-fluoro-4H,6H-pyrrolo[1,2-a][4,1]benzoxazepin-4-yl]ethyl}-2H-1,2,3,4-tetrazol-5-yl)acetate (A) and ethyl 2-(2-{2-[(4R,6S)-8-chloro-6-(2,3-dimethoxyphenyl)-10-fluoro-4H,6H-pyrrolo[1,2-a][4,1]benzoxazepin-4-yl]ethyl}-1H-1,2,3,4-tetrazol-5-yl)acetate (B), the title compounds, that is, isomer A (35 mg) and isomer B (18 mg) were obtained from 2-[8-chloro-6-(6-fluoro-2,3-dimethoxyphenyl)-4H,6H-pyrrolo[1,2-a][4,1]benzoxazepin-4-yl]ethyl methanesulfonate (99 mg) by using ethyl 2-(1H-1,2,3,4-tetrazol-5-yl)acetate (94 mg), potassium carbonate (166 mg, 1.20 mmol) and tetrabutylammonium iodide (147 mg).
Isomer A
MS (ESI) m/z : 578 (M + Na)⁺.
¹H-NMR (CDCl₃) δ: 1.21-1.27 (3H, m), 2.33-2.54 and 2.66-2.78 (2H, m), 3.58 and 3.62 (3H, s), 3.81 and 3.86 (3H, s), 3.93 and 3.94 (2H, s), 4.14-4.22 (2H, m), 4.56-4.64 and 4.70-4.97 (3H, m), 5.90-6.03 and 6.21-6.24 (1H, m), 6.28-6.46 (2H, m), 6.78-6.95 (2H, m), 7.00-7.14 (2H, m), 7.43-7.27 (2H, m).
Isomer B ¹H-NMR (CDCl₃) δ: 1.20-1.28 (3H, m), 2.58-2.81 (2H, m), 3.55 and 3.55 (3H, s), 3.84 and 3.86 (3H, s), 3.98 and 3.99 (2H, s), 4.08-4.22 (2H, m), 4.36-4.64 and 4.74-4.82 (3H, m), 5.91-6.01 and 6.20-6.25 (1H, m), 6.29-6.41 (2H, m), 6.88-6.95 (2H, m), 7.10-6.97 (2H, m), 7.45-7.32 (2H, m).

### Example 388

2-(1-{2-[(4R,6S)-8-Chloro-6-(6-fluoro-2,3-dimethoxyphenyl)-4H,6H-pyrrolo[1,2-a][4,1]benzoxazepin-4-yl]ethyl}-2H-1,2,3,4-tetrazol-5-yl)acetic acid

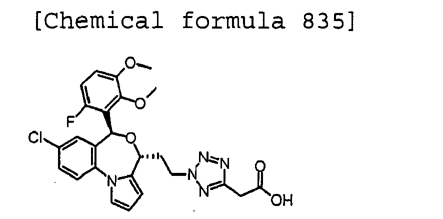

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (14 mg) was obtained from ethyl 2-(1-{2-[(4R,6S)-8-chloro-6-(6-fluoro-2,3-dimethoxyphenyl)-4H,6H-pyrrolo[1,2-a][4,1]benzoxazepin-4-yl]ethyl}-2H-1,2,3,4-tetrazol-5-yl)acetate (18 mg) by using potassium carbonate (34 mg).
MS (ESI) m/z : 550 (M + Na)⁺.
¹H-NMR (CDCl₃) δ: 2.31-2.52 and 2.64-2.78 (2H, m), 3.57 and 3.61 (3H, s), 3.81 and 3.85 (3H, s), 3.96 (2H, br s), 4.53-4.97 (3H, m), 5.92-6.01 and 6.18-6.45 (3H, m), 6.78-7.14 (4H, m), 7.31-7.44 (2H, m).
Elemental analysis for: C₂₅H₂₃ClFN₅O₅·0.3H₂O·0.2CHCl₃
Calculated: C, 54.32; H, 4.31; N, 12.57.
Found: C, 54.70; H, 4.42; N, 12.19.

### Example 389

2-(1-{2-[(4R,6S)-8-Chloro-6-(6-fluoro-2,3-dimethoxyphenyl)-4H,6H-pyrrolo[1,2-a][4,1]benzoxazepin-4-yl]ethyl}-1H-1,2,3,4-tetrazol-5-yl)acetic acid

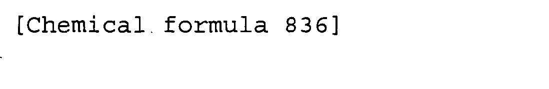

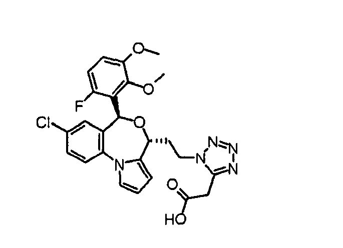

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (14 mg) was obtained from ethyl 2-(1-{2-[(4R,6S)-8-chloro-6-(6-fluoro-2,3-dimethoxyphenyl)-4H,6H-pyrrolo[1,2-a][4,1]benzoxazepin-4-yl]ethyl}-1H-1,2,3,4-tetrazol-5-yl)acetate(18 mg) by using potassium carbonate (34 mg).
MS (ESI) m/z : 528 (M + H)⁺.
¹H-NMR (CDCl₃) δ: 2.00-2.33 and 2.54-2.68 (2H, m), 3.52 and 3.54 (3H, s), 3.77-3.86 (5H, m), 4.30-4.56 and 4.75-4.83 (3H, m), 5.88-5.97 and 6.23-6.35 (3H, m), 6.81-6.91 (2H, m), 6.96-7.09 (2H, m), 7.38-7.27 (2H, m).
Elemental analysis for: C₂₅H₂₃ClFN₅O₅·1.0H₂O·0.25CHCl₃·0.25 n-hexane
Calculated: C, 53.79; H, 4.85; N, 11.72.
Found: C, 53.86; H, 4.84; N, 11.43.

### Referential Example 270

5-Methyl-2,4-dihydro-3H-pyrazol-3-one

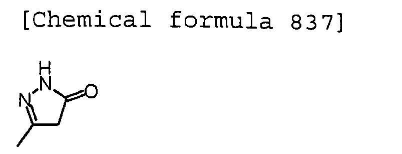

To an ethanol solution (130 mL) of ethyl acetyl acetate (10.0 g) was added hydrazine monohydrate (5.0 mL) under ice cooling. While warming to room temperature, the reaction mixture was stirred for 20 hours. The insoluble material was collected by filtration and washed with diethyl ether to give the title compound (6.3 g).
¹H-NMR (DMSO-d₆) δ: 2.07 (3H, s), 5.20 (1H, s) .

### Referential Example 271

Ethyl 2-methyl-2-[(3-methyl-1H-pyrazol-5-yl)oxy]propanoate

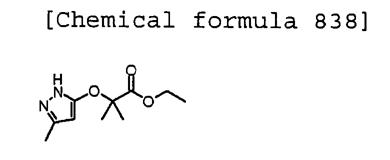

To an N,N-dimethylformamide solution (20 mL) of 5-methyl-2,4-dihydro-3H-pyrazol-3-one (2.00 g) were added ethyl 2-bromoisobutyrate (3.29 mL) and potassium carbonate (3.10 g). The resulting mixture was stirred at 100°C for 30 minutes. To the reaction mixture was added ethyl 2-bromoisobutyrate (3.29 mL). The resulting mixture was stirred at 80°C for 5 hours. The insoluble material was collected by filtration. The filtrate was concentrated under reduced pressure to give the title compound (3.75 g). MS (ESI) m/z : 213(M + H)⁺.
¹H-NMR (CDCl₃) δ: 1.20 (3H, t, J = 7.1 Hz), 1.64 (6H, s), 2.24 (3H, s), 4.19 (2H, q, J = 7.1 Hz), 5.47 (1H, s).

### Example 390

Ethyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-pyrazol-3-yl)oxy]-2-methylpropanoate

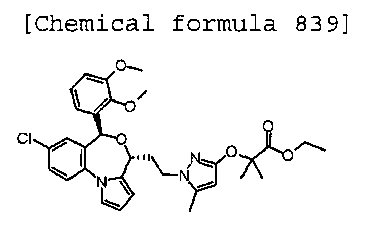

To an N,N-dimethylformamide solution (10 mL) of ethyl 2-methyl-2-[(3-methyl-1H-pyrazol-5-yl)oxy]propanoate (220 mg) was added sodium hydride (54 mg) under ice cooling. The resulting mixture was stirred for 10 minutes. Under ice cooling, 2-[(4R,6S)-8-chloro-6-(6-fluoro-2,3-dimethoxyphenyl)-4H,6H-pyrrolo[1,2-a][4,1]benzoxazepin-4-yl]ethyl methanesulfonate (515 mg) and tetrabutylammonium iodide (382 mg) were added to the reaction mixture. While warming to room temperature, the reaction mixture was stirred for 23.5 hours. The reaction mixture was concentrated under reduced pressure. To the residue were added dichloromethane, distilled water, and a saturated aqueous solution of ammonium chloride and the layers separated. The resulting organic layer was dried over anhydrous sodium sulfate. The solvent was then distilled off under reduced pressure to give the title compound (140 mg).
MS (ESI) m/z : 594 (M + H)⁺.
¹H-NMR (CDCl₃) δ: 1.12-1.17 (3H, m), 1.56 (6H, s), 2.13 (3H, s), 2.41-2.50 (2H, m), 3.42 (3H, s), 3.86 (3H, s), 4.05-4.15 (4H, m), 4.26-4.32 (1H, m), 5.32 (1H, s), 5.71 (1H, s), 6.27-6.31 (1H, m), 6.35-6.38 (1H, m), 6.70-6.73 (1H, m), 6.94-6.98 (1H, m), 7.06-7.10 (1H, m), 7.17-7.23 (1H, m), 7.37-7.30 (3H, m).

### Example 391

2-[(1-{2-[(4R,6S)-8-Chloro-6-(2,3-dimethoxyphenyl)-4H,6H-pyrrolo[1,2-a][4,1]benzoxazepin-4-yl]ethyl}-5-methyl-1H-pyrazol-3-yl)oxy]-2-methylpropanoic acid

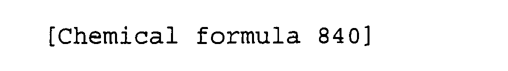

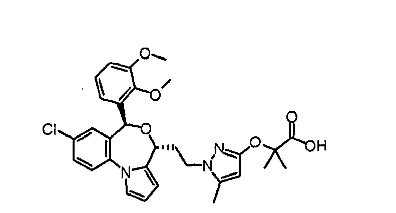

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (113 mg) was obtained from ethyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-pyrazol-3-yl)oxy]-2-methylpropanoate (155 mg) by using potassium carbonate (180 mg).
MS (ESI) m/z : 566(M + H)⁺.
¹H-NMR (CDCl₃) δ: 1.47 (3H, s), 1.49 (3H, s), 2.17 (3H, s), 2.42-2.53 (2H, m), 3.42 (3H, s), 3.86 (3H, s), 4.15-4.22 (2H, m), 4.30-4.36 (1H, m), 5.46 (1H, s), 5.71 (1H, s), 6.27-6.31 (1H, m), 6.35-6.39 (1H, m), 6.71-6.73 (1H, m), 6.93-6.98 (1H, m), 7.07-7.10 (1H, m), 7.23-7.17 (1H, m), 7.38-7.29 (3H, m).
Elemental analysis for: C₃₀H₃₂ClN₃O₆·0.25H₂O·0.25CHCl₃
Calculated: C, 60.51; H, 5.50; N, 7.00.
Found: C, 60.56; H, 5.44; N, 7.03.

### Referential Example 272

(4R,6S)-4-(2-Azidoethyl)-8-chloro-6-(2,3,-dimethoxyphenyl)-4H,6H-pyrrolo[1,2-a][4,1]benzoxazepine

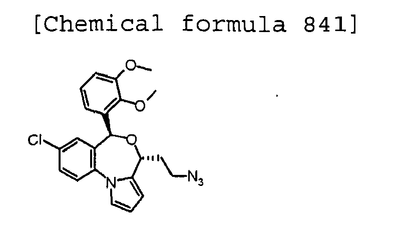

To an N,N-dimethylformamide solution (8.0 mL) of 2-[(4R,6S)-8-chloro-6-(6-fluoro-2,3-dimethoxyphenyl)-4H,6H-pyrrolo[1,2-a][4,1]benzoxazepin-4-yl]ethyl methanesulfonate(518 mg) was added sodium azide (173 mg). The resulting mixture was stirred at 40°C for 6 hours. The reaction mixture was concentrated under reduced pressure. To the residue were added ethyl acetate and distilled water and the layers separated. The organic layer thus obtained was dried over anhydrous sodium sulfate. The solvent was distilled off under reduced pressure. The residue was purified by silica gel column chromatography (n-hexane:ethyl acetate=4:1) to give the title compound (444 mg).
¹H-NMR (CDCl₃) δ: 2.20-2.29 (1H, m), 2.33-2.43 (1H, m), 3.43 (3H, s), 3.52-3.68 (2H, m), 3.85 (3H, s), 4.48-4.53 (1H, m), 5.72 (1H, s), 6.2,9-6.31 (1H, m), 6.37-6.40 (1H, m), 6.72-6.74 (1H, m), 6.93-6.97 (1H, m), 7.10-7.12 (1H, m), 7.17-7.23 (1H, m), 7.27-7.31 (2H, m), 7.40-7.33 (2H, m) .

### Referential Example 273

Methyl 2,2-dimethyl-3-(2-propynyloxy)propanoate

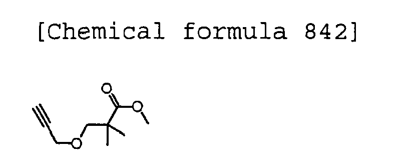

To an N,N-dimethylformamide solution (40 mL) of methyl 2,2-dimethyl-3-hydroxypropionate (611 mg) was added sodium hydride (220 mg) under ice cooling. The resulting mixture was stirred for 5 minutes. While still ice cooling, propargyl bromide (500 mg) was added to the reaction mixture. While warming to room temperature, stirring was performed overnight. Distilled water was added to the reaction mixture and extraction of an organic matter was performed using diethyl ether. The organic layers were combined, washed with saturated brine and dried over anhydrous sodium sulfate. The solvent was distilled off under reduced pressure. The residue was purified by silica gel column chromatography (n-hexane:ethyl acetate=10:1) to give the title compound (554 mg).
¹H-NMR (CDCl₃) δ: 1.21 (6H, s), 2.40-2.42 (1H, m), 3.53 (2H, s), 3.69 (3H, s), 4.16-4.13 (2H, m).

### Example 392

Methyl 3-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazolo-5-yl)methoxy]-2,2-dimethylpropanoate
Isomer A

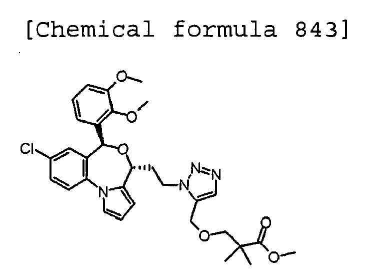

Methyl 3-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazolo-4-yl)methoxy]-2,2-dimethylpropanoate
Isomer B

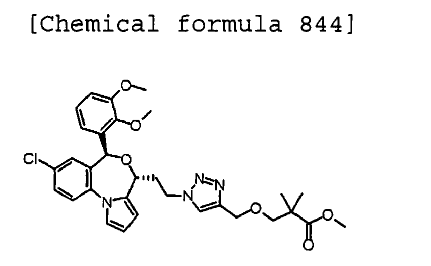

To a toluene solution (3.0 mL) of (4R,6S)-4-(2-azidoethyl)-8-chloro-6-(2,3-dimethoxyphenyl)-4H,6H-pyrrolo[1,2-a][4,1]benzoxazepine (280 mg) was added methyl 2,2-dimethyl-3-(2-propynyloxy)propanoate (224 mg). The resulting mixture was stirred at 130°C for 9 hours. The solvent was distilled off under reduced pressure. The residue was purified by silica gel column chromatography (n-hexane:ethyl acetate=4:1) to give a mixture of the title compounds. The mixture was resolved using HPLC equipped with CHIRALPAK AD (Daicel Chemical). By using 25% 2-propanol-hexane as a solvent and setting a flow rate at 10 mL/min, separation was performed to give, as the title compounds, the isomer A (108 mg) with a short retention time and the isomer B (172 mg) with a long retention time. Isomer A
MS (ESI) m/z : 595 (M + H)⁺.
¹H-NMR (CDCl₃) δ: 1.14 (3H, s), 1.14 (3H, s), 2.56-2.74 (2H, m), 3.39 (2H, s), 3.43 (3H, s), 3.59 (3H, s), 3.86 (3H, s), 4.39-4.71 (5H, m), 5.74 (1H, s), 6.31-6.33 (1H, m), 6.36-6.39 (1H, m), 6.73-6.74 (1H, m), 6.94-6.98 (1H, m), 7.08-7.10 (1H, m), 7.17-7.23 (1H, m), 7.39-7.32 (3H, m), 7.56 (1H, s).
Isomer B
MS (ESI) m/z : 595 (M + H)⁺.
¹H-NMR (CDCl₃) δ: 1.15 (3H, s), 1.16 (3H, s), 2.57-2.75 (2H, m), 3.44 (3H, s), 3.49 (2H, s), 3.65 (3H, s), 3.86 (3H, s), 4.38-4.44 (1H, m), 4.60 (2H, s), 4.62-4.74 (2H, m), 5.76 (1H, s), 6.30-6.33 (1H, m), 6.37-6.39 (1H, m), 6.73 (1H, d, J = 1.7 Hz), 6.95-7.00 (1H, m), 7.09-7.11 (1H, m), 7.18-7.24 (1H, m), 7.39-7.29 (3H, m), 7.52 (1H, s).

### Example 393

3-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazolo-5-yl)methoxy]-2,2-dimethylpropanoic acid

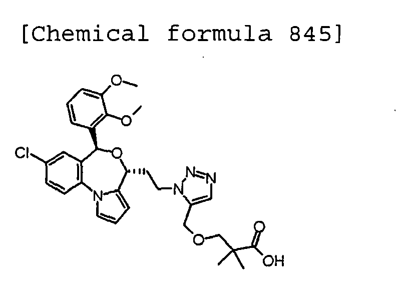

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (91 mg) was obtained from methyl 3-[(1-{2-[(4R,6S)-8-chloro-6-(2,3-dimethoxyphenyl)-4H,6H-pyrrolo[1,2-a][4,1]benzoxazepine5-yl]ethyl}-1H-1,2,3-triazolo-4-yl)methoxy]-2,2-dimethylpropanoate (93 mg) by using potassium carbonate (108 mg).
MS (ESI) m/z : 581 (M + H)⁺.
¹H-NMR (CDCl₃) δ: 1.16 (3H, s), 1.16 (3H, s), 2.55-2.74 (2H, m), 3.41 (2H, s), 3.43 (3H, s), 3.86 (3H, s), 4.41-4.70 (5H, m), 5.76 (1H, s), 6.31-6.34 (1H, m), 6.36-6.39 (1H, m), 6.74 (1H, d, J = 2.0 Hz), 6.94-6.98 (1H, m), 7.08-7.11 (1H, m), 7.17-7.24 (1H, m), 7.32-7.40 (3H, m), 7.58 (1H, s).
Elemental analysis for: C₃₀H₃₃ClN₄O₆.0.33H₂O
Calculated: C, 61.38; H, 5.78; N, 9.54.
Found: C, 61.52; H, 5.89; N, 9.14.

### Example 394

3-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazolo-4-yl)methoxy]-2,2-dimethylpropanoic acid

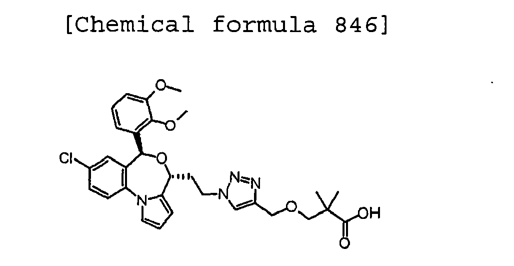

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (122 mg) was obtained from methyl 3-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazolo-4-yl)methoxy]-2,2-dimethylpropanoate (125 mg) by using potassium carbonate (145 mg) and a 1N aqueous sodium hydroxide solution (1.05 mL) .
MS (ESI) m/z : 581(M + H)⁺.
¹H-NMR (CDCl₃) δ: 1.18 (6H, s), 2.56-2.75 (2H, m), 3.44 (3H, s), 3.50 (2H, s), 3.86 (3H, s), 4.38-4.43 (1H, m), 4.60-4.75 (4H, m), 5.76 (1H, s), 6.29-6.32 (1H, m), 6.36-6.39 (1H, m), 6.72-6.74 (1H, m), 6.95-6.99 (1H, m), 7.09-7.12 (1H, m), 7.18-7.24 (1H, m), 7.28-7.40 (3H, m), 7.55 (1H, s).
Elemental analysis for: C₃₀H₃₃ClN₄O₆·1.0H₂O·0.25 n-hexane
Calculated: C, 60.96; H, 6.25; N, 9.03.
Found: C, 60.81; H, 5.89; N, 8.93.

### Example 395

Ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-4-carboxylate

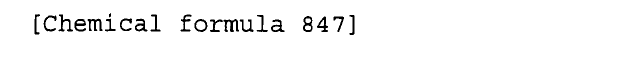

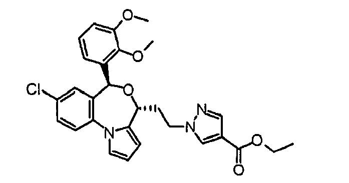

To an N,N-dimethylformamide solution (10.0 mL) of ethyl 1H-pyrazole-4-carboxylate (261 mg) was added sodium hydride (92 mg) under ice cooling. The resulting mixture was stirred for 5 minutes. To the reaction mixture were added an N,N-dimethylformamide solution (5.0 mL) of 2-[(4R,6S)-8-chloro-6-(6-fluoro-2,3-dimethoxyphenyl)-4H,6H-pyrrolo[1,2-a][4,1]benzoxazepin-4-yl]ethyl methanesulfonate (719 mg) and tetrabutylammonium iodide (556 mg). While warming to room temperature, the resulting mixture was stirred for 14 hours. The reaction mixture was concentrated under reduced pressure. To the residue were added diethyl ether and distilled water and the layers separated. The organic layer was dried over anhydrous sodium sulfate. The solvent was distilled off under reduced pressure. The residue was purified by silica gel column chromatography (dichloromethane:methanol=100:1) to give the title compound (661 mg).
¹H-NMR (CDCl₃) δ: 1.32 (3H, t, J = 7.1 Hz), 2.57-2.69 (2H, m), 3.44 (3H, s), 3.86 (3H, s), 4.27 (2H, q, J = 7.1 Hz), 4.32-4.55 (3H, m), 5.74 (1H, s), 6.30-6.33 (1H, m), 6.36-6.39 (1H, m), 6.71-6.75 (1H, m), 6.95-6.99 (1H, m), 7.08-7.11 (1H, m), 7.24-7.18 (1H, m), 7.38-7.29 (3H, m), 7.92-7.87 (2H, m).

### Referential Example 274

(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-4-yl)methanol

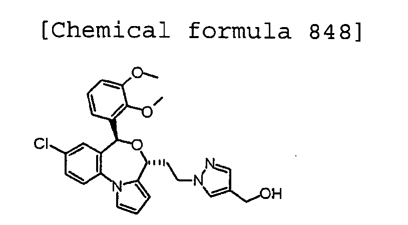

To a tetrahydrofuran solution (12 mL) of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-4-carboxylate (592 mg) was added lithium aluminum hydride (187 mg) under ice cooling. The resulting mixture was stirred for 2 hours. While still ice cooling, a 1N aqueous sodium hydroxide solution (0.46 mL) and distilled water (0.30 mL) were added successively to the reaction mixture, followed by filtration through Celite 545. The filtrate thus obtained was concentrated under reduced pressure to give the title compound (512 mg).
¹H-NMR (CDCl₃) δ: 2.56-2.63 (2H, m), 3.44 (3H, s), 3.86 (3H, s), 4.29-4.57 (5H, m), 5.73 (1H, s), 6.29-6.32 (1H, m), 6.35-6.39 (1H, m), 6.71-6.79 (1H, m), 6.93-7.00 (1H, m), 7.06-7.12 (1H, m), 7.18-7.24 (1H, m), 7.30-7.40 (3H, m), 7.48-7.41 (2H, m).

### Referential Example 275

2-(1-{2-[(4R,6S)-8-Chloro-6-(2,3-dimethoxyphenyl)-4H,6H-pyrrolo[1,2-a][4,1]benzoxazepin-4-yl]ethyl}-1H-pyrazol-4-yl)acetonitrile

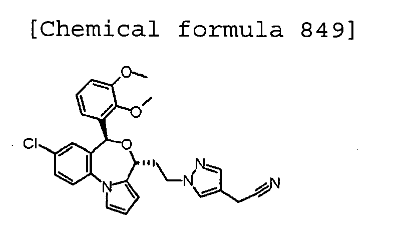

To a dichloromethane solution (5.0 mL) of (1-{2-[(4R,6S)-8-chloro-6-(2,3-dimethoxyphenyl)-4H,6H-pyrrolo[1,2-a] [4,1]benzoxazepin-4-yl]ethyl}-1H-pyrazol-4-yl)methanol (244 mg) were added triphenylphosphine (160 mg) and carbon tetrabromide (185 mg) under ice cooling. While warming to room temperature, the resulting mixture was stirred for 4.5 hours. The reaction mixture was ice cooled again. Triphenylphosphine (160 mg) and carbon tetrabromide (185 mg) were added and the resulting mixture was stirred for one hour. The solvent was distilled off under reduced pressure. The solvent was dissolved in dimethylsulfoxide and to the resulting dimethylsulfoxide solution (5.0 mL) was added sodium cyanide (124 mg). The resulting mixture was stirred at room temperature for 16 hours. To the reaction mixture were added salt and distilled water, followed by extraction with ethyl acetate. The organic layers were combined, washed successively with distilled water and saturated brine, and dried over anhydrous sodium sulfate. The solvent was distilled off under reduced pressure. The residue thus obtained was purified by silica gel column chromatography (dichloromethane:methanol=100:1) to give the title compound (139 mg).
MS (ESI) m/z : 489(M + H)⁺.
¹H-NMR (CDCl₃) δ: 2.55-2.64 (2H, m), 3.44 (3H, s), 3.53 (2H, s), 3.86 (3H, s), 4.29-4.52 (3H, m), 5.73-5.81 (1H, m), 6.28-6.40 (2H, m), 6.72-6.76 (1H, m), 6.94-7.00 (1H, m), 7.07-7.15 (1H, m), 7.19-7.24 (1H, m), 7.29-7.45 (5H, m) .

### Example 396

2-(1-{2-[(4R,6S)-8-Chloro-6-(2,3-dimethoxyphenyl)-4H,6H-pyrrolo [1, 2-a] [4,1]benzoxazepin-4-yl]ethyl}-1H-pyrazol-4-yl)acetic acid

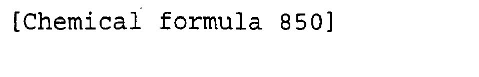

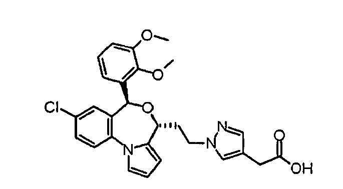

To a 2-propanol solution (2.4 mL) of 2-(1-{2-[(4R,6S)-8-chloro-6-(2,3-dimethoxyphenyl)-4H,6H-pyrrolo[1,2-a][4,1]benzoxazepin-4-yl]ethyl}-1H-pyrazol-4-yl)acetonitrile (139 mg) was added a 1N aqueous sodium hydroxide solution (2.42 mL). The resulting mixture was stirred at 85°C for 20 hours. The reaction mixture was concentrated under reduced pressure. To the residue thus obtained were added a 10% aqueous citric acid solution and dichloromethane and the layers separated. The organic layer thus obtained was dried over anhydrous sodium sulfate. The residue thus obtained was purified by thin layer chromatography (dichloromethane:methanol=20:1) to give the title compound (80 mg).
MS (ESI) m/z : 508 (M + H)⁺.
¹H-NMR (CDCl₃) δ: 2.52-2.61 (2H, m), 3.42 (3H, s), 3.44 (2H, s), 3.85 (3H, s), 4.27-4.48 (3H, m), 5.72 (1H, s), 6.25-6.32 (1H, m), 6.34-6.37 (1H, m), 6.71-6.78 (1H, m), 6.92-7.00 (1H, m), 7.06-7.13 (1H, m), 7.16-7.22 (1H, m), 7.42-7.27 (5H, m).
Elemental analysis for: C₂₇H₂₆ClN₃O₅·1.0H₂O
Calculated: C, 61.65; H, 5.37; N, 7.99.
Found: C, 61.33; H, 5.23; N, 7.64.

### Example 397

1-{2-[(4R,6S)-8-Chloro-6-(2,3-dimethoxyphenyl)-4H,6H-pyrrolo[1,2-a][4,1]benzoxazepin-4-yl]ethyl}-1H-pyrazole-4-carboxylic acid

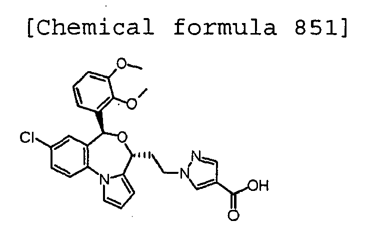

To a tetrahydrofuran-methanol mixed solution (9.0 mL-9.0 mL) of ethyl 1-{2-[(4R,6S)-8-chloro-6-(2,3-dimethoxyphenyl)-4H,6H-pyrrolo[1,2-a][4,1]benzoxazepin-4-yl]ethyl}-1H-pyrazole-4-carboxylate (470 mg) was added a 5N aqueous sodium hydroxide solution (9.0 mL). The resulting mixture was stirred at room temperature for 15 hours.
After the reaction mixture was concentrated under reduced pressure, a 10% aqueous citric acid solution and dichloromethane were added to the residue and the layers separated. The organic layers thus obtained were combined, washed with saturated brine, and dried over anhydrous sodium sulfate. The solvent was distilled off under reduced pressure and the residue thus obtained was purified by thin layer chromatography to give the title compound (280 mg).
MS (ESI) m/z : 494(M + H)⁺.
¹H-NMR (CDCl₃) δ: 2.59-2.67 (2H, m), 3.44 (3H, s), 3.86 (3H, s), 4.32-4.55 (3H, m), 5.74 (1H, s), 6.30-6.33 (1H, m), 6.36-6.39 (1H, m), 6.73-6.74 (1H, m), 6.95-6.99 (1H, m), 7.08-7.11 (1H, m), 7.18-7.24 (1H, m), 7.26-7.26 (2H, m), 7.28-7.38 (3H, m), 7.97-7.92 (2H, m).
Elemental analysis for: C₂₆H₂₄ClN₃O₅·0.25H₂O·0.25 n-hexane
Calculated: C, 63.52; H, 5.43; N, 8.08.
Found: C, 63.46; H, 5.28; N, 8.01.

### Example 398

Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-methylpropanoate

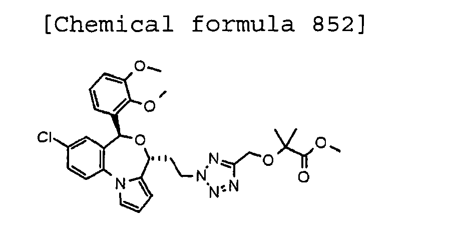

Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-methylpropanoate

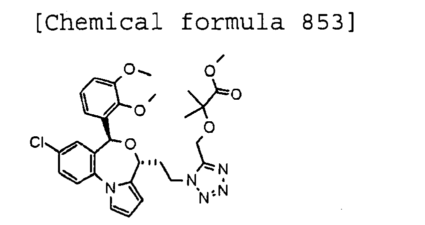

2-[(4R,6S)-8-Chloro-6-(2,3-dimethoxyphenyl)-4H,6H-pyrrolo[1,2-a][4,1]benzoxazepin-4-yl]ethyl methanesulfonate (179 mg) and methyl 2-methyl-2-(1H-1,2,3,4-tetrazol-5-ylmethoxy)propanoate (180 mg) were dissolved in N,N-dimethylformamide (4 mL). To the resulting solution were added potassium carbonate (155 mg) and tetrabutylammonium iodide (138 mg). The resulting mixture was stirred at 80°C for 4 hours. The reaction mixture was diluted with ethyl acetate and washed with saturated brine. The organic layer was dried over anhydrous sodium sulfate. After concentration of the filtrate, the residue thus obtained was purified by preparative thin layer chromatography on silica gel (ethyl acetate:hexane=1:2) to give methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-methylpropanoate (100 mg) as a low polarity substance and methyl 2-[(2-{2-[(4R,6S)-8-chloro-6-(2,3-dimethoxyphenyl)-4H,6H-pyrrolo[1,2-a][4,1]benzoxazepin-4-yl]ethyl)-1H-1,2,3,4-tetrazol-5-yl)methoxy]-2-methylpropanoate(68.9 mg) as a high polarity substance.
Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-methylpropanoate MS (ESI) m/z : 582 (M+H)⁺.
¹H-NMR (CDCl₃) δ: 1.51 (6H, d, J = 2.2 Hz), 2.73-2.81 (2H, m), 3.44(3H, s), 3.75 (3H, s), 3.86 (3H, s), 4.40-4.44 (1H, m), 4.75 (2H, s), 4.91-4.95 (2H, m), 5.75 (1H, s), 6.32-6.35 (1H, m), 6.38-6.40 (1H, m), 6.72 (1H, d, J = 2.0 Hz), 6.95-6.98 (1H, m), 7.09-7.12 (1H, m), 7.21 (1H, t, J = 8.1 Hz), 7.32-7.38 (3H, m).
Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-methylpropanoate MS (ESI) m/z : 582 (M+H)⁺.
¹H-NMR (CDCl₃) δ: 1.45 (6H, d, J = 1.5 Hz), 2.65-2.76 (2H, m), 3.43 (3H, s), 3.65 (3H, s), 3.76 (2H, s), 3.86 (3H, s), 4.48-4.52 (1H, m), 4.87-4.88 (2H, m), 5.75 (1H, s), 6.31-6.33 (1H, m), 6.37-6.38 (1H, m), 6.74 (1H, d, J = 2.2 Hz), 6.96 (1H, dd, J = 8.1, 1.5 Hz), 7.10-7.11 (1H, m), 7.18 (1H, t, J = 8.1 Hz), 7.32-7.39 (3H, m).

### Example 399

Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-ethylbutanoate

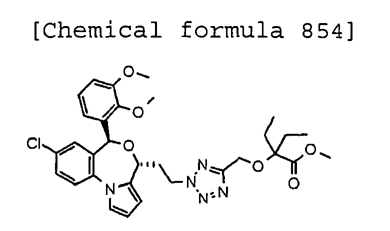

Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-ethylbutanoate

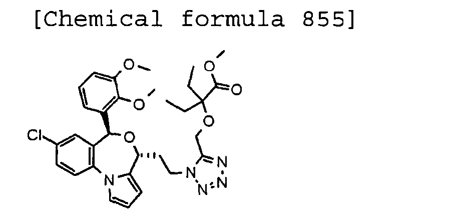

2-[(4R,6S)-8-Chloro-6-(2,3-dimethoxyphenyl)-4H,6H-pyrrolo[1,2-a][4,1]benzoxazepin-4-yl]ethyl methanesulfonate (179 mg) and methyl 2-ethyl-2-(1H-1,2,3,4-tetrazol-5-ylmethoxy)butanoate (203 mg) were treated in a similar manner to that employed for the treatment of methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-methylpropanoate and methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-methylpropanoate, methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-ethylbutanoate (101 mg) and methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-ethylbutanoate (73.5 mg) were obtained as low polarity substance and high polarity substance, respectively. Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-ethylbutanoate MS (ESI) m/z : 610 (M+H)⁺.
¹H-NMR (CDCl₃) δ: 0.86 (6H, t, J = 7.4 Hz), 1.84-1.90 (4H, m), 2.74-2.80 (2H, m), 3.44 (3H, s), 3.74 (3H, s), 3.86 (3H, s), 4.41-4.45 (1H, m), 4.68 (2H, s), 4.91-4.95 (2H, m), 5.75 (1H, s), 6.32-6.34 (1H, m), 6.39 (1H, t, J = 3.3 Hz), 6.72 (1H, d, J = 2.0 Hz), 6.97 (1H, dd, J = 8.3, 1.5 Hz), 7.10-7.11 (1H, m), 7.21 (1H, t, J = 7.9 Hz), 7.32-7.38 (3H, m).
Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-ethylbutanoate
MS (ESI) m/z : 610 (M+H)⁺.
¹H-NMR (CDCl₃) δ: 0.71-0.75 (6H, m), 1.75 (4H, q, J = 7.4 Hz), 2.55-2.63 (1H, m), 2.66-2.75 (1H, m), 3.36 (3H, s), 3.56 (3H, s), 3.79 (3H, s), 4.43-4.47 (1H, m), 4.76-4.88 (4H, m), 5.68 (1H, s), 6.25-6.26 (1H, m), 6.31 (1H, t, J = 3.2 Hz), 6.66 (1H, d, J = 2.0 Hz), 6.88 (1H, dd, J = 8.3, 1.5 Hz), 7.03-7.04 (1H, m), 7.10 (1H, t, J = 8.1 Hz), 7.24-7.32 (3H, m).

### Example 400

Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]-2-methylpropanoate

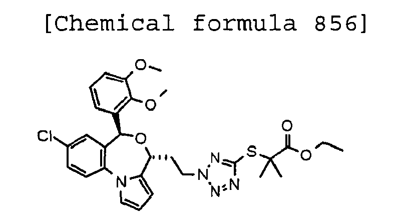

Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]-2-methylpropanoate

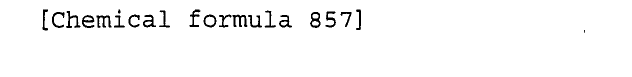

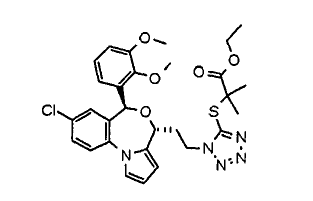

2-[(4R,6S)-8-Chloro-6-(2,3-dimethoxyphenyl)-4H,6H-pyrrolo[1,2-a] [4,1]benzoxazepin-4-yl]ethyl methanesulfonate (179 mg) and ethyl 2-methyl-2-(1H-1,2,3,4-tetrazol-5-ylsulfanyl)propanoate (162 mg) were treated in a similar manner to that employed for the treatment of methyl 2-[(2-(2-[(4R,6S)-8-chloro-6-(2,3-dimethoxyphenyl)-4H,6H-pyrrolo[1,2-a] [4,1]benzoxazepin-4-yl]ethyl)-2H-1,2,3,4-tetrazol-5-yl)methoxy]-2-methylpropanoate and methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-methylpropanoate to give ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]-2-methylpropanoate (124 mg) and ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]-2-methylpropanoate (39 mg) as low polarity substance and high polarity substance, respectively. Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]-2-methylpropanoate MS (ESI) m/z : 598 (M+H)⁺.
¹H-NMR (CDCl₃) δ: 1.17 (3H, t, J = 7.2 Hz), 1.56-1.56 (6H, m), 2.69-2.81 (2H, m), 3.44 (3H, s), 3.86 (3H, s), 4.10 (2H, q, J = 7.1 Hz), 4.36-4.40 (1H, m), 4.87-5.00 (2H, m), 5.75 (1H, s), 6.32-6.33 (1H, m), 6.38-6.39 (1H, m), 6.71 (1H, d, J = 2.2 Hz), 6.97 (1H, dd, J = 8.3, 1.5 Hz), 7.10-7.11 (1H, m), 7.22 (1H, t, J = 8.1 Hz), 7.31-7.37 (3H, m).
Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]-2-methylpropanoate MS (ESI) m/z : 598 (M+H)⁺.
¹H-NMR (CDCl₃) δ: 1.17 (3H, t, J = 7.1 Hz), 1.71-1.72 (6H, m), 2.54-2.75 (2H, m), 3.44 (3H, s), 3.86 (3H, s), 4.12 (2H, q, J = 7.2 Hz), 4.34-4.37 (1H, m), 4.63 (2H, t, J = 7.1 Hz), 5.75 (1H, s), 6.30-6.31 (1H, m), 6.37-6.39 (1H, m), 6.73 (1H, d, J = 2.2 Hz), 6.97 (1H, dd, J = 8.2, 1.3 Hz), 7.10-7.11 (1H, m), 7.22 (1H, t, J = 8.1 Hz), 7.32-7.43 (3H, m).

### Example 401

2-[(2-{2-[(4R,6S)-8-Chloro-6-(2,3-dimethoxyphenyl)-4H,6H-pyrrolo[1,2-a] [4,1]benzoxazepin-4-yl]ethyl}-2H-1,2,3,4-tetrazol-5-yl)methoxy]-2-methylpropanoic acid

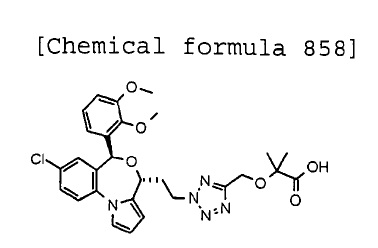

Methyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-methylpropanoate (100 mg) was dissolved in methanol-water-tetrahydrofuran (1:0.5:1, 2.5 mL). To the resulting solution was added potassium carbonate (71.3 mg). The resulting mixture was stirred overnight at room temperature. After the reaction mixture was neutralized with an ion exchange resin (Amberlite IR-120B), the resin was filtered out and the filtrate was concentrated. The residue thus obtained was purified by preparative thin layer chromatography on silica gel (methanol:chloroform=1:10) to give the title compound (64 mg).
MS (ESI) m/z : 568 (M+H)⁺.
¹H-NMR (CDCl₃) δ: 1.46-1.48 (6H, m), 2.62-2.78 (2H, m), 3.43 (3H, s), 3.85 (3H, s), 4.38-4.42 (1H, m), 4.72 (2H, s), 4.85-4.89 (2H, m), 5.73 (1H, s), 6.29-6.31 (1H, m), 6.35-6.37 (1H, m), 6.71 (1H, d, J = 2.0 Hz), 6.94-6.97 (1H, m), 7.08-7.09 (1H, m), 7.19 (1H, t, J = 7.9 Hz), 7.29-7.37 (3H, m).

### Example 402

2-[(2-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-methylpropanoic acid

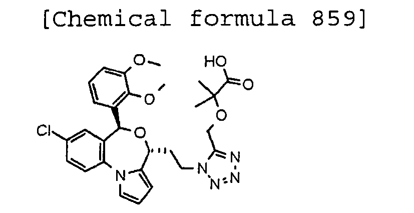

In a similar manner to that employed for the treatment of 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-methylpropanoic acid, methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-methylpropanoate (69 mg) was treated using potassium carbonate(49 mg) to give the title compound (59 mg).
MS (ESI) m/z : 568 (M+H)⁺.
¹H-NMR (CDCl₃) δ: 1.40 (6H, s), 2.60-2.73 (2H, m), 3.42 (3H, s), 3.85 (3H, s), 4.44-4.48 (1H, m), 4.63-4.83 (4H, m), 5.74 (1H, s), 6.29-6.32 (1H, m), 6.35-6.37 (1H, m), 6.72 (1H, d, J = 2.0 Hz), 6.94-6.96 (1H, m), 7.08-7.10 (1H, m), 7.18 (1H, t, J = 7.9 Hz), 7.29-7.37 (3H, m).

### Example 403

2-[(1-{2-[(4R,6S)-8-Chloro-6-(2,3-dimethoxyphenyl)-4H,6H-pyrrolo[1,2-a] [4,1]benzoxazepin-4-yl]ethyl}-2H-1,2,3,4-tetrazol-5-yl)methoxy]-2-ethylbutanoic acid

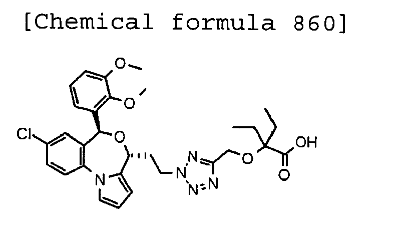

Methyl 2-[(2-{2-[(4R,6S)-8-chloro-6-(2,3-dimethoxyphenyl)-4H,6H-pyrrolo[1,2-a] [4,1]benzoxazepin-4-yl]ethyl}-2H-1,2,3,4-tetrazol-5-yl)methoxy]-2-ethylbutanoate (101 mg) was dissolved in methanol-water-tetrahydrofuran (1:0.5:1, 2.5 mL). To the resulting solution were successively added potassium carbonate (64.6 mg) and a 1N aqueous sodium hydroxide solution (1.36 mL). The resulting mixture was stirred at 50°C for 4 days and then at 80°C for 7 days. After the reaction mixture was neutralized with an ion exchange resin (Amberlite IR-120B), the resin was filtered out and the filtrate was concentrated. The residue thus obtained was purified by preparative thin layer chromatography on silica gel (methanol:chloroform=1:10) to give the title compound (76 mg).
MS (ESI) m/z : 596 (M+H)⁺.
¹H-NMR (CDCl₃) δ: 0.79-0.85 (6H, m), 1.74-1.94 (4H, m), 2.63-2.79 (2H, m), 3.43 (3H, s), 3.86 (3H, s), 4.38-4.41 (1H, m), 4.62 (2H, s), 4.87-4.91 (2H, m), 5.75 (1H, s), 6.30-6.31 (1H, m), 6.36-6.38 (1H, m), 6.71 (1H, d, J = 2.2 Hz), 6.96 (1H, dd, J = 8.3, 1.5 Hz), 7.08-7.10 (1H, m), 7.20 (1H, t, J = 7.9 Hz), 7.30-7.37 (3H, m).

### Example 404

2-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxy]-2-ethylbutanoic acid

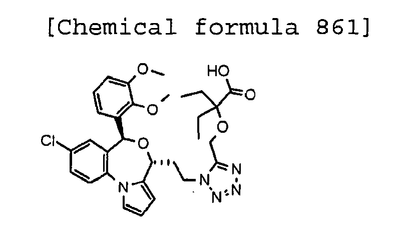

In a similar manner to that employed for the treatment of 2-[(1-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)methoxy]-2-ethylbutanoic acid, methyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)methoxyl-2-ethylbutanoate (73 mg) was treated using potassium carbonate (49.3 mg) and a 1N aqueous sodium hydroxide solution (0.72 mL) to give the title compound (31 mg).
MS (ESI) m/z : 596 (M+H)⁺.
¹H-NMR (CDCl₃) δ: 0.75-0.80 (6H, m), 1.73-1.86 (4H, m), 2.60-2.76 (2H, m), 3.43 (3H, s), 3.85 (3H, s), 4.46-4.49 (1H, m), 4.67-4.85 (4H, m), 5.75 (1H, s), 6.29-6.30 (1H, m), 6.36-6.37 (1H, m), 6.72 (1H, d, J = 2.2 Hz), 6.95 (1H, dd, J = 8.3, 1.5 Hz), 7.09-7.10 (1H, m), 7.17 (1H, t, J = 8.1 Hz), 7.27-7.29 (1H, m), 7.32-7.38 (2H, m).

### Example 405

2-[(2-{2-[(4R,6S)-8-Chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]-2-methylpropanoic acid

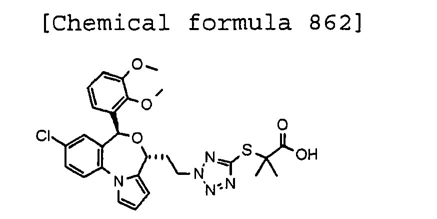

Ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)sulfanyl]-2-methylpropanoate (124 mg) was dissolved in methanol-water-tetrahydrofuran (1:0.5:1, 2.5 mL). To the resulting solution was added a 1N aqueous sodium hydroxide solution (0.42 mL). The resulting mixture was stirred at room temperature for 3 days. After the reaction mixture was neutralized with an ion exchange resin (Amberlite IR-120B), the resin was filtered out and the filtrate was concentrated. The residue thus obtained was purified by preparative thin layer chromatography on silica gel (methanol:chloroform=1:10) to give the title compound (71 mg).
MS (ESI) m/z : 570 (M+H)⁺.
¹H-NMR (CDCl₃) δ: 1.49-1.53 (6H, m), 2.66-2.79 (2H, m), 3.43 (3H, s), 3.85 (3H, s), 4.35-4.38 (1H, m), 4.89-4.93 (2H, m), 5.73 (1H, s), 6.30-6.31 (1H, m), 6.36 (1H, t, J = 3.2 Hz), 6.70 (1H, d, J = 2.2 Hz), 6.95 (1H, dd, J = 8.3, 1.5 Hz), 7.08-7.09 (1H, m), 7.20 (1H, t, J = 8.1 Hz), 7.29-7.35 (3H, m).

### Example 406

2-[(2-{2-[(4R,6S)-8-Chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]-2-methylpropanoic acid

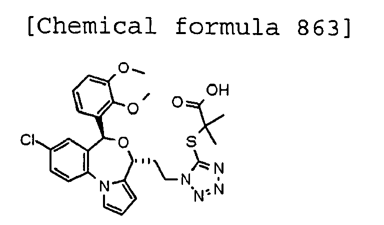

In a similar manner to that described above, ethyl 2-[(2-{2-[(4R,6S)-8-chloro-6-(2,3-dimethoxyphenyl)-4H,6H-pyrrolo[1,2-a][4,1]benzoxazepin-4-yl]ethyl}-1H-1,2,3,4-tetrazol-5-yl)sulfanyl]-2-methylpropanoate (38 mg) was treated using a 1N aqueous sodium hydroxide solution (0.12 mL) to give the title compound (29 mg).
MS (ESI) m/z : 570 (M+H)⁺.
¹H-NMR (CDCl₃) δ: 1.73 (6H, s), 2.53-2.68 (2H, m), 3.43 (3H, s), 3.86 (3H, s), 4.39-4.42 (1H, m), 4.62-4.65 (2H, m), 5.74 (1H, s), 6.30-6.31 (1H, m), 6.36-6.37 (1H, m), 6.73 (1H, d, J = 2. 2 Hz), 6.96 (1H, dd, J = 8.2, 1. 3 Hz), 7.09-7.10 (1H, m), 7.20-7.22 (1H, m), 7.33-7.37 (2H, m), 7.40-7.42 (1H, m).

### Referential Example 276

Methyl 2-(allyloxy)-2-methylpropanoate

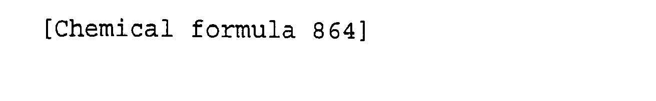

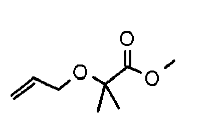

Methyl 2-hydroxy-2-methylpropanoate (3.00 g) was dissolved in N,N-dimethylformamide (60 mL). Under ice cooling, sodium hydride (55% in oil, 1.22 g) was added to the resulting solution, followed by stirring at room temperature for one hour. To the reaction mixture was added allyl bromide (3.22 mL) and the resulting mixture was stirred overnight. Water was added and the resulting mixture was extracted with ethyl acetate. The organic layer was dried over anhydrous sodium sulfate. The filtrate was concentrated to give the title compound (6.00 g).

### Referential Example 277

Methyl 2-(allyloxy)-2-ethylbutanoate

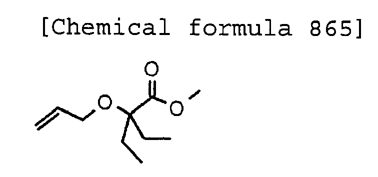

Methyl 2-ethyl-2-hydroxybutanoate (1.50 g) was dissolved in N,N-dimethylformamide (30 mL). Under ice cooling, sodium hydride (55% in oil, 0.49 g) was added to the resulting solution, followed by stirring at room temperature for one hour. To the reaction mixture was added allyl bromide (1.35 mL) and the resulting mixture was stirred overnight. Water was added and the resulting mixture was extracted with ethyl acetate. The organic layer was dried over anhydrous sodium sulfate. The filtrate was concentrated to give the title compound (1.82 g).
¹H-NMR (CDCl₃) δ: 0.83-0.87 (6H, m), 1.81 (4H, q, J = 7.5 Hz), 3.73 (3H, s), 3.88-3.90 (2H, m), 5.13-5.17 (1H, m), 5.29-5.33 (1H, m), 5.92-5.99 (1H, m).

### Referential Example 278

Methyl 2-methyl-2-(2-oxoethoxy)propanoate

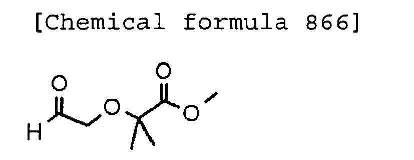

Methyl 2-(allyloxy)-2-methylpropanoate (3.00 g) was dissolved in dichloromethane (60 mL). Ozone was blown into the resulting solution for 30 minutes under ice cooling at -78°C. To the reaction mixture was added dimethylsulfide (5 mL). The resulting mixture was warmed slowly to room temperature over one night. The reaction mixture was concentrated to give the title compound (3.04 g).
¹H-NMR (CDCl₃) δ: 1.49 (6H, s), 3.74 (3H, s), 4.04-4.07 (2H, m), 9.75 (1H, s).

### Referential Example 279

Methyl 2-ethyl-2-(2-oxoethoxy)butanoate

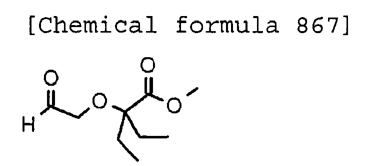

Methyl 2-(allyloxy)-2-ethylbutanoate (1.82 g) was dissolved in dichloromethane (40 mL). Ozone was blown into the resulting solution for 30 minutes under ice cooling at -78°C. To the reaction mixture was added dimethylsulfide (4 mL) and the resulting mixture was warmed slowly to room temperature over one night. The reaction mixture was concentrated to give the title compound(1.84 g).
¹H-NMR (CDCl₃) δ: 0.83-0.91 (6H, m), 1.78-1.84 (4H, m), 3.73 (3H, s), 3.99-3.99 (2H, m), 9.78-9.80 (1H, m).

### Referential Example 280

2-(2-Methoxy-1,1-dimethyl-2-oxoethoxy)acetic acid

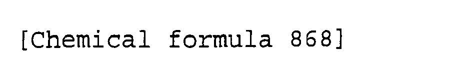

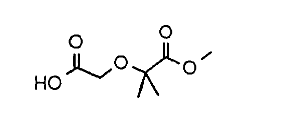

Methyl 2-methyl-2-(2-oxoethoxy)propanoate (3.04 g) was dissolved in a solvent mixture of tert-butanol (30 mL) and water (30 mL). Under ice cooling, monosodium phosphate dihydrate (5.92 g), sulfamic acid (5.52 g) and sodium chlorite (5.15 g) were added to the resulting solution, followed by stirring at room temperature for 30 minutes.
To the reaction mixture was added a 20% aqueous solution of sodium thiosulfate, followed by extraction with chloroform. The organic layer was dried over anhydrous sodium sulfate. The filtrate was then concentrated to give the title compound (1.44 g).
¹H-NMR (CDCl₃) δ: 1.50-1.51 (6H, m), 3.77-3.78 (3H, m), 4.10 (2H, s).

### Referential Example 281

2-[1-Ethyl-1-(methoxycarbonyl)propoxy]acetic acid

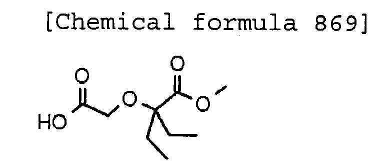

Methyl 2-ethyl-2-(2-oxoethoxy)butanoate (1.84 g) was dissolved in a solvent mixture of tert-butanol (10 mL) and water (10 mL). Under ice cooling, monosodium phosphate dihydrate (3.05 g), sulfamic acid (2.85 g) and sodium chlorite (2.65 g) were added, followed by stirring at room temperature for 30 minutes. To the reaction mixture was added a 20% aqueous solution of sodium thiosulfate. The resulting mixture was extracted with chloroform. The organic layer was dried over anhydrous sodium sulfate. The filtrate was then concentrated to give the title compound (1.80 g).
¹H-NMR (CDCl₃) δ: 0.88-0.92 (6H, m), 1.75-1.93 (4H, m), 3.78 (3H, s), 4.07 (2H, s).

### Referential Example 282

Methyl 2-{2-[(2-cyanoethyl)amino]-2-oxoethoxy}-2-methylpropanoate

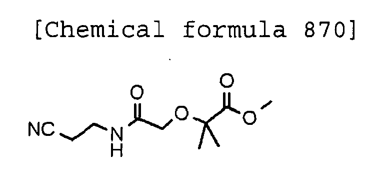

2-(2-Methoxy-1,1-dimethyl-2-oxoethoxy)acetic acid (1.44 g) was dissolved in dichloromethane (30 mL). To the resulting solution were added 3-aminopropionitrile (0.90 mL), 1-ethyl-3-(3-dimethylaminopropyl)carbodiimide hydrochloride (1.88 g), and 1-hydroxybenzotriazole (0.38 g). The resulting mixture was stirred at room temperature for 3 days. The reaction mixture was diluted with chloroform and washed with saturated brine. The organic layer was dried over anhydrous sodium sulfate. The filtrate was then concentrated to give the title compound (998 mg).
¹H-NMR (CDCl₃) δ: 1.48 (6H, s), 2.67 (2H, t, J = 6.5 Hz), 3.59 (2H, q, J = 6.5 Hz), 3.76 (3H, s), 3.96 (2H, s).

### Referential Example 283

Methyl 2-{2-[(2-cyanoethyl)amino]-2-oxoethoxy}-2-ethylbutanoate

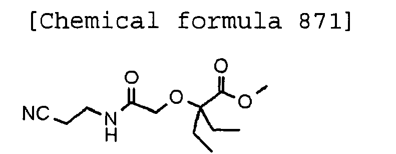

2-[1-Ethyl-1-(methoxycarbonyl)propoxy]acetic acid (1.80 g) was dissolved in dichloromethane (40 mL). To the resulting solution were added 3-aminopropionitrile (0.98 mL), 1-ethyl-3-(3-dimethylaminopropyl)carbodiimide hydrochloride (2.03 g), and 1-hydroxybenzotriazole (0.40 g). The resulting mixture was stirred overnight at room temperature. The reaction mixture was diluted with chloroform and washed with saturated brine. The organic layer was dried over anhydrous sodium sulfate. The filtrate was then concentrated to give the title compound (1.12 g).
¹H-NMR (CDCl₃) δ: 0.86-0.89 (6H, m), 1.77-1.87 (4H, m), 2.66 (2H, t, J = 6.5 Hz), 3.58-3.63 (2H, m), 3.75 (3H, s), 3.93 (2H, s).

### Referential Example 284

Methyl 2-{[1-(2-cyanoethyl)-1H-1,2,3,4-tetrazol-5-yl]methoxy}-2-methylpropanoate

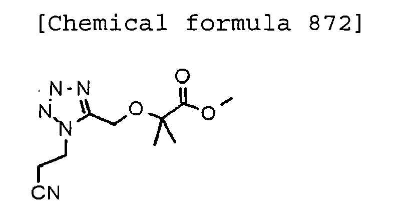

Methyl 2-{2-[(2-cyanoethyl)amino]-2-oxoethoxy}-2-methylpropanoate (921 mg) was dissolved in acetonitrile (20 mL). Under a nitrogen atmosphere, sodium azide (393 mg) and trifluoromethanesulfonic anhydride (1.02 mL) were added at 0°C and the resulting mixture was stirred overnight at room temperature. To the reaction mixture was added a saturated aqueous solution of sodium bicarbonate. The resulting mixture was extracted with ethyl acetate. The organic layer was dried over anhydrous sodium sulfate. After concentration of the filtrate, the residue was purified by silica gel column (ethyl acetate:hexane=1:1) to give the title compound (221 mg).
¹H-NMR (CDCl₃) δ: 1.55 (6H, s), 3.09 (2H, t, J = 7.2 Hz), 3.79 (3H, s), 4.93 (2H, s), 5.01 (2H, t, J = 7.2 Hz).

### Referential Example 285

Methyl 2-{[1-(2-cyanoethyl)-1H-1,2,3,4-tetrazol-5-yl]methoxy}-2-ethylbutanoate

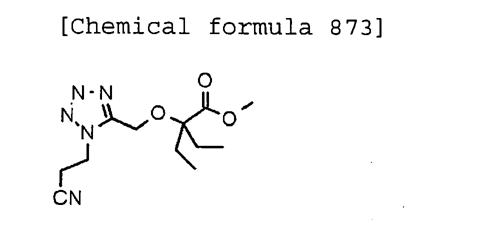

Methyl 2-{2-[(2-cyanoethyl)amino]-2-oxoethoxy}-2-ethylbutanoate (1.80 g) was dissolved in acetonitrile (40 mL). Under a nitrogen atmosphere, sodium azide (0.86 g) and trifluoromethanesulfonic anhydride (2.22 mL) were added to the resulting solution at 0°C and the resulting mixture was stirred overnight at room temperature. To the reaction mixture was added a saturated aqueous solution of sodium bicarbonate. The resulting mixture was extracted with ethyl acetate. The organic layer was dried over anhydrous sodium sulfate. After concentration of the filtrate, the residue was purified by silica gel column (ethyl acetate:hexane=1:2) to give the title compound (779 mg).
¹H-NMR (CDCl₃) δ: 0.90 (6H, t, J = 7.6 Hz), 1.87-1.93 (4H, m), 3.11 (2H, t, J = 7.2 Hz), 3.79 (3H, s), 4.90 (2H, s), 5.01 (2H, t, J = 7.2 Hz).

### Referential Example 286

Methyl 2-methyl-2-(1H-1,2,3,4-tetrazol-5-ylmethoxy)propanoate

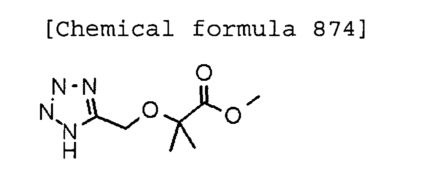

Methyl 2-{[1-(2-cyanoethyl)-1H-1,2,3,4-tetrazol-5-yl]methoxy}-2-methylpropanoate (221 mg) was dissolved in dichloromethane (4 mL). To the resulting solution was added 1,8-diazabicyclo[5,4,0]undec-7-ene (0.326 mL) at room temperature. The resulting mixture was stirred overnight at room temperature. The reaction mixture was concentrated to give the crudely purified product of the title compound.

### Referential Example 287

Methyl 2-ethyl-2-(1H-1,2,3,4-tetrazol-5-ylmethoxy)butanoate

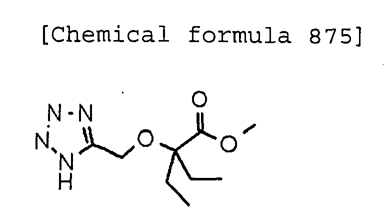

Methyl 2-{[1-(2-cyanoethyl)-1H-1,2,3,4-tetrazol-5-yl]methoxy}-2-ethylbutanoate (250 mg) was dissolved in dichloromethane (5 mL). To the resulting solution was added 1,8-diazabicyclo[5,4,0]undec-7-ene (0.332 mL) at room temperature. The resulting mixture was stirred overnight at room temperature. The reaction mixture was concentrated to give the crudely purified product of the title compound.

### Referential Example 288

Methyl 4-methoxybenzyl carbamodithioate

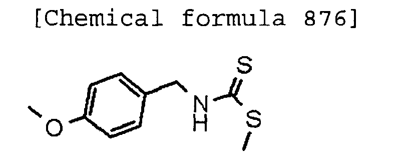

p-Methoxybenzylamine (2.00 g) was dissolved in tetrahydrofuran (40 mL). Under ice cooling, triethylamine (2.44 mL) and carbon disulfide (0.965 mL) were added to the resulting solution and the mixture was stirred for one hour. To the reaction mixture was added methyl iodide (0.908 mL). The reaction mixture was warmed to room temperature and stirred overnight. The reaction mixture was concentrated. The residue was diluted with ethyl acetate and washed successively with an aqueous solution of citric acid, a saturated aqueous solution of sodium bicarbonate, and saturated brine. The organic layer was dried over anhydrous sodium sulfate. The filtrate was then concentrated to give the title compound (3.25 g).

### Referential Example 289

1-(4-Methoxybenzyl)-1H-1,2,3,4-tetrazol-5-thiol

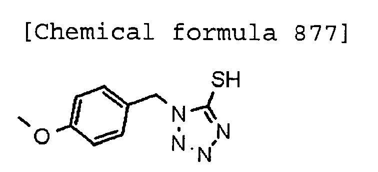

Methyl 4-methoxybenzyl carbamodithioate (3.25 g) was dissolved in a solvent mixture of ethanol (50 mL) and water (10 mL). To the resulting solution was added sodium azide (1.39 g). The resulting mixture was stirred overnight at 60°C. After cooling to room temperature, the reaction mixture was poured into 1N hydrochloric acid under ice cooling, followed by extraction with chloroform. The organic layer was dried over anhydrous sodium sulfate. The filtrate was then concentrated. The residue was purified by silica gel column (methanol:chloroform=1:10) to give the title compound (2.32 g).

### Referential Example 290

Ethyl 2-{[1-(4-methoxybenzyl)-1H-1,2,3,4-tetrazol-5-yl]sulfanyl}-2-methylpropanoate

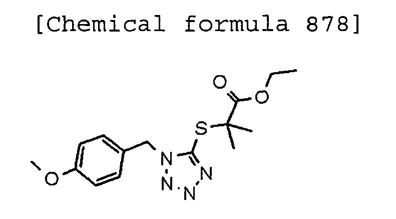

1-(4-Methoxybenzyl)-1H-1,2,3,4-tetrazol-5-thiol (500 mg) was dissolved in N,N-dimethylformamide (10 mL). To the resulting solution were added potassium carbonate (404 mg) and ethyl 2-bromo-2-methylpropanoate (0.825 mL). The resulting mixture was stirred at 80°C for 4 hours. After cooling to room temperature, the reaction mixture was diluted with ethyl acetate and washed with water. The organic layer was dried over anhydrous sodium sulfate. The filtrate was then concentrated to give the title compound (959 mg) .
MS (ESI) m/z : 337 (M+H)⁺.
¹H-NMR (CDCl₃) δ: 1.18 (3H, t, J = 7.2 Hz), 1.74 (6H, s), 3.79 (3H, s), 4.13 (2H, q, J = 7.2 Hz), 5.43 (2H, s), 6.87 (2H, d, J = 8.5 Hz), 7.26 (4H, d, J = 8.5 Hz).

### Referential Example 291

Ethyl 2-methyl-2-(1H-1,2,3,4-tetrazol-5-ylsulfanyl)propanoate

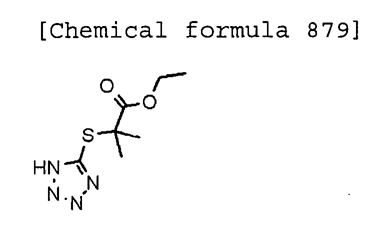

Ethyl 2-{[1-(4-methoxybenzyl)-1H-1,2,3,4-tetrazol-5-yl]sulfanyl}-2-methylpropanoate (757 mg) was dissolved in trifluoroacetic acid (10 mL). To the resulting solution was added anisole (5 drops) and the resulting mixture was stirred at 60°C for 2.5 hours. After cooling to room temperature, the reaction mixture was concentrated. The residue thus obtained was purified by preparative thin layer chromatography on silica gel (methanol:chloroform=1:10) to give the title compound (480 mg).

### Example 407

Methyl 2-[(1-{2-[(4R,65)-8-chloro-6-(2,3-dimethoxyphenyl)-4H,6H-pyrrolo[1,2-a][4,1]benzoxazepin-4-yl]ethyl}-5-methyl-1H-1,2,3-triazol-5-yl)methoxy]-2-methylpropanoate

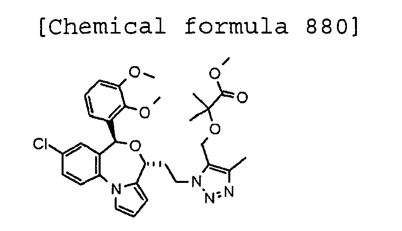

Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoate

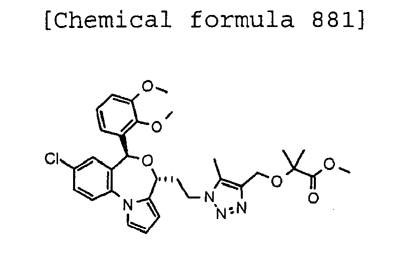

4-(2-Azidoethyl)-8-chloro-6-(2,3-dimethoxyphenyl)-4H,6H-pyrrolo[1,2-a][4,1]benzoxazepine (270 mg) was dissolved in toluene (5 mL). To the resulting solution was added methyl 2-(2-butynyloxy)-2-methylpropanoate (270 mg). The resulting mixture was heated in a sealed tube at 130°C for 6 days. After concentration of the reaction mixture, the residue thus obtained was purified by preparative thin layer chromatography on silica gel (ethyl acetate:hexane=1:1). From the isomer mixture (256 mg) thus obtained, position isomers were separated and purified using CHIRALPAK-AD (Daicel Chemical Industries) to give methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-5-yl)methoxy]-2-methylpropanoate (isomer A, 120 mg) and methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoate (isomer B, 155 mg), respectively.
Flow rate: 15 mL/min
Retention time: 9 minutes for isomer A, 14 minutes for isomer B
Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-5-yl)methoxy]-2-methylpropanoate (isomer A)
MS (ESI) m/z : 595 (M+H)⁺.
¹H-NMR (CDCl₃) δ: 1.42-1.43 (6H, m), 2.30 (3H, s), 2.60-2.75 (2H, m), 3.43 (3H, s), 3.65 (3H, s), 3.86 (3H, s), 4.46-4.52 (3H, m), 4.60-4.75 (2H, m), 5.75 (1H, s), 6.31-6.32 (1H, m), 6.36-6.38 (1H, m), 6.73 (1H, d, J = 2.2 Hz), 6.95 (1H, dd, J = 8.3, 1.5 Hz), 7.09-7.10 (1H, m), 7.17 (1H, t, J = 7.9 Hz), 7.33-7.39 (3H, m).
Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoate (isomer B)
MS (ESI) m/z : 595 (M+H)⁺.
¹H-NMR (CDCl₃) δ: 1.48-1.49 (6H, m), 2.36 (3H, s), 2.55-2.67 (2H, m), 3.43 (3H, s), 3.75 (3H, s), 3.86 (3H, s), 4.40-4.44 (1H, m), 4.48-4.60 (4H, m), 5.75 (1H, s), 6.30-6.31 (1H, m), 6.37-6.39 (1H, m), 6.73 (1H, d, J = 2.2 Hz), 6.97 (1H, dd, J = 8.3, 1.5 Hz), 7.10-7.11 (1H, m), 7.21 (1H, t, J = 7.9 Hz), 7.31-7.39 (3H, m).

### Example 408

Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-ethylbutanoate

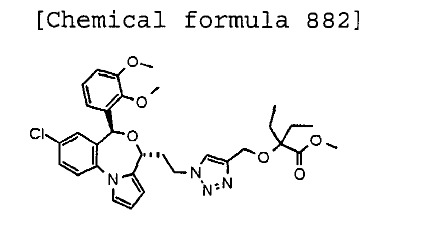

Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5yl)methoxy]-2-ethylbutanoate

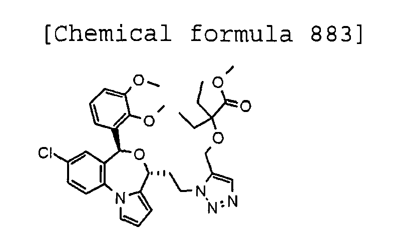

4-(2-Azidoethyl)-8-chloro-6-(2,3-dimethoxyphenyl)-4H,6H-pyrrolo[1,2-a][4,1]benzoxazepine (140 mg) and methyl 2-ethyl-2-(2-propynyloxy)butanoate (182 mg) were treated in a similar manner to that employed for the treatment of methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-5-yl)methoxy]-2-methylpropanoate and methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoate. Then, position isomers were separated and purified by silica gel column chromatography to give, a low polarity substance, methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-ethylbutanoate (140 mg) and as a high polarity substance, methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5yl)methoxy]-2-ethylbutanoate(117 mg).
Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-ethylbutanoate
MS (ESI) m/z : 609 (M+H)⁺.
¹H-NMR (CDCl₃) δ: 0.83-0.87 (6H, m), 1.85 (4H, q, J = 7.5 Hz), 2.58-2.74 (2H, m), 3.44 (3H, s), 3.73 (3H, s), 3.86 (3H, s), 4.40-4.44 (1H, m), 4.52-4.59 (2H, m), 4.62-4.72 (2H, m), 5.76 (1H, s), 6.31-6.32 (1H, m), 6.37-6.39 (1H, m), 6.73 (1H, d, J = 2.0 Hz), 6.96-6.98 (1H, m), 7.10-7.11 (1H, m), 7.22 (1H, t, J = 7.9 Hz), 7.31-7.38 (4H, m), 7.64 (1H, s).
Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5yl)methoxy]-2-ethylbutanoate
MS (ESI) m/z : 609 (M+H)⁺.
¹H-NMR (CDCl₃) δ: 0.77-0.83 (6H, m), 1.76-1.83 (4H, m), 2.62-2.80 (2H, m), 3.43 (3H, s), 3.63 (3H, s), 3.86 (3H, s), 4.50-4.56 (2H, m), 4.65-4.81 (2H, m), 5.75 (1H, s), 6.32-6.34 (1H, m), 6.36-6.38 (1H, m), 6.73 (1H, d, J = 2.2 Hz), 6.95 (1H, dd, J = 8.1, 1.5 Hz), 7.09-7.10 (1H, m), 7.17 (1H, t, J = 8.1 Hz), 7.32-7.38 (3H, m), 7.57 (1H, s).

### Example 409

2-[(1-{2-[(4R,6S)-8-Chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoic acid

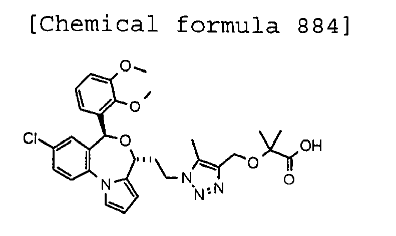

Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoate (155 mg) was dissolved in methanol-water-tetrahydrofuran (1:0.5:1, 2.5 mL). To the resulting solution was added potassium carbonate (108 mg). The resulting mixture was stirred at room temperature for 7 days. After the reaction mixture was neutralized with an ion exchange resin (Amberlite IR-120B), the resin was filtered out and the filtrate was concentrated. The residue thus obtained was purified by preparative thin layer chromatography on silica gel (methanol:chloroform=1:10) to give the title compound (100 mg).
MS (ESI) m/z : 581 (M+H)⁺.
¹H-NMR (CDCl₃) δ: 1.48 (6H, s), 2.26 (3H, s), 2.50-2.65 (2H, m), 3.42 (3H, s), 3.86 (3H, s), 4.39-4.57 (5H, m), 5.74 (1H, s), 6.27-6.28 (1H, m), 6.36 (1H, t, J = 3.3 Hz), 6.72 (1H, d, J = 2.2 Hz), 6.96 (1H, dd, J = 8.3, 1.5 Hz), 7.09-7.10 (1H, m), 7.20 (1H, t, J = 8.1 Hz), 7.29-7.38 (3H, m) .

### Example 410

2-[(1-{2-[(4R,6S)-8-Chloro-6-(2,3-dimethoxyphenyl)-4H,6H-pyrrolo[1,2-a] [4,1]benzoxazepin-4-yl]ethyl}-4-methyl-1H-1,2,3-triazol-5-yl)methoxy]-2-methylpropanoic acid

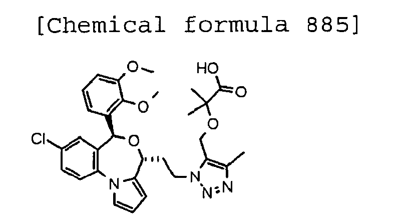

In a similar manner to that employed for the treatment of 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-4-yl)methoxy]-2-methylpropanoic acid, methyl 2-[(1-{2-[(4R,6S)-8-chloro-6-(2,3-dimethoxyphenyl)-4H,6H-pyrrolo[1,2-a][4,1]benzoxazepin-4-yl]ethyl}-5-methyl-1H-1,2,3-triazol-5-yl)methoxy]-2-methylpropanoate (120 mg) was treated using potassium carbonate (84 mg) to give the title compound (100 mg).
MS (ESI) m/z : 581 (M+H)⁺.
¹H-NMR (CDCl₃) δ: 1.40-1.41 (6H, m), 2.25 (3H, s), 2.62-2.68 (2H, m), 3.41 (3H, s), 3.84 (3H, s), 4.44-4.51 (3H, m), 4.57-4.73 (2H, m), 5.74 (1H, s), 6.28-6.31 (1H, m), 6.34 (1H, t, J = 3.3 Hz), 6.72 (1H, d, J = 2.0 Hz), 6.93-6.94 (1H, m), 7.07-7.08 (1H, m), 7.16 (1H, t, J = 7.9 Hz), 7.31-7.37 (3H, m).

### Example 411

2-[(1-{2-[(4R,6S)-8-Chloro-6-(2,3-dimethoxyphenyl)-4H,6H-pyrrolo [1, 2-a] [4,1]benzoxazepin-4-yl]ethyl}-1H-1,2,3-triazol-4-yl)methoxy]-2-ethylbutanoic acid

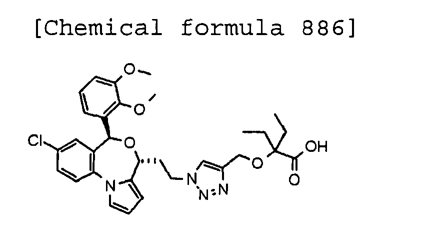

Methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-ethylbutanoate (140 mg) was dissolved in methanol-water-tetrahydrofuran (1:0.5:1, 2.5 mL). To the resulting solution was added a 5N aqueous sodium hydroxide solution (0.23 mL). The resulting mixture was stirred overnight at 80°C. After the reaction mixture was neutralized with an ion exchange resin (Amberlite IR-120B), the resin was filtered out and the filtrate was concentrated. The residue thus obtained was purified by preparative thin layer chromatography on silica gel (methanol:chloroform=1:10) to give the title compound (92 mg).
MS (ESI) m/z : 595 (M+H)⁺.
¹H-NMR (CDCl₃) δ: 0.77-0.86 (6H, m), 1.75-1.90 (4H, m), 2.53-2.70 (2H, m), 3.43 (3H, s), 3.86 (3H, s), 4.37-4.41 (1H, m), 4.47-4.51 (2H, m), 4.59-4.68 (2H, m), 5.75 (1H, s), 6.28-6.29 (1H, m), 6.35-6.37 (1H, m), 6.72 (1H, d, J = 2.0 Hz), 6.95-6.97 (1H, m), 7.08-7.10 (1H, m), 7.20 (1H, t, J = 7.9 Hz), 7.27-7.37 (3H, m), 7.59 (1H, s).

### Example 412

2-[(1-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-5-yl)methoxy]-2-ethylbutanoic acid

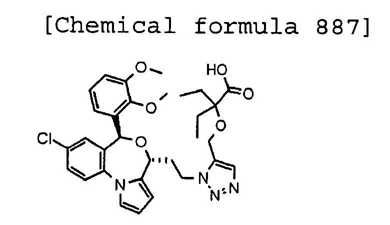

In a similar manner to that employed for the treatment of 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-4-yl)methoxy]-2-ethylbutanoic acid, methyl 2-[(1-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-5yl)methoxy]-2-ethylbutanoate (117 mg) was treated using a 5N aqueous sodium hydroxide solution (0.19 mL) to give the title compound (72.3 mg). MS (ESI) m/z : 595 (M+H)⁺.
¹H-NMR (CDCl₃) δ: 0.77-0.82 (6H, m), 1.74-1.85 (4H, m), 2.66-2.72 (2H, m), 3.43 (3H, s), 3.85 (3H, s), 4.47-4.56 (3H, m), 4.63-4.79 (2H, m), 5.74 (1H, s), 6.29-6.30 (1H, m), 6.35-6.36 (1H, m), 6.72 (1H, d, J = 2.2 Hz), 6.94 (1H, dd, J = 8.2, 1.3 Hz), 7.08-7.09 (1H, m), 7.16 (1H, t, J = 7.9 Hz), 7.29-7.37 (3H, m), 7.59 (1H, s).

### Referential Example 292

Methyl 2-(2-butynyloxy)-2-methylpropanoate

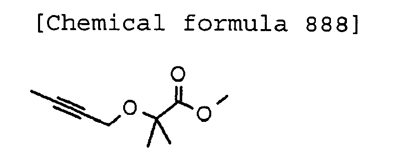

Methyl 2-hydroxy-2-methylpropanoate (1.50 g) was dissolved in N,N-dimethylformamide (30 mL). Under ice cooling, sodium hydride (55% in oil, 0.61 g) was added to the resulting solution. The resulting mixture was stirred at room temperature for one hour. To the reaction mixture was added 1-bromo-2-butyne (1.67 mL) and the mixture was stirred overnight. To the reaction mixture was added water, followed by extraction with ethyl acetate. The organic layer was dried over anhydrous sodium sulfate. After concentration of the filtrate, the residue was purified by silica gel column (ethyl acetate:hexane=1:8) to give the title compound (1.11 g).
¹H-NMR (CDCl₃) δ: 1.46-1.47 (6H, m), 1.84-1.84 (3H, m), 3.74-3.75 (3H, m), 4.10-4.11 (2H, m).

### Referential Example 293

Methyl 2-ethyl-2-(2-propynyloxy)butanoate

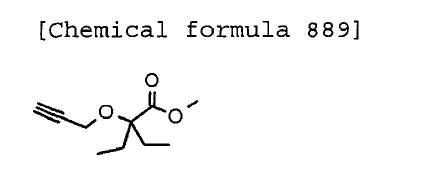

Methyl 2-ethyl-2-hydroxybutanoate (1.10 g) was dissolved in N,N-dimethylformamide (20 mL). Under ice cooling, sodium hydride (55% in oil, 0.36 g) was added to the resulting solution. The resulting mixture was stirred at room temperature for one hour. To the reaction mixture was added propargyl bromide (0.85 mL), followed by stirring overnight. To the reaction mixture was added water and the resulting mixture was extracted with ethyl acetate. The organic layer was dried over anhydrous sodium sulfate. After concentration of the filtrate, the residue was purified by silica gel column (ethyl acetate:hexane=1:5) to give the title compound (0.38 g).
¹H-NMR (CDCl₃) δ: 0.85-0.90 (6H, m), 1.80-1.84 (4H, m), 2.42 (1H, t, J = 2.4 Hz), 3.74 (3H, s), 4.13 (2H, d, J = 2.4 Hz).

### Example 413

Ethyl (3S)-1-{3-[(4R,6S)-1,8-dichloro-6-(2,3-dimethoxyphenyl)-4H,6H-pyrrolo[1,2-a][4,1]benzoxazepin-4-yl]propanoyl}-3-piperidine carboxylate

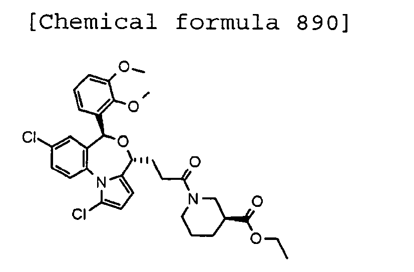

3-[(4R,6S)-1,8-Dichloro-6-(2,3-dimethoxyphenyl)-4H,6H-pyrrolo[1,2-a][4,1]benzoxazepin-4-yl]propanoic acid (218 mg) and ethyl (3S)-3-piperidine carboxylate (81.5 mg) were dissolved in dichloromethane (5mL). To the resulting solution were added 1-ethyl-3-(3-dimethylaminopropyl)carbodiimide hydrochloride (99 mg) and 1-hydroxybenzotriazole (22 mg). The resulting mixture was stirred overnight at room temperature. The reaction mixture was diluted with chloroform and washed with saturated brine. The organic layer was dried over anhydrous sodium sulfate. After concentration of the filtrate, the residue thus obtained was purified by silica gel column (ethyl acetate:hexane=1:2) to give the title compound (197 mg).
MS (ESI) m/z : 601 (M+H)⁺.
¹H-NMR (CDCl₃)δ: 1.23-1.26 (3H, m), 1.37-1.49 (1H, m), 1.64-1.70 (1H, m), 1.77-1.80 (1H, m), 1.98-2.09 (1H, m), 2.33-2.85 (6H, mm), 3.00-3.08 (1H, m), 3.42 (3H, s), 3.74-3.82 (1H, m), 3.85 (3H, s), 3.99-4.18 (3H, m), 4.21-4.25 (1H, m), 5.61 (1H, s), 6.29-6.31 (2H, m), 6.75-6.75 (1H, m), 6.94-6.96 (1H, m), 7.16-7.18 (1H, m), 7.24-7.29 (1H, m), 7.39 (1H, dd, J = 8.5, 2.4 Hz), 7.49 (1H, d, J = 8.5 Hz).

### Example 414

(3S)-1-{3-[(4R,6S)-1,8-Dichloro-6-(2,3-dimethoxyphenyl)-4H,6H-pyrrolo[1,2-a][4,1]benzoxazepin-4-yl]propanoyl}-3-piperidine carboxylic acid

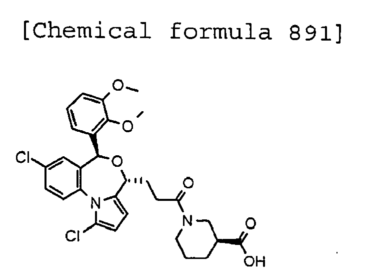

Ethyl (3S)-1-{3-[(4R,6S)-1,8-dichloro-6-(2,3-dimethoxyphenyl)-4H,6H-pyrrolo[1,2-a][4,1]benzoxazepin-4-yl]propanoyl}-3-piperidine carboxylate (197 mg) was dissolved in methanol-water-tetrahydrofuran (1:0.5:1, 5 mL). To the resulting solution was added lithium hydroxide monohydrate (27.5 mg). The resulting mixture was stirred at room temperature for 3 days. After the reaction mixture was neutralized with an ion exchange resin "Amberlite IR-120B", the resin was filtered out and the filtrate was concentrated to give the title compound (176 mg).
MS (ESI) m/z : 573 (M+H)⁺.
¹H-NMR (CDCl₃) δ: 1.43-1.48 (1H, m), 2.00-2.03 (1H, m), 2.33-2.35 (2H, m), 2.51-2.61 (4H, m), 3.13-3.16 (2H, m), 3.41 (3H, s), 3.46-3.49 (1H, m), 3.77-3.95 (4H, m), 4.20-4.23 (1H, m), 4.66-4.68 (1H, m), 5.61-5.61 (1H, m), 6.29 (2H, s), 6.75 (1H, d, J = 2.2 Hz), 6.93-6.94 (1H, m), 7.11-7.18 (1H, m), 7.23-7.27 (1H, m), 7.38-7.40 (1H, m), 7.49 (1H, d, J = 8.5 Hz).

### Example 415

Ethyl 2-(2-{2-[(4R,6S)-1,8-dichloro-6-(2,3-dimethoxyphenyl)-4H,6H-pyrrolo[1,2-a][4,1]benzoxazepin-4-yl]ethyl}-2H-1,2,3,4-tetrazol-5-yl)acetate

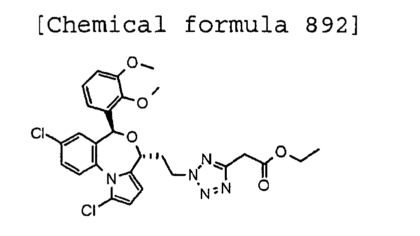

Ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (436 mg) was dissolved in tetrahydrofuran (10 mL). To the resulting solution was added N-chlorosuccinimide (119 mg). The resulting mixture was stirred overnight at room temperature. Water was added and the resulting mixture was extracted with chloroform. The organic layer was dried over anhydrous sodium sulfate. After concentration of the filtrate, the residue thus obtained was purified by silica gel column (ethyl acetate:n-hexane=1:2) to give the title compound (327 mg).
MS (ESI) m/z : 572 (M+H⁺).
¹H-NMR (CDCl₃) δ: 1.25 (3H, t, J = 7.1 Hz), 2.70-2.75 (2H, m), 3.43 (3H, s), 3.85 (3H, s), 3.92 (2H, s), 4.19 (2H, q, J = 7.1 Hz), 4.28 (1H, dd, J = 9.4, 4.3 Hz), 4.87-4.92 (2H, m), 5.67 (1H, s), 6.27 (1H, d, J = 3.7 Hz), 6.30 (1H, d, J = 3.7 Hz), 6.77 (1H, d, J = 2.4 Hz), 6.97 (1H, dd, J = 8.1, 1.5 Hz), 7.20 (1H, t, J = 8.1 Hz), 7.28-7.29 (1H, m), 7.40 (1H, dd, J = 8.5, 2.4 Hz), 7.49 (1H, d, J = 8.5 Hz).

### Example 416

2-(2-{2-[(4R,6S)-1,8-Dichloro-6-(2,3-dimethoxyphenyl)-4H,6H-pyrrolo[1,2-a][4,1]benzoxazepin-4-yl]ethyl}-2H-1,2,3,4-tetrazol-5-yl)acetic acid

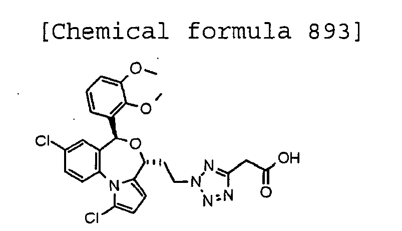

Ethyl 2-(2-{2-[(4R,6S)-1,8-dichloro-6-(2,3-dimethoxyphenyl)-4H,6H-pyrrolo[1,2-a][4,1]benzoxazepin-4-yl]ethyl}-2H-1,2,3,4-tetrazol-5-yl)acetate (327 mg) was dissolved in methanol-water-tetrahydrofuran (1:0.5:1, 7.5mL). To the resulting solution was added lithium hydroxide monohydrate (47.9 mg). The resulting mixture was stirred overnight at room temperature. After the reaction mixture was neutralized with an ion exchange resin "Amberlite IR-120B", the resin was filtered out and the filtrate was concentrated to give the title compound (281 mg).
MS (ESI) m/z : 544 (M+H)⁺.
¹H-NMR (CDCl₃) δ: 2.58-2.69 (2H, m), 3.40 (3H, s), 3.76 (2H, s), 3.83 (3H, s), 4.23-4.27 (1H, m), 4.74-4.83 (2H, m), 5.62 (1H, s), 6.22 (1H, d, J = 3.8 Hz), 6.24 (1H, d, J = 3.8 Hz), 6.73 (1H, d, J = 2.4 Hz), 6.92 (1H, dd, J = 8.1, 1.5 Hz), 7.15 (1H, t, J = 8.1 Hz), 7.22-7.25 (1H, m), 7.35 (1H, dd, J = 8.5, 2.4 Hz), 7.45 (1H, d, J = 8.5 Hz).

### Referential Example 294

[5-Chloro-2-(1H-pyrrol-1-yl)phenyl](1-naphthyl)methanol

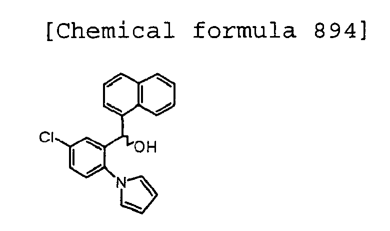

1-(2-Bromo-4-chlorophenyl)-1H-pyrrole (6.00 g) was dissolved in diethyl ether (200 mL). To the resulting solution was added dropwise an n-butyl lithium hexane solution (18.00 mL) at -78°C. After warming to a temperature of ice cooling, the reaction mixture was stirred for one hour. To the reaction mixture was added 1-naphthaldehyde (3.81 mL) under ice cooling, followed by stirring for 30 minutes. To the reaction mixture was added a saturated aqueous solution of ammonium chloride. The resulting mixture was extracted with ethyl acetate. The organic layer was dried over anhydrous sodium sulfate. The solvent was distilled off under reduced pressure. The residue was purified by silica gel column chromatography (hexane:ethyl acetate = 20:1) to give the title compound (5.78 g).
MS (EI) m/z: 333 M⁺.
¹H-NMR (CDCl₃) δ: 6.25-6.28 (2H, m), 6.48 (1H, s), 6.80-6.84 (2H, m), 7.24-7.52 (5H, m), 7.56-7.63 (2H, m), 7.79-7.86 (2H, m).

### Referential Example 295

1-[4-Chloro-2-(1-naphthoyl)phenyl]-1H-pyrrol-2-carbaldehyde

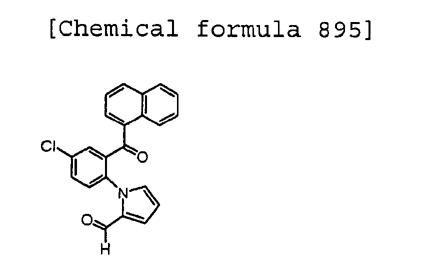

[5-Chloro-2-(1H-pyrrol-1-yl)phenyl](1-naphthyl)methanol (5.78 g) was dissolved in dichloromethane (200 mL). To the resulting solution was added manganese dioxide (15.1 g). The resulting mixture was stirred overnight under heat at 50°C. After the reaction mixture was filtered through a Kiriyama funnel, the filtrate was concentrated under reduced pressure to give [5-chloro-2-(1H-pyrrol-1-yl)phenyl](1-naphthyl)methanone. Phosphorus oxychloride (4.84 mL) was dissolved in dichloromethane (200 mL). Under ice cooling, N,N-dimethylformamide (5.36 mL) was added dropwise to the resulting solution, followed by stirring for 20 minutes. To the reaction mixture was added dropwise the dichloromethane solution (50 mL) of [5-chloro-2-(1H-pyrrol-1-yl)phenyl](1-naphthyl)methanone prepared in advance under ice cooling and the resulting mixture was stirred at room temperature for 3 hours. To the reaction mixture was added an aqueous solution (200 mL) of sodium acetate (21.3 g) and the layers separated. The organic layer was dried over anhydrous sodium sulfate. The solvent was distilled off under reduced pressure. The residue was purified by silica gel column chromatography (hexane:ethyl acetate = 10:1) to give the title compound (2.95 g).
MS (EI) m/z: 359 M⁺.
¹H-NMR (CDCl₃) δ: 5.96 (1H, dd, J = 4.0, 2.6 Hz), 6.60 (1H, dd, J = 4.0, 1.6 Hz), 6.88 (1H, s), 7.25-7.35 (2H, m), 7.47-7.62 (4H, m), 7.69 (1H, d, J = 2.4 Hz), 7.79-7.83 (1H, m), 7.88 (1H, d, J = 8.3 Hz), 8.54 (1H, d, J = 8.3 Hz), 9.29 (1H, s).

### Referential Example 296

Methyl (E)-3-{1-[4-chloro-2-(1-naphthoyl)phenyl]-1H-pyrrol-2-yl}-2-propenoate

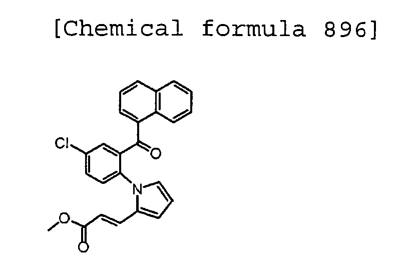

1-[4-Chloro-2-(1-naphthoyl)phenyl]-1H-pyrrol-2-carbaldehyde (2.95 g) was dissolved in toluene (200 mL).
To the resulting solution was added methyl (trimethylphosphoranylidene)acetate (7.13). The resulting mixture was stirred overnight at 70°C. The reaction mixture was concentrated under reduced pressure. The residue was purified by silica gel column chromatography (hexane:ethyl acetate = 10:1) to give the title compound (3.18 g).
MS (FAB) m/z: 415 (M+H)⁺.
¹H-NMR (CDCl₃) δ: 3.75 (3H, s), 5.76-5.84 (2H, m), 6.18-6.21 (1H, m), 6.65-6.68 (1H, m), 7.16 (1H, d, J = 15.6 Hz), 7.20-7.27 (2H, m), 7.30 (1H, d, J = 8.5 Hz), 7.34 (1H, d, J = 7.1 Hz), 7.46-7.56 (2H, m), 7.64 (1H, dd, J = 8.5, 2.4 Hz), 7.75-7.81 (2H, m), 7.84 (1H, d, J = 8.1 Hz), 8.48 (1H, d, J = 8.1 Hz) .

### Example 417

Methyl 2-[trans-8-chloro-6-(1-naphthyl)-4H,6H-pyrrolo[1,2-a][4,1]benzoxazepin-4-yl]acetate

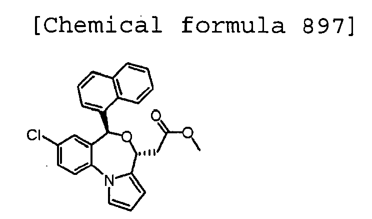

Methyl (E)-3-{1-[4-chloro-2-(1-naphthoyl)phenyl]-1H-pyrrol-2-yl}-2-propenoate (100 mg) was dissolved in methanol (3 mL). To the resulting solution was added sodium borohydride (18 mg). The resulting mixture was stirred at room temperature for one hour. The reaction mixture was concentrated under reduced pressure. To the residue were added water and ethyl acetate and the layers separated. The organic layer was dried over anhydrous sodium sulfate. The solvent was distilled off under reduced pressure. The residue was dissolved in dichloromethane (5 mL). To the resulting solution was added trifluoroacetic acid (28 µl) and the mixture was stirred overnight at room temperature. To the reaction mixture was added a saturated aqueous solution of sodium hydrogen carbonate and the layers separated. The organic layer was dried over anhydrous sodium sulfate and the solvent was distilled off under reduced pressure. The residue was purified by silica gel column chromatography (hexane:ethyl acetate = 9:1) to give the title compound (63 mg).
MS (FAB) m/z: 418 (M+H)⁺.
¹H-NMR (CDCl₃) δ: 3.07 (1H, dd, J = 15.1, 5.6 Hz), 3.16 (1H, dd, J = 15.1, 8.3 Hz), 3.74 (3H, s), 5.04 (1H, dd, J = 8.3, 5.6 Hz), 6.07 (1H, s), 6.30-6.37 (1H, m), 6.40-6.44 (1H, m), 6.59 (1H, d, J = 2.2 Hz), 7.18-7.22 (1H, m), 7.26-7.33 (2H, m), 7.34-7.46 (3H, m), 7.49-7.61 (1H, m), 7.80-7.92 (3H, m).

### Example 418

2-[trans-8-Chloro-6-(1-naphthyl)-4H,6H-pyrrolo[1,2-a] [4,1]benzoxazepin-4-yl]acetic acid

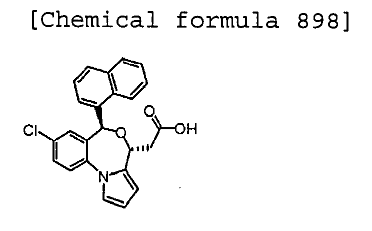

Methyl 2-[trans-8-chloro-6-(1-naphthyl)-4H,6H-pyrrolo[1,2-a][4,1]benzoxazepin-4-yl]acetate (300 mg) was dissolved in tetrahydrofuran (5 mL). To the resulting solution were added methanol (3 mL) and a 1N aqueous sodium hydroxide solution (1.16 mL). The resulting mixture was stirred overnight at room temperature. After concentration of the reaction mixture under reduced pressure, chloroform and a 10% aqueous citric acid solution were added and the layers separated. The organic layer was dried over anhydrous sodium sulfate. The solvent was distilled off under reduced pressure to give the title compound (153 mg). MS (EI) m/z: 403 M⁺.
¹H-NMR (CDCl₃) δ: 3.11-3.18 (1H, m), 3.18-3.25 (1H, m), 5.01-5.07 (1H, m), 6.11 (1H, s), 6.37-6.41 (1H, m), 6.43-6.47 (1H, m), 6.62 (1H, s), 7.20-7.23 (1H, m), 7.25-7.33 (2H, m), 7.37-7.49 (3H, m), 7.56 (1H, t, J = 7.8 Hz), 7.80-7.91 (3H, m).
Elemental analysis for: C₂₉H₂₈ClN₃O₈·0.5H₂O·0.2CHCl₃ Calculated: C, 66.74; H, 4.41; N, 3.19.
Found: C, 66.87; H, 4.24; N, 3.22.

### Example 419

Ethyl 2-(1-{2-[trans-8-chloro-6-(1-naphthyl)-4H,6H-pyrrolo[1,2-a][4,1]benzoxazepin-4-yl]acetyl}-4-piperidinyl)acetate

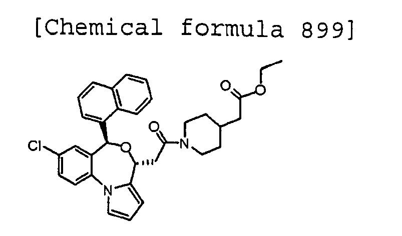

Ethyl 2-(1-{2-[trans-8-chloro-6-(1-naphthyl)-4H,6H-pyrrolo[1,2-a][4,1]benzoxazepin-4-yl]acetyl}-4-piperidinyl)acetate (50 mg) was dissolved in dichloromethane (2 mL). To the resulting solution were added 1-ethyl-3-(3-dimethylaminopropyl)carbodiimide hydrochloride (36 mg), ethyl 2-(4-piperidinyl)acetate (25 mg), and 1-hydroxybenzotriazole monohydrate (19 mg). The resulting mixture was stirred overnight at room temperature. To the reaction mixture was added water and the layers separated. The organic layer was dried over anhydrous sodium sulfate. The solvent was distilled off under reduced pressure. The residue was purified by silica gel column chromatography (hexane:ethyl acetate = 3:1) to give the title compound (47 mg).
MS (FAB) m/z: 557 (M+H)⁺.
¹H-NMR (CDCl₃) δ: 1.09-1.32 (5H, m), 1.59-1.82 (2H, m), 1.87-2.06 (2H, m), 2.14-2.32 (1H, m), 2.77-3.14 (2H, m), 3.27-3.39 (1H, m), 3.98-4.18 (3H, m), 4.62-4.73 (1H, m), 5.05-5.19 (1H, m), 6.02-6.07 (1H, m), 6.28-6.32 (1H, m), 6.40-6.44 (1H, m), 6.54-6.63 (1H, m), 7.18 (1H, s), 7.26-7.46 (5H, m), 7.48-7.59 (1H, m), 7.77-7.91 (3H, m).

### Example 420

2-(1-{2-[trans-8-Chloro-6-(1-naphthyl)-4H,6H-pyrrolo[1,2-a][4,1]benzoxazepin-4-yl]acetyl}-4-piperidinyl)acetic acid

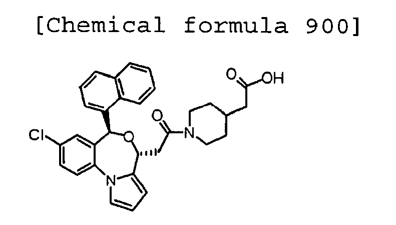

Ethyl 2-(1-{2-[trans-8-chloro-6-(1-naphthyl)-4H,6H-pyrrolo[1,2-a][4,1]benzoxazepin-4-yl]acetyl}-4-piperidinyl)acetate (44 mg) was dissolved in methanol (1 mL). To the resulting solution were added tetrahydrofuran (0.5 mL), water (1 mL), and anhydrous potassium carbonate (33 mg). The resulting mixture was stirred under heat at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (42 mg). MS (EI) m/z: 528 M⁺.
¹H-NMR (CDCl₃) δ: 0.76-1.07 (1H, m), 1.12-1.34 (2H, m), 1.46-2.08 (3H, m), 2.16-2.33 (1H, m), 2.49-2.71 (1H, m), 2.83-3.04 (2H, m), 3.29-3.40 (1H, m), 3.98-4.07 (1H, m), 4.63-4.73 (1H, m), 5.04-5.19 (1H, m), 6.00-6.08 (1H, m), 6.26-6.34 (1H, m), 6.40-6.46 (1H, m), 6.53-6.63 (1H, m), 7.17 (1H, s), 7.27-7.46 (5H, m), 7.47-7.58 (1H, m), 7.69-7.92 (3H, m).
Elemental analysis for: C₃₁H₂₉ClN₂O₄·1.5H₂O·0.3CHCl₃
Calculated: C, 63.74; H, 5.47; N, 4.70.
Found: C, 64.02; H, 5.10; N, 4.61.

### Referential Example 297

[5-Chloro-2-(1H-pyrrol-1-yl)phenyl](2,3-dichlorophenyl)methanol

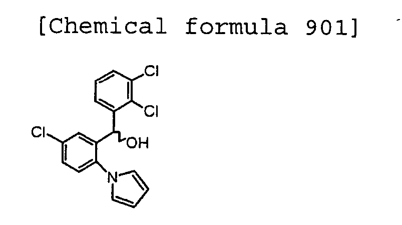

1-(2-Bromo-4-chlorophenyl)-1H-pyrrole (6.00 g) was dissolved in diethyl ether (200 mL). To the resulting solution was added dropwise an n-butyl lithium hexane solution (18.00 mL) at -78°C. The reaction mixture was warmed to the temperature of ice cooling and stirred for one hour. To the reaction mixture was added 2,3-dichlorobenzaldehyde (4.91 g) under ice cooling and the resulting mixture was stirred for 30 minutes. To the reaction mixture was added a saturated aqueous solution of ammonium chloride, followed by extraction with ethyl acetate. The organic layer was dried over anhydrous sodium sulfate. The solvent was distilled off under reduced pressure. The residue was purified by silica gel column chromatography (hexane:ethyl acetate = 20:1) to give the title compound (6.10 g).
MS (EI) m/z: 351 M⁺.
¹H-NMR (CDCl₃) δ: 6.05 (1H, s), 6.29-6.33 (2H, m), 6.78-6.81 (2H, m), 7.17-7.20 (1H, m), 7.25-7.31 (2H, m), 7.33-7.38 (1H, m), 7.43 (1H, d, J = 7.8 Hz), 7.52 (1H, d, J = 7.8 Hz).

### Referential Example 298

1-[4-Chloro-2-(2,3-dichlorobenzoyl)phenyl]-1H-pyrrol-2-carbaldehyde

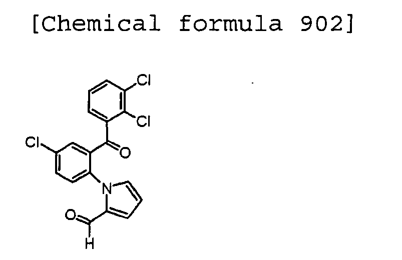

[5-Chloro-2-(1H-pyrrol-1-yl)phenyl](2,3-dichlorophenyl)methanol (6.10 g) was dissolved in dichloromethane (200 mL). To the resulting solution was added manganese dioxide (15.0 g). The resulting mixture was stirred overnight under heat at 50°C. The reaction mixture was filtered through a Kiriyama funnel. The filtrate was concentrated under reduced pressure to give [5-chloro-2-(1H-pyrrol-1-yl)phenyl](2,3-dichlorophenyl)methanone. Phosphorus oxychloride (4.84 mL) was dissolved in dichloromethane (200 mL). To the resulting solution was added dropwise N, N-dimethylformamide (5.36 mL) under ice cooling. The resulting mixture was stirred for 20 minutes. To the reaction mixture, the dichloromethane solution (50 mL) of [5-chloro-2-(1H-pyrrol-1-yl)phenyl](2,3-dichlorophenyl)methanone was added dropwise under ice cooling, followed by stirring at room temperature for 3 hours. To the reaction mixture was added an aqueous solution (200 mL) of sodium acetate (21.3 g) and the layers separated. The organic layer was dried over anhydrous sodium sulfate. The solvent was then distilled off under reduced pressure. The residue was purified by silica gel column chromatography (hexane:ethyl acetate = 9:1) to give the title compound (3.43 g).
MS (EI) m/z: 377 M⁺.
¹H-NMR (CDCl₃) δ: 6.21-6.24 (1H, m), 6.79-6.82 (1H, m), 6.90 (1H, s), 7.06-7.19 (2H, m), 7.29 (1H, d, J = 8.5 Hz), 7.42-7.45 (1H, m), 7.60 (1H, dd, J = 8.5, 2.0 Hz), 7.71 (1H, d, J = 2.0 Hz), 9.35 (1H, s).

### Referential Example 299

Methyl (E)-3-{1-[4-chloro-2-(2,3-dichlorobenzoyl)phenyl]-1H-pyrrol-2-yl}-2-propenoate

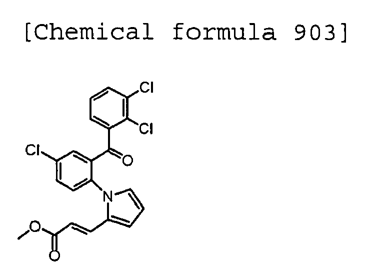

1-[4-Chloro-2-(2,3-dichlorobenzoyl)phenyl]-1H-pyrrol-2-carbaldehyde (3.42 g) was dissolved in toluene (200 mL). To the resulting solution was added methyl (trimethylphosphoranylidene)acetate (7.85 g). At 70°C, the resulting mixture was stirred overnight. The reaction mixture was concentrated under reduced pressure. The residue was purified by silica gel column chromatography (hexane:ethyl acetate = 10:1) to give the title compound (4.05 g).
MS (EI) m/z: 433 M⁺.
¹H-NMR (CDCl₃) δ: 3.73 (3H, s), 5.88 (1H, d, J = 15.9 Hz), 6.03-6.07 (1H, m), 6.37-6.42 (1H, m), 6.66-6.70 (1H, m), 6.99-7.05 (2H, m), 7.12 (1H, d, J = 15.9 Hz), 7.23-7.28 (2H, m), 7.35-7.41 (1H, m), 7.65 (1H, dd, J = 8.3, 2.4 Hz), 7.81 (1H, d, J = 2.4 Hz).

### Example 421

Methyl 2-[trans-8-chloro-6-(2,3-dichlorobenzoyl)-4H,6H-pyrrolo[1,2-a][4,1]benzoxazepin-4-yl]acetate

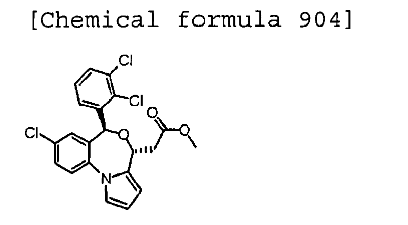

Methyl (E)-3-{1-[4-chloro-2-(2,3-dichlorobenzoyl)phenyl]-1H-pyrrol-2-yl}-2-propenoate (100 mg) was dissolved in methanol (3 mL). To the resulting solution was added sodium borohydride (17 mg). The resulting mixture was stirred at room temperature for one hour. The reaction mixture was concentrated under reduced pressure. To the residue were added water and ethyl acetate and the layers separated. The organic layer was dried over anhydrous sodium sulfate and then, the solvent was distilled off under reduced pressure. The residue was dissolved in dichloromethane (5 mL). To the resulting solution was added trifluoroacetic acid (27 µl). The resulting mixture was stirred overnight at room temperature. To the reaction mixture was added a saturated aqueous solution of sodium hydrogen carbonate and the layers separated. The organic layer was dried over anhydrous sodium sulfate. The solvent was distilled off under reduced pressure. The residue was purified by silica gel column chromatography (hexane:ethyl acetate = 10:1) to give the title compound (73 mg).
MS (FAB) m/z: 436 (M+H)⁺.
¹H-NMR (CDCl₃) δ: 3.03 (1H, dd, J = 15.1, 5.7 Hz), 3.11 (1H, dd, J = 15.1, 8.3 Hz), 3.73 (3H, s), 4.91 (1H, dd, J = 8.3, 5.7 Hz), 5.68 (1H, s), 6.29-6.32 (1H, m), 6.39 (1H, t, J = 3.3 Hz), 6.56 (1H, d, J = 2.2 Hz), 7.11-7.14 (1H, m), 7.33-7.45 (3H, m), 7.50 (1H, dd, J = 7.8, 1.5 Hz), 7.71 (1H, d, J = 7.8 Hz).

### Example 422

2-[trans-8-Chloro-6-(2,3-dichlorobenzoyl)-4H,6H-pyrrolo[1,2-a][4,1]benzoxazepin-4-yl]acetic acid

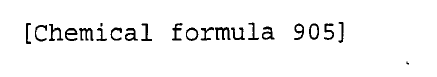

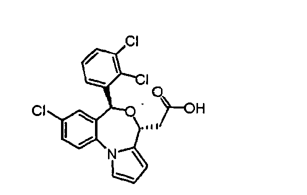

Methyl 2-[trans-8-chloro-6-(2,3-dichlorobenzoyl)-4H,6H-pyrrolo[1,2-a][4,1]benzoxazepin-4-yl]acetate (200 mg) was dissolved in tetrahydrofuran (4 mL). To the resulting solution were added methanol (2 mL) and a 1N aqueous sodium hydroxide solution (733 µl). The resulting mixture was stirred overnight at room temperature. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (61 mg).
MS (EI) m/z: 421 M⁺.
¹H-NMR (CDCl₃) δ: 3.04-3.19 (2H, m), 4.88-4.95 (1H, m), 5.70 (1H, s), 6.32-6.37 (1H, m), 6.38-6.42 (1H, m), 6.57 (1H, d, J = 2.2 Hz), 7.13-7.15 (1H, m), 7.31-7.45 (3H, m), 7.50 (1H, d, J = 8.1 Hz), 7.73 (1H, d, J = 8.1 Hz). Elemental analysis for: C₂₀H₁₄Cl₃NO₃
Calculated: C, 56.83; H, 3.34; N, 3.31.
Found: C, 56.44; H, 3.54; N, 3.14.

### Example 423

Ethyl 2-(1-{2-[trans-8-chloro-6-(2,3-dichlorobenzoyl)-4H,6H-pyrrolo[1,2-a][4,1]benzoxazepin-4-yl]acetyl}-4-piperidinyl)acetate

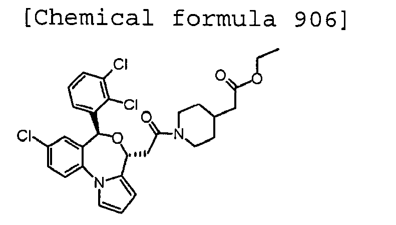

2-[trans-8-Chloro-6-(2,3-dichlorobenzoyl)-4H,6H-pyrrolo[1,2-a][4,1]benzoxazepin-4-yl]acetic acid (47 mg) was dissolved in dichloromethane (2 mL). To the resulting solution were added hydrochloric acid 1-ethyl-3-(3-dimethylaminopropyl)carbodiimide (32 mg), ethyl 2-(4-piperidinyl)acetate (23 mg), and 1-hydroxybenzotriazole monohydrate (17 mg). The resulting mixture was stirred overnight at room temperature. Water was added to the reaction mixture and the layers separated. The organic layer was dried over anhydrous sodium sulfate. The solvent was distilled off under reduced pressure. The residue was purified by silica gel column chromatography (hexane:ethyl acetate = 3:1) to give the title compound (47 mg).
MS (EI) m/z: 574 M⁺.
¹H-NMR (CDCl₃) δ: 1.06-1.29 (5H, m), 1.67-1.85 (2H, m), 1.96-2.09 (1H, m), 2.15-2.20 (1H, m), 2.22-2.28 (1H, m), 2.83-2.93 (1H, m), 3.21-3.30 (1H, m), 4.04 (1H, d, J = 11.5 Hz), 4.14 (2H, dd, J = 7.2, 2.8 Hz), 4.67 (1H, s), 5.69 (1H, d, J = 9.8 Hz), 6.26 (1H, s), 6.38 (1H, t, J = 3.3 Hz), 6.53 (1H, s), 7.11 (1H, s), 7.32-7.47 (3H, m), 7.48 (1H, s), 7.68-7.78 (1H, m).

### Example 424

2-(1-{2-[trans-8-Chloro-6-(2,3-dichlorobenzoyl)-4H,6H-pyrrolo[1,2-a][4,1]benzoxazepin-4-yl]acetyl}-4-piperidinyl)acetic acid

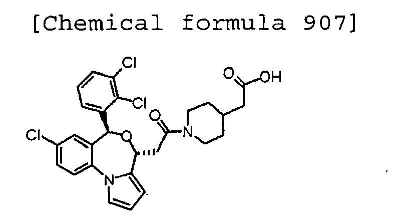

Ethyl 2-(1-{2-[trans-8-chloro-6-(2,3-dichlorobenzoyl)-4H,6H-pyrrolo[1,2-a][4,1]benzoxazepin-4-yl]acetyl}-4-piperidinyl)acetate (44 mg) was dissolved in methanol (1 mL). To the resulting solution were added tetrahydrofuran (0.5 mL), water (1 mL) and anhydrous potassium carbonate (32 mg). The resulting mixture was stirred at 65°C for 2 hours. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (42 mg). MS (EI) m/z: 546 M⁺.
¹H-NMR (CDCl₃) δ: 1.07-1.34 (2H, m), 1.69-1.89 (2H, m), 1.95-2.10 (1H, m), 2.20-2.24 (1H, m), 2.28-2.33 (1H, m), 2.56-2.72 (1H, m), 2.82-2.93 (1H, m), 2.96-3.16 (1H, m), 3.22-3.31 (1H, m), 4.00-4.08 (1H, m), 4.64-4.75 (1H, m), 4.94-5.04 (1H, m), 5.69 (1H, d, J = 9.5 Hz), 6.27 (1H, s), 6.37-6.40 (1H, m), 6.51-6.54 (1H, m), 7.12 (1H, s), 7.31-7.46 (3H, m), 7.48-7.53 (1H, m), 7.67-7.77 (1H, m). Elemental analysis for: C₂₇H₂₅ClN₂O₄·1.25H₂O
Calculated: C, 56.86; H, 4.86; N, 4.91.
Found: C, 56.84; H, 4.56; N, 4.61.

### Example 425

Ethyl 2-[2-({2-[(4R,6S)-8-chloro-6-(2,3-dimethoxyphenyl)-4H,6H-pyrrolo[1,2-a][4,1]benzoxazepin-4-yl]ethyl}amino)-1,3-thiazol-4-yl]acetate

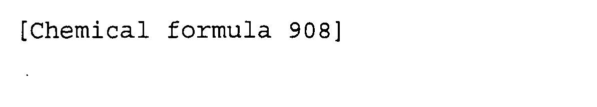

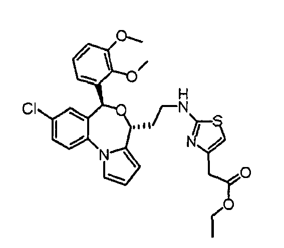

2-[(4R,6S)-8-Chloro-6-(2,3-dimethoxyphenyl)-4H,6H-pyrrolo[1,2-a][4,1]benzoxazepin-4-yl]-1-ethanol (100 mg) was dissolved in dichloromethane (3 mL). To the resulting solution was added a Dess-Martin Reagent (212 mg) under ice cooling. The resulting mixture was stirred for 3 hours under ice cooling. To the resulting mixture was added a saturated aqueous solution of sodium hydrogen carbonate and the layers separated. The organic layer was dried over anhydrous sodium sulfate. The solvent was distilled off under reduced pressure. The residue was dissolved in dichloromethane (3 mL). To the resulting solution were added ethyl (2-amino-4-thiazolyl)acetate (47 mg), acetic acid (14 µl), and sodium triacetoxyborohydride (106 mg). The resulting mixture was stirred overnight at room temperature under a nitrogen gas stream. To the reaction mixture was added a saturated aqueous solution of sodium hydrogen carbonate and the layers separated. The organic layer was dried over anhydrous sodium sulfate. The solvent was distilled off under reduced pressure. The residue was purified by silica gel column chromatography (hexane:ethyl acetate = 8:1) to give the title compound (51 mg).
MS (FAB) m/z: 568 (M+H)⁺.
¹H-NMR (CDCl₃) δ: 1.26 (3H, t, J = 7.1 Hz), 2.26-2.55 (3H, m), 3.44 (3H, s), 3.50-3.65 (3H, m), 3.86 (3H, s), 4.17 (2H, q, J = 7.1 Hz), 4.50-4.56 (1H, m), 5.74 (1H, s), 6.30-6.34 (1H, m), 6.36-6.40 (1H, m), 6.71-6.75 (1H, m), 6.94-6.98 (1H, m), 7.08-7.11 (1H, m), 7.16-7.39 (3H, m).

### Example 426

2-[2-({2-[(4R,6S)-8-Chloro-6-(2,3-dimethoxyphenyl)-4H,6H-pyrrolo[1,2-a][4,1]benzoxazepin-4-yl]ethyl}amino)-1,3-thiazol-4-yl]acetic acid

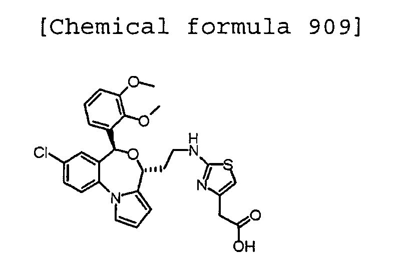

Ethyl 2-[2-({2-[(4R,6S)-8-chloro-6-(2,3-dimethoxyphenyl)-4H,6H-pyrrolo[1,2-a][4,1]benzoxazepin-4-yl]ethyl}amino)-1,3-thiazol-4-yl]acetate (47 mg) was dissolved in methanol (8 mL). To the resulting solution were added tetrahydrofuran (1 mL), water (2 mL), and anhydrous potassium carbonate (34 mg). The resulting mixture was stirred under heat at 65°C for 3 hours. After concentration of the reaction mixture under reduced pressure, chloroform and a 10% aqueous citric acid solution were added to the residue and the layers separated. The organic layer was dried over anhydrous sodium sulfate. The solvent was distilled off under reduced pressure to give the title compound (38 mg).
MS (FAB) m/z: 540 (M+H)⁺.
¹H-NMR (CDCl₃) δ: 2.31-2.52 (2H, m), 3.45 (3H, s), 3.57 (3H, s), 3.83-3.88 (4H, m), 4.48-4.59 (1H, m), 5.71-5.77 (1H, m), 5.92-6.21 (1H, m), 6.29-6.41 (2H, m), 6.69-6.76 (1H, m), 6.92-7.00 (1H, m), 7.10 (1H, s), 7.19-7.38 (3H, m) .

### Referential Example 300

(4R,6S)-4-(3-Butynyl)-8-chloro-6-(2,3-dimethoxyphenyl)-4H,6H-pyrrolo[1,2-a][4,1]benzoxazepine

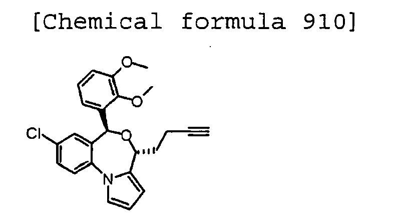

2-[(4R,6S)-8-Chloro-6-(2,3-dimethoxyphenyl)-4H,6H-pyrrolo[1,2-a][4,1]benzoxazepin-4-yl]ethyl methanesulfonate (798 mg) was dissolved in dimethylsulfoxide (8 mL). To the resulting solution were added tetra-n-butylammonium iodide (617 mg) and lithium acetylide ethylenediamine complex (461 mg). The resulting mixture was stirred overnight at room temperature. To the reaction mixture was added water and the resulting mixture was stirred for 5 minutes and then, the reaction mixture was extracted with diethyl ether. After the organic layer was dried over anhydrous magnesium sulfate, the solvent was distilled off under reduced pressure. The residue was purified by silica gel column chromatography (hexane:ethyl acetate = 20:1) to give the title compound (248 mg).
MS (FAB) m/z: 408 (M+H)⁺.
¹H-NMR (CDCl₃) δ: 2.16-2.39 (2H, m), 2.42-2.58 (2H, m), 2.84-2.92 (1H, m), 3.44 (3H, s), 3.85 (3H, s), 4.45-4.56 (1H, m), 5.71 (1H, s), 6.31 (1H, s), 6.35-6.43 (1H, m), 6.71-6.76 (1H, m), 6.92-7.01 (1H, m), 7.10 (1H, s), 7.15-7.23 (1H, m), 7.26-7.28 (1H, m), 7.31-7.39 (2H, m).

### Example 427

Ethyl 2-(4-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate (position isomer A)

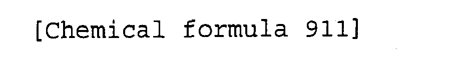

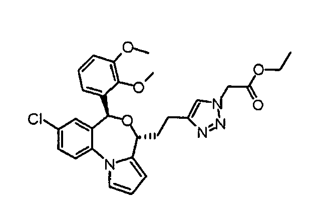

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate (position isomer B)

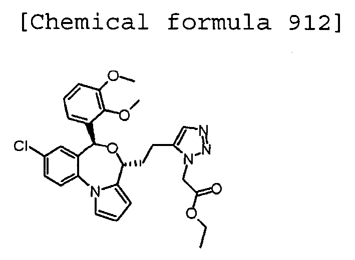

(4R,6S)-4-(3-butynyl)-8-chloro-6-(2,3-dimethoxyphenyl)-4H,6H-pyrrolo[1,2-a][4,1]benzoxazepine (167 mg) was dissolved in toluene (6 mL). To the resulting solution was added ethyl azidoacetate (495 mg). The resulting mixture was heated under reflux overnight. The reaction mixture was concentrated under reduced pressure. The residue was purified by silica gel column chromatography (hexane:ethyl acetate = 3:1) to give a mixture of ethyl 2-(4-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate and ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate. From the resulting mixture, position isomers were separated by high performance liquid chromatography (developing solvent: 30% isopropyl alcohol, n-hexane solution) using CHIRALPAK AD (Daicel Chemical), whereby the isomer A (49 mg) eluted with a retention time of 28 minutes and isomer B (54 mg) eluted with a retention time of 28 minutes were obtained, respectively. Ethyl 2-(4-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate (position isomer A):
MS (FAB) m/z: 537 (M+H)⁺.
¹H-NMR (CDCl₃) δ: 1.25 (3H, t, J = 7.1 Hz), 2.27-2.39 (1H, m), 2.41-2.52 (1H, m), 2.79-2.89 (1H, m), 2.93-3.04 (1H, m), 3.43 (3H, s), 3.86 (3H, s), 4.21 (2H, q, J = 7.1 Hz), 4.37-4.42 (1H, m), 5.07 (2H, s), 5.73 (1H, s), 6.29-6.32 (1H, m), 6.39 (1H, t, J = 3.2 Hz), 6.73 (1H, d, J = 2.0 Hz), 6.96 (1H, d, J = 7.9 Hz), 7.10-7.12 (1H, m), 7.19 (1H, t, J = 7.9 Hz), 7.33-7.41 (3H, m), 7.54 (1H, s).
Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate (position isomer B):
MS (FAB) m/z: 537 (M+H)⁺.
¹H-NMR (CDCl₃) δ: 1.28 (3H, t, J = 7.1 Hz), 2.36-2.54 (2H, m), 2.93-3.03 (1H, m), 3.10-3.19 (1H, m), 3.44 (3H, s), 3.85 (3H, s), 4.25 (2H, q, J = 7.1 Hz), 4.41 (1H, dd, J = 8.7, 4.6 Hz), 5.09 (2H, s), 5.73 (1H, s), 6.32-6.35 (1H, m), 6.37 (1H, t, J = 3.1 Hz), 6.72 (1H, s), 6.95 (1H, d, J = 7.8 Hz), 7.09 (1H, s), 7.19 (1H, t, J = 7.8 Hz), 7.31-7.38 (2H, m), 7.45 (1H, s).

### Example 428

2-(4-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-1-yl)acetic acid (position isomer A)

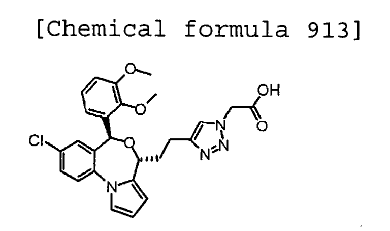

Ethyl 2-(4-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate (position isomer A, 45 mg) was dissolved in methanol (10 mL). To the resulting solution were added water (3 mL) and anhydrous potassium carbonate (35 mg). The resulting mixture was stirred under heat at 65°C for 2 hours. After concentration of the reaction mixture under reduced pressure, chloroform and a 10% aqueous citric acid solution were added to the residue and the layers separated. After the organic layer was dried over anhydrous sodium sulfate, the solvent was distilled off under reduced pressure to give the title compound (37 mg).
MS (FAB) m/z: 509 (M+H)⁺.
¹H-NMR (CDCl₃) δ: 2.27-2.39 (1H, m), 2.39-2.50 (1H, m), 2.78-2.90 (1H, m), 2.94-3.06 (1H, m), 3.42 (3H, s), 3.84 (3H, s), 4.39 (1H, br s), 5.09 (2H, br s), 5.72 (1H, s), 6.28 (1H, s), 6.37 (1H, s), 6.73 (1H, s), 6.91-6.97 (1H, m), 7.09 (1H, s), 7.13-7.20 (1H, m), 7.22-7.25 (1H, m), 7.34 (2H, s), 7.56 (1H, s).
Elemental analysis for: C₂₆H₂₅ClN₄O₅·0.75H₂O·0.3CHCl₃
Calculated: C, 56.86; H, 4.81; N, 9.97.
Found: C, 57.14; H, 4.81; N, 9.69.

### Example 429

2-(5-{2-[(4R,6S)-8-Chloro-6-(2,3-dimethoxyphenyl)-4H,6H-pyrrolo[1,2-a][4,1]benzoxazepin-4-yl]ethyl}-1H-1,2,3-triazol-1-yl)acetic acid (position isomer B)

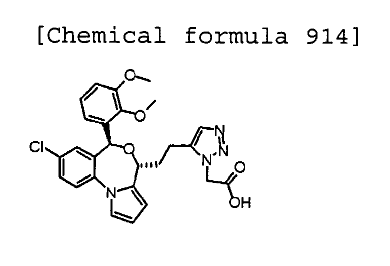

Ethyl 2-(5-{2-[(4R,6S)-8-chloro-6-(2,3-dimethoxyphenyl)-4H,6H-pyrrolo[1,2-a][4,1]benzoxazepin-4-yl]ethyl}-1H-1,2,3-triazol-1-yl)acetate (position isomer B, 48 mg) was dissolved in methanol (10 mL). To the resulting solution were added water (3 mL) and anhydrous potassium carbonate (37 mg). The resulting mixture was stirred under heat at 65°C for 2 hours. After concentration of the reaction mixture under reduced pressure, chloroform and a 10% aqueous citric acid solution were added to the residue and the layers separated. After the organic layer was dried over anhydrous sodium sulfate, the solvent was distilled off under reduced pressure to give the title compound (45 mg).
MS (FAB) m/z: 509 (M+H)⁺.
¹H-NMR (CDCl₃) δ: 2.31-2.49 (2H, m), 2.87-2.99 (1H, m), 3.04-3.17 (1H, m), 3.42 (3H, s), 3.83 (3H, s), 4.39 (1H, br s), 5.05 (2H, s), 5.72 (1H, s), 6.27-6.39 (2H, m), 6.71 (1H, s), 6.93 (1H, s), 7.07 (1H, s), 7.12-7.20 (1H, m), 7.23-7.37 (3H, m), 7.45 (1H, s).
Elemental analysis for: C₂₆H₂₅ClN₄O₅·0.75H₂O·0.4CHCl₃
Calculated: C, 55.98; H, 4.72; N, 9.74.
Found: C, 56.36; H, 4.81; N, 9.43.

### Example 430

1-{2-[(4R,6S)-8-Chloro-6-(2,3-dimethoxyphenyl)-4H,6H-pyrrolo[1,2-a][4,1]benzoxazepin-4-yl]acetyl}-4-piperidine carboxylic acid

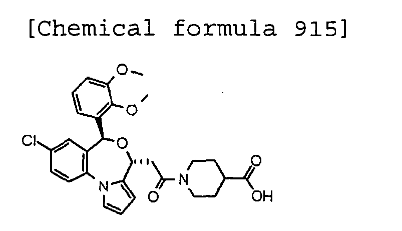

Ethyl 1-{2-[(4R,6S)-8-chloro-6-(2,3-dimethoxyphenyl)-4H,6H-pyrrolo[1,2-a][4,1]benzoxazepin-4-yl]acetyl}-4-piperidine carboxylate (66.1 mg) was dissolved in methanol-water-tetrahydrofuran (1:2:1, 4 mL). To the resulting solution was added potassium carbonate (66.3 mg). The resulting mixture was stirred at 55°C for 3 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained was added ethyl acetate and hexane. The solid thus formed was collected by filtration to give the title compound (58.7 mg).
MS (ESI) m/z : 525 (M⁺+H) .
¹H-NMR (CDCl₃) δ: 1.6-1.8 (2H, m), 1.8-2.0 (2H, m), 2.5-2.6 (1H, m), 2.9 (1H, dd, J = 14.2, 4.6 Hz), 2.9-3.3 (2H, m), 3.4 (3H, s), 3.9 (3H, s), 3.9-4.1 (1H, m), 4.3-4.6 (1H, m), 4.9-5.0 (1H, m), 5.7 (1H, s), 6.2-6.3 (1H, m), 6.3-6.4 (1H, m), 6.7 (1H, d, J = 8.5 Hz), 6.9 (1H, dd, J = 13.7, 7.6 Hz), 7.1 (1H, dd, J = 2.7, 1.5 Hz), 7.1 (1H, td, J = 7.9, 3.3 Hz), 7.2-7.3 (2H, m), 7.3-7.4 (2H, m).
Elemental analysis for: C₂₈H₂₉ClN₂O₆
Calculated: C, 64.06; H, 5.57; N, 5.34.
Found: C, 63.78; H, 5.69; N, 5.21.

### Example 431

1-{2-[(4S,6R)-8-Chloro-6-(2,3-dimethoxyphenyl)-4H,6H-pyrrolo[1,2-a][4,1]benzoxazepin-4-yl]acetyl}-4-piperidine carboxylic acid

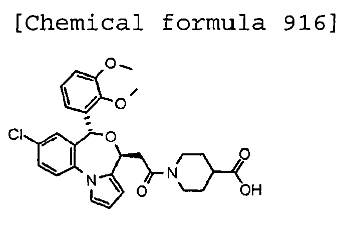

Ethyl 1-{2-[(4S,6R)-8-chloro-6-(2,3-dimethoxyphenyl)-4H,6H-pyrrolo[1,2-a] [4,1]benzoxazepin-4-yl]acetyl}-4-piperidine carboxylate (66.4 mg) was dissolved in methanol-water-tetrahydrofuran (1:2:1, 4 mL). To the resulting solution was added potassium carbonate (66.3 mg). The resulting mixture was stirred at 55°C for 3 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (53.3 mg).
MS (ESI) m/z : 525 (M⁺+H) .
Elemental analysis for: C₂₈H₂₉ClN₂O₆
Calculated: C, 64.06; H, 5.57; N, 5.34.
Found: C, 63.71; H, 5.67; N, 5.16.

### Example 432

3-({2-[(4R,6S)-8-Chloro-6-(2,3-dimethoxyphenyl)-4H,6H-pyrrolo[1,2-a][4,1]benzoxazepin-4-yl]acetyl}amino)benzoic acid

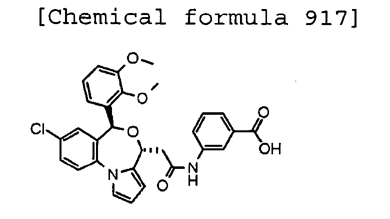

Methyl 3-({2-[(4R,6S)-8-chloro-6-(2,3-dimethoxyphenyl)-4H,6H-pyrrolo[1,2-a][4,1]benzoxazepin-4-yl]acetyl}amino)benzoate (109 mg) was dissolved in methanol-water-tetrahydrofuran (1:2:1, 4 mL). To the resulting solution was added potassium carbonate (111 mg). The resulting mixture was stirred at 55°C for 7 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (71.8 mg).
MS (ESI) m/z : 533 (M⁺+H).
¹H-NMR (CDCl₃) δ: 3.1 (1H, m), 3.5 (3H, s), 3.9 (3H, s), 4.9-5.0 (1H, m), 5.9 (1H, s), 6.4 (2H, m), 6.6-7.4 (6H, m), 7.6-8.1 (6H, m), 8.6 (1H, br s).
Elemental analysis for: C₂₉H₂₅ClN₂O₆
Calculated: C, 65.35; H, 4.73; N, 5.26.
Found: C, 65.11; H, 4.93; N, 5.06.

### Example 433

3-({2-[(4S,6R)-8-Chloro-6-(2,3-dimethoxyphenyl)-4H,6H-pyrrolo[1,2-a][4,1]benzoxazepin-4-yl]acetyl}amino)benzoic acid

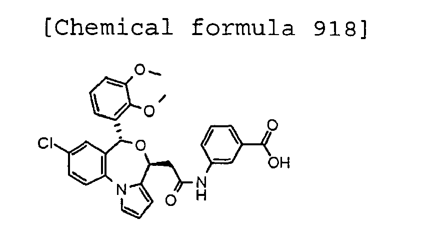

Methyl 3-({2-[(4S,6R)-8-chloro-6-(2,3-dimethoxyphenyl)-4H,6H-pyrrolo[1,2-a][4,1]benzoxazepin-4-yl]acetyl}amino)benzoate (129 mg) was dissolved in methanol-water-tetrahydrofuran (1:2:1, 4 mL). To the resulting solution was added potassium carbonate (130 mg). The resulting mixture was stirred at 55°C for 7 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (79.9 mg).
MS (ESI) m/z : 533 (M⁺+H).
Elemental analysis for: C₂₉H₂₅ClN₂O₆
Calculated: C, 65.35; H, 4.73; N, 5.26.
Found: C, 65.16; H, 4.99; N, 5.06.

### Example 434

2-(4-{2-[(4R,6S)-8-Chloro-6-(2,3-dimethoxyphenyl)-4H,6H-pyrrolo[1,2-a][4,1]benzoxazepin-4-yl]acetyl}-1-piperazinyl)acetic acid

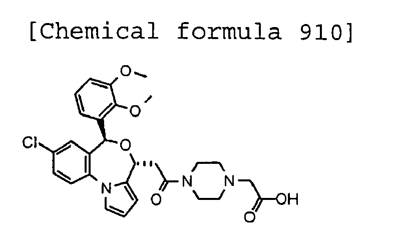

Ethyl 2-(4-{2-[(4R,6S)-8-chloro-6-(2,3-dimethoxyphenyl)-4H,6H-pyrrolo[1,2-a][4,1]benzoxazepin-4-yl]acetyl}-1-piperazinyl)acetate (79 mg) was dissolved in methanol-water-tetrahydrofuran (1:2:1, 4 mL). To the resulting solution was added potassium carbonate (77 mg). The resulting mixture was stirred at 55°C for 18 hours. Under ice cooling, 1N hydrochloric acid was added to the reaction mixture to neutralize it, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (49 mg).
MS (ESI) m/z : 541 (M⁺+H) .
¹H-NMR (CDCl₃) δ: 2.6-2.9 (2H, m), 3.0-3.4 (3H, m), 3.4 (3H, s), 3.6-3.8 (2H, m), 3.8 (3H, s), 4.1-4.4 (3H, m), 4.9-5.0 (1H, m), 5.8 (1H, s), 6.3 (1H, br s), 6.4-6.4 (1H, m), 6.7-6.8 (2H, m), 6.9-7.0 (1H, m), 7.1-7.1 (1H, m), 7.1-7.2 (2H, m), 7.3-7.4 (3H, m).
Elemental analysis for: C₂₈H₃₀ClN₃O₆·1.0H₂O
Calculated: C, 60.27; H, 5.78; N, 7.53.
Found: C, 59.90; H, 5.76; N, 7.12.

### Example 435

2-(4-{2-[(4S,6R)-8-Chloro-6-(2,3-dimethoxyphenyl)-4H,6H-pyrrolo[1,2-a][4,1]benzoxazepin-4-yl]acetyl}-1-piperazinyl)acetic acid

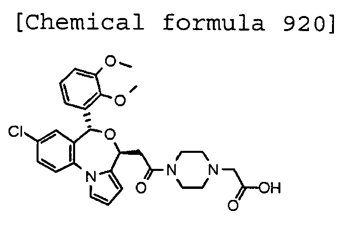

Ethyl 2-(4-{2-[(4S,6R)-8-chloro-6-(2,3-dimethoxyphenyl)-4H,6H-pyrrolo[1,2-a][4,1]benzoxazepin-4-yl]acetyl}-1-piperazinyl)acetate (113 mg) was dissolved in methanol-water-tetrahydrofuran (1:2:1, 4 mL). To the resulting solution was added potassium carbonate (110 mg). The resulting mixture was stirred at 55°C for 18 hours. Under ice cooling, 1N hydrochloric acid was added to the reaction mixture to neutralize it, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (70.5 mg).
MS (ESI) m/z : 541 (M⁺+H).
Elemental analysis for: C₂₈H₃₀ClN₃O₆·1.0HCl·2.0H₂O
Calculated: C, 54.91; H, 5.76; N, 6.86.
Found: C, 54.85; H, 5.24; N, 6.58.

### Example 436

(3S)-1-{2-[(4R,6S)-8-Chloro-6-(2,3-dimethoxyphenyl)-4H,6H-pyrrolo[1,2-a][4,1]benzoxazepin-4-yl]acetyl}-3-piperidine carboxylic acid

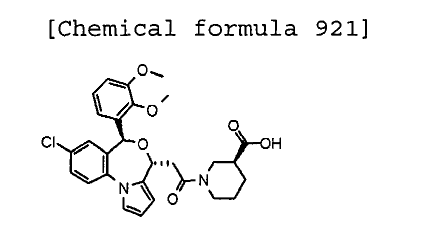

Ethyl (3S)-1-{2-[(4R,6S)-8-chloro-6-(2,3-dimethoxyphenyl)-4H,6H-pyrrolo[1,2-a][4,1]benzoxazepin-4-yl]acetyl}-3-piperidine carboxylate (127 mg) was dissolved in methanol-water-tetrahydrofuran (1:2:1, 4 mL). To the resulting solution was added potassium carbonate (127 mg). The resulting mixture was stirred at 55°C for 4 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (78.7 mg).
MS (ESI) m/z : 525 (M⁺+H).
¹H-NMR (CDCl₃) δ: 1.4-1.6 (1H, m), 1.6-1.8 (2H, m), 1.9-2.1 (1H, m), 2.5-2.6 (1H, m), 2.8-3.1 (2H, m), 3.1-3.4 (2H, m), 3.4 (3H, s), 3.8 (3H, s), 4.0-4.3 (1H, m), 4.6-4.6 (1H, m), 4.9-5.0 (1H, m), 5.8 (1H, s), 6.2 (1H, br s), 6.4 (1H, t, J = 3.2 Hz), 6.7-6.7 (1H, m), 6.9-7.0 (1H, m), 7.1 (1H, br s), 7.1-7.2 (1H, m), 7.2-7.4 (3H, m).
Elemental analysis for: C₂₈H₂₉ClN₂O₆·0.25H₂O
Calculated: C, 63.51; H, 5.62; N, 5.29.
Found: C, 63.62; H, 5.43; N, 5.30.

### Example 437

(3S)-1-{2-[(4S,6R)-8-Chloro-6-(2,3-dimethoxyphenyl)-4H,6H-pyrrolo[1,2-a][4, 1]benzoxazepin-4-yl]acetyl}-3-piperidine carboxylic acid

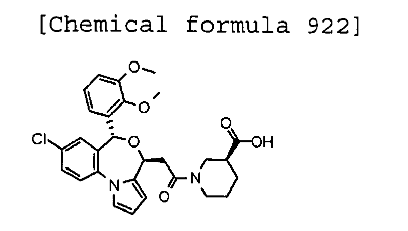

Ethyl (3S)-1-{2-[(4S,6R)-8-chloro-6-(2,3-dimethoxyphenyl)-4H,6H-pyrrolo[1,2-a][4,1]benzoxazepin-4-yl]acetyl}-3-piperidine carboxylate (126 mg) was dissolved in methanol-water-tetrahydrofuran (1:2:1, 4 mL). To the resulting solution was added potassium carbonate (126 mg). The resulting mixture was stirred at 55°C for 4 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (78.7 mg).
MS (ESI) m/z : 525 (M⁺+H) .
¹H-NMR (CDCl₃) δ: 1.4-1.6 (1H, m), 1.6-1.8 (2H, m), 1.9-2.1 (1H, m), 2.5-2.6 (1H, m), 2.8-3.1 (2H, m), 3.1-3.4 (2H, m), 3.4 (3H, s), 3.8 (3H, s), 4.0-4.3 (1H, m), 4.6-4.6 (1H, m), 4.9-5.0 (1H, m), 5.8 (1H, s), 6.2 (1H, br s), 6.4 (1H, t, J = 3.2 Hz), 6.7-6.7 (1H, m), 6.9-7.0 (1H, m), 7.1 (1H, br s), 7.1-7.2 (1H, m), 7.2-7.4 (3H, m).
Elemental analysis for: C₂₈H₂₉ClN₂O₆·0.25H₂O
Calculated: C, 63.51; H, 5.62; N, 5.29.
Found: C, 63.53; H, 5.44; N, 5.28.

### Example 438

2-({2-[(4R,6S)-8-Chloro-6-(2,3-dimethoxyphenyl)-4H,6H-pyrrolo[1,2-a][4,1]benzoxazepin-4-yl]acetyl}amino)acetic acid

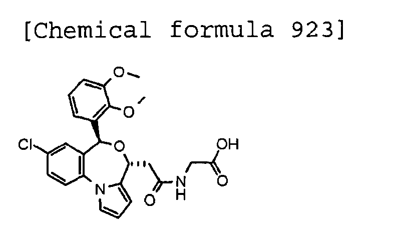

Methyl 2-({2-[(4R,6S)-8-chloro-6-(2,3-dimethoxyphenyl)-4H,6H-pyrrolo[1,2-a][4,1]benzoxazepin-4-yl]acetyl}amino)acetate (63 mg) was dissolved in methanol-water-tetrahydrofuran (1:2:1, 4 mL). To the resulting solution was added potassium carbonate (72 mg). The resulting mixture was stirred at 55°C for 4 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (51.7 mg).
MS (ESI) m/z : 471 (M⁺+H) .
¹H-NMR (CDCl₃) δ: 3.0-3.0 (2H, m), 3.5 (3H, s), 3.9 (3H, s), 4.1-4.1 (2H, m), 4.8-4.9 (1H, m), 5.8 (1H, s), 6.3-6.3 (1H, m), 6.4-6.4 (1H, m), 6.8-6.8 (1H, m), 7.0 (1H, dd, J = 8.1, 1.5 Hz), 7.1-7.1 (1H, m), 7.2 (1H, t, J = 7.9 Hz), 7.2 (2H, br s), 7.3-7.4 (2H, m).
Elemental analysis for: C₂₄H₂₃ClN₂O₆
Calculated: C, 61.21; H, 4.92; N, 5.95.
Found: C, 61.15; H, 4.86; N, 5.71.

### Example 439

2-({2-[(4S,6R)-8-Chloro-6-(2,3-dimethoxyphenyl)-4H,6H-pyrrolo [1, 2-a] [4,1]benzoxazepin-4-yl]acetyl}amino)acetic acid

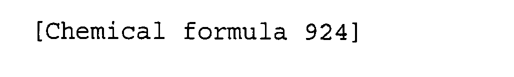

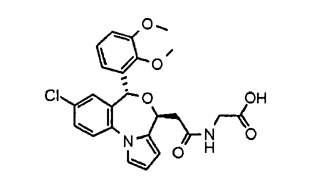

Methyl 2-({2-[(4S,6R)-8-chloro-6-(2,3-dimethoxyphenyl)-4H,6H-pyrrolo[1,2-a][4,1]benzoxazepin-4-yl]acetyl}amino)acetate (103 mg) was dissolved in methanol-water-tetrahydrofuran (1:2:1, 4mL). To the resulting solution was added potassium carbonate (117 mg). The resulting mixture was stirred at 55°C for 4 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (63.9 mg).
MS (ESI) m/z : 471 (M⁺+H)
Elemental analysis for: C₂₄H₂₃ClN₂O₆
Calculated: C, 61.21; H, 4.92; N, 5.95.
Found: C, 61.21; H, 5.09; N, 5.63.

### Example 440

3-({2-[(4R,6S)-8-Chloro-6-(2,3-dimethoxyphenyl)-4H,6H-pyrrolo[1,2-a][4,1]benzoxazepin-4-yl]acetyl}amino)propionic acid

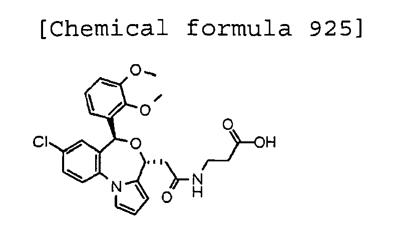

Ethyl 3-({2-[(4R,6S)-8-chloro-6-(2,3-dimethoxyphenyl)-4H,6H-pyrrolo[1,2-a][4,1]benzoxazepin-4-yl]acetyl}amino)propionate (98 mg) was dissolved in methanol-water-tetrahydrofuran (1:2:1, 4 mL). To the resulting solution was added potassium carbonate (106 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (67.8 mg).
MS (ESI) m/z : 485 (M⁺+H).
¹H-NMR (CDCl₃) δ: 2.6-2.6 (2H, m), 2.9-2.9 (2H, m), 3.4 (3H, s), 3.4-3.5 (1H, m), 3.5-3.7 (2H, m), 3.8-3.8 (1H, m), 3.9 (3H, s), 4.8 (1H, dd, J = 5.1, 6.6 Hz), 5.8 (1H, s), 6.3-6.4 (2H, m), 6.7-6.8 (1H, m), 7.0 (1H, dd, J = 7.9, 1.6 Hz), 7.1-7.1 (1H, m), 7.2-7.3 (2H, m), 7.3-7.4 (2H, m). Elemental analysis for: C₂₅H₂₅ClN₂O₆
Calculated: C, 61.92; H, 5.20; N, 5.78.
Found: C, 61.94; H, 5.23; N, 5.47.

### Example 441

3-({2-[(4S,6R)-8-Chloro-6-(2,3-dimethoxyphenyl)-4H,6H-pyrrolo[1,2-a][4,1]benzoxazepin-4-yl]acetyl}amino)propionic acid

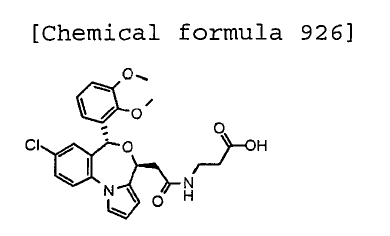

Ethyl 3-({2-[(4S,6R)-8-chloro-6-(2,3-dimethoxyphenyl)-4H,6H-pyrrolo[1,2-a][4,1]benzoxazepin-4-yl]acetyl}amino)propionate (122 mg) was dissolved in methanol-water-tetrahydrofuran (1:2:1, 4 mL). To the resulting solution was added potassium carbonate (131 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (80.1 mg).
MS (ESI) m/z : 485 (M⁺+H).
Elemental analysis for: C₂₅H₂₅ClN₂O₆
Calculated: C, 61.92; H, 5.20; N, 5.78.
Found: C, 61.99; H, 5.43; N, 5.38.

### Example 442

2-(1-{2-[(4R,6S)-8-Chloro-6-(2,3-dimethoxyphenyl)-4H,6H-pyrrolo[1,2-a][4,1]benzoxazepin-4-yl]acetyl}-1,2,3,6-tetrahydro-4-pyridinyl)acetic acid

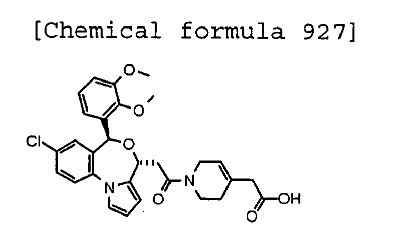

Ethyl 2-(1-{2-[(4R,6S)-8-chloro-6-(2,3-dimethoxyphenyl)-4H,6H-pyrrolo[1,2-a][4,1]benzoxazepin-4-yl]acetyl}-1,2,3,6-tetrahydro-4-pyridinyl)acetate (94 mg) was dissolved in methanol-water-tetrahydrofuran (1:2:1, 4 mL). To the resulting solution was added potassium carbonate (92 mg). The resulting mixture was stirred at 55°C for 15 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (71.2 mg).
MS (ESI) m/z : 538 (M⁺+H).
¹H-NMR (CDCl₃) δ: 2.1-2.3 (3H, br m), 2.9 (1H, dd, J = 14.3, 4.5 Hz), 3.0-3.1 (2H, m), 3.2-3.3 (1H, m), 3.4 (3H, s), 3.7 (1H, d, J = 37.1 Hz), 3.8 (3H, s), 4.0-4.2 (2H, m), 5.0 (1H, dd, J = 8.7, 4.5 Hz), 5.6 (1H, d, J = 45.9 Hz), 5.7-5.7 (1H, m), 6.2-6.3 (1H, m), 6.3-6.4 (1H, m), 6.6-7.4 (7H, m).
Elemental analysis for: C₂₉H₂₉ClN₂O₆
Calculated: C, 64.86; H, 5.44; N, 5.22.
Found: C, 64.54; H, 5.57; N, 5.08.

### Example 443

2-(1-{2-[(4S,6R)-8-chloro-6-(2,3-dimethoxyphenyl)-4H,6H-pyrrolo[1,2-a][4,1]benzoxazepin-4-yl]acetyl}-1,2,3,6-tetrahydro-4-pyridinyl)acetic acid

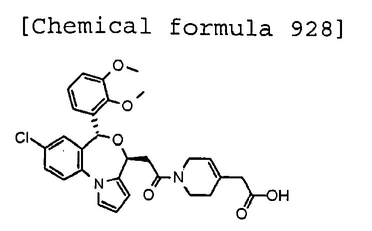

Ethyl 2-(1-{2-[(4S,6R)-8-chloro-6-(2,3-dimethoxyphenyl)-4H,6H-pyrrolo[1,2-a][4,1]benzoxazepin-4-yl]acetyl}-1,2,3,6-tetrahydro-4-pyridinyl)acetate (104 mg) was dissolved in methanol-water-tetrahydrofuran (1:2:1, 4 mL). To the resulting solution was added potassium carbonate (102 mg). The resulting mixture was stirred at 55°C for 15 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (87.4 mg).
MS (ESI) m/z : 538 (M⁺+H).
Elemental analysis for: C₂₉H₂₉ClN₂O₆
Calculated: C, 64.86; H, 5.44; N, 5.22.
Found: C, 64.46; H, 5.60; N, 5.04.

### Example 444

2-[4-({2-[(4R,6S)-8-Chloro-6-(2,3-dimethoxyphenyl)-4H,6H-pyrrolo[1,2-a][4,1]benzoxazepin-4-yl]acetyl}amino)phenyl]acetic acid

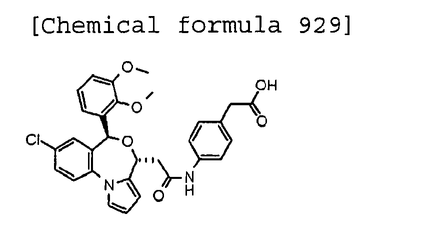

Ethyl 2-[4-({2-[(4R,6S)-8-chloro-6-(2,3-dimethoxyphenyl)-4H,6H-pyrrolo[1,2-a][4,1]benzoxazepin-4-yl]acetyl}amino)phenyl]acetate (91 mg) was dissolved in methanol-water-tetrahydrofuran(1:2:1, 4 mL). To the resulting solution was added potassium carbonate (87 mg). The resulting mixture was stirred at 55°C for 15 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (63.2 mg).
MS (ESI) m/z : 547 (M⁺+H)
Elemental analysis for: C₃₀H₂₇ClN₂O₆·0.25H₂O
Calculated: C, 65.34; H, 5.03; N, 5.08.
Found: C, 65.20; H, 5.01; N, 4.96.

### Example 445

2-[4-({2-[(4S,6R)-8-Chloro-6-(2,3-dimethoxyphenyl)-4H,6H-pyrrolo[1,2-a][4,1]benzoxazepin-4-yl]acetyl}amino)phenyl]acetic acid

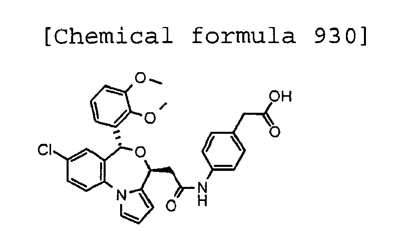

Ethyl 2-[4-({2-[(4S,6R)-8-chloro-6-(2,3-dimethoxyphenyl)-4H,6H-pyrrolo[1,2-a][4,1]benzoxazepin-4-yl]acetyl}amino)phenyl]acetate (91 mg) was dissolved in methanol-water-tetrahydrofuran (1:2:1, 4 mL). To the resulting solution was added potassium carbonate (87 mg). The resulting mixture was stirred at 55°C for 15 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (65 mg).
MS (ESI) m/z : 547 (M⁺+H) .
Elemental analysis for: C₃₀H₂₇ClN₂O₆·0.25H₂O
Calculated: C, 65.34; H, 5.03; N, 5.08.
Found: C, 65.21; H, 5.00; N, 5.02.

### Example 446

4-({2-[(4R,6S)-8-Chloro-6-(2,3-dimethoxyphenyl)-4H,6H-pyrrolo[1,2-a] [4,1]benzoxazepin-4-yl]acetyl}amino)benzoic acid

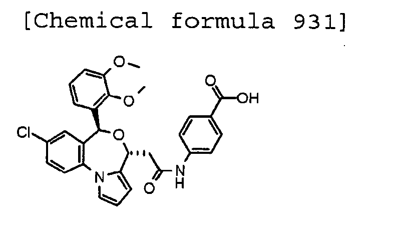

Methyl 4-({2-[(4R,6S)-8-chloro-6-(2,3-dimethoxyphenyl)-4H,6H-pyrrolo[1,2-a][4,1]benzoxazepin-4-yl]acetyl}amino)benzoate (36 mg) was dissolved in methanol-water-tetrahydrofuran (1:2:1, 4 mL). To the resulting solution was added potassium carbonate (36 mg). The resulting mixture was stirred at 55°C for 7 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (18.3 mg).
MS (ESI) m/z : 533 (M⁺+H) .
Elemental analysis for: C₂₉H₂₅ClN₂O₆·0.25H₂O
Calculated: C, 64.81; H, 4.78; N, 5.21.
Found: C, 64.80; H, 4.81; N, 5.16.

### Example 447

4-({2-[(4S,6R)-8-Chloro-6-(2,3-dimethoxyphenyl)-4H,6H-pyrrolo[1,2-a][4,1]benzoxazepin-4-yl]acetyl}amino)benzoic acid

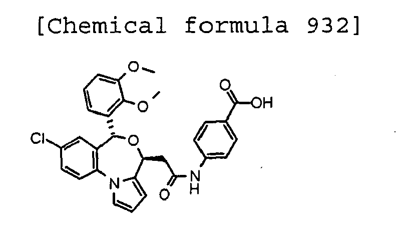

Methyl 4-({2-((4S,6R)-8-chloro-6-(2,3-dimethoxyphenyl)-4H,6H-pyrrolo[1,2-a] [4,1]benzoxazepin-4-yl]acetyl}amino)benzoate (41 mg) was dissolved in methanol-water-tetrahydrofuran (1:2:1, 4 mL). To the resulting solution was added potassium carbonate (41 mg). The resulting mixture was stirred at 55°C for 7 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (22.2 mg).
MS (ESI) m/z : 533 (M⁺+H).
Elemental analysis for: C₂₉H₂₅ClN₂O₆·0.25H₂O
Calculated: C, 64.81; H, 4.78; N, 5.21.
Found: C, 64.74; H, 4.82; N, 5.18.

### Example 448

(1S,2R)-2-({2-[(4R,6S)-8-Chloro-6-(2,3-dimethoxyphenyl)-4H,6H-pyrrolo[1,2-a][4,1]benzoxazepin-4-yl]acetyl}amino)cyclohexane carboxylic acid

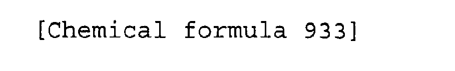

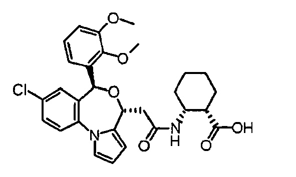

Ethyl (1S,2R)-2-({2-[(4R,6S)-8-chloro-6-(2,3-dimethoxyphenyl)-4H,6H-pyrrolo[1,2-a][4,1]benzoxazepin-4-yl]acetyl}amino)cyclohexane carboxylate (92 mg) was dissolved in methanol-water-tetrahydrofuran (1:2:1, 4 mL). To the resulting solution was added potassium carbonate (90 mg). The resulting mixture was stirred at 55°C for 18 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (71.5 mg).
MS (ESI) m/z : 540 (M⁺+H) .
Elemental analysis for: C₂₉H₃₁ClN₂O₆
Calculated: C, 64.62; H, 5.80; N, 5.20.
Found: C, 64.20; H, 5.83; N, 4.76.

### Example 449

(1S,2R)-2-({2-[(4S,6R)-8-Chloro-6-(2,3-dimethoxyphenyl)-4H, 6H-pyrrolo[1,2-a][4,1]benzoxazepin-4-yl]acetyl}amino)cyclohexane carboxylic acid

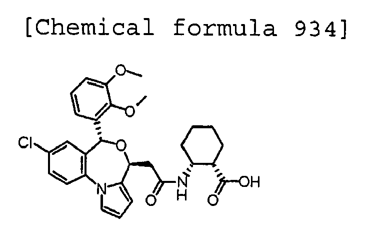

Ethyl (1S,2R)-2-({2-[(4S,6R)-8-chloro-6-(2,3-dimethoxyphenyl)-4H,6H-pyrrolo[1,2-a][4,1]benzoxazepin-4-yl]acetyl}amino)cyclohexane carboxylate (101 mg) was dissolved in methanol-water-tetrahydrofuran (1:2:1, 4 mL). To the resulting solution was added potassium carbonate (98 mg). The resulting mixture was stirred at 55°C for 18 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (81.4 mg).
MS (ESI) m/z : 540 (M⁺+H).
¹H-NMR (CDCl₃) δ: 1.0-1.5 (5H, m), 1.8-1.9 (1H, br m), 2.0-2.1 (2H, br m), 2.2-2.3 (1H, br m), 2.4-2.5 (1H, br m), 2.9 (2H, d, J = 6.8 Hz), 3.4 (3H, s), 3.9 (3H, s), 4.8 (1H, t, J = 6.2 Hz), 5.8 (1H, s), 6.2-6.3 (2H, m), 6.4 (1H, t, J = 3.2 Hz), 6.7 (1H, d, J = 2.0 Hz), 6.9-7.0 (1H, m), 7.1-7.1 (1H, m), 7.2 (1H, t, J = 7.9 Hz), 7.2-7.3 (1H, m), 7.3-7.4 (2H, m).
Elemental analysis for: C₂₉H₃₁ClN₂O₆
Calculated: C, 64.62; H, 5.80; N, 5.20.
Found: C, 64.19; H, 5.84; N, 4.74.

### Example 450

1-{3-[(4R,6S)-8-Chloro-6-(2,3-dimethoxyphenyl)-4H,6H-pyrrolo[1,2-a][4,1]benzoxazepin-4-yl]propanoyl}-4-piperidine carboxylic acid

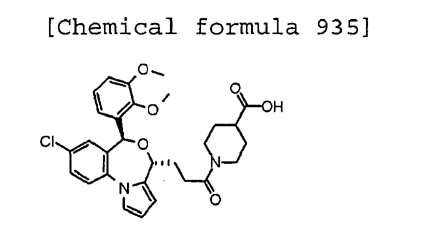

Ethyl 1-{3-[(4R,6S)-8-chloro-6-(2,3-dimethoxyphenyl)-4H,6H-pyrrolo[1,2-a][4,1]benzoxazepin-4-yl]propanoyl}-4-piperidine carboxylate (71 mg) was dissolved in methanol-water-tetrahydrofuran (1:2:1, 4 mL). To the resulting solution was added potassium carbonate (69 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (52.0 mg).
MS (ESI) m/z : 540 (M⁺+H).
¹H-NMR (CDCl₃) δ: 1.2-1.3 (1H, m), 1.6-1.7 (2H, m), 1.9-2.0 (2H, m), 2.4-2.4 (2H, m), 2.5-2.6 (2H, m), 2.6-2.7 (1H, m), 2.8-2.9 (1H, m), 3.1-3.2 (1H, m), 3.4 (3H, s), 3.9 (3H, s), 4.4-4.5 (2H, m), 5.7 (1H, br s), 6.3-6.4 (2H, m), 6.7-6.7 (1H, m), 6.9 (1H, dd, J = 8.2, 1.6 Hz), 7.1 (1H, dd, J = 2.8, 1.6 Hz), 7.2 (1H, t, J = 7.9 Hz), 7.3-7.3 (1H, m), 7.3-7.4 (2H, m).
Elemental analysis for: C₂₉H₃₁ClN₂O₆
Calculated: C, 64.62; H, 5.80; N, 5.20.
Found: C, 64.59; H, 5.92; N, 4.90.

### Example 451

1-{3-[(4S,6R)-8-Chloro-6-(2,3-dimethoxyphenyl)-4H,6H-pyrrolo[1,2-a][4,1]benzaxazepin-4-yl]propanoyl}-4-piperidine carboxylic acid

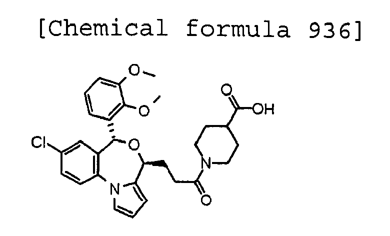

Ethyl 1-{3-[(4S,6R)-8-chloro-6-(2,3-dimethoxyphenyl)-4H,6H-pyrrolo[1,2-a][4,1]benzoxazepin-4-yl]propanoyl}-4-piperidine carboxylate (67 mg) was dissolved in methanol-water-tetrahydrofuran (1:2:1, 4 mL). To the resulting solution was added potassium carbonate (65 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (42.3 mg).
MS (ESI) m/z : 540 (M⁺+H) .
Elemental analysis for: C₂₉H₃₁ClN₂O₆
Calculated: C, 64.62; H, 5.80; N, 5.20.
Found: C, 64.56; H, 5.93; N, 4.72.

### Example 452

2-(1-{3-[(4S,6R)-8-Chloro-6-(2,3-dimethoxyphenyl)-4H,6H-pyrrolo[1,2-a][4,1]benzoxazepin-4-yl]propanoyl}-4-piperidinyl)acetic acid

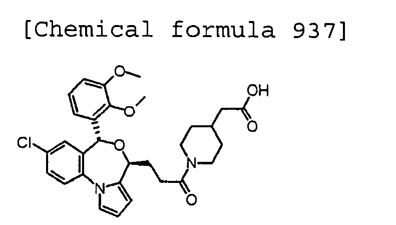

Ethyl 2-(1-{3-[(4S,6R)-8-chloro-6-(2,3-dimethoxyphenyl)-4H,6H-pyrrolo[1,2-a][4,1]benzoxazepin-4-yl]propanoyl}-4-piperidinyl)acetate (80 mg) was dissolved in methanol-water-tetrahydrofuran (1:2:1, 4 mL). To the resulting solution was added potassium carbonate (76 mg). The resulting mixture was stirred at 55°C for 23 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (52.1 mg).
MS (ESI) m/z : 554 (M⁺+H) .
Elemental analysis for: C₃₀H₃₃ClN₂O₆
Calculated: C, 65.15; H, 6.01; N, 5.07.
Found: C, 65.21; H, 6.19; N, 4.69.

### Example 453

2-(1-{2-[(4S,6S)-8-Chloro-6-(2,3-dimethoxyphenyl)-4H,6H-pyrrolo[1,2-a] [4,1]benzoxazepin-4-yl]-2,2-difluoroacetyl}-4-piperidinyl)acetic acid

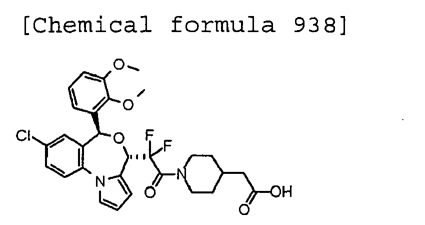

Ethyl 2-(1-{2-[(4S,6S)-8-chloro-6-(2,3-dimethoxyphenyl)-4H,6H-pyrrolo[1,2-a][4,1]benzoxazepin-4-yl]-2,2-difluoroacetyl}-4-piperidinyl)acetate (122 mg) was dissolved in methanol-water-tetrahydrofuran (1:2:1, 4 mL). To the resulting solution was added potassium carbonate (56 mg). The resulting mixture was stirred at 55°C for 16 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. After concentration of the filtrate, the residue thus obtained was purified by preparative thin layer chromatography to give the title compound (7.9 mg).
MS (ESI) m/z : 576 (M⁺+H)
Elemental analysis for: C₂₉H₂₉ClF₂N₂O₆
Calculated: C, 60.58; H, 5.08; N, 4.87.
Found: C, 60.59; H, 5.15; N, 4.74.

### Example 454

2-(1-{2-[(4S,6R)-8-Chloro-6-(2,3-dimethoxyphenyl)-10-fluoro-4H,6H-pyrrolo[1,2-a][4,1]benzoxazepin-4-yl]acetyl}-4-piperidinyl)acetic acid

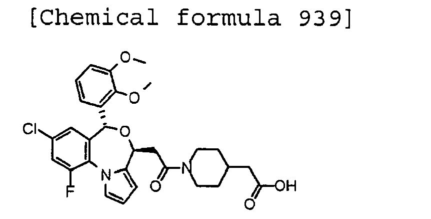

Ethyl 2-(1-{2-[(4S,6R)-8-chloro-6-(2,3-dimethoxyphenyl)-10-fluoro-4H,6H-pyrrolo[1,2-a] [4,1]benzoxazepin-4-yl]acetyl}-4-piperidinyl)acetate (41 mg) was dissolved in methanol-water-tetrahydrofuran (1:2:1, 4 mL). To the resulting solution was added potassium carbonate (39.0 mg). The resulting mixture was stirred at 55°C for 16 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (33.2 mg).
MS (ESI) m/z : 558 (M⁺+H)
Elemental analysis for: C₂₉H₃₀ClFN₂O₆
Calculated: C, 62.53; H, 5.43; N, 5.03.
Found: C, 62.55; H, 5.67; N, 4.73.

### Example 455

1-{2-[(4R,6S)-8-Chloro-6-(2,3-dimethoxyphenyl)-10-fluoro-4H,6H-pyrrolo[1,2-a][4,1]benzoxazepin-4-yl]acetyl}-3-azetidine carboxylic acid

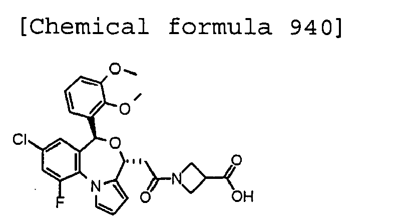

Methyl 1-{2-[(4R,6S)-8-chloro-6-(2,3-dimethoxyphenyl)-10-fluoro-4H,6H-pyrrolo[1,2-a][4,1]benzoxazepin-4-yl]acetyl}-3-azetidine carboxylate (34.0 mg) was dissolved in methanol-water-tetrahydrofuran (1:2:1, 2 mL). To the resulting solution was added potassium carbonate (35.5 mg). The resulting mixture was stirred at 55°C for 6.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (25.1 mg).
MS (ESI) m/z : 515 (M⁺+H).
Elemental analysis for: C₂₆H₂₄ClFN₂O₆·0.25H₂O
Calculated: C, 60.12; H, 4.75; N, 5.39.
Found: C, 60.08; H, 5.00; N, 5.10.

### Example 456

1-{2-[(4R,6S)-8-Chloro-6-(2,3-dimethoxyphenyl)-10-fluoro-4H,6H-pyrrolo[1,2-a][4,1]benzoxazepin-4-yl]acetyl}-3-azetidineacetic acid

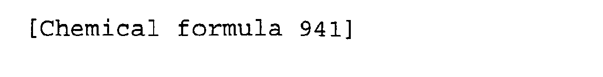

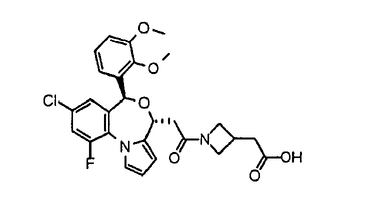

Methyl 1-{2-[(4R,6S)-8-chloro-6-(2,3-dimethoxyphenyl)-10-fluoro-4H,6H-pyrrolo[1,2-a][4,1]benzoxazepin-4-yl]acetyl}-3-azetidineacetate (31.0 mg) was dissolved in methanol-water-tetrahydrofuran (1:2:1, 2 mL). To the resulting solution was added potassium carbonate (30.8 mg). The resulting mixture was stirred at 55°C for 6.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (24.0 mg).
MS (ESI) m/z : 529 (M⁺+H).
Elemental analysis for: C₂₇H₂₆ClFN₂O₆·0.5H₂O
Calculated: C, 60.28; H, 5.06; N, 5.21.
Found: C, 60.58; H, 5.18; N, 4.83.

### Example 457

2-(2-{2-[(trans)-6-(2,3-Dimethoxyphenyl)-8-fluoro-4H,6H-pyrrolo[1,2-a][4,1]benzoxazepin-4-yl]ethyl}-2H-1,2,3,4-tetrazol-5-yl)acetic acid

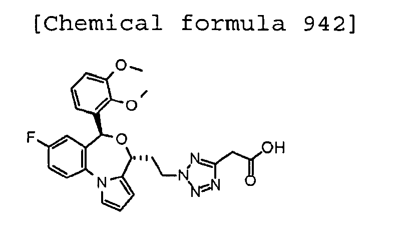

Ethyl 2-(2-{2-[(trans)-6-(2,3-dimethoxyphenyl)-8-fluoro-4H,6H-pyrrolo[1,2-a][4,1]benzoxazepin-4-yl]ethyl}-2H-1,2,3,4-tetrazol-5-yl)acetate (47.5 mg) was dissolved in methanol-water-tetrahydrofuran (1:2:1, 4 mL). To the resulting solution was added potassium carbonate (50.4 mg). The resulting mixture was stirred at 55°C for 1.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added isopropyl ether, ethyl acetate and hexane. The solid thus formed was collected by filtration to give the title compound (19.0 mg).
MS (ESI) m/z : 494 (M⁺+H).
Elemental analysis for: C₂₅H₂₄FN₅O₅·0.5H₂O·0.25 diisopropyl ether
Calculated: C, 60.28; H, 5.44; N, 13.26.
Found: C, 60.27; H, 5.32; N, 13.11.

### Example 458

2-(1-{2-[(trans)-6-(2,3-Dimethoxyphenyl)-8-fluoro-4H,6H-pyrrolo[1,2-a][4,1]benzoxazepin-4-yl]ethyl}-1H-1,2,3,4-tetrazol-5-yl)acetic acid

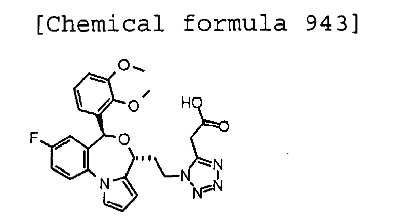

Ethyl 2-(1-{2-[(trans)-6-(2,3-dimethoxyphenyl)-8-fluoro-4H,6H-pyrrolo[1,2-a][4,1]benzoxazepin-4-yl]ethyl}-1H-1,2,3,4-tetrazol-5-yl)acetate (23.5 mg) was dissolved in methanol-water-tetrahydrofuran (1:2:1, 4 mL). To the resulting solution was added potassium carbonate (24.9 mg). The resulting mixture was stirred at 55°C for 1.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added isopropyl ether, ethyl acetate, and hexane and the solid thus formed was collected by filtration to give the title compound (13.9 mg).
MS (ESI) m/z : 494 (M⁺+H).
Elemental analysis for: C₂₅H₂₄FN₅O₅·0.5H₂O
Calculated: C, 59.76; H, 5.01; N, 13.94.
Found: C, 59.59; H, 5.27; N, 14.28.

### Example 459

2-(1-{2-[(trans)-6-(2,3-Dimethoxyphenyl)-8-fluoro-4H,6H-pyrrolo[1,2-a][4,1]benzoxazepin-4-yl]acetyl}-4-piperidinyl)acetic acid

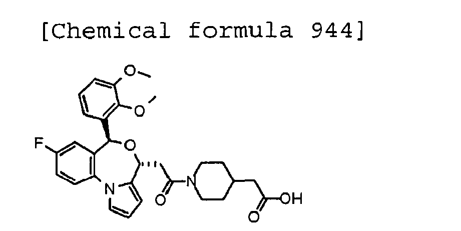

Ethyl 2-(1-{2-[(trans)-6-(2,3-dimethoxyphenyl)-8-fluoro-4H,6H-pyrrolo[1,2-a][4,1]benzoxazepin-4-yl]acetyl}-4-piperidinyl)acetate (39.2 mg) was dissolved in methanol-water-tetrahydrofuran (1:2:1, 4 mL). To the resulting solution was added potassium carbonate (39.4 mg). The resulting mixture was stirred at 55°C for 1 hour. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (28.7 mg).
MS (ESI) m/z : 523 (M⁺+H).
Elemental analysis for: C₂₉H₃₁FN₂O₆·0.25H₂O
Calculated: C, 66.09; H, 6.02; N, 5.31.
Found: C, 66.05; H, 6.19; N, 5.14.

### Example 460

1-{3-[(4R,6S)-8-Chloro-6-(2,3-dimethoxyphenyl)-4H,6H-pyrrolo[1,2-a][4,1]benzoxazepin-4-yl]propyl}-1H-1,2,3-triazol-5-carboxylic acid

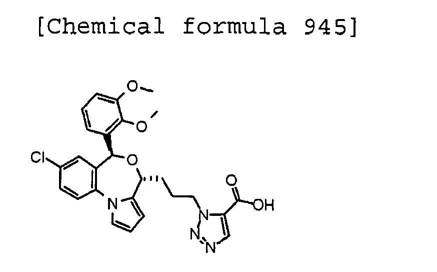

Ethyl 1-{3-[(4R,6S)-8-chlorc-6-(2,3-dimethoxyphenyl)-4H,6H-pyrrolo[1,2-a][4,1]benzoxazepin-4-yl]propyl}-1H-1,2,3-triazol-5-carboxylate (49.1 mg) was dissolved in methanol-water-tetrahydrofuran (1:2:1, 4 mL). To the resulting solution was added potassium carbonate (50.5 mg). The resulting mixture was stirred at 55°C for 1.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (29.2 mg).
MS (ESI) m/z : 509 (M⁺+H).
Elemental analysis for: C₂₆H₂₅ClN₄O₅·0.5 isopropanol
Calculated: C, 61.28; H, 5.42; N, 10.39.
Found: C, 61.49; H, 5.22; N, 10.40.

### Example 461

1-{3-[(4R,6S)-8-Chloro-6-(2,3-dimethoxyphenyl)-4H,6H-pyrrolo[1,2-a][4,1]benzoxazepin-4-yl]propyl}-1H-1,2,3-triazole-4-carboxylic acid

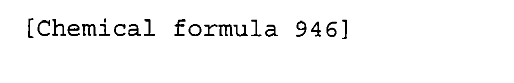

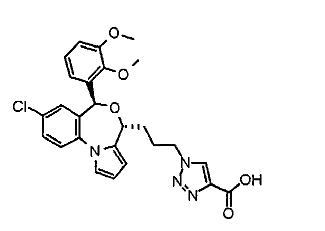

Ethyl 1-{3-[(4R,6S)-8-chloro-6-(2,3-dimethoxyphenyl)-4H,6H-pyrrolo[1,2-a] [4,1]benzoxazepin-4-yl]propyl}-1H-1,2,3-triazol-4-carboxylate (84.9 mg) was dissolved in methanol-water-tetrahydrofuran (1:2:1, 4 mL). To the resulting solution was added potassium carbonate (87.4 mg). The resulting mixture was stirred at 55°C for 1.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (57.1 mg).
MS (ESI) m/z : 509 (M⁺+H)
Elemental analysis for: C₂₆H₂₅ClN₄O₅·0.5H₂O
Calculated: C, 60.29; H, 5.06; N, 10.82.
Found: C, 60.80; H, 5.26; N, 10.38.

### Example 462

2-(1-{3-[(4R,6S)-8-Chloro-6-(2,3-dimethoxyphenyl)-4H,6H-pyrrolo[1,2-a] [4,1]benzoxazepin-4-yl]propyl}-1H-1,2,3-triazol-5-yl)acetic acid

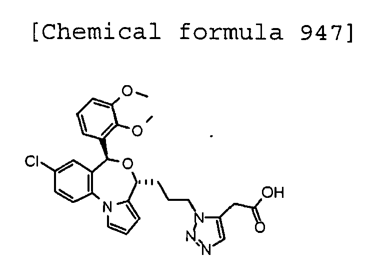

2-(1-{3-[(4R,6S)-8-Chloro-6-(2,3-dimethoxyphenyl)-4H,6H-pyrrolo[1,2-a][4,1]benzoxazepin-4-yl]propyl}-1H-1,2,3-triazol-5-yl)acetonitrile (23 mg) was dissolved in isopropanol (2 mL). To the resulting solution was added a 5N aqueous sodium hydroxide solution (1 mL). The resulting mixture was stirred at 80°C for 18 hours. Under ice cooling, 1N hydrochloric acid was added to the reaction mixture to make it acidic, followed by extraction with chloroform. The organic layer was dried over anhydrous sodium sulfate. The filtrate was then concentrated. To the residue thus obtained were added ethyl acetate and hexane. The solid thus formed was collected by filtration to give the title compound (7.7 mg).
MS (ESI) m/z : 523 (M⁺+H).
Elemental analysis for: C₂₇H₂₇ClN₄O₅·0.5H₂O
Calculated: C, 60.96; H, 5.30; N, 10.53.
Found: C, 61.18; H, 5.29; N, 10.18.

### Example 463

2-(1-{3-[(4R,6S)-8-Chloro-6-(2,3-dimethoxyphenyl)-4H,6H-pyrrolo[1,2-a][4,1]benzoxazepin-4-yl]propyl}-1H-1,2,3-triazol-4-yl)acetic acid

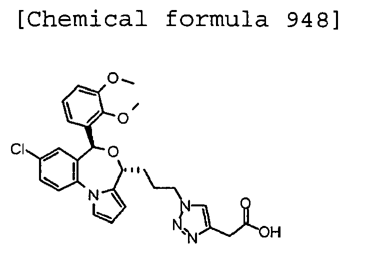

2-(1-{3-[(4R,6S)-8-Chloro-6-(2,3-dimethoxyphenyl)-4H,6H-pyrrolo[1,2-a][4,1]benzoxazepin-4-yl]propyl}-1H-1,2,3-triazol-4-yl)acetonitrile (91 mg) was dissolved in isopropanol (8 mL). To the resulting solution was added a 5N aqueous sodium hydroxide solution (4 mL). The resulting mixture was stirred at 80°C for 18 hours. Under ice cooling, 1N hydrochloric acid was added to the reaction mixture to make it acidic, followed by extraction with chloroform. The organic layer was dried over anhydrous sodium sulfate. The filtrate was then concentrated. To the residue thus obtained were added ethyl acetate and hexane. The solid thus formed was collected by filtration to give the title compound (52.0 mg).
MS (ESI) m/z : 523 (M⁺+H).
Elemental analysis for: C₂₇H₂₇ClN₄O₅
Calculated: C, 62.01; H, 5.20; N, 10.71.
Found: C, 62.03; H, 5.47; N, 10.39.

### Example 464

2-(1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,4-triazol-5-yl)acetic acid

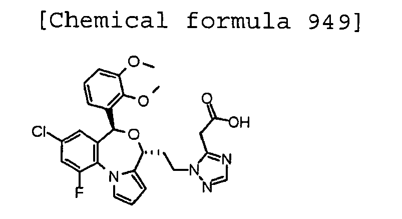

2-(1-{2-[(4R,6S)-8-Chloro-6-(2,3-dimethoxyphenyl)-10-fluoro-4H,6H-pyrrolo[1,2-a] [4,1]benzoxazepin-4-yl]ethyl}-1H-1,2,4-triazol-5-yl)acetate (88.3 mg) was dissolved in methanol-water-tetrahydrofuran (1:2:1, 4 mL). To the resulting solution was added potassium carbonate (87.9 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (69.6 mg).
MS (ESI) m/z : 527 (M⁺+H).
Elemental analysis for: C₂₆H₂₄ClFN₄O₅
Calculated: C, 59.26; H, 4.59; N, 10.63.
Found: C, 59.43; H, 4.82; N, 10.26.

### Example 465

2-(2-{3-[(4R,6S)-8-Chloro-6-(2,3-dimethoxyphenyl)-10-fluoro-4H,6H-pyrrolo[1,2-a][4,1]benzoxazepin-4-yl]propyl}-2H-1,2,3,4-tetrazol-5-yl)acetic acid

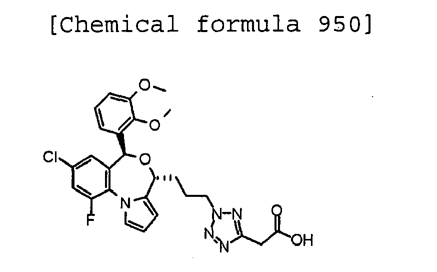

Ethyl 2-(2-{3-[(4R,6S)-8-chloro-6-(2,3-dimethoxyphenyl)-10-fluoro-4H,6H-pyrrolo[1,2-a][4,1]benzoxazepin-4-yl]propyl}-2H-1,2,3,4-tetrazol-5-yl)acetate (76.0 mg) was dissolved in methanol-water-tetrahydrofuran (1:2:1, 4 mL). To the resulting solution was added potassium carbonate (73.7 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (43.6 mg).
MS (ESI) m/z : 542 (M⁺+H).
Elemental analysis for: C₂₆H₂₅ClFN₅O₆·0.5H₂O·1.0 diethyl ether
Calculated: C, 57.64; H, 5.80; N, 11.20.
Found: C, 58.46; H, 4.88; N, 10.42.

### Example 466

2-(1-{3-[(4R,6S)-8-Chloro-6-(2,3-dimethoxyphenyl)-10-fluoro-4H,6H-pyrrolo[1,2-a] [4,1]benzoxazepin-4-yl]propyl}-1H-1,2,3,4-tetrazol-5-yl)acetic acid

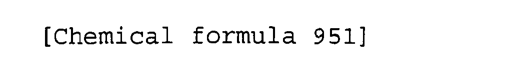

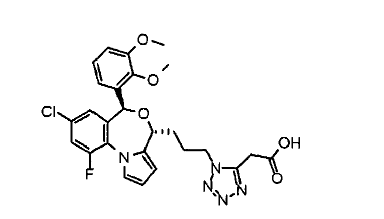

Ethyl 2-(1-{3-[(4R,6S)-8-chloro-6-(2,3-dimethoxyphenyl)-10-fluoro-4H,6H-pyrrolo[1,2-a][4,1]benzoxazepin-4-yl]propyl}-1H-1,2,3,4-tetrazol-5-yl)acetate (26.9 mg) was dissolved in methanol-water-tetrahydrofuran (1:2:1, 2 mL). To the resulting solution was added potassium carbonate (26.0 mg). The resulting mixture was stirred at 55°C for 12 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ethyl acetate and hexane. The solid thus formed was collected by filtration to give the title compound (20.2 mg).
MS (ESI) m/z : 542 (M⁺+H).
Elemental analysis for: C₂₆H₂₅ClFN₅O₅·0.5H₂O
Calculated: C, 56.68; H, 4.76; N, 12.71.
Found: C, 56.56; H, 4.52; N, 12.50.

### Example 467

2-(1-{2-[(4R,65)-8-chloro-6-(2,3-dimethoxyphenyl)-10-fluoro-4H,6H-pyrrolo[1,2-a][4,1]benzoxazepin-4-yl]ethyl}-1H-1,2,3-triazol-5-yl)acetic acid

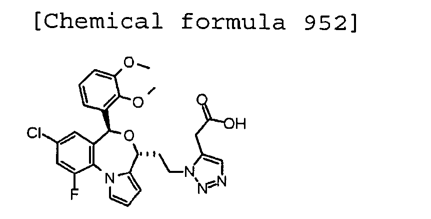

2-(1-{2-[(4R,6S)-8-Chloro-6-(2,3-dimethoxyphenyl)-10-fluoro-4H,6H-pyrrolo[1,2-a][4,1]benzoxazepine5-yl]ethyl}-1H-1,2,3-triazol-4-yl)acetonitrile (44 mg) was dissolved in isopropanol (4 mL). To the resulting solution was added a 5N aqueous sodium hydroxide solution (2 mL). The resulting mixture was heated under reflux for 2.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added ether, ethyl acetate and hexane. The solid thus formed was collected by filtration to give the title compound (41.4 mg).
MS (ESI) m/z : 527 (M⁺+H) .
Elemental analysis for: C₂₆H₂₅ClFN₅O₅·0.25H₂O
Calculated: C, 58.76; H, 4.65; N, 10.54.
Found: C, 58.48; H, 4.74; N, 10.29.

### Example 468

2-(1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-4-yl)acetic acid

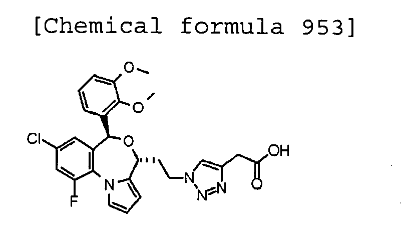

2-(1-{2-[(4R,6S)-8-Chloro-6-(2,3-dimethoxyphenyl)-10-fluoro-4H,6H-pyrrolo[1,2-a][4,1]benzoxazepin-4-yl]ethyl}-1H-1,2,3-triazol-4-yl)acetonitrile (244 mg) was dissolved in isopropanol (20 mL). To the resulting solution was added a 5N aqueous sodium hydroxide solution (10 mL). The reaction mixture was heated under reflux for 2.5 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. The filtrate was then concentrated. To the residue thus obtained were added diethyl ether, ethyl acetate and hexane. The solid thus formed was collected by filtration to give the title compound (188.1 mg).
MS (ESI) m/z : 527 (M⁺+H).
Elemental analysis for: C₂₆H₂₅ClFN₅O₅·0.5H₂O·1.0 diethyl ether
Calculated: C, 59.26; H, 4.59; N, 10.63.
Found: C, 58.95; H, 4.92; N, 10.59.

### Example 469

2-(1-{2-[(4R,6S)-8-Chloro-6-(2,3-dimethoxyphenyl)-4H,6H-pyrrolo[1,2-a][4,1]benzoxazepin-4-yl]-2-methylpropyl}-1H-1,2,3-triazol-4-yl)acetic acid

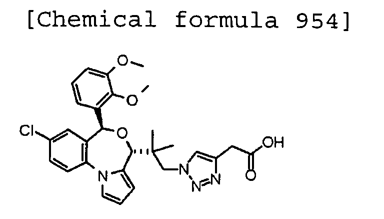

2-(1-{2-[(4R,6S)-8-Chloro-6-(2,3-dimethoxyphenyl)-4H,6H-pyrrolo[1,2-a][4,1]benzoxazepin-4-yl]-2-methylpropyl}-1H-1,2,3-triazol-4-yl)acetonitrile (54 mg) was dissolved in isopropanol (4 mL). To the resulting solution was added a 5N aqueous sodium hydroxide solution (2 mL). The resulting mixture was heated under reflux for 2 hours. Under ice cooling, 1N hydrochloric acid was added to the reaction mixture to make it acidic, followed by extraction with ethyl acetate. The extract was washed with saturated brine. The organic layer was dried over anhydrous sodium sulfate. After concentration of the filtrate, the residue thus obtained was purified by preparative thin layer chromatography to give the title compound (7.7 mg).
MS (ESI) m/z : 538 (M⁺+H).
¹H-NMR (CDCl₃) δ: 1.1 (3H, br s), 1.2-1.3 (4H, br m), 3.3-3.6 (5H, m), 3.8-4.0 (4H, m), 4.4-4.6 (2H, br m), 5.6 (1H, s), 6.3 (1H, br s), 6.5 (1H, br s), 6.7 (1H, s), 6.9 (1H, d, J = 8.5 Hz), 7.0 (1H, s), 7.1-7.2 (1H, br m), 7.2-7.4 (3H, m).
Elemental analysis for: C₂₈H₂₉ClN₄O₅·2.25H₂O
Calculated: C, 58.23; H, 5.85; N, 9.70.
Found: C, 57.83; H, 5.26; N, 9.50.

### Referential Example 301

[5-Chloro-2-(1H-imidazol-1-yl)phenyl](2,3-dimethoxyphenyl)methanone

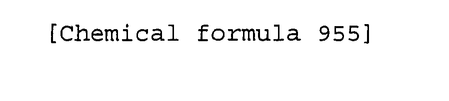

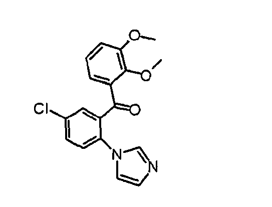

(2-Amino-5-chlorophenyl)(2,3-dimethoxyphenyl)methanone (32.6 g) was dissolved in methanol (500 mL). To the resulting solution was added a 40% aqueous glyoxal solution (12.7 mL). The resulting mixture was stirred overnight at room temperature. To the reaction mixture were added ammonium chloride (12.1 g) and a 38% aqueous formaldehyde solution (17.9 mL) and the resulting mixture was heated under reflux for one hour. After the reaction mixture was cooled to a temperature of ice cooling, a 85% aqueous phosphoric acid solution (15.7 mL) was added and the resulting mixture was heated under reflux for 5 hours. A 35% aqueous sodium hydroxide solution was added to the reaction mixture until it had a pH of from 8 to 10, followed by extraction twice with chloroform. After the organic layer was dried over anhydrous sodium sulfate, the solvent was distilled off under reduced pressure. The residue was purified by silica gel column chromatography (hexane:ethyl acetate = 1:4) to give the title compound (6.68 g).
MS (EI) m/z: 342 M⁺.
¹H-NMR (CDCl₃) δ: 3.49 (3H, s), 3.84 (3H, s), 6.93 (1H, br s), 7.00 (1H, t, J = 1.2 Hz), 7.01-7.08 (3H, m), 7.30 (1H, d, J = 8.8 Hz), 7.52 (1H, br s), 7.53-7.56 (2H, m).

### Referential Example 302

{5-Chloro-2-[2-(hydroxymethyl)-1H-imidazol-1-yl]phenyl}(2,3-dimethoxyphenyl)methanone

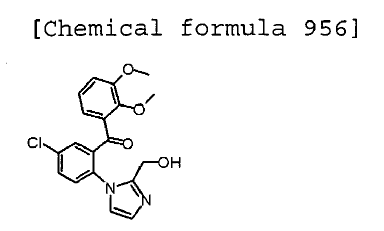

[5-Chloro-2-(1H-imidazol-1-yl)phenyl](2,3-dimethoxyphenyl)methanone (4.32 g) was suspended in xylene (60 mL). To the resulting suspension was added paraformaldehyde (12.0 g). The resulting mixture was stirred under heat at 135°C for one hour in a sealed glass tube. After cooling to room temperature, paraformaldehyde attached to the wall of the sealed tube was suspended in the reaction mixture and the resulting mixture was stirred under heat at 135°C for one hour again. The operation was repeated 8 times. The reaction mixture was then concentrated under reduced pressure. The residue was purified by silica gel column chromatography (chloroform:methanol = 100:1) to give the title compound (2.44 g).
MS (FAB) m/z: 373 (M+H)⁺.
¹H-NMR (CDCl₃) δ: 3.53 (3H, s), 3.84 (3H, s), 4.49 (2H, s), 6.83 (1H, s), 6.87 (1H, s), 6.98-7.05 (3H, m), 7.44 (1H, d, J = 8.1 Hz), 7.54-7.59 (2H, m).

### Referential Example 303

1-[4-Chloro-2-(2,3-dimethoxybenzoyl)phenyl]-1H-imidazol-2-carbaldehyde

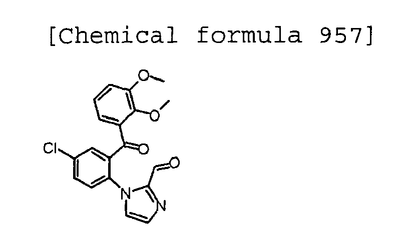

Imidazol-2-carbaldehyde (16 mg) was dissolved in dimethylsulfoxide (1 mL). At room temperature, sodium hydride (60% in oil, 7.0 mg) was added to the resulting solution and the mixture was stirred for one hour. To the reaction mixture was added (5-chloro-2-fluorophenyl)(2,3-dimethoxyphenyl)methanone (50 mg). The resulting mixture was stirred under heat at 95°C for 2 days. After cooling to room temperature, ethyl acetate was added to the reaction mixture and the layers separated. The organic layer was dried over anhydrous sodium sulfate. The solvent was distilled off under reduced pressure. The residue was purified by preparative thin layer chromatography on silica gel (hexane:ethyl acetate = 1:1) to give the title compound (34 mg).
MS (FAB) m/z: 371 (M+H)⁺.
¹H-NMR (CDCl₃) δ: 3.61 (3H, s), 3.85 (3H, s), 6.86-6.91 (1H, m), 6.99-7.02 (2H, m), 7.20 (2H, d, J = 8.8 Hz), 7.28 (1H, d, J = 8.3 Hz), 7.57 (1H, dd, J = 8.3, 2.2 Hz), 7.60 (1H, d, J = 2.2 Hz), 9.66 (1H, s).

### Referential Example 304

1-[4-Chloro-2-(2,3-dimethoxybenzoyl)phenyl]-1H-imidazol-2-carbaldehyde

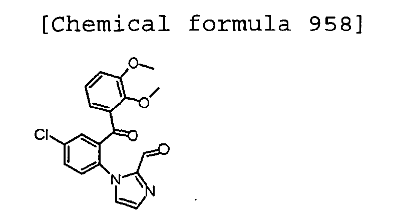

{5-Chloro-2-[2-(hydroxymethyl)-1H-imidazol-1-yl]phenyl}(2,3-dimethoxyphenyl)methanone (2.44 g) was dissolved in dichloromethane (100 mL). To the resulting solution was added manganese dioxide (5.68 g) and the resulting mixture was stirred under heat at 40°C overnight. To the reaction mixture was added tetrahydrofuran (50 mL). The resulting mixture was filtered through a Kiriyama funnel. The filtrate was concentrated under to give the title compound (2.00 g).
MS (FAB) m/z: 371 (M+H)⁺.
¹H-NMR (CDCl₃) δ: 3.61 (3H, s), 3.85 (3H, s), 6.86-6.91 (1H, m), 6.99-7.02 (2H, m), 7.20 (2H, d, J = 8.8 Hz), 7.28 (1H, d, J = 8.3 Hz), 7.57 (1H, dd, J = 8.3, 2.2 Hz), 7.60 (1H, d, J = 2.2 Hz), 9.66 (1H, s).

### Referential Example 305

(5-Chloro-2-{2-[2-(1,3-dioxolan-2-yl)-1-hydroxyethyl]-1H-imidazol-1-yl}phenyl)(2,3-dimethoxyphenyl)methanone

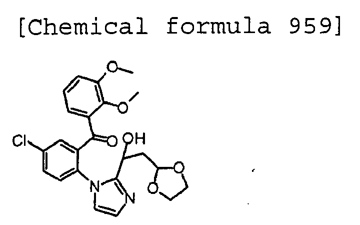

1-[4-Chloro-2-(2,3-dimethoxybenzoyl)phenyl]-1H-imidazol-2-carbaldehyde (500 mg) was dissolved in tetrahydrofuran (10 mL). To the resulting solution was added (1,3-dioxolan-2-ylmethyl)magnesium bromide (0.5 M tetrahydrofuran solution, 4.04 mL). The resulting mixture was heated under reflux for 30 minutes under a nitrogen gas stream. After cooling to a temperature of ice cooling, water and ethyl acetate were added to the reaction mixture and the layers separated. The organic layer was washed with saturated brine and dried over anhydrous sodium sulfate. The solvent was distilled off under reduced pressure. The residue was purified by silica gel column chromatography (chloroform:methanol = 100 : 3) to give the title compound (516 mg).
MS (FAB) m/z: 459 (M+H)⁺.
¹H-NMR (CDCl₃) δ: 2.09-2.26 (1H, m), 2.33-2.43 (1H, m), 3.52 (3H, s), 3.80-3.89 (5H, m), 3.94-3.98 (2H, m), 4.76-4.82 (1H, m), 5.09 (1H, br s), 6.81-6.86 (2H, m), 7.01 (3H, br s), 7.25-7.28 (1H, m), 7.53-7.59 (2H, m).

### Referential Example 306

trans-8-Chloro-6-(2,3-dimethoxyphenyl)-4-(1,3-dioxolan-2-ylmethyl)-4H,6H-imidazo[1,2-a][4,1]benzoxazepine

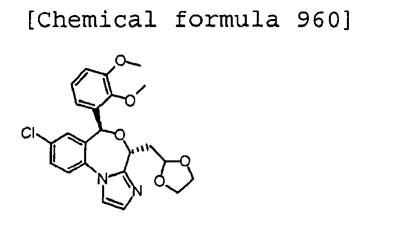

(5-Chloro-2-{2-[2-(1,3-dioxolan-2-yl)-1-hydroxyethyl]-1H-imidazol-1-yl}phenyl)(2,3-dimethoxyphenyl)methanone (34.5 g) was dissolved in ethanol (750 mL). To the resulting solution was added sodium borohydride (11.4 g). The resulting mixture was stirred at room temperature for 2 hours. After concentration of the reaction mixture under reduced pressure, ethyl acetate and water were added to the residue and the layers separated. The organic layer was dried over anhydrous sodium sulfate. The solvent was distilled off under reduced pressure. The residue was dissolved in dichloroethane (7500 mL). In a cooling bath containing methanol-ice, triethylamine (39.0 mL) and chloromethanesulfonate (8.73 mL) were added successively. The resulting mixture was stirred overnight at room temperature. To the reaction mixture was added a 1N aqueous sodium hydroxide solution (2000 mL) and the layers separated. The organic layer was dried over anhydrous sodium sulfate. The solvent was distilled off under reduced pressure. The residue was purified twice by silica gel column chromatography (hexane:ethyl acetate = from 1:1 to 2:3) to give the title compound (10.1 g).
MS (FAB) m/z: 443 (M+H)⁺.
¹H-NMR (CDCl₃) δ: 2.56-2.60 (3H, m), 3.48 (3H, s), 3.83-3.99 (5H, m), 4.65-4.70 (1H, m), 5.27-5.31 (1H, m), 5.66 (1H, s), 6.77 (1H, d, J = 2.2 Hz), 6.96 (1H, d, J = 8.3 Hz), 7.19 (1H, t, J = 8.1 Hz), 7.2-7.27 (1H, m), 7.31-7.42 (4H, m).

### Referential Example 307

8-Chloro-6-(2,3-dimethoxyphenyl)-4-(1,3-dioxolan-2-ylmethyl)-4H,6H-imidazo[1,2-a] [4,1]benzoxazepine

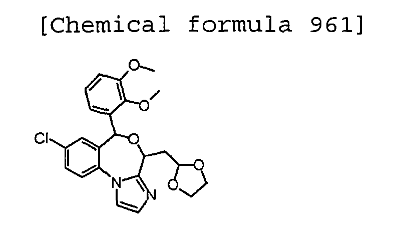

(5-Chloro-2-{2-[2-(1,3-dioxolan-2-yl)-1-hydroxyethyl]-1H-imidazol-1-yl}phenyl)(2,3-dimethoxyphenyl)methanone (47 mg) was dissolved in ethanol (2 mL). To the resulting solution was added sodium borohydride (8 mg). The resulting mixture was stirred at room temperature for 2 hours. After concentration of the reaction mixture under reduced pressure, ethyl acetate and water were added to the residue and the layers separated. The organic layer was dried over anhydrous sodium sulfate. The solvent was distilled off under reduced pressure. The residue was dissolved in dichloroethane (10 mL). To the resulting solution were added successively triethylamine (71µl) and chloromethanesulfonate (12 µl) in a cooling bath containing methanol-ice. The resulting mixture was stirred overnight at room temperature. To the reaction mixture was added a 1N aqueous sodium hydroxide solution (1 mL) and the layers separated. The organic layer was dried over anhydrous sodium sulfate. The solvent was distilled off under reduced pressure. The residue was purified by preparative thin layer silica gel column chromatography (hexane:ethyl acetate = 1:3) to give the title compound as a cis-trans mixture (cis:trans= 1:4, 27 mg).
MS (FAB) m/z: 443 (M+H)⁺.
¹H-NMR (CDCl₃) δ: 2.31-2.35 (cis of 3H, m), 2.56-2.60 (trans of 3H, m), 3.48 (trans of 3H, s), 3.57 (cis of 3H, s), 3.83-3.99 (cis of 5H, trans of 5H, m), 4.65-4.70 (trans of 1H, m), 5.16-5.20 (cis of 1H, m), 5.22-5.26 (cis of 1H, m), 5.27-5.31 (trans of 1H, m), 5.66 (trans of 1H, s), 6.11 (cis of 1H, s), 6.77 (trans of 1H, d, J = 2.2 Hz), 6.83-6.92 (cis of 1H, m), 6.96 (trans of 1H, d, J = 8.3 Hz), 6.99-7.07 (cis of 1H, m), 7.14-7.43 (cis of 6H, trans of 6H, m).

### Referential Example 308

2-[8-Chloro-6-(2,3-dimethoxyphenyl)-4H,6H-imidazo[1,2-a] [4,1]benzoxazepin-4-yl]-1-ethanol

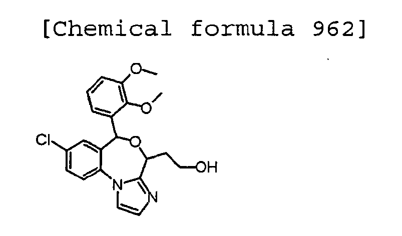

8-Chloro-6-(2,3-dimethoxyphenyl)-4-(1,3-dioxolan-2-ylmethyl)-4H,6H-imidazo[1,2-a][4,1]benzoxazepine as a cis-trans mixture (cis:trans= 1:4, 9 mg) was dissolved in dichloromethane (2 mL). To the resulting solution was added a 30% aqueous perchloric acid solution (2 mL) and the resulting mixture was vigorously stirred overnight at room temperature. The organic layer was washed with a saturated aqueous solution of sodium hydrogen carbonate and dried over anhydrous sodium sulfate. The solvent was distilled off under reduced pressure. The residue was dissolved in tetrahydrofuran (3 mL). To the resulting solution were added water (0.5 mL) and sodium borohydride (3 mg). The resulting mixture was stirred at room temperature for one hour. To the reaction mixture were added ethyl acetate and water and the layers separated. The organic layer was dried over anhydrous sodium sulfate. The solvent was distilled off under reduced pressure. The residue was purified by preparative thin layer chromatography on silica gel (hexane:ethyl acetate = 1:3) to give the title compound as a cis-trans mixture (cis:trans= 1:4, 4 mg).
MS (FAB) m/z: 401 (M+H)⁺.
¹H-NMR (CDCl₃) δ: 2.26-2.35 (cis of 2H, m), 2.37-2.53 (trans of 2H, m), 3.50 (trans of 3H, s), 3.58 (cis of 3H, s), 3.76-3.91 (trans of 4H, cis of 5H, m), 3.97-4.05 (trans of 1H, m), 4.68 (trans of 1H, t, J = 5.7 Hz), 5.19 (cis of 1H, t, J = 6.2 Hz), 5.68 (trans of 1H, s), 6.13 (cis of 1H, s), 6.77-6.81 (trans of 1H, m), 6.86 (cis of 1H, d, J = 8.3 Hz), 6.95-7.04 (trans of 1H, cis of 1H, m), 7.10-7.45 (trans of 6H, cis of 6H, m).

### Referential Example 309

3-[8-Chloro-6-(2,3-dimethoxyphenyl)-4H,6H-imidazo[1,2-a][4,1]benzoxazepin-4-yl]propanenitrile

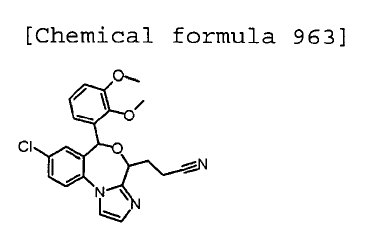

2-[8-Chloro-6-(2,3-dimethoxyphenyl)-4H,6H-imidazo[1,2-a][4,1]benzoxazepin-4-yl]-1-ethanol as a cis-trans mixture (cis:trans= 1:4, 12 mg) was dissolved in dichloromethane (2 mL). To the resulting solution were added triethylamine (12 µl) and chloromethanesulfonate (3.5 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure. The residue was dissolved in dimethylsulfoxide (1 mL). To the resulting solution was added sodium cyanide (7.3 mg). The resulting mixture was stirred under heat at 40°C overnight. Ethyl acetate and water were added to the reaction mixture and the layers separated. The organic layer was dried over anhydrous sodium sulfate. The solvent was distilled off under reduced pressure. The residue was purified by preparative thin layer chromatography on silica gel (hexane:ethyl acetate = 1:3) to give the title compound as a cis-trans mixture (cis:trans= 1:4, 11 mg).
MS (FAB) m/z: 410 (M+H)⁺.
¹H-NMR (CDCl₃) δ: 2.26-2.44 (cis of 2H, m), 2.51-2.65 (majo-2H, cis of 2H, m), 2.68-2.84 (trans of 2H, m), 3.48 (trans of 3H, s), 3.60 (cis of 3H, s), 3.85 (cis of 3H, s), 3.87 (trans of 3H, s), 4.60 (trans of 1H, dd, J = 7.3, 5.6 Hz), 5.19 (cis of 1H, dd, J = 7.7, 5.0 Hz), 5.68 (trans of 1H, s), 6.12 (cis of 1H, s), 6.78-6.92 (trans of 1H, cis of 1H, m), 6.96-7.09 (trans of 1H, cis of 1H, m), 7.17-7.47 (trans of 6H, cis of 6H, m).

### Example 470

3-[8-Chloro-6-(2,3-dimethoxyphenyl)-4H,6H-imidazo[1,2-a] [4,1]benzoxazepin-4-yl]propanoic acid

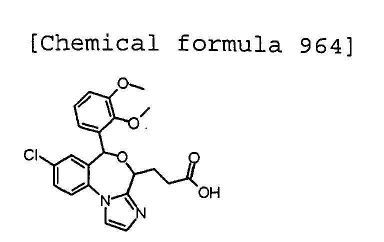

3-[8-Chloro-6-(2,3-dimethoxyphenyl)-4H,6H-imidazo[1,2-a][4,1]benzoxazepin-4-yl]propanenitrile as a cis-trans mixture (cis:trans= 1:4, 82 mg) was dissolved in isopropanol (2 mL). To the resulting solution was added a 5N aqueous sodium hydroxide solution (2.0 mL). The resulting mixture was heated under reflux for 2 days.
After concentration of the reaction mixture under reduced pressure, 1N hydrochloric acid (11 mL) was added to the residue, followed by extraction three times with chloroform. The organic layer was dried over anhydrous sodium sulfate. The solvent was distilled off under reduced pressure to give the title compound as a cis-trans mixture (cis:trans= 1:4, 90 mg).
MS (FAB) m/z: 429 (M+H)⁺.
¹H-NMR (CDCl₃) δ: 2.31-2.40 (cis of 2H, m), 2.43-2.67 (trans of 3H, cis of 2H, m), 2.74-2.83 (trans of 1H, m), 3.50 (trans of 3H, s), 3.60 (cis of 3H, s), 3.84 (cis of 3H, s), 3.87 (trans of 3H, s), 4.56 (trans of 1H, dd, J = 6.3, 4.3 Hz), 5.16 (cis of 1H, d, J = 6.3 Hz), 5.67 (trans of 1H, s), 6.10 (cis of 1H, s), 6.80-6.90 (trans of 1H, cis of 1H, m), 6.95-7.06 (trans of 1H, cis of 1H, m), 7.18-7.47 (trans of 6H, cis of 6H, m).
Elemental analysis for: C₂₂H₂₁ClN₂O₅·0.75H₂O·0.3CHCl₃ Calculated: C, 56.34; H, 4.77; N, 5.81.
Found: C, 56.22; H, 4.64; N, 5.63.

### Example 471

Ethyl 2-(1-{3-[8-chloro-6-(2,3-dimethoxyphenyl)-4H,6H-imidazo[1,2-a][4,1]benzoxazepin-4-yl]propanoyl}-4-piperidinyl)acetate

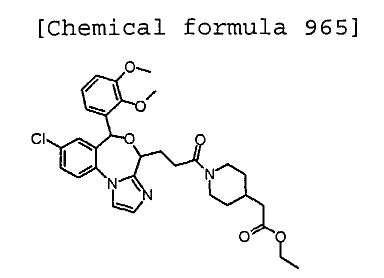

3-[8-Chloro-6-(2,3-dimethoxyphenyl)-4H,6H-imidazo[1,2-a][4,1]benzoxazepin-4-yl]propanoic acid as a cis-trans mixture (cis:trans= 1:4, 35 mg) was dissolved in dichloromethane (1 mL). To the resulting solution were added 1-ethyl-3-(3-dimethylaminopropyl)carbodiimide hydrochloride (47 mg), ethyl piperazineacetate (42 mg), and 1-hydroxybenzotriazole monohydrate (13 mg). The resulting mixture was stirred overnight at room temperature. To the reaction mixture were added a 0.005N hydrochloric acid (20 mL) and dichloromethane (20 mL) and the layers separated. The water layer was extracted twice with dichloromethane. The organic layers were combined and dried over anhydrous sodium sulfate. The solvent was distilled off under reduced pressure. The residue was purified by preparative thin layer chromatography on silica gel (hexane:ethyl acetate = 1:10) to give the title compound as a cis-trans mixture (cis:trans= 1:4, 30 mg).
MS (FAB) m/z: 582 (M+H)⁺.
¹H-NMR (CDCl₃) δ: 1.02-1.36 (trans of 5H, cis of 5H, m), 1.64-1.78 (trans of 3H, trans of 3H, m), 1.92-2.10 (trans of 1H, cis of 1H, m), 2.19-2.30 (trans of 2H, cis of 2H, m), 2.44-2.70 (trans of 4H, cis of 4H, m), 2.91-3.08 (trans of 1H, cis of 1H, m), 3.48 (trans of 3H, s), 3.57 (cis of 3H, s), 3.84-4.02 (trans of 4H, cis of 4H, m), 4.10-4.23 (trans of 2H, cis of 2H, m), 4.53-4.64 (trans of 2H, cis of 1H, m), 5.12-5.28 (cis of 1H, m), 5.65 (trans of 1H, s), 6.07 (cis of 1H, s), 6.75-6.77 (trans of 1H, m), 6.86-7.13 (trans of 1H, cis of 2H, m), 7.15-7.48 (trans of 6H, cis of 6H, m).

### Example 472

Methyl 3-(1-{3-[8-chloro-6-(2,3-dimethoxyphenyl)-4H,6H-imidazo[1,2-a][4,1]benzoxazepin-4-yl]propanoyl}-4-piperidinyl)propanoate

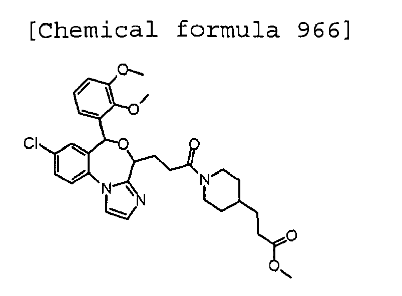

3-[8-Chloro-6-(2,3-dimethoxyphenyl)-4H,6H-imidazo[1,2-a][4,1]benzoxazepin-4-yl]propanoic acid as a cis-trans mixture (cis:trans= 1:4, 35 mg) was dissolved in N,N-dimethylformamide (1 mL). To the resulting solution were added 1-ethyl-3-(3-dimethylaminopropyl)carbodiimide hydrochloride (55 mg), methyl piperazinepropionate hydrochloride (25 mg), 1-hydroxybenzotriazole monohydrate(12 mg), and triethylamine (23 µl). The resulting mixture was stirred overnight at room temperature. After concentration of the reaction mixture under reduced pressure, 0.005N hydrochloric acid (20 mL) and dichloromethane (20 mL) were added to the residue and the layers separated. The water layer was extracted twice with dichloromethane. The organic layers were combined and dried over anhydrous sodium sulfate. The solvent was distilled off under reduced pressure. The residue was purified by preparative thin layer chromatography on silica gel (hexane:ethyl acetate = 1:10) to give the title compound as a cis-trans mixture (cis:trans= 1:4, 36 mg). MS (FAB) m/z: 582 (M+H)⁺.
¹H-NMR (CDCl₃) δ: 0.97-1.12 (trans of 2H, cis of 2H, m), 1.41-1.51 (trans of 1H, cis of 1H, m), 1.54-1.75 (trans of 4H, cis of 4H, m), 2.30-2.36 (trans of 3H, cis of 3H, m), 2.42-2.53 (trans of 2H, cis of 2H, m), 2.56-2.72 (trans of 2H, cis of 2H, m), 2.85-2.99 (trans of 1H, cis of 1H, m), 3.48 (trans of 3H, s), 3.57 (cis of 3H, s), 3.67 (trans of 3H, cis of 3H, s), 3.83-3.99 (trans of 4H, cis of 4H, m), 4.51-4.60 (trans of 2H, cis of 1H, m), 5.14-5.18 (cis of 1H, m), 5.65 (trans of 1H, s), 6.07 (cis of 1H, s), 6.76 (trans of 1H, d, J = 2.1 Hz), 6.87-7.09 (trans of 1H, cis of 2H, m), 7.15-7.42 (trans of 6H, cis of 6H, m).

### Example 473

2-(1-{3-[8-Chloro-6-(2,3-dimethoxyphenyl)-4H,6H-imidazo[1,2-a] [4,1]benzoxazepin-4-yl]propanoyl}-4-piperidinyl)acetic acid

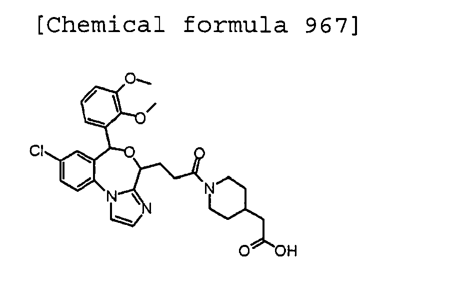

Ethyl 2-(1-{3-[8-chloro-6-(2,3-dimethoxyphenyl)-4H,6H-imidazo[1,2-a] [4,1]benzoxazepin-4-yl]propanoyl}-4-piperidinyl)acetate as a cis-trans mixture (cis:trans= 1:4, 30 mg) was dissolved in methanol (8 mL). To the resulting solution were added water (1 mL) and anhydrous potassium carbonate (21 mg). The resulting mixture was stirred under heat at 65°C for 2 hours. After concentration of the reaction mixture under reduced pressure, chloroform and 0.1N hydrochloric acid (3.3 mL) were added to the residue and the layers separated. The water layer was extracted twice with chloroform. The organic layers were combined and dried over anhydrous sodium sulfate. The solvent was distilled off under reduced pressure to give the title compound as a cis-trans mixture (cis:trans= 1:4, 30 mg).
MS (FAB) m/z: 554 (M+H)⁺.
¹H-NMR (CDCl₃) δ: 1.07-1.31 (trans of 3H, monor-3H, m), 1.68-1.86 (trans of 2H, cis of 2H, m), 1.95-2.08 (trans of 1H, cis of 1H, m), 2.19-2.36 (trans of 2H, cis of 2H, m), 2.44-2.78 (trans of 4H, cis of 4H, m), 2.93-3.07 (trans of 1H, cis of 1H, m), 3.48 (trans of 3H, s), 3.57 (cis of 1H, s), 3.84-3.95 (trans of 4H, cis of 4H, m), 4.50-4.52 (trans of 2H, cis of 1H, m), 5.20-5.26 (cis of 1H, m), 5.64 (trans of 1H, s), 6.03-6.05 (cis of 1H, m), 6.76 (trans of 1H, s), 6.88-7.11 (trans of 1H, cis of 2H, m), 7.16-7.45 (trans of 6H, cis of 6H, m).
Elemental analysis for: C₂₉H₃₂ClN₃O₆·0.4H₂O·0.2CHCl₃ Calculated: C, 60.10; H, 5.66; N, 7.15.
Found: C, 60.52; H, 5.95; N, 6.69.

### Example 474

3-(1-{3-[8-Chloro-6-(2,3-dimethoxyphenyl)-4H,6H-imidazo[1,2-a][4,1]benzoxazepin-4-yl]propanoyl}-4-piperidinyl)propanoic acid

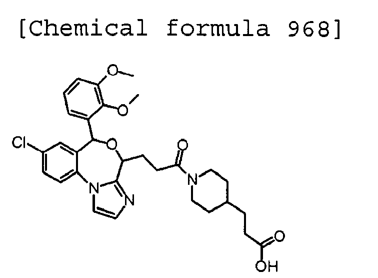

Methyl 3-(1-{3-[8-chloro-6-(2,3-dimethoxyphenyl)-4H,6H-imidazo[1,2-a][4,1]benzoxazepin-4-yl]propanoyl}-4-piperidinyl)propanoate as a cis-trans mixture (cis:trans= 1:4, 36 mg) was dissolved in methanol (8 mL). To the resulting solution were added water (1 mL) and anhydrous potassium carbonate (25 mg). The resulting mixture was stirred under heat at 65°C for 2 hours. After concentration of the reaction mixture under reduced pressure, chloroform and 0.1N hydrochloric acid (4 mL) were added to the residue and the layers separated. The water layer was extracted twice with chloroform. The organic layers were combined and dried over anhydrous sodium sulfate. The solvent was distilled off under reduced pressure to give the title compound as a cis-trans mixture (cis:trans= 1:4, 36 mg).
MS (FAB) m/z: 568 (M+H)⁺.
¹H-NMR (CDCl₃) δ: 0.82-1.18 (trans of 2H, cis of 2H, m), 1.47-1.75 (trans of 5H, cis of 5H, m), 2.19-2.39 (trans of 3H, cis of 3H, m), 2.43-2.73 (trans of 4H, cis of 4H, m), 2.87-3.01 (trans of 1H, cis of 1H, m), 3.48 (trans of 3H, s), 3.58 (cis of 3H, s), 3.84-3.97 (trans of 4H, cis of 4H, m), 4.51-4.62 (trans of 2H, cis of 1H, m), 5.19-5.24 (cis of 1H, m), 5.65 (trans of 1H, s), 6.04 (cis of 1H, s), 6.76 (trans of 1H, s), 6.88-7.11 (trans of 1H, cis of 2H, m), 7.15-7.44 (trans of 6H, cis of 6H, m).
Elemental analysis for: C₃₁H₂₉ClN₂O₄·0.4H₂O·0.3CHCl₃
Calculated: C, 59.79; H, 5.76; N, 6.84.
Found: C, 60.19; H, 6.04; N, 6.40.

### Referential Example 310

(1-{2-[(4R,6S)-8-Chloro-6-(2,3-dimethoxyphenyl)-4H,6H-imidazo[1,2-a][4,1]benzoxazepin-4-yl]ethyl}-1H-pyrazol-4-yl)methanol

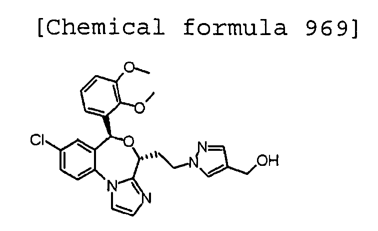

Ethyl 1-{2-[(4R,6S)-8-chloro-6-(2,3-dimethoxyphenyl)-4H,6H-imidazo[1,2-a] [4,1]benzoxazepin-4-yl]ethyl}-1H-pyrazole-4-carboxylate (390 mg) was dissolved in tetrahydrofuran (50 mL). To the resulting solution was added lithium borohydride (405 mg). The resulting mixture was heated under reflux for 3 hours. To the reaction mixture was added ethyl acetate and the layers separated. The organic layer was dried over anhydrous sodium sulfate. The solvent was distilled off under reduced pressure. The residue was purified by preparative thin layer silica gel column chromatography (hexane:ethyl acetate = 1:10) to give the title compound (151 mg).
MS (FAB) m/z: 481 (M+H)⁺.
¹H-NMR (CDCl₃) δ: 2.66-2.85 (2H, m), 3.48 (3H, s), 3.87 (3H, s), 4.38-4.55 (5H, m), 5.68 (1H, s), 6.77 (1H, d, J = 2.3 Hz), 6.98 (1H, dd, J = 8.1, 1.5 Hz), 7.17-7.44 (6H, m).

### Referential Example 311

2-(1-{2-[(4R,6S)-8-Chloro-6-(2,3-dimethoxyphenyl)-4H,6H-imidazo[1,2-a] [4,1]benzoxazepin-4-yl]ethyl}-1H-pyrazol-4-yl)acetonitrile

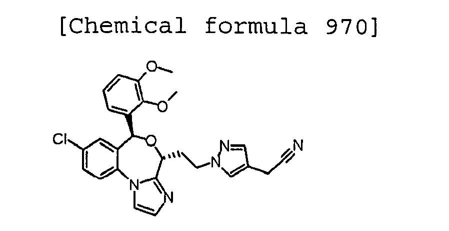

(1-{2-[(4R,6S)-8-Chloro-6-(2,3-dimethoxyphenyl)-4H,6H-imidazo[1,2-a][4,1]benzoxazepin-4-yl]ethyl}-1H-pyrazol-4-yl)methanol (113 mg) was dissolved in dichloromethane (2 mL). To the resulting solution were added carbon tetrabromide (94 mg) and triphenylphosphine (136 mg) successively. The resulting mixture was stirred at room temperature for 2 hours. After concentration of the reaction mixture under reduced pressure, the residue was dissolved in dimethylsulfoxide (4 mL). To the resulting solution was added sodium cyanide (58 mg) and the resulting mixture was stirred at 50°C for 1 hour. Ethyl acetate and water were added to the reaction mixture and the layers separated. The organic layer was dried over anhydrous sodium sulfate. The solvent was distilled off under reduced pressure. The residue was purified by preparative thin layer silica gel column chromatography (hexane:ethyl acetate = 1:3) to give the title compound (60 mg) .
MS (FAB) m/z: 490 (M+H)⁺.
¹H-NMR (CDCl₃) δ: 2.67-2.85 (3H, m), 3.48 (3H, s), 3.87 (3H, s), 4.39-4.53 (3H, m), 5.68 (1H, s), 6.78 (1H, d, J = 2.2 Hz), 6.98 (1H, dd, J = 8.3, 1.5 Hz), 7.22 (1H, t, J = 8.1 Hz), 7.25-7.32 (2H, m), 7.34-7.44 (5H, m).

### Example 475

2-(1-{2-[(4R,6S)-8-Chloro-6-(2,3-dimethoxyphenyl)-4H,6H-imidazo[1,2-a][4,1]benzoxazepin-4-yl]ethyl}-1H-pyrazol-4-yl)acetic acid

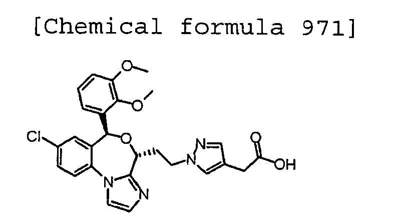

2-(1-{2-[(4R,6S)-8-Chloro-6-(2,3-dimethoxyphenyl)-4H,6H-imidazo[1,2-a][4,1]benzoxazepin-4-yl]ethyl}-1H-pyrazol-4-yl)acetonitrile (58 mg) was dissolved in isopropanol (1 mL). To the resulting solution were added a 35% aqueous sodium hydroxide solution (1.0 mL) and methanol (1 mL). The resulting mixture was heated under reflux for 2 hours. After concentration of the reaction mixture under reduced pressure, 3N hydrochloric acid (3.2 mL) was added to the residue. After confirmation that the resulting mixture became from pH 3 to 4, it was extracted three times with chloroform. The organic layer was dried over anhydrous sodium sulfate. The solvent was then distilled off under reduced pressure to give the title compound (52 mg).
MS (FAB) m/z: 509 (M+H)⁺.
¹H-NMR (CDCl₃) δ: 2.69-2.84 (2H, m), 3.48 (5H, s), 3.87 (3H, s), 4.34-4.50 (3H, m), 5.66 (1H, s), 6.78 (1H, d, J = 2.2 Hz), 6.97 (1H, dd, J = 8.3, 1.3 Hz),

### Referential Example 312

[5-Chloro-2-(4,5-dimethyl-1H-imidazol-1-yl)phenyl](2,3-dimethoxyphenyl)methanone

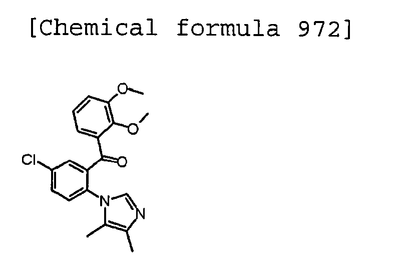

4,5-Dimethylimidazole (39 mg) synthesized in accordance with the process known by the document (J. Heterocyclic Chem., 28, 1819-1820(1991)) was dissolved in dimethylsulfoxide (3 mL). To the resulting solution was added sodium hydride (60% in oil, 16 mg) at room temperature and the mixture was stirred for one hour. To the reaction mixture was added (5-chloro-2-fluorophenyl)(2,3-dimethoxyphenyl)methanone (100 mg). The resulting mixture was stirred under heat at 95°C overnight. After cooling to room temperature, ethyl acetate was added to the reaction mixture and the layers separated. The organic layer was dried over anhydrous sodium sulfate. The solvent was distilled off under reduced pressure. The residue was purified by preparative thin layer silica gel column chromatography (hexane:ethyl acetate = 1:1) to give the title compound (112 mg).
MS (FAB) m/z: 371 (M+H)⁺.
¹H-NMR (CDCl₃) δ: 1.89 (3H, s), 1.99 (3H, s), 3.52 (3H, s), 3.84 (3H, s), 6.88-6.93 (1H, m), 6.96-7.00 (2H, m), 7.17 (1H, d, J = 8.3 Hz), 7.20 (1H, s), 7.55 (1H, dd, J = 8.3, 2.4 Hz), 7.62 (1H, d, J = 2.4 Hz).

### Referential Example 313

{5-Chloro-2-[2-(hydroxymethyl)-4,5-dimethyl-1H-imidazol-1-yl]phenyl}(2,3-dimethoxyphenyl)methanone

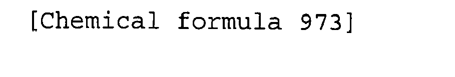

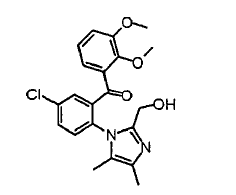

[5-Chloro-2-(4,5-dimethyl-1H-imidazol-1-yl)phenyl](2,3-dimethoxyphenyl)methanone (10.0 g) was suspended in xylene (200 mL). To the resulting suspension was added paraformaldehyde (23.0 g). The resulting mixture was stirred overnight under heat at 135°C in a sealed glass tube. After cooling to room temperature, the reaction mixture was concentrated under reduced pressure. The residue was purified by silica gel column chromatography chloroform:methanol = from 100:1 to 25:1) to give the title compound (6.47 g).
MS (ESI) m/z: 401 (M+H)⁺.
¹H-NMR (CDCl₃) δ: 1.80 (3H, s), 1.97 (3H, s), 3.59 (3H, s), 3.84 (3H, s), 4.25 (1H, d, J = 13.5 Hz), 4.36 (1H, d, J = 13.5 Hz), 6.81-6.86 (1H, m), 6.94-7.01 (2H, m), 7.28 (1H, d, J = 8.3 Hz), 7.59 (1H, dd, J = 8.3, 2.2 Hz), 7.66 (1H, d, J = 2.2 Hz).

### Referential Example 314

1-[4-Chloro-2-(2,3-dimethoxybenzoyl)phenyl]-4,5-dimethyl-1H-imidazol-2-carbaldehyde

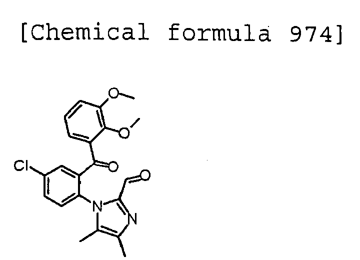

{5-Chloro-2-[2-(hydroxymethyl)-4,5-dimethyl-1H-imidazol-1-yl]phenyl}(2,3-dimethoxyphenyl)methanone (6.47 g) was dissolved in dichloromethane (150 mL). To the resulting solution was added manganese dioxide (14.0 g). The resulting mixture was stirred overnight under heat at 40°C. To the reaction mixture was added tetrahydrofuran (200 mL). The resulting mixture was filtered through a Kiriyama funnel. The filtrate was concentrated under reduced pressure to give the title compound (5.66 g).
MS (FAB) m/z: 399 (M+H)⁺.
¹H-NMR (CDCl₃) δ: 1.99 (3H, s), 2.18 (3H, s), 3.69 (3H, s), 3.86 (3H, s), 6.77 (1H, dd, J = 6.7, 2.3 Hz), 6.94-7.02 (2H, m), 7.17 (1H, d, J = 8.3 Hz), 7.59 (1H, dd, J = 8.3, 2.3 Hz), 7.65 (1H, d, J = 2.3 Hz), 9.44 (1H, s).

### Referential Example 315

(5-Chloro-2-{2-[2-(1,3-dioxolan-2-yl)-1-hydroxyethyl]-4,5-dimethyl-1H-imidazol-1-yl}phenyl)(2,3-dimethoxyphenyl)methanone

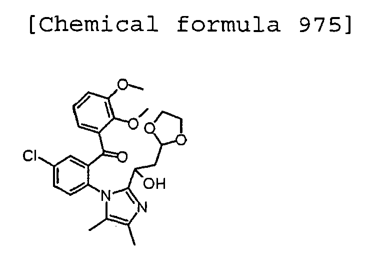

1-[4-Chloro-2-(2,3-dimethoxybenzoyl)phenyl]-4,5-dimethyl-1H-imidazol-2-carbaldehyde (1.00 g) was dissolved in tetrahydrofuran (25 mL). To the resulting solution was added (1,3-dioxolan-2-ylmethyl)magnesium bromide (0.5 M tetrahydrofuran solution, 6.53 mL). The resulting mixture was heated under reflux for 30 minutes in a nitrogen gas stream. After cooling to a temperature of ice cooling, water and ethyl acetate were added to the reaction mixture and the layers separated. The organic layer was washed with saturated brine and then, dried over anhydrous sodium sulfate. The solvent was distilled off under reduced pressure. The residue was purified by silica gel column chromatography (chloroform:methanol = 100 : 1) to give the title compound (1.01 g).
MS (FAB) m/z: 486 (M+H)⁺.
¹H-NMR (CDCl₃) δ: 1.78-1.81 (3H, m), 1.98-2.00 (3H, m), 2.00-2.12 (1H, m), 2.26-2.37 (1H, m), 3.57-3.62 (3H, m), 3.79-3.97 (7H, m), 4.55-4.62 (1H, m), 4.95-5.11 (1H, m), 6.73-7.46 (4H, m), 7.52-7.69 (2H, m).

### Referential Example 316

trans-8-Chloro-6-(2,3-dimethoxyphenyl)-4-(1,3-dioxolan-2-ylmethyl)-1,2-dimethyl-4H,6H-imidazo[1,2-a][4,1]benzoxazepine

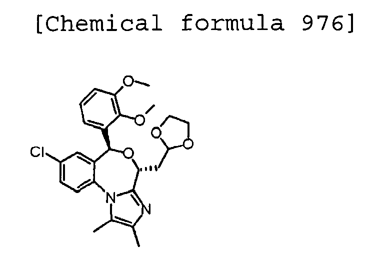

(5-Chloro-2-{2-[2-(1,3-dioxolan-2-yl)-1-hydroxyethyl]-4,5-dimethyl-1H-imidazol-1-yl}phenyl)(2,3-dimethoxyphenyl)methanone (779 mg) was dissolved in ethanol (20 mL). To the resulting solution was added sodium borohydride (121 mg). The resulting mixture was stirred at room temperature for 2 hours. After the reaction mixture was concentrated under reduced pressure, ethyl acetate and water were added to the residue and the layers separated. The organic layer was dried over anhydrous sodium sulfate. The solvent was distilled off under reduced pressure. The residue was dissolved in dichloroethane (160 mL). In a cooling bath containing methanol-ice, triethylamine (1.04 mL) and chloromethanesulfonate (186 µl) were added successively to the resulting solution and the mixture was stirred overnight at room temperature. To the reaction mixture was added a 1N aqueous sodium hydroxide solution (20 mL) and the layers separated. The organic layer was dried over anhydrous sodium sulfate. The solvent was distilled off under reduced pressure. The residue was purified by silica gel column chromatography (hexane:ethyl acetate = from 1:1 to 2:3) to give the title compound (223 mg) .
MS (FAB) m/z: 471 (M+H)⁺.
¹H-NMR (CDCl₃) δ: 2.28 (3H, s), 2.29 (3H, s), 2.45-2.62 (2H, m), 3.44 (3H, s), 3.83-3.98 (7H, m), 4.48-4.53 (1H, m), 5.28-5.32 (1H, m), 5.52 (1H, s), 6.76-6.78 (1H, m), 6.94 (1H, d, J = 8.1 Hz), 7.17 (1H, t, J = 8.1 Hz), 7.23 (1H, d, J = 8.5 Hz), 7.25-7.27 (1H, m), 7.29 (1H, d, J = 7.6 Hz), 7.37-7.42 (1H, m).

### Referential Example 317

2-[trans-8-Chloro-6-(2,3-dimethoxyphenyl)-1,2-dimethyl-4H,6H-imidazo[1,2-a][4,1]benzoxazepin-4-yl]-1-ethanol

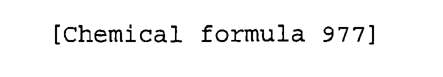

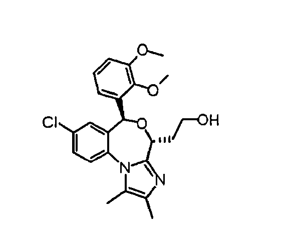

trans-8-Chloro-6-(2,3-dimethoxyphenyl)-4-(1,3-dioxolan-2-ylmethyl)-1,2-dimethyl-4H,6H-imidazo[1,2-a][4,1]benzoxazepine (250 mg) was dissolved in dichloromethane (20 mL). To the resulting solution was added a 30% aqueous perchloric acid solution (20 mL). The resulting mixture was vigorously stirred overnight at room temperature. The organic layer was washed with a saturated aqueous solution of sodium hydrogen carbonate and dried over anhydrous sodium sulfate. The solvent was distilled off under reduced pressure. The residue was dissolved in tetrahydrofuran (20 mL). To the resulting solution were added water (5 mL) and sodium borohydride (80 mg) and the resulting mixture was stirred at room temperature for one hour. Ethyl acetate and water were added to the reaction mixture and the layers separated. The organic layer was dried over anhydrous sodium sulfate. The solvent was distilled off under reduced pressure. The residue was purified by silica gel column chromatography (hexane:ethyl acetate = from 1:1 to 1:3) to give the title compound (130 mg).
MS (FAB) m/z: 429 (M+H)⁺.
¹H-NMR (CDCl₃) δ: 2.28 (3H, s), 2.28 (3H, s), 2.30-2.48 (2H, m), 3.46 (3H, s), 3.69-3.78 (1H, m), 3.86 (3H, s), 3.97-4.04 (1H, m), 4.50 (1H, t, J = 5.6 Hz), 5.54 (1H, s), 6.80 (1H, d, J = 2.2 Hz), 6.96 (1H, dd, J = 8.3, 1.5 Hz), 7.18 (1H, t, J = 8.1 Hz), 7.23-7.28 (2H, m), 7.42 (1H, dd, J = 8.3, 2.2 Hz).

### Referential Example 318

3-[trans-8-Chloro-6-(2,3-dimethoxyphenyl)-1,2-dimethyl-4H,6H-imidazo[1,2-a][4,1]benzoxazepin-4-yl]propanenitrile

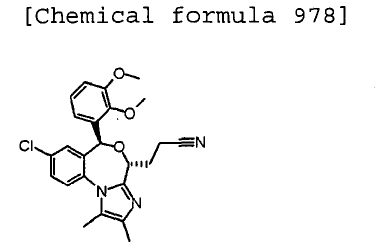

2-[trans-8-Chloro-6-(2,3-dimethoxyphenyl)-1,2-dimethyl-4H,6H-imidazo[1,2-a][4,1]benzoxazepin-4-yl]-1-ethanol (125 mg) was dissolved in dichloromethane (5 mL). To the resulting solution were added triethylamine (113 µl) and chloromethanesulfonate (34 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure. The residue was dissolved in dimethylsulfoxide (3 mL). To the resulting solution was added sodium cyanide (143 mg) and the resulting mixture was stirred under heat at 50°C for 2 hours. Ethyl acetate and water were added to the reaction mixture and the layers separated. The organic layer was dried over anhydrous sodium sulfate. The solvent was distilled off under reduced pressure. The residue was purified by preparative thin layer chromatography on silica gel (hexane:ethyl acetate = 1:3) to give the title compound (93 mg).
MS (FAB) m/z: 438 (M+H)⁺.
¹H-NMR (CDCl₃) δ: 2.29 (6H, s), 2.44-2.62 (2H, m), 2.66-2.80 (2H, m), 3.44 (3H, br s), 3.86 (3H, s), 4.42 (1H, dd, J = 7.8, 5.4 Hz), 5.55 (1H, s), 6.80 (1H, d, J = 2.4 Hz), 6.97 (1H, d, J = 8.0 Hz), 7.20 (1H, t, J = 8.0 Hz), 7.23-7.29 (2H, m), 7.43 (1H, dd, J = 8.3, 2.4 Hz).

### Example 476

3-[trans-8-Chloro-6-(2,3-dimethoxyphenyl)-1,2-dimethyl-4H,6H-imidazo[1,2-a][4,1]benzoxazepin-4-yl]propanoic acid

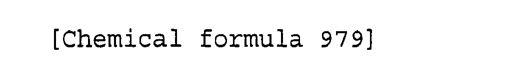

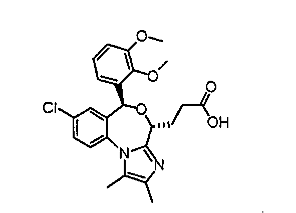

3-[trans-8-Chloro-6-(2,3-dimethoxyphenyl)-1,2-dimethyl-4H,6H-imidazo[1,2-a][4,1]benzoxazepin-4-yl]propanenitrile (388 mg) was dissolved in isopropanol (8 mL). To the resulting solution were added a 35% aqueous sodium hydroxide solution (8 mL) and methanol (8 mL). The resulting mixture was heated under reflux for 3 hours. After concentration of the reaction mixture under reduced pressure, 3N hydrochloric acid (28 mL) was added to the residue. After the pH of the resulting mixture became from 3 to 4, extraction was performed three times with chloroform. The organic layer was dried over anhydrous sodium sulfate. The solvent was distilled off under reduced pressure. To the residue was added a 20:1 hexane:diethyl ether solvent mixture and the solid thus precipitated was collected by filtration to give the title compound (349 mg).
MS (EI) m/z: 456 M⁺.
¹H-NMR (CDCl₃) δ: 2.31 (6H, s), 2.35-2.45 (2H, m), 2.48-2.62 (1H, m), 2.73-2.82 (1H, m), 3.46 (3H, s), 3.87 (3H, s), 4.38-4.43 (1H, m), 5.56 (1H, s), 6.83 (1H, d, J = 2.2 Hz), 6.98 (1H, dd, J = 8.2, 1.6 Hz), 7.19 (1H, t, J = 8.2 Hz), 7.24-7.30 (2H, m), 7.45 (1H, dd, J = 8.4, 2.3 Hz).
Elemental analysis for: C₂₄H₂₅ClN₂O₅·0.25H₂O
Calculated: C, 62.47; H, 5.57; N, 6.07.
Found: C, 62.55; H, 5.60; N, 5.68.

### Example 477

Ethyl 2-(1-{3-[(trans-8-chloro-6-(2,3-dimethoxyphenyl)-1,2-dimethyl-4H,6H-imidazo[1,2-a][4,1]benzoxazepin-4-yl)propanoyl]-4-piperidinyl}acetate

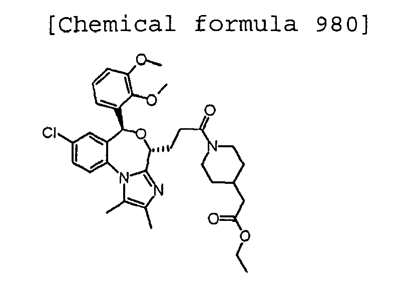

3-[trans-8-Chloro-6-(2,3-dimethoxyphenyl)-1,2-dimethyl-4H,6H-imidazo[1,2-a][4,1]benzoxazepin-4-yl]propanoic acid (35 mg) was dissolved in N,N-dimethylformamide (1 mL). To the resulting solution were added 1-ethyl-3-(3-dimethylaminopropyl)carbodiimide hydrochloride (29 mg), ethyl piperazineacetate (39 mg), and 1-hydroxybenzotriazole monohydrate (12 mg). The resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue was purified by preparative thin layer chromatography on silica gel (hexane:ethyl acetate = 1:10) to give the title compound (34 mg) .
MS (FAB) m/z: 610 (M+H)⁺.
¹H-NMR (CDCl₃) δ: 1.04-1.19 (2H, m), 1.25 (3H, t, J = 7.2 Hz), 1.66-1.80 (2H, m), 1.92-2.05 (1H, m), 2.19-2.23 (2H, m), 2.20-2.24 (6H, m), 2.42-2.70 (5H, m), 2.96-3.05 (1H, m), 3.43 (3H, s), 3.85 (3H, s), 3.93-4.03 (1H, m), 4.13 (2H, q, J = 7.2 Hz), 4.34-4.39 (1H, m), 4.52-4.59 (1H, m), 5.51 (1H, s), 6.76 (1H, d, J = 2.3 Hz), 6.95 (1H, d, J = 8.1 Hz), 7.17 (1H, t, J = 7.9 Hz), 7.23 (1H, d, J = 8.5 Hz), 7.25-7.30 (1H, m), 7.39 (1H, dd, J = 8.4, 2.3 Hz).

### Example 478

Ethyl 3-(1-{3-[trans-8-chloro-6-(2,3-dimethoxyphenyl)-1,2-dimethyl-4H,6H-imidazo[1,2-a][4,1]benzoxazepin-4-yl]propanoyl}-4-piperidinyl)propanoate

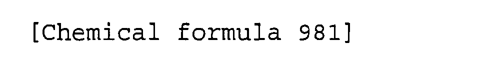

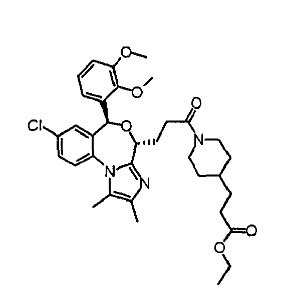

3-[trans-8-Chloro-6-(2,3-dimethoxyphenyl)-1,2-dimethyl-4H,6H-imidazo[1,2-a][4,1]benzoxazepin-4-yl]propanoic acid (35 mg) was dissolved in N,N-dimethylformamide (1 mL). To the resulting solution were added 1-ethyl-3-(3-dimethylaminopropyl)carbodiimide hydrochloride (58 mg), ethyl piperazinepropionate hydrochloride (102 mg), 1-hydroxybenzotriazole monohydrate (12 mg) and triethylamine (64 µl). The resulting mixture was stirred at room temperature for 2 days. After concentration of the reaction mixture under reduced pressure, ethyl acetate and water were added to the residue and the layers separated. The organic layer was dried over anhydrous sodium sulfate. The solvent was distilled off under reduced pressure. The residue was purified by preparative thin layer chromatography on silica gel (hexane:ethyl acetate = 1:10) to give the title compound (33 mg).
MS (FAB) m/z: 624 (M+H)⁺.
¹H-NMR (CDCl₃) δ: 0.97-1.12 (2H, m), 1.21-1.29 (4H, m), 1.37-1.74 (4H, m), 2.23-2.35 (9H, m), 2.37-2.70 (4H, m), 2.85-3.01 (1H, m), 3.44 (3H, s), 3.86 (3H, s), 3.93-4.03 (1H, m), 4.13 (2H, q, J = 7.2 Hz), 4.33-4.39 (1H, m), 4.51-4.59 (1H, m), 5.51 (1H, s), 6.76 (1H, d, J = 2.0 Hz), 6.95 (1H, d, J = 8.1 Hz), 7.17 (1H, t, J = 7.9 Hz), 7.21-7.30 (2H, m), 7.36-7.42 (1H, m).

### Example 479

2-(1-{3-[(trans-8-Chloro-6-(2,3-dimethoxyphenyl)-1,2-dimethyl-4H,6H-imidazo[1,2-a][4,1]benzoxazepin-4-yl)propanoyl]-4-piperidinyl}acetic acid

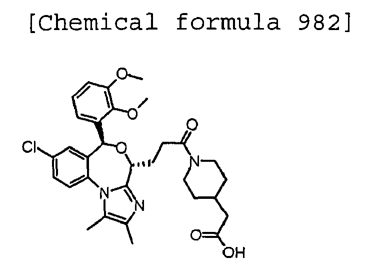

Ethyl 2-(1-{3-[(trans-8-chloro-6-(2,3-dimethoxyphenyl)-1,2-dimethyl-4H,6H-imidazo[1,2-a][4,1]benzoxazepin-4-yl)propanoyl]-4-piperidinyl}acetate (32 mg) was dissolved in methanol (0.5 mL). To the resulting solution were added water (0.5 mL) and anhydrous potassium carbonate (22 mg). The resulting mixture was stirred under heat at 50°C for 2 hours. After concentration of the reaction mixture under reduced pressure, chloroform and 0.1N hydrochloric acid (2 mL) were added to the residue. After confirmation that the pH of the water layer was from 6 to 8, the layers were separated. The water layer was extracted twice with chloroform. The organic layers were combined and dried over anhydrous sodium sulfate. The solvent was distilled off under reduced pressure to give the title compound (30 mg).
MS (FAB) m/z: 582 (M+H)⁺.
¹H-NMR (CDCl₃) δ: 0.99-1.21 (2H, m), 1.46-1.75 (4H, m), 2.24-2.39 (8H, m), 2.42-2.71 (4H, m), 2.87-3.02 (1H, m), 3.44 (3H, br s), 3.86 (3H, s), 3.94 (1H, d, J = 11.5 Hz), 4.32-4.39 (1H, m), 4.53-4.60 (1H, m), 5.50 (1H, d, J = 3.4 Hz), 6.75-6.78 (1H, m), 6.95 (1H, dd, J = 8.1, 1.5 Hz), 7.17 (1H, t, J = 8.1 Hz), 7.23 (1H, d, J = 8.1 Hz), 7.25-7.30 (2H, m), 7.40 (1H, dd, J = 8.4, 2.3 Hz) .
Elemental analysis for: C₃₁H₃₆C1N₃O₆·1.0H₂O·0.4CHCl₃ Calculated: C, 58.52; H, 5.93; N, 6.44.
Found: C, 58.27; H, 5.84; N, 6.10.

### Example 480

3-(1-{3-[trans-8-Chloro-6-(2,3-dimethoxyphenyl)-1,2-dimethyl-4H,6H-imidazo[1,2-a][4,1]benzoxazepin-4-yl]propanoyl}-4-piperidinyl)propanoic acid

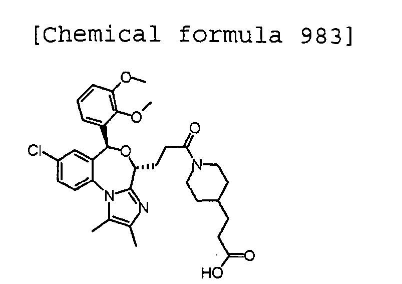

Ethyl 3-(1-{3-[trans-8-chloro-6-(2,3-dimethoxyphenyl)-1,2-dimethyl-4H,6H-imidazo[1,2-a][4,1]benzoxazepin-4-yl]propanoyl}-4-piperidinyl)propanoate (31 mg) was dissolved in methanol (1 mL). To the resulting solution were added water (1 mL) and anhydrous potassium carbonate (21 mg). The resulting mixture was stirred under heat at 50°C for 2 hours. After concentration of the reaction mixture under reduced pressure, chloroform and 0.1N hydrochloric acid (3 mL) were added to the residue. After confirmation that the pH of the water layer was from 6 to 8, the layers were separated. The water layer was extracted twice with chloroform. The organic layers were combined and dried over anhydrous sodium sulfate. The solvent was distilled off under reduced pressure to give the title compound (27 mg).
MS (FAB) m/z: 596 (M+H)⁺.
¹H-NMR (CDCl₃) δ: 1.06-1.22 (2H, m), 1.68-1.86 (2H, m), 1.94-2.06 (1H, m), 2.19-2.33 (10H, m), 2.49-2.65 (5H, m), 2.93-3.06 (1H, m), 3.44 (3H, s), 3.85 (3H, s), 3.88-3.97 (1H, m), 4.34-4.39 (1H, m), 4.52-4.59 (1H, m), 5.49 (1H, d, J = 6.1 Hz), 6.75-6.78 (1H, m), 6.95 (1H, d, J = 8.3 Hz), 7.17 (1H, t, J = 8.3 Hz), 7.23 (1H, dd, J = 8.8, 3.4 Hz), 7.26-7.32 (1H, m), 7.40 (1H, d, J = 8.8 Hz).
Elemental analysis for: C₃₂H₃₈ClN₃O₆·0.75H₂O·0.2CHCl₃
Calculated: C, 61.20; H, 6.29; N, 6.61.
Found: C, 61.60; H, 6.40; N, 6.20.

### Example 481

Ethyl 1-{2-[trans-8-chloro-6-(2,3-dimethoxyphenyl)-1,2-dimethyl-4H,6H-imidazo[1,2-a][4,1]benzoxazepin-4-yl]ethyl}-1H-pyrazole-4-carboxylate

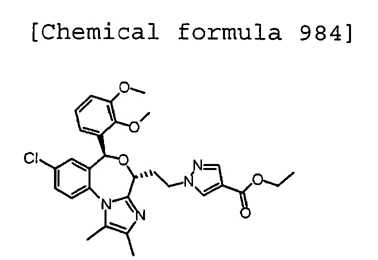

2-[trans-8-Chloro-6-(2,3-dimethoxyphenyl)-1,2-dimethyl-4H,6H-imidazo[1,2-a][4,1]benzoxazepin-4-yl]-1-ethanol (100 mg) was dissolved in dichloromethane (3 mL). To the resulting solution were added triethylamine (90 µl) and chloromethanesulfonate (27 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a crudely purified product of 2-[trans-8-chloro-6-(2,3-dimethoxyphenyl)-1,2-dimethyl-4H,6H-imidazo[1,2-a][4,1]benzoxazepin-4-yl]ethyl methanesulfonate. Ethyl 4-pyrazolecarboxylate (49 mg) was dissolved in N,N-dimethylformamide (3 mL). To the resulting solution was added sodium hydride (14 mg). The resulting mixture was stirred at room temperature for one hour. After addition of tetra-n-butylammonium iodide (86 mg) to the reaction mixture, the crudely purified product obtained above was added. The resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue was purified by preparative thin layer chromatography on silica gel (hexane:ethyl acetate = 1:3) to give the title compound (98 mg).
MS (FAB) m/z: 551 (M+H)⁺.
¹H-NMR (CDCl₃) δ: 1.32 (3H, t, J = 7.2 Hz), 2.28 (3H, s), 2.29 (3H, s), 2.76 (2H, q, J = 7.2 Hz), 3.44 (3H, s), 3.86 (3H, s), 4.21-4.29 (3H, m), 4.40-4.55 (2H, m), 5.54 (1H, s), 6.78 (1H, d, J = 2.2 Hz), 6.97 (1H, dd, J = 8.1, 1.5 Hz), 7.19 (1H, t, J = 8.1 Hz), 7.23 (1H, d, J = 8.1 Hz), 7.25-7.28 (1H, m), 7.40 (1H, dd, J = 8.4, 2.2 Hz), 7.83 (1H, s), 7.89 (1H, s).

### Example 482

1-{2-[trans-8-Chloro-6-(2,3-dimethoxyphenyl)-1,2-dimethyl-4H,6H-imidazo[1,2-a][4,1]benzoxazepin-4-yl]ethyl}-1H-pyrazole-4-carboxylic acid

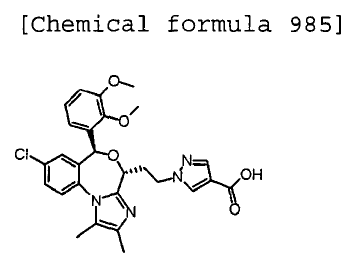

Ethyl 1-{2-[trans-8-chloro-6-(2,3-dimethoxyphenyl)-1,2-dimethyl-4H,6H-imidazo[1,2-a][4,1]benzoxazepin-4-yl]ethyl}-1H-pyrazole-4-carboxylate (95 mg) was dissolved in methanol (3 mL). To the resulting solution was added a 1N aqueous sodium hydroxide solution (862 µl). The resulting mixture was stirred under heat at 50°C for 2 hours. After concentration of the reaction mixture under reduced pressure, 1N hydrochloric acid (845 µl) was added to the residue. After confirmation that the pH of the resulting mixture became from 3 to 4, extraction with chloroform was performed. The organic layer was washed with water and then, dried over anhydrous sodium sulfate. The solvent was distilled off under reduced pressure to give the title compound (85 mg).
MS (FAB) m/z: 523 (M+H)⁺.
¹H-NMR (CDCl₃) δ: 2.30 (3H, s), 2.37 (3H, s), 2.77-2.86 (2H, m), 3.48 (3H, s), 3.86 (3H, s), 4.34-4.52 (3H, m), 5.56 (1H, s), 6.82 (1H, d, J = 2.2 Hz), 6.97 (1H, d, J = 8.1 Hz), 7.18 (1H, t, J = 8.1 Hz), 7.24-7.29 (2H, m), 7.45 (1H, dd, J = 8.4, 2.2 Hz), 7.88 (1H, s), 8.11 (1H, s). Elemental analysis for: C₂₇H₂₇ClN₄O₅·1.25H₂O·0.2CHCl₃ Calculated: C, 57.56; H, 5.24; N, 9.80.
Found: C, 57.55; H, 4.94; N, 9.52.

### Referential Example 319

[5-Chloro-2-(4,5-dichloro-1H-imidazol-1-yl)phenyl](2,3-dimethoxyphenyl)methanone

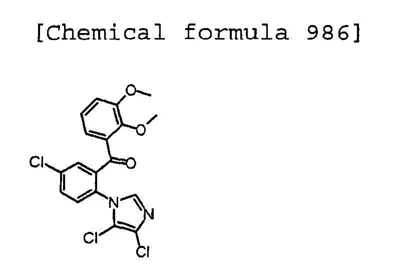

4,5-Dichloroimidazole (18.6 g) was dissolved in dimethylsulfoxide (300 mL). At room temperature, sodium hydride (60% in oil, 5.44 g) was added and the resulting mixture was stirred for one hour. To the reaction mixture was added (5-chloro-2-fluorophenyl)(2,3-dimethoxyphenyl) methanone (40.0 g). The resulting mixture was stirred under heat at 95°C for 24 hours. After cooling to room temperature, ethyl acetate was added to the reaction mixture and the layers separated. The organic layer was dried over anhydrous sodium sulfate. The solvent was distilled off under reduced pressure. The residue was purified by silica gel column chromatography hexane:ethyl acetate = from 6:1 to 1:1) to give the title compound (32.4 g).
MS (FAB) m/z: 411 (M+H)⁺.
¹H-NMR (CDCl₃) d: 3.54 (3H, s), 3.86 (3H, s), 6.99 (1H, dd, J = 6.8, 3.0 Hz), 7.01-7.09 (2H, m), 7.25-7.28 (2H, m), 7.36 (1H, s), 7.61 (1H, dd, J = 8.3, 2.4 Hz), 7.69 (1H, d, J = 2.4 Hz).

### Referential Example 320

4,5-Dichloro-1-[4-chloro-2-(2,3-dimethoxybenzoyl)phenyl]-1H-imidazol-2-carbaldehyde

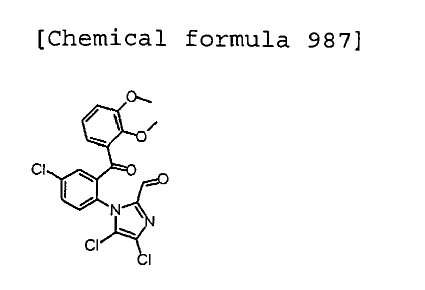

[5-Chloro-2-(4,5-dichloro-1H-imidazol-1-yl)phenyl](2,3-dimethoxyphenyl)methanone (32.3 g) was suspended in xylene (80 mL). To the resulting suspension was added paraformaldehyde (50.0 g). In a sealed glass tube, the resulting mixture was stirred under heat at 135°C for 2 days. After cooling to room temperature, the reaction mixture was concentrated under reduced pressure. The residue was purified by silica gel column chromatography (chloroform:methanol = from 100:1 to 25:1) to give a brown foam. The resulting foam was dissolved in dichloromethane (200 mL). To the resulting solution was added manganese dioxide (13.2 g). The resulting mixture was stirred under heat at 40°C for 4 hours. Ethyl acetate (200 mL) was added and the resulting mixture was filtered through a Kiriyama funnel. The filtrate was concentrated under reduced pressure. The residue was purified by silica gel column chromatography hexane:ethyl acetate = 8:1-6:1) to give the title compound (3.21 g).
MS (EI) m/z: 438 M⁺.
¹H-NMR (CDCl₃) d: 3.68 (3H, s), 3.88 (3H, s), 6.83 (1H, t, J = 4.6 Hz), 7.04 (2H, d, J = 4.6 Hz), 7.23-7.27 (1H, m), 7.65 (1H, dd, J = 8.5, 2.4 Hz), 7.68-7.70 (1H, m), 9.47 (1H, s).

### Referential Example 321

(5-Chloro-2-{4,5-dichloro-2-[2-(1,3-dioxolan-2-yl)-1-hydroxyethyl]-1H-imidazol-1-yl}phenyl)(2,3-dimethoxyphenyl)methanone

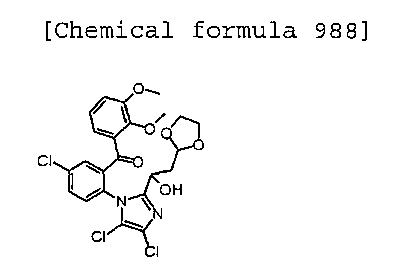

4,5-Dichloro-1-[4-chloro]-2-(2,3-dimethoxybenzoyl)phenyl]-1H-imidazol-2-carbaldehyde (1.63 g) was dissolved in tetrahydrofuran (80 mL). To the resulting solution was added (1,3-dioxolan-2-ylmethyl)magnesium bromide (0.5 M tetrahydrofuran solution, 9.64 mL). Under a nitrogen gas stream, the resulting mixture was heated under reflux for 7 hours. After cooling to a temperature of ice cooling, water and ethyl acetate were added to the reaction mixture and the layers separated. The organic layer was washed with saturated brine and then, dried over anhydrous sodium sulfate. The solvent was distilled off under reduced pressure. The residue was purified by silica gel column chromatography (hexane:ethyl acetate = 2:1) to give the title compound (1.63 g).
MS (FAB) m/z: 527 (M+H)⁺.
¹H-NMR (CDCl₃) d: 2.09-2.38 (2H, m), 3.59-3.65 (4H, m), 3.80-3.99 (7H, m), 4.63-4.73 (1H, m), 4.94-5.15 (1H, m), 6.88-7.12 (3H, m), 7.21-7.50 (2H, m), 7.60-7.66 (1H, m), 7.74-7.77 (1H, m).

### Referential Example 322

1,2,8-Trichloro-6-(2,3-dimethoxyphenyl)-4-(1,3-dioxolan-2-ylmethyl)-4H,6H-imidazo[1,2-a][4,1]benzoxazepine

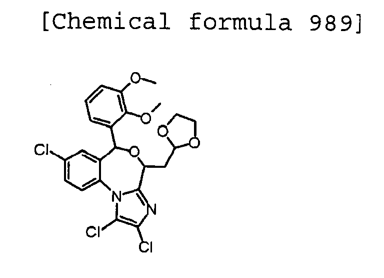

(5-Chloro-2-{4,5-dichloro-2-[2-(1,3-dioxolan-2-yl)-1-hydroxyethyl]-1H-imidazol-1-yl}phenyl)(2,3-dimethoxyphenyl)methanone (1.63 g) was dissolved in ethanol (50 mL). To the resulting solution was added sodium borohydride (349 mg). The resulting mixture was stirred at room temperature for 2 hours. After concentration of the reaction mixture under reduced pressure, ethyl acetate and water were added to the residue and the layers separated. The organic layer was dried over anhydrous sodium sulfate. The solvent was distilled off under reduced pressure. The residue was dissolved in dichloroethane (160 mL). In a cooling bath containing methanol-ice, triethylamine (1.54 mL) and methanesulfonyl chloride (349 ml) were added successively. The resulting mixture was stirred overnight at room temperature. To the reaction mixture was added a 1N aqueous sodium hydroxide solution (100 mL) and the layers separated. The organic layer was dried over anhydrous sodium sulfate. The solvent was distilled off under reduced pressure. The residue was purified by silica gel column chromatography (hexane:ethyl acetate = 6:1) to give the title compound as a trans-cis mixture (trans:cis=1:1, 578 mg).
MS (EI) m/z: 510 M⁺.
¹H-NMR (CDCl₃) d: 2.49-2.52 (2H, m), 3.42 (1.5H, br s), 3.48 (1.5H, s), 3.74-4.00 (7.5H, m), 4.52 (0.5H, dd, J = 7.3, 6.1 Hz), 5.06-5.21 (0.5H, m), 5.24 (0.5H, dd, J = 4.6, 5.8 Hz), 5.60 (0.5H, s), 6.06 (0.5H, br s), 6.75-7.03 (2H, m), 7.18 (0.5H, t, J = 8.1 Hz), 7.24-7.28 (0.5H, m), 7.31-7.52 (3H, m).

### Referential Example 323

2-[1,2,8-Trichloro-6-(2,3-dimethoxyphenyl)-4H,6H-imidazo[1,2-a][4,1]benzoxazepin-4-yl]-1-ethanol

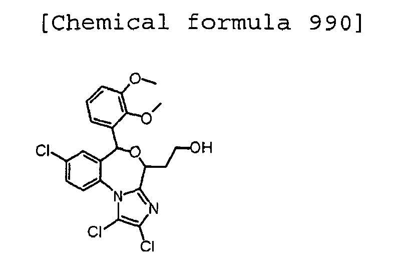

1,2,8-Trichloro-6-(2,3-dimethoxyphenyl)-4-(1,3-dioxolan-2-ylmethyl)-4H,6H-imidazo[1,2-a][4,1]benzoxazepine as a trans-cis mixture (trans:cis=1:1, 573 mg) was dissolved in dichloromethane (60 mL). To the resulting solution was added a 30% aqueous perchloric acid solution (60 mL). The resulting mixture was vigorously stirred overnight at room temperature. The organic layer was washed with a saturated aqueous solution of sodium hydrogen carbonate and dried over anhydrous sodium sulfate. The solvent was distilled off under reduced pressure. The residue was dissolved in tetrahydrofuran (20 mL). To the resulting solution were added water (5 mL) and sodium borohydride (127 mg). The resulting mixture was stirred at room temperature for one hour. Ethyl acetate and water were added to the reaction mixture and the layers separated. The organic layer was dried over anhydrous sodium sulfate. The solvent was distilled off under reduced pressure. The residue was purified by silica gel column chromatography (hexane:ethyl acetate = 4:1) to give the title compound as a trans-cis mixture (trans:cis=1:1, 404 mg).
MS (FAB) m/z: 468 (M+H)⁺.
¹H-NMR (CDCl₃) d: 2.12-2.57 (2H, m), 3.34-3.55 (3H, m), 3.75-3.98 (5H, m), 4.52-4.58 (0.5H, m), 4.64-4.97 (0.5H, m), 5.63 (0.5H, s), 6.09 (0.5H, br s), 6.56-7.06 (2H, m), 7.15-7.60 (4H, m).

### Example 483

Ethyl 1-{2-[1,2,8-trichloro-6-(2,3-dimethoxyphenyl)-4H,6H-imidazo[1,2-a][4,1]benzoxazepin-4-yl]ethyl}-1H-pyrazole-4-carboxylate

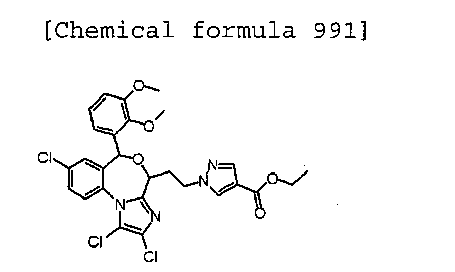

2-[1,2,8-Trichloro-6-(2,3-dimethoxyphenyl)-4H,6H-imidazo[1,2-a] [4,1]benzoxazepin-4-yl]-1-ethanol as a trans-cis mixture (trans:cis=1:1, 100 mg) was dissolved in dichloromethane (3 mL). To the resulting solution were added triethylamine (82 ml) and methanesulfonyl chloride (25 ml) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a crudely purified product of 2-[1,2,8-trichloro-6-(2,3-dimethoxyphenyl)-4H,6H-imidazo[1,2-a][4,1]benzoxazepin-4-yl]ethyl methanesulfonate. Ethyl 4-pyrazolecarboxylate (45 mg) was dissolved in N,N-dimethylformamide (3 mL). To the resulting solution was added sodium hydride (60% in oil, 13 mg). The resulting mixture was stirred at room temperature for one hour. After addition of tetra n-butylammonium iodide (79 mg), the crudely purified product obtained above was added and the resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue was purified by preparative thin layer chromatography on silica gel (hexane:ethyl acetate = 2:1) to give the title compound as a trans-cis mixture (trans:cis=1:1, 106 mg).
MS (FAB) m/z: 591 (M+H)⁺.
¹H-NMR (CDCl₃) d: 1.30-1.38 (3H, m), 2.39-2.79 (2H, m), 3.41 (1.5H, br s), 3.48 (1.5H, s), 3.80 (1.5H, br s), 3.87 (1.5H, s), 4.22-4.53 (5H, m), 5.62 (0.5H, s), 6.06 (0.5H, br s), 6.79-7.03 (2H, m), 7.17-7.52 (4H, m), 7.82-7.92 (2H, m) .

### Example 484

1-{2-[1,2,8-Trichloro-6-(2,3-dimethoxyphenyl)-4H,6H-imidazo[1,2-a] [4,1]benzoxazepin-4-yl]ethyl}-1H-pyrazole-4-carboxylic acid

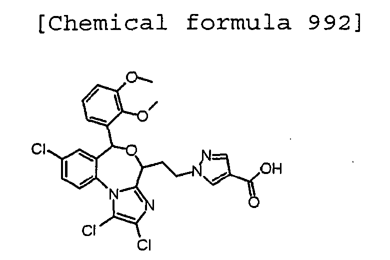

To ethyl 1-{2-[1,2,8-trichloro-6-(2,3-dimethoxyphenyl)-4H,6H-imidazo[1,2-a][4,1]benzoxazepin-4-yl]ethyl}-1H-pyrazole-4-carboxylate as a trans-cis mixture (trans:cis=1:1, 104 mg) was dissolved in methanol (3 mL). To the resulting solution was added a 1N aqueous sodium hydroxide solution (879 ml). The resulting mixture was stirred under heat at 50°C for 2 hours. After concentration of the reaction mixture under reduced pressure, 1N hydrochloric acid (850 ml) was added to the residue. After confirmation that the pH of the resulting mixture fell within a range of from 3 to 4, the mixture was extracted with chloroform. The organic layer was washed with water and then, dried over anhydrous sodium sulfate. The solvent was distilled off under reduced pressure to give the title compound as a trans-cis mixture (trans:cis=1:1, 99 mg).
MS (FAB) m/z: 563 (M+H)⁺.
¹H-NMR (CDCl₃) d: 2.39-2.81 (2H, m), 3.40 (1.5H, br s), 3.48 (1.5H, s), 3.79 (1.5H, br s), 3.87 (1.5H, s), 4.28 (0.5H, dd, J = 7.1, 5.9 Hz), 4.34-4.59 (2.5H, m), 5.63 (0.5H, s), 6.07 (0.5H, br s), 6.78-7.02 (2H, m), 7.16-7.52 (4H, m), 7.88-7.98 (2H, m).
Elemental analysis for: C₂₅H₂₁Cl₃N₄O₅·0.75H₂O·0.3CHCl₃
Calculated: C, 49.86; H, 3.73; N, 9.08.
Found: C, 50.23; H, 3.61; N, 8.71.

### Referential Example 324

(4R,6S)-2,8-Dichloro-6-(2,3-dimethoxyphenyl)-4-(1,3-dioxolan-2-ylmethyl)-4H,6H-imidazo[1,2-a][4,1]benzoxazepine

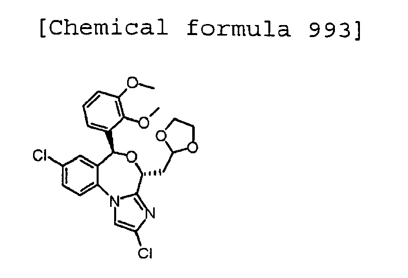

(4R,6S)-8-Chloro-6-(2,3-dimethoxyphenyl)-4-(1,3-dioxolan-2-ylmethyl)-4H,6H-imidazo[1,2-a][4,1]benzoxazepine (150 mg) was dissolved in carbon tetrachloride (7 mL). To the resulting solution was added N-chlorosuccinimide (51 mg). The resulting mixture was stirred under heat at 60°C for one hour. The reaction mixture was concentrated under reduced pressure. The residue was purified by preparative thin layer chromatography on silica gel (hexane:ethyl acetate = 1:3) to give the title compound (118 mg).
MS (FAB) m/z: 477 (M+H)⁺.
¹H-NMR (CDCl₃) δ: 2.54 (2H, dd, J = 6.6, 5.2 Hz), 3.46 (3H, s), 3.82-3.99 (7H, m), 4.54 (1H, t, J = 6.6 Hz), 5.25 (1H, t, J = 5.2 Hz), 5.57 (1H, s), 6.81 (1H, d, J = 2.2 Hz), 6.96 (1H, dd, J = 8.1, 1.3 Hz), 7.17 (1H, s), 7.18 (1H, t, J = 8.1 Hz), 7.26-7.30 (1H, m), 7.45 (1H, dd, J = 8.5, 2.2 Hz), 7.50 (1H, d, J = 8.5 Hz).

### Referential Example 325

2-[(4R,6S)-2,8-Dichloro-6-(2,3-dimethoxyphenyl)-4H,6H-imidazo[1,2-a][4,1]benzoxazepin-4-yl]-1-ethanol

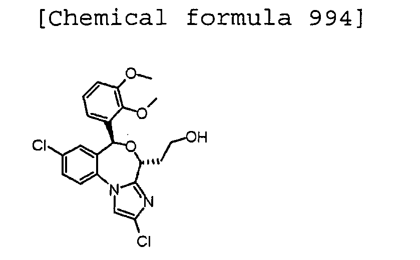

(4R,6S)-2,8-Dichloro-6-(2,3-dimethoxyphenyl)-4-(1,3-dioxolan-2-ylmethyl)-4H,6H-imidazo[1,2-a][4,1]benzoxazepine (116 mg) was dissolved in dichloromethane (14 mL). To the resulting solution was added a 30% aqueous perchloric acid solution (14 mL). The resulting mixture was vigorously stirred overnight at room temperature. The organic layer was washed with a saturated aqueous solution of sodium hydrogen carbonate and dried over anhydrous sodium sulfate. The solvent was distilled off under reduced pressure. The residue was dissolved in tetrahydrofuran (12 mL). To the resulting solution were added water (2 mL) and sodium borohydride (28 mg) and the mixture was stirred at room temperature for 2 hours. Ethyl acetate and water were added to the reaction mixture and the layers separated.
The organic layer was dried over anhydrous sodium sulfate. The solvent was distilled off under reduced pressure. The residue was purified by preparative thin layer chromatography on silica gel (hexane:ethyl acetate = 1:3) to give the title compound (23 mg).
MS (FAB) m/z: 435 (M+H)⁺.
¹H-NMR (CDCl₃) δ: 2.36-2.50 (2H, m), 3.47 (3H, s), 3.77-3.86 (1H, m), 3.86 (3H, s), 3.95-4.02 (1H, m), 4.55 (1H, t, J = 5.9 Hz), 5.59 (1H, s), 6.84 (1H, d, J = 2.2 Hz), 6.97 (1H, dd, J = 8.1, 1.7 Hz), 7.16 (1H, s), 7.19 (1H, t, J = 8.1 Hz), 7.24-7.27 (1H, m), 7.47 (1H, dd, J = 8.5, 2.2 Hz), 7.52 (1H, d, J = 8.5 Hz).

### Example 485

Ethyl 1-{2-[(4R,6S)-2,8-dichloro-6-(2,3-dimethoxyphenyl)-4H,6H-imidazo[1,2-a][4,1]benzoxazepin-4-yl]ethyl}-1H-pyrazole-4-carboxylate

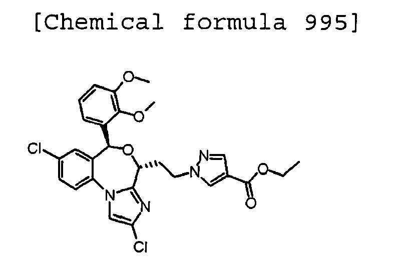

2-[(4R,6S)-2,8-Dichloro-6-(2,3-dimethoxyphenyl)-4H,6H-imidazo[1,2-a][4,1]benzoxazepin-4-yl]-1-ethanol (20 mg) was dissolved in dichloromethane (1 mL). To the resulting solution were added triethylamine (18 µl) and chloromethanesulfonate (5.3 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a crudely purified product of 2-[(4R,6S)-2,8-dichloro-6-(2,3-dimethoxyphenyl)-4H,6H-imidazo[1,2-a][4,1]benzoxazepin-4-yl]ethylmethanesulfonate.
Ethyl 4-pyrazolecarboxylate (13 mg) was dissolved in N,N-dimethylformamide (1 mL). To the resulting solution was added sodium hydride (4 mg) and the resulting mixture was stirred at room temperature for one hour. After addition of tetra-n-butylammonium iodide (17 mg), the crudely purified product obtained above was added and the resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue was purified by preparative thin layer chromatography on silica gel (hexane:ethyl acetate = 1:1) to give the title compound (21 mg).
MS (FAB) m/z: 557 (M+H)⁺.
¹H-NMR (CDCl₃) δ: 1.32 (3H, t, J = 7.2 Hz), 2.72-2.79 (2H, m), 3.46 (3H, s), 3.87 (3H, s), 4.23-4.31 (3H, m), 4.41-4.54 (2H, m), 5.59 (1H, s), 6.82 (1H, d, J = 2.2 Hz), 6.98 (1H, dd, J = 8.1, 1.5 Hz), 7.18 (1H, s), 7.20 (1H, t, J = 8.1 Hz), 7.24-7.27 (1H, m), 7.45 (1H, dd, J = 8.5, 2.2 Hz), 7.50 (1H, d, J = 8.5 Hz), 7.84 (1H, s), 7.89 (1H, s).

### Example 486

1-{2-[(4R,6S)-2,8-Dichloro-6-(2,3-dimethoxyphenyl)-4H,6H-imidazo[1,2-a][4,1]benzoxazepin-4-yl]ethyl}-1H-pyrazole-4-carboxylic acid

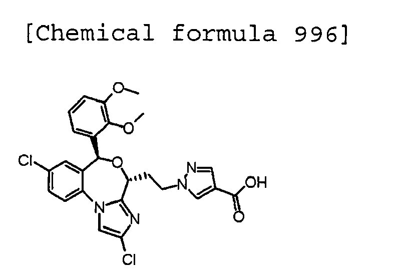

Ethyl 1-{2-[(4R,6S)-2,8-dichloro-6-(2,3-dimethoxyphenyl)-4H,6H-imidazo[1,2-a][4,1]benzoxazepin-4-yl]ethyl}-1H-pyrazole-4-carboxylate (20 mg) was dissolved in methanol (1 mL). To the resulting solution was added a 1N aqueous sodium hydroxide solution (179 µl). The resulting mixture was stirred under heat at 50°C for 2 hours. After concentration of the reaction mixture under reduced pressure, 1N hydrochloric acid (180 µl) was added and the resulting mixture was extracted with chloroform. The organic layer was washed with water and then, dried over anhydrous sodium sulfate. The solvent was distilled off under reduced pressure to give the title compound (14 mg).
MS (FAB) m/z: 529 (M+H)⁺.
¹H-NMR (CDCl₃) δ: 2.69-2.86 (2H, m), 3.48 (3H, s), 3.86 (3H, s), 4.41 (1H, t, J = 6.1 Hz), 4.45-4.53 (2H, m), 5.60 (1H, s), 6.85 (1H, d, J = 2.2 Hz), 6.98 (1H, dd, J = 8.1, 1.6 Hz), 7.19 (1H, t, J = 8.1 Hz), 7.24-7.27 (1H, m), 7.46 (1H, s), 7.48 (1H, dd, J = 8.5, 2.2 Hz), 7.53 (1H, d, J = 8.5 Hz), 7.96 (1H, s), 8.18 (1H, s).
Elemental analysis for: C₂₅H₂₂Cl₂N₄O₅·1.25H₂O·0.3CHCl₃
Calculated: C, 52.00; H, 4.23; N, 9.47.
Found: C, 52.30; H, 4.20; N, 9.20.

### Referential Example 326

(4R,6S)-1,2,8-Trichloro-6-(2,3-dimethoxyphenyl)-4-(1,3-dioxolan-2-ylmethyl)-4H,6H-imidazo[1,2-a][4,1]benzoxazepine

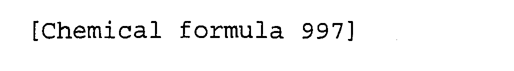

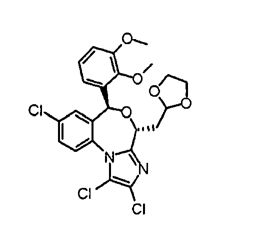

(4R,6S)-8-Chloro-6-(2,3-dimethoxyphenyl)-4-(1,3-dioxolan-2-ylmethyl)-4H,6H-imidazo[1,2-a][4,1]benzoxazepine (1.00 g) was dissolved in carbon tetrachloride (30 mL). To the resulting solution was added N-chlorosuccinimide (756 mg). The resulting mixture was stirred under heat at 60°C for one hour. The reaction mixture was concentrated under reduced pressure. The residue was purified by silica gel column chromatography (hexane:ethyl acetate = 5:1) to give the title compound (526 mg).
MS (FAB) m/z: 511 (M+H)⁺.
¹H-NMR (CDCl₃) δ: 2.48-2.53 (2H, m), 3.48 (3H, s), 3.82-3.97 (7H, m), 4.52 (1H, dd, J = 7.3, 6.1 Hz), 5.24 (1H, t, J = 5.4 Hz), 5.60 (1H, s), 6.81 (1H, d, J = 2.0 Hz), 6.97 (1H, dd, J = 8.1, 1.5 Hz), 7.18 (1H, t, J = 8.1 Hz), 7.24-7.27 (1H, m), 7.46 (1H, dd, J = 8.5, 2.0 Hz), 7.49 (1H, d, J = 8.5 Hz).

### Referential Example 327

2-[(4R,6S)-1,2,8-Trichloro-6-(2,3-dimethoxyphenyl)-4H,6H-imidazo[1,2-a][4,1]benzoxazepin-4-yl]-1-ethanol

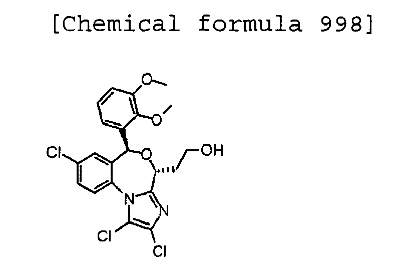

(4R,6S)-1,2,8-Trichloro-6-(2,3-dimethoxyphenyl)-4-(1,3-dioxolan-2-ylmethyl)-4H,6H-imidazo[1,2-a][4,1]benzoxazepine (525 mg) was dissolved in dichloroethane (60 mL). To the resulting solution was added a 30% aqueous perchloric acid solution (60 mL). The resulting mixture was vigorously stirred overnight at room temperature. The organic layer was washed with a saturated aqueous solution of sodium hydrogen carbonate and dried over anhydrous sodium sulfate. The solvent was distilled off under reduced pressure. The residue was dissolved in tetrahydrofuran (20 mL). To the resulting solution were added water (5 mL) and sodium borohydride (156 mg). The resulting mixture was stirred at room temperature for 2 hours. Ethyl acetate and water were added to the reaction mixture and the layers separated. The organic layer was dried over anhydrous sodium sulfate. The solvent was distilled off under reduced pressure. The residue was purified by silica gel column chromatography (hexane:ethyl acetate = from 4:1 to 2:1) to give the title compound (340 mg).
MS (EI) m/z: 468 M⁺.
¹H-NMR (CDCl₃) δ: 2.42 (2H, q, J = 5.7 Hz), 3.50 (3H, s), 3.85-3.97 (5H, m), 4.54 (1H, t, J = 6.2 Hz), 5.64 (1H, s), 6.83 (1H, d, J = 2.0 Hz), 6.98 (1H, dd, J = 7.9, 1.8 Hz), 7.19 (1H, t, J = 7.9 Hz), 7.23 (1H, dd, J = 7.9, 1.3 Hz), 7.46-7.52 (2H, m).
Elemental analysis for: C₂₁H₁₉Cl₃N₂O₄·0.75H₂O·0.1CHCl₃
Calculated: C, 51.30; H, 4.18; N, 5.64.
Found: C, 51.05; H, 3.85; N, 5.35.

### Example 487

Ethyl 1-{2-[(4R,6S)-1,2,8-trichloro-6-(2,3-dimethoxyphenyl)-4H,6H-imidazo[1,2-a][4,1]benzoxazepin-4-yl]ethyl}-1H-pyrazole-4-carboxylate

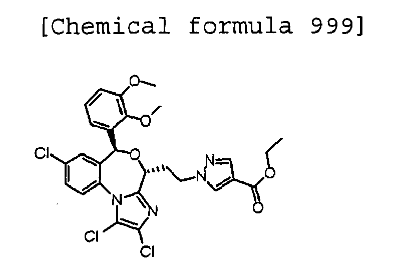

2-[(4R,6S)-1,2,8-Trichloro-6-(2,3-dimethoxyphenyl)-4H,6H-imidazo[1,2-a][4,1]benzoxazepin-4-yl]-1-ethanol (120 mg) was dissolved in dichloromethane (3 mL). To the resulting solution were added triethylamine (132 µl) and chloromethanesulfonate (30 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a crudely purified product of 2-[(4R,6S)-1,2,8-trichloro-6-(2,3-dimethoxyphenyl)-4H,6H-imidazo[1,2-a] [4,1]benzoxazepin-4-yl]ethyl methanesulfonate. Ethyl 4-pyrazolecarboxylate (47 mg) was dissolved in N,N-dimethylformamide (5 mL). To the resulting solution was added sodium hydride (13 mg). The resulting mixture was stirred at room temperature for one hour. After addition of tetra-n-butylammonium iodide (94 mg) to the reaction mixture, the crudely purified product obtained above was added. The resulting mixture was stirred overnight at room temperature. After concentration of the reaction mixture under reduced pressure, ethyl acetate and water were added to the residue and the layers separated. The organic layer was dried over anhydrous sodium sulfate. The solvent was distilled off under reduced pressure. The residue was purified by preparative thin layer chromatography on silica gel (hexane:ethyl acetate = 2:1) to give the title compound (136 mg).
MS (FAB) m/z: 591 (M+H)⁺.
¹H-NMR (CDCl₃) δ: 1.32 (3H, t, J = 7.1 Hz), 2.65-2.77 (2H, m), 3.48 (3H, s), 3.87 (3H, s), 4.22-4.30 (3H, m), 4.38-4.52 (2H, m), 5.62 (1H, s), 6.82 (1H, d, J = 1.7 Hz), 6.99 (1H, dd, J = 7.3, 2.2 Hz), 7.18-7.27 (3H, m), 7.44-7.51 (2H, m), 7.83 (1H, s), 7.89 (1H, s).

### Example 488

1-{2-[(4R,6S)-1,2,8-Trichloro-6-(2,3-dimethoxyphenyl)-4H,6H-imidazo[1,2-a][4,1]benzoxazepin-4-yl]ethyl}-1H-pyrazole-4-carboxylic acid

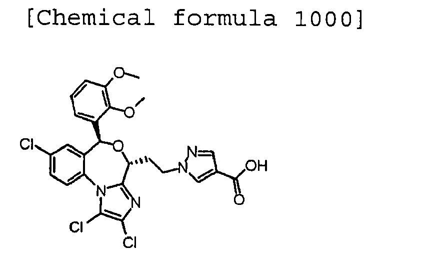

Ethyl 1-{2-[(4R,6S)-1,2,8-trichloro-6-(2,3-dimethoxyphenyl)-4H,6H-imidazo[1,2-a][4,1]benzoxazepin-4-yl]ethyl}-1H-pyrazole-4-carboxylate (134 mg) was dissolved in methanol (5 mL). To the resulting solution was added a 1N aqueous sodium hydroxide solution (1.13 mL) and the resulting mixture was stirred under heat at 50°C for 2 hours. After concentration of the reaction mixture under reduced pressure, 1N hydrochloric acid (1.25 mL) was added to the residue and the mixture was extracted with chloroform. The organic layer was washed with water and then dried over anhydrous sodium sulfate. The solvent was distilled off under reduced pressure. The residue was purified by preparative thin layer chromatography on silica gel (chloroform:methanol = 9:1) to give a colorless foam. The resulting foam was dissolved in diethyl ether (1 mL). To the resulting solution was added hexane (10 mL) and a precipitate thus formed was collected by filtration to give the title compound (94 mg).
MS (FAB) m/z: 563 (M+H)⁺.
¹H-NMR (CDCl₃) δ: 2.65-2.77 (2H, m), 3.47 (3H, s), 3.86 (3H, s), 4.28 (1H, t, J = 6.5 Hz), 4.41-4.49 (2H, m), 5.61 (1H, s), 6.82 (1H, d, J = 2.0 Hz), 6.98 (1H, dd, J = 7.6, 2.0 Hz), 7.16-7.28 (3H, m), 7.43-7.51 (2H, m), 7.83 (1H, s), 7.91 (1H, s).
Elemental analysis for: C₂₅H₂₁Cl₃N₄O₅
Calculated: C, 53.26; H, 3.75; N, 9.94.
Found: C, 52.05; H, 3.76; N, 9.64.

### Referential Example 328

3-[(4R,6S)-1,2,8-Trichloro-6-(2,3-dimethoxyphenyl)-4H,6H-imidazo[1,2-a][4,1]benzoxazepin-4-yl]propanenitrile

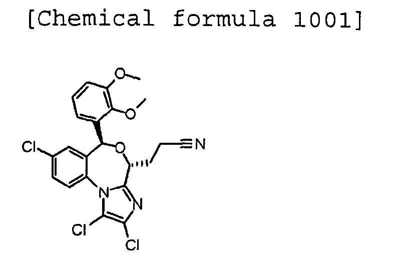

2-[(4R,6S)-1,2,8-Trichloro-6-(2,3-dimethoxyphenyl)-4H,6H-imidazo[1,2-a][4,1]benzoxazepin-4-yl]-1-ethanol (70 mg) was dissolved in dichloromethane (2 mL). To the resulting solution were added triethylamine (77 µl) and chloromethanesulfonate (17 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure to give a pale yellow foam. The resulting foam was dissolved in dimethylsulfoxide (1 mL). To the resulting solution was added sodium cyanide (73 mg) and the mixture was stirred under heat at 50°C for one hour. Ethyl acetate and water were added to the reaction mixture and the layers separated. The organic layer was dried over anhydrous sodium sulfate. The solvent was distilled off under reduced pressure. The residue was purified by preparative thin layer chromatography on silica gel (hexane:ethyl acetate = 1:1) to give the title compound (64 mg).
MS (EI) m/z: 477 M⁺.
¹H-NMR (CDCl₃) δ: 2.40-2.59 (2H, m), 2.72 (3H, t, J = 7.2 Hz), 3.48 (3H, s), 3.87 (3H, s), 4.46 (1H, dd, J = 7.6, 5.4 Hz), 5.63 (0H, s), 6.84 (1H, d, J = 1.7 Hz), 6.99 (1H, dd, J = 7.3, 2.2 Hz), 7.18-7.27 (2H, m), 7.48-7.53 (2H, m).

### Example 489

3-[(4R,6S)-1,2,8-Trichloro-6-(2,3-dimethoxyphenyl)-4H,6H-imidazo[1,2-a][4,1]benzoxazepin-4-yl]propanoic acid

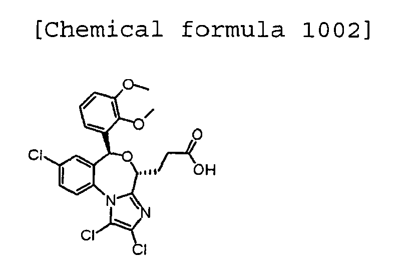

3-[(4R,6S)-1,2,8-Trichloro-6-(2,3-dimethoxyphenyl)-4H,6H-imidazo[1,2-a][4,1]benzoxazepin-4-yl]propanenitrile (62 mg) was dissolved in isopropanol (1 mL). To the resulting solution were added a 35% aqueous sodium hydroxide solution (1 mL) and methanol (1 mL). The resulting mixture was heated under reflux overnight. After concentration of the reaction mixture under reduced pressure, 3N hydrochloric acid (3.2 mL) was added to the residue. After confirmation that the pH of the resulting mixture fell within a range of from 3 to 4, the mixture was extracted three times with chloroform. The organic layer was dried over anhydrous sodium sulfate. The solvent was distilled off under reduced pressure to give the title compound (67 mg).
MS (FAB) m/z: 497 (M+H)⁺.
¹H-NMR (CDCl₃) δ: 2.45-2.52 (2H, m), 2.69 (2H, t, J = 7.0 Hz), 3.48 (3H, s), 3.87 (3H, s), 4.38 (1H, t, J = 6.5 Hz), 5.61 (1H, s), 6.82 (1H, d, J = 2.0 Hz), 6.98 (1H, dd, J = 8.0, 2.0 Hz), 7.16-7.27 (2H, m), 7.46-7.52 (2H, m). Elemental analysis for: C₂₂H₁₉Cl₃N₂O₅
Calculated: C, 51.68; H, 4.04; N, 5.48.
Found: C, 51.66; H, 3.84; N, 5.13.

### Example 490

Ethyl 2-(1-{2-[(4R,6S)-1,2,8-trichloro-6-(2,3-dimethoxyphenyl)-4H,6H-imidazo[1,2-a][4,1]benzoxazepin-4-yl]ethyl}-1H-1,2,3,4-tetrazol-5-yl)acetate

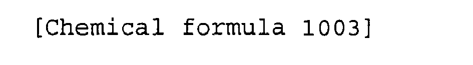

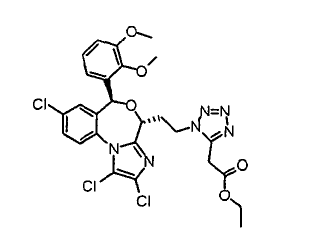

Ethyl 2-(2-{2-[(4R,6S)-1,2,8-trichloro-6-(2,3-dimethoxyphenyl)-4H,6H-imidazo[1,2-a][4,1]benzoxazepin-4-yl]ethyl}-2H-1,2,3,4-tetrazol-5-yl)acetate

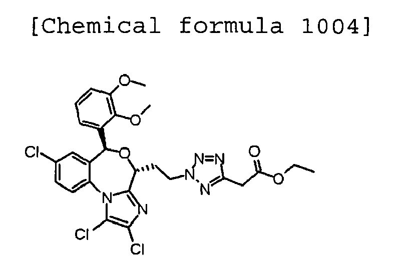

2-[(4R,6S)-1,2,8-Trichloro-6-(2,3-dimethoxyphenyl)-4H,6H-imidazo[1,2-a][4,1]benzoxazepin-4-yl]-1-ethanol (112 mg) was dissolved in dichloromethane (3 mL). To the resulting solution were added triethylamine (123 µl) and chloromethanesulfonate (28 µl) successively. The resulting mixture was stirred at room temperature for one hour. To the reaction mixture was added a saturated aqueous solution of sodium hydrogen carbonate and the layers separated. The organic layer was dried over anhydrous sodium sulfate. The solvent was distilled off under reduced pressure. The residue was dissolved in N,N-dimethylformamide (3 mL). To the resulting solution were added ethyl 1H-tetrazol-5-acetate (111 mg), tetra-n-butylammonium iodide (88 mg) and potassium carbonate (99 mg). The resulting mixture was stirred under heat at 40°C overnight. After concentration of the reaction mixture under reduced pressure, diethyl ether and water were added to the residue and the layers separated. The organic layer was dried over anhydrous magnesium sulfate. The solvent was distilled off under reduced pressure. The residue was purified by preparative thin layer chromatography on silica gel (hexane:ethyl acetate = 2:1) to give ethyl 2-(1-{2-[(4R,6S)-1,2,8-trichloro-6-(2,3-dimethoxyphenyl)-4H,6H-imidazo[1,2-a][4,1]benzoxazepin-4-yl]ethyl}-1H-1,2,3,4-tetrazol-5-yl)acetate (43 mg) as a high polarity substance and ethyl 2-(2-{2-[(4R,6S)-1,2,8-trichloro-6-(2,3-dimethoxyphenyl)-4H,6H-imidazo[1,2-a][4,1]benzoxazepin-4-yl]ethyl}-2H-1,2,3,4-tetrazol-5-yl)acetate (63 mg) as a low polarity substance.
Ethyl 2-(1-{2-[(4R,6S)-1,2,8-trichloro-6-(2,3-dimethoxyphenyl)-4H,6H-imidazo[1,2-a][4,1]benzoxazepin-4-yl]ethyl}-1H-1,2,3,4-tetrazol-5-yl)acetate
MS (FAB) m/z: 607 (M+H)⁺.
¹H-NMR (CDCl₃) δ: 1.27 (3H, t, J = 7.1 Hz), 2.69-2.85 (2H, m), 3.48 (3H, s), 3.88 (3H, s), 4.10 (2H, d, J = 2.9 Hz), 4.19 (2H, q, J = 7.1 Hz), 4.43 (1H, t, J = 6.0 Hz), 4.62-4.78 (2H, m), 5.64 (1H, s), 6.84 (1H, d, J = 1.5 Hz), 6.97-7.02 (1H, m), 7.20 (1H, d, J = 2.0 Hz), 7.21 (1H, s), 7.48-7.50 (2H, m).
Ethyl 2-(2-{2-[(4R,6S)-1,2,8-trichloro-6-(2,3-dimethoxyphenyl)-4H,6H-imidazo[1,2-a][4,1]benzoxazepin-4-yl]ethyl}-2H-1,2,3,4-tetrazol-5-yl)acetate
MS (FAB) m/z: 607 (M+H)⁺.
¹H-NMR (CDCl₃) δ: 1.24 (3H, t, J = 7.0 Hz), 2.87 (2H, q, J = 6.5 Hz), 3.49 (3H, s), 3.87 (3H, s), 3.89 (2H, s), 4.17 (2H, q, J = 7.1 Hz), 4.35 (1H, t, J = 6.5 Hz), 4.89-5.02 (2H, m), 5.64 (1H, s), 6.81 (1H, d, J = 2.0 Hz), 6.99 (1H, dd, J = 8.1, 1.6 Hz), 7.20 (1H, t, J = 8.1 Hz), 7.23-7.27 (1H, m), 7.44-7.52 (2H, m).

### Example 491

### 2-(2-{2-[(4R,6S)-1,2,8-Trichloro-6-(2,3-dimethoxyphenyl)-4H,6H-imidazo[1,2-a][4,1]benzoxazepin-4-yl]ethyl}-2H-1,2,3,4-tetrazol-5-yl)acetic acid

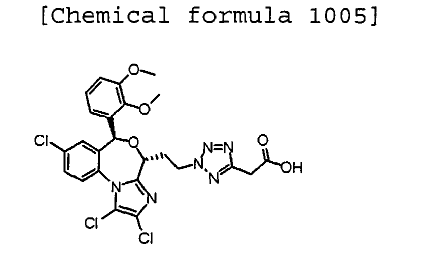

Ethyl 2-(2-{2-[(4R,6S)-1,2,8-trichloro-6-(2,3-dimethoxyphenyl)-4H,6H-imidazo[1,2-a][4,1]benzoxazepin-4-yl]ethyl}-2H-1,2,3,4-tetrazol-5-yl)acetate (144 mg) was dissolved in methanol (8 mL). To the resulting solution were added water (2 mL) and anhydrous potassium carbonate (71 mg). The resulting mixture was stirred under heat at 50°C for 2 hours. After concentration of the reaction mixture under reduced pressure, chloroform and 1N hydrochloric acid (1.1 mL) were added to the residue. After confirmation that the pH of the water layer fell within a range of from 3 to 4, the layers were separated. The water layer was extracted twice with chloroform. The organic layers were combined and dried over anhydrous sodium sulfate. The solvent was distilled off under reduced pressure. To the residue was added a 10:1 hexane:diethyl ether solvent mixture and the solid thus precipitated was collected by filtration to give the title compound (103 mg).
MS (FAB) m/z: 579 (M+H)⁺.
¹H-NMR (CDCl₃) δ: 2.87 (2H, q, J = 6.7 Hz), 3.49 (3H, s), 3.87 (3H, s), 3.95 (2H, s), 4.34 (1H, t, J = 6.7 Hz), 4.90-5.05 (2H, m), 5.64 (1H, s), 6.81 (1H, d, J = 2.0 Hz), 6.99 (1H, dd, J = 7.6, 2.0 Hz), 7.17-7.25 (2H, m), 7.44-7.51 (2H, m).
Elemental analysis for: C₂₄H₂₁Cl₃N₆O₅·0.5H₂O
Calculated: C, 48.96; H, 3.77; N, 14.27.
Found: C, 49.17; H, 3.62; N, 13.77.

### Example 492

2-(1-{2-[(4R,6S)-1,2,8-Trichloro-6-(2,3-dimethoxyphenyl)-4H,6H-imidazo[1,2-a][4,1]benzoxazepin-4-yl]ethyl}-1H-1,2,3,4-tetrazol-5-yl)acetic acid

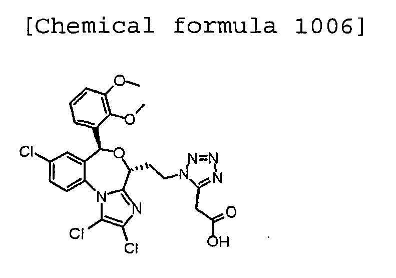

2-(1-(2-[(4R,6S)-1,2,8-Trichloro-6-(2,3-dimethoxyphenyl)-4H,6H-imidazo[1,2-a][4,1]benzoxazepin-4-yl]ethyl)-1H-1,2,3,4-tetrazol-5-yl)acetate (41 mg) was dissolved in methanol (2 mL). To the resulting solution were added water (0.5 mL) and anhydrous potassium carbonate (28 mg). The resulting mixture was stirred under heat at 50°C for 2 hours. After concentration of the reaction mixture under reduced pressure, chloroform and 1N hydrochloric acid (0.4 mL) were added to the residue.
After confirmation of the pH of the water layer fell within a range of from 3 to 4, the layers were separated. The water layer was extracted twice with chloroform. The organic layers were combined and dried over anhydrous sodium sulfate. The solvent was distilled off under reduced pressure to give the title compound (37 mg).
MS (FAB) m/z: 579 (M+H)⁺.
¹H-NMR (CDCl₃) δ: 2.72-2.78 (2H, m), 3.47 (3H, s), 3.87 (3H, s), 4.06 (1H, d, J = 17.8 Hz), 4.24 (1H, d, J = 17.8 Hz), 4.51 (1H, t, J = 6.0 Hz), 4.67 (2H, t, J = 7.0 Hz), 5.65 (1H, s), 6.84 (1H, d, J = 2.2 Hz), 6.97-7.02 (1H, m), 7.17-7.23 (2H, m), 7.45-7.51 (2H, m).
Elemental analysis for: C₂₄H₂₁Cl₃N₆O₅·0.4CHCl₃
Calculated: C, 47.10; H, 3.41; N, 13.29.
Found: C, 47.53; H, 3.63; N, 12.72.

### Example 493

Ethyl trans-2-(8-chloro-6-(2,3-dimethoxyphenyl)-4H,6H-imidazo[(1,2-a)][(4,1)]benzothiazepin-4-yl)acetate

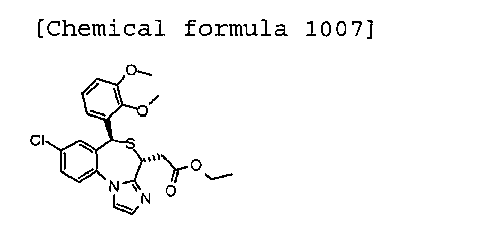

1-[4-Chloro-2-(2,3-dimethoxybenzoyl)phenyl]-1H-imidazol-2-carbaldehyde (2.56 g) was dissolved in toluene (60 mL). To the resulting solution was added (carbethoxymethylene)triphenylphosphine (2.88 g). The resulting mixture was stirred overnight at 80°C. After the reaction mixture was cooled to room temperature, the solvent was distilled off under reduced pressure. The residue was purified by silica gel column chromatography (hexane:ethyl acetate = from 2:1 to 3:1) to give a colorless foam. The resulting foam was dissolved in methanol (70 mL). To the resulting solution was added sodium borohydride (522 mg). The resulting mixture was stirred at room temperature for one hour. After a saturated aqueous solution of ammonium chloride was added to the reaction mixture to terminate the reaction, ethyl acetate was added and the layers separated. The organic layer was dried over anhydrous sodium sulfate and the solvent was distilled off under reduced pressure. The residue was purified by silica gel column chromatography (hexane:ethyl acetate =1:3) to give a colorless foam. The resulting foam was dissolved in toluene (60 mL). To the resulting solution was added Lawesson's reagent (3.32 g) and the mixture was heated under reflux overnight. After the reaction mixture was cooled to room temperature, the solvent was distilled off under reduced pressure. The residue was purified by silica gel column chromatography (hexane:ethyl acetate = from 3:1 to 2:1) to give the title compound (416 mg).
MS (EI) m/z:458 M⁺.
1H-NMR (CDCl3) δ: 1.23 (3H, t, J = 7.1 Hz), 2.82 (1H, dd, J = 16.8, 6.1 Hz), 3.44 (3H, s), 3.48 (1H, dd, J = 16.8, 7.8 Hz), 3.84 (3H, s), 4.07-4.20 (2H, m), 4.41-4.49 (1H, m), 5.4 (1H, s), 6.91-6.99 (2H, m), 7.10-7.24 (3H, m), 7.24-7.29 (2H, m), 7.34 (1H, dd, J = 8.3, 2.2 Hz).

### Example 494

trans-2-(8-Chloro-6-(2,3-dimethoxyphenyl)-4H,6H-imidazo[(1,2-a)][(4,1)]benzothiazepin-4-yl)acetic acid

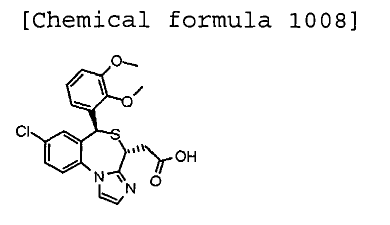

In a similar manner to that employed for the synthesis of 2-(1-(2-(8-chloro-6-(2,3-dimethoxyphenyl)-4H,6H-pyrrolo(1,2-a)(4,1)benzoxazepin-4-yl)acetyl)-4-piperidinyl)acetic acid, the title compound (191 mg) was obtained from ethyl trans-2-(8-chloro-6-(2,3-dimethoxyphenyl)-4H,6H-imidazo(1,2-a)(4,1)benzothiazepin-4-yl)acetate (200 mg) .
MS (FAB) m/z:431(M+H)⁺.
1H-NMR (CDCl3) δ: 3.08 (1H, dd, J = 16.0, 3.5 Hz), 3.23 (1H, dd, J = 16.0, 8.4 Hz), 3.47 (3H, s), 3.84 (3H, s), 4.35 (1H, dd, J = 8.4, 3.5 Hz), 5.38 (1H, s), 6.93-7.01 (2H, m), 7.12-7.19 (2H, m), 7.23-7.30 (2H, m), 7.34-7.39 (1H, m).

### Referential Example 329

trans-Methyl 5-((2-(8-chloro-6-(2,3-dimethoxyphenyl)-4H,6H-imidazo[(1,2-a)][(4,1)]benzothiazepin-4-yl)acetyl)amino)-4-oxopentanoate

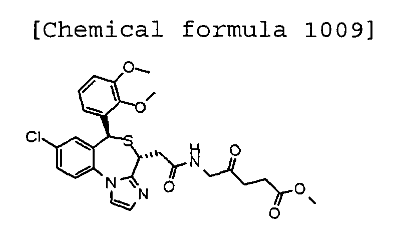

In a similar manner to that employed for the synthesis of methyl 5-({3-[(trans)-8-chloro-1-cyclopropyl-6-(2,3-dimethoxyphenyl)-4H,6H-[1,2,4]triazolo[4,3-a] [4,1]benzothiazepin-4-yl]propanoyl}amino)-4-oxopentanoate, the title compound (159 mg) was obtained from trans-2-(8-chloro-6-(2,3-dimethoxyphenyl)-4H,6H-imidazo(1,2-a)(4,1)benzothiazepin-4-yl)acetic acid (189 mg).
MS (EI) m/z : 557M⁺.
1H-NMR (CDCl3) δ: 2.53-2.84 (5H, m), 3.33-3.42 (1H, m), 3.44 (3H, s), 3.67 (3H, s), 3.84 (3H, s), 4.14 (2H, d, J = 5.1 Hz), 4.45 (1H, q, J = 4.6 Hz), 5.41 (1H, s), 6.92-6.98 (3H, m), 7.13-7.18 (2H, m), 7.25-7.36 (3H, m).

### Example 495

Methyl trans-3-(2-(((8-chloro-6-(2,3-dimethoxyphenyl)-4H,6H-imidazo[(1,2-a)][(4,1)]benzothiazepin-4-yl)methyl)-1,3-thiazol-5-yl)propanoate

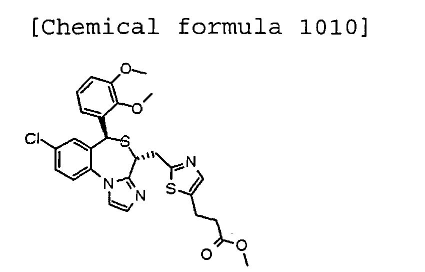

In a similar manner to that employed for the synthesis of methyl 3-(2-{2-[(trans)-8-chloro-1-cyclopropyl-6-(2,3-dimethoxyphenyl)-4H,6H-[1,2,4]triazolo[4,3-a][4,1]benzothiazepin-4-yl]ethyl}-1,3-thiazol-5-yl)propanoate, the title compound (80 mg) was obtained from trans-methyl 5-((2-(8-chloro-6-(2,3-dimethoxyphenyl)-4H,6H-imidazo[(1,2-a)][(4,1)]benzothiazepin-4-yl)acetyl)amino)-4-oxopentanoate (189 mg).
MS (EI) m/z: 555 M⁺.
1H-NMR (CDCl3) δ: 2.64 (2H, t, J = 7.2 Hz), 3.10 (2H, t, J = 7.2 Hz), 3.44 (3H, s), 3.60 (1H, dd, J = 15.1, 7.8 Hz), 3.69 (3H, s), 3.83 (3H, s), 4.01 (1H, dd, J = 15.1,6.6 Hz), 4.52 (1H, dd, J = 7.8, 6.6 Hz), 5.41 (1H, s), 6.92 (1H, dd, J = 8.0, 1.3 Hz), 6.95 (1H, d, J = 2.2 Hz), 7.12 (1H, t, J = 8.0 Hz), 7.15 (1H, d, J = 1.3 Hz), 7.21-7.27 (3H, m), 7.30-7.33 (1H, m), 7.37 (1H, s).

### Example 496

3-(2-{[trans-8-Chloro-6-(2,3-dimethoxyphenyl)-4H,6H-imidazo[1,2-a][4,1]benzothiazepin-4-yl]methyl}-1,3-thiazol-5-yl)propanoic acid

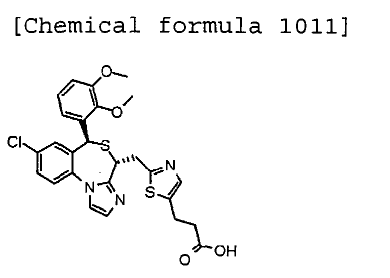

Methyl trans-3-(2-(((8-chloro-6-(2,3-dimethoxyphenyl)-4H,6H-imidazo[(1,2-a)][(4,1)]benzothiazepin-4-yl)methyl)-1,3-thiazol-5-yl)propanoate (78 mg) was dissolved in methanol (2 mL). To the resulting solution were added water (2 mL) and anhydrous potassium carbonate (58 mg). The resulting mixture was stirred overnight at room temperature for 2 hours. After concentration of the reaction mixture under reduced pressure, 0.1N hydrochloric acid was added to the residue. After confirmation that the pH of the resulting mixture fell within a range of from 3 to 4, chloroform was added to the mixture and the layers separated. The organic layer was dried over anhydrous sodium sulfate. The solvent was distilled off under reduced pressure to give the title compound (72 mg).
MS (FAB) m/z: 542 (M+H)⁺.
1H-NMR (CDCl3) δ: 2.68 (2H, t, J = 7.0 Hz), 3.11 (2H, t, J = 7.0 Hz), 3.44 (3H, s), 3.56-3.64 (1H, m), 3.83 (3H, s), 3.95-4.03 (1H, m), 4.57 (1H, t, J = 7.2 Hz), 5.38 (1H, s), 6.92 (1H, dd, J = 8.3, 1.5 Hz), 6.97 (1H, br s), 7.09-7.39 (7H, m).
Elemental analysis for: C₂₆H₂₄ClN₃O₄·1.0H₂O·0.4CHCl₃
Calculated: C, 52.54; H, 4.34; N, 6.86.
Found: C, 52.55; H, 4.23; N, 6.67.

### Example 497

2-[trans-8-Chloro-6-(2,3-dimethoxyphenyl)-1-(trifluoromethyl)-4H,6H-[1,2,4]-triazolo-[4,3-a][4,1]benzoxazepin-4-yl]acetic acid

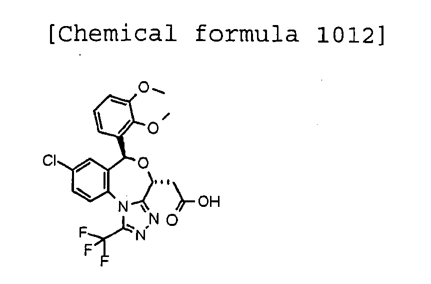

Ethyl 2-[trans-8-chloro-6-(2,3-dimethoxyphenyl)-1-(trifluoromethyl)-4H,6H-[1,2,4]-triazolo-[4,3-a][4,1]benzoxazepin-4-yl]acetate (373 mg) was dissolved in 1,4-dioxane (15 mL). To the resulting solution was added concentrated hydrochloric acid (3.3 mL). The resulting mixture was stirred at 60°C for 13 hours. After cooling to room temperature, the reaction mixture was diluted with ethyl acetate and then washed with water. The organic layer was dried over anhydrous sodium sulfate. The filtrate was then concentrated. The residue obtained by concentration was purified by preparative TLC (methanol:chloroform=1:9) to give the title compound (315 mg).
MS (ESI) m/z : 484 (M+H)⁺.
¹H-NMR (CDCl₃) δ: 3.01-3.04 (1H, m), 3.25-3.27 (1H, m), 3.40 (3H, s), 3.84 (3H, s), 4.84-4.86 (1H, m), 5.49 (1H, s), 6.80 (1H, d, J = 2.2 Hz), 6.93 (1H, d, J = 7.9 Hz), 7.13 (1H, t, J = 7.9 Hz), 7.21 (1H, d, J = 7.9 Hz), 7.39 (1H, dd, J = 8.5, 2.2 Hz), 7.49 (1H, d, J = 8.5 Hz).

### Example 498

Ethyl 2-[(4R,6S)-8-chloro-6-(2,3-dimethoxyphenyl)-1-(trifluoromethyl)-4H,6H-[1,2,4]-triazolo-[4,3-a] [4,1]benzoxazepin-4-yl]acetate (isomer A) Ethyl 2-[(4S,6R)-8-chloro-6-(2,3-dimethoxyphenyl)-1-(trifluoromethyl)-4H,6H-[1,2,4]-triazolo-[4,3-a][4,1]benzoxazepin-4-yl]acetate (isomer B)

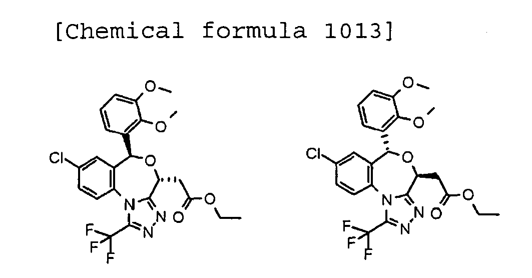

The racemic compound (1138 mg) was optically resolved by an optically active column (CHIRALPAK-AD; 5 cm × 50 cm) into the isomer A (496 mg) and isomer B (500 mg). The isomer A was an isomer with a short retention time, while the isomer B was an isomer with a long retention time.
Flow rate: 50 mL/min
Developing solvent: 15% ethanol-n-hexane

### Example 499

2-[(4R,6S)-8-Chloro-6-(2,3-dimethoxyphenyl)-1-(trifluoromethyl)-4H,6H-[1,2,4]-triazolo-[4,3-a][4,1]benzoxazepin-4-yl]acetic acid

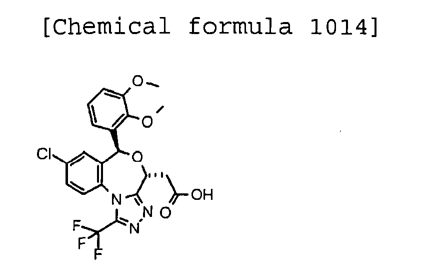

Ethyl 2-[(4R,6S)-8-chloro-6-(2,3-dimethoxyphenyl)-1-(trifluoromethyl)-4H,6H-[1,2,4]-triazolo-[4,3-a][4,1]benzoxazepin-4-yl]acetate (500 mg) was dissolved in 1,4-dioxane (15 mL). To the resulting solution was added concentrated hydrochloric acid (3.3 mL). The resulting mixture was stirred at 60°C for 21 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (333 mg).
MS (ESI) m/z : 484 (M+H)⁺.
¹H-NMR (CDCl₃) δ: 3.01-3.04 (1H, m), 3.25-3.27 (1H, m), 3.40 (3H, s), 3.84 (3H, s), 4.84-4.86 (1H, m), 5.49 (1H, s), 6.80 (1H, d, J = 2.2 Hz), 6.93 (1H, d, J = 7.9 Hz), 7.13 (1H, t, J = 7.9 Hz), 7.21 (1H, d, J = 7.9 Hz), 7.39 (1H, dd, J = 8.5, 2.2 Hz), 7.49 (1H, d, J = 8.5 Hz).

### Example 500

2-[(4S,6R)-8-Chloro-6-(2,3-dimethoxyphenyl)-1-(trifluoromethyl)-4H,6H-[1,2,4]-triazolo-[4,3-a][4,1]benzoxazepin-4-yl]acetic acid

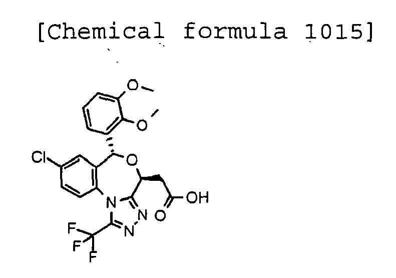

Ethyl 2-[(4S,6R)-8-chloro-6-(2,3-dimethoxyphenyl)-1-(trifluoromethyl)-4H,6H-[1,2,4]-triazolo-[4,3-a][4,1]benzoxazepin-4-yl]acetate (496 mg) was dissolved in 1,4-dioxane (15 mL). To the resulting solution was added concentrated hydrochloric acid (3.3 mL). The resulting mixture was stirred at 60°C for 21 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (353 mg).
MS (ESI) m/z : 484 (M+H)⁺.
¹H-NMR: completely coincided with that of 2-[(4R,6S)-8-chloro-6-(2,3-dimethoxypheriyl)-1-(trifluoromethyl)-4H,6H-[1,2,4]-triazolo-[4,3-a][4,1]benzoxazepin-4-yl]acetic acid.

### Example 501

Ethyl 2-(1-{2-[trans-8-chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate

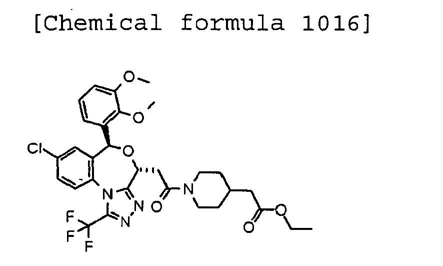

2-[trans-8-Chloro-6-(2,3-dimethoxyphenyl)-1-(trifluoromethyl)-4H,6H-[1,2,4]-triazolo-[4,3-a][4,1]benzoxazepin-4-yl]acetic acid (1.58 g) and ethyl 2-(4-piperidinyl)acetate (0.67 g) were dissolved in dichloromethane (30 mL). To the resulting solution were added 1-ethyl-3-(3-dimethylaminopropyl)carbodiimide hydrochloride (0.75 g) and 1-hydroxybenzotriazole (0.15 g). The resulting mixture was stirred at room temperature for 4 hours. The reaction mixture was diluted with chloroform and washed with saturated brine. The organic layer was dried over anhydrous sodium sulfate. After concentration of the filtrate, the residue thus obtained was purified by silica gel column (ethyl acetate:hexane=1:1, methanol:chloroform=5:95) to give the title compound (1.19 g).
MS (ESI) m/z : 637 (M+H)⁺.
¹H-NMR (CDCl₃) δ: 1.10-1.21 (1.5H, m), 1.25-1.27 (3.0H, m), 1.41-1.42 (0.5H, m), 1.74-1.80 (2.0H, m), 2.03-2.06 (1.0H, m), 2.22-2.27 (2.0H, m), 2.58-2.65 (1.0H, m), 3.05-3.24 (2.0H, m), 3.42 (3.0H, d, J = 1.2 Hz), 3.52-3.58 (1.0H, m), 3.86 (3.0H, d, J = 0.7 Hz), 4.02-4.05 (1.0H, m), 4.12-4.16 (2.0H, m), 4.53-4.57 (1.0H, m), 5.06-5.08 (1.0H, m), 5.53 (1.0H, d, J = 5.4 Hz), 6.82-6.85 (1.0H, m), 6.97-6.99 (1.0H, m), 7.17-7.25 (2.0H, m), 7.50-7.52 (2.0H, m).

### Example 502

Ethyl 2-(1-{2-[(4R,6S)-8-chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate
(isomer B) Ethyl 2-(1-{2-[(4S,6R)-8-chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate
(isomer A)

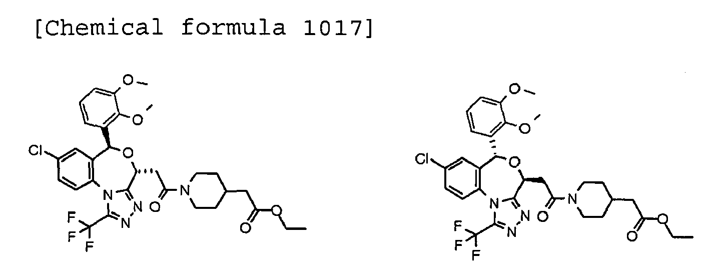

The racemic compound (1.19 g) was optically resolved using an optically active column (CHIRALPAK-AD; 2 cm × 25 cm) into the isomer A (596 mg) and isomer B (585 mg).
Flow rate: 10 mL/min
Developing solvent: 100% ethanol
Retention time: isomer A: 7.5 min., isomer B: 60 min.

### Example 503

2-(1-{2-[(4R,6S)-8-Chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetic acid

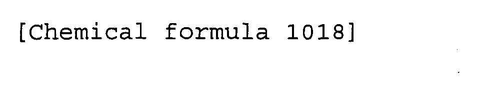

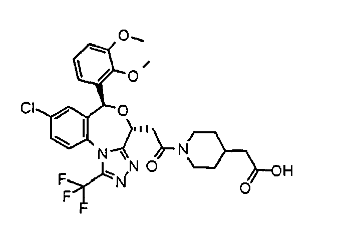

Ethyl 2-(1-{2-[(4R,6S)-8-chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate (585 mg) was dissolved in 1,4-dioxane (35 mL). To the resulting solution was added concentrated hydrochloric acid (5 mL). The resulting mixture was stirred at room temperature for 71 hours. The reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (383 mg) and the ester (178 mg) used as the starting material. MS (ESI) m/z : 609 (M+H)⁺.
¹H-NMR (CDCl₃) δ: 1.17-1.19 (1.5H, m), 1.42-1.47 (0.5H, m), 1.77-1.83 (2.0H, m), 2.03-2.06 (1.0H, m), 2.26-2.33 (2.0H, m), 2.61-2.64 (1.0H, m), 3.15-3.21 (2.0H, m), 3.42 (3.0H, s), 3.51-3.59 (1.0H, m), 3.86 (3.0H, s), 4.05-4.08 (1.0H, m), 4.55-4.58 (1.0H, m), 5.06-5.08 (1.0H, m), 5.52-5.54 (1.0H, m), 6.84 (1.0H, s), 6.97-6.99 (1.0H, m), 7.19-7.22 (2.0H, m), 7.49-7.51 (2.0H, m).

### Example 504

2-(1-{2-[(4S,6R)-8-Chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetic acid

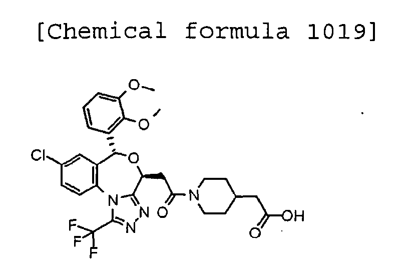

Ethyl 2-(1-{2-[(4S,6R)-8-chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate (91.2 mg) was dissolved in 1,4-dioxane (5 mL). To the resulting solution was added concentrated hydrochloric acid (2 mL). The resulting mixture was stirred at 60°C for 16 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (18.5 mg).
MS (ESI) m/z : 609 (M+H)⁺.
¹H-NMR coincided completely with that of 2-(1-{2-[(4R,6S)-8-chloro-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetic acid.

### Example 505

Ethyl 2-[(4R,6S)-8-chloro-6-(2,3-dimethoxyphenyl)-1-(fluoromethyl)-4H,6H-[1,2,4]triazolo[4,3-a] [4,1]benzoxazepin-4-yl]acetate (isomer B) Ethyl 2-[(4S,6R)-8-chloro-6-(2,3-dimethoxyphenyl)-1-(fluoromethyl)-4H,6H-[1,2,4]triazolo[4,3-a] [4,1]benzoxazepin-4-yl]acetate (isomer A)

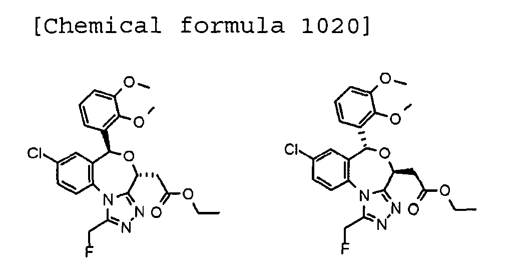

The racemic compound (175 mg) was optically resolved by an optically active column (CHIRALPAK-AD; 2 cm × 25 cm) into the isomer A (85.4 mg) and isomer B (74.0 mg).
Flow rate: 10 mL/min
Developing solvent: 15% ethanol-n-hexane
Retention time: isomer A: 21 min., isomer B: 31 min.

### Example 506

2-[(4R,6S)-8-Chloro-6-(2,3-dimethoxyphenyl)-1-(fluoromethyl)-4H,6H-[1,2,4]triazolo[4,3-a][4,1]benzoxazepin-4-yl]acetic acid

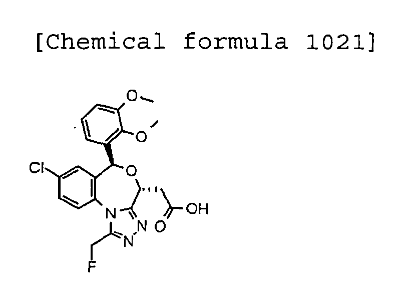

Ethyl 2-[(4R,6S)-8-chloro-6-(2,3-dimethoxyphenyl)-1-(fluoromethyl)-4H,6H-[1,2,4]triazolo[4,3-a][4,1]benzoxazepin-4-yl]acetate (74.0 mg) was dissolved in 1,4-dioxane (5 mL). To the resulting solution was added concentrated hydrochloric acid (1.1 mL). The resulting mixture was stirred at 60°C for 21 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform:water=3:7:1). A mixture (45.2 mg) of the title compound and a chloromethyl compound thus obtained was dissolved in acetonitrile (2 mL). To the resulting solution was added tetrabutylammonium fluoride trihydrate (61.4 mg). At room temperature, the resulting mixture was stirred for 3 hours. Water was added and the mixture was extracted with ethyl acetate. The organic layer was dried over anhydrous sodium sulfate. The filtrate was then concentrated. The residue obtained by concentration was purified by preparative TLC (methanol:chloroform:water=3:7:1) to give the title compound (22.5 mg).
MS m/z : 448 (M+H⁺).
¹H-NMR (CDCl₃) δ: 3.07-3.10 (1H, m), 3.35-3.37 (1H, m), 3.40 (3H, s), 3.84 (3H, s), 4.91-4.93 (1H, m), 5.41 (1H, dd, J = 48.2, 11.8 Hz, Hz), 5.53 (1H, s), 5.83 (1H, dd, J = 48.2, 11.8 Hz, Hz), 6.77-6.80 (1H, m), 6.93 (1H, d, J = 7.9 Hz), 7.13 (1H, t, J = 7.9 Hz), 7.22 (1H, d, J = 7.9 Hz), 7.38-7.40 (1H, m), 7.55-7.57 (1H, m).

### Example 507

2-[(4S,6R)-8-Chloro-6-(2,3-dimethoxyphenyl)-1-(fluoromethyl)-4H,6H-[1,2,4]triazolo[4,3-a][4,1]benzoxazepin-4-yl]acetic acid

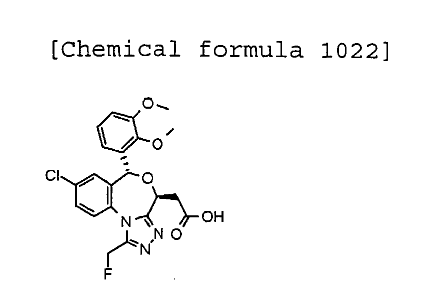

Ethyl 2-[(4S,6R)-8-chloro-6-(2,3-dimethoxyphenyl)-1-(fluoromethyl)-4H, 6H-[1,2,4]triazolo[4,3-a] [4, 1] benzoxazepin-4-yl] acetate (84.5 mg) was dissolved in 1,4-dioxane (5 mL). To the resulting solution was added concentrated hydrochloric acid (1.1 mL). The resulting mixture was stirred at 60°C for 21 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform:water=3:7:1). A mixture (54.0 mg) of the title compound and a chloromethyl compound thus obtained was dissolved in acetonitrile (2 mL). To the resulting solution was added tetrabutylammonium fluoride trihydrate (73.4 mg). The resulting mixture was stirred at room temperature for 3 hours. Water was added and the mixture was extracted with ethyl acetate. The organic layer was dried over anhydrous sodium sulfate. The filtrate was then concentrated. The residue obtained by concentration was purified by preparative TLC (methanol:chloroform:water=3:7:1) to give the title compound (26.3 mg).
MS m/z : 448 (M+H⁺).
¹H-NMR coincided completely with that of 2-[(4R,6S)-8-chloro-6-(2,3-dimethoxyphenyl)-1-(fluoromethyl)-4H,6H-[1,2,4]triazolo[4,3-a][4,1]benzoxazepin-4-yl]acetic acid.

### Example 508

Methyl 3-(2-{[trans-8-chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoate

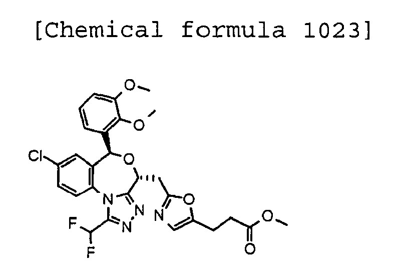

Methyl 3-(2-{[trans-7-chloro-5-(2,3-dimethoxyphenyl)-2-[(Z)hydrazono]-1,5-dihydro-4,1-benzoxazepin3(3H)-yl]methyl}-1,3-oxazol-5-yl)propanoate (659 mg) was dissolved in 1,2-dichloroethane (5 mL). To the resulting solution were added difluoroacetic anhydride (623 mg) and difluoroacetic acid (1.61 mL). The resulting mixture was stirred at room temperature for one hour. After the reaction mixture was stirred at 55°C for 2 hours, toluene (12 mL) was added. The resulting mixture was heated under reflux for 1.5 hours. After cooling to room temperature, the reaction mixture was concentrated. The residue was diluted with chloroform and washed with a saturated aqueous solution of sodium bicarbonate. The organic layer was dried over anhydrous sodium sulfate. The filtrate was concentrated and the residue thus obtained was purified by silica gel column (ethyl acetate:hexane=1:1) to give the title compound (655 mg).
MS (ESI) m/z : 575 (M+H)⁺.
¹H-NMR (CDCl₃) δ: 2.66 (2H, t, J = 7.4 Hz), 2.98 (2H, t, J = 7.4 Hz), 3.41 (3H, s), 3.66 (1H, dd, J = 15.9, 7.6 Hz), 3.69 (3H, s), 3.85 (1H, dd, J = 15.9, 5.9 Hz), 3.85 (3H, s), 5.01 (1H, dd, J = 7.6, 5.9 Hz), 5.59 (1H, s), 6.67-6.69 (1H, m), 6.81 (1H, d, J = 2.2 Hz), 6.96 (1H, dd, J = 8.1, 1.5 Hz), 7.08 (1H, dd, J = 7.8, 1.5 Hz), 7.12 (1H, dd, J = 53.7, 50.5 Hz), 7.16 (1H, t, J = 7.8 Hz), 7.50 (1H, dd, J = 8.5, 2.2 Hz), 7.68 (1H, d, J = 8.5 Hz).

### Example 509

Methyl 3-(2-{[(4R,6S)-8-chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoate
(isomer B) Methyl 3-(2-{[(4S,6R)-8-chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoate
(isomer A)

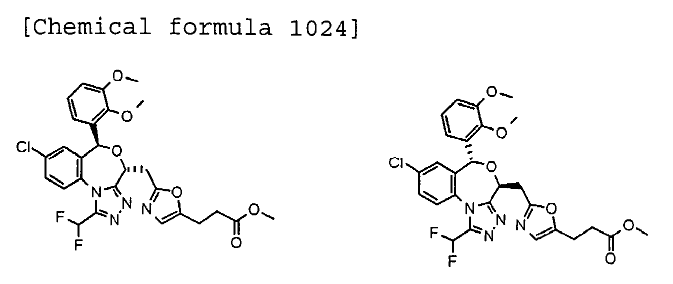

The racemic compound (655 mg) was optically resolved using an optically active column (CHIRALPAK-AD; 5 cm × 50 cm) into the isomer A (244 mg) and isomer B (248 mg).
Flow rate: 50 mL/min
Developing solvent: 90% ethanol-n-hexane
Retention time: isomer A: 57 min., isomer B: 103 min.

### Example 510

3-(2-{[(4R,6S)-8-Chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoic acid

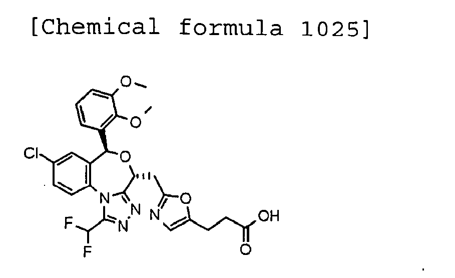

Methyl 3-(2-{[(4R,6S)-8-chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoate (248 mg) was dissolved in methanol-water-tetrahydrofuran (2:1:2, 7.5 mL). To the resulting solution was added potassium carbonate (178 mg). The resulting mixture was stirred at room temperature for 16 hours. The reaction mixture was neutralized with 1N hydrochloric acid, diluted with water and extracted with chloroform. The organic layer was dried over anhydrous sodium sulfate. After concentration of the filtrate, the residue thus obtained was purified by preparative TLC (methanol:chloroform=1:9) to give the title compound (234 mg).
MS (ESI) m/z : 561 (M+H)⁺.
¹H-NMR (CDCl₃) δ: 2.67 (2H, t, J = 7.3 Hz), 2.96 (2H, t, J = 7.3 Hz), 3.41 (3H, s), 3.66 (1H, dd, J = 15.6, 7.3 Hz, Hz), 3.85 (1H, dd, J = 15.6, 6.3 Hz, Hz), 3.86 (3H, s), 5.00 (1H, dd, J = 7.3, 6.3 Hz, Hz), 5.59 (1H, s), 6.70 (1H, s), 6.82 (1H, d, J = 2.0 Hz), 6.95-7.18 (4H, m), 7.50 (1H, dd, J = 8.5, 2.0 Hz, Hz), 7.68 (1H, d, J = 8.5 Hz).

### Example 511

3-(2-{[(4S,6R)-8-Chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl) propanoic acid

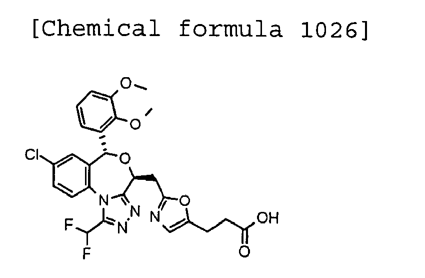

Methyl 3-(2-{[(4S,6R)-8-chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoate (244 mg) was dissolved in methanol-water-tetrahydrofuran (2:1:2, 7.5 mL). To the resulting solution was added potassium carbonate (176 mg). The resulting mixture was stirred at room temperature for 16 hours. The reaction mixture was neutralized with 1N hydrochloric acid, diluted with water and extracted with chloroform. The organic layer was dried over anhydrous sodium sulfate. The residue thus obtained was purified by preparative TLC (methanol:chloroform=1:9) to give the title compound (223 mg).
MS (ESI) m/z : 561 (M+H)⁺.
¹H-NMR coincided completely with that of 3-(2-{[(4R,6S)-8-chloro-1-(difluoromethyl)-6-(2,3-dimethoxyphenyl)-4H,6H-[1,2,4]triazolo[4,3-a][4,1]benzoxazepin-4-yl]methyl}-1,3-oxazol-5-yl)propanoic acid.

### Referential Example 330

{5-Chloro-2-[(2,4-dimethoxybenzyl)amino]phenyl}(2-chloro-3-methoxyphenyl)methanol

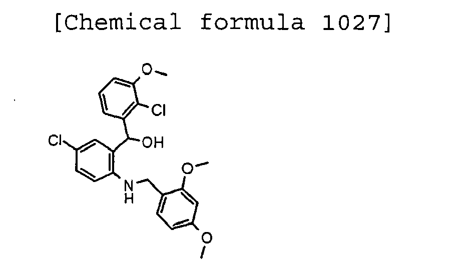

2-Amino-5-chlorophenyl)(2-chloro-3-methoxyphenyl)methanol (3.63 g) and 2,4-dimethoxybenzaldehyde (2.43 g) were dissolved in acetic acid (30 mL). The resulting solution was stirred at room temperature for 20 minutes. To the reaction mixture was added sodium borohydride (1.38 g) at 0°C and the mixture was stirred at room temperature for one hour. The reaction mixture was concentrated. The residue was diluted with ethyl acetate and washed with a saturated aqueous solution of sodium bicarbonate and saturated brine. The organic layer was dried over anhydrous sodium sulfate. The filtrate was then concentrated. The residue was purified by silica gel column (ethyl acetate:hexane=1:1) to give the title compound (4.17 g).
¹H-NMR (CDCl₃) δ: 3.78 (6H, s), 3.92 (3H, s), 4.26 (2H, s), 6.17 (1H, s), 6.40 (1H, dd, J = 8.3, 2.4 Hz), 6.45 (1H, d, J = 2.4 Hz), 6.64 (1H, d, J = 8.8 Hz), 6.82 (1H, d, J = 2.7 Hz), 6.93 (1H, dd, J = 8.2, 1.3 Hz), 7.07-7.11 (3H, m), 7.27 (1H, t, J = 7.8 Hz).

### Referential Example 331

Ethyl (E)-4-[4-chloro-2-[(2-chloro-3-methoxyphenyl) (hydroxy) methyl] (2,4-dimethoxybenzyl)anilino]-4-oxo-2-butenoate

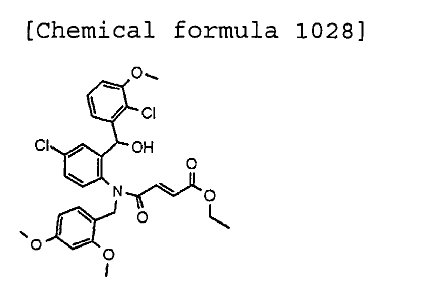

{5-Chloro-2-[(2,4-dimethoxybenzyl)amino]phenyl}(2-chloro-3-methoxyphenyl)methanol (5.46 g) was dissolved in dichloromethane (40 mL). Sodium hydrogen carbonate (1.43 g) and a methylene chloride solution (20 mL) of monoethyl chlorofumarate (2.43 g) prepared separately were added dropwise to the resulting solution at 0°C. After stirring the reaction mixture overnight at room temperature, the reaction mixture was diluted with dichloromethane and washed with a saturated aqueous solution of sodium bicarbonate and saturated brine. The organic layer was dried over anhydrous sodium sulfate. The filtrate was then concentrated to give the title compound (6.48 g).

### Referential Example 332

Ethyl 2-[trans-7-chloro-5-(2-chloro-3-methoxyphenyl)-1-(2,4-dimethoxybenzyl)-2-oxo-1,2,3,5-tetrahydro-4,1-benzoxazepin-3-yl]acetate

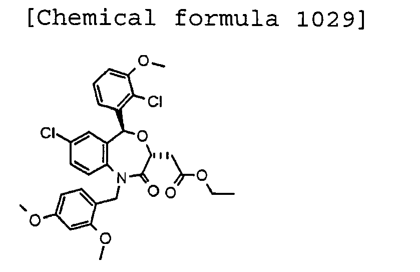

Ethyl (E)-4-[4-chloro-2-[(2-chloro-3-methoxyphenyl)(hydroxy)methyl](2,4-dimethoxybenzyl)anilino]-4-oxo-2-butenoate (6.48 g) was dissolved in ethanol (120 mL). To the resulting solution was added potassium carbonate (1.87 g). The resulting mixture was stirred overnight at room temperature. The reaction mixture was concentrated. The residue was diluted with chloroform and washed with saturated brine. The organic layer was dried over anhydrous sodium sulfate. The filtrate was then concentrated. Crystals thus formed were collected by filtration in diethyl ether to give the title compound (3.80 g). The mother liquor was concentrated and the residue thus obtained was purified by silica gel column (ethyl acetate:hexane=1:3) to give the title compound (0.28 g).
¹H-NMR (CDCl₃) δ: 1.24-1.27 (3H, m), 2.81 (1H, dd, J = 16.5, 6.0 Hz), 3.10 (1H, dd, J = 16.5, 7.6 Hz), 3.60 (3H, s), 3.75 (3H, s), 3.90 (3H, s), 4.12-4.18 (2H, m), 4.46 (1H, dd, J = 7.6, 6.0 Hz), 4.46 (1H, d, J = 14.6 Hz), 4.86 (1H, d, J = 14.6 Hz), 5.87 (1H, s), 6.33 (1H, d, J = 2.2 Hz), 6.37-6.40 (2H, m), 6.95 (1H, dd, J = 8.1, 1.5 Hz), 7.31-7.35 (5H, m).

### Referential Example 333

Ethyl 2-[trans-7-chloro-5-(2-chloro-3-methoxyphenyl)-2-oxo-1,2,3,5-tetrahydro-4,1-benzoxazepin-3-yl]acetate

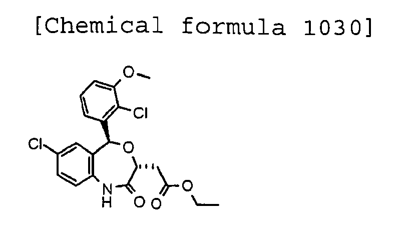

Ethyl 2-[trans-7-chloro-5-(2-chloro-3-methoxyphenyl)-1-(2,4-dimethoxybenzyl)-2-oxo-1,2,3,5-tetrahydro-4,1-benzoxazepin-3-yl]acetate (4.16 g) was dissolved in acetone (80 mL). To the resulting solution was added an aqueous solution (20 mL) of ammonium dicerium (IV) nitrate (11.9 g) at 0°C. The resulting mixture was stirred at room temperature for 1.5 hours. To the reaction mixture was added a saturated aqueous solution of sodium bicarbonate. The resulting mixture was extracted with ethyl acetate.
The extract was washed with saturated brine. The organic layer was dried over anhydrous sodium sulfate. The filtrate was then concentrated and the residue was purified by silica gel column (ethyl acetate:hexane=1:2) to give the title compound (1.82 g).
¹H-NMR (CDCl₃) δ: 1.23 (3H, t, J = 7.1 Hz), 2.81 (1H, dd, J = 16.5, 6.5 Hz), 3.04 (1H, dd, J = 16.5, 6.7 Hz), 3.94 (3H, s), 4.12-4.14 (2H, m), 4.61 (1H, dd, J = 6.7, 6.5 Hz), 6.21 (1H, s), 6.59 (1H, d, J = 2.2 Hz), 6.99-7.03 (2H, m), 7.21-7.22 (1H, m), 7.30-7.37 (2H, m), 7.72 (1H, s).

### Referential Example 334

Ethyl 2-[trans-7-chloro-5-(2-chloro-3-methoxyphenyl)-2-thioxo-1,2,3,5-tetrahydro-4,1-benzoxazepin-3-yl]acetate

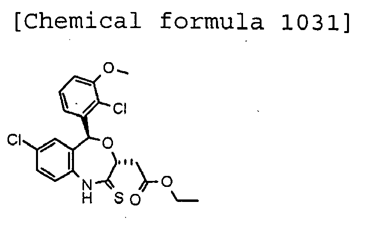

Ethyl 2-[trans-7-chloro-5-(2-chloro-3-methoxyphenyl)-2-oxo-1,2,3,5-tetrahydro-4,1-benzoxazepin-3-yl]acetate (1.82 g) was dissolved in tetrahydrofuran (30 mL). To the resulting solution were added sodium hydrogen carbonate (0.76 g) and diphosphorus pentasulfide (2.01 g). The resulting mixture was stirred at room temperature for 2.5 hours. The reaction mixture was diluted with ethyl acetate and washed with saturated brine. The organic layer was dried over anhydrous sodium sulfate. After concentration of the filtrate, the residue thus obtained was purified by silica gel column (ethyl acetate:hexane=1:2) to give the title compound (0.57 g).
¹H-NMR (CDCl₃) δ : 1.25 (3H, t, J = 7.1 Hz), 2.99 (1H, dd, J = 16.5, 6.5 Hz), 3.26 (1H, dd, J = 16.5,-6.7 Hz), 3.93 (3H, s), 4.11-4.17 (2H, m), 4.69 (1H, dd, J = 6.7, 6.5 Hz), 6.13 (1H, s), 6.56 (1H, d, J = 2.2 Hz), 7.00 (1H, dd, J = 8.3, 1.5 Hz), 7.09 (1H, d, J = 8.3 Hz), 7.27-7.27 (1H, m), 7.35-7.39 (2H, m), 9.62 (1H, s).

### Example 512

Ethyl 2-[trans-8-chloro-6-(2-chloro-3-methoxyphenyl)-1-(trifluoromethyl)-4H,6H-[1,2,4]triazolo[4,3-a][4,1]benzoxazepin-4-yl]acetate

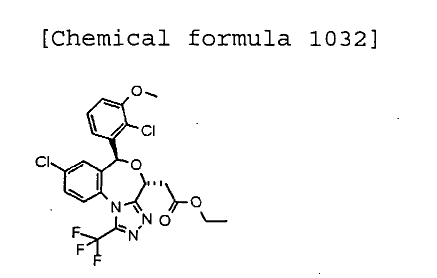

Ethyl 2-[trans-7-chloro-5-(2-chloro-3-methoxyphenyl)-2-thioxo-1,2,3,5-tetrahydro-4,1-benzoxazepin-3-yl]acetate (667 mg) was dissolved in tetrahydrofuran (12 mL). To the resulting solution was added hydrazine monohydrate (0.147 mL). The resulting mixture was stirred at room temperature for one hour. To the reaction mixture was added 1,2-dichloroethane (20 mL). Then, trifluoroacetic anhydride (1.07 mL) and trifluoroacetic acid (2.33 mL) were added and the resulting mixture was stirred at room temperature for 0.5 hour. After stirring at 55°C for 2 hours, toluene (20 mL) was added to the reaction mixture, followed by heating under reflux for 1 hour. The reaction mixture was cooled to room temperature and then concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:3) to give the title compound (606 mg).
MS (ESI) m/z : 516 (M+H)⁺.
¹H-NMR (CDCl₃) δ: 1.29 (3H, t, J = 7.1 Hz), 3.26 (1H, dd, J = 16.7, 7.2 Hz), 3.50 (1H, dd, J = 16.7, 6.3 Hz), 3.91 (3H, s), 4.17-4.23 (2H, m), 4.96 (1H, dd, J = 7.2, 6.3 Hz), 5.56 (1H, s), 6.74-6.74 (1H, m), 7.00-7.02 (1H, m), 7.34-7.36 (1H, m), 7.40 (1H, t, J = 7.9 Hz), 7.54-7.56 (2H, m).

### Example 513

Ethyl 2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetate (isomer A) Ethyl 2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetate (isomer B)

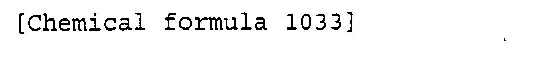

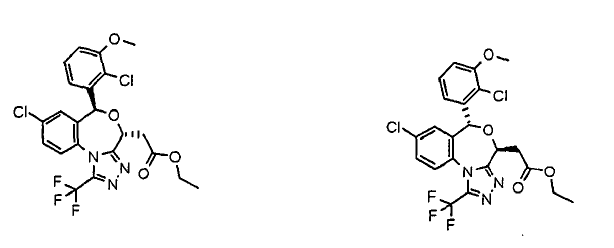

The racemic compound (606 mg) was optically resolved using an optically active column (CHIRALPAK-AD; 5 cm × 50 cm) into the isomer A (261 mg) and isomer B (240 mg). Flow rate: 50 mL/min
Developing solvent: 10% iso-propanol-n-hexane
Retention time: isomer A: 45 min., isomer B: 70 min.

### Example 514

2-[(4R,6S)-8-Chloro-6-(2-chloro-3-methoxyphenyl)-1-(trifluoromethyl)-4H,6H-[1,2,4]triazolo[4,3-a][4,1]benzoxazepin-4-yl]aceticacid

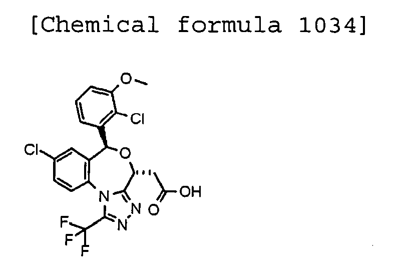

Ethyl 2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetate (261 mg) was dissolved in 1,4-dioxane (5 mL). To the resulting solution was added concentrated hydrochloric acid (1.1 mL). The resulting mixture was stirred at 60°C for 20 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (131 mg).
MS (ESI) m/z : 488 (M+H)⁺.
¹H-NMR (CDCl₃) δ: 3.19-3.23 (1H, m), 3.41-3.43 (1H, m), 3.89 (3H, s), 4.89 (1H, t, J = 6.7 Hz), 5.47 (1H, s), 6.69-6.70 (1H, m), 6.96-6.98 (1H, m), 7.35-7.37 (2H, m), 7.51-7.52 (2H, m).

### Example 515

2-[(4S,6R)-8-Chloro-6-(2-chloro-3-methoxyphenyl)-1-(trifluoromethyl)-4H,6H-[1,2,4]triazolo[4,3-a][4,1]benzoxazepin-4-yl]acetic acid

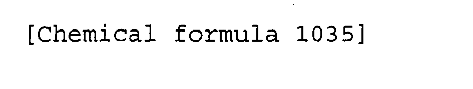

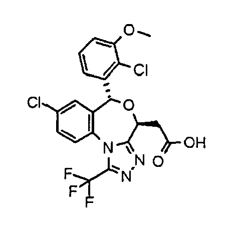

Ethyl 2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetate (240 mg) was dissolved in 1,4-dioxane (5 mL). To the resulting solution was added concentrated hydrochloric acid (1.1 mL). The resulting mixture was stirred at 60°C for 16 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (185 mg).
MS (ESI) m/z : 488 (M+H)⁺.
¹H-NMR coincided completely with that of 2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetic acid.

### Referential Example 335

2,3-dihydro-1,4-benzdioxin-5-ylmethanol

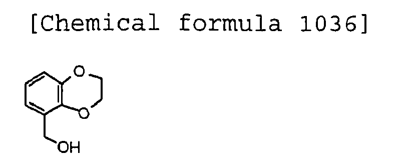

Lithium aluminum hydride (3.03 g) was suspended in tetrahydrofuran (30 mL). At 0°C, a solution of 1,4-benzodioxane-5-carboxylic acid (2.50 g) in tetrahydrofuran (20 mL) was slowly added dropwise to the resulting suspension under stirring. The stirring was continued as was for 30 minutes. To the reaction mixture was added an aqueous solution of potassium sodium tartrate and the resulting mixture was concentrated. The residue was extracted with ethyl acetate. The organic layer was dried over anhydrous sodium sulfate. The filtrate was concentrated to give the title compound (1.56 g).
MS m/z : 149 (M-OH)⁺.
¹H-NMR (CDCl₃) δ: 4.26-4.28 (2H, m), 4.31-4.33 (2H, m), 4.67 (2H, s), 6.82-6.85 (3H, m).

### Referential Example 336

2,3-dihydro-1,4-benzdioxin-5-carbaldehyde

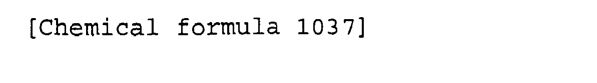

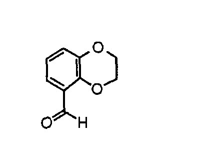

2,3-Dihydro-1,4-benzdioxin-5-ylmethanol (1.56 g) was dissolved in dichloromethane (30 mL). To the resulting solution was added manganese(IV) dioxide (7.97 g) and the mixture was stirred overnight at room temperature. Manganese dioxide was filtered out using celite and the filtrate was concentrated to give the title compound (1.50 g).
¹H-NMR (CDCl₃) δ: 4.32-4.33 (2H, m), 4.38-4.40 (2H, m), 6.91 (1H, td, J = 7.9, 0.7 Hz), 7.09 (1H, dd, J = 7.9, 1.6 Hz), 7.39 (1H, dd, J = 7.9, 1.6 Hz), 10.37 (1H, d, J = 0.7 Hz).

### Referential Example 337

tert-Butyl 4-chloro-2-[2,3-dihydro-1,4-benzdioxin-5-yl(hydroxy)methyl]phenylcarbamate

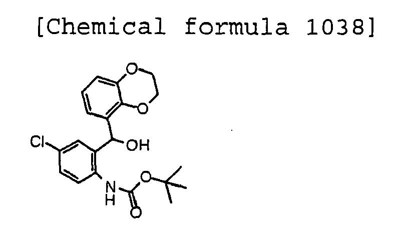

tert-Butyl 4-chlorophenylcarbamate (2.18 g) was dissolved in tetrahydrofuran (30 mL). To the resulting solution was added sec-butyl lithium (hexane solution) (23.1 mL) at -78°C in a nitrogen atmosphere. The resulting mixture was stirred at -20°C for 2.5 hours. To the reaction mixture was slowly added dropwise a tetrahydrofuran solution (20 mL) of 2,3-dihydro-1,4-benzdioxin-5-carbaldehyde (1.50 g). After stirring at - 20°C for further 2 hours, a saturated aqueous solution of ammonium chloride was added and the resulting mixture was extracted with ethyl acetate. The extract was washed with saturated brine. The organic layer was dried over anhydrous sodium sulfate. After concentration of the filtrate, the residue thus obtained was purified by silica gel column (ethyl acetate:hexane=1:4) to give the title compound (2.17 g).
¹H-NMR (CDCl₃) δ: 1.50 (9H, s), 3.14 (1H, d, J = 5.4 Hz), 4.28-4.34 (4H, m), 6.03 (1H, d, J = 5.4 Hz), 6.78-6.80 (1H, m), 6.86-6.88 (2H, m), 7.08 (1H, d, J = 2.4 Hz), 7.23 (1H, dd, J = 8.8, 2.4 Hz), 7.91-7.93 (2H, m).

### Referential Example 338

(2-Amino-5-chlorophenyl)(2,3-dihydro-1,4-benzdioxin-5-yl)methanol

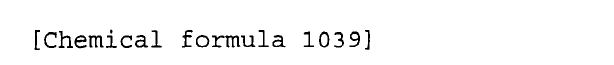

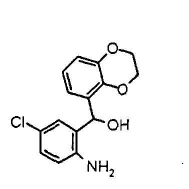

tert-Butyl 4-chloro-2-[2,3-dihydro-1,4-benzdioxin-5-yl(hydroxy)methyl]phenylcarbamate (2.15 g) was dissolved in 1,4-dioxane (20 mL). To the resulting solution was added concentrated hydrochloric acid (10 mL). The resulting mixture was stirred at room temperature for 6 hours. The reaction mixture was neutralized with a 5M aqueous sodium hydroxide solution and extracted with ethyl acetate. The organic layer was dried over anhydrous sodium sulfate. The filtrate was then concentrated to give the title compound (1.71 g).

### Referential Example 339

{5-Chloro-2-[(2,4-dimethoxybenzyl)amino]phenyl}(2,3-dihydro-1,4-benzdioxin-5-yl)methanol

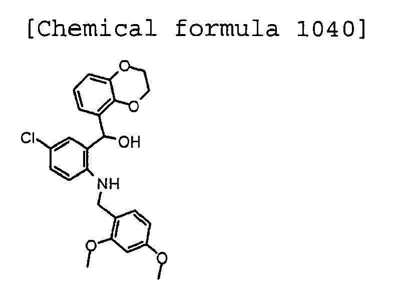

(2-Amino-5-chlorophenyl)(2,3-dihydro-1,4-benzdioxin-5-yl)methanol (1.60 g) and 2,4-dimethoxybenzaldehyde (1.09 g) were dissolved in acetic acid (30 mL). The resulting solution was stirred at room temperature for 20 minutes.
To the reaction mixture was added sodium borohydride (0.62 g) at 0°C. The reaction mixture was stirred at room temperature for 0.5 hour. The reaction mixture was concentrated. The residue was diluted with ethyl acetate and washed with a saturated aqueous solution of sodium bicarbonate and saturated brine. The organic layer was dried over anhydrous sodium sulfate. The filtrate was then concentrated to give the title compound (2.86 g).

### Referential Example 340

Ethyl (E)-4-[4-chloro-2-[2,3-dihydro-1,4-benzdioxin-5-yl(hydroxy)methyl](2,4-dimethoxybenzyl)anilino]-4-oxo-2-butenoate

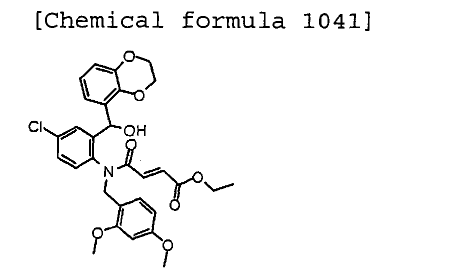

{5-Chloro-2-[(2,4-dimethoxybenzyl)amino]phenyl}(2,3-dihydro-1,4-benzdioxin-5-yl)methanol (2.45 g) was dissolved in dichloromethane (50 mL). Sodium hydrogen carbonate (0.65 g) and a methylene chloride solution (20 mL) of monoethyl chlorofumarate (1.08 g) prepared separately were added dropwise to the resulting solution at 0°C. After stirring overnight at room temperature, the reaction mixture was diluted with chloroform and washed with a saturated aqueous solution of sodium bicarbonate and saturated brine. The organic layer was dried over anhydrous sodium sulfate. The filtrate was then concentrated to give the title compound (3.65 g).

### Referential Example 341

Ethyl 2-[trans-7-chloro-5-(2,3-dihydro-1,4-benzdioxin-5-yl)-1-(2,4-dimethoxybenzyl)-2-oxo-1,2,3,5-tetrahydro-4,1-benzoxazepin-3-yl]acetate

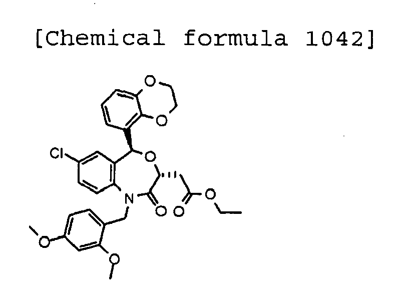

Ethyl (E)-4-[4-chloro-2-[2,3-dihydro-1,4-benzdioxin-5-yl(hydroxy)methyl](2,4-dimethoxybenzyl)anilino]-4-oxo-2-butenoate (3.15 g) was dissolved in ethanol (60 mL). To the resulting solution was added potassium carbonate (0.92 g). The resulting mixture was stirred overnight at room temperature. The reaction mixture was concentrated. The residue was diluted with chloroform and washed with saturated brine. The organic layer was dried over anhydrous sodium sulfate. After concentration of the filtrate, the residue thus obtained was dissolved in ethanol (60 mL). To the resulting solution was added 1,8-diazabicyclo[5,4,0]undec-7-ene (1.24 mL). The resulting mixture was heated under reflux overnight. The reaction mixture was concentrated. The residue was diluted with chloroform and washed with saturated brine. The organic layer was dried over anhydrous sodium sulfate. After concentration of the filtrate, the residue thus obtained was purified by silica gel column (ethyl acetate:hexane=1:2) to give the title compound (1.18 g).
¹H-NMR (CDCl₃) δ: 1.25 (3H, t, J = 7.1 Hz), 2.78 (1H, dd, J = 16.6, 5.6 Hz), 3.10 (1H, dd, J = 16.5, 7.9 Hz), 3.64 (3H, s), 3.77 (3H, s), 3.92-3.96 (2H, m), 4.10-4.16 (4H, m), 4.48 (1H, dd, J = 7.9, 5.6 Hz), 4.93 (1H, d, J = 15.1 Hz), 5.36 (1H, d, J = 15.1 Hz), 5.86 (1H, s), 6.36 (1H, d, J = 2.2 Hz), 6.40 (1H, dd, J = 8.3, 2.2 Hz), 6.61-6.62 (1H, m), 6.87 (1H, dd, J = 8.3, 1.8 Hz), 6.93 (1H, t, J = 7.8 Hz), 7.10-7.12 (1H, m), 7.23-7.26 (1H, m), 7.28-7.29 (2H, m).

### Referential Example 342

Ethyl 2-[trans-7-chloro-5-(2,3-dihydro-1,4-benzdioxin-5-yl)-2-oxo-1,2,3,5-tetrahydro-4,1-benzoxazepin-3-yl]acetate

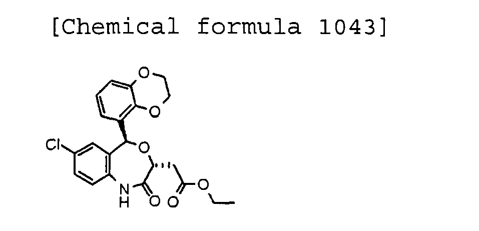

Ethyl 2-[trans-7-chloro-5-(2,3-dihydro-1,4-benzdioxin-5-yl)-1-(2,4-dimethoxybenzyl)-2-oxo-1,2,3,5-tetrahydro-4,1-benzoxazepin-3-yl]acetate (1.18 g) was dissolved in acetone (40 mL). To the resulting solution was added an aqueous solution (10 mL) of ceric(IV) ammonium nitrate (3.42 g) at 0°C. The resulting mixture was stirred at room temperature for 1.5 hours. To the reaction mixture was added a saturated aqueous solution of sodium bicarbonate, followed by extraction with ethyl acetate.
The extract was washed with saturated brine. The organic layer was dried over anhydrous sodium sulfate. The filtrate was then concentrated and the residue was purified by silica gel column (ethyl acetate:hexane=1:2) to give the title compound (0.47 g).
¹H-NMR (CDCl₃) δ: 1.22-1.25 (3H, m), 2.79 (1H, dd, J = 16.4, 6.8 Hz), 3.04 (1H, dd, J = 16.2, 6.5 Hz), 4.08-4.23 (10H, m), 4.65 (1H, dd, J = 6.8, 6.5 Hz), 6.16 (1H, s), 6.76-6.77 (1H, m), 6.90-6.92 (2H, m), 6.95-6.97 (1H, m), 7.06 (1H, d, J = 8.5 Hz), 7.25-7.28 (1H, m), 9.36 (1H, s).

### Referential Example 343

Ethyl 2-[trans-7-chloro-5-(2,3-dihydro-1,4-benzdioxin-5-yl)-2-thioxo-1,2,3,5-tetrahydro-4,1-benzoxazepin-3-yl]acetate

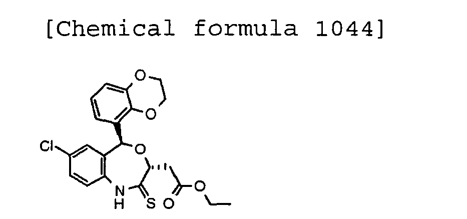

Ethyl 2-[trans-7-chloro-5-(2,3-dihydro-1,4-benzdioxin-5-yl)-2-oxo-1,2,3,5-tetrahydro-4,1-benzoxazepin-3-yl]acetate (467 mg) was dissolved in tetrahydrofuran (10 mL). To the resulting solution were added sodium hydrogen carbonate (197 mg) and diphosphorus pentasulfide (522 mg). The resulting mixture was stirred at room temperature for 5 hours. The reaction mixture was diluted with ethyl acetate and washed with saturated brine. The organic layer was dried over anhydrous sodium sulfate. After concentration of the filtrate, the residue thus obtained was purified by silica gel column (ethyl acetate:hexane=1:2) to give the title compound (289 mg). ¹H-NMR (CDCl₃) δ: 1.21-1.27 (3H, m), 2.96 (1H, dd, J = 16.4, 6.5 Hz), 3.26 (1H, dd, J = 16.4, 7.3 Hz), 4.08-4.21 (6H, m), 4.70 (1H, dd, J = 7.3, 6.5 Hz), 6.08 (1H, s), 6.71-6.75 (1H, m), 6.89-6.96 (2H, m), 7.06-7.08 (1H, m), 7.11 (1H, d, J = 8.3 Hz), 7.34 (1H, dd, J = 8.3, 2.4 Hz), 10.36 (1H, s).

### Example 516

Ethyl 2-[trans-8-chloro-6-(2-chloro-3-methoxyphenyl)-1-(trifluoromethyl)-4H,6H-[1,2,4]triazolo[4,3-a][4,1]benzoxazepin-4-yl]acetate

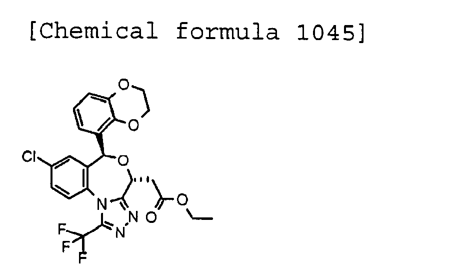

Ethyl 2-[trans-7-chloro-5-(2,3-dihydro-1,4-benzdioxin-5-yl)-2-thioxo-1,2,3,5-tetrahydro-4,1-benzoxazepin-3-yl]acetate (284 mg) was dissolved in tetrahydrofuran (6 mL). To the resulting solution was added hydrazine monohydrate (0.064 mL). The resulting mixture was stirred at room temperature for one hour. To the reaction mixture was added 1,2-dichloroethane (10 mL), followed by the addition of trifluoroacetic anhydride (0.46 mL) and trifluoroacetic acid (1.01 mL). The resulting mixture was stirred at room temperature for 0.5 hour.
After stirring at 55°C for 2 hours, toluene (10 mL) was added and the mixture was heated under reflux for one hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:3) to give the title compound (55.6 mg).
MS (ESI) m/z : 510 (M+H)⁺.
¹H-NMR (CDCl₃) δ: 1.28 (3H, t, J = 7.1 Hz), 3.23 (1H, dd, J = 16.6, 7.1 Hz), 3.48 (1H, dd, J = 16.8, 6.6 Hz), 4.00-4.02 (2H, m), 4.11-4.22 (4H, m), 4.93 (1H, dd, J = 7.1, 6.6 Hz), 5.51 (1H, s), 6.92-6.99 (3H, m), 7.16-7.18 (1H, m), 7.53-7.53 (2H, m).

### Example 517

2-[trans-8-Chloro-6-(2-chloro-3-methoxyphenyl)-1-(trifluoromethyl)-4H,6H-[1,2,4]triazolo[4,3-a][4,1]benzoxazepin-4-yl]acetic acid

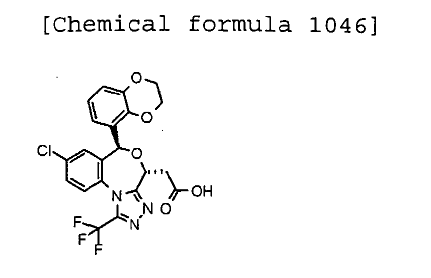

Ethyl 2-[trans-8-chloro-6-(2-chloro-3-methoxyphenyl)-1-(trifluoromethyl)-4H,6H-[1,2,4]triazolo[4,3-a][4,1]benzoxazepin-4-yl]acetate (55.6 mg) was dissolved in 1,4-dioxane (5 mL). To the resulting solution was added concentrated hydrochloric acid (1.1 mL). The resulting mixture was stirred at 60°C for 23 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (36.4 mg).
MS (ESI) m/z : 482 (M+H)⁺.
¹H-NMR (CDCl₃) δ: 3.10-3.14 (1H, m), 3.38 (1H, dd, J = 16.2, 7.4 Hz), 3.99-4.00 (2H, m), 4.12-4.13 (2H, m), 4.85-4.87 (1H, m), 5.46 (1H, s), 6.88-6.95 (3H, m), 7.17 (1H, d, J = 7.3 Hz), 7.46 (1H, dd, J = 8.5, 2.0 Hz), 7.51 (1H, d, J = 8.5 Hz).

### Referential Example 344

Ethyl 6-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetyl}amino)-5-oxohexanoate

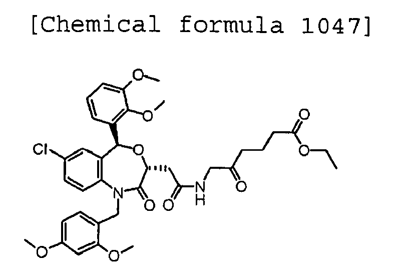

To an N,N-dimethylformamide solution (37.0 mL) of 2-[(trans)-7-Chloro-1-(2,4-dimethoxybenzyl)-5-(2,3-dimethoxyphenyl)-2-oxo-1,2,3,5-tetrahydro-4,1-benzoxazepin-3-yl]acetic acid (2.00 g) were added ethyl 6-amino-5-oxohexanoate hydrochloride (1.13 g), 1-ethyl-3-(3-dimethylaminopropyl)carbodiimide hydrochloride (1.06 g), 1-hydroxybenzotriazole (0.28 g), and triethylamine(0.62 mL). The resulting mixture was stirred at room temperature for 9.5 hours. The solvent was distilled off under reduced pressure. To the residue were added ethyl acetate and distilled water and the layers separated. The organic layer was washed with saturated brine and dried over anhydrous sodium sulfate. The solvent was distilled off under reduced pressure and the residue thus obtained was purified by column chromatography (dichloromethane:methanol=20:1) to give the title compound (1.00 g).
MS (ESI) m/z : 665 (M + H)⁺.
¹H-NMR (CDCl₃) δ: 1.88-1.97 (2H, m), 2.31-2.38 (2H, m), 2.47-2.54 (2H, m), 2.69-2.79 (1H, m), 2.90-3.01 (1H, m), 3.18 (3H, s), 3.66 (3H, s), 3.67 (3H, s), 3.76 (3H, s), 3.85 (3H, s), 4.02-4.17 (3H, m), 4.43-4.51 (1H, m), 4.82 (1H, d, J = 14.7 Hz), 5.50 (1H, d, J = 15.0 Hz), 5.97 (1H, s), 6.37-6.43 (2H, m), 6.53-6.55 (1H, m), 6.57-6.64 (1H, m), 6.90-6.97 (1H, m), 7.11-7.18 (2H, m), 7.34-7.20 (3H, m).

### Referential Example 345

Methyl 4-(2-{[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)butanoate

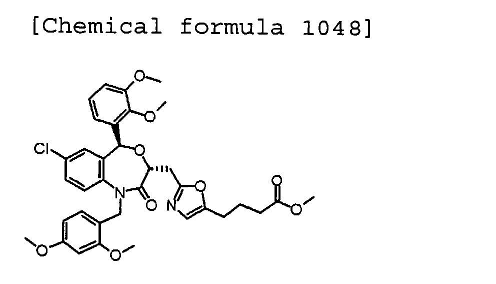

In a similar manner to that employed for the synthesis of methyl 3-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)propanoate, the title compound (787 mg) was obtained from ethyl 6-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetyl}amino)-5-oxohexanoate (1.00 g) by using phosphorus oxychloride (205 µl).
MS (ESI) m/z : 665 (M + H)⁺.
¹H-NMR (CDCl₃) δ: 1.91-2.00 (2H, m), 2.33-2.42 (2H, m), 2.64-2.70 (2H, m), 3.20 (3H, s), 3.21-3.28 (1H, m), 3.34-3.45 (2H, m), 3.67 (3H, s), 3.67 (3H, s), 3.76 (3H, s), 3.85 (3H, s), 4.50-4.59 (1H, m), 4.85 (1H, d, J = 15.0 Hz), 5.50 (1H, d, J = 15.0 Hz), 6.00 (1H, s), 6.37-6.42 (2H, m), 6.50-6.52 (1H, m), 6.62-6.65 (1H, m), 6.92 (1H, dd, J = 8.1, 1.5 Hz), 6.98-7.02 (1H, m), 7.07-7.13 (1H, m), 7.33-7.23 (3H, m).

### Referential Example 346

Methyl 4-(2-{[(trans)-7-chloro-5-(2,3-dimethoxyphenyl)-2-oxo-1,2,3,5-tetrahydro-4,1-benzoxazepin-3-yl]methyl}-1,3-oxazol-5-yl)butanoate

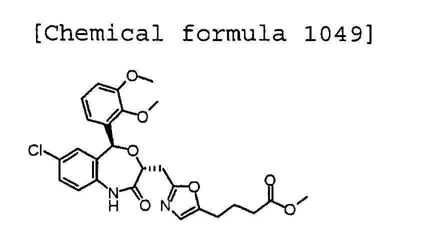

To an acetone-water mixed solution (24 mL - 6 mL) of methyl 4-(2-{[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)butanoate (780 mg) was added diammonium cerium(IV) nitrate (1286 mg). The resulting mixture was stirred at room temperature for 3 hours. Diammonium cerium(IV) nitrate (643 mg) was added again and the resulting mixture was stirred for 2 hours. Diammonium cerium(IV) nitrate (321 mg) was added once again. After stirring for 30 minutes, a saturated aqueous solution of sodium hydrogen carbonate was added. The resulting mixture was filtered through celite and washed sufficiently with ethyl acetate. To the filtrates combined, ethyl acetate was added and the layers separated. The organic layers were combined, washed with saturated brine and dried over anhydrous sodium sulfate. The organic solvent was distilled off under reduced pressure. The residue was purified by flash column chromatography ("Ultra pack B", Yamazen Corporation) to give the title compound (604 mg).
MS (ESI) m/z : 515 (M + H)⁺.
¹H-NMR (CDCl₃) δ: 1.88-1.98 (2H, m), 2.31-2.42 (2H, m), 2.60-2.71 (2H, m), 3.17-3.29 (1H, m), 3.35-3.44 (1H, m), 3.62 (3H, s), 3.67 (3H, s), 3.88 (3H, s), 4.66-4.71 (1H, m), 6.24 (1H, s), 6.59-6.68 (2H, m), 6.92-7.02 (3H, m), 7.08-7.15 (1H, m), 7.24-7.29 (1H, m), 7.84-7.80 (1H, m).

### Referential Example 347

Methyl 4-(2-{[(trans)-7-chloro-5-(2,3-dimethoxyphenyl)-2-thioxo-1,2,3,5-tetrahydro-4,1-benzoxazepin-3-yl]methyl}-1,3-oxazol-5-yl)butanoate

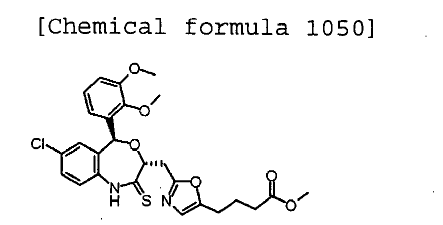

To a tetrahydrofuran solution (24 mL) of methyl 4-(2-{[(trans)-7-chloro-5-(2,3-dimethoxyphenyl)-2-oxo-1,2,3,5-tetrahydro-4,1-benzoxazepin-3-yl]methyl}-1,3-oxazol-5-yl)butanoate (780 mg) were added sodium hydrogen carbonate (568 mg) and diphosphorus pentasulfide (571 mg). The resulting mixture was stirred at 40°C for one hour. To the reaction mixture were added a saturated aqueous solution of sodium hydrogen carbonate and ethyl acetate and the layers separated. The organic layer thus obtained was washed with saturated brine and dried over anhydrous sodium sulfate. The organic solvent was distilled off under reduced pressure. The residue was purified by flash column chromatography ("Ultra pack B-L", Yamazen Corp.) to give the title compound (311 mg).
MS (ESI) m/z : 531 (M + H)⁺.
¹H-NMR (CDCl₃) δ: 1.91-1.99 (2H, m), 2.33-2.38 (2H, m), 2.63-2.69 (2H, m), 3.38-3.46 (1H, m), 3.51-3.58 (1H, m), 3.61 (3H, s), 3.68 (3H, s), 3.88 (3H, s), 4.73-4.78 (1H, m), 6.15 (1H, s), 6.61-6.64 (2H, m), 6.90-6.97 (2H, m), 7.04-7.14 (2H, m), 7.36-7.29 (1H, m), 9.55 (1H, s).

### Referential Example 348

Methyl 4-(2-{[(trans)-7-chloro-5-(2,3-dimethoxyphenyl)-2-[hydrazono]-1,5-dihydro-4,1-benzoxazepin3(3H)-yl]methyl}-1,3-oxazol-5-yl)butanoate

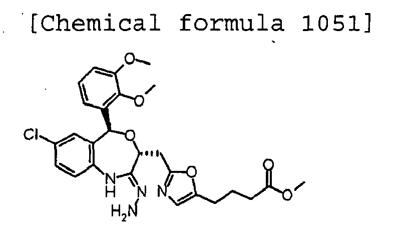

In a similar manner to that employed for the synthesis of methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate, the title compound (305 mg) was obtained from methyl 4-(2-{[(trans)-7-chloro-5-(2,3-dimethoxyphenyl)-2-thioxo-1,2,3,5-tetrahydro-4,1-benzoxazepin-3-yl]methyl}-1,3-oxazol-5-yl)butanoate (306 mg) by using hydrazine monohydrate (168 µl).
MS (ESI) m/z : 529 (M + H)⁺.
¹H-NMR (CDCl₃) δ: 1.91-2.00 (2H, m), 2.33-2.38 (2H, m), 2.63-2.70 (3H, m), 3.17-3.26 (1H, m), 3.30-3.37 (1H, m), 3.53 (3H, s), 3.67 (3H, s), 3.87 (3H, s), 4.52 (2H, s), 4.80-4.85 (1H, m), 6.18 (1H, s), 6.53 (1H, d, J = 2.5 Hz), 6.64-6.66 (1H, m), 6.79 (1H, s), 6.84-6.88 (1H, m), 6.91-6.95 (1H, m), 6.99-7.03 (1H, m), 7.08-7.13 (1H, m), 7.24-7.15 (1H, m).

### Example 518

Methyl 4-(2-{[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate

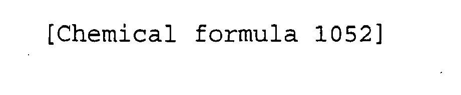

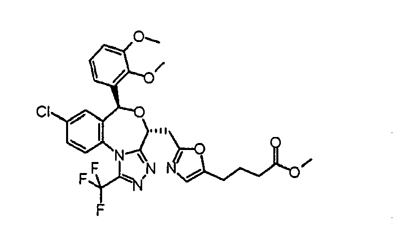

In a similar manner to that employed for the synthesis of methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate, the title compound (312 mg) was obtained from methyl 4-(2-{[(trans)-7-chloro-5-(2,3-dimethoxyphenyl)-2-[hydrazono]-1,5-dihydro-4,1-benzoxazepin3(3H)-yl]methyl}-1,3-oxazol-5-yl)butanoate (304 mg) by using trifluoroacetic anhydride (239 µl) and trifluoroacetic acid (853 µl).
MS (ESI) m/z : 607 (M + H)⁺.
¹H-NMR (CDCl₃) δ: 1.92-2.01 (2H, m), 2.34-2.39 (2H, m), 2.66-2.72 (2H, m), 3.43 (3H, s), 3.63-3.70 (1H, m), 3.67 (3H, s), 3.84-3.90 (1H, m), 3.85 (3H, s), 4.97-5.02 (1H, m), 5.57 (1H, s), 6.65-6.67 (1H, m), 6.82-6.84 (1H, m), 6.95-7.00 (1H, m), 7.06-7.10 (1H, m), 7.12-7.18 (1H, m), 7.53-7.51 (2H, m).

### Example 519

Methyl 4-(2-{((4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate
Isomer A

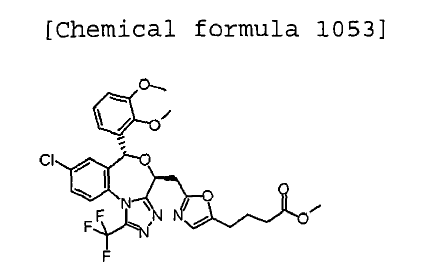

Methyl 4-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate
Isomer B

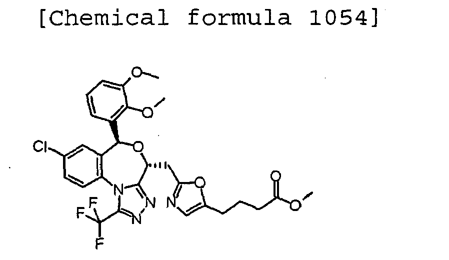

Methyl 4-(2-{[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate(309 mg) was optically resolved using HPLC equipped with CHIRALPAK IA (Daicel Chemical). As the title compounds, the isomer A (138 g) with a short retention time and the isomer B (81 mg) with a long retention time were separately obtained by using 70% ethanol-hexane as a solvent at a flow rate of 10 mL/min.

### Example 520

Methyl 4-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate

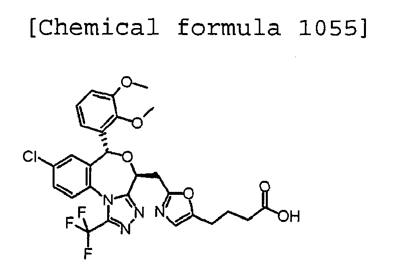

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (126 mg) was obtained from methyl 4-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate(137 mg) by using potassium carbonate (1.88 mg).
MS (ESI) m/z : 593 (M + H)⁺.
¹H-NMR (CDCl₃) δ: 1.89-2.02 (2H, m), 2.34-2.40 (2H, m), 2.66-2.72 (2H, m), 3.43 (3H, s), 3.59-3.70 (1H, m), 3.85 (3H, s), 3.85-3.92 (1H, m), 4.98-5.04 (1H, m), 5.57 (1H, s), 6.65 (1H, s), 6.82-6.84 (1H, m), 6.99-6.94 (1H, m), 7.18-7.07 (2H, m), 7.55-7.49 (2H, m).
Elemental analysis for: C₂₇H₂₄ClF₃N₄O₆·0.5H₂O·0.2 n-hexane Calculated: C, 54.70; H, 4.53; N, 9.05.
Found: C, 54.85; H, 4.57; N, 8.78.

### Example 521

Methyl 4-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate

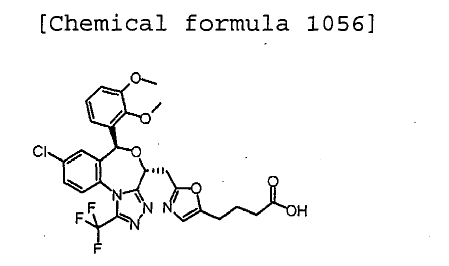

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (79 mg) was obtained from methyl 4-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate (81 mg) by using potassium hydrogen carbonate (110 mg).
MS (ESI) m/z : 593 (M + H)⁺.
¹H-NMR (CDCl₃) δ: 1.90-2.03 (2H, m), 2.35-2.42 (2H, m), 2.67-2.74 (2H, m), 3.43 (3H, s), 3.60-3.71 (1H, m), 3.80-3.93 (1H, m), 3.85 (3H, s), 4.99-5.04 (1H, m), 5.57 (1H, s), 6.66 (1H, s), 6.83 (1H, s), 6.94-7.00 (1H, m), 7.07-7.19 (2H, m), 7.55-7.49 (2H, m).
Elemental analysis for: C₂₇H₂₄ClF₃N₄O₆·0.5H₂O·0.25 n-hexane Calculated: C, 54.90; H, 4.61; N, 8.99.
Found: C, 54.92; H, 4.52; N, 8.65.

### Referential Example 349

2-[(trans)-7-Chloro-1-(2,4-dimethoxybenzyl)-5-(2,3-dimethoxyphenyl)-2-oxo-1,2,3,5-tetrahydro-4,1-benzoxazepin-3-yl] acetamide

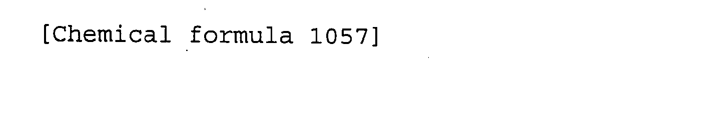

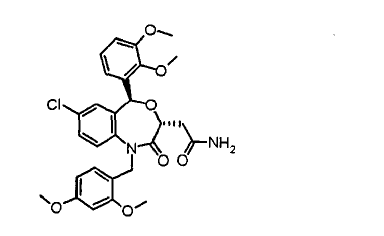

To a tetrahydrofuran solution (30 mL) of 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetic acid (2.20 g) were added N-methylmorpholine (0.51 mL) and ethyl chloroformate (0.44 mL) under ice cooling. The resulting mixture was stirred as was for 2 hours. To the reaction mixture was added dropwise aqueous ammonia (20 mL) and the resulting mixture was stirred for 30 minutes. After the reaction mixture was warmed to room temperature, the solid formed in the reaction system was filtered and washed with diethyl ether. The resulting solid was dried under reduced pressure to give the title compound (1.50 g).
MS (ESI) m/z : 541 (M + H)⁺.
¹H-NMR (CDCl₃) δ: 2.68-2.75 (1H, m), 2.90-2.97 (1H, m), 3.19 (3H, s), 3.67 (3H, s), 3.76 (3H, s), 3.86 (3H, s), 4.45-4.50 (1H, m), 4.84 (1H, d, J = 15.0 Hz), 5.29 (1H, s), 5.50 (1H, d, J = 15.0 Hz), 5.91 (1H, s), 5.99 (1H, s), 6.38-6.44 (2H, m), 6.54-6.56 (1H, m), 6.91-6.97 (1H, m), 7.12-7.18 (2H, m), 7.32-7.27 (2H, m), 7.24-7.20 (1H, m).

### Referential Example 350

2-[(trans)-7-Chloro-1-(2,4-dimethoxybenzyl)-5-(2,3-dimethoxyphenyl)-2-oxo-1,2,3,5-tetrahydro-4,1-benzoxazepin-3-yl]ethanethioamide

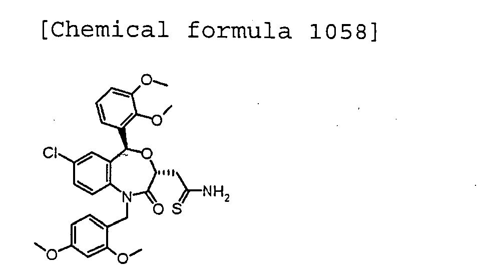

To a tetrahydrofuran solution (11 mL) of 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetamide (594 mg) was added Lawesson's reagent (310 mg) at room temperature. The resulting mixture was stirred at room temperature for 4 hours. The reaction mixture was subjected to column chromatography (dichloromethane:methanol=20:1) and then, solidified from diethyl ether-n-hexane. The solid thus obtained was collected by filtration, washed with n-hexane and dried under reduced pressure to give the title compound (610 mg). MS (ESI) m/z : 557 (M + H)⁺.
¹H-NMR (CDCl₃) δ: 3.10-3.16 (1H, m), 3.19 (3H, s), 3.31-3.43 (1H, m), 3.67 (3H, s), 3.76 (3H, s), 3.86 (3H, s), 4.50-4.55 (1H, m), 4.84 (1H, d, J = 15.0 Hz), 5.48 (1H, d, J = 15.0 Hz), 5.98 (1H, s), 6.39-6.42 (2H, m), 6.55-6.58 (1H, m), 6.91-6.98 (1H, m), 7.12-7.25 (4H, m), 7.28-7.34 (2H, m), 7.40 (1H, s), 7.66 (1H, s).

### Referential Example 351

Methyl 3-(2-{[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-thiazol-4-yl)propanoate

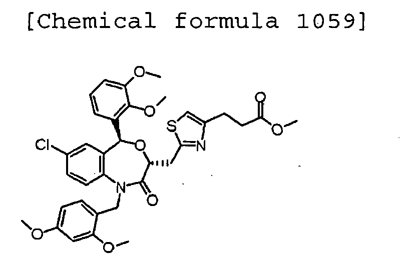

In a similar manner to that employed for the synthesis of ethyl 3-(2-{[(trans)-7-chloro-5-(2,3-dimethoxyphenyl)-2-oxo-1,2,3,5-tetrahydro-4,1-benzothiazepin-3-3yl]methyl}-1,3-thiazol-4-yl)propanoate, the title compound (554 mg) was obtained from 2-[7-chloro-1-(2,4-dimethoxybenzyl)-5-(2,3-dimethoxyphenyl)-2-oxo-1,2,3,5-tetrahydro-4,1-benzoxazepin-3-yl]ethanethioamide (600 mg) by using methyl 5-bromo-4-oxopentanoate (225 mg).
MS (ESI) m/z : 667 (M + H)⁺. ¹H-NMR (CDCl₃) δ: 2.67-2.73 (2H, m), 3.01-3.07 (2H, m), 3.18 (3H, s), 3.45-3.52 (1H, m), 3.65 (3H, s), 3.67 (3H, s), 3.76 (3H, s), 3.85 (3H, s), 4.38-4.42 (1H, m), 4.83 (1H, d, J = 15.0 Hz), 5.51 (1H, d, J = 15.0 Hz), 6.02 (1H, s), 6.37-6.42 (2H, m), 6.48-6.58 (1H, m), 6.81-6.84 (1H, m), 6.91-6.95 (1H, m), 7.06-7.17 (2H, m), 7.31-7.19 (2H, m).

### Referential Example 352

Methyl 3-(2-{[(trans)-7-chloro-5-(2,3-dimethoxyphenyl)-2-oxo-1,2,3,5-tetrahydro-4,1-benzoxazepin-3-yl]methyl}-1,3-thiazol-4-yl)propanoate

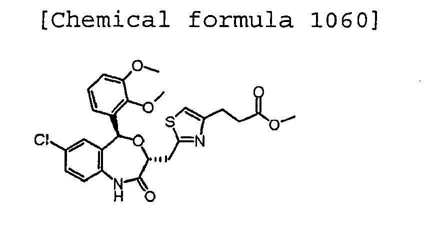

In a similar manner to that employed for the synthesis of methyl 4-(2-{[(trans)-7-chloro-5-(2,3-dimethoxyphenyl)-2-oxo-1,2,3,5-tetrahydro-4,1-benzoxazepin-3-yl]methyl}-1,3-oxazol-5-yl)butanoate, the title compound (187 mg) was obtained from methyl 3-(2-{[7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-thiazol-4-yl)propanoate (292 mg) by using diammonium cerium(IV) nitrate (719 mg).
MS (ESI) m/z : 517 (M + H)⁺.
¹H-NMR (CDCl₃) δ: 2.67-2.73 (2H, m), 3.01-3.07 (2H, m), 3.18 (3H, s), 3.45-3.52 (1H, m), 3.65 (3H, s), 3.67 (3H, s), 3.76 (3H, s), 3.85 (3H, s), 4.38-4.42 (1H, m), 4.83 (1H, d, J = 15.0 Hz), 5.51 (1H, d, J = 15.0 Hz), 6.02 (1H, s), 6.37-6.42 (2H, m), 6.48-6.58 (1H, m), 6.81-6.84 (1H, m), 6.91-6.95 (1H, m), 7.06-7.17 (2H, m), 7.31-7.19 (2H, m).

### Referential Example 353

Methyl 3-(2-{[(trans)-7-chloro-5-(2,3-dimethoxyphenyl)-2-thioxo-1,2,3,5-tetrahydro-4,1-benzoxazepin-3-yl]methyl}-1,3-thiazol-4-yl)propanoate

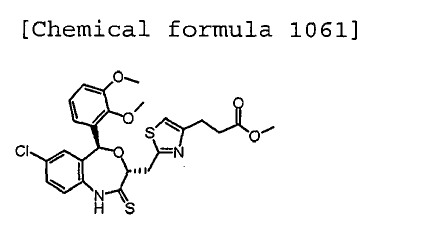

In a similar manner to that employed for the synthesis of methyl 4-(2-{[(trans)-7-chloro-5-(2,3-dimethoxyphenyl)-2-thioxo-1,2,3,5-tetrahydro-4,1-benzoxazepin-3-yl]methyl}-1,3-oxazol-5-yl)butanoate, the title compound (154 mg) was obtained from methyl 3-(2-{[7-chloro-5-(2,3-dimethoxyphenyl)-2-oxo-1,2,3,5-tetrahydro-4,1-benzoxazepin-3-yl]methyl}-1,3-thiazol-4-yl)propanoate (187 mg) by using sodium hydrogen carbonate (176 mg) and diphosphorus pentasulfide (177 mg).
MS (ESI) m/z : 533 (M + H)⁺.
¹H-NMR (CDCl₃) δ: 2.66-2.72 (2H, m), 3.00-3.05 (2H, m), 3.60 (3H, s), 3.63-3.68 (1H, m), 3.65 (3H, s), 3.73-3.82 (1H, m), 3.88 (3H, s), 4.59-4.64 (1H, m), 6.16 (1H, s), 6.60-6.63 (1H, m), 6.81-6.83 (1H, m), 6.93-7.05 (3H, m), 7.09-7.15 (1H, m), 7.34-7.29 (1H, m), 9.55 (1H, s).

### Referential Example 354

Methyl 3-(2-{[(trans)-7-chloro-5-(2,3-dimethoxyphenyl)-2-[hydrazono]-1,5-dihydro-4,1-benzoxazepin3(3H)-yl]methyl}-1,3-thiazol-4-yl)propanoate

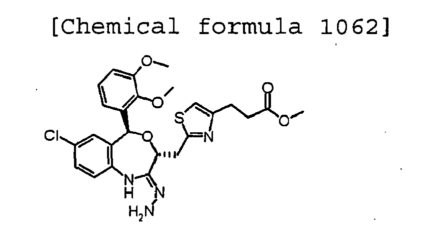

In a similar manner to that employed for the synthesis of methyl 4-(2-{[(trans)-7-chloro-5-(2,3-dimethoxyphenyl)-2-[hydrazono]-1,5-dihydro-4,1-benzoxazepin3(3H)-yl]methyl}-1,3-oxazol-5-yl)butanoate, the title compound (349 mg) was obtained from methyl 3-(2-{[7-chloro-5-(2,3-dimethoxyphenyl)-2-thioxo-1,2,3,5-tetrahydro-4,1-benzoxazepin-3-yl]methyl}-1,3-thiazol-4-yl)propanoate (350 mg) by using hydrazine monohydrate (191 µl).
MS (ESI) m/z : 531 (M + H)⁺.
¹H-NMR (CDCl₃) δ: 2.68-2.73 (2H, m), 3.01-3.07 (2H, m), 3.40-3.50 (1H, m), 3.52 (3H, s), 3.57-3.64 (1H, m), 3.65 (3H, s), 3.87 (3H, s), 4.57 (1H, br s), 4.63-4.68 (1H, m), 6.20 (1H, s), 6.53 (1H, d, J = 2.2 Hz), 6.76 (1H, br s), 6.80-6.86 (2H, m), 6.92-6.97 (1H, m), 7.09-7.21 (2H, m), 7.28-7.23 (1H, m).

### Example 522

Methyl 3-(2-{[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanoate

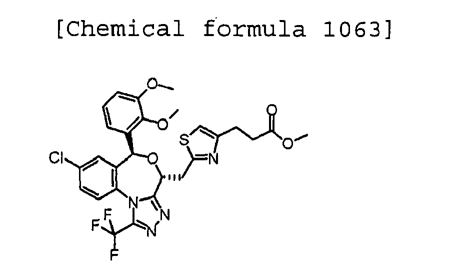

In a similar manner to that employed for the synthesis of ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-4-yl)propanoate, the title compound (375 mg) was obtained from methyl 3-(2-{[7-chloro-5-(2,3-dimethoxyphenyl)-2-[hydrazono]-1,5-dihydro-4,1-benzoxazepin3(3H)-yl]methyl}-1,3-thiazol-4-yl)propanoate (349 mg) by using trifluoroacetic anhydride (274 µl) and trifluoroacetic acid (975 µl).
MS (ESI) m/z : 609 (M + H)⁺.
¹H-NMR (CDCl₃) δ: 2.66-2.71 (2H, m), 3.01-3.06 (2H, m), 3.43 (3H, s), 3.64 (3H, s), 3.86 (3H, s), 3.87-3.93 (1H, m), 4.05-4.12 (1H, m), 4.83-4.88 (1H, m), 5.58 (1H, s), 6.78-6.88 (2H, m), 6.96-7.00 (1H, m), 7.11-7.21 (2H, m), 7.52-7.49 (2H, m).

### Example 523

Methyl 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanoate
Isomer A

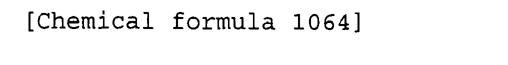

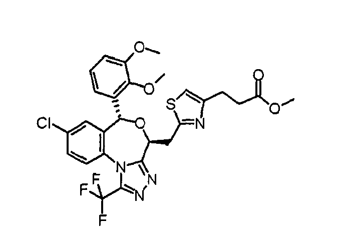

Methyl 3-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanoate
Isomer B

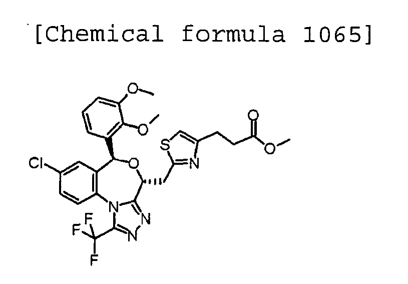

In a similar manner to that employed for the optical resolution of methyl 4-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate, methyl 3-(2-{[(trans)-8-chloro-6-(2,3-dimethoxyphenyl)-1-(trifluoromethyl)-4H,6H-[1,2,4]triazolo[4,3-a] [4,1]benzoxazepin-4-yl]methyl}-1,3-thiazol-4-yl)propanoate (373 mg) was optically resolved by using HPLC equipped with CHIRALPAK IA (Daicel Chemical) to give the title compounds, that is, isomer A (143 mg) with a short retention time and isomer B (186 mg) with a long retention time.

### Example 524

3-(2-{[(4S,6R)-8-Chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanoic acid

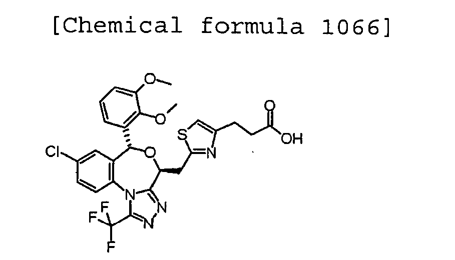

In a similar manner to that employed for the synthesis of 3-(2-{[8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-4-yl)propanic acid, the title compound (105 mg) was obtained from methyl 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanoate(143 mg) by using potassium hydrogen carbonate (390 mg).
MS (ESI) m/z : 595 (M + H)⁺.
¹H-NMR (CDCl₃) δ: 2.64-2.75 (2H, m), 2.96-3.05 (2H, m), 3.43 (3H, s), 3.85 (3H, s), 3.87-3.95 (1H, m), 4.02-4.15 (1H, m), 4.83-4.89 (1H, m), 5.58 (1H, s), 6.81-6.84 (1H, m), 6.87-6.91 (1H, m), 6.95-7.01 (1H, m), 7.14-7.21 (2H, m), 7.55-7.48 (2H, m).
Elemental analysis for: C₂₆H₂₂ClF₃N₄O₅S₂·0.25H₂O·0.25 n-hexane Calculated: C, 53.18; H, 4.22; N, 9.02.
Found: C, 52.91; H, 4.04; N, 8.82.

### Example 525

3-(2-([(4R,6S)-8-Chloro-6-(2,3-dimethoxyphenyl)-1-(trifluoromethyl)-4H,6H-[1,2,4]triazolo[4,3-a][4,1]benzoxazepin-4-yl]methyl)-1,3-thiazol-4-yl)propanoic acid

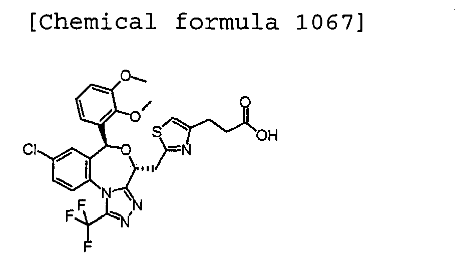

In a similar manner to that employed for the synthesis of 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanic acid, the title compound (104 mg) was obtained from methyl 3-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-thiazol-4-yl)propanoate(186 mg) by using potassium hydrogen carbonate (254 mg).
MS (ESI) m/z : 595 (M + H)⁺.
¹H-NMR (CDCl₃) δ: 2.66-2.73 (2H, m), 2.97-3.05 (2H, m), 3.43 (3H, s), 3.86 (3H, s), 3.87-3.95 (1H, m), 4.07-4.16 (1H, m), 4.82-4.88 (1H, m), 5.58 (1H, s), 6.81-6.84 (1H, m), 6.88-6.91 (1H, m), 6.95-7.02 (1H, m), 7.14-7.21 (2H, m), 7.56-7.49 (2H, m).
Elemental analysis for: C₂₆H₂₂ClF₃N₄O₅S₂·0.25H₂O·0.25 n-hexane
Calculated: C, 53.18; H, 4.22; N, 9.02.
Found: C, 52.86; H, 4.08; N, 8.69.

### Referential Example 355

Ethyl 5-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetyl}amino)-4-oxohexanoate

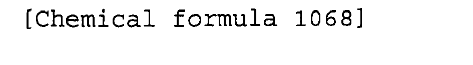

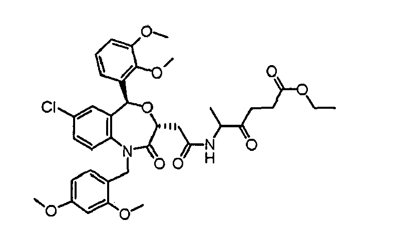

In a similar manner to that employed for the synthesis of ethyl 5-({2-[(trans)-7-chloro-5-(2,3-dimethoxyphenyl)-2-oxo-1,2,3,5-tetrahydro-4,1-benzothiazepin-3-yl]acetyl}amino)-4-oxohexanoate, the title compound (1178 mg) was obtained from 2-[8-chloro-6-(2,3-dimethoxyphenyl)-1-(trifluoromethyl)-4H,6H-[1,2,4]triazolo[4,3-a][4,1]benzothiazepin-4-yl]acetic acid (1800 mg) by using (5S)-5-amino-4-oxohexanoate hydrochloride (905 mg), 1-ethyl-3-(3-dimethylaminopropyl)carbodiimide hydrochloride (955 mg), 1-hydroxybenzotriazole monohydrate (254 mg), and triethylamine (602 µl).
MS (ESI) m/z : 697 (M + H)⁺.
¹H-NMR (CDCl₃) δ: 1.21-1.29 (3H, m), 1.33-1.40 (3H, m), 2.42-3.13 (7H, m), 3.19 (3H, s), 3.66 (3H, s), 3.76 (3H, s), 3.85 (3H, s), 4.08-4.18 (2H, m), 4.38-4.51 (1H, m), 4.52-4.64 (1H, m), 4.79-4.87 (1H, m), 5.41-5.57 (1H, m), 5.95-5.99 (1H, m), 6.36-6.43 (2H, m), 6.48-6.72 (2H, m), 6.87-7.00 (1H, m), 7.08-7.17 (2H, m), 7.33-7.19 (2H, m).

### Referential Example 356

Ethyl 3-(2-{[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-4-methyl-1,3-oxazol-5-yl)propanoate

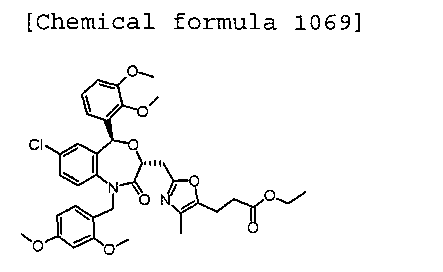

To an N,N-dimethylformamide solution (16.0 mL) of ethyl 5-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetyl}amino)-4-oxohexanoate (1111 mg) was added phosphorus oxychloride (366 mg). The resulting mixture was stirred at 70°C for 45 minutes. After cooling to room temperature, a saturated aqueous solution of sodium hydrogen carbonate and ethyl acetate were added to the reaction mixture and the layers separated. The organic layer thus obtained was washed with saturated brine and dried over anhydrous sodium sulfate. The solvent was distilled off under reduced pressure and the residue thus obtained was purified by flash column chromatography ("Cartridge B-L", Yamazen Corp.) to give the title compound (1038 mg).
MS (ESI) m/z : 679 (M + H)⁺.
¹H-NMR (CDCl₃) δ: 1.20-1.26 (3H, m), 2.07 (3H, s), 2.54-2.64 (2H, m), 2.84-2.98 (3H, m), 3.17-3.24 (1H, m), 3.21 (3H, s), 3.28-3.40 (1H, m), 3.68 (3H, s), 3.76 (3H, s), 3.85 (3H, s), 4.06-4.18 (2H, m), 4.47-4.55 (1H, m), 4.88 (1H, d, J = 15.0 Hz), 5.45 (1H, d, J = 15.0 Hz), 5.98 (1H, d, J = 14.7 Hz), 6.37-6.44 (2H, m), 6.49-6.52 (1H, m), 6.89-6.97 (1H, m), 6.98-7.05 (1H, m), 7.09-7.15 (1H, m), 7.31-7.22 (3H, m).

### Referential Example 357

Ethyl 3-(2-{[(trans)-7-chloro-5-(2,3-dimethoxyphenyl)-2-oxo-1,2,3,5-tetrahydro-4,1-benzoxazepin-3-yl]methyl}-4-methyl-1,3-oxazol-5-yl)propanoate

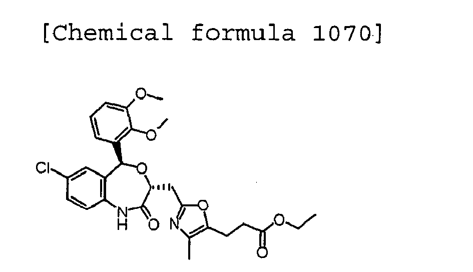

In a similar manner to that employed for the synthesis of methyl 3-(2-{[(trans)-7-chloro-5-(2,3-dimethoxyphenyl)-2-oxo-1,2,3,5-tetrahydro-4,1-benzoxazepin-3-yl]methyl}-1,3-thiazol-4-yl)propanoate, the title compound (343 mg) was obtained from ethyl 3-(2-{[7-chloro-1-(2,4-dimethoxybenzyl)-5-(2,3-dimethoxyphenyl)-2-oxo-1,2,3,5-tetrahydro-4,1-benzoxazepin-3-yl]methyl}-4-methyl-1,3-oxazol-5-yl)propanoate (1051 mg) by using diammonium cerium(IV) nitrate (2800 mg).
MS (ESI) m/z : 529 (M + H)⁺.
¹H-NMR (CDCl₃) δ: 1.22-1.29 (3H, m), 2.05 (3H, s), 2.51-2.58 (2H, m), 2.79-2.95 (2H, m), 3.09-3.23 (1H, m), 3.30-3.38 (1H, m), 3.62 (3H, s), 3.88 (3H, s), 4.08-4.17 (2H, m), 4.60-4.68 (1H, m), 6.23 (1H, s), 6.64-6.73 (1H, m), 6.89-7.20 (3H, m), 7.24-7.31 (1H, m), 8.05-7.81 (1H, m).

### Referential Example 358

Ethyl 3-(2-{[(trans)-7-chloro-5-(2,3-dimethoxyphenyl)-2-thioxo-1,2,3,5-tetrahydro-4,1-benzoxazepin-3-yl]methyl}-4-methyl-1,3-oxazol-5-yl)propanoate

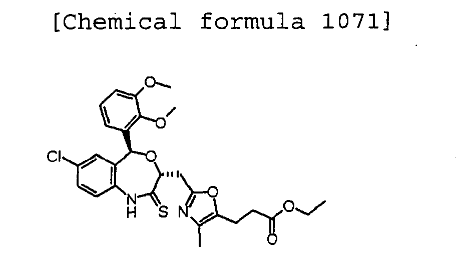

In a similar manner to that employed for the synthesis of methyl 4-(2-{[(trans)-7-chloro-5-(2,3-dimethoxyphenyl)-2-thioxo-1,2,3,5-tetrahydro-4,1-benzoxazepin-3-yl]methyl}-1,3-oxazol-5-yl)butanoate, the title compound (260 mg) was obtained using sodium hydrogen carbonate (313 mg) and diphosphorus pentasulfide (315 mg). MS (ESI) m/z : 545 (M + H)⁺.
¹H-NMR (CDCl₃) δ: 1.22-1.27 (3H, m), 2.04 (3H, s), 2.49-2.66 (2H, m), 2.84-3.08 (2H, m), 3.31-3.52 (2H, m), 3.61 (3H, s), 3.88 (3H, s), 4.09-4.18 (2H, m), 4.66-4.76 (1H, m), 6.14-6.17 (1H, m), 6.59-6.73 (1H, m), 6.88-7.21 (4H, m), 7.29-7.37 (1H, m), 9.62-9.54 (1H, m).

### Example 526

Ethyl 3-(2-{[(trans)-7-chloro-5-(2,3-dimethoxyphenyl)-2-[hydrazono]-1,5-dihydro-4,1-benzoxazepin3(3H)-yl]methyl}-4-methyl-1,3-oxazol-5-yl)propanoate

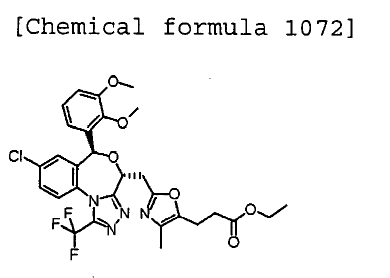

To a tetrahydrofuran solution (9.5 mL) of ethyl 3-(2-{[(trans)-7-chloro-5-(2,3-dimethoxyphenyl)-2-thioxo-1,2,3,5-tetrahydro-4,1-benzoxazepin-3-yl]methyl}-4-methyl-1,3-oxazol-5-yl)propanoate (258 mg) was added hydrazine monohydrate (138 µl) at room temperature. The resulting mixture was stirred for 3 hours The solvent was distilled off under reduced pressure and the residue thus obtained was dissolved to give a toluene-dichloromethane (5 mL - 2 mL) mixed solution. To the resulting solution were added trifluoroacetic anhydride (197 µl) and trifluoroacetic acid (703 µl). The resulting mixture was stirred at 55°C for 3 hours. The temperature was raised to 90°C and stirring was performed for further 3.5 hours. To the reaction mixture was added trifluoroacetic anhydride (197 µl) again and the resulting mixture was stirred at 90°C for 2 hours. The reaction mixture was cooled to room temperature. The solvent was distilled off under reduced pressure. To the residue thus obtained were added dichloromethane and a saturated aqueous solution of sodium hydrogen carbonate and the layers separated. The resulting organic layer was washed with saturated brine and dried over anhydrous sodium sulfate. The solvent was distilled off under reduced pressure. The residue was purified by flash column chromatography (cartridge B-L, Yamazen Corp.) to give the title compound (72 mg) and a methyl ester (51 mg) as an inclusion.
¹H-NMR (CDCl₃) δ: 1.21-1.28 (3H, m), 2.05 (3H, s), 2.57-2.64 (2H, m), 2.86-2.93 (2H, m), 3.43 (3H, s), 3.57-3.70 (1H, m), 3.76-3.85 (1H, m), 3.86 (3H, s), 4.08-4.17 (2H, m), 4.92-4.97 (1H, m), 5.56 (1H, s), 6.78-6.84 (1H, m), 6.95-6.99 (1H, m), 7.07-7.11 (1H, m), 7.13-7.20 (1H, m), 7.54-7.48 (2H, m).
Inclusion
Methyl 3-(2-{[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate
¹H-NMR (CDCl₃) δ: 2.05 (3H, s), 2.59-2.66 (2H, m), 2.87-2.93 (2H, m), 3.43 (3H, s), 3.55-3.66 (1H, m), 3.68 (3H, s), 3.76-3.85 (1H, m), 3.86 (3H, s), 4.92-4.98 (1H, m), 5.56 (1H, s), 6.81-6.83 (1H, m), 6.95-7.00 (1H, m), 7.06-7.10 (1H, m), 7.13-7.19 (1H, m), 7.53-7.50 (2H, m).

### Example 527

Ethyl 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate
Isomer A

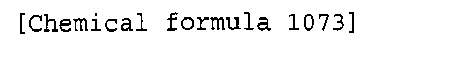

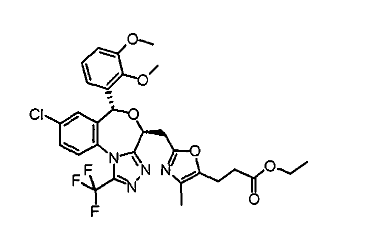

Ethyl 3-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate
Isomer B

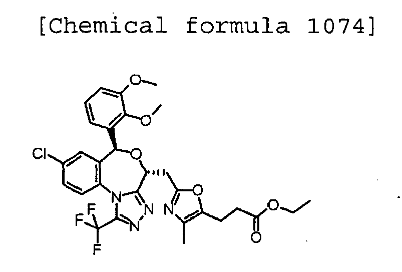

Ethyl 3-(2-{[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl) (72 mg) was optically resolved by HPLC equipped with CHIRALPAK IA (Daicel Chemical) while using a 70% ethanol-n-hexane solution as a mobile phase at a flow rate of 10 mL/min to give the title compounds, that is, isomer A (27 mg) with a short retention time and isomer B (25 mg) with a long retention time.

### Example 528

Methyl 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate
Isomer A

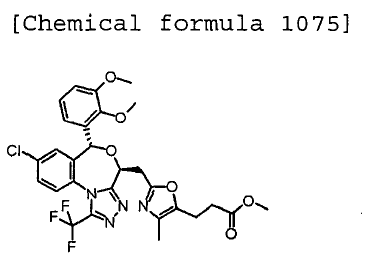

Methyl 3-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate
Isomer B

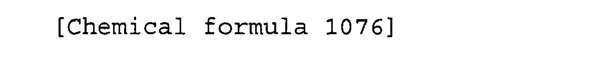

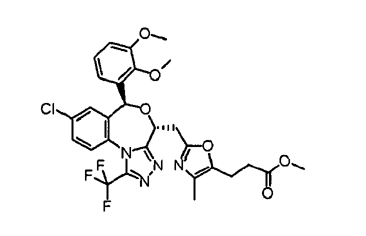

Methyl 3-(2-{[(trans)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl) (50.6 mg) was optically resolved by HPLC equipped with CHIRALPAK IA (Daicel Chemical) while using a 70% ethanol-n-hexane solution as a mobile phase at a flow rate of 10 mL/min to give the title compounds, that is, isomer A (17 mg) and isomer B (17 mg).

### Example 529

3-(2-{[(4S,6R)-8-Chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoic acid

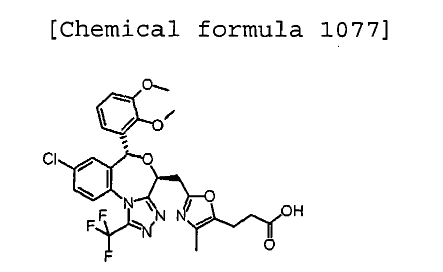

In a similar manner to that employed for the synthesis of methyl 4-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate, the title compound (31.5 mg) was obtained from methyl 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate (17.4 mg) and ethyl 3-(2-{[(4S,6R)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate (26.9 mg) by using potassium hydrogen carbonate (118 mg).
MS (ESI) m/z : 593 (M + H)⁺.
¹H-NMR (CDCl₃) δ: 2.04 (3H, s), 2.59-2.68 (2H, m), 2.85-2.93 (2H, m), 3.43 (3H, s), 3.55-3.68 (1H, m), 3.78-3.84 (1H, m), 3.85 (3H, s), 4.92-4.98 (1H, m), 5.56 (1H, s), 6.82 (1H, s), 6.94-7.00 (1H, m), 7.05-7.11 (1H, m), 7.12-7.19 (1H, m), 7.54-7.49 (2H, m).
Elemental analysis for: C₂₇H₂₄ClF₃N₄O₆·0.5H₂O·0.5 n-hexane
Calculated: C, 55.86; H, 5.00; N, 8.69.
Found: C, 56.11; H, 4.72; N, 8.52.

### Example 530

3-(2-{[(4R,6S)-8-Chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoic acid

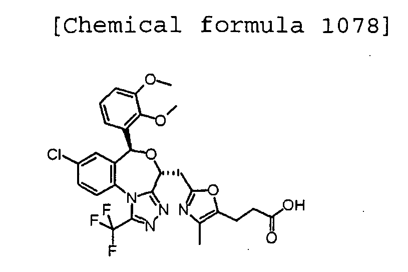

In a similar manner to that employed for the synthesis of methyl 4-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-1,3-oxazol-5-yl)butanoate, the title compound (20.1 mg) was obtained from methyl 3-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate (16.7 mg) and ethyl 3-(2-{[(4R,6S)-8-chloro-6-(2,3-dimethoxyphenyl)-1-(trifluoromethyl)-4H,6H-[1,2,4]triazolo[4,3-a][4,1]benzoxazepin-4-yl]methyl}-4-methyl-1,3-oxazol-5-yl)propanoate (24.8 mg) by using potassium hydrogen carbonate (112 mg).
MS (ESI) m/z : 593 (M + H)⁺.
¹H-NMR (CDCl₃) δ: 2.04 (3H, s), 2.58-2.68 (2H, m), 2.84-2.92 (2H, m), 3.43 (4H, s), 3.56-3.67 (1H, m), 3.76-3.84 (1H, m), 3.85 (3H, s), 4.92-4.98 (1H, m), 5.56 (1H, s), 6.82 (1H, s), 6.93-7.00 (1H, m), 7.06-7.19 (2H, m), 7.53-7.50 (2H, m).
Elemental analysis for: C₂₇H₂₄ClF₃N₄O₆·0.5H₂O·0.5 n-hexane Calculated: C, 55.86; H, 5.00; N, 8.69.
Found: C, 56.05; H, 4.72; N, 8.48.

### Referential Example 359

{5-Chloro-2-[(2,4-dimethoxybenzyl)amino]-3-methoxyphenyl}(2,3-dimethoxyphenyl)methanol

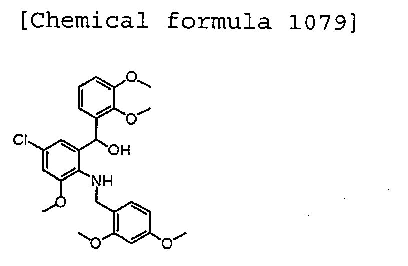

To an acetic acid solution (46 mL) of (2-amino-5-chloro-3-methoxyphenyl)(2,3-dimethoxyphenyl)methanol (3.00 g) was added 2,4-dimethoxybenzaldehyde (1.85 g). The resulting mixture was stirred for 10 minutes. Under ice cooling, sodium borohydride (0.53 g) was added to the reaction mixture. After warming to room temperature, the resulting mixture was stirred for one hour. The solvent was distilled off under reduced pressure. To the residue thus obtained were added ethyl acetate, distilled water and a saturated aqueous solution of sodium hydrogen carbonate and the layers separated. The organic layer thus obtained was washed with saturated brine and dried over anhydrous sodium sulfate. The solvent was distilled off under reduced pressure. The residue was purified by flash column chromatography (Cartridge B-L, Yamazen Corp.) to give the title compound (4.19 g).
MS (ESI) m/z : 474 (M + H)⁺.
¹H-NMR (CDCl3) δ: 3.70 (3H, s), 3.77 (3H, s), 3.78 (3H, s), 3.81 (3H, s), 3.87 (3H, s), 4.08 (1H, d, J = 13.0 Hz), 4.20 (1H, d, J = 13.0 Hz), 4.61 (1H, s), 6.24 (1H, s), 6.33-6.38 (1H, m), 6.43-6.49 (1H, m), 6.53-6.55 (1H, m), 6.70-6.72 (1H, m), 6.86-6.9.1 (1H, m), 6.96-7.02 (2H, m), 7.09-7.04 (1H, m).

### Referential Example 360

Ethyl 2-[(trans)-7-chloro-1-(2,4-dimethoxybenzyl)-5-(2,3-dimethoxyphenyl)-9-methoxy-2-oxo-1,2,3,5-tetrahydro-4,1-benzoxazepin-3-yl]acetate

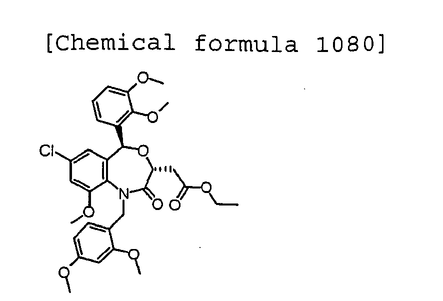

In a similar manner to that employed for the synthesis of ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, ethyl (E)-4-{4-chloro(2,4-dimethoxybenzyl)-2-[(2,3-dimethoxyphenyl)(hydroxy)methyl]-6-methoxyanilino}-4-oxo-2-butenoate (4.82 g) was obtained from {5-chloro-2-[(2,4-dimethoxybenzyl)amino]-3-methoxyphenyl}(2,3-dimethoxyphenyl)methanol(4.19 g) by using sodium hydrogen carbonate (2.23 g) and ethyl chlorofumarate (1.73 g).
Then, in a similar manner to that employed for the synthesis of ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (2.91 g) was obtained using potassium carbonate (3.32 g).
MS (ESI) m/z : 600 (M + H)⁺.
¹H-NMR (CDCl₃) δ: 1.22-1.28 (3H, m), 2.76-2.84 (1H, m), 2.97-3.03 (1H, m), 3.03 (3H, s), 3.57 (3H, s), 3.74 (3H, s), 3.84 (3H, s), 3.96 (3H, s), 4.08-4.20 (2H, m), 4.38-4.43 (1H, m), 4.79 (1H, d, J = 14.0 Hz), 5.64 (1H, d, J = 14.0 Hz), 5.90 (1H, s), 6.10 (1H, d, J = 2.2 Hz), 6.30-6.39 (2H, m), 6.87-6.92 (2H, m), 7.03-7.12 (2H, m), 7.25-7.20 (1H, m).

### Referential Example 361

Ethyl 2-[(trans)-7-chloro-5-(2,3-dimethoxyphenyl)-9-methoxy-2-oxo-1,2,3,5-tetrahydro-4,1-benzoxazepin-3-yl]acetate

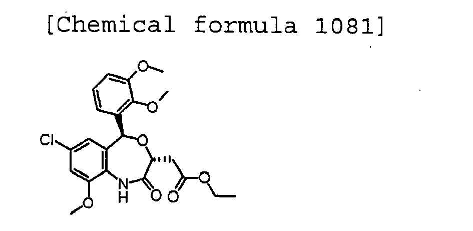

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (1.04 g) was obtained from ethyl 2-[(trans)-7-chloro-1-(2,4-dimethoxybenzyl)-5-(2,3-dimethoxyphenyl)-9-methoxy-2-oxo-1,2,3,5-tetrahydro-4,1-benzoxazepin-3-yl]acetate (2.90 g) by using ceric(IV) ammonium nitrate (10.6 g).
¹H-NMR (CDCl₃) δ: 1.19 (3H, t, J = 7.1 Hz), 2.74-2.90 (1H, m), 2.95-3.04 (1H, m), 3.68 (3H, s), 3.89 (3H, s), 3.90 (3H, s), 4.04-4.16 (2H, m), 4.65-4.69 (1H, m), 6.23 (1H, s), 6.31-6.33 (1H, m), 6.84 (1H, d, J = 2.0 Hz), 6.94-6.99 (2H, m), 7.14-7.08 (1H, m), 7.85 (1H, s).

### Referential Example 362

Ethyl 2-[(trans)-7-chloro-5-(2,3-dimethoxyphenyl)-9-methoxy-2-thioxo-1,2,3,5-tetrahydro-4,1-benzoxazepin-3-yl]acetate

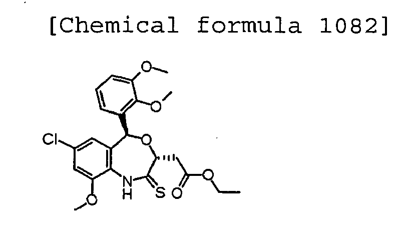

In a similar manner to that employed for the synthesis of methyl 4-(2-{[(trans)-7-chloro-5-(2,3-dimethoxyphenyl)-2-thioxo-1,2,3,5-tetrahydro-4,1-benzoxazepin-3-yl]methyl}-1,3-oxazol-5-yl)butanoate, the title compound (0.85 g) was obtained from ethyl 2-[(trans)-7-chloro-5-(2,3-dimethoxyphenyl)-9-methoxy-2-oxo-1,2,3,5-tetrahydro-4,1-benzoxazepin-3-yl]acetate (1.03 g) by using sodium hydrogen carbonate (0.58 g) and diphosphorus pentasulfide (1.02 g).
MS (ESI) m/z : 466 (M + H)⁺.
¹H-NMR (CDCl₃) δ: 1.21-1.28 (3H, m), 2.92-2.99 (1H, m), 3.22-3.29 (1H, m), 3.65 (3H, s), 3.88 (3H, s), 3.92 (3H, s), 4.08-4.15 (3H, m), 4.73-4.78 (1H, m), 6.12 (1H, s), 6.26-6.28 (1H, m), 6.87-6.89 (1H, m), 6.94-6.98 (1H, m), 7.04-7.08 (1H, m), 7.17-7.10 (1H, m), 9.61 (1H, s).

### Example 531

Ethyl 2-[(trans)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate

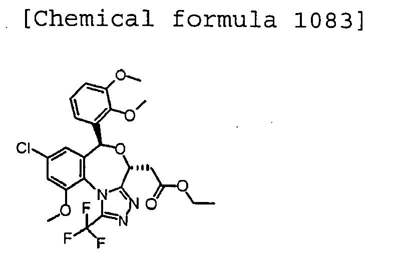

In a similar manner to that employed for the synthesis of ethyl 3-(2-{[(trans)-7-chloro-5-(2,3-dimethoxyphenyl)-2-[hydrazono]-1,5-dihydro-4,1-benzoxazepin3(3H)-yl]methyl}-4-methyl-1,3-oxazol-5-yl)propanoate, the title compound (102 mg) was obtained from ethyl 2-[(trans)-7-chloro-5-(2,3-dimethoxyphenyl)-9-methoxy-2-thioxo-1,2,3,5-tetrahydro-4,1-benzoxazepin-3-yl]acetate (149 mg) by using hydrazine monohydrate (23.3 µl), trifluoroacetic anhydride (444 µl) and trifluoroacetic acid (238 µl).
MS (ESI) m/z : 542 (M + H)⁺.
¹H-NMR (CDCl₃) δ: 1.25-1.31 (3H, m), 3.20-3.28 (1H, m), 3.43-3.49 (1H, m), 3.45 (3H, s), 3.85 (3H, s), 3.90 (3H, s), 4.14-4.24 (2H, m), 4.86-4.91 (1H, m), 5.50 (1H, s), 6.38-6.39 (1H, m), 6.94-7.01 (1H, m), 7.04-7.06 (1H, m), 7.18-7.13 (2H, m).

### Example 532

Ethyl 2-[(4S,6R)-8-chloro-6-(2,3-dimethoxyphenyl)-10-methoxy-1-(trifluoromethyl)-4H,6H-[1,2,4]triazolo[4,3-a] [4, 1] benzoxazepin-4-yl] acetate
Isomer A

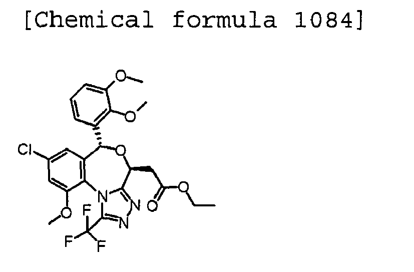

Ethyl 2-[(4R,6S)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate
Isomer B

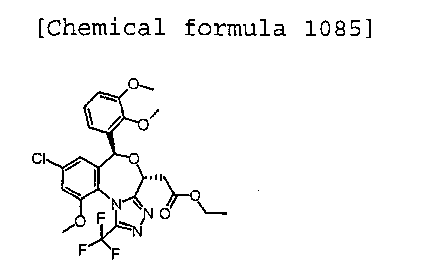

Ethyl 2-[(trans)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate (558 mg) was optically resolved by HPLC equipped with CHIRALPAK AD (Daicel Chemical). By using 70% ethanol-hexane as a solvent and setting a flow rate at 10 mL/min, separation was performed to give, as the title compounds, the isomer A (217 mg) with a short retention time and the isomer B (253 mg) with a long retention time.

### Example 533

2-[(4S,6R)-8-Chloro-6-(2,3-dimethoxyphenyl)-10-methoxy-1-(trifluoromethyl)-4H,6H-[1,2,4]triazolo[4,3-a][4,1]benzoxazepin-4-yl]acetic acid

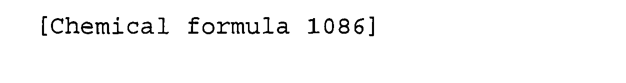

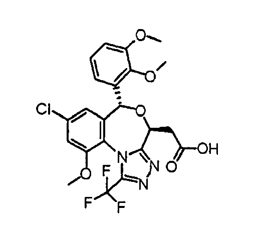

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetic acid, the title compound (93 mg) was obtained from ethyl 2-[(4S,6R)-8-chloro-6-(2,3-dimethoxyphenyl)-10-methoxy-1-(trifluoromethyl)-4H,6H-[1,2,4]triazolo[4,3-a] [4,1]benzoxazepin-4-yl]acetate (214 mg) by using concentrated hydrochloric acid (5.0 mL).
MS (ESI) m/z : 514 (M + H)⁺.
¹H-NMR (CDCl₃) δ: 3.15-3.24 (1H, m), 3.36-3.42 (1H, m), 3.43 (3H, s), 3.84 (3H, s), 3.88 (3H, s), 4.80-4.89 (1H, m), 5.47 (1H, s), 6.35-6.38 (1H, m), 6.91-6.96 (1H, m), 7.04-7.02 (1H, m), 7.20-7.10 (2H, m).
Elemental analysis for: C₂₂H₁₉ClF₃N₃O₆·1.0H₂O·0.1 n-hexane
Calculated: C, 50.22; H, 4.18; N, 7.77.
Found: C, 50.55; H, 3.88; N, 7.49.

### Example 534

2-[(4R,6S)-8-Chloro-6-(2,3-dimethoxyphenyl)-10-methoxy-1-(trifluoromethyl)-4H,6H-[1,2,4]triazolo[4,3-a] [4,1]benzoxazepin-4-yl]acetic acid

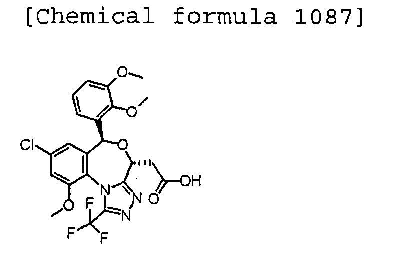

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetic acid, the title compound (81 mg) was obtained from ethyl 2-[(4R,6S)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate (253 mg) by using concentrated hydrochloric acid (3.5 mL).
MS (ESI) m/z : 514 (M + H)⁺.
¹H-NMR (CDCl₃) δ: 3.15-3.24 (1H, m), 3.36-3.42 (1H, m), 3.43 (3H, s), 3.84 (3H, s), 3.88 (3H, s), 4.80-4.89 (1H, m), 5.47 (1H, s), 6.35-6.38 (1H, m), 6.91-6.96 (1H, m), 7.04-7.02 (1H, m), 7.20-7.10 (2H, m).
Elemental analysis for: C₂₂H₁₉ClF₃N₃O₆·1.0H₂O·0.1 n-hexane
Calculated: C, 50.22; H, 4.18; N, 7.77.
Found: C, 50.56; H, 3.92; N, 7.51.

### Referential Example 363

(3-Methoxy-2-methylphenyl)methanol

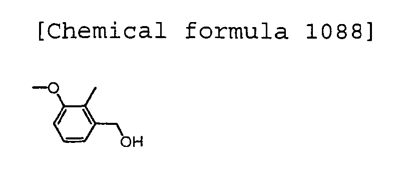

To a tetrahydrofuran solution (690 mL) of 3-methoxy-2-methylbenzoic acid (29.1 g) was added dropwise a borane hydride-tetrahydrofuran solution (1.0 mol/l, 210 mL) over 30 minutes under ice cooling. While warming to room temperature, the resulting mixture was stirred for 14.5 hours. The reaction mixture was ice cooled and distilled water was added thereto in portions. To the resulting mixture was added ethyl acetate and the layers separated. The organic layer thus obtained was washed with saturated brine and dried over anhydrous sodium sulfate. The filtrate was then concentrated to give the title compound (25.3 g).
¹H-NMR (CDCl3) δ: 2.23 (3H, s), 3.83 (3H, s), 4.70 (2H, s), 6.85-6.81 (1H, m), 6.97-7.01 (1H, m), 7.15-7.20 (1H, m).

### Referential Example 364

3-Methoxy-2-methylbenzaldehyde

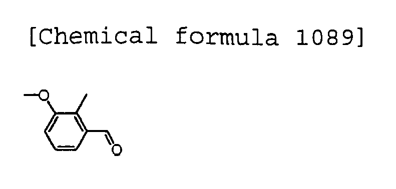

A dichloromethane solution (200 mL) of (3-methoxy-2-methylphenyl)methanol (15.2 g) was added manganese dioxide (purity: 85%, 118 g). The resulting mixture was heated under reflux for 8.5 hours. The reaction mixture was filtered through Celite 545, followed by sufficient washing with dichloromethane. The filtrates were combined and concentrated under reduced pressure to give the title compound (15.1 g).
¹H-NMR (CDCl3) δ: 2.54 (3H, s), 3.88 (3H, s), 7.06-7.10 (1H, m), 7.34-7.28 (1H, m), 7.46-7.41 (1H, m), 10.28-10.36 (1H, m).

### Referential Example 365

tert-Butyl 4-chloro-2-[hydroxy(3-methoxy-2-methylphenyl)methyl]phenylcarbamate

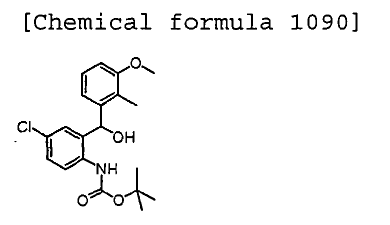

A tetrahydrofuran solution (150 mL) of tert-butyl 4-chlorophenylcarbamate (9.00 g) was cooled to -78°C. To the reaction mixture was added dropwise a sec-butyl lithium (a 1.01 mol/l cyclohexane-hexane mixed solution, 90.3 mL) over 15 minutes. After stirring for 15 minutes at -78°C, the temperature was raised to -30°C and the reaction mixture was stirred for 2 hours. The reaction mixture was then cooled to -78°C and added dropwise to a tetrahydrofuran solution (100 mL) of 3-methoxy-2-methylbenzaldehyde (7.14 g) of -20°C via a double-ended cannula over 2 hours. After stirring at -20°C for one hour, an aqueous ammonium chloride solution and ethyl acetate were added to the reaction mixture and the layers separated. The organic layer thus obtained was washed with saturated brine and dried over anhydrous sodium sulfate. The solvent was distilled off under reduced pressure and the residue was purified by silica gel column chromatography (hexane:ethyl acetate=4:1) to give the title compound (10.2 g).
MS (ESI) m/z : 304 (M - OH)⁺, 400 (M + Na)⁺.
¹H-NMR (CDCl3) δ: 1.51 (9H, s), 2.04 (3H, s), 3.86 (3H, s), 6.03-6.05 (1H, m), 6.78-6.80 (1H, m), 6.85-6.90 (1H, m), 7.07-7.10 (1H, m), 7.20-7.25 (2H, m), 7.54 (1H, s), 7.77-7.82 (1H, m).

### Referential Example 366

(2-Amino-5-chlorophenyl)(3-methoxy-2-methylphenyl)methanol

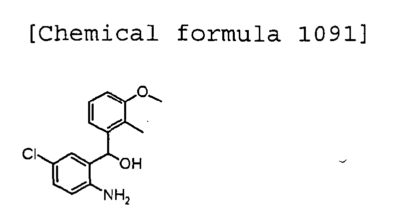

To a dioxane solution (300 mL) of tert-butyl 4-chloro-2-[hydroxy(3-methoxy-2-methylphenyl)methyl]phenylcarbamate (11.9 g) were added distilled water (100 mL) and concentrated hydrochloric acid (100 mL) under ice cooling. The resulting mixture was stirred at room temperature for 8 hours. After the reaction mixture was concentrated under reduced pressure, saturated brine and ethyl acetate were added to the residue and the layers separated. The organic layer thus obtained was washed with a saturated aqueous solution of sodium hydrogen carbonate and saturated brine and dried over anhydrous sodium sulfate. The solvent was distilled off under reduced pressure to give the title compound (8.7 g). ¹H-NMR (CDCl3) δ: 2.12 (3H, s), 3.86 (3H, s), 4.04 (2H, br s), 6.01 (1H, s), 6.60-6.65 (1H, m), 6.79-6.83 (1H, m), 6.85-6.90 (1H, m), 6.99-7.09 (2H, m), 7.26-7.20 (1H, m).

### Referential Example 367

{5-Chloro-2-[(2,4-dimethoxybenzyl)amino]phenyl}(3-methoxy-2-methylphenyl)methanol

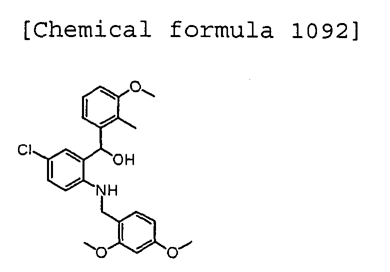

In a similar manner to that employed for the synthesis of {5-chloro-2-[(2,4-dimethoxybenzyl)amino]-3-methoxyphenyl}(2,3-dimethoxyphenyl)methanol, the title compound (12.7 g) was obtained from (2-amino-5-chlorophenyl)(3-methoxy-2-methylphenyl)methanol (8.25 g) by using 2,4-dimethoxybenzaldehyde(6.42 g) and sodium borohydride (1.69 g).
MS (ESI) m/z : 428 (M + H)⁺.
¹H-NMR (CDCl3) δ: 2.03-2.09 (3H, m), 3.77-3.82 (6H, m), 3.84-3.88 (3H, m), 4.20-4.30 (2H, m), 4.63-4.60 (1H, m), 5.98 (1H, s), 6.38-6.49 (3H, m), 6.62-6.66 (1H, m), 6.75-6.78 (1H, m), 6.83-6.92 (1H, m), 6.99-7.13 (2H, m), 7.15-7.25 (1H, m).

### Referential Example 368

Ethyl 2-[(trans)-7-chloro-1-(2,4-dimethoxybenzyl)-5-(3-methoxy-2-methylphenyl)-2-oxo-1,2,3,5-tetrahydro-4,1-benzoxazepin-3-yl]acetate

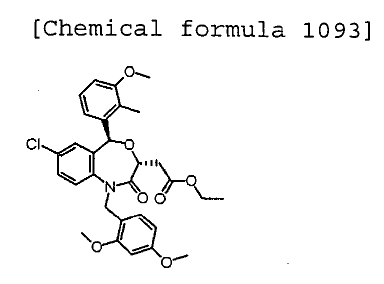

In a similar manner to that employed for the synthesis of ethyl 2-[(trans)-7-chloro-1-(2,4-dimethoxybenzyl)-5-(2,3-dimethoxyphenyl)-9-methoxy-2-oxo-1,2,3,5-tetrahydro-4,1-benzoxazepin-3-yl]acetate, the title compound (3.46 g) was obtained from {5-chloro-2-[(2,4-dimethoxybenzyl)amino]phenyl}(3-methoxy-2-methylphenyl)methanol (12.7 g) while carrying out amidation by using sodium hydrogen carbonate (7.49 g) and monoethyl chlorofumarate (7.24 g) and then using potassium carbonate (3.76 g).
MS (ESI) m/z : 554 (M + H)⁺.
¹H-NMR (CDCl₃) δ: 1.23-1.28 (3H, m), 1.36 (3H, s), 2.75-2.84 (1H, m), 3.06-3.14 (1H, m), 3.57 (3H, s), 3.76 (3H, s), 3.81 (3H, s), 4.08-4.20 (2H, m), 4.43-4.48 (1H, m), 4.83-4.94 (1H, m), 5.45-5.51 (1H, m), 5.62 (1H, s), 6.30-6.48 (3H, m), 6.82-6.86 (1H, m), 7.15-7.18 (1H, m), 7.21-7.26 (1H, m), 7.29-7.42 (3H, m).

### Referential Example 369

Ethyl 2-[(trans)-7-chloro-5-(3-methoxy-2-methylphenyl)-2-oxo-1,2,3,5-tetrahydro-4,1-benzoxazepin-3-yl]acetate

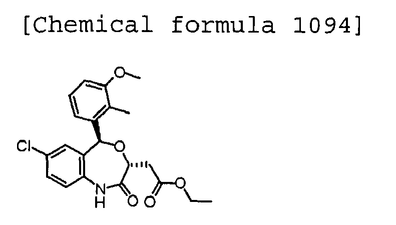

In a similar manner to that employed for the synthesis of ethyl 2-[(trans)-7-chloro-5-(2,3-dimethoxyphenyl)-9-methoxy-2-oxo-1,2,3,5-tetrahydro-4,1-benzoxazepin-3-yl]acetate, the title compound (4.05 g) was obtained from ethyl 2-[(trans)-7-chloro-1-(2,4-dimethoxybenzyl)-5-(3-methoxy-2-methylphenyl)-2-oxo-1,2,3,5-tetrahydro-4,1-benzoxazepin-3-yl]acetate (5.81 g) by using ceric(IV) ammonium nitrate (17.3 g).
MS (ESI) m/z : 404 (M + H)⁺.
¹H-NMR (CDCl₃) δ : 1.20-1.25 (3H, m), 1.99 (3H, s), 2.76-2.83 (1H, m), 2.95-3.04 (1H, m), 3.86 (3H, s), 4.05-4.18 (2H, m), 4.53-4.57 (1H, m), 6.06 (1H, s), 6.73 (1H, d, J = 2.2 Hz), 6.88-6.91 (1H, m), 6.94-7.00 (2H, m), 7.19-7.31 (2H, m), 7.71 (1H, s).

### Referential Example 370

Ethyl 2-[(trans)-7-chloro-5-(3-methoxy-2-methylphenyl)-2-thioxo-1,2,3,5-tetrahydro-4,1-benzoxazepin-3-yl]acetate

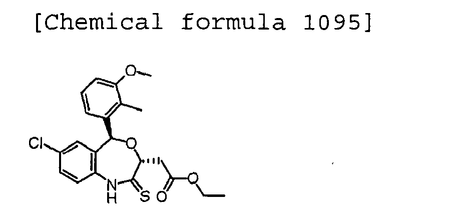

In a similar manner to that employed for the synthesis of ethyl 2-[(trans)-7-chloro-5-(2,3-dimethoxyphenyl)-9-methoxy-2-thioxo-1,2,3,5-tetrahydro-4,1-benzoxazepin-3-yl]acetate, the title compound (3.25 g) was obtained from ethyl 2-[(trans)-7-chloro-5-(3-methoxy-2-methylphenyl)-2-oxo-1,2,3,5-tetrahydro-4,1-benzoxazepin-3-yl]acetate (4.04 g) by using sodium hydrogen carbonate (2.52 g) and diphosphorus pentasulfide (4.46 g).
MS (ESI) m/z : 420 (M + H)⁺.
¹H-NMR (CDCl₃) δ: 1.22-1.28 (3H, m), 1.90 (3H, s), 2.93-3.00 (1H, m), 3.21-3.29 (1H, m), 3.85 (3H, s), 4.09-4.17 (2H, m), 4.67-4.72 (1H, m), 5.96 (1H, s), 6.65-6.68 (1H, m), 6.87-6.93 (1H, m), 7.05-7.11 (2H, m), 7.22-7.28 (1H, m), 7.37-7.33 (1H, m), 9.58 (1H, s).

### Example 535

Ethyl 2-[(trans)-8-chloro-6-(3-methoxy-2-methylphenyl)-1-(trifluoromethyl)-4H,6H-[1,2,4]triazolo[4,3-a][4,1]benzoxazepin-4-yl]acetate

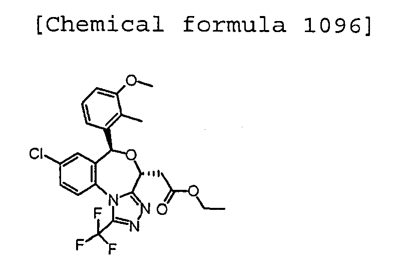

In a similar manner to that employed for the synthesis of ethyl 2-[(trans)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate, the title compound (2.54 g) was obtained from ethyl 2-[(trans)-7-chloro-5-(3-methoxy-2-methylphenyl)-2-thioxo-1,2,3,5-tetrahydro-4,1-benzoxazepin-3-yl]acetate (3.25 g) by using hydrazine monohydrate (0.75 mL) and trifluoroacetic anhydride (5.38 mL).
MS (ESI) m/z : 496 (M + H)⁺.
¹H-NMR (CDCl₃) δ: 1.25-1.31 (3H, m), 1.66 (3H, s), 3.22-3.29 (1H, m), 3.45-3.53 (1H, m), 3.84 (3H, s), 4.13-4.27 (2H, m), 4.93-4.98 (1H, m), 5.38 (1H, s), 6.79-6.81 (1H, m), 6.89-6.93 (1H, m), 7.22-7.33 (3H, m), 7.53-7.55 (2H, m) .

### Example 536

2-[(trans)-8-Chloro-6-(3-methoxy-2-methylphenyl)-1-(trifluoromethyl)-4H,6H-[1,2,4]triazolo[4,3-a][4,1]benzoxazepin-4-yl]acetic acid

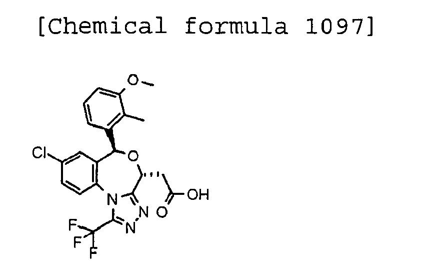

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (25 mg) was obtained from ethyl 2-[(trans)-8-chloro-6-(3-methoxy-2-methylphenyl)-1-(trifluoromethyl)-4H,6H-[1,2,4]triazolo[4,3-a][4,1]benzoxazepin-4-yl]acetate (25.0 mg) by using concentrated hydrochloric acid (0.55 mL).
MS (ESI) m/z : 468(M + H)⁺.
¹H-NMR (CDCl₃) δ: 1.63 (3H, s), 3.16 (1H, dd, J = 16.6, 6.1 Hz), 3.41 (1H, dd, J = 16.4, 7.6 Hz), 3.82 (3H, s), 4.88 (1H, dd, J = 7.6, 6.1 Hz), 5.34 (1H, s), 6.76 (1H, d, J = 2.2 Hz), 6.87 (1H, dd, J = 7.1, 2.2 Hz), 7.25-7.28 (2H, m), 7.46 (1H, dd, J = 8.5, 2.0 Hz), 7.52 (1H, d, J = 8.5 Hz).

### Example 537

Ethyl 2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetate
Isomer A

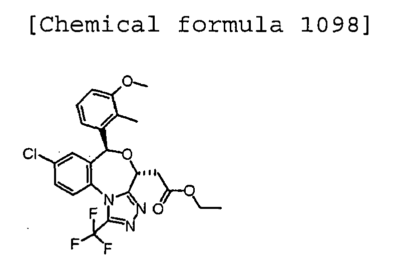

Ethyl 2-[(4S,6S)-8-chloro-6-(3-methoxy-2-methylphenyl)-1-(trifluoromethyl)-4H,6H-[1,2,4]triazolo[4,3-a][4,1]benzoxazepin-4-yl]acetate
Isomer B

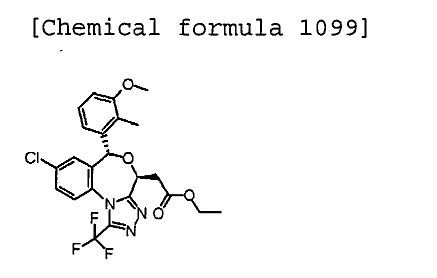

Ethyl 2-[(trans)-8-chloro-6-(3-methoxy-2-methylphenyl)-1-(trifluoromethyl)-4H,6H-[1,2,4]triazolo[4,3-a][4,1]benzoxazepin-4-yl]acetate (316 mg) was optically resolved by HPLC equipped with CHIRALPAK IA (Daicel Chemical). By using dichloromethane-hexane as a solvent and setting a flow rate at 10 mL/min, separation was performed to give, as the title compounds, the isomer A (158 mg) and the isomer B (158 mg).

### Example 538

2-[(4R,6R)-8-Chloro-6-(3-methoxy-2-methylphenyl)-1-(trifluoromethyl)-4H,6H-[1,2,4]triazolo[4,3-a] [4,1]benzoxazepin-4-yl]acetic acid

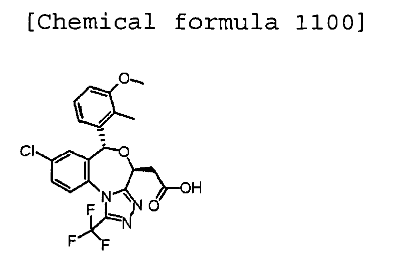

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (131 mg) was obtained from ethyl 2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetate (163 mg) by using concentrated hydrochloric acid (3.0 mL).
MS (ESI) m/z : 468 (M + H)⁺.
¹H-NMR (CDCl₃) δ: 1.61 (3H, s), 3.14-3.25 (1H, m), 3.34-3.50 (1H, m), 3.81 (3H, s), 4.83-4.93 (1H, m), 5.30 (1H, s), 6.76-6.73 (1H, m), 6.83-6.89 (1H, m), 7.23-7.29 (2H, m), 7.44-7.53 (2H, m).
Elemental analysis for: C₂₁H₁₇ClF₃N₃O₄·1.4H₂O·0.1 n-hexane Calculated: C, 51.71; H, 4.26; N, 8.38.
Found: C, 52.09; H, 4.05; N, 7.98.

### Example 539

2-[(4S,6S)-8-Chloro-6-(3-methoxy-2-methylphenyl)-1-(trifluoromethyl)-4H,6H-[1,2,4]triazolo[4,3-a] [4,1]benzoxazepin-4-yl]acetic acid

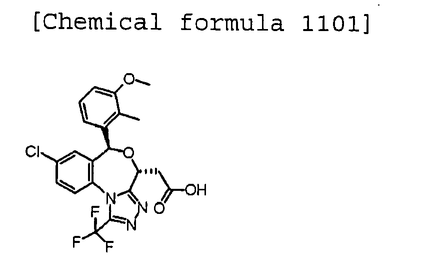

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (140 mg) was obtained from ethyl 2-[(4S,6S)-8-chloro-6-(3-methoxy-2-methylphenyl)-1-(trifluoromethyl)-4H,6H-[1,2,4]triazolo[4,3-a] [4,1]benzoxazepin-4-yl]acetate(157 mg) by using concentrated hydrochloric acid (3.0 mL).
MS (ESI) m/z : 468 (M + H)⁺.
¹H-NMR (CDCl₃) δ: 1.61 (3H, s), 3.15-3.27 (1H, m), 3.35-3.52 (1H, m), 3.81 (3H, s), 4.85-4.93 (1H, m), 5.29-5.33 (1H, m), 6.74-6.77 (1H, m), 6.84-6.90 (1H, m), 7.24-7.28 (2H, m), 7.54-7.45 (2H, m).
Elemental analysis for: C₂₁H₁₇ClF₃N₃O₄·1.3H₂O·0.1 n-hexane Calculated: C, 51.90; H, 4.23; N, 8.41.
Found: C, 51.90; H, 4.01; N, 8.15.

### Example 540

Ethyl 2-[(trans)-8-chloro-1-[chloro(difluoro)methyl]-6-(3-methoxy-2-methylphenyl)-4H,6H-[1,2,4]triazolo[4,3-a] [4,1]benzoxazepin-4-yl]acetate

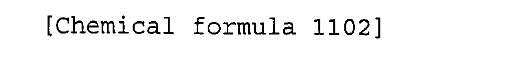

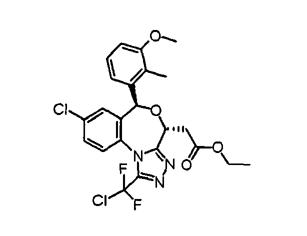

In a similar manner to that employed for the synthesis of ethyl 2-[(trans)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate, the title compound (1.00 g) was obtained from ethyl 2-[(trans)-7-chloro-5-(3-methoxy-2-methylphenyl)-2-thioxo-1,2,3,5-tetrahydro-4,1-benzoxazepin-3-yl]acetate (1.00 g) by using hydrazine monohydrate (0.23 mL) and chlorodifluoroacetic anhydride (2.28 mL).
MS (ESI) m/z : 512 (M + H)⁺.
¹H-NMR (CDCl₃) δ: 1.26-1.31 (3H, m), 1.67 (3H, s), 3.22-3.29 (1H, m), 3.45-3.53 (1H, m), 3.83 (3H, s), 4.14-4.24 (2H, m), 4.90-4.95 (1H, m), 5.38 (1H, s), 6.78-6.80 (1H, m), 6.89-6.93 (1H, m), 7.33-7.24 (2H, m), 7.61-7.52 (2H, m) .

### Example 541

2-[(trans)-8-Chloro-1-[chloro(difluoro)methyl]-6-(3-methoxy-2-methylphenyl)-4H,6H-[1,2,4]triazolo[4,3-a][4,1]benzoxazepin-4-yl]acetic acid

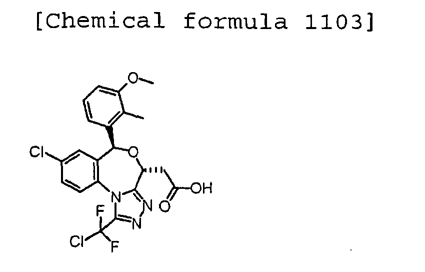

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (1.32 g) was obtained from ethyl 2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetate (1.46 g) by using concentrated hydrochloric acid (30.0 mL). ¹H-NMR (CDCl₃) δ:1.62 (3H, s), 3.28-3.36 (1H, m), 3.52-3.61 (1H, m), 3.84 (3H, s), 4.88-4.93 (1H, m), 5.39 (1H, s), 6.80 (1H, d, J = 2.2 Hz), 6.89-6.94 (1H, m), 7.34-7.24 (2H, m), 7.63-7.51 (2H, m).

### Example 542

Ethyl 2-[(4R,6R)-8-chloro-1-[chloro(difluoro)methyl]-6-(3-methoxy-2-methylphenyl)-4H,6H-[1,2,4]triazolo[4,3-a] [4,1]benzoxazepin-4-yl]acetate
Isomer A

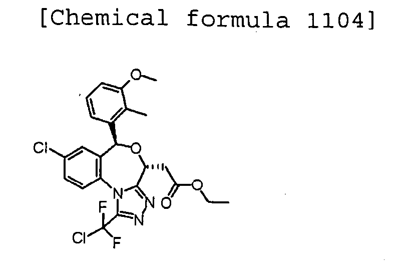

Ethyl 2-[(4S,6S)-8-chloro-1-[chloro(difluoro)methyl]-6-(3-methoxy-2-methylphenyl)-4H,6H-[1,2,4]triazolo[4,3-a] [4,1] benzoxazepin-4-yl] acetate
Isomer B

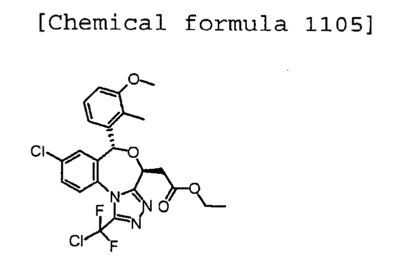

Ethyl 2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetate (1003 mg) was optically resolved by HPLC equipped with CHIRALPAK IA (Daicel Chemical). By using dichloromethane-hexane as a solvent and setting a flow rate at 10 mL/min, separation was performed to give, as the title compounds, the isomer A (437 mg) with a short retention time and the isomer B (432 mg) with a long retention time.

### Example 543

2-[(4R,6R)-8-Chloro-1-[chloro(difluoro)methyl]-6-(3-methoxy-2-methylphenyl)-4H,6H-[1,2,4]triazolo[4,3-a] [4,1]benzoxazepin-4-yl]acetic acid

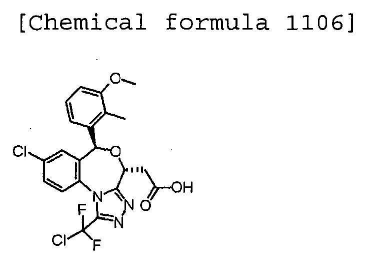

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (356 mg) was obtained from ethyl 2-[(4R,6R)-8-chloro-1-[chloro(difluoro)methyl]-6-(3-methoxy-2-methylphenyl)-4H,6H-[1,2,4]triazolo[4,3-a][4,1]benzoxazepin-4-yl]acetate (426 mg) by using concentrated hydrochloric acid (9.0 mL). MS (ESI) m/z : 484 (M + H)⁺.
¹H-NMR (CDCl₃) δ: 1.62 (3H, s), 3.17-3.26 (1H, m), 3.37-3.49 (1H, m), 3.82 (3H, s), 4.84-4.90 (1H, m), 5.31 (1H, s), 6.74-6.77 (1H, m), 6.85-6.90 (1H, m), 7.21-7.32 (2H, m), 7.45-7.50 (1H, m), 7.59-7.53 (1H, m).
Elemental analysis for: C₂₁H₁₇Cl₂F₂N₃O₄·0.5H₂O
Calculated: C, 51.13; H, 3.68; N, 8.52.
Found: C, 51.28; H, 3.70; N, 8.20.

### Example 544

2-[(4S,6S)-8-Chloro-1-[chloro(difluoro)methyl]-6-(3-methoxy-2-methylphenyl)-4H,6H-[1,2,4]triazolo[4,3-a][4,1]benzoxazepin-4-yl]acetic acid

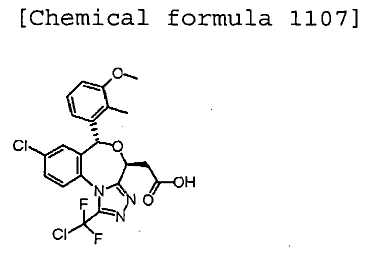

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (357 mg) was obtained from ethyl 2-[(4S,6S)-8-chloro-1-[chloro(difluoro)methyl]-6-(3-methoxy-2-methylphenyl)-4H,6H-[1,2,4]triazolo[4,3-a][4,1]benzoxazepin-4-yl]acetate (422 mg) by using concentrated hydrochloric acid (9.0 mL).
MS (ESI) m/z : 484 (M + H)⁺.
¹H-NMR (CDCl₃) δ: 1.61 (3H, s), 3.15-3.25 (1H, m), 3.38-3.50 (1H, m), 3.81 (3H, s), 4.82-4.88 (1H, m), 5.29 (1H, s), 6.73-6.75 (1H, m), 6.82-6.90 (1H, m), 7.23-7.30 (2H, m), 7.45-7.50 (1H, m), 7.58-7.54 (1H, m).
Elemental analysis for: C₂₁H₁₇Cl₂F₂N₃O₄·0.5H₂O
Calculated: C, 51.13; H, 3.68; N, 8.52.
Found: C, 50.95; H, 3.65; N, 8.18.

### Example 545

Ethyl 2-[(trans)-8-chloro-1-(1,1-difluoroethyl)-6-(3-methoxy-2-methylphenyl)-4H,6H-[1,2,4]triazolo[4,3-a][4,1]benzoxazepin-4-yl]acetate

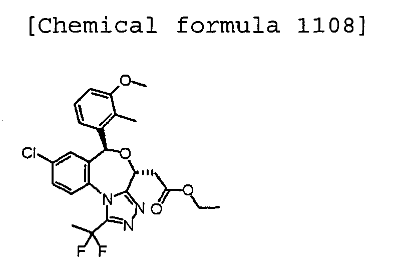

To a tetrahydrofuran solution (29 mL) of ethyl 2-[(trans)-7-chloro-5-(3-methoxy-2-methylphenyl)-2-thioxo-1,2,3,5-tetrahydro-4,1-benzoxazepin-3-yl]acetate (1215 mg) was added hydrazine monohydrate (211 µl). The resulting mixture was stirred at room temperature for one hour. The reaction mixture was concentrated under reduced pressure and a dichloromethane solution (29 mL) of the residue was prepared. To the resulting solution were added 2,2-difluoropropanoic acid (478 mg), 1-ethyl-3-(3-dimethylaminopropyl)carbodiimide hydrochloride (1109 mg) and 1-hydroxybenzotriazole (443 mg). The resulting mixture was stirred at room temperature for 18.5 hours. To the reaction mixture were added saturated brine and ethyl acetate and the layers separated. The organic layer thus obtained was washed with saturated brine and dried over anhydrous sodium sulfate. The solvent was distilled off under reduced pressure. An acetic acid solution (29 mL) of the residue was prepared and stirred for 15 hours while heating under reflux. The solvent was distilled off under reduced pressure. To the residue were added saturated sodium hydrogen carbonate and ethyl acetate and the layers separated. The organic layer thus obtained was washed with saturated brine and dried over anhydrous sodium sulfate. The solvent was distilled off under reduced pressure and the residue thus obtained was purified by flash column chromatography (Cartridge B-L, Yamazen Corp.) to give the title compound (862 mg).
MS (ESI) m/z : 492 (M + H)⁺.
¹H-NMR (CDCl₃) δ: 1.25-1.31 (3H, m), 1.65 (3H, s), 2.33 (3H, t, J = 19.2 Hz), 3.21-3.28 (1H, m), 3.42-3.50 (1H, m), 3.83 (3H, s), 4.14-4.24 (2H, m), 4.90-4.95 (1H, m), 5.38 (1H, s), 6.75 (1H, d, J = 2.2 Hz), 6.87-6.92 (1H, m), 7.24-7.32 (2H, m), 7.48-7.52 (1H, m), 7.74-7.69 (1H, m).

### Example 546

Ethyl 2-[(4R,6R)-8-chloro-1-(1,1-difluoroethyl)-6-(3-methoxy-2-methylphenyl)-4H,6H-[1,2,4]triazolo[4,3-a][4,1]benzoxazepin-4-yl]acetate
Isomer A

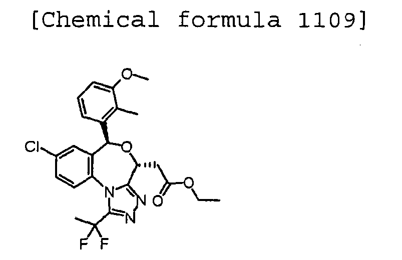

Ethyl 2-[(4S,6S)-8-chloro-1-(1,1-difluorcethyl)-6-(3-methoxy-2-methylphenyl)-4H,6H-[1,2,4]triazolo[4,3-a][4,1]benzoxazepin-4-yl]acetate
Isomer B

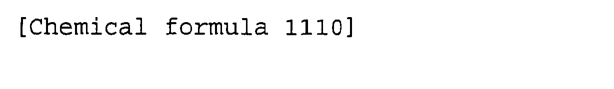

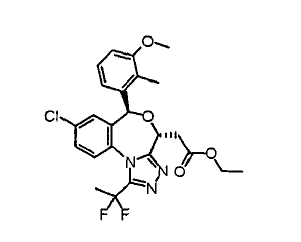

Ethyl 2-[(trans)-8-chloro-1-(1,1-difluoroethyl)-6-(3-methoxy-2-methylphenyl)-4H,6H-[1,2,4]triazolo[4,3-a][4,1]benzoxazepin-4-yl]acetate (782 mg) was optically resolved by HPLC equipped with CHIRALPAK IA (Daicel Chemical). By using 25% ethyl acetate-hexane as a solvent and setting a flow rate at 10 mL/min, separation was performed to give, as the title compounds, the isomer A (393 mg) with a short retention time and the isomer B (410 mg) with a long retention time.

### Example 547

2-[(4R,6R)-8-Chloro-1-(1,1-difluoroethyl)-6-(3-methoxy-2-methylphenyl)-4H,6H-[1,2,4]triazolo[4,3-a][4,1]benzoxazepin-4-yl]acetic acid

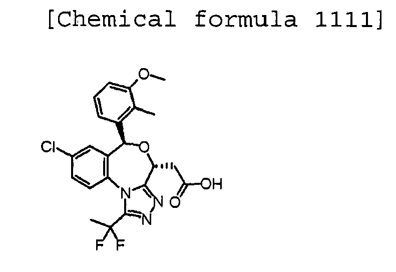

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (340 mg) was obtained from ethyl 2-[(4R,6R)-8-chloro-1-(1,1-difluoroethyl)-6-(3-methoxy-2-methylphenyl)-4H,6H-[1,2,4]triazolo[4,3-a][4,1]benzoxazepin-4-yl]acetate (387 mg) by using concentrated hydrochloric acid (10.0 mL).
MS (ESI) m/z : 464 (M + H)⁺.
¹H-NMR (CDCl₃) δ: 1.65 (3H, s), 2.33 (3H, t, J = 19.2 Hz), 3.26-3.34 (1H, m), 3.50-3.59 (1H, m), 3.83 (3H, s), 4.86-4.91 (1H, m), 5.38 (1H, s), 6.76 (1H, d, J = 2.5 Hz), 6.88-6.93 (1H, m), 7.27-7.34 (2H, m), 7.48-7.53 (1H, m), 7.74-7.70 (1H, m).
Elemental analysis for: C₂₂H₂₀ClF₂N₃O₄·0.5H₂O·0.2 n-hexane Calculated: C, 56.85; H, 4.89; N, 8.57.
Found: C, 57.03; H, 4.66; N, 8.69.

### Example 548

2-[(4S,6S)-8-Chloro-1-(1,1-difluoroethyl)-6-(3-methoxy-2-methylphenyl)-4H,6H-[1,2,4]triazolo[4,3-a] [4,1]benzoxazepin-4-yl]acetic acid

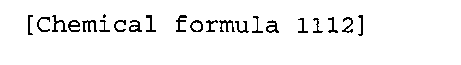

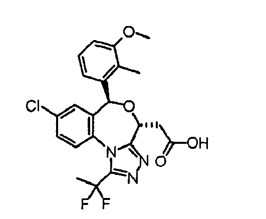

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (359 mg) was obtained from ethyl 2-[(4S,6S)-8-chloro-1-(1,1-difluoroethyl)-6-(3-methoxy-2-methylphenyl)-4H,6H-[1,2,4]triazolo[4,3-a][4,1]benzoxazepin-4-yl]acetate (387 mg) by using concentrated hydrochloric acid (10.0 mL).
MS (ESI) m/z : 464 (M + H)⁺.
¹H-NMR (CDCl₃) δ: 1.64 (3H, s), 2.32 (3H, t, J = 19.2 Hz), 3.25-3.33 (1H, m), 3.49-3.58 (1H, m), 3.83 (3H, s), 4.86-4.91 (1H, m), 5.38 (1H, s), 6.76 (1H, d, J = 2.2 Hz), 6.88-6.92 (1H, m), 7.24-7.33 (2H, m), 7.47-7.53 (1H, m), 7.74-7.70 (1H, m).
Elemental analysis for: C₂₂H₂₀ClF₂N₃O₄·0.5H₂O·0.25 n-hexane
Calculated: C, 57.09; H, 4.99; N, 8.50.
Found: C, 57.12; H, 4.79; N, 8.56.

### Example 549

tert-Butyl 2-(1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-4-piperidinyl)acetate

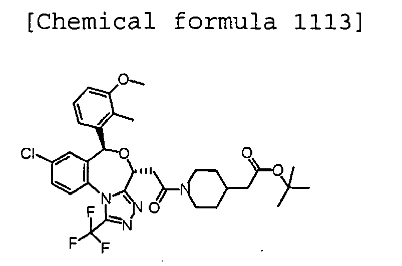

In a similar manner to that employed for the synthesis of ethyl 2-(1-(2-(8-chloro-6-(2,3-dimethoxyphenyl)-4H,6H-pyrrolo[(1,2-a)][(4,1)]benzoxazepin-4-yl)acetyl)-4-piperidinyl)acetate, the title compound (270 mg) was obtained from 2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetic acid (205 mg) by using tert-butyl 2-(4-piperidinyl)acetate (105 mg), 1-ethyl-3-(3-dimethylaminopropyl)carbodiimide hydrochloride (168 mg), and 1-hydroxybenzotriazole (67 mg). MS (ESI) m/z : 649 (M + H)⁺.
¹H-NMR (CDCl₃) δ: 1.08-1.54 (3H, m), 1.45 (9H, s), 1.64-1.68 (3H, m), 1.69-1.85 (2H, m), 1.94-2.07 (1H, m), 2.11-2.21 (2H, m), 2.56-2.69 (1H, m), 3.01-3.30 (2H, m), 3.47-3.61 (1H, m), 3.84 (3H, s), 3.96-4.08 (1H, m), 4.49-4.60 (1H, m), 5.07-5.11 (1H, m), 5.33-5.35 (1H, m), 6.77-6.79 (1H, m), 6.89-6.94 (1H, m), 7.22-7.33 (2H, m), 7.54-7.51
(2H, m).

### Example 550

2-(1-(2-[(4R,6R)-8-Chloro-6-(3-methoxy-2-methylphenyl)-1-(trifluoromethyl)-4H,6H-[1,2,4]triazolo[4,3-a] [4,1]benzoxazepin-4-yl]acetyl)-4-piperidinyl)acetic acid

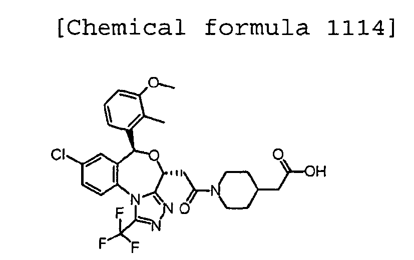

To a dichloromethane solution (20 mL) of tert-butyl 2-(1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-4-piperidinyl)acetate (260 mg) was added trifluoroacetic acid (4.0 mL) under ice cooling. While warming to room temperature, the resulting mixture was stirred for 5 hours. The solvent was distilled off under reduced pressure and the residue thus obtained was purified by thin layer chromatography (dichloromethane:methanol=20:1) to give the title compound (228 mg).
MS (ESI) m/z : 593 (M + H)⁺.
¹H-NMR (CDCl₃) δ: 1.16-1.34 (1H, m), 1.39-1.92 (3H, m), 1.66 and 1.67 (3H, s), 1.97-2.15 (1H, m), 2.24-2.38 (2H, m), 2.57-2.68 (1H, m), 3.03-3.33 (2H, m), 3.44-3.64 (1H, m), 3.84 (3H, s), 3.99-4.10 (1H, m), 4.53-4.62 (1H, m), 5.06-5.12 (1H, m), 5.33-5.36 (1H, m), 6.77-6.79 (1H, m), 6.89-6.93 (1H, m), 7.25-7.34 (2H, m), 7.55-7.51 (2H, m). Elemental analysis for: C₂₈H₂₈ClF₂N₄O₅·0.25H₂O·0.25 n-hexane Calculated: C, 57.24; H, 5.21; N, 9.05.
Found: C, 57.04; H, 5.21; N, 8.91.

### Example 551

Ethyl (3S)-1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-3-piperidine carboxylate

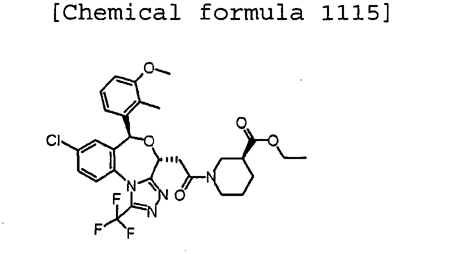

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (404 mg) was obtained from 2-[(4R,6R)-8-chloro-6-(3-methoxy-2-methylphenyl)-1-(trifluoromethyl)-4H,6H-[1,2,4]triazolo[4,3-a] [4,1]benzoxazepin-4-yl]acetic acid (300 mg) by using ethyl (3S)-3-piperidine carboxylate(121 mg), 1-ethyl-3-(3-dimethylaminopropyl)carbodiimide hydrochloride (246 mg), and 1-hydroxybenzotriazole (98 mg). MS (ESI) m/z : 607 (M + H)⁺.
¹H-NMR (CDCl₃) δ: 1.21-1.29 (3H, m), 1.65 and 1.66 (3H, s), 1.68-1.88 (1H, m), 2.00-2.17 (1H, m), 2.38-2.53 (1H, m), 2.71-2.91 (1H, m), 2.95-3.36 (2H, m), 3.45-3.63 (1H, m), 3.64-3.74 (1H, m), 3.84 (3H, s), 3.90-4.02 (2H, m), 4.08-4.20 (2H, m), 4.55-4.68 (1H, m), 5.07-5.13 (1H, m), 5.32-5.38 (1H, m), 6.77-6.81 (1H, m), 6.89-6.94 (1H, m), 7.25-7.34 (2H, m), 7.56-7.49 (2H, m).

### Example 552

(3S)-1-{2-[(4R,6R)-8-Chloro-6-(3-methoxy-2-methylphenyl)-1-(trifluoromethyl)-4H,6H-[1,2,4]triazolo[4,3-a][4,1]benzoxazepin-4-yl]acetyl}-3-piperidine carboxylic acid

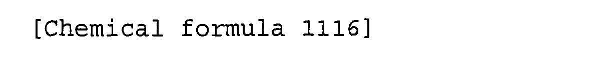

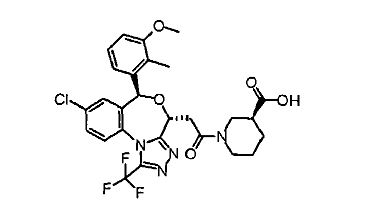

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (196 mg) was obtained from ethyl (3S)-1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-3-piperidine carboxylate (400 mg) by using concentrated hydrochloric acid (10.0 mL).
MS (ESI) m/z : 579 (M + H)⁺.
¹H-NMR (CDCl₃) δ: 1.44-1.58 (1H, m), 1.65 and 1.67 (3H, s), 1.68-2.25 (2H, m), 2.47-2.61 (0.5H, m), 2.80-2.88 (0.5H, m), 2.97-3.11 (1H, m), 3.17-3.32 (1H, m), 3.45-3.55 (1H, m), 3.67-3.75 (0.5H, m), 3.84 (3H, s), 4.00-4.08 (1H, m), 4.42-4.53 (0.5H, m), 5.07-5.13 (1H, m), 5.33-5.37 (1H, m), 6.77-6.80 (1H, m), 6.89-6.93 (1H, m), 7.20-7.35 (2H, m), 7.56-7.50 (2H, m).
Elemental analysis for: C₂₇H₂₆ClF₂N₄O₅·0.5H₂O·0.25 n-hexane
Calculated: C, 56.16; H, 5.04; N, 9.19.
Found: C, 55.88; H, 4.75; N, 9.19.

### Example 553

Ethyl 1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-4-piperidine carboxylate

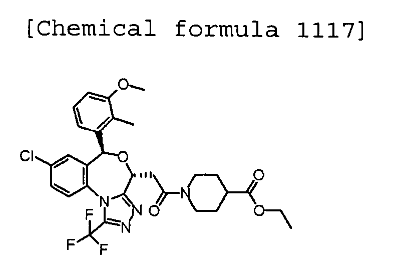

In a similar manner to that employed for the synthesis of ethyl 2-(1-(2-(8-chloro-6-(2,3-dimethoxyphenyl)-4H,6H-pyrrolo[(1,2-a)][(4,1)]benzoxazepin-4-yl)acetyl)-4-piperidinyl)acetate, the title compound (240 mg) was obtained from 2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetic acid (300 mg) by using ethyl 4-piperidine carboxylate (121 mg), 1-ethyl-3-(3-dimethylaminopropyl)carbodiimide hydrochloride (246 mg), and 1-hydroxybenzotriazole (98 mg).
MS (ESI) m/z : 607 (M + H)⁺.
¹H-NMR (CDCl₃) δ: 1.22-1.29 (3H, m), 1.59-1.77 (2H, m), 1.66 and 1.66 (3H, s), 1.81-2.11 (2H, m), 2.49-2.61 (1H, m), 2.84-2.97 (1H, m), 3.14-3.34 (2H, m), 3.49-3.62 (1H, m), 3.84 (3H, s), 3.91-4.05 (1H, m), 4.09-4.20 (2H, m), 4.29-4.38 (1H, m), 5.06-5.11 (1H, m), 5.33-5.36 (1H, m), 6.77-6.80 (1H, m), 6.88-6.94 (1H, m), 7.22-7.35 (2H, m), 7.54-7.51 (2H, m).

### Example 554

1-{2-[(4R,6R)-8-Chloro-6-(3-methoxy-2-methylphenyl)-1-(trifluoromethyl)-4H,6H-[1,2,4]triazolo[4,3-a][4,1]benzoxazepin-4-yl]acetyl}-4-piperidine carboxylic acid

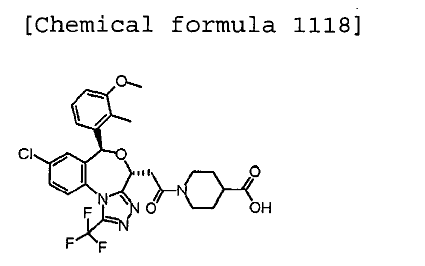

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (106 mg) was obtained from ethyl 1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-4-piperidine carboxylate (237 mg) by using concentrated hydrochloric acid (10.0 mL).
MS (ESI) m/z : 579 (M + H)⁺.
¹H-NMR (CDCl₃) δ: 1.66 and 1.66 (3H, s), 1.67-2.10 (4H, m), 2.55-2.68 (1H, m), 2.88-2.99 (1H, m), 3.17-3.38 (2H, m), 3.51-3.62 (1H, m), 3.83-3.84 (3H, m), 3.93-4.05 (1H, m), 4.29-4.37 (1H, m), 5.06-5.11 (1H, m), 5.33-5.36 (1H, m), 6.77-6.80 (1H, m), 6.88-6.93 (1H, m), 7.23-7.34 (2H, m), 7.56-7.52 (2H, m).
Elemental analysis for: C₂₇H₂₆ClF₂N₄O₅·0.5H₂O·0.25 n-hexane
Calculated: C, 56.16; H, 5.04; N, 9.19.
Found: C, 55.85; H, 4.74; N, 9.17.

### Example 555

tert-Butyl 2-(1-{2-[8-chloro-1-[chloro(difluoro)methyl]-6-(3-methoxy-2-methylphenyl)-4H,6H-[1,2,4]triazolo[4,3-a] [4,1]benzoxazepin-4-yl]acetyl}-4-piperidinyl)acetate

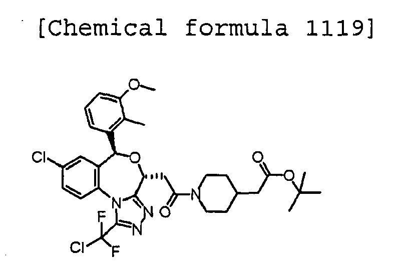

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (270 mg) was obtained from 2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetic acid (150 mg) by using tert-butyl 2-(4-piperidinyl)acetate (74 mg), 1-ethyl-3-(3-dimethylaminopropyl)carbodiimide hydrochloride (119 mg), and 1-hydroxybenzotriazole (47 mg). MS (ESI) m/z : 665 (M + H)⁺.
¹H-NMR (CDCl₃) δ: 1.08-1.44 (2H, m), 1.45 and 1.45 (9H, s), 1.66 and 1.67 (3H, s), 1.69-1.85 (2H, m), 1.94-2.06 (1H, m), 2.11-2.21 (2H, m), 2.57-2.67 (1H, m), 3.02-3.29 (2H, m), 3.47-3.60 (1H, m), 3.84 (3H, s), 3.98-4.07 (1H, m), 4.50-4.61 (1H, m), 5.04-5.08 (1H, m), 5.33-5.35 (1H, m), 6.76-6.78 (1H, m), 6.88-6.93 (1H, m), 7.21-7.35 (2H, m), 7.49-7.54 (1H, m), 7.60-7.56 (1H, m).

### Example 556

2-(1-{2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetyl}-4-piperidinyl)acetic acid

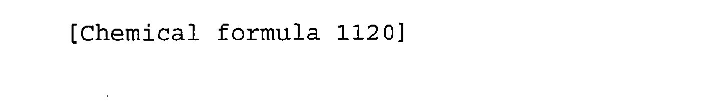

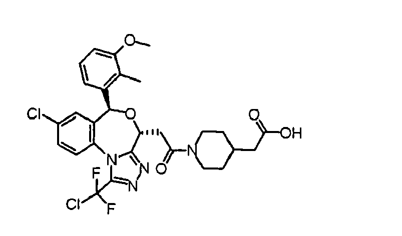

The title compound (157 mg) was obtained from tert-butyl 2-(1-{2-[8-chloro-1-[chloro(difluoro)methyl]-6-(3-methoxy-2-methylphenyl)-4H,6H-[1,2,4]triazolo[4,3-a][4,1]benzoxazepin-4-yl]acetyl}-4-piperidinyl)acetate (200 mg) by using trifluoroacetic acid (2.0 mL).
MS (ESI) m/z : 609(M + H)⁺.
¹H-NMR (CDCl₃) δ: 1.11-1.33 (2H, m), 1.39-1.54 (0.5H, m), 1.66 and 1.68 (3H, s), 1.73-1.92 (2.5H, m), 2.20-2.38 (2H, m), 2.58-2.68 (1H, m), 3.04-3.34 (2H, m), 3.44-3.63 (1H, m), 3.83 and 3.84 (3H, s), 4.00-4.11 (1H, m), 4.52-4.63 (1H, m), 5.03-5.08 (1H, m), 5.33-5.36 (1H, m), 6.77 (1H, d, J = 2.2 Hz), 6.89-6.94 (1H, m), 7.25-7.34 (2H, m), 7.54-7.49 (1H, m), 7.61-7.56 (1H, m).
Elemental analysis for: C₂₈H₂₈Cl₂F₂N₄O₅·1.0H₂O
Calculated: C, 53.60; H, 4.82; N, 8.93.
Found: C, 53.82; H, 4.65; N, 8.80.

### Example 557

Ethyl (3S)-1-{2-[(trans)-8-chloro-1-[chloro(difluoro)methyl]-6-(3-methoxy-2-methylphenyl)-4H,6H-[1,2,4]triazolo[4,3-a] [4,1]benzoxazepin-4-yl]acetyl}-3-piperidine carboxylate

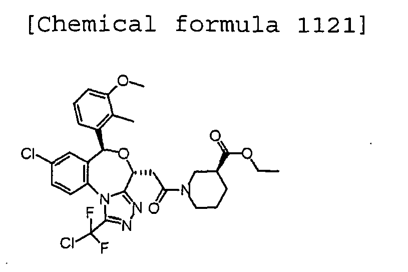

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (190 mg) was obtained from 2-[(trans)-8-chloro-1-[chloro(difluoro)methyl]-6-(3-methoxy-2-methylphenyl)-4H,6H-[1,2,4]triazolo[4,3-a] [4,1]benzoxazepin-4-yl] acetic acid (150 mg) by using ethyl (3S)-3-piperidine carboxylate (58 mg), 1-ethyl-3-(3-dimethylaminopropyl)carbodiimide hydrochloride (192 mg), and 1-hydroxybenzotriazole (47 mg). MS (ESI) m/z : 623 (M + H)⁺.
¹H-NMR (CDCl₃) δ: 1.20-1.31 (3H, m), 1.62-1.85 (5H, m), 1.98-2.17 (1H, m), 2.40-2.51 (1H, m), 2.72-2.92 (1H, m), 2.94-3.36 (2H, m), 3.44-3.74 (2H, m), 3.83 (3H, s), 3.89-4.04 (1.5H, m), 4.08-4.20 (2H, m), 4.55-4.68 (0.5H, m), 5.03-5.10 (1H, m), 5.32-5.37 (1H, m), 6.76-6.79 (1H, m), 6.88-6.94 (1H, m), 7.34-7.24 (2H, m), 7.61-7.49 (2H, m).

### Example 558

(3S)-1-{2-[(trans)-8-Chloro-1-[chloro(difluoro)methyl]-6-(3-methoxy-2-methylphenyl)-4H,6H-[1,2,4]triazolo[4,3-a][4,1]benzoxazepin-4-yl]acetyl}-3-piperidine carboxylic acid

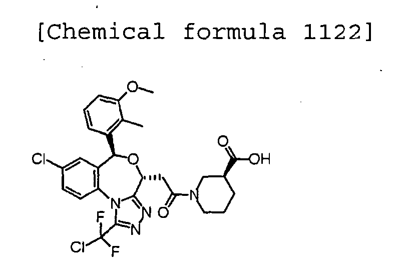

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (30 mg) was obtained from ethyl (3S)-1-{2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetyl}-3-piperidine carboxylate (185 mg) by using concentrated hydrochloric acid (5.0 mL).
MS (ESI) m/z : 595 (M + H)⁺.
¹H-NMR (CDCl₃) δ: 1.46-1.58 (1H, m), 1.60-2.18 (5H, m), 2.49-2.62 (1H, m), 2.80-2.89 (0.5H, m), 2.95-3.12 (1H, m), 3.14-3.31 (1.5H, m), 3.33-3.44 (0.5H, m), 3.44-3.78 (2H, m), 3.83 (3H, s), 3.86-4.11 (1H, m), 5.04-5.11 (0.5H, m), 5.33-5.37 (1H, m), 6.76-6.80 (1H, m), 6.88-6.94 (1H, m), 7.25-7.34 (2H, m), 7.62-7.50 (2H, m).
Elemental analysis for: C₂₇H₂₆Cl₂F₂N₄O₅·0.5H₂O
Calculated: C, 53.65; H, 4.50; N, 9.27.
Found: C, 53.67; H, 4.48; N, 9.22.

### Example 559

Ethyl 1-{2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetyl}-4-piperidine carboxylate

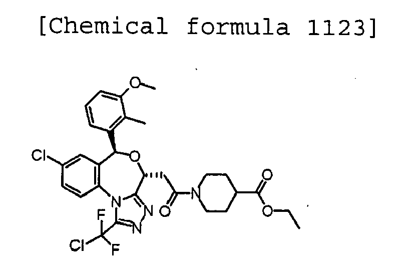

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (190 mg) was obtained from 2-[(trans)-8-chloro-1-[chloro(difluoro)methyl]-6-(3-methoxy-2-methylphenyl)-4H,6H-[1,2,4]triazolo[4,3-a] [4,1]benzoxazepin-4-yl] acetic acid (150 mg) by using ethyl 4-piperidine carboxylate (58 mg), 1-ethyl-3-(3-dimethylaminopropyl)carbodiimide hydrochloride (192 mg), and 1-hydroxybenzotriazole (47 mg). MS (ESI) m/z : 623 (M + H)⁺.
¹H-NMR (CDCl₃) δ: 1.23-1.33 (3H, m), 1.60-1.89 (5H, m), 1.89-2.05 (2H, m), 2.48-2.60 (1H, m), 2.83-2.96 (1H, m), 3.15-3.35 (2H, m), 3.48-3.61 (1H, m), 3.83 (3H, s), 3.92-4.04 (1H, m), 4.11-4.20 (2H, m), 4.28-4.37 (1H, m), 5.03-5.08 (1H, m), 5.33-5.35 (1H, m), 6.75-6.79 (1H, m), 6.88-6.93 (1H, m), 7.22-7.34 (2H, m), 7.61-7.49 (2H, m).

### Example 560

1-{2-[(trans)-8-Chloro-1-[chloro(difluoro)methyl]-6-(3-methoxy-2-methylphenyl)-4H,6H-[1,2,4]triazolo[4,3-a][4,1]benzoxazepin-4-yl]acetyl}-4-piperidine carboxylic acid

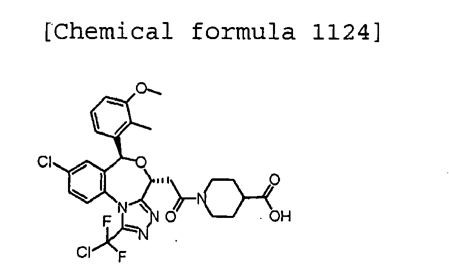

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (79 mg) was obtained from ethyl 1-{2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetyl}-4-piperidine carboxylate (185 mg) by using concentrated hydrochloric acid (5.0 mL).
MS (ESI) m/z : 579 (M + H)⁺.
¹H-NMR (CDCl₃) δ: 1.59-2.14 (4H, m), 1.66 and 1.66 (3H, s), 2.56-2.68 (1H, m), 2.89-2.99 (1H, m), 3.18-3.38 (2H, m), 3.52-3.61 (1H, m), 3.83 and 3.84 (3H, s), 3.92-4.05 (1H, m), 4.28-4.38 (1H, m), 5.02-5.10 (1H, m), 5.34 (1H, s), 6.76-6.79 (1H, m), 6.88-6.94 (1H, m), 7.36-7.22 (2H, m), 7.61-7.50 (2H, m).
Elemental analysis for: C₂₇H₂₆ClF₂N₄O₅·0.5H₂O
Calculated: C, 53.65; H, 4.50; N, 9.27.
Found: C, 53.44; H, 4.42; N, 9.41.

### Referential Example 371

tert-Butyl 4-chloro-2-hydroxyphenylcarbamate

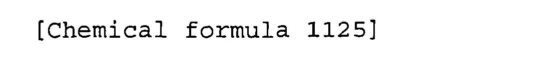

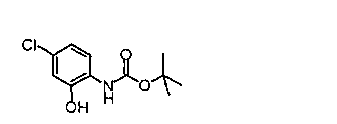

To a tert-butanol solution (500 mL) of 2-amino-5-chlorophenol (24.9 g) was added di-tert-butyl dicarbonate (37.9 g). The resulting mixture was stirred at room temperature for 2 hours. The solvent was distilled off under reduced pressure. The residue thus obtained was solidified from diethyl ether-hexane and collected by filtration. The resulting solid was washed with hexane and dried to give the title compound (28.2 g).
MS (ESI) m/z : 266 (M + Na)⁺.
¹H-NMR (CDCl3) δ: 1.53 (9H, s), 6.56 (1H, s), 6.80-6.85 (1H, m), 6.93 (1H, d, J = 8.6 Hz), 6.99-6.97 (1H, m), 8.40
(1H, s).

### Referential Example 372

tert-Butyl 2-[3-(benzyloxy)propoxy]-4-chlorophenylcarbamate

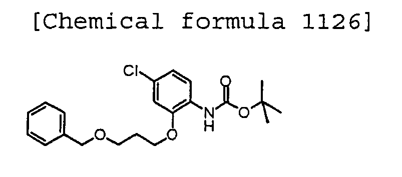

To a tetrahydrofuran solution (200 mL) of tert-butyl 4-chloro-2-hydroxyphenylcarbamate (5.00 g) were added triphenylphosphine (8.07 g) and 3-benzyloxypropane-1-ol (5.12 g, 3.37 mL). Under ice cooling, a toluene solution (13.4 g, 14.0 mL) of 40% diethylazodicarboxylate was added dropwise to the resulting mixture. After stirring at room temperature for 4 hours, the reaction mixture was concentrated under reduced pressure. The residue thus obtained was purified by silica gel column chromatography (n-hexane:ethyl acetate=6:1) to give the title compound (6.05 g).
MS (ESI) m/z : 414 (M + Na)⁺.
¹H-NMR (CDCl3) δ: 1.51 (9H, s), 2.08-2.15 (2H, m), 3.62-3.66 (2H, m), 4.09-4.15 (2H, m), 4.55 (2H, s), 6.82-6.85 (1H, m), 6.88-6.94 (1H, m), 6.95-7.04 (1H, m), 7.26-7.36 (5H, m), 8.06-7.97 (1H, m).

### Referential Example 373

tert-Butyl 4-chloro-2-(3-hydroxypropoxy)phenylcarbamate

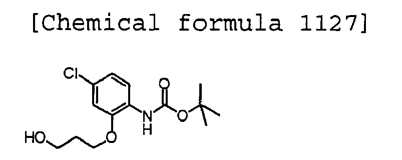

To an ethyl acetate solution (154 mL) of tert-butyl 2-[3-(benzyloxy)propoxy]-4-chlorophenylcarbamate (6.04 g) was added 10% palladium-carbon (51.7%, 0.9 g). In a hydrogen atmosphere, the resulting mixture was stirred for 15 hours. The reaction mixture was filtered through Celite 545, followed by sufficient washing with ethyl acetate. The filtrate thus obtained was concentrated under reduced pressure. The residue thus obtained was purified by flash column chromatography (Cartridge B-L, Yamazen Corp.) to give the title compound (4.07 g).
MS (ESI) m/z : 324 (M + Na)⁺.
¹H-NMR (CDCl3) δ: 1.52 (9H, s), 2.02-2.14 (2H, m), 3.85-3.90 (2H, m), 4.08-4.19 (2H, m), 6.85-6.87 (1H, m), 6.89-6.94 (1H, m), 7.02 (1H, s), 8.04-7.96 (1H, m).

### Referential Example 374

3-(2-Amino-5-chlorophenoxy)-1-propanol

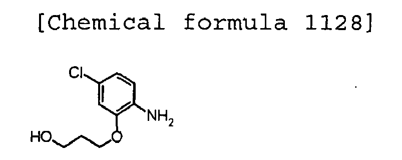

To a dichloromethane solution (190 mL) of tert-butyl 4-chloro-2-(3-hydroxypropoxy)phenylcarbamate (5.82 g) was added trifluoroacetic acid (10 mL) under ice cooling. While warming to room temperature, the resulting mixture was stirred for 22 hours. The solvent was distilled off under reduced pressure. The residue thus obtained was purified by silica gel column chromatography (n-hexane:ethyl acetate=4:1) to give the title compound (3.89 g).
MS (ESI) m/z : 202 (M + Na)⁺.
¹H-NMR (CDCl3) δ: 2.24-2.34 (2H, m), 4.07-4.13 (2H, m), 4.54-4.60 (2H, m), 6.65-6.62 (1H, m), 6.72-6.82 (2H, m).

### Referential Example 375

N-[4-Chloro-2-(3-hydroxypropoxy)phenyl]-2,2-dimethylpropanamide

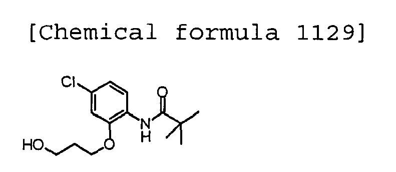

To a dichloromethane solution (16 mL) of 3-(2-amino-5-chlorophenoxy)-1-propanol (326 mg) was added sodium hydrogen carbonate (203 mg). Under ice cooling, pivaloyl chloride (239 µl) was added to the resulting mixture.
While warming to room temperature, the resulting mixture was stirred for one hour. To the reaction mixture was added distilled water and the layers separated. The organic layer thus obtained was dried over anhydrous sodium sulfate. The solvent was distilled off under reduced pressure to give the title compound (3.71 g).
MS (ESI) m/z : 286 (M + Na)⁺.
¹H-NMR (CDCl3) δ: 1.31 (9H, s), 2.28-2.35 (2H, m), 4.09-4.18 (2H, m), 4.59-4.55 (2H, m), 6.86 (1H, d, J = 2.2 Hz), 6.99-6.95 (1H, m), 7.95 (1H, s), 8.34 (1H, d, J = 8.6 Hz).

### Referential Example 376

N-[4-Chloro-2-[(2,3-dimethoxyphenyl)(hydroxy)methyl]-6-(3-hydroxypropoxy)phenyl]-2,2-dimethylpropanamide

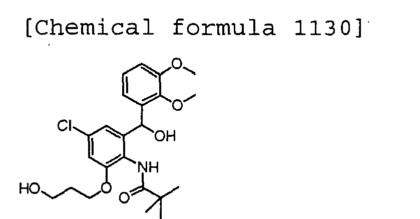

A tetrahydrofuran solution (55 mL) of N-[4-chloro-2-(3-hydroxypropoxy)phenyl]-2,2-dimethylpropanamide(1.54 g) was cooled to -78°C, followed by the dropwise addition of tert-butyl lithium (a 1.43 mol/l pentane solution, 12.5 mL). While warming to -30°C, the mixture was stirred for 3.5 hours. The reaction mixture was then cooled to -78°C again and 2,3-dimethoxybenzaldehyde (1.35 g) was added thereto. While warming to room temperature, the resulting mixture was stirred for 3 hours. To the reaction mixture were added a saturated aqueous solution of ammonium chloride and ethyl acetate and the layers separated. The organic layer thus obtained was washed with saturated brine and dried over anhydrous sodium sulfate. The solvent was distilled off under reduced pressure and the residue was purified by flash column chromatography (Cartridge C-L, Yamazen Corp.) to give the title compound (1.99 g).
MS (ESI) m/z : 434 (M - OH)⁺, 474 (M + Na)⁺.
¹H-NMR (CDCl3) δ: 1.26 (9H, s), 1.97-2.05 (2H, m), 3.62 (3H, s), 3.68-3.75 (1H, m), 3.80 (1H, s), 3.84 (3H, s), 3.85-3.90 (1H, m), 4.10-4.15 (2H, m), 4.41-4.44 (1H, m), 5.95-5.97 (1H, m), 6.79 (1H, d, J = 2.2 Hz), 6.84-6.91 (2H, m), 7.11-7.02 (2H, m), 7.65 (1H, s).

### Referential Example 377

3-{2-Amino-5-chloro-3-[(2,3-dimethoxyphenyl)(hydroxy)methyl]phenoxy}-1-propanol

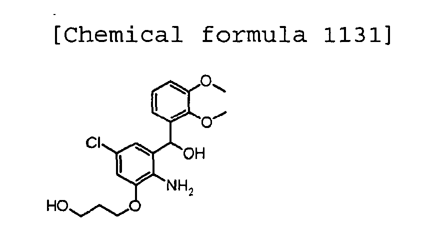

To an ethanol solution (15.5 mL) of N-[4-chloro-2-[(2,3-dimethoxyphenyl) (hydroxy)methyl]-6-(3-hydroxypropoxy)phenyl]-2,2-dimethylpropanamide (2.06 g) was added potassium hydroxide (2.40 g). The resulting mixture was stirred at 80°C for 5.5 hours. The solvent was distilled off under reduced pressure. The residue thus obtained was purified by flash column chromatography (Cartridge C-L, Yamazen Corp.) to give the title compound (1.11 g).
¹H-NMR (CDCl3) δ: 2.02-2.10 (3H, m), 3.84 (3H, s), 3.84-3.88 (2H, m), 3.88 (3H, s), 4.08-4.16 (2H, m), 6.07 (1H, s), 6.73-6.78 (2H, m), 6.87-6.91 (2H, m), 7.09-7.03 (1H, m).

### Referential Example 378

3-{5-Chloro-2-[(2,4-dimethoxybenzyl)amino]-3-[(2,3-dimethoxyphenyl)(hydroxy)methyl]phenoxy}-1-propanol

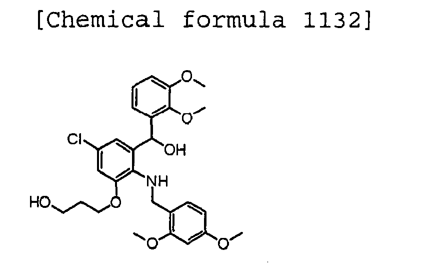

In a similar manner to that employed for the synthesis of {5-chloro-2-[(2,4-dimethoxybenzyl)amino]-3-methoxyphenyl}(2,3-dimethoxyphenyl)methanol, the title compound (1.27 g) was obtained from 3-{2-amino-5-chloro-3-[(2,3-dimethoxyphenyl)(hydroxy)methyl]phenoxy}-1-propanol(1.10 g) by using 2,4-dimethoxybenzaldehyde (0.60 g) and sodium borohydride(0.17 g).
MS (ESI) m/z : 518 (M + H)⁺.
¹H-NMR (CDCl3) δ: 1.99-2.08 (2H, m), 3.69 (3H, s), 3.70-3.75 (1H, m), 3.78 (3H, s), 3.79 (3H, s), 3.82-3.86 (1H, m), 3.87 (3H, s), 3.97-4.22 (3H, m), 4.20 (1H, d, J = 13.2 Hz), 6.17 (1H, s), 6.35-6.39 (1H, m), 6.44-6.47 (1H, m), 6.55-6.57 (1H, m), 6.75-6.78 (1H, m), 6.86-6.90 (1H, m), 7.10-6.93 (3H, m).

### Referential Example 379

Ethyl 2-[(trans)-7-chloro-1-(2,4-dimethoxybenzyl)-5-(2,3-dimethoxyphenyl)-9-(3-hydroxypropoxy)-2-oxo-1,2,3,5-tetrahydro-4,1-benzoxazepin-3-yl]acetate

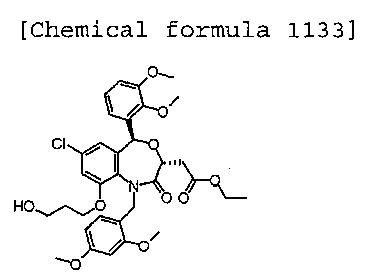

In a similar manner to that employed for the synthesis of ethyl 2-[(trans)-7-chloro-1-(2,4-dimethoxybenzyl)-5-(2,3-dimethoxyphenyl)-9-methoxy-2-oxo-1,2,3,5-tetrahydro-4,1-benzoxazepin-3-yl]acetate, the title compound (1.26 g) was obtained from 3-{5-chloro-2-[(2,4-dimethoxybenzyl)amino]-3-[(2,3-dimethoxyphenyl)(hydroxy)methyl]phenoxy}-1-propanol(1.01 g) while carrying out amidation by using sodium hydrogen carbonate (0.49 g) and monoethyl chlorofumarate (0.47 g) and then using potassium carbonate (1.34 g).
MS (ESI) m/z : 666 (M + H)⁺.
¹H-NMR (CDCl₃) δ: 1.23-1.27 (3H, m), 2.04-2.14 (2H, m), 2.71-2.78 (1H, m), 3.05 (3H, s), 3.06-3.12 (1H, m), 3.56 (3H, s), 3.75 (3H, s), 3.84 (3H, s), 3.85-3.94 (2H, m), 4.05-4.18 (2H, m), 4.18-4.34 (2H, m), 4.37-4.42 (1H, m), 4.82 (1H, d, J = 14.0 Hz), 5.59 (1H, d, J = 14.2 Hz), 5.87 (1H, s), 6.11-6.13 (1H, m), 6.32-6.40 (2H, m), 6.87-6.92 (1H, m), 6.97-7.00 (1H, m), 7.03-7.12 (2H, m), 7.53-7.43
(1H, m).

### Referential Example 380

Ethyl 2-[(trans)-7-chloro-5-(2,3-dimethoxyphenyl)-9-(3-hydroxypropoxy)-2-oxo-1,2,3,5-tetrahydro-4,1-benzoxazepin-3-yl] acetate

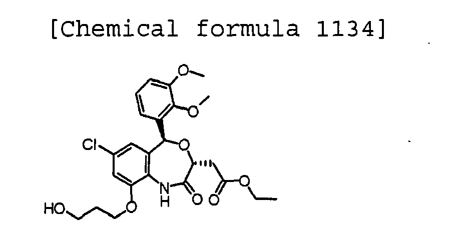

In a similar manner to that employed for the synthesis of ethyl 2-[(trans)-7-chloro-5-(2,3-dimethoxyphenyl)-9-methoxy-2-oxo-1,2,3,5-tetrahydro-4,1-benzoxazepin-3-yl]acetate, the title compound (172 mg) was obtained from ethyl 2-[(trans)-7-chloro-1-(2,4-dimethoxybenzyl)-5-(2,3-dimethoxyphenyl)-9-(3-hydroxypropoxy)-2-oxo-1,2,3,5-tetrahydro-4,1-benzoxazepin-3-yl]acetate (754 mg) by using ceric(IV) ammonium nitrate (1.93 g).
MS (ESI) m/z : 494 (M + H)⁺.
¹H-NMR (CDCl₃) δ: 1.17-1.27 (3H, m), 2.05-2.14 (2H, m), 2.73-2.83 (1H, m), 2.96-3.07 (1H, m), 3.68 (3H, s), 3.68-3.76 (2H, m), 3.88 (3H, s), 4.03-4.13 (3H, m), 4.15-4.27 (2H, m), 4.62-4.70 (1H, m), 6.21 (1H, s), 6.30-6.32 (1H, m), 6.88-6.90 (1H, m), 7.02-6.93 (2H, m), 7.12 (1H, t, J = 8.0 Hz), 8.03 (1H, s).

### Referential Example 381

Ethyl 2-[9-[3-(acetyloxy)propoxy]-7-chloro-5-(2,3-dimethoxyphenyl)-2-oxo-1,2,3,5-tetrahydro-4,1-benzoxazepin-3-yl] acetate

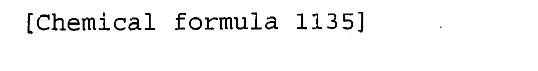

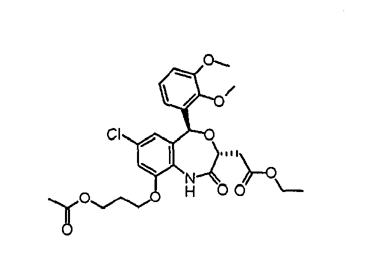

To a dichloromethane solution (3.5 mL) of ethyl 2-[(trans)-7-chloro-5-(2,3-dimethoxyphenyl)-9-(3-hydroxypropoxy)-2-oxo-1,2,3,5-tetrahydro-4,1-benzoxazepin-3-yl]acetate (171 mg) were added triethylamine (145 µl) and 4-(N,N-dimethylamino)pyridine (4.2 mg). Under ice cooling, acetyl chloride (36.9 µl) was added dropwise. While warming to room temperature, the resulting mixture was stirred for 1.5 hours. To the reaction mixture were added a phosphate buffer solution and dichloromethane and the layers separated. The organic layer thus obtained was washed with distilled water and saturated brine and dried over anhydrous sodium sulfate. The solvent was distilled off under reduced pressure to give the title compound (173 mg).
MS (ESI) m/z : 536 (M + H)⁺.
¹H-NMR (CDCl₃) δ: 1.17-1.22 (3H, m), 2.10 (3H, s), 2.15-2.25 (2H, m), 2.75-2.82 (1H, m), 2.98-3.04 (1H, m), 3.69 (3H, s), 3.89 (3H, s), 4.03-4.19 (4H, m), 4.23-4.34 (2H, m), 4.64-4.70 (1H, m), 6.21 (1H, s), 6.31-6.33 (1H, m), 6.84-6.86 (1H, m), 6.94-7.01 (2H, m), 7.14-7.09 (1H, m), 7.83 (1H, s).

### Referential Example 382

Ethyl 2-[9-[(trans)-3-(acetyloxy)propoxy]-7-chloro-5-(2,3-dimethoxyphenyl)-2-thioxo-1,2,3,5-tetrahydro-4,1-benzoxazepin-3-yl]acetate

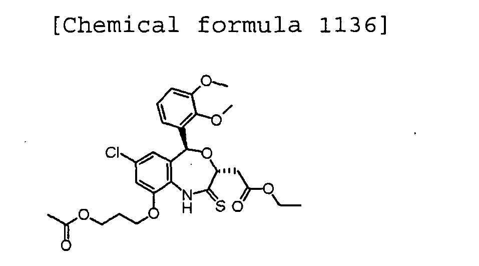

In a similar manner to that employed for the synthesis of ethyl 2-[(trans)-7-chloro-5-(2,3-dimethoxyphenyl)-9-methoxy-2-thioxo-1,2,3,5-tetrahydro-4,1-benzoxazepin-3-yl]acetate, the title compound (340 mg) was obtained from ethyl 2-[9-[3-(acetyloxy)propoxy]-7-chloro-5-(2,3-dimethoxyphenyl)-2-oxo-1,2,3,5-tetrahydro-4,1-benzoxazepin-3-yl]acetate (473 mg) by using sodium hydrogen carbonate (222 mg) and diphosphorus pentasulfide (492 mg). MS (ESI) m/z : 552 (M + H)⁺.
¹H-NMR (CDCl₃) δ: 1.20-1.26 (3H, m), 2.10 (3H, s), 2.18-2.25 (2H, m), 2.92-3.00 (1H, m), 3.21-3.28 (1H, m), 3.65 (3H, s), 3.88 (3H, s), 4.08-4.20 (4H, m), 4.22-4.37 (2H, m), 4.72-4.78 (1H, m), 6.11 (1H, s), 6.26-6.28 (1H, m), 6.88-6.90 (1H, m), 6.94-6.98 (1H, m), 7.05-7.09 (1H, m), 7.16-7.11 (1H, m), 9.62 (1H, s).

### Referential Example 383

Ethyl 2-[(trans)-10-[3-(acetyloxy)propoxy]-8-chloro-6-(2,3-dimethoxyphenyl)-1-(trifluoromethyl)-4H,6H-[1,2,4]triazolo[4,3-a][4,1]benzoxazepin-4-yl]acetate

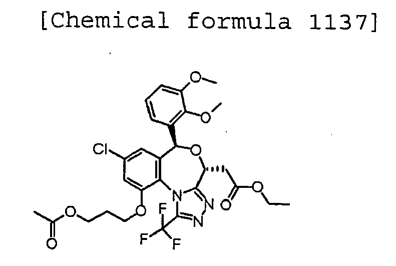

In a similar manner to that employed for the synthesis of ethyl 2-[(trans)-8-chloro-6-(2,3-dimethoxyphenyl)-10-methoxy-1-(trifluoromethyl)-4H,6H-[1,2,4]triazolo[4,3-a] [4,1]benzoxazepin-4-yl]acetate, the title compound (402 mg) was obtained from ethyl 2-[9-[(trans)-3-(acetyloxy)propoxy]-7-chloro-5-(2,3-dimethoxyphenyl)-2-thioxo-1,2,3,5-tetrahydro-4,1-benzoxazepin-3-yl]acetate (440 mg) by using hydrazine monohydrate (77.3 µl), trifluoroacetic anhydride (554 µl), and trifluoroacetic acid (222 µl).
¹H-NMR (CDCl₃) δ: 1.24-1.31 (3H, m), 2.05 (3H, s), 2.06-2.23 (2H, m), 3.22-3.30 (1H, m), 3.40-3.49 (1H, m), 3.45 (3H, s), 3.85 (3H, s), 4.06-4.30 (6H, m), 4.87-4.91 (1H, m), 5.45 (1H, s), 6.39-6.40 (1H, m), 6.93-7.00 (1H, m), 7.08-7.06 (1H, m), 7.19-7.12 (2H, m).

### Example 561

2-[(trans)-8-Chloro-6-(2,3-dimethoxyphenyl)-10-(3-hydroxypropoxy)-1-(trifluoromethyl)-4H,6H-[1,2,4]triazolo[4,3-a] [4,1]benzoxazepin-4-yl]acetic acid

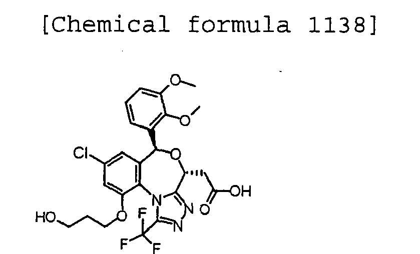

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (150 mg) was obtained from ethyl 2-[(trans)-10-[3-(acetyloxy)propoxy]-8-chloro-6-(2,3-dimethoxyphenyl)-1-(trifluoromethyl)-4H,6H-[1,2,4]triazolo[4,3-a][4,1]benzoxazepin-4-yl]acetate (262 mg) by using concentrated hydrochloric acid(2.0 mL).
MS (ESI) m/z : 586 (M + H)⁺.
¹H-NMR (CDCl₃) δ: 1.88-2.01 (2H, m), 3.09-3.18 (1H, m), 3.34-3.43 (1H, m), 3.46 (3H, s), 3.47-3.58 (2H, m), 3.85 (3H, s), 4.14-4.22 (1H, m), 4.26-4.33 (1H, m), 4.72-4.78 (1H, m), 5.47 (1H, s), 6.40-6.38 (1H, m), 6.99-6.94 (1H, m), 7.21-7.10 (3H, m).
Elemental analysis for: C₂₄H₂₃ClF₃N₃O₇·1.5H₂O·0.3CHCl₃
Calculated: C, 47.02; H, 4.27; N, 6.77.
Found: C, 47.21; H, 4.12; N, 6.85.

### Referential Example 384

N-[4-Chloro-2-[(2-chloro-3-methoxyphenyl)(hydroxy)methyl]-6-(3-hydroxypropoxy)phenyl]-2,2-dimethylpropanamide

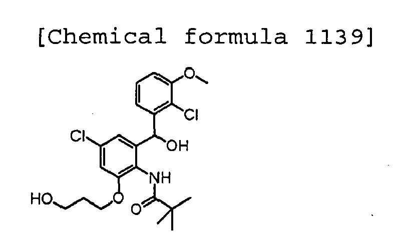

To a tetrahydrofuran solution (400 mL) of N-[4-chloro-2-(3-hydroxypropoxy)phenyl]-2,2-dimethylpropanamide (11.5 g) were added dropwise sec-butyl lithium (a 0.99 mol/l cyclohexane-pentane solution, 89.5 mL) and tert-butyl lithium (a 1.43 mol/l pentane solution, 28.2 mL) successively. While warming to -30°C, the reaction mixture was stirred for 2.5 hours. The reaction mixture was cooled to -78°C again and 2-chloro-3-methoxybenzaldehyde (10.3 g) was added thereto. While warming to room temperature, the resulting mixture was stirred for 3 hours. To the reaction mixture were added a saturated aqueous solution of ammonium chloride and ethyl acetate and the layers separated. The organic layer thus obtained was washed with saturated brine and dried over anhydrous sodium sulfate. The solvent was distilled off under reduced pressure. The residue thus obtained was solidified from diethyl ether-n-hexane. The resulting solid was collected by filtration and washed with n-hexane. The powder thus obtained was dried under reduced pressure to give the title compound (14.8 g).
¹H-NMR (CDCl3) δ: 1.36 (9H, s), 1.67 (1H, br s), 2.02-2.09 (2H, m), 3.81-3.87 (2H, m), 3.88 (3H, s), 4.09-4.14 (2H, m), 4.84-4.86 (1H, m), 5.93-5.95 (1H, m), 6.64-6.65 (1H, m), 6.81-6.83 (1H, m), 6.89-6.93 (1H, m), 7.32-7.38 (2H, m), 7.53-7.49 (1H, m).

### Referential Example 385

3-{2-Amino-5-chloro-3-[(2-chloro-3-methoxyphenyl)(hydroxy)methyl]phenoxy}-1-propanol

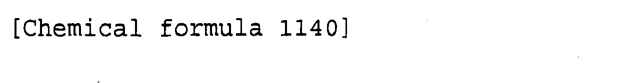

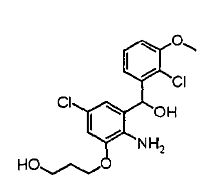

In a similar manner to that employed for the synthesis of 3-{2-amino-5-chloro-3-[(2,3-dimethoxyphenyl)(hydroxy)methyl]phenoxy}-1-propanol, the title compound (7.27 g) was obtained from N-[4-chloro-2-[(2-chloro-3-methoxyphenyl)(hydroxy)methyl]-6-(3-hydroxypropoxy)phenyl]-2,2-dimethylpropanamide (14.8 g) by using potassium hydroxide (17.1 g).
MS (ESI) m/z : 372 (M + H)⁺.
¹H-NMR (CDCl3) δ: 2.04-2.11 (3H, m), 3.84-3.89 (2H, m), 3.93 (3H, s), 4.11-4.15 (2H, m), 6.22 (1H, s), 6.56-6.58 (1H, m), 6.76-6.78 (1H, m), 6.92-6.96 (1H, m), 7.09-7.13 (1H, m), 7.34-7.27 (1H, m).

### Referential Example 386

3-{5-Chloro-3-[(2-chloro-3-methoxyphenyl)(hydroxy)methyl]-2-[(2,4-dimethoxybenzyl)amino]phenoxy}-1-propanol

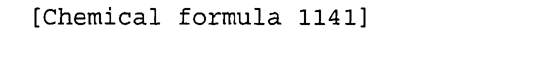

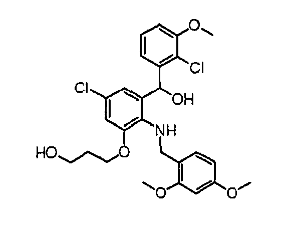

In a similar manner to that employed for the synthesis of {5-chloro-2-[(2,4-dimethoxybenzyl)amino]-3-methoxyphenyl}(2,3-dimethoxyphenyl)methanol, the title compound (10.2 g) was obtained from 3-{2-amino-5-chloro-3-[(2-chloro-3-methoxyphenyl)(hydroxy)methyl]phenoxy}-l-propanol (7.26 g) by using 2,4-dimethoxybenzaldehyde(4.21 g) and sodium borohydride (1.11 g).
MS (ESI) m/z : 522 (M + H)⁺.
¹H-NMR (CDCl3) δ: 1.99-2.09 (2H, m), 3.78-3.86 (6H, m), 3.92-3.96 (3H, m), 4.02-4.16 (4H, m), 4.18-4.28 (1H, m), 4.61 (1H, s), 6.26-6.29 (1H, m), 6.37-6.51 (4H, m), 6.77-6.79 (1H, m), 6.92-6.96 (1H, m), 7.05-7.09 (1H, m), 7.15-7.25 (1H, m), 7.35-7.28 (1H, m).

### Referential Example 387

Ethyl 2-[(trans)-7-chloro-5-(2-chloro-3-methoxyphenyl)-1-(2,4-dimethoxybenzyl)-9-(3-hydroxypropoxy)-2-oxo-1,2,3,5-tetrahydro-4,1-benzoxazepin-3-yl]acetate

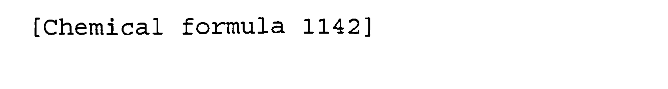

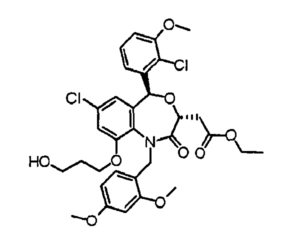

In a similar manner to that employed for the synthesis of ethyl 2-[(trans)-7-chloro-1-(2,4-dimethoxybenzyl)-5-(2,3-dimethoxyphenyl)-9-methoxy-2-oxo-1,2,3,5-tetrahydro-4,1-benzoxazepin-3-yl]acetate, the title compound (11.3 g) was obtained from 3-{5-chloro-3-[(2-chloro-3-methoxyphenyl)(hydroxy)methyl]-2-[(2,4-dimethoxybenzyl)amino]phenoxy}-1-propanol(10.2 g) while carrying out amidation using sodium hydrogen carbonate (4.92 g) and monoethyl chlorofumarate (4.76 g) and then using potassium carbonate (13.5 g).
MS (ESI) m/z : 670 (M + H)⁺.
¹H-NMR (CDCl₃) δ: 1.19-1.31 (3H, m), 2.07-2.15 (2H, m), 2.69-2.86 (2H, m), 3.01-3.13 (1H, m), 3.47 (3H, s), 3.74 (3H, s), 3.75-3.82 (1H, m), 3.88 (3H, s), 4.10-4.43 (6H, m), 4.88-4.94 (1H, m), 5.52-5.58 (1H, m), 5.70-5.75 (1H, m), 5.94-5.97 (1H, m), 6.26-6.29 (1H, m), 6.33-6.39 (1H, m), 6.89-6.95 (1H, m), 7.01-7.03 (1H, m), 7.16-7.20 (1H, m), 7.26-7.31 (1H, m), 7.39-7.35 (1H, m).

### Referential Example 388

Ethyl 2-[(trans)-9-[3-(acetyloxy)propoxy]-7-chloro-5-(2-chloro-3-methoxyphenyl)-1-(2,4-dimethoxybenzyl)-2-oxo-1,2,3,5-tetrahydro-4,1-benzoxazepin-3-yl]acetate

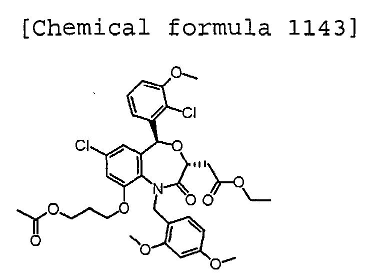

In a similar manner to that employed for the synthesis of ethyl 2-[(trans)-9-[3-(acetyloxy)propoxy]-7-chloro-5-(2,3-dimethoxyphenyl)-2-oxo-1,2,3,5-tetrahydro-4,1-benzoxazepin-3-yl]acetate, the title compound (8.40 g) was obtained from ethyl 2-[(trans)-7-chloro-5-(2-chloro-3-methoxyphenyl)-1-(2,4-dimethoxybenzyl)-9-(3-hydroxypropoxy)-2-oxo-1,2,3,5-tetrahydro-4,1-benzoxazepin-3-yl]acetate (12.6 g) by using triethylamine(8.15 mL) and 4-(N,N-dimethylamino)pyridine (0.24 g) and using acetyl chloride (2.08 mL) under ice cooling.
¹H-NMR (CDCl₃) δ: 1.23-1.28 (3H, m), 2.08 (3H, s), 2.17-2.28 (2H, m), 2.77-2.86 (1H, m), 2.97-3.05 (1H, m), 3.48 (3H, s), 3.74 (3H, s), 3.88 (3H, s), 4.09-4.23 (4H, m), 4.27-4.31 (2H, m), 4.38-4.43 (1H, m), 4.87-4.94 (1H, m), 5.48-5.57 (1H, m), 5.72 (1H, s), 5.95-5.97 (1H, m), 6.27-6.29 (1H, m), 6.33-6.38 (1H, m), 6.89-6.93 (1H, m), 6.96-6.98 (1H, m), 7.17-7.21 (1H, m), 7.26-7.31 (1H, m), 7.37-7.33 (1H, m).

### Referential Example 389

Ethyl 2-[(trans)-9-[3-(acetyloxy)propoxy]-7-chloro-5-(2-chloro-3-methoxyphenyl)-2-oxo-1,2,3,5-tetrahydro-4,1-benzoxazepin-3-yl]acetate

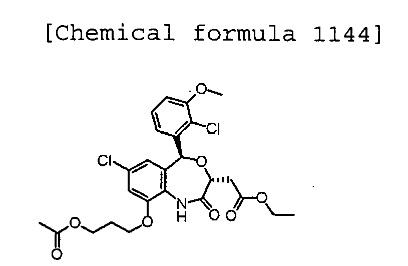

In a similar manner to that employed for the synthesis of ethyl 2-[7-chloro-5-(2,3-dimethoxyphenyl)-9-methoxy-2-oxo-1,2,3,5-tetrahydro-4,1-benzoxazepin-3-yl]acetate, the title compound (4.29 g) was obtained from ethyl 2-[(trans)-9-[3-(acetyloxy)propoxy]-7-chloro-5-(2-chloro-3-methoxyphenyl)-1-(2,4-dimethoxybenzyl)-2-oxo-1,2,3,5-tetrahydro-4,1-benzoxazepin-3-yl]acetate (8.40 g) by using ceric(IV) ammonium nitrate (20.1 g).
MS (ESI) m/z : 540 (M + H)⁺.
¹H-NMR (CDCl₃) δ: 1.20-1.29 (3H, m), 2.10 (3H, s), 2.17-2.24 (2H, m), 2.77-2.84 (1H, m), 2.99-3.06 (1H, m), 3.94 (3H, s), 4.08-4.17 (4H, m), 4.23-4.34 (2H, m), 4.62-4.66 (1H, m), 6.19-6.24 (2H, m), 6.88-6.89 (1H, m), 6.96-7.01 (1H, m), 7.10-7.15 (1H, m), 7.36-7.28 (1H, m), 7.81 (1H, s).

### Referential Example 390

Ethyl 2-[(trans)-9-[3-(acetyloxy)propoxy]-7-chloro-5-(2-chloro-3-methoxyphenyl)-2-thioxo-1,2,3,5-tetrahydro-4,1-benzoxazepin-3-yl]acetate

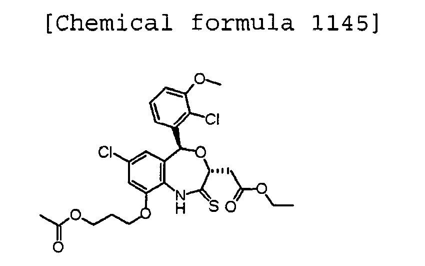

In a similar manner to that employed for the synthesis of ethyl 2-[(trans)-7-chloro-5-(2,3-dimethoxyphenyl)-9-methoxy-2-thioxo-1,2,3,5-tetrahydro-4,1-benzoxazepin-3-yl]acetate, the title compound (3.37 g) was obtained from ethyl 2-[(trans)-9-[3-(acetyloxy)propoxy]-7-chloro-5-(2-chloro-3-methoxyphenyl)-2-oxo-1,2,3,5-tetrahydro-4,1-benzoxazepin-3-yl]acetate (4.29 g) by using sodium hydrogen carbonate (2.00 g) and diphosphorus pentasulfide (3.54 g).
MS (ESI) m/z : 556 (M + H)⁺.
¹H-NMR (CDCl₃) δ: 1.22-1.28 (3H, m), 2.10 (3H, s), 2.18-2.26 (2H, m), 2.96-3.04 (1H, m), 3.21-3.29 (1H, m), 3.93 (3H, s), 4.09-4.21 (4H, m), 4.22-4.38 (2H, m), 4.70-4.75 (1H, m), 6.10 (1H, s), 6.17-6.18 (1H, m), 6.91-6.94 (1H, m), 6.97-7.01 (1H, m), 7.20-7.24 (1H, m), 7.37-7.32 (1H, m), 9.61 (1H, s).

### Referential Example 391

Ethyl 2-[(trans)-10-[3-(acetyloxy)propoxy]-8-chloro-6-(2-chloro-3-methoxyphenyl)-1-(trifluoromethyl)-4H,6H-[1,2,4]triazolo[4,3-a] [4,1]benzoxazepin-4-yl]acetate

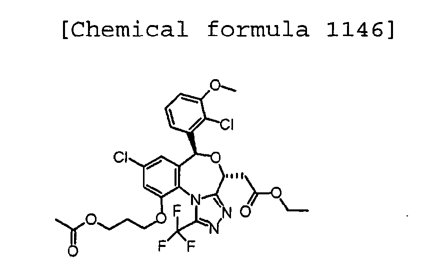

In a similar manner to that employed for the synthesis of ethyl 2-[(trans)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetate,the title compound (1.29 g) was obtained from ethyl 2-[(trans)-9-[3-(acetyloxy)propoxy]-7-chloro-5-(2-chloro-3-methoxyphenyl)-2-thioxo-1,2,3,5-tetrahydro-4,1-benzoxazepin-3-yl]acetate (3.36 g) by using hydrazine monohydrate (0.59 mL) and trifluoroacetic anhydride (5.88 mL).
¹H-NMR (CDCl₃) δ: 1.24-1.30 (3H, m), 2.07 (3H, s), 2.18-2.35 (2H, m), 2.92-3.00 (1H, m), 3.07-3.14 (1H, m), 3.92 (3H, s), 4.07-4.41 (6H, m), 4.62-4.67 (1H, m), 6.12 (1H, s), 6.28 (1H, d, J = 2.2 Hz), 6.97-7.03 (1H, m), 7.06 (1H, d, J = 2.2 Hz), 7.26-7.29 (1H, m), 7.38-7.33 (1H, m).

### Example 562

2-[(trans)-8-Chloro-6-(2-chloro-3-methoxyphenyl)-10-(3-hydroxypropoxy)-1-(trifluoromethyl)-4H,6H-[1,2,4]triazolo[4,3-a] [4,1]benzoxazepin-4-yl]acetic acid

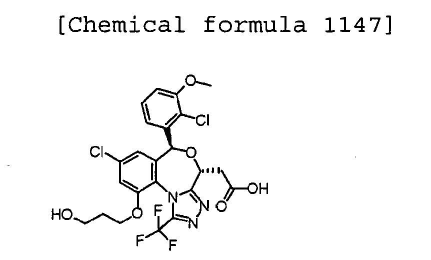

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a] [4,1]benzoxazepin-4-yl]acetic acid, the title compound (70 mg) was obtained from ethyl 2-[(trans)-10-[3-(acetyloxy)propoxy]-8-chloro-6-(2-chloro-3-methoxyphenyl)-1-(trifluoromethyl)-4H,6H-[1,2,4]triazolo[4,3-a] [4,1]benzoxazepin-4-yl]acetate (154 mg) by using concentrated hydrochloric acid (2.0 mL).
MS (ESI) m/z : 562 (M + H)⁺.
¹H-NMR (CDCl₃) δ: 1.85-1.97 (2H, m), 3.01-3.13 (1H, m), 3.27-3.56 (3H, m), 3.90 (3H, s), 4.12-4.34 (2H, m), 4.68-4.81 (1H, m), 5.44 (1H, s), 6.24-6.27 (1H, m), 6.96-7.00 (1H, m), 7.10-7.15 (1H, m), 7.29-7.40 (2H, m).
Elemental analysis for: C₂₃H₂₀Cl₂F₃N₃O₆·1.5H₂O-0.25CHCl₃
Calculated: C, 45.10; H, 3.78; N, 6.79.
Found: C, 45.12; H, 3.57; N, 6.60.

### Example 563

Ethyl 2-(1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-3-azetidinyl)acetate

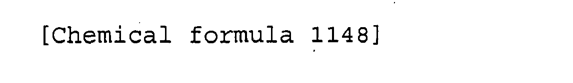

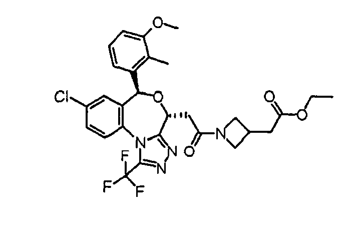

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (510 mg) was obtained from 2-[(4R,6R)-8-chloro-6-(3-methoxy-2-methylphenyl)-1-(trifluoromethyl)-4H,6H-[1,2,4]triazolo[4,3-a] [4,1]benzoxazepin-4-yl]acetic acid (400 mg) by using ethyl 2-(3-azetidinyl)acetate hydrochloride (a 0.3 mol/l ethanol solution, 3.42 mL), 1-ethyl-3-(3-dimethylaminopropyl)carbodiimide hydrochloride (328 mg), 1-hydroxybenzotriazole (131 mg), and triethylamine (143 µl).
MS (ESI) m/z : 593 (M + H) +.
¹H-NMR (CDCl₃) δ: 1.22-1.29 (3H, m), 1.66 (3H, s), 2.59-2.71 (2H, m), 2.91-3.03 (2H, m), 3.13-3.24 (1H, m), 3.65-3.75 (1H, m), 3.84 (3H, s), 3.87-3.94 and 4.09-4.23 (4H, m), 4.37-4.43 and 4.58-4.65 (1H, m), 4.98-5.04 (1H, m), 5.36 (1H, s), 6.78 (1H, s), 6.88-6.95 (1H, m), 7.27-7.36 (2H, m), 7.55-7.48 (2H, m).

### Example 564

2-(1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-3-azetidinyl)acetic acid

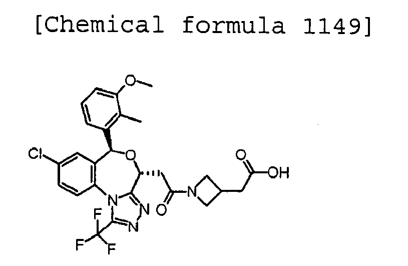

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzoxazepin-4-yl]acetic acid, the title compound (245 mg) was obtained from ethyl 2-(1-{2-[(4R,6R)-8-chloro-6-(3-methoxy-2-methylphenyl)-1-(trifluoromethyl)-4H,6H-[1,2,4]triazolo[4,3-a][4,1]benzoxazepin-4-yl]acetyl}-3-azetidinyl)acetate (505 mg) by using concentrated hydrochloric acid (10.0 mL).
MS (ESI) m/z : 565(M + H)⁺.
¹H-NMR (CDCl₃) δ: 1.66 (3H, s), 2.07-2.86 (4H, m), 2.88-3.68 (4H, m), 3.69-3.97 and 4.15-4.45 (1H, m), 3.84 (3H, s), 4.90-5.04 (1H, m), 5.36-5.40 (1H, m), 6.77-6.82 (1H, m), 6.89-6.94 (1H, m), 7.21-7.37 (2H, m), 7.56-7.51 (2H, m) .
Elemental analysis for: C₂₆H₂₄ClF₃N₄O₅·1.0H₂O·0.1 n-hexane Calculated: C, 54.01; H, 4.67; N, 9.47.
Found: C, 54.08; H, 4.36; N, 9.16.

### Example 565

Ethyl 2-(1-{2-[(trans)-8-chloro-1-[chloro(difluoro)methyl]-6-(3-methoxy-2-methylphenyl)-4H,6H-[1,2,4]triazolo[4,3-a] [4,1]benzoxazepin-4-yl]acetyl}-3-azetidinyl)acetate

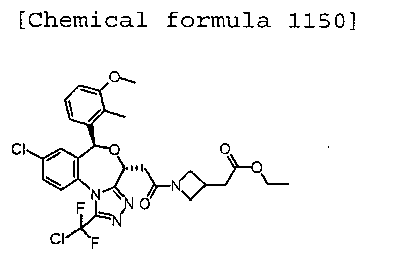

In a similar manner to that employed for the synthesis of ethyl 2-(1-(2-(8-chloro-6-(2,3-dimethoxyphenyl)-4H,6H-pyrrolo(1,2-a)(4,1)benzoxazepin-4-yl)acetyl)-4-piperidinyl)acetate, the title compound (189 mg) was obtained from 2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetic acid (150 mg) by using ethyl 2-(3-azetidinyl)acetate (129 mg), 1-ethyl-3-(3-dimethylaminopropyl)carbodiimide hydrochloride (119 mg), and 1-hydroxybenzotriazole (47 mg).
MS (ESI) m/z : 609 (M + H)⁺.
¹H-NMR (CDCl₃) δ: 1H-NMR (CDCl3) δ: 1.23-1.29 (3H, m), 1.66 (3H, s), 2.58-2.71 (2H, m), 2.91-3.05 (2H, m), 3.13-3.25 (1H, m), 3.64-3.76 (1H, m), 3.82 (3H, s), 3.88-3.94 (0.5H, m), 4.09-4.23 (3.5H, m), 4.37-4.45 (0.5H, m), 4.59-4.66 (0.5H, m), 4.95-5.01 (1H, m), 5.36 (1H, s), 6.75-6.79 (1H, m), 6.89-6.94 (1H, m), 7.25-7.36 (2H, m), 7.59-7.49 (2H, m).

### Example 566

2-(1-{2-[(trans)-8-Chloro-1-[chloro(difluoro)methyl]-6-(3-methoxy-2-methylphenyl)-4H,6H-[1,2,4]triazolo[4,3-a][4,1]benzoxazepin-4-yl]acetyl}-3-azetidinyl)acetic acid

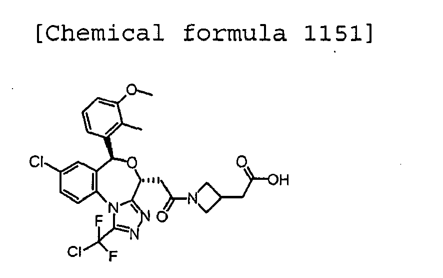

The title compound (20 mg) was obtained from ethyl 2-(1-{2-[(trans)-8-chloro-1-[chloro(difluoro)methyl]-6-(3-methoxy-2-methylphenyl)-4H,6H-[1,2,4]triazolo[4,3-a][4,1]benzoxazepin-4-yl]acetyl}-3-azetidinyl)acetate (185 mg) by using concentrated hydrochloric acid (5.0 mL).
MS (ESI) m/z : 581 (M + H)⁺.
¹H-NMR (CDCl₃) δ: 1.67 (3H, s), 2.19-2.37 (1H, m), 2.55-2.64 (1H, m), 2.75-2.86 (1H, m), 3.06-3.14 (1H, m), 3.28-3.55 (4H, m), 3.84 (3H, s), 3.99-4.17 and 4.30-4.39 (1H, m), 4.87-4.94 (1H, m), 5.37 (1H, s), 6.41-6.56 (1H, m), 6.78-6.80 (1H, m), 6.92 (1H, d, J = 7.8 Hz), 7.21-7.36 (2H, m), 7.64-7.51 (2H, m).
Elemental analysis for: C₂₆H₂₄Cl₂F₂N₄O₅·1.5H₂O·0.1 n-hexane Calculated: C, 51.78; H, 4.64; N, 9.08.
Found: C, 51.94; H, 4.29; N, 8.79.

### Example 567

Ethyl 2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetate

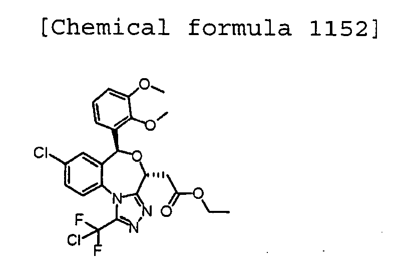

Ethyl 2-[(3R,5S)-7-chloro-5-(2,3-dimethoxyphenyl)-2-thioxo-1,2,3,5-tetrahydro-4,1-benzoxazepin-3-yl]acetate (218 mg) was dissolved in tetrahydrofuran (5 mL). To the resulting solution was added hydrazine monohydrate (0.036 mL). The resulting mixture was stirred at room temperature for 2 hours. To the reaction mixture were added chlorodifluoroacetic anhydride (0.353 mL) and chlorodifluoroacetic acid (0.193 mL) and the resulting mixture was stirred at room temperature for 2 hours. The reaction mixture was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography (ethyl acetate:n-hexane=1:2) to give the title compound (296 mg).
MS (ESI) m/z : 528 (M + H)⁺.
¹H-NMR (CDCl₃) δ: 1.28 (3H, t, J = 7.2 Hz), 3.24 (1H, dd, J = 16.8, 6.8 Hz), 3.44 (3H, s), 3.49 (1H, dd, J = 16.8, 6.8 Hz), 3.86 (3H, s), 4.19 (2H, q, J = 7.2 Hz), 4.90 (1H, t, J = 6.8 Hz), 5.53 (1H, s), 6.83 (1H, d, J = 2.2 Hz), 6.98 (1H, dd, J = 6.8, 2.7 Hz), 7.16-7.22 (2H, m), 7.51 (1H, dd, J = 8.5, 2.2 Hz), 7.57 (1H, d, J = 8.8 Hz).

### Example 568

2-[(4R,6S)-8-Chloro-1-[chloro(difluoro)methyl]-6-(2,3-dimethoxyphenyl)-4H,6H-[1,2,4]triazolo[4,3-a] [4, 1] benzoxazepin-4-yl] acetic acid

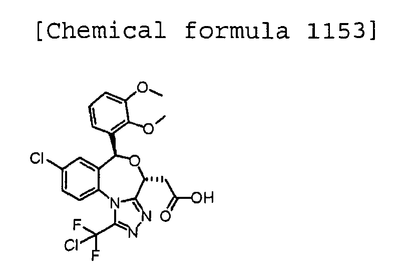

Ethyl 2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetate (264 mg) was dissolved in 1,4-dioxane (5 mL). To the resulting solution was added concentrated hydrochloric acid (1 mL). The resulting mixture was stirred at 60°C for 13 hours. The reaction mixture was concentrated under reduced pressure and the residue thus obtained was purified by preparative thin layer chromatography on silica gel (a chloroform:methanol:water=7:3:1 organic layer), followed by solidification from an ether-n-hexane solvent mixture to give the title compound (192 mg).
MS (ESI) m/z : 500 (M + H)⁺.
¹H-NMR (CDCl₃) δ: 3.18 (1H, dd, J = 16.8, 6.4 Hz), 3.39-3.48 (4H, m), 3.85 (3H, s), 4.85 (1H, t, J = 6.4 Hz), 5.50 (1H, s), 6.80 (1H, d, J = 2.0 Hz), 6.96 (1H, dd, J = 8.1, 1.5 Hz), 7.14-7.23 (2H, m), 7.46 (1H, d, J = 8.3 Hz), 7.55 (1H, d, J = 8.3 Hz).
Elemental analysis for: C₂₁H₁₇Cl₂F₂N₃O5·0.5H₂O
Calculated: C, 49.53; H, 3.56; N, 8.25.
Found: C, 49.57; H, 3.52; N, 7.99.

### Referential Example 392

N-[2-(2,3-Dimethoxybenzoyl)-4-(trifluoromethyl)phenyl]-2,2-dimethylpropanamide

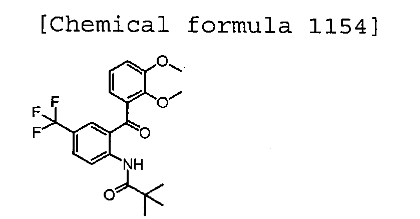

N-[2-[(2,3-Dimethoxyphenyl)(hydroxy)methyl]-4-(trifluoromethyl)phenyl]-2,2-dimethylpropanamide (7.11 g) was dissolved in a solvent mixture of methylene chloride (100 mL) and tetrahydrofuran (50 mL). To the resulting solution was added manganese dioxide (17.07 g). The resulting mixture was heated under reflux for 2 days. The insoluble material was filtered out and the filtrate was concentrated under reduced pressure to give the title compound (7.22 g).
MS (ESI) m/z : 410 (M + H)⁺.
¹H-NMR (CDCl₃) δ: 1.39 (9H, s), 3.74 (3H, s), 3.93 (3H, s), 6.87 (1H, dd, J = 7.8, 1.2 Hz), 7.10 (1H, dd, J = 7.8, 1.7 Hz), 7.16 (1H, t, J = 7.8 Hz), 7.71 (1H, s), 7.77 (1H, d, J = 8.8 Hz), 8.98 (1H, d, J = 8.8 Hz), 11.92 (1H, br s).

### Referential Example 393

[2-Amino-5-(trifluoromethyl)phenyl](2,3-dimethoxyphenyl)methanone

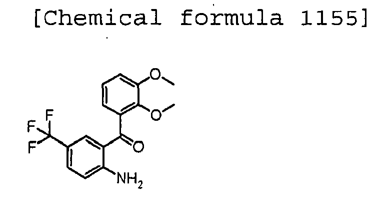

N-[2-(2,3-Dimethoxybenzoyl)-4-(trifluoromethyl)phenyl]-2,2-dimethylpropanamide (4.90 g) was dissolved in ethanol (83 mL). To the resulting solution was added sodium hydroxide (4.14 g). The resulting mixture was heated under reflux for one hour.
The reaction mixture was concentrated and saturated brine was then added to the residue. The resulting mixture was extracted with ethyl acetate. The organic layer was washed with saturated brine and dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.61) to give the title compound (3.64 g).
MS (ESI) m/z : 326 (M + H)⁺.
¹H-NMR (CDCl₃) δ: 3.77 (3H, s), 3.92 (3H, s), 6.75 (1H, d, J = 8.5 Hz), 6.83 (1H, dd, J = 7.6, 1.5 Hz), 7.04 (1H, dd, J = 8.1, 1.5 Hz), 7.13 (1H, t, J = 7.9 Hz), 7.45 (1H, dd, J = 8.8, 2.2 Hz), 7.54-7.55 (1H, m).

### Referential Example 394

[2-Amino-5-(trifluoromethyl)phenyl](2,3-dimethoxyphenyl)methanol

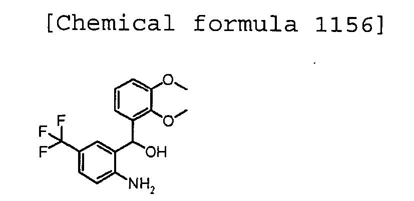

[2-Amino-5-(trifluoromethyl)phenyl](2,3-dimethoxyphenyl)methanone (7.07 g) was dissolved in ethanol (100 mL). To the resulting solution was added sodium borohydride (0.81 g) in portions under ice cooling. The reaction mixture was stirred at room temperature for 3 days. The reaction mixture was concentrated. The residue was diluted with ethyl acetate and washed with saturated brine. The organic layer was dried over anhydrous sodium sulfate. The filtrate was then concentrated to give the title compound (3.70 g).
MS m/z : 328(M+H⁺).
¹H-NMR (CDCl₃) δ: 3.83 (3H, s), 3.88 (3H, s), 4.56 (2H, s), 6.05 (1H, d, J = 3.2 Hz), 6.65 (1H, d, J = 8.3 Hz), 6.84 (1H, dd, J = 7.9, 1.5 Hz), 6.91 (1H, dd, J = 8.3, 1.5 Hz), 7.06 (1H, t, J = 7.9 Hz), 7.32 (1H, dd, J = 8.3, 1.8 Hz), 7.38 (1H, d, J = 1.8 Hz).

### Referential Example 395

[2-[(2,4-Dimethoxybenzyl)amino]-5-(trifluoromethyl)phenyl](2,3-dimethoxyphenyl) methanol

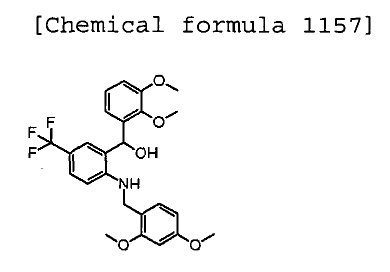

[2-Amino-5-(trifluoromethyl)phenyl](2,3-dimethoxyphenyl)methanol (3.70 g) and 2,4-dimethoxybenzaldehyde (2.25 g) were dissolved in acetic acid (30 mL). The resulting solution was stirred at room temperature for 20 minutes. Under ice cooling, sodium borohydride (1.28 g) was added and the resulting mixture was stirred at room temperature for 30 minutes. The reaction mixture was concentrated. The residue was diluted with ethyl acetate and then, washed with a saturated aqueous solution of sodium bicarbonate and saturated brine. The organic layer was dried over anhydrous sodium sulfate. The filtrate was then concentrated to give the title compound (6.12 g).
MS m/z : 478(M+H⁺).
¹H-NMR (CDCl₃) δ: 3.77 (3H, s), 3.79 (6H, s), 3.88 (3H, s), 4.30 (2H, d, J = 4.1 Hz), 5.74 (1H, br s), 6.04 (1H, d, J = 4.9 Hz), 6.38 (1H, dd, J = 8.3, 2.2 Hz), 6.44-6.47 (2H, m), 6.66 (1H, d, J = 8.8 Hz), 6.79-6.82 (1H, m), 6.91 (1H, dd, J = 8.0, 1.5 Hz), 7.04 (2H, t, J = 8.2 Hz), 7.36 (1H, dd, J = 8.5, 2.0 Hz).

### Referential Example 396

Ethyl 2-[3,5-trans-1-(2,4-dimethoxybenzyl)-5-(2,3-dimethoxyphenyl)-2-oxo-7-(trifluoromethyl)-1,2,3,5-tetrahydro-4,1-benzoxazepin-3-yl]acetate

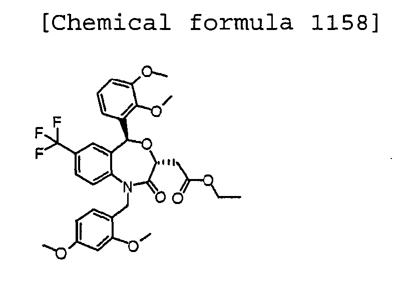

[2-[(2,4-Dimethoxybenzyl)amino]-5-(trifluoromethyl)phenyl](2,3-dimethoxyphenyl) methanol (14.36 g) was dissolved in dichloromethane (150 mL).
Sodium hydrogen carbonate (658 mg) and monoethyl chlorofumarate (5.87 g) prepared separately were added to the resulting solution at 0°C. After stirring at room temperature for 4.2 hours, the reaction mixture was diluted with dichloromethane and washed with a saturated aqueous solution of sodium bicarbonate and saturated brine. The organic layer was dried over anhydrous sodium sulfate. The filtrate was then concentrated.
The residue thus obtained was dissolved in ethanol (150 mL). To the resulting solution was added 1,8-diazabicycloundec-7-ene (6.75 mL). The resulting mixture was heated under reflux for 2 hours and stirred at room temperature for 3 days. The reaction mixture was concentrated. The residue was diluted with ethyl acetate and washed successively with a 10% aqueous citric acid solution and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.60) to give the title compound (11.10 g).
MS (ESI) m/z : 604 (M + H)⁺.
¹H-NMR (CDCl₃) δ: 1.25 (3H, t, J = 7.0 Hz), 2.80 (1H, dd, J = 16.5, 5.8 Hz), 3.09-3.17 (4H, m), 3.64 (3H, s), 3.76 (3H, s), 3.85 (3H, s), 4.14 (2H, q, J = 7.0 Hz), 4.47 (1H, dd, J = 8.0, 5.8 Hz), 4.92 (1H, d, J = 15.0 Hz), 5.52 (1H, d, J = 15.0 Hz), 6.02 (1H, s), 6.39-6.43 (2H, m), 6.84 (1H, d, J = 2.0 Hz), 6.94 (1H, dd, J = 6.7, 3.1 Hz), 7.13-7.18 (2H, m), 7.25-7.28 (1H, m), 7.49 (1H, d, J = 8.3 Hz), 7.58 (1H, dd, J = 8.5, 1.8 Hz).

### Referential Example 397

Ethyl 2-[3,5-trans-5-(2,3-dimethoxyphenyl)-2-oxo-7-(trifluoromethyl)-1,2,3,5-tetrahydro-4,1-benzoxazepin-3-yl]acetate

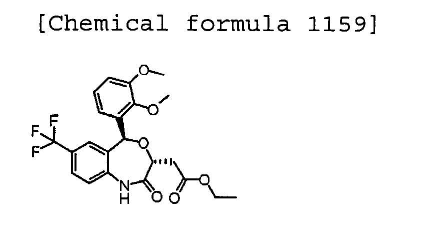

Ethyl 2-[3,5-trans-1-(2,4-dimethoxybenzyl)-5-(2,3-dimethoxyphenyl)-2-oxo-7-(trifluoromethyl)-1,2,3,5-tetrahydro-4,1-benzoxazepin-3-yl]acetate (4.76 g) was dissolved in acetone (120 mL). At 0°C, an aqueous solution (30 mL) of ceric(IV) ammonium nitrate (12.97 g) was added and the resulting mixture was stirred at room temperature for 2 hours. At 0°C, an aqueous solution (10 mL) of ceric(IV) ammonium nitrate (4.32 g) was added and the resulting mixture was stirred at room temperature for one hour and, after addition of an aqueous solution (10 mL) of ceric(IV) ammonium nitrate (4.32 g) at 0°C, was stirred at room temperature for one hour. To the reaction mixture was added a saturated aqueous solution of sodium bicarbonate. The resulting mixture was extracted with ethyl acetate. The extract was washed with saturated brine. The organic layer was dried over anhydrous sodium sulfate. After concentration of the filtrate, the residue thus obtained was purified by silica gel chromatography (automatic purification system of Yamazen Corporation, ethyl acetate:n-hexane=1:2, Rf=0.35) to give the title compound (2.46 g).
MS (ESI) m/z : 454 (M + H)⁺.
¹H-NMR (CDCl₃) δ: 2.81 (1H, dd, J = 16.4, 6.6 Hz), 3.04 (1H, dd, J = 16.4, 6.6 Hz), 3.65 (3H, s), 3.89 (3H, s), 4.09 (2H, qd, J = 7.1, 1.9 Hz), 4.67 (1H, t, J = 6.3 Hz), 6.30 (1H, s), 6.97-7.03 (3H, m), 7.10-7.16 (2H, m), 7.55 (1H, dd, J = 8.3, 2.0 Hz), 8.17 (1H, s).

### Referential Example 398

Ethyl 2-[3,5-trans-5-(2,3-dimethoxyphenyl)-2-thioxo-7-(trifluoromethyl)-1,2,3,5-tetrahydro-4,1-benzoxazepin-3-yl] acetate

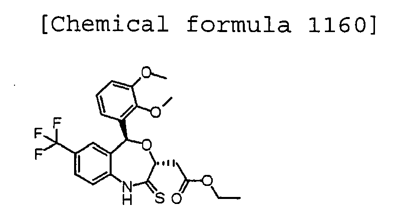

Ethyl 2-[3,5-trans-5-(2,3-dimethoxyphenyl)-2-oxo-7-(trifluoromethyl)-1,2,3,5-tetrahydro-4,1-benzoxazepin-3-yl]acetate (2.46 g) was dissolved in tetrahydrofuran (50 mL). To the resulting solution were added sodium hydrogen carbonate (0.96 g) and diphosphorus pentasulfide (2.53 g). The resulting mixture was stirred at room temperature for 4 hours. The reaction mixture was diluted with ethyl acetate and washed successively with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.55) to give the title compound (1.93 g).
MS (ESI) m/z : 470 (M + H)⁺.
¹H-NMR (CDCl₃) δ: 1.23 (3H, t, J = 7.2 Hz), 2.96 (1H, dd, J = 16.5, 6.5 Hz), 3.28 (1H, dd, J = 16.5, 6.5 Hz), 3.60 (3H, s), 3.88 (3H, s), 4.12 (2H, q, J = 7.2 Hz), 4.73 (1H, t, J = 6.5 Hz), 6.21 (1H, s), 6.96-7.01 (2H, m), 7.10 (1H, dd, J = 7.9, 1.7 Hz), 7.17 (1H, t, J = 7.9 Hz), 7.21 (1H, d, J = 8.3 Hz), 7.62 (1H, dd, J = 8.3, 2.0 Hz), 9.63 (1H, br s).

### Example 569

Ethyl 2-[4,6-trans-6-(2,3-dimethoxyphenyl)-1,8-bis(trifluoromethyl)-4H,6H-[1,2,4]triazolo[4,3-a] [4,1]benzoxazepin-4-yl]acetate

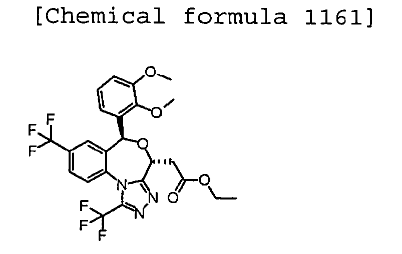

Ethyl 2-[3,5-trans-5-(2,3-dimethoxyphenyl)-2-thioxo-7-(trifluoromethyl)-1,2,3,5-tetrahydro-4,1-benzoxazepin-3-yl]acetate (3.60 g) was dissolved in tetrahydrofuran (40 mL). To the resulting solution was added hydrazine monohydrate (0.575 mL). The resulting mixture was stirred at room temperature for 1 hour. Under ice cooling, trifluoroacetic anhydride (5.582 mL) was added to the reaction mixture, followed by stirring at room temperature for 2.3 hours. The reaction mixture was diluted with ethyl acetate and then washed with a saturated aqueous solution of sodium bicarbonate and saturated brine. The organic layer was dried over sodium sulfate and concentrated under reduced pressure. The residue thus obtained was purified by silica gel chromatography (Automatic purification system of Yamazen Corporation, ethyl acetate:n-hexane=1:2, Rf=0.55) to give the title compound (3.50 g).
MS (ESI) m/z : 546 (M + H)⁺.
¹H-NMR (CDCl₃) δ: 1.28 (3H, t, J = 7.2 Hz), 3.24 (1H, dd, J = 16.6, 6.8 Hz), 3.39 (3H, s), 3.52 (1H, dd, J = 16.7, 6.7 Hz), 3.85 (3H, s), 4.19 (2H, qd, J = 7.2, 1.3 Hz), 4.93 (1H, t, J = 6.8 Hz), 5.62 (1H, s), 6.99 (1H, dd, J = 7.6, 2.2 Hz), 7.14 (1H, d, J = 1.5 Hz), 7.17-7.24 (2H, m), 7.72 (1H, d, J = 8.5 Hz), 7.83 (1H, dd, J = 8.4, 1.8 Hz).

### Example 570

Ethyl 2-[(4S,6R)-6-(2,3-dimethoxyphenyl)-1,8-bis(trifluoromethyl)-4H,6H-[1,2,4]triazolo[4,3-a] [4,1]benzoxazepin-4-yl]acetate (isomer A) Ethyl 2-[(4R,6S)-6-(2,3-dimethoxyphenyl)-1,8-bis(trifluoromethyl)-4H,6H-[1,2,4]triazolo[4,3-a][4,1]benzoxazepin-4-yl]acetate (isomer B)

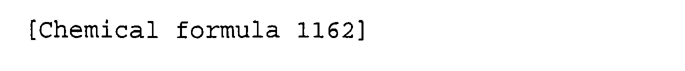

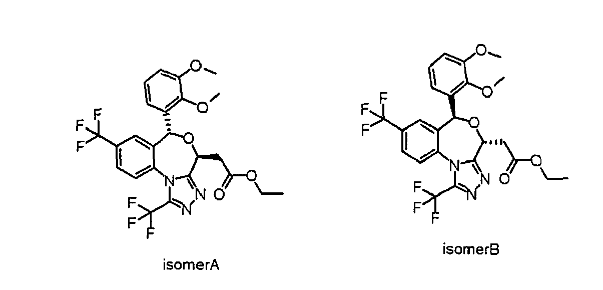

Ethyl 2-[4,6-trans-6-(2,3-dimethoxyphenyl)-1,8-bis(trifluoromethyl)-4H,6H-[1,2,4]triazolo[4,3-a][4,1]benzoxazepin-4-yl] was optically resolved by HPLC. Column: Chiralpak AD (5 × 50cm)
Eluting solution: 20% isopropanol-n-hexane
Flow rate: 50 mL/min
Elution time: isomer A: 34 min., isomer B: 53 min.

### Example 571

2-[(4S,6R)-6-(2,3-Dimethoxyphenyl)-1,8-bis(trifluoromethyl)-4H,6H-[1,2,4]triazolo[4,3-a] [4,1]benzoxazepin-4-yl]acetic acid

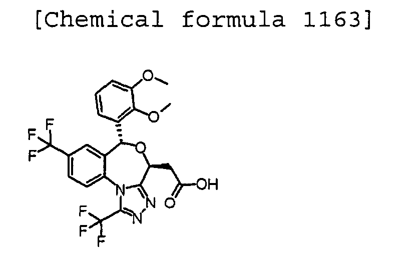

Ethyl 2-[(4S,6R)-6-(2,3-dimethoxyphenyl)-1,8-bis(trifluoromethyl)-4H,6H-[1,2,4]triazolo[4,3-a] [4,1]benzoxazepin-4-yl]acetate (1419 mg) was dissolved in 1,4-dioxane (30 mL). To the resulting solution were added concentrated hydrochloric acid (15 mL) and distilled water (15 mL). The resulting mixture was stirred at 60°C for 14 hours. The reaction mixture was dissolved in 1,4-dioxane (20 mL) again and concentrated hydrochloric acid (5 mL) was added to the resulting solution. The resulting mixture was stirred at 65°C for 19 hours. The reaction mixture was concentrated under reduced pressure. The residue thus obtained was solidified from a methylene chloride-n-hexane solvent mixture to give the title compound (1295 mg).
MS (ESI) m/z : 518 (M + H)⁺.
¹H-NMR (CDCl₃) δ: 3.30 (1H, dd, J = 17.4, 7.0 Hz), 3.40 (3H, s), 3.60 (1H, dd, J = 17.4, 7.0 Hz), 3.86 (3H, s), 4.90 (1H, t, J = 7.0 Hz), 5.62 (1H, s), 6.97-7.01 (1H, m), 7.13-7.23 (3H, m), 7.73 (1H, d, J = 8.1 Hz), 7.83 (1H, d, J = 8.3 Hz).
Elemental analysis for: C₂₂H₁₇F₆N₃O₅·0.25H₂O
Calculated: C, 50.63; H, 3.38; N, 8.05.
Found: C, 49.57; H, 3.39; N, 7.77.

### Example 572

2-[(4R,6S)-6-(2,3-Dimethoxyphenyl)-1,8-bis(trifluoromethyl)-4H,6H-[1,2,4]triazolo[4,3-a][4,1]benzoxazepin-4-yl]acetic acid

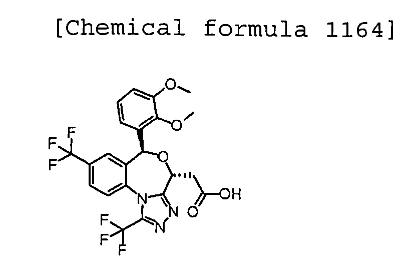

Ethyl 2-[(4R,6S)-6-(2,3-dimethoxyphenyl)-1,8-bis(trifluoromethyl)-4H,6H-[1,2,4]triazolo[4,3-a][4,1]benzoxazepin-4-yl]acetate(1543 mg) was dissolved in 1,4-dioxane (30 mL). To the resulting solution were added concentrated hydrochloric acid (15 mL) and distilled water (15 mL). The resulting mixture was stirred at 60°C for 14 hours. The reaction mixture was dissolved in 1,4-dioxane (20 mL) again. To the resulting solution was added concentrated hydrochloric acid (5 mL). The resulting mixture was stirred at 65°C for 19 hours. The reaction mixture was concentrated under reduced pressure. The residue thus obtained was solidified from a methylene chloride-n-hexane solvent mixture to give the title compound (1275 mg).
MS (ESI) m/z : 518 (M + H)⁺.
¹H-NMR (CDCl₃) δ: 3.31 (1H, dd, J = 17.2, 6.7 Hz), 3.40 (3H, s), 3.59 (1H, dd, J = 17.2, 6.7 Hz), 3.86 (3H, s), 4.90 (1H, t, J = 6.7 Hz), 5.62 (1H, s), 7.00 (1H, dd, J = 7.6, 2.0 Hz), 7.15-7.24 (3H, m), 7.73 (1H, d, J = 8.5 Hz), 7.82-7.86 (1H, m).
Elemental analysis for: C₂₂H₁₇F₆N₃O₅·0.25H₂O·0.1 n-Hexane Calculated: C, 51.17; H, 3.59; N, 7.92.
Found: C, 50.91; H, 3.31; N, 7.64.

### Example 573

tert-Butyl 2-(1-{2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}-4-piperidinyl)acetate

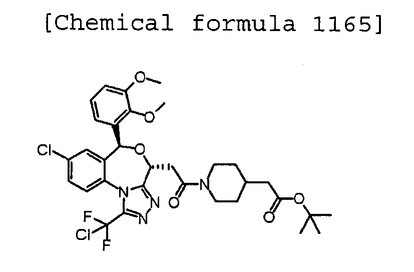

2-[(4R,6S)-8-Chloro-1-[chloro(difluoro)methyl]-6-(2,3-dimethoxyphenyl)-4H,6H-[1,2,4]triazolo[4,3-a] [4,1]benzoxazepin-4-yl]acetic acid (74 mg) was dissolved in methylene chloride (2 mL). To the resulting solution were added 1-ethyl-3-(3-dimethylaminopropyl)carbodiimide hydrochloride (57 mg), tert-butyl 2-(4-piperidinyl)acetate (38 mg), and 1-hydroxybenzotriazole monohydrate (23 mg). The resulting mixture was stirred at room temperature for 3.5 hours. The reaction mixture was purified by preparative thin layer silica gel column chromatography (ethyl acetate:n-hexane=2:1) to give the title compound (107 mg).
MS (ESI) m/z : 681 (M + H)⁺.
¹H-NMR (CDCl₃) δ: 1.10-1.29 (2H, m), 1.45 (9H, s), 1.69-1.84 (2H, m), 1.95-2.06 (1H, m), 2.12-2.20 (2H, m), 2.57-2.66 (1H, m), 3.04-3.25 (2H, m), 3.43 (3H, s), 3.49-3.61 (1H, m), 3.86 (3H, s), 3.99-4.07 (1H, m), 4.51-4.58 (1H, m), 5.02-5.07 (1H, m), 5.50-5.52 (1H, m), 6.81-6.83 (1H, m), 6.96-7.00 (1H, m), 7.17-7.26 (2H, m), 7.48-7.51 (1H, m), 7.54-7.58 (1H, m).

### Example 574

2-(1-{2-[(4R,6S)-8-Chloro-1-[chloro(difluoro)methyl]-6-(2,3-dimethoxyphenyl)-4H,6H-[1,2,4]triazolo[4,3-a][4,1]benzoxazepin-4-yl]acetyl}-4-piperidinyl)acetic acid

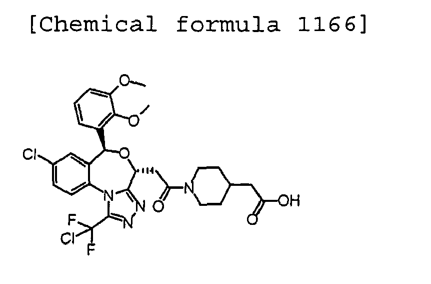

tert-Butyl 2-(1-{2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}-4-piperidinyl)acetate (101 mg) was dissolved in methylene chloride (5 mL). To the resulting solution was added trifluoroacetic acid (1 mL). The resulting mixture was stirred at room temperature for 1.5 hours. The reaction mixture was concentrated under reduced pressure. The residue was solidified from a methylene chloride-n-hexane solvent mixture to give the title compound(84 mg).
MS (ESI) m/z : 625 (M + H)⁺.
¹H-NMR (CDCl₃) δ: 1.13-1.33 (2H, m), 1.74-1.90 (2H, m), 2.00-2.10 (1H, m), 2.27 (1H, dd, J = 7.0, 1.8 Hz), 2.34 (1H, d, J = 6.8 Hz), 2.58-2.67 (1H, m), 3.05-3.28 (2H, m), 3.43 (3H, s), 3.47-3.63 (1H, m), 3.86 (3H, s), 4.01-4.10 (1H, m), 4.53-4.60 (1H, m), 5.02-5.07 (1H, m), 5.52 (1H, d, J = 6.8 Hz), 6.82 (1H, d, J = 2.4 Hz), 6.96-7.00 (1H, m), 7.19 (2H, t, J = 7.8 Hz), 7.22 (2H, dd, J = 5.9, 1.7 Hz), 7.50 (1H, dd, J = 8.7, 2.3 Hz), 7.57 (1H, d, J = 8.3 Hz).
Elemental analysis for: C₂₈H₂₈Cl₂F₂N₄O₆·1.0 H₂O·0.25 n-Hexane
Calculated: C, 53.28; H, 5.08; N, 8.42.
Found: C, 53.27; H, 4.74; N, 8.10.

### Referential Example 399

tert-Butyl 2-[(2,3-dimethoxyphenyl)(hydroxy)methyl]-4-(trifluoromethoxy) phenylcarbamate

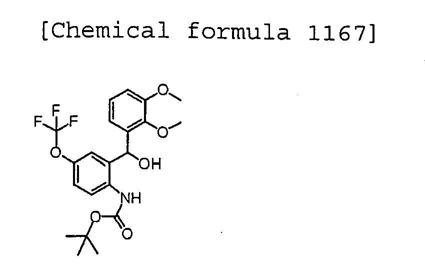

To a tetrahydrofuran solution (5 mL) of tert-Butyl 4-(trifluoromethoxy)phenylcarbamate (2.77 g) was added dropwise a 1N sec-butyl lithium c-hexane and hexane solution (23.00 mL) at -78°C. The reaction mixture was stirred at -25°C for 2.5 hours. To the reaction mixture was added 2,3-dimethoxybenzaldehyde (1.83 g) at the same temperature and the resulting mixture was stirred for 3.5 hours. To the reaction mixture were added a saturated aqueous ammonium chloride solution, distilled water and ethyl acetate and the layers separated. The organic layer thus obtained was washed with saturated brine and dried over anhydrous sodium sulfate. The solvent was distilled off to give the title compound (4.80 g).
MS (ESI) m/z : 370 (M - tBu-OH)⁺.
¹H-NMR (CDCl₃) δ: 1.50 (9H, s), 3.81 (3H, s), 3.89 (3H, s), 6.06 (1H, d, J = 4.9 Hz), 6.89 (1H, dd, J = 7.6, 1.2 Hz), 6.92 (1H, dd, J = 8.3, 1.5 Hz), 6.96 (1H, d, J = 2.7 Hz), 7.06-7.16 (2H, m), 7.93 (1H, d, J = 8.8 Hz), 8.15 (1H, br
s).

### Referential Example 400

[2-Amino-5-(trifluoromethoxy)phenyl](2,3-dimethoxyphenyl)methanol

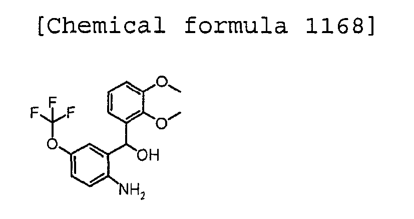

tert-Butyl 2-[(2,3-dimethoxyphenyl)(hydroxy)methyl]-4-(trifluoromethoxy) phenylcarbamate (4.43 g) was dissolved in 1,4-dioxane (50 mL). To the resulting solution was added concentrated hydrochloric acid (20 mL). The resulting mixture was stirred at room temperature for 6 hours. After concentration, the residue thus obtained was purified by silica gel chromatography (Automatic purification system of Yamazen Corporation, ethyl acetate:n-hexane=1:2, Rf=0.30) to give the title compound (2.07 g).
MS (ESI) m/z : 344 (M + H)⁺.
¹H-NMR (CDCl₃) δ: 3.87 (3H, s), 3.89 (3H, s), 6.27 (1H, s), 6.52 (1H, d, J = 2.7 Hz), 6.60 (1H, d, J = 8.5 Hz), 6.83 (1H, dd, J = 7.8, 1.5 Hz), 6.88 (2H, dd, J = 8.1, 1.5 Hz), 7.02 (1H, t, J = 8.1 Hz) .

### Referential Example 401

[2-[(2,4-Dimethoxybenzyl)amino]-5-(trifluoromethoxy)phenyl](2,3-dimethoxyphenyl)methanol

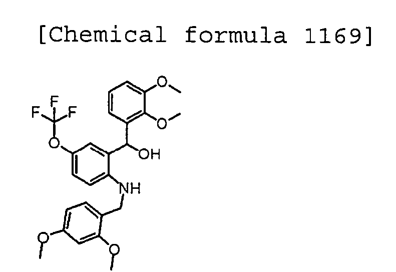

[2-Amino-5-(trifluoromethoxy)phenyl](2,3-dimethoxyphenyl)methanol (2.07 g) and 2,4-dimethoxybenzaldehyde (1.20 g) were dissolved in acetic acid (20 mL). The resulting solution was stirred at room temperature for 20 minutes. Under ice cooling, sodium borohydride (0.68 g) was added and the resulting mixture was stirred at room temperature for 40 minutes. After concentration of the reaction mixture, the residue was diluted with ethyl acetate and washed with a saturated aqueous solution of sodium bicarbonate and saturated brine. The organic layer was dried over anhydrous sodium sulfate. The filtrate was then concentrated to give the title compound (3.03 g).
MS (ESI) m/z : 494 (M + H)⁺.

### Referential Example 402

Ethyl 2-[3,5-trans-1-(2,4-dimethoxybenzyl)-5-(2,3-dimethoxyphenyl)-2-oxo-7-(trifluoromethoxy)-1,2,3,5-tetrahydro-4,1-benzoxazepin-3-yl]acetate

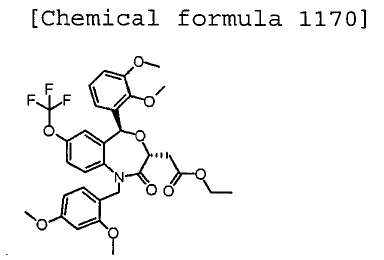

[2-[(2,4-Dimethoxybenzyl)amino]-5-(trifluoromethoxy)phenyl](2,3-dimethoxyphenyl)methanol (2.97 g) was dissolved in dichloromethane (30 mL). Sodium hydrogen carbonate (658 mg) and monoethyl chlorofumarate (1.17 g) prepared separately were added to the resulting solution at 0°C. The reaction mixture was stirred at room temperature for 12 hours, followed by concentration. The residue was diluted with ethyl acetate and then washed with a saturated aqueous solution of sodium bicarbonate and saturated brine. The organic layer was dried over anhydrous sodium sulfate. The filtrate was concentrated.
The residue thus obtained was dissolved in ethanol (20 mL). To the resulting solution was added 1,8-diazabicycloundec-7-ene (1.35 mL). The resulting mixture was heated under reflux for 2.5 hours. The reaction mixture was concentrated. The residue was diluted with ethyl acetate and washed with saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:4, Rf=0.10) to give the title compound (2.23 g).
MS (ESI) m/z : 620 (M + H)⁺.
¹H-NMR (CDCl₃) δ: 1.25 (3H, t, J = 7.2 Hz), 2.80 (1H, dd, J = 16.5, 5.7 Hz), 3.11 (1H, dd, J = 16.5, 7.9 Hz), 3.17 (3H, s), 3.64 (3H, s), 3.76 (3H, s), 3.85 (3H, s), 4.14 (2H, q, J = 7.2 Hz), 4.49 (1H, dd, J = 7.9, 5.7 Hz), 4.88 (1H, d, J = 15.0 Hz), 5.48 (1H, d, J = 15.0 Hz), 5.98 (1H, s), 6.39-6.44 (3H, m), 6.94 (1H, t, J = 4.9 Hz), 7.13-7.21 (3H, m), 7.26-7.28 (1H, m), 7.40 (1H, d, J = 8.8 Hz).

### Referential Example 403

Ethyl 2-[3,5-trans-5-(2,3-dimethoxyphenyl)-2-oxo-7-(trifluoromethoxy)-1,2,3,5-tetrahydro-4,1-benzoxazepin-3-yl]acetate

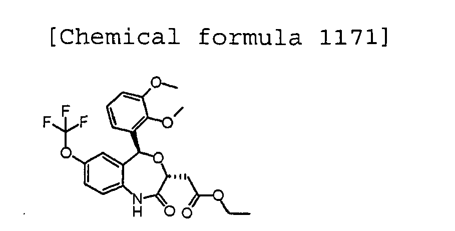

Ethyl 2-[3,5-trans-1-(2,4-dimethoxybenzyl)-5-(2,3-dimethoxyphenyl)-2-oxo-7-(trifluoromethoxy)-1,2,3,5-tetrahydro-4,1-benzoxazepin-3-yl]acetate (2.23 g) was dissolved in acetone (60 mL). At 0°C, an aqueous solution (15 mL) of ceric(IV) ammonium nitrate (5.92 g) was added and the resulting mixture was stirred at room temperature for 16 hours. At 0°C, an aqueous solution (10 mL) of ceric(IV) ammonium nitrate (3.95 g) was added and the resulting mixture was stirred at room temperature for 30 minutes. Ethyl acetate was added and the resulting mixture was washed with saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.27) to give the title compound (1.03 g).
MS (ESI) m/z : 470 (M + H)⁺.
¹H-NMR (CDCl₃) δ: 1.21 (3H, t, J = 7.1 Hz), 2.80 (1H, dd, J = 16.5, 6.6 Hz), 3.05 (1H, dd, J = 16.5, 6.6 Hz), 3.62 (3H, s), 3.89 (3H, s), 4.10 (2H, q, J = 7.1 Hz), 4.67 (1H, t, J = 6.6 Hz), 6.24 (1H, s), 6.56 (1H, d, J = 2.4 Hz), 6.98 (1H, dd, J = 8.0, 1.7 Hz), 7.05-7.10 (2H, m), 7.13-7.20 (2H, m), 8.09-8.16 (1H, m).

### Referential Example 404

Ethyl 2-[3,5-trans-5-(2,3-dimethoxyphenyl)-2-thioxo-7-(trifluoromethoxy)-1,2,3,5-tetrahydro-4,1-benzoxazepin-3-yl]acetate

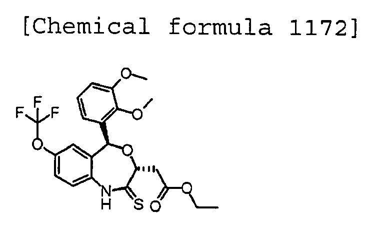

Ethyl 2-[3,5-trans-5-(2,3-dimethoxyphenyl)-2-oxo-7-(trifluoromethoxy)-1,2,3,5-tetrahydro-4,1-benzoxazepin-3-yl]acetate (1.03 g) was dissolved in tetrahydrofuran (20 mL). To the resulting solution were added sodium hydrogen carbonate (0.39 g) and diphosphorus pentasulfide (1.02 g). The resulting mixture was stirred at room temperature for 4 hours. The reaction mixture was diluted with ethyl acetate and washed successively with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.55) to give the title compound (0.74 g).
MS (ESI) m/z : 486 (M + H)⁺.
¹H-NMR (CDCl₃) δ: 1.24 (3H, t, J = 7.1 Hz), 2.96 (1H, dd, J = 16.5, 6.6 Hz), 3.28 (1H, dd, J = 16.5, 6.6 Hz), 3.60 (3H, s), 3.88 (3H, s), 4.13 (2H, qd, J = 7.1, 1.7 Hz), 4.72 (1H, t, J = 6.6 Hz), 6.16 (1H, s), 6.54 (1H, d, J = 2.7 Hz), 6.98 (1H, dd, J = 7.9, 1.8 Hz), 7.10-7.19 (3H, m), 7.21-7.25 (1H, m), 9.60 (1H, s).

### Example 575

Ethyl 2-[4,6-trans-6-(2,3-dimethoxyphenyl)-8-(trifluoromethoxy)-1-(trifluoromethyl)-4H,6H-[1,2,4]triazolo[4,3-a] [4,1]benzoxazepin-4-yl]acetate

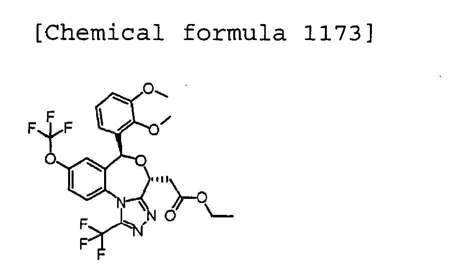

Ethyl 2-[3,5-trans-5-(2,3-dimethoxyphenyl)-2-thioxo-7-(trifluoromethoxy)-1,2,3,5-tetrahydro-4,1-benzoxazepin-3-yl]acetate (793 mg) was dissolved in tetrahydrofuran (20 mL). To the resulting solution was added hydrazine monohydrate (0.119 mL). The resulting mixture was stirred at room temperature for 30 minutes. Under ice cooling, trifluoroacetic anhydride (1.15 mL) was added and the resulting mixture was stirred at room temperature for one hour. The reaction mixture was diluted with ethyl acetate and then washed with a saturated aqueous solution of sodium bicarbonate and saturated brine. The organic layer was dried over sodium sulfate and concentrated under reduced pressure. The residue thus obtained was purified by silica gel chromatography (Automatic purification system of Yamazen Corporation, ethyl acetate:n-hexane=1:2, Rf=0.55) to give the title compound (736 mg).
MS (ESI) m/z : 562 (M + H)⁺.
¹H-NMR (CDCl₃) δ: 1.28 (3H, t, J = 7.1 Hz), 3.25 (1H, dd, J = 16.8, 6.8 Hz), 3.41 (3H, s), 3.52 (1H, dd, J = 16.8, 6.8 Hz), 3.85 (3H, s), 4. 19 (2H, q, J = 7.1 Hz), 4.95 (1H, t, J = 6.8 Hz), 5.59 (1H, s), 6.72 (1H, d, J = 2.4 Hz), 6.99 (1H, dd, J = 7.1, 2.4 Hz), 7.17-7.23 (2H, m), 7.39-7.44 (1H, m), 7.65 (1H, d, J = 8.8 Hz).

### Example 576

Ethyl 2-[(4S,6R)-6-(2,3-dimethoxyphenyl)-8-(trifluoromethoxy)-1-(trifluoromethyl)-4H,6H-[1,2,4]triazolo[4,3-a][4,1]benzoxazepin-4-yl]acetate
(isomer A) Ethyl 2-[(4R,6S)-6-(2,3-dimethoxyphenyl)-8-(trifluoromethoxy)-1-(trifluoromethyl)-4H,6H-[1,2,4]triazolo[4,3-a][4,1]benzoxazepin-4-yl]acetate
(isomer B)

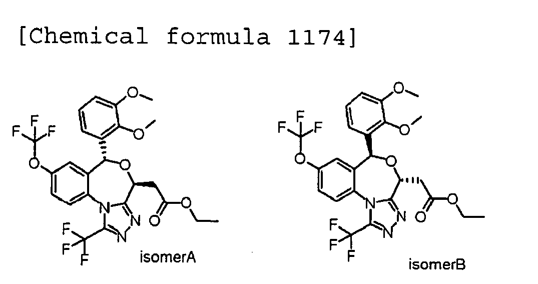

Ethyl 2-[4,6-trans-6-(2,3-dimethoxyphenyl)-8-(trifluoromethoxy)-1-(trifluoromethyl)-4H,6H-[1,2,4]triazolo[4,3-a][4,1]benzoxazepin-4-yl]acetate was optically resolved by HPLC.
Column: Chiralpak AD (5 × 50cm)
Eluting solution: 20% isopropanol-n-hexane
Flow rate: 50 mL/min
Elution time: isomer A: 34 min., isomer B: 53 min.

### Example 577

2-[(4R,6S)-6-(2,3-Dimethoxyphenyl)-8-(trifluoromethoxy)-1-(trifluoromethyl)-4H,6H-[1,2,4]triazolo[4,3-a] [4,1]benzoxazepin-4-yl]acetic acid

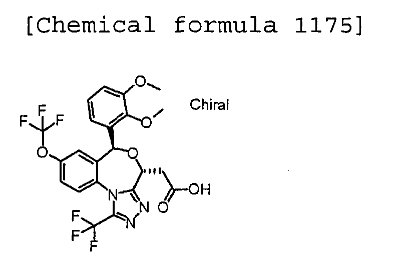

Ethyl 2-[(4R,6S)-6-(2,3-dimethoxyphenyl)-8-(trifluoromethoxy)-1-(trifluoromethyl)-4H,6H-[1,2,4]triazolo[4,3-a][4,1]benzoxazepin-4-yl]acetate (357 mg) was dissolved in 1,4-dioxane (10 mL). To the resulting solution was added concentrated hydrochloric acid (2 mL). The resulting mixture was stirred at 60°C for 15.6 hours and at 70°C for 4 hours. The reaction mixture was concentrated under reduced pressure. The residue thus obtained was purified by preparative thin layer chromatography on silica gel (a chloroform:methanol:water=7:3:1 organic layer) to give the title compound (277 mg).
MS (ESI) m/z : 534 (M + H)⁺.
¹H-NMR (CDCl₃) δ: 3.18-3.27 (1H, m), 3.40 (3H, s), 3.42-3.52 (1H, m), 3.85 (3H, s), 4.90 (1H, t, J = 6.5 Hz), 5.57 (1H, s), 6.70 (1H, s), 6.97 (1H, d, J = 7.8 Hz), 7.15-7.23 (2H, m), 7.36 (1H, d, J = 8.3 Hz), 7.62 (1H, d, J = 8.3 Hz).
Elemental analysis for: C₂₂H₁₇F₆N₃O₅·0.75 H₂O
Calculated: C, 48.32; H, 3.41; N, 7.68.
Found: C, 48.22; H, 3.10; N, 7.47.

### Example 578

tert-Butyl 2-(1-{2-[(4R,6S)-6-(2,3-dimethoxyphenyl)-1,8-bis(trifluoromethyl)-4H,6H-[1,2,4]triazolo[4,3-a][4,1]benzoxazepin-4-yl]acetyl}-4-piperidinyl)acetate

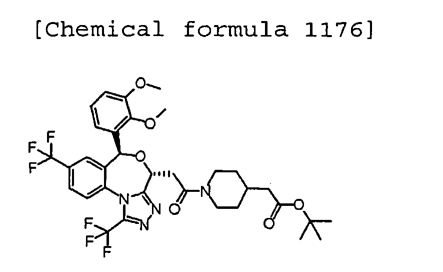

2-(1-{2-[6-(2,3-dimethoxyphenyl)-1,8-bis(trifluoromethyl)-4H,6H-[1,2,4]triazolo[4,3-a][4,1]benzoxazepin-4-yl]acetic acid (672 mg) was dissolved in methylene chloride (10 mL). To the resulting solution were added 1-ethyl-3-(3-dimethylaminopropyl)carbodiimide hydrochloride (498 mg), tert-butyl 2-(4-piperidinyl)acetate (337 mg), and 1-hydroxybenzotriazole monohydrate (199 mg). The resulting mixture was stirred at room temperature for 20 hours. The reaction mixture was purified by silica gel chromatography (Automatic purification system of Yamazen Corporation, ethyl acetate:n-hexane=1:2, Rf = 0.16), followed by optical resolution by ChiralPak IA (2 × 25 cm, 10 mL/min EtOH, retention time: 35 min) to give the title compound (23 mg).
MS (ESI) m/z 699 (M + H)⁺.
¹H-NMR (CDCl₃) δ: 1.09-1.28 (2H, m), 1.43-1.47 (9H, m), 1.69-1.85 (2H, m), 1.95-2.06 (1H, m), 2.11-2.15 (1H, m), 2.17-2.21 (1H, m), 2.57-2.66 (1H, m), 3.05-3.24 (2H, m), 3.37-3.39 (3H, m), 3.52-3.64 (1H, m), 3.86 (3H, s), 3.98-4.07 (1H, m), 4.49-4.57 (1H, m), 5.04-5.08 (1H, m), 5.58-5.61 (1H, m), 6.97-7.01 (1H, m), 7.12-7.14 (1H, m), 7.18-7.22 (1H, m), 7.23-7.26 (1H, m), 7.69-7.74 (1H, m), 7.79-7.83 (1H, m).

### Example 579

2-(1-{2-[(4R,6S)-6-(2,3-Dimethoxyphenyl)-1,8-bis(trifluoromethyl)-4H,6H-[1,2,4]triazolo[4,3-a][4,1]benzoxazepin-4-yl]acetyl}-4-piperidinyl)acetic acid

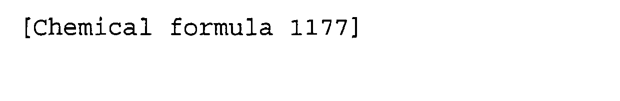

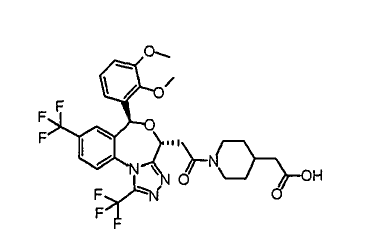

tert-Butyl 2-(1-{2-[(4R,6S)-6-(2,3-dimethoxyphenyl)-1,8-bis(trifluoromethyl)-4H,6H-[1,2,4]triazolo[4,3-a] [4,1]benzoxazepin-4-yl]acetyl}-4-piperidinyl)acetate (23 mg) was dissolved in methylene chloride (5 mL). To the resulting solution was added trifluoroacetic acid (1 mL). The resulting mixture was stirred at room temperature for 13 hours. The reaction mixture was concentrated under reduced pressure, followed by azeotropy with n-hexane. The residue thus obtained was lyophilized from 1,4-dioxane to give the title compound (20 mg).
MS (ESI) m/z : 643 (M + H) ⁺.
¹H-NMR (CDCl₃) δ: 1.16-1.25 (2H, m), 1.76-1.90 (2H, m), 2.02-2.10 (1H, m), 2.26-2.29 (1H, m), 2.33-2.36 (1H, m), 2.58-2.67 (1H, m), 3.06-3.27 (2H, m), 3.52-3.66 (1H, m), 4.00-4.10 (1H, m), 4.53-4.59 (1H, m), 5.04-5.09 (1H, m), 5.59-5.62 (1H, m), 6.98-7.01 (1H, m), 7.13-7.15 (1H, m), 7.18-7.25 (2H, m), 7.70-7.74 (1H, m), 7.80-7.83 (1H, m). Elemental analysis for: C₂₉H₂₈F₆N₄O₆·0.75 H₂O·0.25 n-Hexane Calculated: C, 54.06; H, 4.91; N, 8.27.
Found: C, 53.66; H, 4.50; N, 7.98.

### Example 580

tert-Butyl (2S)-2-({2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}amino)-4-methylpentanoate (isomer A) tert-Butyl (2S)-2-({2-[(4S,6R)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}amino)-4-methylpentanoate (isomer B)

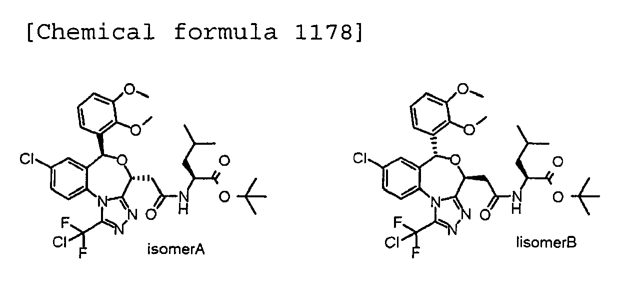

2-[4,6-trans-8-Chloro-1-[chloro(difluoro)methyl]-6-(2,3-dimethoxyphenyl)-4H,6H-[1,2,4]triazolo[4,3-a][4,1]benzoxazepin-4-yl]acetic acid (217 mg) was dissolved in methylene chloride (2 mL). To the resulting solution were added 1-ethyl-3-(3-dimethylaminopropyl)carbodiimide hydrochloride (166 mg), leucine tert-butyl ester hydrochloride (126 mg), 1-hydroxybenzotriazole monohydrate (66 mg) and triethylamine (0.09 mL). The resulting mixture was stirred at room temperature for 4.7 hours. The reaction mixture was purified by silica gel chromatography (Automatic purification system of Yamazen Corporation, ethyl acetate:n-hexane=1:2, Rf=0.40) to give the title compound (isomer A) (117 mg) and the title compound (isomer B) (118 mg).
Isomer A
MS (ESI) m/z : 669 (M + H)⁺.
¹H-NMR (CDCl₃) δ: 0.87-0.93 (6H, m), 1.47 (9H, s), 1.50-1.65 (3H, m), 3.12 (1H, dd, J = 14.8, 6.8 Hz), 3.38 (1H, dd, J = 14.8, 6.8 Hz), 3.43 (3H, s), 3.85 (3H, s), 4.43-4.49 (1H, m), 4.93 (1H, t, J = 6.8 Hz), 5.51 (1H, s), 6.30 (1H, d, J = 8.3 Hz), 6.82 (1H, d, J = 2.2 Hz), 6.98 (1H, dd, J = 8.3, 1.5 Hz), 7.18 (1H, t, J = 8.1 Hz), 7.26-7.28 (1H, m), 7.49 (1H, dd, J = 8.5, 2.4 Hz), 7.53-7.57 (1H, m). Isomer B
MS (ESI) m/z : 669 (M + H)⁺.
¹H-NMR (CDCl₃) δ: 1.00-0.94 (6H, m), 1.44 (9H, s), 1.77-1.50 (3H, m), 3.13 (1H, dd, J = 14.7, 7.6 Hz), 3.34 (1H, dd, J = 14.7, 5.4 Hz), 3.44 (3H, s), 3.86 (3H, s), 4.48-4.55 (1H, m), 4.94 (1H, dd, J = 7.6, 5.4 Hz), 5.53 (1H, s), 6.37 (1H, d, J = 8.3 Hz), 6.81 (7H, d, J = 2.2 Hz), 6.97 (7H, dd, J = 8.0, 1.6 Hz), 7.16 (1H, t, J = 8.1 Hz), 7.19-7.22 (1H, m), 7.50 (1H, dd, J = 8.6, 2.2 Hz), 7.53-7.56 (1H, m).

### Example 581

(2S)-2-({2-[(4R,6S)-8-Chloro-1-[chloro(difluoro)methyl]-6-(2,3-dimethoxyphenyl)-4H,6H-[1,2,4]triazolo[4,3-a][4,1]benzoxazepin-4-yl]acetyl}amino)-4-methylpentanoic acid

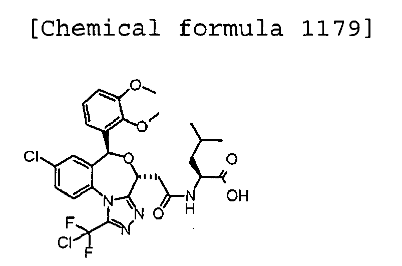

tert-Butyl (2S)-2-({2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}amino)-4-methylpentanoate (157 mg) was dissolved in methylene chloride (5 mL). To the resulting solution was added trifluoroacetic acid (2 mL). The resulting mixture was stirred at room temperature for 13 hours. The reaction mixture was concentrated under reduced pressure and the residue was solidified from methylene chloride-n-hexane to give the title compound (81 mg).
MS (ESI) m/z : 613 (M + H)⁺.
¹H-NMR (CDCl₃) δ: 0.88-0.96 (6H, m), 1.57-1.78 (3H, m), 3.17 (1H, dd, J = 14.8, 6.7 Hz), 3.38 (1H, dd, J = 14.8, 6.7 Hz), 3.43 (3H, s), 3.85 (3H, s), 4.52-4.58 (1H, m), 4.91 (1H, t, J = 6.7 Hz), 5.53 (1H, s), 6.44 (1H, d, J = 7.8 Hz), 6.83 (1H, d, J = 2.4 Hz), 6.96-6.99 (1H, m), 7.17-7.24 (0H, m), 7.51 (1H, dd, J = 8.7, 2.3 Hz), 7.54-7.57 (1H, m).
Elemental analysis for: C₂₇H₂₈Cl₂F₂N₄O₆
Calculated: C, 52.87; H, 4.60; N, 9.13.
Found: C, 52.55; H, 4.60; N, 9.09.

### Example 582

(2S)-2-({2-[(4S,6R)-8-Chloro-1-[chloro(difluoro)methyl]-6-(2,3-dimethoxyphenyl)-4H,6H-[1,2,4]triazolo[4,3-a][4,1]benzoxazepin-4-yl]acetyl}amino)-4-methylpentanoic acid

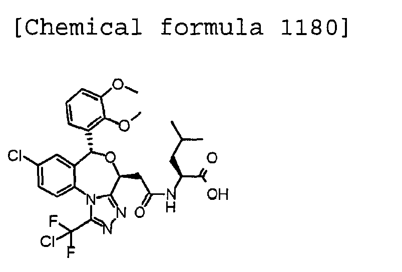

tert-Butyl (2S)-2-({2-[(4S,6R)-8-chloro-1-[chloro(difluoro)methyl]-6-(2,3-dimethoxyphenyl)-4H,6H-[1,2,4]triazolo[4,3-a] [4,1]benzoxazepin-4-yl]acetyl}amino)-4-methylpentanoate (118 mg) was dissolved in methylene chloride (5 mL). To the resulting solution was added trifluoroacetic acid (2 mL). The resulting mixture was stirred at room temperature for 13 hours. The reaction mixture was concentrated under reduced pressure and the residue was solidified from methylene chloride-n-hexane to give the title compound (81 mg).
MS (ESI) m/z : 613 (M + H)⁺.
¹H-NMR (CDCl₃) δ: 0.92-0.98 (6H, m), 1.58-1.76 (3H, m), 3.18 (1H, dd, J = 14.9, 6.5 Hz), 3.42-3.50 (4H, m), 3.86 (3H, s), 4.58-4.64 (1H, m), 4.94 (1H, t, J = 6.5 Hz), 5.52 (1H, s), 6.74 (1H, d, J = 8.1 Hz), 6.82 (1H, d, J = 2.2 Hz), 6.97-7.00 (1H, m), 7.15-7.22 (2H, m), 7.50-7.53 (1H, m), 7.56-7.59 (1H, m).
Elemental analysis for: C₂₇H₂₈Cl₂F₂N₄O₆
Calculated: C, 52.87; H, 4.60; N, 9.13.
Found: C, 52.62; H, 4.68; N, 9.06.

### Example 583

Ethyl (3S)-1-{2-[(4S,6R)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}-3-piperidine carboxylate
Ethyl (3S)-1-{2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}-3-piperidine carboxylate

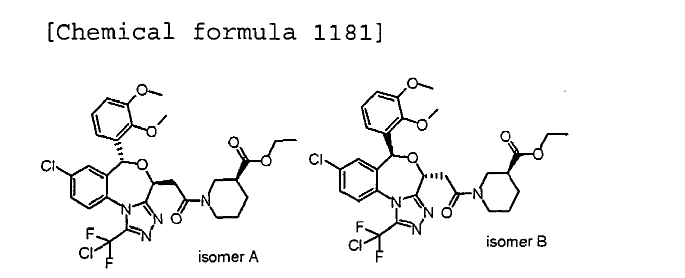

2-[4,6-trans-8-chloro-1-[chloro(difluoro)methyl]-6-(2,3-dimethoxyphenyl)-4H,6H-[1,2,4]triazolo[4,3-a][4,1]benzoxazepin-4-yl]acetic acid (289 mg) was dissolved in methylene chloride (5 mL). To the resulting solution were added 1-ethyl-3-(3-dimethylaminopropyl)carbodiimide hydrochloride (222 mg), (S)-ethyl nipecotate (136 mg), and 1-hydroxybenzotriazole monohydrate (89 mg). The resulting mixture was stirred at room temperature for 2 hours. The reaction mixture was purified by silica gel chromatography (Automatic purification system of Yamazen Corporation, ethyl acetate:n-hexane=1:1, Rf=0.30), followed by optical resolution of the mixture by HPLC (ChiralPak IA, 10 mL/min EtOH, retention time: 8 min., 24 min.) to give, as the title compounds, the isomer A (176 mg) and the isomer B (167 mg).
Isomer A MS (ESI) m/z : 639 (M + H)⁺.
¹H-NMR (CDCl₃) δ: 1.21-1.28 (3H, m), 1.44-1.86 (3H, m), 2.00-2.18 (1H, m), 2.42-2.50 (1H, m), 2.78-3.32 (3H, m), 3.42-3.44 (3H, m), 3.46-3.66 (1H, m), 3.86 (3H, s), 3.95-4.02 (1H, m), 4.09-4.62 (3H, m), 5.02-5.07 (1H, m), 5.50-5.54 (1H, m), 6.82 (1H, d, J = 2.2 Hz), 6.97-7.00 (1H, m), 7.17-7.25 (2H, m), 7.50 (1H, dd, J = 8.5, 2.3 Hz), 7.57 (1H, d, J = 8.6 Hz).
Isomer B
MS (ESI) m/z : 639 (M + H)⁺.
¹H-NMR (CDCl₃) δ: 1.22-1.29 (3H, m), 1.41-1.86 (4H, m), 2.04-2.12 (1H, m), 2.40-3.27 (3H, m), 3.43 (3H, s), 3.48-3.74 (1H, m), 3.86 (3H, s), 3.94-4.67 (4H, m), 5.03-5.08 (1H, m), 5.50-5.54 (1H, m), 6.82-6.83 (1H, m), 6.98 (1H, dd, J = 8.0, 1.6 Hz), 7.17-7.26 (2H, m), 7.48-7.52 (1H, m), 7.57 (1H, d, J = 8.6 Hz).

### Example 584

(3S)-1-{2-[(4S,6R)-8-Chloro-1-[chloro(difluoro)methyl]-6-(2,3-dimethoxyphenyl)-4H,6H-[1,2,4]triazolo[4,3-a][4,1]benzoxazepin-4-yl]acetyl}-3-piperidine carboxylic acid

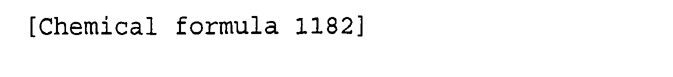

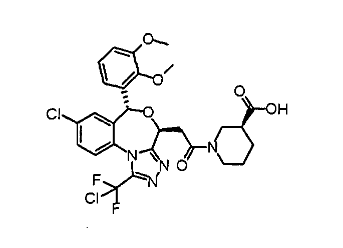

Ethyl (3S)-1-{2-[(4S,6R)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}-3-piperidine carboxylate (176 mg) was dissolved in 1,4-dioxane (4 mL). To the resulting solution were added concentrated hydrochloric acid (2 mL) and distilled water (2 mL). The resulting mixture was stirred at 60°C for 13 hours. The reaction mixture was concentrated under reduced pressure. The residue thus obtained was diluted with ethyl acetate and washed with saturated brine. The organic layer was dried over anhydrous sodium sulfate and concentrated. The residue was solidified from an ether-n-hexane solvent mixture to give the title compound (148 mg). MS (ESI) m/z : 611 (M + H)⁺.
¹H-NMR (CDCl₃) δ: 1.47-1.81 (3H, m), 1.96-2.17 (1H, m), 2.51-2.60 (1H, m), 2.99-3.35 (3H, m), 3.42-3.46 (3H, m), 3.59-3.71 (1H, m), 3.77-4.52 (5H, m), 5.03-5.08 (1H, m), 5.51 (1H, s), 6.80-6.84 (1H, m), 6.96-7.00 (1H, m), 7.16-7.24 (2H, m), 7.48-7.53 (1H, m), 7.55-7.59 (1H, m). Elemental analysis for: C₂₇H₂₆Cl₂F₂N₄O₆·0.25H₂O·0.25 1,4-dioxane
Calculated: C, 52.72; H, 4.50; N, 8.78.
Found: C, 52.55; H, 4.39; N, 8.59.

### Example 585

(3S)-1-{2-[(4R,6S)-8-Chloro-1-[chloro(difluoro)methyl]-6-(2,3-dimethoxyphenyl)-4H,6H-[1,2,4]triazolo[4,3-a][4,1]benzoxazepin-4-yl]acetyl}-3-piperidine carboxylic acid

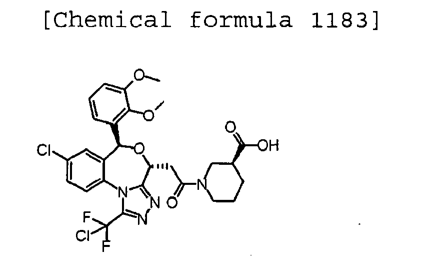

Ethyl (3S)-1-{2-[(4R,6S)-8-chloro-1-[chloro(difluoro)methyl]-6-(2,3-dimethoxyphenyl)-4H,6H-[1,2,4]triazolo[4,3-a][4,1]benzoxazepin-4-yl]acetyl}-3-piperidine carboxylate (167 mg) was dissolved in 1,4-dioxane (4 mL). To the resulting solution were added concentrated hydrochloric acid (2 mL) and distilled water (2 mL). The resulting mixture was stirred at 60°C for 13 hours. The reaction mixture was concentrated under reduced pressure. The residue thus obtained was diluted with ethyl acetate and washed with saturated brine. The organic layer was dried over anhydrous sodium sulfate and concentrated. The residue was solidified from an ether-n-hexane solvent mixture to give the title compound (148 mg). MS (ESI) m/z : 611 (M + H)⁺.
¹H-NMR (CDCl₃) δ: 1.48-1.87 (4H, m), 2.07-2.17 (1H, m), 2.49-3.29 (3H, m), 3.43 (3H, s), 3.48-3.76 (1H, m), 3.85-3.92 (4H, m), 4.00-4.53 (1H, m), 5.02-5.08 (1H, m), 5.49-5.54 (1H, m), 6.82-6.83 (1H, m), 6.96-7.00 (1H, m), 7.17-7.23 (2H, m), 7.48-7.52 (1H, m), 7.55-7.58 (1H, m). Elemental analysis for: C₂₇H₂₆Cl₂F₂N₄O₆·0.5H₂O·0.5 1,4-dioxane Calculated: C, 52.42; H, 4.70; N, 8.43.
Found: C, 52.32; H, 4.46; N, 8.27.

### Example 586

Ethyl 2-[4,6-trans-8-cyano-6-(2,3-dimethoxyphenyl)-1-(trifluoromethyl)-4H,6H-[1,2,4]triazolo[4,3-a] [4,1]benzoxazepin-4-yl]acetate

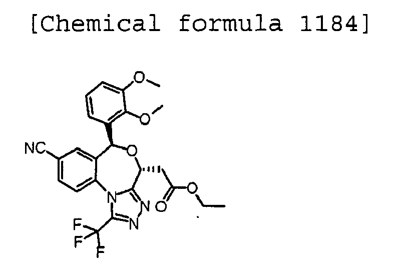

Ethyl 2-[4,6-trans-8-bromo-6-(2,3-dimethoxyphenyl)-1-(trifluoromethyl)-4H,6H-[1,2,4]triazolo[4,3-a] [4,1]benzoxazepin-4-yl]acetate (556 mg) was dissolved in acetonitrile (10 mL). To the resulting solution were added potassium cyanide (98 mg), 9,9-dimethyl-4,6-bis(diphenylphosphino)xanthene (30 mg), bis(dibenzylideneacetone)palladium(0) (46 mg) and a 1N tributyltin chloride-hexane solution (0.52 mL) under a nitrogen gas stream. The resulting mixture was stirred at room temperature for 30 minutes and at 80°C for 17 hours and then heated under reflux for 2 hours. The reaction mixture was diluted with ethyl acetate, washed with saturated brine, dried over anhydrous sodium sulfate and then concentrated. The residue thus obtained was purified by silica gel chromatography (Automatic purification system of Yamazen Corporation, ethyl acetate: n-hexane=1/2, Rf=0.37) to give the title compound (194 mg) and collect the raw material (350 mg).
MS (ESI) m/z : 503 (M + H)⁺.
¹H-NMR (CDCl₃) δ: 1.28 (3H, t, J = 7.2 Hz), 3.25 (1H, dd, J = 16.8, 6.8 Hz), 3.45 (3H, s), 3.52 (1H, dd, J = 16.8, 6.8 Hz), 3.88 (3H, s), 4.19 (2H, q, J = 7.2 Hz), 4.92 (1H, t, J = 6.8 Hz), 5.57 (1H, s), 7.01 (1H, dd, J = 6.8, 2.9 Hz), 7.17-7.22 (3H, m), 7.71 (1H, d, J = 8.3 Hz), 7.85 (1H, dd, J = 8.3, 1.7 Hz).

### Example 587

Ethyl 2-[(4S,6R)-8-cyano-6-(2,3-dimethoxyphenyl)-1-(trifluoromethyl)-4H,6H-[1,2,4]triazolo[4,3-a][4,1]benzoxazepin-4-yl]acetate (isomer A) Ethyl 2-[(4R,6S)-8-cyano-6-(2,3-dimethoxyphenyl)-l-(trifluoromethyl)-4H,6H-[1,2,4]triazolo[4,3-a][4,1]benzoxazepin-4-yl]acetate (isomer B)

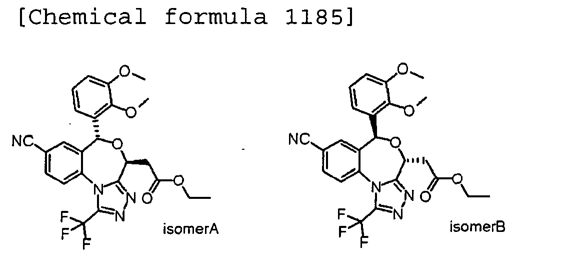

Ethyl 2-[4,6-trans-8-cyano-6-(2,3-dimethoxyphenyl)-1-(trifluoromethyl)-4H,6H-[1,2,4]triazolo[4,3-a][4,1]benzoxazepin-4-yl]acetate was optically resolved by HPLC.
Column: Chiralpak AD (2 × 25cm)
Eluting solution: 30% isopropanol-n-hexane
Flow rate: 10 mL/min
Elution time: isomer A: 27 min., isomer B: 30 min.

### Example 588

2-[(4S,6R)-8-Cyano-6-(2,3-dimethoxyphenyl)-1-(trifluoromethyl)-4H,6H-[1,2,4]triazolo[4,3-a] [4,1]benzoxazepin-4-yl]acetic acid

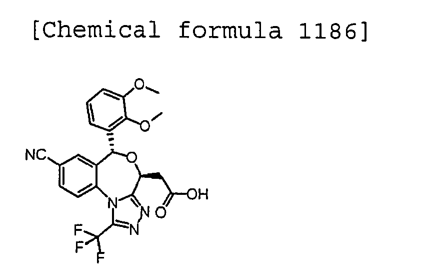

Ethyl 2-[(4S,6R)-8-cyano-6-(2,3-dimethoxyphenyl)-1-(trifluoromethyl)-4H,6H-[1,2,4]triazolo[4,3-a] [4,1]benzoxazepin-4-yl]acetate(91 mg) was dissolved in 1,4-dioxane (8 mL). To the resulting solution were added concentrated hydrochloric acid (4 mL) and distilled water (4 mL). The resulting mixture was stirred at 60°C for 13 hours. The reaction mixture was concentrated under reduced pressure. The residue thus obtained was purified by preparative thin layer chromatography on silica gel (a chloroform:methanol:water=7:3:1 organic layer), followed by solidification from an n-hexane solvent mixture to give the title compound (75 mg).
MS (ESI) m/z : 475 (M + H)⁺.
¹H-NMR (CDCl₃) δ: 3.14-3.24 (1H, m), 3.38-3.49 (4H, m), 3.87 (3H, s), 4.85-4.91 (1H, m), 5.55 (1H, s), 6.98-7.02 (1H, m), 7.14-7.22 (3H, m), 7.69 (1H, d, J = 7.7 Hz), 7.79 (1H, d, J = 7.7 Hz).
Elemental analysis for: C₂₂H₁₇F₃N₄O₅·0.25CHCl₃·0.25 n-Hexane Calculated: C, 52.69; H, 4.24; N, 10.24.
Found: C, 52.48; H, 3.88; N, 10.16.

### Example 589

2-[(4R,6S)-8-Cyano-6-(2,3-dimethoxyphenyl)-1-(trifluoromethyl)-4H,6H-[1,2,4]triazolo[4,3-a] [4,1]benzoxazepin-4-yl]acetic acid

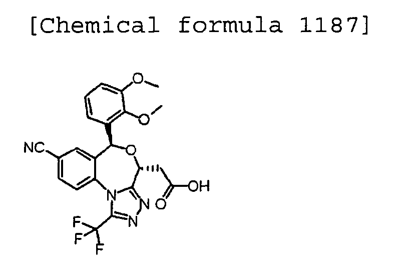

Ethyl 2-[(4S,6R)-8-cyano-6-(2,3-dimethoxyphenyl)-1-(trifluoromethyl)-4H,6H-[1,2,4]triazolo[4,3-a] [4,1]benzoxazepin-4-yl]acetate (91 mg) was dissolved in 1,4-dioxane (8 mL). To the resulting solution were added concentrated hydrochloric acid (4 mL) and distilled water (4 mL). The resulting mixture was stirred at 60°C for 13 hours. The reaction mixture was concentrated under reduced pressure. The residue thus obtained was purified by preparative thin layer chromatography on silica gel (a chloroform:methanol:water=7:3:1 organic layer), followed by solidification from an n-hexane solvent mixture to give the title compound (75 mg).
MS (ESI) m/z : 475 (M + H)⁺.
¹H-NMR (CDCl₃) δ: 3.14-3.26 (1H, m), 3.39-3.50 (4H, m), 3.87 (3H, s), 4.84-4.90 (1H, m), 5.55 (1H, s), 6.98-7.03 (1H, m), 7.15-7.23 (3H, m), 7.68-7.73 (1H, m), 7.79-7.85 (1H, m).
Elemental analysis for: C₂₂H₁₇F₃N₄O₅·0.75 H₂O·0.25CHCl₃·0.25 n-Hexane
Calculated: C, 53.13; H, 4.18; N, 10.33.
Found: C, 53.31; H, 3.97; N, 10.59.

### Example 590

Ethyl 2-[4,6-trans-1-[chloro(difluoro)methyl]-6-(2,3-dimethoxyphenyl)-8-(trifluoromethyl)-4H,6H-[1,2,4]triazolo[4,3-a][4,1]benzoxazepin-4-yl]acetate

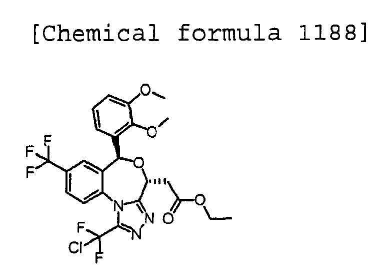

Ethyl 2-[3,5-trans-5-(2,3-dimethoxyphenyl)-2-thioxo-7-(trifluoromethyl)-1,2,3,5-tetrahydro-4,1-benzoxazepin-3-yl]acetate (939 mgl) was dissolved in tetrahydrofuran (20 mL). To the resulting solution was added hydrazine monohydrate (0.146 mL). The resulting mixture was stirred at room temperature for 1.6 hours. To the reaction mixture was added chlorodifluoroacetic anhydride (1.41 mL) and the mixture was stirred at room temperature for 4.3 hours. The reaction mixture was diluted with ethyl acetate and then washed with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.55) to give the title compound (1095 mg).
MS (ESI) m/z : 562 (M + H)⁺.
¹H-NMR (CDCl₃) δ: 1.28 (3H, t, J = 7.2 Hz), 3.25 (1H, dd, J = 16.7, 6.8 Hz), 3.41 (3H, s), 3.51 (1H, dd, J = 16.7, 6.8 Hz), 3.86 (3H, s), 4.16-4.22 (2H, m), 4.90 (1H, t, J = 6.8 Hz), 5.61 (1H, s), 7.00 (1H, dd, J = 7.7, 2.0 Hz), 7.13 (1H, d, J = 1.7 Hz), 7.18-7.25 (2H, m), 7.78 (1H, d, J = 8.5 Hz), 7.83 (1H, dd, J = 8.5, 2.0 Hz).

### Example 591

Ethyl 2-[(4S,6R)-1-[chloro(difluoro)methyl]-6-(2,3-dimethoxyphenyl)-8-(trifluoromethyl)-4H,6H-[1,2,4]triazolo[4,3-a][4,1]benzoxazepin-4-yl]acetate isomer A
Ethyl 2-[(4R,6S)-1-[chloro(difluoro)methyl]-6-(2,3-dimethoxyphenyl)-8-(trifluoromethyl)-4H,6H-[1,2,4]triazolo[4,3-a][4,1]benzoxazepin-4-yl]acetate isomer B

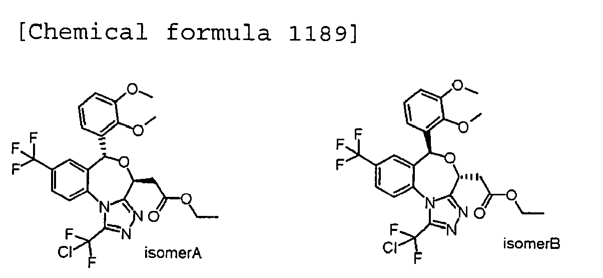

Ethyl 2-[4,6-trans-1-[chloro(difluoro)methyl]-6-(2,3-dimethoxyphenyl)-8-(trifluoromethyl)-4H,6H-[1,2,4]triazolo[4,3-a][4,1]benzoxazepin-4-yl]acetate was optically resolved by HPLC.
Column: Chiralpak AD (5 × 50cm)
Eluting solution: 15% isopropanol-n-hexane
Flow rate: 30 mL/min
Elution time: isomer A: 60 min., isomer B: 105 min.

### Example 592

2-[(4S,6R)-1-[Chloro(difluoro)methyl]-6-(2,3-dimethoxyphenyl)-8-(trifluoromethyl)-4H,6H-[1,2,4]triazolo[4,3-a][4,1]benzoxazepin-4-yl]acetic acid

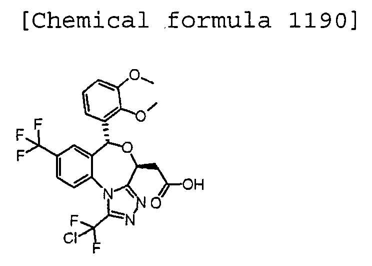

Ethyl 2-[(4S,6R)-1-[chloro(difluoro)methyl]-6-(2,3-dimethoxyphenyl)-8-(trifluoromethyl)-4H,6H-[1,2,4]triazolo[4,3-a][4,1]benzoxazepin-4-yl]acetate (522 mg) was dissolved in 1,4-dioxane (30 mL). To the resulting solution were added concentrated hydrochloric acid (15 mL) and distilled water (15 mL). The resulting mixture was stirred at 60°C for 14 hours. The reaction mixture was concentrated under reduced pressure. The residue thus obtained was solidified from a methylene chloride-n-hexane solvent mixture to give the title compound(461 mg).
MS (ESI) m/z : 534 (M + H)⁺.
¹H-NMR (CDCl₃) δ: 3.31 (1H, dd, J = 17.2, 6.6 Hz), 3.41 (3H, s), 3.59 (1H, dd, J = 17.2, 6.6 Hz), 3.86 (3H, s), 4.86 (1H, t, J = 6.6 Hz), 5.61 (1H, s), 7.00 (1H, dd, J = 7.7, 2.1 Hz), 7.12-7.14 (1H, m), 7.18-7.25 (2H, m), 7.78 (1H, d, J = 8.3 Hz), 7.83 (1H, dd, J = 8.3, 1.7 Hz). Elemental analysis for: C₂₂H₁₇ClF₅N₃O₅
Calculated: C, 49.50; H, 3.21; N, 7.87.
Found: C, 49.16; H, 3.37; N, 7.68.

### Example 593

2-[(4R,6S)-1-[Chloro(difluoro)methyl]-6-(2,3-dimethoxyphenyl)-8-(trifluoromethyl)-4H,6H-[1,2,4]triazolo[4,3-a] [4,1]benzoxazepin-4-yl]acetic acid

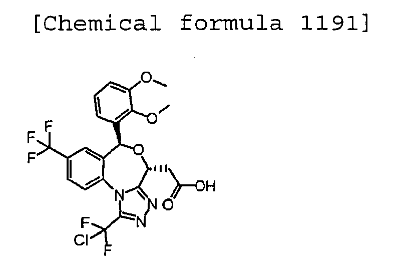

Ethyl 2-[(4S,6R)-1-[chloro(difluoro)methyl]-6-(2,3-dimethoxyphenyl)-8-(trifluoromethyl)-4H,6H-[1,2,4]triazolo[4,3-a][4,1]benzoxazepin-4-yl]acetate (505 mg) was dissolved in 1,4-dioxane (30 mL). To the resulting solution were added concentrated hydrochloric acid (15 mL) and distilled water (15 mL). The resulting mixture was stirred at 60°C for 14 hours. The reaction mixture was concentrated under reduced pressure. The residue thus obtained was solidified from a methylene chloride-n-hexane solvent mixture to give the title compound (442 mg).
MS (ESI) m/z : 534 (M + H)⁺.
¹H-NMR (CDCl₃) δ: 3.31 (1H, dd, J = 16.9, 6.6 Hz), 3.41 (3H, s), 3.59 (1H, dd, J = 16.9, 6.6 Hz), 3.86 (3H, s), 4.86 (1H, t, J = 6.6 Hz), 5.61 (1H, s), 7.00 (1H, dd, J = 7.6, 2.0 Hz), 7.13-7.14 (1H, m), 7.18-7.25 (2H, m), 7.78 (1H, d, J = 8.3 Hz), 7.82-7.85 (1H, m).
Elemental analysis for: C₂₂H₁₇ClF₅N₃O₅·0.25 H₂O
Calculated: C, 49.08; H, 3.28; N, 7.81.
Found: C, 49.07; H, 3.34; N, 7.49.

### Example 594

Ethyl 2-[4,6-trans-1-(1,1-difluoroethyl)-6-(2,3-dimethoxyphenyl)-8-(trifluoromethyl)-4H,6H-[1,2,4]triazolo[4,3-a] [4,1]benzoxazepin-4-yl]acetate

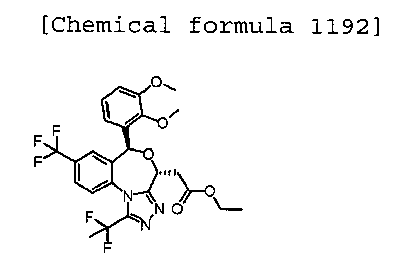

Ethyl 2-[3,5-trans-5-(2,3-dimethoxyphenyl)-2-thioxo-7-(trifluoromethyl)-1,2,3,5-tetrahydro-4,1-benzoxazepin-3-yl]acetate (939 mg) was dissolved in tetrahydrofuran (20 mL). To the resulting solution was added hydrazine monohydrate (0.146 mL). The resulting mixture was stirred at room temperature for 2 hours. The reaction mixture was diluted with ethyl acetate and washed with saturated brine. The organic layer was dried over anhydrous sodium sulfate. After concentration of the filtrate, the residue thus obtained was dissolved in methylene chloride (10 mL). To the resulting solution were added 2,2-difluoropropionic acid (242 mg), 1-ethyl-3-(3-dimethylaminopropyl)carbodiimide hydrochloride (767 mg), and 1-hydroxybenzotriazole monohydrate (337 mg). The resulting mixture was stirred at room temperature for 19 hours. The reaction mixture was diluted with ethyl acetate and washed with saturated brine. The organic layer was dried over anhydrous sodium sulfate. After concentration of the filtrate, the residue thus obtained was diluted with acetic acid (10 mL) and heated under reflux for 3 hours. After concentration, the residue was diluted with ethyl acetate and washed successively with a saturated aqueous solution of sodium hydrogen carbonate and saturated brine. The organic layer was dried over anhydrous sodium sulfate and concentrated. The residue was purified by silica gel chromatography (Automatic purification system of Yamazen Corporation, ethyl acetate:n-hexane=1:2, Rf=0.17) to give the title compound (303 mg).
MS (ESI) m/z : 562 (M + H)⁺.
¹H-NMR (CDCl₃) δ: 1.28 (3H, t, J = 7.2 Hz), 2.34 (3H, t, J = 19.3 Hz), 3.23 (1H, dd, J = 16.6, 6.8 Hz), 3.35 (3H, s), 3.49 (1H, dd, J = 16.6, 6.8 Hz), 3.85 (3H, s), 4.16-4.22 (2H, m), 4.90 (1H, t, J = 6.8 Hz), 5.65 (1H, s), 6.98 (1H, dd, J = 8.2, 1.7 Hz), 7.11 (1H, d, J = 1.7 Hz), 7.17-7.25 (2H, m), 7.79 (1H, dd, J = 8.2, 1.7 Hz), 7.92 (1H, d, J = 8.4 Hz).

### Example 595

Ethyl 2-[(4S,6R)-1-(1,1-difluoroethyl)-6-(2,3-dimethoxyphenyl)-8-(trifluoromethyl)-4H,6H-[1,2,4]triazolo[4,3-a][4,1]benzoxazepin-4-yl]acetate isomer A
Ethyl 2-[(4R,6S)-1-(1,1-difluoroethyl)-6-(2,3-dimethoxyphenyl)-8-(trifluoromethyl)-4H,6H-[1,2,4]triazolo[4,3-a][4,1]benzoxazepin-4-yl]acetate isomer B

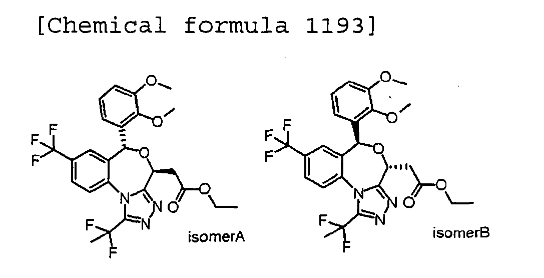

Ethyl 2-[4,6-trans-1-(1,1-difluoroethyl)-6-(2,3-dimethoxyphenyl)-8-(trifluoromethyl)-4H,6H-[1,2,4]triazolo[4,3-a][4,1]benzoxazepin-4-yl]acetate was optically resolved by HPLC.
Column: Chiralpak AD (5 × 50cm)
Eluting solution: 30% isopropanol-n-hexane
Flow rate: 50 mL/min
Elution time: isomer A: 29 min., isomer B: 105 min.

### Example 596

2-[(4S,6R)-1-(1,1-Difluoroethyl)-6-(2,3-dimethoxyphenyl)-8-(trifluoromethyl)-4H,6H-[1,2,4]triazolo[4,3-a][4,1]benzoxazepin-4-yl]acetic acid

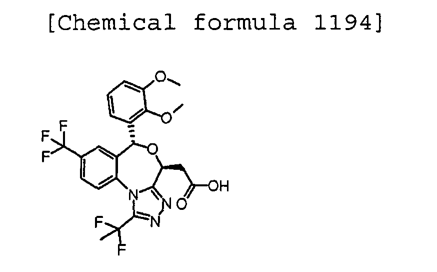

Ethyl 2-[(4S,6R)-1-(1,1-difluoroethyl)-6-(2,3-dimethoxyphenyl)-8-(trifluoromethyl)-4H,6H-[1,2,4]triazolo[4,3-a][4,1]benzoxazepin-4-yl]acetate (133 mg) was dissolved in 1,4-dioxane (8 mL). To the resulting solution were added concentrated hydrochloric acid (4 mL) and distilled water (4 mL). The resulting mixture was stirred at 60°C for 12 hours. The reaction mixture was concentrated under reduced pressure. The residue thus obtained was solidified from a methylene chloride-n-hexane solvent mixture to give the title compound (99 mg).
MS (ESI) m/z : 514 (M + H)⁺.
¹H-NMR (CDCl₃) δ: 2.34 (3H, t, J = 19.3 Hz), 3.29 (1H, dd, J = 16.9, 6.7 Hz), 3.35 (3H, s), 3.56 (1H, dd, J = 16.9, 6.7 Hz), 3.85 (3H, s), 4.86 (1H, t, J = 6.7 Hz), 5.64 (1H, s), 6.99 (1H, dd, J = 8.1, 1.7 Hz), 7.12 (1H, d, J = 1.5 Hz), 7.20 (1H, t, J = 7.9 Hz), 7.23-7.26 (1H, m), 7.79 (1H, dd, J = 8.4, 1.7 Hz), 7.92 (1H, d, J = 8.4 Hz).
Elemental analysis for: C₂₃H₂₀F₅N₃O₅
Calculated: C, 53.81; H, 3.93; N, 8.18.
Found: C, 53.73; H, 4.24; N, 7.97.

### Example 597

2-[(4R,6S)-1-(1,1-Difluoroethyl)-6-(2,3-dimethoxyphenyl)-8-(trifluoromethyl)-4H,6H-[1,2,4]triazolo[4,3-a] [4,1]benzoxazepin-4-yl]acetic acid

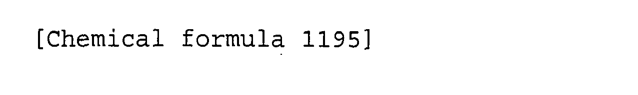

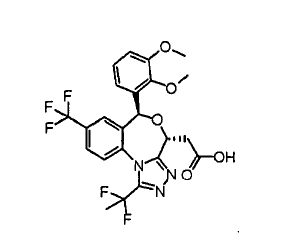

Ethyl 2-[(4R,6S)-1-(1,1-difluoroethyl)-6-(2,3-dimethoxyphenyl)-8-(trifluoromethyl)-4H,6H-[1,2,4]triazolo[4,3-a][4,1]benzoxazepin-4-yl]acetate (129 mg) was dissolved in 1,4-dioxane (8 mL). To the resulting solution were added concentrated hydrochloric acid (4 mL) and distilled water (4 mL). The resulting mixture was stirred at 60°C for 12 hours. The reaction mixture was concentrated under reduced pressure. The residue thus obtained was solidified from a methylene chloride-n-hexane solvent mixture to give the title compound (111 mg).
MS (ESI) m/z : 514 (M + H)⁺.
¹H-NMR (CDCl₃) δ: 2.34 (3H, t, J = 19.3 Hz), 3.30 (1H, dd, J = 16.9, 6.7 Hz), 3.35 (3H, s), 3.56 (1H, dd, J = 16.9, 6.7 Hz), 3.85 (3H, s), 4.86 (1H, t, J = 6.7 Hz), 5.65 (1H, s), 6.99 (1H, dd, J = 8.2, 1.6 Hz), 7.12 (1H, d, J = 1.7 Hz), 7.20 (1H, t, J = 7.9 Hz), 7.24-7.26 (1H, m), 7.79 (1H, dd, J = 8.7, 1.8 Hz), 7.92 (1H, d, J = 8.3 Hz).
Elemental analysis for: C₂₃H₂₀F₅N₃O₅·0.25 H₂O
Calculated: C, 53.34; H, 3.99; N, 8.11.
Found: C, 53.17; H, 3.93; N, 8.04.

### Referential Example 405

2-[(4R,6S)-8-Chloro-6-(2,3-dimethoxyphenyl)-1-(trifluoromethyl)-4H,6H-[1,2,4]triazolo[4,3-a] [4,1]benzoxazepin-4-yl]-1-ethanol

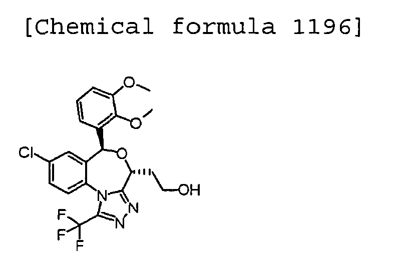

2-[(4S,6R)-8-Chloro-6-(2,3-dimethoxyphenyl)-1-(trifluoromethyl)-4H,6H-[1,2,4]-triazolo-[4,3-a][4,1]benzoxazepin-4-yl]acetic acid (968 mg) was dissolved in tetrahydrofuran (10 mL). To the resulting solution was added N-methylmorpholine (0.26 mL). Under ice cooling, ethyl chloroformate (0.23 mL) was added and the resulting mixture was stirred for 1.1 hours at the same temperature. Sodium borohydride (227 mg) and methanol (10 mL) were added at the same temperature, followed by stirring at room temperature for 7.5 hours. To the reaction mixture was added a 10% aqueous citric acid solution. The resulting mixture was extracted with ethyl acetate. The organic layer was washed successively with a saturated aqueous solution of sodium hydrogen carbonate and saturated brine, and dried over anhydrous sodium sulfate. The solvent was distilled off under reduced pressure to give the title compound (968 mg).
MS (ESI) m/z : 470 (M + H)⁺.
¹H-NMR (CDCl₃) δ: 2.55-2.61 (2H, m), 3.45 (3H, s), 3.70-3.75 (1H, m), 3.86 (3H, s), 3.96-4.00 (0H, m), 4.66 (1H, t, J = 6.4 Hz), 5.56 (1H, s), 6.85-6.87 (1H, m), 6.97-7.00 (1H, m), 7.18-7.24 (2H, m), 7.51-7.52 (2H, m).

### Referential Example 406

3-[(4R,6S)-8-Chloro-6-(2,3-dimethoxyphenyl)-1-(trifluoromethyl)-4H,6H-[1,2,4]triazolo[4,3-a][4,1]benzoxazepin-4-yl]propanenitrile

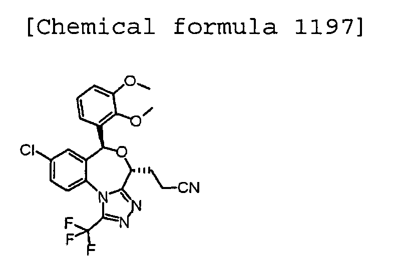

To a solution of 2-[(4R,6S)-8-Chloro-6-(2,3-dimethoxyphenyl)-1-(trifluoromethyl)-4H,6H-[1,2,4]triazolo[4,3-a][4,1]benzoxazepin-4-yl]-1-ethanol (968 mg) and triethylamine (574 µL) in dichloromethane (10 mL) was added mesyl chloride (239 µL) under ice cooling. The resulting mixture was stirred at the same temperature for 2 hours. To the reaction mixture was added ethyl acetate. The resulting mixture was washed successively with a 10% aqueous citric acid solution and saturated brine and then, dried over anhydrous sodium sulfate. The filtrate was concentrated under reduced pressure.
The residue thus obtained was dissolved in dimethylsulfoxide (10 mL). To the resulting solution was added sodium cyanide (208 mg). The resulting mixture was stirred at 50°C for 4 hours. To the reaction mixture was added ethyl acetate. The resulting mixture was washed with saturated brine and dried over anhydrous sodium sulfate. After filtration, the filtrate was concentrated under reduced pressure. The residue was purified by silica gel chromatography (Automatic purification system of Yamazen Corporation, ethyl acetate:n-hexane=1:2, Rf=0.60) to give the title compound (788 mg).
MS (ESI) m/z : 479 (M + H)⁺.
¹H-NMR (CDCl₃) δ: 2.58-2.83 (4H, m), 3.44 (3H, s), 3.87 (3H, s), 4.59 (1H, dd, J = 7.7, 5.2 Hz), 5.57 (1H, s), 6.87 (1H, d, J = 2.0 Hz), 7.00 (1H, t, J = 4.9 Hz), 7.21-7.23 (2H, m), 7.52-7.54 (2H, m).

### Example 598

3-[(4R,6S)-8-Chloro-6-(2,3-dimethoxyphenyl)-1-(trifluoromethyl)-4H,6H-[1,2,4]triazolo[4,3-a] [4,1]benzoxazepin-4-yl]propanoicacid

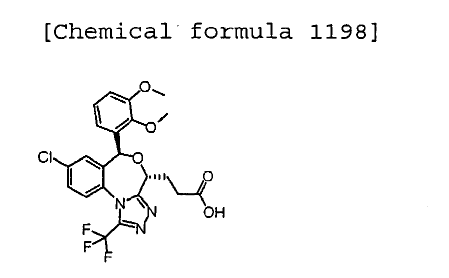

3-[(4R,6S)-8-Chloro-6-(2,3-dimethoxyphenyl)-1-(trifluoromethyl)-4H,6H-[1,2,4]triazolo[4,3-a] [4,1]benzoxazepin-4-yl]propanenitrile (788 mg) was dissolved in 1,4-dioxane (20 mL). To the resulting solution were added concentrated hydrochloric acid (10 mL) and distilled water (10 mL). The resulting mixture was stirred at 60°C for 12 hours. The reaction mixture was diluted with ethyl acetate and washed with saturated brine. The organic layer was dried over anhydrous sodium sulfate and concentrated under reduced pressure. The residue thus obtained was purified by silica gel chromatography (Automatic purification system of Yamazen Corporation, 10% methanol-chloroform), followed by solidification from a methylene chloride-n-hexane solvent mixture to give the title compound (580 mg).
MS (ESI) m/z : 498 (M + H)⁺.
¹H-NMR (CDCl₃) δ: 2.62-2.69 (2H, m), 2.75-2.80 (2H, m), 3.43 (3H, s), 3.86 (3H, s), 4.52 (1H, dd, J = 7.2, 5.5 Hz), 5.54 (1H, s), 6.84 (1H, d, J = 1.5 Hz), 6.99 (1H, dd, J = 7.5, 2.3 Hz), 7.17-7.22 (2H, m), 7.50-7.52 (2H, m).
Elemental analysis for: C₂₂H₁₉ClF₃N₃O₅·0.25 n-Hexane
Calculated: C, 54.34; H, 4.37; N, 8.09.
Found: C, 54.14; H, 4.30; N, 7.74.

### Example 599

Ethyl 2-(1-{3-[(4R,6S)-8-chloro-6-(2,3-dimethoxyphenyl)-1-(trifluoromethyl)-4H,6H-[1,2,4]triazolo[4,3-a][4,1]benzoxazepin-4-yl]propanoyl}-4-piperidinyl)acetate

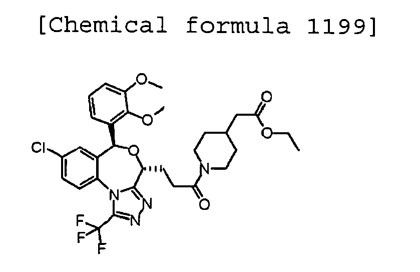

In a similar manner to that employed for the synthesis of ethyl 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetate, the title compound (266 mg) was obtained using 3-[(4R,6S)-8-chloro-6-(2,3-dimethoxyphenyl)-1-(trifluoromethyl)-4H,6H-[1,2,4]triazolo[4,3-a][4,1]benzoxazepin-4-yl]propanoicacid (308 mg) .
MS (ESI) m/z : 651 (M + H)⁺.
¹H-NMR (CDCl₃) δ: 1.07-1.20 (2H, m), 1.26 (3H, t, J = 7.2 Hz), 1.70-1.82 (2H, m), 1.96-2.06 (1H, m), 2.20-2.25 (2H, m), 2.50-2.80 (5H, m), 2.98-3.07 (1H, m), 3.42-3.44 (3H, m), 3.86 (3H, s), 3.90-3.97 (1H, m), 4.13 (2H, q, J = 7.1 Hz), 4.51-4.62 (2H, m), 5.52-5.54 (1H, m), 6.82-6.85 (1H, m), 6.97-7.00 (1H, m), 7.17-7.23 (2H, m), 7.49-7.52 (2H, m).

### Example 600

2-(1-{3-[(4R,6S)-8-Chloro-6-(2,3-dimethoxyphenyl)-1-(trifluoromethyl)-4H,6H-[1,2,4]triazolo[4,3-a][4,1]benzoxazepin-4-yl]propanoyl}-4-piperidinyl)acetic acid

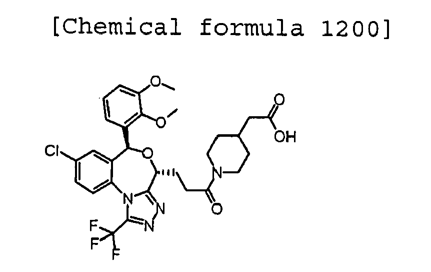

In a similar manner to that employed for the synthesis of 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetic acid, the title compound (192 mg) was obtained using ethyl 2-(1-{3-[(4R,6S)-8-chloro-6-(2,3-dimethoxyphenyl)-1-(trifluoromethyl)-4H,6H-[1,2,4]triazolo[4,3-a][4,1]benzoxazepin-4-yl]propanoyl}-4-piperidinyl)acetate (266 mg).
MS (ESI) m/z : 623 (M + H)⁺.
¹H-NMR (CDCl₃) δ: 1.04-1.21 (2H, m), 1.73-1.87 (2H, m), 1.96-2.07 (1H, m), 2.26-2.31 (2H, m), 2.51-2.80 (5H, m), 2.99-3.07 (1H, m), 3.42-3.44 (3H, m), 3.85-3.87 (3H, m), 3.92-3.99 (1H, m), 4.50-4.62 (2H, m), 5.52-5.54 (1H, m), 6.82-6.84 (1H, m), 6.97-7.00 (1H, m), 7.18-7.23 (2H, m), 7.49-7.52 (2H, m).
Elemental analysis for: C₂₉H₃₀ClF₃N₄O₆·1.25 CHCl₃·0.25 diisopropyl ether
Calculated: C, 47.64; H, 4.60; N, 6.73.
Found: C, 47.87; H, 4.80; N, 7.05.

### Example 601

tert-Butyl 2-(1-{2-[(4R,6S)-8-cyano-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate

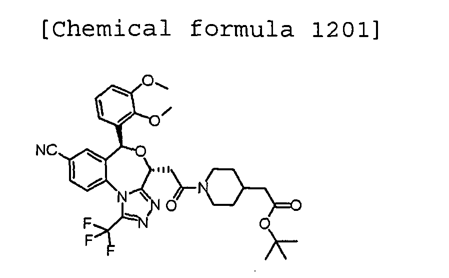

2-[(4R,6S)-8-Cyano-6-(2,3-dimethoxyphenyl)-1-(trifluoromethyl)-4H,6H-[1,2,4]triazolo[4,3-a][4,1]benzoxazepin-4-yl]acetic acid (20 mg) was dissolved in methylene chloride (2 mL). To the resulting solution were added 1-ethyl-3-(3-dimethylaminopropyl)carbodiimide hydrochloride (16 mg), tert-butyl 2-(4-piperidinyl)acetate (11 mg), and 1-hydroxybenzotriazole monohydrate (6 mg).
The resulting mixture was stirred at room temperature for 2 hours. The reaction mixture was purified by preparative thin layer chromatography on silica gel (ethyl acetate:n-hexane=2:1) to give the title compound (31 mg).
MS (ESI) m/z 656 (M + H)⁺.
¹H-NMR (CDCl₃) δ: 1.09-1.22 (2H, m), 1.45 (9H, s), 1.70-1.86 (2H, m), 1.97-2.06 (1H, m), 2.13-2.15 (1H, m), 2.18-2.20 (1H, m), 2.57-2.66 (1H, m), 3.05-3.23 (2H, m), 3.43 (3H, s), 3.53-3.66 (1H, m), 3.87 (3H, s), 3.97-4.06 (1H, m), 4.49-4.56 (1H, m), 5.03-5.08 (1H, m), 5.53-5.55 (1H, m), 6.99-7.03 (1H, m), 7.16 (1H, d, J = 2.0 Hz), 7.20-7.23 (2H, m), 7.70 (1H, d, J = 8.3 Hz), 7.83 (1H, dd, J = 8.3, 1.7 Hz) .

### Example 602

2-(1-{2-[(4R,6S)-8-Cyano-6-(2,3-dimethoxyphenyl)-1-(trifluoromethyl)-4H,6H-[1,2,4]triazolo[4,3-a] [4,1]benzoxazepin-4-yl]acetyl}-4-piperidinyl)acetic acid

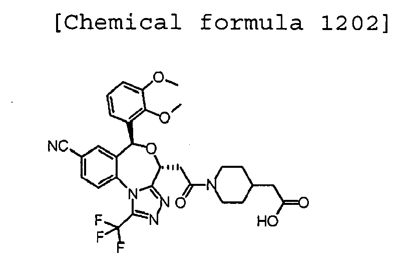

tert-Butyl 2-(1-{2-{(4R,6S)-8-cyano-6-(2,3-dimethoxyphenyl)-1-(trifluoromethyl)-4H,6H-[1,2,4]triazolo[4,3-a][4,1]benzoxazepin-4-yl]acetyl}-4-piperidinyl)acetate (31 mg) was dissolved in methylene chloride (5 mL). To the resulting solution was added trifluoroacetic acid (1 mL). The resulting mixture was stirred at room temperature for 1.4 hours. The reaction mixture was concentrated under reduced pressure, followed by azeotropy with n-hexane. The residue thus obtained was lyophilized from 1,4-dioxane to give the title compound (26 mg) .
MS (ESI) m/z : 600 (M + H)⁺.
¹H-NMR (CDCl₃) δ: 1.13-1.27 (2H, m), 1.75-2.03 (3H, m), 2.27-2.30 (1H, m), 2.33-2.36 (1H, m), 2.58-2.67 (1H, m), 3.06-3.25 (2H, m), 3.43 (3H, s), 3.54-3.68 (1H, m), 3.87 (3H, s), 3.99-4.09 (1H, m), 4.51-4.58 (1H, m), 5.04-5.08 (1H, m), 5.53-5.56 (1H, m), 6.99-7.03 (1H, m), 7.16-7.17 (1H, m), 7.20-7.23 (2H, m), 7.70 (1H, d, J = 8.1 Hz), 7.84 (1H, dd, J = 8.4, 1.6 Hz).
Elemental analysis for: C₂₉H₂₈F₃N₅O₆·0.75 H₂O·0.5 CHCl₃·0.1 1,4-dioxane
Calculated: C, 53.07; H, 4.58; N, 10.18.
Found: C, 53.36; H, 4.62; N, 9.89.

### Example 603

Ethyl 2-[4,6-trans-1-(difluoromethyl)-6-(2,3-dimethoxyphenyl)-8-(trifluoromethyl)-4H,6H-[1,2,4]triazolo[4,3-a][4,1]benzoxazepin-4-yl]acetate

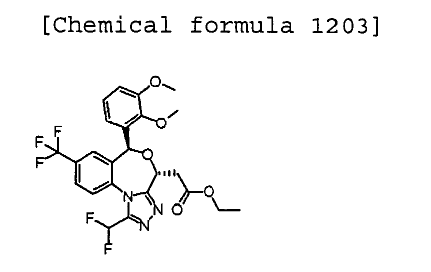

Ethyl 2-[3,5-trans-5-(2,3-dimethoxyphenyl)-2-thioxo-7-(trifluoromethyl)-1,2,3,5-tetrahydro-4,1-benzoxazepin-3-yl]acetate (704 mg) was dissolved in tetrahydrofuran (15 mL). To the resulting solution was added hydrazine monohydrate (0.11 mL). The resulting mixture was stirred at room temperature for 1.2 hours. Under ice cooling, difluoroacetic anhydride (1.04 mL) was added to the reaction mixture. The resulting mixture was stirred at room temperature for 2.8 hours and then heated under reflux for 1.2 hours. The reaction mixture was diluted with ethyl acetate and then washed with a saturated aqueous solution of sodium bicarbonate and saturated brine. The organic layer was dried over sodium sulfate and concentrated under reduced pressure. The residue thus obtained was purified by silica gel chromatography (Automatic purification system of Yamazen Corporation, ethyl acetate:n-hexane=1:2, Rf=0.29) to give the title compound (646 mg).
MS (ESI) m/z : 528 (M + H)⁺.
¹H-NMR (CDCl₃) δ: 3.24 (1H, dd, J = 16.5, 6.8 Hz), 3.36 (3H, s), 3.50 (1H, dd, J = 16.5, 6.8 Hz), 3.85 (3H, s), 4.16-4.22 (2H, m), 4.95 (1H, t, J = 6.8 Hz), 5.64 (1H, s), 6.98 (1H, dd, J = 7.9, 1.6 Hz), 7.12-7.14 (1H, m), 7.19 (1H, t, J = 7.9 Hz), 7.22-7.25 (1H, m), 7.79-7.83 (1H, m), 7.88 (1H, d, J = 8.4 Hz).

### Example 604

Ethyl 2-[(4S,6R)-1-(difluoromethyl)-6-(2,3-dimethoxyphenyl)-8-(trifluoromethyl)-4H,6H-[1,2,4]triazolo[4,3-a] [4,1]benzoxazepin-4-yl]acetate isomer A
Ethyl 2-[(4R,6S)-l-(difluoromethyl)-6-(2,3-dimethoxyphenyl)-8-(trifluoromethyl)-4H,6H-[1,2,4]triazolo[4,3-a] [4,1]benzoxazepin-4-yl]acetate isomer B

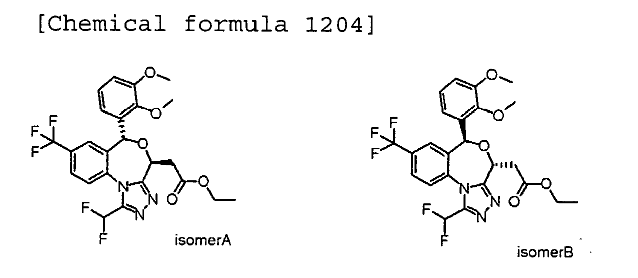

Ethyl 2-[4,6-trans-1-(difluoromethyl)-6-(2,3-dimethoxyphenyl)-8-(trifluoromethyl)-4H,6H-[1,2,4]triazolo[4,3-a][4,1]benzoxazepin-4-yl]acetate was optically resolved by HPLC.
Column: Chiralpak AD (5 × 50cm)
Eluting solution: 50% isopropanol-n-hexane
Flow rate: 50 mL/min
Elution time: isomer A: 31 min., isomer B: 82 min.

### Example 605

2-[(4S,6R)-1-(Difluoromethyl)-6-(2,3-dimethoxyphenyl)-8-(trifluoromethyl)-4H,6H-[1,2,4]triazolo[4,3-a] [4,1]benzoxazepin-4-yl]acetic acid

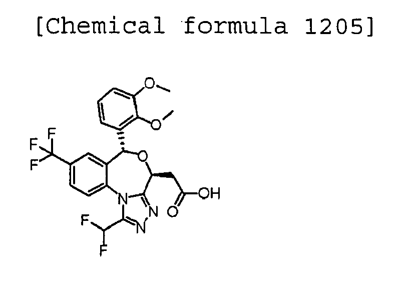

Ethyl 2-[(4S,6R)-1-(difluoromethyl)-6-(2,3-dimethoxyphenyl)-8-(trifluoromethyl)-4H,6H-[1,2,4]triazolo[4,3-a][4,1]benzoxazepin-4-yl]acetate (316 mg) was dissolved in 1,4-dioxane (24 mL). To the resulting solution were added concentrated hydrochloric acid (12 mL) and distilled water (12 mL). The resulting mixture was stirred at 60°C for 14 hours. The reaction mixture was concentrated under reduced pressure. The residue thus obtained was solidified from a methylene chloride-n-hexane solvent mixture to give the title compound (1295 mg).
MS (ESI) m/z : 500 (M + H)⁺.
¹H-NMR (CDCl₃) δ: 3.31 (1H, dd, J = 17.0, 6.7 Hz), 3.37 (3H, s), 3.58 (1H, dd, J = 17.0, 6.7 Hz), 3.85 (3H, s), 4.93 (1H, t, J = 6.7 Hz), 5.65 (1H, s), 6.98-7.24 (5H, m), 7.82 (1H, dd, J = 8.5, 1.8 Hz), 7.89 (1H, d, J = 8.1 Hz). Elemental analysis for: C₂₂H₁₈F₅N₃O₅·0.5H₂O·0.1 n-Hexane Calculated: C, 52.50; H, 3.98; N, 8.13.
Found: C, 52.37; H, 3.94; N, 7.83.

### Example 606

2-[(4R,6S)-1-(Difluoromethyl)-6-(2,3-dimethoxyphenyl)-8-(trifluoromethyl)-4H,6H-[1,2,4]triazolo[4,3-a][4,1]benzoxazepin-4-yl]acetic acid

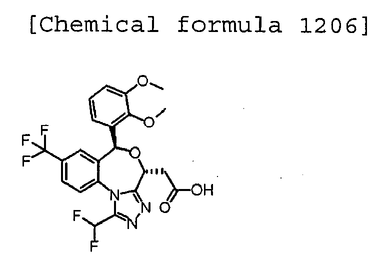

Ethyl 2-[(4R,6S)-1-(difluoromethyl)-6-(2,3-dimethoxyphenyl)-8-(trifluoromethyl)-4H,6H-[1,2,4]triazolo[4,3-a][4,1]benzoxazepin-4-yl]acetate (308 mg) was dissolved in 1,4-dioxane (24 mL). To the resulting solution were added concentrated hydrochloric acid (12 mL) and distilled water (12 mL). The resulting mixture was stirred at 60°C for 15 hours. The reaction mixture was concentrated under reduced pressure. The residue thus obtained was solidified from a methylene chloride-n-hexane solvent mixture to give the title compound (270 mg).
MS (ESI) m/z : 500 (M + H)⁺.
¹H-NMR (CDCl₃) δ: 3.31 (1H, dd, J = 17.0, 6.7 Hz), 3.37 (3H, s), 3.58 (1H, dd, J = 17.0, 6.7 Hz), 3.85 (3H, s), 4.93 (1H, t, J = 6.7 Hz), 5.65 (1H, s), 6.98-7.24 (5H, m), 7.82 (1H, dd, J = 8.6, 1.5 Hz), 7.89 (1H, d, J = 8.3 Hz). Elemental analysis for: C22H18F5N305.·1.25H20·0.1 n-Hexane Calculated: C, 51.17; H, 4.16; N, 7.92.
Found: C, 51.37; H, 3.86; N, 7.60.

### Example 607

Ethyl 2-[(4R,6S)-8-chloro-1-(1,1-difluorcethyl)-6-(2,3-dimethoxyphenyl)-4H,6H-[1,2,4]triazolo[4,3-a] [4,1]benzoxazepin-4-yl]acetate

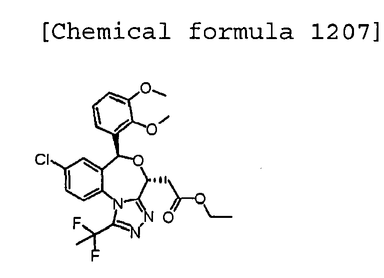

Ethyl 2-[(3R,5S)-5-(2,3-dimethoxyphenyl)-2-thioxo-7-(trifluoromethyl)-1,2,3,5-tetrahydro-4,1-benzoxazepin-3-yl]acetate (654 mg) was dissolved in tetrahydrofuran (10 mL). To the resulting solution was added hydrazine monohydrate (0.11 mL). The resulting mixture was stirred at room temperature for 2.5 hours. The solvent was distilled off and the residue thus obtained was dissolved in methylene chloride (20 mL). To the resulting solution were added 2,2-difluoropropionic acid (248 mg), 1-ethyl-3-(3-dimethylaminopropyl)carbodiimide hydrochloride (575 mg), and 1-hydroxybenzotriazole monohydrate (230 mg). The resulting mixture was stirred at room temperature for 12.5 hours. The reaction mixture was diluted with ethyl acetate and washed successively with a saturated aqueous solution of sodium bicarbonate and saturated brine. The organic layer was dried over anhydrous sodium sulfate and concentrated. Acetic acid (20 mL) was added to the residue and the mixture was heated under reflux for 5.5 hours. The reaction mixture was diluted with chloroform and washed successively with a saturated aqueous solution of sodium bicarbonate and saturated brine. The organic layer was dried over anhydrous sodium sulfate and concentrated. The residue was purified by silica gel chromatography (Automatic purification system of Yamazen Corporation, ethyl acetate:n-hexane=1:1 Rf=0.30) to give the title compound (229 mg).
MS (ESI) m/z : 508 (M + H)⁺.
¹H-NMR (CDCl₃) δ: 1.28 (3H, t, J = 7.2 Hz), 2.32 (3H, t, J = 19.2 Hz), 3.23 (1H, dd, J = 16.6, 6.8 Hz), 3.39 (3H, s), 3.46 (1H, dd, J = 16.6, 6.8 Hz), 3.85 (3H, s), 4.16-4.22 (2H, m), 4.90 (1H, t, J = 6.8 Hz), 5.58 (1H, s), 6.81 (1H, d, J = 2.3 Hz), 6.97 (1H, dd, J = 7.7, 1.8 Hz), 7.16-7.23 (2H, m), 7.48 (1H, dd, J = 8.6, 2.3 Hz), 7.71 (1H, d, J = 8.6 Hz).

### Example 608

2-[(4R,6S)-8-Chloro-1-(1,1-difluoroethyl)-6-(2,3-dimethoxyphenyl)-4H,6H-[1,2,4]triazolo[4,3-a] [4,1]benzoxazepin-4-yl]acetic acid

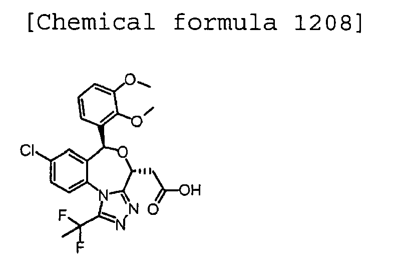

Ethyl 2-[(4R,6S)-8-chloro-1-(1,1-difluorcethyl)-6-(2,3-dimethoxyphenyl)-4H,6H-[1,2,4]triazolo[4,3-a][4,1]benzoxazepin-4-yl]acetate (229 mg) was dissolved in 1,4-dioxane (20 mL). To the resulting solution were added concentrated hydrochloric acid (10 mL) and distilled water (10 mL). The resulting mixture was stirred at 60°C for 12 hours. The reaction mixture was concentrated under reduced pressure. The residue thus obtained was solidified from a methylene chloride-n-hexane solvent mixture to give the title compound (199 mg).
MS (ESI) m/z : 480 (M + H)⁺.
¹H-NMR (CDCl₃) δ: 2.32 (3H, t, J = 19.2 Hz), 3.29 (1H, dd, J = 16.9, 6.7 Hz), 3.39 (3H, s), 3.54 (1H, dd, J = 16.9, 6.7 Hz), 3.85 (3H, s), 4.87 (1H, t, J = 6.7 Hz), 5.57 (1H, s), 6.83 (1H, d, J = 2.4 Hz), 6.97 (1H, dd, J = 7.8, 1.7 Hz), 7.16-7.24 (2H, m), 7.49 (1H, dd, J = 8.7, 2.3 Hz), 7.71 (1H, d, J = 8.5 Hz).
Elemental analysis for: C₂₂H₂₀ClF₂N₃O₅·1.75 H₂O
Calculated: C, 51.67; H, 4.63; N, 8.22.
Found: C, 51.04; H, 4.51; N, 8.71.

### Referential Example 407

(2-Amino-5-chlorophenyl)(2-hydroxy-3-methoxyphenyl)methanone

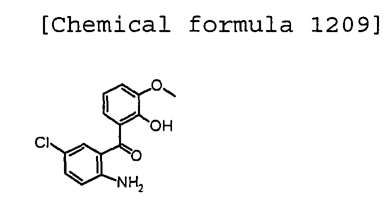

(2-Amino-5-chlorophenyl)(2,3-dimethoxyphenyl)methanone (10.00 g) was dissolved in acetonitrile (100 mL). To the resulting solution were added cerium(III) chloride heptahydrate (19.16 g) and sodium iodide (7.71 g). The resulting mixture was heated under reflux for 3 days. The reaction mixture was diluted with ethyl acetate, washed with saturated brine and dried over anhydrous sodium sulfate. The solvent was distilled off and the residue thus obtained was purified by silica gel chromatography (ethyl acetate:n-hexane=1:3 → 1:0) to give the title compound (1.65 g).
MS (ESI) m/z : 278 (M + H)⁺.
¹H-NMR (CDCl₃) δ: 3.95 (3H, s), 5.43 (2H, br s), 6.70 (1H, d, J = 8.8 Hz), 6.87 (1H, t, J = 8.1 Hz), 7.06-7.12 (2H, m), 7.24 (1H, dd, J = 8.8, 2.4 Hz), 7.37 (1H, d, J = 2.4 Hz), 10.69 (1H, s).

### Referential Example 408

(2-Amino-5-chlorophenyl)(2-ethoxy-3-methoxyphenyl) methanone

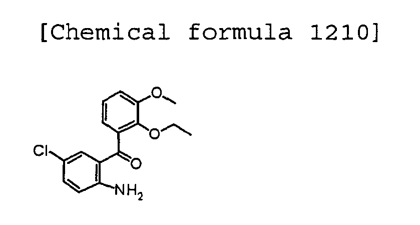

(2-Amino-5-chlorophenyl)(2-hydroxy-3-methoxyphenyl)methanone (1.65 g) was dissolved in N,N-dimethylformamide (20 mL). To the resulting solution were added ethyl iodide (1.30 mL) and potassium carbonate (2.25 g). The resulting mixture was stirred at 60°C for 16 hours. The reaction mixture was diluted with ethyl acetate, washed with saturated brine and dried over anhydrous sodium sulfate. The solvent was distilled off to give the title compound (1.86 g).
MS (ESI) m/z : 278 (M + H)⁺.
¹H-NMR (CDCl₃) δ: 1.13 (3H, t, J = 7.0 Hz), 3.90 (3H, s), 4.00 (2H, q, J = 7.0 Hz), 6.34 (2H, br s), 6.65 (1H, d, J = 9.0 Hz), 6.83 (1H, dd, J = 7.7, 1.5 Hz), 7.02 (1H, dd, J = 8.2, 1.6 Hz), 7.11 (1H, t, J = 7.7 Hz), 7.18-7.25 (2H, m).

### Referential Example 409

(2-Amino-5-chlorophenyl)(2-ethoxy-3-methoxyphenyl)methanol

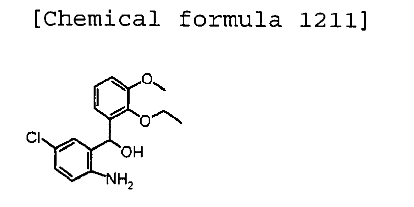

(2-Amino-5-chlorophenyl)(2-ethoxy-3-methoxyphenyl)methanone (1.86 g) was dissolved in ethanol (20 mL). To the resulting solution was added sodium borohydride (1.39 g). The resulting mixture was stirred at room temperature for 3.8 hours. To the reaction mixture was added saturated brine. The resulting mixture was extracted with ethyl acetate. The organic layer was dried over anhydrous sodium sulfate. The solvent was distilled off under reduced pressure to give the title compound (1.84 g).
MS (ESI) m/z : 308 (M + H)⁺.
¹H-NMR (CDCl₃) δ: 1.33 (3H, t, J = 7.1 Hz), 3.15 (1H, d, J = 4.9 Hz), 3.87 (3H, s), 4.04-4.17 (2H, m), 4.22 (2H, br s), 6.04-6.07 (1H, m), 6.58 (1H, d, J = 8.5 Hz), 6.84 (1H, dd, J = 7.8, 1.5 Hz), 6.89 (1H, dd, J = 8.1, 1.5 Hz), 7.01-7.06 (2H, m), 7.11 (1H, d, J = 2.4 Hz).

### Referential Example 410

{5-Chloro-2-[(2,4-dimethoxybenzyl)amino]phenyl}(2-ethoxy-3-methoxyphenyl)methanol

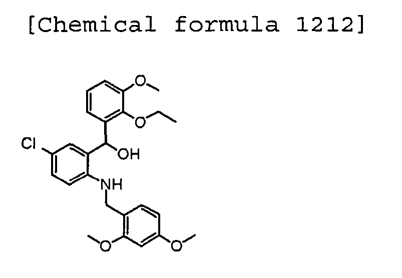

(2-Amino-5-chlorophenyl)(2-ethoxy-3-methoxyphenyl)methanol (1.84 g) and 2,4-dimethoxybenzaldehyde (1.09 g) were dissolved in acetic acid (50 mL). The resulting solution was stirred at room temperature for 15 minutes. Under ice cooling, sodium borohydride (0.68 g) was added and the resulting mixture was stirred at room temperature for 30 minutes. After concentration of the reaction mixture, the residue was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.42) to give the title compound (1.61 g).
MS m/z : 440(M-OH⁺).
¹H-NMR (CDCl₃) δ: 1.24 (3H, t, J = 7.2 Hz), 3.70 (2H, s), 3.77-3.80 (6H, m), 3.87 (3H, s), 4.24 (2H, s), 5.31 (1H, br s), 6.04 (1H, s), 6.37 (1H, dd, J = 8.3, 2.2 Hz), 6.44 (1H, d, J = 2.2 Hz), 6.56 (1H, d, J = 8.5 Hz), 6.80-6.83 (1H, m), 6.88-6.91 (1H, m), 6.98-7.08 (4H, m).

### Referential Example 411

Ethyl 2-[3,5-trans-7-chloro-1-(2,4-dimethoxybenzyl)-5-(2-ethoxy-3-methoxyphenyl)-2-oxo-1,2,3,5-tetrahydro-4,1-benzoxazepin-3-yl]acetate

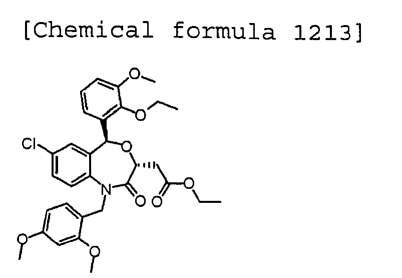

{5-Chloro-2-[(2,4-dimethoxybenzyl)amino]phenyl}(2-ethoxy-3-methoxyphenyl) methanol (1610 mg) was dissolved in dichloromethane (20 mL). Sodium hydrogen carbonate (443 mg) and monoethyl chlorofumarate (743 mg) prepared separately were added to the resulting solution at 0°C.
The resulting mixture was stirred at room temperature for 3.5 hours. The reaction mixture was diluted with dichloromethane and washed with a saturated aqueous solution of sodium bicarbonate and saturated brine. The organic layer was dried over anhydrous sodium sulfate. The filtrate was concentrated.
The residue thus obtained was dissolved in ethanol (20 mL). To the resulting solution was added 1,8-diazabicycloundec-7-ene (0.79 mL). The resulting mixture was heated under reflux for 4 hours and then, stirred at room temperature for 8 hours. After concentration, the residue was diluted with ethyl acetate and washed successively with a 10% aqueous citric acid solution and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.60) to give the title compound (1142 mg).
MS (ESI) m/z : 584 (M + H)⁺.
¹H-NMR (CDCl₃) δ: 1.25 (3H, t, J = 7.1 Hz), 2.79 (1H, dd, J = 16.6, 5.6 Hz), 3.11 (1H, dd, J = 16.6, 7.8 Hz), 3.70 (3H, s), 3.78 (3H, s), 3.85 (3H, s), 4.14 (2H, q, J = 7.1 Hz), 4.53 (1H, dd, J = 7.8, 5.6 Hz), 5.12 (2H, s), 6.04 (1H, s), 6.42-6.46 (2H, m), 6.61 (1H, d, J = 2.0 Hz), 6.93 (1H, dd, J = 7.8, 2.0 Hz), 7.12-7.18 (2H, m), 7.24-7.25 (3H, m).

### Referential Example 412

Ethyl 2-[3,5-trans-7-chloro-5-(2-ethoxy-3-methoxyphenyl)-2-oxo-1,2,3,5-tetrahydro-4,1-benzoxazepin-3-yl]acetate

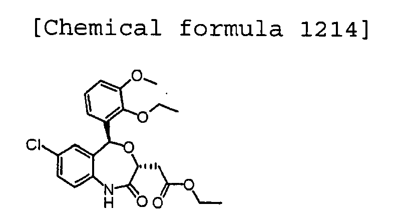

Ethyl 2-[3,5-trans-7-chloro-1-(2,4-dimethoxybenzyl)-5-(2-ethoxy-3-methoxyphenyl)-2-oxo-1,2,3,5-tetrahydro-4,1-benzoxazepin-3-yl]acetate (1.14 g) was dissolved in acetone (10 mL). To the resulting solution was added an aqueous solution (2 mL) of ceric(IV) ammonium nitrate (3.75 g) at 0°C. The resulting mixture was stirred at room temperature for 2.2 hours. To the reaction mixture was added an aqueous solution (10 mL) of ceric(IV) ammonium nitrate (1.61 g) at 0°C and the resulting mixture was stirred at room temperature for 1.7 hours. To the reaction mixture was added a saturated aqueous solution of sodium bicarbonate and the resulting mixture was extracted with ethyl acetate. The extract was washed with saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.41) to give the title compound (493 mg).
MS (ESI) m/z : 434 (M + H)⁺.
¹H-NMR (CDCl₃) δ: 1.07 (3H, t, J = 7.1 Hz), 1.20-1.28 (3H, m), 2.78 (1H, dd, J = 16.5, 6.6 Hz), 3.03 (1H, dd, J = 16.5, 6.6 Hz), 3.75-3.80 (1H, m), 3.87 (3H, s), 3.96-4.01 (1H, m), 4.09-4.14 (2H, m), 4.63 (1H, t, J = 6.6 Hz), 6.25 (1H, s), 6.70 (1H, d, J = 2.4 Hz), 6.95-7.00 (2H, m), 7.07 (1H, dd, J = 7.9, 1.7 Hz), 7.14 (1H, t, J = 7.9 Hz), 7.27-7.30 (1H, m), 7.64 (1H, br s).

### Referential Example 413

Ethyl 2-[3,5-trans-7-chloro-5-(2-ethoxy-3-methoxyphenyl)-2-thioxo-1,2,3,5-tetrahydro-4,1-benzoxazepin-3-yl]acetate

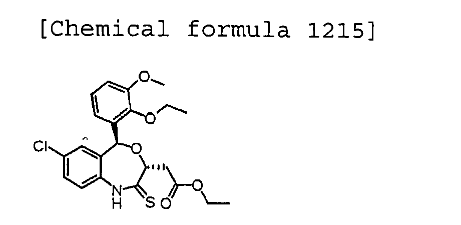

Ethyl 2-[3,5-trans-7-chloro-5-(2-ethoxy-3-methoxyphenyl)-2-oxo-1,2,3,5-tetrahydro-4,1-benzoxazepin-3-yl]acetate (493 mg) was dissolved in tetrahydrofuran (5 mL). To the resulting solution were added sodium hydrogen carbonate (572 mg) and diphosphorus pentasulfide (1.26 g). The resulting mixture was stirred at room temperature for 2 hours. The reaction mixture was diluted with ethyl acetate and washed successively with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.60) to give the title compound (461 mg).
MS (ESI) m/z : 450 (M + H)⁺.
¹H-NMR (CDCl₃) δ: 1.04 (3H, t, J = 7.1 Hz), 1.24 (3H, t, J = 7.1 Hz), 2.94 (1H, dd, J = 16.3, 6.3 Hz), 3.26 (1H, dd, J = 16.5, 7.0 Hz), 3.71-3.79 (1H, m), 3.86 (3H, s), 3.95-4.03 (1H, m), 4.10-4.15 (2H, m), 4.70 (1H, t, J = 6.5 Hz), 6.16 (1H, s), 6.69 (1H, d, J = 2.0 Hz), 6.96 (1H, dd, J = 7.6, 2.2 Hz), 7.05 (1H, d, J = 8.5 Hz), 7.10-7.15 (2H, m), 7.33 (1H, dd, J = 8.0, 2.4 Hz), 9.57 (1H, s).

### Example 609

Ethyl 2-[4,6-trans-8-chloro-6-(2-ethoxy-3-methoxyphenyl)-1-(trifluoromethyl)-4H,6H-[1,2,4]triazolo[4,3-a][4,1]benzoxazepin-4-yl]acetate

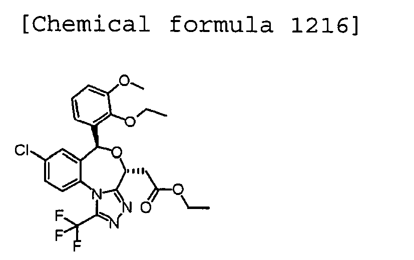

Ethyl 2-[3,5-trans-7-chloro-5-(2-ethoxy-3-methoxyphenyl)-2-thioxo-1,2,3,5-tetrahydro-4,1-benzoxazepin-3-yl]acetate (461 mg) was dissolved in tetrahydrofuran (5 mL). To the resulting solution was added hydrazine monohydrate (0.075 mL). The resulting mixture was stirred at room temperature for 1 hour. Under ice cooling, trifluoroacetic anhydride (0.724 mL) was added and the resulting mixture was stirred at room temperature for 16 hours. The reaction mixture was diluted with ethyl acetate and then washed with a saturated aqueous solution of sodium bicarbonate and saturated brine. The organic layer was dried over sodium sulfate and concentrated under reduced pressure. The residue thus obtained was purified by silica gel chromatography (Automatic purification system of Yamazen Corporation, ethyl acetate:n-hexane=1:2, Rf=0.60) to give the title compound (450 mg).
MS (ESI) m/z : 526 (M + H)⁺.
¹H-NMR (CDCl₃) δ: 0.84 (3H, t, J = 7.1 Hz), 1.28 (3H, t, J = 7.2 Hz), 3.24 (1H, dd, J = 16.7, 6.8 Hz), 3.49 (1H, dd, J = 16.7, 6.8 Hz), 3.64 (1H, dd, J = 9.3, 7.2 Hz), 3.82-3.88 (4H, m), 4.16-4.22 (2H, m), 4.93 (1H, t, J = 6.8 Hz), 5.59 (1H, s), 6.86 (1H, s), 6.95-6.99 (1H, m), 7.17 (1H, t, J = 7.9 Hz), 7.20-7.23 (1H, m), 7.52 (2H, s).

### Example 610

Ethyl 2-[(4S,6R)-8-chloro-6-(2-ethoxy-3-methoxyphenyl)-1-(trifluoromethyl)-4H,6H-[1,2,4]triazolo[4,3-a][4,1]benzoxazepin-4-yl]acetate isomer A
Ethyl 2-[(4R,6S)-8-chloro-6-(2-ethoxy-3-methoxyphenyl)-1-(trifluoromethyl)-4H,6H-[1,2,4]triazolo[4,3-a][4,1]benzoxazepin-4-yl]acetate isomer B

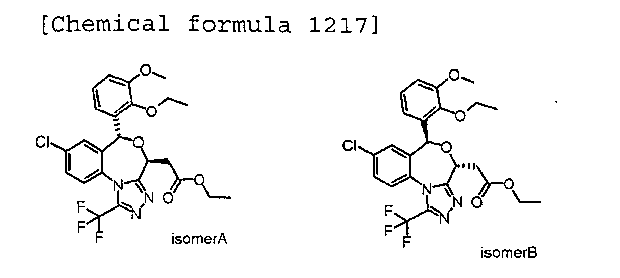

Ethyl 2-[4,6-trans-8-chloro-6-(2-ethoxy-3-methoxyphenyl)-1-(trifluoromethyl)-4H,6H-[1,2,4]triazolo[4,3-a] [4,1]benzoxazepin-4-yl]acetate was optically resolved by HPLC.
Column: Chiralpak AD (5 × 50cm)
Eluting solution: 30% isopropanol-n-hexane
Flow rate: 50 mL/min
Elution time: isomer A: 24 min., isomer B: 91 min.

### Example 611

2-[(4S,6R)-8-Chloro-6-(2-ethoxy-3-methoxyphenyl)-1-(trifluoromethyl)-4H,6H-[1,2,4]triazolo[4,3-a][4,1]benzoxazepin-4-yl]acetic acid

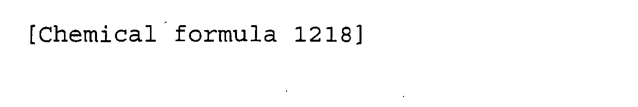

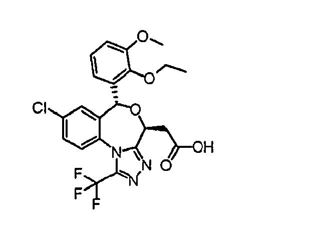

Ethyl 2-[(4S,6R)-8-chloro-6-(2-ethoxy-3-methoxyphenyl)-1-(trifluoromethyl)-4H,6H-[1,2,4]triazolo[4,3-a][4,1]benzoxazepin-4-yl]acetate (206 mg) was dissolved in 1,4-dioxane (16 mL). To the resulting solution were added concentrated hydrochloric acid (8 mL) and distilled water (8 mL). The resulting mixture was stirred at 60°C for 13 hours. The reaction mixture was concentrated under reduced pressure. The residue thus obtained was solidified from a methylene chloride-n-hexane solvent mixture to give the title compound (191 mg).
MS (ESI) m/z : 498 (M + H)⁺.
¹H-NMR (CDCl₃) δ: 0.84 (3H, t, J = 7.1 Hz), 3.31 (1H, dd, J = 17.0, 6.7 Hz), 3.54-3.67 (2H, m), 3.82-3.87 (4H, m), 4.90 (1H, t, J = 6.7 Hz), 5.60 (1H, s), 6.87 (1H, s), 6.98 (1H, dd, J = 8.0, 1.7 Hz), 7.18 (1H, t, J = 7.8 Hz), 7.23 (1H, dd, J = 7.8, 1.7 Hz), 7.51-7.54 (2H, m).
Elemental analysis for: C₂₂H₁₉ClF₃N₃O₅·0.75H₂O·0.15 n-Hexane
Calculated: C, 52.46; H, 4.34; N, 8.01.
Found: C, 52.68; H, 4.23; N, 7.69.

### Example 612

2-[(4R,6S)-8-Chloro-6-(2-ethoxy-3-methoxyphenyl)-1-(trifluoromethyl)-4H,6H-[1,2,4]triazolo[4,3-a] [4,1]benzoxazepin-4-yl]acetic acid

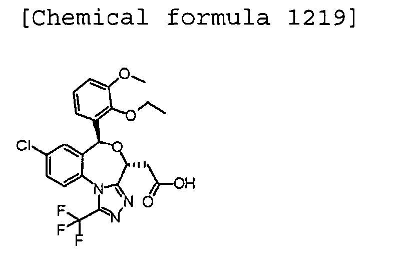

Ethyl 2-[(4R,6S)-8-chloro-6-(2-ethoxy-3-methoxyphenyl)-1-(trifluoromethyl)-4H,6H-[1,2,4]triazolo[4,3-a][4,1]benzoxazepin-4-yl]acetate (223 mg) was dissolved in 1,4-dioxane (16 mL). To the resulting solution were added concentrated hydrochloric acid (8 mL) and distilled water (8 mL). The resulting mixture was stirred at 60°C for 13 hours. The reaction mixture was concentrated under reduced pressure. The residue thus obtained was solidified from a methylene chloride-n-hexane solvent mixture to give the title compound (200 mg).
MS (ESI) m/z : 498 (M + H)⁺.
¹H-NMR (CDCl₃) δ: 0.84 (3H, t, J = 7.1 Hz), 3.31 (1H, dd, J = 17.1, 6.6 Hz), 3.54-3.68 (2H, m), 3.82-3.87 (4H, m), 4.90 (1H, t, J = 6.6 Hz), 5.60 (1H, s), 6.87 (1H, s), 6.98 (1H, dd, J = 8.0, 1.7 Hz), 7.18 (1H, t, J = 7.8 Hz), 7.23 (1H, dd, J = 7.8, 1.7 Hz), 7.50-7.55 (2H, m).
Elemental analysis for: C₂₂H₁₉ClF₃N₃O₅·0.5H₂O·0.15 n-Hexane Calculated: C, 52.92; H, 4.29; N, 8.08.
Found: C, 52.69; H, 4.11; N, 7.77.

### Referential Example 414

tert-Butyl 4-chloro-2-[(2,3-diethoxyphenyl)(hydroxy)methyl]phenylcarbamate

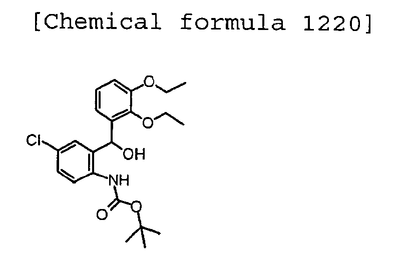

To a tetrahydrofuran solution (60 mL) of tert-butyl 4-chlorophenylcarbamate (6.22 g) was added dropwise 1N sec-butyl lithium (68.30 mL) at -78°C. The reaction mixture was stirred at -25°C for 2.2 hours. Then, 2,3-diethoxybenzaldehyde (5.11 mL) was added at -25°C and the resulting mixture was stirred for 2 hours. To the reaction mixture was added a saturated aqueous solution of ammonium chloride and the layers separated. The water phase was washed with ethyl acetate. The organic layers were combined, dried over anhydrous sodium sulfate, and concentrated under reduced pressure. The residue thus obtained was solidified from n-hexane to give the title compound (7.60 g).
MS (ESI) m/z : 410 (M + H)⁺.
¹H-NMR (CDCl₃) δ: 1.29-1.38 (6H, m), 1.48 (9H, s), 3.63-3.67 (1H, m), 4.06-4.11 (2H, m), 6.03-6.06 (1H, m), 6.77-6.81 (1H, m), 6.87-6.90 (1H, m), 7.02 (1H, t, J = 7.9 Hz), 7.08 (1H, d, J = 2.4 Hz), 7.22 (1H, dd, J = 8.8, 2.4 Hz), 7.87 (1H, d, J = 8.5 Hz), 8.10 (1H, br s).

### Referential Example 415

(2-Amino-5-chlorophenyl)(2,3-diethoxyphenyl)methanol

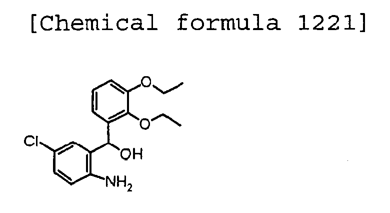

tert-Butyl 4-chloro-2-[(2,3-diethoxyphenyl)(hydroxy)methyl]phenylcarbamate (7.60 g) was dissolved in 1,4-dioxane (30 mL). To the resulting solution was added 8N hydrochloric acid (20 mL). The resulting mixture was stirred at room temperature for 16 hours. The reaction mixture was made alkaline with a saturated aqueous solution of sodium bicarbonate, followed by extraction with chloroform. The organic layer was dried over anhydrous sodium sulfate. The solvent was distilled off to give the title compound (5.90 g).
MS (ESI) m/z : 322 (M + H)⁺.
¹H-NMR (CDCl₃) δ: 1.31-1.37 (3H, m), 1.45-1.50 (3H, m), 4.06-4.11 (2H, m), 6.04 (1H, s), 6.57-6.60 (1H, m), 6.73-7.10 (5H, m).

### Referential Example 416

{5-Chloro-2-[(2,4-dimethoxybenzyl)amino]phenyl}(2,3-diethoxyphenyl) methanol

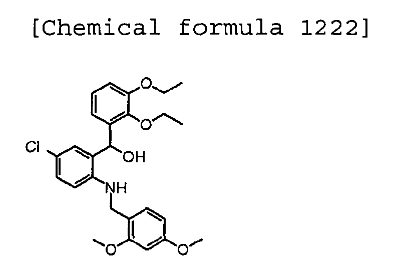

(2-Amino-5-chlorophenyl)(2,3-diethoxyphenyl)methanol (5.80 g) and 2,4-dimethoxybenzaldehyde (3.29 g) were dissolved in acetic acid (50 mL). The resulting solution was stirred at room temperature for 20 minutes. Under ice cooling, sodium borohydride (2.05 g) was added and the resulting mixture was stirred at room temperature for 1.5 hours. After concentration of the reaction mixture, the residue was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.61) to give the title compound (5.67 g).
MS m/z : 472(M+H)⁺.
¹H-NMR (CDCl₃) δ: 1.24 (3H, t, J = 7.0 Hz), 1.46 (3H, t, J = 7.0 Hz), 3.26 (1H, br s), 3.77 (3H, s), 3.78 (3H, s), 4.07 (4H, q, J = 7.0 Hz), 4.24 (2H, s), 5.31 (1H, br s), 6.03 (1H, s), 6.37 (1H, dd, J = 8.3, 2.4 Hz), 6.44 (1H, d, J = 2.4 Hz), 6.56 (1H, d, J = 8.8 Hz), 6.78 (1H, dd, J = 7.9, 1.5 Hz), 6.88 (1H, dd, J = 7.9, 1.5 Hz), 6.98-7.08
(4H, m).

### Referential Example 417

Ethyl 2-[3,5-trans-7-chloro-5-(2,3-diethoxyphenyl)-1-(2,4-dimethoxybenzyl)-2-oxo-1,2,3,5-tetrahydro-4,1-benzoxazepin-3-yl] acetate

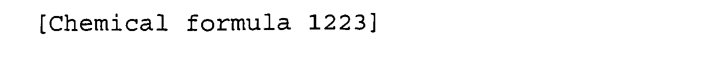

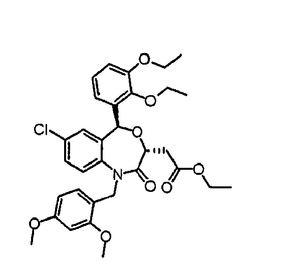

{5-Chloro-2-[(2,4-dimethoxybenzyl)amino]phenyl}(2,3-diethoxyphenyl) methanol (5.67 g) was dissolved in dichloromethane (60 mL). Sodium hydrogen carbonate (1.51 g) and monoethyl chlorofumarate (2.15 g) prepared separately were added to the resulting solution at 0°C. After stirring at room temperature for 13 hours, the reaction mixture was diluted with dichloromethane and washed with a saturated aqueous solution of sodium bicarbonate and saturated brine. The organic layer was dried over anhydrous sodium sulfate. The filtrate was concentrated.
The residue thus obtained was dissolved in ethanol (60 mL). To the resulting solution was added 1,8-diazabicycloundec-7-ene (2.69 mL). The resulting mixture was heated under reflux for 3 hours. After concentration, the residue was diluted with ethyl acetate and washed successively with a 10% aqueous citric acid solution and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:4, Rf=0.40) to give the title compound (8.05 g).
MS (ESI) m/z : 598 (M + H)⁺.
¹H-NMR (CDCl₃) δ: 1.22-1.27 (6H, m), 1.43 (3H, t, J = 7.0 Hz), 2.79 (1H, dd, J = 16.6, 5.6 Hz), 3.12 (1H, dd, J = 16.6, 8.0 Hz), 3.54 (1H, dd, J = 9.7, 6.9 Hz), 3.71 (3H, s), 3.78 (3H, s), 3.87 (1H, dd, J = 9.7, 6.9 Hz), 4.06 (2H, q, J = 7.0 Hz), 4.14 (2H, q, J = 6.9 Hz), 4.53 (1H, dd, J = 8.0, 5.6 Hz), 5.13 (2H, s), 6.04 (1H, s), 6.42-6.47 (2H, m), 6.60-6.61 (1H, m), 6.91-6.94 (1H, m), 7.09-7.18 (2H, m), 7.23-7.26.(1H, m).

### Referential Example 418

Ethyl 2-[3,5-trans-7-chloro-5-(2,3-diethoxyphenyl)-2-oxo-1,2,3,5-tetrahydro-4,1-benzoxazepin-3-yl]acetate

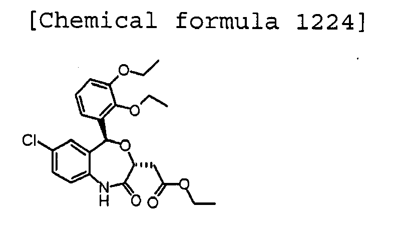

Ethyl 2-[3,5-trans-7-chloro-5-(2,3-diethoxyphenyl)-1-(2,4-dimethoxybenzyl)-2-oxo-1,2,3,5-tetrahydro-4,1-benzoxazepin-3-yl]acetate (8.02 g) was dissolved in acetone (80 mL). To the resulting solution was added an aqueous solution (16 mL) of ceric(IV) ammonium nitrate (25.74 g) at 0°C. The resulting mixture was stirred at room temperature for 2.3 hours. Ceric(IV) ammonium nitrate (11.03 g) was then added at 0°C and the resulting mixture was stirred at room temperature for 3 hours. Ceric(IV) ammonium nitrate (11.03 g) was added again and the resulting mixture was stirred at room temperature for one hour. To the reaction mixture was added a saturated aqueous solution of sodium bicarbonate. The resulting mixture was extracted with ethyl acetate. The extract was washed with saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.45) to give the title compound (2.07 g).
MS (ESI) m/z : 448 (M + H)⁺.
¹H-NMR (CDCl₃) δ: 1.08 (3H, t, J = 7.0 Hz), 1.22 (3H, t, J = 7.1 Hz), 1.45 (3H, t, J = 7.0 Hz), 2.78 (1H, dd, J = 16.4, 6.6 Hz), 3.03 (1H, dd, J = 16.4, 6.6 Hz), 3.78-3.84 (1H, m), 3.97-4.14 (5H, m), 4.63 (1H, t, J = 6.6 Hz), 6.24 (1H, s), 6.69 (1H, d, J = 2.5 Hz), 6.94 (1H, dd, J = 8.2, 1.7 Hz), 6.98 (1H, d, J = 8.2 Hz), 7.05 (1H, dd, J = 7.8, 1.7 Hz), 7.11 (1H, t, J = 7.8 Hz), 7.28-7.29 (1H, m), 7.71 (1H, br s).

### Referential Example 419

Ethyl 2-[3,5-trans-7-chloro-5-(2,3-diethoxyphenyl)-2-thioxo-1,2,3,5-tetrahydro-4,1-benzoxazepin-3-yl]acetate

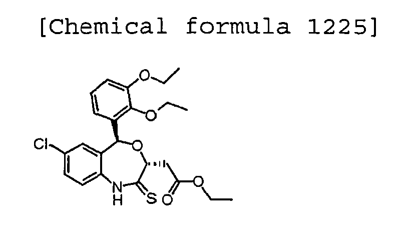

Ethyl 2-[3,5-trans-7-chloro-5-(2,3-diethoxyphenyl)-2-oxo-1,2,3,5-tetrahydro-4,1-benzoxazepin-3-yl]acetate (2.07 g) was dissolved in tetrahydrofuran (20 mL). To the resulting solution were added sodium hydrogen carbonate (2.33 g) and diphosphorus pentasulfide (5.14 g). The resulting mixture was stirred at room temperature for one hour. The reaction mixture was diluted with ethyl acetate and washed successively with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:4, Rf=0.40) to give the title compound (1.82 g).
MS (ESI) m/z : 464 (M + H)⁺.
¹H-NMR (CDCl₃) δ: 1.05 (3H, t, J = 7.0 Hz), 1.24 (3H, t, J = 7.1 Hz), 1.44 (3H, t, J = 7.1 Hz), 2.95 (1H, dd, J = 16.5, 6.6 Hz), 3.26 (1H, dd, J = 16.5, 6.6 Hz), 3.74-3.82 (1H, m), 4.00-4.15 (5H, m), 4.69 (1H, t, J = 6.6 Hz), 6.16 (1H, s), 6.68 (1H, d, J = 2.2 Hz), 6.94 (1H, dd, J = 6.8, 2.9 Hz), 7.05 (1H, d, J = 8.3 Hz), 7.09-7.13 (2H, m), 7.33 (1H, dd, J = 8.3, 2.4 Hz), 9.55 (1H, br s).

### Example 613

Ethyl 2-[4,6-trans-8-chloro-6-(2,3-diethoxyphenyl)-1-(trifluoromethyl)-4H,6H-[1,2,4]triazolo[4,3-a][4,1]benzoxazepin-4-yl]acetate

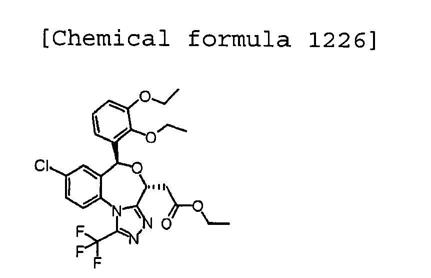

Ethyl 2-[3,5-trans-7-chloro-5-(2,3-diethoxyphenyl)-2-thioxo-1,2,3,5-tetrahydro-4,1-benzoxazepin-3-yl]acetate (900 mg) was dissolved in tetrahydrofuran (10 mL). To the resulting solution was added hydrazine monohydrate (0.141 mL). The resulting mixture was stirred at room temperature for 1.3 hours. Under ice cooling, trifluoroacetic anhydride (1.37 mL) was added and the resulting mixture was stirred at room temperature for 14 hours. The reaction mixture was diluted with ethyl acetate and then washed with a saturated aqueous solution of sodium bicarbonate and saturated brine. The organic layer was dried over sodium sulfate and concentrated under reduced pressure. The residue thus obtained was purified by silica gel chromatography (Automatic purification system of Yamazen Corporation, ethyl acetate:n-hexane=1:2, Rf=0.60) to give the title compound (915 mg).
MS (ESI) m/z : 540 (M + H)⁺.
¹H-NMR (CDCl₃) δ: 0.85 (3H, t, J = 7.1 Hz), 1.28 (3H, t, J = 7.1 Hz), 1.41 (3H, t, J = 7.0 Hz), 3.24 (1H, dd, J = 16.7, 6.8 Hz), 3.49 (1H, dd, J = 16.7, 6.8 Hz), 3.65-3.73 (1H, m), 3.81-3.89 (1H, m), 4.05 (2H, q, J = 7.0 Hz), 4.16-4.22 (2H, m), 4.92 (1H, t, J = 6.8 Hz), 5.58 (1H, s), 6.84-6.86 (1H, m), 6.95 (1H, dd, J = 8.1, 1.5 Hz), 7.14 (1H, t, J = 7.9 Hz), 7.20 (1H, d, J = 7.6 Hz), 7.50-7.54 (2H, m).

### Example 614

Ethyl 2-[(4S,6R)-8-chloro-6-(2,3-diethoxyphenyl)-1-(trifluoromethyl)-4H,6H-[1,2,4]triazolo[4,3-a] [4,1]benzoxazepin-4-yl]acetate isomer A Ethyl 2-[(4R,6S)-8-chloro-6-(2,3-diethoxyphenyl)-1-(trifluoromethyl)-4H,6H-[1,2,4]triazolo[4,3-a] [4,1]benzoxazepin-4-yl]acetate isomer B

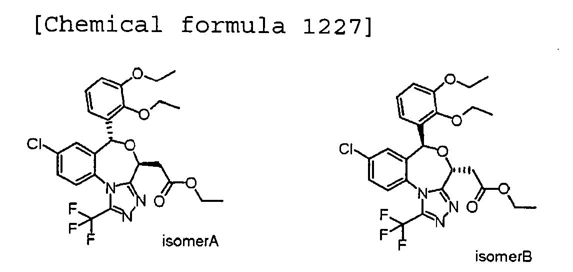

Ethyl 2-[4,6-trans-8-chloro-6-(2,3-diethoxyphenyl)-1-(trifluoromethyl)-4H,6H-[1,2,4]triazolo[4,3-a][4,1]benzoxazepin-4-yl]acetate was optically resolved by HPLC.
Column: Chiralpak AD (5 × 50cm)
Eluting solution: 30% isopropanol-n-hexane
Flow rate: 50 mL/min
Elution time: isomer A: 22 min., isomer B: 80 min.

### Example 615

2-[(4S,6R)-8-Chloro-6-(2,3-diethoxyphenyl)-1-(trifluoromethyl)-4H,6H-[1,2,4]triazolo[4,3-a][4,1]benzoxazepin-4-yl]acetic acid

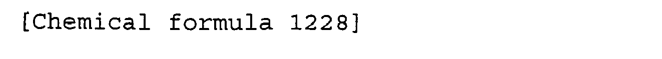

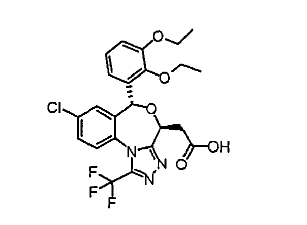

Ethyl 2-[(4S,6R)-8-chloro-6-(2,3-diethoxyphenyl)-1-(trifluoromethyl)-4H,6H-[1,2,4]triazolo[4,3-a] [4,1]benzoxazepin-4-yl]acetate (453 mg) was dissolved in 1,4-dioxane (32 mL). To the resulting solution were added concentrated hydrochloric acid (16 mL) and distilled water (16 mL). The resulting mixture was stirred at 60°C for 13 hours. The reaction mixture was concentrated under reduced pressure. The residue thus obtained was solidified from a methylene chloride-n-hexane solvent mixture to give the title compound (393 mg).
MS (ESI) m/z : 512 (M + H)⁺.
¹H-NMR (CDCl₃) δ: 0.85 (3H, t, J = 7.1 Hz), 1.41 (3H, t, J = 7.0 Hz), 3.31 (1H, dd, J = 17.0, 6.7 Hz), 3.57 (1H, dd, J = 17.0, 6.7 Hz), 3.67-3.73 (1H, m), 3.84-3.90 (1H, m), 4.05 (2H, q, J = 7.0 Hz), 4.90 (1H, t, J = 6.7 Hz), 5.59 (1H, s), 6.87 (1H, s), 6.94-6.98 (1H, m), 7.15 (1H, t, J = 8.1 Hz), 7.20-7.23 (1H, m), 7.51-7.53 (2H, m).
Elemental analysis for: C₂₃H₂₁ClF₃N₃O₅·0.5H₂O
Calculated: C, 53.03; H, 4.26; N, 8.07. Found: C, 53.06; H, 4.21; N, 7.90.

### Example 616

2-[(4R,6S)-8-Chloro-6-(2,3-diethoxyphenyl)-1-(trifluoromethyl)-4H,6H-[1,2,4]triazolo[4,3-a] [4,1]benzoxazepin-4-yl]acetic acid

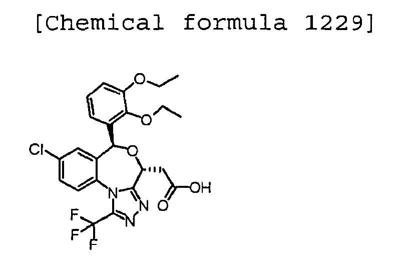

Ethyl 2-[(4R,6S)-8-chloro-6-(2,3-diethoxyphenyl)-1-(trifluoromethyl)-4H,6H-[1,2,4]triazolo[4,3-a][4,1]benzoxazepin-4-yl]acetate (460 mg) was dissolved in 1,4-dioxane (32 mL). To the resulting solution were added concentrated hydrochloric acid (16 mL) and distilled water (16 mL). The resulting mixture was stirred at 60°C for 13 hours. The reaction mixture was concentrated under reduced pressure. The residue thus obtained was solidified from a methylene chloride-n-hexane solvent mixture to give the title compound (401 mg).
MS (ESI) m/z : 512 (M + H)⁺.
¹H-NMR (CDCl₃) δ: 0.85 (3H, t, J = 7.1 Hz), 1.41 (3H, t, J = 7.0 Hz), 3.31 (1H, dd, J = 17.0, 6.7 Hz), 3.57 (1H, dd, J = 17.0, 6.7 Hz), 3.67-3.72 (1H, m), 3.82-3.90 (1H, m), 4.05 (2H, q, J = 7.0 Hz), 4.90 (1H, t, J = 6.7 Hz), 5.59 (1H, s), 6.87 (1H, s), 6.95-6.98 (1H, m), 7.15 (1H, t, J = 7.9 Hz), 7.20-7.23 (1H, m), 7.52=7.53 (2H, m).
Elemental analysis for: C₂₃H₂₁ClF₃N₃O₅·0.5H₂O
Calculated: C, 53.03; H, 4.26; N, 8.07.
Found: C, 52.85; H, 4.36; N, 7.82.

### Referential Example 420

tert-Butyl 4-chloro-2-[[2-(difluoromethoxy)-3-methoxyphenyl](hydroxy)methyl]phenylcarbamate

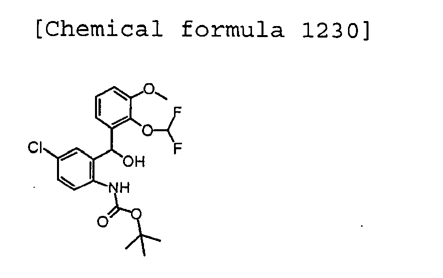

To a tetrahydrofuran solution (60 mL) of tert-butyl 4-chlorophenylcarbamate (5.65 g) was added dropwise 1N sec-butyl lithium (62.04 mL) at -78°C. The reaction mixture was stirred at -25°C for 2.2 hours. At -25°C, 2-difluoromethoxy-3-methoxybenzaldehyde (5.02 g) was added and the resulting mixture was stirred for 1.8 hours. To the reaction mixture was added a saturated aqueous solution of ammonium chloride and the layers separated. The water phase was washed with ethyl acetate. The organic layers were combined, dried over anhydrous sodium sulfate and concentrated under reduced pressure. The residue thus obtained was solidified from n-hexane to give the title compound (8.60 g).
MS (ESI) m/z : 430 (M + H)⁺.
¹H-NMR (CDCl₃) δ: 1.50 (9H, s), 3.92 (3H, s), 6.26 (1H, d, J = 3.4 Hz), 6.74 (1H, dd, J = 77.5, 74.8 Hz), 6.98 (1H, dd, J = 8.3, 1.5 Hz), 7.06 (1H, dd, J = 7.8, 1.2 Hz), 7.15 (1H, d, J = 2.4 Hz), 7.22-7.27 (2H, m), 7.48 (1H, br s), 7.74 (1H, d, J = 8.5 Hz).

### Referential Example 421

(2-Amino-5-chlorophenyl)[2-(difluoromethoxy)-3-methoxyphenyl]methanol

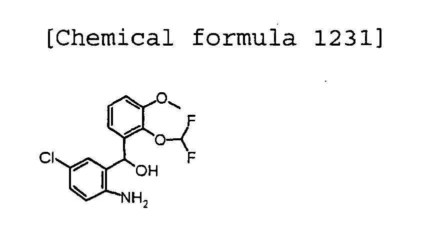

tert-Butyl 4-chloro-2-[[2-(difluoromethoxy)-3-methoxyphenyl](hydroxy)methyl]phenylcarbamate (8.76 g) was dissolved in 1,4-dioxane (50 mL). To the resulting solution was added 8N hydrochloric acid (30 mL). The resulting mixture was stirred at room temperature for 4 days. The reaction mixture was made alkaline with a concentrated aqueous solution of sodium hydroxide, followed by extraction with methylene chloride. The organic layer was dried over anhydrous sodium sulfate. The solvent was distilled off to give the title compound (6.61 g).
MS (ESI) m/z : 330 (M + H)⁺.
¹H-NMR (CDCl₃) δ: 3.90 (3H, s), 6.20 (2H, s), 6.54-6.60 (2H, m), 6.92-7.00 (3H, m), 7.05 (1H, dd, J = 8.3, 2.4 Hz), 7.14 (1H, d, J = 2.4 Hz), 7.20 (1H, t, J = 8.1 Hz).

### Referential Example 422

{5-Chloro-2-[(2,4-dimethoxybenzyl)amino]phenyl}[2-(difluoromethoxy)-3-methoxyphenyl] methanol

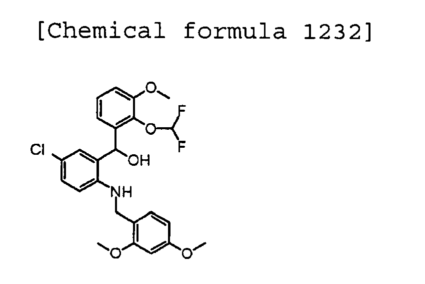

(2-Amino-5-chlorophenyl)[2-(difluoromethoxy)-3-methoxyphenyl]methanol (6.61 g) and 2,4-dimethoxybenzaldehyde (3.66 g) were dissolved in acetic acid (70 mL). The resulting solution was stirred at room temperature for 1 hour. Under ice cooling, sodium borohydride (2.28 g) was added and the resulting mixture was stirred at room temperature for 12 hours. After concentration of the reaction mixture, the residue was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.40) to give the title compound (6.13 g).
¹H-NMR (CDCl₃) δ: 3.78 (3H, s), 3.78 (3H, s), 3.89 (3H, s), 4.23 (2H, s), 6.20 (1H, s), 6.37 (1H, dd, J = 8.3, 2.5 Hz), 6.44 (1H, d, J = 2.5 Hz), 6.57-6.61 (2H, m), 6.68 (1H, dd, J = 77.2, 75.0 Hz), 6.95 (2H, d, J = 8.1 Hz), 7.02 (1H, d, J = 8.1 Hz), 7.07-7.10 (2H, m), 7.19 (1H, t, J = 8.1 Hz).

### Referential Example 423

Ethyl 2-[3,5-trans-7-chloro-5-[2-(difluoromethoxy)-3-methoxyphenyl]-1-(2,4-dimethoxybenzyl)-2-oxo-1,2,3,5-tetrahydro-4,1-benzoxazepin-3-yl]acetate

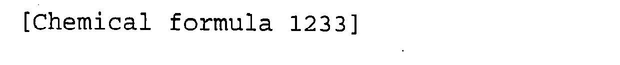

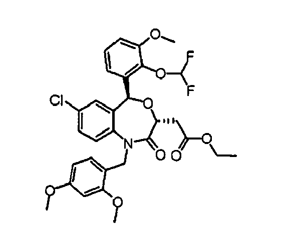

{5-Chloro-2-[(2,4-dimethoxybenzyl)amino]phenyl}[2-(difluoromethoxy)-3-methoxyphenyl] methanol (6.13 g) was dissolved in ethyl acetate (100 mL). Sodium hydrogen carbonate (2.41 g) and monoethyl chlorofumarate (3.03 g) prepared separately were added to the resulting solution at 0°C. The reaction mixture was stirred at room temperature for 20 minutes and washed with saturated brine. The organic layer was dried over anhydrous sodium sulfate. The filtrate was concentrated.
The residue thus obtained was dissolved in ethanol (100 mL). To the resulting solution was added 1,8-diazabicycloundec-7-ene (4.28 mL). The resulting mixture was stirred at 60°C for 21 hours and then heated under reflux for 2 hours. The reaction mixture was concentrated. The residue was diluted with ethyl acetate and washed successively with a 10% aqueous citric acid solution and saturated brine. The organic layer was dried over anhydrous sodium sulfate and concentrated to give the title compound(8.22 g).
MS (ESI) m/z : 606 (M + H)⁺.
¹H-NMR (CDCl₃) δ: 1.25 (3H, t, J = 7.1 Hz), 2.80 (1H, dd, J = 16.6, 6.1 Hz), 3.10 (1H, dd, J = 16.6, 7.6 Hz), 3.72 (3H, s), 3.77 (3H, s), 3.89 (3H, s), 4.14 (2H, q, J = 7.1 Hz), 4.53 (1H, dd, J = 7.6, 6.1 Hz), 5.07 (1H, d, J = 15.5 Hz), 5.22 (1H, d, J = 15.5 Hz), 6.07 (1H, s), 6.34-6.54 (4H, m), 7.01 (1H, dd, J = 7.9, 1.8 Hz), 7.20-7.33 (5H, m).

### Referential Example 424

Ethyl 2-{3,5-trans-7-chloro-5-[2-(difluoromethoxy)-3-methoxyphenyl]-2-oxo-1,2,3,5-tetrahydro-4,1-benzoxazepin-3-yl}acetate

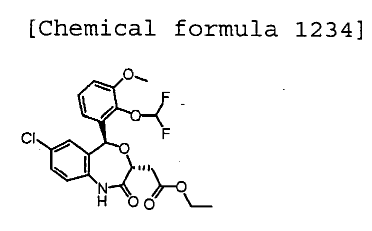

Ethyl 2-[3,5-trans-7-chloro-5-[2-(difluoromethoxy)-3-methoxyphenyl]-1-(2,4-dimethoxybenzyl)-2-oxo-1,2,3,5-tetrahydro-4,1-benzoxazepin-3-yl]acetate (8.22 g) was dissolved in acetone (80 mL). To the resulting solution was added an aqueous solution (20 mL) of ceric(IV) ammonium nitrate (30.14 g) at 0°C. The reaction mixture was stirred at room temperature for one hour. A saturated aqueous solution of sodium bicarbonate was added and the resulting mixture was extracted with ethyl acetate. The extract was washed with saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:2, Rf=0.31) to give the title compound (3.63 g).
MS (ESI) m/z : 456 (M + H)⁺.
¹H-NMR (CDCl₃) δ: 1.22 (3H, t, J = 7.2 Hz), 2.79 (1H, dd, J = 16.5, 6.7 Hz), 3.03 (1H, dd, J = 16.5, 6.7 Hz), 3.91 (3H, s), 4.11 (2H, q, J = 7.2 Hz), 4.63 (1H, t, J = 6.7 Hz), 6.26 (1H, s), 6.36-6.75 (2H, m), 7.00 (1H, d, J = 8.3 Hz), 7.03 (1H, dd, J = 8.3, 1.5 Hz), 7.15-7.18 (1H, m), 7.28-7.33 (2H, m), 7.77 (1H, br s).

### Referential Example 425

Ethyl 2-{3,5-trans-7-chloro-5-[2-(difluoromethoxy)-3-methoxyphenyl]-2-thioxo-1,2,3,5-tetrahydro-4,1-benzoxazepin-3-yl}acetate

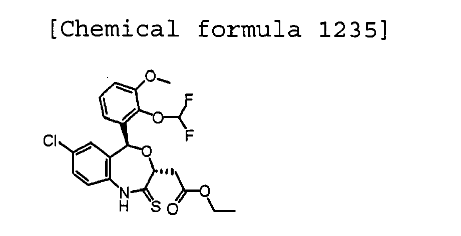

Ethyl 2-{3,5-trans-7-chloro-5-[2-(difluoromethoxy)-3-methoxyphenyl]-2-oxo-1,2,3,5-tetrahydro-4,1-benzoxazepin-3-yl}acetate (3.63 g) was dissolved in tetrahydrofuran (100 mL). To the resulting solution were added sodium hydrogen carbonate (4.09 g) and diphosphorus pentasulfide (9.01 g). The resulting mixture was stirred at room temperature for 2 hours. Sodium hydrogen carbonate (1.36 g) and diphosphorus pentasulfide (3.60 g) were added and the resulting mixture was stirred at room temperature for one hour. The reaction mixture was diluted with ethyl acetate and washed successively with a saturated aqueous solution of sodium bicarbonate and saturated brine. The organic layer was dried over anhydrous sodium sulfate. After concentration of the filtrate, the residue thus obtained was purified by silica gel chromatography (Automatic purification system of Yamazen Corporation, ethyl acetate:n-hexane=1:4, Rf=0.52) to give the title compound (3.38 g).
MS (ESI) m/z : 472 (M + H)⁺.
¹H-NMR (CDCl₃) δ: 1.24 (3H, t, J = 7.1 Hz), 2.96 (1H, dd, J = 16.4, 6.6 Hz), 3.25 (1H, dd, J = 16.4, 6.6 Hz), 3.91 (3H, s), 4.13 (2H, q, J = 7.1 Hz), 4.70 (1H, t, J = 6.6 Hz), 6.18 (1H, s), 6.52 (2H, dd, J = 76.1, 75.1 Hz), 6.59 (2H, d, J = 2.2 Hz), 7.04 (1H, dd, J = 8.3, 1.7 Hz), 7.07 (1H, d, J = 8.5 Hz), 7.21-7.24 (1H, m), 7.30-7.37 (2H, m), 9.57 (1H, br s).

### Example 617

Ethyl 2-[4,6-trans-8-chloro-6-[2-(difluoromethoxy)-3-methoxyphenyl]-1-(trifluoromethyl)-4H,6H-[1,2,4]triazolo[4,3-a][4,1]benzoxazepin-4-yl]acetate

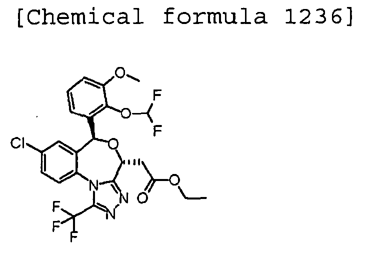

Ethyl 2-{3,5-trans-7-chloro-5-[2-(difluoromethoxy)-3-methoxyphenyl]-2-thioxo-1,2,3,5-tetrahydro-4,1-benzoxazepin-3-yl}acetate (675 mg) was dissolved in tetrahydrofuran (5 mL). To the resulting solution was added hydrazine monohydrate (0.109 mL) and the resulting mixture was stirred at room temperature for 2 hours. Under ice cooling, trifluoroacetic anhydride (1.06 mL) was added and the resulting mixture was stirred at room temperature for 16 hours. The reaction mixture was diluted with ethyl acetate and then washed with a saturated aqueous solution of sodium bicarbonate and saturated brine. The organic layer was dried over sodium sulfate and concentrated under reduced pressure. The residue thus obtained was purified by silica gel chromatography (Automatic purification system of Yamazen Corporation, ethyl acetate:n-hexane=1:2, Rf=0.60) to give the title compound (741 mg).
MS (ESI) m/z : 548 (M + H)⁺.
¹H-NMR (CDCl₃) δ: 1.28 (3H, t, J = 7.1 Hz), 3.24 (1H, dd, J = 16.8, 6.7 Hz), 3.49 (1H, dd, J = 16.8, 6.7 Hz), 3.88 (3H, s), 4.16-4.22 (2H, m), 4.94 (1H, t, J = 6.7 Hz), 5.60 (1H, s), 6.41 (1H, t, J = 75.9 Hz), 6.79-6.81 (1H, m), 7.04 (1H, dd, J = 8.0, 1.7 Hz), 7.31 (1H, dd, J = 8.0, 1.7 Hz), 7.35 (1H, t, J = 7.9 Hz), 7.53-7.55 (2H, m).

### Example 618

Ethyl 2-[(4S,6R)-8-chloro-6-[2-(difluoromethoxy)-3-methoxyphenyl]-1-(trifluoromethyl)-4H,6H-[1,2,4]triazolo[4,3-a][4,1]benzoxazepin-4-yl]acetate isomer A
Ethyl 2-[(4R,6S)-8-chloro-6-[2-(difluoromethoxy)-3-methoxyphenyl]-1-(trifluoromethyl)-4H,6H-[1,2,4]triazolo[4,3-a][4,1]benzoxazepin-4-yl]acetateisomer B

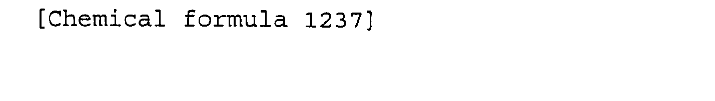

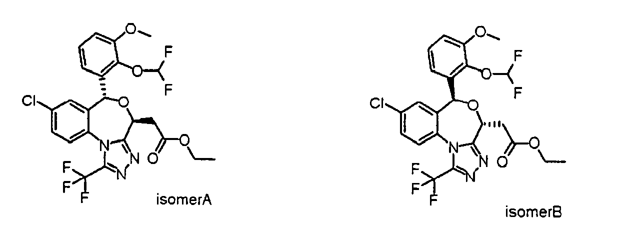

Ethyl 2-[4,6-trans-8-chloro-6-[2-(difluoromethoxy)-3-methoxyphenyl]-1-(trifluoromethyl)-4H,6H-[1,2,4]triazolo[4,3-a][4,1]benzoxazepin-4-yl]acetate was optically resolved by HPLC.
Column: Chiralpak AD (5 × 50cm)
Eluting solution: 10% isopropanol-n-hexane
Flow rate: 50 mL/min
Elution time: isomer A: 90 min., isomer B: 108 min.

### Example 619

2-[(4S,6R)-8-Chloro-6-[2-(difluoromethoxy)-3-methoxyphenyl]-1-(trifluoromethyl)-4H,6H-[1,2,4]triazolo[4,3-a][4,1]benzoxazepin-4-yl]acetic acid

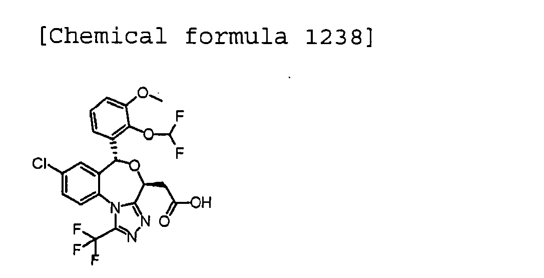

Ethyl 2-[(4S,6R)-8-chloro-6-[2-(difluoromethoxy)-3-methoxyphenyl]-1-(trifluoromethyl)-4H,6H-[1,2,4]triazolo[4,3-a][4,1]benzoxazepin-4-yl]acetate (266 mg) was dissolved in 1,4-dioxane (20 mL). To the resulting solution were added concentrated hydrochloric acid (10 mL) and distilled water (10 mL). The resulting mixture was stirred at 60°C for 11 hours. The reaction mixture was concentrated under reduced pressure. The residue thus obtained was solidified from a methylene chloride-n-hexane solvent mixture to give the title compound (216 mg).
MS (ESI) m/z : 520 (M + H)⁺.
¹H-NMR (CDCl₃) δ: 3.31 (1H, dd, J = 17.1, 6.7 Hz), 3.57 (1H, dd, J = 17.1, 6.7 Hz), 3.88 (3H, s), 4.91 (1H, t, J = 6.7 Hz), 5.60 (1H, s), 6.41 (1H, t, J = 75.9 Hz), 6.80-6.82 (1H, m), 7.05 (1H, dd, J = 8.1, 1.7 Hz), 7.30-7.38 (2H, m), 7.53-7.55 (2H, m).
Elemental analysis for: C₂₁H₁₅ClF₅N₃O₅·0.1 1,4-dioxane Calculated: C, 48.62; H, 3.01; N, 7.95.
Found: C, 48.49; H, 3.32; N, 7.60.

### Example 620

2-[(4R,6S)-8-Chloro-6-[2-(difluoromethoxy)-3-methoxyphenyl]-1-(trifluoromethyl)-4H,6H-[1,2,4]triazolo[4,3-a][4,1]benzoxazepin-4-yl]acetic acid

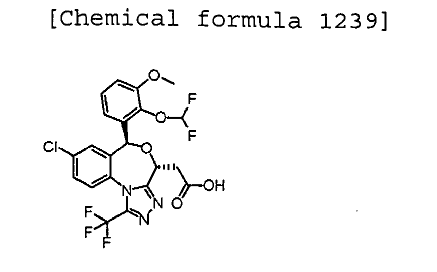

Ethyl 2-[(4R,6S)-8-chloro-6-[2-(difluoromethoxy)-3-methoxyphenyl]-1-(trifluoromethyl)-4H,6H-[1,2,4]triazolo[4,3-a][4,1]benzoxazepin-4-yl]acetate (242 mg) was dissolved in 1,4-dioxane (20 mL). To the resulting solution were added concentrated hydrochloric acid (10 mL) and distilled water (10 mL). The resulting mixture was stirred at 60°C for 11 hours. The reaction mixture was concentrated under reduced pressure. The residue thus obtained was solidified from a methylene chloride-n-hexane solvent mixture to give the title compound (216 mg).
MS (ESI) m/z : 520 (M + H)⁺.
¹H-NMR (CDCl₃) δ: 3.30 (1H, dd, J = 17.1, 6.8 Hz), 3.57 (1H, dd, J = 17.1, 6.8 Hz), 3.88 (3H, s), 4.91 (1H, t, J = 6.8 Hz), 5.60 (1H, s), 6.42 (1H, t, J = 75.8 Hz), 6.80-6.82 (1H, m), 7.05 (1H, dd, J = 7.9, 1.8 Hz), 7.30-7.38 (2H, m), 7.53-7.55 (2H, m).
Elemental analysis for: C₂₁H₁₅ClF₅N₃O₅·0.25 H₂O·0.1 1,4-dioxane
Calculated: C, 48.37; H, 3.23; N, 7.69.
Found: C, 48.07; H, 3.35; N, 7.30.

### Referential Example 426

{5-Chloro-2-[(2,4-dimethoxybenzyl)amino]phenyl}[3-methoxy-2-(trifluoromethoxy)phenyl]methanol

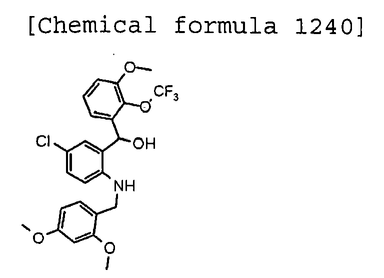

In a similar manner to that employed for the synthesis of {5-chloro-2-[(2,4-dimethoxybenzyl)amino]phenyl}(2,3-dimethoxyphenyl)methanol, the title compound (25.1 g) was obtained from (2-amino-5-chlorophenyl)[3-methoxy-2-(trifluoromethoxy)phenyl]methanol (20.4 g).
MS (EI) m/z: 497 M⁺.
¹H-NMR (CDCl3) δ: 3.77 (3H, s), 3.79 (3H, s), 3.88 (3H, s), 4.23 (2H, s), 6.13 (1H, s), 6.38 (1H, dd, J = 8.3, 2.4 Hz), 6.44 (1H, d, J = 2.4 Hz), 6.61 (1H, d, J = 8.3 Hz), 6.95-7.05 (4H, m), 7.09 (1H, dd, J = 8.8, 2.4 Hz), 7.23-7.30 (1H, m).

### Referential Example 427

Ethyl 2-{trans-7-chloro-1-(2,4-dimethoxybenzyl)-5-[3-methoxy-2-(trifluoromethoxy)phenyl]-2-oxo-1,2,3,5-tetrahydro-4,1-benzoxazepin-3-yl}acetate

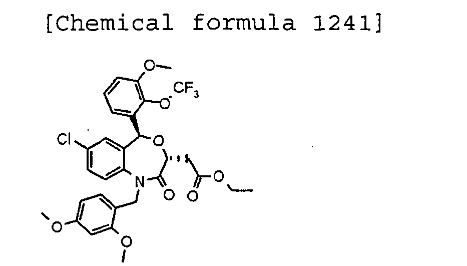

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (22.2 g) was obtained from {5-chloro-2-[(2,4-dimethoxybenzyl)amino]phenyl}[3-methoxy-2-(trifluoromethoxy)phenyl]methanol (25.1 g).
MS (FAB) m/z: 624 (M+H)⁺.
¹H-NMR (CDCl3) δ: 1.25 (3H, t, J = 7.1 Hz), 2.79 (1H, dd, J = 16.6, 5.9 Hz), 3.11 (1H, dd, J = 16.6, 7.8 Hz), 3.73 (3H, s), 3.78 (3H, s), 3.88 (3H, s), 4.14 (2H, q, J = 7.1 Hz), 4.55 (1H, dd, J = 7.8, 5.9 Hz), 5.12 (2H, s), 6.00 (1H, s), 6.40-6.47 (2H, m), 6.54 (1H, d, J = 2.0 Hz), 7.03 (1H, dd, J = 8.3, 1.3 Hz), 7.20 (1H, d, J = 8.3 Hz), 7.25-7.29 (3H, m), 7.37 (1H, t, J = 8.3 Hz).

### Referential Example 428

Ethyl 2-{trans-7-chloro-5-[3-methoxy-2-(trifluoromethoxy)phenyl]-2-oxo-1,2,3,5-tetrahydro-4,1-benzoxazepin-3-yl}acetate

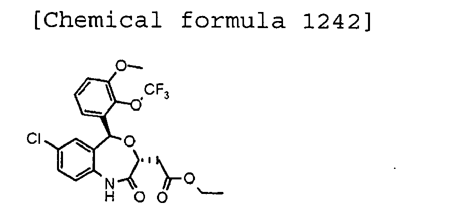

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (13.9 g) was obtained from ethyl 2-{trans-7-chloro-1-(2,4-dimethoxybenzyl)-5-[3-methoxy-2-(trifluoromethoxy)phenyl]-2-oxo-1,2,3,5-tetrahydro-4,1-benzoxazepin-3-yl}acetate (22.2 g).
MS (FAB) m/z: 474 (M+H)⁺.
¹H-NMR (CDCl3) δ: 1.22 (3H, t, J = 7.2 Hz), 2.78 (1H, dd, J = 16.6, 6.6 Hz), 3.03 (1H, dd, J = 16.6, 6.6 Hz), 3.91 (3H, s), 4.11 (2H, q, J = 7.2 Hz), 4.62 (1H, t, J = 6.6 Hz), 6.19 (1H, s), 6.64 (1H, d, J = 2.4 Hz), 7.02 (1H, d, J = 8.3 Hz), 7.06 (1H, dd, J = 8.3, 1.5 Hz), 7.18 (1H, dd, J = 8.0, 1.2 Hz), 7.31 (1H, dd, J = 8.3, 2.3 Hz), 7.37 (1H, t, J = 8.0 Hz), 7.93 (1H, br s).

### Referential Example 429

Ethyl 2-{trans-7-chloro-5-[3-methoxy-2-(trifluoromethoxy)phenyl]-2-thioxo-1,2,3,5-tetrahydro-4,1-benzoxazepin-3-yl}acetate

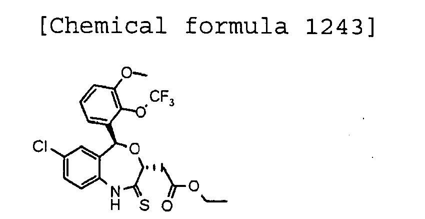

Ethyl 2-{trans-7-chloro-5-[3-methoxy-2-(trifluoromethoxy)phenyl]-2-oxo-1,2,3,5-tetrahydro-4,1-benzoxazepin-3-yl}acetate (13.9 g) was dissolved in tetrahydrofuran (500 mL). To the resulting solution was added sodium hydrogen carbonate (18.5 g). Under ice cooling, diphosphorus pentasulfide (42.4 g) was added. After warming to room temperature, the reaction mixture was stirred for 4 hours. Under ice cooling, a saturated aqueous solution of sodium hydrogen carbonate (1500 mL) was added in portions and the reaction mixture was extracted with ethyl acetate. The organic layer was dried over anhydrous sodium sulfate. The solvent was distilled off under reduced pressure. The residue was purified by silica gel column chromatography (hexane:ethyl acetate=4:1) to give the title compound (13.0 g).
MS (FAB) m/z: 490 (M+H)⁺.
¹H-NMR (CDCl3) δ: 1.24 (3H, t, J = 7.1 Hz), 2.95 (1H, dd, J = 16.6, 6.6 Hz), 3.24 (1H, dd, J = 16.6, 6.6 Hz), 3.90 (3H, s), 4.13 (2H, q, J = 7.1 Hz), 4.70 (1H, t, J = 6.6 Hz), 6.10 (1H, s), 6.63 (1H, d, J = 2.2 Hz), 7.07 (2H, t, J = 8.3 Hz), 7.23 (1H, d, J = 8.0 Hz), 7.34-7.42 (2H, m), 9.63 (1H, s).

### Example 621

Ethyl 2-[trans-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate

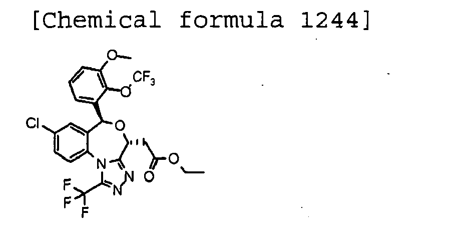

In a similar manner to that employed for the synthesis of methyl 2-[trans-8-chloro-6-(2,3-dimethoxyphenyl)-10-fluoro-1-(trifluoromethyl)-4H,6H-[1,2,4]triazolo[4,3-a] [4,1] benzothiazepin-4-yl] acetate, the title compound (2.35 g) was obtained from ethyl 2-{trans-7-chloro-5-[3-methoxy-2-(trifluoromethoxy)phenyl]-2-thioxo-1,2,3,5-tetrahydro-4,1-benzoxazepin-3-yl}acetate (3.00 g).
MS (FAB) m/z: 566 (M+H)⁺.
¹H-NMR (CDCl3) δ: 1.28 (3H, t, J = 7.2 Hz), 3.24 (1H, dd, J = 16.6, 6.8 Hz), 3.49 (1H, dd, J = 16.6, 6.8 Hz), 3.88 (3H, s), 4.19 (2H, q, J = 7.2 Hz), 4.95 (1H, t, J = 6.8 Hz), 5.55 (1H, s), 6.84 (1H, d, J = 2.0 Hz), 7.07 (1H, dd, J = 8.2, 1.6 Hz), 7.31 (1H, dd, J = 8.0, 1.0 Hz), 7.42 (1H, t, J = 8.0 Hz), 7.51-7.58 (2H, m).

### Example 622

Ethyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate
(isomer A)

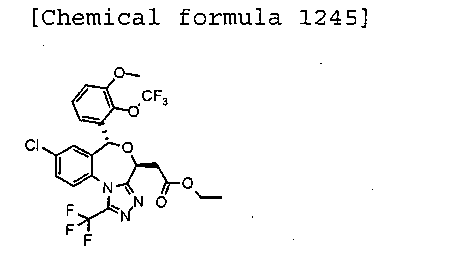

Ethyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate
(isomer B)

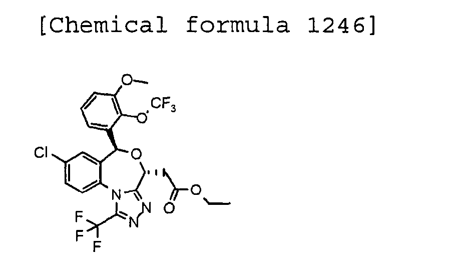

Ethyl 2-[trans-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate(2.34 g) was optically resolved by a CHIRALPAK AD column (Daicel Chemical Industries, hexane:ethanol=9:1, flow rate: 50 mL/min) to give ethyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate (isomer A, retention time: 34 min., 1.18 g) and ethyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate (isomer B, retention time: 55 min., 1.18 g), respectively. Ethyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate
(isomer A)
MS (FAB) m/z: 566 (M+H)⁺.
¹H-NMR (CDCl3) δ: 1.28 (3H, t, J = 7.2 Hz), 3.24 (1H, dd, J = 16.6, 6.8 Hz), 3.49 (1H, dd, J = 16.6, 6.8 Hz), 3.88 (3H, s), 4.19 (2H, q, J = 7.2 Hz), 4.95 (1H, t, J = 6.8 Hz), 5.55 (1H, s), 6.84 (1H, d, J = 2.0 Hz), 7.07 (1H, dd, J = 8.2, 1.6 Hz), 7.31 (1H, dd, J = 8.0, 1.0 Hz), 7.42 (1H, t, J = 8.0 Hz), 7.51-7.58 (2H, m).
Ethyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate
(isomer B)
MS (FAB) m/z: 566 (M+H)⁺.
¹H-NMR (CDCl3) δ: 1.28 (3H, t, J = 7.2 Hz), 3.24 (1H, dd, J = 16.6, 6.8 Hz), 3.49 (1H, dd, J = 16.6, 6.8 Hz), 3.88 (3H, s), 4.19 (2H, q, J = 7.2 Hz), 4.95 (1H, t, J = 6.8 Hz), 5.55 (1H, s), 6.84 (1H, d, J = 2.0 Hz), 7.07 (1H, dd, J = 8.2, 1.6 Hz), 7.31 (1H, dd, J = 8.0, 1.0 Hz), 7.42 (1H, t, J = 8.0 Hz), 7.51-7.58 (2H, m).

### Example 623

2-[(4S,6R)-8-Chloro-6-[3-methoxy-2-(trifluoromethoxy)phenyl]-1-(trifluoromethyl)-4H,6H-[1,2,4]triazolo[4,3-a][4,1]benzoxazepin-4-yl]acetic acid
(isomer A)

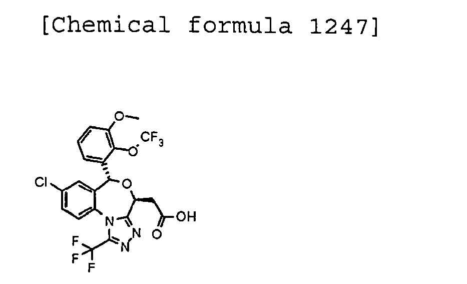

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (1.12 g) was obtained from ethyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate (isomer A, 1.18 g).
MS (FAB) m/z: 538 (M+H)⁺.
¹H-NMR (CDCl3) δ: 3.30 (1H, dd, J = 17.2, 6.8 Hz), 3.57 (1H, dd, J = 17.2, 6.8 Hz), 3.88 (3H, s), 4.91 (1H, t, J = 6.8 Hz), 5.55 (1H, s), 6.84 (1H, d, J = 2.0 Hz), 7.07 (1H, dd, J = 8.3, 1.5 Hz), 7.31 (1H, dd, J = 7.8, 1.0 Hz), 7.42 (1H, t, J = 8.3 Hz), 7.55 (2H, t, J = 2.8 Hz).
Elemental analysis for: C₂₁H₁₄ClF₆N₃O₅
Calculated: C, 46.90; H, 2.62; N, 7.81
Found: C, 46.75; H, 2.69; N, 7.46.

### Example 624

2-[(4R,6S)-8-Chloro-6-[3-methoxy-2-(trifluoromethoxy)phenyl]-1-(trifluoromethyl)-4H,6H-[1,2,4]triazolo[4,3-a][4,1]benzoxazepin-4-yl]acetic acid
(isomer B)

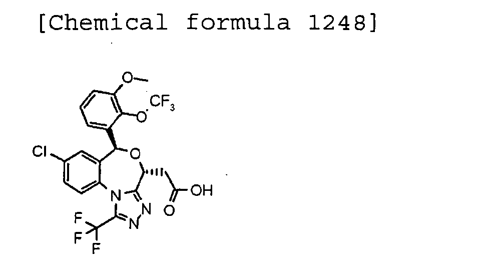

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a] [4,1]benzothiazepin-4-yl]methyl}-1,3-thiazol-5-yl)propanoic acid, the title compound (1.11 g) was obtained from ethyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetate (isomer B, 1.18 g).
MS (FAB) m/z: 538 (M+H)⁺. ¹H-NMR (CDCl3) δ: 3.30 (1H, dd, J = 17.2, 6.8 Hz), 3.57 (1H, dd, J = 17.2, 6.8 Hz), 3.88 (3H, s), 4.91 (1H, t, J = 6.8 Hz), 5.55 (1H, s), 6.84 (1H, d, J = 2.0 Hz), 7.07 (1H, dd, J = 8.3, 1.5 Hz), 7.31 (1H, dd, J = 7.8, 1.0 Hz), 7.42 (1H, t, J = 8.3 Hz), 7.55 (2H, t, J = 2.8 Hz).
Elemental analysis for: C₂₁H₁₄ClF₆N₃O₅
Calculated: C, 46.90; H, 2.62; N, 7.81
Found: C, 46.64; H, 2.71; N, 7.42.

### Example 625

Ethyl 2-(1-{2-{(4R,6S)-8-chloro-6-[3-methoxy-2-(trifluoromethoxy)phenyl]-1-(trifluoromethyl)-4H,6H-[1,2,4]triazolo[4,3-a] [4,1]benzoxazepin-4-yl]acetyl}-4-piperidinyl)acetate

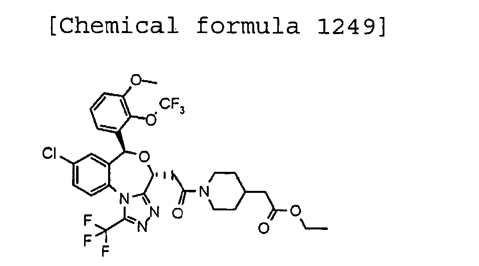

In a similar manner to that employed for the synthesis of ethyl 2-(1-{2- [8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl) acetate, the title compound (137 mg) was obtained from 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzoxazepin-4-yl]acetic acid (isomer B, 170 mg).
MS (EI) m/z: 690 M⁺.
¹H-NMR (CDCl3) δ: 1.07-1.45 (5H, m), 1.70-1.86 (2H, m), 1.98-2.11 (1H, m), 2.20 (1H, d, J = 6.8 Hz), 2.27 (1H, d, J = 7.1 Hz), 2.57-2.66 (1H, m), 3.03-3.25 (2H, m), 3.47-3.63 (1H, m), 3.88 (3H, s), 3.97-4.07 (1H, m), 4.14 (2H, q, J = 7.1 Hz), 4.54 (1H, d, J = 12.7 Hz), 5.06-5.13 (1H, m), 5.52 (1H, d, J = 3.9 Hz), 6.82 (1H, s), 7.07 (1H, d, J = 8.3 Hz), 7.32-7.37 (1H, m), 7.39-7.45 (1H, m), 7.53 (2H, br s).

### Example 626

2-(1-{2-[(4R,6S)-8-Chloro-6-[3-methoxy-2-(trifluoromethoxy)phenyl]-1-(trifluoromethyl)-4H,6H-[1,2,4]triazolo[4,3-a] [4,1]benzoxazepin-4-yl]acetyl}-4-piperidinyl)acetic acid

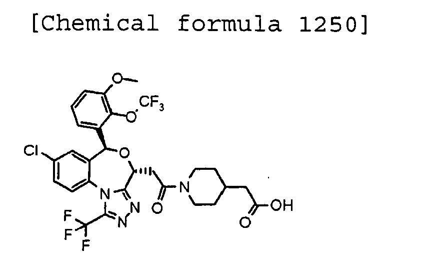

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (89 mg) was obtained from ethyl 2-(1-{2-[(4R,6S)-8-chloro-6-[3-methoxy-2-(trifluoromethoxy)phenyl]-1-(trifluoromethyl)-4H,6H-[1,2,4]triazolo[4,3-a] [4,1]benzoxazepin-4-yl]acetyl}-4-piperidinyl)acetate (133 mg).
MS (FAB) m/z: 662 M⁺.
¹H-NMR (CDCl3) δ: 1.09-1.51 (2H, m), 1.72-1.90 (2H, m), 1.99-2.10 (1H, m), 2.25 (1H, d, J = 6.1 Hz), 2.34 (1H, d, J = 6.8 Hz), 2.57-2.66 (1H, m), 3.03-3.27 (2H, m), 3.47-3.64 (1H, m), 3.88 (3H, s), 3.98-4.09 (1H, m), 4.56 (1H, d, J = 12.7 Hz), 5.08-5.12 (1H, m), 5.52 (1H, d, J = 5.1 Hz), 6.82 (1H, s), 7.07 (1H, d, J = 8.0 Hz), 7.32-7.37 (1H, m), 7.40-7.45 (1H, m), 7.53 (2H, br s).
Elemental analysis for: C₂₈H₂₅ClF₆N₄O₆·1.0H₂O·0.2CHCl₃ Calculated: C, 48.23; H, 3.88; N, 7.92
Found: C, 48.01; H, 3.69; N, 7.54.

### Referential Example 430

tert-Butyl 4-chloro-2-{hydroxy[2-(trifluoromethyl)phenyl]methyl}phenylcarbamate

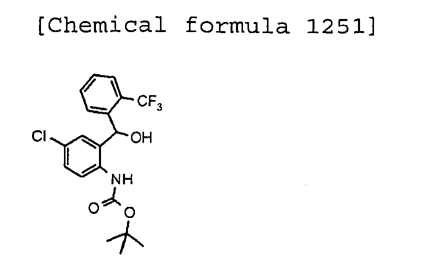

In a similar manner to that employed for the synthesis of tert-butyl 4-chloro-2-{hydroxy[2-(trifluoromethoxy)phenyl]methyl}phenylcarbamate, the title compound (11.6 g) was obtained from tert-butyl 4-chlorophenylcarbamate (8.32 g) and 2-fluoromethylbenzaldehyde (7.00 g).
MS (EI) m/z: 401 M⁺.
¹H-NMR (CDCl3) δ: 1.48 (9H, s), 3.12 (1H, br s), 6.31 (1H, s), 6.95 (1H, d, J = 2.4 Hz), 7.13 (1H, br s), 7.22-7.26 (1H, m), 7.48 (1H, t, J = 7.6 Hz), 7.60-7.73 (4H, m).

### Referential Example 431

(2-Amino-5-chlorophenyl)[2-(trifluoromethyl)phenyl]methanol

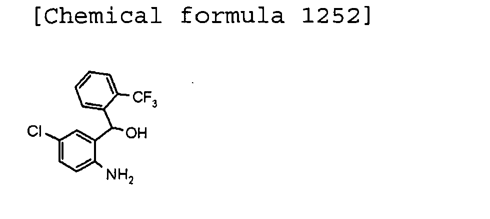

In a similar manner to that employed for the synthesis of 2-amino-5-chloro-3-[(2,3-dimethoxyphenyl)(hydroxy)methyl]pyridine, the title compound (10.9 g) was obtained from tert-butyl 4-chloro-2-{hydroxy[2-(trifluoromethyl)phenyl]methyl}phenylcarbamate (11.6 g).
MS (EI) m/z: 301 M⁺.
¹H-NMR (CDCl3) δ: 6.23 (1H, s), 6.63 (1H, d, J = 8.5 Hz), 6.92 (1H, d, J = 2.4 Hz), 7.08 (1H, dd, J = 8.5, 2.4 Hz), 7.48 (1H, t, J = 7.8 Hz), 7.57-7.65 (2H, m), 7.73 (1H, d, J = 7.8 Hz).

### Referential Example 432

{5-Chloro-2-[(2,4-dimethoxybenzyl)amino]phenyl}[2-(trifluoromethyl)phenyl]methanol

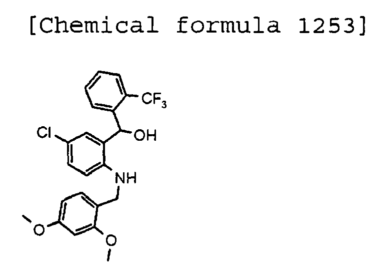

In a similar manner to that employed for the synthesis of (5-chloro-2-[(2,4-dimethoxybenzyl)amino]phenyl}(2,3-dimethoxyphenyl)methanol, the title compound (10.7 g) was obtained from (2-amino-5-chlorophenyl)[2-(trifluoromethyl)phenyl]methanol (10.9 g).
MS (EI) m/z: 451 M⁺.
¹H-NMR (CDCl3) δ: 3.74 (3H, s), 3.78 (3H, s), 4.22 (2H, s), 6.21 (1H, s), 6.38 (1H, dd, J = 8.3, 2.4 Hz), 6.43 (1H, d, J = 2.4 Hz), 6.64 (1H, d, J = 8.8 Hz), 6.88 (1H, d, J = 2.4 Hz), 7.01 (1H, d, J = 8.3 Hz), 7.11 (1H, dd, J = 8.3, 2.4 Hz), 7.26 (1H, s), 7.46 (1H, t, J = 7.8 Hz), 7.55-7.64 (2H, m), 7.71 (1H, d, J = 7.8 Hz).

### Referential Example 433

Ethyl 2-{trans-7-chloro-1-(2,4-dimethoxybenzyl)-2-oxo-5-[2-(trifluoromethyl)phenyl]-1,2,3,5-tetrahydro-4,1-benzoxazepin-3-yl}acetate

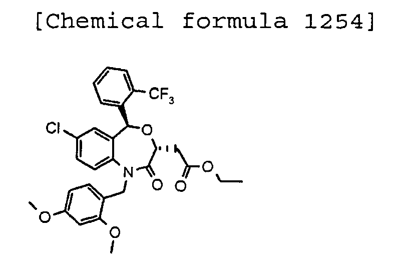

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (13.1 g) was obtained from {5-chloro-2-[(2,4-dimethoxybenzyl)amino]phenyl}[2-(trifluoromethyl)phenyl]methanol (10.71 g).
MS (FAB) m/z: 578 (M+H)⁺.
¹H-NMR (CDCl3) δ: 1.25 (3H, t, J = 7.1 Hz), 2.82 (1H, dd, J = 16.7, 6.2 Hz), 3.08 (1H, dd, J = 16.7, 7.2 Hz), 3.67 (2H, s), 3.78 (3H, s), 4.15 (2H, q, J = 7.1 Hz), 4.56 (1H, dd, J = 7.2, 6.2 Hz), 5.02 (1H, d, J = 15.1 Hz), 5.28 (1H, d, J = 15.1 Hz), 5.98 (1H, s), 6.32 (1H, d, J = 2.2 Hz), 6.40 (1H, d, J = 2.4 Hz), 6.44 (1H, dd, J = 8.5, 2.4 Hz), 7.25-7.34 (3H, m), 7.49 (1H, t, J = 7.8 Hz), 7.62-7.69 (2H, m), 7.87 (1H, d, J = 7.8 Hz) .

### Referential Example 434

Ethyl 2-{trans-7-chloro-2-oxo-5-[2-(trifluoromethyl)phenyl]-1,2,3,5-tetrahydro-4,1-benzoxazepin-3-yl}acetate

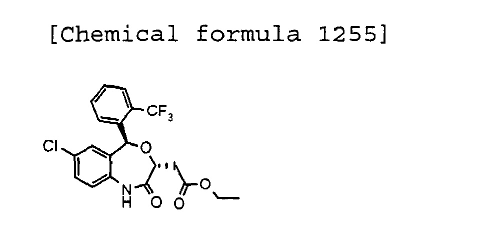

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (6.50 g) was obtained from ethyl 2-{trans-7-chloro-1-(2,4-dimethoxybenzyl)-2-oxo-5-[2-(trifluoromethyl)phenyl]-1,2,3,5-tetrahydro-4,1-benzoxazepin-3-yl}acetate (12.1 g).
MS (FAB) m/z: 428 (M+H)⁺.
¹H-NMR (CDCl3) δ: 1.22 (3H, t, J = 7.1 Hz), 2.82 (1H, dd, J = 16.6, 7.1 Hz), 3.04 (1H, dd, J = 16.6, 6.3 Hz), 4.11 (2H, q, J = 7.1 Hz), 4.70 (1H, dd, J = 7.1, 6.3 Hz), 6.24 (1H, s), 6.45 (1H, d, J = 2.2 Hz), 7.03 (1H, d, J = 8.5 Hz), 7.31 (1H, dd, J = 8.5, 2.4 Hz), 7.54 (1H, t, J = 7.8 Hz), 7.66-7.74 (2H, m), 7.84 (1H, d, J = 7.8 Hz), 8.10 (1H, s).

### Referential Example 435

Ethyl 2-{trans-7-chloro-2-thioxo-5-[2-(trifluoromethyl)phenyl]-1,2,3,5-tetrahydro-4,1-benzoxazepin-3-yl}acetate

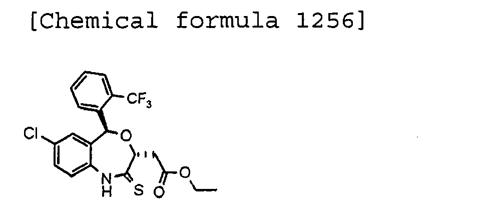

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (2.67 g) was obtained from ethyl 2-{trans-7-chloro-2-oxo-5-[2-(trifluoromethyl)phenyl]-1,2,3,5-tetrahydro-4,1-benzoxazepin-3-yl}acetate (2.00 g).
MS (EI) m/z: 443 M⁺.
¹H-NMR (CDCl3) δ: 1.25 (3H, t, J = 7.1 Hz), 2.99 (1H, dd, J = 16.6, 6.9 Hz), 3.23 (1H, dd, J = 16.6, 6.4 Hz), 4.14 (2H, q, J = 7.1 Hz), 4.75 (1H, dd, J = 6.9, 6.4 Hz), 6.16 (1H, s), 6.44 (1H, d, J = 2.2 Hz), 7.08 (1H, d, J = 8.3 Hz), 7.36 (1H, dd, J = 8.3, 2.2 Hz), 7.54 (1H, t, J = 7.8 Hz), 7.70 (1H, t, J = 7.8 Hz), 7.71 (1H, d, J = 7.8 Hz), 7.87 (1H, d, J = 7.8 Hz), 9.65 (1H, s).

### Example 627

Ethyl 2-{trans-8-chloro-1-(trifluoromethyl)-6-[2-(trifluoromethyl)phenyl]-4H,6H-[1,2,4]triazolo[4,3-a][4,1]benzoxazepin-4-yl}acetate

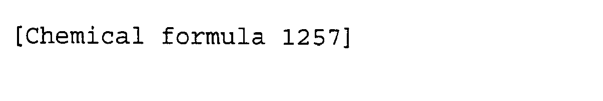

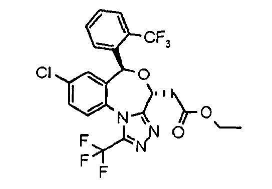

In a similar manner to that employed for the synthesis of ethyl 2-[trans-8-chloro-6-(2-fluoro-3-methoxyphenyl)-1-(trifluoromethyl)-4H,6H-[1,2,4]triazolo[4,3-a][4,1]benzothiazepin-4-yl]acetate, the title compound (2.33 g) was obtained from ethyl 2-{trans-7-chloro-2-thioxo-5-[2-(trifluoromethyl)phenyl]-1,2,3,5-tetrahydro-4,1-benzoxazepin-3-yl}acetate (2.67 g).
MS (EI) m/z: 519 M⁺.
¹H-NMR (CDCl3) δ: 1.29 (3H, t, J = 7.1 Hz), 3.27 (1H, dd, J = 16.7, 7.4 Hz), 3.49 (1H, dd, J = 16.7, 6.0 Hz), 4.20 (2H, q, J = 7.1 Hz), 4.99 (1H, dd, J = 7.4, 6.0 Hz), 5.61 (1H, s), 6.65 (1H, d, J = 1.5 Hz), 7.51-7.59 (3H, m), 7.69 (1H, d, J = 7.8 Hz), 7.74 (1H, t, J = 7.8 Hz), 7.95 (1H, d, J = 7.8 Hz).

### Example 628

2-{trans-8-Chloro-1-(trifluoromethyl)-6-[2-(trifluoromethyl)phenyl]-4H,6H-[1,2,4]triazolo[4,3-a][4,1]benzoxazepin-4-yl}acetic acid

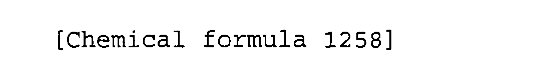

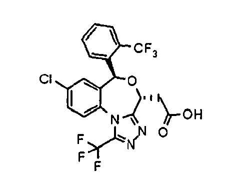

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (101 mg) was obtained from ethyl 2-{trans-8-chloro-1-(trifluoromethyl)-6-[2-(trifluoromethyl)phenyl]-4H,6H-[1,2,4]triazolo[4,3-a][4,1]benzoxazepin-4-yl}acetate (100 mg).
MS (EI) m/z: 491 M⁺.
¹H-NMR (CDCl3) δ: 3.34 (1H, dd, J = 17.3, 7.1 Hz), 3.58 (1H, dd, J = 17.2, 6.2 Hz), 4.97 (1H, dd, J = 7.1, 6.2 Hz), 5.62 (1H, s), 6.66 (1H, d, J = 1.7 Hz), 7.52-7.59 (3H, m), 7.70 (1H, d, J = 7.8 Hz), 7.75 (2H, t, J = 7.8 Hz), 7.96 (1H, d, J = 7.8 Hz).
Elemental analysis for: C₂₀H₁₂ClF₄N₃O₃·0.75H₂O·0.1CHCl₃ Calculated: C, 46.80; H, 2.64; N, 8.11
Found: C, 47.01; H, 2.41; N, 7.87.

### Referential Example 436

3-Fluoro-2-methoxybenzaldehyde

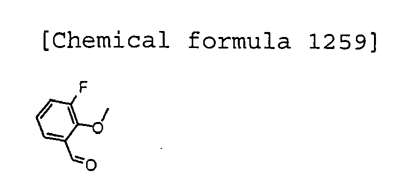

3-Fluorosalicylbenzaldehyde (4.00 g) was dissolved in N,N-dimethylformamide (100 mL). To the resulting solution were added anhydrous potassium carbonate (6.70 g) and methyl iodide (8.88 mL). The resulting mixture was stirred at 50°C for 3 hours. After cooling to room temperature, ice water and diethyl ether were added to the reaction mixture and the layers separated. The organic layer was dried over anhydrous magnesium sulfate and the solvent was distilled off to give the title compound (4.59 g).
MS (EI) m/z: 154 M⁺.
¹H-NMR (CDCl3) δ: 4.10 (3H, d, J = 2.9 Hz), 7.05-7.13 (1H, m), 7.30-7.37 (1H, m), 7.59-7.63 (1H, m), 10.41 (1H, s).

### Referential Example 437

tert-Butyl 4-chloro-2-[(3-fluoro-2-methoxyphenyl)(hydroxy)methyl]phenylcarbamate

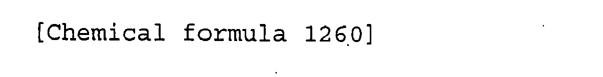

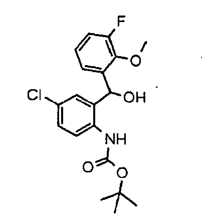

In a similar manner to that employed for the synthesis of tert-butyl 4-chloro-2-{hydroxy[2-(trifluoromethoxy)phenyl]methyl}phenylcarbamate, the title compound (5.51 g) was obtained from tert-butyl 4-chlorophenylcarbamate (5.65 g) and 3-fluoro-2-methoxybenzaldehyde (4.59 g).
MS (EI) m/z: 381 M⁺.
¹H-NMR (CDCl3) δ: 1.49 (9H, s), 3.27 (1H, d, J = 4.9 Hz), 3.90 (3H, d, J = 2.4 Hz), 6.06 (1H, d, J = 4.9 Hz), 6.99-7.12 (4H, m), 7.24 (2H, dd, J = 8.8, 2.2 Hz), 7.83 (1H, d, J = 8.8 Hz), 7.87 (1H, s).

### Referential Example 438

(2-Amino-5-chlorophenyl)(3-fluoro-2-methoxyphenyl)methanol

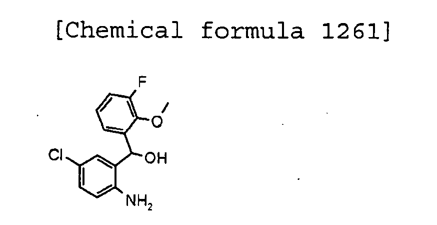

In a similar manner to that employed for the synthesis of 2-amino-5-chloro-3-[(2,3-dimethoxyphenyl)(hydroxy)methyl]pyridine, the title compound (5.20 g) was obtained from tert-butyl 4-chloro-2-[(3-fluoro-2-methoxyphenyl)(hydroxy)methyl]phenylcarbamate (5.50 g).
MS (EI) m/z: 281 M⁺.
¹H-NMR (CDCl3) δ: 3.92 (3H, d, J = 2.4 Hz), 6.02 (1H, s), 6.61 (1H, d, J = 8.5 Hz), 6.98-7.12 (4H, m).

### Referential Example 439

{5-Chloro-2-[(2,4-dimethoxybenzyl)amino]phenyl}(3-fluoro-2-methoxyphenyl)methanol

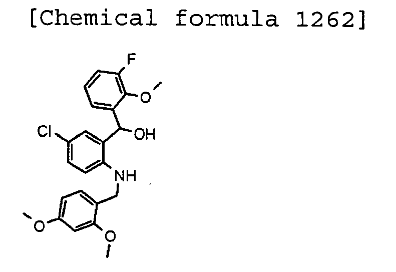

In a similar manner to that employed for the synthesis of {5-chloro-2-[(2,4-dimethoxybenzyl)amino]phenyl}(2,3-dimethoxyphenyl)methanol, the title compound (5.76 g) was obtained from (2-amino-5-chlorophenyl)(3-fluoro-2-methoxyphenyl)methanol (4.06 g).
MS (EI) m/z: 431 M⁺.
¹H-NMR (CDCl3) δ: 2.92 (1H, br s), 3.78 (3H, s), 3.79 (3H, s), 3.84 (3H, br s), 4.23 (2H, s), 5.17 (1H, br s), 6.01 (1H, s), 6.40 (2H, d, J = 8.0 Hz), 6.45 (1H, s), 6.62 (1H, d, J = 8.8 Hz), 6.95-7.12 (6H, m), 7.26 (1H, s).

### Referential Example 440

Ethyl 2-[trans-7-chloro-1-(2,4-dimethoxybenzyl)-5-(3-fluoro-2-methoxyphenyl)-2-oxo-1,2,3,5-tetrahydro-4,1-benzoxazepin-3-yl]acetate

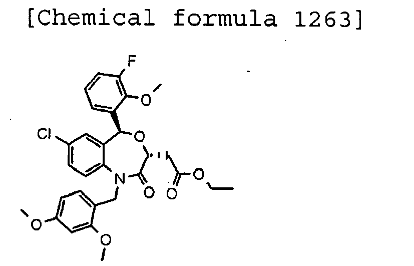

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (5.30 g) was obtained from {5-chloro-2-[(2,4-dimethoxybenzyl)amino]phenyl}(3-fluoro-2-methoxyphenyl)methanol (5.76 g).
MS (EI) m/z: 557 M⁺.
¹H-NMR (CDCl3) δ: 1.25 (3H, t, J = 7.1 Hz), 2.80 (1H, dd, J = 16.5, 5.9 Hz), 3.10 (1H, dd, J = 16.5, 7.8 Hz), 3.34 (3H, d, J = 2.0 Hz), 3.66 (3H, s), 3.76 (3H, s), 4.14 (2H, q, J = 7.1 Hz), 4.47 (1H, dd, J = 7.8, 5.9 Hz), 4.86 (1H, d, J = 14.9 Hz), 5.49 (1H, d, J = 14.9 Hz), 5.93 (1H, s), 6.38-6.43 (2H, m), 6.49 (1H, d, J = 2.2 Hz), 7.08-7.12 (2H, m), 7.30-7.33 (2H, m).

### Referential Example 441

Ethyl 2-[trans-7-chloro-5-(3-fluoro-2-methoxyphenyl)-2-oxo-1,2,3,5-tetrahydro-4,1-benzoxazepin-3-yl]acetate

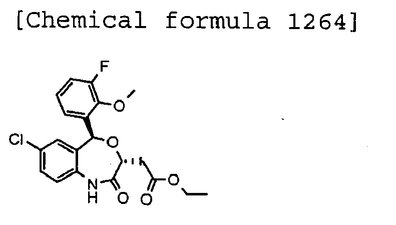

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (2.81 g) was obtained from ethyl 2-[trans-7-chloro-1-(2,4-dimethoxybenzyl)-5-(3-fluoro-2-methoxyphenyl)-2-oxo-1,2,3,5-tetrahydro-4,1-benzoxazepin-3-yl]acetate (5.30 g).
MS (FAB) m/z: 407 (M+H)⁺.
¹H-NMR (CDCl3) δ: 1.23 (3H, t, J = 7.1 Hz), 2.79 (1H, dd, J = 16.5, 6.7 Hz), 3.04 (1H, dd, J = 16.5, 6.7 Hz), 3.77 (3H, d, J = 2.4 Hz), 4.11 (2H, q, J = 7.1 Hz), 4.64 (1H, t, J = 6.7 Hz), 6.18 (1H, s), 6.64 (1H, d, J = 2.4 Hz), 7.02 (1H, d, J = 8.3 Hz), 7.08-7.18 (2H, m), 7.24-7.28 (1H, m), 7.30 (1H, dd, J = 8.4, 2.3 Hz), 8.06 (1H, s).

### Referential Example 442

Ethyl 2-[trans-7-chloro-5-(3-fluoro-2-methoxyphenyl)-2-thioxo-1,2,3,5-tetrahydro-4,1-benzoxazepin-3-yl]acetate

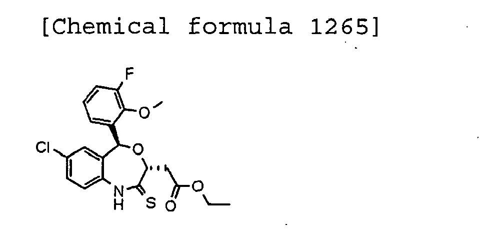

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (3.15 g) was obtained from ethyl 2-[trans-7-chloro-5-(3-fluoro-2-methoxyphenyl)-2-oxo-1,2,3,5-tetrahydro-4,1-benzoxazepin-3-yl]acetate (2.80 g).
MS (EI) m/z: 423 M⁺.
¹H-NMR (CDCl3) δ: 1.25 (3H, t, J = 7.1 Hz), 2.96 (1H, dd, J = 16.6, 6.6 Hz), 3.26 (1H, dd, J = 16.6, 6.6 Hz), 3.75 (3H, d, J = 2.7 Hz), 4.14 (2H, q, J = 7.1 Hz), 4.70 (1H, t, J = 6.6 Hz), 6.09 (1H, s), 6.62 (1H, br s), 7.05-7.18 (3H, m), 7.28-7.37 (2H, m), 9.72 (1H, br s).

### Example 629

Ethyl 2-[trans-8-chloro-6-(3-fluoro-2-methoxyphenyl)-1-(trifluoromethyl)-4H,6H-[1,2,4]triazolo[4,3-a][4,1]benzoxazepin-4-yl]acetate

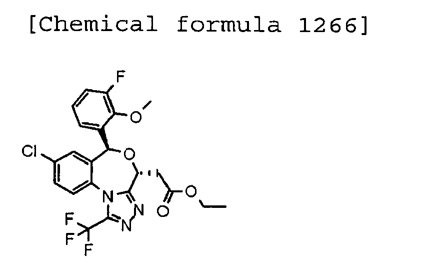

In a similar manner to that employed for the synthesis of ethyl 2-[trans-8-chloro-6-(2-fluoro-3-methoxyphenyl)-1-(trifluoromethyl)-4H,6H-[1,2,4]triazolo[4,3-a] [4,1]benzothiazepin-4-yl]acetate, the title compound (3.33 g) was obtained from ethyl 2-[trans-7-chloro-5-(3-fluoro-2-methoxyphenyl)-2-thioxo-1,2,3,5-tetrahydro-4,1-benzoxazepin-3-yl]acetate(3.15 g).
MS (EI) m/z: 499 M⁺.
¹H-NMR (CDCl3) δ: 1.28 (3H, t, J = 7.1 Hz), 3.24 (1H, dd, J = 16.8, 7.1 Hz), 3.50 (1H, dd, J = 16.8, 6.6 Hz), 3.59 (3H, d, J = 2.7 Hz), 4.19 (2H, q, J = 7.1 Hz), 4.94 (1H, dd, J = 7.1, 6.6 Hz), 5.51 (1H, s), 6.81 (1H, s), 7.12-7.17 (2H, m), 7.36-7.40 (1H, m), 7.53-7.55 (2H, m).

### Example 630

2-[trans-8-Chloro-6-(3-fluoro-2-methoxyphenyl)-1-(trifluoromethyl)-4H,6H-[1,2,4]triazolo[4,3-a][4,1]benzoxazepin-4-yl]acetic acid

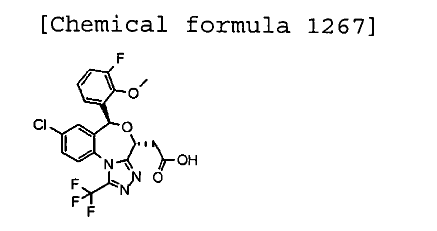

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (89 mg) was obtained from ethyl 2-[trans-8-chloro-6-(3-fluoro-2-methoxyphenyl)-1-(trifluoromethyl)-4H,6H-[1,2,4]triazolo[4,3-a] [4,1]benzoxazepin-4-yl]acetate (100 mg).
MS (FAB) m/z: 472 (M+H)⁺.
¹H-NMR (CDCl3) δ: 3.31 (1H, dd, J = 17.2, 6.7 Hz), 3.58 (1H, dd, J = 17.2, 6.7 Hz), 3.60 (3H, d, J = 2.4 Hz), 4.91 (1H, t, J = 6.7 Hz), 5.52 (1H, s), 6.82 (1H, s), 7.12-7.19 (2H, m), 7.36-7.42 (1H, m), 7.55 (2H, br s) .
Elemental analysis for: C₂₀H₁₄ClF₄N₃O₄·0.5H₂O
Calculated: C, 49.12; H, 3.08; N, 8.51
Found: C, 48.93; H, 2.80; N, 8.28.

### Referential Example 443

3-Chloro-2-methoxybenzaldehyde

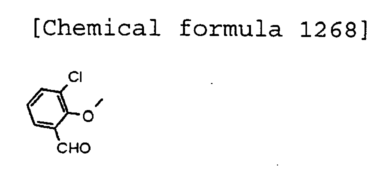

3-Chlorosalicylbenzaldehyde (4.11 g) synthesized in accordance with the process described in the document (Acta. Chem. Scand. 53, 258-262(1999)) was dissolved in N,N-dimethylformamide (100 mL). To the resulting solution were added anhydrous potassium carbonate (6.18 g) and methyl iodide (8.32 mL). The resulting mixture was stirred at 50°C for 2 hours. After cooling to room temperature, ice water and diethyl ether were added to the reaction mixture and the layers separated. The organic layer was dried over anhydrous magnesium sulfate. The solvent was distilled off to give the title compound (4.28 g).
MS (EI) m/z: 170 M⁺.
¹H-NMR (CDCl3) δ: 4.02 (3H, s), 7.19 (1H, td, J = 7.9, 0.7 Hz), 7.64 (1H, dd, J = 7.9, 1.6 Hz), 7.76 (1H, dd, J = 7.8, 1.7 Hz), 10.38 (1H, d, J = 0.7 Hz).

### Referential Example 444

tert-Butyl 4-chloro-2-[(3-chloro-2-methoxyphenyl)(hydroxy)methyl]phenylcarbamate

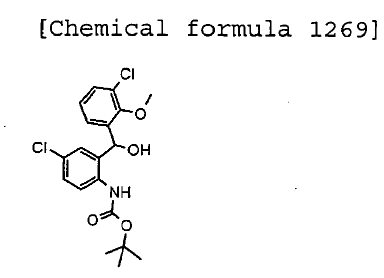

In a similar manner to that employed for the synthesis of tert-butyl 4-chloro-2-{hydroxy[2-(trifluoromethoxy)phenyl]methyl}phenylcarbamate, the title compound (4.92 g) was obtained from tert-butyl 4-chlorophenylcarbamate (5.65 g) and 3-chloro-2-methoxybenzaldehyde (4.28 g).
MS (FAB) m/z: 397 (M+H)⁺.
¹H-NMR (CDCl3) δ: 1.49 (9H, s), 3.28-3.30 (1H, m), 3.82 (3H, s), 6.06-6.11 (1H, m), 7.06-7.13 (2H, m), 7.22-7.29 (2H, m), 7.33-7.38 (1H, m), 7.79 (2H, br s).

### Referential Example 445

(2-Amino-5-chlorophenyl)(3-chloro-2-methoxyphenyl)methanol

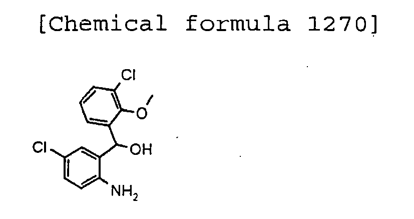

In a similar manner to that employed for the synthesis of 2-amino-5-chloro-3-[(2,3-dimethoxyphenyl)(hydroxy)methyl]pyridine, the title compound (4.80 g) was obtained from tert-butyl 4-chloro-2-[(3-chloro-2-methoxyphenyl)(hydroxy)methyl]phenylcarbamate (4.91 g).
MS (EI) m/z: 297 M⁺.
¹H-NMR (CDCl3) δ: 3.86 (3H, s), 6.08 (1H, s), 6..61 (1H, d, J = 8.3 Hz), 7.06 (1H, dd, J = 8.3, 2.5 Hz), 7.08-7.11 (2H, m), 7.24 (1H, dd, J = 7.8, 1.5 Hz), 7.36 (1H, dd, J = 8.0, 1.5 Hz).

### Referential Example 446

{5-chloro-2-[(2,4-dimethoxybenzyl)amino]phenyl}(3-chloro-2-methoxyphenyl)methanol

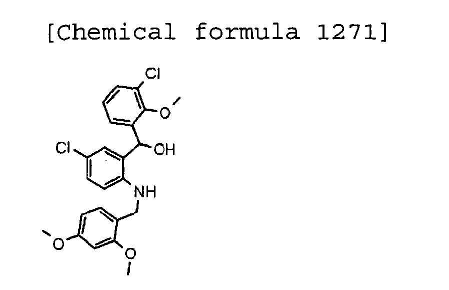

In a similar manner to that employed for the synthesis of {5-chloro-2-[(2,4-dimethoxybenzyl)amino]phenyl}(2,3-dimethoxyphenyl)methanol, the title compound (5.04 g) was obtained from (2-amino-5-chlorophenyl)(3-chloro-2-methoxyphenyl)methanol (4.80 g).
MS (EI) m/z: 447 M⁺.
¹H-NMR (CDCl3) δ: 2.81 (1H, br s), 3.76 (6H, s), 3.79 (3H, s), 4.22 (2H, s), 5.07 (1H, br s), 6.06 (1H, s), 6.39 (1H, dd, J = 8.3, 2.4 Hz), 6.42-6.45 (1H, m), 6.62 (1H, d, J = 8.8 Hz), 6.98-7.12 (4H, m), 7.20 (1H, d, J = 7.8 Hz), 7.35 (1H, d, J = 7.8 Hz).

### Referential Example 447

Ethyl 2-[trans-7-chloro-1-(2,4-dimethoxybenzyl)-5-(3-chloro-2-methoxyphenyl)-2-oxo-1,2,3,5-tetrahydro-4,1-benzoxazepin-3-yl]acetate

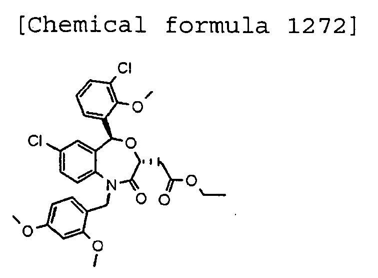

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (5.45 g) was obtained from 5-chloro-2-[(2,4-dimethoxybenzyl)amino]phenyl}(3-chloro-2-methoxyphenyl)methanol (5.04 g).
MS (EI) m/z: 573 M⁺.
¹H-NMR (CDCl3) δ: 1.25 (3H, t, J = 7.1 Hz), 2.79 (1H, dd, J = 16.6, 6.1 Hz), 3.09 (1H, dd, J = 16.6, 7.3 Hz), 3.14 (3H, s), 3.67 (3H, s), 3.77 (3H, s), 4.14 (2H, q, J = 7.1 Hz), 4.48 (1H, dd, J = 7.3, 6.1 Hz), 4.84 (1H, d, J = 15.1 Hz), 5.52 (1H, d, J = 15.1 Hz), 5.95 (1H, s), 6.39-6.43 (2H, m), 6.49 (1H, d, J = 2.2 Hz), 7.16 (1H, t, J = 7.8 Hz), 7.23-7.41 (4H, m), 7.48 (1H, d, J = 7.8 Hz).

### Referential Example 448

Ethyl 2-[trans-7-chloro-5-(3-chloro-2-methoxyphenyl)-2-oxo-1,2,3,5-tetrahydro-4,1-benzoxazepin-3-yl]acetate

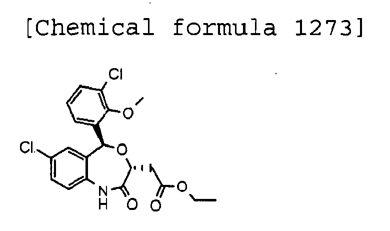

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (3.05 g) was obtained from ethyl 2-[trans-7-chloro-1-(2,4-dimethoxybenzyl)-5-(3-chloro-2-methoxyphenyl)-2-oxo-1,2,3,5-tetrahydro-4,1-benzoxazepin-3-yl]acetate (5.45 g).
MS (EI) m/z: 423 M⁺.
¹H-NMR (CDCl3) δ: 1.22 (3H, t, J = 7.2 Hz), 2.79 (1H, dd, J = 16.6, 6.6 Hz), 3.04 (1H, dd, J = 16.6, 6.6 Hz), 3.67 (3H, s), 4.11 (2H, q, J = 7.2 Hz), 4.65 (1H, t, J = 6.6 Hz), 6.21 (1H, s), 6.63 (1H, d, J = 2.2 Hz), 7.03 (1H, d, J = 8.5 Hz), 7.18 (1H, t, J = 7.8 Hz), 7.31 (1H, dd, J = 8.5, 2.2 Hz), 7.41-7.46 (2H, m), 8.17 (1H, s).

### Referential Example 449

Ethyl 2-[trans-7-chloro-5-(3-chloro-2-methoxyphenyl)-2-thioxo-1,2,3,5-tetrahydro-4,1-benzoxazepin-3-yl]acetate

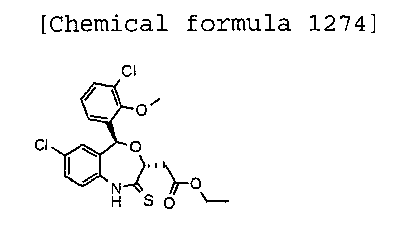

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (3.06 g) was obtained from ethyl 2-[trans-7-chloro-5-(3-chloro-2-methoxyphenyl)-2-oxo-1,2,3,5-tetrahydro-4,1-benzoxazepin-3-yl] acetate (3.05 g).
MS (EI) m/z: 439 M⁺.
¹H-NMR (CDCl3) δ: 1.2.4 (3H, t, J = 7.1 Hz), 2.95 (1H, dd, J = 16.6, 6.6 Hz), 3.25 (1H, dd, J = 16.6, 6.6 Hz), 3.64 (3H, s), 4.13 (2H, q, J = 7.1 Hz), 4.71 (1H, t, J = 6.6 Hz), 6.12 (1H, s), 6.62 (1H, d, J = 2.2 Hz), 7.08 (1H, d, J = 8.5 Hz), 7.19 (1H, t, J = 7.8 Hz), 7.36 (1H, dd, J = 8.5, 2.2 Hz), 7.42-7.49 (2H, m), 9.58 (1H, br s).

### Example 631

Ethyl 2-[trans-8-chloro-6-(3-chloro-2-methoxyphenyl)-1-(trifluoromethyl)-4H,6H-[1,2,4]triazolo[4,3-a] [4,1]benzoxazepin-4-yl]acetate

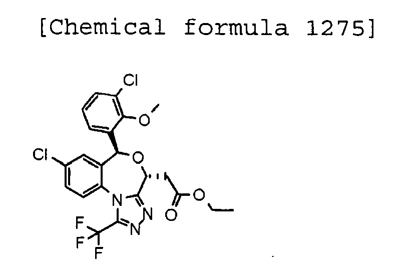

In a similar manner to that employed for the synthesis of ethyl 2-[trans-8-chloro-6-(2-fluoro-3-methoxyphenyl)-1-(trifluoromethyl)-4H,6H-[1,2,4]triazolo[4,3-a] [4,1]benzothiazepin-4-yl]acetate, the title compound (3.14 g) was obtained from ethyl 2-[trans-7-chloro-5-(3-chloro-2-methoxyphenyl)-2-thioxo-1,2,3,5-tetrahydro-4,1-benzoxazepin-3-yl]acetate (3.06 g).
MS (EI) m/z: 515 M⁺.
¹H-NMR (CDCl3) δ: 1.28 (3H, t, J = 7.1 Hz), 3.24 (1H, dd, J = 16.7, 7.2 Hz), 3.44 (3H, s), 3.50 (1H, dd, J = 16.7, 6:5 Hz), 4.19 (2H, q, J = 7.1 Hz), 4.94 (1H, dd, J = 7.2, 6.5 Hz), 5.53 (1H, s), 6.82 (1H, s), 7.22 (1H, t, J = 8.0 Hz), 7.44 (1H, dd, J = 8.0, 1.5 Hz), 7.53-7.58 (3H, m).

### Example 632

2-[trans-8-Chloro-6-(3-chloro-2-methoxyphenyl)-1-(trifluoromethyl)-4H,6H-[1,2,4]triazolo[4,3-a][4,1]benzoxazepin-4-yl]acetic acid

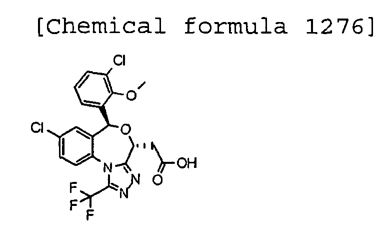

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a] [4,1]benzothiazepin-4-yl]methyl}-1,3-thiazol-5-yl)propanoic acid, the title compound (100 mg) was obtained from ethyl 2-[trans-8-chloro-6-(3-chloro-2-methoxyphenyl)-1-(trifluoromethyl)-4H,6H-[1,2,4]triazolo[4,3-a] [4,1]benzoxazepin-4-yl]acetate (100 mg).
MS (EI) m/z: 487 M⁺.
¹H-NMR (CDCl3) δ: 3.31 (1H, dd, J = 17.2, 6.7 Hz), 3.45 (3H, s), 3.58 (1H, dd, J = 17.2, 6.7 Hz), 4.91 (1H, t, J = 6.7 Hz), 5.54 (1H, s), 6.83 (1H, s), 7.23 (1H, t, J = 8.0 Hz), 7.45 (1H, dd, J = 8.0, 1.5 Hz), 7.54-7.58 (3H, m). Elemental analysis for: C₂₀H₁₄C₁₂F₃N₃O4·1.0H₂O
Calculated: C, 47.45; H, 3.19; N, 8.30
Found: C, 47.11; H, 3.03; N, 7.95.

### Referential Example 450

{5-Chloro-2-[(2,4-dimethoxybenzyl)amino]phenyl}(2-fluoro-3-methoxyphenyl)methanol

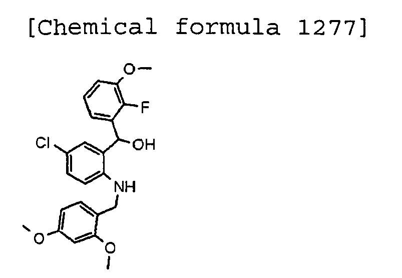

In a similar manner to that employed for the synthesis of {5-chloro-2-[(2,4-dimethoxybenzyl)amino]phenyl}(2,3-dimethoxyphenyl)methanol, the title compound (12.1 g) was obtained from (2-amino-5-chlorophenyl)(2-fluoro-3-methoxyphenyl)methanol (7.34 g).
MS (EI) m/z: 431 M⁺.
¹H-NMR (CDCl3) δ: 3.79 (6H, s), 3.89 (3H, s), 4.24 (2H, s), 6.09 (1H, s), 6.40 (1H, dd, J = 8.8, 2.0 Hz), 6.45 (1H, d, J = 2.0 Hz), 6.63 (1H, d, J = 8.8 Hz), 6.95 (3H, t, J = 7.6 Hz), 7.05-7.11 (3H, m), 7.26 (1H, s).

### Referential Example 451

Ethyl 2-[trans-7-chloro-1-(2,4-dimethoxybenzyl)-5-(2-fluoro-3-methoxyphenyl)-2-oxo-1,2,3,5-tetrahydro-4,1-benzoxazepin-3-yl]acetate

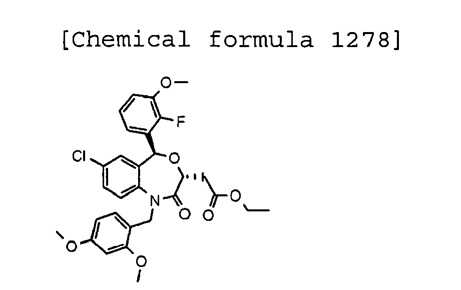

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,l-benzoxazepin-3-yl]acetate, the title compound (11.5 g) was obtained from {5-chloro-2-[(2,4-dimethoxybenzyl)amino]phenyl}(2-fluoro-3-methoxyphenyl)methanol (12.1 g).
MS (FAB) m/z: 558 (M+H)⁺.
¹H-NMR (CDCl3) δ: 1.25 (3H, t, J = 7.1 Hz), 2.77 (1H, dd, J = 16.6, 6.1 Hz), 3.08 (1H, dd, J = 16.6, 7.8 Hz), 3.60 (3H, s), 3.77 (3H, s), 3.88 (3H, s), 4.14 (2H, q, J = 7.1 Hz), 4.47 (1H, dd, J = 7.8, 6.1 Hz), 4.85 (1H, d, J = 14.7 Hz), 5.38 (1H, d, J = 14.7 Hz), 5.89 (1H, s), 6.38 (1H, d, J = 2.2 Hz), 6.41 (1H, dd, J = 8.3, 2.2 Hz), 6.56 (1H, d, J = 2.0 Hz), 6.95-7.02 (1H, m), 7.13-7.17 (2H, m), 7.24-7.27 (1H, m), 7.28-7.32 (2H, m).

### Referential Example 452

Ethyl 2-[trans-7-chloro-5-(2-fluoro-3-methoxyphenyl)-2-oxo-1,2,3,5-tetrahydro-4,1-benzoxazepin-3-yl]acetate

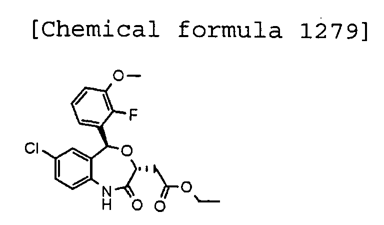

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (6.05 g) was obtained from ethyl 2-[trans-7-chloro-1-(2,4-dimethoxybenzyl)-5-(2-fluoro-3-methoxyphenyl)-2-oxo-1,2,3,5-tetrahydro-4,1-benzoxazepin-3-yl]acetate (11.5 g).
MS (EI) m/z: 407 M⁺.
¹H-NMR (CDCl3) δ: 1.23 (3H, t, J = 7.1 Hz), 2.79 (1H, dd, J = 16.4, 6.6 Hz), 3.03 (1H, dd, J = 16.4, 6.6 Hz), 3.91 (3H, s), 4.12 (2H, q, J = 7.1 Hz), 4.63 (1H, t, J = 6.6 Hz), 6.20 (1H, s), 6.72 (1H, d, J = 2.2 Hz), 7.00-7.07 (3H, m), 7.14-7.20 (1H, m), 7.31 (1H, dd, J = 8.5, 2.4 Hz), 8.14 (1H, s).

### Referential Example 453

Ethyl 2-[trans-7-chloro-5-(2-fluoro-3-methoxyphenyl)-2-thioxo-1,2,3,5-tetrahydro-4,1-benzoxazepin-3-yl]acetate

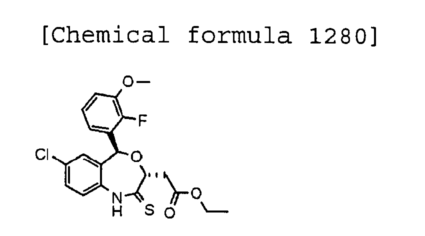

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (5.81 g) was obtained from ethyl 2-[trans-7-chloro-5-(2-fluoro-3-methoxyphenyl)-2-oxo-1,2,3,5-tetrahydro-4,1-benzoxazepin-3-yl] acetate (6.05 g).
MS (EI) m/z: 423 M⁺.
¹H-NMR (CDCl3) δ: 1.25 (3H, t, J = 7.1 Hz), 2.96 (1H, dd, J = 16.6, 6.6 Hz), 3.26 (1H, dd, J = 16.6, 6.6 Hz), 3.91 (3H, s), 4.14 (2H, q, J = 7.1 Hz), 4.70 (1H, t, J = 6.6 Hz), 6.11 (1H, s), 6.69 (1H, s), 6.99-7.23 (4H, m), 7.37 (1H, dd, J = 8.3, 2.4 Hz), 9.59 (1H, s).

### Example 633

Ethyl 2-[trans-8-chloro-6-(2-fluoro-3-methoxyphenyl)-1-(trifluoromethyl)-4H,6H-[1,2,4]triazolo[4,3-a] [4,1]benzoxazepin-4-yl]acetate

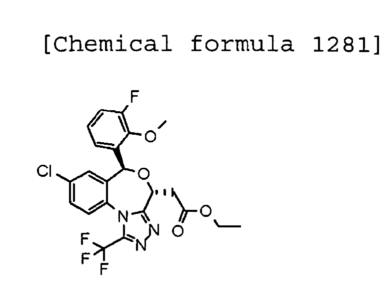

In a similar manner to that employed for the synthesis of ethyl 2-[trans-8-chloro-6-(2-fluoro-3-methoxyphenyl)-1-(trifluoromethyl)-4H,6H-[1,2,4]triazolo[4,3-a] [4,1]benzothiazepin-4-yl]acetate, the title compound (5.29 g) was obtained from ethyl 2-[trans-7-chloro-5-(2-fluoro-3-methoxyphenyl)-2-thioxo-1,2,3,5-tetrahydro-4,1-benzoxazepin-3-yl]acetate (5.81 g).
MS (EI) m/z: 499 M⁺.
¹H-NMR (CDCl3) δ: 1.29 (3H, t, J = 7.1 Hz), 3.24 (1H, dd, J = 16.7, 6.8 Hz), 3.49 (1H, dd, J = 16.7, 6.8 Hz), 3.89 (3H, s), 4.19 (2H, q, J = 7.1 Hz), 4.96 (1H, t, J = 6.8 Hz), 5.58 (1H, s), 6.87 (1H, s), 7.01-7.08 (1H, m), 7.18-7.25 (2H, m), 7.50-7.59 (2H, m).

### Example 634

Ethyl 2-[(4R,6S)-8-chloro-6-(2-fluoro-3-methoxyphenyl)-1-(trifluoromethyl)-4H,6H-[1,2,4]triazolo[4,3-a][4,1]benzoxazepin-4-yl]acetate(isomer A)

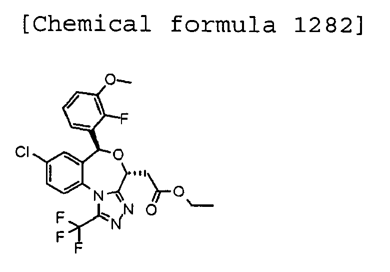

Ethyl 2-[(4S,6R)-8-chloro-6-(2-fluoro-3-methoxyphenyl)-1-(trifluoromethyl)-4H,6H-[1,2,4]triazolo[4,3-a][4,1]benzoxazepin-4-yl]acetate(isomer B)

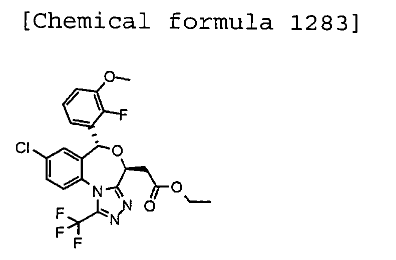

Ethyl 2-[trans-8-chloro-6-(2-fluoro-3-methoxyphenyl)-1-(trifluoromethyl)-4H,6H-[1,2,4]triazolo[4,3-a][4,1]benzoxazepin-4-yl]acetate (1.50 g) was optically resolved by a CHIRALPAK AD column (Daicel Chemical Industries, hexane:ethanol = 90:10, flow rate: 50 mL/min) to give ethyl 2-[(4R,6S)-8-chloro-6-(2-fluoro-3-methoxyphenyl)-1-(trifluoromethyl)-4H,6H-[1,2,4]triazolo[4,3-a] [4,1]benzoxazepin-4-yl]acetate (isomer A, retention time: 35 minutes, 742 mg) and ethyl 2-[(4S,6R)-8-chloro-6-(2-fluoro-3-methoxyphenyl)-1-(trifluoromethyl)-4H,6H-[1,2,4]triazolo[4,3-a][4,1]benzoxazepin-4-yl]acetate (isomer B, retention time: 65 minutes, 740 mg). 2-[(4R,6S)-8-Chloro-6-(2-fluoro-3-methoxyphenyl)-1-(trifluoromethyl)-4H,6H-[1,2,4]triazolo[4,3-a][4,1]benzoxazepin-4-yl]acetate (isomer A)
MS (EI) m/z: 499 M⁺.
¹H-NMR (CDCl3) δ: 1.29 (3H, t, J = 7.1 Hz), 3.24 (1H, dd, J = 16.7, 6.8 Hz), 3.49 (1H, dd, J = 16.7, 6.8 Hz), 3.89 (3H, s), 4.19 (2H, q, J = 7.1 Hz), 4.96 (1H, t, J = 6.8 Hz), 5.58 (1H, s), 6.87 (1H, s), 7.01-7.08 (1H, m), 7.18-7.25 (2H, m), 7.50-7.59 (2H, m).
Ethyl 2-[(4S,6R)-8-chloro-6-(2-fluoro-3-methoxyphenyl)-1-(trifluoromethyl)-4H,6H-[1,2,4]triazolo[4,3-a][4,1]benzoxazepin-4-yl]acetate (isomer B)
MS (EI) m/z: 499 M⁺.
¹H-NMR (CDCl3) δ: 1.29 (3H, t, J = 7.1 Hz), 3.24 (1H, dd, J = 16.7, 6.8 Hz), 3.49 (1H, dd, J =16.7, 6.8 Hz), 3.89 (3H, s), 4.19 (2H, q, J = 7.1 Hz), 4.96 (1H, t, J = 6.8 Hz), 5.58 (1H, s), 6.87 (1H, s), 7.01-7.08 (1H, m), 7.18-7.25 (2H, m), 7.50-7.59 (2H, m).

### Example 635

2-[(4R,6S)-8-Chloro-6-(2-fluoro-3-methoxyphenyl)-1-(trifluoromethyl)-4H,6H-[1,2,4]triazolo[4,3-a][4,1]benzoxazepin-4-yl]acetic acid

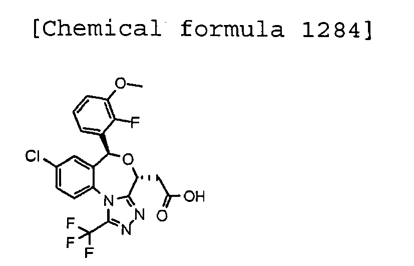

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (618 mg) was obtained from ethyl 2-[(4R,6S)-8-chloro-6-(2-fluoro-3-methoxyphenyl)-1-(trifluoromethyl)-4H,6H-[1,2,4]triazolo[4,3-a][4,1]benzoxazepin-4-yl]acetate (isomer A, 742 mg).
MS (EI) m/z: 471 M⁺.
¹H-NMR (CDCl3) δ: 3.31 (1H, dd, J = 17.1, 6.7 Hz), 3.57 (1H, dd, J = 17.1, 6.7 Hz), 3.89 (3H, s), 4.92 (1H, t, J = 6.7 Hz), 5.58 (1H, s), 6.88 (1H, d, J = 2.0 Hz), 7.01-7.08 (1H, m), 7.18-7.24 (2H, m), 7.51-7.59 (2H, m).
Elemental analysis for: C₂₀H₁₄ClF₄N₃O₄·0.25H₂O
Calculated: C, 50.43; H, 3.07; N, 8.82
Found: C, 50.53; H, 3.10; N, 8.70.

### Example 636

2-[(4S,6R)-8-Chloro-6-(2-fluoro-3-methoxyphenyl)-1-(trifluoromethyl)-4H,6H-[1,2,4]triazolo[4,3-a][4,1]benzoxazepin-4-yl]acetic acid

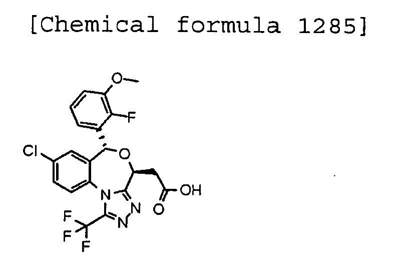

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (667 mg) was obtained from ethyl 2-[(4S,6R)-8-chloro-6-(2-fluoro-3-methoxyphenyl)-1-(trifluoromethyl)-4H,6H-[1,2,4]triazolo[4,3-a][4,1]benzoxazepin-4-yl]acetate (isomer B, 740 mg).
MS (EI) m/z: 471 M⁺. ¹H-NMR (CDCl3) δ: 3.31 (1H, dd, J = 17.1, 6.7 Hz), 3.57 (1H, dd, J = 17.1, 6.7 Hz), 3.89 (3H, s), 4.92 (1H, t, J = 6.7 Hz), 5.58 (1H, s), 6.88 (1H, d, J = 2.0 Hz), 7.01-7.08 (1H, m), 7.18-7.24 (2H, m), 7.51-7.59 (2H, m).
Elemental analysis for: C₂₀H₁₄ClF₄N₃O₄
Calculated: C, 50.92; H, 2.99; N, 8.91
Found: C, 50.79; H, 3.18; N, 8.66.

### Referential Example 454

3-Methoxy-2-methylbenzaldehyde

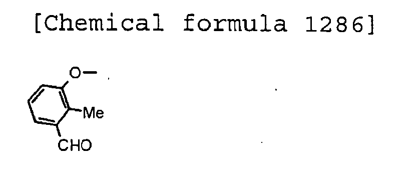

3-Methoxy-2-methylbenzoic acid (13.8 g) was dissolved in tetrahydrofuran (300 mL). To the resulting solution was added dropwise a tetrahydrofuran solution (a 1.0M solution, 100 mL) of a borane-tetrahydrofuran complex under ice cooling. The reaction mixture was heated under reflux overnight. After cooling to a temperature of ice cooling, water was added dropwise to terminate the reaction. To the reaction mixture were added ethyl acetate and 1N hydrochloric acid and the layers separated. The organic layer was washed successively with a saturated aqueous solution of sodium hydrogen carbonate and saturated brine and dried over anhydrous magnesium sulfate. The solvent was distilled off to give a crudely purified product of (3-methoxy-2-methylphenyl)methanol. Oxalyl chloride (10.7 mL) was dissolved in dichloromethane (500 mL). At -78°C, dimethylsulfoxide (17.8 mL) was added dropwise to the resulting solution. After stirring at -78°C for 15 minutes, a dichloromethane solution (50 mL) of the crudely purified product of (3-methoxy-2-methylphenyl)methanol was added dropwise. The reaction mixture was warmed to -40°C and then stirred for 2 hours. Triethylamine (57.7 mL) was added dropwise and the reaction mixture was warmed to room temperature. To the reaction mixture were added ethyl acetate and 1N hydrochloric acid and the layers separated. The organic layer was washed successively with a saturated aqueous solution of sodium hydrogen carbonate and saturated brine and dried over anhydrous magnesium sulfate. The solvent was distilled off and the residue was purified by silica gel column chromatography (hexane:ethyl acetate=30:1) to give the title compound (11.7 g).
MS (EI) m/z: 150 M⁺.
¹H-NMR (CDCl3) δ: 2.54 (3H, s), 3.87 (3H, s), 7.08 (1H, d, J = 7.8 Hz), 7.31 (1H, t, J = 7.8 Hz), 7.43 (1H, d, J = 7.8 Hz), 10.33 (1H, s).

### Referential Example 455

tert-Butyl 4-(trifluoromethyl)phenylcarbamate

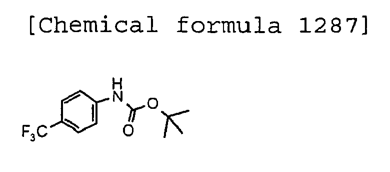

4-(Trifluoromethyl)aniline (50.0 mL) was dissolved in t-butanol (800 mL). To the resulting solution was added di-t-butyl dicarbonate (104 g). The resulting mixture was stirred overnight at 50°C. The reaction mixture was concentrated under reduced pressure. Hexane was added to the residue, followed by slurry washing to give the title compound (95.0 g).
MS (EI) m/z: 161 M⁺.
¹H-NMR (CDCl3) δ: 1.53 (9H, s), 6.60 (1H, br s), 7.41-7.56 (4H, m).

### Referential Example 456

tert-Butyl 4-chloro-2-[hydroxy(3-methoxy-2-methylphenyl)methyl]phenylcarbamate

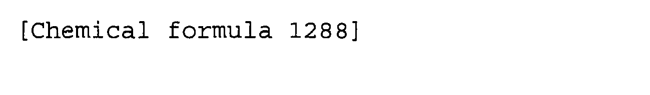

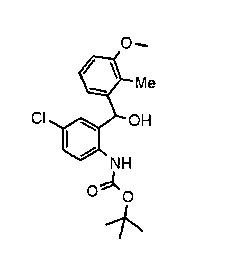

tert-Butyl 4-(trifluoromethyl)phenylcarbamate (13.0 g) was dissolved in anhydrous tetrahydrofuran (200 mL). At -78°C, sec-butyl lithium (a 1.00 M solution, 110 mL) was added dropwise. The reaction mixture was warmed to -20°C, stirred for one hour and then, cooled to -78°C again. The reaction mixture was thus obtained as a lithium salt solution of tert-butyl 4-(trifluoromethyl)phenylcarbamate. On the other hand, 3-methoxy-2-methylbenzaldehyde (9.00 g) was dissolved in anhydrous tetrahydrofuran (100 mL). After cooling to -20°C, the lithium salt solution of tert-butyl 4-(trifluoromethyl)phenylcarbamate was added dropwise to the resulting solution. After the reaction mixture was stirred for 20 minutes, a saturated aqueous solution of ammonium chloride was added to terminate the reaction. To the reaction mixture was added ethyl acetate and the layers separated. The organic layer was dried over anhydrous sodium sulfate. The solvent was distilled off under reduced pressure. The residue was purified by silica gel column chromatography (hexane:ethyl acetate= from 40:1 to 20:1) to give the title compound (10.8 g).
MS (EI) m/z: 411 M⁺.
¹H-NMR (CDCl3) δ: 1.52 (9H, s), 2.08 (3H, s), 3.86 (3H, s), 6.11 (1H, d, J = 3.7 Hz), 6.90 (1H, d, J = 8.3 Hz), 7.03 (1H, d, J = 8.3 Hz), 7.06 (1H, s), 7.23-7.29 (1H, m), 7.53 (1H, d, J = 8.5 Hz), 7.97 (1H, br s), 8.12 (1H, d, J = 8.5 Hz).

### Referential Example 457

[2-[(2,4-Dimethoxybenzyl)amino]-5-(trifluoromethyl)phenyl](3-methoxy-2-methylphenyl)methanol

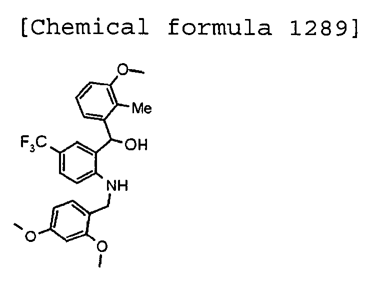

In a similar manner to that employed for the synthesis of 2-amino-5-chloro-3-[(2,3-dimethoxyphenyl)(hydroxy)methyl]pyridine and {5-chloro-2-[(2,4-dimethoxybenzyl)amino]phenyl}(2,3-dimethoxyphenyl)methanol, the title compound (1.74 g) was obtained from tert-butyl 4-chloro-2-[hydroxy(3-methoxy-2-methylphenyl)methyl]phenylcarbamate (2.47 g).
MS (EI) m/z: 461 M⁺.
¹H-NMR (CDCl3) δ: 2.09 (3H, s), 3.77 (3H, s), 3.79 (3H, s), 3.85 (3H, s), 4.30 (2H, s), 5.30 (1H, br s), 6.00 (1H, s), 6.40 (1H, dd, J = 8.3, 2.2 Hz), 6.45 (1H, d, J = 2.2 Hz), 6.72 (1H, d, J = 8.5 Hz), 6.87 (1H, d, J = 8.5 Hz), 6.99 (1H, d, J = 7.6 Hz), 7.05 (1H, d, J = 8.3 Hz), 7.09 (1H, s), 7.21 (1H, t, J = 7.6 Hz), 7.38 (1H, d, J = 8.3 Hz).

### Referential Example 458

Ethyl 2-[trans-1-(2,4-dimethoxybenzyl)-5-(3-methoxy-2-methylphenyl)-2-oxo-7-(trifluoromethyl)-1,2,3,5-tetrahydro-4,1-benzoxazepin-3-yl]acetate

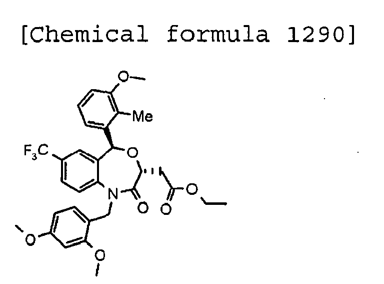

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (927 mg) was obtained from [2-[(2,4-dimethoxybenzyl)amino]-5-(trifluoromethyl)phenyl](3-methoxy-2-methylphenyl)methanol (1.74 g).
MS (EI) m/z: 587 M⁺.
¹H-NMR (CDCl3) δ: 1.25 (3H, t, J = 7.1 Hz), 1.35 (3H, s), 2.80 (1H, dd, J = 16.6, 5.5 Hz), 3.13 (1H, dd, J = 16.6, 8.0 Hz), 3.54 (3H, s), 3.76 (3H, s), 3.81 (3H, s), 4.15 (2H, q, J = 7.1 Hz), 4.44 (1H, dd, J = 8.0, 5.5 Hz), 4.94 (1H, d, J = 14.6 Hz), 5.49 (1H, d, J = 14.6 Hz), 5.67 (1H, s), 6.31 (1H, d, J = 2.2 Hz), 6.41 (1H, dd, J = 8.3, 2.2 Hz), 6.76 (1H, s), 6.85 (1H, d, J = 8.3 Hz), 7.18 (1H, d, J = 7.8 Hz), 7.22-7.27 (2H, m), 7.39 (1H, d, J = 8.3 Hz), 7.57 (1H, d, J = 8.3 Hz), 7.62 (1H, d, J = 8.3 Hz).

### Referential Example 459

Ethyl 2-[trans-5-(3-methoxy-2-methylphenyl)-2-oxo-7-(trifluoromethyl)-1,2,3,5-tetrahydro-4,1-benzoxazepin-3-yl] acetate

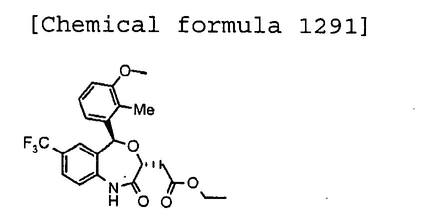

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (589 mg) was obtained from ethyl 2-[trans-1-(2,4-dimethoxybenzyl)-5-(3-methoxy-2-methylphenyl)-2-oxo-7-(trifluoromethyl)-1,2,3,5-tetrahydro-4,1-benzoxazepin-3-yl]acetate (925 mg).
MS (FAB) m/z: 438 (M+H)⁺.
¹H-NMR (CDCl3) δ: 1.21 (3H, t, J = 7.2 Hz), 2.09 (3H, s), 2.82 (1H, dd, J = 16.2, 6.1 Hz), 2.98 (1H, dd, J = 16.2, 6.1 Hz), 3.86 (3H, s), 4.05-4.15 (2H, m), 4.50 (1H, t, J = 6.1 Hz), 6.18 (1H, s), 6.76 (1H, d, J = 8.0 Hz), 6.90 (1H, d, J = 8.3 Hz), 7.10 (1H, s), 7.11 (1H, d, J = 8.3 Hz), 7.18 (1H, t, J = 8.0 Hz), 7.56 (1H, dd, J = 8.3, 2.0 Hz), 8.10 (1H, s).

### Referential Example 460

Ethyl 2-[trans-5-(3-methoxy-2-methylphenyl)-2-thioxo-7-(trifluoromethyl)-1,2,3,5-tetrahydro-4,1-benzoxazepin-3-yl] acetate

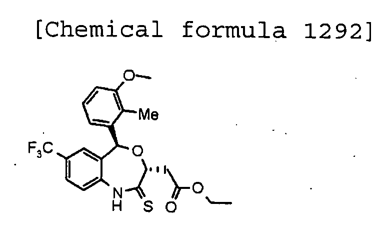

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (527 mg) was obtained from ethyl 2-[trans-5-(3-methoxy-2-methylphenyl)-2-oxo-7-(trifluoromethyl)-1,2,3,5-tetrahydro-4,1-benzoxazepin-3-yl] acetate (585 mg).
MS (FAB) m/z: 454 (M+H)⁺.
¹H-NMR (CDCl3) δ: 1.24 (3H, t, J = 7.1 Hz), 1.93 (3H, s), 2.96 (1H, dd, J = 16.6, 6.5 Hz), 3.27 (1H, dd, J = 16.6, 6.5 Hz), 3.86 (3H, s), 4.12 (2H, q, J = 7.1 Hz), 4.70 (1H, t, J = 6.5 Hz), 6.07 (1H, s), 6.91 (1H, d, J = 8.0 Hz), 7.00 (1H, s), 7.01 (1H, d, J = 8.0 Hz), 7.20-7.27 (2H, m), 7.63 (1H, dd, J = 8. 0, 1. 6 Hz), 9.68 (1H, s).

### Example 637

Ethyl 2-[trans-6-(3-methoxy-2-methylphenyl)-1,8-bis(trifluoromethyl)-4H,6H-[1,2,4]triazolo[4,3-a] [4,1]benzoxazepin-4-yl]acetate

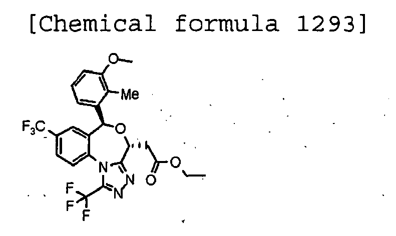

In a similar manner to that employed for the synthesis of methyl 2-[trans-8-chloro-6-(2,3-dimethoxyphenyl)-10-fluoro-1-(trifluoromethyl)-4H,6H-[1,2,4]triazolo[4,3-a] [4,1]benzothiazepin-4-yl]acetate, the title compound (488 mg) was obtained from ethyl 2-[trans-5-(3-methoxy-2-methylphenyl)-2-thioxo-7-(trifluoromethyl)-1,2,3,5-tetrahydro-4,1-benzoxazepin-3-yl]acetate (527 mg).
MS (EI) m/z: 529 M⁺.
¹H-NMR (CDCl3) δ: 1.28 (3H, t, J = 7.1 Hz), 1.64 (3H, s), 3.26 (1H, dd, J = 16.8, 6.7 Hz), 3.51 (1H, dd, J = 16.8, 6.7 Hz), 3.84 (3H, s), 4.19 (2H, q, J = 7.1 Hz), 4.95 (1H, t, J = 6.7 Hz), 5.46 (1H, s), 6.92 (1H, d, J = 8.0 Hz), 7.10 (1H, s), 7.25-7.28 (1H, m), 7.32 (1H, t, J = 8.0 Hz), 7.74 (1H, d, J = 8.5 Hz), 7.85 (1H, d, J = 8.5 Hz).

### Example 638

Ethyl 2-[(4R,6R)-6-(3-methoxy-2-methylphenyl)-1,8-bis(trifluoromethyl)-4H,6H-[1,2,4]triazolo[4,3-a][4,1]benzoxazepin-4-yl]acetate (isomer A)

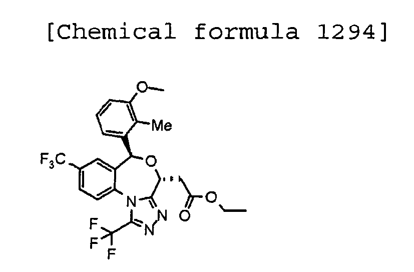

Ethyl 2-[(4S,6S)-6-(3-methoxy-2-methylphenyl)-1,8-bis(trifluoromethyl)-4H,6H-[1,2,4]triazolo[4,3-a][4,1]benzoxazepin-4-yl]acetate (isomer B)

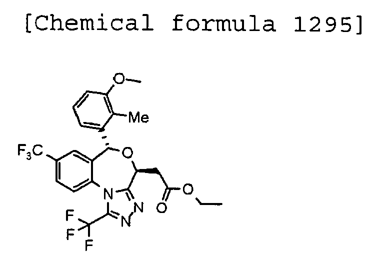

Ethyl 2-[trans-6-(3-methoxy-2-methylphenyl)-1,8-bis(trifluoromethyl)-4H,6H-[1,2,4]triazolo[4,3-a] [4,1]benzoxazepin-4-yl]acetate (485 mg) was optically resolved by CHIRALPAK IA column (Daicel Chemical Industries, hexane:dichloromethane=2:1, flow rate: 10 mL/min) to give ethyl 2-[(4R,6R)-6-(3-methoxy-2-methylphenyl)-1,8-bis(trifluoromethyl)-4H,6H-[1,2,4]triazolo[4,3-a] [4,1]benzoxazepin-4-yl]acetate (isomer A, retention time: 11 min., 174 mg) and ethyl 2-[(4S,6S)-6-(3-methoxy-2-methylphenyl)-1,8-bis(trifluoromethyl)-4H,6H-[1,2,4]triazolo[4,3-a] [4,1]benzoxazepin-4-yl]acetate (isomer B, retention time: 14 min., 198 mg).
Ethyl 2-[(4R,6R)-6-(3-methoxy-2-methylphenyl)-1,8-bis(trifluoromethyl)-4H,6H-[1,2,4]triazolo[4,3-a][4,1]benzoxazepin-4-yl]acetate (isomer A)
MS (EI) m/z: 529 M⁺.
¹H-NMR (CDCl3) δ: 1.28 (3H, t, J = 7.1 Hz), 1.64 (3H, s), 3.26 (1H, dd, J = 16.8, 6.7 Hz), 3.51 (1H, dd, J = 16.8, 6.7 Hz), 3.84 (3H, s), 4.19 (2H, q, J = 7.1 Hz), 4.95 (1H, t, J = 6.7 Hz), 5.46 (1H, s), 6.92 (1H, d, J = 8.0 Hz), 7.10 (1H, s), 7.25-7.28 (1H, m), 7.32 (1H, t, J = 8.0 Hz), 7.74 (1H, d, J = 8.5 Hz), 7.85 (1H, d, J = 8.5 Hz).
Ethyl 2-[(4S,6S)-6-(3-methoxy-2-methylphenyl)-1,8-bis(trifluoromethyl)-4H,6H-[1,2,4]triazolo[4,3-a] [4, 1] benzoxazepin-4-yl] acetate (isomer B)
MS (EI) m/z: 529 M⁺.
¹H-NMR (CDCl3) δ: 1.28 (3H, t, J = 7.1 Hz), 1.64 (3H, s), 3.26 (1H, dd, J = 16.8, 6.7 Hz), 3.51 (1H, dd, J = 16.8, 6.7 Hz), 3.84 (3H, s), 4.19 (2H, q, J = 7.1 Hz), 4.95 (1H, t, J = 6.7 Hz), 5.46 (1H, s), 6.92 (1H, d, J = 8.0 Hz), 7.10 (1H, s), 7.25-7.28 (1H, m), 7.32 (1H, t, J = 8.0 Hz), 7.74 (1H, d, J = 8.5 Hz), 7.85 (1H, d, J = 8.5 Hz).

### Example 639

2-[(4R,6R)-6-(3-Methoxy-2-methylphenyl)-1,8-bis(trifluoromethyl)-4H,6H-[1,2,4]triazolo[4,3-a][4,1]benzoxazepin-4-yl]acetic acid

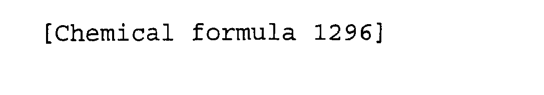

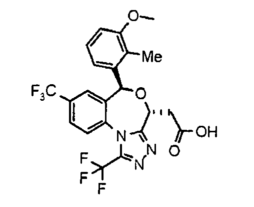

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (123 mg) was obtained from ethyl 2-[(4R,6R)-6-(3-methoxy-2-methylphenyl)-1,8-bis(trifluoromethyl)-4H,6H-[1,2,4]triazolo[4,3-a][4,1]benzoxazepin-4-yl]acetate (isomer A, 174 mg).
MS (FAB) m/z: 502 (M+H)⁺.
¹H-NMR (CDCl3) δ: 1.63 (3H, s), 3.29 (1H, dd, J = 17.1, 6.7 Hz), 3.55 (1H, dd, J = 17.1, 6.7 Hz), 3.83 (3H, s), 4.91 (1H, t, J = 6.7 Hz), 5.44 (1H, s), 6.91 (1H, d, J = 8.0 Hz), 7.09 (1H, s), 7.27-7.34 (2H, m), 7.73 (1H, d, J = 8.3 Hz), 7.83 (1H, d, J = 8.3 Hz).
Elemental analysis for: C₂₂H₁₇F₆N₃O₄·0.75H₂O·0.2CHCl₃ Calculated: C, 49.72; H, 3.48; N, 7.76
Found: C, 50.06; H, 3.19; N, 7.36.

### Example 640

2-[(4S,6S)-6-(3-Methoxy-2-methylphenyl)-1,8-bis(trifluoromethyl)-4H,6H-[1,2,4]triazolo[4,3-a] [4,1]benzoxazepin-4-yl]acetic acid

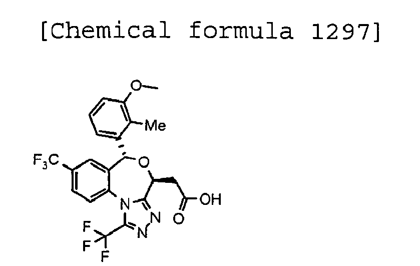

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a] [4,1]benzothiazepin-4-yl]methyl}-1,3-thiazol-5-yl)propanoic acid, the title compound (163 mg) was obtained from ethyl 2-[(4S,6S)-6-(3-methoxy-2-methylphenyl)-1,8-bis(trifluoromethyl)-4H,6H-[1,2,4]triazolo[4,3-a] [4,1]benzoxazepin-4-yl]acetate (isomer B, 198 mg).
MS (FAB) m/z: 502 (M+H)⁺.
¹H-NMR (CDCl3) δ: 1.63 (3H, s), 3.29 (1H, dd, J = 17.1, 6.7 Hz), 3.55 (1H, dd, J = 17.1, 6.7 Hz), 3.83 (3H, s), 4.91 (1H, t, J = 6.7 Hz), 5.44 (1H, s), 6.91 (1H, d, J = 8.0 Hz), 7.09 (1H, s), 7.27-7.34 (2H, m), 7.73 (1H, d, J = 8.3 Hz), 7.83 (1H, d, J = 8.3 Hz).
Elemental analysis for: C₂₂H₁₇F₆N₃O4·0.5H₂O·0.3CHCl₃
Calculated: C, 49.37; H, 3.35; N, 7.64
Found: C, 49.85; H, 3.04; N, 7.20.

### Example 641

Ethyl 2-[trans-1-[chloro(difluoro)methyl]-6-(3-methoxy-2-methylphenyl)-8-(trifluoromethyl)-4H,6H-[1,2,4]triazolo[4,3-a] [4,1]benzoxazepin-4-yl]acetate

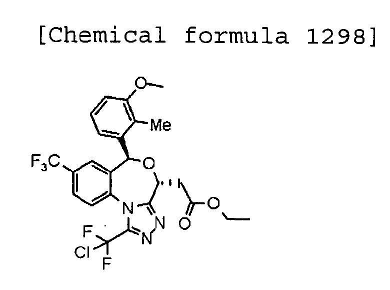

Ethyl 2-[trans-5-(3-methoxy-2-methylphenyl)-2-thioxo-7-(trifluoromethyl)-1,2,3,5-tetrahydro-4,1-benzoxazepin-3-yl]acetate (900 mg) was dissolved in tetrahydrofuran (20 mL). At room temperature, hydrazine hydrate (145 µl) was added. The resulting mixture was stirred at room temperature for one hour. After the reaction mixture was cooled to -20°C, chlorodifluoroacetic anhydride (1.72 mL) was slowly added dropwise. After warming to room temperature, the reaction mixture was stirred overnight. A saturated aqueous solution of sodium hydrogen carbonate and ethyl acetate were added to the reaction mixture and the layers separated. The organic layer was dried over anhydrous sodium sulfate. The solvent was distilled off under reduced pressure. The residue was purified by silica gel column chromatography (hexane:ethyl acetate=4:1) to give a colorless foam. The foam thus obtained was dissolved in isopropanol and the resulting solution was left to stand at room temperature for one hour. Crystals thus precipitated were collected by filtration to give the title compound (854 mg). MS (EI) m/z: 545 M⁺.
¹H-NMR (CDCl3) δ: 1.28 (3H, t, J = 7.1 Hz), 1.65 (3H, s), 3.26 (1H, dd, J = 16.7, 6.7 Hz), 3.51 (1H, dd, J = 16.7, 6.7 Hz), 3.83 (3H, s), 4.16-4.24 (2H, m), 4.92 (1H, t, J = 6.7 Hz), 5.46 (1H, s), 6.92 (1H, d, J = 8.0 Hz), 7.09 (1H, s), 7.26-7.36 (2H, m), 7.77-7.87 (2H, m).

### Example 642

Ethyl 2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate
(isomer A)

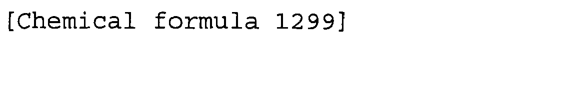

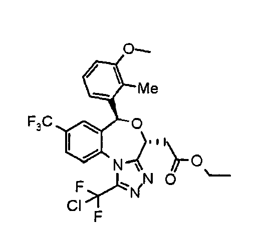

Ethyl 2-[(4S,6S)-1-[chloro(difluoro)methyl]-6-(3-methoxy-2-methylphenyl)-8-(trifluoromethyl)-4H,6H-[1,2,4]triazolo[4,3-a] [4,1]benzoxazepin-4-yl]acetate
(isomer B)

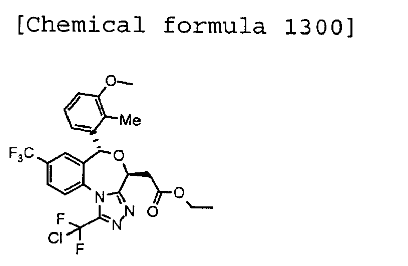

Ethyl 2-[trans-1-[chloro(difluoro)methyl]-6-(3-methoxy-2-methylphenyl)-8-(trifluoromethyl)-4H,6H-[1,2,4]triazolo[4,3-a] [4,1]benzoxazepin-4-yl]acetate (819 mg) was optically resolved by a CHIRALPAK IA column (Daicel Chemical Industries, hexane:dichloromethane=3:1, flow rate: 10 mL/min) to give ethyl 2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate (isomer A, retention time: 13 minutes, 371 mg) and ethyl 2-[(4S,6S)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate (isomer B, retention time: 21 minutes, 380 mg), each as a colorless foam. Ethyl 2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate
(isomer A)
MS (EI) m/z: 545 M⁺.
¹H-NMR (CDCl3) δ: 1.28 (3H, t, J = 7.1 Hz), 1.65 (3H, s), 3.26 (1H, dd, J = 16.7, 6.7 Hz), 3.51 (1H, dd, J = 16.7, 6.7 Hz), 3.83 (3H, s), 4.16-4.24 (2H, m), 4.92 (1H, t, J = 6.7 Hz), 5.46 (1H, s), 6.92 (1H, d, J = 8.0 Hz), 7.09 (1H, s), 7.26-7.36 (2H, m), 7.77-7.87 (2H, m).
Ethyl 2-[(4S,6S)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate
(isomer B)
MS (EI) m/z: 545 M⁺.
¹H-NMR (CDCl3) δ: 1.28 (3H, t, J = 7.1 Hz), 1.65 (3H, s), 3.26 (1H, dd, J = 16.7, 6.7 Hz), 3.51 (1H, dd, J = 16.7, 6.7 Hz), 3.83 (3H, s), 4.16-4.24 (2H, m), 4.92 (1H, t, J = 6.7 Hz), 5.46 (1H, s), 6.92 (1H, d, J = 8.0 Hz), 7.09 (1H, s), 7.26-7.36 (2H, m), 7.77-7.87 (2H, m).

### Example 643

2-[(4R,6R)-1-[Chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetic acid

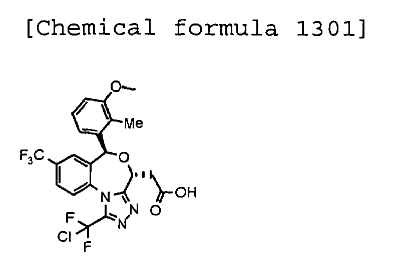

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (244 mg) was obtained from ethyl 2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate (isomer A, 371 mg).
MS (FAB) m/z: 518 (M+H)⁺.
¹H-NMR (CDCl3) δ: 1.64 (3H, s), 3.33 (1H, dd, J = 17.1, 6.7 Hz), 3.60 (1H, dd, J = 17.1, 6.7 Hz), 3.83 (3H, s), 4.89 (1H, t, J = 6.7 Hz), 5.46 (1H, s), 6.93 (1H, d, J = 8.3 Hz), 7.10 (1H, s), 7.28-7.36 (2H, m), 7.80 (2H, d, J = 8.3 Hz), 7.85 (2H, d, J = 8.3 Hz).
Elemental analysis for: C₂₂H₁₇ClF₅N₃O₄·0.25H₂O
Calculated: C, 50.59; H, 3.38; N, 8.04
Found: C, 50.58; H, 3.37; N, 7.87.

### Example 644

2-[(4S,6S)-1-[Chloro(difluoro)methyl]-6-(3-methoxy-2-methylphenyl)-8-(trifluoromethyl)-4H,6H-[1,2,4]triazolo[4,3-a] [4,1]benzoxazepin-4-yl]acetic acid

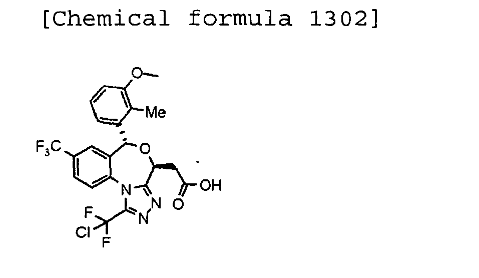

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (273 mg) was obtained from ethyl 2-[(4S,6S)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate (isomer B, 380 mg).
MS (FAB) m/z: 518 (M+H)⁺.
¹H-NMR (CDCl3) δ: 1.64 (3H, s), 3.33 (1H, dd, J = 17.1, 6.7 Hz), 3.60 (1H, dd, J = 17.1, 6.7 Hz), 3.83 (3H, s), 4.89 (1H, t, J = 6.7 Hz), 5.46 (1H, s), 6.93 (1H, d, J = 8.3 Hz), 7.10 (1H, s), 7.28-7.36 (2H, m), 7.80 (2H, d, J = 8.3 Hz), 7.85 (2H, d, J = 8.3 Hz).
Elemental analysis for: C₂₂H₁₇ClF₅N₃O₄
Calculated: C, 51.03; H, 3.31; N, 8.11
Found: C, 50.82; H, 3.45; N, 7.95.

### Example 645

Ethyl 2-[trans-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate

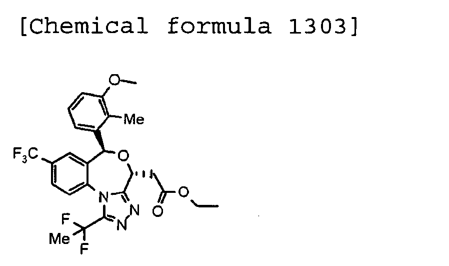

Ethyl 2-[trans-5-(3-methoxy-2-methylphenyl)-2-thioxo-7-(trifluoromethyl)-1,2,3,5-tetrahydro-4,1-benzoxazepin-3-yl]acetate (1.68 g) was dissolved in tetrahydrofuran (40 mL). To the resulting solution was added hydrazine hydrate (270 µl) at room temperature. The resulting mixture was stirred at room temperature for one hour. The reaction mixture was concentrated under reduced pressure. The residue was dissolved in dichloromethane (40 mL). To the resulting solution were added 2,2-difluoropropionic acid (611 mg), 1-ethyl-3-(3-dimethylaminopropyl)carbodiimide hydrochloride (1.41 g), and 1-hydroxybenzotriazole hydrate (567 mg). The resulting mixture was stirred overnight at room temperature. A saturated aqueous solution of sodium hydrogen carbonate was added to the reaction mixture and the layers separated. The organic layer was washed with saturated brine and dried over anhydrous sodium sulfate. The solvent was distilled off under reduced pressure. The residue was purified by silica gel column chromatography (hexane:ethyl acetate=4:1) to give the title compound (535 mg).
MS (FAB) m/z: 526 (M+H)⁺.
¹H-NMR (CDCl3) δ: 1.28 (3H, t, J = 7.1 Hz), 1.63 (3H, s), 2.35 (3H, t, J = 19.3 Hz), 3.25 (1H, dd, J = 16.6, 6.8 Hz), 3.48 (1H, dd, J = 16.7, 6.8 Hz), 3.83 (3H, s), 4.16-4.24 (2H, m), 4.92 (1H, t, J = 6.8 Hz), 5.45 (1H, s), 6.91 (1H, dd, J = 7.8, 1.5 Hz), 7.05 (1H, d, J = 1.5 Hz), 7.27-7.34 (2H, m), 7.80 (1H, dd, J = 8.3, 2.0 Hz), 7.92 (1H, d, J = 8.3 Hz).

### Example 646

Ethyl 2-[(4R,6R)-1-(1,1-difluoroethyl)-6-(3-methoxy-2-methylphenyl)-8-(trifluoromethyl)-4H,6H-[1,2,4]triazolo[4,3-a] [4,1]benzoxazepin-4-yl]acetate
(isomer A)

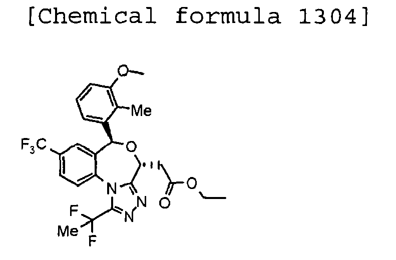

Ethyl 2-[(4S,6S)-1-(1,1-difluoroethyl)-6-(3-methoxy-2-methylphenyl)-8-(trifluoromethyl)-4H,6H-[1,2,4]triazolo[4,3-a] [4,1]benzoxazepin-4-yl]acetate
(isomer B)

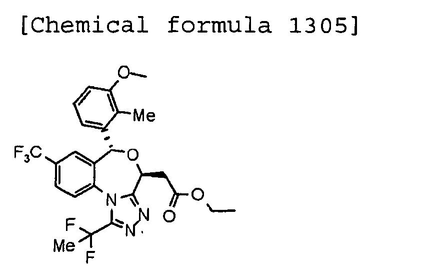

Ethyl 2-[trans-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate (535 mg) was optically resolved by a CHIRALPAK IA column (Daicel Chemical Industries, hexane:dichloromethane=3:2, flow rate: 10 mL/min) to give ethyl 2-[(4R,6R)-1-(1,1-difluoroethyl)-6-(3-methoxy-2-methylphenyl)-8-(trifluoromethyl)-4H,6H-[1,2,4]triazolo[4,3-a] [4, 1] benzoxazepin-4-yl] acetate (isomer A, retention time: 9 min., 252 mg) and ethyl 2-[(4S,6S)-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate (isomer B, retention time: 16 min., 258 mg).
Ethyl 2-[(4R,6R)-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate
(isomer A)
MS (FAB) m/z: 526 (M+H)⁺.
¹H-NMR (CDCl3) δ: 1.28 (3H, t, J = 7.1 Hz), 1.63 (3H, s), 2.35 (3H, t, J = 19.3 Hz), 3.25 (1H, dd, J = 16.6, 6.8 Hz), 3.48 (1H, dd, J = 16.7, 6.8 Hz), 3.83 (3H, s), 4.16-4.24 (2H, m), 4.92 (1H, t, J = 6.8 Hz), 5.45 (1H, s), 6.91 (1H, dd, J = 7.8, 1.5 Hz), 7.05 (1H, d, J = 1.5 Hz), 7.27-7.34 (2H, m), 7.80 (1H, dd, J = 8.3, 2.0 Hz), 7.92 (1H, d, J = 8.3 Hz).
Ethyl 2-[(4S,6S)-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate
(isomer B)
MS (FAB) m/z: 526 (M+H)⁺.
¹H-NMR (CDCl3) δ: 1.28 (3H, t, J = 7.1 Hz), 1.63 (3H, s), 2.35 (3H, t, J = 19.3 Hz), 3.25 (1H, dd, J = 16.6, 6.8 Hz), 3.48 (1H, dd, J = 16.7, 6.8 Hz), 3.83 (3H, s), 4.16-4.24 (2H, m), 4.92 (1H, t, J = 6.8 Hz), 5.45 (1H, s), 6.91 (1H, dd, J = 7.8, 1.5 Hz), 7.05 (1H, d, J = 1.5 Hz), 7.27-7.34 (2H, m), 7.80 (1H, dd, J = 8.3, 2.0 Hz), 7.92 (1H, d, J = 8.3 Hz).

### Example 647

2-[(4R,6R)-1-(1,1-Difluoroethyl)-6-(3-methoxy-2-methylphenyl)-8-(trifluoromethyl)-4H,6H-[1,2,4]triazolo[4,3-a][4,1]benzoxazepin-4-yl]acetic acid

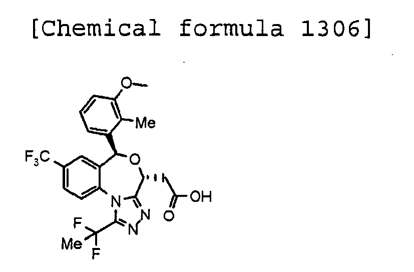

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (149 mg) was obtained from ethyl 2-[(4R,6R)-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate (isomer A, 252 mg).
MS (EI) m/z: 497 M⁺.
¹H-NMR (CDCl3) δ: 1.63 (3H, s), 2.35 (3H, t, J = 19.3 Hz), 3.30 (1H, dd, J = 17.0, 6.8 Hz), 3.57 (1H, dd, J = 17.0, 6.8 Hz), 3.83 (3H, s), 4.88 (1H, t, J = 6.8 Hz), 5.45 (1H, s), 6.91 (1H, dd, J = 7.2, 1.7 Hz), 7.06 (1H, s), 7.27-7.35 (2H, m), 7.81 (1H, d, J = 8.3 Hz), 7.92 (1H, d, J = 8.3 Hz).
Elemental analysis for: C₂₃H₂₀F₅N₃O₄
Calculated: C, 55.54; H, 4.05; N, 8.45
Found: C, 55.42; H, 4.10; N, 8.22.

### Example 648

2-[(4S,6S)-1-(1,1-Difluoroethyl)-6-(3-methoxy-2-methylphenyl)-8-(trifluoromethyl)-4H,6H-[1,2,4]triazolo[4,3-a][4,1]benzoxazepin-4-yl]acetic acid

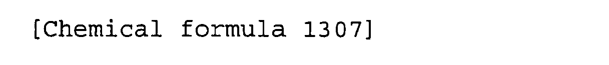

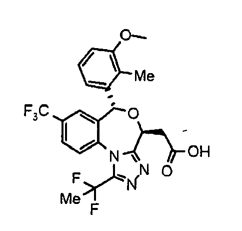

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (163 mg) was obtained from ethyl 2-[(4S,6S)-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate (isomer B, 258 mg).
MS (EI) m/z: 497 M⁺.
¹H-NMR (CDCl3) δ: 1.63 (3H, s), 2.35 (3H, t, J = 19.3 Hz), 3.30 (1H, dd, J = 17.0, 6.8 Hz), 3.57 (1H, dd, J = 17.0, 6.8 Hz), 3.83 (3H, s), 4.88 (1H, t, J = 6.8 Hz), 5.45 (1H, s), 6.91 (1H, dd, J = 7.2, 1.7 Hz), 7.06 (1H, s), 7.27-7.35 (2H, m), 7.81 (1H, d, J = 8.3 Hz), 7.92 (1H, d, J = 8.3 Hz).
Elemental analysis for: C₂₃H₂₀F₅N₃O₄
Calculated: C, 55.54; H, 4.05; N, 8.45
Found: C, 55.57; H, 4.15; N, 7.84.

### Referential Example 461

tert-Butyl 2-[(2-chloro-3-methoxyphenyl)(hydroxy)methyl]-4-(trifluoromethyl)phenylcarbamate

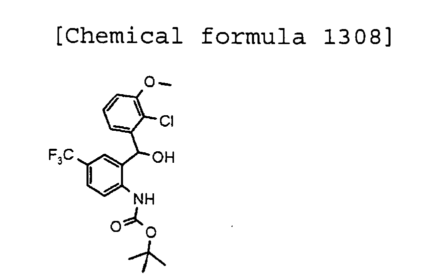

In a similar manner to that employed for the synthesis of tert-butyl 4-chloro-2-{hydroxy[2-(trifluoromethoxy)phenyl]methyl}phenylcarbamate, the title compound (5.90 g) was obtained from tert-butyl 4-(trifluoromethyl)phenylcarbamate (6.37 g) and 2-chloro-3-methoxybenzaldehyde (5.00 g).
MS (EI) m/z: 431 M⁺.
¹H-NMR (CDCl3) δ: 1.51 (9H, s), 3.09 (1H, d, J = 4.2 Hz), 3.93 (3H, s), 6.28 (1H, d, J = 4.2 Hz), 6.96 (1H, d, J = 8.0 Hz), 7.07 (1H, dd, J = 8.0, 1.2 Hz), 7.20 (1H, s), 7.30 (1H, t, J = 8.0 Hz), 7.53 (1H, d, J = 8.5 Hz), 7.92 (1H, s), 8.07 (1H, d, J = 8.5 Hz).

### Referential Example 462

(2-Chloro-3-methoxyphenyl)[2-[(2,4-dimethoxybenzyl)amino]-5-(trifluoromethyl)phenyl]methanol

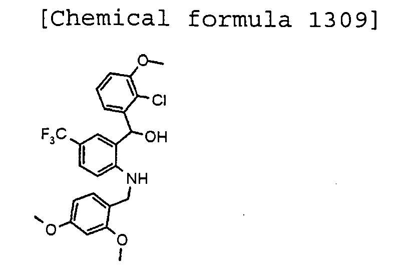

In a similar manner to that employed for the synthesis of 2-amino-5-chloro-3-[(2,3-dimethoxyphenyl)(hydroxy)methyl]pyridine and {5-chloro-2-[(2,4-dimethoxybenzyl)amino]phenyl}(2,3-dimethoxyphenyl)methanol, the title compound (5.82 g) was obtained from tert-butyl 2-[(2-chloro-3-methoxyphenyl)(hydroxy)methyl]-4-(trifluoromethyl)phenylcarbamate (5.90 g).
MS (EI) m/z: 481 M⁺.
¹H-NMR (CDCl3) δ: 2.52 (1H, br s), 3.78 (3H, s), 3.79 (3H, s), 3.93 (3H, s), 4.31 (2H, s), 5.32 (1H, br s), 6.21 (1H, s), 6.40 (1H, dd, J = 8.0, 2.2 Hz), 6.45 (1H, d, J = 2.2 Hz), 6.72 (1H, d, J = 8.5 Hz), 6.94 (1H, dd, J = 8.0, 1.2 Hz), 7.03 (1H, dd, J = 8.0, 1.2 Hz), 7.06 (2H, d, J = 8.5 Hz), 7.17 (1H, d, J = 2.0 Hz), 7.26 (1H, t, J = 8.0 Hz), 7.39 (1H, dd, J = 8.5, 1.7 Hz).

### Referential Example 463

Ethyl 2-[trans-5-(2-chloro-3-methoxyphenyl)-1-(2-hydroxy-4-methoxybenzyl)-2-oxo-7-(trifluoromethyl)-1,2,3,5-tetrahydro-4,1-benzoxazepin-3-yl]acetate

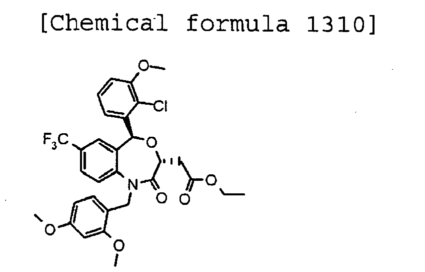

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (5.45 g) was obtained from (2-chloro-3-methoxyphenyl)[2-[(2,4-dimethoxybenzyl)amino]-5-(trifluoromethyl)phenyl]methanol (5.80 g).
MS (FAB) m/z: 608 (M+H)⁺.
¹H-NMR (CDCl3) δ: 1.26 (3H, t, J = 7.1 Hz), 2.81 (1H, dd, J = 16.6, 5.6 Hz), 3:13 (1H, dd, J = 16.6, 7.8 Hz), 3.57 (3H, s), 3.75 (3H, s), 3.90 (3H, s), 4.15 (2H, q, J = 7.1 Hz), 4.45 (1H, dd, J = 7.8, 5.6 Hz), 4.94 (1H, d, J = 14.7 Hz), 5.50 (1H, d, J = 14.7 Hz), 5.93 (1H, s), 6.33 (1H, d, J = 2.2 Hz), 6.40 (1H, dd, J = 8.5, 2.2 Hz), 6.70 (1H, s), 6.96 (1H, dd, J = 8.0, 1.5 Hz), 7.27-7.38 (3H, m), 7.56 (1H, d, J = 8.5 Hz), 7.62 (1H, dd, J = 8.5, 1.7 Hz).

### Referential Example 464

Ethyl 2-[trans-5-(2-chloro-3-methoxyphenyl)-2-oxo-7-(trifluoromethyl)-1,2,3,5-tetrahydro-4,1-benzoxazepin-3-yl] acetate

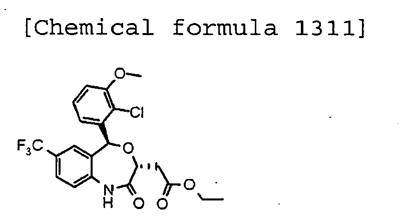

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (2.66 g) was obtained from ethyl 2-[trans-5-(2-chloro-3-methoxyphenyl)-1-(2-hydroxy-4-methoxybenzyl)-2-oxo-7-(trifluoromethyl)-1,2,3,5-tetrahydro-4,1-benzoxazepin-3-yl]acetate (5.45 g).
MS (EI) m/z: 457 M⁺.
¹H-NMR (CDCl3) δ: 1.22 (3H, t, J = 7.1 Hz), 2.83 (1H, dd, J = 16.5, 6.5 Hz), 3.06 (1H, dd, J = 16.5, 6.5 Hz), 3.94 (3H, s), 4.12 (2H, q, J = 7.1 Hz), 4.63 (1H, t, J = 6.5 Hz), 6.29 (1H, s), 6.93 (1H, s), 7.01 (1H, d, J = 8.3 Hz), 7.12 (1H, d, J = 8.3 Hz), 7.18 (1H, d, J = 8.3 Hz), 7.34 (1H, t, J = 8.3 Hz), 7.60 (1H, d, J = 8.3 Hz), 8.23 (1H, s).

### Referential Example 465

Ethyl 2-[trans-5-(2-chloro-3-methoxyphenyl)-2-thioxo-7-(trifluoromethyl)-1,2,3,5-tetrahydro-4,1-benzoxazepin-3-yl] acetate

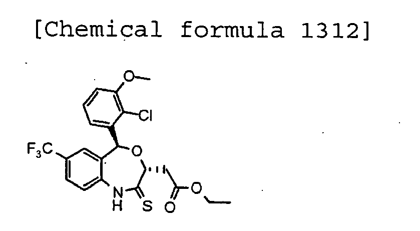

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (2.53 g) was obtained from ethyl 2-[trans-5-(2-chloro-3-methoxyphenyl)-2-oxo-7-(trifluoromethyl)-1,2,3,5-tetrahydro-4,1-benzoxazepin-3-yl]acetate (2.66 g).
MS (EI) m/z: 473 M⁺.
¹H-NMR (CDCl3) δ: 1.26 (3H, t, J = 7.1 Hz), 2.99 (1H, dd, J = 16.5, 6.5 Hz), 3.29 (1H, dd, J = 16.5, 6.5 Hz), 3.93 (3H, s), 4.12 (2H, q, J = 7.1 Hz), 4.71 (1H, t, J = 6.5 Hz), 6.20 (1H, s), 6.87 (1H, s), 7.01 (1H, dd, J = 8.3, 1.3 Hz), 7.24 (1H, s), 7.38 (1H, t, J = 8.3 Hz), 7.66 (1H, d, J = 8.3 Hz), 9.67 (1H, br s).

### Example 649

Ethyl 2-[trans-6-(2-chloro-3-methoxyphenyl)-1,8-bis(trifluoromethyl)-4H,6H-[1,2,4]triazolo[4,3-a][4,1]benzoxazepin-4-yl]acetate

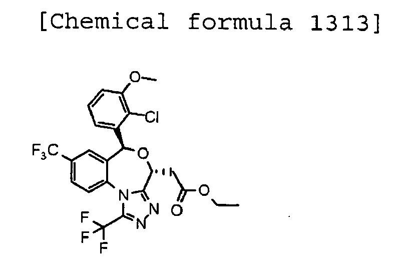

In a similar manner to that employed for the synthesis of methyl 2-[trans-8-chloro-6-(2,3-dimethoxyphenyl)-10-fluoro-1-(trifluoromethyl)-4H,6H-[1,2,4]triazolo[4,3-a][4,1]benzothiazepin-4-yl]acetate, the title compound (2.42 g) was obtained from ethyl 2-[trans-5-(2-chloro-3-methoxyphenyl)-2-thioxo-7-(trifluoromethyl)-1,2,3,5-tetrahydro-4,1-benzoxazepin-3-yl]acetate (2.53 g).
MS (FAB) m/z: 550 (M+H)⁺.
¹H-NMR (CDCl3) δ: 1.29 (3H, t, J = 7.1 Hz), 3.27 (1H, dd, J = 16.7, 6.8 Hz), 3.53 (1H, dd, J = 16.7, 6.8 Hz), 3.91 (3H, s), 4.19 (2H, dq, J = 7.1, 1.4 Hz), 4.95 (1H, t, J = 6.8 Hz), 5.63 (1H, s), 7.01-7.05 (2H, m), 7.37 (1H, dd, J = 8.0, 1.5 Hz), 7.42 (1H, t, J = 8.0 Hz), 7.74 (1H, d, J = 8.3 Hz), 7.86 (1H, dd, J = 8.3, 1.5 Hz).

### Example 650

Ethyl 2-[(4R,6S)-6-(2-chloro-3-methoxyphenyl)-1,8-bis(trifluoromethyl)-4H,6H-[1,2,4]triazolo[4,3-a][4,1]benzoxazepin-4-yl]acetate (isomer A)

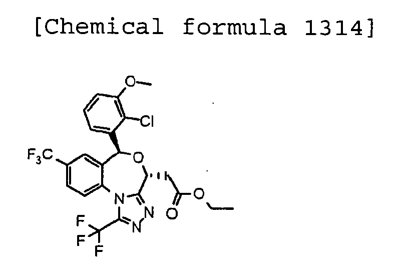

Ethyl 2-[(4S,6R)-6-(2-chloro-3-methoxyphenyl)-1,8-bis(trifluoromethyl)-4H,6H-[1,2,4]triazolo[4,3-a][4,1]benzoxazepin-4-yl]acetate (isomer B)

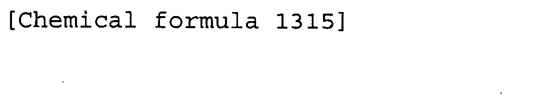

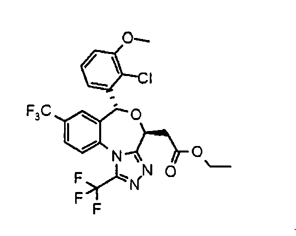

Ethyl 2-[trans-6-(2-chloro-3-methoxyphenyl)-1,8-bis(trifluoromethyl)-4H,6H-[1,2,4]triazolo[4,3-a][4,1]benzoxazepin-4-yl]acetate (1.50 g) was optically resolved by a CHIRALPAK AD column (Daicel Chemical Industries, hexane:isopropanol=85:15, flow rate: 50 mL/min) to give ethyl 2-[(4R,6S)-6-(2-chloro-3-methoxyphenyl)-1,8-bis(trifluoromethyl)-4H,6H-[1,2,4]triazolo[4,3-a] [4,1]benzoxazepin-4-yl]acetate (isomer A, retention time: 29 min., 720 mg) and ethyl 2-[(4S,6R)-6-(2-chloro-3-methoxyphenyl)-1,8-bis(trlfluoromethyl)-4H,6H-[1,2,4]triazolo[4,3-a][4,1]benzoxazepin-4-yl]acetate (isomer B, retention time: 45 min., 750 mg). Ethyl 2-[(4R,6S)-6-(2-chloro-3-methoxyphenyl)-1,8-bis(trifluoromethyl)-4H,6H-[1,2,4]triazolo[4,3-a][4,1]benzoxazepin-4-yl]acetate (isomer A)
MS (FAB) m/z: 550 (M+H)⁺.
¹H-NMR (CDCl3) δ: 1.29 (3H, t, J = 7.1 Hz), 3.27 (1H, dd, J = 16.7, 6.8 Hz), 3.53 (1H, dd, J = 16.7, 6.8 Hz), 3.91 (3H, s), 4.19 (2H, dq, J = 7.1, 1.4 Hz), 4.95 (1H, t, J = 6.8 Hz), 5.63 (1H, s), 7.01-7.05 (2H, m), 7.37 (1H, dd, J = 8.0, 1.5 Hz), 7.42 (1H, t, J = 8.0 Hz), 7.74 (1H, d, J = 8.3 Hz), 7.86 (1H, dd, J = 8.3, 1.5 Hz). Ethyl 2-[(4S,6R)-6-(2-chloro-3-methoxyphenyl)-1,8-bis(trifluoromethyl)-4H,6H-[1,2,4]triazolo[4,3-a] [4,1]benzoxazepin-4-yl]acetate (isomer B)
MS (FAB) m/z: 550 (M+H)⁺.
¹H-NMR (CDCl3) δ: 1.29 (3H, t, J = 7.1 Hz), 3.27 (1H, dd, J = 16.7, 6.8 Hz), 3.53 (1H, dd, J = 16.7, 6.8 Hz), 3.91 (3H, s), 4.19 (2H, dq, J = 7.1, 1.4 Hz), 4.95 (1H, t, J = 6.8 Hz), 5.63 (1H, s), 7.01-7.05 (2H, m), 7.37 (1H, dd, J = 8.0, 1.5 Hz), 7.42 (1H, t, J = 8.0 Hz), 7.74 (1H, d, J = 8.3 Hz), 7.86 (1H, dd, J = 8.3, 1.5 Hz).

### Example 651

2-[(4R,6S)-6-(2-Chloro-3-methoxyphenyl)-1,8-bis(trifluoromethyl)-4H,6H-[1,2,4]triazolo[4,3-a][4,1]benzoxazepin-4-yl]acetic acid

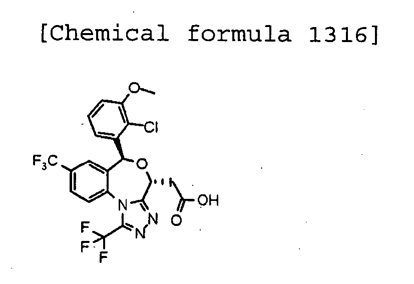

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (317 mg) was obtained from ethyl 2-[(4R,6S)-6-(2-chloro-3-methoxyphenyl)-1,8-bis(trifluoromethyl)-4H,6H-[1,2,4]triazolo[4,3-a][4,1]benzoxazepin-4-yl]acetate (isomer A, 720 mg).
MS (EI) m/z: 521 M⁺.
¹H-NMR (CDCl3) δ: 3.34 (1H, dd, J = 17.1, 6.8 Hz), 3.61 (1H, dd, J = 17.1, 6.8 Hz), 3.91 (3H, s), 4.92 (1H, t, J = 6.8 Hz), 5.63 (1H, s), 7.00-7.05 (2H, m), 7.38 (1H, t, J = 8.5 Hz), 7.42 (1H, t, J = 8.5 Hz), 7.75 (1H, d, J = 8.5 Hz), 7.87 (1H, d, J = 8.5 Hz).
Elemental analysis for: C₂₁H₁₄ClF₆N₃O₄
Calculated: C, 48.34; H, 2.70; N, 8.05
Found: C, 47.96; H, 2.74; N, 8.08.

### Example 652

2-[(4S,6R)-6-(2-Chloro-3-methoxyphenyl)-1,8-bis(trifluoromethyl)-4H,6H-[1,2,4]triazolo[4,3-a][4,1]benzoxazepin-4-yl]acetic acid

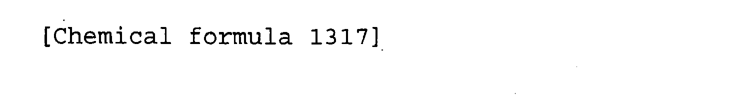

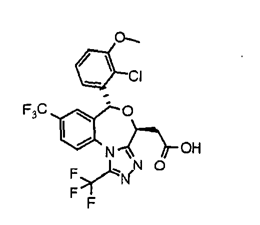

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (305 mg) was obtained from ethyl 2-[(4S,6R)-6-(2-chloro-3-methoxyphenyl)-1,8-bis(trifluoromethyl)-4H,6H-[1,2,4]triazolo[4,3-a][4,1]benzoxazepin-4-yl]acetate (isomer B, 789 mg).
MS (EI) m/z: 521 M⁺.
¹H-NMR (CDCl3) δ: 3.34 (1H, dd, J = 17.1, 6.8 Hz), 3.61 (1H, dd, J = 17.1, 6.8 Hz), 3.91 (3H, s), 4.92 (1H, t, J = 6.8 Hz), 5.63 (1H, s), 7.00-7.05 (2H, m), 7.38 (1H, t, J = 8.5 Hz), 7.42 (1H, t, J = 8.5 Hz), 7.75 (1H, d, J = 8.5 Hz), 7.87 (1H, d, J = 8.5 Hz).
Elemental analysis for: C₂₁H₁₄ClF₆N₃O₄·0.25H₂O
Calculated: C, 47.92; H, 2.78; N, 7.98
Found: C, 47.81; H, 2.73; N, 7.87.

### Example 653

Ethyl 2-[trans-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate

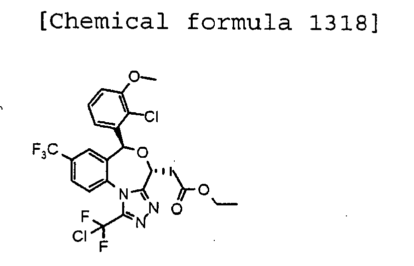

In a similar manner to that employed for the synthesis of ethyl 2-[trans-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate, the title compound (997 mg) was obtained from ethyl 2-[trans-5-(2-chloro-3-methoxyphenyl)-2-thioxo-7-(trifluoromethyl)-1,2,3,5-tetrahydro-4,1-benzoxazepin-3-yl]acetate (1.00 g).
MS (FAB) m/z: 566 (M+H)⁺.
¹H-NMR (CDCl3) δ: 1.29 (3H, t, J = 7.2 Hz), 3.27 (1H, dd, J = 16.7, 6.8 Hz), 3.52 (1H, dd, J = 16.7, 6.8 Hz), 3.91 (3H, s), 4.20 (2H, q, J = 7.2 Hz), 4.92 (1H, t, J = 6.8 Hz), 5.63 (1H, s), 7.00-7.04 (2H, m), 7.37 (1H, d, J = 8.0 Hz), 7.41 (1H, t, J = 8.0 Hz), 7.80 (1H, d, J = 8.0 Hz), 7.85 (1H, d, J = 8.0 Hz).

### Example 654

Ethyl 2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate
(isomer A)

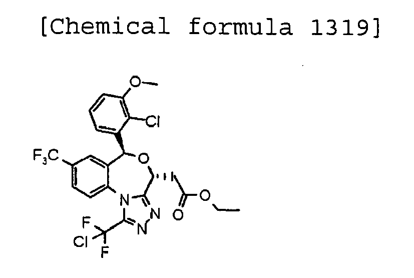

Ethyl 2-[(4S,6R)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate
(isomer B)

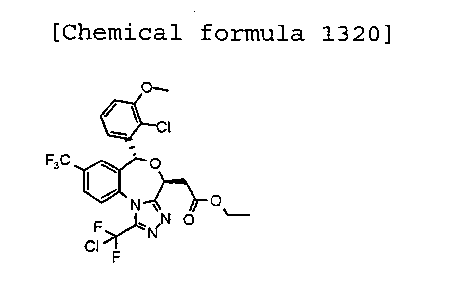

Ethyl 2-[trans-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate (995 mg) was optically resolved by a CHIRALPAK AD column (Daicel Chemical Industries, hexane:isopropanol=85:15, flow rate: 50 mL/min) to give ethyl 2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate (isomer A, retention time: 28 min., 497 mg) and ethyl 2-[(4S,6R)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate (isomer B, retention time: 52 min., 499 mg). Ethyl 2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate
(isomer A)
MS (FAB) m/z: 566 (M+H)⁺.
¹H-NMR (CDCl3) δ: 1.29 (3H, t, J = 7.2 Hz), 3.27 (1H, dd, J = 16.7, 6.8 Hz), 3.52 (1H, dd, J = 16.7, 6.8 Hz), 3.91 (3H, s), 4.20 (2H, q, J = 7.2 Hz), 4.92 (1H, t, J = 6.8 Hz), 5.63 (1H, s), 7.00-7.04 (2H, m), 7.37 (1H, d, J = 8.0 Hz), 7.41 (1H, t, J = 8.0 Hz), 7.80 (1H, d, J = 8.0 Hz), 7.85 (1H, d, J = 8.0 Hz).
Ethyl 2-[(4S,6R)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate
(isomer B)
MS (FAB) m/z: 566 (M+H)⁺.
¹H-NMR (CDCl3) δ: 1.29 (3H, t, J = 7.2 Hz), 3.27 (1H, dd, J = 16.7, 6.8 Hz), 3.52 (1H, dd, J = 16.7, 6.8 Hz), 3.91 (3H, s), 4.20 (2H, q, J = 7.2 Hz), 4.92 (1H, t, J = 6.8 Hz), 5.63 (1H, s), 7.00-7.04 (2H, m), 7.37 (1H, d, J = 8.0 Hz), 7.41 (1H, t, J = 8.0 Hz), 7.80 (1H, d, J = 8.0 Hz), 7.85 (1H, d, J = 8.0 Hz).

### Example 655

2-[(4R,6S)-1-[Chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetic acid

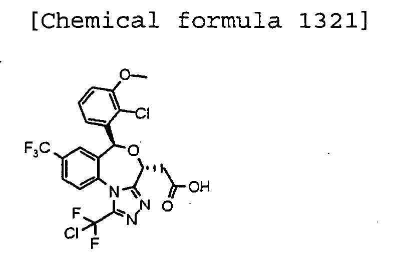

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (437 mg) was obtained from ethyl 2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate (isomer A, 497 mg).
MS (FAB) m/z: 538 (M+H)⁺.
¹H-NMR (CDCl3) δ: 3.33 (1H, dd, J = 17.1, 6.7 Hz), 3.60 (1H, dd, J = 17.1, 6.7 Hz), 3.91 (3H, s), 4.88 (1H, t, J = 6.7 Hz), 5.63 (1H, s), 7.00-7.05 (2H, m), 7.37 (3H, d, J = 8.3 Hz), 7.42 (3H, t, J = 8.3 Hz), 7.80 (3H, d, J = 8.3 Hz), 7.85 (3H, d, J = 8.3 Hz).
Elemental analysis for: C₂₁H₁₄Cl₂F₅N₃O₄
Calculated: C, 46.86; H, 2.62; N, 7.81
Found: C, 46.74; H, 2.48; N, 7.61.

### Example 656

2-[(4S,6R)-1-[Chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetic acid

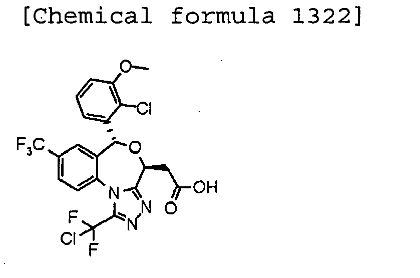

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (432 mg) was obtained from ethyl 2-[(4S,6R)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetate (isomer B, 499 mg).
MS (FAB) m/z: 538 (M+H)⁺.
¹H-NMR (CDCl3) δ: 3.33 (1H, dd, J = 17.1, 6.7 Hz), 3.60 (1H, dd, J = 17.1, 6.7 Hz), 3.91 (3H, s), 4.88 (1H, t, J = 6.7 Hz), 5.63 (1H, s), 7.00-7.05 (2H, m), 7.37 (3H, d, J = 8.3 Hz), 7.42 (3H, t, J = 8.3 Hz), 7.80 (3H, d, J = 8.3 Hz), 7.85 (3H, d, J = 8.3 Hz).
Elemental analysis for: C₂₁H₁₄Cl₂F₅N₃O₄
Calculated: C, 46.86; H, 2.62; N, 7.81
Found: C, 46.88; H, 2.57; N, 7.60.

### Example 657

Ethyl 2-[trans-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate

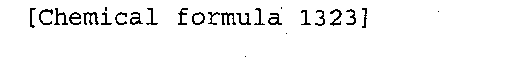

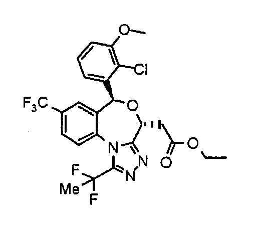

In a similar manner to that employed for the synthesis of ethyl 2-[trans-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate, the title compound (690 mg) was obtained from ethyl 2-[trans-5-(2-chloro-3-methoxyphenyl)-2-thioxo-7-(trifluoromethyl)-1,2,3,5-tetrahydro-4,1-benzoxazepin-3-yl]acetate (1.40 g).
MS (FAB) m/z: 546 (M+H)⁺.
¹H-NMR (CDCl3) δ: 1.29 (3H, t, J = 7.1 Hz), 2.34 (3H, t, J = 19.3 Hz), 3.25 (1H, dd, J = 16.7, 6.7 Hz), 3.49 (1H, dd, J = 16.7, 6.7 Hz), 3.91 (3H, s), 4.20 (2H, q, J = 7.1 Hz), 4.91 (1H, t, J = 6.7 Hz), 5.63 (1H, s), 6.97 (1H, s), 6.99-7.03 (1H, m), 7.36-7.43 (2H, m), 7.81 (1H, d, J = 8.5 Hz), 7.93 (1H, d, J = 8.5 Hz).

### Example 658

Ethyl 2-[(4R,6S)-6-(2-chloro-3-methoxyphenyl)-1-(1,1-difluoroethyl)-8-(trifluoromethyl)-4H,6H-[1,2,4]triazolo[4,3-a] [4,1]benzoxazepin-4-yl]acetate
(isomer A)

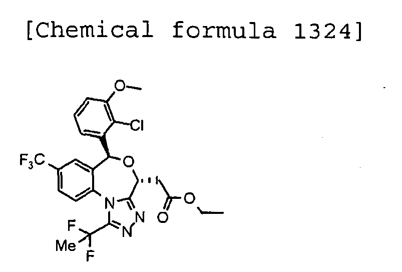

Ethyl 2-[(4S,6R)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate
(isomer B)

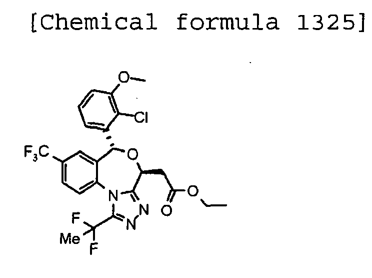

Ethyl 2-[trans-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate (690 mg) was optically resolved by a CHIRALPAK AD column (Daicel Chemical Industries, hexane:isopropanol=92.5:7.5, flow rate: 50 mL/min) to give ethyl 2-[(4R,6S)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate (isomer A, retention time: 72 min., 311 mg) and ethyl 2-[(4S,6R)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate (isomer B, retention time: 100 min., 303 mg). ethyl 2-[(4R,6S)-6-(2-chloro-3-methoxyphenyl)-1-(1,1-difluoroethyl)-8-(trifluoromethyl)-4H,6H-[1,2,4]triazolo[4,3-a][4,1] benzoxazepin-4-yl]acetate
(isomer A)
MS (FAB) m/z: 546 (M+H)⁺.
¹H-NMR (CDCl3) δ: 1.29 (3H, t, J = 7.1 Hz), 2.34 (3H, t, J = 19.3 Hz), 3.25 (1H, dd, J = 16.7, 6.7 Hz), 3.49 (1H, dd, J = 16.7, 6.7 Hz), 3.91 (3H, s), 4.20 (2H, q, J = 7.1 Hz), 4.91 (1H, t, J = 6.7 Hz), 5.63 (1H, s), 6.97 (1H, s), 6.99-7.03 (1H, m), 7.36-7.43 (2H, m), 7.81 (1H, d, J = 8.5 Hz), 7.93 (1H, d, J = 8.5 Hz).
Ethyl 2-[(4S,6R)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate
(isomer B)
MS (FAB) m/z: 546 (M+H)⁺.
¹H-NMR (CDCl3) δ: 1.29 (3H, t, J = 7.1 Hz), 2.34 (3H, t, J = 19.3 Hz), 3.25 (1H, dd, J = 16.7, 6.7 Hz), 3.49 (1H, dd, J = 16.7, 6.7 Hz), 3.91 (3H, s), 4.20 (2H, q, J = 7.1 Hz), 4.91 (1H, t, J = 6.7 Hz), 5.63 (1H, s), 6.97 (1H, s), 6.99-7.03 (1H, m), 7.36-7.43 (2H, m), 7.81 (1H, d, J = 8.5 Hz), 7.93 (1H, d, J = 8.5 Hz).

### Example 659

2-[(4R,6S)-6-(2-Chloro-3-methoxyphenyl)-1-(1,1-difluoroethyl)-8-(trifluoromethyl)-4H,6H-[1,2,4]triazolo[4,3-a] [4,1]benzoxazepin-4-yl]acetic acid

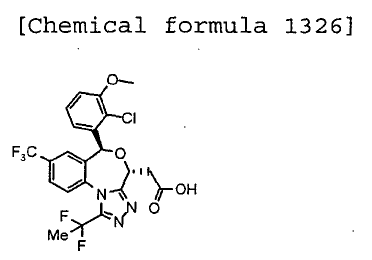

In a similar manner to that employed for the synthesis of 3-(2-{[trans-8-chloro-6-(2,3-dimethoxyphenyl)-1-(2,2;2-trifluoroethyl)-4H,6H-[1,2,4]triazolo[4,3-a][4,1]benzothiazepin-4-yl]methyl}-1,3-thiazol-5-yl)propanoic acid, the title compound (261 mg) was obtained from ethyl 2-[(4R,6S)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate (isomer A, 311 mg).
MS (EI) m/z: 517 M⁺.
¹H-NMR (CDCl3) δ: 2.33 (3H, t, J = 19.3 Hz), 3.32 (1H, dd, J = 17.1, 6.6 Hz), 3.58 (1H, dd, J = 17.1, 6.8 Hz), 3.91 (3H, s), 4.88 (1H, t, J = 6.7 Hz), 5.63 (1H, s), 6.99 (1H, s), 7.01 (1H, dd, J = 8.3, 2.0 Hz), 7.36-7.43 (2H, m), 7.81 (1H, d, J = 8.3 Hz), 7.93 (1H, d, J = 8.3 Hz).
Elemental analysis for: C₂₂H₁₇ClF₅N₃O₄
Calculated: C, 51.03; H, 3.31; N, 8.11
Found: C, 50.83; H, 3.30; N, 7.82.

### Example 660

2-[(4S,6R)-6-(2-Chloro-3-methoxyphenyl)-1-(1,1-difluoroethyl)-8-(trifluoromethyl)-4H,6H-[1,2,4]triazolo[4,3-a][4,1]benzoxazepin-4-yl]acetic acid

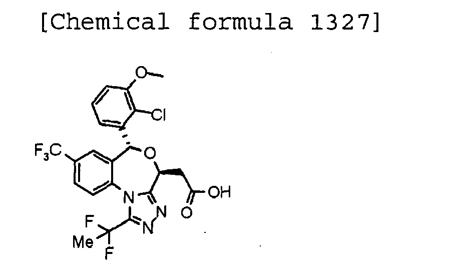

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a] [4,1]benzothiazepin-4-yl]methyl}-1,3-thiazol-5-yl)propanoic acid, the title compound (252 mg) was obtained from ethyl 2-[(4S,6R)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetate (isomer B, 303 mg).
MS (EI) m/z: 517 M⁺.
¹H-NMR (CDCl3) δ: 2.33 (3H, t, J = 19.3 Hz), 3.32 (1H, dd, J = 17.1, 6.6 Hz), 3.58 (1H, dd, J = 17.1, 6.8 Hz), 3.91 (3H, s), 4.88 (1H, t, J = 6.7 Hz), 5.63 (1H, s), 6.99 (1H, s), 7.01 (1H, dd, J = 8.3, 2.0 Hz), 7.36-7.43 (2H, m), 7.81 (1H, d, J = 8.3 Hz), 7.93 (1H, d, J = 8.3 Hz).
Elemental analysis for: C₂₂H₁₇ClF₅N₃O₄
Calculated: C, 51.03; H, 3.31; N, 8.11
Found: C, 50.99; H, 3.28; N, 7.82.

### Referential Example 466

tert-Butyl 2-[(2-fluoro-3-methoxyphenyl)(hydroxy)methyl]-4-(trifluoromethyl)phenylcarbamate

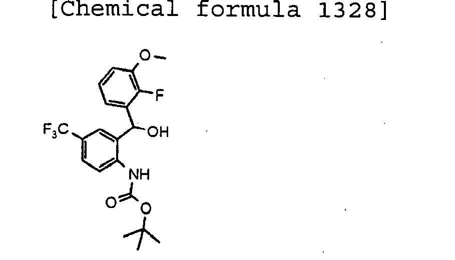

In a similar manner to that employed for the synthesis of tert-butyl 4-chloro-2-{hydroxy[2-(trifluoromethoxy)phenyl]methyl}phenylcarbamate, the title compound (8.10 g) was obtained from tert-butyl 4-(trifluoromethyl)phenylcarbamate (7.06 g) and 2-fluoro-3-methoxybenzaldehyde (5.00 g).
MS (EI) m/z: 415 M⁺.
¹H-NMR (CDCl3) δ: 1.50 (9H, s), 2.85 (1H, d, J = 4.4 Hz), 3.89 (3H, s), 6.21 (1H, d, J = 4.4 Hz), 6.94-7.00 (2H, m), 7.11 (1H, td, J = 8.0, 1.4 Hz), 7.32 (1H, s), 7.53 (1H, dd, J = 8.5, 1.6 Hz), 8.03 (1H, s), 8.08 (1H, d, J = 8.5 Hz).

### Referential Example 467

[2- [(2,4-Dimethoxybenzyl) amino] -5-(trifluoromethyl)phenyl](2-fluoro-3-methoxyphenyl)methanol

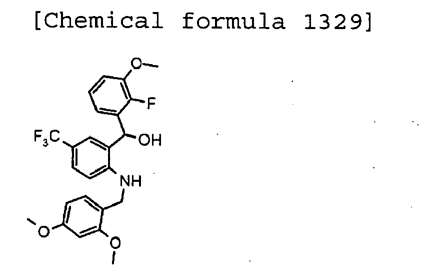

In a similar manner to that employed for the synthesis of 2-amino-5-chloro-3-[(2,3-dimethoxyphenyl)(hydroxy)methyl]pyridine and {5-chloro-2-[(2,4-dimethoxybenzyl)amino]phenyl}(2,3-dimethoxyphenyl)methanol, the title compound (8.30 g) was obtained from tert-butyl 2-[(2-fluoro-3-methoxyphenyl)(hydroxy)methyl]-4-(trifluoromethyl)phenylcarbamate (8.10 g).
MS (FAB) m/z: 466 (M+H)⁺.
¹H-NMR (CDCl3) δ: 2.44 (1H, br s), 3.79 (6H, s), 3.89 (3H, s), 4.29 (2H, br s), 5.46 (1H, s), 6.13 (1H, br s), 6.39 (1H, dd, J = 8.5, 2.2 Hz), 6.46 (1H, d, J = 2.2 Hz), 6.70 (1H, d, J = 8.5 Hz), 6.89-6.97 (2H, m), 7.01-7.10 (2H, m), 7.23-7.27 (1H; m), 7.38 (1H, d, J = 8.5 Hz).

### Referential Example 468

Ethyl 2-[(trans-5-(2-fluoro-3-methoxyphenyl)-1-(2-hydroxy-4-methoxybenzyl)-2-oxo-7-(trifluoromethyl)-1,2,3,5-tetrahydro-4,1-benzoxazepin-3-yl)acetate

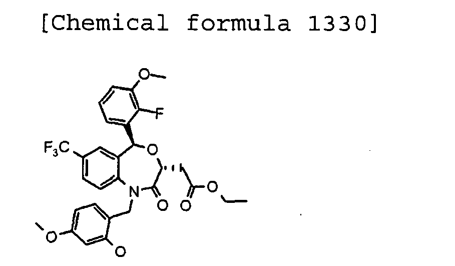

In a similar manner to that employed for the synthesis of ethyl (E)-4-{4-chloro(2,4-dimethoxybenzyl)-2-[(2,3-dimethoxyphenyl)(hydroxy)methyl]anilino}-4-oxo-2-butenoate and ethyl 2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]acetate, the title compound (2.36 g) was obtained from [2-[(2,4-dimethoxybenzyl)amino]-5-(trifluoromethyl)phenyl](2-fluoro-3-methoxyphenyl)methanol (8.30 g).
MS (EI) m/z: 591 M⁺.
¹H-NMR (CDCl3) δ: 1.25 (3H, t, J = 7.1 Hz), 2.77 (1H, dd, J = 16.5, 5.8 Hz), 3.10 (1H, dd, J = 16.5, 7.9 Hz), 3.57 (3H, s), 3.77 (3H, s), 3.88 (3H, s), 4.14 (2H, q, J = 7.1 Hz), 4.46 (1H, dd, J = 7.9, 5.8 Hz), 4.92 (1H, d, J = 14.9 Hz), 5.40 (1H, d, J = 14.9 Hz), 5.94 (1H, s), 6.37-6.47 (2H, m), 6.87 (1H, s), 6.92-7.10 (1H, m), 7.16-7.19 (2H, m), 7.25-7.28 (1H, m), 7.48 (1H, d, J = 8.5 Hz), 7.61 (1H, d, J = 8.5 Hz).

### Referential Example 469

Ethyl 2-[trans-5-(2-fluoro-3-methoxyphenyl)-2-oxo-7-(trifluoromethyl)-1,2,3,5-tetrahydro-4,1-benzoxazepin-3-yl] acetate

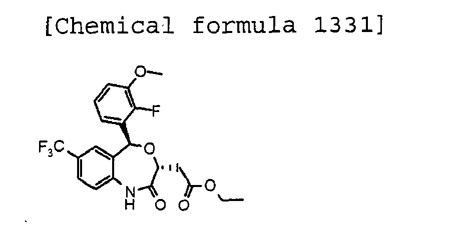

In a similar manner to that employed for the synthesis of ethyl 2-[(trans)-7-chloro-5-(2,3-dimethoxyphenyl)-2-oxo-1,2,3,5-tetrahydro-4,1-benzoxazepin-3-yl]acetate, the title compound (866 mg) was obtained from ethyl 2-[(trans-5-(2-fluoro-3-methoxyphenyl)-1-(2-hydroxy-4-methoxybenzyl)-2-oxo-7-(trifluoromethyl)-1,2,3,5-tetrahydro-4,1-benzoxazepin-3-yl)acetate (2.36 g).
MS (EI) m/z: 441 M⁺.
¹H-NMR (CDCl3) δ: 1.22 (3H, t, J = 7.1 Hz), 2.81 (1H, dd, J = 16.3, 6.5 Hz), 3.04 (1H, dd, J = 16.3, 6.5 Hz), 3.92 (3H, s), 4.07-4.16 (2H, m), 4.63 (1H, t, J = 6.5 Hz), 6.29 (1H, s), 6.91-6.96 (1H, m), 7.03-7.07 (2H, m), 7.13-7.18 (2H, m), 7.59 (1H, d, J = 8.5 Hz), 8.32 (1H, br s).

### Referential Example 470

Ethyl 2-[trans-5-(2-fluoro-3-methoxyphenyl)-2-thioxo-7-(trifluoromethyl)-1,2,3,5-tetrahydro-4,1-benzoxazepin-3-yl]acetate

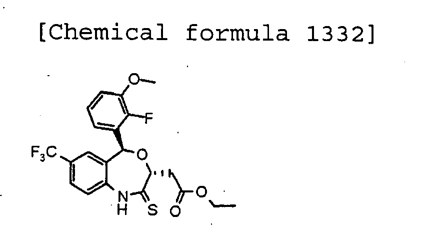

In a similar manner to that employed for the synthesis of ethyl 2-{trans-7-chloro-5-[3-methoxy-2-(trifluoromethoxy)phenyl]-2-thioxo-1,2,3,5-tetrahydro-4,1-benzoxazepin-3-yl}acetate, the title compound (670 mg) was obtained from ethyl 2-[trans-5-(2-fluoro-3-methoxyphenyl)-2-oxo-7-(trifluoromethyl)-1,2,3,5-tetrahydro-4,1-benzoxazepin-3-yl]acetate (866 mg).
MS (FAB) m/z: 458 (M+H)⁺.
¹H-NMR (CDCl3) δ: 1.24 (3H, t, J = 7.1 Hz), 2.96 (1H, dd, J. = 16.3, 6.6 Hz), 3.28 (1H, dd, J = 16.3, 6.6 Hz), 3.91 (3H, s), 4.13 (2H, q, J = 7.1 Hz), 4.72 (1H, t, J = 6.6 Hz), 6.19 (1H, s), 7.00 (1H, s), 7.04 (1H, t, J = 8.3 Hz), 7.11 (1H, d, J = 6.3 Hz), 7.17-7.26 (2H, m), 7.66 (1H, d, J = 8.3 Hz), 9.73 (1H, br s).

### Example 661

Ethyl 2-[trans-6-(2-fluoro-3-methoxyphenyl)-1,8-bis(trifluoromethyl)-4H,6H-[1,2,4]triazolo[4,3-a][4,1]benzoxazepin-4-yl]acetate

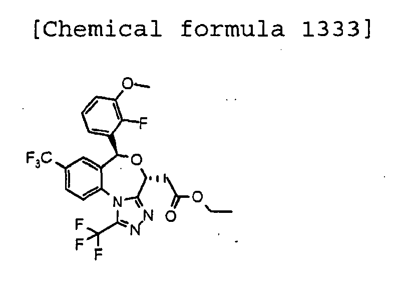

In a similar manner to that employed for the synthesis of methyl 2-[trans-8-chloro-6-(2,3-dimethoxyphenyl)-10-fluoro-1-(trifluoromethyl)-4H,6H-[1,2,4]triazolo[4,3-a][4,1]benzothiazepin-4-yl]acetate, the title compound (506 mg) was obtained from ethyl 2-[trans-5-(2-fluoro-3-methoxyphenyl)-2-thioxo-7-(trifluoromethyl)-1,2,3,5-tetrahydro-4,1-benzoxazepin-3-yl]acetate (668 mg).
MS (EI) m/z: 533 M⁺.
¹H-NMR (CDCl3) δ: 1.28 (3H, t, J = 7.1 Hz), 3.24 (1H, dd, J = 16.8, 6.7 Hz), 3.52 (1H, dd, J = 16.8, 6.7 Hz), 3.88 (3H, s), 4.19 (2H, q, J = 7.1 Hz), 4.95 (1H, t, J = 6.7 Hz), 5.66 (1H, s), 7.02-7.08 (1H, m), 7.17 (1H, s), 7.22-7.25 (2H, m), 7.74 (1H, d, J = 8.0 Hz), 7.86 (1H, d, J = 8.0 Hz).

### Example 662

Ethyl 2-[(4R,6S)-6-(2-fluoro-3-methoxyphenyl)-1,8-bis(trifluoromethyl)-4H,6H-[1,2,4]triazolo[4,3-a][4,1]benzoxazepin-4-yl]acetate (isomer A)

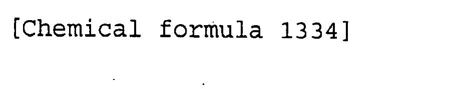

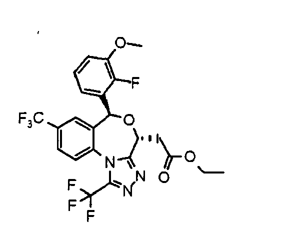

Ethyl 2-[(4S,6R)-6-(2-fluoro-3-methoxyphenyl)-1,8-bis(trifluoromethyl)-4H,6H-[1,2,4]triazolo[4,3-a] [4,1]benzoxazepin-4-yl]acetate (isomer B)

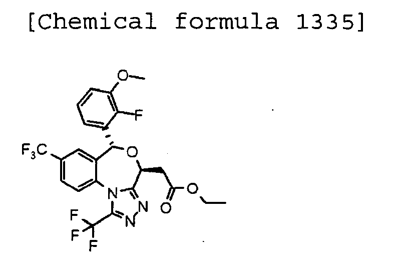

Ethyl 2-[trans-6-(2-fluoro-3-methoxyphenyl)-1,8-bis(trifluoromethyl)-4H,6H-[1,2,4]triazolo[4,3-a] [4,1]benzoxazepin-4-yl]acetate (500 mg) was optically resolved by a CHIRALPAK AD column (Daicel Chemical Industries, hexane:ethanol=85:15, flow rate: 50 mL/min) to give ethyl 2-[(4R,6S)-6-(2-fluoro-3-methoxyphenyl)-1,8-bis(trifluoromethyl)-4H,6H-[1,2,4]triazolo[4,3-a] [4,1]benzoxazepin-4-yl]acetate (isomer A, retention time: 30 min., 249 mg) and ethyl 2-[(4S,6R)-6-(2-fluoro-3-methoxyphenyl)-1,8-bis(trifluoromethyl)-4H,6H-[1,2,4]triazolo[4,3-a][4,1]benzoxazepin-4-yl]acetate (isomer B, retention time: 42 min., 250 mg).
Ethyl 2-[(4R,6S)-6-(2-fluoro-3-methoxyphenyl)-1,8-bis(trifluoromethyl)-4H,6H-[1,2,4]triazolo[4,3-a][4,1]benzoxazepin-4-yl]acetate (isomer A)
MS (EI) m/z: 533 M⁺.
¹H-NMR (CDCl3) δ: 1.28 (3H, t, J = 7.1 Hz), 3.24 (1H, dd, J = 16.8, 6.7 Hz), 3.52 (1H, dd, J = 16.8, 6.7 Hz), 3.88 (3H, s), 4.19 (2H, q, J = 7.1 Hz), 4.95 (1H, t, J = 6.7 Hz), 5.66 (1H, s), 7.02-7.08 (1H, m), 7.17 (1H, s), 7.22-7.25 (2H, m), 7.74 (1H, d, J = 8.0 Hz), 7.86 (1H, d, J = 8.0 Hz).
Ethyl 2-[(4S,6R)-6-(2-fluoro-3-methoxyphenyl)-1,8-bis(trifluoromethyl)-4H,6H-[1,2,4]triazolo[4,3-a][4,1]benzoxazepin-4-yl]acetate (isomer B)
MS (EI) m/z: 533 M⁺.
¹H-NMR (CDCl3) δ: 1.28 (3H, t, J = 7.1 Hz), 3.24 (1H, dd, J = 16.8, 6.7 Hz), 3.52 (1H, dd, J = 16.8, 6.7 Hz), 3.88 (3H, s), 4.19 (2H, q, J = 7.1 Hz), 4.95 (1H, t, J = 6.7 Hz), 5.66 (1H, s), 7.02-7.08 (1H, m), 7.17 (1H, s), 7.22-7.25 (2H, m), 7.74 (1H, d, J = 8.0 Hz), 7.86 (1H, d, J = 8.0 Hz).

### Example 663

2-[(4R,6S)-6-(2-Fluoro-3-methoxyphenyl)-1,8-bis(trifluoromethyl)-4H,6H-[1,2,4]triazolo[4,3-a] [4,1]benzoxazepin-4-yl]acetic acid

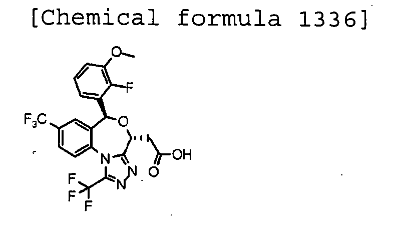

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (216 mg) was obtained from ethyl 2-[(4R,6S)-6-(2-fluoro-3-methoxyphenyl)-1,8-bis(trifluoromethyl)-4H,6H-[1,2,4]triazolo[4,3-a][4,1]benzoxazepin-4-yl]acetate (isomer A, 249 mg) .
MS (FAB) m/z: 506 (M+H)⁺.
¹H-NMR (CDCl3) δ: 3.31 (1H, dd, J = 17.2, 6.7 Hz), 3.59 (1H, dd, J = 17.2, 6.7 Hz), 3.88 (3H, s), 4.92 (1H, t, J = 6.7 Hz), 5.66 (1H, s), 7.02-7.08 (1H, m), 7.17 (1H, s), 7.22-7.25 (2H, m), 7.74 (1H, d, J = 8.3 Hz), 7.87 (1H, d, J = 8.3 Hz).
Elemental analysis for: C₂₁H₁₄F₇N₃O₄
Calculated: C, 49.91; H, 2.79; N, 8.32
Found: C, 49.83; H, 2.72; N, 8.24.

### Example 664

2-[((4S,6R)-6-(2-Fluoro-3-methoxyphenyl)-1,8-bis(trifluoromethyl)-4H,6H-[1,2,4]triazolo[4,3-a][4,1]benzoxazepin-4-yl)acetic acid

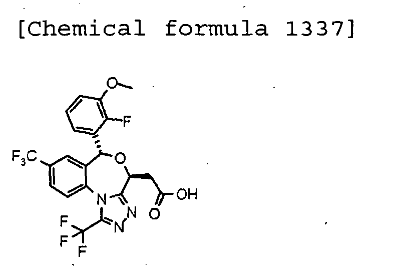

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (228 mg) was obtained from ethyl 2-[(4S,6R)-6-(2-fluoro-3-methoxyphenyl)-1,8-bis(trifluoromethyl)-4H,6H-[1,2,4]triazolo[4,3-a][4,1]benzoxazepin-4-yl]acetate (isomer B, 250 mg).
MS (FAB) m/z: 506 (M+H)⁺.
¹H-NMR (CDCl3) δ: 3.31 (1H, dd, J = 17.2, 6.7 Hz.), 3.59 (1H, dd, J = 17.2, 6.7 Hz), 3.88 (3H, s), 4.92 (1H, t, J = 6.7 Hz), 5.66 (1H, s), 7.02-7.08 (1H, m), 7.17 (1H, s), 7.22-7.25 (2H, m), 7.74 (1H, d, J = 8.3 Hz), 7.87 (1H, d, J = 8.3 Hz).
Elemental analysis for: C₂₁H₁₄F₇N₃O₄
Calculated: C, 49.91; H, 2.79; N, 8.32
Found: C, 49.84; H, 2.68; N, 8.13.

### Example 665

Ethyl 2-[trans-8-chloro-6-(2-chloro-3-methoxyphenyl)-1-(1,1-difluoroethyl)-4H,6H-[1,2,4]triazolo[4,3-a] [4,1]benzoxazepin-4-yl]acetate

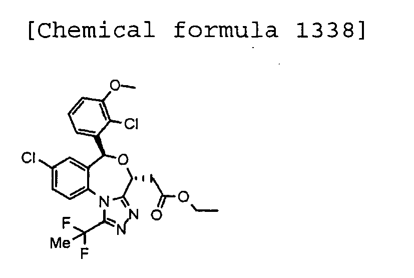

In a similar manner to that employed for the synthesis of ethyl 2-[trans-1-(1,1-difluoroethyl)-6-(3-methoxy-2-methylphenyl)-8-(trifluoromethyl)-4H,6H-[1,2,4]triazolo[4,3-a][4,1]benzoxazepin-4-yl]acetate, the title compound (1.58 g) was obtained from ethyl 2-[trans-7-chloro-5-(2-chloro-3-methoxyphenyl)-2-thioxo-1,2,3,5-tetrahydro-4,1-benzoxazepin-3-yl]acetate (2.80 g).
MS (FAB) m/z: 512 (M+H)⁺.
¹H-NMR (CDCl3) δ: 1.29 (3H, t, J = 7.1 Hz), 1.56 (2H, s), 2.32 (3H, t, J = 19.2 Hz), 3.25 (1H, dd, J = 16.6, 7.3 Hz), 3.47 (1H, dd, J = 16.6, 6.1 Hz), 3.91 (3H, s), 4.17-4.23 (2H, m), 4.92 (1H, dd, J = 7.3, 6.1 Hz), 5.56 (1H, s), 6.68 (1H, d, J = 2.0 Hz), 7.00 (1H, dd, J = 7.8, 2.0 Hz), 7.33-7.42 (2H, m), 7.51 (1H, dd, J = 8.5, 2.4 Hz), 7.72 (1H, d, J = 8.5 Hz).

### Example 666

Ethyl 2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetate (isomer A)

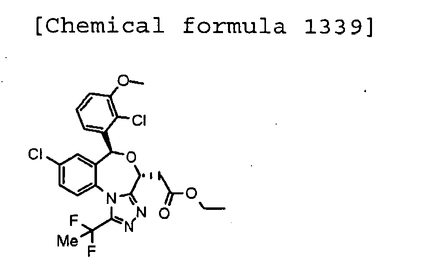

Ethyl 2-[(4S,6R)-8-chloro-6-(2-chloro-3-methoxyphenyl)-1-(1,1-difluoroethyl)-4H,6H-[1,2,4]triazolo[4,3-a][4,1]benzoxazepin-4-yl]acetate (isomer B)

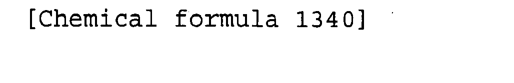

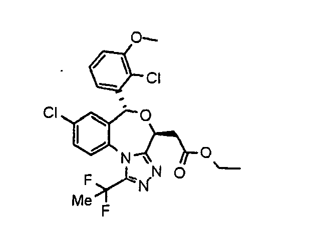

Ethyl 2-[trans-8-chloro-6-(2-chloro-3-methoxyphenyl)-1-(1,1-difluoroethyl)-4H,6H-[1,2,4]triazolo[4,3-a][4,1]benzoxazepin-4-yl]acetate (1.58 g) was optically resolved by a CHIRALPAK AD column (Daicel Chemical Industries, hexane:isopropanol=85:15, flow rate:50 mL/min) to give ethyl 2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetate (isomer A, retention time: 54 min., 769 mg) and ethyl 2-[(4S,6R)-8-chloro-6-(2-chloro-3-methoxyphenyl)-1-(1,1-difluoroethyl)-4H,6H-[1,2,4]triazolo[4,3-a][4,1]benzoxazepin-4-yl]acetate (isomer B, retention time: 80 min., 748 mg).
Ethyl 2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetate (isomer A)
MS (FAB) m/z: 512 (M+H)⁺.
¹H-NMR (CDCl3) δ: 1.29 (3H, t, J = 7.1 Hz), 1.56 (2H, s), 2.32 (3H, t, J = 19.2 Hz), 3.25 (1H, dd, J = 16.6, 7.3 Hz), 3.47 (1H, dd, J = 16.6, 6.1 Hz), 3.91 (3H, s), 4.17-4.23 (2H, m), 4.92 (1H, dd, J = 7.3, 6.1 Hz), 5.56 (1H, s), 6.68 (1H, d, J = 2.0 Hz), 7.00 (1H, dd, J = 7.8, 2.0 Hz), 7.33-7.42 (2H, m), 7.51 (1H, dd, J = 8.5, 2.4 Hz), 7.72 (1H, d, J = 8.5 Hz).
Ethyl 2-[(4S,6R)-8-chloro-6-(2-chloro-3-methoxyphenyl)-1-(1,1-difluoroethyl)-4H,6H-[1,2,4]triazolo[4,3-a] [4,1]benzoxazepin-4-yl]acetate (isomer B)
MS (FAB) m/z: 512 (M+H)⁺.
¹H-NMR (CDCl3) δ: 1.29 (3H, t, J = 7.1 Hz), 1.56 (2H, s), 2.32 (3H, t, J = 19.2 Hz), 3.25 (1H, dd, J = 16.6, 7.3 Hz), 3.47 (1H, dd, J = 16.6, 6.1 Hz), 3.91 (3H, s), 4.17-4.23 (2H, m), 4.92 (1H, dd, J = 7.3, 6.1 Hz), 5.56 (1H, s), 6.68 (1H, d, J = 2.0 Hz), 7.00 (1H, dd, J = 7.8, 2.0 Hz), 7.33-7.42 (2H, m), 7.51 (1H, dd, J = 8.5, 2.4 Hz), 7.72 (1H, d, J = 8.5 Hz).

### Example 667

2-[(4R,6S)-8-Chloro-6-(2-chloro-3-methoxyphenyl)-1-(1,1-difluoroethyl)-4H,6H-[1,2,4]triazolo[4,3-a][4,1]benzoxazepin-4-yl]acetic acid

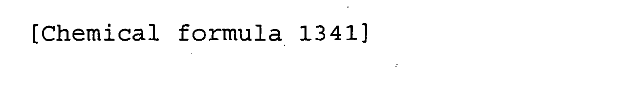

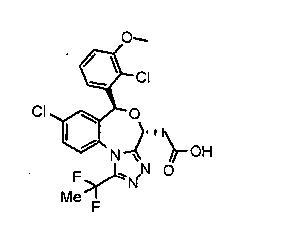

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (583 mg) was obtained from ethyl 2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetate-(isomer A, 769 mg).
MS (FAB) m/z: 484 (M+H)⁺.
¹H-NMR (CDCl3) δ: 2.31 (3H, t, J = 19.2 Hz), 3.31 (1H, dd, J = 17.0, 6.7 Hz), 3.55 (1H, dd, J = 17.0, 6.7 Hz), 3.91 (3H, s), 4.89 (1H, t, J = 6.7 Hz), 5.56 (1H, s), 6.69 (1H, d, J = 2.4 Hz), 7.00 (1H, dd, J = 7.6, 2.2 Hz), 7.34-7.42 (2H, m), 7.51 (1H, dd, J = 8.5, 2.4 Hz), 7.72 (1H, d, J = 8.5 Hz).
Elemental analysis for: C₂₁H₁₇Cl₂F₂N₃O₄
Calculated: C, 52.08; H, 3.54; N, 8.68
Found: C, 52.02; H, 3.52; N, 8.51.

### Example 668

2-[(4S,6R)-8-Chloro-6-(2-chloro-3-methoxyphenyl)-1-(1,1-difluoroethyl)-4H,6H-[1,2,4]triazolo[4,3-a][4,1]benzoxazepin-4-yl]acetic acid

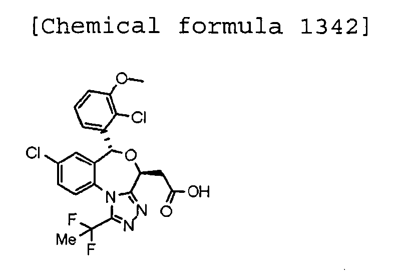

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (621 mg) was obtained from ethyl 2-[(4S,6R)-8-chloro-6-(2-chloro-3-methoxyphenyl)-1-(1,1-difluoroethyl)-4H,6H-[1,2,4]triazolo[4,3-a][4,1]benzoxazepin-4-yl]acetate (isomer B, 748 mg).
MS (FAB) m/z: 484 (M+H)⁺.
¹H-NMR (CDCl3) δ: 2.31 (3H, t, J = 19.2 Hz), 3.31 (1H, dd, J = 17.0, 6.7 Hz), 3.55 (1H, dd, J = 17.0, 6.7 Hz), 3.91 (3H, s), 4.89 (1H, t, J = 6.7 Hz), 5.56 (1H, s), 6.69 (1H, d, J = 2.4 Hz), 7.00 (1H, dd, J = 7.6, 2.2 Hz), 7.34-7.42 (2H, m), 7.51 (1H, dd, J = 8.5, 2.4 Hz), 7.72 (1H, d, J = 8.5 Hz).
Elemental analysis for: C₂₁H₁₇Cl₂F₂N₃O₄
Calculated: C, 52.08; H, 3.54; N, 8.68
Found: C, 52.02; H, 3.48; N, 8.38.

### Example 669

Ethyl 2-[trans-8-chloro-6-(2-chloro-3-methoxyphenyl)-1-(1-fluoro-1-methylethyl)-4H,6H-[1,2,4]triazolo[4,3-a] [4,1]benzoxazepin-4-yl]acetate

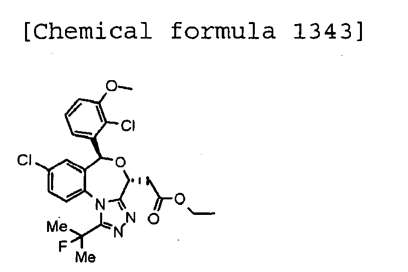

Ethyl 2-[trans-7-chloro-5-(2-chloro-3-methoxyphenyl)-2-thioxo-1,2,3,5-tetrahydro-4,1-benzoxazepin-3-yl]acetate (1.80 g) was dissolved in tetrahydrofuran (40 mL). To the resulting solution was added hydrazine hydrate (299 µl) at room temperature and the resulting mixture was stirred at room temperature for one hour. After concentration under reduced pressure, the residue was dissolved in dichloromethane (40 mL). To the resulting solution were added 2-fluoroisobutyric acid (651 mg), 1-ethyl-3-(3-dimethylaminopropyl)carbodiimide hydrochloride (1.57 g), and 1-hydroxybenzotriazole hydrate (626 mg). The resulting mixture was stirred overnight at room temperature. A saturated aqueous solution of sodium hydrogen carbonate was added to the reaction mixture and the layers separated.
The organic layer was washed with saturated brine and then, dried over anhydrous sodium sulfate. The solvent was distilled off under reduced pressure. The residue was purified by silica gel column chromatography (hexane:ethyl acetate= from 4:1 to 2:1) to give the title compound (780 mg).
MS (FAB) m/z: 508 (M+H)⁺.
¹H-NMR (CDCl3) δ: 1.28 (3H, t, J = 7.1 Hz), 1.53 (3H, d, J = 22.2 Hz), 2.12 (3H, d, J = 22.2 Hz), 3.24 (1H, dd, J = 16.7, 6.7 Hz), 3.45 (1H, dd, J = 16.7, 6.7 Hz), 3.91 (3H, s), 4.15-4.23 (2H, m), 4.87 (1H, t, J = 6.7 Hz), 5.53 (1H, s), 6.68 (1H, d, J = 2.4 Hz), 7.00 (1H, dd, J = 7.8, 2.0 Hz), 7.34-7.42 (2H, m), 7.49 (1H, dd, J = 8.5, 2.4 Hz), 7.78 (1H, d, J = 8.5 Hz).

### Example 670

Ethyl 2-[(4R,6S)-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (isomer A)

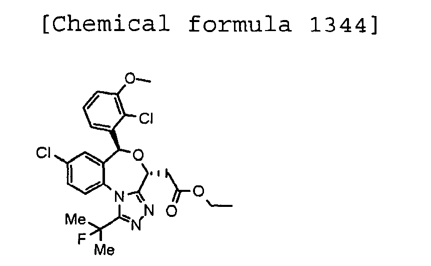

Ethyl 2-[(4S,6R)-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (isomer B)

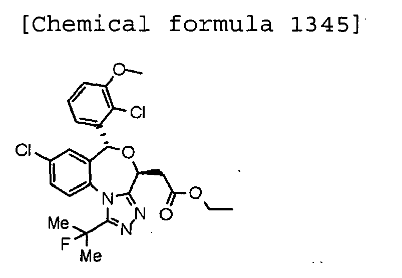

Ethyl 2-[trans-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (960 mg) was optically resolved by a CHIRALPAK AD column (Daicel Chemical Industries, hexane:isopropanol=85:5, flow rate: 50 mL/min) to give ethyl 2-[(4R,6S)-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (isomer A, retention time: 58 min., 376 mg) and ethyl 2-[(4S,6R)-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (isomer B, retention time: 90 min., 366 mg).
Ethyl 2-[(4R,6S)-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (isomer A)
MS (FAB) m/z: 508 (M+H)⁺.
¹H-NMR (CDCl3) δ: 1.28 (3H, t, J = 7.1 Hz), 1.53 (3H, d, J = 22.2 Hz), 2.12 (3H, d, J = 22.2 Hz), 3.24 (1H, dd, J = 16.7, 6.7 Hz), 3.45 (1H, dd, J = 16.7, 6.7 Hz), 3.91 (3H, s), 4.15-4.23 (2H, m), 4.87 (1H, t, J = 6.7 Hz), 5.53 (1H, s), 6.68 (1H, d, J = 2.4 Hz), 7.00 (1H, dd, J = 7.8, 2.0 Hz), 7.34-7.42 (2H, m), 7.49 (1H, dd, J = 8.5, 2.4 Hz), 7.78 (1H, d, J = 8.5 Hz).
Ethyl 2-[(4S,6R)-8-chloro-6-(2-chloro-3-methoxyphenyl)-1-(1-fluoro-1-methylethyl)-4H,6H-[1,2,4]triazolo[4,3-a] [4,1]benzoxazepin-4-yl]acetate (isomer B)
MS (FAB) m/z: 508 (M+H)⁺.
¹H-NMR (CDCl3) δ: 1.28 (3H, t, J = 7.1 Hz), 1.53 (3H, d, J = 22.2 Hz), 2.12 (3H, d, J = 22.2 Hz), 3.24 (1H, dd, J = 16.7,6.7 Hz), 3.45 (1H, dd, J = 16.7,6.7 Hz), 3.91 (3H, s), 4.15-4.23 (2H, m), 4.87 (1H, t, J = 6.7 Hz), 5.53 (1H, s), 6.68 (1H, d, J = 2.4 Hz), 7.00 (1H, dd, J = 7.8, 2.0 Hz), 7.34-7.42 (2H, m), 7.49 (1H, dd, J = 8.5, 2.4 Hz), 7.78 (1H, d, J = 8.5 Hz).

### Example 671

2-[(4R,6S)-8-Chloro-6-(2-chloro-3-methoxyphenyl)-1-(1-fluoro-1-methylethyl)-4H,6H-[1,2,4]triazolo[4,3-a][4,1]benzoxazepin-4-yl]acetic acid

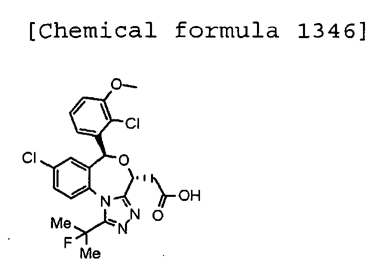

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (310 mg) was obtained from ethyl 2-[(4R,6S)-8-chloro-6-(2-chloro-3-methoxyphenyl)-1-(1-fluoro-1-methylethyl)-4H,6H-[1,2,4]triazolo[4,3-a][4,1]benzoxazepin-4-yl]acetate (isomer A, 376 mg).
MS (EI) m/z: 479 M⁺.
¹H-NMR (CDCl3) δ: 1.53 (3H, d, J = 21.2 Hz), 2.11 (3H, d, J = 21.2 Hz), 3.31 (1H, dd, J = 16.8, 6.7 Hz), 3.53 (1H, dd, J = 16.8, 6.7 Hz), 3.91 (3H, s), 4.83 (1H, t, J = 6.7 Hz), 5.52 (1H, s), 6.69 (1H, d, J = 2.2 Hz), 7.00 (1H, dd, J = 6.8, 2.9 Hz), 7.36-7.43 (2H, m), 7.50 (1H, dd, J = 8.7, 2.3 Hz), 7.77 (1H, dd, J = 8.7, 1.3 Hz).
Elemental analysis for: C₂₂H₂₀Cl₂FN₃O₄
Calculated: C, 55.01; H, 4.20; N, 8.75
Found: C, 55.16; H, 4.24; N, 8.40.

### Example 672

Ethyl 2-[trans-8-chloro-1-[chloro(difluoro)methyl]-6-(2-chloro-3-methoxyphenyl)-4H,6H-[1,2,4]triazolo[4,3-a][4,1]benzoxazepin-4-yl]acetate

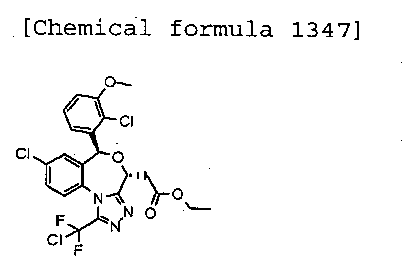

In a similar manner to that employed for the synthesis of ethyl 2-[trans-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetate, the title compound (1.06 g) was obtained from ethyl 2-[trans-7-chloro-5-(2-chloro-3-methoxyphenyl)-2-thioxo-1,2,3,5-tetrahydro-4,1-benzoxazepin-3-yl]acetate (1.00 g).
MS (FAB) m/z: 532 (M+H)⁺.
¹H-NMR (CDCl3) δ: 1.29 (3H, t, J = 7.1 Hz), 3.26 (1H, dd, J = 16.7, 6.8 Hz), 3.50 (1H, dd, J = 16.7, 6.8 Hz), 3.91 (3H, s), 4.20 (2H, q, J = 7.1 Hz), 4.93 (1H, t, J = 6.8 Hz), 5.56 (1H, s), 6.73 (1H, d, J = 2.2 Hz), 7.01 (1H, dd, J = 8.1, 1.7 Hz), 7.34 (1H, dd, J = 8.1, 1.7 Hz), 7.40 (1H, t, J = 8.1 Hz), 7.54 (1H, dd, J = 8.5, 1.7 Hz), 7.60 (1H, d, J = 8.5 Hz).

### Example 673

Ethyl 2-[,(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer A)

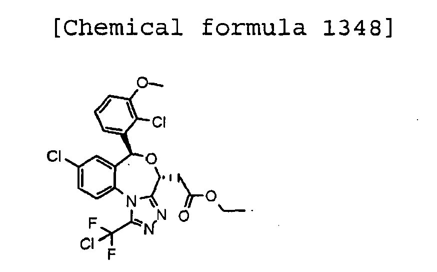

Ethyl 2-[(4S,6R)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer B)

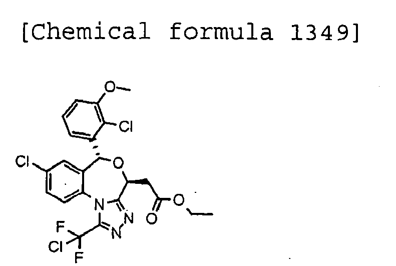

Ethyl 2-[trans-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (1.06 g) was optically resolved by a CHIRALPAK AD column (Daicel Chemical Industries, hexane:isopropanol=92.5:7.5, flow rate: 50 mL/min) to give ethyl 2-[(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer A, retention time: 70 min., 530 mg) and ethyl 2-[(4S,6R)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer B, retention time: 100 min., 530 mg).
Ethyl 2-[(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer A) MS (FAB) m/z: 532 (M+H)⁺.
¹H-NMR (CDCl3) δ: 1.29 (3H, t, J = 7.1 Hz), 3.26 (1H, dd, J = 16.7, 6.8 Hz), 3.50 (1H, dd, J = 16.7, 6.8 Hz), 3.91 (3H, s), 4.20 (2H, q, J = 7.1 Hz), 4.93 (1H, t, J = 6.8 Hz), 5.56 (1H, s), 6.73 (1H, d, J = 2.2 Hz), 7.01 (1H, dd, J = 8.1, 1.7 Hz), 7.34 (1H, dd, J = 8.1, 1.7 Hz), 7.40 (1H, t, J = 8.1 Hz), 7.54 (1H, dd, J = 8.5, 1.7 Hz), 7.60 (1H, d, J = 8.5 Hz).
Ethyl 2-[(4S,6R)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer B) MS (FAB) m/z: 532 (M+H)⁺.
¹H-NMR (CDCl3) δ: 1.29 (3H, t, J = 7.1 Hz), 3.26 (1H, dd, J = 16.7, 6.8 Hz), 3.50 (1H, dd, J = 16.7, 6.8 Hz), 3.91 (3H, s), 4.20 (2H, q, J = 7.1 Hz), 4.93 (1H, t, J = 6.8 Hz), 5.56 (1H, s), 6.73 (1H, d, J = 2.2 Hz), 7.01 (1H, dd, J = 8.1, 1.7 Hz), 7.34 (1H, dd, J = 8.1, 1.7 Hz), 7.40 (1H, t, J = 8.1 Hz), 7.54 (1H, dd, J = 8.5, 1.7 Hz), 7.60 (1H, d, J = 8.5 Hz).

### Example 674

2-[(4R,6S)-8-Chloro-1-[chloro(difluoro)methyl]-6-(2-chloro-3-methoxyphenyl)-4H,6H-[1,2,4]triazolo[4,3-a] [4,1]benzoxazepin-4-yl]acetic acid

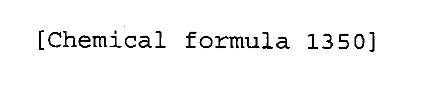

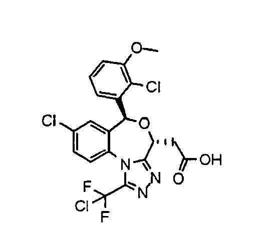

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (484 mg) was obtained from ethyl 2-[(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer A, 530 mg).
MS (FAB) m/z: 504 (M+H)⁺.
¹H-NMR (CDCl3) δ: 3.33 (1H, dd, J = 17.2, 6.6 Hz), 3.58 (1H, dd, J = 17.2, 6.6 Hz), 3.91 (3H, s), 4.89 (1H, t, J = 6.6 Hz), 5.56 (1H, s), 6.74 (1H, d, J = 2.2 Hz), 7.01 (1H, dd, J = 8.1, 1.5 Hz), 7.35 (1H, dd, J = 8.1, 1.5 Hz), 7.40 (1H, t, J = 8.1 Hz), 7.55 (1H, dd, J = 8.8, 2.0 Hz), 7.60 (1H, d, J = 8.8 Hz).
Elemental analysis for: C₂₀H₁₄Cl₃F₂N₃O₄
Calculated: C, 47.60; H, 2.80; N, 8.33
Found: C, 47.39; H, 2.76; N, 8.15.

### Example 675

2-[(4S,6R)-8-Chloro-1-[chloro(difluoro)methyl]-6-(2-chloro-3-methoxyphenyl)-4H,6H-[1,2,4]triazolo[4,3-a] [4,1]benzoxazepin-4-yl]acetic acid

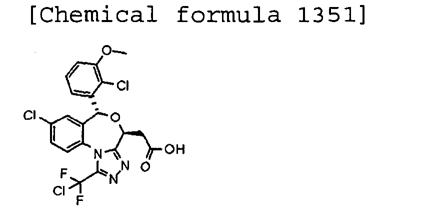

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (466 mg) was obtained from ethyl 2-[(4S,6R)-8-chloro-1-[chloro(difluoro)methyl]-6-(2-chloro-3-methoxyphenyl)-4H,6H-[1,2,4]triazolo[4,3-a][4,1]benzoxazepin-4-yl]acetate (isomer B, 530 mg).
MS (FAB) m/z: 504 (M+H)⁺.
¹H-NMR (CDCl3) δ: 3.33 (1H, dd, J = 17.2, 6.6 Hz), 3.58 (1H, dd, J = 17.2, 6.6 Hz), 3.91 (3H, s), 4.89 (1H, t, J = 6.6 Hz), 5.56 (1H, s), 6.74 (1H, d, J = 2.2 Hz), 7.01 (1H, dd, J = 8.1, 1.5 Hz), 7.35 (1H, dd, J = 8.1, 1.5 Hz), 7.40 (1H, t, J = 8.1 Hz), 7.55 (1H, dd, J = 8.8, 2.0 Hz), 7.60 (1H, d, J = 8.8 Hz).
Elemental analysis for: C₂₀H₁₄Cl₃F₂N₃O₄
Calculated: C, 47.60; H, 2.80; N, 8.33
Found: C, 47.67; H, 2.85; N, 8.23.

### Example 676

tert-Butyl 2-(1-{2-[(4R,6S)-8-chloro-6-(2-chloro-3-methoxyphenyl)-1-(trifluoromethyl)-4H,6H-[1,2,4]triazolo[4,3-a] [4,1]benzoxazepin-4-yl]acetyl}-4-piperidinyl)acetate

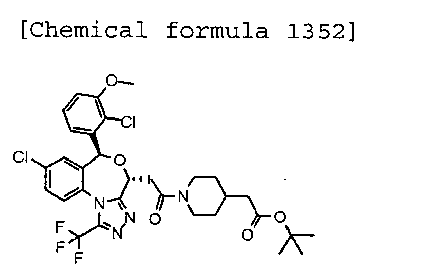

2-[(4R,6S)-8-Chloro-6-(2-chloro-3-methoxyphenyl)-1-(trifluoromethyl)-4H,6H-[1,2,4]triazolo[4,3-a][4,1]benzoxazepin-4-yl]acetic acid (609 mg) was dissolved in dichloromethane (15 mL). To the resulting solution were added tert-butyl 2-(4-piperidinyl)acetate (613 mg), 1-ethyl-3-(3-dimethylaminopropyl)carbodiimide hydrochloride (1.57 g), 1-hydroxybenzotriazole hydrate (235 mg). The resulting mixture was stirred overnight at room temperature. To the reaction mixture was added a saturated aqueous solution of sodium hydrogen carbonate and the layers separated. The organic layer was washed with saturated brine and then, dried over anhydrous sodium sulfate. The solvent was distilled off under reduced pressure. The residue was purified by silica gel column chromatography (hexane:ethyl acetate= from 4:1 to 3:1) to give the title compound (762 mg).
MS (FAB) m/z: 669 (M+H)⁺.
¹H-NMR (CDCl3) δ: 1.08-1.34 (2H, m), 1.45 (9H, s), 1.68-1.85 (2H, m), 1.95-2.06 (1H, m), 2.16 (1H, d, J = 10.0 Hz), 2.16 (1H, d, J = 10.0 Hz), 2.57-2.68 (1H, m), 3.02-3.27 (2H, m), 3.50-3.61 (1H, m), 3.92 (3H, s), 3.97-4.08 (1H, m), 4.50-4.59 (1H, m), 5.09 (1H, t, J = 6.5 Hz), 5.51-5.54 (1H, m), 6.71 (1H, s), 7.01 (1H, d, J = 6.8 Hz), 7.34-7.43 (2H, m), 7.53 (2H, s).

### Example 677

2-(1-{2-[(4R,6S)-8-Chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid

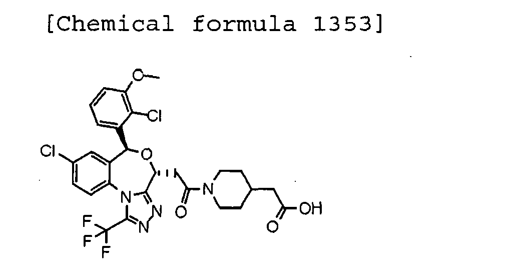

tert-Butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate (762 mg) was dissolved in dichloromethane (30 mL). To the resulting solution was added trifluoroacetic acid (6 mL). The resulting mixture was stirred overnight at room temperature. The reaction mixture was concentrated under reduced pressure, followed by azeotropy with water twice to remove trifluoroacetic acid. To the residue was added ethanol (1 mL) to dissolve the former in the latter. Water (30 mL) was added to the resulting solution and a precipitate thus formed was collected by filtration to give the title compound (611 mg).
MS (FAB) m/z : 613 (M+H)⁺.
¹H-NMR (CDCl3) δ: 1.12-1.52 (2H, m), 1.74-1.91 (2H, m), 2.00-2.12 (1H, m), 2.28 (1H, d, J = 6.3 Hz), 2.34 (1H, d, J = 6.3 Hz), 2.63 (1H, t, J = 11.7 Hz), 3.05-3.30 (2H, m), 3.49-3.64 (1H, m), 3.92 (3H, s), 4.06 (1H, t, J = 13.8 Hz), 4.57 (1H, d, J = 13.8 Hz), 5.09 (1H, t, J = 6.0 Hz), 5.53 (1H, d, J = 5.1 Hz), 6.72 (1H, s), 6.99-7.04 (1H, m), 7.34-7.43 (2H, m), 7.54 (2H, s).
Elemental analysis for: C₂₇H₂₅Cl₂F₃N₄O₅·1.0H₂O
Calculated: C, 51.36; H, 4.31; N, 8.87
Found: C, 51-60; H, 4.09; N, 8.55.

### Example 678

tert-Butyl 2-(1-{2-[(4R,6R)-6-(3-methoxy-2-methylphenyl)-1,8-bis(trifluoromethyl)-4H,6H-[1,2,4]triazolo[4,3-a][4,1]benzoxazepin-4-yl]acetyl}-4-piperidinyl)acetate

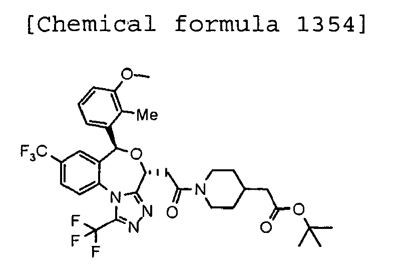

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (105 mg) was obtained from 2-[(4R,6R)-6-(3-methoxy-2-methylphenyl)-1,8-bis(trifluoromethyl)-4H,6H-[l,2,4]triazolo[4,3-a][4,1] benzoxazepin-4-yl] acetic acid (80 mg) .
MS (FAB) m/z: 683 (M+H) +.
¹H-NMR (CDCl3) δ: 1.08-1.40 (2H, m), 1.45 (9H, s), 1.64 (3H, s), 1.69-1.86 (2H, m), 1.95-2.06 (1H, m), 2.13 (1H, d, J = 6.8 Hz), 2.19 (1H, d, J = 6.8 Hz), 2.57-2.67 (1H, m), 3.03-3.28 (2H, m), 3.50-3.63 (1H, m), 3.84 (3H, s), 3.96-4.07 (1H, m), 4.48-4.58 (1H, m), 5.08 (1H, t, J = 6.6 Hz), 5.42 (1H, d, J = 3.0 Hz), 6.92 (1H, d, J = 7.3 Hz), 7.08 (1H, s), 7.27-7.36 (2H, m), 7.73 (1H, d, J = 8.3 Hz), 7.83 (1H, d, J = 8.3 Hz).

### Example 679

2-(1-{2-[(4R,6R)-6-(3-Methoxy-2-methylphenyl)-1,8-bis(trifluoromethyl)-4H,6H-[1,2,4]triazolo[4,3-a][4,1]benzoxazepin-4-yl]acetyl}-4-piperidinyl)acetic acid

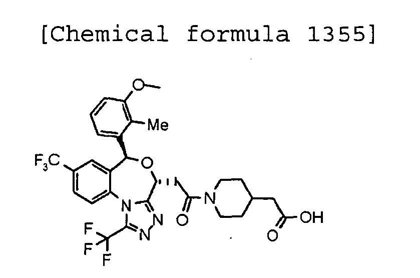

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (77 mg) was obtained from tert-butyl 2-(1-{2-[(4R,6R)-6-(3-methoxy-2-methylphenyl)-1,8-bis(trifluoromethyl)-4H,6H-[1,2,4]triazolo[4,3-a][4,1]benzoxazepin-4-yl]acetyl}-4-piperidinyl)acetate (105 mg).
MS (FAB) m/z: 627 (M+H)⁺.
¹H-NMR (CDCl3) δ: 1.12-1.51 (2H, m), 1.64 (1.5H, s), 1.66 (1.5H, s), 1.74-1.91 (2H, m), 1.99-2.12 (1H, m), 2.27 (1H, d, J = 7.0 Hz), 2.34 (1H, d, J = 7.0 Hz), 2.58-2.68 (1H, m), 3.05-3.31 (2H, m), 3.49-3.67 (1H, m), 3.84 (3H, s), 3.99-4.10 (1H, m), 4.51-4.62 (1H, m), 5.08 (1H, t, J = 6.5 Hz), 5.43 (1H, d, J = 4.2 Hz), 6.92 (1H, d, J = 7.8 Hz), 7.08 (1H, s), 7.27-7.36 (2H, m), 7.73 (1H, d, J = 8.3 Hz), 7.84 (1H, d, J = 8.3 Hz).
Elemental analysis for: C₂₉H₂₈F₆N₄O₅·0.5H₂O
Calculated: C, 54.80; H, 4.60; N, 8.82
Found: C, 54.79; H, 4.58; N, 8.50.

### Example 680

tert-Butyl 2-(1-{2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetyl}-4-piperidinyl)acetate

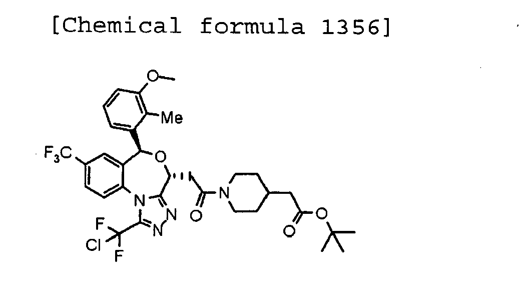

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (103 mg) was obtained from 2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetic acid (83 mg).
MS (FAB) m/z: 699 (M+H)⁺.
¹H-NMR (CDCl3) δ: 1.09-1.41 (2H, m), 1.45 (9H, s), 1.64 (1.5H, s), 1.66 (1.5H, s), 1.69-1.85 (2H, m), 2.01 (1H, br s), 2.13 (1H, d, J = 6.8 Hz), 2.19 (1H, d, J = 6.8 Hz), 2.56-2.68 (1H, m), 3.03-3.28 (2H, m), 3.49-3.64 (1H, m), 3.83 (3H, s), 3.97-4.07 (1H, m), 4.49-4.59 (1H, m), 5.05 (1H, t, J = 6.6 Hz), 5.41 (0.5H, s), 5.43 (0.5H, s), 6.92 (1H, d, J = 6.8 Hz), 7.07 (1H, s), 7.27-7.36 (2H, m), 7.78 (1H, d, J = 8.0 Hz), 7.82 (1H, d, J = 8.0 Hz).

### Example 681

2-(1-{2-[(4R,6R)-1-[Chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetyl}-4-piperidinyl)acetic acid

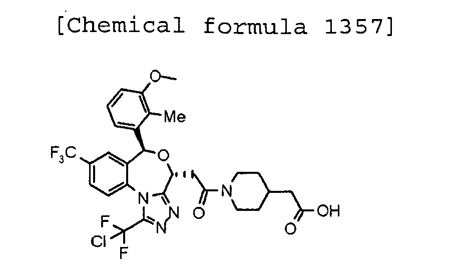

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (81 mg) was obtained from tert-butyl 2-(1-{2-[(4R,6R)-1-[chloro(difluoro)methyl]-6-(3-methoxy-2-methylphenyl)-8-(trifluoromethyl)-4H,6H-[1,2,4]triazolo[4,3-a][4,1]benzoxazepin-4-yl]acetyl}-4-piperidinyl)acetate (100 mg).
MS (FAB) m/z: 643 (M+H)⁺.
¹H-NMR (CDCl3) δ: 1.11-1.51 (2H, m), 1.64 (1.5H, s), 1.66 (1.5H, s), 1.73-1.92 (2H, m), 1.98-2.12 (1H, m), 2.26 (1H, d, J = 6.8 Hz), 2.34 (1H, d, J = 6.8 Hz), 2.58-2.68 (1H, m), 3.04-3.32 (2H, m), 3.47-3.67 (1H, m), 3.83 (3H, s), 4.00-4.10 (1H, m), 4.51-4.61 (1H, m), 5.05 (1H, t, J = 6.7 Hz), 5.42 (0.5H, s), 5.43 (0.5H, s), 6.92 (1H, d, J = 7.6 Hz), 7.07 (1H, s), 7.27-7.35 (2H, m), 7.79 (1H, d, J = 8.5 Hz), 7.83 (1H, d, J = 8.5 Hz).
Elemental analysis for: C₂₉H₂₈ClF₅N₄O₅·0.5H₂O
Calculated: C, 53.06; H, 4.53; N, 8.53
Found: C, 53.24; H, 4.39; N, 8.14.

### Example 682

tert-Butyl 2-(1-{2-[(4R,6S)-8-chloro-6-(2-fluoro-3-methoxyphenyl)-1-(trifluoromethyl)-4H,6H-[1,2,4]triazolo[4,3-a][4,1]benzoxazepin-4-yl]acetyl}-4-piperidinyl)acetate

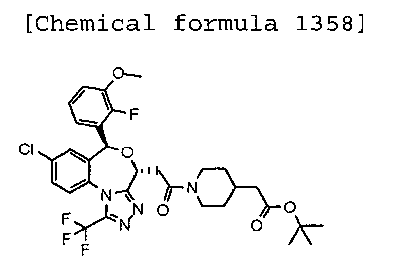

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (647 mg) was obtained from 2-[(4R,6S)-8-chloro-6-(2-fluoro-3-methoxyphenyl)-1-(trifluoromethyl)-4H,6H-[1,2,4]triazolo[4,3-a][4,1]benzoxazepin-4-yl]acetic acid (500 mg) .
MS (FAB) m/z: 653 (M+H)⁺.
¹H-NMR (CDCl3) δ: 1.05-1.27 (2H, m), 1.45 (9H, s), 1.65-1.84 (2H, m), 1.92-2.05 (1H, m), 2.12 (1H, d, J = 6.8 Hz), 2.19 (1H, d, J = 6.8 Hz), 2.55-2.67 (1H, m), 3.00-3.26 (2H, m), 3.46-3.63 (1H, m), 3.89 (3H, s), 3.94-4.07 (1H, m), 4.47-4.57 (1H, m), 5.05-5.12 (1H, m), 5.54 (1H, s), 6.85 (1H, s), 6.98-7.07 (1H, m), 7.19-7.26 (2H, m), 7.53 (2H, s) .

### Example 683

2-(1-{2-[(4R,6S)-8-Chloro-6-(2-fluoro-3-methoxyphenyl)-1-(trifluoromethyl)-4H,6H-[1,2,4]triazolo[4,3-a][4,1]benzoxazepin-4-yl]acetyl}-4-piperidinyl)acetic acid

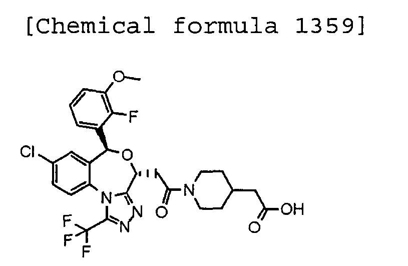

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (505 mg) was obtained from tert-butyl 2-(1-{2-[(4R,6S)-8-chloro-6-(2-fluoro-3-methoxyphenyl)-1-(trifluoromethyl)-4H,6H-[1,2,4]triazolo[4,3-a][4,1]benzoxazepin-4-yl]acetyl}-4-piperidinyl)acetate (645 mg).
MS (FAB) m/z: 597 (M+H)⁺.
¹H-NMR (CDCl3) δ: 1.09-1.27 (1.5H, m), 1.38-1.51 (0.5H, m), 1.73-1.90 (2H, m), 1.98-2.11 (1H, m), 2.25 (1H, d, J = 6.8 Hz), 2.33 (1H, d, J = 6.8 Hz), 2.62 (1H, t, J = 12.8 Hz), 3.04-3.28 (2H, m), 3.51 (0.5H, dd, J = 15.7, 7.4 Hz), 3.60 (0.5H, dd, J = 15.7, 7.4 Hz), 3.89 (3H, s), 4.04 (1H, t, J = 12.8 Hz), 4.51-4.61 (1H, m), 5.06-5.13 (1H, m), 5.54 (0.5H, s), 5.56 (0.5H, s), 6.85 (1H, s), 7.00-7.08 (1H, m), 7.19-7.24 (2H, m), 7.54 (2H, s).
Elemental analysis for: C₂₇H₂₅ClF₄N₄O₅·0.5H₂O
Calculated: C, 53.52; H, 4.32; N, 9.25
Found: C, 53.56; H, 4.20; N, 9.09.

### Example 684

tert-Butyl 2-(1-{2-[(4R,6S)-6-(2-fluoro-3-methoxyphenyl)-1,8-bis(trifluoromethyl)-4H,6H-[1,2,4]triazolo[4,3-a][4,1]benzoxazepin-4-yl]acetyl}-4-piperidinyl)acetate

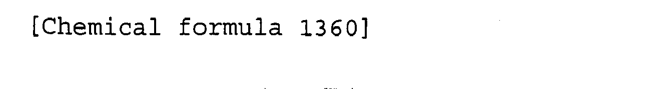

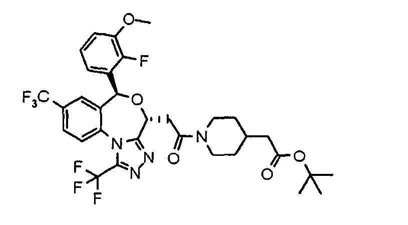

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (197 mg) was obtained from 2-[(4R,6S)-6-(2-fluoro-3-methoxyphenyl)-1,8-bis(trifluoromethyl)-4H,6H-[1,2,4]triazolo[4,3-a] [4, 1] benzoxazepin-4-yl] acetic acid (150 mg) .
MS (FAB) m/z: 687 (M+H)⁺.
¹H-NMR (CDCl3) δ: 1.07-1.35 (2H, m), 1.45 (9H, s), 1.68-1.86 (2H, m), 1.94-2.06 (1H, m), 2.12 (1H, d, J = 6.8 Hz), 2.19 (1H, d, J = 6.8 Hz), 2.56-2.66 (1H, m), 3.03-3.25 (2H, m), 3.50-3.66 (1H, m), 3.88 (3H, s), 3.96-4.07 (1H, m), 4.47-4.57 (1H, m), 5.05-5.11 (1H, m), 5.62 (1H, s), 7.01-7.08 (1H, m), 7.15 (1H, s), 7.21-7.26 (2H, m), 7.73 (1H, d, J = 8.0 Hz), 7.84 (1H, d, J = 8.0 Hz).

### Example 685

2-(1-{2-[(4R,6S)-6-(2-Fluoro-3-methoxyphenyl)-1,8-bis(trifluoromethyl)-4H,6H-[1,2,4]triazolo[4,3-a][4,1]benzoxazepin-4-yl]acetyl}-4-piperidinyl)acetic acid

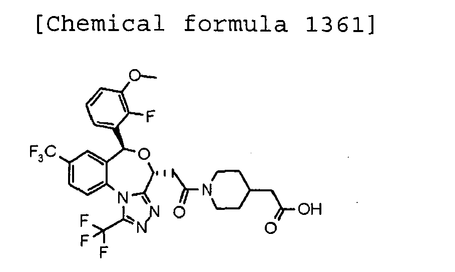

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (156 mg) was obtained from tert-butyl 2-(1-{2-[(4R,6S)-6-(2-fluoro-3-methoxyphenyl)-1,8-bis(trifluoromethyl)-4H,6H-[1,2,4]triazolo[4,3-a][4,1]benzoxazepin-4-yl]acetyl}-4-piperidinyl)acetate (195 mg).
MS (FAB) m/z: 631 (M+H)⁺.
¹H-NMR (CDCl3) δ: 1.09-1.35 (1.5H, m), 1.40-1.53 (0.5H, m), 1.75-1.92 (2H, m), 2.00-2.10 (1H, m), 2.26 (1H, d, J = 6.8 Hz), 2.34 (1H, d, J = 6.8 Hz), 2.62 (1H, t, J = 12.8 Hz), 3.04-3.28 (2H, m), 3.54 (0.5H, dd, J = 15.7, 7.6 Hz), 3.63 (0.5H, dd, J = 15.7, 7.6 Hz), 3.89 (3H, s), 4.04 (1H, t, J = 12.8 Hz), 4.50-4.60 (1H, m), 5.06-5.12 (1H, m), 5.62 (0.5H, s), 5.63 (0.5H, s), 7.03-7.10 (1H, m), 7.15 (1H, s), 7.23-7.26 (2H, m), 7.73 (1H, d, J = 8.3 Hz), 7.85 (1H, d, J = 8.3 Hz) .
Elemental analysis for: C₂₈H₂₅F₇N₄O₅
Calculated: C, 53.34; H, 4.00; N, 8.89
Found: C, 53.33; H, 4.10; N, 8.67.

### Example 686

tert-Butyl 2-(1-{2-[(4R,6S)-6-(2-chloro-3-methoxyphenyl)-1,8-bis(trifluoromethyl)-4H,6H-[1,2,4]triazolo[4,3-a][4,1]benzoxazepin-4-yl]acetyl}-4-piperidinyl)acetate

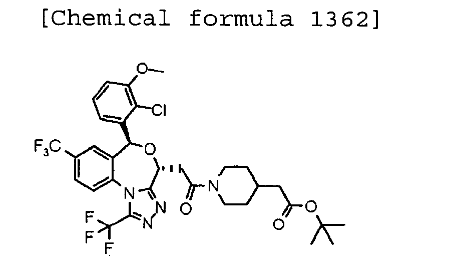

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (171 mg) was obtained from 2-[(4R,6S)-6-(2-chloro-3-methoxyphenyl)-1,8-bis(trifluoromethyl)-4H,6H-[1,2,4]triazolo[4,3-a] [4,1]benzoxazepin-4-yl]acetic acid (100 mg).
MS (FAB) m/z: 703 (M+H)⁺.
¹H-NMR (CDCl3) δ: 1.09-1.41 (2H, m), 1.45 (9H, s), 1.69-1.88 (2H, m), 1.96-2.06 (1H, m), 2.14 (1H, d, J = 6.8 Hz), 2.19 (1H, d, J = 6.8 Hz), 2.63 (1H, t, J = 12.8 Hz), 3.03-3.27 (2H, m), 3.51-3.66 (1H, m), 3.92 (3H, s), 3.98-4.08 (1H, m), 4.54 (1H, d, J = 12.8 Hz), 5.07 (1H, t, J = 6.2 Hz), 5.59 (0.5H, s), 5.61 (0.5H, s), 6.99-7.05 (2H, m), 7.38-7.45 (2H, m), 7.73 (1H, d, J = 8.3 Hz), 7.84 (1H, d, J = 8.3 Hz).

### Example 687

2-(1-{2-[(4R,6S)-6-(2-Chloro-3-methoxyphenyl)-1,8-bis(trifluoromethyl)-4H,6H-[1,2,4]triazolo[4,3-a][4,1]benzoxazepin-4-yl]acetyl}-4-piperidinyl)acetic acid

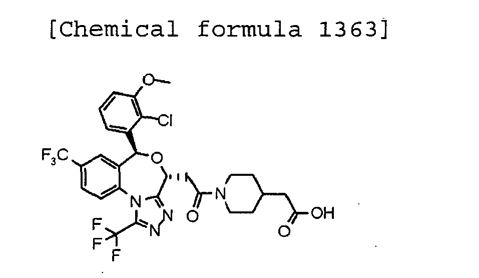

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (105 mg) was obtained from tert-butyl 2-(1-{2-[(4R,65)-6-(2-chloro-3-methoxyphenyl)-1,8-bis(trifluoromethyl)-4H,6H-[1,2,4]triazolo[4,3-a][4,1]benzoxazepin-4-yl]acetyl}-4-piperidinyl)acetate (125 mg).
MS (FAB) m/z: 647 (M+H)⁺.
¹H-NMR (CDCl3) δ: 1.12-1.28 (1.5H, m), 1.40-1.54 (0.5H, m), 1.72-1.92 (2H, m), 1.99-2.12 (1H, m), 2.28 (1H, d, J = 7.0 Hz), 2.35 (1H, d, J = 7.0 Hz), 2.58-2.68 (1H, m), 3.05-3.29 (2H, m), 3.56 (0.5H, dd, J = 15.8, 6.7 Hz), 3.64 (0.5H, dd, J = 15.8, 6.7 Hz), 3.92 (3H, s), 4.00-4.11 (1H, m), 4.51-4.60 (1H, m), 5.06-5.11 (1H, m), 5.60 (0.5H, s), 5.61 (0.5H, s), 6.99-7.05 (2H, m), 7.37-7.45 (2H, m), 7.74 (1H, d, J = 8.3 Hz), 7.84 (1H, d, J = 8.3 Hz).
Elemental analysis for: C₂₈H₂₅ClF₆N₄O₅
Calculated: C, 51.98; H, 3.89; N, 8.66
Found: C, 51.62; H, 3.83; N, 8.46.

### Example 688

tert-Butyl 2-(1-{2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetyl}-4-piperidinyl)acetate

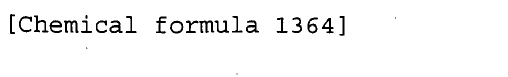

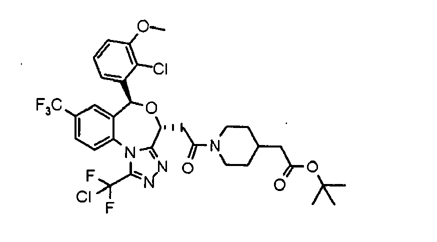

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (229 mg) was obtained from 2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetic acid (150 mg).
MS (FAB) m/z: 719 (M+H)⁺.
¹H-NMR (CDCl3) δ: 1.10-1.39 (2H, m), 1.45 (9H, s), 1.69-1.86 (2H, m), 1.95-2.07 (1H, m), 2.14 (1H, d, J = 6.8 Hz), 2.19 (1H, d, J = 6.8 Hz), 2.62 (1H, t, J = 12.8 Hz), 3.04-3.27 (2H, m), 3.51-3.65 (1H, m), 3.91 (3H, s), 3.98-4.08 (1H, m), 4.54 (1H, d, J = 12.8 Hz), 5.05 (1H, t, J = 6.6 Hz), 5.59 (0.5H, s), 5.61 (0.5H, s), 6.98-7.05 (2H, m), 7.40-7.42 (2H, m), 7.79 (1H, d, J = 8.3 Hz), 7.83 (1H, d, J = 8.3 Hz).

### Example 689

2-(1-{2-[(4R,6S)-1-[Chloro(difluoro)methyl]-6-(2-chloro-3-methoxyphenyl)-8-(trifluoromethyl)-4H,6H-[1,2,4]triazolo[4,3-a] [4,1]benzoxazepin-4-yl]acetyl}-4-piperidinyl)acetic acid

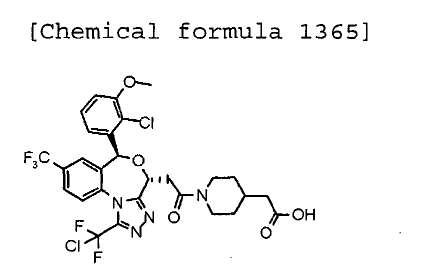

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (154 mg) was obtained from tert-butyl 2-(1-{2-[(4R,6S)-1-[chloro(difluoro)methyl]-6-(2-chloro-3-methoxyphenyl)-8-(trifluoromethyl)-4H,6H-[1,2,4]triazolo[4,3-a][4,1]benzoxazepin-4-yl]acetyl}-4-piperidinyl)acetate (185 mg).
MS (FAB) m/z: 663 (M+H)⁺.
¹H-NMR (CDCl3) δ: 1.12-1.27 (1.5H, m), 1.39-1.53 (0.5H, m), 1.74-1.92 (2H, m), 1.99-2.11 (1H, m), 2.27 (1H, d, J = 6.8 Hz), 2.34 (1H, d, J = 6.8 Hz), 2.63 (1H, t, J = 12.8 Hz), 3.05-3.29 (2H, m), 3.56 (1H, dd, J = 15.8, 7.3 Hz), 3.63 (1H, dd, J = 15.8, 7.3 Hz), 3.91 (3H, s), 4.00-4.11 (1H, m), 4.52-4.60 (1H, m), 5.03-5.08 (1H, m), 5.60 (0.5H, s), 5.61 (0.5H, s), 6.98-7.06 (2H, m), 7.37-7.44 (2H, m), 7.80 (1H, d, J = 8.3 Hz), 7.84 (1H, d, J = 8.3 Hz).
Elemental analysis for: C₂₈H₂₅Cl₂F₅N₄O₅
Calculated: C, 50.69; H, 3.80; N, 8.45
Found: C, 50.43; H, 3.73; N, 8.45.

### Example 690

tert-Butyl 2-(1-{2-[(4R,6S)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetyl}-4-piperidinyl)acetate

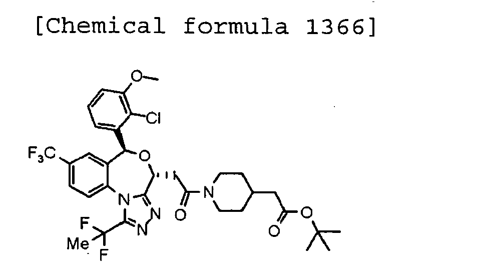

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (310 mg) was obtained from 2-[(4R,6S)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetic acid (200 mg).
MS (FAB) m/z: 699 (M+H)⁺.
¹H-NMR (CDCl3) δ: 1.09-1.41 (2H, m), 1.45 (9H, s), 1.69-1.84 (2H, m), 1.95-2.06 (1H, m), 2.13 (1H, d, J = 6.8 Hz), 2.18 (1H, d, J = 6.8 Hz), 2.33 (3H, t, J = 19.2 Hz), 3.03-3.27 (2H, m), 3.48-3.61 (1H, m), 3.91 (3H, s), 4.00-4.08 (1H, m), 4.51-4.59 (1H, m), 5.05 (1H, t, J = 6.6 Hz), 5.60 (0.5H, s), 5.62 (0.5H, s), 6.96 (1H, s), 6.99-7.03 (1H, m), 7.38-7.43 (2H, m), 7.79 (1H, d, J = 8.3 Hz), 7.91 (1H, d, J = 8.3 Hz).

### Example 691

2-(1-{2-[(4R,6S)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetyl}-4-piperidinyl)acetic acid

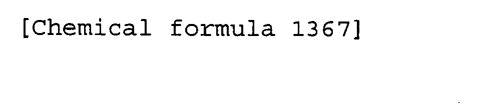

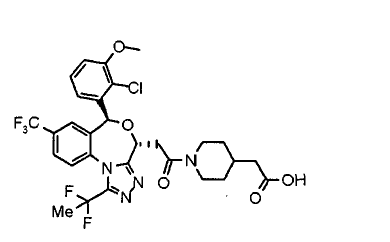

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (216 mg) was obtained from tert-butyl 2-(1-{2-[(4R,6S)-6-(2-chloro-3-methoxyphenyl)-1-(1,1-difluoroethyl)-8-(trifluoromethyl)-4H,6H-[1,2,4]triazolo[4,3-a][4,1]benzoxazepin-4-yl]acetyl}-4-piperidinyl)acetate (265 mg).
MS (FAB) m/z: 643 (M+H)⁺.
¹H-NMR (CDCl3) δ: 1.11-1.29 (1.5H, m), 1.38-1.47 (0.5H, m), 1.73-1.90 (2H, m), 1.99-2.12 (1H, m), 2.25-2.39 (5H, m), 2.63 (1H, t, J = 12.8 Hz), 3.04-3.30 (2H, m), 3.52 (0.5H, dd, J = 15.8, 7.3 Hz), 3.59 (0.5H, dd, J = 15.8, 7.3 Hz), 3.91 (3H, s), 4.02-4.11 (1H, m), 4.53-4.62 (1H, m), 5.05 (1H, t, J = 6.7 Hz), 5.60 (0.5H, s), 5.62 (0.5H, s), 6.95-6.97 (1H, m), 7.00-7.03 (1H, m), 7.38-7.43 (2H, m), 7.79 (1H, dd, J = 8.3, 1.7 Hz), 7.92 (1H, d, J = 8.3 Hz).
Elemental analysis for: C₂₉H₂₈ClF₅N₄O₅
Calculated: C, 54.17; H, 4.39; N, 8.71
Found: C, 54.54; H, 4.54; N, 8.27.

### Example 692

tert-Butyl 2-(1-{2-[(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetyl}-4-piperidinyl)acetate

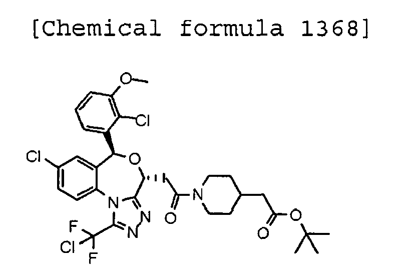

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetate, the title compound (400 mg) was obtained from 2-[(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetic acid (300 mg).
MS (FAB) m/z: 685 (M+H)⁺.
¹H-NMR (CDCl3) δ: 1.09-1.23 (1.5H, m), 1.32-1.41 (0.5H, m), 1.45 (9H, s), 1.68-1.85 (2H, m), 1.94-2.06 (1H, m), 2.14 (1H, d, J = 7.0 Hz), 2.19 (1H, d, J = 7.0 Hz), 2.62 (1H, t, J = 12.8 Hz), 3.03-3.27 (2H, m), 3.53 (0.5H, dd, J = 15.3, 6.7 Hz), 3.58 (0.5H, dd, J = 15.3, 6.7 Hz), 3.91 (3H, s), 3.99-4.08 (1H, m), 4.55 (1H, d, J = 12.8 Hz), 5.07 (1H, t, J = 6.7 Hz), 5.52 (0.5H, s), 5.53 (0.5H, s), 6.71 (1H, s), 6.99-7.03 (1H, m), 7.35-7.43 (2H, m), 7.52 (1H, dd, J = 8.5, 2.2 Hz), 7.59 (1H, d, J = 8.5 Hz).

### Example 693

2-(1-{2-[(4R,6S)-8-Chloro-1-[chloro(difluoro)methyl]-6-(2-chloro-3-methoxyphenyl)-4H,6H-[1,2,4]triazolo[4,3-a][4,1]benzoxazepin-4-yl]acetyl}-4-piperidinyl)acetic acid

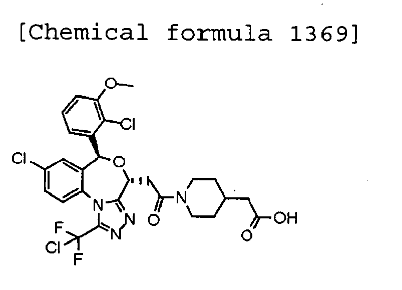

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (332 mg) was obtained from tert-butyl 2-(1-{2-[(4R,6S)-8-chloro-1-[chloro(difluoro)methyl]-6-(2-chloro-3-methoxyphenyl)-4H,6H-[1,2,4]triazolo[4,3-a][4,1]benzoxazepin-4-yl]acetyl}-4-piperidinyl)acetate (395 mg).
MS (FAB) m/z: 629 (M+H)⁺.
¹H-NMR (CDCl3) δ: 1.13-1.28 (1.5H, m), 1.39-1.52 (0.5H, m), 1.73-1.90 (2H, m), 1.99-2.12 (1H, m), 2.27 (1H, d, J = 6.8 Hz), 2.34 (1H, d, J = 6.8 Hz), 2.63 (2H, t, J = 12.8 Hz), 3.05-3.31 (2H, m), 3.52 (0.5H, dd, J = 15.8, 7.3 Hz), 3.52 (0.5H, dd, J = 10.0, 5.0 Hz), 3.91 (3H, s), 4.01-4.12 (1H, m), 4.53-4.61 (1H, m), 5.04-5.10 (1H, m), 5.53 (0.5H, s), 5.54 (0.5H, s), 6.71 (1H, s), 6.99-7.03 (1H, m), 7.34-7.43 (2H, m), 7.53 (1H, dd, J = 8.5, 2.2 Hz), 7.59 (1H, d, J = 8.5 Hz).
Elemental analysis for: C₂₇H₂₅Cl₃F₂N₄O₅·0.5H₂O
Calculated: C, 50.76; H, 4.10; N, 8.77
Found: C, 50.93; H, 3.98; N, 8.58.

### Example 694

tert-Butyl 2-(1-{2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetyl}-4-piperidinyl)acetate

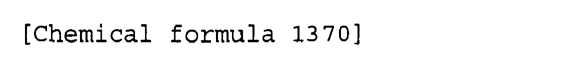

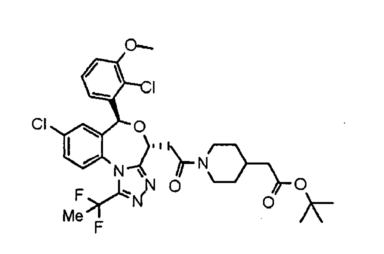

In a similar manner to that employed for the synthesis of tert-butyl 2-(1-{2-[(4R,6S)-8-chloro-6-(2-chloro-3-methoxyphenyl)-1-(trifluoromethyl)-4H,6H-[1,2,4]triazolo[4,3-a] [4,1]benzoxazepin-4-yl]acetyl}-4-piperidinyl)acetate, the title compound (564 mg) was obtained from 2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetic acid (400 mg) .
MS (FAB) m/z: 665 (M+H)⁺.
¹H-NMR (CDCl3) δ: 0.94-0.94 (1.5H, m), 1.32-1.41 (0.5H, m), 1.45 (9H, s), 1.68-1.84 (2H, m), 1.94-2.05 (1H, m), 2.13 (1H, d, J = 7.0 Hz), 2.18 (1H, d, J = 7.0 Hz), 2.31 (3H, t, J = 19.2 Hz), 2.56-2.68 (1H, m), 3.02-3.27 (2H, m), 3.43-3.58 (1H, m), 3.91 (3H, s), 4.05 (1H, d, J = 12.8 Hz), 4.51-4.61 (1H, m), 5.06 (1H, t, J = 6.5 Hz), 5.53 (0.5H, s), 5.54 (0.5H, s), 6.66 (1H, s), 6.97-7.03 (1H, m), 7.36-7.42 (2H, m), 7.49 (1H, dd, J = 8.8, 2.2 Hz), 7.71 (1H, d, J = 8.8 Hz) .

### Example 695

2-(1-{2-[(4R,6S)-8-Chloro-6-(2-chloro-3-methoxyphenyl)-1-(1,1-difluoroethyl)-4H,6H-[1,2,4]triazolo[4,3-a][4,1]benzoxazepin-4-yl]acetyl}-4-piperidinyl)acetic acid

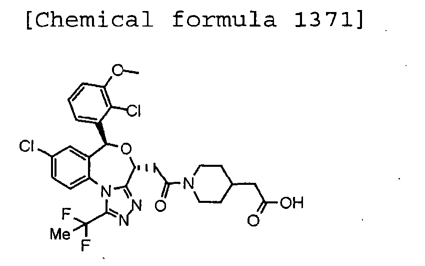

In a similar manner to that employed for the synthesis of 2-(1-{2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}-4-piperidinyl)acetic acid, the title compound (450 mg) was obtained from tert-butyl 2-(1-{2-[(4R,6S)-8-chloro-6-(2-chloro-3-methoxyphenyl)-1-(1,1-difluoroethyl)-4H,6H-[1,2,4]triazolo[4,3-a][4,1]benzoxazepin-4-yl]acetyl}-4-piperidinyl)acetate (559 mg).
MS (FAB) m/z: 609 (M+H)⁺.
¹H-NMR (CDCl3) δ: 1.13-1.28 (1.5H, m), 1.36-1.48 (0.5H, m), 1.73-1.91 (2H, m), 1.99-2.11 (1H, m), 2.24-2.36 (5H, m), 2.63 (1H, t, J = 12.8 Hz), 3.03-3.30 (2H, m), 3.48 (0.5H, dd, J = 15.6, 7.0 Hz), 3.56 (0.5H, dd, J = 15.6, 7.0 Hz), 3.91 (3H, s), 4.03-4.11 (1H, m), 4.54-4.63 (1H, m), 5.06 (1H, t, J = 6.6 Hz), 5.53 (0.5H, s), 5.55 (0.5H, s), 6.67 (1H, s), 6.97-7.03 (1H, m), 7.35-7.42 (2H, m), 7.49 (1H, dd, J = 8.5, 2.2 Hz), 7.71 (1H, d, J = 8.5 Hz).
Elemental analysis for: C₂₈H₂₈Cl₂F₂N₄O₅·0.5H₂O
Calculated: C, 54.38; H, 4.73; N, 9.06
Found: C, 54.64; H, 4.66; N, 8.72.

### Referential Example 471

tert-Butyl 4-chloro-2-chloro-2-[hydroxy(2-methoxy-3-pyridinyl)methyl]phenylcarbamate

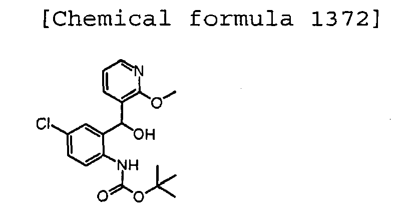

tert-Butyl 4-chlorophenylcarbamate (3.65 g) was dissolved in tetrahydrofuran (80 mL). At -78°C, s-butyl lithium (a 1.0M cyclohexane, n-hexane solution, 35.0 mL) was added dropwise over 20 minutes to the resulting solution. After stirring at the same temperature for 30 minutes, the reaction mixture was warmed to -15°C and stirred for 45 minutes. The reaction mixture was cooled to -78°C again and a solution of 2-methoxypyridine-3-carbaldehyde (2.0 g) in tetrahydrofuran (20 mL) was added dropwise. While gradually warming to 0°C, the reaction mixture was stirred for 4 hours. Under ice cooling, a saturated aqueous solution of ammonium chloride was added. The resulting mixture was extracted with ethyl acetate, washed with saturated brine, and dried over anhydrous sodium sulfate. The solvent was distilled off under reduced pressure. The residue was purified by silica gel column chromatography (hexane-ethyl acetate) to give the title compound (4.73 g).
¹H-NMR (CDCl₃) δ: 1.5 (9H, s), 3.4-3.4 (1H, m), 4.0 (3H, s), 6.0 (1H, d, J = 3.9 Hz), 6.9-6.9 (1H, m), 7.1-7.1 (1H, m), 7.2-7.3 (1H, m), 7.4-7.5 (1H, m), 7.8-8.0 (2H, m), 8.1-8.2 (1H, m).

### Referential Example 472

(2-Amino-5-chlorophenyl)(2-methoxy-3-pyridine)methanol

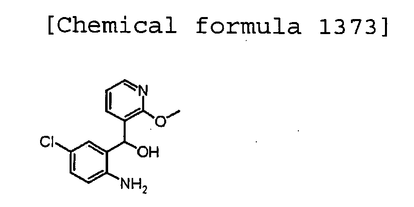

tert-Butyl 4-chloro-2-chloro-2-[hydroxy(2-methoxy-3-pyridinyl)methyl]phenylcarbamate (4.73 g) was dissolved in 1,4-dioxane (80 mL). Under ice cooling, concentrated hydrochloric acid (40 mL) was added and the resulting mixture was stirred at room temperature for 40 minutes. Under ice cooling, a 5N aqueous sodium hydroxide solution was added to make it alkaline, followed by extraction with ethyl acetate. The extract was washed with saturated brine and dried over anhydrous sodium sulfate. The solvent was distilled off under reduced pressure to give the title compound (3.21 g).
¹H-NMR (CDCl₃) δ: 3.2 (1H, br s), 4.0 (3H, s), 4.2 (2H, br s), 5.9 (1H, br s), 6.6 (1H, d, J = 8.3 Hz), 6.9 (1H, dd, J = 7.3, 5.1 Hz), 7.0 (1H, d, J = 2.7 Hz), 7.1 (1H, dd, J = 8.3, 2.4 Hz), 7.4 (1H, dd, J = 7.1, 1.5 Hz), 8.1 (1H, dd, J = 5.0, 1.8 Hz).

### Referential Example 473

{5-Chloro-2-[(2,4-dimethoxybenzyl)amino]phenyl}(2-methoxy-3-pyridinyl)methanol

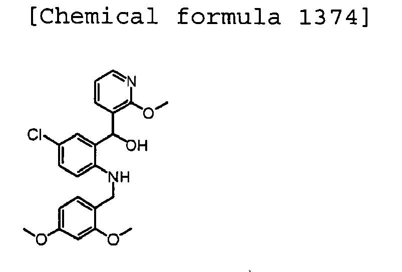

(2-Amino-5-chlorophenyl)(2-methoxy-3-pyridine)methanol(1.0 g), 2,4-dimethoxybenzaldehyde (942 mg) was dissolved in methanol (40 mL). Under ice cooling, acetic acid (4 mL) and sodium borohydride (233 mg) were added and the resulting mixture was stirred at room temperature for 1 hour. Under ice cooling, ethyl acetate and a saturated aqueous solution of sodium bicarbonate were added. The resulting mixture was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (1.52 g).
¹H-NMR (CDCl₃) δ: 3.2 (1H, br s), 3.7-3.8 (6H, m), 3.9-4.0 (3H, m), 4.2 (2H, br s), 5.2 (1H, br s), 5.9 (1H, br s), 6.4-6.5 (3H, m), 6.6 (1H, d, J = 8.8 Hz), 6.8-7.1 (3H, m), 7.4 (1H, dd, J = 7.3, 2.0 Hz), 8.1 (1H, dd, J = 5.0, 1.8 Hz) .

### Referential Example 474

Ethyl (Z)-4-{4-chloro(2,4-dimethoxybenzyl)-2-[hydroxy(2-methoxy-3-pyridinyl)methyl]anilino}-4-oxo-2-butenoate

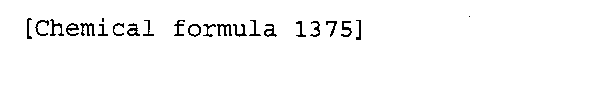

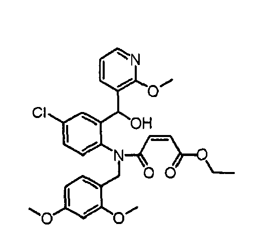

{5-Chloro-2-[(2,4-dimethoxybenzyl)amino]phenyl}(2-methoxy-3-pyridinyl)methanol (1.52 g) was dissolved in dichloromethane (80 mL). Under ice cooling, sodium hydrogen carbonate (709 mg) was added. To the resulting mixture was added dropwise a solution of monoethyl chlorofumarate (1.13 g) in dichloromethane (20 mL). After stirring for 20 hours, water was added and the resulting mixture was extracted with dichloromethane. After the extract was dried over anhydrous sodium sulfate, the solvent was distilled off under reduced pressure. The residue was purified by silica gel column chromatography (hexane-ethyl acetate) to give the title compound (1.20 g).

### Referential Example 475

Ethyl 2-[trans-7-chloro-1-(2,4-dimethoxybenzyl)-5-(2-methoxy-3-pyridinyl)-2-oxo-1,2,3,5-tetrahydro-4,1-benzoxazepin-3-yl]acetate

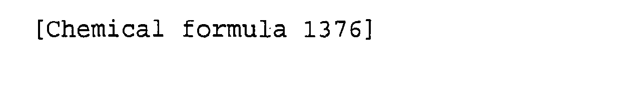

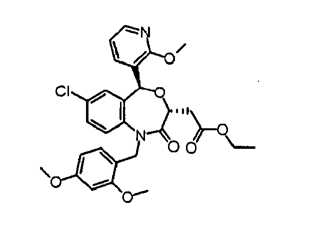

Ethyl (Z)-4-{4-chloro(2,4-dimethoxybenzyl)-2-[hydroxy(2-methoxy-3-pyridinyl)methyl]anilino}-4-oxo-2-butenoate (1.20 g) was dissolved in ethanol (15 mL). While stirring at room temperature, potassium carbonate (458 mg) was added to the resulting solution. After stirring for 23 hours, ethyl acetate was added. The resulting mixture was washed with saturated brine and dried over anhydrous sodium sulfate. The solvent was distilled off under reduced pressure. The residue was purified by silica gel column chromatography (hexane-ethyl acetate) to give the title compound (1.10 g).
¹H-NMR (CDCl₃) δ: 1.3 (3H, t, J = 7.2 Hz), 2.8 (1H, dd, J = 16.6, 5.9 Hz), 3.1 (1H, dd, J = 16.5, 7.7 Hz), 3.6-3.7 (6H, m), 3.7-3.8 (4H, m), 4.1 (2H, q, J = 7.1 Hz), 4.5 (1H, dd, J = 7.6, 5.9 Hz), 4.9 (1H, d, J = 14.6 Hz), 5.4 (1H, d, J = 14.4 Hz), 5.8 (1H, s), 6.4-6.5 (3H, m), 7.0-7.0 (1H, m), 7.2-7.3 (2H, m), 8.1-8.2 (1H, m), 8.1-8.2 (1H, m).

### Referential Example 476

Ethyl 2-[trans-7-chloro-5-(2-methoxy-3-pyridinyl)-2-oxo-1,2,3,5-tetrahydro-4,1-benzoxazepin-3-yl]acetate

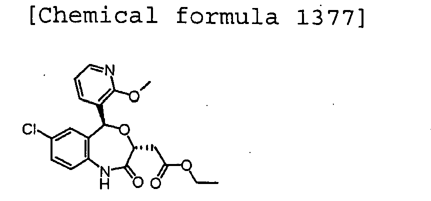

Ethyl 2-[trans-7-chloro-1-(2,4-dimethoxybenzyl)-5-(2-methoxy-3-pyridinyl)-2-oxo-1,2,3,5-tetrahydro-4,1-benzoxazepin-3-yl]acetate (551 mg) was dissolved in acetone (50 mL). Under ice cooling, an aqueous solution (10 mL) of ceric(IV) ammonium nitrate (1.67 g) was added and the resulting mixture was stirred at room temperature for 5 hours. Under ice cooling, an aqueous solution (10 mL) of ceric(IV) ammonium nitrate (1 g) was added again and the resulting mixture was stirred at room temperature for 1 hour. Under ice cooling, a saturated aqueous solution of sodium bicarbonate and ethyl acetate were added. The insoluble material was filtered off from the reaction mixture through celite. The filtrate was extracted with ethyl acetate and dried over saturated brine. After drying over anhydrous sodium sulfate, the solvent was distilled off under reduced pressure. The residue was purified by silica gel column chromatography (hexane-ethyl acetate) to give the title compound (314 mg).
¹H-NMR (CDCl₃) δ: 1.2 (3H, t, J = 7.1 Hz), 2.8 (1H, dd, J = 16.5, 6.7 Hz), 3.0 (1H, dd, J = 16.5, 6.7 Hz), 3.8 (3H, s), 4.1 (2H, q, J = 7.1 Hz), 4.6 (1H, t, J = 6.6 Hz), 6.1 (1H, s), 6.6-6.6 (1H, m), 7.0-7.0 (2H, m), 7.3-7.3 (1H, m), 7.7-7.8 (1H, m), 7.8-7.9 (1H, m), 8.2-8.2 (1H, m).

### Referential Example 477

Ethyl 2-[trans-7-chloro-5-(2-methoxy-3-pyridinyl)-2-thioxo-1,2,3,5-tetrahydro-4,1-benzoxazepin-3-yl]acetate

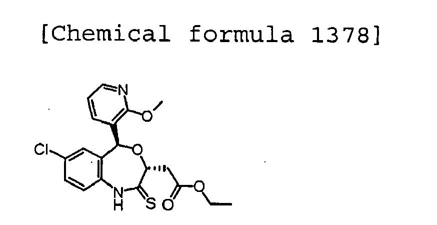

Ethyl 2-[trans-7-chloro-5-(2-methoxy-3-pyridinyl)-2-oxo-1,2,3,5-tetrahydro-4,1-benzoxazepin-3-yl]acetate (314 mg) was dissolved in tetrahydrofuran (10 mL). At room temperature, sodium hydrogen carbonate (142 mg) and diphosphorus pentasulfide (375 mg) were added and the resulting mixture was stirred for 9 hours. To the reaction mixture was added ethyl acetate. The resulting mixture was washed with a saturated aqueous solution of sodium bicarbonate and saturated brine and dried over anhydrous sodium sulfate. The solvent was distilled off under reduced pressure. Dichloromethane-hexane was added to the residue and the solid thus formed was collected by filtration to give the title compound (290 mg).
¹H-NMR (CDCl₃) δ: 1.2 (3H, t, J = 7.2 Hz), 3.0 (1H, dd, J = 16.5, 6.5 Hz), 3.3 (1H, dd, J = 16.6, 6.6 Hz), 3.8 (3H, s), 4.1 (2H, q, J = 7.1 Hz), 4.7 (1H, t, J = 6.6 Hz), 6.0 (1H, s), 6.6-6.6 (1H, m), 7.0-7.1 (2H, m), 7.3-7.4 (1H, m), 7.8-7.9 (1H, m), 8.2-8.3 (1H, m), 9.7 (1H, br s).

### Example 696

Ethyl 2-[trans-8-chloro-6-(2-methoxy-3-pyridinyl)-1-(trifluoromethyl)-4H,6H-[1,2,4]triazolo[4,3-a][4,1]benzoxazepin-4-yl]acetate

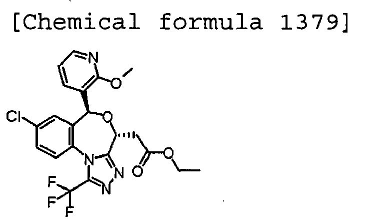

Ethyl 2-[trans-7-chloro-5-(2-methoxy-3-pyridinyl)-2-thioxo-1,2,3,5-tetrahydro-4,1-benzoxazepin-3-yl]acetate (290 mg) was dissolved in tetrahydrofuran (10 mL). At room temperature, hydrazine monohydrate (0.069 mL) was added and the resulting mixture was stirred for 3 hours. To the reaction mixture was added dichloroethane (10 mL). Under ice cooling, trifluoroacetic anhydride (0.30 mL) was added and the resulting mixture was stirred at room temperature for 19 hours. Under ice cooling, trifluoroacetic anhydride (0.30 mL) was added again and the resulting mixture was stirred for 8 hours. The solvent was distilled off under reduced pressure. Chloroform was added to the residue. Under ice cooling, a saturated aqueous solution of sodium bicarbonate was added, followed by extraction with chloroform. The extract was dried over anhydrous sodium sulfate. The solvent was distilled off under reduced pressure. To the residue was added dichloromethane-hexane and the solid thus formed was filtered off. The filtrate was concentrated and the residue was purified by silica gel column chromatography (hexane-ethyl acetate) to give the title compound (87 mg).
¹H-NMR (CDCl₃) δ: 1.3 (3H, t, J = 7.1 Hz), 3.2 (1H, dd, J = 16.8, 7.1 Hz), 3.5 (1H, dd, J = 16.7, 6.5 Hz), 3.7 (3H, s), 4.2 (2H, q, J = 7.2 Hz), 4.9 (1H, t, J = 6.8 Hz), 5.4 (1H, s), 6.8 (1H, s), 7.0 (1H, dd, J = 7.4, 5.0 Hz), 7.5 (2H, d, J = 2.2 Hz), 7.9-7.9 (1H, m), 8.2-8.2 (1H, m).

### Example 697

2-[trans-8-Chloro-6-(2-methoxy-3-pyridinyl)-1-(trifluoromethyl)-4H,6H-[1,2,4]triazolo[4,3-a] [4,1]benzoxazepin-4-yl]acetic acid

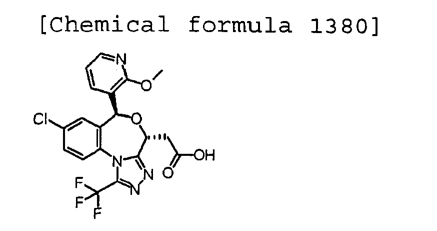

Ethyl 2-[trans-8-chloro-6-(2-methoxy-3-pyridinyl)-1-(trifluoromethyl)-4H,6H-[1,2,4]triazolo[4,3-a][4,1]benzoxazepin-4-yl]acetate (82 mg) was dissolved in 1,4-dioxane. Under ice cooling, concentrated hydrochloric acid (0.82 mL) was added and the resulting mixture was stirred at room temperature for 26 hours. Water was added to the reaction mixture, followed by extraction with ethyl acetate. The extract was washed with saturated brine and dried over anhydrous sodium sulfate. The solvent was distilled off under reduced pressure. The residue was purified by silica gel column chromatography (chloroform-methanol) to give the title compound (45 mg).
¹H-NMR (CDCl₃) δ: 3.2-3.4 (1H, m), 3.5-3.6 (1H, m), 3.7 (3H, s), 4.9-4.9 (1H, m), 5.4 (1H, s), 6.8 (1H, s), 7.0-7.1 (1H, m), 7.5 (2H, s), 7.9-8.0 (1H, m), 8.3-8.2 (1H, m).
Elemental analysis for: C₁₉H₁₄ClF₃N₄O₄·0.5H₂O
Calculated: C, 49.20; H, 3.26; N, 12.08.
Found: C, 49.50; H, 3.03; N, 11.86.

### Referential Example 478

tert-Butyl (4-chloro-2-{hydroxy[2-(trifluoromethyl)pyridin-3-yl]methyl}phenyl)carbamate

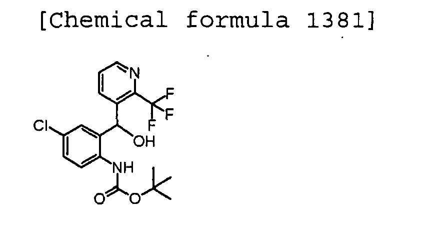

tert-Butyl 4-chlorophenylcarbamate (1.57 g) was dissolved in tetrahydrofuran (35 mL). The resulting solution was cooled to -65°C or less and sec-butyl lithium (a 1.0M hexane solution, 15.0 mL) was added dropwise thereto over 5 minutes. Five minutes later, the reaction mixture was warmed to -20°C and then, cooled again to -60°C or less. A tetrahydrofuran solution (3 mL) of 2-trifluoromethylpyridine-3-carbaldehyde (1.33 g) was added dropwise over 5 minutes. While warming to room temperature, the resulting mixture was stirred for one hour. The reaction mixture was poured into saturated aqueous ammonium chloride, followed by extraction with ethyl acetate. The organic layer was washed with saturated brine and dried over anhydrous sodium sulfate. The solvent was distilled off under reduced pressure. The residue was purified by silica gel column chromatography (20% ethyl acetate-hexane) to give the title compound (2.37 g).
MS (ESI) m/z: 403 (M+H)⁺.
¹H-NMR (CDCl₃) δ: 1.49 (9H, s), 6.34 (1H, d, J = 2.20 Hz), 6.88 (1H, s), 6.92 (1H, d, J = 2.44 Hz), 7.26 (1H, dd, J = 8.54, 2.44 Hz), 7.48 (1H, d, J = 8.54 Hz), 7.60 (1H, dd, J = 8.06, 4.64 Hz), 8.23 (1H, d, J = 7.81 Hz), 8.68 (1H, dd, J = 4.52, 1.34 Hz).

### Referential Example 479

(2-Amino-5-chlorophenyl)[2-(trifluoromethyl)pyridin-3-yl]methanol

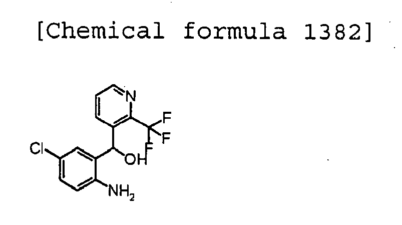

tert-Butyl (4-chloro-2-{hydroxy[2-(trifluoromethyl)pyridin-3-yl]methyl}phenyl)carbamate (2.36 g) was dissolved in dioxane (10 mL). To the resulting solution was added concentrated hydrochloric acid (7 mL) and the resulting mixture was stirred at room temperature for 1.25 hours. The reaction mixture was concentrated under reduced pressure. To the residue was added 1N aqueous sodium hydroxide to make it alkaline, followed by extraction with ethyl acetate. The organic layer was washed with saturated brine and dried over anhydrous sodium sulfate. The solvent was distilled off under reduced pressure. The residue was purified by silica gel column chromatography (30% ethyl acetate-hexane) to give the title compound (1.75 g).
MS (ESI) m/z: 303 (M+H)⁺.
¹H-NMR (CDCl₃) δ: 6.28 (1H, s), 6.65 (1H, d, J = 8.54 Hz), 6.87 (1H, d, J = 2.44 Hz), 7.10 (1H, dd, J = 8.30, 2.44 Hz), 7.55 (1H, dd, J = 8..06, 4.64 Hz), 8.03-8.05 (1H, m), 8.69 (1H, dd, J = 4.64, 1.46 Hz).

### Referential Example 480

{5-Chloro-2-[(2,4-dimethoxybenzyl)amino]phenyl}[2-(trifluoromethyl)pyridin-3-yl]methanol

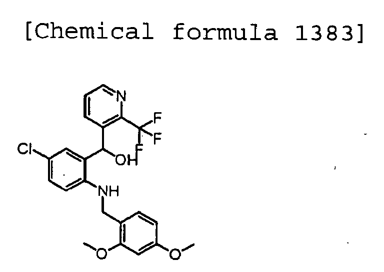

(2-Amino-5-chlorophenyl)[2-(trifluoromethyl)pyridin-3-yl]methanol (1.74 g) was dissolved in acetic acid (30 mL). To the resulting solution was added 2,4-dimethoxybenzaldehyde (1.15 g) and the resulting mixture was stirred at room temperature for 2.5 hours. The reaction mixture was cooled with ice water and sodium borohydride (0.326 g) was added thereto. The resulting mixture was stirred at room temperature for one hour. The reaction mixture was diluted with ethyl acetate, and washed twice with water and then, with saturated brine. After drying over anhydrous sodium sulfate, the solvent was distilled off under reduced pressure to give the title compound (2.93 g).
MS (ESI) m/z: 475 (M+Na)⁺.
¹H-NMR (CDCl₃) δ: 3.74 (3H, s), 3.78 (3H, s), 4.22 (2H, s), 6.24 (1H, s), 6.39 (1H, dd, J = 8.18, 2.32 Hz), 6.43 (1H, d, J = 2.44 Hz), 6.69 (1H, d, J = 8.54 Hz), 6.84 (1H, d, J = 2.44 Hz), 7.04 (1H, d, J = 8.30 Hz), 7.14 (1H, dd, J = 8.79, 2.44 Hz), 7.51 (1H, dd, J = 8.06, 4.64 Hz), 7.98 (1H, d, J = 6.59 Hz), 8.67 (1H, dd, J = 4.64, 1.46 Hz).

### Referential Example 481

Ethyl {trans-7-chloro-1-(2,4-dimethoxybenzyl)-2-oxo-5-[2-(trifluoromethyl)pyridin-3-yl]-1,2,3,5-tetrahydro-4,1-benzoxazepin-3-yl}acetate

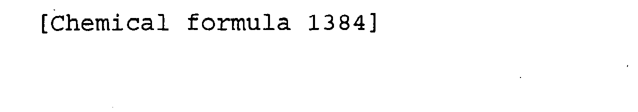

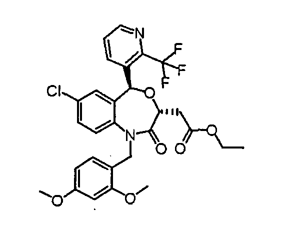

{5-Chloro-2-[(2,4-dimethoxybenzyl)amino]phenyl}[2-(trifluoromethyl)pyridin-3-yl]methanol (2.93 g) was dissolved in ethyl acetate (25 mL). To the resulting solution was added sodium hydrogen carbonate (0.966 g). An ethyl acetate solution (5 mL) of monoethyl chlorofumarate (0.982 g) was added dropwise and the resulting mixture was stirred at room temperature for 3 hours. The reaction mixture was diluted with ethyl acetate, washed with water and saturated brine, and dried over anhydrous sodium sulfate. The residue (3.51 g) obtained by distilling off the solvent was dissolved in ethanol(50 mL). Potassium carbonate (1.66 g) was added and the resulting mixture was stirred at room temperature for one hour. The residue obtained by concentrating the reaction mixture under reduced pressure was dissolved in ethyl acetate. The resulting solution was washed with water and saturated brine and dried over anhydrous sodium sulfate. The solvent was distilled off under reduced pressure. The residue was subjected to silica gel column chromatography (40% ethyl acetate-hexane) to give the title compound (3.08 g).
MS (ESI) m/z: 579 (M+H)⁺.
¹H-NMR (CDCl₃) δ: 1.25 (3H, t, J = 7.19 Hz), 2.81 (1H, dd, J = 16.71, 6.46 Hz), 3.07 (1H, dd, J = 16.71, 6.95 Hz), 3.67 (3H, s), 3.79 (3H, s), 4.15 (2H, q, J = 7.01 Hz), 4.54 (1H, t, J = 6.71 Hz), 4.98 (1H, d, J = 15.12 Hz), 5.34 (1H, d, J = 15.12 Hz), 6.00 (1H, s), 6.27 (1H, d, J = 2.20 Hz), 6.41 (1H, d, J = 2.44 Hz), 6.44 (1H, dd, J = 8.54, 2.44 Hz), 7.27 (1H, d, J = 7.07 Hz), 7.35-7.37 (2H, m), 7.63 (1H, dd, J = 8.05, 4.63 Hz), 8.26 (1H, d, J = 7.56 Hz), 8.72 (1H, dd, J = 4.63, 1.46 Hz).

### Referential Example 482

Ethyl {trans-7-chloro-2-oxo-5-[2-(trifluoromethyl)pyridin-3-yl]-1,2,3,5-tetrahydro-4,1-benzoxazepin-3-yl}acetate

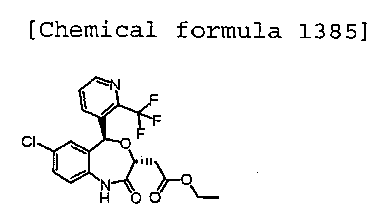

Ethyl {trans-7-chloro-1-(2,4-dimethoxybenzyl)-2-oxo-5-[2-(trifluoromethyl)pyridin-3-yl]-1,2,3,5-tetrahydro-4,1-benzoxazepin-3-yl}acetate (3.08 g) was dissolved in acetone (80 mL). To the resulting solution was added an aqueous solution (20 mL) of ceric ammonium nitrate (8.75 g). The resulting mixture was stirred at room temperature for 2.5 hours. To the reaction mixture were added a saturated aqueous solution of sodium bicarbonate and ethyl acetate. The resulting mixture was filtered through celite. The filtrate was extracted with ethyl acetate. The organic layer was washed with saturated brine and dried over anhydrous sodium sulfate. The solvent was distilled off under reduced pressure. The residue was subjected to silica gel column chromatography (30% ethyl acetate-hexane) to give the title compound (1.75 g).
MS (ESI) m/z: 429 (M+H)⁺.
¹H-NMR (CDCl₃) δ: 1.23 (3H, t, J = 7.19 Hz), 2.81 (1H, dd, J = 16.83, 7.07 Hz), 3.03 (1H, dd, J = 16.83, 6.10 Hz), 4.12 (2H, q, J = 7.15 Hz), 4.68 (1H, t, J = 6.59 Hz), 6.28 (1H, s), 6.40 (1H, d, J = 2.20 Hz), 7.06 (1H, d, J = 8.54 Hz), 7.35 (1H, dd, J = 8.41, 2.32 Hz), 7.67 (1H, dd, J = 8.05, 4.63 Hz), 7.75 (1H, s), 8.26 (1H, d, J = 8.05 Hz), 8.77 (1H, dd, J = 4.76, 1.34 Hz).

### Referential Example 483

Ethyl {trans-7-chloro-2-thioxo-5-[2-(trifluoromethyl)pyridin-3-yl]-1,2,3,5-tetrahydro-4,1-benzoxazepin-3-yl}acetate

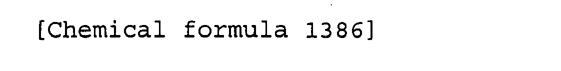

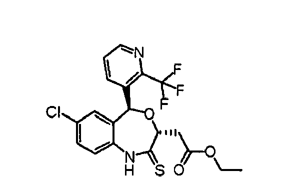

Ethyl {trans-7-chloro-2-oxo-5-[2-(trifluoromethyl)pyridin-3-yl]-1,2,3,5-tetrahydro-4,1-benzoxazepin-3-yl}acetate (1.75 g) was dissolved in tetrahydrofuran (40 mL). Under cooling with ice water, sodium hydrogen carbonate (0.754 g) and diphosphorus pentasulfide (1.81 g) were added and the resulting mixture was stirred at room temperature for 4 hours. Saturated brine was added and the resulting mixture was extracted with ethyl acetate. The organic layer was washed with saturated brine and then, dried over anhydrous sodium sulfate. The solvent was distilled off under reduced pressure. The residue was subjected to silica gel column chromatography (20% ethyl acetate-hexane) to give the title compound (1.51 g).
MS (ESI) m/z: 445 (M+H)⁺.
¹H-NMR (CDCl₃) δ: 1.25 (3H, t, J = 7.07 Hz), 2.99 (1H, dd, J = 16.71, 7.19 Hz), 3.22 (1H, dd, J = 16.83, 6.10 Hz), 4.12-4.17 (2H, m), 4.74 (1H, dd, J = 7.07, 6.10 Hz), 6.20 (1H, s), 6.41 (1H, d, J = 2.20 Hz), 7.12 (1H, d, J = 8.29 Hz), 7.40 (1H, dd, J = 8.41, 2.32 Hz), 7.67 (1H, dd, J = 8.05, 4.63 Hz), 8.28 (1H, d, J = 7.32 Hz), 8.78 (1H, dd, J = 4.63, 1.46 Hz), 9.61 (1H, s).

### Example 698

Ethyl {trans-8-chloro-1-(trifluoromethyl)-6-[2-(trifluoromethyl)pyridin-3-yl]-4H,6H-[1,2,4]triazolo[4,3-a][4,1]benzoxazepin-4-yl}acetate

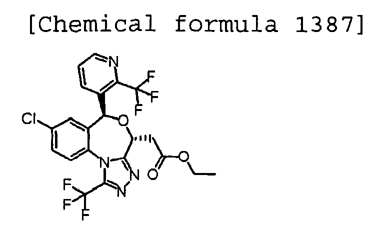

Ethyl {trans-7-chloro-2-thioxo-5-[2-(trifluoromethyl)pyridin-3-yl]-1,2,3,5-tetrahydro-4,1-benzoxazepin-3-yl}acetate (1.51 g) was dissolved in tetrahydrofuran (25 mL). Under cooling with ice water, hydrazine hydrate (0.50 mL) was added and the resulting mixture was stirred at room temperature for 100 minutes. To the reaction mixture was added saturated brine and the resulting mixture was extracted with ethyl acetate. The organic layer was washed twice with saturated brine and dried over anhydrous sodium sulfate. The solvent was distilled off under reduced pressure. The residue thus obtained was dissolved in dichloroethane (25 mL). Under cooling with ice water, trifluoroacetic anhydride (1.42 mL) was added and the resulting mixture was stirred at room temperature for 2.5 hours. To the reaction mixture was added trifluoroacetic acid (1 mL) and the resulting mixture was stirred at 70°C for one hour. After the reaction mixture was returned to room temperature, the solvent was distilled off under reduced pressure. The residue was dissolved in dichloromethane. The organic layer was washed with a saturated aqueous solution of sodium bicarbonate and saturated brine and dried over anhydrous sodium sulfate. The solvent was distilled off under reduced pressure. The residue was subjected to silica gel column chromatography (40% ethyl acetate-hexane) to give the title compound (1.05 g).
MS (ESI) m/z: 521 (M+H)⁺.
¹H-NMR (CDCl₃) δ: 1.29 (3H, t, J = 7.19 Hz), 3.27 (1H, dd, J = 16.83, 7.56 Hz), 3.49 (1H, dd, J = 17.07, 5.85 Hz), 4.21 (2H, q, J = 6.99 Hz), 5.00 (1H, dd, J = 7.56, 6.10 Hz), 5.66 (1H, s), 6.61 (1H, d, J = 1.95 Hz), 7.52-7.63 (2H, m), 7.70 (1H, dd, J = 8.05, 4.63 Hz), 8.34 (1H, d, J = 7.80 Hz), 8.79 (1H, dd, J = 4.63, 1.46 Hz).

### Example 699

{(trans-8-Chloro-1-(trifluoromethyl)-6-[2-(trifluoromethyl)pyridin-3-yl]-4H,6H-[1,2,4]triazolo(4,3-a] [4,1]benzoxazepin-4-yl)acetic acid

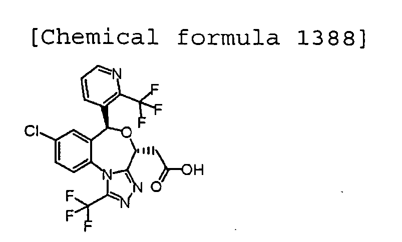

Ethyl {trans-8-chloro-1-(trifluoromethyl)-6-[2-(trifluoromethyl)pyridin-3-yl]-4H,6H-[1,2,4]triazolo[4,3-a][4,1]benzoxazepin-4-yl}acetate (0.200 g) was dissolved in dioxane (6 mL). To the resulting solution was added concentrated hydrochloric acid (3 mL). The resulting mixture was stirred at room temperature for 2 hours and then at 60°C for 8 hours. After cooling, the reaction mixture was concentrated to dryness under reduced pressure. To the crystalline residue thus obtained was added water and the resulting mixture was stirred at room temperature for 5 hours. The resulting solid was collected by filtration and washed with water to give the title compound (0.176 g).
MS (ESI) m/z: 493 (M+H)⁺.
¹H-NMR (CDCl₃) δ: 3.35 (1H, dd, J = 17.33, 7.32 Hz), 3.58 (1H, dd, J = 17.33, 6.10 Hz), 4.98 (1H, dd, J = 7.20, 5.98 Hz), 5.67 (1H, s), 6.62 (1H, d, J = 1.71 Hz), 7.55-7.63 (2H, m), 7.72 (1H, dd, J = 7.93, 4.52 Hz), 8.35 (1H, d, J = 7.81 Hz), 8.79-8.82 (1H, m).
Elemental analysis for: C₁₉H₁₁ClN₄O₃F₆
Calculated: C, 46.31; H, 2.25; Cl, 7.19; N, 11.37; F, 23.13.
Found: C, 46.27; H, 2.13; Cl, 7.20; N, 10.87; F, 22.97.

### Referential Example 484

N-{2-[(2-Chloro-3-methoxyphenyl)(hydroxy)methyl]-4-methylphenyl}-2,2-dimethylpropanamide

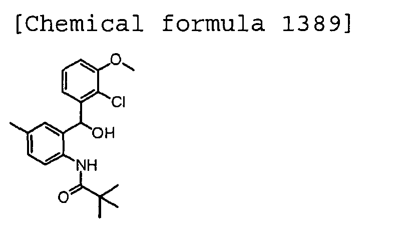

2,2-Dimethyl-N-(4-methylphenyl)propanamide (5.17 g) was dissolved in tetrahydrofuran (100 mL). While keeping the temperature at -40°C or less on a cooling bath, sec-butyl lithium (a 1.0M cyclohexane, hexane solution, 57 mL) was added dropwise to the resulting solution. The cooling bath was removed. While warming to room temperature, the resulting mixture was stirred for 2 hours. After cooling to -40°C or less, a tetrahydrofuran solution (15 mL) of 2-chloro-3-methoxybenzaldehyde (4.84 g) was added dropwise. While warming to room temperature, the reaction mixture was stirred for 80 minutes. To the reaction mixture was added a saturated aqueous solution of ammonium chloride and the mixture was extracted with ethyl acetate. The organic layer was washed with saturated brine and dried over anhydrous sodium sulfate. The solvent was distilled off under reduced pressure. The residue was subjected to silica gel column chromatography (30% ethyl acetate-hexane) to give the title compound (2.87 g).
MS (ESI) m/z: 344 (M-OH)⁺.
¹H-NMR (CDCl₃) δ: 1.24 (9H, s), 2.23 (3H, s), 3.91 (3H, s), 4.79 (1H, s), 6.12 (1H, s), 6.81 (1H, d, J = 2.0 Hz), 6.92 (1H, dd, J = 8.2, 1.3 Hz), 7.07 (1H, dd, J = 7.9, 1.3 Hz), 7.09-7.13 (1H, m), 7.23-7.27 (1H, m), 7.67 (1H, d, J = 8.1 Hz), 8.43 (1H, s).

### Referential Example 485

N-[2-(2-chloro-3-methoxybenzoyl)-4-methylphenyl]-2,2-dimethylpropanamide

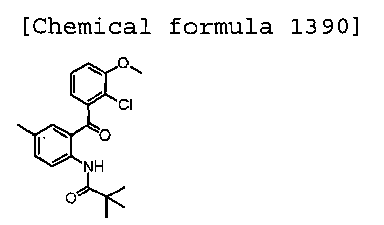

N-{2-[(2-chloro-3-methoxyphenyl)(hydroxy)methyl]-4-methylphenyl}-2,2-dimethylpropanamide (2.68 g) was dissolved in dichloromethane (60 mL). To the resulting solution was added manganese dioxide (7.73 g). The resulting mixture was heated under reflux for 18 hours. After cooling, the reaction mixture was filtered through celite and the filtrate was washed with dichloromethane and ether. The filtrate and washing were combined and distilled under reduced pressure. The residue was subjected to silica gel column chromatography (15% ethyl acetate-hexane) to give the title compound (1.37 g).
MS (ESI) m/z: 360 (M+H)⁺.
¹H-NMR (CDCl₃) δ: 1.39 (9H, s), 2.22 (3H, s), 3.97 (3H, s), 6.91 (1H, dd, J = 7.7, 1.3 Hz), 7.07 (1H, dd, J = 8.3, 1.2 Hz), 7.15 (1H, d, J = 2.2 Hz), 7.35 (1H, dd, J = 8.2, 7.7 Hz), 7.39 (1H, dd, J = 8.5, 2.2 Hz), 8.74 (1H, d, J = 8.8 Hz), 11.70 (1H, s).

### Referential Example 486

(2-Amino-5-methylphenyl)(2-chloro-3-methoxyphenyl)methanol

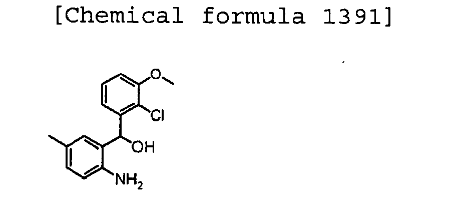

N-[2-(2-chloro-3-methoxybenzoyl)-4-methylphenyl]-2,2-dimethylpropanamide (1.37 g) was dissolved in ethanol (18 mL). To the resulting solution were added water (5 mL) and sodium hydroxide (2.0 g). The resulting mixture was heated under reflux for 12 hours. After cooling, the solvent was distilled off under reduced pressure. To the residue was added water and the resulting mixture was extracted with ethyl acetate. The organic layer was washed with saturated brine and dried over anhydrous sodium sulfate. The solvent was distilled off under reduced pressure to give (2-amino-5-methylphenyl)(2-chloro-3-methoxyphenyl)methanone (1.64 g). The compound thus obtained was dissolved in methanol (20 mL). While adding sodium borohydride (864 mg) in three portions, the resulting mixture was stirred at room temperature for 4 hours. The solvent was distilled off under reduced pressure. The residue was dissolved in ethyl acetate, followed by washing with water and saturated brine. After drying over anhydrous sodium sulfate, the solvent was distilled off under reduced pressure. The residue was subjected to silica gel column chromatography (40% ethyl acetate-hexane) to give the title compound (0.80 g).
MS (ESI) m/z: 260 (M-OH)⁺.
¹H-NMR (CDCl₃) δ: 2.17 (3H, s), 3.93 (3H, s), 6.23 (1H, s), 6.64 (1H, d, J = 7.8 Hz), 6.72 (1H, d, J = 1.5 Hz), 6.92-6.94 (2H, m), 7.13-7.15 (1H, m), 7.29 (1H, d, J = 7.8 Hz).

### Referential Example 487

(2-Chloro-3-methoxyphenyl){2-[(2,4-dimethoxybenzyl)amino]-5-methylphenyl}methanol

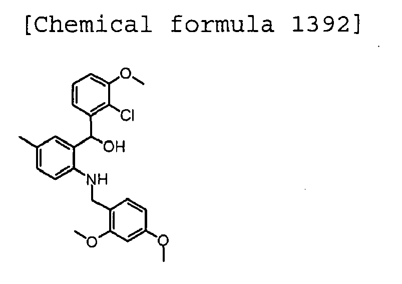

In a similar manner to that employed for the synthesis of {5-chloro-2-[(2,4-dimethoxybenzyl)amino]phenyl}[2-(trifluoromethyl)pyridin-3-yl]methanol, the title compound (1.26 g) was obtained from (2-amino-5-methylphenyl)(2-chloro-3-methoxyphenyl)methanol (0.80 g) and 2,4-dimethoxybenzaldehyde (527 mg).
MS (ESI) m/z: 428 (M+H)⁺.
¹H-NMR (CDCl₃) δ: 2.14 (3H, s), 3.78 (3H, s), 3.79 (3H, s), 3.92 (3H, s), 4.27 (2H, s), 6.22 (1H, s), 6.40 (1H, dd, J = 8.3, 2.4 Hz), 6.45 (1H, d, J = 2.4 Hz), 6.65-6.69 (2H, m), 6.92 (1H, dd, J = 8.3, 1.2 Hz), 6.97 (1H, dd, J = 8.2, 1.8 Hz), 7.11-7.15 (2H, m), 7.26 (1H, t, J = 8.1 Hz).

### Referential Example 488

Ethyl [trans-5-(2-chloro-3-methoxyphenyl)-1-(2,4-dimethoxybenzyl)-7-methyl-2-oxo-1,2,3,5-tetrahydro-4,1-benzoxazepin-3-yl]acetate

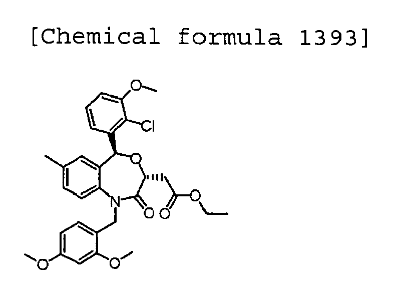

To an ethyl acetate (15 mL) solution of (2-Chloro-3-methoxyphenyl){2-[(2,4-dimethoxybenzyl)amino]-5-methylphenyl}methanol (1.26 g) was added sodium hydrogen carbonate (484 mg) under cooing with ice water. Then, an ethyl acetate solution (3 mL) of monoethyl chlorofumarate (0.515 g) was added dropwise. After stirring at room temperature for 30 minutes, the reaction mixture was washed with water and saturated brine and then dried over anhydrous sodium sulfate. The solvent was distilled off under reduced pressure. The residue was dissolved in ethanol (20 mL). To the resulting solution was added potassium carbonate (0.796 g) and the resulting mixture was stirred at room temperature for 40 hours. After concentration under reduced pressure, the residue was dissolved in ethyl acetate. The resulting solution was washed with water and saturated brine and dried over anhydrous sodium sulfate. The solvent was distilled off under reduced pressure. To the crystalline residue thus obtained were added small amounts of ethyl acetate and hexane and the mixture was stirred at room temperature for 15 minutes. The solid was collected by filtration and washed with hexane to give the title compound (1.23 g).
MS (ESI) m/z: 554 (M+H)⁺.
¹H-NMR (CDCl₃) δ: 1.25 (3H, t, J = 7.2 Hz), 2.14 (3H, s), 2.81 (1H, dd, J = 16.3, 6.1 Hz), 3.08 (1H, dd, J = 16.6, 7.6 Hz), 3.61 (3H, s), 3.75 (3H, s), 3.89 (3H, s), 4.11-4.17 (2H, m), 4.48 (1H, dd, J = 7.6, 6.1 Hz), 4.90 (1H, d, J = 14.6 Hz), 5.45 (1H, d, J = 14.6 Hz), 5.92 (1H, s), 6.20 (1H, d, J = 1.5 Hz), 6.34 (1H, d, J = 2.4 Hz), 6.38 (1H, dd, J = 8.4, 2.3 Hz), 6.93 (1H, dd, J = 7.4, 2.3 Hz), 7.13 (1H, dd, J = 8.0, 2.0 Hz), 7.27-7.35 (4H, m).

### Referential Example 489

Ethyl [trans-5-(2-chloro-3-methoxyphenyl)-7-methyl-2-oxo-1,2,3,5-tetrahydro-4,1-benzoxazepin-3-yl]acetate

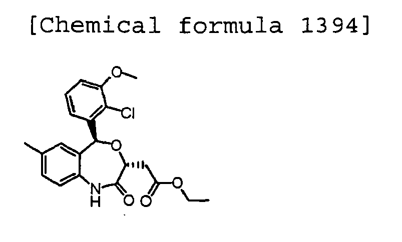

In a similar manner to that employed for the synthesis of ethyl {trans-7-chloro-2-oxo-5-[2-(trifluoromethyl)pyridin-3-yl]-1,2,3,5-tetrahydro-4,1-benzoxazepin-3-yl}acetate, the title compound (0.55 g) was obtained from ethyl [trans-5-(2-chloro-3-methoxyphenyl)-1-(2,4-dimethoxybenzyl)-7-methyl-2-oxo-1,2,3,5-tetrahydro-4,1-benzoxazepin-3-yl]acetate (1.23 g).
MS (ESI) m/z: 404 (M+H)⁺.
¹H-NMR (CDCl₃) δ: 1.23 (3H, t, J = 7.1 Hz), 2.19 (3H, s), 2.80 (1H, dd, J = 16.3, 6.6 Hz), 3.03 (1H, dd, J = 16.3, 6.6 Hz), 3.93 (3H, s), 4.09-4.16 (2H, m), 4.60 (1H, t, J = 6.6 Hz), 6.23 (1H, s), 6.40 (1H, s), 6.96 (1H, d, J = 8.0 Hz), 6.98 (1H, dd, J = 8.7, 1.8 Hz), 7.14 (1H, d, J = 9.5 Hz), 7.22-7.24 (1H, m), 7.34 (1H, t, J = 8.0 Hz), 7.70 (1H, s).

### Referential Example 490

Ethyl [trans-5-(2-chloro-3-methoxyphenyl)-7-methyl-2-thioxo-1,2,3,5-tetrahydro-4,1-benzoxazepin-3-yl]acetate

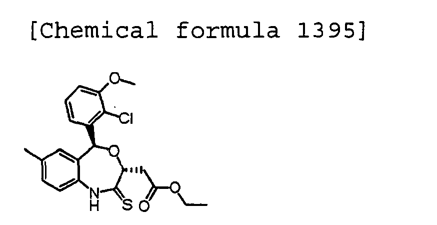

In a similar manner to that employed for the synthesis of ethyl {trans-7-chloro-2-thioxo-5-[2-(trifluoromethyl)pyridin-3-yl]-1,2,3,5-tetrahydro-4,1-benzoxazepin-3-yl}acetate, the title compound (0.47 g) was obtained from ethyl [trans-5-(2-chloro-3-methoxyphenyl)-7-methyl-2-oxo-1,2,3,5-tetrahydro-4,1-benzoxazepin-3-yl] acetate (0.55 g).
MS (ESI) m/z: 420 (M+H)⁺.
¹H-NMR (CDCl₃) δ: 1.25 (3H, t, J = 7.2 Hz), 2.20 (3H, s), 2.99 (1H, dd, J = 16.5, 6.7 Hz), 3.25 (1H, dd, J = 16.5, 6.5 Hz), 3.93 (3H, s), 4.14 (2H, q, J = 7.2 Hz), 4.69 (1H, t, J = 6.6 Hz), 6.14 (1H, s), 6.37 (1H, s), 6.98 (1H, dd, J = 8.2, 1.6 Hz), 7.02 (1H, d, J = 8.0 Hz), 7.17-7.20 (1H, m), 7.26-7.29 (1H, m), 7.34 (1H, t, J = 8.0 Hz), 9.62 (1H, s).

### Example 700

Ethyl [trans-6-(2-chloro-3-methoxyphenyl)-8-methyl-1-(trifluoromethyl)-4H,6H-[1,2,4]triazolo[4,3-a][4,1]benzoxazepin-4-yl]acetate

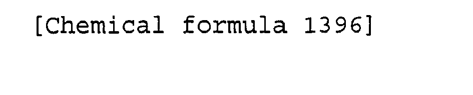

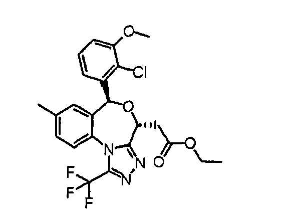

In a similar manner to that employed for the synthesis of ethyl {trans-8-chloro-1-(trifluoromethyl)-6-[2-(trifluoromethyl)pyridin-3-yl]-4H,6H-[1,2,4]triazolo[4,3-a][4,1]benzoxazepin-4-yl}acetate, the title compound (0.52 g) was obtained from ethyl [trans-5-(2-chloro-3-methoxyphenyl)-7-methyl-2-thioxo-1,2,3,5-tetrahydro-4,1-benzoxazepin-3-yl]acetate (0.47 g).
MS (ESI) m/z: 496 (M+H)⁺.
¹H-NMR (CDCl₃) δ: 1.29 (3H, t, J = 7.1 Hz), 2.29 (3H, s), 3.27 (1H, dd, J = 16.7, 7.4 Hz), 3.48 (1H, dd, J = 16.6, 6.3 Hz), 3.91 (3H, s), 4.17-4.23 (2H, m), 4.94 (1H, dd, J = 7.4, 6.2 Hz), 5.57 (1H, s), 6.53 (1H, s), 6.99 (1H, dd, J = 7.1, 2.7 Hz), 7.33-7.40 (3H, m), 7.46 (1H, d, J = 8.5 Hz).

### Example 701

Ethyl [(4R,6S)-6-(2-chloro-3-methoxyphenyl)-8-methyl-1-(trifluoromethyl)-4H,6H-[1,2,4]triazolo[4,3-a] [4,1]benzoxazepin-4-yl]acetate and ethyl [(4S,6R)-6-(2-chloro-3-methoxyphenyl)-8-methyl-1-(trifluoromethyl)-4H,6H-[1,2,4]triazolo[4,3-a][4,1]benzoxazepin-4-yl]acetate

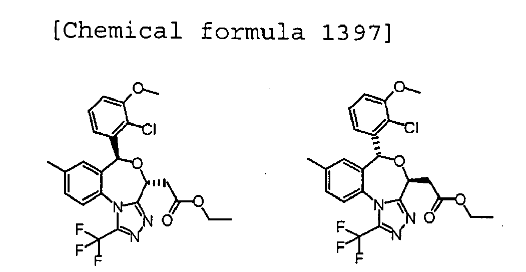

Ethyl [trans-6-(2-chloro-3-methoxyphenyl)-8-methyl-1-(trifluoromethyl)-4H,6H-[1,2,4]triazolo[4,3-a][4,1]benzoxazepin-4-yl]acetate (0.52 g) was optically resolved by Shimadzu HPLC (flow rate: 50 mL/min, eluting solvent: 20% 2-propanol-hexane) equipped with CHIRALPAK AD (Daicel Chemical, 50 × 500 mm), whereby ethyl [(4R,6S)-6-(2-chloro-3-methoxyphenyl)-8-methyl-1-(trifluoromethyl)-4H,6H-[1,2,4]triazolo[4,3-a] [4,1]benzoxazepin-4-yl]acetate(236 mg) was obtained from a fraction with a retention time of 36 minutes and ethyl [(4S,6R)-6-(2-chloro-3-methoxyphenyl)-8-methyl-1-(trifluoromethyl)-4H,6H-[1,2,4]triazolo[4,3-a] [4,1]benzoxazepin-4-yl]acetate (238 mg) was obtained from a fraction with a retention time of 43 minutes.

### Example 702

[(4R,6S)-6-(2-Chloro-3-methoxyphenyl)-8-methyl-1-(trifluoromethyl)-4H,6H-[1,2,4]triazolo[4,3-a][4,1]benzoxazepin-4-yl]acetic acid

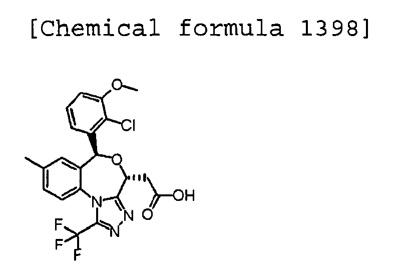

Ethyl [(4R,6S)-6-(2-chloro-3-methoxyphenyl)-8-methyl-1-(trifluoromethyl)-4H,6H-[1,2,4]triazolo[4,3-a] [4,1]benzoxazepin-4-yl]acetate (236 mg) was dissolved in dioxane (6 mL). To the resulting solution was added concentrated hydrochloric acid (3 mL). The resulting mixture was stirred at room temperature for 4 hours and then, at 60°C for 19 hours. After cooling, the reaction mixture was concentrated under reduced pressure. Water was added to the residue, followed by concentration under reduced pressure. To the residue was added water and the resulting mixture was vigorously stirred at room temperature for 3.5 hours. The solid was collected by filtration and washed with water to give the title compound (201 mg).
MS (ESI) m/z: 468 (M+H)⁺.
¹H-NMR (CDCl₃) δ: 2.29 (3H, s), 3.34 (1H, dd, J = 16.8, 7.1 Hz), 3.56 (1H, dd, J = 17.0, 6.5 Hz), 3.91 (3H, s), 4.90 (1H, t, J = 6.7 Hz), 5.58 (1H, s), 6.55 (1H, s), 6.99 (1H, dd, J = 6.6, 3.2 Hz), 7.34-7.41 (3H, m), 7.47 (1H, d, J = 8.0 Hz).
Elemental analysis for: C₂₁H₁₇ClN₃O₄F₃
Calculated: C, 53.91; H, 3.66; Cl, 7.58; N, 8.98; F, 12.18.
Found: C, 53.51; H, 3.73; Cl, 7.46; N, 8.70; F, 11.87.

### Example 703

[(4S,6R)-6-(2-Chloro-3-methoxyphenyl)-8-methyl-1-(trifluoromethyl)-4H,6H-[1,2,4]triazolo[4,3-a] [4,1]benzoxazepin-4-yl]acetic acid

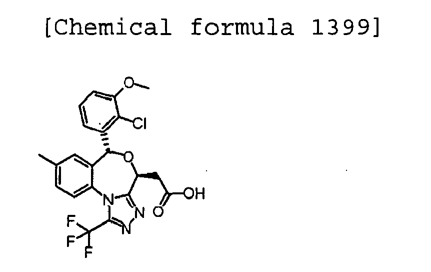

In a similar manner to that employed for the synthesis of [(4R,6S)-6-(2-chloro-3-methoxyphenyl)-8-methyl-1-(trifluoromethyl)-4H,6H-[1,2,4]triazolo[4,3-a] [4,1]benzoxazepin-4-yl]acetic acid, the title compound (202 mg) was obtained from ethyl [(4S,6R)-6-(2-chloro-3-methoxyphenyl)-8-methyl-1-(trifluoromethyl)-4H,6H-[1,2,4]triazolo[4,3-a][4,1]benzoxazepin-4-yl]acetate (238 mg) .
MS (ESI) m/z: 468 (M+H)⁺.
¹H-NMR (CDCl₃) δ: 2.29 (3H, s), 3.34 (1H, dd, J = 16.8, 7.1 Hz), 3.56 (1H, dd, J = 17.0, 6.5 Hz), 3.91 (3H, s), 4.90 (1H, t, J = 6.7 Hz), 5.58 (1H, s), 6.55 (1H, s), 6.99 (1H, dd, J = 6.6, 3.2 Hz), 7.34-7.41 (3H, m), 7.47 (1H, d, J = 8.0 Hz) .
Elemental analysis for: C₂₁H₁₇ClN₃O₄F₃·0.5H₂O
Calculated: C, 52.90; H, 3.80; Cl, 7.44; N, 8.81; F, 11.95.
Found: C, 53.03; H, 3.77; Cl, 8.08; N, 8.53; F, 11.89.

### Referential Example 491

4-(3-Methoxy-2-methylbenzoyl)morpholine

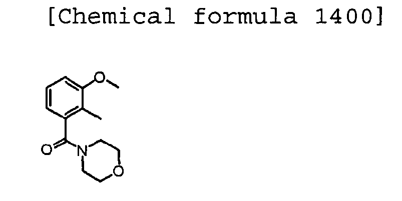

3-Methoxy-2-methylbenzoic acid (10.36 g) was dissolved in dichloromethane (250 mL). Under cooling with ice water, morpholine (6.0 mL), 1-hydroxybenzotriazole (1.69 g), and 1-ethyl-3-(3-dimethylaminopropyl)carbodiimide hydrochloride (15.5 g) were added. The resulting mixture was stirred at room temperature for 4 days. The reaction mixture was washed with water and dried over anhydrous sodium sulfate. The solvent was distilled off under reduced pressure. The residue was subjected to silica gel column chromatography (ethyl acetate:hexane=2:1) to give the title compound (14.98 g).
MS (ESI) m/z: 236 (M+H)⁺.
¹H-NMR (CDCl₃) δ: 2.16 (3H, s), 3.22-3.23 (2H, m), 3.55-3.58 (2H, m), 3.75-3.77 (2H, m), 3.81-3.82 (2H, m), 3.84 (1H, s), 6.77 (1H, d, J = 7.6 Hz), 6.84 (1H, d, J = 7.9 Hz), 7.20 (1H, t, J = 7.9 Hz).

### Referential Example 492

N-[2-(3-Methoxy-2-methylbenzoyl)-4-methylphenyl]-2,2-dimethylpropanamide

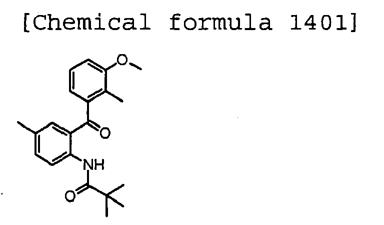

2,2-Dimethyl-N-(4-methylphenyl)propanamide (3.83 g) was dissolved in tetrahydrofuran (75 mL). While keeping the temperature at -40°C or less on a cooling bath, sec-butyl lithium (a 1.0 M cyclohexane, hexane solution, 42 mL) was added dropwise to the resulting solution. The cooling bath was removed. While warming to room temperature, the resulting mixture was stirred for 3 hours. After cooling to -40°C or less again, a tetrahydrofuran solution (20 mL) of 4-(3-methoxy-2-methylbenzoyl)morpholine (4.94 g) was added dropwise. While warming to room temperature, the resulting mixture was stirred for 80 minutes. To the reaction mixture was added saturated aqueous ammonium chloride, followed by extraction with ethyl acetate. The organic layer was washed with saturated brine and then, dried over anhydrous sodium sulfate. The solvent was distilled off under reduced pressure. The residue was subjected to silica gel column chromatography (10% ethyl acetate-hexane) to give the title compound (2.63 g).
MS (ESI) m/z: 340 (M+H)⁺.
¹H-NMR (CDCl₃) δ: 1.38 (9H, s), 2.11 (3H, s), 2.21 (3H, s), 3.89 (3H, s), 6.83 (1H, dd, J = 7.7, 0.9 Hz), 6.97 (1H, d, J = 7.8 Hz), 7.19 (1H, d, J = 2.2 Hz), 7.23 (1H, d, J = 7.6 Hz), 7.37 (1H, dd, J = 8.5, 2.2 Hz), 8.71 (1H, d, J = 8.5 Hz), 11.78 (1H, s).

### Referential Example 493

(2-Amino-5-methylphenyl)(3-methoxy-2-methylphenyl)methanol

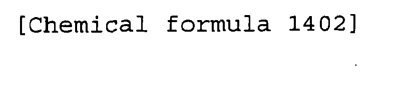

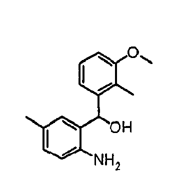

In a similar manner to that employed for the synthesis of (2-amino-5-methylphenyl)(2-chloro-3-methoxyphenyl)methanol, the title compound (0.71 g) was obtained from N-[2-(3-methoxy-2-methylbenzoyl)-4-methylphenyl]-2,2-dimethylpropanamide (2.63 g).
MS (ESI) m/z: 240 (M-OH)⁺.
¹H-NMR (CDCl₃) δ: 2.12 (3H, s), 2.15 (3H, s), 3.85 (3H, s), 6.04 (1H, s), 6.63-6.64 (2H, m), 6.86 (1H, d, J = 7.8 Hz), 6.92 (1H, dd, J = 7.9, 2.1 Hz), 7.07 (1H, d, J = 7.8 Hz), 7.22 (1H, t, J = 7.9 Hz).

### Referential Example 494

{2-[(2,4-Dimethoxybenzyl)amino]-5-methylphenyl}(3-methoxy-2-methylphenyl)methanol

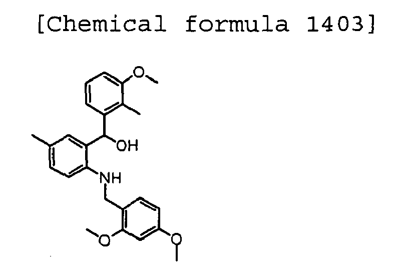

In a similar manner to that employed for the synthesis of {5-chloro-2-[(2,4-dimethoxybenzyl)amino]phenyl}[2-(trifluoromethyl)pyridin-3-yl]methanol, the title compound (0.56 g) was obtained from (2-amino-5-methylphenyl)(3-methoxy-2-methylphenyl)methanol (0.71 g).
MS (ESI) m/z: 408 (M+H)⁺.
¹H-NMR (CDCl₃) δ: 2.07 (3H, s), 2.13 (3H, s), 3.78 (3H, s), 3.79 (3H, s), 3.85 (3H, s), 4.26 (2H, s), 6.02 (1H, s), 6.40 (1H, dd, J = 8.3, 2.4 Hz), 6.45 (1H, d, J = 2.4 Hz), 6.60 (1H, d, J = 2.0 Hz), 6.67 (1H, d, J = 8.0 Hz), 6.85 (1H, d, J = 7.8 Hz), 6.96 (1H, dd, J = 8.3, 1.7 Hz), 7.08 (1H, d, J = 7.1 Hz), 7.12 (1H, d, J = 8.0 Hz), 7.21 (1H, t, J = 7.9 Hz).

### Referential Example 495

Ethyl [1-(2,4-dimethoxybenzyl)-5-(3-methoxy-2-methylphenyl)-7-methyl-2-oxo-1,2,3,5-tetrahydro-4,1-benzoxazepin-3-yl]acetate

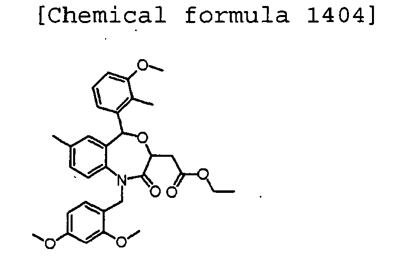

In a similar manner to that employed for the synthesis of ethyl [trans-5-(2-chloro-3-methoxyphenyl)-1-(2,4-dimethoxybenzyl)-7-methyl-2-oxo-1,2,3,5-tetrahydro-4,1-benzoxazepin-3-yl]acetate, the title compound (0.57 g) was obtained as a 14:1 trans isomer:cis isomer mixture from {2-[(2,4-dimethoxybenzyl)amino]-5-methylphenyl}(3-methoxy-2-methylphenyl)methanol (0.56 g).
MS (ESI) m/z: 534 (M+H)⁺.
trans Isomer
¹H-NMR (CDCl₃) δ: 1.25 (3H, t, J = 7.2 Hz), 1.39 (3H, s), 2.14 (3H, s), 2.80 (1H, dd, J = 16.3, 6.1 Hz), 3.09 (1H, dd, J = 16.3, 7.8 Hz), 3.58 (3H, s), 3.75 (3H, s), 3.81 (3H, s), 4.10-4.18 (2H, m), 4.47 (1H, dd, J = 7.8, 5.9 Hz), 4.91 (1H, d, J = 14.6 Hz), 5.44 (1H, d, J = 14.6 Hz), 5.66 (1H, s), 6.27 (1H, s), 6.32 (1H, d, J = 2.4 Hz), 6.40 (1H, dd, J = 8.5, 2.4 Hz), 6.82-6.85 (1H, m), 7.12-7.15 (1H, m), 7.18-7.21 (1H, m), 7.22 (1H, d, J = 7.6 Hz), 7.30 (1H, d, J = 8.3 Hz), 7.36 (1H, d, J = 8.3 Hz).

### Referential Example 496

Ethyl [5-(3-methoxy-2-methylphenyl)-7-methyl-2-oxo-1,2,3,5-tetrahydro-4,1-benzoxazepin-3-yl]acetate

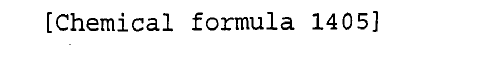

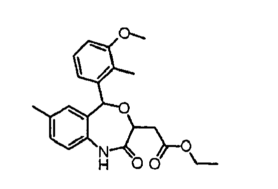

In a similar manner to that employed for the synthesis of ethyl {trans-7-chloro-2-oxo-5-[2-(trifluoromethyl)pyridin-3-yl]-1,2,3,5-tetrahydro-4,1-benzoxazepin-3-yl}acetate, the title compound (0.19 g) was obtained as a 3:1 trans isomer:cis isomer mixture from ethyl [1-(2,4-dimethoxybenzyl)-5-(3-methoxy-2-methoxyphenyl)-7-methyl-2-oxo-1,2,3,5-tetrahydro-4,1-benzoxazepin-3-yl]acetate (0.57 g).
MS (ESI) m/z: 384 (M+H)⁺.
trans Isomer
¹H-NMR (CDCl₃) δ: 1.22 (3H, t, J = 7.2 Hz), 1.98 (3H, s), 2.19 (3H, s), 2.79 (1H, dd, J = 16.3, 6.6 Hz), 3.00 (1H, dd, J = 16.3, 6.3 Hz), 3.85 (3H, s), 4.09-4.14 (2H, m), 4.55 (1H, t, J = 6.5 Hz), 6.07 (1H, s), 6.53 (1H, s), 6.88 (1H, d, J = 7.8 Hz), 6.92 (1H, d, J = 8.0 Hz), 7.00 (1H, d, J = 7.8 Hz), 7.11 (1H, d, J = 8.0 Hz), 7.21 (1H, t, J = 7.9 Hz), 7.63 (1H, s).

### Referential Example 497

Ethyl [5-(3-methoxy-2-methylphenyl)-7-methyl-2-thioxo-1,2,3,5-tetrahydro-4,1-benzoxazepin-3-yl]acetate

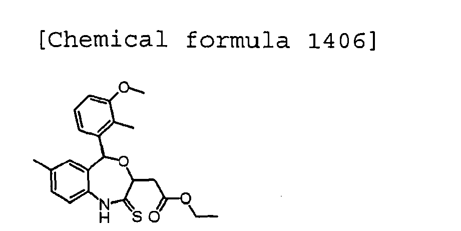

In a similar manner to that employed for the synthesis of ethyl {trans-7-chloro-2-thioxo-5-[2-(trifluoromethyl)pyridin-3-yl]-1,2,3,5-tetrahydro-4,1-benzoxazepin-3-yl}acetate, the title compound (0.14 g) was obtained as a 25:1 trans isomer:cis isomer mixture from ethyl [5-(3-methoxy-2-methylphenyll)-7-methyl-2-oxo-1,2,3,5-tetrahydro-4,1-benzoxazepin-3-yl]acetate (0.19 g).
MS (ESI) m/z: 400 (M+H)⁺.
trans Isomer
¹H-NMR (CDCl₃) δ: 1.24 (3H, t, J = 7.1 Hz), 1.90 (3H, s), 2.20 (3H, s), 2.96 (1H, dd, J = 16.2, 6.7 Hz), 3.25 (1H, dd, J = 16.4, 6.6 Hz), 3.85 (3H, s), 4.13 (2H, q, J = 7.2 Hz), 4.69 (1H, t, J = 6.6 Hz), 5.97 (1H, s), 6.48 (1H, s), 6.89 (1H, d, J = 7.8 Hz), 7.00 (1H, d, J = 8.1 Hz), 7.11 (1H, d, J = 7.6 Hz), 7.16 (1H, dd, J = 8.1, 1.7 Hz), 7.23 (1H, d, J = 8.1 Hz), 9.60 (1H, s).

### Example 704

Ethyl [trans-6-(3-methoxy-2-methylphenyl)-8-methyl-1-(trifluoromethyl)-4H,6H-[1,2,4]triazolo[4,3-a][4,1]benzoxazepin-4-yl]acetate

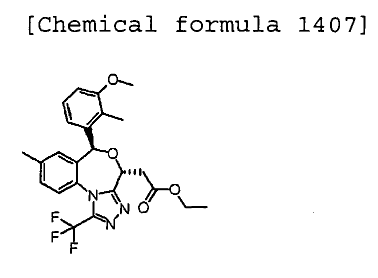

Ethyl [5-(3-methoxy-2-methylphenyl)-7-methyl-2-thioxo-1,2,3,5-tetrahydro-4,1-benzoxazepin-3-yl]acetate (0.466 g) was dissolved in tetrahydrofuran (10 mL). Under cooling with ice water, hydrazine hydrate (0.17 mL) was added and the resulting mixture was stirred at room temperature for one hour. The reaction mixture was diluted with saturated brine and extracted with ethyl acetate. The organic layer was washed twice with saturated brine and dried over anhydrous sodium sulfate. The solvent was distilled off under reduced pressure. The residue was dissolved in dichloroethane (15 mL). To the resulting solution was added trifluoroacetic anhydride (0.49 mL).
The resulting mixture was stirred at room temperature for one hour. After distilling off the solvent under reduced pressure, toluene (15 mL) and trifluoroacetic anhydride (0.49 mL) were added to the residue, followed by heating under reflux for 50 minutes. After cooling, the reaction mixture was concentrated under reduced pressure. The residue was dissolved in ethyl acetate. The resulting solution was washed with a saturated aqueous solution of sodium bicarbonate and saturated brine and dried over anhydrous sodium sulfate. The solvent was distilled off under reduced pressure. The residue was subjected to silica gel column chromatography (25% ethyl acetate-hexane) to give the title compound (526 mg).
MS (ESI) m/z : 476 (M+H)⁺.
¹H-NMR (CDCl₃) δ: 1.28 (3H, t, J = 7.2 Hz), 1.66 (3H, s), 2.28 (3H, s), 3.26 (1H, dd, J = 16.6, 7.3 Hz), 3.47 (1H, dd, J = 16.6, 6.3 Hz), 3.83 (3H, s), 4.15-4.23 (2H, m), 4.94 (1H, dd, J = 7.3, 6.3 Hz), 5.39 (1H, s), 6.60 (1H, s), 6.90 (1H, dd, J = 7.4, 2.1 Hz), 7.26-7.32 (2H, m), 7.34 (1H, d, J = 6.6 Hz), 7.46 (1H, d, J = 8.5 Hz).

### Example 705

Ethyl [(4S,6S)-6-(3-methoxy-2-methylphenyl)-8-methyl-1-(trifluoromethyl)-4H,6H-[1,2,4]triazolo[4,3-a][4,1]benzoxazepin-4-yl]acetate, and ethyl [(4R,6R)-6-(3-methoxy-2-methylphenyl)-8-methyl-1-(trifluoromethyl)-4H,6H-[1,2,4]triazolo[4,3-a][4,1]benzoxazepin-4-yl]acetate

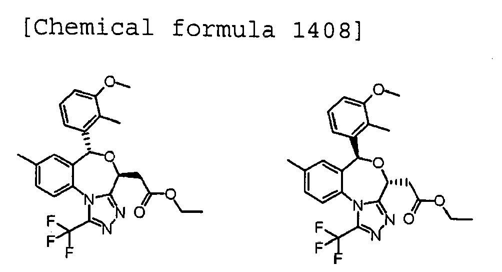

Ethyl [trans-6-(3-methoxy-2-methylphenyl)-8-methyl-1-(trifluoromethyl)-4H,6H-[1,2,4]triazolo[4,3-a] [4,1]benzoxazepin-4-yl]acetate (0.524 g) was optically resolved by Shimadzu HPLC (flow rate: 50 mL/min, eluting solvent: 10% 2-propanol-hexane) equipped with CHIRALPAK OD (Daicel Chemical, 50 × 500 mm) to give ethyl [(4S,6S)-6-(3-methoxy-2-methylphenyl)-8-methyl-1-(trifluoromethyl)-4H,6H-[1,2,4]triazolo[4,3-a][4,1]benzoxazepin-4-yl]acetate (0.23 g) from a fraction with a retention time of 44 minutes and ethyl [(4R,6R)-6-(3-methoxy-2-methylphenyl)-8-methyl-1-(trifluoromethyl)-4H,6H-[1,2,4]triazolo[4,3-a] [4,1]benzoxazepin-4-yl]acetate (0.24 g) from a fraction with a retention time of 62 minutes.

### Example 706

[(4S,6S)-6-(3-Methoxy-2-methylphenyl)-8-methyl-1-(trifluoromethyl)-4H,6H-[1,2,4]triazolo[4,3-a][4,1]benzoxazepin-4-yl]acetic acid

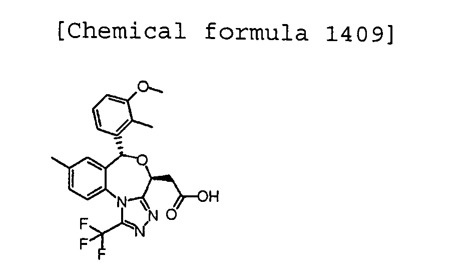

Ethyl [(4S,6S)-6-(3-methoxy-2-methylphenyl)-8-methyl-1-(trifluoromethyl)-4H,6H-[1,2,4]triazolo[4,3-a][4,1]benzoxazepin-4-yl]acetate (0.23 g) was dissolved in dioxane (6 mL). To the resulting solution were added water (3 mL) and concentrated hydrochloric acid (3 mL). The resulting mixture was stirred at 60°C for 13.5 hours and at room temperature for 27 hours. The reaction mixture was concentrated under reduced pressure. Water was added to the residue and the resulting mixture was concentrated to dryness under reduced pressure. Water was added to the residue and the resulting mixture was stirred vigorously at room temperature for 4 hours. The solid was collected by filtration and washed with water to give the title compound (200 mg).
MS (ESI) m/z: 448 (M+H)⁺.
¹H-NMR (CDCl₃) δ: 1.65 (3H, s), 2.29 (3H, s), 3.32 (1H, dd, J = 17.0, 7.0 Hz), 3.55 (1H, dd, J = 17.1, 6.6 Hz), 3.83 (3H, s), 4.91 (1H, t, J = 6.7 Hz), 5.39 (1H, s), 6.61 (1H, s), 6.90 (1H, dd, J = 7.1, 2.4 Hz), 7.27-7.33 (2H, m), 7.35 (1H, d, J = 8.3 Hz), 7.47 (1H, d, J = 8.3 Hz).
Elemental analysis for: C₂₂H₂₀N₃O₄F₃·0.125C₄H₈O₂·0.33H₂O
Calculated: C, 58.19; H, 4.70; N, 9.05; F, 12.27.
Found: C, 58.25; H, 4.56; N, 8.97; F, 12.25

### Example 707

[(4R,6R)-6-(3-Methoxy-2-methylphenyl)-8-methyl-1-(trifluoromethyl)-4H,6H-[1,2,4]triazolo[4,3-a] [4,1] benzoxazepin-4-yl] acetic acid

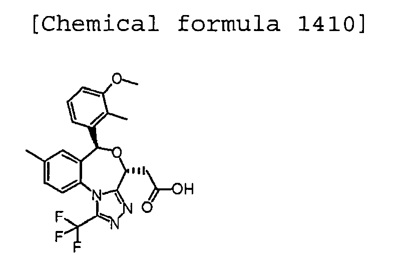

In a similar manner to that employed for the synthesis of [(4S,6S)-6-(3-methoxy-2-methylphenyl)-8-methyl-1-(trifluoromethyl)-4H,6H-[1,2,4]triazolo[4,3-a][4,1]benzoxazepin-4-yl]acetic acid, the title compound (205 mg) was obtained from ethyl [(4R,6R)-6-(3-methoxy-2-methylphenyl)-8-methyl-1-(trifluoromethyl)-4H,6H-[1,2,4]triazolo[4,3-a][4,1]benzoxazepin-4-yl]acetate (0.24 g).
MS (ESI) m/z: 448 (M+H)⁺. ¹H-NMR (CDCl₃) δ: 1.65 (3H, s), 2.29 (3H, s), 3.32 (1H, dd, J = 17.0, 7.0 Hz), 3.55 (1H, dd, J = 17.1, 6.6 Hz), 3.83 (3H, s), 4.91 (1H, t, J = 6.7 Hz), 5.39 (1H, s), 6.61 (1H, s), 6.90 (1H, dd, J = 7.1, 2.4 Hz), 7.27-7.33 (2H, m), 7.35 (1H, d, J = 8.3 Hz), 7.47 (1H, d, J = 8.3 Hz).
Elemental analysis for: C₂₂H₂₀N₃O₄F₃·0.125C₄H₈O₂·0.33H₂O Calculated: C, 58.19; H, 4.70; N, 9.05; F, 12.27.
Found: C, 58.25; H, 4.56; N, 8.97; F, 12.25.

### Referential Example 498

4-(2-Methoxy-3-methylbenzoyl)morpholine

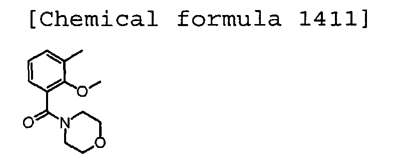

In a similar manner to that employed for the synthesis of 4-(3-methoxy-2-methylbenzoyl)morpholine, the title compound (3.77 g) was obtained from 2-methoxy-3-methylbenzoic acid (3.0 g).
MS (ESI) m/z: 236 (M+H)⁺.
¹H-NMR (CDCl₃) δ: 2.29 (3H, s), 3.19-3.22 (1H, m), 3.30-3.34 (1H, m), 3.56-3.59 (1H, m), 3.64-3.69 (1H, m), 3.73-3.81 (6H, m), 3.84-3.90 (1H, m), 7.05 (1H, t, J = 7.5 Hz), 7.10 (1H, dd, J = 7.5, 2.0 Hz), 7.20-7.23 (1H, m).

### Referential Example 499

N-[4-Chloro-2-(2-methoxy-3-methylbenzoyl)phenyl]-2,2-dimethylpropanamide

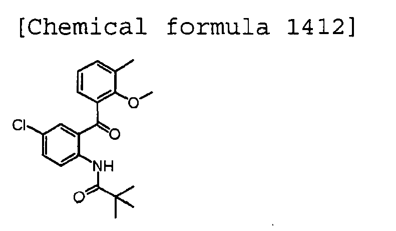

In a similar manner to that employed for the synthesis of N-[2-(3-methoxy-2-methylbenzoyl)-4-methylphenyl]-2,2-dimethylpropanamide, the title compound (2.84 g) was obtained from N-(4-chlorophenyl)-2,2-dimethylpropanamide (2.12 g) and 4-(2-methoxy-3-methylbenzoyl)morpholine (2.59 g).
MS (ESI) m/z: 360 (M+H)+..
¹H-NMR (CDCl₃) δ: 1.38 (9H, s), 2.35 (3H, s), 3.65 (3H, s), 7.10-7.13 (2H, m), 7.36 (1H, td, J = 4.6, 0.8 Hz), 7.42 (1H, d, J = 2.4 Hz), 7.51 (1H, dd, J = 9.0, 2.7 Hz), 8.80 (1H, d, J = 9.0 Hz), 11.69 (1H, br s).

### Referential Example 500

(2-Amino-5-chlorophenyl)(2-methoxy-3-methylphenyl)methanol

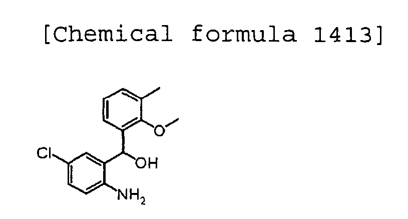

In a similar manner to that employed for the synthesis of (2-amino-5-methylphenyl)(3-methoxy-2-methylphenyl)methanol, the title compound (1.955 g) was obtained from N-[4-chloro-2-(2-methoxy-3-methylbenzoyl)phenyl]-2,2-dimethylpropanamide (2.84 g).
MS (ESI) m/z: 260 (M-OH)⁺.
¹H-NMR (CDCl₃) δ: 2.17 (3H, s), 2.33 (3H, s), 3.76 (3H, s), 6.07 (1H, s), 6.59 (1H, d, J = 8.3 Hz), 7.02-7.06 (2H, m), 7.10 (1H, dd, J = 7.4, 1.7 Hz), 7.13 (1H, d, J = 2.4 Hz), 7.16 (1H, dd, J = 7.4, 1.0 Hz).

### Referential Example 501

### {5-Chloro-2-[(2,4-dimethoxybenzyl)amino]phenyl}(2-methoxy-3-methylphenyl)methanol

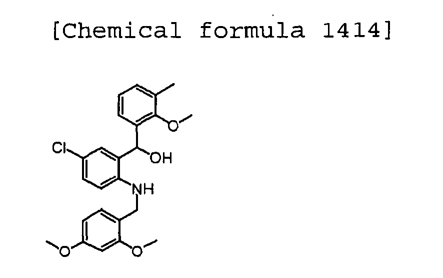

In a similar manner to that employed for the synthesis of {5-chloro-2-[(2,4-dimethoxybenzyl)amino]phenyl}[2-(trifluoromethyl)pyridin-3-yl]methanol, the title compound (3.35 g) was obtained from (2-amino-5-chlorophenyl)(2-methoxy-3-methylphenyl)methanol (1.95 g) and 2,4-dimethoxybenzaldehyde (1.28 g).
MS (ESI) m/z: 428 (M+H)⁺.
¹H-NMR (CDCl₃) δ: 2.31 (3H s), 3.67 (3H, s), 3.76 (3H, s), 3.78 (3H, s), 4.22 (2H, s), 6.06 (1H, s), 6.37 (1H, dd, J = 8.3, 2.4 Hz), 6.43 (1H, d, J = 2.4 Hz), 6.58 (1H, d, J = 9.5 Hz), 6.99-7.10 (5H, m), 7.15 (1H, d, J = 8.5 Hz).

### Referential Example 502

Ethyl [7-chloro-1-(2,4-dimethoxybenzyl)-5-(2-methoxy-3-methylphenyl)-2-oxo-1,2,3,5-tetrahydro-4,1-benzoxazepin-3-yl] acetate

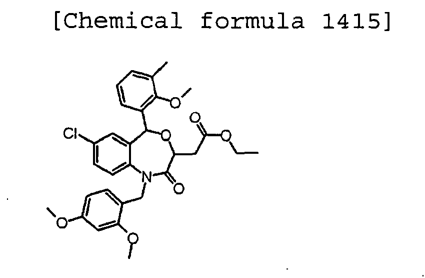

In a similar manner to that employed for the synthesis of ethyl [trans-5-(2-chloro-3-methoxyphenyl)-1-(2,4-dimethoxybenzyl)-7-methyl-2-oxo-1,2,3,5-tetrahydro-4,1-benzoxazepin-3-yl]acetate, the title compound (3.65 g) was obtained as a 6:1 trans isomer:cis isomer mixture from {5-chloro-2-[(2,4-dimethoxybenzyl)amino]phenyl}(2-methoxy-3-methylphenyl)methanol (3.55 g).
MS (ESI) m/z: 554 (M+H)⁺.
trans isomer
¹H-NMR (CDCl₃) δ: 1.25 (3H, t, J = 7.1 Hz), 2.24 (3H, s), 2.79 (1H, dd, J = 16.6, 5.9 Hz), 3.03 (3H, s), 3.09 (1H, dd, J = 16.6, 7.8 Hz), 3.68 (3H, s), 3.77 (3H, s), 4.14 (2H, q, J = 7.0 Hz), 4.50 (1H, dd, J = 7.6, 5.9 Hz), 4.90 (1H, d, J = 15.1 Hz), 5.45 (1H, d, J = 15.1 Hz), 5.97 (1H, s), 6.40-6.44 (2H, m), 6.57 (1H, d, J = 2.2 Hz), 7.12 (1H, t, J = 7.6 Hz), 7.16-7.20 (1H, m), 7.25-7.34 (5H, m), 7.39 (1H, dd, J = 7.6, 1.7 Hz).

### Referential Example 503

Ethyl [7-chloro-5-(2-methoxy-3-methylphenyl)-2-oxo-1,2,3,5-tetrahydro-4,1-benzoxazepin-3-yl]acetate

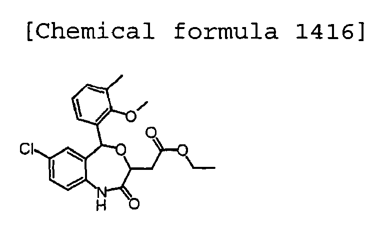

Ethyl [7-chloro-1-(2,4-dimethoxybenzyl)-5-(2-methoxy-3-methylphenyl)-2-oxo-1,2,3,5-tetrahydro-4,1-benzoxazepin-3-yl]acetate (3.65 g) was dissolved in acetone (100 mL). Under cooling with ice water, an aqueous solution (25 mL) of ceric ammonium nitrate (13.0 g) was added and the resulting mixture was stirred at room temperature for 140 minutes. To the reaction mixture were added a saturated aqueous solution of sodium bicarbonate and water and the resulting mixture was filtered through celite. The filtrate was extracted with ethyl acetate. The organic layer was washed with a saturated aqueous solution of sodium bicarbonate and saturated brine, and then dried over anhydrous sodium sulfate. The solvent was distilled off under reduced pressure. The residue was subjected to silica gel column chromatography (40% ethyl acetate-hexane) to give a solid. After stirring the solid in hexane at room temperature for 2 hours, the solid was collected by filtration to give the title compound (1.89 g) as a 9:1 trans isomer:cis isomer mixture.
MS (ESI) m/z: 404 (M+H)⁺.
trans Isomer
¹H-NMR (CDCl₃) δ: 1.21 (3H, t, J = 7.2 Hz), 2.31 (3H, s), 2.78 (1H, dd, J = 16.4, 6.5 Hz), 3.02 (1H, dd, J = 16.4, 6.5 Hz), 3.54 (3H, s), 4.07-4.13 (2H, m), 4.65 (1H, t, J = 6.6 Hz), 6.23 (1H, s), 6.69 (1H, d, J = 2.2 Hz), 6.99 (1H, d, J = 8.5 Hz), 7.13 (1H, t, J = 7.6 Hz), 7.22-7.25 (1H, m), 7.28 (1H, dd, J = 8.4, 2.3 Hz), 7.30-7.33 (1H, m), 7.82 (1H, s).

### Referential Example 504

Ethyl [trans-7-chloro-5-(2-methoxy-3-methylphenyl)-2-thioxo-1,2,3,5-tetrahydro-4,1-benzoxazepin-3-yl]acetate

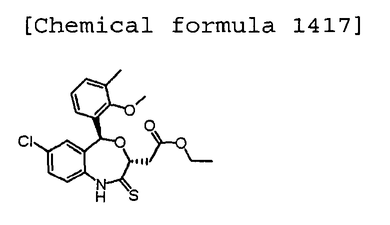

In a similar manner to that employed for the synthesis of ethyl {trans-7-chloro-2-thioxo-5-[2-(trifluoromethyl)pyridin-3-yl]-1,2,3,5-tetrahydro-4,1-benzoxazepin-3-yl}acetate, a solid obtained from ethyl [7-chloro-5-(2-methoxy-3-methylphenyl)-2-oxo-1,2,3,5-tetrahydro-4,1-benzoxazepin-3-yl]acetate (1.88 g) was stirred in hexane containing a small amount of ethyl acetate and then collected by filtration to give the title compound (1.47 g).
MS (ESI) m/z: 420 (M+H)⁺.
¹H-NMR (CDCl₃) δ: 1.24 (3H, t, J = 7.2 Hz), 2.30 (3H, s), 2.94 (1H, dd, J = 16.5, 6.5 Hz), 3.25 (1H, dd, J = 16.6, 6.8 Hz), 3.51 (3H, s), 4.12 (2H, q, J = 7.1 Hz), 4.72 (1H, t, J = 6.6 Hz), 6.14 (1H, s), 6.68 (1H, d, J = 2.4 Hz), 7.06 (1H, d, J = 8.5 Hz), 7.15 (1H, t, J = 7.6 Hz), 7.24 (1H, d, J = 7.3 Hz), 7.33-7.38 (2H, m), 9.54 (1H, br s).

### Example 708

Ethyl [trans-8-chloro-6-(2-methoxy-3-methylphenyl)-1-(trifluoromethyl)-4H,6H-[1,2,4]triazolo[4,3-a] [4,1]benzoxazepin-4-yl]acetate

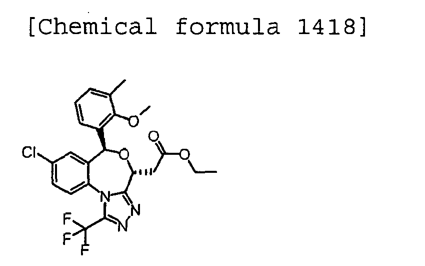

Ethyl [trans-7-chloro-5-(2-methoxy-3-methylphenyl)-2-thioxo-1,2,3,5-tetrahydro-4,1-benzoxazepin-3-yl]acetate (1.47 g) was dissolved in tetrahydrofuran (25 mL). Under cooling with ice water, hydrazine hydrate (0.51 mL) was added and the resulting mixture was stirred at room temperature for one hour. The reaction mixture was diluted with saturated brine and extracted with ethyl acetate. The organic layer was washed twice with saturated brine and then, dried over anhydrous sodium sulfate. The solvent was distilled off under reduced pressure. The residue was dissolved in dichloroethane (25 mL). Under cooling with ice water, trifluoroacetic anhydride (1.46 mL) was added and the resulting mixture was stirred at room temperature for 16 hours. Trifluoroacetic anhydride (1.5 mL) was added again and the resulting mixture was heated under reflux for 1.5 hours. After cooling, the reaction mixture was concentrated under reduced pressure. Toluene was added to the residue and the resulting mixture was concentrated under reduced pressure. The residue was subjected to silica gel column chromatography (20% ethyl acetate-hexane) to give the title compound (1.57 g).
MS (ESI) m/z: 496 (M+H)⁺.
¹H-NMR (CDCl₃) δ: 1.28 (3H, t, J = 7.1 Hz), 2.26 (3H, s), 3.23 (1H, dd, J = 16.6, 7.1 Hz), 3.28 (3H, s), 3.49 (1H, dd, J = 16.6, 6.6 Hz), 4.16-4.22 (2H, m), 4.94 (1H, t, J = 6.8 Hz), 5.56 (1H, s), 6.88 (1H, t, J = 1.3 Hz), 7.18 (1H, t, J = 7.6 Hz), 7.24 (1H, dd, J = 7.4, 1.1 Hz), 7.46 (1H, dd, J = 7.6, 1.7 Hz), 7.53 (1H, d, J = 1.2 Hz).

### Example 709

Ethyl [(4S,6R)-8-chloro-6-(2-methoxy-3-methylphenyl)-1-(trifluoromethyl)-4H,6H-[1,2,4]triazolo[4,3-a][4,1]benzoxazepin-4-yl]acetate and ethyl [(4R,6S)-8-chloro-6-(2-methoxy-3-methylphenyl)-1-(trifluoromethyl)-4H,6H-[1,2,4]triazolo[4,3-a][4,1]benzoxazepin-4-yl]acetate

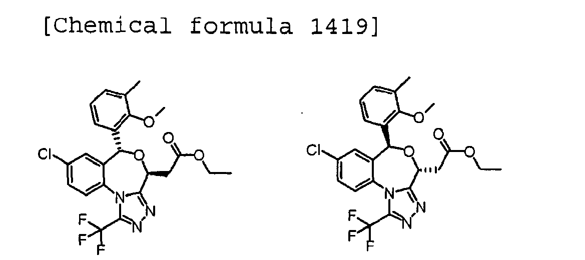

Ethyl [trans-8-chloro-6-(2-methoxy-3-methylphenyl)-1-(trifluoromethyl)-4H,6H-[1,2,4]triazolo[4,3-a][4,1]benzoxazepin-4-yl]acetate(1.57 g) was optically resolved by Shimadzu HPLC (flow rate: 50 mL/min, eluting solvent: 10% 2-propanol-hexane) equipped with CHIRALPAK AD (Daicel Chemical, 50 × 500 mm) to give ethyl [(4S,6R)-8-chloro-6-(2-methoxy-3-methylphenyl)-1-(trifluoromethyl)-4H,6H-[1,2,4]triazolo[4,3-a][4,1]benzoxazepin-4-yl]acetate (0.77 g) from a fraction with a retention time of 39 minutes and ethyl [(4R,6S)-8-chloro-6-(2-methoxy-3-methylphenyl)-1-(trifluoromethyl)-4H,6H-[1,2,4]triazolo[4,3-a][4,1]benzoxazepin-4-yl]acetate (0.77 g) from a fraction with a retention time of 48 minutes.

### Example 710

[(4S,6R)-8-Chloro-6-(2-methoxy-3-methylphenyl)-1-(trifluoromethyl)-4H,6H-[1,2,4]triazolo[4,3-a][4,1]benzoxazepin-4-yl]acetic acid

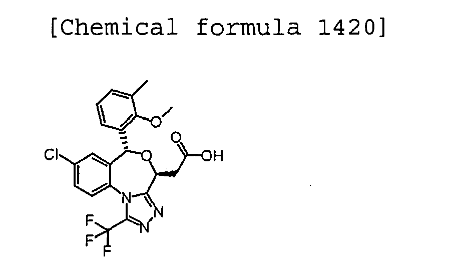

Ethyl [(4S,6R)-8-chloro-6-(2-methoxy-3-methylphenyl)-1-(trifluoromethyl)-4H,6H-[1,2,4]triazolo[4,3-a][4,1]benzoxazepin-4-yl]acetate (0.77 g) was dissolved in dioxane (18 mL). To the resulting solution was added concentrated hydrochloric acid (9 mL). The resulting mixture was stirred at 60°C for 22 hours. The reaction mixture was concentrated under reduced pressure. To the residue was added water and the resulting mixture was concentrated under reduced pressure. Water was added to the residue, followed by stirring overnight at room temperature. The solid was collected by filtration and washed with water to give a pale yellow solid (0.798 g). The solid was subjected to silica gel column chromatography (chloroform:methanol=7:3) to give the title compound (90 mg) .
MS (ESI) m/z: 468 (M+H)⁺.
¹H-NMR (CD₃OD) δ: 2.27 (3H, s), 3.15 (1H, dd, J = 16.5, 7.4 Hz), 3.30 (3H, s), 3.35 (1H, dd, J = 15.1, 5.1 Hz), 4.92 (1H, t, J = 7.0 Hz), 5.56 (1H, s), 6.81 (1H, d, J = 2.2 Hz), 7.20 (1H, t, J = 7.6 Hz), 7.29 (1H, d, J = 6.8 Hz), 7.52 (1H, d, J = 6.6 Hz), 7.70 (1H, dd, J = 8.5, 2.2 Hz), 7.73 (1H, d, J = 8.5 Hz).
Elemental analysis for: C₂₁H₁₇ClN₃O₄F₃·0.33H₂O
Calculated: C, 53.23; H, 3.76; Cl, 7.48; N, 8.87; F, 12.03. Found: C, 53.45; H, 3.64; Cl, 7.19; N, 9.39; F, 11.61.

### Example 711

[(4R,6S)-8-Chloro-6-(2-methoxy-3-methylphenyl)-1-(trifluoromethyl)-4H,6H-[1,2,4]triazolo[4,3-a] [4,1] benzoxazepin-4-yl] acetic acid

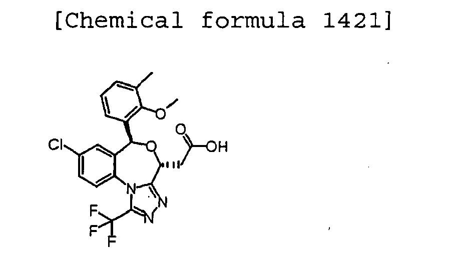

In a similar manner to that employed for the synthesis of [(4S,6R)-8-chloro-6-(2-methoxy-3-methylphenyl)-1-(trifluoromethyl)-4H,6H-[1,2,4]triazolo[4,3-a][4,1]benzoxazepin-4-yl]acetic acid, the title compound (248 mg) was obtained from ethyl [(4R,6S)-8-chloro-6-(2-methoxy-3-methylphenyl)-1-(trifluoromethyl)-4H,6H-[1,2,4]triazolo[4,3-a][4,1]benzoxazepin-4-yl]acetate (0.77 g).
MS (ESI) m/z: 468 (M+H)⁺.
¹H-NMR (CD₃OD) δ: 2.27 (3H, s), 3.16 (1H, dd, J = 16.6, 7.3 Hz), 3.30 (3H, s), 3.35 (1H, dd, J = 17.1, 6.8 Hz), 4.92 (1H, t, J = 6.8 Hz), 5.57 (1H, s), 6.81 (1H, d, J = 2.2 Hz), 7.19 (1H, t, J = 7.6 Hz), 7.29 (1H, d, J = 7.6 Hz), 7.52 (1H, d, J = 7.6 Hz), 7.70 (1H, dd, J = 8.5, 2.2 Hz), 7.74 (1H, d, J = 8.5 Hz).
Elemental analysis for: C₂₁H₁₇ClN₃O₄F₃·0.33H₂O
Calculated: C, 53.23; H, 3.76; Cl, 7.48; N, 8.87; F, 12.03.
Found: C, 53.30; H, 3.59; Cl, 7.31; N, 8.78; F, 11.91.

### Referential Example 505

4-(2-Methoxy-4-methylbenzoyl)morpholine

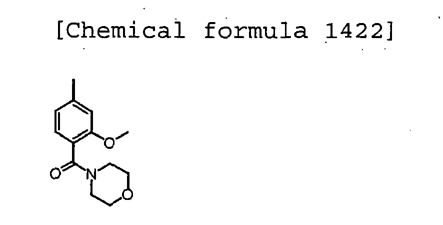

In a similar manner to that employed for the synthesis of 4-(3-methoxy-2-methylbenzoyl)morpholine, the title compound (2.92 g) was obtained from 2-methoxy-4-methylbenzoic acid (2.50 g).
MS (ESI) m/z: 236 (M+H)⁺.
¹H-NMR (CDCl₃) δ: 2.36 (3H, s), 3.20-3.32 (2H, m), 3.53-3.67 (2H, m), 3.77-3.83 (7H, m), 6.72 (1H, s), 6.81 (1H, d, J = 7.6 Hz), 7.13 (1H, d, J = 7.6 Hz).

### Referential Example 506

N-[4-Chloro-2-(2-methoxy-4-methylbenzoyl)phenyl]-2,2-dimethylpropanamide

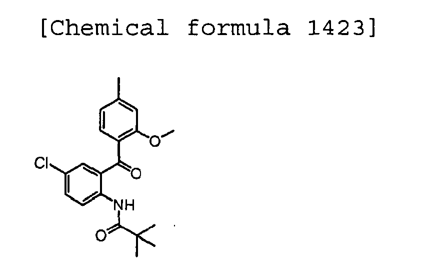

In a similar manner to that employed for the synthesis of N-[2-(3-methoxy-2-methylbenzoyl)-4-methylphenyl]-2,2-dimethylpropanamide, the title compound (2.78 g) was obtained from N-(4-chlorophenyl)-2,2-dimethylpropanamide(2.12 g) and 4-(2-methoxy-4-methylbenzoyl)morpholine (2.59 g).
MS (ESI) m/z: 360 (M+H)⁺.
¹H-NMR (CDCl₃) δ: 1.37 (9H, s), 2.44 (3H, s), 3.75 (3H, s), 6.81 (1H, s), 6.87-6.88 (1H, m), 7.21 (1H, d, J = 7.8 Hz), 7.26 (1H, s), 7.42 (1H, d, J = 2.4 Hz), 7.47 (1H, dd, J = 9.0, 2.4 Hz), 8.76 (1H, d, J = 9.0 Hz).

### Referential Example 507

(2-Amino-5-chlorophenyl)(2-methoxy-4-methylphenyl)methanol

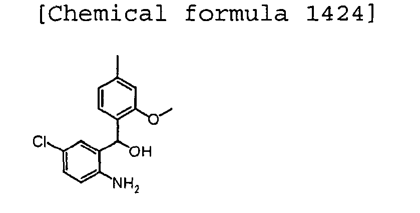

In a similar manner to that employed for the synthesis of (2-amino-5-methylphenyl)(3-methoxy-2-methylphenyl)methanol, the title compound (1.95 g) was obtained from N-[4-chloro-2-(2-methoxy-4-methylbenzoyl)phenyl]-2,2-dimethylpropanamide (2.78 g).
MS (ESI) m/z: 260 (M-OH)⁺.
¹H-NMR (CDCl₃) δ: 2.36 (3H, s), 3.87 (3H, s), 5.96 (1H, s), 6.60 (1H, d, J = 8.3 Hz), 6.76-6.77 (2H, m), 6.98 (1H, d, J = 2.9 Hz), 7.00 (1H, d, J = 8.0 Hz), 7.04.(1H, dd, J = 8.3, 2.4 Hz).

### Referential Example 508

{5-Chloro-2-[(2,4-dimethoxybenzyl)amino]phenyl}(2-methoxy-4-methylphenyl)methanol

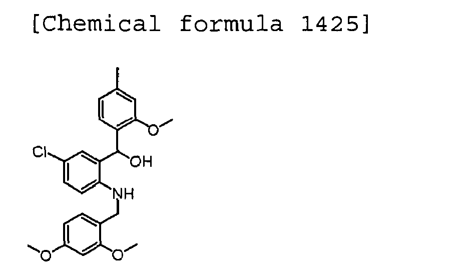

In a similar manner to that employed for the synthesis of {5-chloro-2-[(2,4-dimethoxybenzyl)amino]phenyl}[2-(trifluoromethyl)pyridin-3-yl]methanol, the title compound (2.67 g) was obtained from (2-amino-5-chlorophenyl)(2-methoxy-4-methylphenyl)methanol (1.95 g) and 2,4-dimethylbenzaldehyde (1.28 g).
MS (ESI) m/z: 428 (M+H)⁺.
¹H-NMR (CDCl₃) δ: 2.36 (3H, s), 3.18 (1H, br s), 3.76 (3H, s), 3.78 (3H, s), 3.79 (3H, s), 4.23 (2H, s), 5.22 (1H, br s), 5.96 (1H, s), 6.39 (1H, dd, J = 8.3, 2.4 Hz), 6.44 (1H, d, J = 2.4 Hz), 6.58 (1H, d, J = 8.5 Hz), 6.74 (1H, s), 6.75 (1H, d, J = 6.8 Hz), 6.98 (1H, d, J = 2.4 Hz), 7.01 (1H, d, J = 8.1 Hz), 7.07 (1H, dd, J = 8.7, 2.6 Hz), 7.08 (1H, d, J = 8.3 Hz).

### Referential Example 509

Ethyl [7-chloro-1-(2,4-dimethoxybenzyl)-5-(2-methoxy-4-methylphenyl)-2-oxo-1,2,3,5-tetrahydro-4,1-benzoxazepin-3-yl]acetate

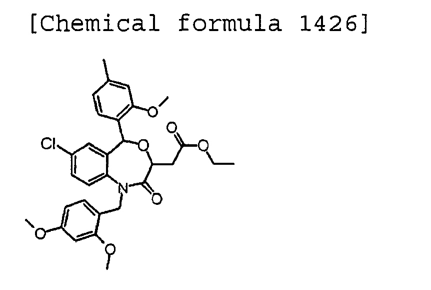

In a similar manner to that employed for the synthesis of ethyl [trans-5-(2-chloro-3-methoxyphenyl)-1-(2,4-dimethoxybenzyl)-7-methyl-2-oxo-1,2,3,5-tetrahydro-4,1-benzoxazepin-3-yl]acetate, the title compound (2.52 g) was obtained as a 9:1 trans isomer:cis isomer mixture from {5-chloro-2-[(2,4-dimethoxybenzyl)amino]phenyl}(2-methoxy-4-methylphenyl)methanol (2.65 g).
MS (ESI) m/z: 554 (M+H)⁺.
trans Isomer
¹H-NMR (CDCl₃) δ: 1.25 (3H, t, J = 7.2 Hz), 2.38 (3H, s), 2.78 (1H, dd, J = 16.6, 5.9 Hz), 3.09 (1H, dd, J = 16.5, 7.9 Hz), 3.48 (3H, s), 3.66 (3H, s), 3.77 (3H, s), 4.10-4.17 (2H, m), 4.47 (1H, dd, J = 7.9, 5.7 Hz), 4.93 (1H, d, J = 15.2 Hz), 5.37 (1H, d, J = 15.2 Hz), 5.91 (1H, s), 6.38-6.42 (2H, m), 6.56 (1H, d, J = 2.0 Hz), 6.63 (1H, s), 6.86 (1H, d, J = 7.6 Hz), 7.23-7.29 (3H, m), 7.41 (1H, d, J = 7.8 Hz) .

### Referential Example 510

Ethyl [7-chloro-5-(2-methoxy-4-methylphenyl)-2-oxo-1,2,3,5-tetrahydro-4,1-benzoxazepin-3-yl]acetate

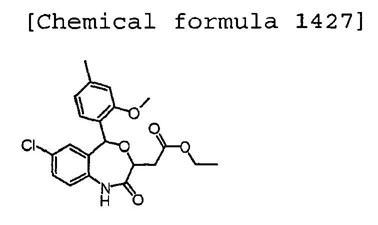

In a similar manner to that employed for the synthesis of ethyl [7-chloro-5-(2-methoxy-3-methylphenyl)-2-oxo-1,2,3,5-tetrahydro-4,1-benzoxazepin-3-yl]acetate, the title compound (1.085 g) was obtained as a 18:1 trans isomer:cis isomer mixture from ethyl [7-chloro-1-(2,4-dimethoxybenzyl)-5-(2-methoxy-4-methylphenyl)-2-oxo-1,2,3,5-tetrahydro-4,1-benzoxazepin-3-yl]acetate (2.52 g).
MS (ESI) m/z: 404 (M+H)⁺.
trans Isomer
¹H-NMR (CDCl₃) δ: 1.22 (3H, t, J = 7.1 Hz), 2.39 (3H, s), 2.77 (1H, dd, J = 16.3, 6.6 Hz), 3.01 (1H, dd, J = 16.3, 6.6 Hz), 3.70 (3H, s), 4.08-4.11 (2H, m), 4.60 (1H, t, J = 6.6 Hz), 6.18 (1H, s), 6.72-6.73 (2H, m), 6.84 (1H, d, J = 7.8 Hz), 6.96 (1H, d, J = 8.5 Hz), 7.25-7.27 (1H, m), 7.72 (1H, s).

### Referential Example 511

Ethyl [trans7-chloro-5-(2-methoxy-4-methylphenyl)-2-thioxo-1,2,3,5-tetrahydro-4,1-benzoxazepin-3-yl]acetate

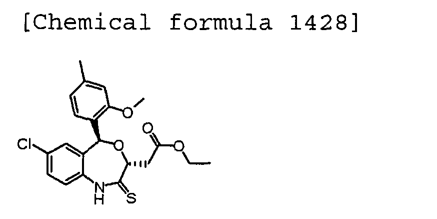

In a similar manner to that employed for the synthesis of ethyl {trans-7-chloro-2-thioxo-5-[2-(trifluoromethyl)pyridin-3-yl]-1,2,3,5-tetrahydro-4,1-benzoxazepin-3-yl}acetate, a solid obtained from ethyl [7-chloro-5-(2-methoxy-4-methylphenyl)-2-oxo-1,2,3,5-tetrahydro-4,1-benzoxazepin-3-yl]acetate (1.085 g) was crystallized from a small amount of ethyl acetate-hexane to give the title compound (0.947 g).
MS (ESI) m/z: 420 (M+H)⁺.
¹H-NMR (CDCl₃) δ: 1.24 (3H, t, J = 7.2 Hz), 2.40 (3H, s), 2.94 (1H, dd, J = 16.4, 6.3 Hz), 3.25 (1H, dd, J = 16.4, 6.8 Hz), 3.66 (3H, s), 4.13 (2H, q, J = 7.1 Hz), 4.69 (1H, t, J = 6.6 Hz), 6.09 (1H, s), 6.69-6.71 (2H, m), 6.87 (1H, d, J = 8.1 Hz), 7.04 (1H, d, J = 8.5 Hz), 7.32 (1H, dd, J = 8.3, 2.4 Hz), 7.35 (1H, d, J = 7.6 Hz), 9.54 (1H, br s).

### Example 712

Ethyl [trans-8-chloro-6-(2-methoxy-4-methylphenyl)-1-(trifluoromethyl)-4H,6H-[1,2,4]triazolo[4,3-a][4,1]benzoxazepin-4-yl]acetate

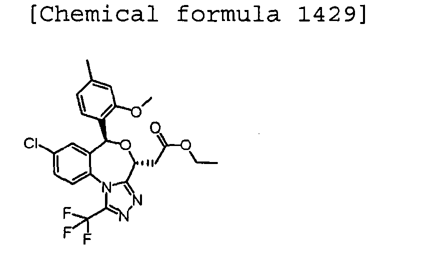

Ethyl [trans 7-chloro-5-(2-methoxy-4-methylphenyl)-2-thioxo-1,2,3,5-tetrahydro-4,1-benzoxazepin-3-yl]acetate (0.947 g) was dissolved in tetrahydrofuran (17 mL). To the resulting solution was added hydrazine hydrate (0.33 mL). The resulting mixture was stirred at room temperature for 50 minutes. The reaction mixture was diluted with saturated brine and extracted with ethyl acetate. The organic layer was washed twice with saturated brine, dried over anhydrous sodium sulfate and distilled under reduced pressure. The residue thus obtained was dissolved in dichloroethane (18 mL). To the resulting solution was added trifluoroacetic anhydride (0.94 mL) and the resulting mixture was stirred at room temperature for 80 minutes and then heated under reflux for 6.5 hours. After cooling, the solvent was distilled off under reduced pressure. The residue was subjected to silica gel column chromatography (25-30% ethyl acetate-hexane) and the crystallized from hexane containing a small amount of ethyl acetate to give the title compound(0.465 g).
MS (ESI) m/z: 496 (M+H)⁺.
¹H-NMR (CDCl₃) δ: 1.28 (3H, t, J = 7.1 Hz), 2.40 (3H, s), 3.23 (1H, dd, J = 16.6, 7.1 Hz), 3.48 (1H, dd, J = 16.8, 6.6 Hz), 3.54 (3H, s), 4.15-4.22 (2H, m), 4.92 (1H, t, J = 6.8 Hz), 5.51 (1H, s), 6.66 (1H, s), 6.86 (1H, t, J = 1.2 Hz), 6.90 (1H, d, J = 7.8 Hz), 7.46 (1H, d, J = 7.8 Hz), 7.49-7.51 (2H, m).

### Example 713

[trans-8-Chloro-6-(2-methoxy-4-methylphenyl)-1-(trifluoromethyl)-4H,6H-[1,2,4]triazolo[4,3-a][4,1]benzoxazepin-4-yl]acetic acid

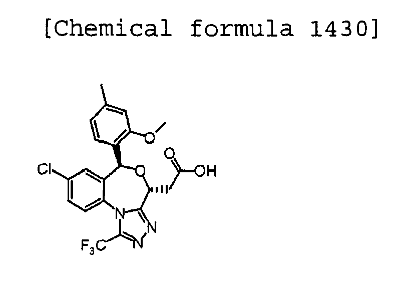

Ethyl [trans-8-chloro-6-(2-methoxy-4-methylphenyl)-1-(trifluoromethyl)-4H,6H-[1,2,4]triazolo[4,3-a][4,1]benzoxazepin-4-yl]acetate (0.20 g) was dissolved in dioxane (5 mL). To the resulting solution were added concentrated hydrochloric acid (2.5 mL) and water (2.5 mL). The resulting mixture was stirred at 60°C for 26 hours. After cooling, the reaction mixture was stirred under cooling with ice water and a crystalline solid was collected by filtration. The solid was washed with water and a very small amount of acetone to give the title compound (63 mg).
MS (ESI) m/z: 468 (M+H)⁺.
¹H-NMR (CDCl₃) δ: 2.40 (3H, s), 3.29 (1H, dd, J = 17.1, 6.6 Hz), 3.54 (3H, s), 3.55 (1H, dd, J = 17.1, 6.8 Hz), 4.89 (1H, t, J = 6.7 Hz), 5.51 (1H, s), 6.66 (1H, s), 6.87 (1H, s), 6.91 (1H, d, J = 8.1 Hz), 7.47 (1H, d, J = 7.8 Hz), 7.50-7.52 (2H, m).
Elemental analysis for: C₂₁H₁₇ClN₃O₄F₃·0.25H₂O
Calculated: C, 53.40; H, 3.73; Cl, 7.51; N, 8.90; F, 12.07.
Found: C, 53.44; H, 3.79; Cl, 7.51; N, 8.91; F, 12.13.

### Referential Example 512

tert-Butyl {4-chloro-2-[hydroxy(2-methoxyphenyl)methyl]phenyl}carbamate

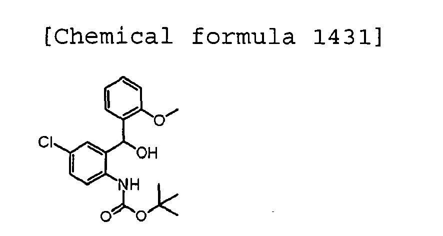

In a similar manner to that employed for the synthesis of tert-butyl (4-chloro-2-{hydroxy[2-(trifluoromethyl)pyridin-3-yl]methyl}phenyl)carbamate, the title compound (2.21 g) was obtained from tert-butyl 4-chlorophenylcarbamate(2.28 g) and 2-methoxybenzaldehyde (1.36 g) .
MS (ESI) m/z: 386 (M+Na)⁺.
¹H-NMR (CDCl₃)δ: 1.49 (9H, s), 3.33 (1H, d, J = 5.1 Hz), 3.90 (3H, s), 6.07 (1H, d, J = 4.9 Hz), 6.95-7.01 (2H, m), 7.07 (1H, d, J = 2.4 Hz), 7.18-7.24 (2H, m), 7.32 (1H, td, J = 7.9, 1.5 Hz), 7.92 (1H, d, J = 8.5 Hz), 8.06 (1H, br s).

### Referential Example 513

{5-Chloro-2-[(2,4-dimethoxybenzyl)amino]phenyl}(2-methoxyphenyl)methanol

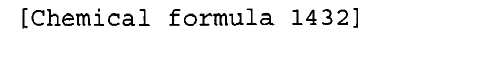

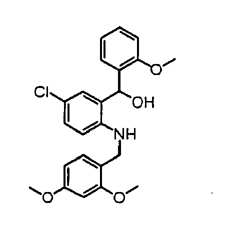

tert-Butyl {4-chloro-2-[hydroxy(2-methoxyphenyl)methyl]phenyl}carbamate (2.21 g) was dissolved in dioxane (10 mL). To the resulting solution was added concentrated hydrochloric acid (5 mL). The resulting mixture was stirred at room temperature for 2 hours. The reaction mixture was concentrated under reduced pressure. To the residue was added a 1N aqueous sodium hydroxide solution (50 mL), followed by extraction with ethyl acetate. The organic layer was dried over anhydrous sodium sulfate and the solvent was distilled off under reduced pressure to give (2-amino-5-chlorophenyl)(2-methoxyphenyl)methanol. The resulting product was dissolved in acetic acid (25 mL). To the resulting solution was added 2,4-dimethoxybenzaldehyde (1.06 g) and the resulting mixture was stirred at room temperature for one hour. Under cooling with ice water, sodium borohydride (0.34 g) was added and the resulting mixture was stirred at room temperature for 3 hours. The reaction mixture was diluted with ethyl acetate, washed twice with water and then successively with sodium bicarbonate water and brine, and dried over anhydrous sodium sulfate. The solvent was distilled off under reduced pressure. The residue was subjected to silica gel column chromatography (30% ethyl acetate-hexane) to give the title compound (1.12 g).
MS (ESI) m/z: 414 (M+H)⁺.
¹H-NMR (CDCl₃) δ: 3.76 (3H, s), 3.79 (3H, s), 3.80 (3H, s), 4.24 (2H, s), 6.01 (1H, s), 6.40 (1H, dd, J = 8.3, 2.2 Hz), 6.45 (1H, d, J = 2.4 Hz), 6.59 (1H, d, J = 8.5 Hz), 6.93-6.95 (2H, m), 6.97 (1H, d, J = 2.4 Hz), 7.07-7.09 (2H, m), 7.15 (1H, dd, J = 7.4, 1.6 Hz), 7.28-7.33 (1H, m).

### Referential Example 514

Ethyl [7-chloro-1-(2,4-dimethoxybenzyl)-5-(2-methoxyphenyl)-2-oxo-1,2,3,5-tetrahydro-4,1-benzoxazepin-3-yl] acetate

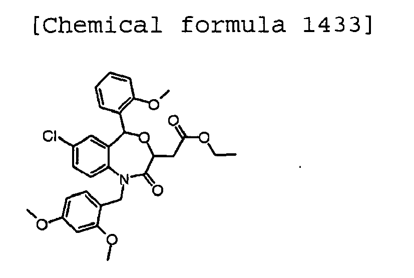

In a similar manner to that employed for the synthesis of ethyl [trans-5-(2-chloro-3-methoxyphenyl)-1-(2,4-dimethoxybenzyl)-7-methyl-2-oxo-1,2,3,5-tetrahydro-4,1-benzoxazepin-3-yl]acetate, the title compound (1.39 g) was obtained as a 10:1 trans isomer:cis isomer mixture from {5-chloro-2-[(2,4-dimethoxybenzyl)amino]phenyl}(2-methoxyphenyl)methanol (1.12 g).
MS (ESI) m/z: 541 (M+H)⁺.
trans Isomer ¹H-NMR (CDCl₃) δ: 1.25 (3H, t, J = 7.1 Hz), 2.80 (1H, dd, J = 16.6, 5.9 Hz), 3.10 (1H, dd, J = 16.3, 7.8 Hz), 3.50 (3H, s), 3.67 (3H, s), 3.77 (3H, s), 4.11-4.17 (2H, m), 4.49 (1H, dd, J = 7.9, 5.7 Hz), 4.93 (1H, d, J = 15.1 Hz), 5.40 (1H, d, J = 14.9 Hz), 5.95 (1H, s), 6.38-6.43 (2H, m), 6.52 (1H, d, J = 2.2 Hz), 6.82 (1H, d, J = 8.3 Hz), 7.05 (1H, t, J = 7.6 Hz), 7.24-7.35 (4H, m), 7.55 (1H, d, J = 6.3 Hz).

### Referential Example 515

Ethyl [7-chloro-5-(2-methoxyphenyl)-2-oxo-1,2,3,5-tetrahydro-4,1-benzoxazepin-3-yl]acetate

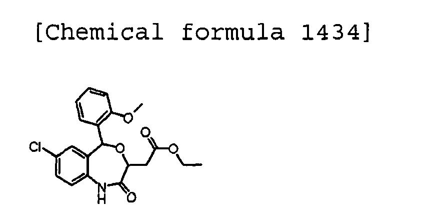

In a similar manner to that employed for the synthesis of ethyl [7-chloro-5-(2-methoxy-3-methylphenyl)-2-oxo-1,2,3,5-tetrahydro-4,1-benzoxazepin-3-yl]acetate, a solid obtained from ethyl [7-chloro-1-(2,4-dimethoxybenzyl)-5-(2-methoxyphenyl)-2-oxo-1,2,3,5-tetrahydro-4,1-benzoxazepin-3-yl]acetate (1.39 g) was stirred in hexane at room temperature for 2 hours and then collected by filtration to give the title compound (308 mg) as a 28:1 trans isomer:cis isomer mixture.
MS (ESI) m/z: 390 (M+H)⁺.
trans Isomer
¹H-NMR (CDCl₃) δ: 1.22 (3H, t, J = 7.2 Hz), 2.78 (1H, dd, J = 16.4, 6.6 Hz), 3.03 (1H, dd, J = 16.5, 6.7 Hz), 3.71 (3H, s), 4.07-4.15 (2H, m), 4.62 (1H, t, J = 6.6 Hz), 6.21 (1H, s), 6.68 (1H, d, J = 2.4 Hz), 6.91 (1H, d, J = 8.3 Hz), 6.98 (1H, d, J = 8.5 Hz), 7.05 (1H, td, J = 7.5, 0.9 Hz), 7.26-7.30 (1H, m), 7.37 (1H, td, J = 7.9, 1.7 Hz), 7.44 (1H, dd, J = 7.6, 1.7 Hz), 7.70 (1H, s).

### Referential Example 516

Ethyl [trans-7-chloro-5-(2-methoxyphenyl)-2-thioxo-1,2,3,5-tetrahydro-4,1-benzoxazepin-3-yl]acetate

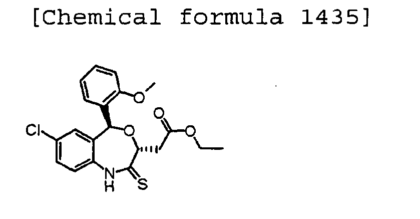

In a similar manner to that employed for the synthesis of ethyl {trans-7-chloro-2-thioxo-5-[2-(trifluoromethyl)pyridin-3-yl]-1,2,3,5-tetrahydro-4,1-benzoxazepin-3-yl}acetate, a solid obtained from ethyl [7-chloro-5-(2-methoxyphenyl)-2-oxo-1,2,3,5-tetrahydro-4,1-benzoxazepin-3-yl]acetate (308 mg) was crystallized from hexane containing a small amount of ethyl acetate to give the title compound (248 mg).
MS (ESI) m/z: 406 (M+H)⁺.
¹H-NMR (CDCl₃) δ: 1.24 (3H, dd, J = 10.0, 4.2 Hz), 2.96 (1H, dd, J = 16.4, 6.6 Hz), 3.26 (1H, dd, J = 16.4, 6.8 Hz), 3.67 (3H, s), 4.13 (2H, q, J = 7.2 Hz), 4.70 (1H, t, J = 6.6 Hz), 6.13 (1H, s), 6.65 (1H, d, J = 2.2 Hz), 6.90 (1H, d, J = 8.3 Hz), 7.04-7.09 (2H, m), 7.32-7.39 (2H, m), 7.51 (1H, dd, J = 7.4, 1.8 Hz).

### Example 714

Ethyl [trans-8-chloro-6-(2-methoxyphenyl)-1-(trifluoromethyl)-4H,6H-[1,2,4]triazolo[4,3-a] [4,1]benzoxazepin-4-yl]acetate

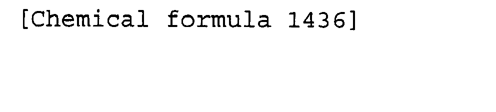

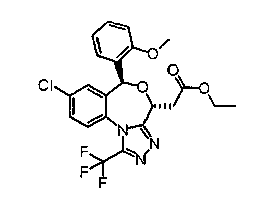

Ethyl [trans-7-chloro-5-(2-methoxyphenyl)-2-thioxo-1,2,3,5-tetrahydro-4,1-benzoxazepin-3-yl]acetate (247 mg) was dissolved in tetrahydrofuran (5 mL). Under cooling with ice water, hydrazine hydrate (0.089 mL) was added and the resulting mixture was stirred at room temperature for one hour. To the reaction mixture was added saturated brine and the resulting mixture was extracted with ethyl acetate. The organic layer was washed twice with saturated brine and dried over anhydrous sodium sulfate. The solvent was distilled off under reduced pressure. The residue thus obtained was dissolved in dichloroethane (7 mL). Under cooling with ice water, trifluoroacetic anhydride (0.25 mL) was added and the resulting mixture was stirred at room temperature for 70 minutes. The reaction mixture was concentrated under reduced pressure. The residue was dissolved in toluene (7 mL). To the resulting solution was added trifluoroacetic anhydride (0.25, mL). The resulting mixture was heated under reflux for 1 hour. After cooling, the reaction mixture was concentrated under reduced pressure. The residue was crystallized from hexane containing a small amount of ethyl acetate to give the title compound (261 mg).
MS (ESI) m/z: 482 (M+H)⁺.
¹H-NMR (CDCl₃) δ: 1.28 (3H, t, J = 7.1 Hz), 3.24 (1H, dd, J = 16.6, 7.1 Hz), 3.49 (1H, dd, J = 16.6, 6.6 Hz), 3.56 (3H, s), 4.16-4.22 (2H, m), 4.94 (1H, t, J = 6.8 Hz), 5.55 (1H, s), 6.81-6.83 (1H, m), 6.85 (1H, d, J = 8.3 Hz), 7.10 (1H, t, J = 7.6 Hz), 7.38 (1H, td, J = 7.9, 1.7 Hz), 7.51 (1H, s), 7.52 (1H, s), 7.61 (1H, dd, J = 7.4, 1.3 Hz) .

### Example 715

[trans-8-Chloro-6-(2-methoxyphenyl)-1-(trifluoromethyl)-4H,6H-[1,2,4]triazolo[4,3-a][4,1]benzoxazepin-4-yl]acetic acid

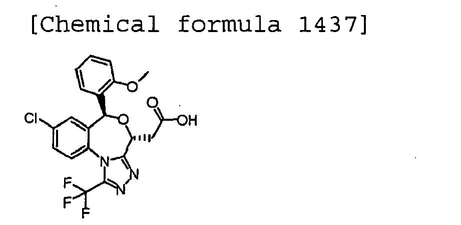

In a similar manner to that employed for the synthesis of [trans-8-chloro-6-(2-methoxy-4-methylphenyl)-1-(trifluoromethyl)-4H,6H-[1,2,4]triazolo[4,3-a] [4, 1] benzoxazepin-4-yl] acetic acid, a solid obtained from ethyl [trans-8-chloro-6-(2-methoxyphenyl)-1-(trifluoromethyl)-4H,6H-[1,2,4]triazolo[4,3-a][4,1]benzoxazepin-4-yl]acetate (261 mg) was subjected to silica gel column chromatography (chloroform:methanol=7:3), followed by crystallization from hexane containing a small amount of acetone to give the title compound (60 mg).
MS (ESI) m/z: 454 (M+H)⁺.
¹H-NMR (CDCl₃)δ: 3.30 (1H, dd, J = 17.1, 6.8 Hz), 3.56 (1H, dd, J = 17.0, 6.7 Hz), 3.56 (1H, s), 4.91 (1H, t, J = 6.7 Hz), 5.55 (1H, s), 6.83 (1H, t, J = 1.3 Hz), 6.86 (1H, d, J = 7.8 Hz), 7.11 (1H, t, J = 7.3 Hz), 7.39 (1H, td, J = 7.9, 1.7 Hz), 7.52 (1H, s), 7.52 (1H, s), 7.62 (1H, d, J = 6.3 Hz).
Elemental analysis for: C₂₀H₁₅ClN₃O₄F₃
Calculated: C, 52.93; H, 3.33; Cl, 7.81; N, 9.26; F, 12.56.
Found: C, 52.76; H, 3.22; Cl, 7.78; N, 9.17; F, 12.49.

### Referential Example 517

2-(Methoxymethoxy)-3-(trifluoromethyl)benzoic acid

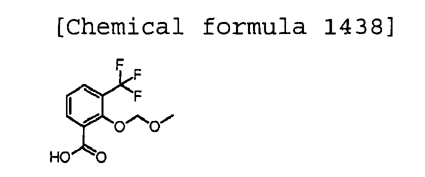

1-(Methoxymethoxy)-2-(trifluoromethyl)benzene (1.03 g) was dissolved in tetrahydrofuran (8.00 mL). At -78°C, a 2.55M n-butyl lithium hexane solution (1.96 mL) and potassium tert-butoxide (0.56 g) were added to the resulting solution. After stirring for one hour, a piece of dry ice was added to the brown suspended reaction mixture at -78°C. A very pale yellow solution thus obtained was diluted with water. A 1N aqueous sodium hydroxide solution (10 mL) was added and the resulting mixture was extracted with diethyl ether. After neutralization with citric acid (4.00 g), the water layer was separated and extracted dichloromethane. The organic layer was dried over anhydrous sodium sulfate. The filtrate was then concentrated to give the title compound (1.16 g). The compound was provided for the subsequent reaction without purification.

### Referential Example 518

2-Hydroxy-3-(trifluoromethyl)benzoic acid

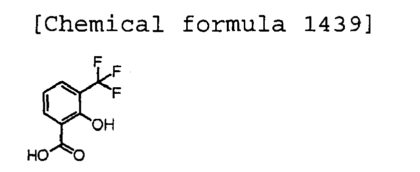

2-(Methoxymethoxy)-3-(trifluoromethyl)benzoic acid (1.16 g) was dissolved in methanol (5.00 mL). To the resulting solution was added a boron trifluoride diethyl ether complex (65.7 mg) at room temperature. The resulting mixture was stirred overnight. After the reaction mixture was concentrated, hexane was added to the residue and the solid thus formed was collected by filtration and dried under reduced pressure to give the title compound.
¹H-NMR (CDCl₃) δ: 7.04 (1H, t, J = 7.8 Hz), 7.84 (1H, d, J = 7.8 Hz), 8.14 (1H, dd, J = 7.8, 1.7 Hz), 11.07 (1H, s). MS (ESI) m/z : 207 (M + H)⁺.

### Referential Example 519

Methyl 2-methoxy-3-(trifluoromethyl)benzoate

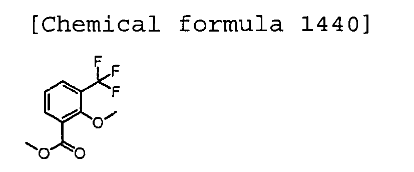

2-Hydroxy-3-(trifluoromethyl)benzoic acid (0.95 g) was dissolved in acetonitrile (16.0 mL). To the resulting solution were added potassium carbonate (1.92 g) and dimethyl sulfate (1.12 mL) at room temperature. After warming to 65°C and stirring for 3.5 hours, the reaction mixture was allowed to cool and water was added thereto. The resulting mixture was extracted with a dichloromethane:methanol (5:1) solvent mixture. The organic layer was dried over anhydrous sodium sulfate. The filtrate was then concentrated to give the title compound. The compound was provided for the subsequent reaction without purification.

### Referential Example 520

2-Methoxy-3-(trifluoromethyl)benzoic acid

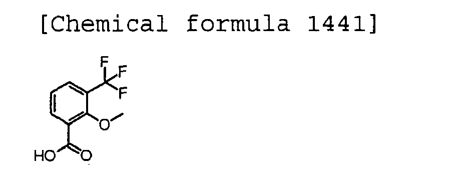

Methyl 2-methoxy-3-(trifluoromethyl)benzoate (1.08 g) was dissolved in tetrahydrofuran (20.0 mL). To the resulting solution were added a 5N aqueous sodium hydroxide solution (2.32 mL) and methanol (2.00 mL). The resulting mixture was stirred at room temperature for 1.5 hours and at 50°C for 2 hours. The reaction mixture was allowed to cool and then concentrated. The residue was neutralized with 1N hydrochloric acid (12 mL) and water was added. The resulting mixture was extracted with a dichloromethane:methanol (10:1) solvent mixture. The organic layer was dried over anhydrous sodium sulfate. The filtrate was then concentrated to give the title compound at a stoichiometric ratio. The compound was provided for the subsequent reaction without purification. ¹H-NMR (CDCl₃) δ: 4.02 (3H, s), 7.35 (1H, t, J = 7.8 Hz), 7.85 (1H, d, J = 7.8 Hz), 8.24 (1H, d, J = 7.8 Hz) .
MS (ESI) m/z : 221 (M + H)⁺.

### Referential Example 521

4-[2-Methoxy-3-(trifluoromethyl)benzoyl]morpholine

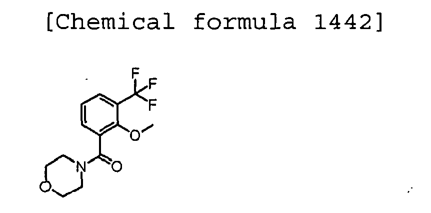

2-Methoxy-3-(trifluoromethyl)benzoic acid (1.02 g) was dissolved in dichloromethane (20.0 mL). To the resulting solution were added 1-hydroxybenzotriazole(0.63 g), morpholine (0.48 mL), and hydrochloric acid 1-(dimethylaminopropyl)-3-ethylcarbodiimide(1.07 g). The resulting mixture was stirred at room temperature for one day.
The reaction mixture was diluted with dichloromethane and then, washed with a saturated aqueous solution of sodium bicarbonate. The organic layer was dried over anhydrous sodium sulfate. After filtration, the filtrate was concentrated and the residue thus obtained was purified by medium pressure column chromatography (hexane:ethyl acetate = 1:1) to give the title compound at a stoichiometric ratio.
¹H-NMR (CDCl₃) δ: 3.15-3.23 (1H, m), 3.29-3.38 (1H, m), 3.55-3.63 (1H, m), 3.65-3.73 (1H, m), 3.75-3.84 (3H, m), 3.84-3.92 (4H, m), 7.26 (1H, t, J = 7.8 Hz), 7.51 (1H, d, J = 7.8 Hz), 7.65 (1H, d, J = 7.8 Hz).
MS (ESI) m/z : 290 (M + H)⁺.

### Referential Example 522

N-{4-Chloro-2-[2-methoxy-3-(trifluoromethyl)benzoyl]phenyl}-2,2-dimethylpropanamide

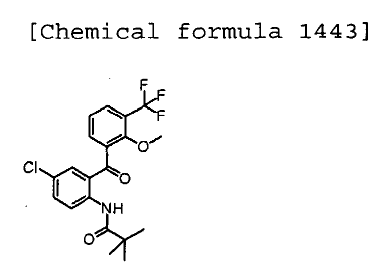

N-(4-Chlorophenyl)-2,2-dimethylpropanamide (891.2 mg) was dissolved in tetrahydrofuran (10.0 mL). To the resulting solution was added a 2.55M n-butyl lithium hexane solution (3.80 mL) at -15°C and the resulting mixture was stirred at room temperature for one hour. After cooling the reaction mixture to -30°C, a tetrahydrofuran solution (10.0 mL) of 4-[2-methoxy-3-(trifluoromethyl)benzoyl]morpholine (1.34 g) was added via a cannula. The reaction mixture was stirred at room temperature for 50 minutes. At -5°C, a saturated aqueous solution of ammonium chloride was added to the reaction mixture. After extraction with ethyl acetate, the organic layer was dried over anhydrous sodium sulfate. After filtration, the filtrate was concentrated and the residue thus obtained was purified by medium pressure column chromatography (hexane:ethyl acetate = 7:1) to give the title compound (1.47 g).
¹H-NMR (CDCl₃) δ: 1.38 (9H, s), 3.72 (3H, s), 7.32 (1H, t, J = 7.7 Hz), 7.38 (1H, d, J = 2.7 Hz), 7.49-7.53 (1H, m), 7.55 (1H, dd, J = 9.1, 2.7 Hz), 7.80 (1H, d, J = 7.7 Hz), 8.83 (1H, d, J = 9.1 Hz), 11.59 (1H, s).
MS (ESI) m/z : 414 (M + H)⁺.

### Referential Example 523

(2-Amino-5-chlorophenyl)[2-methoxy-3-(trifluoromethyl)phenyl]methanone

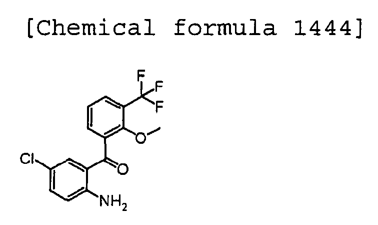

N-{4-Chloro-2-[2-methoxy-3-(trifluoromethyl)benzoyl]phenyl}-2,2-dimethylpropanamide (1.47 g) was suspended in ethanol (8.0 mL). To the resulting suspension was added a 5N aqueous sodium hydroxide solution (3.54 mL) at room temperature. The resulting mixture was stirred overnight at 90°C. The reaction mixture was allowed to cool and water was added thereto. After extraction with ethyl acetate, the organic layer was dried over anhydrous sodium sulfate. After filtration, the filtrate was concentrated and the residue thus obtained was purified by medium pressure column chromatography (hexane:ethyl acetate = 6:1) to give the title compound (1.04 g).
¹H-NMR (CDCl₃) δ: 3.73 (3H, s), 6.43 (2H, br s), 6.69 (1H, d, J = 8.8 Hz), 7.18 (1H, d, J = 2.4 Hz), 7.21-7.30 (2H, m), 7.46 (1H, d, J = 7.8 Hz), 7.73 (1H, d, J = 7.8 Hz). MS (ESI) m/z : 330 (M + H)⁺.

### Referential Example 524

(2-Amino-5-chlorophenyl)[2-methoxy-3-(trifluoromethyl)phenyl]methanol

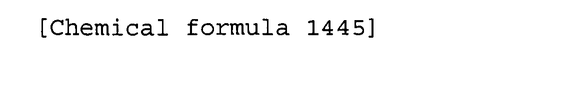

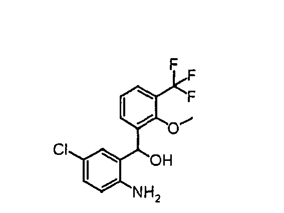

(2-Amino-5-chlorophenyl)[2-methoxy-3-(trifluoromethyl)phenyl]methanone (1.04 g) was dissolved in methanol (15.0 mL). To the resulting solution was added sodium borohydride (0.36 g) under ice cooling and the reaction mixture was stirred for 40 minutes. After stirring for further 2.5 hours at room temperature, the reaction mixture was quenched with water. Methanol was then distilled off under reduced pressure. To the residue was added water and the resulting mixture was extracted with ethyl acetate. The organic layer was dried over anhydrous sodium sulfate. After filtration, the filtrate was concentrated to give the title compound (1.04 g) at a stoichiometric ratio. The compound was provided for the subsequent reaction without purification.
MS (ESI) m/z : 314 (M - OH)⁺.

### Referential Example 525

{5-Chloro-2-[(2,4-dimethoxybenzyl)amino]phenyl}[2-methoxy-3-(trifluoromethyl)phenyl]methanol

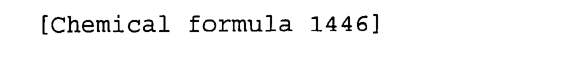

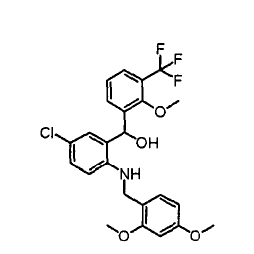

(2-Amino-5-chlorophenyl)[2-methoxy-3-(trifluoromethyl)phenyl]methanol (1.04 g) was dissolved in acetic acid (20.0 mL). Under ice cooling, 2,4-dimethoxybenzaldehyde (626.7 mg) was added and the resulting mixture was stirred at room temperature for 5 minutes. After ice cooling again, sodium borohydride (178.3 mg) was added. The resulting mixture was stirred at room temperature for 5 hours. The reaction mixture was concentrated. The residue was neutralized with a saturated aqueous solution of sodium bicarbonate and extracted with ethyl acetate. The organic layer was dried over anhydrous sodium sulfate. After filtration, the filtrate was concentrated and the residue thus obtained was purified by medium pressure column chromatography (hexane:ethyl acetate = 7:1) to give the title compound (1.03 g).
¹H-NMR (CDCl₃) δ: 3.73-3.80 (10H, m), 4.21 (2H, s), 6.15 (1H, s), 6.37 (1H, dd, J = 8.3, 2.2 Hz), 6.43 (1H, d, J = 2.2 Hz), 6.59-6.64 (1H, m), 6.99 (1H, d, J = 8.3 Hz), 7.07-7.13 (2H, m), 7.22 (1H, t, J = 7.8 Hz), 7.52-7.61 (2H, m). MS (ESI) m/z : 482 (M + H)⁺.

### Referential Example 526

Ethyl (2E)-4-[(4-chloro-2-{hydroxy[2-methoxy-3-(trifluoromethyl)phenyl]methyl}phenyl)(2,4-dimethoxybenzyl)amino]-4-oxobut-2-enoate

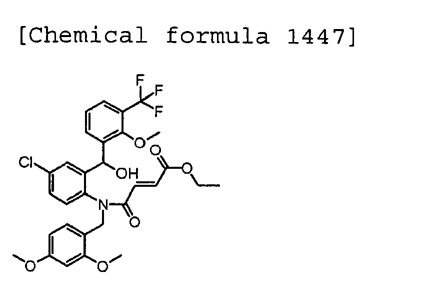

To a dichloromethane solution (10.0 mL) of {5-chloro-2-[(2,4-dimethoxybenzyl)amino]phenyl}[2-methoxy-3-(trifluoromethyl)phenyl]methanol (1.03 g) were added sodium hydrogen carbonate (0.27 g) and a dichloromethane solution (5.0 mL) of ethyl monoethyl chlorofumarate (0.42 g) under ice cooling. The reaction mixture was stirred overnight at room temperature. Sodium hydrogen carbonate (0.27 g) and a dichloromethane solution (2.0 mL) of ethyl monoethyl chlorofumarate (104.2 mg) were added further under ice cooling and the reaction mixture was stirred at room temperature for 6.5 hours. To the reaction mixture was added water, followed by extraction with dichloromethane. The organic layer was dried over anhydrous sodium sulfate.
After filtration, the filtrate was concentrated to give the title compound at a stoichiometric ratio. The compound was provided for the subsequent reaction without purification. MS (ESI) m/z : 608 (M + H)⁺.

### Referential Example 527

Ethyl {(trans)-7-chloro-1-(2,4-dimethoxybenzyl)-5-[2-methoxy-3-(trifluoromethyl)phenyl]-2-oxo-1,2,3,5-tetrahydro-4,1-benzoxazepin-3-yl}acetate

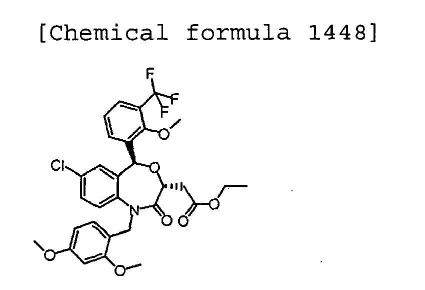

To an ethanol solution (26.0 mL) of ethyl (2E)-4-[(4-chloro-2-{hydroxy[2-methoxy-3-(trifluoromethyl)phenyl]methyl}phenyl)(2,4-dimethoxybenzyl)amino]-4-oxobut-2-enoate (1.30 g) was added potassium carbonate (0.44 g) at room temperature. The resulting mixture was stirred overnight. Potassium carbonate was filtered off and the filtrate was concentrated. The residue was dissolved in ethyl acetate and the resulting solution was washed with water. The organic layer was dried over anhydrous sodium sulfate. After filtration, the filtrate was concentrated and the residue thus obtained was purified by medium pressure column chromatography (hexane:ethyl acetate = 4:1) to give the title compound (1.13 g).
¹H-NMR (CDCl₃) δ: 1.25 (3H, t, J = 7.2 Hz), 2.79 (1H, dd, J = 16.6, 6.1 Hz), 2.98 (3H, s), 3.07 (1H, dd, J = 16.6, 7.3 Hz), 3.66 (3H, s), 3.77 (3H, s), 4.14 (2H, q, J = 7.2 Hz), 4.48-4.55 (1H, m), 4.85 (1H, d, J = 14.7 Hz), 5.52 (1H, d, J = 14.7 Hz), 6.00 (1H, s), 6.37-6.45 (2H, m), 6.56 (1H, d, J = 2.2 Hz), 7.22-7.43 (4H, m), 7.63 (1H, d, J = 7.7 Hz), 7.81 (1H, d, J = 7.7 Hz).
MS (ESI) m/z : 608 (M + H)⁺.

### Referential Example 528

Ethyl {(trans)-7-chloro-5-[2-methoxy-3-(trifluoromethyl)phenyl]-2-oxo-1,2,3,5-tetrahydro-4,1-benzoxazepin-3-yl}acetate

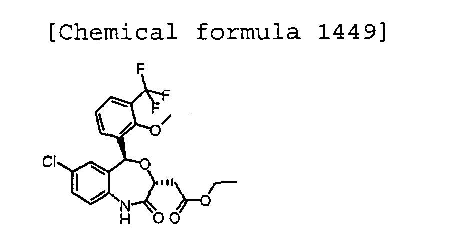

To an acetone solution (30.0 mL) of ethyl {(trans)-7-chloro-1-(2,4-dimethoxybenzyl)-5-[2-methoxy-3-(trifluoromethyl)phenyl]-2-oxo-1,2,3,5-tetrahydro-4,1-benzoxazepin-3-yl}acetate (1.13 g,) was added an aqueous solution (7.5 mL) of ceric ammonium nitrate (3.06 g) under ice cooling. After stirring for 2.5 hours, the reaction mixture was stirred at room temperature for further 3 hours. Water and brine were added and the resulting mixture was extracted with ethyl acetate. The organic layer was dried over anhydrous sodium sulfate. After filtration, the filtrate was concentrated and the residue thus obtained was purified by medium pressure column chromatography (hexane:ethyl acetate = 2:1) to give the title compound (699.6 mg).
¹H-NMR (CDCl₃) δ: 1.21 (3H, t, J = 7.1 Hz), 2.79 (1H, dd, J = 16.6, 6.8 Hz), 3.04 (1H, dd, J = 16.6, 6.5 Hz), 3.61 (3H, s), 4.10 (2H, q, J = 7.1 Hz), 4.66-4.74 (1H, m), 6.29 (1H, s), 6.67 (1H, d, J = 2.4 Hz), 7.09 (1H, d, J = 8.5 Hz), 7.29-7.40 (2H, m), 7.68 (1H, d, J = 7.8 Hz), 7.76 (1H, d, J = 7.8 Hz), 9.02 (1H, br s).
MS (ESI) m/z : 458 (M + H)⁺.

### Referential Example 529

Ethyl {(trans)-7-chloro-5-[2-methoxy-3-(trifluoromethyl)phenyl]-2-thioxo-1,2,3,5-tetrahydro-4,1-benzoxazepin-3-yl}acetate

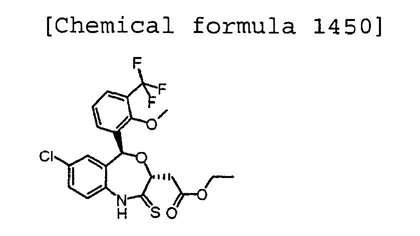

To a tetrahydrofuran solution (15.0 mL) of ethyl {(trans)-7-chloro-5-[2-methoxy-3-(trifluoromethyl)phenyl]-2-oxo-1,2,3,5-tetrahydro-4,1-benzoxazepin-3-yl}acetate (699.6 mg) were added sodium hydrogen carbonate (282.4 mg) and diphosphorus pentasulfide (679.3 mg) under ice cooling. The resulting mixture was stirred at room temperature for 6 hours. To the reaction mixture were added water and brine. The resulting mixture was extracted with ethyl acetate.
The organic layer was dried over anhydrous sodium sulfate. After filtration, the filtrate was concentrated and the residue thus obtained was purified was purified by silica gel column chromatography (hexane:ethyl acetate = 3:1) to give the title compound (700.3 mg).
¹H-NMR (CDCl₃) δ: 1.24 (3H, t, J = 7.1 Hz), 2.95 (1H, dd, J = 16.6, 6.6 Hz), 3.24 (1H, dd, J = 16.6, 6.3 Hz), 3.57 (3H, s), 4.13 (2H, q, J = 7.1 Hz), 4.70-4.78 (1H, m), 6.18 (1H, s), 6.66 (1H, d, J = 2.4 Hz), 7.12 (1H, d, J = 8.5 Hz), 7.33-7.41 (2H, m), 7.69 (1H, d, J = 7.6 Hz), 7.81 (1H, d, J = 7.8 Hz), 9.90 (1H, br s).
MS (ESI) m/z : 474 (M + H)⁺.

### Example 716

Ethyl [(trans)-8-chloro-6-[2-methoxy-3-(trifluoromethyl)phenyl]-1-(trifluoromethyl)-4H,6H-[1,2,4]triazolo[4,3-a] [4,1]benzoxazepin-4-yl]acetate

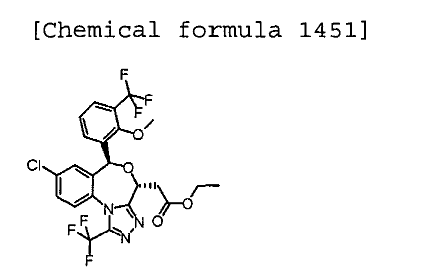

To a tetrahydrofuran solution (14.0 mL) of ethyl {(trans)-7-chloro-5-[2-methoxy-3-(trifluoromethyl)phenyl]-2-thioxo-1,2,3,5-tetrahydro-4,1-benzoxazepin-3-yl}acetate (700.3 mg) was added hydrazine monohydrate (143.4 µl) under ice cooling. The resulting mixture was stirred at room temperature for 45 minutes. To the reaction mixture were added 1,2-dichloroethane (14.0 mL), trifluoroacetic anhydride (1.03 mL) and trifluoroacetic acid (2.28 mL).
The resulting mixture was stirred at room temperature for 25 minutes and at 55°C for 75 minutes. Toluene (14.0 mL) was added and the resulting mixture was heated under reflux for 1.5 hours. The reaction mixture was allowed to cool and then concentrated. The residue was neutralized with a saturated aqueous solution of sodium bicarbonate. After addition of water, the resulting mixture was extracted with ethyl acetate. The organic layer was dried over anhydrous sodium sulfate. After filtration, the filtrate was concentrated and the residue thus obtained was purified by medium pressure column chromatography (hexane:ethyl acetate = 3:1) to give the title compound (737.5 mg).
¹H-NMR (CDCl₃) δ: 1.28 (3H, t, J = 7.1 Hz), 3.24 (1H, dd, J = 16.8, 7.1 Hz), 3.35 (3H, s), 3.49 (1H, dd, J = 16.8, 6.2 Hz), 4.19 (2H, q, J = 7.1 Hz), 4.94-5.02 (1H, m), 5.62 (1H, s), 6.87 (1H, s), 7.41 (1H, t, J = 7.8 Hz), 7.54-7.62 (2H, m), 7.70 (1H, d, J = 7.8 Hz), 7.89 (1H, d, J = 7.8 Hz).
MS (ESI) m/z : 550 (M + H)⁺.

### Example 717

[(trans)-8-Chloro-6-[2-methoxy-3-(trifluoromethyl)phenyl]-1-(trifluoromethyl)-4H,6H-[1,2,4]triazolo[4,3-a] [4,1]benzoxazepin-4-yl]acetic acid

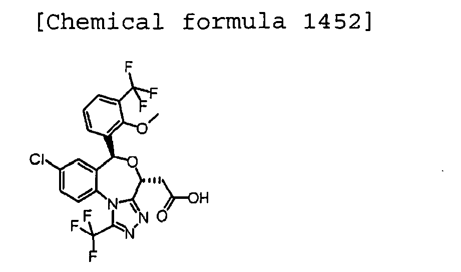

To a dioxane solution (4.0 mL) of ethyl [(trans)-8-chloro-6-[2-methoxy-3-(trifluoromethyl)phenyl]-1-(trifluoromethyl)-4H,6H-[1,2,4]triazolo[4,3-a][4,1]benzoxazepin-4-yl]acetate (143.6 mg) was added concentrated hydrochloric acid (1.0 mL). The resulting mixture was stirred at 60°C for 3 days. To the reaction mixture was added water, followed by extraction with a dichloromethane:methanol (10:1) solvent mixture. The organic layer was dried over anhydrous sodium sulfate. After filtration, the filtrate was concentrated and the residue thus obtained was purified by preparative thin layer chromatography on silica gel (dichloromethane:methanol = 10:1). The solid thus obtained was dissolved in a small amount of ethyl acetate. Hexane was added. The solid thus precipitated was collected by filtration and purified further by medium pressure column chromatography (dichloromethane:methanol = 9:1). The resulting solid was dissolved in dioxane and lyophilized to give the title compound (88.5 mg).
¹H-NMR (CDCl₃) δ: 3.26-3.36 (4H, m), 3.57 (1H, dd, J = 17.2, 6.8 Hz), 4.95 (1H, t, J = 6.8 Hz), 5.62 (1H, s), 6.88 (1H, s), 7.42 (1H, t, J = 7.9 Hz), 7.55-7.62 (2H, m), 7.70 (1H, d, J = 7.8 Hz), 7.90 (1H, d, J = 7.8 Hz).
MS (ESI) m/z : 522 (M + H)⁺.
Elemental analysis for: C₂₁H₁₄ClF₆N₃O₄·0.5H₂O·0.25 hexane·0.1 dioxane
Calculated: C, 49.01; H, 3.47; Cl, 6.32; F, 20.31; N, 7.49.
Found: C, 49.36; H, 3.11; Cl, 6.32; F, 20.05; N, 7.29.

### Referential Example 530

### 4-[2-Methoxy-3-(trifluoromethoxy)benzoyl]morpholine

2-Methoxy-3-(trifluoromethoxy)benzoic acid (1.96 g) was dissolved in dichloromethane (40.0 mL). To the resulting solution were added 1-hydroxybenzotriazole(1.12 g), morpholine (0.87 mL), and hydrochloric acid 1-(dimethylaminopropyl)-3-ethylcarbodiimide (1.91 g). The resulting mixture was stirred at room temperature for one day. The reaction mixture was diluted with dichloromethane and then, washed with a saturated aqueous solution of sodium bicarbonate. The organic layer was dried over anhydrous sodium sulfate. After filtration, the filtrate was concentrated and the residue thus obtained was purified by medium pressure column chromatography (hexane:ethyl acetate = 1:2) to give the title compound (2.50 g).
¹H-NMR (CDCl₃) δ: 3.16-3.24 (1H, m), 3.25-3.34 (1H, m), 3.54-3.62 (1H, m), 3.63-3.71 (1H, m), 3.72-3.80 (3H, m), 3.84-3.95 (4H, m), 7.16 (1H, t, J = 7.8 Hz), 7.21 (1H, dd, J = 7.8, 1.8 Hz), 7.28-7.33 (1H, m).
MS (ESI) m/z : 306 (M + H)⁺.

### Referential Example 531

N-{4-Chloro-2-[2-methoxy-3-(trifluoromethoxy)benzoyl]phenyl}-2,2-dimethylpropanamide

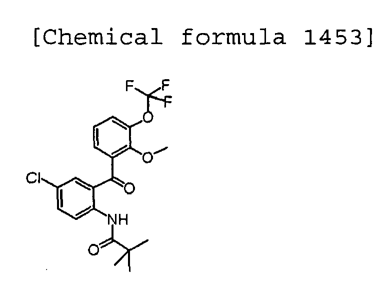

N-(4-Chlorophenyl)-2,2-dimethylpropanamide (1.58 g) was dissolved in tetrahydrofuran (30.0 mL). To the resulting solution was added a 2.55M n-butyl lithium hexane solution (6.72 mL) at -13°C. The resulting mixture was stirred at room temperature for 1.5 hours. The reaction mixture was cooled to -40°C and a tetrahydrofuran solution (10.0 mL) of 4-[2-methoxy-3-(trifluoromethoxy)benzoyl]morpholine (2.50 g) was added thereto via cannula. The reaction mixture was stirred at room temperature for 1.5 hours. To the reaction mixture was added a saturated aqueous solution of ammonium chloride at -8°C. After extraction with ethyl acetate, the organic layer was dried over anhydrous sodium sulfate. After filtration, the filtrate was concentrated and the residue thus obtained was purified by medium pressure column chromatography (hexane:ethyl acetate = 9:1) to give the title compound (2.77 g).
¹H-NMR (CDCl₃) δ: 1.38 (9H, s), 3.81 (3H, s), 7.21-7.27 (2H, m), 7.36 (1H, d, J = 2.6 Hz), 7.42-7.48 (1H, m), 7.53 (1H, dd, J = 9.1, 2.6 Hz), 8.83 (1H, d, J = 9.1 Hz), 11.60 (1H, s).
MS (ESI) m/z : 430 (M + H)⁺.

### Referential Example 532

(2-Amino-5-chlorophenyl)[2-methoxy-3-(trifluoromethoxy) phenyl]methanone

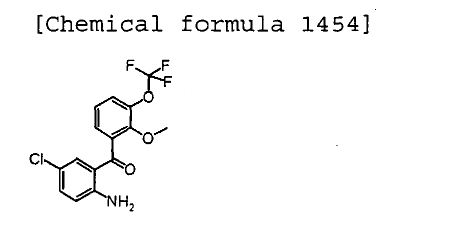

N-{4-Chloro-2-[2-methoxy-3-(trifluoromethoxy)benzoyl]phenyl}-2,2-dimethylpropanamide (2.77 g) was suspended in ethanol (15.0 mL). To the resulting suspension was added a 5N aqueous sodium hydroxide solution (6.44 mL) at room temperature. The resulting mixture was stirred overnight at 90°C. The reaction mixture was allowed to cool and concentrated. Water was added to the residue, followed by extraction with ethyl acetate. The organic layer was dried over anhydrous sodium sulfate. After filtration, the filtrate was concentrated and the residue thus obtained was purified by medium pressure column chromatography (hexane:ethyl acetate = 9:1) to give the title compound (2.15 g).
¹H-NMR (CDCl₃) δ: 3.81 (3H, s), 6.39 (2H, br s), 6.68 (1H, d, J = 8.8 Hz), 7.15-7.21 (3H, m), 7.23 (1H, dd, J = 8.8, 2.6 Hz), 7.35-7.41 (1H, m).
MS (ESI) m/z : 346 (M + H)⁺.

### Referential Example 533

(2-Amino-5-chlorophenyl)[2-methoxy-3-(trifluoromethoxy)phenyl]methanol

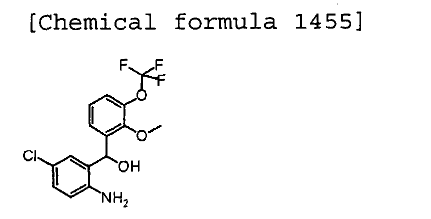

(2-Amino-5-chlorophenyl)[2-methoxy-3-(trifluoromethoxy)phenyl]methanone (2.15 g) was dissolved in methanol (32.0 mL). Under ice cooling, sodium borohydride (0.71 g) was added and the resulting mixture was stirred for one hour. After stirring at room temperature for further 2.5 hours, the reaction mixture was quenched with water. Methanol was distilled under reduced pressure. Water was added to the residue and the resulting mixture was extracted with ethyl acetate. The organic layer was dried over anhydrous sodium sulfate. After filtration, the filtrate was concentrated to give the title compound (2.37 g) at a stoichiometric ratio. The compound was provided for the subsequent reaction without purification.
MS (ESI) m/z : 330 (M - OH)⁺.

### Referential Example 534

{5-Chloro-2-[(2,4-dimethoxybenzyl)amino]phenyl}[2-methoxy-3-(trifluoromethoxy)phenyl]methanol

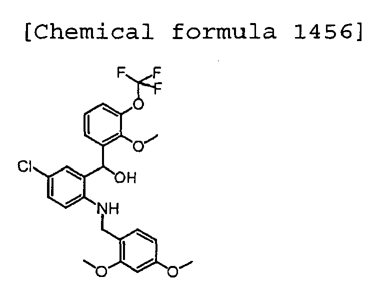

(2-Amino-5-chlorophenyl)[2-methoxy-3-(trifluoromethoxy)phenyl]methanol (2.37 g, crude) was dissolved in acetic acid (40.0 mL). Under ice cooling, 2,4-dimethoxybenzaldehyde (1.24 g) was added and the resulting mixture was stirred at room temperature for 30 minutes. The reaction mixture was ice cooled again and sodium borohydride (352.8 mg) was added. After stirring at room temperature for 2.5 hours, the reaction mixture was concentrated. The residue was neutralized with a saturated aqueous solution of sodium bicarbonate and extracted with ethyl acetate. The organic layer was dried over anhydrous sodium sulfate. After filtration, the residue obtained by concentrating the filtrate was purified by medium pressure column chromatography (hexane:ethyl acetate = 7:1) to give the title compound (2.86 g).
¹H-NMR (CDCl₃) δ: 3.73-3.81 (10H, m), 4.22 (2H, s), 6.04 (1H, s), 6.38 (1H, dd, J = 8.3, 2.2 Hz), 6.44 (1H, d, J = 2.2 Hz), 6.62 (1H, d, J = 8.8 Hz), 6.99 (1H, d, J = 2.2 Hz), 7.03 (1H, d, J = 8.3 Hz), 7.06-7.12 (2H, m), 7.20-7.27 (2H, m).
MS (ESI) m/z : 498 (M + H)⁺.

### Referential Example 535

Ethyl (2E)-4-[(4-chloro-2-{hydroxy[2-methoxy-3-(trifluoromethoxy)phenyl]methyl}phenyl)(2,4-dimethoxybenzyl)amino]-4-oxobut-2-enoate

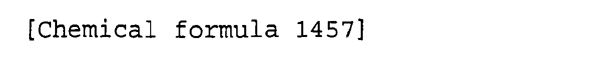

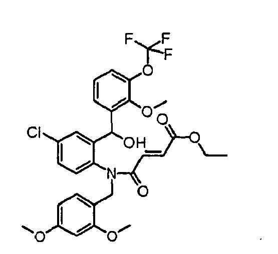

To a dichloromethane solution (25.0 mL) of {5-chloro-2-[(2,4-dimethoxybenzyl)amino]phenyl}[2-methoxy-3-(trifluoromethoxy)phenyl]methanol (2.86 g) were added sodium hydrogen carbonate (1.45 g) and a dichloromethane solution (5.0 mL) of ethyl monoethyl chlorofumarate (1.12 g) under ice cooling. The resulting mixture was stirred at room temperature for 3 hours. Water was added and the resulting mixture was extracted with dichloromethane. The organic layer was dried over anhydrous sodium sulfate. After filtration, the filtrate was concentrated to give the title compound (3.78 g) at a stoichiometric ratio. The compound was provided for the subsequent reaction without purification.
MS (ESI) m/z : 624 (M + H)⁺.

### Referential Example 536

Ethyl {(trans)-7-chloro-1-(2,4-dimethoxybenzyl)-5-[2-methoxy-3-(trifluoromethoxy)phenyl]-2-oxo-1,2,3,5-tetrahydro-4,1-benzoxazepin-3-yl}acetate

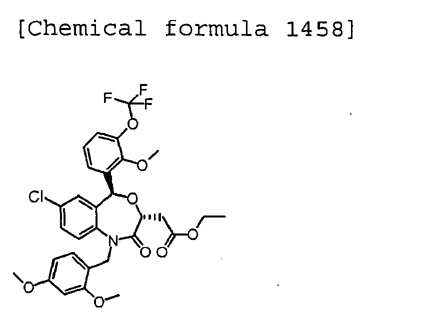

To an ethanol solution (70.0 mL) of ethyl (2E)-4-[(4-chloro-2-{hydroxy[2-methoxy-3-(trifluoromethoxy)phenyl]methyl}phenyl)(2,4-dimethoxybenzyl)amino]-4-oxobut-2-enoate (3.78 g, equivalent to 5.74 mmol) was added potassium carbonate (1.19 g) at room temperature. The resulting mixture was stirred for 2 days. After potassium carbonate was filtered off, the residue obtained by concentration of the filtrate was dissolved in ethyl acetate and the resulting solution was washed with water. The organic layer was dried over anhydrous sodium sulfate. After filtration, the filtrate was concentrated and the residue thus obtained was purified by medium pressure column chromatography (hexane:ethyl acetate= from 9:1, 7:1, 6:1, 5:1 to 4:1) to give the title compound (3.23 g).
¹H-NMR (CDCl₃) δ: 1.25 (3H, t, J = 7.2 Hz), 2.80 (1H, dd, J = 16.6, 6.0 Hz), 3.10 (1H, dd, J = 16.6, 7.6 Hz), 3.19 (3H, s), 3.67 (3H, s), 3.76 (3H, s), 4.10-4.19 (2H, m), 4.44-4.50 (1H, m), 4.82 (1H, d, J = 14.9 Hz), 5.54 (1H, d, J = 14.9 Hz), 5.96 (1H, s), 6.36-6.43 (2H, m), 6.48 (1H, d, J = 2.2 Hz), 7.15-7.38 (5H, m), 7.47-7.54 (1H, m).
MS (ESI) m/z : 624 (M + H)⁺.

### Referential Example 537

Ethyl {(trans)-7-chloro-5-[2-methoxy-3-(trifluoromethoxy)phenyl]-2-oxo-1,2,3,5-tetrahydro-4,1-benzoxazepin-3-yl}acetate

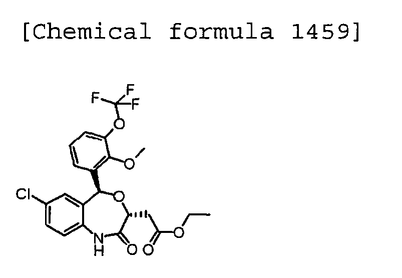

To an acetone solution (88.0 mL) of ethyl {(trans)-7-chloro-1-(2,4-dimethoxybenzyl)-5-[2-methoxy-3-(trifluoromethoxy)phenyl]-2-oxo-1,2,3,5-tetrahydro-4,1-benzoxazepin-3-yl}acetate (3.23 g) was added an aqueous solution (22.0 mL) of ceric ammonium nitrate (8.52 g) under ice cooling. The resulting mixture was stirred for 25 minutes. The reaction mixture was stirred at room temperature for further 2 hours and then, water and brine were added thereto. The resulting mixture was extracted with ethyl acetate. The organic layer was dried over anhydrous sodium sulfate. After filtration, the residue obtained by concentrating the filtrate was purified by medium pressure column chromatography (hexane:ethyl acetate = 2:1) to give the title compound (2.07 g).
¹H-NMR (CDCl₃) δ: 1.22 (3H, t, J = 7.2 Hz), 2.81 (1H, dd, J = 16.4, 6.6 Hz), 3.05 (1H, dd, J = 16.4, 6.7 Hz), 3.69 (3H, s), 4.07-4.16 (2H, m), 4.63-4.69 (1H, m), 6.20 (1H, s), 6.62 (1H, d, J = 2.4 Hz), 7.08 (1H, d, J = 8.3 Hz), 7.22 (1H, t, J = 8.0 Hz), 7.28-7.35 (2H, m), 7.43-7.48 (1H, m), 9.10 (1H, br s).
MS (ESI) m/z : 474 (M + H)⁺.

### Referential Example 538

Ethyl {(trans)-7-chloro-5-[2-methoxy-3-(trifluoromethoxy)phenyl]-2-thioxo-1,2,3,5-tetrahydro-4,1-benzoxazepin-3-yl}acetate

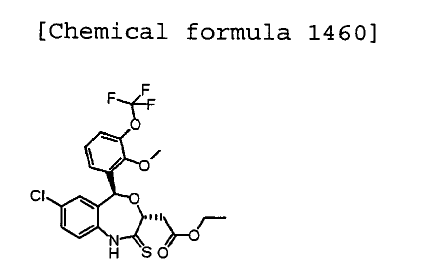

To a tetrahydrofuran solution (45.0 mL) of ethyl {(trans)-7-chloro-5-[2-methoxy-3-(trifluoromethoxy)phenyl]-2-oxo-1,2,3,5-tetrahydro-4,1-benzoxazepin-3-yl}acetate (2.07 g) were added sodium hydrogen carbonate (0.81 g) and diphosphorus pentasulfide (1.94 g) under ice cooling. The resulting mixture was stirred at room temperature for 6 hours. Water and brine were added and the resulting mixture was extracted with ethyl acetate. The organic layer was dried over anhydrous sodium sulfate. After filtration, the residue obtained by concentrating the filtrate was purified by medium pressure column chromatography (hexane:ethyl acetate = 3:1) to give the title compound (1.73 g).
¹H-NMR (CDCl₃) δ: 1.25 (3H, t, J = 7.2 Hz), 2.97 (1H, dd, J = 16.5, 6.5 Hz), 3.27 (1H, dd, J = 16.5, 6.8 Hz), 3.66 (3H, s), 4.14 (2H, q, J = 7.2 Hz), 4.71 (1H, t, J = 6.6 Hz), 6.11 (1H, s), 6.60 (1H, d, J = 2.4 Hz), 7.12 (1H, d, J = 8.3 Hz), 7.20-7.28 (1H, m), 7.29-7.38 (2H, m), 7.48-7.54 (1H, m), 10.29 (1H, s).
MS (ESI) m/z : 490 (M + H)⁺.

### Example 718

Ethyl [(trans)-8-chloro-6-[2-methoxy-3-(trifluoromethoxy)phenyl]-1-(trifluoromethyl)-4H,6H-[1,2,4]triazolo[4,3-a][4,1]benzoxazepin-4-yl]acetate

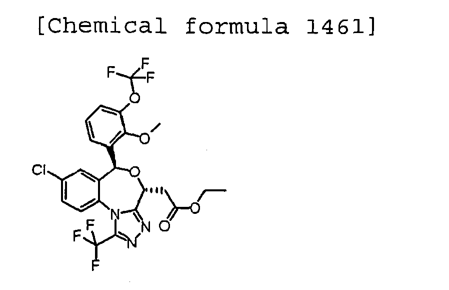

To a tetrahydrofuran solution (35.0 mL) of ethyl {(trans)-7-chloro-5-[2-methoxy-3-(trifluoromethoxy)phenyl]-2-thioxo-1,2,3,5-tetrahydro-4,1-benzoxazepin-3-yl}acetate (1.73 g) was added hydrazine monohydrate (0.34 mL) under ice cooling. The resulting mixture was stirred at room temperature for 45 minutes. To the reaction mixture were added 1,2-dichloroethane (35.0 mL), trifluoroacetic anhydride (2.45 mL) and trifluoroacetic acid (5.44 mL).
The resulting mixture was stirred at room temperature for 25 minutes and then, at 55°C for 75 minutes. Toluene (35.0 mL) was added and the resulting mixture was heated under reflux for 1.5 hours. After the reaction mixture was allowed to cool and concentrated, the residue was neutralized with a saturated aqueous solution of sodium bicarbonate. Water was added and the resulting mixture was extracted with ethyl acetate. The organic layer was dried over anhydrous sodium sulfate. After filtration, the residue obtained by concentrating the filtrate was purified by medium pressure column chromatography (hexane:ethyl acetate = 3:1) to give the title compound (1.69 g).
¹H-NMR (CDCl₃) δ: 1.29 (3H, t, J = 7.2 Hz), 3.25 (1H, dd, J = 16.8, 7.1 Hz), 3.45-3.54 (4H, m), 4.16-4.24 (2H, m), 4.96 (1H, t, J = 6.7 Hz), 5.55 (1H, s), 6.80 (1H, s), 7.21-7.28 (1H, m), 7.33 (1H, d, J = 8.3 Hz), 7.53-7.60 (3H, m).
MS (ESI) m/z : 566 (M + H)⁺.

### Example 719

[(trans)-8-Chloro-6-[2-methoxy-3-(trifluoromethoxy)phenyl]-1-(trifluoromethoxy)-4H,6H-[1,2,4]triazolo[4,3-a] [4,1]benzoxazepin-4-yl]acetic acid

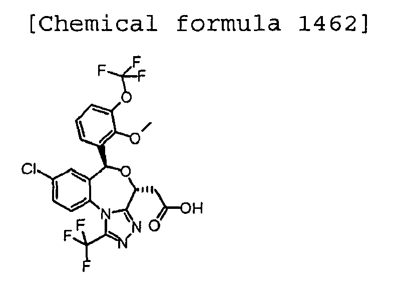

To a dioxane solution (4.0 mL) of ethyl [(trans)-8-chloro-6-[2-methoxy-3-(trifluoromethoxy)phenyl]-1-(trifluoromethyl)-4H,6H-[1,2,4]triazolo[4,3-a][4,1]benzoxazepin-4-yl]acetate (149.2 mg) was added concentrated hydrochloric acid (1.0 mL). The resulting mixture was stirred at 60°C for 3 days. To the reaction mixture was added water and the resulting mixture was extracted with a dichloromethane:methanol (10:1) solvent mixture. The organic layer was dried over anhydrous sodium sulfate. After filtration, the residue obtained by concentrating the filtrate was purified by preparative thin layer chromatography on silica gel (dichloromethane:methanol = 10:1). The resulting compound was purified further by medium pressure column chromatography (dichloromethane:methanol = 9:1), dissolved in dioxane and lyophilized to give the title compound (118.2 mg).
¹H-NMR (CDCl₃) δ: 3.31 (1H, dd, J = 17.1, 6.8 Hz), 3.50 (3H, s), 3.58 (1H, dd, J = 17.1, 6.8 Hz), 4.92 (1H, t, J = 6.8 Hz), 5.54 (1H, s), 6.80 (1H, s), 7.22-7.28 (1H, m), 7.33 (1H, d, J = 6.8 Hz), 7.55-7.61 (3H, m).
MS (ESI) m/z : 538 (M + H)⁺.
Elemental analysis for: C₂₁H₁₄ClF₆N₃O₅·0.1 hexane·0.1 dioxane Calculated: C, 47.59; H, 2.94; Cl, 6.39; F, 20.53; N, 7.57.
Found: C, 47.40; H, 2.84; Cl, 6.38; F, 20.10; N, 7.33.

### Referential Example 539

(2-Amino-3-chlorophenyl)(2,3-dimethoxyphenyl)methanone

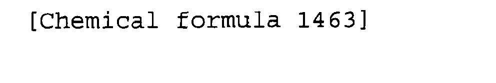

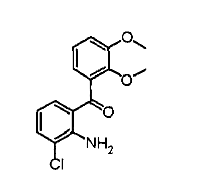

To a solution of 1,2-dimethoxybenzene (1.85 mL) in tetrahydrofuran (20.0 mL) were added N,N,N',N'-tetramethylethylenediamine (2.18 mL) and a 2.55M n-butyl lithium hexane solution (6.24 mL) at -78°C. The resulting mixture was stirred at room temperature for 2 hours. The reaction mixture was cooled to -78°C again. After dropwise addition of a solution of 8-chloro-2-methyl-4H-3,1-benzoxazin-4-one (2.83 g) in tetrahydrofuran (30.0 mL) via cannula, the resulting mixture was stirred at room temperature for 75 minutes. The reaction mixture was concentrated and the residue was dissolved in ethanol (20.0 mL) and water (5.00 mL). To the resulting solution was added concentrated hydrochloric acid (5.00 mL) at room temperature. After stirring overnight at 90°C, concentrated hydrochloric acid (5.00 mL) was added again at room temperature. The resulting mixture was stirred at 90°C for further 9 hours. After the reaction mixture was allowed to cool and concentrated, the residue was neutralized with a saturated aqueous solution of sodium bicarbonate under ice cooling and extracted with ethyl acetate. The organic layer was dried over anhydrous sodium sulfate. After filtration, the residue obtained by concentrating the filtrate was purified by medium pressure column chromatography (hexane:ethyl acetate = 9:1) to give the title compound (2.60 g).
¹H-NMR (CDCl₃) δ: 3.76 (3H, s), 3.91 (3H, s), 6.48 (1H, t, J = 7.6 Hz), 6.82 (1H, dd, J = 7.6, 1.5 Hz), 6.91 (2H, br s), 7.01 (1H, dd, J = 7.6, 1.5 Hz), 7.11 (1H, t, J = 7.6 Hz), 7.22-7.27 (1H, m), 7.40 (1H, dd, J = 7.6, 1.5 Hz). MS (ESI) m/z : 292 (M + H)⁺.

### Referential Example 540

(2-Amino-3-chlorophenyl)(2,3-dimethoxyphenyl)methanol

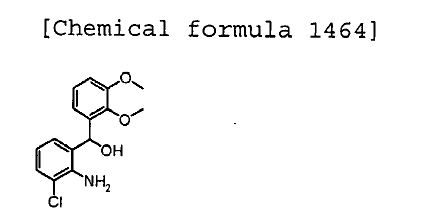

(2-Amino-3-chlorophenyl)(2,3-dimethoxyphenyl)methanone (2.60 g) was dissolved in methanol (40.0 mL). Under ice cooling, sodium borohydride (1.01 g) was added and the resulting mixture was stirred for 80 minutes. After stirring for further 2.5 hours at room temperature, the reaction mixture was quenched with water and distilled under reduced pressure to remove methanol. After addition of water to the residue and extraction with ethyl acetate, the organic layer was dried over anhydrous sodium sulfate. After filtration, the filtrate was concentrated to give the title compound (2.50 g). The compound was provided for the subsequent reaction without purification.
MS (ESI) m/z : 276 (M - OH)⁺.

### Referential Example 541

{3-Chloro-2-[(2,4-dimethoxybenzyl)amino]phenyl}(2,3-dimethoxyphenyl)methanol

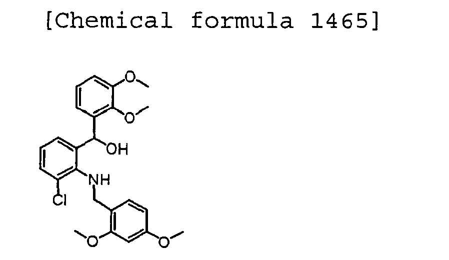

(2-Amino-3-chlorophenyl)(2,3-dimethoxyphenyl)methanol (2.50 g) was dissolved in acetic acid (50.0 mL). To the resulting solution was added 2,4-dimethoxybenzaldehyde (1.70 g) under ice cooling. The resulting mixture was stirred at room temperature for 40 minutes. After ice cooling again, sodium borohydride (0.48 g) was added and the mixture was stirred at room temperature for 3 hours. The reaction mixture was concentrated. The residue was neutralized with a saturated aqueous solution of sodium bicarbonate and extracted with ethyl acetate. The organic layer was dried over anhydrous sodium sulfate. After filtration, the residue obtained by concentrating the filtrate was purified by medium pressure column chromatography (hexane:ethyl acetate = 4:1) to give the title compound (2.96 g).
¹H-NMR (CDCl₃) δ: 3.69 (3H, s), 3.76-3.89 (9H, m), 4.18 (1H, d, J = 12.9 Hz), 4.29 (1H, d, J = 12.9 Hz), 6.33 (1H, s), 6.39 (1H, dd, J = 8.2, 2.3 Hz), 6.45-6.48 (1H, m), 6.80-6.92 (3H, m), 6.97-7.02 (1H, m), 7.04-7.12 (2H, m), 7.22-7.27 (1H, m).
MS (ESI) m/z : 444 (M + H)⁺.

### Referential Example 542

Ethyl (2E)-4-[{2-chloro-6-[(2,3-dimethoxyphenyl)(hydroxy)methyl]phenyl}(2,4-dimethoxybenzyl)amino]-4-oxobut-2-enoate

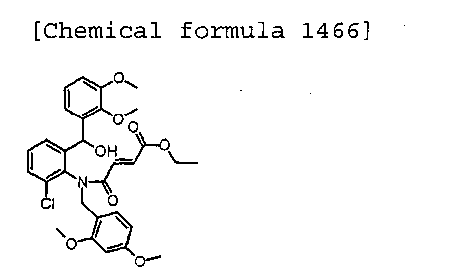

To a dichloromethane solution (21.0 mL) of {3-chloro-2-[(2,4-dimethoxybenzyl)amino]phenyl}(2,3-dimethoxyphenyl)methanol (2.96 g) were added sodium hydrogen carbonate (1.68 g) and a dichloromethane solution (9.0 mL) of monoethyl chlorofumarate (1.30 g) under ice cooling. The reaction mixture was stirred overnight at room temperature. Water was added to the reaction mixture, followed by extraction with dichloromethane. The organic layer was dried over anhydrous sodium sulfate. After filtration, the filtrate was concentrated to give the title compound (4.13 g). The compound thus obtained was provided for the subsequent reaction without purification.

### Referential Example 543

Ethyl [(trans)-9-chloro-1-(2,4-dimethoxybenzyl)-5-(2,3-dimethoxyphenyl)-2-oxo-1,2,3,5-tetrahydro-4,1-benzoxazepin-3-yl] acetate

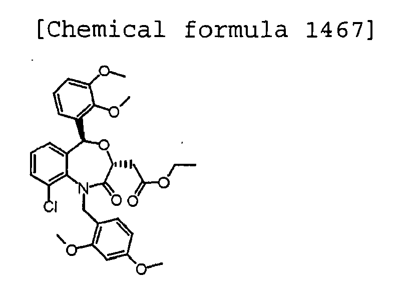

To an ethanol solution (76.0 mL) of ethyl (2E)-4-[{2-chloro-6-[(2,3-dimethoxyphenyl)(hydroxy)methyl]phenyl}(2,4-dimethoxybenzyl)amino]-4-oxobut-2-enoate (4.13 g, crude) was added potassium carbonate (1.38 g) at room temperature. The resulting mixture was stirred for 5 days. After filtering off potassium carbonate, the residue obtained by concentrating the filtrate was dissolved in ethyl acetate, followed by washing with water. The organic layer was dried over anhydrous sodium sulfate. After filtration, the residue obtained by concentrating the filtrate was purified by medium pressure column chromatography (hexane:ethyl acetate = 3:1) to give the title compound (2.91 g).
¹H-NMR (CDCl₃) δ: 1.24 (3H, t, J = 7.0 Hz), 2.78 (1H, dd, J = 16.4, 6.6 Hz), 3.02 (1H, dd, J = 16.4, 7.1 Hz), 3.09 (3H, s), 3.45 (3H, s), 3.74 (3H, s), 3.83 (3H, s), 4.09-4.16 (2H, m), 4.36-4.42 (1H, m), 4.95 (1H, d, J = 14.1 Hz), 5.62 (1H, d, J = 14.1 Hz), 5.85 (1H, s), 6.32-6.38 (2H, m), 6.44-6.49 (1H, m), 6.89 (1H, dd, J = 8.0, 1.7 Hz), 6.98-7.12 (3H, m), 7.37-7.48 (2H, m).
MS (ESI) m/z : 570 (M + H)⁺.

### Referential Example 544

Ethyl [(trans)-9-chloro-5-(2,3-dimethoxyphenyl)-2-oxo-1,2,3,5-tetrahydro-4,1-benzoxazepin-3-yl]acetate

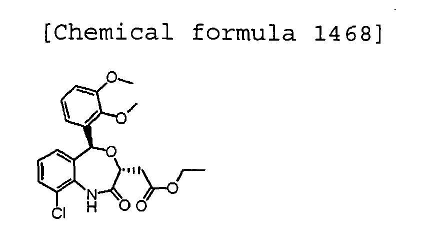

To an acetone solution (80.0 mL) of ethyl [(trans)-9-chloro-1-(2,4-dimethoxybenzyl)-5-(2,3-dimethoxyphenyl)-2-oxo-1,2,3,5-tetrahydro-4,1-benzoxazepin-3-yl]acetate (2.91 g) was added an aqueous solution (20.0 mL) of ceric ammonium nitrate (8.40 g) under ice cooling. The resulting mixture was stirred for one hour. After the reaction mixture was stirred at room temperature for further 4 hours, water and brine were added. The resulting mixture was extracted with ethyl acetate. The organic layer was dried over anhydrous sodium sulfate. After filtration, the filtrate was concentrated and the residue obtained by concentrating the filtrate was purified by medium pressure column chromatography (hexane:ethyl acetate = 4:1) to give the title compound (1.83 g).
¹H-NMR (CDCl₃) δ: 1.22 (3H, t, J = 7.2 Hz), 2.83 (1H, dd, J = 16.4, 6.7 Hz), 3.04 (1H, dd, J = 16.4, 6.7 Hz), 3.62 (3H, s), 3.87 (3H, s), 4.08-4.17 (2H, m), 4.65 (1H, dd, J = 6.7, 6.7 Hz), 6.20 (1H, s), 6.62 (1H, d, J = 7.8 Hz), 6.93-6.98 (1H, m), 7.01 (1H, d, J = 7.8 Hz), 7.10-7.17 (2H, m), 7.36-7.41 (1H, m), 7.98 (1H, br s).
MS (ESI) m/z : 420 (M + H)⁺.

### Referential Example 545

Ethyl [(trans)-9-chloro-5-(2,3-dimethoxyphenyl)-2-thioxo-1,2,3,5-tetrahydro-4,1-benzoxazepin-3-yl]acetate

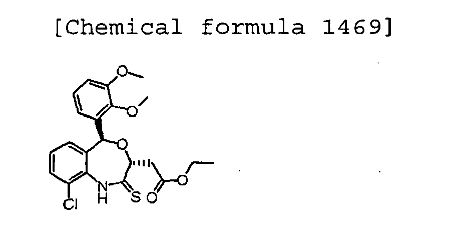

To a tetrahydrofuran solution (47.0 mL) of ethyl [(trans)-9-chloro-5-(2,3-dimethoxyphenyl)-2-oxo-1,2,3,5-tetrahydro-4,1-benzoxazepin-3-yl]acetate (2.14 g) were added sodium hydrogen carbonate (0.94 g) and diphosphorus pentasulfide (2.27 g) under ice cooling. The resulting mixture was stirred at room temperature for 2.5 hours. Under ice cooling, sodium hydrogen carbonate (0.94 g) and diphosphorus pentasulfide (2.27 g) were added, followed by stirring at room temperature for further 4 hours. To the reaction mixture were added water and brine. The resulting mixture was extracted with ethyl acetate. The organic layer was dried over anhydrous sodium sulfate. After filtration, the residue obtained by concentrating the filtrate was purified by medium pressure column chromatography (hexane:ethyl acetate = 3:1) to give the title compound (1.64 g).
¹H-NMR (CDCl₃) δ: 1.25 (3H, t, J = 7.1 Hz), 3.01 (1H, dd, J = 16.5, 7.0 Hz), 3.23 (1H, dd, J = 16.5, 6.2 Hz), 3.60 (3H, s), 3.87 (3H, s), 4.10-4.18 (2H, m), 4.67-4.73 (1H, m), 6.11 (1H, s), 6.58-6.63 (1H, m), 6.93-6.98 (1H, m), 7.09 (1H, t, J = 7.9 Hz), 7.13-7.17 (2H, m), 7.42 (1H, dd, J = 8.0, 1.2 Hz), 9.55 (1H, s).
MS (ESI) m/z : 436 (M + H)⁺.

### Example 720

Ethyl [(trans)-10-chloro-6-(2,3-dimethoxyphenyl)-1-(trifluoromethyl)-4H,6H-[1,2,4]triazolo[4,3-a][4,1]benzoxazepin-4-yl]acetate

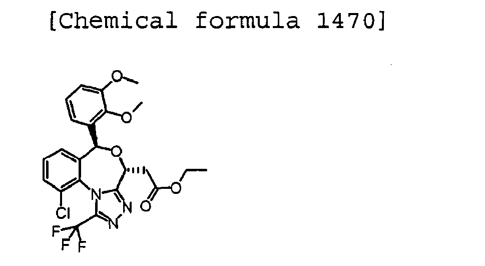

To a tetrahydrofuran solution (8.50 mL) of ethyl [(trans)-9-chloro-5-(2,3-dimethoxyphenyl)-2-thioxo-1,2,3,5-tetrahydro-4,1-benzoxazepin-3-yl]acetate (424.5 mg) was added hydrazine monohydrate (94.5 µl) under ice cooling. The resulting mixture was stirred at room temperature for 35 minutes. To the reaction mixture were added 1,2-dichloroethane (8.50 mL), trifluoroacetic anhydride (0.68 mL) and trifluoroacetic acid (1.50 mL). The resulting mixture was stirred at room temperature for 45 minutes and then at 55°C for 50 minutes. Toluene (8.50 mL) was added and the resulting mixture was heated under reflux for one hour. After the reaction mixture was allowed to cool and concentrated, the residue was neutralized with a saturated aqueous solution of sodium bicarbonate. Water was added, followed by extraction with ethyl acetate. The organic layer was dried over anhydrous sodium sulfate. After filtration, the residue obtained by concentrating the filtrate was purified by medium pressure column chromatography (hexane:ethyl acetate = 4:1) to give the title compound (305.1 mg).
¹H-NMR (CDCl₃) δ: 1.27 (3H, t, J = 7.2 Hz), 3.27 (1H, dd, J = 16.6, 7.6 Hz), 3.43-3.51 (4H, m), 3.84 (3H, s), 4.16-4.24 (2H, m), 4.85 (1H, dd, J = 7.6, 6.2 Hz), 5.39 (1H, s), 6.76 (1H, dd, J = 7.8, 1.2 Hz), 6.93-6.98 (1H, m), 7.14-7.18 (2H, m), 7.36 (1H, t, J = 7.9 Hz), 7.59 (1H, dd, J = 7.9, 1.3 Hz).
MS (ESI) m/z : 512 (M + H)⁺.

### Example 721

[(trans)-10-Chloro-6-(2,3-dimethoxyphenyl)-1-(trifluoromethyl)-4H,6H-[1,2,4]triazolo[4,3-a][4,1]benzoxazepin-4-yl]acetic acid

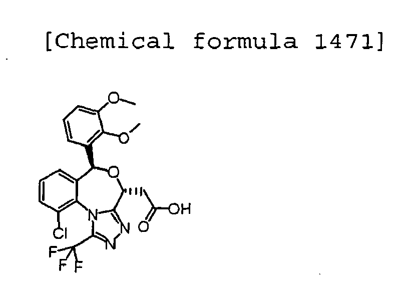

To a dioxane solution (8.0 mL) of ethyl [(trans)-10-chloro-6-(2,3-dimethoxyphenyl)-1-(trifluoromethyl)-4H,6H-[1,2,4]triazolo[4,3-a][4,1]benzoxazepin-4-yl]acetate (305.1 mg) was added concentrated hydrochloric acid (2.0 mL). The resulting mixture was stirred overnight at 60°C. After the reaction mixture was allowed to cool, water was added, followed by extraction with a dichloromethane:methanol (10:1) solvent mixture. The organic layer was dried over anhydrous sodium sulfate. After filtration, the residue obtained by concentrating the filtrate was purified by preparative thin layer chromatography on silica gel (dichloromethane:methanol = 10:1) to give the title compound (127.4 mg).
¹H-NMR (CDCl₃) δ: 3.07-3.17 (1H, m), 3.26-3.35 (1H, m), 3.41 (3H, s), 3.82 (3H, s), 4.78 (1H, t, J = 6.8 Hz), 5.33 (1H, s), 6.70-6.75 (1H, m), 6.87-6.92 (1H, m), 7.11 (1H, t, J = 7.9 Hz), 7.17 (1H, d, J = 7.3 Hz), 7.30 (1H, t, J = 7.9 Hz), 7.50-7.54 (1H, m).
MS (ESI) m/z : 484 (M + H)⁺.
Elemental analysis for: C₂₁H₁₇ClF₃N₃O₅·1.5H₂O
Calculated: C, 49.37; H, 3.95; Cl, 6.94; F, 11.16; N, 8.23.
Found: C, 49.81; H, 3.54; Cl, 7.07; F, 10.80; N, 7.77.

### Referential Example 546

N-[4-Chloro-2-(3-ethyl-2-methoxybenzoyl)phenyl]-2,2-dimethylpropanamide

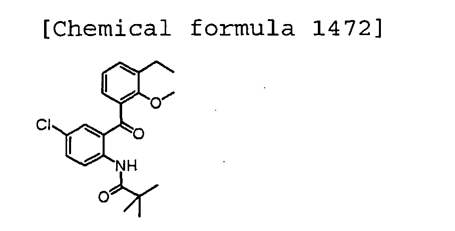

N-(4-Chlorophenyl)-2,2-dimethylpropanamide (1.68 g) was dissolved in tetrahydrofuran (20 mL). In a nitrogen atmosphere, n-butyl lithium (2.6M, a hexane solution) (7.0 mL) was added to the resulting solution at -78°C. The resulting mixture was stirred at -15°C for 2.5 hours. To the reaction mixture was added dropwise a tetrahydrofuran solution (10 mL) of (3-ethyl-2-methoxyphenyl)morpholine-4-yl methanone (2.08 g) at -78°C over 30 minutes. After stirring for further 4.5 hours at -15°C, a saturated aqueous solution of ammonium chloride was added to the reaction mixture. The resulting mixture was extracted with dichloromethane and the extract was washed with saturated brine. The organic layer was dried over anhydrous sodium sulfate. After concentration of the filtrate, the residue thus obtained was purified by silica gel column (hexane:ethyl acetate= from 10:0, 9:1 to 6:1) to give the title compound (1.64 g).
¹H-NMR (CDCl₃) δ: 1.28 (3H, t, J = 7.84 Hz), 1.38 (9H, s), 2.73 (2H, q, J = 7.84 Hz), 3.64 (3H, s), 7.17-7.10 (2H, m), 7.40 (1H, dd, J = 6.99, 2.33 Hz), 7.43 (1H, d, J = 2.45 Hz), 7.51 (1H, dd, J = 9.07, 2.70 Hz), 8.80 (1H, d, J = 9.07 Hz), 11.69 (1H, s).
MS (ESI) m/z : 374 (M + H)⁺.

### Referential Example 547

(2-Amino-5-chlorophenyl)(3-ethyl-2-methoxyphenyl)methanone

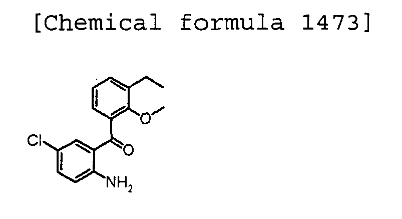

N-[4-Chloro-2-(3-ethyl-2-methoxybenzoyl)phenyl]-2,2-dimethylpropanamide (1.64 g) was dissolved in methanol (20 mL). To the resulting solution were added potassium hydroxide (3.0 g) and water (10 mL). The resulting mixture was stirred at 85°C for 25 hours. After cooling to room temperature, the reaction mixture was diluted with water and extracted with dichloromethane. The extract was washed with saturated brine. The organic layer was dried over anhydrous sodium sulfate. After concentration of the filtrate, the residue thus obtained was purified by silica gel column (hexane:ethyl acetate= from 10:0, 9:1 to 6:1) to give the title compound (1.02 g).
¹H-NMR (CDCl₃) δ: 1.27 (3H, t, J = 7.35 Hz), 2.72 (2H, q, J = 7.52 Hz), 3.66 (3H, s), 6.41-6.35 (2H, m), 6.66 (1H, d, J = 8.58 Hz), 7.07-7.14 (2H, m), 7.20-7.25 (2H, m), 7.33 (1H, dd, J = 7.11, 2.21 Hz).
MS (ESI) m/z : 290 (M + H)⁺.

### Referential Example 548

(2-Amino-5-chlorophenyl)(3-ethyl-2-methoxyphenyl)methanol

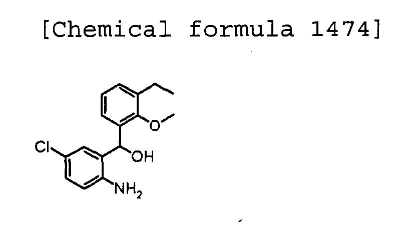

(2-Amino-5-chlorophenyl)(3-ethyl-2-methoxyphenyl)methanone (1.02 g) was dissolved in methanol (20 mL). To the resulting solution was added sodium borohydride (0.40 g). The resulting mixture was stirred at room temperature for 3 hours. After the reaction mixture was concentrated, water was added to the residue. The resulting mixture was extracted with chloroform. The extract was washed with saturated brine. The organic layer was dried over anhydrous sodium sulfate. The filtrate was concentrated to give the title compound (1.1 g).
MS (ESI) m/z : 274 (M - OH)⁺.

### Referential Example 549

{5-Chloro-2-[(2,4-dimethoxybenzyl)amino]phenyl}(3-ethyl-2-methoxyphenyl)methanol

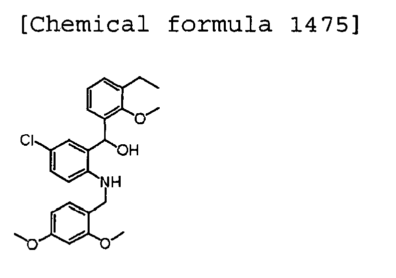

(2-Amino-5-chlorophenyl)(3-ethyl-2-methoxyphenyl)methanol (1.03 g) and 2,4-dimethoxybenzaldehyde (0.70 g) were dissolved in acetic acid (20 mL). The resulting solution was stirred at room temperature for 2 hours. To the reaction mixture was added sodium borohydride (0.20 g) at 0°C and the resulting mixture was stirred at room temperature for 4 hours. The reaction mixture was concentrated. The residue was diluted with dichloromethane, and washed successively with water, a saturated aqueous solution of sodium bicarbonate and saturated brine. The organic layer was dried over anhydrous sodium sulfate. After concentration of the filtrate, the residue thus obtained was purified by silica gel column (hexane:ethyl acetate= from 6:1, 3:1 to 1:1) to give the title compound (1.52 g).
¹H-NMR (CDCl₃) δ: 1.24 (3H, t, J = 7.60 Hz), 2.68 (2H, q, J = 7.60 Hz), 3.70 (3H, s), 3.76 (3H, s), 3.78 (3H, s), 4.23 (2H, s), 6.08 (1H, s), 6.36 (1H, dd, J = 8.33, 2.45 Hz), 6.43 (1H, d, J = 2.21 Hz), 6.58 (1H, d, J = 8.82 Hz), 6.98 (1H, d, J = 8.09 Hz), 7.06-7.11 (4H, m), 7.20 (1H, dd, J = 6.62, 2.70 Hz).
MS (ESI) m/z : 442 (M + H)⁺.

### Referential Example 550

Ethyl (2E)-4-[{4-chloro-2-[(3-ethyl-2-methoxyphenyl)(hydroxy)methyl]phenyl}(2,4-dimethoxybenzyl)amino]-4-oxobut-2-enoate

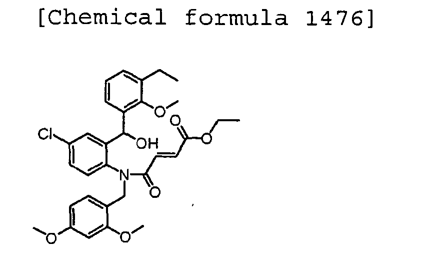

{5-Chloro-2-[(2,4-dimethoxybenzyl)amino]phenyl}(3-ethyl-2-methoxyphenyl)methanol (1.52 g) was dissolved in dichloromethane (10 mL). Sodium hydrogen carbonate (0.58 g) and a methylene chloride solution (10 mL) of monoethyl chlorofumarate (0.84 g) prepared separately were added dropwise to the resulting solution at 0°C. The reaction mixture was stirred overnight at room temperature, diluted with dichloromethane and washed with a saturated aqueous solution of sodium bicarbonate and saturated brine. The organic layer was dried over anhydrous sodium sulfate. The filtrate was concentrated to give the title compound (2.22 g).
MS (ESI) m/z : 568 (M + H)⁺.

### Referential Example 551

Ethyl [(trans)-7-chloro-1-(2,4-dimethoxybenzyl)-5-(3-ethyl-2-methoxyphenyl)-2-oxo-1,2,3,5-tetrahydro-4,1-benzoxazepin-3-yl] acetate

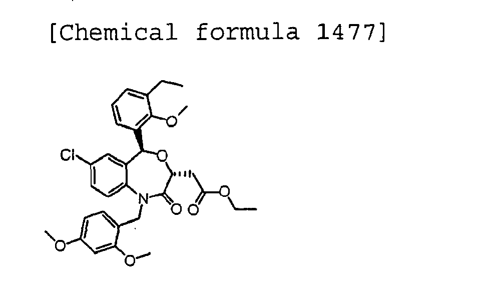

Ethyl (2E)-4-[{4-chloro-2-[(3-ethyl-2-methoxyphenyl)(hydroxy)methyl]phenyl}(2,4-dimethoxybenzyl)amino]-4-oxobut-2-enoate (2.22 g) was dissolved in ethanol (20 mL). To the resulting solution was added potassium carbonate (0.81 g). The resulting mixture was stirred overnight at room temperature. The reaction mixture was concentrated. The residue was diluted with ethyl acetate and washed with water and saturated brine. The organic layer was dried over anhydrous sodium sulfate. The filtrate was concentrated and the residue thus obtained was purified by silica gel column (hexane:ethyl acetate= from 6:1, 3:1 to 1:1) to give the title compound (1.71 g).
MS (ESI) m/z : 568 (M + H)⁺.

### Referential Example 552

Ethyl [(trans)-7-chloro-5-(3-ethyl-2-methoxyphenyl)-2-oxo-1,2,3,5-tetrahydro-4,1-benzoxazepin-3-yl]acetate

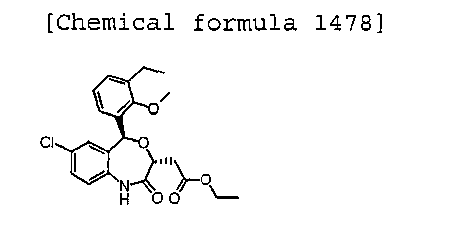

Ethyl [(trans)-7-chloro-1-(2,4-dimethoxybenzyl)-5-(3-ethyl-2-methoxyphenyl)-2-oxo-1,2,3,5-tetrahydro-4,1-benzoxazepin-3-yl]acetate (1.71 g) was dissolved in acetone (50 mL). To the resulting solution was added an aqueous solution (16 mL) of ceric ammonium nitrate (4.95 g) at 0°C. The resulting mixture was stirred at room temperature for 2 hours. To the reaction mixture was added a (1:1) mixture of water and saturated brine. The resulting mixture was extracted with ethyl acetate. The extract was washed with saturated brine. The organic layer was dried over anhydrous sodium sulfate. After concentration of the filtrate, the residue was purified by silica gel column (hexane:ethyl acetate= from 4:1, 2:1 to 1:2) to give the title compound (1.15 g).
MS (ESI) m/z : 418 (M + H)⁺.

### Referential Example 553

Ethyl [(trans)-7-chloro-5-(3-ethyl-2-methoxyphenyl)-2-thioxo-1,2,3,5-tetrahydro-4,1-benzoxazepin-3-yl]acetate

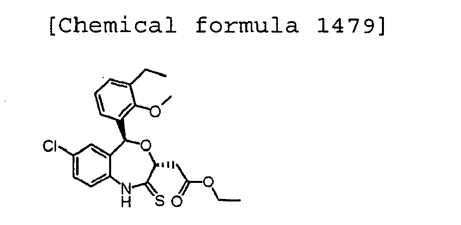

Ethyl [(trans)-7-chloro-5-(3-ethyl-2-methoxyphenyl)-2-oxo-1,2,3,5-tetrahydro-4,1-benzoxazepin-3-yl]acetate (1.15 g) was dissolved in tetrahydrofuran (20 mL). To the resulting solution were added sodium hydrogen carbonate (0.51 g) and diphosphorus pentasulfide (1.22 g). The resulting mixture was stirred at room temperature for 3 hours. The reaction mixture was diluted with ethyl acetate and washed with saturated brine. The organic layer was dried over anhydrous sodium sulfate. The filtrate was concentrated and the residue thus obtained was purified by silica gel column (hexane:ethyl acetate= from 9:1, 6:1 to 3:1) to give the title compound (0.76 g).
¹H-NMR (CDCl₃) δ: 1.24 (3H, t, J = 7.60 Hz), 1.24 (3H, t, J = 7.35 Hz), 2.67 (2H, q, J = 7.52 Hz), 2.94 (1H, dd, J = 16.55, 6.50 Hz), 3.25 (1H, dd, J = 16.42, 6.86 Hz), 3.50 (3H, s), 4.12 (2H, q, J = 7.11 Hz), 4.72 (1H, t, J = 6.50 Hz), 6.15 (1H, s), 6.70 (1H, d, J = 2.45 Hz), 7.06 (1H, d, J = 8.33 Hz), 7.20 (1H, t, J = 7.48 Hz), 7.27-7.29 (1H, m), 7.39-7.32 (2H, m), 9.54 (1H, s).
MS (ESI) m/z : 434 (M + H)⁺.

### Example 722

Ethyl [(4R,6S)-8-chloro-6-(3-ethyl-2-methoxyphenyl)-1-(trifluoromethyl)-4H,6H-[1,2,4]triazolo[4,3-a] [4,1]benzoxazepin-4-yl]acetate

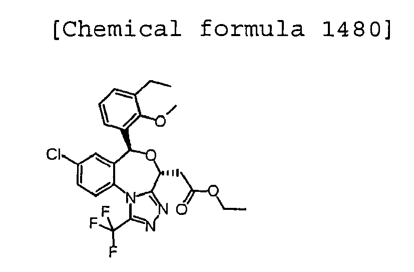

Ethyl [(trans)-7-chloro-5-(3-ethyl-2-methoxyphenyl)-2-thioxo-1,2,3,5-tetrahydro-4,1-benzoxazepin-3-yl]acetate (0.76 g) was dissolved in tetrahydrofuran (20 mL). To the resulting solution was added hydrazine monohydrate (1.0 mL). The resulting mixture was stirred at room temperature for one hour. The reaction mixture was diluted with water and dichloromethane, followed by extraction with dichloromethane three times. The organic layer was dried over anhydrous sodium sulfate. The filtrate was concentrated and the residue thus obtained was dissolved in 1,2-dichloroethane (20 mL). To the resulting solution was added trifluoroacetic anhydride (2.19 mL) at 0°C and the mixture was stirred at room temperature for 2.5 hours. To the reaction mixture were added toluene (5.0 mL) and trifluoroacetic anhydride (1.46 mL). The resulting mixture was stirred at 80°C for 3 hours. After cooling to room temperature, the reaction mixture was neutralized with a saturated aqueous solution of sodium bicarbonate, diluted with ethyl acetate, and washed successively with water and saturated brine. The organic layer was dried over anhydrous sodium sulfate. The filtrate was concentrated and the residue thus obtained was purified by silica gel column (hexane:ethyl acetate= from 8:1, 4:1 to 2:1) to give a racemic mixture of the title compound (0.66 g). The racemic mixture thus obtained was optically resolved by an optically active column (CHIRALPAK-AD; 5 cm × 50 cm) into an isomer A (0.29 g) and a target product, that is, an isomer B (0.29 g).
Flow rate: 50 mL/min
Developing solvent: 10% isopropanol-n-hexane
Retention time: isomer A: 30 min. , isomer B: 42 min.
¹H-NMR (CDCl₃) δ: 1.21 (3H, t, J = 7.48 Hz), 1.28 (3H, t, J = 7.11 Hz), 2.59-2.66 (2H, m), 3.20-3.25 (1H, m), 3.26 (3H, s), 3.49 (2H, dd, J = 16.67, 6.62 Hz), 4.19 (2H, q, J = 6.62 Hz), 4.95 (1H, t, J = 6.74 Hz), 5.58 (1H, s), 6.91 (1H, s), 7.23 3 (1H, t, J = 7.60 Hz), 7.26-7.30 (1H, m), 7.48 (1H, d, J = 7.60 Hz), 7.54 (2H, s).
MS (ESI) m/z : 510 (M + H)⁺.

### Example 723

[(4R,6S)-8-Chloro-6-(3-ethyl-2-methoxyphenyl)-1-(trifluoromethyl)-4H,6H-[1,2,4]triazolo[4,3-a] [4, 1] benzoxazepin-4-yl] acetic acid

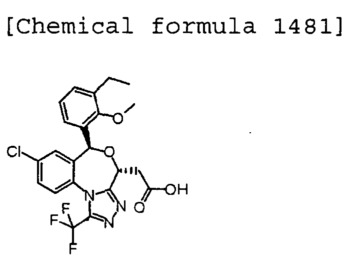

Ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate (0.29 g) was dissolved in 1,4-dioxane (5.0 mL). To the resulting solution was added concentrated hydrochloric acid (1.0 mL). The resulting mixture was stirred at 60°C for 8 days. After the reaction mixture was cooled to room temperature, a 10% aqueous citric acid solution was added thereto. The resulting mixture was extracted with ethyl acetate. The organic layer was washed with saturated brine and dried over anhydrous sodium sulfate. The filtrate was concentrated. The residue obtained by concentration was purified by silica gel column (chloroform:methanol= from 99:1, 9:1 to 7:1), followed by recrystallization from dichloromethane, hexane and isopropanol to give the title compound (193 mg). ¹H-NMR (CDCl₃) δ: 1.21 (3H, t, J = 7.48 Hz), 2.57-2.66 (2H, m), 3.27 (3H, s), 3.30 (1H, dd, J = 17.28, 6.99 Hz), 3.56 (1H, dd, J = 17.16, 6.86 Hz), 4.92 (1H, t, J = 6.74 Hz), 5.58 (1H, s), 6.92 (1H, s), 7.22-7.30 (2H, m), 7.48 (1H, d, J = 7.60 Hz), 7.54 (2H, br s).
MS (ESI) m/z : 482 (M + H)⁺.
Elemental analysis for: C₂₂H₁₉ClF₃N₃O₄
Calculated: C, 54.84; H, 3.97; N, 8.72.
Found: C, 54.95; H, 4.20; N, 8.54.

### Referential Example 554

(2-Amino-5-bromophenyl)(2,3-dimethoxyphenyl)methanone

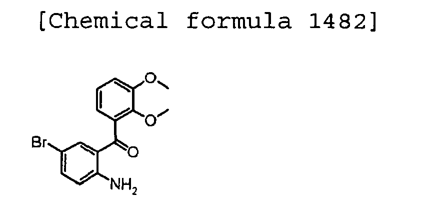

Veratrole (2.0 g) was dissolved in tetrahydrofuran (20 mL). To the resulting solution were added N,N,N',N'-tetramethylethylenediamine (2.18 mL) and n-butyl lithium (2.6 mol/l, a hexane solution) (6.20 mL) were added at - 78°C in a nitrogen atmosphere. The resulting mixture was stirred at -10°C for one hour and then, at room temperature for 30 minutes. To the reaction mixture were added dropwise a tetrahydrofuran solution (30 mL) of 6-bromo-2-methyl-4H-3,1-benzoxazin-4-one (3.47 g) at -78°C. After stirring for one hour while gradually returning to room temperature, the reaction mixture was concentrated. The residue thus obtained was dissolved in ethanol (50 mL). To the resulting solution was added concentrated hydrochloric acid (5.0 mL) diluted with water (5.0 mL). The resulting mixture was stirred overnight at 90°C. After the reaction mixture was concentrated, a saturated aqueous solution of sodium hydrogen carbonate was added to the residue. The resulting mixture was extracted with ethyl acetate and washed with saturated brine. The organic layer was dried over anhydrous sodium sulfate. The filtrate was concentrated and the residue thus obtained was purified by silica gel column (hexane:ethyl acetate= from 6:1, 3:1 to 1:1) to give the title compound (3.26 g).
¹H-NMR (CDCl₃) δ: 3.78 (3H, s), 3.92 (3H, s), 6.39 (2H, br s), 6.60 (1H, d, J = 8.82 Hz), 6.81 (1H, dd, J = 7.60, 1.47, Hz), 7.03 (1H, dd, J = 8.21, 1.59 Hz), 7.12 (1H, t, J = 7.84 Hz), 7.32 (1H, dd, J = 8.82, 2.21 Hz), 7.37 (1H, d, J = 2.45 Hz).
MS (ESI) m/z : 336 (M + H)⁺.

### Referential Example 555

(2-Amino-5-bromophenyl)(2,3-dimethoxyphenyl)methanol

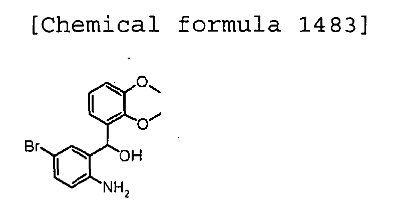

In a similar manner to that employed for the synthesis of (2-amino-5-chlorophenyl)(3-ethyl-2-methoxyphenyl)methanol, the title compound (2.8 g) was obtained from (2-amino-5-bromophenyl)(2,3-dimethoxyphenyl)methanone (3.26 g).
MS (ESI) m/z : 320 (M - OH)⁺.

### Referential Example 556

{5-Bromo-2-[(2,4-dimethoxybenzyl)amino]phenyl}(2,3-dimethoxyphenyl)methanol

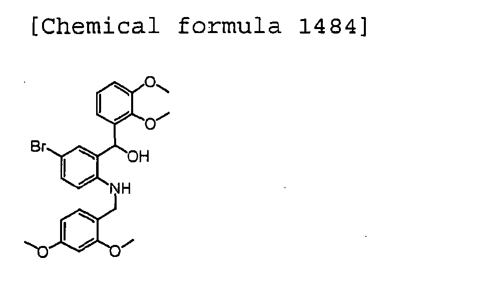

In a similar manner to that employed for the synthesis of {5-chloro-2-[(2,4-dimethoxybenzyl)amino]phenyl}(3-ethyl-2-methoxyphenyl)methanol, the title compound (4.1 g) was obtained from (2-amino-5-bromophenyl)(2,3-dimethoxyphenyl)methanol (2.80 g).
MS (ESI) m/z : 490 (M + H)⁺.

### Referential Example 557

Ethyl (2E)-4-[{4-bromo-2-[(2,3-dimethoxyphenyl)(hydroxy)methyl]phenyl}(2,4-dimethoxybenzyl)amino]-4-oxobut-2-enoate

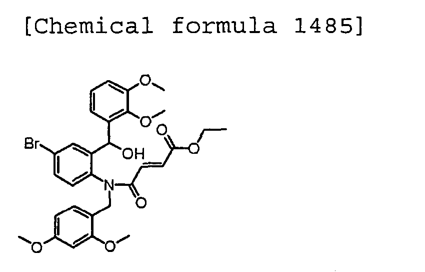

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, the title compound was obtained from {5-bromo-2-[(2,4-dimethoxybenzyl)amino]phenyl}(2,3-dimethoxyphenyl)methanol (4.14 g).
MS (ESI) m/z : 616 (M + H)⁺.

### Referential Example 558

Ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate

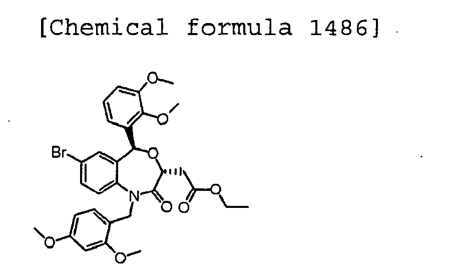

Ethyl (2E)-4-[{4-bromo-2-[(2,3-dimethoxyphenyl)(hydroxy)methyl]phenyl}(2,4-dimethoxybenzyl)amino]-4-oxobut-2-enoate (6.06 g) was dissolved in ethanol (60 mL). To the resulting solution was added potassium carbonate (2.04 g). The resulting mixture was stirred at room temperature for 3.5 hours. The reaction mixture was concentrated. The residue was diluted with ethyl acetate and washed with water and saturated brine. The organic layer was dried over anhydrous sodium sulfate. The filtrate was concentrated and the residue thus obtained was purified by silica gel column (hexane:ethyl acetate= from 6:1, 3:1 to 1:1). The residue thus obtained was dissolved in ethanol (50 mL). To the resulting solution was added 1,8-diazabicyclo[5,4,0]undec-7-ene (1.64 mL). The resulting mixture was heated under reflux overnight. The reaction mixture was concentrated. The residue was diluted with ethyl acetate and washed with water and saturated brine. The organic layer was dried over anhydrous sodium sulfate. After concentration of the filtrate, the residue thus obtained was purified by silica gel column (hexane:ethyl acetate= from 6:1, 3:1 to 1:1) to give the title compound (3.92 g).
¹H-NMR (CDCl₃) δ: 1.25 (3H, t, J = 7.14 Hz), 2.79 (1H, dd, J = 16.49, 5.86 Hz), 3.09 (1H, dd, J = 16.49, 7.69 Hz), 3.19 (3H, s), 3.66 (3H, s), 3.76 (3H, s), 3.85 (3H, s), 4.14 (2H, q, J = 7.33 Hz), 4.48 (1H, dd, J = 7.69, 5.86 Hz), 4.85 (1H, d, J = 15.02 Hz), 5.48 (1H, d, J = 15.02 Hz), 5.96 (1H, s), 6.39-6.42 (2H, m), 6.68 (1H, d, J = 2.20 Hz), 6.93 (1H, t, J = 4.76 Hz), 7.13-7.14 (2H, m), 7.23 (1H, s), 7.42 (1H, dd, J = 8.61, 2.38 Hz).
MS (ESI) m/z : 616 (M + H)⁺.

### Referential Example 559

Ethyl [(trans)-7-bromo-5-(2,3-dimethoxyphenyl)-2-oxo-1,2,3,5-tetrahydro-4,1-benzoxazepin-3-yl]acetate

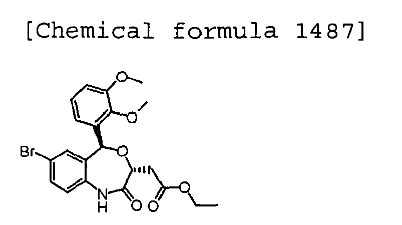

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (3.0 g) was obtained from ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate (3.92 g).
MS (ESI) m/z : 464 (M + H)⁺.

### Referential Example 560

Ethyl [(trans)-7-bromo-5-(2,3-dimethoxyphenyl)-2-thioxo-1,2,3,5-tetrahydro-4,1-benzoxazepin-3-yl]acetate

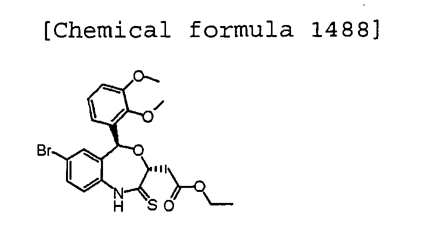

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (2.58 g) was obtained from ethyl [(trans)-7-bromo-5-(2,3-dimethoxyphenyl)-2-oxo-1,2,3,5-tetrahydro-4,1-benzoxazepin-3-yl] acetate (3.00 g).
¹H-NMR (CDCl₃) δ: 1.24(3H, t, J = 7.56 Hz), 2.95 (1H, dd, J = 16.46, 6.46 Hz), 3.26 (1H, dd, J = 16.46, 6.71 Hz), 3.63 (3H, s), 3.89 (3H, s), 4.13 (2H, q, J = 7.07 Hz), 4.71 (1H, t, J = 6.59 Hz), 6.13 (1H, s), 6.81 (1H, d, J = 2.20 Hz), 6.96-7.00 (2H, m), 7.10-7.18 (2H, m), 7.49 (1H, dd, J = 8.54, 2.20 Hz), 9.52 (1H, s).
MS (ESI) m/z : 482 (M + H)⁺.

### Example 724

Ethyl [(4R,6S)-8-bromo-6-(2,3-dimethoxyphenyl)-1-(trifluoromethyl)-4H,6H-[1,2,4]triazolo[4,3-a] [4,1]benzoxazepin-4-yl]acetate

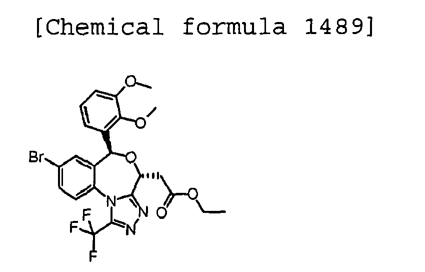

A racemic mixture (0.35 g) obtained from ethyl [(trans)-7-bromo-5-(2,3-dimethoxyphenyl)-2-thioxo-1,2,3,5-tetrahydro-4,1-benzoxazepin-3-yl]acetate (2.58 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50 cm) into an isomer A (0.155 g) and a target product, that is, an isomer B (0.158 g).
Flow rate: 50 mL/min
Developing solvent: 30% isopropanol - n-hexane
Retention time: isomer A: 28 min. , isomer B: 50 min.
¹H-NMR (CDCl₃) δ: 1.28 (3H, t, J = 7.56 Hz), 3.24 (1H, dd, J = 16.83, 7.07 Hz), 3.43 (3H, s), 3.49 (1H, dd, J = 16.58, 6.59 Hz), 3.86 (3H, s), 4.19 (2H, q, J = 7.32 Hz), 4.93 (1H, t, J = 6.83 Hz), 5.54 (1H, s), 6.97-7.00 (2H, m), 7.20-7.18 (2H, m), 7.45 (1H, d, J = 8.29 Hz), 7.68 (1H, dd, J = 8.54, 2.20 Hz).
MS (ESI) m/z : 558 (M + H)⁺.

### Example 725

[(4R,6S)-8-Bromo-6-(2,3-dimethoxyphenyl)-1-(trifluoromethyl)-4H,6H-[1,2,4]triazolo[4,3-a] [4,1]benzoxazepin-4-yl]acetic acid

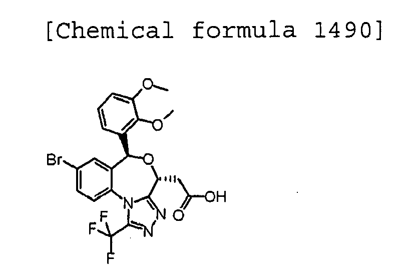

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (123 mg) was obtained from ethyl [(4R,6S)-8-bromo-6-(2,3-dimethoxyphenyl)-1-(trifluoromethyl)-4H,6H-[1,2,4]triazolo[4,3-a][4,1]benzoxazepin-4-yl]acetate (158 mg) .
¹H-NMR (CDCl₃) δ: 3.30 (1H, dd, J = 16.91, 6.62 Hz), 3.43 (3H, s), 3.57 (1H, dd, J = 17.04, 6.74 Hz), 3.86 (3H, s), 4.90 (1H, t, J = 6.74 Hz), 5.54 (1H, s), 6.97-7.01 (2H, m), 7.17-7.22 (2H, m), 7.46 (1H, d, J = 8.33 Hz), 7.69 (1H, dd, J = 8.58, 2.21 Hz).
MS (ESI) m/z : 528 (M + H)⁺.
Elemental analysis for: C₂₁H₁₇BrF₃N₃O₅
Calculated: C, 47.74; H, 3.24; N, 7.95.
Found: C, 47.96; H, 3.34; N, 7.58.

### Referential Example 561

(2-Amino-5-fluorophenyl)(2,3-dimethoxyphenyl)methanone

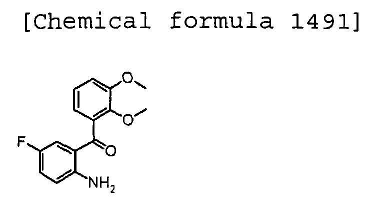

In a similar manner to that employed for the synthesis of (2-amino-5-bromophenyl)(2,3-dimethoxyphenyl)methanone, the title compound (0.61 g) was obtained from Veratrole (0.71 mL) and 6-fluoro-2-methyl-4H-3,1-benzoxazin-4-one (1.0 g).
¹H-NMR (CDCl₃) δ: 3.78 (3H, s), 3.92 (3H, s), 6.23 (2H, br s), 6.66 (1H, dd, J = 8.90, 4.51 Hz), 6.82 (1H, dd, J = 7.56, 1.46 Hz), 6.96 (1H, dd, J = 9.76, 2.93 Hz), 7.01-7.07 (2H, m), 7.12 (1H, t, J = 7.93 Hz).
MS (ESI) m/z : 276 (M + H)⁺.

### Referential Example 562

(2-Amino-5-fluorophenyl)(2,3-dimethoxyphenyl)methanol

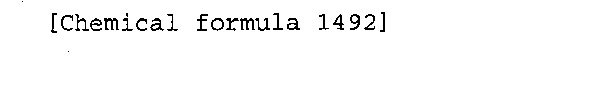

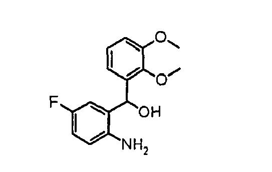

In a similar manner to that employed for the synthesis of (2-amino-5-chlorophenyl)(3-ethyl-2-methoxyphenyl)methanol, the title compound (0.58 g) was obtained from (2-amino-5-fluorophenyl)(2,3-dimethoxyphenyl)methanone (0.61 g).
MS (ESI) m/z : 260 (M - OH)⁺.

### Referential Example 563

{2-[(2,4-Dimethoxybenzyl)amino]-5-fluorophenyl}(2,3-dimethoxyphenyl)methanol

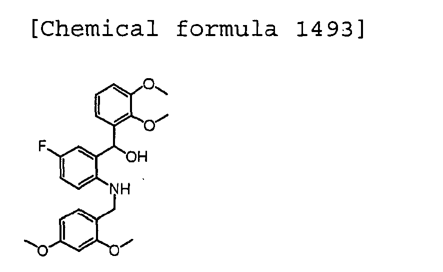

In a similar manner to that employed for the synthesis of {5-chloro-2-[(2,4-dimethoxybenzyl)amino]phenyl}(3-ethyl-2-methoxyphenyl)methanol, the title compound (0.98 g) was obtained from (2-amino-5-fluorophenyl)(2,3-dimethoxyphenyl)methanol (0.58 g).
MS (ESI) m/z : 428 (M + H)⁺.

### Referential Example 564

Ethyl (2E)-4-((2,4-dimethoxybenzyl){2-[(2,3-dimethoxyphenyl)(hydroxy)methyl]-4-fluorophenyl}amino)-4-oxobut-2-enoate

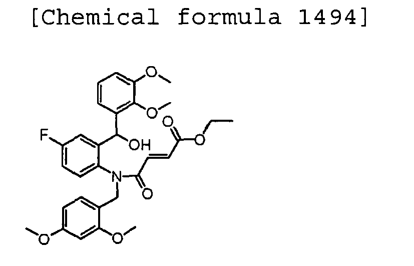

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, the title compound was obtained from {2-[(2,4-dimethoxybenzyl)amino]-5-fluorophenyl}(2,3-dimethoxyphenyl)methanol (0.98 g).
MS (ESI) m/z : 554 (M + H)⁺.

### Referential Example 565

Ethyl [(trans)-1-(2,4-dimethoxybenzyl)-5-(2,3-dimethoxyphenyl)-7-fluoro-2-oxo-1,2,3,5-tetrahydro-4,1-benzoxazepin-3-yl]acetate

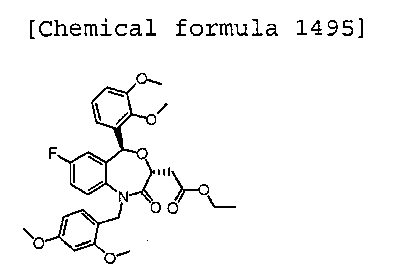

In a similar manner to that employed for the synthesis of ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate, the title compound (1.3 g) was obtained from ethyl (2E)-4-((2,4-dimethoxybenzyl){2-[(2,3-dimethoxyphenyl)(hydroxy)methyl]-4-fluorophenyl}amino)-4-oxobut-2-enoate.
¹H-NMR (CDCl₃) δ: 1.25 (3H, t, J = 7.07 Hz), 2.80 (1H, dd, J = 16.58, 5.85 Hz), 3.09 (1H, dd, J = 16.46, 7.68 Hz), 3.21 (3H, s), 3.66 (3H, s), 3.76 (3H, s), 3.85 (3H, s), 4.14 (2H, q, J = 7.32 Hz), 4.48 (1H, dd, J = 7.56, 6.10 Hz), 4.86 (1H, d, J = 14.88 Hz), 5.48 (1H, d, J = 14.88 Hz), 5.98 (1H, s), 6.28 (1H, dd, J = 9.27, 2.93 Hz), 6.40-6.42 (2H, m), 6.91-6.94 (1H, m), 6.98-7.03 (1H, m), 7.13-7.14 (2H, m), 7.25-7.27 (1H, m), 7.34 (1H, dd, J = 9.02, 4.88 Hz).
MS (ESI) m/z : 554 (M + H)⁺.

### Referential Example 566

Ethyl [(trans)-5-(2,3-dimethoxyphenyl)-7-fluoro-2-oxo-1,2,3,5-tetrahydro-4,1-benzoxazepin-3-yl]acetate

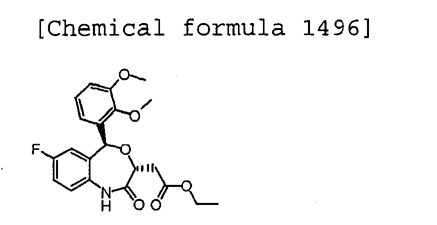

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.8 g) was obtained from ethyl [(trans)-1-(2,4-dimethoxybenzyl)-5-(2,3-dimethoxyphenyl)-7-fluoro-2-oxo-1,2,3,5-tetrahydro-4,1-benzoxazepin-3-yl]acetate (1.30 g).
¹H-NMR (CDCl₃) δ: 1.23 (3H, t, J = 7.32 Hz), 2.79 (1H, dd, J = 16.34, 6.34 Hz), 3.04 (1H, dd, J = 16.46, 6.95 Hz), 3.64 (3H, s), 3.75 (1H, s), 3.88 (3H, s), 4.12 (2H, q, J = 6.91 Hz), 4.63 (1H, t, J = 6.71 Hz), 6.21 (1H, s), 6.41 (1H, d, J = 9.02 Hz), 6.97 (1H, t, J = 3.90 Hz), 7.01-7.03 (2H, m), 7.18-7.10 (2H, m), 7.58 (1H, s).
MS (ESI) m/z : 404 (M + H)⁺.

### Referential Example 567

Ethyl [(trans)-5-(2,3-dimethoxyphenyl)-7-fluoro-2-thioxo-1,2,3,5-tetrahydro-4,1-benzoxazepin-3-yl]acetate

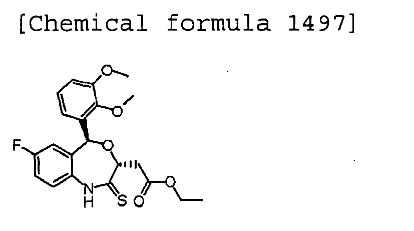

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.66 g) was obtained from ethyl [(trans)-5-(2,3-dimethoxyphenyl)-7-fluoro-2-oxo-1,2,3,5-tetrahydro-4,1-benzoxazepin-3-yl] acetate (0.80 g) .
¹H-NMR (CDCl₃) δ: 1.24 (3H, t, J = 7.32 Hz), 2.97 (1H, dd, J = 16.48, 6.47 Hz), 3.26 (1H, dd, J = 16.36, 6.84 Hz), 3.63 (3H, s), 3.88 (3H, s), 4.13 (2H, q, J = 6.59 Hz), 4.70 (1H, t, J = 6.59 Hz), 6.12 (1H, s), 6.40 (1H, dd, J = 9.03, 2.69 Hz), 6.97 (1H, dd, J = 7.57, 2.20 Hz), 7.03-7.18 (4H, m), 9.57 (1H, s).
MS (ESI) m/z : 420 (M + H)⁺.

### Example 726

Ethyl [(4R,6S)-6-(2,3-dimethoxyphenyl)-8-fluoro-1-(trifluoromethyl)-4H,6H-[1,2,4]triazolo[4,3-a] [4,1]benzoxazepin-4-yl]acetate

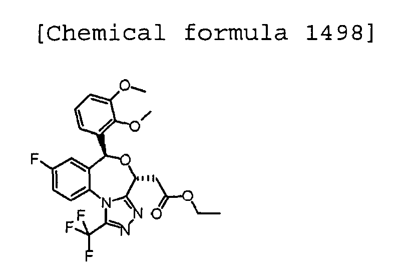

A racemic mixture obtained from ethyl [(trans)-5-(2,3-dimethoxyphenyl)-7-fluoro-2-thioxo-1,2,3,5-tetrahydro-4,1-benzoxazepin-3-yl]acetate (0.66 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50 cm) into an isomer A (0.27 g) and a target product, an isomer B (0.28 g).
Flow rate: 50 mL/min
Developing solvent: 30% isopropanol- n-hexane
Retention time: isomer A: 20 min., isomer B: 50 min.
¹H-NMR (CDCl₃) δ: 1.28 (3H, t, J = 7.11 Hz), 3.24 (1H, dd, J = 16.67, 7.11 Hz), 3.44 (3H, s), 3.50 (1H, dd, J = 16.67, 6.62 Hz), 3.85 (3H, s), 4.19 (2H, q, J = 7.11 Hz), 4.93 (1H, t, J = 6.86 Hz), 5.55 (1H, s), 6.59 (1H, dd, J = 8.70, 2.82 Hz), 6.97 (1H, dd, J = 7.23, 2.57 Hz), 7.16-7.24 (3H, m), 7.58 (1H, dd, J = 8.58, 4.17 Hz).
MS (ESI) m/z : 496 (M + H)⁺.

### Example 727

[(4R,6S)-6-(2,3-Dimethoxyphenyl)-8-fluoro-1-(trifluoromethyl)-4H,6H-[1,2,4]triazolo[4,3-a][4,1]benzoxazepin-4-yl]acetic acid

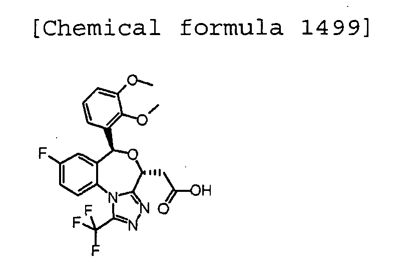

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (153 mg) was obtained from ethyl [(4R,6S)-6-(2,3-dimethoxyphenyl)-8-fluoro-1-(trifluoromethyl)-4H,6H-[1,2,4]triazolo[4,3-a][4,1]benzoxazepin-4-yl]acetate (0.28 g). ¹H-NMR (CDCl₃) δ: 3.30 (1H, dd, J = 17.16, 6.62 Hz), 3.44 (3H, s), 3.57 (1H, dd, J = 17.28, 6.99 Hz), 3.85 (3H, s), 4.90 (1H, t, J = 6.74 Hz), 5.55 (1H, s), 6.60 (1H, dd, J = 8.58, 2.70 Hz), 6.96-7.00 (1H, m), 7.16-7.25 (3H, m), 7.58 (1H, dd, J = 8.82, 4.66 Hz).
MS (ESI) m/z : 468 (M + H)⁺.
Elemental analysis for: C₂₁H₁₇F₄N₃O₅·0.25H₂O·0.1 n-hexane Calculated: C, 53.99; H, 3.96; N, 8.75.
Found: C, 54.28; H, 3.77; N, 8.56.

### Referential Example 568

(2-Amino-5-methylphenyl)(2,3-dimethoxyphenyl)methanone

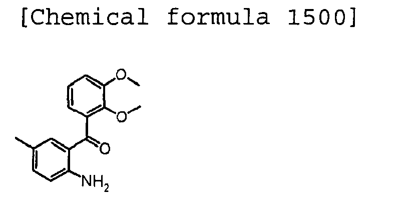

In a similar manner to that employed for the synthesis of (2-amino-5-bromophenyl)(2,3-dimethoxyphenyl)methanone, the title compound (1.16 g) was obtained from Veratrole (1.01 mL, 7.96 mmol) and 2,6-dimethyl-4H-3,1-benzoxazin-4-one (1.39 g).
¹H-NMR (CDCl₃) δ: 2.11 (3H, s), 3.77 (3H, s), 3.92 (3H, s), 6.22 (2H, br s), 6.63 (1H, d, J = 8.33 Hz), 6.82 (1H, dd, J = 7.60, 1.47 Hz), 6.99-7.13 (4H, m).
MS (ESI) m/z : 272 (M + H)⁺.

### Referential Example 569

(2-Amino-5-methylphenyl)(2,3-dimethoxyphenyl)methanol

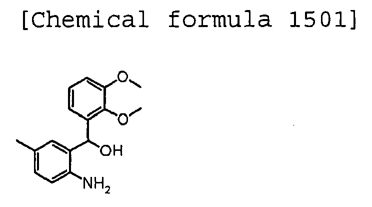

In a similar manner to that employed for the synthesis of (2-amino-5-chlorophenyl)(3-ethyl-2-methoxyphenyl)methanol, the title compound (1.44 g) was obtained from (2-amino-5-methylphenyl)(2,3-dimethoxyphenyl)methanone (1.16 g).
MS (ESI) m/z : 256 (M - OH)⁺.

### Referential Example 570

{2-[(2,4-Dimethoxybenzyl)amino]-5-methylphenyl}(2,3-dimethoxyphenyl)methanol

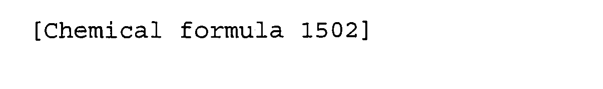

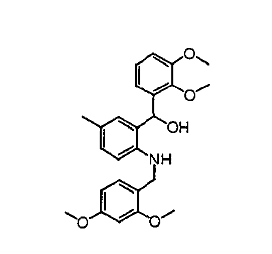

In a similar manner to that employed for the synthesis of {5-chloro-2-[(2,4-dimethoxybenzyl)amino]phenyl}(3-ethyl-2-methoxyphenyl)methanol, the title compound (1.74 g) was obtained from (2-amino-5-methylphenyl)(2,3-dimethoxyphenyl)methanol (1.44 g).
¹H-NMR (CDCl₃) δ: 2.18 (3H, s), 3.75 (3H, s), 3.78 (3H, s), 3.78 (3H, s), 3.88 (3H, s), 4.25 (2H, s), 4.61 (1H, d, J = 5.88 Hz), 6.08 (1H, s), 6.38 (1H, dd, J = 8.21, 2.33 Hz), 6.44 (1H, d, J = 2.45 Hz), 6.62 (1H, d, J = 8.09 Hz), 6.84 (1H, d, J = 1.96 Hz), 6.87-6.90 (2H, m), 6.93-6.96 (1H, m), 7.01-7.10 (2H, m).
MS (ESI) m/z : 424 (M + H)⁺.

### Referential Example 571

Ethyl (2E)-4-((2,4-dimethoxybenzyl){2-[(2,3-dimethoxyphenyl)(hydroxy)methyl]-4-methylphenyl}amino)-4-oxobut-2-enoate

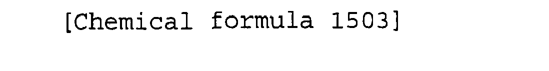

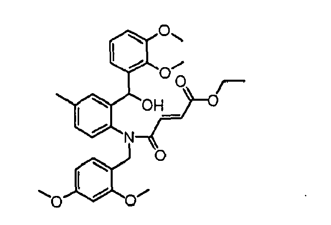

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, the title compound was obtained from {2-[(2,4-dimethoxybenzyl)amino]-5-methylphenyl}(2,3-dimethoxyphenyl)methanol (1.74 g)
MS (ESI) m/z : 550 (M + H)⁺.

### Referential Example 572

Ethyl [(trans)-1-(2,4-dimethoxybenzyl)-5-(2,3-dimethoxyphenyl)-7-methyl-2-oxo-1,2,3,5-tetrahydro-4,1-benzoxazepin-3-yl]acetate

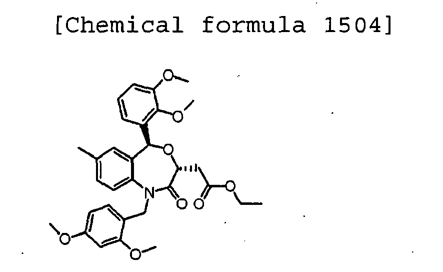

In a similar manner to that employed for the synthesis of ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate, the title compound (1.62 g) was obtained from ethyl (2E)-4-((2,4-dimethoxybenzyl){2-[(2,3-dimethoxyphenyl)(hydroxy)methyl]-4-methylphenyl}amino)-4-oxobut-2-enoate.
¹H-NMR (CDCl₃) δ: 1.25 (3H, t, J = 7.20 Hz), 2.14 (3H, s), 2.79 (1H, dd, J = 16.36, 6.10 Hz), 3.08 (1H, dd, J = 16.48, 7.45 Hz), 3.19 (3H, s), 3.68 (3H, s), 3.75 (3H, s), 3.85 (3H, s), 4.13 (2H, q, J = 7.45 Hz), 4.50 (1H, t, J = 6.84 Hz), 4.90 (1H, d, J = 15.14 Hz), 5.46 (1H, d, J = 15.14 Hz), 6.02 (1H, s), 6.34 (1H, s), 6.39-6.41 (2H, m), 6.91 (1H, d, J = 7.81 Hz), 7.08-7.11 (1H, m), 7.13-7.18 (2H, m), 7.22-7.25 (2H, m).
MS (ESI) m/z : 550 (M + H)⁺.

### Referential Example 573

Ethyl [(trans)-5-(2,3-dimethoxyphenyl)-7-methyl-2-oxo-1,2,3,5-tetrahydro-4,1-benzoxazepin-3-yl]acetate

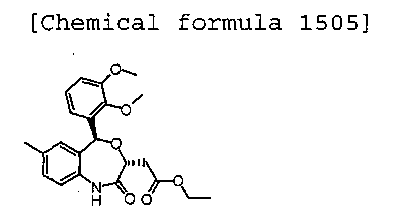

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.86 g) was obtained from ethyl [(trans)-1-(2,4-dimethoxybenzyl)-5-(2,3-dimethoxyphenyl)-7-methyl-2-oxo-1,2,3,5-tetrahydro-4,1-benzoxazepin-3-yl]acetate (1.62 g).
¹H-NMR (CDCl₃) δ: 1.22 (3H, t, J = 7.68 Hz), 2.18 (3H, s), 2.78 (1H, dd, J = 16.34, 6.59 Hz), 3.02 (1H, dd, J = 16.46, 6.71 Hz), 3.63 (3H, s), 3.88 (3H, s), 4.10 (2H, q, J = 7.62 Hz), 4.62 (1H, t, J = 6.59 Hz), 6.24 (1H, s), 6.48 (1H, s), 6.91 (1H, d, J = 7.80 Hz), 6.95 (1H, d, J = 8.05 Hz), 7.08-7.16 (3H, m), 7.58 (1H, s).
MS (ESI) m/z : 400 (M + H)⁺.

### Referential Example 574

Ethyl [(trans)-5-(2,3-dimethoxyphenyl)-7-methyl-2-thioxo-1,2,3,5-tetrahydro-4,1-benzoxazepin-3-yl]acetate

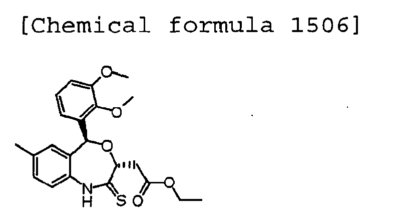

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.68 g) was obtained from ethyl [(trans)-5-(2,3-dimethoxyphenyl)-7-methyl-2-oxo-1,2,3,5-tetrahydro-4,1-benzoxazepin-3-yl]acetate (0.86 g).
¹H-NMR (CDCl₃) δ: 1.24 (3H, t, J = 7.08 Hz), 2.19 (3H, s), 2.96 (1H, dd, J = 16.48, 6.71 Hz), 3.25 (1H, dd, J = 16.36, 6.59 Hz), 3.61 (3H, s), 3.88 (3H, s), 4.13 (2H, q, J = 7.08 Hz), 4.71 (1H, t, J = 6.59 Hz), 6.14 (1H, s), 6.46 (1H, s), 6.94-7.00 (2H, m), 7.13-7.16 (3H, m), 9.61 (1H, s).
MS (ESI) m/z : 416 (M + H)⁺.

### Example 728

Ethyl [(4R,6S)-6-(2,3-dimethoxyphenyl)-8-methyl-1-(trifluoromethyl)-4H,6H-[1,2,4]triazolo[4,3-a][4,1]benzoxazepin-4-yl]acetate

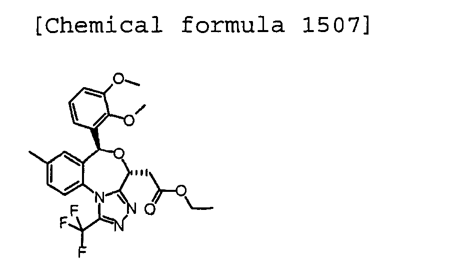

A racemic mixture obtained from ethyl [(trans)-5-(2,3-dimethoxyphenyl)-7-methyl-2-thioxo-1,2,3,5-tetrahydro-4,1-benzoxazepin-3-yl]acetate (0.68 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50 cm) into an isomer A (0.30 g) and a target product, an isomer B (0.27 g).
Flow rate: 50 mL/min
Developing solvent: 30% isopropanol - n-hexane
Retention time: isomer A: 25 min., isomer B: 55 min.
¹H-NMR (CDCl₃) δ: 1.28 (3H, t, J = 7.11 Hz), 2.29 (3H, s), 3.24 (1H, dd, J = 16.67, 7.11 Hz), 3.40 (3H, s), 3.47 (1H, dd, J = 16.67, 6.37 Hz), 3.85 (3H, s), 4.15-4.21 (2H, m), 4.92 (1H, t, J = 6.74 Hz), 5.58 (1H, s), 6.65 (1H, s), 6.96 (1H, dd, J = 7.97, 1.84 Hz), 7.17 (1H, t, J = 7.97 Hz), 7.22 (1H, dd, J = 7.84, 1.23 Hz), 7.32 (1H, d, J = 8.33 Hz), 7.45 (1H, d, J = 8.09 Hz).
MS (ESI) m/z : 492 (M + H)⁺.

### Example 729

[(4R,6S)-6-(2,3-Dimethoxyphenyl)-8-methyl-1-(trifluoromethyl)-4H,6H-[1,2,4]triazolo[4,3-a][4,1]benzoxazepin-4-yl]acetic acid

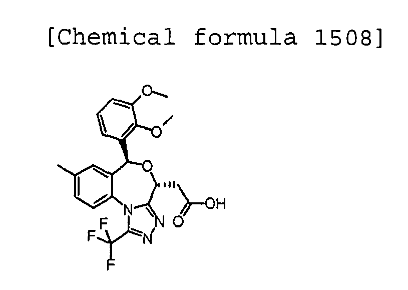

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (190 mg) was obtained from ethyl [(4R,6S)-6-(2,3-dimethoxyphenyl)-8-methyl-1-(trifluoromethyl)-4H,6H-[1,2,4]triazolo[4,3-a][4,1]benzoxazepin-4-yl]acetate (277 mg).
¹H-NMR (CDCl₃) δ: 2.29 (3H, s), 3.30 (1H, dd, J = 16.91, 6.86 Hz), 3.40 (3H, s), 3.55 (1H, dd, J = 16.91, 6.62 Hz), 3.85 (3H, s), 4.88 (1H, t, J = 6.74 Hz), 5.58 (1H, s), 6.66 (1H, s), 6.96 (1H, dd, J = 7.97, 1.59 Hz), 7.16-7.25 (2H, m), 7.33 (1H, d, J = 8.09 Hz), 7.45 (1H, d, J = 8.33 Hz). MS (ESI) m/z : 464 (M + H)⁺.
Elemental analysis for: C₂₂H₂₀F₃N₃O₅·0.25H₂O·0.2 n-hexane Calculated: C, 57.44; H, 4.84; N, 8.66.
Found: C, 57.46; H, 4.82; N, 8.81.

### Example 730

Ethyl [(trans)-8-cyclopropyl-6-(2,3-dimethoxyphenyl)-1-(trifluoromethyl)-4H,6H-[1,2,4]triazolo[4,3-a][4,1]benzoxazepin-4-yl]acetate

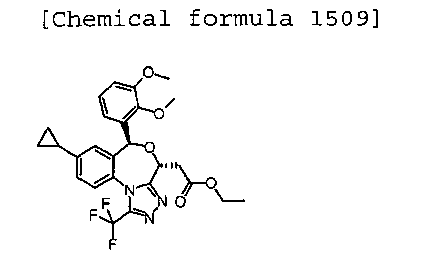

In a similar manner to that employed for the synthesis of ethyl [(4R,6S)-6-(2,3-dimethoxyphenyl)-8-phenyl-1-(trifluoromethyl)-4H,6H-[1,2,4]triazolo[4,3-a][4,1]benzoxazepin-4-yl]acetate, the title compound (31 mg) was obtained from ethyl [(trans)-8-bromo-6-(2,3-dimethoxyphenyl)-1-(trifluoromethyl)-4H,6H-[1,2,4]triazolo[4,3-a][4,1]benzoxazepin-4-yl]acetate (100 mg).
¹H-NMR (CDCl₃) δ: 0.52-0.64 (2H, m), 0.94-0.97 (2H, m), 1.28 (3H, t, J = 7.11 Hz), 1.76-1.83 (1H, m), 3.23 (1H, dd, J = 16.55, 7.23 Hz), 3.39 (3H, s), 3.44-3.50 (2H, m), 3.85 (3H, s), 4.16-4.21 (3H, m), 4.92 (1H, t, J = 6.86 Hz), 5.59 (1H, s), 6.58 (1H, d, J = 1.96 Hz), 6.96 (1H, dd, J = 7.97, 1.84 Hz), 7.12 (1H, dd, J = 8.33, 1.96 Hz), 7.15-7.24 (2H, m), 7.43 (1H, d, J = 8.09 Hz).
MS (ESI) m/z : 518 (M + H)⁺.

### Example 731

[(trans)-8-Cyclopropyl-6-(2,3-dimethoxyphenyl)-1-(trifluoromethyl)-4H,6H-[1,2,4]triazolo[4,3-a][4,1]benzoxazepin-4-yl]acetic acid

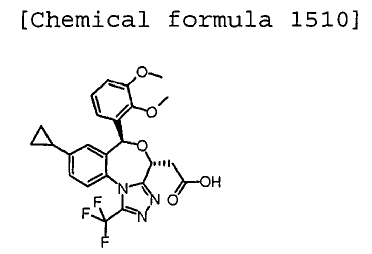

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (25 mg) was obtained from ethyl [(4R,6S)-8-cyclopropyl-6-(2,3-dimethoxyphenyl)-1-(trifluoromethyl)-4H,6H-[1,2,4]triazolo[4,3-a][4,1]benzoxazepin-4-yl]acetate (30 mg).
¹H-NMR (CDCl₃) δ: 0.54-0.63 (2H, m), 0.94-0.97 (2H, m), 1.76-1.83 (1H, m), 3.27 (1H, dd, J = 16.85, 6.35 Hz), 3.39 (3H, s), 3.53 (1H, dd, J = 17.09, 6.84 Hz), 3.85 (3H, s), 4.88 (1H, t, J = 6.84 Hz), 5.58 (1H, s), 6.59 (1H, s), 6.96 (1H, d, J = 8.06 Hz), 7.12 (1H, d, J = 8.54 Hz), 7.16-7.24 (2H, m), 7.43 (1H, d, J = 8.30 Hz).
MS (ESI) m/z : 490 (M + H)⁺.
Elemental analysis for: C₂₄H₂₂F₃N₃O₅·0.5H₂O·0.2 n-hexane Calculated: C, 57.72; H, 4.80; N, 8.14.
Found: C, 57.51; H, 4.69; N, 8.13.

### Referential Example 575

tert-Butyl (4-chloro-3-methylphenyl)carbamate

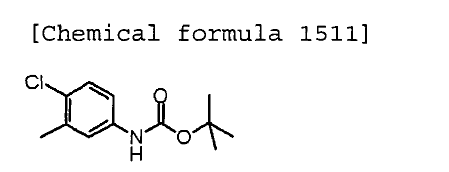

4-Chloro-3-methylphenylamine (2.0 g) was dissolved in tert-butanol (30 mL). To the resulting solution was added di-tert-butyl dicarbonate (3.39 g). The resulting mixture was heated under reflux overnight. The reaction mixture was concentrated and the residue thus obtained was purified by silica gel column (hexane:ethyl acetate= from 10:0, 9:1 to 6:1) to give the title compound (3.7 g).
¹H-NMR (CDCl₃) δ: 1.51 (9H, s), 2.34 (3H, s), 6.41 (1H, br s), 7.08 (1H, dd, J = 8.58, 2.45 Hz), 7.22 (1H, d, J = 8.82 Hz), 7.31 (1H, s).

### Referential Example 576

tert-Butyl {4-chloro-2-[(2,3-dimethoxyphenyl)(hydroxy)methyl]-5-methylphenyl}carbamate

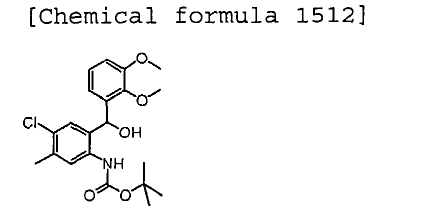

In a similar manner to that employed for the synthesis of tert-butyl {4-chloro-2-[(2,3-dichlorophenyl)(hydroxy)methyl]phenyl}carbamate, the title compound (1.89 g) was obtained from tert-butyl (4-chloro-3-methylphenyl)carbamate (1.50 g) and 2,3-dimethoxybenzaldehyde (1.08 g).
¹H-NMR (CDCl₃) δ: 1.49 (9H, s), 2.33 (3H, s), 3.37 (1H, d, J = 4.90 Hz), 3.82 (3H, s), 3.89 (3H, d, J = 5.64 Hz), 6.03 (1H, d, J = 4.90 Hz), 6.87-6.92 (2H, m), 7.04-7.09 (2H, m), 7.82 (1H, s), 8.08 (1H, s).
MS (ESI) m/z : 430 (M + Na)⁺.

### Referential Example 577

(2-Amino-5-chloro-4-methylphenyl)(2,3-dimethoxyphenyl)methanol

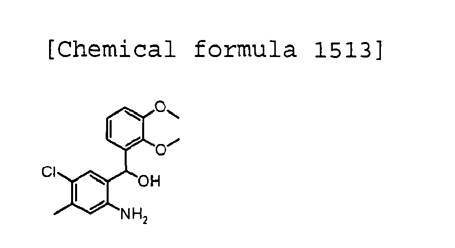

tert-Butyl {4-chloro-2-[(2,3-dimethoxyphenyl)(hydroxy)methyl]-5-methylphenyl}carbamate (1.60 g) was dissolved in methanol (30 mL). To the resulting solution were added a 1N aqueous sodium hydroxide solution (5 mL) and a 2N sodium hydroxide solution (10 mL). The resulting mixture was stirred overnight at 50°C. The reaction mixture was distilled under reduced pressure to remove the solvent. The residue was diluted with dichloromethane and washed with saturated brine. The organic layer was dried over anhydrous sodium sulfate. The filtrate was concentrated to give the title compound (1.06 g).
MS (ESI) m/z : 290 (M - OH)⁺.

### Referential Example 578

{5-Chloro-2-[(2,4-dimethoxybenzyl)amino]-4-methylphenyl} (2,3-dimethoxyphenyl)methanol

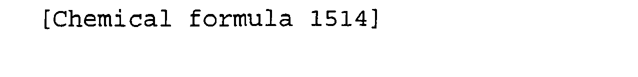

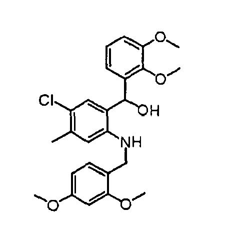

In a similar manner to that employed for the synthesis of {5-chloro-2-[(2,4-dimethoxybenzyl)amino]phenyl}(3-ethyl-2-methoxyphenyl)methanol, the title compound (1.28 g) was obtained from (2-amino-5-chloro-4-methylphenyl)(2,3-dimethoxyphenyl)methanol (1.14 g).
¹H-NMR (CDCl₃) δ: 2.26 (3H, s), 3.08 (1H, s), 3.76 (3H, s), 3.79 (3H, s), 3.79 (3H, s), 3.88 (3H, s), 4.24 (2H, br s), 5.22 (1H, br s), 5.99 (1H, s), 6.40 (1H, dd, J = 8.33, 2.45 Hz), 6.45 (1H, d, J = 2.21 Hz), 6.55 (1H, s), 6.84 (1H, dd, J = 7.84, 1.47 Hz), 6.90 (1H, dd, J = 8.09, 1.47 Hz), 6.92 (1H, s), 7.04 (1H, t, J = 7.97 Hz), 7.08 (1H, d, J = 8.09 Hz).
MS (ESI) m/z : 458 (M + H)⁺.

### Referential Example 579

Ethyl (2E)-4-[{4-chloro-2-[(2,3-dimethoxyphenyl)(hydroxy)methyl]-5-methylphenyl}(2,4-dimethoxybenzyl)amino]-4-oxobut-2-enoate

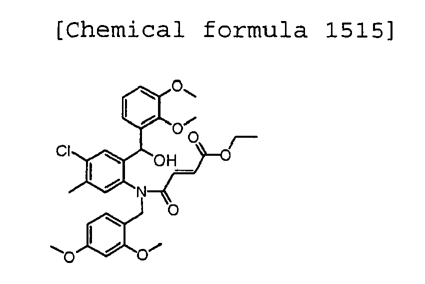

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, the title compound was obtained from {5-chloro-2-[(2,4-dimethoxybenzyl)amino]-4-methylphenyl}(2,3-dimethoxyphenyl)methanol (1.28 g).
MS (ESI) m/z : 584 (M + H)⁺.

### Referential Example 580

Ethyl [(trans)-7-chloro-1-(2,4-dimethoxybenzyl)-5-(2,3-dimethoxyphenyl)-8-methyl-2-oxo-1,2,3,5-tetrahydro-4,1-benzoxazepin-3-yl]acetate

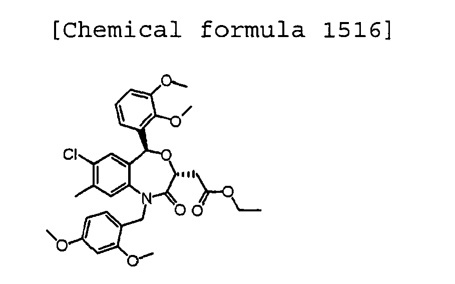

In a similar manner to that employed for the synthesis of ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate, the title compound (1.63 g) was obtained from ethyl (2E)-4-[{4-chloro-2-[(2,3-dimethoxyphenyl)(hydroxy)methyl]-5-methylphenyl}(2,4-dimethoxybenzyl)amino]-4-oxobut-2-enoate. ¹H-NMR (CDCl₃) δ: 1.25 (3H, t, J = 6.86 Hz), 2.34 (3H, s), 2.78 (1H, dd, J = 16.42, 5.88 Hz), 3.09 (1H, dd, J = 16.55, 7.72 Hz), 3.21 (3H, s), 3.68 (3H, s), 3.76 (3H, s), 3.85 (3H, s), 4.12 (2H, q, J = 7.35 Hz), 4.49 (1H, dd, J = 7.84, 5.88 Hz), 4.86 (1H, d, J = 14.95 Hz), 5.47 (1H, d, J = 14.95 Hz), 5.96 (1H, s), 6.40-6.43 (2H, m), 6.51 (1H, s), 6.92 (1H, t, J = 4.90 Hz), 7.13-7.14 (2H, m), 7.25-7.26 (2H, m).
MS (ESI) m/z : 584 (M + H)⁺.

### Referential Example 581

Ethyl [(trans)-7-chloro-5-(2,3-dimethoxyphenyl)-8-methyl-2-oxo-1,2,3,5-tetrahydro-4,1-benzoxazepin-3-yl]acetate

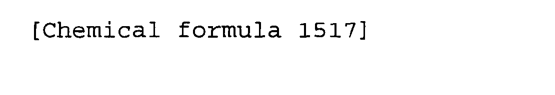

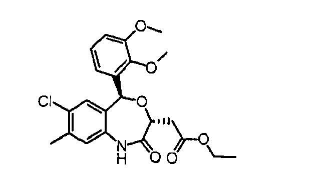

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.65 g) was obtained from ethyl [(trans)-7-chloro-1-(2,4-dimethoxybenzyl)-5-(2,3-dimethoxyphenyl)-8-methyl-2-oxo-1,2,3,5-tetrahydro-4,1-benzoxazepin-3-yl]acetate (1.63 g).
¹H-NMR (CDCl₃) δ: 1.26 (3H, t, J = 6.59 Hz), 2.34 (3H, s), 2.77 (1H, dd, J = 16.36, 6.35 Hz), 3.02 (1H, dd, J = 16.36, 6.84 Hz), 3.65 (3H, s), 3.88 (3H, s), 4.12 (2H, q, J = 7.32 Hz), 4.63 (1H, t, J = 6.71 Hz), 6.19 (1H, s), 6.65 (1H, s), 6.90 (1H, s), 6.96 (1H, dd, J = 8.18, 1.59 Hz), 7.07 (1H, dd, J = 7.81, 1.46 Hz), 7.14 (1H, t, J = 7.93 Hz), 7.57 (1H, s).
MS (ESI) m/z : 434 (M + H)⁺.

### Referential Example 582

Ethyl [(trans)-7-chloro-5-(2,3-dimethoxyphenyl)-8-methyl-2-thioxo-1,2,3,5-tetrahydro-4,1-benzoxazepin-3-yl]acetate

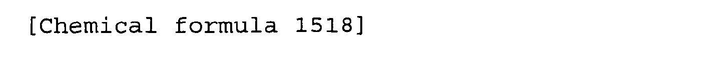

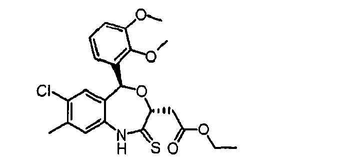

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.70 g) was obtained from ethyl [(trans)-7-chloro-5-(2,3-dimethoxyphenyl)-8-methyl-2-oxo-1,2,3,5-tetrahydro-4,1-benzoxazepin-3-yl]acetate (0.65 g).
¹H-NMR (CDCl₃) δ: 1,2,4 (3H, t, J = 7.32 Hz), 2.36 (3H, s), 2.94 (1H, dd, J = 16.48, 6.47 Hz), 3.25 (1H, dd, J = 16.36, 6.59 Hz), 3.62 (3H, s), 3.88 (3H, s), 4.13 (2H, q, J = 7.08 Hz), 4.70 (1H, t, J = 6.59 Hz), 6.10 (1H, s), 6.63 (1H, s), 6.95-6.98 (2H, m), 7.10-7.18 (2H, m), 9.48 (1H, s).
MS (ESI) m/z : 450 (M + H)⁺.

### Example 732

Ethyl [(4R,6S)-8-chloro-6-(2,3-dimethoxyphenyl)-9-methyl-1-(trifluoromethyl)-4H,6H-[1,2,4]triazolo[4,3-a][4,1]benzoxazepin-4-yl]acetate

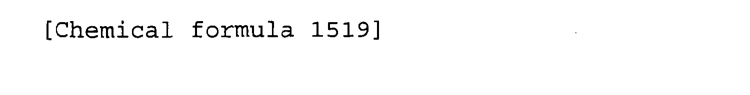

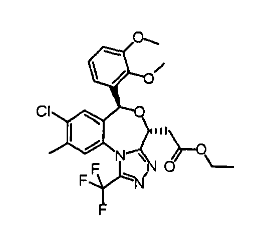

A racemic mixture obtained from ethyl [(trans)-7-chloro-5-(2,3-dimethoxyphenyl)-8-methyl-2-thioxo-1,2,3,5-tetrahydro-4,1-benzoxazepin-3-yl]acetate (0.70 g) in a similar manner to that employed for the synthesis of ethyl [(4R,6S)-8-chloro-6-(3-ethyl-2-methoxyphenyl)-1-(trifluoromethyl)4H,6H-[1,2,4]triazolo[4,3-a][4,1]benzoxazepin-4-yl]acetate was optically resolved by an optically active column (CHIRALPAK-AD; 5 cm × 50 cm) into an isomer A (0.21 g) and a target product, an isomer B (0.20 g).
Flow rate: 50 mL/min
Developing solvent: 15% isopropanol- n-hexane
Retention time: isomer A: 20 min., isomer B: 65 min.
¹H-NMR (CDCl₃) δ: 1.28 (3H, t; J = 7.11 Hz), 2.46 (3H, s), 3.23 (1H, dd, J = 16.79, 6.99 Hz), 3.43 (3H, s), 3.49 (1H, dd, J = 16.67, 6.62 Hz), 3.86 (3H, s), 4.19 (2H, q, J = 7.11 Hz), 4.92 (1H, t, J = 6.74 Hz), 5.54 (1H, s), 6.82 (1H, s.), 6.97 (1H, dd, J = 7.11, 2.70 Hz), 7.17-7.19 (2H, m), 7.43 (1H, s).
MS (ESI) m/z : 526 (M + H)⁺.

### Example 733

[(4R,6S)-8-Chloro-6-(2,3-dimethoxyphenyl)-9-methyl-1-(trifluoromethyl)-4H,6H-[1,2,4]triazolo[4,3-a][4,1]benzoxazepin-4-yl]acetic acid

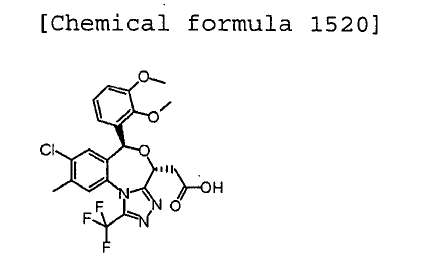

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (160 mg) was obtained from ethyl [(4R,6S)-8-chloro-6-(2,3-dimethoxyphenyl)-9-methyl-1-(trifluoromethyl)-4H,6H-[1,2,4]triazolo[4,3-a][4,1]benzoxazepin-4-yl]acetate (205 mg).
¹H-NMR (CDCl₃) δ: 2.46 (3H, s), 3.29 (1H, dd, J = 17.16, 6.62 Hz), 3.44 (3H, s), 3.56 (1H, dd, J = 16.91, 6.86 Hz), 3.86 (3H, s), 4.89 (1H, t, J = 6.74 Hz), 5.55 (1H, s), 6.83 (1H, s), 6.98 (1H, dd, J = 7.23, 2.57 Hz), 7.16-7.20 (2H, m), 7.44 (1H, s).
MS (ESI) m/z : 498 (M + H)⁺.
Elemental analysis for: C₂₂H₁₉ClF₃N₃O₅·0.25H₂O·0.2 n-hexane Calculated: C, 53.63; H, 4.33; N, 8.09.
Found: C, 53.21; H, 4.34; N, 7.87.

### Referential Example 583

tert-Butyl [4-chloro-3-(trifluoromethyl)phenyl]carbamate

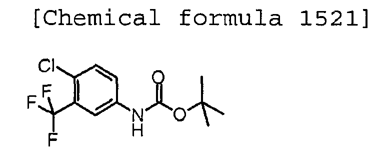

In a similar manner to that employed for the synthesis of tert-butyl (4-chloro-3-methylphenyl)carbamate, the title compound (3.0 g) was obtained from 4-chloro-3-trifluoromethylphenylamine (2.0 g).
¹H-NMR (CDCl₃) δ: 1.52 (9H, s), 6.56 (1H, br s), 7.40 (1H, d, J = 8.54 Hz), 7.50 (1H, d, J = 8.54 Hz), 7.73 (1H, d, J = 2.44 Hz).

### Referential Example 584

tert-Butyl [4-chloro-2-[(2,3-dimethoxyphenyl)(hydroxy)methyl]-5-(trifluoromethyl)phenyl]carbamate

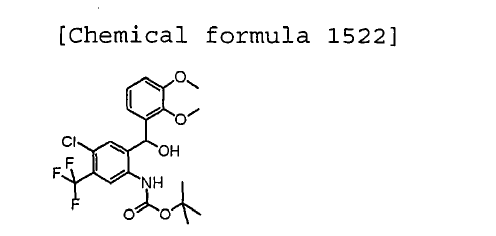

In a similar manner to that employed for the synthesis of tert-butyl {4-chloro-2-[(2,3-dichlorophenyl)(hydroxy)methyl]phenyl}carbamate, the title compound (1.67 g) was obtained from tert-butyl [4-chloro-3-(trifluoromethyl)phenyl]carbamate (1.50 g) and 2,3-dimethoxybenzaldehyde (0.89 g).
¹H-NMR (CDCl₃) δ: 1.51 (9H, s), 3.37 (1H, d, J = 4.66 Hz), 3.87 (3H, s), 3.90 (3H, s), 6.07 (1H, d, J = 4.66 Hz), 6.87 (1H, dd, J = 8.09, 1.23 Hz), 6.94 (1H, dd, J = 8.33, 1.47 Hz), 7.11-7.07 (1H, m), 8.28 (1H, s), 8.36 (1H, s).

### Referential Example 585

[2-Amino-5-chloro-4-(trifluoromethyl)phenyl](2,3-dimethoxyphenyl)methanol

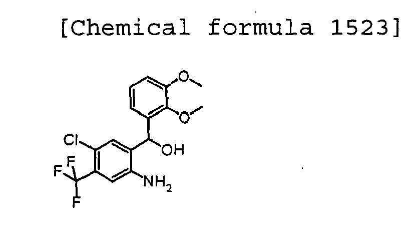

In a similar manner to that employed for the synthesis of (2-amino-5-chlorophenyl)(2,3-dichlorophenyl)methanol, the title compound (1.27 g) was obtained from tert-butyl [4-chloro-2-[(2,3-dimethoxyphenyl)(hydroxy)methyl]-5-(trifluoromethyl)phenyl]carbamate (1.67 g).
MS (ESI) m/z : 344 (M - OH)⁺.

### Referential Example 586

[5-Chloro-2-[(2,4-dimethoxybenzyl)amino]-4-(trifluoromethyl)phenyl](2,3-dimethoxyphenyl)methanol

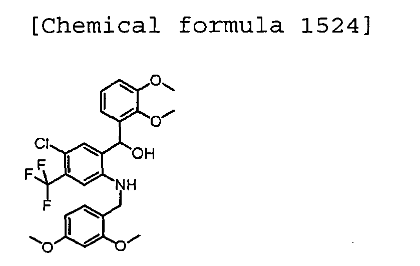

In a similar manner to that employed for the synthesis of {5-chloro-2-[(2,4-dimethoxybenzyl)amino]phenyl}(3-ethyl-2-methoxyphenyl)methanol, the title compound (1.16 g) was obtained from [2-amino-5-chloro-4-(trifluoromethyl)phenyl](2,3-dimethoxyphenyl)methanol (1.27 g).
¹H-NMR (CDCl₃) δ: 3.78 (2H, s), 3.79-3.79 (4H, m), 3.89 (2H, s), 4.25 (2H, s), 5.46 (1H, s), 5.99 (1H, s), 6.39 (1H, dd, J = 8.33, 2.45 Hz), 6.44 (1H, d, J = 2.45 Hz), 6.47 (1H, dd, J = 5.52, 2.33 Hz), 6.78 (1H, dd, J = 7.84, 1.47 Hz), 6.90-6.93 (1H, m), 6.98 (1H, s), 7.02-7.07 (2H, m), 7.16 (1H, s).

### Referential Example 587

Ethyl (2E)-4-[[4-chloro-2-[(2,3-dimethoxyphenyl)(hydroxy)methyl]-5-(trifluoromethyl)phenyl](2,4-dimethoxybenzyl)amino]-4-oxobut-2-enoate

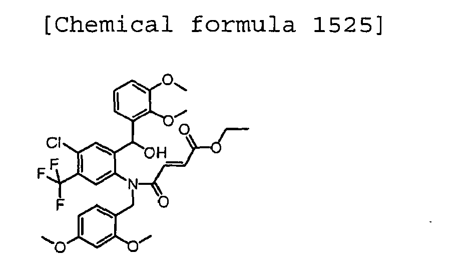

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, a crudely purified product of the title compound was obtained from [5-chloro-2-[(2,4-dimethoxybenzyl)amino]-4-(trifluoromethyl)phenyl](2,3-dimethoxyphenyl)methanol (1.16 g).
MS (ESI) m/z : 638 (M + H)⁺.

### Referential Example 588

Ethyl [(trans)-7-chloro-1-(2,4-dimethoxybenzyl)-5-(2,3-dimethoxyphenyl)-2-oxo-8-(trifluoromethyl)-1,2,3,5-tetrahydro-4,1-benzoxazepin-3-yl]acetate

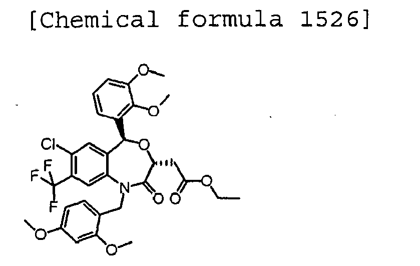

In a similar manner to that employed for the synthesis of ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate, the title compound (0.43 g) was obtained from ethyl (2E)-4-[[4-chloro-2-[(2,3-dimethoxyphenyl)(hydroxy)methyl]-5-(trifluoromethyl)phenyl](2,4-dimethoxybenzyl)amino]-4-oxobut-2-enoate (0.70 g).
¹H-NMR (CDCl₃) δ: 1.26 (3H, t, J = 7.11 Hz), 2.80 (1H, dd, J = 16.67, 5.88 Hz), 3.10 (1H, dd, J = 16.67, 7.60 Hz), 3.20 (3H, s), 3.63 (3H, s), 3.75 (3H, s), 3.85 (3H, s), 4.10-4.18 (2H, m), 4.46 (1H, dd, J = 7.60, 5.88 Hz), 4.87 (1H, d, J = 14.71 Hz), 5.57 (1H, d, J = 14.71 Hz), 5.90 (1H, s), 6.36 (1H, d, J = 2.21 Hz), 6.42 (1H, dd, J = 8.46, 2.33 Hz), 6.66 (1H, s), 6.94 (1H, dd, J = 7.84, 1.96 Hz), 7.10-7.17 (2H, m), 7.33 (1H, d, J = 8.33 Hz), 7.77 (1H, s). MS (ESI) m/z : 638 (M + H)⁺.

### Referential Example 589

Ethyl [(trans)-7-chloro-5-(2,3-dimethoxyphenyl)-2-oxo-8-(trifluoromethyl)-1,2,3,5-tetrahydro-4,1-benzoxazepin-3-yl]acetate

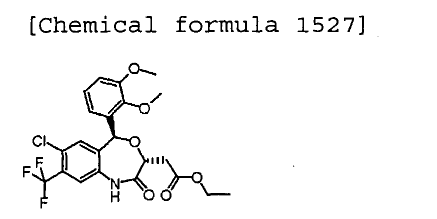

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.56 g) was obtained from ethyl [(trans)-7-chloro-1-(2,4-dimethoxybenzyl)-5-(2,3-dimethoxyphenyl)-2-oxo-8-(trifluoromethyl)-1,2,3,5-tetrahydro-4,1-benzoxazepin-3-yl]acetate (0.85 g).
¹H-NMR (CDCl₃) δ: 1.21 (3H, t, J = 7.08 Hz), 2.80 (1H, dd, J = 16.48, 6.47 Hz), 3.03 (1H, dd, J = 16.48, 6.47 Hz), 3.70 (3H, s), 3.90 (3H, s), 4.06-4.15 (2H, m), 4.66 (1H, t, J = 6.47 Hz), 6.23 (1H, s), 6.85 (1H, s), 6.98-7.00 (2H, m), 7.15 (1H, t, J = 7.93 Hz), 7.35 (1H, s), 7.83 (1H, s). MS (ESI) m/z : 488 (M + H)⁺.

### Referential Example 590

Ethyl [(trans)-7-chloro-5-(2,3-dimethoxyphenyl)-2-thioxo-8-(trifluoromethyl)-1,2,3,5-tetrahydro-4,1-benzoxazepin-3-yl]acetate

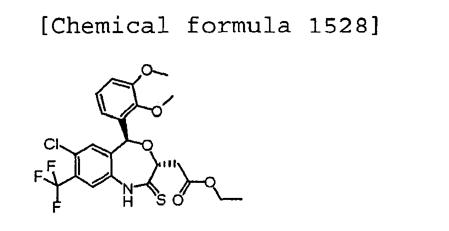

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.52 g) was obtained from ethyl [(trans)-7-chloro-5-(2,3-dimethoxyphenyl)-2-oxo-8-(trifluoromethyl)-1,2,3,5-tetrahydro-4,1-benzoxazepin-3-yl]acetate (0.56 g).
¹H-NMR (CDCl₃) δ: 1.24 (3H, t, J = 7.23 Hz), 2.95 (1H, dd, J = 16.42, 6.37 Hz), 3.27 (1H, dd, J = 16.55, 6.74 Hz), 3.67 (3H, s), 3.89 (3H, s), 4.13 (2H, q, J = 7.11 Hz), 4.73 (1H, t, J = 6.50 Hz), 6.15 (1H, s), 6.82 (1H, s), 7.00 (1H, dd, J = 8.09, 1.47 Hz), 7.08 (1H, dd, J = 7.84, 1.47 Hz), 7.17 (1H, t, J = 7.97 Hz), 7.44 (1H, s), 9.52 (1H, s).
MS (ESI) m/z : 504 (M + H)⁺.

### Example 734

Ethyl [(4R,6S)-8-chloro-6-(2,3-dimethoxyphenyl)-1,9-bis(trifluoromethyl)-4H,6H-[1,2,4]triazolo[4,3-a][4,1]benzoxazepin-4-yl]acetate

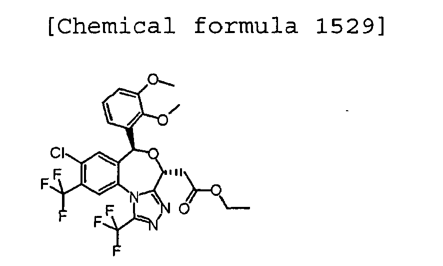

A racemic mixture obtained from ethyl [(trans)-7-chloro-5-(2,3-dimethoxyphenyl)-2-thioxo-8-(trifluoromethyl)-1,2,3,5-tetrahydro-4,1-benzoxazepin-3-yl]acetate (0.66 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-OD; 5 cm × 50 cm) into a target product, that is, an isomer A (0.20 g) and an isomer B (0.19 g).
Flow rate: 50 mL/min
Developing solvent: 10% isopropanol- n-hexane
Retention time: isomer A: 29 min., isomer B: 38 min.
¹H-NMR (CDCl₃) δ: 1.29 (3H, t, J = 7.11 Hz), 3.25 (1H, dd, J = 16.67, 6.86 Hz), 3.49 (3H, s), 3.51 (1H, dd, J = 17.16, 7.35 Hz), 3.87 (3H, s), 4.20 (2H, q, J = 7.35 Hz), 4.95 (1H, t, J = 6.74 Hz), 5.54 (1H, s), 6.99-7.02 (2H, m), 7.18-7.20 (2H, m), 7.91 (1H, s).
MS (ESI) m/z : 580 (M + H)⁺.

### Example 735

[(4R,6S)-8-Chloro-6-(2,3-dimethoxyphenyl)-1,9-bis(trifluoromethyl)-4H,6H-[1,2,4]triazolo[4,3-a][4,1]benzoxazepin-4-yl]acetic acid

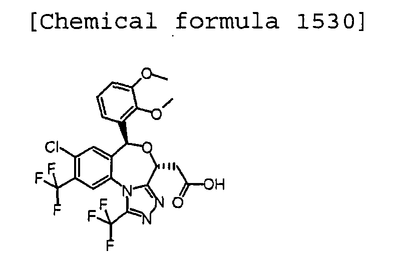

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (160 mg) was obtained from ethyl [(4R,6S)-8-chloro-6-(2,3-dimethoxyphenyl)-1,9-bis(trifluoromethyl)-4H,6H-[1,2,4]triazolo[4,3-a][4,1]benzoxazepin-4-yl]acetate (200 mg).
¹H-NMR (CDCl₃) δ: 3.32 (1H, dd, J = 17.16, 6.86 Hz), 3.49 (3H, s), 3.59 (1H, dd, J = 17.16, 6.86 Hz), 3.87 (3H, s), 4.93 (1H, t, J = 6.62 Hz), 5.55 (1H, s), 7.00-7.02 (2H, m), 7.18-7.21 (2H, m), 7.92 (1H, s).
MS (ESI) m/z : 552 (M + H)⁺.
Elemental analysis for: C₂₂H₁₆ClF₆N₃O₅·0.3CHCl₃
Calculated: C, 45.58; H, 2.80; N, 7.15.
Found: C, 45.40; H, 2.75; N, 7.23.

### Referential Example 591

tert-Butyl (4-chloro-3-fluorophenyl)carbamate

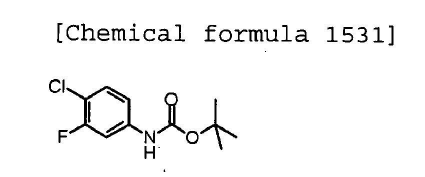

In a similar manner to that employed for the synthesis of tert-butyl (4-chloro-3-methylphenyl)carbamate, the title compound (4.12 g) was obtained from 4-chloro-3-fluorophenylamine (2.50 g).
¹H-NMR (CDCl₃) δ: 1.52 (9H, s), 6.49 (1H, s), 6.93-6.96 (1H, m), 7.24-7.28 (1H, m), 7.43 (1H, dd, J = 11.22, 2.20 Hz) .

### Referential Example 592

tert-Butyl {4-chloro-2-[(2,3-dimethoxyphenyl)(hydroxy)methyl]-5-fluorophenyl}carbamate

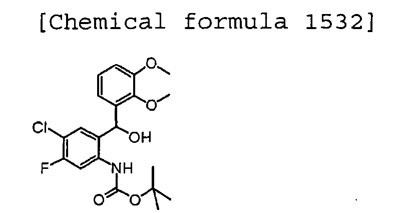

In a similar manner to that employed for the synthesis of tert-butyl {4-chloro-2-[(2,3-dichlorophenyl)(hydroxy)methyl]phenyl}carbamate, the title compound (1.75 g) was obtained from tert-butyl (4-chloro-3-fluorophenyl)carbamate (1.50 g) and 2,3-dimethoxybenzaldehyde (1.01 g).
¹H-NMR (CDCl₃) δ: 1.41 (9H, s), 3.68 (1H, d, J = 2.93 Hz), 3.88 (3H, s), 3.91 (3H, s), 6.56-6.61 (2H, m), 6.90 (1H, d, J = 8.06 Hz), 6.97 (1H, t, J = 8.06 Hz), 7.31 (1H, t, J = 8.54 Hz), 7.83 (1H, d, J = 9.03 Hz), 8.44 (1H, s).
MS (ESI) m/z : 434 (M + Na)⁺.

### Referential Example 593

(2-Amino-5-chloro-4-fluorophenyl)(2,3-dimethoxyphenyl)methanol

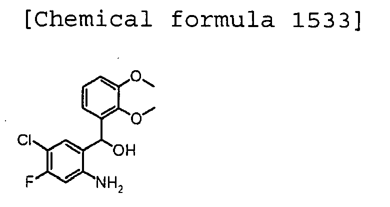

In a similar manner to that employed for the synthesis of (2-amino-5-chlorophenyl)(2,3-dichlorophenyl)methanol, the title compound (1.95 g) was obtained from tert-butyl {4-chloro-2-[(2,3-dimethoxyphenyl)(hydroxy)methyl]-5-fluorophenyl}carbamate (2.11 g).
MS (ESI) m/z : 294 (M - OH)⁺.

### Referential Example 594

{5-Chloro-2-[(2,4-dimethoxybenzyl)amino]-4-fluorophenyl}(2,3-dimethoxyphenyl)methanol

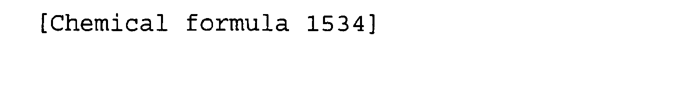

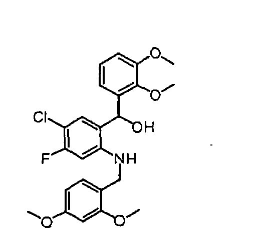

In a similar manner to that employed for the synthesis of {5-chloro-2-[(2,4-dimethoxybenzyl)amino]phenyl}(3-ethyl-2-methoxyphenyl)methanol, the title compound (2.47 g) was obtained from (2-amino-5-chloro-4-fluorophenyl)(2,3-dimethoxyphenyl)methanol (2.62 g).
¹H-NMR (CDCl₃) δ: 3.74 (2H, s), 3.77 (2H, s), 3.88-3.88 (4H, br m), 4.19 (1H, s), 6.33-6.41 (2H, m), 6.58 (1H, d, J = 8.82 Hz), 6.69 (1H, d, J = 7.60 Hz), 6.88-6.98 (3H, m), 7.10 (1H, t, J = 8.70 Hz).
MS (ESI) m/z : 462 (M + H)⁺.

### Referential Example 595

Ethyl (2E)-4-[[4-chloro-2-[(2,3-dimethoxyphenyl)(hydroxy) methyl]-5-fluorophenyl (2,4-dimethoxybenzyl)amino]-4-oxobut-2-enoate

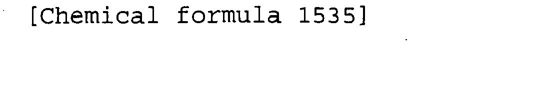

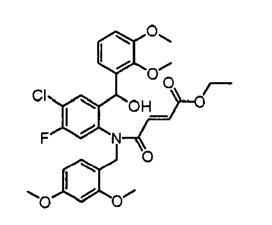

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, a crudely purified product of the title compound was obtained from {5-chloro-2-[(2,4-dimethoxybenzyl)amino]-4-fluorophenyl}(2,3-dimethoxyphenyl)methanol(2.47 g, 5.35 mmol) .

### Referential Example 596

Ethyl [(trans)-7-chloro-1-(2,4-dimethoxybenzyl)-5-(2,3-dimethoxyphenyl)-8-fluoro-2-oxo-1,2,3,5-tetrahydro-4,1-benzoxazepin-3-yl]acetate

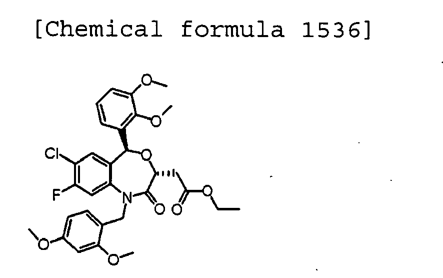

In a similar manner to that employed for the synthesis of ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate, the title compound (2.64 g) was obtained from ethyl (2E)-4-[[4-chloro-2-[(2,3-dimethoxyphenyl)(hydroxy)methyl]-5-fluorophenyl](2,4-dimethoxybenzyl)amino]-4-oxobut-2-enoate obtained as the crudely purified product.
MS (ESI) m/z : 588 (M + H)⁺.

### Referential Example 597

Ethyl [(trans)-7-chloro-5-(2,3-dimethoxyphenyl)-8-fluoro-2-oxo-1,2,3,5-tetrahydro-4,1-benzoxazepin-3-yl]acetate

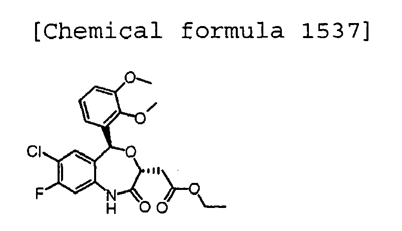

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (1.0 g) was obtained from ethyl [(trans)-7-chloro-1-(2,4-dimethoxybenzyl)-5-(2,3-dimethoxyphenyl)-8-fluoro-2-oxo-1,2,3,5-tetrahydro-4,1-benzoxazepin-3-yl]acetate (2.63 g).
MS (ESI) m/z : 438 (M + H)⁺.

### Referential Example 598

Ethyl [(trans)-7-chloro-5-(2,3-dimethoxyphenyl)-8-fluoro-2-thioxo-1,2,3,5-tetrahydro-4,1-benzoxazepin-3-yl]acetate

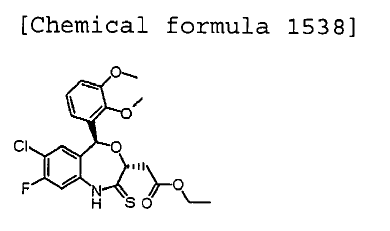

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.87 g) was obtained from ethyl [(trans)-7-chloro-5-(2,3-dimethoxyphenyl)-8-fluoro-2-oxo-1,2,3,5-tetrahydro-4,1-benzoxazepin-3-yl]acetate (1.00 g).
¹H-NMR (CDCl₃) δ: 1.29 (3H, t, J = 6.86 Hz), 2.96 (1H, dd, J = 17.16, 8.82 Hz), 3.25 (1H, dd, J = 17.16, 4.41 Hz), 3.80 (3H, s), 3.83 (3H, s), 4.16-4.24 (2H, m), 4.90 (1H, dd, J = 8.82, 4.41 Hz), 6.61 (1H, d, J = 1.47 Hz), 6.74-6.77 (2H, m), 6.86-6.88 (1H, m), 6.97(1H, t, J = 7.84 Hz), 7.29 (1H, t, J = 8.21 Hz), 9.28 (1H, s).
MS (ESI) m/z : 454 (M + H)⁺.

### Example 736

Ethyl [(4R,6S)-8-chloro-6-(2,3-dimethoxyphenyl)-9-fluoro-1-(trifluoromethyl)-4H,6H-[1,2,4]triazolo[4,3-a] [4,1]benzoxazepin-4-yl]acetate

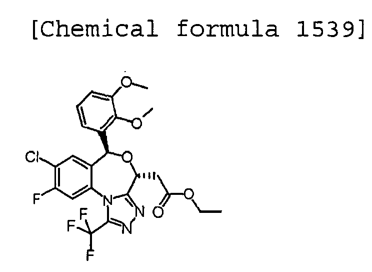

A racemic mixture obtained from ethyl [(trans)-7-chloro-5-(2,3-dimethoxyphenyl)-8-fluoro-2-thioxo-1,2,3,5-tetrahydro-4,1-benzoxazepin-3-yl]acetate (0.87 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50cm) into an isomer A (0.21 g) and a target product, that is, an isomer B (0.20 g).
Flow rate: 50 mL/min
Developing solvent: 10% isopropanol- n-hexane Retention time: isomer A: 35 min., isomer B: 60 min.
¹H-NMR (CDCl₃) δ: 1.29 (3H, t, J = 7.11 Hz), 3.35 (1H, dd, J = 16.91, 7.11 Hz), 3.44 (3H, s), 3.60 (1H, dd, J = 16.79, 6.50 Hz), 3.73 (3H, s), 4.20 (2H, dq, J = 14.22, 3.43 Hz), 5.02 (1H, t, J = 6.74 Hz), 6.44 (1H, d, J = 7.84 Hz), 6.63 (1H, s), 6.72 (1H, d, J = 7.11 Hz), 6.81 (1H, t, J = 8.09 Hz), 7.13 (1H, d, J = 8.58 Hz), 7.57 (1H, dd, J = 8.70, 7.48 Hz).
MS (ESI) m/z : 530 (M + H)⁺.

### Example 737

[(4R,6S)-8-Chloro-6-(2,3-dimethoxyphenyl)-9-fluoro-1-(trifluoromethyl)-4H,6H-[1,2,4]triazolo[4,3-a][4,1]benzoxazepin-4-yl]acetic acid

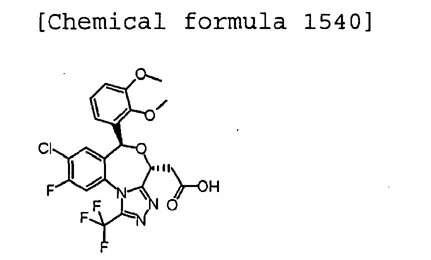

In a similar manner to that employed for the synthesis of , [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (166 mg) was obtained from ethyl [(4R,6S)-8-chloro-6-(2,3-dimethoxyphenyl)-9-fluoro-1-(trifluoromethyl)-4H,6H-[1,2,4]triazolo[4,3-a] [4,1]benzoxazepin-4-yl]acetate (206 mg).
¹H-NMR (CDCl₃) δ: 3.42 (1H, dd, J = 16.55, 6.25 Hz), 3.45 (3H, s), 3.67 (1H, dd, J = 17.04, 6.50 Hz), 3.73 (3H, s), 4.99 (1H, t, J = 6.62 Hz), 6.43 (1H, d, J = 7.84 Hz), 6.65 (1H, s), 6.73 (1H, d, J = 8.09 Hz), 6.81 (1H, t, J = 8.09 Hz), 7.13 (1H, d, J = 9.07 Hz), 7.58 (1H, dd, J = 8.70, 7.48 Hz).
MS (ESI) m/z : 502 (M + H)⁺.
Elemental analysis for: C₂₁H₁₆ClF₄N₃O₅·0.5H₂O·0.25 Hexane
Calculated: C, 50.76; H, 3.88; N, 7.89.
Found: C, 50.71; H, 3.57; N, 8.08.

### Referential Example 599

Ethyl [(trans)-1-(2,4-dimethoxybenzyl)-5-(2,3-dimethoxyphenyl)-7-ethyl-2-oxo-1,2,3,5-tetrahydro-4,1-benzoxazepin-3-yl]acetate

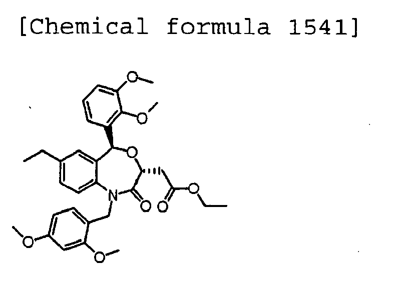

Ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate (1.50 g) was dissolved in 1,4-dioxane (30 mL). To the resulting solution were added ethylboronic acid (0.30 g), [1,1'-bis(diphenylphosphino)ferrocene]palladium(II) chloride (0.44 g) and tripotassium phosphate (2.59 g). The resulting mixture was stirred at 60°C for 24 hours. The reaction mixture was diluted with ethyl acetate and washed successively with water and saturated brine. The organic layer was dried over anhydrous sodium sulfate. The filtrate was concentrated and the residue was purified by silica gel column (hexane:ethyl acetate= from 4:1, 2:1 to 1:2) to give the title compound (1.5 g).
¹H-NMR (CDCl₃) δ: 1.04 (3H, t, J = 7.44 Hz), 1,2,4 (3H, t, J = 6.59 Hz), 2.43 .(2H, q, J = 7.48 Hz), 2.79 (1H, dd, J = 16.34, 5.85 Hz), 3.09 (1H, dd, J = 16.58, 7.56 Hz), 3.17 (3H, s), 3.67 (3H, s), 3.76 (3H, s), 3.85 (3H, s), 4.14 (2H, q, J = 6.75 Hz), 4.51 (1H, t, J = 6.83 Hz), 4.92 (1H, d, J = 14.88 Hz), 5.45 (1H, d, J = 15.12 Hz), 6.03 (1H, s), 6.36-6.42 (3H, m), 6.92 (1H, d, J = 7.80 Hz), 7.12-7.19 (3H, m), 7.24-7.27 (2H, m).
MS (ESI) m/z : 564 (M + H)⁺.

### Referential Example 600

Ethyl [(trans)-5-(2,3-dimethoxyphenyl)-7-ethyl-2-oxo-1,2,3,5-tetrahydro-4,1-benzoxazepin-3-yl]acetate

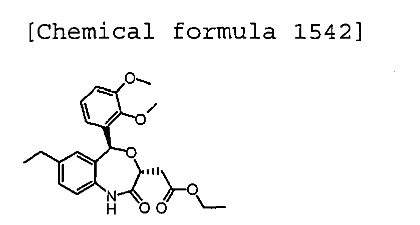

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.41 g) was obtained from ethyl [(trans)-1-(2,4-dimethoxybenzyl)-5-(2,3-dimethoxyphenyl)-7-ethyl-2-oxo-1,2,3,5-tetrahydro-4,1-benzoxazepin-3-yl]acetate (1.60 g).
¹H-NMR (CDCl₃) δ: 1.08 (3H, t, J = 7.56 Hz), 1.22 (3H, t, J = 7.07 Hz), 2.47 (2H, q, J = 7.56 Hz), 2.78 (1H, dd, J = 16.22, 6.46 Hz), 3.02 (1H, dd, J = 16.34, 6.59 Hz), 3.62 (3H, s), 3.88 (3H, s), 4.10 (2H, q, J = 7.07 Hz), 4.63 (1H, t, J = 6.71 Hz), 6.26 (1H, s), 6.50 (1H, s), 6.93-6.97 (2H, m), 7.08-7.16 (3H, m), 7.59 (1H, s).
MS (ESI) m/z : 414 (M + H)⁺.

### Referential Example 601

Ethyl [(trans)-5-(2,3-dimethoxyphenyl)-7-ethyl-2-thioxo-1,2,3,5-tetrahydro-4,1-benzoxazepin-3-yl]acetate

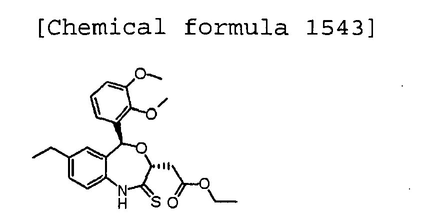

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.39 g) was obtained from ethyl [(trans)-5-(2,3-dimethoxyphenyl)-7-ethyl-2-oxo-1,2,3,5-tetrahydro-4,1-benzoxazepin-3-yl] acetate (0.41 g).
¹H-NMR (CDCl₃) δ: 1.08 (3H, t, J = 7.57 Hz), 1,2,4 (3H, t, J = 7.20 Hz), 2.49 (2H, q, J = 7.57 Hz), 2.96 (1H, dd, J = 16.36, 6.59 Hz), 3.26 (1H, dd, J = 16.36, 6.59 Hz), 3.59 (3H, s), 3.88 (3H, s), 4.12 (2H, q, J = 7.08 Hz), 4.72 (1H, t, J = 6.71 Hz), 6.16 (1H, s), 6.49 (1H, s), 6.96 (1H, dd, J = 6.47, 3.05 Hz), 7.00-7.03 (1H, m), 7.13-7.19 (3H, m), 9.31 (1H, s).
MS (ESI) m/z : 430 (M + H)⁺.

### Example 738

Ethyl [(4R,6S)-6-(2,3-dimethoxyphenyl)-8-ethyl-1-(trifluoromethyl)-4H,6H-[1,2,4]triazolo[4,3-a][4,1]benzoxazepin-4-yl]acetate

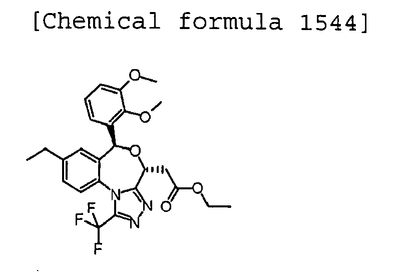

A racemic mixture obtained from ethyl [(trans)-5-(2,3-dimethoxyphenyl)-7-ethyl-2-thioxo-1,2,3,5-tetrahydro-4,1-benzoxazepin-3-yl]acetate (0.39 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50 cm) into an isomer A (0.11 g) and a target product, that is, an isomer B (0.11 g).
Flow rate: 50 mL/min
Developing solvent: 30% isopropanol- n-hexane
Retention time: isomer A: 20 min., isomer B: 40 min.
¹H-NMR (CDCl₃) δ: 1.14 (3H, t, J = 7.60 Hz), 1.28 (3H, t, J = 7.11 Hz), 2.58 (2H, q, J = 7.60 Hz), 3.24 (1H, dd, J = 16.55, 7.23 Hz), 3.37 (3H, s), 3.48 (1H, dd, J = 16.67, 6.62 Hz), 3.85 (3H, s), 4.15-4.21 (2H, m), 4.93 (1H, t, J = 6.74 Hz), 5.60 (1H, s), 6.67 (1H, d, J = 1.72 Hz), 6.96 (1H, dd, J = 8.09, 1.47 Hz), 7.18 (1H, t, J = 7.84 Hz), 7.23 (1H, dd, J = 7.84, 1.47 Hz), 7.35 (1H, dd, J = 8.21, 1.84 Hz), 7.48 (1H, d, J = 8.09 Hz).
MS (ESI) m/z : 506 (M + H)⁺.

### Example 739

[(4R,6S)-6-(2,3-Dimethoxyphenyl)-8-ethyl-1-(trifluoromethyl)-4H,6H-[1,2,4]triazolo[4,3-a][4,1]benzoxazepin-4-yl]acetic acid

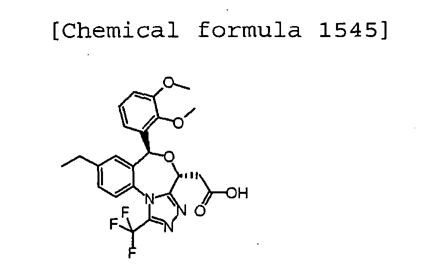

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (96 mg) was obtained from ethyl [(4R,6S)-6-(2,3-dimethoxyphenyl)-8-ethyl-1-(trifluoromethyl)-4H,6H-[1,2,4]triazolo[4,3-a][4,1]benzoxazepin-4-yl]acetate (106 mg).
¹H-NMR (CDCl₃) δ: 1.14 (3H, t, J = 7.60 Hz), 2.58 (2H, q, J = 7.60 Hz), 3.29 (1H, dd, J = 16.91, 6.62 Hz), 3.37 (3H, s), 3.56 (1H, dd, J = 16.67, 6.62 Hz), 3.85 (3H, s), 4.89 (1H, t, J = 6.86 Hz), 5.60 (1H, s), 6.68 (1H, d, J = 1.96 Hz), 6.97 (1H, dd, J = 8.09, 1.47 Hz), 7.19 (1H, t, J = 7.84 Hz), 7.24 (1H, dd, J = 7.97, 1.59 Hz), 7.36 (1H, dd, J = 8.21, 1.84 Hz), 7.48 (1H, d, J = 8.09 Hz).
MS (ESI) m/z : 478 (M + H)⁺.
Elemental analysis for: C₂₃H₂₂F₃N₃O₅·0.75H₂O·0.51,4-dioxane Calculated: C, 56.13; H, 5.18; N, 7.85.
Found: C, 55.96; H, 5.17; N, 7.58.

### Referential Example 602

1-Bromo-3-methoxy-2-methylbenzene

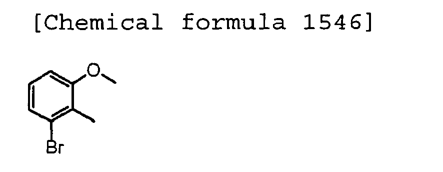

To a mixture obtained by adding water (6 mL) and a 48% aqueous bromide solution (2.4 mL) to 3-methoxy-2-methylaniline (0.77 mL) was added dropwise an aqueous solution (1.20 mL) of sodium nitrite (0.46 g) at 0°C and the resulting mixture was stirred for 10 minutes. After addition of acetone (24 mL), copper bromide (0.95 g) was added at 0°C and the resulting mixture was stirred for 3. hours. The reaction mixture was warmed to room temperature and concentrated. The residue was diluted with dichloromethane and washed with a mixture of a saturated aqueous solution of sodium hydrogen carbonate and aqueous ammonia. The organic layer was dried over anhydrous sodium sulfate. The filtrate was concentrated to give the title compound (1.38 g).
¹H-NMR (CDCl₃) δ: 2.31 (3H, s), 3.82 (3H, s), 6.78 (1H, d, J = 8.06 Hz), 7.01 (1H, t, J = 8.12 Hz), 7.15 (1H, dd, J = 8.06, 0.73 Hz).

### Referential Example 603

(5-Bromo-2-fluorophenyl)(3-methoxy-2-methylphenyl)methanol

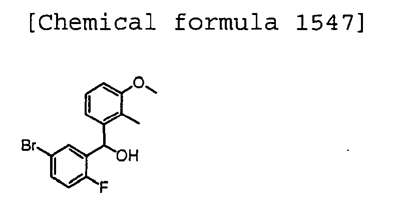

1-Bromo-3-methoxy-2-methylbenzene (7.17 g) was dissolved in tetrahydrofuran (60 mL). In a nitrogen atmosphere, n-butyl lithium (2.6 mol/l, a hexane solution) (15.38 mL) was added to the resulting solution at -78°C and the resulting mixture was stirred at -78°C for 2.5 hours. To the reaction mixture was added dropwise a tetrahydrofuran solution (40 mL) of 5-bromo-2-fluorobenzaldehyde (4.23 mL) at -78°C. While gradually returning to room temperature, the resulting mixture was stirred for 3 hours. To the reaction mixture was then added a saturated aqueous solution of ammonium chloride. The resulting mixture was extracted with ethyl acetate.
The extract was washed with saturated brine. The organic layer was dried over anhydrous sodium sulfate. After the filtrate was concentrated, the residue thus obtained was purified by silica gel column (hexane:ethyl acetate= from 8:1, 4:1 to 2:1) to give the title compound (7.45 g).
¹H-NMR (CDCl₃) δ: 2.18 (3H, s), 3.83 (3H, s), 6.30 (1H, d, J = 4.17 Hz), 6.83 (1H, d, J = 8.09 Hz), 6.91 (1H, dd, J = 9.56, 8.82 Hz), 7.01 (1H, d, J = 7.84 Hz), 7.20 (1H, t, J = 7.97 Hz), 7.34-7.38 (1H, m), 7.51 (1H, dd, J = 6.62, 2.45 Hz) .
MS (ESI) m/z : 307 (M - OH)⁺.

### Referential Example 604

(5-Bromo-2-fluorophenyl)(3-methoxy-2-methylphenyl)methanone

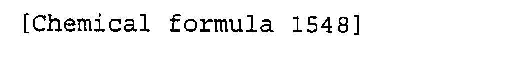

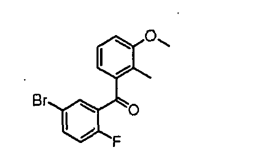

(5-Bromo-2-fluorophenyl)(3-methoxy-2-methylphenyl)methanol (6.81 g) was dissolved in dichloromethane (150 mL). To the resulting solution was added manganese dioxide (20.69 g). The resulting mixture was heated under reflux overnight. The reaction mixture was filtered and the filtrate was concentrated. The residue thus obtained was purified by silica gel column (hexane:ethyl acetate= from 18:1, 9:1 to 4:1) to give the title compound (6.26 g).
¹H-NMR (CDCl₃) δ: 2.27 (3H, s), 3.88 (3H, s), 6.92 (1H, d, J = 7.56 Hz), 6.98-7.03 (2H, m), 7.21 (1H, t, J = 7.93 Hz), 7.60-7.64 (1H, m), 7.75 (1H, dd, J = 6.22, 2.56 Hz).

### Referential Example 605

{5-Bromo-2-[(2,4-dimethoxybenzyl)amino]phenyl}(3-methoxy-2-methylphenyl)methanone

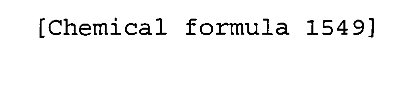

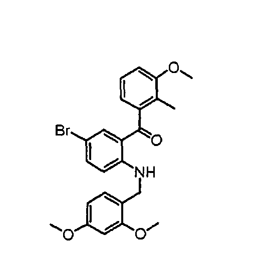

(5-Bromo-2-fluorophenyl)(3-methoxy-2-methylphenyl)methanone (100 mg) was dissolved in acetonitrile (5.0 mL). To the resulting solution were added 2,4-dimethoxybenzylamine mono hydrochloric acid (95 mg) and potassium carbonate (128 mg). The resulting mixture was heated under reflux overnight. The reaction mixture was filtered and the filtrate was concentrated.
The residue thus obtained was purified by silica gel column (hexane:ethyl acetate= from 8:1, 4:1 to 2:1) to give the title compound (156 mg).
¹H-NMR (CDCl₃) δ: 2.09 (3H, s), 3.80 (3H, s), 3.86 (3H, s), 3.88 (3H, s), 4.43 (2H, d, J = 5.88 Hz), 6.45 (1H, dd, J = 8.33, 2.45 Hz), 6.49 (1H, d, J = 2.45 Hz), 6.67 (1H, d, J = 8.82 Hz), 6.81 (1H, d, J = 7.60 Hz), 6.92 (1H, d, J = 8.33 Hz), 7.18 (1H, d, J = 8.33 Hz), 7.22 (1H, t, J = 7.97 Hz), 7.32-7.35 (2H, m), 9.35 (1H, br s).

### Referential Example 606

{5-Bromo-2-[(2,4-dimethoxybenzyl)amino]phenyl}(3-methoxy-2-methylphenyl)methanol

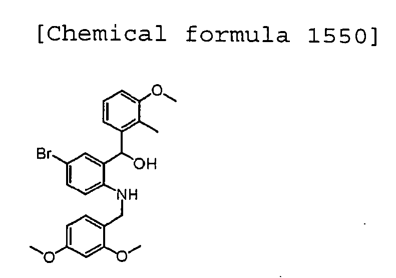

{5-Bromo-2-[(2,4-dimethoxybenzyl)amino]phenyl}(3-methoxy-2-methylphenyl)methanone (9.35 g) was dissolved in methanol (100 mL) and tetrahydrofuran (50 mL). To the resulting solution was added sodium borohydride (4.52 g) at 0°C. The resulting mixture was stirred overnight at room temperature. After the reaction mixture was concentrated, water was added to the residue. The resulting mixture was extracted with ethyl acetate. The organic layer was washed successively with water and saturated brine. The organic layer was dried over anhydrous sodium sulfate. The filtrate was concentrated and then, the residue thus obtained was purified by silica gel column (hexane:ethyl acetate= from 8:1, 4:1 to 2:1) to give the title compound (9.98 g).
¹H-NMR (CDCl₃) δ: 2.08 (3H, s), 3.77 (3H, s), 3.79 (3H, s), 3.85 (3H, s), 4.24 (2H, s), 5.97 (1H, s), 6.40 (1H, dd, J = 8.18, 2.32 Hz), 6.45 (1H, d, J = 2.44 Hz), 6.59 (1H, d, J = 8.79 Hz), 6.86 (1H, d, J = 8.06 Hz), 6.91 (1H, d, J = 2.20 Hz), 7.02 (1H, d, J = 7.57 Hz), 7.06 (1H, d, J = 8.30 Hz), 7.19-7.24 (2H, m).

### Referential Example 607

Ethyl (2E)-4-[{4-bromo-2-[hydroxy(3-methoxy-2-methylphenyl)methyl]phenyl}(2,4-dimethoxybenzyl)amino]-4-oxobut-2-enoate

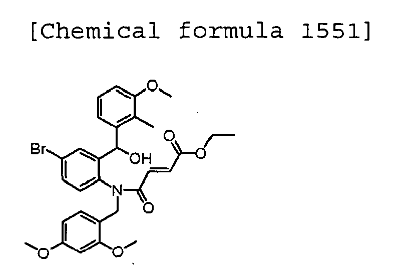

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, a crudely purified product of the title compound was obtained from {5-bromo-2-[(2,4-dimethoxybenzyl)amino]phenyl}(3-methoxy-2-methylphenyl)methanol (11.5 g).

### Referential Example 608

Ethyl [(trans)-7-bromo-1-(2,4-dimethoxybenzyl)-5-(3-methoxy-2-methylphenyl)-2-oxo-1,2,3,5-tetrahydro-4,1-benzoxazepin-3-yl]acetate

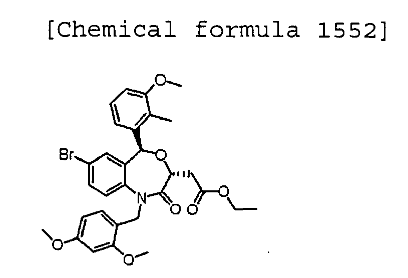

In a similar manner to that employed for the synthesis of ethyl [(trans)-7-bromo-1-(2,4-dimethoxybenzyl)-5-(2,3-dimethoxyphenyl)-2-oxo-1,2,3,5-tetrahydro-4,1-benzoxazepin-3-yl]acetate, the title compound (11.88 g) was obtained from the crudely purified product of ethyl (2E)-4-[{4-bromo-2-[hydroxy(3-methoxy-2-methylphenyl)methyl]phenyl}(2,4-dimethoxybenzyl)amino]-4-oxobut-2-enoate
¹H-NMR (CDCl₃) δ: 1.25 (3H, t, J = 7.11 Hz), 1.36 (3H, s), 2.80 (1H, dd, J = 16.42, 5.88 Hz), 3.10 (1H, dd, J = 16.55, 7.97 Hz), 3.57 (3H, s), 3.76 (3H, s), 3.81 (3H, s), 4.11-4.17 (2H, m), 4.45 (1H, dd, J = 7.84, 5.88 Hz), 4.86 (1H, d, J = 14.46 Hz), 5.47 (1H, d, J = 14.46 Hz), 5.61 (1H, s), 6.32 (1H, d, J = 2.45 Hz), 6.40 (1H, dd, J = 8.33, 2.45 Hz), 6.60 (1H, d, J = 2.21 Hz), 6.84 (1H, d, J = 8.09 Hz), 7.16 (1H, d, J = 7.60 Hz), 7.23 (1H, d, J = 8.33 Hz), 7.35 (2H, dd, J = 15.93, 8.58 Hz), 7.48 (1H, dd, J = 8.70, 2.33 Hz).

### Referential Example 609

Ethyl [(trans)-7-bromo-5-(3-methoxy-2-methylphenyl)-2-oxo-1,2,3,5-tetrahydro-4,1-benzoxazepin-3-yl]acetate

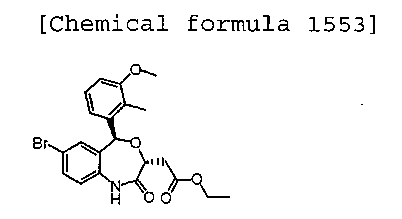

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (6.99 g) was obtained from ethyl [(trans)-7-bromo-1-(2,4-dimethoxybenzyl)-5-(3-methoxy-2-methylphenyl)-2-oxo-1,2,3,5-tetrahydro-4,1-benzoxazepin-3-yl]acetate (11.9 g)
¹H-NMR (CDCl₃) δ: 1.22 (3H, t, J = 7.20 Hz), 2.01 (3H, s), 2.79 (1H, dd, J = 16.36, 6.35 Hz), 2.99 (1H, dd, J = 16.36, 6.35 Hz), 3.86 (3H, s), 4.07-4.15 (2H, m), 4.54 (1H, t, J = 6.35 Hz), 6.06 (1H, s), 6.88-6.94 (4H, m), 7.21 (1H, t, J = 7.93 Hz), 7.43 (1H, dd, J = 8.42, 2.32 Hz), 7.83 (1H, s). MS (ESI) m/z : 450 (M + H) +.

### Referential Example 610

Ethyl [(trans)-7-bromo-5-(3-methoxy-2-methylphenyl)-2-thioxo-1,2,3,5-tetrahydro-4,1-benzoxazepin-3-yl]acetate

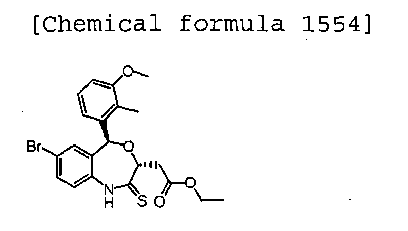

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (1.65 g) was obtained from ethyl [(trans)-7-bromo-5-(3-methoxy-2-methylphenyl)-2-oxo-1,2,3,5-tetrahydro-4,1-benzoxazepin-3-yl] acetate (2.00 g).
¹H-NMR (CDCl₃) δ: 1.24 (3H, t, J = 7.08 Hz), 1.91 (3H, s), 2.96 (1H, dd, J = 16.36, 6.59 Hz), 3.25 (1H, dd, J = 16.60, 6.59 Hz), 3.85 (3H, s), 4.13 (2H, q, J = 7.16 Hz), 4.69 (1H, t, J = 6.47 Hz), 5.96 (1H, s), 6.82 (1H, d, J = 2.20 Hz), 6.90 (1H, d, J = 8.06 Hz), 7.00 (1H, d, J = 8.54 Hz), 7.08 (1H, d, J = 7.81 Hz), 7.23-7.28 (2H, m), 7.50 (1H, dd, J = 8.30, 2.20 Hz), 9.56 (1H, s).
MS (ESI) m/z : 464 (M + H)⁺.

### Referential Example 611

Ethyl [(trans)-7-bromo-2-hydrazono-5-(3-methoxy-2-methylphenyl)-1,2,3,5-tetrahydro-4,1-benzoxazepin-3-yl] acetate

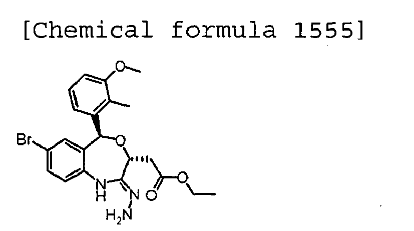

Ethyl [(trans)-7-bromo-5-(3-methoxy-2-methylphenyl)-2-thioxo-1,2,3,5-tetrahydro-4,1-benzoxazepin-3-yl]acetate (1.65 g) was dissolved in tetrahydrofuran (30 mL). To the resulting solution was added hydrazine monohydrate (0.52 mL) at 0°C. The resulting mixture was stirred at room temperature for 2 hours. The reaction mixture was diluted with ethyl acetate and washed successively with water and saturated brine. The organic layer was dried over anhydrous sodium sulfate. The filtrate was concentrated to give the title compound (1.85 g). MS (ESI) m/z : 462 (M + H)⁺.

### Example 740

Ethyl [(4R,6S)-8-bromo-1-[chloro(difluoro)methyl]-6-(3-methoxy-2-methylphenyl)-4H,6H-[1,2,4]triazolo[4,3-a] [4,1]benzoxazepin-4-yl]acetate

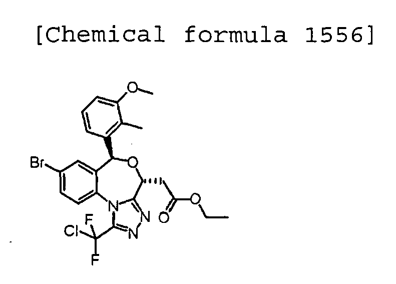

Ethyl [(trans)-7-bromo-2-hydrazono-5-(3-methoxy-2-methylphenyl)-1,2;3,5-tetrahydro-4,1-benzoxazepin-3-yl]acetate (0.61 g) was dissolved in 1,2-dichloroethane (15 mL). To the resulting solution was added chlorodifluoroacetic anhydride (1.84 mL) at 0°C. The resulting mixture was stirred overnight at room temperature. The reaction mixture was concentrated. To the residue were added toluene (15 mL) and chlorodifluoroacetic anhydride (1.84 mL). The resulting mixture was heated under reflux for 3 hours. After cooling to room temperature, the reaction mixture was concentrated. The residue thus obtained was purified by silica gel column (hexane:ethyl acetate= from 8:1, 4:1 to 2:1) to give a racemic mixture (0.65 g) of the title compound. The racemic mixture thus obtained was optically resolved by an optically active column (CHIRALPAK-AD; 5 cm × 50 cm) into a target product, that is, an isomer A (0.32 g) and an isomer
B (0.32 g).
Flow rate: 50 mL/min
Developing solvent: 10% isopropanol- n-hexane
Retention time: isomer A: 50 min., isomer B: 60 min.
MS (ESI) m/z : 556 (M + H)⁺.

### Example 741

[(4R,6S)-8-Bromo-1-[chloro(difluoro)methyl]-6-(3-methoxy-2-methylphenyl)-4H,6H-[1,2,4]triazolo[4,3-a] [4,1]benzoxazepin-4-yl]acetic acid

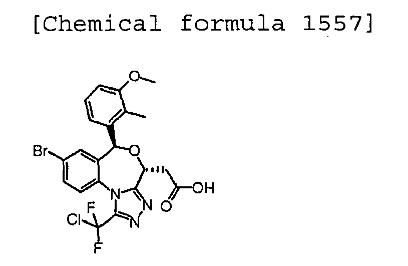

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (225 mg) was obtained from ethyl [(4R,6S)-8-bromo-1-[chloro(difluoro)methyl]-6-(3-methoxy-2-methylphenyl)-4H,6H-[1,2,4]triazolo[4,3-a][4,1]benzoxazepin-4-yl]acetate (315 mg). ¹H-NMR (CDCl₃) δ: 1.66 (3H, s), 3.32 (1H, dd, J = 17.16, 6.86 Hz), 3.57 (1H, dd, J = 17.04, 6.74 Hz), 3.84 (3H, s), 4.90 (1H, t, J = 6.74 Hz), 5.38 (1H, s), 6.91 (1H, dd, J = 7.97, 1.10 Hz), 6.95 (1H, d, J = 2.21 Hz), 7.25-7.27 (1H, m), 7.32 (1H, t, J = 7.97 Hz), 7.52 (1H, d, J = 8.58 Hz), 7.71 (1H, dd, J = 8.58, 2.21 Hz).
MS (ESI) m/z : 528 (M + H)⁺.
Elemental analysis for: C₂₁H₁₇BrClF₂N₃O₄·0.1 n-hexane Calculated: C, 48.28; H, 3.45; N, 7.82.
Found: C, 48.37; H, 3.62; N, 7.35.

### Example 742

Ethyl [(4R,6S)-8-bromo-1-(1,1-difluoroethyl)-6-(3-methoxy-2-methylphenyl)-4H,6H-[1,2,4]triazolo[4,3-a] [4,1]benzoxazepin-4-yl]acetate

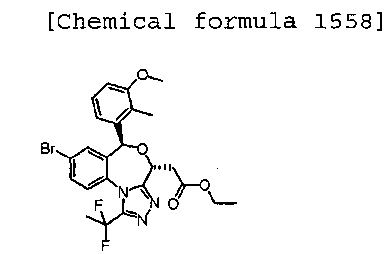

Ethyl [(trans)-7-bromo-2-hydrazono-5-(3-methoxy-2-methylphenyl)-1,2,3,5-tetrahydro-4,1-benzoxazepin-3-yl]acetate (0.63 g) was dissolved in dichloromethane (15 mL). To the resulting solution were added difluoropropionic acid (0.34 g), 1-ethyl-3-(3-dimethylaminopropyl)carbodiimide hydrochloride (0.39 g), and 1-hydroxybenzotriazole (0.31 g) at 0°C. While warming gradually to room temperature, the resulting mixture was stirred overnight. The reaction mixture was diluted with ethyl acetate and washed successively with water and saturated brine. The organic layer was dried over anhydrous sodium sulfate. The filtrate was concentrated. To the residue thus obtained were added toluene (10 mL) and acetic acid (3.0 mL). The resulting mixture was stirred at 90°C for one hour. After cooling to room temperature, the reaction mixture was concentrated. The residue thus obtained was purified by silica gel column (hexane:ethyl acetate= from 6:1, 3:1 to 1:1) to give a racemic mixture (0.62 g) of the title compound. The racemic mixture thus obtained was optically resolved by an optically active column (CHIRALPAK-AD; 5 cm × 50 cm) into an isomer A (0.29 g) as the target product and an isomer B (0.29 g).
Flow rate: 50 mL/min
Developing solvent: 30% isopropanol- n-hexane
Retention time: isomer A: 30 min., isomer B: 40 min.
MS (ESI) m/z : 536 (M + H)⁺.

### Example 743

[(4R,6S)-8-Bromo-1-(1,1-difluoroethyl)-6-(3-methoxy-2-methylphenyl)-4H,6H-[1,2,4]triazolo[4,3-a][4,1]benzoxazepin-4-yl]acetic acid

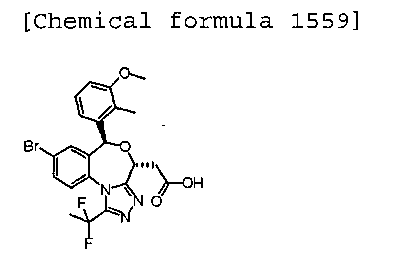

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (200 mg) was obtained from ethyl [(4R,6S)-8-bromo-1-(1,1-difluoroethyl)-6-(3-methoxy-2-methylphenyl)-4H,6H-[1,2,4]triazolo[4,3-a][4,1]benzoxazepin-4-yl]acetate (285 mg).
¹H-NMR (CDCl₃) δ: 1.65 (3H, s), 2.33 (3H, t, J = 19.24 Hz), 3.30 (1H, dd, J = 16.91, 6.62 Hz), 3.55 (1H, dd, J = 17.04, 6.99 Hz), 3.83 (3H, s), 4.89 (1H, t, J = 6.74 Hz), 5.37 (1H, s), 6.89-6.91 (2H, m), 7.28-7.33 (2H, m), 7.63-7.68 (2H, m).
MS (ESI) m/z : 508 (M + H)⁺.
Elemental analysis for: C₂₂H₂₀BrF₂N₃O₄·0.5H₂O·0.1Hexane
Calculated: C, 52.13; H, 4.49; N, 7.86.
Found: C, 51.72; H, 4.31; N, 7.51.

### Example 744

Ethyl [(4R,6S)-8-bromo-6-(3-methoxy-2-methylphenyl)-1-(trifluoromethyl)-4H,6H-[1,2,4]triazolo[4,3-a][4,1]benzoxazepin-4-yl]acetate

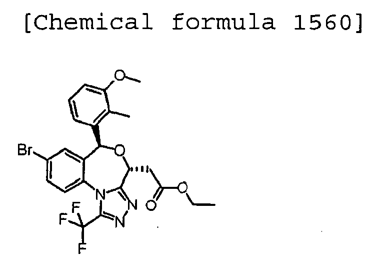

Ethyl [(trans)-7-bromo-2-hydrazono-5-(3-methoxy-2-methylphenyl)-1,2,3,5-tetrahydro-4,1-benzoxazepin-3-yl]acetate (0.61 g) was dissolved in 1,2-dichloroethane (15 mL). To the resulting solution was added trifluoroacetic anhydride (1.47 mL) at 0°C. The resulting mixture was stirred overnight at room temperature. After the reaction mixture was concentrated, toluene (15 mL) and trifluoroacetic anhydride (1.47 mL) were added to the residue. The resulting mixture was heated under reflux for 3 hours. After cooling to room temperature, the reaction mixture was concentrated. The residue thus obtained was purified by silica gel column (hexane:ethyl acetate= from 8:1, 4:1 to 2:1) to give a racemic mixture (0.62 g) of the title compound. The racemic mixture thus obtained was optically resolved by an optically active column (CHIRALPAK-AD; 5 cm × 50cm) into an isomer A (0.33 g) as the target product and an isomer B (0.28 g).
Flow rate: 50 mL/min
Developing solvent: 10% isopropanol- n-hexane
Retention time: isomer A: 40 min., isomer B: 50 min.
MS (ESI) m/z : 540 (M + H)⁺.

### Example 745

[(4R,6S)-8-Bromo-6-(3-methoxy-2-methylphenyl)-1-(trifluoromethyl)-4H,6H-[1,2,4]triazolo[4,3-a] [4,1]benzoxazepin-4-yl]acetic acid

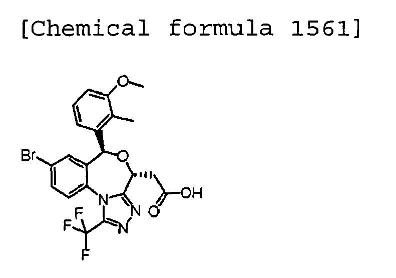

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (250 mg) was obtained from ethyl [(4R,6S)-8-bromo-6-(3-methoxy-2-methylphenyl)-1-(trifluoromethyl)-4H,6H-[1,2,4]triazolo[4,3-a][4,1]benzoxazepin-4-yl]acetate (325 mg).
¹H-NMR (CDCl₃) δ: 1.66 (3H, s), 3.32 (1H, dd, J = 17.16, 6.86 Hz), 3.58 (1H, dd, J = 17.16, 6.86 Hz), 3.84 (3H, s), 4.93 (1H, t, J = 6.86 Hz), 5.38 (1H, s), 6.92 (1H, d, J = 8.09 Hz), 6.96 (1H, d, J = 2.21 Hz), 7.24-7.26 (1H, m), 7.32 (1H, t, J = 7.97 Hz), 7.47 (1H, d, J = 8.58 Hz), 7.71 (1H, dd, J = 8.58, 2.21 Hz).
MS (ESI) m/z : 514 (M + H)⁺.
Elemental analysis for: C₂₁H₁₇BrF₃N₃O₄·0.5H₂O·0.3 n-hexane
Calculated: C, 50.05; H, 4.09; N, 7.68.
Found: C, 50.12; H, 3.73; N, 7.62.

### Referential Example 612

2-Chloro-3-(difluoromethoxy)benzaldehyde

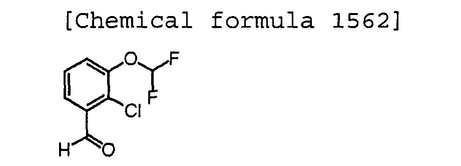

2-Chloro-3-hydroxybenzaldehyde (2.0 g) was dissolved in dimethylformamide (20 mL). To the resulting solution were added sodium chlorodifluoroacetate (2.83 g), potassium carbonate (3.43 g) and water (3 mL). The resulting mixture was stirred at 100°C for 2 hours. After filtration of the reaction mixture, the filtrate was concentrated. The residue thus obtained was purified by silica gel column (hexane:ethyl acetate= from 10:0, 9:1 to 5:1) to give the title compound (2.41 g).
¹H-NMR (CDCl₃) δ: 6.59 (1H, t, J = 72.75 Hz), 7.41 (1H, t, J = 7.81 Hz), 7.51 (1H, d, J = 7.32 Hz), 7.83 (1H, dd, J = 7.69, 1.59 Hz), 10.51 (1H, s).
MS (ESI) m/z : 207 (M + H)⁺.

### Referential Example 613

tert-Butyl {4-chloro-2-[[2-chloro-3-(difluoromethoxy)phenyl](hydroxy)methyl]phenyl}carbamate

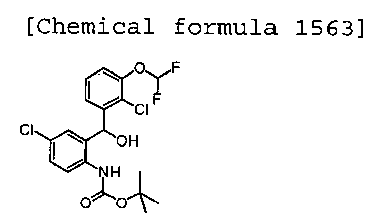

In a similar manner to that employed for the synthesis of tert-butyl {4-chloro-2-[(2,3-dichlorophenyl)(hydroxy)methyl]phenyl}carbamate, the title compound (1.67 g) was obtained from tert-butyl 4-chlorophenylcarbamate (2.20 g) and 2-chloro-3-(difluoromethoxy)benzaldehyde (2.99 g).
¹H-NMR (CDCl₃) δ: 1.50 (9H, s), 3.66 (1H, s), 6.17 (1H, d, J = 3.91 Hz), 6.53 (1H, t, J = 73.24 Hz), 6.92 (1H, d, J = 2.44 Hz), 7.21-7.25 (2H, m), 7.37 (1H, t, J = 8.06 Hz), 7.53-7.59 (2H, m).

### Referential Example 614

(2-Amino-5-chlorophenyl)[2-chloro-3-(difluoromethoxy)phenyl]methanol

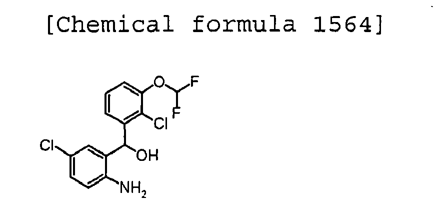

In a similar manner to that employed for the synthesis of (2-amino-5-chlorophenyl)(2,3-dichlorophenyl)methanol, a crudely purified product of the title compound was obtained from tert-butyl {4-chloro-2-[[2-chloro-3-(difluoromethoxy)phenyl](hydroxy)methyl]phenyl}carbamate (1.67 g).
MS (ESI) m/z : 334 (M + H)⁺.

### Referential Example 615

[2-chloro-3-(difluoromethoxy)phenyl]{5-chloro-2-[(2,4-dimethoxybenzyl)amino]phenyl}methanol

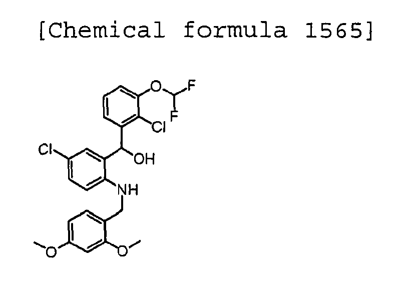

In a similar manner to that employed for the synthesis of {5-chloro-2-[(2,4-dimethoxybenzyl)amino]phenyl}(3-ethyl-2-methoxyphenyl)methanol, the title compound (1.33 g) was obtained from the crudely purified product of (2-amino-5-chlorophenyl)[2-chloro-3-(difluoromethoxy)phenyl]methanol.
¹H-NMR (CDCl₃) δ: 2.55 (1H, br s), 3.78 (3H, s), 3.80 (3H, s), 4.26 (2H, s), 4.88 (1H, br s), 6.16 (1H, s), 6.35-6.53 (3H, m), 6.69 (1H, t, J = 8.66 Hz), 6.81 (1H, d, J = 2.44 Hz), 7.08-7.14 (2H, m), 7.23-7.26 (1H, m), 7.32 (1H, t, J = 7.93 Hz), 7.39 (1H, dd, J = 7.80, 1.71 Hz).

### Referential Example 616

Ethyl (2E)-4-[{4-chloro-2-[[2-chloro-3-(difluoromethoxy)phenyl](hydroxy)methyl]phenyl}(2,4-dimethoxybenzyl)amino]-4-oxobut-2-enoate

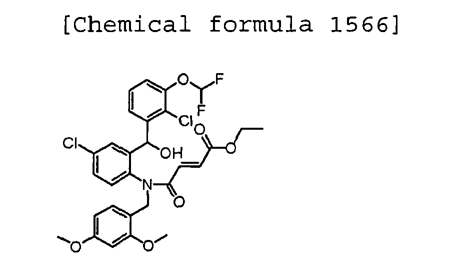

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, a crudely purified product of the title compound was obtained from [2-chloro-3-(difluoromethoxy)phenyl]{5-chloro-2-[(2,4-dimethoxybenzyl)amino]phenyl}methanol (1.33 g).
MS (ESI) m/z : 610 (M + H)⁺.

### Referential Example 617

Ethyl [(trans)-7-chloro-5-[2-chloro-3-(difluoromethoxy)phenyl]-1-(2,4-dimethoxybenzyl)-2-oxo-1,2,3,5-tetrahydro-4,1-benzoxazepin-3-yl]acetate

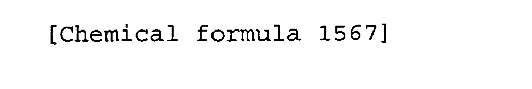

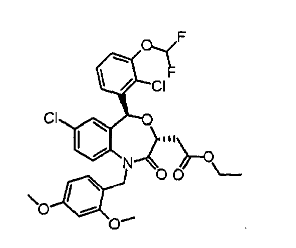

Ethyl (2E)-4-[{4-chloro-2-[[2-chloro-3-(difluoromethoxy)phenyl](hydroxy)methyl]phenyl}(2,4-dimethoxybenzyl)amino]-4-oxobut-2-enoate (1.68 g) was dissolved in ethanol (20 mL). To the resulting solution was added 1,8-diazabicyclo[5,4,0]undec-7-ene (2.05 mL).
The resulting mixture was heated under reflux overnight. The reaction mixture was concentrated. The residue was diluted with ethyl acetate and washed with water and saturated brine. The organic layer was dried over anhydrous sodium sulfate. The filtrate was concentrated and the residue thus obtained was purified by silica gel column (hexane:ethyl acetate= from 6:1, 3:1 to 1:1) to give the title compound (1.25 g).
¹H-NMR (CDCl₃) δ: 1.26 (3H, t, J = 7.20 Hz), 2.81 (1H, dd, J = 16.48, 5.98 Hz), 3.09 (1H, dd, J = 16.60, 7.57 Hz), 3.61 (3H, s), 3.76 (3H, s), 4.11-4.18 (2H, m), 4.47 (1H, dd, J = 7.57, 6.10 Hz), 4.85 (1H, d, J = 14.65 Hz), 5.51 (1H, d, J = 14.89 Hz), 5.85 (1H, s), 6.33-6.35 (2H, m), 6.39 (1H, dd, J = 8.42, 2.32 Hz), 6.49 (1H, t, J = 73.49 Hz), 7.25 (1H, d, J = 7.57 Hz), 7.31-7.41 (4H, m), 7.55 (1H, dt, J = 6.02, 2.20 Hz).
MS (ESI) m/z : 610 (M + H)⁺.

### Referential Example 618

Ethyl {(trans)-7-chloro-5-[2-chloro-3-(difluoromethoxy)phenyl]-2-oxo-1,2,3,5-tetrahydro-4,1-benzoxazepin-3-yl}acetate

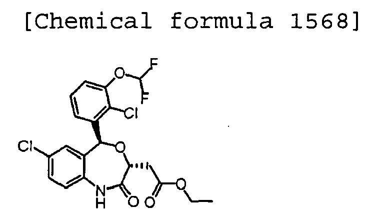

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.83 g) was obtained from ethyl [(trans)-7-chloro-5-[2-chloro-3-(difluoromethoxy)phenyl]-1-(2,4-dimethoxybenzyl)-2-oxo-1,2,3,5-tetrahydro-4,1-benzoxazepin-3-yl]acetate (1.25 g).
¹H-NMR (CDCl₃) δ: 1.26 (3H, t, J = 7.20 Hz), 2.81 (1H, dd, J = 16.60, 6.59 Hz), 3.05 (1H, dd, J = 16.60, 6.59 Hz), 4.10-4.16 (2H, m), 4.62 (1H, t, J = 6.71 Hz), 6.21 (1H, s), 6.37-6.74 (2H, m), 7.04 (1H, d, J = 8.54 Hz), 7.31-7.36 (2H, m), 7.41 (1H, t, J = 7.93 Hz), 7.50-7.52 (1H, m), 7.80 (1H, s).
MS (ESI) m/z : 460 (M + H)⁺.

### Referential Example 619

Ethyl {(trans)-7-chloro-5-[2-chloro-3-(difluoromethoxy)phenyl]-2-thioxo-1,2,3,5-tetrahydro-4,1-benzoxazepin-3-yl}acetate

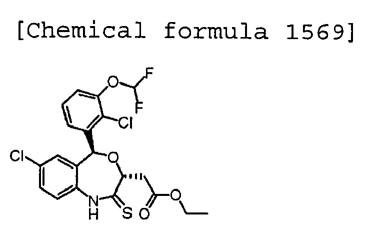

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.72 g) was obtained from ethyl {(trans)-7-chloro-5-[2-chloro-3-(difluoromethoxy)phenyl]-2-oxo-1,2,3,5-tetrahydro-4,1-benzoxazepin-3-yl}acetate (0.83 g).
¹H-NMR (CDCl₃) δ: 1.26 (3H, t, J = 7.07 Hz), 2.99 (1H, dd, J = 16.58, 6.59 Hz), 3.26 (1H, dd, J = 16.58, 6.59 Hz), 4.15 (2H, q, J = 6.59 Hz), 4.69 (1H, t, J = 6.71 Hz), 6.11 (1H, s), 6.52 (1H, d, J = 2.44 Hz), 6.54 (1H, t, J = 73.41 Hz), 7.11 (1H, d, J = 8.29 Hz), 7.32 (1H, d, J = 8.05 Hz), 7.38-7.45 (2H, m), 7.56-7.58 (1H, m), 9.61 (1H, s).
MS (ESI) m/z : 476 (M + H)⁺.

### Example 746

Ethyl [(4R,6S)-8-chloro-6-[2-chloro-3-(difluoromethoxy)phenyl]-1-(trifluoromethyl)-4H,6H-[1,2,4]triazolo[4,3-a][4,1]benzoxazepin-4-yl]acetate

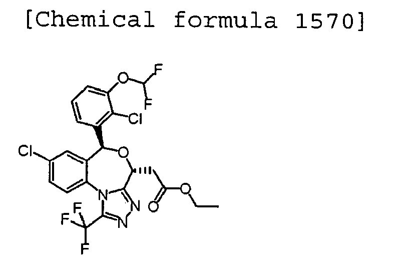

A racemic mixture obtained from ethyl {(trans)-7-chloro-5-[2-chloro-3-(difluoromethoxy)phenyl]-2-thioxo-1,2,3,5-tetrahydro-4,1-benzoxazepin-3-yl}acetate (0.72 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50cm) into an isomer A (0.31 g) as the target product and an isomer B (0.29 g) .
Flow rate: 50 mL/min
Developing solvent: 15% isopropanol - n-hexane
Retention time: isomer A: 20 min., isomer B: 40 min.
¹H-NMR (CDCl₃) δ: 1.29 (3H, t, J = 7.11 Hz), 3.27 (1H, dd, J = 16.79, 7.23 Hz), 3.51 (1H, dd, J = 16.67, 6.37 Hz), 4.18-4.24 (2H, m), 4.97 (1H, t, J = 6.74 Hz), 5.54 (1H, s), 6.52 (1H, t, J = 71.94 Hz), 6.70 (1H, s), 7.34 (1H, d, J = 8.09 Hz), 7.46 (1H, t, J = 8.09 Hz), 7.57-7.58 (2H, m), 7.63-7.65 (1H, m).
MS (ESI) m/z : 552 (M + H)⁺.

### Example 747

[(4R,6S)-8-Chloro-6-[2-chloro-3-(difluoromethoxy)phenyl]-1-(trifluoromethyl)-4H,6H-[1,2,4]triazolo[4,3-a][4,1]benzoxazepin-4-yl]acetic acid

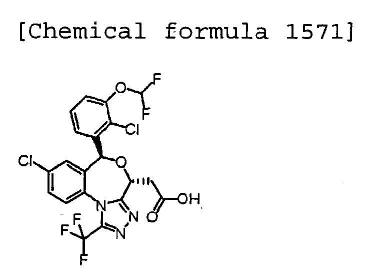

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (220 mg) was obtained from ethyl [(4R,6S)-8-chloro-6-[2-chloro-3-(difluoromethoxy)phenyl]-1-(trifluoromethyl)-4H,6H-[1,2,4]triazolo[4,3-a][4,1]benzoxazepin-4-yl]acetate (310 mg).
¹H-NMR (CDCl₃) δ: 3.34 (1H, dd, J = 17.16, 6.86 Hz), 3.59 (1H, dd, J = 17.16, 6.62 Hz), 4.94 (1H, t, J = 6.62 Hz), 5.55 (1H, s), 6.52 (1H, t, J = 73.29 Hz), 6.71 (1H, s), 7.34 (1H, d, J = 8.09 Hz), 7.47 (1H, t, J = 8.09 Hz), 7.55-7.61 (2H, m), 7.65 (1H, d, J = 7.84 Hz).
MS (ESI) m/z : 524 (M + H)⁺.
Elemental analysis for: C₂₀H₁₂Cl₂F₅N₃O₄·0.25 n-hexane Calculated: C, 47.32; H, 2.86; N, 7.07.
Found: C, 46.91; H, 2.83; N, 7.48.

### Referential Example 620

2-Chloro-3-(trifluoromethoxy)benzaldehyde

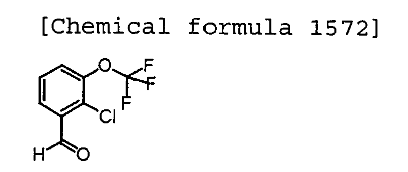

2-(Trifluoromethoxy)chlorobenzene (3.0 g) was dissolved in tetrahydrofuran (30 mL). In a nitrogen atmosphere, n-butyl lithium (2.6M, a hexane solution) (6.58 mL) was added dropwise to the resulting solution at -78°C and the resulting mixture was stirred for 30 minutes. To the reaction mixture was added dropwise dimethylformamide (1.29 mL) at -78°C. While warming gradually to room temperature, the resulting mixture was stirred for one hour. To the reaction mixture was added ice, followed by extraction with ethyl acetate. The organic layer was washed successively with an aqueous solution of citric acid, water and saturated brine. The organic layer was dried over anhydrous sodium sulfate. The filtrate was concentrated and the residue thus obtained was purified by silica gel column (hexane:ethyl acetate= from 10:0, 9:1 to 6:1) to give the title compound (2.64 g).
MS (ESI) m/z : 225 (M + H)⁺.

### Referential Example 621

tert-Butyl {4-chloro-2-[[2-chloro-3-(trifluoromethoxy)phenyl](hydroxy)methyl]phenyl}carbamate

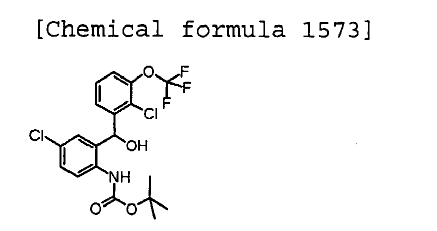

In a similar manner to that employed for the synthesis of tert-butyl {4-chloro-2-[(2,3-dichlorophenyl)(hydroxy)methyl]phenyl}carbamate, the title compound (2.52 g) was obtained from tert-butyl 4-chlorophenylcarbamate (2.23 g) and 2-chloro-3-(trifluoromethoxy)benzaldehyde (2.00 g, 8.92 mmol)
¹H-NMR (CDCl₃) δ: 1.49 (9H, s), 3.41 (1H, br s), 6.20 (1H, d, J = 3.68 Hz), 7.03 (1H, br s), 7.05 (1H, d, J = 2.45 Hz), 7.24 (1H, d, J = 2.21 Hz), 7.33 (1H, t, J = 7.97 Hz), 7.46 (1H, dd, J = 7.97, 1.10 Hz), 7.56 (2H, d, J = 8.09 Hz).
MS (ESI) m/z : 453 (M + H)⁺.

### Referential Example 622

(2-Amino-5-chlorophenyl)[2-chloro-3-(trifluoromethoxy)phenyl]methanol

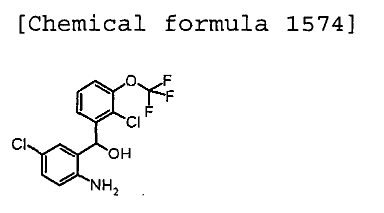

In a similar manner to that employed for the synthesis of (2-amino-5-chlorophenyl)(2,3-dichlorophenyl)methanol, the title compound (0.85 g) was obtained from tert-butyl {4-chloro-2-[[2-chloro-3-(trifluoromethoxy)phenyl](hydroxy)methyl]phenyl}carbamate (2.52 g).
¹H-NMR (CDCl₃) δ: 3.92-4.06 (2H, m), 6.16 (1H, s), 6.62 (1H, d, J = 8.54 Hz), 7.02 (1H, d, J = 2.44 Hz), 7.08 (1H, dd, J = 8.54, 2.44 Hz), 7.31 (1H, t, J = 8.05 Hz), 7.44-7.48 (2H, m).
MS (ESI) m/z : 334 (M - OH)⁺.

### Referential Example 623

{5-Chloro-2-[(2,4-dimethoxybenzyl)amino]phenyl}[2-chloro-3-(trifluoromethoxy)phenyl]methanol

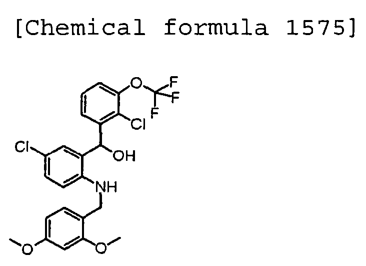

In a similar manner to that employed for the synthesis of {5-chloro-2-[(2,4-dimethoxybenzyl)amino]phenyl}(3-ethyl-2-methoxyphenyl)methanol, the title compound (0.94 g) was obtained from -5-chlorophenyl)[2-chloro-3-(trifluoromethoxy)phenyl]methanol (0.85 g).
¹H-NMR (CDCl₃) δ: 2.49 (1H, br s), 3.77 (3H, s), 3.79 (3H, s), 4.22 (2H, s), 4.69 (1H, br s), 6.13 (1H, s), 6.40 (1H, dd, J = 8.30, 2.44 Hz), 6.45 (1H, d, J = 2.20 Hz), 6.64 (1H, d, J = 8.54 Hz), 6.99-7.03 (2H, m), 7.12 (1H, dd, J = 8.67, 2.56 Hz), 7.27 (1H, t, J = 7.57 Hz), 7.41 (1H, dd, J = 8.06, 1.71 Hz), 7.46 (1H, dd, J = 7.93, 1.59 Hz).

### Referential Example 624

Ethyl (2E)-4-[{4-chloro-2-[[2-chloro-3-(trifluoromethoxy)phenyl](hydroxy)methyl]phenyl}(2,4-dimethoxybenzyl)amino]-4-oxobut-2-enoate

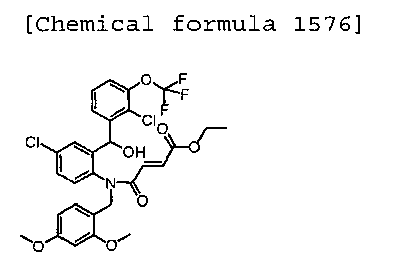

In a similar manner to that employed for the synthesis of ethyl (2E)-4-[{4-chloro-2-[(3-ethyl-2-methoxyphenyl)(hydroxy)methyl]phenyl}(2,4-dimethoxybenzyl)amino]-4-oxobut-2-enoate, a crudely purified product of the title compound was obtained from {5-chloro-2-[(2,4-dimethoxybenzyl)amino]phenyl}[2-chloro-3-(trifluoromethoxy)phenyl]methanol (0.94 g)
MS (ESI) m/z : 628 (M + H)⁺.

### Referential Example 625

Ethyl [(trans)-7-chloro-5-[2-chloro-3-(trifluoromethoxy)phenyl]-1-(2,4-dimethoxybenzyl)-2-oxo-1,2,3,5-tetrahydro-4,1-benzoxazepin-3-yl]acetate

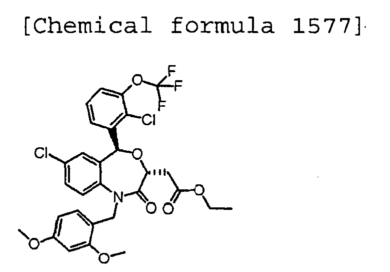

In a similar manner to that employed for the synthesis of ethyl [(trans)-7-chloro-5-[2-chloro-3-(difluoromethoxy)phenyl]-1-(2,4-dimethoxybenzyl)-2-oxo-1,2,3,5-tetrahydro-4,1-benzoxazepin-3-yl]acetate, the title compound (0.90 g) was obtained from the crudely purified product of ethyl (2E)-4-[{4-chloro-2-[[2-chloro-3-(trifluoromethoxy)phenyl](hydroxy)methyl]phenyl}(2,4-dimethoxybenzyl)amino]-4-oxobut-2-enoate.
¹H-NMR (CDCl₃) δ: 1,2,4 (3H, t, J = 7.11 Hz), 2.79 (1H, dd, J = 16.67, 5.88 Hz), 3.10 (1H, dd, J = 16.91, 7.60 Hz), 3.74 (3H, s), 3.79 (3H, s), 4.14 (2H, q, J = 7.11 Hz), 4.57 (1H, dd, J = 7.60, 6.13 Hz), 5.07-5.16 (2H, m), 6.01 (1H, s), 6.42-6.50 (3H, m), 7.21 (1H, d, J = 8.09 Hz), 7.28-7.29 (2H, m), 7.41 (1H, t, J = 7.84 Hz), 7.53 (1H, dd, J = 7.97, 1.59 Hz), 7.66 (1H, dd, J = 7.84, 1.72 Hz).
MS (ESI) m/z : 629 (M + H)⁺.

### Referential Example 626

Ethyl {(trans)-7-chloro-5-[2-chloro-3-(trifluoromethoxy)phenyl]-2-oxo-1,2,3,5-tetrahydro-4,1-benzoxazepin-3-yl}acetate

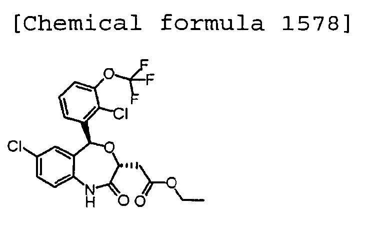

In a similar manner to that employed for the synthesis of ethyl [(trans)-7-chloro-5-(3-ethyl-2-methoxyphenyl)-2-oxo-1,2,3,5-tetrahydro-4,1-benzoxazepin-3-yl]acetate, the title compound (0.83 g) was obtained from ethyl [(trans)-7-chloro-5-[2-chloro-3-(trifluoromethoxy)phenyl]-1-(2,4-dimethoxybenzyl)-2-oxo-1,2,3,5-tetrahydro-4,1-benzoxazepin-3-yl]acetate (0.90 g).
¹H-NMR (CDCl₃) δ: 1.23 (3H, t, J = 6.35 Hz), 2.77 (1H, dd, J = 16.60, 6.59 Hz), 3.02 (1H, dd, J = 16.85, 6.59 Hz), 4.12 (2H, q, J = 7.08 Hz), 4.63 (1H, t, J = 6.59 Hz), 6.19 (1H, s), 6.60 (1H, d, J = 2.20 Hz), 7.04 (1H, d, J = 8.30 Hz), 7.34 (1H, dd, J = 8.42, 2.32 Hz), 7.41 (1H, t, J = 7.93 Hz), 7.55-7.60 (2H, m), 7.84 (1H, s).
MS (ESI) m/z : 500 (M + Na)⁺.

### Referential Example 627

Ethyl {(trans)-7-chloro-5-[2-chloro-3-(trifluoromethoxy)phenyl]-2-thioxo-1,2,3,5-tetrahydro-4,1-benzoxazepin-3-yl}acetate

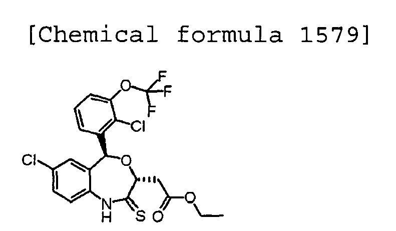

In a similar manner to that employed for the synthesis of ethyl [(trans)-7-chloro-5-(3-ethyl-2-methoxyphenyl)-2-thioxo-1,2,3,5-tetrahydro-4,1-benzoxazepin-3-yl]acetate, the title compound (0.51 g) was obtained from ethyl {(trans)-7-chloro-5-[2-chloro-3-(trifluoromethoxy)phenyl]-2-oxo-1,2,3,5-tetrahydro-4,1-benzoxazepin-3-yl}acetate (0.53 g).
¹H-NMR (CDCl₃) δ: 1,2,4 (3H, t, J = 6.86 Hz), 2.95 (1H, dd, J = 16.55, 6.74 Hz), 3.22 (1H, dd, J = 16.67, 6.37 Hz), 4.10-4.15 (2H, m), 4.71 (1H, t, J = 6.50 Hz), 6.11 (1H, s), 6.59 (1H, d, J = 2.21 Hz), 7.10 (1H, d, J = 8.33 Hz), 7.39 (1H, dd, J = 8.58, 2.45 Hz), 7.44 (1H, d, J = 8.09 Hz), 7.56 (1H, dd, J = 8.09, 1.72 Hz), 7.63 (1H, dd, J = 7.84, 1.23 Hz), 9.58 (1H, br s).
MS (ESI) m/z : 494 (M + H)⁺.

### Example 748

Ethyl [(4R,6S)-8-chloro-6-[2-chloro-3-(trifluoromethoxy)phenyl]-1-(trifluoromethyl)-4H,6H-[1,2,4]triazolo[4,3-a][4,1]benzoxazepin-4-yl]acetate

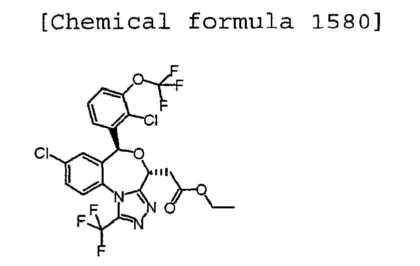

A racemic mixture obtained from ethyl {(trans)-7-chloro-5-[2-chloro-3-(trifluoromethoxy)phenyl]-2-thioxo-1,2,3,5-tetrahydro-4,1-benzoxazepin-3-yl}acetate (0.51 g) in a similar manner to that employed for the synthesis of ethyl [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetate was optically resolved by an optically active column (CHIRALPAK-AD; 5 cm × 50 cm) into an isomer A (0.16 g) as the target product and an isomer B (0.23 g).
Flow rate: 50 mL/min
Developing solvent: 7% isopropanol - n-hexane
Retention time: isomer A: 20 min., isomer B: 40 min.
MS (ESI) m/z : 570 (M + H)⁺.

### Example 749

[(4R,6S)-8-Chloro-6-[2-chloro-3-(trifluoromethoxy)phenyl]-1-(trifluoromethyl)-4H,6H-[1,2,4]triazolo[4,3-a][4,1]benzoxazepin-4-yl]acetic acid

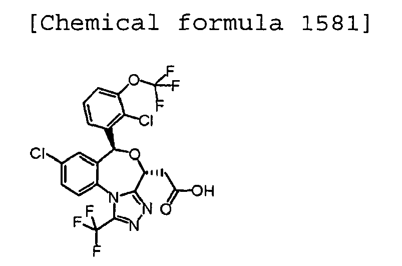

In a similar manner to that employed for the synthesis of [(4R,6S)-8-chloro-6-(3-ethyl-2-methoxyphenyl)-1-(trifluoromethyl)-4H,6H-[1,2,4]triazolo[4,3-a][4,1]benzoxazepin-4-yl]acetic acid, the title compound (125 mg) was obtained from ethyl [(4R,6S)-8-chloro-6-[2-chloro-3-(trifluoromethoxy)phenyl]-1-(trifluoromethyl)-4H,6H-[1,2,4]triazolo[4,3-a][4,1]benzoxazepin-4-yl]acetate (160 mg) .
¹H-NMR (CDCl₃) δ: 3.31 (1H, dd, J = 17.28, 6.99 Hz), 3.56 (1H, dd, J = 17.16, 6.37 Hz), 4.94 (1H, t, J = 6.74 Hz), 5.57 (1H, s), 6.81 (1H, d, J = 1.96 Hz), 7.46 (1H, t, J = 7.97 Hz), 7.55-7.61 (3H, m), 7.71 (1H, dd, J = 7.84, 1.47 Hz).
MS (ESI) m/z : 542 (M + H)⁺.
Elemental analysis for: C₂₀H₁₁Cl₂F₆N₃O₄·0.51,4-dioxane Calculated: C, 45.07; H, 2.58; N, 7.17.
Found: C, 44.72; H, 2.36; N, 7.36.

### Referential Example 659

tert-Butyl {4-chloro-2-[(3-fluoro-2-methylphenyl)(hydroxy)methyl]phenyl}carbamate

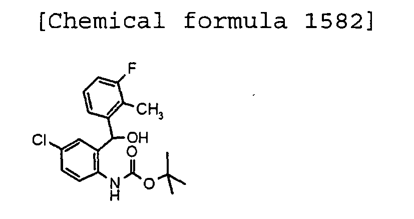

In a similar manner to that employed for the synthesis of tert-butyl {4-chloro-2-[(5-chloro-2,3-dimethoxyphenyl)(hydroxy)methyl]phenyl}carbamate, the title compound (3.59 g) was obtained from 3-fluoro-2-methylbenzaldehyde (1.52 g) and tert-butyl (4-chlorophenyl)carbamate (2.16 g).
¹H-NMR (CDCl₃) δ: 1.53 (9H, s), 2.05 (3H, d, J = 2.0 Hz), 2.89 (1H, s), 6.01 (1H, d, J = 3.9 Hz), 6.79 (1H, d, J = 2.5 Hz), 7.05 (1H, t, J = 8.8 Hz), 7.22-7.39 (4H, m), 7.73 (1H, d, J = 8.8 Hz).

### Referential Example 660

(2-Amino-5-chlorophenyl)(3-fluoro-2-methylphenyl)methanol

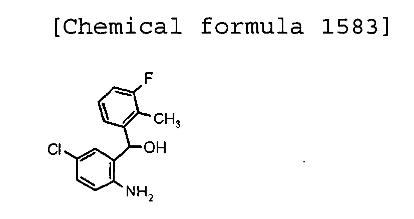

In a similar manner to that employed for the synthesis of (2-amino-5-fluorophenyl)(2-chloro-3-methoxyphenyl)methanol, a crude product of the title compound (2.19 g) was obtained from tert-butyl {4-chloro-2-[(3-fluoro-2-methylphenyl)(hydroxy)methyl]phenyl}carbamate (3.59 g).

### Referential Example 661

{5-Chloro-2-[(2,4-dimethoxybenzyl)amino]phenyl}(3-fluoro-2-methylphenyl)methanol

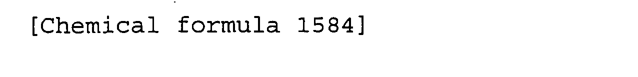

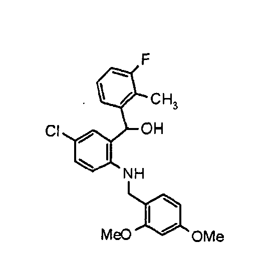

In a similar manner to that employed for the synthesis of {5-bromo-2-[(2,4-dimethoxybenzyl)amino]phenyl}(2-chloro-3-methoxyphenyl)methanol, a crudely purified product of the title compound (1.694 g) was obtained from (2-amino-5-chlorophenyl)(3-fluoro-2-methylphenyl)methanol (2.19 g).

### Referential Example 662

Ethyl [(trans)-7-chloro-1-(2,4-dimethoxybenzyl)-5-(3-fluoro-2-methylphenyl)-2-oxo-1,2,3,5-tetrahydro-4,1-benzoxazepin-3-yl]acetate

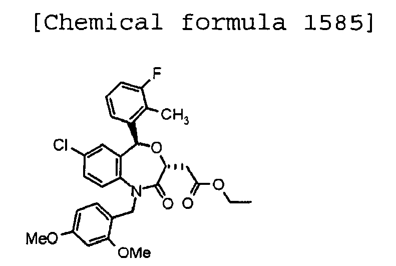

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (1.54 g) was obtained from {5-chloro-2-[(2,4-dimethoxybenzyl)amino]phenyl}(3-fluoro-2-methylphenyl)methanol (1.694 g).
MS (ESI) m/z : 542 (M+H)⁺.
¹H-NMR (CDCl₃) δ: 1.26 (3H, t, J = 7.1 Hz), 1.38 (3H, d, J = 2.0 Hz), 2.80 (1H, dd, J = 16.5, 5.9 Hz), 3.10 (1H, dd, J = 16.5, 7.8 Hz), 3.58 (3H, s), 3.76 (3H, s), 4.10-4.19 (2H, m), 4.45 (1H, dd, J = 7.8, 5.9 Hz), 4.85 (1H, d, J = 14.5 Hz), 5.50 (1H, d, J = 14.5 Hz), 5.58 (1H, s), 6.32 (1H, d, J = 2.5 Hz), 6.41 (2H, dd, J = 7.6, 2.2 Hz), 7.01 (1H, t, J = 8.6 Hz), 7.20-7.26 (2H, m), 7.32-7.38 (2H, m), 7.41 (1H, t, J = 8.5 Hz).

### Referential Example 663

Ethyl [(trans)-7-chloro-5-(3-fluoro-2-methylphenyl)-2-oxo-1,2,3,5-tetrahydro-4,1-benzoxazepin-3-yl]acetate

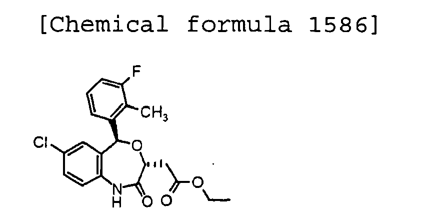

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (886 mg) was obtained from ethyl [(trans)-7-chloro-1-(2,4-dimethoxybenzyl)-5-(3-fluoro-2-methylphenyl)-2-oxo-1,2,3,5-tetrahydro-4,1-benzoxazepin-3-yl]acetate (1.54 g).
MS (ESI) m/z : 392 (M+H)⁺.
¹H-NMR (CDCl₃) δ: 1.23 (3H, t, J = 6.9 Hz), 2.02 (3H, d, J = 2.0 Hz), 2.80 (1H, dd, J = 16.4, 6.6 Hz), 3.02 (1H, dd, J = 16.4, 6.4 Hz), 4.05-4.19 (2H, m), 4.57 (1H, t, J = 6.5 Hz), 6.03 (1H, s), 6.66 (1H, d, J = 2.5 Hz), 7.01 (1H, d, J = 8.6 Hz), 7.08 (1H, t, J = 8.6 Hz), 7.17-7.26 (2H, m), 7.32 (1H, dd, J = 8.6, 2.5 Hz), 7.85 (1H, s).

### Referential Example 664

Ethyl [(trans)-7-chloro-5-(3-fluoro-2-methylphenyl)-2-thioxo-1,2,3,5-tetrahydro-4,1-benzoxazepin-3-yl]acetate

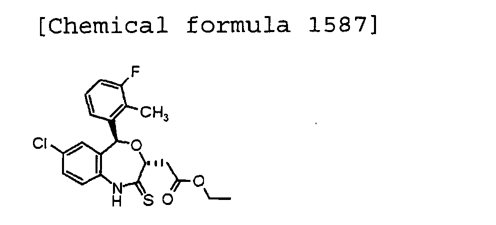

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (847 mg) was obtained from ethyl [(trans)-7-chloro-5-(3-fluoro-2-methylphenyl)-2-oxo-1,2,3,5-tetrahydro-4,1-benzoxazepin-3-yl] acetate (883 mg).
MS (ESI) m/z : 408 (M+H)⁺.
¹H-NMR (CDCl₃) δ: 1.25 (3H, t, J = 7.1 Hz), 1.94 (3H, d, J = 2.0 Hz), 2.97 (1H, dd, J = 16.4, 6.6 Hz), 3.26 (1H, dd, J = 16.5, 6.5 Hz), 4.14 (2H, q, J = 7.1 Hz), 4.70 (1H, t, J = 6.5 Hz), 5.93 (1H, s), 6.61 (1H, d, J = 2.2 Hz), 7.05-7.12 (2H, m), 7.24-7.33 (2H, m), 7.38 (1H, dd, J = 8.5, 2.3 Hz), 9.59 (1H, s).

### Example 758

Ethyl [(trans)-8-chloro-6-(3-fluoro-2-methylphenyl)-1-(trifluoromethyl)-4H,6H-[1,2,4]triazolo[4,3-a][4,1]benzoxazepin-4-yl]acetate

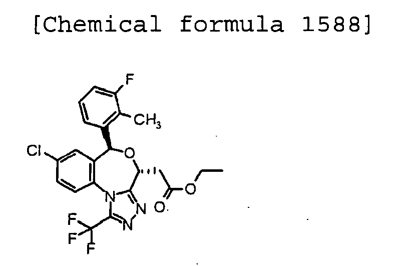

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (594 mg) was obtained from ethyl [(trans)-7-chloro-5-(3-fluoro-2-methylphenyl)-2-thioxo-1,2,3,5-tetrahydro-4,1-benzoxazepin-3-yl]acetate (480 mg).
MS (ESI) m/z . 484 (M+H)⁺.
¹H-NMR (CDCl₃) δ: 1.29 (3H, t, J = 7.1 Hz), 1.73 (3H, d, J = 1.7 Hz), 3.26 (1H, dd, J = 16.7, 6.8 Hz), 3.50 (1H, dd, J = 16.7, 6.8 Hz), 4.16-4.25 (2H, m), 4.97 (1H, t, J = 6.8 Hz), 5.36 (1H, s), 6.75 (1H, d, J = 1.7 Hz), 7.10 (1H, t, J = 8.6 Hz), 7.28-7.35 (1H, m), 7.44 (1H, d, J = 7.6 Hz), 7.54-7.59 (2H, m).

### Example 759

[(trans)-8-Chloro-6-(3-fluoro-2-methylphenyl)-1-(trifluoromethyl)-4H,6H-[1,2,4]triazolo[4,3-a] [4,1]benzoxazepin-4-yl]acetic acid

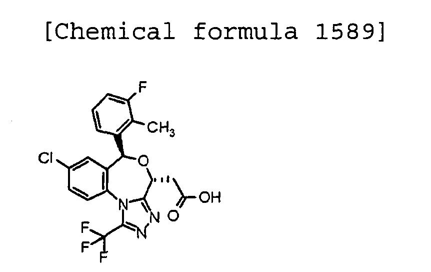

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (110 mg) was obtained from ethyl [(trans)-8-chloro-6-(3-fluoro-2-methylphenyl)-1-(trifluoromethyl)-4H,6H-[1,2,4]triazolo[4,3-a][4,1]benzoxazepin-4-yl]acetate (128 mg) .
MS (ESI) m/z : 456 (M+H)⁺.
¹H-NMR (CDCl₃) δ: 1.72 (3H, d, J = 1.5 Hz), 3.33 (1H, dd, J = 17.3, 6.7 Hz), 3.58 (1H, dd, J = 17.3, 6.7 Hz), 4.94 (1H, t, J = 6.7 Hz), 5.37 (1H, s), 6.77 (1H, d, J = 2.0 Hz), 7.10 (1H, t, J = 8.8 Hz), 7.29-7.37 (1H, m), 7.45 (1H, d, J = 7.6 Hz), 7.55-7.61 (2H, m).
Elemental analysis for: C₂₀H₁₄ClF₄N₃O₃
Calculated: C, 52.70; H, 3.10; N, 9.22; Cl, 7.78; F, 16.67.
Found: C, 52.64; H, 3.03; N, 9.08; Cl, 7.64; F, 16.55.

### Referential Example 665

{5-Chloro-2-[(2,4-dimethoxybenzyl)amino]phenyl}(2,3-dimethylphenyl)methanol

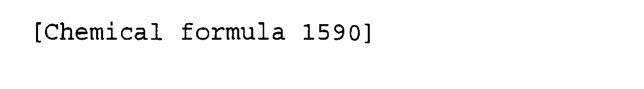

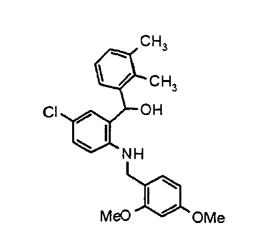

In a similar manner to that employed for the synthesis of {5-bromo-2-[(2,4-dimethoxybenzyl)amino]phenyl}(2-chloro-3-methoxyphenyl)methanol, the title compound (691 mg) was obtained from (2-amino-5-chlorophenyl)(2,3-dimethylphenyl)methanol (2.77 g) synthesized in accordance with the process described in the document (J. Org. Chem. 68, 92-103).
MS (ESI) m/z : 412 (M+H)⁺.

### Referential Example 666

Ethyl [(trans)-7-chloro-1-(2,4-dimethoxybenzyl)-5-(2,3-dimethylphenyl)-2-oxo-1,2,3,5-tetrahydro-4,1-benzoxazepin-3-yl] acetate

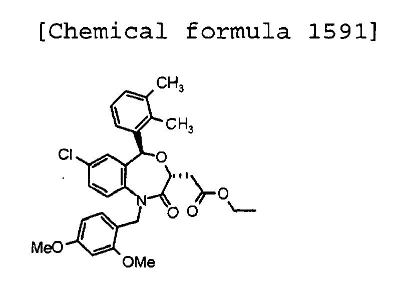

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (523 mg) was obtained from {5-chloro-2-[(2,4-dimethoxybenzyl)amino]phenyl}(2,3-dimethylphenyl)methanol (688 mg).
MS (ESI) m/z : 538 (M+H)⁺.
¹H-NMR (CDCl₃) δ: 1.26 (3H, t, J = 7.1 Hz), 1.54 (3H, s), 2.21 (3H, s), 2.80 (1H, dd, J = 16.4, 5.9 Hz), 3.10 (1H, dd, J = 16.4, 7.8 Hz), 3.59 (3H, s), 3.77 (3H, s), 4.08-4.20 (2H, m), 4.46 (1H, dd, J = 7.8, 5.8 Hz), 4.87 (1H, d, J = 14.7 Hz), 5.48 (1H, d, J = 14.7 Hz), 5.65 (1H, s), 6.33 (1H, d, J = 2.2 Hz), 6.41 (1H, dd, J = 8.3, 2.5 Hz), 6.45 (1H, d, J = 2.5 Hz), 7.13 (1H, dd, J = 7.4, 2.0 Hz), 7.17 (1H, t, J = 7.4 Hz), 7.32 (1H, dd, J = 8.6, 2.5 Hz), 7.36-7.42 (3H, m).

### Referential Example 667

Ethyl [(trans)-7-chloro-5-(2,3-dimethylphenyl)-2-oxo-1,2,3,5-tetrahydro-4,1-benzoxazepin-3-yl]acetate

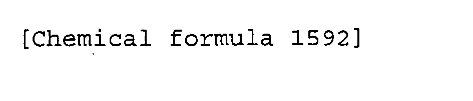

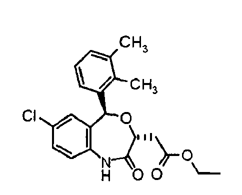

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (274 mg) was obtained from ethyl [(trans)-7-chloro-1-(2,4-dimethoxybenzyl)-5-(2,3-dimethylphenyl)-2-oxo-1,2,3,5-tetrahydro-4,1-benzoxazepin-3-yl]acetate (521 mg).
MS (ESI) m/z : 388 (M+H)⁺.
¹H-NMR (CDCl₃) δ: 1.22 (3H, t, J = 7.4 Hz)., 2.03 (3H, s), 2.31 (3H, s), 2.79 (1H, dd, J = 16.4, 6.4 Hz), 2.99 (1H, dd, J = 16.4, 6.4 Hz), 4.04-4.18 (2H, m), 4.54 (1H, t, J = 6.4 Hz), 6.09 (1H, s), 6.73 (1H, d, J = 2.5 Hz), 6.97 (1H, d, J = 8.6 Hz), 7.11-7.21 (3H, m), 7.29 (1H, dd, J = 8.6, 2.5 Hz), 7.69 (1H, s).

### Referential Example 668

Ethyl [(trans)-7-chloro-5-(2,3-dimethylphenyl)-2-thioxo-1,2,3,5-tetrahydro-4,1-benzoxazepin-3-yl]acetate

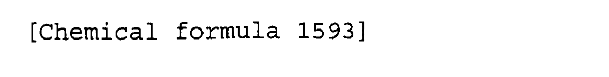

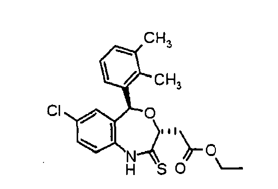

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (279 mg) was obtained from ethyl [(trans)-7-chloro-5-(2,3-dimethylphenyl)-2-oxo-1,2,3,5-tetrahydro-4,1-benzoxazepin-3-yl]acetate (270 mg).
MS (ESI) m/z : 404 (M+H)⁺.
¹H-NMR (CDCl₃) δ: 1.24 (3H, t, J = 7.1 Hz), 1.94 (3H, s), 2.30 (3H, s), 2.96 (1H, dd, J = 16.4, 6.5 Hz), 3.25 (1H, dd, J = 16.4, 6.5 Hz), 4.13 (2H, q, J = 7.1 Hz), 4.69 (1H, t, J = 6.5 Hz), 5.99 (1H, s), 6.67 (1H, d, J = 2.3 Hz), 7.07 (1H, d, J = 8.3 Hz), 7.18 (1H, d, J = 2.7 Hz), 7.20 (1H, s), 7.29-7.33 (1H, m), 7.35 (1H, dd, J = 8.3, 2.3 Hz), 9.52 (1H, s).

### Example 760

Ethyl [(trans)-8-chloro-6-(2,3-dimethylphenyl)-1-(trifluoromethyl)-4H,6H-[1,2,4]triazolo[4,3-a][4,1]benzoxazepin-4-yl]acetate

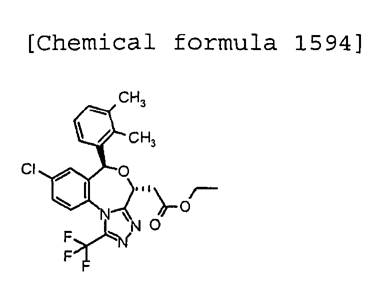

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (308 mg) was obtained from ethyl [(trans)-7-chloro-5-(2,3-dimethylphenyl)-2-thioxo-1,2,3,5-tetrahydro-4,1-benzoxazepin-3-yl]acetate (276 mg).
MS (ESI) m/z : 480 (M+H)⁺.
¹H-NMR (CDCl₃) δ: 1.29 (3H, t, J = 6.9 Hz), 1.69 (3H, s), 2.26 (3H, s), 3.25 (1H, dd, J = 16.8, 6.8 Hz), 3.49 (1H, dd, J = 16.8, 6.8 Hz), 4.14-4.24 (2H, m), 4.96 (1H, t, J = 6.8 Hz), 5.41 (1H, s), 6.79 (1H, s), 7.18-7.26 (2H, m), 7.49 (1H, d, J = 7.6 Hz), 7.54-7.55 (2H, m).

### Example 761

Ethyl [(4R,6R)-8-chloro-6-(2,3-dimethylphenyl)-1-(trifluoromethyl)-4H,6H-[1,2,4]triazolo[4,3-a][4,1]benzoxazepin-4-yl]acetate (isomer A)

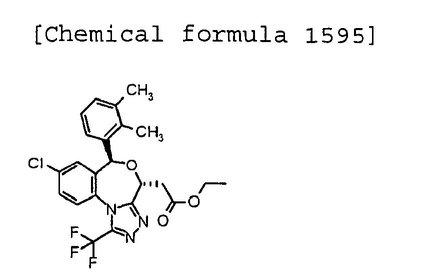

Ethyl [(4S,6S)-8-chloro-6-(2,3-dimethylphenyl)-1-(trifluoromethyl)-4H,6H-[1,2,4]triazolo[4,3-a][4,1]benzoxazepin-4-yl]acetate (isomer B)

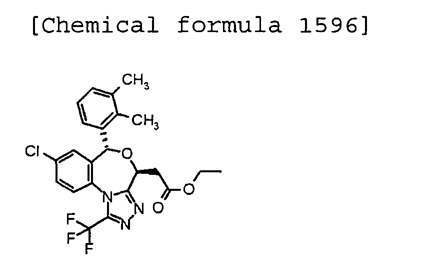

Ethyl [(trans)-8-chloro-6-(2,3-dimethylphenyl)-1-(trifluoromethyl)-4H,6H-[1,2,4]triazolo[4,3-a][4,1]benzoxazepin-4-yl]acetate (308 mg, 0.642 mmol) was optically resolved by an optically active column (CHIRALPAK-OD) into ethyl [(4R,6R)-8-chloro-6-(2,3-dimethylphenyl)-1-(trifluoromethyl)-4H,6H-[1,2,4]triazolo[4,3-a][4,1]benzoxazepin-4-yl]acetate (isomer A, 149 mg) and ethyl [(4S,6S)-8-chloro-6-(2,3-dimethylphenyl)-1-(trifluoromethyl)-4H,6H-[1,2,4]triazolo[4,3-a][4,1]benzoxazepin-4-yl]acetate (isomer B, 152 mg).
Resolution conditions:
Flow rate: 50 mL/min
Developing solvent: 15% isopropanol- n-hexane
Retention time: isomer A: 40 min., isomer B: 50 min.

### Example 762

[(4R,6R)-8-Chloro-6-(2,3-dimethylphenyl)-1-(trifluoromethyl)-4H,6H-[1,2,4]triazolo[4,3-a][4,1]benzoxazepin-4-yl]acetic acid

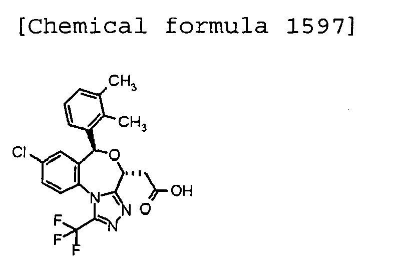

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (112 mg) was obtained from ethyl [(4R,6R)-8-chloro-6-(2,3-dimethylphenyl)-1-(trifluoromethyl)-4H,6H-[1,2,4]triazolo[4,3-a][4,1]benzoxazepin-4-yl]acetate (149 mg).
MS (ESI) m/z : 452 (M+H)⁺.
¹H-NMR (CDCl₃) δ: 1.69 (3H, s), 2.26 (3H, s), 3.31 (1H, dd, J = 17.2, 6.6 Hz), 3.57 (1H, dd, J = 17.2, 6.6 Hz), 4.93 (1H, t, J = 6.6 Hz), 5.41 (1H, s), 6.80 (1H, s), 7.18-7.25 (2H, m), 7.50 (1H, d, J = 7.1 Hz), 7.55 (2H, s).
Elemental analysis for: C₂₁H₁₇ClF₃N₃O₃·0.75 dioxane
Calculated: C, 55.18; H, 4.53; N, 8.04; Cl, 6.79; F, 10.91.
Found: C, 55.32; H, 4.39; N, 7.72; Cl, 6.77; F, 10.77.

### Example 763

[(4S,6S)-8-Chloro-6-(2,3-dimethylphenyl)-1-(trifluoromethyl)-4H,6H-[1,2,4]triazolo[4,3-a] [4, 1] benzoxazepin-4-yl] acetic acid

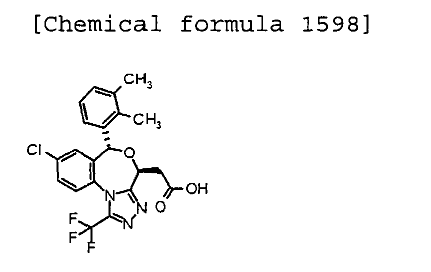

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (107 mg) was obtained from ethyl [(4S,6S)-8-chloro-6-(2,3-dimethylphenyl)-1-(trifluoromethyl)-4H,6H-[1,2,4]triazolo[4,3-a] [4,1]benzoxazepin-4-yl]acetate (152 mg) .
MS (ESI) m/z : 452 (M+H)⁺.
¹H-NMR (CDCl₃) δ: 1.69 (3H, s), 2.26 (3H, s), 3.31 (1H, dd, J = 17.2, 6.6 Hz), 3.57 (1H, dd, J = 17.2, 6.6 Hz), 4.93 (1H, t, J =6.6 Hz), 5.41 (1H, s), 6.80 (1H, s), 7.18-7.25 (2H, m), 7.50 (1H, d, J = 7.1 Hz), 7.55 (2H, s).
Elemental analysis for: C₂₁H₁₇ClF₃N₃O₃·0.25 dioxane Calculated: C, 55.76; H, 4.04; N, 8.87; Cl, 7.48; F, 12.02.
Found: C, 55.48; H, 3.97; N, 8.63; Cl, 7.63; F, 11.80.

### Referential Example 669

(2-Amino-4-fluorophenyl)(2,3-dimethoxyphenyl)methanone

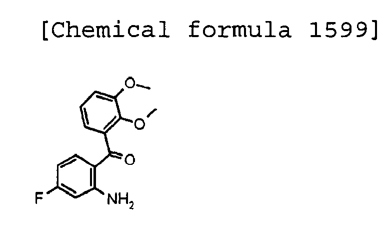

1,2-Dimethoxybenzene (1.30 g) was dissolved in tetrahydrofuran (20 mL). To the resulting solution was added dropwise n-butyl lithium (a 2.55M solution, 1.42 mL) at from -10°C to -15°C. The resulting mixture was stirred at room temperature for 30 minutes. At -78°C, a solution of 7-fluoro-2-methyl-4H-3,1-benzoxazin-4-one (1.68 g) in tetrahydrofuran (15 mL) synthesized in accordance with the process described in the patent (EP0360417) was added dropwise to the reaction mixture. The resulting mixture was warmed to room temperature and then, stirred for one hour. After the reaction mixture was concentrated, ethanol (10 mL), concentrated hydrochloric acid (5 mL) and water (5 mL) were added to the residue. The resulting mixture was stirred at 90°C for 2 hours. After the reaction mixture was concentrated, a saturated aqueous solution of sodium bicarbonate was added to the residue, followed by extraction with ethyl acetate. The organic layer was washed with saturated brine and dried over anhydrous sodium sulfate. The filtrate was then concentrated. The residue was purified by silica gel column (ethyl acetate:hexane=1:4) to give the title compound (1.07 g).
MS (ESI) m/z : 276 (M+H)⁺.
¹H-NMR (CDCl₃) δ: 3.77 (3H, s), 3.91 (3H, s), 6.23 (1H, td, J = 8.5, 2.5 Hz), 6.35 (1H, dd, J = 10.9, 2.3 Hz), 6.55 (2H, br s), 6.82 (1H, dd, J = 7.6, 1.4 Hz), 7.00 (1H, dd, J = 8.1, 1.4 Hz), 7.11 (1H, t, J = 7.8 Hz), 7.29 (1H, dd, J = 9.1, 6.6 Hz).

### Referential Example 670

(2-Amino-4-fluorophenyl)(2,3-dimethoxyphenyl)methanol

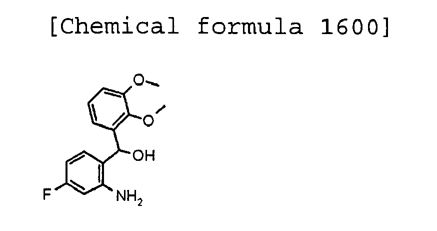

In a similar manner to that employed for the synthesis of (2-amino-5-bromophenyl)(2-chloro-3-methoxyphenyl)methanol, the title compound (890 mg) was obtained from (2-amino-4-fluorophenyl)(2,3-dimethoxyphenyl)methanone (987 mg).
MS (ESI) m/z : 260 (M-18+H)⁺.
¹H-NMR (CDCl₃) δ: 1.54 (2H, br s); 3.82 (3H, s), 3.89 (3H, s), 4.36 (1H, br s), 6.04 (1H, s), 6.33-6.39 (2H, m), 6.86 (1H, dd, J = 7.8, 1.5 Hz), 6.88-6.97 (2H, m), 7.06 (1H, t, J = 8.1 Hz).

### Referential Example 671

{2-[(2,4-Dimethoxybenzyl)amino]-4-fluorophenyl}(2,3-dimethoxyphenyl)methanol

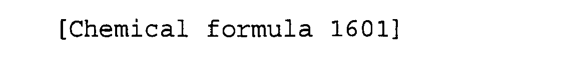

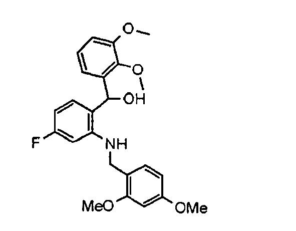

In a similar manner to that employed for the synthesis of {5-bromo-2-[(2,4-dimethoxybenzyl)amino]phenyl}(2-chloro-3-methoxyphenyl)methanol, the title compound (552 mg) was obtained from (2-amino-4-fluorophenyl)(2,3-dimethoxyphenyl)methanol (889 mg).
MS (ESI) m/z : 428 (M+H)⁺.
¹H-NMR (CDCl₃) δ: 3.75 (3H, s), 3.79 (3H, s), 3.80 (3H, s), 3.88 (3H, s), 4.24 (2H, s), 6.01 (1H, s), 6.26 (1H, td, J = 8.3, 2.4 Hz), 6.36-6.47 (3H, m), 6.81-6.86 (2H, m), 6.90 (1H, dd, J = 8.1, 1.5 Hz), 7.04 (1H, t, J = 8.0 Hz), 7.09 (1H, d, J = 8.6 Hz).

### Referential Example 672

Ethyl [(trans)-1-(2,4-dimethoxybenzyl)-5-(2,3-dimethoxyphenyl)-8-fluoro-2-oxo-1,2,3,5-tetrahydro-4,1-benzoxazepin-3-yl]acetate

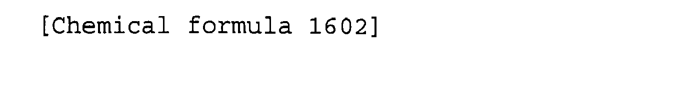

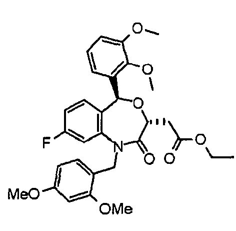

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (483 mg) was obtained from {2-[(2,4-dimethoxybenzyl)amino]-4-fluorophenyl}(2,3-dimethoxyphenyl)methanol (520 mg).
MS (ESI) m/z : 576 (M+Na)⁺.
¹H-NMR (CDCl₃) δ: 1.25 (3H, t, J = 7.1 Hz), 2.80 (1H, dd, J = 16.6, 5.9 Hz), 3.10 (1H, dd, J = 16.6, 7.7 Hz), 3.19 (3H, s), 3.69 (3H, s), 3.76 (3H, s), 3.84 (3H, s), 4.12-4.17 (2H, m), 4.50 (1H, dd, J = 7.7, 5.9 Hz), 4.87 (1H, d, J = 15.6 Hz), 5.49 (1H, d, J = 15.6 Hz), 5.97 (1H, s), 6.39-6.44 (2H, m), 6.55 (1H, dd, J = 8.6, 6.4 Hz), 6.72 (1H, td, J = 8.3, 2.5 Hz), 6.91 (1H, dd, J = 7.6, 2.0 Hz), 7.08-7.29 (4H, m).

### Referential Example 673

Ethyl [(trans)-1-(2,4-dimethoxybenzyl)-8-fluoro-2-oxo-1,2,3,5-tetrahydro-4,1-benzoxazepin-3-yl]acetate

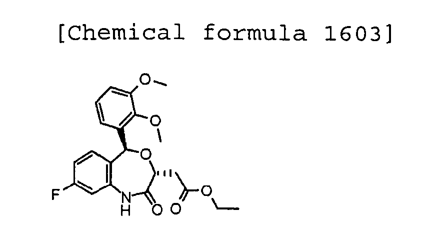

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (281 mg) was obtained from ethyl [(trans)-1-(2,4-dimethoxybenzyl)-5-(2,3-dimethoxyphenyl)-8-fluoro-2-oxo-1,2,3,5-tetrahydro-4,1-benzoxazepin-3-yl]acetate (482 mg).
MS (ESI) m/z : 404 (M+H)⁺.
¹H-NMR (CDCl₃) δ: 1.23 (3H, t, J = 7.1 Hz), 2.80 (1H, dd, J = 16.4, 6.7 Hz), 3.04 (1H, dd, J = 16.4, 6.7 Hz), 3.63 (3H, s), 3.88 (3H, s), 4.11 (2H, q, J = 7.1 Hz), 4.65 (1H, t, J = 6.7 Hz), 6.20 (1H, s), 6.67 (1H, dd, J = 9.3, 6.4 Hz), 6.73-6.79 (2H, m), 6.95 (1H, dd, J = 7.8, 1.9 Hz), 7.09 (1H, dd, J = 7.8, 1.9 Hz), 7.14 (1H, t, J = 7.8 Hz), 7.61 (1H, s) .

### Referential Example 674

Ethyl [(trans)-1-(2,4-dimethoxybenzyl)-8-fluoro-2-thioxo-1,2,3,5-tetrahydro-4,1-benzoxazepin-3-yl]acetate

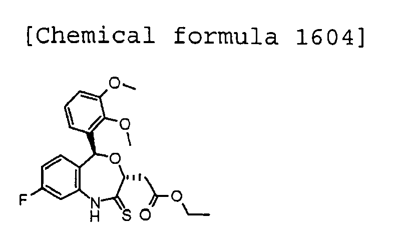

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (192 mg) was obtained from ethyl [(trans)-1-(2,4-dimethoxybenzyl)-8-fluoro-2-oxo-1,2,3,5-tetrahydro-4,1-benzoxazepin-3-yl] acetate (280 mg).
MS (ESI) m/z : 420(M+H)⁺.
¹H-NMR (CDCl₃) δ: 1.24 (3H, t, J = 7.4 Hz), 2.97 (1H, dd, J = 16.4, 6.6 Hz), 3.26 (1H, dd, J = 16.4, 6.6 Hz), 3.61 (3H, s), 3.87 (3H, s), 4.13 (2H, q, J = 7.4 Hz), 4.72 (1H, t, J - 6.6 Hz), 6.11 (1H, s), 6.67 (1H, dd, J = 8.8, 6.1 Hz), 6.84 (2H, d, J = 8.3 Hz), 6.95 (1H, dd, J = 7.1, 2.7 Hz), 7.12-7.18 (2H, m), 9.50 (1H, s).

### Example 764

Ethyl [(trans)-6-(2,3-dimethoxyphenyl)-9-fluoro-1-(trifluoromethyl)-4H,6H-[1,2,4]triazolo[4,3-a] [4,1]benzoxazepin-4-yl]acetate

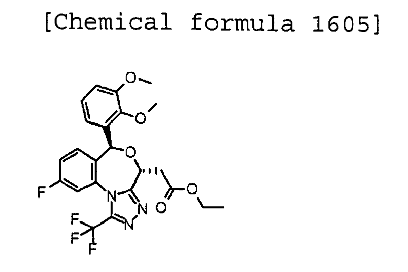

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (185 mg) was obtained from ethyl [(trans)-1-(2,4-dimethoxybenzyl)-8-fluoro-2-thioxo-1,2,3,5-tetrahydro-4,1-benzoxazepin-3-yl]acetate (191 mg).
MS (ESI) m/z : 496 (M+H)⁺.
¹H-NMR (CDCl₃) δ: 1.28 (3H, t, J = 7.2 Hz), 3.25 (1H, dd, J = 16.9, 6.8 Hz), 3.42 (3H, s), 3.50 (1H, dd, J = 16.9, 6.8 Hz), 3.84 (3H, s), 4.19 (2H, q, J = 7.2 Hz), 4.93 (1H, t, J = 6.8 Hz), 5.55.(1H, s), 6.89 (1H, dd, J = 8.8, 5.9 Hz), 6.96 (1H, dd, J = 8.0, 1.8 Hz), 7.09-7.23 (3H, m), 7.33 (1H, dd, J = 8.0, 2.0 Hz).

### Example 765

[(trans)-6-(2,3-Dimethoxyphenyl)-9-fluoro-1-(trifluoromethyl)-4H,6H-[1,2,4]triazolo[4,3-a][4,1]benzoxazepin-4-yl]acetic acid

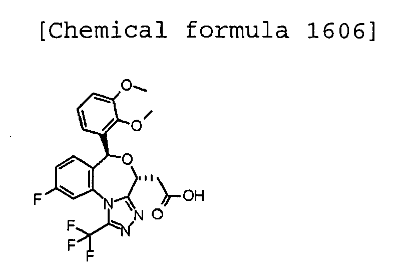

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the compound (84 mg) was obtained from ethyl [(trans)-6-(2,3-dimethoxyphenyl)-9-fluoro-1-(trifluoromethyl)-4H,6H-[1,2,4]triazolo[4,3-a][4,1]benzoxazepin-4-yl]acetate (182 mg).
MS (ESI) m/z : 468 (M+H)⁺.
¹H-NMR (CDCl₃) δ: 3.31 (1H, dd, J = 17.0, 6.7 Hz), 3.42 (3H, s), 3.58 (1H, dd, J = 17.0, 6.7 Hz), 3.85 (3H, s), 4.90 (1H, t, J = 6.7 Hz), 5.56 (1H, s), 6.91 (1H, dd, J = 8.8, 5.9 Hz), 6.97 (1H, dd, J = 8.0, 1.8 Hz), 7.13 (1H, td, J = 8.3, 2.5 Hz), 7.18 (1H, t, J = 7.8 Hz), 7.22 (1H, dd, J = 8.0, 1.8 Hz), 7.33 (1H, dd, J = 8.3, 2.5 Hz).
Elemental analysis for: C₂₁H₁₇F₄N₃O₅·0.25H₂O·0.25 dioxane
Calculated: C, 53.50; H, 3.97; N, 8.51; F, 15.37.
Found: C, 53.55; H, 3.71; N, 8.38; F, 15.10.

### Referential Example 675

(2-Amino-6-chlorophenyl)(2,3-dimethoxyphenyl)methanone

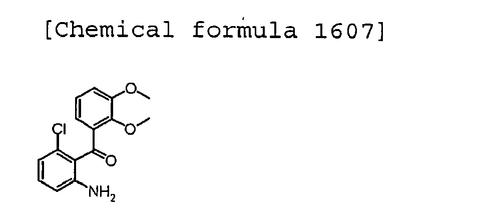

To a solution of 1,2-dimethoxybenzene (1.85 mL) in tetrahydrofuran (20.0 mL) were added N,N,N',N'-tetramethylethylenediamine (2.18 mL) and a 2.55M n-butyl lithium hexane solution (6.24 mL) at -78°C. The resulting mixture was stirred at room temperature for 2 hours. After cooling to -78°C again, a solution of 5-chloro-2-methyl-4H-3,1-benzoxazin-4-one (2.83 g) in tetrahydrofuran (45.0 mL) was added dropwise to the reaction mixture via cannula.
The resulting mixture was stirred at room temperature for 75 minutes. The reaction mixture was concentrated and the residue was dissolved in ethanol (20.0 mL) and water (5.00 mL). To the resulting solution was added concentrated hydrochloric acid (5.00 mL) at room temperature. After stirring overnight at 90°C, the reaction mixture was allowed to cool and then concentrated. Under ice cooling, the residue was neutralized with a saturated aqueous solution of sodium bicarbonate and was extracted with ethyl acetate. The organic layer was dried over anhydrous sodium sulfate. After filtration, the filtrate was concentrated and the residue thus obtained was purified by medium pressure column chromatography (hexane:ethyl acetate = from 9:1, 7:1, 6:1 to 5:1) to give the title compound (3.05 g). ¹H-NMR (CDCl₃) δ: 3.67 (3H, s), 3.87 (3H, s), 4.42 (2H, br s), 6.61-6.71 (2H, m), 7.03-7.15 (4H, m).
MS (ESI) m/z : 292 (M + H)⁺.

### Referential Example 676

(2-Amino-6-chlorophenyl)(2,3-dimethoxyphenyl)methanol

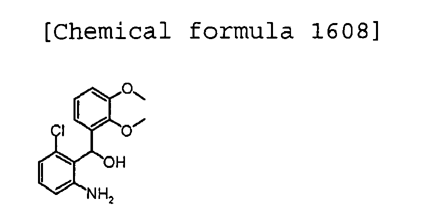

(2-Amino-6-chlorophenyl)(2,3-dimethoxyphenyl)methanone (3.05 g) was dissolved in methanol (47.0 mL). Under ice cooling, sodium borohydride (1.19 g) was added and the resulting mixture was stirred for 35 minutes. After stirring for further 2 hours at room temperature, the reaction mixture was quenched with water and distilled under reduced pressure to remove the solvent. To the residue was added water, followed by extraction with ethyl acetate. The organic layer was dried over anhydrous sodium sulfate. After filtration, the filtrate was concentrated to give the title compound (3.01 g). The compound was provided for the subsequent reaction without purification.
MS (ESI) m/z : 276 (M - OH)⁺.

### Referential Example 677

{2-Chloro-6-[(2,4-dimethoxybenzyl)amino]phenyl}(2,3-dimethoxyphenyl)methanol

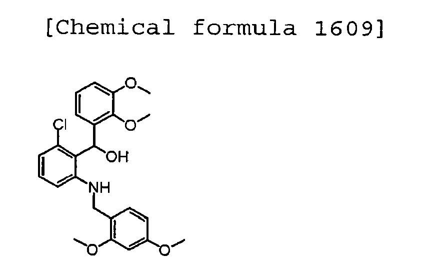

(2-Amino-6-chlorophenyl)(2,3-dimethoxyphenyl)methanol (3.01 g) was dissolved in acetic acid (75.0 mL). To the resulting solution was added 2,4-dimethoxybenzaldehyde (2.04 g) under ice cooling. The resulting mixture was stirred at room temperature for 45 minutes. After ice cooling again, sodium borohydride (0.58 g) was added and the resulting mixture was stirred at room temperature for 2.5 hours. The reaction mixture was concentrated. The residue was neutralized with a saturated aqueous solution of sodium bicarbonate and extracted with ethyl acetate. The organic layer was dried over anhydrous sodium sulfate. After filtration, the filtrate was concentrated and the residue thus obtained was purified by medium pressure column chromatography (hexane:ethyl acetate = from 11:1 to 6:1) to give the title compound (4.34 g).
¹H-NMR (CDCl₃) δ: 3.73 (3H, s), 3.77 (3H, s), 3.89 (3H, s), 3.92 (3H, s), 4.21 (2H, s), 6.31-6.37 (1H, m), 6.38-6.41 (1H, m), 6.51-6.61 (2H, m), 6.70 (1H, d, J = 8.0 Hz), 6.84 (1H, s), 6.86-6.95 (2H, m), 6.97-7.08 (2H, m).
MS (ESI) m/z : 444 (M + H)⁺.

### Referential Example 678

Ethyl (2E)-4-[{3-chloro-2-[(2,3-dimethoxyphenyl)(hydroxy)methyl]phenyl}(2,4-dimethoxybenzyl)amino]-4-oxobut-2-enoate

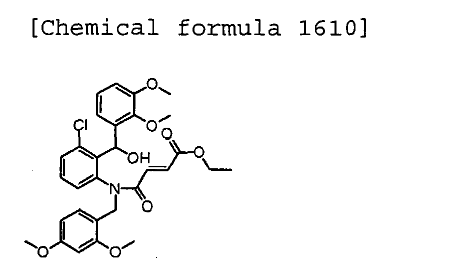

To a dichloromethane solution (80.0 mL) of {2-chloro-6-[(2,4-dimethoxybenzyl)amino]phenyl}(2,3-dimethoxyphenyl)methanol (4.34 g) were added sodium hydrogen carbonate (2.46 g) and a dichloromethane solution (10.0 mL) of ethyl (2E)-4-chloro-4-oxobut-2-enoate (1.91 g) under ice cooling. The resulting mixture was stirred overnight at room temperature. To the reaction mixture was added water, followed by extraction with dichloromethane. The organic layer was dried over anhydrous sodium sulfate. After filtration, the filtrate was concentrated to give the title compound (5.89 g, crude). The compound was provided for the subsequent reaction without purification.

### Referential Example 679

Ethyl [(trans)-6-chloro-1-(2,4-dimethoxybenzyl)-5-(2,3-dimethoxyphenyl)-2-oxo-1,2,3,5-tetrahydro-4,1-benzoxazepin-3-yl]acetate

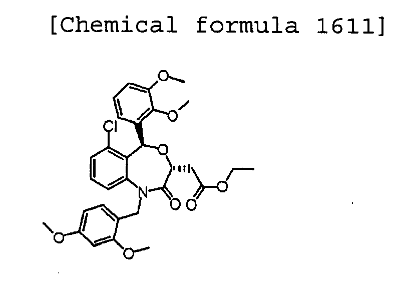

To an ethanol solution (110 mL) of ethyl (2E)-4-[{3-chloro-2-[(2,3-dimethoxyphenyl)(hydroxy)methyl]phenyl}(2,4-dimethoxybenzyl)amino]-4-oxobut-2-enoate (5.89 g, crude) was added potassium carbonate (2.03 g) at room temperature. The resulting mixture was stirred for 5 days. After potassium carbonate was filtered off, the residue obtained by concentrating the filtrate was dissolved in ethyl acetate, followed by washing with water. The organic layer was dried over anhydrous sodium sulfate. After filtration, the filtrate was concentrated and the residue thus obtained was purified by medium pressure column chromatography (hexane:ethyl acetate = from 9:1 to 3:1) to give the title compound (2.96 g).
¹H-NMR (CDCl₃) δ: 1.21-1.29 (3H, m), 2.87 (1H, dd, J = 16.6, 5.6 Hz), 3.17-3.27 (4H, m), 3.67 (3H, s), 3.75 (3H, s), 3.82 (3H, s), 4.08-4.25 (3H, m), 4.57-4.65 (2H, m), 6.25-6.30 (1H, m), 6.31-6.34 (1H, m), 6.60 (1H, d, J = 8.5 Hz), 6.79-6.85 (2H, m), 6.98-7.06 (3H, m), 7.13 (1H, t, J = 8.0 Hz), 7.32-7.36 (1H, m).
MS (ESI) m/z : 570 (M + H)⁺.

### Referential Example 680

Ethyl [(trans)-6-chloro-5-(2,3-dimethoxyphenyl)-2-oxo-1,2,3,5-tetrahydro-4,1-benzoxazepin-3-yl]acetate

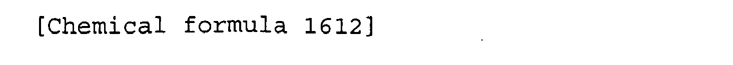

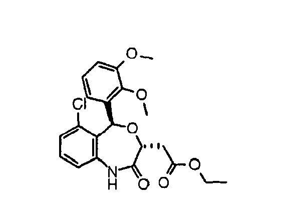

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (1.17 g) was obtained from ethyl [(trans)-6-chloro-1-(2,4-dimethoxybenzyl)-5-(2,3-dimethoxyphenyl)-2-oxo-1,2,3,5-tetrahydro-4,1-benzoxazepin-3-yl]acetate (1.85 g).
MS (ESI) m/z : 420 (M+H)⁺.
¹H-NMR (CDCl₃) δ: 1.30 (3H, t, J = 7.1 Hz), 2.80 (1H, dd, J = 17.2, 8.2 Hz), 3.02 (1H, dd, J = 17.2, 5.4 Hz), 3.79 (3H, s), 3.84 (3H, s), 4.14-4.27 (2H, m), 4.85 (1H, dd, J = 8.2, 5.4 Hz), 6.52 (1H, dd, J = 7.7, 1.6 Hz), 6.78 (1H, s), 6.84-6.89 (2H, m), 6.93 (1H, t, J = 8.0 Hz), 7.15 (1H, dd, J = 8.0, 1.3 Hz), 7.21 (1H, t, J = 8.0 Hz), 7.75 (1H, s).

### Referential Example 681

Ethyl [(trans)-6-chloro-5-(2,3-dimethoxyphenyl)-2-thioxo-1,2,3,5-tetrahydro-4,1-benzoxazepin-3-yl]acetate

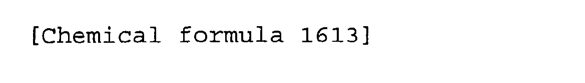

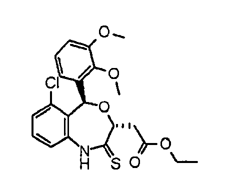

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (236 mg) was obtained from ethyl [(trans)-6-chloro-5-(2,3-dimethoxyphenyl)-2-oxo-1,2,3,5-tetrahydro-4,1-benzoxazepin-3-yl]acetate (1.17 g).
¹H-NMR (CDCl₃) δ: 1.31 (3H, t, J = 7.1 Hz), 2.96 (1H, dd, J = 17.2, 8.6 Hz), 3.26 (1H, dd, J = 17.2, 4.7 Hz), 3.79 (3H, s), 3.84 (3H, s), 4.14-4.30 (2H, m), 4.92 (1H, dd, J = 8.6, 4.7 Hz), 6.53 (1H, dd, J = 7.8, 1.7 Hz), 6.80 (1H, s), 6.85 (1H, dd, J = 8.1, 1.7 Hz), 6.89-6.96 (2H, m), 7.18 (1H, dd, J = 7.8, 1.7 Hz), 7.23 (1H, d, J = 7.8 Hz), 9.22 (1H, s).

### Example 766

Ethyl [(trans)-7-chloro-6-(2,3-dimethoxyphenyl)-1-(trifluoromethyl)-4H,6H-[1,2,4]triazolo[4,3-a][4,1]benzoxazepin-4-yl]acetate

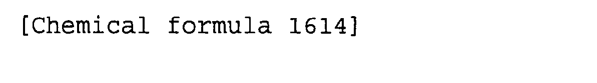

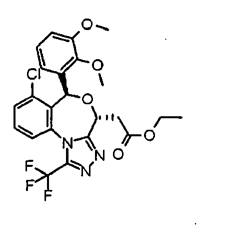

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (150 mg) was obtained from ethyl [(trans)-6-chloro-5-(2,3-dimethoxyphenyl)-2-thioxo-1,2,3,5-tetrahydro-4,1-benzoxazepin-3-yl]acetate (232 mg).
MS (ESI) m/z : 512 (M+H)⁺.
¹H-NMR (CDCl₃) δ: 1.29 (3H, t, J = 7.1 Hz), 3.28 (3H, s), 3.34 (1H, dd, J = 16.5, 6.9 Hz), 3.64 (1H, dd, J = 16.5, 6.9 Hz), 3.72 (3H, s), 4.08-4.28 (2H, m), 4.92 (1H, t, J = 6.9 Hz), 6.48 (1H, d, J = 7.8 Hz), 6.70 (1H, d, J = 7.8 Hz), 6.79 (1H, t, J = 7.8 Hz), 6.84 (1H, s), 7.26 (1H, d, J = 8.1 Hz), 7.46 (1H, t, J = 8.1 Hz), 7.71 (1H, d, J = 8.1 Hz).

### Example 767

[(trans)-7-Chloro-6-(2,3-dimethoxyphenyl)-1-(trifluoromethyl)-4H,6H-[1,2,4]triazolo[4,3-a] [4,1]benzoxazepin-4-yl]acetic acid

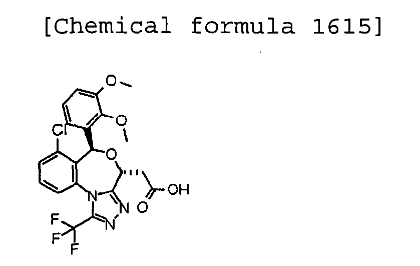

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (55 mg) was obtained from ethyl [(trans)-7-chloro-6-(2,3-dimethoxyphenyl)-1-(trifluoromethyl)-4H,6H-[1,2,4]triazolo[4,3-a][4,1]benzoxazepin-4-yl]acetate (147 mg).
MS (ESI) m/z : 484 (M+H)⁺.
¹H-NMR (CDCl₃) δ: 3.29 (3H, s), 3.41 (1H, dd, J = 17.0, 6.5 Hz), 3.70 (1H, dd, J = 17.0, 6.5 Hz), 3.72 (3H, s), 4.88 (1H, t, J = 6.5 Hz), 6.47 (1H, dt, J = 7.8, 1.2 Hz), 6.70 (1H, dd, J = 8.1, 1.2 Hz), 6.79 (1H, t, J = 8.1 Hz), 6.85 (1H, s), 7.27 (1H, d, J = 7.8 Hz), 7.47 (1H, d, J = 7.8 Hz), 7.72 (1H, dd, J = 8.1, 1.2 Hz).
Elemental analysis for: C₂₁H₁₇ClF₃N₃O₅·0.25H₂O·0.5 dioxane
Calculated: C, 51.89; H, 4.07; N, 7.89; Cl, 6.65; F, 10.70.
Found: C, 51.95; H, 3.77; N, 7.63; Cl, 6.73; F, 10.58.

### Referential Example 682

{5-Chloro-2-[(2,4-dimethoxybenzyl)amino]phenyl}(2-ethyl-3-methoxyphenyl)methanol

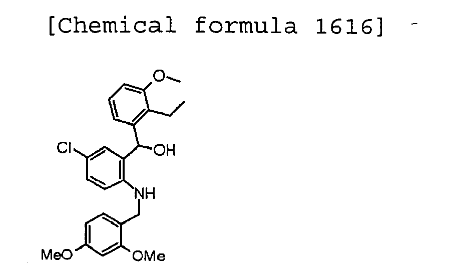

In a similar manner to that employed for the synthesis of {5-bromo-2-[(2,4-dimethoxybenzyl)amino]phenyl}(2-chloro-3-methoxyphenyl)methanol, the title compound (781 mg) was obtained from (2-amino-5-chlorophenyl)(2-ethyl-3-methoxyphenyl)methanol (584 mg).
MS (ESI) m/z : 442 (M+H)⁺.
¹H-NMR (CDCl₃) δ: 1.00 (3H, t, J = 7.5 Hz), 2.08 (1H, br s), 2.49-2.59 (1H, m), 2.61-2.72 (1H, m), 3.74 (3H, s), 3.79 (3H, s), 3.85 (3H, s), 4.23 (2H, s), 4.78 (1H, s), 6.01 (1H, s), 6.39 (1H, dd, J = 8.0, 2.5 Hz), 6.43 (1H, d, J = 2.5 Hz), 6.63 (1H, d, J = 8.6 Hz), 6.85-6.90 (2H, m), 6.98 (1H, dd, J = 7.8, 1.0 Hz), 7.03 (1H, d, J = 8.3 Hz), 7.10 (1H, dd, J = 8.6, 2.5 Hz), 7.21 (1H, t, J = 8.0 Hz).

### Referential Example 683

Ethyl [(trans)-7-chloro-1-(2,4-dimethoxybenzyl)-5-(2-ethyl-3-methoxyphenyl)-2-oxo-1,2,3,5-tetrahydro-4,1-benzoxazepin-3-yl] acetate

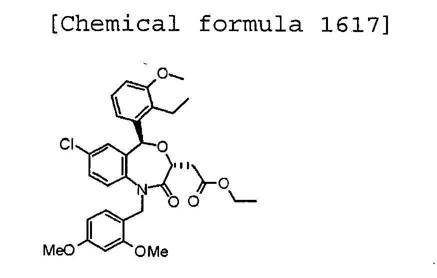

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (879 mg) was obtained from {5-chloro-2-[(2,4-dimethoxybenzyl)amino]phenyl}(2-ethyl-3-methoxyphenyl)methanol (780 mg).
MS (ESI) m/z : 568 (M+H)⁺.
¹H-NMR (CDCl₃) δ: 0.57 (3H, t, J = 7.4 Hz), 1.25 (3H, t, J = 7.1 Hz), 1.79-2.00 (2H, m), 2.79 (1H, dd, J = 16.7, 5.9 Hz), 3.10 (1H, dd, J = 16.5, 7.8 Hz), 3.60 (3H, s), 3.76 (3H, s), 3.81 (3H, s), 4.14 (2H, q, J = 7.1 Hz), 4.47 (1H, dd, J = 7.8, 5.9 Hz), 4.88 (1H, d, J = 14.5 Hz), 5.47 (1H, d, J = 14.5 Hz), 5.71 (1H, s), 6.35 (1H, d, J = 2.3 Hz), 6.42 (1H, dd, J = 8.5, 2.3 Hz), 6.49 (1H, d, J = 2.5 Hz), 6.85 (1H, d, J = 8.1 Hz), 7.15 (1H, d, J = 7.8 Hz), 7.23 (1H, d, J = 8.1 Hz), 7.31 (1H, dd, J = 8.5, 2.5 Hz), 7.34-7.41 (2H, m).

### Referential Example 684

Ethyl [(trans)-7-chloro-5-(2-ethyl-3-methoxyphenyl)-2-oxo-1,2,3,5-tetrahydro-4,1-benzoxazepin-3-yl]acetate

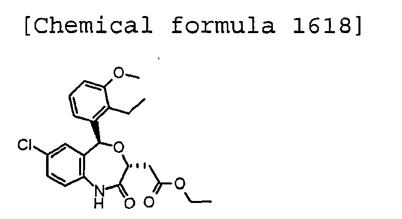

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (600 mg) was obtained from ethyl [(trans)-7-chloro-1-(2,4-dimethoxybenzyl)-5-(2-ethyl-3-methoxyphenyl)-2-oxo-1,2,3,5-tetrahydro-4,1-benzoxazepin-3-yl]acetate (877 mg).
MS (ESI) m/z : 418 (M+H)⁺.
¹H-NMR (CDCl₃) δ: 0.98 (3H, t, J = 7.5 Hz), 1.22 (3H, t, J = 7.2 Hz), 2.29-2.40 (1H, m), 2.52-2.62 (1H, m), 2.79 (1H, dd, J = 16.4, 6.5 Hz), 3.00 (1H, dd, J = 16.4, 6.5 Hz), 3.86 (3H, s), 4.05-4.16 (2H, m), 4.58 (1H, t, J = 6.5 Hz), 6.09 (1H, s), 6.71 (1H, d, J = 2.2 Hz), 6.90 (1H, d, J = 8.1 Hz), 6.95-7.01 (2H, m), 7.23 (1H, t, J = 8.1 Hz), 7.28 (1H, dd, J = 8.3, 2.5 Hz), 7.68 (1H, s).

### Referential Example 685

Ethyl [(trans)-7-chloro-5-(2-ethyl-3-methoxyphenyl)-2-thioxo-1,2,3,5-tetrahydro-4,1-benzoxazepin-3-yl]acetate

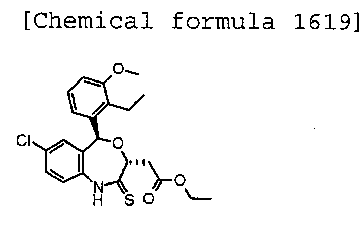

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (488 mg) was obtained from ethyl [(trans)-7-chloro-5-(2-ethyl-3-methoxyphenyl)-2-oxo-1,2,3,5-tetrahydro-4,1-benzoxazepin-3-yl] acetate (595 mg).
MS (ESI) m/z : 434 (M+H)⁺.
¹H-NMR (CDCl₃) δ: 0.92 (3H, t, J = 7.5 Hz), 1.24 (3H, t, J = 7.1 Hz), 2.20-2.32 (1H, m), 2.45-2.55 (1H, m), 2.95 (1H, dd, J = 16.4, 6.5 Hz), 3.26 (1H, dd, J = 16.4, 6.5 Hz), 3.85 (3H, s), 4.13 (2H, q, J = 7.1 Hz), 4.71 (1H, t, J = 6.5 Hz), 6.00 (1H, s), 6.68 (1H, d, J = 2.3 Hz), 6.91 (1H, d, J = 7.8 Hz), 7.05 (1H, d, J = 8.6 Hz), 7.09 (1H, d, J = 7.8 Hz), 7.24-7.28 (1H, m), 7.34 (1H, dd, J = 8.6, 2.3 Hz), 9.49 (1H, s).

### Example 768

Ethyl [(trans)-8-chloro-6-(2-ethyl-3-methoxyphenyl)-1-(trifluoromethyl)-4H,6H-[1,2,4]triazolo[4,3-a][4,1]benzoxazepin-4-yl]acetate

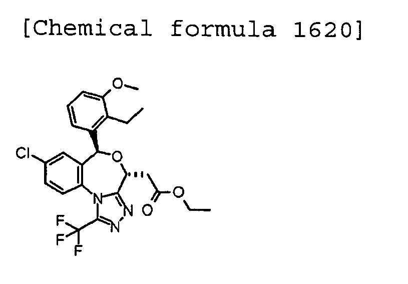

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (521 mg) was obtained from ethyl [(trans)-7-chloro-5-(2-ethyl-3-methoxyphenyl)-2-thioxo-1,2,3,5-tetrahydro-4,1-benzoxazepin-3-yl]acetate (487 mg).
MS (ESI) m/z : 510 (M+H)⁺.
¹H-NMR (CDCl₃) δ: 0.71 (3H, t, J = 7.6 Hz), 1.28 (3H, t, J = 7.1 Hz), 1.99-2.11 (1H, m), 2.19-2.31 (1H, m), 3.25 (1H, dd, J = 16.8, 6.7 Hz), 3.49 (1H, dd, J = 16.8, 6.7 Hz), 3.84 (3H, s), 4.15-4.23 (2H, m), 4.96 (1H, t, J = 6.7 Hz), 5.42 (1H, s), 6.85 (1H, s), 6.92 (1H, d, J = 8.1 Hz), 7.24 (1H, d, J = 8.1 Hz), 7.31 (1H, t, J = 8.0 Hz), 7.51-7.55 (2H, m).

### Example 769

Ethyl [(4R,6R)-8-chloro-6-(2-ethyl-3-methoxyphenyl)-1-(trifluoromethyl)-4H,6H-[1,2,4]triazolo[4,3-a][4,1]benzoxazepin-4-yl]acetate (isomer A)

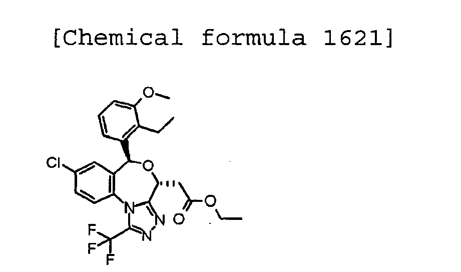

Ethyl [(4S,6S)-8-chloro-6-(2-ethyl-3-methoxyphenyl)-1-(trifluoromethyl)-4H,6H-[1,2,4]triazolo[4,3-a][4,1]benzoxazepin-4-yl]acetate (isomer B)

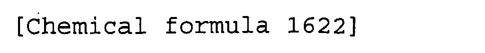

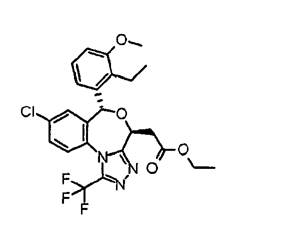

Ethyl [(trans)-8-chloro-6-(2-ethyl-3-methoxyphenyl)-1-(trifluoromethyl)-4H,6H-[1,2,4]triazolo[4,3-a] [4,1]benzoxazepin-4-yl]acetate (521 mg) was optically resolved by an optically active column (CHIRALPAK-AD) into ethyl [(4R,6R)-8-chloro-6-(2-ethyl-3-methoxyphenyl)-1-(trifluoromethyl)-4H,6H-[1,2,4]triazolo[4,3-a] [4,1]benzoxazepin-4-yl]acetate (isomer A, 234 mg) and ethyl [(4S,6S)-8-chloro-6-(2-ethyl-3-methoxyphenyl)-1-(trifluoromethyl)-4H,6H-[1,2,4]triazolo[4,3-a][4,1]benzoxazepin-4-yl]acetate (isomer B, 234 mg). Resolution conditions:
Flow rate: 50 mL/min
Developing solvent: 10% isopropanol- n-hexane
Retention time: isomer A: 32 min., isomer B: 40 min.

### Example 770

[(4R,6R)-8-Chloro-6-(2-ethyl-3-methoxyphenyl)-1-(trifluoromethyl)-4H,6H-[1,2,4]triazolo[4,3-a] [4,1]benzoxazepin-4-yl]acetic acid

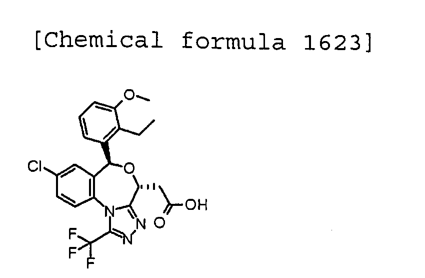

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (181 mg) was obtained from ethyl [(4R,6R)-8-chloro-6-(2-ethyl-3-methoxyphenyl)-1-(trifluoromethyl)-4H,6H-[1,2,4]triazolo[4,3-a][4,1]benzoxazepin-4-yl]acetate (232 mg).
MS (ESI) m/z : 482 (M+H)⁺.
¹H-NMR (CDCl₃) δ: 0.71 (3H, t, J = 7.5 Hz), 2.00-2.11 (1H, m), 2.20-2.32 (1H, m), 3.32 (1H, dd, J = 17.2, 6.7 Hz), 3.58 (1H, dd, J = 17.2, 6.7 Hz), 3.84 (3H, s), 4.93 (1H, t, J = 6.7 Hz), 5.43 (1H, s), 6.86 (1H, s), 6.92 (1H, d, J = 8.3 Hz), 7.25 (1H, d, J = 7.8 Hz), 7.32 (1H, t, J = 7.8 Hz), 7.51-7.57 (2H, m).
Elemental analysis for: C₂₂H₁₉ClF₃N₃O₄·0.25H₂O·0.25 dioxane
Calculated: C, 54.34; H, 4.26; N, 8.26; Cl, 6.97; F, 11.21.
Found: C, 54.26; H, 4.12; N, 8.09; Cl, 6.91; F, 11.07.

### Example 771

[(4S,6S)-8-Chloro-6-(2-ethyl-3-methoxyphenyl)-1-(trifluoromethyl)-4H,6H-[1,2,4]triazolo[4,3-a] [4, 1] benzoxazepin-4-yl] acetic acid

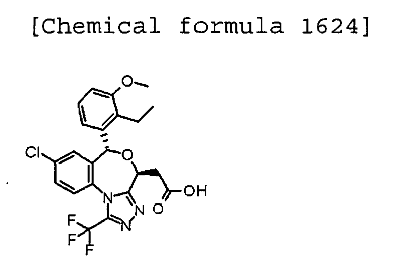

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (181 mg) was obtained from ethyl [(4S,6S)-8-chloro-6-(2-ethyl-3-methoxyphenyl)-1-(trifluoromethyl)-4H,6H-[1,2,4]triazolo[4,3-a][4,1]benzoxazepin-4-yl]acetate (232 mg).
MS (ESI) m/z : 482 (M+H)⁺.
¹H-NMR (CDCl₃) δ: 0.71 (3H, t, J = 7.5 Hz), 2.00-2.11 (1H, m), 2.20-2.32 (1H, m), 3.32 (1H, dd, J = 17.2, 6.7 Hz), 3.58 (1H, dd, J = 17.2, 6.7 Hz), 3.84 (3H, s), 4.93 (1H, t, J = 6.7 Hz), 5.43 (1H, s), 6.86 (1H, s), 6.92 (1H, d, J = 8.3 Hz), 7.25 (1H, d, J = 7.8 Hz), 7.32 (1H, t, J = 7.8 Hz), 7.51-7.57 (2H, m).
Elemental analysis for: C₂₂H₁₉ClF₃N₃O₄·0.25H₂O·0.25 dioxane Calculated: C, 54.34; H, 4.26; N, 8.26; Cl, 6.97; F, 11.21.
Found: C, 54.40; H, 4.18; N, 8.06; Cl, 6.96; F, 11.13.

### Referential Example 686

tert-Butyl {2-[(2-chloro-3-methoxyphenyl)(hydroxy)methyl]-4-fluorophenyl}carbamate

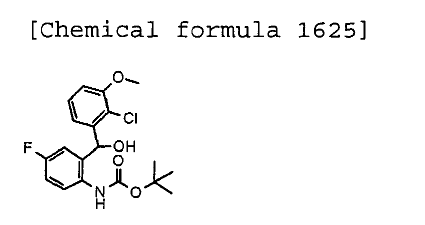

tert-Butyl (4-fluorophenyl)carbamate (4.04 g) was dissolved in tetrahydrofuran (80 mL). To the resulting solution was added dropwise sec-butyl lithium (a 1M solution, 50 mL) at -78°C. After stirring at the same temperature for 3 hours, a tetrahydrofuran solution of 2-chloro-3-methoxybenzaldehyde (5.0 g) was added dropwise at -78°C or less. After gradually warming to 0°C, the reaction mixture was stirred for one hour. A saturated aqueous solution of ammonium chloride was added to terminate the reaction. The reaction mixture was diluted with ethyl acetate and washed with water. The organic layer was washed with saturated brine and then, dried over anhydrous sodium sulfate. The filtrate was concentrated and the residue was purified by silica gel column (ethyl acetate:hexane= from 1:4 to 1:2) to give the title compound (4.47 g).
MS (ESI) m/z : 404 (M+Na)⁺.
¹H-NMR (CDCl₃) δ: 1.51 (9H, s), 3.50 (1H, br s), 3.91 (3H, s), 6.20 (1H, d, J = 3.7 Hz), 6.66 (1H, dd, J = 9.3, 2.9 Hz), 6.91-7.00 (2H, m), 7.09 (1H, s), 7.24-7.28 (1H, m), 7.33 (1H, t, J = 8.1 Hz), 7.56 (1H, dd, J = 8.8, 5.1 Hz).

### Referential Example 687

(2-Amino-5-fluorophenyl)(2-chloro-3-methoxyphenyl)methanol

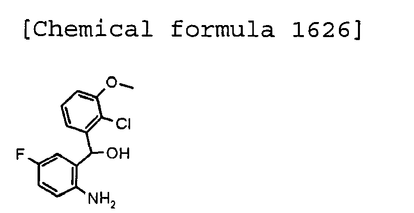

tert-Butyl {2-[(2-chloro-3-methoxyphenyl)(hydroxy)methyl]-4-fluorophenyl}carbamate (4.47 g) was dissolved in dioxane (20 mL). To the resulting solution were added concentrated hydrochloric acid (10 mL) and water (5 mL). The resulting mixture was stirred overnight at room temperature. In an ice water bath, the reaction mixture was made alkaline with a 5N aqueous sodium hydroxide solution, followed by extraction with chloroform. The organic layer was dried over anhydrous sodium sulfate. The filtrate was concentrated to give the title compound (3.52 g).

### Referential Example 688

(2-Chloro-3-methoxyphenyl){2-[(2,4-dimethoxybenzyl)amino]-5-fluorophenyl}methanol

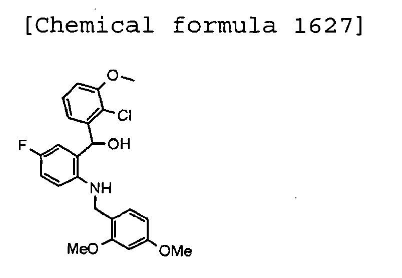

In a similar manner to that employed for the synthesis of {5-bromo-2-[(2,4-dimethoxybenzyl)amino]phenyl}(2-chloro-3-methoxyphenyl)methanol, the title compound (4.40 g) was obtained from (2-amino-5-fluorophenyl)(2-chloro-3-methoxyphenyl)methanol (3.52 g).
MS (ESI) m/z : 432 (M+H)⁺.
¹H-NMR (CDCl₃) δ: 2.67 (1H, br s), 3.80 (6H, s), 3.93 (3H, s), 4.26 (2H, s), 4.70 (1H, br s), 6.21 (1H, s), 6.41 (1H, dd, J = 8.3, 2.5 Hz), 6.46 (1H, d, J = 2.2 Hz), 6.60 (1H, dd, J = 9.8, 3.2 Hz), 6.68 (1H, dd, J = 8.8, 4.9 Hz), 6.87 (1H, td, J = 8.5, 3.0 Hz), 6.94 (1H, dd, J = 8.3, 1.5 Hz), 7.11 (2H, d, J = 8.3 Hz), 7.29 (1H, t, J = 8.1 Hz).

### Referential Example 689

Ethyl [(trans)-5-(2-chloro-3-methoxyphenyl)-1-(2,4-dimethoxybenzyl)-7-fluoro-2-oxo-1,2,3,5-tetrahydro-4,1-benzoxazepin-3-yl]acetate

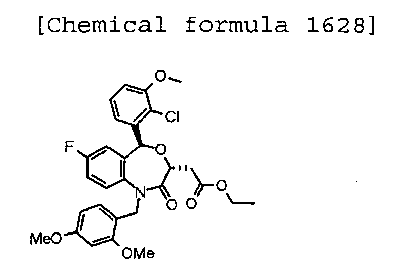

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (5.03 g) was obtained from (2-chloro-3-methoxyphenyl){2-[(2,4-dimethoxybenzyl)amino]-5-fluorophenyl}methanol (4.40 g).
MS (ESI) m/z : 558 (M+H)⁺.
¹H-NMR (CDCl₃) δ: 1.26 (3H, t, J = 7.1 Hz), 2.81 (1H, dd, J = 16.5, 5.9 Hz), 3.10 (1H, dd, J = 16.5, 7.6 Hz), 3.60 (3H, s), 3.75 (3H, s), 3.89 (3H, s), 4.10-4.20 (2H, m), 4.47 (1H, dd, J = 7.6, 5.9 Hz), 4.86 (1H, d, J = 14.5 Hz), 5.49 (1H, d, J = 14.5 Hz), 5.87 (1H, s), 6.13 (1H, dd, J = 9.2, 3.1 Hz), 6.33 (1H, d, J = 2.4 Hz), 6.39 (1H, dd, J = 8.5, 2.4 Hz), 6.94 (1H, dd, J = 8.2, 2.0 Hz), 7.01-7.08 (1H, m), 7.26-7.35 (3H, m), 7.40 (1H, dd, J = 8.8, 4.9 Hz).

### Referential Example 690

Ethyl [(trans)-5-(2-chloro-3-methoxyphenyl)-7-fluoro-2-oxo-1,2,3,5-tetrahydro-4,1-benzoxazepin-3-yl]acetate

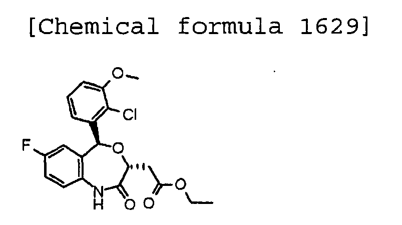

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (3.07 g) was obtained from ethyl [(trans)-5-(2-chloro-3-methoxyphenyl)-1-(2,4-dimethoxybenzyl)-7-fluoro-2-oxo-1,2,3,5-tetrahydro-4,1-benzoxazepin-3-yl]acetate (5.03 g).
MS (ESI) m/z : 408 (M+H)⁺.
¹H-NMR (CDCl₃) δ: 1.24 (3H, t, J = 7.1 Hz), 2.82 (1H, dd, J = 16.6, 6.7 Hz), 3.05 (1H, dd, J = 16.6, 6.7 Hz), 3.93 (3H, s), 4.14 (2H, q, J = 7.1 Hz), 4.62 (1H, t, J = 6.7 Hz), 6.21 (1H, s), 6.32 (1H, dd, J = 8.9, 2.3 Hz), 7.00 (1H, dd, J = 8.3, 1.5 Hz), 7.04-7.09 (2H, m), 7.24-7.27 (1H, m), 7.36 (1H, t, J = 8.1 Hz), 7.80 (1H, s).

### Referential Example 691

Ethyl [(trans)-5-(2-chloro-3-methoxyphenyl)-7-fluoro-2-thioxo-1,2,3,5-tetrahydro-4,1-benzoxazepin-3-yl]acetate

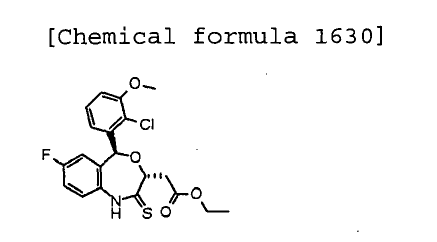

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (2.81 g) was obtained from ethyl [(trans)-5-(2-chloro-3-methoxyphenyl)-7-fluoro-2-oxo-1,2,3,5-tetrahydro-4,1-benzoxazepin-3-yl]acetate (3.07 g).
MS (ESI) m/z : 424 (M+H)⁺.
¹H-NMR (CDCl₃) δ: 1.26 (3H, t, J = 7.1 Hz), 3.00 (1H, dd, J = 16.5, 6.6 Hz), 3.27 (1H, dd, J = 1.6.5, 6.6 Hz), 3.93 (3H, s), 4.15 (2H, q, J = 7.1 Hz), 4.69 (1H, t, J = 6.6 Hz), 6.13 (1H, s), 6.31 (1H, dd, J = 8.8, 2.5 Hz), 7.00 (1H, d, J = 8.1 Hz), 7.06-7.17 (2H, m), 7.26-7.30 (1H, m), 7.37 (1H, t, J = 8.1 Hz), 9.62 (1H, s).

### Example 772

Ethyl [(trans)-6-(2-chloro-3-methoxyphenyl)-8-fluoro-1-(trifluoromethyl)-4H,6H-[1,2,4]triazolo[4,3-a][4,1]benzoxazepin-4-yl]acetate

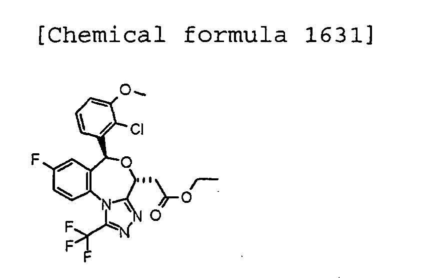

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (1.41 g) was obtained from ethyl [(trans)-5-(2-chloro-3-methoxyphenyl)-7-fluoro-2-thioxo-1,2,3,5-tetrahydro-4,1-benzoxazepin-3-yl]acetate (1.51 g).

### Example 773

Ethyl [(4R,6S)-6-(2-chloro-3-methoxyphenyl)-8-fluoro-1-(trifluoromethyl)-4H,6H-[1,2,4]triazolo[4,3-a] [4, 1] benzoxazepin-4-yl] acetate (isomer A)

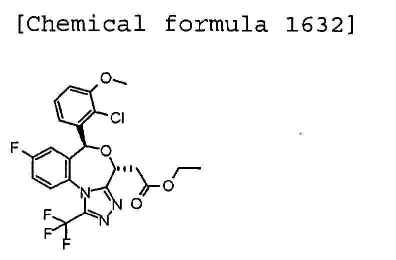

Ethyl [(4S,6R)-6-(2-chloro-3-methoxyphenyl)-8-fluoro-1-(trifluoromethyl)-4H,6H-[1,2,4]triazolo[4,3-a][4,1]benzoxazepin-4-yl]acetate(isomer B)

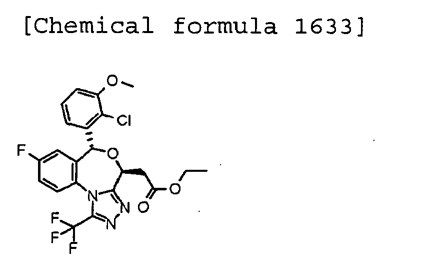

Ethyl [(trans)-6-(2-chloro-3-methoxyphenyl)-8-fluoro-1-(trifluoromethyl)-4H,6H-[1,2,4]triazolo[4,3-a][4,1]benzoxazepin-4-yl]acetate (1.23 g) was optically resolved by an optically active column (CHIRALPAK-AD; 5 cm × 50 cm) into ethyl [(4R,6S)-6-(2-chloro-3-methoxyphenyl)-8-fluoro-1-(trifluoromethyl)-4H,6H-[1,2,4]triazolo[4,3-a][4,1]benzoxazepin-4-yl]acetate (isomer A, 480 mg) and ethyl [(4S,6R)-6-(2-chloro-3-methoxyphenyl)-8-fluoro-1-(trifluoromethyl)-4H,6H-[1,2,4]triazolo[4,3-a][4,1]benzoxazepin-4-yl]acetate (isomer B, 502 mg).
Resolution conditions:
Flow rate: 50 mL/min
Developing solvent: 10% isopropanol- n-hexane
Retention time: isomer A: 58 min., isomer B: 73 min.
Ethyl [(4R,6S)-6-(2-chloro-3-methoxyphenyl)-8-fluoro-1-(trifluoromethyl)-4H,6H-[1,2,4]triazolo[4,3-a][4,1]benzoxazepin-4-yl]acetate MS (ESI) m/z : 500 (M+H)⁺.
¹H-NMR (CDCl₃) δ: 1.29 (3H, t, J = 7.1 Hz), 3.27 (1H, dd, J = 16.7, 6.8 Hz), 3.51 (1H, dd, J = 16.7, 6.8 Hz), 3.91 (3H, s), 4.16-4.24 (2H, m), 4.96 (1H, t, J = 6.8 Hz), 5.56 (1H, s), 6.48 (1H, dd, J = 8.6, 2.7 Hz), 7.01 (1H, dd, J = 8.1, 1.6 Hz), 7.23-7.30 (1H, m), 7.34 (1H, dd, J = 7.8, 1.6 Hz), 7.39 (1H, t, J = 8.0 Hz), 7.60 (1H, dd, J = 8.6, 4.4 Hz).
Ethyl [(4S,6R)-6-(2-chloro-3-methoxyphenyl)-8-fluoro-1-(trifluoromethyl)-4H,6H-[1,2,4]triazolo[4,3-a][4,1]benzoxazepin-4-yl]acetate MS (ESI) m/z : 500 (M+H)⁺.
¹H-NMR (CDCl₃) δ: 1.29 (3H, t, J = 7.1 Hz), 3.27 (1H, dd, J = 16.7, 7.1 Hz), 3.51 (1H, dd, J = 16.7, 6.4 Hz), 3.91 (3H, s), 4.15-4.26 (2H, m), 4.96 (1H, t, J = 6.7 Hz), 5.56 (1H, s), 6.48 (1H, dd, J = 8.6, 2.9 Hz), 7.01 (1H, dd, J = 8.0, 1.7 Hz), 7.24-7.31 (1H, m), 7.34 (1H, dd, J = 7.8, 1.7 Hz), 7.39 (1H, t, J = 8.0 Hz), 7.60 (1H, dd, J = 8.6, 4.7 Hz).

### Example 774

[(4R,6S)-6-(2-Chloro-3-methoxyphenyl)-8-fluoro-1-(trifluoromethyl)-4H,6H-[1,2,4]triazolo[4,3-a][4,1]benzoxazepin-4-yl]acetic acid

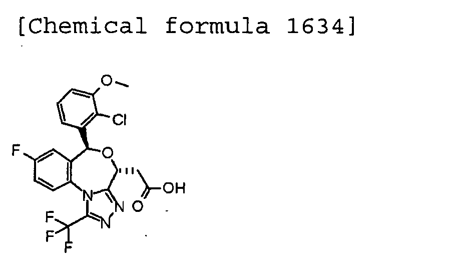

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (402 mg) was obtained from ethyl [(4R,6S)-6-(2-chloro-3-methoxyphenyl)-8-fluoro-1-(trifluoromethyl)-4H,6H-[1,2,4]triazolo[4,3-a][4,1]benzoxazepin-4-yl]acetate (477 mg).
MS (ESI) m/z : 472 (M+H)⁺.
¹H-NMR (CDCl₃) δ: 3.35 (1H, dd, J = 17.2, 6.7 Hz), 3.59 (1H, dd, J = 17.2, 6.7 Hz), 3.91 (3H, s), 4.93 (1H, t, J = 6.7 Hz), 5.56 (1H, s), 6.49 (1H, dd, J = 8.6, 2.9 Hz), 7.01 (1H, dd, J = 8.0, 1.6 Hz), 7.25-7.31 (1H, m), 7.35 (1H, dd, J = 7.8, 1.6 Hz), 7.40 (1H, t, J = 7.8 Hz), 7.61 (1H, dd, J = 8.6, 4.5 Hz).
Elemental analysis for: C₂₀H₁₄ClF₄N₃O₄·0.25H₂O
Calculated: C, 50.44; H, 3.07; N, 8.82; Cl, 7.44; F, 15.95.
Found: C, 50.50; H, 2.94; N, 8.89; Cl, 7.51; F, 15.99.

### Example 775

[(4S,6R)-6-(2-Chloro-3-methoxyphenyl)-8-fluoro-1-(trifluoromethyl)-4H,6H-[1,2,4]triazolo[4,3-a] [4,1]benzoxazepin-4-yl]acetic acid

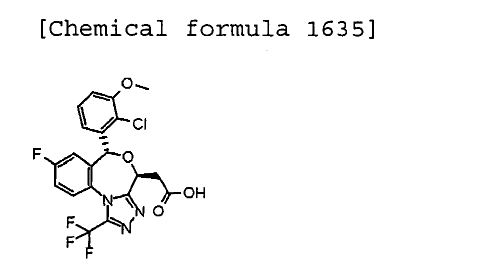

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (387 mg) was obtained from ethyl [(4S,6R)-6-(2-chloro-3-methoxyphenyl)-8-fluoro-1-(trifluoromethyl)-4H,6H-[1,2,4]triazolo[4,3-a][4,1]benzoxazepin-4-yl]acetate(500 mg) .
MS (ESI) m/z : 472 (M+H)⁺.
¹H-NMR (CDCl₃) δ: 3.34 (1H, dd, J = 17.0, 6.7 Hz), 3.59 (1H, dd, J = 17.0, 6.7 Hz), 3.91 (3H, s), 4.93 (1H, t, J = 6.7 Hz), 5.56 (1H, s), 6.49 (1H, dd, J = 8.6, 2.9 Hz), 7.01 (1H, dd, J = 7.8, 1.7 Hz), 7.24-7.31 (1H, m), 7.33-7.37 (1H, m), 7.40 (1H, t, J = 7.8 Hz), 7.61 (1H, dd, J = 8.6, 4.5 Hz).
Elemental analysis for: C₂₀H₁₄ClF₄N₃O₄
Calculated: C, 50.92; H, 2.99; N, 8.91; Cl, 7.51; F, 16.11.
Found: C, 50.66; H, 2.87; N, 8.85; Cl, 7.55; F, 16.10.

### Referential Example 692

tert-Butyl [4-fluoro-2-(3-methoxy-2-methylbenzoyl)phenyl]carbamate

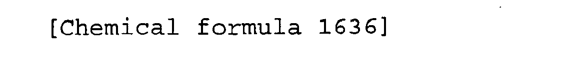

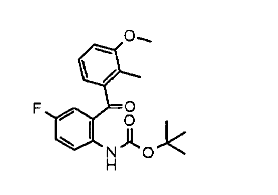

tert-Butyl (4-fluorophenyl)carbamate (3.0 g) was dissolved in tetrahydrofuran (50 mL). To the resulting solution was added dropwise sec-butyl lithium (a 1M solution, 29.8 mL) at -78°C. After stirring at the same temperature for 3 hours, a tetrahydrofuran solution (30 mL) of 4-(2-methyl-3-methoxybenzoyl)morpholine (3.70 g) was added dropwise at -78°C. The resulting mixture was stirred at the same temperatures for 10 minutes and then at room temperature for one hour. A saturated ammonium chloride solution was added to terminate the reaction. The reaction mixture was diluted with ethyl acetate and washed with water. The organic layer was washed with saturated brine and dried over anhydrous sodium sulfate. The filtrate was concentrated. The residue was purified by silica gel column (ethyl acetate:hexane=1:9) to give the title compound (2.41 g).
MS (ESI) m/z : 382 (M+Na)⁺.

### Referential Example 693

(2-Amino-5-fluorophenyl)(3-methoxy-2-methylphenyl)methanone

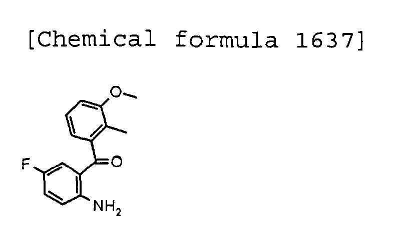

tert-Butyl [4-fluoro-2-(3-methoxy-2-methylbenzoyl)phenyl]carbamate (2.41 g) was dissolved in dioxane (20 mL). To the resulting solution was added concentrated hydrochloric acid (7 mL). The resulting mixture was stirred at room temperature for 4 hours. The reaction mixture was made alkaline with a 5N aqueous sodium hydroxide solution, followed by extraction with chloroform. The organic layer was dried over anhydrous sodium sulfate. The filtrate was concentrated to give the title compound (1.82 g).

### Referential Example 694

(2-Amino-5-fluorophenyl) (3-methoxy-2-methylphenyl) methanol

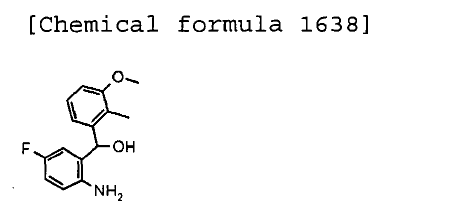

In a similar manner to that employed for the synthesis of (2-amino-5-chlorophenyl)(2-ethyl-3-methoxyphenyl)methanol, the title compound (757 mg) was obtained from (2-amino-5-fluorophenyl)(3-methoxy-2-methylphenyl)methanone (1.82 g).
MS (ESI) m/z : 244 (M-OH)⁺.
¹H-NMR (CDCl₃) δ: 2.12 (3H, s), 2.20 (1H, br s), 3.86 (3H, s), 3.88 (2H, br s), 6.03 (1H, s), 6.58 (1H, dd, J = 9.8, 2.9 Hz), 6.64 (1H, dd, J = 8.6, 4.7 Hz), 6.82 (1H, td, J = 8.3, 2.9 Hz), 6.87 (1H, d, J = 8.3 Hz), 7.04 (1H, d, J = 7.8 Hz), 7.21-7.26 (1H, m).

### Referential Example 695

{2-[(2,4-Dimethoxybenzyl)amino]-5-fluorophenyl}(3-methoxy-2-methylphenyl)methanol

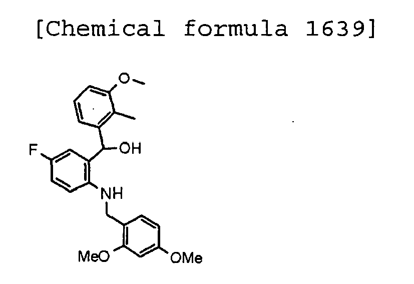

In a similar manner to that employed for the synthesis of {5-bromo-2-[(2,4-dimethoxybenzyl)amino]phenyl}(2-chloro-3-methoxyphenyl)methanol, the title compound (1.13 g) was obtained from (2-amino-5-fluorophenyl)(3-methoxy-2-methylphenyl)methanol (755 mg).
MS (ESI) m/z : 412 (M+H)⁺.
¹H-NMR (CDCl₃) δ: 2.06 (3H, s), 3.78 (3H, s), 3.80 (3H, s), 3.85 (3H, s), 4.24 (2H, s), 4.72 (1H, s), 6.02 (1H, s), 6.41 (1H, dd, J = 8.3, 2.5 Hz), 6.46 (1H, d, J = 2.5 Hz), 6.55 (1H, dd, J = 9.8, 3.2 Hz), 6.66 (1H, dd, J = 8.8, 4.9 Hz), 6.82-6.89 (2H, m), 7.05 (1H, d, J = 7.8 Hz), 7.10 (1H, d, J = 8.1 Hz), 7.23 (1H, t, J = 8.3 Hz).

### Referential Example 696

Ethyl [(trans)-1-(2,4-dimethoxybenzyl)-7-fluoro-5-(3-methoxy-2-methylphenyl)-2-oxo-1,2,3,5-tetrahydro-4,1-benzoxazepin-3-yl]acetate

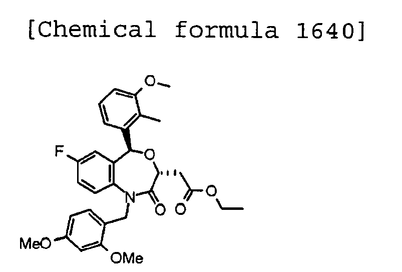

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (1.35 g) was obtained from {2-[(2,4-dimethoxybenzyl)amino]-5-fluorophenyl}(3-methoxy-2-methylphenyl)methanol (1.13 g).
MS (ESI) m/z : 538 (M+H)⁺.
¹H-NMR (CDCl₃) δ: 1.26 (3H, t, J = 7.1 Hz), 1.35 (3H, s), 2.81 (1H, dd, J = 16.4, 5.6 Hz), 3.10 (1H, dd, J = 16.4, 7.8 Hz), 3.56 (3H, s), 3.76 (3H, s), 3.80 (3H, s), 4.09-4.19 (2H, m), 4.45 (1H, dd, J = 7.8, 5.6 Hz), 4.86 (1H, d, J = 14.7 Hz), 5.48 (1H, d, J = 14.7 Hz), 5.62 (1H, s), 6.19 (1H, dd, J = 8.9, 2.8 Hz), 6.31 (1H, d, J = 2.5 Hz), 6.41 (1H, dd, J = 8.1, 2.5 Hz), 6.84 (1H, d, J = 7.8 Hz), 7.02-7.09 (1H, m), 7.16 (1H, d, J = 8.1 Hz), 7.22 (1H, d, J = 7.8 Hz), 7.39 (1H, d, J = 8.3 Hz), 7.43 (1H, dd, J = 8.8, 4.9 Hz).

### Referential Example 697

Ethyl [(trans)-7-fluoro-5-(3-methoxy-2-methylphenyl)-2-oxo-1,2,3,5-tetrahydro-4,1-benzoxazepin-3-yl]acetate

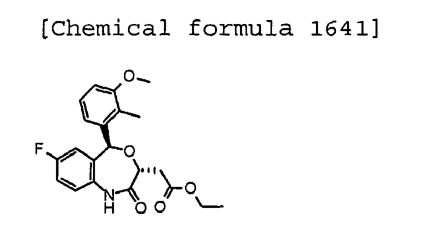

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (791 mg) was obtained from ethyl [(trans)-1-(2,4-dimethoxybenzyl)-7-fluoro-5-(3-methoxy-2-methylphenyl)-2-oxo-1,2,3,5-tetrahydro-4,1-benzoxazepin-3-yl]acetate (1.34 g).
MS (ESI) m/z : 388 (M+H)⁺.
¹H-NMR (CDCl₃) δ: 1.23 (3H, t, J = 7.1 Hz), 1.93(3H,s), 2.80 (1H, dd, J = 16.4, 6.4 Hz), 3.02 (1H, dd, J = 16.4, 6.6 Hz), 3.85 (3H, s), 4.08-4.17 (2H, m), 4.58 (1H, t, J = 6.5 Hz), 6.05 (1H, s), 6.42 (1H, d, J = 8.8 Hz), 6.89 (1H, d, J = 8.1 Hz), 7.03 (1H, d, J = 1.5 Hz), 7.04 (1H, d, J = 1.5 Hz), 7.06 (1H, d, J = 7.8 Hz), 7.22-7.27 (1H, m), 7.67 (1H, s).

### Referential Example 698

Ethyl [(trans)-7-fluoro-5-(3-methoxy-2-methylphenyl)-2-thioxo-1,2,3,5-tetrahydro-4,1-benzoxazepin-3-yl]acetate

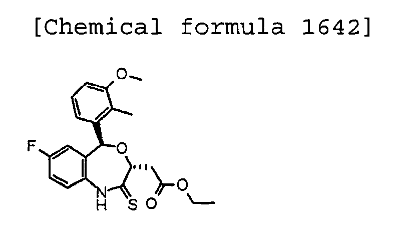

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (734 mg) was obtained from ethyl [(trans)-7-fluoro-5-(3-methoxy-2-methylphenyl)-2-oxo-1,2,3,5-tetrahydro-4,1-benzoxazepin-3-yl] acetate (789 mg).
MS (ESI) m/z : 404 (M+H)⁺.
¹H-NMR (CDCl₃) δ: 1.25 (3H, t, J = 7.1 Hz), 1.88 (3H, s), 2.98 (1H, dd, J = 16.4, 6.6 Hz), 3.26 (1H, dd, J = 16.4, 6.6 Hz), 3.85 (3H, s), 4.14 (2H, q, J = 7.1 Hz), 4.69 (1H, t, J = 6.6 Hz), 5.96 (1H, s), 6.39 (1H, dd, J = 8.9, 2.6 Hz), 6.89 (1H, d, J = 8.3 Hz), 7.05-7.15 (3H, m), 7.26 (1H, t, J = 8.0 Hz), 9.57 (1H, s).

### Example 776

Ethyl [(trans)-8-fluoro-6-(3-methoxy-2-methylphenyl)-1-(trifluoromethyl)-4H,6H-[1,2,4]triazolo[4,3-a][4,1]benzoxazepin-4-yl]acetate

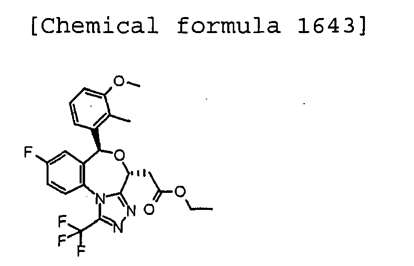

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (756 mg) was obtained from ethyl [(trans)-7-fluoro-5-(3-methoxy-2-methylphenyl)-2-thioxo-1,2,3,5-tetrahydro-4,1-benzoxazepin-3-yl]acetate (700 mg).

### Example 777

Ethyl [(4R,6R)-8-fluoro-6-(3-methoxy-2-methylphenyl)-1-(trifluoromethyl)-4H,6H-[1,2,4]triazolo[4,3-a][4,1] benzoxazepin-4-yl] acetate (isomer A)

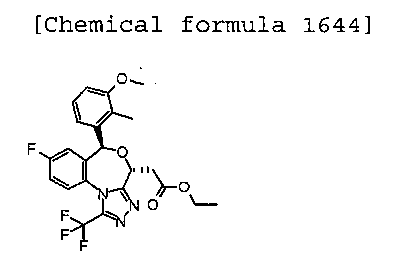

Ethyl [(4S,6S)-8-fluoro-6-(3-methoxy-2-methylphenyl)-1-(trifluoromethyl)-4H,6H-[1,2,4]triazolo[4,3-a][4,1]benzoxazepin-4-yl]acetate (isomer B)

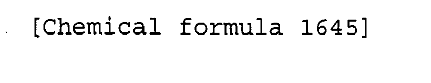

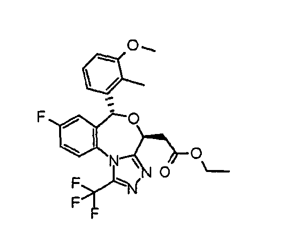

Ethyl [(trans)-8-fluoro-6-(3-methoxy-2-methylphenyl)-1-(trifluoromethyl)-4H,6H-[1,2,4]triazolo[4,3-a][4,1]benzoxazepin-4-yl]acetate (756 mg) was optically resolved by an optically active column (CHIRALPAK-AD; 5 cm × 50 cm) into ethyl [(4R,6R)-8-fluoro-6-(3-methoxy-2-methylphenyl)-1-(trifluoromethyl)-4H,6H-[1,2,4]triazolo[4,3-a][4,1]benzoxazepin-4-yl]acetate (isomer A, 368 mg) and ethyl [(4S,6S)-8-fluoro-6-(3-methoxy-2-methylphenyl)-1-(trifluoromethyl)-4H,6H-[1,2,4]triazolo[4,3-a][4,1]benzoxazepin-4-yl]acetate (isomer B, 366 mg).
Resolution conditions:
Flow rate: 50 mL/min
Developing solvent: 9% isopropanol- n-hexane
Retention time: isomer A: 42 min., isomer B: 46 min.
Ethyl [(4R,6R)-8-fluoro-6-(3-methoxy-2-methylphenyl)-1-(trifluoromethyl)-4H,6H-[1,2,4]triazolo[4,3-a][4,1]benzoxazepin-4-yl]acetate (isomer A) MS (ESI) m/z : 480 (M+H)⁺.
¹H-NMR (CDCl₃) δ: 1.29 (3H, t, J = 7.1 Hz), 1.66 (3H, s), 3.26 (1H, dd, J = 16.7, 7.1 Hz), 3.50 (1H, dd, J = 16.7, 6.6 Hz), 3.83 (3H, s), 4.15-4.24 (2H, m), 4.96 (1H, t, J = 6.9 Hz), 5.39 (1H, s), 6.54 (1H, dd, J = 8.8, 2.9 Hz), 6.91 (1H, d, J = 8.1 Hz), 7.23-7.33 (3H, m), 7.60 (1H, dd, J = 8.8, 4.5 Hz).
Ethyl [(4S,6S)-8-fluoro-6-(3-methoxy-2-methylphenyl)-1-(trifluoromethyl)-4H,6H-[1,2,4]triazolo[4,3-a] [4,1]benzoxazepin-4-yl]acetate (isomer B) MS (ESI) m/z : 480 (M+H)⁺.
¹H-NMR (CDCl₃) δ: 1.29 (3H, t, J = 7.1 Hz), 1.66 (3H, s), 3.26 (1H, dd, J = 16.7, 7.1 Hz), 3.50 (1H, dd, J = 16.7, 6.6 Hz), 3.83 (3H, s), 4.15-4.24 (2H, m), 4.96 (1H, t, J = 6.9 Hz), 5.39 (1H, s), 6.54 (1H, dd, J = 8.8, 2.9 Hz), 6.91 (1H, d, J = 7.8 Hz), 7.23-7.33 (3H, m), 7.60 (1H, dd, J = 8.8, 4.7 Hz).

### Example 778

[(4R,6R)-8-Fluoro-6-(3-methoxy-2-methylphenyl)-1-(trifluoromethyl)-4H,6H-[1,2,4]triazolo[4,3-a] [4,1]benzoxazepin-4-yl]acetic acid

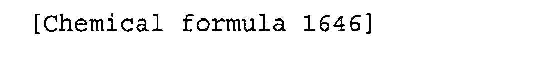

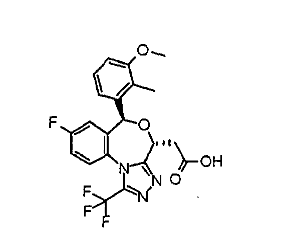

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (309 mg) was obtained from ethyl [(4R,6R)-8-fluoro-6-(3-methoxy-2-methylphenyl)-1-(trifluoromethyl)-4H,6H-[1,2,4]triazolo[4,3-a][4,1]benzoxazepin-4-yl]acetate (isomer A, 365 mg).
MS (ESI) m/z : 452 (M+H)⁺.
¹H-NMR (CDCl₃) δ: 1.66 (3H, s), 3.33 (1H, dd, J = 17.1, 6.7 Hz), 3.58 (1H, dd, J = 17.1, 6.7 Hz), 3.83 (3H, s), 4.93 (1H, t, J = 6.7 Hz), 5.39 (1H, s), 6.55 (1H, dd, J = 8.8, 2.9 Hz), 6.91 (1H, d, J = 7.8 Hz), 7.24-7.33 (3H, m), 7.60 (1H, dd, J = 8.8, 4.7 Hz).
Elemental analysis for: C₂₁H₁₇F₄N₃O₄·0.5H₂O·0.1 hexane
Calculated: C, 55.32; H, 4.17; N, 8.96; F, 16.19.
Calculated: C, 55.51; H, 3.85; N, 9.06; F, 16.07.

### Example 779

[(4S,6S)-8-Fluoro-6-(3-methoxy-2-methylphenyl)-1-(trifluoromethyl)-4H,6H-[1,2,4]triazolo[4,3-a][4,1]benzoxazepin-4-yl]acetic acid

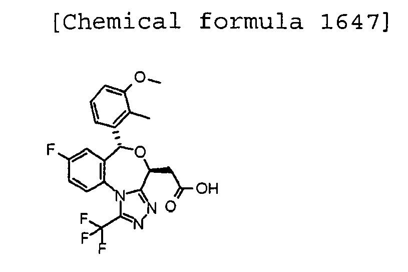

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (302 mg) was obtained from ethyl [(4S,6S)-8-fluoro-6-(3-methoxy-2-methylphenyl)-1-(trifluoromethyl)-4H,6H-[1,2,4]triazolo[4,3-a][4,1]benzoxazepin-4-yl]acetate (isomer B, 363 mg).
MS (ESI) m/z : 452 (M+H)⁺.
¹H-NMR (CDCl₃) δ: 1.66 (3H, s), 3.33 (1H, dd, J = 17.2, 6.7 Hz), 3.59 (1H, dd, J = 17.2, 6.7 Hz), 3.83 (3H, s), 4.93 (1H, t, J = 6.7 Hz), 5.39 (1H, s), 6.55 (1H, dd, J = 8.9, 2.9 Hz), 6.91 (1H, dd, J = 7.8, 1.2 Hz), 7.24-7.34 (3H, m), 7.60 (1H, dd, J = 8.8, 4.7 Hz).
Elemental analysis for: C₂₁H₁₇F₄N₃O₄·0.5H₂O·0.1hexane
Calculated: C, 55.32; H, 4.17; N, 8.96; F, 16.19.
Calculated: C, 55.69; H, 3.94; N, 8.93; F, 16.01.

### Referential Example 699

4-(2-Isopropyl-3-methoxybenzoyl)morpholine

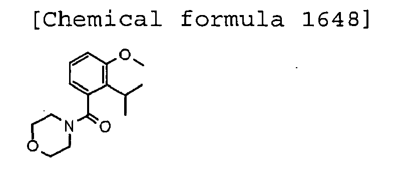

In a similar manner to that employed for the synthesis of 4-(2-ethyl-3-methoxybenzoyl)morpholine, the title compound (2.16 g) was obtained from 2-isopropyl-3-methoxybenzoic acid (1.78 g).
MS (ESI) m/z : 264 (M+H)⁺.
¹H-NMR (CDCl₃) δ: 1.32 (3H, d, J = 2.6 Hz), 1.34 (3H, d, J = 2.6 Hz), 2.90-3.02 (1H, m), 3.18-3.30 (2H, m), 3.52-3.61 (2H, m), 3.74-3.78 (2H, m), 3.79-3.84 (5H, m), 6.69 (1H, d, J = 7.6 Hz), 6.87 (1H, d, J = 8.3 Hz), 7.18 (1H, t, J = 7.8 Hz).

### Referential Example 700

N-[4-Chloro-2-(2-isopropyl-3-methoxybenzoyl)phenyl]-2,2-dimethylpropanamide

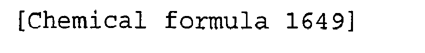

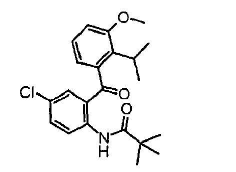

In a similar manner to that employed for the synthesis of N-[4-chloro-2-(2-ethyl-3-methoxybenzoyl)phenyl]-2,2-dimethylpropanamide, the title compound (1.30 g) was obtained from 4-(2-isopropyl-3-methoxybenzoyl)morpholine (2.16 g) and N-(4-chlorophenyl)-2,2-dimethylpropanamide (1.74 g).
MS (ESI) m/z : 388 (M+H) ⁺.
¹H-NMR (CDCl₃) δ: 1.25 (6H, d, J = 7.1 Hz), 1.39 (9H, s), 2.77-2.86 (1H, m), 3.89 (3H, s), 6.72 (1H, dd, J = 7.6, 1.2 Hz), 7.00 (1H, d, J = 8.3 Hz), 7.23-7.30 (1H, m), 7.36 (1H, d, J = 2.5 Hz), 7.50 (1H, dd, J = 9.1, 2.5 Hz), 8.83 (1H, d, J = 9.1 Hz), 11.88 (1H, s).

### Referential Example 701

(2-Amino-5-chlorophenyl)(2-isopropyl-3-methoxyphenyl)methanone

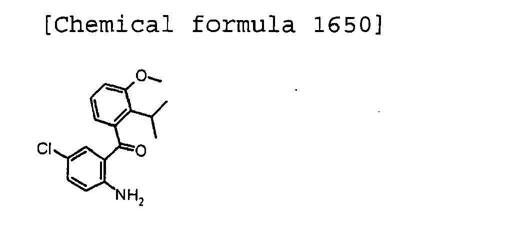

In a similar manner to that employed for the synthesis of (2-amino-5-chlorophenyl)(2-ethyl-3-methoxyphenyl)methanone, the title compound (1.02 g) was obtained from N-[4-chloro-2-(2-isopropyl-3-methoxybenzoyl)phenyl]-2,2-dimethylpropanamide (1.30 g).
MS (ESI) m/z : 304 (M+H)⁺.
¹H-NMR (CDCl₃) δ: 1.25 (6H, d, J = 6.9 Hz), 2.81-2.89 (1H, m), 3.88 (3H, s), 6.43 (2H, s), 6.66 (1H, d, J = 8.6 Hz), 6.72 (1H, dd, J = 7.6, 1.1 Hz), 6.95 (1H, d, J = 7.6 Hz), 7.17 (1H, d, J = 2.5 Hz), 7.19-7.25 (2H, m).

### Referential Example 702

(2-Amino-5-chlorophenyl)(2-isopropyl-3-methoxyphenyl)methanol

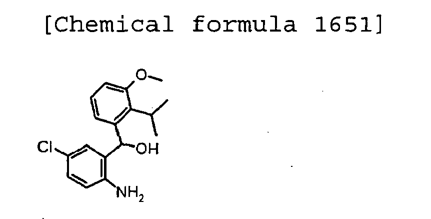

In a similar manner to that employed for the synthesis of (2-amino-5-chlorophenyl)(2-ethyl-3-methoxyphenyl)methanol, the title compound (1.06 g) was obtained from (2-amino-5-chlorophenyl)(2-isopropyl-3-methoxyphenyl)methanone (1.02 g).
MS (ESI) m/z : 288 (M-OH)⁺.
¹H-NMR (CDCl₃) δ: 1.16 (3H, d, J = 7.1 Hz), 1.30 (3H, d, J = 7.1 Hz), 1.54 (2H, br s), 2.98-3.13 (1H, m), 3.85 (3H, s), 4.00-4.20 (1H, br m), 6.07 (1H, s), 6.64 (1H, d, J = 8.6 Hz), 6.79 (1H, d, J = 2.5 Hz), 6.91 (1H, d, J = 8.1 Hz), 7.03 (1H, dd, J = 7.8, 1.0 Hz), 7.06 (1H, dd, J = 8.6, 2.5 Hz), 7.22 (1H, t, J = 8.1 Hz).

### Referential Example 703

{5-Chloro-2-[(2,4-dimethoxybenzyl)amino]phenyl}(2-isopropyl-3-methoxyphenyl)methanol

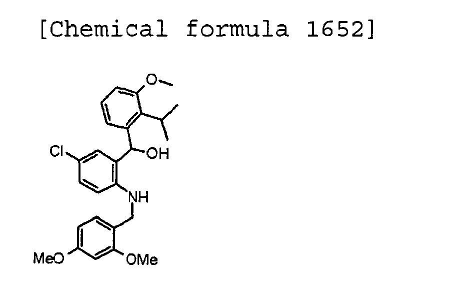

In a similar manner to that employed for the synthesis of {5-bromo-2-[(2,4-dimethoxybenzyl)amino]phenyl}(2-chloro-3-methoxyphenyl)methanol, the title compound (1.43 g) was obtained from 2-amino-5-chlorophenyl)(2-isopropyl-3-methoxyphenyl)methanol (1.06 g).
MS (ESI) m/z : 456 (M+H)⁺.
¹H-NMR (CDCl₃) δ: 1.10 (3H, d, J = 7.1 Hz), 1.29 (3H, d, J = 7.1 Hz), 2.07 (1H, br s), 2.96-3.10 (1H, m), 3.76 (3H, s), 3.79 (3H, s), 3.84 (3H, s), 4.25 (2H, s), 4.92 (1H, br s), 6.04 (1H, s), 6.40 (1H, dd, J = 8.2, 2.3 Hz), 6.45 (1H, d, J = 2.2 Hz), 6.65 (1H, d, J = 8.8 Hz), 6.80 (1H, d, J = 2.5 Hz), 6.89 (1H, d, J = 8.1 Hz), 7.01 (1H, d, J = 7.8 Hz), 7.07 (1H, d, J = 8.1 Hz), 7.10 (1H, dd, J = 8.8, 2.5 Hz), 7.20 (1H, t, J = 7.8 Hz).

### Referential Example 704

Ethyl [(trans)-7-chloro-1-(2,4-dimethoxybenzyl)-5-(2-isopropyl-3-methoxyphenyl)-2-oxo-1,2,3,5-tetrahydro-4,1-benzoxazepin-3-yl]acetate

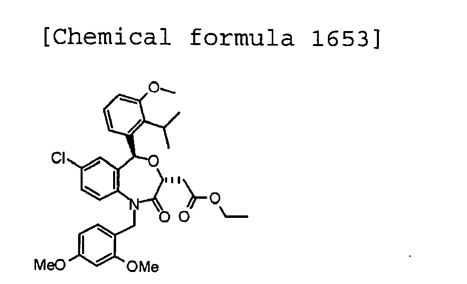

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (1.41 g) was obtained from {5-chloro-2-[(2,4-dimethoxybenzyl)amino]phenyl}(2-isopropyl-3-methoxyphenyl)methanol (1.43 g).
¹H-NMR (CDCl₃) δ: 0.73 (3H, d, J = 6.9 Hz), 1.01 (3H, d, J = 6.9 Hz), 1.26 (3H, t, J = 7.1 Hz), 2.66-2.92 (2H, m), 3.10 (1H, dd, J = 16.4, 7.6 Hz), 3.62 (3H, s), 3.76 (3H, s), 3.79 (3H, s), 4.10-4.19 (2H, m), 4.46 (1H, dd, J = 7.6, 5.9 Hz), 4.85 (1H, d, J = 14.7 Hz), 5.52 (1H, d, J = 14.7 Hz), 5.72 (1H, s), 6.36 (1H, d, J = 2.2 Hz), 6.41 (1H, dd, J = 8.3, 2.5 Hz), 6.46 (1H, d, J = 2.5 Hz), 6.87 (1H, d, J = 8.1 Hz), 7.13-7.22 (2H, m), 7.30-7.37 (2H, m), 7.41 (1H, d, J = 8.6 Hz).

### Referential Example 705

Ethyl [(trans)-7-chloro-5-(2-isopropyl-3-methoxyphenyl)-2-oxo-1,2,3,5-tetrahydro-4,1-benzoxazepin-3-yl]acetate

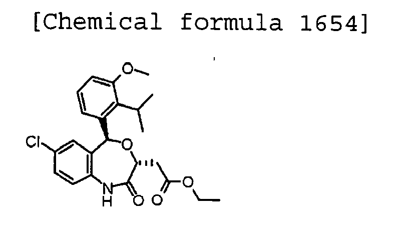

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (946 mg) was obtained from ethyl [(trans)-7-chloro-1-(2,4-dimethoxybenzyl)-5-(2-isopropyl-3-methoxyphenyl)-2-oxo-1,2,3,5-tetrahydro-4,1-benzoxazepin-3-yl]acetate (1.41 g).
¹H-NMR (CDCl₃) δ: 1.06 (3H, d, J = 6.9 Hz), 1.20-1.28 (6H, m), 2.65-2.77 (1H, m), 2.83 (1H, dd, J = 16.7, 5.9 Hz), 3.01 (1H, dd, J = 16.7, 6.9 Hz), 3.84 (3H, s), 4.09-4.16 (2H, m), 4.60 (1H, dd, J = 6.9, 5.9 Hz), 6.08 (1H, s), 6.73 (1H, d, J = 2.5 Hz), 6.92 (1H, d, J = 8.3 Hz), 6.98 (1H, d, J = 8.6 Hz), 7.04 (1H, d, J = 8.1 Hz), 7.22 (1H, t, J = 8.1 Hz), 7.30 (1H, dd, J = 8.3, 2.5 Hz), 7.62 (1H, s).

### Referential Example 706

Ethyl [(trans)-7-chloro-5-(2-isopropyl-3-methoxyphenyl)-2-thioxo-1,2,3,5-tetrahydro-4,1-benzoxazepin-3-yl]acetate

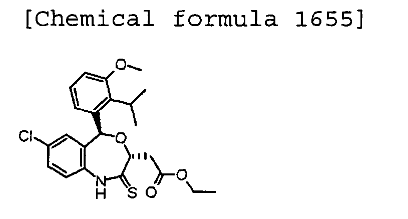

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (835 mg) was obtained from ethyl [(trans)-7-chloro-5-(2-isopropyl-3-methoxyphenyl)-2-oxo-1,2,3,5-tetrahydro-4,1-benzoxazepin-3-yl] acetate (944 mg) .
MS (ESI) m/z : 448 (M+H)⁺.
¹H-NMR (CDCl₃) δ: 1.02 (3H, d, J = 6.9 Hz), 1.21-1.27 (6H, m), 2.60 (1H, br s), 2.98 (1H, dd, J = 16.4, 6.1 Hz), 3.26 (1H, dd, J = 16.4, 6.9 Hz), 3.84 (3H, s), 4.14 (2H, q, J = 7.1 Hz), 4.70 (1H, t, J = 6.5 Hz), 6.00 (1H, s), 6.71 (1H, d, J = 2.2 Hz), 6.92 (1H, d, J = 8.1 Hz), 7.07 (1H, d, J = 8.6 Hz), 7.12 (1H, d, J = 7.6 Hz), 7.22-7.26 (1H, m), 7.35 (1H, dd, J = 8.6, 2.2 Hz), 9.56 (1H, s).

### Example 780

Ethyl [(trans)-8-chloro-6-(2-isopropyl-3-methoxyphenyl)-1-(trifluoromethyl)-4H,6H-[1,2,4]triazolo[4,3-a][4,1]benzoxazepin-4-yl]acetate

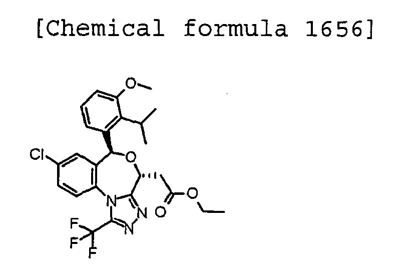

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a] [4,1]benzoxazepin-4-yl]acetate, the title compound (805 mg) was obtained from ethyl [(trans)-7-chloro-5-(2-isopropyl-3-methoxyphenyl)-2-thioxo-1,2,3,5-tetrahydro-4,1-benzoxazepin-3-yl]acetate (832 mg).
MS (ESI) m/z : 524 (M+H)⁺.
¹H-NMR (CDCl₃) δ: 0.88 (3H, d, J = 6.9 Hz), 1.10 (3H, d, J = 6.9 Hz), 1.29 (3H, t, J = 7.1 Hz), 2.31 (1H, br s), 3.26 (1H, dd, J = 16.8, 7.1 Hz), 3.50 (1H, dd, J = 16.8, 6.9 Hz), 3.82 (3H, s), 4.16-4.24 (2H, m), 4.95 (1H, t, J = 6.9 Hz), 5.45 (1H, s), 6.86 (1H, s), 6.94 (1H, d, J = 8.1 Hz), 7.21-7.31 (2H, m), 7.51-7.58 (2H, m).

### Example 781

[(trans)-8-Chloro-6-(2-isopropyl-3-methoxyphenyl)-1-(trifluoromethyl)-4H,6H-[1,2,4]triazolo[4,3-a][4,1]benzoxazepin-4-yl]acetic acid

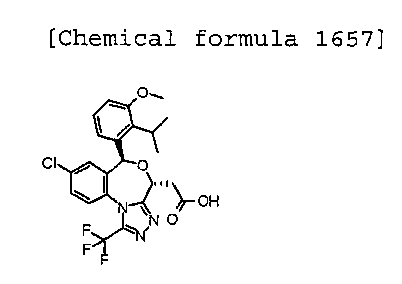

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (38 mg) was obtained from ethyl [(trans)-8-chloro-6-(2-isopropyl-3-methoxyphenyl)-1-(trifluoromethyl)-4H,6H-[1,2,4]triazolo[4,3-a] [4,1]benzoxazepin-4-yl]acetate (80 mg).
MS (ESI) m/z : 496 (M+H)⁺.
¹H-NMR (CDCl₃) δ: 0.88 (3H, d, J = 6.9 Hz), 1.10 (3H, d, J = 6.9 Hz), 2.29 (1H, br s), 3.32 (1H, dd, J = 17.0, 6.7 Hz), 3.58 (1H, dd, J = 17.0, 6.7 Hz), 3.82 (3H, s), 4.93 (1H, t, J = 6.7 Hz), 5.46 (1H, s), 6.87 (1H, s), 6.94 (1H, dd, J = 8.1, 1.2 Hz), 7.21-7.33 (2H, m), 7.51-7.58 (2H, m).
Elemental analysis for: C₂₃H₂₁ClF₃N₃O₄
Calculated: C, 55.71; H, 4.27; N, 8.47.
Found: C, 55.40; H, 4.26; N, 8.21.

### Referential Example 707

tert-Butyl {4-chloro-2-[(5-chloro-2,3-dimethoxyphenyl)(hydroxy)methyl]phenyl}carbamate

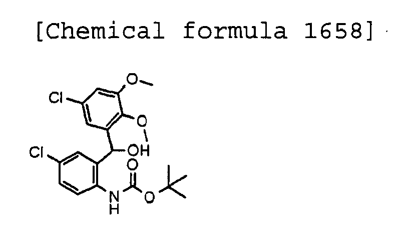

tert-Butyl (4-chlorophenyl)carbamate (1.14 g) was dissolved in tetrahydrofuran (15 mL). To the resulting solution was added dropwise sec-butyl lithium (a 1M solution, 10.9 mL) at from -30 to -40°C. After stirring at the same temperature for one hour, a tetrahydrofuran solution of 5-chloro-2,3-dimethoxybenzaldehyde (1.0 g) was added dropwise at -30°C or less. The resulting mixture was warmed gradually to room temperature and stirred for one hour. Saturated ammonium chloride was added to terminate the reaction and the reaction mixture was diluted with ethyl acetate and washed with water. The organic layer was washed with saturated brine and dried over anhydrous sodium sulfate. The filtrate was concentrated and the residue was purified by silica gel column (ethyl acetate:hexane=1:2) to give the title compound (890 mg).
MS (ESI) m/z : 450 (M+Na)⁺.
¹H-NMR (CDCl₃) δ: 1.53 (9H, s), 3.14 (1H, d, J = 4.7 Hz), 3.72 (3H, s), 3.86 (3H, s), 6.02 (1H, d, J = 4.7 Hz), 6.88 (1H, d, J = 2.5 Hz), 7.02 (1H, d, J = 2.5 Hz), 7.13 (1H, d, J = 2.5 Hz), 7.23 (1H, dd, J = 8.6, 2.5 Hz), 7.80 (1H, d, J = 8.6 Hz); 7.86 (1H, s).

### Referential Example 708

(2-Amino-5-chlorophenyl)(5-chloro-2,3-dimethoxyphenyl)methanol

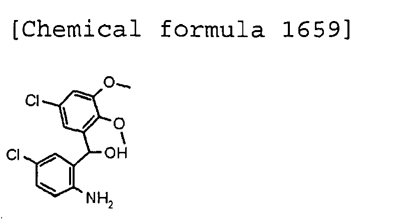

tert-Butyl {4-chloro-2-[(5-chloro-2,3-dimethoxyphenyl)(hydroxy)methyl]phenyl}carbamate (4.48 g) was dissolved in methanol (50 mL). To the resulting solution was added a 1N aqueous sodium hydroxide solution (30 mL). The resulting mixture was stirred at 50°C for 2 hours. After the reaction mixture was concentrated, the residue was diluted with methylene chloride and washed with water. The organic layer was dried over anhydrous sodium sulfate. The filtrate was concentrated to give the title compound (3.48 g).

### Referential Example 709

{5-Chloro-2-[(2,4-dimethoxybenzyl)amino]phenyl}(5-chloro-2,3-dimethoxyphenyl)methanol

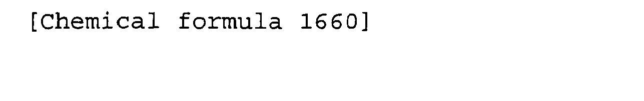

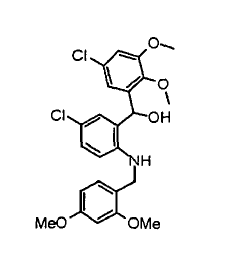

In a similar manner to that employed for the synthesis of {5-bromo-2-[(2,4-dimethoxybenzyl)amino]phenyl}(2-chloro-3-methoxyphenyl)methanol, the title compound (2.96 g) was obtained from (2-amino-5-chlorophenyl)(5-chloro-2,3-dimethoxyphenyl)methanol (3.48 g).
MS (ESI) m/z : 478 (M+H)⁺.
¹H-NMR (CDCl₃) δ: 2.95(1H, brs), 3.71 (3H, s), 3.79 (3H, s), 3.80 (3H, s), 3.87 (3H, s), 4.24 (2H, s), 5.17(1H, brs), 5.98 (1H, s), 6.41 (1H, dd, J = 8.3, 2.5 Hz), 6.45 (1H, d, J = 2.5 Hz), 6.60 (1H, d, J = 8.8 Hz), 6.87-6.89 (2H, m), 6.99 (1H, d, J = 2.5 Hz), 7.07 (1H, d, J = 8.3 Hz), 7.09 (1H, dd, J = 8.8, 2.5 Hz).

### Referential Example 710

Ethyl [(trans)-7-chloro-5-(5-chloro-2,3-dimethoxyphenyl)-1-(2,4-dimethoxybenzyl)-2-oxo-1,2,3,5-tetrahydro-4,1-benzoxazepin-3-yl]acetate

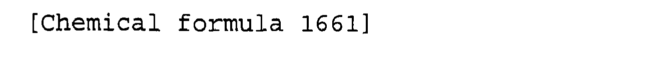

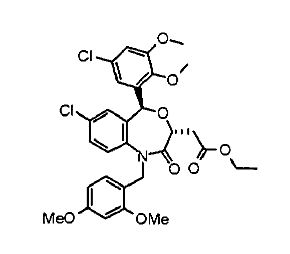

In a similar manner to that employed for the synthesis of ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate, the title compound (2.98 g) was obtained from {5-chloro-2-[(2,4-dimethoxybenzyl)amino]phenyl}(5-chloro-2,3-dimethoxyphenyl)methanol (2.66 g).
MS (ESI) m/z : 604 (M+H)⁺.
¹H-NMR (CDCl₃) δ: 1.27 (3H, t, J = 7.1 Hz), 2.81 (1H, dd, J = 16.4, 6.1 Hz), 3.07 (1H, dd, J = 16.4, 7.6 Hz), 3.15 (3H, s), 3.66 (3H, s), 3.75 (3H, s), 3.85 (3H, s), 4.09-4.21 (2H, m), 4.45 (1H, dd, J = 7.6, 6.1 Hz), 4.83 (1H, d, J = 15.0 Hz), 5.50 (1H, d, J = 15.0 Hz), 5.90 (1H, s), 6.38-6.42 (2H, m), 6.53 (1H, d, J = 2.0 Hz), 6.91 (1H, d, J = 2.5 Hz), 7.13 (1H, d, J = 2.5 Hz), 7.23 (1H, d, J = 9.1 Hz), 7.29-7.35 (2H, m).

### Referential Example 711

Ethyl [(trans)-7-chloro-5-(5-chloro-2,3-dimethoxyphenyl)-2-oxo-1,2,3,5-tetrahydro-4,1-benzoxazepin-3-yl]acetate

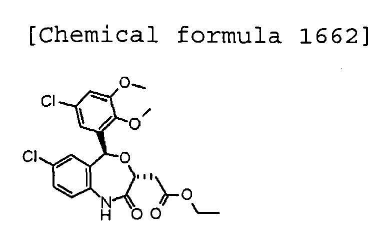

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (1.61 g) was obtained from ethyl [(trans)-7-chloro-5-(5-chloro-2,3-dimethoxyphenyl)-1-(2,4-dimethoxybenzyl)-2-oxo-1,2,3,5-tetrahydro-4,1-benzoxazepin-3-yl]acetate (2.97 g).

### Referential Example 712

Ethyl [(trans)-7-chloro-5-(5-chloro-2,3-dimethoxyphenyl)-2-thioxo-1,2,3,5-tetrahydro-4,1-benzoxazepin-3-yl]acetate

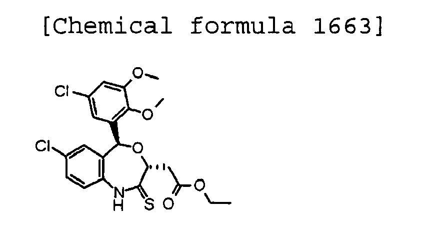

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (1.37 g) was obtained from ethyl [(trans)-7-chloro-5-(5-chloro-2,3-dimethoxyphenyl)-2-oxo-1,2,3,5-tetrahydro-4,1-benzoxazepin-3-yl] acetate (1.61 g).
MS (ESI) m/z : 470 (M+H)⁺.
¹H-NMR (CDCl₃) δ: 1.27 (3H, t, J = 7.1 Hz), 2.99 (1H, dd, J = 16.4, 6.6 Hz), 3.22 (1H, dd, J = 16.4, 6.6 Hz), 3.59 (3H, s), 3.88 (3H, s), 4.10-4.21 (2H, m), 4.67 (1H, t, J = 6.6 Hz), 6.04 (1H, s), 6.65 (1H, d, J = 2.2 Hz), 6.95 (1H, d, J = 2.2 Hz), 7.07 (1H, d, J = 8.5 Hz), 7.12 (1H, d, J = 2.3 Hz), 7.36 (1H, dd, J = 8.5, 2.3 Hz), 9.61 (1H, s).

### Example 782

Ethyl [(trans)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate

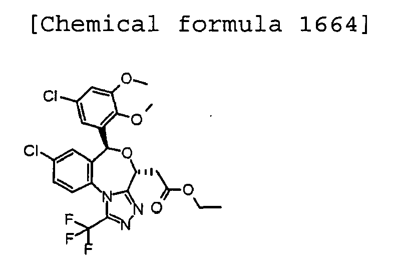

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a] [4,1]benzoxazepin-4-yl]acetate, the title compound (800 mg) was obtained from ethyl [(trans)-7-chloro-5-(5-chloro-2,3-dimethoxyphenyl)-2-thioxo-1,2,3,5-tetrahydro-4,1-benzoxazepin-3-yl]acetate (680 mg).

### Example 783

Ethyl [(4S,6R)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate (isomer A)

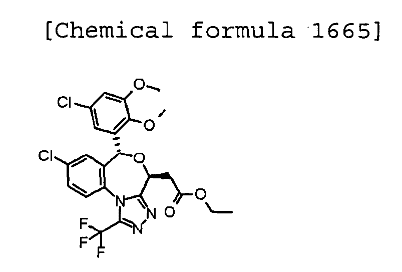

Ethyl [(4R,6S)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate(isomer B)

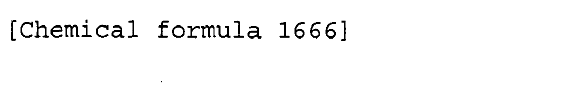

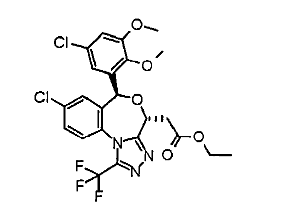

Ethyl [(trans)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate (800 mg) was optically resolved by an optically active column (CHIRALPAK-AD; 5 cm × 50 cm) into ethyl [(4S,6R)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate (isomer A, 405 mg) and ethyl [(4R,6S)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate (isomer B, 423 mg).
Resolution conditions:
Flow rate: 50 mL/min
Developing solvent: from 20% isopropanol- n-hexane to 50% isopropanol- n-hexane (on and after 40 min.)
Retention time: isomer A: 27 min., isomer B: 67 min.
Ethyl [(4S,6R)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate
MS (ESI) m/z : 546 (M+H)⁺.
¹H-NMR (CDCl₃) δ: 1.30 (3H, t, J = 7.1 Hz), 3.25 (1H, dd, J = 16.7, 6.7 Hz), 3.41 (3H, s), 3.49 (1H, dd, J = 16.7, 6.7 Hz), 3.86 (3H, s), 4.13-4.28 (2H, m), 4.92 (1H, t, J = 6.7 Hz), 5.47 (1H, s), 6.86 (1H, d, J = 1.7 Hz), 6.96 (1H, d, J = 2.5 Hz), 7.19 (1H, d, J = 2.5 Hz), 7.51-7.57 (2H, m).
Ethyl [(4R,6S)-8-chloro-6-(5-chloro-2,3-dimethoxyphenyl)-1-(trifluoromethyl)-4H,6H-[1,2,4]triazolo[4,3-a] [4,1]benzoxazepin-4-yl]acetate MS (ESI) m/z : 546 (M+H)⁺.
¹H-NMR (CDCl₃) δ: 1.30 (3H, t, J = 7.1 Hz), 3.25 (1H, dd, J = 16.7, 7.0 Hz), 3.41 (3H, s), 3.48 (1H, dd, J = 16.7, 6.6 Hz), 3.85 (3H, s), 4.14-4.28 (2H, m), 4.91 (1H, t, J = 6.9 Hz), 5.47 (1H, s), 6.86 (1H, d, J = 1.7 Hz), 6.96 (1H, d, J = 2.5 Hz), 7.19 (1H, dd, J = 2.5, 0.7 Hz), 7.51-7.57 (2H, m).

### Example 784

[(4S,6R)-8-Chloro-6-(5-chloro-2,3-dimethoxyphenyl)-1-(trifluoromethyl)-4H,6H-[1,2,4]triazolo[4,3-a][4,1]benzoxazepin-4-yl]acetic acid

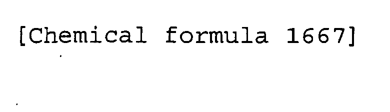

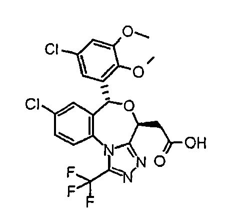

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (324 mg) was obtained from ethyl [(4S,6R)-8-chloro-6-(5-chloro-2,3,-dimethoxyphenyl)-1-(trifluoromethyl)-4H,6H-[1,2,4]triazolo[4,3-a][4,1]benzoxazepin-4-yl]acetate (403 mg).
MS (ESI) m/z : 518 (M+H)⁺.
¹H-NMR (CDCl₃) δ: 3.31 (1H, dd, J = 17.1, 6.4 Hz), 3.41 (3H, s), 3.58 (1H, dd, J = 17.1, 7.0 Hz), 3.86 (3H, s), 4.89 (1H, t, J = 6.9 Hz), 5.48 (1H, s), 6.86 (1H, d, J = 1.7 Hz), 6.96 (1H, d, J = 2.5 Hz), 7.20 (1H, d, J = 2.5 Hz), 7.52-7.57 (2H, m).
Elemental analysis for: C₂₁H₁₆Cl₂F₃N₃O₅
Calculated: C, 48.67; H, 3.11; N, 8.11; Cl, 13.68; F, 11.00.
Found: C, 48.62; H, 3.20; N, 8.06; Cl, 13.47; F, 10.75.

### Example 785

[(4R,6S)-8-Chloro-6-(5-chloro-2,3-dimethoxyphenyl)-1-(trifluoromethyl)-4H,6H-[1,2,4]triazolo[4,3-a][4,1]benzoxazepin-4-yl]acetic acid

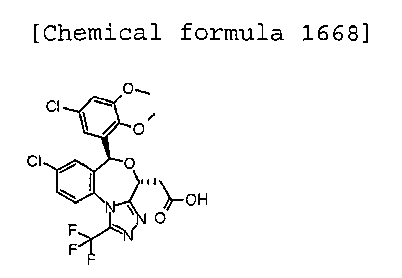

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (329 mg) was obtained from ethyl [(4R,6S)-8-chloro-6-(5-chloro-2,3-dimethoxyphenyl)-1-(trifluoromethyl)-4H,6H-[1,2,4]triazolo[4,3-a][4,1]benzoxazepin-4-yl]acetate (422 mg).
MS (ESI) m/z :518 (M+H)⁺.
¹H-NMR (CDCl₃) δ: 3.31 (1H, dd, J = 17.1, 6.5 Hz), 3.41 (3H, s), 3.59 (1H, dd, J = 17.1, 7.0 Hz), 3.85 (3H, s), 4.88 (1H, t, J = 6.9 Hz), 5.47 (1H, s), 6.86 (1H, s), 6.96 (1H, d, J = 2.5 Hz), 7.20 (1H, d, J = 2.5 Hz), 7.51-7.58 (2H, m).
Elemental analysis for: C₂₁H₁₆Cl₂F₃N₃O₅
Calculated: C, 48.67; H, 3.11; N, 8.11; Cl, 13.68; F, 11.00.
Found: C, 48.82; H, 3.27; N, 8.16; Cl, 13.38; F, 10.69.

### Referential Example 713

(2-Amino-5-bromophenyl)(2-chloro-3-methoxyphenyl)methanone

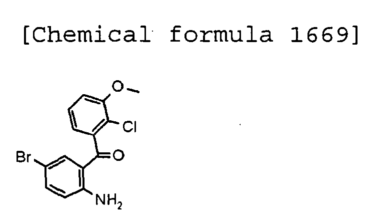

1-Chloro-2-methoxybenzene (7.12 g) was dissolved in tetrahydrofuran (200 mL). To the resulting solution was added dropwise sec-butyl lithium (a 1M solution, 50 mL) at -100°C. After stirring at the same temperature for 2 hours, a tetrahydrofuran solution (100 mL) of 6-bromo-2-methyl-4H-3,1-benzoxazin-4-one (10.0 g) was added dropwise at -100°C or less. The reaction mixture was warmed gradually to room temperature and was stirred overnight. The reaction mixture was concentrated. To the residue were added hydrochloric acid (30 mL), water (30 mL) and ethanol (300 mL). The resulting mixture was stirred at 90°C for 4 hours. The reaction mixture was allowed to cool and then, concentrated. The residue was made alkaline with a saturated aqueous solution of sodium bicarbonate and extracted with ethyl acetate. The organic layer was washed with saturated brine and dried over anhydrous sodium sulfate. The filtrate was concentrated. The residue was purified by silica gel column (ethyl acetate:hexane=15:85) to give the title compound (4.02 g) as a crudely purified product.
MS (ESI) m/z : 342 (M+H)⁺.
¹H-NMR (CDCl₃) δ: 3.97 (3H, s), 6.46 (2H, s), 6.63 (1H, d, J = 8.8 Hz), 6.89 (1H, dd, J = 7.6, 1.5 Hz), 7.04(1H, dd, J = 8.3, 1.5 Hz), 7.28-7.38 (3H, m).

### Referential Example 714

(2-Amino-5-bromophenyl)(2-chloro-3-methoxyphenyl)methanol

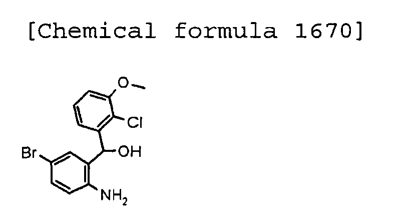

(2-Amino-5-bromophenyl)(2-chloro-3-methoxyphenyl)methanone (4.02 g) was dissolved in methanol (50 mL). To the resulting solution was added sodium borohydride (1.34 g) at room temperature. After stirring overnight, the reaction mixture was concentrated. To the residue was added a saturated aqueous solution of ammonium chloride. The resulting mixture was extracted with ethyl acetate. The organic layer was washed with saturated brine and dried over anhydrous sodium sulfate. The filtrate was concentrated. The residue was purified by silica gel column (ethyl acetate:hexane=3:7) to give the title compound (2.40 g).
MS (ESI) m/z : 326(M-OH)⁺.
¹H-NMR (CDCl₃) δ: 2.49 (1H, d, J = 4.9 Hz), 3.94 (3H, s), 4.07 (2H, br s), 6.19 (1H, d, J = 4.4 Hz), 6.59 (1H, d, J = 8.1 Hz), 6.95 (1H, d, J = 8.1 Hz), 7.05 (1H, d, J = 2.2 Hz), 7.08 (1H, d, J = 7.8 Hz), 7.21 (1H, dd, J = 8.6, 2.2 Hz), 7.29 (1H, t, J = 8.1 Hz).

### Referential Example 715

{5-Bromo-2-[(2,4-dimethoxybenzyl)amino]phenyl}(2-chloro-3-methoxyphenyl)methanol

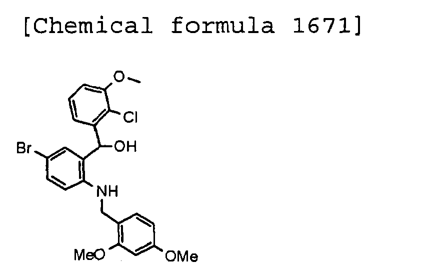

(2-Amino-5-bromophenyl)(2-chloro-3-methoxyphenyl)methanol (3.10 g) was dissolved in ethyl acetate. In a bath of ice water, 2,4-dimethoxybenzaldehyde (1.65 g) was added to the resulting solution. After stirring at the same temperature for 30 minutes, sodium borohydride (513 mg) was added and the resulting mixture was stirred for 2 hours. The reaction mixture was concentrated and the residue was partitioned between an aqueous solution of sodium bicarbonate and methylene chloride, followed by extraction. The organic layer was dried over anhydrous sodium sulfate. The filtrate was concentrated and the residue was purified by silica gel column (ethyl acetate:hexane=1:3) to give the title compound (3.92 g).
MS (ESI) m/z : 492 (M+H)⁺.
¹H-NMR (CDCl₃) δ: 2.48 (1H, br s), 3.79 (3H, s), 3.79 (3H, s), 3.93 (3H, s), 4.26 (2H, s), 4.95 (1H, br s), 6.17 (1H, s), 6.40 (1H, dd, J = 8.3, 2.5 Hz), 6.45 (1H, d, J = 2.2 Hz), 6.60 (1H, d, J = 8.8 Hz), 6.94 (1H, dd, J = 8.2, 1.3 Hz), 6.98 (1H, d, J = 2.5 Hz), 7.06-7.10 (2H, m), 7.23 (1H, dd, J = 8.3, 2.5 Hz), 7.27 (1H, t, J = 8.1 Hz).

### Referential Example 716

Ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl] acetate

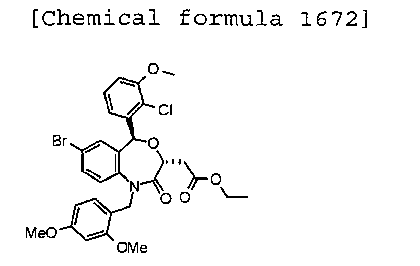

{5-Bromo-2-[(2,4-dimethoxybenzyl)amino]phenyl}(2-chloro-3-methoxyphenyl)methanol (4.32 g) was dissolved in methylene chloride (50 mL). In a bath of ice water, sodium hydrogen carbonate (2.21 g) and monoethyl chlorofumarate (2.18 g) were added and the resulting mixture was stirred at room temperature for 3 hours. The reaction mixture was diluted with dichloromethane and washed with a saturated aqueous solution of sodium bicarbonate and saturated brine. The organic layer was dried over anhydrous sodium sulfate. The filtrate was concentrated. The residue was dissolved in ethanol (50 mL). To the resulting solution was added potassium carbonate (3.68 g). The resulting mixture was stirred at room temperature for 3 days. The solvent was distilled off. The residue was partitioned between methylene chloride and water and washed. The organic layer was dried over anhydrous sodium sulfate. The filtrate was concentrated. To the residue was added ethyl acetate and the insoluble material was collected by filtration to give the title compound (3.87 g).
MS (ESI) m/z : 618 (M+H)⁺.
¹H-NMR (CDCl₃) δ: 1.26 (3H, t, J = 7.1 Hz), 2.80 (1H, dd, J = 16.5, 5.8 Hz), 3.10 (1H, dd, J = 16.5, 7.7 Hz), 3.60 (3H, s), 3.75 (3H, s), 3.90 (3H, s), 4.11-4.19 (2H, m), 4.46 (1H, dd, J = 7.7, 5.8 Hz), 4.86 (1H, d, J = 14.7 Hz), 5.48 (1H, d, J = 14.7 Hz), 5.87 (1H, s), 6.33 (1H, d, J = 2.5 Hz), 6.39 (1H, dd, J = 8.5, 2.5 Hz), 6.54 (1H, d, J = 2.2 Hz), 6.95 (1H, dd, J = 7.8, 1.5 Hz), 7.26-7.35 (4H, m), 7.48 (1H, dd, J = 8.6, 2.2 Hz).

### Referential Example 717

Ethyl [(trans)-7-bromo-5-(2-chloro-3-methoxyphenyl)-2-oxo-1,2,3,5-tetrahydro-4,1-benzoxazepin-3-yl]acetate

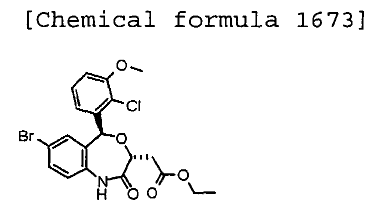

Ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate (2.48 g) was suspended in acetone (100 mL). At 0°C, an aqueous solution (10 mL) of ceric(IV) ammonium nitrate (6.60 g) was added and the resulting mixture was stirred at room temperature for 3 hours. Since remaining of the raw material was confirmed, an aqueous solution (5 mL) of ceric(IV) ammonium nitrate (3.30 g) was added further at 0°C and the resulting mixture was stirred for 1.5 hours. The reaction mixture was diluted with ethyl acetate and washed twice with water. The organic layer was dried over anhydrous sodium sulfate. The filtrate was then concentrated. The residue was purified by silica gel column (ethyl acetate:hexane= from 1:3 to 1:2) to give the title compound (1.55 g).
MS (ESI) m/z : 468 (M+H)⁺.
¹H-NMR (CDCl₃) δ: 1.24 (3H, t, J = 7.1 Hz), 2.80 (1H, dd, J = 16.5, 6.4 Hz), 3.04 (1H, dd, J = 16.7, 6.6 Hz), 3.94 (3H, s), 4.09-4.16 (2H, m), 4.61 (1H, t, J = 6.6 Hz), 6.21 (1H, s), 6.74 (1H, d, J = 2.2 Hz), 6.95 (1H, d, J = 8.6 Hz), 7.00 (1H, dd, J = 8.1, 1.2 Hz), 7.20 (1H, d, J = 7.8 Hz), 7.35 (1H, t, J = 8.1 Hz), 7.47 (1H, dd, J = 8.3, 2.2 Hz), 7.58 (1H, s).

### Referential Example 718

Ethyl [(trans)-7-bromo-5-(2-chloro-3-methoxyphenyl)-2-thioxo-1,2,3,5-tetrahydro-4,1-benzoxazepin-3-yl]acetate

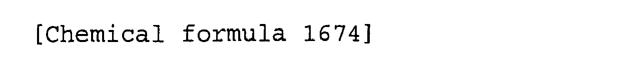

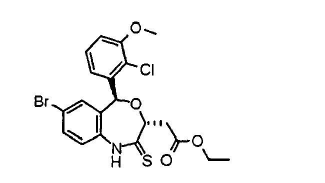

Ethyl [(trans)-7-bromo-5-(2-chloro-3-methoxyphenyl)-2-oxo-1,2,3,5-tetrahydro-4,1-benzoxazepin-3-yl]acetate (1.55 g) was dissolved in tetrahydrofuran (20 mL). To the resulting solution were added sodium hydrogen carbonate (611 mg) and diphosphorus pentasulfide (1.47 g). The resulting mixture was stirred at room temperature for 6 hours. The reaction mixture was diluted with ethyl acetate and washed with saturated brine. The organic layer was dried over anhydrous sodium sulfate. After concentration of the filtrate, the residue thus obtained was purified by silica gel column (ethyl acetate:hexane=1:4) to give the title compound (1.40 g).
MS (ESI) m/z : 486 (M+H)⁺.
¹H-NMR (CDCl₃) δ: 1.25 (3H, t, J = 7.1 Hz), 2.98 (1H, dd, J = 16.3, 6.7 Hz), 3.26 (1H, dd, J = 16.3, 6.7 Hz), 3.94 (3H, s), 4.14 (2H, q, J = 7.1 Hz), 4.69 (1H, t, J = 6.7 Hz), 6.12 (1H, s), 6.71 (1H, d, J = 2.2 Hz), 6.92-7.05 (2H, m), 7.23-7.28 (1H, m), 7.37 (1H, t, J = 8.1 Hz), 7.51-7.54 (1H, m), 9.49 (1H, s).

### Example 786

Ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate

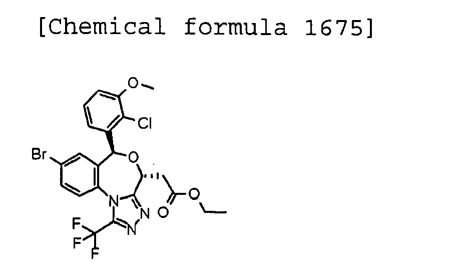

To a solution of ethyl [(trans)-7-bromo-5-(2-chloro-3-methoxyphenyl)-2-thioxo-1,2,3,5-tetrahydro-4,1-benzoxazepin-3-yl]acetate (1.40 g) in tetrahydrofuran (20 mL) was added hydrazine monohydrate (0.42 mL) in a bath of ice water. The resulting mixture was stirred for one hour. The reaction mixture was diluted with ethyl acetate and washed with water. The organic layer was washed with saturated brine and dried over anhydrous sodium sulfate. The filtrate was concentrated to give a hydrazone derivative (1.37 g) as a crudely purified product. The resulting hydrazone derivative (1.37 g) was dissolved in 1,2-dichloroethane (10 mL). To the resulting solution was added trifluoroacetic anhydride (1.2 mL) at room temperature and the resulting mixture was stirred for 30 minutes. The reaction mixture was concentrated and the residue was dissolved in toluene (10 mL). To the resulting solution was added trifluoroacetic anhydride (1.2 mL). The resulting mixture was heated under reflux for one hour.
The reaction mixture was concentrated. The residue was purified by silica gel column (ethyl acetate:hexane=1:10) to give the title compound (1.40 g).

### Example 787

Ethyl [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetate (isomer A)

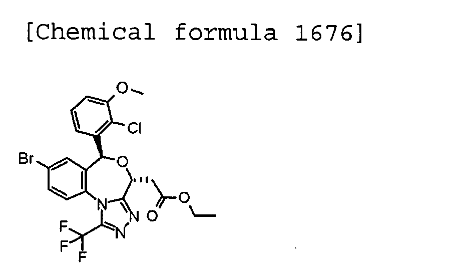

Ethyl [(4S,6R)-8-bromo-6-(2-chloro-3-methoxyphenyl)-1-(trifluoromethyl)-4H,6H-[1,2,4]triazolo[4,3-a][4,1]benzoxazepin-4-yl]acetate(isomer B)

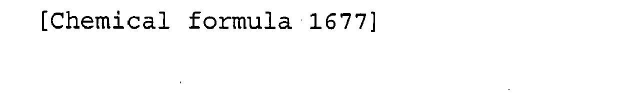

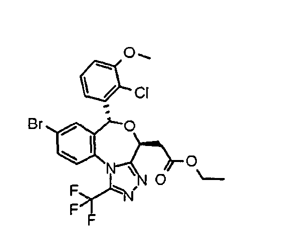

Ethyl [(trans)-8-bromo-6-(2-chloro-3-methoxyphenyl)-1-(trifluoromethyl)-4H,6H-[1,2,4]triazolo[4,3-a] [4,1]benzoxazepin-4-yl]acetate (255 mg) was optically resolved by an CHIRALPAK AD (isopropanol:hexane=2:8) into ethyl [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetate (isomer A, 98 mg) and ethyl [(4S,6R)-8-bromo-6-(2-chloro-3-methoxyphenyl)-1-(trifluoromethyl)-4H,6H-[1,2,4]triazolo[4,3-a][4,1]benzoxazepin-4-yl]acetate (isomer B, 107 mg).
Resolution conditions:
Flow rate: 50 mL/min
Developing solvent: 20% isopropanol- n-hexane
Retention time: isomer A: 37 min., isomer B: 52 min.
Ethyl [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetate
MS (ESI) m/z : 562 (M+H)⁺.
¹H-NMR (CDCl₃) δ: 1.29 (3H, t, J = 7.1 Hz), 3.26 (1H, dd, J = 16.9, 7.1 Hz), 3.50 (1H, dd, J = 16.9, 6.4 Hz), 3.92 (3H, s), 4.15-4.24 (2H, m), 4.95 (1H, t, J = 6.7 Hz), 5.55 (1H, s), 6.88 (1H, d, J = 2.2 Hz), 7.01 (1H, dd, J = 7.8, 1.5 Hz), 7.34 (1H, dd, J = 7.8, 1.5 Hz), 7.40 (1H, t, J = 8.0 Hz), 7.47 (1H, d, J = 8.6 Hz), 7.71 (1H, dd, J = 8.6, 2.2 Hz).
Ethyl [(4S,6R)-8-bromo-6-(2-chloro-3-methoxyphenyl)-1-(trifluoromethyl)-4H,6H-[1,2,4]triazolo[4,3-a] [4,1]benzoxazepin-4-yl]acetate
MS (ESI) m/z : 562 (M+H)⁺.
¹H-NMR (CDCl₃) δ: 1.29 (3H, t, J = 7.1 Hz), 3.26 (1H, dd, J = 16.9, 7.1 Hz), 3.50 (1H, dd, J = 16.9, 6.4 Hz), 3.92 (3H, s), 4.15-4.25 (2H, m), 4.95 (1H, t, J = 6.7 Hz), 5.55 (1H, s), 6.88 (1H, d, J = 2.2 Hz), 7.01 (1H, dd, J = 8.1, 1.5 Hz), 7.34 (1H, dd, J = 7.8, 1.5 Hz), 7.40 (1H, t, J = 8.1 Hz), 7.47 (1H, d, J = 8.6 Hz), 7.71 (1H, dd, J = 8.6, 2.2 Hz).

### Example 788

[(4R,6S)-8-Bromo-6-(2-chloro-3-methoxyphenyl)-1-(trifluoromethyl)-4H,6H-[1,2,4]triazolo[4,3-a] [4,1]benzoxazepin-4-yl]acetic acid

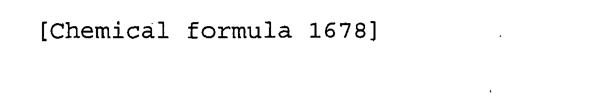

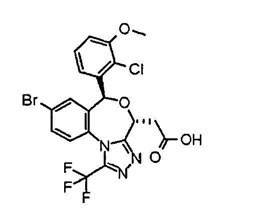

Ethyl [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetate (97 mg) was dissolved in dioxane (4 mL). To the resulting -solution were added concentrated hydrochloric acid (2 mL) and water (2 mL). At 60°C, the resulting mixture was stirred overnight. After the reaction mixture was allowed to cool, the solvent was distilled off, followed by azeotropy of the residue with dioxane and methylene chloride. The residue was dissolved in chloroform. To the resulting solution was added hexane. From the resulting mixture, the solvent was distilled off. To the residue was added hexane and the insoluble material was collected by filtration to give the title compound (75 mg).
MS (ESI) m/z : 532 (M+H)⁺.
¹H-NMR (CDCl₃) δ: 3.34 (1H, dd, J = 16.9, 6.9 Hz), 3.58 (1H, dd, J = 16.9, 6.9 Hz), 3.92 (3H, s), 4.93 (1H, t, J = 6.9 Hz), 5.56 (1H, s), 6.89 (1H, d, J = 2.2 Hz), 7.01 (1H, dd, J = 8.1, 1.5 Hz), 7.35 (1H, dd, J = 7.8, 1.5 Hz), 7.40 (1H, t, J = 8.1 Hz), 7.48 (1H, d, J = 8.6 Hz), 7.72 (1H, dd, J = 8.6, 2.2 Hz).
Elemental analysis for: C₂₀H₁₄BrClF₃N₃O₄·0.5H₂O·0.1 hexane
Calculated: C, 44.96; H, 3.00; N, 7.64; Br, 14.52; Cl, 6.44; F, 10.35.
Found: C, 45.17; H, 2.90; N, 7.74; Br, 14.20; Cl, 6.25; F, 9.99.

### Example 789

[(4S,6R)-8-Bromo-6-(2-chloro-3-methoxyphenyl)-1-(trifluoromethyl)-4H,6H-[1,2,4]triazolo[4,3-a] [4,1]benzoxazepin-4-yl]acetic acid

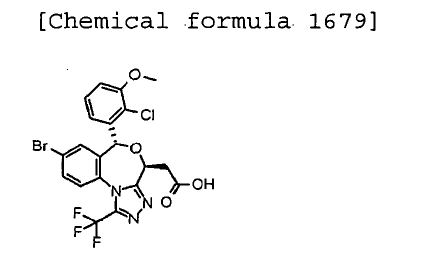

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (79 mg) was obtained from ethyl [(4S,6R)-8-bromo-6-(2-chloro-3-methoxyphenyl)-1-(trifluoromethyl)-4H,6H-[1,2,4]triazolo[4,3-a][4,1]benzoxazepin-4-yl]acetate (105 mg) .
MS (ESI) m/z : 532 (M+H)⁺.
¹H-NMR (CDCl₃) δ: 3.33 (1H, dd, J = 17.0, 6.9 Hz), 3.58 (1H, dd, J = 17.0, 6.9 Hz), 3.92 (3H, s), 4.93 (1H, t, J = 6.9 Hz), 5.56 (1H, s), 6.89 (1H, d, J = 2.0 Hz), 7.01 (1H, dd, J = 8.1, 1.5 Hz), 7.35 (1H, dd, J = 8.1, 1.5 Hz), 7.40 (1H, t, J = 8.1 Hz), 7.48 (1H, d, J = 8.1 Hz), 7.72 (1H, dd, J = 8.3, 2.0 Hz).
Elemental analysis for: C₂₀H₁₄BrClF₃N₃O₄·0.1 hexane Calculated: C, 45.71; H, 2.87; N, 7.76; Br, 14.76; Cl, 6.55; F, 10.52.
Found: C, 45.46; H, 2.98; N, 7.83; Br, 14.60; Cl, 6.43; F, 10.55.

### Example 790

Ethyl [(trans)-8-bromo-1-[chloro(difluoro)methyl]-6-(2-chloro-3-methoxyphenyl)-4H,6H-[1,2,4]triazolo[4,3-a][4,1]benzoxazepin-4-yl]acetate

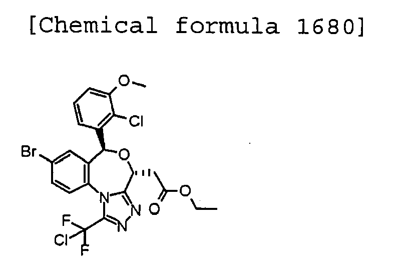

To a solution of ethyl [(trans)-7-bromo-5-(2-chloro-3-methoxyphenyl)-2-thioxo-1,2,3,5-tetrahydro-4,1-benzoxazepin-3-yl]acetate (1.04 g) in tetrahydrofuran (20 mL) was added hydrazine monohydrate (0.32 mL) in a bath of ice water and the resulting mixture was stirred for 30 minutes. The reaction mixture was diluted with ethyl acetate and washed with water. The organic layer was washed with saturated brine and dried over anhydrous sodium sulfate. The filtrate was concentrated to give a hydrazone derivative (1.08 g) as a crudely purified product. The resulting hydrazone derivative (454 mg) was dissolved in 1,2-dichloroethane (10 mL). To the resulting solution was added chlorodifluoroacetic anhydride (0.7 mL) at room temperature and the resulting mixture was stirred for 30 minutes. The reaction mixture was concentrated and the residue was dissolved in toluene (10 mL). To the resulting solution was added trifluoroacetic anhydride (0.7 mL). The resulting mixture was heated under reflux for one hour.
The reaction mixture was concentrated. The residue was purified by silica gel column (ethyl acetate:hexane=1:3) to give the title compound (536 mg).
MS (ESI) m/z : 578 (M+H)⁺.
¹H-NMR (CDCl₃) δ: 1.29 (3H, t, J = 7.1 Hz), 3.26 (1H, dd, J = 16.7, 7.1 Hz), 3.50 (1H, dd, J = 16.7, 6.4 Hz), 3.91 (3H, s), 4.15-4.25 (2H, m), 4.92 (1H, dd, J = 7.1, 6.4 Hz), 5.55 (1H, s), 6.87 (1H, d, J = 2.2 Hz), 7.01 (1H, dd, J = 8.1, 1.5 Hz), 7.33-7.43 (2H, m), 7.52 (1H, d, J = 8.6 Hz), 7.71 (1H, dd, J = 8.6, 2.2 Hz).

### Example 791

Ethyl [(4R,6S)-8-bromo-1-[chloro(difluoro)methyl]-6-(2-chloro-3-methoxyphenyl)-4H,6H-[1,2,4]triazolo[4,3-a] [4,1]benzoxazepin-4-yl]acetate (isomer A)

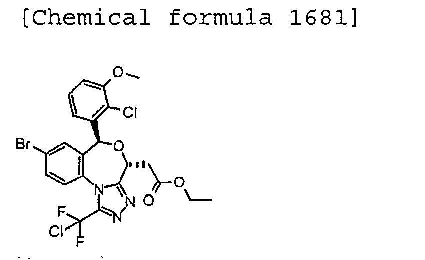

Ethyl [(4S,6R)-8-bromo-1-[chloro(difluoro)methyl]-6-(2-chloro-3-methoxyphenyl)-4H,6H-[1,2,4]triazolo[4,3-a][4,1]benzoxazepin-4-yl]acetate(isomer B)

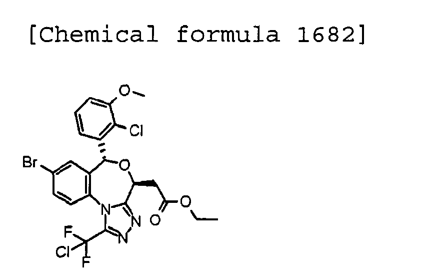

Ethyl [(trans)-8-bromo-1-[chloro(difluoro)methyl]-6-(2-chloro-3-methoxyphenyl)-4H,6H-[1,2,4]triazolo[4,3-a][4,1]benzoxazepin-4-yl]acetate (536 mg) was optically resolved by an optically active column (CHIRALPAK-OD; 5 cm × 50 cm) into ethyl [(4R,6S)-8-bromo-1-[chloro(difluoro)methyl]-6-(2-chloro-3-methoxyphenyl)-4H,6H-[1,2,4]triazolo[4,3-a][4,1]benzoxazepin-4-yl] acetate (isomer A, 237 mg) and ethyl [(4S,6R)-8-bromo-1-[chloro(difluoro)methyl]-6-(2-chloro-3-methoxyphenyl)-4H,6H-[1,2,4]triazolo[4,3-a][4,1]benzoxazepin-4-yl]acetate (isomer B, 234 mg).
Resolution conditions:
Flow rate: 50 mL/min
Developing solvent: 20% isopropanol- n-hexane
Retention time: isomer A: 30 min., isomer B: 54 min.

### Example 792

[(4R,6S)-8-Bromo-1-[chloro(difluoro)methyl]-6-(2-chloro-3-methoxyphenyl)-4H,6H-[1,2,4]triazolo[4,3-a][4,1]benzoxazepin-4-yl]acetic acid

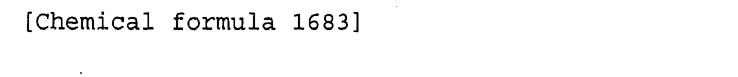

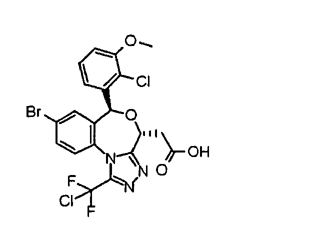

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (183 mg) was obtained from ethyl [(4R,6S)-8-bromo-1-[chloro(difluoro)methyl]-6-(2-chloro-3-methoxyphenyl)-4H,6H-[1,2,4]triazolo[4,3-a][4,1]benzoxazepin-4-yl]acetate (235 mg) .
MS (ESI) m/z : 550 (M+H)⁺.
¹H-NMR (CDCl₃) δ: 3.33 (1H, dd, J = 17.2, 6.6 Hz), 3.58 (1H, dd, J = 17.2, 6.6 Hz), 3.91 (3H, s), 4.90 (1H, t, J = 6.6 Hz), 5.56 (1H, s), 6.88 (1H, d, J = 2.2 Hz), 7.01 (1H, dd, J = 8.1, 1.5 Hz), 7.33-7.43 (2H, m), 7.53 (1H, d, J = 8.3 Hz), 7.71 (1H, dd, J = 8.3, 2.2 Hz).
Elemental analysis for: C₂₀H₁₄BrCl₂F₂N₃O₄·0.15 hexane·0.05 chloroform
Calculated: C, 44.30; H, 2.68; N, 7.40; Br, 14.07; Cl, 13.39; F, 6.69.
Found: C, 44.61; H, 2.85; N, 7.33; Br, 12.86; Cl, 13.39; F, 7.02.

### Example 793

[(4S,6R)-8-Bromo-1-[chloro(difluoro)methyl]-6-(2-chloro-3-methoxyphenyl)-4H,6H-[1,2,4]triazolo[4,3-a][4,1]benzoxazepin-4-yl]acetic acid

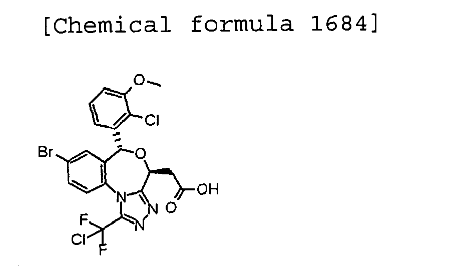

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (191 mg) was obtained from ethyl [(4S,6R)-8-bromo-1-[chloro(difluoro)methyl]-6-(2-chloro-3-methoxyphenyl)-4H,6H-[1,2,4]triazolo[4,3-a][4,1]benzoxazepin-4-yl]acetate (232 mg).
MS (ESI) m/z : 550 (M+H)⁺.
¹H-NMR (CDCl₃) δ: 3.33 (1H, dd, J = 17.2, 6.9 Hz), 3.58 (1H, dd, J = 17.2, 6.6 Hz), 3.91 (3H, s), 4.90 (1H, t, J = 6.6 Hz), 5.56 (1H, s), 6.88 (1H, d, J = 2.2 Hz), 7.01 (1H, dd, J = 8.1, 1.7 Hz), 7.35 (1H, dd, J = 7.8, 1.7 Hz), 7.40 (1H, t, J = 7.8 Hz), 7.53 (1H, d, J = 8.1 Hz), 7.71 (1H, dd, J = 8.3, 2.2 Hz).
Elemental analysis for: C₂₀H₁₄BrCl₂F₂N₃O₄·0.1 hexane Calculated: C, 44.36; H, 2.78; N, 7.53; Br, 14.32; C1, 12.71; F, 6.81.
Found: C, 44.07; H, 2.83; N, 7.28; Br, 13.92; Cl, 13.14; F, 6.77.

### Example 794

Ethyl [(trans)-8-bromo-6-(2-chloro-3-methoxyphenyl)-1-(1,1-difluoroethyl)-4H,6H-[1,2,4]triazolo[4,3-a] [4,1]benzoxazepin-4-yl]acetate

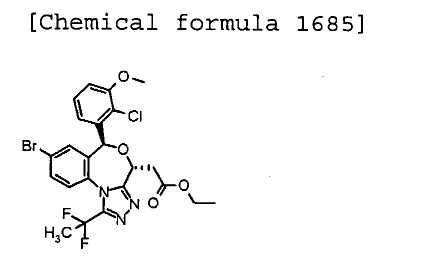

To a solution of ethyl [(trans)-7-bromo-5-(2-chloro-3-methoxyphenyl)-2-thioxo-1,2,3,5-tetrahydro-4,1-benzoxazepin-3-yl]acetate (1.04 g) in tetrahydrofuran (20 mL) was added hydrazine monohydrate (0.32 mL) in a bath of ice water. The resulting mixture was stirred for 30 minutes. The reaction mixture was diluted with ethyl acetate and washed with water. The organic layer was washed with saturated brine and dried over anhydrous sodium sulfate. The filtrate was concentrated to give a hydrazone derivative (1.08 g) as a crudely purified product. The resulting hydrazone derivative (623 mg) was dissolved in dichloromethane (10 mL). To the resulting solution were added 2,2-difluoropropionic acid (having a purity of about 60%) (312 mg), 1-ethyl-3-(3-dimethylaminopropyl)carbodiimide hydrochloride (477 mg), and 1-hydroxybenzotriazole (190 mg) at room temperature. The resulting mixture was stirred overnight. The reaction mixture was washed with water and the organic layer was dried over anhydrous sodium sulfate. The filtrate was concentrated. The residue was purified by silica gel column (ethyl acetate:hexane=1:3) to give the title compound (534 mg).

### Example 795

Ethyl [(4R,6S)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (isomer A)

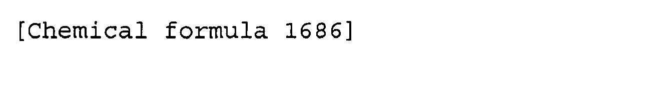

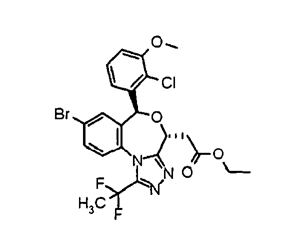

Ethyl [(4S,6R)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (isomer B)

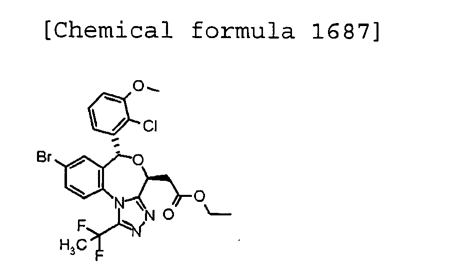

Ethyl [(trans)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (534 mg) was optically resolved by an optically active column (CHIRALPAK-OD; 5 cm × 50 cm) into ethyl [(4R,6S)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (isomer A, 278 mg) and [(4S,6R)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (isomer B, 255 mg).
Flow rate: 50 mL/min
Developing solvent: 20% isopropanol- n-hexane Retention time: isomer A: 33 min., isomer B: 47 min.
Ethyl [(4R,6S)-8-bromo-6-(2-chloro-3-methoxyphenyl)-1-(1,1-difluoroethyl)-4H,6H-[1,2,4]triazolo[4,3-a][4,1]benzoxazepin-4-yl]acetate
MS (ESI) m/z : 558 (M+H)⁺.
¹H-NMR (CDCl₃) δ: 1.29 (3H, t, J = 7.1 Hz), 2.32 (3H, t, J = 19.4 Hz), 3.25 (1H, dd, J = 16.7, 7.4 Hz), 3.47 (1H, dd, J = 16.7, 6.1 Hz), 3.91 (3H, s), 4.16-4.24 (2H, m), 4.92 (1H, dd, J = 7.1, 6.4 Hz), 5.56 (1H, s), 6.83 (1H, d, J = 1.5 Hz), 7.00 (1H, dd, J = 7.8, 1.7 Hz), 7.33-7.42 (2H, m), 7.63-7.69 (2H, m).
Ethyl [(4S,6R)-8-bromo-6-(2-chloro-3-methoxyphenyl)-1-(1,1-difluoroethyl)-4H,6H-[1,2,4]triazolo[4,3-a][4,1]benzoxazepin-4-yl]acetate
MS (ESI) m/z : 558 (M+H)⁺.
¹H-NMR (CDCl₃) δ: 1.29 (3H, t, J = 7.1 Hz), 2.32 (3H, t, J = 19.4 Hz), 3.25 (1H, dd, J = 16.7, 7.6 Hz), 3.47 (1H, dd, J = 16.7, 6.1 Hz), 3.91 (3H, s), 4.15-4.25 (2H, m), 5.05 (1H, t, J = 6.6 Hz), 5.56 (1H, s), 6.83 (1H, d, J = 1.7 Hz), 7.00 (1H, dd, J = 7.8, 1.7 Hz), 7.33-7.41 (2H, m), 7.62-7.69 (2H, m).

### Example 796

[(4R,6S)-8-Bromo-6-(2-chloro-3-methoxyphenyl)-1-(1.,1-difluoroethyl)-4H,6H-[1,2,4]triazolo[4,3-a][4,1]benzoxazepin-4-yl]acetic acid

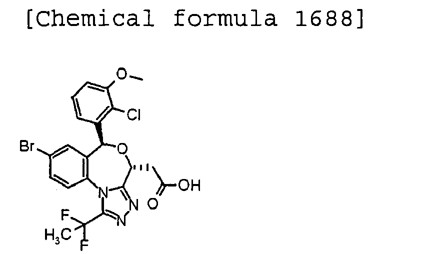

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (237 mg) was obtained from ethyl [(4R,6S)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (275 mg).
MS (ESI) m/z : 530 (M+H)⁺.
¹H-NMR (CDCl₃) δ: 2.32 (3H, t, J = 19.2 Hz), 3.31 (1H, dd, J = 16.9, 6.6 Hz), 3.55 (1H, dd, J = 16.9, 6.6 Hz), 3.91 (3H, s), 4.89 (1H, t, J = 6.6 Hz), 5.56 (1H, s), 6.84 (1H, d, J = 1.7 Hz), 7.00 (1H, dd, J = 7.6, 2.2 Hz), 7.34-7.42 (2H, m), 7.62-7.70 (2H, m).
Elemental analysis for: C₂₁H₁₇BrClF₂N₃O₄·0.2 hexane·0.05 chloroform
Calculated: C, 48.42; H, 3.61; N, 7.61; Br, 14.47; Cl, 7.39; F, 6.88.
Found: C, 48.40; H, 3.50; N, 7.64; Br, 13.48; Cl, 7.19; F, 7.09.

### Example 797

[(4S,6R)-8-Bromo-6-(2-chloro-3-methoxyphenyl)-1-(1,1-difluoroethyl)-4H,6H-[1,2,4]triazolo[4,3-a][4,1]benzoxazepin-4-yl]acetic acid

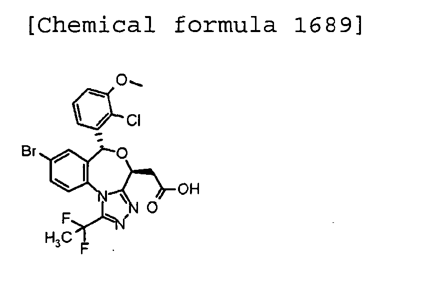

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (224 mg) was obtained from ethyl [(4S,6R)-8-bromo-6-(2-chloro-3-methoxyphenyl)-1-(1,1-difluoroethyl)-4H,6H-[1,2,4]triazolo[4,3-a][4,1]benzoxazepin-4-yl]acetate (253 mg).
MS (ESI) m/z : 530 (M+H)⁺.
¹H-NMR (CDCl₃) δ: 2.32 (3H, t, J = 19.1 Hz), 3.32 (1H, dd, J = 16.9, 6.6 Hz), 3.55 (1H, dd, J = 16.9, 6.6 Hz), 3.91 (3H, s), 4.89 (1H, t, J = 6.6 Hz), 5.56 (1H, s), 6.84 (1H, d, J = 1.7 Hz), 7.00 (1H, dd, J = 7.8, 2.2 Hz), 7.34-7.42 (2H, m), 7.63-7.70 (2H, m).
Elemental analysis for: C₂₁H₁₇BrClF₂N₃O₄·0.1H₂O
Calculated: C, 47.54; H, 3.27; N, 7.92; Br, 15.06; Cl, 6.68; F, 7.16.
Found: C, 47.83; H, 3.34; N, 7.53; Br, 14.74; Cl, 6.89; F, 7.22.

### Referential Example 719

Ethyl [(trans)-5-(2-chloro-3-methoxyphenyl)-7-cyano-1-(2,4-dimethoxybenzyl)-2-oxo-1,2,3,5-tetrahydro-4,1-benzoxazepin-3-yl]acetate

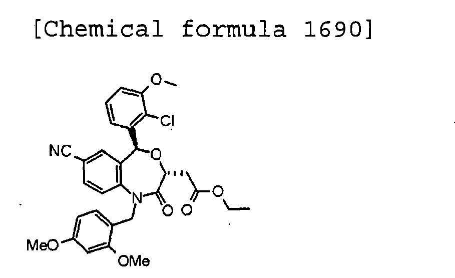

In an argon atmosphere, ethyl [(trans)-7-bromo-5-(2-chloro-3-methoxyphenyl)-1-(2,4-dimethoxybenzyl)-2-oxo-1,2,3,5-tetrahydro-4,1-benzoxazepin-3-yl]acetate (4.0 g) was dissolved in acetonitrile (160 mL). To the resulting solution were added sodium cyanide (866 mg), 4,5-bis(diphenylphosphino)-9,9-dimethylxanthene (240 mg), and tris(dibenzylidene acetone)dipalladium(0) (320 mg). To the resulting mixture was added tributyltin chloride (0.05M, a hexane solution) (4.4 mL) at room temperature. After stirring for a while, the reaction mixture was stirred overnight at 80°C. The reaction mixture was diluted with ethyl acetate and washed with saturated brine. The organic layer was dried over anhydrous sodium sulfate. The filtrate was concentrated. The residue was purified by silica gel column (ethyl acetate:hexane=1:2) to give the title compound (2.12 g).
MS (ESI) m/z : 565 (M+H)⁺.
¹H-NMR (CDCl₃) δ: 1.26 (3H, t, J = 7.1 Hz), 2.82 (1H, dd, J = 16.7, 5.6 Hz), 3.13 (1H, dd, J = 16.5, 7.8 Hz), 3.59 (3H, s), 3.75 (3H, s), 3.91 (3H, s), 4.08-4.19 (2H, m), 4.44 (1H, dd, J = 7.8, 5.6 Hz), 4.90 (1H, d, J = 15.0 Hz), 5.52 (1H, d, J = 15.0 Hz), 5.88 (1H, s), 6.33 (1H, d, J = 2.5 Hz), 6.39 (1H, dd, J = 8.3, 2.5 Hz), 6.74 (1H, d, J = 2.0 Hz), 6.98 (1H, dd, J = 8.1, 1.2 Hz), 7.25-7.29 (1H, m), 7.31 (1H, d, J = 8.3 Hz), 7.36 (1H, t, J = 8.1 Hz), 7.54 (1H, d, J = 8.6 Hz), 7.64 (1H, dd, J = 8.6, 2.0 Hz).

### Referential Example 720

Ethyl [(trans)-5-(2-chloro-3-methoxyphenyl)-7-cyano-2-oxo-1,2,3,5-tetrahydro-4,1-benzoxazepin-3-yl]acetate

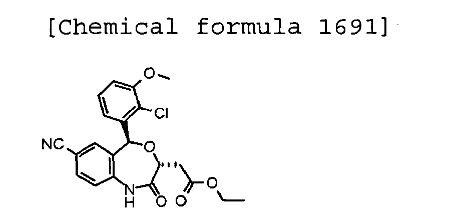

In a similar manner to that employed for the synthesis of ethyl [(trans)-7-bromo-5-(2-chloro-3-methoxyphenyl)-2-oxo-1,2,3,5-tetrahydro-4,1-benzoxazepin-3-yl]acetate, the title compound (1.40 g) was obtained from ethyl [(trans)-5-(2-chloro-3-methoxyphenyl)-7-cyano-1-(2,4-dimethoxybenzyl)-2-oxo-1,2,3,5-tetrahydro-4,1-benzoxazepin-3-yl]acetate (2.25 g).
MS (ESI) m/z : 415 (M+H)⁺.
¹H-NMR (CDCl₃) δ: 1.22 (3H, t, J = 7.1 Hz), 2.84 (1H, dd, J = 16.5, 6.5 Hz), 3.07 (1H, dd, J = 16.5, 6.5 Hz), 3.96 (3H, s), 4.12 (2H, q, J = 7.1 Hz), 4.66 (1H, t, J = 6.5 Hz), 6.22 (1H, s), 6.94 (1H, d, J = 1.5 Hz), 7.03 (1H, d, J = 8.1 Hz), 7.11-7.16 (2H, m), 7.36 (1H, t, J = 8.1 Hz), 7.63 (1H, dd, J = 8.3, 1.7 Hz), 7.78 (1H, s).

### Referential Example 721

Ethyl [(trans)-5-(2-chloro-3-methoxyphenyl)-7-cyano-2-thioxo-1,2,3,5-tetrahydro-4,1-benzoxazepin-3-yl]acetate

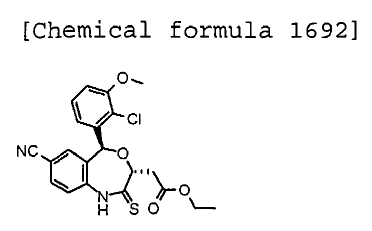

In a similar manner to that employed for the synthesis of ethyl [(trans)-7-bromo-5-(2-chloro-3-methoxyphenyl)-2-thioxo-1,2,3,5-tetrahydro-4,1-benzoxazepin-3-yl]acetate, the title compound (1.27 g) was obtained from ethyl [(trans)-5-(2-chloro-3-methoxyphenyl)-7-cyano-2-oxo-1,2,3,5-tetrahydro-4,1-benzoxazepin-3-yl] acetate (1.40 g).
MS (ESI) m/z : 431 (M+H)⁺.
¹H-NMR (CDCl₃) δ: 1.25 (3H, t, J = 7.1 Hz), 2.99 (1H, dd, J = 16.4, 6.4 Hz), 3.28 (1H, dd, J = 16.4, 6.6 Hz), 3.95 (3H, s), 4.14 (2H, q, J = 7.1 Hz), 4.71 (1H, t, J = 6.6 Hz), 6.15 (1H, s), 6.90 (1H, d, J = 1.7 Hz), 7.04 (1H, d, J = 8.1 Hz), 7.19-7.25 (2H, m), 7.39 (1H, t, J = 8.1 Hz), 7.69 (1H, dd, J = 8.3, 2.0 Hz), 9.60 (1H, s).

### Example 798

Ethyl [(trans)-6-(2-chloro-3-methoxyphenyl)-8-cyano-1-(trifluoromethyl)-4H,6H-[1,2,4]triazolo[4,3-a] [4,1]benzoxazepin-4-yl]acetate

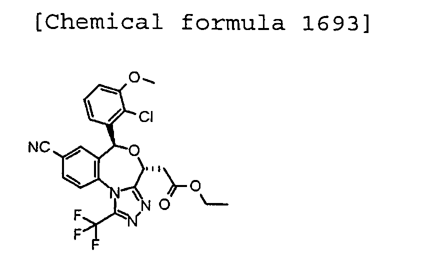

In a bath of ice water, hydrazine monohydrate (0.43 mL) was added to a solution of ethyl [(trans)-5-(2-chloro-3-methoxyphenyl)-7-cyano-2-thioxo-1,2,3,5-tetrahydro-4,1-benzoxazepin-3-yl]acetate (1.27 g) in tetrahydrofuran (20 mL) and the resulting mixture was stirred for one hour. The reaction mixture was diluted with ethyl acetate and washed with water. The organic layer was washed with saturated brine and dried over anhydrous sodium sulfate. The filtrate was concentrated to give a hydrazone derivative (1.376 g) as a crudely purified product. The hydrazone derivative (455 mg) was dissolved in 1,2-dichloroethane (10 mL). To the resulting solution was added trifluoroacetic anhydride (0.39 mL) and the resulting mixture was stirred for 30 minutes. The reaction mixture was concentrated and the residue was dissolved in toluene (10 mL). To the resulting solution was added trifluoroacetic anhydride (0.39 mL) and the resulting mixture was heated under reflux for one hour. The reaction mixture was concentrated and the residue was purified by silica gel column (ethyl acetate:hexane=1:2) to give the title compound (483 mg).

### Example 799

Ethyl [(4R,6S)-6-(2-chloro-3-methoxyphenyl)-8-cyano-1-(trifluoromethyl)-4H,6H-[1,2,4]triazolo[4,3-a][4,1]benzoxazepin-4-yl]acetate (isomer A)

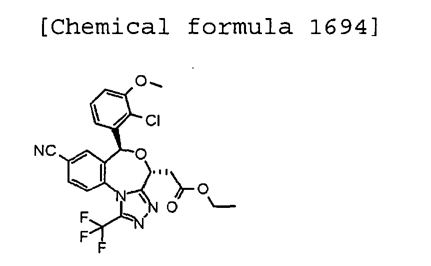

Ethyl [(4S,6R)-6-(2-chloro-3-methoxyphenyl)-8-cyano-1-(trifluoromethyl)-4H,6H-[1,2,4]triazolo[4,3-a][4,1]benzoxazepin-4-yl]acetate(isomer B)

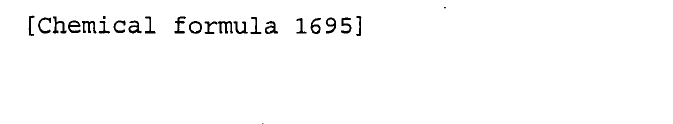

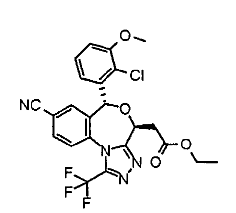

Ethyl [(trans)-6-(2-chloro-3-methoxyphenyl)-8-cyano-1-(trifluoromethyl)-4H,6H-[1,2,4]triazolo[4,3-a][4,1]benzoxazepin-4-yl]acetate (483 mg) was optically resolved by an optically active column (CHIRALPAK-AD; 5 cm × 50 cm) into ethyl [(4R,6S)-6-(2-chloro-3-methoxyphenyl)-8-cyano-1-(trifluoromethyl)-4H,6H-[1,2,4]triazolo[4,3-a][4,1]benzoxazepin-4-yl]acetate (isomer A, 249 mg) and ethyl [(4S,6R)-6-(2-chloro-3-methoxyphenyl)-8-cyano-1-(trifluoromethyl)-4H,6H-[1,2,4]triazolo[4,3-a][4,1]benzoxazepin-4-yl]acetate (isomer B, 253 mg).
Resolution conditions:
Flow rate: 50 mL/min
Developing solvent: 30% isopropanol- n-hexane
Retention time: isomer A: 36 min., isomer B: 63 min.
   Ethyl [(4R,6S)-6-(2-chloro-3-methoxyphenyl)-8-cyano-1-(trifluoromethyl)-4H,6H-[1,2,4]triazolo[4,3-a][4,1]benzoxazepin-4-yl]acetate
   MS (ESI) m/z : 507 (M+H)⁺.
   ¹H-NMR (CDCl₃) δ: 1.29 (3H, t, J = 7.1 Hz), 3.27 (1H, dd, J = 16.8, 7.1 Hz), 3.53 (1H, dd, J = 16.8, 6.6 Hz), 3.93 (3H, s), 4.20 (2H, q, J = 7.1 Hz), 4.95 (1H, t, J = 6.7 Hz), 5.60 (1H, s), 7.02-7.09 (2H, m), 7.35 (1H, d, J = 7.8 Hz), 7.43 (1H, t, J = 8.0 Hz), 7.74 (1H, d, J = 8.3 Hz), 7.89 (1H, dd, J = 8.3, 1.7 Hz) .
   Ethyl [(4S,6R)-6-(2-chloro-3-methoxyphenyl)-8-cyano-1-(trifluoromethyl)-4H,6H-[1,2,4]triazolo[4,3-a] [4,1]benzoxazepin-4-yl]acetate
   MS (ESI) m/z : 507 (M+H)⁺.
   ¹H-NMR (CDCl₃) δ: 1.29 (3H, t, J = 7.1 Hz), 3.27 (1H, dd, J = 16.9, 6.9 Hz), 3.53 (1H, dd, J = 16.9, 6.6 Hz), 3.93 (3H, s), 4.17-4.24 (2H, m), 4.95 (1H, t, J = 6.6 Hz), 5.60 (1H, s), 7.02-7.09 (2H, m), 7.35 (1H, dd, J = 7.8, 1.5 Hz), 7.43 (1H, t, J = 8.0 Hz), 7.73 (1H, d, J = 8.3 Hz), 7.89 (1H, dd, J = 8.3, 1.7 Hz).

### Example 800

[(4R,6S)-6-(2-Chloro-3-methoxyphenyl)-8-cyano-1-(trifluoromethyl)-4H,6H-[1,2,4]triazolo[4,3-a] [4,1]benzoxazepin-4-yl]acetic acid

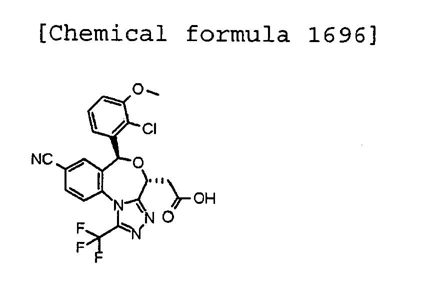

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (178 mg) was obtained from ethyl [(4R,6S)-6-(2-chloro-3-methoxyphenyl)-8-cyano-1-(trifluoromethyl)-4H,6H-[1,2,4]triazolo[4,3-a][4,1]benzoxazepin-4-yl]acetate (247 mg).
MS (ESI) m/z : 479 (M+H)⁺.
¹H-NMR (CDCl₃) δ: 3.34 (1H, dd, J = 17.2, 6.9 Hz), 3.61 (1H, dd, J = 17.2, 6.9 Hz), 3.94 (3H, s), 4.92 (1H, t, J = 6.7 Hz), 5.60 (1H, s), 7.05 (1H, dd, J = 8.3, 1.5 Hz), 7.08 (1H, d, J = 2.0 Hz), 7.34-7.38 (1H, m), 7.43 (1H, t, J = 8.1 Hz), 7.74 (1H, d, J = 8.3 Hz), 7.89 (1H, dd, J = 8.3, 2.0 Hz).
Elemental analysis for: C₂₁H₁₄ClF₃N₄O₄·0.4H₂O·0.1 hexane Calculated: C, 52.45; H, 3.30; N, 11.33; Cl, 7.17; F, 11.52.
Found: C, 52.79; H, 3.38; N, 11.15; Cl, 7.51; F, 11.14.

### Example 801

[(4S,6R)-6-(2-Chloro-3-methoxyphenyl)-8-cyano-1-(trifluoromethyl)-4H,6H-[1,2,4]triazolo[4,3-a][4,1]benzoxazepin-4-yl]acetic acid

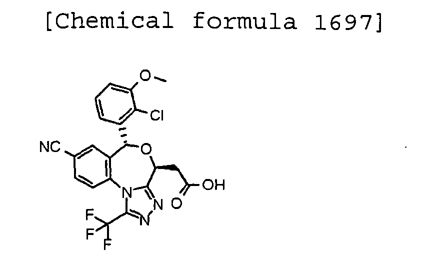

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (185 mg) was obtained from ethyl [(4S,6R)-6-(2-chloro-3-methoxyphenyl)-8-cyano-1-(trifluoromethyl)-4H,6H-[1,2,4]triazolo[4,3-a][4,1]benzoxazepin-4-yl]acetate (251 mg).
MS (ESI) m/z : 479 (M+H)⁺.
¹H-NMR (CDCl₃) δ: 3.34 (1H, dd, J = 17.2, 6.6 Hz), 3.61 (1H, dd, J = 17.2, 6.6 Hz), 3.93 (3H, s), 4.92 (1H, t, J = 6.6 Hz), 5.60 (1H, s), 7.05 (1H, dd, J = 8.2, 1.3 Hz), 7.08 (1H, d, J = 1.7 Hz), 7.33-7.37 (1H, m), 7.43 (1H, t, J = 8.1 Hz), 7.74 (1H, d, J = 8.3 Hz), 7.89 (1H, dd, J = 8.3, 1.7 Hz).
Elemental analysis for: C₂₁H₁₄ClF₃N₄O₄·0.25H₂O·0.25 hexane
Calculated: C, 53.53; H, 3.39; N, 11.10; Cl, 7.02; F, 11.28.
Found: C, 53.23; H, 3.49; N, 10.84; Cl, 7.24; F, 10.89.

### Example 802

Ethyl [(trans)-1-[chloro(difluoro)methyl]-6-(2-chloro-3-methoxyphenyl)-8-cyano-4H,6H-[1,2,4]triazolo[4,3-a] [4,1]benzoxazepin-4-yl]acetate

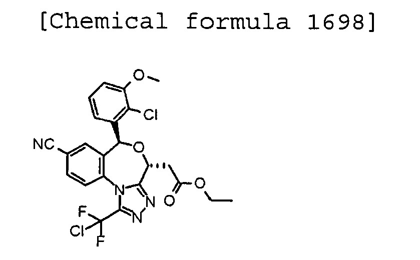

In a bath of ice water, hydrazine monohydrate (0.43 mL) was added to a solution of ethyl [(trans)-5-(2-chloro-3-methoxyphenyl)-7-cyano-2-thioxo-1,2,3,5-tetrahydro-4,1-benzoxazepin-3-yl]acetate (1.27 g) in tetrahydrofuran (20 mL) and the resulting mixture was stirred for one hour. The reaction mixture was diluted with ethyl acetate and washed with water. The organic layer was washed with saturated brine and dried over anhydrous sodium sulfate. The filtrate was concentrated to give a hydrazone derivative (1.376 g) as a crudely purified product. The resulting hydrazone derivative (467 mg) was dissolved in 1,2-dichloroethane (10 mL). To the resulting solution was added chlorodifluoroacetic anhydride (0.435 mL) at room temperature and the resulting mixture was stirred for 30 minutes. The reaction mixture was concentrated. The residue was dissolved in toluene (10 mL). To the resulting solution was added trifluoroacetic anhydride (0.435 mL) and the mixture was heated under reflux for one hour. The reaction mixture was concentrated. The residue was purified by silica gel column (ethyl acetate:hexane=1:2) to give the title compound (569 mg).

### Example 803

Ethyl [(4R,6S)-1-[chloro(difluoro)methyl]-6-(2-chloro-3-methoxyphenyl)-8-cyano-4H,6H-[1,2,4]triazolo[4,3-a] [4,1]benzoxazepin-4-yl]acetate (isomer A)

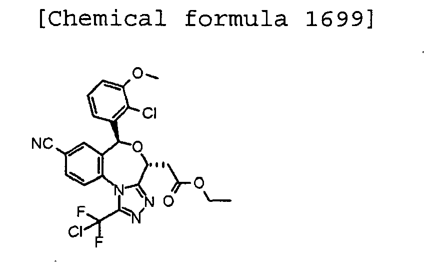

Ethyl [(4S,6R)-1-[chloro(difluoro)methyl]-6-(2-chloro-3-methoxyphenyl)-8-cyano-4H,6H-[1,2,4]triazolo[4,3-a][4,1]benzoxazepin-4-yl]acetate (isomer B) 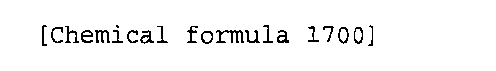 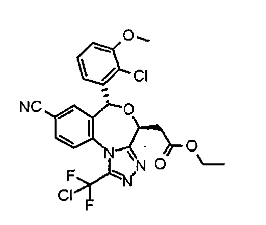 Ethyl [(trans)-1-[chloro(difluoro)methyl]-6-(2-chloro-3-methoxyphenyl)-8-cyano-4H,6H-[1,2,4]triazolo[4,3-a] [4,1]benzoxazepin-4-yl]acetate (569 mg) was optically resolved by an optically active column (CHIRALPAK-AD; 5 cm × 50 cm) into ethyl [(4R,6S)-1-[chloro(difluoro)methyl]-6-(2-chloro-3-methoxyphenyl)-8-cyano-4H,6H-[1,2,4]triazolo[4,3-a][4,1]benzoxazepin-4-yl]acetate (isomer A, 208 mg) and ethyl [(4S,6R)-1-[chloro(difluoro)methyl]-6-(2-chloro-3-methoxyphenyl)-8-cyano-4H,6H-[1,2,4]triazolo[4,3-a][4,1]benzoxazepin-4-yl]acetate (isomer B, 259 mg).
Resolution conditions:
Flow rate: 50 mL/min
Developing solvent: 30% isopropanol- n-hexane
Retention time: isomer A: 38 min., isomer B: 63 min.
Ethyl [(4R,6S)-1-[chloro(difluoro)methyl]-6-(2-chloro-3-methoxyphenyl)-8-cyano-4H,6H-[1,2,4]triazolo[4,3-a] [4,1]benzoxazepin-4-yl]acetate
MS (ESI) m/z : 523 (M+H)⁺.
¹H-NMR (CDCl₃) δ: 1.29 (3H, t, J = 7.1 Hz), 3.27 (1H, dd, J = 16.9, 6.9 Hz), 3.53 (1H, dd, J = 16.9, 6.6 Hz), 3.93 (3H, s), 4.20 (2H, q, J = 7.2 Hz), 4.92 (1H, t, J = 6.9 Hz), 5.60 (1H, s), 7.03-7.08 (2H, m), 7.33-7.36 (1H, m), 7.43 (1H, t, J = 8.1 Hz), 7.80 (1H, d, J = 8.3 Hz), 7.88 (1H, dd, J = 8.3, 2.0 Hz).
Ethyl [(4S,6R)-1-[chloro(difluoro)methyl]-6-(2-chloro-3-methoxyphenyl)-8-cyano-4H,6H-[1,2,4]triazolo[4,3-a] [4,1]benzoxazepin-4-yl]acetate
MS (ESI) m/z : 523 (M+H)⁺.
¹H-NMR (CDCl₃) δ: 1.29 (3H, t, J = 7.1 Hz), 3.27 (1H, dd, J = 16.8, 7.0 Hz), 3.53 (1H, dd, J = 16.8, 6.5 Hz), 3.93 (3H, s), 4.16-4.24 (2H, m), 4.92 (1H, t, J = 6.7 Hz), 5.60 (1H, s), 7.03-7.07 (2H, m), 7.33-7.36 (1H, m), 7.43 (1H, t, J = 8.2 Hz), 7.79 (1H, d, J = 8.3 Hz), 7.88 (1H, dd, J = 8.3, 2.0 Hz).

### Example 804

[(4R,6S)-1-[Chloro(difluoro)methyl]-6-(2-chloro-3-methoxyphenyl)-8-cyano-4H,6H-[1,2,4]triazolo[4,3-a] [4,1]benzoxazepin-4-yl]acetic acid

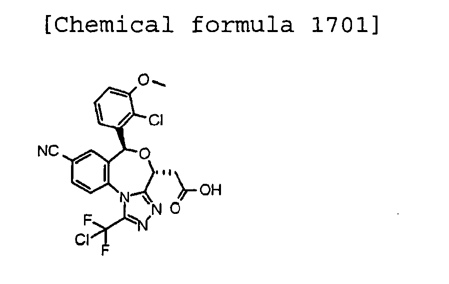

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (194 mg) was obtained from ethyl [(4R,6S)-1-[chloro(difluoro)methyl]-6-(2-chloro-3-methoxyphenyl)-8-cyano-4H,6H-[1,2,4]triazolo[4,3-a] [4,1]benzoxazepin-4-yl]acetate (261 mg).
MS (ESI) m/z : 495 (M+H)⁺.
¹H-NMR (CDCl₃) δ: 3.34 (1H, dd, J = 17.1, 6.7 Hz), 3.61 (1H, dd, J = 17.1, 6.7 Hz), 3.93 (3H, s), 4.89 (1H, t, J = 6.7 Hz), 5.61 (1H, s), 7.03-7.08 (2H, m), 7.37 (1H, d, J = 7.8 Hz), 7.43 (1H, t, J = 8.0 Hz), 7.80 (1H, d, J = 8.3 Hz), 7.89 (1H, dd, J = 8.3, 1.7 Hz).
Elemental analysis for: C₂₁H₁₄Cl₂F₂N₄O₄·0.25H₂O·0.25 hexane Calculated: C, 51.40; H, 3.45; N, 10.65; Cl, 13.48; F, 7.22.
Found: C, 51.65; H, 3.38; N, 10.63; Cl, 13.90; F, 6.86.

### Example 805

[(4S,6R)-1-[Chloro(difluoro)methyl]-6-(2-chloro-3-methoxyphenyl)-8-cyano-4H,6H-[1,2,4]triazolo[4,3-a][4,1]benzoxazepin-4-yl]acetic acid

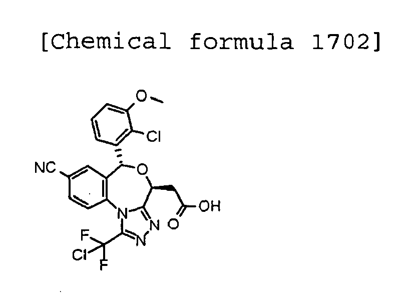

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (185 mg) was obtained from ethyl [(4S,6R)-1-[chloro(difluoro)methyl]-6-(2-chloro-3-methoxyphenyl)-8-cyano-4H,6H-[1,2,4]triazolo[4,3-a][4,1]benzoxazepin-4-yl] acetate (247 mg).
MS (ESI) m/z : 495 (M+H)⁺.
¹H-NMR (CDCl₃) δ: 3.34 (1H, dd, J = 17.2, 6.6 Hz), 3.60 (1H, dd, J = 17.2, 6.6 Hz), 3.93 (3H, s), 4.89 (1H, t, J = 6.6 Hz), 5.61 (1H, s), 7.04-7.09 (2H, m), 7.34-7.38 (1H, m), 7.44 (1H, t, J = 8.0 Hz), 7.80 (1H, d, J = 8.3 Hz), 7.89 (1H, dd, J = 8.3, 1.7 Hz).
Elemental analysis for: C₂₁H₁₄Cl₂F₂N₄O₄·0.25H₂O·0.25 hexane Calculated: C, 51.40; H, 3.45; N, 10.65; Cl, 13.48; F, 7.22.
Found: C, 51.66; H, 3.39; N, 10.72; Cl, 13.78; F, 6.93.

### Example 806

Ethyl [(trans)-6-(2-chloro-3-methoxyphenyl)-8-cyano-1-(pentafluoroethyl)-4H,6H-[1,2,4]triazolo[4,3-a] [4,1]benzoxazepin-4-yl]acetate

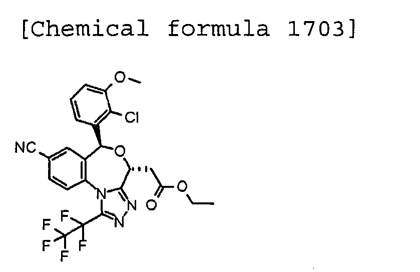

In a bath of ice water, hydrazine monohydrate (0.43 mL) was added to a solution of ethyl [(trans)-5-(2-chloro-3-methoxyphenyl)-7-cyano-2-thioxo-1,2,3,5-tetrahydro-4,1-benzoxazepin-3-yl]acetate (1.27 g) in tetrahydrofuran (20 mL) and the resulting mixture was stirred for one hour. The reaction mixture was diluted with ethyl acetate and washed with water. The organic layer was washed with saturated brine and dried over anhydrous sodium sulfate. The filtrate was concentrated to give a hydrazone derivative (1.376 g) as a crudely purified product. The resulting hydrazone derivative (451 mg) was dissolved in 1,2-dichloroethane (10 mL). To the resulting solution was added pentafluoroacetic anhydride (750 mg) at room temperature and the resulting mixture was stirred for 30 minutes. The reaction mixture was concentrated and the residue was dissolved in toluene (10 mL). To the resulting solution was added pentafluoroacetic anhydride (750 mg). The resulting mixture was heated under reflux for one hour. The reaction mixture was concentrated and the residue was purified by silica gel column (ethyl acetate:hexane=1:2) to give the title compound (497 mg).
MS (ESI) m/z : 557 (M+H)⁺.
¹H-NMR (CDCl₃) δ: 1.29 (3H, t, J = 7.1 Hz), 3.27 (1H, dd, J = 16.8, 6.8 Hz), 3.53 (1H, dd, J = 16.7, 6.6 Hz), 3.93 (3H, s), 4.20 (2H, q, J = 7.1 Hz), 4.92 (1H, t, J = 6.8 Hz), 5.59 (1H, s), 7.03-7.08 (2H, m), 7.34 (1H, d, J = 8.1 Hz), 7.43 (1H, t, J = 7.8 Hz), 7.78 (1H, dd, J = 8.2, 1.8 Hz), 7.88 (1H, dd, J = 8.3, 1.8 Hz) .

### Example 807

[(trans)-6-(2-Chloro-3-methoxyphenyl)-8-cyano-1-(pentafluoroethyl)-4H,6H-[1,2,4]triazolo[4,3-a][4,1]benzoxazepin-4-yl]acetic acid

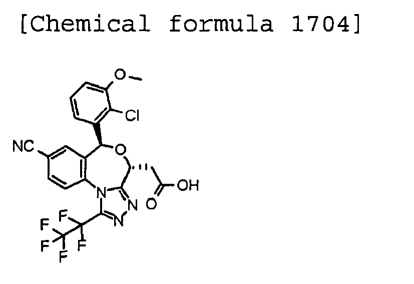

In a similar manner to that employed for the synthesis of [(4R,6S)-8-bromo-6-(2-chloro-3-methoxyphenyl)-1-(trifluoromethyl)-4H,6H-[1,2,4]triazolo[4,3-a][4,1]benzoxazepin-4-yl]acetic acid, the title compound (56 mg) was obtained from ethyl [(trans)-6-(2-chloro-3-methoxyphenyl)-8-cyano-1-(pentafluoroethyl)-4H,6H-[1,2,4]triazolo[4,3-a][4,1]benzoxazepin-4-yl]acetate (104 mg).
MS (ESI) m/z : 529 (M+H)⁺.
¹H-NMR (CDCl₃) δ: 3.34 (1H, dd, J = 17.3, 6.7 Hz), 3.61 (1H, dd, J = 17.3, 6.7 Hz), 3.94 (3H, s), 4.90 (1H, t, J = 6.7 Hz), 5.60 (1H, s), 7.06 (1H, dd, J = 8.3, 1.5 Hz), 7.08 (1H, d, J = 1.7 Hz), 7.33-7.37 (1H, m), 7.44 (1H, t, J = 8.0 Hz), 7.79 (1H, dd, J = 8.3, 1.7 Hz), 7.89 (1H, dd, J = 8.6, 1.7 Hz).
Elemental analysis for: C₂₂H₁₄ClF₅N₄O₄·0.5 isopropyl alcohol Calculated: C, 50.51; H, 3.24; N, 10.02; Cl, 6.34; F, 16.99.
Found: C, 50.48; H, 3.32; N, 9.93; C1, 6.27; F, 16.80.

### Example 808

Methyl 3-(2-{[trans-8-chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoate

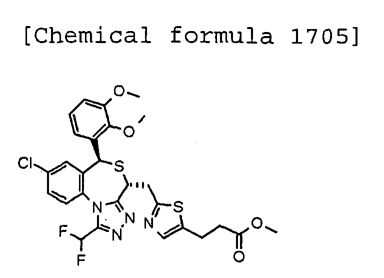

Methyl 3-(2-{[(3R,5S)-7-chloro-5-(2,3-dimethoxyphenyl)-2-[(Z)hydrazono]-1,5-dihydro-4,1-benzothiazepin-3(3H)-yl]methyl}-1,3-thiazol-5-yl)propanoate (218 mg) was dissolved in chloromethane (5 mL). To the resulting solution were added difluoroacetic anhydride (193 mg, 1.11 mmol) and difluoroacetic acid (0.50 mL). The resulting mixture was stirred at room temperature for 0.5 hour. The reaction mixture was stirred at 55°C for 1.1 hours and then, distilled to remove dichloromethane. To the residue was added toluene (5 mL) and the resulting mixture was heated under reflux for 2 hours. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:1) to give the title compound (168 mg). MS (ESI) m/z : 607 (M+H)⁺.
¹H-NMR (CDCl₃) δ: 2.65 (2H, t, J = 7.4 Hz), 3.11 (2H, t, J = 7.4 Hz), 3.40 (3H, s), 3.65 (1H, dd, J = 15.4, 7.3 Hz), 3.69 (3H, s), 3.84 (3H, br s), 4.10 (1H, dd, J = 15.4, 7.1 Hz), 4.48 (1H, dd, J = 7.3, 7.1 Hz), 5.39 (1H, s), 6.08 (1H, t, J = 53.2 Hz), 6.96-7.04 (2H, m), 7.14-7.19 (2H, m), 7.37-7.47 (3H, m).

### Example 809

Methyl 3-(2-{[(4R,6S)-8-chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoate (isomer B)
Methyl 3-(2-([(4S,6R)-8-chloro-1-(difluoromethyl)-6-(2,3-dimethoxyphenyl)-4H,6H-[1,2,4]triazolo[4,3-a][4,1]benzothiazepin-4-yl]methyl)-1,3-thiazol-5-yl)propanoate (isomer A)

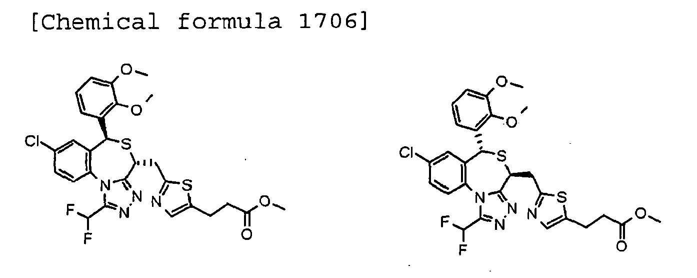

The racemic mixture (168 mg) was optically resolved by an optically active column (CHIRALCEL-OD; 2 cm × 25 cm) into the isomer A (51 mg) and the isomer B (54.9 mg).
Flow rate: 10 mL/min
Developing solvent: 15% ethanol - n-hexane
Retention time: isomer A: 40 min., isomer B: 50 min.

### Example 810

3-(2-{[(4R,6S)-8-Chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoic acid

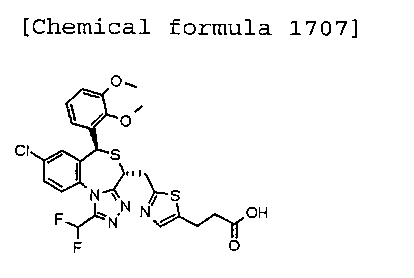

Methyl 3-(2-{[(4R,6S)-8-chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoate (54.9 mg) was dissolved in methanol-water-tetrahydrofuran (2:1:2, 5 mL). To the resulting solution was added potassium carbonate (37.5 mg). The resulting mixture was stirred at room temperature for 21 hours. The reaction mixture was neutralized with 1N hydrochloric acid, diluted with water and extracted with chloroform. The organic layer was dried over anhydrous sodium sulfate. After concentration of the filtrate, the residue thus obtained was purified by preparative TLC (methanol:chloroform=1:9) to give the title compound (38.2 mg).
MS (ESI) m/z : 593 (M+H)⁺.
¹H-NMR (CDCl₃) δ: 2.58 (2H, t, J = 7.2 Hz), 3.02 (2H, t, J = 7.2 Hz), 3.37 (3H, s), 3.57 (1H, dd, J = 15.4, 6.8 Hz), 3.83 (3H, s), 4.07 (1H, dd, J = 15.4, 7.6 Hz), 4.45 (1H, dd, J = 7.6, 6.8 Hz), 5.37 (1H, s), 6.96-7.02 (3H, m), 7.13 (1H, t, J = 8.1 Hz), 7.19 (1H, dd, J = 7.8, 1.2 Hz), 7.30 (1H, s), 7.38 (1H, dd, J = 8.5, 2.2 Hz), 7.43 (1H, dd, J = 8.5, 1.7 Hz).

### Example 811

3-(2-{[(4S,6R)-8-Chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoic acid

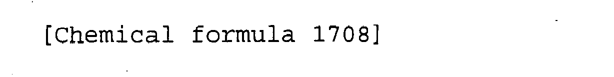

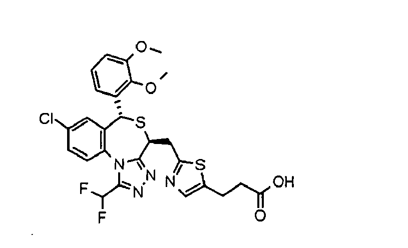

Methyl 3-(2-{[(4S,6R)-8-chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoate (51.0 mg) was dissolved in methanol-water-tetrahydrofuran (2:1:2, 5 mL). To the resulting solution was added potassium carbonate (34.8 mg). The resulting mixture was stirred at room temperature for 20 hours. The reaction mixture was neutralized with 1N hydrochloric acid, diluted with water and extracted with chloroform. The organic layer was dried over anhydrous sodium sulfate. The residue thus obtained was purified by preparative TLC (methanol:chloroform=1:9) to give the title compound (36.0 mg).
MS (ESI) m/z : 593 (M+H)⁺.
¹H-NMR coincided completely with that of 3-(2-{[(4R,6S)-8-chloro-1-(difluoromethyl)-6-(2,3-dimethoxyphenyl)-4H,6H-[1,2,4]triazolo[4,3-a][4,1]benzothiazepin-4-yl]methyl}-1,3-thiazol-5-yl)propanoic acid.

### Referential Example 722

Methyl 3-(2-{[trans-7-chloro-5-(2,3-dimethoxyphenyl)-2-[(Z)-2-(2-fluoroacetyl)hydrazono]-1,5-dihydro-4,1-benzothiazepin-3(3H)-yl]methyl}-1,3-thiazol-5-yl)propanoate

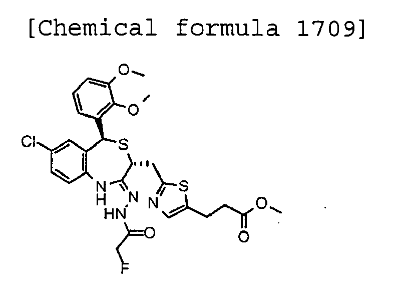

Methyl 3-(2-{[trans-7-chloro-5-(2,3-dimethoxyphenyl)-2-[(Z)hydrazono]-1,5-dihydro-4,1-benzothiazepin-3(3H)-yl]methyl}-1,3-thiazol-5-yl)propanoate (325 mg) was dissolved in tetrahydrofuran (6 mL). To the resulting solution was added 2-fluoroacetyl chloride (86.2 mg). The resulting mixture was stirred at room temperature for 3 hours. To the reaction mixture was added a saturated aqueous solution of sodium bicarbonate and the resulting mixture was extracted with ethyl acetate. The organic layer was dried over anhydrous sodium sulfate to give the title compound (361 mg).
MS (ESI) m/z : 607(M+H)⁺.

### Example 812

Methyl 3-(2-{[trans-8-chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoate

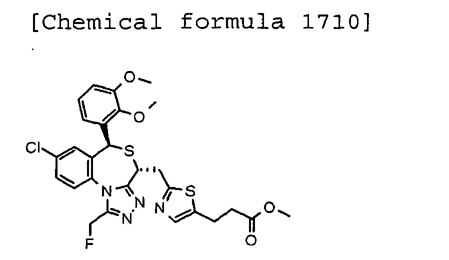

Methyl 3-(2-{[trans-7-chloro-5-(2,3-dimethoxyphenyl)-2-[(Z)-2-(2-fluoroacetyl)hydrazono]-1,5-dihydro-4,1-benzothiazepin-3(3H)-yl]methyl}-1,3-thiazol-5-yl)propanoate (361 mg) was dissolved in acetic acid (5 mL). The resulting solution was heated under reflux for 3 hours. The reaction mixture was cooled to room temperature and then concentrated. The residue was diluted with chloroform and washed with a saturated aqueous solution of sodium bicarbonate and saturated brine. The organic layer was dried over anhydrous sodium sulfate. After concentration of the filtrate, the residue thus obtained was purified by silica gel column TLC (methanol:chloroform=5:95) and preparative TLC (methanol:chloroform=5:95) to give the title compound (191 mg) as a cis isomer mixture.
MS (ESI) m/z : 589 (M+H)⁺.
¹H-NMR (CDCl₃) δ: 2.65 (2H, t, J = 7.3 Hz), 3.11 (2H, t, J = 7.3 Hz), 3.42 (3H, s), 3.67 (1H, dd, J = 15.4, 7.6 Hz), 3.69 (3H, s), 3.84 (3H, s), 4.12 (1H, dd, J = 15.6, 6.6 Hz), 4.53 (1H, t, J = 7.2 Hz), 5.35 (1H, dd, J = 48.1, 11.7 Hz), 5.35 (1H, s), 5.70 (1H, dd, J = 48.1, 11.7 Hz), 6.95-6.97 (2H, m), 7.13-7.21 (2H, m), 7.38-7.40 (3H, m).

### Example 813

Methyl 3-(2-{[(4R,6S)-8-chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoate (isomer B)

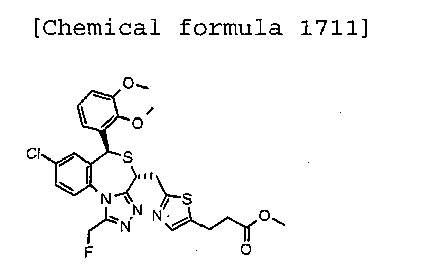

Methyl 3-(2-{[(4S,6R)-8-chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoate (isomer A)

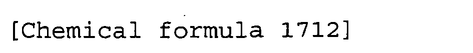

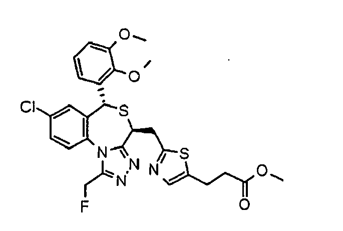

The racemic mixture (191 mg) was optically resolved by an optically active column (CHIRALCEL-OD; 2 cm × 25cm) into the isomer A (45.1 mg) and isomer B (42.0 mg).
Flow rate: 15 mL/min
Developing solvent: 25% ethanol - n-hexane
Retention time: isomer A: 51 min., isomer B: 68 min.

### Example 814

3-(2-{[(4R,6S)-8-Chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoic acid

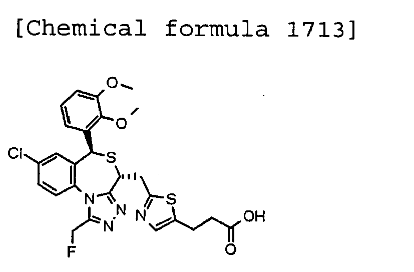

Methyl 3-(2-{[(4R,6S)-8-chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoate (42.0 mg) was dissolved in methanol-water-tetrahydrofuran (2:1:2, 5 mL). To the resulting solution was added potassium carbonate (30.0 mg). The resulting mixture was stirred at room temperature for 22 hours. The reaction mixture was neutralized with an acidic resin (Amberlite IR-120B). The resin was filtered out and the residue was washed with methanol..After concentration of the filtrate, the residue thus obtained was purified by preparative TLC (methanol:chloroform=1:9) to give the title compound (36.5 mg).
¹H-NMR (CDCl₃) δ: 2.60-2.63 (2H, m), 3.05 (2H, t, J = 7.0 Hz), 3.40 (3H, s), 3.60 (1H, dd, J = 15.4, 7.0 Hz), 3.83 (3H, s), 4.09 (1H, dd, J = 15.4, 7.5 Hz), 4.50 (1H, t, J = 7.2 Hz), 5.33 (1H, s), 5.37 (1H, dd, J = 47.6, 11.7 Hz), 5.69 (1H, dd, J = 48.2, 11.7 Hz), 6.94-6.97 (2H, m), 7.12-7.21 (2H, m), 7.35-7.39 (3H, m).

### Example 815

3-(2-{[(4S,6R)-8-Chloro-6-(2,3-dimethoxyphenyl)-1-(fluoromethyl)-4H,6H-[1,2,4]triazolo[4,3-a] [4,1]benzothiazepin-4-yl]methyl}-1,3-thiazol-5-yl)propanoic acid

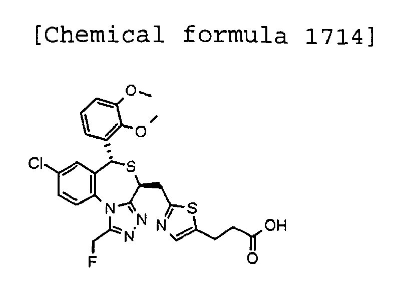

Methyl 3-(2-{[(4S,6R)-8-chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoate (45.1 mg) was dissolved in methanol-water-tetrahydrofuran (2:1:2, 5 mL). To the resulting solution was added potassium carbonate (31.7 mg). The resulting mixture was stirred at room temperature for 22 hours. The reaction mixture was neutralized with an acidic resin (Amberlite IR-120B). The resin was filtered out and the residue was washed with methanol..After concentration of the filtrate, the residue thus obtained was purified by preparative TLC (methanol:chloroform=1:9) to give the title compound (30.8 mg).
¹H-NMR coincided completely with that of 3-(2-{[(4R,6S)-8-chloro-6-(2,3-dimethoxyphenyl)-1-(fluoromethyl)-4H,6H-[1,2,4]triazolo[4,3-a][4,1]benzothiazepin-4-yl]methyl}-1,3-thiazol-5-yl)propanoic acid.

### Referential Example 723

2-(Benzyloxy)-3-methoxybenzaldehyde

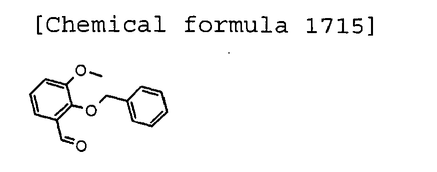

o-Vanillin (5.00 g) was dissolved in N,N-dimethylformamide (50 mL). To the resulting solution were added benzyl bromide (5.86 mL) and potassium carbonate (5.45 g). The resulting mixture was stirred at room temperature for 19 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:4) to give the title compound (7.75 g).
¹H-NMR (CDCl₃) δ: 3.95 (3H, s), 5.18 (2H, s), 7.12-7.16 (1H, m), 7.17-7.19 (1H, m), 7.34-7.40 (6H, m), 10.24 (1H, d, J = 0.7 Hz).

### Referential Example 724

N-{2-[[2-(Benzyloxy)-3-methoxyphenyl](hydroxy)methyl]-4-chlorophenyl}-2,2-dimethylpropanamide

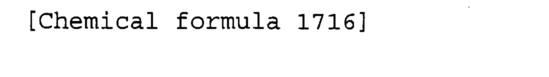

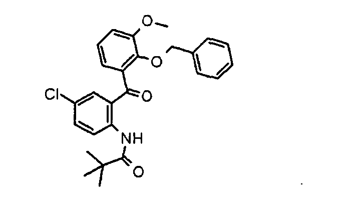

N-(4-Chlorophenyl)-2,2-dimethylpropanamide (2.75 g) was dissolved in tetrahydrofuran (50 mL). To the resulting solution was added n-butyl lithium (a hexane solution) (19.4 mL) at 0°C in a nitrogen atmosphere. The resulting mixture was stirred at room temperature for 2 hours. To the reaction mixture was added dropwise a tetrahydrofuran solution (25 mL) of 2-(benzyloxy)-3-methoxybenzaldehyde (3.00 g) at 0°C slowly. The resulting mixture was stirred at room temperature for further one hour. To the reaction mixture was added a saturated aqueous solution of ammonium chloride, followed by extraction with ethyl acetate. The extract was washed with saturated brine. The organic layer was dried over anhydrous sodium sulfate. After concentration of the filtrate, the residue thus obtained was purified by silica gel column (ethyl acetate:hexane=1:4) to give the title compound (3.70 g).
¹H-NMR (CDCl₃) δ: 1.05 (9H, s), 3.95 (3H, s), 4.01 (1H, d, J = 3.7 Hz), 4.99 (1H, d, J = 11.0 Hz), 5.18 (1H, d, J = 11.0 Hz), 5.69 (1H, d, J = 3.7 Hz), 6.44-6.46 (1H, m), 6.84 (1H, d, J = 2.7 Hz), 6.94-7.01 (2H, m), 7.28-7.32 (3H, m), 7.36-7.38 (3H, m), 8.08 (1H, d, J = 8.5 Hz), 8.96 (1H, s).

### Referential Example 725

2-[[2-(Benzyloxy)-3-methoxyphenyl](ethoxy)methyl]-4-chloroaniline

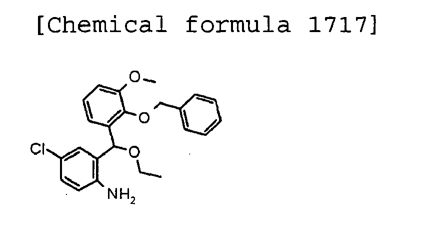

N-{2-[[2-(benzyloxy)-3-methoxyphenyl](hydroxy)methyl]-4-chlorophenyl}-2,2-dimethylpropanamide (3.70 g) was dissolved in ethanol (60 mL). To the resulting solution was added sodium hydroxide (1.83 g) and the resulting mixture was heated under reflux for 11 hours. After cooling to room temperature, the reaction mixture was diluted with water and extracted with ethyl acetate. The extract was washed with saturated brine. The organic layer was dried over anhydrous sodium sulfate. After concentration of the filtrate, the residue thus obtained was purified by silica gel column (ethyl acetate:hexane=1:4) to give the title compound (1.70 g). ¹H-NMR (CDCl₃) δ: 1.19 (3H, t, J = 7.0 Hz), 3.35-3.37 (2H, m), 3.89 (3H, s), 4.05-4.08 (2H, m), 4.99 (1H, d, J = 11.0 Hz), 5.03 (1H, d, J = 11.0 Hz), 5.69 (1H, s), 6.49 (1H, d, J = 8.3 Hz), 6.88 (1H, dd, J = 7.8, 1.7 Hz, Hz), 6.96 (1H, dd, J = 8.3, 2.4 Hz, Hz), 7.04 (1H, dd, J = 8.3, 1.7 Hz, Hz), 7.09 (1H, t, J = 7.8 Hz), 7.12-7.13 (1H, m), 7.33-7.39 (3H, m), 7.42-7.44 (2H, m).

### Referential Example 726

(2-Amino-5-chlorophenyl)(2-methoxyphenyl)methanol

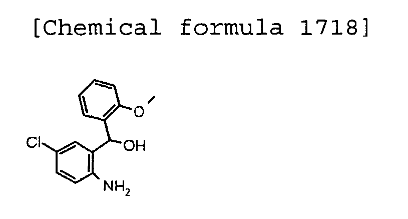

N-{4-chloro-2-[hydroxy(2-methoxyphenyl)methyl]phenyl}-2,2-dimethylpropanamide (5.00 g) was dissolved in ethanol (60 mL). To the resulting solution was added sodium hydroxide (3.22 g). The resulting mixture was heated under reflux for 3 hours. After cooling to room temperature, the reaction mixture was diluted with water and extracted with ethyl acetate. The extract was washed with saturated brine. The organic layer was dried over anhydrous sodium sulfate. After concentration of the filtrate, the residue thus obtained was purified by silica gel column (ethyl acetate:hexane=1:1) to give the title compound (1.28 g).
¹H-NMR (CDCl₃) δ: 3.89 (3H, s), 6.01 (1H, s), 6.61 (1H, d, J = 8.5 Hz), 6.95-6.98 (3H, m), 7.05 (1H, dd, J = 8.5, 2.6 Hz), 7.15 (1H, dd, J = 7.6, 1.7 Hz), 7.30-7.34 (1H, m).

### Referential Example 727

tert-Butyl 4-chloro-2-[(2-chloro-3-methoxyphenyl)(hydroxy)methyl]phenylcarbamate

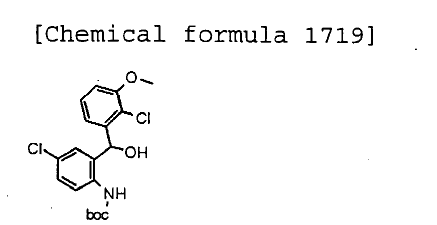

tert-Butyl 4-chlorophenylcarbamate (1.47 g) was dissolved in tetrahydrofuran (20 mL). To the resulting solution was added sec-butyl lithium (a hexane solution) (14.8 mL) at -78°C in a nitrogen atmosphere. The resulting mixture was stirred at -20°C for 2.5 hours. To the reaction mixture was added dropwise a tetrahydrofuran solution (10 mL) of 2-chloro-3-methoxybenzaldehyde (1.00 g) slowly. The resulting mixture was stirred at -20°C for further 2.5 hours. To the reaction mixture was added a saturated aqueous solution of ammonium chloride, followed by extraction with ethyl acetate. The extract was washed with saturated brine. The organic layer was dried over anhydrous sodium sulfate. After concentration of the filtrate, the residue thus obtained was purified by silica gel column (ethyl acetate:hexane=1:4) to give the title compound (2.14 g).
¹H-NMR (CDCl₃) δ: 1.50 (9H, s), 3.92 (3H, s), 6.20 (1H, d, J = 4.2 Hz), 6.90 (1H, d, J = 2.4 Hz), 6.94-6.95 (1H, m), 7.17-7.19 (1H, m), 7.24 (1H, dd, J = 8.8, 2.4 Hz), 7.31 (1H, t, J = 8.1 Hz), 7.40 (1H, s), 7.70 (1H, d, J = 8.5 Hz).

### Referential Example 728

tert-Butyl 4-chloro-2-[hydroxy(3-methoxyphenyl)methyl]phenylcarbamate

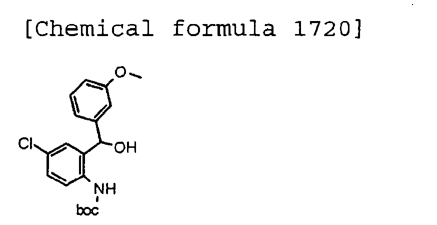

tert-Butyl 4-chlorophenylcarbamate (1.84 g) was dissolved in tetrahydrofuran (20 mL). To the resulting solution was added sec-butyl lithium (a hexane solution) (18.6 mL) at -78°C in a nitrogen atmosphere. The resulting mixture was stirred at -20°C for 2.5 hours. To the reaction mixture was added dropwise a tetrahydrofuran solution (10 mL) of 3-methoxybenzaldehyde (1.00 g) slowly. The reaction mixture was stirred at -20°C for further 2.5 hours. To the reaction mixture was added a saturated aqueous solution of ammonium chloride, followed by extraction with ethyl acetate. The extract was washed with saturated brine. The organic layer was dried over anhydrous sodium sulfate. After concentration of the filtrate, the residue thus obtained was purified by silica gel column (ethyl acetate:hexane=1:4) to give the title compound (2.18 g).
¹H-NMR (CDCl₃) δ: 1.45 (9H, s), 3.81 (3H, d, J = 5.1 Hz), 5.85 (1H, d, J = 3.2 Hz), 6.85 (1H, dd, J = 8.3, 2.4 Hz), 6.88-6.90 (1H, m), 6.92-6.95 (1H, m), 7.04-7.04 (1H, m), 7.24-7.26 (1H, m), 7.29 (1H, d, J = 7.8 Hz), 7.52 (1H, s), 7.79 (1H, d, J = 8.5 Hz).

### Referential Example 729

tert-Butyl 4-chloro-2-[hydroxy(1-naphthyl)methyl]phenylcarbamate

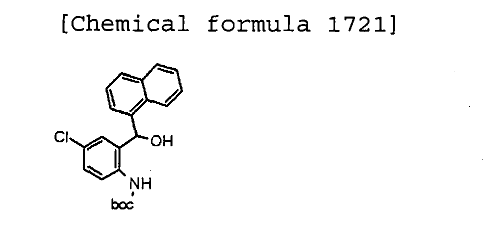

tert-Butyl 4-chlorophenylcarbamate (1.60 g) was dissolved in tetrahydrofuran (20 mL). To the resulting solution was added sec-butyl lithium (a hexane solution) (16.2 mL) at -78°C in a nitrogen atmosphere. The resulting mixture was stirred at -20°C for 2.5 hours. To the reaction mixture was adde dropwise a tetrahydrofuran solution (10 mL) of 1-naphthaldehyde (1.00 g) slowly. After stirring at -20°C for further 2.5 hours, a saturated aqueous solution of ammonium chloride was added to the reaction mixture, followed by extraction with ethyl acetate. The extract was washed with saturated brine. The organic layer was dried over anhydrous sodium sulfate. After concentration of the filtrate, the residue thus obtained was purified by silica gel column (ethyl acetate:hexane=1:4) to give the title compound (2.46 g).
¹H-NMR (CDCl₃) δ: 1.51 (9H, s), 6.58 (1H, d, J = 3.9 Hz), 6.82 (1H, d, J = 2.4 Hz), 7.24 (1H, dd, J = 8.8, 2.4 Hz), 7.45-7.55 (3H, m), 7.66 (1H, d, J = 7.1 Hz), 7.80-7.82 (2H, m), 7.87-7.92 (2H, m).

### Referential Example 730

(2-Amino-5-chlorophenyl)(2-chloro-3-methoxyphenyl)methanol

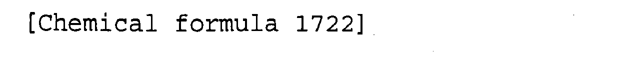

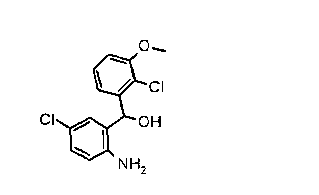

tert-Butyl 4-chloro-2-[(2-chloro-3-methoxyphenyl)(hydroxy)methyl]phenylcarbamate (2.14 g) was dissolved in 1,4-dioxane (40 mL). To the resulting solution was added concentrated hydrochloric acid (20 mL). The resulting mixture was stirred at room temperature for 7 hours. The reaction mixture was neutralized with a 5N aqueous sodium hydroxide solution and extracted with ethyl acetate. The organic layer was dried over anhydrous sodium sulfate. The filtrate was then concentrated to give the title compound (1.95 g).
MS (ESI) m/z : 280 (M-OH⁺).
¹H-NMR (CDCl₃) δ: 3.93 (3H, s), 6.20 (1H, s), 6.64 (1H, d, J = 8.5 Hz), 6.90 (1H, d, J = 2.2 Hz), 6.95 (1H, dd, J = 8.3, 1.5 Hz), 7.05-7.10 (2H, m), 7.29 (1H, t, J = 8.1 Hz).

### Referential Example 731

(2-Amino-5-chlorophenyl)(3-methoxyphenyl)methanol

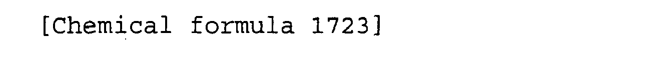

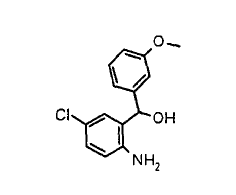

tert-Butyl 4-chloro-2-[hydroxy(3-methoxyphenyl)methyl]phenylcarbamate (2.19 g) was dissolved in 1,4-dioxane (40 mL). To the resulting solution was added concentrated hydrochloric acid (20 mL). The resulting mixture was stirred at room temperature for 13 hours. The reaction mixture was neutralized with a 5N aqueous sodium hydroxide solution and extracted with ethyl acetate. The organic layer was dried over anhydrous sodium sulfate. The filtrate was then concentrated to give the title compound (1.42 g).
MS (ESI) m/z : 246 (M-OH⁺).
¹H-NMR (CDCl₃) δ: 2.46 (1H, d, J = 4.4 Hz), 3.80 (3H, s), 5.77 (1H, d, J = 3.9 Hz), 6.59-6.61 (1H, m), 6.86-6.87 (1H, m), 6.94-6.96 (2H, m), 7.07-7.08 (2H, m), 7.28-7.30 (1H, m).

### Referential Example 732

(2-Amino-5-chlorophenyl)(1-naphthyl)methanol

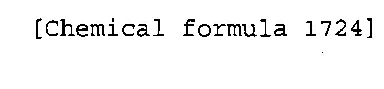

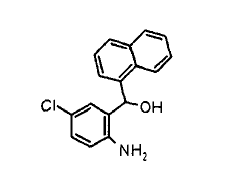

tert-Butyl 4-chloro-2-[hydroxy(1-naphthyl)methyl]phenylcarbamate (1.49 g) was dissolved in 1,4-dioxane (30 mL). To the resulting solution was added concentrated hydrochloric acid (15 mL). The resulting mixture was stirred at room temperature for 13 hours. The reaction mixture was neutralized with a 5N aqueous sodium hydroxide solution and extracted with ethyl acetate. The organic layer was dried over anhydrous sodium sulfate. The filtrate was then concentrated to give the title compound (1.24 g).
MS (ESI) m/z : 266 (M-OH⁺).
¹H-NMR (CDCl₃) δ: 6.54 (1H, s), 6.68 (1H, d, J = 8.5 Hz), 6.90 (1H, d, J = 2.4 Hz), 7.08 (1H, dd, J = 8.5, 2.4 Hz), 7.47-7.55 (4H, m), 7.87-7.90 (2H, m), 7.98-8.00 (1H, m).

### Referential Example 733

(3-Methoxy-2-methylphenyl)(4-morpholinyl)methanone

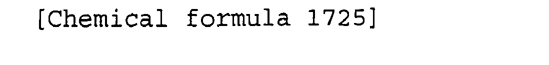

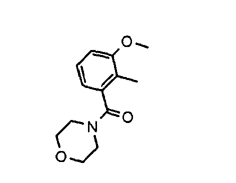

3-Methoxy-2-methylbenzoic acid (1.50 g) and morpholine (0.94 mL) were dissolved in dichloromethane (30 mL). To the resulting solution were added 1-ethyl-3-(3-dimethylaminopropyl)carbodiimide hydrochloride (2.08 g) and 1-hydroxybenzotriazole (0.41 g). The resulting mixture was stirred at room temperature for 23 hours. The reaction mixture was diluted with chloroform and washed with saturated brine. The organic layer was dried over anhydrous sodium sulfate. After concentration of the filtrate, the residue thus obtained was purified by silica gel column (methanol:chloroform=1:9) to give the title compound (2.40 g).
¹H-NMR (CDCl₃) δ: 2.17 (3H, s), 3.22-3.23 (2H, m), 3.55-3.58 (2H, m), 3.76-3.77 (2H, m), 3.82-3.82 (2H, m), 3.84 (3H, s), 6.76-6.78 (1H, m), 6.84 (1H, d, J = 8.0 Hz), 7.18-7.22 (1H, m).

### Referential Example 734

N-[4-Chloro-2-(3-methoxy-2-methylbenzoyl)phenyl]-2,2-dimethylpropanamide

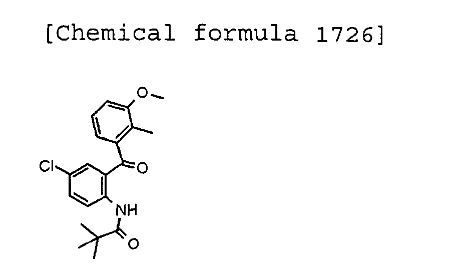

N-(4-Chlorophenyl)-2,2-dimethylpropanamide (2.26 g) was dissolved in tetrahydrofuran (60 mL). To the resulting solution was added n-butyl lithium (a hexane solution) (15.9 mL) at 0°C in a nitrogen atmosphere. The resulting mixture was stirred at room temperature for 2 hours. A tetrahydrofuran solution (40 mL) of (3-methoxy-2-methylphenyl)(4-morpholinyl)methanone (2.40 g) was added to the reaction mixture in portions at 0°C. After stirring at room temperature for further three hours, a saturated aqueous solution of ammonium chloride was added to the reaction mixture, followed by extraction with ethyl acetate. The extract was washed with saturated brine. The organic layer was dried over anhydrous sodium sulfate. After concentration of the filtrate, the residue thus obtained was purified by silica gel column (ethyl acetate:hexane=1:6) to give the title compound (0.49 g).
MS (ESI) m/z : 360 (M+H⁺).
¹H-NMR (CDCl₃) δ: 1.38 (9H, s), 2.12 (3H, s), 3.90 (3H, s), 6.81-6.83 (1H, m), 6.99 (1H, d, J = 8.0 Hz), 7.24-7.28 (1H, m), 7.37 (1H, d, J = 2.7 Hz), 7.49-7.52 (1H, m), 8.82 (1H, d, J = 9.0 Hz), 11.77 (1H, s).

### Referential Example 735

(2-Amino-5-chlorophenyl)(3-methoxy-2-methylphenyl)methanone

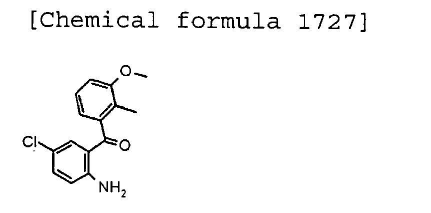

N-[4-Chloro-2-(3-methoxy-2-methylbenzoyl)phenyl]-2,2-dimethylpropanamide (490 mg) was dissolved in methanol (10 mL). To the resulting solution were added water (1.5 mL) and potassium hydroxide (305 mg) and the resulting mixture was heated under reflux for 139 hours. After cooling to room temperature, the reaction mixture was diluted with water and extracted with ethyl acetate. The extract was washed with saturated brine. The organic layer was dried over anhydrous sodium sulfate. The filtrate was then concentrated to give the title compound (388 mg).
MS (ESI) m/z : 276 (M+H⁺).
¹H-NMR (CDCl₃) δ: 2.09 (3H, s), 3.88 (3H, s), 6.42 (2H, s), 6.66 (1H, d, J = 8.8 Hz), 6.81 (1H, d, J = 7.6 Hz), 6.93 (1H, d, J = 8.1 Hz), 7.17-7.23 (3H, m).

### Referential Example 736

(2-Amino-5-chlorophenyl)(3-methoxy-2-methylphenyl)methanol

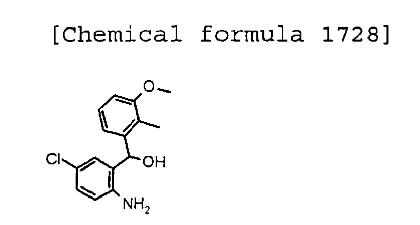

(2-Amino-5-chlorophenyl)(3-methoxy-2-methylphenyl)methanone (388 mg) was dissolved in methanol (10 mL). To the resulting solution was added sodium borohydride (266 mg). The resulting mixture was stirred at room temperature for one hour. Water was added and the resulting mixture was extracted with ethyl acetate. The extract was washed with saturated brine. The organic layer was dried over anhydrous sodium sulfate. The filtrate was then concentrated to give the title compound (395 mg).
MS (ESI) m/z : 260 (M-OH⁺).
¹H-NMR (CDCl₃) δ: 2.12 (3H, s), 3.86 (3H, s), 6.00 (1H, s), 6.63 (1H, d, J = 8.3 Hz), 6.81 (1H, d, J = 2.4 Hz), 6.87 (1H, d, J = 8.0 Hz), 7.02 (1H, d, J = 7.6 Hz), 7.06 (1H, dd, J = 8.3, 2.4 Hz), 7.23 (1H, dd, J = 8.3, 8.0 Hz).

### Referential Example 737

(2-Amino-5-chlorophenyl)(2-chlorophenyl)methanol

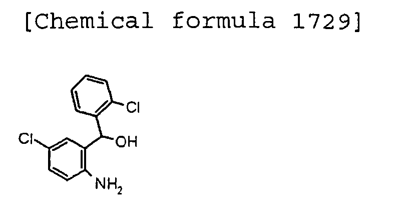

(2-amino-5-chlorophenyl)(2-chlorophenyl)methanone (1.00 g) was dissolved in methanol (20 mL). To the resulting solution was added sodium borohydride (780 mg). The resulting mixture was stirred at room temperature for 3 hours. To the reaction mixture was added water, followed by extraction with ethyl acetate. The extract was washed with saturated brine. The organic layer was dried over anhydrous sodium sulfate. The filtrate was then concentrated to give the title compound (1.11 g).
MS (ESI) m/z : 214 (M-OH⁺).
¹H-NMR (CDCl₃) δ: 2.53 (1H, s), 4.05-4.09 (2H, br m), 6.17-6.18 (1H, m), 6.64 (1H, d, J = 8.4 Hz), 6.90 (1H, d, J = 2.2 Hz), 7.08 (1H, dd, J = 8.4, 2.6 Hz), 7.28-7.34 (2H, m), 7.40-7.43 (1H, m), 7.47-7.49 (1H, m).

### Referential Example 738

2-[(trans)-7-Chloro-5-(2-chloro-3-methoxyphenyl)-2-oxo-1,2,3,5-tetrahydro-4,1-benzothiazepin-3-yl]acetic acid

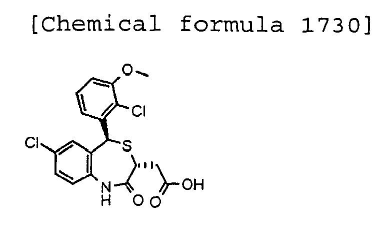

(2-Amino-5-chlorophenyl)(2-chloro-3-methoxyphenyl)methanol (1.62 g) and thiomalic acid (0.82 g) was dissolved in a solvent mixture of concentrated hydrochloric acid (7 mL) and acetic acid (15 mL). The resulting solution was stirred at 70°C for 6 hours. The reaction mixture was cooled, neutralized with a 5N aqueous sodium hydroxide solution, and extracted with dichloromethane containing 10% tetrahydrofuran. The extract was washed with saturated brine. The organic layer was dried over anhydrous magnesium sulfate. The filtrate was then concentrated. The residue was dissolved in toluene (40 mL). The resulting solution was heated under reflux for 16 hours. After cooling to room temperature, the reaction mixture was concentrated. The residue thus obtained was dissolved in methanol-water-tetrahydrofuran (2:1:2, 50 mL). To the resulting solution was added lithium hydroxide monohydrate (0.40 g). The resulting mixture was stirred at 50°C for 23 hours. The reaction mixture was made acidic with 1N hydrochloric acid and then, extracted with ethyl acetate. The extract was washed with water. The organic layer was dried over anhydrous sodium sulfate. The filtrate was then concentrated. The solid thus precipitated was collected by filtration using ethanol to give the title compound (1.08 g).
MS (ESI) (ESI) m/z : 412 (M+H)⁺.
¹H-NMR (DMSO-d₆) δ: 2.43 (1H, dd, J = 16.6, 3.8 Hz), 2.78 (1H, dd, J = 16.6, 10.2 Hz), 3.60 (1H, dd, J = 10.2, 3.8 Hz), 3.89 (3H, s), 6.01 (1H, s), 6.52 (1H, d, J = 2.4 Hz), 7.18 (1H, d, J = 8.3 Hz), 7.27-7.28 (1H, m), 7.39 (1H, d, J = 7.8 Hz), 7.44 (1H, dd, J = 8.3, 2.4 Hz), 7.52 (1H, t, J = 8.2 Hz).

### Referential Example 739

Isopropyl 2-[(trans)-7-chloro-5-(2-chloro-3-methoxyphenyl)-2-oxo-1,2,3,5-tetrahydro-4,1-benzothiazepin-3-yl]acetate

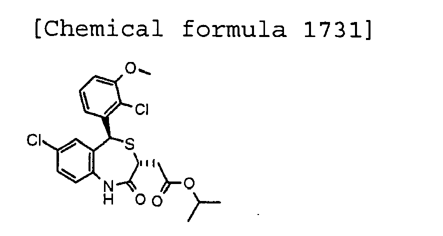

2-[(trans)-7-Chloro-5-(2-chloro-3-methoxyphenyl)-2-oxo-1,2,3,5-tetrahydro-4,1-benzothiazepin-3-yl]acetic acid (1.08 g) was dissolved in N,N-dimethylformamide (20 mL). To the resulting solution were added 2-iodopropane (0.29 mL) and potassium carbonate (0.43 g). The resulting mixture was stirred at room temperature for 170 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:1) to give the title compound (886 mg).
MS (ESI) m/z : 454 (M+H⁺).
¹H-NMR (CDCl₃) δ: 1.21. (3H, d, J = 6.1 Hz), 1.23 (3H, d, J = 6.1 Hz), 2.42 (1H, dd, J = 17.1, 3.9 Hz), 3.10 (1H, dd, J = 17.1, 10.5 Hz), 3.81 (1H, dd, J = 10.5,3.9 Hz), 3.92 (3H, s), 4.97-4.98 (1H, m), 6.23 (1H, s), 6.67 (1H, d, J = 2.2 Hz), 7.01 (1H, dd, J = 7.8, 2.2 Hz), 7.11 (1H, d, J = 8.5 Hz), 7.27-7.28 (1H, m), 7.37-7.41 (2H, m).

### Referential Example 740

Isopropyl 2-[(trans)-7-chloro-5-(2-chloro-3-methoxyphenyl)-2-thioxo-1,2,3,5-tetrahydro-4,1-benzothiazepin-3-yl]acetate

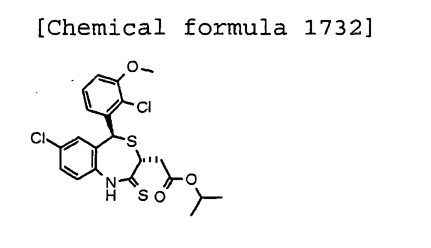

Isopropyl 2-[(trans)-7-chloro-5-(2-chloro-3-methoxyphenyl)-2-oxo-1,2,3,5-tetrahydro-4,1-benzothiazepin-3-yl]acetate (886 mg) was dissolved in toluene (20 mL). To the resulting solution was added Lawesson's reagent (867 mg). The resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (889 mg).
MS (ESI) m/z : 470 (M+H⁺).
¹H-NMR (CDCl₃) δ: 1.21 (3H, d, J = 4.1 Hz), 1.22 (3H, d, J = 4.1 Hz), 2.60 (1H, dd, J = 17.0, 3.9 Hz), 3.39 (1H, dd, J = 17.0, 10.1 Hz), 3.92 (3H, s), 4.05 (1H, dd, J = 10.2, 3.9 Hz), 4.94-5.00 (1H, m), 6.05 (1H, s), 6.65 (1H, d, J = 2.4 Hz), 7.00-7.01 (1H, m), 7.14 (1H, d, J = 8.3 Hz), 7.30 (1H, dd, J = 8.3, 2.3 Hz), 7.35-7.39 (2H, m), 9.53 (1H, s).

### Example 816

Isopropyl 2-[(trans)-8-chloro-6-(2-chloro-3-methoxyphenyl)-1-(trifluoromethyl)-4H,6H-[1,2,4]triazolo[4,3-a][4,1]benzothiazepin-4-yl]acetate

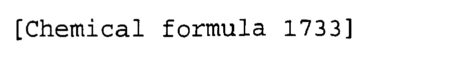

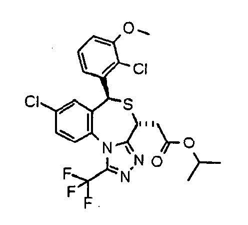

Isopropyl 2-[(trans)-7-chloro-5-(2-chloro-3-methoxyphenyl)-2-thioxo-1,2,3,5-tetrahydro-4,1-benzothiazepin-3-yl]acetate (889 mg) was dissolved in tetrahydrofuran (20 mL). To the resulting solution was added hydrazine monohydrate (0.183 mL). The resulting mixture was stirred at room temperature for 2 hours. To the reaction mixture was added 1,2-dichloroethane (20 mL), followed by the addition of trifluoroacetic anhydride (1.33 mL) and trifluoroacetic acid (2.91 mL). The resulting mixture was stirred at room temperature for one hour. After stirring at 55°C for 2 hours, toluene (20 mL) was added. The reaction mixture was heated under reflux for further one hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) to give the title compound (688 mg).
MS (ESI) (ESI) m/z : 546 (M+H)⁺.
¹H-NMR (CDCl₃) δ: 1.22 (3H, d, J = 6.3 Hz), 1.24 (3H, d, J = 6.3 Hz), 2.90 (1H, dd, J = 17.3, 5.4 Hz), 3.61 (1H, dd, J = 17.3, 9.0 Hz), 3.90 (3H, s), 4.32 (1H, dd, J = 9.0, 5.4 Hz), 4.98-5.00 (1H, m), 5.35 (1H, s), 6.84 (1H, d, J = 2.0 Hz), 7.01 (1H, dd, J = 8.1, 1.5 Hz), 7.33 (1H, dd, J = 7.8, 1.5 Hz), 7.37-7.40 (2H, m), 7.46 (1H, dd, J = 8.4, 2.3 Hz).

### Example 817

2-[(trans)-8-Chloro-6-(2-chloro-3-methoxyphenyl)-1-(trifluoromethyl)-4H,6H-[1,2,4]triazolo[4,3-a][4,1]benzothiazepin-4-yl]aceticacid

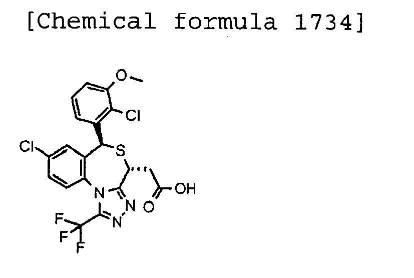

Isopropyl 2-[(trans)-8-chloro-6-(2-chloro-3-methoxyphenyl)-1-(trifluoromethyl)-4H,6H-[1,2,4]triazolo[4,3-a][4,1]benzothiazepin-4-yl]acetate (200 mg) was dissolved in 1,4-dioxane (5 mL). To the resulting solution was added concentrated hydrochloric acid (1.1 mL). The resulting mixture was stirred at 60°C for 27 hours. After cooling to room temperature, the reaction mixture was diluted with ethyl acetate and washed with water. The organic layer was dried over anhydrous sodium sulfate. The filtrate was then concentrated. The residue thus obtained was purified by preparative TLC (methanol:chloroform=1:9) to give the title compound (75.2 mg).
MS (ESI) m/z : 504 (M+H)⁺.
¹H-NMR (CDCl₃) δ: 2.83-2.86 (1H, m), 3.41-3.44 (1H, m), 3.89 (3H, s), 4.28-4.31 (1H, m), 5.31 (1H, s), 6.79 (1H, s), 6.99 (1H, d, J = 7.3 Hz), 7.32-7.38 (4H, m).

### Referential Example 741

2-[(trans)-7-Chloro-5-(3-methoxyphenyl)-2-oxo-1,2,3,5-tetrahydro-4,1-benzothiazepin-3-yl]acetic acid

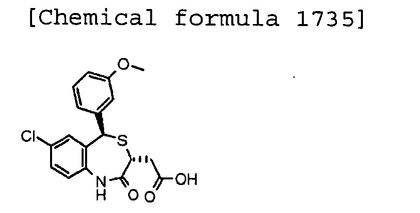

(2-Amino-5-chlorophenyl)(3-methoxyphenyl)methanol (1.42 g) and thiomalic acid (0.81 g) were dissolved in a solvent mixture of concentrated hydrochloric acid (7 mL) and acetic acid (15 mL). The resulting solution was stirred at 70°C for 2.2 hours. The reaction mixture was cooled, neutralized with a 5N aqueous sodium hydroxide solution, and extracted with dichloromethane containing 10% tetrahydrofuran. The extract was washed with saturated brine. The organic layer was dried over anhydrous magnesium sulfate. The filtrate was then concentrated.
The residue was dissolved in toluene (30 mL). The resulting solution was heated under reflux for 34 hours. After cooling to room temperature, the reaction mixture was concentrated. The residue thus obtained was dissolved in methanol-water-tetrahydrofuran (2:1:2, 50 mL). To the resulting solution was added lithium hydroxide monohydrate (0.45 g) and the resulting mixture was stirred at 50°C for 23 hours. The reaction mixture was made acidic with 1N hydrochloric acid and extracted with ethyl acetate. The extract was washed with water. The organic layer was dried over anhydrous sodium sulfate. The filtrate was then concentrated to give the title compound (2.04 g).
MS (ESI) m/z : 378 (M+H)⁺.
¹H-NMR (DMSO-d₆) δ: 2.66-2.68 (1H, m), 3.65-3.67 (1H, m), 3.75-3.77 (4H, m), 5.65 (1H, s), 6.73 (1H, s), 6.99-7.01 (1H, m), 7.09-7.14 (3H, m), 7.39-7.41 (2H, m), 9.83 (1H, s) .

### Referential Example 742

Isopropyl 2-[(trans)-7-chloro-5-(3-methoxyphenyl)-2-oxo-1,2,3,5-tetrahydro-4,1-benzothiazepin-3-yl]acetate

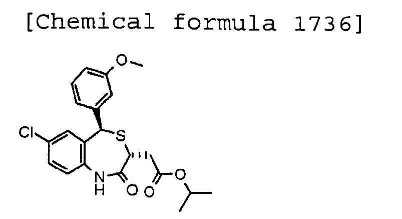

2-[(trans)-7-Chloro-5-(3-methoxyphenyl)-2-oxo-1,2,3,5-tetrahydro-4,1-benzothiazepin-3-yl]acetic acid (2.04 g) was dissolved in N,N-dimethylformamide (20 mL).
To the resulting solution were added 2-iodopropane (0.592 mL) and potassium carbonate (0.894 g). The resulting mixture was stirred at 30°C for 18 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:1) to give the title compound (519 mg).
MS (ESI) m/z : 420 (M+H⁺).
¹H-NMR (CDCl₃) δ: 1.18 (3H, d, J = 5.9 Hz), 1.20 (3H, d, J = 6.1 Hz), 2.40 (1H, dd, J = 17.1, 3.8 Hz), 3.05 (1H, dd, J = 17.1, 10.5 Hz), 3.83 (3H, d, J = 1.7 Hz), 3.85 (1H, dd, J = 10.5, 3.8 Hz), 4.89-4.95 (1H, m), 5.73 (1H, s), 6.90 (1H, d, J = 2.2 Hz), 6.92-6.94 (1H, m), 7.01-7.02 (1H, m), 7.06 (1H, d, J = 7.8 Hz), 7.11 (1H, d, J = 8.3 Hz), 7.25 (1H, dd, J = 8.5, 2.2 Hz), 7.34 (1H, t, J = 7.9 Hz), 8.26 (1H, s).

### Referential Example 743

Isopropyl 2-[(trans)-7-chloro-5-(3-methoxyphenyl)-2-thioxo-1,2,3,5-tetrahydro-4,1-benzothiazepin-3-yl]acetate

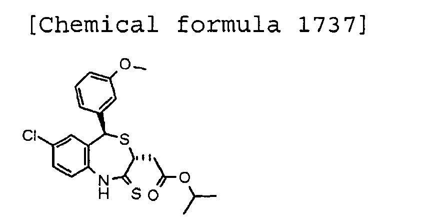

Isopropyl 2-[(trans)-7-chloro-5-(3-methoxyphenyl)-2-oxo-1,2,3,5-tetrahydro-4,1-benzothiazepin-3-yl]acetate (519 mg) was dissolved in toluene (15 mL). To the resulting solution was added Lawesson's reagent (550 mg). The resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (381 mg).
¹H-NMR (CDCl₃) δ: 1.20-1.22 (6H, m), 2.57 (1H, dd, J = 17.1, 3.9 Hz), 3.36 (1H, dd, J = 17.1, 10.3 Hz), 3.83 (3H, s), 4.08 (1H, dd, J = 10.3, 3.9 Hz), 4.95-4.97 (1H, m), 5.57 (1H, s), 6.90 (1H, d, J = 2.4 Hz), 6.92-6.94 (1H, m), 6.98-6.98 (1H, m), 7.02-7.04 (1H, m), 7.12 (1H, d, J = 8.5 Hz), 7.30 (1H, dd, J = 8.5, 2.3 Hz), 7.34 (1H, t, J = 7.9 Hz), 9.30 (1H, s).

### Example 818

Isopropyl 2-[(trans)-8-chloro-6-(3-methoxyphenyl)-1-(trifluoromethyl)-4H,6H-[1,2,4]triazolo[4,3-a] [4, 1] benzothiazepin-4-yl] acetate

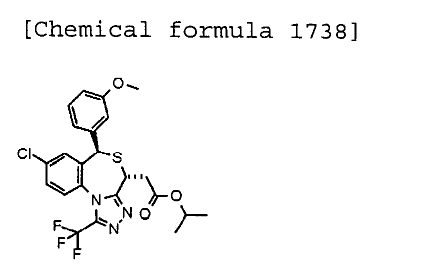

Isopropyl 2-[(trans)-7-chloro-5-(3-methoxyphenyl)-2-thioxo-1,2,3,5-tetrahydro-4,1-benzothiazepin-3-yl]acetate (381 mg) was dissolved in tetrahydrofuran (10 mL). To the resulting solution was added hydrazine monohydrate (0.085 mL) and the resulting mixture was stirred at room temperature for 2 hours. To the reaction mixture was added 1,2-dichloroethane (7 mL), followed by the addition of trifluoroacetic anhydride (0.454 mL) and trifluoroacetic acid (0.991 mL). The resulting mixture was stirred at room temperature for one hour. After stirring the reaction mixture at 55°C for 2 hours, toluene (7 mL) was added and the mixture was heated under reflux for further one hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) and preparative TLC (ethyl acetate:hexane=1:4) to give the title compound (94 mg).
MS (ESI) m/z : 512 (M+H)⁺.
¹H-NMR (CDCl₃) δ: 1.21 (3H, d, J = 5.9 Hz), 1.24 (3H, d, J = 5.4 Hz), 2.85 (1H, dd, J = 17.2, 5.4 Hz), 3.57 (1H, dd, J = 17.2, 9.0 Hz), 3.82 (3H, s), 4.34 (1H, dd, J = 9.0, 5.4 Hz), 4.89 (1H, s), 4.97-4.99 (1H, m), 6.87-6.88 (1H, m), 6.93-6.95 (2H, m), 7.10 (1H, d, J = 2.4 Hz), 7.33 (1H, d, J = 8.1 Hz), 7.36-7.39 (1H, m), 7.45 (1H, dd, J = 8.4, 2.3 Hz).

### Example 819

2-[(trans)-8-Chloro-6-(3-methoxyphenyl)-1-(trifluoromethyl)-4H,6H-[1,2,4]triazolo[4,3-a] [4,1]benzothiazepin-4-yl]acetic acid

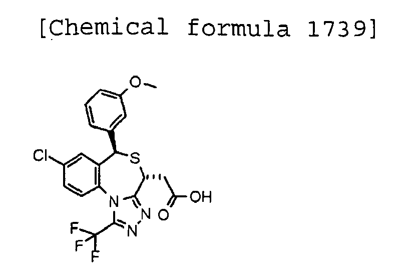

Isopropyl 2-[(trans)-8-chloro-6-(3-methoxyphenyl)-1-(trifluoromethyl)-4H,6H-[1,2,4]triazolo[4,3-a][4,1]benzothiazepin-4-yl]acetate (200 mg) was dissolved in 1,4-dioxane (2.5 mL). To the resulting solution was added concentrated hydrochloric acid (0.55 mL). The resulting mixture was stirred at 60°C for 41 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (51.9 mg).
MS (ESI) m/z : 470 (M+H)⁺.
¹H-NMR (CDCl₃) δ: 2.69 (1H, dd, J = 16.5, 4.6 Hz), 3.29 (1H, dd, J = 16.5, 10.3 Hz), 3.78 (3H, s), 4.29 (1H, dd, J = 10.3, 4.6 Hz), 4.87 (1H, s), 6.84 (1H, s), 6.9.0-6.93 (2H, m), 7.01 (1H, d, J = 2.0 Hz), 7.32-7.36 (3H, m).

### Referential Example 744

2-[(trans)-7-Chloro-5-(1-naphthyl)-2-oxo-1,2,3,5-tetrahydro-4,1-benzothiazepin-3-yl]acetic acid

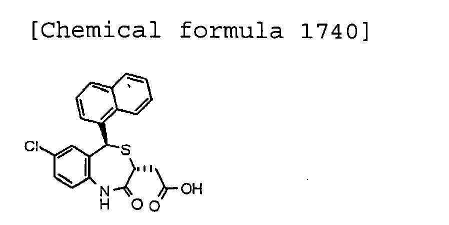

(2-Amino-5-chlorophenyl)(1-naphthyl)methanol (1.24 g) and thiomalic acid (0.66 g) were dissolved in a solvent mixture of concentrated hydrochloric acid (7 mL) and acetic acid (15 mL). The resulting solution was stirred at 70°C for 3 hours. After cooling, the reaction mixture was neutralized with a 5N aqueous sodium hydroxide solution and extracted with dichloromethane containing 10% tetrahydrofuran. The extract was washed with saturated brine. The organic layer was dried over anhydrous magnesium sulfate. The filtrate was then concentrated.
The residue was dissolved in toluene (30 mL) and the resulting solution was heated under reflux for 34 hours. The reaction mixture was cooled to room temperature and then concentrated. The residue thus obtained was dissolved in methanol-water-tetrahydrofuran (2:1:2, 50 mL). To the resulting solution was added lithium hydroxide monohydrate (0.360 g) and the resulting mixture was stirred at 50°C for 23 hours. The reaction mixture was made acidic with 1N hydrochloric acid and then extracted with ethyl acetate. The extract was washed with water. The organic layer was dried over anhydrous sodium sulfate. The filtrate was then concentrated. The solid thus precipitated was collected by filtration with ethanol to give the title compound (1.08 g).
MS (ESI) m/z : 398 (M+H)⁺.
¹H-NMR (DMSO-d₆) δ: 2.49 (1H, dd, J = 16.8, 3.9 Hz), 2.84 (1H, dd, J = 16.8, 10.3 Hz), 3.66 (1H, dd, J = 10.3, 3.9 Hz), 6.40 (1H, s), 6.45 (1H, d, J = 2.2 Hz), 7.26 (1H, d, J = 8.5 Hz), 7.41 (1H, dd, J = 8.3, 2.4 Hz), 7.49-7.57 (3H, m), 7.67 (1H, t, J = 7.8 Hz), 7.88 (1H, d, J = 7.1 Hz), 8.03-8.04 (2H, m).

### Referential Example 745

Isopropyl 2-[(trans)-7-chloro-5-(1-naphthyl)-2-oxo-1,2,3,5-tetrahydro-4,1-benzothiazepin-3-yl]acetate

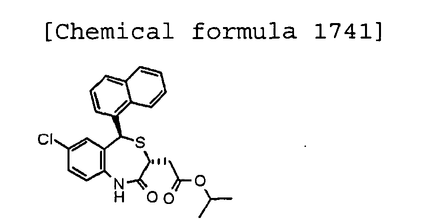

2-[(trans)-7-Chloro-5-(1-naphthyl)-2-oxo-1,2,3,5-tetrahydro-4,1-benzothiazepin-3-yl]acetic acid (1.69 g) was dissolved in N,N-dimethylformamide (20 mL). To the resulting solution were added 2-iodopropane (0.467 mL) and potassium carbonate (0.705 g). The resulting mixture was stirred at 30°C for 18 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:1) to give the title compound (749 mg).
MS (ESI) m/z : 440 (M+H⁺).
¹H-NMR (CDCl₃) δ: 1.22 (3H, d, J = 6.6 Hz), 1.24 (3H, d, J = 6.1 Hz), 2.49 (1H, dd, J = 17.1, 3.8 Hz), 3.14 (1H, dd, J = 17.1, 10.5 Hz), 3.89 (1H, dd, J = 10.5, 3.8 Hz), 4.98-5.00 (1H, m), 6.55 (1H, s), 6.69 (1H, d, J = 2.2 Hz), 7.17 (1H, d, J = 8.3 Hz), 7.24 (1H, dd, J = 8.3, 2.2 Hz), 7.36-7.39 (1H, m), 7.43-7.47 (1H, m), 7.54-7.59 (2H, m), 7.80 (1H, s), 7.87-7.91 (3H, m).

### Referential Example 746

Isopropyl 2-[(trans)-7-chloro-5-(1-naphthyl)-2-thioxo-1,2,3,5-tetrahydro-4,1-benzothiazepin-3-yl]acetate

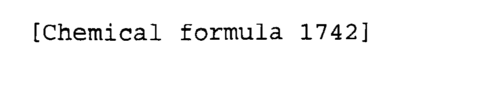

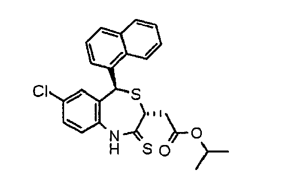

Isopropyl 2-[(trans)-7-chloro-5-(1-naphthyl)-2-oxo-1,2,3,5-tetrahydro-4,1-benzothiazepin-3-yl]acetate (749 mg) was dissolved in toluene (15 mL). To the resulting solution was added Lawesson's reagent (757 mg). The resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (790 mg).
¹H-NMR (CDCl₃) δ: 2.68 (1H, dd, J = 17.1, 3.9 Hz), 3.46 (1H, dd, J = 17.1, 10.7 Hz), 4.15 (1H, dd, J = 10.7, 3.9 Hz), 4.96-5.03 (1H, m), 6.33 (1H, s), 6.65 (1H, d, J = 2.2 Hz), 7.21 (1H, d, J = 8.5 Hz), 7.26-7.34 (3H, m), 7.47-7.55 (2H, m), 7.77-7.85 (3H, m), 10.26 (1H, s).

### Example 820

Isopropyl 2-[(trans)-8-chloro-6-(1-naphthyl)-1-(trifluoromethyl)-4H,6H-[1,2,4]triazolo[4,3-a][4,1]benzothiazepin-4-yl]acetate

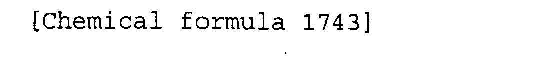

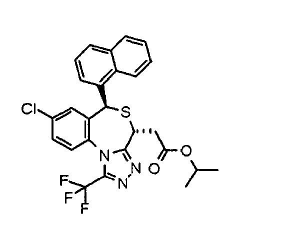

Isopropyl 2-[(trans)-7-chloro-5-(1-naphthyl)-2-thioxo-1,2,3,5-tetrahydro-4,1-benzothiazepin-3-yl]acetate (790 mg) was dissolved in tetrahydrofuran (15 mL). To the resulting solution was added hydrazine monohydrate (0.168 mL) and the resulting mixture was stirred at room temperature for 2 hours. To the reaction mixture was added 1,2-dichloroethane (15 mL), followed by the addition of trifluoroacetic anhydride (1.22 mL) and trifluoroacetic acid (2.67 mL). The resulting mixture was stirred at room temperature for one hour. After the reaction mixture was stirred at 55°C for 2 hours, toluene (15 mL) was added and the resulting mixture was heated under reflux for further one hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) to give the title compound (618 mg).
MS (ESI) m/z : 532 (M+H)⁺.
¹H-NMR (CDCl₃) δ: 1.23 (3H, d, J = 6.6 Hz), 1.26 (3H, d, J = 6.3 Hz), 2.94 (2H, dd, J = 17.2, 5.9 Hz), 3.65 (1H, dd, J = 17.2, 9.2 Hz), 4.39 (1H, dd, J = 9.2, 5.9 Hz), 4.98-5.04 (1H, m), 5.67 (1H, s), 6.86-6.87 (1H, m), 7.18-7.20 (1H, m), 7.30-7.34 (1H, m), 7.42-7.45 (3H, m), 7.55-7.57 (1H, m), 7.85-7.90 (3H, m).

### Example 821

2-[(trans)-8-Chloro-6-(1-naphthyl)-1-(trifluoromethyl)-4H,6H-[1,2,4]triazolo[4,3-a][4,1]benzothiazepin-4-yl]acetic acid

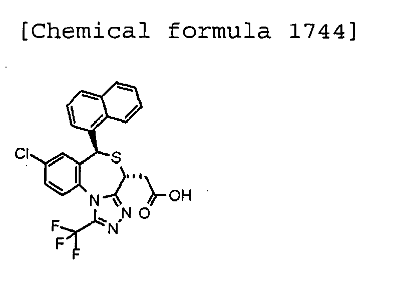

Isopropyl 2-[(trans)-8-chloro-6-(1-naphthyl)-1-(trifluoromethyl)-4H,6H-[1,2,4]triazolo[4,3-a][4,1]benzothiazepin-4-yl]acetate (200 mg) was dissolved in 1,4-dioxane (5 mL). To the resulting solution was added concentrated hydrochloric acid (1.1 mL). The resulting mixture was stirred at 60°C for 67 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (86 mg).
MS (ESI) m/z : 490 (M+H)⁺.
¹H-NMR (CDCl₃) δ: 2.87-2.90 (1H, m), 3.48-3.52 (1H, m), 4.35 (1H, dd, J = 9.4, 4.5 Hz), 5.60 (1H, s), 6.75-6.77 (1H, m), 7.14 (1H, d, J = 8.5 Hz), 7.29-7.32 (2H, m), 7.38-7.40 (1H, m), 7.46-7.48 (2H, m), 7.76-7.81 (3H, m).

### Referential Example 747

2-[(trans)-7-Chloro-5-(2-chlorophenyl)-2-oxo-1,2,3,5-tetrahydro-4,1-benzothiazepin-3-yl]acetic acid

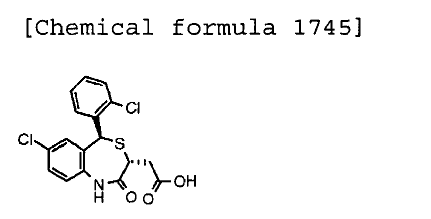

(2-Amino-5-chlorophenyl)(2-chlorophenyl)methanol (1.01 g) and thiomalic acid (0.564 g) were dissolved in a mixed solution of concentrated hydrochloric acid (7 mL) and acetic acid (15 mL). The resulting solution was stirred at 70°C for 6 hours. After cooling, the reaction mixture was neutralized with a 5N aqueous sodium hydroxide solution and extracted with dichloromethane containing 10% tetrahydrofuran. The extract was washed with saturated brine. The organic layer was dried over anhydrous magnesium sulfate. The filtrate was then concentrated.
The residue was dissolved in toluene (30 mL). The resulting solution was heated under reflux for 15 hours. The reaction mixture was cooled to room temperature and then concentrated. The residue thus obtained was dissolved in methanol-water-tetrahydrofuran (2:1:2, 37.5 mL). To the resulting solution was added lithium hydroxide monohydrate (0.311 g). The resulting mixture was stirred at 50°C for 20 hours. The reaction mixture was made acidic with 1N hydrochloric acid and then, extracted with ethyl acetate. The extract was washed with water. The organic layer was dried over anhydrous sodium sulfate. The filtrate was then concentrated. The solid thus precipitated was collected by filtration with ethanol to give the title compound (1.40 g).
MS (ESI) m/z : 382 (M+H)⁺.

### Referential Example 748

Isopropyl 2-[(trans)-7-chloro-5-(2-chlorophenyl)-2-oxo-1,2,3,5-tetrahydro-4,1-benzothiazepin-3-yl]acetate

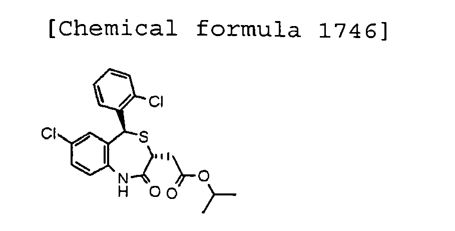

2-[(trans)-7-Chloro-5-(2-chlorophenyl)-2-oxo-1,2,3,5-tetrahydro-4,1-benzothiazepin-3-yl]acetic acid (1.40 g) was dissolved in N,N-dimethylformamide (20 mL). To the resulting solution were added 2-iodopropane (0.402 mL) and potassium carbonate (0.607 g). The resulting mixture was stirred at 30°C for 11 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:1) to give the title compound (761 mg).
MS (ESI) m/z : 424 (M+H⁺).
¹H-NMR (CDCl₃) δ: 1.21 (6H, d, J = 6.6 Hz), 1.23 (6H, d, J = 6.3 Hz), 2.43 (1H, dd, J = 17.1, 3.9 Hz), 3.10 (1H, dd, J = 17.1, 10.5 Hz), 3.81 (1H, dd, J = 10.5, 3.9 Hz), 4.97-4.99 (1H, m), 6.20 (1H, s), 6.65 (1H, d, J = 2.4 Hz), 7.11 (1H, d, J = 8.3 Hz), 7.28-7.29 (1H, m), 7.35-7.37 (1H, m), 7.41-7.44 (2H, m), 7.79-7.80 (1H, m).

### Referential Example 749

Isopropyl 2-[(trans)-7-chloro-5-(2-chlorophenyl)-2-thioxo-1,2,3,5-tetrahydro-4,1-benzothiazepin-3-yl]acetate

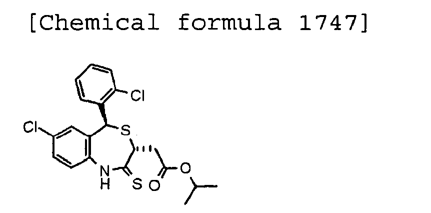

Isopropyl 2-[(trans)-7-chloro-5-(2-chlorophenyl)-2-oxo-1,2,3,5-tetrahydro-4,1-benzothiazepin-3-yl]acetate (761 mg) was dissolved in toluene (15 mL). To the resulting solution was added Lawesson's reagent (797 mg). The resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (828 mg).
MS (ESI) m/z : 440 (M+H⁺).
¹H-NMR (CDCl₃) δ: 1.22-1.23 (6H, m), 2.60 (1H, dd, J = 17.1, 3.9 Hz), 3.40 (1H, dd, J = 17.1, 10.0 Hz), 4.06 (1H, dd, J = 10.0, 3.9 Hz), 4.97-4.99 (1H, m), 6.02 (1H, s), 6.64 (1H, d, J = 2.2 Hz), 7.14 (1H, d, J = 8.5 Hz), 7.34-7.42 (4H, m), 7.74 (1H, dd, J = 7.6, 1.7 Hz), 9.21 (1H, s).

### Example 822

Isopropyl 2-[(trans)-8-chloro-6-(2-chlorophenyl)-1-(trifluoromethyl)-4H,6H-[1,2,4]triazolo[4,3-a][4,1]benzothiazepin-4-yl]acetate

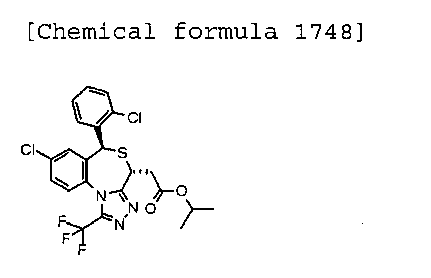

Isopropyl 2-[(trans)-7-chloro-5-(2-chlorophenyl)-2-thioxo-1,2,3,5-tetrahydro-4,1-benzothiazepin-3-yl]acetate (828 mg) was dissolved in tetrahydrofuran (20 mL). To the resulting solution was added hydrazine monohydrate (0.228 mL). The resulting mixture was stirred at room temperature for 4 hours. To the reaction mixture was added 1,2-dichloroethane (15 mL), followed by the addition of trifluoroacetic anhydride(1.86 mL) and trifluoroacetic acid (2.90 mL). The resulting mixture was stirred at room temperature for one hour. After stirring at 55°C for 2 hours, toluene (15 mL) was added. The resulting mixture was heated under reflux for further one hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) to give the title compound (816 mg) .
MS (ESI) m/z : 516 (M+H)⁺.
¹H-NMR (CDCl₃) δ: 1.22 (3H, d, J = 6.3 Hz), 1.25 (3H, d, J = 6.3 Hz), 2.91 (1H, dd, J = 17.1, 5.4 Hz), 3.61 (1H, dd, J = 17.1, 9.0 Hz), 4.33 (1H, dd, J = 9.0, 5.4 Hz), 4.98-5.00 (1H, m), 5.33 (1H, s), 6.82 (1H, d, J = 2.2 Hz), 7.39-7.46 (5H, m), 7.71-7.72 (1H, m).

### Example 823

2-[(trans)-8-Chloro-6-(2-chlorophenyl)-1-(trifluoromethyl)-4H,6H-[1,2,4]triazolo[4,3-a][4,1]benzothiazepin-4-yl]acetic acid

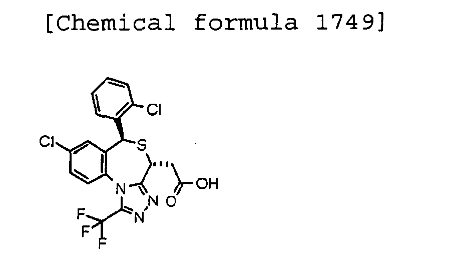

Isopropyl 2-[(trans)-8-chloro-6-(2-chlorophenyl)-1-(trifluoromethyl)-4H,6H-[1,2,4]triazolo[4,3-a][4,1]benzothiazepin-4-yl]acetate (225 mg) was dissolved in 1,4-dioxane (5 mL). To the resulting solution was added concentrated hydrochloric acid (1.1 mL). The resulting mixture was stirred at 60°C for 39 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (126 mg).
MS (ESI) m/z : 474 (M+H)⁺.
¹H-NMR (CDCl₃) δ: 2.97 (1H, dd, J = 17.1, 4.9 Hz), 3.63 (1H, dd, J = 17.1, 9.3 Hz), 4.30 (1H, dd, J = 9.3, 4.9 Hz), 5.33 (1H, s), 6.81-6.82 (1H, m), 7.39-7.46 (5H, m), 7.70-7.72 (1H, m).

### Referential Example 750

2-[trans-7-Chloro-5-(3-methoxy-2-methylphenyl)-2-oxo-1,2,3,5-tetrahydro-4,1-benzothiazepin-3-yl]acetic acid

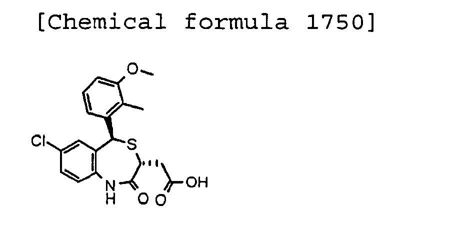

(2-Amino-5-chlorophenyl)(3-methoxy-2-methylphenyl)methanol (377 mg) and thiomalic acid (204 mg) were dissolved in a mixed solvent of concentrated hydrochloric acid (3 mL) and acetic acid (6 mL). The resulting solution was stirred at 70°C for 6 hours. After cooling, the reaction mixture was neutralized with a 5N aqueous sodium hydroxide solution and extracted with dichloromethane containing 10% tetrahydrofuran. The extract was washed with saturated brine. The organic layer was dried over anhydrous magnesium sulfate. The filtrate was then concentrated and the residue was dissolved in toluene (30 mL). The resulting solution was heated under reflux for 15 hours. The reaction mixture was cooled to room temperature and then, concentrated, The residue thus obtained was dissolved in methanol-water-tetrahydrofuran (2:1:2, 25 mL). To the resulting solution was added lithium hydroxide monohydrate (110 mg). The resulting mixture was stirred at 50°C for 20 hours. The reaction mixture was made acidic with 1N hydrochloric acid and extracted with ethyl acetate. The extract was washed with water. The organic layer was dried over anhydrous sodium sulfate. The filtrate was then concentrated. The solid thus precipitated was collected by filtration with ethanol to give the title compound (493 mg).
MS (ESI) m/z : 392 (M+H)⁺.

### Referential Example 751

Isopropyl 2-[trans-7-chloro-5-(3-methoxy-2-methylphenyl)-2-oxo-1,2,3,5-tetrahydro-4,1-benzothiazepin-3-yl]acetate

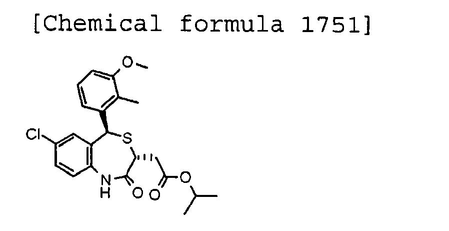

2-[trans-7-Chloro-5-(3-methoxy-2-methylphenyl)-2-oxo-1,2,3,5-tetrahydro-4,1-benzothiazepin-3-yl]acetic acid (493 mg) was dissolved in N,N-dimethylformamide (10 mL). To the resulting solution were added 2-iodopropane (0.138 mL) and potassium carbonate (209 mg). The resulting mixture was stirred at 30°C for 11 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:1) to give the title compound (324 mg).
MS (ESI) m/z : 434 (M+H)⁺.
¹H-NMR (CDCl₃) δ: 1.21 (3H, d, J = 6.1 Hz), 1.23 (3H, d, J = 6.3 Hz), 1.92 (3H, s), 2.42 (1H, dd, J = 17.1, 3.7 Hz), 3.09 (1H, dd, J = 17.0, 10.6 Hz), 3.80 (1H, dd, J = 10.6, 3.7 Hz), 3.84 (3H, s), 4.96-4.98 (1H, m), 5.96 (1H, s), 6.75 (1H, d, J = 2.4 Hz), 6.89-6.91 (1H, m), 7.09 (1H, d, J = 8.3 Hz), 7.25-7.26 (1H, m), 7.31-7.33 (2H, m).

### Referential Example 752

Isopropyl 2-[trans-7-chloro-5-(3-methoxy-2-methylphenyl)-2-thioxo-1,2,3,5-tetrahydro-4,1-benzothiazepin-3-yl]acetate

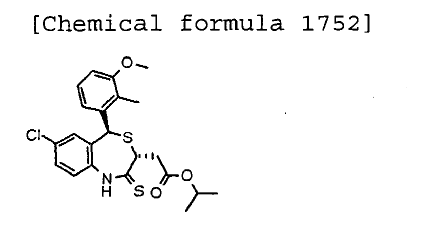

Isopropyl 2-[trans-7-chloro-5-(3-methoxy-2-methylphenyl)-2-oxo-1,2,3,5-tetrahydro-4,1-benzothiazepin-3-yl]acetate (324 mg) was dissolved in toluene (10 mL). To the resulting solution was added Lawesson's reagent (332 mg). The resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (345 mg).
MS (ESI) m/z : 450 (M+H)⁺.
¹H-NMR (CDCl₃) δ: 1.22 (6H, t, J = 6.3 Hz), 1.93 (3H, s), 2.60 (1H, dd, J = 16.8, 3.9 Hz), 3.38 (1H, dd, J = 16.8, 10.2 Hz), 3.84 (3H, s), 4.05 (1H, dd, J = 10.2, 3.9 Hz), 4.97-4.99 (1H, m), 5.78 (1H, s), 6.74 (1H, d, J = 2.4 Hz), 6.90-6.91 (1H, m), 7.12 (1H, d, J = 8.5 Hz), 7.28-7.30 (3H, m), 9.15 (1H, s).

### Example 824

Isopropyl 2-[trans-8-chloro-6-(3-methoxy-2-methylphenyl)-1-(trifluoromethyl)-4H,6H-[1,2,4]triazolo[4,3-a] [4,1]benzothiazepin-4-yl]acetate

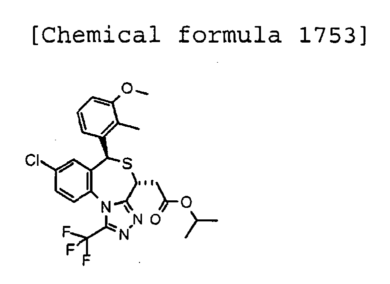

Isopropyl 2-[trans-7-chloro-5-(3-methoxy-2-methylphenyl)-2-thioxo-1,2,3,5-tetrahydro-4,1-benzothiazepin-3-yl]acetate (345 mg) was dissolved in tetrahydrofuran (10 mL). To the resulting solution was added hydrazine monohydrate (0.093 mL). The resulting mixture was stirred at room temperature for 4 hours. To the reaction mixture was added 1,2-dichloroethane (15 mL), followed by the addition of trifluoroacetic anhydride (0.541 mL) and trifluoroacetic acid (1.18 mL). The resulting mixture was stirred at room temperature for one hour. The reaction mixture was stirred at 55°C for 2 hours and toluene (15 mL) was the added thereto. The resulting mixture was heated under reflux for further 1 hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) to give the title compound (209 mg).
MS (ESI) m/z : 526 (M+H)⁺.
¹H-NMR (CDCl₃) δ: 1.22 (3H, d, J = 6.1 Hz), 1.24 (3H, d, J = 6.3 Hz), 2.88 (1H, dd, J = 17.1, 5.4 Hz), 3.59 (1H, dd, J = 17.1, 9.2 Hz), 3.81 (3H, s), 4.31 (1H, dd, J = 9.2, 5.4 Hz), 4.98-4.99 (1H, m), 5.09 (1H, s), 6.90-6.93 (2H, m), 7.27-7.29 (2H, m), 7.39 (1H, d, J = 8.5 Hz), 7.45 (1H, dd, J = 8.5, 2.3 Hz).

### Example 825

2-[trans-8-Chloro-6-(3-methoxy-2-methylphenyl)-1-(trifluoromethyl)-4H,6H-[1,2,4]triazolo[4,3-a] [4,1]benzothiazepin-4-yl]acetic acid

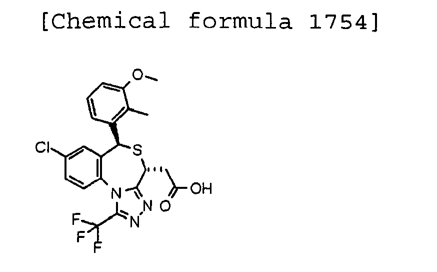

Isopropyl 2-[trans-8-chloro-6-(3-methoxy-2-methylphenyl)-1-(trifluoromethyl)-4H,6H-[1,2,4]triazolo[4,3-a] [4,1]benzothiazepin-4-yl]acetate (209 mg) was dissolved in 1,4-dioxane (5 mL). To the resulting solution was added concentrated hydrochloric acid (1.1 mL). The resulting mixture was stirred at 60°C for 39 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (142 mg).
MS (ESI) m/z : 484 (M+H)⁺.
¹H-NMR (CDCl₃) δ: 1.72 (3H, s), 2.87 (1H, dd, J = 17.1, 4.4 Hz), 3.52 (1H, dd, J = 17.1, 10.0 Hz), 3.81 (3H, s), 4.27 (1H, dd, J = 10.0, 4.4 Hz), 5.07 (1H, s), 6.90-6.91 (2H, m), 7.24-7.30 (2H, m), 7.41-7.43 (2H, m).

### Referential Example 753

2-[trans-7-Chloro-5-(2-chlorophenyl)-2-oxo-1,2,3,5-tetrahydro-4,1-benzothiazepin-3-yl]acetic acid

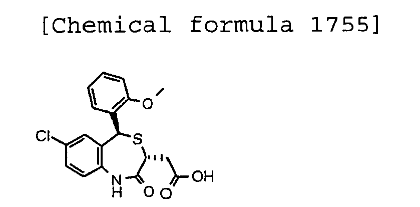

(2-Amino-5-chlorophenyl)(2-methoxyphenyl)methanol (1.28 g) and thiomalic acid (0.73 g) were dissolved in a mixed solution of concentrated hydrochloric acid (7 mL) and acetic acid (15 mL). The resulting solution was stirred at 70°C for one hour. After cooling, the reaction mixture was neutralized with a 5N aqueous sodium hydroxide solution and extracted with dichloromethane containing 10% tetrahydrofuran. The extract was washed with saturated brine. The organic layer was dried over anhydrous magnesium sulfate. The filtrate was then concentrated.
The residue was dissolved in toluene (40 mL). The resulting solution was heated under reflux for 12 hours. The reaction mixture was cooled to room temperature and then, concentrated. The residue thus obtained was dissolved in methanol-water-tetrahydrofuran (2:1:2, 50 mL). To the resulting solution was added lithium hydroxide monohydrate (0.38 g) and the resulting mixture was stirred at 50°C for 21 hours. The reaction mixture was made acidic with 1N hydrochloric acid and then, extracted with ethyl acetate. The extract was washed with water. The organic layer was dried over anhydrous sodium sulfate. The filtrate was then concentrated to give the title compound (1.63 g).
MS (ESI) m/z : 382 (M+H)⁺.
¹H-NMR (DMSO-d₆) δ: 2.39 (1H, dd, J = 16.8, 4.0 Hz), 2.75 (1H, dd, J = 16.8, 10.3 Hz), 3.59 (1H, dd, J = 10.3, 4.0 Hz), 3.71 (3H, s), 6.02 (1H, s), 6.67 (1H, d, J = 2.4 Hz), 7.11-7.14 (3H, m), 7.41-7.44 (2H, m), 7.66 (1H, dd, J = 7.6, 1.5 Hz).

### Referential Example 754

Isopropyl 2-[trans-7-chloro-5-(2-methoxyphenyl)-2-oxo-1,2,3,5-tetrahydro-4,1-benzothiazepin-3-yl]acetate

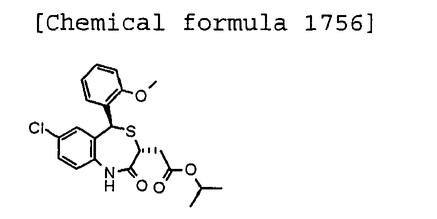

2-[trans-7-chloro-5-(2-chlorophenyl)-2-oxo-1,2,3,5-tetrahydro-4,1-benzothiazepin-3-yl]acetic acid (1.63 g) was dissolved in N,N-dimethylformamide (20 mL). To the resulting solution were added 2-iodopropane (0.470 mL) and potassium carbonate (0.72 g). The resulting mixture was stirred at room temperature for 17 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:1) to give the title compound (0.89 g).
MS (ESI) m/z : 420 (M+H)⁺.
¹H-NMR (CDCl₃) δ: 1.21 (3H, d, J = 6.3 Hz), 1.23 (3H, d, J = 6.3 Hz), 2.40 (1H, dd, J = 17.0, 3.8 Hz), 3.08 (1H, dd, J = 17.1, 10.5 Hz), 3.70 (3H, s), 3.81 (1H, dd, J = 10.5,3.8 Hz), 4.94-5.01 (1H, m), 6.23 (1H, s), 6.83 (1H, d, J = 2.4 Hz), 6.90-6.91 (1H, m), 7.07-7.09 (2H, m), 7.18 (1H, s), 7.24 (1H, dd, J = 8.3, 2.4 Hz), 7.34-7.38 (1H, m), 7.69 (1H, dd, J = 7.6, 1.7 Hz).

### Referential Example 755

Isopropyl 2-[trans-7-chloro-5-(2-methoxyphenyl)-2-thioxo-1,2,3,5-tetrahydro-4,1-benzothiazepin-3-yl]acetate

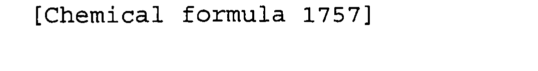

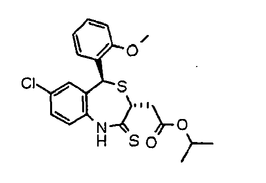

Isopropyl 2-[trans-7-chloro-5-(2-methoxyphenyl)-2-oxo-1,2,3,5-tetrahydro-4,1-benzothiazepin-3-yl]acetate (0.89 g) was dissolved in toluene (15 mL). To the resulting solution was added Lawesson's reagent (0.94 mg). The resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (765 mg). MS (ESI) m/z : 436 (M+H)⁺.
¹H-NMR (CDCl₃) δ: 1.22 (6H, t, J = 6.0 Hz), 2.58 (1H, dd, J = 17.1, 3.9 Hz), 3.37 (1H, dd, J = 17.1, 10.2 Hz), 3.68 (3H, s), 4.06 (1H, dd, J = 10.2, 3.9 Hz), 4.97-4.98 (1H, m), 6.04 (1H, s), 6.80 (1H, d, J = 2.2 Hz), 6.89 (1H, d, J = 8.3 Hz), 7.05-7.09 (1H, m), 7.11 (1H, d, J = 8.3 Hz), 7.28 (1H, dd, J = 8.3, 2.4 Hz), 7.34-7.36 (1H, m), 7.64 (1H, dd, J = 7.7, 1.6 Hz) .

### Example 826

Isopropyl 2-[trans-8-chloro-6-(2-methoxyphenyl)-1-(trifluoromethyl)-4H,6H-[1,2,4]triazolo[4,3-a] [4,1]benzothiazepin-4-yl]acetate

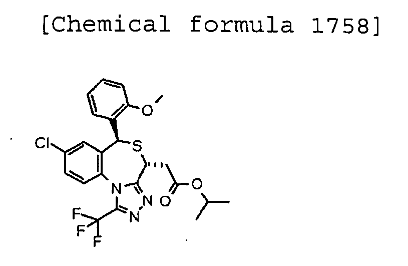

Isopropyl 2-[trans-7-chloro-5-(2-methoxyphenyl)-2-thioxo-1,2,3,5-tetrahydro-4,1-benzothiazepin-3-yl]acetate (765 mg) was dissolved in tetrahydrofuran (20 mL). To the resulting solution was added hydrazine monohydrate (0.170 mL). The resulting mixture was stirred at room temperature for 1.5 hours and concentrated. To the residue thus obtained was added 1,2-dichloroethane (15 mL), followed by the addition of trifluoroacetic anhydride (0.694 mL) and trifluoroacetic acid (2.70 mL). The resulting mixture was stirred at room temperature for one hour. The reaction mixture was stirred at 55°C for 2 hours and toluene(15 mL) was added thereto. The resulting mixture was heated under reflux for further one hour. After cooling to room temperature, the reaction mixture was concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) to give the title compound (455 mg).
MS (ESI) m/z : 512 (M+H)⁺.
¹H-NMR (CDCl₃) δ: 1.21 (3H, d, J = 6.3 Hz), 1.24 (3H, d, J = 6.3 Hz), 2.88 (1H, dd, J = 17.3, 5.4 Hz), 3.57 (3H, s), 3.59 (1H, dd, J = 17.3, 9.3 Hz), 4.29 (1H, dd, J = 9.3, 5.4 Hz), 4.95-5.02 (1H, m), 5.29 (1H, s), 6.84-6.86 (1H, m), 6.93 (1H, d, J = 2.2 Hz), 7.05-7.07 (1H, m), 7.35-7.37 (2H, m), 7.42 (1H, dd, J = 8.4, 2.2 Hz), 7.56 (1H, dd, J = 7.6, 1.5 Hz).

### Example 827

2-[trans-8-Chloro-6-(2-methoxyphenyl)-1-(trifluoromethyl)-4H,6H-[1,2,4]triazolo[4,3-a][4,1]benzothiazepin-4-yl]acetic acid

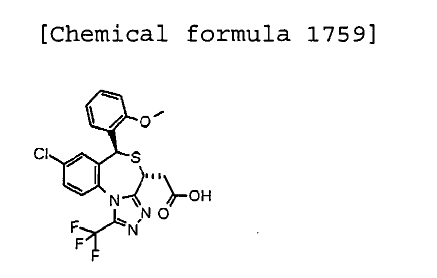

Isopropyl 2-[trans-8-chloro-6-(2-methoxyphenyl)-1-(trifluoromethyl)-4H,6H-[1,2,4]triazolo[4,3-a] [4,1]benzothiazepin-4-yl]acetate (169 mg) was dissolved in 1,4-dioxane (5 mL). To the resulting solution was added concentrated hydrochloric acid (1.0 mL). The resulting mixture was stirred at 60°C for 48 hours. After cooling to room temperature, the reaction mixture was diluted with ethyl acetate and washed with water. The organic layer was dried over anhydrous sodium sulfate. The filtrate was then concentrated. The residue obtained by concentration was purified by preparative TLC (methanol:chloroform=1:9) to give the title compound (60 mg).
MS (ESI) m/z : 470 (M+H)⁺.
¹H-NMR (CDCl₃) δ: 2.84-2.86 (1H, m), 3.40-3.43 (1H, m), 3.56 (3H, s), 4.26-4.27 (1H, m), 5.26 (1H, s), 6.84 (1H, d, J = 8.3 Hz), 6.88-6.90 (1H, m), 7.03-7.05 (1H, m), 7.34-7.36 (3H, m), 7.53-7.54 (1H, m).

### Referential Example 756

2-[trans-7-Chloro-5-(2-hydroxy-3-methoxyphenyl)-2-oxo-1,2,3,5-tetrahydro-4,1-benzothiazepin-3-yl]acetic acid

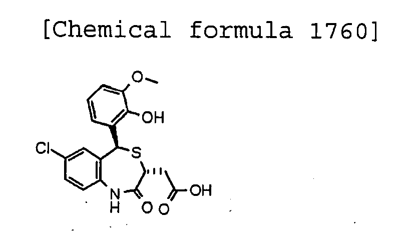

2-[[2-(Benzyloxy)-3-methoxyphenyl](ethoxy)methyl]-4-chloroaniline) (1.70 g) and thiomalic acid (0.64 g) were dissolved in a mixed solution of concentrated hydrochloric acid (7 mL) and acetic acid (15 mL). The resulting solution was stirred at 70°C for one hour. The reaction mixture was cooled and neutralized with a 5N aqueous sodium hydroxide solution and extracted with dichloromethane containing 10% tetrahydrofuran. The extract was washed with saturated brine. The organic layer was dried over anhydrous magnesium sulfate. The filtrate was then concentrated and the residue was dissolved in toluene (40 mL). The resulting solution was heated under reflux for 12 hours. The reaction mixture was cooled to room temperature and then, concentrated. The residue thus obtained was dissolved in methanol-water-tetrahydrofuran (2:1:2, 50 mL). To the resulting solution was added lithium hydroxide monohydrate (0.52 g). The resulting mixture was stirred at 50°C for 21 hours. The reaction mixture was made acidic with 1N hydrochloric acid, was extracted with ethyl acetate, and washed with water. The organic layer was dried over anhydrous sodium sulfate. The filtrate was then concentrated to give the title compound (1.48 g).
¹H-NMR (DMSO-d₆) δ: 2.39 (1H, dd, J = 16.8, 4.2 Hz), 2.75 (1H, dd, J = 16.8, 10.3 Hz), 3.57 (1H, dd, J = 10.1, 4.0 Hz), 3.82 (3H, s), 6.01 (1H, s), 6.70 (1H, d, J = 2.4 Hz), 6.95 (1H, t, J = 7.9 Hz), 7.03 (1H, dd, J = 8.3, 1.5 Hz), 7.13 (1H, d, J = 8.5 Hz), 7.17 (1H, dd, J = 7.9, 1.5 Hz), 7.39 (1H, dd, J = 8.5, 2.4 Hz), 9.03 (1H, s), 9.99 (1H, s).

### Referential Example 757

Allyl 2-{trans-5-[2-(allyloxy)-3-methoxyphenyl]-7-chloro-2-oxo-1,2,3,5-tetrahydro-4,1-benzothiazepin-3-yl}acetate

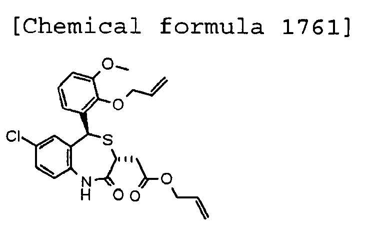

2-[trans-7-Chloro-5-(2-hydroxy-3-methoxyphenyl)-2-oxo-1,2,3,5-tetrahydro-4,1-benzothiazepin-3-yl]acetic acid (1.50 g) was dissolved in N,N-dimethylformamide (20 mL).
To the resulting solution were added allyl bromide (0.73 mL) and potassium carbonate (1.26 g). The resulting mixture was stirred at room temperature for 73 hours. The reaction mixture was diluted with ethyl acetate and washed with saturated brine. The organic layer was dried over anhydrous sodium sulfate. The filtrate was then concentrated. The residue was purified by silica gel column (ethyl acetate:hexane=1:2) to give the title compound (0.54 g).
MS (ESI) m/z : 474 (M+H)⁺.
¹H-NMR (CDCl₃) δ: 2.47 (1H, dd, J = 17.2, 3.9 Hz), 3.15 (1H, dd, J = 17.2, 10.4 Hz), 3.83 (1H, dd, J = 10.4, 3.9 Hz), 3.86 (3H, s), 4.09-4.14 (1H, m), 4.40-4.44 (1H, m), 4.56-4.57 (2H, m), 5.03-5.09 (2H, m), 5.20-5.32 (2H, m), 5.73-5.78 (1H, m), 5.85-5.89 (1H, m), 6.24 (1H, s), 6.85 (1H, d, J = 2.4 Hz), 6.97 (1H, dd, J = 8.3, 1.5 Hz), 7.08 (1H, d, J = 8.3 Hz), 7.18 (1H, t, J = 8.0 Hz), 7.23 (1H, dd, J = 8.3, 2.4 Hz), 7.31 (1H, dd, J = 7.8, 1.5 Hz), 7.74 (1H, s).

### Referential Example 758

Allyl 2-{trans-5-[2-(allyloxy)-3-methoxyphenyl]-7-chloro-2-thioxo-1,2,3,5-tetrahydro-4,1-benzothiazepin-3-yl}acetate

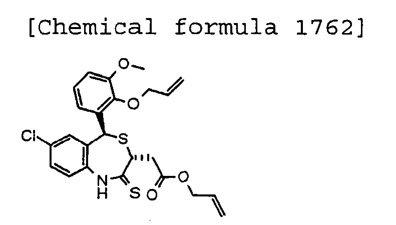

Allyl 2-{trans-5-[2-(allyloxy)-3-methoxyphenyl]-7-chloro-2-oxo-1,2,3,5-tetrahydro-4,1-benzothiazepin-3-yl}acetate (0.536 g) was dissolved in toluene (15 mL). To the resulting solution was added Lawesson's reagent (0.503 mg) and the resulting mixture was heated under reflux for one hour. The reaction mixture was cooled to room temperature and then concentrated. The residue was purified by silica gel column (ethyl acetate:hexane=1:4 and ethyl acetate:hexane=1:2) to give the title compound (480 mg).
MS (ESI) m/z : 490 (M+H)⁺.
¹H-NMR (CDCl₃) δ: 2.64 (1H, dd, J = 17.1, 3.9 Hz), 3.47 (1H, dd, J = 17.1, 10.0 Hz), 3.86 (3H, s), 4.07 (1H, dd, J = 10.0, 3.9 Hz), 4.16-4.21 (1H, m), 4.41-4.43 (1H, m), 4.56-4.58 (2H, m), 5.08-5.14 (2H, m), 5.21-5.23 (1H, m), 5.28-5.32 (1H, m), 5.76-5.91 (2H, m), 6.07 (1H, s), 6.85 (1H, d, J = 2.4 Hz), 6.97 (1H, dd, J = 8.2, 1.6 Hz), 7.09-7.10 (1H, m), 7.18 (1H, t, J = 8.0 Hz), 7.24-7.26 (1H, m), 7.27-7.28 (1H, m).

### Example 828

Allyl 2-[trans-6-[2-(allyloxy)-3-methoxyphenyl]-8-chloro-1-(trifluoromethyl)-4H,6H-[1,2,4]triazolo[4,3-a] [4,1]benzothiazepin-4-yl]acetate

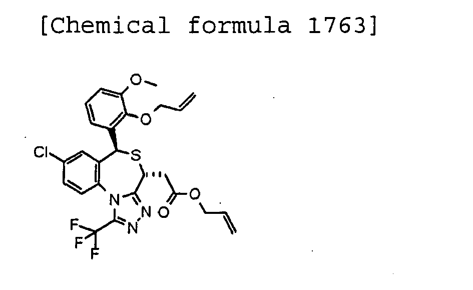

Allyl 2-{trans-5-[2-(allyloxy)-3-methoxyphenyl]-7-chloro-2-thioxo-1,2,3,5-tetrahydro-4,1-benzothiazepin-3-yl}acetate (400 mg) was dissolved in tetrahydrofuran (10 mL). To the resulting solution was added hydrazine monohydrate (0.079 mL). The resulting mixture was stirred at room temperature for 1.5 hours, followed by concentration. To the residue thus obtained was added 1,2-dichloroethane (10 mL), followed by the addition of trifluoroacetic anhydride (0.322 mL) and trifluoroacetic acid (1.26 mL). The resulting mixture was stirred at room temperature for one hour. After stirring at 55°C for 2 hours, toluene (10 mL) was added to the reaction mixture. The resulting mixture was heated under reflux for further one hour. The reaction mixture was cooled to room temperature and then, concentrated. The residue was diluted with chloroform and washed with a saturated aqueous solution of sodium bicarbonate. The organic layer was dried over anhydrous sodium sulfate. After concentration of the filtrate, the residue thus obtained was purified by silica gel column (ethyl acetate:hexane=1:2) to give the title compound (191 mg).
MS (ESI) m/z : 566(M+H)⁺.
¹H-NMR (CDCl₃) δ: 2.93 (1H, dd, J = 17.3, 5.4 Hz), 3.66 (1H, dd, J = 17.3, 9.2 Hz), 3.85 (3H, s), 4.14-4.17 (1H, m), 4.32-4.34 (2H, m), 4.59-4.60 (2H, m), 5.22-5.26 (2H, m), 5.29-5.33 (2H, m), 5.42 (1H, s), 5.85-5.91 (2H, m), 6.96-6.98 (1H, m), 7.03 (1H, d, J = 2.2 Hz), 7.17-7.19 (1H, m), 7.24-7.25 (1H, m), 7.34-7.37 (1H, m), 7.41-7.43 (1H, m).

### Example 829

2-[trans-8-Chloro-6-(2-hydroxy-3-methoxyphenyl)-1-(trifluoromethyl)-4H,6H-[1,2,4]triazolo[4,3-a] [4,1]benzothiazepin-4-yl]acetic acid

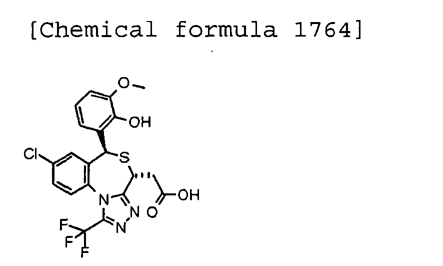

Allyl 2-[trans-6-[2-(allyloxy)-3-methoxyphenyl]-8-chloro-1-(trifluoromethyl)-4H,6H-[1,2,4]triazolo[4,3-a] [4,1]benzothiazepin-4-yl]acetate (143 mg) was dissolved in toluene (12 mL). To the resulting solution were added a triethylaluminum hexane solution (0.47 mL) and [1,3-bis(diphenylphosphino)propane]nickel chloride at room temperature in a nitrogen atmosphere. The resulting mixture was stirred at 100°C for 2 hours. After cooling to room temperature, a saturated aqueous solution of sodium bicarbonate was added. The resulting mixture was neutralized with 1N hydrochloric acid and extracted with ethyl acetate. The organic layer was dried over anhydrous sodium sulfate. The filtrate was concentrated. The residue thus obtained was purified by preparative TLC (methanol:chloroform:water=3:7:1) to give the title compound (57 mg).
MS (ESI) m/z : 486 (M+H)⁺.
¹H-NMR (CDCl₃) δ: 2.78-2.81 (1H, m), 3.36-3.39 (1H, m), 3.86 (3H, s), 4.27-4.30 (1H, m), 5.29 (1H, s), 6.89-6.91 (2H, m), 6.98-7.00 (1H, m), 7.15-7.16 (1H, m), 7.34-7.37 (2H, m).

### Referential Example 759

Ethyl (4S)-4-{[(benzyloxy)carbonyl]amino}-3-oxopentanoate

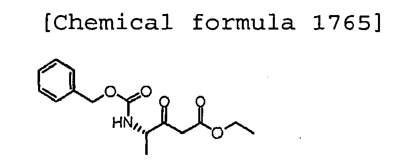

(2S)-2-{[(Benzyloxy)carbonyl]amino}propionic acid (6.00 g) was dissolved in tetrahydrofuran (200 mL). To the resulting solution was added carbonyldiimidazole (3.46 g). The resulting mixture was stirred at room temperature for 2 hours. After ice cooling, monoethyl malonate potassium salt (3.63 g) and magnesium chloride (1.86 g) were added. The resulting mixture was stirred for 17 hours while warming slowly. To the reaction mixture were added a saturated aqueous solution of ammonium chloride and ethyl acetate and the layers separated. The organic layer thus obtained was washed with saturated brine and dried over anhydrous sodium sulfate. The organic solvent was distilled off under reduced pressure. The residue thus obtained was purified by column chromatography (ethyl acetate:n-hexane=1:1) to give the title compound (2.69 g). ¹H-NMR (CDCl₃) δ: 1.27 (3H, t, J = 7.1 Hz), 1.36-1.44 (3H, m), 3.56 (2H, dd, J = 22.8, 16.7 Hz), 4.15-4.24 (2H, m), 4.43-4.54 (1H, m), 5.12 (2H, s), 5.39-5.47 (1H, m), 7.40-7.30 (5H, m).

### Referential Example 760

Ethyl (5S)-5-{[(benzyloxy)carbonyl]amino}-4-oxohexanoate

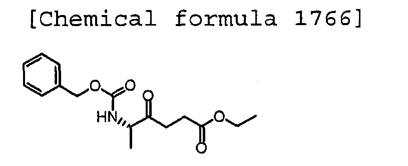

To a solution of diiodomethane (13.01 g, 3.91 mL) in dichloromethane(44 mL) was added dropwise diethylzinc (a 1.0 mol/l hexane solution, 32.05 mL) under ice cooling and the resulting mixture was stirred for 2 minutes. To the reaction mixture was added dropwise a dichloromethane solution (20 mL) of (4S)-4-{[(benzyloxy)carbonyl]amino}-3-oxopentanoate (1.88 g) over 15 minutes. The reaction mixture was stirred for further 30 minutes under ice cooling, followed by the addition of a saturated aqueous solution of ammonium chloride. The resulting mixture was stirred at room temperature for 15 minutes and then the layers were separated. The organic layer thus obtained was washed with saturated brine and was dried over anhydrous sodium sulfate. The organic solvent was distilled off under reduced pressure. The residue thus obtained was purified by column chromatography (ethyl acetate:n-hexane=1:2) to give the title compound (1.48 g).
¹H-NMR (CDCl₃) δ: 1.25 (3H, t, J = 7.1 Hz), 1.40 (3H, d, J = 7.1 Hz), 2.54-2.93 (4H, m), 4.12 (2H, q, J = 7.1 Hz), 4.35-4.52 (1H, m), 5.10 (2H, s), 5.48-5.54 (1H, m), 7.39-7.29 (5H, m).

### Referential Example 761

Ethyl (5S)-5-amino-4-oxohexanoate hydrochloride

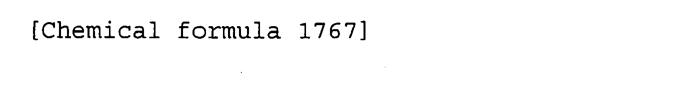

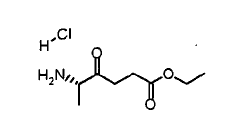

To an ethyl acetate solution (40 mL) of ethyl (5S)-5-{[(benzyloxy)carbonyl]amino}-4-oxohexanoate (1.48 g) were added 10% palladium-carbon (51.7% weight ratio, 1.48 g) and a 4N hydrochloric acid-ethyl acetate solution (8 mL). The resulting mixture was stirred for 72 hours in a hydrogen atmosphere. The reaction mixture was filtered through Celite 545 and the filtrate was washed sufficiently with ethyl acetate. The filtrates were combined and
concentrated under reduced pressure to give the title compound (1.03 g).
¹H-NMR (CDCl₃) δ: 1.20-1.30 (3H, m), 2.46-3.11 (4H, m), 3.63-3.75 (2H, m), 4.07-4.18 (1H, m), 8.69-8.02 (3H, m).

### Referential Example 762

Ethyl 5-({2-[(trans)-7-chloro-5-(2,3-dimethoxyphenyl)-2-oxo-1,2,3,5-tetrahydro-4,1-benzothiazepin-3-yl]acetyl}amino)-4-oxohexanoate

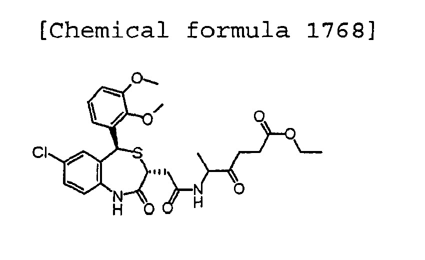

To an N,N-dimethylformamide solution (9.0 mL) of 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetic acid (368 mg) were added (5S)-5-amino-4-oxohexanoate hydrochloride (208 mg), 1-ethyl-3-(3-dimethylaminopropyl)carbodiimide hydrochloride (259 mg), 1-hydroxybenzotriazole monohydrate(69 mg), and triethylamine (100 mg). The resulting mixture was stirred at room temperature for 12.5 hours. The reaction mixture was concentrated under reduced pressure. The residue was dissolved in ethyl acetate. To the resulting solution was added distilled water and the layers separated. The organic layer was washed with distilled water and saturated brine and dried over anhydrous sodium sulfate. The organic solvent was distilled off under reduced pressure. The residue thus obtained was purified by flash column chromatography (Yamazen Corp., Ultra pack B) to give the title compound (438 mg).
MS (ESI) m/z : 563 (M + H)⁺.
¹H-NMR (CDCl₃) δ: 1.24 (3H, t, J = 7.1 Hz), 1.37 (3H, d, J = 7.4 Hz), 2.31-2.43 (1H, m), 2.52-3.04 (5H, m), 3.58 (3H, s), 3.87 (3H, s), 3.88-3.93 (1H, m), 4.11 (2H, q, J = 7.1 Hz), 6.17-6.20 (1H, m), 6.43 (1H, d, J = 6.9 Hz), 6.84 (1H, d, J = 2.5 Hz), 6.97 (1H, dd, J = 8.3, 1.5 Hz), 7.07 (1H, d, J = 8.3 Hz), 7.11-7.13 (2H, m), 7.29-7.34 (1H, m), 8.02 (1H, s).

### Referential Example 763

Ethyl 3-(2-{[(trans)-7-chloro-5-(2,3-dimethoxyphenyl)-2-thioxo-1,2,3,5-tetrahydro-4,1-benzothiazepin-3-yl]methyl}-4-methyl-1,3-thiazol-5-yl)propionate

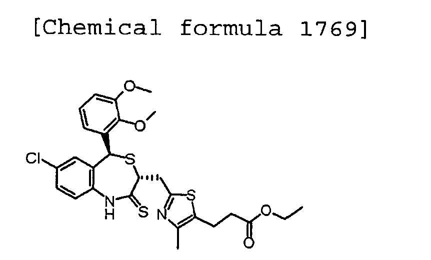

To a tetrahydrofuran solution (4.0 mL) of ethyl 5-({2-[(trans)-7-chloro-5-(2,3-dimethoxyphenyl)-2-oxo-1,2,3,5-tetrahydro-4,1-benzothiazepin-3-yl]acetyl}amino)-4-oxohexanoate (399 mg) was added Lawesson's reagent (287 mg). The resulting mixture was stirred at 70°C for 4.5 hours. To the reaction mixture were added Lawesson's reagent (430 mg) and toluene (3 mL) further. The resulting mixture was stirred at 100°C for 8 hours. After cooling to room temperature, the reaction mixture was purified by column chromatography (dichloromethane:methanol=50:1) to give the title compound (357 mg).
MS (ESI) m/z : 577 (M + H)⁺.
¹H-NMR (CDCl₃) δ: 1.25 (3H, t, J = 7.1 Hz), 2.27 (3H, s), 2.57 (2H, t, J = 7.7 Hz), 3.00 (2H, t, J = 7.6 Hz), 3.18-3.34 (1H, m), 3.61 (3H, s), 3.85-3.92 (1H, m), 3.87 (3H, s), 4.08-4.22 (4H, m), 6.03 (1H, s), 6.82 (1H, d, J = 2.5 Hz), 6.94-7.04 (2H, m), 7.17 (1H, t, J = 8.0 Hz), 7.21-7.25 (1H, m), 7.38-7.31 (1H, m), 9.11 (1H, s).

### Referential Example 764

Ethyl 3-(2-{[(trans)-7-chloro-5-(2,3-dimethoxyphenyl)-2-[hydrazono]-1,5-dihydro-4,1-benzothiazepin-3(3H)-yl]methyl}-4-methyl-1,3-thiazol-5-yl)propanoate

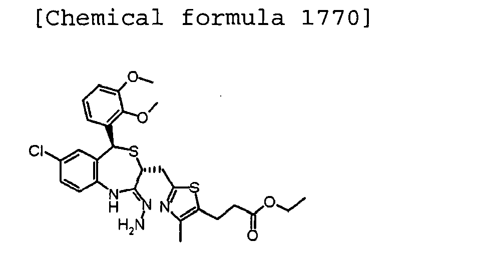

In a similar manner to that employed for the synthesis of ethyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate, the title compound (370 mg) was obtained from ethyl 3-(2-{[(trans)-7-chloro-5-(2,3-dimethoxyphenyl)-2-thioxo-1,2,3,5-tetrahydro-4,1-benzothiazepin-3-yl]methyl}-4-methyl-1,3-thiazol-5-yl)propionate (345 mg) by using hydrazine monohydrate (87 µl).
MS (ESI) m/z : 575 (M + H)⁺.
¹H-NMR (CDCl₃) δ: 1.22-1.27 (3H, m), 2.28 (3H, s), 2.55-2.62 (2H, m), 2.97-3.02 (2H, m), 3.10-3.20 (1H, m), 3.50 (3H, s), 3.58-3.70 (1H, m), 3.82-3.90 (1H, m), 3.86 (3H, s), 4.08-4.21 (3H, m), 6.09 (1H, s), 6.76 (1H, d, J = 2.5 Hz), 6.85 (2H, d, J = 8.3 Hz), 6.94 (2H, dd, J = 8.1, 1.2 Hz), 7.06-7.20 (2H, m), 7.44-7.55 (1H, m), 7.39-7.29 (1H, m) .

### Example 830

Ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-4-methyl-1,3-thiazol-5-yl)propanoate

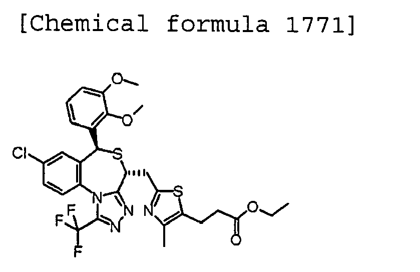

In a similar manner to that employed for the synthesis of methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate, the title compound (179 mg) was obtained from ethyl 3-(2-{[(trans)-7-chloro-5-(2,3-dimethoxyphenyl)-2-[hydrazono]-1,5-dihydro-4,1-benzothiazepin-3(3H)-yl]methyl}-4-methyl-1,3-thiazol-5-yl)propanoate(343 mg) by using trifluoroacetic anhydride (249 µl) and trifluoroacetic acid (887 µl).
MS (ESI) m/z : 653 (M + H)⁺.
¹H-NMR (CDCl₃) δ: 1.25 (3H, t, J = 7.4 Hz), 2.28 (3H, s), 2.55-2.60 (2H, m), 2.97-3.03 (2H, m), 3.43 (3H, s), 3.58 (1H, dd, J = 15.1, 7.2 Hz), 3.85 (3H, s), 4.02-4.19 (4H, m), 4.34-4.41 (1H, m), 5.34 (1H, s), 6.94-6.99 (2H, m), 7.08-7.20 (2H, m), 7.29-7.35 (1H, m), 7.44-7.38 (1H, m).

### Example 831

Ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-4-methyl-1,3-thiazol-5-yl)propanoic acid

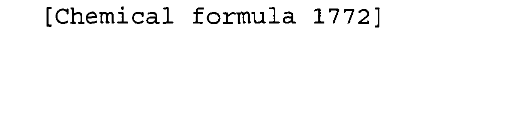

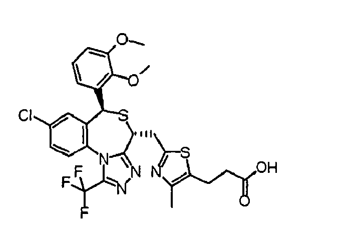

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (156 mg) was obtained from ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-4-methyl-1,3-thiazol-5-yl)propanoate (179 mg) by using potassium hydrogen carbonate (227 mg).
MS (ESI) m/z : 625 (M + H)⁺.
¹H-NMR (CDCl₃) δ: 2.28 (3H, s), 2.64 (2H, t, J = 7.5 Hz), 3.01 (2H, t, J = 7.5 Hz), 3.43 (3H, s), 3.54-3.63 (1H, m), 3.85 (3H, s), 4.03-4.11 (1H, m), 4.35-4.43 (1H, m), 5.34 (1H, s), 6.94-7.00 (2H, m), 7.12-7.20 (2H, m), 7.35-7.29 (13H, m), 7.45-7.38 (1H, m).
Elemental analysis for: C₂₇H₂₄ClF₃N₄O₄S₂·0.75H₂O
Calculated: C, 50.78; H, 4.02; N, 8.77.
Found: C, 50.87; H, 3.89; N, 8.70.

### Referential Example 765

2-[(trans)-7-Chloro-5-(2,3-dimethoxyphenyl)-2-oxo-1,2,3,5-tetrahydro-4,1-benzothiazepin-3-yl]ethanethioamide

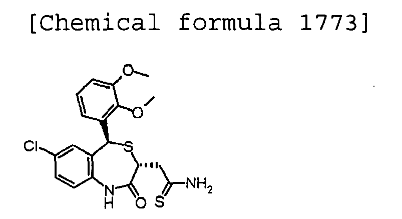

To a tetrahydrofuran solution (10 mL) of 2-[(trans)-7-chloro-5-(2,3-dimethoxyphenyl)-2-oxo-1,2,3,5-tetrahydro-4,1-benzothiazepin-3-yl]acetamide (652 mg) was added Lawesson's reagent (454 mg). The resulting mixture was stirred at room temperature for 5 hours. The solvent was distilled off under reduced pressure. The residue was purified by silica gel column chromatography (dichloromethane:methanol=20:1) to give the title compound (384 mg).
MS (ESI) m/z : 423 (M + H)⁺.
¹H-NMR (DMSO-d6) δ: 2.38-2.45 (1H, m), 2.99-3.07 (1H, m), 3.45 (3H, s), 3.82 (3H, s), 4.05-4.11 (1H, m), 5.98 (1H, s), 6.68 (1H, d, J = 2.5 Hz), 7.09-7.17 (2H, m), 7.22-7.26 (2H, m), 7.43-7.38 (1H, m), 9.32 (1H, s), 9.49 (1H, s), 9.93 (1H, s).

### Referential Example 766

Methyl 5-bromo-4-oxopentanoate

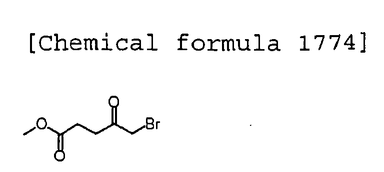

To an acetonitrile solution (16 mL) of methyl 4-chloro-4-oxobutanoate (1.22 g, 1.00 mL) was added dropwise trimethylsilyldiazomethane (a 2.0 mol/l n-hexane solution, 7.86 mL) at room temperature over 30 minutes. The reaction mixture was stirred at room temperature for 2 hours. A 30% hydrogen bromide-acetic acid solution (2.8 mL) was added and the resulting mixture was stirred at room temperature for one hour. The solvent was distilled off under reduced pressure to give the title compound (1.80 g).
MS (ESI) m/z : 231 (M + Na)⁺.
¹H-NMR (CDCl₃) δ: 2.64-2.70 (2H, m), 2.88-3.01 (2H, m), 3.69 (3H, s), 3.96 (1H, s), 4.15 (1H, s).

### Referential Example 767

Ethyl 3-(2-{[(trans)-7-chloro-5-(2,3-dimethoxyphenyl)-2-oxo-1,2,3,5-tetrahydro-4,1-benzothiazepin-3-3yl]methyl}-1,3-thiazol-4-yl)propanoate

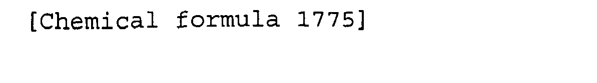

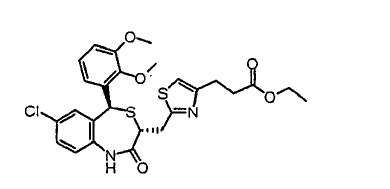

To an ethanol solution (5.0 mL) of 2-[(trans)-7-chloro-5-(2,3-dimethoxyphenyl)-2-oxo-1,2,3,5-tetrahydro-4,1-benzothiazepin-3-yl]ethanethioamide (180 mg) was added methyl 5-bromo-4-oxopentanoate (89.0 mg). The resulting mixture was heated under reflux for 10.5 hours. After cooling, the reaction mixture was distilled under reduced pressure to remove the solvent. The residue thus obtained was purified by flash column chromatography (Cartridge B-L, Yamazen Corp.) to give the title compound (204 mg).
MS (ESI) m/z : 547 (M + H)⁺.
¹H-NMR (CDCl₃) δ: 1.20-1.28 (3H, m), 2.65-2.72 (2H, m), 2.98-3.04 (2H, m), 3.13 (1H, dd, J = 15.2, 5.1 Hz), 3.60 (3H, s), 3.64-3.74 (1H, m), 3.88 (3H, s), 3.96 (1H, dd, J = 8.6, 5.1 Hz), 4.08-4.16 (2H, m), 6.22 (1H, s), 6.79-6.85 (2H, m), 6.95-6.99 (1H, m), 7.03 (1H, d, J = 8.3 Hz), 7.15-7.25 (2H, m), 7.36-7.28 (1H, m).

### Referential Example 768

Ethyl 3-(2-{[7-chloro-5-(2,3-dimethoxyphenyl)-2-thioxo-1,2,3,5-tetrahydro-4,1-benzothiazepin-3-yl]methyl}-1,3-thiazol-4-yl)propanoate

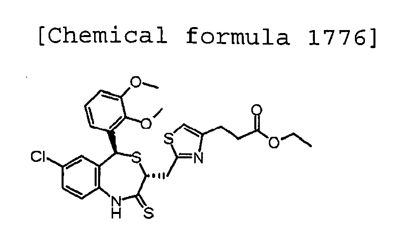

To a toluene solution (6.0 mL) of ethyl 3-(2-{[(trans)-7-chloro-5-(2,3-dimethoxyphenyl)-2-oxo-1,2,3,5-tetrahydro-4,1-benzothiazepin-3-yl]methyl}-1,3-thiazol-4-yl)propanoate (204 mg) was added Lawesson's reagent (71.9 mg). The resulting mixture was heated under reflux for 2.5 hours. To the resulting solution was added Lawesson's reagent (71.9 mg) and the resulting mixture was heated under reflux for one hour. After cooling, the solvent was distilled off under reduced pressure and the residue thus obtained was purified by column chromatography (dichloromethane:methanol=40:1) to give the title compound (143 mg).
MS (ESI) m/z : 563 (M + H)⁺.
¹H-NMR (CDCl₃) δ: 1.23 (3H, t, J = 7.1 Hz), 2.63-2.71 (2H, m), 2.97-3.03 (2H, m), 3.25-3.36 (1H, m), 3.62 (3H, s), 3.88 (3H, s), 3.93-4.00 (1H, m), 4.12 (2H, q, J = 7.1 Hz), 4.19 (1H, dd, J = 8.6, 5.1 Hz), 6.04 (1H, s), 6.78-6.84 (2H, m), 6.97 (1H, dd, J = 8.1, 1.5 Hz), 7.05 (1H, d, J = 8.3 Hz), 7.17 (1H, t, J = 8.0 Hz), 7.29-7.22 (2H, m), 9.12 (1H, s).

### Referential Example 769

Ethyl 3-[2-({(trans)-7-chloro-5-(2,3-dimethoxyphenyl)-2-[hydrazono]-1,2,3,5-tetrahydro-4,1-benzothiazepin-3-yl}methyl)-1,3-thiazol-4-yl]propanoate

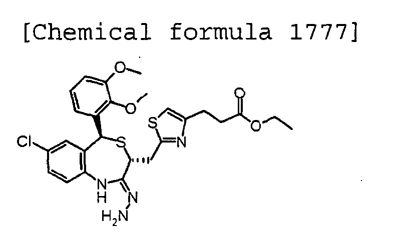

In a similar manner to that employed for the synthesis of methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate, the title compound (140 mg) was obtained from ethyl 3-(2-{[(trans)-7-chloro-5-(2,3-dimethoxyphenyl)-2-thioxo-1,2,3,5-tetrahydro-4,1-benzothiazepin-3-yl]methyl}-1,3-thiazol-4-yl)propanoate (140 mg) by using hydrazine monohydrate (72 µl).
MS (ESI) m/z : 561 (M + H)⁺.
¹H-NMR (CDCl₃) δ: 1.19-1.25 (3H, m), 2.65-2.72 (2H, m), 2.99-3.05 (2H, m), 3.50 (3H, s), 3.64-3.78 (1H, m), 3.87 (3H, s), 3.89-3.95 (1H, m), 4.08-4.14 (2H, m), 6.12 (1H, s), 6.75-6.80 (2H, m), 6.85 (1H, d, J = 8.5 Hz), 6.93-6.97 (1H, m), 7.12-7.19 (2H, m), 7.31-7.27 (1H, m).

### Example 832

Ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-4-yl)propanoate

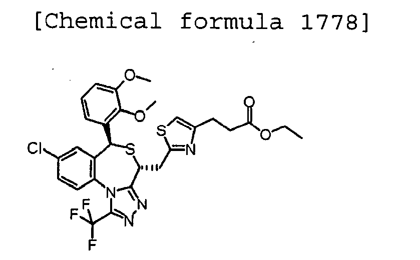

In a similar manner to that employed for the synthesis of methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate, the title compound (158 mg) was obtained from ethyl 3-[2-({(trans)-7-chloro-5-(2,3-dimethoxyphenyl)-2-[hydrazono]-1,2,3,5-tetrahydro-4,1-benzothiazepin-3-yl}methyl)-1,3-thiazol-4-yl]propanoate (140 mg) by using trifluoroacetic anhydride (104 µl) and trifluoroacetic acid (369 µl).
MS (ESI) m/z : 639 (M + H)⁺.
¹H-NMR (CDCl₃) δ: 1.21 (3H, t, J = 7.1 Hz), 2.59-2.68 (2H, m), 2.98-3.03 (2H, m), 3.43 (3H, s), 3.60-3.68 (1H, m), 3.85 (3H, s), 4.06-4.19 (4H, m), 4.40-4.46 (1H, m), 5.35 (1H, s), 6.71-6.87 (1H, m), 6.95-7.00 (2H, m), 7.13-7.20 (2H, m), 7.34-7.38 (1H, m), 7.46-7.40 (1H, m).

### Example 833

3-(2-{[(trans)-8-Chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-4-yl)propanoic acid

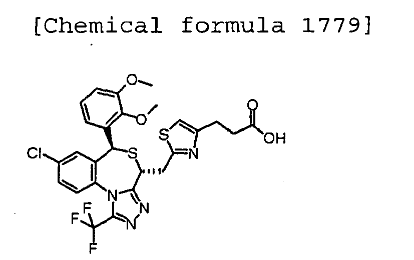

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (109 mg) was obtained from ethyl 3-(2-{[(trans)-8-chloro-6-(2,3-dimethoxyphenyl)-1-(trifluoromethyl)-4H,6H-[1,2,4]triazolo[4,3-a] [4,1]benzothiazepin-4-yl]methyl}-1,3-thiazol-4-yl)propanoate (150 mg) by using potassium hydrogen carbonate (584 mg).
MS (ESI) m/z : 611 (M + H)⁺.
¹H-NMR (CDCl₃) δ: 2.65-2.71 (2H, m), 2.96-3.01 (2H, m), 3.43 (3H, s), 3.58-3.65 (1H, m), 3.85 (3H, s), 4.19-4.27 (1H, m), 4.37-4.45 (1H, m), 5.38 (1H, s), 6.88 (1H, s), 6.96-7.01 (2H, m), 7.14-7.21 (2H, m), 7.47-7.43 (2H, m).
Elemental analysis for: C₂₆H₂₂ClF₃N₄O₄S₂·0.5H₂O
Calculated: C, 50.36; H, 3.74; N, 9.04.
Found: C, 50.24; H, 3.62; N, 9.02.

### Referential Example 770

Ethyl (tetrazol-5-yl)propanoate

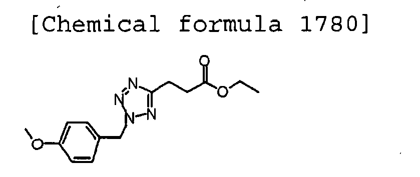

To a solution of 3-[2-(4-methoxybenzyl)-2H-1,2,3,4-tetrazol-5-yl]propanoic acid (2.25 g) in ethanol (45 mL) was added dropwise concentrated sulfuric acid (4.58 mL) under ice cooling. The reaction mixture was stirred for 17 hours while heating under reflux. The solvent was distilled off under reduced pressure. Ethyl acetate and distilled water were added. The resulting mixture was neutralized with potassium carbonate and the layers separated. The organic layer thus obtained was washed with saturated brine. The solvent was distilled off under reduced pressure. The residue was dried to give the title compound (1.28 g).
MS (ESI) m/z : 291 (M + H)⁺.
¹H-NMR (CDCl₃) δ: 1.24 (3H, t, J = 7.1 Hz), 2.82-2.89 (2H, m), 2.95-3.01 (2H, m), 3.80 (3H, s), 4.13 (2H, q, J = 7.1 Hz), 5.52 (2H, s), 6.85-6.92 (2H, m), 7.20-7.15 (2H, m).

### Referential Example 771

Ethyl 3-(2H-1,2,3,4-tetrazol-5-yl)propanoate

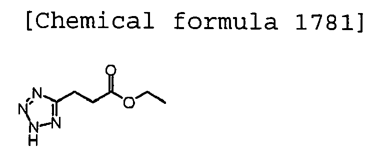

To an ethanol solution (6.0 mL) of ethyl(tetrazol-5-yl)propanoate (183 mg) was added 10% palladium-carbon (51.7%, 182 mg). The resulting mixture was stirred for 2 days in a hydrogen atmosphere. The catalyst was filtered, followed by washing with ethanol. The ethanol solutions thus obtained were combined and concentrated to give the title compound (107 mg).

### Example 834

Ethyl 3-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)propanoate

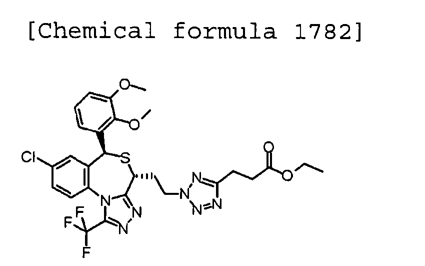

Ethyl 3-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-1,2,3,4-tetrazol-5-yl)propanoate

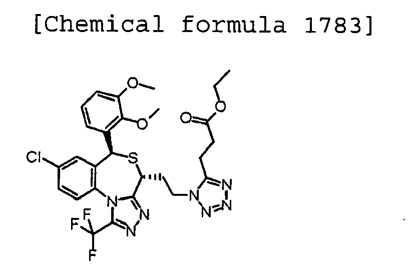

2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl methanesulfonate (163 mg) was dissolved in N,N-dimethylformamide (30 mL). To the resulting solution were added ethyl (tetrazol-5-yl)propionate (73.5 mg), potassium carbonate (239 mg) and tetrabutylammonium iodide (106 mg). The resulting mixture was stirred at 40°C for 16 hours. Ethyl (tetrazol-5-yl)propionate (49 mg) was added again and the resulting mixture was stirred at 70°C for 4 hours. To the reaction mixture were added water and ethyl acetate and the layers separated. The organic layer thus obtained was dried over anhydrous sodium sulfate. The solvent was distilled off under reduced pressure. The residue thus obtained was purified by flash column chromatography (Cartridge B-L, Yamazen Corp.) to give the title compounds, that is, 2H-isomer (92 mg) and 1H-isomer (34 mg).
2H-isomer (with low polarity)
MS (ESI) m/z : 660 (M + Na)⁺.
¹H-NMR (CDCl₃) δ: 1.25 (4H, t, J = 7.1 Hz), 2.54-2.65 (1H, m), 2.72-2.79 (2H, m), 3.10-3.16 (3H, m), 3.44 (3H, s), 3.80-3.84 (1H, m), 3.85 (3H, s), 4.14 (2H, q, J = 7.2 Hz), 4.81-4.99 (2H, m), 5.35 (1H, s), 6.95-7.01 (2H, m), 7.19-7.15 (2H, m), 7.47-7.36 (2H, m).
1H-isomer (with high polarity)
MS (ESI) m/z : 660 (M + Na)⁺.
¹H-NMR (CDCl₃) δ: 1.24 (3H, t, J = 7.1 Hz), 2.54-2.64 (1H, m), 2.88-3.13 (6H, m), 3.43 (3H, s), 3.85 (3H, s), 3.88-3.94 (1H, m), 4.10 (2H, q, J = 7.1 Hz), 4.58-4.67 (1H, m), 4.77-4.86 (1H, m), 5.36 (1H, s), 6.96-7.02 (2H, m), 7.16-7.21 (2H, m), 7.46-7.38 (2H, m).

### Example 835

3-(2-{2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)propanoic acid

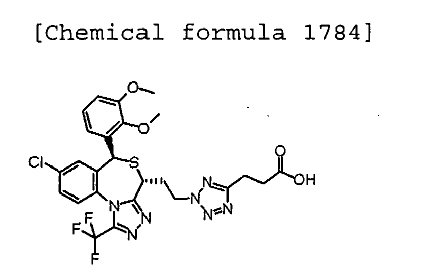

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (175 mg) was obtained from ethyl 3-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)propanoate (171 mg) by using potassium carbonate (333 mg).
MS (ESI) m/z : 610 (M + H) ⁺.
¹H-NMR (CDCl₃) δ: 2.55-2.66 (1H, m), 2.80-2.87 (2H, m), 3.11-3.19 (3H, m), 3.44 (3H, s), 3.82-3.87 (1H, m), 3.85 (3H, s), 4.81-4.99 (2H, m), 5.35 (1H, s), 6.95-7.01 (2H, m), 7.14-7.20 (2H, m), 7.47-7.36 (2H, m).
Elemental analysis for: C₂₅H₂₃ClF₃N₇O₄S·0.25H₂O·0.25 n-hexane
Calculated: C, 50.04; H, 4.28; N, 15.41; S, 5.04.
Found: C, 49.92; H, 4.21; N, 15.09; S, 4.98.

### Example 836

3-(2-{2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl}-1H-1,2,3,4-tetrazol-5-yl)propanoic acid

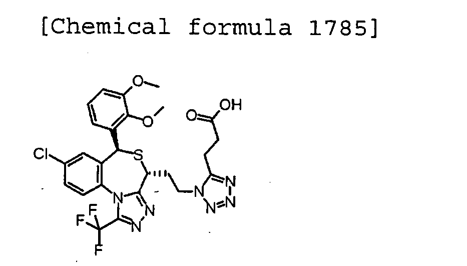

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (31 mg) was obtained from ethyl 3-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-1,2,3,4-tetrazol-5-yl)propanoate (32 mg) by using potassium carbonate (62 mg).
MS (ESI) m/z : 610 (M + H)⁺.
¹H-NMR (CDCl₃) δ: 2.54-2.64 (1H, m), 2.95-3.16 (5H, m), 3.43 (3H, s), 3.85 (3H, s), 3.90-3.95 (1H, m), 4.57-4.67 (1H, m), 4.73-4.83 (1H, m), 5.37 (1H, s), 6.95-7.01 (2H, m), 7.16-7.19 (2H, m), 7.47-7.36 (2H, m).
Elemental analysis for: C₂₅H₂₃ClF₃N₇O₄S·0.5H₂O·0.25 n-hexane Calculated: C, 49.69; H, 4.33; N, 15.31; S, 5.01.
Found: C, 49.78; H, 4.11; N, 15.12; S, 5.05.

### Example 837

Ethyl 2-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)acetate

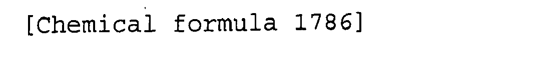

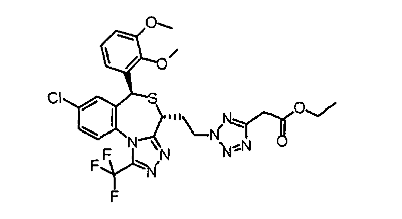

Ethyl 2-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-1,2,3,4-tetrazol-5-yl)acetate

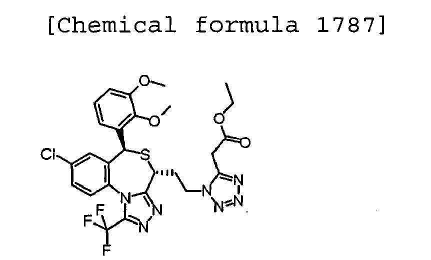

In a similar manner to that employed for the synthesis of ethyl 2-(2-{2-[(4R,6S)-8-chloro-6-(2,3-dimethoxyphenyl)-4H,6H-pyrrolo[1,2-a][4,1]benzoxazepin-4-yl]ethyl}-2H-1,2,3,4-tetrazol-5-yl)acetate, the title compounds, that is, 2H-isomer (46 mg) and 1H-isomer (22 mg) were obtained from 2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl methanesulfonate (116 mg) by using ethyl (tetrazol-5-yl)acetate (51 mg), potassium carbonate (171 mg) and tetrabutylammonium iodide (76 mg).
2H-isomer
MS (ESI) m/z : 624 (M + H)⁺.
¹H-NMR (CDCl₃) δ: 1.23-1.29 (3H, m), 2.59-2.70 (1H, m), 3.12-3.26 (1H, m), 3.44 (3H, s), 3.85 (3H, s), 3.89 (2H, s), 4.14-4.22 (2H, m), 4.86-4.94 (1H, m), 4.96-5.05 (1H, m), 5.36 (1H, s), 6.93-7.02 (2H, m), 7.21-7.14 (2H, m), 7.46-7.34 (2H, m).
1H-isomer
MS (ESI) m/z : 646 (M + Na)⁺.
¹H-NMR (CDCl₃) δ: 1.24-1.32 (3H, m), 2.73-2.86 (1H, m), 3.04-3.14 (2H, m), 3.42 (3H, s), 3.82-3.87 (2H, m), 3.85 (3H, s), 4.03-4.19 (4H, m), 4.47-4.61 (1H, m), 4.71-4.81 (1H, m), 5.38 (1H, s), 6.94-7.02 (2H, m), 7.15-7.21 (2H, m), 7.47-7.29 (2H, m).

### Example 838

2-(2-{2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)acetic acid

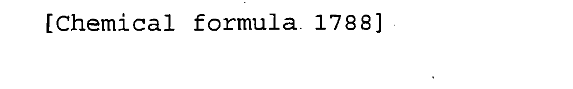

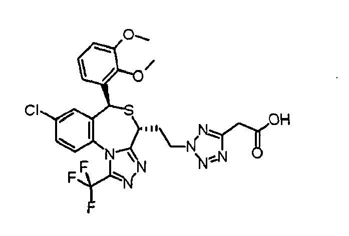

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (42 mg) was obtained from ethyl 2-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)acetate (44 mg) by using potassium carbonate (58 mg).
MS (ESI) m/z : 596 (M + H)⁺.
¹H-NMR (CDCl₃) δ: 2.60-2.70 (1H, m), 3.12-3.22 (1H, m), 3.44 (3H, s), 3.82-3.86 (1H, m), 3.85 (3H, s), 3.96 (2H, s), 4.86-5.05 (2H, m), 5.36 (1H, s), 6.95-7.01 (2H, m), 7.14-7.21 (2H, m), 7.34-7.39 (1H, m), 7.46-7.41 (1H, m). Elemental analysis for: C₂₄H₂₁ClF₃N₇O₄S·0.25H₂O·0.5CHCl₃ Calculated: C, 44.57; H, 3.36; N, 14.85.
Found: C, 44.93; H, 3.41; N, 14.42.

### Example 839

2-(2-{2-[(trans)-8-Chloro-6-(2,3-dimethoxyphenyl)-1-(trifluoromethyl)-4H,6H-[1,2,4]triazolo[4,3-a][4,1]benzothiazepin-4-yl]ethyl}-1H-1,2,3,4-tetrazol-5-yl)acetic acid

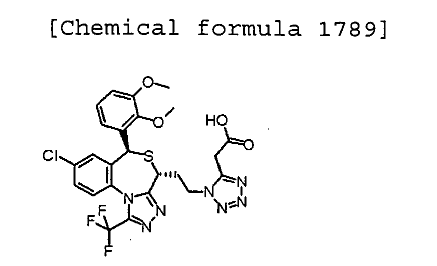

In a similar manner to that employed for the synthesis of 2-(2-{2-[(4R,6S)-8-chloro-6-(2,3-dimethoxyphenyl-4H,6H-pyrrolo[1,2-a][4,1]benzoxazepin-4-yl)ethyl]-2H-1,2,3,4-tetrazol-5-yl}acetic acid, the title compound (18 mg) was obtained from ethyl 2-(2-{2-[(trans)-8-chloro-6-(2,3-dimethoxyphenyl)-1-(trifluoromethyl)-4H,6H-[1,2,4]triazolo[4,3-a][4,1]benzothiazepin-4-yl]ethyl}-1H-1,2,3,4-tetrazol-5-yl)acetate (19 mg) by using potassium carbonate (25 mg).
MS (ESI) m/z : 596 (M + H)⁺.
¹H-NMR (CDCl₃) δ: 2.70-2.80 (1H, m), 2.98-3.09 (1H, m), 3.43 (3H, s), 3.85 (3H, s), 3.91-3.97 (1H, m), 4.08 (1H, d, J = 17.6 Hz), 4.21 (1H, d, J = 17.6 Hz), 4.51-4.62 (1H, m), 4.71-4.80 (1H, m), 5.38 (1H, s), 6.97-7.02 (2H, m), 7.16-7.22 (2H, m), 7.33-7.38 (1H, m), 7.45-7.40 (1H, m).

### Referential Example 772

4-Chloro-2-methoxyaniline

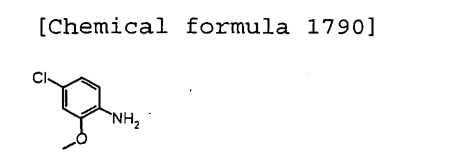

To an N,N-dimethylformamide solution (500 mL) of 4-chloro-2-nitroaniline (25.1 g) were added ferric trichloride hexahydrate (53.1 g) and distilled water (250 mL). The resulting mixture was ice cooled. To the reaction mixture was added zinc powder (77.8 g) in portions. After completion of the addition, stirring was continued for 10 minutes. The reaction mixture was filtered through Celite 545, followed by sufficient washing with ethyl acetate. The filtrate was concentrated under reduced pressure. The residue thus obtained was purified by column chromatography (hexane:ethyl acetate= from 5:1 to 3:1) to give the title compound (15.1 g).
¹H-NMR (CDCl₃) δ: 3.76 (2H, br s), 3.84 (3H, s), 6.63-6.59 (1H, m), 6.78-6.74 (2H, m).

### Referential Example 773

tert-Butyl 4-chloro-2-methoxyphenylcarbamate

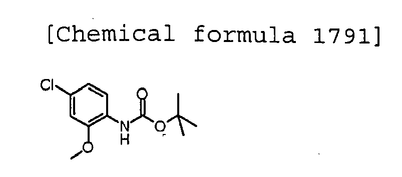

To a tert-butyl alcohol solution (140 mL) of 4-chloro-2-methoxyaniline (27.8 g) was added di-tert-butyl dicarbonate (42.4 g) at room temperature and the resulting mixture was stirred for 10 hours. To the reaction mixture was added di-tert-butyl dicarbonate (1.9 g) again, followed by stirring for 13.5 hours. The reaction mixture was concentrated under reduced pressure. The residue thus obtained was purified by column chromatography (hexane:ethyl acetate=5:1) to give the title compound (45.5 g).
¹H-NMR (CDCl₃) δ: 1.52 (9H, s), 3.86 (3H, s), 6.82 (1H, d, J = 2.2 Hz), 6.91 (1H, dd, J = 8.8, 2.2 Hz), 6.99 (1H, s), 8.05-7.95 (1H, m).

### Referential Example 774

tert-Butyl 4-chloro-2-[(2,3-dimethoxyphenyl)(hydroxy)methyl]-6-methoxyphenylcarbamate

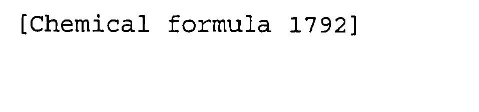

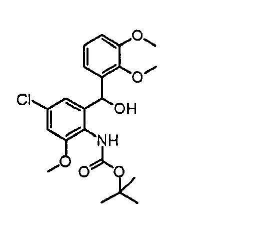

To a tetrahydrofuran solution (4.0 mL) of tert-butyl 4-chloro-2-methoxyphenylcarbamate (104 mg) was added dropwise sec-butyl lithium (a 1.00 mol/l cyclohexane-n-hexane solution, 0.85 mL) at -78°C. While warming to -30°C, the resulting mixture was stirred for 2.5 hours. The reaction mixture was cooled to -78°C and 2,3-dimethoxybenzaldehyde (74 mg) was added thereto. While warming to -10°C, stirring was conducted for one hour. To the reaction mixture were added a saturated aqueous solution of ammonium chloride and ethyl acetate and the layers separated. The organic layer thus obtained was dried over anhydrous sodium sulfate. The solvent was distilled off under reduced pressure and the residue thus obtained was purified by thin layer chromatography to give the title compound (62 mg).
¹H-NMR (CDCl₃) δ: 1.50 (9H, s), 3.44 (3H, s), 3.83 (3H, s), 3.83 (3H, s), 4.49 (1H, s), 6.13-6.17 (1H, m), 6.36 (1H, s), 6.75-6.82 (2H, m), 6.85-6.89 (1H, m), 7.10-7.16 (1H, m), 7.33 (1H, s).

### Referential Example 775

(2-Amino-5-chloro-3-methoxyphenyl)(2,3-dimethoxyphenyl)methanol

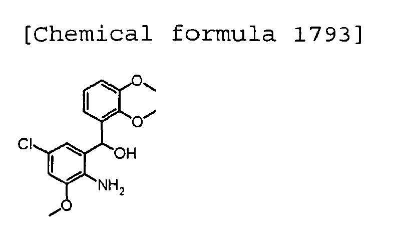

To a dioxane:methanol (0.6 mL:0.6 mL) mixed solution of tert-butyl 4-chloro-2-[(2,3-dimethoxyphenyl)(hydroxy)methyl]-6-methoxyphenylcarbamate (103 mg) was added potassium hydroxide (160 mg). The resulting mixture was stirred at 100°C for 15 hours. The solvent was distilled off under reduced pressure. To the residue thus obtained was added ethyl acetate and distilled water and the layers separated. The organic layer thus obtained was washed with saturated brine and dried over anhydrous sodium sulfate. The solvent was distilled off under reduced pressure to give the title compound (73 mg). MS (ESI) m/z : 306 (M - OH)⁺.
¹H-NMR (CDCl₃) δ: 3.06 (1H, br s), 3.83 (3H, s), 3.84 (3H, s), 3.88 (3H, s), 4.35 (2H, s), 6.08 (1H, s), 6.70-6.72 (1H, m), 6.76-6.78 (1H, m), 6.92-6.87 (2H, m), 7.09-7.03 (1H, m).

### Referential Example 776

2-[(trans)-7-Chloro-5-(2,3-dimethoxyphenyl)-9-methoxy-2-oxo-1,2,3,5-tetrahydro-4,1-benzothiazepin-3-yl]acetic acid

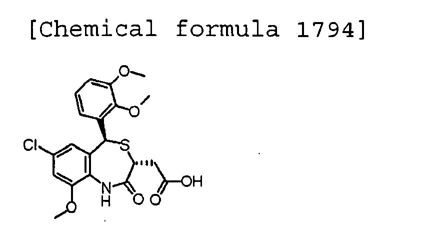

To a 1,4-dioxane solution (80.0 mL) of (2-amino-5-chloro-3-methoxyphenyl)(2,3-dimethoxyphenyl)methanol (2.71 g) was added a 4N hydrochloric acid-1,4-dioxane solution (20.0 mL). The resulting mixture was stirred at 110°C for 85 hours. After cooling to room temperature, the solvent was distilled off under reduced pressure. To the residue thus obtained was added diethyl ether-n-hexane. The solid thus obtained was filtered, followed by washing with diethyl ether and drying under reduced pressure to give the title compound (3.51 g).
MS (ESI) m/z : 438 (M + H)⁺.
¹H-NMR (DMSO-d6) δ: 2.31-2.41 (1H, m), 2.65-2.79 (1H, m), 3.46 (3H, s), 3.52-3.56 (1H, m), 3.83 (3H, s), 3.86 (3H, s), 5.98 (1H, s), 6.28 (1H, d, J = 2.2 Hz), 7.29-7.12 (5H, m), 9.51 (1H, s).

### Referential Example 777

Isopropyl 2-[(trans)-7-chloro-5-(2,3-dimethoxyphenyl)-9-methoxy-2-oxo-1,2,3,5-tetrahydro-4,1-benzothiazepin-3-yl] acetate

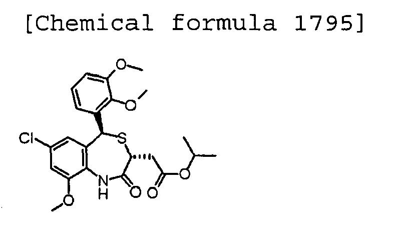

To an N,N-dimethylformamide solution (40 mL) of 2-[(trans)-7-chloro-5-(2,3-dimethoxyphenyl)-9-methoxy-2-oxo-1,2,3,5-tetrahydro-4,1-benzothiazepin-3-yl]acetic acid (3.51 g) were added potassium carbonate (3.32 g) and 2-iodopropane (1.50 g). The resulting mixture was stirred at room temperature for 14.5 hours. The solvent was distilled off under reduced pressure. To the residue thus obtained were added ethyl acetate and distilled water and the layers separated. The organic layer thus obtained was washed with distilled water and saturated brine, dried over anhydrous sodium sulfate and distilled under reduced pressure to remove the solvent. To the residue thus obtained was added diethyl ether to solidify it. The resulting solid was collected by filtration, washed with diethyl ether and dried under reduced pressure to give the title compound (2.50 g).
MS (ESI) m/z : 480 (M + H)⁺.
¹H-NMR (CDCl₃) δ: 1.19-1.23 (6H, m), 2.33-2.46 (1H, m), 3.01-3.10 (3H, m), 3.59 (3H, s), 3.77-3.85 (1H, m), 3.86 (3H, s), 3.87 (3H, s), 4.93-5.04 (1H, m), 6.19 (1H, s), 6.45 (1H, d, J = 2.0 Hz), 6.81 (1H, d, J = 2.0 Hz), 6.94-7.03 (2H, m), 7.14-7.19 (1H, m), 7.35-7.28 (1H, m).

### Referential Example 778

Isopropyl 2-[7-chloro-5-(2,3-dimethoxyphenyl)-9-methoxy-2-thioxo-1,2,3,5-tetrahydro-4,1-benzothiazepin-3-yl]acetate

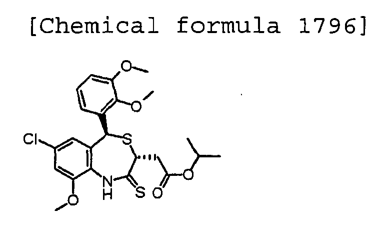

In a similar manner to that employed for the synthesis of ethyl 3-(2-{[(trans)-7-chloro-5-(2,3-dimethoxyphenyl)-2-thioxo-1,2,3,5-tetrahydro-4,1-benzothiazepin-3-yl]methyl}-1,3-thiazol-4-yl)propanoate, the title compound (2.21 g) was obtained from isopropyl 2-[7-chloro-5-(2,3-dimethoxyphenyl)-9-methoxy-2-oxo-1,2,3,5-tetrahydro-4,1-benzothiazepin-3-yl]acetate (2.50 g) by using Lawesson's reagent (2.53 g).
MS (ESI) m/z : 496 (M + H)⁺.
¹H-NMR (CDCl₃) δ: 1.19-1.28 (6H, m), 2.55-2.63 (2H, m), 3.31-3.40 (1H, m), 3.61 (3H, s), 3.87 (3H, s), 3.88 (3H, s), 4.05-4.16 (1H, m), 4.95-5.03 (1H, m), 6.00 (1H, s), 6.43 (1H, d, J = 2.2 Hz), 6.84 (1H, d, J = 2.2 Hz), 6.93-6.98 (1H, m), 7.13-7.18 (1H, m), 7.27-7.22 (2H, m), 8.90 (1H, s).

### Referential Example 779

Isopropyl2-[(trans)-7-chloro-5-(2,3-dimethoxyphenyl)-2-[(Z)hydrazono]-9-methoxy-1,5-dihydro-4,1-benzothiazepin-3(3H)-yl]acetate

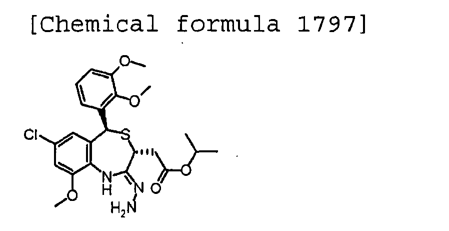

In a similar manner to that employed for the synthesis of ethyl 3-[2-({(trans)-7-chloro-5-(2,3-dimethoxyphenyl)-2-[hydrazono]-1,2,3,5-tetrahydro-4,1-benzothiazepin-3-yl}methyl)-1,3-thiazol-4-yl]propanoate, the title compound (2.19 g) was obtained from isopropyl 2-[7-chloro-5-(2,3-dimethoxyphenyl)-9-methoxy-2-thioxo-1,2,3,5-tetrahydro-4,1-benzothiazepin-3-yl]acetate (2.20 g) by using hydrazine monohydrate (0.43 mL).
MS (ESI) m/z : 494 (M + H)⁺.
¹H-NMR (CDCl₃) δ: 1.17-1.22 (6H, m), 2.42-2.50 (1H, m), 3.01-3.10 (1H, m), 3.51 (3H, s), 3.85 (3H, s), 3.86 (3H, s), 3.90-3.97 (1H, m), 4.92-5.05 (1H, m), 6.12 (1H, s), 6.41-6.43 (1H, m), 6.54 (1H, s), 6.75-6.78 (1H, m), 6.90-7.00 (1H, m), 7.12-7.21 (2H, m), 7.33-7.23 (1H, m).

### Example 840

Isopropyl 2-[(trans)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetate

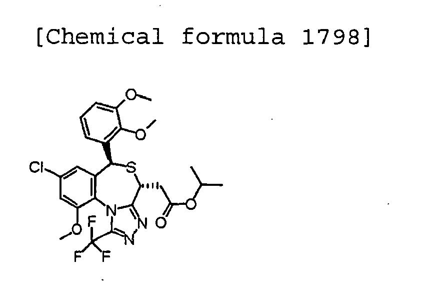

In a similar manner to that employed for the synthesis of ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-4-yl)propanoate, the title compound (1.88 g) was obtained from isopropyl 2-[7-chloro-5-(2,3-dimethoxyphenyl)-2-[(Z)hydrazono]-9-methoxy-1,5-dihydro-4,1-benzothiazepin-3(3H)-yl]acetate (2.19 g) by using trifluoroacetic anhydride (3.08 mL) and trifluoroacetic acid (6.59 mL).
MS (ESI) m/z : 572 (M + H)⁺.
¹H-NMR (CDCl₃) δ: 1.17-1.25 (6H, m), 2.87-2.94 (1H, m), 3.45 (3H, s), 3.53-3.61 (1H, m), 3.84 (3H, s), 3.85 (3H, s), 4.25-4.30 (1H, m), 4.96-5.04 (1H, m), 5.31 (1H, s), 6.53-6.55 (1H, m), 6.94-7.00 (2H, m), 7.22-7.10 (3H, m).

### Example 841

Isopropyl 2-[(4S,6R)-8-chloro-6-(2,3-dimethoxyphenyl)-10-methoxy-1-(trifluoromethyl)-4H,6H-[1,2,4]triazolo[4,3-a] [4,1]benzothiazepin-4-yl]acetate
Isomer A

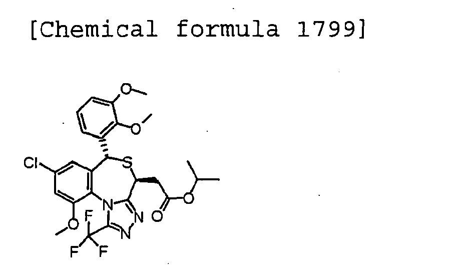

Isopropyl 2-[(4R,6S)-8-chloro-6-(2,3-dimethoxyphenyl)-10-methoxy-1-(trifluoromethyl)-4H,6H-[1,2,4]triazolo[4,3-a] [4,1]benzothiazepin-4-yl]acetate
Isomer B

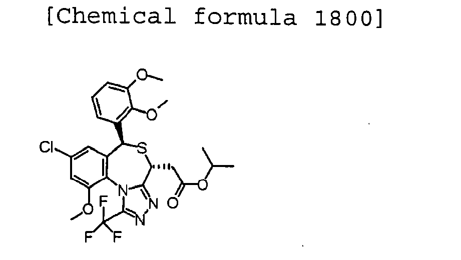

Isopropyl 2-[(trans)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetate (1888 mg) was optically resolved by HPLC equipped with CHIRALPAK AD (Daicel Chemical). The isomer A (425 mg) with a long retention time and the isomer B (443 mg) with a short retention time were obtained as the title compounds by separating at a flow rate of 10 mL/min while using 70% ethanol-hexane as a solvent.

### Example 842

2-[(4S,6R)-8-Chloro-6-(2,3-dimethoxyphenyl)-10-methoxy-1-(trifluoromethyl)-4H,6H-[1,2,4]triazolo[4,3-a] [4,1]benzothiazepin-4-yl]acetic acid

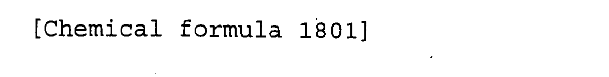

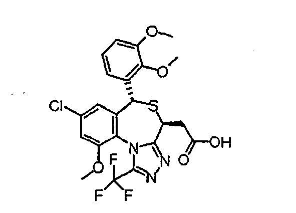

To a dioxane solution (20 mL) of isopropyl 2-[(4S,6R)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetate (411 mg) was added concentrated hydrochloric acid (4.0 mL). The resulting mixture was stirred at 80°C for 24 hours. To the reaction mixture was added concentrated hydrochloric acid (4.0 mL) again and the resulting mixture was stirred at 80°C for 6 hours. The solvent was distilled off under reduced pressure and the residue thus obtained was purified by thin layer chromatography to give the title compound (381 mg). MS (ESI) m/z : 530 (M + H)⁺.
¹H-NMR (CDCl₃) δ: 2.75-2.89 (1H, m), 3.40-3.43 (1H, m), 3.41 (3H, s), 3.81 (3H, s), 3.83 (3H, s), 4.21-4.31 (1H, m), 5.26 (1H, s), 6.48 (1H, s), 7.00-6.91 (2H, m), 7.19-7.06 (2H, m).
Elemental analysis for: C₂₂H₁₉ClF₃N₃O₅S·0.5H₂O·0.3CHCl₃
Calculated: C, 46.60; H, 3.56; N, 7.31.
Found: C, 46.71; H, 3.48; N, 7.20.

### Example 843

2-[(4R,6S)-8-Chloro-6-(2,3-dimethoxyphenyl)-10-methoxy-1-(trifluoromethyl)-4H,6H-[1,2,4]triazolo[4,3-a] [4,1]benzothiazepin-4-yl]acetic acid

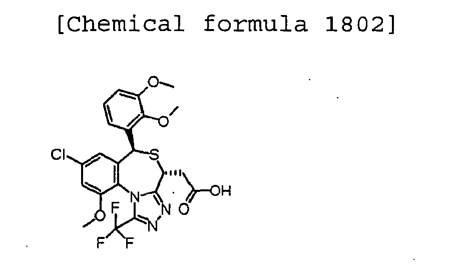

In a similar manner to that employed for the synthesis of 2-[(4S,6R)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetic acid, the title compound (396 mg) was obtained from isopropyl 2-[(4R,6S)-8-chloro-6-(2,3-dimethoxyphenyl)-10-methoxy-1-(trifluoromethyl)-4H,6H-[1,2,4]triazolo[4,3-a] [4,1] benzothiazepin-4-yl] acetate (427 mg) by using concentrated hydrochloric acid (8.0 mL).
MS (ESI) m/z : 530 (M + H)⁺.
¹H-NMR (CDCl₃) δ: 2.72-2.84 (1H, m), 3.27-3.38 (1H, m), 3.41 (3H, s), 3.80 (3H, s), 3.83 (3H, s), 4.22-4.29 (1H, m), 5.25 (1H, s), 6.45-6.49 (1H, m), 7.00-6.89 (2H, m), 7.18-7.06 (2H, m). Elemental analysis for: C₂₂H₁₉ClF₃N₃O₅S·1.0H₂O·0.3CHCl₃
Calculated: C, 45.88; H, 3.68; N, 7.20.
Found: C, 45.87; H, 3.41; N, 7.09.

### Example 844

Ethyl 2-(1-{2-[(4R,6S)-8-chloro-6-(2,3-dimethoxyphenyl)-10-methoxy-1-(trifluoromethyl)-4H,6H-[1,2,4]triazolo[4,3-a] [4,1]benzothiazepin-4-yl]acetyl}-4-piperidinyl)acetate

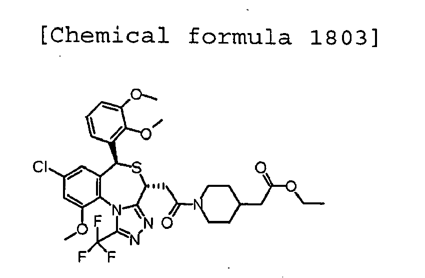

In a similar manner to that employed for the synthesis of ethyl 5-({2-[(trans)-7-chloro-5-(2,3-dimethoxyphenyl)-2-oxo-1,2,3,5-tetrahydro-4,1-benzothiazepin-3-yl]acetyl}amino)-4-oxohexanoate, the title compound (116 mg) was obtained from 2-[(4R,6S)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetic acid (127 mg) by using ethyl 2-(4-piperidinyl)acetate (53 mg), 1-ethyl-3-(3-dimethylaminopropyl)carbodiimide hydrochloride (69 mg), and 1-hydroxybenzotriazole monohydrate (18 mg).
MS (ESI) m/z : 683 (M + H)⁺.
¹H-NMR (CDCl₃) δ: 1.07-1.43 (5H, m), 1.69-1.88 (2H, m), 1.96-2.11 (1H, m), 2.20-2.31 (2H, m), 2.55-2.68 (1H, m), 2.89-2.99 (1H, m), 3.03-3.19 (1H, m), 3.44 (3H, s), 3.55-3.68 (1H, m), 3.83-3.86 (6H, m), 3.95-4.05 (1H, m), 4.10-4.17 (2H, m), 4.44-4.59 (2H, m), 5.29 (1H, s), 6.52-6.55 (1H, m), 6.93-6.98 (2H, m), 7.10-7.18 (1H, m), 7.23-7.19 (1H, m).

### Example 845

2-(1-{2-[(4R,6S)-8-Chloro-6-(2,3-dimethoxyphenyl)-10-methoxy-1-(trifluoromethyl)-4H,6H-[1,2,4]triazolo[4,3-a] [4,1]benzothiazepin-4-yl]acetyl}-4-piperidinyl)acetic acid

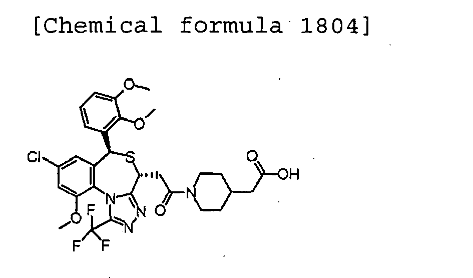

In a similar manner to that employed for the synthesis of ethyl 3-(2-{[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-4-methyl-1,3-thiazol-5-yl)propanoic acid, the title compound (81 mg) was obtained from ethyl 2-(1-{2-[(4R,6S)-8-chloro-6-(2,3-dimethoxyphenyl)-10-methoxy-1-(trifluoromethyl)-4H,6H-[1,2,4]triazolo[4,3-a][4,1]benzothiazepin-4-yl]acetyl}-4-piperidinyl)acetate (112 mg) by using potassium carbonate (409 mg).
MS (ESI) m/z : 655 (M + H)⁺.
¹H-NMR (CDCl₃) δ: 1.09-1.48 (2H, m), 1.72-1.92 (2H, m), 1.98-2.11 (1H, m), 2.24-2.36 (2H, m), 2.54-2.68 (1H, m), 2.87-3.01 (1H, m), 3.02-3.21 (1H, m), 3.44 (3H, s), 3.55-3.68 (1H, m), 3.83-3.85 (6H, m), 3.93-4.07 (1H, m), 4.43-4.60 (2H, m), 5.29 (1H, s), 6.53 (1H, d, J = 2.0 Hz), 6.93-6.98 (2H, m), 7.10-7.17 (1H, m), 7.24-7..18 (1H, m). Elemental analysis for: C₂₉H₃₀ClF₃N₄O₆S·1.0H₂O·0.25 n-hexane Calculated: C, 52.74; H, 5.15; N, 8.07.
Found: C, 52.49; H, 4.87; N, 7.739.

### Referential Example 780

Ethyl 4-({2-[3,5-trans-7-chloro-5-(2,3-dimethoxyphenyl)-2-oxo-1,2,3,5-tetrahydro-4,1-benzothiazepin-3-yl]acetyl}amino)-3-oxobutanoate

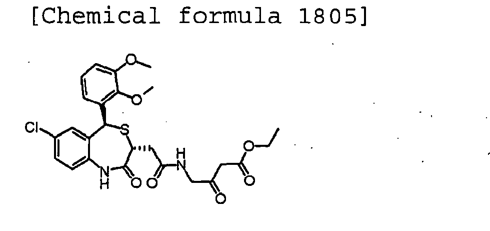

In a similar manner to that employed for the synthesis of methyl 5-({2-[(trans)-7-chloro-5-(2,3-dimethoxyphenyl)-2-oxo-1,2,3,5-tetrahydro-4,1-benzoxazepin-3-yl]acetyl}amino)-4-oxopentanoate, the title compound (1163 mg) was obtained using 2-(3,5-trans-7-chloro-5-(2,3-dimethoxyphenyl)-2-oxo-1,2,3,5-tetrahydro-4,1-benzothiazepin-3-yl)acetic acid (1224 mg) and amino-3-oxobutanoate (545 mg)
MS (ESI) m/z : 535 (M + H)⁺.
¹H-NMR (CDCl₃) δ: 1.28 (3H, t, J = 7.1 Hz), 2.40 (1H, dd, J = 15.0, 3.8 Hz), 2.99 (1H, dd, J = 15.0, 10.1 Hz), 3.48 (2H, d, J = 2.2 Hz), 3.59 (3H, s), 3.86-3.92 (4H, m), 4.17-4.23 (4H, m), 6.19 (1H, s), 6.48-6.53 (1H, m), 6.84 (1H, d, J = 2.2 Hz), 6.97 (1H, d, J = 8.4 Hz), 7.08 (1H, d, J = 8.4 Hz), 7.19 (1H, t, J = 8.1 Hz), 7.23 (1H, dd, J = 8.4, 2.2 Hz), 7.32 (1H, d, J = 7.6 Hz).

### Referential Example 781

Ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)acetate

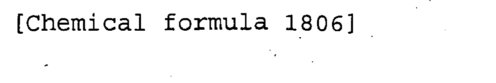

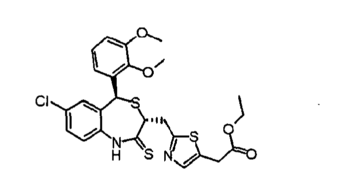

In a similar manner to that employed for the synthesis of methyl 3-({2-[(trans)-7-chloro-5-(2,3-dimethoxyphenyl)-2-thioxo-1,2,3,5-tetrahydro-4,1-benzoxazepin-3-yl]methyl}-1,3-thiazol-5-yl)propanoate, the title compound (598 mg) was obtained using ethyl 4-({2-[3,5-trans-7-chloro-5-(2,3-dimethoxyphenyl)-2-oxo-1,2,3,5-tetrahydro-4,1-benzothiazepin-3-yl]acetyl}amino)-3-oxobutanoate (1163 mg).
MS (ESI) m/z : 549 (M + H)⁺.
¹H-NMR (CDCl₃) δ: 1.28 (3H, t, J = 7.1 Hz), 3.39 (1H, dd, J = 15.2, 5.4 Hz), 3.60 (3H, s), 3.78 (2H, s), 3.87 (3H, s), 3.99 (1H, dd, J = 15.2, 8.1 Hz), 4.16-4.23 (3H, m), 6.04 (1H, s), 6.81 (1H, d, J = 2.2 Hz), 6.97 (1H, dd, J = 8.0, 1.5 Hz), 7.02 (1H, d, J = 8.3 Hz), 7.16 (1H, t, J = 8.0 Hz), 7.22 (2H, dd, J = 8.3, 2.0 Hz), 7.51 (1H, s).

### Example 846

Ethyl 2-(2-{[4,6-trans-8-chloro-1-cyclopropyl-6-(2,3-dimethoxyphenyl)-4H,6H-[1,2,4]triazolo[4,3-a][4,1]benzothiazepin-4-yl]methyl}-1,3-thiazol-5-yl)acetate

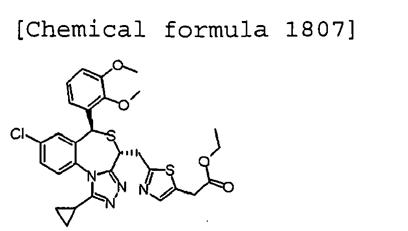

In a similar manner to that employed for the synthesis of ethyl 2-[(trans)-8-chloro-1-cyclopropyl-6-(2,3-dimethoxyphenyl)-4H,6H-[1,2,4]triazolo[4,3-a][4,1]benzoxazepin-4-yl]acetate, the title compound (42 mg) was obtained using ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)acetate (110 mg).
MS (ESI) m/z : 597 (M + H)⁺.
¹H-NMR (CDCl₃) δ: 0.91-0.99 (1H, m), 1.05-1.13 (1H, m), 1.21-1.33 (5H, m), 1.74-1.81 (1H, m), 3.67 (1H, dd, J = 15.4, 7.8 Hz), 3.76-3.78 (3H, m), 3.84 (3H, s), 4.08-4.21 (5H, m), 4.47 (1H, dd, J = 7.8, 6.6 Hz), 5.38 (1H, s), 6.93-6.97 (2H, m), 7.14 (1H, t, J = 7.9 Hz), 7.21 (1H, dd, J = 7.9, 1.5 Hz), 7.33-7.38 (2H, m), 7.47 (1H, s).

### Example 847

Ethyl 2-(2-{[4,6-trans-8-chloro-6-(2,3-dimethoxyphenyl)-1-phenyl-4H,6H-[1,2,4]triazolo[4,3-a][4,1]benzothiazepin-4-yl]methyl}-1,3-thiazol-5-yl)acetate

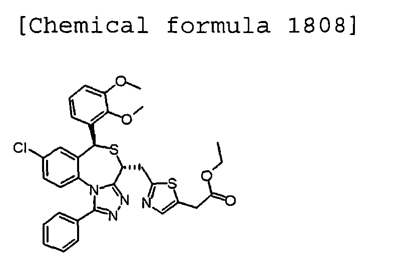

In a similar manner to that employed for the synthesis of ethyl 2-[(trans)-8-chloro-1-cyclopropyl-6-(2,3-dimethoxyphenyl)-4H,6H-[1,2,4]triazolo[4,3-a][4,1]benzoxazepin-4-yl]acetate, the title compound (36 mg) was obtained using ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)acetate (110 mg).
MS (ESI) m/z : 633 (M + H)⁺.
¹H-NMR (CDCl₃) δ: 1.29 (3H, t, J = 7.1 Hz), 3.59 (3H, s), 3.74 (1H, dd, J = 15.4, 7.8 Hz), 3.79-3.80 (2H, m), 3.88 (3H, s), 4.17-4.24 (3H, m), 4.48 (1H, dd, J = 7.8, 6.6 Hz), 5.63 (1H, s), 6.80 (1H, d, J = 8.3 Hz), 6.91 (1H, d, J = 2.4 Hz), 6.99 (1H, dd, J = 8.3, 1.5 Hz), 7.14-7.19 (2H, m), 7.26-7.30 (1H, m), 7.35-7.45 (3H, m), 7.49 (1H, s), 7.54-7.58 (2H, m).

### Example 848

Ethyl 2-(2-{[4,6-trans-8-chloro-1-(tert-butyl)-6-(2,3-dimethoxyphenyl)-4H,6H-[1,2,4]triazolo[4,3-a][4,1]benzothiazepin-4-yl]methyl}-1,3-thiazol-5-yl)acetate

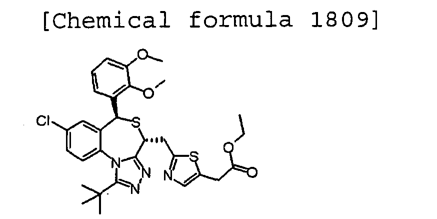

In a similar manner to that employed for the synthesis of ethyl 2-[(trans)-8-chloro-1-cyclopropyl-6-(2,3-dimethoxyphenyl)-4H,6H-[1,2,4]triazolo[4,3-a][4,1]benzoxazepin-4-yl]acetate, the title compound (48 mg) was obtained using ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)acetate (110 mg).
MS (ESI) m/z : 613 (M + H)⁺.
¹H-NMR (CDCl₃) δ: 1.27 (3H, t, J = 6.8 Hz), 1.27 (3H, s), 1.42 (9H, s), 3.58 (3H, s), 3.63 (1H, dd, J = 15.4, 7.3 Hz), 3.77 (2H, s), 3.86 (3H, s), 4.07-4.28 (4H, m), 5.34 (1H, s), 6.93-6.97 (2H, m), 7.14 (1H, t, J = 9.0 Hz), 7.23-7.26 (1H, m), 7.33-7.38 (2H, m), 7.46 (1H, s).

### Example 849

Ethyl 2-(2-{[4,6-trans-8-chloro-6-(2,3-dimethoxyphenyl)-1-phenyl-4H,6H-[1,2,4]triazolo[4,3-a][4,1]benzothiazepin-4-yl]methyl}-1,3-thiazol-5-yl)acetate

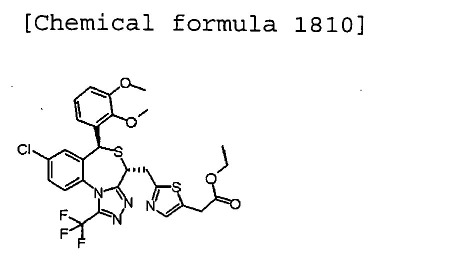

In a similar manner to that employed for the synthesis of methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate, the title compound (26 mg) was obtained using ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)acetate (110 mg).
MS (ESI) m/z : 625 (M + H)⁺.
¹H-NMR (CDCl₃) δ: 1.28 (3H, t, J = 7.2 Hz), 3.43 (3H, s), 3.68 (1H, dd, J = 15.5, 7.2 Hz), 3.78 (2H, s), 3.84 (3H, s), 4.15 (1H, dd, J = 15.5, 7.2 Hz), 4.19 (3H, q, J = 7.2 Hz), 4.48 (1H, t, J = 7.2 Hz), 5.35 (1H, s), 6.95-6.98 (2H, m), 7.11-7.20 (2H, m), 7.34 (1H, d, J = 8.4 Hz), 7.41 (1H, dd, J = 8.4, 2.2 Hz), 7.46 (1H, s).

### Example 850

2-(2-{[4,6-trans-8-Chloro-1-cyclopropyl-6-(2,3-dimethoxyphenyl)-4H,6H-[1,2,4]triazolo[4,3-a][4,1]benzothiazepin-4-yl]methyl}-1,3-thiazol-5-yl)acetic acid

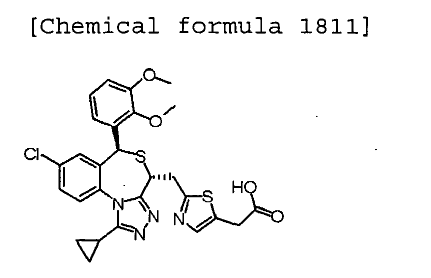

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]aceticacid, the title compound (25 mg) was obtained using ethyl 2-(2-{[4,6-trans-8-chloro-1-cyclopropyl-6-(2,3-dimethoxyphenyl)-4H,6H-[1,2,4]triazolo[4,3-a] [4,1]benzothiazepin-4-yl]methyl}-1,3-thiazol-5-yl)acetate (42 mg).
MS (ESI) m/z : 569 (M + H)⁺.
¹H-NMR (CDCl₃) δ: 0.86-1.34 (4H, m), 1.72-1.79 (1H, m), 3.45-3.56 (4H, m), 3.68-3.76 (2H, m), 3.84 (3H, s), 4.07 (1H, dd, J = 15.2, 7.8 Hz), 4.42-4.48 (1H, m), 5.34 (1H, s), 6.94-7.00 (2H, m), 7.14 (1H, t, J = 8.0 Hz), 7.21 (1H, d, J = 7.4 Hz), 7.32-7.40 (3H, m).
Elemental analysis for: C₂₇H₂₅ClN₄O₄S₂·2.5H₂O·0.25CHCl₃
Calculated: C, 50.80; H, 4.77; N, 8.70.
Found: C, 50.98; H, 4.47; N, 8.46.

### Example 851

2-(2-{[4,6-trans-8-Chloro-6-(2,3-dimethoxyphenyl)-1-phenyl-4H,6H-[1,2,4]triazolo[4,3-a][4,1]benzothiazepin-4-yl]methyl}-1,3-thiazol-5-yl)acetic acid

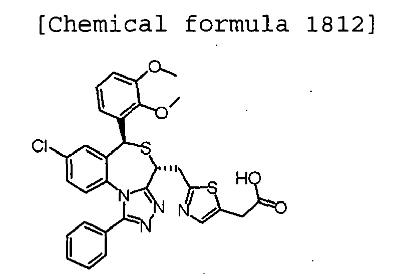

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (17 mg) was obtained using ethyl 2-(2-{[4,6-trans-8-chloro-6-(2,3-dimethoxyphenyl)-1-phenyl-4H,6H-[1,2,4]triazolo[4,3-a][4,1]benzothiazepin-4-yl]methyl}-1,3-thiazol-5-yl)acetate (36 mg) .
MS (ESI) m/z : 605 (M + H)⁺.
¹H-NMR (CDCl₃) δ: 3.57-3.77 (6H, m), 3.89 (3H, s), 4.16 (1H, dd, J = 14.5, 7.1 Hz), 4.45-4.53 (1H, m), 5.62 (1H, s), 6.81 (1H, d, J = 8.6 Hz), 6.92 (1H, d, J = 2.0 Hz), 7.01 (1H, d, J = 8.3 Hz), 7.15-7.22 (2H, m), 7.26-7.29 (1H, m), 7.36-7.46 (4H, m), 7.50-7.56 (2H, m).
Elemental analysis for: C₃₀H₂₅ClN₄O₄S₂·2.25H₂O·0.25CHCl₃ Calculated: C, 53.77; H, 4.47; N, 8.29.
Found: C, 53.82; H, 4.22; N, 7.90

### Example 852

2-(2-{[4,6-trans-8-Chloro-1-(tert-butyl)-6-(2,3-dimethoxyphenyl)-4H,6H-[1,2,4]triazolo[4,3-a][4,1]benzothiazepin-4-yl]methyl}-1,3-thiazol-5-yl)acetic acid

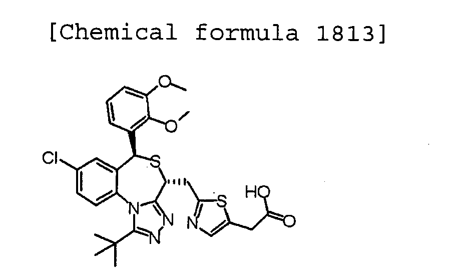

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (10 mg) was obtained using ethyl 2-(2-{[4,6-trans-8-chloro-1-(tert-butyl)-6-(2,3-dimethoxyphenyl)-4H,6H-[1,2,4]triazolo[4,3-a][4,1]benzothiazepin-4-yl]methyl}-1,3-thiazol-5-yl)acetate (48 mg).
MS (ESI) m/z : 597 (M + H)⁺.
¹H-NMR (CDCl₃) δ: 1.39 (9H, s), 3.48-3.61 (4H, m), 3.74-3.81 (2H, m), 3.86 (3H, s), 4.04-4.13 (1H, m), 4.24-4.30 (1H, m), 5.32 (1H, s), 6.92-6.99 (2H, m), 7.15 (1H, t, J = 8.0 Hz), 7.24-7.26 (1H, m), 7.32-7.44 (3H, m).
Elemental analysis for: C₂₈H₂₉ClN₄O₄S₂·2.5H₂O·0.5CHCl₃ Calculated: C, 49.58; H, 5.11; N, 8.12.
Found: C, 49.25; H, 4.94; N, 7.99.

### Example 853

2-(2-{[4,6-trans-8-Chloro-6-(2,3-dimethoxyphenyl)-1-phenyl-4H,6H-[1,2,4]triazolo[4,3-a][4,1]benzothiazepin-4-yl]methyl}-1,3-thiazol-5-yl)acetic acid

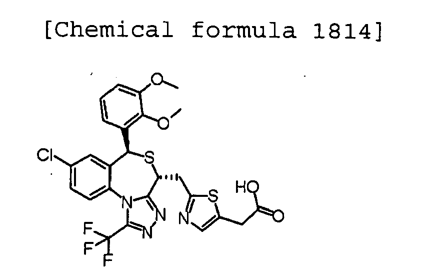

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (19 mg) was obtained using ethyl 2-(2-{[4,6-trans-8-chloro-6-(2,3-dimethoxyphenyl)-1-phenyl-4H,6H-[1,2,4]triazolo[4,3-a][4,1]benzothiazepin-4-yl]methyl}-1,3-thiazol-5-yl)acetate (26 mg).
MS (ESI) m/z : 597 (M + H)⁺.
¹H-NMR (CDCl₃) δ: 3.41 (3H, s), 3.55-3.63 (1H, m), 3.71-3.75 (2H, m), 3.84 (3H, s), 4.05-4.13 (1H, m), 4.45-4.50 (1H, m), 5.33 (1H, s), 6.93-6.97 (2H, m), 7.10-7.19 (2H, m), 7.32-7.41 (3H, m).
Elemental analysis for: C₂₅H₂₀ClF₃N₄O₄S₂·1.5H₂O·0.25CHCl₃ Calculated: C, 46.36; H, 3.62; N, 8.56.
Found: C, 46.27; H, 3.52; N, 8.33.

### Example 854

Ethyl 2-(1-{3-[4,6-trans-8-chloro-6-(2,3-dimethoxyphenyl)-1-(trifluoromethyl)-4H,6H-[1,2,4]triazolo[4,3-a][4,1]benzothiazepin-4-yl]propanoyl}-4-piperidinyl)acetate

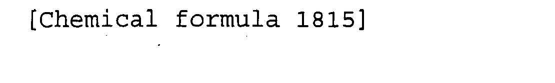

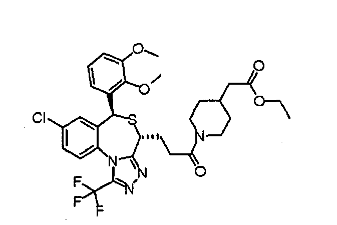

In a similar manner to that employed for the synthesis of ethyl 2-(1-{2-[8-chloro-6-(2,3-dimethoxyphenyl)-1-(trifluoromethyl)-4H,6H-[1,2,4]triazolo[4,3-a] [4,1]benzothiazepin-4-yl]acetyl}-4-piperidinyl) acetate, the title compound (32 mg) was obtained using 3-[(trans)-8-chloro-6-(2,3-dimethoxyphenyl)-1-(trifluoromethyl)-4H,6H,-[1,2,4]triazolo[4,3-a] [4,1]benzothiazepin-4-yl]propanoic acid (38 mg). MS (ESI) m/z : 653 (M + H)⁺.
¹H-NMR (CDCl₃) δ: 1.08-1.46 (6H, m), 1.62-1.90 (4H, m), 2.00-2.09 (1H, m), 2.22-2.28 (2H, m), 2.56-2.65 (1H, m), 2.78-2.92 (1H, m), 3.05-3.24 (1H, m), 3.29-3.44 (3H, m), 3.62-4.06 (5H, m), 4.11-4.17 (2H, m), 4.46-4.53 (2H, m), 5.32-5.41 (1H, m), 6.96-6.99 (1H, m), 7.14-7.25 (2H, m), 7.40-7.79 (2H, m).

### Example 855

2-(1-{3-[4,6-trans-8-Chloro-6-(2,3-dimethoxyphenyl)-1-(trifluoromethyl)-4H,6H-[1,2,4]triazolo[4,3-a][4,1]benzothiazepin-4-yl]propanoyl}-4-piperidinyl)acetic acid

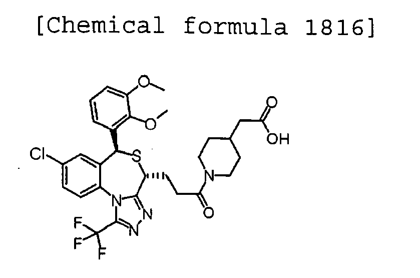

In a similar manner to that employed for the synthesis of 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetic acid, the title compound (10 mg) was obtained using ethyl 2-(1-{3-[4,6-trans-8-chloro-6-(2,3-dimethoxyphenyl)-1-(trifluoromethyl)-4H,6H-[1,2,4]triazolo[4,3-a][4,1]benzothiazepin-4-yl]propanoyl}-4-piperidinyl)acetate (9 mg).
MS (ESI) m/z : 639 (M + H)⁺.
¹H-NMR (CDCl₃) δ: 1.07-1.24 (2H, m), 1.72-2.08 (3H, m), 2.25-3.12 (8H, m), 3.29-3.44 (3H, m), 3.75-3.87 (3H, m), 3.91-4.07 (1H, m), 4.49-4.6.8 (1H, m), 5.31-5.37 (1H, m), 6.60-6.85 (1H, m), 6.95-7.01 (1H, m), 7.15-7.24 (1H, m), 7.32-7.78 (2H, m).
Elemental analysis for: C₂₉H₃₀ClF₃N₄O₅S·1.5 1,4-dioxane
Calculated: C, 54.51; H, 5.49; N, 7.26.
Found: C, 54.21; H, 5.30; N, 7.06.

### Referential Example 782

Methyl 4-(2-{2-[3,5-trans-7-chloro-5-(2,3-dimethoxyphenyl)-2-oxo-1,2,3,5-tetrahydro-4,1-benzothiazepin-3-yl]acetyl}hydrazine)-4-oxobutanoate

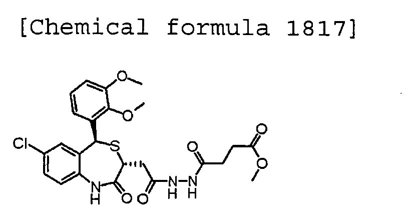

2-[(trans)-7-Chloro-5-(2,3-dimethoxyphenyl)-2-oxo-1,2,3,5-tetrahydro-4,1-benzothiazepin-3-yl]acetic acid (844 mg, 2.00 mmol) was dissolved in dichloromethane (5 mL). To the resulting solution were added 1-ethyl-3-(3-dimethylaminopropyl)carbodiimide hydrochloride (767 mg) and 1-hydroxybenzotriazole (306 mg). The resulting mixture was stirred at room temperature for 0.5 hour. To the reaction mixture was added hydrazine monohydrate (207 µl) and the resulting mixture was stirred at room temperature for one hour. The reaction mixture was diluted with chloroform and washed with a saturated aqueous solution of sodium bicarbonate and saturated brine. The organic layer was dried over anhydrous sodium sulfate. The filtrate was then concentrated. After addition of dichloromethane (50 mL) and sodium hydrogen carbonate (336 mg) to the residue, methyl 4-chloro-4-oxobutyrate (479 µl) was added at room temperature. After stirring at room temperature for 16 hours, the reaction mixture was diluted with ethyl acetate and washed with a saturated aqueous solution of sodium bicarbonate and saturated brine. The organic layer was dried over anhydrous sodium sulfate. The filtrate was then concentrated and the residue was purified by silica gel column (methanol:chloroform=1:10) to give a mixture of the title compound (457 mg).
MS (ESI) m/z : 536 (M + H)⁺.

### Referential Example 783

Methyl 3-(5-{[3,5-trans-7-chloro-5-(2,3-dimethoxyphenyl)-2-oxo-1,2,3,5-tetrahydro-4,1-benzothiazepin-3-yl]methyl}-1,3,4-thiadiazole-2-yl)propanoate

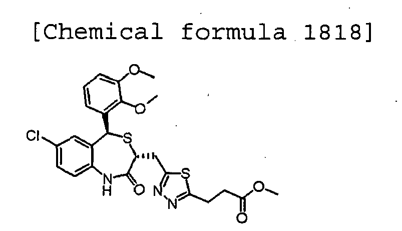

In a similar manner to that employed for the synthesis of methyl 3-({2-[(trans)-7-chloro-5-(2,3-dimethoxyphenyl)-2-oxo-1,2,3,5-tetrahydro-4,1-benzothiazepin-3-yl]methyl}-1,3-thiazol-5-yl)propanoate, the title compound (234 mg) was obtained using methyl 4-(2-{2-[3,5-trans-7-chloro-5-(2,3-dimethoxyphenyl)-2-oxo-1,2,3,5-tetrahydro-4,1-benzothiazepin-3-yl]acetyl}hydrazine)-4-oxobutanoate (457 mg) .
MS (ESI) m/z : 534 (M + H)⁺.
¹H-NMR (CDCl₃) δ: 2.87 (2H, t, J = 7.2 Hz), 3.23 (1H, dd, J = 15.4, 4.7 Hz), 3.34 (2H, t, J = 7.2 Hz), 3.59 (3H, s), 3.70 (3H, s), 3.75 (1H, dd, J = 15.4, 9.0 Hz), 3.87 (3H, s), 4.00 (1H, dd, J = 9.0, 4.7 Hz), 6.23 (1H, s), 6.84 (1H, d, J = 2.4 Hz), 6.97 (1H, dd, J = 8.3; 1.5 Hz), 7.05 (1H, d, J = 8.3 Hz), 7.16-7.24 (2H, m), 7.31 (1H, dd, J = 7.9, 1.3 Hz), 8.00 (1H, s).

### Referential Example 784

Methyl 3-(5-{[3,5-trans-7-chloro-5-(2,3-dimethoxyphenyl)-2-thioxo-1,2,3,5-tetrahydro-4,1-benzothiazepin-3-yl]methyl}-1,3,4-thiadiazole-2-yl)propanoate

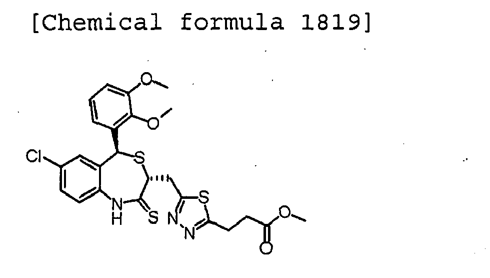

In a similar manner to that employed for the synthesis of methyl 2-[(trans)-7-chloro-5-(2,3-dimethoxyphenyl)-2-thioxo-1,2,3,5-tetrahydro-4,1-benzothiazepin-3-yl]acetate, the title compound (186 mg) was obtained using methyl 3-(5-{[3,5-trans-7-chloro-5-(2,3-dimethoxyphenyl)-2-oxo-1,2,3,5-tetrahydro-4,1-benzothiazepin-3-yl]methyl}-1,3,4-thiadiazole-2-yl)propanoate (234 mg).
MS (ESI) m/z : 550 (M + H)⁺.
¹H-NMR (CDCl₃) δ: 2.87 (2H, t, J = 7.3 Hz), 3.35 (2H, t, J = 7.3 Hz), 3.41 (1H, dd, J = 15.1, 4.6 Hz), 3.60 (3H, s), 3.70 (3H, s), 3.88 (3H, s), 4.05 (1H, dd, J = 15.1, 8.9 Hz), 4.21 (1H, dd, J = 8.9, 4.6 Hz), 6.06 (1H, s), 6.83 (1H, d, J = 2.2 Hz), 6.98 (1H, dd, J = 8.3, 1.2 Hz), 7.05 (1H, d, J = 8.3 Hz), 7.18 (1H, t, J = 7.9 Hz), 7.22-7.26 (2H, m), 9.40 (1H, br s).

### Example 856

Methyl 3-(5-{[(4,6-trans-8-chloro-6-(2,3-dimethoxyphenyl)-1-(trifluoromethyl)-4H,6H-[1,2,4]triazolo[4,3-a][4,1]benzothiazepin-4-yl)methyl]-1,3,4-thiadiazole-2-yl}propanoate

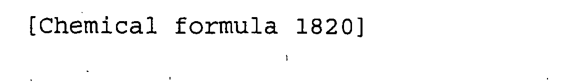

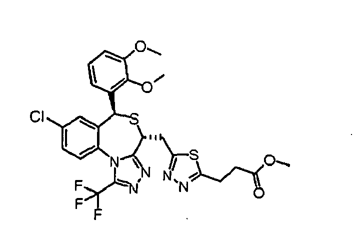

In a similar manner to that employed for the synthesis of methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate, the title compound (128 mg) was obtained using methyl 3-(5-{[3,5-trans-7-chloro-5-(2,3-dimethoxyphenyl)-2-thioxo-1,2,3,5-tetrahydro-4,1-benzothiazepin-3-yl]methyl}-1,3,4-thiadiazole-2-yl)propanoate (186 mg).
MS (ESI) m/z : 626 (M + H)⁺.
¹H-NMR (CDCl₃) δ: 2.87 (2H, t, J = 7.2 Hz), 3.36 (2H, t, J = 7.2 Hz), 3.43 (3H, s), 3.67 (3H, dd, J = 15.6, 5.6 Hz), 3.70 (3H, s), 3.85 (3H, s), 4.25 (1H, dd, J = 15.6, 8.6 Hz), 4.54 (1H, dd, J = 8.6, 5.6 Hz), 6.97-6.99 (2H, m), 7.15-7.19 (2H, m), 7.36 (1H, dd, J = 8.6, 0.7 Hz), 7.43 (1H, dd, J = 8.6, 2.2 Hz).

### Example 857

3-(5-{[4,6-trans-8-Chloro-6-(2,3-dimethoxyphenyl)-1-(trifluoromethyl)-4H,6H-[1,2,4]triazolo[4,3-a][4,1]benzothiazepin-4-yl]methyl}-1,3,4-thiadiazole-2-yl)propanoic acid

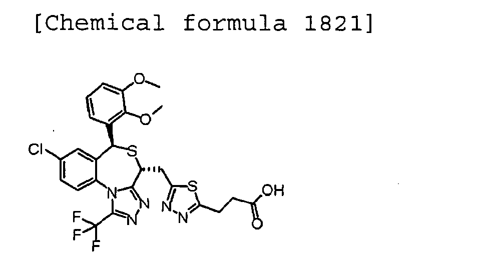

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (51 mg) was obtained using methyl 3-(5-{[(4,6-trans-8-chloro-6-(2,3-dimethoxyphenyl)-1-(trifluoromethyl)-4H,6H-[1,2,4]triazolo[4,3-a][4,1]benzothiazepin-4-yl)methyl]-1,3,4-thiadiazole-2-yl}propanoate (128 mg).
MS (ESI) m/z : 612 (M + H)⁺.
¹H-NMR- (CDCl₃) δ: 2.34-2.51 (1H, m), 3.00-3.17 (1H, m), 3.38 (3H, s), 3.52-3.62 (1H, m), 3.82 (3H, s), 4.01-4.15 (1H, m), 4.40-4.48 (1H, m), 5.32 (1H, s), 6.89-6.96 (2H, m), 7.07-7.17 (2H, m), 7.32-7.45 (2H, m).
Elemental analysis for: C₂₅H₂₁ClF₃N₅O₄S₂·1.5H₂O·0.5CHCl₃
Calculated: C, 43.80; H, 3.60; N, 10.02.
Found: C, 43.69; H, 3.86; N, 9.88.

### Example 858

Methyl 3-(2-{[4,6-trans-8-chloro-1-cyclohexyl-6-(2,3-dimethoxyphenyl)-4H,6H-[1,2,4]triazolo[4,3-a] [4,1]benzothiazepin-4-yl]methyl}-1,3-thiazol-5-yl)propanoate

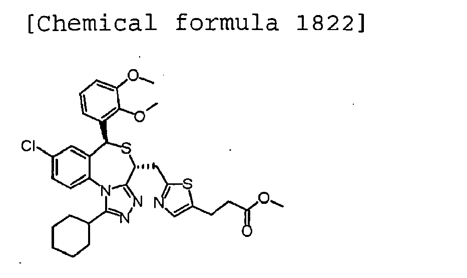

In a similar manner to that employed for the synthesis of ethyl 2-[(trans)-8-chloro-1-cyclopropyl-6-(2,3-dimethoxyphenyl)-4H,6H-[1,2,4]triazolo[4,3-a][4,1]benzoxazepin-4-yl]acetate, the title compound (112 mg) was obtained using ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)acetate (110 mg).
MS (ESI) m/z : 639 (M + H)⁺.
¹H-NMR (CDCl₃) δ: 1.09-2.21 (10H, m), 2.65 (2H, t, J = 7.4 Hz), 2.70-2.79 (1H, m), 3.10 (2H, t, J = 7.4 Hz), 3.50 (3H, s), 3.66 (1H, dd, J = 15.4, 8.1 Hz), 3.69 (3H, s), 3.85 (3H, s), 4.08 (1H, t, J = 7.8 Hz), 4.40 (1H, dd, J = 7.8, 6.6 Hz), 5.36 (1H, s), 6.94 (1H, d, J = 2.2 Hz), 6.95, (1H, dd, J = 8.3, 1.5 Hz), 7.15 (1H, t, J = 8.1 Hz), 7.20 (1H, d, J = 8.5 Hz), 7.24 (1H, dd, J = 7.8, 1.2 Hz), 7.36-7.40 (2H, m).

### Example 859

3-(2-{[4,6-trans-8-Chloro-1-cyclohexyl-6-(2,3-dimethoxyphenyl)-4H,6H-[1,2,4]triazolo[4,3-a] [4,1]benzothiazepin-4-yl]methyl}-1,3-thiazol-5-yl)propanoic acid

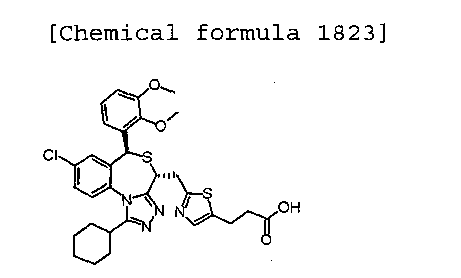

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (74 mg) was obtained using methyl 3-(2-{[4,6-trans-8-chloro-1-cyclohexyl-6-(2,3-dimethoxyphenyl)-4H,6H-[1,2,4]triazolo[4,3-a][4,1]benzothiazepin-4-yl]methyl}-1,3-thiazol-5-yl)propanoate (112 mg).
MS (ESI) m/z : 625 (M + H)⁺.
¹H-NMR (CDCl₃) δ: 1.06-1.19 (1H, m), 1.26-1.37 (2H, m), 1.46-1.57 (1H, m), 1.66-1.76 (3H, m), 1.90-2.19 (3H, m), 2.65-2.78 (3H, m), 3.10 (2H, t, J = 7.0 Hz), 3.50 (3H, s), 3.63 (1H, dd, J = 15.4, 7.6 Hz), 3.84 (3H, s), 4.09 (1H, dd, J = 15.4, 6.9 Hz), 4.41 (1H, t, J = 7.1 Hz), 5.34 (1H, s), 6.93 (1H, d, J = 2.2 Hz), 6.95 (1H, dd, J = 8.3, 1.5 Hz), 7.14 (1H, t, J = 8.1 Hz), 7.20 (1H, d, J = 8.6 Hz), 7.24 (2H, dd, J = 8.0, 1.1 Hz), 7.36-7.40 (2H, m). Elemental analysis for: C₃₁H₃₃ClN₄O₄S₂·1.25H₂O
Calculated: C, 57.48; H, 5.52; N, 8.65.
Found: C, 57.34; H, 5.48; N, 8.17.

### Referential Example 785

Methyl 2-[(3R,5S)-7-chloro-5-(2,3-dimethoxyphenyl)-2-thioxo-1,2,3,5-tetrahydro-4,1-benzothiazepin-3-yl]acetate
(isomer A)
Methyl 2-[(3S,5R)-7-chloro-5-(2,3-dimethoxyphenyl)-2-thioxo-1,2,3,5-tetrahydro-4,1-benzothiazepin-3-yl]acetate
(isomer B)

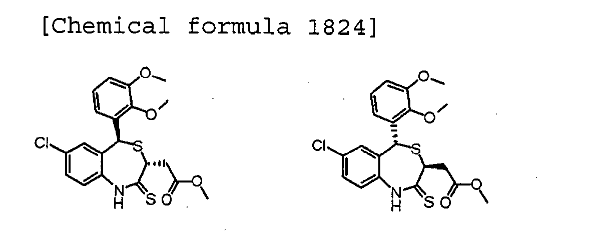

Methyl 2-[(trans)-7-chloro-5-(2,3-dimethoxyphenyl)-2-thioxo-1,2,3,5-tetrahydro-4,1-benzothiazepin-3-yl]acetate was optically resolved by HPLC.
Column: Chiralcel OD (5 × 50 cm)
Eluting solution: 10% ethanol-n-hexane
Flow rate: 40 mL/min
Elution time: isomer A: 46.6 min., isomer B: 60.4 min.

### Example 860

Methyl 2-[(4R,6S)-8-chloro-6-(2,3-dimethoxyphenyl)-i-(trifluoromethyl)-4H,6H-[1,2,4]triazolo[4,3-a][4,1]benzothiazepin-4-yl]acetate

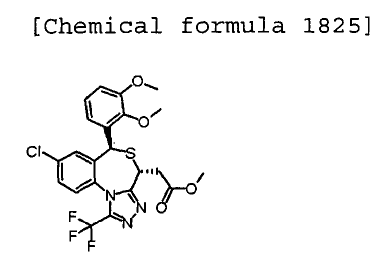

In a similar manner to that employed for the synthesis of methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate, the title compound (286 mg) was obtained using methyl 2-[(3R,5S)-7-chloro-5-(2,3-dimethoxyphenyl)-2-thioxo-1,2,3,5-tetrahydro-4,1-benzothiazepin-3-yl]acetate (438 mg).
MS (ESI) m/z : 514 (M + H)⁺.
¹H-NMR (CDCl₃) δ: 2.92 (1H, dd, J = 17.2, 5.6 Hz), 3.43 (3H, s), 3.64 (1H, dd, J = 17.2, 8.9 Hz), 3.71 (3H, s), 3.85 (3H, s), 4.31 (1H, dd, J = 8.9, 5.6 Hz), 5.35 (1H, s), 7.00-6.96 (2H, m), 7.15-7.22 (2H, m), 7.41-7.37 (1H, m), 7.42-7.46 (1H, m).

### Example 861

methyl 2-[(4S,6R)-8-chloro-6-(2,3-dimethoxyphenyl)-1-(trifluoromethyl)-4H,6H-[1,2,4]triazolo[4,3-a] [4,1]benzothiazepin-4-yl]acetate

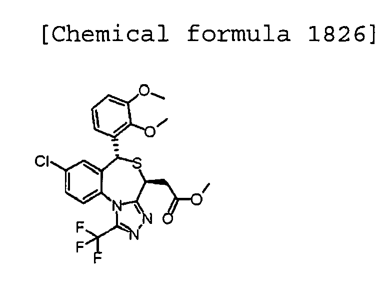

In a similar manner to that employed for the synthesis of methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate, the title compound (289 mg) was obtained using methyl 2-[(3S,5R)-7-chloro-5-(2,3-dimethoxyphenyl)-2-thioxo-1,2,3,5-tetrahydro-4,1-benzothiazepin-3-yl]acetate (438 mg).
MS (ESI) m/z : 514 (M + H)⁺.
¹H-NMR (CDCl₃) δ: 2.92 (1H, dd, J = 17.2, 5.6 Hz), 3.43 (3H, s), 3.64 (1H, dd, J = 17.2, 8.9 Hz), 3.71 (3H, s), 3.85 (3H, s), 4.31 (1H, dd, J = 8.9, 5.6 Hz), 5.35 (1H, s), 6.96-7.00 (2H, m), 7.15-7.22 (2H, m), 7.37-7.41 (1H, m), 7.42-7.46 (1H, m).

### Example 862

2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetic acid

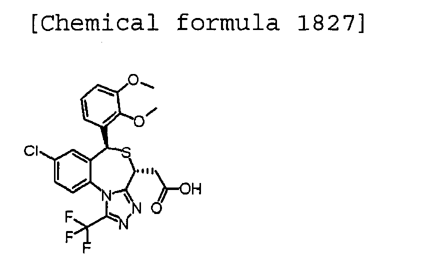

Methyl 2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetate (286 mg) was dissolved in acetone (8.00 mL). To the resulting solution were added water (5.60 mL) and concentrated hydrochloric acid (2.40 mL). The resulting mixture was heated under reflux for 5 hours. To the reaction mixture was added ethyl acetate and the resulting mixture was washed with saturated brine and dried over magnesium sulfate. The solvent was then distilled off under reduced pressure. The residue was purified by HPLC ("Capcellpak C18", 30 × 250 mm, 15 mL/min, 50-100% aq.CH₃CN) to give the title compound (146 mg). MS (ESI) m/z : 500 (M + H)⁺.
¹H-NMR (CDCl₃) δ: 2.81 (1H, dd, J = 17.6, 4.9 Hz), 3.30 (3H, s), 3.50 (1H, dd, J = 17.6, 9.8 Hz), 3.73 (3H, s), 4.14 (1H, dd, J = 9.8, 4.9 Hz), 5.21 (1H, s), 6.85-6.88 (2H, m), 7.05 (2H, t, J = 7.8 Hz), 7.08 (2H, dd, J = 7.8, 2.0 Hz), 7.26 (1H, d, J = 8.3 Hz), 7.32 (1H, dd, J = 8.4, 2.3 Hz).
Elemental analysis for: C₂₁H₁₇ClF₃N₃O₄S
Calculated: C, 50.46; H, 3.43; N, 8.41, S 6.41.
Found: C, 50.23; H, 3.66; N, 8.15, S 6.35.

### Example 863

2-[(4S,6R)-8-Chloro-6-(2,3-dimethoxyphenyl)-1-(trifluoromethyl)-4H,6H-[1,2,4]triazolo[4,3-a][4,1]benzothiazepin-4-yl]acetic acid

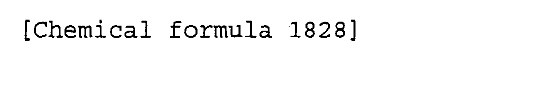

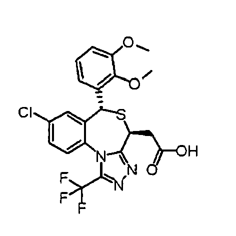

Methyl 2-[(4S,6R)-8-chloro-6-(2,3-dimethoxyphenyl)-1-(trifluoromethyl)-4H,6H-[1,2,4]triazolo[4,3-a][4,1]benzothiazepin-4-yl]acetate (289 mg) was dissolved in acetone (8.00 mL). To the resulting solution were added water (5.60 mL) and concentrated hydrochloric acid (2.40 mL). The resulting mixture was heated under reflux for 5 hours. To the reaction mixture was added ethyl acetate and the resulting mixture was washed with saturated brine and dried over magnesium sulfate. The solvent was distilled off under reduced pressure. The residue was purified by HPLC ("Capcellpak C18", 30 × 250 mm, 15 mL/min, 50-100% aq.CH₃CN) to give the title compound (146 mg). MS (ESI) m/z : 500 (M + H)⁺.
¹H-NMR (CDCl₃) δ: 2.81 (1H, dd, J = 17.6, 4.9 Hz), 3.30 (3H, s), 3.50 (1H, dd, J = 17.6, 9.8 Hz), 3.73 (3H, s), 4.14 (1H, dd, J = 9.8, 4.9 Hz), 5.21 (1H, s), 6.85-6.88 (2H, m), 7.05 (2H, t, J = 7.8 Hz), 7.08 (2H, dd, J = 7.8, 2.0 Hz), 7.26 (1H, d, J = 8.3 Hz), 7.32 (1H, dd, J = 8.4, 2.3 Hz).
Elemental analysis for: C₂₁H₁₇ClF₃N₃O₄S
Calculated: C, 50.46; H, 3.43; N, 8.41, S 6.41.
Found: C, 50.71; H, 3.80; N, 8.06, S 6.35.

### Example 864

Ethyl 3-(1-{2-[(4,6-trans-8-chloro-6-(2,3-dimethoxyphenyl)-1-(trifluoromethyl)-4H,6H-[1,2,4]triazolo[4,3-a][4,1]benzothiazepin-4-yl)acetyl]-4-piperidinyl}propanoate

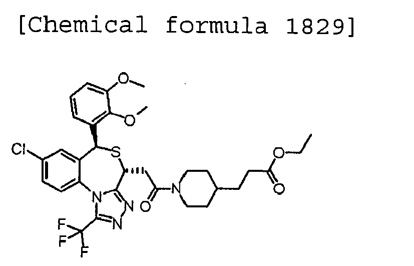

In a similar manner to that employed for the synthesis of ethyl 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl) acetate, the title compound (118 mg) was obtained using 3-[(trans)-8-chloro-6-(2,3-dimethoxyphenyl)-1-(trifluoromethyl)-4H,6H-[1,2,4]triazolo[4,3-a] [4,1]benzothiazepin-4-yl]acetic acid (100 mg) . MS (ESI) m/z : 667 (M + H)⁺.
¹H-NMR (CDCl₃) δ: 1.01-1.17 (1H, m), 1.26 (3H, t, J = 7.2 Hz), 1.48-1.83 (6H, m), 2.30-2.36 (2H, m), 2.51-2.60 (1H, m), 2.79-2.87 (1H, m), 2.99-3.16 (1H, m), 3.42 (3H, s), 3.63-3.71 (1H, m), 3.85 (3H, s), 3.99 (1H, t, J = 15.5 Hz), 4.09-4.16 (2H, m), 4.50 (2H, td, J = 9.0, 5.0 Hz), 5.32 (1H, s), 6.96-6.99 (2H, m), 7.17 (1H, td, J = 8.1, 2.9 Hz), 7.22-7.25 (1H, m), 7.38-7.43 (2H, m).

### Example 865

3-(1-{2-[(4,6-trans-8-Chloro-6-(2,3-dimethoxyphenyl)-1-(trifluoromethyl)-4H,6H-[1,2,4]triazolo[4,3-a][4,1]benzothiazepin-4-yl)acetyl]-4-piperidinyl}propanoic acid

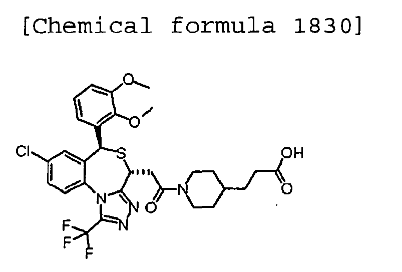

In a similar manner to that employed for the synthesis of 1-{4-[(4R,6S)-8-chloro-6-(2,3-dimethoxyphenyl)-4H,6H-pyrrolo[1,2-a][4,1]benzoxazepin-4-yl]-3-hydroxybutanoyl}-3-azetidine carboxylic acid, the title compound (83 mg) was obtained using ethyl 3-(1-{2-[(4,6-trans-8-chloro-6-(2,3-dimethoxyphenyl)-1-(trifluoromethyl)-4H,6H-[1,2,4]triazolo[4,3-a][4,1]benzothiazepin-4-yl)acetyl]-4-piperidinyl}propanoate (118 mg).
MS (ESI) m/z : 639 (M + H)⁺.
¹H-NMR (CDCl₃) δ: 1.03-1.18 (1H, m), 1.45-1.86 (6H, m), 2.36-2.44 (2H, m), 2.51-2.62 (1H, m), 2.77-2.88 (1H, m), 3.01-3.17 (1H, m), 3.42 (3H, s), 3.63-3.72 (1H, m), 3.85 (3H, s), 3.95-4.04 (1H, m), 4.46-4.54 (2H, m), 5.32 (1H, s), 6.95-7.00 (2H, m), 7.14-7.25 (0H, m), 7.38-7.44 (2H, m).
Elemental analysis for: C₂₉H₃₀ClF₃N₄O₅S·0.75H₂O
Calculated: C, 53.37; H, 4.87; N, 8.59.
Found: C, 53.64; H, 4.88; N, 8.44.

### Example 866

Ethyl (3S)-1-{2-[4,6-trans-8-chloro-6-(2,3-dimethoxyphenyl)-1-(trifluoromethyl)-4H,6H-[1,2,4]triazolo[4,3-a][4,1]benzothiazepin-4-yl]acetyl}-3-piperidine carboxylate

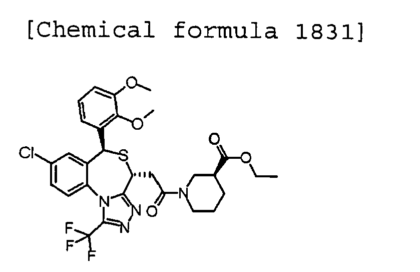

In a similar manner to that employed for the synthesis of ethyl 2-(1-{2-[8-chloro-6-(2,3-dimethoxyphenyl)-1-(trifluoromethyl)-4H,6H-[1,2,4]triazolo[4,3-a] [4,1]benzothiazepin-4-yl]acetyl}-4-piperidinyl) acetate, the title compound (58 mg) was obtained from 3-[(trans)-8-chloro-6-(2,3-dimethoxyphenyl)-1-(trifluoromethyl)-4H,6H-[1,2,4]triazolo[4,3-a] [4,1]benzothiazepin-4-yl]acetic acid (50 mg). MS (ESI) m/z : 639 (M + H)⁺.
¹H-NMR (CDCl₃) δ: 1.20-1.31 (3H, m), 1.42-1.90 (3H, m), 2.00-2.16 (1H, m), 2.35-2.51 (1H, m), 2.72-3.03 (2H, m), 3.08-3.24 (1H, m), 3.42 (3H, s), 3.46-3.79 (2H, m), 3.85 (3H, s), 3.87-3.95 (1H, m), 4.08-4.23 (2H, m), 4.46-4.53 (1H, m), 5.33 (1H, s), 6.96-6.99 (2H, m), 7.17 (1H, t, J = 7.9 Hz), 7.22-7.25 (1H, m), 7.39-7.42 (2H, m).

### Example 867

(3S)-1-{2-[4,6-trans-8-Chloro-6-(2,3-dimethoxyphenyl)-1-(trifluoromethyl)-4H,6H-[1,2,4]triazolo[4,3-a][4,1]benzothiazepin-4-yl]acetyl}-3-piperidine carboxylic acid

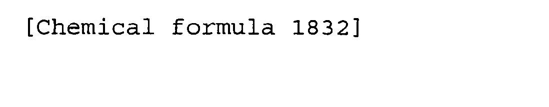

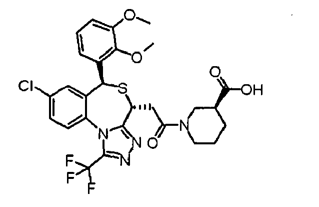

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (35 mg) was obtained using ethyl (3S)-1-{2-[4,6-trans-8-chloro-6-(2,3-dimethoxyphenyl)-1-(trifluoromethyl)-4H,6H-[1,2,4]triazolo[4,3-a][4,1]benzothiazepin-4-yl]acetyl}-3-piperidine carboxylate (58 mg).
MS (ESI) m/z : 611 (M + H)⁺.
¹H-NMR (CDCl₃) δ: 1.42-1.90 (4H, m), 2.41-2.59 (1H, m), 2.71-3.33 (3H, m), 3.41 (3H, s), 3.60-3.82 (2H, m), 3.85 (3H, s), 3.89-4.00 (1H, m), 4.44-4.53 (1H, m), 5.25-5.34 (1H, m), 6.94-6.99 (2H, m), 7.13-7.19 (1H, m), 7.20-7.24 (1H, m), 7.40-7.44 (2H, m).
Elemental analysis for: C₂₇H₂₆ClF₃N₄O₅S?2.0H₂O
Calculated: C, 50.12; H, 4.67; N, 8.66.
Found: C, 49.77; H, 4.32; N, 8.49.

### Example 868

Ethyl 2-(1-{2-[4,6-trans-8-chloro-6-(2,3-dimethoxyphenyl)-1-(trifluoromethyl)-4H,6H-[1,2,4]triazolo[4,3-a][4,1]benzothiazepin-4-yl]acetyl}-3-azetidinyl)acetate

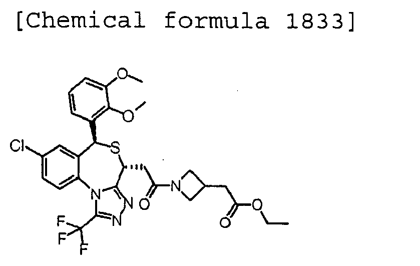

In a similar manner to that employed for the synthesis of ethyl 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl) acetate, the title compound (55 mg) was obtained using 3-[(trans)-8-chloro-6-(2,3-dimethoxyphenyl)-1-(trifluoromethyl)-4H,6H-[1,2,4]triazolo[4,3-a][4,1]benzothiazepin-4-yl]acetic acid (50 mg). MS (ESI) m/z : 625 (M + H)⁺.
¹H-NMR (CDCl₃) δ: 1.24-1.29 (3H, m), 2.52-2.60 (1H, m), 2.63-2.71 (2H, m), 2.95-3.06 (1H, m), 3.26-3.35 (1H, m), 3.41 (3H, s), 3.63-3.69 (1H, m), 3.85 (3H, s), 4.11-4.25 (4H, m), 4.37-4.72 (2H, m), 5.34 (1H, s), 6.96-6.99 (2H, m), 7.17 (1H, t, J = 7.9 Hz), 7.21-7.24 (1H, m), 7.36 (1H, d, J = 8.5 Hz), 7.41 (1H, dd, J = 8.5, 2.2 Hz).

### Example 869

2-(1-{2-[4,6-trans-8-chloro-6-(2,3-dimethoxyphenyl)-1-(trifluoromethyl)-4H,6H-[1,2,4]triazolo[4,3-a][4,1]benzothiazepin-4-yl]acetyl}-3-azetidinyl)acetic acid

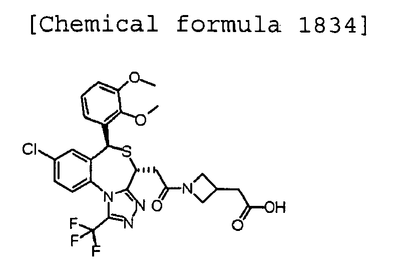

In a similar manner to that employed for the synthesis of 2-[8-chloro-6-(2,3-dimethoxyphenyl)-1-(trifluoromethyl)-4H,6H-[1,2,4]triazolo[4,3-a][4,1]benzothiazepin-4-yl]acetic acid, the title compound (35 mg) was obtained using ethyl 2-(1-{2-[4,6-trans-8-chloro-6-(2,3-dimethoxyphenyl)-1-(trifluoromethyl)-4H,6H-[1,2,4]triazolo[4,3-a] [4,1]benzothiazepin-4-yl]acetyl}-3-azetidinyl)acetate (58 mg).
MS (ESI) m/z : 597 (M + H)⁺.
¹H-NMR (CDCl₃) δ: 2.56 (1H, td, J = 15.1, 5.4 Hz), 2.64-2.70 (2H, m), 2.93-3.04 (1H, m), 3.26-3.34 (1H, m), 3.41 (3H, s), 3.63-3.75 (1H, m), 3.84 (3H, s), 4.12 (1H, q, J = 9.7 Hz), 4.26-4.69 (3H, m), 5.33 (1H, s), 6.96-6.99 (2H, m), 7.17 (1H, t, J = 7.9 Hz), 7.20-7.24 (1H, m), 7.35-7.43 (2H, m).
Elemental analysis for: C₂₆H₂₄ClF₃N₄O₅S?1.75H₂O?0.25CHCl₃?0.25 1,4-dioxane
Calculated: C, 48.31; H, 4.42; N, 8.20.
Found: C, 48.15; H, 4.20; N, 8.54.

### Example 870

Methyl 3-(2-{[4,6-trans-1-(tert-butyl)-8-chloro-6-(2,3-dimethoxyphenyl)-4H,6H-[1,2,4]triazolo[4,3-a][4,1]benzothiazepin-4-yl]methyl}-1,3-thiazol-5-yl)propanoate

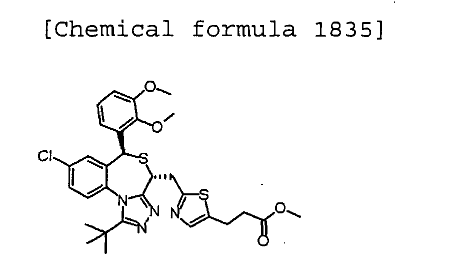

In a similar manner to that employed for the synthesis of ethyl 2-[(trans)-8-chloro-1-cyclopropyl-6-(2,3-dimethoxyphenyl)-4H,6H-[1,2,4]triazolo[4,3-a][4,1]benzoxazepin-4-yl]acetate, the title compound (94 mg) was obtained using ethyl 2-(2-{[3,5-trans-7-chloro-5-(2,3-dimethoxyphenyl)-2-thioxo-1,2,3,5-tetrahydro-4,1-benzothiazepin-3-yl]methyl}-1,3-thiazol-5-yl)propanoate (120 mg).
MS (ESI) m/z : 613 (M + H)⁺.
¹H-NMR (CDCl₃) δ: 1.42 (9H, s), 2.65 (2H, t, J = 7.0 Hz), 3.09 (2H, t, J = 7.0 Hz), 3.56-3.64 (4H, m), 3.69 (3H, s), 3.86 (2H, s), 4.07 (1H, dd, J = 15.4, 7.1 Hz), 4.24 (1H, t, J = 7.1 Hz), 5.34 (1H, s), 6.92-6.98 (2H, m), 7.15 (1H, t, J = 7.8 Hz), 7.23-7.26 (1H, m), 7.37-7.34 (2H, m).

### Example 871

3-(2-{[4,6-trans-1-(tert-Butyl)-8-chloro-6-(2,3-dimethoxyphenyl)-4H,6H-[1,2,4]triazolo[4,3-a][4,1]benzothiazepin-4-yl]methyl}-1,3--5-yl)propanoic acid

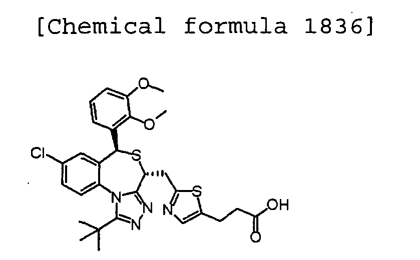

In a similar manner to that employed for the synthesis of 1-{4-[(4R,6S)-8-chloro-6-(2,3-dimethoxyphenyl)-4H,6H-pyrrolo[1,2-a][4,1]benzoxazepin-4-yl]-3-hydroxybutanoyl}-3-azetidine carboxylic acid, the title compound (73 mg) was obtained using methyl 3-(2-{[4,6-trans-1-(tert-butyl)-8-chloro-6-(2,3-dimethoxyphenyl)-4H,6H-[1,2,4]triazolo[4,3-a][4,1]benzothiazepin-4-yl]methyl}-1,3-thiazol-5-yl)propanoate (94 mg).
MS (ESI) m/z : 599 (M + H)⁺.
¹H-NMR (CDCl₃) δ: 1.42 (9H, s), 2.69 (2H, t, J = 7.0 Hz), 3.11 (2H, t, J = 7.0 Hz), 3.56-3.64 (4H, m), 3.86 (2H, s), 4.09 (1H, dd, J = 15.4, 7.3 Hz), 4.26 (1H, t, J = 7.3 Hz), 5.33 (1H, s), 6.92-6.98 (2H, m), 7.15 (1H, t, J = 7.9 Hz), 7.23-7.26 (1H, m), 7.35 (2H, s), 7.40 (1H, s).
Elemental analysis for: C₂₉H₃₁ClN₄O₄S₂·0.75 H₂O·0.5 n-Hexane Calculated: C, 58.61; H, 6.07; N, 8.54.
Found: C, 58.46; H, 5.79; N, 8.66.

### Example 872

Ethyl 3-(1-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)propanoate

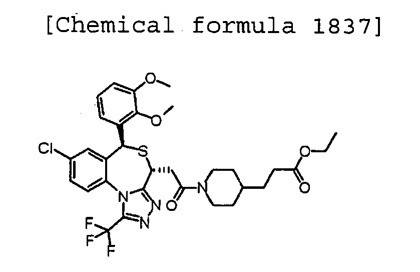

In a similar manner to that employed for the synthesis of ethyl 2-(1-(2-(8-chloro-6-(2,3-dimethoxyphenyl)-1-(trifluoromethyl)-4H,6H-(1,2,4)triazolo(4,3-a)(4,1)benzothiazepin-4-yl)acetyl)-4-piperidinyl)acetate, the title compound (120 mg) was obtained using 2-[(4R,6S)-8-chloro-6-(2,3-dimethoxyphenyl)-1-(trifluoromethyl)-4H,6H-[1,2,4]triazolo[4,3-a] [4,1] benzothiazepin-4-yl] acetic acid (150 mg) . MS (ESI) m/z : 667 (M + H)⁺.
¹H-NMR (CDCl₃) δ: 1.02-1.17 (1H, m), 1.24-1.29 (3H, m), 1.48-1.84 (6H, m), 2.30-2.36 (2H, m), 2.50-2.60 (1H, m), 2.76-2.88 (1H, m), 2.99-3.16 (1H, m), 3.42 (3H, s), 3.62-3.71 (1H, m), 3.85 (3H, s), 3.94-4.04 (1H, m), 4.09-4.16 (2H, m), 4.45-4.53 (2H, m), 5.32 (1H, s), 6.95-6.99 (2H, m), 7.14-7.19 (1H, m), 7.22-7.26 (1H, m), 7.39-7.42 (2H, m).

### Example 873

3-(1-{2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)propanoic acid

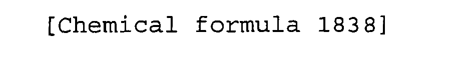

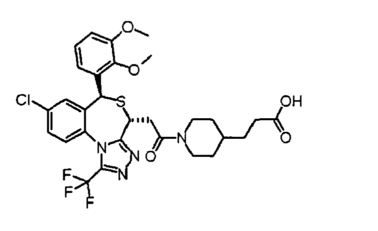

Ethyl 3-(1-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)propanoate (120 mg) was dissolved in 1,4-dioxane (7.5 mL). To the resulting solution was added concentrated hydrochloric acid (1.65 mL). The resulting mixture was stirred at 70°C for 7 hours. Ethyl acetate was added and the resulting mixture was washed with saturated brine and dried over magnesium sulfate. The solvent was distilled off under reduced pressure. The residue was purified by preparative thin layer chromatography (C18, 70% aq. MeOH) to give the title compound (94 mg). MS (ESI) m/z : 639 (M + H)⁺.
¹H-NMR (CDCl₃) δ: 1.02-1.17 (1H, m), 1.31-1.84 (6H, m), 2.32-2.41 (2H, m), 2.50-2.60 (1H, m), 2.76-2.88 (1H, m), 2.99-3.16 (1H, m), 3.42 (3H, s), 3.62-3.73 (1H, m), 3.85 (3H, s), 3.98 (1H, t, J = 13.7 Hz), 4.45-4.53 (2H, m), 5.32 (1H, s), 6.96-7.00 (2H, m), 7.14-7.19 (1H, m), 7.22-7.26 (1H, m), 7.40-7.42 (2H, m). Elemental analysis for: C₂₉H₃₀ClF₃N₄O₅S·0.5 H₂O·0.25 CHCl₃·0.25 n-Hexane
Calculated: C, 52.98; H, 5.02; N, 7.97.
Found: C, 53.15; H, 5.01; N, 7.79.

### Referential Example 786

Isopropyl 2-[3,5-trans-7-chloro-5-(2,3-dimethoxyphenyl)-2-oxo-1,2,3,5-tetrahydro-4,1-benzothiazepin-3-yl]acetate

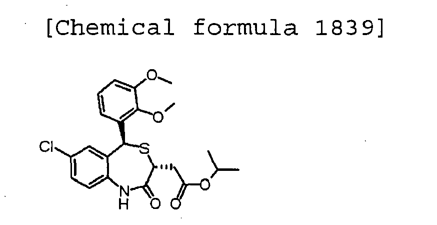

2-[7-Chloro-5-(2,3-dimethoxyphenyl)-2-oxo-1,2,3,5-tetrahydro-4,1-benzothiazepin-3-yl]acetic acid (2189 mg) was dissolved in N,N-dimethylformamide (5 mL). To the resulting solution were added potassium carbonate (2225 mg) and 2-iodopropane (1607 µl). The resulting mixture was stirred at room temperature for 19 hours. The reaction mixture was diluted with ethyl acetate and washed with saturated brine. The organic layer was dried over anhydrous sodium sulfate and concentrated to give the title compound (2564 mg).
MS m/z : 514 (M+H⁺).
¹H-NMR (CDCl₃) δ: 1.19-1.24 (6H, m), 2.40 (1H, dd, J = 17.1, 3.9 Hz), 3.08 (1H, dd, J = 17.1, 10.5 Hz), 3.60 (3H, s), 3.82 (1H, dd, J = 10.5, 3.9 Hz), 3.87 (3H, s), 4.93-5.01 (1H, m), 6.19 (1H, s), 6.85 (1H, d, J = 2.2 Hz), 6.97 (1H, dd, J = 8.3, 1.5 Hz), 7.08 (1H, d, J = 8.5 Hz), 7.18 (1H, t, J = 8.1 Hz), 7.24 (1H, dd, J = 8.4, 2.3 Hz), 7.31 (1H, dd, J = 7.9, 1.3 Hz), 7.38 (1H, br s) .

### Referential Example 787

Isopropyl 2-[3,5-trans-7-chloro-5-(2,3-dimethoxyphenyl)-2-thioxo-1,2,3,5-tetrahydro-4,1-benzothiazepin-3-yl]acetate

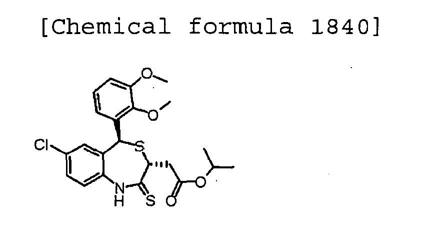

Isopropyl 2-[3,5-trans-7-chloro-5-(2,3-dimethoxyphenyl)-2-oxo-1,2,3,5-tetrahydro-4,1-benzothiazepin-3-yl]acetate (450 mg) was dissolved in toluene (10 mL). To the resulting solution was added Lawesson's reagent (334 mg). The resulting mixture was stirred at 80°C for 4 hours and then, at 100°C for one hour. The reaction mixture was cooled to room temperature, followed by concentration. The residue was purified by silica gel column (ethyl acetate:n-hexane=1:2) to give the title compound (444 mg). MS m/z : 466 (M+H⁺).
¹H-NMR (CDCl₃) δ: 1.22 (6H, t, J = 6.6 Hz), 2.58 (1H, dd, J = 17.0, 4.0 Hz), 3.37 (1H, dd, J = 17.0, 10.1 Hz), 3.61 (3H, s), 3.88 (3H, s), 4.07 (1H, dd, J = 10.3, 4.0 Hz), 4.94-5.01 (1H, m), 6.02 (1H, s), 6.84 (1H, d, J = 2.2 Hz), 6.97 (1H, dd, J = 8.2, 1.6 Hz), 7.10 (1H, d, J = 8.3 Hz), 7.18 (1H, t, J = 7.9 Hz), 7.24-7.30 (2H, m).

### Example 874

Isopropyl 2-[3,5-trans-8-chloro-6-(2,3-dimethoxyphenyl)-1-(trifluoromethyl)-4H,6H-[1,2,4]triazolo[4,3-a][4,1]benzothiazepin-4-yl]acetate

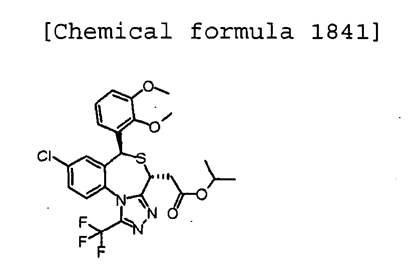

Isopropyl 2-[(trans)-7-chloro-5-(2,3-dimethoxyphenyl)-2-thioxo-1,2,3,5-tetrahydro-4,1-benzothiazepin-3-yl]acetate (398 mg) was dissolved in tetrahydrofuran (10 mL). To the resulting solution was added hydrazine monohydrate (0.171 mL). The resulting mixture was stirred at room temperature for 2 hours. After concentration, the residue was dissolved in toluene (10 mL). To the resulting solution were added trifluoroacetic anhydride (0.375 mL) and trifluoroacetic acid (1.36 mL). The resulting mixture was stirred at room temperature for 1.3 hours, and then at 55°C for one hour. The reaction mixture was cooled to room temperature, diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography to give the title compound (304 mg).
MS (ESI) m/z : 542 (M + H)⁺.
¹H-NMR (CDCl₃) δ: 1.21 (3H, d, J = 6.3 Hz), 1.24 (3H, d, J = 6.3 Hz), 2.87 (1H, dd, J = 17.3, 5.4 Hz), 3.43 (3H, s), 3.59 (1H, dd, J = 17.3, 9.0 Hz), 3.85 (3H, s), 4.30 (1H, dd, J = 9.0, 5.4 Hz), 4.95-5.01 (1H, m), 5.34 (1H, s), 6.96-7.00 (2H, m), 7.17 (2H, t, J = 7.9 Hz), 7.21 (1H, dd, J = 8.1, 1.7 Hz), 7.38 (1H, d, J = 8.5 Hz), 7.44 (1H, dd, J = 8.5, 2.2 Hz).

### Example 875

2-[4,6-trans-8-Chloro-6-(2,3-dimethoxyphenyl)-1-(trifluoromethyl)-4H,6H-[1,2,4]triazolo[4,3-a] [4,1]benzothiazepin-4-yl]acetic acid

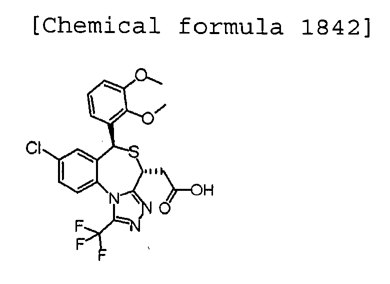

Isopropyl 2-[3,5-trans-8-chloro-6-(2,3-dimethoxyphenyl)-1-(trifluoromethyl)-4H,6H-[1,2,4]triazolo[4,3-a][4,1]benzothiazepin-4-yl]acetate (56.61 g) was dissolved in 1,4-dioxane (1000 mL). To the resulting solution was added concentrated hydrochloric acid (500 mL). The resulting mixture was stirred at 80°C for 2 hours and then, at 65°C for 15 hours. To the reaction mixture was added ethyl acetate. The resulting mixture was washed with saturated brine, dried over magnesium sulfate and distilled under reduced pressure to remove the solvent. The residue was solidified from a methylene chloride-n-hexane solvent mixture to give the title compound (21.02 g).
MS (ESI) m/z : 500 (M + H)⁺.
¹H-NMR (CDCl₃) δ: 2.96 (1H, dd, J = 17.6, 5.1 Hz), 3.43 (3H, s), 3.65 (1H, dd, J = 17.6, 9.5 Hz), 3.85 (3H, s), 4.27 (1H, dd, J = 9.5, 5.1 Hz), 5.34 (1H, s), 6.97-7.00 (2H, m), 7.15-7.22 (2H, m), 7.39 (1H, d, J = 8.3 Hz), 7.44 (1H, dd, J = 8.5, 2.2 Hz).

### Example 876

Methyl (2S)-2-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)-4-methylpentanoate (isomer A)
Methyl (2S)-2-({2-[(4S,6R)-8-chloro-6-(2,3-dimethoxyphenyl)-1-(trifluoromethyl)-4H,6H-[1,2,4]triazolo[4,3-a][4,1]benzothiazepin-4-yl]acetyl}amino)-4-methylpentanoate (isomer B)

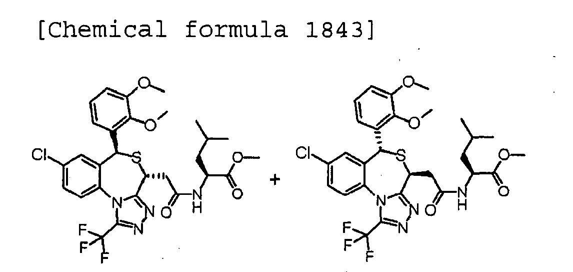

In a similar manner to that employed for the synthesis of ethyl 2-(1-(2-(8-chloro-6-(2,3-dimethoxyphenyl)-1-(trifluoromethyl)-4H,6H-(1,2,4)triazolo(4,3-a)(4,1)benzothiazepin-4-yl)acetyl)-4-piperidinyl)acetate, the title compounds, that is, the isomer A (102 mg) and the isomer B (108 mg) were obtained using 2-[4,6-trans--8-chloro-6-(2,3-dimethoxyphenyl)-1-(trifluoromethyl)-4H,6H-[1,2,4]triazolo[4,3-a] [4,1]benzothiazepin-4-yl]aceticacid (182 mg).
Isomer A
MS (ESI) m/z : 627 (M + H)⁺.
¹H-NMR (CDCl₃) δ: 0.86 (3H, d, J = 6.3 Hz), 0.91 (3H, d, J = 6.1 Hz), 1.51-1.65 (3H, m), 2.82 (1H, dd, J = 15.2, 5.0 Hz), 3.42 (3H, s), 3.48 (1H, dd, J = 15.2, 9.5 Hz),3.72 (3H, s), 3.85 (3H, s), 4.40 (1H, dd, J = 9.5, 4.9 Hz), 4.50-4.56 (1H, m), 5.33 (1H, s), 6.36 (1H, d, J = 8.0 Hz), 6.96-6.99 (2H, m), 7.16 (1H, t, J = 8.0 Hz), 7.22 (1H, dd, J = 7.9, 1.6 Hz), 7.33-7.37 (1H, m), 7.42 (1H, dd, J = 8.4, 2.3 Hz).
Isomer B
MS (ESI) m/z : 627 (M + H)⁺.
¹H-NMR (CDCl₃) δ: 0.93 (6H, d, J = 6.3 Hz), 1.51-1.70 (3H, m), 2.83 (1H, dd, J = 14.7, 5.4 Hz), 3.42 (3H, s), 3.48 (1H, dd, J = 14.7, 8.9 Hz), 3.68 (3H, s), 3.85 (3H, s), 4.35 (1H, dd, J = 8.9, 5.4 Hz), 4.51-4.57 (1H, m), 5.35 (1H, s), 6.51 (1H, d, J = 8.5 Hz), 6.96-6.99 (2H, m), 7.16 (1H, t, J = 7.9 Hz), 7.20-7.23 (1H, m), 7.35-7.38 (1H, m), 7.42 (1H, dd, J = 8.5, 2.2 Hz).

### Example 877

(2S)-2-({2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}amino)-4-methylpentanoic acid

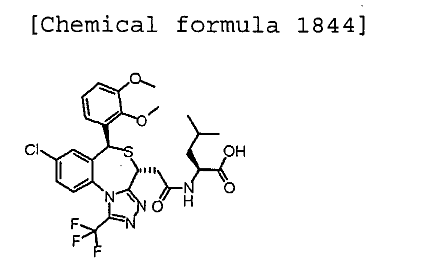

Methyl (2S)-2-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)-4-methylpentanoate (5.68 g) was dissolved in 1,4-dioxane (50 mL). To the resulting solution were added concentrated hydrochloric acid (10 mL) and distilled water (10 mL). The resulting mixture was stirred at 50°C for 23 hours. To the reaction mixture was added ethyl acetate. The resulting mixture was washed with saturated brine, dried over magnesium sulfate, and distilled under reduced pressure to remove the solvent. The residue was purified by silica gel chromatography to give the title compound (2.50 g).
MS (ESI) m/z : 613 (M + H)⁺.
¹H-NMR (CDCl₃) δ: 0.86 (3H, d, J = 6.3 Hz), 0.92 (3H, d, J = 6.3 Hz), 1.54-1.73 (3H, m), 2.82 (1H, dd, J = 15.0, 4.8 Hz), 3.42 (3H, s), 3.50 (1H, dd, J = 15.0, 9.9 Hz), 3.85 (3H, s), 4.40 (1H, dd, J = 9.9, 4.8 Hz), 4.52-4.58 (1H, m), 5.32 (1H, s), 6.62-6.68 (1H, m), 6.96-6.99 (2H, m), 7.16 (1H, t, J = 7.9 Hz), 7.21 (1H, dd, J = 7.8, 1.7 Hz), 7.35 (1H, d, J = 7.8 Hz), 7.43 (1H, dd, J = 8.4, 2.3 Hz). Elemental analysis for: C₂₇H₂₈ClF₃N₄O₅S
Calculated: C, 52.90; H, 4.60; N, 9.14.
Found: C, 52.46; H, 4.65; N, 8.91.

### Example 878

Ethyl 2-(1-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetate

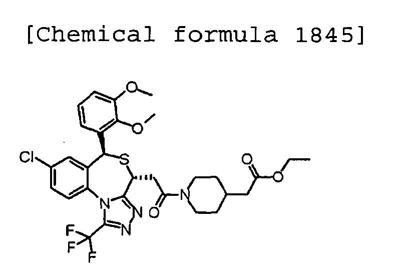

To a solution of 2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetic acid (102 mg) in dichloromethane (5.0 mL) were added ethyl 2-(4-piperidinyl)acetate (56 mg), 1-ethyl-3-(3-dimethylaminopropyl)carbodiimide hydrochloride (78 mg), and 1-hydroxybenzotriazole monohydrate (31 mg). The resulting mixture was stirred at room temperature for 1 hour. The reaction mixture was purified by preparative thin layer chromatography (ethyl acetate:n-hexane=2:1) to give the title compound (41 mg). MS (ESI) m/z : 653 (M + H)⁺.
¹H-NMR (CDCl₃) δ: 1.09-1.46 (6H, m), 1.70-1.88 (2H, m), 2.01-2.09 (1H, m), 2.25 (2H, dd, J = 13.1, 7.2 Hz), 2.56-2.66 (1H, m), 2.77-2.88 (1H, m), 3.04-3.21 (1H, m), 3.42 (3H, s), 3.85 (3H, s), 3.99 (1H, t, J = 15.3 Hz), 4.14 (2H, q, J = 7.2 Hz), 4.46-4.53 (2H, m), 5.32 (1H, s), 6.96-6.99 (2H, m), 7.16 (1H, td, J = 8.0, 1.5 Hz), 7.22-7.25 (1H, m), 7.39-7.42 (2H, m).

### Example 879

2-(1-{2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl)acetic acid

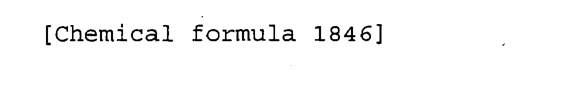

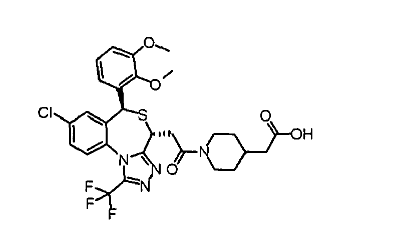

Ethyl 2-(1-{2-[(4R,6S)-8-chloro-6-(2,3-, dimethoxyphenyl)-1-(trifluoromethyl)-4H,6H-[1,2,4]triazolo[4,3-a][4,1]benzothiazepin-4-yl]acetyl}-4-piperidinyl)acetate (78 mg) was dissolved in 1,4-dioxane (3.00 mL). To the resulting solution was added concentrated hydrochloric acid (1.00 mL). The resulting mixture was stirred at 80°C for one hour. To the reaction mixture was added ethyl acetate. The resulting mixture was washed with saturated brine and dried over magnesium sulfate. The solvent was then distilled off under reduced pressure. The residue was purified by preparative thin layer chromatography (10% MeOH-CHCl₃) to give the title compound (41 mg).
MS (ESI) m/z : 625 (M + H)⁺.
¹H-NMR (CDCl₃) δ: 3.47 (3H, s), 3.62-3.67 (1H, m), 3.73-3.79 (1H, m), 3.85 (3H, s), 4.23-4.28 (1H, m), 5.33 (1H, s), 6.93-6.97 (2H, m), 7.12-7.24 (2H, m), 7.35-7.44 (2H, m).
Elemental analysis for: C₂₈H₂₈ClF₃N₄O₅S.·1.5 H2O·0.25 1,4-dioxane
Calculated: C, 51.67; H, 4.93; N, 8.31.
Found: C, 51.48; H, 4.57; N, 8.08.

### Example 880

Methyl 3-(2-{[4,6-trans-8-chloro-1-(1,1-difluoroethyl)-6-(2,3-dimethoxyphenyl)-4H,6H-[1,2,4]triazolo[4,3-a][4,1]benzothiazepin-4-yl]methyl}-1,3-thiazol-5-yl)propanoate

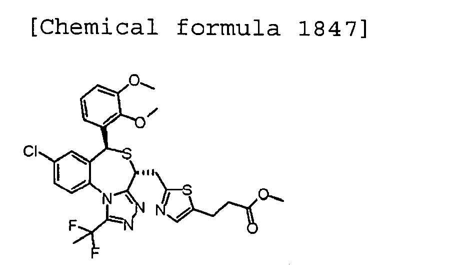

Methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate (556 mg) was dissolved in methylene chloride (10 mL). To the resulting solution were added difluoropropionic acid (447 mg) and dichloromethylenedimethyliminium chloride (330 mg). The resulting mixture was stirred at room temperature for 2 hours. The reaction mixture was diluted with ethyl acetate and washed successively with a saturated aqueous solution of sodium bicarbonate and saturated brine. The organic layer was dried over anhydrous sodium sulfate. After concentration, acetic acid (10 mL) was added to the residue and the resulting mixture was heated under reflux for 18 hours. The reaction mixture was diluted with ethyl acetate and washed successively with a saturated aqueous solution of sodium bicarbonate and saturated brine. The organic layer was dried over anhydrous sodium sulfate. After concentration, the residue was purified by silica gel chromatography (Automatic purification system of Yamazen Corporation, ethyl acetate:n-hexane=1:1 Rf=0.15) to give the title compound (95 mg). MS (ESI) m/z : 621 (M + H)⁺.
¹H-NMR (CDCl₃) δ: 2.29 (3H, t, J = 19.0 Hz), 2.63-2.67 (0H, m), 3.08-3.12 (2H, m), 3.38 (3H, s), 3.63 (1H, dd, J = 15.4, 7.3 Hz), 3.69 (3H, s), 3.84 (3H, s), 4.07-4.14 (1H, m), 4.41 (1H, t, J = 6.7 Hz), 5.40 (1H, s), 6.94-6.97 (2H, m), 7.14 (1H, t, J = 7.9 Hz), 7.19-7.21 (1H, m), 7.36-7.45 (3H, m).

### Example 881

3-(2-{[4,6-trans-8-Chloro-1-(1,1-difluoroethyl)-6-(2,3-dimethoxyphenyl)-4H,6H-[1,2,4]triazolo[4,3-a][4,1]benzothiazepin-4-yl]methyl}-1,3-thiazol-5-yl)propanoic acid

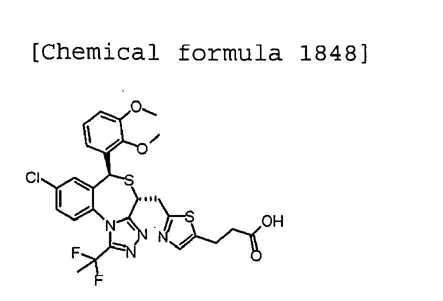

Methyl 3-(2-{[4,6-trans-8-chloro-1-(1,1-difluoroethyl)-6-(2,3-dimethoxyphenyl)-4H,6H-[1,2,4]triazolo[4,3-a][4,1]benzothiazepin-4-yl]methyl}-1,3-thiazol-5-yl)propanoate (95 mg) was dissolved in a solvent mixture of tetrahydrofuran (5 mL), methanol (5 mL) and distilled water (5 mL). To the resulting solution was added potassium carbonate (63.42 mg). The resulting mixture was stirred at room temperature for 18 hours. To the reaction mixture was added an ion exchange resin IR-120B to make it acidic. The resin was filtered out and the solvent was concentrated under reduced pressure. The residue was purified by reversed phase preparative thin layer chromatography (C18, 80% aq. MeCN) to give the title compound (62 mg).
MS (ESI) m/z : 606 (M + H)⁺.
¹H-NMR (CDCl₃) δ: 2.18 (3H, t, J = 19.0 Hz), 2.35-2.44 (2H, m), 2.81-2.90 (2H, m), 3.34 (3H, s), 3.41-3.47 (1H, m), 3.81 (3H, s), 3.95-4.03 (1H, m), 4.29-4.35 (1H, m), 5.33 (1H, s), 6.89-6.95 (2H, m), 7.06-7.19 (2H, m), 7.28-7.37 (3H, m).
Elemental analysis for: C₂₇H₂₅ClF₂N₄O₄S₂·1.0H₂O·0.75CHCl₃ Calculated: C, 47.25; H, 3.97; N, 7.73.
Found: C, 47.29; H, 4.08; N, 7.83.

### Referential Example 788

Isopropyl 2-[4,6-trans-1-(tert-butyl)-8-chloro-6-(2,3-dimethoxyphenyl)-4H,6H-[1,2,4]triazolo[4,3-a] [4,1]benzothiazepin-4-yl]acetate

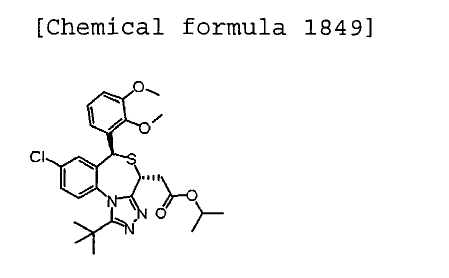

Isopropyl 2-[(trans)-7-chloro-5-(2,3-dimethoxyphenyl)-2-thioxo-1,2,3,5-tetrahydro-4,1-benzothiazepin-3-yl]acetate (100 mg) was dissolved in tetrahydrofuran (1 mL). To the resulting solution was added hydrazine monohydrate (0.042 mL). The resulting mixture was stirred at room temperature for 1.3 hours. To the reaction mixture was added toluene (5 mL), followed by the addition of trimethylacetic anhydride (0.088 mL) and pivalic acid (112 mg). The resulting mixture was stirred at room temperature for 3.8 hours. The reaction mixture was heated under reflux for 10 hours. After cooling to room temperature, the reaction mixture was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography to give the title compound (99 mg).
MS (ESI) m/z : 530 (M + H)⁺.
¹H-NMR (CDCl₃) δ: 1.20 (3H, d, J = 6.1 Hz), 1.23 (3H, d, J = 6.6 Hz), 1.42 (9H, s), 2.84 (1H, dd, J = 17.2, 5.9 Hz), 3.53 (1H, dd, J = 17.2, 8.3 Hz), 3.57 (3H, s), 3.86 (3H, s), 4.98 (1H, dd, J = 8.3, 5.9 Hz), 5.34 (1H, s), 6.96-6.99 (2H, m), 7.17 (1H, t, J = 8.0 Hz), 7.27-7.30 (1H, m), 7.38-7.41 (2H, m).

### Referential Example 789

Isopropyl 2-[4,6-trans-8-chloro-6-(2,3-dimethoxyphenyl)-1-isopropyl-4H,6H-[1,2,4]triazolo[4,3-a][4,1]benzothiazepin-4-yl]acetate

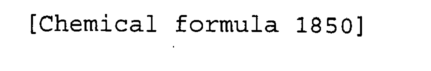

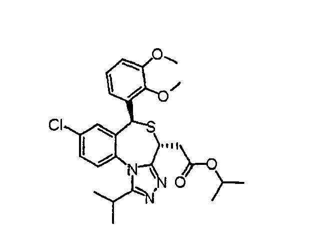

Isopropyl 2-[(trans)-7-chloro-5-(2,3-dimethoxyphenyl)-2-thioxo-1,2,3,5-tetrahydro-4,1-benzothiazepin-3-yl]acetate (100 mg) was dissolved in tetrahydrofuran (1 mL). To the resulting solution was added hydrazine monohydrate (0.042 mL). The resulting mixture was stirred at room temperature for 1.3 hours. To the reaction mixture was added toluene (5 mL), followed by the addition of isobutyric anhydride (0.071 mL) and isobutyric acid (0.100 mL). The resulting mixture was stirred at room temperature for 3.8 hours. The reaction mixture was heated under reflux for 10 hours. After cooling to room temperature, the reaction mixture was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography to give the title compound (94 mg). MS (ESI) m/z : 516 (M + H)⁺.
¹H-NMR (CDCl₃) δ: 1.16-1.23 (9H, m), 1.57 (3H, d, J = 6.9 Hz), 2.87 (1H, dd, J = 17.2, 6.3 Hz), 3.08-3.16 (1H, m), 3.52 (3H, s), 3.56 (1H, dd, J = 17.2, 8.1 Hz), 3.85 (3H, s), 4.30 (1H, dd, J = 8.1, 6.3 Hz), 4.95-5.02 (1H; m), 5.38 (1H, s), 6.95-6.98 (2H, m), 7.17 (1H, t, J = 8.0 Hz), 7.25 (1H, t, J = 9.7 Hz), 7.29 (1H, dd, J = 7.8, 1.2 Hz), 7.39 (1H, dd, J = 8.3, 2.2 Hz).

### Example 882

Isopropyl 2-[4,6-trans-8-chloro-1-[chloro(difluoro)methyl]-6-(2,3-dimethoxyphenyl)-4H,6H-[1,2,4]triazolo[4,3-a][4,1]benzothiazepin-4-yl]acetate

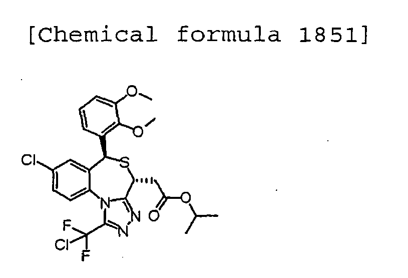

Isopropyl 2-[(trans)-7-chloro-5-(2,3-dimethoxyphenyl)-2-thioxo-1,2,3,5-tetrahydro-4,1-benzothiazepin-3-yl]acetate (100 mg) was dissolved in tetrahydrofuran (1 mL). To the resulting solution was added hydrazine monohydrate (0.042 mL). The resulting mixture was stirred at room temperature for 2 hours. To the reaction mixture was added toluene (5 mL), followed by the addition of chlorodifluoroacetic anhydride (0.075 mL) and chlorodifluoroacetic acid (0.091 mL). The resulting mixture was stirred at room temperature for 2.6 hours. The reaction mixture was heated under reflux for 3.8 hours. After cooling to room temperature, the reaction mixture was concentrated. The residue thus obtained was purified by silica gel chromatography to give the title compound (88 mg).
MS (ESI) m/z : 558 (M + H)⁺.
¹H-NMR (CDCl₃) δ: 1.21 (3H, d, J = 6.4 Hz), 1.24 (3H, d, J = 6.4 Hz), 2.87 (1H, dd, J = 17.2, 5.4 Hz), 3.48 (3H, s), 3.58 (1H, dd, J = 17.3, 9.2 Hz), 3.85 (3H, s), 4.28 (1H, dd, J = 9.1, 5.4 Hz), 4.95-5.02 (1H, m)., 5.35 (1H, s), 6.96-7.00 (2H, m), 7.17 (1H, t, J = 8.0 Hz), 7.24 (1H, dd, J = 8.0, 1.6 Hz), 7.41-7.43 (2H, m).

### Referential Example 790

2-[4,6-trans-1-(tert-Butyl)-8-chloro-6-(2,3-dimethoxyphenyl)-4H,6H-[1,2,4]triazolo[4,3-a][4,1]benzothiazepin-4-yl]acetic acid

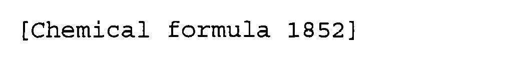

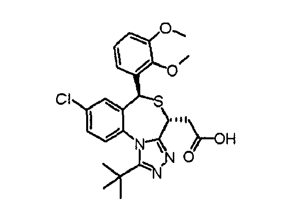

Isopropyl 2-[4,6-trans-1-(tert-butyl)-8-chloro-6-(2,3-dimethoxyphenyl)-4H,6H-[1,2,4]triazolo[4,3-a][4,1]benzothiazepin-4-yl]acetate (99 mg) was dissolved in 1,4-dioxane (5 mL). To the resulting solution was added concentrated hydrochloric acid (0.5 mL). The resulting mixture was stirred at room temperature for 15 hours and then, at 60°C for 8 hours. The reaction mixture was concentrated under reduced pressure. The residue thus obtained was solidified from an ethyl acetate-n-hexane solvent mixture to give the title compound (73 mg). MS (ESI) m/z : 488 (M + H)⁺.
¹H-NMR (CDCl₃) δ: 3.46-3.57 (4H, m), 3.71-3.78 (1H, m), 3.87-3.89 (3H, m), 4.26-4.32 (1H, m), 5.37-5.38 (1H, m), 7.00-7.04 (2H, m), 7.20-7.30 (2H, m), 7.50-7.55 (2H, m). Elemental analysis for: C₂₃H₂₄ClN₃O₄S.·2.25H₂O·0.45CHCl₃ Calculated: C, 50.00; H, 5.16; N, 7.32.
Found: C, 49.53; H, 4.64.30; N, 7.88.

### Referential Example 791

2-[4,6-trans-8-Chloro-6-(2,3-dimethoxyphenyl)-1-isopropyl-4H,6H-[1,2,4]triazolo[4,3-a] [4,1]benzothiazepin-4-yl] acetic acid

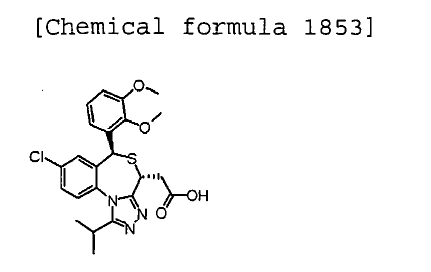

Isopropyl 2-[4,6-trans-8-chloro-6-(2,3-dimethoxyphenyl)-1-isopropyl-4H,6H-[1,2,4]triazolo[4,3-a][4,1]benzothiazepin-4-yl]acetate (94 mg) was dissolved in 1,4-dioxane (5 mL). To the resulting solution was added concentrated hydrochloric acid (0.5 mL). The resulting mixture was stirred at room temperature for 15 hours and then, at 60°C for 8 hours. The reaction mixture was concentrated under reduced pressure. The residue thus obtained was solidified from an ethyl acetate-n-hexane solvent mixture to give the title compound (75 mg).
MS (ESI) m/z : 474 (M + H)⁺.
¹H-NMR (CDCl₃) δ: 3.46-3.57 (4H, m), 3.71-3.78 (1H, m), 3.87-3.89 (3H, m), 4.26-4.32 (1H, m), 5.37-5.38 (1H, m), 7.00-7.04 (2H, m), 7.20-7.30 (2H, m), 7.50-7.55 (2H, m). Elemental analysis for: C₂₃H₂₄ClN₃O₄S.·2.25H₂O·0.45CHCl₃ Calculated: C, 50.00; H, 5.16; N, 7.32.
Found: C, 49.53; H, 4.64.30; N, 7.88.

### Example 883

2-[4,6-trans-8-Chloro-1-[chloro(difluoro)methyl]-6-(2,3-dimethoxyphenyl)-4H,6H-[1,2,4]triazolo[4,3-a] [4,1]benzothiazepin-4-yl]acetic acid

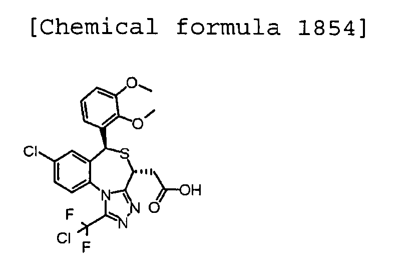

Isopropyl 2-[4,6-trans-8-chlorc-1-[chloro(difluoro)methyl]-6-(2,3-dimethoxyphenyl)-4H,6H-[1,2,4]triazolo[4,3-a][4,1]benzothiazepin-4-yl]acetate (88 mg) was dissolved in 1,4-dioxane (5 mL). To the resulting solution was added concentrated hydrochloric acid (0.5 mL). The resulting mixture was stirred at room temperature for 15 hours and then, at 60°C for one day. The reaction mixture was concentrated under reduced pressure. The residue thus obtained was solidified from an ether-n-hexane solvent mixture to give the title compound (66 mg).
MS (ESI) m/z : 516 (M + H)⁺.
¹H-NMR (CDCl₃) δ: 1.09-1.23 (1H, m), 1.72-1.82 (1H, m), 1.86-1.92 (1H, m), 2.00-2.10 (1H, m), 2.28-2.35 (2H, m), 2.56-2.67 (1H, m), 2.77-2.88 (1H, m), 3.05-3.24 (1H, m), 3.42 (3H, s), 3.61-3.77 (2H, m), 3.85 (3H, s), 4.00 (1H, t, J = 15.4 Hz), 4.46-4.53 (2H, m), 5.32 (1H, s), 6.96-6.99 (2H, m), 7.16 (1H, td, J = 8.1, 1.5 Hz), 7.22-7.25 (1H, m), 7.40-7.42 (2H, m).
Elemental analysis for: C₂₁H₁₇Cl₂F₂N₃O₄S.·0.5H₂O·0.25CHCl₃ Calculated: C, 46.24; H, 3.34; N, 7.52.
Found: C, 46.49; H, 3.30; N, 7.03.

### Referential Example 792

3-(2-{[4,6-trans-1-(tert-Butyl)-8-chloro-6-(2,3-dimethoxyphenyl)-4H,6H-[1,2,4]triazolo[4,3-a] [4,1]benzothiazepin-4-yl]methyl}-1,3-thiazol-5-yl)-N'-(2,2-dimethylpropanoyl)propanohydrazide

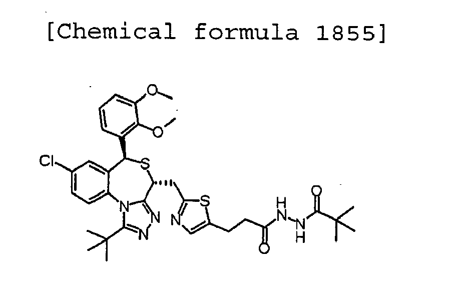

3-(2-{[3,5-trans-7-Chloro-5-(2,3-dimethoxyphenyl)-2-[(Z)hydrazono]-1,5-dihydro-4,1-benzothiazepin-3(3H)-yl]methyl}-1,3-thiazol-5-yl)propanohydrazide (2490 mg) was suspended in toluene (50 mL). To the resulting suspension were added trimethylacetic anhydride (1.87 mL) and pivalic acid (2.32 g). The resulting mixture was stirred at room temperature for 3.5 hours. The reaction mixture was heated under reflux for 2 hours. After cooling to room temperature, the reaction mixture was diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography to give the title compound (2.41 g).
MS (ESI) m/z : 697 (M + H)⁺.
¹H-NMR (CDCl₃) δ: 2.96 (1H, dd, J = 17.6, 5.1 Hz), 3.43 (3H, s), 3.65 (1H, dd, J = 17.6, 9.5 Hz), 3.85 (3H, s), 4.27 (1H, dd, J = 9.5, 5.1 Hz), 5.34 (1H, s), 6.97-7.00 (2H, m), 7.15-7.22 (2H, m), 7.39 (1H, d, J = 8.3 Hz), 7.44 (1H, dd, J = 8.5, 2.2 Hz).

### Example 884

Methyl 3-(2-{[(4R,6S)-1-(tert-butyl)-8-chloro-6-(2,3-dimethoxyphenyl)-4H,6H-[1,2,4]triazolo[4,3-a][4,1]benzothiazepin-4-yl]methyl}-1,3-thiazol-5-yl)propanoate

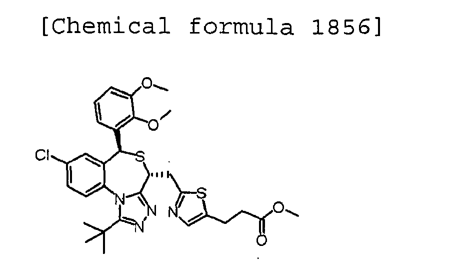

3-(2-{[4,6-trans-1-(tert-Butyl)-8-chloro-6-(2,3-dimethoxyphenyl)-4H,6H-[1,2,4]triazolo[4,3-a][4,1]benzothiazepin-4-yl]methyl}-1,3-thiazol-5-yl)-N'-(2,2-dimethylpropanoyl)propanohydrazide (2.41 g) was dissolved in 1,4-dioxane (50 mL). To the resulting solution was added concentrated hydrochloric acid (50 mL). The resulting mixture was heated under reflux for 3 days. To the reaction mixture was added ethyl acetate. The resulting mixture was washed with saturated brine and then, dried over magnesium sulfate. The solvent was distilled off under reduced pressure. The residue was dissolved in N,N-dimethylformamide (50 mL). To the resulting solution were added potassium carbonate (955 mg) and methyl iodide (1.08 mL). The resulting mixture was stirred at room temperature for 14.5 hours. To the reaction mixture was added ethyl acetate. The resulting mixture was washed with saturated brine and dried over magnesium sulfate. The solvent was distilled off under reduced pressure. After the residue was purified by silica gel chromatography, the racemic mixture thus obtained was separated by HPLC (ChiralPak AD, 5 cm × 50 cm, 50 mL/min., ethanol, 136 min.) to give the title compound (248 mg).
MS (ESI) m/z : 613 (M + H)⁺.
¹H-NMR (CDCl₃) δ: 1.35 (9H, s), 2.57 (2H, t, J = 7.4 Hz), 3.02 (2H, t, J = 7.4 Hz), 3.49-3.58 (4H, m), 3.61 (3H, s), 3.78 (3H, s), 4.00 (1H, dd, J = 15.4, 7.1 Hz), 4.17 (1H, t, J = 7.1 Hz), 5.27 (1H, s), 6.87-6.90 (2H, m), 7.07 (1H, t, J = 8.1 Hz), 7.18 (1H, dd, J = 7.8, 1.2 Hz), 7.27-7.30 (3H, m).

### Example 885

3-(2-{[(4R,6S)-trans-1-(tert-Butyl)-8-chloro-6-(2,3-dimethoxyphenyl)-4H,6H-[1,2,4]triazolo[4,3-a][4,1]benzothiazepin-4-yl]methyl}-1,3-thiazol-5-yl)propanoic acid

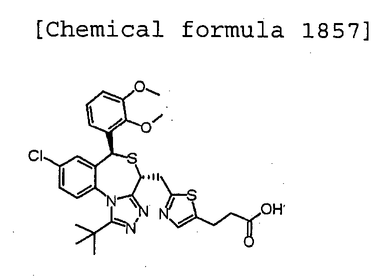

Methyl 3-(2-{[(4R,6S)-1-(tert-butyl)-8-chloro-6-(2,3-dimethoxyphenyl)-4H,6H-[1,2,4]triazolo[4,3-a][4,1]benzothiazepin-4-yl]methyl}-1,3-thiazol-5-yl)propanoate (248 mg) was dissolved in a solvent mixture of tetrahydrofuran (10 mL), methanol (10 mL) and distilled water (10 mL). To the resulting solution was added potassium carbonate (168 mg). The resulting mixture was stirred at room temperature for 12 hours and then, at 50°C for 5 hours. To the reaction mixture was added an ion exchange resin IR-120B to make it acidic. The resin was then filtered out and the solvent was concentrated under reduced pressure. The residue was solidified from an isopropyl ether-n-hexane solvent mixture to give the title compound (175 mg).
MS (ESI) m/z : 599 (M + H)⁺.
¹H-NMR (CDCl₃) δ: 1.42 (9H, s), 2.69 (2H, t, J = 7.2 Hz), 3.11 (2H, t, J = 7.0 Hz), 3.57-3.69 (4H, m), 3.86 (3H, s), 4.09 (1H, dd, J = 15.2, 7.4 Hz), 4.26 (1H, t, J = 7.2 Hz), 5.33 (1H, s), 6.93-6.97 (2H, m), 7.15 (1H, t, J = 8.0 Hz), 7.23-7.26 (1H, m), 7.34-7.36 (2H, m), 7.39-7.41 (1H, m). Elemental analysis for: C₂₉H₃₁ClN₄O₄S₂·0.5H₂O·0.25 isopropyl ether
Calculated: C, 57.81; H, 5.65; N, 8.84.
Found: C, 57.70; H, 5.42; N, 8.58.

### Example 886

tert-butyl 2-({2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}amino)acetate

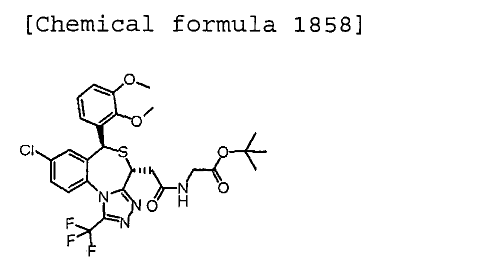

To a solution of 2-[(4R,6S)-8-chloro-6-(2,3-dimethoxyphenyl)-1-(trifluoromethyl)-4H,6H-[1,2,4]triazolo[4,3-a] [4,1]benzothiazepin-4-yl]acetic acid (541 mg) in dichloromethane (10 mL) were added glycine tert-butyl ester hydrochloride (181 mg), 1-ethyl-3-(3-dimethylaminopropyl)carbodiimide hydrochloride (415 mg), 1-hydroxybenzotriazole monohydrate (166 mg) and triethylamine (0.151 mL). The resulting mixture was stirred at room temperature for 12.5 hours. The reaction mixture was concentrated. The residue was purified by silica gel chromatography (Automatic purification system of Yamazen Corporation, ethyl acetate:n-hexane=1:2, Rf=0.59) to give the title compound (418 mg).
MS (ESI) m/z : 613 (M + H)⁺.
¹H-NMR (CDCl₃) δ: 1.45 (9H, s), 2.83 (1H, dd, J = 15.1, 5.1 Hz), 3.42 (3H, s), 3.49 (1H, dd, J = 15.1, 9.2 Hz), 3.84-3.91 (5H, m), 4.40 (1H, dd, J = 9.2, 5.1 Hz), 5.34 (1H, s), 6.96-7.00 (2H, m), 7.16 (1H, t, J = 8.1 Hz), 7.21-7.24 (1H, m), 7.35-7.38 (1H, m), 7.40-7.44 (1H, m).

### Example 887

2-({2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}amino)acetic acid

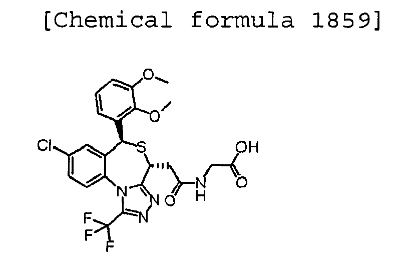

tert-Butyl 2-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)acetate (418 mg) was dissolved in methylene chloride (5 mL). To the resulting solution was added trifluoroacetic acid (1 mL). The resulting mixture was stirred at room temperature for 4.2 hours. The reaction mixture was concentrated under reduced pressure. The residue thus obtained was solidified from a solvent mixture of methylene chloride and n-hexane to give the title compound (266 mg).
MS (ESI) m/z : 557 (M + H)⁺.
¹H-NMR (CDCl₃) δ: 2.86 (1H, dd, J = 15.2, 4.7 Hz), 3.42 (3H, s), 3.58 (1H, dd, J = 15.2, 10.0 Hz), 3.85 (3H, s), 3.97-4.14 (2H, m), 4.43 (1H, dd, J = 10.0, 4.6 Hz), 5.34 (1H, s), 6.97-7.02 (2H, m), 7.15-7.24 (2H, m), 7.38-7.46 (2H, m).
Elemental analysis for: C₂₃H₂₀ClF₃N₄O₅S
Calculated: C, 49.60; H, 3.62; N, 10.06.
Found: C, 49.51; H, 3.61; N, 9.69.

### Example 888

Ethyl 3-(4-{2-[(4R,6S)-8-chloro-6-(2,3-dimethoxyphenyl)-1-(trifluoromethyl)-4H,6H-[1,2,4]triazolo[4,3-a] [4,1]benzothiazepin-4-yl]acetyl}-2-oxo-1-piperazinyl)propanoate

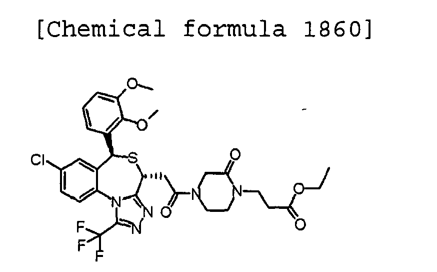

2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetic acid (50 mg) was dissolved in methylene chloride (5 mL). To the resulting solution were added 1-ethyl-3-(3-dimethylaminopropyl)carbodiimide hydrochloride (38 mg), ethyl 2-oxopiperazinepropionate hydrochloride (47 mg), 1-hydroxybenzotriazole monohydrate (15 mg) and triethylamine (28 µl). The resulting mixture was stirred at room temperature for 18 hours. The reaction mixture was purified by preparative thin layer silica gel column chromatography (ethyl acetate) to give the title compound (63 mg).
MS (ESI) m/z : 682 (M + H)⁺.
¹H-NMR (CDCl₃) δ: 1.27 (3H, t, J = 7.2 Hz), 2.64-2.68 (2H, m), 2.72-2.82 (1H, m), 3.41-3.47 (4H, m), 3.62-3.78 (5H, m), 3.85 (3H, s), 4.09-4.39 (5H, m), 4.43-4.52 (1H, m), 5.33-5.35 (1H, m), 6.97-7.00 (2H, m), 7.15-7.24 (2H, m), 7.38-7.45 (2H, m).

### Example 889

3-(4-{2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}-2-oxo-1-piperazinyl)propanoic acid

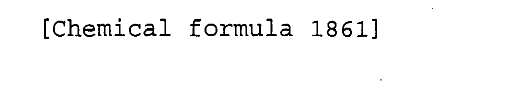

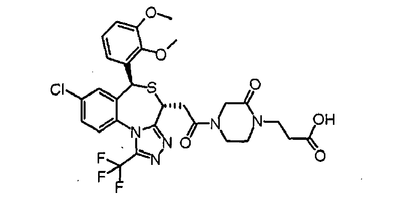

Ethyl 3-(4-{2-[(4R,6S)-8-chloro-6-(2,3-dimethoxyphenyl)-1-(trifluoromethyl)-4H,6H-[1,2,4]triazolo[4,3-a] [4,1]benzothiazepin-4-yl]acetyl}-2-oxo-1-piperazinyl)propanoate (60 mg) was dissolved in 1,4-dioxane (4 mL). To the resulting solution was added concentrated hydrochloric acid (1 mL). The resulting mixture was stirred at room temperature for 16 hours. The reaction mixture was distilled under reduced pressure to remove the solvent. The residue was purified by preparative thin layer chromatography on silica gel (chloroform:methanol:water=7:3:1 organic layer) to give the title compound (51 mg).
MS (ESI) m/z : 654 (M + H)⁺.
¹H-NMR (CDCl₃) δ: 2.60-2.83 (3H, m), 3.40-3.76 (7H, m), 3.77-4.20 (6H, m), 4.32-4.52 (2H, m), 5.32-5.35 (1H, m), 6.96-7.01 (2H, m), 7.14-7.24 (2H, m), 7.38-7.46 (2H, m). Elemental analysis for: C₂₉H₃₁ClN₄O₄S₂·0.5H₂O·0.25 1,4-dioxane
Calculated: C, 50.51; H, 4.46; N, 10.16.
Found: C, 50.72; H, 4.35; N, 9.76.

### Example 890

Ethyl 3-{2-[-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl]-3-azabicyclo[3.1.0]hexane-1-carboxylate

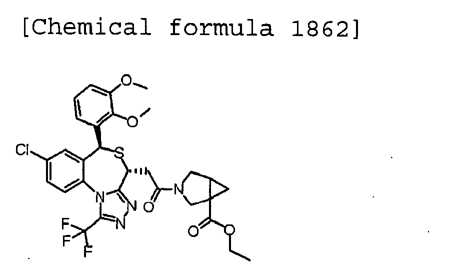

2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetic acid (50 mg) was dissolved in methylene chloride (5 mL). To the resulting solution were added 1-ethyl-3-(3-dimethylaminopropyl)carbodiimide hydrochloride (38 mg), ethyl 3-azabicyclo[3.1.0]hexane-1-carboxylate (39 mg), and 1-hydroxybenzotriazole monohydrate (15 mg). The resulting mixture was stirred at room temperature for 18 hours. The reaction mixture was purified by preparative thin layer silica gel column chromatography (ethyl acetate:n-hexane=1:2) to give the title compound (60 mg).
MS (ESI) m/z : 638 (M + H)⁺.
¹H-NMR (CDCl₃) δ: 0.78-0.98 (2H, m), 1.22-1.33 (3H, m), 1.62-1.69 (1H, m), 2.08-2.21 (1H, m), 2.63-2.81 (1H, m), 3.40-3.56 (4H, m), 3.64-3.91 (5H, m), 3.97-4.28 (3H, m), 4.44-4.50 (1H, m), 5.33 (1H, s), 6.96-6.99 (2H, m), 7.14-7.24 (2H, m), 7.36-7.44 (2H, m).

### Example 891

3-{2-[(4R,6S)-8-Chloro-6-(2,3-dimethoxyphenyl)-1-(trifluoromethyl)-4H,6H-[1,2,4]triazolo[4,3-a] [4,1]benzothiazepin-4-yl]acetyl}-3-azabicyclo[3.1.0]hexane-1-carboxylic acid

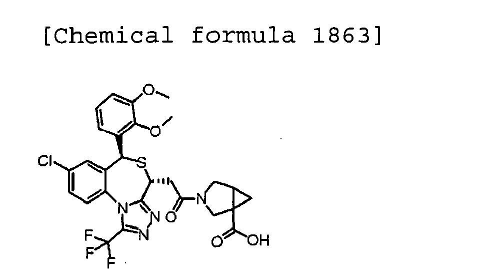

Ethyl 3-{2-[-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl]-3-azabicyclo[3.1.0]hexane-1-carboxylate (60 mg) was dissolved in 1,4-dioxane (4 mL). To the resulting solution was added concentrated hydrochloric acid (1 mL). The resulting mixture was stirred at room temperature for 5 days. The reaction mixture was distilled under reduced pressure to remove the solvent. The residue was purified by preparative thin layer chromatography on silica gel (chloroform:methanol:water=7:3:1 organic layer) to give the title compound (37 mg).
MS (ESI) m/z : 609 (M + H)⁺.
¹H-NMR (CDCl₃) δ: 0.81-0.93 (1H, m), 1.22-1.34 (1H, m), 1.70-1.78 (1H, m), 2.15-2.30 (1H, m), 2.62-2.83 (1H, m), 3.40-3.92 (9H, m), 4.00-4.29 (1H, m), 4.43-4.50 (1H, m), 5.32-5.34 (1H, m), 6.96-6.99 (2H, m), 7.14-7.24 (2H, m), 7.36-7.44 (2H, m).
Elemental analysis for: C₂₇H₂₄ClF₃N₄O₅S·0.25 1,4-dioxane
Calculated: C, 53.29; H, 4.15; N, 8.88.
Found: C, 52.90; H, 4.42; N, 8.19.

### Referential Example 793

Methyl 2-(3,5-trans-5-(2,3-dimethoxyphenyl)-2-oxo-7-(trifluoromethyl)-1,2,3,5-tetrahydro-4,1-benzothiazepin-3-yl)acetate

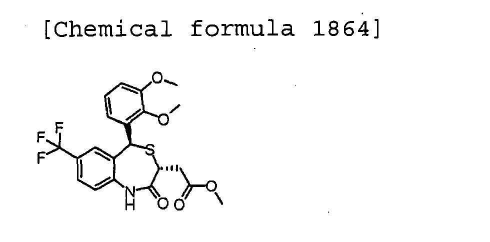

[2-Amino-5-(trifluoromethyl)phenyl](2,3-dimethoxyphenyl)methanol (1257 mg) and thiomalic acid (634 mg) were dissolved in dichloroethane (20 mL). To the resulting solution was added p-toluenesulfonic acid monohydrate (877 mg). The resulting mixture was stirred at 70°C for one hour. The reaction mixture was cooled and ethyl acetate was added thereto. The resulting mixture was washed with saturated brine. The organic layer was dried over anhydrous magnesium sulfate and concentrated.
The residue thus obtained was dissolved in toluene (50 mL) and the resulting solution was heated under reflux for 63 hours. The reaction mixture was concentrated.
The residue thus obtained was dissolved in N,N-dimethylformamide (20 mL). To the resulting solution were added potassium carbonate (531 mg) and methyl iodide (0.717 mL). The resulting mixture was stirred at room temperature for 6 hours. Ethyl acetate was added and the resulting mixture was washed with saturated brine. The organic layer was dried over anhydrous magnesium sulfate and concentrated.
The residue thus obtained was dissolved in methanol (50 mL). Potassium carbonate (531 mg) was added and the resulting mixture was stirred at room temperature for 17 hours. The reaction mixture was concentrated. The residue was diluted with ethyl acetate and washed with a 10% aqueous solution of citric acid. The organic layer was dried over anhydrous sodium sulfate. After concentration of the filtrate, the residue thus obtained was purified by silica gel chromatography (Automatic purification system of Yamazen Corporation, 10%MeOH-CHCl3, Rf=0.52) to give the title compound (481 mg).
MS (ESI) m/z : 456 (M + H)⁺.
¹H-NMR (CDCl₃) δ: 2.41-2.47 (1H, m), 3.13-3.23 (1H, m), 3.55 (3H, s), 3.67 (3H, s), 3.82-3.88 (4H, m), 6.00 (1H, s), 6.97-7.00 (1H, m), 7.08 (1H, t, J = 8.2 Hz), 7.18 (1H, t, J = 7.9 Hz), 7.41-7.44 (1H, m), 7.50-7.61 (2H, m).

### Referential Example 794

Methyl 2-[3,5-trans-5-(2,3-dimethoxyphenyl)-2-thioxo-7-(trifluoromethyl)-1,2,3,5-tetrahydro-4,1-benzothiazepin-3-yl] acetate

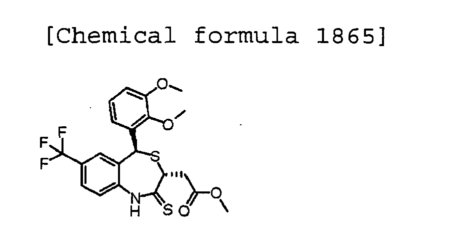

Methyl 2-(3,5-trans-5-(2,3-dimethoxyphenyl)-2-oxo-7-(trifluoromethyl)-1,2,3,5-tetrahydro-4,1-benzothiazepin-3-yl)acetate (481 mg) was dissolved in toluene (10 mL). To the resulting solution was added Lawesson's reagent (440 mg) and the resulting mixture was stirred at 60°C for 14 hours. The reaction mixture was cooled to room temperature and purified by silica gel column (ethyl acetate:hexane=1:2) to give the title compound (368 mg).
MS (ESI) m/z : 472 (M + H)⁺.
¹H-NMR (CDCl₃) δ: 2.63 (1H, dd, J = 17.0, 3.8 Hz), 3.45 (1H, dd, J = 17.0, 10.0 Hz), 3.58 (3H, s), 3.67 (3H, s), 3.87 (3H, s), 4.05 (1H, dd, J = 10.0, 3.8 Hz), 6.10 (1H, s), 6.99 (1H, dd, J = 8.1, 1.5 Hz), 7.14-7.16 (1H, m), 7.19 (1H, t, J = 8.1 Hz), 7.27-7.30 (1H, m), 7.56-7.60 (1H, m), 9.31 (1H, br s).

### Referential Example 795

Methyl 2-[4,6-trans-1-(tert-butyl)-6-(2,3-dimethoxyphenyl)-8-(trifluoromethyl)-4H,6H-[1,2,4]triazolo[4,3-a][4,1]benzothiazepin-4-yl]acetate

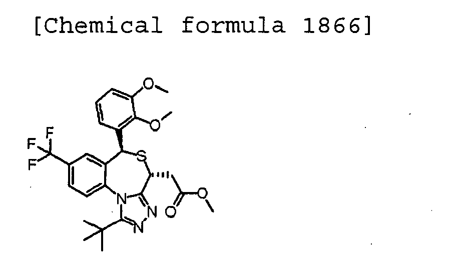

Methyl 2-[3,5-trans-5-(2,3-dimethoxyphenyl)-2-thioxo-7-(trifluoromethyl)-1,2,3,5-tetrahydro-4,1-benzothiazepin-3-yl]acetate (112 mg) was dissolved in tetrahydrofuran (2 mL). To the resulting solution was added hydrazine monohydrate (0.014 mL). The resulting mixture was stirred at room temperature for 1.6 hours. To the reaction mixture was added toluene (2 mL), followed by the addition of trimethylacetic anhydride (0.146 mL) and pivalic acid (49 mg). The resulting mixture was stirred at room temperature for one hour and then heated under reflux for 21 hours. After cooling to room temperature, the reaction mixture was diluted with ethyl acetate and washed with a saturated aqueous solution of sodium bicarbonate and saturated brine. The organic layer was dried over anhydrous sodium sulfate. After concentration of the filtrate, the residue thus obtained was purified by preparative thin layer chromatography on silica gel (ethyl acetate:n-hexane=2:1) to give the title compound (65 mg).
MS (ESI) m/z : 536 (M + H)⁺.
¹H-NMR (CDCl₃) δ: 1.42 (9H, s), 2.88 (1H, dd, J = 17.1, 5.9 Hz), 3.56-3.62 (4H, m), 3.69 (4H, s), 3.86 (3H, s), 4.10-4.15 (1H, m), 5.42 (1H, s), 6.99 (1H, dd, J = 8.3, 1.5 Hz), 7.18 (1H, t, J = 8.0 Hz), 7.26-7.31 (2H, m), 7.60 (1H, d, J = 8.3 Hz), 7.66-7.70 (1H, m).

### Referential Example 796

2-[4,6-trans-1-(tert-Butyl)-6-(2,3-dimethoxyphenyl)-8-(trifluoromethyl)-4H,6H-[1,2,4]triazolo[4,3-a][4,1]benzothiazepin-4-yl]acetic acid

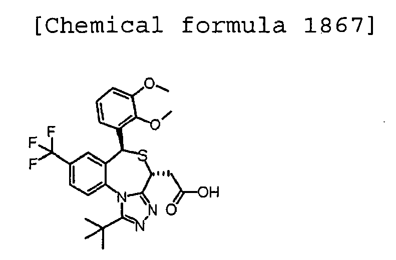

Methyl 2-[4,6-trans-1-(tert-butyl)-6-(2,3-dimethoxyphenyl)-8-(trifluoromethyl)-4H,6H-[1,2,4]triazolo[4,3-a] [4,1] benzothiazepin-4-yl]acetate (60 mg) was dissolved in 1,4-dioxane (2 mL). To the resulting solution were added concentrated hydrochloric acid (1 mL) and distilled water (1 mL). The resulting mixture was stirred at 60°C for 2.5 hours. The reaction mixture was distilled under reduced pressure to remove the solvent.
The residue was purified by preparative thin layer chromatography on silica gel (chloroform:methanol:water=7:3:1 organic layer) to give the title compound (38 mg).
MS (ESI) m/z : 522 (M + H)⁺.
¹H-NMR (CDCl₃) δ: 1.40 (9H, s), 2.84-3.04 (1H, m), 3.39-3.60 (4H, m), 3.86 (3H, s), 4.05-4.14 (1H, m), 5.40 (1H, s), 6.97-7.01 (1H, m), 7.16-7.31 (3H, m), 7.61-7.73 (2H, m) .
Elemental analysis for: C₂₅H₂₆F₃N₃O4S·0.25 1,4-dioxane
Calculated: C, 55.61; H, 5.38; N, 7.48.
Found: C, 55.91; H, 5.14; N, 7.32.

### Example 892

Methyl 2-[trans-6-(2,3-dimethoxyphenyl)-1,8-bis(trifluoromethyl)-4H,6H-[1,2,4]triazolo[4,3-a] [4,1]benzothiazepin-4-yl]acetate

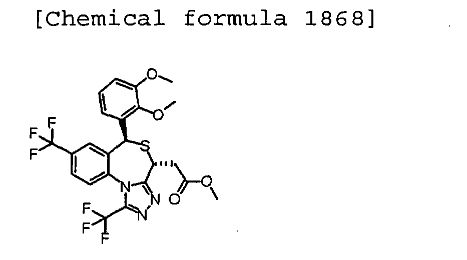

Methyl 2-[3,5-trans-5-(2,3-dimethoxyphenyl)-2-thioxo-7-(trifluoromethyl)-1,2,3,5-tetrahydro-4,1-benzothiazepin-3-yl]acetate (216 mg) was dissolved in tetrahydrofuran (5 mL). To the resulting solution was added hydrazine monohydrate (0.027 mL). The resulting mixture was stirred at room temperature for 3.1 hours. To the reaction mixture was added toluene (5 mL), followed by the addition of trimethylacetic anhydride (0.146 mL) and pivalic acid (49 mg). The resulting mixture was stirred at room temperature for 2 hours and then heated under reflux for 18 hours. The reaction mixture was cooled to room temperature, diluted with ethyl acetate and washed with a saturated aqueous solution of sodium bicarbonate and saturated brine. The organic layer was dried over anhydrous sodium sulfate. After concentration of the filtrate, the residue thus obtained was purified by silica gel chromatography (ethyl acetate:n-hexane=1:1) to give the title compound (121 mg). MS (ESI) m/z : 548 (M + H)⁺.
¹H-NMR (CDCl₃) δ: 2.93 (1H, dd, J = 17.3, 5.4 Hz), 3.40 (3H, s), 3.65 (1H, dd, J = 17.3, 9.0 Hz), 3.71 (3H, s), 3.85 (3H, s), 4.29 (1H, dd, J = 9.0, 5.4 Hz), 5.41 (1H, s), 6.97-7.01 (1H, m), 7.18 (1H, t, J = 7.9 Hz), 7.21-7.24 (2H, m), 7.28-7.29 (1H, m), 7.57-7.61 (1H, m), 7.72-7.76 (1H, m).

### Example 893

2-[trans-6-(2,3-Dimethoxyphenyl)-1,8-bis(trifluoromethyl)-4H,6H-[1,2,4]triazolo[4,3-a][4,1]benzothiazepin-4-yl]acetic acid

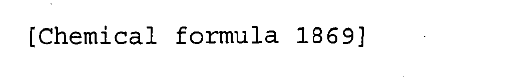

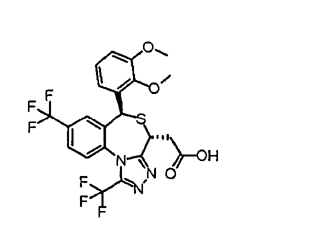

Methyl 2-[trans-6-(2,3-dimethoxyphenyl)-1,8-bis(trifluoromethyl)-4H,6H-[1,2,4]triazolo[4,3-a][4,1]benzothiazepin-4-yl]acetate(121 mg) was dissolved in 1,4-dioxane (2 mL). To the resulting solution were added concentrated hydrochloric acid (1 mL) and distilled water (1 mL). The resulting mixture was stirred at 60°C for 2.5 hours. The reaction mixture was distilled under reduced pressure to remove the solvent. The residue was purified by preparative thin layer chromatography on silica gel (chloroform:methanol:water=7:3:1 organic layer) to give the title compound (103 mg).
MS (ESI) m/z : 522 (M + H)⁺.
¹H-NMR (CDCl₃) δ: 2.85-3.02 (1H, m), 3.39 (3H, s), 3.49-3.62 (1H, m), 3.85 (3H, s), 4.22-4.28 (1H, m), 5.40 (1H, s), 6.97-7.01 (1H, m), 7.15-7.29 (3H, m), 7.58-7.63 (1H, m), 7.69-7.74 (1H, m).
Elemental analysis for: C₂₂H₁₇F₆N₃O₄S·0.25CHCl₃·0.25 1,4-dioxane
Calculated: C, 47.96; H, 3.34; N, 7.14.
Found: C, 47.94; H, 3.18; N, 7.14.

### Referential Example 797

tert-Butyl (E)-3-(6-amino-3-pyridinyl)-2-propenoate

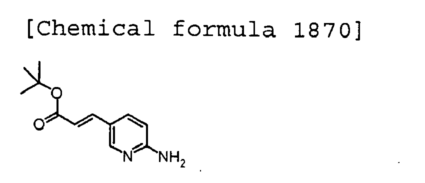

2-Amino-5-bromopyridine (2.25 g) and tert-butyl acrylate (3.05 mL) were dissolved in propionitrile (50 mL). To the resulting solution were added palladium(II) acetate (321 mg), tri-o-trylphosphine (752 mg) and diisopropylethylamine (3.51 mL). In a nitrogen atmosphere, the resulting mixture was heated under reflux for 19 hours. After cooling, the reaction mixture was filtered through celite to remove the insoluble material, followed by concentration under reduced pressure. The residue thus obtained was purified by silica gel chromatography (Automatic purification system of Yamazen Corporation, ethyl acetate:n-hexane=1:1, Rf=0.1) to give title compound (2.35 g).
MS (ESI) m/z : 221 (M + H)⁺.
¹H-NMR (CDCl₃) δ: 1.52 (9H, s), 4.80 (2H, br s), 6.19 (1H, d, J = 15.9 Hz), 6.49 (1H, d, J = 8.5 Hz), 7.47 (1H, d, J = 15.9 Hz), 7.62 (1H, dd, J = 8.5, 2.3 Hz), 8.16 (1H, d, J = 2.3 Hz) .

### Referential Example 798

tert-Butyl 3-(6-amino-3-pyridinyl)propanoate

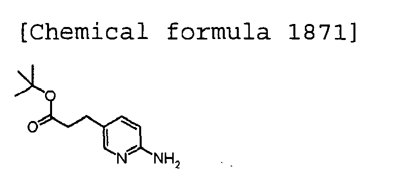

tert-Butyl (E)-3-(6-amino-3-pyridinyl)-2-propenoate (604 mg) was dissolved in a solvent mixture of ethanol (10 mL) and tetrahydrofuran (10 mL). To the resulting solution were added ammonium formate (1.73 g) and 10% pd-C (274 mg). The resulting mixture was heated under reflux for 6.2 hours. The insoluble material was filtered out, followed by concentration. The residue was diluted with chloroform and dried over anhydrous sodium sulfate. The solvent was distilled off to give the title compound (600 mg).
MS (ESI) m/z : 223 (M + H)⁺.

### Example 894

tert-Butyl 3-[6-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)-3-pyridinyl]propanoate

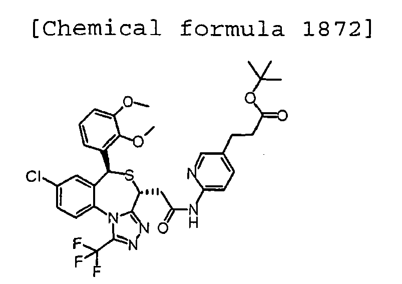

2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetic acid (150 mg) was dissolved in methylene chloride (5 mL). To the resulting solution were added 1-ethyl-3-(3-dimethylaminopropyl)carbodiimide hydrochloride (115 mg), tert-butyl 3-(6-amino-3-pyridinyl)propanoate (56 mg), and 1-hydroxybenzotriazole monohydrate (46 mg). The resulting mixture was stirred at room temperature for 3 days. The reaction mixture was purified by preparative thin layer silica gel column chromatography (ethyl acetate:n-hexane=1:1) to give the title compound (100 mg).
MS (ESI) m/z : 223 (M + H)⁺.
¹H-NMR (CDCl₃) δ: 1.41 (9H, s), 2.51 (2H, t, J = 7.6 Hz), 2.86 (2H, t, J = 7.6 Hz), 3.00 (1H, dd, J = 15.6, 5.4 Hz), 3.64 (1H, dd, J = 15.6, 8.9 Hz), 3.85 (3H, s), 4.47 (1H, dd, J = 8.9, 5.4 Hz), 5.37 (1H, s), 6.95-7.01 (2H, m), 7.16 (1H, t, J = 7.9 Hz), 7.20-7.23 (1H, m), 7.36-7.45 (2H, m), 7.51 (1H, dd, J = 8.5, 2.2 Hz), 7.98 (1H, d, J = 8.5 Hz), 8.14 (1H, d, J = 2.2 Hz), 8.37 (1H, br s).

### Example 895

3-[6-({2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}amino)-3-pyridinyl]propanoic acid

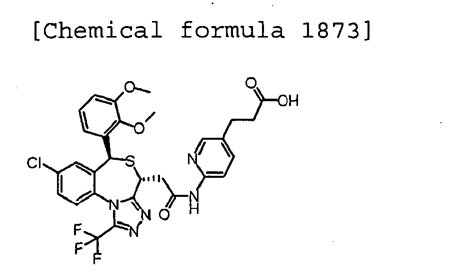

tert-Butyl 3-[6-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)-3-pyridinyl]propanoate (100 mg) was dissolved in methylene chloride (10 mL). To the resulting solution was added trifluoroacetic acid (1 mL). The resulting mixture was stirred at room temperature for 4 hours. The reaction mixture was concentrated under reduced pressure to give the title compound (100 mg).
MS (ESI) m/z : 648 (M + H)⁺. ¹H-NMR (CDCl₃) δ: 2.68-2.76 (2H, m), 2.95-3.02 (2H, m), 3.12 (1H, dd, J = 16.8, 5.0 Hz), 3.44 (3H, s), 3.78-3.88 (4H, m), 4.43 (1H, dd, J = 9.4, 5.0 Hz), 5.35 (1H, s), 6.96-7.01 (2H, m), 7.14-7.23 (2H, m), 7.38-7.48 (2H, m), 7.92 (1H, dd, J = 8.9, 2.1 Hz), 8.18-8.23 (1H, m), 8.38 (1H, d, J = 8.8 Hz).
Elemental analysis for: C₂₉H₂₅ClF₃N₅O₅S·0.75 CHCl₃
Calculated: C, 49.02; H, 3.57; N, 9.37.
Found: C, 49.26; H, 3.67; N, 9.00.

### Example 896

Ethyl 1-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)cyclobutane carboxylate

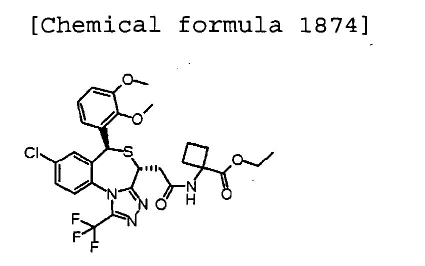

Ethyl 1-[(tert-butoxycarbonyl)amino]cyclobutane carboxylate (43 mg) was dissolved in 4N hydrochloric acid-dioxane. The resulting mixture was stirred at room temperature for 2.5 hours. The reaction mixture was concentrated under reduced pressure. The residue was dissolved in methylene chloride (5 mL). To the resulting solution were added 2-[(4R,6S)-8-chloro-6-(2,3-dimethoxyphenyl)-1-(trifluoromethyl)-4H,6H-[1,2,4]triazolo[4,3-a] [4,1]benzothiazepin-4-yl]acetic acid (100 mg), 1-ethyl-3-(3-dimethylaminopropyl)carbodiimide hydrochloride (77 mg), 1-hydroxybenzotriazole monohydrate (31 mg), and triethylamine (0.056 mL). The resulting mixture was stirred at room temperature for 16 hours. The reaction mixture was purified by preparative thin layer chromatography (ethyl acetate:n-hexane=1:1) to give the title compound (145 mg).
MS (ESI) m/z : 625 (M + H)⁺.
¹H-NMR (CDCl₃) δ: 1.21-1.29 (3H, m), 1.98-2.08 (2H, m), 2.27-2.41 (2H, m), 2.56-2.66 (1H, m), 2.81-3.00 (1H, m), 3.41-3.89 (8H, m), 4.12-4.21 (2H, m), 4.37-4.45 (1H, m), 5.32-5.41 (1H, m), 6.95-7.03 (2H, m), 7.13-7.23 (2H, m), 7.36-7.46 (2H, m).

### Example 897

1-({2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]acetyl}amino)cyclobutane carboxylic acid

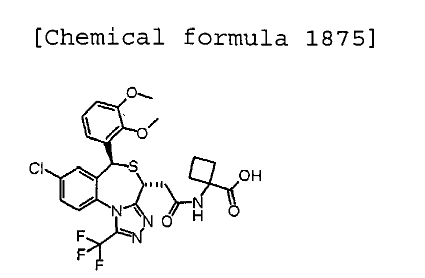

Ethyl 1-({2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetyl}amino)cyclobutane carboxylate (145 mg) was dissolved in 1,4-dioxane (5 mL). To the resulting solution was added concentrated hydrochloric acid (1 mL). The resulting mixture was stirred at 50°C for 20 hours. The reaction mixture was concentrated under reduced pressure.
The residue was purified by preparative thin layer chromatography on silica gel (chloroform:methanol:water=7:3:1 organic layer) to give the title compound (23 mg).
MS (ESI) m/z : 597 (M + H)⁺.
¹H-NMR (CDCl₃) δ: 1.77-2.98 (10H, m), 3.36-3.55 (4H, m), 3.83 (3H, s), 4.30-4.38 (1H, m), 5.31 (1H, s), 6.90-6.99 (2H, m), 7.10-7.22 (2H, m), 7.34-7.43 (2H, m).
Elemental analysis for: C₂₆H₂₄ClF₃N₄O₅S·0.25 1,4-dioxane·0.5CHCl₃
Calculated: C, 49.08; H, 3.97; N, 8.18.
Found: C, 48.87; H, 4.23; N, 7.94.

### Referential Example 799

tert-Butyl 5-chloro-2-pyridinylcarbamate

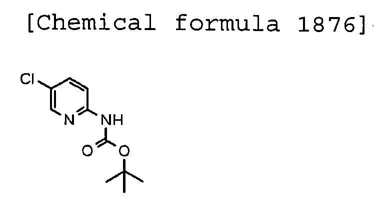

2-Amino-5-chloropyridine (25.0 g) was dissolved in t-butanol. To the resulting solution was added di-t-butyl dicarbonate (55.2 g) and the resulting mixture was stirred under heat at 60°C. The reaction mixture was concentrated under reduced pressure. The residue was purified by silica gel column chromatography (chloroform) to give the title compound (21.6 g).
MS (FAB) m/z: 229 (M+H)⁺.
¹H-NMR (CDCl3) δ: 1.54 (9H, s), 7.62 (1H, dd, J = 9.0, 2.6 Hz), 7.96 (1H, d, J = 9.0 Hz), 8.20 (1H, s), 8.24 (1H, d, J = 2.6 Hz).

### Referential Example 800

tert-Butyl 5-chloro-3-[(2,3-dimethoxyphenyl)(hydroxy)methyl]-2-pyridinylcarbamate

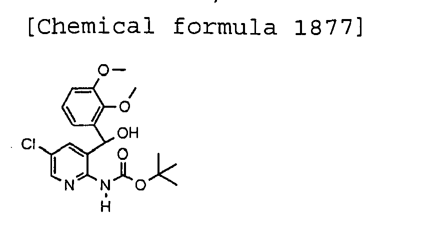

tert-Butyl 5-chloro-2-pyridinylcarbamate (19.3 g) was dissolved in anhydrous tetrahydrofuran (200 mL). To the resulting solution was added dropwise sec-butyl lithium (a 0.95M solution, 197 mL) at -78°C. After stirring at -40°C for one hour, an anhydrous tetrahydrofuran solution (50 mL) of 2,3-dimethoxybenzaldehyde (15.5 g) was added dropwise at -40°C. After the reaction mixture was warmed to -10°C, a saturated aqueous solution of ammonium chloride was added to terminate the reaction. Then, ethyl acetate was added and the layers separated. The organic layer was dried over anhydrous sodium sulfate and the solvent was distilled off under reduced pressure. The solid thus precipitated was subjected to slurry washing with hexane-ethyl acetate (hexane:ethyl acetate=4:1) to give the title compound (25.2 g).
MS (FAB) m/z: 395 (M+H)⁺.
¹H-NMR (CDCl3) δ: 1.52 (9H, s), 3.65 (3H, s), 3.86 (3H, s), 4.02 (1H, br s), 6.02 (1H, s), 6.89-6.94 (1H, m), 7.10-7.14 (2H, m), 7.43 (1H, d, J = 2.4 Hz), 7.89 (1H, s), 8.27 (1H, d, J = 2.4 Hz).

### Referential Example 801

2-Amino-5-chloro-3-[(2,3-dimethoxyphenyl)(hydroxy)methyl]pyridine

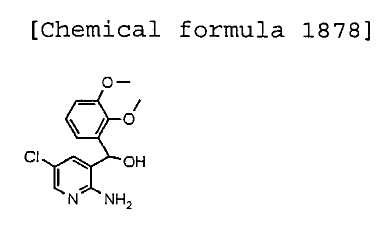

tert-Butyl 5-chloro-3-[(2,3-dimethoxyphenyl)(hydroxy)methyl]-2-pyridinylcarbamate (1.50 g) was dissolved in 1,4-dioxane (9 mL). To the resulting solution were added water (2 mL) and concentrated hydrochloric acid (4 mL). The resulting mixture was stirred at room temperature for 9 hours. Under ice cooling, a 35% aqueous sodium hydroxide solution (6 mL) was added, followed by extraction with chloroform. The resulting organic layer was dried over anhydrous sodium sulfate. The solvent was distilled off under reduced pressure to give the title compound (1.40 g).
MS (FAB) m/z: 295 (M+H)⁺.
¹H-NMR (CDCl3) δ: 3.85 (3H, s), 3.89 (3H, s), 5.09 (2H, br s), 5.92 (1H, s), 6.87 (1H, d, J = 7.8 Hz), 6.92 (1H, d, J = 7.8 Hz), 7.09 (1H, t, J = 7.8 Hz), 7.34 (1H, d, J = 2.2 Hz), 7.89 (1H, d, J = 2.2 Hz).

### Referential Example 802

2-[trans-7-Chloro-5-(2,3-dimethoxyphenyl)-2-oxo-1,2,3,5-tetrahydropyrido[2,3-e][1,4]thiazepin-3-yl]acetic acid

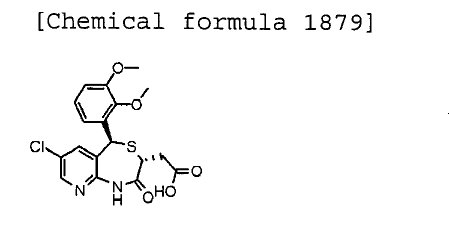

2-Amino-5-chloro-3-[(2,3-dimethoxyphenyl)(hydroxy)methyl]pyridine (3.72 g) was dissolved in dichloroethane (150 mL). To the resulting solution were added tosic acid hydrate (1.99 g) and thiomalic acid (1.99 g). The resulting mixture was stirred at 80°C for 24 hours. After the reaction mixture was cooled to room temperature, a 1N aqueous sodium hydroxide solution (21 mL), water (150 mL), acetic acid (1 mL) and tetrahydrofuran (20 mL) were added. After confirmation that the water layer had a pH of from 3 to 4, the layers were separated. The organic layer was dried over anhydrous sodium sulfate. The solvent was distilled off under reduced pressure. The residue was suspended in xylene (100 mL). To the resulting suspension was added N,N-dimethylformamide (8 mL). The resulting mixture was heated under reflux overnight. The reaction mixture was concentrated under reduced pressure and ethyl acetate was added to the residue. The solid thus precipitated was collected by filtration to give the title compound (1.43 g).
MS (FAB) m/z: 409 (M+H)⁺.
¹H-NMR (CDCl3) δ: 2.43 (1H, dd, J = 17.0, 3.6 Hz), 2.79 (1H, dd, J = 17.0, 10.5 Hz), 3.50 (3H, s), 3.64 (1H, dd, J = 10.5, 3.6 Hz), 3.84 (3H, s), 5.93 (1H, s), 7.12 (1H, d, J = 2.2 Hz), 7.17 (1H, dd, J = 6.8, 2.5 Hz), 7.24-7.30 (2H, m), 8.46 (1H, d, J = 2.2 Hz), 10.59 (1H, s), 12.48 (1H, s).

### Referential Example 803

Methyl 5-({2-[trans-7-chloro-5-(2,3-dimethoxyphenyl)-2-oxo-1,2,3,5-tetrahydropyrido[2,3-e][1,4]thiazepin-3-yl]acetyl}amino)-4-oxopentanoate

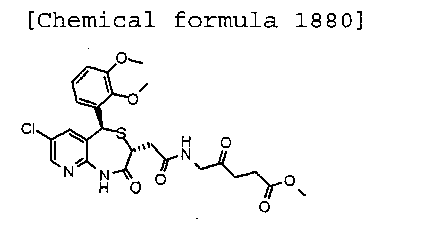

In a similar manner to that employed for the synthesis of methyl 5-({3-[(trans)-8-chloro-1-cyclopropyl-6-(2,3-dimethoxyphenyl)-4H,6H-[1,2,4]triazolo[4,3-a][4,1]benzothiazepin-4-yl]propanoyl}amino)-4-oxopentanoate, the title compound (887 mg) was obtained from 2-[trans-7-chloro-5-(2,3-dimethoxyphenyl)-2-oxo-1,2,3,5-tetrahydropyrido[2,3-e] [1,4]thiazepin-3-yl]acetic acid (800 mg).
MS (EI) m/z: 535 M⁺.
¹H-NMR (CDCl3) δ: 2.42 (1H, dd, J = 14.9, 4.5 Hz), 2.63-2.68 (2H, m), 2.71-2.75 (2H, m), 3.00 (1H, dd, J = 14.9, 9.2 Hz), 3.64 (3H, s), 3.68 (3H, s), 3.88 (3H, s), 3.93 (1H, dd, J = 9.2, 4.5 Hz), 4.13 (1H, dd, J = 19.6, 4.4 Hz), 4.20 (1H, dd, J = 19.6, 4.4 Hz), 6.12 (1H, s), 6.54 (1H, br s), 6.98 (1H, dd, J = 8.1, 1.5 Hz), 7.16-7.21 (2H, m), 7.31 (1H, dd, J = 8.1, 1.5 Hz), 7.81 (1H, s), 8.25 (1H, d, J = 2.4 Hz).

### Referential Example 804

Methyl 3-(2-{[7-chloro-5-(2,3-dimethoxyphenyl)-2-thioxo-1,2,3,5-tetrahydropyrido[2,3-e][1,4]thiazepin-3-yl]methyl}-1,3-thiazol-5-yl)propanoate

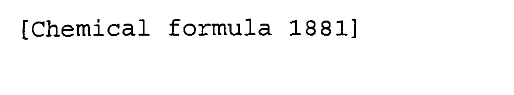

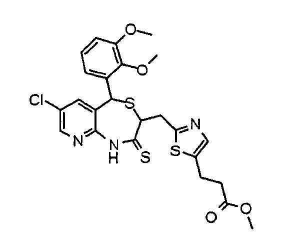

Methyl 5-({2-[trans-7-chloro-5-(2,3-dimethoxyphenyl)-2-oxo-1,2,3,5-tetrahydropyrido[2,3-e][1,4]thiazepin-3-yl]acetyl}amino)-4-oxopentanoate (885 mg) was dissolved in tetrahydrofuran (20 mL). To the resulting solution was added Lawesson's reagent (2.54 g). The resulting mixture was heated under reflux for 4 hours. The reaction mixture was concentrated under reduced pressure. The residue was purified by silica gel column chromatography (hexane:ethyl acetate= from 4:1 to 1:1) to give the title compound (622 mg) as a trans-cis mixture (trans:cis=6:1).
MS (FAB) m/z: 550 (M+H)⁺.
¹H-NMR (CDCl3) δ: 2.61-2.68 (trans and cis of 2H, m), 3.06-3.13 (trans and cis of 2H, m), 3.29-3.40 (trans and cis of 1H, m), 3.60 (cis of 3H, s), 3.64 (trans of 3H, s), 3.69 (trans and cis of 3H, s), 3.81 (cis of 3H, s), 3.86-3.96 (trans and cis of 4H, m), 4.18-4.23 (trans of 1H, m), 4.29 (cis of 1H, t, J = 7.0 Hz), 5.65 (cis of 1H, s), 6.02 (trans of 1H, s), 6.87 (cis of 1H, d, J = 7.6 Hz), 6.98 (trans of 1H, d, J = 8.1 Hz), 7.09 (cis of 1H, t, J = 8.1 Hz), 7.15-7.25 (trans and cis of 3H, m), 7.34 (trans of 1H, s), 7.36 (cis of 1H, s), 7.57 (cis of 1H, d, J = 8.1 Hz), 7.76 (cis of 1H, d, J = 2.4 Hz), 8.27-8.30 (trans and cis of 1H, m), 9.18 (cis of 1H, s), 9.47 (trans of 1H, s).

### Example 898

Methyl 3-(2-{[trans-3-chloro-5-(2,3-dimethoxyphenyl)-10-(trifluoromethyl)-5H,7H-pyrido[2,3-e][1,2,4]triazolo[3,4-c][1,4]thiazepin-7-yl]methyl}-1,3-thiazol-5-yl)propanoate

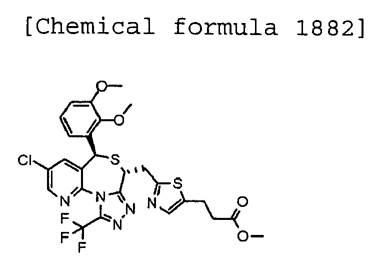

In a similar manner to that employed for the synthesis of methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate and methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate, the title compound (184 mg) was obtained from methyl 3-(2-{[7-chloro-5-(2,3-dimethoxyphenyl)-2-thioxo-1,2,3,5-tetrahydropyrido[2,3-e][1,4]thiazepin-3-yl]methyl}-1,3-thiazol-5-yl)propanoate (200 mg).
MS (FAB) m/z: 626 (M+H)⁺.
¹H-NMR (CDCl3) δ: 2.65 (2H, t, J = 7.4 Hz), 3.11 (2H, t, J = 7.4 Hz), 3.46 (3H, s), 3.66 (1H, dd, J = 15.4, 7.3 Hz), 3.69 (3H, s), 3.85 (3H, s), 4.12 (1H, dd, J = 15.4, 7.3 Hz), 4.54 (1H, t, J = 7.3 Hz), 5.40 (1H, s), 6.98 (1H, dd, J = 7.2, 2.6 Hz), 7.13-7.19 (2H, m), 7.34 (1H, d, J = 2.6 Hz), 7.38 (1H, s), 8.45 (1H, d, J = 2.6 Hz).

### Example 899

3-(2-{[3-Chloro-5-(2,3-dimethoxyphenyl)-10-(trifluoromethyl)-5H,7H-pyrido[2,3-e][1,2,4]triazolo[3,4-c] [1,4]thiazepin-7-yl]methyl}-1,3-thiazol-5-yl)propanoic acid

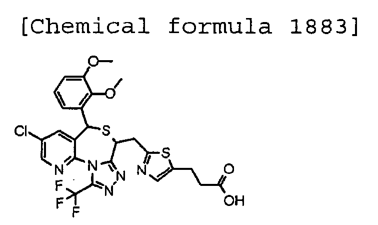

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (136 mg) was obtained as a trans-cis mixture (trans:cis=4:1) from methyl 3-(2-{[trans-3-chloro-5-(2,3-dimethoxyphenyl)-10-(trifluoromethyl)-5H,7H-pyrido[2,3-e][1,2,4]triazolo[3,4-c] [1,4] thiazepin-7-yl] methyl}-1,3-thiazol-5-yl) propanoate (180 mg).
MS (FAB) m/z: 612 (M+H)⁺.
¹H-NMR (CDCl3) δ: 2.66-2.72 (trans and cis of 2H, m), 3.07-3.14 (trans and cis of 2H, m), 3.40 (cis of 3H, s), 3.46 (trans of 3H, s), 3.61-3.73 (trans and cis of 1H, m), 3.77 (cis of 3H, s), 3.85 (trans of 3H, s), 4.04-4.21 (trans and cis of 1H, m), 4.53-4.63 (trans and cis of 1H, m), 5.38 (cis of 1H, s), 5.40 (trans of 1H, s), 6.75-6.95 (cis of 3H, m), 6.98 (trans of 1H, dd, J = 7.4, 2.3 Hz), 7.13-7.20 (trans of 2H, m), 7.30-7.42 (trans and cis of 2H, m), 8.45 (trans of 1H, d, J = 2.4 Hz), 8.46 (cis of 1H, d, J = 2.4 Hz).
Elemental analysis for: C₂₅H₂₁ClF₃N₅O₄S₂·1.0H₂O·0.4CHCl₃
Calculated: C, 45.40; H, 3.46; N, 10.26
Found: C, 45.05; H, 3.23; N, 10.56.

### Referential Example 805

2-[trans-1-Allyl-7-chloro-5-(2,3-dimethoxyphenyl)-2-oxo-1,2,3,5-tetrahydro-4,1-benzothiazepin-3-yl]acetic acid

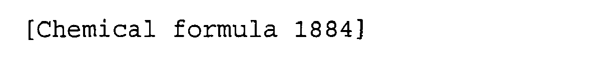

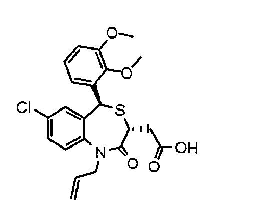

2-[(trans)-7-Chloro-5-(2,3-dimethoxyphenyl)-2-oxo-1,2,3,5-tetrahydro-4,1-benzothiazepin-3-yl]acetic acid (60.0 g) was dissolved in N,N-dimethylformamide (500 mL). To the resulting solution was added sodium hydride (14.7 g). The resulting mixture was stirred at room temperature for 30 minutes. To the reaction mixture was added allyl bromide (64.5 mL). The resulting mixture was stirred at room temperature for 3 hours. After the reaction mixture was concentrated under reduced pressure, tetrahydrofuran (150 mL), water (350 mL) and methanol (750 mL) were added to the residue. To the resulting mixture was added lithium hydroxide hydrate (9.66 g), followed by stirring at 50°C for one hour. The reaction mixture was concentrated under reduced pressure. To the residue were added ethyl acetate (600 mL), tetrahydrofuran (250 mL) and 1N hydrochloric acid (250 mL) and the layers separated. The organic layer was dried over anhydrous sodium sulfate. The solvent was distilled off under reduced pressure. The residue was subjected to slurry washing with a hexane:ethanol solvent (hexane:ethanol=20:1) mixture to give the title compound (44.1 g).
MS (FAB) m/z: 448 (M+H)⁺.
¹H-NMR (CDCl3) δ: 2.55 (1H, dd, J = 17.5, 3.5 Hz), 3.14 (1H, dd, J = 17.5, 10.0 Hz), 3.59 (3H, s), 3.69 (1H, dd, J = 10.0, 3.5 Hz), 3.88 (3H, s), 4.21 (1H, dd, J = 14.9, 6.8 Hz), 4.84 (1H, dd, J = 14.9, 7.4 Hz), 5.20-5.30 (2H, m), 5.93-6.05 (2H, m), 6.81 (1H, d, J = 1.5 Hz), 6.98 (1H, d, J = 8.0 Hz), 7.12-7.21 (1H, m), 7.23-7.31 (3H, m).

### Referential Example 806

Methyl 5-({2-[trans-1-allyl-7-chloro-5-(2,3-dimethoxyphenyl)-2-oxo-1,2,3,5-tetrahydro-4,1-benzothiazepin-3-yl]acetyl}amino)-4-oxopentanoate

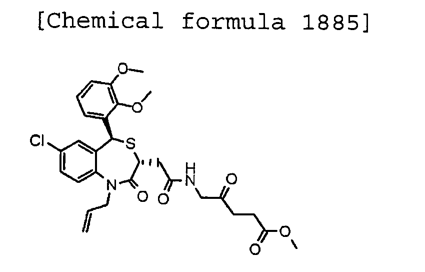

In a similar manner to that employed for the synthesis of methyl 5-({3-[(trans)-8-chloro-1-cyclopropyl-6-(2,3-dimethoxyphenyl)-4H,6H-[1,2,4]triazolo[4,3-a][4,1]benzothiazepin-4-yl]propanoyl}amino)-4-oxopentanoate, the title compound (933 mg) was obtained from 2-[trans-1-allyl-7-chloro-5-(2,3-dimethoxyphenyl)-2-oxo-1,2,3,5-tetrahydro-4,1-benzothiazepin-3-yl]acetic acid (770 mg).
MS (FAB) m/z: 575 (M+H)⁺.
¹H-NMR (CDCl3) δ: 2.38 (1H, dd, J = 14.8, 3.8 Hz), 2.62-2.67 (2H, m), 2.69-2.74 (2H, m), 3.04 (1H, dd, J = 14.8, 10.0 Hz), 3.57 (3H, s), 3.68 (3H, s), 3.79 (1H, dd, J = 10.0, 3.8 Hz), 3.88 (3H, s), 4.13-4.20 (3H, m), 4.86 (1H, dd, J = 14.5, 6.5 Hz), 5.17-5.30 (2H, m), 5.93-6.05 (2H, m), 6.44-6.48 (1H, m), 6.79 (1H, s), 6.97 (1H, dd, J = 8.3, 1.5 Hz), 7.17 (1H, t, J = 8.3 Hz), 7.24-7.29 (3H, m).

### Referential Example 807

Methyl 3-(2-{[trans-1-allyl-7-chloro-5-(2,3-dimethoxyphenyl)-2-oxo-1,2,3,5-tetrahydro-4,1-benzothiazepin-3-yl]methyl}-1,3-oxazol-5-yl)propanoate

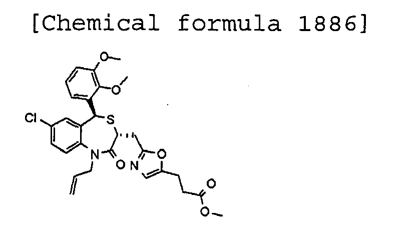

In a similar manner to that employed for the synthesis of methyl 3-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)propanoate, the title compound (881 mg) was obtained from methyl 5-({2-[trans-1-allyl-7-chloro-5-(2,3-dimethoxyphenyl)-2-oxo-1,2,3,5-tetrahydro-4,1-benzothiazepin-3-yl]acetyl}amino)-4-oxopentanoate (927 mg).
MS (FAB) m/z: 556 (M+H)⁺.
¹H-NMR (CDCl3) δ: 2.62 (2H, t, J = 8.0 Hz), 2.90-2.97 (3H, m), 3.48 (1H, dd, J = 16.5, 8.0 Hz), 3.58 (3H, s), 3.69 (3H, s), 3.83-3.91 (4H, m), 4.21 (1H, dd, J = 14.9, 6.5 Hz), 4.82 (1H, dd, J = 14.9, 5.9 Hz), 5.18-5.31 (2H, m), 5.94-6.05 (2H, m), 6.62 (1H, s), 6.79 (1H, d, J = 2.0 Hz), 6.97 (1H, dd, J = 8.0, 1.5 Hz), 7.16 (1H, t, J = 8.0 Hz), 7.23-7.29 (3H, m).

### Referential Example 808

Methyl 3-(2-{[trans-7-chloro-5-(2,3-dimethoxyphenyl)-2-oxo-1,2,3,5-tetrahydro-4,1-benzothiazepin-3-yl]methyl}-1,3-oxazol-5-yl)propanoate

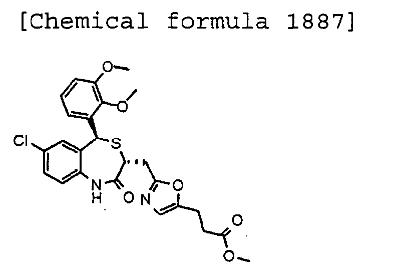

Methyl 3-(2-{[trans-1-allyl-7-chloro-5-(2,3-dimethoxyphenyl)-2-oxo-1,2,3,5-tetrahydro-4,1-benzothiazepin-3-yl]methyl}-1,3-oxazol-5-yl)propanoate (1.22 g) was dissolved in anhydrous toluene (75 mL). To the resulting solution were added bis(1,3-diphenylphosphinopropane)nickel(II) chloride (118 mg) and an n-hexane solution (a 1.08M solution, 4.04 mL) of trimethylaluminum and the resulting mixture was stirred at 100°C for 3 hours under a nitrogen gas stream. After the reaction mixture was cooled to a temperature of ice cooling, 1N hydrochloric acid was added to terminate the reaction. To the reaction mixture was added ethyl acetate and the layers separated. The organic layer was washed successively with a saturated aqueous solution of sodium hydrogen carbonate and saturated brine and then dried over anhydrous sodium sulfate. The solvent was distilled off under reduced pressure. The residue was purified by silica gel column chromatography (chloroform:methanol=200:1) to give the title compound (679 mg).
MS (FAB) m/z: 517 (M+H)⁺.
¹H-NMR (CDCl3) δ: 2.62 (2H, t, J = 7.6 Hz), 2.87-2.97 (3H, m), 3.47 (1H, dd, J = 15.4, 8.3 Hz), 3.60 (3H, s), 3.69 (3H, s), 3.87 (3H, s), 3.94-3.98 (1H, m), 6.22 (1H, s), 6.63 (1H, s), 6.84 (1H, d, J = 2.2 Hz), 6.97 (1H, dd, J = 8.3, 1.7 Hz), 7.08 (1H, d, J = 8.3 Hz), 7.17 (1H, t, J = 7.9 Hz), 7.22-7.26 (2H, m), 7.28-7.32 (1H, m).

### Referential Example 809

Methyl 3-(2-{[trans-7-chloro-5-(2,3-dimethoxyphenyl)-2-thioxo-1,2,3,5-tetrahydro-4,1-benzothiazepin-3-yl]methyl}-1,3-oxazol-5-yl)propanoate

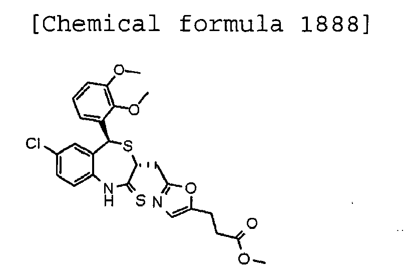

In a similar manner to that employed for the synthesis of methyl 3-({2-[(trans)-7-chloro-5-(2,3-dimethoxyphenyl)-2-thioxo-1,2,3,5-tetrahydro-4,1-benzothiazepin-3-yl]methyl}-1,3-thiazol-5-yl)propanoate, the title compound (589 mg) was obtained from methyl 3-(2-{[trans-7-chloro-5-(2,3-dimethoxyphenyl)-2-oxo-1,2,3,5-tetrahydro-4,1-benzothiazepin-3-yl]methyl}-1,3-oxazol-5-yl)propanoate (666 mg).
MS (FAB) m/z: 533 (M+H)⁺.
¹H-NMR (CDCl3) δ: 2.63 (2H, t, J =7.6 Hz), 2.94 (2H, t, J = 7.4 Hz), 3.13 (1H, dd, J = 16.4, 5.9 Hz), 3.61 (3H, s), 3.67-3.75 (4H, m), 3.87 (3H, s), 4.17-4.23 (1H, m), 6.05 (1H, s), 6.64 (1H, s), 6.83 (1H, d, J = 2.4 Hz), 6.95-6.99 (1H, m), 7.10 (1H, d, J = 8.1 Hz), 7.17 (1H, t, J = 8.1 Hz), 7.22-7.30 (2H, m), 9.22 (1H, s).

### Example 900

Methyl 3-(2-{[8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate

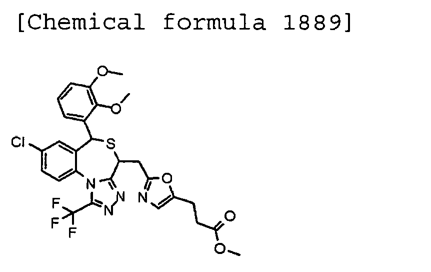

In a similar manner to that employed for the synthesis of methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate and methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate, the title compound (329 mg) was obtained as a trans-cis mixture (trans:cis=6:1) from methyl 3-(2-{[trans-7-chloro-5-(2,3-dimethoxyphenyl)-2-thioxo-1,2,3,5-tetrahydro-4,1-benzothiazepin-3-yl]methyl}-1,3-oxazol-5-yl)propanoate (587 mg).
MS (FAB) m/z: 609 (M+H)⁺.
¹H-NMR (CDCl3) δ: 2.57-2.66 (trans and cis of 2H, m), 2.85-2.99 (trans and cis of 2H, m), 3.30 (cis of 3H, s), 3.43-3.62 (trans of 3H, trans and cis of 1H, m), 3.69 (trans and cis of 3H, s), 3.75 (cis of 3H, s), 3.85 (trans of 3H, s), 3.91-4.04 (trans and cis of 1H, m), 4.40-4.49 (trans and cis of 1H, m), 5.36 (trans of 1H, s), 5.38 (cis of 1H, s), 6.65 (trans of 1H, s), 6.67 (cis of 1H, s), 6.75-6.89 (cis of 2H, m), 6.96-6.98 (trans of 2H, m), 7.12-7.22 (trans and cis of 2H, m), 7.36-7.48 (trans and cis of 1H, trans of 1H, m), 7.78 (cis of 1H, s).

### Example 901

Methyl 3-(2-{[(4S,6R)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (isomer A)

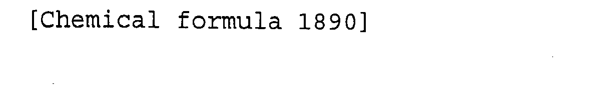

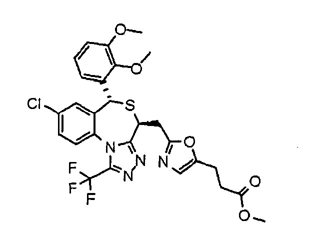

Methyl 3-(2-{[(4R,6S)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (isomer B)

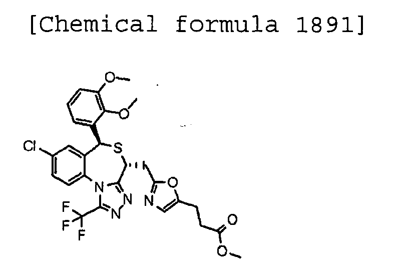

Methyl 3-(2-{[8-chloro-6-(2,3-dimethoxyphenyl)-1-(trifluoromethyl)-4H,6H-[1,2,4]triazolo[4,3-a] [4,1]benzothiazepin-4-yl]methyl}-1,3-oxazol-5-yl)propanoate (trans:cis=6:1, 1770 mg) was dissolved in ethanol. The resulting solution was left to stand for one hour at room temperature. The solid thus precipitated was collected by filtration to give 3-(2-{[trans-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate. The resulting compound was optically resolved by a CHIRALPAK AD column (Daicel Chemical Industries, hexane:ethanol=15:85, flow rate: 50 mL/min) into methyl 3-(2-{[(4S,6R)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (isomer A) (retention time: 31 min., 604 mg) and methyl 3-(2-{[(4R,6S)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (isomer B) (retention time: 102 min., 529 mg).
Methyl 3-(2-{[(4S,6R)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (isomer A)
MS (FAB) m/z: 609 (M+H)⁺.
¹H-NMR (CDCl3) δ: 2.64 (2H, t, J = 7.2 Hz), 2.96 (2H, t, J = 7.2 Hz), 3.43 (3H, s), 3.48 (1H, dd, J = 16.4, 7.7 Hz), 3.69 (3H, s), 3.85 (3H, s), 3.95 (1H, dd, J = 16.4-, 7.1 Hz), 4.46 (1H, t, J = 7.3 Hz), 5.36 (1H, s), 6.65 (1H, s), 6.95-6.99 (2H, m), 7.12-7.21 (2H, m), 7.38 (1H, d, J = 8.3 Hz), 7.43 (1H, dd, J = 8.3, 2.2 Hz).
Methyl 3-(2-{[(4R,6S)-8-chloro-6-(2,3-dimethoxyphenyl)-1-(trifluoromethyl)-4H,6H-[1,2,4]triazolo[4,3-a] [4,1]benzothiazepin-4-yl]methyl}-1,3-oxazol-5-yl)propanoate (isomer B)
MS (FAB) m/z: 609 (M+H)⁺.
¹H-NMR (CDCl3) δ: 2.64 (2H, t, J = 7.2 Hz), 2.96 (2H, t, J = 7.2 Hz), 3.43 (3H, s), 3.48 (1H, dd, J = 16.4, 7.7 Hz), 3.69 (3H, s), 3.85 (3H, s), 3.95 (1H, dd, J = 16.4, 7.1 Hz), 4.46 (1H, t, J = 7.3 Hz), 5.36 (1H, s), 6.65 (1H, s), 6.95-6.99 (2H, m), 7.12-7.21 (2H, m), 7.38 (1H, d, J = 8.3 Hz), 7.43 (1H, dd, J = 8.3,2.2 Hz).

### Example 902

3-(2-{[(4S,6R)-8-Chloro-6-(2,3-dimethoxyphenyl)-1-(trifluoromethyl)-4H,6H-[1,2,4]triazolo[4,3-a] [4,1]benzothiazepin-4-yl]methyl}-1,3-oxazol-5-yl)propanoic acid (isomer A)

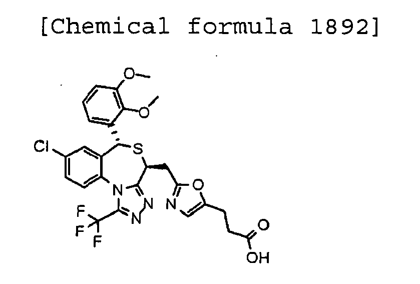

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (616 mg) was obtained from methyl 3-(2-{[(4S,6R)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (604 mg).
MS (FAB) m/z: 595 (M+H)⁺.
¹H-NMR (CDCl3) δ: 2.68 (2H, t, J = 7.3 Hz), 2.97 (2H, t, J = 7.3 Hz), 3.40-3.51 (4H, m), 3.85 (3H, s), 4.46 (1H, t, J = 7.3 Hz), 5.37 (1H, s), 6.68 (1H, s), 6.95-7.00 (2H, m), 7.10-7.22 (2H, m), 7.37-7.45 (2H, m).
Elemental analysis for: C₂₆H₂₂ClF₃N₄O₅S·1.0H₂O·0.2CHCl₃ Calculated: C, 49.60; H, 3.82; N, 8.76
Found: C, 49.54; H, 3.45; N, 8.75.

### Example 903

3-(2-{[(4R,6S)-8-Chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoic acid (isomer B)

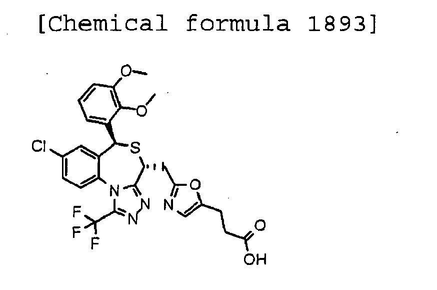

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (567 mg) was obtained from methyl 3-(2-{[(4R,6S)-8-chloro-6-(2,3-dimethoxyphenyl)-1-(trifluoromethyl)-4H,6H-[1,2,4]triazolo[4,3-a] [4,1]benzothiazepin-4-yl]methyl}-1,3-oxazol-5-yl)propanoate (567 mg).
MS (FAB) m/z: 595 (M+H)⁺.
¹H-NMR (CDCl3) δ: 2.68 (2H, t, J = 7.3 Hz), 2.97 (2H, t, J = 7.3 Hz), 3.40-3.51 (4H, m), 3.85 (3H, s), 4.46 (1H, t, J = 7.3 Hz), 5.37 (1H, s), 6.68 (1H, s), 6.95-7.00 (2H, m), 7.10-7.22 (2H, m), 7.37-7.45 (2H, m).
Elemental analysis for: C₂₆H₂₂ClF₃N₄O₅S·0.75H₂O·0.2CHCl₃
Calculated: C, 49.95; H, 3.76; N, 8.83
Found: C, 49.92; H, 3.54; N, 8.75.

### Example 904

3-(2-{[8-Chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoic acid

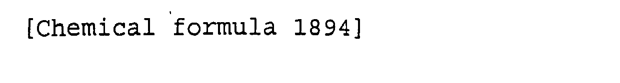

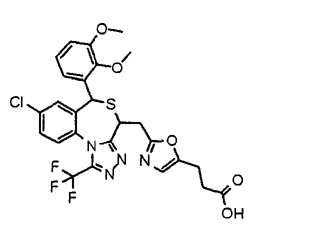

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (135 mg) was obtained as a trans-cis mixture (trans:cis=6:1) from methyl 3-(2-{[8-chloro-6-(2,3-dimethoxyphenyl)-1-(trifluoromethyl)-4H,6H-[1,2,4]triazolo[4,3-a][4,1]benzothiazepin-4-yl]methyl}-1,3-oxazol-5-yl)propanoate (130 mg).
MS (FAB) m/z: 595 (M+H)⁺.
¹H-NMR (CDCl3) δ: 2.59-2.70 (trans and cis of 2H, m), 2.87-3.00 (trans and cis of 2H, m), 3.30 (cis of 3H, s), 3.43-3.63 (trans of 3H, trans and cis of 1H, m), 3.79 (cis of 3H, s), 3.85 (trans of 3H, s), 3.93-4.09 (trans and cis of 1H, m), 4.41-4.50 (trans and cis of 1H, m), 5.36 (trans of 1H, s), 5.38 (cis of 1H, s), 6.60-6.87 (trans and cis of 1H, cis of 2H, m), 6.98-7.02 (trans of 2H, m), 7.14-7.24 (trans and cis of 2H, m), 7.39-7.49 (trans and cis of 1H, trans of 1H, m), 7.78 (cis of 1H, s). Elemental analysis for: C₂₆H₂₂ClF₃N₄O₅S·1.0H₂O·0.4CHCl₃ Calculated: C, 48.36; H, 3.70; N, 8.42
Found: C, 48.11; H, 3.48; N, 8.53.

### Referential Example 810

trans-1-Allyl-7-chloro-5-(2,3-dimethoxyphenyl)-3-(2-hydroxyethyl)-1,5-dihydro-4,1-benzothiazepin-2(3H)-one

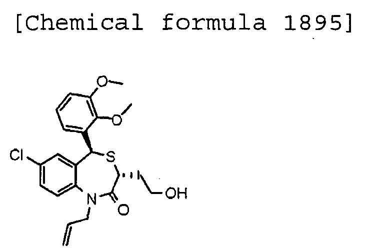

2-[trans-1-Allyl-7-chloro-5-(2,3-dimethoxyphenyl)-2-oxo-1,2,3,5-tetrahydro-4,1-benzothiazepin-3-yl]acetic acid (22.1 g) was dissolved in tetrahydrofuran (300 mL). To the resulting solution was added N-methylmorpholine (10.8 mL). To the resulting mixture was added dropwise ethyl chloroformate (6.12 mL) at -20°C, followed by stirring at -20°C for 15 minutes. After addition of sodium borohydride (7.45 g) at -20°C, methanol (30 mL) was added and the resulting mixture was stirred under ice cooling for 15 minutes. After warming to room temperature, ethyl acetate and 1N hydrochloric acid (500 mL) were added to the reaction mixture and the layers separated. The organic layer was washed successively with a saturated aqueous solution of sodium hydrogen carbonate and saturated brine and dried over anhydrous sodium sulfate. The solvent was distilled off under reduced pressure. The residue was purified by silica gel column chromatography (hexane: ethyl acetate =2:1) to give the title compound (20.8 g) as a pale yellow foam.
MS (FAB) m/z: 434 (M+H)⁺.
¹H-NMR (CDCl3) δ: 1.80-1.90 (1H, m), 2.26-2.36 (1H, m), 3.50 (1H, dd, J = 8.2, 4.8 Hz), 3.59 (3H, s), 3.62-3.78 (2H, m), 3.88 (3H, s), 4.17 (1H, dd, J = 14.5, 7.2 Hz), 4.87 (1H, dd, J = 14.5, 5.9 Hz), 5.19-5.27 (2H, m), 5.95-6.06 (2H, m), 6.79 (1H, d, J = 2.2 Hz), 6.97 (1H, dd, J = 8.0, 2.2 Hz), 7.15-7.21 (2H, m), 7.25-7.29 (2H, m).

### Referential Example 811

3-[trans-1-Allyl-7-chloro-5-(2,3-dimethoxyphenyl)-2-oxo-1,2,3,5-tetrahydro-4,1-benzothiazepin-3-yl]propanenitrile

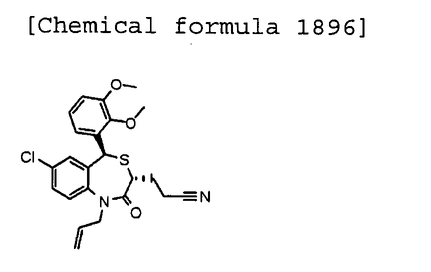

In a similar manner to that employed for the synthesis of 2-[(trans)-8-chloro-1-cyclopropyl-6-(2,3-dimethoxyphenyl)-4H,6H-[1,2,4]triazolo[4,3-a][4,1]benzothiazepin-4-yl]ethyl methanesulfonate and 3-[(trans)-8-chloro-1-cyclopropyl-6-(2,3-dimethoxyphenyl)-4H,6H-[1,2,4]triazolo[4,3-a] [4,1]benzothiazepin-4-yl]propanenitrile, the title compound (1.40 g) was obtained from trans-1-allyl-7-chloro-5-(2,3-dimethoxyphenyl)-3-(2-hydroxyethyl)-1,5-dihydro-4,1-benzothiazepin-2(3H)-one (1.94 g).
MS (EI) m/z: 442 M⁺.
¹H-NMR (CDCl3) δ: 1.88-1.98 (1H, m), 2.29-2.39 (1H, m), 2.49-2.55 (2H, m), 3.42 (1H, dd, J = 8.8, 5.1 Hz), 3.59 (3H, s), 3.88 (3H, s), 4.15 (1H, dd, J = 7.2, 14.4 Hz), 4.88 (1H, dd, J = 14.4, 5.8 Hz), 5.20-5.30 (2H, m), 5.94-6.05 (2H, m), 6.81 (1H, d, J = 2.4 Hz), 6.98 (1H, dd, J = 8.2, 1.6 Hz), 7.17-7.21 (2H, m), 7.24-7.31 (2H, m).

### Referential Example 812

Ethyl 3-[trans-1-allyl-7-chloro-5-(2,3-dimethoxyphenyl)-2-oxo-1,2,3,5-tetrahydro-4,1-benzothiazepin-3-yl]propanoate

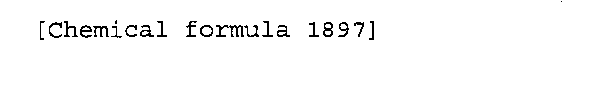

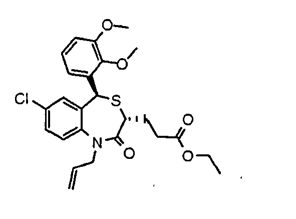

3-[trans-1-Allyl-7-chloro-5-(2,3-dimethoxyphenyl)-2-oxo-1,2,3,5-tetrahydro-4,1-benzothiazepin-3-yl]propanenitrile (1.20 g) was dissolved in ethanol (30 mL). To the resulting solution was added tosic acid hydrate (5.16 g) and the resulting mixture was heated under reflux for 3 hours. After cooling to room temperature, ethyl acetate and a saturated aqueous solution of sodium hydrogen carbonate were added to the reaction mixture and the layers separated. The organic layer was washed successively with saturated brine and dried over anhydrous sodium sulfate. The solvent was distilled off under reduced pressure to give the title compound (1.33 g).
MS (FAB) m/z: 490 (M+H)⁺.
¹H-NMR (CDCl3) δ: 1.19 (3H, t, J = 7.2 Hz), 1.23-1.32 (1H, m), 1.88-1.99 (1H, m), 2.26-2.48 (3H, m), 3.33 (1H, dt, J = 2.0, 6.4 Hz), 3.58 (3H, s), 3.88 (3H, s), 4.07 (2H, q, J = 7.2 Hz), 4.16 (1H, dd, J = 7.4, 14.9 Hz), 4.86 (1H, dd, J = 14.9, 6.1 Hz), 5.18-5.27 (2H, m), 5.95-6.06 (2H, m), 6.78 (1H, d, J = 2.2 Hz), 6.97 (1H, dd, J = 8.2, 1.3 Hz), 7.15-7.20 (2H, m), 7.23-7.29 (2H, m).

### Referential Example 813

3-[trans-1-Allyl-7-chloro-5-(2,3-dimethoxyphenyl)-2-oxo-1,2,3,5-tetrahydro-4,1-benzothiazepin-3-yl]propanoic acid

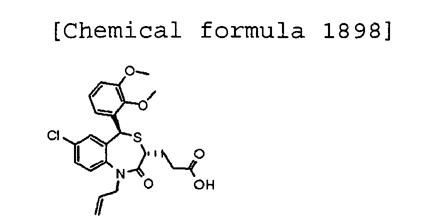

In a similar manner to that employed for the synthesis of 3-(2-(2-[(trans)-8-chloro-1-cyclopropyl-6-(2,3-dimethoxyphenyl)-4H,6H-[1,2,4]triazolo[4,3-a][4,1]benzothiazepin-4-yl]ethyl)-1,3-thiazol-5-yl)propanoic acid, the title compound (626 mg) was obtained from ethyl 3-[trans-1-allyl-7-chloro-5-(2,3-dimethoxyphenyl)-2-oxo-1,2,3,5-tetrahydro-4,1-benzothiazepin-3-yl]propanoate (715 mg).
MS (FAB) m/z: 462 (M+H)⁺.
¹H-NMR (CDCl3) δ: 1.87-1.99 (1H, m), 2.25-2.37 (1H, m), 2.38-2.54 (2H, m), 3.31-3.37 (1H, m), 3.58 (3H, s), 3.88 (3H, s), 4.15 (1H, dd, J = 7.2, 14.5 Hz), 4.87 (1H, dd, J = 14.5, 6.3 Hz), 5.18-5.27 (2H, m), 5.94-6.05 (2H, m), 6.78 (1H, d, J = 2.0 Hz), 6.97 (1H, d, J = 8.3 Hz), 7.15-7.20 (2H, m), 7.24-7.29 (2H, m).

### Referential Example 814

Methyl 5-({3-[trans-1-allyl-7-chloro-5-(2,3-dimethoxyphenyl)-2-oxo-1,2,3,5-tetrahydro]-4,1-benzothiazepin-3-yl}propanoyl)amino)-4-oxopentanoate

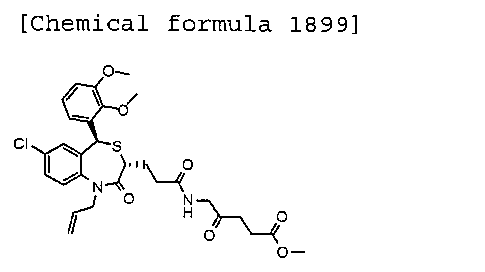

In a similar manner to that employed for the synthesis of methyl 5-({3-[(trans)-8-chloro-1-cyclopropyl-6-(2,3-dimethoxyphenyl)-4H,6H-[1,2,4]triazolo[4,3-a][4,1]benzothiazepin-4-yl]propanoyl}amino)-4-oxopentanoate, the title compound (780 mg) was obtained from 3-[trans-1-allyl-7-chloro-5-(2,3-dimethoxyphenyl)-2-oxo-1,2,3,5-tetrahydro-4,1-benzothiazepin-3-yl]propanoic acid (62 mg) .
MS (EI) m/z: 588 M⁺.
¹H-NMR (CDCl3) δ: 1.91-2.04 (1H, m), 2.24-2.38 (3H, m), 2.62-2.75 (4H, m), 3.30-3.37 (1H, m), 3.58 (3H, s), 3.68 (3H, s), 3.88 (3H, s), 4.07-4.20 (3H, m), 4.89 (1H, dd, J = 14.8, 6.0 Hz), 5.17-5.28 (2H, m), 5.94-6.06 (2H, m), 6.28 (1H, br s), 6.77 (1H, d, J = 2.2 Hz), 6.97 (1H, dd, J = 8.1, 1.2 Hz), 7.14-7.20 (2H, m), 7.23-7.30 (2H, m).

### Referential Example 815

Methyl 3-(2-{2-[trans-1-allyl-7-chloro-5-(2,3-dimethoxyphenyl)-2-oxo-1,2,3,5-tetrahydro-4,1-benzothiazepin-3-yl]ethyl}-1,3-oxazol-5-yl)propanoate

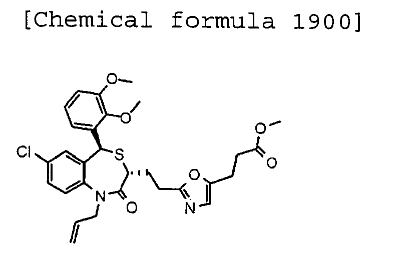

In a similar manner to that employed for the synthesis of methyl 3-({2-[(trans)-7-chloro-1-(2,4-dimethoxybenzyl)-5-(2,3-dimethoxyphenyl)-2-oxo-1,2,3,5-tetrahydro-4,1-benzoxazepin-3-yl]methyl}-1,3-oxazol-5-yl)propanoate, the title compound (489 mg) was obtained from methyl 5-({3-[trans-1-allyl-7-chloro-5-(2,3-dimethoxyphenyl)-2-oxo-1,2,3,5-tetrahydro]-4,1-benzothiazepin-3-yl}propanoyl)amino)-4-oxopentanoate (775 mg).
MS (FAB) m/z: 571 (M+H)⁺.
¹H-NMR (CDCl3) δ: 1.94-2.05 (1H, m), 2.40-2.50 (1H, m), 2.60 (2H, t, J = 7.6 Hz), 2.73-2.86 (2H, m), 2.90 (2H, t, J = 7.6 Hz), 3.34 (1H, dd, J = 8.7, 5.1 Hz), 3.58 (3H, s), 3.70 (3H, s), 3.88 (3H, s), 4.15 (1H, dd, J = 7.3, 14.8 Hz), 4.87 (1H, dd, J = 14.8, 5.9 Hz), 5.18-5.27 (2H, m), 5.95-6.05 (2H, m), 6.59 (1H, s), 6.77 (1H, d, J = 2.2 Hz), 6.97 (1H, dd, J = 8.2, 1.3 Hz), 7.14-7.20 (2H, m), 7.22-7.29 (2H, m).

### Referential Example 816

Methyl 3-(2-{2-[trans-7-chloro-5-(2,3-dimethoxyphenyl)-2-oxo-1,2,3,5-tetrahydro-4,1-benzothiazepin-3-yl]ethyl}-1,3-oxazol-5-yl)propanoate

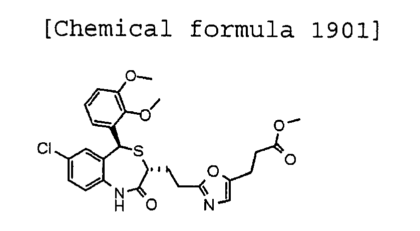

In a similar manner to that employed for the synthesis of methyl 3-(2-{[trans-7-chloro-5-(2,3-dimethoxyphenyl)-2-oxo-1,2,3,5-tetrahydro-4,1-benzothiazepin-3-yl]methyl}-1,3-oxazol-5-yl)propanoate, the title compound (171 mg) was obtained from methyl 3-(2-{2-[trans-1-allyl-7-chloro-5-(2,3-dimethoxyphenyl)-2-oxo-1,2,3,5-tetrahydro-4,1-benzothiazepin-3-yl]ethyl}-1,3-oxazol-5-yl)propanoate (485 mg).
MS (FAB) m/z: 531 (M+H)⁺.
¹H-NMR (CDCl3) δ: 1.95-2.05 (1H, m), 2.37-2.49 (1H, m), 2.60 (2H, t, J = 7.5 Hz), 2.82-2.87 (2H, m), 2.91 (2H, t, J = 7.5 Hz), 3.44 (1H, dd, J = 8.2, 5.7 Hz), 3.60 (3H, s), 3.70 (3H, s), 3.88 (3H, s), 6.18 (1H, s), 6.61 (1H, s), 6.83 (1H, d, J = 2.2 Hz), 6.97 (1H, dd, J = 1.2, 8.1 Hz), 7.03 (1H, d, J = 8.3 Hz), 7.14-7.17 (3H, m), 7.29-7.33 (1H, m).

### Referential Example'817

Methyl 3-(2-{2-[trans-7-chloro-5-(2,3-dimethoxyphenyl)-2-thioxo-1,2,3,5-tetrahydro-4,1-benzothiazepin-3-yl]ethyl}-1,3-oxazol-5-yl)propanoate

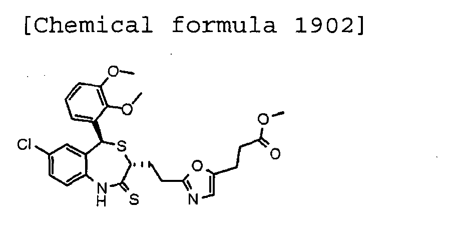

In a similar manner to that employed for the synthesis of methyl 3-({2-[(trans)-7-chloro-5-(2,3-dimethoxyphenyl)-2-thioxo-1,2,3,5-tetrahydro-4,1-benzothiazepin-3-yl]methyl}-1,3-thiazol-5-yl)propanoate, the title compound (133 mg) was obtained from methyl 3-(2-{2-[trans-7-chloro-5-(2,3-dimethoxyphenyl)-2-oxo-1,2,3,5-tetrahydro-4,1-benzothiazepin-3-yl]ethyl}-1,3-oxazol-5-yl)propanoate (168 mg).
MS (FAB) m/z: 547 (M+H)⁺.
¹H-NMR (CDCl3) δ: 2.09-2.23 (1H, m), 2.56-2.71 (3H, m), 2.82-2.98 (4H, m), 3.59-3.67 (4H, m), 3.70 (3H, s), 3.87 (3H, s), 6.01 (1H, s), 6.59 (1H, s), 6.82 (1H, d, J = 2.2 Hz), 6.97 (1H, dd, J = 8.1, 1.5 Hz), 7.06 (1H, d, J = 8.1 Hz), 7.18 (1H, t, J = 8.1 Hz), 7.23-7.31 (2H, m), 9.19 (1H, s).

### Example 905

Methyl 3-(2-{2-[8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate

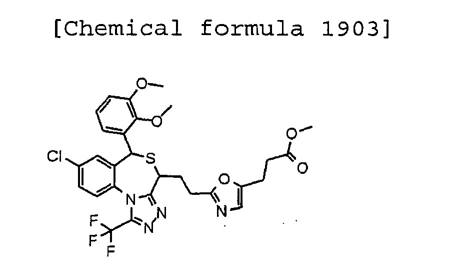

In a similar manner to that employed for the synthesis of methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate and methyl 3-({2-[(trans)-8-chloro-6-(2,3-dimethoxyphenyl)-1-(trifluoromethyl)-4H,6H-[1,2,4]triazolo[4,3-a][4,1]benzothiazepin-4-yl]methyl}-1,3-thiazol-5-yl)propanoate, the title compound (123 mg) was obtained as a trans-cis mixture (trans:cis=8:1) from methyl 3-(2-{2-[trans-7-chloro-5-(2,3-dimethoxyphenyl)-2-thioxo-1,2,3,5-tetrahydro-4,1-benzothiazepin-3-yl]ethyl}-1,3-oxazol-5-yl)propanoate (130 mg).
MS (EI) m/z: 622 M⁺.
¹H-NMR (CDCl3) δ: 2.41-2.52 (trans and cis of 1H, m), 2.60 (trans and cis of 2H, t, J = 7.4 Hz), 2.87-3.07 (trans and cis of 5H, m), 3.31 (cis of 3H, s), 3.43 (trans of 3H, s), 3.69 (trans and cis of 3H, s), 3.75 (cis of 3H, s), 3.85 (trans of 3H, s), 3.94-4.00 (trans and cis of 1H, m), 5.33 (trans of 1H, s), 5.37 (cis of 1H, s), 6.58 (trans of 1H, s), 6.62-6.84 (cis of 3H, m), 6.95-7.00 (trans of 2H, m), 7.13-7.25 (trans of 2H, cis of 1H, m), 7.32-7.44 (trans and cis of 2H, m), 7.76-7.78 (cis of 1H, m).

### Example 906

3-(2-{2-[8-Chloro-6-(2,3-dimethoxyphenyl)-1-(trifluoromethyl)-4H,6H-[1,2,4]triazolo[4,3-a][4,1]benzothiazepin-4-yl]}ethyl}-1,3-oxazol-5-yl)propanoic acid

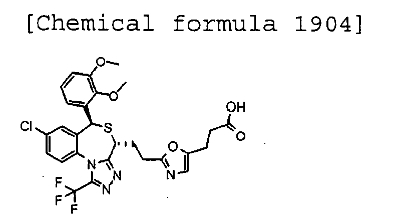

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (103 mg) was obtained as a trans-cis mixture (trans:cis=8:1) from methyl 3-(2-{2-[8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (120 mg).
MS (FAB) m/z: 609 (M+H)⁺.
¹H-NMR (CDCl3) δ: 2.41-2.52 (trans and cis of 1H, m), 2.67 (trans and cis of 2H, t, J = 7.3 Hz), 2.90-3.08 (trans and cis of 5H, m), 3.30 (cis of 3H, s), 3.43 (trans of 3H, s), 3.75 (cis of 3H, s), 3.85 (trans of 3H, s), 3.90-3.99 (trans and cis of 1H, m), 5.32 (trans of 1H, s), 5.37 (cis of 1H, s), 6.62 (trans of 1H, s), 6.63-6.86 (cis of 3H, m), 6.95-7.00 (trans of 2H, m), 7.17-7.23 (trans of 2H, cis of 1H, m), 7.34-7.44 (trans and cis of 2H, m), 7.74-7.81 (cis of 1H, m).
Elemental analysis for: C₂₇H₂₄ClF₃N₄O₅S·0.75H₂O.0.1CHCl₃
Calculated: C, 51.39; H, 4.06; N, 8.81
Found: C, 51.64; H, 3.88; N, 8.41.

### Example 907

Methyl 3-(2-{2-[(4S,6R)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer A)

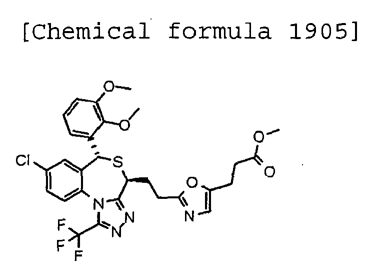

Methyl 3-(2-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer B)

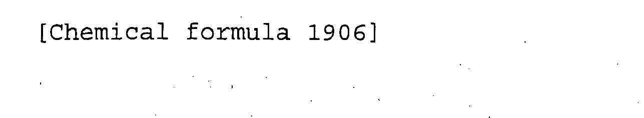

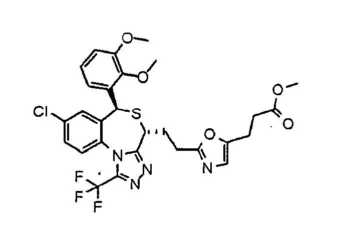

A trans-cis mixture (trans:cis=10:1, 994 mg) of methyl 3-(2-{2-[8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate was dissolved in ethanol. The resulting solution was left to stand at room temperature for one hour. The solid thus precipitated was collected by filtration to give methyl 3-(2-{2-[trans-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate. The resulting compound was optically resolved by a CHIRALPAK AD column (Daicel Chemical Industries, hexane:ethanol =15:85, flow rate: 50 mL/min) into methyl 3-(2-{2-[(4S,6R)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer A) (retention time: 33 min., 391 mg) and methyl 3-(2-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer B) (retention time): 53 min., 382 mg).
Methyl 3-(2-{2-[(4S,6R)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer A)
MS (EI) m/z: 622 M⁺.
¹H-NMR (CDCl3) δ: 2.47 (1H, td, J = 13.7, 7.1 Hz), 2.61 (2H, t, J = 7.3 Hz), 2.89-2.98 (3H, m), 3.03 (2H, t, J = 7.1 Hz), 3.43 (3H, s), 3.69 (3H, s), 3.85 (3H, s), 3.98 (1H, dd, J = 7.9, 6.0 Hz), 5.33 (1H, s), 6.59 (1H, s), 6.96-6.99 (2H, m), 7.14-7.24 (2H, m), 7.36 (1H, d, J = 8.5 Hz), 7.42 (2H, dd, J = 8.5, 2.0 Hz).
Methyl 3-(2-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer B)
MS (EI) m/z: 622 M⁺.
¹H-NMR (CDCl3) δ: 2.47 (1H, td, J = 13.7, 7.1 Hz), 2.61 (2H, t, J = 7.3 Hz), 2.89-2.98 (3H, m), 3.03 (2H, t, J = 7.1 Hz), 3.43 (3H, s), 3.69 (3H, s), 3.85 (3H, s), 3.98 (1H, dd, J = 7.9, 6.0 Hz), 5.33 (1H, s), 6.59 (1H, s), 6.96-6.99 (2H, m), 7.14-7.24 (2H, m), 7.36 (1H, d, J = 8.5 Hz), 7.42 (2H, dd, J = 8.5, 2.0 Hz).

### Example 908

3-(2-(2-[(4S,6R)-8-Chloro-6-(2,3-dimethoxyphenyl)-1-(trifluoromethyl)-4H,6H-[1,2,4]triazolo[4,3-a][4,1]benzothiazepin-4-yl]ethyl)-1,3-oxazol-5-yl)propanoic acid (isomer A)

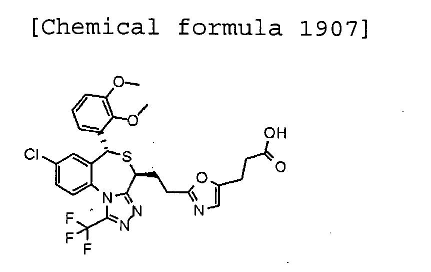

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (385 mg) was obtained from methyl 3-(2-{2-[(4S,6R)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer A) (391 mg).
MS (FAB) m/z: 609 (M+H)⁺.
¹H-NMR (CDCl3) δ: 2.41-2.52 (1H, m), 2.66 (2H, t, J =7.3 Hz), 2.93 (3H, t, J = 7.3 Hz), 3.00-3.07 (2H, m), 3.43 (3H, s), 3.85 (3H, s), 3.93-3.99 (1H, m), 5.33 (1H, s), 6.61 (1H, s), 6.98 (2H, dd, J = 8.3, 1.5 Hz), 7.15-7.23 (2H, m), 7.36 (1H, d, J = 8.5 Hz), 7.42 (1H, dd, J = 8.5, 2.0 Hz). Elemental analysis for: C₂₇H₂₄ClF₃N₄O₅S·1.0H₂O·0.2CHCl₃ Calculated: C, 50.37; H, 4.04; N, 8.58
Found: C, 50.21; H, 3.70; N, 8.57.

### Example 909

3-(2-{2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoic acid (isomer B)

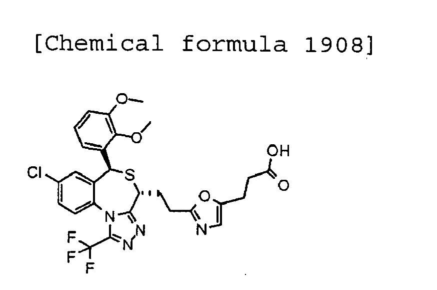

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (351 mg) was obtained from methyl 3-(2-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]ethyl}-1,3-oxazol-5-yl)propanoate (isomer B) (382 mg).
MS (FAB) m/z: 609 (M+H)⁺.
¹H-NMR (CDCl3) δ: 2.41-2.52 (1H, m), 2.66 (2H, t, J = 7.3 Hz), 2.93 (3H, t, J = 7.3 Hz), 3.00-3.07 (2H, m), 3.43 (3H, s), 3.85 (3H, s), 3.93-3.99 (1H, m), 5.33 (1H, s), 6.61 (1H, s), 6.98 (2H, dd, J = 8.3, 1.5 Hz), 7.15-7.23 (2H, m), 7.36 (1H, d, J = 8.5 Hz), 7.42 (1H, dd, J = 8.5, 2.0 Hz). Elemental analysis for: C₂₇H₂₄ClF₃N₄O₅S·0.75H₂O·0.2CHCl₃ Calculated: C, 50.72; H, 3.99; N, 8.64
Found: C, 50.65; H, 3.73; N, 8.56.

### Example 910

Methyl 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoate

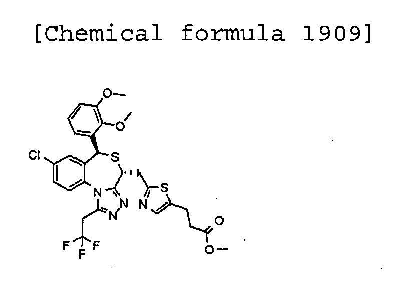

Methyl 3-({2-[(trans)-7-chloro-5-(2,3-dimethoxyphenyl)-2-[hydrazono]-1,5-dihydro-4,1-benzothiazepin-3(3H)-yl]methyl}-1,3-thiazol-5-yl)propanoate (150 mg) was dissolved in tetrahydrofuran (3 mL). To the resulting solution was added 3,3,3-trifluoropropanoyl chloride (200 mg) synthesized in accordance with the process described in the patent (US02143026). The resulting mixture was stirred at room temperature for 2 hours. To the reaction mixture were added a saturated aqueous solution of sodium hydrogen carbonate and ethyl acetate and the layers separated. After the organic layer was washed with saturated brine, the organic layer was dried over anhydrous sodium sulfate and the solvent was distilled off under reduced pressure. The residue was dissolved in acetic acid (3 mL). The resulting solution was heated under reflux for 2 hours. The reaction mixture was concentrated under reduced pressure. To the residue were added a saturated aqueous solution of sodium hydrogen carbonate and chloroform and the layers separated. After the organic layer was washed with saturated brine, the organic layer was dried over anhydrous sodium sulfate and the solvent was distilled off under reduced pressure. The residue was purified by thin layer chromatography (hexane: ethyl acetate =1:3) to give the title compound (99 mg).
MS (FAB) m/z: 639 (M+H)⁺.
¹H-NMR (CDCl3) δ: 2.65 (2H, t, J = 7.4 Hz), 3.10 (2H, t, J = 7.4 Hz), 3.54 (3H, s), 3.61-3.72 (5H, m), 3.76-3.84 (1H, m), 3.85 (3H, s), 4.11 (1H, dd, J = 15.5, 6.7 Hz), 4.50 (1H, t, J = 7.2 Hz), 5.40 (1H, s), 6.95 (1H, d, J = 8.3 Hz), 7.02 (1H, d, J = 2.2 Hz), 7.15 (2H, t, J = 8.3 Hz), 7.23-7.26 (1H, m), 7.37 (1H, s), 7.42 (1H, dd, J = 8.3, 2.2 Hz).

### Example 911

3-(2-([trans-8-Chloro-6-(2,3-dimethoxyphenyl)-1-(2,2,2-trifluoroethyl)-4H,6H-[1,2,4]triazolo[4,3-a][4,1]benzothiazepin-4-yl]methyl)-1,3-thiazol-5-yl)propanoic acid

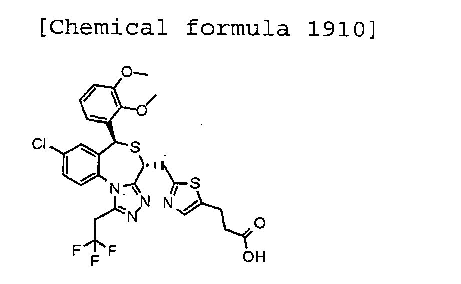

Methyl 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoate (126 mg) was dissolved in 1,4-dioxane (6.30 mL). To the resulting solution was added concentrated hydrochloric acid (1.39 mL) and the resulting mixture was stirred at 60°C for 4 hours. To the reaction mixture were added ice water and chloroform and the layers separated. After the organic layer was dried over anhydrous sodium sulfate, the solvent was distilled off under reduced pressure. By azeotropy of the residue with water, 1,4-dioxane was removed to give the title compound (124 mg). MS (FAB) m/z: 625 (M+H)⁺.
¹H-NMR (CDCl3) δ: 2.68 (2H, t, J = 7.3 Hz), 3.10 (2H, t, J = 7.3 Hz), 3.54 (3H, s), 3.63 (1H, dd, J = 15.4, 7.3 Hz), 3.71 (2H, s), 3.85 (3H, s), 4.12 (1H, dd, J = 15.4, 7.1 Hz), 4.50 (1H, t, J = 7.2 Hz), 5.40 (1H, s), 6.96 (1H, dd, J = 8.3, 1.5 Hz), 7.02 (1H, d, J = 2.0 Hz), 7.12-7.19 (2H, m), 7.23-7.26 (1H, m), 7.39-7.44 (2H, m).
Elemental analysis for: C₂₇H₂₄ClF₃N₄O₄S₂·0.75H₂O·0.2CHCl₃
Calculated: C, 49.50; H, 3.90; N, 8.43
Found: C, 49.76; H, 3.83; N, 8.02.

### Referential Example 818

[3-Methoxy-2-(trifluoromethoxy)phenyl](4-morpholinyl)methanone hydrate

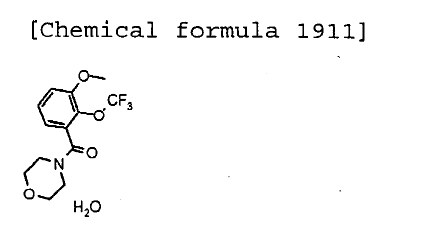

In a similar manner to that employed for the synthesis of methyl 5-({3-[(trans)-8-chloro-1-cyclopropyl-6-(2,3-dimethoxyphenyl)-4H,6H-[1,2,4]triazolo[4,3-a] [4,1]benzothiazepin-4-yl]propanoyl}amino)-4-oxopentanoate, the title compound (2.78 g) was obtained from 3-methoxy-2-(trifluoromethoxy)benzoic acid (2.00 g) synthesized in accordance with the process described in the document (Chem. Eur. J., 8, 799-804(2002)) and morpholine (1.48 mL).
MS (FAB) m/z: 306 (M+H)⁺.
¹H-NMR (CDCl3) δ: 3.19-3.28 (1H, m), 3.29-3.38 (1H, m), 3.56-3.82 (5H, m), 3.86-3.92 (4H, m), 6.96 (1H, dd, J = 7.8, 1.5 Hz), 7.05 (1H, dd, J = 7.8, 1.5 Hz), 7.33 (1H, t, J = 7.8 Hz).

### Referential Example 819

N-{4-Chloro-2-[3-methoxy-2-(trifluoromethoxy)benzoyl]}phenyl}-2,2-dimethylpropanamide

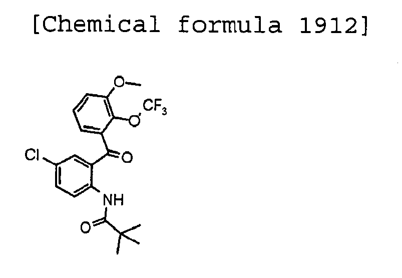

N-(4-Chlorophenyl)-2,2-dimethylpropanamide (16.9 g) was dissolved in anhydrous tetrahydrofuran (280 mL). To the resulting solution was added dropwise an n-hexane solution (2.55M, 72.0 mL) of n-butyl lithium at -20°C. After stirring for 2 hours under ice cooling, an anhydrous tetrahydrofuran (50 mL) of [3-methoxy-2-(trifluoromethoxy)phenyl](4-morpholinyl)methanone (26.8 g) which had been made anhydrous by azeotropy with benzene was added dropwise at -20°C. The reaction mixture was warmed to room temperature and stirred for one hour. A saturated aqueous solution of ammonium chloride was added to terminate the reaction. To the reaction mixture was added ethyl acetate and the layers separated. After the organic layer was dried over anhydrous sodium sulfate, the solvent was distilled off under reduced pressure. The solid thus precipitated was subjected to slurry washing with hexane-ethyl acetate (hexane:ethyl acetate=20:1) to give the title compound (29.1 g).
MS (FAB) m/z: 430 (M+H)⁺.
¹H-NMR (CDCl3) δ: 1.36 (9H, s), 3.95 (3H, s), 6.99 (1H, dd, J = 7.7, 1.3 Hz), 7.20 (1H, dd, J = 8.4, 1.3 Hz), 7.33 (1H, d, J = 2.4 Hz), 7.41 (1H, t, J = 7.7 Hz), 7.53 (1H, dd, J = 9.0, 2.4 Hz), 8.81 (1H, d, J = 9.0 Hz), 11.43 (1H, s).

### Referential Example 820

(2-Amino-5-chlorophenyl)[3-methoxy-2-(trifluoromethoxy)phenyl]methanone

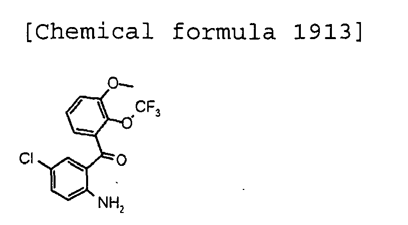

N-{4-Chloro-2-[3-methoxy-2-(trifluoromethoxy)benzoyl]}phenyl}-2,2-dimethylpropanamide (29.1 g) was dissolved in methanol (100 mL). To the resulting solution were added water (100 mL) and potassium hydroxide (45.6 g). The resulting mixture was heated under reflux overnight. After the reaction mixture was cooled to a temperature of ice cooling, ice water (1000 mL) was added in portions for dilution. To the reaction mixture was added chloroform and the layers separated. The organic layer was dried over anhydrous sodium sulfate. The solvent was distilled off under reduced pressure. The residue was purified by silica gel column chromatography (hexane:ethyl acetate= from 20:1 to 5:1) to give the title compound (19.8 g).
MS (EI) m/z: 345 M⁺.
¹H-NMR (CDCl3) δ: 3.94 (3H, s), 6.33 (2H, br s), 6.67 (1H, d, J = 8.8 Hz), 6.95 (1H, dd, J = 8.0, 1.3 Hz), 7.13 (1H, d, J = 8.0 Hz), 7.17 (1H, d, J = 2.4 Hz), 7.23 (1H, dd, J = 8.8, 2.4 Hz), 7.36 (1H, t, J = 8.0 Hz).

### Referential Example 821

(2-Amino-5-chlorophenyl)[3-methoxy-2-(trifluoromethoxy)phenyl]methanol

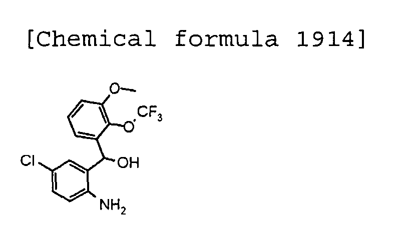

In a similar manner to that employed for the synthesis of methyl (E)-3-(1-(4-chloro-2-((2,3-dimethoxyphenyl)(hydroxy)methyl)phenyl)-1H-pyrrol-2-yl)-2-propenoate, the title compound (20.4 g) was obtained from (2-amino-5-chlorophenyl)[3-methoxy-2-(trifluoromethoxy)phenyl]methanone (19.8 g).
MS (EI) m/z: 347 M⁺.
¹H-NMR (CDCl3) δ: 3.89 (3H, s), 6.14 (1H, s), 6.60 (1H, d, J = 8.8 Hz), 6.97 (1H, dd, J = 8.1, 1.3 Hz), 7.03-7.08 (2H, m), 7.29 (1H, t, J = 8.1 Hz), 7.36 (1H, s).

### Referential Example 822

2-{trans-7-Chloro-5-[3-methoxy-2-(trifluoromethoxy)phenyl]-2-oxo-1,2,3,5-tetrahydro-4,1-benzothiazepin-3-yl}acetic acid

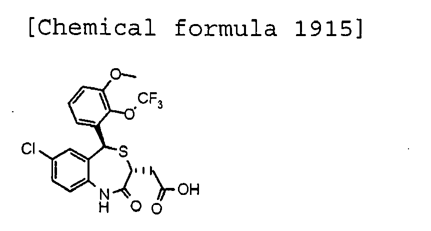

(2-Amino-5-chlorophenyl)[3-methoxy-2-(trifluoromethoxy)phenyl]methanol (3.14 g) was dissolved in dichloroethane (80 mL). To the resulting solution were added tosic acid hydrate (1.13 g) and thiomalic acid (1.24 g) and the resulting mixture was stirred at 80°C for 80 minutes. The reaction mixture was cooled to room temperature and a 1N aqueous sodium hydroxide solution (10.6 mL), 3N hydrochloric acid (3 mL), water (200 mL), chloroform (200 mL) and tetrahydrofuran (20 mL) were added thereto. After confirmation that the water layer had a pH of from 3 to 4, the layers were separated. The organic layer was dried over anhydrous sodium sulfate. The solvent was distilled off under reduced pressure. The residue was suspended in toluene (100 mL). The resulting suspension was heated under reflux overnight. The reaction mixture was concentrated under reduced pressure. To the residue were added lithium hydroxide hydrate (661 mg), methanol (45 mL), water (28 mL) and tetrahydrofuran (7 mL). The resulting mixture was stirred at 50°C for 24 hours. The reaction mixture was concentrated under reduced pressure. To the residue were added 3N hydrochloric acid (6 mL), water (200 mL) and ethyl acetate. After confirmation that the water layer had a pH of from 3 to 4, the layers were separated. The organic layer was dried over anhydrous sodium sulfate. The solvent was distilled off under reduced pressure. The solid thus precipitated was subjected to slurry washing with a hexane:ethyl acetate solvent mixture (hexane:ethyl acetate=2:1) to give the title compound (2.12 g).
MS (FAB) m/z: 462 (M+H)⁺.
¹H-NMR (CDCl3) δ: 2.40 (1H, dd, J = 16.8, 3.9 Hz), 2.76 (1H, dd, J = 16.8, 10.3 Hz), 3.63 (1H, dd, J = 10.3, 3.9 Hz), 3.88 (3H, s), 5.90 (1H, s), 6.72 (1H, d, J = 2.4 Hz), 7.16 (1H, d, J = 8.5 Hz), 7.36 (1H, d, J = 8.1 Hz), 7.42-7.47 (2H, m), 7.57 (1H, t, J = 8.1 Hz), 10.10 (1H, s), 12.44 (1H, s).

### Referential Example 823

Methyl 2-{trans-7-chloro-5-[3-methoxy-2-(trifluoromethoxy)phenyl]-2-oxo-1,2,3,5-tetrahydro-4,1-benzothiazepin-3-yl}acetate

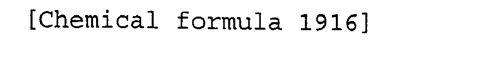

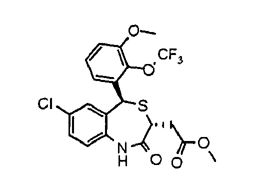

2-{trans-7-Chloro-5-[3-methoxy-2-(trifluoromethoxy)phenyl]-2-oxo-1,2,3,5-tetrahydro-4,1-benzothiazepin-3-yl}acetic acid (2.26 g) was dissolved in methanol (30 mL). To the resulting solution was added concentrated sulfuric acid (100 µl) and the resulting mixture was heated under reflux for one hour. After cooling to room temperature, a saturated aqueous solution of sodium hydrogen carbonate and ethyl acetate were added to the reaction mixture and the layers separated. The organic layer was dried over anhydrous sodium sulfate. The solvent was distilled off under reduced pressure. The solid thus precipitated was subjected to slurry washing with a hexane:ethyl acetate solvent mixture (hexane:ethyl acetate=7:1) to give the title compound (2.17 g).
MS (FAB) m/z: 476 (M+H)⁺.
¹H-NMR (CDCl3) δ: 2.43 (1H, dd, J = 17.2, 4.0 Hz), 3.12 (1H, dd, J = 17.2, 10.1 Hz), 3.67 (3H, s), 3.85 (1H, dd, J = 10.1, 4.0 Hz), 3.89 (3H, s), 6.13 (1H, s), 6.85 (1H, d, J = 2.2 Hz), 7.06 (1H, dd, J = 7.3, 2.7 Hz), 7.11 (1H, d, J = 8.5 Hz), 7.27 (2H, dd, J = 8.3, 2.2 Hz), 7.38-7.45 (2H, m).

### Referential Example 824

Methyl 2-{trans-7-chloro-5-[3-methoxy-2-(trifluoromethoxy)phenyl]-2-thioxo-1,2,3,5-tetrahydro-4,1-benzothiazepin-3-yl}acetate

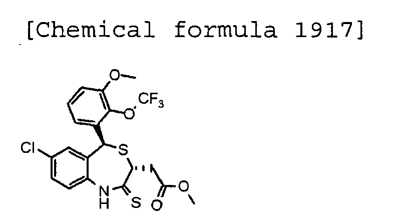

In a similar manner to that employed for the synthesis of methyl 2-[(trans)-7-chloro-5-(2,3-dimethoxyphenyl)-2-thioxo-1,2,3,5-tetrahydro-4,1-benzothiazepin-3-yl]acetate, the title compound (2.37 g) was obtained from methyl 2-{trans-7-chloro-5-[3-methoxy-2-(trifluoromethoxy)phenyl]-2-oxo-1,2,3,5-tetrahydro-4,1-benzothiazepin-3-yl}acetate(2.17 g).
MS (EI) m/z: 491 M⁺.
¹H-NMR (CDCl3) δ: 2.54-2.65 (1H, m), 3.44 (1H, dd, J = 16.8, 10.0 Hz), 3.68 (3H, s), 3.89 (3H, s), 4.08 (1H, dd, J = 10.0, 4.1 Hz), 5.96 (1H, s), 6.83 (1H, d, J = 2.2 Hz), 7.06 (1H, d, J = 8.0 Hz), 7.12 (1H, d, J = 8.5 Hz), 7.28-7.44 (3H, m), 9.13 (1H, br s).

### Example 912

Methyl 2-[8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate

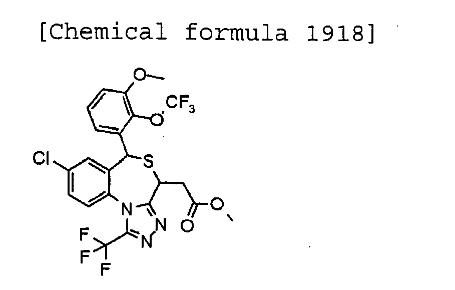

In a similar manner to that employed for the synthesis of methyl 2-[(trans)-8-chloro-6-(2,3-dimethoxyphenyl)-1-(trifluoromethyl)-4H,6H-[1,2,4]triazolo[4,3-a] [4,1]benzothiazepin-4-yl]acetate, the title compound (59 mg) was obtained as a trans-cis mixture (trans:cis=4:1) from methyl 2-{trans-7-chloro-5-[3-methoxy-2-(trifluoromethoxy)phenyl]-2-thioxo-1,2,3,5-tetrahydro-4,1-benzothiazepin-3-yl}acetate (150 mg).
MS (EI) m/z: 567 M⁺.
¹H-NMR (CDCl3) δ: 2.91 (trans of 2H, dd, J = 17.3, 5.7 Hz), 3.00 (cis of 2H, dd, J = 16.8, 5.4 Hz), 3.59-3.69 (trans and cis of 2H, m), 3.71 (trans and cis of 3H, s), 3.76 (cis of 3H, s), 3.87 (trans of 3H, s), 4.29 (cis of 1H, dd, J = 5.4, 9.0 Hz), 4.36 (trans of 1H, dd, J = 8.7, 5.7 Hz), 5.33 (trans of 1H, s), 5.38 (cis of 1H, s), 6.83-6.88 (cis of 2H, m), 7.05-7.09 (trans of 2H, m), 7.16 (cis of 1H, d, J = 8.0 Hz), 7.37-7.50 (trans of 4H, cis of 2H, m), 7.72-7.74 (cis of 1H, m).

### Example 913

2-[8-Chloro-6-[3-methoxy-2-(trifluoromethoxy)phenyl]-1-(trifluoromethyl)-4H,6H-[1,2,4]triazolo[4,3-a][4,1]benzothiazepin-4-yl]acetic acid

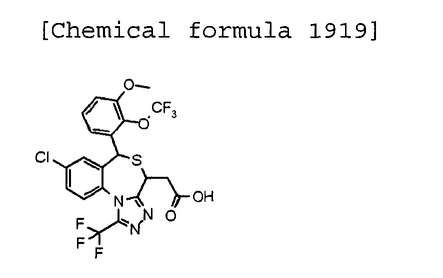

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (51 mg) was obtained as a trans-cis mixture (trans:cis=4:1) from methyl 2-[8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate (53 mg).
MS (FAB) m/z: 554 (M+H)⁺. ¹H-NMR (CDCl3) δ: 2.91-2.93 (trans of 2H, m), 3.00-3.08 (cis of 2H, m), 3.60-3.80 (trans and cis of1H, cis of 3H, m), 3.88 (trans of 3H, s), 4.22-4.28 (cis of 1H, m), 4.32 (trans of 1H, dd, J = 8.5, 5.4 Hz), 5.33 (trans of 1H, s), 5.38 (cis of 1H, s), 6.84-6.88 (cis of 2H, m), 7.05-7.20 (trans of 2H, cis of 1H, m), 7.34-7.37 (trans of 4H, cis 1H, m), 7.73 (cis of 1H, m).
Elemental analysis for: C₂₁H₁₄ClF₆N₃O₄S·0.1 Hexane·0.2 dioxane
Calculated: C, 46.10; H, 2.94; N, 7.20
Found: C, 46.09; H, 2.95; N, 6.83.

### Example 914

Methyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate
(isomer A)

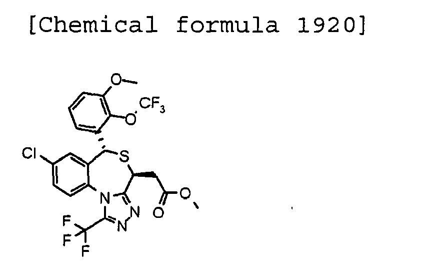

Methyl 2-[(4R,6S)-8-chloro-6-[3-methoxy-2-(trifluoromethoxy)phenyl]-1-(trifluoromethyl)-4H,6H-[1,2,4]triazolo[4,3-a] [4,1]benzothiazepin-4-yl]acetate
(isomer B)

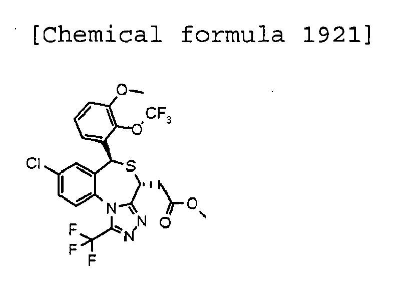

The trans-cis mixture (trans:cis=4:1, 1.07 g) of methyl 2-[8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate was dissolved in ethanol. The resulting solution was left to stand at room temperature for one hour. The crystals thus precipitated were collected by filtration to give methyl 2-[trans-8-chloro-6-[3-methoxy-2-(trifluoromethoxy)phenyl]-1-(trifluoromethyl)-4H,6H-[1,2,4]triazolo[4,3-a] [4,1]benzothiazepin-4-yl] acetate (805 mg). The resulting compound was optically resolved by a CHIRALPAK AD column (Daicel Chemical Industries, hexane:ethanol=35:65, flow rate: 50 mL/min) into methyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate (isomer A, retention time: 25 min., 400 mg) and methyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate (isomer B, retention time: 35 min., 368 mg).
Methyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate
(isomer A)
MS (EI) m/z: 567 M⁺.
¹H-NMR (CDCl3) δ: 2.91 (1H, dd, J = 17.2, 5.7 Hz), 3.63 (1H, q, J = 8.6 Hz), 3.71 (3H, s), 3.87 (3H, s), 4.36 (1H, dd, J = 8.6, 5.7 Hz), 5.33 (1H, s), 7.05-7.09 (2H, m), 7.36-7.49 (4H, m).
Methyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate(isomer B)
MS (EI) m/z: 567 M⁺.
¹H-NMR (CDCl3) δ: 2.91 (1H, dd, J = 17.2, 5.7 Hz), 3.63 (1H, q, J = 8.6 Hz), 3.71 (3H, s), 3.87 (3H, s), 4.36 (1H, dd, J = 8.6, 5.7 Hz), 5.33 (1H, s), 7.05-7.09 (2H, m), 7.36-7.49 (4H, m).

### Example 915

2-[(4S,6R)-8-Chloro-6-[3-methoxy-2-(trifluoromethoxy)phenyl]-1-(trifluoromethyl)-4H,6H-[1,2,4]triazolo[4,3-a][4,1]benzothiazepin-4-yl]acetic acid
(isomer A)

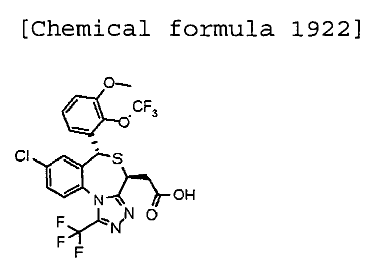

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (isomer A, 450 mg) was obtained from methyl 2-[(4S,6R)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate (isomer A, 434 mg).
MS (FAB) m/z: 554 (M+H)⁺.
¹H-NMR (CDCl3) δ: 2.95 (1H, dd, J = 17.7, 5.0 Hz), 3.64 (1H, dd, J = 17.7, 9.5 Hz), 3.88 (3H, s), 4.32 (1H, dd, J = 5.0, 9.5 Hz), 5.33 (1H, s), 7.05-7.09 (2H, m), 7.36-7.50 (4H, m).
Elemental analysis for: C₂₁H₁₄ClF₆N₃O₄S·1.0H₂O
Calculated: C, 44.11; H, 2.82; N, 7.35
Found: C, 44.45; H, 2.57; N, 7.04.

### Example 916

2-[(4R,6S)-8-Chloro-6-[3-methoxy-2-(trifluoromethoxy)phenyl]-1-(trifluoromethyl)-4H,6H-[1,2,4]triazolo[4,3-a][4,1]benzothiazepin-4-yl]acetic acid
(isomer B)

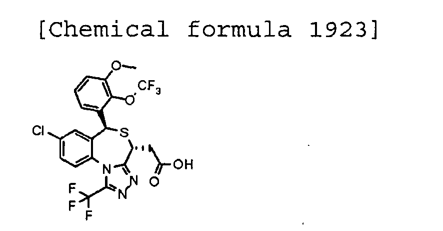

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a] [4,1]benzothiazepin-4-yl]methyl}-1,3-thiazol-5-yl)propanoic acid, the title compound (isomer B, 421 mg) was obtained from methyl 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetate (isomer B, 400 mg).
MS (FAB) m/z: 554 (M+H)⁺.
¹H-NMR (CDCl3) δ: 2.95 (1H, dd, J = 17.7, 5.0 Hz), 3.64 (1H, dd, J = 17.7, 9.5 Hz), 3.88 (3H, s), 4.32 (1H, dd, J = 5.0, 9.5 Hz), 5.33 (1H, s), 7.05-7.09 (2H, m), 7.36-7.50 (4H, m) .
Elemental analysis for: C₂₁H₁₄ClF₆N₃O₄S·0.3H₂O·0.2 dioxane
Calculated: C, 45.11; H, 2.81; N, 7.24
Found: C, 44.96; H, 2.78; N, 7.03.

### Example 917

Ethyl 2-(1-{2-[(4R,6S)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetyl}-4-piperidinyl)acetate

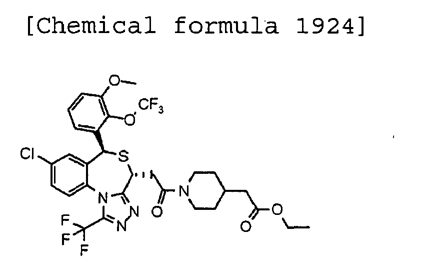

In a similar manner to that employed for the synthesis of ethyl 2-(1-{2-[8-chloro-6-(2,3-dimethoxyphenyl)-1-(trifluoromethyl)-4H,6H-[1,2,4]triazolo[4,3-a][4,1]benzothiazepin-4-yl]acetyl}-4-piperidinyl) acetate, the title compound (163 mg) was obtained from 2-[(4R,6S)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetic acid (isomer B, 150 mg).
MS (FAB) m/z: 707 (M+H)⁺.
¹H-NMR (CDCl3) δ: 1.08-1.21 (1H, m), 1.25-1.35 (3H, m), 1.37-1.48 (1H, m), 1.69-1.89 (2H, m), 2.00-2.11 (1H, m), 2.23-2.29 (2H, m), 2.55-2.65 (1H, m), 2.77-2.89 (1H, m), 3.05-3.20 (1H, m), 3.61-3.71 (1H, m), 3.87 (3H, s), 3.91-4.05 (1H, m), 4.10-4.18 (2H, m), 4.46-4.60 (2H, m), 5.30 (1H, s), 7.04-7.09 (2H, m), 7.39-7.47(4H, m).

### Example 918

2-(1-{2-[(4R,6S)-8-Chloro-6-[3-methoxy-2-(trifluoromethoxy)phenyl]-1-(trifluoromethyl)-4H,6H-[1,2,4]triazolo[4,3-a][4,1]benzothiazepin-4-yl]acetyl}-4-piperidinyl)acetic acid

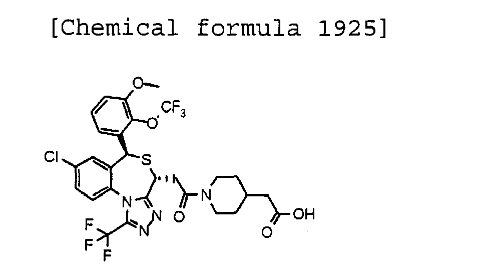

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a] [4,1]benzothiazepin-4-yl]methyl}-1,3-thiazol-5-yl)propanoic acid, the title compound (115 mg) was obtained from ethyl 2-(1-{2-[(4R,6S)-8-chloro-6-[3-methoxy-2-(trifluoromethoxy)phenyl]-1-(trifluoromethyl)-4H,6H-[1,2,4]triazolo[4,3-a][4,1]benzothiazepin-4-yl]acetyl}-4-piperidinyl)acetate (160 mg).
MS (FAB) m/z: 679 (M+H)⁺.
¹H-NMR (CDCl3) δ: 1.09-1.51 (2H, m), 1.72-1.94 (2H, m), 1.99-2.12 (1H, m), 2.28-2.36 (2H, m), 2.55-2.68 (1H, m), 2.76-2.90 (1H, m), 3.04-3.22 (1H, m), 3.61-3.79 (1H, m), 3.87 (3H, s), 3.94-4.07 (1H, m), 4.46-4.62 (2H, m), 5.30 (1H, s), 7.03-7.09 (2H, m), 7.38-7.47 (4H, m).
Elemental analysis for: C₂₈H₂₅ClF₆N₄O₅S·1.0H₂O·0.2CHCl₃
Calculated: C, 47.16; H, 3.79; N, 7.75
Found: C, 47.20; H, 3.57; N, 7.59.

### Referential Example 825

2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]-1-ethanol

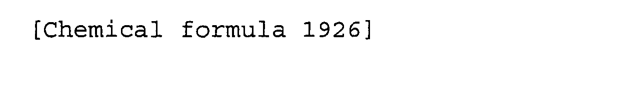

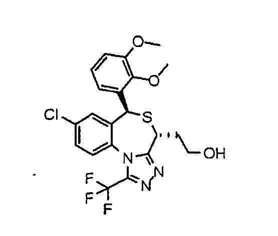

In a similar manner to that employed for the synthesis of trans-1-allyl-7-chloro-5-(2,3-dimethoxyphenyl)-3-(2-hydroxyethyl)-1,5-dihydro-4,1-benzothiazepin-2(3H)-one, the title compound (706 mg) was obtained from 2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]acetic acid (950 mg).
MS (EI) m/z: 485 M⁺.
¹H-NMR (CDCl3) δ: 2.26-2.35 (1H, m), 2.67-2.76 (1H, m), 3.44 (3H, s), 3.83-3.89 (4H, m), 3.92-3.98 (1H, m), 4.10 (1H, dd, J = 7.6, 6.1 Hz), 5.34 (1H, s), 6.96-7.00 (2H, m), 7.15-7.25 (2H, m), 7.35 (1H, d, J = 8.8 Hz), 7.43 (1H, dd, J = 8.8, 2.0 Hz).

### Example 919

Ethyl 3-(2-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)propanoate

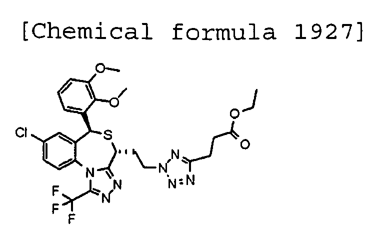

In a similar manner to that employed for the synthesis of 2-[(4R, 6S)-8-chloro-6-(2,3-dimethoxyphenyl)-4H,6H-pyrrolo[1,2-a][4,1]benzoxazepin-4-yl]ethyl methanesulfonate and ethyl 2-(1-{2-[(4R,6S)-8-chloro-6-(2,3-dimethoxyphenyl)-4H,6H-pyrrolo[1,2-a][4,1]benzoxazepin-4-yl]ethyl}-1H-1,2,4-triazol-3-yl)acetate, the title compound (236 mg) was obtained from 2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]-1-ethanol (300 mg).
MS (FAB) m/z: 638 (M+H)⁺.
¹H-NMR (CDCl3) δ: 1.25 (3H, t, J = 7.1 Hz), 2.55-2.65 (1H, m), 2.75 (2H, t, J = 7.6 Hz), 3.10-3.19 (3H, m), 3.44 (3H, s), 3.81-3.86 (4H, m), 4.14 (2H, q, J = 7.1 Hz), 4.81-4.89 (1H, m), 4.90-4.99 (1H, m), 5.35 (1H, s), 6.95 (1H, d, J = 2.0 Hz), 6.96-7.01 (1H, m), 7.16-7.18 (2H, m), 7.38 (1H, d, J = 8.5 Hz), 7.44 (1H, dd, J = 8.5, 2.4 Hz).

### Example 920

3-(2-{2-[(4R,6S)-8-Chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)propanoic acid

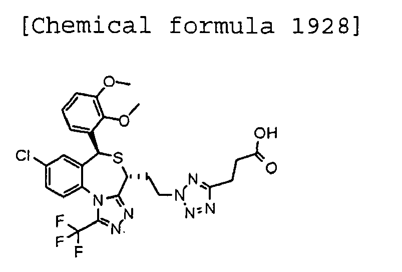

In a similar manner to that employed for the synthesis of 3-(2-{[trans-8-chloro-6-(2,3-dimethoxyphenyl)-4H,6H-imidazo[1,2-a][4,1]benzothiazepin-4-yl]methyl}-1,3-thiazol-5-yl)propanoic acid, the title compound (191 mg) was obtained from ethyl 3-(2-{2-[(4R,6S)-8-chloro-6-(2,3-dimethoxyphenyl)-1-(trifluoromethyl)-4H,6H-[1,2,4]triazolo[4,3-a][4,1]benzothiazepin-4-yl]ethyl}-2H-1,2,3,4-tetrazol-5-yl)propanoate (231 mg).
MS (FAB) m/z: 610 (M+H)⁺.
¹H-NMR (CDCl3) δ: 2.56-2.66 (1H, m), 2.83 (2H, t, J = 7.4 Hz), 3.10-3.20 (3H, m), 3.44 (3H, s), 3.82-3.87 (4H, m), 4.82-4.90 (1H, m), 4.90-4.99 (1H, m), 5.35 (1H, s), 6.95-7.00 (2H, m), 7.16-7.19 (2H, m), 7.38 (1H, d, J = 8.5 Hz), 7.45 (1H, dd, J = 8.5, 2.4 Hz).
Elemental analysis for: C₂₅H₂₃ClF₃N₇O₄S·0.8H₂O
Calculated: C, 48.09; H, 3.97; N, 15.70
Found: C, 48.50; H, 3.80; N, 15.30.

### Referential Example 826

tert-Butyl 2-bromo-4-chloro-6-fluorophenylcarbamate

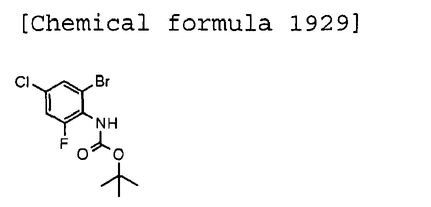

2-Bromo-4-chloro-6-fluoroaniline (25.0 g) was dissolved in tetrahydrofuran (1000 mL). To the resulting solution were added di-t-butyl dicarbonate (72.9 g) and N,N-dimethylaminopyridine (1.36 g). The resulting mixture was heated under reflux for 2 hours. After the reaction mixture was cooled to room temperature, ethyl acetate and 0.5N hydrochloric acid were added thereto and the layers separated. The organic layer was washed successively with a saturated aqueous solution of sodium hydrogen carbonate and saturated brine and then, dried over anhydrous sodium sulfate. The solvent was distilled off under reduced pressure. The residue was dissolved in methanol (1000 mL). To the resulting solution was added anhydrous potassium carbonate (46.0 g). The resulting mixture was heated under reflux overnight. After cooling to room temperature, the insoluble material was filtered off through a Kiriyama funnel. The filtrate was concentrated under reduced pressure. To the residue was added hexane and the solid thus precipitated was collected by filtration to give the title compound (31.9 g).
MS (FAB) m/z: 325 (M+H)⁺.
¹H-NMR (CDCl3) δ: 1.50 (9H, s), 5.97 (1H, br s), 7.13 (1H, dd, J = 9.5, 2.4 Hz), 7.41 (1H, t, J = 2.0 Hz).

### Referential Example 827

tert-Butyl 4-chloro-2-[(2,3-dimethoxyphenyl)(hydroxy)methyl]-6-fluorophenylcarbamate

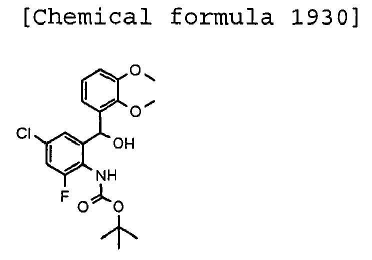

tert-Butyl 2-bromo-4-chloro-6-fluorophenylcarbamate (27.8 g) was dissolved in anhydrous tetrahydrofuran (600 mL). To the resulting solution was added dropwise n-butyl lithium (a 2.55M solution, 73.9 mL) at -95°C over 50 minutes. After the reaction mixture was stirred at -95°C for 30 minutes, an anhydrous tetrahydrofuran solution (100 mL) of dimethoxybenzaldehyde (15.5 g) was added dropwise at -95°C. The reaction mixture was warmed to -10°C and a saturated aqueous solution of ammonium chloride was then added to terminate the reaction. To the reaction mixture was added ethyl acetate and the layers separated. The organic layer was dried over anhydrous sodium sulfate and the solvent was distilled off under reduced pressure. The residue was purified by silica gel column chromatography (hexane:ethyl acetate=6:1) to give the title compound (2.4 g).
MS (EI) m/z: 411 M⁺.
¹H-NMR (CDCl3) δ: 1.48 (9H, s), 3.66 (3H, s), 3.85 (3H, s), 6.12-6.15 (1H, m), 6.65 (1H, br s), 6.82-6.96 (1H, m), 7.02-7.16 (4H, m).

### Referential Example 828

(2-Amino-5-chloro-3-fluorophenyl)(2,3-dimethoxyphenyl)methanol

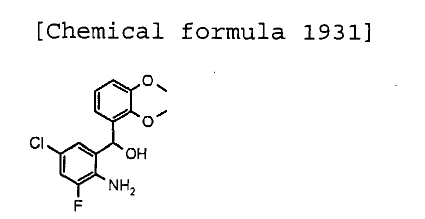

In a similar manner to that employed for the synthesis of 2-amino-5-chloro-3-[(2,3-dimethoxyphenyl)(hydroxy)methyl]pyridine, the title compound (477 mg) was obtained from tert-butyl 4-chloro-2-[(2,3-dimethoxyphenyl)(hydroxy)methyl]-6-fluorophenylcarbamate (675 mg).
MS (EI) m/z: 311 M⁺.
¹H-NMR (CDCl3) δ: 3.85 (3H, s), 3.89 (3H, s), 6.06 (1H, s), 6.82-6.97 (4H, m), 7.07 (1H, t, J = 8.0 Hz).

### Referential Example 829

2-[trans-7-Chloro-5-(2,3-dimethoxyphenyl)-9-fluoro-2-oxo-1,2,3,5-tetrahydro-4,1-benzothiazepin-3-yl]acetic acid

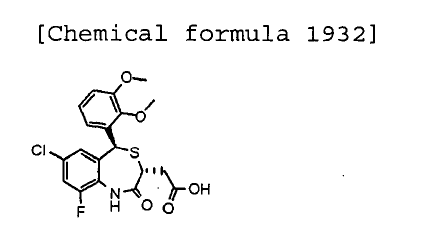

In a similar manner to that employed for the synthesis of 2-{trans-7-chloro-5-[3-methoxy-2-(trifluoromethoxy)phenyl]-2-oxo-1,2,3,5-tetrahydro-4,1-benzothiazepin-3-yl}acetic acid, the title compound (6.07 g) was obtained from (2-amino-5-chloro-3-fluorophenyl)(2,3-dimethoxyphenyl)methanol (470 mg).
MS (FAB) m/z : 426 (M+H)⁺.
¹H-NMR (CDCl3) δ: 2.42 (1H, dd, J = 16.6, 3.9 Hz), 2.76 (1H, dd, J = 16.6, 10.2 Hz), 3.49 (3H, s), 3.63 (1H, dd, J = 10.2, 3.9 Hz), 3.84 (3H, s), 6.00 (1H, s), 6.53 (1H, s), 7.17 (1H, dd, J = 7.8, 1.5 Hz), 7.21-7.29 (2H, m), 7.57 (1H, dd, J = 9.3, 2.0 Hz), 10.03 (1H, s), 12.44 (1H, br s).

### Referential Example 830

Methyl 2-[trans-7-chloro-5-(2,3-dimethoxyphenyl)-9-fluoro-2-oxo-1,2,3,5-tetrahydro-4,1-benzothiazepin-3-yl]acetate

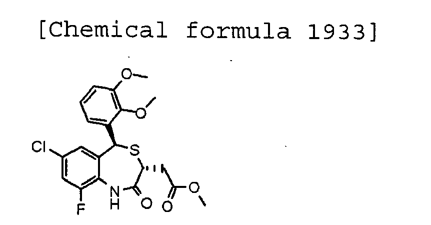

In a similar manner to that employed for the synthesis of methyl 2-{trans-7-chloro-5-[3-methoxy-2-(trifluoromethoxy)phenyl]-2-oxo-1,2,3,5-tetrahydro-4,1-benzothiazepin-3-yl}acetate, the title compound (6.42 g) was obtained from 2-[trans-7-chloro-5-(2,3-dimethoxyphenyl)-9-fluoro-2-oxo-1,2,3,5-tetrahydro-4,1-benzothiazepin-3-yl]acetic acid (6.31 g).
MS (FAB) m/z: 440 (M+H)⁺.
¹H-NMR (CDCl3) δ: 2.54 (1H, dd, J = 16.6, 4.0 Hz), 2.84 (1H, dd, J = 16.6, 10.3 Hz), 3.51 (3H, s), 3.59 (3H, s), 3.65-3.70 (1H, m), 3.84 (3H, s), 6.01 (1H, s), 6.54 (1H, s), 7.15-7.29 (3H, m), 7.58 (1H, dd, J = 9.3, 2.2 Hz), 10.07 (1H, s).

### Referential Example 831

Methyl 2-[trans-7-chloro-5-(2,3-dimethoxyphenyl)-9-fluoro-2-thioxo-1,2,3,5-tetrahydro-4,1-benzothiazepin-3-yl]acetate

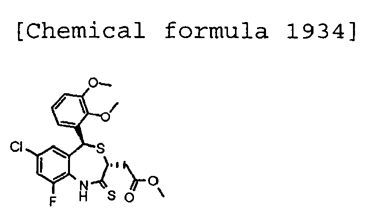

In a similar manner to that employed for the synthesis of methyl 2-{trans-7-chloro-5-[3-methoxy-2-(trifluoromethoxy)phenyl]-2-thioxo-1,2,3,5-tetrahydro-4,1-benzothiazepin-3-yl}acetate, the title compound (5.44 g) was obtained from methyl 2-[trans-7-chloro-5-(2,3-dimethoxyphenyl)-9-fluoro-2-oxo-1,2,3,5-tetrahydro-4,1-benzothiazepin-3-yl]acetate (6.40 g).
MS (EI) m/z: 455 M⁺.
¹H-NMR (CDCl3) δ: 2.65 (1H, dd, J = 16.8, 4.3 Hz), 3.42 (1H, dd, J = 16.8, 9.5 Hz), 3.64 (3H, s), 3.68 (3H, s), 3.88 (3H, s), 4.10 (1H, dd, J = 4.3, 9.5 Hz), 6.02 (1H, s), 6.63-6.65 (1H, m), 6.98 (1H, dd, J = 8.1, 2.0 Hz), 7.12 (1H, dd, J = 8.8, 2.0 Hz), 7.17 (1H, t, J = 8.1 Hz), 7.24 (1H, dd, J = 8.1, 1.5 Hz), 8.89 (1H, s).

### Example 921

Methyl 2-[trans-8-chloro-6-(2,3-dimethoxyphenyl)-10-fluoro-1-(trifluoromethyl)-4H,6H-[1,2,4]triazolo[4,3-a] [4,1]benzothiazepin-4-yl]acetate

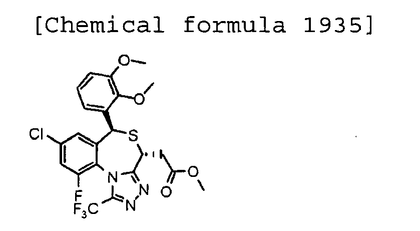

In a similar manner to that employed for the synthesis of methyl 2-[(trans)-8-chloro-6-(2,3-dimethoxyphenyl)-1-(trifluoromethyl)-4H,6H-[1,2,4]triazolo[4,3-a][4,1]benzothiazepin-4-yl]acetate, a trans-cis mixture (trans:cis=4:1, 82 mg) of methyl 2-[8-chloro-6-(2,3-dimethoxyphenyl)-10-fluoro-1-(trifluoromethyl)-4H,6H-[1,2,4]triazolo[4,3-a][4,1]benzothiazepin-4-yl]acetate was obtained from methyl 2-[trans-7-chloro-5-(2,3-dimethoxyphenyl)-9-fluoro-2-thioxo-1,2,3,5-tetrahydro-4,1-benzothiazepin-3-yl]acetate (150 mg). The resulting compound was dissolved in ethanol and the resulting solution was left to stand at room temperature for one hour. The crystals thus precipitated were collected by filtration to give the title compound (49 mg).
MS (FAB) m/z: 532 (M+H)⁺.
¹H-NMR (CDCl3) δ: 2.97 (1H, dd, J = 17.2, 6.0 Hz), 3.49 (3H, s), 3.60-3.68 (1H, m), 3.72 (3H, s), 3.85 (3H, s), 4.32 (1H, dd, J = 8.3, 6.2 Hz), 5.30 (1H, s), 6.77-6.79 (1H, m), 6.97-7.01 (1H, m), 7.15-7.19 (2H, m), 7.26-7.29 (1H, m).

### Example 922

2-[trans-8-Chloro-6-(2,3-dimethoxyphenyl)-10-fluoro-1-(trifluoromethyl)-4H,6H-[1,2,4]triazolo[4,3-a][4,1]benzothiazepin-4-yl]acetic acid

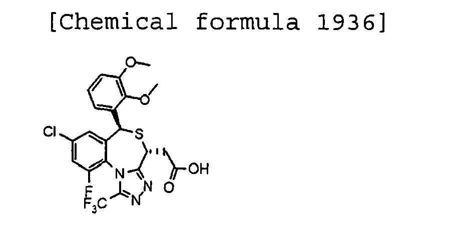

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (48 mg) was obtained from methyl 2-[trans-8-chloro-6-(2,3-dimethoxyphenyl)-10-fluoro-1-(trifluoromethyl)-4H,6H-[1,2,4]triazolo[4,3-a] [4, 1] benzothiazepin-4-yl] acetate (46 mg) .
MS (FAB) m/z: 518 (M+H)⁺.
¹H-NMR (CDCl3) δ: 3.04 (1H, dd, J = 17.6, 5.6 Hz), 3.49 (3H, s), 3.68 (1H, dd, J = 17.6, 8.4 Hz), 3.86 (3H, s), 4.29 (1H, dd, J = 8.4, 5.6 Hz), 5.30 (1H, s), 6.78 (1H, br s), 6.97-7.02 (1H, m), 7.15-7.18 (2H, m), 7.27-7.30 (1H, m) .
Elemental analysis for: C₂₁H₁₆ClF₄N₃O₄S·0.75H₂O·0.8CHCl₃
Calculated: C, 42.65; H, 2.90; N, 6.60
Found: C, 42.37; H, 2.65; N, 6.52.

### Referential Example 832

tert-Butyl 4-chloro-2-{hydroxy[2-(trifluoromethoxy)phenyl]methyl}phenylcarbamate

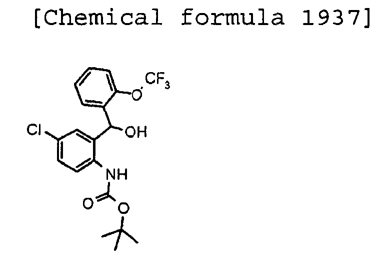

tert-Butyl 4-chlorophenylcarbamate (10.9 g) was dissolved in anhydrous tetrahydrofuran (200 mL). To the resulting solution was added dropwise sec-butyl lithium (a 1.00 M solution, 105 mL) at -78°C. After warming to -20°C, the reaction mixture was stirred for one hour. To the reaction mixture was added dropwise an anhydrous tetrahydrofuran solution (10 mL) of 2-(trifluoromethoxy)benzaldehyde (10.0 g) at -20°C. The reaction mixture was stirred for 20 minutes. A saturated aqueous solution of ammonium chloride was added to terminate the reaction. To the reaction mixture was added ethyl acetate and the layers separated. The organic layer was dried over anhydrous sodium sulfate. The solvent was distilled off under reduced pressure. The residue was purified by silica gel column chromatography (hexane:ethyl acetate= from 20:1 to 10:1) to give the title compound (14.5 g).
MS (EI) m/z: 417 M⁺.
¹H-NMR (CDCl3) δ: 1.48 (9H, s), 3.07-3.28 (1H, m), 6.15 (1H, s), 7.07 (1H, d, J = 2.4 Hz), 7.21-7.26 (3H, m), 7.31-7.40 (2H, m), 7.60-7.66 (2H, m).

### Referential Example 833

(2-Amino-5-chlorophenyl)[2-(trifluoromethoxy)phenyl]methanol

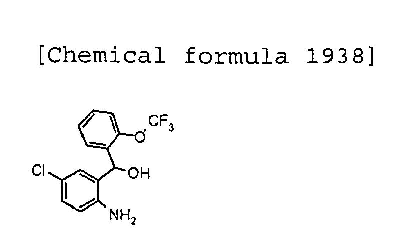

In a similar manner to that employed for the synthesis of 2-amino-5-chloro-3-[(2,3-dimethoxyphenyl)(hydroxy)methyl]pyridine, the title compound (11.3 g) was obtained from tert-butyl 4-chloro-2-{hydroxy[2-(trifluoromethoxy)phenyl]methyl}phenylcarbamate (14.5 g).
MS (EI) m/z: 317 M⁺.
¹H-NMR (CDCl3) δ: 6.12 (1H, s), 6.62 (1H, d, J = 8.3 Hz), 7.01 (1H, d, J = 2.4 Hz), 7.07 (1H, dd, J = 8.5, 2.4 Hz), 7.28-7.39 (3H, m), 7.53. (1H, dd, J = 7.6, 2.0 Hz).

### Referential Example 834

2-{7-Chloro-2-oxo-5-[2-(trifluoromethoxy)phenyl]-1,2,3,5-tetrahydro-4,1-benzothiazepin-3-yl}acetic acid

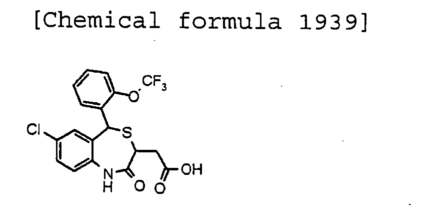

In a similar manner to that employed for the synthesis of 2-{trans-7-chloro-5-[3-methoxy-2-(trifluoromethoxy)phenyl]-2-oxo-1,2,3,5-tetrahydro-4,1-benzothiazepin-3-yl}acetic acid, the title compound (12.5 g) was obtained as a trans-cis mixture (trans:cis=6:1) from (2-amino-5-chlorophenyl)[2-(trifluoromethoxy)phenyl]methanol (11.2 g).
MS (EI) m/z: 431 M⁺.
¹H-NMR (CDCl3) δ: 2.41 (trans and cis of 1H, dd, J = 17.0, 4.0 Hz), 2.71-2.84 (trans and cis of 1H, m), 3.55-3.65 (1H, m), 5.66 (cis of 1H, s), 5.91 (trans of 1H, s), 6.63 (trans of 1H, d, J = 2.2 Hz), 6.94 (cis of 1H, d, J = 8.5 Hz), 7.14-7.24 (tans and cis of 1H, m), 7.36-7.52 (trans and cis of 2H, m), 7.58-7.64 (trans and cis of 2H, m), 7.71 (cis of 1H, d, J = 2.4 Hz), 7.88-7.95 (trans and cis of 1H, m), 9.46 (cis of 1H, s), 10.11 (trans of 1H, s), 12.43 (trans and cis of 1H, s).

### Referential Example 835

Ethyl 2-{trans-7-chloro-2-oxo-5-[2-(trifluoromethoxy)phenyl]-1,2,3,5-tetrahydro-4,1-benzothiazepin-3-yl}acetate

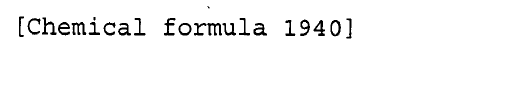

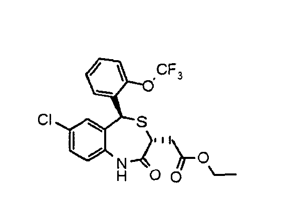

The trans-cis mixture (trans:cis=6:1, 12.5 g) of 2-{7-chloro-2-oxo-5-[2-(trifluoromethoxy)phenyl]-1,2,3,5-tetrahydro-4,1-benzothiazepin-3-yl}acetic acid was dissolved in ethanol (180 mL). To the resulting solution was added concentrated sulfuric acid (300 µl). The resulting mixture was heated under reflux for 5 hours. After the reaction mixture was cooled to room temperature, a saturated aqueous solution of sodium hydrogen carbonate and ethyl acetate were added thereto and the layers separated. After the organic layer was dried over anhydrous sodium sulfate, the solvent was distilled off under reduced pressure. The solid thus precipitated was subjected to slurry washing with a hexane-ethyl acetate solvent mixture (hexane:ethyl acetate=9:1) to give the title compound (9.00 g).
MS (FAB) m/z: 460 (M+H)⁺.
¹H-NMR (CDCl3) δ: 1.24 (3H, t, J = 7.1 Hz), 2.44 (1H, dd, J = 17.2, 3.8 Hz), 3.12 (1H, dd, J = 17.2, 10.4 Hz), 3.84 (1H, dd, J = 10.4, 3.8 Hz), 4.13 (2H, q, J = 7.1 Hz), 6.13 (1H, s), 6.75 (1H, d, J = 2.2 Hz), 7.12 (1H, d, J = 8.5 Hz), 7.26-7.33 (3H, m), 7.42-7.48 (2H, m), 7.84-7.89 (1H, m).

### Referential Example 836

Ethyl 2-{trans-7-chloro-2-thioxo-5-[2-(trifluoromethoxy)phenyl]-1,2,3,5-tetrahydro-4,1-benzothiazepin-3-yl}acetate

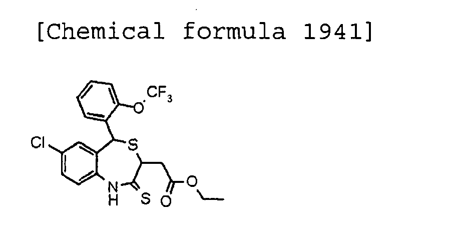

In a similar manner to that employed for the synthesis of methyl 2-[(trans)-7-chloro-5-(2,3-dimethoxyphenyl)-2-thioxo-1,2,3,5-tetrahydro-4,1-benzothiazepin-3-yl]acetate, the title compound (9.33 g) was obtained as a trans-cis mixture (trans:cis=3:1) from ethyl 2-{trans-7-chloro-2-oxo-5-[2-(trifluoromethoxy)phenyl]-1,2,3,5-tetrahydro-4,1-benzothiazepin-3-yl}acetate (9.00 g).
MS (EI) m/z: 475 M⁺.
¹H-NMR (CDCl3) δ: 1.21-1.27 (trans and cis of 3H, m), 2.58-2.65 (trans and cis of 1H, m), 3.34-3.46 (trans and cis of 1H, m), 4.05-4.17 (trans and cis of 3H, m), 5.41 (cis of 1H, s), 5.95 (trans of 1H, s), 6.74 (trans of 1H, d, J = 2.2 Hz), 6.98 (cis of H, d, J = 8.5 Hz), 7.10-7.16 (trans and cis of 1H, m), 7.29-7.36 (trans and cis of 2H, m), 7.41-7.50 (trans and cis of 1H, trans of 1H, m), 7.56 (cis of 1H, d, J = 2.4 Hz), 7.81 (trans of 1H, dd, J = 7.1, 2.4 Hz), 8.36 (cis of 1H, dd, J = 7.6, 2.0 Hz), 8.54 (cis of 1H, s), 9.21 (trans of 1H, s).

### Example 923

Ethyl 2-[trans-8-chloro-6-[2-(trifluoromethoxy)phenyl]-1-(trifluoromethyl)-4H,6H-[1,2,4]triazolo[4,3-a][4,1]benzothiazepin-4-yl]acetate

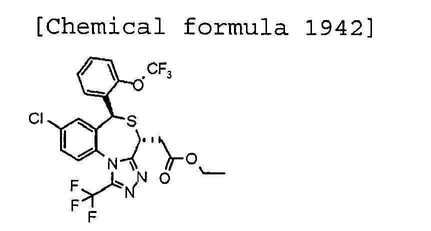

The trans-cis mixture (trans:cis=3:1, 2.00 g) of ethyl 2-{trans-7-chloro-2-thioxo-5-[2-(trifluoromethoxy)phenyl]-1,2,3,5-tetrahydro-4,1-benzothiazepin-3-yl}acetate was dissolved in tetrahydrofuran (200 mL). To the resulting solution was added hydrazine hydrate (307 µl) at room temperature. The resulting mixture was stirred at room temperature for one hour. The reaction mixture was concentrated under reduced pressure. The residue was dissolved in dichloroethane (60 mL). Under ice cooling, trifluoroacetic anhydride (3.56 mL) and trifluoroacetic acid (6.47 mL) were added. The resulting mixture was stirred at room temperature for 30 minutes and then, at 55°C for one hour. To the reaction mixture was added toluene (60 mL) and the resulting mixture was heated under reflux for 2 hours. After the reaction mixture was cooled to room temperature, a saturated aqueous solution of sodium hydrogen carbonate was added thereto and the layers separated. The organic layer was dried over anhydrous sodium sulfate. The solvent was distilled off under reduced pressure. The residue was purified by silica gel column chromatography (chloroform:ethyl acetate=100:1) to give a colorless foam. The resulting foam was dissolved in ethanol and the resulting solution was left to stand at room temperature for one hour. The crystals thus precipitated were filtered off and the filtrate was concentrated under reduced pressure. The residue was dissolved in ethanol and the resulting solution was left to stand at room temperature for one hour. The above-described operation was repeated. The solid thus precipitated was collected by filtration to give the title compound (377 mg).
MS (FAB) m/z: 552 (M+H)⁺.
¹H-NMR (CDCl3) δ: 1.26 (3H, t, J = 7.1 Hz), 2.92 (1H, dd, J = 17.3, 5.6 Hz), 3.63 (1H, dd, J = 17.3, 8.8 Hz), 4.16 (2H, q, J = 7.1 Hz), 4.35 (1H, dd, J = 8.8, 5.6 Hz), 5.30 (1H, s), 6.93 (1H, d, J = 2.2 Hz), 7.28-7.32 (1H, m), 7.41 (1H, t, J = 8.0 Hz), 7.44-7.51 (3H, m), 7.81 (1H, dd, J = 8.0, 2.2 Hz).

### Example 924

2-[trans-8-Chloro-6-[2-(trifluoromethoxy)phenyl]-1-(trifluoromethyl)-4H,6H-[1,2,4]triazolo[4,3-a] [4,1]benzothiazepin-4-yl]acetic acid

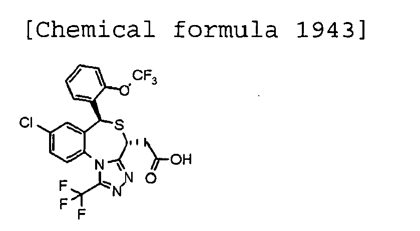

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (72 mg) was obtained from ethyl 2-[trans-8-chloro-6-[2-(trifluoromethoxy)phenyl]-1-(trifluoromethyl)-4H,6H-[1,2,4]triazolo[4,3-a][4,1]benzothiazepin-4-yl]acetate (75 mg).
MS (EI) m/z: 523 M⁺.
¹H-NMR (CDCl3) δ: 2.99 (1H, dd, J = 17.5, 5.2 Hz), 3.67 (1H, dd, J = 17.5, 9.3 Hz), 4.31 (1H, dd, J = 9.3, 5.2 Hz), 5.31 (1H, s), 6.94 (1H, d, J = 2.2 Hz), 7.29-7.33 (1H, m), 7.40 (1H, d, J = 8.3 Hz), 7.45-7.52 (3H, m), 7.81 (1H, dd, J = 7.1, 2.4 Hz).
Elemental analysis for: C₂₀H₁₂ClF₆N₃O₃S
Calculated: C, 45.86; H, 2.31; N, 8.02
Found: C, 45.69; H, 2.19; N, 8.02.

### Referential Example 837

tert-Butyl 4-chloro-2-[(2-fluoro-3-methoxyphenyl)(hydroxy)methyl]phenylcarbamate

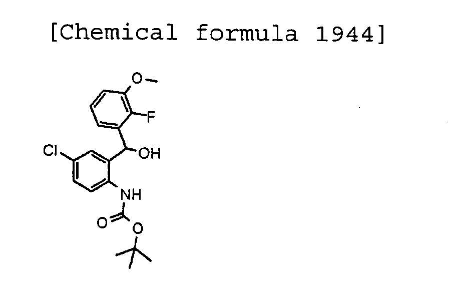

In a similar manner to that employed for the synthesis of tert-butyl 4-chloro-2-{hydroxy[2-(trifluoromethoxy)phenyl]methyl}phenylcarbamate, the title compound (17.9 g) was obtained from tert-butyl 4-chlorophenylcarbamate (10.9 g) and 2-fluoro-3-methoxybenzaldehyde (10.0 g).
MS (EI) m/z: 381 M⁺.
¹H-NMR (CDCl3) δ: 1.49 (9H, s), 2.93-2.99 (1H, m), 3.89 (3H, s), 6.14 (1H, d, J = 4.2 Hz), 6.95 (1H, td, J = 8.1, 1.5 Hz), 7.00-7.05 (2H, m), 7.12 (1H, td, J = 8.1, 1.5 Hz), 7.24 (1H, dd, J = 8.8, 2.4 Hz), 7.59 (1H, br s), 7.72-7.78 (1H, m).

### Referential Example 838

(2-Amino-5-chlorophenyl)(2-fluoro-3-methoxyphenyl)methanol

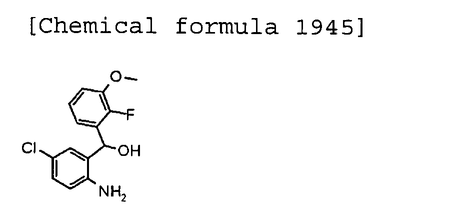

In a similar manner to that employed for the synthesis of 2-amino-5-chloro-3-[(2,3-dimethoxyphenyl)(hydroxy)methyl]pyridine, the title compound (13.4 g) was obtained from tert-butyl 4-chloro-2-[(2-fluoro-3-methoxyphenyl)(hydroxy)methyl]phenylcarbamate(17.9 g).
MS (EI) m/z: 281 M⁺.
¹H-NMR (CDCl3) δ: 3.89 (3H, s), 6.10 (1H, s), 6.60 (1H, d, J = 8.5 Hz), 6.92-7.13 (5H, m).

### Referential Example 839

2-[trans-7-Chloro-5-(2-fluoro-3-methoxyphenyl)-2-oxo-1,2,3,5-tetrahydro-4,l-benzothiazepin-3-yl]acetic acid

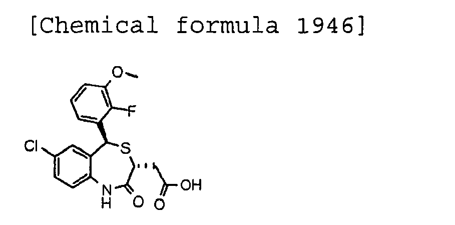

In a similar manner to that employed for the synthesis of 2-{trans-7-chloro-5-[3-methoxy-2-(trifluoromethoxy)phenyl]-2-oxo-1,2,3,5-tetrahydro-4,1-benzothiazepin-3-yl}acetic acid, the title compound (6.58 g) was obtained from (2-amino-5-chlorophenyl)(2-fluoro-3-methoxyphenyl)methanol (6.00 g).
MS (FAB) m/z: 396 (M+H)⁺.
¹H-NMR (CDCl3) δ: 2.41 (1H, dd, J = 17.0, 4.0 Hz), 2.76 (1H, dd, J = 17.0, 10.4 Hz), 3.31 (1H, s), 3.61 (1H, dd, J = 10.4, 4.0 Hz), 3.86 (3H, s), 5.88 (1H, s), 6.68 (1H, d, J = 2.4 Hz), 7.18 (1H, d, J = 8.5 Hz), 7.25-7.36 (3H, m), 7.45 (1H, dd, J = 8.5, 2.4 Hz), 10.04 (1H, s), 12.43 (1H, s).

### Referential Example 840

Ethyl 2-[trans-7-chloro-5-(2-fluoro-3-methoxyphenyl)-2-oxo-1,2,3,5-tetrahydro-4,1-benzothiazepin-3-yl]acetate

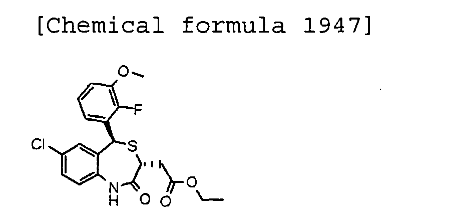

In a similar manner to that employed for the synthesis of ethyl 2-{trans-7-chloro-2-oxo-5-[2-(trifluoromethoxy)phenyl]-1,2,3,5-tetrahydro-4,1-benzothiazepin-3-yl}acetate, the title compound (5.75 g) was obtained from 2-[trans-7-chloro-5-(2-fluoro-3-methoxyphenyl)-2-oxo-1,2,3,5-tetrahydro-4,1-benzothiazepin-3-yl]acetic acid (6.58 g).
MS (EI) m/z: 423 M⁺.
¹H-NMR (CDCl3) δ: 1.24 (3H, t, J = 7.2 Hz), 2.43 (1H, dd, J = 17.1, 3.9 Hz), 3.11 (1H, dd, J = 17.1, 10.3 Hz), 3.84 (1H, dd, J = 10.3, 3.9 Hz), 3.90 (3H, s), 4.12 (2H, q, J = 7.2 Hz), 6.10 (1H, s), 6.83 (1H, d, J = 2.4 Hz), 7.02 (1H, td, J = 8.3, 1.3 Hz), 7.10 (1H, d, J = 8.3 Hz), 7.21 (1H, td, J = 8.3, 1.3 Hz), 7.26-7.35 (3H, m).

### Referential Example 841

Ethyl 2-[trans-7-chloro-5-(2-fluoro-3-methoxyphenyl)-2-thioxo-1,2,3,5-tetrahydro-4,1-benzothiazepin-3-yl]acetate

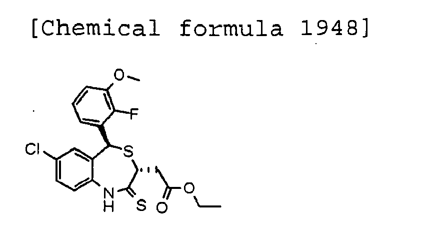

In a similar manner to that employed for the synthesis of methyl 2-[(trans)-7-chloro-5-(2,3-dimethoxyphenyl)-2-thioxo-1,2,3,5-tetrahydro-4,1-benzothiazepin-3-yl]acetate, the title compound (1.10 g) was obtained from ethyl 2-[trans-7-chloro-5-(2-fluoro-3-methoxyphenyl)-2-oxo-1,2,3,5-tetrahydro-4,1-benzothiazepin-3-yl]acetate (1.00 g).
MS (EI) m/z: 439 M⁺.
¹H-NMR (CDCl3) δ: 1.24 (3H, t, J = 7.1 Hz), 2.60 (1H, dd, J = 17.1, 4.1 Hz), 3.42 (1H, dd, J = 17.1, 10.0 Hz), 3.90 (3H, s), 4.05-4.16 (3H, m), 5.93 (1H, s), 6.81 (1H, d, J = 2.2 Hz), 7.03 (1H, td, J = 8.0, 1.6 Hz), 7.12 (1H, d, J = 8.5 Hz), 7.20 (1H, t, J = 8.0 Hz), 7.23-7.28 (1H, m), 7.31 (3H, dd, J = 8.3, 2.2 Hz), 9.23 (1H, br s).

### Example 925

Ethyl 2-[trans-8-chloro-6-(2-fluoro-3-methoxyphenyl)-1-(trifluoromethyl)-4H,6H-[1,2,4]triazolo[4,3-a][4,1]benzothiazepin-4-yl]acetate

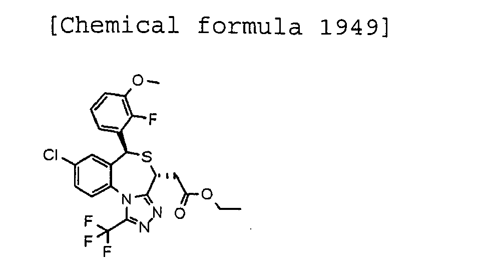

Ethyl 2-[trans-7-chloro-5-(2-fluoro-3-methoxyphenyl)-2-thioxo-1,2,3,5-tetrahydro-4,1-benzothiazepin-3-yl]acetate (1.09 g) was dissolved in tetrahydrofuran (25 mL). To the resulting solution was added hydrazine hydrate (182 µl) at room temperature. The resulting mixture was stirred at room temperature for one hour. The reaction mixture was concentrated under reduced pressure. The residue was dissolved in dichloroethane (25 mL). To the resulting solution were added trifluoroacetic anhydride (3.14 mL) and trifluoroacetic acid (3.84 mL) under ice cooling and the resulting mixture was stirred at room temperature for 30 minutes and then at 55°C for one hour. To the reaction mixture was added toluene (60 mL) and the mixture was heated under reflux for 2 hours. After the reaction mixture was cooled to room temperature, a saturated aqueous solution of sodium hydrogen carbonate was added thereto and the layers separated. The organic layer was dried over anhydrous sodium sulfate. The solvent was distilled off under reduced pressure. The residue was purified by silica gel column chromatography (chloroform:ethyl acetate=100:1). The crudely purified product was dissolved in ethanol and the resulting mixture was left to stand at room temperature for one hour. The solid thus precipitated was collected by filtration to give the title compound (464 mg).
MS (EI) m/z: 515 M⁺.
¹H-NMR (CDCl3) δ: 1.26 (3H, t, J = 7.2 Hz), 2.91 (1H, dd, J = 17.3, 5.4 Hz), 3.63 (1H, dd, J = 17.3, 9.0 Hz), 3.87 (3H, s), 4.15 (2H, q, J = 7.2 Hz), 4.33 (1H, dd, J = 9.0, 5.4 Hz), 5.24 (1H, s), 6.98 (1H, d, J = 2.2 Hz), 7.01-7.06 (1H, m), 7.19-7.23 (2H, m), 7.39 (1H, d, J = 8.5 Hz), 7.46 (1H, dd, J = 8.5, 2.2 Hz).

### Example 926

Ethyl 2-[trans-8-chloro-6-(2-fluoro-3-methoxyphenyl)-i-(trifluoromethyl)-4H,6H-[1,2,4]triazolo[4,3-a] [4,1]benzothiazepin-4-yl]acetic acid

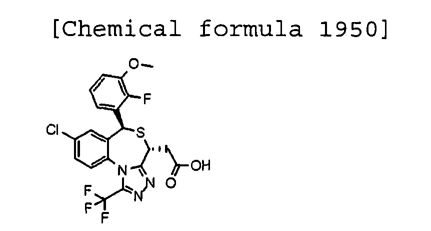

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (95 mg) was obtained from ethyl 2-[trans-8-chloro-6-(2-fluoro-3-methoxyphenyl)-1-(trifluoromethyl)-4H,6H-[1,2,4]triazolo[4,3-a][4,1]benzothiazepin-4-yl]acetate (100 mg).
MS (FAB) m/z: 488 (M+H)⁺.
¹H-NMR (CDCl3) δ: 2.97 (1H, dd, J = 17.6, 4.9 Hz), 3.66 (1H, dd, J = 17.6, 9.5 Hz), 3.88 (3H, s), 4.29 (1H, dd, J = 9.5, 4.9 Hz), 5.24 (1H, s), 6.99 (1H, d, J = 2.2 Hz), 7.01-7.07 (1H, m), 7.19-7.23 (2H, m), 7.39 (1H, d, J = 8.5 Hz), 7.47 (1H, dd, J = 8.5, 2.2 Hz).
Elemental analysis for: C₂₀H₁₄ClF₄N₃O₃S·1.25H₂O·0.2CHCl₃
Calculated: C, 45.66; H, 3.14; N, 7.83
Found: C, 45.94; H, 2.97; N, 7.57.

### Referential Example 842

tert-Butyl {4-chloro-2-[(2,6-dimethoxyphenyl)(hydroxy)methyl]phenyl}carbamate.

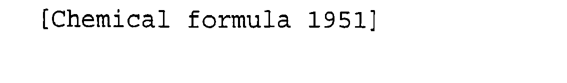

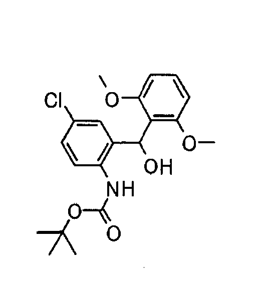

tert-Butyl (4-chlorophenyl)carbamate (1.48 g) was dissolved in tetrahydrofuran (15 mL). After the resulting solution was cooled to -30°C in an argon atmosphere, sec-butyl lithium (15.9 mL) was added dropwise thereto. The resulting mixture was stirred at -30°C for one hour, followed by the addition of a tetrahydrofuran solution (20 mL) of 2,6-dimethoxybenzaldehyde (1.20 g). The reaction mixture was gradually warmed to room temperature and stirred at room temperature for 2 hours. To the reaction mixture was added a saturated aqueous solution of ammonium chloride. Ethyl acetate was then added to the resulting mixture and the layers separated. The organic layer was washed with saturated brine and dried over anhydrous sodium sulfate. The solvent was distilled off under reduced pressure. The residue was purified by flash silica gel column chromatography (hexane:ethyl acetate=5:1) to give the title compound (2.23 g).
1H-NMR (400 MHz, DMSO-d6) δ 1.54 (9H, s), 3.82 (6H, s), 4.53 (1H, d, J = 10.8 Hz), 6.30 (1H, d, J = 11.5 Hz), 6.66 (2H, d, J = 8.3 Hz), 6.74 (1H, d, J = 2.5 Hz), 7.19 (1H, dd, J = 8.8, 2.5 Hz), 7.32 (1H, t, J = 8.3 Hz), 8.00 (1H, d, J = 8.8 Hz), 8.43 (1H, s).
MS (EI) m/z: 416 (M + Na+).

### Referential Example 843

[trans-7-Chloro-5-(2,6-dimethoxyphenyl)-2-oxo-1,2,3,5-tetrahydro-4,1-benzothiazepin-3-yl]acetic acid

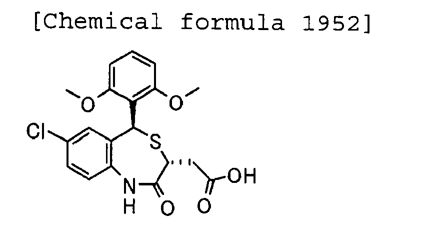

tert-Butyl {4-chloro-2-[(2,6-dimethoxyphenyl)(hydroxy)methyl]phenyl}carbamate (3.84 g) was dissolved in dichloromethane (50 mL). To the resulting solution was added p-toluenesulfonic acid monohydrate (342 mg). The resulting mixture was stirred at room temperature for 2 hours. To the reaction mixture was added p-toluenesulfonic acid monohydrate (342 mg)again, followed by stirring at room temperature for 2.5 hours. The solid thus precipitated was collected by filtration and washed with diethyl ether to give a pale yellow solid (2.47 g). The filtrate was purified by flash silica gel column chromatography (hexane:ethyl acetate=1:1) to give a thiomalic acid adduct (129 mg).
The adduct and the solid obtained by the above-described operation were combined and suspended in 1,2-dichloroethane (80 mL). To the resulting suspension were added DL-mercaptosuccinic acid (1.38 g) and p-toluenesulfonic acid monohydrate (1.84 g). The resulting mixture was stirred at 70°C for 20 hours. The solid was colleted by filtration and washed with dichloromethane to give a white solid (4.47 g). To the filtrate was added water and the layers separated. The organic layer was dried over anhydrous sodium sulfate. The solvent was distilled off under reduced pressure.
The residue thus obtained and white solid (4.47 g) were combined. To the resulting mixture was added toluene (250 mL). Using a Dean-Stark water separator, the resulting mixture was heated under reflux for 15 hours. The solvent was distilled off under reduced pressure. To the residue was added diethyl ether and the solid was collected by filtration. The solid thus obtained was dissolved in a solvent mixture of methanol (150 mL) and water (15 mL). To the resulting solution was added potassium carbonate (1.05 g) and the resulting mixture was stirred at 50°C for 2.5 hours. The solvent was distilled off under reduced pressure and to the residue were added water and ethyl acetate. After the water layer was made acidic with 1N hydrochloric acid, the layers were separated. The organic layer was dried over anhydrous sodium sulfate. The solvent was distilled off under reduced pressure. To the residue was added diethyl ether and the solid was collected by filtration to give the title compound (1.98 g).
1H-NMR (DMSO-d6) δ: 2.31 (1H, dd, J = 16.7, 4.1 Hz), 2.70 (1H, dd, J = 16.7, 10.3 Hz), 3.66 (1H, dd, J = 10.4, 3.8 Hz), 3.80 (6H, br s), 6.30 (1H, s), 6.69-6.87 (2H, m), 6.96 (1H, d, J = 2.2 Hz), 7.09 (1H, d, J = 8.3 Hz), 7.36-7.41 (2H, m), 9.89 (1H, s), 12.37 (1H, br s).

### Referential Example 844

Allyl [trans-7-chloro-5-(2,6-dimethoxyphenyl)-2-oxo-1,2,3,5-tetrahydro-4,1-benzothiazepin-3-yl]acetate

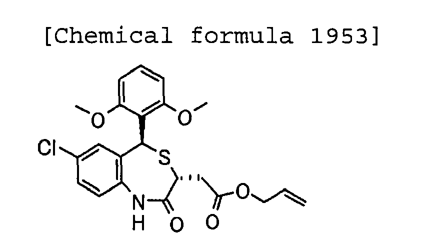

[trans-7-Chloro-5-(2,6-dimethoxyphenyl)-2-oxo-1,2,3,5-tetrahydro-4,1-benzothiazepin-3-yl]acetic acid (1.97 g) was dissolved in N,N-dimethylformamide (20 mL). To the resulting solution were added allyl bromide (404 µL) and potassium carbonate (639 mg). The resulting mixture was stirred at room temperature for 3 days. To the reaction mixture were added water and ethyl acetate and the layers separated. The organic layer was washed with water and saturated brine and dried over anhydrous sodium sulfate. The solvent was distilled off under reduced pressure. The residue was purified by flash silica gel column chromatography (hexane:ethyl acetate=2:1 → 1:1) to give the title compound (753 mg).
1H-NMR (400 MHz, CDCl3) δ2.46-2.52 (1H, m), 2.78 (1H, dd, J = 16.9, 10.3 Hz), 3.32 (6H, s), 3.72 (1H, dd, J = 10.3, 3.9 Hz), 4.51 (2H, dt, J = 5.2, 1.5 Hz), 5.17-5.30 (2H, m), 5.82-5.91 (1H, m), 6.31 (1H, s), 6.80 (2H, br s), 6.97 (1H, d, J = 2.5 Hz), 7.08 (1H, d, J = 8.6 Hz), 7.37-7.41 (2H, m), 9. 93 (1H, s).
MS(FAB) m/z: 448(M + H)+.

### Referential Example 845

Allyl [trans-7-chloro-5-(2,6-dimethoxyphenyl)-2-thioxo-1,2,3,5-tetrahydro-4,1-benzothiazepin-3-yl]acetate

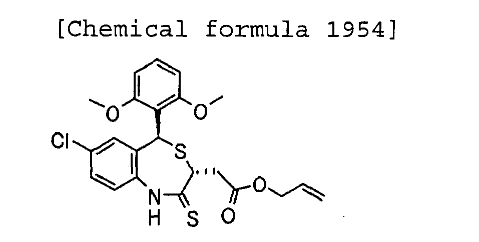

Allyl [trans-7-chloro-5-(2,6-dimethoxyphenyl)-2-oxo-1,2,3,5-tetrahydro-4,1-benzothiazepin-3-yl]acetate (715 mg) was dissolved in toluene (20 mL). To the resulting solution was added Lawesson's reagent (712 mg) and the resulting mixture was heated under reflux for 20 minutes. The reaction mixture was allowed to cool and then distilled under reduced pressure to remove the solvent. The residue was purified by flash silica gel column chromatography (hexane:ethyl acetate=2:1) to give the title compound (756 mg) .
1H-NMR (CDCl3) δ: 2.61 (1H, dd, J = 17.2, 3.7 Hz), 3.43 (1H, dd, J = 17.0, 10.4 Hz), 3.82 (6H, br s), 4.23 (1H, dd, J = 10.3, 3.7 Hz), 4.57 (2H, d, J = 5.4 Hz), 5.20-5.32 (2H, m), 5.83-5.93 (1H, m), 6.35 (1H, s), 6.62-6.63 (2H, m), 7.07 (1H, d, J = 8.5 Hz), 7.10 (1H, d, J = 2.4 Hz), 7.26-7.33 (2H, m), 9.21 (1H, s).

### Referential Example 846

Allyl [trans-7-chloro-5-(2,6-dimethoxyphenyl)-2-hydrazono-1,2,3,5-tetrahydro-4,1-benzothiazepin-3-yl]acetate

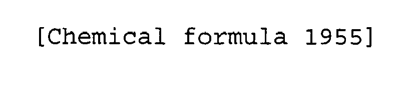

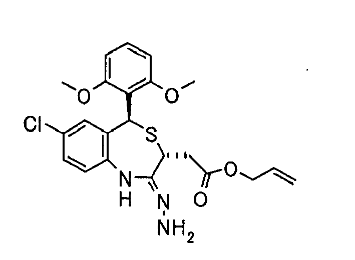

Allyl [trans-7-chloro-5-(2,6-dimethoxyphenyl)-2-thioxo-1,2,3,5-tetrahydro-4,1-benzothiazepin-3-yl]acetate (731 mg, containing about 0.4 molecule of ethyl acetate) was dissolved in tetrahydrofuran (20 mL). To the resulting solution was added hydrazine monohydrate (71 µL) at 0°C. The resulting mixture was stirred at 0°C for 20 minutes. To the reaction mixture was added hydrazine monohydrate (71 µL) again. After warming to room temperature, the resulting mixture was stirred for 40 minutes. To the reaction mixture were added water and ethyl acetate and the layers separated. The water layer was extracted with ethyl acetate. The organic layer was dried over anhydrous sodium sulfate. The solvent was distilled off under reduced pressure. The residue was purified by flash silica gel column chromatography (hexane:ethyl acetate=1:2) to give the title compound (353 mg).
1H-NMR (400 MHz, CDCl3) δ: 2.33 (0.3H, dd, J = 18.1, 1.5 Hz), 2.46 (0.7H, dd, J = 16.7, 4.4 Hz), 2.89 (0.3H, dd, J = 18.3, 8.2 Hz), 3.15 (0.7H, dd, J = 16.4, 10.3 Hz), 3.75 (1H, br s), 3.82 (6H, s), 3.92 (1H, br s), 4.03-4.15 (1H, m), 4.39 (1H, br s), 4.51-4.60 (2H, m), 5.18-5.31 (1.3H, m), 5.82-5.92 (0.7H, m), 6.30 (0.3H, s), 6.41 (0.7H, s), 6.52-6.77 (2H, br m), 6.62 (1H, d, J = 8.6 Hz), 6.87 (0.7H, d, J = 8.3 Hz), 6.94 (0.3H, d, J = 8.6 Hz), 7.03 (0.3H, d, J = 2.5 Hz), 7.07 (0.7H, d, J = 2.5 Hz), 7.15 (0.7H, dd, J = 8.3, 2.5 Hz), 7.19 (0.3H, dd, J = 8.6, 2.5 Hz), 7.26-7.33 (1H, m).
MS(ESI) m/z:462(M + H)+.

### Example 927

Allyl [trans-8-chloro-6-(2,6-dimethoxyphenyl)-1-(trifluoromethyl)-4H,6H-[1,2,4]triazolo[4,3-a][4,1]benzothiazepin-4-yl]acetate

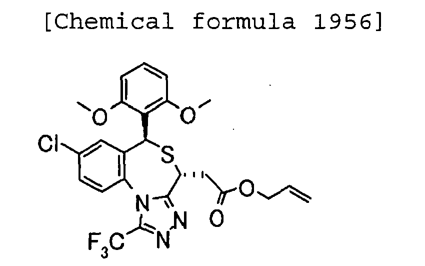

A mixture (353 mg) of allyl [trans-7-chloro-5-(2,6-dimethoxyphenyl)-2-hydrazono-1,2,3,5-tetrahydro-4,1-benzothiazepin-3-yl]acetate and allyl [trans-7-chloro-5-(2,6-dimethoxyphenyl)-2-hydrazono-1,2,3,5-tetrahydro-4,1-benzothiazepin-3-yl]acetate was dissolved in dichloromethane (10 mL). To the resulting solution were added triethylamine (117 µL) and trifluoroacetic anhydride (117 µL) at 0°C and the resulting mixture was stirred for 30 minutes. Triethylamine (117 µL) and trifluoroacetic anhydride (117 µL) were added again and the resulting mixture was stirred at room temperature for 30 minutes. Triethylamine (117 µL) and trifluoroacetic anhydride (117 µL) were added once again and the resulting mixture was stirred at room temperature for 30 minutes. To the reaction mixture was added water and the layers separated. The organic layer was dried over anhydrous sodium sulfate. The solvent was distilled off under reduced pressure. The yellow oil thus obtained was dissolved in toluene (50 mL). The resulting solution was stirred at 80°C for one hour. After returning to room temperature, p-toluenesulfonic acid monohydrate (10 mg) was added and the resulting mixture was stirred at 50°C for 14 hours. The solvent was distilled off under reduced pressure. The residue was purified by flash silica gel column chromatography (hexane:ethyl acetate=2:1 → 1:1) to give the title compound (191 mg). 1H-NMR (400 MHz, CDCl3) δ: 3.05 (1H, dd, J = 17.0, 5.8 Hz), 3.39 (1H, dd, J = 17.1, 9.1 Hz), 3.56 (3H, br s), 3.87 (3H, br s), 4.30 (1H, dd, J = 9.0, 5.8 Hz), 4.54-4.57 (2H, m), 5.18-5.32 (2H, m), 5.33 (1H, s), 5.83-5.92 (1H, m), 6.68 (1H, br s), 6.85 (1H, br s), 7.17 (1H, d, J = 2.2 Hz), 7.38 (1H, t, J = 8.4 Hz), 7.70 (1H, dd, J = 8.3, 2.5 Hz), 7.76 (1H, d, J = 8.8 Hz).
MS(ESI) m/z:540 (M + H)+.

### Example 928

[trans-8-Chloro-6-(2,6-dimethoxyphenyl)-1-(trifluoromethyl)-4H,6H-[1,2,4]triazolo[4,3-a][4,1]benzothiazepin-4-yl]acetic acid

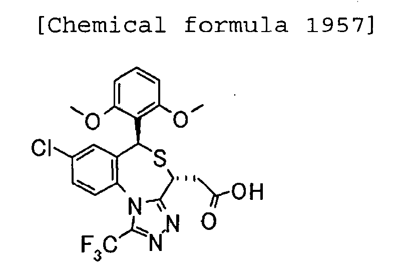

Allyl [trans-8-chloro-6-(2,6-dimethoxyphenyl)-1-(trifluoromethyl)-4H,6H-[1,2,4]triazolo[4,3-a][4,1]benzothiazepin-4-yl]acetate (159 mg) was dissolved in dichloromethane (10 mL). To the resulting solution were added pyrrolidine (26 µL), triphenylphosphine (15 mg) and tetrakis(triphenylphosphine)palladium (34 mg) at 0°C. The resulting mixture was stirred for 30 minutes while maintaining at 0°C. Tetrakis(triphenylphosphine)palladium (34 mg) was added again and the resulting mixture was stirred at room temperature for 30 minutes. Tetrakis(triphenylphosphine)palladium (34 mg) was added once again and the resulting mixture was stirred at room temperature for 30 minutes. To the reaction mixture was added water and the layers separated. The organic layer was dried over anhydrous sodium sulfate. The solvent was distilled off under reduced pressure. The residue was purified by preparative TLC (thickness: 2 mm, dichloromethane:methanol=9:1), followed by further purification by preparative TLC (thickness: 2 mm, chloroform:methanol:water=7:3:1 lower layer) to give the title compound (60 mg).
1H-NMR (400 MHz, CDCl3) δ: 2.78 (1H, dd, J = 17.0, 5.5 Hz), 3.20-3.27 (1H, m), 3.56 (3H, br s), 3.87 (3H, br s), 4.23 (1H, dd, J = 8.7, 5.5 Hz), 5.31 (1H, s), 6.67 (1H, br s), 6.84 (1H, br s), 7.17 (1H, d, J = 2.2 Hz), 7.37 (1H, t, J = 8.3 Hz), 7.68 (1H, dd, J = 8.6, 2.2 Hz), 7.75 (1H, dd, J = 8.6, 0.98 Hz).
MS(FAB) m/z: 500 (M + H)+.
Elemental analysis for: C₂₁H₁₇ClF₃N₃O₄S·0.5H₂O
Calculated: C : 49.56, H : 3.57, Cl: 6.97, F: 11.20, N : 8.26, S: 6.30.
Found: C : 49.52, H : 3.48, Cl: 6.73, F: 11.04, N : 8.08, S: 6.41.

### Referential Example 847

tert-Butyl {4-chloro-2-[(2,3-dichlorophenyl)(hydroxy)methyl]phenyl}carbamate

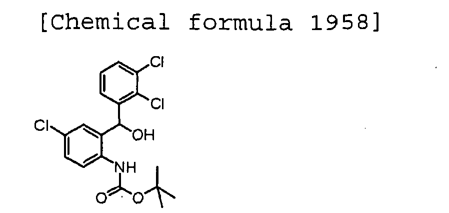

tert-Butyl 4-chlorophenylcarbamate (1.00 g) was dissolved in tetrahydrofuran (10 mL). To the resulting solution was added sec-butyl lithium (1.0M, hexane solution) (10.0 mL) at -50°C in a nitrogen atmosphere. The resulting mixture was stirred at -20°C for 0.5 hour. To the reaction mixture were slowly added dropwise a tetrahydrofuran solution (10 mL) of 2,3-dichlorobenzaldehyde (0.81 g). After stirring for further 2.5 hours at -20°C, a saturated aqueous solution of ammonium chloride was added to the reaction mixture. The resulting mixture was extracted with ethyl acetate. The extract was washed with saturated brine. The organic layer was dried over anhydrous sodium sulfate. After concentration of the filtrate, the residue thus obtained was purified by silica gel column (hexane:ethyl acetate= from 8:1, 4:1 to 2:1) to give the title compound (0.91 g). 1H-NMR (CDCl3) δ: 1.51 (9H, s), 3.59 (1H, br s), 6.16-6.16 (1H, m), 6.90-6.91 (1H, m), 7.16 (1H, br s), 7.24-7.27 (1H, m), 7.33-7.29 (1H, m), 7.47 (1H, d, J = 7.81 Hz), 7.56-7.59 (2H, m).

### Referential Example 848

(2-Amino-5-chlorophenyl)(2,3-dichlorophenyl)methanol

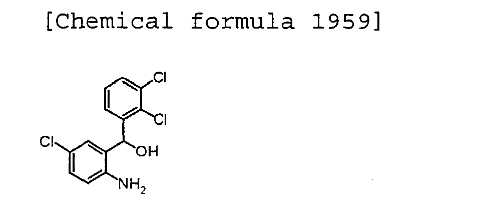

tert-Butyl {4-chloro-2-[(2,3-dichlorophenyl)(hydroxy)methyl]phenyl}carbamate (0.90 g) was dissolved in dichloromethane (5.0 mL). To the resulting solution was added a 4N hydrochloric acid-dioxane solution (10 mL) and the resulting mixture was stirred at room temperature for 3 days. After the reaction mixture was distilled under reduced pressure to remove the solvent, the residue was neutralized with a 5 mol/l aqueous sodium hydroxide solution and extracted with dichloromethane. The organic layer was dried over anhydrous sodium sulfate. The filtrate was concentrated to give the title compound (0.68 g).
MS (ESI) m/z : 286 (M - OH)+.

### Referential Example 849

[7-Chloro-5-(2,3-dichlorophenyl)-2-oxo-1,2,3,5-tetrahydro-4,1-benzothiazepin-3-yl]acetic acid

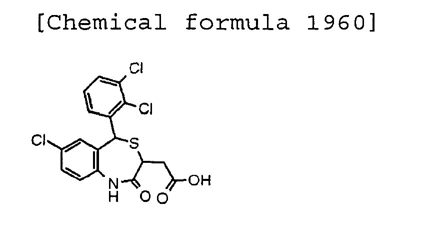

(2-Amino-5-chlorophenyl)(2,3-dichlorophenyl)methanol (0.83 g) and thiomalic acid (0.82 g) were dissolved in 1,4-dioxane (20 mL). To the resulting solution was added a 4N hydrochloric acid-dioxane solution (0.8 mL). The resulting mixture was stirred overnight at 70°C. A 4N hydrochloric acid-dioxane solution (30 mL) was added again and the resulting mixture was stirred overnight. After the reaction mixture was concentrated, the residue was dissolved in toluene (30 mL) and the resulting mixture was heated under reflux for 4 hours. The reaction mixture was cooled to room temperature and then, concentrated. The residue thus obtained was dissolved in ethanol-water (10:3, 13 mL). To the resulting solution was added potassium carbonate (0.55 g). The resulting mixture was stirred overnight at 70°C. The reaction mixture was made acidic with 1N hydrochloric acid. The solid thus precipitated was collected by filtration to give the title compound (1.00 g).
1H-NMR (CDCl3) δ: 2.41-2,47 (1H, m), 2.75-2.82 (1H, m), 3.59-3.63 (1H, m), 6.03 (1H, s), 6.51 (1H, d, J = 2.44 Hz), 7.20 (1H, d, J = 8.54 Hz), 7.46 (1H, dd, J = 8.54, 2.44 Hz), 7.59 (1H, t, J = 7.93 Hz), 7.77-7.82 (2H, m), 10.13 (1H, s).
MS (ESI) m/z : 416 (M + H) +.

### Referential Example 850

Ethyl [(trans)-7-chloro-5-(2,3-dichlorophenyl)-2-oxo-1,2,3,5-tetrahydro-4,1-benzothiazepin-3-yl]acetate.

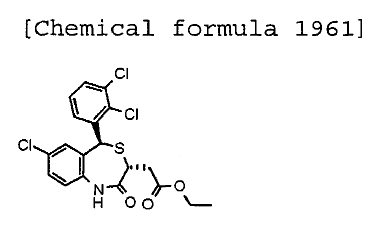

[7-Chloro-5-(2,3-dichlorophenyl)-2-oxo-1,2,3,5-tetrahydro-4,1-benzothiazepin-3-yl]acetic acid (0.56 g) was dissolved in ethanol (15 mL). To the resulting solution was added thionyl chloride and the resulting mixture was stirred at 50°C for 4 hours..The reaction mixture was cooled to room temperature and then concentrated. The residue was diluted with ethyl acetate and washed successively with water, a saturated aqueous solution of sodium bicarbonate and saturated brine. After the organic layer was dried over anhydrous sodium sulfate, the residue thus obtained was purified by silica gel column (hexane:ethyl acetate= from 4:1, 2:1 to 1:1) to give the title compound (0.18 g).
1H-NMR (CDCl3) δ: 1.25 (3H, t, J = 7.35 Hz), 2.45 (1H, dd, J = 17.04, 3.80 Hz), 3.13 (1H, dd, J = 17.16, 10.54 Hz), 3.82 (1H, dd, J = 10.05, 3.92 Hz), 4.13 (2H, q, J = 7.11 Hz), 6.22 (1H, s), 6.62 (1H, d, J = 2.45 Hz), 7.13 (1H, d, J = 8.58 Hz), 7.18 (1H, br s), 7.30 (1H, dd, J = 8.33, 2.45 Hz), 7.37 (1H, t, J = 7.97 Hz), 7.52-7.55 (1H, m), 7.73 (1H, d, J = 6.37 Hz).
MS (ESI) m/z : 446(M + H)+.

### Referential Example 851

Ethyl [(trans)-7-chloro-5-(2,3-dichlorophenyl)-2-thioxo-1,2,3,5-tetrahydro-4,1-benzothiazepin-3-yl]acetate

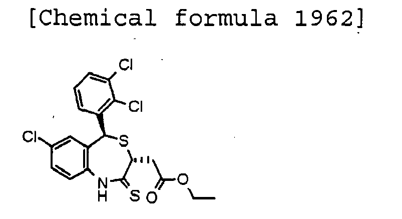

Ethyl [(trans)-7-chloro-5-(2,3-dichlorophenyl)-2-oxo-1,2,3,5-tetrahydro-4,1-benzothiazepin-3-yl]acetate (180 mg) was dissolved in toluene (10 mL). To the resulting solution was added Lawesson's reagent (118 mg) and the resulting mixture was stirred at 100°C for one hour. The reaction mixture was cooled to room temperature and then concentrated. The residue was dissolved in a chloroform-methanol (9:1) mixed solution. The resulting solution was filtered through a pad of layered celite/silica gel/celite and the filtrate was concentrated. The residue was purified by silica gel column (hexane:ethyl acetate= from 8:1, 4:1 to 2:1) to give the title compound (145 mg).
1H-NMR (CDCl3) δ: 2.62 (1H, dd, J = 16.8, 3.9 Hz), 3.44 (1H, dd, J = 16.8, 10.0 Hz), 3.61-3.61 (3H, m), 3.67 (3H, s), 3.87 (3H, s), 4.07 (1H, dd, J = 10.0, 3.9 Hz), 6.03 (1H, s), 6.85 (1H, d, J = 2.4 Hz), 6.96-6.99 (1H, m), 7.11 (1H, d, J = 8.3 Hz), 7.18 (1H, t, J = 7.9 Hz), 7.24-7.30 (2H, m).
MS (ESI) m/z : 460 (M + H) +.

### Example 929

Ethyl [(trans)-8-chloro-6-(2,3-dichlorophenyl)-1-(trifluoromethyl)-4H,6H-[1,2,4]triazolo[4,3-a][4,1]benzothiazepin-4-yl]acetate

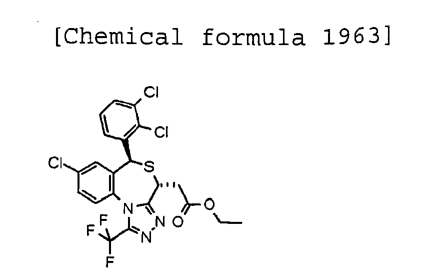

Ethyl [(trans)-7-chloro-5-(2,3-dichlorophenyl)-2-thioxo-1,2,3,5-tetrahydro-4,1-benzothiazepin-3-yl]acetate (145 mg) was dissolved in tetrahydrofuran (5.0 mL). To the resulting solution was added hydrazine monohydrate (0.30 mL). The resulting mixture was stirred at room temperature for one hour. The reaction mixture was diluted with water and dichloromethane, followed by extraction three times with dichloromethane. The organic layer was dried over anhydrous sodium sulfate. After concentration of the filtrate, the residue thus obtained was dissolved in 1,2-dichloroethane (5.0 mL). To the resulting solution was added trifluoroacetic anhydride (0.13 mL) at 0°C and the resulting mixture was stirred at 50°C for 2 hours. To the reaction mixture were added toluene (5.0 mL), trifluoroacetic anhydride (0.2 mL) and trifluoroacetic acid (0.20 mL). The resulting mixture was heated under reflux for 2 hours. After cooling to room temperature, the reaction mixture was neutralized with a saturated aqueous solution of sodium bicarbonate, diluted with ethyl acetate and washed successively with water and saturated brine. The organic layer was dried over anhydrous sodium sulfate. After the filtrate was concentrated, the residue thus obtained was purified by silica gel column (hexane:ethyl acetate= from 4:1, 2:1 to 1:2) to give the title compound (143 mg).
1H-NMR (CDCl3) δ: 1.26 (3H, t, J = 6.86 Hz), 2.94 (1H, dd, J = 17.16, 5.64 Hz), 3.64 (1H, dd, J = 17.40, 8.82 Hz), 4.16 (2H, q, J = 7.19 Hz), 4.33 (1H, dd, J = 8.82, 5.39 Hz), 5.33 (1H, s), 6.80 (1H, d, J = 2.21 Hz), 7.35-7.43 (2H, m), 7.49 (1H, dd, J = 8.46, 2.33 Hz), 7.55 (1H, dd, J = 8.09, 1.47 Hz), 7.64 (1H, dd, J = 7.72, 1.59 Hz).
MS (ESI) m/z : 538 (M + H) +.

### Example 930

[(trans)-8-Chloro-6-(2,3-dichlorophenyl)-1-(trifluoromethyl)-4H,6H-[1,2,4]triazolo[4,3-a][4,1]benzothiazepin-4-yl]acetic acid

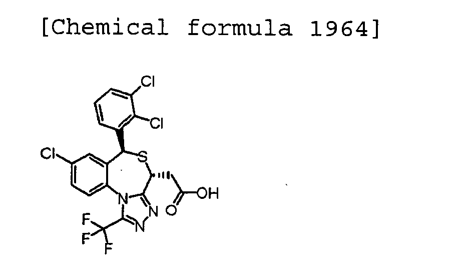

Ethyl [(trans)-8-chloro-6-(2,3-dichlorophenyl)-1-(trifluoromethyl)-4H,6H-[1,2,4]triazolo[4,3-a] [4, 1] benzothiazepin-4-yl] acetate (220 mg) was dissolved in 1,4-dioxane (5.0 mL). To the resulting solution was added concentrated hydrochloric acid (1.0 mL). The resulting mixture was stirred at 60°C for 3 days. After the reaction mixture was cooled to room temperature and a 10% aqueous citric acid solution was added thereto, the resulting mixture was extracted three times with dichloromethane. The organic layer thus obtained was washed with saturated brine and then, dried over anhydrous sodium sulfate. The filtrate was concentrated. The residue obtained by concentration was purified by silica gel column (chloroform:methanol= from 99:1, 9:1 to 7:1), followed by recrystallization from dichloromethane and hexane to give the title compound (95 mg).
1H-NMR (CD3OD) δ: 2.95 (1H, dd, J = 17.04, 5.27 Hz), 3.42-3.49 (2H, m), 4.34 (1H, dd, J = 8.95, 5.76 Hz), 5.38 (1H, s), 6.79 (1H, s), 7.50 (1H, t, J = 7.97 Hz), 7.62-7.67 (3H, m), 7.77 (1H, d, J = 7.84 Hz).
MS (ESI) m/z : 508 (M + H)+.
Elemental analysis for: C₁₉H₁₁Cl₃F₃N₃O₂S·0.75H₂O
Calculated: C, 43.70; H, 2.41; N, 8.05.
Found: C, 43.81; H, 2.16; N, 7.80.

### Referential Example 852

tert-Butyl {4-chloro-2-[2,3-(difluorophenyl)hydroxymethyl]phenyl}carbamate

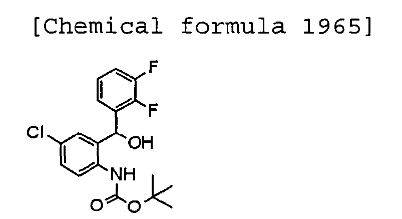

tert-Butyl-4-chlorophenylcarbamate (1.44 g) was dissolved in tetrahydrofuran (15 mL). The resulting solution was cooled at an outside temperature of -30°C. To the reaction mixture was added dropwise sec-butyl lithium (a 1M solution, 14 mL) over 20 minutes via a syringe.
After stirring at the same temperature for one hour, 2,3-difluorobenzaldehyde (1.00 g) was added dropwise in 5 minutes. While warming gradually to room temperature, the resulting mixture was stirred for 4.5 hours. To the reaction mixture was added a saturated aqueous solution of ammonium chloride and the resulting mixture was stirred for 30 minutes. The reaction mixture was transferred to a separatory funnel and extracted three times with ethyl acetate. The organic layer was washed with saturated brine, dried over anhydrous sodium sulfate, and concentrated under reduced pressure. The residue was purified by medium pressure column chromatography (product of Biotage, ethyl acetate:hexane=1:3) to give the title compound (1.75 g).
¹H-NMR (400 MHz, CDCl₃) δ: 1.49 (9H, s), 3.16 (1H, br s), 6.15 (1H, d, J = 2.9 Hz), 7.04 - 7.05 (1H, m), 7.12 - 7.19 (2H, m), 7.25 - 7.29 (2H, m), 7.39 (1H, s), 7.68 (1H, d, J = 8.8 Hz).
MS (ESI) m/z: 392 (M + Na)⁺.

### Referential Example 853

(2-Amino-5-chlorophenyl)-(2,3-difluorophenyl)methanol

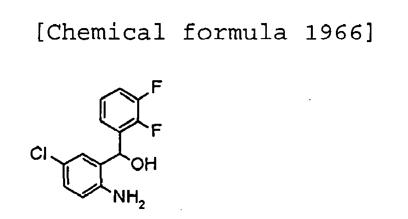

tert-Butyl {4-chloro-2-[2,3-(difluorophenyl)hydroxymethyl]phenyl}carbamate (1.75 g) was dissolved in dichloromethane (6 mL). To the resulting solution was added 4N hydrochloric acid-dioxane (3 mL). The resulting mixture was stirred overnight at room temperature. To the reaction mixture was added sodium hydrogen carbonate and the resulting mixture was extracted three times with ethyl acetate. The organic layer was washed with saturated brine, dried over anhydrous sodium sulfate and then, concentrated under reduced pressure to give a crude product of the title compound (1.28 g).
¹H-NMR (400 MHz, CDCl₃) δ: 3.70 (1H, s), 6.12 (1H, s), 6.67 (1H, d, J = 8.5 Hz), 6.98 (1H, d, J = 2.4 Hz), 7.06 - 7.25 (4H, m).
MS (ESI) m/z: 270 (M + H)⁺, 252 (M - OH)⁺.

### Referential Example 854

[7-Chloro-5-(2,3-difluorophenyl)-2-oxo-1,2,3,5-tetrahydro-4,1-benzothiazepin-3-yl]acetic acid

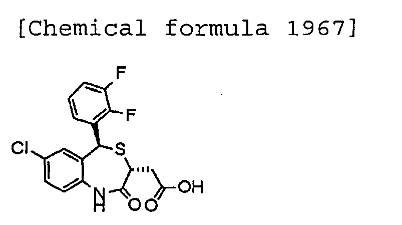

(2-Amino-5-chlorophenyl)-(2,3-difluorophenyl)methanol (1.28 g) was dissolved in dioxane (20 mL). To the resulting solution were added thiomalic acid (0.75 g) and 4N hydrochloric acid-dioxane (4 mL). The resulting mixture was stirred at 70°C for 3 hours. To the reaction mixture was added 4N hydrochloric acid-dioxane (4 mL) again, followed by stirring at 100°C for 3 days. The reaction mixture was concentrated under reduced pressure. The residue was dissolved in methanol (20 mL). To the resulting solution were added a 2N aqueous potassium carbonate solution (2 mL) and potassium carbonate (0.65 g) and the resulting mixture was stirred at 70°C for 5 hours. To the reaction mixture was added concentrated hydrochloric acid (0.5 mL). The insoluble material was collected by filtration to give the title compound (1.37 g).
¹H-NMR (400 MHz, DMSO-d₆) δ: 2.42 (1H, dd, J = 3.9, 16.9 Hz), 2.77 (1H, dd, J = 10.3, 16.9 Hz), 3.62 (1H, dd, J = 3.9, 10.3 Hz), 5.92 (1H, s), 6.68 (1H, d, J = 2.5 Hz), 7.20 (1H, d, J = 8.6 Hz), 7.39 - 7.44 (1H, m), 7.47 (1H, dd, J = 2.2, 8.3 Hz), 7.53 - 7.60 (2H, m), 10.09 (1H, s).
MS (ESI) m/z: 384 (M + H)⁺.

### Referential Example 855

Ethyl [(trans)-7-chloro-5-(2,3-difluorophenyl)-2-oxo-1,2,3,5-tetrahydro-4,1-benzothiazepin-3-yl]acetate

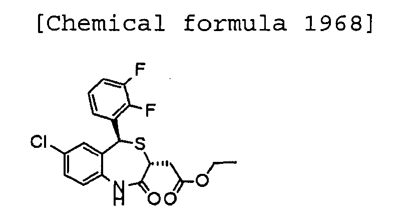

[7-Chloro-5-(2,3-difluorophenyl)-2-oxo-1,2,3,5-tetrahydro-4,1-benzothiazepin-3-yl]acetic acid (1.37 g) was suspended in 1N hydrochloric acid in ethanol (15 mL). The resulting suspension was stirred at 60°C for 3 hours. The reaction mixture was concentrated under reduced pressure. To the residue was added water and the resulting mixture was extracted three times with ethyl acetate. The organic layer was washed with saturated brine, dried over anhydrous sodium sulfate and concentrated under reduced pressure.
The residue was purified by a medium pressure purification apparatus (product of Biotage, ethyl acetate:hexane=3:7) to give the title compound (785 mg).
¹H-NMR (400 MHz, CDCl₃) δ: 1.24 (3H, t, J = 7.1 Hz), 2.43 (1H, dd, J = 3.9, 16.9 Hz), 3.11 (1H, dd, J = 10.3, 16.9 Hz), 3.84 (1H, dd, J = 3.9, 10.3 Hz), 4.13 (2H, q, J = 7.1 Hz), 6.09 (1H, s), 6.78 (1H, d, J = 2.2 Hz), 7.13 (1H, d, J = 8.3 Hz), 7.22 - 7.34 (3H, m), 7.50 - 7.53 (1H, m).
MS (ESI) m/z: 412 (M + H)⁺.

### Referential Example 856

Ethyl [(trans)-7-chloro-5-(2,3-difluorophenyl)-2-thioxo-1,2,3,5-tetrahydro-4,1-benzothiazepin-3-yl]acetate

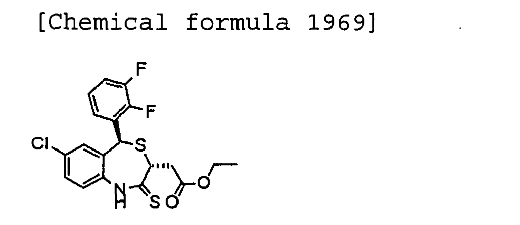

Ethyl [(trans)-7-chloro-5-(2,3-difluorophenyl)-2-oxo-1,2,3,5-tetrahydro-4,1-benzothiazepin-3-yl]acetate (715 mg) was suspended in toluene (10 mL). To the resulting suspension was added Lawesson's reagent (429 mg). The resulting mixture was stirred at 100°C for one hour. The reaction mixture was cooled to room temperature and concentrated under reduced pressure. The residue was subjected to silica gel column chromatography (chloroform → chloroform:methanol=98:2), followed by purification by a medium pressure purification apparatus (product of Biotage, ethyl acetate:hexane=1:4) to give the title compound (733 mg).
¹H-NMR (400 MHz, CDCl₃) δ: 1.24 (3H, t, J = 7.1 Hz), 2.60 (1H, dd, J = 3.9, 17.2 Hz), 3.42 (1H, dd, J = 10.0, 17.2 Hz), 4.07 - 4.16 (3H, m), 6.76 (1H, d, J = 2.2 Hz), 7.15 (1H, d, J = 8.3 Hz), 7.22 - 7.26 (2H, m), 7.34 (1H, dd, J = 2.2, 8.6 Hz), 7.46 - 7.48 (1H, m), 9.23 (1H, s).
MS (ESI) m/z: 428 (M + H)⁺.

### Example 931

Ethyl [(trans)-8-chloro-6-(2,3-difluorophenyl)-1-(trifluoromethyl)-4H,6H-[1,2,4]triazolo[4,3-a] [4,1]benzothiazepin-4-yl]acetate

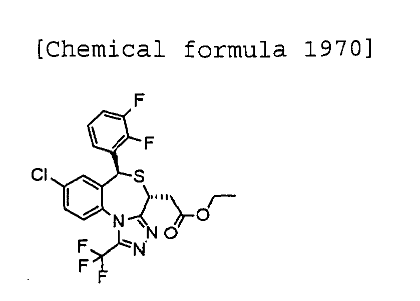

Ethyl [(trans)-7-chloro-5-(2,3-difluorophenyl)-2-thioxo-1,2,3,5-tetrahydro-4,1-benzothiazepin-3-yl]acetate (697 mg) was dissolved in tetrahydrofuran (10 mL). To the resulting solution was added hydrazine monohydrate (154 µl) in an ice bath. The resulting mixture was stirred at room temperature for 30 minutes. To the reaction mixture was added hydrazine monohydrate (154 µl), followed by stirring for further 30 minutes. To the reaction mixture was added brine and the resulting mixture was extracted three times with ethyl acetate. The organic layer was washed with saturated brine, dried over anhydrous sodium sulfate, and concentrated under reduced pressure. The residue was dissolved in 1,2-dichloroethane (10 mL). To the resulting solution was added trifluoroacetic anhydride (679 µl) in an ice bath. After stirring at room temperature for 1 hour, trifluoroacetic anhydride (679 µl) was added twice every 30 minutes. Thirty minutes after the final addition, an aqueous solution of sodium hydrogen carbonate was added and the resulting mixture was extracted three times with ethyl acetate. The organic layer was washed with saturated brine, dried over anhydrous sodium sulfate and concentrated under reduced pressure. The residue was purified by a medium pressure purification apparatus (product of Biotage, ethyl acetate:hexane=1:9 → 1:2) to give the title compound (618 mg).
¹H-NMR (400 MHz, CDCl₃) δ: 1.26 (3H, t, J = 7.1 Hz), 2.92 (1H, dd, J = 5.4, 17.1 Hz), 3.63 (1H, dd, J = 8.8, 17.1 Hz), 4.16 (2H, q, J = 7.1 Hz), 4.35 (1H, dd, J = 5.4, 8.8 Hz), 5.24 (1H, s), 6.95 (1H, d, J = 2.2 Hz), 7.21 - 7.27 (2H, m), 7.40 - 7.45 (2H, m), 7.49 (1H, dd, J = 2.5, 8.6 Hz) .
MS (ESI) m/z: 504 (M + H)⁺.

### Example 932

[(trans)-8-Chloro-6-(2,3-difluorophenyl)-1-(trifluoromethyl)-4H,6H-[1,2,4]triazolo[4,3-a][4,1]benzothiazepin-4-yl]acetic acid

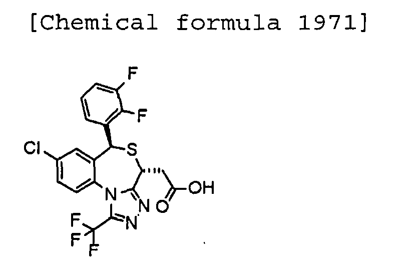

Ethyl [(trans)-8-chloro-6-(2,3-difluorophenyl)-1-(trifluoromethyl)-4H,6H-[1,2,4]triazolo[4,3-a][4,1]benzothiazepin-4-yl]acetate (158 mg) was dissolved in dioxane (8 mL). To the resulting solution was added concentrated hydrochloric acid (1 mL). The resulting mixture was stirred at 60°C for 17 hours. To the reaction mixture was added a 10% aqueous citric acid solution, followed by extraction three times with ethyl acetate. The organic layer was washed with saturated brine, dried over anhydrous sodium sulfate and concentrated under reduced pressure. The residue was purified by a medium pressure purification apparatus (product of Biotage, chloroform:methanol=98:2 → 80:20) to give the title compound (80 mg).
¹H-NMR (400 MHz, CDCl₃) δ: 2.98 (1H, dd, J = 5.1, 17.6 Hz), 3.67 (1H, dd, J = 9.3, 17.6 Hz), 4.31 (1H, dd, J = 5.1, 9.3 Hz), 5.24 (1H, s), 6.95 (1H, d, J = 2.2 Hz), 7.24 - 7.27 (2H, m), 7.41 - 7.45 (2H, m), 7.50 (1H, dd, J = 2.2, 8.6 Hz).
MS (ESI) m/z: 476 (M + H)⁺.
IR (ATR): 1626, 1485, 1286, 1173, 1147, 995, 831 cm⁻¹. Elemental analysis for: C₁₉H₁₁ClF₅N₃O₂S·0.75H₂O
Calculated: C, 46.64; H, 2.57; N, 8.59; S, 6.55.
Found: C, 46.49; H, 2.60; N, 8.36; S, 6.49.

### Referential Example 857

(2-Amino-5-chlorophenyl)(6-fluoro-2,3-dimethoxyphenyl)methanone

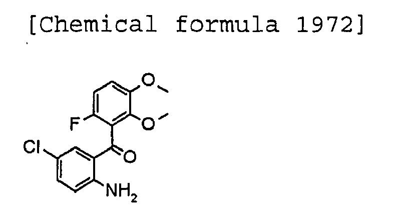

4-Fluoro-1,2-dimethoxybenzene (2.0 g) was dissolved in tetrahydrofuran (4 mL). To the resulting solution was added dropwise 2N lithium diisopropylamide (7.04 mL) at -78°C via a syringe. After the resulting mixture was stirred for 30 minutes, a solution of 6-chloro-2-methylbenzo [d] [1,3] oxazine-4-one (2.51 g) in tetrahydrofuran (4 mL) was added at once and the resulting mixture was stirred for 30 minutes in an ice bath. The reaction mixture was concentrated under reduced pressure. The residue was dissolved in ethanol (20 mL). To the resulting solution were added concentrated hydrochloric acid (5 mL) and water (3 mL) and the resulting mixture was heated under reflux for 4 hours. After cooling to room temperature, saturated brine was added to the reaction mixture, followed by extraction three times with ethyl acetate. The organic layer was washed with saturated brine, dried over anhydrous sodium sulfate and concentrated under reduced pressure. The residue was purified by a medium pressure purification apparatus (product of Biotage, ethyl acetate:hexane=2:5) to give the title compound (923 mg).
¹H-NMR (400 MHz, CDCl₃) δ: 3.79 (3H, s), 3.89 (3H, s), 6.67 (1H, d, J = 9.1 Hz), 6.86 (1H, dd, J = 8.1, 9.1 Hz), 6.94 (1H, dd, J = 5.4, 9.1 Hz), 7.21 - 7.24 (2H, m).
MS (ESI) m/z: 310 (M + H)⁺.

### Referential Example 858

(2-Amino-5-chlorophenyl)-(6-fluoro-2,3-dimethoxyphenyl)methanol

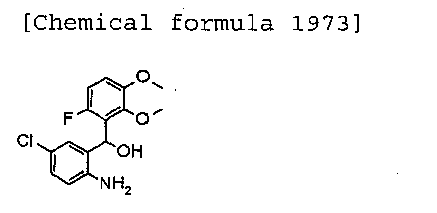

(2-Amino-5-chlorophenyl)-(6-fluoro-2,3-dimethoxyphenyl)methanone (1.11 g) was dissolved in tetrahydrofuran (15 mL). To the resulting solution was added lithium aluminum hydride (140 mg) in an ice bath, followed by stirring for 15 minutes. To the reaction mixture were added water and ethyl acetate. The insoluble material was filtered off through celite and the filtrate was extracted three times with ethyl acetate. The organic layer was washed with saturated brine, dried anhydrous sodium sulfate and concentrated under reduced pressure to give a crude product (954 mg) of the title compound.
¹H-NMR (400 MHz, CDCl₃) δ: 3.80 (3H, s), 3.88 (3H, s), 6.13 (1H, s), 6.65 (1H, d, J = 8.6 Hz), 6.73 (1H, d, J = 2.2 Hz), 6.83 - 6.91 (2H, m), 7.04 (1H, dd, J = 2.5, 8.6 Hz). MS (ESI) m/z: 294 (M - OH)⁺.

### Referential Example 859

[trans-7-Chloro-5-(6-fluoro-2,3-dimethoxyphenyl)-2-oxo-1,2,3,5-tetrahydro-4,1-benzothiazepin-3-yl]acetic acid

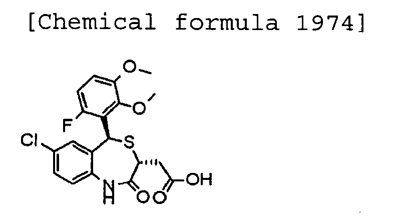

(2-Amino-5-chlorophenyl)-(6-fluoro-2,3-dimethoxyphenyl)methanol (950 mg) was dissolved in dioxane (20 mL). To the resulting solution were added thiomalic acid (480 mg) and 4N hydrochloric acid-dioxane (4 mL). The resulting mixture was stirred at 70°C for 3 hours. 4N hydrochloric acid-dioxane (4 mL) was added and the resulting mixture was stirred at 100°C for 5 hours. The reaction mixture was concentrated under reduced pressure and the residue was dissolved in methanol (20 mL). To the resulting solution were added a 2N aqueous potassium carbonate solution (1 mL) and potassium carbonate (758 mg). The resulting mixture was stirred at 70°C for 15 hours. To the reaction mixture was added concentrated hydrochloric acid. The insoluble material was filtered off. To the filtrate was added ethanol. The solid thus precipitated was collected by filtration to give the title compound (570 mg).
¹H-NMR (400 MHz, DMSO-d₆) δ: 2.37 (1H, dd, J = 3.9, 16.8 Hz), 2.72 (1H, dd, J = 10.3,16.8 Hz), 3.65 (3H, s), 3.70 (1H, dd, J = 3.9, 10.3 Hz), 3.83 (3H, s), 4.34 (1H, s), 6.13 (1H, s), 6.95 (1H, t, J = 2.5 Hz), 7.11 - 7.22 (3H, m), 7.46 (1H, dd, J = 2.2, 8.3 Hz), 10.01 (1H, s).
MS (ESI) m/z: 424 (M - H)⁻.

### Referential Example 860

Ethyl [(trans)-7-chloro-5-(6-fluoro-2,3-dimethoxyphenyl)-2-oxo-1,2,3,5-tetrahydro-4,1-benzothiazepin-3-yl]acetate

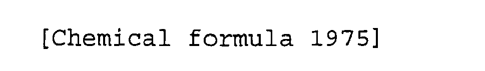

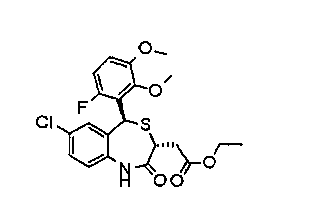

[trans-7-Chloro-5-(6-fluoro-2,3-dimethoxyphenyl)-2-oxo-1,2,3,5-tetrahydro-4,1-benzothiazepin-3-yl]acetic acid (565 mg) was dissolved in a thionyl chloride-ethanol solution (15 mL). The resulting solution was stirred at 50°C for one hour. The reaction mixture was concentrated under reduced pressure. To the residue was added water, followed by extraction three times with ethyl acetate. The organic layer was washed with saturated brine, dried over anhydrous sodium sulfate and concentrated under reduced pressure. The residue was purified by a medium pressure purification apparatus (product of Biotage, ethyl acetate:hexane=2:3) to give the title compound (405 mg).
¹H-NMR (400 MHz, CDCl₃) δ: 1.23 (3H, t, J = 7.1 Hz), 2.41 (1H, dd, J = 3.4, 16.9 Hz), 3.07 (1H, dd, J = 10.5,16.9 Hz), 3.80 (3H, s), 3.87 (3H, s), 3.99 (1H, dd, J = 3.4, 10.5 Hz), 4.12 (2H, q, J = 7.1 Hz), 6.33 (1H, s), 6.92 (2H, d, J = 8.3 Hz), 7.08 (2H, d, J = 8.3 Hz), 7.12 (1H, t, J = 2.5 Hz), 7.17 (1H, s), 7.26 - 7.29 (1H, m).
MS (ESI) m/z: 454 (M + H)⁺.

### Referential Example 861

Ethyl [(trans)-7-chloro-5-(6-fluoro-2,3-dimethoxyphenyl)-2-thioxo-1,2,3,5-tetrahydro-4,1-benzothiazepin-3-yl]acetate

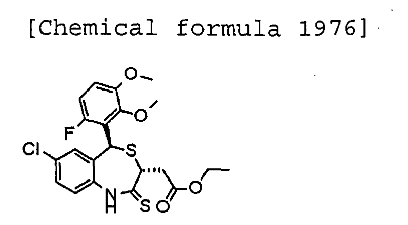

[trans-7-Chloro-5-(6-fluoro-2,3-dimethoxyphenyl)-2-oxo-1,2,3,5-tetrahydro-4,1-benzothiazepin-3-yl]acetic acid (494 mg) was dissolved in toluene (13 mL). To the resulting solution was added Lawesson's reagent (329 mg) and the resulting mixture was stirred at 100°C for one hour. The reaction mixture was concentrated under reduced pressure. The residue was subjected to silica gel column chromatography (chloroform → chloroform:methanol=98:2), followed by purification by a medium pressure purification apparatus (product of Biotage, ethyl acetate:hexane=2:3) to give the title compound (470 mg).
¹H-NMR (400 MHz, CDCl₃) δ : 1.23 (3H, t, J = 7.1 Hz), 2.58 (1H, dd, J = 3.7, 16.9 Hz), 3.38 (1H, dd, J = 10.3,16.9 Hz), 3.82 (3H, s), 3.87 (3H, s), 4.11 (2H, q, J = 7.1 Hz), 4.23 (1H, dd, J = 3.7, 10.3 Hz), 6.17 (1H, s), 6.90 - 6.93 (2H, m), 7.00 - 7.01 (1H, m), 7.11 (2H, d, J = 8.6 Hz), 7.31 (1H, dd, J = 2.5, 8.6 Hz), 9.09 (1H, s).
MS (ESI) m/z: 470 (M + H)⁺.

### Example 933

Ethyl [(trans)-8-chloro-6-(6-fluoro-2,3-dimethoxyphenyl)-1-(trifluoromethyl)-4H,6H-[1,2,4]triazolo[4,3-a] [4, 1] benzothiazepin-4-yl] acetate

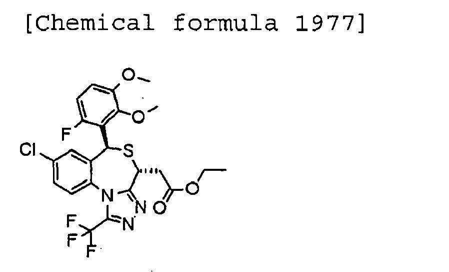

Ethyl [(trans)-7-chloro-5-(6-fluoro-2,3-dimethoxyphenyl)-2-thioxo-1,2,3,5-tetrahydro-4,1-benzothiazepin-3-yl]acetate (465 mg) was dissolved in tetrahydrofuran (8 mL). To the resulting solution was added hydrazine monohydrate (144 µl) three times every 30 minutes in an ice bath. Thirty minutes after completion of the addition, brine was added and the resulting mixture was extracted three times with ethyl acetate. The organic layer was washed with saturated brine, dried over anhydrous sodium sulfate and concentrated under reduced pressure The residue was dissolved in 1,2-dichloroethane (8 mL). To the resulting solution was added trifluoroacetic anhydride (413 µl) in an ice bath. The resulting mixture was stirred at room temperature for 1 hour. To the reaction mixture was added an aqueous solution of sodium hydrogen carbonate, followed by extraction three times with dichloromethane. The organic layer was washed with saturated brine, dried over anhydrous sodium sulfate and concentrated under reduced pressure. The residue was purified by a medium pressure purification apparatus (product of Biotage, ethyl acetate:hexane=1:1) to give the title compound (430 mg). MS (ESI) m/z: 546 (M + H)⁺.

### Example 934

(trans)-8-Chloro-6-(6-fluoro-2,3-dimethoxyphenyl)-1-(trifluoromethyl)-4H,6H-[1,2,4]triazolo[4,3-a][4,1]benzothiazepin-4-yl]acetic acid

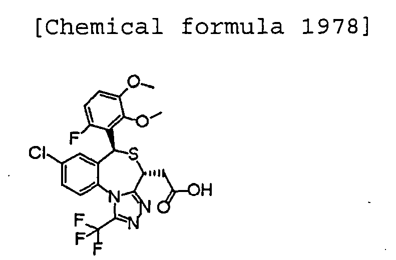

Ethyl [(trans)-8-chloro-6-(6-fluoro-2,3-dimethoxyphenyl)-1-(trifluoromethyl)-4H,6H-[1,2,4]triazolo[4,3-a][4,1]benzothiazepin-4-yl]acetate (425 mg) was dissolved in dioxane (8 mL). To the resulting solution was added concentrated hydrochloric acid (1 mL). The resulting mixture was stirred at 60°C for 8 hours. To the reaction mixture was added a 10% aqueous citric acid solution, followed by extraction three times with ethyl acetate. The organic layer was washed with saturated brine, dried over anhydrous sodium sulfate and concentrated under reduced pressure. The residue was purified successively by a medium pressure purification apparatus (product of Biotage, chloroform:methanol=98:2 → 80:20) and preparative thin layer chromatography (chloroform:methanol=88:12) to give the title compound (237 mg).
¹H-NMR (400 MHz, CDCl₃) δ: 2.90 (1H, dd, J = 4.4, 17.4 Hz), 3.62 (3H, br s), 3.63 (1H, dd, J = 9.8, 17.4 Hz), 3.84 (3H, s), 4.40 (1H, dd, J = 4.4, 5.4 Hz), 5.47 (1H, br s), 6.91 - 6.92 (2H, m), 7.30 (1H, t, J = 2.2 Hz), 7.40 (1H, d, J = 8.6 Hz), 7.48 (1H, dd, J = 2.2, 8.6 Hz).
MS (ESI) m/z: 518 (M + H)⁺.
Elemental analysis for: C₂₁H₁₆ClF₄N₃O₄S·1.5H₂O
Calculated: C, 49.19; H, 3.51; N, 7.71; S, 5.88.
Found: C, 46.41; H, 3.22; N, 7.26; S, 5.76. IR (ATR): 1736, 1485, 1246, 1194, 1171, 1117, 1078, 1039, 866, 613 cm⁻¹.

### Referential Example 862

(2,3-Dihydrobenzofuran-7-yl)methanol

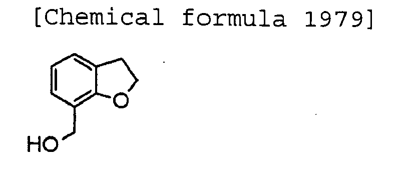

2,3-Dihydrobenzofuran-7-carboxylic acid (1.38 g) was suspended in tetrahydrofuran (15 mL). To the resulting suspension was added lithium aluminum hydride (0.32 g) in an ice bath and the resulting mixture was stirred for 1.5 hours. To the reaction mixture was added water and the resulting mixture was filtered through celite. The mother liquor was extracted three times with ethyl acetate. The organic layer was washed with saturated brine, dried over anhydrous sodium sulfate and concentrated under reduced pressure. The residue was purified by a medium pressure purification apparatus (product of Biotage, ethyl acetate:hexane=3:7) to give the title compound (1.60 g). ¹H-NMR (400 MHz, CDCl₃) δ: 3.22 (2H, t, J = 8.6 Hz), 4.61 (2H, t, J = 8.6 Hz), 4.68 (2H, s), 6.84 (1H, t, J = 7.6 Hz), 7.08 (1H, d, J = 7.6 Hz), 7.15 (1H, d, J = 7.6 Hz).

### Referential Example 863

7-Formyl-2,3-dihydrobenzofuran

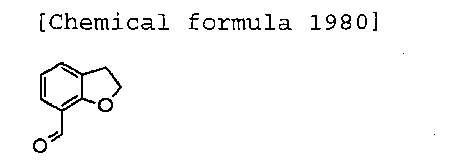

(2,3-Dihydrofuran-7-yl)methanol (1.05 g) was dissolved in dimethylsulfoxide (13 mL). To the resulting solution were added triethylamine (4.87 mL) and a sulfur trioxide-pyridine complex (2.23 g). The resulting mixture was stirred at room temperature for 22 hours. To the reaction mixture was added water, followed by extraction three times with ethyl acetate. The organic layer was washed with saturated brine, dried over anhydrous sodium sulfate and concentrated under reduced pressure. The residue was purified by a medium pressure purification apparatus (product of Biotage, ethyl acetate:hexane=15:85) to give the title compound (0.61 g).
¹H-NMR (400 MHz, CDCl₃) δ: 3.25 (2H, t, J = 8.8 Hz), 4.74 (2H, t, J = 8.8 Hz), 6.93 (1H, t, J = 7.6 Hz), 7.41 (1H, dd, J = 1.2, 7.6 Hz), 7.58 (1H, dd, J = 1.2, 7.6 Hz), 10.20 (1H, s).

### Referential Example 864

tert-Butyl {4-chloro-2-[(2,3dihydrobenzofuran-7-yl)hydroxymethyl]phenyl}carbamate

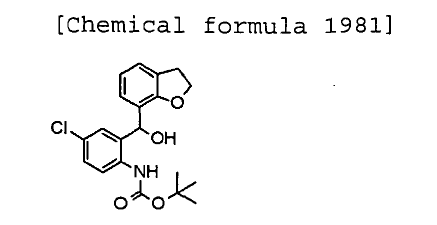

tert-Butyl-4-chlorophenylcarbamate (937 mg) was dissolved in tetrahydrofuran (15 mL). The resulting solution was cooled at an outside temperature of -30°C and sec-butyl lithium (a 2M solution, 9.0 mL) was added dropwise over 20 minutes via a syringe. After stirring at the same temperature for one hour, a tetrahydrofuran solution (10 mL) of 7-formyl-2,3-dihydrobenzofuran (610 mg) was added dropwise in 5 minutes. While gradually warming to room temperature, the resulting mixture was stirred for 15 hours. To the reaction mixture was added a saturated aqueous solution of ammonium chloride and the resulting mixture was extracted three times with ethyl acetate. The organic layer was washed with saturated brine, dried over anhydrous sodium sulfate and concentrated under reduced pressure. The residue was purified by medium pressure column chromatography (product of Biotage, ethyl acetate:hexane=3:7) to give the title compound (1011 mg). ¹H-NMR (400 MHz, CDCl₃) δ: 1.49 (9H, s), 3.17 (1H, s), 3.26 (2H, dt, J = 3.2, 8.8 Hz), 4.64 (2H, dt, J = 1.7, 8.8 Hz), 5.98 (1H, s), 6.86 (1H, d, J = 7.4 Hz), 7.00 (1H, d, J = 7.1 Hz), 7.10 (1H, d, J = 2.5 Hz), 7.17 (1H, d, J = 7.1 Hz), 7.22 (1H, dd, J = 2.5, 8.8 Hz), 7.89 (1H, d, J = 8.8 Hz), 8.00 (1H, s).
MS (ESI) m/z: 398 (M + Na)⁺.

### Referential Example 865

(2-Amino-5-chlorophenyl)-(2,3-dihydrobenzofuran-7-yl)methanol

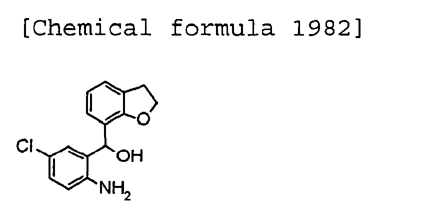

tert-Butyl {4-chloro-2-[(2,3dihydrobenzofuran-7-yl)hydroxymethyl]phenyl}carbamate (1011 mg) was dissolved in dichloromethane (8 mL). To the resulting solution was added a 4N hydrochloric acid-dioxane solution (3 mL). The resulting mixture was stirred at room temperature for 19 hours. To the reaction mixture was added an aqueous sodium hydrogen carbonate solution. The resulting mixture was extracted three times with ethyl acetate. The organic layer was washed with saturated brine, dried over anhydrous sodium sulfate and concentrated under reduced pressure.
The residue was provided for the subsequent reaction without purification.
MS (ESI) m/z: 258 (M - OH)⁺.

### Referential Example 866

[trans-7-Chloro-5-(2,3-dihydrobenzofuran-7-yl)-2-oxo-1,2,3,5-tetrahydro-4,1-benzothiazepin-3-yl]acetic acid

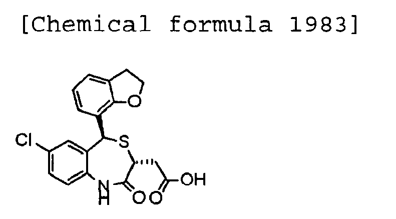

(2-Amino-5-chlorophenyl)-(2,3-dihydrobenzofuran-7-yl)methanol (833 mg) was dissolved in dioxane (20 mL). To the resulting solution were added thiomalic acid (444 mg) and a 4N hydrochloric acid-dioxane solution (4 mL). After the resulting mixture was stirred at 70°C for 2.5 hours, a 4N hydrochloric acid-dioxane solution (4 mL) was added. The resulting mixture was stirred at 100°C for 3 days. The reaction mixture was concentrated under reduced pressure. The residue was dissolved in methanol (20 mL). To the resulting solution were added a 2N aqueous potassium carbonate solution (1.35 mL) and potassium carbonate (372 mg). The resulting mixture was stirred at 70°C for 6 hours. To the reaction mixture was added potassium carbonate (372 mg) and stirring was performed for further one hour. The reaction mixture was neutralized with concentrated hydrochloric acid (1.6 mL). Brine was added and the resulting mixture was extracted three times with ethyl acetate. The organic layer was washed with saturated brine, dried over anhydrous sodium sulfate and concentrated under reduced pressure. The residue was provided for the subsequent reaction without purification.

### Referential Example 867

Ethyl [(trans)-7-chloro-5-(2,3-dihydrobenzofuran-7-yl)-2-oxo-1,2,3,5-tetrahydro-4,1-benzothiazepin-3-yl]acetate

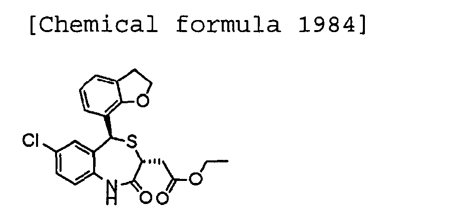

[trans-7-Chloro-5-(2,3-dihydrobenzofuran-7-yl)-2-oxo-1,2,3,5-tetrahydro-4,1-benzothiazepin-3-yl]acetic acid (1049 mg) was dissolved in N,N-dimethylformamide (20 mL). To the resulting solution were added potassium carbonate (818 mg) and iodoethane (430 µl). The resulting mixture was stirred at 30°C for 15 hours. To the reaction mixture was added iodoethane (430 µl) and the mixture was stirred for further 5 hours. The reaction mixture was concentrated under reduced pressure. To the residue was added water, followed by extraction three times with ethyl acetate. The organic layer was washed with saturated brine, dried over anhydrous sodium sulfate and concentrated under reduced pressure. The residue was purified by a medium pressure purification apparatus (Biotage, ethyl acetate:hexane=3:2) to give the title compound (770 mg).
¹H-NMR (400 MHz, CDCl₃) δ: 1.24 (3H, t, J = 7.1 Hz), 2.41 (1H, dd, J = 3.9, 17.1 Hz), 3.09 (1H, dd, J = 10.3, 17.1 Hz), 3.22 (2H, t, J = 8.6 Hz), 3.83 (1H, dd, J = 3.7, 10.3 Hz), 4.12 (2H, q, J = 7.1 Hz), 4.48 (2H, t, J = 8.8 Hz), 5.99 (1H, s), 6.92 - 6.97 (2H, m), 7.08 (1H, d, J = 8.6 Hz), 7.20 - 7.28 (2H, m), 7.33 (1H, s), 7.45 (1H, d, J = 7.8 Hz).
MS (ESI) m/z: 418 (M + H)⁺.

### Referential Example 868

Ethyl [(trans)-7-chloro-5-(2,3-dihydrobenzofuran-7-yl)-2-thioxo-1,2,3,5-tetrahydro-4,1-benzothiazepin-3-yl]acetate

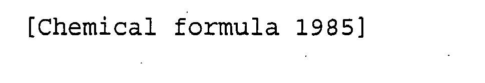

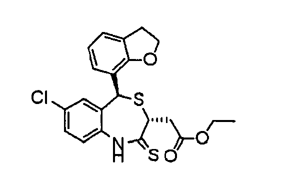

Ethyl [(trans)-7-chloro-5-(2,3-dihydrobenzofuran-7-yl)-2-oxo-1,2,3,5-tetrahydro-4,1-benzothiazepin-3-yl]acetate (765 mg) was dissolved in toluene (15 mL). To the resulting solution was added Lawesson's reagent (534 mg). The resulting mixture was stirred at 100°C for one hour. The reaction mixture was concentrated. The residue was purified successively by silica gel column chromatography (chloroform → chloroform:methanol=98:2) and
a medium pressure purification apparatus (product of Biotage, ethyl acetate:hexane=3:7) to give the title compound (508 mg).
¹H-NMR (400 MHz, CDCl₃) δ: 1.24 (3H, t, J = 7.1 Hz), 2.59 (1H, dd, J = 3.9, 17.1 Hz), 3.22 (2H, t, J = 8.8 Hz), 3.40 (1H, dd, J = 10.3, 17.1 Hz), 4.05 - 4.16 (3H, m), 4.49 (2H, t, J = 8.8 Hz), 5.82 (1H, s), 6.94 (1H, d, J = 2.2 Hz), 6.95 (1H, t, J = 7.4 Hz), 7.10 (1H, d, J = 8.3 Hz), 7.21 (1H, dd, J = 1.2, 7.4 Hz), 7.29 (1H, dd, J = 2.2, 8.3 Hz), 7.40 (1H, d, J = 7.8 Hz), 9.13 (1H, s).
MS (ESI) m/z: 434 (M + H)⁺.

### Example 935

Ethyl [(trans)-8-chloro-6-(2,3-dihydrobenzofuran-7-yl)-1-(trifluoromethyl)-4H,6H-[1,2,4]triazolo[4,3-a] [4,1]benzothiazepin-4-yl]acetate

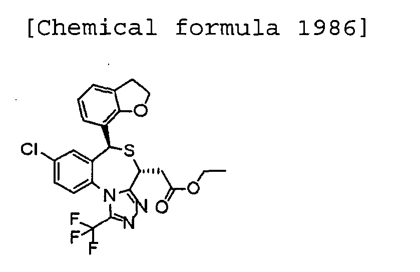

Ethyl [(trans)-7-chloro-5-(2,3-dihydrobenzofuran-7-yl)-2-thioxo-1,2,3,5-tetrahydro-4,1-benzothiazepin-3-yl]acetate (504 mg) was dissolved in tetrahydrofuran (8 mL). To the resulting solution was added hydrazine monohydrate (169 µl) four times every 30 minutes in an ice bath. One hour after the completion of the final addition, water was added to the reaction mixture, followed by extraction three times with ethyl acetate. The organic layer was washed with saturated brine, dried over anhydrous sodium sulfate and concentrated under reduced pressure. The residue was dissolved in 1,2-dichloroethane (8 mL). To the resulting solution was added trifluoroacetic anhydride (484 µl) in an ice bath and the resulting mixture was stirred at room temperature for 30 minutes. To the reaction mixture was added an aqueous solution of sodium hydrogen carbonate, followed by extraction three times with ethyl acetate. The organic layer was washed with saturated brine, dried over anhydrous sodium sulfate and concentrated under reduced pressure. The residue was purified by a medium pressure purification apparatus (product of Biotage, ethyl acetate:hexane=1:1) to give the title compound (484 mg).
MS (ESI) m/z: 510 (M + H)⁺.

### Example 936

[(trans)-8-Chloro-6-(2,3-dihydrobenzofuran-7-yl)-1-(trifluoromethyl)-4H,6H-[1,2,4]triazolo[4,3-a] [4,1]benzothiazepin-4-yl]acetic acid

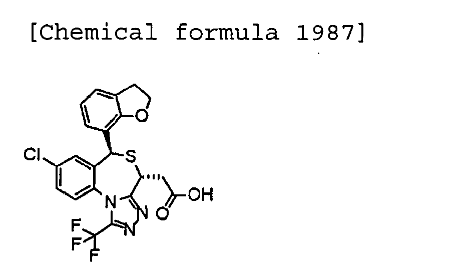

Ethyl [(trans)-8-chloro-6-(2,3-dihydrobenzofuran-7-yl)-1-(trifluoromethyl)-4H,6H-[1,2,4]triazolo[4,3-a][4,1]benzothiazepin-4-yl]acetate (480 mg) was dissolved in dioxane (8 mL). To the resulting solution was added concentrated hydrochloric acid (1 mL) and the resulting mixture was stirred at 60°C for 15 hours. To the reaction mixture was added a 10% aqueous citric acid solution and the resulting mixture was extracted three times with ethyl acetate. The organic layer was washed with saturated brine, dried over anhydrous sodium sulfate and concentrated under reduced pressure. The residue was purified successively by a medium pressure purification apparatus (product of Biotage, chloroform:methanol=8:2) and preparative thin layer chromatography (chloroform:methanol =90:10) to give the title compound (172 mg).
¹H-NMR (400 MHz, CDCl₃) δ: 2.96 (1H, dd, J = 4.9, 17.4 Hz), 3.19 (2H, t, J = 8.3 Hz), 3.64 (1H, dd, J = 9.3, 17.4 Hz), 4.28 (1H, dd, J = 4.9, 9.3 Hz), 4.43 (2H, t, J = 8.3 Hz), 5.16 (1H, s), 6.94 (1H, t, J = 7.6 Hz), 7.12 (1H, d, J = 2.2 Hz), 7.22 (1H, d, J = 7.4 Hz), 7.36 (2H, t, J = 8.6 Hz), 7.45 (1H, dd, J = 2.2, 8.3 Hz).
MS (ESI) m/z: 482 (M + H)⁺.
IR (ATR): 1741, 1485, 1454, 1192, 1151, 995, 833, 746 cm⁻¹. Elemental analysis for: C₂₁H₁₅ClF₃N₃O₃S·0.75H₂O
Calculated: C, 50.92; H, 3.36; N, 8.48; S, 6.47.
Found: C, 50.96; H, 3.38; N, 8.33; S, 6.50.

### Referential Example 869

4-(2-Ethyl-3-methoxybenzoyl)morpholine

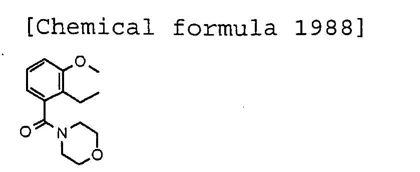

2-Ethyl-3-methoxybenzoic acid (1.40 g) and morpholine (1.01 g) were dissolved in dichloromethane (10 mL). To the resulting solution were added 1-ethyl-3-(3-dimethylaminopropyl)carbodiimide hydrochloride (1.86 g) and 1-hydroxybenzotriazole (238 mg). The resulting mixture was stirred at room temperature for 1.5 hours. The reaction mixture was diluted with chloroform, and washed with water and saturated brine. The organic layer was dried over anhydrous sodium sulfate. The filtrate was then concentrated. The residue was purified by silica gel column (ethyl acetate:hexane=1:2) to give the title compound (1.68 g).
¹H-NMR (CDCl₃) δ: 1.15 (3H, t, J = 7.5 Hz), 2.44-2.55 (1H, m), 2.67-2.78 (1H, m), 3.16-3.30 (2H, m), 3.54-3.60 (2H, m), 3.74-3.79 (2H, m), 3.81-3.84 (5H, m), 6.74 (1H, dd, J = 7.6, 1.0 Hz), 6.86 (1H, d, J = 8.1 Hz), 7.19 (1H, t, J = 8.0 Hz) .

### Referential Example 870

N-[4-Chloro-2-(2-ethyl-3-methoxybenzoyl)phenyl]-2,2-dimethylpropanamide

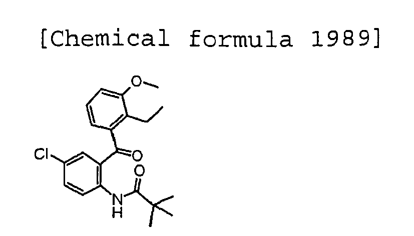

N-(4-Chlorophenyl)-2,2-dimethylpropanamide (1.43 g) was dissolved in tetrahydrofuran (30 mL). To the resulting solution was added dropwise butyl lithium (a 2.55M solution, 6.08mL) in a salt-ethanol-ice bath. After the resulting mixture was stirred for 2 hours under the same conditions, a solution of 4-(2-ethyl-3-methoxybenzoyl)morpholine (1.68 g) in tetrahydrofuran (10 mL) was added dropwise. After stirring for one hour under the same conditions, stirring was performed at room temperature for one hour. To the reaction mixture was added a saturated aqueous solution of ammonium chloride to terminate the reaction . The reaction mixture was then diluted with ethyl acetate and washed with water. The organic layer was washed with saturated brine and dried over anhydrous sodium sulfate. The filtrate was then concentrated. The residue was purified by silica gel column (ethyl acetate:hexane=1:10) to give the title compound (1.89 g).
MS (ESI) m/z : 374 (M+H)⁺.
¹H-NMR (CDCl₃) δ: 1.08 (3H, t, J = 7.5 Hz), 1.38 (9H, s), 2.56 (2H, q, J = 7.4 Hz), 3.90 (3H, s), 6.78 (1H, d, J = 7.6 Hz), 7.00 (1H, d, J = 8.6 Hz), 7.25 (1H, t, J = 7.8 Hz), 7.36 (1H, d, J = 2.5 Hz), 7.50 (1H, dd, J = 9.1, 2.7 Hz), 8.81 (1H, d, J = 9.1 Hz), 11.78 (1H, s).

### Referential Example 871

(2-Amino-5-chlorophenyl)(2-ethyl-3-methoxyphenyl)methanone

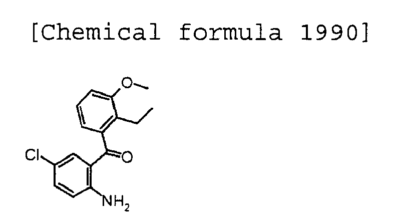

N-[4-Chloro-2-(2-ethyl-3-methoxybenzoyl)phenyl]-2,2-dimethylpropanamide (1.89 g) was dissolved in methanol (20 mL). To the resulting solution were added an aqueous solution (10 mL) of potassium hydroxide (3.0 g) and then, ethanol (10 mL). The resulting mixture was stirred at 85°C for 14 hours. The reaction mixture was allowed to cool and then, concentrated. The residue was partitioned between chloroform and water and the layers separated. The organic layer was dried over anhydrous sodium sulfate. The filtrate was then concentrated. The residue was purified by silica gel column (ethyl acetate:hexane=1:10) to give the title compound (1.33 g).
MS (ESI) m/z : 290 (M+H)⁺.
¹H-NMR (CDCl₃) δ: 1.08 (3H, t, J = 7.4 Hz), 2.55 (2H, q, J = 7.4 Hz), 3.89 (3H, s), 6.41 (2H, s), 6.66 (1H, d, J = 8.6 Hz), 6.77 (1H, dd, J = 7.6, 1.0 Hz), 6.95 (1H, d, J = 8.1 Hz), 7.17-7.25 (3H, m).

### Referential Example 872

(2-Amino-5-chlorophenyl)(2-ethyl-3-methoxyphenyl)methanol

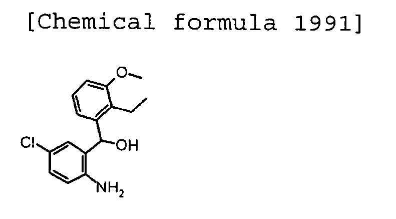

(2-Amino-5-chlorophenyl)(2-ethyl-3-methoxyphenyl)methanone (1.33 g) was dissolved in methanol (50 mL). To the resulting solution was added sodium borohydride(520 mg) in an ice water bath. The resulting mixture was stirred at room temperature for 1.5 hours. To the reaction mixture was added sodium borohydride (260 mg) further and the resulting mixture was stirred at room temperature for 15 hours. Sodium borohydride (520 mg) was added, followed by stirring for one hour. The reaction mixture was concentrated. To the residue was added an aqueous sodium chloride solution. The resulting mixture was suspended in ethyl acetate and washed with water. The organic layer was washed with a saturated aqueous solution of sodium bicarbonate and then, dried over anhydrous sodium sulfate. The filtrate was concentrated to give the title compound(1.16 g).
MS (ESI) m/z : 290 (M+H)⁺.
¹H-NMR (CDCl₃) δ: 1.07 (3H, t, J = 7.5 Hz), 2.53-2.63 (1H, m), 2.66-2.77 (1H, m), 3.86 (3H, s), 6.04 (1H, s), 6.62 (1H, d, J = 8.3 Hz), 6.86-6.90 (2H, m), 6.99 (1H, d, J = 7.8 Hz), 7.06 (1H, dd, J = 8.5, 2.3 Hz), 7.22 (1H, t, J = 8.1 Hz), 7.26 (2H, s).

### Referential Example 873

[(trans)-7-Chloro-5-(2-ethyl-3-methoxyphenyl)-2-thioxo-1,2,3,5-tetrahydro-4,1-benzothiazepin-3-yl]acetic acid

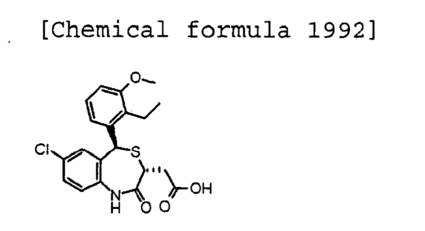

(2-Amino-5-chlorophenyl)(2-ethyl-3-methoxyphenyl)methanol (1.16 g) and 2-mercaptosuccinic acid (630 mg) were dissolved in a 4N hydrochloric acid-dioxane solution (20 mL). The resulting solution was stirred at 70°C for 2 hours. To the reaction mixture was added a 4N hydrochloric acid-dioxane solution (20 mL). The resulting mixture was stirred overnight at 100°C. The reaction mixture was concentrated. To the residue was added a 4N hydrochloric acid-dioxane solution (20 mL) further and the resulting mixture was heated under reflux for 12 hours.
The reaction mixture was allowed to cool and then concentrated. The residue thus obtained was dissolved in ethanol (20 mL)-water (1 mL). To the resulting solution was added potassium carbonate (604 mg). The resulting mixture was stirred overnight at 70°C. The reaction mixture was neutralized with 1N hydrochloric acid. The solid thus precipitated was collected by filtration to give the title compound (1.38 g).
MS (ESI) m/z : 406 (M+H)⁺.
¹H-NMR (CDCl₃) δ: 0.87 (3H, t, J = 7.4 Hz), 2.24-2.35 (1H, m), 2.40 (1H, dd, J = 16.7, 3.7 Hz), 2.51-2.62 (1H, m), 3.05 (1H, dd, J = 16.9, 10.0 Hz), 3.77-3.85 (4H, m), 5.97 (1H, s), 6.77 (1H, d, J = 1.5 Hz), 6.92 (1H, d, J = 8.3 Hz), 7.10 (1H, d, J = 7.8 Hz), 7.18-7.23 (1H, m), 7.30 (1H, t, J = 8.0 Hz), 7.37 (1H, d, J = 7.8 Hz), 9.10 (1H, s).

### Referential Example 874

Ethyl [(trans)-7-chloro-5-(2-ethyl-3-methoxyphenyl)-2-oxo-1,2,3,5-tetrahydro-4,1-benzothiazepin-3-yl]acetate

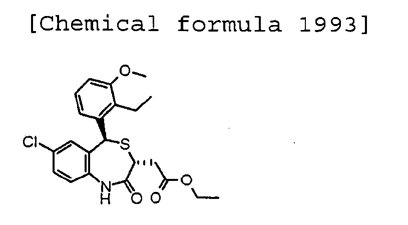

[(trans)-7-Chloro-5-(2-ethyl-3-methoxyphenyl)-2-thioxo-1,2,3,5-tetrahydro-4,1-benzothiazepin-3-yl]acetic acid(1.38 g) was dissolved in dimethylformamide (20 mL). To the resulting solution were added potassium carbonate (1.03 g) and iodoethane (1.06 g). The resulting mixture was stirred at 30°C for 4 hours. The reaction mixture was diluted with ethyl acetate and washed with water. The organic layer was washed with saturated brine and dried over anhydrous sodium sulfate. The filtrate was concentrated to give the title compound (1.15 g).
MS (ESI) m/z : 434 (M+H)⁺.
¹H-NMR (CDCl₃) δ: 0.79 (3H, t, J = 7.5 Hz), 1.16 (3H, t, J = 7.4 Hz), 2.20-2.31 (1H, m), 2.45-2.53 (2H, m), 2.73-2.91 (1H, m), 3.64 (1H, dd, J = 10.4, 4.0 Hz), 3.82 (3H, s), 3.98-4.07 (2H, m), 5.86 (1H, s), 6.64 (1H, d, J = 2.5 Hz), 7.05 (1H, d, J = 7.8 Hz), 7.16 (1H, d, J = 8.3 Hz), 7.29 (1H, dd, J = 7.7, 1.1 Hz), 7.35 (1H, d, J = 7.7 Hz), 7.39 (1H, dd, J = 8.5, 2.5 Hz), 10.06 (1H, s).

### Referential Example 875

Ethyl [(trans)-7-chloro-5-(2-ethyl-3-methoxyphenyl)-2-thioxo-1,2,3,5-tetrahydro-4,1-benzothiazepin-3-yl]acetate

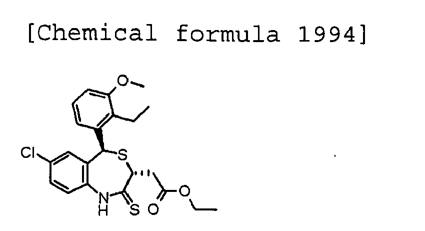

Ethyl [(trans)-7-chloro-5-(2-ethyl-3-methoxyphenyl)-2-oxo-1,2,3,5-tetrahydro-4,1-benzothiazepin-3-yl]acetate (1.15 g) was dissolved in toluene (30 mL). To the resulting solution was added Lawesson's reagent (750 mg). The resulting mixture was heated under reflux for 2 hours. The reaction mixture was concentrated. The residue was purified by silica gel column (methanol:chloroform=2:98) to give the title compound (1.046 g).
MS (ESI) m/z : 450 (M+H)⁺.
¹H-NMR (CDCl₃) δ: 0.81 (3H, t, J = 7.4 Hz), 1.15 (3H, t, J = 7.2 Hz), 2.19-2.31 (1H, m), 2.49-2.55 (1H, m), 2.66 (1H, dd, J = 16.5, 3.8 Hz), 3.18 (1H, dd, J = 16.7, 10.5 Hz), 3.82 (3H, s), 3.89 (1H, dd, J = 10.3, 3.7 Hz), 4.02 (2H, q, J = 7.2 Hz), 5.65 (1H, s), 6.66 (1H, d, J = 2.5 Hz), 7.07 (1H, d, J = 8.3 Hz), 7.20-7.25 (2H, m), 7.37 (1H, t, J = 8.0 Hz), 7.47 (1H, dd, J = 8.6, 2.5 Hz), 8.30 (1H, s).

### Example 937

Ethyl [(trans)-8-chloro-6-(2-ethyl-3-methoxyphenyl)-1-(trifluoromethyl)-4H,6H-[1,2,4]triazolo[4,3-a][4,1]benzothiazepin-4-yl]acetate

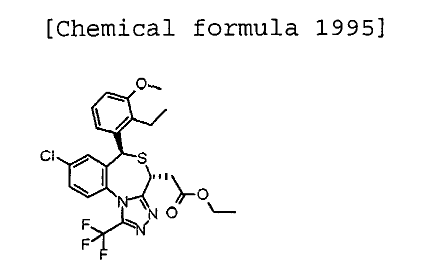

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a][4,1]benzoxazepin-4-yl]acetate, the title compound (444 mg) was obtained from ethyl [(trans)-7-chloro-5-(2-ethyl-3-methoxyphenyl)-2-thioxo-1,2,3,5-tetrahydro-4,1-benzothiazepin-3-yl]acetate (440 mg).
MS (ESI) m/z : 526 (M+H)⁺.
¹H-NMR (CDCl₃) δ: 0.68 (3H, t, J = 7.5 Hz), 1.26 (3H, t, J = 7.2 Hz), 2.09-2.20 (1H, m), 2.29-2.41 (1H, m), 2.90 (1H, dd, J = 17.2, 5.1 Hz), 3.62 (1H, dd, J = 17.3, 8.9 Hz), 3.82 (3H, s), 4.15 (2H, q, J = 7.2 Hz), 4.33 (1H, dd, J = 9.1, 5.4 Hz), 5.16 (1H, s), 6.88-6.94 (1H, m), 7.03 (1H, d, J = 2.0 Hz), 7.28-7.31 (2H, m), 7.39 (1H, d, J = 8.8 Hz), 7.45 (1H, dd, J = 8.6, 2.2 Hz).

### Example 938

[(trans)-8-Chloro-6-(2-ethyl-3-methoxyphenyl)-1-(trifluoromethyl)-4H,6H-[1,2,4]triazolo[4,3-a][4,1]benzothiazepin-4-yl]acetic acid

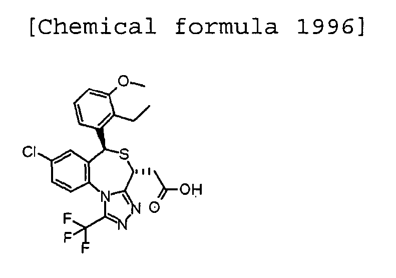

Ethyl [(trans)-8-chloro-6-(2-ethyl-3-methoxyphenyl)-1-(trifluoromethyl)-4H,6H-[1,2,4]triazolo[4,3-a][4,1]benzothiazepin-4-yl]acetate (440 mg) was dissolved in dioxane (10 mL). To the resulting solution was added concentrated hydrochloric acid (2 mL) and the resulting mixture was stirred at 60°C for one day. To the reaction mixture was added ethyl acetate, followed by washing with water. The organic layer was washed with saturated brine and dried over anhydrous sodium sulfate. The filtrate was concentrated to give a crudely purified title compound (498 mg). The resulting crudely purified title compound (158 mg) was purified using (methanol:chloroform=2:98) to give the title compound (138 mg).
MS (ESI) m/z : 498 (M+H)⁺.
¹H-NMR (CDCl₃) δ: 0.68 (3H, t, J = 7.5 Hz), 2.08-2.20 (1H, m), 2.29-2.40 (1H, m), 2.97 (1H, dd, J = 17.6, 4.9 Hz), 3.66 (1H, dd, J = 17.6, 9.3 Hz), 3.82 (3H, s), 4.30 (1H, dd, J = 9.3, 4.9 Hz), 5.17 (1H, s), 6.92 (1H, dd, J = 7.1, 2.0 Hz), 7.04 (1H, d, J = 2.5 Hz), 7.27-7.33 (2H, m), 7.40 (1H, d, J = 8.6 Hz), 7.46 (1H, dd, J = 8.6, 2.0 Hz).
Elemental analysis for: C₂₂H₁₉ClF₃N₃O₃S·0.25H₂O
Calculated: C, 52.59; H, 3.91; N, 8.36; S, 6.38; Cl, 7.06; F, 11.33.
Found: C, 52.53; H, 3.87; N, 8.31; S, 6.28; Cl, 6.99; F, 11.15.

### Referential Example 876

(2-Amino-5-chlorophenyl)(2,3-dimethoxyphenyl)methanone

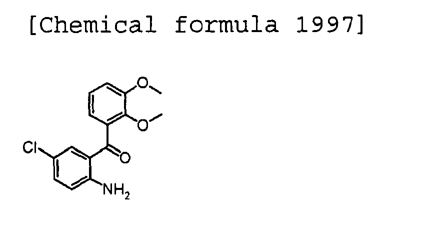

(2-Amino-5-chlorophenyl)(2,3-dimethoxyphenyl)methanol (48.7 g) was dissolved in dichloromethane (500 mL). To the resulting solution was added manganese dioxide (70.5 g) and the resulting mixture was stirred at 50°C for 13 hours.
The reaction mixture was allowed to cool to room temperature and filtered through celite. The solvent was distilled off under reduced pressure to give the title compound (49.2 g).
1H-NMR (CDCl3) δ: 3.78 (3H, s), 3.92 (3H, s), 6.38 (2H, br s), 6.65 (1H, d, J = 8.78 Hz), 6.82 (1H, dd, J = 7.68, 1.59 Hz), 7.00-7.05 (1H, m), 7.12 (1H, t, J = 7.93 Hz), 7.24-7.18 (2H, m).

### Referential Example 877

4-Chloro-2-[1-(2,3-dimethoxyphenyl)vinyl]aniline

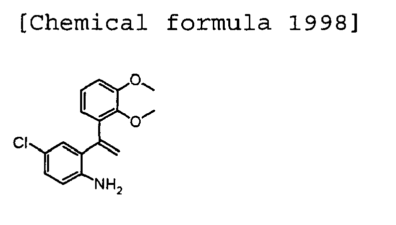

(2-Amino-5-chlorophenyl)(2,3-dimethoxyphenyl)methanone (49.2 g) was dissolved in tetrahydrofuran (500 mL). Under ice cooling, methyl triphenylphosphonium iodide (102.7 g) and potassium t-butoxide (27.9 g) were added to the resulting solution. While warming to room temperature, the resulting mixture was stirred for 5 hours. Under ice cooling, water was added to the reaction mixture, followed by extraction with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (52.1 g).
1H-NMR (CDCl3) δ: 3.48 (3H, s), 3.85 (3H, s), 5.50 (1H, d, J = 1.71 Hz), 5.62 (1H, d, J = 1.71 Hz), 6.59 (1H, d, J = 8.79 Hz), 6.92-6.87 (2H, m), 7.07-6.99 (3H, m).

### Referential Example 878

4-Chloro-N-(2,4-dimethoxybenzyl)-2-[1-(2,3-dimethoxyphenyl)vinyl]aniline

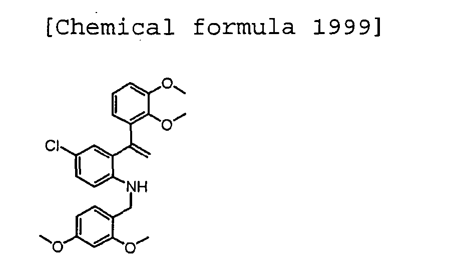

4-Chloro-2-[1-(2,3-dimethoxyphenyl)vinyl]aniline (52.1 g) was dissolved in a solvent mixture of methanol(600 mL) and acetic acid (300 mL). To the resulting solution were added Molecular Sieve 3A and 2,4-dimethoxybenzaldehyde (33.1 g). The resulting mixture was stirred at 60°C for 2 hours. Under ice cooling, sodium borocyanide (13.6 g) was added and the resulting mixture was stirred for 1.5 hours. After distilling off the solvent under reduced pressure, the residue was dissolved in ethyl acetate under ice cooling. To the resulting solution was added a saturated aqueous solution of sodium bicarbonate and the mixture was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (73.5 g). 1H-NMR (CDCl3) δ: 3.40 (3H, s), 3.75 (3H, s), 3.78 (3H, s), 3.84 (3H, s), 4.19 (2H, s), 4.61 (1H, s), 5.50 (1H, d, J = 1.71 Hz), 5.66 (1H, d, J = 1.71 Hz), 6.36 (1H, dd, J = 8.30, 2.44 Hz), 6.41 (1H, d, J = 2.44 Hz), 6.56 (1H, d, J = 8.79 Hz), 6.83 (1H, dd, J = 7.57, 1.46 Hz), 6.87 (1H, dd, J = 8.18, 1.34 Hz), 7.00-6.94 (3H, m), 7.06-7.01 (1H, m).

### Referential Example 879

Methyl 3-chloro-4-{4-chloro(2,4-dimethoxybenzyl)-2-[1-(2,3-dimethoxyphenyl)vinyl]anilino}-4-oxobutanoate

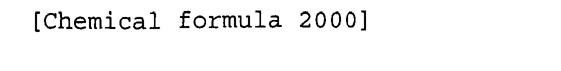

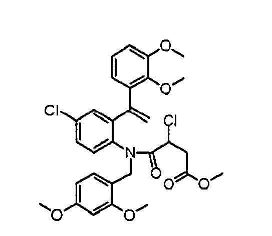

4-Chloro-N-(2,4-dimethoxybenzyl)-2-[1-(2,3-dimethoxyphenyl)vinyl]aniline (11.6 g) was dissolved in dichloromethane (180 mL). Under ice cooling, sodium hydrogen carbonate (4.42 g) was added to the resulting solution. To the resulting mixture was added dropwise a solution of methyl 3,4-dichloro-4-oxobutanoate (7.31 g) in dichloromethane (20 mL) over 10 minutes. While warming to room temperature, the resulting mixture was stirred for 5 hours. To the reaction mixture was added a saturated aqueous solution of sodium bicarbonate, followed by extraction with dichloromethane. The extract was dried over anhydrous sodium sulfate and then, the solvent was distilled off under reduced pressure. The residue was purified by silica gel column chromatography (hexane-ethyl acetate) to give the title compound (6.84 g).

### Referential Example 880

Methyl 2-[7-chloro-1-(2,4-dimethoxybenzyl)-5-(2,3-dimethoxyphenyl)-2-oxo-2,3,4,5-tetrahydro-1H-1-benzazepin-3-yl] acetate

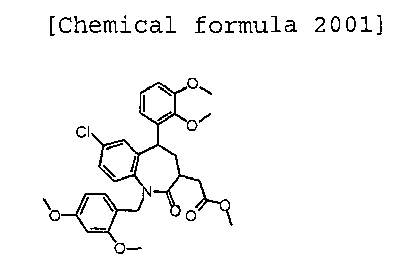

Methyl 3-chloro-4-{4-chloro(2,4-dimethoxybenzyl)-2-[1-(2,3-dimethoxyphenyl)vinyl]amlino}-4-oxobutanoate (6.84 g) was dissolved in toluene (100 mL). To the resulting solution were added dropwise a solution of tributyltin hydride (4.83 mL) and azobisisobutyronitrile (195 mg) in toluene (50 mL) over 20 minutes while heating under reflux at 120°C. The reaction mixture was heated under reflux for further 2 hours. The solvent was distilled off under reduced pressure. To the residue were added diethyl ether (150 mL) and a 8% aqueous potassium fluoride solution (150 mL). The resulting mixture was stirred at room temperature for 2 hours. The reaction mixture was extracted with diethyl ether. The extract was washed with saturated brine and then, dried over anhydrous sodium sulfate. The solvent was distilled off under reduced pressure. The residue was purified by silica gel column chromatography (hexane-ethyl acetate) to give the title compound (1.76 g).
1H-NMR (CDCl3) δ: 2.0-2.1 (1H, m), 2.3-2.4 (1H, m), 2.4-2.5 (1H, m), 3.0-3.1 (1H, m), 3.2 (3H, s), 3.4-3.5 (1H, m), 3.6-3.7 (6H, m), 3.8-3.8 (3H, m), 3.8-3.9 (3H, m), 4.6-4.6 (1H, m), 5.1 (2H, d, J = 2.4 Hz), 6.4-7.3 (9H, m).

### Referential Example 881

2-[7-Chloro-1-(2,4-dimethoxybenzyl)-5-(2,3-dimethoxyphenyl)-2-oxo-2,3,4,5-tetrahydro-1H-1-benzazepin-3-yl]acetic acid

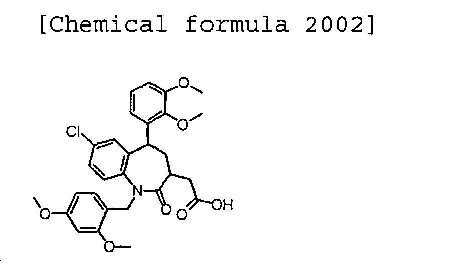

Methyl 2-[7-chloro-1-(2,4-dimethoxybenzyl)-5-(2,3-dimethoxyphenyl)-2-oxo-2,3,4,5-tetrahydro-1H-1-benzazepin-3-yl]acetate (928 mg) was dissolved in a solvent mixture of tetrahydrofuran (10 mL), methanol(10 mL) and water(5 mL). To the resulting solution was added lithium hydroxide (211 mg). The resulting mixture was stirred at room temperature for four hours and then at 50°C for one hour. Under ice cooling, a 1N aqueous hydrochloric acid solution was added to make the reaction mixture acidic, followed by extraction with ethyl acetate. The extract was washed with saturated brine and dried over anhydrous sodium sulfate. The solvent was distilled off under reduced pressure to give the title compound.

### Referential Example 882

Methyl 5-({2-[7-chloro-1-(2,4-dimethoxybenzyl)-5-(2,3-dimethoxyphenyl)-2-oxo-2,3,4,5-tetrahydro-1H-1-benzazepin-3-yl]acetyl}amino)-4-oxopentanoate

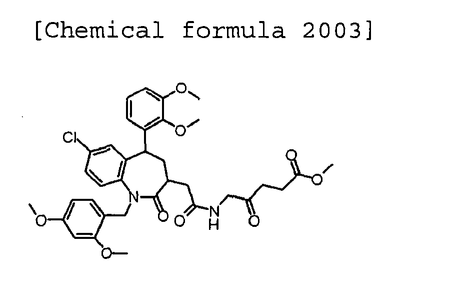

2-[7-chloro-1-(2,4-dimethoxybenzyl)-5-(2,3-dimethoxyphenyl)-2-oxo-2,3,4,5-tetrahydro-1H-1-benzazepin-3-yl]acetic acid obtained above was dissolved in dichloromethane (20 mL). Under ice cooling, methyl 5-amino-4-oxopentanoate hydrochloric acid (367 mg), 1-ethyl-3-(3-dimethylaminopropyl)carbodiimide hydrochloride (387 mg), 1-hydroxybenzotriazole (77.2 mg), and triethylamine (0.282 mL) were added to the resulting solution. The resulting mixture was stirred for 37 hours. To the reaction mixture was added chloroform. The resulting mixture washed with saturated brine and dried over anhydrous sodium sulfate. The solvent was distilled off under reduced pressure. The residue was purified by silica gel column chromatography (chloroform-methanol) to give the title compound (1.15 g).

### Referential Example 883

Methyl 3-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1,3-thiazol-5-yl)propanoate

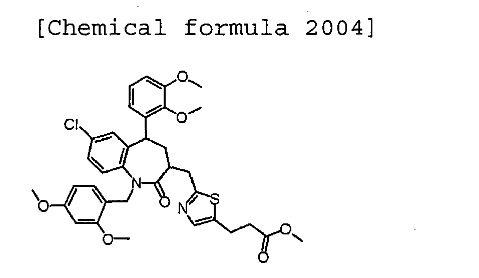

Methyl 5-({2-[7-chloro-1-(2,4-dimethoxybenzyl)-5-(2,3-dimethoxyphenyl)-2-oxo-2,3,4,5-tetrahydro-1H-1-benzazepin-3-yl]acetyl}amino)-4-oxopentanoate (622 mg) was dissolved in tetrahydrofuran (5 mL). To the resulting solution was added Lawesson's reagent (428 mg) at room temperature. The resulting mixture was stirred at 70°C for 2 hours. The solvent was distilled off under reduced pressure. The residue was purified by silica gel column chromatography (hexane-ethyl acetate) to give the title compound (444 mg).
1H-NMR (CDCl3) δ: 2.13 (1H, td, J = 12.21, 6.59 Hz), 2.56 (1H, td, J = 12.94, 7.57 Hz), 2.65 (2H, t, J = 7.45 Hz), 3.02 (1H, dd, J = 14.89, 5.37 Hz), 3.10 (2H, t, J = 7.32 Hz), 3.21 (3H, s), 3.56 (1H, dd, J = 14.89, 8.06 Hz), 3.64-3.70 (7H, m), 3.79 (3H, s), 3.85 (3H, s), 4.61 (1H, q, J = 6.67 Hz), 5.06 (2H, d, J = 3.66 Hz), 6.39-6.43 (2H, m), 6.60-6.63 (1H, m), 6.87-6.92 (2H, m), 7.08-7.15 (3H, m), 7.19 (1H, d, J = 9.03 Hz), 7.33-7.35 (1H, m).

### Referential Example 884

Methyl 3-(2-{[7-chloro-5-(2,3-dimethoxyphenyl)-2-oxo-2,3,4,5-tetrahydro-1H-1-benzazepin-3-yl]methyl}-1,3-thiazol-5-yl)propanoate

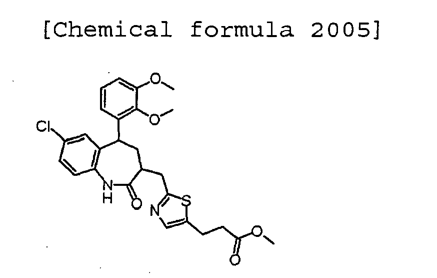

Methyl 3-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1,3-thiazol-5-yl)propanoate (444 mg) was dissolved in acetone (30 mL). Under ice cooling, an aqueous solution (6 mL) of ceric(IV) ammonium nitrate (1.10 g) was added and the resulting mixture was stirred at room temperature for 4.5 hours. To the reaction mixture were added a saturated aqueous solution of sodium bicarbonate and ethyl acetate, followed by filtration through celite. The filtrate was extracted with ethyl acetate. The extract was washed with saturated brine and dried over anhydrous sodium sulfate. The solvent was distilled off under reduced pressure. The residue was purified by silica gel column chromatography (ethyl acetate-hexane) to give the title compound (249 mg). 1H-NMR (CDCl3) δ: 2.14 (1H, td, J = 12.21, 6.59 Hz), 2.61-2.72 (3H, m), 2.97-3.13 (4H, m), 3.43-3.55 (4H, m), 3.69 (3H, s), 3.87 (3H, s), 4.81 (1H, q, J = 6.75 Hz), 6.68-6.81 (1H, m), 6.91-7.00 (3H, m), 7.07-7.18 (2H, m), 7.34-7.31 (1H, m).

### Referential Example 885

Methyl 3-(2-{[7-chloro-5-(2,3-dimethoxyphenyl)-2-thioxo-2,3,4,5-tetrahydro-1H-1-benzazepin-3-yl]methyl}-1,3-thiazol-5-yl)propanoate

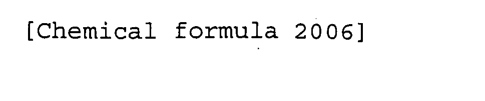

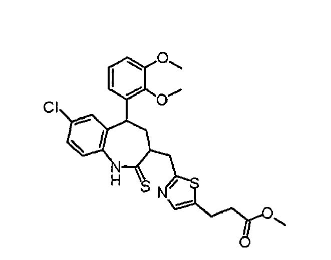

Methyl 3-(2-{[7-chloro-5-(2,3-dimethoxyphenyl)-2-oxo-2,3,4,5-tetrahydro-1H-1-benzazepin-3-yl]methyl}-1,3-thiazol-5-yl)propanoate (249 mg) was dissolved in toluene (5 mL). To the resulting solution was added Lawesson's reagent (222 mg). The resulting mixture was heated under reflux for 3 hours. The solvent was distilled off under reduced pressure. The residue was purified by silica gel column chromatography (hexane-ethyl acetate) to give the title compound (164 mg).
1H-NMR (CDCl3) δ: 2.16-2.26 (1H, m), 2.64 (2H, t, J = 7.56 Hz), 2.68-2.76 (1H, m), 3.09 (2H, t, J = 7.93 Hz), 3.17-3.33 (1H, m), 3.51 (3H, s), 3.69 (3H, s), 3.77-3.84 (1H, m), 3.85 (3H, d, J = 14.39 Hz), 4.69 (1H, dd, J = 13.41, 6.34 Hz), 6.70 (1H, d, J = 2.20 Hz), 6.90-6.95 (2H, m), 6.99 (1H, d, J = 8.29 Hz), 7.23-7.12 (2H, m), 7.31 (1H, s), 9.26 (1H, s).

### Referential Example 886

Methyl 3-[2-({7-chloro-5-(2,3-dimethoxyphenyl)-2-[(Z)hydrazono]-1,3,4,5-tetrahydro-2H-1-benzazepin-3-yl}methyl)-1,3-thiazol-5-yl]propanoate

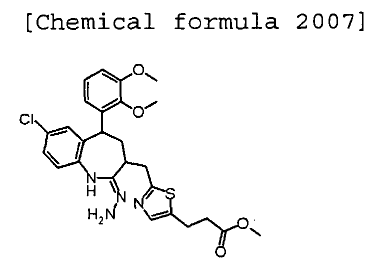

Methyl 3-(2-{[7-chloro-5-(2,3-dimethoxyphenyl)-2-thioxo-2,3,4,5-tetrahydro-1H-1-benzazepin-3-yl]methyl}-1,3-thiazol-5-yl)propanoate (103 mg) was dissolved in tetrahydrofuran (3 mL). To the resulting solution was added hydrazine monohydrate (0.047 mL) and the resulting mixture was stirred at room temperature for 22 hours. The solvent was distilled off under reduced pressure to give the title compound. The resulting compound was provided for the subsequent reaction without purification.

### Example 939

Methyl 3-(2-{[8-chloro-6-(2,3-dimethoxyphenyl)-1-(trifluoromethyl)-5,6-dihydro-4H-[1,2,4]triazolo[4,3-a][1]benzazepin-4-yl]methyl}-1,3-thiazol-5-yl)propanoate

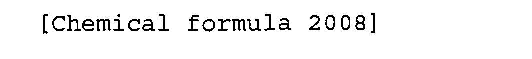

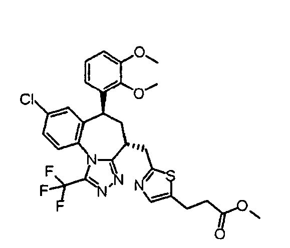

Methyl 3-[2-({7-chloro-5-(2,3-dimethoxyphenyl)-2-[(Z)hydrazono]-1,3,4,5-tetrahydro-2H-1-benzazepin-3-yl}methyl)-1,3-thiazol-5-yl]propanoate (68.8 mg) was dissolved in dichloromethane (3 mL). Under ice cooling, trifluoroacetic anhydride (0.055 mL) and trifluoroacetic acid (0.265 mL) were added. The resulting mixture was stirred at room temperature for 2 hours and then at 55°C for 2 hours. To the reaction mixture was added toluene (3 mL) further and the resulting mixture was heated under reflux for 3 hours. The solvent was distilled off under reduced pressure. To the residue were added ethyl acetate and a saturated aqueous solution of sodium bicarbonate, followed by extraction with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (45.2 mg) .
1H-NMR (CDCl3) δ: 2.13 (1H, dd, J = 17.32, 12.68 Hz), 2.65 (2H, t, J = 7.44 Hz), 2.84-2.72 (1H, m), 3.11 (2H, t, J = 7.19 Hz), 3.41-3.29 (4H, m), 3.44-3.53 (1H, m), 3.69 (3H, s), 3.84 (3H, s), 3.88-3.97 (1H, m), 4.09 (1H, dd, J = 13.05, 5.00 Hz), 6.82 (1H, s), 6.92 (2H, t, J = 7.19 Hz), 7.14 (1H, t, J = 7.93 Hz), 7.34-7.41 (3H, m).

### Example 940

3-(2-{[(trans)-8-Chloro-6-(2,3-dimethoxyphenyl)-1-(trifluoromethyl)-5,6-dihydro-4H-[1,2,4]triazolo[4,3-a][1]benzazepin-4-yl]methyl}-1,3-thiazol-5-yl)propanoic acid

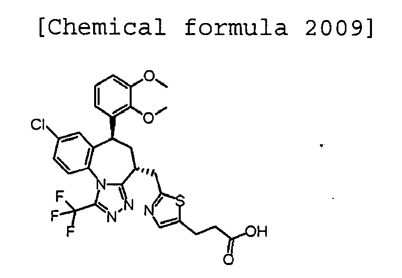

Methyl 3-(2-{[8-chloro-6-(2,3-dimethoxyphenyl)-1-(trifluoromethyl)-5,6-dihydro-4H-[1,2,4]triazolo[4,3-a] [1]benzazepin-4-yl]methyl}-1,3-thiazol-5-yl)propanoate (45.2 mg) was dissolved in a mixed solution of tetrahydrofuran, methanol and water (2:2:1, 4 mL). To the resulting solution was added potassium carbonate (41 mg). The resulting mixture was stirred at 50°C for 2 hours. Under ice cooling, 1N hydrochloric acid was added to the reaction mixture to make it acidic, followed by extraction with ethyl acetate. The extract washed with saturated brine and dried over anhydrous sodium sulfate. To the residue obtained by distilling off the solvent was added ether-hexane. The precipitate thus formed was collected by filtration to give the title compound (29 mg).
1H-NMR (CDCl3) δ: 2.09-2.21 (1H, m), 2.69 (2H, t, J = 7.32 Hz), 2.73-2.83 (1H, m), 3.11 (2H, t, J = 7.32 Hz), 3.31-3.39 (4H, m), 3.43-3.58 (1H, m), 3.84 (3H, s), 3.89-3.98 (1H, m), 4.01-4.16 (1H, m), 6.80-6.83 (1H, m), 6.90-6.95 (2H, m), 7.17-7.11 (1H, m), 7.40-7.35 (3H, m).
Elemental analysis for: C₂₇H₂₄ClF₃N₄O₄S
Calculated: C, 54.68; H, 4.08; N, 9.45.
Found: C, 54.77; H, 4.03; N, 9.07.

### Example 941

Methyl 3-(2-{[8-chloro-6-(2,3-dimethoxyphenyl)-1-isopropyl-5,6-dihydro-4H-[1,2,4]triazolo[4,3-a][1]benzazepin-4-yl]methyl}-1,3-thiazol-5-yl)propanoate

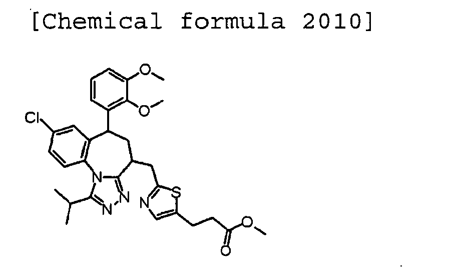

Methyl 3-[2-({7-chloro-5-(2,3-dimethoxyphenyl)-2-[(Z)hydrazono]-1,3,4,5-tetrahydro-2H-1-benzazepin-3-yl}methyl)-1,3-thiazol-5-yl]propanoate (50.0 mg) was dissolved in dichloromethane (3 mL). Under ice cooling, isobutyric anhydride (0.047 mL) and isobutyric acid (0.176 mL) were added. The resulting mixture was stirred at room temperature for 2 hours and then, at 55°C for 2 hours. Toluene (3 mL) was added further and the resulting mixture was heated under reflux for 3 hours. The solvent was distilled off under reduced pressure. To the residue was added ethyl acetate and a saturated aqueous solution of sodium bicarbonate. The resulting mixture was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (41.6 mg).
1H-NMR (CDCl3) δ: 1.15 (3H, d, J = 6.83 Hz), 1.60 (3H, d, J = 6.83 Hz), 2.02-2.12 (1H, m), 2.60-2.79 (3H, m), 3.10 (2H, t, J = 7.44 Hz), 3.18-3.31 (2H, m), 3.39-3.51 (4H, m), 3.69 (3H, s), 3.82-3.94 (4H, m), 4.08-4.17 (1H, m), 6.76-6.79 (1H, m), 6.91-6.97 (2H, m), 7.14 (1H, t, J = 8.05 Hz), 7.21-7.39 (3H, m).

### Example 942

3-(2-{[8-Chloro-6-(2,3-dimethoxyphenyl)-1-isopropyl-5,6-dihydro-4H-[1,2,4]triazolo[4,3-a] [1]benzazepin-4-yl]methyl}-1,3-thiazol-5-yl)propanoic acid

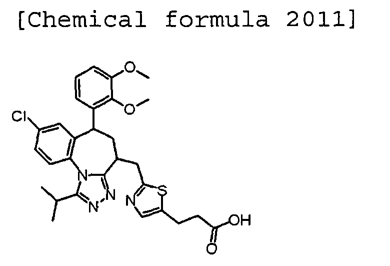

Methyl 3-(2-{[8-chloro-6-(2,3-dimethoxyphenyl)-1-isopropyl-5,6-dihydro-4H-[1,2,4]triazolo[4,3-a][1]benzazepin-4-yl]methyl}-1,3-thiazol-5-yl)propanoate (41.6 mg) was dissolved in a mixed solution of tetrahydrofuran, methanol and water (2:2:1, 4 mL). To the resulting solution was added potassium carbonate (40 mg) and the resulting mixture was stirred at 50°C for 2 hours. Under ice cooling, 1N hydrochloric acid was added to the reaction mixture to make it acidic, followed extraction with ethyl acetate. The extract was washed with saturated brine and dried over anhydrous sodium sulfate. To the residue obtained by distilling off the solvent was added ether-hexane. The precipitate thus formed was collected by filtration to give the title compound (20 mg). 1H-NMR (CDCl3) δ: 1.15 (3H, d, J = 6.83 Hz), 1.60 (3H, d, J = 6.83 Hz), 2.03-2.12 (1H, m), 2.65-2.79 (3H, m), 3.09-3.15 (2H, m), 3.21-3.32 (2H, m), 3.36-3.55 (4H, m), 3.73-3.97 (4H, m), 4.02-4.19 (1H, m), 6.75-6.79 (1H, m), 6.90-6.96 (2H, m), 7.11-7.17 (1H, m), 7.22-7.29 (1H, m), 7.29-7.33 (1H, m), 7.40-7.37 (1H, m).
Elemental analysis for: C₂₉H₃₁ClN₄O₄S-0.375H₂O
Calculated: C, 60.70; H, 5.58; N, 9.76.
Found: C, 61.00; H, 5.55; N, 9.35.

### Referential Example 887

Methyl 3-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1,3-oxazol-5-yl)propanoate

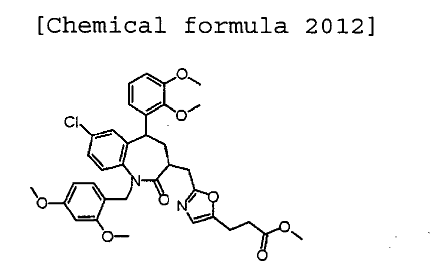

Methyl 5-({2-[7-chloro-1-(2,4-dimethoxybenzyl)-5-(2,3-dimethoxyphenyl)-2-oxo-2,3,4,5-tetrahydro-1H-1-benzazepin-3-yl]acetyl}amino)-4-oxopentanoate (525 mg) was dissolved in dimethylformamide (5 mL). To the resulting solution was added phosphorus oxychloride (0.110 mL). The resulting mixture was stirred at 70°C for 2 hours. Under ice cooling, a saturated aqueous solution of sodium bicarbonate was added, followed by extraction with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (413 mg).

### Referential Example 888

Methyl 3-(2-{[7-chloro-5-(2,3-dimethoxyphenyl)-2-oxo-2,3,4,5-tetrahydro-1H-1-benzazepin-3-yl]methyl}-1,3-oxazol-5-yl)propanoate

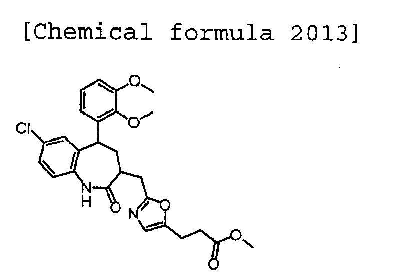

Methyl 3-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1,3-oxazol-5-yl)propanoate (413 mg) was dissolved in acetone (30 mL). Under ice cooling, an aqueous solution (6 mL) of ceric(IV) ammonium nitrate (1.05 g) was added and the resulting mixture was stirred at room temperature for 5 hours. To the reaction mixture was added a saturated aqueous solution of sodium bicarbonate-ethyl acetate. The resulting mixture was filtered through celite. The filtrate was extracted with ethyl acetate.
The extract was washed with saturated brine and dried over anhydrous sodium sulfate. The solvent was distilled off under reduced pressure. The residue was purified by silica gel column chromatography (ethyl acetate-hexane) to give the title compound (187 mg).
1H-NMR (CDCl3) δ: 2.09-2.19 (1H, m), 2.58-2.72 (3H, m), 2.80 (1H, dd, J = 16.24, 6.96 Hz), 2.94 (2H, t, J = 7.57 Hz), 3.03-3.14 (1H, m), 3.29 (1H, dd, J = 15.99, 6.96 Hz), 3.50 (3H, s), 3.68 (3H, s), 3.87 (3H, s), 4.82 (1H, q, J = 6.75 Hz), 6.61-6.65 (1H, m), 6.69-6.73 (1H, m), 6.91-7.00 (3H, m), 7.11-7.19 (2H, m).

### Referential Example 889

Methyl 3-(2-{[7-chloro-5-(2,3-dimethoxyphenyl)-2-thioxo-2,3,4,5-tetrahydro-1H-1-benzazepin-3-yl]methyl}-1,3-oxazol-5-yl)propanoate

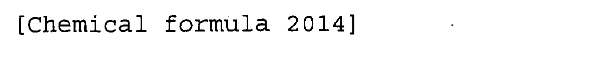

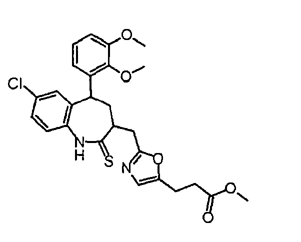

Methyl 3-(2-{[7-chloro-5-(2,3-dimethoxyphenyl)-2-oxo-2,3,4,5-tetrahydro-1H-1-benzazepin-3-yl]methyl}-1,3-oxazol-5-yl)propanoate (187 mg) was dissolved in toluene (5 mL). To the resulting solution was added Lawesson's reagent (172 mg) at room temperature. The resulting mixture was heated under reflux for 2 hours. The solvent was distilled off under reduced pressure. The residue was purified by silica gel column chromatography (hexane-ethyl acetate) to give the title compound (177 mg).
1H-NMR (CDCl3) δ: 2.15-2.24 (1H, m), 2.59-2.77 (3H, m), 2.94 (2H, t, J = 7.20 Hz), 3.04 (1H, dd, J = 15.99, 7.20 Hz), 3.24-3.34 (1H, m), 3.49-3.56 (4H, m), 3.68 (3H, s), 3.87 (3H, s), 4.70 (1H, q, J = 6.67 Hz), 6.62 (1H, s), 6.71 (1H, d, J = 2.20 Hz), 6.93 (2H, d, J = 8.06 Hz), 7.03 (1H, d, J = 8.54 Hz), 7.14 (1H, t, J = 8.06 Hz), 7.21 (1H, dd, J = 8.42, 2.32 Hz), 9.29 (1H, s).

### Referential Example 890

Methyl 3-[2-({7-chloro-5-(2,3-dimethoxyphenyl)-2-[(Z)hydrazono]-1,3,4,5-tetrahydro-2H-1-benzazepin-3-yl}methyl)-1,3-oxazol-5-yl]propanoate

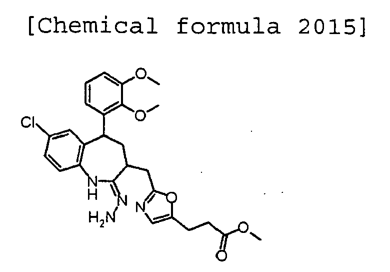

Methyl 3-(2-{[7-chloro-5-(2,3-dimethoxyphenyl)-2-thioxo-2,3,4,5-tetrahydro-1H-1-benzazepin-3-yl]methyl}-1,3-oxazol-5-yl)propanoate (120 mg) was dissolved in tetrahydrofuran (5 mL). To the resulting solution was added hydrazine monohydrate (0.057 mL). The resulting mixture was stirred at room temperature for 12 hours. The solvent was distilled off under reduced pressure to give the title compound. The compound was provided for the subsequent reaction without purification.

### Example 943

Methyl 3-(2-{[8-chloro-6-(2,3-dimethoxyphenyl)-1-(trifluoromethyl)-5,6-dihydro-4H-[1,2,4]triazolo[4,3-a][1]benzazepin-4-yl]methyl}-1,3-oxazol-5-yl)propanoate

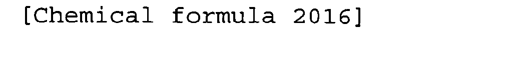

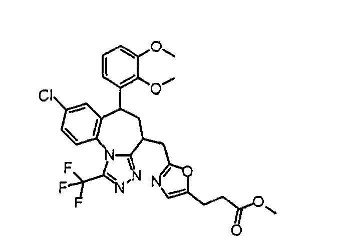

Methyl 3-[2-((7-chloro-5-(2,3-dimethoxyphenyl)-2-[(Z)hydrazono]-1,3,4,5-tetrahydro-2H-1-benzazepin-3-yl)methyl)-1,3-oxazol-5-yl]propanoate (77.1 mg) was dissolved in dichloromethane (3 mL). Under ice cooling, trifluoroacetic anhydride (0.064 mL) and trifluoroacetic acid (0.306 mL) were added. The resulting mixture was stirred at room temperature for 2 hours and then, at 55°C for 2 hours. Toluene (3 mL) was added further and the resulting mixture was heated under reflux for 3 hours. The solvent was distilled off under reduced pressure. To the residue was added ethyl acetate and a saturated aqueous solution of sodium bicarbonate. The resulting mixture was extracted with ethyl acetate. The extract was washed with saturated brine and then, dried over anhydrous sodium sulfate. The solvent was distilled off under reduced pressure. The residue was purified by silica gel column chromatography (hexane-ethyl acetate) to give the title compound (72.7 mg).
1H-NMR (CDCl3) δ: 2.21.-2.02 (1H, m), 2.64 (2H, t, J = 7.57 Hz), 2.74-2.88 (1H, m), 2.96 (2H, t, J = 7.45 Hz), 3.27-3.39 (5H, m), 3.64-3.76 (4H, m), 3.84 (3H, s), 4.10 (1H, dd, J = 13.79, 5.98 Hz), 6.65 (1H, s), 6.82-6.85 (1H, m), 6.94 (2H, d, J = 7.81 Hz), 7.14 (1H, t, J = 8.06 Hz), 7.35-7.43 (2H, m).

### Example 944

3-(2-{[8-Chloro-6-(2,3-dimethoxyphenyl)-1-(trifluoromethyl)-5,6-dihydro-4H-[1,2,4]triazolo[4,3-a][1]benzazepin-4-yl]methyl}-1,3-oxazol-5-yl)propanoic acid

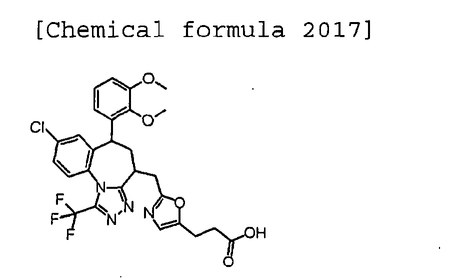

Methyl 3-(2-{[8-chloro-6-(2,3-dimethoxyphenyl)-1-(trifluoromethyl)-5,6-dihydro-4H-[1,2,4]triazolo[4,3-a][1]benzazepin-4-yl]methyl}-1,3-oxazol-5-yl)propanoate (72.7 mg) was dissolved in a mixed solution of tetrahydrofuran, methanol and water (2:2:4, 8 mL). To the resulting solution was added potassium carbonate (68.0 mg) and the resulting mixture was stirred at 50°C for 2 hours. Under ice cooling, 1N hydrochloric acid was added to the reaction mixture to make it acidic, followed by extraction with ethyl acetate. The extract was washed with saturated brine and dried over anhydrous sodium sulfate. To the residue obtained by distilling off the solvent was added ether-hexane. The precipitate thus formed was collected by filtration to give the title compound (51 mg).
1H-NMR (CDCl3) δ: 2.07-2.20 (1H, m), 2.67 (2H, t, J = 7.19 Hz), 2.74-2.88 (1H, m), 2.96 (2H, t, J = 7.19 Hz), 3.26-3.46 (5H, m), 3.68-3.78 (1H, m), 3.84 (3H, s), 4.07-4.15 (1H, m), 6.68 (1H, s), 6.82-6.88 (1H, m), 6.94 (2H, d, J = 7.80 Hz), 7.14 (1H, t, J = 8.05 Hz), 7.35-7.45 (2H, m). Elemental analysis for: C₂₇H₂₄ClF₃N₄O₅·0.25 diethyl ether Calculated: C, 56.48; H, 4.49; N, 9.41.
Found: C, 56.47; H, 4.33; N, 9.24.

### Example 945

Methyl 3-(2-{[8-chloro-6-(2,3-dimethoxyphenyl)-1-isopropyl-5,6-dihydro-4H-[1,2,4]triazolo[4,3-a][1] benzazepin-4-yl]methyl}-1,3-oxazol-5-yl)propanoate

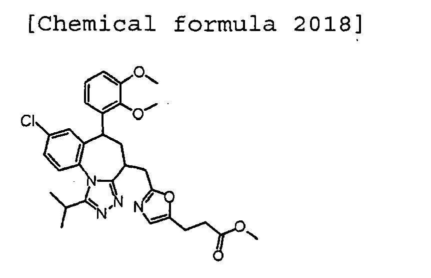

Methyl 3-[2-({7-chloro-5-(2,3-dimethoxyphenyl)-2-[(Z)hydrazono]-1,3,4,5-tetrahydro-2H-1-benzazepin-3-yl}methyl)-1,3-oxazol-5-yl]propanoate (52.7 mg) was dissolved in dichloromethane (3 mL). Under ice cooling, isobutyric anhydride (0.051 mL) and isobutyric acid (0.191 mL) were added. The resulting mixture was stirred at room temperature for 2 hours and then, at 55°C for 2 hours. Toluene (3 mL) was added further and the resulting mixture was heated under reflux for 3 hours. The solvent was distilled off under reduced pressure. To the residue was added ethyl acetate and a saturated aqueous solution of sodium bicarbonate and the resulting mixture was extracted with ethyl acetate. The extract was washed with saturated brine and dried over anhydrous sodium sulfate. The solvent was distilled off under reduced pressure. The residue was purified by silica gel column chromatography (chloroform-methanol) to give the title compound (53.5 mg).
1H-NMR (CDCl3) δ: 1.16 (3H, d, J = 6.84 Hz), 1.60 (3H, d, J = 6.84 Hz), 2.00-2.12 (1H, m), 2.55-2.68 (3H, m), 2.69-2.81 (1H, m), 2.96 (2H, t, J = 7.45 Hz), 3.19-3.36 (3H, m), 3.42 (3H, s), 3.68 (3H, s), 3.84 (3H, s), 4.13 (1H, dd, J = 13.06, 5.98 Hz), 6.65 (1H, s), 6.78 (1H, d, J = 2.44 Hz), 6.94 (2H, dd, J = 12.08, 7.45 Hz), 7.14 (1H, t, J = 8.06 Hz), 7.30-7.22 (1H, m), 7.32 (1H, dd, J = 8.42, 2.32 Hz).

### Example 946

3-(2-{[8-Chloro-6-(2,3-dimethoxyphenyl)-1-isopropyl-5,6-dihydro-4H-[1,2,4]triazolo[4,3-a][1]benzazepin-4-yl]methyl}-1,3-oxazol-5-yl)propanoic acid

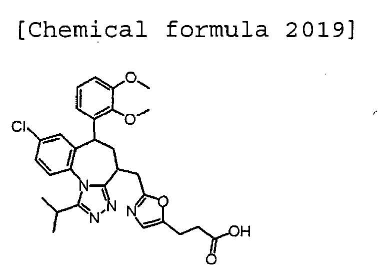

Methyl 3-(2-{[8-chloro-6-(2,3-dimethoxyphenyl)-1-isopropyl-5,6-dihydro-4H-[1,2,4]triazolo[4,3-a][1]benzazepin-4-yl]methyl}-1,3-oxazol-5-yl)propanoate (53.5 mg) was dissolved in a mixed solution of tetrahydrofuran, methanol and water (1:1:2, 4 mL). To the resulting solution was added potassium carbonate (52.5 mg) and the resulting mixture was stirred at 50°C for 2 hours. Under ice cooling, 1N hydrochloric acid was added to the reaction mixture to make it acidic, followed by extraction with ethyl acetate. The extract was washed with saturated brine and dried over anhydrous sodium sulfate. To the residue obtained by distilling off the solvent was added ether-hexane. The precipitate thus formed was collected by filtration to give the title compound (19 mg). 1H-NMR (CDCl3) δ: 1.16 (3H, d, J = 6.83 Hz), 1.60 (3H, d, J = 6.83 Hz), 1.96-2.23 (1H, m), 2.63-2.71 (1H, m), 2.71-2.84 (1H, m), 2.97 (2H, t, J = 7.68 Hz), 3.22-3.30 (3H, m), 3.42 (3H, s), 3.85 (3H, s), 4.02-4.02 (2H, m), 4.10-4.14 (1H, m), 6.39-7.57 (1H, m), 6.67 (1H, s), 6.79 (1H, t, J = 4.15 Hz), 6.90-7.01 (2H, m), 7.18-7.11 (1H, m), 7.25-7.30 (2H, m), 7.32-7.36 (1H, m).
Elemental analysis for: C₂₉H₃₁ClN₄O₅·0.25H₂O
Calculated: C, 62.70; H, 5.72; N, 10.09.
Found: C, 62.48; H, 5.75; N, 9.86.

### Referential Example 891

4-Chloro-2-[1-(2,3-dimethoxyphenyl)vinyl]aniline

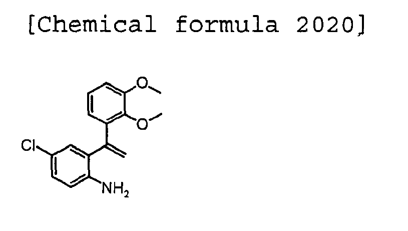

(2-Amino-5-chlorophenyl)(2;3-dimethoxyphenyl)methanone (49.1 g) was dissolved in tetrahydrofuran (500 mL). Under ice cooling, triphenylphosphine methyl iodide (103 g) and t-butoxy potassium (27.9 g) were added. The resulting mixture was stirred for 5 hours while warming to room temperature. To the reaction mixture was added water and the resulting mixture was extracted with ethyl acetate. The extract was washed with saturated brine and dried over anhydrous sodium sulfate. The solvent was distilled off. The residue was purified by silica gel column chromatography (hexane-ethyl acetate) to give the title compound (52.1 g).
1H-NMR (CDCl3) δ: 3.48 (3H, s), 3.85 (3H, s), 5.50 (1H, d, J = 1.71 Hz), 5.62 (1H, d, J = 1.71 Hz), 6.59 (1H, d, J = 8.79 Hz), 6.92-6.87 (2H, m), 7.07-6.99 (3H, m).

### Referential Example 892

4-Chloro-N-(2,4-dimethoxybenzyl)-2-[1-(2,3-dimethoxyphenyl)vinyl]aniline

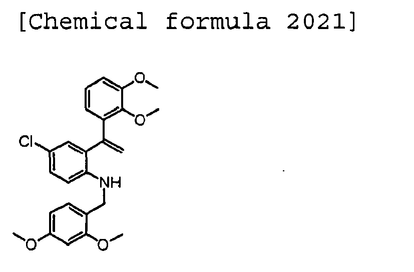

4-Chloro-2-[1-(2,3-dimethoxyphenyl)vinyl]aniline (52.1 g) was dissolved in a mixed solution of methanol (600 mL) and acetic acid (300 mL). To the resulting solution were added Molecular Sieve 3A and 2,4-dimethoxybenzaldehyde (33.1 g). The resulting mixture was stirred at 60°C for 2 hours. After ice cooling, sodium cyanoborohydride (13.6 g) was added and the mixture was stirred for 1.5 hours. After the solvent was distilled off under reduced pressure, the residue was ice cooled. A saturated aqueous solution of sodium bicarbonate was added to make it alkaline, followed by extraction with ethyl acetate. The extract was washed with saturated brine and dried over anhydrous sodium sulfate. The solvent was distilled off. The residue was purified by silica gel column chromatography (hexane-ethyl acetate) to give the title compound (73.5 g).
1H-NMR (CDCl3) δ: 3.40 (3H, s), 3.75 (3H, s), 3.78 (3H, s), 3.84 (3H, s), 4.19 (2H, s), 4.61 (1H, s), 5.50 (1H, d, J = 1.71 Hz), 5.66 (1H, d, J = 1.71 Hz), 6.36 (1H, dd, J = 8.30, 2.44 Hz), 6.41 (1H, d, J = 2.44 Hz), 6.56 (1H, d, J = 8.79 Hz), 6.83 (1H, dd, J = 7.57, 1.46 Hz), 6.87 (1H, dd, J = 8.18, 1.34 Hz), 7.00-6.94 (3H, m), 7.06-7.01 (1H, m).

### Referential Example 893

Methyl 2,3-dichloro-3-oxopropanoate

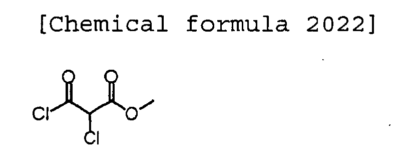

Methylmalonyl chloride (5.53 mL) was dissolved in thionyl chloride (10.9 mL). To the resulting solution were added N-chlorosuccinimide (13.6 g) and concentrated hydrochloric acid (0.3 mL). The resulting mixture was stirred at 85°C for 3 hours. The solvent was distilled off at normal temperature under reduced pressure. To the residue was added carbon tetrachloride. The resulting mixture was filtered through a filter paper. The solvent was distilled off at normal temperature under reduced pressure to give the title compound. The resulting compound was provided for the subsequent reaction without purification.

### Referential Example 894

Methyl 2-chloro-3-{4-chloro(2,4-dimethoxybenzyl)-2-[1-(2,3-dimethoxyphenyl)vinyl]anilino}-3-oxopropanoate

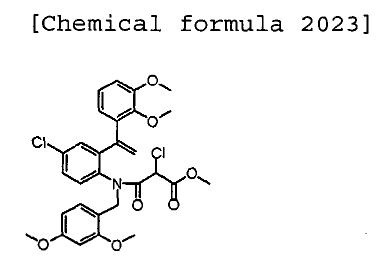

4-Chloro-N-(2,4-dimethoxybenzyl)-2-[1-(2,3-dimethoxyphenyl)vinyl]aniline (14.7 g) was dissolved in dichloromethane (180 mL). After the addition of sodium hydrogen carbonate (5.60 g) under ice cooling, a solution of methyl 2,3-dichloro-3-oxopropanoate (8.55 g) in dichloromethane (20 mL) was added dropwise. The resulting mixture was stirred at room temperature for 15 hours. To the reaction mixture was added chloroform. The resulting mixture was washed with a saturated aqueous solution of sodium bicarbonate and saturated brine and dried over anhydrous sodium sulfate. The solvent was distilled off under reduced pressure. The residue was purified by silica gel column chromatography (hexane-ethyl acetate) to give the title compound (11.3 g).
1H-NMR (CDCl3) δ: 3.49-3.56 (6.0H, m), 3.64-3.89 (9.0H, m), 4.63 (1.0H, d, J = 24.66 Hz), 4.77 (0.5H, d, J = 14.65 Hz), 4.92 (0.5H, d, J = 14.65 Hz), 5.52 (1.0H, d, J = 17.58 Hz), 5.67 (1.0H, d, J = 8.54 Hz), 6.30 (1.0H, dd, J = 12.82, 2.32 Hz), 6.35-6.43 (1.0H, m), 6.51 (0.5H, d, J = 8.54 Hz), 6.70 (0.5H, d, J = 8.54 Hz), 6.88-6.96 (2.0H, m), 7.03-7.24 (3.0H, m), 7.49 (1.0H, dd, J = 18.80, 2.44 Hz).

### Referential Example 895

Methyl 7-chloro-1-(2,4-dimethoxybenzyl)-5-(2,3-dimethoxyphenyl)-2-oxo-2,3,4,5-tetrahydro-1H-1-benzazepin-3-carboxylate

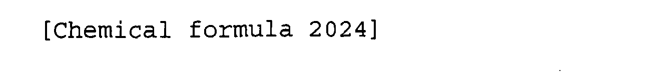

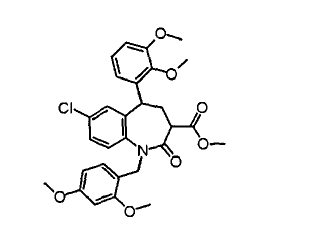

Methyl 2-chloro-3-{4-chloro(2,4-dimethoxybenzyl)-2-[1-(2,3-dimethoxyphenyl)vinyl]anilino}-3-oxopropanoate (11.3 g) was dissolved in toluene (100 mL). To the resulting solution was added dropwise a mixed solution of tributyltin (8.15 mL) and azobisisobutyronitrile (328 mg) in toluene (50 mL) over 2 hours while heating under reflux. The reaction mixture was returned to room temperature and the solvent was distilled off under reduced pressure. To the residue was added a 8% aqueous potassium fluoride solution-ether. After stirring, the reaction mixture was filtered through celite, followed by extraction with ether. The extract was washed with saturated brine. The solvent was distilled off under reduced pressure. The residue was purified by silica gel column chromatography (hexane-ethyl acetate) to give the title compound (9.95 g).

### Referential Example 896

7-Chloro-1-(2,4-dimethoxybenzyl)-5-(2,3-dimethoxyphenyl)-2-oxo-2,3,4,5-tetrahydro-1H-1-benzazepin-3-carboxylic acid

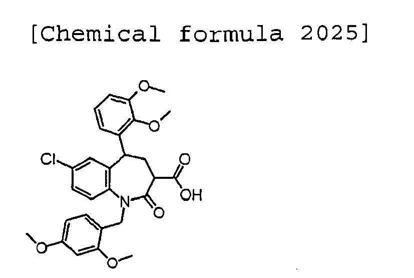

Methyl 7-chloro-1-(2,4-dimethoxybenzyl)-5-(2,3-dimethoxyphenyl)-2-oxo-2,3,4,5-tetrahydro-1H-1-benzazepin-3-carboxylate (9.95 g) was dissolved in a tetrahydrofuran-methanol-water (2:2:1, 250 mL) mixed solution. Under ice cooling, lithium hydroxide (2.32 g) was added. The resulting mixture was stirred at room temperature for 8 hours. Under ice cooling, 1N hydrochloric acid was added to the reaction mixture to make it acidic, followed by extraction with ethyl acetate. The extract was washed with saturated brine and dried over anhydrous sodium sulfate. The solvent was distilled off under reduced pressure to give the title compound (9.58 g).
1H-NMR (CDCl3) δ: 2.5 (1H, td, J = 12.8, 6.8 Hz), 3.0 (1H, td, J = 13.2, 7.7 Hz), 3.1 (3H, s), 3.3-3.4 (1H, m), 3.6 (3H, s), 3.7-3.9 (6H, m), 4.5 (1H, dd, J = 13.7, 6.8 Hz), 4.9 (1H, d, J = 14.4 Hz), 5.4 (1H, d, J = 14.6 Hz), 6.4-6.5 (2H, m), 6.7 (1H, s), 6.9 (2H, d, J = 8.3 Hz), 7.1 (1H, t, J = 8.1 Hz), 7.2-7.2 (3H, m).

### Referential Example 897

7-Chloro-1-(2,4-dimethoxybenzyl)-5-(2,3-dimethoxyphenyl)-3-(hydroxymethyl)-1,3,4,5-tetrahydro-2H-1-benzazepin-2-one

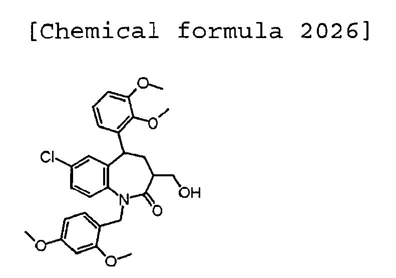

7-Chloro-1-(2,4-dimethoxybenzyl)-5-(2,3-dimethoxyphenyl)-2-oxo-2,3,4,5-tetrahydro-1H-1-benzazepin-3-carboxylic acid (216 mg) was dissolved in tetrahydrofuran (10 mL). To the resulting solution were added N-methylmorpholine (0.054 mL) and ethyl chloroformate (0.047 mL) at -10°C. The resulting mixture was stirred for 3 hours while warming to 0°C. Under ice cooling, sodium borohydride (46.6 mg) was added. Ten minutes later, methanol (5 mL) was added and the resulting mixture was stirred for one hour. Under ice cooling, 1N hydrochloric acid was added and the resulting mixture was extracted with ethyl acetate. The extract was washed with saturated brine and dried over anhydrous sodium sulfate. The solvent was distilled off under reduced pressure. The residue was purified by silica gel column chromatography (chloroform-methanol) to give the title compound (220 mg).
1H-NMR (CDCl3) δ: 2.3-2.4 (2H, m), 2.6-2.7 (1H, m), 2.7-3.1 (1H, m), 3.1 (3H, s), 3.2 (1H, t, J = 7.0 Hz), 3.7 (3H, s), 3.8 (3H, s), 3.9 (3H, s), 4.6 (1H, dd, J = 12.8, 7.2 Hz), 4.9 (1H, d, J = 15.1 Hz), 5.3 (1H, d, J = 15.1 Hz), 6.4-6.5 (2H, m), 6.6 (1H, d, J = 2.4 Hz), 6.9-7.0 (3H, m), 7.2-7.1 (3H, m).

### Referential Example 898

Ethyl 2-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-2H-1,2,3,4-tetrazol-5-yl)acetate (A) and ethyl 2-(1-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1H-1,2,3,4-tetrazol-5-yl)acetate (B)

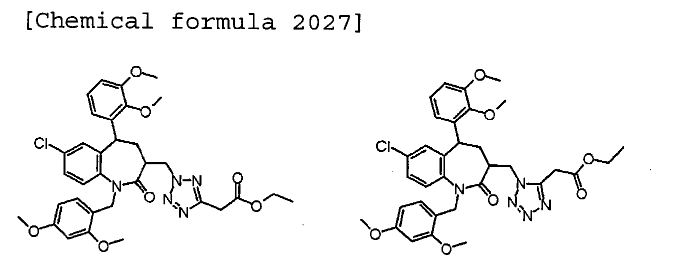

7-Chloro-1-(2,4-dimethoxybenzyl)-5-(2,3-dimethoxyphenyl)-3-(hydroxymethyl)-1,3,4,5-tetrahydro-2H-1-benzazepin-2-one (797 mg) was dissolved in tetrahydrofuran (30 mL). To the resulting solution were added ethyl tetrazole acetate (269 mg), triphenylphosphine (451 mg) and a 40% toluene solution (0.853 mL) of diethylazodicarboxylic acid. The resulting mixture was stirred at room temperature for 19 hours. The solvent was distilled off under reduced pressure. The residue was purified by silica gel column chromatography (hexane-ethyl acetate) to give the title compound (A) (673 mg) in the form of a white solid, the title compound (B) (143 mg) and a mixture (175 mg) of them.
The title compound (A)
1H-NMR (CDCl3) δ: 1.2-1.3 (3H, m), 2.2-2.4 (2H, m), 3.2 (3H, s), 3.2-3.4 (1H, m), 3.6 (3H, s), 3.8 (3H, s), 3.8 (3H, s), 4.0 (2H, s), 4.2 (2H, q, J = 7.2 Hz), 4.6 (1H, dd, J = 13.3, 6.7 Hz), 4.7 (1H, dd, J = 13.9, 7.6 Hz), 5.0 (1H, d, J = 15.1 Hz), 5.1 (1H, dd, J = 13.7, 6.3 Hz), 5.2 (1H, d, J = 14.9 Hz), 6.4 (2H, t, J = 7.8 Hz), 6.6 (1H, s), 6.8 (1H, d, J = 8.1 Hz), 6.9 (1H, d, J = 8.1 Hz), 7.1 (1H, t, J = 8.1 Hz), 7.1-7.2 (3H, m).
The title compound (B)
1H-NMR (CDCl3) δ: 1.3 (3H, td, J = 8.3, 7.2 Hz), 2.1-2.2 (1H, m), 2.6-2.7 (1H, m), 3.2 (3H, s), 3.6 (3H, s), 3.8 (3H, s), 3.9 (3H, s), 4.1-4.3 (5H, m), 4.5 (1H, d, J = 17.3 Hz), 4.6 (1H, dd, J = 13.1, 6.5 Hz), 4.7-4.9 (2H, m), 5.0 (1H, d, J = 14.9 Hz), 6.3-6.4 (2H, m), 6.6-6.7 (1H, m), 6.8-6.9 (1H, m), 7.0-6.9 (2H, m), 7.2-7.1 (3H, m).

### Referential Example 899

Ethyl 2-(2-{[7-chloro-5-(2,3-dimethoxyphenyl)-2-oxo-2,3,4,5-tetrahydro-1H-1-benzazepin-3-yl]methyl}-2H-1,2,3,4-tetrazol-5-yl)acetate

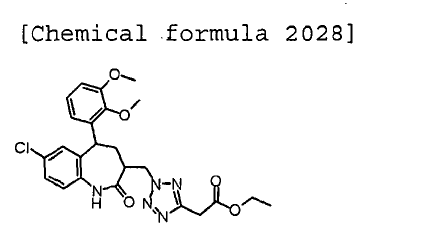

Ethyl 2-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-2H-1,2,3,4-tetrazol-5-yl)acetateIV (673 mg) was dissolved in acetone (50 mL). Under ice cooling, an aqueous solution (10 mL) of ceric ammonium nitrate (1.70 g) was added and the resulting mixture was stirred at room temperature for 15 hours. After addition of a saturated aqueous solution of sodium bicarbonate-ethyl acetate and stirring, the insoluble material was filtered off through celite. The residue was extracted with ethyl acetate, washed with saturated brine and dried over anhydrous sodium sulfate. The solvent was distilled off under reduced pressure. The residue was purified by silica gel column chromatography (hexane-ethyl acetate) to give the title compound (227 mg).
1H-NMR (CDCl3) δ: 1.2-1.3 (3H, m), 2.2-2.3 (1H, m), 2.4-2.5 (1H, m), 3.3-3.4 (1H, m), 3.5 (3H, s), 3.9 (3H, s), 4.0 (2H, s), 4.2-4.2 (2H, m), 4.7 (1H, dd, J = 13.9, 7.6 Hz), 4.8 (1H, dd, J = 13.2, 6.6 Hz), 5.1 (1H, dd, J = 13.7, 6.8 Hz), 6.7-6.7 (1H, m), 6.9 (1H, d, J = 7.3 Hz), 6.9 (1H, d, J = 8.3 Hz), 7.0 (1H, d, J = 8.0 Hz), 7.1 (1H, t, J = 8.0 Hz), 7.2 (1H, d, J = 8.5 Hz), 7.3 (1H, s).

### Referential Example 900

Ethyl 2-(2-{[7-chloro-5-(2,3-dimethoxyphenyl)-2-thioxo-2,3,4,5-tetrahydro-1H-1-benzazepin-3-yl]methyl}-2H-1,2,3,4-tetrazol-5-yl)acetate

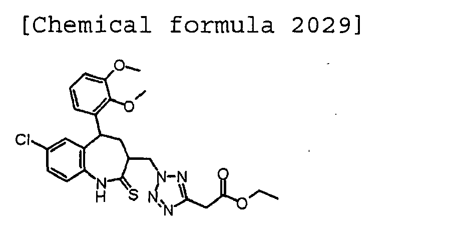

Ethyl 2-(2-{[7-chloro-5-(2,3-dimethoxyphenyl)-2-oxo-2,3,4,5-tetrahydro-1H-1-benzazepin-3-yl]methyl}-2H-1,2,3,4-tetrazol-5-yl)acetate (226 mg) was dissolved in toluene (5 mL). To the resulting solution was added Lawesson's reagent (207 mg). The resulting mixture was stirred at 85°C for 14 hours. The solvent was distilled off under reduced pressure. The residue was purified by silica gel column chromatography (hexane-ethyl acetate) to give the title compound (158 mg).
1H-NMR (CDCl3) δ: 1.2-1.3 (3H, m), 2.2-2.4 (2H, m), 3.5 (3H, s), 3.9 (3H, s), 4.0 (2H, s), 4.2 (2H, q, J = 7.2 Hz), 4.7 (1H, dd, J = 13.3, 6.5 Hz), 5.0 (1H, dd, J = 13.9, 8.5 Hz), 5.2 (1H, dd, J = 13.9, 5.6 Hz), 6.7-6.7 (1H, m), 6.8-6.8 (1H, m), 6.9-7.0 (1H, m), 7.0-7.1 (1H, m), 7.0 (1H, d, J = 8.3 Hz), 7.1 (1H, t, J = 7.8 Hz), 7.2 (1H, dd, J = 8.3, 2.2 Hz), 9.3 (1H, s).

### Example 947

Ethyl 2-(2-{[8-chloro-6-(2,3-dimethoxyphenyl)-1-(trifluoromethyl)-5,6-dihydro-4H-[1,2,4]triazolo[4,3-a][1]benzazepin-4-yl]methyl}-2H-1,2,3,4-tetrazol-5-yl)acetate

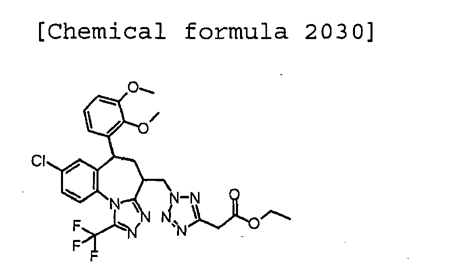

Ethyl 2-(2-{[7-chloro-5-(2,3-dimethoxyphenyl)-2-thioxo-2,3,4,5-tetrahydro-1H-1-benzazepin-3-yl]methyl}-2H-1,2,3,4-tetrazol-5-yl)acetate (157 mg) was dissolved in tetrahydrofuran (5 mL). To the resulting solution was added hydrazine monohydrate (0.074 mL). The resulting mixture was stirred at room temperature for 14 hours. After the solvent was distilled off under reduced pressure, the residue was dissolved in dichloromethane (6 mL). Under ice cooling, trifluoroacetic anhydride (0.129 mL) and trifluoroacetic acid (0.620 mL) were added. The resulting mixture was stirred at 0°C for 20 minutes, at room temperature for one hour and at 55°C for 2 hours. Toluene (6 mL) was added further and the resulting mixture was heated under reflux for 2 hours. The solvent was distilled off under reduced pressure. To the residue was added a saturated aqueous solution of sodium bicarbonate and the resulting mixture was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (143 mg).
1H-NMR (CDCl3) δ: 1.3 (3H, t, J = 7.3 Hz), 2.2-2.3 (1H, m), 2.4-2.5 (1H, m), 3.3 (3H, s), 3.5-3.6 (1H, m), 3.8 (3H, s), 4.0 (2H, s), 4.1 (1H, dd, J = 13.4, 5.6 Hz), 4.2 (2H, q, J = 7.2 Hz), 5.2 (1H, dd, J = 13.9, 9.5 Hz), 5.4 (1H, dd, J = 14.0, 5.5 Hz), 6.8-6.8 (2H, m), 6.9-7.0 (1H, m), 7.1 (1H, t, J = 7.9 Hz), 7.4-7.4 (2H, m).

### Example 948

2-(2-{[8-Chloro-6-(2,3-dimethoxyphenyl)-1-(trifluoromethyl)-5,6-dihydro-4H-[1,2,4]triazolo[4,3-a][1]benzazepin-4-yl]methyl}-2H-1,2,3,4-tetrazol-5-yl)acetic acid

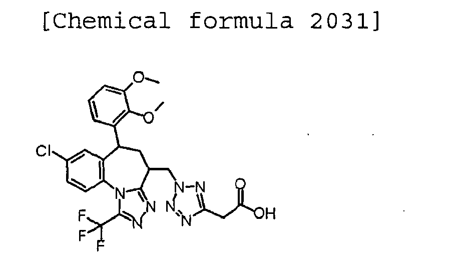

-(2-{[8-Chloro-6-(2,3-dimethoxyphenyl)-1-(trifluoromethyl)-5,6-dihydro-4H-[1,2,4]triazolo[4,3-a][1]benzazepin-4-yl]methyl}-2H-1,2,3,4-tetrazol-5-yl)acetate (142 mg) was dissolved in a mixed solution of tetrahydrofuran, methanol and water (2:2:1, 8 mL). To the resulting solution was added potassium carbonate (133 mg). The resulting mixture was stirred at 50°C for 2 hours. Under ice cooling, 1N hydrochloric acid was added to the reaction mixture to make it acidic, followed by extraction with ethyl acetate. The extract was washed with saturated brine and dried over anhydrous sodium sulfate. To the residue obtained by distilling off the solvent was added ether-hexane. The precipitate thus formed was collected by filtration to give the title compound (97 mg).
1H-NMR (CDCl3) δ: 2.2-2.3 (1H, m), 2.4-2.5 (1H, m), 3.3 (3H, s), 3.5-3.6 (1H, m), 3.8 (3H, s), 4.1 (2H, s), 4.1 (1H, dd, J = 13.5, 6.0 Hz), 5.2 (1H, dd, J = 14.0, 9.1 Hz), 5.5 (1H, dd, J = 14.0, 6.0 Hz), 6.8-6.8 (2H, m), 6.9-7.0 (1H, m), 7.1 (1H, t, J = 8.0 Hz), 7.4-7.4 (2H, m). Elemental analysis for: C₂₄H₂₁ClF₃N₇O₄
Calculated: C, 51.12; H, 3.75; N, 17.39.
Found: C, 51.31; H, 3.82; N, 17.07.

### Referential Example 901

Ethyl 2-(1-{[7-chloro-5-(2,3-dimethoxyphenyl)-2-oxo-2,3,4,5-tetrahydro-1H-1-benzazepin-3-yl]methyl}-1H-1,2,3,4-tetrazol-5-yl)acetate

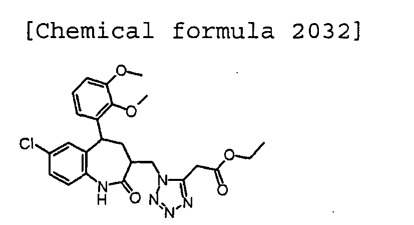

Ethyl 2-(1-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1H-1,2,3,4-tetrazol-5-yl)acetate (143 mg) was dissolved in acetone (15 mL). Under ice cooling, an aqueous solution (3 mL) of ceric(IV) ammonium nitrate (360 mg) was added and the resulting mixture was stirred at room temperature for 22 hours. To the reaction mixture was added a saturated aqueous solution of sodium bicarbonate-ethyl acetate. After stirring, the insoluble material was filtered off through celite. The residue was extracted with ethyl acetate, washed with saturated brine and dried over anhydrous sodium sulfate. The solvent was distilled off under reduced pressure. The residue was purified by silica gel column chromatography (hexane-ethyl acetate) to give the title compound (77.9 mg).
1H-NMR (CDCl3) δ: 1.3 (3H, t, J = 7.1 Hz), 2.2 (1H, td, J = 12.3, 6.4 Hz), 2.8 (1H, td, J = 13.0, 7.9 Hz), 3.3-3.4 (1H, m), 3.5-3.5 (4H, m), 3.9-3.9 (4H, m), 4.1-4.4 (4H, m), 4.7-4.9 (1H, m), 6.7-6.8 (1H, m), 7.0-6.9 (3H, m), 7.2-7.2 (3H, m) .

### Referential Example 902

Ethyl 2-(1-{[7-chloro-5-(2,3-dimethoxyphenyl)-2-thioxo-2,3,4,5-tetrahydro-1H-1-benzazepin-3-yl]methyl}-1H-1,2,3,4-tetrazol-5-yl)acetate

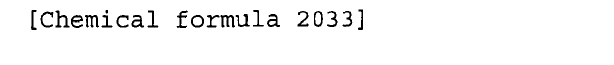

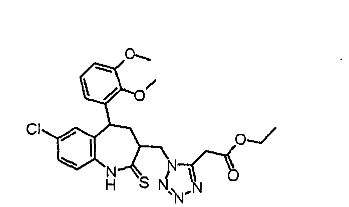

Ethyl 2-(1-{[7-chloro-5-(2,3-dimethoxyphenyl)-2-oxo-2,3,4,5-tetrahydro-1H-1-benzazepin-3-yl]methyl}-1H-1,2,3,4-tetrazol-5-yl)acetate (78 mg) was dissolved in toluene (5 mL). Lawesson's reagent (72 mg) was added and the resulting mixture was stirred at 85°C for 14 hours. The solvent was distilled off under reduced pressure. The residue was purified by silica gel column chromatography (hexane-ethyl acetate) to give the title compound (77 mg). 1H-NMR (CDCl3) δ: 1.3 (3H, t, J = 7.1 Hz), 2.2-2.3 (1H, m), 2.7-2.8 (1H, m), 3.5-3.5 (4H, m), 3.8-3.9 (4H, m), 4.2-4.3 (2H, m), 4.4-4.3 (2H, m), 4.7 (1H, dd, J = 13.5, 6.5 Hz), 5.0 (1H, dd, J = 14.0, 9.2 Hz), 6.8 (1H, s), 7.0-6.9 (4H, m), 7.2 (1H, t, J = 7.9 Hz), 9.2 (1H, s).

### Example 949

Ethyl 2-(1-{[8-chloro-6-(2,3-dimethoxyphenyl)-1-(trifluoromethyl)-5,6-dihydro-4H-[1,2,4]triazolo[4,3-a][1]benzazepin-4-yl]methyl}-1H-1,2,3,4-tetrazol-5-yl)acetate

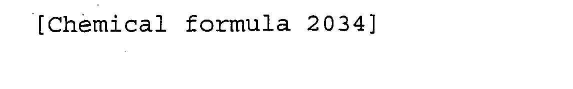

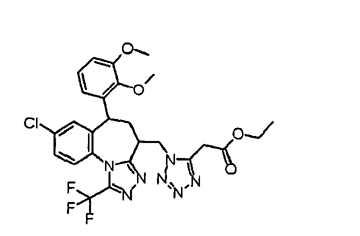

Ethyl 2-(1-{[7-chloro-5-(2,3-dimethoxyphenyl)-2-thioxo-2,3,4,5-tetrahydro-1H-1-benzazepin-3-yl]methyl}-1H-1,2,3,4-tetrazol-5-yl)acetate (77.3 mg) was dissolved in tetrahydrofuran (5 mL). To the resulting solution was added hydrazine monohydrate (0.036 mL). The resulting mixture was stirred at room temperature for 14 hours. The solvent was distilled off under reduced pressure. The residue was dissolved in dichloromethane (3 mL). Under ice cooling, trifluoroacetic anhydride (0.064 mL) and trifluoroacetic acid (0.306 mL) were added. The resulting mixture was stirred at 0°C for 20 minutes, at room temperature for one hour and at 55°C for 2 hours. Toluene (3 mL) was added further and the resulting mixture was heated under reflux for 2 hours. The solvent was distilled off under reduced pressure. To the residue was added a saturated aqueous solution of sodium bicarbonate. The resulting mixture was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (33.1 mg).
1H-NMR (CDCl3) δ: 1.3 (3H, t, J = 7.3 Hz), 2.2-2.3 (1H, m), 2.8-3.0 (1H, m), 3.3 (3H, s), 3.6-3.7 (1H, m), 3.9 (3H, s), 4.1 (1H, dd, J = 13.3, 5.5 Hz), 4.2-4.3 (2H, m), 4.3 (1H, d, J = 17.6 Hz), 4.6-4.8 (2H, m), 5.2 (1H, dd, J = 14.3, 10.1 Hz), 6.9-7.0 (3H, m), 7.2 (1H, t, J = 8.1 Hz), 7.3-7.4 (2H, m).

### Example 950

2-(1-{[8-Chloro-6-(2,3-dimethoxyphenyl)-1-(trifluoromethyl)-5,6-dihydro-4H-[1,2,4]triazolo[4,3-a][1]benzazepin-4-yl]methyl}-1H-1,2,3,4-tetrazol-5-yl)acetic acid

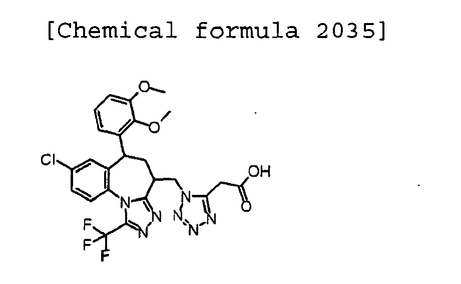

Ethyl 2-(1-{[8-chloro-6-(2,3-dimethoxyphenyl)-1-(trifluoromethyl)-5,6-dihydro-4H-[1,2,4]triazolo[4,3-a][1]benzazepin-4-yl]methyl}-1H-1,2,3,4-tetrazol-5-yl)acetate (33.1 mg) was dissolved in a mixed solution of tetrahydrofuran, methanol and water (2:2:1, 4 mL). To the resulting solution was added potassium carbonate (31 mg). The resulting mixture was stirred at 50°C for 2 hours. Under ice cooling, 1N hydrochloric acid was added to the reaction mixture to make it acidic, followed by extraction with ethyl acetate. The extract was washed with saturated brine and dried over anhydrous sodium sulfate. To the residue obtained by distilling off the solvent was added ether-hexane. The precipitate thus formed was collected by filtration to give the title compound (18 mg).
1H-NMR (CDCl3) δ: 2.2-2.3 (1H, m), 2.8-2.9 (1H, m), 3.3 (3H, s), 3.7-3.7 (1H, m), 3.8 (3H, s), 4.1 (1H, dd, J = 13.2, 5.6 Hz), 4.4 (1H, d, J = 17.8 Hz), 4.7-4.8 (2H, m), 5.2 (1H, dd, J = 14.0, 9.4 Hz), 6.9-6.9 (1H, m), 6.9-7.0 (2H, m), 7.2 (1H, t, J = 8.1 Hz), 7.4-7.4 (2H, m). Elemental analysis for: C₂₄H₂₁ClF₃N₇O₄·0.75H₂O
Calculated: C, 49.92; H, 3.93; N, 16.98.
Found: C, 50.24; H, 3.99; N, 16.46.

### Referential Example 903

Ethyl 3-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-2H-1,2,3,4-tetrazol-5-yl)propanoate (A) and ethyl 3-(1-{[7-chloro-1-(2,4-dimethoxybenzyl)-5-(2,3-dimethoxyphenyl)-2-oxo-2,3,4,5-tetrahydro-1H-1-benzazepin-3-yl]methyl}-1H-1,2,3,4-tetrazol-5-yl)propanoate (B)

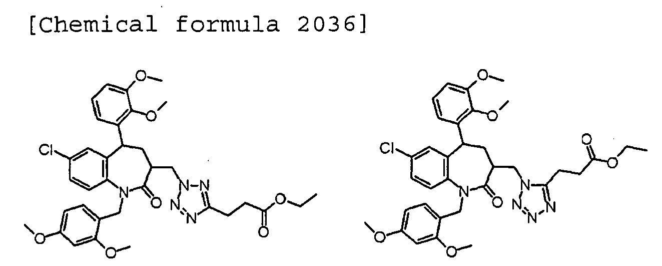

7-Chloro-1-(2,4-dimethoxybenzyl)-5-(2,3-dimethoxyphenyl)-3-(hydroxymethyl)-1,3,4,5-tetrahydro-2H-1-benzazepin-2-one (1.0 g) was dissolved in tetrahydrofuran (30 mL). To the resulting solution were added ethyl tetrazolepropionate (366 mg), triphenylphosphine (564 mg), a 40% toluene solution (1.07 mL) of diethylazodicarboxylic acid. The resulting mixture was stirred at room temperature for 12 hours. The solvent was distilled off under reduced pressure. The residue was purified by silica gel column chromatography (hexane-ethyl acetate) to give the title compound (A) (1.29 g) or the title compound (B) (300 mg) .
Title compound (A)
1H-NMR (CDCl3) δ: 1.2 (3H, t, J = 6.6 Hz), 2.2 (1H, td, J = 12.2, 6.8 Hz), 2.3 (1H, td, J = 12.8, 7.6 Hz), 2.8 (2H, t, J = 7.3 Hz), 3.2-3.2 (5H, m), 3.6 (3H, s), 3.8 (3H, s), 3.8 (3H, s), 4.1 (2H, q, J = 7.2 Hz), 4.6-4.7 (2H, m), 5.0 (1H, d, J = 14.9 Hz), 5.1 (1H, dd, J = 13.9, 6.6 Hz), 5.2 (1H, d, J = 14.9 Hz), 6.4-6.5 (2H, m), 6.6-6.6 (1H, m), 6.8 (1H, d, J = 7.8 Hz), 6.9 (1H, d, J = 8.3 Hz), 7.1 (1H, t, J = 8.1 Hz), 7.2-7.1 (2H, m), 7.2 (1H, s).

### Referential Example 904

Ethyl 3-(2-{[7-chloro-5-(2,3-dimethoxyphenyl)-2-oxo-2,3,4,5-tetrahydro-1H-1-benzazepin-3-yl]methyl}-2H-1,2,3,4-tetrazol-5-yl)propanoate

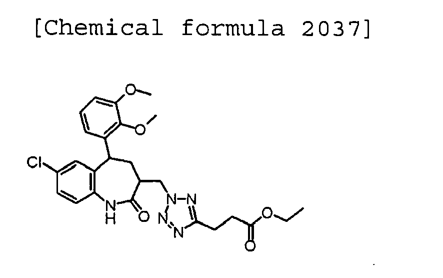

Ethyl 3-(2-{[7-chloro-1-(2,4-dimethoxybenzyl)-5-(2,3-dimethoxyphenyl)-2-oxo-2,3,4,5-tetrahydro-1H-1-benzazepin-3-yl]methyl}-2H-1,2,3,4-tetrazol-5-yl)propanoate (1.29 g) was dissolved in acetone (100 mL). Under ice cooling, an aqueous solution (20 mL) of ceric(IV) ammonium nitrate (3.19 g) was added and the resulting mixture was stirred at room temperature for 18 hours. To the reaction mixture was added a saturated aqueous solution of sodium bicarbonate-ethyl acetate. After stirring, the insoluble material was filtered off through celite. The filtrate was extracted with ethyl acetate, washed with saturated brine, and dried over anhydrous sodium sulfate. The solvent was distilled off under reduced pressure. The residue was purified by silica gel column chromatography (hexane-ethyl acetate) to give the title compound (587 mg).
1H-NMR (CDCl3) δ: 1.2 (3H, t, J = 7.3 Hz), 2.2 (1H, td, J = 12.3, 6.7 Hz), 2.4 (1H, td, J = 13.1, 7.6 Hz), 2.8 (2H, t, J = 7.4 Hz), 3.2 (2H, t, J = 7.3 Hz), 3.5 (3H, s), 3.9 (3H, s), 4.1-4.3 (3H, m), 4.7 (1H, dd, J = 13.9, 7.8 Hz), 4.8 (1H, dd, J = 13.2, 6.1 Hz), 5.0 (1H, dd, J = 13.9, 6.3 Hz), 6.7-6.7 (1H, m), 6.8-6.9 (1H, m), 6.9-7.0 (1H, m), 7.0-7.0 (1H, m), 7.1 (1H, t, J = 7.9 Hz), 7.2-7.2 (1H, m), 7.3 (1H, br s).

### Referential Example 905

Ethyl 3-(2-{[7-chloro-5-(2,3-dimethoxyphenyl)-2-thioxo-2,3,4,5-tetrahydro-1H-1-benzazepin-3-yl]methyl}-2H-1,2,3,4-tetrazol-5-yl)propanoate

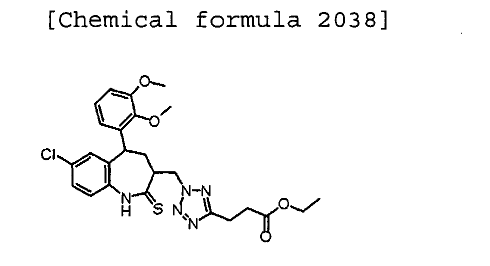

Ethyl 3-(2-{[7-chloro-5-(2,3-dimethoxyphenyl)-2-oxo-2,3,4,5-tetrahydro-1H-1-benzazepin-3-yl]methyl}-2H-1,2,3,4-tetrazol-5-yl)propanoate (587 mg) was dissolved in toluene (20 mL). To the resulting solution was added Lawesson's reagent (525 mg). The resulting mixture was stirred at 85°C for 9 hours. The solvent was distilled off under reduced pressure. The residue was purified by silica gel column chromatography (hexane-ethyl acetate) to give the title compound (282 mg).

### Example 951

Ethyl 3-(2-{[trans-8-chloro-6-(2,3-dimethoxyphenyl)-1-(trifluoromethyl)-5,6-dihydro-4H-[1,2,4]triazolo[4,3-a][1]benzazepin-4-yl]methyl}-2H-1,2,3,4-tetrazol-5-yl)propanoate

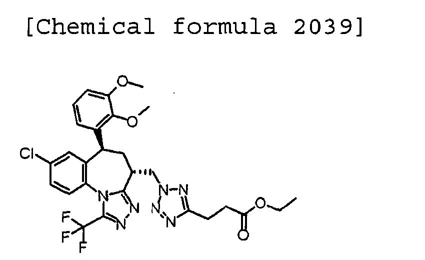

Ethyl 3-(2-{[7-chloro-5-(2,3-dimethoxyphenyl)-2-thioxo-2,3,4,5-tetrahydro-1H-1-benzazepin-3-yl]methyl}-2H-1,2,3,4-tetrazol-5-yl)propanoate (282 mg) was dissolved in tetrahydrofuran (15 mL). To the resulting solution was added hydrazine monohydrate (0.129 mL). The resulting mixture was stirred at room temperature for 21 hours. The residue obtained by distilling off the solvent under reduced pressure was dissolved in dichloromethane (10 mL). Under ice cooling, trifluoroacetic anhydride (0.225 mL) and trifluoroacetic acid (1.09 mL) were added and the resulting mixture was stirred for one hour. After stirring at 50°C for further one hour, toluene (10 mL) was added and the resulting mixture was stirred at 100°C for 6 hours. The solvent was distilled off under reduced pressure. To the residue was added chloroform and a saturated aqueous solution of sodium bicarbonate and the layers separated. The organic layer was dried over anhydrous sodium sulfate. The solvent was distilled off under reduced pressure. The residue was purified twice by silica gel column chromatography (chloroform-methanol, hexane-ethyl acetate) to give the title compound (16.9 mg).
1H-NMR (CDCl3) δ: 1.2 (3H, dt, J = 13.3, 5.7 Hz), 2.2-2.3 (1H, m), 2.4-2.5 (1H, m), 2.8 (2H, t, J = 7.4 Hz), 3.2 (2H, t, J = 7.4 Hz), 3.3 (3H, s), 3.5-3.6 (1H, m), 3.8 (3H, s), 4.1 (2H, q, J = 6.9 Hz), 5.2-5.2 (1H, m), 5.4 (1H, dd, J = 14.0, 5.5 Hz), 6.9-6.8 8 (2H, m), 6.9 (1H, d, J = 8.1 Hz), 7.1 (1H, t, J = 8.1 Hz), 7.4-7.4 (2H, m).

### Example 952

3-(2-([trans-8-chloro-6-(2,3-dimethoxyphenyl)-1-(trifluoromethyl)-5,6-dihydro-4H-[1,2,4]triazolo[4,3-a] [1]benzazepin-4-yl]methyl)-2H-1,2,3,4-tetrazol-5-yl)propanoic acid

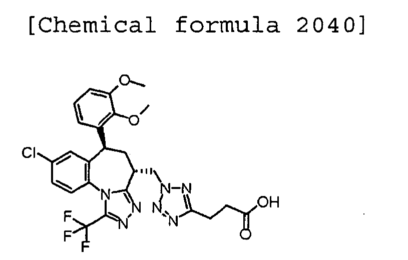

Ethyl 3-(2-{[trans-8-chloro-6-(2,3-dimethoxyphenyl)-1-(trifluoromethyl)-5,6-dihydro-4H-[1,2,4]triazolo[4,3-a][1]benzazepin-4-yl)methyl}-2H-1,2,3,4-tetrazol-5-yl)propanoate (16.9 mg) was dissolved in a mixed solution of tetrahydrofuran, methanol and water (2:2:1, 2 mL). To the resulting solution was added potassium carbonate (15.5 mg) and the resulting mixture was stirred at 50°C for one hour. Under ice cooling, 1N hydrochloric acid was added to the reaction mixture to make it acidic, followed by extraction with ethyl acetate. The extract was washed with saturated brine and dried over anhydrous sodium sulfate. To the residue obtained by distilling off the solvent was added ether-hexane. The precipitate thus formed was collected by filtration to give the title compound (6.4 mg).
1H-NMR (CDCl3) δ: 2.2-2.3 (1H, m), 2.4-2.5 (1H, m), 2.9 (2H, t, J = 7.3 Hz), 3.2 (2H, t, J = 7.4 Hz), 3.3 (3H, s), 3.5-3.6 (1H, m), 3.8 (3H, s), 4.1 (1H, dd, J = 13.4, 5.6 Hz), 5.1-5.2 (1H, m), 5.4 (1H, dd, J = 13.8, 5.7 Hz), 6.8-6.9 (2H, m), 6.9 (1H, d, J = 8.0 Hz), 7.1 (1H, t, J = 8.0 Hz), 7.5-7.4 (2H, m).
Elemental analysis for: C₂₅H₂₃ClF₃N₇O₄·0.75 ethyl acetate Calculated: C, 52.22; H, 4.54; N, 15.22.
Found: C, 52.58; H, 4.30; N, 15.43.

### Referential Example 906

Ethyl 3-(1-{[7-chloro-5-(2,3-dimethoxyphenyl)-2-oxo-2,3,4,5-tetrahydro-1H-1-benzazepin-3-yl]methyl}-1H-1,2,3,4-tetrazol-5-yl)propanoate

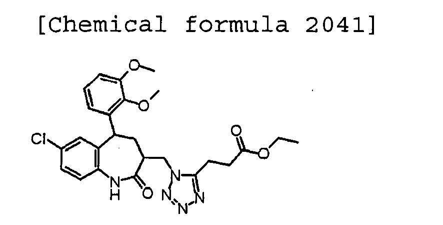

Ethyl 3-(1-([7-chloro-1-(2,4-dimethoxybenzyl)-5-(2,3-dimethoxyphenyl)-2-oxo-2,3,4,5-tetrahydro-1H-1-benzazepin-3-yl]methyl}-1H-1,2,3,4-tetrazol-5-yl)propanoate (300 mg) was dissolved in acetone (25 mL). Under ice cooling, an aqueous solution (5 mL) of ceric(IV) ammonium nitrate (743 mg) was added and the resulting mixture was stirred at room temperature for 18 hours. After addition of a saturated aqueous solution of sodium bicarbonate and ethyl acetate and stirring, the reaction mixture was filtered through celite to remove the insoluble material. The filtrate was extracted with ethyl acetate, washed with saturated brine and dried over anhydrous sodium sulfate. The solvent was distilled off under reduced pressure. The residue thus obtained was purified by silica gel column chromatography (hexane-ethyl acetate) to give the title compound (85.7 mg).
1H-NMR (CDCl3) δ: 1.2 (3H, t, J = 7.2 Hz), 2.2-2.1 (1H, m), 2.7-2.8 (1H, m), 2.9-3.1 (2H, m), 3.2-3.3 (2H, m), 3.3-3.4 (1H, m), 3.5 (3H, s), 3.9 (3H, s), 4.1 (2H, q, J = 7.1 Hz), 4.2 (1H, dd, J = 13.9, 4.6 Hz), 4.7-4.9 (2H, m), 6.7-6.8 (1H, m), 6.9-7.0 (3H, m), 7.2-7.2 (3H, m).

### Referential Example 907

Ethyl 3-(1-{[7-chloro-5-(2,3-dimethoxyphenyl)-2-thioxo-2,3,4,5-tetrahydro-1H-1-benzazepin-3-yl]methyl}-1H-1,2,3,4-tetrazol-5-yl)propanoate

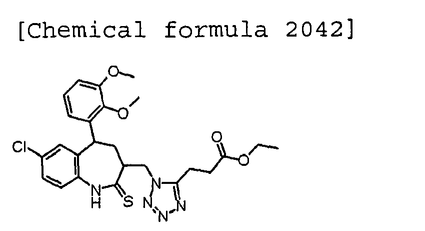

Ethyl 3-(1-{[7-chloro-5-(2,3-dimethoxyphenyl)-2-oxo-2,3,4,5-tetrahydro-1H-1-benzazepin-3-yl]methyl}-1H-1,2,3,4-tetrazol-5-yl)propanoate (85.7 mg) was dissolved in toluene (5 mL). To the resulting solution was added Lawesson's reagent (76.7 mg). The resulting mixture was stirred at 85°C for 9 hours. The solvent was distilled off under reduced pressure. The residue was purified by silica gel column chromatography (hexane-ethyl acetate) to give the title compound (45.2 mg).
1H-NMR (CDCl3) δ: 1.2-1.3 (3H, m), 2.2-2.3 (1H, m), 2.7 (1H, td, J = 13.1, 7.2 Hz), 2.9-3.1 (2H, m), 3.1-3.3 (2H, m), 3.5 (3H, s), 3.7-3.8 (1H, m), 3.9 (3H, s), 4.1-4.2 (2H, m), 4.4 (1H, dd, J = 13.9, 5.4 Hz), 4.7 (1H, dd, J = 13.7, 6.6 Hz), 5.0 (1H, dd, J = 13.8, 8.2 Hz), 6.7-6.7 (1H, m), 6.9-7.0 (2H, m), 7.0 (1H, d, J = 8.5 Hz), 7.2 (1H, t, J = 8.0 Hz), 7.2-7.3 (1H, m), 9.2 (1H, s).

### Example 953

Ethyl 3-(1-{[trans-8-chloro-6-(2,3-dimethoxyphenyl)-1-(trifluoromethyl)-5,6-dihydro-4H-[1,2,4]triazolo[4,3-a][1]benzazepin-4-yl]methyl}-1H-1,2,3,4-tetrazol-5-yl)propanoate

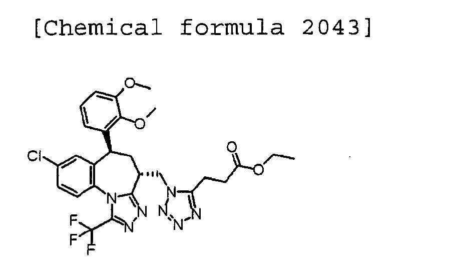

Ethyl 3-(1-{[7-chloro-5-(2,3-dimethoxyphenyl)-2-thioxo-2,3,4,5-tetrahydro-1H-1-benzazepin-3-yl]methyl}-1H-1,2,3,4-tetrazol-5-yl)propanoate (45.2 mg) was dissolved in tetrahydrofuran (3 mL). To the resulting solution was added hydrazine monohydrate (0.021 mL). The resulting mixture was stirred at room temperature for 21 hours. The residue obtained by distilling off the solvent under reduced pressure was dissolved in dichloromethane (3 mL). Under ice cooling, trifluoroacetic anhydride (0.036 mL) and trifluoroacetic acid (0.173 mL) were added and the resulting mixture was stirred for one hour. After stirring at 50°C for further one hour, toluene (10 mL) was added and the resulting mixture was stirred at 100°C for 6 hours.
The solvent was distilled off under reduced pressure. To the residue was added chloroform-a saturated aqueous solution of sodium bicarbonate and the layers separated.
The organic layer was dried over anhydrous sodium sulfate. The solvent was distilled off under reduced pressure. The residue was purified by silica gel column chromatography (chloroform-methanol) to give the title compound (31.0 mg). 1H-NMR (CDCl3) δ: 1.2-1.3 (3H, m), 2.2-2.3 (1H, m), 2.8-2.9 (1H, m), 2.9-3.1 (2H, m), 3.3-3.4 (5H, m), 3.6-3.7 (1H, m), 3.8 (3H, s), 4.0-4.2 (3H, m), 4.6-4.7 (1H, m), 5.1-5.2 (1H, m), 6.8-6.9 (1H, m), 6.9-7.0 (2H, m), 7.2 (1H, t, J = 7.6 Hz), 7.4-7.3 (2H, m).

### Example 954

3-(1-{[trans-8-Chloro-6-(2,3-dimethoxyphenyl)-1-(trifluoromethyl)-5,6-dihydro-4H-[1,2,4]triazolo[4,3-a][1]benzazepin-4-yl]methyl}-1H-1,2,3,4-tetrazol-5-yl)propanoic acid

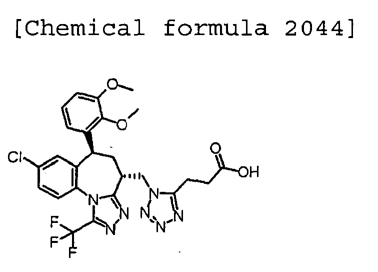

Ethyl 3-(1-{[trans-8-chloro-6-(2,3-dimethoxyphenyl)-1-(trifluoromethyl)-5,6-dihydro-4H-[1,2,4]triazolo[4,3-a] [1]benzazepin-4-yl]methyl}-1H-1,2,3,4-tetrazol-5-yl)propanoate (31.0 mg) was dissolved in a mixed solution of tetrahydrofuran, methanol and water (2:2:1, 2 mL). To the resulting solution was added potassium carbonate (28.2 mg) and the resulting mixture was stirred at 50°C for one hour. Under ice cooling, 1N hydrochloric acid was added to the reaction mixture to make it acidic, followed by extraction with ethyl acetate. The extract was washed with saturated brine and dried over anhydrous sodium sulfate.
To the residue obtained by distilling off the solvent was added ether-hexane. The precipitate thus formed was collected by filtration to give the title compound (13.0 mg).
1H-NMR (CDCl3) δ: 2.2-2.3 (1H, m), 2.8-2.9 (1H, m), 3.0-3.1 (2H, m), 3.3-3.4 (4H, m), 3.4-3.5 (1H, m), 3.6-3.7 (1H, m), 3.8 (3H, s), 4.1 (1H, dd, J = 13.3, 5.7 Hz), 4.7 (1H, dd, J = 13.9, 5.4 Hz), 5.2 (1H, dd, J = 13.8, 9.1 Hz), 6.9 (1H, s), 6.9-7.0 (2H, m), 7.2 (1H, t, J = 8.0 Hz), 7.4-7.4 (2H, m) .
Elemental analysis for: C25H23ClF3N7O4·0.25 ethyl acetate
Calculated: C, 52.05; H, 4.20; N, 16.34.
Found: C, 52.01; H, 4.12; N, 16.12.

### Referential Example 908

Methyl 3,4-dichloro-4-oxobutanoate

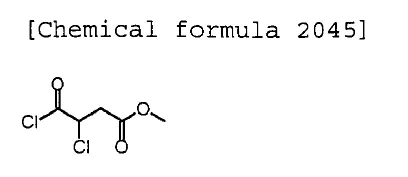

Methyl 4-chloro-4-oxobutanoate (5.0 mL) was dissolved in thionyl chloride (8.9 mL). To the resulting solution were added N-chlorosuccinimide (11.1 g) and concentrated hydrochloric acid (0.3 mL). The resulting mixture was stirred at 85°C for 3 hours. The solvent was distilled off under reduced pressure at normal temperature. To the residue was added carbon tetrachloride. The resulting mixture was filtered through a pleated filter paper and the solvent was distilled off under reduced pressure at normal temperature to give the title compound in the form of a brown oil. The resulting compound was provided for the subsequent reaction without purification.

### Referential Example 909

Methyl 3-chloro-4-{4-chloro(2,4-dimethoxybenzyl)-2-[1-(2,3-dimethoxyphenyl)vinyl]anilino}-4-oxobutanoate

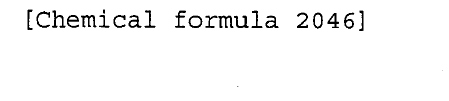

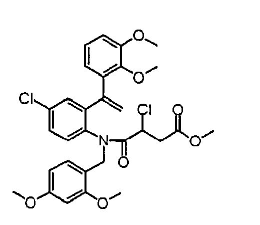

4-Chloro-N-(2,4-dimethoxybenzyl)-2'[1-(2,3-dimethoxyphenyl)vinyl]aniline (11.6 g) was dissolved in dichloromethane (180 mL). Under ice cooling, sodium hydrogen carbonate (4.42 g) was added to the resulting solution. To the resulting mixture was added dropwise a solution of methyl 3,4-dichloro-4-oxobutanoate (7.3 g) in dichloromethane (20 mL). After stirring at room temperature for 5 hours, the reaction mixture was washed with a saturated aqueous solution of sodium bicarbonate and saturated brine and dried over anhydrous sodium sulfate. The solvent was distilled off under reduced pressure. The residue was purified by silica gel column chromatography (hexane-ethyl acetate) to give the title compound (6.84 g).

### Referential Example 910

Methyl 2-[7-chloro-1-(2,4-dimethoxybenzyl)-5-(2,3-dimethoxyphenyl)-2-oxo-2,3,4,5-tetrahydro-1H-1-benzazepin-3-yl]acetate

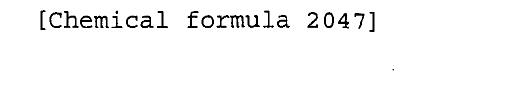

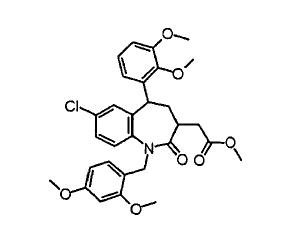

Methyl 3-chloro-4-{4-chloro(2,4-dimethoxybenzyl)-2-[1-(2,3-dimethoxyphenyl)vinyl]anilino}-4-oxobutanoate (6.84 g) was dissolved in toluene (100 mL). To the resulting solution was added dropwise a mixed solution of tributyltin (4.83 mL) and 2,2'-azobis(2-methylpropionitrile) (195 mg) in toluene (50 mL) over 20 minutes while heating under reflux. Two hours later, the reaction mixture was then returned to room temperature. The solvent was distilled off under reduced pressure. To the residue was added a 8% aqueous solution of potassium fluoride-ether and the resulting mixture was stirred. After filtration through celite, the filtrate was extracted with ether. The extract was washed with saturated brine. The solvent was distilled off under reduced pressure. The residue was purified by silica gel column chromatography (hexane-ethyl acetate) to give the title compound (1.76 g).
1H-NMR (CDCl3) δ: 2.3-2.5 (2H, m), 3.0-3.1 (2H, m), 3.2 (3H, s), 3.4 (1H, s), 3.6-3.7 (6H, m), 3.8-3.8 (3H, m), 3.8-3.9 (3H, m), 4.6-4.6 (1H, m), 5.0-5.1 (2H, m), 6.3-7.4 (9H, m).

### Referential Example 911

Methyl 2-[7-chloro-5-(2,3-dimethoxyphenyl)-2-oxo-2,3,4,5-tetrahydro-1H-1-benzazepin-3-yl]acetate

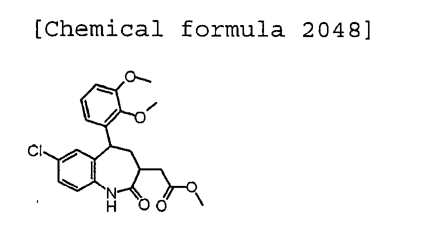

Methyl 2-[7-chloro-1-(2,4-dimethoxybenzyl)-5-(2,3-dimethoxyphenyl)-2-oxo-2,3,4,5-tetrahydro-1H-1-benzazepin-3-yl]acetate (1.43 g) was dissolved in acetone (50 mL). Under ice cooling, an aqueous solution (10 mL) of ceric(IV) ammonium nitrate (4.24 g) was added and the resulting mixture was stirred at room temperature for 4 hours. To the reaction mixture was added a saturated aqueous solution of sodium bicarbonate-ethyl acetate. The resulting mixture was filtered through celite. The filtrate was extracted with ethyl acetate. The extract was washed with saturated brine and dried over anhydrous sodium sulfate. The solvent was distilled off under reduced pressure. The residue was purified by silica gel column chromatography (ethyl acetate-hexane) to give the title compound (817 mg).
1H-NMR (CDCl3) δ: 2.0-2.1 (1H, m), 2.3-2.4 (1H, m), 2.6-2.7 (1H, m), 2.9-3.2 (2H, m), 3.5-3.6 (3H, m), 3.7-3.7 (3H, m), 3.8-3.9 (3H, m), 4.7-4.8 (1H, m), 6.7-6.7 (1H, m), 6.8-6.8 (1H, m), 6.9-7.1 (3H, m), 7.2-7.1 (2H, m).

### Referential Example 912

Methyl 2-[7-chloro-5-(2,3-dimethoxyphenyl)-2-thioxo-2,3,4,5-tetrahydro-1H-1-benzazepin-3-yl]acetate

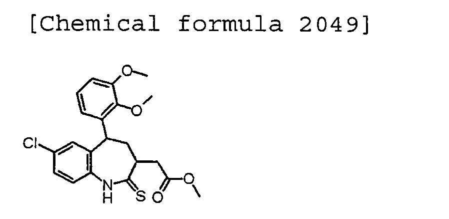

Methyl 2-[7-chloro-5-(2,3-dimethoxyphenyl)-2-oxo-2,3,4,5-tetrahydro-1H-1-benzazepin-3-yl]acetate (410 mg) was dissolved in toluene (10 mL). To the resulting solution was added Lawesson's reagent (468 mg). The resulting mixture was heated under reflux for 12 hours.
The solvent was distilled off under reduced pressure. The residue was purified by silica gel column chromatography (hexane-ethyl acetate) to give the title compound (380 mg). 1H-NMR (CDCl3) δ: 2.1-2.2 (1H, m), 2.5-2.6 (1H, m), 2.6-2.7 (1H, m), 3.2-3.3 (2H, m), 3.5 (3H, s), 3.7 (3H, s), 3.9 (3H, s), 4.6-4.7 (1H, m), 6.7-6.7 (1H, m), 6.9-7.1 (2H, m), 7.2 (1H, t, J = 8.0 Hz), 7.2 (1H, dd, J = 8.4, 2.3 Hz), 8.1 (1H, dd, J = 16.1, 8.8 Hz), 9.3 (1H, s).

### Example 955

Methyl 2-[8-chloro-6-(2,3-dimethoxyphenyl)-1-(trifluoromethyl)-5,6-dihydro-4H-[1,2,4]triazolo[4,3-a][1]benzazepin-4-yl]acetate

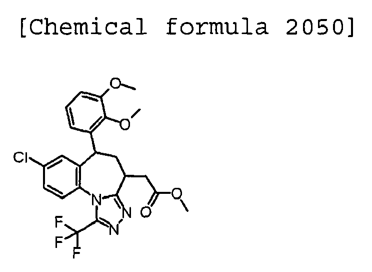

Methyl 2-[7-chloro-5-(2,3-dimethoxyphenyl)-2-thioxo-2,3,4,5-tetrahydro-1H-1-benzazepin-3-yl]acetate (100 mg) was dissolved in tetrahydrofuran (5 mL). To the resulting solution was added hydrazine monohydrate (0.023 mL) at room temperature and the resulting mixture was stirred for 2 hours. Hydrazine monohydrate (0.046 mL) was added further and the resulting mixture was stirred for 4 hours. Under ice cooling, trifluoroacetic anhydride (0.336 mL) was added, followed by stirring at room temperature for 12 hours. Under ice cooling, trifluoroacetic anhydride (0.336 mL) was added further and the resulting mixture was stirred at room temperature for 6 hours. The solvent was distilled off under reduced pressure. The residue was dissolved in chloroform. Under ice cooling, a saturated aqueous solution of sodium bicarbonate was added to the resulting solution to make it alkaline, followed by extraction with chloroform. The extract was dried over anhydrous sodium sulfate. The solvent was distilled off under reduced pressure. The residue was purified by silica gel column chromatography (hexane-ethyl acetate) to give the title compound (20.0 mg).
1H-NMR (CDCl3) δ: 2.0-2.1 (1H, m), 2.7-2.9 (2H, m), 3.2-3.3 (1H, m), 3.3-3.4 (4H, m), 3.7 (3H, s), 3.8 (3H, s), 4.0-4.1 (1H, m), 6.8-6.9 (1H, m), 6.9-7.0 (2H, m), 7.1-7.2 (1H, m), 7.4-7.4 (2H, m).

### Example 956

2-[8-Chloro-6-(2,3-dimethoxyphenyl)-1-(trifluoromethyl)-5,6-dihydro-4H-[1,2,4]triazolo[4,3-a][1]benzazepin-4-yl]acetic acid

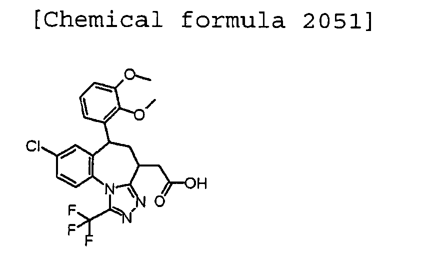

Methyl 2-[8-chloro-6-(2,3-dimethoxyphenyl)-1-(trifluoromethyl)-5,6-dihydro-4H-[1,2,4]triazolo[4,3-a][1]benzazepin-4-yl]acetate (37.8 mg) was dissolved in 1,4-dioxane (2 mL). To the resulting solution was added concentrated hydrochloric acid (0.4 mL) under ice cooling. The resulting mixture was stirred at 50°C for 19 hours. After the reaction mixture was allowed to cool, water was added. The resulting mixture was extracted with ethyl acetate. The extract was washed with saturated brine and dried over anhydrous sodium sulfate. The solvent was then distilled off under reduced pressure. To the residue was added 2-isopropanol-ether-hexane. The solid thus formed was collected by filtration to give the title compound (20.4 mg).
1H-NMR (CDCl3) δ: 2.1-2.2 (1H, m), 2.7-2.9 (1H, m), 2.9-3.0 (1H, m), 3.2-3.3 (1H, m), 3.3-3.4 (4H, m), 3.8 (3H, s), 4.0-4.1 (1H, m), 6.9 (1H, br s), 7.0 (2H, t, J = 9.0 Hz), 7.2 (1H, t, J = 8.0 Hz), 7.4-7.4 (2H, m).
Elemental analysis for: C₂₂H₁₉ClF₃N₃O₄·0.25 isopropanol
Calculated: C, 54.99; H, 4.26; N, 8.46.
Found: C, 55.08; H, 4.13; N, 8.31.

### Referential Example 913

Isopropyl 7-chloro-1-(2,4-dimethoxybenzyl)-5-(2,3-dimethoxyphenyl)-2-oxo-2,3,4,5-tetrahydro-1H-1-benzazepin-3-carboxylate

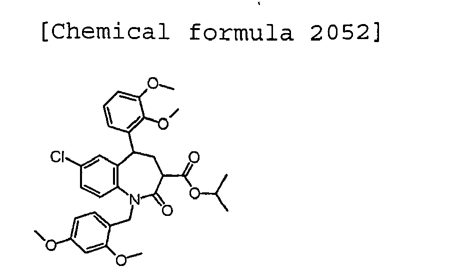

7-Chloro-1-(2,4-dimethoxybenzyl)-5-(2,3-dimethoxyphenyl)-2-oxo-2,3,4,5-tetrahydro-1H-1-benzazepin-3-carboxylic acid (3.31 g) was dissolved in dimethylformamide (50 mL). To the resulting solution were added potassium carbonate (1.04 g) and 2-iodopropane (0.69 mL). The resulting mixture was stirred at 40°C for 22 hours. Under ice cooling, water was added, followed by extraction with ethyl acetate. The extract was washed with saturated brine and dried over anhydrous sodium sulfate. The solvent was then distilled off under reduced pressure. The residue was purified by silica gel column chromatography (hexane-ethyl acetate) to give the title compound (2.80 g).

### Referential Example 914

Isopropyl 7-chloro-5-(2,3-dimethoxyphenyl)-2-oxo-2,3,4,5-tetrahydro-1H-1-benzazepin-3-carboxylate

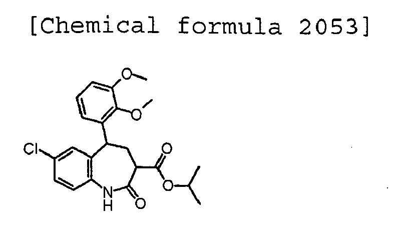

Isopropyl 7-chloro-1-(2,4-dimethoxybenzyl)-5-(2,3-dimethoxyphenyl)-2-oxo-2,3,4,5-tetrahydro-1H-1-benzazepin-3-carboxylate (2.80 mg) was dissolved in acetone (250 mL). Under ice cooling, an aqueous solution (50 mL) of ceric(IV) ammonium nitrate (8.09 g) was added and the resulting mixture was stirred at room temperature for 5 hours. After addition of a saturated aqueous solution of sodium bicarbonate-ethyl acetate and stirring, the reaction mixture was filtered through celite to remove the insoluble material. The filtrate was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (1.33 g).
1H-NMR (CDCl3) δ: 1.3 (6H, dd, J = 13.1, 6.5 Hz), 2.5-2.7 (1H, m), 2.8-2.9 (1H, m), 3.4-3.5 (1H, m), 3.5 (3H, s), 3.9 (3H, s), 4.8 (1H, dd, J = 13.3, 6.7 Hz), 5.1-5.1 (1H, m), 6.7-6.8 (1H, m), 6.8-7.0 (3H, m), 7.3-7.1 (3H, m).

### Referential Example 915

Isopropyl 7-chloro-5-(2,3-dimethoxyphenyl)-2-thioxo-2,3,4,5-tetrahydro-1H-1-benzazepin-3-carboxylate

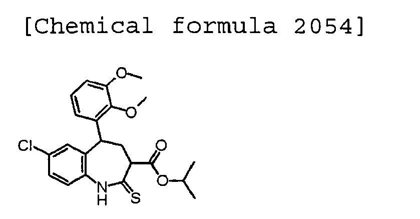

Isopropyl 7-chloro-5-(2,3-dimethoxyphenyl)-2-oxo-2,3,4,5-tetrahydro-1H-1-benzazepin-3-carboxylate (1.33 g) was dissolved in tetrahydrofuran (100 mL). To the resulting solution were added sodium hydrogen carbonate (563 mg) and diphosphorus pentasulfide (1.49 g) at room temperature. The resulting mixture was stirred at room temperature for 14 hours and then, at 60°C for 72 hours. To the reaction mixture was added ethyl acetate. The resulting mixture was washed with a saturated aqueous solution of sodium bicarbonate and saturated brine and dried over anhydrous sodium sulfate. The solvent was distilled off under reduced pressure. The residue was purified by silica gel column chromatography (hexane-ethyl acetate) to give the title compound (584 mg). At the same time, the raw material (329 mg) was collected.
1H-NMR (CDCl3) δ: 1.0-1.1 (3H, m), 1.2-1.3 (3H, m), 2.6-2.8 (1H, m), 2.9-3.3 (1H, m), 3.5 (3H, s), 3.6-4.3 (1H, m), 3.9 (3H, s), 4.6-4.7 (1H, m), 4.8-5.2 (1H, m), 6.7 (1H, br s), 6.9-7.2 (5H, m), 9.3 (1H, br s).

### Example 957

Isopropyl (trans)-8-chloro-6-(2,3-dimethoxyphenyl)-1-(trifluoromethyl)-5,6-dihydro-4H-[1,2,4]triazolo[4,3-a][1]benzazepin-4-carboxylate

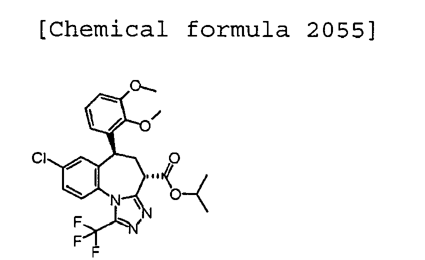

Isopropyl 7-chloro-5-(2,3-dimethoxyphenyl)-2-thioxo-2,3,4,5-tetrahydro-1H-1-benzazepin-3-carboxylate (583 mg) was dissolved in tetrahydrofuran (10 mL). To the resulting solution was added hydrazine monohydrate (0.196 mL). The resulting mixture was stirred at room temperature for 2 hours. Under ice cooling, trifluoroacetic anhydride (1.91 mL) was added to the reaction mixture. The resulting mixture was stirred for 9 hours while returning to room temperature. Dichloroethane (20 mL) was added and the resulting mixture was stirred at 55°C for 12 hours. The solvent was distilled off under reduced pressure. Under ice cooling, a saturated aqueous solution of sodium bicarbonate was added and the resulting mixture was extracted with ethyl acetate. The extract was washed with saturated brine and dried over anhydrous sodium sulfate. The solvent was distilled off under reduced pressure. The residue was purified by silica gel column chromatography (hexane-ethyl acetate) to give the title compound (371 mg). 1H-NMR (CDCl3) δ: 1.0 (6H, dd, J = 14.1, 6.3 Hz), 2.6-2.7 (1H, m), 3.3-3.4 (4H, m), 3.8 (3H, s), 4.2 (1H, dd, J = 13.4, 5.6 Hz), 4.5 (1H, d, J = 8.8 Hz), 4.6-4.7 (1H, m), 6.8-6.8 (1H, m), 7.0 (1H, d, J = 8.3 Hz), 7.1 (1H, d, J = 7.8 Hz), 7.2 (1H, t, J = 8.0 Hz), 7.2-7.4 (2H, m).

### Example 958

(trans)-8-Chloro-6-(2,3-dimethoxyphenyl)-1-(trifluoromethyl)-5,6-dihydro-4H-[1,2,4]triazolo[4,3-a][1]benzazepin-4-carboxylic acid

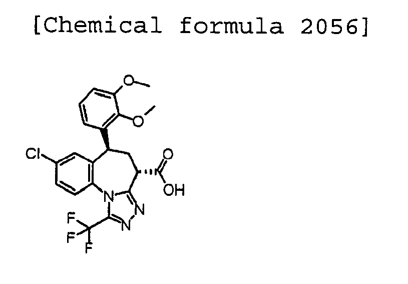

Isopropyl (trans)-8-chloro-6-(2,3-dimethoxyphenyl)-1-(trifluoromethyl)-5,6-dihydro-4H-[1,2,4]triazolo[4,3-a][1]benzazepin-4-carboxylate (187 mg) was dissolved in ethanol (10 mL). To the resulting solution was added pulverized potassium hydroxide (670 mg) and the resulting mixture was stirred at room temperature for 2 hours. Under ice cooling, 1N hydrochloric acid was added to make the reaction mixture acidic. The resulting mixture was extracted with chloroform. After the extract was dried over anhydrous sodium sulfate, the solvent was distilled off under reduced pressure. Hexane was added to the residue and the solid thus formed was collected by filtration, followed by drying at 40°C under reduced pressure to give the title compound (151 mg).
MS (ESI) m/z : 468 (M+H)+.
1H-NMR (CDCl3) δ: 2.6-2.7 (1H, m), 3.2-3.3 (1H, m), 3.4 (3H, s), 3.8 (3H, s), 4.2 (1H, dd, J = 13.5, 5.5 Hz), 4.6 (1H, d, J = 9.0 Hz), 6.8-6.9 (1H, m), 6.9-7.0 (1H, m), 7.1-7.1 (1H, m), 7.2-7.2 (1H, m), 7.3-7.3 (1H, m), 7.4 (1H, br s).
Elemental analysis for: C₂₁H₁₇ClF₃N₃O₄
Calculated: C, 53.91; H, 3.66; N, 8.98.
Found: C, 53.54; H, 3.57; N, 8.73.

### Example 959

(S)-(2-Amino-5-chlorophenyl)(2-chloro-3-methoxyphenyl)methanol (+)-di-p-toluoyl-D-tartrate

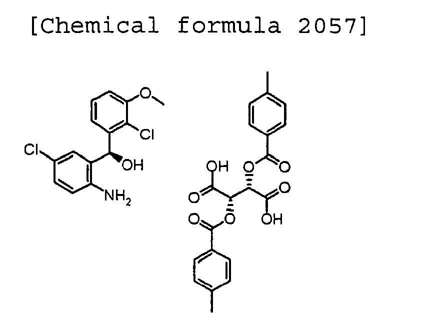

(2-Amino-5-chlorophenyl)(2-chloro-3-methoxyphenyl)methanol (9.28 g) and (+)-di-p-toluoyl-D-tartaric acid (6.01 g) were suspended in acetonitrile (100 mL) and dissolved at 80°C. The resulting solution was stirred for 14 hours while gradually cooling to room temperature. The solid thus precipitated was filtered out and washed with isopropyl ether (10 mL × 2). The solid thus obtained was dissolved in acetonitrile (80 mL). The resulting solution was stirred at room temperature for 14 hours. The solid thus precipitated was filtered out and washed with isopropyl ether (10 mL) to give the title compound (5.44 g). The title compound had an optical purity of 95.0%ee in the free amine form under the following HPLC measurement conditions.
¹H-NMR (CD₃OD) δ: 2.42 (6H, s), 3.89 (3H, s), 5.94 (2H, s), 6.13 (1H, s), 6.60-6.62 (1H, m), 6.75 (1H, d, J = 8.3 Hz), 7.00 (1H, dd, J = 8.3, 2.5 Hz), 7.06 (1H, d, J = 8.3 Hz), 7.20 (1H, d, J = 7.8 Hz), 7.30-7.38 (5H, m), 7.96-8.02 (4H, m).
Column: Chiralpak OD (4.6 × 150 mm)
Eluting solvent: 30% isopropanol-hexane
Elution rate: 1 mL/min
Retention time:
(S)-(2-Amino-5-chlorophenyl)(2-chloro-3-methoxyphenyl)methanol: 10.3 minutes
(R)-(2-Amino-5-chlorophenyl)(2-chloro-3-methoxyphenyl)methanol: 13.2 minutes

### Example 960

[Optical resolution of (4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetic acid (R)-(+)-N-benzyl-1-phenylethylamine salt [[trans-8-chloro-6-(2-chloro-3-methoxyphenyl)-1-(trifluoromethyl)-4H,6H-[1,2,4]triazolo[4,3-a][4,1]benzoxazepin-4-yl]acetic acid]

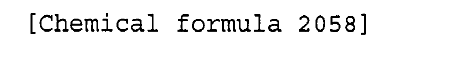

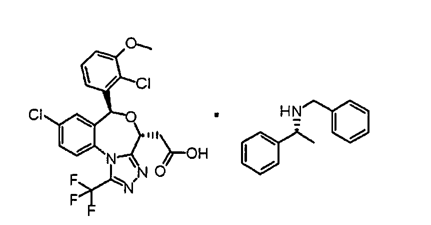

[trans-8-Chloro-6-(2-chloro-3-methoxyphenyl)-1-(trifluoromethyl)-4H,6H-[1,2,4]triazolo[4,3-a][4,1]benzoxazepin-4-yl]acetic acid (500 mg) was heat dissolved in methanol (15 mL). To the resulting solution was added (R)-(+)-N-benzyl-1-phenylethylamine (0.107 mL) under stirring at room temperature. A very small amount of seed crystals was added and the resulting mixture was stirred at room temperature for 17.5 hours. The solid thus formed was collected by filtration and washed with methanol to give the title compound (234 mg) as a colorless solid. The resulting solid had an optical purity of 98.1 %ee in the form of a free carboxylic acid under the following HPLC measurement conditions.
¹H-NMR (CD₃OD) δ: 1.61 (3H, d, J = 6.8 Hz), 3.09 (1H, dd, J = 16.0, 7.4 Hz), 3.25 (0.5H, d, J = 6.3 Hz), 3.82 (1H, d, J = 13.2 Hz), 3.89 (3H, s), 3.96 (1H, d, J = 12.7 Hz), 4.25 (1H, q, J = 6.8 Hz), 4.91 (1H, dd, J = 7.4, 6.2 Hz), 5.51 (1H, s), 6.65 (1H, d, J = 2.2 Hz), 7.16 (1H, dd, J = 6.8, 2.9 Hz), 7.32-7.35 (2H, m), 7.37-7.49 (9H, m), 7.68 (1H, dd, J = 8.7, 2.1 Hz), 7.71 (1H, d, J = 8.5 Hz).
HPLC measurement conditions:
Column: Chiralpak QD-AX (4.6 × 150 mm)
Eluting solvent: 4% acetic acid-methanol
Elution rate:1 mL/min
Retention time:
[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetic acid: 17.79 minutes. The enantiomer thereof (in the (4S,6R)-form): 13.16 minutes (Both were detected in the free carboxylic acid form.)

### Example 961

[(4R,6S)-8-Chloro-6-(2-chloro-3-methoxyphenyl)-1-(trifluoromethyl)-4H,6H-[1,2,4]triazolo[4,3-a] [4,1]benzoxazepin-4-yl]acetic acid

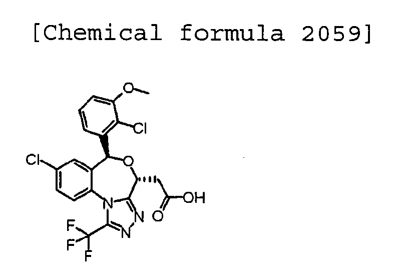

[(4R,6S)-8-Chloro-6-(2-chloro-3-methoxyphenyl)-1-(trifluoromethyl)-4H,6H-[1,2,4]triazolo[4,3-a][4,1]benzoxazepin-4-yl]acetic acid (R)-(+)-N-benzyl-1-phenylethylamine salt (127 mg) was suspended in ethyl acetate (5 mL). To the resulting suspension were added water (2 mL) and a 1N aqueous hydrochloric acid solution (0.8 mL) and the layers separated. The organic layer thus obtained was washed with saturated brine (2 mL) and dried over anhydrous sodium sulfate. The solvent was distilled off under reduced pressure to give the title compound (85 mg). The resulting compound had an optical purity of 98.3 %ee under the following HPLC measurement conditions.
HPLC measurement conditions
Column: Chiralpak QD-AX (4.6 × 150 mm)
Eluting solvent:4% acetic acid-methanol
Elution rate:1 mL/min
Retention time:
The title compound: 18.30 minutes. The enantiomer ((4S,6R)-form) thereof: 13.43 minutes.

### Example 962

2-[trans-8-Chloro-6-(2-chloro-3-methoxyphenyl)-1-(trifluoromethyl)-4H,6H-[1,2,4]triazolo[4,3-a] [4,1]benzoxazepin-4-yl]acetic acid

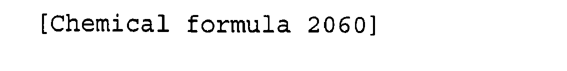

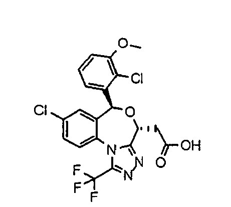

In a similar manner to that employed for the synthesis of 3-(2-{[trans-8-chloro-6-(2,3-dimethoxyphenyl)-1-(2,2,2-trifluoroethyl)-4H,6H-[1,2,4]triazolo[4,3-a][4,1]benzothiazepin-4-yl]methyl}-1,3-thiazol-5-yl)propanoic acid, the title compound (1.76 g) was obtained from ethyl 2-[trans-8-chloro-6-(2-chloro-3-methoxyphenyl)-1-(trifluoromethyl)-4H,6H-[1,2,4]triazolo[4,3-a] [4,1]benzoxazepin-4-yl]acetate (2.00 g).
MS (FAB) m/z: 488 (M+H)⁺.
¹H-NMR (CDCl3) δ: 3.19-3.23 (1H, m), 3.41-3.43 (1H, m), 3.89 (3H, s), 4.89 (1H, t, J = 6.7 Hz), 5.47 (1H, s), 6.69-6.70 (1H, m), 6.96-6.98 (1H, m), 7.35-7.37 (2H, m), 7.51-7.52 (2H, m).

### Example 963

Ethyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer A)

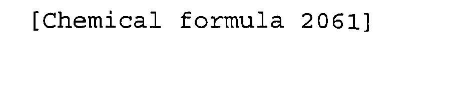

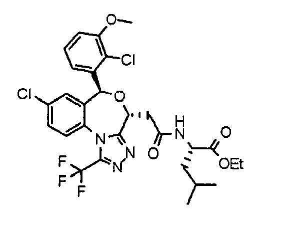

Ethyl (2S)-2-({2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer B)

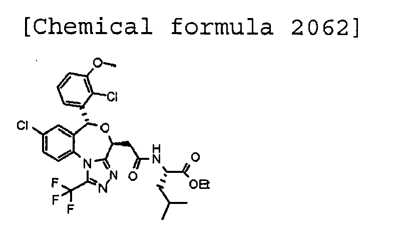

2-[trans-8-Chloro-6-(2-chloro-3-methoxyphenyl)-1-(trifluoromethyl)-4H,6H-[1,2,4]triazolo[4,3-a][4,1]benzoxazepin-4-yl]acetic acid (1.00 g) was dissolved in dichloromethane (20 mL). To the resulting solution were added 1-ethyl-3-(3-dimethylaminopropyl)carbodiimide hydrochloride (786 mg), 1-hydroxybenzotriazole (314 mg), and triethylamine (454 µl). The resulting mixture was stirred overnight at room temperature. To the residue was added a saturated aqueous solution of sodium hydrogen carbonate and the layers separated. The organic layer was washed with saturated brine and then, dried over anhydrous sodium sulfate. The solvent was distilled off under reduced pressure. The residue was purified twice by silica gel column chromatography (hexane:ethyl acetate=6:1) to give, as a low polarity substance, ethyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer A, 454 mg) and, as a high polarity substance, ethyl (2S)-2-({2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer B, 526 mg).
Ethyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer A)
MS (FAB) m/z: 629 (M+H)⁺.
¹H-NMR (CDCl₃) δ: 0.87-0.96 (6H, m), 1.25-1.30 (4H-, m), 1.52-1.68 (2H, m), 3.15 (1H, dd, J = 14.8, 6.7 Hz), 3.39 (1H, dd, J = 14.8, 6.7 Hz), 3.91 (3H, s), 4.19 (2H, q, J = 7.1 Hz), 4.53-4.61 (1H, m), 4.98 (1H, t, J = 6.7 Hz), 5.53 (1H, s), 6.30 (1H, d, J = 8.1 Hz), 6.72 (1H, d, J = 1.7 Hz), 6.98-7.05 (1H, m), 7.37-7.43 (2H, m), 7.50-7.56 (2H, m) . Ethyl (2S)-2-({2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer B)
MS (FAB) m/z: 629 (M+H)⁺.
¹H-NMR (CDCl₃) δ: 0.94-1.01 (6H, m), 1.22-1.29 (4H, m), 1.53-1.73 (2H, m), 3.16 (1H, dd, J = 14.8, 7.4 Hz), 3.38 (1H, dd, J = 14.8, 5.9 Hz), 3.91 (3H, s), 4.16 (2H, q, J = 7.1 Hz), 4.57-4.67 (1H, m), 4.99 (1H, dd, J = 7.4, 5.9 Hz), 5.55 (1H, s), 6.42 (1H, d, J = 8.5 Hz), 6.71 (1H, d, J = 1.7 Hz), 7.01 (1H, dd, J = 7.6, 2.2 Hz), 7.32-7.40 (2H, m), 7.50-7.56 (2H, m).

### Example 964

Methyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-3-cyclohexyl propanoate (isomer A)

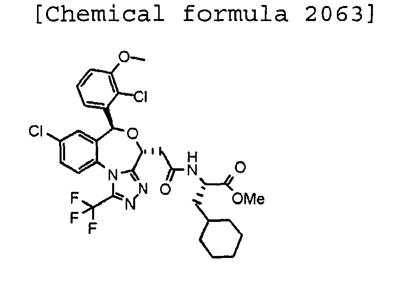

Methyl (2S)-2-({2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-3-cyclohexyl propanoate (isomer B)

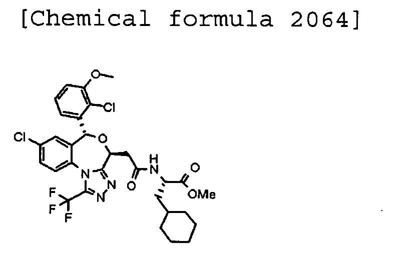

In a similar manner to that employed for the synthesis of ethyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate and ethyl (2S)-2-({2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate, methyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-3-cyclohexyl propanoate(isomer A, 294 mg) was obtained as a low polarity substance and methyl (2S)-2-({2-[(4S,6R)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-3-cyclohexyl propanoate (isomer B, 262 mg) was obtained as a high polarity substance, respectively, from 2-[trans-8-chloro-6-(2-chloro-3-methoxyphenyl)-1-(trifluoromethyl)-4H,6H-[1,2,4]triazolo[4,3-a] [4,1]benzoxazepin-4-yl]acetic acid (500 mg).
Methyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-3-cyclohexyl propanoate (isomer A)
MS (FAB) m/z: 655 (M+H)⁺.
¹H-NMR (CDCl₃) δ: 0.79-0.97 (2H, m), 1.01-1.28 (4H, m), 1.49-1.73 (7H, m), 3.14 (1H, dd, J = 14.5, 6.7 Hz), 3.40 (1H, dd, J = 14.5, 6.7 Hz), 3.74 (3H, s), 3.91 (3H, s), 4.58-4.66 (1H, m), 4.97 (1H, t, J = 6.7 Hz), 5.53 (1H, s), 6.31 (1H, d, J = 8.3 Hz), 6.73 (1H, d, J = 2.2 Hz), 6.98-7.04 (1H, m), 7.38-7.44 (2H, m), 7.49-7.57 (2H, m).
Methyl (2S)-2-({2-[(4S,6R)-8-chloro-6-(2-chloro-3-methoxyphenyl)-1-(trifluoromethyl)-4H,6H-[1,2,4]triazolo[4,3-a] [4,1]benzoxazepin-4-yl]acetyl}amino)-3-cyclohexyl propanoate (isomer B)
MS (FAB) m/z: 655 (M+H)⁺.
¹H-NMR (CDCl₃) δ: 0.85-1.01 (2H, m), 1.11-1.40 (4H, m), 1.51-1.85 (7H, m), 3.15 (1H, dd, J = 14.8, 6.6 Hz), 3.41 (1H, dd, J = 14.8, 6.6 Hz), 3.70 (3H, s), 3.92 (3H, s), 4.60-4.67 (1H, m), 4.97 (1H, t, J = 6.6 Hz), 5.54 (1H, s), 6.42 (1H, d, J = 7.8 Hz), 6.72 (1H, s), 6.99-7.05 (1H, m), 7.35-7.42 (2H, m), 7.51-7.56 (2H, m).

### Example 965

2-[(4R,6S)-8-Chloro-6-(2-chloro-3-methoxyphenyl)-1-(trifluoromethyl)-4H,6H-[1,2,4]triazolo[4,3-a] [4,1]benzoxazepin-4-yl]acetic acid

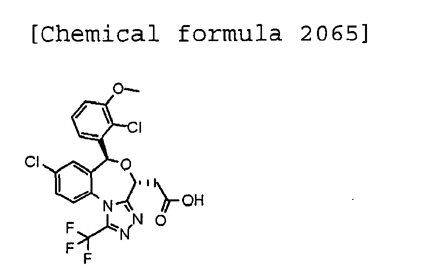

Ethyl (2S)-2-({2-[(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetyl}amino)-4-methylpentanoate (isomer A, 447 mg) was dissolved in 1,4-dioxane (100 mL). To the resulting solution were added water (50 mL) and concentrated hydrochloric acid (50 mL). The resulting mixture was heated under reflux overnight at 120°C. To the reaction mixture were added ice water (400 mL) and ethyl acetate (400 mL) and the layers separated. The organic layer was dried over anhydrous sodium sulfate. The solvent was then distilled off under reduced pressure. After complete removal of 1,4-dioxane from the residue by azeotropy with water, water:ethanol (50:1) was added and the solid thus precipitated was collected by filtration to give the title compound (288 mg).
MS (FAB) m/z: 488 (M+H)⁺.
¹H-NMR (CDCl3) δ: 3.19-3.23 (1H, m), 3.41-3.43 (1H, m), 3.89 (3H, s), 4.89 (1H, t, J = 6.7 Hz), 5.47 (1H, s), 6.69-6.70 (1H, m), 6.96-6.98 (1H, m), 7.35-7.37 (2H, m), 7.51-7.52 (2H, m).

### Referential Example 916

(S)-(2-Amino-5-chlorophenyl)(2-chloro-3-methoxyphenyl)methanol

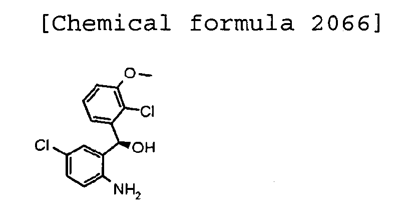

(2-Amino-5-chlorophenyl)(2-chloro-3-methoxyphenyl)methanone (500 mg) was suspended in degassed isopropanol (30 mL). The resulting suspension was stirred at 70°C for one hour in an argon gas stream. The solution thus obtained was cooled to a temperature of from 30 to 40°C and a t-butanol solution (a 0.1M solution, 339 µl) of potassium t-butoxide and dichloro[(S)-2,2'-bis(diphenylphosphino)1,1'-binaphthyl][(S)-1,1'-bis(p-methoxyphenyl)-2-isopropyl-1,2-ethanediamine]-ruthenium(II) (19 mg) were added successively. The resulting mixture was vigorously stirred overnight at room temperature in a hydrogen gas stream of 20 atmospheres. After the pressure was returned to a normal one, the reaction mixture was concentrated under reduced pressure. The residue was purified by silica gel column chromatography (hexane:ethyl acetate = from 15:1 to 6:1) to give the title compound (478 mg). The optical purity of the title compound was measured by a CHIRALCEL OD column (product of Daicel Chemical Industries, hexane:isopropanol=7:3, flow rate: 1.0 mL/min), resulting in 99.7 %ee.
MS (FAB) M/z: 297(M+H)+.
1H-NMR (CDCl3) δ: 3.93 (3H, s), 6.20 (1H, s), 6.64 (1H, d, J = 8.5 Hz), 6.90 (1H, d, J = 2.2 Hz), 6.95 (1H, dd, J = 8.3, 1.5 Hz), 7.05-7.10 (2H, m), 7.29 (1H, t, J = 8.1 Hz).

### Referential Example 917

Ethyl [(trans)-7-chloro-1-(2,4-dimethoxybenzyl)-5-(2-chloro-3-methoxyphenyl)-2-thioxo-1,2,3,5-tetrahydro-4,1-benzoxazepin-3-yl]acetate

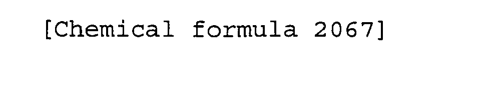

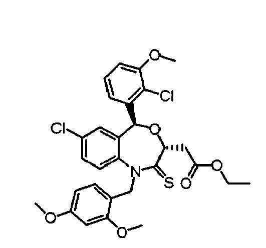

Ethyl [(trans)-7-chloro-1-(2,4-dimethoxybenzyl)-5-(2-chloro-3-methoxyphenyl)-2-oxo-1,2,3,5-tetrahydro-4,1-benzoxazepin-3-yl]acetate (0.50 g) was dissolved in toluene (15 mL). To the resulting solution was added Lawesson's reagent (0.54 g) and the resulting mixture was heated under reflux for 5 hours. The reaction mixture was diluted with ethyl acetate and washed successively with a saturated aqueous solution of sodium hydrogen carbonate and saturated brine. The organic layer was dried over anhydrous sodium sulfate. After concentration of the filtrate, the residue thus obtained was purified by silica gel column (hexane:ethyl acetate= from 8:1, 4:1 to 2:1) to give the title compound (0.60 g).
¹H-NMR (CDCl₃) δ: 1.25 (3H, t, J = 7.32 Hz), 2.99 (1H, dd, J = 16.60, 5.86 Hz), 3.39 (1H, dd, J = 16.48, 7.45 Hz), 3.62 (3H, s), 3.76 (3H, s), 3.88 (3H, s), 4.09-4.17 (2H, m), 4.59 (1H, dd, J = 7.45, 5.98 Hz), 5.41 (1H, d, J = 15.14 Hz), 5.83 (1H, s), 6.17 (1H, d, J = 14.89 Hz), 6.94-6.97 (1H, m), 6.99-7.02 (2H, m), 7.04-7.07 (1H, m), 7.31-7.40 (2H, m), 7.46 (1H, d, J = 8.30 Hz), 8.09 (1H, dd, J = 16.11, 9.03 Hz), 8.25 (1H, dd, J = 16.60, 9.03 Hz).
MS (ESI) m/z : 590 (M + H)⁺.

### Referential Example 918

Ethyl {(trans)-7-chloro-5-(2-chloro-3-methoxyphenyl)-1-(2,4-dimethoxybenzyl)-2-[(trifluoroacetyl)hydrazono]-1,2,3,5-tetrahydro-4,1-benzoxazepin-3-yl}acetate

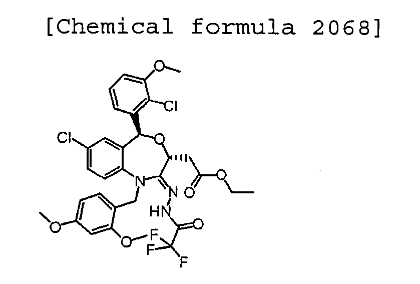

Ethyl [(trans)-7-chloro-1-(2,4-dimethoxybenzyl)-5-(2-chloro-3-methoxyphenyl)-2-thioxo-1,2,3,5-tetrahydro-4,1-benzoxazepin-3-yl]acetate (100 mg) was dissolved in tetrahydrofuran (5 mL). To the resulting solution were added successively triethylamine (0.50 mL), trifluoroacetic acid hydrazide mono trifluoroacetate (490 mg) and mercury chloride(230 mg) at 0°C. The resulting mixture was stirred at room temperature for one hour. After filtration of the reaction mixture, the filtrate was diluted with ethyl acetate and washed successively with water and saturated brine. The organic layer was dried over anhydrous sodium sulfate. The filtrate was concentrated and the residue was purified by silica gel column (hexane:ethyl acetate= from 2:1, 1:1 to 1:2) to give the title compound (70 mg).
¹H-NMR (CDCl₃) δ: 1.26 (3H, t, J = 7.07 Hz), 2.97 (1H, dd, J = 16.83, 6.10 Hz), 3.29 (1H, dd, J = 16.58, 7.56 Hz), 3.78 (3H, s), 3.83 (3H, s), 3.93 (3H, s), 4.09-4.19 (2H, m), 4.73 (1H, t, J = 6.95 Hz), 4.99 (1H, d, J = 17.56 Hz), 5.59 (1H, dd, J = 5.98, 2.07 Hz), 6.15 (1H, s), 6.42-6.49 (3H, m), 7.00 (1H, d, J = 8.05 Hz), 7.13 (2H, d, J = 8.29 Hz), 7.23-7.30 (2H, m), 7.37 (1H, t, J = 8.05 Hz).
MS (ESI) m/z : 684 (M + H)⁺.

### Example 966

Ethyl [(trans)-8-chloro-6-(2-chloro-3-methoxyphenyl)-1-(trifluoromethyl)-4H,6H-[1,2,4]triazolo[4,3-a] [4,1]benzoxazepin-4-yl]acetate

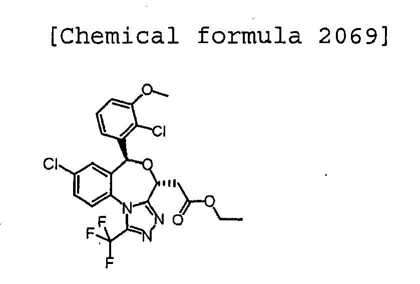

Ethyl {(trans)-7-chloro-5-(2-chloro-3-methoxyphenyl)-1-(2,4-dimethoxybenzyl)-2-[(trifluoroacetyl)hydrazono]-1,2,3,5-tetrahydro-4,1-benzoxazepin-3-yl}acetate (50 mg) was dissolved in dichloromethane (2.0 mL). After addition of anisole (79 mg), trifluoroacetic acid (3 mL) was added dropwise at 0°C. The resulting mixture was stirred for 2 hours while gradually returning to room temperature. To the reaction mixture was added a saturated aqueous solution of sodium hydrogen carbonate. The resulting mixture was then extracted with ethyl acetate. The organic layer was washed successively with a saturated aqueous solution of sodium hydrogen carbonate, water and saturated brine. The organic layer was dried over anhydrous sodium sulfate. The filtrate was concentrated and the residue thus obtained was purified by silica gel column (hexane:ethyl acetate= from 4:1, 2:1 to 1:2) to give a racemic mixture of the title compound (22.5 mg).
¹H-NMR (CDCl₃) δ: 1.29 (3H, t, J = 6.83 Hz), 3.27 (1H, dd, J = 16.71, 7.19 Hz), 3.50 (1H, dd, J = 16.83, 6.34 Hz), 3.92 (3H, s), 4.17-4.23 (2H, m), 4.96 (1H, t, J = 6.83 Hz), 5.56 (1H, s), 6.74 (1H, s), 7.02 (1H, d, J = 8.05 Hz), 7.35 (1H, d, J = 8.05 Hz), 7.40 (1H, t, J = 8.05 Hz), 7.54-7.56 (2H, m).
MS (ESI) m/z : 516 (M + H)⁺.

### Referential Example 919

Allyl [(3R,5S)-7-chloro-5-(2-chloro-3-methoxyphenyl)-1-(2,4-dimethoxybenzyl)-2-oxo-1,2,3,5-tetrahydro-4,1-benzoxazepin-3-yl]acetate

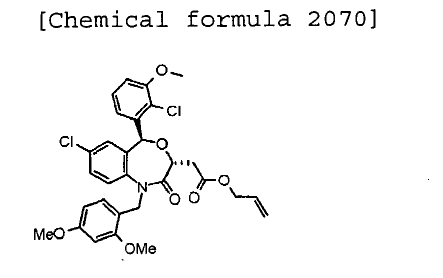

(S)-(2-Amino-5-chlorophenyl)(2-chloro-3-methoxyphenyl)methanol (637 mg) was dissolved in methylene chloride (5 mL). To the resulting solution were added a methylene chloride solution (3 mL) of sodium hydrogen carbonate (358 mg) and monoallyl chlorofumarate (372 mg) in an ice water bath. After stirring at the same temperature for one hour, the reaction mixture was diluted with methylene chloride and washed with water. The organic layer was dried over anhydrous sodium sulfate and then, the filtrate was concentrated. The residue was dissolved in tetrahydrofuran (20 mL). To the resulting solution was added 1,8-diazabicyclo[5.4.0]undec-7-ene (0.65 mL) and the resulting mixture was stirred at 80°C for one hour. The reaction mixture was diluted with ethyl acetate and washed with water. The organic layer was washed with saturated brine and then dried over anhydrous sodium sulfate. The filtrate was concentrated. The residue was purified by silica gel column (ethyl acetate:hexane=1:2) to give the title compound (479 mg) as a mixture with the cis isomer. MS (ESI) m/z : 586 (M+H)⁺.
Trans isomer
¹H-NMR (CDCl₃) δ: 2.85 (1H, dd, J = 16.6, 6.0 Hz), 3.14 (1H, dd, J = 16.6, 7.6 Hz), 3.60 (3H, s), 3.75 (3H, s), 3.90 (3H, s), 4.47 (1H, dd, J = 7.6, 6.0 Hz), 4.58-4.63 (2H, m), 4.85 (1H, d, J = 14.2 Hz), 5.23 (1H, dd, J = 10.5, 1.5 Hz), 5.31 (1H, dd, J = 17.2, 1.5 Hz), 5.49 (1H, d, J = 14.7 Hz), 5.85-5.96 (2H, m), 6.33 (1H, d, J = 2.2 Hz), 6.37-6.42 (2H, m), 6.95 (1H, dd, J = 8.1, 1.7 Hz), 7.24-7.39 (5H, m).

### Referential Example 920

Allyl [(3R,5S)-7-chloro-5-(2-chloro-3-methoxyphenyl)-2-oxo-1,2,3,5-tetrahydro-4,1-benzoxazepin-3-yl]acetate

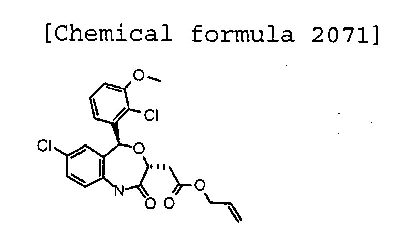

To allyl [(3R,5S)-7-chloro-5-(2-chloro-3-methoxyphenyl)-1-(2,4-dimethoxybenzyl)-2-oxo-1,2,3,5-tetrahydro-4,1-benzoxazepin-3-yl]acetate (cis/trans mixture) (205 mg) was added trifluoroacetic acid (5 mL). The resulting mixture was stirred at 50°C for 90 minutes. The reaction mixture was concentrated and the residue was purified by a silica gel column (ethyl acetate:hexane=3:7) to give the title compound (126 mg) as a mixture with the cis isomer.
MS (ESI) m/z : 436 (M+H)⁺.
Trans Isomer
¹H-NMR (CDCl₃) δ: 2.86 (1H, dd, J = 16.4, 6.6 Hz), 3.09 (1H, dd, J = 16.4, 6.6 Hz), 3.94 (3H, s), 4.58 (2H, d, J = 5.6 Hz), 4.63 (1H, t, J = 6.6 Hz), 5.22 (1H, dd, J = 10.0, 1.5 Hz), 5.30 (1H, dd, J = 17.2, 1.5 Hz), 5.81-5.95 (1H, m), 6.22 (1H, s), 6.59 (1H, d, J = 2.2 Hz), 7.00 (1H, d, J = 8.1 Hz), 7.04 (1H, d, J = 8.3 Hz), 7.21 (1H, d, J = 8.1 Hz), 7.28-7.38 (2H, m), 8.33 (1H, s).

### Referential Example 921

Allyl [(3R,5S)-7-chloro-5-(2-chloro-3-methoxyphenyl)-2-thioxo-1,2,3,5-tetrahydro-4,1-benzoxazepin-3-yl]acetate

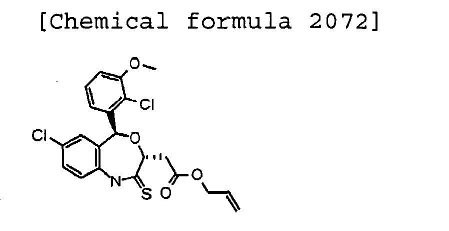

Allyl [(3R,5S)-7-chloro-5-(2-chloro-3-methoxyphenyl)-2-oxo-1,2,3,5-tetrahydro-4,1-benzoxazepin-3-yl]acetate (cis/trans mixture) (270 mg) was dissolved in tetrahydrofuran (10 mL). To the resulting solution were added sodium hydrogen carbonate (239 mg) and diphosphorus pentasulfide (550 mg) in an ice water bath. The resulting mixture was stirred at room temperature for 3 hours. The reaction mixture was diluted with ethyl acetate and washed with water. The organic layer was dried over anhydrous sodium sulfate. The filtrate was concentrated. The residue was purified by silica gel column (ethyl acetate:hexane=1:2) to give the title compound (479 mg) as a mixture with a cis isomer.
MS (ESI) m/z : 452 (M+H)⁺.
Trans Isomer
¹H-NMR (CDCl₃) δ: 3.03 (1H, dd, J = 16.7, 6.6 Hz), 3.31 (1H, dd, J = 16.7, 6.6 Hz), 3.94 (3H, s), 4.60 (2H, d, J = 5.4 Hz), 4.70 (1H, t, J = 6.6 Hz), 5.23 (1H, d, J = 10.3 Hz), 5.31 (1H, dd, J = 16.7, 1.4 Hz), 5.82-5.98 (1H, m), 6.13 (1H, s), 6.57 (1H, d, J = 2.2 Hz), 6.92-6.98 (1H, m), 7.00 (1H, d, J = 8.1 Hz), 7.09 (1H, d, J = 8.6 Hz), 7.34-7.39 (2H, m), 9.52 (1H, s).

### Example 967

Allyl [(4R,6S)-8-chloro-6-(2-chloro-3-methoxyphenyl)-1-(trifluoromethyl)-4H,6H-[1,2,4]triazolo[4,3-a][4,1]benzoxazepin-4-yl]acetate

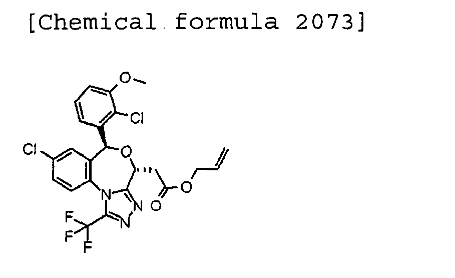

In a similar manner to that employed for the synthesis of ethyl [(trans)-8-bromo-6-(2-chloro-3-methoxyphenyl)-1-(trifluoromethyl)-4H,6H-[1,2,4]triazolo[4,3-a] [4,1]benzoxazepin-4-yl]acetate, the title compound (155 mg) was obtained from allyl [(3R,5S)-7-chloro-5-(2-chloro-3-methoxyphenyl)-2-thioxo-1,2,3,5-tetrahydro-4,1-benzoxazepin-3-yl]acetate (cis/trans mixture) (223 mg) .
MS (ESI) m/z : 528 (M+H)⁺.
¹H-NMR (CDCl₃) δ: 3.31 (1H, dd, J = 16.7, 7.1 Hz), 3.54 (1H, dd, J = 16.7, 6.1 Hz), 3.92 (3H, s), 4.59-4.71 (2H, m), 4.96 (1H, dd, J = 7.1, 6.4 Hz), 5.26 (1H, ddd, J = 10.4, 2.4, 1.2 Hz), 5.34 (1H, ddd, J = 17.3, 2.9, 1.5 Hz), 5.56 (1H, s), 5.86-5.98 (1H, m), 6.74 (1H, d, J = 1.7 Hz), 7.01 (1H, dd, J = 8.1, 1.5 Hz), 7.32-7.36 (1H, m), 7.40 (1H, t, J = 8.0 Hz), 7.52-7.57 (2H, m).

### Example 968

Allyl [(trans)-8-chloro-6-(2-chloro-3-methoxyphenyl)-1-(trifluoromethyl)-4H,6H-[1,2,4]triazolo[4,3-a] [4,1]benzoxazepin-4-yl]acetate

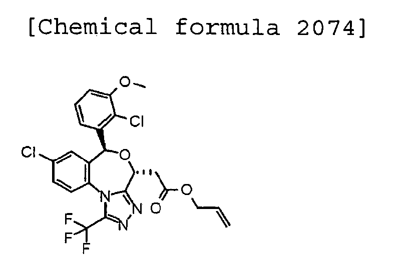

[(trans)-8-Chloro-6-(2-chloro-3-methoxyphenyl)-1-(trifluoromethyl)-4H,6H-[1,2,4]triazolo[4,3-a][4,1]benzoxazepin-4-yl]acetic acid (512 mg) was dissolved in methylene chloride (10 mL). To the resulting solution were added diisopropylethylamine (0.55 mL) and allyl bromide (0.37 mL) in an ice water bath. The resulting mixture was stirred overnight at room temperature. The reaction mixture was diluted with methylene chloride and washed with 1N hydrochloric acid. The organic layer was dried over anhydrous sodium sulfate. The filtrate was concentrated. The residue was purified by silica gel column (ethyl acetate:hexane=1:3) to give the title compound (541 mg).
MS (ESI) m/z : 528 (M+H)⁺.
¹H-NMR (CDCl₃) δ: 3.31 (1H, dd, J = 16.7, 7.1 Hz), 3.54 (1H, dd, J = 16.7, 6.4 Hz), 3.92 (3H, s), 4.59-4.70 (2H, m), 4.96 (1H, t, J = 6.7 Hz), 5.23-5.28 (1H, m), 5.30-5.37 (1H, m), 5.56 (1H, s), 5.86-5.98 (1H, m), 6.73 (1H, s), 7.01 (1H, d, J = 8.3 Hz), 7.34 (1H, d, J = 7.8 Hz), 7.39 (1H, t, J = 8.0 Hz), 7.50-7.58 (2H, m).

### Example 969

[(trans)-8-Chloro-6-(2-chloro-3-methoxyphenyl)-1-(trifluoromethyl)-4H,6H-[1,2,4]triazolo[4,3-a][4,1]benzoxazepin-4-yl]acetic acid

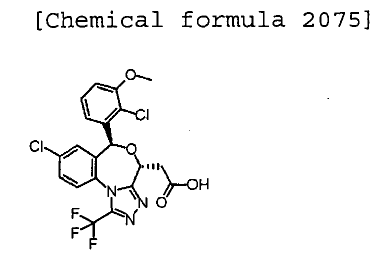

Allyl [(trans)-8-chloro-6-(2-chloro-3-methoxyphenyl)-1-(trifluoromethyl)-4H,6H-[1,2,4]triazolo[4,3-a][4,1]benzoxazepin-4-yl]acetate (84 mg), tetrakis(triphenylphosphine)palladium(0) (10 mg) and triphenylphosphine (6 mg) were dissolved in methylene chloride (2 mL). In an ice water bath, pyrrolidine (0.1 mL) was added and the resulting mixture was stirred for one hour. The reaction mixture was diluted with methylene chloride and then washed with 1N hydrochloric acid. The organic layer was dried over anhydrous sodium sulfate. The filtrate was concentrated. The residue was purified by silica gel column (methanol:chloroform=4:96) to give the title compound (85 mg). MS (ESI) m/z : 488 (M+H)⁺.
¹H-NMR (CDCl₃) δ: 3.33 (1H, dd, J = 17.3, 6.7 Hz), 3.57 (1H, dd, J = 16.7, 6.6 Hz), 3.92 (3H, s), 4.93 (1H, t, J = 6.9 Hz), 5.56 (1H, s), 6.74 (1H, s), 7.01 (1H, dd, J = 7.8, 1.5 Hz), 7.34-7.43 (2H, m), 7.52-7.58 (2H, m).
Test
The rat squalene synthetase inhibitory effect and rat liver-cholesterol synthesis inhibitory effect of the compounds of the present invention were confirmed by the method shown below.

1. Rat squalene synthetase inhibitory activity
   (1) Preparation of enzyme source
      As an enzyme source, a microsomal fraction prepared from a rat liver was employed. First, eight-week-old SD male rats were subjected to laparotomy under anesthesia with pentobarbital sodium. After exsanguination from the inferior vena cava, the liver was excised. To the liver thus obtained were added 0.3M sucrose, 1 mM dithiothreitol (DTT), 1 mM sodium ethylenediamine tetraacetate (EDTA) and various protease inhibitors. The resulting mixture was homogenized in the presence of a 10 mM phosphate buffer adjusted to pH 7.4 and centrifuged for 5 minutes at 2,000 × g and for 15 minutes at 10,000 × g. As the protease inhibitor, phenylmethanesulfonyl fluoride (PMSF), leupeptin and aprotinin were added to give the final concentrations of 1 mM, 10 µM, and 5 µg/mL, respectively. The supernatant after centrifugation was centrifuged further at 105,000 × g for 60 minutes. The sediment thus obtained was suspended in a 10 mM phosphate buffer (pH 7.4) containing 1 mM DTT, 1 mM EDTA, and the above-described protease inhibitors and the resulting suspension was centrifuged at 105,000 × g for 30 minutes, which operation was performed twice. After washing, the final sediment thus obtained was used as a microsomal fraction for the measurement of enzymatic activity.
   (2) Measurement of squalene synthetase inhibitory activity
      Squalene synthetase activity was measured in accordance with the method of Schechter, et al., described in Journal of Biological Chemistry, 267, 8628-8635(1992). Described specifically, the compound of the present invention dissolved in water or dimethylsulfoxide (DMSO) was added to an enzyme reaction mixture (total amount: 50 µl) containing 5 mM reduced nicotinamide adenine dinucleotide phosphate (NADPH), 5 mM 3-[(3-cholamidopropyl)dimethylammonio]propanesulfonic acid (CHAPS), 10 mM potassium fluoride, 10 mM magnesium chloride, 10 mM DTT, 1 µM/mL NB-598 (squalene epoxydase inhibitor, Journal of Biological Chemistry, 265, 18075-180788(1990)), 50 mM HEPES buffer (pH 7.4)), 30 µg/mL of the rat liver microsomal fraction, and 5 µM [³H] farnesyl-pyrophosphate and the resulting mixture was reacted at 37°C for 20 minutes (enzyme reaction was started by adding [³H]farnesyl-pyrophosphate to the enzyme reaction mixture which had been incubated in advance at 37°C for 10 minutes). To the enzyme reaction mixture was added 5 µl of 1M EDTA (pH 9.2) to terminate the enzyme reaction, followed by the addition of 5 µl of a 0.5% squalene ethanol solution. After spotting of a 40 µl portion of the resulting mixture on a plastic sheet for thin layer chromatography, development with 5% toluene-95% hexane and drying, these pots were visualized by exposure to iodine vapor and the band of squalene was identified. The spots were cut out with scissors and put in a vial container. To the container was added 10 mL of Aquasol-2 (product of New England Nuclear Research Products, USA) and radioactivity was measured by a liquid scintillation counter. The results are shown in the following table. The squalene synthetase inhibitory activity was expressed as a concentration (IC₅₀) of the compound inhibiting 50% of radioactivity incorporated into squalene.

**[Table 1]**

| | IC₅₀ (nM) |
|---|---|
| Example 210 | 0.56 |
| Example 263 | 3.9 |
| Example 322 | 4.6 |
| Example 330 | 4.9 |
| Example 341 | 4.6 |
| Example 343 | 7.6 |

As is apparent from Table 1, the compounds of the present invention exhibit an excellent squalene synthetase inhibitory effect.

2. Rat single-dose liver-cholesterol synthesis inhibitory effect
   A rat single-dose liver-cholesterol synthesis inhibitory effect was measured as described below.
   To each the compounds of the present invention was added a necessary amount of a 0.5% methyl cellulose solution immediately before use. Then, an equivalent molar amount of sodium hydroxide or sodium hydrogen carbonate was added to dissolve or suspend it in the resulting solution. To 6-week-old Wistar male rats were orally administered each of the compounds of the present invention (3 mg/kg), while only a 0.5% methyl cellulose solution was administered to a control group. One hour later, physiological saline of mevalonic acid (5 µCi/5 mL/kg) labeled with a radioisotope ¹⁴C was intraperitoneally administered. The rats were sacrificed one hour later. To 1 g of the liver thus obtained from the rats was added 5 mL of a 15% KOH ethanol solution and the resulting mixture was left to stand for 15 hours. After heating at 75°C for 2 hours, the reaction mixture was extracted with 5 mL of water and 10 mL of petroleum ether. The petroleum ether layer was collected, evaporated to dryness and then dissolved in 50 µl of a chloroform:acetone=2:1 solution. The cholesterol band was separated by silica gel thin layer chromatography (Art.5748, toluene:ethyl acetate=3:1) and cut out. It was put in a vial container, followed by the addition of 10 mL of Aquasol-2 (product of Packard BioScience Company). The radioactivity was measured using a liquid scintillation counter. A ratio of the radioactivity relative to that of a control group was determined and liver-cholesterol synthesis inhibitory activity (%) was calculated.
   Activity of each of Example compounds measured by the above-described method is shown in Table 2.

**[Table 2]**

| | Liver-cholesterol synthesis inhibitory effect |
|---|---|
| Example 21 | 68% |
| Example 263 | 84% |
| Example 322 | 76% |
| Example 330 | 75% |
| Example 341 | 90% |
| Example 343 | 80% |

As is apparent from Table 2, the compounds of the present invention exhibit a high liver-cholesterol synthesis inhibitory effect.

### [Industrial applicability]

The compounds of the present invention exhibit an excellent squalene synthetase inhibitory effect and cholesterol synthesis inhibitory effect so that they are useful as a drug such as preventive and/or remedy for diseases in mammals including humans such as hyperlipemia, e.g., hypercholesterolemia, hypertriglyceridemia, and low HDL cholesterolemia and/or arteriosclerosis.

## Claims

1. A compound represented by the following formula (I) : 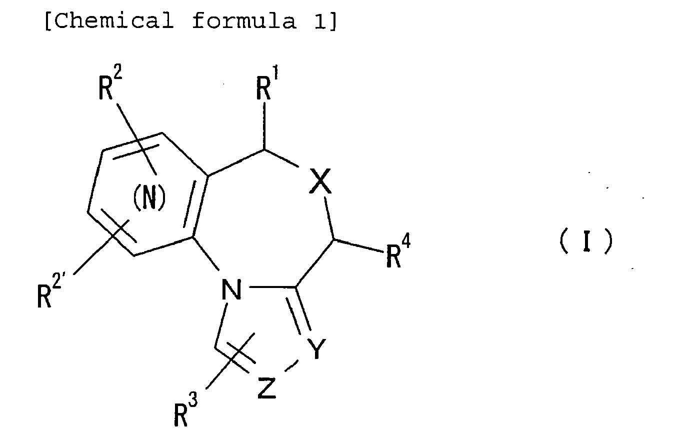 (wherein, R¹ means an aryl group or heteroaryl group which may have from one to three substituents selected from the class consisting of halogen atoms, hydroxy group, nitro group, cyano group, C₁₋₆ alkyl groups, C₁₋₆ alkoxy groups, halogeno(C₁₋₆ alkyl) groups, halogeno(C₁₋₆ alkoxy) groups, and C₁₋₆ alkylenedioxy groups which may have, at the alkylene portion thereof, substituents;
R² and R^{2'} each independently means a hydrogen atom, halogen atom, hydroxy group, nitro group, cyano group, C₁₋₆ alkyl group, C₁₋₆ alkoxy group, halogeno (C₁₋₆ alkyl) group, halogeno(C₁₋₆ alkoxy) group, hydroxy(C₁₋₆ alkyl) group, hydroxy(C₁₋₆ alkoxy) group, (C₁₋₆ alkoxy) (C₁₋₆ alkyl) group, or (C₁₋₆ alkoxy) (C₁₋₆ alkoxy) group;
R³ means a hydrogen atom, halogen atom, cyano group, C₁₋₆ alkyl group, C₂₋₆ alkenyl group, C₂₋₆ alkynyl group, C₁₋₆ alkoxy group, C₁₋₆ alkanoyl group, (C₁₋₆ alkoxy)(C₁₋₆ alkyl) group, halogeno(C₁₋₆ alkyl) group, halogeno(C₂₋₆ alkenyl) group, halogeno(C₁₋₆ alkoxy) group, halogeno(C₁₋₆ alkoxy)(C₁₋₆ alkyl) group, halogeno(C₁₋₆ alkanoyl) group, hydroxy(C₁₋₆ alkyl) group, azido(C₁₋₆ alkyl) group, carbamoyl group, mono- or di-(C₁₋₆ alkyl) carbamoyl group, C₁₋₆ alkanoylamino group, C₁₋₆ alkanoylamino(C₁₋₆ alkyl) group, (C₁₋₆ alkanoyloxy)(C₁₋₆ alkyl) group, or aryl group;
X means CH₂, O, or S;
Y means N or C-R^{3'}(wherein, R^{3'} has the same meaning as defined above in R³);
Z means N or C-R^{3"}(wherein, R^{3"} has the same meaning as defined above in R³);
R⁴ means a carboxyl group, carboxycarbonyl group,
carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and arylalkenyl groups, carboxy(C₂₋₆ alkenyl) group,
carboxy(C₁₋₆ alkoxy) group,
carboxy(C₁₋₆ alkyl)thio group,
carboxy(C₁₋₆ alkyl)thio(C₁₋₆ alkyl) group,
carboxyaryl group,
carboxy(C₁₋₆ alkoxy) (C₁₋₆ alkyl) group,
mono- or di-(carboxy(C₁₋₆ alkyl))carbamoyl group
(with the proviso that the carboxy group of the groups may be esterified or amidated),
or a group represented by the following formula: 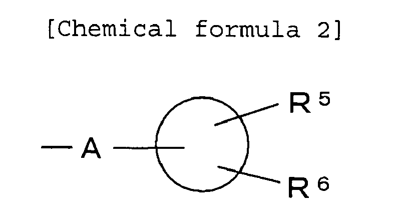 (wherein, A means a single bond, C₁₋₄ alkylene group which may have a hydroxy group and/or halogen atom, C₁₋₄ alkylenecarbonyl group which may have a hydroxy group and/or halogen atom, C₁₋₄ alkylenecarbonylamino group which may have, on the amino group thereof, a C₁₋₆ alkyl group as a substituent, or C₁₋₄ alkyleneamino group having, on the amino group thereof, a C₁₋₆ alkyl group as a substituent,
R⁵ means a carboxyl group, carboxycarbonyl group,
carboxy(C₁₋₆ alkyl) group which may have, on the alkylene group thereof, from one to four substituents selected from the class consisting of hydroxy group, halogen atoms, C₁₋₆ alkoxyimino groups, aralkyl groups, and arylalkenyl groups, carboxy(C₂₋₆ alkenyl) group,
carboxy(C₁₋₆ alkoxy) group,
carboxy(C₁₋₆ alkyl)thio group,
carboxy(C₁₋₆ alkyl)thio(C₁₋₆ alkyl) group,
carboxyaryl group,
carboxy(C₁₋₆ alkoxy)(C₁₋₆ alkyl) group, or
mono- or di-(carboxy(C₁₋₆ alkyl))carbamoyl group
(with the proviso that the carboxy group of the groups may be esterified or amidated),
R⁶ means a hydrogen atom, hydroxy group, halogen atom, C₁₋₆ alkyl group, carboxy group, C₁₋₆ alkoxy group, carboxy(C₁₋₆ alkoxy) group, halogenoalkyl group, halogeno(C₁₋₆ alkoxy) group, or aralkyl group (with the proviso that the carboxy group of the groups may be esterified or amidated), and
the group: 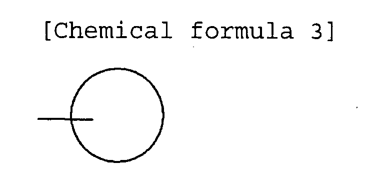 means a saturated or unsaturated cyclic hydrocarbon group or a saturated or unsaturated heterocyclic group), and the following partial structure: 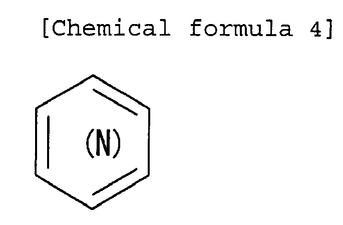 means a benzene ring or pyridine ring); salt of the compound; or solvate of the compound or the salt.

2. The compound according to Claim 1, wherein R¹ is an aryl group which may have from one to three substituents selected from the class consisting of halogen atoms, hydroxy group, nitro group, cyano group, C₁₋₆ alkyl groups, C₁₋₆ alkoxy groups, halogeno (C₁₋₆ alkyl) groups, halogeno (C₁₋₆ alkoxy) groups, and C₁₋₆ alkylenedioxy groups which may have, at the alkylene portion thereof, substituents; a salt of the compound; or a solvate of the compound or the salt.

3. The compound according to Claim 1, wherein R¹ is an aryl group which may have one or two substituents selected from the class consisting of halogen atoms, hydroxy group, and C₁₋₆ alkoxy groups; a salt of the compound; or a solvate of the compound or the salt.

4. The compound according to any one of Claims 1 to 3, wherein the aryl group is a phenyl group; a salt of the compound; or a solvate of the compound or the salt.

5. The compound according to any one of Claims 1 to 4, wherein R² and R^{2'} are each independently a hydrogen atom or halogen atom; a salt of the compound; or a solvate of the compound or the salt.

6. The compound according to any one of Claims 1 to 5, wherein R² is a halogen atom and R^{2'} is a hydrogen atom; a salt of the compound; or a solvate of the compound or the salt.

7. The compound according to any one of Claims 1 to 6, wherein R³ is a hydrogen atom, halogen atom, C₁₋₆ alkyl group, (C₁₋₆ alkoxy)(C₁₋₆ alkyl) group, halogeno(C₁₋₆ alkyl) group, halogeno(C₁₋₆ alkoxy)(C₁₋₆ alkyl) group, or halogeno(C₁₋₆ alkanoyl) group; a salt of the compound; or a solvate of the compound or the salt.

8. The compound according to any one of Claims 1 to 7, wherein R⁴ is a carboxy(C₁₋₆ alkyl) group having, on the alkylene group thereof, from one to four substituents selected from the class consisting of hydroxy group, halogen atoms, C₁₋₆ alkoxyimino groups, aralkyl groups, and aryl(C₂₋₆ alkenyl) groups, carboxy(C₁₋₆ alkoxy)(C₁₋₆ alkyl) groups (with the proviso that the carboxy group of the groups may be esterified or amidated), or a group represented by the following formula: 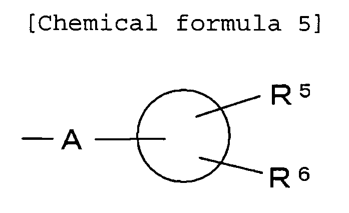 (wherein, A means a C₁₋₄ alkylene group which may have a hydroxy group or C₁₋₄ alkylenecarbonyl group which may have a hydroxy group, R⁵ means a carboxyl group, carboxy(C₁₋₆ alkoxy) group, carboxy(C₁₋₆ alkyl)thio group, carboxy(C₁₋₆ alkoxy)(C₁₋₆ alkyl) group, or carboxy(C₁₋₆ alkyl) group which may have, on the alkylene group thereof, one to four substituents selected from the class consisting of hydroxy group, halogen atoms, C₁₋₆ alkoxyimino groups, aralkyl groups, and aryl(C₂₋₆ alkenyl) groups (with the proviso that the carboxy group of the groups may be esterified or amidated),
R⁶ means a hydrogen atom, and
the group: 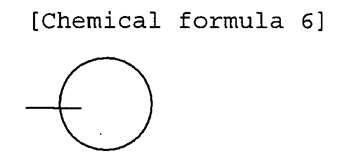 means a saturated or unsaturated 4- to 7-memered heterocyclic group); a salt of the compound; or a solvate of the compound or the salt.

9. The compound according to any one of Claims 1 to 8, wherein X is CH₂, Y is C-R^{3'}, and Z is C-R^{3"}; a salt of the compound; or a solvate of the compound or the salt.

10. The compound according to any one of Claims 1 to 8, wherein X is CH₂, Y is N, and Z is N; a salt of the compound; or a solvate of the compound or the salt.

11. The compound according to any one of Claims 1 to 8, wherein X is O, Y is N, and Z is N; a salt of the compound; or a solvate of the compound or the salt.

12. The compound according to any one of Claims 1 to 8, wherein X is O, Y is C-R^{3'}, and Z is CR^{3"}; a salt of the compound; or a solvate of the compound or the salt.

13. The compound according to any one of Claims 1 to 8, wherein X is O, Y is N, and Z is C-R^{3"}; a salt of the compound; or a solvate of the compound or the salt.

14. The compound according to any one of Claims 1 to 8, wherein X is S, Y is N, and Z is N; a salt of the compound; or a solvate of the compound or the salt.

15. The compound according to any one of Claims 1 to 8, wherein X is S, Y is N, and Z is C-R^{3"}; a salt of the compound; or a solvate of the compound or the salt.

16. The compound according to any one of Claims 1 to 8, wherein X is S, Y is C-R^{3'}, and Z is C-R^{3"}; a salt of the compound; or a solvate of the compound or the salt.

17. A drug comprising the compound, salt or solvate as claimed in any one of Claims 1 to 16.

18. A preventive and/or remedy for hypercholesterolemia, hyperlipemia, and/or arteriosclerosis, which comprises the compound, salt or solvate as claimed in any one of Claims 1 to 16.

19. A pharmaceutical composition comprising the compound, salt, or solvate as claimed in any one of Claims 1 to 16 and a pharmaceutically acceptable carrier.

20. Use of the compound, salt, or solvate as claimed in any one of Claims 1 to 16 for preparation of a drug.

21. Use according to Claim 20, wherein the drug is a preventive and/or remedy for hypercholesterolemia, hyperlipemia and/or arteriosclerosis.

22. A method of preventing and/or treating hypercholesterolemia, hyperlipemia, and/or arteriosclerosis, which comprises administering an effective amount of the compound, salt or solvate as claimed in any one of Claims 1 to 16.
